# EUROPEAN PATENT APPLICATION

(11) **EP 4 446 324 A1**
(43) Date of publication of application: **16.10.2024**
(21) Application number: 22903574.6
(22) Date of filing: 08.12.2022
(51) Int. Cl.: C07D 487/04, C07D 498/22, C07D 413/14, C07D 237/08, C07D 401/14, C07D 401/12, C07D 209/46, C07D 227/093, A61K 31/4412, A61K 31/44, A61K 31/4035, A61K 31/506, A61K 31/496, A61P 35/00

(54) **LIGAND COMPOUNDS FOR E3 UBIQUITIN LIGASE, PROTEIN DEGRADERS DEVELOPED ON BASIS OF LIGAND COMPOUNDS, AND USES THEREOF**

(30) Priority: 08.12.2021 CN 202111493174; 31.12.2021 CN 202111683076; 03.08.2022 CN 202210927231
(71) Applicant: Gluetacs Therapeutics (Shanghai) Co., Ltd., Shanghai 201306 (CN)
(72) Inventor: YANG, Xiaobao, Shanghai 201306 (CN); SUN, Renhong, Shanghai 201306 (CN); LI, Yan, Shanghai 201306 (CN); ZHAO, Baoyin, Shanghai 201306 (CN); ZHANG, Ruiyan, Shanghai 201306 (CN)
(74) Representative: J A Kemp LLP
(86) International application number: PCT/CN2022/137582
(87) International publication number: WO 2023/104155

(57) **Abstract**

The present disclosure provides compounds of Formula (I) and (II) or salts, enantiomers, stereoisomers, solvates, isotopically enriched analogs, prodrugs, or polymorphs thereof, and uses thereof. The present disclosure also provides pharmaceutical compositions comprising, as an active ingredient, the compounds of Formula (I) and (II) or salts, enantiomers, stereoisomers, solvates, isotopically enriched analogs, prodrugs, or polymorphs thereof, and uses thereof.

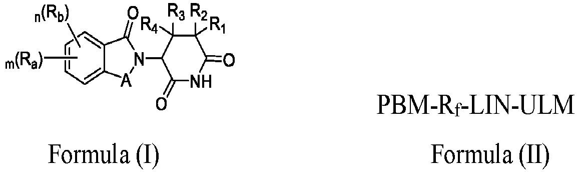

## Description

### Technical Field

The present disclosure relates to cereblon (CRBN) E3 ubiquitin ligase ligand compounds of Formula (I), protein degrader compounds of Formula (II) comprising the same, and their applications. The CRBN E3 ubiquitin ligase ligand compounds can effectively treat or prevent cereblon protein mediated diseases or disorders. The protein degrader compounds of Formula (II) can effectively treat or prevent diseases or disorders associated with the proteins degraded.

### Background

Bifunctional proteolysis-targeting drugs consist of three moieties, one end of which is a ligand warhead that can bind to a specific target protein, and the other end is a small molecule ligand of E3 ubiquitin ligase, both of which are connected together through linkers of different lengths and categories located in the middle. The bifunctional proteolysis-targeting small molecules can utilize the binding effects of the ligands at both ends to simultaneously bind to a specific target protein and E3 ubiquitin ligase, thereby recruiting E3 ubiquitin ligase to the vicinity of the specific target protein and enabling E3 ubiquitin ligase to ubiquitinate the target protein. The polyubiquitinated target protein will be recognized and degraded by proteasomes in the body. The biggest difference between the bifunctional proteolysis-targeting drugs and traditional small molecule-based inhibitor drugs is that the former mobilize the entire cell as a drug effector unit. This drug action mode only requires small molecule drugs to briefly bind to the target protein and tag it for degradation. Therefore, a low drug dose can meet the requirements, greatly reducing the risk of off-target effects, and potentially eliminating tumor progression caused by abnormal expression of driver genes and drug resistance caused by acquired mutations in driver genes.

The E3 ubiquitin ligases involved in the design of proteolysis-targeting drugs include over 500 different proteins, each with a distinct cellular expression profile, and can be classified into multiple categories based on the structural elements related to their E3 functional activity. Currently, only a few ligases have been successfully applied in preclinical degraders research. Among them, Cereblon (CRBN) E3 ubiquitin ligase is one of the most widely used E3 ubiquitin ligases. The known ligands for CRBN-type E3 ubiquitin ligase (CRBN ligands) include thalidomide and its analogs pomalidomide and lenalidomide, all of which possess a phthalimide backbone. CRBN-type E3 ubiquitin ligase ligands can also act as molecular glues to induce the degradation of specific proteins. Cereblon (CRBN) forms a functional E3 ubiquitin ligase complex with damaged DNA binding protein 1 (DDB1) and Cullin 4A, where CRBN serves as the substrate receptor. Molecular glue degraders like thalidomide bind to CRBN, inducing it to recognize new substrate proteins. The binding leads to the ubiquitination of these proteins, followed by their recognition and degradation by proteasomes.

CRBN ligands have been widely used in protein degradation, and a series of small molecules protein degraders based on CRBN ligands, which utilize the function of the CRBN E3 ubiquitin ligase complex, have gradually been developed. Since CRBN ligands can serve not only as molecular glue degraders to induce the degradation of different substrate proteins but also as a component of bifunctional proteolysis-targeting drugs to achieve effective degradation of specific pathogenic proteins, and given the unavoidable side effects and drug resistance issues associated with various existing domides compounds, such as thalidomide, it is of great significance to design novel and optimized E3 ubiquitin ligase ligands and study their binding ability to CRBN and evaluate their biological activity. Furthermore, these E3 ubiquitin ligase ligands can potentially be applied in bifunctional proteolysis-targeting small molecules.

Therefore, there is an urgent need for a series of novel CRBN E3 ubiquitin ligase ligands that can serve as effective molecular glue degraders and can also be further used to synthesize corresponding bifunctional proteolysis-targeting degraders for the treatment and/or prevention of diseases or conditions mediated or associated with the proteins degraded.

### Summary of Invention

In view of the above, the objectives of the present disclosure are to provide novel CRBN E3 ubiquitin ligase ligands, protein degrader molecules designed based on the novel CRBN E3 ubiquitin ligase ligands and various target protein ligands, as well as their applications and usage methods. The advantages of the compounds of the present disclosure lie in their ability to exhibit a wide range of pharmacological activities through the degradation/inhibition of diverse types or families of pathogenic proteins.

To achieve the above objectives and other related goals, in one aspect, the present disclosure provides novel CRBN E3 ubiquitin ligase ligands that can themselves act as molecular glues binding to CRBN E3 ligase, and subsequently induce the degradation of CRBN substrate proteins, including but not limited to GSPT1, IKZF1, IKZF2, IKZF3 (Aiolos), and IKZF4 proteins.

In some embodiments, the novel CRBN E3 ubiquitin ligase ligands of the present disclosure can be compounds of Formula (I): 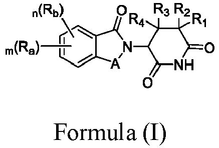
or salts, stereoisomers (including enantiomers), solvates, isotopically enriched analogs, prodrugs, or polymorphs thereof,
   wherein A represents CO, CH₂ or CD₂;
   R₁, R₂, R₃ and R₄ are the same or different and independently represent e.g., hydrogen, deuterium, halogen, optionally deuterated C₁₋₆ alkyl, optionally deuterated C₁₋₆ alkoxy, or halogenated C₁₋₆ alkyl;
   (Rₐ)ₘ indicates that benzene ring is substituted with m Rₐ substituents, with each Rₐ being the same or different and independently representing the structure of the following formula: 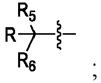
      wherein R represents chlorine, bromine, iodine, hydroxy, leaving group, NR₇R₈ or a group W¹,
         wherein R₇ and R₈ are the same or different and independently represent hydrogen, C₁₋₆ alkyl, C₂₋₆ alkynyl-C₁₋₆ alkylene, C₂₋₆ alkenyl-C₁₋₆ alkylene, optionally substituted 4- to 15-membered nitrogen-containing heterocyclyl, or R^{2a}-R^{1a}-, where R^{1a} is optionally substituted C₁₋₆ alkylene or optionally substituted C₂₋₆ alkenylene, R^{2a} represents halogen, amino, hydroxy, carboxyl, C₂₋₆ alkynyl, C₂₋₆ alkenyl or a leaving group; and
         W¹ represents the structure of the following formula: 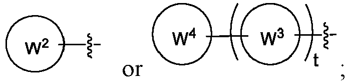 wherein ring W² represents optionally substituted nitrogen-containing heterocyclyl, each ring W³ is the same or different and independently represents optionally substituted nitrogen-containing heterocyclylene, t represents an integer of 1 or 2, and ring W⁴ represents optionally substituted aryl, optionally substituted heteroaryl or optionally substituted heterocyclyl;
      R₅ and R₆ are the same or different and independently represent hydrogen, fluorine, chlorine, bromine, iodine, optionally substituted methyl, or optionally substituted linear or branched C₂₋₃₀ alkyl, wherein one or more groups R_{c} and/or one or more groups R_{d} and/or any combination of one or more groups R_{c} and R_{d} are optionally inserted into the backbone carbon chain of the linear or branched C₂₋₃₀ alkyl group, wherein carbon-carbon bond between one or more pairs of adjacent carbon atoms in the backbone carbon chain is interrupted by the group R_{c}, R_{d}, or a combination of R_{c} and R_{d}; wherein each R_{c} is selected from the group consisting of O, N(Rₑ), C(O), C(O)O, S(O), S(O)₂, S(O)₂NH, NHS(O)₂, OC(O), C(O)N(Rₑ), N(Rₑ)C(O), or N(Rₑ)C(O)N(Rₑ), where each Rₑ independently represents H or C₁₋₆ alkyl, and in case that two or more groups R_{c} are inserted into the backbone carbon chain of the linear or branched C₂₋₃₀ alkyl group, the two or more groups R_{c} are not directly connected to each other; and wherein each R_{d} is selected from the group consisting of cycloalkylene, arylene, heterocyclylene, heteroarylene, alkynylene, alkenylene, or any combination thereof, wherein the cycloalkylene, arylene, heterocyclylene, and heteroarylene are independently optionally substituted with a substituent selected from the group consisting of deuterium, optionally deuterated C₁₋₆ alkyl, optionally deuterated C₃₋₆ cycloalkyl, hydroxy, amino, mercapto, halogen, cyano, optionally deuterated C₁₋₆ alkoxy, optionally deuterated C₁₋₆ alkyl-NH-, halogenated C₁₋₆ alkyl, NH₂-C₁₋₆ alkylene, optionally deuterated C₁₋₆ alkyl-NHC(O)-, optionally deuterated C₁₋₆ alkyl-C(O)NH-, or any combination thereof;
         or
      R and R₅, together with the carbon atom to which they are connected, form carbonyl group, R₆ represents hydrogen, optionally substituted methyl, or optionally substituted linear or branched C₂₋₃₀ alkyl, wherein one or more groups R_{c} and/or one or more groups R_{d} and/or any combination of one or more groups R_{c} and R_{d} are optionally inserted into the backbone carbon chain of the linear or branched C₂₋₃₀ alkyl group, wherein carbon-carbon bond between one or more pairs of adjacent carbon atoms in the backbone carbon chain is interrupted by the group R_{c}, R_{d}, or a combination of R_{c} and R_{d}; wherein each R_{c} is selected from the group consisting of O, N(Rₑ), C(O), C(O)O, S(O), S(O)₂, S(O)₂NH, NHS(O)₂, OC(O), C(O)N(Rₑ), N(Rₑ)C(O), or N(Rₑ)C(O)N(Rₑ), where each Rₑ independently represents H or C₁₋₆ alkyl, and in case that two or more groups R_{c} are inserted into the backbone carbon chain of the linear or branched C₂₋₃₀ alkyl group, the two or more groups R_{c} are not directly connected to each other; and wherein each R_{d} is selected from the group consisting of cycloalkylene, arylene, heterocyclylene, heteroarylene, alkynylene, alkenylene, or any combination thereof, wherein the cycloalkylene, arylene, heterocyclylene, and heteroarylene are independently optionally substituted with a substituent selected from the group consisting of deuterium, optionally deuterated C₁₋₆ alkyl, optionally deuterated C₃₋₆ cycloalkyl, hydroxy, amino, mercapto, halogen, optionally deuterated C₁₋₆ alkoxy, optionally deuterated C₁₋₆ alkyl-NH-, halogenated C₁₋₆ alkyl, NH₂-C₁₋₆ alkylene, optionally deuterated C₁₋₆ alkyl-NHC(O)-, optionally deuterated C₁₋₆ alkyl-C(O)NH-, cyano, or any combination thereof;
   (R_{b})ₙ indicates that the benzene ring is substituted with n R_{b} substituents, with each R_{b} being the same or different and independently representing deuterium, halogen, hydroxy, mercapto, nitro, amino, cyano, optionally deuterated C₁₋₆ alkyl, optionally deuterated C₁₋₆ alkoxy, halogenated C₁₋₆ alkyl, optionally substituted C₃₋₆ cycloalkyl, C₂₋₆ alkenyl or C₂₋₆ alkynyl; and
   m represents an integer of 1, 2, 3, or 4, n represents an integer of 0, 1, 2, or 3, and the sum of m and n is an integer of 1, 2, 3, or 4;
wherein when R and R₅ together with the carbon atom to which they are connected form a carbonyl group and R₆ represents hydrogen, m represents an integer of 1, 2, or 3, n represents an integer of 1, 2, or 3, and the sum of m and n is an integer of 2, 3, or 4;
with the proviso that the following compounds and compounds of Formula (I') are excluded: 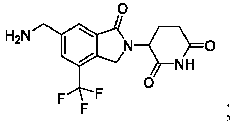 and
   when A represents CH₂ or C(O) and R represents unsubstituted piperazinyl, m represents an integer of 1 and n represents an integer of 0;
   when A represents CH₂ or C(O) and Rₐ represents H₂N-CH₂-, m represents an integer of 1 and n represents an integer of 0;
   when A represents C(O) and R represents bromine, m represents an integer of 1 and n represents an integer of 0; and
   the compounds of Formula (I'): 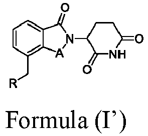
   wherein in Formula (I'), A represents CH₂ or C(O), and R represents unsubstituted piperidinyl or methylamino substituted piperidinyl.

In another aspect, the protein degrader compounds of the present disclosure can be compounds of Formula (II):

PBM-R_{f}-LIN-ULM Formula (II)

or salts, stereoisomers (including enantiomers), solvates, isotopically enriched analogs, prodrugs, or polymorphs thereof,
wherein PBM-R_{f}- represents a targeting moiety capable of binding protein, ULM represents a E3 ubiquitin ligase ligand moiety, LIN is a linker moiety, and PBM-R_{f}- is covalently bonded to ULM through LIN;
ULM represents the structure of Formula (ULM): 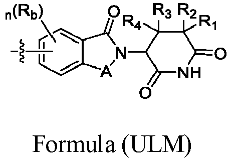
wherein A represents CO, CH₂ or CD₂;
R₁, R₂, R₃ and R₄ are the same or different and each independently represent hydrogen, deuterium, halogen, optionally deuterated C₁₋₆ alkyl, optionally deuterated C₁₋₆ alkoxy, or halogenated C₁₋₆ alkyl;
(R_{b})ₙ indicates that the benzene ring is optionally substituted with n R_{b} substituents, with each R_{b} being the same or different and independently representing deuterium, halogen, hydroxy, mercapto, nitro, amino, cyano, optionally deuterated C₁₋₆ alkyl, optionally deuterated C₁₋₆ alkoxy, halogenated C₁₋₆ alkyl, optionally substituted C₃₋₆ cycloalkyl, C₂₋₆ alkenyl or C₂₋₆ alkynyl;
n represents an integer of 0, 1, 2 or 3;
LIN represents the structure of the following formula: 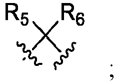
wherein R₅ and R₆ are the same or different and each independently represent hydrogen, fluorine, chlorine, bromine, iodine, optionally substituted methyl, or optionally substituted linear or branched C₂₋₃₀ alkyl, wherein one or more groups R_{c} and/or one or more groups R_{d} and/or any combination of one or more groups R_{c} and R_{d} are optionally inserted into the backbone carbon chain of the linear or branched C₂₋₃₀ alkyl group, wherein carbon-carbon bond between one or more pairs of adjacent carbon atoms in the backbone carbon chain is interrupted by the group R_{c}, R_{d}, or a combination of R_{c} and R_{d}; wherein each R_{c} is selected from the group consisting of O, N(Rₑ), C(O), C(O)O, S(O), S(O)₂, S(O)₂NH, NHS(O)₂, OC(O), C(O)N(Rₑ), N(Rₑ)C(O), or N(Rₑ)C(O)N(Rₑ), where each Rₑ independently represents H or C₁₋₆ alkyl, and in case that two or more groups R_{c} are inserted into the backbone carbon chain of the linear or branched C₂₋₃₀ alkyl group, the two or more groups R_{c} are not directly connected to each other; and wherein each R_{d} is selected from the group consisting of cycloalkylene, arylene, heterocyclylene, heteroarylene, alkynylene, alkenylene, or any combination thereof, wherein the cycloalkylene, arylene, heterocyclylene, and heteroarylene are each independently optionally substituted with a substituent selected from the group consisting of deuterium, optionally deuterated C₁₋₆ alkyl, optionally deuterated C₃₋₆ cycloalkyl, hydroxy, amino, mercapto, halogen, cyano, optionally deuterated C₁₋₆ alkoxy, optionally deuterated C₁₋₆ alkyl-NH-, halogenated C₁₋₆ alkyl, NH₂-C₁₋₆ alkylene, optionally deuterated C₁₋₆ alkyl-NHC(O)-, optionally deuterated C₁₋₆ alkyl-C(O)NH-, or any combination thereof;
R_{f} represents a bond, -O-, -N(R_{g})-, -NHC(O)-Rₕ-W⁵-*, -C(O)NH-Rₕ-W⁵-*, -NHC(O)-W⁵-*, - C(O)NH-W⁵-*, -NHC(O)-*, -C(O)NH-*, -O-Rₕ-W⁵-*, -O-W⁵-*, -O-Rₕ-N(Rᵢ)-*, -N(R_{g})-Rₕ-N(Rᵢ)-*, - W⁵-, -W⁵-N(R_{g})-*, -N(R_{g})-W⁵-*, -N(R_{g})-W⁵-N(Rᵢ)-*, -Rₕ-W⁵-*, -Rₕ-C(O)-W⁵-*, -C(O)-W⁵-*, -Rₕ-C(O)NH-Rⱼ-W⁵-*, -Rₕ-NHC(O)-Rⱼ-W⁵-*, -Rₕ-C(O)NH-*, -Rₕ-NHC(O)-*, -Rₕ-, -Rₕ-N(Rᵢ)-*, or 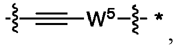
   wherein W⁵ represents the structure of the following formula: 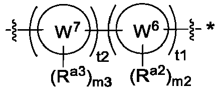
   wherein each ring W⁶ is the same or different and each independently represents nitrogen-containing heterocyclylene, t1 represents an integer of 1 or 2, and (R^{a2})ₘ₂ indicates that each ring W⁶ is optionally substituted with m2 R^{a2} substituents, with each R^{a2} being independently deuterium, optionally deuterated C₁₋₆ alkyl, optionally halogenated C₁₋₆ alkyl, C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{12a}-R^{11a}-, where R^{11a} is optionally substituted C₁₋₆ alkylene or optionally substituted C₂₋₆ alkenylene, and R^{12a} represents halogen, R^{13a}R^{14a}N-, hydroxy, carboxyl, ethynyl, or vinyl, wherein R^{13a} and R^{14a} are the same or different and each independently represent hydrogen or C₁₋₃ alkyl, and m2 represents an integer of 0-20;
   each ring W⁷ is the same or different and each independently represents nitrogen-containing heterocyclylene, arylene, or heteroarylene, t2 represents an integer of 0 or 1, and (R^{a3})ₘ₃ indicates that each ring W⁷ is independently optionally substituted with m3 R^{a3} substituents, with each R^{a3} being independently deuterium, optionally deuterated C₁₋₆ alkyl, C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{16a}-R^{15a}-, where R^{15a} is optionally substituted C₁₋₆ alkylene or optionally substituted C₂₋₆ alkenylene, and R^{16a} represents halogen, R^{17a}R^{18a}N-, hydroxy, carboxyl, ethynyl, or vinyl, wherein R^{17a} and R^{18a} are the same or different and each independently represent hydrogen or C₁₋₃ alkyl, and m3 represents an integer of 0-20; and
   R_{g} and Rᵢ each independently represent hydrogen or C₁₋₆ alkyl, Rₕ and Rⱼ each independently represent optionally substituted C₁₋₆ alkylene or optionally substituted C₂₋₆ alkenylene, and symbol * indicates the point of attachment to LIN;
wherein when PBM represents the structure of the following formula: 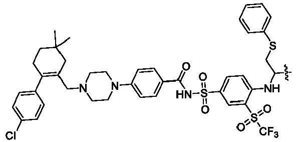 and simultaneously -R_{f}-LIN- represents 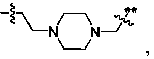 where symbol ** indicates the point of attachment to ULM, (R_{b})ₙ indicates that benzene ring of Formula (ULM) is substituted with 1, 2, or 3 R_{b}, with each R_{b} being the same or different and independently representing deuterium, halogen, hydroxy, mercapto, nitro, amino, cyano, optionally deuterated C₁₋₆ alkyl, optionally deuterated C₁₋₆ alkoxy, halogenated C₁₋₆ alkyl, optionally substituted C₃₋₆ cycloalkyl, C₂₋₆ alkenyl, or C₂₋₆ alkynyl;
with the proviso that the following compounds are excluded:
   3-(4-((3-(4-amino-3-(4-phenoxyphenyl)-1H-pyrazolo[3,4-*d*]pyrimidin-1-yl)piperidin-1 - yl)methyl)-1-oxoisoindolin-2-yl)piperidine-2,6-dione;
   N-(5-(3,5-difluorobenzyl)-1H-indazol-3-yl)-4-(4-(1-((2-(2,6-dioxopiperidin-3-yl)-1-oxoisoindolin-5-yl)methyl)piperidin-3-yl)piperazin-1-yl)-2-((tetrahydro-2H-pyran-4-yl)amino)benzamide;
   N-(5-(3,5-difluorobenzyl)-1H-indazol-3-yl)-4-(4-((2-(2,6-dioxopiperidin-3-yl)-1,3-dioxoisoindolin-5-yl)methyl)piperazin-1-yl)-2-((tetrahydro-2H-pyran-4-yl)amino)benzamide;
   N-(5-(3,5-difluorobenzyl)-1H-indazol-3-yl)-4-(4-(1-((2-(2,6-dioxopiperidin-3-yl)-3-oxoisoindolin-5-yl)methyl)piperidin-3-yl)piperazin-1-yl)-2-((tetrahydro-2H-pyran-4-yl)amino)benzamide;
   N-(5-(3,5-difluorobenzyl)-1H-indazol-3-yl)-4-(4-(1-((2-(2,6-dioxopiperidin-3-yl)-1,3-dioxoisoindolin-5-yl)methyl)piperidin-4-yl)piperazin-1-yl)-2-((tetrahydro-2H-pyran-4-yl)amino)benzamide;
   N-(5-(3,5-difluorobenzyl)-1H-indazol-3-yl)-4-(4-(1-((2-(2,6-dioxopiperidin-3-yl)-1,3-dioxoisoindolin-5-yl)methyl)pyrrolidin-3-yl)piperazin-1-yl)-2-((tetrahydro-2H-pyran-4-yl)amino)benzamide;
   N-(5-(3,5-difluorobenzyl)-1H-indazol-3-yl)-4-(4-(1-((2-(2,6-dioxopiperidin-3-yl)-1,3-dioxoisoindolin-5-yl)methyl)piperidin-3-yl)piperazin-1-yl)-2-((tetrahydro-2H-pyran-4-yl)amino)benzamide;
   N-(5-(3,5-difluorobenzyl)-1H-indazol-3-yl)-4-(4-(1-((2-(2,6-dioxopiperidin-3-yl)-1,3-dioxoisoindolin-5-yl)methyl)azetidin-3-yl)piperazin-1-yl)-2-((tetrahydro-2H-pyran-4-yl)amino)benzamide;
   2-(2,6-dioxopiperidin-3-yl)-5-((4-(6-(6-(2-(3-fluorophenyl)pyrrolidin-1-yl)imidazo[1,2-b]pyridazin-3-yl)pyridin-2-yl)piperazin-1-yl)methyl)isoindoline-1,3-dione; and
   2-(2,6-dioxopiperidin-3-yl)-5-((3-(4-(6-(6-(2-(3-fluorophenyl)pyrrolidin-1-yl)imidazo[1,2-b]pyridazin-3 -yl)pyridin-2-yl)piperazin-1-yl)azetidin-1-yl)methyl)isoindoline-1,3 -dione.

In a further aspect, the present disclosure provides a pharmaceutical composition comprising the compound of Formula (I) or salts, stereoisomers (including enantiomers), solvates, isotopically enriched analogs, prodrugs, or polymorphs thereof, and at least one pharmaceutically acceptable carrier.

In a further aspect, the present disclosure provides a pharmaceutical composition comprising the compound of Formula (II) or salts, stereoisomers (including enantiomers), solvates, isotopically enriched analogs, prodrugs, or polymorphs thereof, and at least one pharmaceutically acceptable carrier.

In a further aspect, the present disclosure further provides a medicine kit or reagent kit comprising: the compound of Formula (I) or a pharmaceutically acceptable salt, or the pharmaceutical composition comprising the same; or the compound of Formula (II) or a pharmaceutically acceptable salt, or the pharmaceutical composition comprising the same.

In a further aspect, the present disclosure provides the compound of Formula (I) or salts, stereoisomers (including enantiomers), solvates, isotopically enriched analogs, prodrugs, or polymorphs thereof, or the pharmaceutical composition comprising the same as active ingredient for use as a medicament.

In a further aspect, the present disclosure provides the compound of Formula (I) or salts, stereoisomers (including enantiomers), solvates, isotopically enriched analogs, prodrugs, or polymorphs thereof, or the pharmaceutical composition comprising the same as active ingredient for use in the prevention or treatment of cereblon protein-mediated disease or disorder.

In some embodiments, the cereblon protein-mediated disease or disorder includes, but not limited to, tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, cardiovascular disease, Unverricht's syndrome, Richter syndrome (RS), acute liver failure, or diabetes.

In a further aspect, the present disclosure provides the compound of Formula (II) or salts, stereoisomers (including enantiomers), solvates, isotopically enriched analogs, prodrugs, or polymorphs thereof, or the pharmaceutical composition comprising the same as active ingredient for use as a medicament.

In a further aspect, the present disclosure provides the compound of Formula (II) or salts, stereoisomers (including enantiomers), solvates, isotopically enriched analogs, prodrugs, or polymorphs thereof, or the pharmaceutical composition comprising the same as active ingredient for use in the treatment and/or prevention of a disease or disorder selected from the group consisting of tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, Unverricht's syndrome, Richter syndrome (RS), acute liver failure, diabetes, Kennedy disease, seborrheic alopecia, hirsutism, skin disease, cardiovascular disease, dysfunctional uterine bleeding, anemia, pediatric aplastic anemia, endometriosis, transplant rejection, polycystic ovary syndrome, and thyroid disease.

In a further aspect, the present disclosure provides use of the compound of Formula (I) or salts, stereoisomers (including enantiomers), solvates, isotopically enriched analogs, prodrugs, or polymorphs thereof for the preparation of the compound of Formula (II) or salts, enantiomers, stereoisomers, solvates, isotopically enriched analogs, prodrugs, or polymorphs thereof of the present disclosure.

In a further aspect, the present disclosure also provides a method for preparing the compound of Formula (II) or salts, enantiomers, stereoisomers, solvates, isotopically enriched analogs, prodrugs, or polymorphs thereof of the present disclosure by using the compound of Formula (I) or salts, stereoisomers (including enantiomers), solvates, isotopically enriched analogs, prodrugs, or polymorphs thereof.

In a further aspect, the present disclosure provides use of the compound of Formula (I) or salts, stereoisomers (including enantiomers), solvates, isotopically enriched analogs, prodrugs, or polymorphs thereof for the manufacture of a medicament for the prevention or treatment of cereblon protein-mediated disease or disorder.

In a further aspect, the present disclosure provides use of the compound of Formula (II) or salts, stereoisomers (including enantiomers), solvates, isotopically enriched analogs, prodrugs, or polymorphs thereof for the manufacture of a medicament for the prevention and/or treatment of a disease or disorder selected from the group consisting of tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, Unverricht's syndrome, Richter syndrome (RS), acute liver failure, diabetes, Kennedy disease, seborrheic alopecia, hirsutism, skin disease, cardiovascular disease, dysfunctional uterine bleeding, anemia, pediatric aplastic anemia, endometriosis, transplant rejection, polycystic ovary syndrome, and thyroid disease.

In a further aspect, the present disclosure provides a method for treating or preventing a cereblon protein-mediated disease or disorder in a subject, comprising administering to the subject a therapeutically effective amount of the compound of Formula (I) or salts, enantiomers, stereoisomers, solvates, isotopically enriched analogs, prodrugs, or polymorphs thereof, or the pharmaceutical composition comprising the same.

In a further aspect, the present disclosure provides a method for treating or preventing a disease or disorder in a subject, comprising administering to the subject a therapeutically effective amount of the compound of Formula (II) or salts, enantiomers, stereoisomers, solvates, isotopically enriched analogs, prodrugs, or polymorphs thereof, or the pharmaceutical composition comprising the same, wherein the disease or disorder comprises tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, Unverricht's syndrome, Richter syndrome (RS), acute liver failure, diabetes, Kennedy disease, seborrheic alopecia, hirsutism, skin disease, cardiovascular disease, dysfunctional uterine bleeding, anemia, pediatric aplastic anemia, endometriosis, transplant rejection, polycystic ovary syndrome, and thyroid disease.

### Detailed Description of the Invention

The following detailed description is provided as exemplary specific embodiments to assist those skilled in the art in understanding and practicing the present disclosure. It should be appreciated, however, that such description is not intended to limit the scope of the present disclosure, and that various modifications and changes may be made to the specific embodiments described in the present disclosure without departing from the spirit and scope of the present disclosure. Such changes and modifications are to be understood as being included within the scope of the present invention as defined by the appended claims.

### I. Compounds

### Compounds of Formula (I)

The present disclosure provides a compound of Formula (I) or salts (including pharmaceutically acceptable salts), stereoisomers (including enantiomers), solvates, isotopically enriched analogs, prodrugs, or polymorphs thereof: 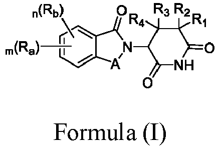 wherein A, R₁, R₂, R₃, R₄, (Rₐ)ₘ, (R_{b})ₙ, m and n are s defined in the compounds of Formula (I) above.

In some embodiments, R₁, R₂, R₃ and R₄ of the compound of Formula (I) are the same or different and each independently represent hydrogen, deuterium, halogen (e.g., fluorine, chlorine, bromine, or iodine), optionally deuterated C₁₋₆ alkyl (e.g., optionally deuterated C₁₋₄ alkyl, such as methyl, CD₃, ethyl, propyl, isopropyl, butyl, sec-butyl or tert-butyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy or tert-butoxy), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R₁, R₂, R₃ and R₄ of the compound of Formula (I) are the same or different and each independently represent hydrogen or deuterium.

In some embodiments, R₁, R₂, R₃ and R₄ of the compound of Formula (I) represent hydrogen.

In some embodiments, A of the compound of Formula (I) represents CO.

In some embodiments, A of the compound of Formula (I) represents CH₂.

In some embodiments, A of the compound of Formula (I) represents CD₂.

In some embodiments, m of the compound of Formula (I) represents an integer of 1, n represents an integer of 0, 1, 2, or 3, and the sum of m and n is an integer of 1, 2, 3, or 4.

In some embodiments, m of the compound of Formula (I) represents an integer of 2, n represents an integer of 0, 1, or 2, and the sum of m and n is an integer of 2, 3, or 4.

In some embodiments, when R and R₅ together with the carbon atom to which they are connected form a carbonyl group and R₆ represents hydrogen, m represents an integer of 1, 2, or 3, n represents an integer of 1, 2, or 3, and the sum of m and n is an integer of 2, 3, or 4. In some sub-embodiments, m represents an integer of 1, n represents an integer of 1, 2, or 3, and the sum of m and n is an integer of 2, 3, or 4. In some sub-embodiments, m represents an integer of 2, n represents an integer of 1 or 2, and the sum of m and n is an integer of 3 or 4. In some sub-embodiments, m represents an integer of 3, n represents an integer of 1.

In some embodiments, R of the compound of Formula (I) represents chlorine, bromine, iodine, hydroxy, leaving group (e.g., -N₃, chlorine, bromine, iodine, sulfonate, e.g., methanesulfonyloxy (MsO-), trifluoromethanesulfonyloxy (TfO-), or p-toluenesulfonyloxy (TsO-)).

In some embodiments, R of the compound of Formula (I) represents NR₇R₈, wherein R₇ and R₈ are the same or different and each independently represent hydrogen, C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), C₂₋₆ alkynyl-C₁₋₆ alkylene- (e.g., C₂₋₆ alkynyl-C₁₋₄ alkylene- or C₂₋₆ alkynyl-C₁₋₃ alkylene-, such as ethynyl-methylene-, ethynyl-ethylene-, ethynyl-propylene-, ethynyl-butylene-, ethynyl-pentylene-, or ethynyl-hexylene-), C₂₋₆ alkenyl-C₁₋₆ alkylene- (e.g., C₂₋₆ alkenyl-C₁₋₄ alkylene- or C₂₋₆ alkenyl-C₁₋₃ alkylene-, such as vinyl-methylene-, vinyl-ethylene-, vinyl-propylene-, vinyl-butylene-, vinyl-pentylene-, or vinyl-hexylene-), optionally substituted 4- to 15-membered (e.g., 4- to 11-membered, 4- to 10-membered, 4- to 9-membered, 4- to 8-membered, 4- to 7-membered, 4- to 6-membered, 5- to 15-membered, or 5- to 9-membered) nitrogen-containing monocyclic heterocyclyl, optionally substituted 4- to 15-membered (e.g., 4- to 11-membered, 4- to 10-membered, 4- to 9-membered, 4- to 8-membered, 4- to 7-membered, 4- to 6-membered, 5- to 15-membered, or 5- to 9-membered) nitrogen-containing bridged heterocyclyl, optionally substituted 4- to 15-membered (e.g., 4- to 11-membered, 4- to 10-membered, 4- to 9-membered, 4- to 8-membered, 4- to 7-membered, 4- to 6-membered, 5- to 15-membered, or 5- to 9-membered) nitrogen-containing spiro-heterocyclyl, or R^{2a}-R^{1a}-, where R^{1a} is optionally substituted C₁₋₆ alkylene (e.g., optionally substituted C₁₋₄ alkylene, such as optionally substituted methylene, ethylene, or propylene) or optionally substituted C₂₋₆ alkenylene (e.g., optionally substituted C₂₋₄ alkenylene, such as optionally substituted vinylene, propenylene, or butenylene), and R^{2a} represents halogen (e.g., fluorine, chlorine, bromine, or iodine), amino, hydroxy, carboxyl, CHO, C₂₋₆ alkynyl (e.g., C₂₋₄ alkynyl, such as ethynyl, propynyl, or butynyl), C₂₋₆ alkenyl (e.g., C₂₋₄ alkenyl, such as vinyl, propenyl, or butenyl) or a leaving group (e.g., -N₃, chlorine, bromine, iodine, sulfonate, e.g., methanesulfonyloxy (MsO-), trifluoromethanesulfonyloxy (TfO-), or p-toluenesulfonyloxy (TsO-)). The 4- to 15-membered nitrogen-containing monocyclic heterocyclyl, the 4- to 15-membered nitrogen-containing bridged heterocyclyl, and the 4- to 15-membered nitrogen-containing spiro-heterocyclyl are each independently optionally substituted with one or more (e.g., 1-10, such as 1, 2, 3, 4, 5, 6, 8, or 10) substituents selected from the group consisting of deuterium, C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, tert-butoxy, pentyloxy, or hexyloxy), halogen (e.g., fluorine, chlorine, bromine, or iodine), leaving group (e.g., -N₃, chlorine, bromine, iodine, sulfonate, e.g., methanesulfonyloxy (MsO-), trifluoromethanesulfonyloxy (TfO-), or p-toluenesulfonyloxy (TsO-)), amino, hydroxy, carboxyl, CHO, C₂₋₆ alkynyl (e.g., C₂₋₄ alkynyl, such as ethynyl, propynyl, or butynyl), C₂₋₆ alkenyl (e.g., C₂₋₄ alkenyl, such as vinyl, propenyl, or butenyl), R^{4a}-R^{3a}-, or any combination thereof, where R^{3a} represents optionally substituted C₁₋₆ alkylene (e.g., optionally substituted C₁₋₄ alkylene, such as optionally substituted methylene, ethylene, or propylene) or optionally substituted C₂₋₆ alkenylene (e.g., optionally substituted C₂₋₄ alkenylene, such as optionally substituted vinylene, propenylene, or butenylene), R^{4a} represents halogen, R^{5a}R^{6a}N-, hydroxy, carboxyl, CHO, ethynyl, vinyl, or leaving group (e.g., -N₃, chlorine, bromine, iodine, sulfonate, e.g., methanesulfonyloxy (MsO-), trifluoromethanesulfonyloxy (TfO-), or p-toluenesulfonyloxy (TsO-)), wherein R^{5a} and R^{6a} are the same or different and each independently represent hydrogen or C₁₋₃ alkyl (e.g., methyl, ethyl, or propyl). The number of substituents is not theoretically limited in any way or automatically limited by the size of the building units. For example, when the substituent is deuterium, the 4- to 15-membered nitrogen-containing monocyclic heterocyclyl, the 4- to 15-membered nitrogen-containing bridged heterocyclyl, and the 4-to 15-membered nitrogen-containing spiro-heterocyclyl are each independently optionally perdeuterated or partially deuterated.

In some sub-embodiments, R of the compound of Formula (I) represents NR₇R₈, wherein R₇ and R₈ are the same or different and each independently represent:
hydrogen, C₁₋₆ alkyl, C₂₋₆ alkynyl-C₁₋₆ alkylene-, or C₂₋₆ alkenyl-C₁₋₆ alkylene-; or
optionally substituted 4- to 15-membered nitrogen-containing monocyclic heterocyclyl, optionally substituted 4- to 15-membered nitrogen-containing bridged heterocyclyl, or optionally substituted 4- to 15-membered nitrogen-containing spiro-heterocyclyl, e.g.,
   piperidinyl, piperazinyl, morpholinyl, azetidinyl, pyrrolidinyl, imidazolidinyl, pyrazolidyl, oxazolidinyl, thiazolidinyl, thiomorpholinyl, azepanyl, diazacycloheptanyl, azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl, diazabicyclo[2.2.2]octanyl, 3-azaspiro[5.5]undecan-3-yl, 8-azaspiro[4.5]decan-4-yl, 2-oxa-8-azaspiro[4.5]decan-4-yl, or 3-methyl-2-oxa-8-azaspiro[4.5]decan-4-yl, or 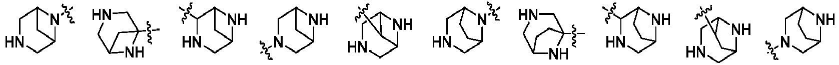 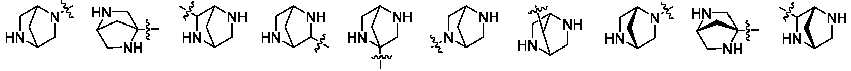 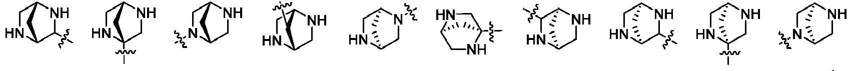 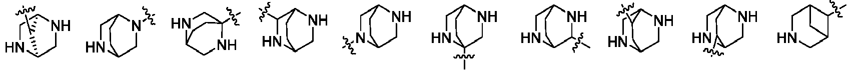 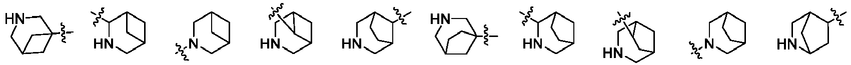 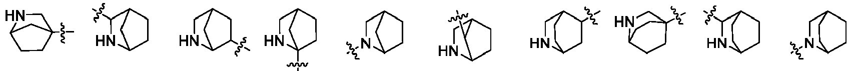 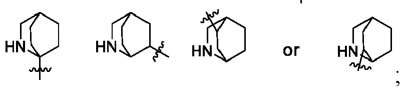 or
R^{2a}-R^{1a}-, wherein R^{1a} represents optionally substituted C₁₋₆ alkylene or optionally substituted C₂₋₆ alkenylene, and R^{2a} represents halogen, amino, hydroxy, carboxyl, CHO, C₂₋₆ alkynyl, C₂₋₆ alkenyl, or leaving group, and
wherein the 4- to 15-membered nitrogen-containing monocyclic heterocyclyl, 4- to 15-membered nitrogen-containing bridged heterocyclyl and 4- to 15-membered nitrogen-containing spiro-heterocyclyl are each independently optionally substituted with one or more (e.g., 1-10, such as 1, 2, 3, 4, 5, 6, 8, or 10) substituents selected from the group consisting of deuterium, C₁₋₆ alkyl, C₁₋₆ alkoxy, halogen, leaving group, amino, hydroxy, carboxyl, CHO, C₂₋₆ alkynyl, C₂₋₆ alkenyl, R^{4a}-R^{3a}-, or any combination thereof, wherein R^{3a} represents optionally substituted C₁₋₆ alkylene or optionally substituted C₂₋₆ alkenylene, and R^{4a} represents halogen, R^{5a}R^{6a}N-, hydroxy, carboxyl, CHO, ethynyl, vinyl, or leaving group, wherein R^{5a} and R^{6a} are the same or different and each independently represent hydrogen or C₁₋₃ alkyl. The number of substituents is not theoretically limited in any way or automatically limited by the size of the building units. For example, when the substituent is deuterium, the 4- to 15-membered nitrogen-containing monocyclic heterocyclyl, the 4- to 15-membered nitrogen-containing bridged heterocyclyl, and the 4- to 15-membered nitrogen-containing spiro-heterocyclyl are each independently optionally perdeuterated or partially deuterated.

In some embodiments, R of the compound of Formula (I) represents NH₂, -NHCH₃, - NHCH₂CH₃, -N(CH₃)₂, -N(CH₃)-CH₂CH₃, -N(CH₃)-CH₂CH₂Br, 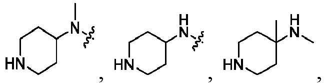 or

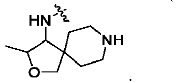

In some embodiments, R of the compound of Formula (I) represents W¹ represented by the structure of the following formula: 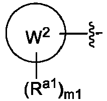
wherein ring W² represents 4- to 20-membered (e.g., 4- to 15-membered, 4- to 12-membered, 4-to 11-membered, 4- to 10-membered, 4- to 9-membered, 4- to 8-membered, 4- to 7-membered, 4- to 6-membered, 5- to 15-membered, or 5- to 9-membered) nitrogen-containing heterocyclyl, e.g., 4- to 20-membered (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) heterocyclyl containing one nitrogen atom and optionally a heteroatom selected from the group consisting of oxygen, nitrogen, and sulfur,
(R^{a1})ₘ₁ indicates that ring W² is optionally substituted with m1 R^{a1} substituents, with each R^{a1} being independently deuterium, optionally deuterated C₁₋₆ alkyl (e.g., optionally deuterated C₁₋₄ alkyl, such as methyl, CD₃, ethyl, propyl, isopropyl, butyl, sec-butyl, or tert-butyl), C₁₋₆ alkoxy (e.g., C₁₋₄ alkoxy, such as methoxy, CD₃-O-, ethoxy, propoxy, isopropoxy, n-butoxy, isobutoxy, or tert-butoxy), halogen (e.g., fluorine, chlorine, bromine, or iodine), leaving group (e.g., -N₃, chlorine, bromine, iodine, sulfonate, e.g., methanesulfonyloxy (MsO-), trifluoromethanesulfonyloxy (TfO-) or p-toluenesulfonyloxy (TsO-)), amino, hydroxy, mercapto, cyano, carboxyl, CHO, C₂₋₆ alkynyl (e.g., C₂₋₄ alkynyl, such as ethynyl, propynyl, or butynyl), C₂₋₆ alkenyl (e.g., C₂₋₄ alkenyl, such as vinyl, propenyl, or butenyl), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), or R^{8a}-R^{7a}-, wherein R^{7a} is optionally substituted C₁₋₆ alkylene (e.g., optionally substituted C₁₋₄ alkylene, such as optionally substituted methylene, ethylene or propylene) or optionally substituted C₂₋₆ alkenylene (e.g., optionally substituted C₂₋₄ alkenylene, such as optionally substituted vinylene, propenylene, butenylene); and R^{8a} represents halogen (e.g., fluorine, chlorine, bromine, or iodine), R^{9a}R^{10a}N-, hydroxy, carboxyl, CHO, ethynyl, vinyl, or leaving group (e.g., -N₃, chlorine, bromine, iodine, sulfonate, e.g., methanesulfonyloxy (MsO-), trifluoromethanesulfonyloxy (TfO-) or p-toluenesulfonyloxy (TsO-)), wherein R^{9a} and R^{10a} are the same or different and each independently represent hydrogen or C₁₋₃ alkyl (e.g., methyl, ethyl, or propyl);
wherein m1 represents an integer of 0-20 (e.g., an integer of 0, 1, 2, 3, 4, 5, 6, 7, 8, 9, 10, 11, 12, 13, 14, 15, or 20). The number of substituents is not theoretically limited in any way or automatically limited by the size of the building units.

In some sub-embodiments, R of the compound of Formula (I) represents W¹ represented by the structure of the following formula: 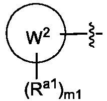
wherein ring W² represents 4- to 20-membered nitrogen-containing heterocyclyl, e.g., 4- to 20-membered (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) heterocyclyl containing one nitrogen atom and optionally a heteroatom selected from the group consisting of oxygen, nitrogen, and sulfur, e.g.,
   piperidinyl, piperazinyl, morpholinyl, azetidinyl, pyrrolidinyl, imidazolidinyl, pyrazolidyl, oxazolidinyl, thiazolidinyl, thiomorpholinyl, azepanyl, diazacycloheptanyl, azacyclooctyl, diazacyclooctyl, azabicyclo[3.1. 1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl, diazabicyclo[2.2.2]octanyl, 3-azaspiro[5.5]undecan-3-yl, 8-azaspiro[4.5]decan-4-yl, 2-oxa-8-azaspiro[4.5]decan-4-yl, or 3-methyl-2-oxa-8-azaspiro[4.5]decan-4-yl, or 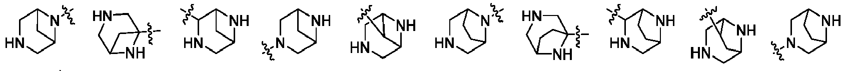 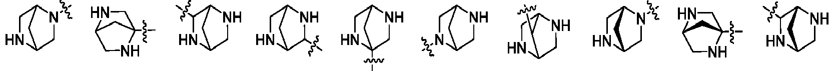 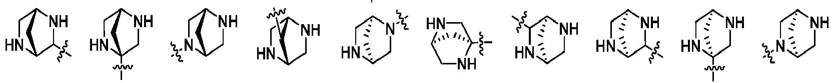 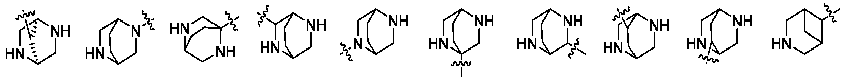 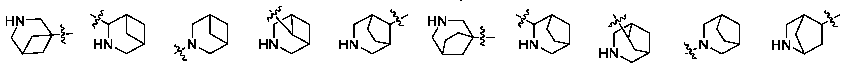 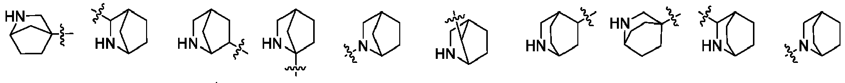 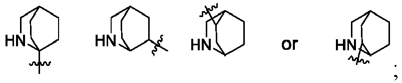
(R^{a1})ₘ₁ indicates that ring W² is optionally substituted with m1 R^{a1} substituents, with each R^{a1} being independently deuterium, optionally deuterated C₁₋₆ alkyl, C₁₋₆ alkoxy, halogen, leaving group, amino, hydroxy, mercapto, cyano, carboxyl, CHO, C₂₋₆ alkynyl, C₂₋₆ alkenyl, optionally deuterated C₃₋₆ cycloalkyl, or R^{8a}-R^{7a}-, wherein R^{7a} is optionally substituted C₁₋₆ alkylene or optionally substituted C₂₋₆ alkenylene, and R^{8a} represents halogen, R^{9a}R^{10a}N-, hydroxy, carboxyl, CHO, ethynyl, vinyl, or leaving group, wherein R^{9a} and R^{10a} are the same or different and each independently represent hydrogen or C₁₋₃ alkyl;
wherein m1 represents an integer of 0-20 (e.g., an integer of 0, 1, 2, 3, 4, 5, 6, 7, 8, 9, 10, 11, 12, 13, 14, 15, or 20). The number of substituents is not theoretically limited in any way or automatically limited by the size of the building units.

In some embodiments, ring W² is optionally perdeuterated or partially deuterated.

In some embodiments, R of the compound of Formula (I) represents W¹ represented by the structure of the following formula: 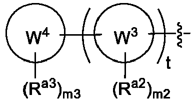
wherein each ring W³ is the same or different and each independently represents 4- to 20-membered (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) nitrogen-containing heterocyclylene, e.g., 4- to 20-membered (e.g., 4- to 15-membered, 4-to 12-membered, 4- to 11-membered, 4- to 10-membered, 4- to 9-membered, 4- to 8-membered, 4- to 7-membered, 4- to 6-membered, 5- to 15-membered, or 5- to 9-membered) heterocyclylene containing one nitrogen atom and optionally a heteroatom selected from the group consisting of oxygen, nitrogen, and sulfur;
t represents an integer of 1 or 2, and (R^{a2})ₘ₂ indicates that each ring W³ is optionally substituted with m2 R^{a2} substituents, with each R^{a2} being independently deuterium, optionally deuterated C₁₋₆ alkyl (e.g., optionally deuterated C₁₋₄ alkyl, such as methyl, CD₃, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, sec-butoxy, tert-butoxy, pentyloxy, or hexyloxy), halogen (e.g., fluorine, chlorine, bromine, or iodine), leaving group (e.g., -N₃, chlorine, bromine, iodine, sulfonate, e.g., methanesulfonyloxy (MsO-), trifluoromethanesulfonyloxy (TfO-) or p-toluenesulfonyloxy (TsO-)), amino, hydroxy, mercapto, cyano, carboxyl, CHO, C₂₋₆ alkynyl (e.g., C₂₋₄ alkynyl, such as ethynyl, propynyl, or butynyl), C₂₋₆ alkenyl (e.g., C₂₋₄ alkenyl, such as vinyl, propenyl, or butenyl), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), or R^{12a}-R^{11a}-, wherein R^{11a} is optionally substituted C₁₋₆ alkylene (e.g., optionally substituted C₁₋₄ alkylene, such as optionally substituted methylene, ethylene or propylene) or optionally substituted C₂₋₆ alkenylene (e.g., optionally substituted C₂₋₄ alkenylene, such as optionally substituted methylene, ethylene or propylene), and R^{12a} represents halogen (e.g., fluorine, chlorine, bromine, or iodine), R^{13a}R^{14a}N-, hydroxy, carboxyl, CHO, ethynyl, vinyl, or leaving group (e.g., -N₃, chlorine, bromine, iodine, sulfonate, e.g., methanesulfonyloxy (MsO-), trifluoromethanesulfonyloxy (TfO-), or p-toluenesulfonyloxy (TsO-)), wherein R^{13a} and R^{14a} are the same or different and each independently represent hydrogen or C₁₋₃ alkyl (e.g., methyl, ethyl, or propyl); and
ring W⁴ represents 4- to 15-membered (e.g., 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) nitrogen-containing heterocyclyl (e.g., 4- to 15-membered (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) heterocyclyl containing one nitrogen atom and optionally a heteroatom selected from the group consisting of oxygen, nitrogen, and sulfur), 6- to 10-membered aryl, or 5- to 10-membered (e.g., 5- to 6-membered, or 5- to 9-membered) heteroaryl (e.g., 5- to 10-membered (e.g., 5- to 9-membered, 5- to 8-membered, 5- to 7-membered, or 5- to 6-membered) heteroaryl containing one nitrogen atom and optionally a heteroatom selected from the group consisting of oxygen, nitrogen, and sulfur);
(R^{a3})ₘ₃ indicates that ring W⁴ is optionally substituted with m3 R^{a3} substituents, with each R^{a3} independently representing deuterium, optionally deuterated C₁₋₆ alkyl (e.g., optionally deuterated C₁₋₄ alkyl, such as methyl, CD₃, ethyl, propyl, isopropyl, butyl, sec-butyl, or tert-butyl), C₁₋₆ alkoxy (e.g., C₁₋₄ alkoxy, such as methoxy, ethoxy, propoxy, isopropoxy, n-butoxy, sec-butoxy, tert-butoxy, pentyloxy, or hexyloxy), halogen (e.g., fluorine, chlorine, bromine, or iodine), leaving group (e.g., -N₃, chlorine, bromine, iodine, sulfonate, e.g., methanesulfonyloxy (MsO-), trifluoromethanesulfonyloxy (TfO-) or p-toluenesulfonyloxy (TsO-)), amino, hydroxy, mercapto, cyano, carboxyl, CHO, C₂₋₆ alkynyl (e.g., C₂₋₄ alkynyl, such as ethynyl, propynyl, or butynyl), C₂₋₆ alkenyl (e.g., C₂₋₄ alkenyl, such as vinyl, propenyl, or butenyl), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), or R^{16a}-R^{15a}-, wherein R^{15a} is optionally substituted C₁₋₆ alkylene (e.g., optionally substituted C₁₋₄ alkylene, such as optionally substituted methylene, ethylene or propylene) or optionally substituted C₂₋₆ alkenylene (e.g., optionally substituted C₂₋₄ alkenylene, such as optionally substituted vinylene, propenylene, butenylene), and R^{16a} represents halogen (e.g., fluorine, chlorine, bromine, or iodine), R^{17a}R^{18a}N-, hydroxy, carboxyl, CHO, ethynyl, vinyl, or leaving group (e.g., -N₃, chlorine, bromine, iodine, sulfonate, e.g., methanesulfonyloxy (MsO-), trifluoromethanesulfonyloxy (TfO-) or p-toluenesulfonyloxy (TsO-)), wherein R^{17a} and R^{18a} are the same or different and each independently represent hydrogen or C₁₋₃ alkyl (e.g., methyl, ethyl, or propyl);
wherein m2 and m3 each independently represent an integer of 0-20 (e.g., an integer of 0, 1, 2, 3, 4, 5, 6, 7, 8, 9, 10, 11, 12, 13, 14, 15, or 20). The number of substituents is not theoretically limited in any way or automatically limited by the size of the building units.

In some sub-embodiments, R of the compound of Formula (I) represents W¹ represented by the structure of the following formula: 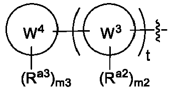
wherein each ring W³ is the same or different and each independently represents 4- to 20-membered nitrogen-containing heterocyclylene, e.g.,
   piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, 3-azaspiro[5.5]undecan-3-ylene, 8-azaspiro[4.5]decan-4-ylene, 2-oxa-8-azaspiro[4.5]decan-4-ylene, or 3-methyl-2-oxa-8-azaspiro[4.5]decan-4-ylene, or 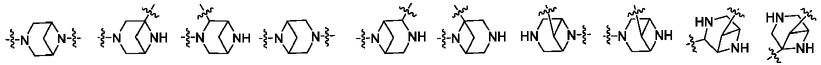 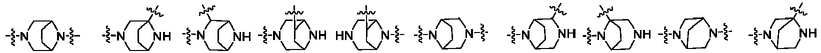 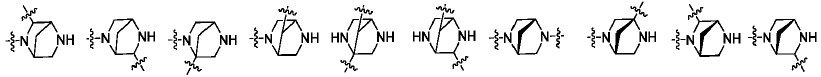 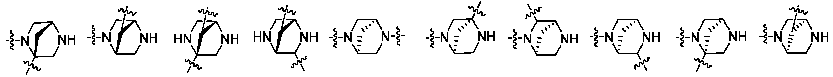 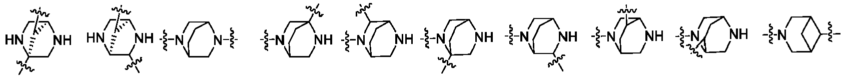 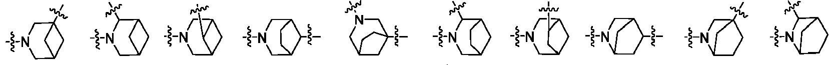 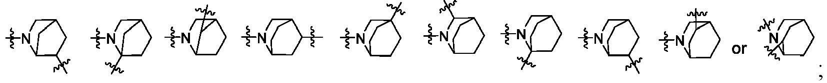
t represents an integer of 1 or 2, and (R^{a2})ₘ₂ indicates that each ring W³ is optionally substituted with m2 R^{a2} substituents, with each R^{a2} being independently deuterium, optionally deuterated C₁₋₆ alkyl, C₁₋₆ alkoxy, halogen, leaving group, amino, hydroxy, mercapto, cyano, carboxyl, CHO, C₂₋₆ alkynyl, C₂₋₆ alkenyl, optionally deuterated C₃₋₆ cycloalkyl, or R^{12a}-R^{11a}-, wherein R^{11a} is optionally substituted C₁₋₆ alkylene or optionally substituted C₂₋₆ alkenylene, and R^{12a} represents halogen, R^{13a}R^{14a}N-, hydroxy, carboxyl, CHO, ethynyl, vinyl, or leaving group, wherein R^{13a} and R^{14a} are the same or different and each independently represent hydrogen or C₁₋₃ alkyl; and
ring W⁴ represents 4- to 15-membered nitrogen-containing heterocyclyl, 6- to 10-membered aryl, or 5- to 10-membered heteroaryl, e.g.,
   piperidinyl, piperazinyl, morpholinyl, azetidinyl, pyrrolidinyl, imidazolidinyl, pyrazolidyl, oxazolidinyl, thiazolidinyl, thiomorpholinyl, azepanyl, diazacycloheptanyl, azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl, diazabicyclo[2.2.2]octanyl, 3-azaspiro[5.5]undecan-3-yl, 8-azaspiro[4.5]decan-4-yl, 2-oxa-8-azaspiro[4.5]decan-4-yl, 3-methyl-2-oxa-8-azaspiro[4.5]decan-4-yl, phenyl, naphthyl, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, or imidazo[2,1-b]thiazolyl; or 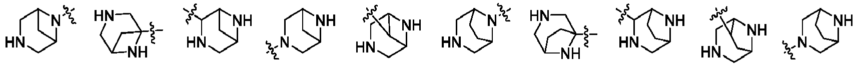 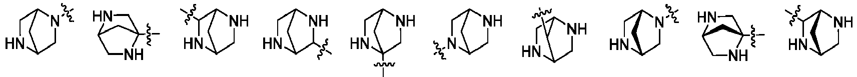 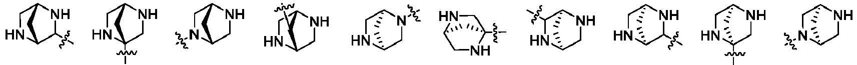 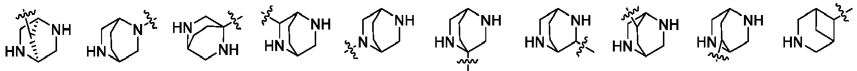 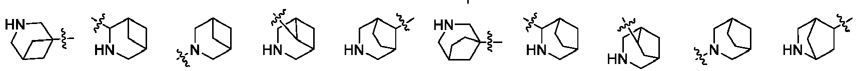 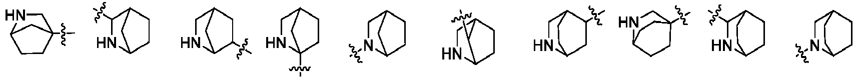 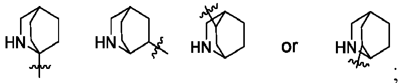
(R^{a3})ₘ₃ indicates that ring W⁴ is optionally substituted with m3 R^{a3} substituents, with each R^{a3} independently representing deuterium, optionally deuterated C₁₋₆ alkyl, C₁₋₆ alkoxy, halogen, leaving group, amino, hydroxy, mercapto, cyano, carboxyl, CHO, C₂₋₆ alkynyl, C₂₋₆ alkenyl, optionally deuterated C₃₋₆ cycloalkyl, or R^{16a}-R^{15a}-, wherein R^{15a} is optionally substituted C₁₋₆ alkylene or optionally substituted C₂₋₆ alkenylene, and R^{16a} represents halogen, R^{17a}R^{18a}N-, hydroxy, carboxyl, CHO, ethynyl, vinyl, or leaving group, wherein R^{17a} and R^{18a} are the same or different and each independently represent hydrogen or C₁₋₃ alkyl;
wherein m2 and m3 each independently represent an integer of 0-20 (e.g., an integer of 0, 1, 2, 3, 4, 5, 6, 7, 8, 9, 10, 11, 12, 13, 14, 15, or 20). The number of substituents is not theoretically limited in any way or automatically limited by the size of the building units.

In some sub-embodiments, each ring W³ is independently optionally perdeuterated or partially deuterated.

In some sub-embodiments, ring W⁴ is optionally perdeuterated or partially deuterated.

In some sub-embodiments, R of the compound of Formula (I) represents the following groups: chlorine, bromine, iodine, hydroxy, -N₃, sulfonate, e.g., methanesulfonyloxy (MsO-), trifluoromethanesulfonyloxy (TfO-) or p-toluenesulfonyloxy (TsO-), NH₂, or 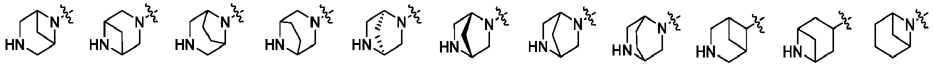 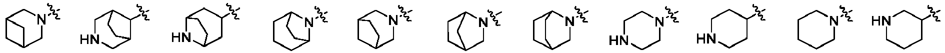 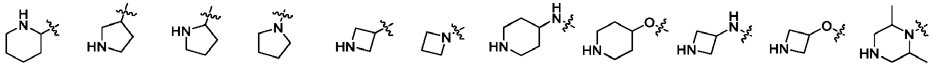 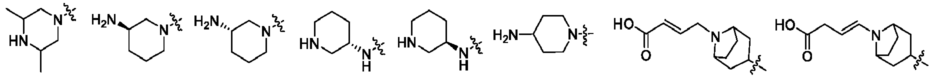 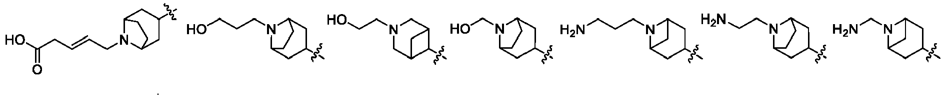 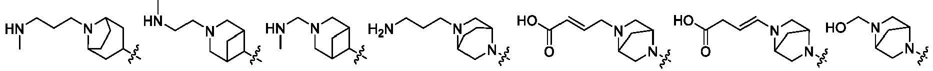 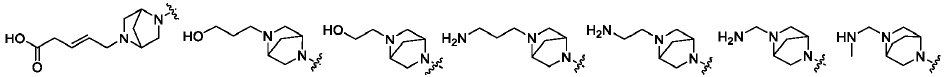 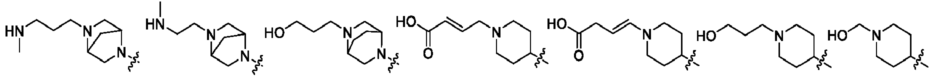 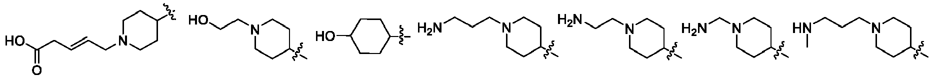 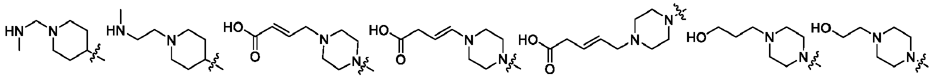 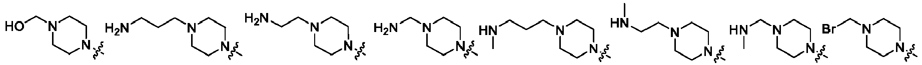 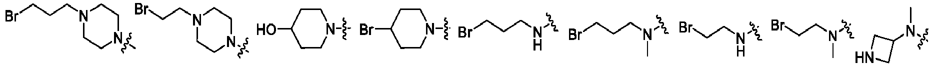 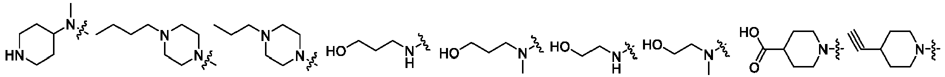 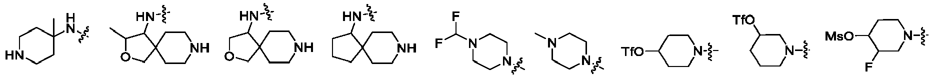 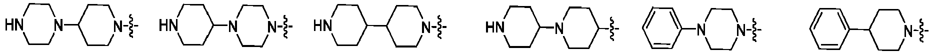 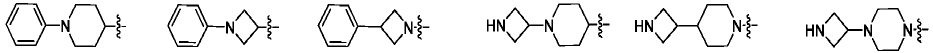 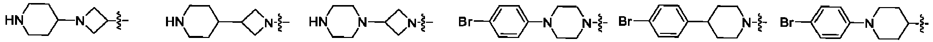 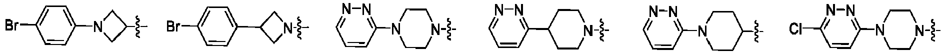 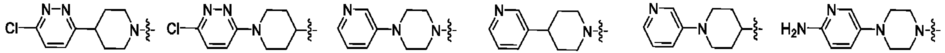 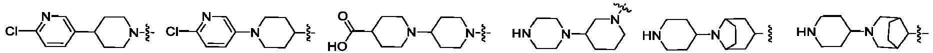 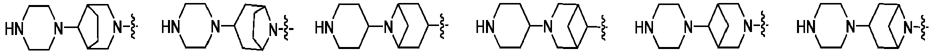 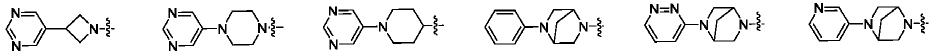 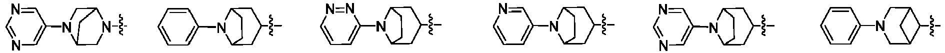 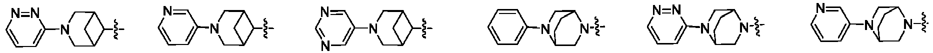 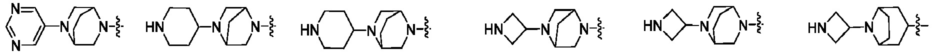 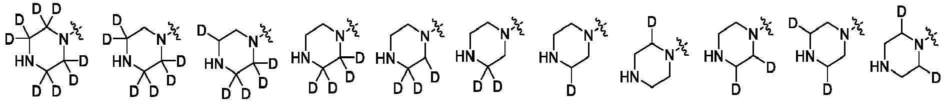 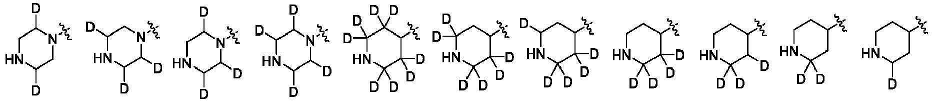 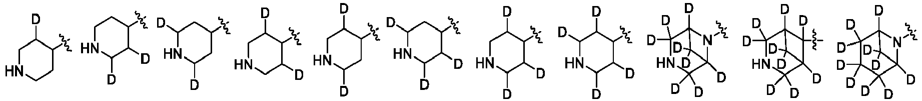 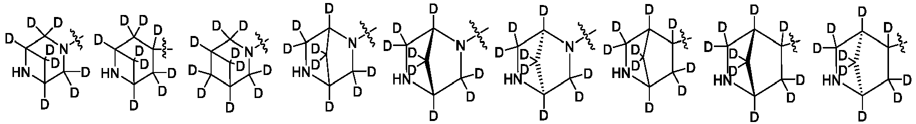 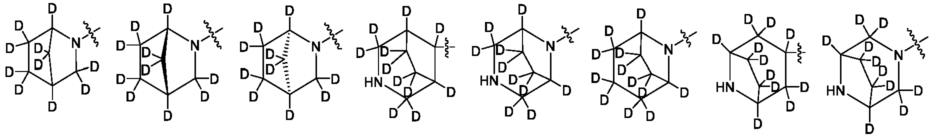 or

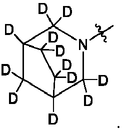

In some embodiments, R₅ and R₆ of the compound of Formula (I) each independently represent:
hydrogen, fluorine, chlorine, bromine, iodine, optionally substituted methyl, or optionally substituted linear or branched C₂₋₃₀ alkyl,
wherein one or more (e.g., 1-20, or 1-15, 1-10, 1-9, 1-8, 1-7, 1-6, 1-5, 1-4, 1-3, 1-2, or 1) groups R_{c} and/or one or more (e.g., 1-20, or 1-15, 1-10, 1-9, 1-8, 1-7, 1-6, 1-5, 1-4, 1-3, 1-2, or 1) groups R_{d} and/or any combination of one or more (e.g., 1-20, or 1-15, 1-10, 1-9, 1-8, 1-7, 1-6, 1-5, 1-4, 1-3, 1-2, or 1) groups R_{c} and R_{d} are optionally inserted into the backbone carbon chain of the linear or branched C₂₋₃₀ alkyl group, wherein carbon-carbon bond between one or more (e.g., 1-20, or 1-15, 1-10, 1-9, 1-8, 1-7, 1-6, 1-5, 1-4, 1-3, 1-2, or 1) pairs of adjacent carbon atoms in the backbone carbon chain is interrupted by the group R_{c}, R_{d}, or a combination of R_{c} and R_{d}; wherein each R_{c} is selected from the group consisting of O, N(Rₑ), C(O), C(O)O, S(O), S(O)₂, S(O)₂NH, NHS(O)₂, OC(O), C(O)N(Rₑ), N(Rₑ)C(O), or N(Rₑ)C(O)N(Rₑ), where each Rₑ independently represents H or C₁₋₆ alkyl (e.g., C₁₋₄ alkyl, such as methyl, ethyl, propyl, isopropyl, butyl, sec-butyl, tert-butyl, pentyl, or hexyl), and in case that two or more groups R_{c} are inserted into the backbone carbon chain of the linear or branched C₂₋₃₀ alkyl group, the two or more groups R_{c} are not directly connected to each other; and wherein each R_{d} is selected from the group consisting of cycloalkylene (e.g., C₃₋₂₀ cycloalkylene), arylene (e.g., C₃₋₂₀ arylene), heterocyclylene (e.g., 4- to 20-membered heterocyclylene), heteroarylene (e.g., 4- to 20-membered heteroarylene), alkynylene (e.g., C₂₋₆ alkynylene), alkenylene (e.g., C₂₋₆ alkenylene), or any combination thereof, wherein the cycloalkylene, arylene, heterocyclylene, and heteroarylene are each independently optionally substituted with a substituent selected from the group consisting of deuterium, optionally deuterated C₁₋₆alkyl (e.g., optionally deuterated C₁₋₄ alkyl, such as methyl, CD₃, ethyl, propyl, isopropyl, butyl, sec-butyl, tert-butyl, pentyl, or hexyl), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), hydroxy, amino, mercapto, halogen (e.g., fluorine, chlorine, bromine, or iodine), optionally deuterated C₁₋₆ alkoxy (e.g., optionally deuterated C₁₋₄ alkoxy, such as methoxy, CD₃-O-, ethoxy, propoxy, isopropoxy, n-butoxy, sec-butoxy, or tert-butoxy), optionally deuterated C₁₋₆ alkyl-NH- (e.g., optionally deuterated C₁₋₃ alkyl-NH-, such as CH₃NH-, CH₃CH₂NH- or CH₃CH₂CH₂NH-), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH-(e.g., optionally deuterated C₁₋₄ alkyl-C(O)NH-, such as CH₃-C(O)NH-, CH₃CH₂-C(O)NH-, and CH₃CH₂CH₂-C(O)NH-), cyano or any combination thereof; and
a hydrogen atom of methyl and hydrogen atom of one or more (e.g., 1-30, such as 1-25, 1-20, 1-15, 1-10, 1-5, 1-4, 1-3, 1-2 or 1) CH₂ of C₂₋₃₀ alkyl are optionally substituted with a substituent selected from the group consisting of deuterium, hydroxy, amino, mercapto, nitro, halogen (e.g., fluorine, chlorine, bromine, or iodine), cyano, optionally deuterated C₁₋₆ alkyl (e.g., optionally deuterated C₁₋₄ alkyl, such as methyl, CD₃, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, n-butoxy, sec-butoxy, or tert-butoxy), optionally deuterated C₁₋₆ alkyl-NH- (e.g., optionally deuterated C₁₋₃ alkyl-NH-, such as CH₃NH-, CH₃CH₂NH- or CH₃CH₂CH₂NH-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH- (e.g., optionally deuterated C₁₋₄ alkyl-C(O)NH-, such as CH₃-C(O)NH-, CH₃CH₂-C(O)NH-, and CH₃CH₂CH₂-C(O)NH-), or any combination thereof.

In some embodiments, R₅ of the compound of Formula (I) represents hydrogen, fluorine, chlorine, bromine, iodine, optionally substituted methyl, optionally substituted methoxy, optionally substituted ethoxy, optionally substituted propoxy, optionally substituted butoxy, or optionally substituted tert-butoxy.

In some embodiments, R and R₅ together with the carbon atom to which they are connected of the compound of Formula (I) form carbonyl group. In other words, Rₐ of the compound of Formula (I) represents the following group: 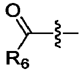 wherein R₆ is as defined above.

In some embodiments, R and R₅ are as defined in the compound of Formula (I) above and any one of its embodiments, and R₆ represents:
hydrogen, fluorine, chlorine, bromine, iodine, optionally substituted methyl, or optionally substituted linear or branched C₂₋₃₀ alkyl,
wherein one or more (e.g., 1-20, or 1-15, 1-10, 1-9, 1-8, 1-7, 1-6, 1-5, 1-4, 1-3, 1-2, or 1) groups R_{c} and/or one or more (e.g., 1-20, or 1-15, 1-10, 1-9, 1-8, 1-7, 1-6, 1-5, 1-4, 1-3, 1-2, or 1) groups R_{d} and/or any combination of one or more (e.g., 1-20, or 1-15, 1-10, 1-9, 1-8, 1-7, 1-6, 1-5, 1-4, 1-3, 1-2, or 1) groups R_{c} and R_{d} are optionally inserted into the backbone carbon chain of the linear or branched C₂₋₃₀ alkyl group, wherein carbon-carbon bond between one or more (e.g., 1-15, 1-10, 1-9, 1-8, 1-7, 1-6, 1-5, 1-4, 1-3, 1-2, or 1) pairs of adjacent carbon atoms in the backbone carbon chain is interrupted by the group R_{c}, R_{d}, or a combination of R_{c} and R_{d}; wherein each R_{c} is selected from the group consisting of O, N(Rₑ), C(O), C(O)O, S(O), S(O)₂, S(O)₂NH, NHS(O)₂, OC(O), C(O)N(Rₑ), N(Rₑ)C(O), or N(Rₑ)C(O)N(Rₑ), where each Rₑ independently represents H or C₁₋₆ alkyl (e.g., C₁₋₄ alkyl, such as methyl, ethyl, propyl, isopropyl, butyl, sec-butyl, tert-butyl, pentyl, or hexyl), and in case that two or more groups R_{c} are inserted into the backbone carbon chain of the linear or branched C₂₋₃₀ alkyl group, the two or more groups R_{c} are not directly connected to each other; and wherein each R_{d} is selected from the group consisting of cycloalkylene (e.g., C₃₋₂₀ cycloalkylene), arylene (e.g., C₃₋₂₀ arylene), heterocyclylene (e.g., 4- to 20-membered heterocyclylene), heteroarylene (e.g., 4- to 20-membered heteroarylene), alkynylene (e.g., C₂₋₆ alkynylene), alkenylene (e.g., C₂₋₆ alkenylene), or any combination thereof, wherein the cycloalkylene, arylene, heterocyclylene, and heteroarylene are each independently optionally substituted with a substituent selected from the group consisting of deuterium, optionally deuterated C₁₋₆alkyl (e.g., optionally deuterated C₁₋₄ alkyl, such as methyl, CD₃, ethyl, propyl, isopropyl, butyl, sec-butyl, tert-butyl, pentyl, or hexyl), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), hydroxy, amino, mercapto, halogen (e.g., fluorine, chlorine, bromine, or iodine), optionally deuterated C₁₋₆ alkoxy (e.g., optionally deuterated C₁₋₄ alkoxy, such as methoxy, CD₃-O-, ethoxy, propoxy, isopropoxy, n-butoxy, sec-butoxy, or tert-butoxy), optionally deuterated C₁₋₆ alkyl-NH- (e.g., optionally deuterated C₁₋₃ alkyl-NH-, such as CH₃NH-, CH₃CH₂NH- or CH₃CH₂CH₂NH-), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH-(e.g., optionally deuterated C₁₋₄ alkyl-C(O)NH-, such as CH₃-C(O)NH-, CH₃CH₂-C(O)NH-, and CH₃CH₂CH₂-C(O)NH-), cyano or any combination thereof; and
a hydrogen atom of methyl and hydrogen atom of one or more (e.g., 1-30, such as 1-25, 1-20, 1-15, 1-10, 1-5, 1-4, 1-3, 1-2 or 1) CH₂ of C₂₋₃₀ alkyl are optionally substituted with a substituent selected from the group consisting of deuterium, hydroxy, amino, mercapto, nitro, halogen (e.g., fluorine, chlorine, bromine, or iodine), cyano, optionally deuterated C₁₋₆ alkyl (e.g., optionally deuterated C₁₋₄ alkyl, such as methyl, CD₃, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, n-butoxy, sec-butoxy, or tert-butoxy), optionally deuterated C₁₋₆ alkyl-NH- (e.g., optionally deuterated C₁₋₃ alkyl-NH-, such as CH₃NH-, CH₃CH₂NH- or CH₃CH₂CH₂NH-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH- (e.g., optionally deuterated C₁₋₄ alkyl-C(O)NH-, such as CH₃-C(O)NH-, CH₃CH₂-C(O)NH-, and CH₃CH₂CH₂-C(O)NH-), or any combination thereof.

In some sub-embodiments, R₆ of the compound of Formula (I) represents hydrogen, fluorine, chlorine, bromine, or iodine.

In some sub-embodiments, R₆ of the compound of Formula (I) represents the structure of the following formula:
-C₁₋₃₀ alkyl;
-(C(R^{a4})(R^{a5}))ₙ₁-(R_{c}-(C(R^{a6})(R^{a7}))ₙ₂)ₘ₄-H;
-(C(R^{a4})(R^{a5}))ₙ₁-(R_{c}-(C(R^{a6})(R^{a7}))ₙ₂)ₘ₄-(R_{c}-(C(R^{a8})(R^{a9}))ₙ₃)ₘ₅-H;
-(C(R^{a4})(R^{a5}))ₙ₁-(R_{c}-(C(R^{a6})(R^{a7}))ₙ₂)ₘ₄-(R_{c}-(C(R^{a8})(R^{a9}))ₙ₃)ₘ₅-(R_{c}-(C(R^{a10})(R^{a11}))ₙ₄)ₘ₆-H;
-(C(R^{a4})(R^{a5}))ₙ₁-(R_{d}-(C(R^{a6})(R^{a7}))ₙ₂)ₘ₄-H;
-(C(R^{a4})(R^{a5}))ₙ₁-(R_{d}-(C(R^{a6})(R^{a7}))ₙ₂)ₘ₄-(R_{d}-(C(R^{a8})(R^{a9}))ₙ₃)ₘ₅-H;
-(C(R^{a4})(R^{a5}))ₙ₁-(R_{d}-(C(R^{a6})(R^{a7}))ₙ₂)ₘ₄-(R_{d}-(C(R^{a8})(R^{a9}))ₙ₃)ₘ₅-(R_{d}-(C(R^{a10})(R^{a11}))ₙ₄)ₘ₆-H;
-(C(R^{a4})(R^{a5}))ₙ₁-(R_{c}-R_{d}-(C(R^{a6})(R^{a7}))ₙ₂)ₘ₄-H;
-(C(R^{a4})(R^{a5}))ₙ₁-(R_{c}-(C(R^{a6})(R^{a7}))ₙ₂)ₘ₄-(R_{d}-(C(R^{a8})(R^{a9}))ₙ₃)ₘ₅-H;
-(C(R^{a4})(R^{a5}))ₙ₁-(R_{d}-R_{c}-(C(R^{a6})(R^{a7}))ₙ₂)ₘ₄-H; or
-(C(R^{a4})(R^{a5}))ₙ₁-(R_{d}-(C(R^{a6})(R^{a7}))ₙ₂)ₘ₄-(R_{c}-(C(R^{a8})(R^{a9}))ₙ₃)ₘ₅-H;
wherein each R_{c} is selected from the group consisting of O, N(Rₑ), C(O), C(O)O, OC(O), S(O), S(O)₂, S(O)₂NH, NHS(O)₂, C(O)N(Rₑ), N(Rₑ)C(O), or N(Rₑ)C(O)N(Rₑ), where each Rₑ independently represents H or C₁₋₆ alkyl; and wherein each R_{d} is selected from the group consisting of cycloalkylene (e.g., C₃₋₂₀ cycloalkylene), arylene (e.g., C₅₋₂₀ arylene), heterocyclylene (e.g., 4- to 20-membered heterocyclylene), heteroarylene (e.g., 5- to 20-membered heteroarylene), alkynylene (e.g., C₂₋₆ alkynylene), alkenylene (e.g., C₂₋₆ alkenylene), or any combination thereof, wherein the cycloalkylene, arylene, heterocyclylene, and heteroarylene are each independently optionally substituted with a substituent selected from the group consisting of deuterium, (e.g., optionally deuterated C₁₋₄ alkyl, such as methyl, CD₃, ethyl, propyl, isopropyl, butyl, sec-butyl, tert-butyl, pentyl, or hexyl), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), hydroxy, amino, mercapto, halogen (e.g., fluorine, chlorine, bromine, or iodine), optionally deuterated C₁₋₆ alkoxy (e.g., optionally deuterated C₁₋₄ alkoxy, such as methoxy, CD₃-O-, ethoxy, propoxy, isopropoxy, n-butoxy, sec-butoxy, or tert-butoxy), optionally deuterated C₁₋₆ alkyl-NH- (e.g., optionally deuterated C₁₋₃ alkyl-NH-, such as CH₃NH-, CH₃CH₂NH- or CH₃CH₂CH₂NH-), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH- (e.g., optionally deuterated C₁₋₄ alkyl-C(O)NH-, such as CH₃-C(O)NH-, CH₃CH₂-C(O)NH-, and CH₃CH₂CH₂-C(O)NH-), cyano, or any combination thereof;
a hydrogen atom of one or more (e.g., 1-30, such as 1-25, 1-20, 1-15, 1-10, 1-5, 1-4, 1-3, 1-2 or 1) CH₂ of C₁₋₃₀ alkyl are optionally substituted with a substituent selected from the group consisting of deuterium, hydroxy, amino, mercapto, nitro, halogen (e.g., fluorine, chlorine, bromine, or iodine), cyano, optionally deuterated C₁₋₆ alkyl (e.g., optionally deuterated C₁₋₄ alkyl, such as methyl, CD₃, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, n-butoxy, sec-butoxy, or tert-butoxy), optionally deuterated C₁₋₆ alkyl-NH- (e.g., optionally deuterated C₁₋₃ alkyl-NH-, such as CH₃NH-, CH₃CH₂NH- or CH₃CH₂CH₂NH-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH- (e.g., optionally deuterated C₁₋₄ alkyl-C(O)NH-, such as CH₃-C(O)NH-, CH₃CH₂-C(O)NH-, and CH₃CH₂CH₂-C(O)NH-), or any combination thereof;
R^{a4}, R^{a5}, R^{a6}, R^{a7}, R^{a8}, R^{a9}, R^{a10} and R^{a11} each independently represent H, deuterium, hydroxy, amino, mercapto, nitro, halogen, cyano, optionally deuterated C₁₋₆ alkyl (e.g., optionally deuterated C₁₋₄ alkyl, such as methyl, CD₃, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, n-butoxy, sec-butoxy, or tert-butoxy), optionally deuterated C₁₋₆ alkyl-NH- (e.g., optionally deuterated C₁₋₃ alkyl-NH-, such as CH₃NH-, CH₃CH₂NH- or CH₃CH₂CH₂NH-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), or optionally deuterated C₁₋₆ alkyl-C(O)NH- (e.g., optionally deuterated C₁₋₄ alkyl-C(O)NH-, such as CH₃-C(O)NH-, CH₃CH₂-C(O)NH-, and CH₃CH₂CH₂-C(O)NH-); and
n1, n2, n3, n4, m4, m5, and m6 each independently represent an integer of 1, 2, 3, 4, 5, 6, 7, 8, 9, 10, 11, 12, 13, 14, or 15.

In some sub-embodiments, R₆ of the compound of Formula (I) represents the structure of the following formula:
-(C(R^{a4})(R^{a5}))ₙ₁-(O-(C(R^{a6})(R^{a7}))ₙ₂)ₘ₄-H;
-(C(R^{a4})(R^{a5}))ₙ₁-(O-(C(R^{a6})(R^{a7}))ₙ₂)ₘ₄-(O-(C(R^{a8})(R^{a9}))ₙ₃)ₘ₅-H;
-(C(R^{a4})(Ra⁵))ₙ₁-(O-(C(R^{a6})(R^{a7}))ₙ₂)ₘ₄-(O-(C(R^{a8})(R^{a9}))ₙ₃)ₘ₅-(O-(C(R^{al0})(R^{al}l))ₙ₄)ₘ₆-H;
-(C(Ra⁴)(R^{a5}))ₙᵢ-(N(Re)-(C(Ra⁶)(Ra⁷))ₙ₂)ₘ₄-H;
-(C(R^{a4})(R^{a5}))ₙ₁-(N(Rₑ)-(C(R^{a6})(R^{a7}))ₙ₂)ₘ₄-(N(Rₑ)-(C(R^{a8})(R^{a9}))ₙ₃)^{m5}-H;
-(C(R^{a4})(R^{a5}))ₙ₁-(N(Rₑ)-(C(R^{a6})(R^{a7}))ₙ₂)ₘ₄-(N(Rₑ)-(C(R^{a8})(R^{a9}))ₙ₃)ₘ₅-(N(Rₑ)-(C(R^{a10})(R^{a11}))ₙ₄)ₘ₆-H;
-(C(R^{a4})(R^{a5}))ₙ₁-(C(O)N(Rₑ)-(C(R^{a6})(R^{a7}))ₙ₂)ₘ₄-H;
-(C(R^{a4})(R^{a5}))ₙ₁-(C(O)N(Rₑ)-(C(R^{a6})(R^{a7}))ₙ₂)ₘ₄-(C(O)N(Rₑ)-(C(R^{a8})(R^{a9}))ₙ₃)ₘ₅-H;
-(C(R^{a4})(R^{a5}))ₙ₁-(C(O)N(Rₑ)-(C(R^{a6})(R^{a7}))ₙ₂)ₘ₄-(C(O)N(Rₑ)-(C(R^{a8})(R^{a9}))ₙ₃)ₘ₅-(C(O)N(Rₑ)-(C(R^{a10})(R^{a11}))ₙ₄)ₘ₆-H;
-(C(R^{a4})(R^{a5}))ₙ₁-(C(O)N(Rₑ)-(C(R^{a6})(R^{a7}))ₙ₂)ₘ₄-(O-(C(R^{a8})(R^{a9}))ₙ₃)ₘ₅-H;
-(C(R^{a4})(R^{a5}))ₙ₁-C(O)N(Rₑ)-(C(R^{a6})(R^{a7}))ₙ₂-(O-(C(R^{a8})(R^{a9}))ₙ₃)ₘ₄-H;
-(C(R^{a4})(R^{a5}))ₙ₁-(N(Rₑ)C(O)-(C(R^{a6})(R^{a7}))ₙ₂)ₘ₄-H;
-(C(R^{a4})(R^{a5}))ₙ₁-(N(Rₑ)C(O)-(C(R^{a6})(R^{a7}))ₙ₂)ₘ₄-(O-(C(R^{a8})(R^{a9}))ₙ₃)ₘ₅-H;
-(C(R^{a4})(R^{a5}))ₙ₁-(N(Rₑ)C(O)-(C(R^{a6})(R^{a7}))ₙ₂)ₘ₄-(O-(C(R^{a8})(R^{a9}))ₙ₃)ₘ₅-(O-(C(R^{a10})(R^{a11}))ₙ₄)ₘ₆-H;
-(C(R^{a4})(R^{a5}))ₙ₁-N(Rₑ)C(O)-(C(R^{a6})(R^{a7}))ₙ₂-(O-(C(R^{a8})(R^{a9}))ₙ₃)ₘ₄-H;
-(C(R^{a4})(R^{a5}))ₙ₁-N(Rₑ)C(O)N(Rₑ)-(C(R^{a6})(R^{a7}))ₙ₂-H;
-(C(R^{a4})(R^{a5}))ₙ₁-C(O)-(C(R^{a6})(R^{a7}))ₙ₂-H;
-(C(R^{a}4)arylene-(C(R^{a6})(R^{a}7)-H;
-(C(R^{a}4)arylene-(C(R^{a6})(R^{a7})-arylene-(C(R^{a8})(R^{a9})H;
-(C(R^{a4})(R^{a5}))ₙ₁-(heterocyclylene-(C(R^{a6})(R^{a7}))ₙ₂)ₘ₄-H;
-(C(R^{a4})(R^{a5}))ₙ₁-(heterocyclylene-(C(R^{a6})(R^{a7}))ₙ₂)ₘ₄-(heterocyclylene-(C(R^{a8})(R^{a9}))ₙ₃)ₘ₅-H;
-(C(R^{a4})(R^{a5}))ₙ₁-(heteroarylene-(C(R^{a6})(R^{a7}))ₙ₂)ₘ₄-H;
-(C(R^{a4})(R^{as}))ₙ₁-(heteroarylene-(C(R^{a6})(R^{a7}))ₙ₂)ₘ₄-(heteroarylene-(C(R^{a8})(R^{a9}))ₙ₃)ₘ₅-H;
-(C(R^{a4})(R^{a5}))ₙ₁-(cycloalkylene-(C(R^{a6})(R^{a7}))ₙ₂)ₘ₄-H;or
-(C(R^{a4})(R^{a5}))ₙ₁-(cycloalkylene-(C(R^{a6})(R^{a7}))ₙ₂)ₘ₄-(cycloalkylene-(C(R^{a8})(R^{a9}))ₙ₃)ₘ₅-H;

wherein each Rₑ independently represents H or C₁₋₆ alkyl (e.g., C₁₋₄ alkyl, such as methyl, ethyl, propyl, isopropyl, butyl, sec-butyl, tert-butyl, pentyl, or hexyl);
the cycloalkylene (e.g., C₃₋₂₀ cycloalkylene, C₃₋₁₅ cycloalkylene, C₃₋₁₀ cycloalkylene, C₃₋₈ cycloalkylene, or C₃₋₆ cycloalkylene), arylene (e.g., C₅₋₂₀ arylene, C₅₋₁₅ arylene, C₅₋₁₀ arylene, or C₅₋₆ arylene), heterocyclylene (e.g., 4- to 20-membered heterocyclylene, 4- to 15-membered heterocyclylene, 4- to 10-membered heterocyclylene, 4- to 9-membered heterocyclylene, 4- to 8-membered heterocyclylene, 5- to 7-membered heterocyclylene, or 6-membered heterocyclylene) and heteroarylene (e.g., 5- to 20-membered heteroarylene, 5- to 15-membered heteroarylene, 5- to 10-membered heteroarylene, 5- to 9-membered heteroarylene, 5- to 8-membered heteroarylene, 5- to 7-membered heteroarylene, or 6-membered heteroarylene,) are each independently optionally substituted with a substituent selected from the group consisting of deuterium, optionally deuterated C₁₋₆ alkyl (e.g., optionally deuterated C₁₋₄ alkyl, such as methyl, CD₃, ethyl, propyl, isopropyl, butyl, sec-butyl, tert-butyl, pentyl, or hexyl), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), hydroxy, amino, mercapto, halogen (e.g., fluorine, chlorine, bromine, or iodine), optionally deuterated C₁₋₆ alkoxy (e.g., optionally deuterated C₁₋₄ alkoxy, such as methoxy, CD₃-O-, ethoxy, propoxy, isopropoxy, n-butoxy, sec-butoxy, or tert-butoxy), optionally deuterated C₁₋₆ alkyl-NH- (e.g., optionally deuterated C₁₋₃ alkyl-NH-, such as CH₃NH-, CH₃CH₂NH- or CH₃CH₂CH₂NH-), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH- (e.g., optionally deuterated C₁₋₄ alkyl-C(O)NH-, such as CH₃-C(O)NH-, CH₃CH₂-C(O)NH-, and CH₃CH₂CH₂-C(O)NH-), cyano, or any combination thereof;
R^{a4}, R^{a5}, R^{a6}, R^{a7}, R^{a8}, R^{a9}, R^{a10} and R^{a11} each independently represent H, deuterium, hydroxy, amino, mercapto, nitro, halogen (e.g., fluorine, chlorine, bromine, or iodine), cyano, optionally deuterated C₁₋₆ alkyl (e.g., optionally deuterated C₁₋₄ alkyl, such as methyl, CD₃, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, n-butoxy, sec-butoxy, or tert-butoxy), optionally deuterated C₁₋₆ alkyl-NH- (e.g., optionally deuterated C₁₋₃ alkyl-NH-, such as CH₃NH-, CH₃CH₂NH- or CH₃CH₂CH₂NH-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), or optionally deuterated C₁₋₆ alkyl-C(O)NH- (e.g., optionally deuterated C₁₋₄ alkyl-C(O)NH-, such as CH₃-C(O)NH-, CH₃CH₂-C(O)NH-, and CH₃CH₂CH₂-C(O)NH-); and
n1, n2, n3, n4, m4, m5, and m6 each independently represent an integer of 1, 2, 3, 4, 5, 6, 7, 8, 9, 10, 11, 12, 13, 14, or 15.

In some sub-embodiments, R₆ of the compound of Formula (I) represents the following groups:
H, fluorine, chlorine, bromine, iodine, -CH₃, -CH₂-CH₃, -(CH₂)₂-CH₃, -(CH₂)₃-CH₃, -(CH₂)₄-CH₃, -(CH₂)₅-CH₃, -(CH₂)₆-CH₃, -(CH₂)₇-CH₃, -(CH₂)₈-CH₃, -(CH₂)₉-CH₃, -(CH₂)_{1O}-CH₃, -(CH₂)₁₁-CH₃, - (CH₂)₁₂-CH₃, -(CH₂)₁₃-CH₃, -(CH₂)₁₄-CH₃, -(CH₂)₁₅-CH₃, -(CH₂)₁₆-CH₃, -(CH₂)₁₇-CH₃, -(CH₂)₁₈-CH₃, -(CH₂)₁₉-CH₃, -(CH₂)₂₀-CH₃, -(CH₂)₂₁-CH₃, -(CH₂)₂₂-CH₃, -(CH₂)₂₅-CH₃, -(CH₂)₂₉-CH₃;-CH₂-O-CH₃, -CH₂-O-CH₂-CH₃, -CH₂-O-(CH₂)₂-CH₃, -(CH₂)₁-O-(CH₂)₃-CH₃, -(CH₂)₁-O-(CH₂)₄-CH₃, -(CH₂)₁-O-(CH₂)₅-CH₃, -(CH₂)₁-O-(CH₂)₆-CH₃, -(CH₂)₁-O-(CH₂)₇-CH₃, -(CH₂)₁-O-(CH₂)₈-CH₃, -(CH₂)₁-O-(CH₂)₉-CH₃, -(CH₂)₁-O-(CH₂)₁₀-CH₃, -(CH₂)₂-O-CH₃, -(CH₂)₂-O-CH₂-CH₃, -(CH₂)₂-O-(CH₂)₂-CH₃, - (CH₂)₂-O-(CH₂)₃-CH₃, -(CH₂)₂-O-(CH₂)₄-CH₃, -(CH₂)₂-O-(CH₂)₅-CH₃, -(CH₂)₂-O-(CH₂)₆-CH₃, - (CH₂)₂-O-(CH₂)₇-CH₃, -(CH₂)₂-O-(CH₂)₈-CH₃, -(CH₂)₂-O-(CH₂)₉-CH₃, -(CH₂)₂-O-(CH₂)_{I0}-CH₃, - (CH₂)₂-O-(CH₂)₁₁-CH₃, -(CH₂)₂-O-(CH₂)₁₂-CH₃, -(CH₂)₃-O-CH₃, -(CH₂)₃-O-CH₂-CH₃, -(CH₂)₃-O-(CH₂)₂-CH₃, -(CH₂)₃-O-(CH₂)₃-CH₃, -(CH₂)₃-O-(CH₂)₄-CH₃, -(CH₂)₃-O-(CH₂)₅-CH₃, -(CH₂)₃-O-(CH₂)₆-CH₃, -(CH₂)₃-O-(CH₂)₇-CH₃, -(CH₂)₄-O-CH₃, -(CH₂)₄-O-CH₂-CH₃, -(CH₂)₄-O-(CH₂)₂-CH₃, - (CH₂)₄-O-(CH₂)₃-CH₃, -(CH₂)₄-O-(CH₂)₄-CH₃, -(CH₂)₄-O-(CH₂)₅-CH₃, -(CH₂)₄-O-(CH₂)₆-CH₃, - (CH₂)₅-O-CH₃, -(CH₂)₅-O-CH₂-CH₃, -(CH₂)₅-O(CH₂)₂-CH₃, -(CH₂)₅-O-(CH₂)₃-CH₃, -(CH₂)₅-O-(CH₂)₄-CH₃, -(CH₂)₅-O-(CH₂)₅-CH₃, -(CH₂)₆-O-CH₃, -(CH₂)₆-O-CH₂-CH₃, -(CH₂)₆-O-(CH₂)₂-CH₃, - (CH₂)₆-O-(CH₂)₃-CH₃, -(CH₂)₆-O-(CH₂)₄-CH₃, -(CH₂)₇-O-CH₃, -(CH₂)₇-O-CH₂-CH₃, -(CH₂)₇-O-(CH₂)₂-CH₃, -(CH₂)₇-O-(CH₂)₃-CH₃, -(CH₂)₈-O-CH₃, -(CH₂)₈-O-CH₂-CH₃, -(CH₂)₈-O-(CH₂)₂-CH₃, - CH(CH₃)-O-CH₃, -CH(CH₃)-O-CH₂-CH₃, -CH(CH₃)-O-(CH₂)₂-CH₃, -CH(CH₃)-O-(CH₂)₃-CH₃, - CH(CH₃)-O-(CH₂)₄-CH₃, -CH(CH₃)-O-(CH₂)₅-CH₃, -CH(CH₃)-O-(CH₂)₆-CH₃, -CH(CH₃)-O-(CH₂)₇-CH₃, -CH(CH₃)-O-(CH₂)₈-CH₃, -CH(CH₃)-O-(CH₂)₉-CH₃, -CH(CH₃)-O-(CH₂)₁₀-CH₃, -CH₂-(O(CH₂)₂)₁-OCH₂CH₃, -CH₂-(O(CH₂)₂)₂-OCH₂CH₃, -CH₂-(O(CH₂)₂)₃-OCH₂CH₃, -CH₂-(O(CH₂)₂)₄-OCH₂CH₃, -CH₂-(O(CH₂)₂)₅-OCH₂CH₃, -CH₂-(O(CH₂)₂)₆-OCH₂CH₃, -CH₂-(O(CH₂)₂)₇-OCH₂CH₃, - CH₂-(O(CH₂)₂)₈-OCH₂CH₃, -CH₂-(O(CH₂)₂)₉-OCH₂CH₃, -CH₂-(O(CH₂)₂)₁₀-OCH₂CH₃, -CH₂-(O(CH₂)₂)₁-OCH₃, -CH₂-(O(CH₂)₂)₂-OCH₃, -CH₂-(O(CH₂)₂)₃-OCH₃, -CH₂-(O(CH₂)₂)₄-OCH₃, -CH₂-(O(CH₂)₂)₅-OCH₃, -CH₂-(O(CH₂)₂)₆-OCH₃, -CH₂-(O(CH₂)₂)₇-OCH₃, -CH₂-(O(CH₂)₂)₈-OCH₃, -CH₂-(O(CH₂)₂)₉-OCH₃, -CH₂-(O(CH₂)₂)₁₀-OCH₃, -(CH₂)₂-(O(CH₂)₂)₁-OCH₂CH₃, -(CH₂)₂-(O(CH₂)₂)₂-OCH₂CH₃, -(CH₂)₂-(O(CH₂)₂)₃-OCH₂CH₃, -(CH₂)₂-(O(CH₂)₂)₄-OCH₂CH₃, -(CH₂)₂-(O(CH₂)₂)₅-OCH₂CH₃, -(CH₂)₂-(O(CH₂)₂)₆-OCH₂CH₃, -(CH₂)₂-(O(CH₂)₂)₇-OCH₂CH₃, -(CH₂)₂-(O(CH₂)₂)₈-OCH₂CH₃, -(CH₂)₂-(O(CH₂)₂)₉-OCH₂CH₃, -(CH₂)₂-(O(CH₂)₂)₁₀-OCH₂CH₃, -(CH₂)₃-(O(CH₂)₂)₁-OCH₂CH₃, -(CH₂)₃-(O(CH₂)₂)₂-OCH₂CH₃, -(CH₂)₃-(O(CH₂)₂)₃-OCH₂CH₃, -(CH₂)₃-(O(CH₂)₂)₄-OCH₂CH₃, -(CH₂)₃-(O(CH₂)₂)₅-OCH₂CH₃, -(CH₂)₃-(O(CH₂)₂)₆-OCH₂CH₃, -(CH₂)₃-(O(CH₂)₂)₇-OCH₂CH₃, -(CH₂)₃-(O(CH₂)₂)₈-OCH₂CH₃, -(CH₂)₃-(O(CH₂)₂)₉-OCH₂CH₃, -(CH₂)₃-(O(CH₂)₂)₁₀-OCH₂CH₃, -(CH₂)₄-(O(CH₂)₂)₁-OCH₂CH₃, -(CH₂)₄-(O(CH₂)₂)₂-OCH₂CH₃, -(CH₂)₄-(O(CH₂)₂)₃-OCH₂CH₃, -(CH₂)₄-(O(CH₂)₂)₄-OCH₂CH₃, -(CH₂)₄-(O(CH₂)₂)₅-OCH₂CH₃, -(CH₂)₄-(O(CH₂)₂)₆-OCH₂CH₃, -(CH₂)₄-(O(CH₂)₂)₇-OCH₂CH₃, -(CH₂)₄-(O(CH₂)₂)₈-OCH₂CH₃, -(CH₂)₄-(O(CH₂)₂)₉-OCH₂CH₃, -(CH₂)₄-(0(CH₂)₂)_{lO}-OCH₂CH₃, -CH₂-(O(CH₂)₃)₁-OCH₂CH₂CH₃, -CH₂-(O(CH₂)₃)₂-OCH₂CH₂CH₃, -CH₂-(O(CH₂)₃)₃-OCH₂CH₂CH₃, -CH₂-(O(CH₂)₃)₄-OCH₂CH₂CH₃, -CH₂-(O(CH₂)₃)₅-OCH₂CH₂CH₃, -CH₂-(O(CH₂)₃)₆-OCH₂CH₂CH₃, -CH₂-(O(CH₂)₃)₇-OCH₂CH₂CH₃, -CH₂-(O(CH₂)₃)₈-OCH₂CH₂CH₃, -CH₂-(O(CH₂)₃)₉-OCH₂CH₂CH₃, -CH₂-(O(CH₂)₃)ₗo-OCH₂CH₂CH₃, -(CH₂)₂-(O(CH₂)₃)ₜ-OCH₂CH₂CH₃, -(CH₂)₂-(O(CH₂)₃)₂-OCH₂CH₂CH₃, -(CH₂)₂-(O(CH₂)₃)₃-OCH₂CH₂CH₃, - (CH₂)₂-(O(CH₂)₃)₄-OCH₂CH₂CH₃, -(CH₂)₂-(O(CH₂)₃)₅-OCH₂CH₂CH₃, -(CH₂)₂-(O(CH₂)₃)₆-OCH₂CH₂CH₃, -(CH₂)₂-(O(CH₂)₃)₇-OCH₂CH₂CH₃, -(CH₂)₂-(O(CH₂)₃)_{S}-OCH₂CH₂CH₃, -(CH₂)₃-(O(CH₂)₃)ₜ-OCH₂CH₂CH₃, -(CH₂)₃-(O(CH₂)₃)₂-OCH₂CH₂CH₃, -(CH₂)₃-(O(CH₂)₃)₃-OCH₂CH₂CH₃, - (CH₂)₃-(O(CH₂)₃)₄-OCH₂CH₂CH₃, -(CH₂)₃-(O(CH₂)₃)₅-OCH₂CH₂CH₃, -(CH₂)₃-(O(CH₂)₃)₆-OCH₂CH₂CH₃, -(CH₂)₃-(O(CH₂)₃)₇-OCH₂CH₂CH₃, -(CH₂)₃-(O(CH₂)₃)₈-OCH₂CH₂CH₃, -CH₂-O-(CH₂)₂-O-(CH₂)₂-CH₃, -CH₂-(O(CH₂)₂)₂-(O(CH₂)₃)₁-OCH₂CH₂CH₃, -CH₂-(O(CH₂)₂)₃-(O(CH₂)₃)₂-OCH₂CH₂CH₃, -CH₂-(O(CH₂)₂)₄-(O(CH₂)₃)₃-OCH₂CH₂CH₃, -CH₂-(O(CH₂)₂)₅-(O(CH₂)₃)₄-OCH₂CH₂CH₃, -(CH₂)₂-O-(CH₂)₂-O-(CH₂)₂CH₃, -(CH₂)₂-(O(CH₂)₂)₂-(O(CH₂)₃)₁-OCH₂CH₂CH₃, - (CH₂)₂-(O(CH₂)₂)₃-(O(CH₂)₃)₂-OCH₂CH₂CH₃, -(CH₂)₂-(O(CH₂)₂)₄-(O(CH₂)₃)₃-OCH₂CH₂CH₃, - (CH₂)₂-(O(CH₂)₂)₅-(O(CH₂)₃)₄-OCH₂CH₂CH₃, -(CH₂)₃-O-(CH₂)₂-O-(CH₂)₂CH₃, -(CH₂)₃-(O(CH₂)₂)₂-(O(CH₂)₃)ₜ-OCH₂CH₂CH₃, -(CH₂)₃-(O(CH₂)₂)₃-(O(CH₂)₃)₂-OCH₂CH₂CH₃, -(CH₂)₃-(O(CH₂)₂)₄-(O(CH₂)₃)₃-OCH₂CH₂CH₃, -(CH₂)₃-(O(CH₂)₂)₅-(O(CH₂)₃)₄-OCH₂CH₂CH₃, -CH₂-O-(CH₂)₃-O-CH₂CH₃, -CH₂-(O(CH₂)₃)₂-(O(CH₂)₂)_{I}-O-CH₂CH₃, -CH₂-(O(CH₂)₃)₃-(O(CH₂)₂)₂-O-CH₂CH₃, -CH₂-(O(CH₂)₃)₄-(O(CH₂)₂)₃-O-CH₂CH₃, -CH₂-(O(CH₂)₃)₅-(O(CH₂)₂)₄-O-CH₂CH₃, -(CH₂)₂-O-(CH₂)₃-O-CH₂CH₃, -(CH₂)₂-(O(CH₂)₃)₂-(O(CH₂)₂)₁-O-CH₂CH₃, -(CH₂)₂-(O(CH₂)₃)₃-(O(CH₂)₂)₂-O-CH₂CH₃, - (CH₂)₂-(O(CH₂)₃)₄-(O(CH₂)₂)₃-O-CH₂CH₃, -(CH₂)₂-(O(CH₂)₃)₅-(O(CH₂)₂)₄-O-CH₂CH₃, -(CH₂)₃-O-(CH₂)₃-O-CH₂CH₃, -(CH₂)₃-(O(CH₂)₃)₂-(O(CH₂)₂)₁-O-CH₂CH₃, -(CH₂)₃-(O(CH₂)₃)₃-(O(CH₂)₂)₂-O-CH₂CH₃, -(CH₂)₃-(O(CH₂)₃)₄-(O(CH₂)₂)₃-O-CH₂CH₃, -(CH₂)₃-(O(CH₂)₃)₅-(O(CH₂)₂)₄-O-CH₂CH₃, - CH₂-O-(CH₂)₂-O-CH₃, -(CH₂)₂-O-(CH₂)₂-O-CH₃, -(CH₂)₂-(O(CH₂)₂)₂-O-(CH₂)₂CH₃, -(CH₂)₂-(O(CH₂)₂)₃-O-(CH₂)₂CH₃, -(CH₂)₂-(O(CH₂)₂)₄-O-(CH₂)₂CH₃, -(CH₂)₅-(O(CH₂)₂)₂-O-(CH₂)₄CH₃, - (CH₂)₅-(O(CH₂)₂)₂-O-(CH₂)₅CH₃, -(CH₂)₁-N(Rₑ)-CH₃, -(CH₂)₁-N(Rₑ)-(CH₂)₁-CH₃, -(CH₂)₁-N(Rₑ)-(CH₂)₂-CH₃, -(CH₂)₁-N(Rₑ)-(CH₂)₃-CH₃, -(CH₂)₁-N(Rₑ)-(CH₂)₄-CH₃, -(CH₂)₁-N(Re)-(CH₂)₅-CH₃, - (CH₂)₁-N(Rₑ)-(CH₂)₆-CH₃, -(CH₂)₁-N(Rₑ)-(CH2)₇-CH₃, -(CH₂)₁-N(Rₑ)-(CH₂)₈-CH₃, -(CH₂)₁-N(Rₑ)-(CH₂)₉-CH₃, -(CH₂)₂-N(Rₑ)-CH₃, -(CH₂)₂-N(Rₑ)-(CH₂)₁-CH₃, -(CH₂)₂-N(Rₑ)-(CH₂)₂-CH₃, -(CH₂)₂-N(Rₑ)-(CH₂)₃-CH₃, -(CH₂)₂-N(Rₑ)-(CH₂)₄-CH₃, -(CH₂)₂-N(Rₑ)-(CH₂)₅-CH₃, -(CH₂)₂-N(Rₑ)-(CH₂)₆-CH₃, -(CH₂)₂-N(Rₑ)-(CH₂)₇-CH₃, -(CH₂)₂-N(Rₑ)-(CH₂)₈-CH₃, -(CH₂)₂-N(Rₑ)-(CH₂)₉-CH₃, -(CH₂)₂-N(Rₑ)-(CH₂)₁₀-CH₃, -(CH₂)₂-N(Rₑ)-(CH₂)₁₁-CH₃, -(CH₂)₃-N(Rₑ)-CH₃, -(CH₂)₃-N(Rₑ)-(CH₂)₁-CH₃, - (CH₂)₃-N(Rₑ)-(CH₂)₂-CH₃, -(CH₂)₄-N(Rₑ)-CH₃, -(CH₂)₄-N(Rₑ)-(CH₂)₁-CH₃, -(CH₂)₄-N(Rₑ)-(CH₂)₂-CH₃, -(CH₂)₄-N(Rₑ)-(CH₂)₃-CH₃, -(CH₂)₅-N(Rₑ)-CH₃, -(CH₂)₅-N(Rₑ)-(CH₂)₁-CH₃, -(CH₂)₅-N(Rₑ)-(CH₂)₂-CH₃, -(CH₂)₅-N(Rₑ)-(CH₂)₃-CH₃, -(CH₂)₅-N(Rₑ)-(CH₂)₄-CH₃, -(CH₂)₆-N(Rₑ)-CH₃, -(CH₂)₆-N(Rₑ)-(CH₂)₁-CH₃, -(CH₂)₆-N(Rₑ)-(CH₂)₂-CH₃, -(CH₂)₇-N(Rₑ)-CH₃, -(CH₂)₇-N(Rₑ)-(CH₂)₁-CH₃, - (CH₂)₇-N(Rₑ)-(CH₂)₂-CH₃, -(CH₂)₈-N(Rₑ)-CH₃, -(CH₂)₈-N(Rₑ)-(CH₂)₁-CH₃, -(CH₂)₈-N(Rₑ)-(CH₂)₂-CH₃, -CH(CH₃)-N(Rₑ)-CH₃, -CH(CH₃)-N(Rₑ)-(CH₂)₁-CH₃, -CH(CH₃)-N(Rₑ)-(CH₂)₂-CH₃, -CH(CH₃)-N(Rₑ)-(CH₂)₃-CH₃, -CH(CH₃)-N(Rₑ)-(CH₂)₄-CH₃, -CH(CH₃)-N(Rₑ)-(CH₂)₅-CH₃, -CH(CH₃)-N(Rₑ)-(CH₂)₆-CH₃, -CH(CH₃)-N(Rₑ)-(CH₂)₇-CH₃, -CH(CH₃)-N(Rₑ)-(CH₂)₈-CH₃, -CH(CH₃)-N(Rₑ)-(CH₂)₉-CH₃, -CH₂C(O)NHCH₃, -(CH₂)₂C(O)NHCH₂CH₃, -(CH₂)₂C(O)NH(CH₂)₂-CH₃, - (CH₂)₂C(O)NH(CH₂)₃-CH₃, -(CH₂)₂C(O)NH(CH₂)₄-CH₃, -(CH₂)₃C(O)NH(CH₂)₂-CH₃, - (CH₂)₃C(O)NH(CH₂)₃-CH₃, -(CH₂)₄C(O)NH(CH₂)₃-CH₃, -(CH₂)₅C(O)NH(CH₂)₄-CH₃, - (CH₂)₆C(O)NH(CH₂)₆-CH₃, -(CH₂)₆C(O)NH(CH₂)₅-CH₃, -(CH₂)₇C(O)NH(CH₂)₆-CH₃, - (CH₂)₈C(O)NH(CH₂)₇-CH₃, U-(CH₂)₉C(O)NH(CH₂)₈-CH₃, -(CH₂)₁₀C(O)NH(CH₂)₉-CH₃, - (CH₂)₂C(O)NH(CH₂)₂-O-CH₂-CH₃, -CH₂NHC(O)CH₃, -(CH₂)₂NHC(O)CH₂CH₃, - (CH₂)₂NHC(O)(CH₂)₂-CH₃, -(CH₂)₂NHC(O)(CH₂)₃-CH₃, -(CH₂)₂NHC(O)(CH₂)₄-CH₃, - (CH₂)₃NHC(O)(CH₂)₂-CH₃, -(CH₂)₃NHC(O)(CH₂)₃-CH₃, -(CH₂)₄NHC(O)(CH₂)₃-CH₃, - (CH₂)₅NHC(O)(CH₂)₄-CH₃, -(CH₂)₆NHC(O)(CH₂)₆-CH₃, -(CH₂)₆NHC(O)(CH₂)₅-CH₃, - (CH₂)₇NHC(O)(CH₂)₆-CH₃, -(CH₂)₈NHC(O)(CH₂)₇-CH₃, -(CH₂)₉NHC(O)(CH₂)₈-CH₃, - (CH₂)₁₀NHC(O)(CH₂)₉-CH₃, -(CH₂)₄NHC(O)(CH₂)₇-CH₃, -(CH₂)₂NHC(O)(CH₂)₂-O-CH₂-CH₃, - (CH₂)₄NHC(O)CH₃, -CH₂-pipendinylene-CH₃, -CH₂-piperidinylene-CH₂-CH₃, -CH₂-piperidinylene-(CH₂)₂-CH₃, -CH₂-piperidinylene-(CH₂)₃-CH₃, -CH₂-piperidinylene-(CH₂)₄-CH₃, -CH₂-piperidinylene-(CH₂)₅-CH₃, -CH₂-piperidinylene-(CH₂)₆-CH₃, -CH₂-piperidinylene-(CH₂)₇-CH₃, - (CH₂)₂-piperidinylene-CH₃, -(CH₂)₂-piperidinylene-CH₂-CH₃, -(CH₂)₂-piperidinylene-(CH₂)₂-CH₃, - (CH₂)₂-piperidinylene-(CH₂)₃-CH₃, -(CH₂)₂-piperidinylene-(CH₂)₄-CH₃, -(CH₂)₂-piperidinylene-(CH₂)₅-CH₃, -(CH₂)₂-piperidinylene-(CH₂)₆-CH₃, -(CH₂)₂-piperidinylene-(CH₂)₇-CH₃, -(CH₂)₃-piperidinylene-CH₃, -(CH₂)₃-piperidinylene-CH₂-CH₃, -(CH₂)₃-piperidinylene-(CH₂)₂-CH₃, -(CH₂)₃-piperidinylene-(CH₂)₃-CH₃, -(CH₂)₃-piperidinylene-(CH₂)₄-CH₃, -(CH₂)₃-piperidinylene-(CH₂)₅-CH₃, -(CH₂)₃-piperidinylene-(CH₂)₆-CH₃, -(CH₂)₃-piperidinylene-(CH₂)₇-CH₃, -(CH₂)₄-piperidinylene-CH₃, -(CH₂)₄-piperidinylene-CH₂-CH₃, -(CH₂)₄-piperidinylene-(CH₂)₂-CH₃, -(CH₂)₄-piperidinylene-(CH₂)₃-CH₃, -(CH₂)₄-piperidinylene-(CH₂)₄-CH₃, -(CH₂)₄-piperidinylene-(CH₂)₅-CH₃, -(CH₂)₄-piperidinylene-(CH₂)₆-CH₃, -(CH₂)₄-piperidinylene-(CH₂)₇-CH₃, -(CH₂)₅-piperidinylene-CH₃, - (CH₂)₅-piperidinylene-CH₂-CH₃, -(CH₂)₅-piperidinylene-(CH₂)₂-CH₃, -(CH₂)₅-piperidinylene-(CH₂)₃-CH₃, -(CH₂)₅-piperidinylene-(CH₂)₄-CH₃, -(CH₂)₅-piperidinylene-(CH₂)₅-CH₃, -(CH₂)₅-piperidinylene-(CH₂)₆-CH₃, -(CH₂)₅-piperidinylene-(CH₂)₇-CH₃, -(CH₂)₆-piperidinylene-CH₃, - (CH₂)₆-piperidinylene-CH₂-CH₃, -(CH₂)₆-piperidinylene-(CH₂)₂-CH₃, -(CH₂)₆-piperidinylene-(CH₂)₃-CH₃, -(CH₂)₆-piperidinylene-(CH₂)₄-CH₃, -(CH₂)₆-piperidinylene-(CH₂)₅-CH₃, -(CH₂)₆-piperidinylene-(CH₂)₆-CH₃, -(CH₂)₆-piperidinylene-(CH₂)₇-CH₃, -(CH₂)₇-piperidinylene-CH₃, - (CH₂)₇-piperidinylene-CH₂-CH₃, -(CH₂)₇-piperidinylene-(CH₂)₂-CH₃, -(CH₂)₇-piperidinylene-(CH₂)₃-CH₃, -(CH₂)₇-piperidinylene-(CH₂)₇-CH₃, -(CH₂)₈-piperidinylene-CH₃, -(CH₂)₈-piperidinylene-CH₂-CH₃, -(CH₂)₈-piperidinylene-(CH₂)₂-CH₃, -(CH₂)₈-piperidinylene-(CH₂)₃-CH₃, -(CH₂)₈-piperidinylene-(CH₂)₄-CH₃, -(CH₂)₈-piperidinylene-(CH₂)₅-CH₃, -(CH₂)₈-piperidinylene-(CH₂)₆-CH₃, -(CH₂)₈-piperidinylene-(CH₂)₇-CH₃, -CH₂-N(Rₑ)-CH₂-piperidinylene-CH₃, -CH₂-N(Rₑ)-CH₂-piperidinylene-CH₃, -CH₂-N(Rₑ)-CH₂-piperidinylene-(CH₂)₂-CH₃, -CH₂-N(Rₑ)-CH₂-piperidinylene-(CH₂)₃-CH₃, -CH₂-N(Rₑ)-CH₂-piperidinylene-(CH₂)₄-CH₃, -CH₂-N(Rₑ)-CH₂-piperidinylene-(CH₂)₅-CH₃, -CH₂-N(Rₑ)-CH₂-piperidinylene-(CH₂)₆-CH₃, -CH₂-N(Rₑ)-CH₂-piperidinylene-(CH₂)₇-CH₃, - CH₂-N(Rₑ)-(CH₂)₂-piperidinylene-CH₃, -CH₂-N(Rₑ)-(CH₂)₂-piperidinylene-CH₂-CH₃, -CH₂-N(Rₑ)-(CH₂)₂-piperidinylene-(CH₂)₂-CH₃, -CH₂-N(Rₑ)-(CH₂)₂-piperidinylene-(CH₂)₃-CH₃, -CH₂-N(Rₑ)-(CH₂)₂-piperidinylene-(CH₂)₄-CH₃, -CH₂-N(Rₑ)-(CH₂)₂-pipendinylene-(CH₂)₅-CH₃, -CH₂-N(Rₑ)-(CH₂)₂-piperidinylene-(CH₂)₆-CH₃, -CH₂-N(Rₑ)-(CH₂)₂-piperidinylene-(CH₂)₇-CH₃, -(CH₂)₂-N(Rₑ)-CH₂-piperidinylene-CH₃, -(CH₂)₂-N(Rₑ)-CH₂-piperidinylene-CH₂-CH₃, -(CH₂)₂-N(Rₑ)-CH₂-piperidinylene-(CH₂)₂-CH₃, -(CH₂)₂-N(Rₑ)-CH₂-piperidinylene-(CH₂)₃-CH₃, -(CH₂)₂-N(Rₑ)-CH₂-piperidinylene-(CH₂)₄-CH₃, -(CH₂)₂-N(Rₑ)-CH₂-piperidinylene-(CH₂)₅-CH₃, -(CH₂)₂-N(Rₑ)-CH₂-piperidinylene-(CH₂)₆-CH₃, -(CH₂)₂-N(Rₑ)-CH₂-piperidinylene-(CH₂)₇-CH₃, -(CH₂)₃-N(Rₑ)-CH₂-piperidinylene-CH₃, -(CH₂)₃-N(Rₑ)-CH₂-piperidinylene-CH₂-CH₃, -(CH₂)₃-N(Rₑ)-CH₂-piperidinylene-(CH₂)₂-CH₃, -(CH₂)₃-N(Rₑ)-CH₂-piperidinylene-(CH₂)₇-CH₃, -(CH₂)₄-N(Rₑ)-CH₂-piperidinylene-CH₃, -(CH₂)₄-N(Rₑ)-CH₂-piperidinylene-CH₂-CH₃, -(CH₂)₄-N(Rₑ)-CH₂-piperidinylene-(CH₂)₂-CH₃, - (CH₂)₄-N(Rₑ)-CH₂-piperidinylene-(CH₂)₇-CH₃, -(CH₂)₅-N(Rₑ)-CH₂-piperidinylene-(CH₂)₂-CH₃, - (CH₂)₅-N(Rₑ)-CH₂-piperidinylene-(CH₂)₇-CH₃, -(CH₂)₆-N(Rₑ)-CH₂-piperidinylene-(CH₂)₂-CH₃, - (CH₂)₆-N(Rₑ)-CH₂-piperidinylene-(CH₂)₇-CH₃, -(CH₂)₇-N(Rₑ)-CH₂-piperidinylene-(CH₂)₂-CH₃, - (CH₂)₇-N(Rₑ)-CH₂-piperidinylene-(CH₂)₇-CH₃, -(CH₂)₈-N(Rₑ)-CH₂-piperidinylene-CH₃, -(CH₂)₈-N(Rₑ)-CH₂-piperidinylene-CH₂-CH₃, -(CH₂)₈-N(Rₑ)-CH₂-piperidinylene-(CH₂)₂-CH₃, -(CH₂)₈-N(Rₑ)-CH₂-piperidinylene-(CH₂)₃-CH₃, -(CH₂)₈-N(Rₑ)-CH₂-piperidinylene-(CH₂)₄-CH₃, -(CH₂)₈-N(Rₑ)-CH₂-piperidinylene-(CH₂)₅-CH₃, -(CH₂)₈-N(Rₑ)-CH₂-piperidinylene-(CH₂)₆-CH₃, -(CH₂)₈-N(Rₑ)-CH₂-piperidinylene-(CH₂)₇-CH₃, -CH₂-piperazinylene-CH₃, -CH₂-piperazinylene-CH₂-CH₃, -CH₂-piperazinylene-(CH₂)₂-CH₃, -CH₂-piperazinylene-(CH₂)₃-CH₃, -CH₂-piperazinylene-(CH₂)₄-CH₃, - CH₂-piperazinylene-(CH₂)₅-CH₃, -CH₂-piperazinylene-(CH₂)₆-CH₃, -CH₂-piperazinylene-(CH₂)₇-CH₃, -(CH₂)₂-piperazinylene-CH₃, -(CH₂)₂-piperazinylene-CH₂-CH₃, -(CH₂)₂-piperazinylene-(CH₂)₂-CH₃, - (CH₂)₂-piperazinylene-(CH₂)₃-CH₃, -(CH₂)₂-piperazinylene-(CH₂)₄-CH₃, -(CH₂)₂-piperazinylene-(CH₂)₅-CH₃, -(CH₂)₂-piperazinylene-(CH₂)₆-CH₃, -(CH₂)₂-piperazinylene-(CH₂)₇-CH₃, -(CH₂)₃-piperazinylene-CH₃, -(CH₂)₃-piperazinylene-CH₂-CH₃, -(CH₂)₃-piperazinylene-(CH₂)₂-CH₃, -(CH₂)₃-piperazinylene-(CH₂)₃-CH₃, -(CH₂)₃-piperazinylene-(CH₂)₄-CH₃, -(CH₂)₃-piperazinylene-(CH₂)₅-CH₃, -(CH₂)₃-piperazinylene-(CH₂)₆-CH₃, -(CH₂)₃-piperazinylene-(CH₂)₇-CH₃, -(CH₂)₄-piperazinylene-CH₃, -(CH₂)₄-piperazinylene-CH₂-CH₃, -(CH₂)₄-piperazinylene-(CH₂)₂-CH₃, -(CH₂)₄-piperazinylene-(CH₂)₃-CH₃, -(CH₂)₄-piperazinylene-(CH₂)₄-CH₃, -(CH₂)₄-piperazinylene-(CH₂)₅-CH₃, -(CH₂)₄-piperazinylene-(CH₂)₆-CH₃, -(CH₂)₄-piperazinylene-(CH₂)₇-CH₃, -(CH₂)₅-piperazinylene-CH₃, - (CH₂)₅-piperazinylene-CH₂-CH₃, -(CH₂)₅-piperazinylene-(CH₂)₂-CH₃, -(CH₂)₅-piperazinylene-(CH₂)₃-CH₃, -(CH₂)₅-piperazinylene-(CH₂)₄-CH₃, -(CH₂)₅-piperazinylene-(CH₂)₅-CH₃, -(CH₂)₅-piperazinylene-(CH₂)₆-CH₃, -(CH₂)₅-piperazinylene-(CH₂)₇-CH₃, -(CH₂)₆-piperazinylene-CH₃, - (CH₂)₆-piperazinylene-CH₂-CH₃, -(CH₂)₆-piperazinylene-(CH₂)₂-CH₃, -(CH₂)₆-piperazinylene-(CH₂)₃-CH₃, -(CH₂)₆-piperazinylene-(CH₂)₄-CH₃, -(CH₂)₆-piperazinylene-(CH₂)₅-CH₃, -(CH₂)₆-piperazinylene-(CH₂)₆-CH₃, -(CH₂)₆-piperazinylene-(CH₂)₇-CH₃, -(CH₂)₇-piperazinylene-CH₃, - (CH₂)₇-piperazinylene-CH₂-CH₃, -(CH₂)₇-piperazinylene-(CH₂)₂-CH₃, -(CH₂)₇-piperazinylene-(CH₂)₃-CH₃, -(CH₂)₇-piperazinylene-(CH₂)₇-CH₃, -(CH₂)₈-piperazinylene-CH₃, -(CH₂)₈-piperazinylene-CH₂-CH₃, -(CH₂)₈-piperazinylene-(CH₂)₂-CH₃, -(CH₂)₈-piperazinylene-(CH₂)₃-CH₃, -(CH₂)₈-piperazinylene-(CH₂)₄-CH₃, -(CH₂)₈-piperazinylene-(CH₂)₅-CH₃, -(CH₂)₈-piperazinylene-(CH₂)₆-CH₃, or -(CH₂)₈-piperazinylene-(CH₂)₇-CH₃;
wherein the piperidinylene and piperazinylene are each independently optionally substituted with a substituent selected from the group consisting of deuterium, optionally deuterated C₁₋₆ alkyl (e.g., optionally deuterated C₁₋₄ alkyl, such as methyl, CD₃, ethyl, propyl, isopropyl, butyl, sec-butyl, tert-butyl, pentyl, or hexyl), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), hydroxy, amino, mercapto, halogen (e.g., fluorine, chlorine, bromine, or iodine), optionally deuterated C₁₋₆ alkoxy (e.g., optionally deuterated C₁₋₄ alkoxy, such as methoxy, CD₃-O-, ethoxy, propoxy, isopropoxy, n-butoxy, sec-butoxy, or tert-butoxy), optionally deuterated C₁₋₆ alkyl-NH- (e.g., optionally deuterated C₁₋₃ alkyl-NH-, such as CH₃NH-, CH₃CH₂NH- or CH₃CH₂CH₂NH-), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH- (e.g., optionally deuterated C₁₋₄ alkyl-C(O)NH-, such as CH₃-C(O)NH-, CH₃CH₂-C(O)NH-, and CH₃CH₂CH₂-C(O)NH-), cyano, or any combination thereof;
a hydrogen atom of CH₃ of the groups and a hydrogen atom of one or more (e.g., 1-30, such as 1-25, 1-20, 1-15, 1-10, 1-5, 1-4, 1-3, 1-2 or 1) CH₂ of the groups are optionally substituted with a substituent selected from the group consisting of deuterium, hydroxy, amino, mercapto, nitro, halogen (e.g., fluorine, chlorine, bromine, or iodine), cyano, optionally deuterated C₁₋₆ alkyl (e.g., optionally deuterated C₁₋₄ alkyl, such as methyl, CD₃, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, n-butoxy, sec-butoxy, or tert-butoxy), optionally deuterated C₁₋₆ alkyl-NH- (e.g., optionally deuterated C₁₋₃ alkyl-NH-, such as CH₃NH-, CH₃CH₂NH- or CH₃CH₂CH₂NH-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH- (e.g., optionally deuterated C₁₋₄ alkyl-C(O)NH-, such as CH₃-C(O)NH-, CH₃CH₂-C(O)NH-, and CH₃CH₂CH₂-C(O)NH-) or any combination thereof;
ach Rₑ independently represents H or C₁₋₆ alkyl (e.g., C₁₋₄ alkyl, such as methyl, CD₃, ethyl, propyl, isopropyl, butyl, sec-butyl, tert-butyl, pentyl, or hexyl); and
n1, n2, n3, n4, m4, m5, and m6 each independently represent an integer of 1, 2, 3, 4, 5, 6, 7, 8, 9, 10, 11, 12, 13, 14, or 15.

In some embodiments, R₅ and R₆ of the compound of Formula (I) represent hydrogen.

In some embodiments, (R_{b})ₙ of the compound of Formula (I) indicates that the benzene ring is substituted with n R_{b} substituents, with each R_{b} being the same or different and independently representing hydrogen, deuterium, halogen (e.g., fluorine, chlorine, bromine, or iodine), hydroxy, mercapto, nitro, amino, cyano, optionally deuterated C₁₋₆ alkyl (e.g., optionally deuterated C₁₋₄ alkyl, such as methyl, CD₃, ethyl, propyl, isopropyl, butyl, sec-butyl, or tert-butyl), optionally deuterated C₁₋₆ alkoxy (e.g., optionally deuterated C₁₋₄ alkoxy, such as methoxy, CD₃-O-, ethoxy, propoxy, isopropoxy, n-butoxy, sec-butoxy, or tert-butoxy), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally substituted C₃₋₆ cycloalkyl (e.g., optionally perdeuterated cyclopropyl, optionally perdeuterated cyclobutyl, optionally perdeuterated cyclopentyl, or optionally perdeuterated cyclohexyl), C₂₋₆ alkenyl (e.g., vinyl, propenyl, or butenyl) or C₂₋₆ alkynyl (e.g., ethynyl, propynyl, or butynyl); and wherein n represents an integer of 0, 1, 2, or 3. In some sub-embodiments, the substituents of the optionally substituted C₃₋₆ cycloalkyl are selected from C₁-C₃ alkyl, C₃₋₆ cycloalkyl, hydroxy, amino, mercapto, halogen, C₁-C₃ alkoxy, C₁-C₃ alkyl-NH-, halogenated C₁-C₃ alkyl, NH₂-C₁₋₃ alkylene, C₁₋₃ alkyl-NHC(O)-, C₁₋₃ alkyl-C(O)NH-, cyano or any combination thereof; and the number of substituents can be one or more (e.g., 1-5, 1-4, 1-3, 1-2, or 1). The number of substituents is not theoretically limited in any way or automatically limited by the size of the building units.

In some embodiments, the compound of Formula (I) is also of Formula (1-1) or Formula (1-2): 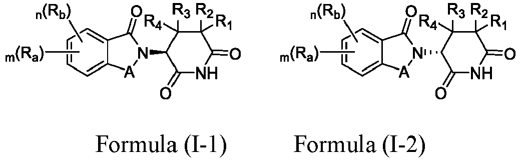 wherein A, R₁, R₂, R₃, R₄, (R_{b})ₙ and (Rₐ)ₘ are as defined in the compound of Formula (I) and its various embodiments above.

In some embodiments, the compound of Formula (I) is also of Formula (1-3) or Formula (1-4): 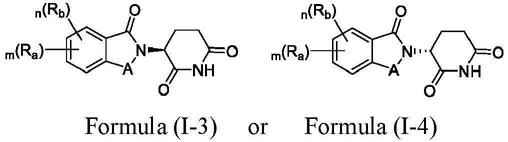 wherein A, (R_{b})ₙ and (Rₐ)ₘ are as defined in the compound of Formula (I) and its various embodiments above.

In some embodiments, the compound of Formula (I) is also of Formula (1-5), Formula (1-6), Formula (1-7) or Formula (1-8): 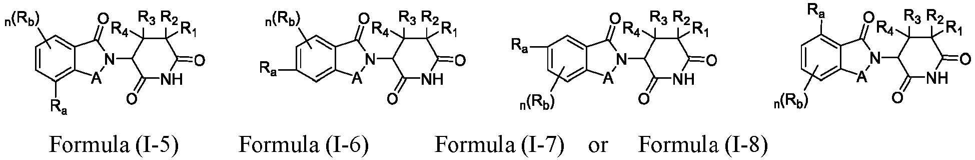 wherein A, R₁, R₂, R₃, R₄, (R_{b})ₙ and Rₐ are as defined in the compound of Formula (I) and its various embodiments above.

In some embodiments, in the compounds of Formula (1-5), (1-6), (1-7) or (I-8),
A represents CO, CH₂ or CD₂;
R₁, R₂, R₃ and R₄ are the same or different and each independently represent hydrogen, deuterium, halogen (e.g., fluorine, chlorine, bromine, or iodine), optionally deuterated C₁₋₆ alkyl (e.g., optionally deuterated C₁₋₄ alkyl, such as methyl, CD₃, ethyl, propyl, isopropyl, butyl, sec-butyl, tert-butyl, pentyl, or hexyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
(Rₐ)ₘ indicates that benzene ring is substituted with m Rₐ substituents, with each Rₐ being the same or different and independently representing the structure of the following formula: 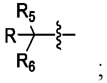
   wherein R represents chlorine, bromine, iodine, hydroxy, leaving group (e.g., -N₃, chlorine, bromine, iodine, sulfonate, such as methanesulfonyloxy (MsO-), trifluoromethanesulfonyloxy (TfO-) or p-toluenesulfonyloxy (TsO-)), NR₇R₈ or W¹,
      wherein R₇ and R₈ are the same or different and each independently represent hydrogen, C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), C₂₋₆ alkynyl-C₁₋₆ alkylene- (e.g., C₂₋₆ alkynyl-C₁₋₄ alkylene- or C₂₋₆ alkynyl-C₁₋₃ alkylene-, such as ethynyl-methylene-, ethynyl-ethylene-, ethynyl-propylene-, ethynyl-butylene-, ethynyl-pentylene-, or ethynyl-hexylene-), C₂₋₆ alkenyl-C₁₋₆ alkylene- (e.g., C₂₋₆ alkenyl-C₁₋₄ alkylene- or C₂₋₆ alkenyl-C₁₋₃ alkylene-, such as vinyl-methylene-, vinyl-ethylene-, vinyl-propylene-, vinyl-butylene-, vinyl-pentylene-, or vinyl-hexylene-), optionally substituted 4- to 15-membered (e.g., 4- to 11-membered, 4- to 10-membered, 4- to 9-membered, 4- to 8-membered, 4- to 7-membered, 4- to 6-membered, 5- to 15-membered, or 5- to 9-membered) nitrogen-containing heterocyclyl, or R^{2a}-R^{1a}-, where R^{1a} is optionally substituted C₁₋₆ alkylene (e.g., optionally substituted C₁₋₄ alkylene, such as optionally substituted methylene, ethylene, or propylene) or optionally substituted C₂₋₆ alkenylene (e.g., optionally substituted C₂₋₄ alkenylene, such as optionally substituted vinylene, propenylene, or butenylene), and R^{2a} represents halogen (e.g., fluorine, chlorine, bromine, or iodine), amino, hydroxy, carboxyl, C₂₋₆ alkynyl (e.g., C₂₋₄ alkynyl, such as ethynyl, propynyl, or butynyl), C₂₋₆ alkenyl (e.g., C₂₋₄ alkenyl, such as vinyl, propenyl, or butenyl) or a leaving group (e.g., -N₃, chlorine, bromine, iodine, sulfonate, e.g., methanesulfonyloxy (MsO-), trifluoromethanesulfonyloxy (TfO-), or p-toluenesulfonyloxy (TsO-)); and
      W¹ represents the structure of the following formula: 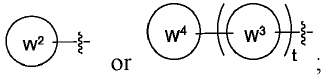 wherein ring W² represents optionally substituted nitrogen-containing heterocyclyl (e.g., optionally substituted 4- to 20-membered nitrogen-containing heterocyclyl), and each ring W³ is the same or different and each independently represents optionally substituted nitrogen-containing heterocyclylene (e.g., optionally substituted 4- to 20-membered nitrogen-containing heterocyclylene), t represents an integer of 1 or 2, and ring W⁴ represents optionally substituted aryl (e.g., optionally substituted 6- to 10-membered aryl), optionally substituted heteroaryl (e.g., optionally substituted 5- to 10-membered heteroaryl) or optionally substituted heterocyclyl (e.g., 4- to 15-membered nitrogen-containing heterocyclyl);
   R₅ and R₆ are the same or different and each independently represent hydrogen, fluorine, chlorine, bromine, iodine, optionally substituted methyl, or optionally substituted linear or branched C₂₋₃₀ alkyl, wherein one or more (e.g., 1-20, or 1-15, 1-10, 1-9, 1-8, 1-7, 1-6, 1-5, 1-4, 1-3, 1-2, or 1) groups R_{c} and/or one or more (e.g., 1-20, or 1-15, 1-10, 1-9, 1-8, 1-7, 1-6, 1-5, 1-4, 1-3, 1-2, or 1) groups R_{d} and/or any combination of one or more (e.g., 1-20, or 1-15, 1-10, 1-9, 1-8, 1-7, 1-6, 1-5, 1-4, 1-3, 1-2, or 1) groups R_{c} and R_{d} are optionally inserted into the backbone carbon chain of the linear or branched C₂₋₃₀ alkyl group, wherein carbon-carbon bond between one or more (e.g., 1-15, 1-10, 1-9, 1-8, 1-7, 1-6, 1-5, 1-4, 1-3, 1-2, or 1) pairs of adjacent carbon atoms in the backbone carbon chain is interrupted by the group R_{c}, R_{d}, or a combination of R_{c} and R_{d}; wherein each R_{c} is selected from the group consisting of O, N(Rₑ), C(O), C(O)O, S(O), S(O)₂, S(O)₂NH, NHS(O)₂, OC(O), C(O)N(Rₑ), N(Rₑ)C(O), or N(Rₑ)C(O)N(Rₑ), where each Rₑ independently represents H or C₁₋₆ alkyl (e.g., C₁₋₄ alkyl, such as methyl, ethyl, propyl, isopropyl, butyl, sec-butyl, tert-butyl, pentyl, or hexyl), and in case that two or more groups R_{c} are inserted into the backbone carbon chain of the linear or branched C₂₋₃₀ alkyl group, the two or more groups R_{c} are not directly connected to each other; and wherein each R_{d} is selected from the group consisting of cycloalkylene (e.g., C₃₋₂₀ cycloalkylene), arylene (e.g., C₃₋₂₀ arylene), heterocyclylene (e.g., 4- to 20-membered heterocyclylene), heteroarylene (e.g., 4- to 20-membered heteroarylene), alkynylene (e.g., C₂₋₆ alkynylene), alkenylene (e.g., C₂₋₆ alkenylene), or any combination thereof, wherein the cycloalkylene, arylene, heterocyclylene, and heteroarylene are each independently optionally substituted with a substituent selected from the group consisting of deuterium, (e.g., optionally deuterated C₁₋₄ alkyl, such as methyl, CD₃, ethyl, propyl, isopropyl, butyl, sec-butyl, tert-butyl, pentyl, or hexyl), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), hydroxy, amino, mercapto, halogen (e.g., fluorine, chlorine, bromine, or iodine), cyano, optionally deuterated C₁₋₆ alkoxy (e.g., optionally deuterated C₁₋₄ alkoxy, such as methoxy, CD₃-O-, ethoxy, propoxy, isopropoxy, n-butoxy, sec-butoxy, or tert-butoxy), optionally deuterated C₁₋₆ alkyl-NH- (e.g., optionally deuterated C₁₋₃ alkyl-NH-, such as CH₃NH-, CH₃CH₂NH- or CH₃CH₂CH₂NH-), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH- (e.g., optionally deuterated C₁₋₄ alkyl-C(O)NH-, such as CH₃-C(O)NH-, CH₃CH₂-C(O)NH-, and CH₃CH₂CH₂-C(O)NH-), or any combination thereof;
      or
   R and R₅ together with the carbon atom to which they are connected form carbonyl group, and R₆ represents hydrogen, optionally substituted methyl, or linear or branched C₂₋₃₀ alkyl, wherein one or more (e.g., 1-20, or 1-15, 1-10, 1-9, 1-8, 1-7, 1-6, 1-5, 1-4, 1-3, 1-2, or 1) groups R_{c} and/or one or more (e.g., 1-20, or 1-15, 1-10, 1-9, 1-8, 1-7, 1-6, 1-5, 1-4, 1-3, 1-2, or 1) groups R_{d} and/or any combination of one or more (e.g., 1-20, or 1-15, 1-10, 1-9, 1-8, 1-7, 1-6, 1-5, 1-4, 1-3, 1-2, or 1) groups R_{c} and R_{d} are optionally inserted into the backbone carbon chain of the linear or branched C₂₋₃₀ alkyl group, wherein carbon-carbon bond between one or more (e.g., 1-15, 1-10, 1-9, 1-8, 1-7, 1-6, 1-5, 1-4, 1-3, 1-2, or 1) pairs of adjacent carbon atoms in the backbone carbon chain is interrupted by the group R_{c}, R_{d}, or a combination of R_{c} and R_{d}; wherein each R_{c} is selected from the group consisting of O, N(Rₑ), C(O), C(O)O, S(O), S(O)₂, S(O)₂NH, NHS(O)₂, OC(O), C(O)N(Rₑ), N(Rₑ)C(O), or N(Rₑ)C(O)N(Rₑ), where each Rₑ independently represents H or C₁₋₆ alkyl (e.g., C₁₋₄ alkyl, such as methyl, ethyl, propyl, isopropyl, butyl, sec-butyl, tert-butyl, pentyl, or hexyl), and in case that two or more groups R_{c} are inserted into the backbone carbon chain of the linear or branched C₂₋₃₀ alkyl group, the two or more groups R_{c} are not directly connected to each other; and wherein each R_{d} is selected from the group consisting of cycloalkylene (e.g., C₃₋₂₀ cycloalkylene), arylene (e.g., C₃₋₂₀ arylene), heterocyclylene (e.g., 4- to 20-membered heterocyclylene), heteroarylene (e.g., 4- to 20-membered heteroarylene), alkynylene (e.g., C₂₋₆ alkynylene), alkenylene (e.g., C₂₋₆ alkenylene), or any combination thereof, wherein the cycloalkylene, arylene, heterocyclylene, and heteroarylene are each independently optionally substituted with a substituent selected from the group consisting of deuterium, optionally deuterated C₁₋₆ alkyl (e.g., optionally deuterated C₁₋₄ alkyl, such as methyl, CD₃, ethyl, propyl, isopropyl, butyl, sec-butyl, tert-butyl, pentyl, or hexyl), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), hydroxy, amino, mercapto, halogen (e.g., fluorine, chlorine, bromine, or iodine), optionally deuterated C₁₋₆ alkoxy (e.g., optionally deuterated C₁₋₄ alkoxy, such as methoxy, CD₃-O-, ethoxy, propoxy, isopropoxy, n-butoxy, sec-butoxy, or tert-butoxy), optionally deuterated C₁₋₆ alkyl-NH- (e.g., optionally deuterated C₁₋₃ alkyl-NH-, such as CH₃NH-, CH₃CH₂NH- or CH₃CH₂CH₂NH-), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH- (e.g., optionally deuterated C₁₋₄ alkyl-C(O)NH-, such as CH₃-C(O)NH-, CH₃CH₂-C(O)NH-, and CH₃CH₂CH₂-C(O)NH-), cyano, or any combination thereof;
   (R_{b})ₙ indicates that the benzene ring is substituted with n R_{b} substituents, with each R_{b} being the same or different and independently representing hydrogen, deuterium, halogen (e.g., fluorine, chlorine, bromine, or iodine), hydroxy, mercapto, nitro, amino, cyano, optionally deuterated C₁₋₆ alkyl (e.g., optionally deuterated C₁₋₄ alkyl, such as methyl, CD₃, ethyl, propyl, isopropyl, butyl, sec-butyl, or tert-butyl), optionally deuterated C₁₋₆ alkoxy (e.g., optionally deuterated C₁₋₄ alkoxy, such as methoxy, CD₃-O-, ethoxy, propoxy, isopropoxy, n-butoxy, sec-butoxy, or tert-butoxy), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally substituted C₃₋₆ cycloalkyl (e.g., optionally perdeuterated cyclopropyl, optionally perdeuterated cyclobutyl, optionally perdeuterated cyclopentyl, or optionally perdeuterated cyclohexyl), C₂₋₆ alkenyl (e.g., vinyl, propenyl, or butenyl) or C₂₋₆ alkynyl (e.g., ethynyl, propynyl, or butynyl); and
   m represents an integer of 1, 2, 3, or 4, n represents an integer of 0, 1, 2, or 3, and the sum of m and n is an integer of 1, 2, 3, or 4;
   wherein when R and R₅ together with the carbon atom to which they are connected form a carbonyl group and R₆ represents hydrogen, m represents an integer of 1, 2, or 3, n represents an integer of 1, 2, or 3, and the sum of m and n is an integer of 2, 3, or 4;
with the proviso that the following compounds and compounds of Formula (I') are excluded: 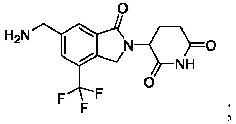 and
   when A represents CH₂ or C(O) and R represents unsubstituted piperazinyl, m represents an integer of 1 and n represents an integer of 0;
   when A represents CH₂ or C(O) and Rₐ represents H₂N-CH₂-, m represents an integer of 1 and n represents an integer of 0;
   when A represents C(O) and R represents bromine, m represents an integer of 1 and n represents an integer of 0; and
   the compounds of Formula (I'): 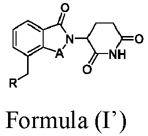
   wherein A represents CH₂ or C(O), and R represents unsubstituted piperidinyl or methylamino substituted piperidinyl.

In some embodiments, the compounds of Formula (I) and their salts (including pharmaceutically acceptable salts, such as hydrochloride, etc.), prodrugs, solvates, isotopically enriched analogs, polymorphs, stereoisomers (including enantiomers and diastereomers), or mixture of stereoisomers thereof in Table 1 below are provided:

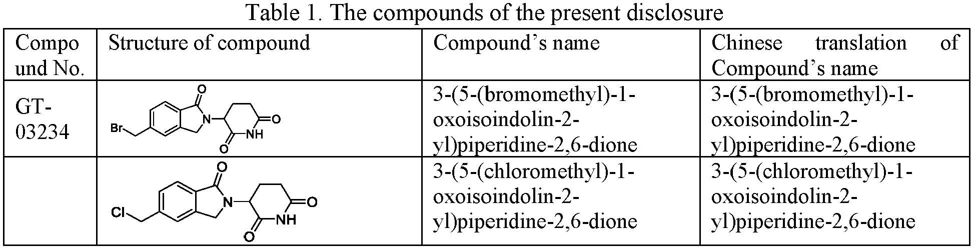

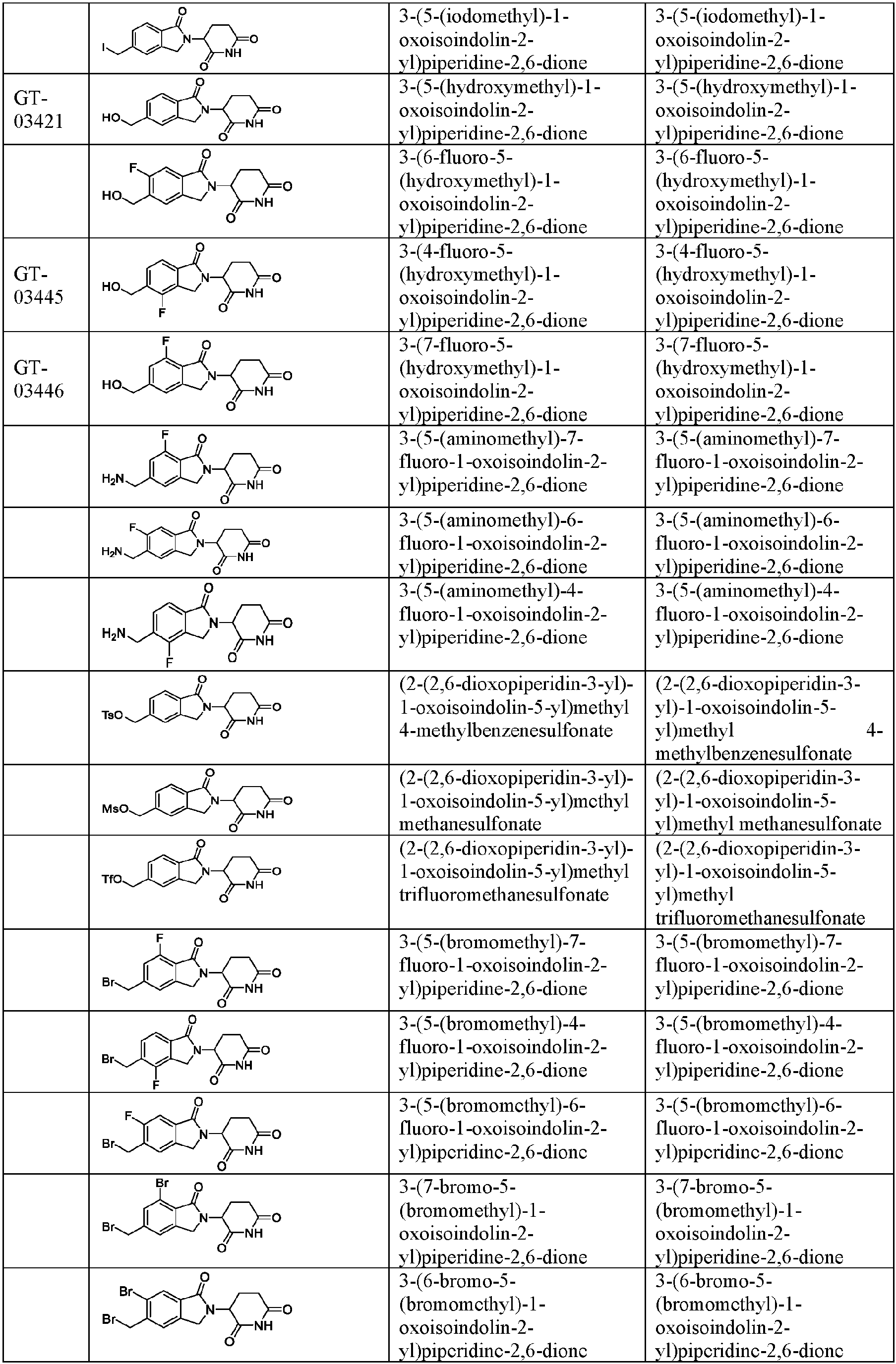

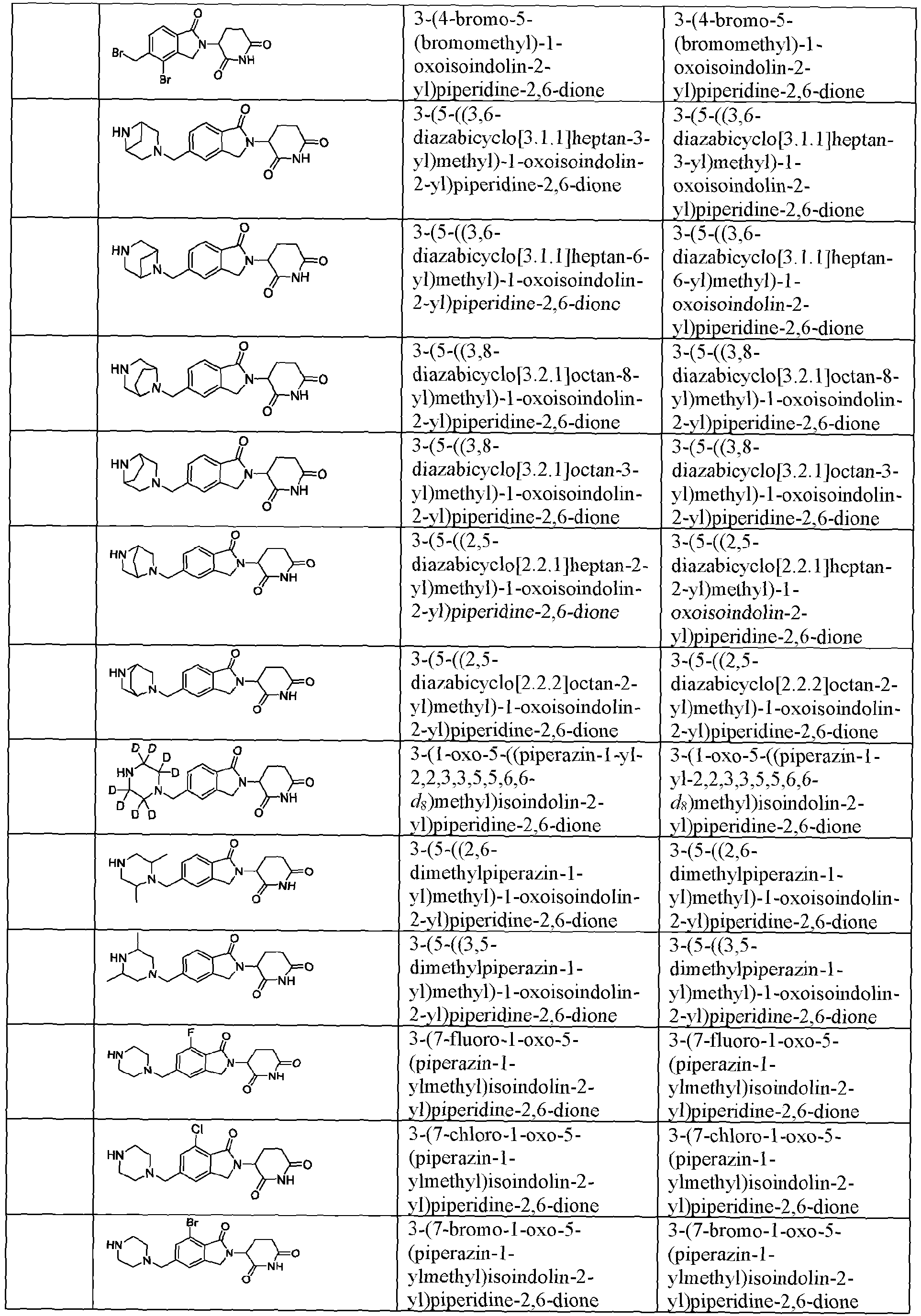

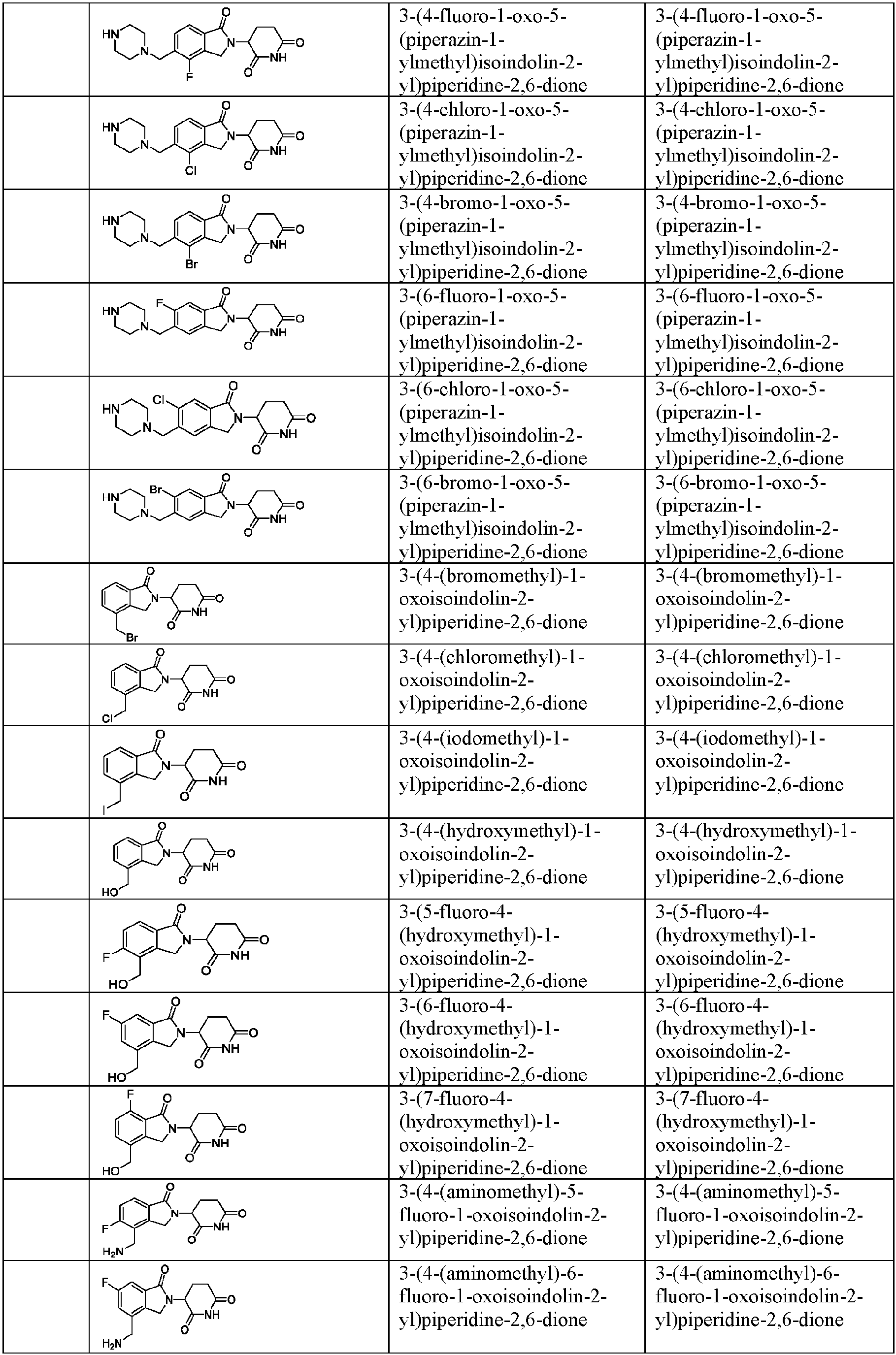

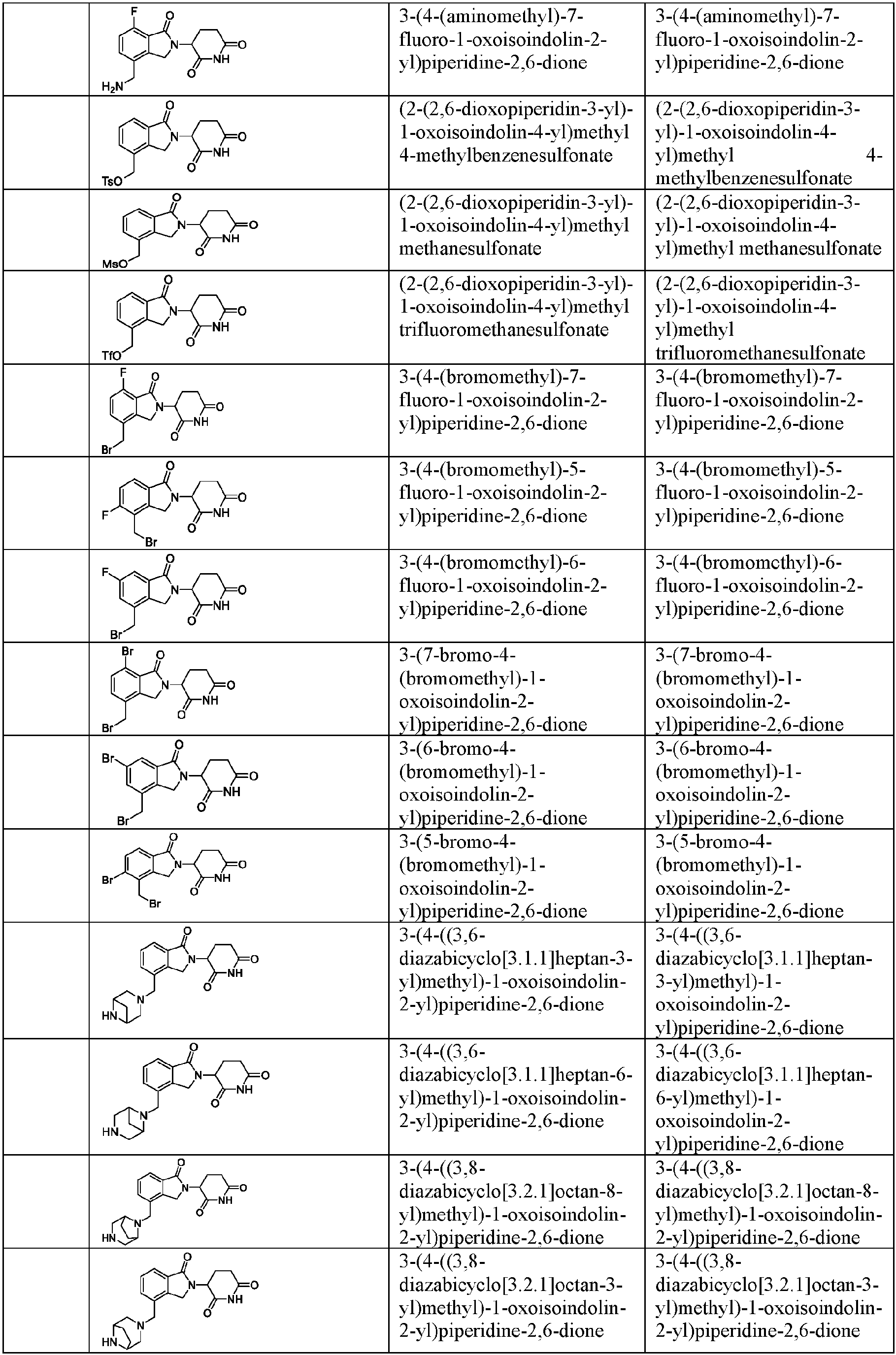

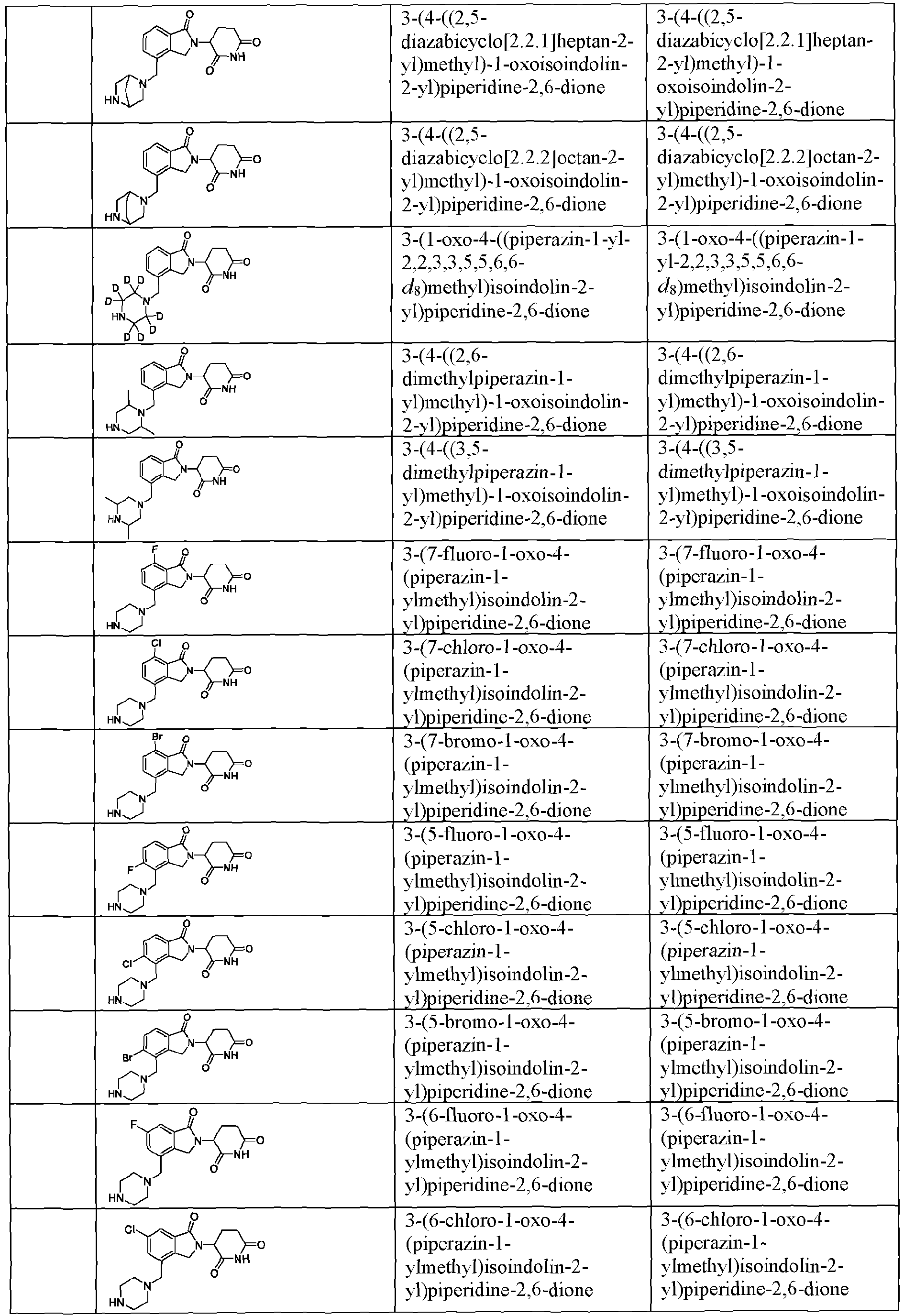

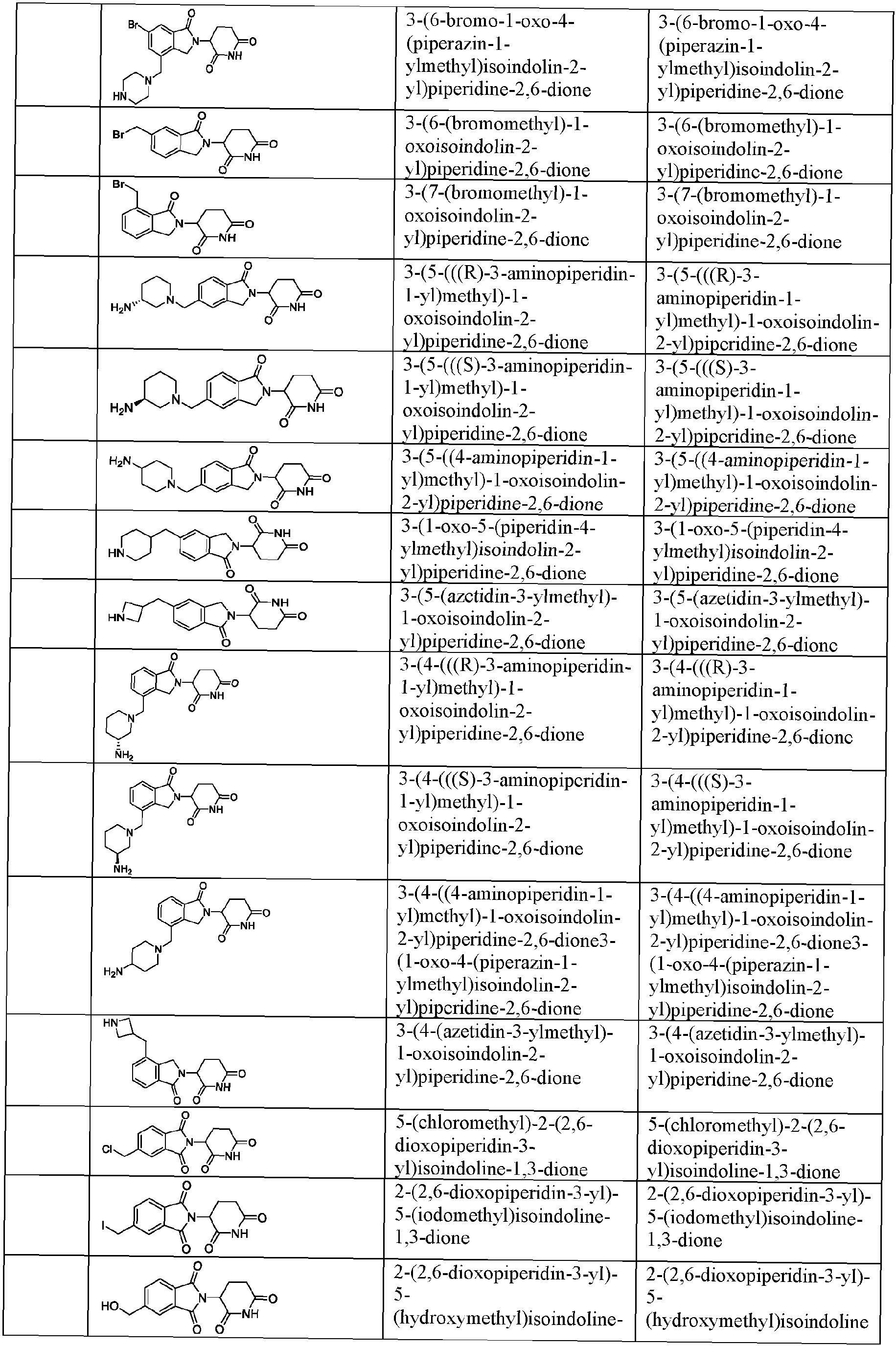

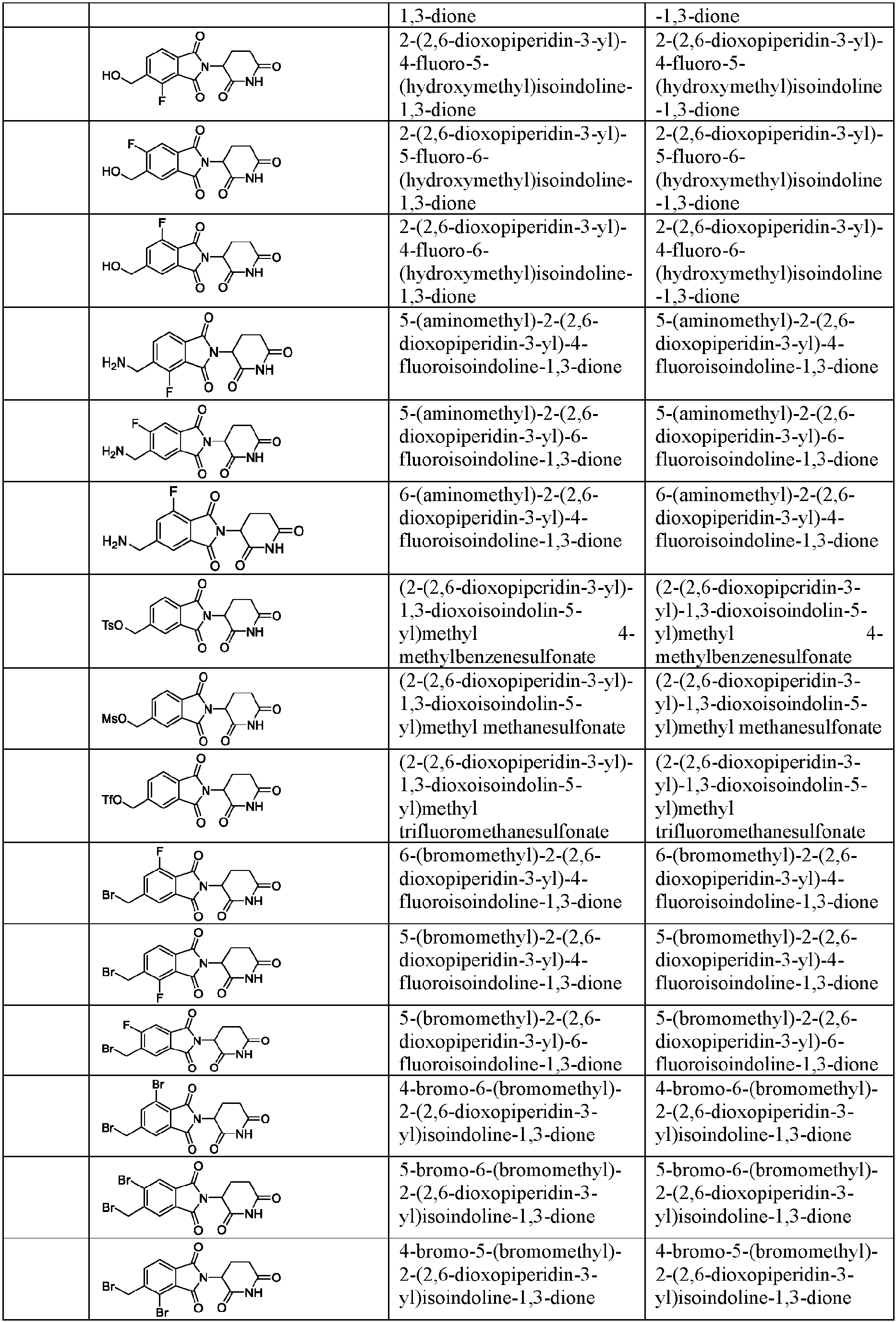

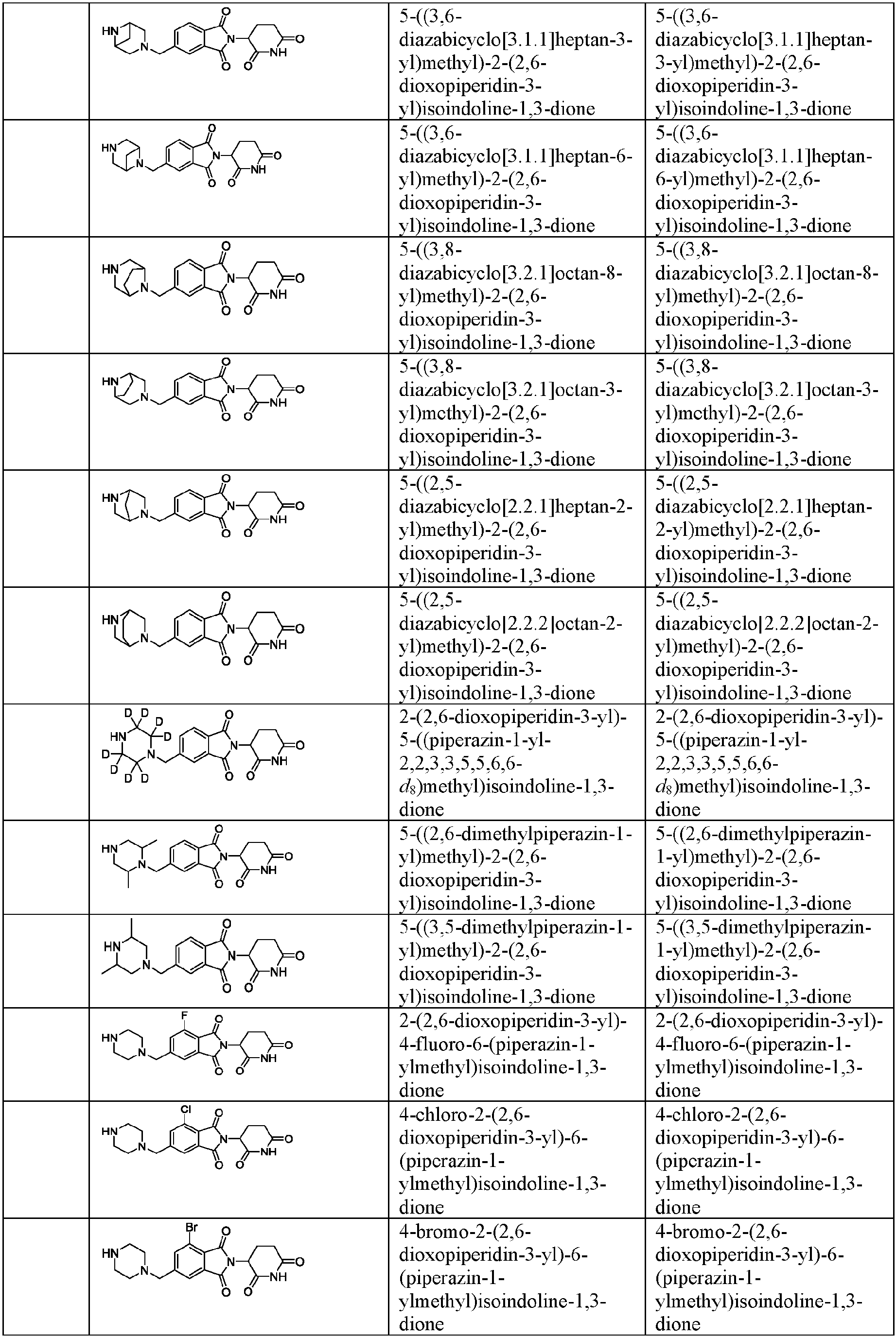

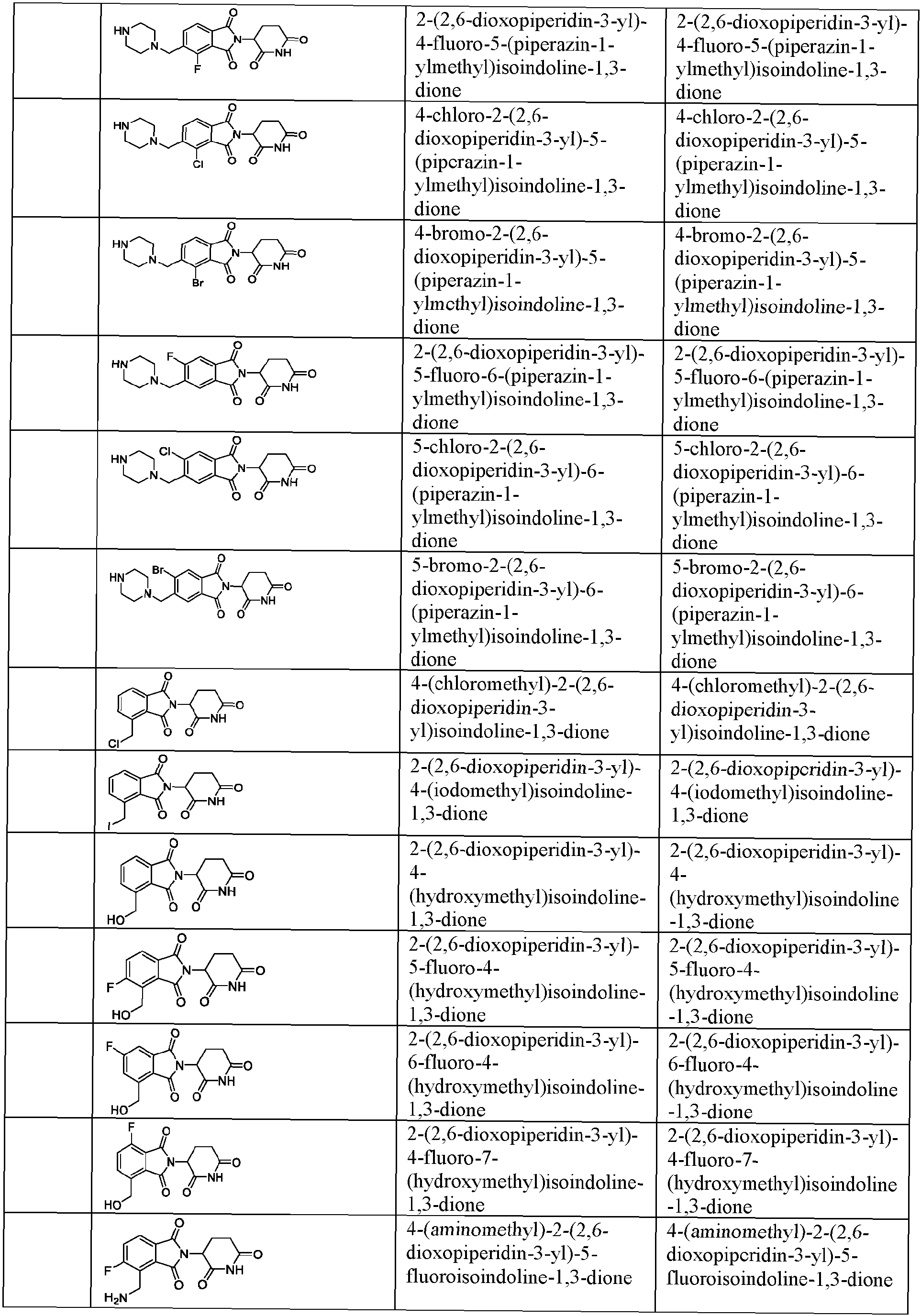

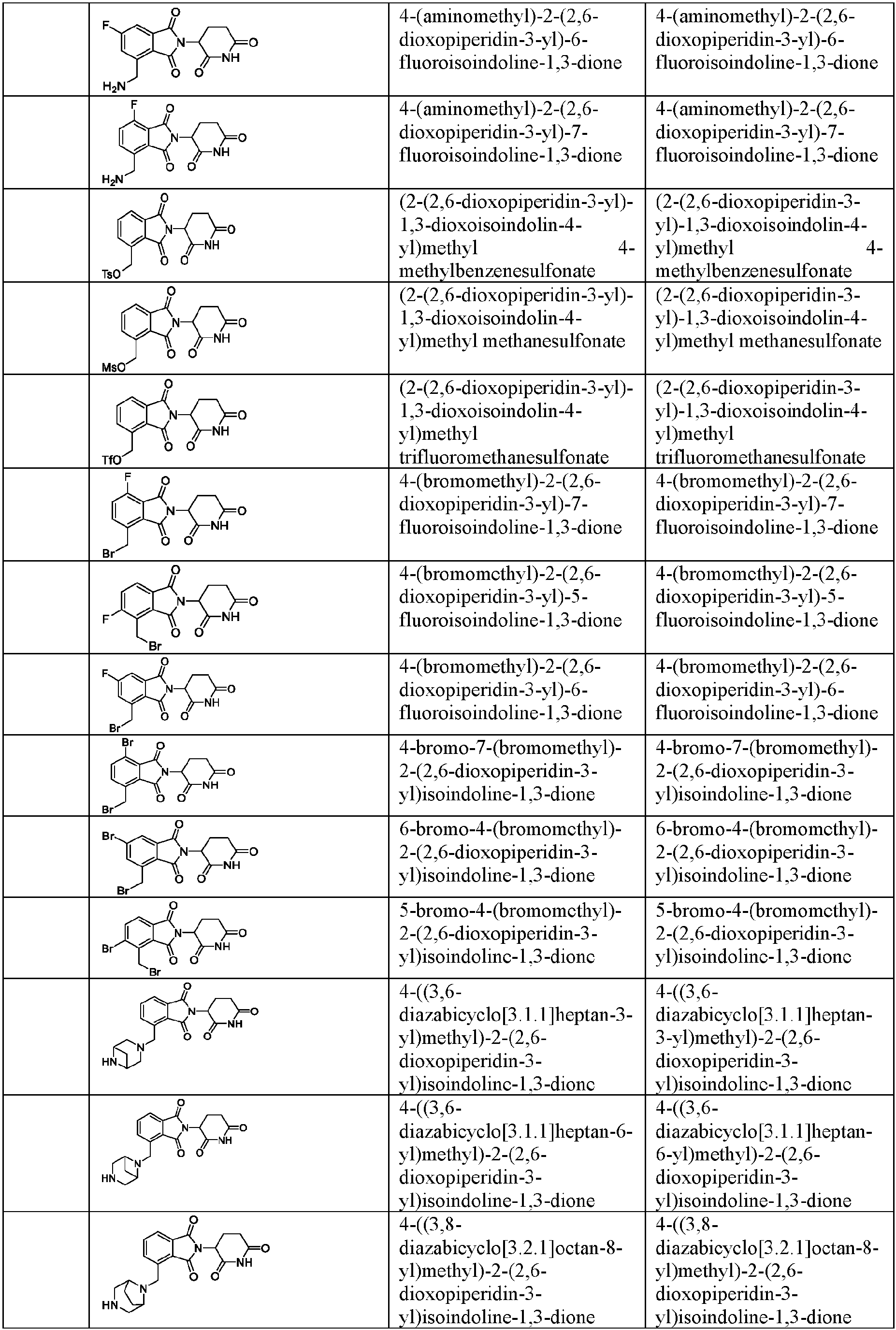

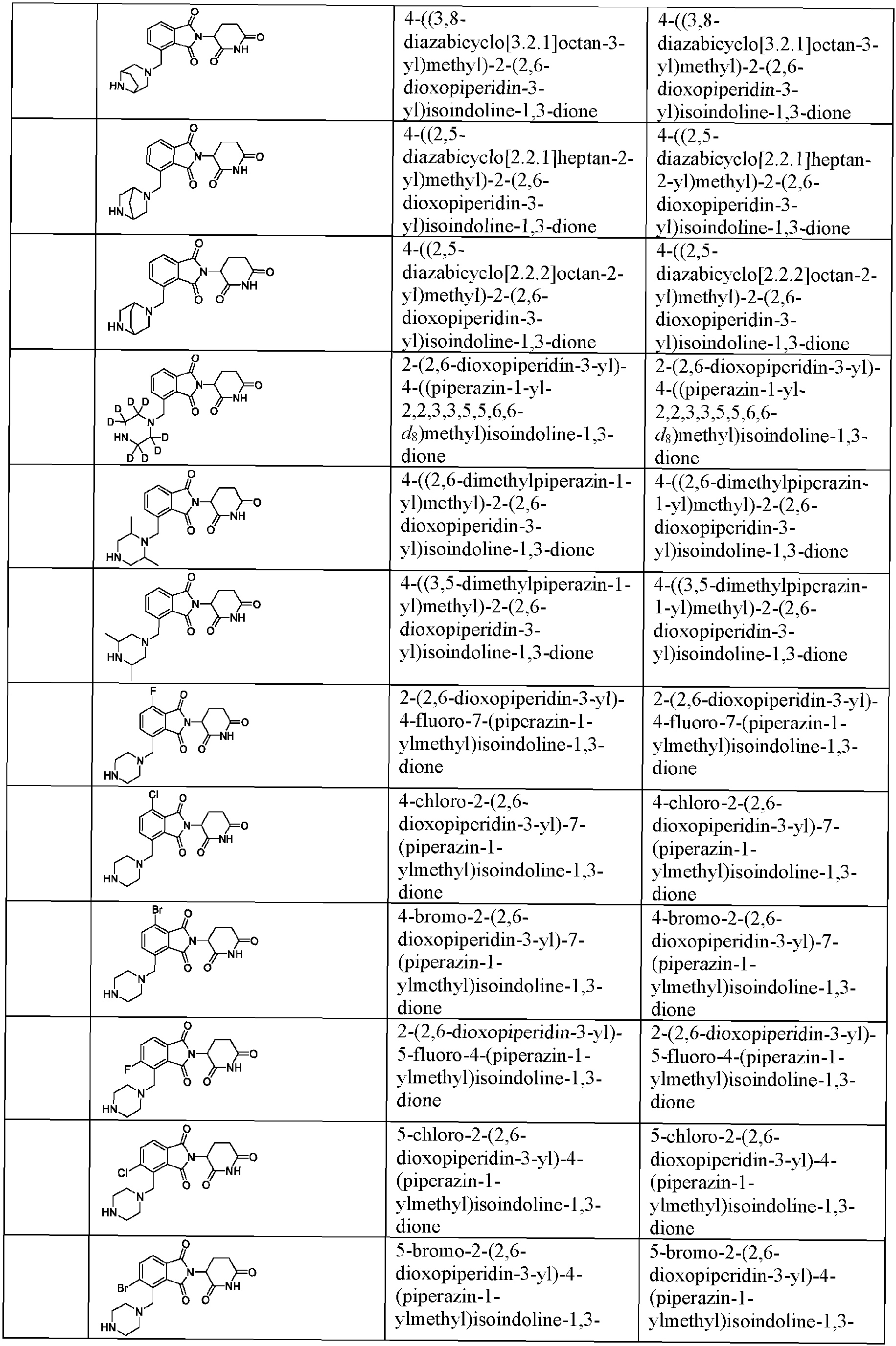

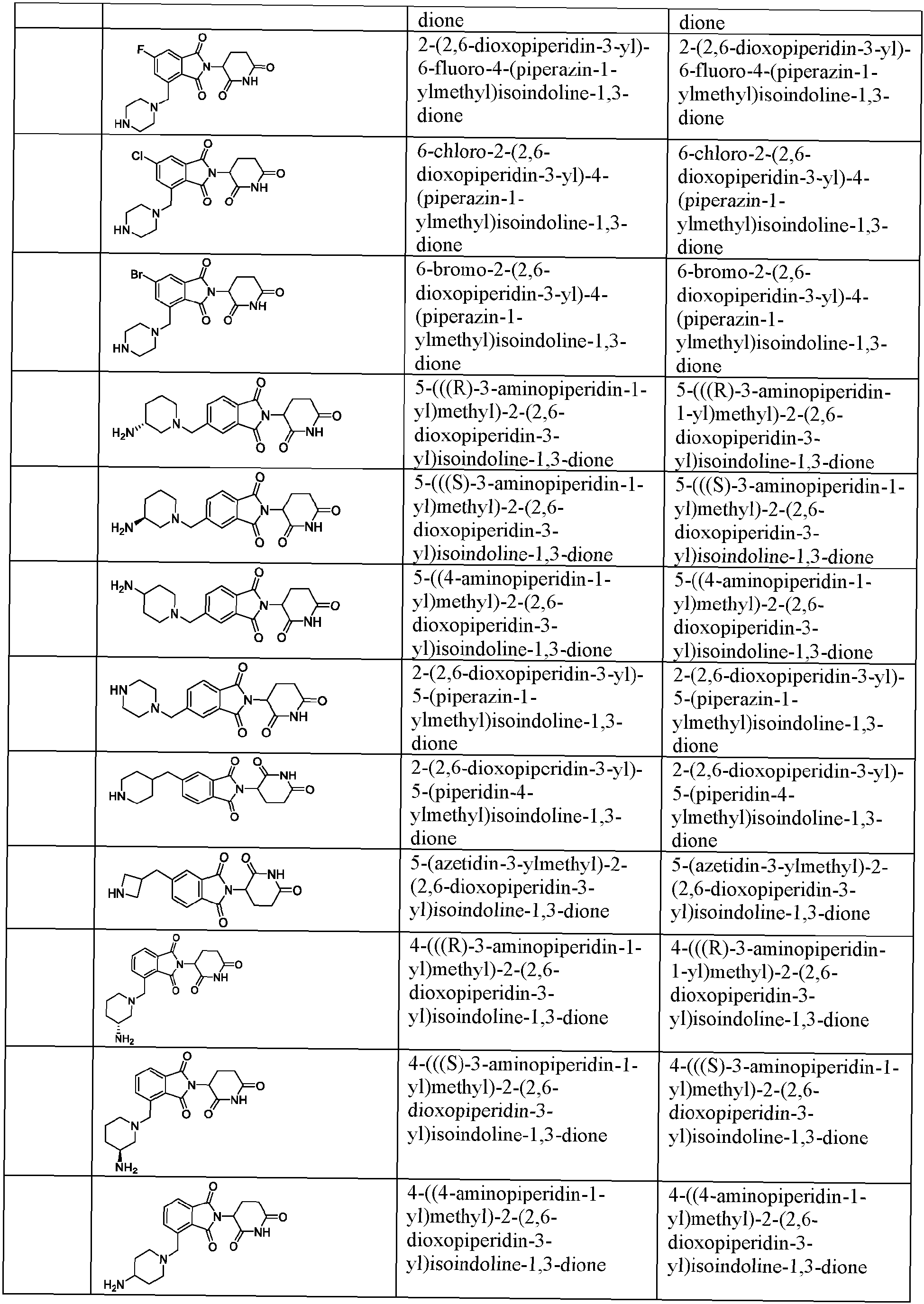

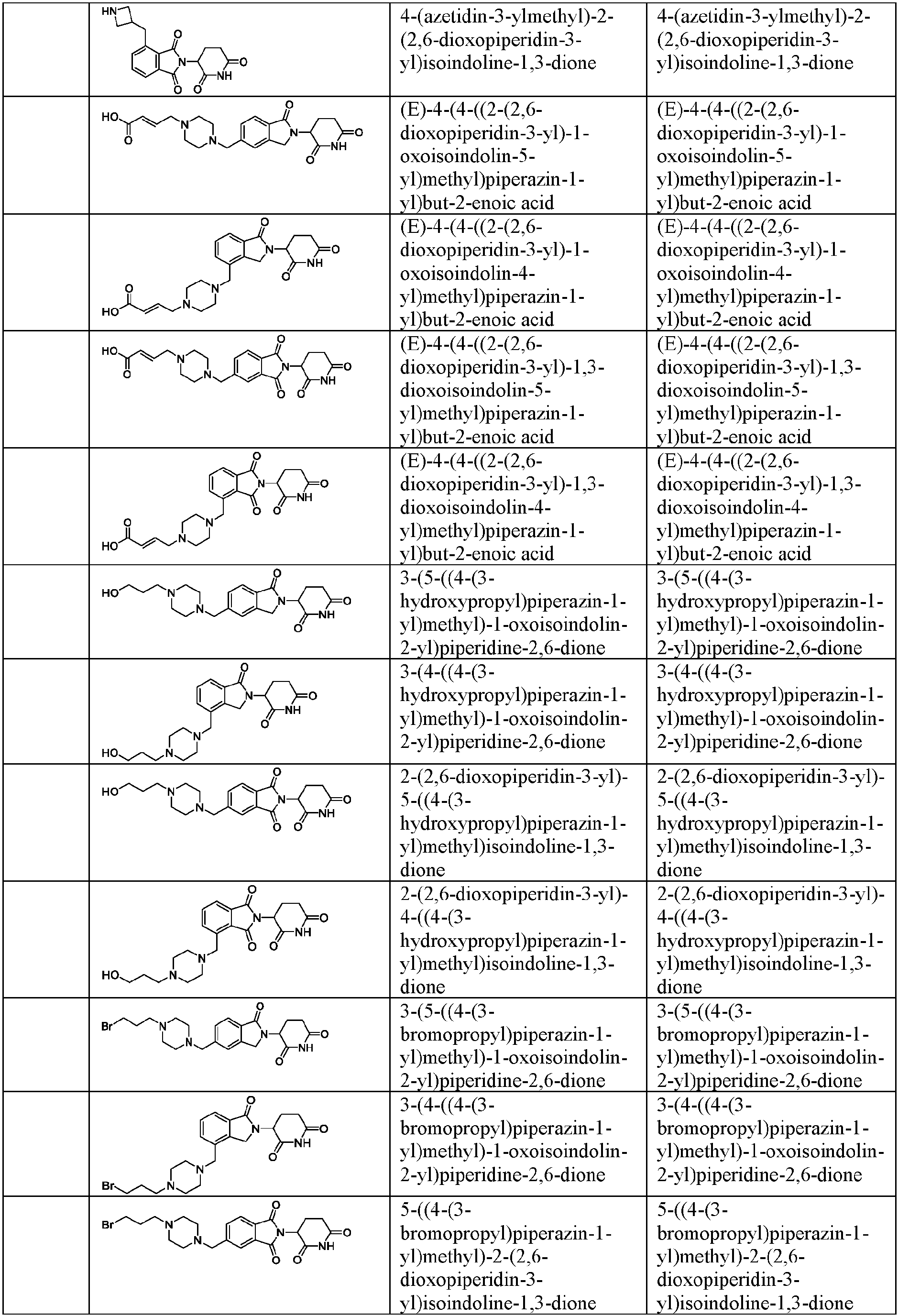

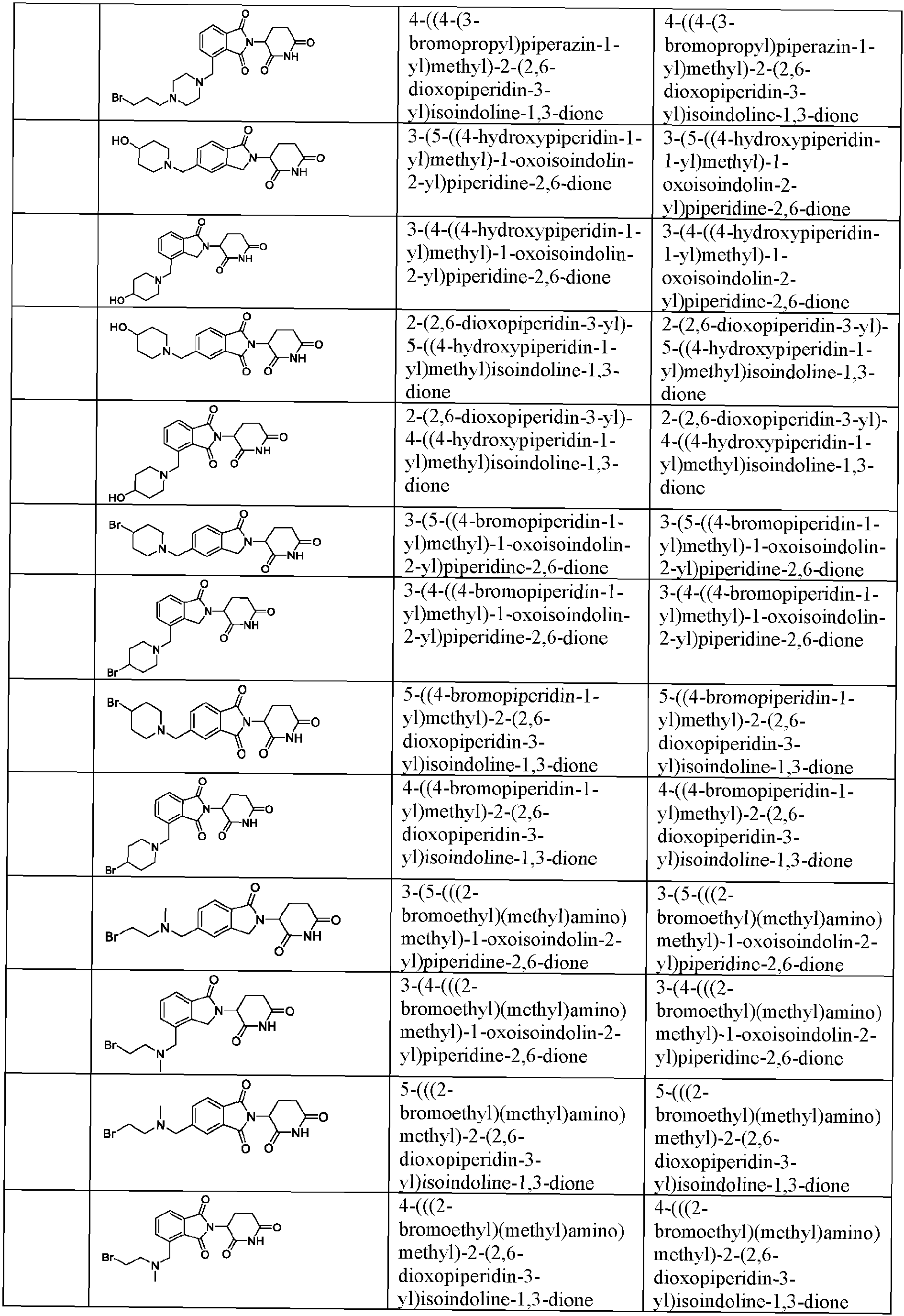

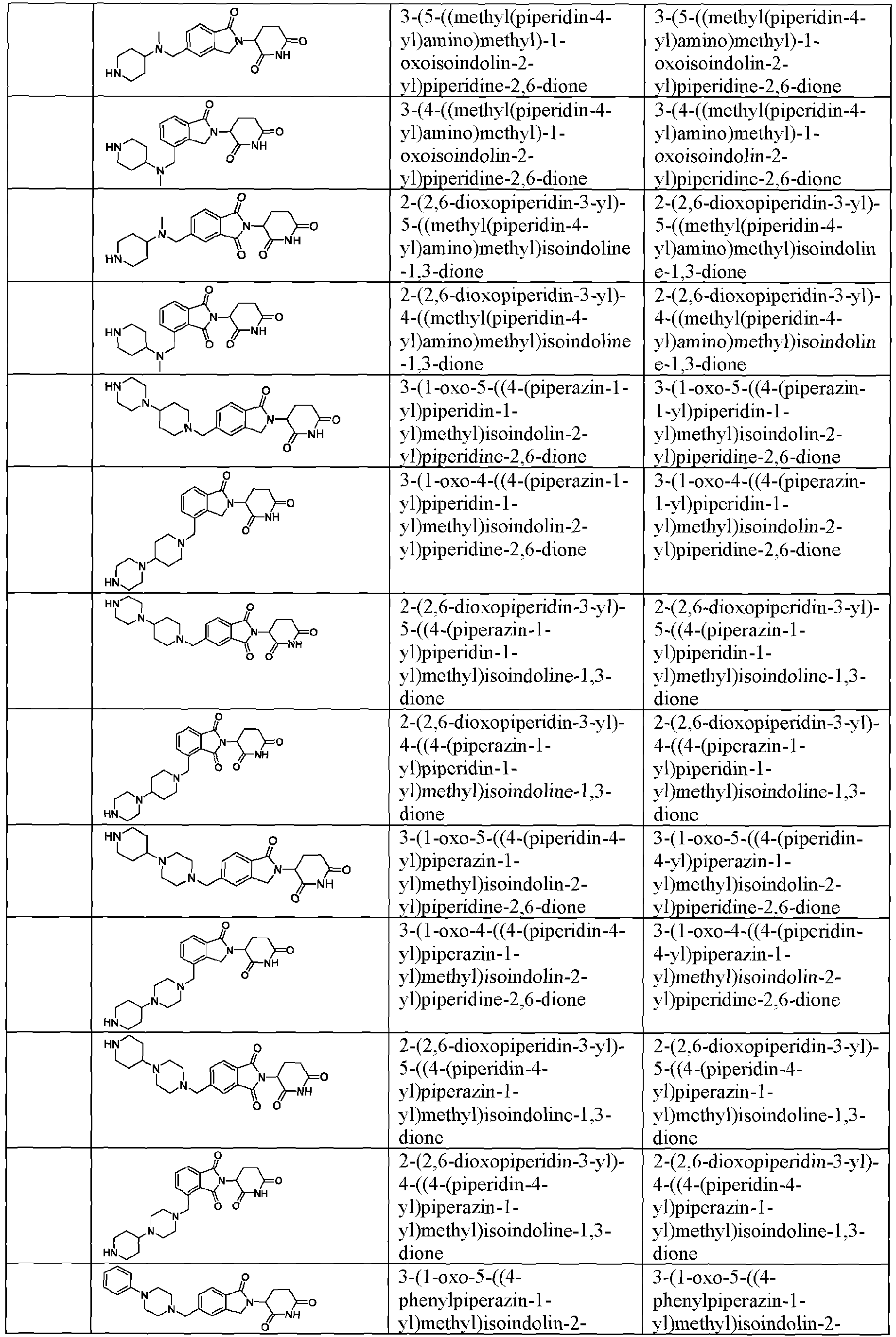

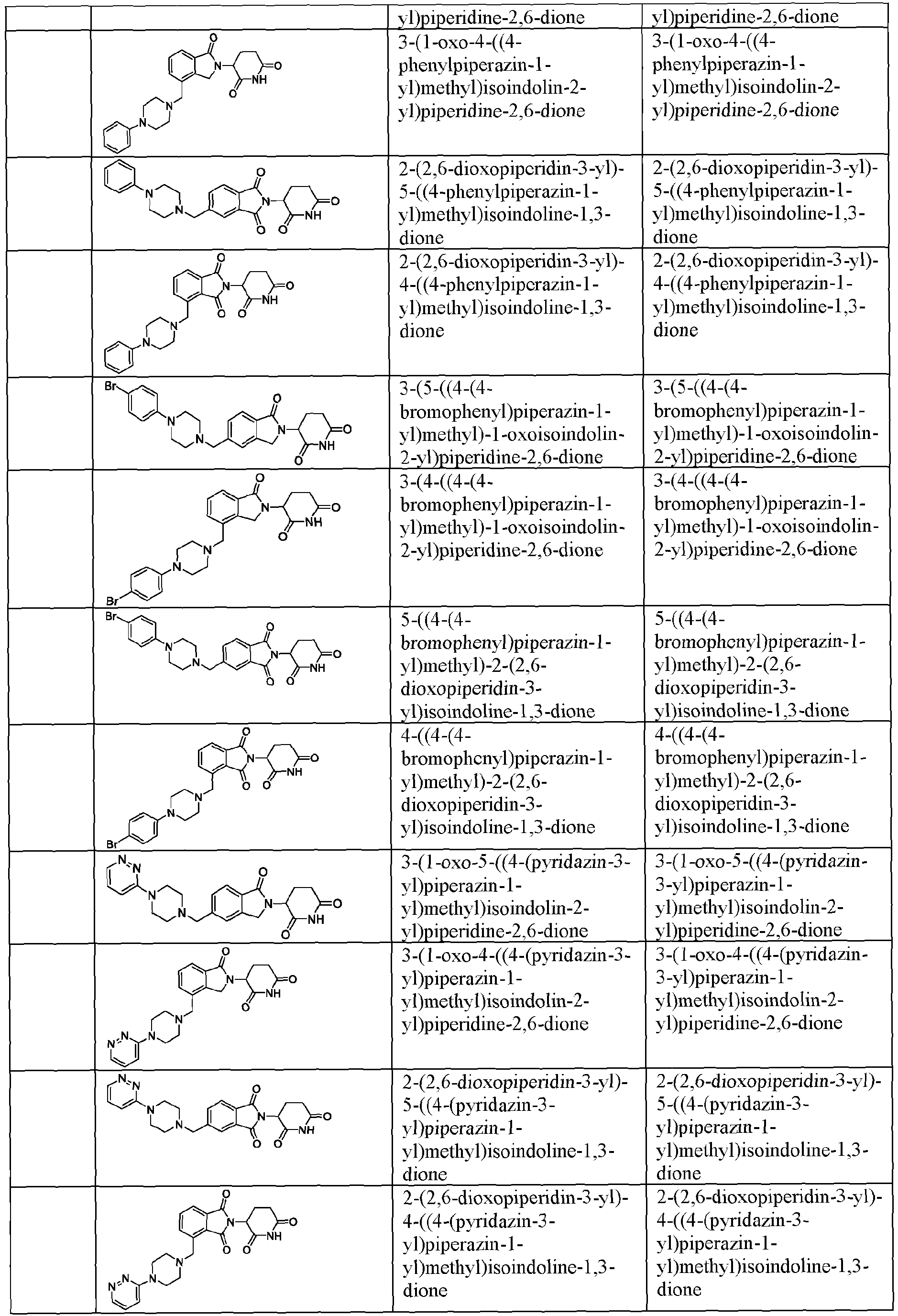

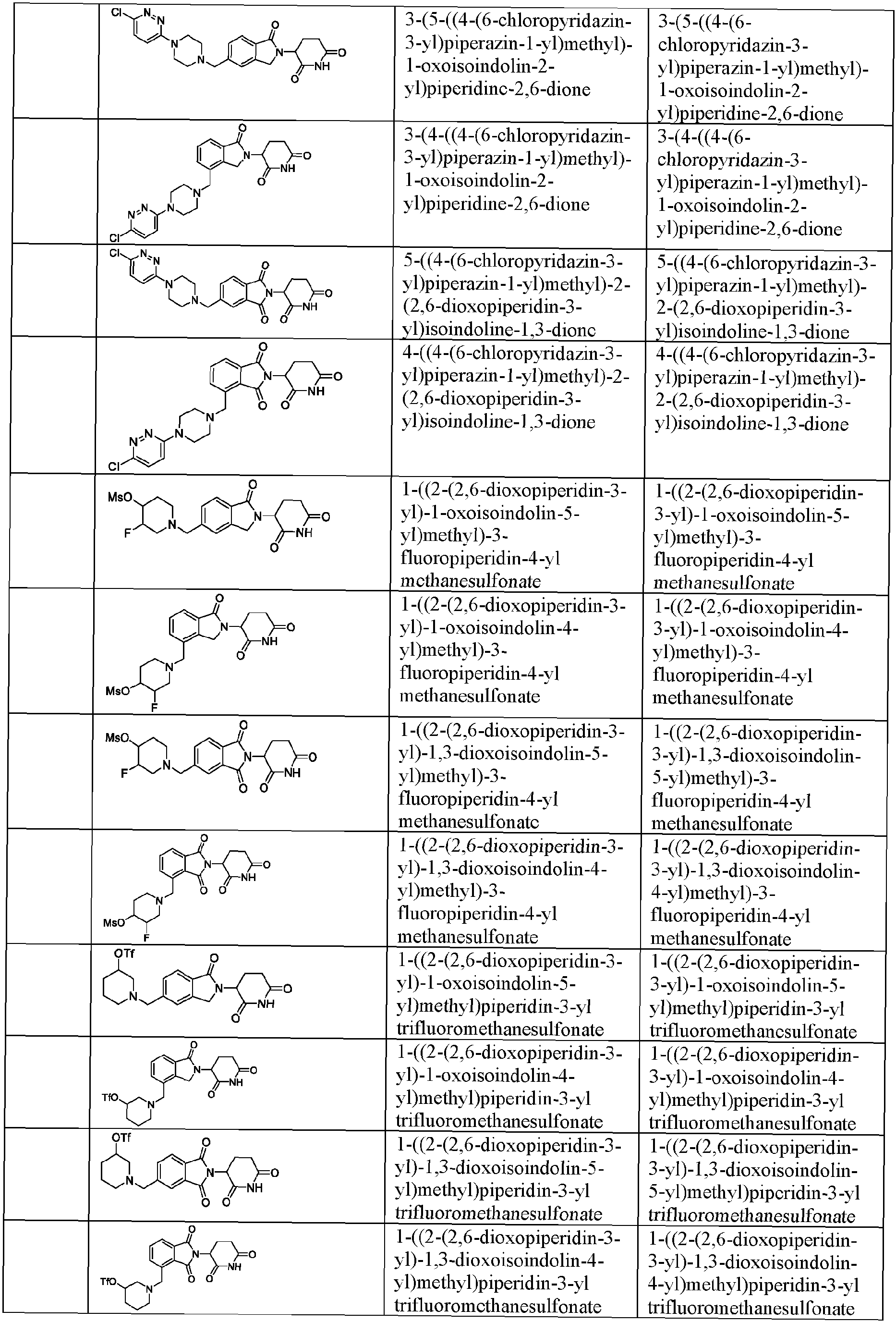

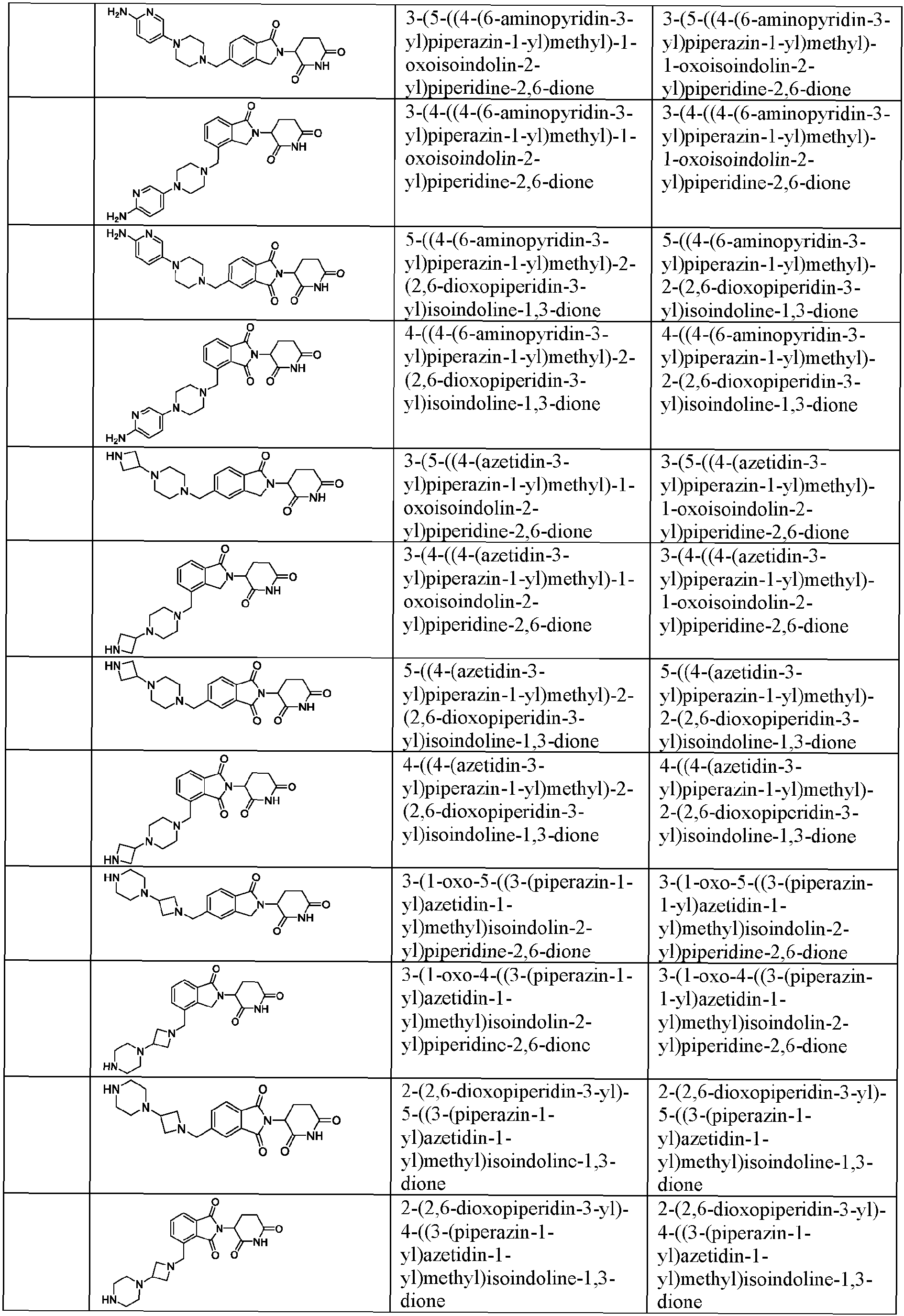

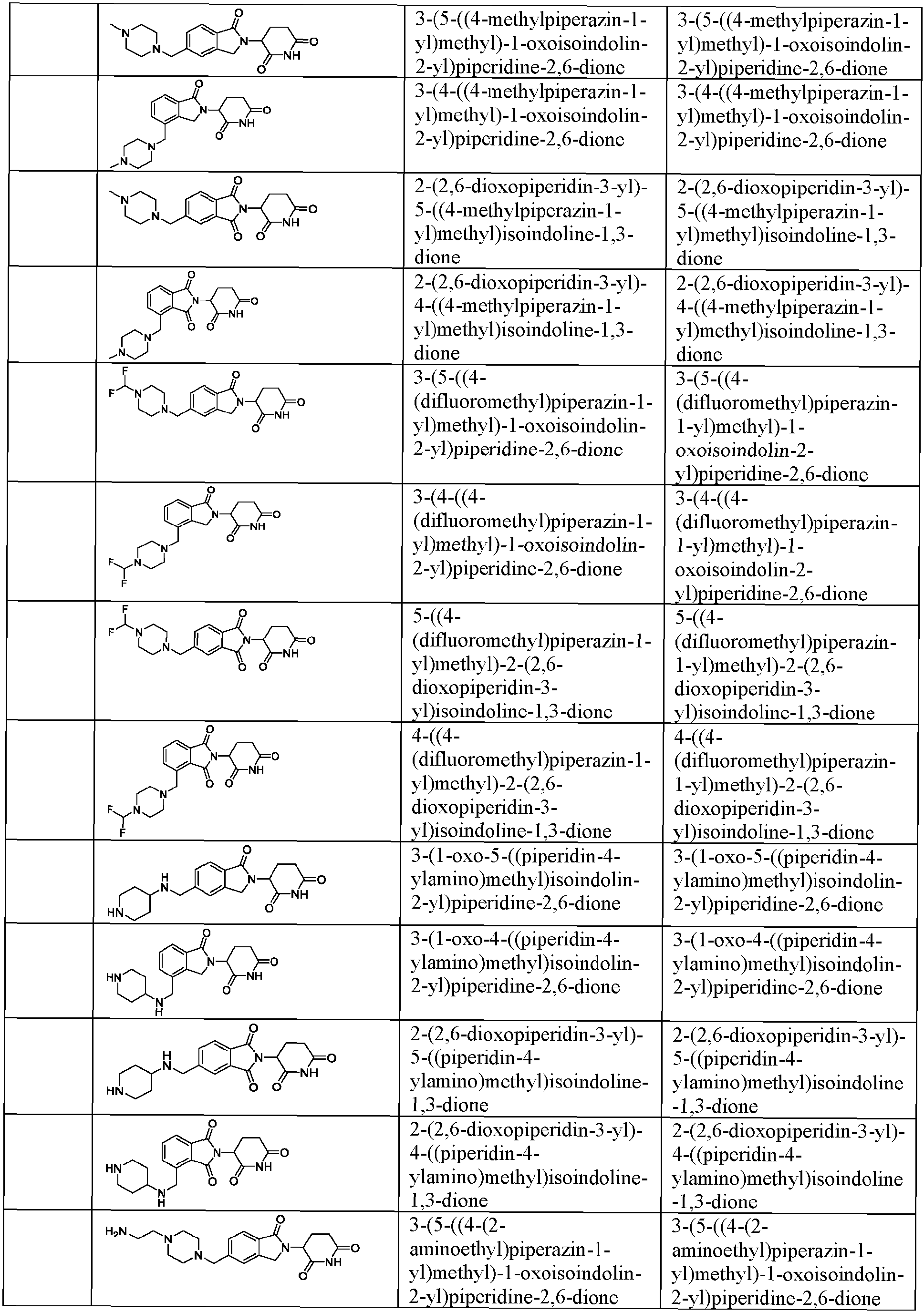

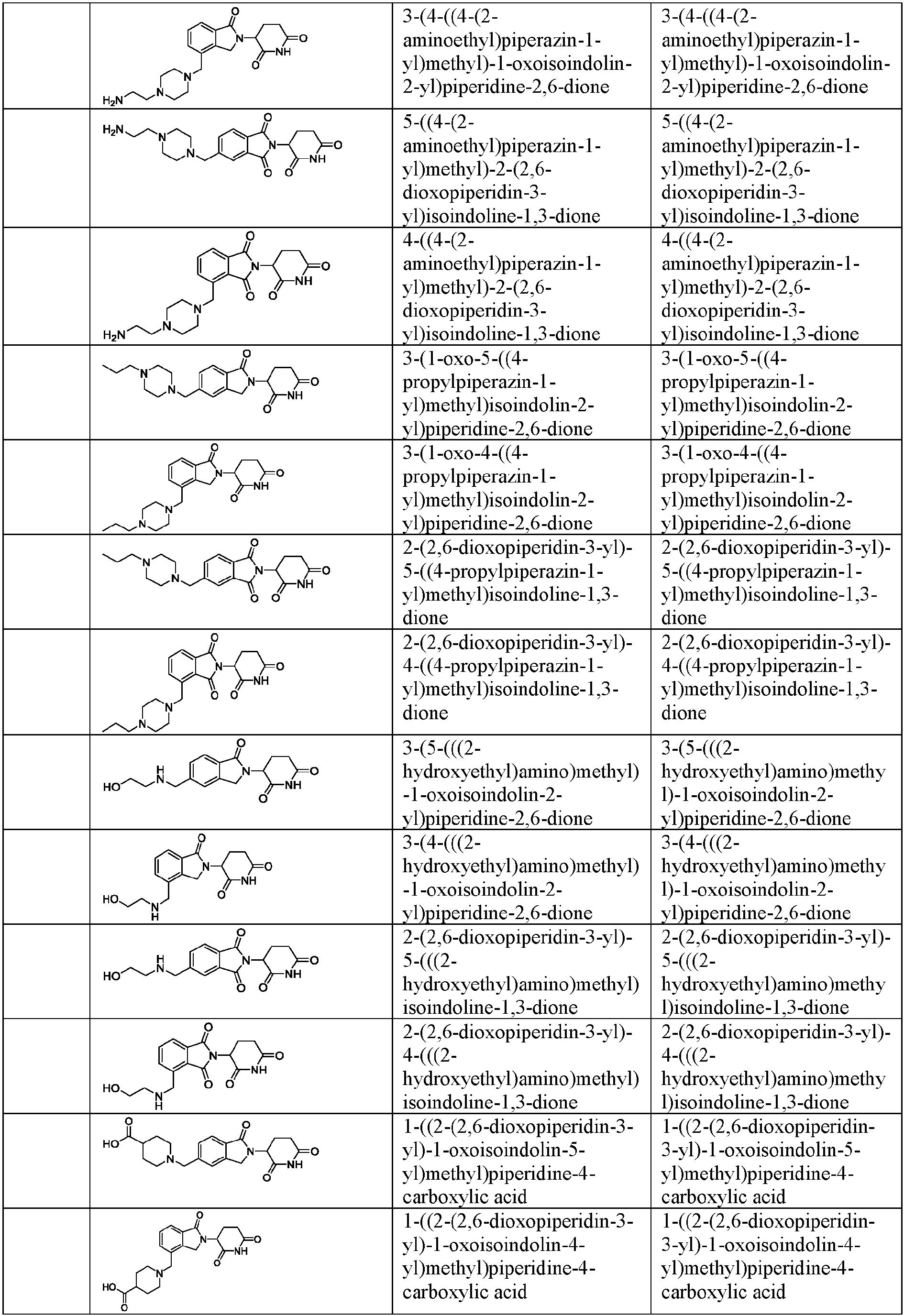

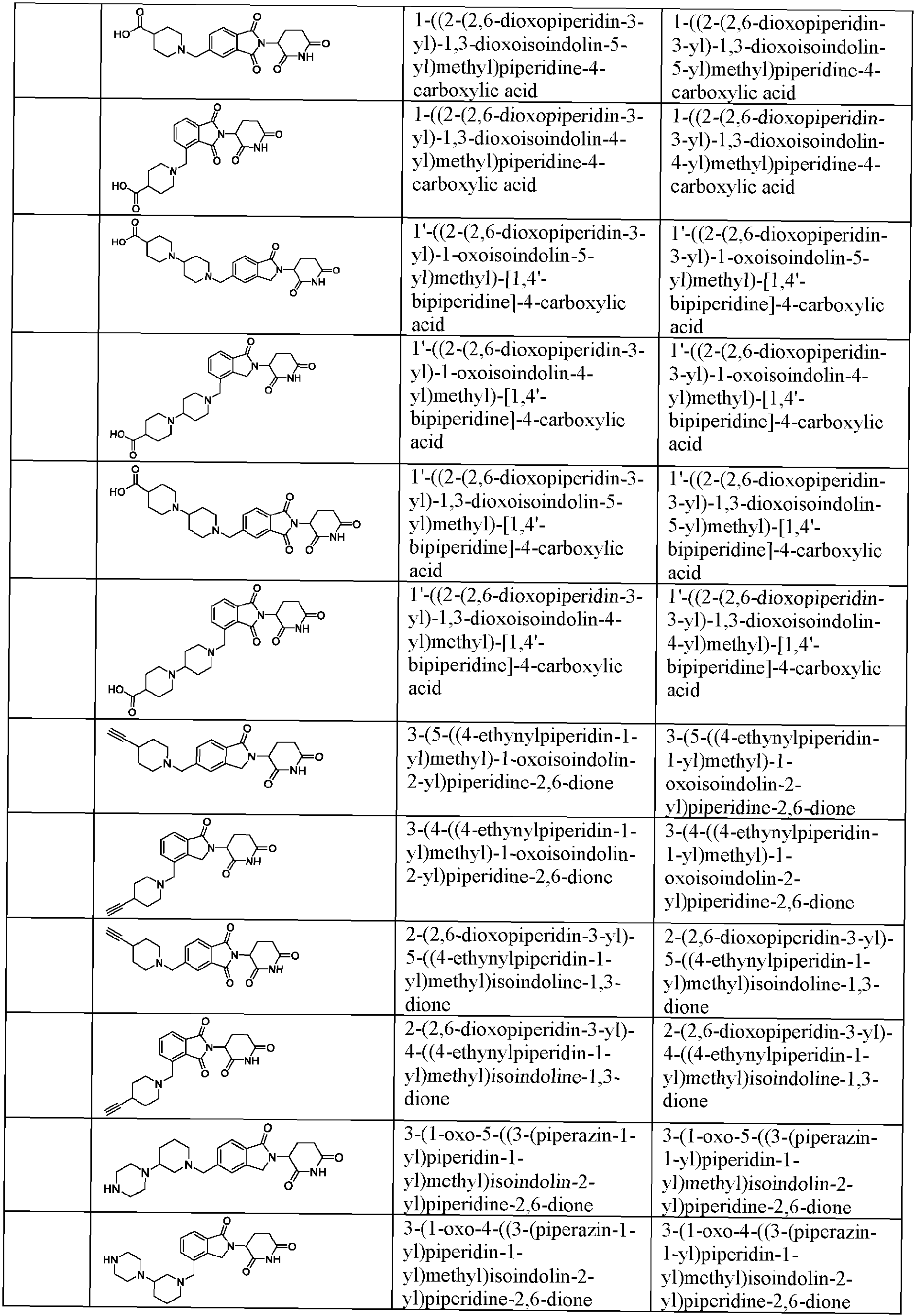

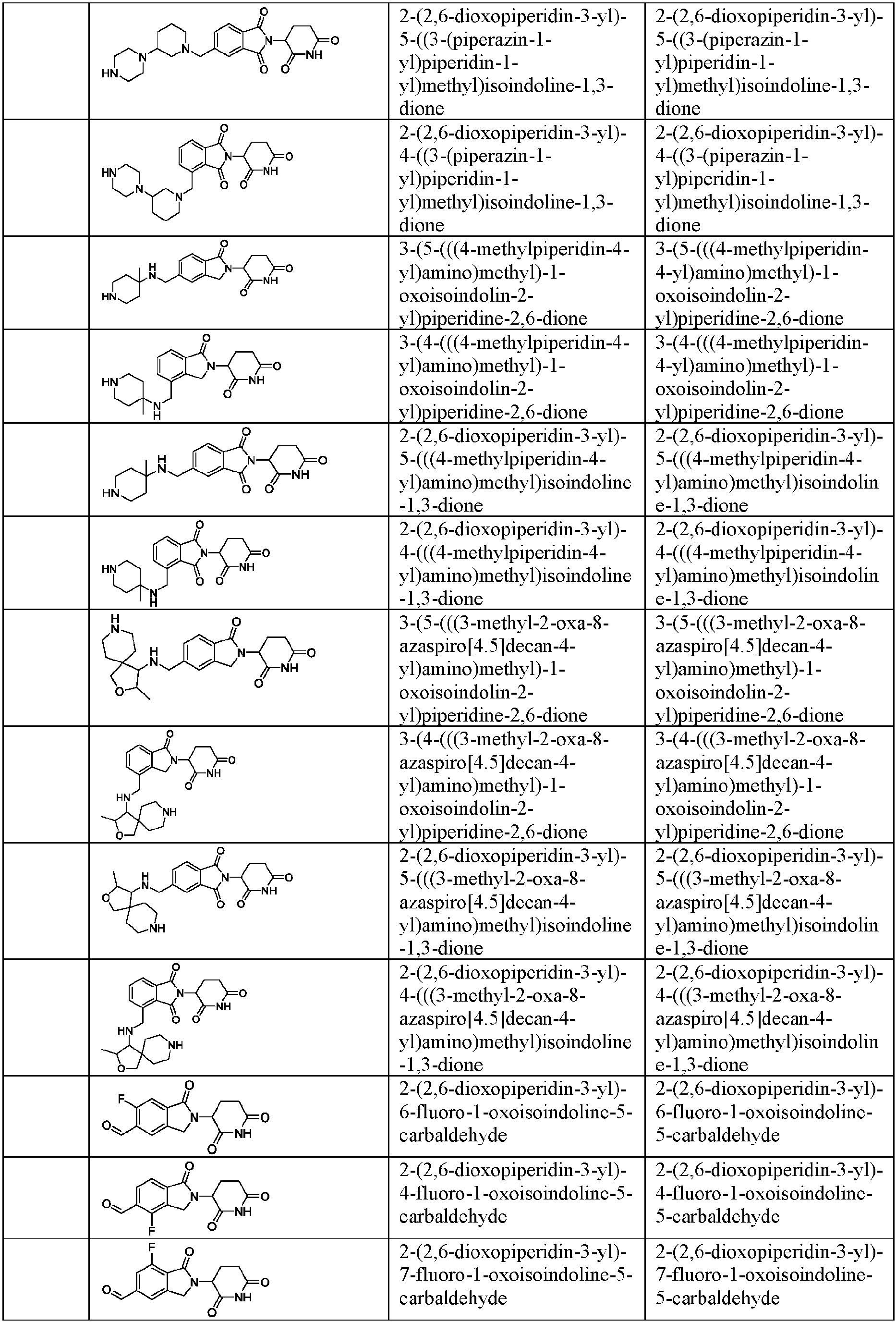

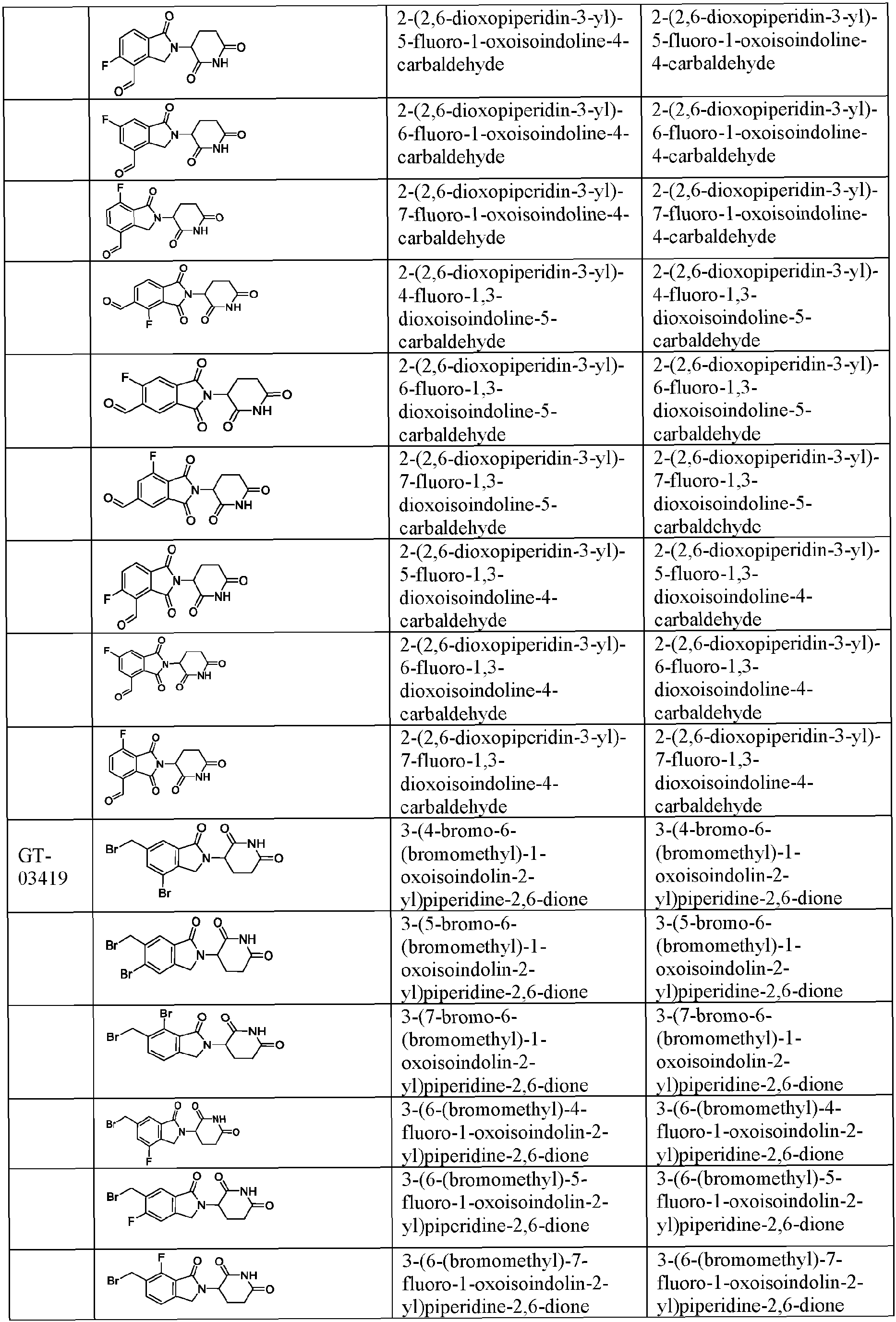

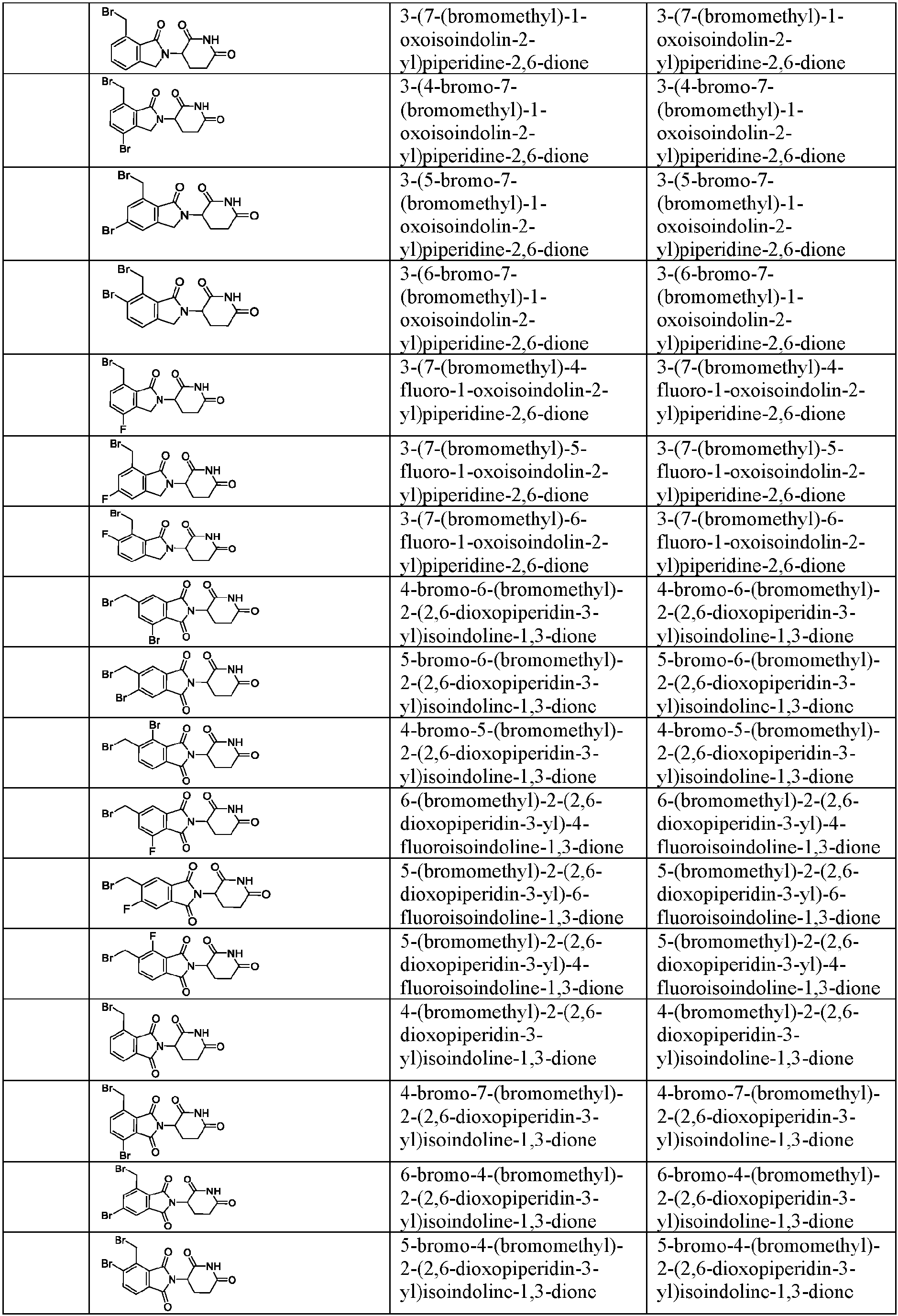

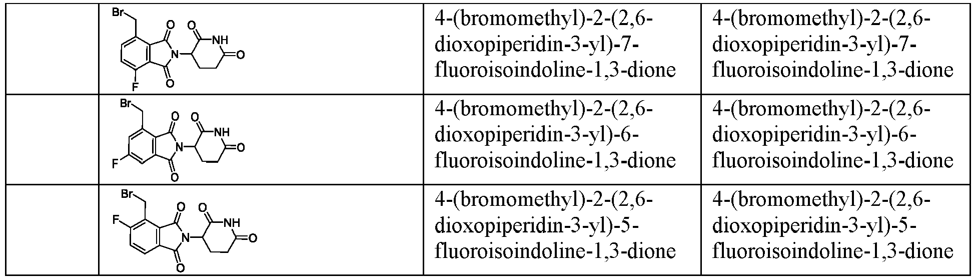

### Compounds of Formula (II)

The present disclosure provides a compound of Formula (II) or salts (including pharmaceutically acceptable salts), stereoisomers (including enantiomers), solvates, isotopically enriched analogs, prodrugs, or polymorphs thereof:

PBM-R_{f}-LIN-ULM Formula (II)

wherein PBM, R_{f}, LIN and ULM are as defined in the compounds of Formula (II) above.

The compound of Formula (II) of the present disclosure does not comprise the following compounds:
3 -(4-((3 -(4-amino-3 -(4-phenoxyphenyl)-1H-pyrazolo[3,4-*d*]pyrimidin-1-yl)piperidin-1 - yl)methyl)-1-oxoisoindolin-2-yl)piperidine-2,6-dione;
N-(5-(3,5-difluorobenzyl)-1H-indazol-3-yl)-4-(4-(1-((2-(2,6-dioxopiperidin-3-yl)-1-oxoisoindolin-5-yl)methyl)piperidin-3-yl)piperazin-1-yl)-2-((tetrahydro-2H-pyran-4-yl)amino)benzamide;
N-(5-(3,5-difluorobenzyl)-1H-indazol-3-yl)-4-(4-((2-(2,6-dioxopiperidin-3-yl)-1,3-dioxoisoindolin-5-yl)methyl)piperazin-1-yl)-2-((tetrahydro-2H-pyran-4-yl)amino)benzamide;
N-(5-(3,5-difluorobenzyl)-1H-indazol-3-yl)-4-(4-(1-((2-(2,6-dioxopiperidin-3-yl)-3-oxoisoindolin-5-yl)methyl)piperidin-3-yl)piperazin-1-yl)-2-((tetrahydro-2H-pyran-4-yl)amino)benzamide;
N-(5-(3,5-difluorobenzyl)-1H-indazol-3-yl)-4-(4-(1-((2-(2,6-dioxopiperidin-3-yl)-1,3-dioxoisoindolin-5-yl)methyl)piperidin-4-yl)piperazin-1-yl)-2-((tetrahydro-2H-pyran-4-yl)amino)benzamide;
N-(5-(3,5-difluorobenzyl)-1H-indazol-3-yl)-4-(4-(1-((2-(2,6-dioxopiperidin-3-yl)-1,3-dioxoisoindolin-5-yl)methyl)pyrrolidin-3-yl)piperazin-1-yl)-2-((tetrahydro-2H-pyran-4-yl)amino)benzamide;
N-(5-(3,5-difluorobenzyl)-1H-indazol-3-yl)-4-(4-(1-((2-(2,6-dioxopiperidin-3-yl)-1,3-dioxoisoindolin-5-yl)methyl)piperidin-3-yl)piperazin-1-yl)-2-((tetrahydro-2H-pyran-4-yl)amino)benzamide;
N-(5-(3,5-difluorobenzyl)-1H-indazol-3-yl)-4-(4-(1-((2-(2,6-dioxopiperidin-3-yl)-1,3-dioxoisoindolin-5-yl)methyl)azetidin-3-yl)piperazin-1-yl)-2-((tetrahydro-2H-pyran-4-yl)amino)benzamide;
2-(2,6-dioxopiperidin-3-yl)-5-((4-(6-(6-(2-(3-fluorophenyl)pyrrolidin-l-yl)imidazo[1,2-b]pyridazin-3-yl)pyridin-2-yl)piperazin-1-yl)methyl)isoindoline-1,3-dione; and
2-(2,6-dioxopiperidin-3-yl)-5-((3-(4-(6-(6-(2-(3-fluorophenyl)pyrrolidin-1-yl)imidazo[1,2-b]pyridazin-3 -yl)pyridin-2-yl)piperazin-1-yl)azetidin-1-yl)methyl)isoindoline-1,3 -dione.

The compound of Formula (II) or salts (including pharmaceutically acceptable salts), stereoisomers (including enantiomers), solvates, isotope enriched analogues, prodrugs, or polymorphs thereof of the present disclosure can bind to target proteins, recruit target proteins to E3 ubiquitin ligases for ubiquitination labeling and degradation. The compound of Formula (II) of the present disclosure can specifically target specific type or family of target protein, and exhibit a wide range of pharmacological activities by degrading/inhibiting diverse types or families of target proteins.

In some embodiments, specific target protein includes, but is not limited to, the target proteins in Table 2:

**Table 2. Applicable specific target proteins**

| abbreviation of target protein | target protein's name | Chinese translation of target protein's name |
|---|---|---|
| Cas9 | CRISPR-associated endonuclease Cas9 | CRISPR-associated endonuclease Cas9 |
| SHP2 | Src homology 2 domain containing protein tyrosine phosphatase | Src homology 2 domain containing protein tyrosine phosphatase |
| ER | Estrogen receptor | Estrogen receptor |
| AR | Androgen receptor | Androgen receptor |
| BTK | Bruton tyrosine kinase | Bruton tyrosine kinase |
| IRAK4 | Interleukin-1 receptor-associated kinase 4 | Interleukin-1 receptor-associated kinase 4 |
| EGFR | Epidermal growth factor receptor | Epidermal growth factor receptor |
| MET | Hepatocyte growth factor receptor | Hepatocyte growth factor receptor |
| KIT | Mast/stem cell growth factor receptor Kit | Mast/stem cell growth factor receptor Kit |
| EPHA2 | Ephrin type-A receptor 2 | Ephrin type-A receptor 2 |
| PDE4D | cAMP-specific 3',5'-cyclic phosphodiesterase 4D | cAMP-specific 3',5'-cyclic phosphodiesterase 4D |
| SRC | Proto-oncogene tyrosine-protein kinase Src | Proto-oncogene tyrosine-protein kinase Src |
| BRAF | Serine/threonine-protein kinase B-raf | Serine/threonine-protein kinase B-raf |
| FGFR | (fibroblast growth factor receptors,FGFRs) | (fibroblast growth factor receptors,FGFRs) |
| FGFR2 | Fibroblast growth factor receptor 2 | Fibroblast growth factor receptor 2 |
| FGFR1 | Fibroblast growth factor receptor 1 | Fibroblast growth factor receptor 1 |
| LYN | Tyrosine-protein kinase Lyn | Tyrosine-protein kinase Lyn |
| ITK | Tyrosine-protein kinase ITK/TSK | Tyrosine-protein kinase ITK/TSK |
| PARP1 | Poly [ADP-ribose] polymerase 1 | Poly [ADP-ribose] polymerase 1 |
| HDAC2 | Histone deacetylase 2 | Histone deacetylase 2 |
| HDAC3 | Histone deacetylase 3 | Histone deacetylase 3 |
| JAK1 | Tyrosine-protein kinase JAK1 | Tyrosine-protein kinase JAK1 |
| BCL2 | Apoptosis regulator Bcl-2 | Apoptosis regulator Bcl-2 |
| HDAC6 | Histone deacetylase 6 | Histone deacetylase 6 |
| CRBN | Protein cereblon | Protein cereblon |
| EPHB2 | Ephrin type-B receptor 2 | Ephrin type-B receptor 2 |
| BLK | Tyrosine-protein kinase Blk | Tyrosine-protein kinase Blk |
| HDAC1 | Histone deacetylase 1 | Histone deacetylase 1 |
| IGF1R | Insulin-like growth factor 1 receptor | Insulin-like growth factor 1 receptor |
| TGFBR1 | TGF-beta receptor type-1 | TGF-beta receptor type-1 |
| AKT2 | RAC-beta serine/threonine-protein kinase | RAC-beta serine/threonine-protein kinase |
| AKT1 | RAC-alpha serine/threonine-protein kinase | RAC-alpha serine/threonine-protein kinase |
| FAK | Focal adhesion kinase | Focal adhesion kinase |
| MAPK1 | Mitogen-activated protein kinase 1 | Mitogen-activated protein kinase 1 |
| MAPK14 | Mitogen-activated protein kinase 14 | Mitogen-activated protein kinase 14 |
| CDK9 | Cyclin-dependent kinase 9 | Cyclin-dependent kinase 9 |
| MCL1 | Induced myeloid leukemia cell differentiation protein Mcl-1 | Induced myeloid leukemia cell differentiation protein Mcl-1 |
| BET | Bromodomain and extra-terminal domain protein | Bromodomain and extra-terminal domain protein |
| BRD4 | Bromodomain-containing protein 4 | Bromodomain-containing protein 4 |
| BRD3 | Bromodomain-containing protein 3 | Bromodomain-containing protein 3 |
| CDK13 | Cyclin-dependent kinase 13 | Cyclin-dependent kinase 13 |
| BCL2L1 | Bcl-2-like protein 1 | Bcl-2-like protein 1 |
| CDK12 | Cyclin-dependent kinase 12 | Cyclin-dependent kinase 12 |
| CDK1 | Cyclin-dependent kinase 1 | Cyclin-dependent kinase 1 |
| AKT3 | RAC-gamma serine/threonine-protein kinase | RAC-gamma serine/threonine-protein kinase |
| CDK11B | Cyclin-dependent kinase 11B | Cyclin-dependent kinase 11B |
| PAK4 | Serine/threonine-protein kinase PAK 4 | Serine/threonine-protein kinase PAK 4 |
| MAPKAPK2 | MAP kinase-activated protein kinase 2 | MAP kinase-activated protein kinase 2 |
| TNK2 | Activated CDC42 kinase 1 | Activated CDC42 kinase 1 |
| SIRT2 | NAD-dependent protein deacetylase sirtuin-2 | NAD-dependent protein deacetylase sirtuin-2 |
| DAPK1 | Death-associated protein kinase 1 | Death-associated protein kinase 1 |
| ABL2 | Tyrosine-protein kinase ABL2 | Tyrosine-protein kinase ABL2 |
| PRKAA2 | 5'-AMP-activated protein kinase catalytic subunit alpha-2 | 5'-AMP-activated protein kinase catalytic subunit alpha-2 |
| KAT2A | Histone acetyltransferase KAT2A | Histone acetyltransferase KAT2A |
| PBRM1 | Protein polybromo-1 | Protein polybromo-1 |
| EIF2AK2 | Interferon-induced, double-stranded RNA-activated protein kinase | Interferon-induced, double-stranded RNA-activated protein kinase |
| MAP3K7 | Mitogen-activated protein kinase kinase kinase 7 | Mitogen-activated protein kinase kinase kinase 7 |
| MAPT | Microtubule-associated protein tau | Microtubule-associated protein tau |
| RIPK1 | Receptor-interacting serine/threonine-protein kinase 1 | Receptor-interacting serine/threonine-protein kinase 1 |
| IRAK1 | Interleukin-1 receptor-associated kinase 1 | Interleukin-1 receptor-associated kinase 1 |
| MAP4K1 | Mitogen-activated protein kinase kinase kinase kinase 1 | Mitogen-activated protein kinase kinase kinase kinase 1 |
| MARK4 | MAP/microtubule affinity-regulating kinase 4 | MAP/microtubule affinity-regulating kinase 4 |
| BRD9 | Bromodomain-containing protein 9 | Bromodomain-containing protein 9 |
| RIPK2 | Receptor-interacting serine/threonine-protein kinase 2 | Receptor-interacting serine/threonine-protein kinase 2 |
| LIMK1 | LIM domain kinase 1 | LIM domain kinase 1 |
| STK38 | Serine/threonine-protein kinase 38 | Serine/threonine-protein kinase 38 |
| TRIM24 | Transcription intermediary factor 1-alpha | Transcription intermediary factor 1-alpha |
| SMARCA4 | Transcription activator BRG1 | Transcription activator BRG1 |
| PRKAA1 | 5'-AMP-activated protein kinase catalytic subunit alpha-1 | 5'-AMP-activated protein kinase catalytic subunit alpha-1 |
| TBK1 | Serine/threonine-protein kinase TBK1 | Serine/threonine-protein kinase TBK1 |
| KRAS | GTPase KRas | GTPase KRas |
| SMARCA2 | Probable global transcription activator SNF2L2 | Probable global transcription activator SNF2L2 |
| PCNA | Proliferating cell nuclear antigen | Proliferating cell nuclear antigen |
| BRD7 | Bromodomain-containing protein 7 | Bromodomain-containing protein 7 |
| SUZ12 | Polycomb protein SUZ12 | Polycomb protein SUZ12 |
| IKZF1 | DNA-binding protein Ikaros | DNA-binding protein Ikaros |
| HTT | Huntingtin | Huntingtin |
| SNCA | Alpha-synuclein | Alpha-synuclein |
| SLC9A1 | Sodium/hydrogen exchanger 1 | Sodium/hydrogen exchanger 1 |
| FER | Tyrosine-protein kinase Fer | Tyrosine-protein kinase Fer |
| MAP4K2 | Mitogen-activated protein kinase kinase kinase kinase 2 | Mitogen-activated protein kinase kinase kinase kinase 2 |
| DLG4 | Disks large homolog 4 | Disks large homolog 4 |
| IKZF3 | Zinc finger protein Aiolos | Zinc finger protein Aiolos |
| PDE4A | cAMP-specific 3',5'-cyclic phosphodiesterase 4A | cAMP-specific 3',5'-cyclic phosphodiesterase 4A |
| HMGCR | 3-hydroxy-3-methylglutaryl-coenzyme A reductase | 3-hydroxy-3-methylglutaryl-coenzyme A reductase |
| ABL1 | Tyrosine-protein kinase ABL1 | Tyrosine-protein kinase ABL1 |
| RARA | Retinoic acid receptor alpha | Retinoic acid receptor alpha |
| FLT3 | Receptor-type tyrosine-protein kinase FLT3 | Receptor-type tyrosine-protein kinase FLT3 |
| RARG | Retinoic acid receptor gamma | Retinoic acid receptor gamma |
| VEGFR-1 | Vascular endothelial growth factor receptor 1 | Vascular endothelial growth factor receptor 1 |
| EZH2 | Histone-lysine N-methyltransferase EZH2 | Histone-lysine N-methyltransferase EZH2 |
| EPHA1 | Ephrin type-A receptor 1 | Ephrin type-A receptor 1 |
| AURKA | Aurora kinase A | Aurora kinase A |
| CHEK1 | Serine/threonine-protein kinase Chk1 | Serine/threonine-protein kinase Chk1 |
| WEE1 | Weel-like protein kinase | Weel-like protein kinase |
| PLK1 | Serine/threonine-protein kinase PLK1 | Serine/threonine-protein kinase PLK1 |
| CDC7 | Cell division cycle 7-related protein kinase | Cell division cycle 7-related protein kinase |
| AURKB | Aurora kinase B | Aurora kinase B |
| PLK4 | Serine/threonine-protein kinase PLK4 | Serine/threonine-protein kinase PLK4 |
| MDM2 | E3 ubiquitin-protein ligase Mdm2 | E3 ubiquitin-protein ligase Mdm2 |
| MAPK6 | Mitogen-activated protein kinase 6 | Mitogen-activated protein kinase 6 |
| BUB1 | Mitotic checkpoint serine/threonine-protein kinase BUB1 | Mitotic checkpoint serine/threonine-protein kinase BUB1 |
| ESRRA | Steroid hormone receptor ERR1 | Steroid hormone receptor ERR1 |
| BCL6 | B-cell lymphoma 6 protein | B-cell lymphoma 6 protein |
| MAPK12 | Mitogen-activated protein kinase 12 | Mitogen-activated protein kinase 12 |
| CRABP2 | Cellular retinoic acid-binding protein 2 | Cellular retinoic acid-binding protein 2 |
| HIPK1 | Homeodomain-interacting protein kinase 1 | Homeodomain-interacting protein kinase 1 |
| ADRM1 | Proteasomal ubiquitin receptor ADRM1 | Proteasomal ubiquitin receptor ADRM1 |
| CDC20 | Cell division cycle protein 20 homolog | Cell division cycle protein 20 homolog |
| BUB1B | Mitotic checkpoint serine/threonine-protein kinase BUB1 beta | Mitotic checkpoint serine/threonine-protein kinase BUB1 beta |
| NTRK2 | BDNF/NT-3 growth factors receptor | BDNF/NT-3 growth factors receptor |
| NTRK1 | High affinity nerve growth factor receptor | High affinity nerve growth factor receptor |
| CD274 | Programmed cell death 1 ligand 1 | Programmed cell death 1 ligand 1 |
| NUAK1 | NUAK family SNF1-like kinase 1 | NUAK family SNF1-like kinase 1 |
| PDCD1 | Programmed cell death protein 1 | Programmed cell death protein 1 |
| ERBB2 | Receptor tyrosine-protein kinase erbB-2 | Receptor tyrosine-protein kinase erbB-2 |
| EPHA3 | Ephrin type-A receptor 3 | Ephrin type-A receptor 3 |
| PIK3CG | Phosphatidylinositol 4,5-bisphosphate 3-kinase catalytic subunit gamma isoform | Phosphatidylinositol 4,5-bisphosphate 3-kinase catalytic subunit gamma isoform |
| MAP2K1 | Dual specificity mitogen-activated protein kinase kinase 1 | Dual specificity mitogen-activated protein kinase kinase 1 |
| LCK | Tyrosine-protein kinase Lck | Tyrosine-protein kinase Lck |
| MAP2K2 | Dual specificity mitogen-activated protein kinase kinase 2 | Dual specificity mitogen-activated protein kinase kinase 2 |
| JAK3 | Tyrosine-protein kinase JAK3 | Tyrosine-protein kinase JAK3 |
| GSK3B | Glycogen synthase kinase-3 beta | Glycogen synthase kinase-3 beta |
| JAK2 | Tyrosine-protein kinase JAK2 | Tyrosine-protein kinase JAK2 |
| PRKCI | Protein kinase C iota type | Protein kinase C iota type |
| FKBP1A | Peptidyl-prolyl cis-trans isomerase FKBP1A | Peptidyl-prolyl cis-trans isomerase FKBP1A |
| DHODH | Dihydroorotate dehydrogenase | Dihydroorotate dehydrogenase |
| PIK3CA | Phosphatidylinositol 4,5-bisphosphate 3-kinase catalytic subunit alpha isoform | Phosphatidylinositol 4,5-bisphosphate 3-kinase catalytic subunit alpha isoform |
| CDK6 | Cyclin-dependent kinase 6 | Cyclin-dependent kinase 6 |
| CDK4 | Cyclin-dependent kinase 4 | Cyclin-dependent kinase 4 |
| PDE4B | cAMP-specific 3',5'-cyclic phosphodiesterase 4B | cAMP-specific 3',5'-cyclic phosphodiesterase 4B |
| FYN | Tyrosine-protein kinase Fyn | Tyrosine-protein kinase Fyn |
| TYK2 | Non-receptor tyrosine-protein kinase TYK2 | Non-receptor tyrosine-protein kinase TYK2 |
| PDK1 | Pyruvate dehydrogenase 1 | Pyruvate dehydrogenase 1 |
| PDK2 | Pyruvate dehydrogenase 2 | Pyruvate dehydrogenase 2 |
| PDK3 | Pyruvate dehydrogenase 3 | Pyruvate dehydrogenase 3 |
| YES1 | Tyrosine-protein kinase Yes | Tyrosine-protein kinase Yes |
| GSK3A | Glycogen synthase kinase-3 alpha | Glycogen synthase kinase-3 alpha |
| CDK16 | Cyclin-dependent kinase 16 | Cyclin-dependent kinase 16 |
| CSNK2A1 | Casein kinase II subunit alpha | Casein kinase II subunit alpha |
| CDK7 | Cyclin-dependent kinase 7 | Cyclin-dependent kinase 7 |
| PTK2B | Protein-tyrosine kinase 2-beta | Protein-tyrosine kinase 2-beta |
| MAPK10 | Mitogen-activated protein kinase 10 | Mitogen-activated protein kinase 10 |
| MAPK9 | Mitogen-activated protein kinase 9 | Mitogen-activated protein kinase 9 |
| MAPK8 | Mitogen-activated protein kinase 8 | Mitogen-activated protein kinase 8 |
| CDK5 | Cyclin-dependent-like kinase 5 | Cyclin-dependent-like kinase 5 |
| METAP2 | Methionine aminopeptidase 2 | Methionine aminopeptidase 2 |
| BRD2 | Bromodomain-containing protein 2 | Bromodomain-containing protein 2 |
| MAPK3 | Mitogen-activated protein kinase 3 | Mitogen-activated protein kinase 3 |
| TEC | Tyrosine-protein kinase Tec | Tyrosine-protein kinase Tec |
| CDK14 | Cyclin-dependent kinase 14 | Cyclin-dependent kinase 14 |
| CDK17 | Cyclin-dependent kinase 17 | Cyclin-dependent kinase 17 |
| MAP3K11 | Mitogen-activated protein kinase kinase kinase 11 | Mitogen-activated protein kinase kinase kinase 11 |
| RPS6KB1 | Ribosomal protein S6 kinase beta-1 | Ribosomal protein S6 kinase beta-1 |
| CSK | Tyrosine-protein kinase CSK | Tyrosine-protein kinase CSK |
| MERTK | Tyrosine-protein kinase Mer | Tyrosine-protein kinase Mer |
| STK17B | Serine/threonine-protein kinase 17B | Serine/threonine-protein kinase 17B |
| CSNK2A2 | Casein kinase II subunit alpha' | Casein kinase II subunit alpha' |
| RPS6KA1 | Ribosomal protein S6 kinase alpha-1 | Ribosomal protein S6 kinase alpha-1 |
| MAPK13 | Mitogen-activated protein kinase 13 | Mitogen-activated protein kinase 13 |
| GAK | Cyclin-G-associated kinase | Cyclin-G-associated kinase |
| CLK1 | Dual specificity protein kinase CLK1 | Dual specificity protein kinase CLK1 |
| STK4 | Serine/threonine-protein kinase 4 | Serine/threonine-protein kinase 4 |
| EIF4E | Eukaryotic translation initiation factor 4E | Eukaryotic translation initiation factor 4E |
| STK10 | Serine/threonine-protein kinase 10 | Serine/threonine-protein kinase 10 |
| LRRK2 | Leucine-rich repeat serine/threonine-protein kinase 2 | Leucine-rich repeat serine/threonine-protein kinase 2 |
| TAOK3 | Serine/threonine-protein kinase TAO3 | Serine/threonine-protein kinase TAO3 |
| MARK2 | Serine/threonine-protein kinase MARK2 | Serine/threonine-protein kinase MARK2 |
| CSNK1D | Casein kinase I isoform delta | Casein kinase I isoform delta |
| AAK1 | AP2-associated protein kinase 1 | AP2-associated protein kinase 1 |
| IRAK3 | Interleukin-1 receptor-associated kinase 3 | Interleukin-1 receptor-associated kinase 3 |
| STAT3 | Signal transducer and activator of transcription 3 | Signal transducer and activator of transcription 3 |
| CAMKK1 | Calcium/calmodulin-dependent protein kinase kinase 1 | Calcium/calmodulin-dependent protein kinase kinase 1 |
| EED | Polycomb protein EED | Polycomb protein EED |
| CSNK1A1 | Casein kinase I isoform alpha | Casein kinase I isoform alpha |
| NEK1 | Serine/threonine-protein kinase Nek1 | Serine/threonine-protein kinase Nek1 |
| BMP2K | BMP-2-inducible protein kinase | BMP-2-inducible protein kinase |
| MAPK7 | Mitogen-activated protein kinase 7 | Mitogen-activated protein kinase 7 |
| ULK1 | Serine/threonine-protein kinase ULK1 | Serine/threonine-protein kinase ULK1 |
| RPS6KA3 | Ribosomal protein S6 kinase alpha-3 | Ribosomal protein S6 kinase alpha-3 |
| PTPN11 | Tyrosine-protein phosphatase non-receptor type 11 | Tyrosine-protein phosphatase non-receptor type 11 |
| LIMK2 | LIM domain kinase 2 | LIM domain kinase 2 |
| CSNK1E | Casein kinase I isoform epsilon | Casein kinase I isoform epsilon |
| EIF2AK4 | eIF-2-alpha kinase GCN2 | eIF-2-alpha kinase GCN2 |
| MAP2K5 | Dual specificity mitogen-activated protein kinase kinase 5 | Dual specificity mitogen-activated protein kinase kinase 5 |
| MAP4K3 | Mitogen-activated protein kinase kinase kinase kinase 3 | Mitogen-activated protein kinase kinase kinase kinase 3 |
| VHL | von Hippel-Lindau disease tumor suppressor | von Hippel-Lindau disease tumor suppressor |
| MARK3 | MAP/microtubule affinity-regulating kinase 3 | MAP/microtubule affinity-regulating kinase 3 |
| TAOK2 | Serine/threonine-protein kinase TAO2 | Serine/threonine-protein kinase TAO2 |
| MAP4K5 | Mitogen-activated protein kinase kinase kinase kinase 5 | Mitogen-activated protein kinase kinase kinase kinase 5 |
| SNRK | SNF-related serine/threonine-protein kinase | SNF-related serine/threonine-protein kinase |
| EEF2K | Eukaryotic elongation factor 2 kinase | Eukaryotic elongation factor 2 kinase |
| SGK3 | Serine/threonine-protein kinase Sgk3 | Serine/threonine-protein kinase Sgk3 |
| AHR | Aryl hydrocarbon receptor | Aryl hydrocarbon receptor |
| NEK4 | Serine/threonine-protein kinase Nek4 | Serine/threonine-protein kinase Nek4 |
| NEK9 | Serine/threonine-protein kinase Nek9 | Serine/threonine-protein kinase Nek9 |
| CIT | Citron Rho-interacting kinase | Citron Rho-interacting kinase |
| LATS1 | Serine/threonine-protein kinase LATS1 | Serine/threonine-protein kinase LATS1 |
| MINK1 | Misshapen-like kinase 1 | Misshapen-like kinase 1 |
| SIK3 | Serine/threonine-protein kinase SIK3 | Serine/threonine-protein kinase SIK3 |
| RPS6KA4 | Ribosomal protein S6 kinase alpha-4 | Ribosomal protein S6 kinase alpha-4 |
| NEK3 | Serine/threonine-protein kinase Nek3 | Serine/threonine-protein kinase Nek3 |
| SIK2 | Serine/threonine-protein kinase SIK2 | Serine/threonine-protein kinase SIK2 |
| MAST3 | Microtubule-associated serine/threonine-protein kinase 3 | Microtubule-associated serine/threonine-protein kinase 3 |
| STK32C | Serine/threonine-protein kinase 32C | Serine/threonine-protein kinase 32C |
| ALK | ALK tyrosine kinase receptor | ALK tyrosine kinase receptor |
| ALK2 | (activin receptor-like kinase 2,ALK2) | (activin receptor-like kinase 2,ALK2) |
| EPHB4 | Ephrin type-B receptor 4 | Ephrin type-B receptor 4 |
| PARP2 | Poly (ADP-ribose) polymerase 2 | Poly (ADP-ribose) polymerase 2 |
| PTK6 | Protein-tyrosine kinase 6 | Protein-tyrosine kinase 6 |
| PARP3 | Protein mono-ADP-ribosyltransferase PARP3 | Protein mono-ADP-ribosyltransferase PARP3 |
| CDK2 | Cyclin-dependent kinase 2 | Cyclin-dependent kinase 2 |
| CDK8 | Cyclin-dependent kinase 8 | Cyclin-dependent kinase 8 |
| BRDT | Bromodomain testis-specific protein | Bromodomain testis-specific protein |
| MAPKAPK5 | MAP kinase-activated protein kinase 5 | MAP kinase-activated protein kinase 5 |
| MAP3K1 | Mitogen-activated protein kinase kinase kinase 1 | Mitogen-activated protein kinase kinase kinase 1 |
| CDK18 | Cyclin-dependent kinase 18 | Cyclin-dependent kinase 18 |
| CDK10 | Cyclin-dependent kinase 10 | Cyclin-dependent kinase 10 |
| TTK | Dual specificity protein kinase TTK | Dual specificity protein kinase TTK |
| PIM2 | Serine/threonine-protein kinase pim-2 | Serine/threonine-protein kinase pim-2 |
| PKMYT1 | Membrane-associated tyrosine- and threonine-specific cdc2-inhibitory kinase | Membrane-associated tyrosine- and threonine-specific cdc2-inhibitory kinase |
| MKNK2 | MAP kinase-interacting serine/threonine-protein kinase 2 | MAP kinase-interacting serine/threonine-protein kinase 2 |
| KAT2B | Histone acetyltransferase KAT2B | Histone acetyltransferase KAT2B |
| NEK2 | Serine/threonine-protein kinase Nek2 | Serine/threonine-protein kinase Nek2 |
| HASPIN | Serine/threonine-protein kinase haspin | Serine/threonine-protein kinase haspin |
| PIR | Pirin | Pirin |
| CYP1B1 | Cytochrome P450 1B1 | Cytochrome P450 1B1 |
| ERN1 | Serine/threonine-protein kinase/endoribonuclease IRE1 | Serine/threonine-protein kinase/endoribonuclease IRE1 |
| MELK | Maternal embryonic leucine zipper kinase | Maternal embryonic leucine zipper kinase |
| COQ8A | Atypical kinase COQ8A, mitochondrial | Atypical kinase COQ8A, mitochondrial |
| RIOK2 | Serine/threonine-protein kinase RI02 | Serine/threonine-protein kinase RI02 |
| RPS6KA6 | Ribosomal protein S6 kinase alpha-6 | Ribosomal protein S6 kinase alpha-6 |
| MAP3K20 | Mitogen-activated protein kinase kinase kinase 20 | Mitogen-activated protein kinase kinase kinase 20 |
| MAPKAPK3 | MAP kinase-activated protein kinase 3 | MAP kinase-activated protein kinase 3 |
| ULK3 | Serine/threonine-protein kinase ULK3 | Serine/threonine-protein kinase ULK3 |
| MAP3K21 | Mitogen-activated protein kinase kinase kinase 21 | Mitogen-activated protein kinase kinase kinase 21 |
| COQ8B | Atypical kinase COQ8B, mitochondrial | Atypical kinase COQ8B, mitochondrial |
| TNK1 | Non-receptor tyrosine-protein kinase TNK1 | Non-receptor tyrosine-protein kinase TNK1 |
| BMPR1A | Bone morphogenetic protein receptor type-1A | Bone morphogenetic protein receptor type-1A |
| STK17A | Serine/threonine-protein kinase 17A | Serine/threonine-protein kinase 17A |
| CDK11A | Cyclin-dependent kinase 11A | Cyclin-dependent kinase 11A |
| TESK2 | Dual specificity testis-specific protein kinase 2 | Dual specificity testis-specific protein kinase 2 |
| NLK | Serine/threonine-protein kinase NLK | Serine/threonine-protein kinase NLK |
| STK35 | Serine/threonine-protein kinase 35 | Serine/threonine-protein kinase 35 |
| PKN3 | Serine/threonine-protein kinase N3 | Serine/threonine-protein kinase N3 |
| STK33 | Serine/threonine-protein kinase 33 | Serine/threonine-protein kinase 33 |
| SBK1 | Serine/threonine-protein kinase SBK1 | Serine/threonine-protein kinase SBK1 |
| SLC9A2 | Sodium/hydrogen exchanger 2 | Sodium/hydrogen exchanger 2 |
| CSNK2A3 | Casein kinase II subunit alpha 3 | Casein kinase II subunit alpha 3 |
| TACC3 | Transforming acidic coiled-coil-containing protein 3 | Transforming acidic coiled-coil-containing protein 3 |
| GSPT1 | Eukaryotic peptide chain release factor GTP-binding subunit ERF3A | Eukaryotic peptide chain release factor GTP-binding subunit ERF3A |
| SLC9A7 | Sodium/hydrogen exchanger 7 | Sodium/hydrogen exchanger 7 |
| RPS6KC1 | Ribosomal protein S6 kinase delta-1 | Ribosomal protein S6 kinase delta-1 |
| TBCK | TBC domain-containing protein kinase-like protein | TBC domain-containing protein kinase-like protein |
| CRABP1 | Cellular retinoic acid-binding protein 1 | Cellular retinoic acid-binding protein 1 |
| BCKDK | [3-methyl-2-oxobutanoate dehydrogenase [lipoamide]] kinase, mitochondrial | [3-methyl-2-oxobutanoate dehydrogenase [lipoamide]] kinase, mitochondrial |
| TRPM7 | Transient receptor potential cation channel subfamily M member 7 | Transient receptor potential cation channel subfamily M member 7 |
| TRIB3 | Tribbles homolog 3 | Tribbles homolog 3 |
| UHMK1 | Serine/threonine-protein kinase Kist | Serine/threonine-protein kinase Kist |
| PDIK1L | Serine/threonine-protein kinase PDIK1L | Serine/threonine-protein kinase PDIK1L |
| STK40 | Serine/threonine-protein kinase 40 | Serine/threonine-protein kinase 40 |
| SLC9A4 | Sodium/hydrogen exchanger 4 | Sodium/hydrogen exchanger 4 |
| EPHB3 | Ephrin type-B receptor 3 | Ephrin type-B receptor 3 |
| NTRK3 | NT-3 growth factor receptor | NT-3 growth factor receptor |
| MAP3K12 | Mitogen-activated protein kinase kinase kinase 12 | Mitogen-activated protein kinase kinase kinase 12 |
| MAPK11 | Mitogen-activated protein kinase 11 | Mitogen-activated protein kinase 11 |
| DDR2 | Discoidin domain-containing receptor 2 | Discoidin domain-containing receptor 2 |
| RARB | Retinoic acid receptor beta | Retinoic acid receptor beta |
| PDE4C | cAMP-specific 3',5'-cyclic phosphodiesterase 4C | cAMP-specific 3',5'-cyclic phosphodiesterase 4C |
| IDO1 | Indoleamine 2,3-dioxygenase 1 | Indoleamine 2,3-dioxygenase 1 |
| SLC9B1 | Sodium/hydrogen exchanger 9B1 | Sodium/hydrogen exchanger 9B1 |
| PRAG1 | Inactive tyrosine-protein kinase PRAG1 | Inactive tyrosine-protein kinase PRAG1 |
| TOP 1 | DNA topoisomerase 1 | DNA topoisomerase 1 |
| TOP2A | DNA topoisomerase 2-alpha | DNA topoisomerase 2-alpha |
| CXCR4 | C-X-C chemokine receptor type 4 | C-X-C chemokine receptor type 4 |
| ERBB4 | Receptor tyrosine-protein kinase erbB-4 | Receptor tyrosine-protein kinase erbB-4 |
| HCK | Tyrosine-protein kinase HCK | Tyrosine-protein kinase HCK |
| SYK | Tyrosine-protein kinase SYK | Tyrosine-protein kinase SYK |
| ACLY | ATP-citrate synthase | ATP-citrate synthase |
| IMPDH2 | Inosine-5'-monophosphate dehydrogenase 2 | Inosine-5'-monophosphate dehydrogenase 2 |
| CYP3A4 | Cytochrome P450 3A4 | Cytochrome P450 3A4 |
| TOP2B | DNA topoisomerase 2-beta | DNA topoisomerase 2-beta |
| KEAP1 | Kelch-like ECH-associated protein 1 | Kelch-like ECH-associated protein 1 |
| NR3C1 | Glucocorticoid receptor | Glucocorticoid receptor |
| DNMT1 | DNA (cytosine-5)-methyltransferase 1 | DNA (cytosine-5)-methyltransferase 1 |
| TXNRD1 | Thioredoxin reductase 1, cytoplasmic | Thioredoxin reductase 1, cytoplasmic |
| HDAC4 | Histone deacetylase 4 | Histone deacetylase 4 |
| PDGFRA | Platelet-derived growth factor receptor alpha | Platelet-derived growth factor receptor alpha |
| TUBB | Tubulin beta chain | Tubulin beta chain |
| TUBB2B | Tubulin beta-2B chain | Tubulin beta-2B chain |
| GABRA5 | Gamma-aminobutyric acid receptor subunit alpha-5 | Gamma-aminobutyric acid receptor subunit alpha-5 |
| EPHB1 | Ephrin type-B receptor 1 | Ephrin type-B receptor 1 |
| KCNQ2 | Potassium voltage-gated channel subfamily KQT member 2 | Potassium voltage-gated channel subfamily KQT member 2 |
| GRIK2 | Glutamate receptor ionotropic, kainate 2 | Glutamate receptor ionotropic, kainate 2 |
| CALM1 | Calmodulin-1 | Calmodulin-1 |
| HDAC5 | Histone deacetylase 5 | Histone deacetylase 5 |
| DNMT3A | DNA (cytosine-5)-methyltransferase 3A | DNA (cytosine-5)-methyltransferase 3A |
| KIF5B | Kinesin-1 heavy chain | Kinesin-1 heavy chain |
| POLD1 | DNA polymerase delta catalytic subunit | DNA polymerase delta catalytic subunit |
| CUL4A | Cullin-4A | Cullin-4A |
| TUBB2A | Tubulin beta-2A chain | Tubulin beta-2A chain |
| PDGFRB | Platelet-derived growth factor receptor beta | Platelet-derived growth factor receptor beta |
| FGR | Tyrosine-protein kinase Fgr | Tyrosine-protein kinase Fgr |
| PRKCG | Protein kinase C gamma type | Protein kinase C gamma type |
| SCN3A | Sodium channel protein type 3 subunit alpha | Sodium channel protein type 3 subunit alpha |
| HDAC9 | Histone deacetylase 9 | Histone deacetylase 9 |
| BACE1 | Beta-secretase 1 | Beta-secretase 1 |
| HSP90AA1 | Heat shock protein HSP 90-alpha | Heat shock protein HSP 90-alpha |
| HSP90AB1 | Heat shock protein HSP 90-beta | Heat shock protein HSP 90-beta |
| PDPK1 | 3-phosphoinositide-dependent protein kinase 1 | 3-phosphoinositide-dependent protein kinase 1 |
| BIRC2 | Baculoviral IAP repeat-containing protein 2 | Baculoviral IAP repeat-containing protein 2 |
| CHEK2 | Serine/threonine-protein kinase Chk2 | Serine/threonine-protein kinase Chk2 |
| MAP3K5 | Mitogen-activated protein kinase kinase kinase 5 | Mitogen-activated protein kinase kinase kinase 5 |
| CASP1 | Caspase-1 | Caspase-1 |
| NAE1 | NEDD8-activating enzyme E1 regulatory subunit | NEDD8-activating enzyme E1 regulatory subunit |
| PIK3R1 | Phosphatidylinositol 3-kinase regulatory subunit alpha | Phosphatidylinositol 3-kinase regulatory subunit alpha |
| IKBKB | Inhibitor of nuclear factor kappa-B kinase subunit beta | Inhibitor of nuclear factor kappa-B kinase subunit beta |
| PSMA1 | Proteasome subunit alpha type-1 | Proteasome subunit alpha type-1 |
| ITGB1 | Integrin beta-1 | Integrin beta-1 |
| PRKDC | DNA-dependent protein kinase catalytic subunit | DNA-dependent protein kinase catalytic subunit |
| PIK3R2 | Phosphatidylinositol 3-kinase regulatory subunit beta | Phosphatidylinositol 3-kinase regulatory subunit beta |
| FGF2 | Fibroblast growth factor 2 | Fibroblast growth factor 2 |
| ZAP70 | Tyrosine-protein kinase ZAP-70 | Tyrosine-protein kinase ZAP-70 |
| PIP4K2B | Phosphatidylinositol 5-phosphate 4-kinase type-2 beta | Phosphatidylinositol 5-phosphate 4-kinase type-2 beta |
| GAPDH | Glyceraldehyde-3-phosphate dehydrogenase | Glyceraldehyde-3-phosphate dehydrogenase |
| FASN | Fatty acid synthase | Fatty acid synthase |
| PKM | Pyruvate kinase PKM | Pyruvate kinase PKM |
| VCP | Transitional endoplasmic reticulum ATPase | Transitional endoplasmic reticulum ATPase |
| KDM1A | Lysine-specific histone demethylase 1A | Lysine-specific histone demethylase 1A |
| TAB1 | TGF-beta-activated kinase 1 and MAP3K7-binding protein 1 | TGF-beta-activated kinase 1 and MAP3K7-binding protein 1 |
| EP300 | Histone acetyltransferase p300 | Histone acetyltransferase p300 |
| VRK1 | Serine/threonine-protein kinase VRK1 | Serine/threonine-protein kinase VRK1 |
| RPS6KA5 | Ribosomal protein S6 kinase alpha-5 | Ribosomal protein S6 kinase alpha-5 |
| UBA2 | SUMO-activating enzyme subunit 2 | SUMO-activating enzyme subunit 2 |
| LDHA | L-lactate dehydrogenase A chain | L-lactate dehydrogenase A chain |
| TKT | Transketolase | Transketolase |
| HPRT1 | Hypoxanthine-guanine phosphoribosyltransferase | Hypoxanthine-guanine phosphoribosyltransferase |
| KDM2A | Lysine-specific demethylase 2A | Lysine-specific demethylase 2A |
| EHMT1 | Histone-lysine N-methyltransferase EHMT1 | Histone-lysine N-methyltransferase EHMT1 |
| CAMK1D | Calcium/calmodulin-dependent protein kinase type 1D | Calcium/calmodulin-dependent protein kinase type 1D |
| WDR5 | WD repeat-containing protein 5 | WD repeat-containing protein 5 |
| EHMT2 | Histone-lysine N-methyltransferase EHMT2 | Histone-lysine N-methyltransferase EHMT2 |
| RAC1 | Ras-related C3 botulinum toxin substrate 1 | Ras-related C3 botulinum toxin substrate 1 |
| OGT | UDP-N-acetylglucosamine--peptide N-acetylglucosaminyltransferase 110 kDa subunit | UDP-N-acetylglucosamine--peptide N-acetylglucosaminyltransferase 110 kDa subunit |
| NCOA1 | Nuclear receptor coactivator 1 | Nuclear receptor coactivator 1 |
| PHGDH | D-3-phosphoglycerate dehydrogenase | D-3-phosphoglycerate dehydrogenase |
| ARHGDIA | Rho GDP-dissociation inhibitor 1 | Rho GDP-dissociation inhibitor 1 |
| RBBP4 | Histone-binding protein RBBP4 | Histone-binding protein RBBP4 |
| BPTF | Nucleosome-remodeling factor subunit BPTF | Nucleosome-remodeling factor subunit BPTF |
| KAT5 | Histone acetyltransferase KAT5 | Histone acetyltransferase KAT5 |
| NOTCH1 | Neurogenic locus notch homolog protein 1 | Neurogenic locus notch homolog protein 1 |
| PPIA | Peptidyl-prolyl cis-trans isomerase A | Peptidyl-prolyl cis-trans isomerase A |
| FKBP4 | Peptidyl-prolyl cis-trans isomerase FKBP4 | Peptidyl-prolyl cis-trans isomerase FKBP4 |
| CREBBP | CREB-binding protein | CREB-binding protein |
| POLB | DNA polymerase beta | DNA polymerase beta |
| APEX1 | DNA-(apurinic or apyrimidinic site) endonuclease | DNA-(apurinic or apyrimidinic site) endonuclease |
| CCNT1 | Cyclin-T1 | Cyclin-T 1 |
| EIF4A1 | Eukaryotic initiation factor 4A-I | Eukaryotic initiation factor 4A-I |
| USP7 | Ubiquitin carboxyl-terminal hydrolase 7 | Ubiquitin carboxyl-terminal hydrolase 7 |
| CTNNB1 | Catenin beta-1 | Catenin beta-1 |
| TXN | Thioredoxin | Thioredoxin |
| DDX3X | ATP-dependent RNA helicase DDX3X | ATP-dependent RNA helicase DDX3X |
| NLRP3 | NACHT, LRR and PYD domains-containing protein 3 | NACHT, LRR and PYD domains-containing protein 3 |
| TNKS | Poly [ADP-ribose] polymerase tankyrase-1 | Poly [ADP-ribose] polymerase tankyrase-1 |
| PAK3 | Serine/threonine-protein kinase PAK 3 | Serine/threonine-protein kinase PAK 3 |
| PKN1 | Serine/threonine-protein kinase N1 | Serine/threonine-protein kinase N1 |
| CISD1 | CDGSH iron-sulfur domain-containing protein 1 | CDGSH iron-sulfur domain-containing protein 1 |
| WNK1 | Serine/threonine-protein kinase WNK1 | Serine/threonine-protein kinase WNK1 |
| BRD1 | Bromodomain-containing protein 1 | Bromodomain-containing protein 1 |
| XIAP | E3 ubiquitin-protein ligase XIAP | E3 ubiquitin-protein ligase XIAP |
| PRDX1 | Peroxiredoxin-1 | Peroxiredoxin-1 |
| KMT2A | Histone-lysine N-methyltransferase 2A | Histone-lysine N-methyltransferase 2A |
| KDM5C | Lysine-specific demethylase 5C | Lysine-specific demethylase 5C |
| RPA1 | Replication protein A 70 kDa DNA-binding subunit | Replication protein A 70 kDa DNA-binding subunit |
| CAMK1 | Calcium/calmodulin-dependent protein kinase type 1 | Calcium/calmodulin-dependent protein kinase type 1 |
| EIF4A3 | Eukaryotic initiation factor 4A-III | Eukaryotic initiation factor 4A-III |
| TAF1 | Transcription initiation factor TFIID subunit 1 | Transcription initiation factor TFIID subunit |
| | | 1 |
| ALKBH3 | Alpha-ketoglutarate-dependent dioxygenase alkB homolog 3 | Alpha-ketoglutarate-dependent dioxygenase alkB homolog 3 |
| MAP3K2 | Mitogen-activated protein kinase kinase kinase 2 | Mitogen-activated protein kinase kinase kinase 2 |
| RELA | Transcription factor p65 | Transcription factor p65 |
| NCOR2 | Nuclear receptor corepressor 2 | Nuclear receptor corepressor 2 |
| PRPF4B | Serine/threonine-protein kinase PRP4 homolog | Serine/threonine-protein kinase PRP4 homolog |
| RPS3 | 40S ribosomal protein S3 | 40S ribosomal protein S3 |
| RPSA | 40S ribosomal protein SA | 40S ribosomal protein SA |
| RPS27A | Ubiquitin-40S ribosomal protein S27a | Ubiquitin-40S ribosomal protein S27a |
| RPS20 | 40S ribosomal protein S20 | 40S ribosomal protein S20 |
| RPL18 | 60S ribosomal protein L18 | 60S ribosomal protein L18 |
| RPS5 | 40S ribosomal protein S5 | 40S ribosomal protein S5 |
| RPL6 | 60S ribosomal protein L6 | 60S ribosomal protein L6 |
| RPLP0 | 60S acidic ribosomal protein P0 | 60S acidic ribosomal protein P0 |
| RPL3 | 60S ribosomal protein L3 | 60S ribosomal protein L3 |
| RPL18A | 60S ribosomal protein L18a | 60S ribosomal protein L18a |
| RPL28 | 60S ribosomal protein L28 | 60S ribosomal protein L28 |
| RPL19 | 60S ribosomal protein L19 | 60S ribosomal protein L19 |
| RPL34 | 60S ribosomal protein L34 | 60S ribosomal protein L34 |
| RPS13 | 40S ribosomal protein S13 | 40S ribosomal protein S13 |
| RPL24 | 60S ribosomal protein L24 | 60S ribosomal protein L24 |
| RPS15 | 40S ribosomal protein S15 | 40S ribosomal protein S15 |
| RPL5 | 60S ribosomal protein L5 | 60S ribosomal protein L5 |
| RPS10 | 40S ribosomal protein S10 | 40S ribosomal protein S10 |
| RPL35 | 60S ribosomal protein L35 | 60S ribosomal protein L35 |
| RPS6 | 40S ribosomal protein S6 | 40S ribosomal protein S6 |
| RPS24 | 40S ribosomal protein S24 | 40S ribosomal protein S24 |
| RPS2 | 40S ribosomal protein S2 | 40S ribosomal protein S2 |
| RPL11 | 60S ribosomal protein L11 | 60S ribosomal protein L11 |
| RPS8 | 40S ribosomal protein S8 | 40S ribosomal protein S8 |
| RPL32 | 60S ribosomal protein L32 | 60S ribosomal protein L32 |
| RPS3A | 40S ribosomal protein S3a | 40S ribosomal protein S3a |
| RPL10 | 60S ribosomal protein L10 | 60S ribosomal protein L10 |
| RPL7A | 60S ribosomal protein L7a | 60S ribosomal protein L7a |
| RPL30 | 60S ribosomal protein L30 | 60S ribosomal protein L30 |
| RPL8 | 60S ribosomal protein L8 | 60S ribosomal protein L8 |
| RPL26 | 60S ribosomal protein L26 | 60S ribosomal protein L26 |
| RPS14 | 40S ribosomal protein S14 | 40S ribosomal protein S14 |
| RPL13 | 60S ribosomal protein L13 | 60S ribosomal protein L13 |
| RPS9 | 40S ribosomal protein S9 | 40S ribosomal protein S9 |
| RPS21 | 40S ribosomal protein S21 | 40S ribosomal protein S21 |
| RPS7 | 40S ribosomal protein S7 | 40S ribosomal protein S7 |
| RPL38 | 60S ribosomal protein L38 | 60S ribosomal protein L38 |
| RPL4 | 60S ribosomal protein L4 | 60S ribosomal protein L4 |
| RPL15 | 60S ribosomal protein L15 | 60S ribosomal protein L15 |
| RPS17 | 40S ribosomal protein S17 | 40S ribosomal protein S17 |
| RPS23 | 40S ribosomal protein S23 | 40S ribosomal protein S23 |
| RPL14 | 60S ribosomal protein L14 | 60S ribosomal protein L14 |
| RPL12 | 60S ribosomal protein L12 | 60S ribosomal protein L12 |
| RPS4X | 40S ribosomal protein S4, X isoform | 40S ribosomal protein S4, X isoform |
| RPL23A | 60S ribosomal protein L23a | 60S ribosomal protein L23a |
| RPL10A | 60S ribosomal protein L23a | 60S ribosomal protein L23a |
| RPS18 | 40S ribosomal protein S18 | 40S ribosomal protein S18 |
| SLC7A5 | Large neutral amino acids transporter small subunit 1 | Large neutral amino acids transporter small subunit 1 |
| ORAI1 | Calcium release-activated calcium channel protein 1 | Calcium release-activated calcium channel protein 1 |
| PI4K2A | Phosphatidylinositol 4-kinase type 2-alpha | Phosphatidylinositol 4-kinase type 2-alpha |
| SUMO1 | Small ubiquitin-related modifier 1 | Small ubiquitin-related modifier 1 |
| HSPA8 | Heat shock cognate 71 kDa protein | Heat shock cognate 71 kDa protein |
| HSPD1 | 60 kDa heat shock protein, mitochondrial | 60 kDa heat shock protein, mitochondrial |
| CSNK2B | Casein kinase II subunit beta | Casein kinase II subunit beta |
| PPP2CA | Serine/threonine-protein phosphatase 2A catalytic subunit alpha isoform | Serine/threonine-protein phosphatase 2A catalytic subunit alpha isoform |
| FSCN1 | Fascin | Fascin |
| BID | BH3-interacting domain death agonist | BH3-interacting domain death agonist |
| DCUN1D1 | DCN1-like protein 1 | DCN1-like protein 1 |
| USP28 | Ubiquitin carboxyl-terminal hydrolase 28 | Ubiquitin carboxyl-terminal hydrolase 28 |
| TP53BP1 | TP53-binding protein 1 | TP53-binding protein 1 |
| KDM5A | Lysine-specific demethylase 5A | Lysine-specific demethylase 5A |
| BAZ2A | Bromodomain adjacent to zinc finger domain protein 2A | Bromodomain adjacent to zinc finger domain protein 2A |
| UBE2I | SUMO-conjugating enzyme UBC9 | SUMO-conjugating enzyme UBC9 |
| SPIN1 | Spindlin-1 | Spindlin-1 |
| TDP2 | Tyrosyl-DNA phosphodiesterase 2 | Tyrosyl-DNA phosphodiesterase 2 |
| KDM5B | Lysine-specific demethylase 5B | Lysine-specific demethylase 5B |
| RBBP5 | Retinoblastoma-binding protein 5 | Retinoblastoma-binding protein 5 |
| PRMT1 | Protein arginine N-methyltransferase 1 | Protein arginine N-methyltransferase 1 |
| MLLT1 | Protein ENL | Protein ENL |
| SETDB1 | Histone-lysine N-methyltransferase SETDB1 | Histone-lysine N-methyltransferase SETDB1 |
| ARNT | Aryl hydrocarbon receptor nuclear translocator | Aryl hydrocarbon receptor nuclear translocator |
| SNRNP200 | U5 small nuclear ribonucleoprotein 200 kDa helicase | U5 small nuclear ribonucleoprotein 200 kDa helicase |
| ATAD2 | ATPase family AAA domain-containing protein 2 | ATPase family AAA domain-containing protein 2 |
| SQSTM1 | Sequestosome-1 | Sequestosome-1 |
| DPY30 | Protein dpy-30 homolog | Protein dpy-30 homolog |
| WRN | Werner syndrome ATP-dependent helicase | Werner syndrome ATP-dependent helicase |
| JMJD1C | Probable JmjC domain-containing histone demethylation protein 2C | Probable JmjC domain-containing histone demethylation protein 2C |
| MDM4 | Protein Mdm4 | Protein Mdm4 |
| L3MBTL3 | Lethal(3)malignant brain tumor-like protein 3 | Lethal(3)malignant brain tumor-like protein 3 |
| LDLR | Low-density lipoprotein receptor | Low-density lipoprotein receptor |
| GLUL | Glutamine synthetase | Glutamine synthetase |
| HRAS | GTPase HRas | GTPase HRas |
| APOBEC3G | DNA dC->dU-editing enzyme APOBEC-3G | DNA dC->dU-editing enzyme APOBEC-3G |
| TSG101 | Tumor susceptibility gene 101 protein | Tumor susceptibility gene 101 protein |
| LRP6 | Low-density lipoprotein receptor-related protein 6 | Low-density lipoprotein receptor-related protein 6 |
| ENO1 | Alpha-enolase | Alpha-enolase |
| ANXA2 | Annexin A2 | Annexin A2 |
| SOD1 | Superoxide dismutase [Cu-Zn] | Superoxide dismutase [Cu-Zn] |
| KAT6A | Histone acetyltransferase KAT6A | Histone acetyltransferase KAT6A |
| CBS | Cystathionine beta-synthase | Cystathionine beta-synthase |
| RUVBL1 | RuvB-like 1 | RuvB-like 1 |
| UHRF1 | E3 ubiquitin-protein ligase UHRF1 | E3 ubiquitin-protein ligase UHRF1 |
| HSPA5 | Endoplasmic reticulum chaperone BiP | Endoplasmic reticulum chaperone BiP |
| SQLE | Squalene monooxygenase | Squalene monooxygenase |
| NEDD4 | E3 ubiquitin-protein ligase NEDD4 | E3 ubiquitin-protein ligase NEDD4 |
| RBBP7 | Histone-binding protein RBBP7 | Histone-binding protein RBBP7 |
| UBE2N | Ubiquitin-conjugating enzyme E2 N | Ubiquitin-conjugating enzyme E2 N |
| NRAS | GTPase NRas | GTPase NRas |
| PTEN | Phosphatidylinositol 3,4,5-trisphosphate 3-phosphatase and dual-specificity protein phosphatase PTEN | Phosphatidylinositol 3,4,5-trisphosphate 3-phosphatase and dual-specificity protein phosphatase PTEN |
| RHOA | Transforming protein RhoA | Transforming protein RhoA |
| RNF31 | E3 ubiquitin-protein ligase RNF31 | E3 ubiquitin-protein ligase RNF31 |
| PIN4 | Peptidyl-prolyl cis-trans isomerase NIMA-interacting 4 | Peptidyl-prolyl cis-trans isomerase NIMA-interacting 4 |
| TPI1 | Triosephosphate isomerase | Triosephosphate isomerase |
| PPIG | Peptidyl-prolyl cis-trans isomerase G | Peptidyl-prolyl cis-trans isomerase G |
| NCOA2 | Nuclear receptor coactivator 2 | Nuclear receptor coactivator 2 |
| FOXO1 | Forkhead box protein O1 | Forkhead box protein O1 |
| PSIP1 | PC4 and SFRS1-interacting protein | PC4 and SFRS1-interacting protein |
| MACROD2 | ADP-ribose glycohydrolase MACROD2 | ADP-ribose glycohydrolase MACROD2 |
| SETD2 | Histone-lysine N-methyltransferase SETD2 | Histone-lysine N-methyltransferase SETD2 |
| NFKBIA | NF-kappa-B inhibitor alpha | NF-kappa-B inhibitor alpha |
| PLA2G4A | Cytosolic phospholipase A2 | Cytosolic phospholipase A2 |
| ICAM1 | Intercellular adhesion molecule 1 | Intercellular adhesion molecule 1 |
| ACVR1B | Activin receptor type-1B | Activin receptor type-1B |
| CYBB | Cytochrome b-245 heavy chain | Cytochrome b-245 heavy chain |
| STAMBP | STAM-binding protein | STAM-binding protein |
| NFKB1 | Nuclear factor NF-kappa-B p105 subunit | Nuclear factor NF-kappa-B p105 subunit |
| PAK2 | Serine/threonine-protein kinase PAK 2 | Serine/threonine-protein kinase PAK 2 |
| ITPR1 | Inositol 1,4,5-trisphosphate receptor type 1 | Inositol 1,4,5-trisphosphate receptor type 1 |
| ELAVL1 | ELAV-like protein 1 | ELAV-like protein 1 |
| NFKB2 | Nuclear factor NF-kappa-B p100 subunit | Nuclear factor NF-kappa-B p100 subunit |
| STAT1 | Signal transducer and activator of transcription 1-alpha/beta | Signal transducer and activator of transcription 1-alpha/beta |
| STATSA | Signal transducer and activator of transcription 5A | Signal transducer and activator of transcription 5A |
| RIPK3 | Receptor-interacting serine/threonine-protein kinase 3 | Receptor-interacting serine/threonine-protein kinase 3 |
| BRSK2 | Serine/threonine-protein kinase BRSK2 | Serine/threonine-protein kinase BRSK2 |
| ERCC4 | DNA repair endonuclease XPF | DNA repair endonuclease XPF |
| NPM1 | Nucleophosmin | Nucleophosmin |
| IKBKG | NF-kappa-B essential modulator | NF-kappa-B essential modulator |
| PBK | Lymphokine-activated killer T-cell-originated protein kinase | Lymphokine-activated killer T-cell-originated protein kinase |
| CD22 | B-cell receptor CD22 | B-cell receptor CD22 |
| APC | Adenomatous polyposis coli protein | Adenomatous polyposis coli protein |
| PLCG1 | 1-phosphatidylinositol 4,5-bisphosphate phosphodiesterase gamma-1 | 1-phosphatidylinositol 4,5-bisphosphate phosphodiesterase gamma-1 |
| ING2 | Inhibitor of growth protein 2 | Inhibitor of growth protein 2 |
| ERCC1 | DNA excision repair protein ERCC-1 | DNA excision repair protein ERCC-1 |
| KDM2B | Lysine-specific demethylase 2B | Lysine-specific demethylase 2B |
| ETV6 | Transcription factor ETV6 | Transcription factor ETV6 |
| CBX4 | E3 SUMO-protein ligase CBX4 | E3 SUMO-protein ligase CBX4 |
| USP25 | Ubiquitin carboxyl-terminal hydrolase 25 | Ubiquitin carboxyl-terminal hydrolase 25 |
| HSF1 | Heat shock factor protein 1 | Heat shock factor protein 1 |
| TRIM33 | E3 ubiquitin-protein ligase TRIM33 | E3 ubiquitin-protein ligase TRIM33 |
| HNRNPA1 | Heterogeneous nuclear ribonucleoprotein A1 | Heterogeneous nuclear ribonucleoprotein A1 |
| TERF2 | Telomeric repeat-binding factor 2 | Telomeric repeat-binding factor 2 |
| PTBP1 | Polypyrimidine tract-binding protein 1 | Polypyrimidine tract-binding protein 1 |
| NR2C2 | Nuclear receptor subfamily 2 group C member 2 | Nuclear receptor subfamily 2 group C member 2 |
| NOTCH2 | Neurogenic locus notch homolog protein 2 | Neurogenic locus notch homolog protein 2 |
| USP8 | Ubiquitin carboxyl-terminal hydrolase 8 | Ubiquitin carboxyl-terminal hydrolase 8 |
| RGS12 | Regulator of G-protein signaling 12 | Regulator of G-protein signaling 12 |
| ATF1 | Cyclic AMP-dependent transcription factor ATF-1 | Cyclic AMP-dependent transcription factor ATF-1 |
| PPM1B | Protein phosphatase 1B | Protein phosphatase 1B |
| PDCD4 | Programmed cell death protein 4 | Programmed cell death protein 4 |
| LMNA | Prelamin-A/C | Prelamin-A/C |
| MITF | Microphthalmia-associated transcription factor | Microphthalmia-associated transcription factor |
| ADCY3 | Adenylate cyclase type 3 | Adenylate cyclase type 3 |
| EEF1A1 | Elongation factor 1-alpha 1 | Elongation factor 1-alpha 1 |
| MYH9 | Myosin-9 | Myosin-9 |
| BCL3 | B-cell lymphoma 3 protein | B-cell lymphoma 3 protein |
| XRCCS | X-ray repair cross-complementing protein 5 | X-ray repair cross-complementing protein 5 |
| USP4 | Ubiquitin carboxyl-terminal hydrolase 4 | Ubiquitin carboxyl-terminal hydrolase 4 |
| AGL | Glycogen debranching enzyme | Glycogen debranching enzyme |
| XRCC6 | X-ray repair cross-complementing protein 6 | X-ray repair cross-complementing protein 6 |
| MAGI3 | Membrane-associated guanylate kinase, WW and PDZ domain-containing protein 3 | Membrane-associated guanylate kinase, WW and PDZ domain-containing protein 3 |
| DVL1 | Segment polarity protein dishevelled homolog DVL-1 | Segment polarity protein dishevelled homolog DVL-1 |
| VDAC2 | Voltage-dependent anion-selective channel protein 2 | Voltage-dependent anion-selective channel protein 2 |
| MDH1 | Malate dehydrogenase, cytoplasmic | Malate dehydrogenase, cytoplasmic |
| CBX5 | Chromobox protein homolog 5 | Chromobox protein homolog 5 |
| USP10 | Ubiquitin carboxyl-terminal hydrolase 10 | Ubiquitin carboxyl-terminal hydrolase 10 |
| CBX1 | Chromobox protein homolog 1 | Chromobox protein homolog 1 |
| BRD8 | Bromodomain-containing protein 8 | Bromodomain-containing protein 8 |
| TSNAX | Translin-associated protein X | Translin-associated protein X |
| CREB1 | Cyclic AMP-responsive element-binding protein 1 | Cyclic AMP-responsive element-binding protein 1 |
| RBCK1 | RanBP-type and C3HC4-type zinc finger-containing protein 1 | RanBP-type and C3HC4-type zinc finger-containing protein 1 |
| UBA1 | Ubiquitin-like modifier-activating enzyme 1 | Ubiquitin-like modifier-activating enzyme 1 |
| MACROD1 | ADP-ribose glycohydrolase MACROD1 | ADP-ribose glycohydrolase MACROD1 |
| PELP1 | Proline-, glutamic acid- and leucine-rich protein 1 | Proline-, glutamic acid- and leucine-rich protein 1 |
| BAP 1 | Ubiquitin carboxyl-terminal hydrolase BAP1 | Ubiquitin carboxyl-terminal hydrolase BAP1 |
| TERF2IP | Telomeric repeat-binding factor 2-interacting protein 1 | Telomeric repeat-binding factor 2-interacting protein 1 |
| LARP7 | La-related protein 7 | La-related protein 7 |
| UBA7 | Ubiquitin-like modifier-activating enzyme 7 | Ubiquitin-like modifier-activating enzyme 7 |
| SUMO3 | Small ubiquitin-related modifier 3 | Small ubiquitin-related modifier 3 |
| SUMO2 | Small ubiquitin-related modifier 2 | Small ubiquitin-related modifier 2 |
| GIGYF2 | GRB10-interacting GYF protein 2 | GRB10-interacting GYF protein 2 |
| S100A10 | Protein S100-A10 | Protein S100-A10 |
| GRIK3 | Glutamate receptor ionotropic, kainate 3 | Glutamate receptor ionotropic, kainate 3 |
| TLR8 | Toll-like receptor 8 | Toll-like receptor 8 |
| DYRK2 | Dual specificity tyrosine-phosphorylation-regulated kinase 2 | Dual specificity tyrosine-phosphorylation-regulated kinase 2 |
| NOS1 | Nitric oxide synthase, brain | Nitric oxide synthase, brain |
| WNK3 | Serine/threonine-protein kinase WNK3 | Serine/threonine-protein kinase WNK3 |
| TLR2 | Toll-like receptor 2 | Toll-like receptor 2 |
| BCL2L11 | Bcl-2-like protein 11 | Bcl-2-like protein 11 |
| SLC11A2 | Natural resistance-associated macrophage protein 2 | Natural resistance-associated macrophage protein 2 |
| ABCB11 | Bile salt export pump | Bile salt export pump |
| ITGAL | Integrin alpha-L | Integrin alpha-L |
| ROCK1 | Rho-associated protein kinase 1 | Rho-associated protein kinase 1 |
| CA12 | Carbonic anhydrase 12 | Carbonic anhydrase 12 |
| EPHA7 | Ephrin type-A receptor 7 | Ephrin type-A receptor 7 |
| PRKCA | Protein kinase C alpha type | Protein kinase C alpha type |
| CA2 | Carbonic anhydrase 2 | Carbonic anhydrase 2 |
| PDE2A | cGMP-dependent 3',5'-cyclic phosphodiesterase | cGMP-dependent 3',5'-cyclic phosphodiesterase |
| EPHA4 | Ephrin type-A receptor 4 | Ephrin type-A receptor 4 |
| ACE | Angiotensin-converting enzyme | Angiotensin-converting enzyme |
| RAF1 | RAF proto-oncogene serine/threonine-protein kinase | RAF proto-oncogene serine/threonine-protein kinase |
| GLA | Alpha-galactosidase A | Alpha-galactosidase A |
| MAOB | Amine oxidase [flavin-containing] B | Amine oxidase [flavin-containing] B |
| AKR1B1 | Aldo-keto reductase family 1 member B1 | Aldo-keto reductase family 1 member B1 |
| GSR | Glutathione reductase, mitochondrial | Glutathione reductase, mitochondrial |
| UMPS | Uridine 5'-monophosphate synthase | Uridine 5'-monophosphate synthase |
| THRA | Thyroid hormone receptor alpha | Thyroid hormone receptor alpha |
| GART | Trifunctional purine biosynthetic protein adenosine-3 [Includes: Phosphoribosylamine- -glycine ligase | Trifunctional purine biosynthetic protein adenosine-3 [Includes: Phosphoribosylamine--glycine ligase |
| FDPS | Farnesyl pyrophosphate synthase | Farnesyl pyrophosphate synthase |
| ADH5 | Alcohol dehydrogenase class-3 | Alcohol dehydrogenase class-3 |
| RXRB | Retinoic acid receptor RXR-beta | Retinoic acid receptor RXR-beta |
| DHFR | Dihydrofolate reductase | Dihydrofolate reductase |
| PDE10A | cAMP and cAMP-inhibited cGMP 3',5'-cyclic phosphodiesterase 10A | cAMP and cAMP-inhibited cGMP 3',5'-cyclic phosphodiesterase 10A |
| IDH1 | Isocitrate dehydrogenase [NADP] cytoplasmic | Isocitrate dehydrogenase [NADP] cytoplasmic |
| ITGB2 | Integrin beta-2 | Integrin beta-2 |
| COMT | Catechol O-methyltransferase | Catechol O-methyltransferase |
| PRKCQ | Protein kinase C theta type | Protein kinase C theta type |
| IMPDH1 | Inosine-5'-monophosphate dehydrogenase 1 | Inosine-5'-monophosphate dehydrogenase 1 |
| HDAC7 | Histone deacetylase 7 | Histone deacetylase 7 |
| TUBB3 | Tubulin beta-3 chain | Tubulin beta-3 chain |
| TUBA1A | Tubulin alpha-1A chain | Tubulin alpha-1A chain |
| ADA | Adenosine deaminase | Adenosine deaminase |
| GABRG2 | Gamma-aminobutyric acid receptor subunit gamma-2 | Gamma-aminobutyric acid receptor subunit gamma-2 |
| GABBR2 | Gamma-aminobutyric acid type B receptor subunit 2 | Gamma-aminobutyric acid type B receptor subunit 2 |
| GABRB2 | Gamma-aminobutyric acid receptor subunit beta-2 | Gamma-aminobutyric acid receptor subunit beta-2 |
| GABRB3 | Gamma-aminobutyric acid receptor subunit beta-3 | Gamma-aminobutyric acid receptor subunit beta-3 |
| GABBR1 | Gamma-aminobutyric acid type B receptor subunit 1 | Gamma-aminobutyric acid type B receptor subunit 1 |
| PRKCE | Protein kinase C epsilon type | Protein kinase C epsilon type |
| SIGMAR1 | Sigma non-opioid intracellular receptor 1 | Sigma non-opioid intracellular receptor 1 |
| MTOR | Serine/threonine-protein kinase mTOR | Serine/threonine-protein kinase mTOR |
| SLC29A1 | Equilibrative nucleoside transporter 1 | Equilibrative nucleoside transporter 1 |
| KCNJ11 | ATP-sensitive inward rectifier potassium channel 11 | ATP-sensitive inward rectifier potassium channel 11 |
| GAA | Lysosomal alpha-glucosidase | Lysosomal alpha-glucosidase |
| PRKCH | Protein kinase C eta type | Protein kinase C eta type |
| PIK3CD | Phosphatidylinositol 4,5-bisphosphate 3-kinase catalytic subunit delta isoform | Phosphatidylinositol 4,5-bisphosphate 3-kinase catalytic subunit delta isoform |
| ROCK2 | Rho-associated protein kinase 2 | Rho-associated protein kinase 2 |
| MAOA | Amine oxidase [flavin-containing] A | Amine oxidase [flavin-containing] A |
| SOAT1 | Sterol O-acyltransferase 1 | Sterol O-acyltransferase 1 |
| BCR | Breakpoint cluster region protein | Breakpoint cluster region protein |
| PSMBS | Proteasome subunit beta type-5 | Proteasome subunit beta type-5 |
| XPO1 | Exportin-1 | Exportin-1 |
| MALT1 | Mucosa-associated lymphoid tissue lymphoma translocation protein 1 | Mucosa-associated lymphoid tissue lymphoma translocation protein 1 |
| IDH2 | Isocitrate dehydrogenase [NADP], mitochondrial | Isocitrate dehydrogenase [NADP], mitochondrial |
| RRM1 | Ribonucleoside-diphosphate reductase large subunit | Ribonucleoside-diphosphate reductase large subunit |
| NDUFA10 | NADH dehydrogenase [ubiquinone] 1 alpha subcomplex subunit 10, mitochondrial | NADH dehydrogenase [ubiquinone] 1 alpha subcomplex subunit 10, mitochondrial |
| EPHA5 | Ephrin type-A receptor 5 | Ephrin type-A receptor 5 |
| TUBB4B | Tubulin beta-4B chain | Tubulin beta-4B chain |
| TUBB1 | Tubulin beta-1 chain | Tubulin beta-1 chain |
| TUBB4A | Tubulin beta-4A chain | Tubulin beta-4A chain |
| TUBB6 | Tubulin beta-6 chain | Tubulin beta-6 chain |
| TUBA4A | Tubulin alpha-4A chain | Tubulin alpha-4A chain |
| TUBA1C | Tubulin alpha-1C chain | Tubulin alpha-1C chain |
| CACNB1 | Voltage-dependent L-type calcium channel subunit beta-1 | Voltage-dependent L-type calcium channel subunit beta-1 |
| ATP1A2 | Sodium/potassium-transporting ATPase subunit alpha-2 | Sodium/potassium-transporting ATPase subunit alpha-2 |
| ATP1A3 | Sodium/potassium-transporting ATPase subunit alpha-3 | Sodium/potassium-transporting ATPase subunit alpha-3 |
| GRIA1 | Glutamate receptor 1 | Glutamate receptor 1 |
| GABRB1 | Gamma-aminobutyric acid receptor subunit beta-1 | Gamma-aminobutyric acid receptor subunit beta-1 |
| ATP1A1 | Sodium/potassium-transporting ATPase subunit alpha-1 | Sodium/potassium-transporting ATPase subunit alpha-1 |
| KCNQ5 | Potassium voltage-gated channel subfamily KQT member 5 | Potassium voltage-gated channel subfamily KQT member 5 |
| PRKCD | Protein kinase C delta type | Protein kinase C delta type |
| CACNAIH | Voltage-dependent T-type calcium channel subunit alpha-1H | Voltage-dependent T-type calcium channel subunit alpha-1H |
| GRIA4 | Glutamate receptor 4 | Glutamate receptor 4 |
| KCNA3 | Potassium voltage-gated channel subfamily A member 3 | Potassium voltage-gated channel subfamily A member 3 |
| S1PR3 | Sphingosine 1-phosphate receptor 3 | Sphingosine 1-phosphate receptor 3 |
| NISCH | Nischarin | Nischarin |
| PRKCB | Protein kinase C beta type | Protein kinase C beta type |
| TLR7 | Toll-like receptor 7 | Toll-like receptor 7 |
| CACNA1B | Voltage-dependent N-type calcium channel subunit alpha-1B | Voltage-dependent N-type calcium channel subunit alpha-1B |
| CACNA2D2 | Voltage-dependent calcium channel subunit alpha-2/delta-2 | Voltage-dependent calcium channel subunit alpha-2/delta-2 |
| CPT2 | Carnitine O-palmitoyltransferase 2, mitochondrial | Carnitine O-palmitoyltransferase 2, mitochondrial |
| FAAH | Fatty-acid amide hydrolase 1 | Fatty-acid amide hydrolase 1 |
| UGCG | Ceramide glucosyltransferase | Ceramide glucosyltransferase |
| PDE1A | Calcium/calmodulin-dependent 3',5'-cyclic nucleotide phosphodiesterase 1A | Calcium/calmodulin-dependent 3',5' -cyclic nucleotide phosphodiesterase 1A |
| HPD | 4-hydroxyphenylpyruvate dioxygenase | 4-hydroxyphenylpyruvate dioxygenase |
| CA5B | Carbonic anhydrase 5B, mitochondrial | Carbonic anhydrase 5B, mitochondrial |
| CA5A | Carbonic anhydrase 5A, mitochondrial | Carbonic anhydrase 5A, mitochondrial |
| ANO1 | Anoctamin-1 | Anoctamin-1 |
| DHCR24 | Delta(24)-sterol reductase | Delta(24)-sterol reductase |
| CCDC6 | Coiled-coil domain-containing protein 6 | Coiled-coil domain-containing protein 6 |
| EML4 | Echinoderm microtubule-associated protein-like 4 | Echinoderm microtubule-associated protein-like 4 |
| KCNA2 | Potassium voltage-gated channel subfamily A member 2 | Potassium voltage-gated channel subfamily A member 2 |
| KCNB2 | Potassium voltage-gated channel subfamily B member 2 | Potassium voltage-gated channel subfamily B member 2 |
| PLA2G7 | Platelet-activating factor acetylhydrolase | Platelet-activating factor acetylhydrolase |
| GRM5 | Metabotropic glutamate receptor 5 | Metabotropic glutamate receptor 5 |
| PLA2G2A | Phospholipase A2, membrane associated | Phospholipase A2, membrane associated |
| MIF | Macrophage migration inhibitory factor | Macrophage migration inhibitory factor |
| GBA | Lysosomal acid glucosylceramidase | Lysosomal acid glucosylceramidase |
| INSR | Insulin receptor | Insulin receptor |
| GSTP1 | Glutathione S-transferase P | Glutathione S-transferase P |
| PNP | Purine nucleoside phosphorylase | Purine nucleoside phosphorylase |
| FDFT1 | Squalene synthase | Squalene synthase |
| CASP8 | Caspase-8 | Caspase-8 |
| CASP9 | Caspase-9 | Caspase-9 |
| CASP6 | Caspase-6 | Caspase-6 |
| CASP3 | Caspase-3 | Caspase-3 |
| CASP7 | Caspase-7 | Caspase-7 |
| ERAP1 | Endoplasmic reticulum aminopeptidase 1 | Endoplasmic reticulum aminopeptidase 1 |
| UBA3 | NEDD8-activating enzyme E1 catalytic subunit | NEDDS-activating enzyme E1 catalytic subunit |
| PSEN1 | Presenilin-1 | Presenilin-1 |
| FNTA | Protein famesyltransferase/geranylgeranyltransferase type-1 subunit alpha | Protein famesyltransferase/geranylgeranyltransferase type-1 subunit alpha |
| METAP1 | Methionine aminopeptidase 1 | Methionine aminopeptidase 1 |
| CASP2 | Caspase-2 | Caspase-2 |
| ITGA4 | Integrin alpha-4 | Integrin alpha-4 |
| ABCB1 | ATP-dependent translocase ABCB1 | ATP-dependent translocase ABCB1 |
| NTSR1 | Neurotensin receptor type 1 | Neurotensin receptor type 1 |
| GRM3 | Metabotropic glutamate receptor 3 | Metabotropic glutamate receptor 3 |
| LNPEP | Leucyl-cystinyl aminopeptidase | Leucyl-cystinyl aminopeptidase |
| APH1A | Gamma-secretase subunit APH-1A | Gamma-secretase subunit APH-1A |
| NCSTN | Nicastrin | Nicastrin |
| PSMB1 | Proteasome subunit beta type-1 | Proteasome subunit beta type-1 |
| PSMA2 | Proteasome subunit alpha type-2 | Proteasome subunit alpha type-2 |
| PSMB7 | Proteasome subunit beta type-7 | Proteasome subunit beta type-7 |
| PSMA5 | Proteasome subunit alpha type-5 | Proteasome subunit alpha type-5 |
| PSMA4 | Proteasome subunit alpha type-4 | Proteasome subunit alpha type-4 |
| PSMA3 | Proteasome subunit alpha type-3 | Proteasome subunit alpha type-3 |
| PSMA6 | Proteasome subunit alpha type-6 | Proteasome subunit alpha type-6 |
| PSMA7 | Proteasome subunit alpha type-7 | Proteasome subunit alpha type-7 |
| PSMB2 | Proteasome subunit beta type-2 | Proteasome subunit beta type-2 |
| EGLN1 | Egl nine homolog 1 | Egl nine homolog 1 |
| PSMB6 | Proteasome subunit beta type-6 | Proteasome subunit beta type-6 |
| PSMB4 | Proteasome subunit beta type-4 | Proteasome subunit beta type-4 |
| PSMB8 | Proteasome subunit beta type-8 | Proteasome subunit beta type-8 |
| PSMB10 | Proteasome subunit beta type-10 | Proteasome subunit beta type-10 |
| PSMB9 | Proteasome subunit beta type-9 | Proteasome subunit beta type-9 |
| FNTB | Protein farnesyltransferase subunit beta | Protein farnesyltransferase subunit beta |
| PSMB3 | Proteasome subunit beta type-3 | Proteasome subunit beta type-3 |
| ERAP2 | Endoplasmic reticulum aminopeptidase 2 | Endoplasmic reticulum aminopeptidase 2 |
| DNPEP | Aspartyl aminopeptidase | Aspartyl aminopeptidase |
| STS | Steryl-sulfatase | Steryl-sulfatase |
| SELL | L-selectin | L-selectin |
| CCR1 | C-C chemokine receptor type 1 | C-C chemokine receptor type 1 |
| PIK3CB | Phosphatidylinositol 4,5-bisphosphate 3-kinase catalytic subunit beta isoform | Phosphatidylinositol 4,5-bisphosphate 3-kinase catalytic subunit beta isoform |
| PORCN | Protein-serine O-palmitoleoyltransferase porcupine | Protein-serine O-palmitoleoyltransferase porcupine |
| P4HTM | Transmembrane prolyl 4-hydroxylase | Transmembrane prolyl 4-hydroxylase |
| ATR | Serine/threonine-protein kinase ATR | Serine/threonine-protein kinase ATR |
| CDK19 | Cyclin-dependent kinase 19 | Cyclin-dependent kinase 19 |
| CDK3 | Cyclin-dependent kinase 3 | Cyclin-dependent kinase 3 |
| CASP4 | Caspase-4 | Caspase-4 |
| PIK3R5 | Phosphoinositide 3-kinase regulatory subunit 5 | Phosphoinositide 3-kinase regulatory subunit 5 |
| CASP10 | Caspase-10 | Caspase-10 |
| NPEPPS | Puromycin-sensitive aminopeptidase | Puromycin-sensitive aminopeptidase |
| RNPEP | Aminopeptidase B | Aminopeptidase B |
| LAP3 | Cytosol aminopeptidase | Cytosol aminopeptidase |
| CDK20 | Cyclin-dependent kinase 20 | Cyclin-dependent kinase 20 |
| DPYD | Dihydropyrimidine dehydrogenase [NADP(+)] | Dihydropyrimidine dehydrogenase [NADP(+)] |
| XPNPEP1 | Xaa-Pro aminopeptidase 1 | Xaa-Pro aminopeptidase 1 |
| NAMPT | Nicotinamide phosphoribosyltransferase | Nicotinamide phosphoribosyltransferase |
| PRKACA | cAMP-dependent protein kinase catalytic subunit alpha | cAMP-dependent protein kinase catalytic subunit alpha |
| PSMD14 | 26S proteasome non-ATPase regulatory subunit 14 | 26S proteasome non-ATPase regulatory subunit 14 |
| ARAF | Serine/threonine-protein kinase A-Raf | Serine/threonine-protein kinase A-Raf |
| PRKACB | cAMP-dependent protein kinase catalytic subunit beta | cAMP-dependent protein kinase catalytic subunit beta |
| ITGAV | Integrin alpha-V | Integrin alpha-V |
| TGM2 | Protein-glutamine gamma-glutamyltransferase 2 | Protein-glutamine gamma-glutamyltransferase 2 |
| ACE2 | Angiotensin-converting enzyme 2 | Angiotensin-converting enzyme 2 |
| PAK1 | Serine/threonine-protein kinase PAK 1 | Serine/threonine-protein kinase PAK 1 |
| CAPN1 | Calpain-1 catalytic subunit | Calpain-1 catalytic subunit |
| CDC42 | Cell division control protein 42 homolog | Cell division control protein 42 homolog |
| NMT1 | Glycylpeptide N-tetradecanoyltransferase 1 | Glycylpeptide N-tetradecanoyltransferase 1 |
| CAMK2D | Calcium/calmodulin-dependent protein kinase type II subunit delta | Calcium/calmodulin-dependent protein kinase type II subunit delta |
| AKR1B10 | Aldo-keto reductase family 1 member B10 | Aldo-keto reductase family 1 member B10 |
| P4HB | Protein disulfide-isomerase | Protein disulfide-isomerase |
| CDC42BPB | Serine/threonine-protein kinase MRCK beta | Serine/threonine-protein kinase MRCK beta |
| CAMK2G | Calcium/calmodulin-dependent protein kinase type II subunit gamma | Calcium/calmodulin-dependent protein kinase type II subunit gamma |
| PTP4A1 | Protein tyrosine phosphatase type IVA 1 | Protein tyrosine phosphatase type IVA 1 |
| G6PD | Glucose-6-phosphate 1-dehydrogenase | Glucose-6-phosphate 1-dehydrogenase |
| LSS | Lanosterol synthase | Lanosterol synthase |
| ALB | Albumin | Albumin |
| ECE1 | Endothelin-converting enzyme 1 | Endothelin-converting enzyme 1 |
| CTSD | Cathepsin D | Cathepsin D |
| CTSB | Cathepsin B | Cathepsin B |
| ILK | Integrin-linked protein kinase | Integrin-linked protein kinase |
| MGLL | Monoglyceride lipase | Monoglyceride lipase |
| CASK | Peripheral plasma membrane protein CASK | Peripheral plasma membrane protein CASK |
| ADAM17 | Disintegrin and metalloproteinase domain-containing protein 17 | Disintegrin and metalloproteinase domain-containing protein 17 |
| PYGL | Glycogen phosphorylase, liver form | Glycogen phosphorylase, liver form |
| ATIC | Bifunctional purine biosynthesis protein ATIC | Bifunctional purine biosynthesis protein ATIC |
| HSD17B10 | 3-hydroxyacyl-CoA dehydrogenase type-2 | 3-hydroxyacyl-CoA dehydrogenase type-2 |
| SRPK1 | SRSF protein kinase 1 | SRSF protein kinase 1 |
| LTA4H | Leukotriene A-4 hydrolase | Leukotriene A-4 hydrolase |
| STK16 | Serine/threonine-protein kinase 16 | Serine/threonine-protein kinase 16 |
| ADK | Adenosine kinase | Adenosine kinase |
| GRK6 | G protein-coupled receptor kinase 6 | G protein-coupled receptor kinase 6 |
| ACVR1 | Activin receptor type-1 | Activin receptor type-1 |
| CES1 | Liver carboxylesterase 1 | Liver carboxylesterase 1 |
| CSNK1G3 | Casein kinase I isoform gamma-3 | Casein kinase I isoform gamma-3 |
| CYP51A1 | Lanosterol 14-alpha demethylase | Lanosterol 14-alpha demethylase |
| CAMK2A | Calcium/calmodulin-dependent protein kinase type II subunit alpha | Calcium/calmodulin-dependent protein kinase type II subunit alpha |
| BCAT1 | Branched-chain-amino-acid aminotransferase, cytosolic | Branched-chain-amino -acid aminotransferase, cytosolic |
| MAP2K4 | Dual specificity mitogen-activated protein kinase kinase 4 | Dual specificity mitogen-activated protein kinase kinase 4 |
| ACACB | Acetyl-CoA carboxylase 2 | Acetyl-CoA carboxylase 2 |
| MTAP | S-methyl-5'-thioadenosine phosphorylase | S-methyl-5'-thioadenosine phosphorylase |
| AHCY | Adenosylhomocysteinase | Adenosylhomocysteinase |
| STK24 | Serine/threonine-protein kinase 24 | Serine/threonine-protein kinase 24 |
| GALK1 | Galactokinase | Galactokinase |
| MAP2K6 | Dual specificity mitogen-activated protein kinase kinase 6 | Dual specificity mitogen-activated protein kinase kinase 6 |
| DCPS | m7GpppX diphosphatase | m7GpppX diphosphatase |
| LDHB | L-lactate dehydrogenase B chain | L-lactate dehydrogenase B chain |
| EPHX2 | Bifunctional epoxide hydrolase 2 [Includes: Cytosolic epoxide hydrolase 2 | Bifunctional epoxide hydrolase 2 [Includes: Cytosolic epoxide hydrolase 2 |
| NQO2 | Ribosyldihydronicotinamide dehydrogenase [quinone] | Ribosyldihydronicotinamide dehydrogenase [quinone] |
| SRPK2 | SRSF protein kinase 2 | SRSF protein kinase 2 |
| SAE1 | SUMO-activating enzyme subunit 1 | SUMO-activating enzyme subunit 1 |
| CARM1 | Histone-arginine methyltransferase CARM1 | Histone-arginine methyltransferase CARM1 |
| CAMK4 | Calcium/calmodulin-dependent protein kinase type IV | Calcium/calmodulin-dependent protein kinase type IV |
| TNIK | TRAF2 and NCK-interacting protein kinase | TRAF2 and NCK-interacting protein kinase |
| DCK | Deoxycytidine kinase | Deoxycytidine kinase |
| PHKG2 | Phosphorylase b kinase gamma catalytic chain, liver/testis isoform | Phosphorylase b kinase gamma catalytic chain, liver/testis isoform |
| DYRK1A | Dual specificity tyrosine-phosphorylation-regulated kinase 1A | Dual specificity tyrosine-phosphorylation-regulated kinase 1A |
| CBR1 | Carbonyl reductase [NADPH] 1 | Carbonyl reductase [NADPH] 1 |
| DAPK3 | Death-associated protein kinase 3 | Death-associated protein kinase 3 |
| FEN1 | Flap endonuclease 1 | Flap endonuclease 1 |
| OXSR1 | Serine/threonine-protein kinase OSR1 | Serine/threonine-protein kinase OSR1 |
| CLK3 | Dual specificity protein kinase CLK3 | Dual specificity protein kinase CLK3 |
| UPP1 | Uridine phosphorylase 1 | Uridine phosphorylase 1 |
| AKR1C1 | Aldo-keto reductase family 1 member C1 | Aldo-keto reductase family 1 member C1 |
| AKR1C3 | Aldo-keto reductase family 1 member C3 | Aldo-keto reductase family 1 member C3 |
| CD38 | ADP-ribosyl cyclase/cyclic ADP-ribose hydrolase 1 | ADP-ribosyl cyclase/cyclic ADP-ribose hydrolase 1 |
| SORT1 | Sortilin | Sortilin |
| GALNT2 | Polypeptide N-acetylgalactosaminyltransferase 2 | Polypeptide N-acetylgalactosaminyltransferase 2 |
| LGALS1 | Galectin-1 | Galectin-1 |
| HK2 | Hexokinase-2 | Hexokinase-2 |
| HK1 | Hexokinase-1 | Hexokinase-1 |
| HMOX1 | Heme oxygenase 1 | Heme oxygenase 1 |
| PARP14 | Protein mono-ADP-ribosyltransferase PARP14 | Protein mono-ADP-ribosyltransferase PARP14 |
| GRB2 | Growth factor receptor-bound protein 2 | Growth factor receptor-bound protein 2 |
| HMOX2 | Heme oxygenase 2 | Heme oxygenase 2 |
| DDAH1 | N(G),N(G)-dimethylarginine dimethylaminohydrolase 1 | N(G),N(G)-dimethylarginine dimethylaminohydrolase 1 |
| PARP12 | Protein mono-ADP-ribosyltransferase PARP12 | Protein mono-ADP-ribosyltransferase PARP12 |
| OAT | Ornithine aminotransferase, mitochondrial | Ornithine aminotransferase, mitochondrial |
| SIRT6 | NAD-dependent protein deacetylase sirtuin-6 | NAD-dependent protein deacetylase sirtuin-6 |
| PGK1 | Phosphoglycerate kinase 1 | Phosphoglycerate kinase 1 |
| KAT8 | Histone acetyltransferase KAT8 | Histone acetyltransferase KAT8 |
| SPR | Sepiapterin reductase | Sepiapterin reductase |
| CTH | Cystathionine gamma-lyase | Cystathionine gamma-lyase |
| SIRT5 | NAD-dependent protein deacylase sirtuin-5, mitochondrial | NAD-dependent protein deacylase sirtuin-5, mitochondrial |
| GSTO1 | Glutathione S-transferase omega-1 | Glutathione S-transferase omega-1 |
| PFKFB3 | 6-phosphofructo-2-kinase/fructose-2,6-bisphosphatase 3 | 6-phosphofructo-2-kinase/fructose-2,6-bisphosphatase 3 |
| CTBP2 | C-terminal-binding protein 2 | C-terminal-binding protein 2 |
| PARP10 | Protein mono-ADP-ribosyltransferase PARP 10 | Protein mono-ADP-ribosyltransferase PARP 10 |
| SMYD3 | Histone-lysine N-methyltransferase SMYD3 | Histone-lysine N-methyltransferase SMYD3 |
| SULT1A1 | Sulfotransferase 1A1 | Sulfotransferase 1A1 |
| AKR1C4 | Aldo-keto reductase family 1 member C4 | Aldo-keto reductase family 1 member C4 |
| GSTM2 | Glutathione S-transferase Mu 2 | Glutathione S-transferase Mu 2 |
| STRADA | STE20-related kinase adapter protein alpha | STE20-related kinase adapter protein alpha |
| BMPR2 | Bone morphogenetic protein receptor type-2 | Bone morphogenetic protein receptor type-2 |
| PIK3C2A | Phosphatidylinositol 4-phosphate 3-kinase C2 domain-containing subunit alpha | Phosphatidylinositol 4-phosphate 3-kinase C2 domain-containing subunit alpha |
| PTPN1 | Tyrosine-protein phosphatase non-receptor type 1 | Tyrosine-protein phosphatase non-receptor type 1 |
| IDE | Insulin-degrading enzyme | Insulin-degrading enzyme |
| CAPNS1 | Calpain small subunit 1 | Calpain small subunit 1 |
| GLO1 | Lactoylglutathione lyase | Lactoylglutathione lyase |
| UCHL1 | Ubiquitin carboxyl-terminal hydrolase isozyme L1 | Ubiquitin carboxyl-terminal hydrolase isozyme L1 |
| NPC1 | NPC intracellular cholesterol transporter 1 | NPC intracellular cholesterol transporter 1 |
| FES | Tyrosine-protein kinase Fes/Fps | Tyrosine-protein kinase Fes/Fps |
| CSNK1G1 | Casein kinase I isoform gamma-1 | Casein kinase I isoform gamma-1 |
| SLK | STE20-like serine/threonine-protein kinase | STE20-like serine/threonine-protein kinase |
| MAP2K7 | Dual specificity mitogen-activated protein kinase kinase 7 | Dual specificity mitogen-activated protein kinase kinase 7 |
| PIK3C3 | Phosphatidylinositol 3-kinase catalytic subunit type 3 | Phosphatidylinositol 3-kinase catalytic subunit type 3 |
| HAO1 | Hydroxyacid oxidase 1 | Hydroxyacid oxidase 1 |
| PASK | PAS domain-containing serine/threonine-protein kinase | PAS domain-containing serine/threonine-protein kinase |
| STK25 | Serine/threonine-protein kinase 25 | Serine/threonine-protein kinase 25 |
| KYNU | Kynureninase | Kynureninase |
| DUT | Deoxyuridine 5'-triphosphate | Deoxyuridine 5'-triphosphate |
| | nucleotidohydrolase, mitochondrial | nucleotidohydrolase, mitochondrial |
| PTPN22 | Tyrosine-protein phosphatase non-receptor type 22 | Tyrosine-protein phosphatase non-receptor type 22 |
| NEK7 | Serine/threonine-protein kinase Nek7 | Serine/threonine-protein kinase Nek7 |
| NNMT | Nicotinamide N-methyltransferase | Nicotinamide N-methyltransferase |
| NRP1 | Neuropilin-1 | Neuropilin-1 |
| RPS27 | 40S ribosomal protein S27 | 40S ribosomal protein S27 |
| DDR1 | Epithelial discoidin domain-containing receptor 1 | Epithelial discoidin domain-containing receptor 1 |
| LPL | Lipoprotein lipase | Lipoprotein lipase |
| ABCC1 | Multidrug resistance-associated protein 1 | Multidrug resistance-associated protein 1 |
| ASIC1 | Acid-sensing ion channel 1 | Acid-sensing ion channel 1 |
| SLC2A1 | Solute carrier family 2, facilitated glucose transporter member 1 | Solute carrier family 2, facilitated glucose transporter member 1 |
| PTPRS | Receptor-type tyrosine-protein phosphatase S | Receptor-type tyrosine-protein phosphatase S |
| ABCG2 | Broad substrate specificity ATP-binding cassette transporter ABCG2 | Broad substrate specificity ATP-binding cassette transporter ABCG2 |
| SLC1A3 | Excitatory amino acid transporter 1 | Excitatory amino acid transporter 1 |
| SLC1A1 | Excitatory amino acid transporter 3 | Excitatory amino acid transporter 3 |
| TRPM2 | Transient receptor potential cation channel subfamily M member 2 | Transient receptor potential cation channel subfamily M member 2 |
| GRM1 | Metabotropic glutamate receptor 1 | Metabotropic glutamate receptor 1 |
| MLKL | Mixed lineage kinase domain-like protein | Mixed lineage kinase domain-like protein |
| RAB7A | Ras-related protein Rab-7a | Ras-related protein Rab-7a |
| AHCYL1 | S-adenosylhomocysteine hydrolase-like protein 1 | S-adenosylhomocysteine hydrolase-like protein 1 |
| NAAA | N-acylethanolamine-hydrolyzing acid amidase | N-acylethanolamine-hydrolyzing acid amidase |
| CAMK2B | Calcium/calmodulin-dependent protein kinase type II subunit beta | Calcium/calmodulin-dependent protein kinase type II subunit beta |
| NT5E | 5'-nucleotidase | 5'-nucleotidase |
| CTSL | Procathepsin L | Procathepsin L |
| HSPA1A | Heat shock 70 kDa protein 1A | Heat shock 70 kDa protein 1A |
| FABPS | Fatty acid-binding protein 5 | Fatty acid-binding protein 5 |
| CTSC | Dipeptidyl peptidase 1 | Dipeptidyl peptidase 1 |
| ACVR2A | Activin receptor type-2A | Activin receptor type-2A |
| MMP14 | Matrix metalloproteinase-14 | Matrix metalloproteinase-14 |
| HSP90B1 | Endoplasmin | Endoplasmin |
| GLB1 | Beta-galactosidase | Beta-galactosidase |
| PTPRF | Receptor-type tyrosine-protein phosphatase F | Receptor-type tyrosine-protein phosphatase F |
| ST14 | Suppressor of tumorigenicity 14 protein | Suppressor of tumorigenicity 14 protein |
| PKN2 | Serine/threonine-protein kinase N2 | Serine/threonine-protein kinase N2 |
| ARG1 | Arginase-1 | Arginase-1 |
| PGAM1 | Phosphoglycerate mutase 1 | Phosphoglycerate mutase 1 |
| PPPICA | Serine/threonine-protein phosphatase PP1-alpha catalytic subunit | Serine/threonine-protein phosphatase PP1-alpha catalytic subunit |
| PTP4A2 | Protein tyrosine phosphatase type IVA 2 | Protein tyrosine phosphatase type IVA 2 |
| MAST1 | Microtubule-associated serine/threonine-protein kinase 1 | Microtubule-associated serine/threonine-protein kinase 1 |
| PI4KB | Phosphatidylinositol 4-kinase beta | Phosphatidylinositol 4-kinase beta |
| VRK2 | Serine/threonine-protein kinase VRK2 | Serine/threonine-protein kinase VRK2 |
| SCD | Stearoyl-CoA desaturase | Stearoyl-CoA desaturase |
| NUDT1 | 7,8-dihydro-8-oxoguanine triphosphatase | 7,8-dihydro-8-oxoguanine triphosphatase |
| CSNK1G2 | Casein kinase I isoform gamma-2 | Casein kinase I isoform gamma-2 |
| EBP | 3-beta-hydroxysteroid-Delta(8),Delta(7)-isomerase | 3-beta-hydroxysteroid-Delta(8),Delta(7)-isomerase |
| EIF2AK3 | Eukaryotic translation initiation factor 2-alpha kinase 3 | Eukaryotic translation initiation factor 2-alpha kinase 3 |
| ATP2A2 | Sarcoplasmic/endoplasmic reticulum calcium ATPase 2 | Sarcoplasmic/endoplasmic reticulum calcium ATPase 2 |
| MAN1B1 | Endoplasmic reticulum mannosyl-oligosaccharide 1,2-alpha-mannosidase | Endoplasmic reticulum mannosyl-oligosaccharide 1,2-alpha-mannosidase |
| CDKLS | Cyclin-dependent kinase-like 5 | Cyclin-dependent kinase-like 5 |
| MAP4K4 | Mitogen-activated protein kinase kinase kinase kinase 4 | Mitogen-activated protein kinase kinase kinase kinase 4 |
| DPP8 | Dipeptidyl peptidase 8 | Dipeptidyl peptidase 8 |
| MKNK1 | MAP kinase-interacting serine/threonine-protein kinase 1 | MAP kinase-interacting serine/threonine-protein kinase 1 |
| PREP | Prolyl endopeptidase | Prolyl endopeptidase |
| FKBPS | Peptidyl-prolyl cis-trans isomerase FKBPS | Peptidyl-prolyl cis-trans isomerase FKBPS |
| SIRT1 | NAD-dependent protein deacetylase sirtuin-1 | NAD-dependent protein deacetylase sirtuin-1 |
| STK3 | Serine/threonine-protein kinase 3 | Serine/threonine-protein kinase 3 |
| LIG1 | DNA ligase 1 | DNA ligase 1 |
| DUSP3 | Dual specificity protein phosphatase 3 | Dual specificity protein phosphatase 3 |
| CAMKK2 | Calcium/calmodulin-dependent protein kinase kinase 2 | Calcium/calmodulin-dependent protein kinase kinase 2 |
| PRKAB1 | 5'-AMP-activated protein kinase subunit beta-1 | 5'-AMP-activated protein kinase subunit beta-1 |
| MTR | Methionine synthase | Methionine synthase |
| UCHL3 | Ubiquitin carboxyl-terminal hydrolase isozyme L3 | Ubiquitin carboxyl-terminal hydrolase isozyme L3 |
| ESRRB | Steroid hormone receptor ERR2 | Steroid hormone receptor ERR2 |
| RIOK1 | Serine/threonine-protein kinase RIO1 | Serine/threonine-protein kinase RIO1 |
| TRAP1 | Heat shock protein 75 kDa, mitochondrial | Heat shock protein 75 kDa, mitochondrial |
| PIN1 | Peptidyl-prolyl cis-trans isomerase NIMA-interacting 1 | Peptidyl-prolyl cis-trans isomerase NIMA-interacting 1 |
| ALDH1A2 | Retinal dehydrogenase 2 | Retinal dehydrogenase 2 |
| PAK6 | Serine/threonine-protein kinase PAK 6 | Serine/threonine-protein kinase PAK 6 |
| AOX1 | Aldehyde oxidase | Aldehyde oxidase |
| DPP9 | Dipeptidyl peptidase 9 | Dipeptidyl peptidase 9 |
| ACP1 | Low molecular weight phosphotyrosine protein phosphatase | Low molecular weight phosphotyrosine protein phosphatase |
| PTPN7 | Tyrosine-protein phosphatase non-receptor type 7 | Tyrosine-protein phosphatase non-receptor type 7 |
| TLK2 | Serine/threonine-protein kinase tousled-like 2 | Serine/threonine-protein kinase tousled-like 2 |
| SMG1 | Serine/threonine-protein kinase SMG1 | Serine/threonine-protein kinase SMG1 |
| YWHAZ | 14-3-3 protein zeta/delta | 14-3-3 protein zeta/delta |
| ALDH1A1 | Retinal dehydrogenase 1 | Retinal dehydrogenase 1 |
| PHF8 | Histone lysine demethylase PHF8 | Histone lysine demethylase PHF8 |
| PRKAG1 | 5'-AMP-activated protein kinase subunit gamma-1 | 5'-AMP-activated protein kinase subunit gamma-1 |
| PRMT3 | Protein arginine N-methyltransferase 3 | Protein arginine N-methyltransferase 3 |
| SF3B3 | Splicing factor 3B subunit 3 | Splicing factor 3B subunit 3 |
| AKT1S1 | Proline-rich AKT1 substrate 1 | Proline-rich AKT1 substrate 1 |
| CHUK | Inhibitor of nuclear factor kappa-B kinase subunit alpha | Inhibitor of nuclear factor kappa-B kinase subunit alpha |
| RPL36A | 60S ribosomal protein L36a | 60S ribosomal protein L36a |
| RPL31 | 60S ribosomal protein L31 | 60S ribosomal protein L31 |
| RPS16 | 40S ribosomal protein S16 | 40S ribosomal protein S16 |
| RPS19 | 40S ribosomal protein S19 | 40S ribosomal protein S19 |
| RPS12 | 40S ribosomal protein S12 | 40S ribosomal protein S12 |
| RPL22 | 60S ribosomal protein L22 | 60S ribosomal protein L22 |
| RPS25 | 40S ribosomal protein S25 | 40S ribosomal protein S25 |
| RPL21 | 60S ribosomal protein L21 | 60S ribosomal protein L21 |
| RPL23 | 60S ribosomal protein L23 | 60S ribosomal protein L23 |
| RPS4Y1 | 40S ribosomal protein S4, Y isoform 1 | 40S ribosomal protein S4, Y isoform 1 |
| RPL36 | 60S ribosomal protein L36 | 60S ribosomal protein L36 |
| RPL27 | 60S ribosomal protein L27 | 60S ribosomal protein L27 |
| RPS15A | 40S ribosomal protein S15a | 40S ribosomal protein S15a |
| RPS11 | 40S ribosomal protein S11 | 40S ribosomal protein S11 |
| RPL13A | 60S ribosomal protein L13a | 60S ribosomal protein L13a |
| RPL37 | 60S ribosomal protein L37 | 60S ribosomal protein L37 |
| RPL7 | 60S ribosomal protein L7 | 60S ribosomal protein L7 |
| RPL29 | 60S ribosomal protein L29 | 60S ribosomal protein L29 |
| RPL27A | 60S ribosomal protein L27a | 60S ribosomal protein L27a |
| RPL35A | 60S ribosomal protein L35a | 60S ribosomal protein L35a |
| RPS26 | 40S ribosomal protein S26 | 40S ribosomal protein S26 |
| RPL37A | 60S ribosomal protein L37a | 60S ribosomal protein L37a |
| RPS29 | 40S ribosomal protein S29 | 40S ribosomal protein S29 |
| RPS28 | 40S ribosomal protein S28 | 40S ribosomal protein S28 |
| RPL17 | 60S ribosomal protein L17 | 60S ribosomal protein L17 |
| FAU | 40S ribosomal protein S30 | 40S ribosomal protein S30 |
| SLC2A3 | Solute carrier family 2, facilitated glucose transporter member 3 | Solute carrier family 2, facilitated glucose transporter member 3 |
| ASAH1 | Acid ceramidase | Acid ceramidase |
| BTN3A1 | Butyrophilin subfamily 3 member A1 | Butyrophilin subfamily 3 member A1 |
| NMT2 | Glycylpeptide N-tetradecanoyltransferase 2 | Glycylpeptide N-tetradecanoyltransferase 2 |
| PI4KA | Phosphatidylinositol 4-kinase alpha | Phosphatidylinositol 4-kinase alpha |
| GNG2 | Guanine nucleotide-binding protein G(I)/G(S)/G(O) subunit gamma-2 | Guanine nucleotide-binding protein G(I)/G(S)/G(O) subunit gamma-2 |
| GNA11 | Guanine nucleotide-binding protein subunit alpha-11 | Guanine nucleotide-binding protein subunit alpha-11 |
| PRMT8 | Protein arginine N-methyltransferase 8 | Protein arginine N-methyltransferase 8 |
| LYPLA1 | Acyl-protein thioesterase 1 | Acyl-protein thioesterase 1 |
| LGALS3 | Galectin-3 | Galectin-3 |
| UBE2D3 | Ubiquitin-conjugating enzyme E2 D3 | Ubiquitin-conjugating enzyme E2 D3 |
| S100A4 | Protein S100-A4 | Protein S100-A4 |
| LGALS9 | Galectin-9 | Galectin-9 |
| PIP4K2A | Phosphatidylinositol 5-phosphate 4-kinase type-2 alpha | Phosphatidylinositol 5-phosphate 4-kinase type-2 alpha |
| LGMN | Legumain | Legumain |
| GNB1 | Guanine nucleotide-binding protein G(I)/G(S)/G(T) subunit beta-1 | Guanine nucleotide-binding protein G(I)/G(S)/G(T) subunit beta-1 |
| HINT1 | Histidine triad nucleotide-binding protein 1 | Histidine triad nucleotide-binding protein 1 |
| FTO | Alpha-ketoglutarate-dependent dioxygenase FTO | Alpha-ketoglutarate-dependent dioxygenase FTO |
| BRPF1 | Peregrin | Peregrin |
| ST6GAL1 | Beta-galactoside alpha-2,6-sialyltransferase 1 | Beta-galactoside alpha-2,6-sialyltransferase 1 |
| INPPL1 | Phosphatidylinositol 3,4,5-trisphosphate 5-phosphatase 2 | Phosphatidylinositol 3,4,5-trisphosphate 5-phosphatase 2 |
| PI4K2B | Phosphatidylinositol 4-kinase type 2-beta | Phosphatidylinositol 4-kinase type 2-beta |
| PARP16 | Protein mono-ADP-ribosyltransferase PARP 16 | Protein mono-ADP-ribosyltransferase PARP16 |
| HEXA | Beta-hexosaminidase subunit alpha | Beta-hexosaminidase subunit alpha |
| SGPL1 | Sphingosine-1-phosphate lyase 1 | Sphingosine-1-phosphate lyase 1 |
| MGAT2 | Alpha-1,6-mannosyl-glycoprotein 2-beta-N-acetylglucosaminyltransferase | Alpha-1,6-mannosyl-glycoprotein 2-beta-N-acetylglucosaminyltransferase |
| LGALS8 | Galectin-8 | Galectin-8 |
| ASH2L | Set1/Ash2 histone methyltransferase complex subunit ASH2 | Set1/Ash2 histone methyltransferase complex subunit ASH2 |
| SARM1 | NAD(+) hydrolase SARM1 | NAD(+) hydrolase SARM1 |
| LYPLA2 | Acyl-protein thioesterase 2 | Acyl-protein thioesterase 2 |
| ASNS | Asparagine synthetase [glutamine-hydrolyzing] | Asparagine synthetase [glutamine-hydrolyzing] |
| PABPC1 | Polyadenylate-binding protein 1 | Polyadenylate-binding protein 1 |
| PRMTS | Protein arginine N-methyltransferase 5 | Protein arginine N-methyltransferase 5 |
| DNPH1 | 2'-deoxynucleoside 5'-phosphate N-hydrolase 1 | 2'-deoxynucleoside 5'-phosphate N-hydrolase 1 |
| WDR48 | WD repeat-containing protein 48 | WD repeat-containing protein 48 |
| WDR77 | Methylosome protein 50 | Methylosome protein 50 |
| ATAD2B | ATPase family AAA domain-containing protein 2B | ATPase family AAA domain-containing protein 2B |
| COPSS | COP9 signalosome complex subunit 5 | COP9 signalosome complex subunit 5 |
| PFKFB2 | 6-phosphofructo-2-kinase/fructose-2,6-bisphosphatase 2 | 6-phosphofructo-2-kinase/fructose-2,6-bisphosphatase 2 |
| KDM4B | Lysine-specific demethylase 4B | Lysine-specific demethylase 4B |
| PRKAB2 | 5'-AMP-activated protein kinase subunit beta-2 | 5'-AMP-activated protein kinase subunit beta-2 |
| MEN1 | Menin | Menin |
| PMM2 | Phosphomannomutase 2 | Phosphomannomutase 2 |
| RPTOR | Regulatory-associated protein of mTOR | Regulatory-associated protein of mTOR |
| MLST8 | Target of rapamycin complex subunit LST8 | Target of rapamycin complex subunit LST8 |
| DTYMK | Thymidylate kinase | Thymidylate kinase |
| PTPN9 | Tyrosine-protein phosphatase non-receptor type 9 | Tyrosine-protein phosphatase non-receptor type 9 |
| SHMT2 | Serine hydroxymethyltransferase, mitochondrial | Serine hydroxymethyltransferase, mitochondrial |
| PARG | Poly(ADP-ribose) glycohydrolase | Poly(ADP-ribose) glycohydrolase |
| GLS | Glutaminase kidney isoform, mitochondrial | Glutaminase kidney isoform, mitochondrial |
| APAF1 | Apoptotic protease-activating factor 1 | Apoptotic protease-activating factor 1 |
| KSR2 | Kinase suppressor of Ras 2 | Kinase suppressor of Ras 2 |
| SORD | Sorbitol dehydrogenase | Sorbitol dehydrogenase |
| SMS | Spermine synthase | Spermine synthase |
| GALE | UDP-glucose 4-epimerase | UDP-glucose 4-epimerase |
| PGD | 6-phosphogluconate dehydrogenase, decarboxylating | 6-phosphogluconate dehydrogenase, decarboxylating |
| HNMT | Histamine N-methyltransferase | Histamine N-methyltransferase |
| QDPR | Dihydropteridine reductase | Dihydropteridine reductase |
| GSTK1 | Glutathione S-transferase kappa 1 | Glutathione S-transferase kappa 1 |
| SRM | Spermidine synthase | Spermidine synthase |
| FARS2 | Phenylalanine--tRNA ligase, mitochondrial | Phenylalanine--tRNA ligase, mitochondrial |
| GNAI3 | Guanine nucleotide-binding protein G(i) subunit alpha-3 | Guanine nucleotide-binding protein G(i) subunit alpha-3 |
| CLTC | Clathrin heavy chain 1 | Clathrin heavy chain 1 |
| RHOC | Rho-related GTP-binding protein RhoC | Rho-related GTP-binding protein RhoC |
| DNM1 | Dynamin-1 | Dynamin-1 |
| GALNT10 | Polypeptide N-acetylgalactosaminyltransferase 10 | Polypeptide N-acetylgalactosaminyltransferase 10 |
| MTHFD1 | C-1-tetrahydrofolate synthase, cytoplasmic | C-1-tetrahydrofolate synthase, cytoplasmic |
| RAB2A | Ras-related protein Rab-2A | Ras-related protein Rab-2A |
| USP5 | Ubiquitin carboxyl-terminal hydrolase 5 | Ubiquitin carboxyl-terminal hydrolase 5 |
| GDA | Guanine deaminase | Guanine deaminase |
| UCK2 | Uridine-cytidine kinase 2 | Uridine-cytidine kinase 2 |
| HIF1AN | Hypoxia-inducible factor 1-alpha inhibitor | Hypoxia-inducible factor 1-alpha inhibitor |
| MDH2 | Malate dehydrogenase, mitochondrial | Malate dehydrogenase, mitochondrial |
| CYP2E1 | Cytochrome P450 2E1 | Cytochrome P450 2E1 |
| YWHAG | 14-3-3 protein gamma | 14-3-3 protein gamma |
| GFPT1 | Glutamine-fructose-6-phosphate aminotransferase [isomerizing] 1 | Glutamine-fructose-6-phosphate aminotransferase [isomerizing] 1 |
| FN1 | Fibronectin | Fibronectin |
| PLAUR | Urokinase plasminogen activator surface receptor | Urokinase plasminogen activator surface receptor |
| NCS1 | Neuronal calcium sensor 1 | Neuronal calcium sensor 1 |
| GNAI1 | Guanine nucleotide-binding protein G(i) subunit alpha-1 | Guanine nucleotide-binding protein G(i) subunit alpha-1 |
| HCN2 | Potassium/sodium hyperpolarization-activated cyclic nucleotide-gated channel 2 | Potassium/sodium hyperpolarization-activated cyclic nucleotide-gated channel 2 |
| QSOX1 | Sulfhydryl oxidase 1 | Sulfhydryl oxidase 1 |
| TLR1 | Toll-like receptor 1 | Toll-like receptor 1 |
| USP14 | Ubiquitin carboxyl-terminal hydrolase 14 | Ubiquitin carboxyl-terminal hydrolase 14 |
| KSR1 | Kinase suppressor of Ras 1 | Kinase suppressor of Ras 1 |
| RAB9A | Ras-related protein Rab-9A | Ras-related protein Rab-9A |
| CCR7 | C-C chemokine receptor type 7 | C-C chemokine receptor type 7 |
| PLCG2 | 1-phosphatidylinositol 4,5-bisphosphate phosphodiesterase gamma-2 | 1-phosphatidylinositol 4,5-bisphosphate phosphodiesterase gamma-2 |
| GFER | FAD-linked sulfhydryl oxidase ALR | FAD-linked sulfhydryl oxidase ALR |
| PTPRG | Receptor-type tyrosine-protein phosphatase gamma | Receptor-type tyrosine-protein phosphatase gamma |
| FUT8 | Alpha-(1,6)-fucosyltransferase | Alpha-(1,6)-fucosyltransferase |
| POR | NADPH-cytochrome P450 reductase | NADPH-cytochrome P450 reductase |
| RECQL | ATP-dependent DNA helicase Q1 | ATP-dependent DNA helicase Q1 |
| UNG | Uracil-DNA glycosylase | Uracil-DNA glycosylase |
| CAD | CAD protein [Includes: Glutamine-dependent carbamoyl-phosphate synthase | CAD protein [Includes: Glutamine-dependent carbamoyl-phosphate synthase |
| NCOA3 | Nuclear receptor coactivator 3 | Nuclear receptor coactivator 3 |
| CTDSP1 | Carboxy-terminal domain RNA polymerase II polypeptide A small phosphatase 1 | Carboxy-terminal domain RNA polymerase II polypeptide A small phosphatase 1 |
| KDM1B | Lysine-specific histone demethylase 1B | Lysine-specific histone demethylase 1B |
| TEAD1 | Transcriptional enhancer factor TEF-1 | Transcriptional enhancer factor TEF-1 |
| DDO | D-aspartate oxidase | D-aspartate oxidase |
| CD4 | T-cell surface glycoprotein CD4 | T-cell surface glycoprotein CD4 |
| SLC1A5 | Neutral amino acid transporter B(0) | Neutral amino acid transporter B(0) |
| CD81 | CD81 antigen | CD81 antigen |
| GGT1 | Glutathione hydrolase 1 proenzyme | Glutathione hydrolase 1 proenzyme |
| ADIPOR1 | Adiponectin receptor protein 1 | Adiponectin receptor protein 1 |
| RAP1A | Ras-related protein Rap-1A | Ras-related protein Rap-1A |
| RAB29 | Ras-related protein Rab-7L1 | Ras-related protein Rab-7L1 |
| GOPC | Golgi-associated PDZ and coiled-coil motif-containing protein | Golgi-associated PDZ and coiled-coil motif-containing protein |
| UTRN | Utrophin | Utrophin |
| GNAO1 | Guanine nucleotide-binding protein G(o) subunit alpha | Guanine nucleotide-binding protein G(o) subunit alpha |
| DDOST | Dolichyl-diphosphooligosaccharide--protein glycosyltransferase 48 kDa subunit | Dolichyl-diphosphooligosaccharide--protein glycosyltransferase 48 kDa subunit |
| PYGB | Glycogen phosphorylase, brain form | Glycogen phosphorylase, brain form |
| RABGGTB | Geranylgeranyl transferase type-2 subunit beta | Geranylgeranyl transferase type-2 subunit beta |
| VAV1 | Proto-oncogene vav | Proto-oncogene vav |
| SDHA | Succinate dehydrogenase [ubiquinone] flavoprotein subunit, mitochondrial | Succinate dehydrogenase [ubiquinone] flavoprotein subunit, mitochondrial |
| QTRT1 | Queuine tRNA-ribosyltransferase catalytic subunit 1 | Queuine tRNA-ribosyltransferase catalytic subunit 1 |
| CISD2 | CDGSH iron-sulfur domain-containing protein 2 | CDGSH iron-sulfur domain-containing protein 2 |
| DHPS | Deoxyhypusine synthase | Deoxyhypusine synthase |
| PPP2R5A | Serine/threonine-protein phosphatase 2A 56 kDa regulatory subunit alpha isoform | Serine/threonine-protein phosphatase 2A 56 kDa regulatory subunit alpha isoform |
| PANK2 | Pantothenate kinase 2, mitochondrial | Pantothenate kinase 2, mitochondrial |
| NEDD8 | NEDD8 | NEDD8 |
| DOHH | Deoxyhypusine hydroxylase | Deoxyhypusine hydroxylase |
| UBE2M | NEDD8-conjugating enzyme Ubc12 | NEDD8-conjugating enzyme Ubc12 |
| RNASEL | 2-5A-dependent ribonuclease | 2-5A-dependent ribonuclease |
| SYNJ1 | Synaptoj anin-1 | Synaptoj anin-1 |
| DUS2 | tRNA-dihydrouridine(20) synthase [NAD(P)+]-like | tRNA-dihydrouridine(20) synthase [NAD(P)+]-like |
| CYCS | Cytochrome c | Cytochrome c |
| PPP1CC | Serine/threonine-protein phosphatase PP1-gamma catalytic subunit | Serine/threonine-protein phosphatase PP1-gamma catalytic subunit |
| HMBS | Porphobilinogen deaminase | Porphobilinogen deaminase |
| PNPO | Pyridoxine-5'-phosphate oxidase | Pyridoxine-5'-phosphate oxidase |
| PPIF | Peptidyl-prolyl cis-trans isomerase F, mitochondrial | Peptidyl-prolyl cis-trans isomerase F, mitochondrial |
| DPP7 | Dipeptidyl peptidase 2 | Dipeptidyl peptidase 2 |
| MARK1 | Serine/threonine-protein kinase MARK1 | Serine/threonine-protein kinase MARK1 |
| PRCP | Lysosomal Pro-X carboxypeptidase | Lysosomal Pro-X carboxypeptidase |
| PIP4K2C | Phosphatidylinositol 5-phosphate 4-kinase type-2 gamma | Phosphatidylinositol 5-phosphate 4-kinase type-2 gamma |
| AKR1A1 | Aldo-keto reductase family 1 member A1 | Aldo-keto reductase family 1 member A1 |
| ME1 | NADP-dependent malic enzyme | NADP-dependent malic enzyme |
| ACACA | Acetyl-CoA carboxylase 1 | Acetyl-CoA carboxylase 1 |
| CD74 | HLA class II histocompatibility antigen gamma chain | HLA class II histocompatibility antigen gamma chain |
| NFATC1 | Nuclear factor of activated T-cells, cytoplasmic 1 | Nuclear factor of activated T-cells, cytoplasmic 1 |
| RUNX1 | Runt-related transcription factor 1 | Runt-related transcription factor 1 |
| PTPRC | Receptor-type tyrosine-protein phosphatase C | Receptor-type tyrosine-protein phosphatase C |
| CAPN2 | Calpain-2 catalytic subunit | Calpain-2 catalytic subunit |
| RB1 | Retinoblastoma-associated protein | Retinoblastoma-associated protein |
| ITGA5 | Integrin alpha-5 | Integrin alpha-5 |
| ITGA2 | Integrin alpha-2 | Integrin alpha-2 |
| ADAM10 | Disintegrin and metalloproteinase domain-containing protein 10 | Disintegrin and metalloproteinase domain-containing protein 10 |
| DAGLB | Diacylglycerol lipase-beta | Diacylglycerol lipase-beta |
| SLC16A1 | Monocarboxylate transporter 1 | Monocarboxylate transporter 1 |
| ABCC2 | ATP-binding cassette sub-family C member 2 | ATP-binding cassette sub-family C member 2 |
| SCARB1 | Scavenger receptor class B member 1 | Scavenger receptor class B member 1 |
| DAGLA | Diacylglycerol lipase-alpha | Diacylglycerol lipase-alpha |
| SLC47A1 | Multidrug and toxin extrusion protein 1 | Multidrug and toxin extrusion protein 1 |
| PIK3C2B | Phosphatidylinositol 4-phosphate 3-kinase C2 domain-containing subunit beta | Phosphatidylinositol 4-phosphate 3-kinase C2 domain-containing subunit beta |
| GBA2 | Non-lysosomal glucosylceramidase | Non-lysosomal glucosylceramidase |
| NCEH1 | Neutral cholesterol ester hydrolase 1 | Neutral cholesterol ester hydrolase 1 |
| WNK2 | Serine/threonine-protein kinase WNK2 | Serine/threonine-protein kinase WNK2 |
| LIPE | Hormone-sensitive lipase | Hormone-sensitive lipase |
| SLC27A1 | Long-chain fatty acid transport protein 1 | Long-chain fatty acid transport protein 1 |
| PTPRA | Receptor-type tyrosine-protein phosphatase | Receptor-type tyrosine-protein phosphatase |
| | alpha | alpha |
| PTPN2 | Tyrosine-protein phosphatase non-receptor type 2 | Tyrosine-protein phosphatase non-receptor type 2 |
| ITGB5 | Integrin beta-5 | Integrin beta-5 |
| CTSH | Pro-cathepsin H [Cleaved into: Cathepsin H mini chain; Cathepsin H | Pro-cathepsin H [Cleaved into: Cathepsin H mini chain; Cathepsin H |
| MAN2B1 | Lysosomal alpha-mannosidase | Lysosomal alpha-mannosidase |
| ITPR2 | Inositol 1,4,5-trisphosphate receptor type 2 | Inositol 1,4,5-trisphosphate receptor type 2 |
| ITPR3 | Inositol 1,4,5-trisphosphate receptor type 3 | Inositol 1,4,5-trisphosphate receptor type 3 |
| ABHD12 | Lysophosphatidylserine lipase ABHD12 | Lysophosphatidylserine lipase ABHD12 |
| CTSZ | Cathepsin Z | Cathepsin Z |
| SLC6A12 | Sodium- and chloride-dependent betaine transporter | Sodium- and chloride-dependent betaine transporter |
| SLC6A11 | Sodium- and chloride-dependent GABA transporter 3 | Sodium- and chloride-dependent GABA transporter 3 |
| KCNN3 | Small conductance calcium-activated potassium channel protein 3 | Small conductance calcium-activated potassium channel protein 3 |
| SLC27A4 | Long-chain fatty acid transport protein 4 | Long-chain fatty acid transport protein 4 |
| PTPN6 | Tyrosine-protein phosphatase non-receptor type 6 | Tyrosine-protein phosphatase non-receptor type 6 |
| AMPD3 | AMP deaminase 3 | AMP deaminase 3 |
| PLEC | Plectin | Plectin |
| PLD1 | Phospholipase D1 | Phospholipase D1 |
| PAM | Peptidyl-glycine alpha-amidating monooxygenase | Peptidyl-glycine alpha-amidating monooxygenase |
| PRKD2 | Serine/threonine-protein kinase D2 | Serine/threonine-protein kinase D2 |
| TYROS | Tyrosine-protein kinase receptor TYROS | Tyrosine-protein kinase receptor TYROS |
| RYR2 | Ryanodine receptor 2 | Ryanodine receptor 2 |
| PIP5K1C | Phosphatidylinositol 4-phosphate 5-kinase type-1 gamma | Phosphatidylinositol 4-phosphate 5-kinase type-1 gamma |
| LIPA | Lysosomal acid lipase/cholesteryl ester hydrolase | Lysosomal acid lipase/cholesteryl ester hydrolase |
| PPIB | Peptidyl-prolyl cis-trans isomerase B | Peptidyl-prolyl cis-trans isomerase B |
| ATP2A3 | Sarcoplasmic/endoplasmic reticulum calcium ATPase 3 | Sarcoplasmic/endoplasmic reticulum calcium ATPase 3 |
| CPT1A | Carnitine O-palmitoyltransferase 1, liver isoform | Carnitine O-palmitoyltransferase 1, liver isoform |
| ICMT | Protein-S-isoprenylcysteine O-methyltransferase | Protein-S-isoprenylcysteine O-methyltransferase |
| HSD17B7 | 3-keto-steroid reductase/17-beta-hydroxysteroid dehydrogenase 7 | 3-keto-steroid reductase/17-beta-hydroxysteroid dehydrogenase 7 |
| ELOVL6 | Elongation of very long chain fatty acids protein 6 | Elongation of very long chain fatty acids protein 6 |
| DHCR7 | 7-dehydrocholesterol reductase | 7-dehydrocholesterol reductase |
| ATP2A1 | Sarcoplasmic/endoplasmic reticulum calcium ATPase 1 | Sarcoplasmic/endoplasmic reticulum calcium ATPase 1 |
| RIOK3 | Serine/threonine-protein kinase RI03 | Serine/threonine-protein kinase RI03 |
| HSPB1 | Heat shock protein beta-1 | Heat shock protein beta-1 |
| PTPN12 | Tyrosine-protein phosphatase non-receptor type 12 | Tyrosine-protein phosphatase non-receptor type 12 |
| PLAA | Phospholipase A-2-activating protein | Phospholipase A-2-activating protein |
| MPI | Mannose-6-phosphate isomerase | Mannose-6-phosphate isomerase |
| MATK | Megakaryocyte-associated tyrosine-protein kinase | Megakaryocyte-associated tyrosine-protein kinase |
| UBA6 | Ubiquitin-like modifier-activating enzyme 6 | Ubiquitin-like modifier-activating enzyme 6 |
| MAP2K3 | Dual specificity mitogen-activated protein kinase kinase 3 | Dual specificity mitogen-activated protein kinase kinase 3 |
| RPS6KA2 | Ribosomal protein S6 kinase alpha-2 | Ribosomal protein S6 kinase alpha-2 |
| MAP3K4 | Mitogen-activated protein kinase kinase kinase 4 | Mitogen-activated protein kinase kinase kinase 4 |
| PARP4 | Protein mono-ADP-ribosyltransferase PARP4 | Protein mono-ADP-ribosyltransferase PARP4 |
| EIF4H | Eukaryotic translation initiation factor 4H | Eukaryotic translation initiation factor 4H |
| TESK1 | Dual specificity testis-specific protein kinase 1 | Dual specificity testis-specific protein kinase 1 |
| MLX | Max-like protein X | Max-like protein X |
| NEK6 | Serine/threonine-protein kinase Nek6 | Serine/threonine-protein kinase Nek6 |
| ALDH1B1 | Aldehyde dehydrogenase X, mitochondrial | Aldehyde dehydrogenase X, mitochondrial |
| ATM | Serine-protein kinase ATM | Serine-protein kinase ATM |
| CDYL | Chromodomain Y-like protein | Chromodomain Y-like protein |
| TAOK1 | Serine/threonine-protein kinase TAO1 | Serine/threonine-protein kinase TAO1 |
| STAT6 | Signal transducer and activator of transcription 6 | Signal transducer and activator of transcription 6 |
| MGMT | Methylated-DNA--protein-cysteine methyltransferase | Methylated-DNA--protein-cysteine methyltransferase |
| PPID | Peptidyl-prolyl cis-trans isomerase D | Peptidyl-prolyl cis-trans isomerase D |
| STATSB | Signal transducer and activator of transcription 5B | Signal transducer and activator of transcription 5B |
| TXNRD2 | Thioredoxin reductase 2, mitochondrial | Thioredoxin reductase 2, mitochondrial |
| TXNRD3 | Thioredoxin reductase 3 | Thioredoxin reductase 3 |
| STK39 | STE20/SPS1-related proline-alanine-rich protein kinase | STE20/SPS1-related proline-alanine-rich protein kinase |
| STK38L | Serine/threonine-protein kinase 38-like | Serine/threonine-protein kinase 38-like |
| CDC42BPA | Serine/threonine-protein kinase MRCK alpha | Serine/threonine-protein kinase MRCK alpha |
| ATG4B | Cysteine protease ATG4B | Cysteine protease ATG4B |
| DCLK2 | Serine/threonine-protein kinase DCLK2 | Serine/threonine-protein kinase DCLK2 |
| TUBAL3 | Tubulin alpha chain-like 3 | Tubulin alpha chain-like 3 |
| MAP3K3 | Mitogen-activated protein kinase kinase kinase 3 | Mitogen-activated protein kinase kinase kinase 3 |
| PPME1 | Protein phosphatase methylesterase 1 | Protein phosphatase methylesterase 1 |
| RPLP1 | 60S acidic ribosomal protein P1 | 60S acidic ribosomal protein P1 |
| GSDMD | Gasdermin-D | Gasdermin-D |
| C9 | Complement component C9 [Cleaved into: Complement component C9a; Complement component C9b] | Complement component C9 [Cleaved into: Complement component C9a; Complement component C9b] |
| HMGB1 | High mobility group protein B1 | High mobility group protein B 1 |
| DAB2IP | Disabled homolog 2-interacting protein | Disabled homolog 2-interacting protein |
| PHLPP1 | PH domain leucine-rich repeat-containing protein phosphatase 1 | PH domain leucine-rich repeat-containing protein phosphatase 1 |
| PHKA2 | Phosphorylase b kinase regulatory subunit alpha, liver isoform | Phosphorylase b kinase regulatory subunit alpha, liver isoform |
| PHKA1 | Phosphorylase b kinase regulatory subunit alpha, skeletal muscle isoform | Phosphorylase b kinase regulatory subunit alpha, skeletal muscle isoform |
| PHKB | Phosphorylase b kinase regulatory subunit beta | Phosphorylase b kinase regulatory subunit beta |
| EWSR1 | RNA-binding protein EWS | RNA-binding protein EWS |
| SLC16A3 | Monocarboxylate transporter 4 | Monocarboxylate transporter 4 |
| PTPRE | Receptor-type tyrosine-protein phosphatase epsilon | Receptor-type tyrosine-protein phosphatase epsilon |
| NEU1 | Sialidase-1 | Sialidase-1 |
| PCSK6 | Proprotein convertase subtilisin/kexin type 6 | Proprotein convertase subtilisin/kexin type 6 |
| ANTXR2 | Anthrax toxin receptor 2 | Anthrax toxin receptor 2 |
| STIM1 | Stromal interaction molecule 1 | Stromal interaction molecule 1 |
| DNM2 | Dynamin-2 | Dynamin-2 |
| SDHB | Succinate dehydrogenase [ubiquinone] iron-sulfur subunit, mitochondrial | Succinate dehydrogenase [ubiquinone] iron-sulfur subunit, mitochondrial |
| MAPKAP1 | Target of rapamycin complex 2 subunit MAPKAP1 | Target of rapamycin complex 2 subunit MAPKAP1 |
| PTGES2 | Prostaglandin E synthase 2 | Prostaglandin E synthase 2 |
| TMEM97 | Sigma intracellular receptor 2 | Sigma intracellular receptor 2 |
| SPPL2A | Signal peptide peptidase-like 2A | Signal peptide peptidase-like 2A |
| SLC7A11 | Cystine/glutamate transporter | Cystine/glutamate transporter |
| ABHD6 | Monoacylglycerol lipase ABHD6 | Monoacylglycerol lipase ABHD6 |
| DGKA | Diacylglycerol kinase alpha | Diacylglycerol kinase alpha |
| NAPRT | Nicotinate phosphoribosyltransferase | Nicotinate phosphoribosyltransferase |
| BAX | Apoptosis regulator BAX | Apoptosis regulator BAX |
| SPTLC1 | Serine palmitoyltransferase 1 | Serine palmitoyltransferase 1 |
| SPTLC2 | Serine palmitoyltransferase 2 | Serine palmitoyltransferase 2 |
| MGAT1 | Alpha-1,3-mannosyl-glycoprotein 2-beta-N-acetylglucosaminyltransferase | Alpha-1,3-mannosyl-glycoprotein 2-beta-N-acetylglucosaminyltransferase |
| EPHX1 | Epoxide hydrolase 1 | Epoxide hydrolase 1 |
| FADS1 | Acyl-CoA | Acyl-CoA |
| ABHDS | 1-acylglycerol-3-phosphate O-acyltransferase ABHDS | 1-acylglycerol-3-phosphate O-acyltransferase ABHDS |
| CBFB | Core-binding factor subunit beta | Core-binding factor subunit beta |
| POLR1A | DNA-directed RNA polymerase I subunit RPA1 | DNA-directed RNA polymerase I subunit RPA1 |
| MPG | DNA-3-methyladenine glycosylase | DNA-3-methyladenine glycosylase |
| GYS1 | Glycogen [starch] synthase, muscle | Glycogen [starch] synthase, muscle |
| PRKAG2 | 5'-AMP-activated protein kinase subunit gamma-2 | 5'-AMP-activated protein kinase subunit gamma-2 |
| ASF1A | Histone chaperone ASF1A | Histone chaperone ASF1A |
| MED23 | Mediator of RNA polymerase II transcription subunit 23 | Mediator of RNA polymerase II transcription subunit 23 |
| PFKFB4 | 6-phosphofructo-2-kinase/fructose-2,6-bisphosphatase 4 | 6-phosphofructo-2-kinase/fructose-2,6-bisphosphatase 4 |
| KDM3A | Lysine-specific demethylase 3A | Lysine-specific demethylase 3A |
| USP9X | Probable ubiquitin carboxyl-terminal hydrolase FAF-X | Probable ubiquitin carboxyl-terminal hydrolase FAF-X |
| MAX | Protein max | Protein max |
| MBD2 | Methyl-CpG-binding domain protein 2 | Methyl-CpG-binding domain protein 2 |
| CBX8 | Chromobox protein homolog 8 | Chromobox protein homolog 8 |
| FLI1 | Friend leukemia integration 1 transcription factor | Friend leukemia integration 1 transcription factor |
| ATRIP | ATR-interacting protein | ATR-interacting protein |
| RICTOR | Rapamycin-insensitive companion of mTOR | Rapamycin-insensitive companion of mTOR |
| UBLCP1 | Ubiquitin-like domain-containing CTD phosphatase 1 | Ubiquitin-like domain-containing CTD phosphatase 1 |
| DCTPP1 | dCTP pyrophosphatase 1 | dCTP pyrophosphatase 1 |
| TCF4 | Transcription factor 4 | Transcription factor 4 |
| CNOT7 | CCR4-NOT transcription complex subunit 7 | CCR4-NOT transcription complex subunit 7 |
| OGFRL1 | Opioid growth factor receptor-like protein 1 | Opioid growth factor receptor-like protein 1 |
| MVD | Diphosphomevalonate decarboxylase | Diphosphomevalonate decarboxylase |
| IL6ST | Interleukin-6 receptor subunit beta | Interleukin-6 receptor subunit beta |
| DOCK2 | Dedicator of cytokinesis protein 2 | Dedicator of cytokinesis protein 2 |
| C3 | Complement C3 | Complement C3 |
| PPM1A | Protein phosphatase 1A | Protein phosphatase 1A |
| SHC1 | SHC-transforming protein 1 | SHC-transforming protein 1 |
| DVL2 | Segment polarity protein dishevelled homolog DVL-2 | Segment polarity protein dishevelled homolog DVL-2 |
| NPR3 | Atrial natriuretic peptide receptor 3 | Atrial natriuretic peptide receptor 3 |
| GSTM1 | Glutathione S-transferase Mu 1 | Glutathione S-transferase Mu 1 |
| PPP3CA | Serine/threonine-protein phosphatase 2B catalytic subunit alpha isoform | Serine/threonine-protein phosphatase 2B catalytic subunit alpha isoform |
| SLC12A2 | Solute carrier family 12 member 2 | Solute carrier family 12 member 2 |
| ABCC3 | ATP-binding cassette sub-family C member 3 | ATP-binding cassette sub-family C member 3 |
| ABCC4 | ATP-binding cassette sub-family C member 4 | ATP-binding cassette sub-family C member 4 |
| GGH | Gamma-glutamyl hydrolase | Gamma-glutamyl hydrolase |
| HCN3 | Potassium/sodium hyperpolarization-activated cyclic nucleotide-gated channel 3 | Potassium/sodium hyperpolarization-activated cyclic nucleotide-gated channel 3 |
| ADCY1 | Adenylate cyclase type 1 | Adenylate cyclase type 1 |
| ADCY5 | Adenylate cyclase type 5 | Adenylate cyclase type 5 |
| CYP4F2 | Cytochrome P450 4F2 | Cytochrome P450 4F2 |
| HKDC1 | Hexokinase HKDC1 | Hexokinase HKDC 1 |
| PPT1 | Palmitoyl-protein thioesterase 1 | Palmitoyl-protein thioesterase 1 |
| PRNP | Major prion protein | Major prion protein |
| CXCRS | C-X-C chemokine receptor type 5 | C-X-C chemokine receptor type 5 |
| SMPD2 | Sphingomyelin phosphodiesterase 2 | Sphingomyelin phosphodiesterase 2 |
| SLC6A15 | Sodium-dependent neutral amino acid transporter B(0)AT2 | Sodium-dependent neutral amino acid transporter B(0)AT2 |
| MANBA | Beta-mannosidase | Beta-mannosidase |
| AGPAT2 | 1-acyl-sn-glycerol-3-phosphate acyltransferase beta | 1-acyl-sn-glycerol-3-phosphate acyltransferase beta |
| ABHD16A | Phosphatidylserine lipase ABHD16A | Phosphatidylserine lipase ABHD16A |
| GCLC | Glutamate-cysteine ligase catalytic subunit | Glutamate-cysteine ligase catalytic subunit |
| HAGH | Hydroxyacylglutathione hydrolase, mitochondrial | Hydroxyacylglutathione hydrolase, mitochondrial |
| SENP1 | Sentrin-specific protease 1 | Sentrin-specific protease 1 |
| DNAJA1 | DnaJ homolog subfamily A member 1 | DnaJ homolog subfamily A member 1 |
| SCP2 | Sterol carrier protein 2 | Sterol carrier protein 2 |
| AMPD2 | AMP deaminase 2 | AMP deaminase 2 |
| ERCC5 | DNA repair protein complementing XP-G cells | DNA repair protein complementing XP-G cells |
| SENP7 | Sentrin-specific protease 7 | Sentrin-specific protease 7 |
| RBPJ | Recombining binding protein suppressor of hairless | Recombining binding protein suppressor of hairless |
| USP47 | Ubiquitin carboxyl-terminal hydrolase 47 | Ubiquitin carboxyl-terminal hydrolase 47 |
| NADK | NAD kinase | NAD kinase |
| RBBP9 | Serine hydrolase RBBP9 | Serine hydrolase RBBP9 |
| CD2 | T-cell surface antigen CD2 | T-cell surface antigen CD2 |
| CD44 | CD44 antigen | CD44 antigen |
| SLC27A2 | Very long-chain acyl-CoA synthetase | Very long-chain acyl-CoA synthetase |
| PLEKHA1 | Pleckstrin homology domain-containing family A member 1 | Pleckstrin homology domain-containing family A member 1 |
| ITGA3 | Integrin alpha-3 | Integrin alpha-3 |
| CD47 | Leukocyte surface antigen CD47 | Leukocyte surface antigen CD47 |
| SPPL2B | Signal peptide peptidase-like 2B | Signal peptide peptidase-like 2B |
| PLTP | Phospholipid transfer protein | Phospholipid transfer protein |
| CD63 | CD63 antigen | CD63 antigen |
| SLC20A2 | Sodium-dependent phosphate transporter 2 | Sodium-dependent phosphate transporter 2 |
| ADCY6 | Adenylate cyclase type 6 | Adenylate cyclase type 6 |
| IGF2R | Cation-independent mannose-6-phosphate receptor | Cation-independent mannose-6-phosphate receptor |
| EZR | Ezrin | Ezrin |
| GNAQ | Guanine nucleotide-binding protein G(q) subunit alpha | Guanine nucleotide-binding protein G(q) subunit alpha |
| INPP5A | Inositol polyphosphate-5-phosphatase A | Inositol polyphosphate-5-phosphatase A |
| CRACR2A | EF-hand calcium-binding domain-containing protein 4B | EF-hand calcium-binding domain-containing protein 4B |
| LANCL2 | LanC-like protein 2 | LanC-like protein 2 |
| SMPD3 | Sphingomyelin phosphodiesterase 3 | Sphingomyelin phosphodiesterase 3 |
| EHD1 | EH domain-containing protein 1 | EH domain-containing protein 1 |
| PDIA6 | Protein disulfide-isomerase A6 | Protein disulfide-isomerase A6 |
| CHN2 | Beta-chimaerin | Beta-chimaerin |
| CDH2 | Cadherin-2 | Cadherin-2 |
| TFPI | Tissue factor pathway inhibitor | Tissue factor pathway inhibitor |
| CD58 | Lymphocyte function-associated antigen 3 | Lymphocyte function-associated antigen 3 |
| SLC2A8 | Solute carrier family 2, facilitated glucose transporter member 8 | Solute carrier family 2, facilitated glucose transporter member 8 |
| PTPRM | Receptor-type tyrosine-protein phosphatase mu | Receptor-type tyrosine-protein phosphatase mu |
| STIM2 | Stromal interaction molecule 2 | Stromal interaction molecule 2 |
| ADCY7 | Adenylate cyclase type 7 | Adenylate cyclase type 7 |
| SLC20A1 | Sodium-dependent phosphate transporter 1 | Sodium-dependent phosphate transporter 1 |
| ITGA1 | Integrin alpha-1 | Integrin alpha-1 |
| RABGGTA | Geranylgeranyl transferase type-2 subunit alpha | Geranylgeranyl transferase type-2 subunit alpha |
| MAP1B | Microtubule-associated protein 1B | Microtubule-associated protein 1B |
| PAFAH1B2 | Platelet-activating factor acetylhydrolase IB subunit alpha2 | Platelet-activating factor acetylhydrolase IB subunit alpha2 |
| RIN1 | Ras and Rab interactor 1 | Ras and Rab interactor 1 |
| PLCD1 | 1-phosphatidylinositol 4,5-bisphosphate phosphodiesterase delta-1 | 1-phosphatidylinositol 4,5-bisphosphate phosphodiesterase delta-1 |
| RGS19 | Regulator of G-protein signaling 19 | Regulator of G-protein signaling 19 |
| PNPLA2 | Patatin-like phospholipase domain-containing protein 2 | Patatin-like phospholipase domain-containing protein 2 |
| ST3GAL3 | CMP-N-acetylneuraminate-beta-1,4-galactoside alpha-2,3-sialyltransferase | CMP-N-acetylneuraminate-beta-1,4-galactoside alpha-2,3-sialyltransferase |
| GALNT1 | Polypeptide N-acetylgalactosaminyltransferase 1 | Polypeptide N-acetylgalactosaminyltransferase 1 |
| ATP6V1B2 | V-type proton ATPase subunit B, brain isoform | V-type proton ATPase subunit B, brain isoform |
| ATP6AP1 | V-type proton ATPase subunit S1 | V-type proton ATPase subunit S1 |
| PHLPP2 | PH domain leucine-rich repeat-containing protein phosphatase 2 | PH domain leucine-rich repeat-containing protein phosphatase 2 |
| GNPAT | Dihydroxyacetone phosphate acyltransferase | Dihydroxyacetone phosphate acyltransferase |
| PPP3CB | Serine/threonine-protein phosphatase 2B catalytic subunit beta isoform | Serine/threonine-protein phosphatase 2B catalytic subunit beta isoform |
| SLC25A20 | Mitochondrial carnitine/acylcarnitine carrier protein | Mitochondrial carnitine/acylcarnitine carrier protein |
| ELOVL1 | Elongation of very long chain fatty acids protein 1 | Elongation of very long chain fatty acids protein 1 |
| MAN2A1 | Alpha-mannosidase 2 | Alpha-mannosidase 2 |
| SLC33A1 | Acetyl-coenzyme A transporter 1 | Acetyl-coenzyme A transporter 1 |
| GAB1 | GRB2-associated-binding protein 1 | GRB2-associated-binding protein 1 |
| ARHGEF12 | Rho guanine nucleotide exchange factor 12 | Rho guanine nucleotide exchange factor 12 |
| MAP2 | Microtubule-associated protein 2 | Microtubule-associated protein 2 |
| MBTD1 | MBT domain-containing protein 1 | MBT domain-containing protein 1 |
| PNKP | Bifunctional polynucleotide phosphatase/kinase | Bifunctional polynucleotide phosphatase/kinase |
| NLRP1 | NACHT, LRR and PYD domains-containing protein 1 | NACHT, LRR and PYD domains-containing protein 1 |
| TXN2 | Thioredoxin, mitochondrial | Thioredoxin, mitochondrial |
| EEF1A2 | Elongation factor 1-alpha 2 | Elongation factor 1-alpha 2 |
| GOT1 | Aspartate aminotransferase, cytoplasmic | Aspartate aminotransferase, cytoplasmic |
| PRMT7 | Protein arginine N-methyltransferase 7 | Protein arginine N-methyltransferase 7 |
| MYH10 | Myosin-10 | Myosin-10 |
| TPP2 | Tripeptidyl-peptidase 2 | Tripeptidyl-peptidase 2 |
| EYA3 | Eyes absent homolog 3 | Eyes absent homolog 3 |
| SSU72 | RNA polymerase II subunit A C-terminal domain phosphatase SSU72 | RNA polymerase II subunit A C-terminal domain phosphatase SSU72 |
| AMDHD2 | N-acetylglucosamine-6-phosphate deacetylase | N-acetylglucosamine-6-phosphate deacetylase |
| CLPX | ATP-dependent Clp protease ATP-binding subunit clpX-like, mitochondrial | ATP-dependent Clp protease ATP-binding subunit clpX-like, mitochondrial |
| MECP2 | Methyl-CpG-binding protein 2 | Methyl-CpG-binding protein 2 |
| KPNA2 | Importin subunit alpha-1 | Importin subunit alpha-1 |
| PSMG1 | Proteasome assembly chaperone 1 | Proteasome assembly chaperone 1 |
| CBX6 | Chromobox protein homolog 6 | Chromobox protein homolog 6 |
| PPP2R2A | Serine/threonine-protein phosphatase 2A 55 kDa regulatory subunit B alpha isoform | Serine/threonine-protein phosphatase 2A 55 kDa regulatory subunit B alpha isoform |
| UBE2V1 | Ubiquitin-conjugating enzyme E2 variant 1 | Ubiquitin-conjugating enzyme E2 variant 1 |
| PSMG2 | Proteasome assembly chaperone 2 | Proteasome assembly chaperone 2 |
| PPIC | Peptidyl-prolyl cis-trans isomerase C | Peptidyl-prolyl cis-trans isomerase C |
| PGK2 | Phosphoglycerate kinase 2 | Phosphoglycerate kinase 2 |
| SOS1 | SOS Ras/Rac guanine nucleotide exchange factor 1 | SOS Ras/Rac guanine nucleotide exchange factor 1 |

In some embodiments, the PBM-R_{f}- can be a small molecule ligand that targets and binds to specific target proteins, including but not limited to, epidermal growth factor receptor (EGFR), Cyclin-dependent kinase 4/6 (CDK4/6), anaplastic lymphoma kinase (ALK), activin receptor-like kinase 2 (ALK2), fibroblast growth factor receptors (FGFRs), proto-oncogene tyrosine-protein kinase receptor RET (RET), Focal adhesion kinase (FAK), breakpoint cluster region (BCR)-Abelson leukemia virus (ABL) (BCR-ABL) tyrosine kinase, Bruton tyrosine kinase (BTK), Androgen receptor (AR), Estrogen receptor (ER), Bromodomain and extra-terminal domain protein (BET), Interleukin-1 receptor-associated kinase 4 (IRAK4), Cyclin-dependent kinase 9 (CDK9), Histone-lysine N-methyltransferase EZH2 (EZH2), neurotrophic receptor tyrosine kinase (NTRK), Src homology 2 domain containing protein tyrosine phosphatase (SHP2), Poly (ADP-ribose) polymerase (PARP), signal transducers and activators of transcription 3 (STAT3), FMS-like tyrosine kinase 3 (FLT3), B cell lymphoma-2 (BCL-2) family proteins, SOS Ras/Rac guanine nucleotide exchange factor 1 (SOS1), or GTPase Kras (KRAS).

In some embodiments, PBM comprises the structures of the following formula: 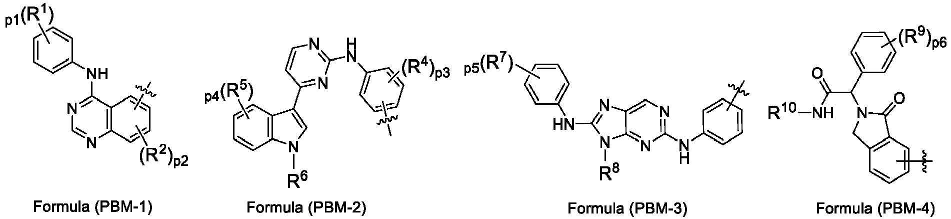 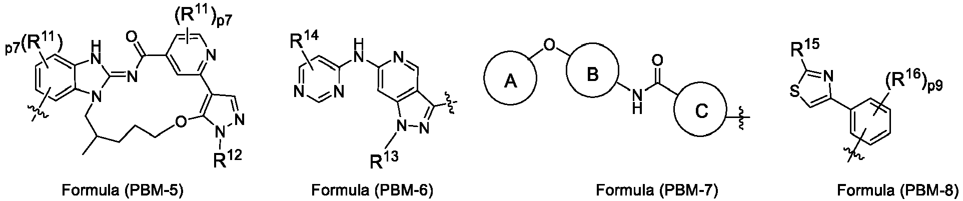 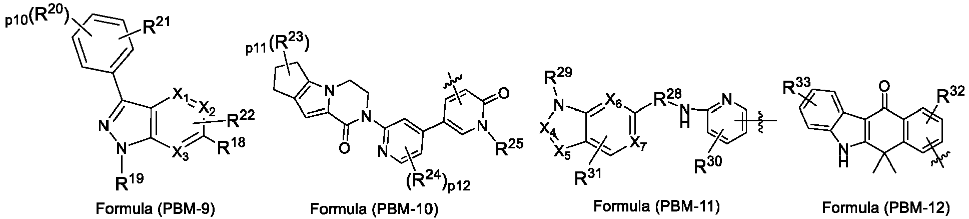 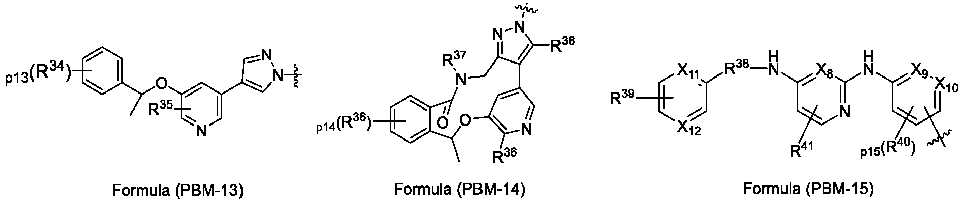 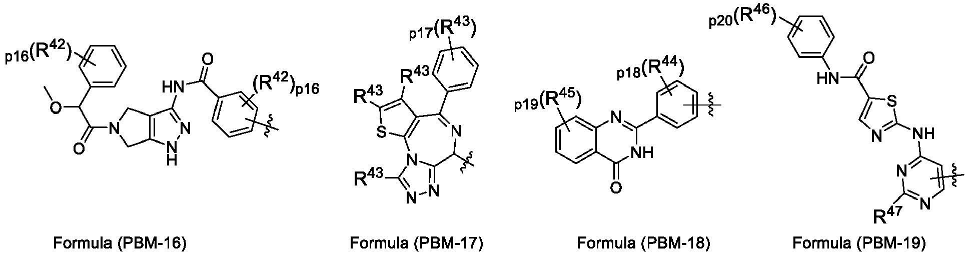 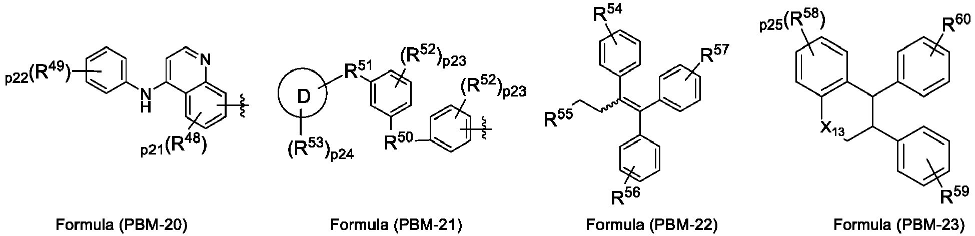 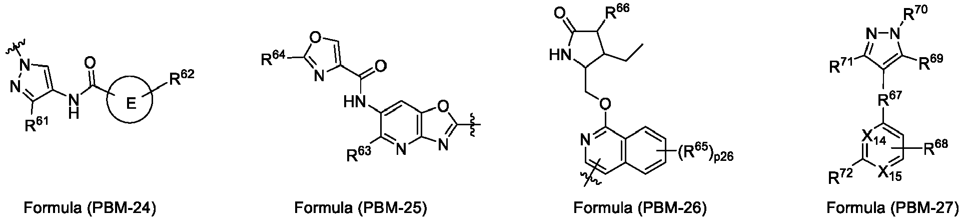 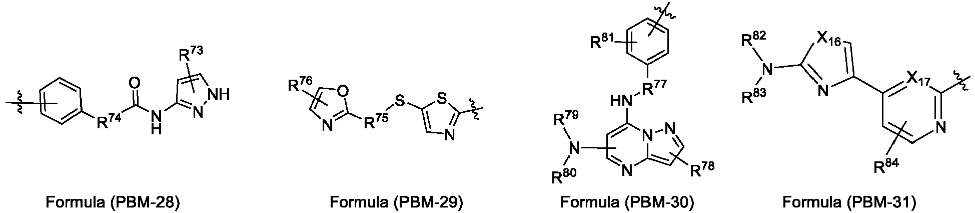 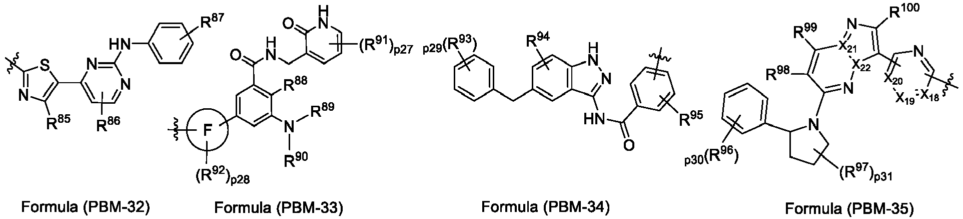 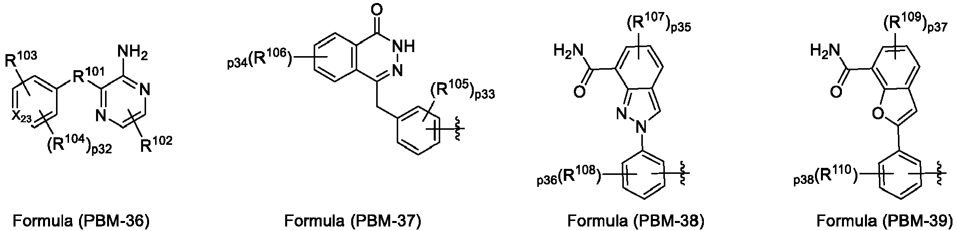 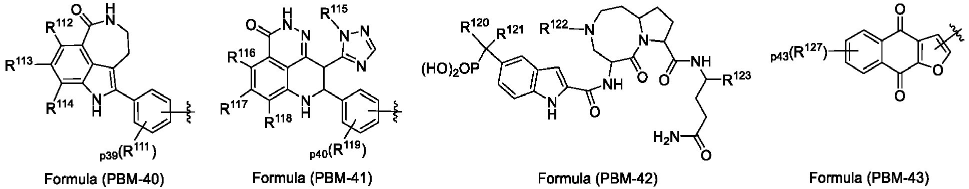 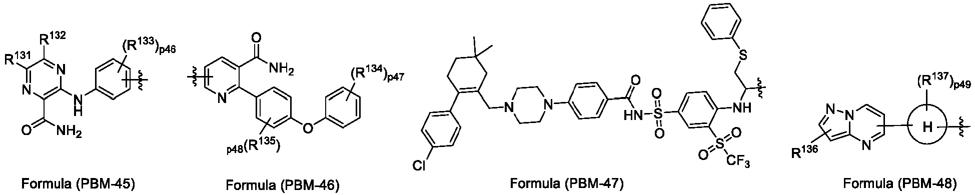 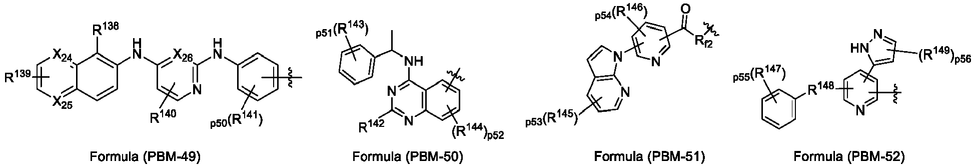 or 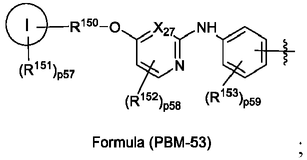 wherein
(R¹)ₚ₁ indicates that the benzene ring in Formula (PBM-1) is substituted with p1 R¹, with each R¹ being the same or different and independently representing halogen, hydroxy, NH₂, NO₂, cyano, C₁₋₆ alkyl, halogenated C₁₋₆ alkyl, deuterated C₁₋₆ alkyl, C₁₋₆ alkoxy, or halogenated C₁₋₆ alkoxy;
(R²)ₚ₂ indicates that the quinazoline ring in Formula (PBM-1) is substituted with p2 R², with each R² being the same or different and independently representing halogen, NO₂, cyano, halogenated C₁₋₆ alkyl, C₁₋₁₀ alkyl, deuterated C₁₋₁₀ alkyl, C₁₋₁₀ alkoxy, halogenated C₁₋₁₀ alkoxy, -O-optionally substituted heterocyclyl, or -NHC(O)R³, wherein R³ is C₁₋₁₀ alkyl, deuterated C₁₋₁₀ alkyl, or C₂₋₁₀ alkenyl, e.g., R² represents methyl, methoxy, 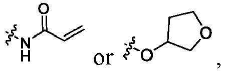 such as 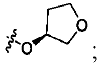
p1 represents an integer of 1, 2, 3, 4 or 5;
p2 represents an integer of 1, 2, or 3;
(R⁴)ₚ₃ indicates that the benzene ring in Formula (PBM-2) is substituted with p3 R⁴, with each R⁴ being the same or different and independently representing halogen, NO₂, cyano, halogenated C₁₋₆ alkyl, C₁₋₁₀ alkyl, deuterated C₁₋₁₀ alkyl, C₁₋₁₀ alkoxy, or -NHC(O)R^{15a}, where R^{15a} is C₁₋₁₀ alkyl, deuterated C₁₋₁₀ alkyl, or C₂₋₁₀ alkenyl, e.g., R⁴ represents methyl, methoxy, NO₂, or 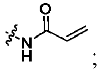
p3 represents an integer of 1, 2, 3 or 4;
(R⁵)ₚ₄ indicates that the 1H-indole ring in Formula (PBM-2) is substituted with p4 R⁵, with each R⁵ being the same or different and independently representing hydrogen, C₁₋₁₀ alkyl or deuterated C₁₋₁₀ alkyl, and p4 represents an integer of 0, 1, 2 or 3;
R⁶ represents hydrogen, C₁₋₁₀ alkyl, or deuterated C₁₋₁₀ alkyl;
(R⁷)ₚ₅ indicates that the benzene ring in Formula (PBM-3) is substituted with p5 R⁷, with each R⁷ being the same or different and independently representing halogen, hydroxy, C₁₋₆ alkyl, halogenated C₁₋₆ alkyl, deuterated C₁₋₆ alkyl, NO₂, NH₂, or cyano;
p5 represents an integer of 0, 1, 2, 3, 4 or 5;
R⁸ represents substituted or unsubstituted C₃₋₁₅ cycloalkyl, such as Cyclopentyl;
(R⁹)ₚ₆ indicates that the benzene ring in Formula (PBM-4) is substituted with p6 R⁹, with each R⁹ being the same or different and independently representing halogen, hydroxy, C₁₋₆ alkyl, halogenated C₁₋₆ alkyl, deuterated C₁₋₆ alkyl, NO₂, NH₂, C₁₋₆ alkoxy, halogenated C₁₋₆ alkoxy, or cyano;
p6 represents an integer of 2, 3, 4 or 5;
R¹⁰ represents C₆₋₁₀ aryl, or heteroaryl containing one or two 5- to 7-membered rings and 1 to 4 heteroatoms selected from nitrogen, oxygen, and sulfur, wherein the C₆₋₁₀ aryl and heteroaryl are each optionally substituted with one or more R^{b1}, with each R^{b1} being the same or different and independently representing halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
(R¹¹)ₚ₇ indicates that the benzene ring and the pyridine ring in Formula (PBM-5) are each optionally substituted with p7 R¹¹, with each p7 being the same or different and independently representing an integer of 0, 1, 2 or 3, and each R¹¹ in Formula (PBM-5) is the same or different and each independently represents hydrogen, halogen, C₁₋₆ alkyl, halogenated C₁₋₆ alkyl, or deuterated C₁₋₆ alkyl;
R¹² represents hydrogen, C₁₋₆ alkyl, halogenated C₁₋₆ alkyl, or deuterated C₁₋₆ alkyl;
R¹³ in Formula (PBM-6) represents hydrogen, C₁₋₁₀ alkyl (e.g., 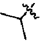 ), deuterated C₁₋₁₀ alkyl or halogenated C₁₋₁₀ alkyl;
R¹⁴ represents 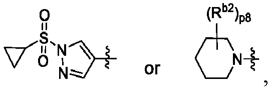 wherein (R^{b2})ₚ₈ indicates that the piperidine ring is optionally substituted with p8 R^{b2}, with each R^{b2} being the same or different and independently representing deuterium, halogen, hydroxy, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, NO₂, NH₂, or cyano, p8 represents an integer of 1, 2, 3, 4 or 5;
ring A in Formula (PBM-7) represents C₆₋₁₀ aryl or 5- to 20-membered monocyclic or bicyclic heteroaryl containing from 1 to 4 heteroatoms selected from the group consisting of oxygen, nitrogen, and sulfur, and the ring A is optionally substituted with one or more R^{b3}, with each R^{b3} being the same or different and independently representing deuterium, halogen, hydroxy, cyano, amino, nitro, C₁₋₆ alkyl, halogenated C₁₋₆ alkyl, deuterated C₁₋₆ alkyl, C₁₋₆ alkoxy, halogenated C₁₋₆ alkoxy, C₂₋₆ alkenyl, or C₂₋₆ alkynyl;
ring B in Formula (PBM-7) represents C₃₋₁₅ cycloalkyl or 3- to 20-membered heterocyclyl containing from 1 to 4 heteroatoms selected from the group consisting of oxygen, nitrogen, and sulfur, and the ring B is optionally substituted with one or more R^{b4}, with each R^{b4} being the same or different and independently representing deuterium, halogen, C₁₋₆ alkyl, halogenated C₁₋₆ alkyl, deuterated C₁₋₆ alkyl, C₁₋₆ alkoxy, or halogenated C₁₋₆ alkoxy;
ring C in Formula (PBM-7) represents 5- to 20-membered monocyclic or bicyclic heteroaryl containing from 1 to 4 heteroatoms selected from the group consisting of oxygen, nitrogen, and sulfur, and the ring C is optionally substituted with one or more R^{b5}, with each R^{b5} being the same or different and independently representing deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
R¹⁵ in Formula (PBM-8) represents 4- to 20-membered heterocyclyl containing from 1 to 4 heteroatoms selected from the group consisting of oxygen, nitrogen, and sulfur, and the heterocyclyl is optionally substituted with one or more R^{b6}, with each R^{b6} being the same or different and independently representing halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
(R¹⁶)ₚ₉ indicates that the benzene ring in Formula (PBM-8) is substituted with p9 R¹⁶, with each R¹⁶ being the same or different and independently representing halogen, C₁₋₆ alkyl, halogenated C₁₋₆ alkyl, or deuterated C₁₋₆ alkyl, and p9 represents an integer of 1, 2, 3 or 4;
X₁ in Formula (PBM-9) represents N or CR^{b7}, wherein R^{b7} is hydrogen or amino, and X₂ represents N or CR¹⁷, wherein R¹⁷ is hydrogen or represents a bond, and X₃ represents CH or N;
R¹⁸ and R¹⁹ each independently represent hydrogen or a bond;
(R²⁰)ₚ₁₀ indicates that the benzene ring in Formula (PBM-9) is substituted with p10 R²⁰, with each R²⁰ being the same or different and independently representing -O-aryl, -O-heteroaryl, -C₁₋₃ alkylene-NHC(O)-heteroaryl, -C₁₋₃ alkylene-C(O)NH-heteroaryl or halogen, wherein the aryl and heteroaryl are each independently optionally substituted with one or more R^{b8}, with each R^{b8} being the same or different and independently representing deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
ρ10 represents an integer of 1, 2, 3 or 4;
R²¹ represents a bond, deuterium, hydrogen, halogen, C₁₋₆ alkyl, halogenated C₁₋₆ alkyl, deuterated C₁₋₆ alkyl, or C₁₋₆ alkoxy;
R²² represents hydrogen, halogen, cyano, or amino;
wherein R¹⁷, R¹⁸, R¹⁹, and R²¹ are not simultaneously hydrogen, and R¹⁷, R¹⁸, R¹⁹, and R²¹ do not simultaneously represent a bond, and only one of R¹⁷, R¹⁸, R¹⁹, and R²¹ represents a bond, wherein
   when R¹⁸ represents a bond, the carbon atom on the ring connected to R¹⁸ is directly connected to R_{f}, X₂ represents CH or N, and R¹⁹ is hydrogen, and R²¹ is not a bond;
   when X₂ represents CR¹⁷', where R¹⁷ represents a bond, the carbon atom on the ring connected to R¹⁷ is directly connected to R_{f}, and R¹⁸ and R¹⁹ are hydrogen, and R²¹ is not a bond;
   when R¹⁹ represents a bond, the nitrogen atom on the ring connected to R¹⁹ is directly connected to R_{f}, X₂ represents N or CH, and R¹⁸ is hydrogen, and R²¹ is not a bond; and
   when R²¹ represents a bond, the carbon atom on the ring connected to R²¹ is directly connected to R_{f}, and X₂ represents N or CH, and R¹⁸ and R¹⁹ are hydrogen;
(R²³)ₚ₁₁ indicates that the cyclopentene ring in Formula (PBM-10) is substituted with p11 R²³, with each R²³ being the same or different and independently representing halogen, C₁₋₆ alkyl, halogenated C₁₋₆ alkyl, or deuterated C₁₋₆ alkyl, and p11 represents an integer of 1, 2, 3, or 4;
(R²⁴)ₚ₁₂ indicates that the pyridine ring in Formula (PBM-10) is substituted with p12 R²⁴, with each R²⁴ being the same or different and independently representing halogen, -C₁₋₃ alkylene-OH, C₁₋₆ alkyl, halogenated C₁₋₆ alkyl, or deuterated C₁₋₆ alkyl, and p12 represents an integer of 1, 2, or 3;
R²⁵ represents hydrogen, C₁₋₆ alkyl, halogenated C₁₋₆ alkyl, or deuterated C₁₋₆ alkyl;
X₄ in Formula (PBM-11) represents CR²⁶ or a fragment 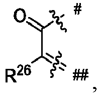 wherein symbol # indicates the point of attachment to N atom adjacent to X₄, symbol ## indicates the point of attachment to X₅, and R²⁶ represents deuterium, hydrogen, halogen, C₁₋₆ alkyl, halogenated C₁₋₆ alkyl, deuterated C₁₋₆ alkyl, - C(O)-C₁₋₆ alkyl, -C(O)-deuterated C₁₋₆ alkyl, or -C(O)NR^{b9}R^{b10}, where R^{b9} and R^{b10} are the same or different and each independently represent hydrogen, C₁₋₆ alkyl or deuterated C₁₋₆ alkyl;
X₅ in Formula (PBM-11) represents N or CR²⁷, where R²⁷ represents hydrogen, deuterium, halogen, C₁₋₆ alkyl, deuterated C₁₋₆ alkyl, or halogenated C₁₋₆ alkyl;
X₆ and X₇ each independently represent CH or N;
R²⁸ represents a bond or optionally substituted heteroarylene (e.g., halogenated heteroarylene);
R²⁹ represents hydrogen, C₁₋₆ alkyl, halogenated C₁₋₆ alkyl, deuterated C₁₋₆ alkyl, or optionally substituted C₃₋₇ cycloalkyl;
R³⁰ and R³¹ are the same or different and each independently represent hydrogen, halogen, C₁₋₆ alkyl, halogenated C₁₋₆ alkyl, or deuterated C₁₋₆ alkyl;
R³² in Formula (PBM-12) represents hydrogen, halogen, C₁₋₆ alkyl, halogenated C₁₋₆ alkyl, or deuterated C₁₋₆ alkyl;
R³³ represents hydrogen, cyano, halogen, C₁₋₆ alkyl, halogenated C₁₋₆ alkyl, or deuterated C₁₋₆ alkyl;
(R³⁴)ₚ₁₃ indicates that the benzene ring in Formula (PBM-13) is substituted with p13 R³⁴, with each R³⁴ being the same or different and independently representing halogen, hydroxy, amino, cyano, C₁₋₆ alkyl, halogenated C₁₋₆ alkyl, or deuterated C₁₋₆ alkyl;
p13 represents an integer of 1, 2, 3, 4 or 5;
R³⁵ represents NR^{b11}R^{b12}, where R^{b11} and R^{b12} are the same or different and each independently represent hydrogen, C₁₋₆ alkyl, halogenated C₁₋₆ alkyl, or deuterated C₁₋₆ alkyl;
(R³⁶)ₚ₁₄ indicates that the benzene ring in Formula (PBM-14) is optionally substituted with p14 R³⁶, with each R³⁶ in Formula (PBM-14) being the same or different and independently representing halogen, amino, cyano, C₁₋₆ alkyl, halogenated C₁₋₆ alkyl, or deuterated C₁₋₆ alkyl, and p14 represents an integer of 1, 2 or 3;
R³⁷ represents hydrogen, C₁₋₆ alkyl, halogenated C₁₋₆ alkyl, or deuterated C₁₋₆ alkyl;
X₈, X₉, X₁₀, X₁₁, and X₁₂ in Formula (PBM-15) are the same or different and each independently represent N or CH;
R³⁸ represents a bond or optionally substituted methylene (e.g., halogenated methylene);
R³⁹ represents -C(O)NHR^{b13}, -NHC(O)-R^{b13}, -S(O)₂NHR^{b13}, -N(R^{b14})S(O)₂R^{b13}, -S(O)₂R^{b13} or - P(O)(R^{b13})₂, wherein each R^{b13} is the same or different and each independently represents C₁₋₆ alkyl or deuterated C₁₋₆ alkyl, and R^{b14} represents hydrogen or C₁₋₆ alkyl;
(R⁴⁰)ₚ₁₅ indicates that the 6-membered ring containing X₉ and X₁₀ in Formula (PBM-15) is optionally substituted with p15 R⁴⁰, with each R⁴⁰ being the same or different and independently representing halogen, C₁₋₆ alkoxy, C₁₋₆ alkyl, deuterated C₁₋₆ alkyl or halogenated C₁₋₆ alkyl;
p15 represents an integer of 0, 1, 2, 3 or 4;
R⁴¹ represents halogen, C₁₋₆ alkoxy, C₁₋₆ alkyl, deuterated C₁₋₆ alkyl or halogenated C₁₋₆ alkyl;
(R⁴²)ₚ₁₆ indicates that the benzene ring in Formula (PBM-16) is optionally substituted with p16 R⁴², with each R⁴² being the same or different and independently representing halogen, C₁₋₆ alkoxy, C₁₋₆ alkyl, deuterated C₁₋₆ alkyl or halogenated C₁₋₆ alkyl;
each p16 is the same or different and each independently represents an integer of 0, 1, 2, 3 or 4;
(R⁴³)ₚ₁₇ indicates that the benzene ring in Formula (PBM-17) is substituted with p17 R⁴³, with each R⁴³ being the same or different and independently representing halogen, C₁₋₆ alkoxy, C₁₋₆ alkyl, deuterated C₁₋₆ alkyl or halogenated C₁₋₆ alkyl, and p17 represents an integer of 0, 1, 2, 3 or 4;
(R⁴⁴)ₚ₁₈ indicates that the benzene ring in Formula (PBM-18) is optionally substituted with p18 R⁴⁴, with each R⁴⁴ being the same or different and independently representing halogen, C₁₋₆ alkoxy, C₁₋₆ alkyl, deuterated C₁₋₆ alkyl or halogenated C₁₋₆ alkyl;
p18 represents an integer of 0, 1, 2, 3 or 4;
(R⁴⁵)ₚ₁₉ indicates that the 4-oxo-3,4-dihydroquinazoline ring in Formula (PBM-18) is substituted with p19 R⁴⁵, with each R⁴⁵ being the same or different and independently representing halogen, C₁₋₆ alkoxy, C₁₋₆ alkyl, deuterated C₁₋₆ alkyl or halogenated C₁₋₆ alkyl;
p19 represents an integer of 1, 2, 3 or 4;
(R⁴⁶)ₚ₂₀ indicates that the benzene ring in Formula (PBM-19) is substituted with p20 R⁴⁶, with each R⁴⁶ being the same or different and independently representing halogen, C₁₋₆ alkoxy, C₁₋₆ alkyl, deuterated C₁₋₆ alkyl, or halogenated C₁₋₆ alkyl;
p20 represents an integer of 1, 2, 3, 4 or 5;
R⁴⁷ represents halogen, C₁₋₆ alkyl, deuterated C₁₋₆ alkyl, or halogenated C₁₋₆ alkyl;
(R⁴⁸)ₚ₂₁ indicates that the quinoline ring in Formula (PBM-20) is substituted with p21 R⁴⁸, with each R⁴⁸ being the same or different and independently representing halogen, cyano, C₁₋₆ alkoxy, C₁₋₆ alkyl, deuterated C₁₋₆ alkyl, or halogenated C₁₋₆ alkyl;
p21 represents an integer of 1, 2, 3 or 4;
(R⁴⁹)ₚ₂₂ indicates that the benzene ring in Formula (PBM-20) is substituted with p22 R⁴⁹, with each R⁴⁹ being the same or different and independently representing halogen, C₁₋₆ alkoxy, C₁₋₆ alkyl, deuterated C₁₋₆ alkyl, or halogenated C₁₋₆ alkyl;
p22 represents an integer of 2, 3 or 4;
R⁵⁰ in Formula (PBM-21) represents -NHC(O)- or -C(O)NH-;
R⁵¹ represents -NH- or ethynylene;
(R⁵²)ₚ₂₃ indicates that the benzene rings in Formula (PBM-21) are each optionally substituted with p23 R⁵², with each R⁵² being the same or different and independently representing halogen, C₁₋₆ alkoxy, C₁₋₆ alkyl, deuterated C₁₋₆ alkyl, or halogenated C₁₋₆ alkyl;
each p23 is the same or different and each independently represents an integer of 0, 1, 2, 3 or 4;
ring D in Formula (PBM-21) represents 5- to 20-membered monocyclic or bicyclic heteroaryl containing from 1 to 4 heteroatoms selected from the group consisting of oxygen, nitrogen, and sulfur, and (R⁵³)ₚ₂₄ indicates that the ring D is optionally substituted with p24 R⁵³, with each R⁵³ being the same or different and independently representing optionally substituted 5- to 15-membered heteroaryl, deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
p24 represents an integer of 0, 1, 2, or 3;
only one of R⁵⁴, R⁵⁵, R⁵⁶, and R⁵⁷ in Formula (PBM-22) represents a bond, and the remaining are the same or different and each independently represent hydrogen, halogen, or hydroxy, wherein when one of R⁵⁴, R⁵⁵, R⁵⁶ and R⁵⁷ represents a bond, the benzene ring or methylene in Formula (PBM-22) connected to the bond is directly connected to R_{f};
symbol " 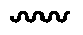 " connected to a double bond in Formula (PBM-22) represents a bond in stereo-configuration (cis or trans configuration, or E- or Z-configuration);
X₁₃ in Formula (PBM-23) represents -O- or -CH₂-;
(R⁵⁸)ₚ₂₅ indicates that 1,2,3,4-tetrahydronaphthalene ring in Formula (PBM-23) is substituted with p25 R⁵⁸, with each R⁵⁸ being the same or different and independently representing hydrogen, hydroxy, halogen, C₁₋₆ alkyl, halogenated C₁₋₆ alkyl, or deuterated C₁₋₆ alkyl;
p25 represents an integer of 1, 2, 3 or 4;
one of R⁵⁹ and R⁶⁰ represents a bond, and another represents hydrogen, halogen, C₁₋₆ alkyl, deuterated C₁₋₆ alkyl or halogenated C₁₋₆ alkyl, wherein when one of R⁵⁹ and R⁶⁰ represents a bond, the carbon atom on the ring in Formula (PBM-23) connected to the bond is directly connected to R_{f};
ring E in Formula (PBM-24) represents 5- to 20-membered monocyclic or bicyclic heteroarylene containing from 1 to 4 heteroatoms selected from the group consisting of oxygen, nitrogen, and sulfur;
R⁶² represents optionally substituted 5- to 15-membered heteroaryl, optionally substituted 5- to 15-membered heterocyclyl, deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
R⁶¹ represents halogen, C₁₋₆ alkyl, halogenated C₁₋₆ alkyl, or deuterated C₁₋₆ alkyl;
R⁶³ in Formula (PBM-25) represents optionally substituted 5- to 15-membered heterocyclyl, deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
R⁶⁴ represents 5- to 20-membered monocyclic or bicyclic heteroaryl containing from 1 to 4 heteroatoms selected from the group consisting of oxygen, nitrogen, and sulfur, and the heteroaryl is optionally substituted with one or more substituents selected from the group consisting of deuterium, halogen, hydroxy, cyano, amino, C₁₋₆ alkyl, halogenated C₁₋₆ alkyl, deuterated C₁₋₆ alkyl, C₁₋₆ alkoxy, halogenated C₁₋₆ alkoxy, or any combination thereof;
(R⁶⁵)ₚ₂₆ indicates that the isoquinoline ring in Formula (PBM-26) is substituted with p26 R⁶⁵, with each R⁶⁵ being the same or different and independently representing hydrogen, hydroxy, halogen, C₁₋₆ alkyl, C₁₋₆ alkoxy, -C(O)NH₂, deuterated C₁₋₆ alkyl, or halogenated C₁₋₆ alkyl;
p26 represents an integer of 1, 2, 3 or 4;
groups R⁶⁶ are the same or different and each independently represent hydrogen, hydroxy, halogen, C₁₋₆ alkyl, C₁₋₆ alkoxy, deuterated C₁₋₆ alkyl, or halogenated C₁₋₆ alkyl;
X₁₄ in Formula (PBM-27) represents N or CR^{b23}, where R^{b23} represents hydrogen or halogen, and X₁₅ represents N or CH;
R⁶⁷ represents a bond, -C(O)NH- or -NHC(O)-;
R⁶⁸ represents halogen, C₁₋₆ alkyl, deuterated C₁₋₆ alkyl, or halogenated C₁₋₆ alkyl;
R⁶⁹ represents hydrogen, C₁₋₆ alkyl, deuterated C₁₋₆ alkyl, halogenated C₁₋₆ alkyl, or -C₁₋₃ alkylene-C₃₋₆ cycloalkyl;
R⁷⁰ represents hydrogen, C₁₋₆ alkyl, deuterated C₁₋₆ alkyl, or halogenated C₁₋₆ alkyl; or
R⁶⁹ and R⁷⁰ together with the corresponding nitrogen and carbon atoms to which they are connected form an optionally substituted 5- to 7-membered nitrogen-containing heterocycle;
R⁷¹ represents hydrogen or a bond, R⁷² represents hydrogen or a bond, wherein R⁷¹ and R⁷² are not simultaneously hydrogen, and only one of R⁷¹ and R⁷² represents a bond, and another represents hydrogen, wherein when R⁷¹ represents a bond, the carbon atom on the ring connected to R⁷¹ is directly connected to R_{f}, and when R⁷² represents a bond, the carbon atom on the ring connected to R⁷² is directly connected to R_{f};
R⁷⁴ in Formula (PBM-28) represents C₁₋₃ alkylene or halogenated C₁₋₃ alkylene;
R⁷³ represents optionally substituted C₃₋₆ cycloalkyl, halogenated C₃₋₆ cycloalkyl, or optionally substituted C₁₋₆ alkyl;
R⁷⁵ in Formula (PBM-29) represents C₁₋₃ alkylene or halogenated C₁₋₃ alkylene;
R⁷⁶ represents C₁₋₆ alkyl, halogenated C₁₋₆ alkyl, deuterated C₁₋₆ alkyl, or optionally substituted C₃₋₆ cycloalkyl;
R⁷⁷ in Formula (PBM-30) represents C₁₋₃ alkylene or halogenated C₁₋₃ alkylene;
R⁷⁸ represents hydroxy, halogen, C₁₋₆ alkyl, C₁₋₆ alkoxy, deuterated C₁₋₆ alkyl, or halogenated C₁₋₆ alkyl;
R⁷⁹ and R⁸⁰ are the same or different and each independently represent hydrogen, optionally substituted C₁₋₁₀ alkyl or optionally substituted C₃₋₆ cycloalkyl;
R⁸¹ represents hydrogen, halogen, C₁₋₆ alkyl, deuterated C₁₋₆ alkyl, or halogenated C₁₋₆ alkyl;
X₁₆ in Formula (PBM-31) represents -S- or a fragment 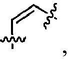 wherein when X₁₆ represents -S-, heteroaryl containing X₁₆ and nitrogen atom in Formula (PBM-31) is thiazolyl, and when X₁₆ represents the fragment 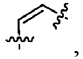 the heteroaryl containing X₁₆ and nitrogen atom in Formula (PBM-31) is pyridyl;
X₁₇ represents N or CH;
R⁸² represents hydrogen or optionally substituted C₁₋₁₀ alkyl;
R⁸³ represents hydrogen, -C₁₋₃ alkylene-optionally substituted 4- to 10-membered heterocyclyl, or optionally substituted 4- to 10-membered heterocyclyl;
R⁸⁴ represents hydroxy, halogen, C₁₋₆ alkyl, C₁₋₆ alkoxy, deuterated C₁₋₆ alkyl, or halogenated C₁₋₆ alkyl;
R⁸⁵ and R⁸⁶ in Formula (PBM-32) are the same or different and each independently represent hydroxy, halogen, C₁₋₆ alkyl, C₁₋₆ alkoxy, deuterated C₁₋₆ alkyl, or halogenated C₁₋₆ alkyl;
R⁸⁷ represents -S(O)₂NH₂ or -C(O)NH₂;
R⁸⁸ in Formula (PBM-33) represents hydrogen, hydroxy, halogen, C₁₋₆ alkyl, deuterated C₁₋₆ alkyl, or halogenated C₁₋₆ alkyl;
R⁸⁹ represents C₁₋₆ alkyl, deuterated C₁₋₆ alkyl, or halogenated C₁₋₆ alkyl; or
R⁸⁸ and R⁸⁹ together with the corresponding carbon and nitrogen atoms to which they are connected form an optionally substituted 4- to 6-membered heteroaromatic ring or optionally substituted 4- to 6-membered heterocycle;
R⁹⁰ represents optionally substituted C₃₋₆ cycloalkyl, C₁₋₆ alkyl, deuterated C₁₋₆ alkyl, halogenated C₁₋₆ alkyl or optionally substituted 4- to 10-membered heterocyclyl;
(R⁹¹)ₚ₂₇ indicates that pyridin-2(1H)-one ring in Formula (PBM-33) is substituted with p27 R⁹¹, with each R⁹¹ being the same or different and independently representing hydroxy, halogen, C₁₋₆ alkyl, C₁₋₆ alkoxy, deuterated C₁₋₆ alkyl, or halogenated C₁₋₆ alkyl;
p27 represents an integer of 1, 2 or 3;
ring F in Formula (PBM-33) represents arylene or 5- to 20-membered monocyclic or bicyclic heteroarylene containing from 1 to 4 heteroatoms selected from the group consisting of oxygen, nitrogen, and sulfur; and (R⁹²)ₚ₂₈ indicates that the ring F is optionally substituted with p28 R⁹², with each R⁹² being the same or different and independently representing deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
p28 represents an integer of 0, 1, 2, 3 or 4;
(R⁹³)ₚ₂₉ indicates that the benzene ring in Formula (PBM-34) is substituted with p29 R⁹³, with each R⁹³ being the same or different and independently representing hydroxy, amino, cyano, halogen, C₁₋₆ alkyl, C₁₋₆ alkoxy, deuterated C₁₋₆ alkyl, or halogenated C₁₋₆ alkyl;
p29 represents an integer of 1, 2, 3, 4 or 5;
R⁹⁴ represents hydrogen, hydroxy, amino, cyano, halogen, C₁₋₆ alkyl, C₁₋₆ alkoxy, deuterated C₁₋₆ alkyl, or halogenated C₁₋₆ alkyl;
R⁹⁵ represents NR^{b15}R^{b16}, where R^{b15} and R^{b16} are the same or different and each independently represent hydrogen, C₁₋₆ alkyl, deuterated C₁₋₆ alkyl, halogenated C₁₋₆ alkyl, optionally substituted C₃₋₆ cycloalkyl, or optionally substituted 4- to 8-membered heterocyclyl;
X₁₈, X₁₉ and X₂₀ in Formula (PBM-35) are the same or different and each independently represent CH or N, wherein X₁₈, X₁₉ and X₂₀ are not simultaneously N, and the 6-membered ring containing X₁₈, X₁₉, X₂₀ and nitrogen atom is optionally substituted with 1 or 2 substituents selected from the group consisting of halogen, cyano, C₁₋₆ alkyl, deuterated C₁₋₆ alkyl, halogenated C₁₋₆ alkyl, or any combination thereof;
X₂₁ and X₂₂ in Formula (PBM-35) are the same or different and each independently represent C or N, wherein X₂₁ and X₂₂ are not simultaneously N;
(R⁹⁶)ₚ₃₀ indicates that the benzene ring in Formula (PBM-35) is substituted with p30 R⁹⁶, with each R⁹⁶ being the same or different and independently representing hydroxy, amino, cyano, halogen, C₁₋₆ alkyl, C₁₋₆ alkoxy, deuterated C₁₋₆ alkyl, or halogenated C₁₋₆ alkyl;
p30 represents an integer of 1, 2, 3, 4 or 5;
(R⁹⁷)ₚ₃₁ indicates that the pyrrolidine ring in Formula (PBM-35) is optionally substituted with p31 R⁹⁷, with each R⁹⁷ being the same or different and independently representing hydrogen, hydroxy, amino, cyano, halogen, C₁₋₆ alkyl, C₁₋₆ alkoxy, deuterated C₁₋₆ alkyl, or halogenated C₁₋₆ alkyl;
p31 represents an integer of 0, 1, 2, 3, 4, 5 or 6;
R⁹⁸, R⁹⁹, and R¹⁰⁰ are the same or different and each independently represent hydrogen, halogen, C₁₋₆ alkyl, deuterated C₁₋₆ alkyl, or halogenated C₁₋₆ alkyl;
X₂₃ in Formula (PBM-36) represents CH or N;
R¹⁰¹ represents a bond or -S-;
R¹⁰² represents a bond or optionally substituted 4- to 15-membered nitrogen-containing heterocyclyl;
R¹⁰³ represents a bond, halogen, amino, C₁₋₆ alkyl, deuterated C₁₋₆ alkyl, halogenated C₁₋₆ alkyl, or optionally substituted 4- to 15-membered nitrogen-containing heterocyclyl;
wherein R¹⁰² and R¹⁰³ are not simultaneously a bond, and only one of R¹⁰² and R¹⁰³ is a bond, wherein when R¹⁰² represents a bond, the carbon atom on the ring connected to R¹⁰² is directly connected to R_{f}, and when R¹⁰³ represents a bond, the carbon atom on the ring connected to R¹⁰³ is directly connected to R_{f};
(R¹⁰⁴)ₚ₃₂ indicates that the 6-membered ring in Formula (PBM-36) is optionally substituted with p32 R¹⁰⁴, with each R¹⁰⁴ being the same or different and independently representing hydroxy, amino, cyano, halogen, C₁₋₆ alkyl, C₁₋₆ alkoxy, deuterated C₁₋₆ alkyl, or halogenated C₁₋₆ alkyl;
p32 represents an integer of 0, 1, 2, 3 or 4;
(R¹⁰⁵)ₚ₃₃ indicates that the benzene ring in Formula (PBM-37) is optionally substituted with p33 R¹⁰⁵, with each R¹⁰⁵ being the same or different and independently representing hydroxy, amino, cyano, halogen, C₁₋₆ alkyl, C₁₋₆ alkoxy, deuterated C₁₋₆ alkyl, or halogenated C₁₋₆ alkyl;
p33 represents an integer of 1, 2, 3 or 4;
(R¹⁰⁶)ₚ₃₄ indicates that the phthalazinone ring in Formula (PBM-37) is optionally substituted with p34 R¹⁰⁶, with each R¹⁰⁶ being the same or different and independently representing hydroxy, amino, cyano, halogen, C₁₋₆ alkyl, C₁₋₆ alkoxy, deuterated C₁₋₆ alkyl or halogenated C₁₋₆ alkyl;
p34 represents an integer of 0, 1, 2, 3 or 4;
(R¹⁰⁷)ₚ₃₅ indicates that the 2H-indazole ring in Formula (PBM-38) is optionally substituted with p35 R¹⁰⁷, with each R¹⁰⁷ being the same or different and independently representing hydroxy, amino, cyano, halogen, C₁₋₆ alkyl, C₁₋₆ alkoxy, deuterated C₁₋₆ alkyl or halogenated C₁₋₆ alkyl;
p35 represents an integer of 0, 1, 2 or 3;
(R¹⁰⁸)ₚ₃₆ indicates that the benzene ring in Formula (PBM-38) is optionally substituted with p36 R¹⁰⁸, with each R¹⁰⁸ being the same or different and independently representing hydroxy, amino, cyano, halogen, C₁₋₆ alkyl, C₁₋₆ alkoxy, deuterated C₁₋₆ alkyl or halogenated C₁₋₆ alkyl;
p36 represents an integer of 0, 1, 2 or 3;
(R¹⁰⁹)ₚ₃₇ indicates that the 2H-indazole ring in Formula (PBM-39) is optionally substituted with p37 R¹⁰⁹, with each R¹⁰⁹ being the same or different and independently representing hydroxy, amino, cyano, halogen, C₁₋₆ alkyl, C₁₋₆ alkoxy, deuterated C₁₋₆ alkyl or halogenated C₁₋₆ alkyl;
p37 represents an integer of 0, 1, 2 or 3;
(R¹¹⁰)ₚ₃₈ indicates that the benzene ring in Formula (PBM-39) is optionally substituted with p38 R¹¹⁰, with each R¹¹⁰ being the same or different and independently representing hydroxy, amino, cyano, halogen, C₁₋₆ alkyl, C₁₋₆ alkoxy, deuterated C₁₋₆ alkyl or halogenated C₁₋₆ alkyl;
p38 represents an integer of 0, 1, 2 or 3;
(R¹¹¹)ₚ₃₉ indicates that the benzene ring in Formula (PBM-40) is optionally substituted with p39 R¹¹¹, with each R¹¹¹ being the same or different and independently representing hydroxy, amino, cyano, halogen, C₁₋₆ alkyl, C₁₋₆ alkoxy, deuterated C₁₋₆ alkyl or halogenated C₁₋₆ alkyl;
p39 represents an integer of 0, 1, 2, 3 or 4;
R¹¹², R¹¹³, and R¹¹⁴ are the same or different and each independently represent hydrogen, halogen, C₁₋₆ alkyl, deuterated C₁₋₆ alkyl or halogenated C₁₋₆ alkyl;
R¹¹⁵ in Formula (PBM-41) represents hydrogen, C₁₋₆ alkyl, deuterated C₁₋₆ alkyl or halogenated C₁₋₆ alkyl;
R¹¹⁶, R¹¹⁷, and R¹¹⁸ are the same or different and each independently represent hydrogen, halogen, C₁₋₆ alkyl, deuterated C₁₋₆ alkyl or halogenated C₁₋₆ alkyl;
(R¹¹⁹)ₚ₄₀ indicates that the benzene ring in Formula (PBM-41) is optionally substituted with p40 R¹¹⁹, with each R¹¹⁹ being the same or different and independently representing hydroxy, amino, cyano, halogen, C₁₋₆ alkyl, C₁₋₆ alkoxy, deuterated C₁₋₆ alkyl or halogenated C₁₋₆ alkyl;
p40 represents an integer of 0, 1, 2, 3 or 4;
R¹²⁰ and R¹²¹ in Formula (PBM-42) are the same or different and each independently represent hydrogen, halogen, C₁₋₃ alkyl or halogenated C₁₋₃ alkyl;
R¹²² represents a bond, hydrogen, C₁₋₆ alkyl, deuterated C₁₋₆ alkyl or halogenated C₁₋₆ alkyl;
R¹²³ represents the structure of the following formula: 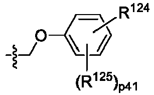
wherein R¹²⁴ represents a bond or hydrogen; and (R¹²⁵)ₚ₄₁ indicates that the benzene ring is optionally substituted with p41 R¹²⁵, with each R¹²⁵ being the same or different and independently representing hydroxy, amino, cyano, halogen, C₁₋₆ alkyl, C₁₋₆ alkoxy, deuterated C₁₋₆ alkyl or halogenated C₁₋₆ alkyl, and p41 represents an integer of 0, 1, 2, 3 or 4; or
R¹²³ represents the structure of the following formula: 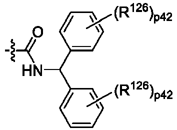
wherein (R¹²⁶)ₚ₄₂ indicates that the benzene ring is optionally substituted with p42 R¹²⁶, with each R¹²⁶ being the same or different and independently representing hydroxy, amino, cyano, halogen, C₁₋₆ alkyl, C₁₋₆ alkoxy, deuterated C₁₋₆ alkyl or halogenated C₁₋₆ alkyl, and p42 represents an integer of 0, 1, 2, 3, 4 or 5;
wherein R¹²² and R¹²⁴ are not simultaneously a bond, and only one of R¹²² and R¹²⁴ represents a bond, wherein when R¹²² represents a bond, the nitrogen atom on the ring connected to R¹²² is directly connected to R_{f}, and when R¹²⁴ represents a bond, the carbon atom on the ring connected to R¹²⁴ is directly connected to R_{f};
(R¹²⁷)ₚ₄₃ indicates that the benzene ring in Formula (PBM-43) is optionally substituted with p43 R¹²⁷, with each R¹²⁷ being the same or different and independently representing hydroxy, amino, cyano, halogen, C₁₋₆ alkyl, C₁₋₆ alkoxy, deuterated C₁₋₆ alkyl or halogenated C₁₋₆ alkyl;
p43 represents an integer of 0, 1, 2, 3 or 4;
R¹³¹ in Formula (PBM-45) represents C₁₋₆ alkyl, deuterated C₁₋₆ alkyl, halogenated C₁₋₆ alkyl or hydrogen;
R¹³² represents C₁₋₆ alkyl, deuterated C₁₋₆ alkyl, halogenated C₁₋₆ alkyl, optionally substituted C₃₋₆ cycloalkyl, optionally substituted 4- to 8-membered nitrogen-containing heterocyclyl or NR^{b17}R^{b18}, where R^{b17} and R^{b18} are the same or different and each independently represent hydrogen, C₁₋₆ alkyl, deuterated C₁₋₆ alkyl or optionally substituted 4- to 8-membered heterocyclyl;
(R¹³³)ₚ₄₆ indicates that the benzene ring in Formula (PBM-45) is optionally substituted with p46 R¹³³, with each R¹³³ being the same or different and independently representing hydroxy, amino, cyano, halogen, C₁₋₆ alkyl, C₁₋₆ alkoxy, deuterated C₁₋₆ alkyl or halogenated C₁₋₆ alkyl;
p46 represents an integer of 0, 1, 2, 3 or 4;
(R¹³⁴)ₚ₄₇ indicates that the benzene ring in Formula (PBM-46) is optionally substituted with p47 R¹³⁴, with each R¹³⁴ being the same or different and independently representing hydroxy, amino, cyano, halogen, C₁₋₆ alkyl, C₁₋₆ alkoxy, deuterated C₁₋₆ alkyl or halogenated C₁₋₆ alkyl;
p47 represents an integer of 0, 1, 2, 3 or 4;
(R¹³⁵)ₚ₄₈ indicates that the benzene ring in Formula (PBM-46) is optionally substituted with p48 R¹³⁵, with each R¹³⁵ being the same or different and independently representing hydroxy, amino, cyano, halogen, C₁₋₆ alkyl, C₁₋₆ alkoxy, deuterated C₁₋₆ alkyl or halogenated C₁₋₆ alkyl;
p48 represents an integer of 0, 1, 2, 3 or 4;
ring H in Formula (PBM-48) represents C₆₋₁₀ arylene or C₅₋₁₀ heteroarylene, and (R¹³⁷)ₚ₄₉ indicates that the ring H is optionally substituted with p49 R¹³⁷, with each R¹³⁷ being the same or different and independently representing deuterium, halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
p49 represents an integer of 0, 1, 2, 3 or 4;
R¹³⁶ represents optionally substituted 5- to 20-membered monocyclic or bicyclic heteroaryl containing from 1 to 4 heteroatoms selected from the group consisting of oxygen, nitrogen, and sulfur, which is optionally substituted with a substituent selected from the group consisting of hydroxy, amino, cyano, halogen, C₁₋₆ alkyl, C₁₋₆ alkoxy, deuterated C₁₋₆ alkyl or halogenated C₁₋₆ alkyl;
X₂₄, X₂₅, and X₂₆ in Formula (PBM-49) each independently represents CH or N;
R¹³⁸ represents -C(O)NHR^{b19}, -NHC(O)-R^{b19}, -S(O)₂NHR^{b19}, -NHS(O)₂R^{b19}, -S(O)₂R^{b19} or - P(O)(R^{b19})₂, wherein each R^{b19} is the same or different and each independently represents C₁₋₆ alkyl or deuterated C₁₋₆ alkyl;
R¹³⁹ and R¹⁴⁰ each independently represent halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
(R¹⁴¹)ₚ₅₀ indicates that the benzene ring is optionally substituted with p50 R¹⁴¹, with each R¹⁴¹ being independently halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
p50 represents an integer of 0, 1, 2, 3 or 4; and
(R¹⁴³)ₚ₅₁ in Formula (PBM-50) indicates that the benzene ring is optionally substituted with p51 R¹⁴³, with each R¹⁴³ being independently halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
p51 represents an integer of 0, 1, 2, 3, 4 or 5;
R¹⁴² represents H, C₁₋₆ alkyl or halogenated C₁₋₆ alkyl;
(R¹⁴⁴)ₚ₅₂ indicates that the quinazoline ring in Formula (PBM-50) is optionally substituted with p52 R¹⁴⁴, with each R¹⁴⁴ being independently halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and
p52 represents an integer of 1, 2, or 3;
R_{f2} in Formula (PBM-51) represents a bond, or R_{f2} represents -NH-R_{f3}-C(O)-***, wherein symbol *** indicates the point of attachment to R_{f1}, and R_{f3} represents optionally substituted C₃₋₈ cycloalkyl;
(R¹⁴⁵)ₚ₅₃ indicates that the 1H-pyrrolo[2,3-b]pyridine ring in Formula (PBM-51) is optionally substituted with p53 R¹⁴⁵, with each R¹⁴⁵ being independently cyano, halogen, hydroxy, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p53 represents an integer of 0, 1, 2, 3, 4 or 5;
(R¹⁴⁶)ₚ₅₄ indicates that the pyridine ring in Formula (PBM-51) is optionally substituted with p54 R¹⁴⁶, with each R¹⁴⁶ being independently cyano, halogen, hydroxy, nitro, C₁₋₆ alkyl, halogenated C₁₋₆ alkyl, deuterated C₁₋₆ alkyl, C₁₋₆ alkoxy, or halogenated C₁₋₆ alkoxy or NR^{b25}R^{b26}, wherein R^{b25} and R^{b26} are the same or different and each independently represent hydrogen, C₁₋₆ alkyl, deuterated C₁₋₆ alkyl, halogenated C₁₋₆ alkyl, optionally substituted C₃₋₆ cycloalkyl or optionally substituted 4- to 8-membered heterocyclyl, and p54 represents an integer of 0, 1, 2 or 3;
(R¹⁴⁷)ₚ₅₅ in Formula (PBM-52) indicates that the benzene ring is optionally substituted with p55 R¹⁴⁷, with each R¹⁴⁷ being independently halogen, hydroxy, cyano, amino, nitro, C₁₋₆ alkyl, halogenated C₁₋₆ alkyl, deuterated C₁₋₆ alkyl, C₁₋₆ alkoxy or halogenated C₁₋₆ alkoxy, and p55 represents an integer of 0, 1, 2, 3, 4 or 5;
R¹⁴⁸ represents NHC(O) or C(O)NH;
(R¹⁴⁹)ₚ₅₆ indicates that the 1H-pyrazole ring in Formula (PBM-52) is optionally substituted with p56 R¹⁴⁹, with each R¹⁴⁹ being independently halogen, hydroxy, cyano, amino, nitro, C₁₋₆ alkyl, halogenated C₁₋₆ alkyl, deuterated C₁₋₆ alkyl, C₁₋₆ alkoxy or halogenated C₁₋₆ alkoxy, and p56 represents an integer of 0, 1 or 2; and
X₂₇ in Formula (PBM-53) represents N or CH;
R¹⁵⁰ represents a bond or optionally substituted methylene (e.g., halogenated methylene);
ring I represents C₆₋₁₀ arylene or 5- to 15-membered heteroarylene, and (R¹⁵¹)ₚ₅₇ indicates that the ring I is optionally substituted with p57 R¹⁵¹, with each R¹⁵¹ being the same or different and independently representing deuterium, halogen, hydroxy, cyano, amino, nitro, oxo, C₁₋₆ alkyl, halogenated C₁₋₆ alkyl, deuterated C₁₋₆ alkyl, C₁₋₆ alkoxy, or halogenated C₁₋₆ alkoxy, and p57 represents an integer of 0, 1, 2, 3 or 4;
(R¹⁵²)ₚ₅₈ indicates that the 6-membered ring containing X₂₇ in Formula (PBM-53) is optionally substituted with p58 R¹⁵², with each R¹⁵² independently representing halogen, C₁₋₆ alkoxy, C₁₋₆ alkyl, deuterated C₁₋₆ alkyl or halogenated C₁₋₆ alkyl, and p58 represents an integer of 0, 1 or 2;
(R¹⁵³)ₚ₅₉ indicates that the benzene ring in Formula (PBM-53) is optionally substituted with p59 R¹⁵³, with each R¹⁵³ being the same or different and independently representing halogen, C₁₋₆ alkoxy, C₁₋₆ alkyl, deuterated C₁₋₆ alkyl or halogenated C₁₋₆ alkyl, and p15 represents an integer of 0, 1, 2, 3 or 4.

In some embodiments, PBM-R_{f}- represents a small molecule ligand targeting EGFR.

In some embodiments, PBM represents the structures of Formula (PBM-1-1A), Formula (PBM-1-1B), Formula (PBM-1-1C) or Formula (PBM-1-1D): 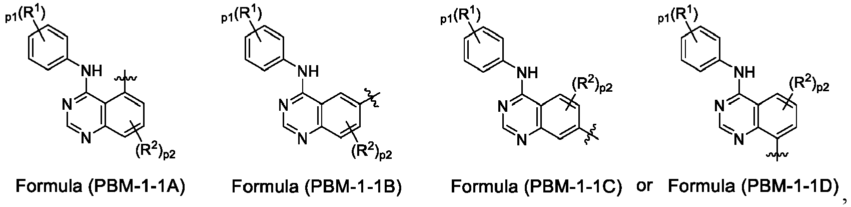 wherein in Formula (PBM-1-1A), Formula (PBM-1-1B), Formula (PBM-1-1C) and Formula (PBM-1-1D),
(R¹)ₚ₁ each independently indicates that the benzene ring is substituted with p1 R¹, with each R¹ being the same or different and independently representing halogen (e.g., fluorine, chlorine, bromine, or iodine), hydroxy, NH₂, NO₂, cyano, C₁₋₆ alkyl (e.g., C₁₋₄ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
(R²)ₚ₂ each independently indicates that the quinazoline ring is substituted with p2 R², with each R² being the same or different and independently representing halogen (e.g., fluorine, chlorine, bromine, or iodine), NO₂, cyano, halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₁₀ alkyl (e.g., C₁₋₈ alkyl, C₁₋₆ alkyl or C₁₋₄ alkyl, such as methyl, ethyl, propyl, isopropyl, butyl, sec-butyl, tert-butyl, pentyl, hexyl, heptyl, or octyl), deuterated C₁₋₁₀ alkyl (e.g., perdeuterated C₁₋₈ alkyl, perdeuterated C₁₋₆ alkyl or perdeuterated C₁₋₄ alkyl, such as CD₃, CD₃CD₂-, CD₃CD₂CD₂- etc.), C₁₋₁₀ alkoxy (e.g., C₁₋₈ alkoxy, C₁₋₆ alkoxy or C₁₋₄ alkoxy, such as methoxy, ethoxy, propoxy, isopropoxy, butoxy, sec-butoxy, or tert-butoxy), halogenated C₁₋₁₀ alkoxy (e.g., halogenated C₁₋₄ alkoxy, such as trifluoromethoxy), -O-optionally substituted heterocyclyl (where heterocyclyl is e.g., 4- to 20-membered monocyclic or bicyclic heterocyclyl containing one or more (e.g., from 1 to 4, or 1, 2, 3, or 4) heteroatoms selected from the group consisting of oxygen, nitrogen, and sulfur), or -NHC(O)R³, wherein R³ is C₁₋₁₀ alkyl (e.g., C₁₋₈ alkyl, C₁₋₆ alkyl or C₁₋₄ alkyl, such as methyl, ethyl, propyl, isopropyl, butyl, sec-butyl, tert-butyl, pentyl, hexyl, heptyl, or octyl), deuterated C₁₋₁₀ alkyl (e.g., perdeuterated C₁₋₈ alkyl, perdeuterated C₁₋₆ alkyl or perdeuterated C₁₋₄ alkyl, such as CD₃, CD₃CD₂-, CD₃CD₂CD₂- etc.), or C₂₋₁₀ alkenyl (e.g., C₂₋₆ alkenyl or C₂₋₄ alkenyl, such as vinyl, propenyl, butenyl or pentenyl), e.g., R² represents methyl, methoxy, 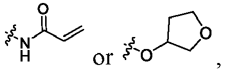 such as 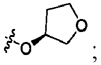
p1 represents an integer of 1, 2, 3, 4 or 5; and
p2 represents an integer of 1, 2, or 3.

In some embodiments, R² represents -O-optionally substituted heterocyclyl, and the heterocyclyl is 4- to 20-membered (e.g., 4- to 15-membered, 4- to 12-membered, 4- to 10-membered, 4- to 7-membered, or 4- to 6-membered) monocyclic or bicyclic heterocyclyl group containing one or more (e.g., 1-4, or 1, 2, 3, or 4) heteroatoms selected from nitrogen, oxygen, and sulfur, and includes but is not limited to azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), diazabicyclo[2.2.2]octanyl (e.g., 2,5-diazabicyclo[2.2.2]octan-2-yl). In some embodiments, the heterocyclyl is optionally substituted with a substituent selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

In some embodiments, R² represents:
fluorine, chlorine, bromine, or iodine;
methyl, ethyl, propyl, isopropyl, butyl, sec-butyl, tert-butyl, pentyl, hexyl, heptyl or octyl;
methoxy, ethoxy, propoxy, isopropoxy, butoxy, sec-butoxy, or tert-butoxy; -NHC(O)-C₂₋₁₀ alkenyl, e.g., -NHC(O)-vinyl (i.e., 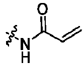 ), -NHC(O)-propenyl, or -NHC(O)-butenyl; or

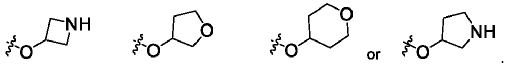

In some embodiments, PBM represents the structures of Formula (PBM-1-1), Formula (PBM-1-2), Formula (PBM-1-3), Formula (PBM-1-4) or Formula (PBM-1-5):

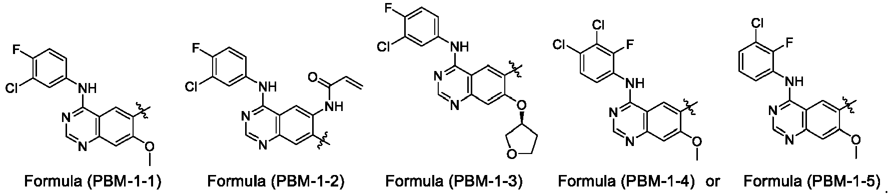

In some embodiments, PBM represents the structures of Formula (PBM-2-1A), Formula (PBM-2-1B), Formula (PBM-2-1C), Formula (PBM-2-1D) or Formula (PBM-2-1E): 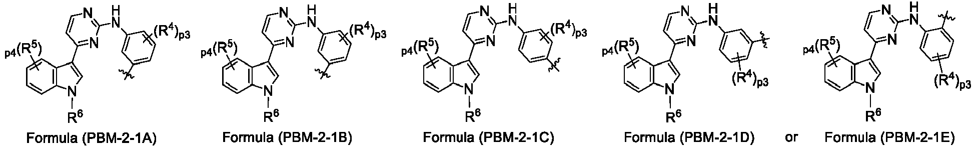 wherein in Formula (PBM-2-1A), Formula (PBM-2-1B), Formula (PBM-2-1C), Formula (PBM-2-1D) and Formula (PBM-2-1E),
(R⁴)ₚ₃ each independently indicates that the benzene ring is substituted with p3 R⁴, with each R⁴ being the same or different and independently representing halogen (e.g., fluorine, chlorine, bromine, or iodine), nitro, cyano, halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₁₀ alkyl (e.g., C₁₋₈ alkyl, C₁₋₆ alkyl or C₁₋₄ alkyl, such as methyl, ethyl, propyl, isopropyl, butyl, sec-butyl, tert-butyl, pentyl, hexyl, heptyl or octyl), deuterated C₁₋₁₀ alkyl (e.g., perdeuterated C₁₋₈ alkyl, perdeuterated C₁₋₆ alkyl or perdeuterated C₁₋₄ alkyl, such as CD₃, CD₃CD₂-, CD₃CD₂CD₂- etc.), C₁₋₁₀ alkoxy (e.g., C₁₋₈ alkoxy, C₁₋₆ alkoxy or C₁₋₄ alkoxy, such as methoxy, ethoxy, propoxy, isopropoxy, butoxy, sec-butoxy, or tert-butoxy) or -NHC(O)R^{19a}, where R^{19a} is C₁₋₁₀ alkyl (e.g., C₁₋₈ alkyl, C₁₋₆ alkyl or C₁₋₄ alkyl, such as methyl, ethyl, propyl, isopropyl, butyl, sec-butyl, tert-butyl, pentyl, hexyl, heptyl or octyl), deuterated C₁₋₁₀ alkyl (e.g., perdeuterated C₁₋₈ alkyl, perdeuterated C₁₋₆ alkyl or perdeuterated C₁₋₄ alkyl, such as CD₃, CD₃CD₂-, CD₃CD₂CD₂- etc.) or C₂₋₁₀ alkenyl (e.g., C₂₋₆ alkenyl or C₂₋₄ alkenyl, such as vinyl, propenyl, butenyl or pentenyl), e.g., R⁴ represents methyl, methoxy, nitro or 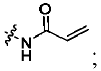
p3 represents an integer of 1, 2, 3 or 4;
(R⁵)ₚ₄ each independently indicates that the 1H-indole ring is substituted with p4 R⁵, with each R⁵ being the same or different and independently representing C₁₋₁₀ alkyl (e.g., C₁₋₈ alkyl, C₁₋₆ alkyl or C₁₋₄ alkyl, such as methyl, ethyl, propyl, isopropyl, butyl, sec-butyl, tert-butyl, pentyl, hexyl, heptyl or octyl) or deuterated C₁₋₁₀ alkyl (e.g., perdeuterated C₁₋₈ alkyl, perdeuterated C₁₋₆ alkyl or perdeuterated C₁₋₄ alkyl, such as CD₃, CD₃CD₂-, CD₃CD₂CD₂- etc.); and
p4 represents an integer of 0, 1, 2 or 3.

In some embodiments, PBM represents the structure of Formula (PBM-2-1) or Formula (PBM-2-2):

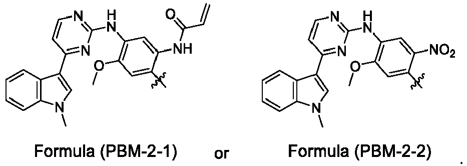

In some embodiments, PBM represents the structure of Formula (PBM-3-1A), Formula (PBM-3-1B), or Formula (PBM-3-1C): 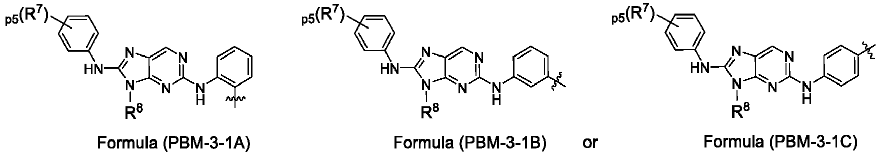 wherein in Formula (PBM-3-1A), Formula (PBM-3-1B) and Formula (PBM-3-1C),
(R⁷)ₚ₅ each independently indicates that the benzene ring is substituted with p5 R⁷, with each R⁷ being the same or different and independently representing halogen (e.g., fluorine, chlorine, bromine, or iodine), hydroxy, C₁₋₆ alkyl (e.g., C₁₋₄ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), NO₂, NH₂, or cyano;
p5 represents an integer of 0, 1, 2, 3, 4 or 5;
R⁸ each independently represents substituted or unsubstituted C₃₋₁₅ cycloalkyl (e.g., substituted or unsubstituted C₃₋₁₀ cycloalkyl or C₃₋₆ cycloalkyl, such as cyclopropyl, cyclobutyl, cyclopentyl, or cyclohexyl). In some embodiments, the C₃₋₁₅ cycloalkyl is optionally substituted with one or more (e.g., 1-3, 1, 2, or 3) substituents selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

In some embodiments, PBM represents the structure of Formula (PBM-3-1):

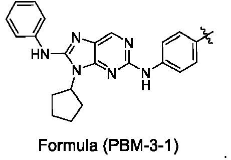

In some embodiments, PBM represents the structure of Formula (PBM-4-1A), Formula (PBM-4-1B), Formula (PBM-4-1C) or Formula (PBM-4-1D): 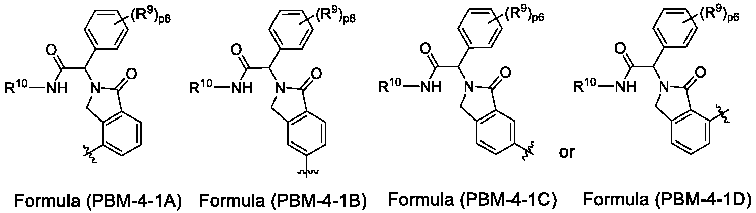 wherein in Formula (PBM-4-1A), Formula (PBM-4-1B), Formula (PBM-4-1C) and Formula (PBM-4-1D),
(R⁹)ₚ₆ each independently indicates that the benzene ring is substituted with p6 R⁹, with each R⁹ being the same or different and independently representing halogen (e.g., fluorine, chlorine, bromine, or iodine), hydroxy, C₁₋₆ alkyl (e.g., C₁₋₆ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), NO₂, NH₂, C₁₋₆ alkoxy (e.g., C₁₋₅ alkoxy or C₁₋₄ alkoxy, such as methoxy, ethoxy, propoxy, isopropoxy, butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), or cyano,
p6 represents an integer of 2, 3, 4 or 5; and
R¹⁰ each independently represents C₆₋₁₀ aryl, or heteroaryl containing one or two 5- to 7-membered rings and 1 to 4 heteroatoms selected from nitrogen, oxygen, and sulfur, wherein the C₆₋₁₀ aryl and heteroaryl are each optionally substituted with one or more (e.g., 1-6, such as 1, 2, 3, 4, 5 or 6) R^{b1}, with each R^{b1} being the same or different and independently representing halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, R¹⁰ represents C₆₋₁₀ aryl, such as but not limited to phenyl or naphthyl, each of which is optionally substituted with one or more (e.g., 1-6, such as 1, 2, 3, 4, 5 or 6) R^{b1} as defined above.

In some embodiments, R¹⁰ represents heteroaryl containing one or two 5- to 7-membered rings and 1 to 4 heteroatoms selected from nitrogen, oxygen, and sulfur. In a sub-embodiment, R¹⁰ represents 5- to 14-membered monocyclic or bicyclic aromatic ring group containing one or more (e.g., from 1 to 6, or from 1 to 5, or from 1 to 4, or from 1 to 3) heteroatoms selected from the group consisting of oxygen, nitrogen, and sulfur. In a sub-embodiment, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, or imidazo[1,2-b]pyridazinyl. The heteroaryl group is optionally substituted with one or more (e.g., 1-6, such as 1, 2, 3, 4, 5 or 6) R^{b1} as defined above.

In some embodiments, PBM represents the structure of Formula (PBM-4-1) or Formula (PBM-4-2):

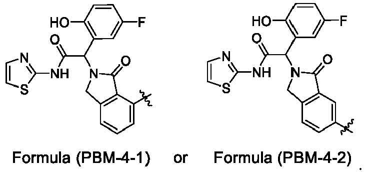

In some embodiments, PBM represents the structure of Formula (PBM-5-1A), Formula (PBM-5-1B), Formula (PBM-5-1C) or Formula (PBM-5-1D): 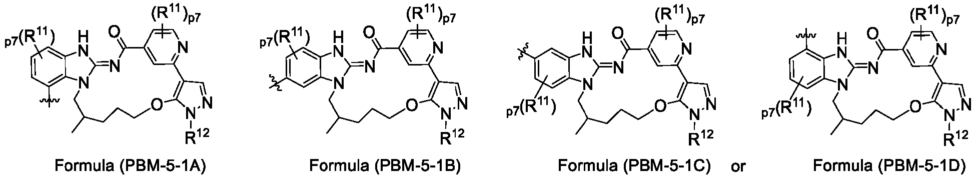 wherein in Formula (PBM-5-1A), Formula (PBM-5-1B), Formula (PBM-5-1C) and Formula (PBM-5-1D),
(R¹¹)ₚ₇ each independently indicates that the benzene ring and the pyridine ring are each optionally substituted with p7 R¹¹, with each p7 being the same or different and each independently representing an integer of 0, 1, 2 or 3, and each R¹¹ is the same or different and each independently represents halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.); and
R¹² each independently represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.).

In some embodiments, PBM represents the structure of Formula (PBM-5-1):

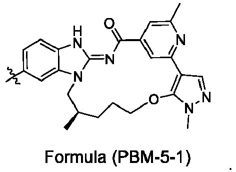

In some embodiments, PBM represents the structure of Formula (PBM-6): 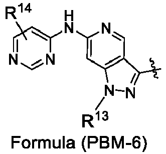 wherein
R¹³ represents hydrogen, C₁₋₁₀ alkyl (e.g., C₁₋₈ alkyl, C₁₋₆ alkyl or C₁₋₄ alkyl, such as methyl, ethyl, propyl, isopropyl( 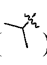 ), butyl, sec-butyl, tert-butyl, pentyl, hexyl, heptyl or octyl), deuterated C₁₋₁₀ alkyl (e.g., perdeuterated C₁₋₈ alkyl, perdeuterated C₁₋₆ alkyl or perdeuterated C₁₋₄ alkyl, such as CD₃, CD₃CD₂-, CD₃CD₂CD₂- etc.) or halogenated C₁₋₁₀ alkyl (e.g., halogenated C₁₋₈ alkyl or halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
R¹⁴ represents 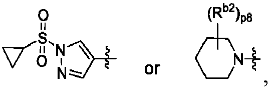 wherein (R^{b2})ₚ₈ represents indicates that the piperidine ring is substituted with p8 R^{b2}, with each R^{b2} being the same or different and each independently representing deuterium, halogen (e.g., fluorine, chlorine, bromine, or iodine), hydroxy, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkoxy (e.g., perdeuterated C₁₋₄ alkoxy, such as CD₃-O-, CD₃CD₂-O-, or CD₃CD₂CD₂-O-), NO₂, NH₂, or cyano, p8 represents an integer of 1, 2, 3, 4 or 5.

In some embodiments, PBM represents the structure of Formula (PBM-6-1) or Formula (PBM-6-2):

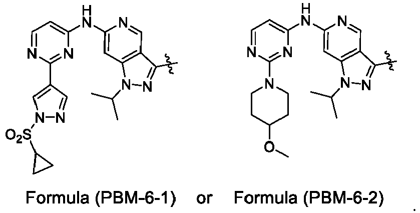

In some embodiments, PBM represents the structure of Formula (PBM-49): 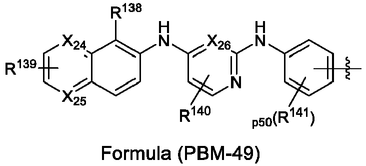
wherein X₂₄, X₂₅, X₂₆ each independently represent CH or N;
R¹³⁸ represents -C(O)NHR^{b19}, -NHC(O)-R^{b19}, -S(O)₂NHR^{b19}, -NHS(O)₂R^{b19}, -S(O)₂R^{b19}or - P(O)(R^{b19})₂, wherein each R^{b19} is the same or different and each independently represents C₁₋₆ alkyl (e.g., C₁₋₄ alkyl, such as methyl, ethyl, propyl, isopropyl, butyl, sec-butyl, tert-butyl, pentyl, or hexyl) or deuterated C₁₋₆ alkyl (e.g., perdeuterated C₁₋₅ alkyl, perdeuterated C₁₋₄ alkyl or perdeuterated C₁₋₃ alkyl, such as CD₃, CD₃CD₂-, CD₃CD₂CD₂- etc.);
R¹³⁹ and R¹⁴⁰ each independently represent halogen (e.g., fluorine, chlorine, bromine, or iodine), hydroxy, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₅ alkyl, perdeuterated C₁₋₄ alkyl or perdeuterated C₁₋₃ alkyl, such as CD₃, CD₃CD₂-, CD₃CD₂CD₂- etc.), C₁₋₆ alkoxy (e.g., C₁₋₅ alkoxy, C₁₋₄ alkoxy or C₁₋₃ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as trifluoromethoxy);
(R¹⁴¹)ₚ₅₀ indicates that the benzene ring is substituted with p50 R¹⁴¹, with each R¹⁴¹ being independently halogen (e.g., fluorine, chlorine, bromine, or iodine), hydroxy, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₅ alkyl, perdeuterated C₁₋₄ alkyl or perdeuterated C₁₋₃ alkyl, such as CD₃, CD₃CD₂-, CD₃CD₂CD₂- etc.), C₁₋₆ alkoxy (e.g., C₁₋₅ alkoxy, C₁₋₄ alkoxy or C₁₋₃ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as trifluoromethoxy); and
p50 represents an integer of 0, 1, 2, 3 or 4.

In some embodiments, PBM represents the structure of Formula (PBM-49-1A), Formula (PBM-49-1B), Formula (PBM-49-1C) or Formula (PBM-49-1D): 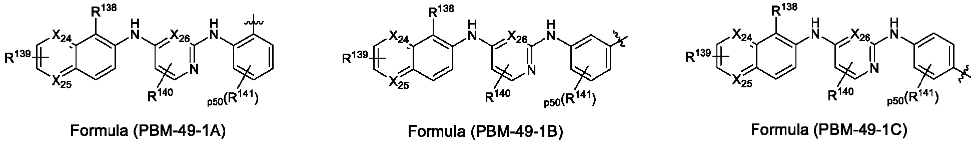 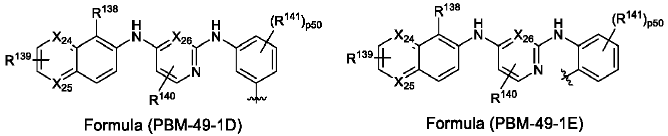 wherein in Formula (PBM-49-1A), Formula (PBM-49-1B), Formula (PBM-49-1C), Formula (PBM-49-1D) and Formula (PBM-49-1E),
X₂₄, X₂₅, X₂₆ each independently represent CH or N;
R¹³⁸ represents -C(O)NHR^{b19}, -NHC(O)-R^{b19}, -S(O)₂NHR^{b19}, -NHS(O)₂R^{b19}, -S(O)₂R^{b19}or - P(O)(R^{bl9})₂, wherein each R^{b19} is the same or different and each independently represents C₁₋₆ alkyl (e.g., C₁₋₄ alkyl, such as methyl, ethyl, propyl, isopropyl, butyl, sec-butyl, tert-butyl, pentyl, or hexyl) or deuterated C₁₋₆ alkyl (e.g., perdeuterated C₁₋₅ alkyl, perdeuterated C₁₋₄ alkyl or perdeuterated C₁₋₃ alkyl, such as CD₃, CD₃CD₂-, CD₃CD₂CD₂- etc.);
R¹³⁹ and R¹⁴⁰ each independently represent halogen (e.g., fluorine, chlorine, bromine, or iodine), hydroxy, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₅ alkyl, perdeuterated C₁₋₄ alkyl or perdeuterated C₁₋₃ alkyl, such as CD₃, CD₃CD₂-, CD₃CD₂CD₂- etc.), C₁₋₆ alkoxy (e.g., C₁₋₅ alkoxy, C₁₋₄ alkoxy or C₁₋₃ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as trifluoromethoxy);
(R^{14I})ₚ₅₀ indicates that the benzene ring is optionally substituted with p50 R¹⁴¹, with each R¹⁴¹ being independently halogen (e.g., fluorine, chlorine, bromine, or iodine), hydroxy, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₅ alkyl, perdeuterated C₁₋₄ alkyl or perdeuterated C₁₋₃ alkyl, such as CD₃, CD₃CD₂-, CD₃CD₂CD₂- etc.), C₁₋₆ alkoxy (e.g., C₁₋₅ alkoxy, C₁₋₄ alkoxy or C₁₋₃ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as trifluoromethoxy); and
p50 represents an integer of 0, 1, 2, 3 or 4.

In some embodiments, X₂₄ and X₂₅ each independently represent CH or N. In some sub-embodiments, X₂₄ represents CH, and X₂₅ represents N. In some sub-embodiments, X₂₄ represents CH, and X₂₅ represents CH. In some sub-embodiments, X₂₄ represents N, and X₂₅ represents CH. In some sub-embodiments, X₂₄ represents N, and X₂₅ represents N.

In some embodiments, X₂₆ represents CH or N. In some sub-embodiments, X₂₆ represents N. In some sub-embodiments, X₂₆ represents CH.

In some embodiments, R¹³⁸ represents -C(O)NHR^{bl9}, -NHC(O)-R^{b19}, -S(O)₂NHR^{b19}, - NHS(O)₂R^{b19}, -S(O)₂R^{b19} or -P(O)(R^{bl9})₂, wherein each R^{bl9} is the same or different and each independently represents C₁₋₆ alkyl (e.g., C₁₋₄ alkyl, such as methyl, ethyl, propyl, isopropyl, butyl, sec-butyl, tert-butyl, pentyl, or hexyl), or deuterated C₁₋₆ alkyl (e.g., perdeuterated C₁₋₅ alkyl, perdeuterated C₁₋₄ alkyl or perdeuterated C₁₋₃ alkyl, such as CD₃, CD₃CD₂-, CD₃CD₂CD₂- etc.). In some sub-embodiments, R¹³⁸ represents -P(O)(R^{bl9})₂, wherein each R^{bl9} is the same or different and each independently represents C₁₋₆ alkyl (e.g., C₁₋₄ alkyl, such as methyl, ethyl, propyl, isopropyl, butyl, sec-butyl, tert-butyl, pentyl, or hexyl), or deuterated C₁₋₆ alkyl (e.g., perdeuterated C₁₋₅ alkyl, perdeuterated C₁₋₄ alkyl or perdeuterated C₁₋₃ alkyl, such as CD₃, CD₃CD₂-, CD₃CD₂CD₂- etc.). In some sub-embodiments, R¹³⁸ represents -P(O)(CH₃)₂.

In some embodiments, p50 represents an integer of 0, 1, 2, 3 or 4. In some sub-embodiments, p50 represents an integer of 2.

In some embodiments, PBM represents the structure of Formula (PBM-49-1), Formula (PBM-49-2), Formula (PBM-49-3), Formula (PBM-49-4) or Formula (PBM-49-5):

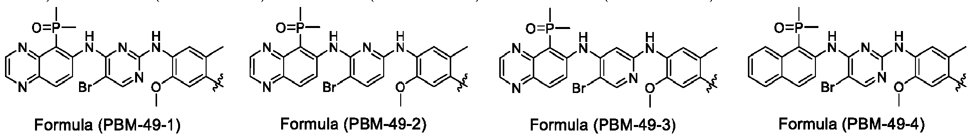

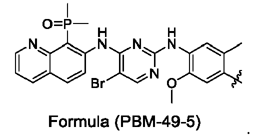

In some embodiments, PBM represents the structure of Formula (PBM-55): 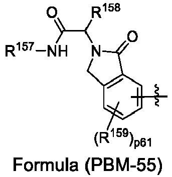 wherein
R¹⁵⁷ represents C_{6-1O} aryl, or heteroaryl containing one or two 5- to 7-membered rings and 1 to 4 heteroatoms selected from nitrogen, oxygen, and sulfur, wherein the C_{6-1O} aryl and heteroaryl are each optionally substituted with one or more (e.g., 1-6, such as 1, 2, 3, 4, 5 or 6) substituents selected from the group consisting of: halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
R¹⁵⁸ represents 5- to 15-membered heteroaryl containing from 1 to 4 heteroatoms selected from nitrogen, oxygen, and sulfur, wherein the 5- to 15-membered heteroaryl is optionally substituted with one or more substituents selected from the group consisting of: halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-); and
(R¹⁵⁹)ₚ₆₁ indicates that the isoindolinone ring in Formula (PBM-55) is substituted with p61 R¹⁵⁹, with each R¹⁵⁹ being the same or different and each independently representing halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p61 represents an integer of 0, 1, 2 or 3.

In some embodiments, R¹⁵⁷ represents C_{6-1O} aryl, such as but not limited to phenyl or naphthyl, each of which is optionally substituted with one or more (e.g., 1-6, such as 1, 2, 3, 4, 5 or 6) substituents selected from the group consisting of: halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CHO-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, R¹⁵⁷ represents heteroaryl containing one or two 5- to 7-membered rings and 1 to 4 heteroatoms selected from nitrogen, oxygen, and sulfur. In a sub-embodiment, R¹⁵⁷ represents 5- to 15-membered monocyclic or bicyclic aromatic ring group containing one or more (e.g., from 1 to 6, or from 1 to 5, or from 1 to 4, or from 1 to 3) heteroatoms selected from the group consisting of oxygen, nitrogen, and sulfur. In a sub-embodiment, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, or imidazo[1,2-b]pyridazinyl. The heteroaryl group is optionally substituted with one or more (e.g., 1-6, such as 1, 2, 3, 4, 5 or 6) substituents selected from the group consisting of: halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, R¹⁵⁸ represents 5- to 15-membered heteroaryl containing 1 to 4 heteroatoms selected from nitrogen, oxygen, and sulfur. In a sub-embodiment, R¹⁵⁸ represents 5- to 15-membered monocyclic or bicyclic aromatic ring group containing from 1 to 4 (e.g., from 1 to 3, or from 1 to 2, or 1) heteroatoms selected from the group consisting of oxygen, nitrogen, and sulfur. In a sub-embodiment, examples of heteroaryl include, but are not limited to, 6,7-dihydro-5H-pyrrolo[l,2-c]imidazolyl, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, or imidazo[1,2-b]pyridazinyl. The heteroaryl group is optionally substituted with one or more (e.g., 1-6, such as 1, 2, 3, 4, 5 or 6) substituents selected from the group consisting of: halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, PBM represents the structure of Formula (PBM-55-1) or Formula (PBM-55-2):

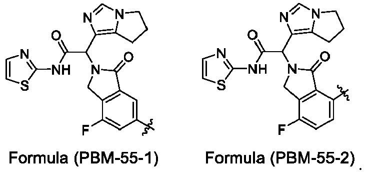

In some embodiments, PBM-R_{f}- represents a small molecule ligand targeting AR.

In some embodiments, PBM represents the structure of Formula (PBM-7-1A): 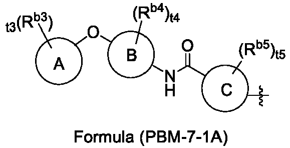 wherein
ring A in (PBM-7-1A) represents C_{6-1O} aryl or 5- to 20-membered monocyclic or bicyclic heteroaryl containing from 1 to 4 heteroatoms selected from the group consisting of oxygen, nitrogen, and sulfur, and (R^{b3})ₜ₃ indicates that ring A is optionally substituted with t3 R^{b3}, with each R^{b3} being the same or different and each independently representing deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₂₋₆ alkenyl (e.g., vinylvinyl, propenyl, or butenyl), or C₂₋₆ alkynyl (e.g., ethynyl, propynyl, or butynyl);
ring B in Formula (PBM-7-1A) represents C₃₋₁₅ cycloalkyl or 3- to 20-membered heterocyclyl containing from 1 to 4 heteroatoms selected from the group consisting of oxygen, nitrogen, and sulfur, and (R^{b4})ₜ₄ indicates that ring B is optionally substituted with t4 R^{b4}, with each R^{b4} being the same or different and each independently representing deuterium, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
ring C in Formula (PBM-7-1A) represents 5- to 20-membered monocyclic or bicyclic heteroaryl containing from 1 to 4 heteroatoms selected from the group consisting of oxygen, nitrogen, and sulfur, and (R^{b5})ₜ₅ indicates that ring C is optionally substituted with t5 R^{b5}, with each R^{b5} being the same or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CHO-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
t3 represents an integer of 1, 2, 3, 4, 5 or 6;
t4 represents an integer of 1, 2, 3, 4, 5 or 6; and
t5 represents an integer of 1 or 2.

In some embodiments, PBM represents the structure of Formula (PBM-7-1A-1): 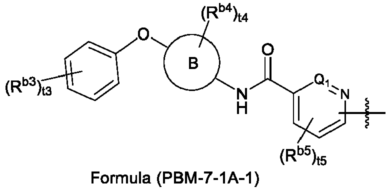
wherein (R^{b3})ₜ₃ in Formula (PBM-7-1A-1) indicates that the benzene ring is optionally substituted with t3 R^{b3}, with each R^{b3} being the same or different and each independently representing deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₂₋₆ alkenyl (e.g., vinyl, propenyl, or butenyl), or C₂₋₆ alkynyl (e.g., ethynyl, propynyl, or butynyl);
ring B in Formula (PBM-7-1A-1) represents C₃₋₁₅ cycloalkyl or 3- to 20-membered heterocyclyl containing from 1 to 4 heteroatoms selected from the group consisting of oxygen, nitrogen, and sulfur, wherein (R^{b4})ₜ₄ indicates that the ring B is optionally substituted with t4 R^{b4}, with each R^{b4} being the same or different and each independently representing deuterium, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
Q₁ represents N or CH; and
(R^{b5})ₜ₅ in Formula (PBM-7-1A-1) indicates that nitrogen-containing heteroaryl is optionally substituted with t5 R^{b5}, with each R^{b5} being the same or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
t3 represents an integer of 1, 2, 3, 4, 5 or 6;
t4 represents an integer of 1, 2, 3, 4, 5 or 6; and
t5 represents an integer of 1 or 2.

In some embodiments, PBM represents the structure of Formula (PBM-7-1), Formula (PBM-7-2), Formula (PBM-7-3) or Formula (PBM-7-4):

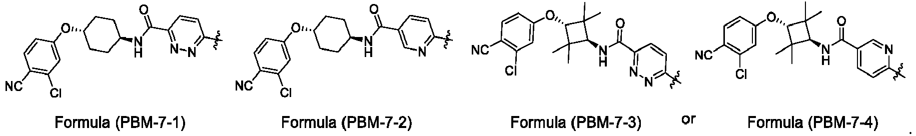

In some embodiments, PBM represents the structure of Formula (PBM-8-1A), Formula (PBM-8-1B), Formula (PBM-8-1C), Formula (PBM-8-1D) or Formula (PBM-8-1E): 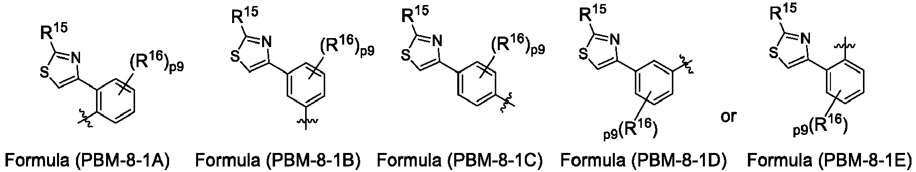 wherein in Formula (PBM-8-1A), Formula (PBM-8-1B), Formula (PBM-8-1C), Formula (PBM-8-1D) and Formula (PBM-8-1E),
R¹⁵ each independently represents 4- to 20-membered heterocyclyl containing from 1 to 4 heteroatoms selected from the group consisting of oxygen, nitrogen, and sulfur, wherein the heterocyclyl is optionally substituted with one or more R^{b6}, with each R^{b6} being the same or different and each independently representing halogen (e.g., fluorine, chlorine, bromine, or iodine), hydroxy, cyano, amino, nitro, C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-); and
(R¹⁶)ₚ₉ each independently indicates that the benzene ring is optionally substituted with p9 R¹⁶, with each R¹⁶ being the same or different and each independently representing halogen, C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), and p9 represents an integer of 1, 2, 3 or 4;

In some embodiments, PBM represents the structure of Formula (PBM-8-1), Formula (PBM-8-2), Formula (PBM-8-3), or Formula (PBM-8-4):

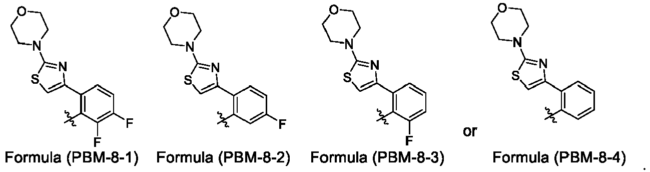

In some embodiments, PBM-R_{f}- representsa small molecule ligand targeting BTK.

In some embodiments, PBM represents the structure of Formula (PBM-9): 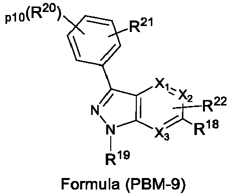
wherein X₁ in Formula (PBM-9) represents N or CR^{b7}, wherein R^{b7} represents hydrogen or amino, X₂ represents N or CR¹⁷, wherein R¹⁷ is hydrogen or represents a bond, and X₃ represents CH or N;
R¹⁸ and R¹⁹ each independently represent hydrogen or represents a bond;
(R²⁰)ₚ₁₀ indicates that the benzene ring in Formula (PBM-9) is substituted with p10 R²⁰, with each R²⁰ being the same or different and each independently representing -O-aryl, -O-heteroaryl, -C₁₋₃ alkylene-NHC(O)-heteroaryl, -C₁₋₃ alkylene-C(O)NH-heteroaryl or halogen (e.g., fluorine, chlorine, bromine, or iodine), wherein the aryl and heteroaryl are each independently optionally substituted with one or more (e.g., 1-5, or 1, 2, 3, 4 or 5) R^{b8}, with each R^{b8} being the same or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CHO-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
ρ10 represents an integer of 1, 2, 3 or 4;
R²¹ represents a bond, deuterium, hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy);
R²² represents hydrogen, halogen (e.g., fluorine, chlorine, bromine, or iodine), cyano, or amino;
wherein R¹⁷, R¹⁸, R¹⁹ and R²¹ are not simultaneously hydrogen, and R¹⁷, R¹⁸, R¹⁹, and R²¹ do not simultaneously represent a bond, and only one of R¹⁷, R¹⁸, R¹⁹, and R²¹ represents a bond, wherein
   when R¹⁸ represents a bond, the carbon atom on the ring connected to R¹⁸ is directly connected to R_{f}, X₂ represents CH or N, and R¹⁹ is hydrogen, and R²¹ is not a bond;
   when X₂ represents CR¹⁷, where R¹⁷ represents a bond, the carbon atom on the ring connected to R¹⁷ is directly connected to R_{f}, and R¹⁸ and R¹⁹ are hydrogen, and R²¹ is not a bond;
   when R¹⁹ represents a bond, the nitrogen atom on the ring connected to R¹⁹ is directly connected to R_{f}, X₂ represents N or CH, and R¹⁸ is hydrogen, and R²¹ is not a bond; and
   when R²¹ represents a bond, the carbon atom on the ring connected to R²¹ is directly connected to R_{f}, and X₂ represents N or CH, and R¹⁸ and R¹⁹ are hydrogen.

In some embodiments, R²⁰ in Formula (PBM-9) represents -O-aryl, -O-heteroaryl, -C₁₋₃ alkylene-NHC(O)-heteroaryl, or -C₁₋₃ alkylene-C(O)NH-heteroaryl, wherein aryl can be optionally substituted C_{6-1O} aryl, including but not limited to e.g., phenyl or naphthyl, and heteroaryl can be optionally substituted 5- to 14-membered monocyclic or bicyclic aromatic ring group containing one or more (e.g., from 1 to 6, or from 1 to 5, or from 1 to 4, or from 1 to 3) heteroatoms selected from the group consisting of oxygen, nitrogen, and sulfur. In a sub-embodiment, R²⁰ represents -O-aryl or -O-naphthyl, and the phenyl or naphthyl is optionally substituted with one or more (e.g., 1-5, such as 1, 2, 3, 4 or 5) R^{b8}, with each R^{b8} being the same or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In a sub-embodiment, R²⁰ represents -O-heteroaryl, -C₁₋₃ alkylene-NHC(O)-heteroaryl, or -C₁₋₃ alkylene-C(O)NH-heteroaryl, wherein heteroaryl is optionally substituted with one or more (e.g., 1-5, such as 1, 2, 3, 4 or 5) R^{b8}, with each R^{b8} being the same or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In a sub-embodiment, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, or imidazo[2,1-b]thiazolyl.

In some embodiments, R¹⁹ represents a bond, R¹⁸ is hydrogen, R²¹ is not a bond, and X₂ represents N or CH.

In some embodiments, R¹⁹ represents a bond, R¹⁸ is hydrogen, R²¹ is not a bond, and X₂ represents N, X₁ represents CR^{b7}, wherein R^{b7} is hydrogen or amino, and X₃ represents N.

In some embodiments, R¹⁹ represents a bond, R¹⁸ is hydrogen, R²¹ is not a bond, and X₂ represents N, X₁ represents CR^{b7}, wherein R^{b7} is hydrogen or amino, and X₃ represents CH.

In some embodiments, R¹⁹ represents a bond, R¹⁸ is hydrogen, R²¹ is not a bond, and X₂ represents N, X₁ represents N, and X₃ represents CH.

In some embodiments, R¹⁹ represents a bond, R¹⁸ is hydrogen, R²¹ is not a bond, and X₂ represents N, X₁ represents N, and X₃ represents N.

In some embodiments, R¹⁹ represents a bond, R¹⁸ is hydrogen, R²¹ is not a bond, and X₂ represents CH, X₁ represents CR^{b7}, wherein R^{b7} is hydrogen or amino, and X₃ represents N.

In some embodiments, R¹⁹ represents a bond, R¹⁸ is hydrogen, R²¹ is not a bond, and X₂ represents CH, X₁ represents CR^{b7}, wherein R^{b7} is hydrogen or amino, and X₃ represents CH.

In some embodiments, R¹⁹ represents a bond, R¹⁸ is hydrogen, R²¹ is not a bond, and X₂ represents CH, X₁ represents N, and X₃ represents CH.

In some embodiments, R¹⁹ represents a bond, R¹⁸ is hydrogen, R²¹ is not a bond, and X₂ represents CH, X₁ represents N, and X₃ represents N.

In some embodiments, when R¹⁸ represents a bond, the carbon atom on the ring connected to R¹⁸ is directly connected to R_{f}, X₂ represents CH or N, and R¹⁹ is hydrogen, and R²¹ is not a bond.

In some embodiments, R¹⁸ represents a bond, R¹⁹ is hydrogen, R²¹ is not a bond, and X₂ represents CH, X₁ represents N, and X₃ represents N.

In some embodiments, R¹⁸ represents a bond, R¹⁹ is hydrogen, R²¹ is not a bond, and X₂ represents CH, X₁ represents N, and X₃ represents CH.

In some embodiments, R¹⁸ represents a bond, R¹⁹ is hydrogen, R²¹ is not a bond, and X₂ represents CH, X₁ represents CR^{b7}, wherein R^{b7} is hydrogen or amino, and X₃ represents N.

In some embodiments, R¹⁸ represents a bond, R¹⁹ is hydrogen, R²¹ is not a bond, and X₂ represents CH, X₁ represents CR^{b7}, wherein R^{b7} is hydrogen or amino, and X₃ represents CH.

In some embodiments, R¹⁸ represents a bond, R¹⁹ is hydrogen, R²¹ is not a bond, and X₂ represents N, X₁ represents CR^{b7}, wherein R^{b7} is hydrogen or amino, and X₃ represents N.

In some embodiments, R¹⁸ represents a bond, R¹⁹ is hydrogen, R²¹ is not a bond, and X₂ represents N, X₁ represents CR^{b7}, wherein R^{b7} is hydrogen or amino, and X₃ represents CH.

In some embodiments, R¹⁸ represents a bond, R¹⁹ is hydrogen, R²¹ is not a bond, and X₂ represents N, X₁ represents N, and X₃ represents CH.

In some embodiments, R¹⁸ represents a bond, R¹⁹ is hydrogen, R²¹ is not a bond, and X₂ represents N, X₁ represents N, and X₃ represents N.

In some embodiments, when X₂ represents CR¹⁷, wherein R¹⁷ represents a bond, the carbon atom on the ring connected to R¹⁷ is directly connected to R_{f}, and R¹⁸ and R¹⁹ are hydrogen, and R²¹ is not a bond.

In some embodiments, when X₂ represents CR¹⁷, where R¹⁷ represents a bond, the carbon atom on the ring connected to R¹⁷ is directly connected to R_{f}, and R¹⁸ and R¹⁹ are hydrogen, and R²¹ is not a bond, and X₁ represents N, and X₃ represents CH.

In some embodiments, when X₂ represents CR¹⁷, where R¹⁷ represents a bond, the carbon atom on the ring connected to R¹⁷ is directly connected to R_{f}, and R¹⁸ and R¹⁹ are hydrogen, and R²¹ is not a bond, and X₁ represents N, and X₃ represents N.

In some embodiments, when X₂ represents CR¹⁷, where R¹⁷ represents a bond, the carbon atom on the ring connected to R¹⁷ is directly connected to R_{f}, and R¹⁸ and R¹⁹ are hydrogen, and R²¹ is not a bond, and X₁ represents CR^{b7}, where R^{b7} is hydrogen or amino, and X₃ represents CH.

In some embodiments, when X₂ represents CR¹⁷, where R¹⁷ represents a bond, the carbon atom on the ring connected to R¹⁷ is directly connected to R_{f}, and R¹⁸ and R¹⁹ are hydrogen, and R²¹ is not a bond, and X₁ represents CR^{b7}, where R^{b7} is hydrogen or amino, and X₃ represents N.

In some embodiments, when R²¹ represents a bond, the carbon atom on the ring connected to R²¹ is directly connected to R_{f}, and X₂ represents N or CH, and R¹⁸ and R¹⁹ are hydrogen.

In some embodiments, when R²¹ represents a bond, the carbon atom on the ring connected to R²¹ is directly connected to R_{f}, and R¹⁸ and R¹⁹ are hydrogen, and X₂ represents N, X₁ represents N, and X₃ represents CH.

In some embodiments, when R²¹ represents a bond, the carbon atom on the ring connected to R²¹ is directly connected to R_{f}, and R¹⁸ and R¹⁹ are hydrogen, and X₂ represents N, X₁ represents N, and X₃ represents N.

In some embodiments, when R²¹ represents a bond, the carbon atom on the ring connected to R²¹ is directly connected to R_{f}, and R¹⁸ and R¹⁹ are hydrogen, and X₂ represents N, X₁ represents CR^{b7}, where R^{b7} is hydrogen or amino, and X₃ represents N.

In some embodiments, when R²¹ represents a bond, the carbon atom on the ring connected to R²¹ is directly connected to R_{f}, and R¹⁸ and R¹⁹ are hydrogen, and X₂ represents N, X₁ represents CR^{b7}, where R^{b7} is hydrogen or amino, and X₃ represents CH.

In some embodiments, when R²¹ represents a bond, the carbon atom on the ring connected to R²¹ is directly connected to R_{f}, and R¹⁸ and R¹⁹ are hydrogen, and X₂ represents CH, X₁ represents N, and X₃ represents CH.

In some embodiments, when R²¹ represents a bond, the carbon atom on the ring connected to R²¹ is directly connected to R_{f}, and R¹⁸ and R¹⁹ are hydrogen, and X₂ represents CH, X₁ represents N, and X₃ represents N.

In some embodiments, when R²¹ represents a bond, the carbon atom on the ring connected to R²¹ is directly connected to R_{f}, and R¹⁸ and R¹⁹ are hydrogen, and X₂ represents CH, X₁ represents CR^{b7}, where R^{b7} is hydrogen or amino, and X₃ represents CH.

In some embodiments, when R²¹ represents a bond, the carbon atom on the ring connected to R²¹ is directly connected to R_{f}, and R¹⁸ and R¹⁹ are hydrogen, and X₂ represents CH, X₁ represents CR^{b7}, where R^{b7} is hydrogen or amino, and X₃ represents N.

In some embodiments, PBM represents the structure of the following Formula (PBM-9-1A): 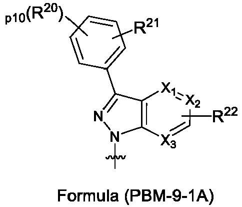 wherein
X₁ in Formula (PBM-9-1A) represents N or CR^{b7}, wherein R^{b7} represents hydrogen or amino, X₂ represents N or CH, and X₃ represents CH or N;
(R²⁰)ₚ₁₀ indicates that the benzene ring in Formula (PBM-9-1A) is substituted with p10 R²⁰ substituents, with each R²⁰ being the same or different and each independently representing -O-aryl, - O-heteroaryl, -C₁₋₃ alkylene-NHC(O)-heteroaryl, or -C₁₋₃ alkylene-C(O)NH-heteroaryl, and the aryl and heteroaryl are independently optionally substituted with one or more (e.g., 1-5, such as 1, 2, 3, 4 or 5) R^{b8}, with each R^{b8} being the same or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
R²¹ represents deuterium, hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), e.g., R²¹ represents hydrogen or methyl; and
R²² represents hydrogen, halogen (e.g., fluorine, chlorine, bromine, or iodine), cyano, or amino.

In some embodiments, R²⁰ in Formula (PBM-9-1A) represents -O-aryl, -O-heteroaryl, -C₁₋₃ alkylene-NHC(O)-heteroaryl, or -C₁₋₃ alkylene-C(O)NH-heteroaryl, wherein the aryl can be optionally substituted C₆₋₁₀ aryl, including but not limited to e.g., phenyl or naphthyl, and the heteroaryl can be optionally substituted 5- to 14-membered monocyclic or bicyclic aromatic ring group containing one or more (e.g., from 1 to 6, or from 1 to 5, or from 1 to 4, or from 1 to 3) heteroatoms selected from the group consisting of oxygen, nitrogen, and sulfur. In a sub-embodiment, R²⁰ represents -O-aryl or -O-naphthyl, where the phenyl or naphthyl is optionally substituted with one or more (e.g., 1-5, such as 1, 2, 3, 4 or 5) R^{b8}, with each R^{b8} being the same or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In a sub-embodiment, R²⁰ represents -O-heteroaryl, -C₁₋₃ alkylene-NHC(O)-heteroaryl, or -C₁₋₃ alkylene-C(O)NH-heteroaryl, where the heteroaryl is optionally substituted with one or more (e.g., 1-5, such as 1, 2, 3, 4 or 5) R^{b8}, with each R^{b8} being the same or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂-etc.), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In a sub-embodiment, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, or imidazo[2,1-b]thiazolyl.

In some embodiments, X₁ in Formula (PBM-9-1A) represents N, X₂ represents N, and X₃ represents CH.

In some embodiments, X₁ in Formula (PBM-9-1A) represents N, X₂ represents N, and X₃ represents N.

In some embodiments, X₁ in Formula (PBM-9-1A) represents N, X₂ represents CH, and X₃ represents CH.

In some embodiments, X₁ in Formula (PBM-9-1A) represents N, X₂ represents CH, and X₃ represents N.

In some embodiments, X₁ in Formula (PBM-9-1A) represents CR^{b7}, where R^{b7} is hydrogen or amino, X₂ represents N, and X₃ represents N.

In some embodiments, X₁ in Formula (PBM-9-1A) represents CR^{b7}, where R^{b7} is hydrogen or amino, X₂ represents N, and X₃ represents CH.

In some embodiments, X₁ in Formula (PBM-9-1A) represents CR^{b7}, where R^{b7} is hydrogen or amino, X₂ represents CH, and X₃ represents N.

In some embodiments, X₁ in Formula (PBM-9-1A) represents CR^{b7}, where R^{b7} is hydrogen or amino, X₂ represents CH, and X₃ represents CH.

In some embodiments, PBM represents the structure of the following Formula (PBM-9-1):

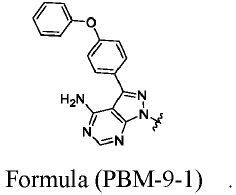

In some embodiments, PBM represents the structure of the following Formula (PBM-9-1B): 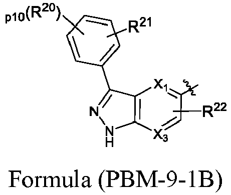 wherein
X₁ in Formula (PBM-9-1B) represents N or CR^{b7}, where R^{b7} is hydrogen or amino, and X₃ represents CH or N;
(R²⁰)ₚ₁₀ indicates that the benzene ring in Formula (PBM-9-1B) is substituted with p10 R²⁰, with each R²⁰ being the same or different and each independently representing -O-aryl, -O-heteroaryl, -C₁₋₃ alkylene-NHC(O)-heteroaryl, or -C₁₋₃ alkylene-C(O)NH-heteroaryl, where the aryl and heteroaryl are independently optionally substituted with one or more (e.g., 1-5, such as 1, 2, 3, 4 or 5) R^{b8}, with each R^{b8} being the same or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, C1₂CH-O-, CF₃CF₂-O-, CF₃CHF-O-, CHF₂CF₂-O-, CHF₂CHF-O-, CF₃CH₂-O- or CH₂ClCH₂-O-);
R²¹ represents deuterium, hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), e.g., R²¹ represents hydrogen or methyl; and
R²² represents hydrogen, halogen (e.g., fluorine, chlorine, bromine, or iodine), cyano, or amino.

In some embodiments, R²⁰ in Formula (PBM-9-1B) represents -O-aryl, -O-heteroaryl, -C₁₋₃ alkylene-NHC(O)-heteroaryl, or -C₁₋₃ alkylene-C(O)NH-heteroaryl, where the aryl can be optionally substituted C₆₋₁₀ aryl, including but not limited to e.g., phenyl or naphthyl, and the heteroaryl can be optionally substituted 5- to 14-membered monocyclic or bicyclic aromatic ring group containing one or more (e.g., from 1 to 6, or from 1 to 5, or from 1 to 4, or from 1 to 3) heteroatoms selected from the group consisting of oxygen, nitrogen, and sulfur. In a sub-embodiment, R²⁰ represents -O-aryl or -O-naphthyl, and the phenyl or naphthyl is optionally substituted with one or more (e.g., 1-5, such as 1, 2, 3, 4 or 5) R^{b8}, with each R^{b8} being the same or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In a sub-embodiment, R²⁰ represents -O-heteroaryl, -C₁₋₃ alkylene-NHC(O)-heteroaryl, or -C₁₋₃ alkylene-C(O)NH-heteroaryl, wherein the heteroaryl is optionally substituted with one or more (e.g., 1-5, such as 1, 2, 3, 4 or 5) R^{b8}, with each R^{b8} being the same or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In a sub-embodiment, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, or imidazo[2,1-b]thiazolyl.

In some embodiments, X₁ in Formula (PBM-9-1B) represents N, and X₃ represents CH.

In some embodiments, X₁ in Formula (PBM-9-1B) represents N, and X₃ represents N.

In some embodiments, X₁ in Formula (PBM-9-1B) represents CR^{b7}, where R^{b7} represents hydrogen or amino, and X₃ represents CH.

In some embodiments, X₁ in Formula (PBM-9-1B) represents CR^{b7}, where R^{b7} represents hydrogen or amino, and X₃ represents N.

In some embodiments, PBM represents the structure of the following Formula (PBM-9-2):

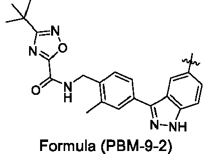

In some embodiments, PBM represents the structure of the following Formula (PBM-10-1A) or Formula (PBM-10-1B): 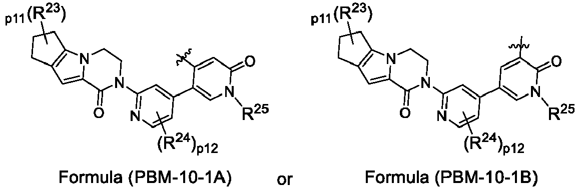 wherein in Formula (PBM-10-1A) and Formula (PBM-10-1B),
(R²³)ₚ₁₁ each independently indicates that the cyclopentene ring is substituted with p11 R²³, with each R²³ being the same or different and each independently representing halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), and p11 represents an integer of 2, 3, or 4;
(R²⁴)ₚ₁₂ each independently indicates that the pyridine ring is substituted with p12 R²⁴, with each R²⁴ being the same or different and each independently representing halogen (e.g., fluorine, chlorine, bromine, or iodine), -C₁₋₃ alkylene-OH (e.g., -CH₂-OH, -CH₂CH₂-OH, -CH₂CH₂CH₂-OH), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), and p12 represents an integer of 1, 2, or 3; and
R²⁵ each independently represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.).

In some embodiments, PBM represents the structure of the following Formula (PBM-10-1) or Formula (PBM-10-2):

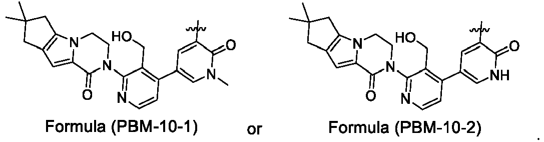

In some embodiments, PBM represents the structure of the following Formula (PBM-46-1A), Formula (PBM-46-1B), or Formula (PBM-46-1C): 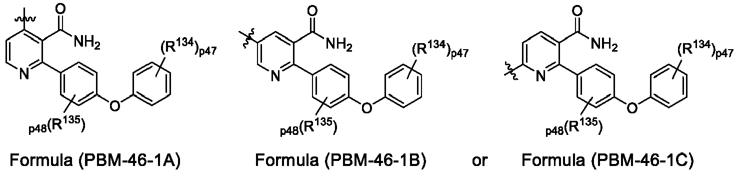 wherein in Formula (PBM-46-1A), Formula (PBM-46-1B) and Formula (PBM-46-1C),
(R¹³⁴)ₚ₄₇ each independently indicates that the benzene ring is optionally substituted with p47 R¹³⁴, with each R¹³⁴ being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p47 represents an integer of 0, 1, 2, 3 or 4;
(R¹³⁵)ₚ₄₈ in Formula (PBM-46-1A), Formula (PBM-46-1B) and Formula (PBM-46-1C) each independently indicates that the benzene ring is optionally substituted with p48 R¹³⁵, with each R¹³⁵ being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p48 represents an integer of 0, 1, 2, 3 or 4.

In some embodiments, PBM represents the structure of the following Formula (PBM-46-1):

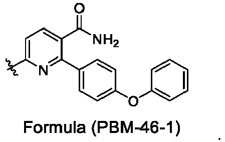

In some embodiments, PBM represents the structure of the following Formula (PBM-45-1A), Formula (PBM-45-1B), Formula (PBM-45-1C), Formula (PBM-45-1D), or Formula (PBM-45-1E): 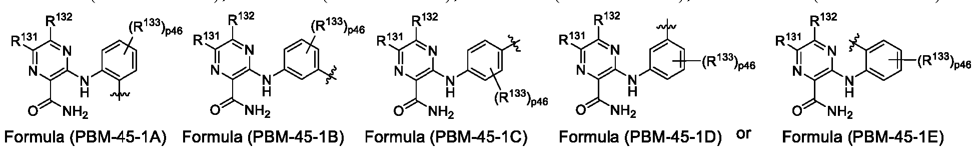 wherein
R¹³¹ represents C₁₋₆ alkyl (e.g., C₁₋₄ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or R¹³¹ represents hydrogen;
R¹³² represents optionally substituted 4- to 8-membered nitrogen-containing heterocyclyl;
(R¹³³)ₚ₄₆ indicates that the benzene ring in Formula (PBM-45-1A), Formula (PBM-45-1B), Formula (PBM-45-1C), Formula (PBM-45-1D), or Formula (PBM-45-1E) is optionally substituted with p46 R¹³³, with each R¹³³ being the same or different and each independently representing hydroxy, amino, cyano, halogen (e.g., fluorine, chlorine, bromine, or iodine), C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p46 represents an integer of 0, 1, 2, 3 or 4.

In some embodiments, R¹³¹ represents ethyl. In some embodiments, R¹³¹ represents hydrogen. In some embodiments, R¹³² represents optionally substituted 4- to 8-membered heterocyclyl. In some embodiments, the optionally substituted 4- to 8-membered heterocyclyl is e.g., optionally substituted 4- to 8-membered (e.g., 4- to 7-membered, 4- to 6-membered, 5- to 7-membered, or 5- to 6-membered) monocyclic or bicyclic heterocyclyl group containing one or more (e.g., 1-4, or 1, 2, 3, or 4) heteroatoms selected from nitrogen, oxygen, and sulfur, including but not limited to the following optionally substituted groups: azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo [2.2.1]heptanyl, azabicyclo [3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 8-membered heterocyclyl group is optionally substituted with a substituent selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, 3-methyl-2-oxoimidazolidin-1-yl or any combination thereof. In some embodiments, R¹³² represents piperidinyl which is optionally substituted with 3-methyl-2-oxoimidazolidin-1-yl. In some embodiments, p46 represents an integer of 0. In some embodiments, p46 represents an integer of 1, and R¹³³ represents methoxy.

In some embodiments, PBM represents the structure of the following Formula (PBM-45-2), Formula (PBM-45-3) or Formula (PBM-45-4):

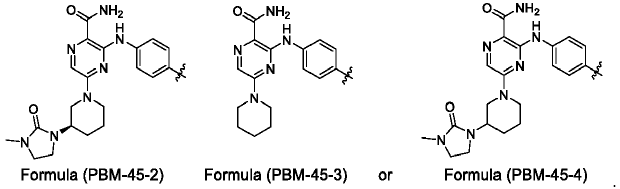

In some embodiments, PBM-R_{f}- represents a small molecule ligand targeting CDK4/6.

In some embodiments, PBM represents the structure of the following Formula (PBM-11-1A), Formula (PBM-11-1B), Formula (PBM-11-1C) or Formula (PBM-11-1D): 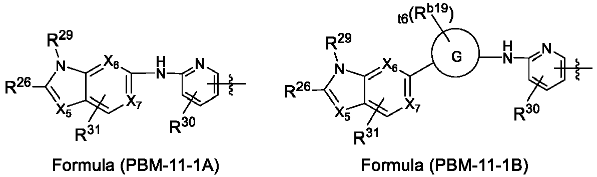 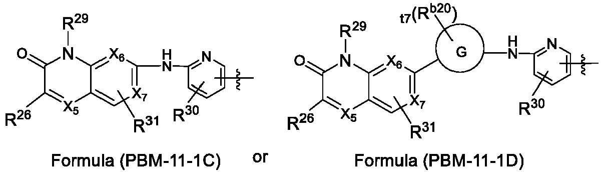 wherein
R²⁶ in Formula (PBM-11-1A), Formula (PBM-11-1B), Formula (PBM-11-1C) and Formula (PBM-11-1D) each independently represents deuterium, hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), -C(O)-C₁₋₆ alkyl (e.g., -C(O)-C₁₋₅ alkyl or -C(O)-C₁₋₃ alkyl, e.g., -C(O)-CH₃, -C(O)-CH₂CH₃), -C(O)-deuterated C₁₋₆ alkyl (e.g., -C(O)-deuterated C₁₋₅ alkyl or -C(O)-deuterated C₁₋₃ alkyl, such as -C(O)-CD₃) or -C(O)NR^{b9}R^{b10}, where R^{b9} and R^{b10} are the same or different and each independently represent hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl) or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), and R^{b9}, R^{b10} are not simultaneously hydrogen;
X₅ in Formula (PBM-11-1A), Formula (PBM-11-1B), Formula (PBM-11-1C) and Formula (PBM-11-1D) each independently represents N or CR²⁷, where R²⁷ represents hydrogen, deuterium, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
X₆ and X₇ in Formula (PBM-11-1A), Formula (PBM-11-1B), Formula (PBM-11-1C) and Formula (PBM-11-1D) each independently represent CH or N;
ring G in Formula (PBM-11-1B) and Formula (PBM-11-1D) each independently represent heteroarylene (e.g., optionally substituted 5- to 20-membered monocyclic or bicyclic aromatic ring group containing one or more (e.g., from 1 to 6, or from 1 to 5, or from 1 to 4, or from 1 to 3) heteroatoms selected from the group consisting of oxygen, nitrogen, and sulfur);
(R^{b19})ₜ₆ in Formula (PBM-11-1B) indicates that ring G is substituted with t6 R^{b19}, with each R^{b19} being the same or different and each independently representing halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CHO-, ClCH₂-O-, C1₂CH-O-, CF₃CF₂-O-, CF₃CHF-O-, CHF₂CF₂-O-, CHF₂CHF-O-, CF₃CH₂-O- or CH₂ClCH₂-O-);
(R^{b20})t₇ in Formula (PBM-11-1D) indicates that ring G is substituted with t7 R^{b20}, with each R^{b20} being the same or different and each independently representing halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CHO-, ClCH₂-O-, C1₂CH-O-, CF₃CF₂-O-, CF₃CHF-O-, CHF₂CF₂-O-, CHF₂CHF-O-, CF₃CH₂-O- or CH₂ClCH₂-O-);
R²⁹ in Formula (PBM-11-1A), Formula (PBM-11-1B), Formula (PBM-11-1C) and Formula (PBM-11-1D) represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or optionally substituted C₃₋₇ cycloalkyl (e.g., cyclopropyl, cyclobutyl, cyclopentyl, cyclopentenyl, cyclohexyl, cyclohexenyl or cycloheptyl, which are optionally substituted with a substituent selected from the group consisting of halogen, C₁₋₆ alkyl, halogenated C₁₋₆ alkyl or deuterated C₁₋₆ alkyl);
R³⁰ and R³¹ in Formula (PBM-11-1A), Formula (PBM-11-1B), Formula (PBM-11-1C) and Formula (PBM-11-1D) are the same or different and each independently represent hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
t6 represents an integer of 0, 1, or 2; and
t7 represents an integer of 0, 1, or 2.

In some embodiments, ring G represents optionally substituted heteroarylene, e.g., optionally substituted 5- to 14-membered monocyclic or bicyclic heteroarylene containing one or more (e.g., from 1 to 6, or from 1 to 5, or from 1 to 4, or from 1 to 3) heteroatoms selected from the group consisting of oxygen, nitrogen, and sulfur. Examples of heteroarylene include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-fluoro[2,3-b]pyrrolylene, pyrrolo[2,1-b]thiazolylene, and imidazo[2,1-b]thiazolylene. Heteroarylene is optionally substituted with one or more (e.g., 1-4, such as 1, 2, 3, or 4) substituents selected from the group consisting of: halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH_{Z}-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, PBM represents the structure of the following Formula (PBM-11-1), Formula (PBM-11-2) or Formula (PBM-11-3):

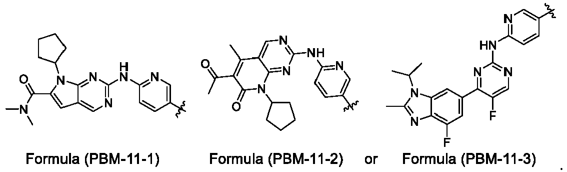

In some embodiments, PBM-R_{f}- represents a small molecule ligand targeting ALK.

In some embodiments, PBM represents the structure of the following Formula (PBM-12-1A), Formula (PBM-12-1B), Formula (PBM-12-1C) or Formula (PBM-12-1D): 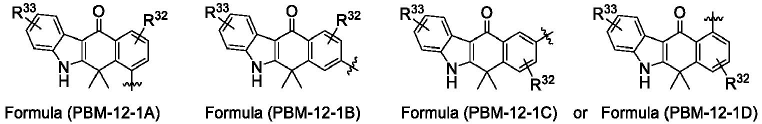 wherein
R³² in Formula (PBM-12-1A), Formula (PBM-12-1B), Formula (PBM-12-1C) and Formula (PBM-12-1D) each independently represent hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.); and
R³³ in Formula (PBM-12-1A), Formula (PBM-12-1B), Formula (PBM-12-1C) and Formula (PBM-12-1D) each independently represent hydrogen, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.).

In some embodiments, PBM represents the structure of the following Formula (PBM-12-1):

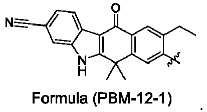

In some embodiments, PBM represents the structure of the following Formula (PBM-13): 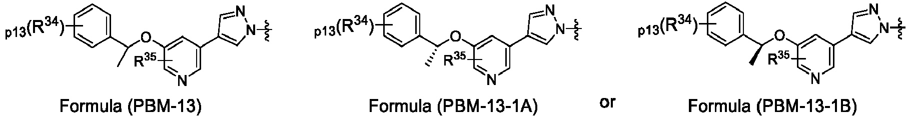 wherein
(R³⁴)ₚ₁₃ indicates that the benzene ring in Formula (PBM-13) is substituted with p13 R³⁴, with each R³⁴ being the same or different and each independently representing halogen (e.g., fluorine, chlorine, bromine, or iodine), hydroxy, amino, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.);
p13 represents an integer of 1, 2, 3, 4 or 5; or
R³⁵ represents NR^{b11}R^{b12}, wherein R^{b11} and R^{b12} are the same or different and each independently represent hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.).

In some embodiments, PBM represents the structure of the following Formula (PBM-13-1):

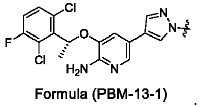

In some embodiments, PBM represents the structure of the following Formula (PBM-14), Formula (PBM-14-1A) or Formula (PBM-14-1B): 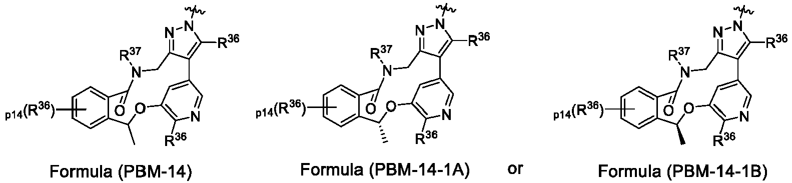 wherein
(R³⁶)ₚ₁₄ indicates that the benzene ring in Formula (PBM-14) is optionally substituted with p14 R³⁶, wherein p14 represents an integer of 1, 2 or 3, and each R³⁶ is the same or different and each independently represents halogen (e.g., fluorine, chlorine, bromine, or iodine), amino, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.); and
R³⁷ represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.).

In some embodiments, PBM represents the structure of the following Formula (PBM-14-1):

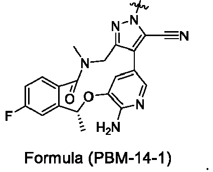

In some embodiments, PBM represents the structure of the following Formula (PBM-15): 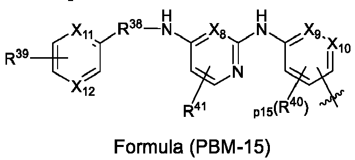 wherein
X₈, X₉, X₁₀, X₁₁ and X₁₂ in Formula (PBM-15) are the same or different and each independently represent N or CH;
R³⁸ represents a bond or optionally substituted methylene (e.g., halogenated methylene);
R³⁹ represents -C(O)NHR^{b13}, -NHC(O)-R^{b13}, -S(O)₂NH^{Rb13}, -N(R^{b14})S(O)₂R^{b13}, -S(O)₂R^{b13} or - P(O)(R^{b13})₂, wherein each R^{b13} is the same or different and each independently represents C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), and R^{b14} represents hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl);
(R⁴⁰)ₚ₁₅ indicates that the 6-membered ring containing X₉ and X₁₀ in Formula (PBM-15) is optionally substituted with p15 R⁴⁰, with each R⁴⁰ being the same or different and each independently representing halogen (e.g., fluorine, chlorine, bromine, or iodine), C₁₋₆ alkoxy (e.g., C₁₋₄ alkoxy, such as methoxy, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p15 represents an integer of 0, 1, 2, 3 or 4; and
R⁴¹ represents halogen (e.g., fluorine, chlorine, bromine, or iodine), C₁₋₆ alkoxy (e.g., C₁₋₄ alkoxy, such as methoxy, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R³⁸ in Formula (PBM-15) represents a bond. In other words, the 6-membered ring containing X₉ and X₁₀ in Formula (PBM-15) is directly connected to -NH-.

In some embodiments, R³⁸ in Formula (PBM-15) represents optionally substituted methylene (e.g., halogenated methylene).

In some embodiments, PBM represents the structure of the following Formula (PBM-15-1A), Formula (PBM-15-1B) or Formula (PBM-15-1C): 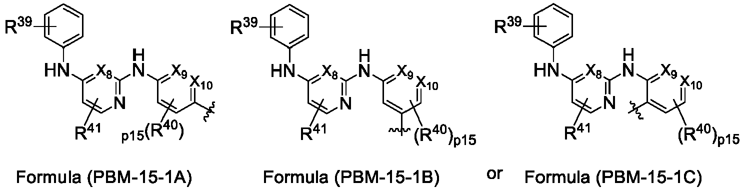 wherein
X₈, X₉ and X₁₀ are the same or different and each independently represent N or CH;
R³⁹ represents -C(O)NHR^{b13}, -NHC(O)-R^{b13}, -S(O)₂NHR^{b13}, -N(R^{b14})S(O)₂R^{b13}, -S(O)₂R^{b13} or - P(O)(R^{b13})₂, where each R^{b13} are the same or different and each independently represent C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), and R^{b14} represents hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl);
(R⁴⁰)ₚ₁₅ indicates that the 6-membered ring containing X₉ and X₁₀ is optionally substituted with p15 R⁴⁰, with each R⁴⁰ being the same or different and each independently representing halogen (e.g., fluorine, chlorine, bromine, or iodine), C₁₋₆ alkoxy (e.g., C₁₋₄ alkoxy, such as methoxy, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p15 represents an integer of 0, 1, 2, 3 or 4; and
R⁴¹ represents halogen (e.g., fluorine, chlorine, bromine, or iodine), C₁₋₆ alkoxy (e.g., C₁₋₄ alkoxy, such as methoxy, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, PBM represents the structure of the following Formula (PBM-15-1D), Formula (PBM-15-1E) or Formula (PBM-15-1F): 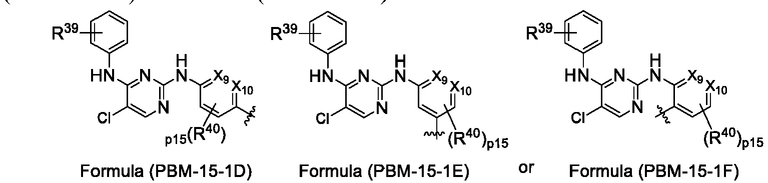 wherein
X₉ and X₁₀ are the same or different and each independently represent N or CH;
R³⁹ represents -C(O)NHR^{b13}, -NHC(O)-R^{b13}, -S(O)₂NHR^{b13}, -N(R^{b14})S(O)₂R^{b13}, -S(O)₂R^{b13} or - P(O)(R^{b13})₂, where each R^{b13} are the same or different and each independently represent C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), and R^{b14} represents hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl);
(R⁴⁰)ₚ₁₅ indicates that the 6-membered ring containing X₉ and X₁₀ is optionally substituted with p15 R⁴⁰, with each R⁴⁰ being the same or different and each independently representing halogen (e.g., fluorine, chlorine, bromine, or iodine), C₁₋₆ alkoxy (e.g., C₁₋₄ alkoxy, such as methoxy, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p15 represents an integer of 0, 1, 2, 3 or 4.

In some embodiments, PBM represents the structure of the following Formula (PBM-15-1) or Formula (PBM-15-2):

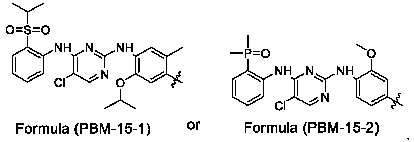

In some embodiments, PBM-R_{f}- represents a small molecule ligand targeting FAK.

In some embodiments, PBM represents the structure of the following Formula (PBM-15-1G): 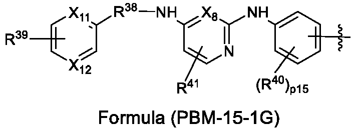 wherein
X₈, X₁₁ and X₁₂ in Formula (PBM-15-1G) are the same or different and each independently represent N or CH;
R³⁸ represents a bond or optionally substituted methylene (e.g., halogenated methylene);
R³⁹ represents -C(O)NHR^{b13}, -NHC(O)-R^{b13}, -S(O)₂NHR^{b13}, -N(R^{b14})S(O)₂R^{b13}, -S(O)₂R^{b13} or - P(O)(R^{b13})₂, where each R^{b13} are the same or different and each independently represent C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), and R^{b14} represents hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl);
(R⁴⁰)ₚ₁₅ indicates that the 6-membered ring containing X₉ and X₁₀ in Formula (PBM-15) is optionally substituted with p15 R⁴⁰, with each R⁴⁰ being the same or different and each independently representing halogen, C₁₋₆ alkoxy (e.g., C₁₋₄ alkoxy, such as methoxy, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p15 represents an integer of 0, 1, 2, 3 or 4; and
R⁴¹ represents halogen, C₁₋₆ alkoxy (e.g., C₁₋₄ alkoxy, such as methoxy, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R³⁸ in Formula (PBM-15-1G) represents a bond. In other words, the 6-membered ring containing X₁₁ and X₁₂ in Formula (PBM-15-1G) is directly connected to -NH-.

In some embodiments, R³⁸ in Formula (PBM-15-1G) represents optionally substituted methylene (e.g., halogenated methylene).

In some embodiments, PBM represents the structure of the following Formula (PBM-15-1H) or Formula (PBM-15-11): 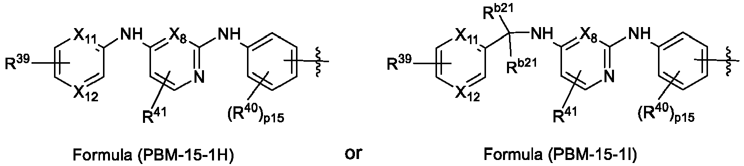 wherein
X₈, X₁₁ and X₁₂ are the same or different and each independently represent N or CH;
each R^{b21} are the same or different and each independently represent hydrogen or halogen;
R³⁹ represents -C(O)NHR^{b13}, -NHC(O)-R^{b13}, -S(O)₂NHR^{b13}, -N(R^{b14})S(O)₂R^{b13}, -S(O)₂R^{b13} or - P(O)(R^{b13})₂, where each R^{b13} are the same or different and each independently represent C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), and R^{b14} represents hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl);
(R⁴⁰)ₚ₁₅ indicates that the 6-membered ring containing X₉ and X₁₀ in Formula (PBM-15) is optionally substituted with p15 R⁴⁰, with each R⁴⁰ being the same or different and each independently representing halogen, C₁₋₆ alkoxy (e.g., C₁₋₄ alkoxy, such as methoxy, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p15 represents an integer of 0, 1, 2, 3 or 4; and
R⁴¹ represents halogen, C₁₋₆ alkoxy (e.g., C₁₋₄ alkoxy, such as methoxy, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, PBM represents the structure of the following Formula (PBM-15-6), Formula (PBM-15-7), Formula (PBM-15-8), Formula (PBM-15-9), Formula (PBM-15-10) or Formula (PBM-15-11):

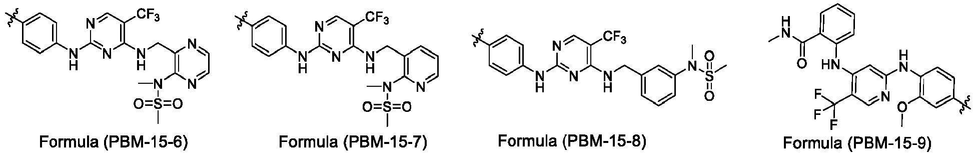

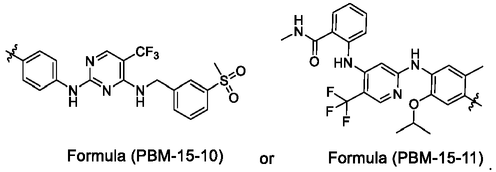

In some embodiments, PBM represents the structure of the following Formula (PBM-53): 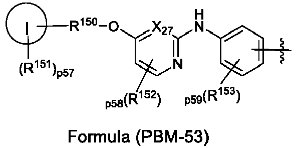
wherein X₂₇ represents N or CH;
R¹⁵⁰ represents a bond or optionally substituted methylene (e.g., halogenated methylene);
ring I represents C₆₋₁₀ aryl or 5- to 15-membered heteroaryl, (R¹⁵¹)ₚ₅₇ indicates that the ring I is optionally substituted with p57 R¹⁵¹, with each R¹⁵¹ being the same or different and each independently representing deuterium, halogen (e.g., fluorine, chlorine, bromine, or iodine), hydroxy, cyano, amino, nitro, oxo, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH_{Z}-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p57 represents an integer of 0, 1, 2, 3 or 4;
(R¹⁵²)ₚ₅₈ indicates that the 6-membered ring containing X₂₇ in Formula (PBM-53) is optionally substituted with p58 R¹⁵², with each R¹⁵² representing halogen (e.g., fluorine, chlorine, bromine, or iodine), C₁₋₆ alkoxy (e.g., C₁₋₄ alkoxy, such as methoxy, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p58 represents an integer of 0, 1 or 2;
(R¹⁵³)ₚ₅₉ indicates that the benzene ring in Formula (PBM-53) is optionally substituted with p59 R¹⁵³, with each R¹⁵³ being the same or different and each independently representing halogen (e.g., fluorine, chlorine, bromine, or iodine), C₁₋₆ alkoxy (e.g., C₁₋₄ alkoxy, such as methoxy, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p15 represents an integer of 0, 1, 2, 3 or 4.

In some embodiments, X₂₇ represents N.

In some embodiments, X₂₇ represents CH.

In some embodiments, R¹⁵⁰ represents a bond.

In some embodiments, R¹⁵⁰ represents optionally substituted methylene (e.g., methylene optionally substituted with halogen (e.g., fluorine, chlorine, bromine, or iodine)).

In some embodiments, ring I represents C₆₋₁₀ aryl or 5- to 15-membered monocyclic or bicyclic heteroaryl. In some embodiments, ring I represents C₆₋₁₀ aryl or 5- to 15-membered (e.g., 5- to 12-membered, 5- to 10-membered , 5- to 9-membered, 5- to 8-membered, 5- to 7-membered, or 6-membered) monocyclic or bicyclic heteroaryl containing from 1 to 4 (e.g., from 1 to 3, or from 1 to 2) heteroatoms selected from the group consisting of oxygen, nitrogen, and sulfur. Examples of aryl include, but are not limited to, phenyl, naphthyl. Examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, isoindolinyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[2,3-b]pyrrolyl, pyrrolo[2,1-b]thiazolyl, or imidazo[2,1-b]thiazolyl.

In some embodiments, (R¹⁵¹)ₚ₅₇ in Formula (PBM-53) indicates that the ring I is optionally substituted with p57 R¹⁵¹, with each R¹⁵¹ being the same or different and each independently representing deuterium, halogen (e.g., fluorine, chlorine, bromine, or iodine), hydroxy, cyano, amino, nitro, oxo, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH_{Z}-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p57 represents an integer of 0, 1, 2, 3 or 4.

In some embodiments, ring I represents isoindolinyl, which is optionally substituted with p57 R¹⁵¹, with each R¹⁵¹ being the same or different and each independently representing deuterium, halogen (e.g., fluorine, chlorine, bromine, or iodine), hydroxy, cyano, amino, nitro, oxo, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH_{Z}-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p57 represents an integer of 0, 1, 2, 3 or 4. In some embodiments, p57 represents an integer of 0, 1, 2, 3 or 4. In some embodiments, ring I represents isoindolinyl, which is optionally substituted with halogen (e.g., fluorine, chlorine, bromine, or iodine), oxo and C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl).

In some embodiments, (R¹⁵²)ₚ₅₈ indicates that the 6-membered ring containing X₂₇ in Formula (PBM-53) is optionally substituted with p58 R¹⁵², with each R¹⁵² representing halogen (e.g., fluorine, chlorine, bromine, or iodine), C₁₋₆ alkoxy (e.g., C₁₋₄ alkoxy, such as methoxy, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p58 represents an integer of 0, 1 or 2. In some embodiments, each R¹⁵² independently represents halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p58 represents an integer of 0, 1 or 2.

In some embodiments, (R¹⁵³)ₚ₅₉ indicates that the benzene ring in Formula (PBM-53) is optionally substituted with p59 R¹⁵³, with each R¹⁵³ being the same or different and each independently representing halogen (e.g., fluorine, chlorine, bromine, or iodine), C₁₋₆ alkoxy (e.g., C₁₋₄ alkoxy, such as methoxy, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p15 represents an integer of 0, 1, 2, 3 or 4. In some embodiments, each R¹⁵³ independently represents halogen (e.g., fluorine, chlorine, bromine, or iodine) or C₁₋₆ alkoxy (e.g., C₁₋₄ alkoxy, such as methoxy, ethoxy, propoxy, isopropoxy, butoxy, sec-butoxy, or tert-butoxy), and p15 represents an integer of 0, 1, 2, 3 or 4.

In some embodiments, PBM represents the structure of the following Formula (PBM-53-1A), Formula (PBM-53-1B), Formula (PBM-53-1C), Formula (PBM-53-2A), Formula (PBM-53-2B) or Formula (PBM-53-2C): 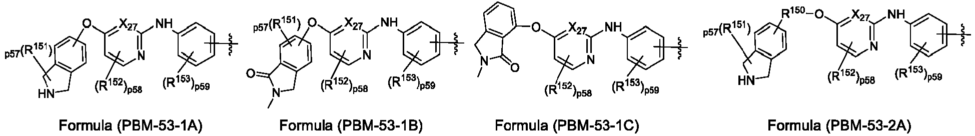 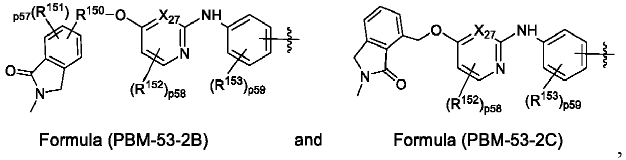 wherein X₂₇, R¹⁵⁰, (R¹⁵¹)ₚ₅₇, (R¹⁵²)ₚ₅₈ and (R¹⁵³)ₚ₅₉ are as defined in various embodiments of Formula (PBM-53) and its various sub-embodiments.

In some embodiments, PBM represents the structure of the following Formula (PBM-53-1), Formula (PBM-53-2), Formula (PBM-53-3) or Formula (PBM-53-4):

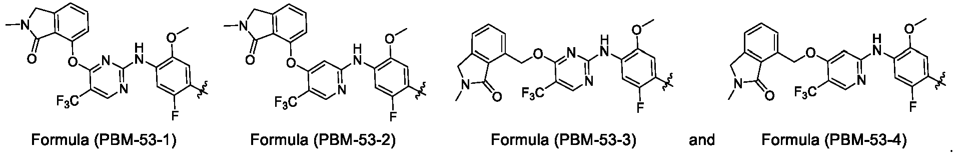

In some embodiments, PBM-R_{f}- represents a small molecule ligand targeting RET.

In some embodiments, PBM represents the structure of the following Formula (PBM-15-1J), Formula (PBM-15-1K) or Formula (PBM-15-IL): 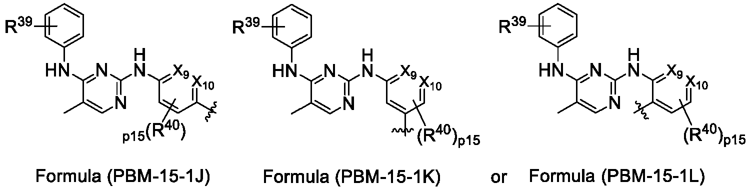 wherein
X₉ and X₁₀ are the same or different and each independently represent N or CH;
R³⁹ represents -C(O)NHR^{b13}, -NHC(O)-R^{b13}, -S(O)₂NHR^{b13}, -N(R^{b14})S(O)₂R^{b13}, -S(O)₂R^{b13} or - P(O)(R^{b13})₂, wherein each R^{b13} is the same or different and each independently represents C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), and R^{b14} represents hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl);
(R⁴⁰)ₚ₁₅ indicates that the 6-membered ring containing X₉ and X₁₀ is optionally substituted with p15 R⁴⁰, with each R⁴⁰ being the same or different and each independently representing halogen (e.g., fluorine, chlorine, bromine, or iodine), C₁₋₆ alkoxy (e.g., C₁₋₄ alkoxy, such as methoxy, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p15 represents an integer of 0, 1, 2, 3 or 4.

In some embodiments, PBM represents the structure of the following Formula (PBM-15-3), Formula (PBM-15-4) or Formula (PBM-15-5):

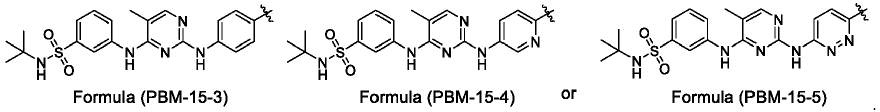

In some embodiments, PBM represents the structure of the following Formula (PBM-16-1A), Formula (PBM-16-1B), Formula (PBM-16-1C) , Formula (PBM-16-ID) or Formula (PBM-16-IE): 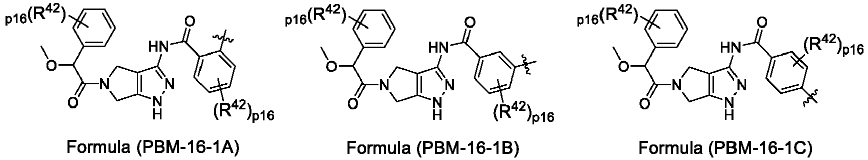 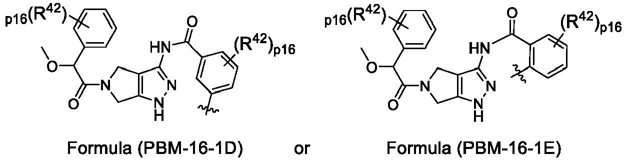 wherein
(R⁴²)ₚ₁₆ in Formula (PBM-16-1A), Formula (PBM-16-1B), Formula (PBM-16-1C), Formula (PBM-16-1D) and Formula (PBM-16-1E) each independently indicates that the benzene ring is optionally substituted with p16 R⁴², with each R⁴² being the same or different and each independently representing halogen (e.g., fluorine, chlorine, bromine, or iodine), C₁₋₆ alkoxy (e.g., C₁₋₄ alkoxy, such as methoxy, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
each p16 is the same or different and each independently represents an integer of 0, 1, 2, 3 or 4.

In some embodiments, PBM represents the structure of the following Formula (PBM-16-1):

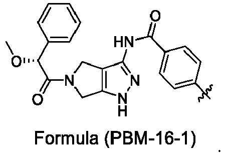

In some embodiments, PBM-R_{f}- represents a small molecule ligand targeting BET.

In some embodiments, PBM represents the structure of the following Formula (PBM-17): 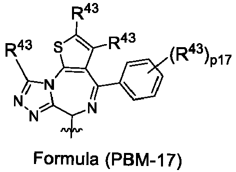 wherein
(R⁴³)ₚ₁₇ indicates that the benzene ring in Formula (PBM-17) is substituted with p17 R⁴³, with each R⁴³ being the same or different and each independently representing halogen (e.g., fluorine, chlorine, bromine, or iodine), C₁₋₆ alkoxy (e.g., C₁₋₄ alkoxy, such as methoxy, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p17 represents an integer of 0, 1, 2, 3 or 4.

In some embodiments, PBM represents the structure of the following Formula (PBM-17-1):

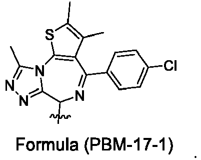

In some embodiments, PBM represents the structure of the following Formula (PBM-18-1A), Formula (PBM-18-1B), Formula (PBM-18-1C), Formula (PBM-18-1D) or Formula (PBM-18-1E): 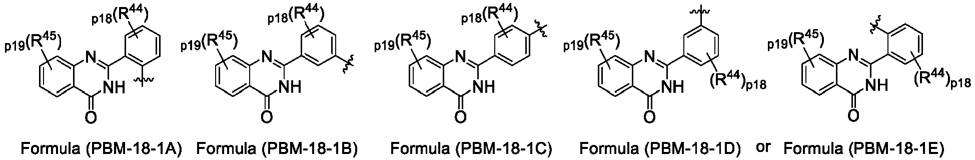 wherein
(R⁴⁴)ₚ₁₈ in Formula (PBM-18-1A), Formula (PBM-18-1B), Formula (PBM-18-1C), Formula (PBM-18-1D) and Formula (PBM-18-1E) each independently indicates that the benzene ring is substituted with p18 R⁴⁴, with each R⁴⁴ being the same or different and each independently representing halogen (e.g., fluorine, chlorine, bromine, or iodine), C₁₋₆ alkoxy (e.g., C₁₋₄ alkoxy, such as methoxy, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p18 represents an integer of 0, 1, 2, 3 or 4;
(R⁴⁵)_{P19} in Formula (PBM-18-1A), Formula (PBM-18-1B), Formula (PBM-18-1C), Formula (PBM-18-1D) and Formula (PBM-18-1E) each independently indicates that the 4-oxo-3,4-dihydroquinazoline ring is substituted with p19 R⁴⁵, with each R⁴⁵ being the same or different and each independently representing halogen (e.g., fluorine, chlorine, bromine, or iodine), C₁₋₆ alkoxy (e.g., C₁₋₄ alkoxy, such as methoxy, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p19 represents an integer of 1, 2, 3 or 4.

In some embodiments, PBM represents the structure of the following Formula (PBM-18-1):

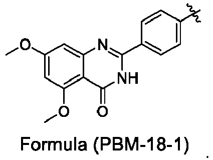

In some embodiments, PBM-R_{f}- represents a small molecule ligand targeting BCR-ABL.

In some embodiments, PBM represents the structure of the following Formula (PBM-19-1A) or Formula (PBM-19-1B): 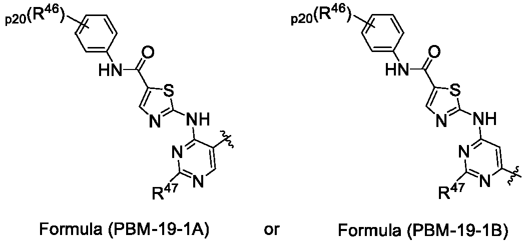 wherein
(R⁴⁶)ₚ₂₀ in Formula (PBM-19-1A) or Formula (PBM-19-1B) each independently indicates that the benzene ring is substituted with p20 R⁴⁶, with each R⁴⁶ being the same or different and each independently representing halogen (e.g., fluorine, chlorine, bromine, or iodine), C₁₋₆ alkoxy (e.g., C₁₋₄ alkoxy, such as methoxy, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p20 represents an integer of 1, 2, 3, 4 or 5; and
R⁴⁷ represents halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, PBM represents the structure of the following Formula (PBM-19-1):

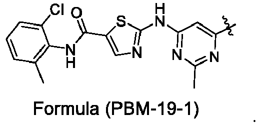

In some embodiments, PBM represents the structure of the following Formula (PBM-20-1A), Formula (PBM-20-1B), Formula (PBM-20-1C) or Formula (PBM-20-1D): 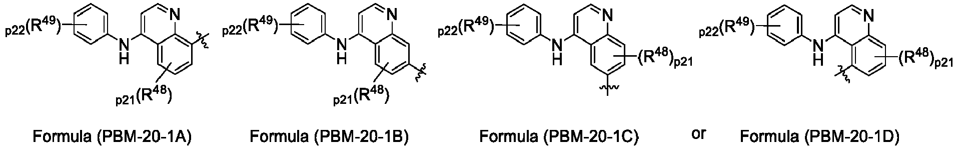 wherein
(R⁴⁸)ₚ₂₁ in Formula (PBM-20-1A), Formula (PBM-20-1B), Formula (PBM-20-1C) and Formula (PBM-20-1D) each independently indicates that the quinoline ring is substituted with p21 R⁴⁸, with each R⁴⁸ being the same or different and each independently representing halogen (e.g., fluorine, chlorine, bromine, or iodine), cyano, C₁₋₆ alkoxy (e.g., C₁₋₄ alkoxy, such as methoxy, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p21 represents an integer of 1, 2, 3 or 4;
(R⁴⁹)ₚ₂₂ in Formula (PBM-20-1A), Formula (PBM-20-1B), Formula (PBM-20-1C) and Formula (PBM-20-1D) each independently indicates that the benzene ring is substituted with p22 R⁴⁹, with each R⁴⁹ being the same or different and each independently representing halogen (e.g., fluorine, chlorine, bromine, or iodine), C₁₋₆ alkoxy (e.g., C₁₋₄ alkoxy, such as methoxy, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p22 represents an integer of 2, 3 or 4.

In some embodiments, PBM represents the structure of the following Formula (PBM-20-1):

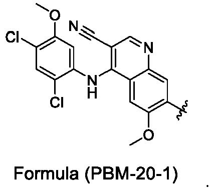

In some embodiments, PBM represents the structure of Formula (PBM-21): 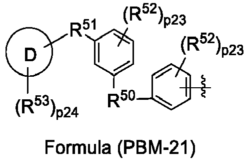 wherein
R⁵⁰ represents -NHC(O)- or -C(O)NH-;
R⁵¹ represents -NH- or ethynylene;
(R⁵²)ₚ₂₃ indicates that the benzene rings in Formula (PBM-21) are independently optionally substituted with p23 R⁵², with each R⁵² being the same or different and each independently representing halogen (e.g., fluorine, chlorine, bromine, or iodine), C₁₋₆ alkoxy (e.g., C₁₋₄ alkoxy, such as methoxy, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
each p23 is the same or different and each independently represents an integer of 0, 1, 2, 3 or 4;
ring D in Formula (PBM-21) represents 5- to 20-membered monocyclic or bicyclic heteroaryl containing from 1 to 4 heteroatoms selected from the group consisting of oxygen, nitrogen, and sulfur, and (R⁵³)ₚ₂₄ indicates that the ring D is optionally substituted with p24 R⁵³, with each R⁵³ being the same or different and each independently representing optionally substituted 5- to 15-membered heteroaryl, deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-); and
p24 represents an integer of 0, 1, 2, or 3.

In some embodiments, ring D in Formula (PBM-21) represents heteroaryl containing one or two 5- to 7-membered rings and 1 to 4 heteroatoms selected from nitrogen, oxygen, and sulfur. In a sub-embodiment, ring D represents 5- to 14-membered monocyclic or bicyclic aromatic ring group containing one or more (e.g., from 1 to 6, or from 1 to 5, or from 1 to 4, or from 1 to 3) heteroatoms selected from the group consisting of oxygen, nitrogen, and sulfur. In a sub-embodiment, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, or imidazo[1,2-b]pyridazinyl. The heteroaryl group is optionally substituted with one or more (e.g., 1-6, such as 1, 2, 3, 4, 5 or 6) substituents optionally selected from the groups consisting of halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy) or any combination thereof.

In some embodiments, R⁵³ in Formula (PBM-21) represents optionally substituted 5- to 15-membered heteroaryl. In some embodiments, 5- to 15-membered heteroaryl is 5- to 14-membered monocyclic or bicyclic aromatic ring group containing one or more (e.g., from 1 to 6, or from 1 to 5, or from 1 to 4, or from 1 to 3) heteroatoms selected from the group consisting of oxygen, nitrogen, and sulfur. In some sub-embodiments, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, or imidazo[1,2-b]pyridazinyl. The heteroaryl group is optionally substituted with one or more (e.g., 1-6, such as 1, 2, 3, 4, 5 or 6) substituents optionally selected from the groups consisting of halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy) or any combination thereof.

In some embodiments, R⁵¹ in Formula (PBM-21) represents -NH-.

In some embodiments, R⁵¹ in Formula (PBM-21) represents ethynylene.

In some embodiments, PBM represents the structure of the following Formula (PBM-21-1A), Formula (PBM-21-1B), Formula (PBM-21-1C), Formula (PBM-21-1D) or Formula (PBM-21-1E): 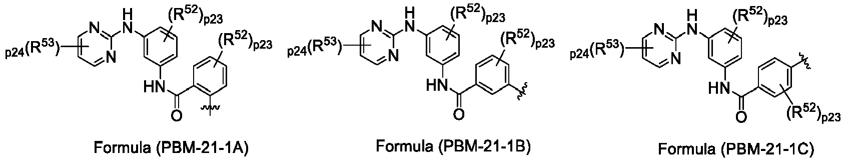 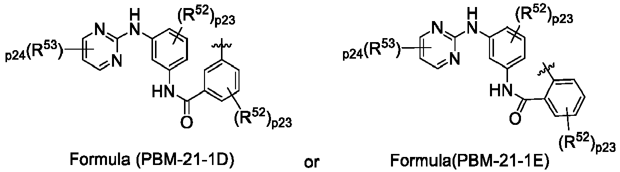 wherein
(R⁵²)ₚ₂₃ in Formula (PBM-21-1A), Formula (PBM-21-1B), Formula (PBM-21-1C), Formula (PBM-21-1D) and Formula (PBM-21-1E) each independently indicates that the benzene rings are each independently optionally substituted with p23 R⁵², with each R⁵² being the same or different and each independently representing halogen (e.g., fluorine, chlorine, bromine, or iodine), C₁₋₆ alkoxy (e.g., C₁₋₄ alkoxy, such as methoxy, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p23 represents an integer of 0, 1, 2, 3 or 4.

In some embodiments, PBM represents the structure of the following Formula (PBM-21-1):

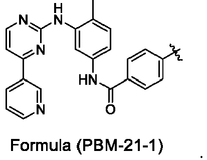

In some embodiments, PBM represents the structure of the following Formula (PBM-21-1F), Formula (PBM-21-1G), Formula (PBM-21-1H), Formula (PBM-21-1I) or Formula (PBM-21-1J): 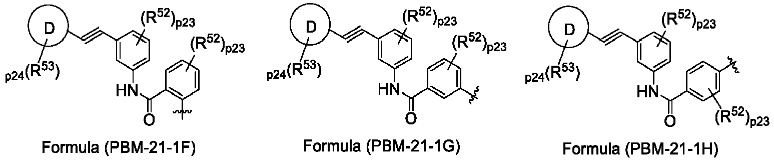 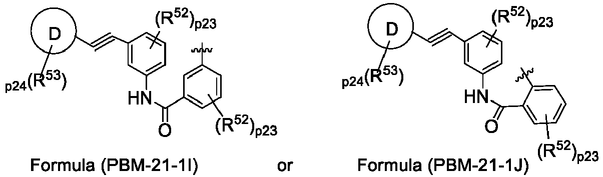 wherein
each (R⁵²)ₚ₂₃ in Formula (PBM-21-1F), Formula (PBM-21-1G), Formula (PBM-21-1H), Formula (PBM-21-1I) and Formula (PBM-21-1J) each independently indicates that the benzene rings are each independently optionally substituted with p23 R⁵², with each R⁵² being the same or different and each independently representing halogen (e.g., fluorine, chlorine, bromine, or iodine), C₁₋₆ alkoxy (e.g., C₁₋₄ alkoxy, such as methoxy, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p23 represents an integer of 0, 1, 2, 3 or 4;
each ring D in Formula (PBM-21-1F), Formula (PBM-21-1G), Formula (PBM-21-1H), Formula (PBM-21-1I) and Formula (PBM-21-1J) each independently represents 5- to 20-membered monocyclic or bicyclic heteroaryl containing from 1 to 4 heteroatoms selected from the group consisting of oxygen, nitrogen, and sulfur, and (R⁵³)ₚ₂₄ indicates that the ring D is optionally substituted with p24 R⁵³, with each R⁵³ being the same or different and each independently representing optionally substituted 5- to 15-membered heteroaryl, deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-); and
p24 represents an integer of 0, 1, 2, or 3.

In some embodiments, each ring D in Formula (PBM-21-1F), Formula (PBM-21-1G), Formula (PBM-21-1H), Formula (PBM-21-1I) and Formula (PBM-21-1J) each independently represents heteroaryl containing one or two 5- to 7-membered rings and 1 to 4 heteroatoms selected from nitrogen, oxygen, and sulfur. In a sub-embodiment, ring D represents 5- to 14-membered monocyclic or bicyclic aromatic ring group containing one or more (e.g., from 1 to 6, or from 1 to 5, or from 1 to 4, or from 1 to 3) heteroatoms selected from the group consisting of oxygen, nitrogen, and sulfur. In a sub-embodiment, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, or imidazo[1,2-b]pyridazinyl. The heteroaryl group is optionally substituted with one or more (e.g., 1-6, such as 1, 2, 3, 4, 5 or 6) substituents optionally selected from the group consisting of halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy) or any combination thereof.

In some embodiments, PBM represents the structure of Formula (PBM-21-2):

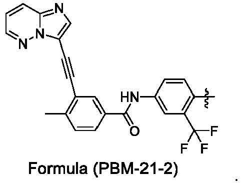

In some embodiments, PBM represents the structure of Formula (PBM-52): 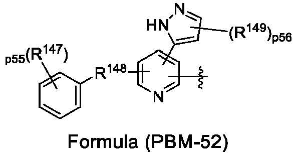 wherein
(R¹⁴⁷)ₚ₅₅ in Formula (PBM-52) indicates that the benzene ring is optionally substituted with p55 R¹⁴⁷, with each R¹⁴⁷ being independently halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy) or halogenated C₁₋₆ alkoxy (e.g., halogenated C₁₋₄ alkoxy, such as F₃C-O-, FCH₂-O-, F₂CH-O-, F₂ClC-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p55 represents an integer of 0, 1, 2, 3, 4 or 5;
R¹⁴⁸ represents NHC(O) or C(O)NH; and
(R¹⁴⁹)ₚ₅₆ indicates that the 1H-pyrazole ring in Formula (PBM-52) is optionally substituted with p56 R¹⁴⁹, with each R¹⁴⁹ being independently halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy) or halogenated C₁₋₆ alkoxy (e.g., halogenated C₁₋₄ alkoxy, such as F₃C-O-, FCH₂-O-, F₂CH-O-, F₂ClC-O-, ClCH₂-O-, Cl₂CH-O-, CF₃CF₂-O-, CF₃CHF-O-, CHF₂CF₂-O-, CHF₂CHF-O-, CF₃CH₂-O-or CH₂ClCH₂-O-), and p56 represents an integer of 0, 1 or 2.

In some embodiments, (R¹⁴⁷)ₚ₅₅ in Formula (PBM-52) indicates that the benzene ring is substituted with p55 R¹⁴⁷, wherein p55 represents an integer of 1, and R¹⁴⁷ is halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy) or halogenated C₁₋₆ alkoxy (e.g., halogenated C₁₋₄ alkoxy, such as F₃C-O-, FCH₂-O-, F₂CH-O-, F₂ClC-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In some embodiments, R¹⁴⁷ is F₂ClC-O-.

In some embodiments, R¹⁴⁸ represents NHC(O). In some embodiments, R¹⁴⁸ represents C(O)NH.

In some embodiments, p56 represents an integer of 0.

In some embodiments, PBM represents the structure of the following Formula (PBM-52-1A) or Formula (PBM-52-1B): 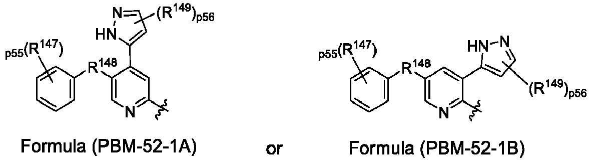 wherein (R¹⁴⁷)ₚ₅₅, R¹⁴⁸ and (R¹⁴⁹)ₚ₅₆ are as defined in various embodiments of Formula (PBM-52) and its various sub-embodiments.

In some embodiments, PBM represents the structure of the following Formula (PBM-52-1):

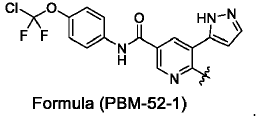

In some embodiments, PBM-R_{f}- represents a small molecule ligand targeting ER.

In some embodiments, PBM represents the structure of the following Formula (PBM-22-1A), Formula (PBM-22-1B), Formula (PBM-22-1C) or Formula (PBM-22-1D): 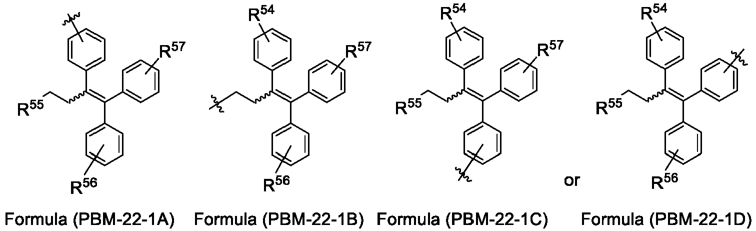 wherein
R⁵⁴, R⁵⁵, R⁵⁶ and R⁵⁷ are the same or different and each independently represent hydrogen, halogen (e.g., fluorine, chlorine, bromine, or iodine), or hydroxy; and
symbol " 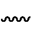 " connected to a double bond in Formula (PBM-22-1A), Formula (PBM-22-1B), Formula (PBM-22-1C) and Formula (PBM-22-1D) represents a bond which can be in stereo-configuration (cis or trans configuration, or E- or Z-configuration).

In some embodiments, PBM represents the structure of the following Formula (PBM-22-1), Formula (PBM-22-2), Formula (PBM-22-3), Formula (PBM-22-4), Formula (PBM-22-5), Formula (PBM-22-6), Formula (PBM-22-7), Formula (PBM-22-8), Formula (PBM-22-9) or Formula (PBM-22-10):

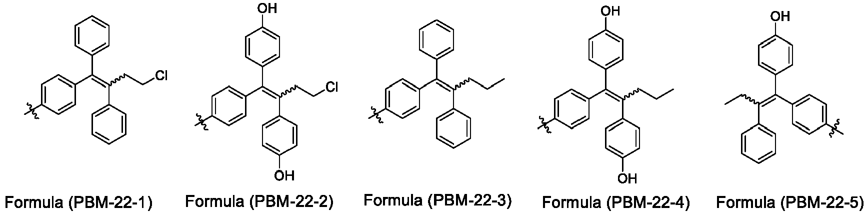

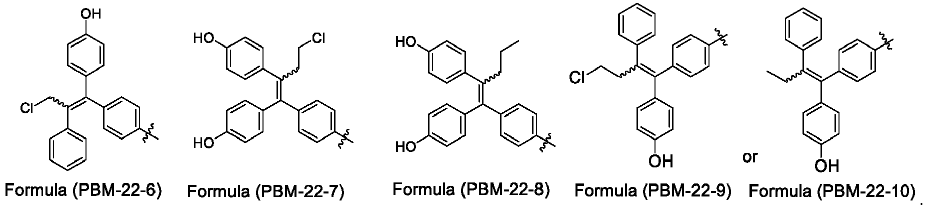

In some embodiments, PBM represents the structure of the following Formula (PBM-23-1A) or Formula (PBM-23-1B): 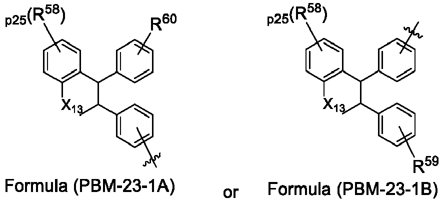 wherein
each X₁₃ in Formula (PBM-23-1A) and Formula (PBM-23-1B) each independently represents -O- or -CH₂-;
each (R⁵⁸)ₚ₂₅ in Formula (PBM-23-1A) and Formula (PBM-23-1B) each independently indicates that 1,2,3,4-tetrahydronaphthalene ring is substituted with p25 R⁵⁸, with each R⁵⁸ being the same or different and each independently representing hydrogen, hydroxy, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p25 represents an integer of 1, 2, 3 or 4; and
R⁵⁹ and R⁶⁰ each independently represents hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, PBM represents the structure of the following Formula (PBM-23-1), Formula (PBM-23-2), Formula (PBM-23-3), Formula (PBM-23-4), Formula (PBM-23-5), Formula (PBM-23-6), Formula (PBM-23-7) or Formula (PBM-23-8):

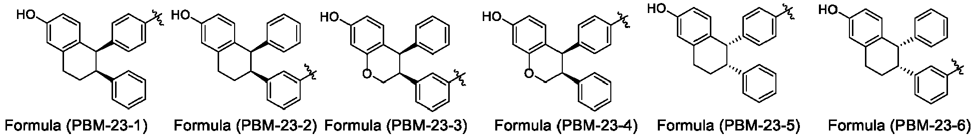

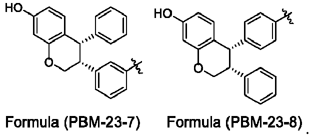

In some embodiments, PBM-R_{f}- represents a small molecule ligand targeting IRAK4.

In some embodiments, PBM represents the structure of the following Formula (PBM-24): 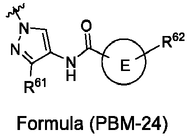 wherein
ring E in Formula (PBM-24) represents 5- to 20-membered monocyclic or bicyclic heteroarylene containing from 1 to 4 heteroatoms selected from the group consisting of oxygen, nitrogen, and sulfur;
R⁶² represents optionally substituted 5- to 15-membered heteroaryl, optionally substituted 5- to 15-membered heterocyclyl, deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-); and
R⁶¹ represents halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.).

In some embodiments, ring E in Formula (PBM-24) represents 5- to 20-membered monocyclic or bicyclic heteroarylene containing from 1 to 4 heteroatoms selected from the group consisting of oxygen, nitrogen, and sulfur. In some embodiments, heteroarylene is 5- to 14-membered monocyclic or bicyclic bivalent aromatic ring group containing from 1 to 4 (e.g., from 1 to 4, or from 1 to 3, or from 1 to 2, or 1) heteroatoms selected from the group consisting of oxygen, nitrogen, and sulfur. In some sub-embodiments, examples of heteroarylene include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, imidazo[2, 1-b]thiazolylene and imidazo[1,2-b]pyridazinylene. Heteroarylene is optionally substituted with one or more (e.g., 1-3, such as 1, 2, or 3) substituents optionally selected from the group consisting of halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy) or any combination thereof.

In some embodiments, R⁶² in Formula (PBM-24) represents optionally substituted 5- to 15-membered heteroaryl. In some embodiments, 5- to 15-membered heteroaryl is 5- to 15-membered monocyclic or bicyclic aromatic ring group containing from 1 to 4 (e.g., from 1 to 4, or from 1 to 3, or from 1 to 2, or 1) heteroatoms selected from the group consisting of oxygen, nitrogen, and sulfur. In some sub-embodiments, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, or imidazo[1,2-b]pyridazinyl. R⁶² represented by heteroaryl is optionally substituted with one or more (e.g., 1-3, such as 1, 2, or 3) subsituents optionally selected from the group consisting of: -N(R^{b22})-C₁₋₆ alkylene-optionally substituted C₃₋₈ cycloalkyl, -N(R^{b22})-optionally halogenated C₁₋₆ alkyl, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy) or any combination thereof, wherein each R^{b22} independently represents hydrogen or C₁₋₃ alkyl. In some embodiments, R⁶² represented by heteroaryl is substituted with a subsituent: -N(R^{b22})-C₁₋₆ alkylene-optionally substituted C₃₋₈ cycloalkyl, wherein R^{b22} represents hydrogen or C₁₋₃ alkyl (e.g., methyl). In some embodiments, examples of C₁₋₆ alkylene include, but are not limited to, C₁₋₃ alkylene . In some embodiments, examples of C₃₋₈ cycloalkyl include, but are not limited to, C₃₋₆ cycloalkyl. In some embodiments, representative examples of C₃₋₈ cycloalkyl include, but are not limited to, cyclopropyl, cyclobutyl, cyclopentyl, cyclopentenyl, cyclohexyl, cyclohexenyl, cycloheptyl and cyclooctyl. C₃₋₈ cycloalkyl is optionally substituted with one or more (e.g., 1-3 , e.g., 1, 2, or 3) substituents selected from the group consisting of C₁-C₃ alkyl, C₃₋₆ cycloalkyl, hydroxy, amino, mercapto, halogen, C₁-C₃ alkoxy, C₁-C₃ alkylamino, halogenated C₁-C₃ alkyl, amino-substituted C₁₋₃ alkylene, C₁₋₃ alkyl-NHC(O)-, C₁₋₃ alkyl-C(O)NH-, cyano or any combination thereof. In some embodiments, R⁶² represented by heteroaryl is substituted with a substituent: -N(R^{b22})-optionally halogenated C₁₋₆ alkyl, wherein R^{b22} represents hydrogen or C₁₋₃ alkyl (e.g., methyl). In some embodiments, examples of optionally halogenated C₁₋₆ alkyl include, but are not limited to, optionally halogenated C₁₋₄ alkyl, optionally halogenated C₁₋₃ alkyl and optionally halogenated C₁₋₂ alkyl. In some embodiments, representative examples of optionally halogenated C₁₋₆ alkyl include, but are not limited to, -CH₂-CF₃.

In some embodiments, R⁶² in Formula (PBM-24) represents optionally substituted 5- to 15-membered heterocyclyl. In some sub-embodiments, "5- to 15-membered heterocyclyl" refers to 5- to 15-membered saturated or partially unsaturated (i.e., containing one or more double bonds, but not having a fully conjugated π-electron system) monocyclic, bicyclic, or tricyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. In some sub-embodiments, examples of heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl (e.g., 1,4-diazacycloheptan-1-yl), and diazacyclooctyl. The heterocyclyl group is optionally substituted with one or more (e.g., from 1 to 5, or from 1 to 4, or from 1 to 3, or from 1 to 2, or 1) substituents selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

In some embodiments, PBM represents the structure of the following Formula (PBM-24-1) or Formula (PBM-24-2):

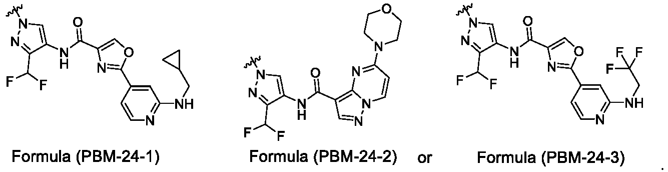

In some embodiments, PBM represents the structure of the following Formula (PBM-25): 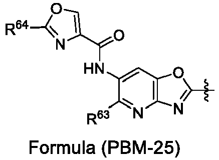 wherein
R⁶³ in Formula (PBM-25) represents optionally substituted 5- to 15-membered heterocyclyl, deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-); and
R⁶⁴ represents 5- to 20-membered monocyclic or bicyclic heteroaryl containing from 1 to 4 heteroatoms selected from the group consisting of oxygen, nitrogen, and sulfur, wherein the heteroaryl is optionally substituted with one or more (e.g., 1-6, such as 1, 2, 3, 4, 5 or 6) selected from the group consisting of deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or any combination thereof.

In some embodiments, R⁶³ in Formula (PBM-25) represents optionally substituted 5- to 15-membered heterocyclyl. In some sub-embodiments, "5- to 15-membered heterocyclyl" refers to 5- to 15-membered saturated or partially unsaturated (i.e., containing one or more double bonds, but not having a fully conjugated π-electron system) monocyclic, bicyclic, or tricyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. In some sub-embodiments, examples of heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl (e.g., 1,4-diazacycloheptan-1-yl), and diazacyclooctyl. The heterocyclyl group is optionally substituted with one or more (e.g., from 1 to 5, or from 1 to 4, or from 1 to 3, or from 1 to 2, or 1) substituents selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

In some embodiments, R⁶⁴ in Formula (PBM-25) represents optionally substituted 5- to 20-membered (e.g., 5- to 15-membered, 5- to 10-membered or 5- to 6-membered) monocyclic or bicyclic heteroaryl containing from 1 to 4 heteroatoms selected from the group consisting of oxygen, nitrogen, and sulfur. In some sub-embodiments, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, or imidazo[1,2-b]pyridazinyl. The heteroaryl is optionally substituted with one or more (e.g., 1-3 , such as 1, 2, or 3) substituents optionally selected from the group consisting of: halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy) or any combination thereof.

In some embodiments, PBM represents the structure of the following Formula (PBM-25-1), Formula (PBM-25-2) or Formula (PBM-25-3):

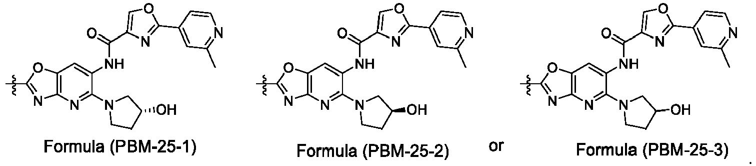

In some embodiments, PBM represents the structure of the following Formula (PBM-26-1A) or Formula (PBM-26-1B): 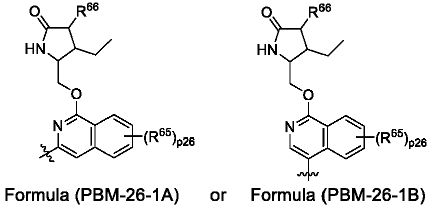 wherein
each (R⁶⁵)ₚ₂₆ in Formula (PBM-26-1A) and Formula (PBM-26-1B) each independently indicates that the isoquinoline ring is substituted with p26 R⁶⁵, with each R⁶⁵ being the same or different and each independently representing hydrogen, hydroxy, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, butoxy, sec-butoxy, or tert-butoxy), -C(O)NH₂, deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p26 represents an integer of 1, 2, 3 or 4; and
R⁶⁶ groups are the same or different and each independently represent hydrogen, hydroxy, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, PBM represents the structure of the following Formula (PBM-26-1):

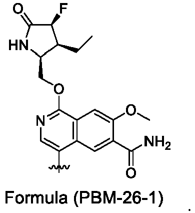

In some embodiments, PBM represents the structure of the following Formula (PBM-27): 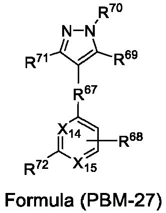 wherein
X₁₄ in Formula (PBM-27) represents N or CR^{b23}, where R^{b23} represents hydrogen or halogen (e.g., fluorine, chlorine, bromine, or iodine), and X₁₅ represents N or CH;
R⁶⁷ represents a bond, -C(O)NH- or -NHC(O)-;
R⁶⁸ represents halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
R⁶⁹ represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂-etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or -C₁₋₃ alkylene-C₃₋₆ cycloalkyl (e.g., -methylene-cyclopropyl);
R⁷⁰ represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂-etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); or
R⁶⁹ and R⁷⁰ together with the corresponding nitrogen and carbon atoms to which they are connected form an optionally substituted 5- to 7-membered nitrogen-containing heterocycle; and
R⁷¹ represents hydrogen or a bond, R⁷² represents hydrogen or a bond, wherein R⁷¹ and R⁷² are not simultaneously hydrogen, and only one of R⁷¹ and R⁷² represents a bond, and another represents hydrogen, wherein when R⁷¹ represents a bond, the carbon atom on the ring connected to R⁷¹ is directly connected to R_{f}, and when R⁷² represents a bond, the carbon atom on the ring connected to R⁷² is directly connected to R_{f}.

In some embodiments, R⁶⁷ in Formula (PBM-27) represents a bond.

In some embodiments, R⁶⁷ in Formula (PBM-27) represents -C(O)NH- or -NHC(O)-.

In some embodiments, R⁶⁹ in Formula (PBM-27) represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or -C₁₋₃ alkylene-C₃₋₆ cycloalkyl; and
R⁷⁰ represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂-etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R⁶⁹ and R⁷⁰ in Formula (PBM-27) together with the corresponding nitrogen and carbon atoms to which they are connected form an optionally substituted 5- to 7-membered nitrogen-containing heterocycle. In some embodiments, 5- to 7-membered nitrogen-containing heterocycle includes, but is not limited to, e.g., 5- to 7-membered (e.g., 5- to 6-membered, 5-membered) heterocycle containing one nitrogen atom and optional heteroatoms selected from the group consisting of oxygen, nitrogen, and sulfur. The 5-7 membered nitrogen-containing heterocycle fused with the pyrazole ring in Formula (PBM-27) to form a fused ring. The 5- to 7-membered nitrogen-containing heterocycle is optionally substituted with one or more (e.g., from 1 to 6, or from 1 to 5, or from 1 to 4, from 1 to 3, or 2) substituents selected from the group consisting of: halogen (e.g., fluorine, chlorine, bromine, or iodine), hydroxy, amino, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.).

In some embodiments, the 5-7 membered nitrogen-containing heterocycle formed by R⁶⁹ and R⁷⁰ together with the corresponding nitrogen and carbon atoms to which they are connected is fused with the adjacent pyrazole ring to form the following group:

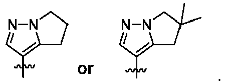

In some embodiments, PBM represents the structure of the following Formula (PBM-27-1A): 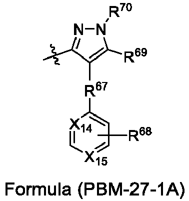 wherein
X₁₄ represents N or CR^{b23}, where R^{b23} represents hydrogen or halogen (e.g., fluorine, chlorine, bromine, or iodine), and X₁₅ represents N or CH;
R⁶⁷ represents -C(O)NH- or -NHC(O)-;
R⁶⁸ represents halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
R⁶⁹ represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂-etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or -C₁₋₃ alkylene-C₃₋₆ cycloalkyl (e.g., -methylene-cyclopropyl);
R⁷⁰ represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂-etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);or
R⁶⁹ and R⁷⁰ together with the corresponding nitrogen and carbon atoms to which they are connected form an optionally substituted 5- to 7-membered nitrogen-containing heterocycle.

In some embodiments, PBM represents the structure of the following Formula (PBM-27-1B): 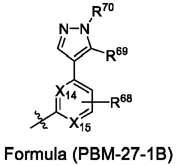 wherein
X₁₄ represents N or CR^{b23}, where R^{b23} represents hydrogen or halogen (e.g., fluorine, chlorine, bromine, or iodine), and X₁₅ represents N or CH;
R⁶⁸ represents halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
R⁶⁹ represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂-etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or -C₁₋₃ alkylene-C₃₋₆ cycloalkyl (e.g., -methylene-cyclopropyl);
R⁷⁰ represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂-etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);or
R⁶⁹ and R⁷⁰ together with the corresponding nitrogen and carbon atoms to which they are connected form an optionally substituted 5- to 7-membered nitrogen-containing heterocycle.

In some embodiments, R⁶⁹ in Formula (PBM-27-1A) and Formula (PBM-27-1B) each independently represent hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or -C₁₋₃ alkylene-C₃₋₆ cycloalkyl (e.g., -methylene-cyclopropyl).

In some embodiments, R⁷⁰ in Formula (PBM-27-1A) and Formula (PBM-27-1B) each independently represent hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R⁶⁹ and R⁷⁰ in Formula (PBM-27-1A) each independently represent hydrogen.

In some embodiments, R⁶⁹ in Formula (PBM-27-1B) represents -methylene-cyclopropyl, and R⁷⁰ represents methyl.

In some embodiments of Formula (PBM-27-1A) and Formula (PBM-27-1B), the nitrogen-containing heterocycle formed by R⁶⁹ and R⁷⁰ together with the corresponding nitrogen and carbon atoms to which they are connected is fused with the adj acent pyrazole ring to form the following group:

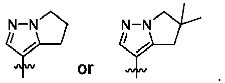

In some embodiments, PBM represents the structure of the following Formula (PBM-27-1), Formula (PBM-27-2) or Formula (PBM-27-3):

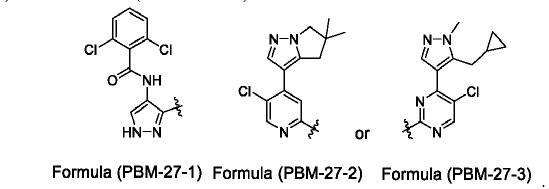

In some embodiments, PBM represents the structure of the following Formula (PBM-28-1A), Formula (PBM-28-1B), Formula (PBM-28-1C), Formula (PBM-28-1D) or Formula (PBM-28-1E): 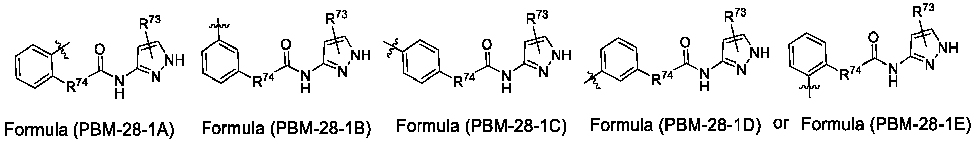 wherein
each R⁷⁴ in Formula (PBM-28-1A), Formula (PBM-28-1B), Formula (PBM-28-1C), Formula (PBM-28-1D) and Formula (PBM-28-1D) each independently represents a bond, C₁₋₃ alkylene (e.g., methylene, ethylene or propylene) or halogenated C₁₋₃ alkylene (e.g., halogenated methylene, halogenated ethylene or halogenated propylene); and
R⁷³ represents optionally substituted C₃₋₆ cycloalkyl (e.g., optionally substituted cyclopropyl, optionally substituted cyclobutyl, optionally substituted cyclopentyl or optionally substituted cyclohexyl), halogenated C₃₋₆ cycloalkyl (e.g., halogenated cyclopropyl, halogenated cyclobutyl, halogenated cyclopentyl or halogenated cyclohexyl), or optionally substituted C₁₋₆ alkyl (e.g., optionally substituted C₁₋₅ alkyl, optionally substituted C₁₋₄ alkyl or optionally substituted C₁₋₃ alkyl, e.g., the following optionally substituted groups: methyl, ethyl, propyl, isopropyl, butyl, sec-butyl, tert-butyl, pentyl, or hexyl). The substituents of the optionally substituted C₁₋₆ alkyl include, but are not limited to, e.g., deuterium, hydroxy, amino, mercapto, nitro, halogen (e.g., fluorine, chlorine, bromine, or iodine), cyano, optionally deuterated C₁₋₄ alkyl (e.g., optionally deuterated C₁₋₃ alkyl, such as methyl, CD₃, ethyl, propyl, isopropyl, butyl, sec-butyl, or tert-butyl), halogenated C₁₋₄ alkyl (e.g., halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), optionally substituted C₆₋₁₀ aryl(e.g., optionally substituted phenyl or optionally substituted naphthyl), or any combination thereof. Examples of optionally substituted C₁₋₆ alkyl include, but are not limited to, e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl; deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.); halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); or -optionally substituted C₁₋₆ alkylene-optionally substituted C₅₋₁₀ aryl. Examples of -optionally substituted C₁₋₆ alkylene-optionally substituted C₅₋₁₀ aryl include, but are not limited to, e.g., -optionally substituted C₁₋₆ alkylene-optionally substituted C₆₋₁₀ aryl, or - optionally substituted C₁₋₆ alkylene-optionally substituted phenyl, wherein the aryl is optionally substituted with one or more (e.g., 1-4, or 2-3) substituents selected from the group consisting of e.g., deuterium, hydroxy, amino, mercapto, nitro, halogen (e.g., fluorine, chlorine, bromine, or iodine), cyano, optionally deuterated C₁₋₄ alkyl (e.g., optionally deuterated C₁₋₃ alkyl, such as methyl, CD₃, ethyl, propyl, isopropyl, butyl, sec-butyl, or tert-butyl), halogenated C₁₋₄ alkyl (e.g., halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), or any combination thereof; and the C₁₋₆ alkylene is optionally substituted with one or more (e.g., 1-4, or 2-3) substituents selected from the group consisting of e.g., deuterium, hydroxy, amino, mercapto, nitro, halogen (e.g., fluorine, chlorine, bromine, or iodine), cyano, optionally deuterated C₁₋₄ alkyl (e.g., optionally deuterated C₁₋₃ alkyl, such as methyl, CD₃, ethyl, propyl, isopropyl, butyl, sec-butyl, or tert-butyl), halogenated C₁₋₄ alkyl (e.g., halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), or any combination thereof.

In some embodiments, each R⁷⁴ in Formula (PBM-28-1A), Formula (PBM-28-1B), Formula (PBM-28-1C), Formula (PBM-28-1D) and Formula (PBM-28-1D) each independently represents C₁₋₃ alkylene (e.g., methylene, ethylene or propylene) or halogenated C₁₋₃ alkylene (e.g., halogenated methylene, halogenated ethylene or halogenated propylene).

In some embodiments, each R⁷³ in Formula (PBM-28-1A), Formula (PBM-28-1B), Formula (PBM-28-1C), Formula (PBM-28-1D) and Formula (PBM-28-1D) each independently represents optionally substituted C₃₋₆ cycloalkyl, e.g., optionally substituted cyclopropyl, optionally substituted cyclobutyl, optionally substituted cyclopentyl or optionally substituted cyclohexyl. In some embodiments, C₃₋₆ cycloalkyl is optionally substituted with one or more (e.g., 1-3, or 1, 2 or 3) substituents selected from the group consisting of: deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

In some embodiments, each R⁷³ in Formula (PBM-28-1A), Formula (PBM-28-1B), Formula (PBM-28-1C), Formula (PBM-28-1D) and Formula (PBM-28-1D) each independently represents optionally substituted C₁₋₆ alkyl. The substituents of the optionally substituted C₁₋₆ alkyl include, but are not limited to, e.g., deuterium, hydroxy, amino, mercapto, nitro, halogen (e.g., fluorine, chlorine, bromine, or iodine), cyano, optionally deuterated C₁₋₄ alkyl (e.g., optionally deuterated C₁₋₃ alkyl, such as methyl, CD₃, ethyl, propyl, isopropyl, butyl, sec-butyl, or tert-butyl), halogenated C₁₋₄ alkyl (e.g., halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), optionally substituted C₅₋₁₀ aryl (e.g., optionally substituted phenylor optionally substituted naphthyl), or any combination thereof. Examples of optionally substituted include, but are not limited to, e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl; deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.); halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); or -optionally substituted C₁₋₆ alkylene-optionally substituted C₅₋₁₀ aryl. In some embodiments, examples of -optionally substituted C₁₋₆ alkylene-optionally substituted C₆₋₁₀ aryl include, but are not limited to, e.g., -optionally substituted C₁₋₆ alkylene-optionally substituted C₆₋₁₀ aryl, or -optionally substituted C₁₋₆ alkylene-optionally substituted phenyl, wherein the aryl or phenyl is optionally substituted with one or more (e.g., 1-4, or 2-3) substituents selected from the group consisting of e.g., deuterium, hydroxy, amino, mercapto, nitro, halogen (e.g., fluorine, chlorine, bromine, or iodine), cyano, optionally deuterated C₁₋₄ alkyl (e.g., optionally deuterated C₁₋₃ alkyl, such as methyl, CD₃, ethyl, propyl, isopropyl, butyl, sec-butyl, or tert-butyl), halogenated C₁₋₄ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), or any combination thereof; and the C₁₋₆ alkylene is optionally substituted with one or more (e.g., 1-4, or 2-3) substituents selected from the group consisting of e.g., deuterium, hydroxy, amino, mercapto, nitro, halogen (e.g., fluorine, chlorine, bromine, or iodine), cyano, optionally deuterated C₁₋₄ alkyl (e.g., optionally deuterated C₁₋₃ alkyl, such as methyl, CD₃, ethyl, propyl, isopropyl, butyl, sec-butyl, or tert-butyl), halogenated C₁₋₄ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), or any combination thereof.

In some embodiments, each R⁷³ in Formula (PBM-28-1A), Formula (PBM-28-1B), Formula (PBM-28-1C), Formula (PBM-28-1D) and Formula (PBM-28-1D) each independently represents cyclobutyl.

In some embodiments, PBM represents the structure of the following Formula (PBM-28-1):

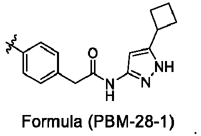

In some embodiments, PBM represents the structure of the following Formula (PBM-51): 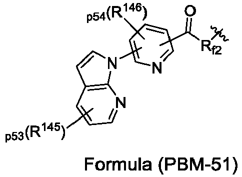 wherein
R_{f2} in Formula (PBM-51) represents a bond, or R_{f2} represents -NH-R_{f3}-C(O)-***, where symbol *** indicates the point of attachment to R_{f1}, and R_{f3} represents optionally substituted C₃₋₈ cycloalkyl;
(R¹⁴⁵)ₚ₅₃ indicates that the 1H-pyrrolo[2,3-b]pyridine ring in Formula (PBM-51) is optionally substituted with p53 R¹⁴⁵, with each R¹⁴⁵ being independently cyano, halogen (e.g., fluorine, chlorine, bromine, or iodine), hydroxy, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, F₂ClC-O-, C1₂CH-O-, CF₃CF₂-O-, CF₃CHF-O-, CHF₂CF₂-O-, CHF₂CHF-O-, CF₃CH₂-O- or CH₂ClCH₂-O-), and p53 represents an integer of 0, 1, 2, 3, 4 or 5; and
(R¹⁴⁶)ₚ₅₄ indicates that the pyridine ring in Formula (PBM-51) is optionally substituted with p54 R¹⁴⁶, with each R¹⁴⁶ being independently cyano, halogen (e.g., fluorine, chlorine, bromine, or iodine), hydroxy, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or NR^{b25}R^{b26}, where R^{b25} and R^{b26} are the same or different and each independently represent hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally substituted C₃₋₆ cycloalkyl (e.g., optionally substituted cyclopropyl, optionally substituted cyclobutyl, optionally substituted cyclopentyl or optionally substituted cyclohexyl) or optionally substituted 4- to 8-membered heterocyclyl (e.g., azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl, azacyclooctyl, and diazacyclooctyl; and the 4- to 8-membered heterocyclyl is optionally substituted with a substituent selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, 3-methyl-2-oxoimidazolidin-1-yl or any combination thereof), and p54 represents an integer of 0, 1, 2 or 3.

In some embodiments, R_{f2} in Formula (PBM-51) represents a bond.

In some embodiments, R_{f2} in Formula (PBM-51) represents -NH-R_{f3}-C(O)-***, wherein symbol *** indicates the point of attachment to R_{f1}, and R_{f3} represents optionally substituted C₃₋₈ cycloalkyl. In some embodiments, examples of optionally substituted C₃₋₈ cycloalkyl include, but are not limited to, e.g., optionally substituted cyclopropyl, optionally substituted cyclobutyl, optionally substituted cyclopentyl, optionally substituted cyclohexyl, optionally substituted cycloheptyl or optionally substituted cyclooctyl. In some embodiments, C₃₋₈ cycloalkyl is optionally substituted with one or more (e.g., 1-3, or 1, 2 or 3) substituents selected from the group consisting of: deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

In some embodiments, (R¹⁴⁵)ₚ₅₃ indicates that the 1H-pyrrolo[2,3-b]pyridine ring in Formula (PBM-51) is optionally substituted with p53 R¹⁴⁵, with each R¹⁴⁵ being independently cyano, halogen (e.g., fluorine, chlorine, bromine, or iodine), hydroxy, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p53 represents an integer of 0, 1, 2, 3, 4 or 5.

In some embodiments, (R¹⁴⁵)ₚ₅₃ indicates that the 1H-pyrrolo[2,3-b]pyridine ring in Formula (PBM-51) is substituted with p53 R¹⁴⁵, wherein p53 represents an integer of 1, and R¹⁴⁵ is cyano.

In some embodiments, (R¹⁴⁶)ₚ₅₄ indicates that the pyridine ring in Formula (PBM-51) is optionally substituted with p54 R¹⁴⁶, with each R¹⁴⁶ being independently cyano, halogen (e.g., fluorine, chlorine, bromine, or iodine), hydroxy, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or NR^{b25}R^{b26}, where R^{b25} and R^{b26} are the same or different and each independently represent hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally substituted C₃₋₆ cycloalkyl (e.g., optionally substituted cyclopropyl, optionally substituted cyclobutyl, optionally substituted cyclopentyl or optionally substituted cyclohexyl) or optionally substituted 4- to 8-membered heterocyclyl (e.g., azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl, azacyclooctyl, or diazacyclooctyl; and the 4- to 8-membered heterocyclyl is optionally substituted with a substituent selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, 3-methyl-2-oxoimidazolidin-1-yl or any combination thereof), and p54 represents an integer of 0, 1, 2 or 3.

In some embodiments, (R¹⁴⁶)ₚ₅₄ indicates that the pyridine ring in Formula (PBM-51) is substituted with p54 R¹⁴⁶, wherein p54 represents an integer of 1, and R¹⁴⁶ is NR^{b25}R^{b26}, where R^{b25} represents hydrogen, and R^{b26} represents C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl).

In some embodiments, PBM represents the structure of the following Formula (PBM-51-1), Formula (PBM-51-2) or Formula (PBM-51-3):

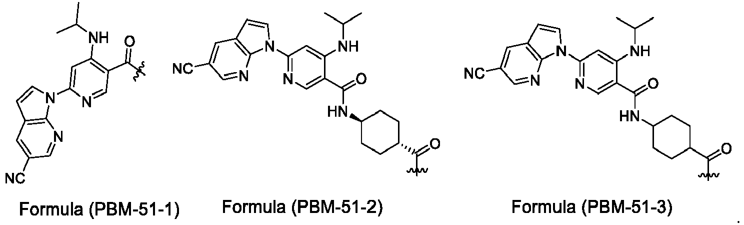

In some embodiments, PBM represents the structure of the following Formula (PBM-56): 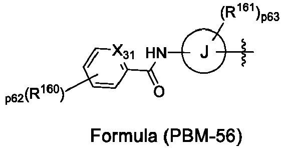 wherein
X₃₁ in Formula (PBM-56) represents N or CH;
(R¹⁶⁰)ₚ₆₂ indicates that the aromatic ring containing X₃₁ in Formula (PBM-56) is substituted with p62 R¹⁶⁰, with each R¹⁶⁰ being the same or different and each independently representing halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., deuterated C₁₋₃ alkyl, such as CD₃), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy, and p62 represents an integer of 0, 1, 2, 3 or 4;
ring J represents 5- to 15-membered heteroarylene, and (R¹⁶¹)ₚ₆₃ indicates that the ring J is substituted with p63 R¹⁶¹, with each R¹⁶¹ being the same or different and each independently representing halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., deuterated C₁₋₃ alkyl, such as CD₃), hydroxy substituted C₁₋₆ alkyl (e.g., hydroxy substituted C₁₋₄ alkyl, such as hydroxy substituted propyl), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₃ alkoxy, such as trifluoromethoxy), and p63 represents an integer of 0, 1, 2 or 3;

In some embodiments, X₃₁ represents N. In some embodiments, X₃₁ represents CH.

In some embodiments, (R¹⁶⁰)ₚ₆₂ indicates that the aromatic ring containing X₃₁ in Formula (PBM-56) is substituted with p62 R¹⁶⁰, with each R¹⁶⁰ being the same or different and each independently representing halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., deuterated C₁₋₃ alkyl, such as CD₃), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy, and p62 represents an integer of 0, 1, 2, 3 or 4, and the aromatic ring containing X₃₁ is pyridine ring or benzene ring. In some embodiments, the aromatic ring containing X₃₁ is pyridine ring, and R¹⁶⁰ represents F₃C-, and p62 represents an integer of 1.

In some embodiments, ring J represents 5- to 15-membered heteroarylene, e.g., 5- to 12-membered heteroarylene, or 5- to 10-membered heteroarylene. In some embodiments, examples of ring J include, but are not limited to, benzothiazolyl, 2H-indazolyl and benzoxazolyl. Ring J is optionally substituted with p63 R¹⁶¹, with each R¹⁶¹ being the same or different and each independently representing halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., deuterated C₁₋₃ alkyl, such as CD₃), hydroxy substituted C₁₋₆ alkyl (e.g., hydroxy substituted C₁₋₄ alkyl, e.g., hydroxy substituted propyl, hydroxy substituted isopropyl), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₃ alkoxy, such as trifluoromethoxy), and p63 represents an integer of 0, 1, 2 or 3. In some embodiments, R¹⁶¹ represents hydroxy substituted propyl or hydroxy substituted isopropyl, and p63 represents an integer of 1.

In some embodiments, PBM represents the structure of the following Formula (PBM-56-1), Formula (PBM-56-2), Formula (PBM-56-3), Formula (PBM-56-4), Formula (PBM-56-5) or Formula (PBM-56-6):

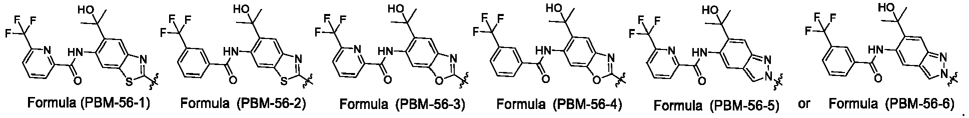

In some embodiments, PBM-R_{f}- represents a small molecule ligand targeting KRAS.

In some embodiments, PBM represents the structure of the following Formula (PBM-54): 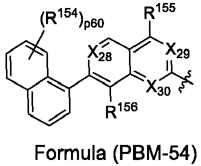 wherein
X₂₈, X₂₉ and X₃₀ in Formula (PBM-54) each independently represent N or CH;
(R¹⁵⁴)ₚ₆₀ indicates that the naphthyl in Formula (PBM-54) is optionally substituted with p60 R¹⁵⁴, with each R¹⁵⁴ independently representing halogen, hydroxy, C₂₋₆ alkynyl , C₂₋₆ alkenyl, C₁₋₆ alkoxy, C₁₋₆ alkyl or halogenated C₁₋₆ alkyl, and p60 represents an integer of 0, 1, 2, 3, 4, 5, 6or 7;
R¹⁵⁵ represents optionally substituted C₃₋₁₁ cycloalkyl or optionally substituted 4- to 15-membered heterocyclyl containing from 1 to 4 heteroatoms selected from the group consisting of oxygen, nitrogen, and sulfur; and
R¹⁵⁶ represents halogen, hydroxy, cyano, amino, mercapto, nitro, C₂₋₆ alkynyl, C₂₋₆ alkenyl, C₁₋₆ alkoxy, C₁₋₆ alkyl or halogenated C₁₋₆ alkyl.

In some embodiments, X₂₈, X₂₉ and X₃₀ in Formula (PBM-54) each independently represent N or CH. In some embodiments, one of X₂₈, X₂₉ and X₃₀ in Formula (PBM-54) represents N, the remaining each independently represent N or CH. In some embodiments, two of X₂₈, X₂₉ and X₃₀ in Formula (PBM-54) represent N, the remaining represents N or CH. In some embodiments, X₂₈, X₂₉ and X₃₀ in Formula (PBM-54) represent N. In some embodiments, X₂₈, X₂₉ and X₃₀ in Formula (PBM-54) represent CH.

In some embodiments, (R¹⁵⁴)ₚ₆₀ indicates that the naphthyl in Formula (PBM-54) is optionally substituted with p60 R¹⁵⁴, with each R¹⁵⁴ independently representing halogen (e.g., fluorine, chlorine, bromine, or iodine), hydroxy, amino, mercapto, nitro, cyano, C₂₋₆ alkynyl (e.g., ethynyl, propynyl, butynyl, pentynyl or hexynyl), C₂₋₆ alkenyl (e.g., vinyl, propenyl, butenyl, pentenyl or hexenyl), C₁₋₆ alkoxy (e.g., C₁₋₄ alkoxy, such as methoxy, CD₃-O-, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p60 represents an integer of 0, 1, 2, 3, 4, 5, 6 or 7. In some embodiments, p60 represents an integer of 2, 3 or 4, and each R¹⁵⁴ independently represents halogen (e.g., fluorine, chlorine, bromine, or iodine), hydroxy or C₂₋₆ alkynyl (e.g., ethynyl, propynyl, butynyl, pentynyl or hexynyl).

In some embodiments, R¹⁵⁵ represents optionally substituted C₃₋₁₁ cycloalkyl or optionally substituted 4- to 15-membered heterocyclyl containing from 1 to 4 heteroatoms independently selected from sulfur, oxygen, and nitrogen. In some embodiments, examples of optionally substituted C₃₋₁₁ cycloalkyl include, but are not limited to, e.g., optionally substituted C₃₋₁₀ cycloalkyl, or optionally substituted C₃₋₉ cycloalkyl, such as cyclopropyl, cyclobutyl, cyclopentyl, cyclohexyl, cycloheptyl, cyclooctyl, noradamantanyl, adamantanyl, bornyl, or norbornyl, wherein the C₃₋₁₁ cycloalkyl is optionally substituted with one or more (e.g., from 1 to 4, or from 2 to 3) substituents selected from the group consisting of e.g., deuterium, oxo, hydroxy, amino, mercapto, nitro, halogen (e.g., fluorine, chlorine, bromine, or iodine), cyano, optionally deuterated C₁₋₄ alkyl (e.g., optionally deuterated C₁₋₃ alkyl, such as methyl, CD₃, ethyl, propyl, isopropyl, butyl, sec-butyl, or tert-butyl), halogenated C₁₋₄ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), or any combination thereof. In some embodiments, examples of optionally substituted 4- to 15-membered heterocyclyl containing from 1 to 4 heteroatoms independently selected from sulfur, oxygen, and nitrogen include, but are not limited to, e.g., optionally substituted 4- to 12-membered heterocyclyl containing from 1 to 4 heteroatoms independently selected from sulfur, oxygen, and nitrogen, or optionally substituted 4- to 11-membered heterocyclyl containing from 1 to 4 heteroatoms independently selected from sulfur, oxygen, and nitrogen, e.g., azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl (e.g., 1,4-diazacycloheptan-1-yl), diazacyclooctyl, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, and 2,5-diazabicyclo[2.2.2]octan-2-yl, and azaspirocycloalkyl (e.g., 3-azaspiro[5.5]undecan-3-yl); and the 4- to 15-membered heterocyclyl is optionally substituted with one or more, e.g., 1-4 or 2-3, substituents selected from the group consisting of: deuterium, oxo, hydroxy, amino, mercapto, nitro, halogen (e.g., fluorine, chlorine, bromine, or iodine), cyano, optionally deuterated C₁₋₄ alkyl (e.g., optionally deuterated C₁₋₃ alkyl, such as methyl, CD₃, ethyl, propyl, isopropyl, butyl, sec-butyl, or tert-butyl), halogenated C₁₋₄ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), or any combination thereof.

In some embodiments, R¹⁵⁶ represents hydrogen, halogen (e.g., fluorine, chlorine, bromine, or iodine), hydroxy, C₂₋₆ alkynyl (e.g., ethynyl, propynyl, butynyl, pentynyl or hexynyl), C₂₋₆ alkenyl (e.g., vinyl, propenyl, butenyl, pentenyl or hexenyl), C₁₋₆ alkoxy (e.g., C₁₋₄ alkoxy, such as methoxy, CD₃-O-, ethoxy, propoxy, isopropoxy, butoxy, sec-butoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, PBM represents the structure of the following Formula (PBM-54-1):

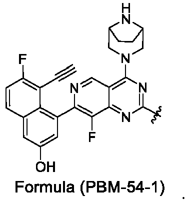

In some embodiments, PBM-R_{f}- represents a small molecule ligand targeting FGFR.

In some embodiments, each R⁷⁴ in Formula (PBM-28-1A), Formula (PBM-28-1B), Formula (PBM-28-1C), Formula (PBM-28-1D) and Formula (PBM-28-1D) independently represents a bond, i.e., R⁷⁴ is absent.

In some embodiments, each R⁷³ in Formula (PBM-28-1A), Formula (PBM-28-1B), Formula (PBM-28-1C), Formula (PBM-28-1D) and Formula (PBM-28-1D) independently represents optionally substituted C₁₋₆ alkyl. The substituents of the optionally substituted C₁₋₆ alkyl include, but are not limited to, e.g., deuterium, hydroxy, amino, mercapto, nitro, halogen (e.g., fluorine, chlorine, bromine, or iodine), cyano, optionally deuterated C₁₋₄ alkyl (e.g., optionally deuterated C₁₋₃ alkyl, such as methyl, CD₃, ethyl, propyl, isopropyl, butyl, sec-butyl, or tert-butyl), halogenated C₁₋₄ alkyl (e.g., halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), optionally substituted C₅₋₁₀ aryl (e.g., optionally substituted phenylor optionally substituted naphthyl), or any combination thereof. Examples of the optionally substituted C₁₋₆ alkyl include, but are not limited to, e.g., -optionally substituted C₁₋₆ alkylene-optionally substituted C₆₋₁₀ aryl. In some embodiments, Examples of the -optionally substituted C₁₋₆ alkylene-optionally substituted C₆₋₁₀ aryl include, but are not limited to, e.g., -optionally substituted C₁₋₆ alkylene-optionally substituted C₆₋₁₀ aryl, or -optionally substituted C₁₋₆ alkylene-optionally substituted phenyl, wherein the aryl or phenyl is optionally substituted with one or more (e.g., 1-4 or 2-3) substituents selected from the group consisting of e.g., deuterium, hydroxy, amino, mercapto, nitro, halogen (e.g., fluorine, chlorine, bromine, or iodine), cyano, optionally deuterated C₁₋₄ alkyl (e.g., optionally deuterated C₁₋₃ alkyl, such as methyl, CD₃, ethyl, propyl, isopropyl, butyl, sec-butyl, or tert-butyl), halogenated C₁₋₄ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), or any combination thereof; and the C₁₋₆ alkylene is optionally substituted with one or more (e.g., 1-4 or 2-3) substituents selected from the group consisting of e.g., deuterium, hydroxy, amino, mercapto, nitro, halogen (e.g., fluorine, chlorine, bromine, or iodine), cyano, optionally deuterated C₁₋₄ alkyl (e.g., optionally deuterated C₁₋₃ alkyl, such as methyl, CD₃, ethyl, propyl, isopropyl, butyl, sec-butyl, or tert-butyl), halogenated C₁₋₄ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), or any combination thereof.

In some embodiments, each R⁷³ in Formula (PBM-28-1A), Formula (PBM-28-1B), Formula (PBM-28-1C), Formula (PBM-28-1D) and Formula (PBM-28-1D) independently represents

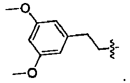

In some embodiments, PBM represents the structure of the following Formula (PBM-28-2):

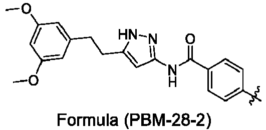

In some embodiments, PBM represents the structure of the following Formula (PBM-29): 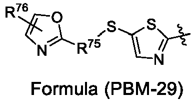 wherein
R⁷⁵ represents C₁₋₃ alkylene (e.g., methylene, ethylene or propylene) or halogenated C₁₋₃ alkylene (e.g., halogenated methylene, halogenated ethylene or halogenated propylene); and
R⁷⁶ represents C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or optionally substituted C₃₋₆ cycloalkyl.

In some embodiments, R⁷⁶ in Formula (PBM-29) represents optionally substituted C₃₋₆ cycloalkyl, e.g., optionally substituted cyclopropyl, optionally substituted cyclobutyl, optionally substituted cyclopentyl or optionally substituted cyclohexyl. In some embodiments, C₃₋₆ cycloalkyl is optionally substituted with one or more (e.g., 1-3, or 1, 2 or 3) substituents selected from the group consisting of: deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

In some embodiments, PBM represents the structure of the following Formula (PBM-29-1):

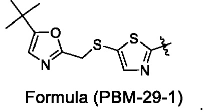

In some embodiments, PBM represents the structure of the following Formula (PBM-30-1A), Formula (PBM-30-1B), Formula (PBM-30-1C), Formula (PBM-30-1D) or Formula (PBM-30-1E): 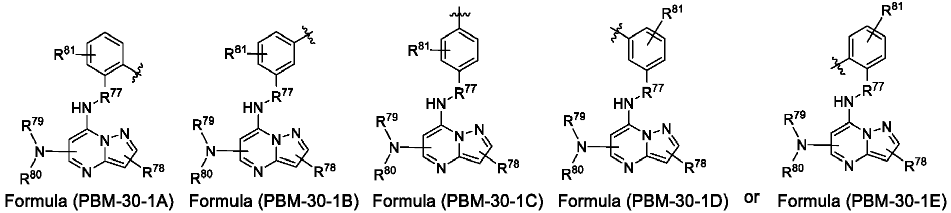 wherein
each R⁷⁷ in Formula (PBM-30-1A), Formula (PBM-30-1B), Formula (PBM-30-1C), Formula (PBM-30-1D) and Formula (PBM-30-1E) independently represents C₁₋₃ alkylene (e.g., methylene, ethylene or propylene) or halogenated C₁₋₃ alkylene (e.g., halogenated methylene, halogenated ethylene or halogenated propylene);
R⁷⁸ represents hydroxy, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
R⁷⁹ and R⁸⁰ are the same or different and each independently represent hydrogen, optionally substituted C₁₋₁₀ alkyl or optionally substituted C₃₋₆ cycloalkyl; and
R⁸¹ represents hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R⁷⁹ and R⁸⁰ in Formula (PBM-30-1A), Formula (PBM-30-1B), Formula (PBM-30-1C), Formula (PBM-30-1D) and Formula (PBM-30-1E) are the same or different and each independently represent hydrogen, optionally substituted C₁₋₁₀ alkyl or optionally substituted C₃₋₆ cycloalkyl. In some embodiments, examples of optionally substituted C₁₋₁₀ alkyl include, but are not limited to, optionally substituted C₁₋₈ alkyl,optionally substituted C₁₋₆ alkyl, or optionally substituted C₁₋₄ alkyl. In some embodiments, C₁₋₁₀ alkyl is optionally substituted with one or more (e.g., 1-3, or 1, 2 or 3) substituents selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof. In some embodiments, examples of optionally substituted C₃₋₆ cycloalkyl include, but are not limited to, e.g., optionally substituted cyclopropyl, optionally substituted cyclobutyl, optionally substituted cyclopentyl or optionally substituted cyclohexyl. In some embodiments, C₃₋₆ cycloalkyl is optionally substituted with one or more (e.g., 1-3, or 1, 2 or 3) substituents selected from the group consisting of: deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

In some embodiments, each R⁷⁹ in Formula (PBM-30-1A), Formula (PBM-30-1B), Formula (PBM-30-1C), Formula (PBM-30-1D) and Formula (PBM-30-1E) represents hydrogen, and R⁸⁰ represents

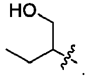

In some embodiments, PBM represents the structure of the following Formula (PBM-30-1):

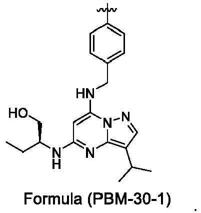

In some embodiments, PBM represents the structure of the following Formula (PBM-31): 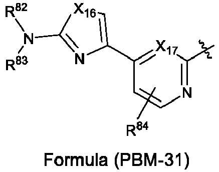 wherein
X₁₆ in Formula (PBM-31) represents -S- or a fragment of 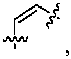 wherein when X₁₆ represents -S-, heteroaryl containing X₁₆ and nitrogen atom in Formula (PBM-31) is thiazolyl; and when X₁₆ represents fragment of 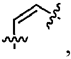 the heteroaryl containing X₁₆ and nitrogen atom in Formula (PBM-31) is pyridyl;
X₁₇ represents N or CH;
R⁸² represents hydrogen or optionally substituted C₁₋₁₀ alkyl;
R⁸³ represents hydrogen, -C₁₋₃ alkylene-optionally substituted 4- to 10-membered heterocyclyl, or optionally substituted 4- to 10-membered heterocyclyl; and
R⁸⁴ represents hydroxy, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, X₁₆ in Formula (PBM-31) represents -S-, and heteroaryl containing X₁₆ and nitrogen atom in Formula (PBM-31) is thiazolyl.

In some embodiments, X₁₆ in Formula (PBM-31) represents fragment 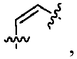 heteroaryl containing X₁₆ and nitrogen atom in Formula (PBM-31) is pyridyl.

In some embodiments, R⁸² in Formula (PBM-31) represents hydrogen or optionally substituted C₁₋₁₀ alkyl. In some embodiments, examples of optionally substituted C₁₋₁₀ alkyl include, but are not limited to, optionally substituted C₁₋₈ alkyl,optionally substituted C₁₋₆ alkyl, or optionally substituted C₁₋₄ alkyl. In some embodiments, C₁₋₁₀ alkyl is optionally substituted with one or more (e.g., 1-3, or 1, 2 or 3) substituents selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

In some embodiments, R⁸³ in Formula (PBM-31) represents hydrogen, -C₁₋₃ alkylene-optionally substituted 4- to 10-membered heterocyclyl, or optionally substituted 4- to 10-membered heterocyclyl. In some embodiments, each optionally substituted 4- to 10-membered heterocyclyl is independently e.g., 4- to 10-membered (e.g., 4- to 9-membered, 4- to 8-membered, 5- to 7-membered, or 4- to 6-membered) monocyclic or bicyclic heterocyclyl containing one or more (e.g., from 1 to 4, or 1, 2, 3 or 4) heteroatoms independently selected from sulfur, oxygen, and nitrogen, including but not limited to azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo [2.2.1]heptanyl, azabicyclo [3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the heterocyclyl is optionally substituted with a substituent selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

In some embodiments, PBM represents the structure of the following Formula (PBM-31-1A) or Formula (PBM-31-1B): 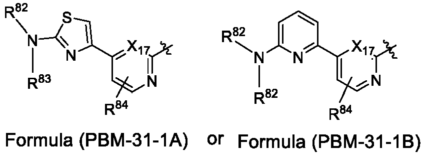 wherein
X₁₇ represents N or CH;
R⁸² represents hydrogen or optionally substituted C₁₋₁₀ alkyl;
R⁸³ represents hydrogen, -C₁₋₃ alkylene-optionally substituted 4- to 10-membered heterocyclyl, or optionally substituted 4- to 10-membered heterocyclyl; and
R⁸⁴ represents hydroxy, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, each R⁸² in Formula (PBM-31-1A) and Formula (PBM-31-1B) each independently represents hydrogen or optionally substituted C₁₋₁₀ alkyl. In some embodiments, examples of optionally substituted C₁₋₁₀ alkyl include, but are not limited to, optionally substituted C₁₋₈ alkyl,optionally substituted C₁₋₆ alkyl, or optionally substituted C₁₋₄ alkyl. In some embodiments, C₁₋₁₀ alkyl is optionally substituted with one or more (e.g., 1-3, or 1, 2 or 3) substituents selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

In some embodiments, each R⁸³ in Formula (PBM-31-1A) and Formula (PBM-31-1B) independently represents hydrogen, -C₁₋₃ alkylene-optionally substituted 4- to 10-membered heterocyclyl, or optionally substituted 4- to 10-membered heterocyclyl. In some embodiments, each optionally substituted 4- to 10-membered heterocyclyl is independently e.g., 4- to 10-membered (e.g., 4- to 9-membered, 4- to 8-membered, 5- to 7-membered, or 4- to 6-membered) monocyclic or bicyclic heterocyclyl containing one or more (e.g., from 1 to 4, or 1, 2, 3 or 4) heteroatoms independently selected from sulfur, oxygen, and nitrogen, including but not limited to azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the heterocyclyl is optionally substituted with a substituent selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

In some embodiments, PBM represents the structure of the following Formula (PBM-31-1):

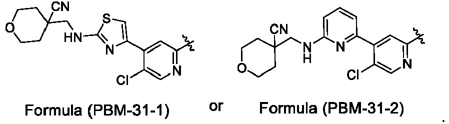

In some embodiments, PBM represents the structure of the following Formula (PBM-32): 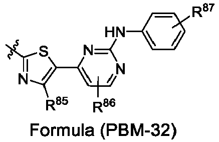 wherein
R⁸⁵ and R⁸⁶ in Formula (PBM-32) are the same or different and each independently represent hydroxy, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₅ alkoxy, C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
R⁸⁷ represents -S(O)₂NH₂ or -C(O)NH₂.

In some embodiments, R⁸⁷ in Formula (PBM-32) represents -S(O)₂NH₂.

In some embodiments, R⁸⁷ in Formula (PBM-32) represents -C(O)NH₂.

In some embodiments, PBM represents the structure of the following Formula (PBM-32-1) or Formula (PBM-32-2):

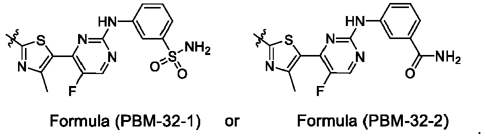

In some embodiments, PBM-R_{f}- represents a small molecule ligand targeting EZH2.

In some embodiments, PBM represents the structure of the following Formula (PBM-33): 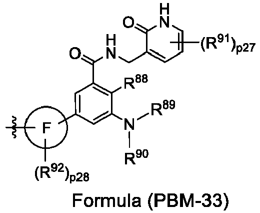 wherein
R⁸⁸ represents hydrogen, hydroxy, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
R⁸⁹ represents C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); or
R⁸⁸ and R⁸⁹ together with the corresponding carbon and nitrogen atoms to which they are connected form an optionally substituted 4- to 6-membered heteroaromatic ring or optionally substituted 4- to 6-membered heterocycle;
R⁹⁰ represents optionally substituted C₃₋₆ cycloalkyl (e.g., optionally substituted cyclopropyl, optionally substituted cyclobutyl, optionally substituted cyclopentyl or optionally substituted cyclohexyl), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or optionally substituted 4- to 10-membered heterocyclyl;
(R⁹¹)ₚ₂₇ incicates that pyridin-2(1H)-one ring in Formula (PBM-33) is substituted with p27 R⁹¹, with each R⁹¹ being the same or different and each independently representing hydroxy, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p27 represents an integer of 1, 2 or 3;
ring F in Formula (PBM-33) represents arylene or 5- to 20-membered monocyclic or bicyclic heteroarylene containing from 1 to 4 heteroatoms selected from the group consisting of oxygen, nitrogen, and sulfur; and (R⁹²)ₚ₂₈ incicates that the ring F is optionally substituted with p28 R⁹², with each R⁹² being the same or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-); and
p28 represents an integer of 0, 1, 2, 3 or 4.

In some embodiments, R⁸⁸ in Formula (PBM-33) represents hydrogen, hydroxy, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R⁸⁹ in Formula (PBM-33) represents C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R⁸⁸ and R⁸⁹ in Formula (PBM-33) together with the corresponding carbon and nitrogen atoms to which they are connected form an optionally substituted 4- to 6-membered nitrogen-containing heteroaromatic ring or optionally substituted 4- to 6-membered nitrogen-containing heterocycle. In some embodiments, R⁸⁸ and R⁸⁹ in Formula (PBM-33) together represent the following fragment: -CH₂-, -CH=CH-, -CH=N-, -N=CH-, -N=N-, -CH₂-CH₂-, -NH-CH₂-, or -CH₂-CH₂-CH₂-. In some embodiments, 4- to 6-membered nitrogen-containing heterocycle includes, but is not limited to, e.g., 4- to 6-membered (e.g., 4-membered, 5-membered, 6-membered) heterocycle containing one nitrogen atom and optional heteroatom selected from the group consisting of oxygen, nitrogen, and sulfur. The 4- to 6-membered nitrogen-containing heterocycle fused with the benzene ring in Formula (PBM-33) to form a fused ring. The 4- to 6-membered nitrogen-containing heterocycle is optionally substituted with one or more (e.g., from 1 to 4, from 1 to 3, or 2) substituents selected from the group consisting of: halogen (e.g., fluorine, chlorine, bromine, or iodine), hydroxy, amino, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.). In some embodiments, R⁹⁰ in Formula (PBM-33) represents optionally substituted C₃₋₆ cycloalkyl (e.g., optionally substituted cyclopropyl, optionally substituted cyclobutyl, optionally substituted cyclopentyl or optionally substituted cyclohexyl), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or optionally substituted 4- to 10-membered heterocyclyl.

In some embodiments, R⁹⁰ in Formula (PBM-33) represents optionally substituted C₃₋₆ cycloalkyl, e.g., optionally substituted cyclopropyl, optionally substituted cyclobutyl, optionally substituted cyclopentyl or optionally substituted cyclohexyl. In some embodiments, C₃₋₆ cycloalkyl is optionally substituted with one or more (e.g., 1-3, or 1, 2 or 3) substituents selected from the group consisting of: deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

In some embodiments, R⁹⁰ in Formula (PBM-33) represents optionally substituted 4- to 10-membered heterocyclyl. The optionally substituted 4- to 10-membered heterocyclyl is e.g., optionally substituted 4- to 10-membered heterocyclyl. In some embodiments, each optionally substituted 4- to 10-membered heterocyclyl is independently e.g., 4- to 10-membered (e.g., 4- to 9-membered, 4- to 8-membered, 5- to 7-membered, or 4- to 6-membered) monocyclic or bicyclic heterocyclyl containing one or more (e.g., from 1 to 4, or 1, 2, 3 or 4) heteroatoms independently selected from sulfur, oxygen, and nitrogen, including but not limited to the following optionally substituted groups: azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo [2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 10-membered heterocyclyl is optionally substituted with a substituent selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

In some embodiments, ring F in Formula (PBM-33) represents arylene (e.g., C₅₋₂₀ arylene, C₅₋₁₅ arylene, C₅₋₁₀ arylene, or C₅₋₆ arylene) or 5- to 20-membered (e.g., 5- to 15-membered, 5- to 10-membered , 5- to 9-membered, 5- to 8-membered, 5- to 7-membered, or 6-membered) monocyclic or bicyclic heteroarylene containing 1-4 (e.g., 1 to 3 , or 1 to 2) heteroatoms selected from the group consisting of N, O and S. Examples of arylene include, but are not limited to, phenylene, naphthylene. Examples of heteroarylene include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-fluoro[2,3-b]pyrrolylene, pyrrolo[2,1-b]thiazolylene, and imidazo[2,1-b]thiazolylene. In some embodiments, the ring F is optionally substituted with p28 R⁹², with each R⁹² being the same or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-); and p28 represents an integer of 0, 1, 2, 3 or 4.

In some embodiments, PBM represents the structure of the following Formula (PBM-33-1A), Formula (PBM-33-1B), Formula (PBM-33-1C), Formula (PBM-33-1D), Formula (PBM-33-1E), Formula (PBM-33-1F), Formula (PBM-33-1G), Formula (PBM-33-1H), Formula (PBM-33-1I), Formula (PBM-33-1J), or Formula (PBM-33-1K): 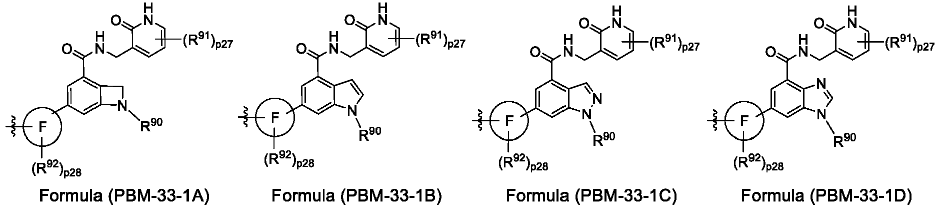 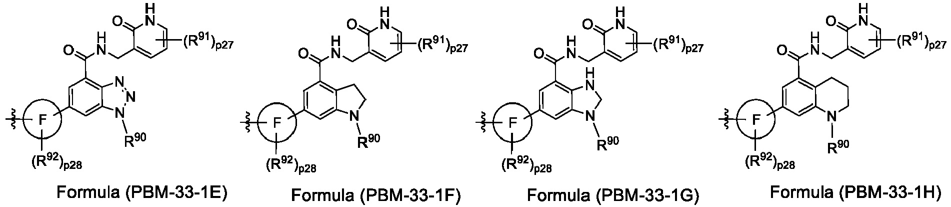 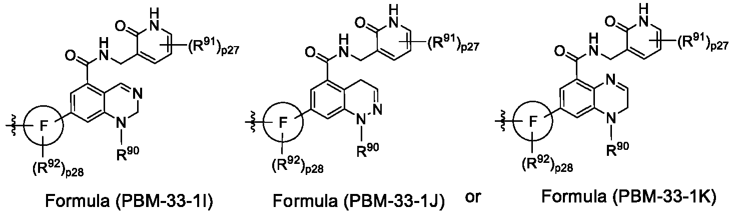 wherein
R⁹⁰ in each formula independently represents optionally substituted C₃₋₆ cycloalkyl (e.g., optionally substituted cyclopropyl, optionally substituted cyclobutyl, optionally substituted cyclopentyl or optionally substituted cyclohexyl), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or optionally substituted 4- to 10-membered heterocyclyl;
(R⁹¹)ₚ₂₇ in each formula independently indicates that the pyridin-2(1H)-one ring in each formula is substituted with p27 R⁹¹, with each R⁹¹ being the same or different and each independently representing hydroxy, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p27 in each formula independently represents an integer of 1, 2 or 3;
ring F in each formula independently represents arylene or 5- to 20-membered monocyclic or bicyclic heteroarylene containing from 1 to 4 heteroatoms selected from the group consisting of oxygen, nitrogen, and sulfur; and the (R⁹²)ₚ₂₈ indicates that the ring F is optionally substituted with p28 R⁹², with each R⁹² being the same or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-); and
p28 in each formula each independently represents an integer of 0, 1, 2, 3 or 4.

In some embodiments, each R⁹⁰ in Formula (PBM-33-1A), Formula (PBM-33-1B), Formula (PBM-33-1C), Formula (PBM-33-1D), Formula (PBM-33-1E), Formula (PBM-33-1F), Formula (PBM-33-1G), Formula (PBM-33-1H), Formula (PBM-33-1I), Formula (PBM-33-1J), and Formula (PBM-33-1K) independently represents optionally substituted C₃₋₆ cycloalkyl, e.g., optionally substituted cyclopropyl, optionally substituted cyclobutyl, optionally substituted cyclopentyl or optionally substituted cyclohexyl. In some embodiments, C₃₋₆ cycloalkyl is optionally substituted with one or more (e.g., 1-3, or 1, 2 or 3) substituents selected from the group consisting of: deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

In some embodiments, each R⁹⁰ in Formula (PBM-33-1A), Formula (PBM-33-1B), Formula (PBM-33-1C), Formula (PBM-33-1D), Formula (PBM-33-1E), Formula (PBM-33-1F), Formula (PBM-33-1G), Formula (PBM-33-1H), Formula (PBM-33-1I), Formula (PBM-33-1J), and Formula (PBM-33-1K) independently represents optionally substituted 4- to 10-membered heterocyclyl. The optionally substituted 4- to 10-membered heterocyclyl is e.g., optionally substituted 4- to 10-membered (e.g., 4- to 9-membered, 4- to 8-membered, 5- to 7-membered, or 4- to 6-membered) monocyclic or bicyclic heterocyclyl containing one or more (e.g., from 1 to 4, or 1, 2, 3 or 4) heteroatoms independently selected from sulfur, oxygen, and nitrogen, including but not limited to the following optionally substituted groups: azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 10-membered heterocyclyl is optionally substituted with a substituent selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

In some embodiments, each ring F in Formula (PBM-33-1A), Formula (PBM-33-1B), Formula (PBM-33-1C), Formula (PBM-33-1D), Formula (PBM-33-1E), Formula (PBM-33-1F), Formula (PBM-33-1G), Formula (PBM-33-1H), Formula (PBM-33-1I), Formula (PBM-33-1J), and Formula (PBM-33-1K) independently represents arylene (e.g., C₅₋₂₀ arylene, C₅₋₁₅ arylene, C₅₋₁₀ arylene, or C₅₋₆ arylene) or 5- to 20-membered (e.g., 5- to 15-membered, 5- to 10-membered , 5- to 9-membered, 5- to 8-membered, 5- to 7-membered, or 6-membered) monocyclic or bicyclic heteroarylene containing from 1 to 4 (e.g., 1 to 3 , or 1 to 2) heteroatoms selected from the group consisting of oxygen, nitrogen, and sulfur. Examples of arylene include, but are not limited to, phenylene, naphthylene. Examples of heteroarylene include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-fluoro[2,3-b]pyrrolylene, pyrrolo[2,1-b]thiazolylene, and imidazo[2,1-b]thiazolylene. In some embodiments, the ring F is optionally substituted with p28 R⁹², with each R⁹² being the same or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-); and p28 represents an integer of 0, 1, 2, 3 or 4.

In some embodiments, PBM represents the structure of the following Formula (PBM-33-1), Formula (PBM-33-2), or Formula (PBM-33-3):

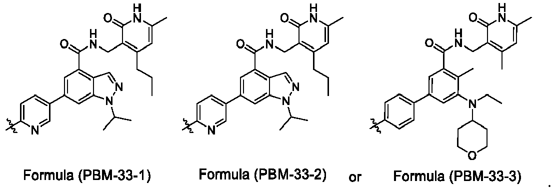

In some embodiments, PBM-R_{f}- represents a small molecule ligand targeting NTRK.

In some embodiments, PBM represents the structure of the following Formula (PBM-34-1A), Formula (PBM-34-1B), Formula (PBM-34-1C), Formula (PBM-34-1D), or Formula (PBM-34-1E): 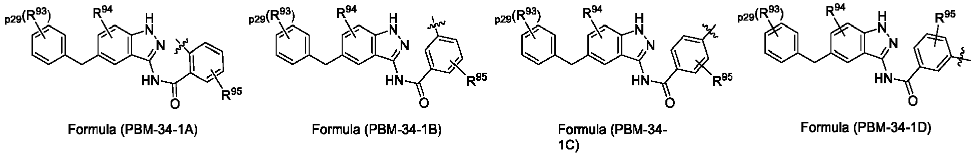 or 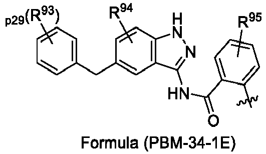 wherein
(R⁹³)ₚ₂₉ in each formula independently indicates that benzene rings in each formula is substituted with p29 R⁹³, with each R⁹³ being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p29 represents an integer of 1, 2, 3, 4 or 5;
R⁹⁴ in each formula independently represents hydrogen, hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
R⁹⁵ in each formula independently represents NR^{b15}R^{b16}, where R^{b15} and R^{b16} are the same or different and each independently represent hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally substituted C₃₋₆ cycloalkyl or optionally substituted 4- to 8-membered heterocyclyl.

In some embodiments, each R^{b15} in Formula (PBM-34-1A), Formula (PBM-34-1B), Formula (PBM-34-1C), Formula (PBM-34-1D), and Formula (PBM-34-1E) independently represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), optionally substituted C₃₋₆ cycloalkyl or optionally substituted 4- to 8-membered heterocyclyl. In some embodiments, examples of optionally substituted C₃₋₆ cycloalkyl include, but are not limited to, e.g., optionally substituted cyclopropyl, optionally substituted cyclobutyl, optionally substituted cyclopentyl or optionally substituted cyclohexyl. In some embodiments, C₃₋₆ cycloalkyl is optionally substituted with one or more (e.g., 1-3, or 1, 2 or 3) substituents selected from the group consisting of: deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof. In some embodiments, the optionally substituted 4- to 8-membered heterocyclyl is e.g., optionally substituted 4- to 8-membered (e.g., 4- to 7-membered, 4- to 6-membered, 5- to 7-membered, or 5- to 6-membered) monocyclic or bicyclic heterocyclyl group containing one or more (e.g., 1-4, or 1, 2, 3, or 4) heteroatoms selected from nitrogen, oxygen, and sulfur, including but not limited to the following optionally substituted groups: azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, and diazacyclooctyl. In some embodiments, the 4- to 8-membered heterocyclyl is optionally substituted with a substituent selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

In some embodiments, each R^{b16} in Formula (PBM-34-1A), Formula (PBM-34-1B), Formula (PBM-34-1C), Formula (PBM-34-1D), and Formula (PBM-34-1E) independently represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), optionally substituted C₃₋₆ cycloalkyl or optionally substituted 4- to 8-membered heterocyclyl. In some embodiments, examples of optionally substituted C₃₋₆ cycloalkyl include, but are not limited to, e.g., optionally substituted cyclopropyl, optionally substituted cyclobutyl, optionally substituted cyclopentyl or optionally substituted cyclohexyl. In some embodiments, C₃₋₆ cycloalkyl is optionally substituted with one or more (e.g., 1-3, or 1, 2 or 3) substituents selected from the group consisting of: deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof. In some embodiments, the optionally substituted 4- to 8-membered heterocyclyl is e.g., optionally substituted 4- to 8-membered (e.g., 4- to 7-membered, 4- to 6-membered, 5- to 7-membered, or 5- to 6-membered) monocyclic or bicyclic heterocyclyl group containing one or more (e.g., 1-4, or 1, 2, 3, or 4) heteroatoms selected from nitrogen, oxygen, and sulfur, including but not limited to the following optionally substituted groups: azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 8-membered heterocyclyl is optionally substituted with a substituent selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

In some embodiments, PBM represents the structure of the following Formula (PBM-34-1):

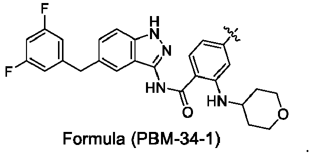

In some embodiments, PBM represents the structure of the following Formula (PBM-35): 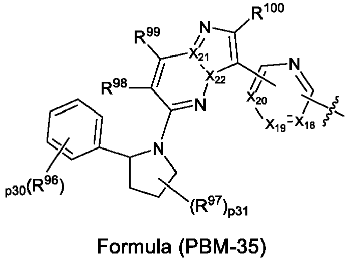 wherein
X₁₈, X₁₉ and X₂₀ in Formula (PBM-35) are the same or different and each independently represent CH or N, wherein X₁₈, X₁₉ and X₂₀ are not simultaneously N, and the 6-membered ring containing X₁₈, X₁₉, X₂₀ and nitrogen atom is optionally substituted with 1 or 2 substituents selected from the group consisting of halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or any combination thereof;
X₂₁ and X₂₂ in Formula (PBM-35) are the same or different and each independently represent Cor N, wherein X₂₁ and X₂₂ are not simultaneously N;
(R⁹⁶)ₚ₃₀ indicates that the benzene ring in Formula (PBM-35) is substituted with p30 R⁹⁶, with each R⁹⁶ being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p30 represents an integer of 1, 2, 3, 4 or 5;
(R⁹⁷)ₚ₃₁ indicates that the pyrrolidine ring in Formula (PBM-35) is optionally substituted with p31 R⁹⁷, with each R⁹⁷ being the same or different and each independently representing hydrogen, hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p31 represents an integer of 0, 1, 2, 3, 4, 5 or 6; and
R⁹⁸, R⁹⁹, R¹⁰⁰ are the same or different and each independently represent hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, X₁₈, X₁₉ and X₂₀ in Formula (PBM-35) represents CH.

In some embodiments, X₁₈ in Formula (PBM-35) represents N, and X₁₉ and X₂₀ represents CH.

In some embodiments, X₁₉ in Formula (PBM-35) represents N, and X₁₈ and X₂₀ represents CH.

In some embodiments, X₂₀ in Formula (PBM-35) represents N, and X₁₈ and X₁₉ represents CH.

In some embodiments, X₂₀ in Formula (PBM-35) represents CH, and X₁₈ and X₂₀ represents N.

In some embodiments, X₂₁ in Formula (PBM-35) represents C, and X₂₂ represents N.

In some embodiments, X₂₁ in Formula (PBM-35) represents N, and X₂₂ represents C.

In some embodiments, PBM represents the structure of the following Formula (PBM-35-1A), Formula (PBM-35-1B), Formula (PBM-35-1C), Formula (PBM-35-1D), Formula (PBM-35-1E), Formula (PBM-35-1F), Formula (PBM-35-1G), Formula (PBM-35-1H), Formula (PBM-35-1I), or Formula (PBM-35-1J): 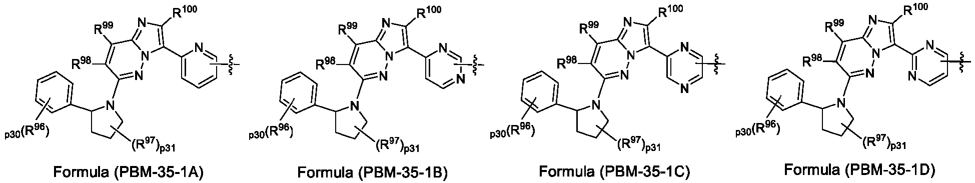 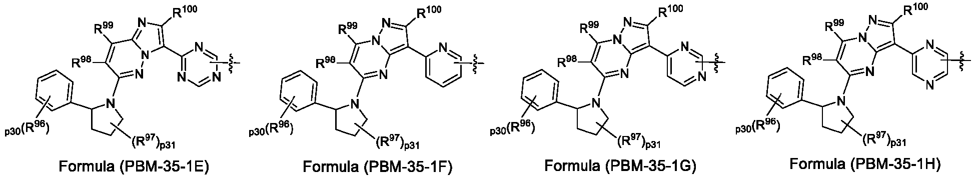 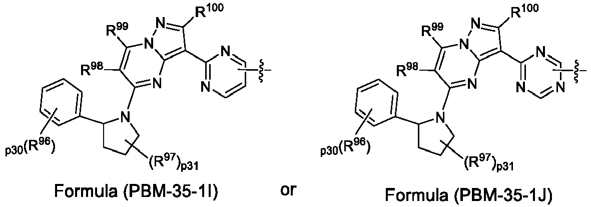 wherein
(R⁹⁶)ₚ₃₀ in each formula independently indicates that benzene ring is substituted with p30 R⁹⁶, with each R⁹⁶ being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p30 represents an integer of 1, 2, 3, 4 or 5;
(R⁹⁷)ₚ₃₁ in each formula independently indicates that the pyrrolidine ring in Formula (PBM-35) is optionally substituted with p31 R⁹⁷, with each R⁹⁷ being the same or different and each independently representing hydrogen, hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p31 represents an integer of 0, 1, 2, 3, 4, 5 or 6; and
R⁹⁸, R⁹⁹, R¹⁰⁰ in each formula each independently are the same or different and each independently represent hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, PBM represents the structure of the following Formula (PBM-35-1), Formula (PBM-35-2), Formula (PBM-35-3), Formula (PBM-35-4), Formula (PBM-35-5), or Formula (PBM-35-6):

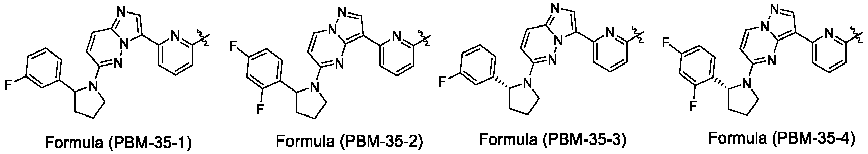

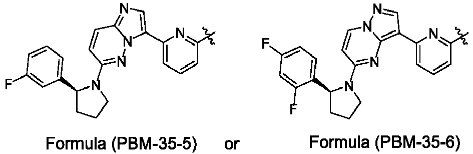

In some embodiments, PBM-R_{f}- represents a small molecule ligand targeting SHP2.

In some embodiments, PBM represents the structure of the following Formula (PBM-36): 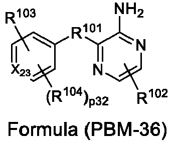 wherein
X₂₃ in Formula (PBM-36) represents CH or N;
R¹⁰¹ represents a bond or -S-;
R¹⁰² represents a bond or optionally substituted 4- to 15-membered nitrogen-containing heterocyclyl;
R¹⁰³ represents a bond, halogen (e.g., fluorine, chlorine, bromine, or iodine), amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or optionally substituted 4- to 15-membered nitrogen-containing heterocyclyl;
wherein R¹⁰² and R¹⁰³ are not simultaneously a bond, and only one of R¹⁰² and R¹⁰³ is a bond, wherein when R¹⁰² represents a bond, the carbon atom on the ring connected to R¹⁰² is directly connected to R_{f}, and when R¹⁰³ represents a bond, the carbon atom on the ring connected to R¹⁰³ is directly connected to R_{f} ;
(R¹⁰⁴)ₚ₃₂ indicates that the 6-membered ring in Formula (PBM-36) is optionally substituted with p32 R¹⁰⁴, with each R¹⁰⁴ being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p32 represents an integer of 0, 1, 2, 3 or 4.

In some embodiments, R¹⁰² in Formula (PBM-36) represents a bond, and R¹⁰³ represents halogen (e.g., fluorine, chlorine, bromine, or iodine), amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or optionally substituted 4- to 15-membered nitrogen-containing heterocyclyl. In some embodiments, optionally substituted 4- to 15-membered nitrogen-containing heterocyclyl is e.g., optionally substituted 4- to 15-membered (e.g., 4- to 10-membered, 4- to 9-membered, 4- to 8-membered, 5- to 7-membered, or 4- to 6-membered) monocyclic or bicyclic heterocyclyl containing one nitrogen atom and optionally one or more (e.g., from 1 to 3, or 1, 2, or 3) heteroatoms independently selected from sulfur, oxygen, and nitrogen, including but not limited to the following optionally substituted groups: azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo [2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 15-membered nitrogen-containing heterocyclyl is optionally substituted with a substituent selected from the group consisting of deuterium, hydroxy, amino, mercapto, nitro, halogen (e.g., fluorine, chlorine, bromine, or iodine), cyano, optionally deuterated C₁₋₆ alkyl (e.g., optionally perdeuterated C₁₋₆ alkyl, optionally perdeuterated C₁₋₅ alkyl or optionally perdeuterated C₁₋₄ alkyl, such as CD₃, CD₃CD₂-, CD₃CD₂CD₂- etc.), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), optionally deuterated C₁₋₆ alkyl-NH-(e.g., optionally deuterated C₁₋₃ alkyl-NH-, such as CH₃NH-, CH₃CH₂NH- or CH₃CH₂CH₂NH-), NH_{Z}-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH-(e.g., optionally deuterated C₁₋₄ alkyl-C(O)NH-, such as CH₃-C(O)NH-, CH₃CH₂-C(O)NH-, and CH₃CH₂CH₂-C(O)NH-), or any combination thereof.

In some embodiments, R¹⁰³ in Formula (PBM-36) represents a bond, and R¹⁰² represents optionally substituted 4- to 15-membered nitrogen-containing heterocyclyl. In some embodiments, the optionally substituted 4- to 15-membered nitrogen-containing heterocyclyl is e.g., optionally substituted 4- to 15-membered (e.g., 4- to 10-membered, 4- to 9-membered, 4- to 8-membered, 5- to 7-membered, or 4- to 6-membered) monocyclic or bicyclic heterocyclyl containing one nitrogen atom and optionally one or more (e.g., from 1 to 3, or 1, 2, or 3) heteroatoms independently selected from sulfur, oxygen, and nitrogen, including but not limited to the following optionally substituted groups: azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 15-membered nitrogen-containing heterocyclyl is optionally substituted with a substituent selected from the group consisting of deuterium, hydroxy, amino, mercapto, nitro, halogen (e.g., fluorine, chlorine, bromine, or iodine), cyano, optionally deuterated C₁₋₆ alkyl (e.g., optionally perdeuterated C₁₋₆ alkyl, optionally perdeuterated C₁₋₅ alkyl or optionally perdeuterated C₁₋₄ alkyl, such as CD₃, CD₃CD₂-, CD₃CD₂CD₂- etc.), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), optionally deuterated C₁₋₆ alkyl-NH-(e.g., optionally deuterated C₁₋₃ alkyl-NH-, such as CH₃NH-, CH₃CH₂NH- or CH₃CH₂CH₂NH-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH- (e.g., optionally deuterated C₁₋₄ alkyl-C(O)NH-, such as CH₃-C(O)NH-, CH₃CH₂-C(O)NH-, and CH₃CH₂CH₂-C(O)NH-), or any combination thereof.

In some embodiments, R¹⁰¹ in Formula (PBM-36) represents a bond.

In some embodiments, R¹⁰¹ in Formula (PBM-36) represents -S-.

In some embodiments, X₂₃ in Formula (PBM-36) represents CH.

In some embodiments, X₂₃ in Formula (PBM-36) represents N.

In some embodiments, PBM represents the structure of the following Formula (PBM-36-1A), Formula (PBM-36-1B), Formula (PBM-36-1C), Formula (PBM-36-1D), Formula (PBM-36-1E), Formula (PBM-36-1F), Formula (PBM-36-1G), or Formula (PBM-36-1H): 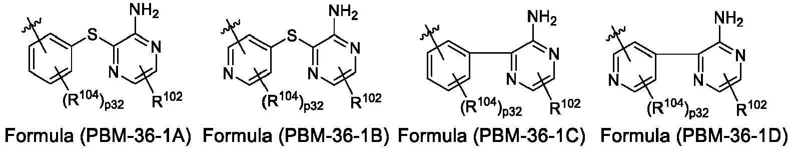 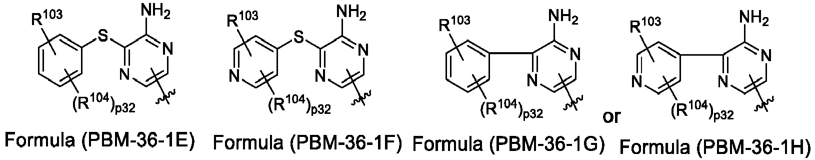 wherein
R¹⁰² in each formula each independently represents optionally substituted 4- to 15-membered nitrogen-containing heterocyclyl;
R¹⁰³ in each formula each independently represents halogen (e.g., fluorine, chlorine, bromine, or iodine), amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or optionally substituted 4- to 15-membered nitrogen-containing heterocyclyl;
(R¹⁰⁴)ₚ₃₂ in each formula each independently indicates that the 6-membered ring in each formula is optionally substituted with p32 R¹⁰⁴, with each R¹⁰⁴ being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p32 represents an integer of 0, 1, 2, 3 or 4.

In some embodiments, each R¹⁰³ in Formula (PBM-36-1E), Formula (PBM-36-1F), Formula (PBM-36-1G), and Formula (PBM-36-1H) each independently represents halogen (e.g., fluorine, chlorine, bromine, or iodine), amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or optionally substituted 4- to 15-membered nitrogen-containing heterocyclyl. In some embodiments, the optionally substituted 4- to 15-membered nitrogen-containing heterocyclyl is e.g., optionally substituted 4- to 15-membered (e.g., 4- to 10-membered, 4- to 9-membered, 4- to 8-membered, 5- to 7-membered, or 4- to 6-membered) monocyclic or bicyclic heterocyclyl containing one nitrogen atom and optionally one or more (e.g., from 1 to 3, or 1, 2, or 3) heteroatoms independently selected from sulfur, oxygen, and nitrogen, including but not limited to the following optionally substituted groups: azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo [2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 15-membered nitrogen-containing heterocyclyl is optionally substituted with a substituent selected from the group consisting of deuterium, hydroxy, amino, mercapto, nitro, halogen (e.g., fluorine, chlorine, bromine, or iodine), cyano, optionally deuterated C₁₋₆ alkyl (e.g., optionally perdeuterated C₁₋₆ alkyl, optionally perdeuterated C₁₋₅ alkyl or optionally perdeuterated C₁₋₄ alkyl, such as CD₃, CD₃CD₂-, CD₃CD₂CD₂- etc.), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), optionally deuterated C₁₋₆ alkyl-NH-(e.g., optionally deuterated C₁₋₃ alkyl-NH-, such as CH₃NH-, CH₃CH₂NH- or CH₃CH₂CH₂NH-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH-(e.g., optionally deuterated C₁₋₄ alkyl-C(O)NH-, such as CH₃-C(O)NH-, CH₃CH₂-C(O)NH-, and CH₃CH₂CH₂-C(O)NH-), or any combination thereof.

In some embodiments, each R¹⁰² in Formula (PBM-36-1A), Formula (PBM-36-1B), Formula (PBM-36-1C), and Formula (PBM-36-1D) independently represents optionally substituted 4- to 15-membered nitrogen-containing heterocyclyl. In some embodiments, optionally substituted 4- to 15-membered nitrogen-containing heterocyclyl is e.g., optionally substituted 4- to 15-membered (e.g., 4-to 10-membered, 4- to 9-membered, 4- to 8-membered, 5- to 7-membered, or 4- to 6-membered) monocyclic or bicyclic heterocyclyl containing one nitrogen atom and optionally one or more (e.g., from 1 to 3, or 1, 2, or 3) heteroatoms independently selected from sulfur, oxygen, and nitrogen, including but not limited to the following optionally substituted groups: azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 15-membered nitrogen-containing heterocyclyl is optionally substituted with a substituent selected from the group consisting of deuterium, hydroxy, amino, mercapto, nitro, halogen (e.g., fluorine, chlorine, bromine, or iodine), cyano, optionally deuterated C₁₋₆ alkyl (e.g., optionally perdeuterated C₁₋₆ alkyl, optionally perdeuterated C₁₋₅ alkyl or optionally perdeuterated C₁₋₄ alkyl, such as CD₃, CD₃CD₂-, CD₃CD₂CD₂-etc.), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), optionally deuterated C₁₋₆ alkyl-NH-(e.g., optionally deuterated C₁₋₃ alkyl-NH-, such as CH₃NH-, CH₃CH₂NH- or CH₃CH₂CH₂NH-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH-(e.g., optionally deuterated C₁₋₄ alkyl-C(O)NH-, such as CH₃-C(O)NH-, CH₃CH₂-C(O)NH-, and CH₃CH₂CH₂-C(O)NH-), or any combination thereof.

In some embodiments, PBM represents the structure of the following Formula (PBM-36-1), Formula (PBM-36-2), Formula (PBM-36-3), Formula (PBM-36-4), Formula (PBM-36-5), Formula (PBM-36-6), Formula (PBM-36-7), Formula (PBM-36-8) or Formula (PBM-36-9): 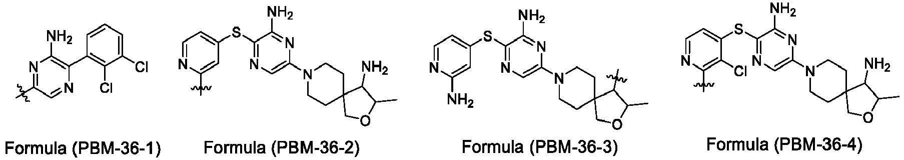 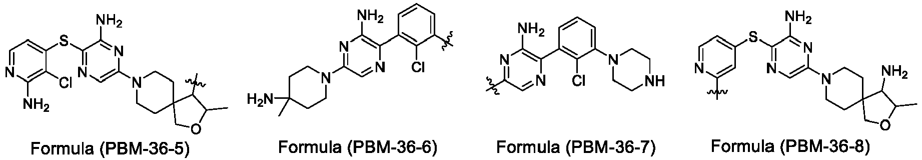 or

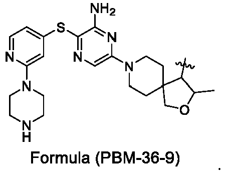

In some embodiments, PBM represents the structure of the following Formula (PBM-9-1C): 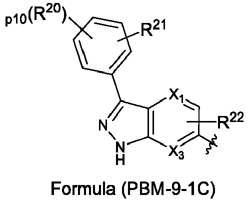 wherein
X₁ in Formula (PBM-9) represents N or CR^{b7}, wherein R^{b7} is hydrogen or amino, and X₃ represents CH or N;
(R²⁰)ₚ₁₀ indicates that the benzene ring in Formula (PBM-9-1C) is substituted with p10 R²⁰, with each R²⁰ being the same or different and each independently representing -O-aryl, -O-heteroaryl, -C₁₋₃ alkylene-NHC(O)-heteroaryl, -C₁₋₃ alkylene-C(O)NH-heteroaryl or halogen (e.g., fluorine, chlorine, bromine, or iodine), wherein the aryl and heteroaryl are each independently optionally substituted with one or more (e.g., 1-5, such as 1, 2, 3, 4 or 5) R^{b8}, with each R^{b8} being the same or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
p10 represents an integer of 1, 2, 3 or 4;
R²¹ represents deuterium, hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy); and
R²² represents hydrogen, halogen (e.g., fluorine, chlorine, bromine, or iodine), cyano, or amino.

In some embodiments, R²⁰ in Formula (PBM-9-1C) represents -O-aryl, -O-heteroaryl, -C₁₋₃ alkylene-NHC(O)-heteroaryl, or -C₁₋₃ alkylene-C(O)NH-heteroaryl, wherein aryl can be optionally substituted C₆₋₁₀ aryl, including but not limited to e.g., phenyl or naphthyl, and the heteroaryl can be optionally substituted 5- to 14-membered monocyclic or bicyclic aromatic ring group containing one or more (e.g., from 1 to 6, or from 1 to 5, or from 1 to 4, or from 1 to 3) heteroatoms independently selected from the group consisting of oxygen, nitrogen, and sulfur. In a sub-embodiment, R²⁰ represents -O-aryl or -O-naphthyl, wherein the phenyl or naphthyl is optionally substituted with one or more (e.g., 1-5, such as 1, 2, 3, 4 or 5) R^{b8}, with each R^{b8} being the same or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In a sub-embodiment, R²⁰ represents -O-heteroaryl, -C₁₋₃ alkylene-NHC(O)-heteroaryl, or -C₁₋₃ alkylene-C(O)NH-heteroaryl, wherein the heteroaryl is optionally substituted with one or more (e.g., 1-5, such as 1, 2, 3, 4 or 5) R^{b8}, with each R^{b8} being the same or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂-etc.), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In a sub-embodiment, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, or imidazo[2,1-b]thiazolyl.

In some embodiments, PBM represents the structure of the following Formula (PBM-9-4) or Formula (PBM-9-5):

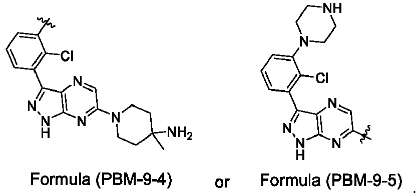

In some embodiments, PBM-R_{f}- represents a small molecule ligand targeting PARP.

In some embodiments, PBM represents the structure of the following Formula (PBM-37-1A), Formula (PBM-37-1B), Formula (PBM-37-1C), Formula (PBM-37-1D), or Formula (PBM-37-1E): 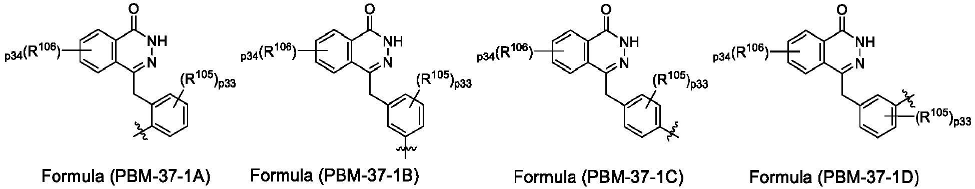 or 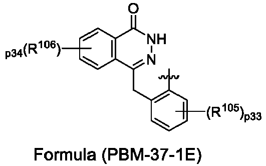 wherein
(R¹⁰⁵)ₚ₃₃ in each formula independently indicates that the benzene ring in each formula is optionally substituted with p33 R¹⁰⁵, with each R¹⁰⁵ being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p33 represents an integer of 1, 2, 3 or 4;
(R¹⁰⁶)ₚ₃₄ in each formula independently indicates that the phthalazinone ring in each formula is optionally substituted with p34 R¹⁰⁶, with each R¹⁰⁶ being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p34 represents an integer of 0, 1, 2, 3 or 4.

In some embodiments, PBM represents the structure of the following Formula (PBM-37-1):

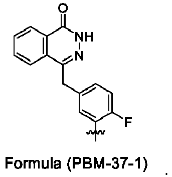

In some embodiments, PBM represents the structure of the following Formula (PBM-38-1A), Formula (PBM-38-1B), Formula (PBM-38-1C), Formula (PBM-38-1D), or Formula (PBM-38-1E): 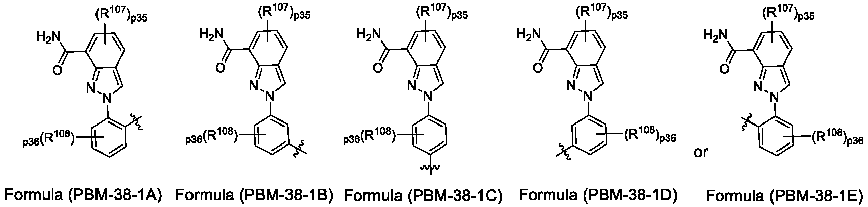 wherein
(R¹⁰⁷)ₚ₃₅ in each formula independently indicates that the 2H-indazole ring in each formula is optionally substituted with p35 R¹⁰⁷, with each R¹⁰⁷ being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p35 represents an integer of 0, 1, 2 or 3;
(R¹⁰⁸)ₚ₃₆ in each formula independently indicates that the benzene ring in each formula is optionally substituted with p36 R¹⁰⁸, with each R¹⁰⁸ being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p36 represents an integer of 0, 1, 2 or 3.

In some embodiments, PBM represents the structure of the following Formula (PBM-38-1):

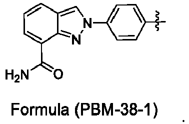

In some embodiments, PBM represents the structure of the following Formula (PBM-39-1A), Formula (PBM-39-1B), Formula (PBM-39-1C), Formula (PBM-39-1D), or Formula (PBM-39-1E): 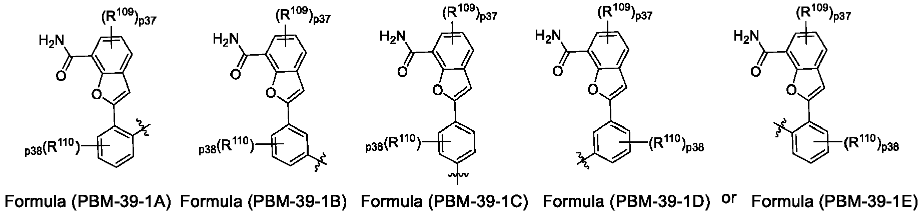 wherein
(R¹⁰⁹)_{p37 6} in each formula independently indicates that the 2H-indazole ring in each formula is optionally substituted with p37 R¹⁰⁹, with each R¹⁰⁹ being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p37 represents an integer of 0, 1, 2 or 3;
(R¹¹⁰)ₚ₃₈ independently indicates that the benzene ring in each formula is optionally substituted with p38 R¹¹⁰, with each R¹¹⁰ being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p38 represents an integer of 0, 1, 2 or 3.

In some embodiments, PBM represents the structure of the following Formula (PBM-39-1):

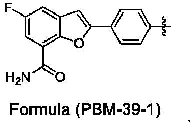

In some embodiments, PBM represents the structure of the following Formula (PBM-40-1A), Formula (PBM-40-1B), Formula (PBM-40-1C), Formula (PBM-40-1D), or Formula (PBM-40-1E): 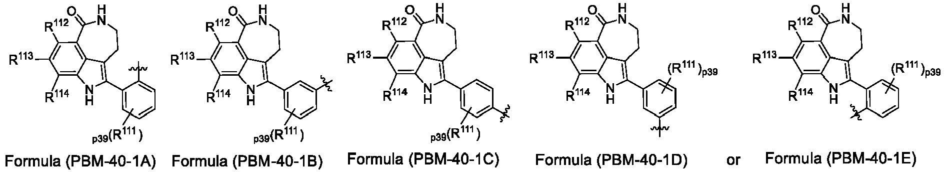 wherein
(R¹¹¹)ₚ₃₉ in each formula independently indicates that the benzene ring in each formula is optionally substituted with p39 R¹¹¹, with each R¹¹¹ being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p39 represents an integer of 0, 1, 2, 3 or 4; and
R¹¹², R¹¹³, R¹¹⁴ are the same or different and each independently represent hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-or CH₂ClCH₂-).

In some embodiments, PBM represents the structure of the following Formula (PBM-40-1):

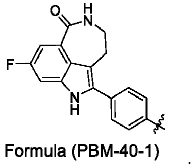

In some embodiments, PBM represents the structure of the following Formula (PBM-41-1A), Formula (PBM-41-1B), Formula (PBM-41-1C), Formula (PBM-41-1D), or Formula (PBM-41-1E): 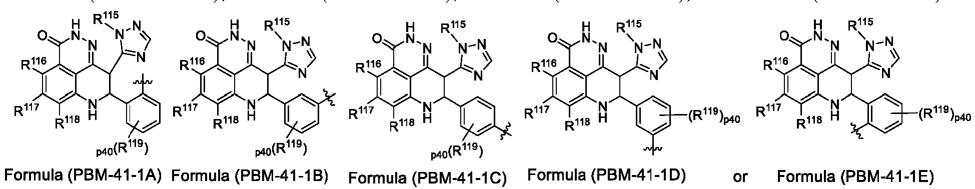 wherein
R¹¹⁵ represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂-etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
R¹¹⁶, R¹¹⁷, R¹¹⁸ are the same or different and each independently represent hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-or CH₂ClCH₂-);
(R¹¹⁹)ₚ₄₀ in each formula independently indicates that benzene in each formula is optionally substituted with p40 R¹¹⁹, with each R¹¹⁹ being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p40 represents an integer of 0, 1, 2, 3 or 4.

In some embodiments, PBM represents the structure of the following Formula (PBM-41-1):

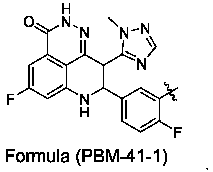

In some embodiments, PBM-R_{f}- represents a small molecule ligand targeting STATS.

In some embodiments, PBM represents the structure of the following Formula (PBM-42): 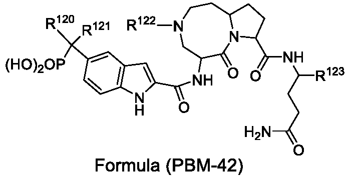 wherein
R¹²⁰, R¹²¹ are the same or different and each independently represent hydrogen, halogen (e.g., fluorine, chlorine, bromine, or iodine), C₁₋₃ alkyl (e.g., methyl, ethyl, or propyl), or halogenated C₁₋₃ alkyl (e.g., halogenated C₁₋₂ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
R¹²² represents a bond, hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
R¹²³ represents the structure of the following formula: 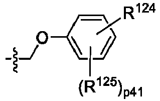
wherein R¹²⁴ represents a bond or hydrogen; and (R¹²⁵)ₚ₄₁ in dicates that the benzene ring is optionally substituted with p41 R¹²⁵, with each R¹²⁵ being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p41 represents an integer of 0, 1, 2, 3 or 4; or
R¹²³ represents the structure of the following formula: 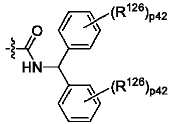
wherein (R¹²⁶)ₚ₄₂ indicates that the benzene rings are optionally substituted with p42 R¹²⁶, with each R¹²⁶ being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p42 represents an integer of 0, 1, 2, 3, 4 or 5; and
wherein R¹²² and R¹²⁴ are not simultaneously a bond, and only one of R¹²² and R¹²⁴ represents a bond, wherein when R¹²² represents a bond, the nitrogen atom on the ring connected to R¹²² is directly connected to R_{f} , and when R¹²⁴ represents a bond, the carbon atom on the ring connected to R¹²⁴ is directly connected to R_{f} .

In some embodiments, R¹²² in Formula (PBM-42) represents a bond. In this case, the nitrogen atom on the ring connected to R¹²² is directly connected to R_{f}. R¹²³ represents the structure of the following formula: 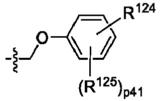 wherein R¹²⁴ represents hydrogen, and (R¹²⁵)ₚ₄₁ indicates that the benzene ring is optionally substituted with p41 R¹²⁵, with each R¹²⁵ being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p41 represents an integer of 0, 1, 2, 3 or 4.

In some embodiments, R¹²² in Formula (PBM-42) represents a bond. In this case, the nitrogen atom on the ring connected to R¹²² is directly connected to R_{f}. R¹²³ represents the structure of the following formula: 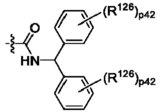 wherein (R¹²⁶)ₚ₄₂ indicates that the benzene ring is optionally substituted with p42 R¹²⁶, with each R¹²⁶ being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p42 represents an integer of 0, 1, 2, 3, 4 or 5.

In some embodiments, when R¹²³ represents the structure of the following formula: 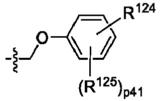 wherein R¹²⁴ represents a bond (i.e., the carbon atom on the ring connected to R¹²⁴ is directly connected to R_{f}), and (R¹²⁵)ₚ₄₁ is as defined above, R¹²² represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, PBM represents the structure of the following Formula (PBM-42-1A): 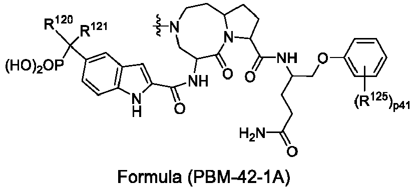 wherein
R¹²⁰ and R¹²¹ are the same or different and each independently represent hydrogen, halogen (e.g., fluorine, chlorine, bromine, or iodine), C₁₋₃ alkyl (e.g., methyl, ethyl, or propyl), or halogenated C₁₋₃ alkyl (e.g., halogenated C₁₋₂ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
(R¹²⁵)ₚ₄₁ indicates that the benzene ring is optionally substituted with p41 R¹²⁵, with each R¹²⁵ being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p41 represents an integer of 0, 1, 2, 3 or 4.

In some embodiments, PBM represents the structure of the following Formula (PBM-42-1B): 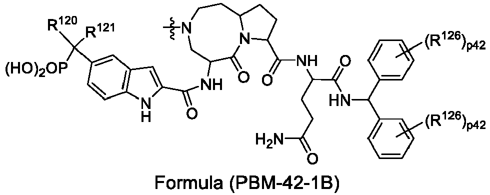 wherein
R¹²⁰ and R¹²¹ are the same or different and each independently represent hydrogen, halogen (e.g., fluorine, chlorine, bromine, or iodine), C₁₋₃ alkyl (e.g., methyl, ethyl, or propyl), or halogenated C₁₋₃ alkyl (e.g., halogenated C₁₋₂ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
(R¹²⁶)ₚ₄₂ indicates that the benzene ring is optionally substituted with p42 R¹²⁶, with each R¹²⁶ being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p42 represents an integer of 0, 1, 2, 3, 4 or 5.

In some embodiments, PBM represents the structure of the following Formula (PBM-42-1C), Formula (PBM-42-1D), Formula (PBM-42-1E), Formula (PBM-42-1F), or Formula (PBM-42-1G): 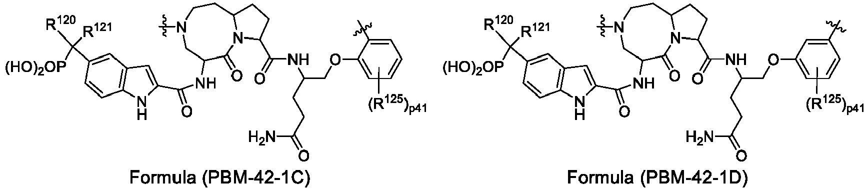 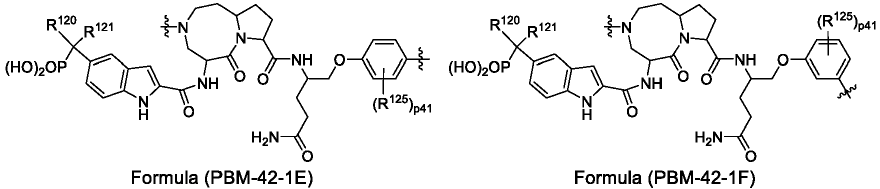 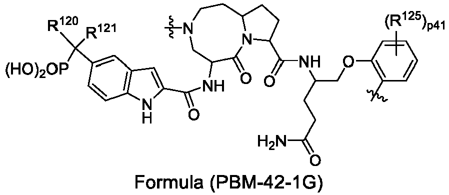 wherein
R¹²⁰ and R¹²¹ are the same or different and each independently represent hydrogen, halogen (e.g., fluorine, chlorine, bromine, or iodine), C₁₋₃ alkyl (e.g., methyl, ethyl, or propyl), or halogenated C₁₋₃ alkyl (e.g., halogenated C₁₋₂ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
(R¹²⁵)ₚ₄₁ indicates that the benzene ring is optionally substituted with p41 R¹²⁵, with each R¹²⁵ being the same or different and each independently representing t hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p41 represents an integer of 0, 1, 2, 3 or 4.

In some embodiments, PBM represents the structure of the following Formula (PBM-42-1) or Formula (PBM-42-2):

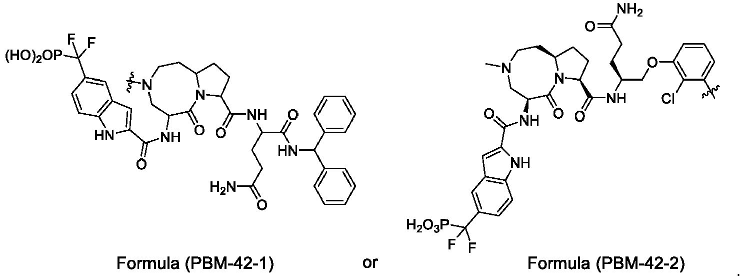

In some embodiments, PBM represents the structure of the following Formula (PBM-43-1A) or Formula (PBM-43-1B): 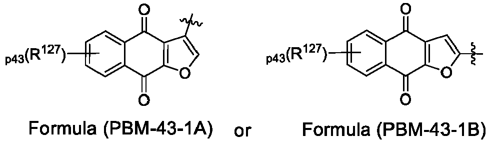 wherein
(R¹²⁷)ₚ₄₃ in each formula independently indicates that the benzene ring in each formula is optionally substituted with p43 R¹²⁷, with each R¹²⁷ being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₅ alkoxy, C₁₋₄ alkoxy or C₁₋₃ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p43 represents an integer of 0, 1, 2, 3 or 4.

In some embodiments, PBM represents the structure of the following Formula (PBM-43-1):

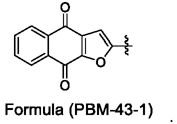

In some embodiments, PBM-R_{f}- represents a small molecule ligand targeting FLT3.

In some embodiments, PBM represents the structure of the following Formula (PBM-45-1A), Formula (PBM-45-1B), Formula (PBM-45-1C), Formula (PBM-45-1D), or Formula (PBM-45-1E): 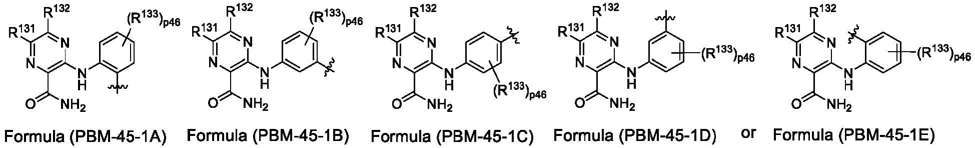 wherein
R¹³¹ represents C₁₋₆ alkyl (e.g., C₁₋₄ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or R¹³¹ represents hydrogen;
R¹³² represents C₁₋₆ alkyl (e.g., C₁₋₄ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally substituted C₃₋₆ cycloalkyl (e.g., optionally substituted cyclopropyl, optionally substituted cyclobutyl, optionally substituted cyclopentyl or optionally substituted cyclohexyl), optionally substituted 4- to 8-membered nitrogen-containing heterocyclyl or NR^{b11}R^{b12}, where R^{b11} and R^{b12} are the same or different and each independently represent hydrogen, C₁₋₆ alkyl (e.g., C₁₋₄ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), or optionally substituted 4- to 8-membered heterocyclyl;
(R¹³³)ₚ₄₆ indicates that the benzene ring in Formula (PBM-45-1A), Formula (PBM-45-1B), Formula (PBM-45-1C), Formula (PBM-45-1D), or Formula (PBM-45-1E) are optionally substituted with p46 R¹³³, with each R¹³³ being the same or different and each independently representing hydroxy, amino, cyano, halogen (e.g., fluorine, chlorine, bromine, or iodine), C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p46 represents an integer of 0, 1, 2, 3 or 4.

In some embodiments, R¹³¹ represents ethyl.

In some embodiments, R¹³¹ represents hydrogen.

In some embodiments, R¹³² represents NR^{b11}R^{b12}, where R^{b11} and R^{b12} are the same or different and each independently represent hydrogen, C₁₋₆ alkyl (e.g., C₁₋₄ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), or optionally substituted 4- to 8-membered heterocyclyl. In some embodiments, optionally substituted 4- to 8-membered heterocyclyl is e.g., optionally substituted 4- to 8-membered (e.g., 4- to 7-membered, 4- to 6-membered, 5- to 7-membered, or 5- to 6-membered) monocyclic or bicyclic heterocyclyl group containing one or more (e.g., 1-4, or 1, 2, 3, or 4) heteroatoms selected from nitrogen, oxygen, and sulfur, including but not limited to the following optionally substituted groups: azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 8-membered heterocyclyl is optionally substituted with a substituent selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof. In some embodiments, R¹³² represents NR^{b11}R^{b12}, where R^{b11} represents hydrogen, and R^{b12} represents optionally substituted tetrahydropyranyl, and the tetrahydropyranyl is optionally substituted with a substituent selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

In some embodiments, R¹³² represents optionally substituted C₃₋₆ cycloalkyl, such as optionally substituted cyclopropyl, optionally substituted cyclobutyl, optionally substituted cyclopentyl or optionally substituted cyclohexyl. In some embodiments, C₃₋₆ cycloalkyl is optionally substituted with one or more (e.g., 1-3, or 1, 2 or 3) substituents selected from the group consisting of: deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

In some embodiments, R¹³² represents optionally substituted 4- to 8-membered heterocyclyl. In some embodiments, optionally substituted 4- to 8-membered heterocyclyl is e.g., optionally substituted 4- to 8-membered (e.g., 4- to 7-membered, 4- to 6-membered, 5- to 7-membered, or 5- to 6-membered) monocyclic or bicyclic heterocyclyl group containing one or more (e.g., 1-4, or 1, 2, 3, or 4) heteroatoms selected from nitrogen, oxygen, and sulfur, including but not limited to the following optionally substituted groups: azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1. l]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 8-membered heterocyclyl is optionally substituted with a substituent selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, 3-methyl-2-oxoimidazolidin-1-yl or any combination thereof. In some embodiments, R¹³² represents piperidinyl, which is optionally substituted with 3-methyl-2-oxoimidazolidin-1-yl.

In some embodiments, p46 represents an integer of 0 . In some embodiments, p46 represents an integer of 1, and R¹³³ represents methoxy.

In some embodiments, PBM represents the structure of the following Formula (PBM-45-1):

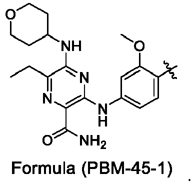

In some embodiments, PBM-R_{f}- represents a small molecule ligand targeting BCL -2.

In some embodiments, PBM represents the structure of the following Formula (PBM-47-1) or Formula (PBM-47-2):

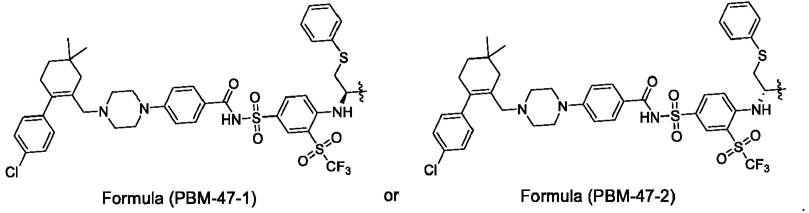

In some embodiments, PBM-R_{f}- represents a small molecule ligand targeting ALK2.

In some embodiments, PBM represents the structure of the following Formula (PBM-48): 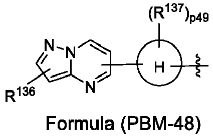
ring H in Formula (PBM-48) represents C₆₋₁₀ arylene or C₅₋₁₀ heteroarylene, and (R¹³⁷)ₚ₄₉ indicates that the ring H is optionally substituted with p49 R¹³⁷, with each R¹³⁷ being the same or different and each independently representing deuterium, halogen (e.g., fluorine, chlorine, bromine, or iodine), hydroxy, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), C₁₋₆ alkoxy (e.g., C₁₋₅ alkoxy or C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
p49 represents an integer of 0, 1, 2, 3 or 4; and
R¹³⁶ represents optionally substituted 5- to 20-membered monocyclic or bicyclic heteroaryl containing from 1 to 4 heteroatoms selected from the group consisting of oxygen, nitrogen, and sulfur, which is optionally substituted with a substituent selected from the group consisting of hydroxy, amino, cyano, halogen (e.g., fluorine, chlorine, bromine, or iodine), C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, ring H in Formula (PBM-48) represents phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, imidazo[2,1-b]thiazolylene and imidazo[1,2-b]pyridazinylene. In some embodiments, ring H is optionally substituted with p49 R¹³⁷, wherein p49 represents an integer of 0, 1, 2, 3 or 4, and each R¹³⁷ is the same or different and each independently represents deuterium, halogen (e.g., fluorine, chlorine, bromine, or iodine), hydroxy, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂-etc.), C₁₋₆ alkoxy (e.g., C₁₋₅ alkoxy or C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, ring H in Formula (PBM-48) represents phenylene, and the phenylene is optionally substituted with p49 R¹³⁷, wherein p49 represents an integer of 0, 1, 2, 3 or 4, and each R¹³⁷ is the same or different and each independently represents deuterium, halogen (e.g., fluorine, chlorine, bromine, or iodine), hydroxy, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), C₁₋₆ alkoxy (e.g., C₁₋₅ alkoxy or C₁₋₄ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, R¹³⁶ in Formula (PBM-48) represents optionally substituted 5- to 20-membered monocyclic or bicyclic heteroaryl containing from 1 to 4 heteroatoms selected from the group consisting of N, O and S. In some embodiments, R¹³⁶ represents furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, or imidazo[1,2-b]pyridazinyl. In some embodiments, R¹³⁶ is optionally substituted with one or more (e.g., 1-6, such as 1, 2, 3, 4, 5 or 6) substituents selected from the group consisting of hydroxy, amino, cyano, halogen (e.g., fluorine, chlorine, bromine, or iodine), C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R¹³⁶ in Formula (PBM-48) represents quinolinyl, which is optionally substituted with one or more (e.g., 1-6, such as 1, 2, 3, 4, 5 or 6) substituents selected from the group consisting of hydroxy, amino, cyano, halogen (e.g., fluorine, chlorine, bromine, or iodine), C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, butoxy, sec-butoxy, or tert-butoxy), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, PBM represents the structure of the following Formula (PBM-48-1) or Formula (PBM-48-2):

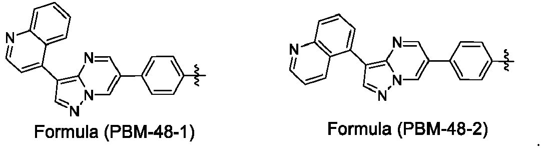

In some embodiments, PBM-R_{f}- represents a small molecule ligand targeting SCSI.

In some embodiments, PBM represents the structure of the following Formula (PBM-50): 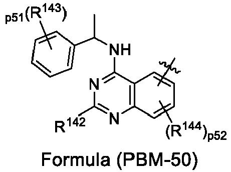 wherein
(R¹⁴³)ₚ₅₁ indicates that the benzene ring in Formula (PBM-50) is optionally substituted with p51 R¹⁴³, with each R¹⁴³ independently representing halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₅ alkoxy, C₁₋₄ alkoxy or C₁₋₃ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as trifluoromethoxy);
p51 represents an integer of 0, 1, 2, 3, 4 or 5;
R¹⁴² represents H, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
(R¹⁴⁴)ₚ₅₂ indicates that the quinazoline ring in Formula (PBM-50) is optionally substituted with p52 R¹⁴⁴, with each R¹⁴⁴ independently representing halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₅ alkoxy, C₁₋₄ alkoxy or C₁₋₃ alkoxy, such as methoxy, ethoxy, propoxy, isopropoxy, butoxy, sec-butoxy, or tert-butoxy), or halogenated C₁₋₆ alkoxy (e.g., halogenated C₁₋₄ alkoxy, such as trifluoromethoxy); and
p52 represents an integer of 1, 2, or 3.

In some embodiments, p51 represents an integer of 0, 1, 2, 3, 4 or 5. In some sub-embodiments, p51 represents an integer of 2.

In some embodiments, p52 represents an integer of 1, 2, or 3. In some sub-embodiments, p52 represents an integer of 1.

In some embodiments, PBM represents the structure of the following Formula (PBM-50-1) or Formula (PBM-50-2):

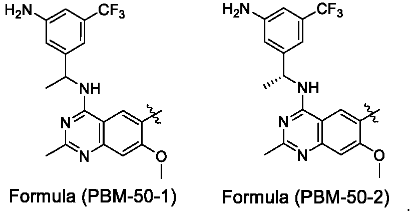

In some embodiments, R_{f} of Formula (II) represents a bond, -O-, -NHC(O)-*, -C(O)NH-*, or -NH-.

In some embodiments, R_{f} of Formula (II) represents the structure of the following formula:
-N(R_{g})-, -NHC(O)-C₁₋₆ alkylene-W⁵-*, -NHC(O)-C₂₋₆ alkenylene -W⁵-*, -C(O)NH-C₁₋₆ alkylene-W⁵-*, -C(O)NH-C₂₋₆ alkenylene -W⁵-*, -NHC(O)-W⁵-*, -C(O)NH-W⁵-*, -O-C₁₋₆ alkylene-W⁵-*, -O-C₂₋₆ alkenylene -W⁵-*, -O-W⁵-*, -O-C₁₋₆ alkylene-N(Rᵢ)-*, -O-C₂₋₆ alkenylene -N(Rᵢ)-*, -N(R_{g})-C₁₋₆ alkylene-N(Rᵢ)-*, -N(R_{g})-C₂₋₆ alkenylene -N(Rᵢ)-*, -W⁵-, -W⁵-N(R_{g})-*, -N(R_{g})-W⁵-*, -N(R_{g})-W⁵-N(Rᵢ)-*, -C₁₋₆ alkylene-W⁵-*, -C₂₋₆ alkenylene -W⁵-*, -C₁₋₆ alkylene-C(O)-W⁵-*, -C₂₋₆ alkenylene - C(O)-W⁵-*, -C(O)-W⁵-*, -C₁₋₆ alkylene-C(O)NH-C₁₋₆ alkylene-W⁵-*, -C₁₋₆ alkylene-C(O)NH-C₂₋₆ alkenylene -W⁵-*, -C₂₋₆ alkenylene -C(O)NH-C₁₋₆ alkylene-W⁵-*, -C₂₋₆ alkenylene -C(O)NH-C₂₋₆ alkenylene -W⁵-*, -C₁₋₆ alkylene-NHC(O)-C₁₋₆ alkylene-W⁵-*, -C₁₋₆ alkylene-NHC(O)-C₂₋₆ alkenylene -W⁵-*, -C₂₋₆ alkenylene -NHC(O)-C₁₋₆ alkylene-W⁵-*, -C₂₋₆ alkenylene -NHC(O)-C₂₋₆ alkenylene -W⁵-*, -C₁₋₆ alkylene-C(O)NH-*, -C₂₋₆ alkenylene -C(O)NH-*, -C₁₋₆ alkylene-NHC(O)-*, -C₂₋₆ alkenylene -NHC(O)-*, -C₁₋₆ alkylene-, -C₂₋₆ alkenylene -, -C₁₋₆ alkylene-N(Rᵢ)-*, -C₂₋₆ alkenylene -N(Rᵢ)-*, or 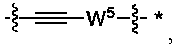
wherein R_{g} and Rᵢ each independently represents hydrogen or C₁₋₆ alkyl, and the C₁₋₆ alkylene and the C₂₋₆ alkenylene are optionally substituted with one or more substituents selected from the group consisting of halogen, hydroxy, mercapto, cyano, amino, nitro, C₁₋₆ alkyl, halogenated C₁₋₃ alkyl, C₁₋₃ alkoxy, halogenated C₁₋₃ alkoxy or any combination thereof;
wherein the group W⁵ represents the structure of the following formula: 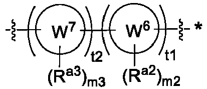
   wherein each ring W⁶ is the same or different and each independently represents nitrogen-containing heterocyclylene, tl represents an integer of 1 or 2, and (R^{a2})ₘ₂ indicates that each ring W6 is optionally substituted with m2 R^{a2}, with each R^{a2} independently representing deuterium, optionally deuterated C₁₋₆ alkyl, optionally halogenated C₁₋₆ alkyl, C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{12a}-R^{11a}-, where R^{11a} is optionally substituted C₁₋₆ alkylene or optionally substituted C₂₋₆ alkenylene, and R^{12a} represents halogen, R^{13a}R^{14a}N-, hydroxy, carboxyl, ethynyl, or vinyl, wherein R^{13a} and R^{14a} are the same or different and each independently represent hydrogen or C₁₋₃ alkyl, and the C₁₋₆ alkylene and C₂₋₆ alkenylene are optionally substituted with one or more substituents selected from the group consisting of halogen, hydroxy, mercapto, cyano, amino, nitro, C₁₋₆ alkyl, halogenated C₁₋₃ alkyl, C₁₋₃ alkoxy, halogenated C₁₋₃ alkoxy or any combination thereof, and m2 represents an integer of 0-20;
   each ring W⁷ is the same or different and each independently represents nitrogen-containing heterocyclylene, arylene, or heteroarylene, t2 represents an integer of 0 or 1, and (R^{a3})ₘ₃ indicates that each ring W⁷ is independently optionally substituted with m3 R^{a3}, with each R^{a3} independently representing deuterium, optionally deuterated C₁₋₆ alkyl, C₁₋₆ alkoxy, halogen, amino, hydroxy, mercapto, cyano, carboxyl, C₂₋₆ alkynyl , C₂₋₆ alkenyl, optionally deuterated C₃₋₆ cycloalkyl, or R^{16a}-R^{15a}-, where R^{15a} is optionally substituted C₁₋₆ alkylene or optionally substituted C₂₋₆ alkenylene, and R^{16a} represents halogen, R^{17a}R^{18a}N-, hydroxy, carboxyl, ethynyl, or vinyl, wherein R^{17a} and R^{18a} are the same or different and each independently represent hydrogen or C₁₋₃ alkyl, and m3 represents an integer of 0-20; and
   symbol * indicates the point of attachment to LIN.

In some embodiments, R_{g} represents hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), and the C₁₋₆ alkylene and C₂₋₆ alkenylene are optionally substituted with one or more substituents selected from the group consisting of halogen, hydroxy, mercapto, cyano, amino, nitro, C₁₋₆ alkyl, halogenated C₁₋₃ alkyl, C₁₋₃ alkoxy, halogenated C₁₋₃ alkoxy or any combination thereof;

In some embodiments, Rᵢ represents hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl).

In some embodiments, tl represents an integer of 1.

In some embodiments, tl represents an integer of 2.

In some embodiments, each ring W⁶ is the same or different and each independently represents 4- to 20-membered (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) nitrogen-containing heterocyclylene containing e.g., one nitrogen atom and optionally heteroatoms selected from the group consisting of oxygen, nitrogen, and sulfur. Examples of nitrogen-containing heterocyclylene include, but are not limited to, e.g., piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, 3-azaspiro[5.5]undecanylene, 8-azaspiro[4.5]decanylene, 2-oxa-8-azaspiro[4.5]decanylene or 3-methyl-2-oxa-8-azaspiro[4.5]decanylene. Each ring W⁶ is independently optionally substituted with m2 R^{a2}, with each R^{a2} being independently deuterium, optionally deuterated C₁₋₆ alkyl (e.g., optionally deuterated C₁₋₄ alkyl, such as methyl, CD₃, ethyl, propyl, isopropyl, butyl, sec-butyl, or tert-butyl), optionally halogenated C₁₋₆ alkyl (e.g., optionally halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, butoxy, sec-butoxy, tert-butoxy, pentyloxy, or hexyloxy), halogen (e.g., fluorine, chlorine, bromine, or iodine), amino, hydroxy, mercapto, cyano, carboxyl, C₂₋₆ alkynyl (e.g., C₂₋₄ alkynyl , such as ethynyl, propynyl, or butynyl), C₂₋₆ alkenyl (e.g., C₂₋₄ alkenyl, such as vinyl, propenyl, or butenyl), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), or R^{12a}-R^{11a}-, wherein R^{11a} is optionally substituted C₁₋₆ alkylene (e.g., optionally substituted C₁₋₄ alkylene, such as optionally substituted methylene, ethylene or propylene) or optionally substituted C₂₋₆ alkenylene (e.g., optionally substituted C₂₋₄ alkenylene, such as optionally substituted vinylene, propenylene, butenylene), and R^{12a} represents halogen (e.g., fluorine, chlorine, bromine, or iodine), R^{13a}R^{14a}N-, hydroxy, carboxyl, ethynyl, or vinyl, wherein R^{13a} and R^{14a} are the same or different and each independently represent hydrogen or C₁₋₃ alkyl (e.g., methyl, ethyl, or propyl), and the C₁₋₆ alkylene and C₂₋₆ alkenylene are optionally substituted with one or more substituents selected from the group consisting of halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₃ alkyl (e.g., halogenated C₁₋₂ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₃ alkoxy (e.g., methoxy, ethoxy, propoxy, or isopropoxy), halogenated C₁₋₃ alkoxy (e.g., halogenated C₁₋₂ alkoxy, such as F₃CO-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), or any combination thereof. m2 represents an integer of 0-20, such as an integer of 0, 1, 2, 3, 4, 5, 6, 7, 8, 9, 10, 11, 12, 13, 14, 15 or 20. The number of subsituents is not theoretically limited in any way or automatically limited by the size of the building units.

In some embodiments, each ring W⁶ is the same or different and each independently represents the following optionally substituted groups: 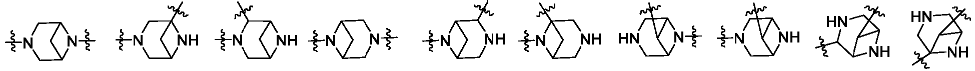 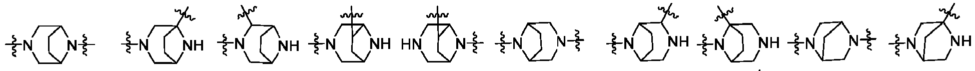 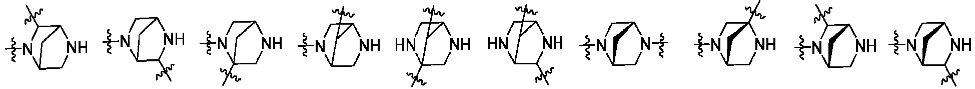 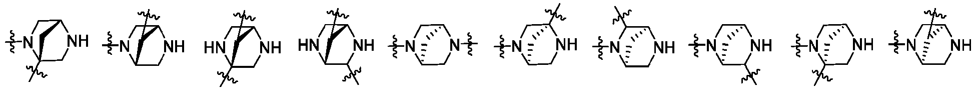 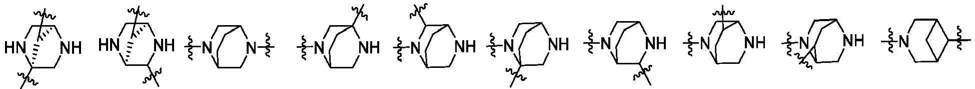 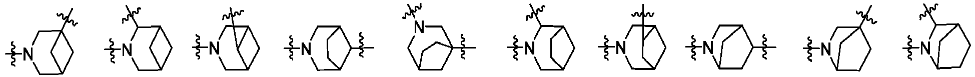 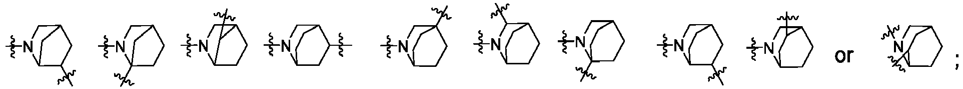 each ring W⁶ is independently optionally substituted with m2 R^{a2}, with each R^{a2} being independently deuterium, optionally deuterated C₁₋₆ alkyl (e.g., optionally deuterated C₁₋₄ alkyl, such as methyl, CD₃, ethyl, propyl, isopropyl, butyl, sec-butyl, or tert-butyl), optionally halogenated C₁₋₆ alkyl (e.g., optionally halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, butoxy, sec-butoxy, tert-butoxy, pentyloxy, or hexyloxy), halogen (e.g., fluorine, chlorine, bromine, or iodine), amino, hydroxy, mercapto, cyano, carboxyl, C₂₋₆ alkynyl (e.g., C₂₋₄ alkynyl , such as ethynyl, propynyl, or butynyl), C₂₋₆ alkenyl (e.g., C₂₋₄ alkenyl, such as vinyl, propenyl, or butenyl), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), or R^{12a}-R^{11a}-, wherein R^{11a} is optionally substituted C₁₋₆ alkylene (e.g., optionally substituted C₁₋₄ alkylene, such as optionally substituted methylene, ethylene or propylene) or optionally substituted C₂₋₆ alkenylene (e.g., optionally substituted C₂₋₄ alkenylene, such as optionally substituted vinylene, propenylene, butenylene), and R^{12a} represents halogen (e.g., fluorine, chlorine, bromine, or iodine), R^{13a}R^{14a}N-, hydroxy, carboxyl, ethynyl, or vinyl, wherein R^{13a} and R^{14a} are the same or different and each independently represent hydrogen or C₁₋₃ alkyl (e.g., methyl, ethyl, or propyl), and the C₁₋₆ alkylene and C₂₋₆ alkenylene are optionally substituted with one or more substituents selected from the group consisting of halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₃ alkyl (e.g., halogenated C₁₋₂ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₃ alkoxy (e.g., methoxy, ethoxy, propoxy, or isopropoxy), halogenated C₁₋₃ alkoxy (e.g., halogenated C₁₋₂ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), or any combination thereof. m2 represents an integer of 0-20, such as an integer of 0, 1, 2, 3, 4, 5, 6, 7, 8, 9, 10, 11, 12, 13, 14, 15 or 20. The number of subsituents is not theoretically limited in any way or automatically limited by the size of the building units.

In some embodiments, each ring W⁷ is the same or different and each independently represents 4- to 15-membered (e.g., 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) nitrogen-containing heterocyclylene, or 6- to 10-membered (e.g., 6- to 8-membered or 6- to 7-membered) arylene, or 5- to 10-membered (e.g., 5- to 9-membered, 5- to 8-membered, 5- to 7-memberedor 5- to 6-membered) heteroarylene. In some embodiments, the 4- to 15-membered nitrogen-containing heterocyclylene is 4- to 15-membered (e.g., 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) heterocyclylene containing one nitrogen atom and optionally heteroatoms selected from the group consisting of oxygen, nitrogen, and sulfur. Examples of the 4- to 15-membered nitrogen-containing heterocyclylene include, but are not limited to, e.g., piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene, diazacyclooctylene, azabicyclo[3.1.1]heptanylene, azabicyclo[2.2.1]heptanylene, azabicyclo[3.2. 1]octanylene, azabicyclo[2.2.2]octanylene, diazabicyclo[3.1. 1]heptanylene, diazabicyclo[2.2.1]heptanylene, diazabicyclo[3.2.1]octanylene, diazabicyclo[2.2.2]octanylene, 3-azaspiro[5.5]undecanylene, 8-azaspiro[4.5]decanylene, 2-oxa-8-azaspiro[4.5]decanylene or 3-methyl-2-oxa-8-azaspiro[4.5]decanylene. In some embodiments, examples of 6- to 10-membered arylene include, but are not limited to, e.g., phenylene or naphthylene. In some embodiments, 5- to 10-membered heteroarylene is 5- to 10-membered (e.g., 5- to 9-membered, 5- to 8-membered, 5- to 7-membered or 5- to 6-membered) heteroarylene containing one nitrogen atom and optionally heteroatoms selected from nitrogen, oxygen, and sulfur. In some embodiments, examples of 5- to 10-membered heteroarylene include, but are not limited to, e.g., oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene. Each ring W⁷ is independently optionally substituted with m3 R^{a3}, with each R^{a3} independently representing deuterium, optionally deuterated C₁₋₆ alkyl (e.g., optionally deuterated C₁₋₄ alkyl, such as methyl, CD₃, ethyl, propyl, isopropyl, butyl, sec-butyl, tert-butyl, pentyl, or hexyl), C₁₋₆ alkoxy (e.g., C₁₋₅ alkoxy or C₁₋₃ alkoxy, such as methoxy, ethoxy, propoxy, or isopropoxy), halogen (e.g., fluorine, chlorine, bromine, or iodine), amino, hydroxy, mercapto, cyano, carboxyl, C₂₋₆ alkynyl (e.g., C₂₋₄ alkynyl , such as ethynyl, propynyl, or butynyl), C₂₋₆ alkenyl (e.g., C₂₋₄ alkenyl, such as vinyl, propenyl, or butenyl), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), or R^{16a}-R^{15a}-, wherein R^{15a} is optionally substituted C₁₋₆ alkylene (e.g., optionally substituted C₁₋₄ alkylene, such as optionally substituted methylene, ethylene or propylene) or optionally substituted C₂₋₆ alkenylene (e.g., optionally substituted C₂₋₄ alkenylene, such as optionally substituted vinylene, propenylene, butenylene), and R^{16a} represents halogen (e.g., fluorine, chlorine, bromine, or iodine), R^{17a}R^{18a}N-, hydroxy, carboxyl, ethynyl, or vinyl, wherein R^{17a} and R^{18a} are the same or different and each independently represent hydrogen or C₁₋₃ alkyl (e.g., methyl, ethyl, or propyl). m3 represents an integer of 0-20, such as an integer of 0, 1, 2, 3, 4, 5, 6, 7, 8, 9, 10, 11, 12, 13, 14, 15 or 20. The number of subsituents is not theoretically limited in any way or automatically limited by the size of the building units.

In some embodiments, each ring W⁷ is the same or different and each independently represents: 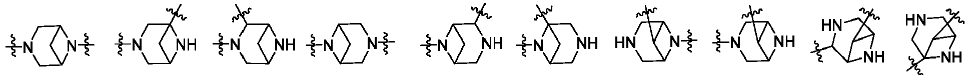 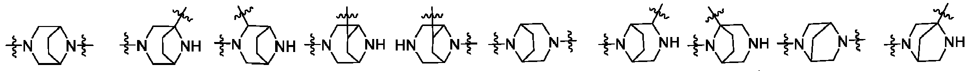 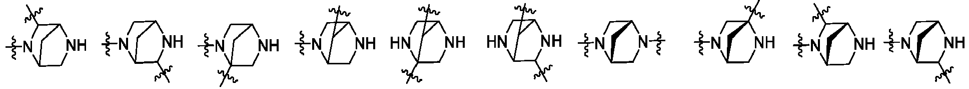 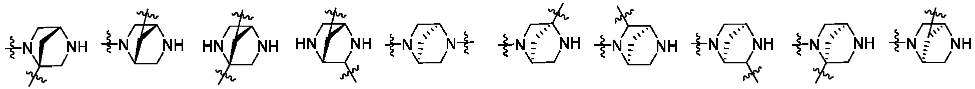 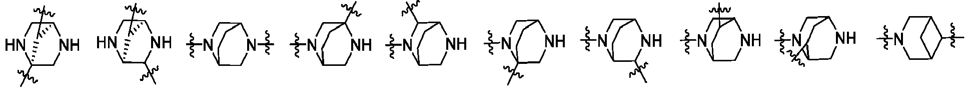 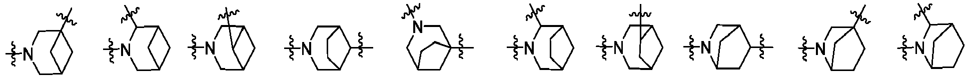 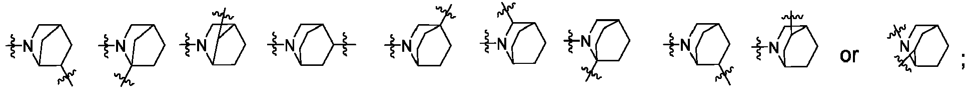 each ring W⁷ is independently optionally substituted with m3 R^{a3}, with each R^{a3} independently representing deuterium, optionally deuterated C₁₋₆ alkyl (e.g., optionally deuterated C₁₋₄ alkyl, such as methyl, CD₃, ethyl, propyl, isopropyl, butyl, sec-butyl, tert-butyl, pentyl, or hexyl), C₁₋₆ alkoxy (e.g., C₁₋₅ alkoxy or C₁₋₃ alkoxy, such as methoxy, ethoxy, propoxy, or isopropoxy), halogen (e.g., fluorine, chlorine, bromine, or iodine), amino, hydroxy, mercapto, cyano, carboxyl, C₂₋₆ alkynyl (e.g., C₂₋₄ alkynyl , such as ethynyl, propynyl, or butynyl), C₂₋₆ alkenyl (e.g., C₂₋₄ alkenyl, such as vinyl, propenyl, or butenyl), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), or R^{16a}-R^{15a}-, wherein R^{15a} is optionally substituted C₁₋₆ alkylene (e.g., optionally substituted C₁₋₄ alkylene, such as optionally substituted methylene, ethylene or propylene) or optionally substituted C₂₋₆ alkenylene (e.g., optionally substituted C₂₋₄ alkenylene, such as optionally substituted vinylene, propenylene, butenylene), and R^{16a} represents halogen (e.g., fluorine, chlorine, bromine, or iodine), R^{17a}R^{18a}N-, hydroxy, carboxyl, ethynyl, or vinyl, wherein R^{17a} and R^{18a} are the same or different and each independently represent hydrogen or C₁₋₃ alkyl (e.g., methyl, ethyl, or propyl). m3 represents an integer of 0-20, such as an integer of 0, 1, 2, 3, 4, 5, 6, 7, 8, 9, 10, 11, 12, 13, 14, 15 or 20. The number of subsituents is not theoretically limited in any way or automatically limited by the size of the building units.

In some embodiments, t2 represents an integer of 0.

In some embodiments, t2 represents an integer of 1.

In some embodiments, the W⁵ represents the following groups: 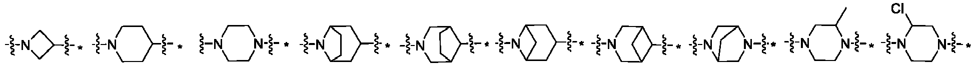 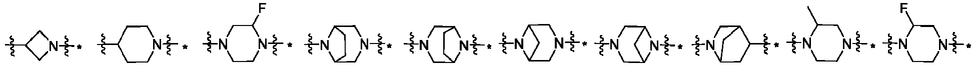 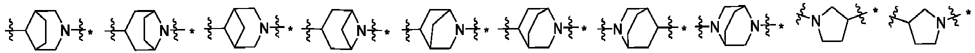 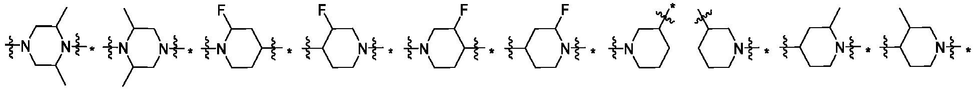 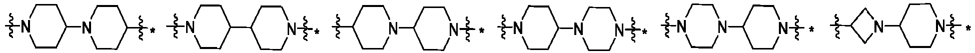 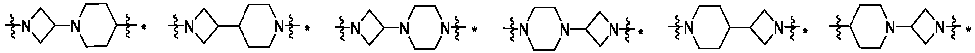 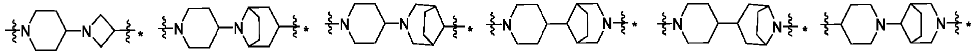 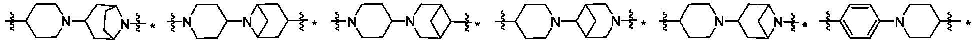 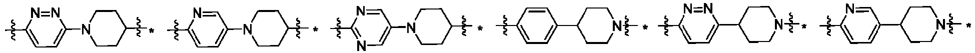 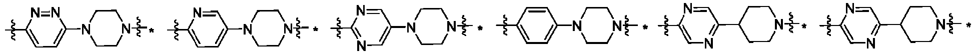 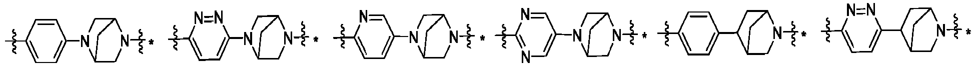 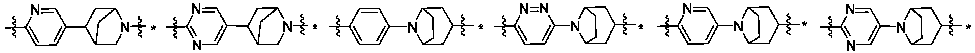 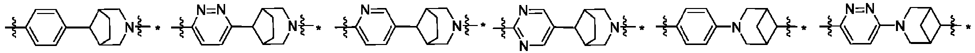 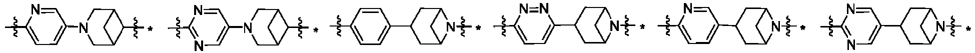 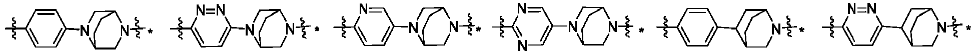 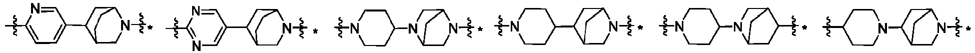 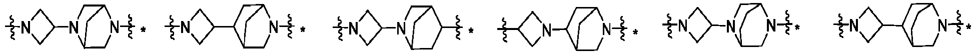 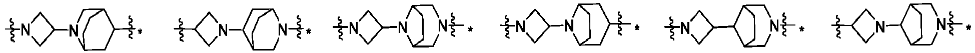 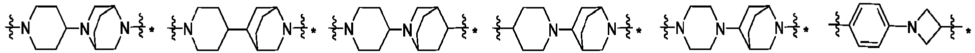 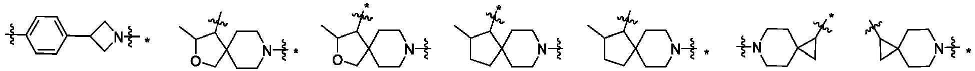 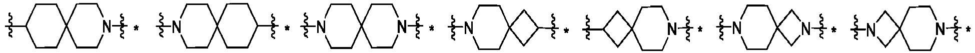 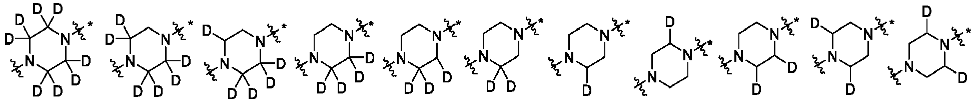 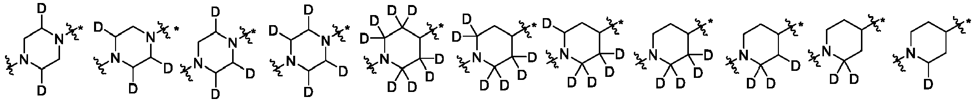 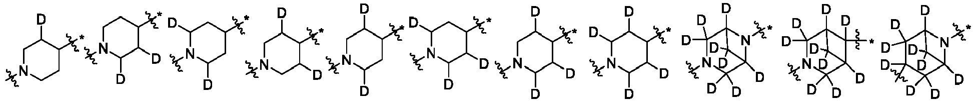 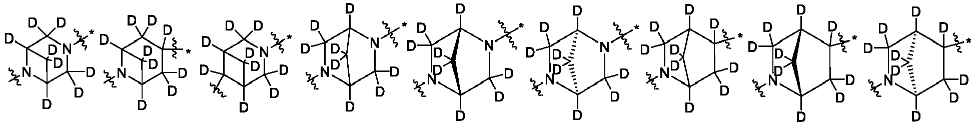 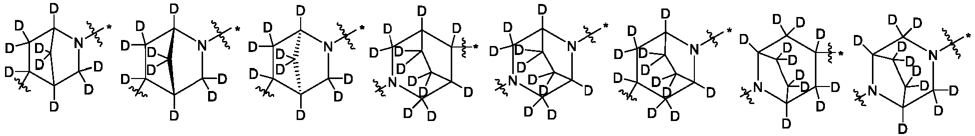 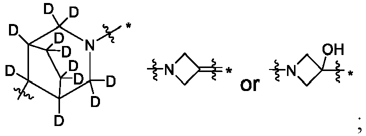
wherein the groups are optionally substituted with one or more substituents selected from the group consisting of deuterium, optionally deuterated C₁₋₆ alkyl (e.g., optionally deuterated C₁₋₄ alkyl, such as methyl, CD₃, ethyl, propyl, isopropyl, butyl, sec-butyl, tert-butyl, pentyl, or hexyl), optionally halogenated C₁₋₆ alkyl (e.g., optionally halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, CD₃-O-, ethoxy, propoxy, isopropoxy, butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkoxy (e.g., perdeuterated C₁₋₄ alkoxy, such as CD₃-O-, CD₃CD₂-O-, or CD₃CD₂CD₂-O-), halogen (e.g., fluorine, chlorine, bromine, or iodine), amino, hydroxy, mercapto, cyano, carboxyl, C₂₋₆ alkynyl (e.g., C₂₋₄ alkynyl , such as ethynyl, propynyl, or butynyl), C₂₋₆ alkenyl (e.g., C₂₋₄ alkenyl, such as vinyl, propenyl, or butenyl), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), R^{12a}-R^{11a}-, or any combination thereof, wherein R^{11a} is optionally substituted C₁₋₆ alkylene (e.g., optionally substituted C₁₋₄ alkylene, such as optionally substituted methylene, ethylene or propylene) or optionally substituted C₂₋₆ alkenylene (e.g., optionally substituted C₂₋₄ alkenylene, such as optionally substituted vinylene, propenylene, butenylene), and R^{12a} represents halogen (e.g., fluorine, chlorine, bromine, or iodine), R^{13a}R^{14a}N-, hydroxy, carboxyl, ethynyl, or vinyl, wherein R^{13a} and R^{14a} are the same or different and each independently represent hydrogen or C₁₋₃ alkyl (e.g., methyl, ethyl, or propyl); and
symbol * indicates the point of attachment to LIN.

In some embodiments, R_{f} of Formula (II) represents -W⁵-Rₕ-W⁵-*, wherein Rₕ represents optionally substituted C₁₋₆ alkylene or optionally substituted C₂₋₆ alkenylene, symbol * indicates the point of attachment to LIN, and W⁵ is as defined above. In some embodiments, Rₕ represents optionally substituted C₁₋₆ alkylene (e.g., C₁₋₅ alkylene, C₁₋₄ alkylene or C₁₋₃ alkylene, such as methylene, ethylene, propylene, isopropylene, butylene, sec-butylene, tertbutylene, pentylene or hexylene), and the C₁₋₆ alkylene is optionally substituted with one or more substituents selected from the group consisting of halogen (e.g., fluorine, chlorine, bromine, or iodine), hydroxy, mercapto, cyano, amino, nitro, C₁₋₆ alkyl, halogenated C₁₋₃ alkyl, C₁₋₃ alkoxy, halogenated C₁₋₃ alkoxy or any combination thereof. In some embodiments, Rₕ represents optionally substituted methylene, and the methylene is optionally substituted with one or more substituents selected from the group consisting of halogen (e.g., fluorine, chlorine, bromine, or iodine), hydroxy, mercapto, cyano, amino, nitro, C₁₋₆ alkyl, halogenated C₁₋₃ alkyl, C₁₋₃ alkoxy, halogenated C₁₋₃ alkoxy or any combination thereof.

In some embodiments, the R_{f} represents the following groups:
a bond, -O-, -NHC(O)-*, -C(O)NH-*, -NH-, -N(CH₃)-, -N(CH₃)-(CH₂)₂-N(CH₃)-*, -N(CH₃)-(CH₂)₃-N(CH₃)-*, -NH-(CH₂)₂-N(CH₃)-*, -N(CH₃)-(CH₂)₃-NH-*, -CH₂-NHC(O)-*, -CH₂-C(O)NH-*, -CH₂-NH-*, -O-(CH₂)₂-N(CH₃)-*, -O-(CH₂)₂-NH-*, or -CH₂-N(CH₃)-*, or 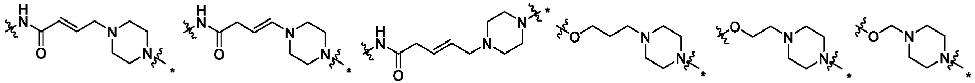 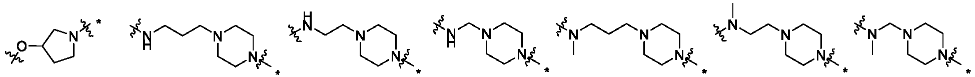 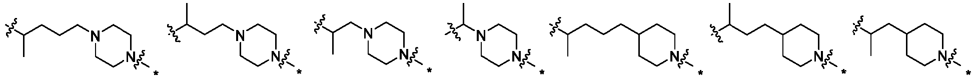 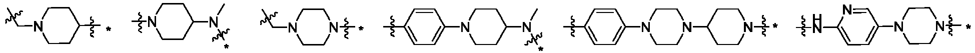 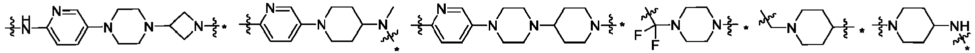 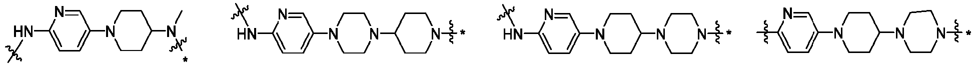 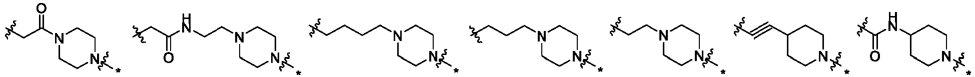 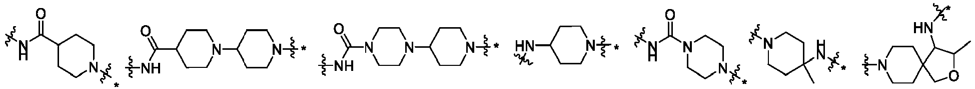 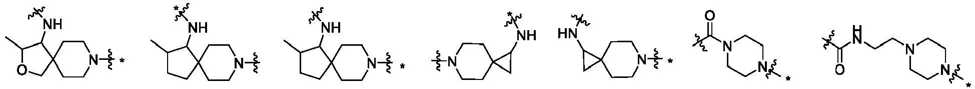 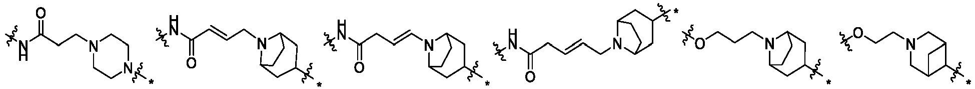 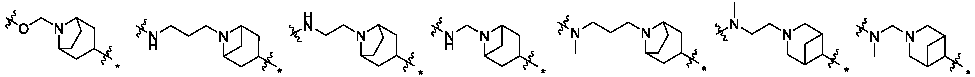 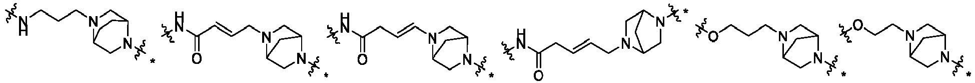 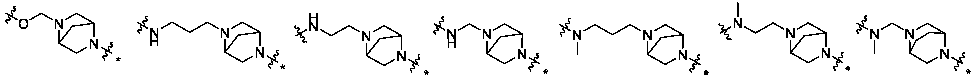 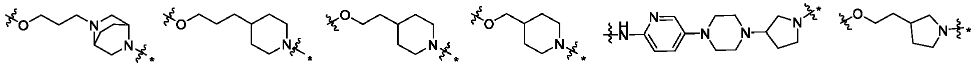 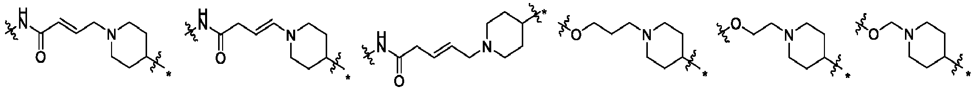 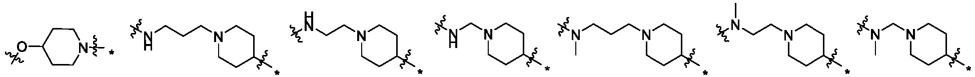 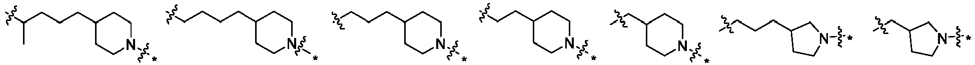 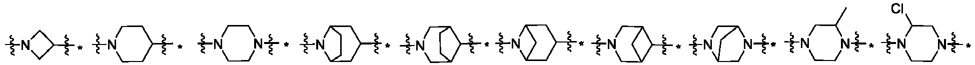 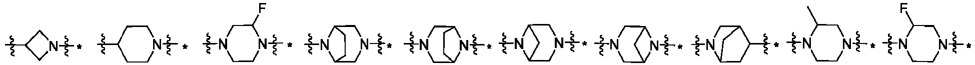 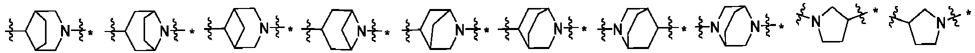 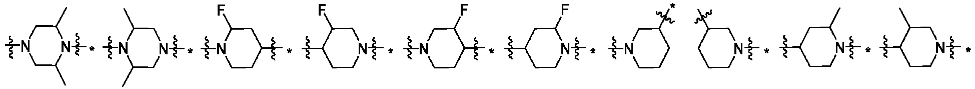 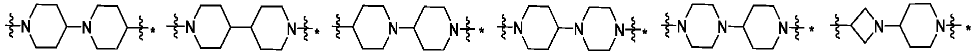 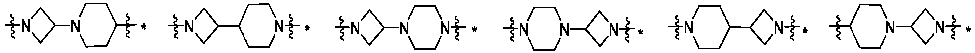 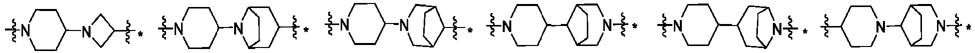 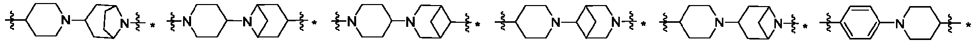 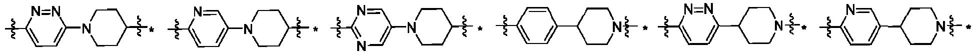 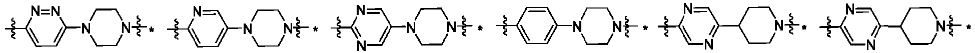 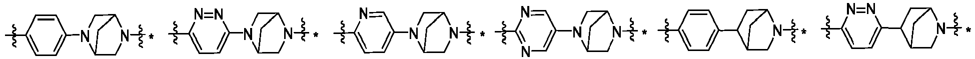 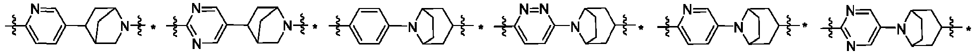 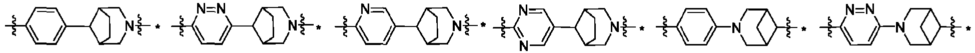 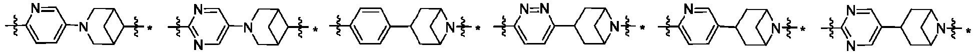 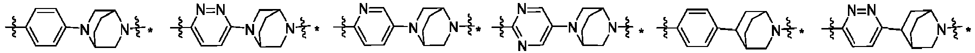 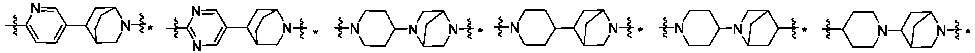 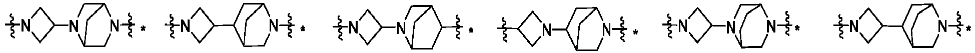 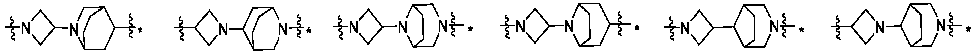 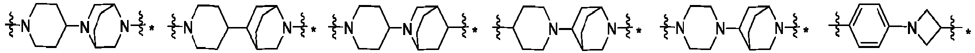 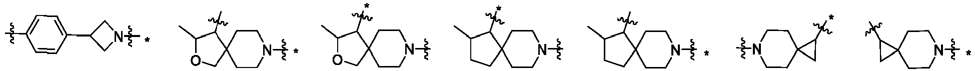 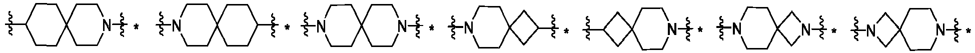 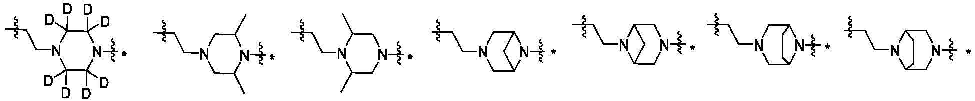 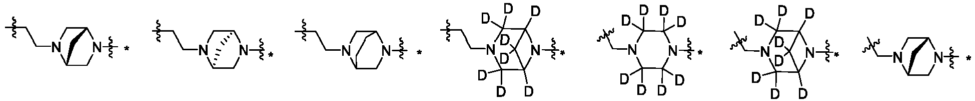 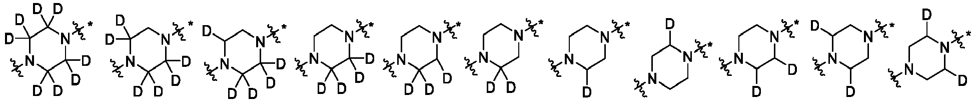 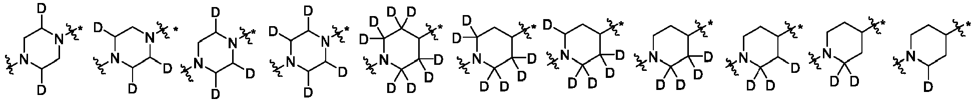 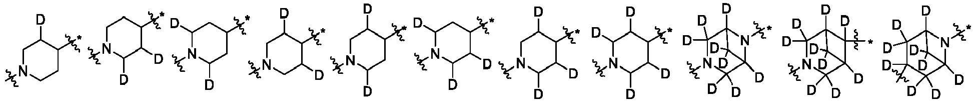 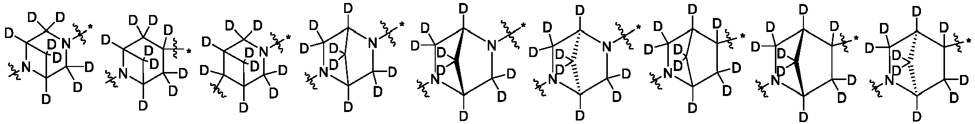 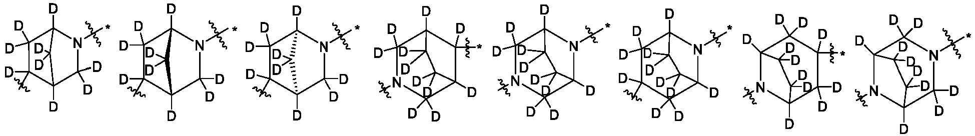 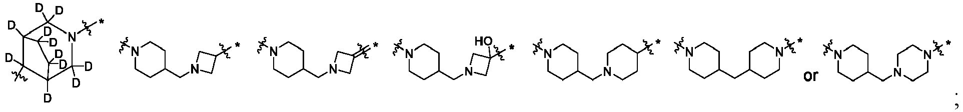 wherein the groups are optionally substituted with one or more substituents selected from the group consisting of deuterium, optionally deuterated C₁₋₆ alkyl (e.g., optionally deuterated C₁₋₄ alkyl, such as methyl, CD₃, ethyl, propyl, isopropyl, butyl, sec-butyl, or tert-butyl), optionally halogenated C₁₋₆ alkyl (e.g., optionally halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, butoxy, sec-butoxy, tert-butoxy, pentyloxy, or hexyloxy), halogen (e.g., fluorine, chlorine, bromine, or iodine), amino, hydroxy, mercapto, cyano, carboxyl, C₂₋₆ alkynyl (e.g., C₂₋₄ alkynyl , such as ethynyl, propynyl, or butynyl), C₂₋₆ alkenyl (e.g., C₂₋₄ alkenyl, such as vinyl, propenyl, or butenyl), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), R^{12a}-R^{11a}-, or any combination thereof, wherein R^{11a} is optionally substituted C₁₋₆ alkylene (e.g., optionally substituted C₁₋₄ alkylene, such as optionally substituted methylene, ethylene or propylene) or optionally substituted C₂₋₆ alkenylene (e.g., optionally substituted C₂₋₄ alkenylene, such as optionally substituted vinylene, propenylene, butenylene), and R^{12a} represents halogen (e.g., fluorine, chlorine, bromine, or iodine), R^{13a}R^{14a}N-, hydroxy, carboxyl, ethynyl, or vinyl, wherein R^{13a} and R^{14a} are the same or different and each independently represent hydrogen or C₁₋₃ alkyl (e.g., methyl, ethyl, or propyl); and
symbol * indicates the point of attachment to LIN.

In some embodiments, the ULM represents the structure of the following Formula (ULM-1), Formula (ULM-2), Formula (ULM-3), Formula (ULM-4), Formula (ULM-5), Formula (ULM-6), Formula (ULM-7), Formula (ULM-8), Formula (ULM-9), Formula (ULM-10) , Formula (ULM-11), or Formula (ULM-12): 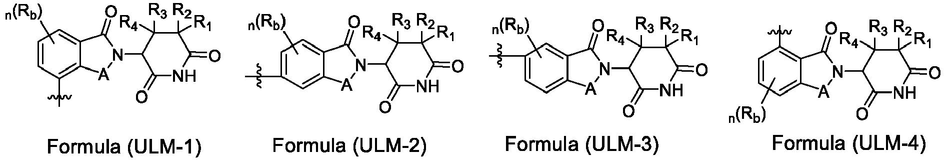 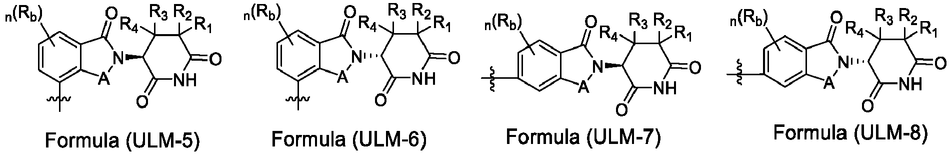 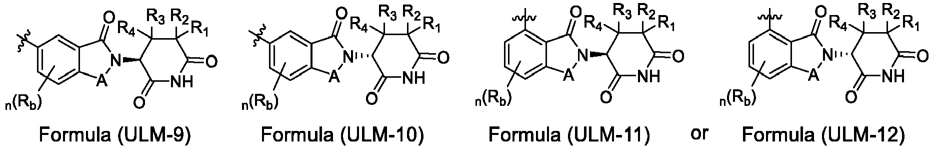 wherein
A represents CO, CH₂or CD₂;
R₁, R₂, R₃ and R₄ are the same or different and each independently represent hydrogen, deuterium, halogen (e.g., fluorine, chlorine, bromine, or iodine), optionally deuterated C₁₋₆ alkyl (e.g., optionally deuterated C₁₋₄ alkyl, such as methyl, CD₃, ethyl, propyl, isopropyl, butyl, sec-butyl, or tert-butyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
(R_{b})ₙ indicates that the benzene ring is optionally substituted with n R_{b}, with each R_{b} being the same or different and each independently representing hydrogen, deuterium, halogen (e.g., fluorine, chlorine, bromine, or iodine), hydroxy, mercapto, nitro, amino, cyano, optionally deuterated C₁₋₆ alkyl (e.g., optionally deuterated C₁₋₄ alkyl, such as methyl, CD₃, ethyl, propyl, isopropyl, butyl, sec-butyl, or tert-butyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally substituted C₃₋₆ cycloalkyl (e.g., optionally substituted cyclopropyl, optionally substituted cyclobutyl, optionally substituted cyclopentyl or optionally substituted cyclohexyl), C₂₋₆ alkenyl (e.g., C₂₋₄ alkenyl, such as vinyl, propenyl, or butenyl), or C₂₋₆ alkynyl (e.g., C₂₋₄ alkynyl , such as ethynyl, propynyl, or butynyl); and
n represents an integer of 0, 1, 2 or 3.

In some embodiments, the ULM represents the structure of the following Formula (ULM-13), Formula (ULM-14), Formula (ULM-15), Formula (ULM-16), Formula (ULM-17), Formula (ULM-18), Formula (ULM-19), Formula (ULM-20), Formula (ULM-21), Formula (ULM-22) , Formula (ULM-23), or Formula (ULM-24): 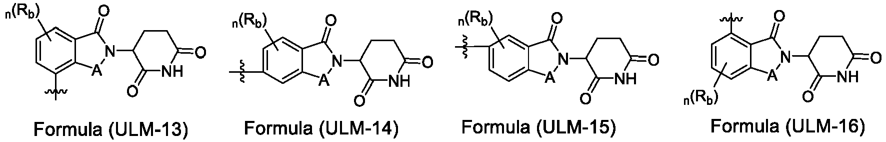 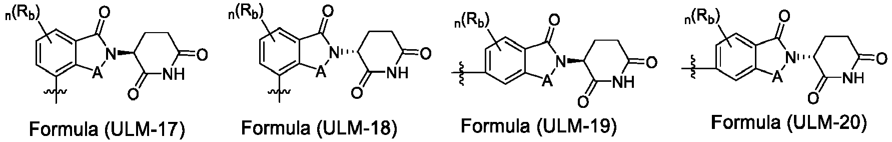 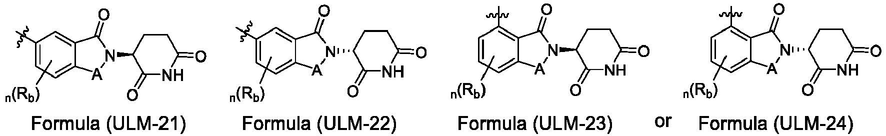 wherein the A, (R_{b})ₙ, R_{b} are as defined above.

In some embodiments, the ULM represents the structure of the following formula:

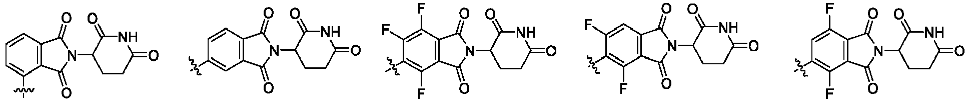

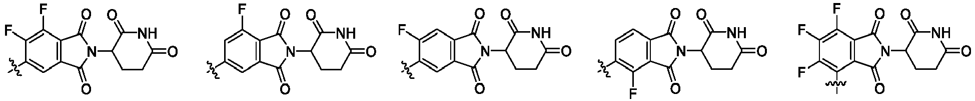

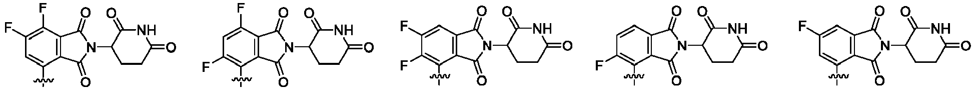

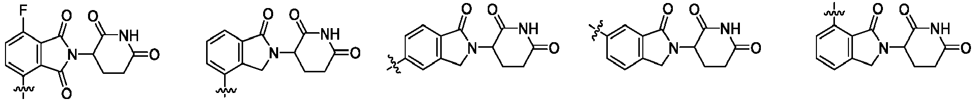

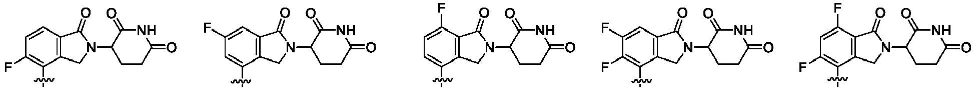

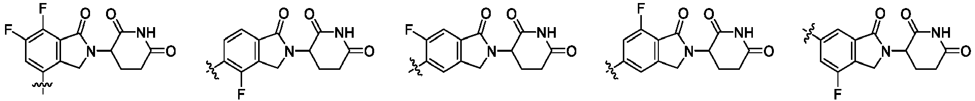

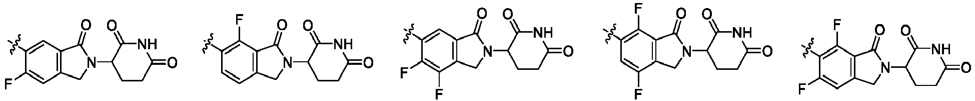

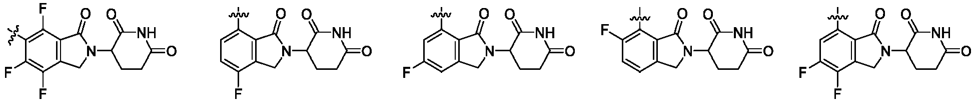

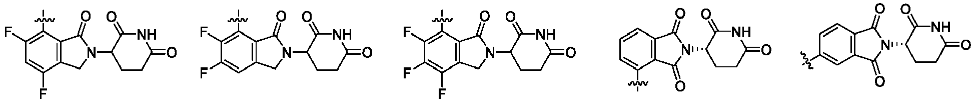

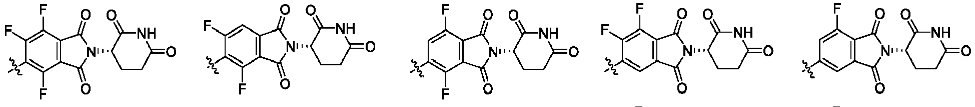

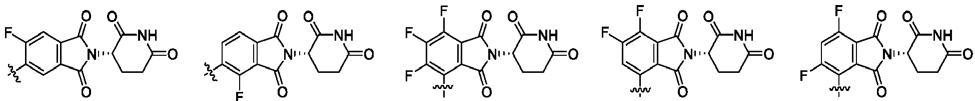

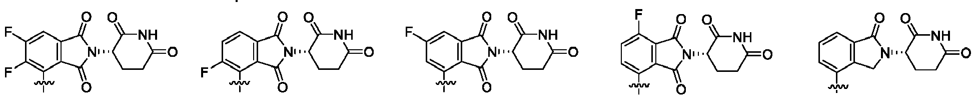

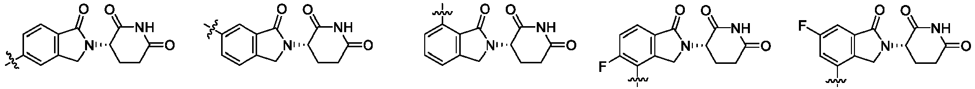

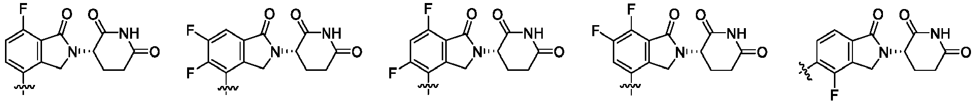

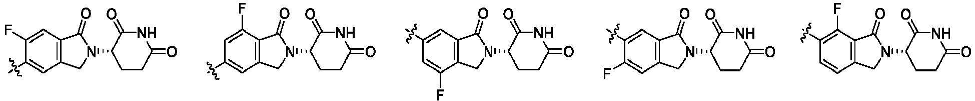

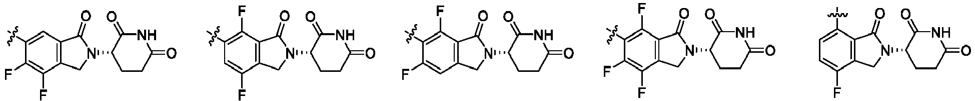

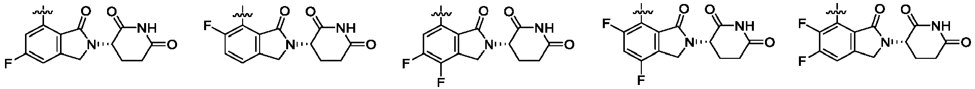

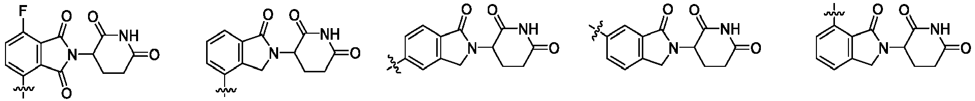

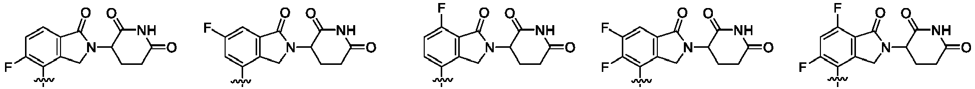

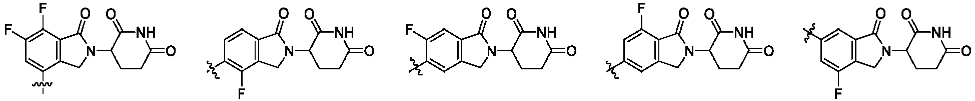

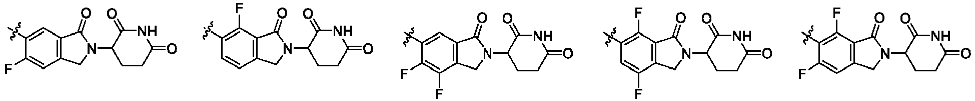

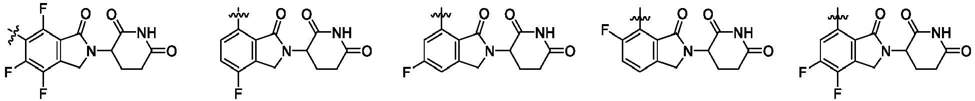

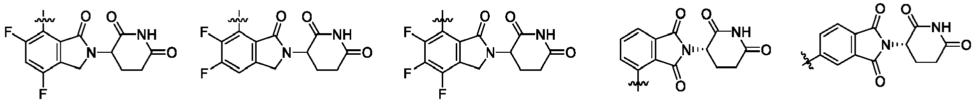

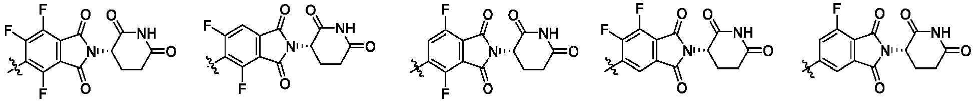

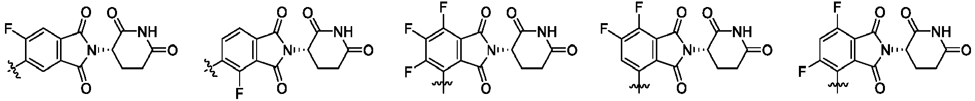

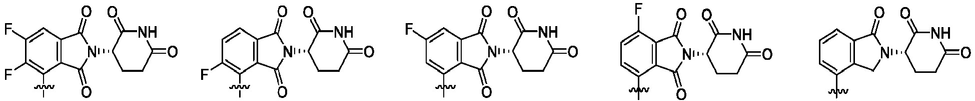

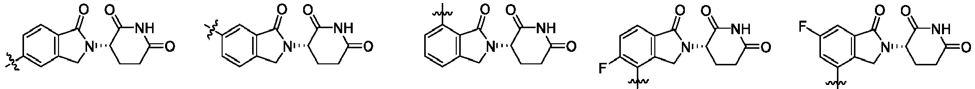

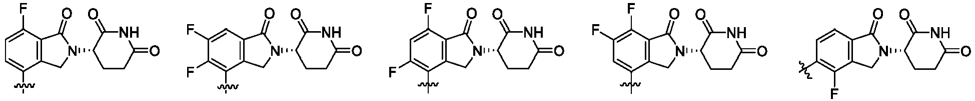

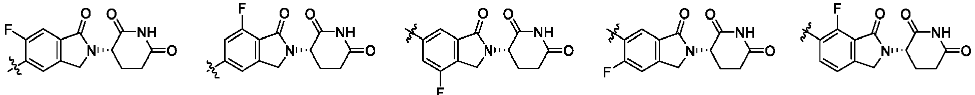

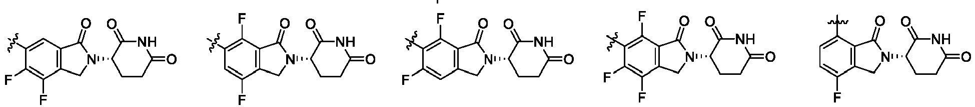

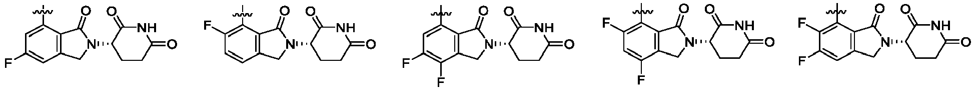

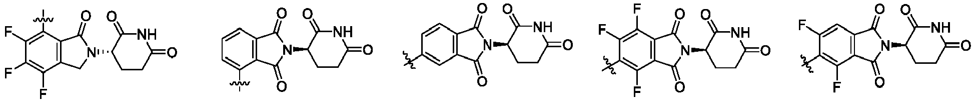

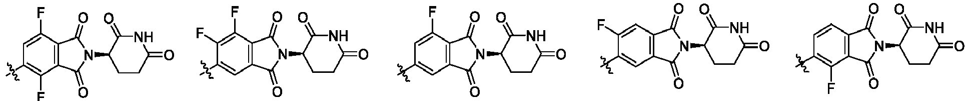

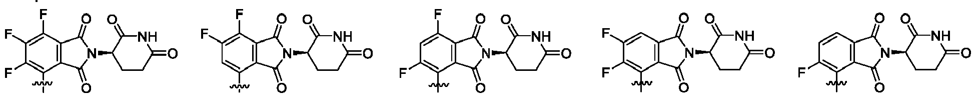

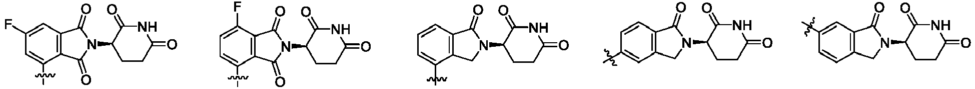

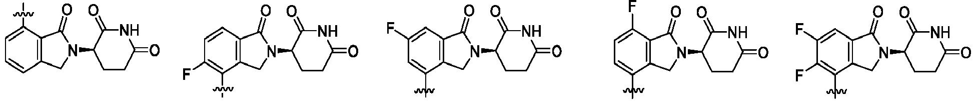

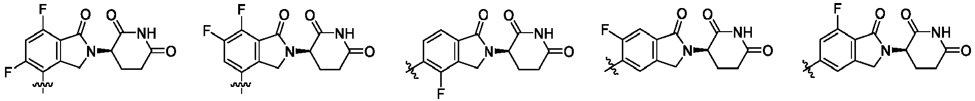

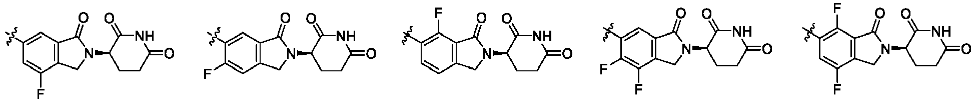

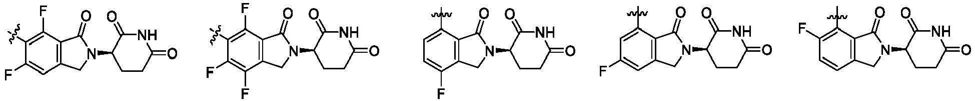

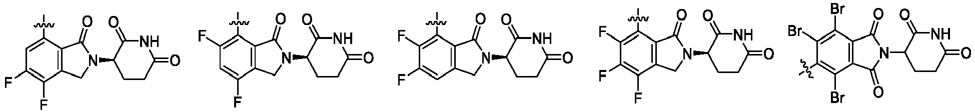

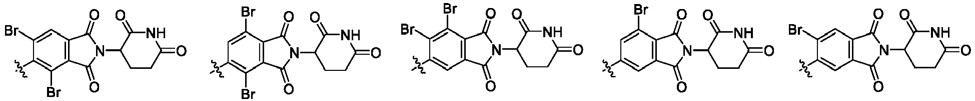

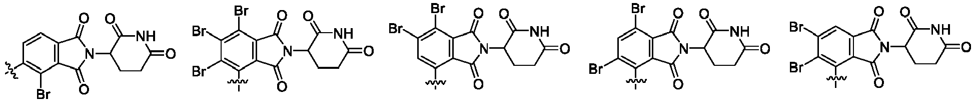

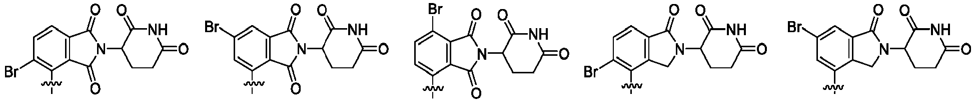

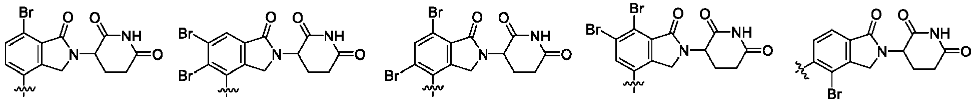

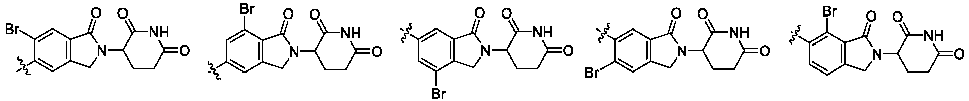

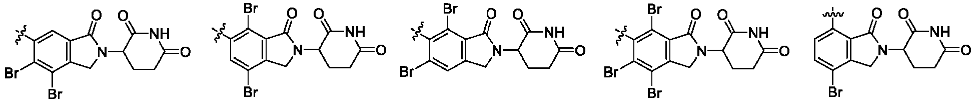

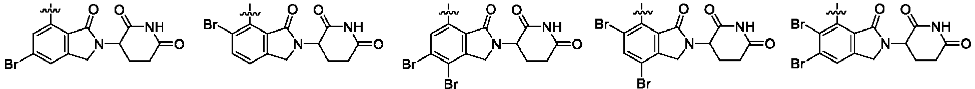

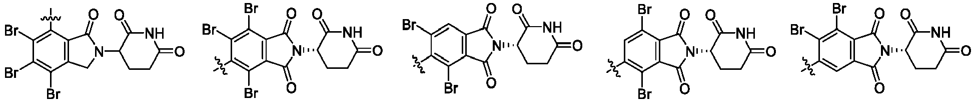

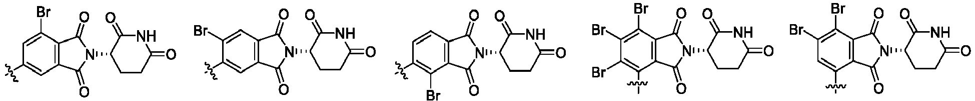

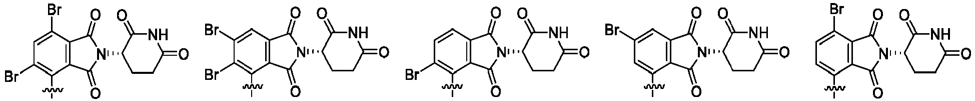

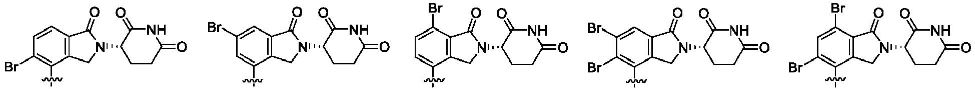

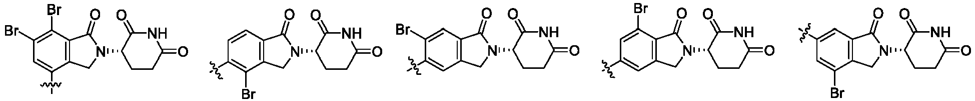

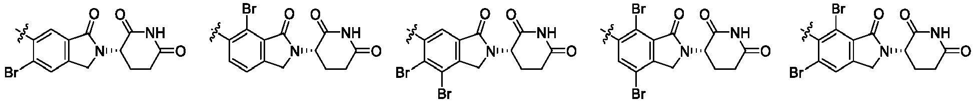

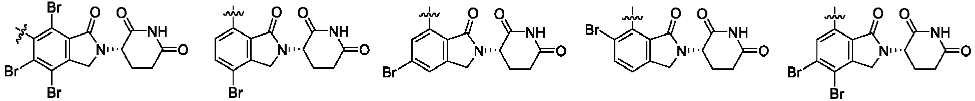

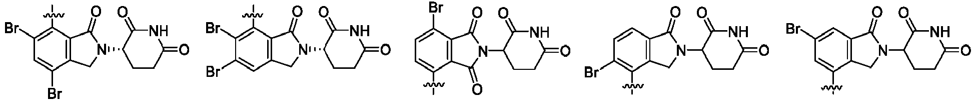

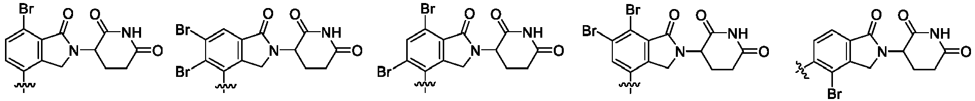

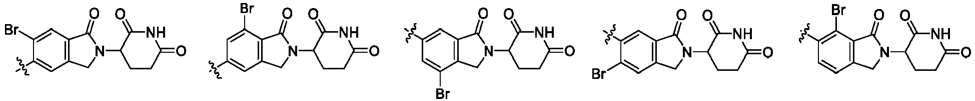

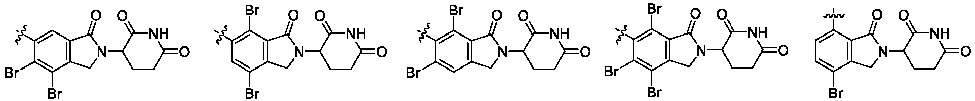

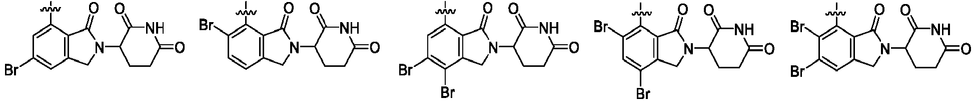

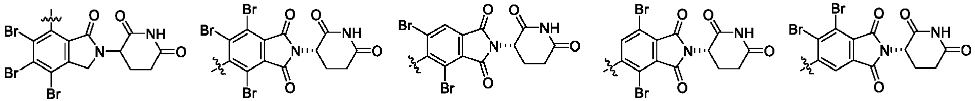

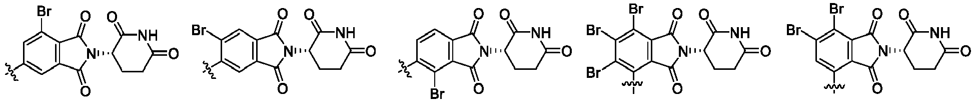

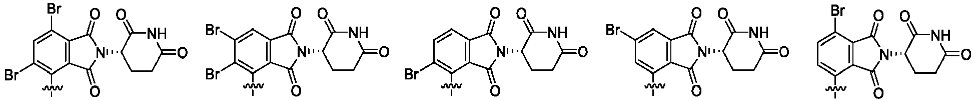

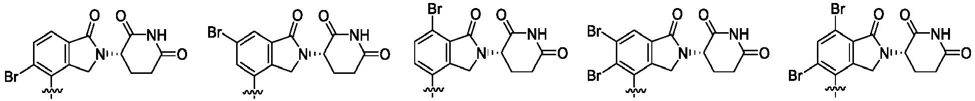

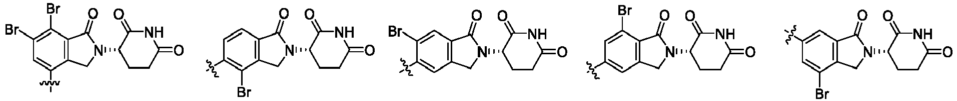

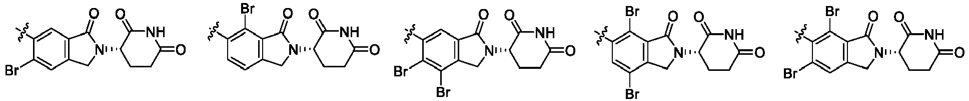

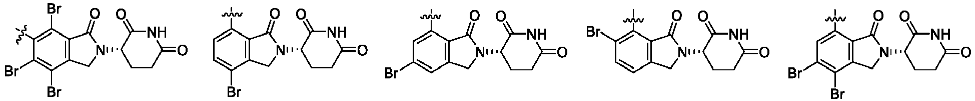

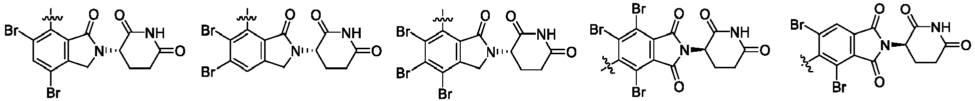

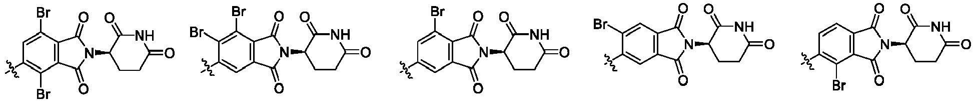

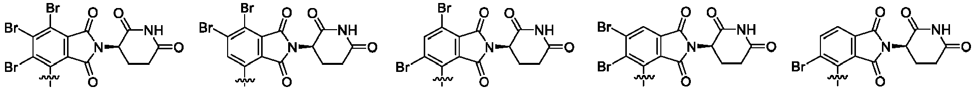

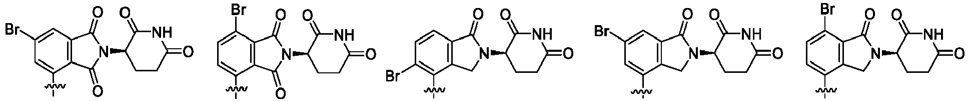

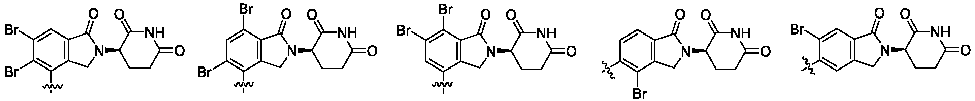

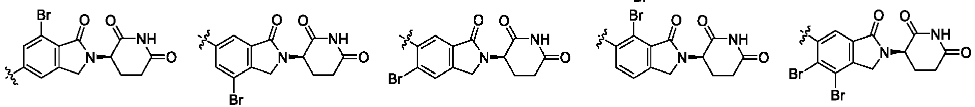

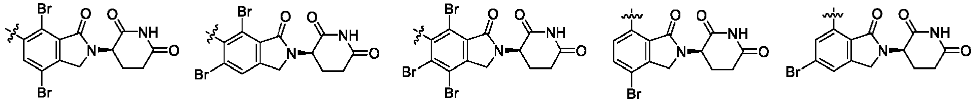

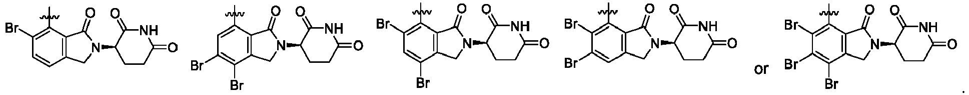

In some embodiments, LIN of the compound of Formula (II) represents the structure of the following formula: 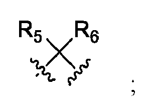 wherein R₅ and R₆ are the same or different and each independently represent:
hydrogen, fluorine, chlorine, bromine, iodine, optionally substituted methyl, or optionally substituted linear or branched C₂₋₃₀ alkyl,
wherein one or more groups R_{c} and/or one or more groups R_{d} and/or any combination of one or more groups R_{c} and R_{d} are optionally inserted into the backbone carbon chain of the linear or branched C₂₋₃₀ alkyl group, wherein carbon-carbon bond between one or more pairs of adjacent carbon atoms in the backbone carbon chain is interrupted by the group R_{c}, R_{d}, or a combination of R_{c} and R_{d}; wherein each R_{c} is selected from the group consisting of O, N(Rₑ), C(O), C(O)O, S(O), S(O)₂, S(O)₂NH, NHS(O)₂, OC(O), C(O)N(Rₑ), N(Rₑ)C(O), or N(Rₑ)C(O)N(Rₑ), where each Rₑ independently represents H or C₁₋₆ alkyl, and in case that two or more groups R_{c} are inserted into the backbone carbon chain of the linear or branched C₂₋₃₀ alkyl group, the two or more groups R_{c} are not directly connected to each other; and wherein each R_{d} is selected from the group consisting of cycloalkylene (e.g., C₃₋₂₀ cycloalkylene), arylene (e.g., C₃₋₂₀ arylene), heterocyclylene (e.g., 4- to 20-membered heterocyclylene), heteroarylene (e.g., 4- to 20-membered heteroarylene), alkynylene (e.g., C₂₋₆ alkynylene), alkenylene (e.g., C₂₋₆ alkenylene), or any combination thereof, wherein the cycloalkylene, arylene, heterocyclylene, and heteroarylene are each independently optionally substituted with a substituent selected from the group consisting of e.g., deuterium, optionally deuterated C₁₋₆ alkyl, optionally deuterated C₃₋₆ cycloalkyl, hydroxy, amino, mercapto, halogen, optionally deuterated C₁₋₆ alkoxy, optionally deuterated C₁₋₆ alkyl-NH-, halogenated C₁₋₆ alkyl, NH₂-C₁₋₆ alkylene, optionally deuterated C₁₋₆ alkyl-NHC(O)-, optionally deuterated C₁₋₆ alkyl-C(O)NH-, cyano or any combination thereof; or
a hydrogen of the methyl and a hydrogen of one or more CH₂ groups of the C₂₋₃₀ alkyl group are optionally replaced with a substituent selected from the group consisting of: deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

In some embodiments, R₅ of the compounds of Formula (II) represents hydrogen, fluorine, chlorine, bromine, iodine, or optionally substituted methyl. A hydrogen of the methyl is optionally substituted with a substituent selected from the group consisting of: deuterium, hydroxy, amino, mercapto, nitro, halogen (e.g., fluorine, chlorine, bromine, or iodine), cyano, optionally deuterated C₁₋₆ alkyl (e.g., optionally deuterated C₁₋₄ alkyl, such as methyl, CD₃, ethyl, propyl, isopropyl, butyl, sec-butyl, or tert-butyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), optionally deuterated C₁₋₆ alkyl-NH-(e.g., optionally deuterated C₁₋₃ alkyl-NH-, such as CH₃NH-, CH₃CH₂NH- or CH₃CH₂CH₂NH-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH-(e.g., optionally deuterated C₁₋₄ alkyl-C(O)NH-, such as CH₃-C(O)NH-, CH₃CH₂-C(O)NH-, and CH₃CH₂CH₂-C(O)NH-), or any combination thereof.

In some embodiments, R₅ of the compounds of Formula (II) represents optionally substituted linear or branched C₂₋₃₀ alkyl, wherein one or more groups R_{c} and/or one or more groups R_{d} and/or any combination of one or more groups R_{c} and R_{d} are optionally inserted into the backbone carbon chain of the linear or branched C₂₋₃₀ alkyl group, wherein carbon-carbon bond between one or more pairs of adjacent carbon atoms in the backbone carbon chain is interrupted by the group R_{c}, R_{d}, or a combination of R_{c} and Ra; wherein each R_{c} is selected from the group consisting of O, N(Rₑ), C(O), C(O)O, S(O), S(O)₂, S(O)₂NH, NHS(O)₂, OC(O), C(O)N(Rₑ), N(R_{c})C(O), or N(Rₑ)C(O)N(Rₑ), wherein each Rₑ independently represents H or C₁₋₆ alkyl, and in case that two or more groups R_{c} are inserted into the backbone carbon chain of the linear or branched C₂₋₃₀ alkyl group, the two or more groups R_{c} are not directly connected to each other; and wherein each R_{d} is selected from the group consisting of cycloalkylene (e.g., C₃₋₂₀ cycloalkylene), arylene (e.g., C₃₋₂₀ arylene), heterocyclylene (e.g., 4- to 20-membered heterocyclylene), heteroarylene (e.g., 4- to 20-membered heteroarylene), alkynylene (e.g., C₂₋₆ alkynylene), alkenylene (e.g., C₂₋₆ alkenylene), or any combination thereof, wherein the cycloalkylene, arylene, heterocyclylene, and heteroarylene are each independently optionally substituted with a substituent selected from the group consisting of deuterium, optionally deuterated C₁₋₆ alkyl (e.g., optionally deuterated C₁₋₄ alkyl, such as methyl, CD₃, ethyl, propyl, isopropyl, butyl, sec-butyl, or tert-butyl), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), hydroxy, amino, mercapto, halogen (e.g., fluorine, chlorine, bromine, or iodine), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), optionally deuterated C₁₋₆ alkyl-NH-(e.g., optionally deuterated C₁₋₃ alkyl-NH-, such as CH₃NH-, CH₃CH₂NH- or CH₃CH₂CH₂NH-), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH-(e.g., optionally deuterated C₁₋₄ alkyl-C(O)NH-, such as CH₃-C(O)NH-, CH₃CH₂-C(O)NH-, and CH₃CH₂CH₂-C(O)NH-), or any combination thereof; and
a hydrogen of one or more CH₂ groups of the C₂₋₃₀ alkyl group is optionally replaced with a substituent selected from the group consisting of: deuterium, hydroxy, amino, mercapto, nitro, halogen (e.g., fluorine, chlorine, bromine, or iodine), cyano, optionally deuterated C₁₋₆ alkyl (e.g., optionally deuterated C₁₋₄ alkyl, such as methyl, CD₃, ethyl, propyl, isopropyl, butyl, sec-butyl, or tert-butyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), optionally deuterated C₁₋₆ alkyl-NH-(e.g., optionally deuterated C₁₋₃ alkyl-NH-, such as CH₃NH-, CH₃CH₂NH- or CH₃CH₂CH₂NH-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)-(e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH-(e.g., optionally deuterated C₁₋₄ alkyl-C(O)NH-, such as CH₃-C(O)NH-, CH₃CH₂-C(O)NH-, and CH₃CH₂CH₂-C(O)NH-), or any combination thereof.

In some embodiments, R₆ of the compounds of Formula (II) represents hydrogen, fluorine, chlorine, bromine, iodine, or optionally substituted methyl. A hydrogen of the methyl is optionally replaced with a substituent selected from the group consisting of: deuterium, hydroxy, amino, mercapto, nitro, halogen (e.g., fluorine, chlorine, bromine, or iodine), cyano, optionally deuterated C₁₋₆ alkyl (e.g., optionally deuterated C₁₋₄ alkyl, such as methyl, CD₃, ethyl, propyl, isopropyl, butyl, sec-butyl, or tert-butyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), optionally deuterated C₁₋₆ alkyl-NH-(e.g., optionally deuterated C₁₋₃ alkyl-NH-, such as CH₃NH-, CH₃CH₂NH- or CH₃CH₂CH₂NH-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH-(e.g., optionally deuterated C₁₋₄ alkyl-C(O)NH-, such as CH₃-C(O)NH-, CH₃CH₂-C(O)NH-, and CH₃CH₂CH₂-C(O)NH-), or any combination thereof.

In some embodiments, R₆ of the compounds of Formula (II) represents optionally substituted linear or branched C₂₋₃₀ alkyl, wherein one or more groups R_{c} and/or one or more groups R_{d} and/or any combination of one or more groups R_{c} and R_{d} are optionally inserted into the backbone carbon chain of the linear or branched C₂₋₃₀ alkyl group, wherein carbon-carbon bond between one or more pairs of adjacent carbon atoms in the backbone carbon chain is interrupted by the group R_{c}, R_{d}, or a combination of R_{c} and R_{d}; wherein each R_{c} is selected from the group consisting of O, N(Rₑ), C(O), C(O)O, S(O), S(O)₂, S(O)₂NH, NHS(O)₂, OC(O), C(O)N(Rₑ), N(Rₑ)C(O), or N(Rₑ)C(O)N(Rₑ), wherein each Rₑ independently represents H or C₁₋₆ alkyl, and in case that two or more groups R_{c} are inserted into the backbone carbon chain of the linear or branched C₂₋₃₀ alkyl group, the two or more groups R_{c} are not directly connected to each other; and wherein each R_{d} is selected from the group consisting of cycloalkylene (e.g., C₃₋₂₀ cycloalkylene), arylene (e.g., C₃₋₂₀ arylene), heterocyclylene (e.g., 4- to 20-membered heterocyclylene), heteroarylene (e.g., 4- to 20-membered heteroarylene), alkynylene (e.g., C₂₋₆ alkynylene), alkenylene (e.g., C₂₋₆ alkenylene) or any combination thereof, wherein the cycloalkylene, the arylene, the heterocyclylene and the heteroarylene are each independently optionally substituted with a substituent selected from the group consisting of deuterium, optionally deuterated C₁₋₆ alkyl (e.g., optionally deuterated C₁₋₄ alkyl, such as methyl, CD₃, ethyl, propyl, isopropyl, butyl, sec-butyl, or tert-butyl), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), hydroxy, amino, mercapto, halogen (e.g., fluorine, chlorine, bromine, or iodine), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), optionally deuterated C₁₋₆ alkyl-NH-(e.g., optionally deuterated C₁₋₃ alkyl-NH-, such as CH₃NH-, CH₃CH₂NH- or CH₃CH₂CH₂NH-), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH-(e.g., optionally deuterated C₁₋₄ alkyl-C(O)NH-, such as CH₃-C(O)NH-, CH₃CH₂-C(O)NH-, and CH₃CH₂CH₂-C(O)NH-), or any combination thereof; and
a hydrogen of one or more CH₂ groups of the C₂₋₃₀ alkyl group is optionally replaced with a substituent selected from the group consisting of: deuterium, hydroxy, amino, mercapto, nitro, halogen (e.g., fluorine, chlorine, bromine, or iodine), cyano, optionally deuterated C₁₋₆ alkyl (e.g., optionally deuterated C₁₋₄ alkyl, such as methyl, CD₃, ethyl, propyl, isopropyl, butyl, sec-butyl, or tert-butyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), optionally deuterated C₁₋₆ alkyl-NH-(e.g., optionally deuterated C₁₋₃ alkyl-NH-, such as CH₃NH-, CH₃CH₂NH- or CH₃CH₂CH₂NH-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)-(e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH-(e.g., optionally deuterated C₁₋₄ alkyl-C(O)NH-, such as CH₃-C(O)NH-, CH₃CH₂-C(O)NH-, and CH₃CH₂CH₂-C(O)NH-), or any combination thereof.

In some embodiments, R₅ and R₆ of the compounds of Formula (II) are the same or different and each independently represent:
hydrogen, fluorine, chlorine, bromine, iodine, or
-C₁₋₃₀ alkyl;
-(C(R^{a4})(R^{a5}))ₙ₁-(R_{c}-(C(R^{a6})(R^{a7}))ₙ₂)ₘ₄-H;
-(C(R^{a4})(R^{a5}))ₙ₁-(R_{c}-(C(R^{a6})(R^{a7}))ₙ₂)ₘ₄-(R_{c}-(C(R^{a8})(R^{a9}))ₙ₃)ₘ₅-H;
-(C(R^{a4})(R^{a5}))ₙ₁-(R_{c}-(C(R^{a6})(R^{a7}))ₙ₂)ₘ₄-(R_{c}-(C(R^{a8})(R^{a9}))ₙ₃)ₘ₅-(R_{c}-(C(R^{a10})(R^{a11}))ₙ₄)ₘ₆-H;
-(C(R^{a4})(R^{a5}))ₙ₁-(R_{d}-(C(R^{a6})(R^{a7}))ₙ₂)ₘ₄-H;
-(C(R^{a4})(R^{a5}))ₙ₁-(R_{d}-(C(R^{a6})(R^{a7}))ₙ₂)ₘ₄-(R_{d}-(C(R^{a8})(R^{a9}))ₙ₃)ₘ₅-H;
-(C(R^{a4})(R^{a5}))ₙ₁-(R_{d}-(C(R^{a6})(R^{a7}))ₙ₂)ₘ₄-(R_{d}-(C(R^{a8})(R^{a9}))ₙ₃)ₘ₅-(R_{d}-(C(R^{a10})(R^{a11}))ₙ₄)ₘ₆-H;
-(C(R^{a4})(R^{a5}))ₙ₁-(R_{c}-R_{d}-(C(R^{a6})(R^{a7}))ₙ₂)ₘ₄-CH₃;
-(C(R^{a4})(R^{a5}))ₙ₁-(R_{c}-(C(R^{a6})(R^{a7}))ₙ₂)ₘ₄-(R_{d}-(C(R^{a8})(R^{a9}))ₙ₃)ₘ₅-H;
-(C(Ra⁴)(R^{a5}))ₙ₁-(R_{d}-R_{c}-(C(R^{a6})(R^{a7}))ₙ₂)ₘ₄-H;or
-(C(R^{a4})(R^{a5}))ₙ₁-(R_{d}-(C(R^{a6})(R^{a7}))ₙ₂)ₘ₄-(R_{c}-(C(R^{a8})(R^{a9}))ₙ₃)ₘ₅-H;

wherein each R_{c} is selected from the group consisting of O, N(Rₑ), C(O), C(O)O, OC(O), S(O), S(O)₂, S(O)₂NH, NHS(O)₂, C(O)N(Rₑ), N(Rₑ)C(O), or N(Rₑ)C(O)N(Rₑ), wherein each Rₑ independently represents H or C₁₋₆ alkyl (e.g., C₁₋₄ alkyl, such as methyl, ethyl, propyl, isopropyl, butyl, sec-butyl or tert-butyl); and each R_{d} is selected from the group consisting of cycloalkylene (e.g., C₃₋₂₀cycloalkylene), arylene (e.g., C₃₋₂₀ arylene), heterocyclylene (e.g., 4- to 20-membered heterocyclylene), heteroarylene (e.g., 4- to 20-membered heteroarylene), alkynylene (e.g., C₂₋₆ alkynylene), alkenylene (e.g., C₂₋₆ alkenylene) or any combination thereof, wherein the cycloalkylene, arylene, heterocyclylene and heteroarylene are independently optionally substituted with a substituent selected from the group consisting of deuterium, optionally deuterated C₁₋₆ alkyl (e.g., optionally deuterated C₁₋₄ alkyl, such as methyl, CD₃, ethyl, propyl, isopropyl, butyl, sec-butyl, or tert-butyl), optionally deuterated C₃₋₆ cycloalkyl (e.g., optionally deuterated cyclopropyl, cyclobutyl, cyclopentyl, or cyclohexyl), hydroxy, amino, mercapto, halogen (e.g., fluorine, chlorine, bromine, or iodine), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), optionally deuterated C₁₋₆ alkyl-NH-(e.g., optionally deuterated C₁₋₃ alkyl-NH-, such as CH₃NH-, CH₃CH₂NH- or CH₃CH₂CH₂NH-), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH-(e.g., optionally deuterated C₁₋₄ alkyl-C(O)NH-, such as CH₃-C(O)NH-, CH₃CH₂-C(O)NH-, and CH₃CH₂CH₂-C(O)NH-), cyano or any combination thereof;
a hydrogen of one or more CH₂ groups of the C₁₋₃₀ alkyl group is optionally substituted with a substituent selected from the group consisting of: deuterium, hydroxy, amino, mercapto, nitro, halogen (e.g., fluorine, chlorine, bromine, or iodine), cyano, optionally deuterated C₁₋₆ alkyl (e.g., optionally deuterated C₁₋₄ alkyl, such as methyl, CD₃, ethyl, propyl, isopropyl, butyl, sec-butyl, or tert-butyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally deuterated cyclopropyl, cyclobutyl, cyclopentyl, or cyclohexyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), optionally deuterated C₁₋₆ alkyl-NH-(e.g., optionally deuterated C₁₋₃ alkyl-NH-, such as CH₃NH-, CH₃CH₂NH- or CH₃CH₂CH₂NH-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH-(e.g., optionally deuterated C₁₋₄ alkyl-C(O)NH-, such as CH₃-C(O)NH-, CH₃CH₂-C(O)NH-, and CH₃CH₂CH₂-C(O)NH-) or any combination thereof;
R^{a4}, R^{a5}, R^{a6}, R^{a7}, R^{a8}, R^{a9}, R^{a10} and R^{a11} independently represent H, deuterium, hydroxy, amino, mercapto, nitro, halogen (e.g., fluorine, chlorine, bromine, or iodine), cyano, optionally deuterated C₁₋₆ alkyl (e.g., optionally deuterated C₁₋₄ alkyl, such as methyl, CD₃, ethyl, propyl, isopropyl, butyl, sec-butyl, or tert-butyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally deuterated cyclopropyl, cyclobutyl, cyclopentyl, or cyclohexyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), optionally deuterated C₁₋₆ alkyl-NH- (e.g., optionally deuterated C₁₋₃ alkyl-NH-, such as CH₃NH-, CH₃CH₂NH-or CH₃CH₂CH₂NH-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH-(e.g., optionally deuterated C₁₋₄ alkyl-C(O)NH-, such as CH₃-C(O)NH-, CH₃CH₂-C(O)NH-, and CH₃CH₂CH₂-C(O)NH-) or any combination thereof; and
n1, n2, n3, n4, m4, m5, m6 are independently selected from the group consisting of an integer of 1, 2, 3, 4, 5, 6, 7, 8, 9, 10, 11, 12, 13, 14, or 15.

In some embodiments, R₅ and R₆ of the compounds of Formula (II) are the same or different and each independently represent the structure of the following formula:
-(C(R^{a4})(R^{a5}))ₙ₁-(O-(C(R^{a6})(R^{a7}))ₙ₂)ₘ₄-H;
-(C(R^{a4})(R^{a5}))ₙ₁-(O-(C(R^{a6})(R^{a7}))ₙ₂)ₘ₄-(O-(C(R^{a8})(R^{a9}))ₙ₃)ₘ₅-H;
-(C(R^{a4})(R^{a5}))ₙ₁-(O-(C(R^{a6})(R^{a7}))ₙ₂)ₘ₄-(O-(C(R^{a8})(R^{a9}))ₙ₃)ₘ₅-(O(C(R^{a10})(R^{a11}))ₙ₄)ₘ₆-H;
-(C(R^{a4})(R^{a5}))ₙ₁-(N(Rₑ)-(C(R^{a6})(R^{a7}))ₙ₂)ₘ₄-H;
-(C(R^{a4})(R^{a5}))ₙ₁-(N(Rₑ)-(C(R^{a6})(R^{a7}))ₙ₂)ₘ₄-(N(Rₑ)-(C(R^{a8})(R^{a9}))ₙ₃)ₘ₅-H;
-(C(R^{a4})(R^{a5}))ₙ₁-(N(Rₑ)-(C(R^{a6})(R^{a7}))ₙ₂)ₘ₄-(N(Rₑ)-(C(R^{a8})(R^{a9}))ₙ₃)ₘ₅-(N(Rₑ)-(C(R^{a10})(R^{a11}))ₙ₄)ₘ₆-H;
-(C(R^{a4})(R^{a5}))ₙ₁-(C(O)N(Rₑ)-(C(R^{a6})(R^{a7}))ₙ₂)ₘ₄-H;
-(C(R^{a4})(R^{a5}))ₙ₁-(C(O)N(Rₑ)-(C(R^{a6})(R^{a7}))ₙ₂)ₘ₄-(C(O)N(Rₑ)-(C(R^{a8})(R^{a9}))ₙ₃)ₘ₅-H;
-(C(R^{a4})(R^{a5}))ₙ₁-(C(O)N(Rₑ)-(C(R^{a6})(R^{a7}))ₙ₂)ₘ₄-(C(O)N(Rₑ)-(C(R^{a8})(R^{a9}))ₙ₃)ₘ₅-(C(O)N(Rₑ)-(C(R^{a10})(R^{a11}))ₙ₄)ₘ₆-H;
-(C(R^{a4})(R^{a5}))ₙ₁-(C(O)N(Rₑ)-(C(R^{a6})(R^{a7}))ₙ₂)ₘ₄-(O-(C(R^{a8})(R^{a9}))ₙ₃)ₘ₅-H;
-(C(R^{a4})(R^{a5}))ₙ₁-C(O)N(Rₑ)-(C(R^{a6})(R^{a7}))ₙ₂-(O-(C(R^{a8})(R^{a9}))ₙ₃)ₘ₄-H;
-(C(R^{a4})(R^{a5}))ₙ₁-(N(Rₑ)C(O)-(C(R^{a6})(R^{a7}))ₙ₂)ₘ₄-H;
-(C(R^{a4})(R^{a5}))ₙ₁-(N(Rₑ)C(O)-(C(R^{a6})(R^{a7}))ₙ₂)ₘ₄-(O-(C(R^{a8})(R^{a9}))ₙ₃)ₘ₅-H;
-(C(R^{a4})(R^{a5}))ₙ₁-(N(Rₑ)C(O)-(C(R^{a6})(R^{a7}))ₙ₂)ₘ₄-(O-(C(R^{a8})(R^{a9}))ₙ₃)ₘ₅-(O-(C(R^{a10})(R^{a11}))ₙ₄)ₘ₆-H;
-(C(R^{a4})(R^{a5}))ₙ₁-N(Rₑ)C(O)-(C(R^{a6})(R^{a7}))ₙ₂-(O-(C(R^{a8})(R^{a9}))ₙ₃)ₘ₄-H;
-(C(R^{a4})(R^{a5}))ₙ₁-N(Rₑ)C(O)N(Rₑ)-(C(R^{a6})(R^{a7}))ₙ₂-H;
-(C(R^{a4})(R^{a5}))ₙ₁-C(O)-(C(R^{a6})(R^{a7}))ₙ₂-H;
-(C(R^{a4})(R^{a5}))ₙ₁-(arylene-(C(R^{a6})(R^{a7}))ₙ₂)ₘ₄-H;
-(C(R^{a4})(R^{a5}))ₙ₁-arylene-(C(R^{a6})(R^{a7}))ₙ₂)ₘ₄-arylene-(C(R^{a8})(R^{a9}))ₙ₃-H;
-(C(R^{a4})(R^{a5}))ₙ₁-(heterocyclylene-(C(R^{a6})(R^{a7}))ₙ₂)ₘ₄-H;
-(C(R^{a4})(R^{a5}))ₙ₁-(heterocyclylene-(C(R^{a6} )(R^{a7}))ₙ₂)ₘ₄-(heterocyclylene-(C(R^{a8})(R^{a9}))ₙ₃)ₘ₅-H;
-(C(R^{a4})(R^{a5}))ₙ₁-(heteroarylene-(C(R^{a6})(R^{a7}))ₙ₂)ₘ₄-H;
-(C(R^{a4})(R^{a5}))ₙ₁-(heteroarylene-(C(R^{a6})(R^{a7}))ₙ₂)ₘ₄-(heteroarylene-(C(R^{a8})(R^{a9}))ₙ₃)ₘ₅-H;
-(C(R^{a4})(R^{a5}))ₙ₁-(cycloalkylene-(C(R^{a6})(R^{a7}))ₙ₂)ₘ₄-H;or
-(C(R^{a4})(R^{a5}))ₙ₁-(cycloalkylene-(C(R^{a6})(R^{a7}))ₙ₂)ₘ₄-(cycloalkylene-(C(R^{a8})(R^{a9}))ₙ₃)ₘ₅-H;

wherein each Rₑ independently represents H or C₁₋₆ alkyl (e.g., C₁₋₄ alkyl or C₁₋₃ alkyl, such as methyl, ethyl, propyl, isopropyl, butyl, sec-butyl or tert-butyl);
the cycloalkylene (e.g., C₃₋₂₀ cycloalkylene), the arylene (e.g., C₃₋₂₀ arylene), the heterocyclylene (e.g., 4- to 20-membered heterocyclylene) and the heteroarylene (e.g., 4- to 20-membered heteroarylene) are independently optionally substituted with a substituent selected from the group consisting of deuterium, optionally deuterated C₁₋₆ alkyl (e.g., optionally deuterated C₁₋₄ alkyl, such as methyl, CD₃, ethyl, propyl, isopropyl, butyl, sec-butyl, or tert-butyl), optionally deuterated C₃₋₆ cycloalkyl (e.g., optionally deuterated cyclopropyl, cyclobutyl, cyclopentyl, or cyclohexyl), hydroxy, amino, mercapto, halogen (e.g., fluorine, chlorine, bromine, or iodine), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), optionally deuterated C₁₋₆ alkyl-NH-(e.g., optionally deuterated C₁₋₃ alkyl-NH-, such as CH₃NH-, CH₃CH₂NH- or CH₃CH₂CH₂NH-), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), NH₂-C₁₋₆ alkylene (e.g., NH₂-C₁₋₃ alkylene-, such as NH₂CH₂-, NH₂CH₂CH₂-and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH-(e.g., optionally deuterated C₁₋₄ alkyl-C(O)NH-, such as CH₃-C(O)NH-, CH₃CH₂-C(O)NH-, and CH₃CH₂CH₂-C(O)NH-), cyano or any combination thereof;
R^{a1}, R^{a2}, R^{a3}, R^{a4}, R^{a5}, R^{a6}, R^{a7} and R^{a8} independently represent H, deuterium, hydroxy, amino, mercapto, nitro, halogen (e.g., fluorine, chlorine, bromine, or iodine), cyano, optionally deuterated C₁₋₆ alkyl (e.g., optionally deuterated C₁₋₄ alkyl, such as methyl, CD₃, ethyl, propyl, isopropyl, butyl, sec-butyl, or tert-butyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally deuterated cyclopropyl, cyclobutyl, cyclopentyl, or cyclohexyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), optionally deuterated C₁₋₆ alkyl-NH-(e.g., optionally deuterated C₁₋₃ alkyl-NH-, such as CH₃NH-, CH₃CH₂NH- or CH₃CH₂CH₂NH-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)- (e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH-(e.g., optionally deuterated C₁₋₄ alkyl-C(O)NH-, such as CH₃-C(O)NH-, CH₃CH₂-C(O)NH-, and CH₃CH₂CH₂-C(O)NH-) or any combination thereof; and
n1, n2, n3, n4, m4, m5, m6 are independently selected from the group consisting of an integer of 1, 2, 3, 4, 5, 6, 7, 8, 9, 10, 11, 12, 13, 14, or 15.

In some embodiments, R₅ and R₆ of the compounds of Formula (II) are the same or different and each independently represent the following groups:
H, fluorine, chlorine, bromine, iodine, -CH₃, -CH₂-CH₃, -(CH₂)₂-CH₃, -(CH₂)₃-CH₃, -(CH₂)₄-CH₃, -(CH₂)₅-CH₃, -(CH₂)₆-CH₃, -(CH₂)₇-CH₃, -(CH₂)₈-CH₃, -(CH₂)₉-CH₃, -(CH₂)₁₀-CH₃, -(CH₂)₁₁-CH₃, - (CH₂)₁₂-CH₃, -(CH₂)₁₃-CH₃, -(CH₂)₁₄-CH₃, -(CH₂)₁₅-CH₃, -(CH₂)₁₆-CH₃, -(CH₂)₁₇-CH₃, -(CH₂)₁₈-CH₃, -(CH₂)₁₉-CH₃, -(CH₂)₂₀-CH₃, -(CH₂)₂₁-CH₃, -(CH₂)₂₂-CH₃, -(CH₂)₂₅-CH₃, -(CH₂)₂₉-CH₃;-CH₂-O-CH₃, -CH₂-O-CH₂-CH₃, -CH₂-O-(CH₂)₂-CH₃, -(CH₂)₁-O-(CH₂)₃-CH₃, -(CH₂)₁-O-(CH₂)₄-CH₃, -(CH₂)₁-O-(CH₂)₅-CH₃, -(CH₂)₁-O-(CH₂)₆-CH₃, -(CH₂)₁-O-(CH₂)₇-CH₃, -(CH₂)₁-O-(CH₂)₈-CH₃, -(CH₂)₁-O-(CH₂)₉-CH₃, -(CH₂)₁-O-(CH₂)₁₀-CH₃, -(CH₂)₂-O-CH₃, -(CH₂)₂-O-CH₂-CH₃, -(CH₂)₂-O-(CH₂)₂-CH₃, - (CH₂)₂-O-(CH₂)₃-CH₃, -(CH₂)₂-O-(CH₂)₄-CH₃, -(CH₂)₂-O-(CH₂)₅-CH₃, -(CH₂)₂-O-(CH₂)₆-CH₃, - (CH₂)₂-O-(CH₂)₇-CH₃, -(CH₂)₂-O-(CH₂)₈-CH₃, -(CH₂)₂-O-(CH₂)₉-CH₃, -(CH₂)₂-O-(CH₂)₁₀-CH₃, - (CH₂)₂-O-(CH₂)₁₁-CH₃, -(CH₂)₂-O-(CH₂)₁₂-CH₃, -(CH₂)₃-O-CH₃, -(CH₂)₃-O-CH₂-CH₃, -(CH₂)₃-O-(CH₂)₂-CH₃, -(CH₂)₃-O-(CH₂)₃-CH₃, -(CH₂)₃-O-(CH₂)₄-CH₃, -(CH₂)₃-O-(CH₂)₅-CH₃, -(CH₂)₃-O-(CH₂)₆-CH₃, -(CH₂)₃-O-(CH₂)₇-CH₃, -(CH₂)₄-O-CH₃, -(CH₂)₄-O-CH₂-CH₃, -(CH₂)₄-O-(CH₂)₂-CH₃, - (CH₂)₄-O-(CH₂)₃-CH₃, -(CH₂)₄-O-(CH₂)₄-CH₃, -(CH₂)₄-O-(CH₂)₅-CH₃, -(CH₂)₄-O-(CH₂)₆-CH₃, - (CH₂)₅-O-CH₃, -(CH₂)₅-O-CH₂-CH₃, -(CH₂)₅-O-(CH₂)₂-CH₃, -(CH₂)₅-O-(CH₂)₃-CH₃, -(CH₂)₅-O-(CH₂)₄-CH₃, -(CH₂)₅-O-(CH₂)₅-CH₃, -(CH₂)₆-O-CH₃, -(CH₂)₆-O-CH₂-CH₃, -(CH₂)₆-O-(CH₂)₂-CH₃, - (CH₂)₆-O-(CH₂)₃-CH₃, -(CH₂)₆-O-(CH₂)₄-CH₃, -(CH₂)₇-O-CH₃, -(CH₂)₇-O-CH₂-CH₃, -(CH₂)₇-O-(CH₂)₂-CH₃, -(CH₂)₇-O-(CH₂)₃-CH₃, -(CH₂)₈-O-CH₃, -(CH₂)₈-O-CH₂-CH₃, -(CH₂)₈-O-(CH₂)₂-CH₃, - CH(CH₃)-O-CH₃, -CH(CH₃)-O-CH₂-CH₃, -CH(CH₃)-O-(CH₂)₂-CH₃, -CH(CH₃)-O-(CH₂)₃-CH₃, - CH(CH₃)-O-(CH₂)₄-CH₃, -CH(CH₃)-O-(CH₂)₅-CH₃, -CH(CH₃)-O-(CH₂)₆-CH₃, -CH(CH₃)-O-(CH₂)₇-CH₃, -CH(CH₃)-O-(CH₂)₈-CH₃, -CH(CH₃)-O-(CH₂)₉-CH₃, -CH(CH₃)-O-(CH₂)₁₀-CH₃, -CH₂-(O(CH₂)₂)₁-OCH₂CH₃, -CH₂-(O(CH₂)₂)₂-OCH₂CH₃, -CH₂-(O(CH₂)₂)₃-OCH₂CH₃, -CH₂-(O(CH₂)₂)₄-OCH₂CH₃, -CH₂-(O(CH₂)₂)₅-OCH₂CH₃, -CH₂-(O(CH₂)₂)₆-OCH₂CH₃, -CH₂-(O(CH₂)₂)₇-OCH₂CH₃, - CH₂-(O(CH₂)₂)₈-OCH₂CH₃, -CH₂-(O(CH₂)₂)₉-OCH₂CH₃, -CH₂-(O(CH₂)₂)₁₀-OCH₂CH₃, -CH₂-(O(CH₂)₂)₁-OCH₃, -CH₂-(O(CH₂)₂)₂-OCH₃, -CH₂-(O(CH₂)₂)₃-OCH₃, -CH₂-(O(CH₂)₂)₄-OCH₃, -CH₂-(O(CH₂)₂)₅-OCH₃, -CH₂-(O(CH₂)₂)₆-OCH₃, -CH₂-(O(CH₂)₂)₇-OCH₃, -CH₂-(O(CH₂)₂)₈-OCH₃, -CH₂-(O(CH₂)₂)₉-OCH₃, -CH₂-(O(CH₂)₂)₁₀-OCH₃, -(CH₂)₂-(O(CH₂)₂)₁-OCH₂CH₃, -(CH₂)₂-(O(CH₂)₂)₂-OCH₂CH₃, -(CH₂)₂-(O(CH₂)₂)₃-OCH₂CH₃, -(CH₂)₂-(O(CH₂)₂)₄-OCH₂CH₃, -(CH₂)₂-(O(CH₂)₂)₅-OCH₂CH₃, -(CH₂)₂-(O(CH₂)₂)₆-OCH₂CH₃, -(CH₂)₂-(O(CH₂)₂)₇-OCH₂CH₃, -(CH₂)₂-(O(CH₂)₂)₈-OCH₂CH₃, -(CH₂)₂-(O(CH₂)₂)₉-OCH₂CH₃, -(CH₂)₂-(O(CH₂)₂)₁₀-OCH₂CH₃, -(CH₂)₃-(O(CH₂)₂)₁-OCH₂CH₃, -(CH₂)₃-(O(CH₂)₂)₂-OCH₂CH₃, -(CH₂)₃-(O(CH₂)₂)₃-OCH₂CH₃, -(CH₂)₃-(O(CH₂)₂)₄-OCH₂CH₃, -(CH₂)₃-(O(CH₂)₂)₅-OCH₂CH₃, -(CH₂)₃-(O(CH₂)₂)₆-OCH₂CH₃, -(CH₂)₃-(O(CH₂)₂)₇-OCH₂CH₃, -(CH₂)₃-(O(CH₂)₂)₈-OCH₂CH₃, -(CH₂)₃-(O(CH₂)₂)₉-OCH₂CH₃, -(CH₂)₃-(O(CH₂)₂)₁₀-OCH₂CH₃, -(CH₂)₄-(O(CH₂)₂)₁-OCH₂CH₃, -(CH₂)₄-(O(CH₂)₂)₂-OCH₂CH₃, -(CH₂)₄-(O(CH₂)₂)₃-OCH₂CH₃, -(CH₂)₄-(O(CH₂)₂)₄-OCH₂CH₃, -(CH₂)₄-(O(CH₂)₂)₅-OCH₂CH₃, -(CH₂)₄-(O(CH₂)₂)₆-OCH₂CH₃, -(CH₂)₄-(O(CH₂)₂)₇-OCH₂CH₃, -(CH₂)₄-(O(CH₂)₂)₈-OCH₂CH₃, -(CH₂)₄-(O(CH₂)₂)₉-OCH₂CH₃, -(CH₂)₄-(O(CH₂)₂)₁₀-OCH₂CH₃, -CH₂-(O(CH₂)₃)₁-OCH₂CH₂CH₃, -CH₂-(O(CH₂)₃)₂-OCH₂CH₂CH₃, -CH₂-(O(CH₂)₃)₃-OCH₂CH₂CH₃, -CH₂-(O(CH₂)₃)₄-OCH₂CH₂CH₃, -CH₂-(O(CH₂)₃)₅-OCH₂CH₂CH₃, -CH₂-(O(CH₂)₃)₆-OCH₂CH₂CH₃, -CH₂-(O(CH₂)₃)₇-OCH₂CH₂CH₃, -CH₂-(O(CH₂)₃)₈-OCH₂CH₂CH₃, -CH₂-(O(CH₂)₃)₉-OCH₂CH₂CH₃, -CH₂-(O(CH₂)₃)₁₀-OCH₂CH₂CH₃, -(CH₂)₂-(O(CH₂)₃)₁-OCH₂CH₂CH₃, -(CH₂)₂-(O(CH₂)₃)₂-OCH₂CH₂CH₃, -(CH₂)₂-(O(CH₂)₃)₃-OCH₂CH₂CH₃, - (CH₂)₂-(O(CH₂)₃)₄-OCH₂CH₂CH₃, -(CH₂)₂-(O(CH₂)₃)₅-OCH₂CH₂CH₃, -(CH₂)₂-(O(CH₂)₃)₆-OCH₂CH₂CH₃, -(CH₂)₂-(O(CH₂)₃)₇-OCH₂CH₂CH₃, -(CH₂)₂-(O(CH₂)₃)₈-OCH₂CH₂CH₃, -(CH₂)₃-(O(CH₂)₃)₁-OCH₂CH₂CH₃, -(CH₂)₃-(O(CH₂)₃)₂-OCH₂CH₂CH₃, -(CH₂)₃-(O(CH₂)₃)₃-OCH₂CH₂CH₃, - (CH₂)₃-(O(CH₂)₃)₄-OCH₂CH₂CH₃, -(CH₂)₃-(O(CH₂)₃)₅-OCH₂CH₂CH₃, -(CH₂)₃-(O(CH₂)₃)₆-OCH₂CH₂CH₃, -(CH₂)₃-(O(CH₂)₃)₇-OCH₂CH₂CH₃, -(CH₂)₃-(O(CH₂)₃)₈-OCH₂CH₂CH₃, -CH₂-O-(CH₂)₂-O-(CH₂)₂-CH₃, -CH₂-(O(CH₂)₂)₂-(O(CH₂)₃)₁-OCH₂CH₂CH₃, -CH₂-(O(CH₂)₂)₃-(O(CH₂)₃)₂-OCH₂CH₂CH₃, -CH₂-(O(CH₂)₂)₄-(O(CH₂)₃)₃-OCH₂CH₂CH₃, -CH₂-(O(CH₂)₂)₅-(O(CH₂)₃)₄-OCH₂CH₂CH₃, -(CH₂)₂-O-(CH₂)₂-O-(CH₂)₂CH₃, -(CH₂)₂-(O(CH₂)₂)₂-(O(CH₂)₃)₁-OCH₂CH₂CH₃, - (CH₂)₂-(O(CH₂)₂)₃-(O(CH₂)₃)₂-OCH₂CH₂CH₃, -(CH₂)₂-(O(CH₂)₂)₄-(O(CH₂)₃)₃-OCH₂CH₂CH₃, - (CH₂)₂-(O(CH₂)₂)₅-(O(CH₂)₃)₄-OCH₂CH₂CH₃, -(CH₂)₃-O-(CH₂)₂-O-(CH₂)₂CH₃, -(CH₂)₃-(O(CH₂)₂)₂-(O(CH₂)₃)₁-OCH₂CH₂CH₃, -(CH₂)₃-(O(CH₂)₂)₃-(O(CH₂)₃)₂-OCH₂CH₂CH₃, -(CH₂)₃-(O(CH₂)₂)₄-(O(CH₂)₃)₃-OCH₂CH₂CH₃, -(CH₂)₃-(O(CH₂)₂)₅-(O(CH₂)₃)₄-OCH₂CH₂CH₃, -CH₂-O-(CH₂)₃-O-CH₂CH₃, -CH₂-(O(CH₂)₃)₂-(O(CH₂)₂)₁-O-CH₂CH₃, -CH₂-(O(CH₂)₃)₃-(O(CH₂)₂)₂-O-CH₂CH₃, -CH₂-(O(CH₂)₃)₄-(O(CH₂)₂)₃-O-CH₂CH₃, -CH₂-(O(CH₂)₃)₅-(O(CH₂)₂)₄-O-CH₂CH₃, -(CH₂)₂-O-(CH₂)₃-O-CH₂CH₃, -(CH₂)₂-(O(CH₂)₃)₂-(O(CH₂)₂)₁-O-CH₂CH₃, -(CH₂)₂-(O(CH₂)₃)₃-(O(CH₂)₂)₂-O-CH₂CH₃, - (CH₂)₂-(O(CH₂)₃)₄-(O(CH₂)₂)₃-O-CH₂CH₃, -(CH₂)₂-(O(CH₂)₃)₅-(O(CH₂)₂)₄-O-CH₂CH₃, -(CH₂)₃-O-(CH₂)₃-O-CH₂CH₃, -(CH₂)₃-(O(CH₂)₃)₂-(O(CH₂)₂)₁-O-CH₂CH₃, -(CH₂)₃-(O(CH₂)₃)₃-(O(CH₂)₂)₂-O-CH₂CH₃, -(CH₂)₃-(O(CH₂)₃)₄-(O(CH₂)₂)₃-O-CH₂CH₃, -(CH₂)₃-(O(CH₂)₃)₅-(O(CH₂)₂)₄-O-CH₂CH₃, - CH₂-O-(CH₂)₂-O-CH₃, -(CH₂)₂-O-(CH₂)₂-O-CH₃, -(CH₂)₂-(O(CH₂)₂)₂-O-(CH₂)₂CH₃, -(CH₂)₂-(O(CH₂)₂)₃-O-(CH₂)₂CH₃, -(CH₂)₂-(O(CH₂)₂)₄-O-(CH₂)₂CH₃, -(CH₂)₅-(O(CH₂)₂)₂-O-(CH₂)₄CH₃, - (CH₂)₅-(O(CH₂)₂)₂-O-(CH₂)₅CH₃, -(CH₂)₁-N(Rₑ)-CH₃, -(CH₂)₁-N(Rₑ)-(CH₂)₁-CH₃, -(CH₂)₁-N(Rₑ)-(CH₂)₂-CH₃, -(CH₂)₁-N(Rₑ)-(CH₂)₃-CH₃, -(CH₂)₁-N(Rₑ)-(CH₂)₄-CH₃, -(CH₂)₁-N(Rₑ)-(CH₂)₅-CH₃, - (CH₂)₁-N(Rₑ)-(CH₂)₆-CH₃, -(CH₂)₁-N(Rₑ)-(CH₂)₇-CH₃, -(CH₂)₁-N(Rₑ)-(CH₂)₈-CH₃, -(CH₂)₁-N(Rₑ)-(CH₂)₉-CH₃, -(CH₂)₂-N(Rₑ)-CH₃, -(CH₂)₂-N(Rₑ)-(CH₂)₁-CH₃, -(CH₂)₂-N(Rₑ)-(CH₂)₂-CH₃, -(CH₂)₂-N(Rₑ)-(CH₂)₃-CH₃, -(CH₂)₂-N(Rₑ)-(CH₂)₄-CH₃, -(CH₂)₂-N(Rₑ)-(CH₂)₅-CH₃, -(CH₂)₂-N(Rₑ)-(CH₂)₆-CH₃, -(CH₂)₂-N(Rₑ)-(CH₂)₇-CH₃, -(CH₂)₂-N(Rₑ)-(CH₂)₈-CH₃, -(CH₂)₂-N(Rₑ)-(CH₂)₉-CH₃, -(CH₂)₂-N(Rₑ)-(CH₂)₁₀-CH₃, -(CH₂)₂-N(Rₑ)-(CH₂)₁₁-CH₃, -(CH₂)₃-N(Rₑ)-CH₃, -(CH₂)₃-N(Rₑ)-(CH₂)₁-CH₃, - (CH₂)₃-N(Rₑ)-(CH₂)₂-CH₃, -(CH₂)₄-N(Rₑ)-CH₃, -(CH₂)₄-N(Rₑ)-(CH₂)₁-CH₃, -(CH₂)₄-N(Rₑ)-(CH₂)₂-CH₃, -(CH₂)₄-N(Rₑ)-(CH₂)₃-CH₃, -(CH₂)₅-N(Rₑ)-CH₃, -(CH₂)₅-N(Rₑ)-(CH₂)₁-CH₃, -(CH₂)₅-N(Rₑ)-(CH₂)₂-CH₃, -(CH₂)₅-N(Rₑ)-(CH₂)₃-CH₃, -(CH₂)₅-N(Rₑ)-(CH₂)₄-CH₃, -(CH₂)₆-N(Rₑ)-CH₃, -(CH₂)₆-N(Rₑ)-(CH₂)₁-CH₃, -(CH₂)₆-N(Rₑ)-(CH₂)₂-CH₃, -(CH₂)₇-N(Rₑ)-CH₃, -(CH₂)₇-N(Rₑ)-(CH₂)₁-CH₃, - (CH₂)₇-N(Rₑ)-(CH₂)₂-CH₃, -(CH₂)₈-N(Rₑ)-CH₃, -(CH₂)₈-N(Rₑ)-(CH₂)₁-CH₃, -(CH₂)₈-N(Rₑ)-(CH₂)₂-CH₃, -CH(CH₃)-N(Rₑ)-CH₃, -CH(CH₃)-N(Rₑ)-(CH₂)₁-CH₃, -CH(CH₃)-N(Rₑ)-(CH₂)₂-CH₃, -CH(CH₃)-N(Rₑ)-(CH₂)₃-CH₃, -CH(CH₃)-N(Rₑ)-(CH₂)₄-CH₃, -CH(CH₃)-N(Rₑ)-(CH₂)₅-CH₃, -CH(CH₃)-N(Rₑ)-(CH₂)₆-CH₃, -CH(CH₃)-N(Rₑ)-(CH₂)₇-CH₃, -CH(CH₃)-N(Rₑ)-(CH₂)-CH₃, -CH(CH₃)-N(Rₑ)-(CH₂)₉-CH₃, -CH₂C(O)NHCH₃, -(CH₂)₂C(O)NHCH₂CH₃, -(CH₂)₂C(O)NH(CH₂)₂-CH₃, - (CH₂)₂C(O)NH(CH₂)₃-CH₃, -(CH₂)₂C(O)NH(CH₂)₄-CH₃, -(CH₂)₃C(O)NH(CH₂)₂-CH₃, - (CH₂)₃C(O)NH(CH₂)₃-CH₃, -(CH₂)₄C(O)NH(CH₂)₃-CH₃, -(CH₂)₅C(O)NH(CH₂)₄-CH₃, - (CH₂)₆C(O)NH(CH₂)₆-CH₃, -(CH₂)₆C(O)NH(CH₂)₅-CH₃, -(CH₂)₇C(O)NH(CH₂)₆-CH₃, - (CH₂)₈C(O)NH(CH₂)₇-CH₃, U-(CH₂)₉C(O)NH(CH₂)₈-CH₃, -(CH₂)₁₀C(O)NH(CH₂)₉-CH₃, - (CH₂)₂C(O)NH(CH₂)₂-O-CH₂-CH₃, -CH₂NHC(O)CH₃, -(CH₂)₂NHC(O)CH₂CH₃, - (CH₂)₂NHC(O)(CH₂)₂-CH₃, -(CH₂)₂NHC(O)(CH₂)₃-CH₃, -(CH₂)₂NHC(O)(CH₂)₄-CH₃, - (CH₂)₃NHC(O)(CH₂)₂-CH₃, -(CH₂)₃NHC(O)(CH₂)₃-CH₃, -(CH₂)₄NHC(O)(CH₂)₃-CH₃, - (CH₂)₅NHC(O)(CH₂)₄-CH₃, -(CH₂)₆NHC(O)(CH₂)₆-CH₃, -(CH₂)₆NHC(O)(CH₂)₅-CH₃, - (CH₂)₇NHC(O)(CH₂)₆-CH₃, -(CH₂)₈NHC(O)(CH₂)₇-CH₃, -(CH₂)₉NHC(O)(CH₂)₈-CH₃, - (CH₂)₁₀NHC(O)(CH₂)₉-CH₃, -(CH₂)₄NHC(O)(CH₂)₇-CH₃, -(CH₂)₂NHC(O)(CH₂)₂-O-CH₂-CH₃, - (CH₂)₄NHC(O)CH₃, -CH₂-piperidinylene-CH₃, -CH₂-piperidinylene-CH₂-CH₃, -CH₂-piperidinylene-(CH₂)₂-CH₃, -CH₂-piperidinylene-(CH₂)₃-CH₃, -CH₂-piperidinylene-(CH₂)₄-CH₃, -CH₂-piperidinylene-(CH₂)₅-CH₃, -CH₂-piperidinylene-(CH₂)₆-CH₃, -CH₂-piperidinylene-(CH₂)₇-CH₃, - (CH₂)₂-piperidinylene-CH₃, -(CH₂)₂-piperidinylene-CH₂-CH₃, -(CH₂)₂-piperidinylene-(CH₂)₂-CH₃, - (CH₂)₂-piperidinylene-(CH₂)₃-CH₃, -(CH₂)₂-piperidinylene-(CH₂)₄-CH₃, -(CH₂)₂-piperidinylene-(CH₂)₅-CH₃, -(CH₂)₂-piperidinylene-(CH₂)₆-CH₃, -(CH₂)₂-piperidinylene-(CH₂)₇-CH₃, -(CH₂)₃-piperidinylene-CH₃, -(CH₂)₃-piperidinylene-CH₂-CH₃, -(CH₂)₃-piperidinylene-(CH₂)₂-CH₃, -(CH₂)₃-piperidinylene-(CH₂)₃-CH₃, -(CH₂)₃-piperidinylene-(CH₂)₄-CH₃, -(CH₂)₃-piperidinylene-(CH₂)₅-CH₃, -(CH₂)₃-piperidinylene-(CH₂)₆-CH₃, -(CH₂)₃-piperidinylene-(CH₂)₇-CH₃, -(CH₂)₄-piperidinylene-CH₃, -(CH₂)₄-piperidinylene-CH₂-CH₃, -(CH₂)₄-piperidinylene-(CH₂)₂-CH₃, -(CH₂)₄-piperidinylene-(CH₂)₃-CH₃, -(CH₂)₄-piperidinylene-(CH₂)₄-CH₃, -(CH₂)₄-piperidinylene-(CH₂)₅-CH₃, -(CH₂)₄-piperidinylene-(CH₂)₆-CH₃, -(CH₂)₄-piperidinylene-(CH₂)₇-CH₃, -(CH₂)₅-piperidinylene-CH₃, - (CH₂)₅-piperidinylene-CH₂-CH₃, -(CH₂)₅-piperidinylene-(CH₂)₂-CH₃, -(CH₂)₅-piperidinylene-(CH₂)₃-CH₃, -(CH₂)₅-piperidinylene-(CH₂)₄-CH₃, -(CH₂)₅-piperidinylene-(CH₂)₅-CH₃, -(CH₂)₅-piperidinylene-(CH₂)₆-CH₃, -(CH₂)₅-piperidinylene-(CH₂)₇-CH₃, -(CH₂)₆-piperidinylene-CH₃, - (CH₂)₆-piperidinylene-CH₂-CH₃, -(CH₂)₆-piperidinylene-(CH₂)₂-CH₃, -(CH₂)₆-piperidinylene-(CH₂)₃-CH₃, -(CH₂)₆-piperidinylene-(CH₂)₄-CH₃, -(CH₂)₆-piperidinylene-(CH₂)₅-CH₃, -(CH₂)₆-piperidinylene-(CH₂)₆-CH₃, -(CH₂)₆-piperidinylene-(CH₂)₇-CH₃, -(CH₂)₇-piperidinylene-CH₃, - (CH₂)₇-piperidinylene-CH₂-CH₃, -(CH₂)₇-piperidinylene-(CH₂)₂-CH₃, -(CH₂)₇-piperidinylene-(CH₂)₃-CH₃, -(CH₂)₇-piperidinylene-(CH₂)₇-CH₃, -(CH₂)₈-piperidinylene-CH₃, -(CH₂)₈-piperidinylene-CH₂-CH₃, -(CH₂)₈-piperidinylene-(CH₂)₂-CH₃, -(CH₂)₈-piperidinylene-(CH₂)₃-CH₃, -(CH₂)₈-piperidinylene-(CH₂)₄-CH₃, -(CH₂)₈-piperidinylene-(CH₂)₅-CH₃, -(CH₂)₈-piperidinylene-(CH₂)₆-CH₃, -(CH₂)₈-piperidinylene-(CH₂)₇-CH₃, -CH₂-N(Rₑ)-CH₂-piperidinylene-CH₃, -CH₂-N(Rₑ)-CH₂-piperidinylene-CH₃, -CH₂-N(Rₑ)-CH₂-piperidinylene-(CH₂)₂-CH₃, -CH₂-N(Rₑ)-CH₂-piperidinylene-(CH₂)₃-CH₃, -CH₂-N(Rₑ)-CH₂-piperidinylene-(CH₂)₄-CH₃, -CH₂-N(Rₑ)-CH₂-piperidinylene-(CH₂)₅-CH₃, -CH₂-N(Rₑ)-CH₂-piperidinylene-(CH₂)₆-CH₃, -CH₂-N(Rₑ)-CH₂-piperidinylene-(CH₂)₇-CH₃, - CH₂-N(Rₑ)-(CH₂)₂-piperidinylene-CH₃, -CH₂-N(Rₑ)-(CH₂)₂-piperidinylene-CH₂-CH₃, -CH₂-N(Rₑ)-(CH₂)₂-piperidinylene-(CH₂)₂-CH₃, -CH₂-N(Rₑ)-(CH₂)₂-piperidinylene-(CH₂)₃-CH₃, -CH₂-N(Rₑ)-(CH₂)₂-piperidinylene-(CH₂)₄-CH₃, -CH₂-N(Rₑ)-(CH₂)₂-piperidinylene-(CH₂)₅-CH₃, -CH₂-N(Rₑ)-(CH₂)₂-piperidinylene-(CH₂)₆-CH₃, -CH₂-N(Rₑ)-(CH₂)₂-piperidinylene-(CH₂)₇-CH₃, -(CH₂)₂-N(Rₑ)-CH₂-piperidinylene-CH₃, -(CH₂)₂-N(Rₑ)-CH₂-piperidinylene-CH₂-CH₃, -(CH₂)₂-N(Rₑ)-CH₂-piperidinylene-(CH₂)₂-CH₃, -(CH₂)₂-N(Rₑ)-CH₂-piperidinylene-(CH₂)₃-CH₃, -(CH₂)₂-N(Rₑ)-CH₂-piperidinylene-(CH₂)₄-CH₃, -(CH₂)₂-N(Rₑ)-CH₂-piperidinylene-(CH₂)₅-CH₃, -(CH₂)₂-N(Rₑ)-CH₂-piperidinylene-(CH₂)₆-CH₃, -(CH₂)₂-N(Rₑ)-CH₂-piperidinylene-(CH₂)₇-CH₃, -(CH₂)₃-N(Rₑ)-CH₂-piperidinylene-CH₃, -(CH₂)₃-N(Rₑ)-CH₂-piperidinylene-CH₂-CH₃, -(CH₂)₃-N(Rₑ)-CH₂-piperidinylene-(CH₂)₂-CH₃, -(CH₂)₃-N(Rₑ)-CH₂-piperidinylene-(CH₂)₇-CH₃, -(CH₂)₄-N(Rₑ)-CH₂-piperidinylene-CH₃, -(CH₂)₄-N(Rₑ)-CH₂-piperidinylene-CH₂-CH₃, -(CH₂)₄-N(Rₑ)-CH₂-piperidinylene-(CH₂)₂-CH₃, - (CH₂)₄-N(Rₑ)-CH₂-piperidinylene-(CH₂)₇-CH₃, -(CH₂)₅-N(Rₑ)-CH₂-piperidinylene-(CH₂)₂-CH₃, - (CH₂)₅-N(Rₑ)-CH₂-piperidinylene-(CH₂)₇-CH₃, -(CH₂)₆-N(Rₑ)-CH₂-piperidinylene-(CH₂)₂-CH₃, - (CH₂)₆-N(Rₑ)-CH₂-piperidinylene-(CH₂)₇-CH₃, -(CH₂)₇-N(Rₑ)-CH₂-piperidinylene-(CH₂)₂-CH₃, - (CH₂)₇-N(Rₑ)-CH₂-piperidinylene-(CH₂)₇-CH₃, -(CH₂)₈-N(Rₑ)-CH₂-piperidinylene-CH₃, -(CH₂)₈-N(Rₑ)-CH₂-piperidinylene-CH₂-CH₃, -(CH₂)₈-N(Rₑ)-CH₂-piperidinylene-(CH₂)₂-CH₃, -(CH₂)₈-N(Rₑ)-CH₂-piperidinylene-(CH₂)₃-CH₃, -(CH₂)₈-N(Rₑ)-CH₂-piperidinylene-(CH₂)₄-CH₃, -(CH₂)₈-N(Rₑ)-CH₂-piperidinylene-(CH₂)₅-CH₃, -(CH₂)₈-N(Rₑ)-CH₂-piperidinylene-(CH₂)₆-CH₃, -(CH₂)₈-N(Rₑ)-CH₂-piperidinylene-(CH₂)₇-CH₃, -CH₂-piperazinylene-CH₃, -CH₂-piperazinylene-CH₂-CH₃, -CH₂-piperazinylene-(CH₂)₂-CH₃, -CH₂-piperazinylene-(CH₂)₃-CH₃, -CH₂-piperazinylene-(CH₂)₄-CH₃, - CH₂-piperazinylene-(CH₂)₅-CH₃, -CH₂-piperazinylene-(CH₂)₆-CH₃, -CH₂-piperazinylene-(CH₂)₇-CH₃, -(CH₂)₂-piperazinylene-CH₃, -(CH₂)₂-piperazinylene-CH₂-CH₃, -(CH₂)₂-piperazinylene-(CH₂)₂-CH₃, - (CH₂)₂-piperazinylene-(CH₂)₃-CH₃, -(CH₂)₂-piperazinylene-(CH₂)₄-CH₃, -(CH₂)₂-piperazinylene-(CH₂)₅-CH₃, -(CH₂)₂-piperazinylene-(CH₂)₆-CH₃, -(CH₂)₂-piperazinylene-(CH₂)₇-CH₃, -(CH₂)₃-piperazinylene-CH₃, -(CH₂)₃-piperazinylene-CH₂-CH₃, -(CH₂)₃-piperazinylene-(CH₂)₂-CH₃, -(CH₂)₃-piperazinylene-(CH₂)₃-CH₃, -(CH₂)₃-piperazinylene-(CH₂)₄-CH₃, -(CH₂)₃-piperazinylene-(CH₂)₅-CH₃, -(CH₂)₃-piperazinylene-(CH₂)₆-CH₃, -(CH₂)₃-piperazinylene-(CH₂)₇-CH₃, -(CH₂)₄-piperazinylene-CH₃, -(CH₂)₄-piperazinylene-CH₂-CH₃, -(CH₂)₄-piperazinylene-(CH₂)₂-CH₃, -(CH₂)₄-piperazinylene-(CH₂)₃-CH₃, -(CH₂)₄-piperazinylene-(CH₂)₄-CH₃, -(CH₂)₄-piperazinylene-(CH₂)₅-CH₃, -(CH₂)₄-piperazinylene-(CH₂)₆-CH₃, -(CH₂)₄-piperazinylene-(CH₂)₇-CH₃, -(CH₂)₅-piperazinylene-CH₃, - (CH₂)₅-piperazinylene-CH₂-CH₃, -(CH₂)₅-piperazinylene-(CH₂)₂-CH₃, -(CH₂)₅-piperazinylene-(CH₂)₃-CH₃, -(CH₂)₅-piperazinylene-(CH₂)₄-CH₃, -(CH₂)₅-piperazinylene-(CH₂)₅-CH₃, -(CH₂)₅-piperazinylene-(CH₂)₆-CH₃, -(CH₂)₅-piperazinylene-(CH₂)₇-CH₃, -(CH₂)₆-piperazinylene-CH₃, - (CH₂)₆-piperazinylene-CH₂-CH₃, -(CH₂)₆-piperazinylene-(CH₂)₂-CH₃, -(CH₂)₆-piperazinylene-(CH₂)₃-CH₃, -(CH₂)₆-piperazinylene-(CH₂)₄-CH₃, -(CH₂)₆-piperazinylene-(CH₂)₅-CH₃, -(CH₂)₆-piperazinylene-(CH₂)₆-CH₃, -(CH₂)₆-piperazinylene-(CH₂)₇-CH₃, -(CH₂)₇-piperazinylene-CH₃, - (CH₂)₇-piperazinylene-CH₂-CH₃, -(CH₂)₇-piperazinylene-(CH₂)₂-CH₃, -(CH₂)₇-piperazinylene-(CH₂)₃-CH₃, -(CH₂)₇-piperazinylene-(CH₂)₇-CH₃, -(CH₂)₈-piperazinylene-CH₃, -(CH₂)₈-piperazinylene-CH₂-CH₃, -(CH₂)₈-piperazinylene-(CH₂)₂-CH₃, -(CH₂)₈-piperazinylene-(CH₂)₃-CH₃, -(CH₂)₈-piperazinylene-(CH₂)₄-CH₃, -(CH₂)₈-piperazinylene-(CH₂)₅-CH₃, -(CH₂)₈-piperazinylene-(CH₂)₆-CH₃, or -(CH₂)₈-piperazinylene-(CH₂)₇-CH₃;
wherein the piperidinylene and the piperazinylene are independently optionally substituted with a substituent selected from the group consisting of deuterium, optionally deuterated C₁₋₆ alkyl (e.g., optionally deuterated C₁₋₄ alkyl, such as methyl, CD₃, ethyl, propyl, isopropyl, butyl, sec-butyl, or tert-butyl), optionally deuterated C₃₋₆ cycloalkyl (e.g., optionally deuterated cyclopropyl, cyclobutyl, cyclopentyl, or cyclohexyl), hydroxy, amino, mercapto, halogen (e.g., fluorine, chlorine, bromine, or iodine), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), optionally deuterated C₁₋₆ alkyl-NH-(e.g., optionally deuterated C₁₋₃ alkyl-NH-, such as CH₃NH-, CH₃CH₂NH- or CH₃CH₂CH₂NH-), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)-(e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH-(e.g., optionally deuterated C₁₋₄ alkyl-C(O)NH-, such as CH₃-C(O)NH-, CH₃CH₂-C(O)NH-, and CH₃CH₂CH₂-C(O)NH-), cyano or any combination thereof;
a hydrogen of CH₃ in the groups and a hydrogen of one or more CH₂ in the groups are optionally substituted with a substituent selected from the group consisting of: deuterium, hydroxy, amino, mercapto, nitro, halogen, cyano, optionally deuterated C₁₋₆ alkyl (e.g., optionally deuterated C₁₋₄ alkyl, such as methyl, CD₃, ethyl, propyl, isopropyl, butyl, sec-butyl, or tert-butyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally deuterated cyclopropyl, cyclobutyl, cyclopentyl, or cyclohexyl), optionally deuterated C₁₋₆ alkoxy (e.g., optionally deuterated C₁₋₄ alkoxy, such as methoxy, CD₃-O-, ethoxy, propoxy, isopropoxy, butoxy, sec-butoxy, or tert-butoxy), optionally deuterated C₁₋₆ alkyl-NH-(e.g., optionally deuterated C₁₋₃ alkyl-NH-, such as CH₃NH-, CH₃CH₂NH- or CH₃CH₂CH₂NH-), NH₂-C₁₋₆ alkylene (e.g., NH₂-C₁₋₃ alkylene-, such as NH₂CH₂-, NH₂CH₂CH₂- and NH₂CH₂CH₂CH₂-), optionally deuterated C₁₋₆ alkyl-NHC(O)-(e.g., optionally deuterated C₁₋₄ alkyl-NHC(O)-, such as CH₃-NHC(O)-, CH₃CH₂-NHC(O)-, and CH₃CH₂CH₂-NHC(O)-), optionally deuterated C₁₋₆ alkyl-C(O)NH-(e.g., optionally deuterated C₁₋₄ alkyl-C(O)NH-, such as CH₃-C(O)NH-, CH₃CH₂-C(O)NH-, and CH₃CH₂CH₂-C(O)NH-), or any combination thereof;
each Rₑ independently represents H or C₁₋₆ alkyl (e.g., C₁₋₄ alkyl or C₁₋₃ alkyl, such as methyl, ethyl, propyl, isopropyl, butyl, sec-butyl or tert-butyl); and
n1, n2, n3, n4, m4, m5, m6 are independently selected from the group consisting of an integer of 1, 2, 3, 4, 5, 6, 7, 8, 9, 10, 11, 12, 13, 14, or 15.

In some embodiments, the -R_{f}-LIN- represents the following groups:
-CH₂-, -O-CH₂-**, -NHC(O)-CH₂-**, -C(O)NH-CH₂-**, -NH-CH₂-**, -N(CH₃)-CH₂-**, - N(CH₃)-(CH₂)₂-N(CH₃)-CH₂-**, -N(CH₃)-(CH₂)₃-N(CH₃)-CH₂-**, -NH-(CH₂)₂-N(CH₃)-CH₂-**, - N(CH₃)-(CH₂)₃-NH-CH₂-**, -CH₂-NHC(O)-CH₂-**, -CH₂-C(O)NH-CH₂-**, -CH₂-NH-CH₂-**, -O-(CH₂)₂-N(CH₃)-CH₂-**, -O-(CH₂)₂-NH-CH₂-**, or -CH₂-N(CH₃) -CH₂-**, or 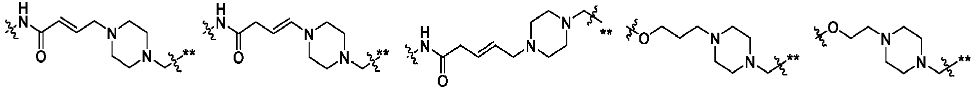 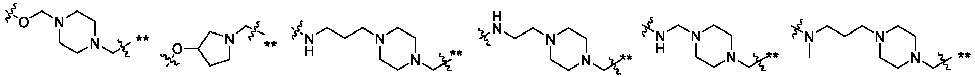 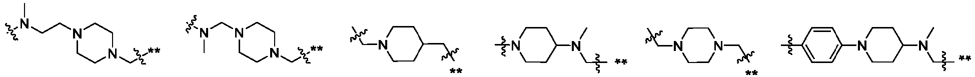 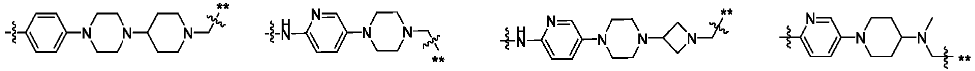 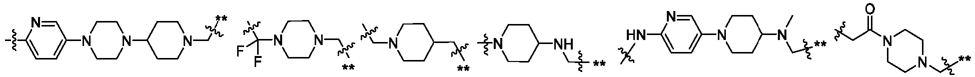 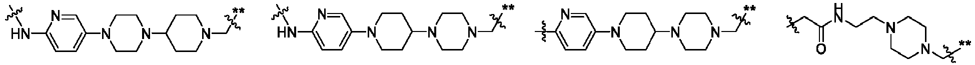 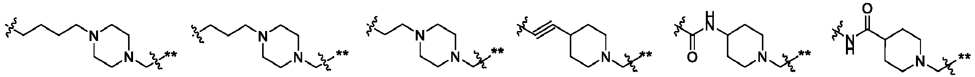 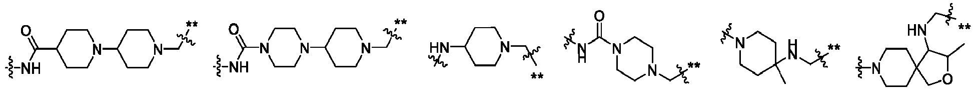 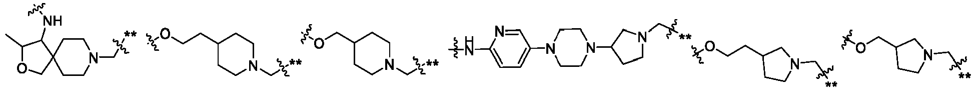 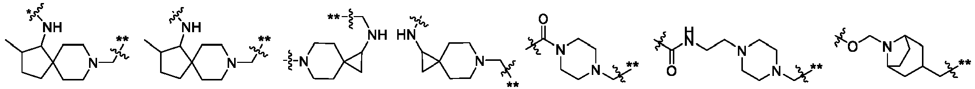 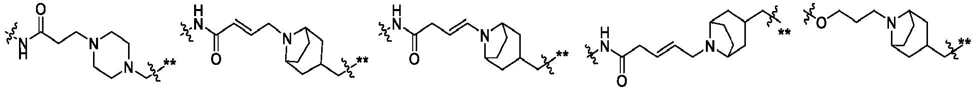 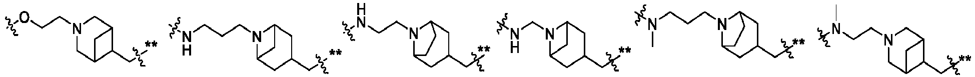 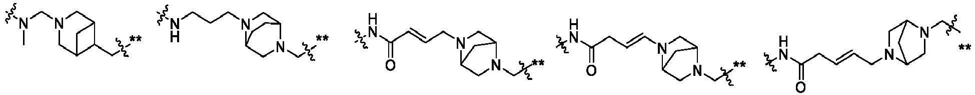 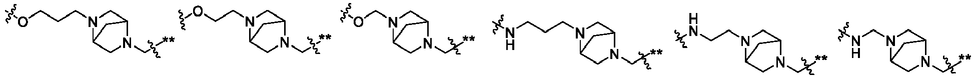 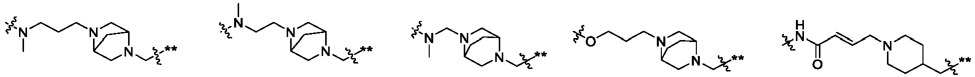 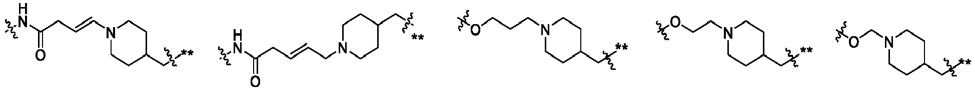 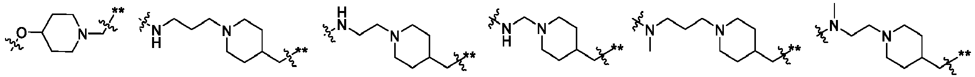 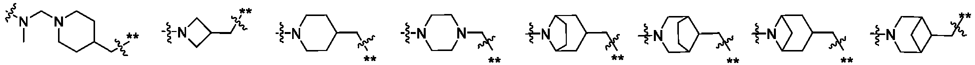 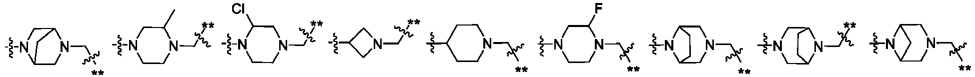 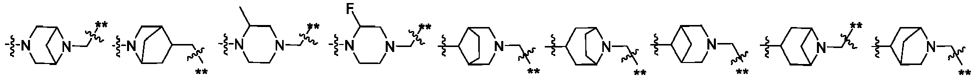 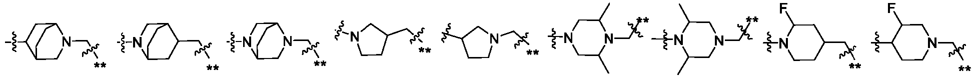 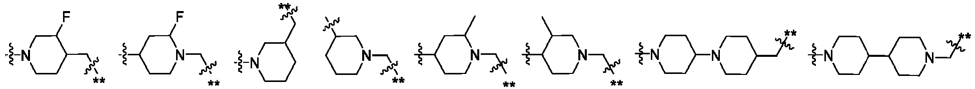 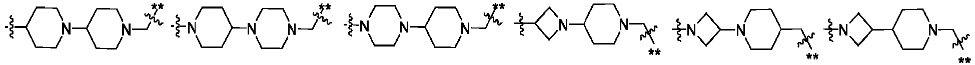 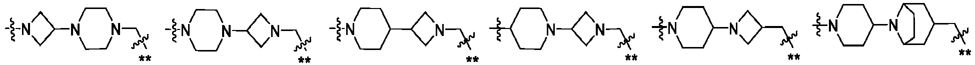 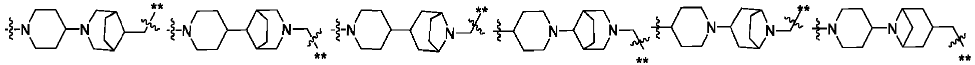 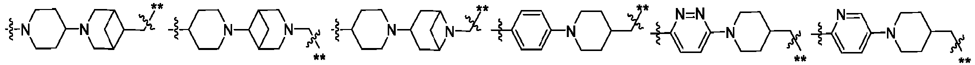 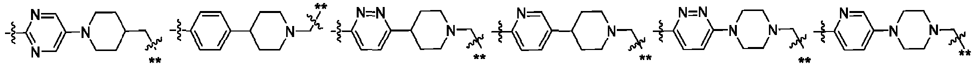 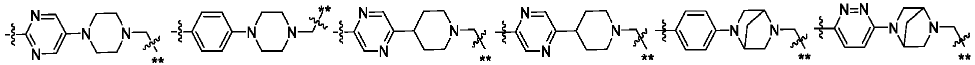 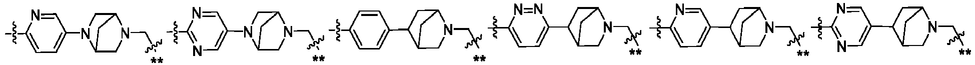 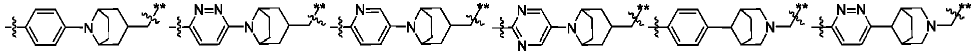 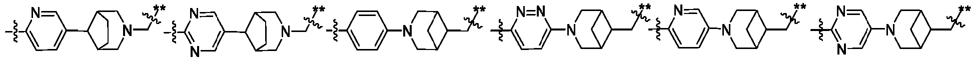 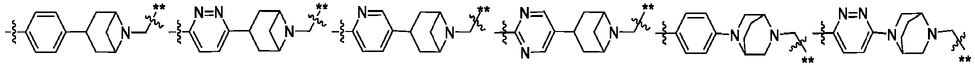 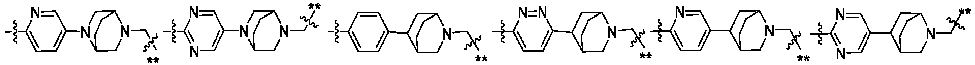 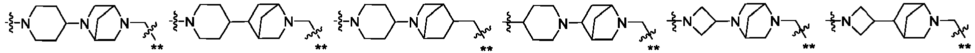 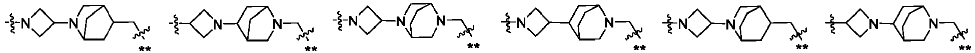 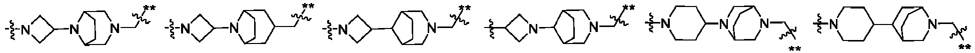 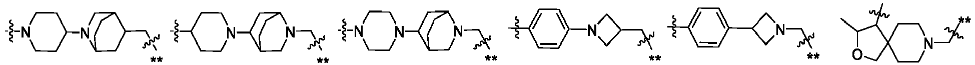 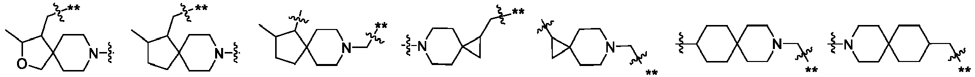 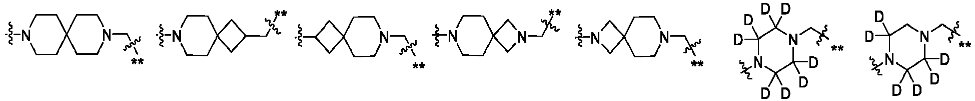 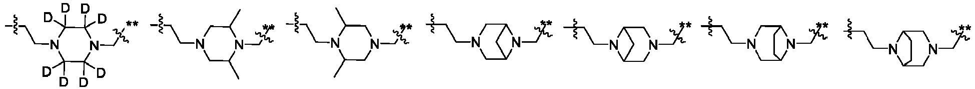 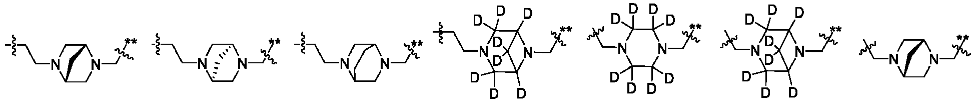 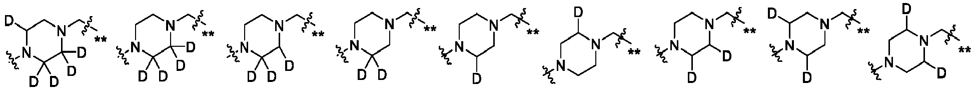 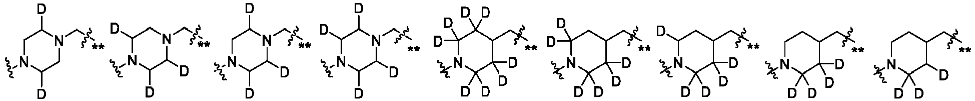 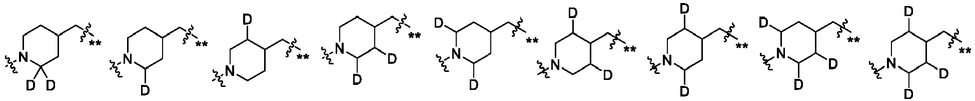 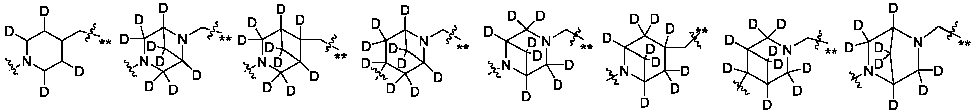 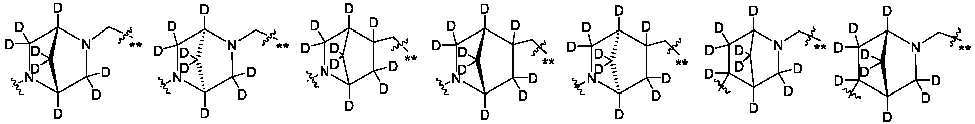 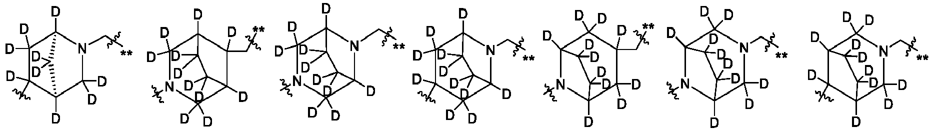 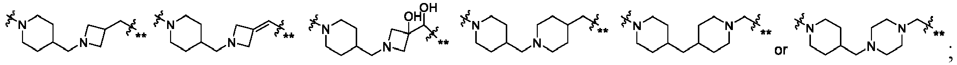 wherein the groups are optionally substituted with one or more (e.g., 1-10 ,1-8 ,1-6, such as 1, 2, 3, 4, 5 or 6) substituents selected from the group consisting of deuterium, halogen (e.g., fluorine, chlorine, bromine, or iodine), cyano, amino, carboxyl, optionally deuterated C₁₋₆ alkyl (e.g., optionally deuterated C₁₋₄ alkyl or optionally deuterated C₁₋₃ alkyl, such as methyl, CD₃, ethyl, propyl, isopropyl, butyl, sec-butyl, or tert-butyl), optionally halogenated C₁₋₆ alkyl (e.g., optionally halogenated C₁₋₄ alkyl or optionally halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₄ alkyl or perdeuterated C₁₋₃ alkyl, such as perdeuterated methyl (CD₃), perdeuterated ethyl, perdeuterated propyl, perdeuterated isopropyl, perdeuterated butyl, perdeuterated sec-butyl or perdeuterated tert-butyl), C₁₋₆ alkoxy (e.g., C₁₋₄ alkoxy or C₁₋₃ alkoxy, such as methoxy, ethoxy, propoxy, isopropoxy, butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkoxy (e.g., perdeuterated C₁₋₄ alkoxy, such as D₃C-O-, CD₃CD₂-O-, or CD₃CD₂CD₂-O-), C₂₋₆ alkynyl (e.g., C₂₋₄ alkynyl , such as ethynyl, propynyl, or butynyl), C₂₋₆ alkenyl (e.g., C₂₋₄ alkenyl, such as vinyl, propenyl, or butenyl), optionally deuterated C₃₋₆ cycloalkyl (e.g., optionally deuterated cyclopropyl, cyclobutyl, cyclopentyl, or cyclohexyl), or any combination thereof; wherein symbol * * indicates the point of attachment to ULM.

Herein, when PBM of the compounds of Formula (II) of the present disclosure represents the following strucutre: 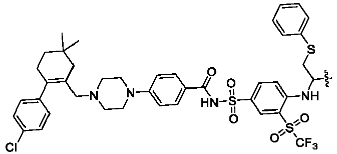 and simultaneously -R_{f}-LIN- represents 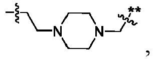 wherein symbol ** indicates the point of attachment to ULM, (R_{b})ₙ indicates that benzene ring of Formula (ULM) is substituted with 1, 2 or 3 R_{b}, with each R_{b} being the same or different and each independently representing deuterium, halogen, hydroxy, mercapto, nitro, amino, cyano, optionally deuterated C₁₋₆ alkyl, optionally deuterated C₁₋₆ alkoxy, halogenated C₁₋₆ alkyl, optionally substituted C₃₋₆ cycloalkyl, C₂₋₆ alkenyl, or C₂₋₆ alkynyl.

In some embodiments, the compounds of Formula (II) is also of the structure of the following Formula (II-1), Formula (II-2), Formula (II-3), Formula (II-4), Formula (II-5), Formula (II-6), Formula (II-7), Formula (II-8), Formula (II-9), Formula (11-10), Formula (II-11), Formula (11-12), Formula (II-13), Formula (11-14), Formula (11-15), Formula (11-16), Formula (11-17), Formula (II-18), Formula (II-19), Formula (II-20), Formula (II-21), Formula (II-22), Formula (II-23), Formula (II-24), Formula (II-25), Formula (II-26), Formula (II-27), Formula (II-28), Formula (II-29), Formula (II-30), Formula (II-31), Formula (II-32), Formula (II-33), Formula (II-34), Formula (II-35), Formula (II-36), Formula (II-37), Formula (II-38), Formula (11-39), Formula (II-40), Formula (11-41), Formula (II-42), Formula (II-43), Formula (II-44), Formula (II-45), Formula (II-46), Formula (II-47), Formula (II-48), Formula (II-49), Formula (II-50), Formula (II-51), Formula (II-52), Formula (II-53), Formula (II-55), Formula (II-56), Formula (II-57), Formula (II-58), Formula (II-59), Formula (II-60), Formula (II-61), Formula (II-62), Formula (11-63), Formula (II-64) , Formula (11-65) or Formula (11-66): 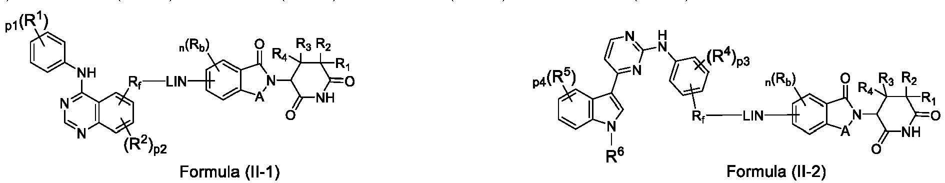 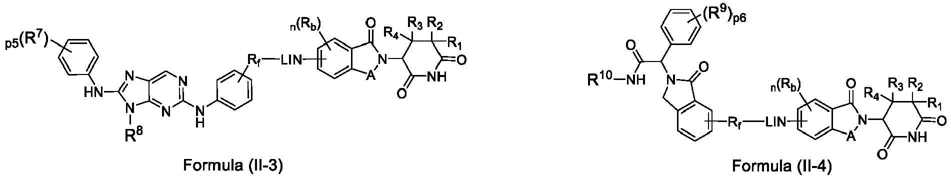 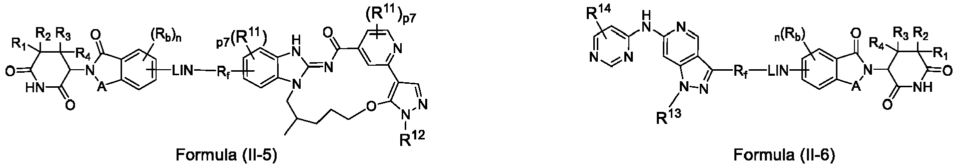 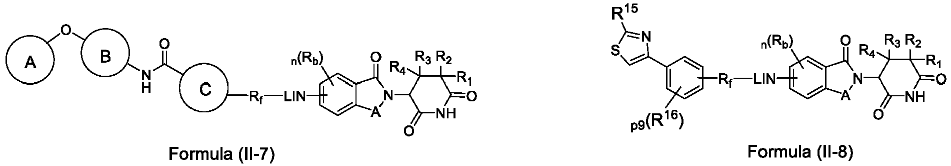 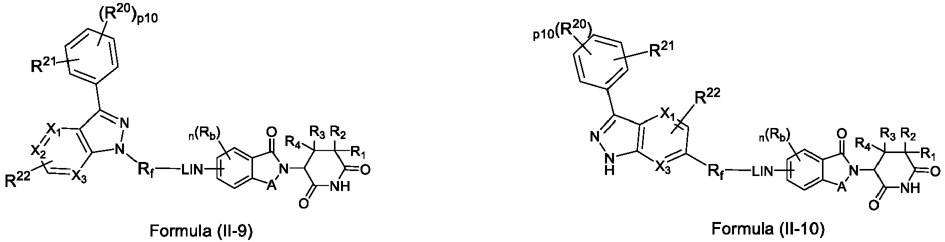 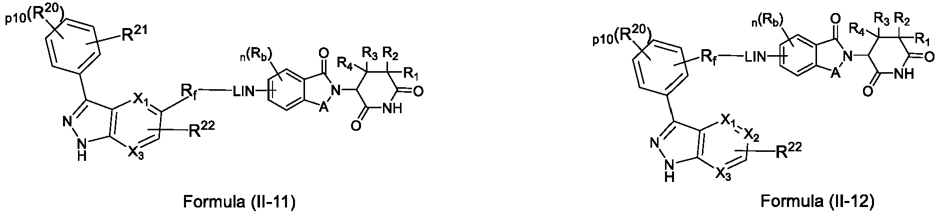 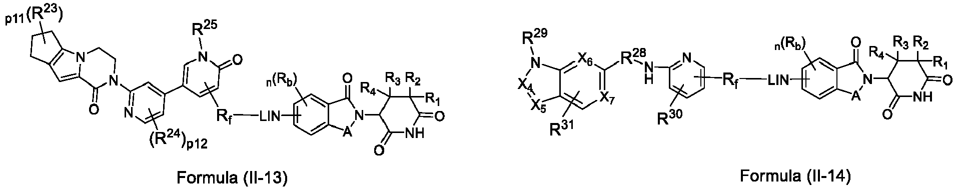 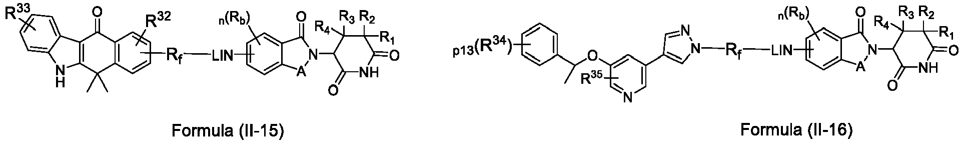 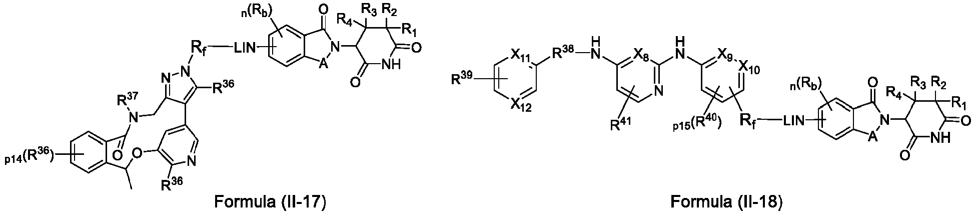 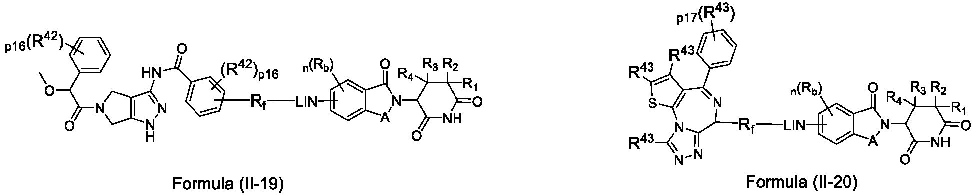 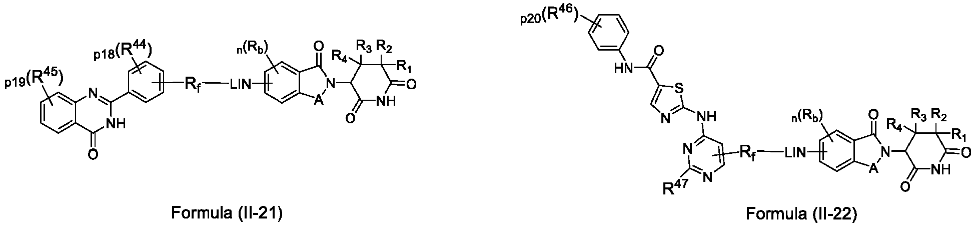 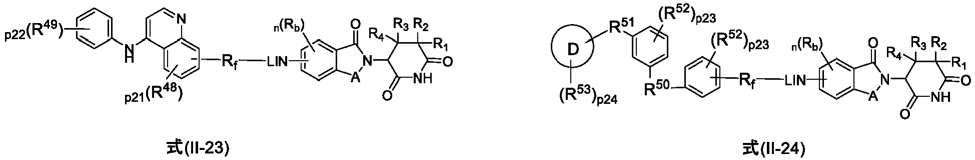 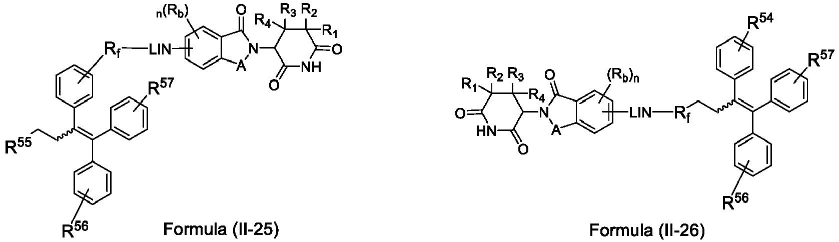 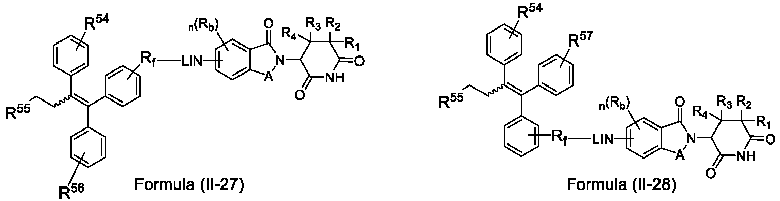 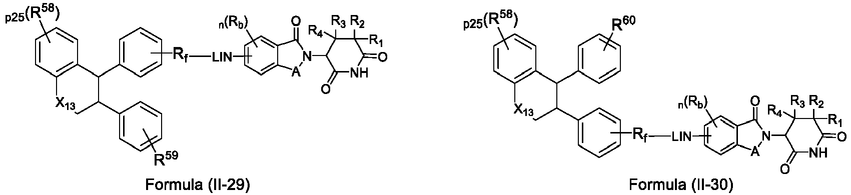 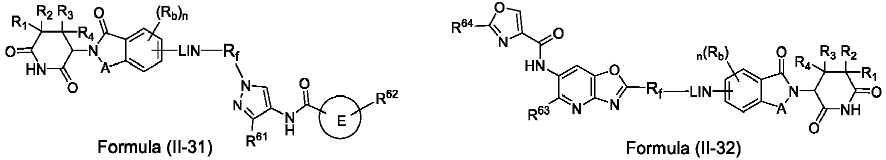 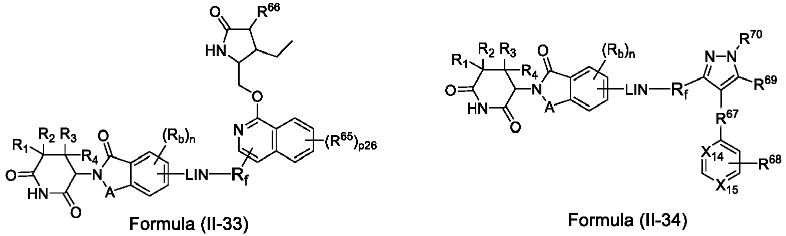 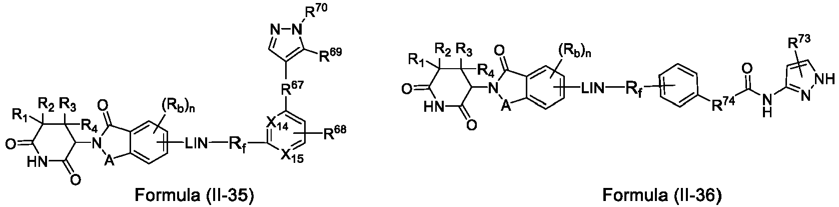 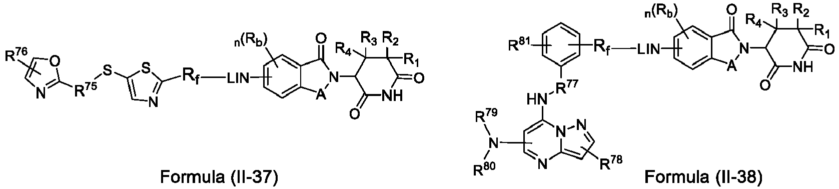 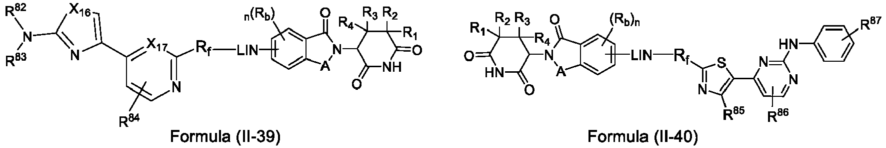 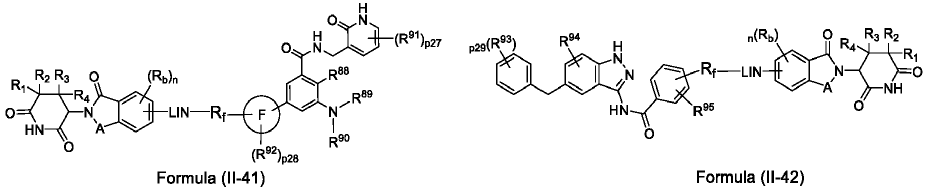 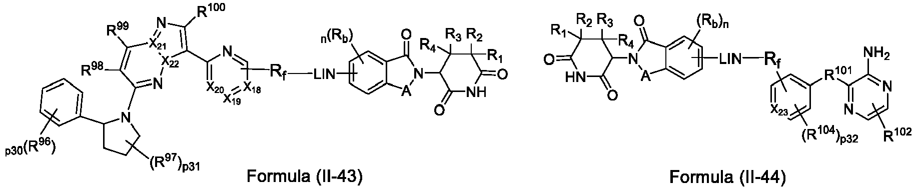 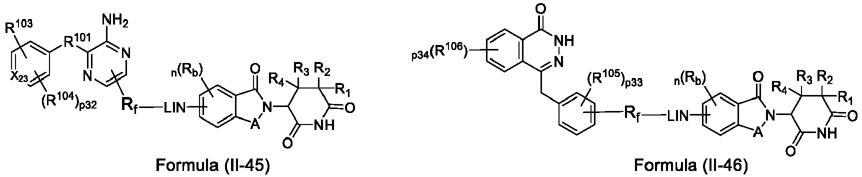 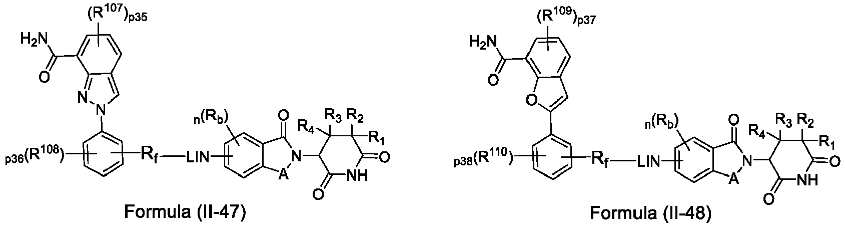 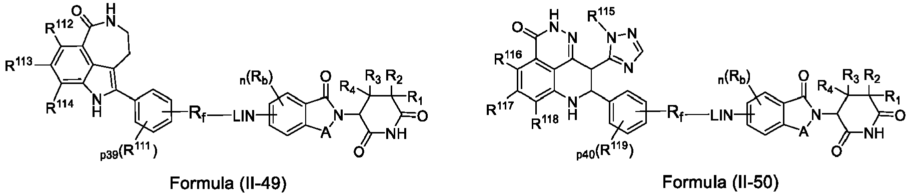 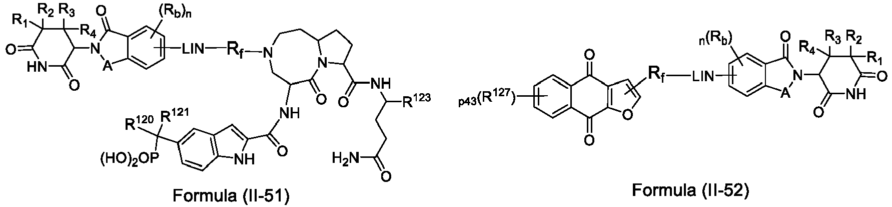 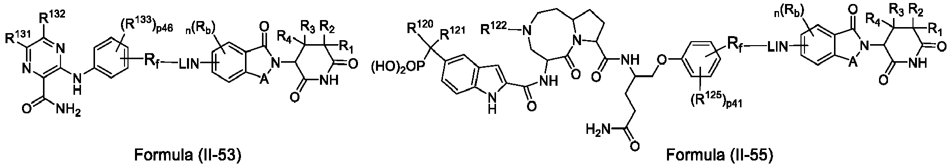 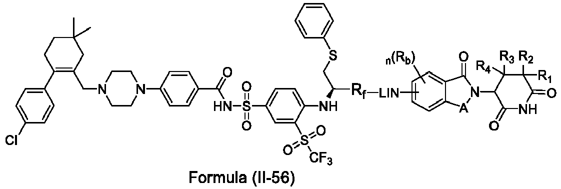 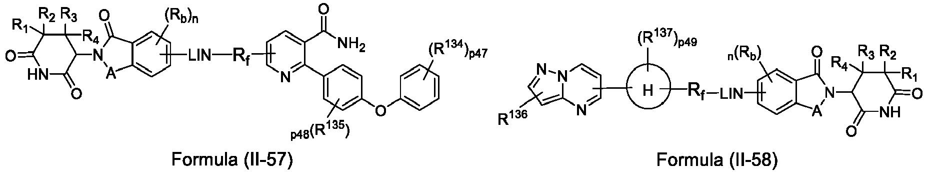 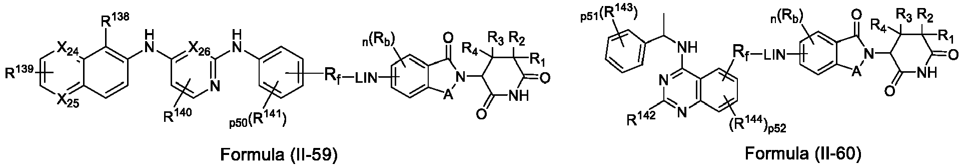 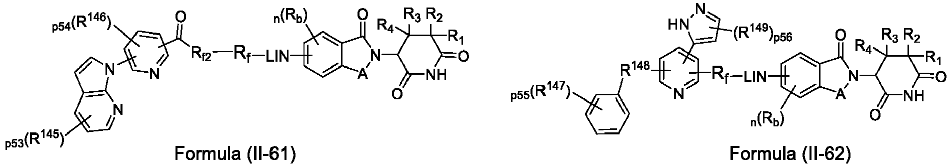 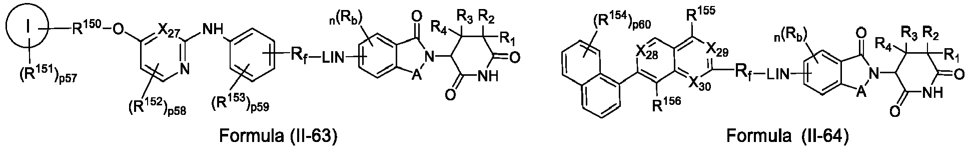 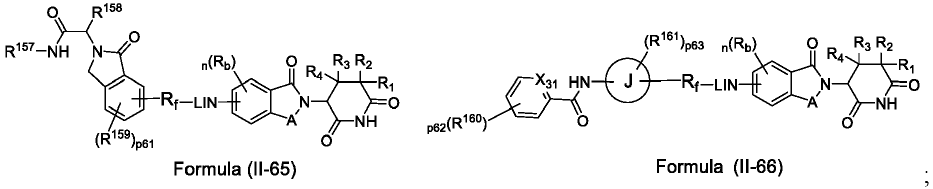 wherein A, R₁, R₂, R₃, R₄, (R_{b})ₙ, LIN, R_{f}, (R¹)ₚ₁, (R²)ₚ₂, (R⁴)ₚ₃, (R⁵)ₚ₄, (R⁷)ₚ₅, R⁸, (R⁹)ₚ₆, R¹⁰, (R¹¹)ₚ₇, R¹², R¹³, R¹⁴, ring A, ring B, ring C, R¹⁵, (R¹⁶)ₚ₉, X₁, X₂, X₃, (R²⁰)ₚ₁₀, R²¹, R²², (R²³)ₚ₁₁, (R²⁴)ₚ₁₂, R²⁵, X₄, X₅, X₆, X₇, R²⁸, R²⁹, R³⁰, R³¹, R³², R³³, (R³⁴)ₚ₁₃, R³⁵, (R³⁶)ₚ₁₄, R³⁷, X₈, X₉, X₁₀, X₁₁, X₁₂, R³⁸, R³⁹, (R⁴⁰)ₚ₁₅, (R⁴²)ₚ₁₆, (R⁴³)ₚ₁₇, (R⁴⁴)ₚ₁₈, (R⁴⁵)ₚ₁₉, (R⁴⁶)ₚ₂₀, (R⁴⁸)ₚ₂₁, (R⁴⁹)ₚ₂₂, R⁵⁰, R⁵¹, (R⁵²)ₚ₂₃, (R⁵³)ₚ₂₄, R⁵⁴, R⁵⁵, R⁵⁶, R⁵⁷, (R⁵⁸)ₚ₂₅, X₁₃, R⁵⁹, R⁶⁰, R⁶¹, R⁶², R⁶³, R⁶⁴, (R⁶⁵)ₚ₂₆, R⁶⁶, R⁶⁷, R⁶⁸, R⁶⁹, R⁷⁰, X₁₄, X₁₅, R⁷³, R⁷⁴, R⁷⁵, R⁷⁶, R⁷⁷, R⁷⁸, R⁷⁹, R⁸⁰, R⁸¹, X₁₆, X₁₇, R⁸², R⁸³, R⁸⁴, R⁸⁵, R⁸⁶, R⁸⁷, R⁸⁸, R⁸⁹, R⁹⁰, (R⁹¹)ₚ₂₇, (R⁹²)ₚ₂₈, (R⁹³)ₚ₂₉, R⁹⁴, R⁹⁵, (R⁹⁶)ₚ₃₀, (R⁹⁷)ₚ₃₁, X₁₈, X₁₉, X₂₀, X₂₁, X₂₂, R⁹⁸, R⁹⁹, R¹⁰⁰, R¹⁰¹, R¹⁰², R¹⁰³, X₂₃, (R¹⁰⁴)ₚ₃₂, (R¹⁰⁵)ₚ₃₃, (R¹⁰⁶)ₚ₃₄, (R¹⁰⁷)ₚ₃₅, (R¹⁰⁸)ₚ₃₆, (R¹⁰⁹)ₚ₃₇, (R¹¹⁰)ₚ₃₈, (R¹¹¹)ₚ₃₉, R¹¹², R¹¹³, R¹¹⁴, R¹¹⁵, R¹¹⁶, R¹¹⁷, R¹¹⁸, (R¹¹⁹)ₚ₄₀, R¹²⁰, R¹²¹, R¹²², R¹²³, (R¹²⁵)ₚ₄₁, (R¹²⁷)ₚ₄₃, R¹³¹, R¹³², (R¹³³)ₚ₄₆, (R¹³⁴)ₚ₄₇, (R¹³⁵)ₚ₄₈, R¹³⁶, (R¹³⁷)ₚ₄₉, X₂₄, X₂₅, R¹²⁰, R¹²¹, R¹²², R¹²³, (R¹²⁵)ₚ₄₁, (R¹²⁷)ₚ₄₃, R¹³¹, R¹³², (R¹³³)ₚ₄₆, (R¹³⁴)ₚ₄₇, (R¹³⁵)ₚ₄₈, R¹³⁶, (R¹³⁷)ₚ₄₉,X₂₄, X₂₅, X₂₆, R¹³⁸, R¹³⁹, R¹⁴⁰, (R¹⁴¹)ₚ₅₀, R¹⁴², (R¹⁴³)ₚ₅₁, (R¹⁴⁴)ₚ₅₂, (R¹⁴⁵)ₚ₅₃, (R¹⁴⁶)ₚ₅₄, R_{f2}, (R¹⁴⁷)ₚ₅₅, R¹⁴⁸, (R¹⁴⁹)ₚ₅₆, X₂₇, ring I, R¹⁵⁰, (R¹⁵¹)ₚ₅₇, (R¹⁵²)ₚ₅₈, (R¹⁵³)ₚ₅₉, X₂₈, X₂₉, X₃₀, (R¹⁵⁴)ₚ₆₀, R¹⁵⁵, R¹⁵⁶, R¹⁵⁷, R¹⁵⁸, (R¹⁵⁹)ₚ₆₁, X₃₁, (R¹⁶⁰)ₚ₆₂, ring J and (R¹⁶¹)ₚ₆₃ are as defined in the compound of Formula (II) above and each sub-embodiments thereof.

In some embodiments, the compounds of Formula (II) of the present invention and their salts (including pharmaceutically acceptable salts, such as hydrochloride, etc.), prodrugs, solvates, isotopically enriched analogs, polymorphs, stereoisomers (including enantiomers and diastereomers), or mixture of stereoisomers thereof in Table 3 below are provided.

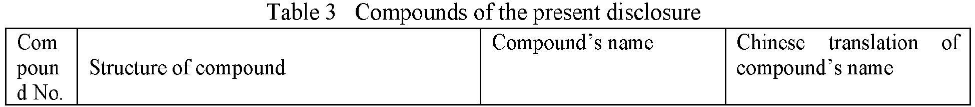

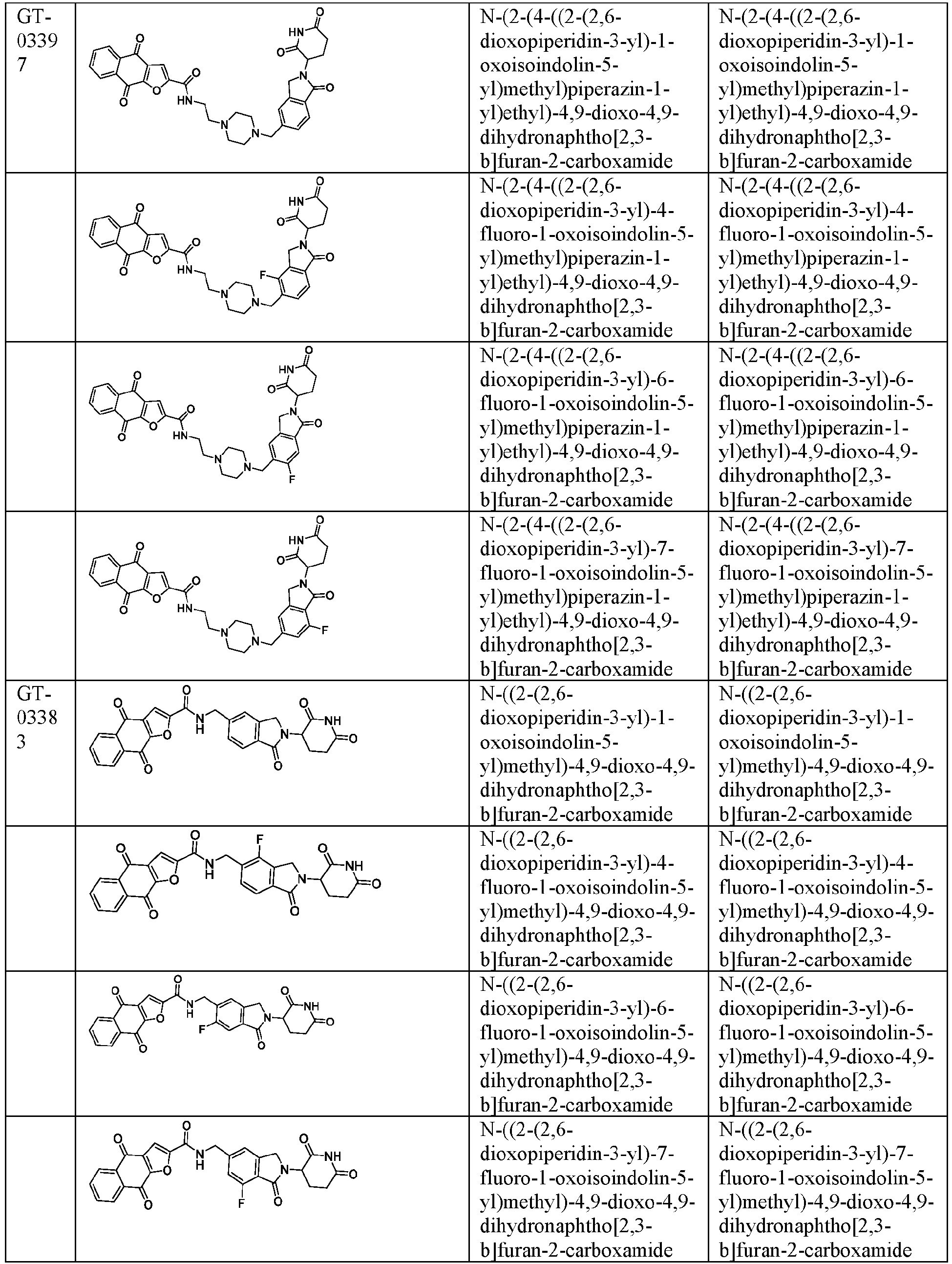

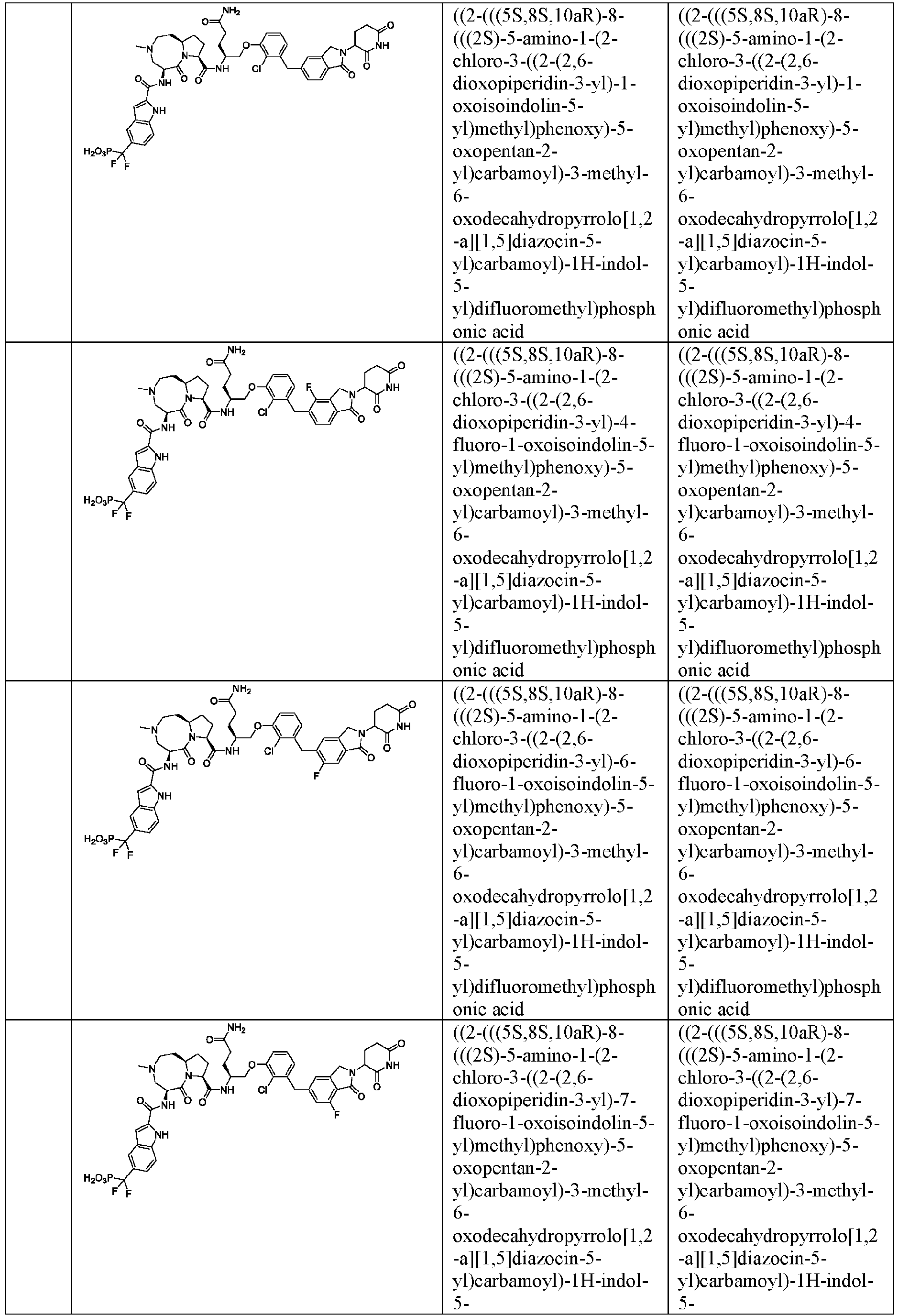

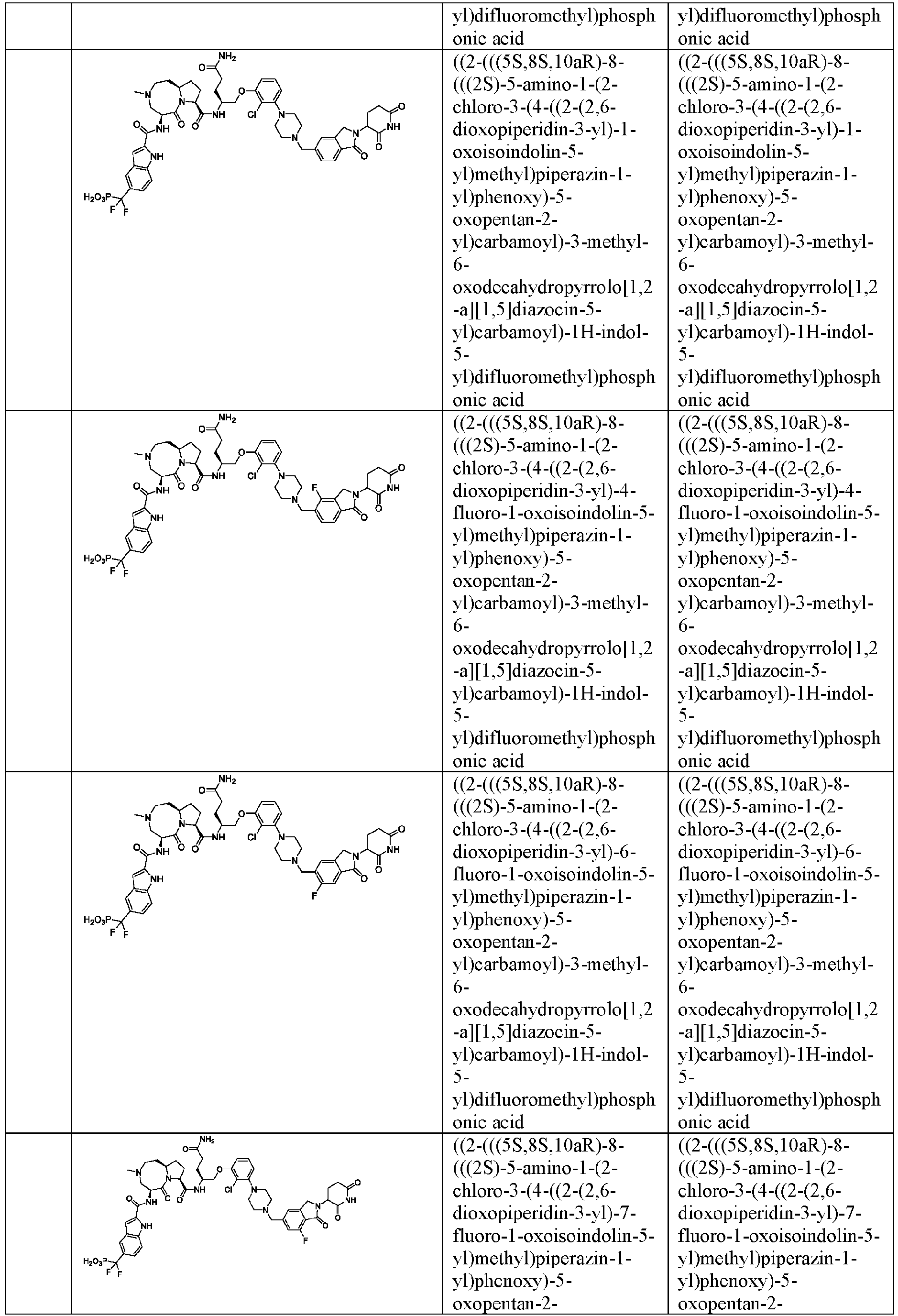

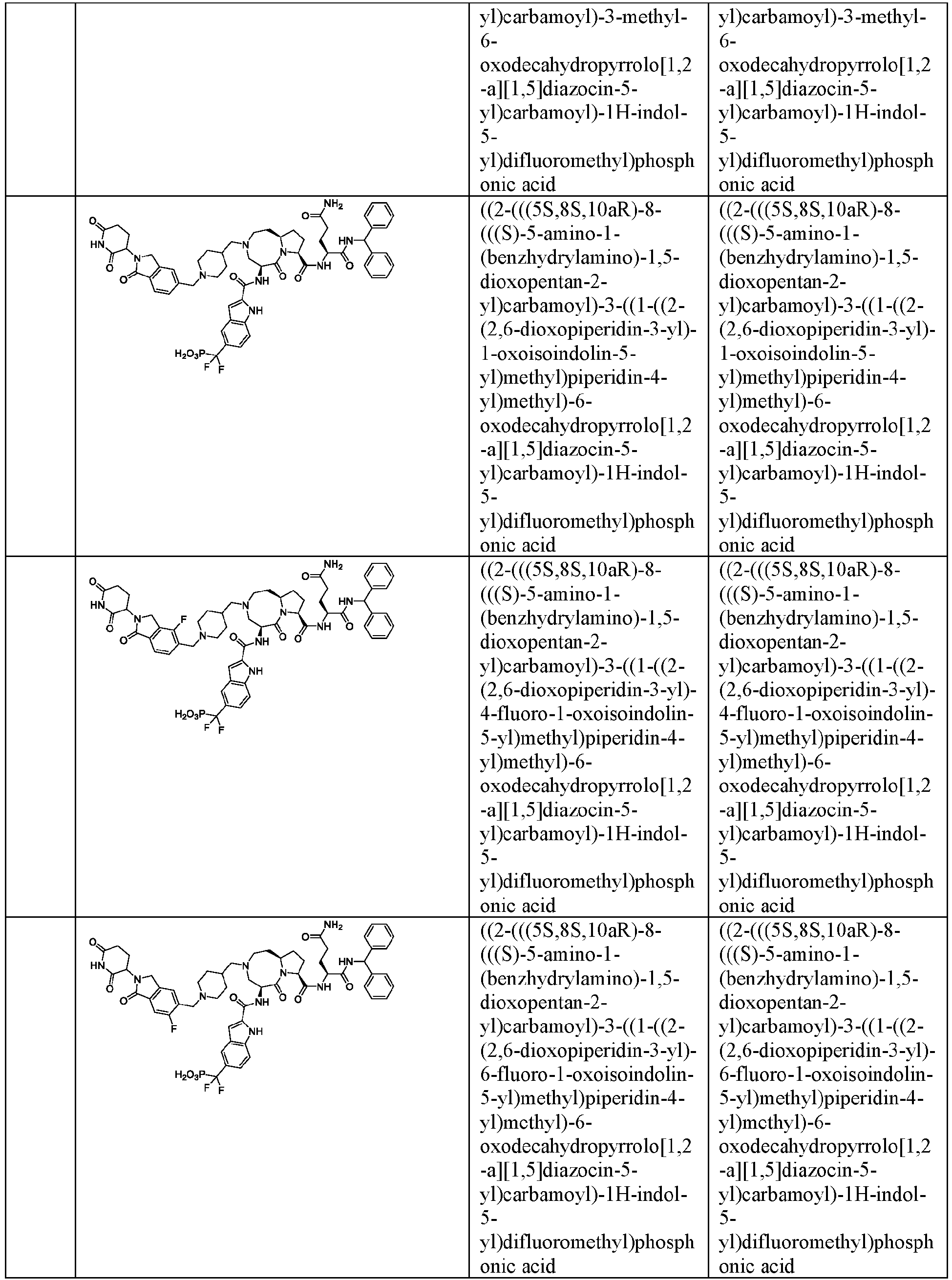

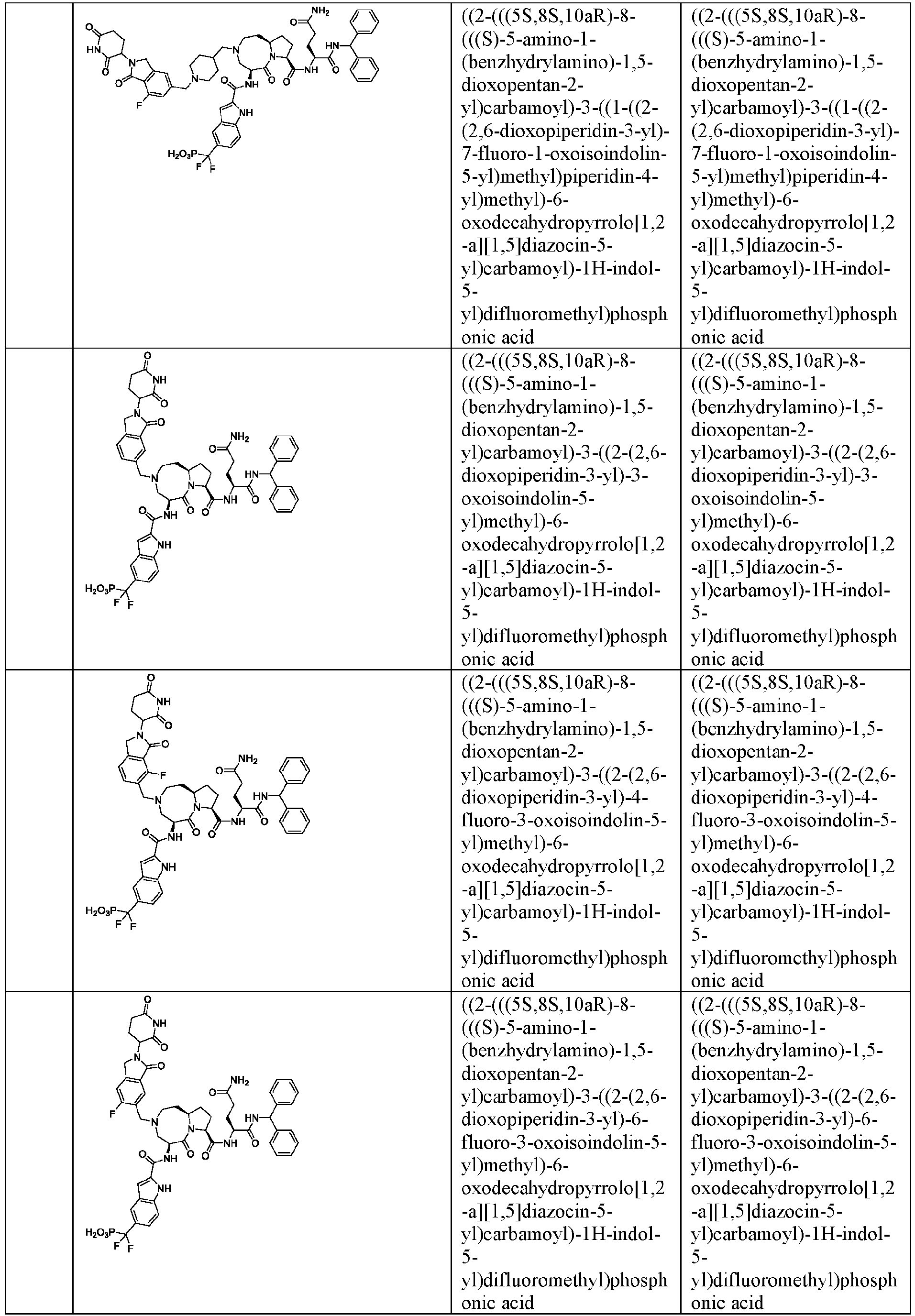

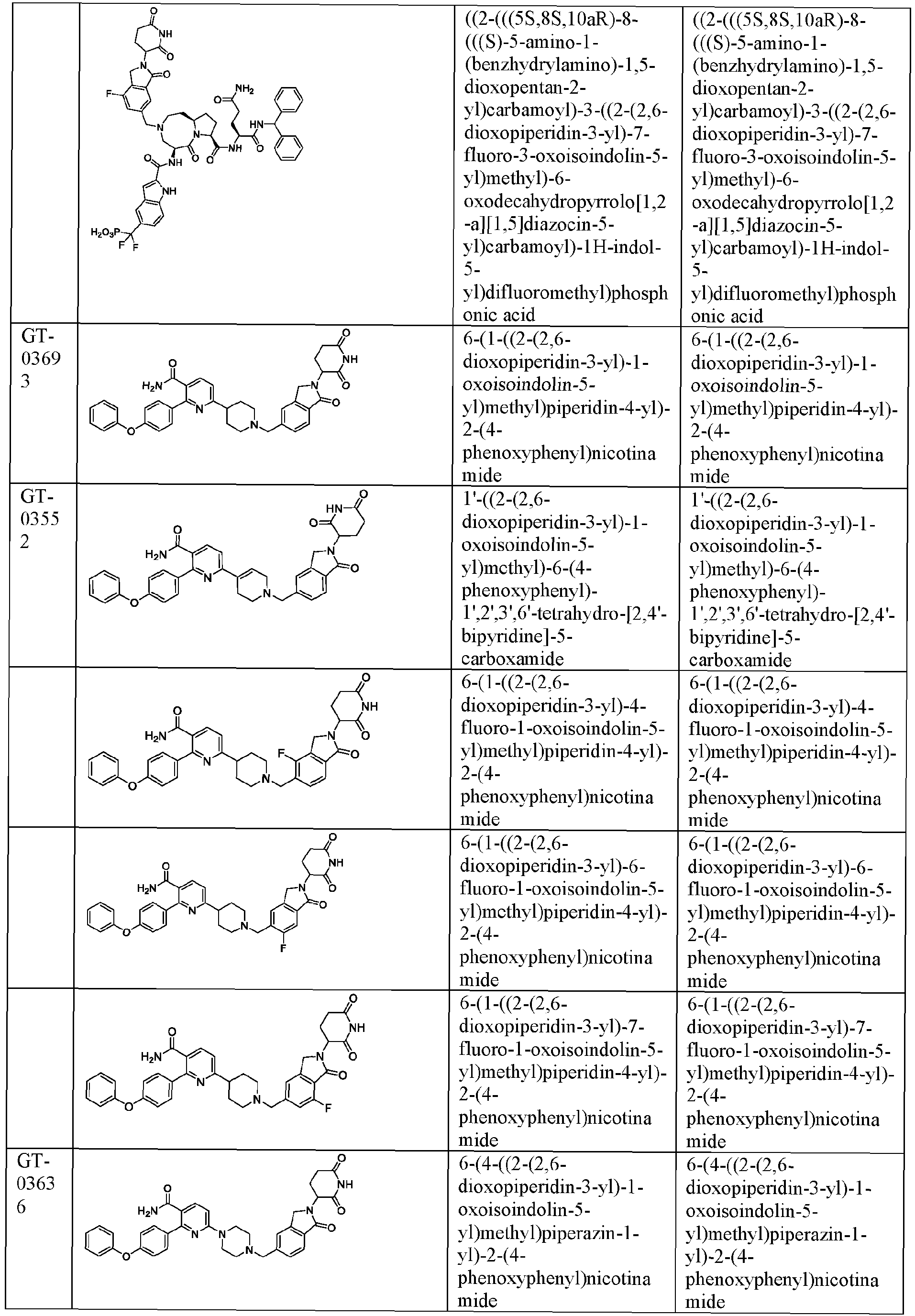

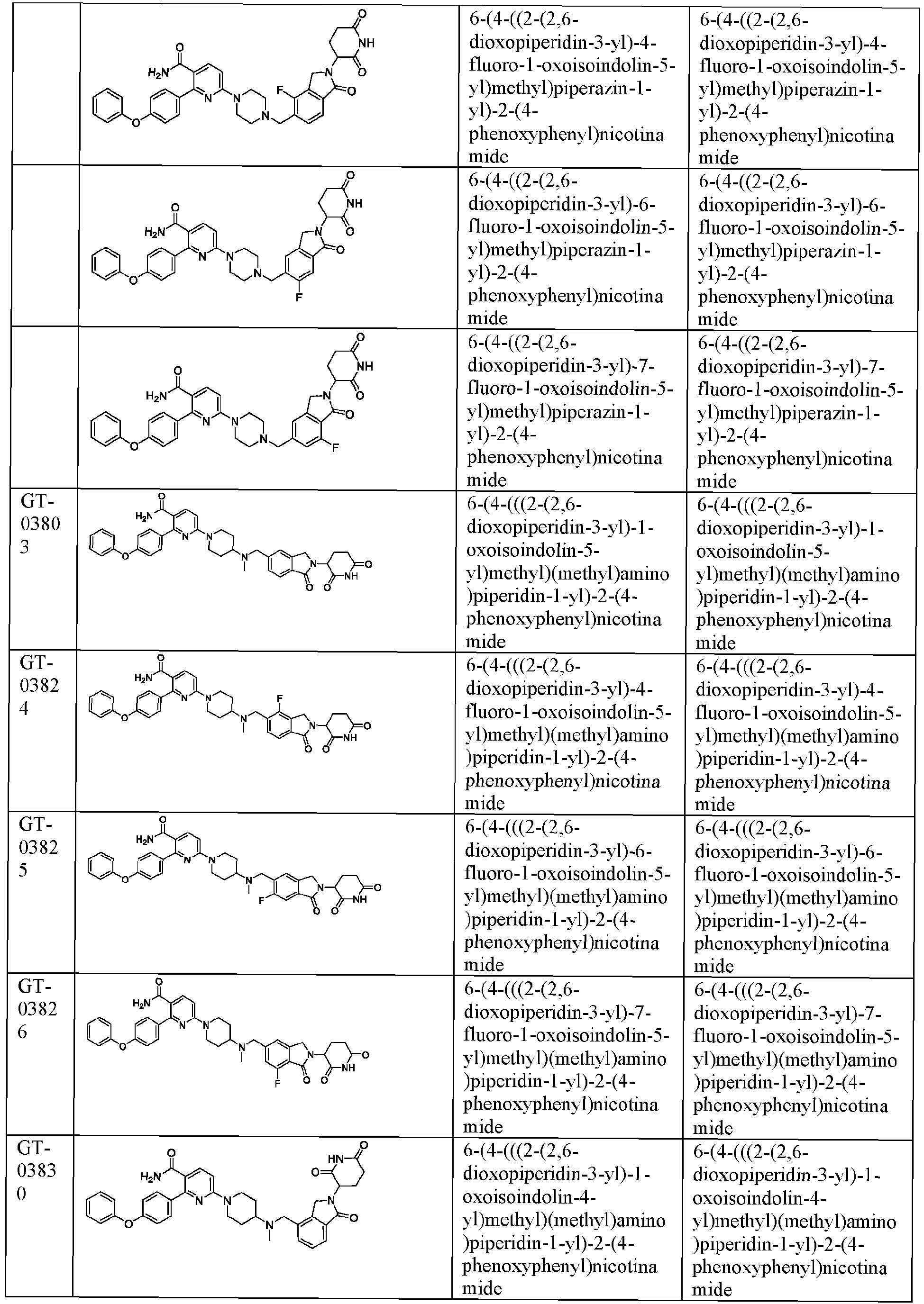

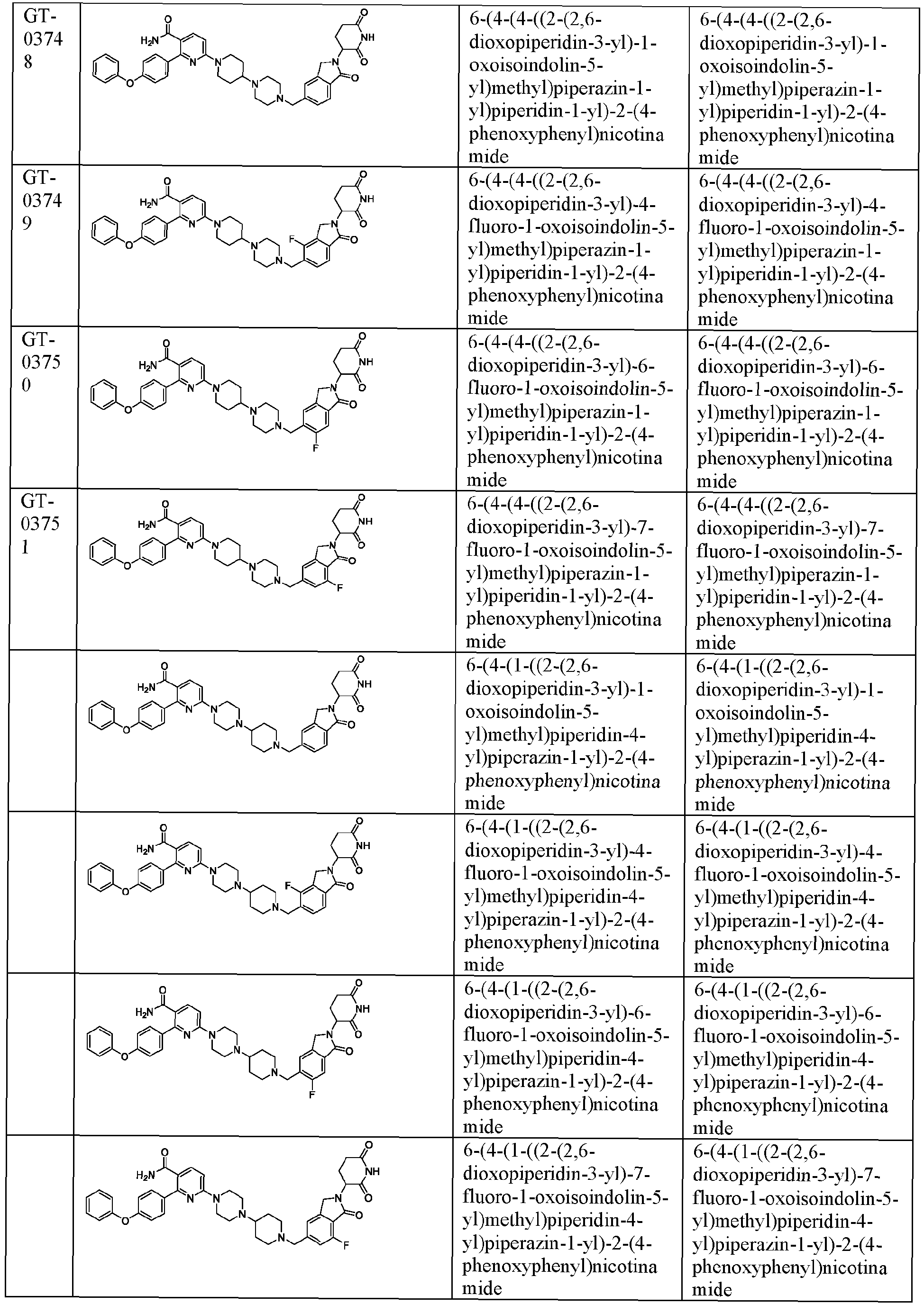

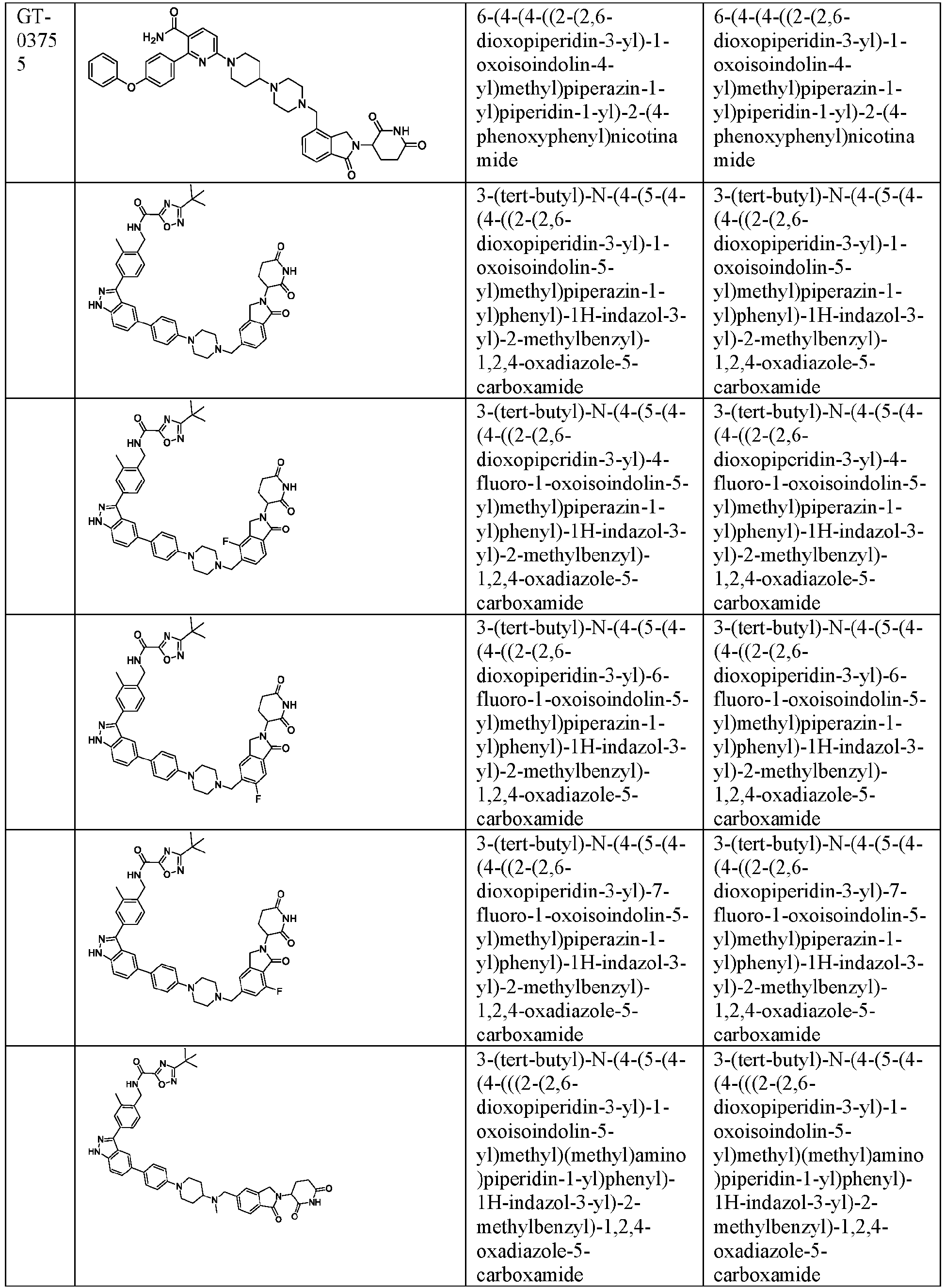

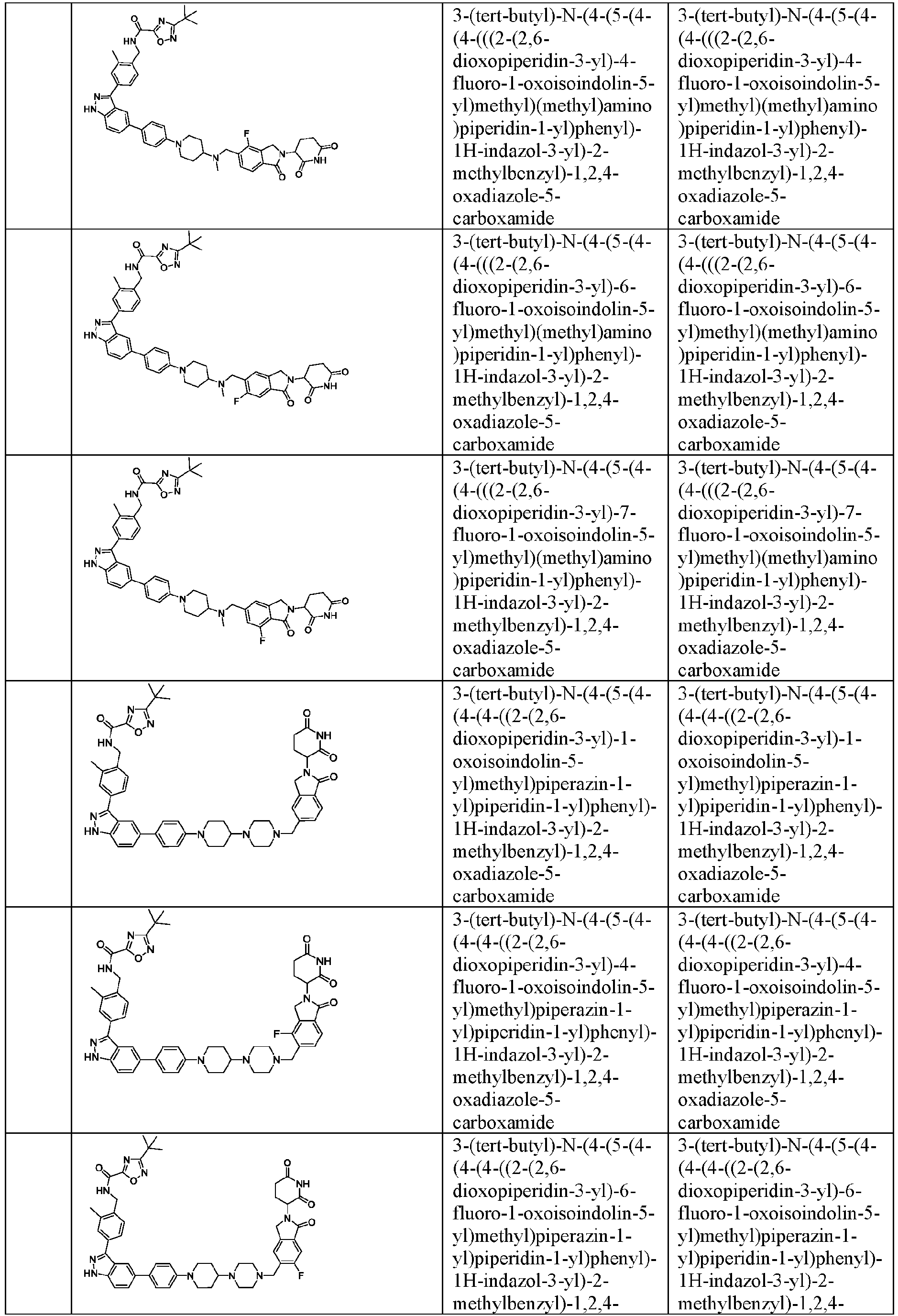

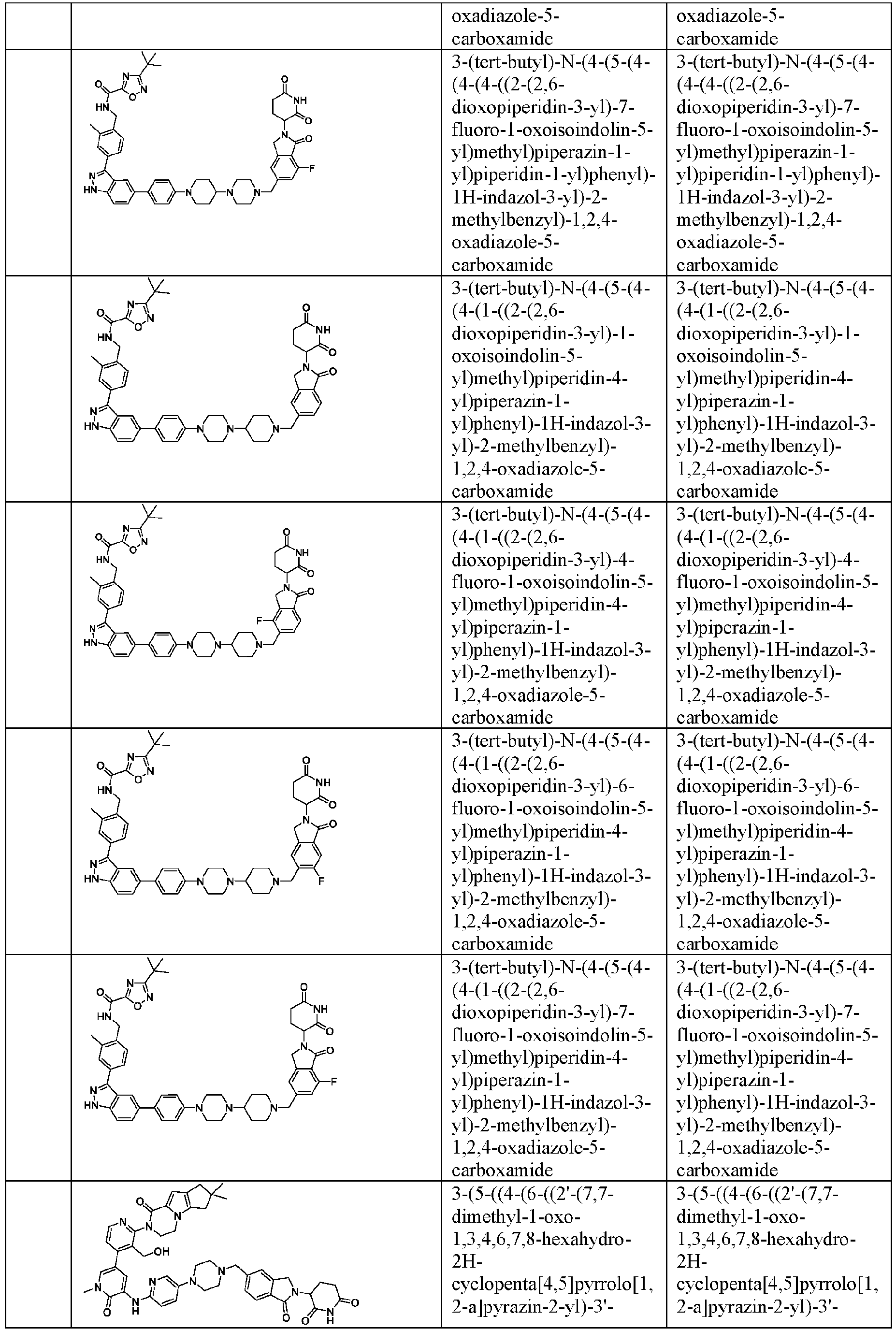

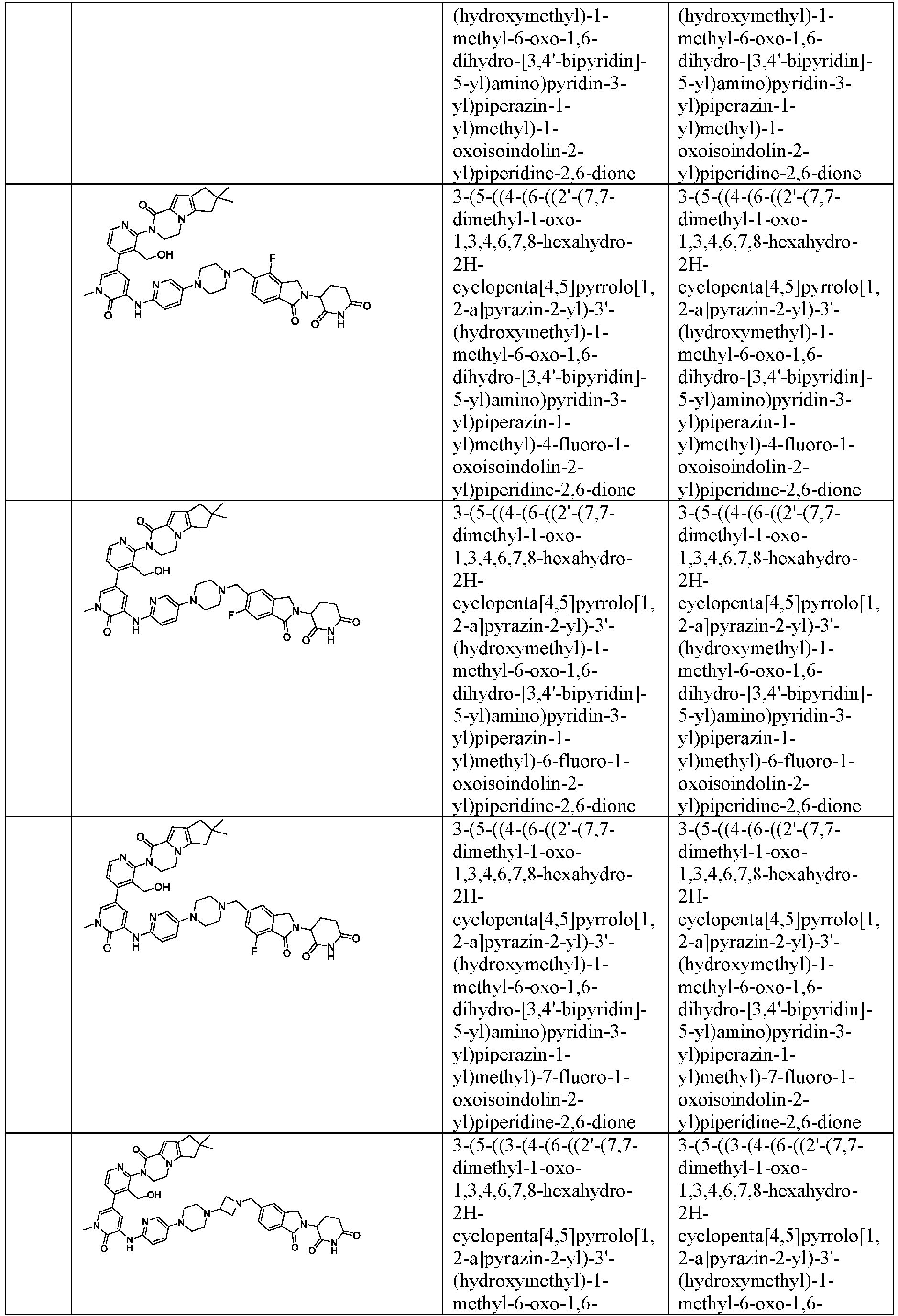

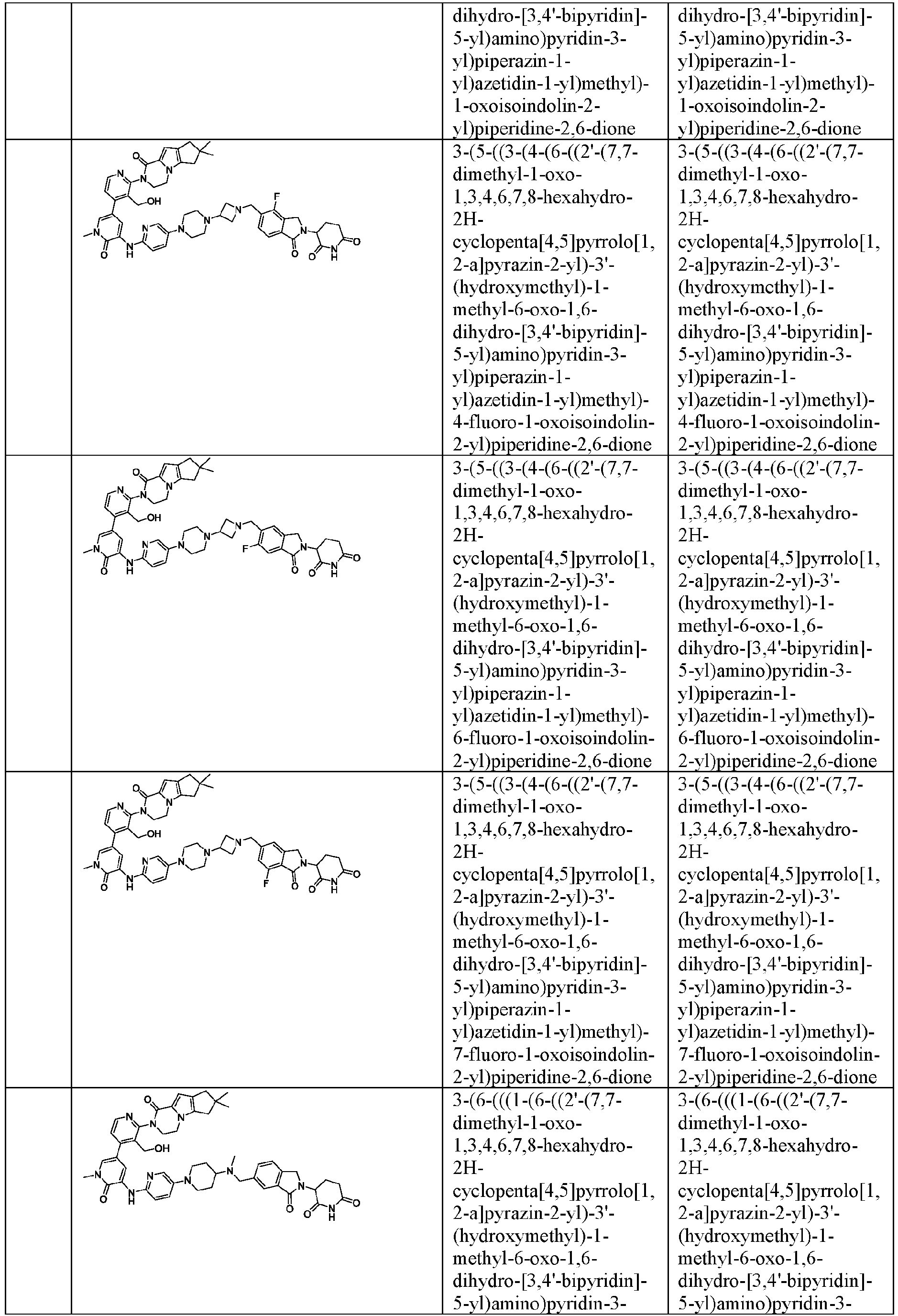

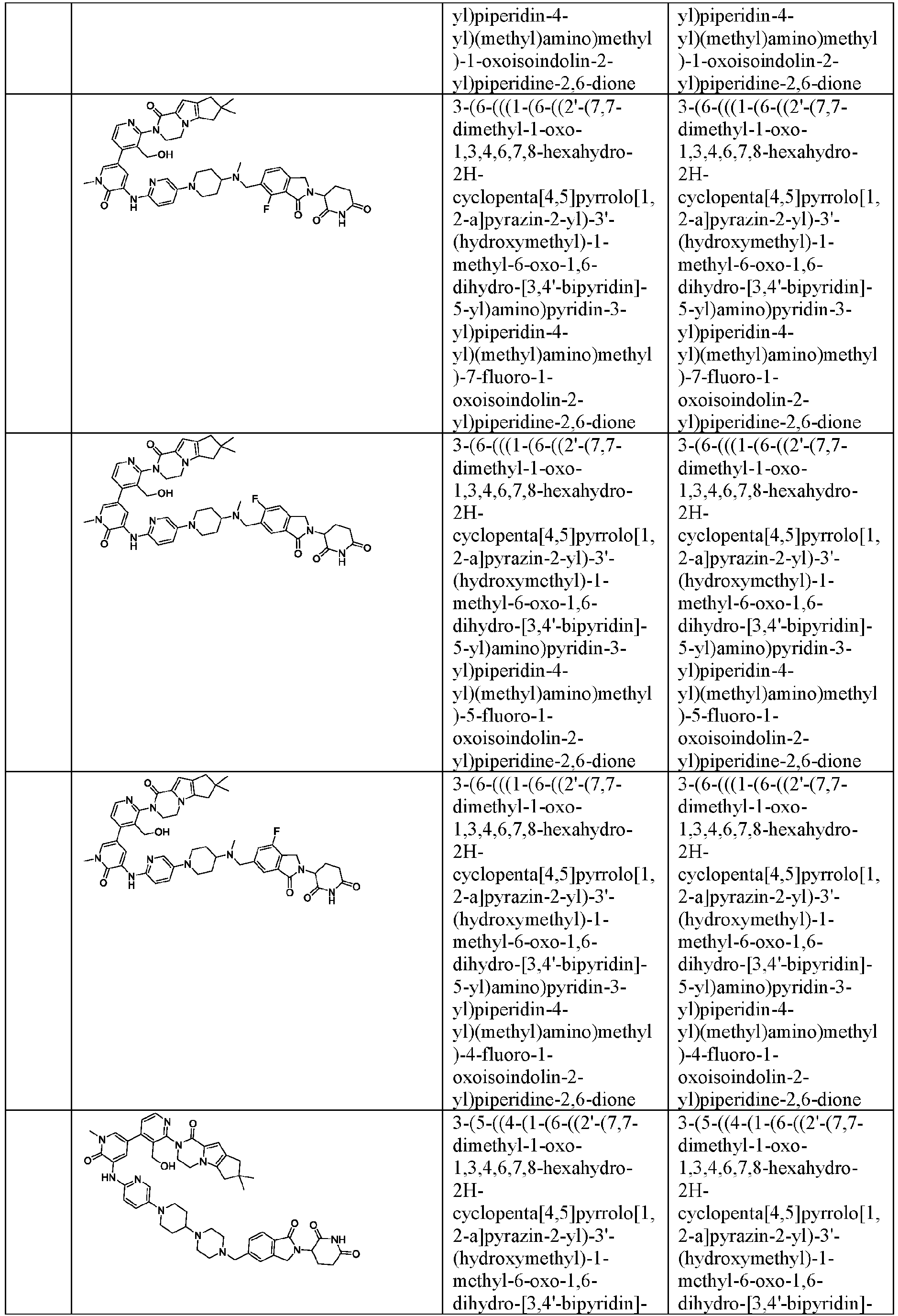

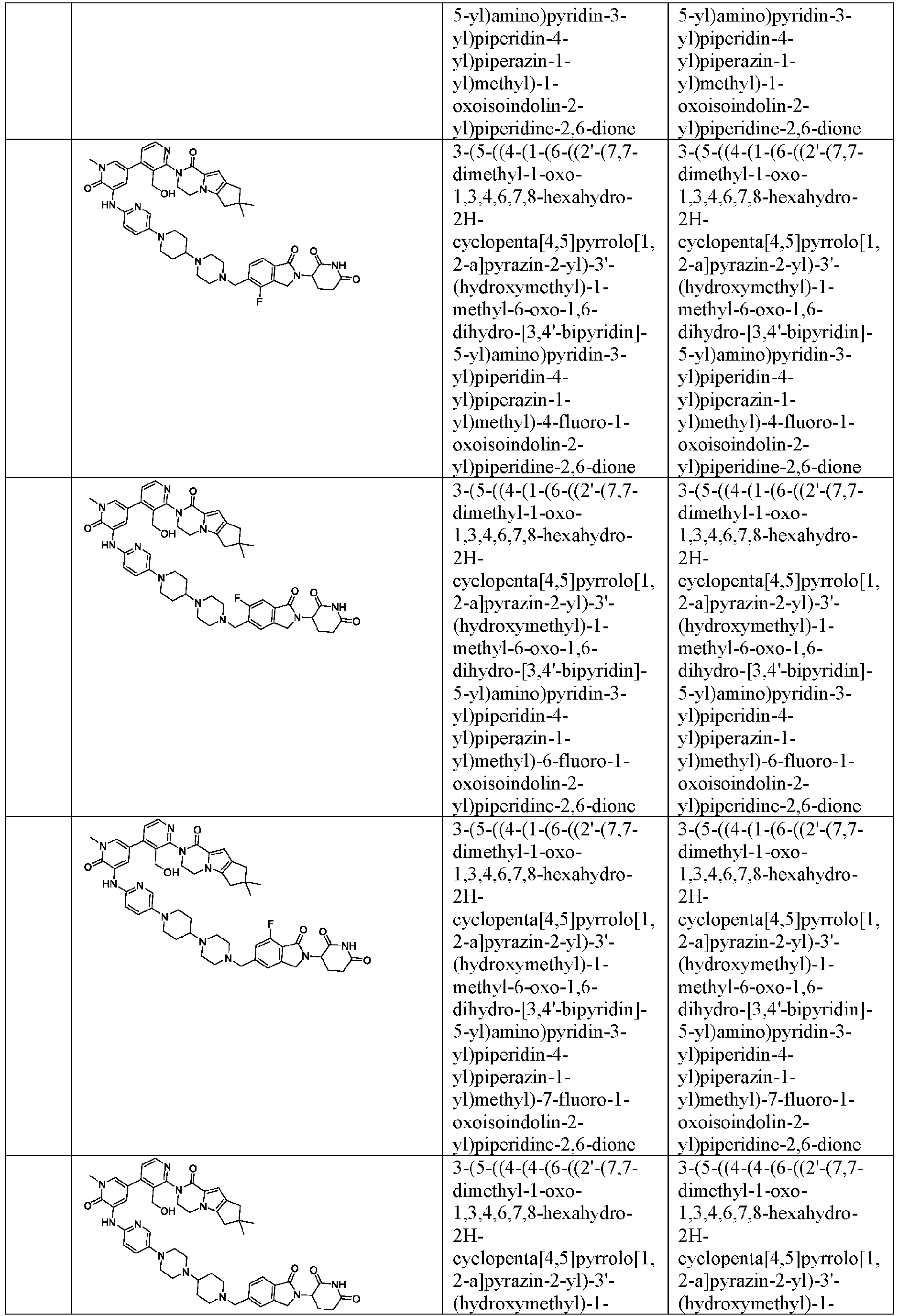

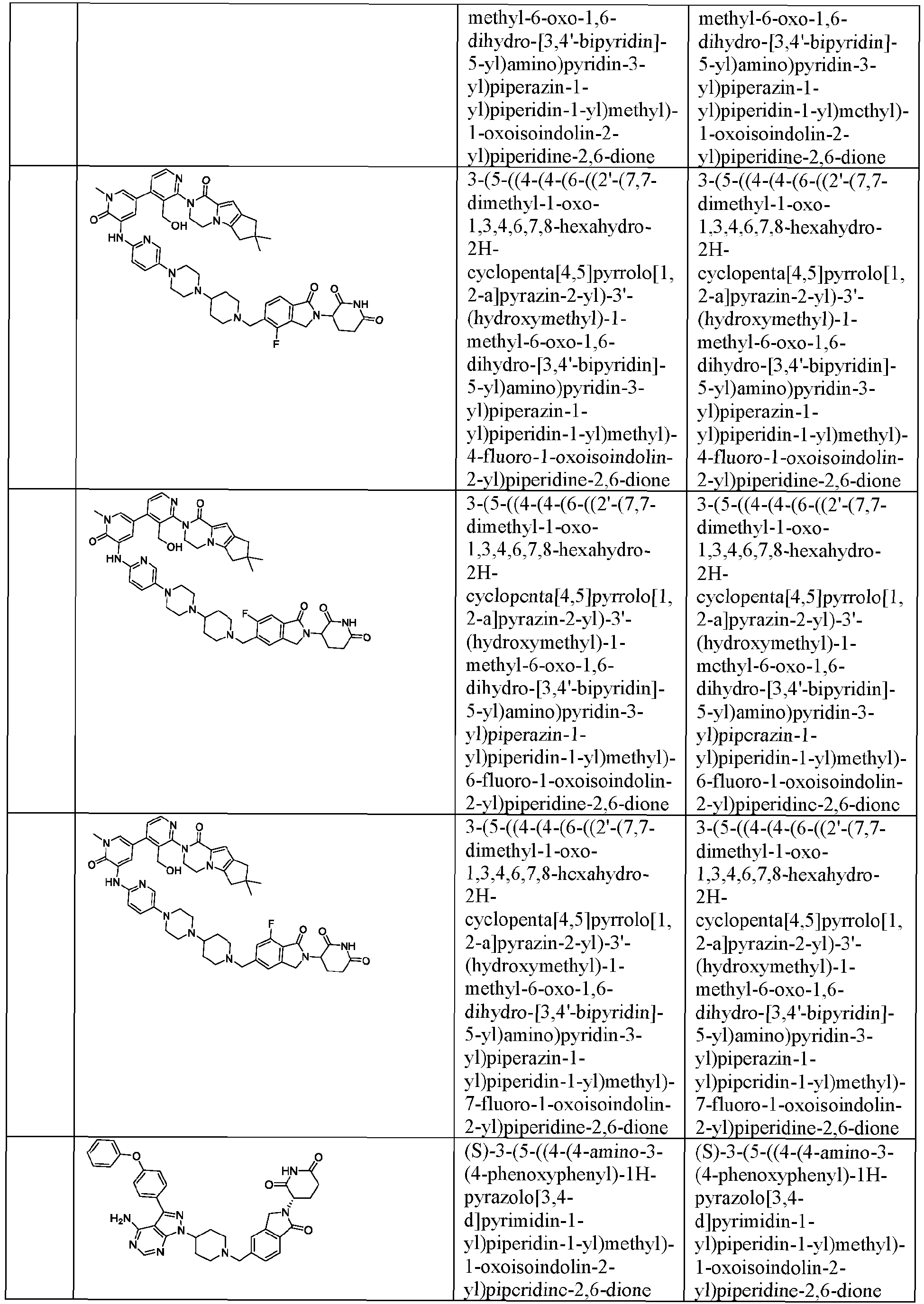

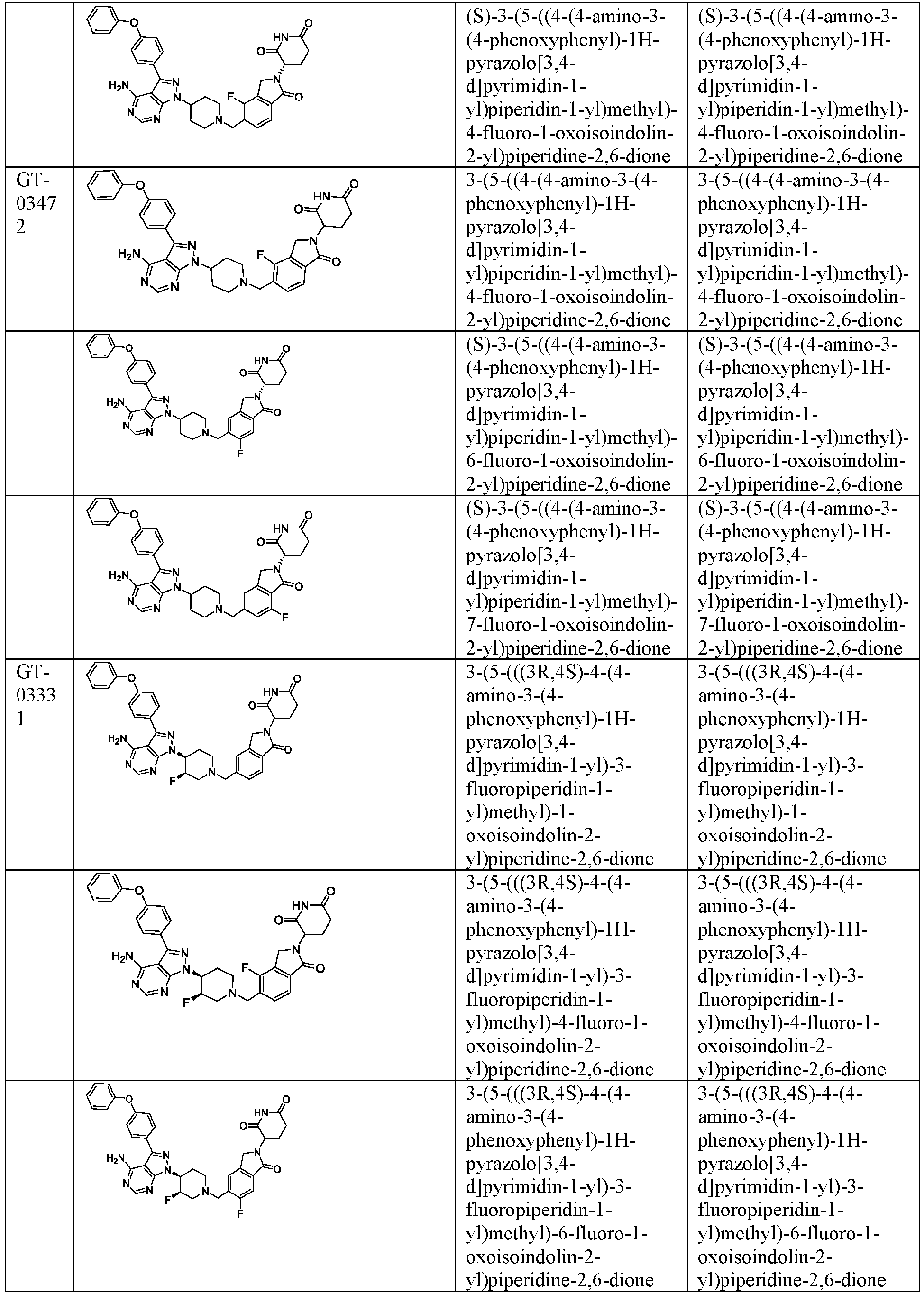

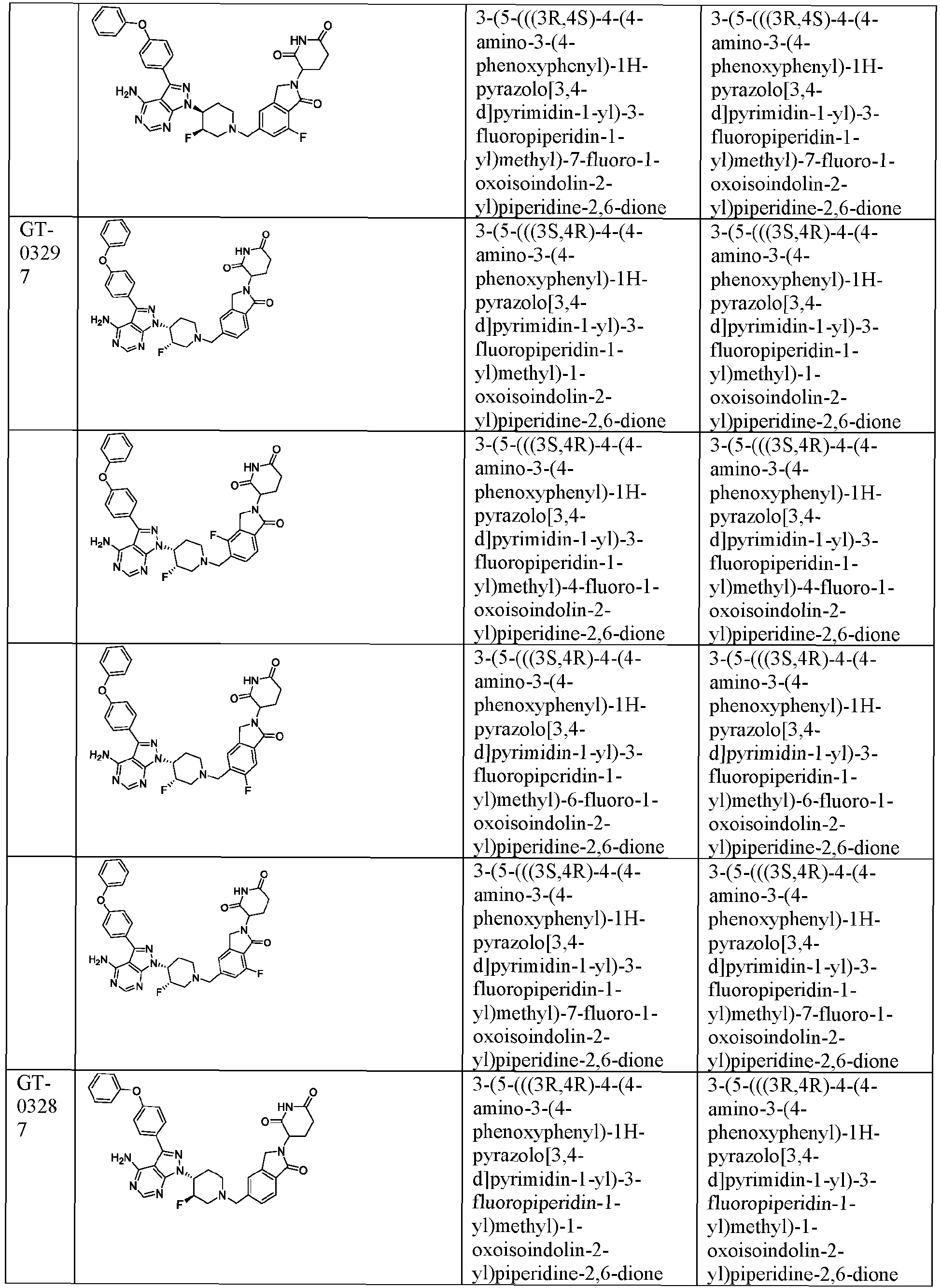

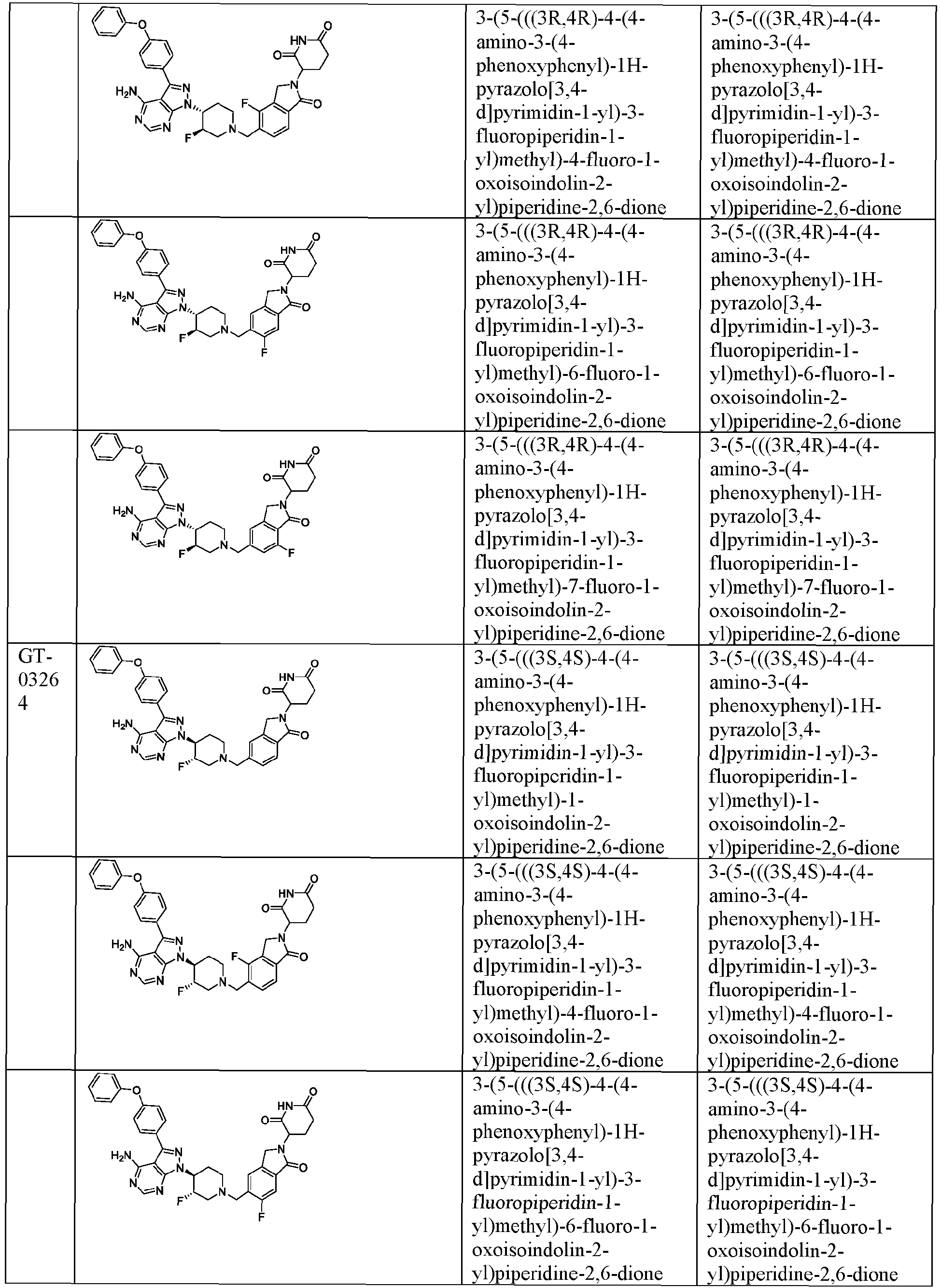

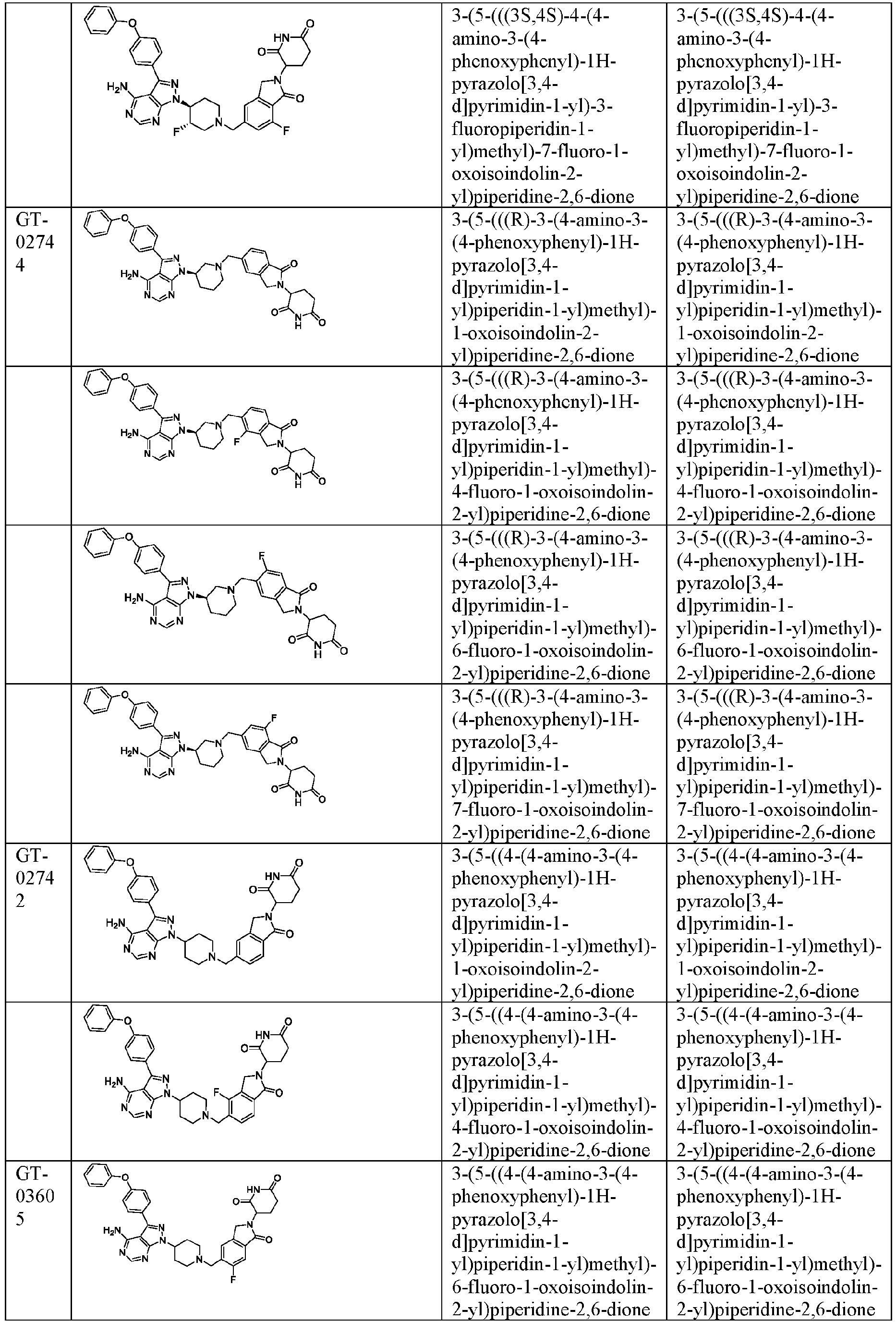

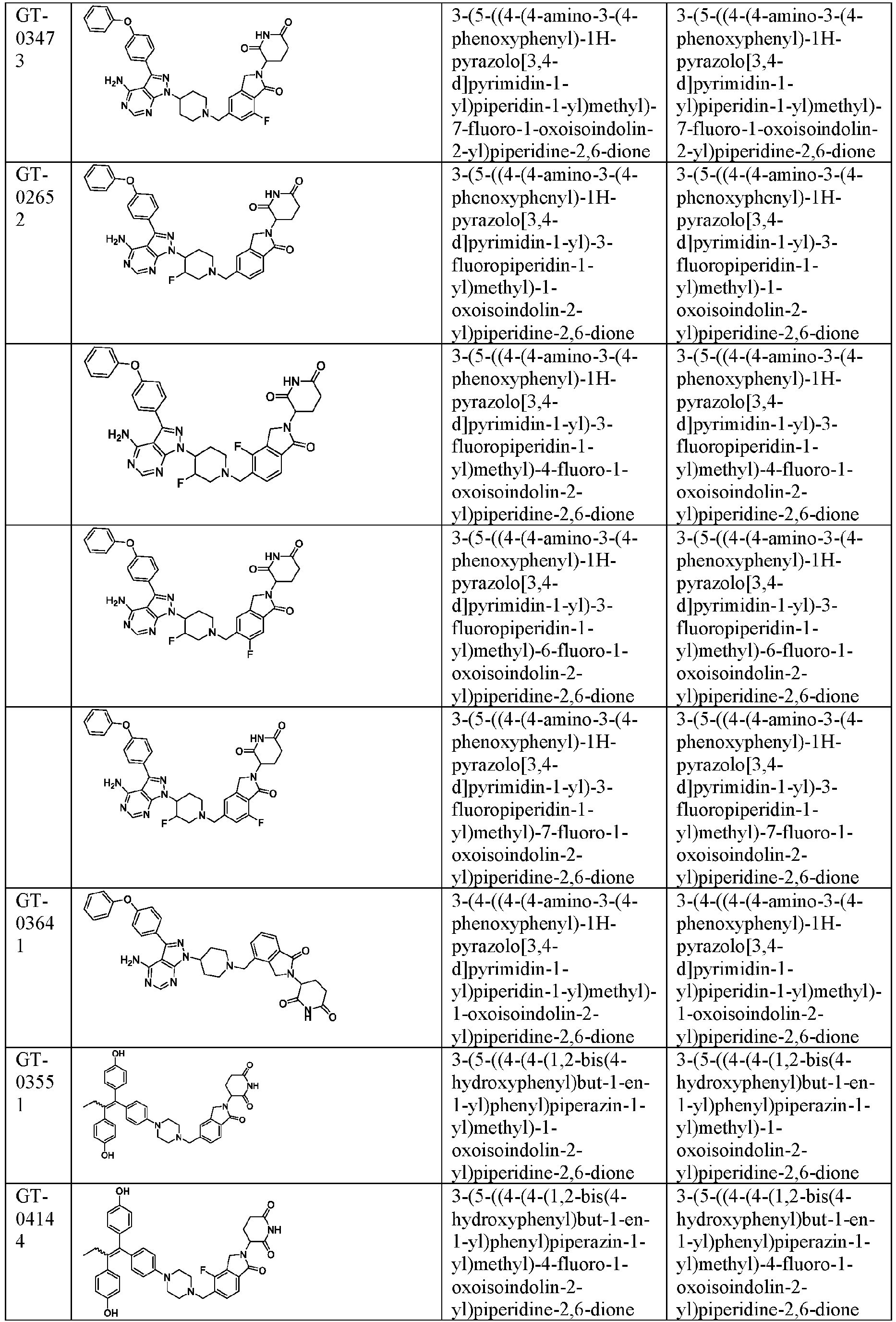

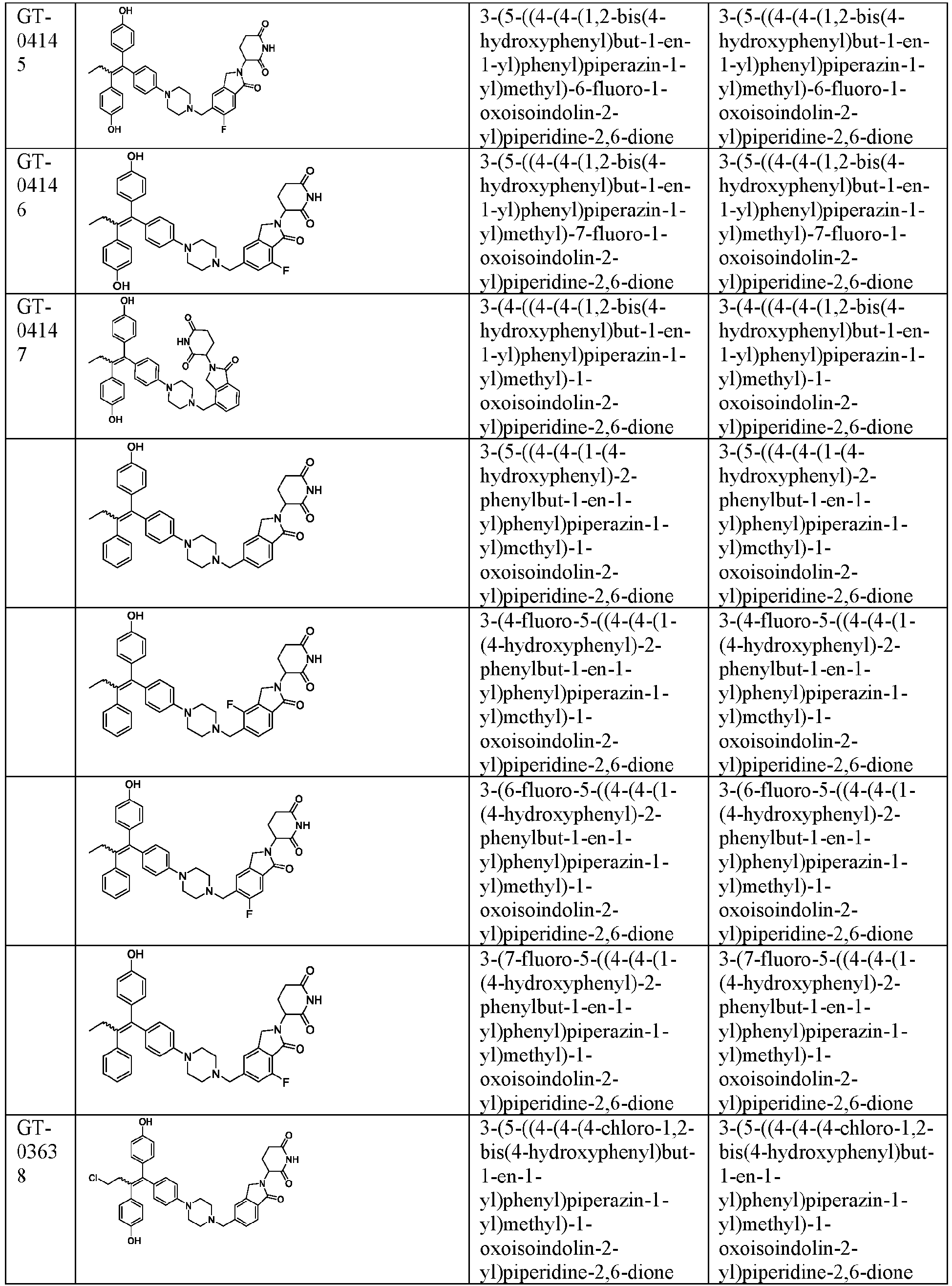

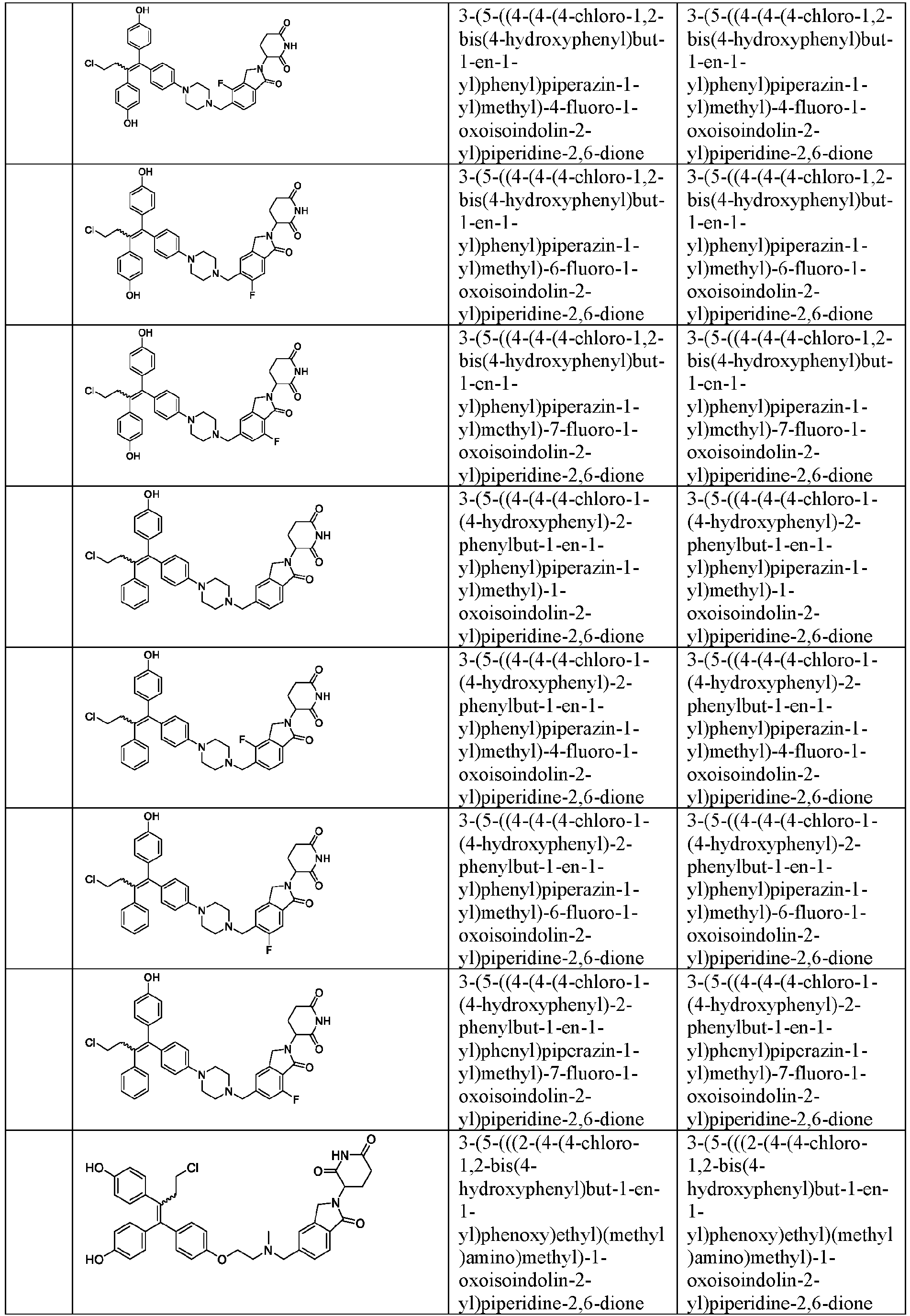

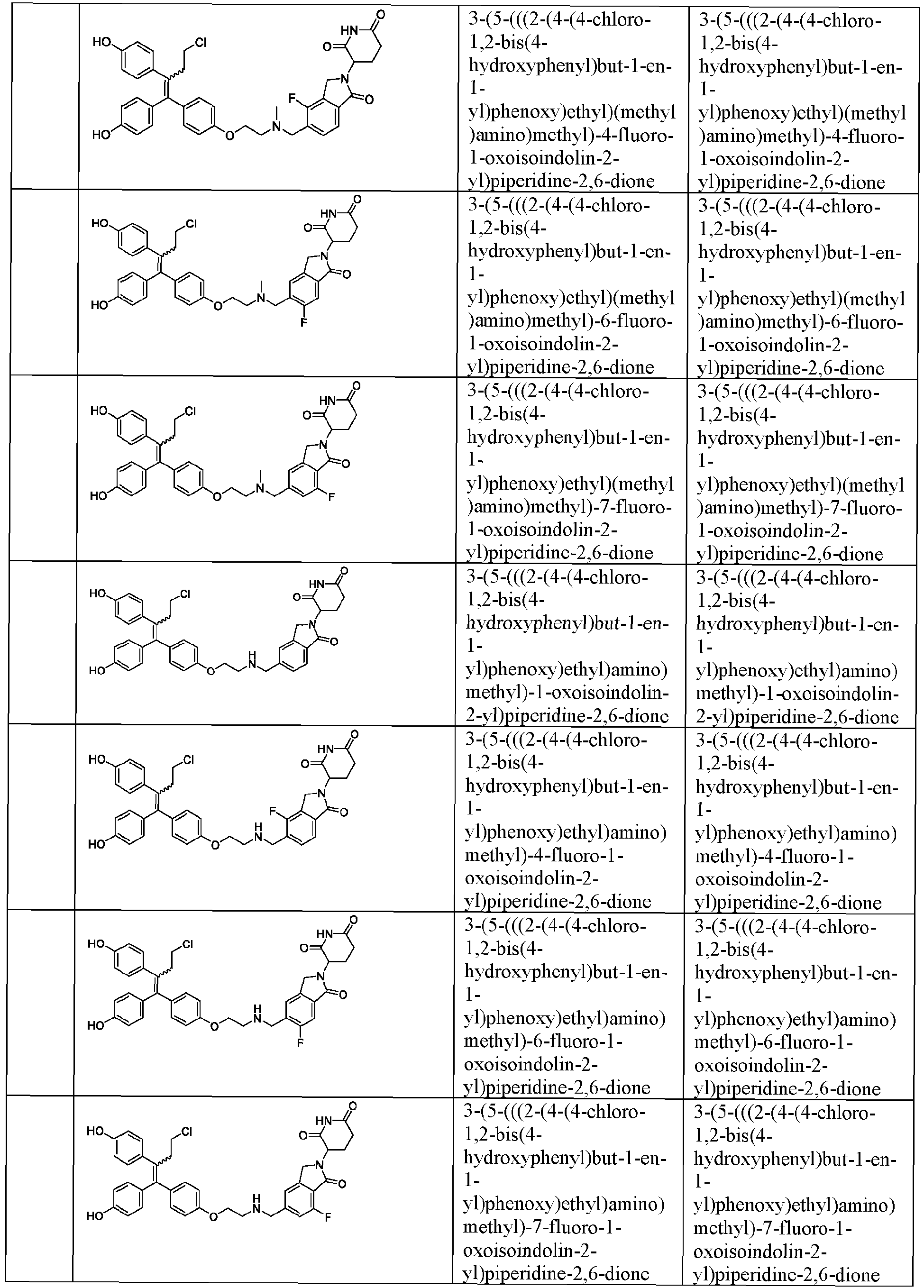

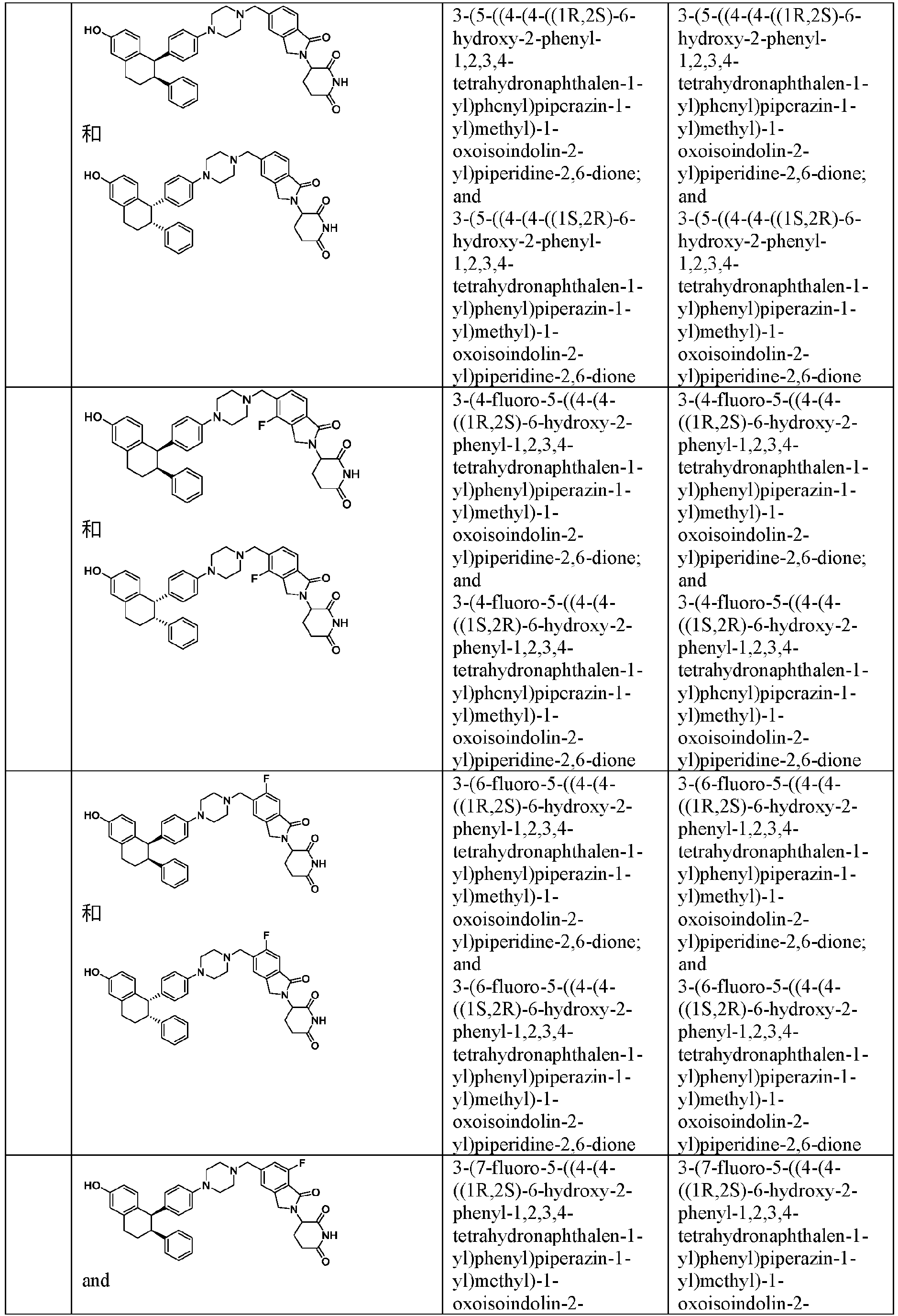

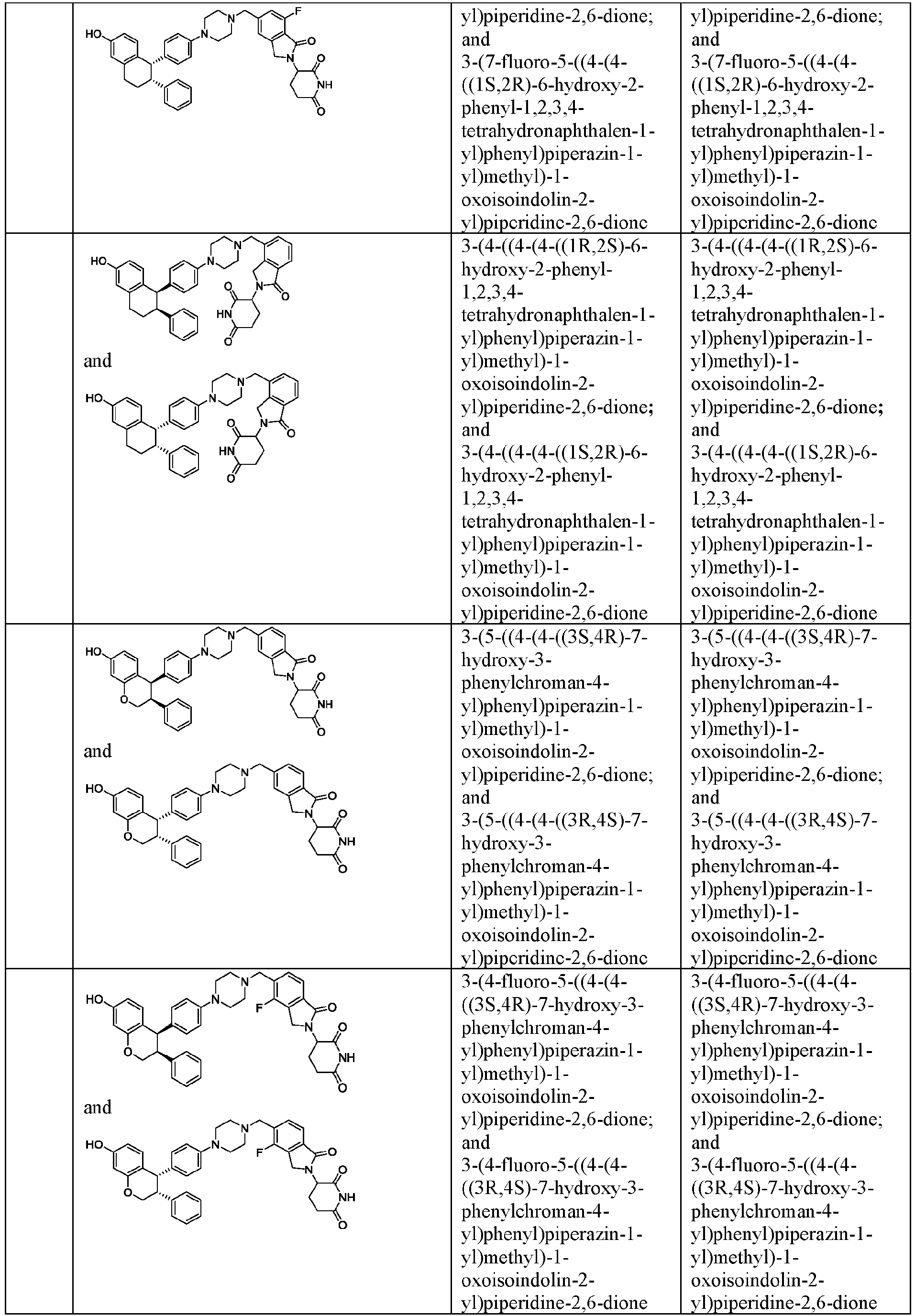

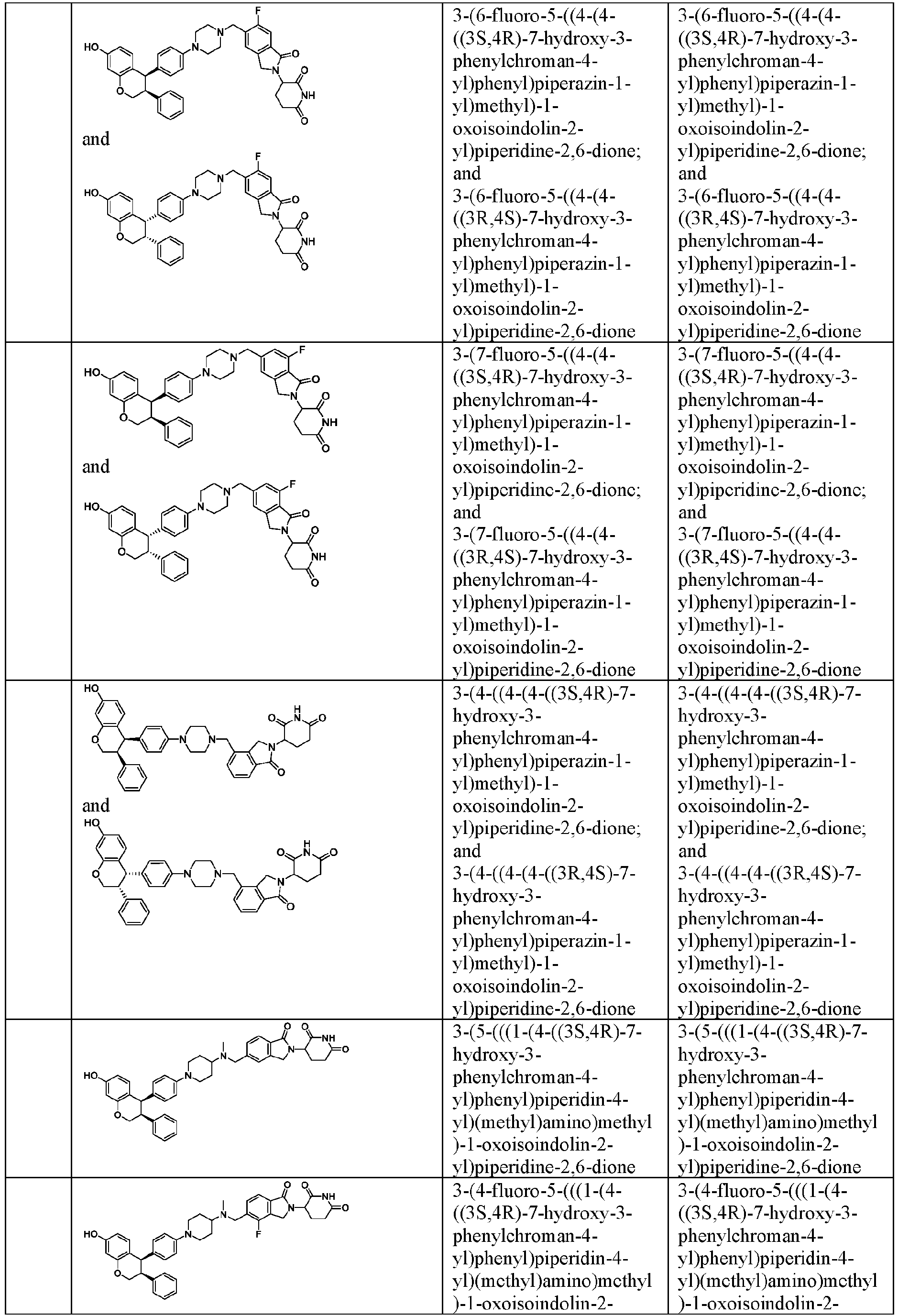

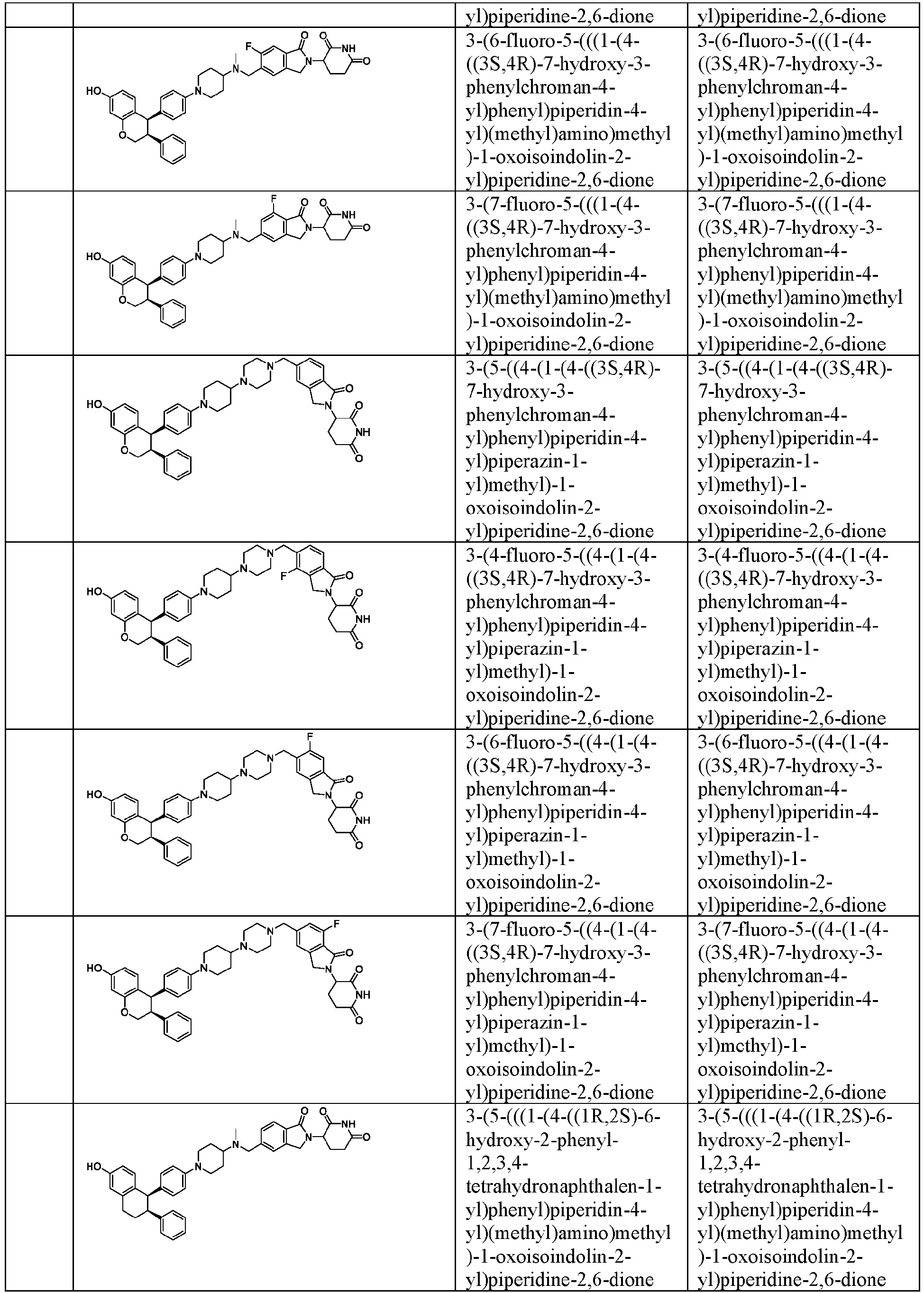

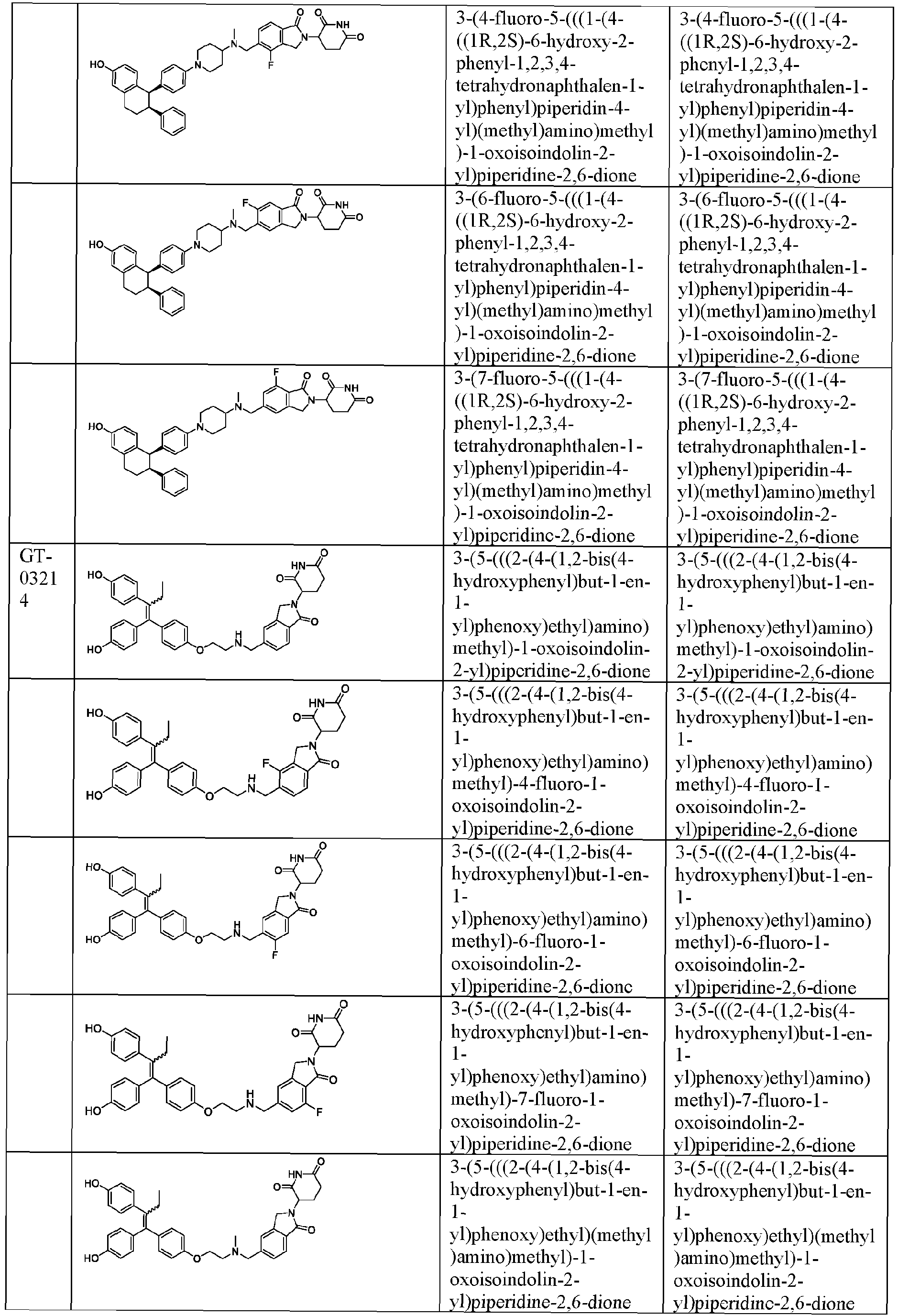

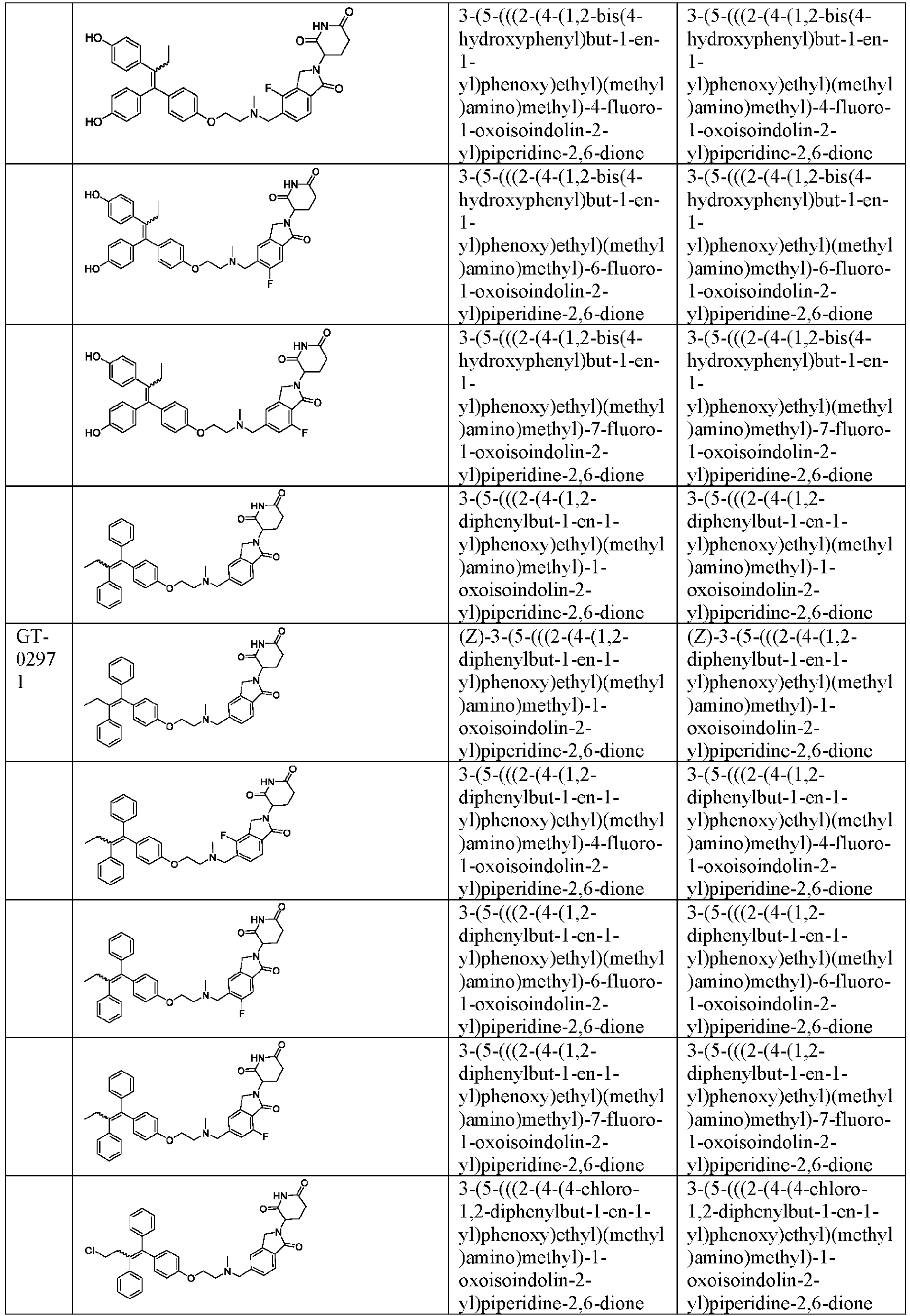

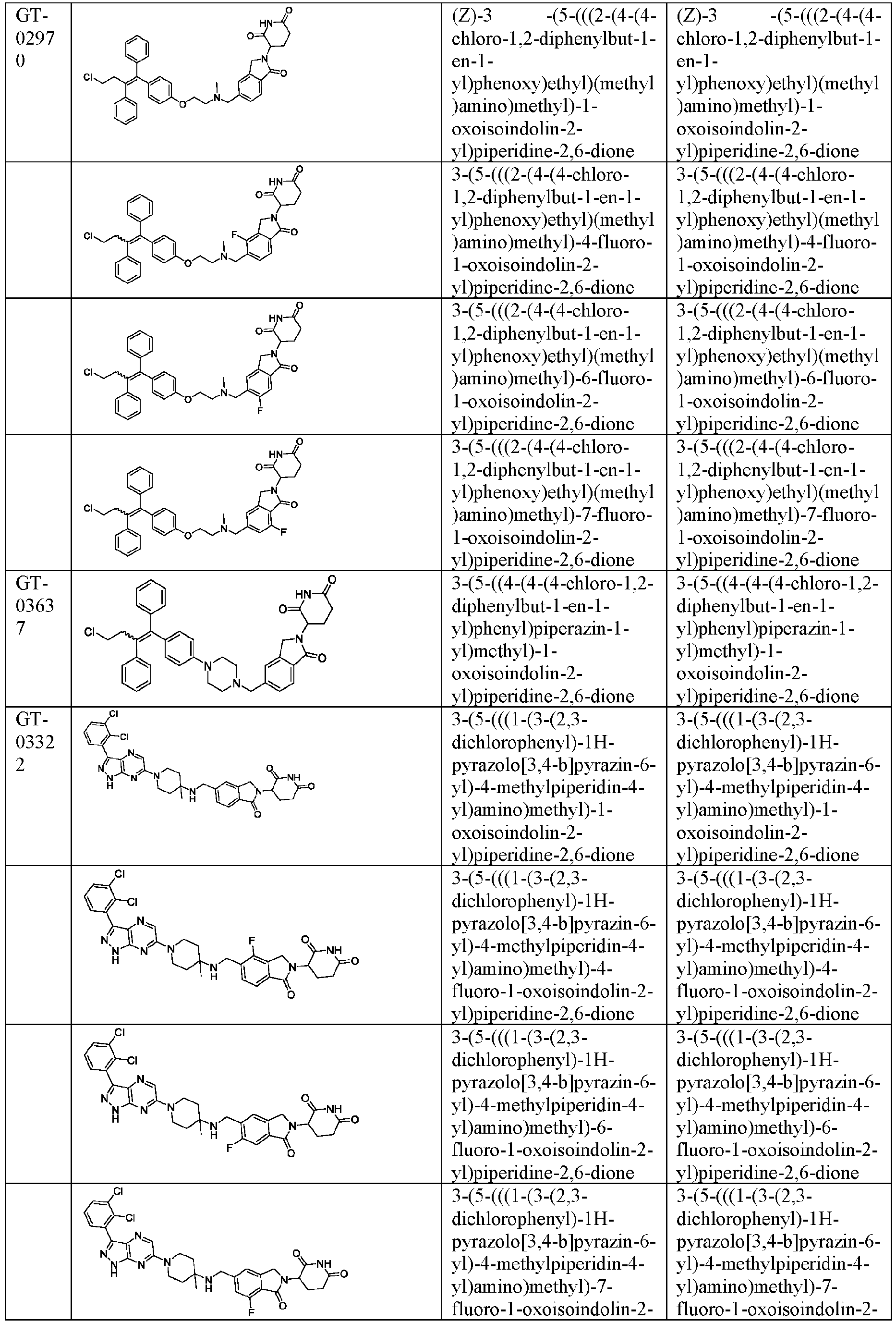

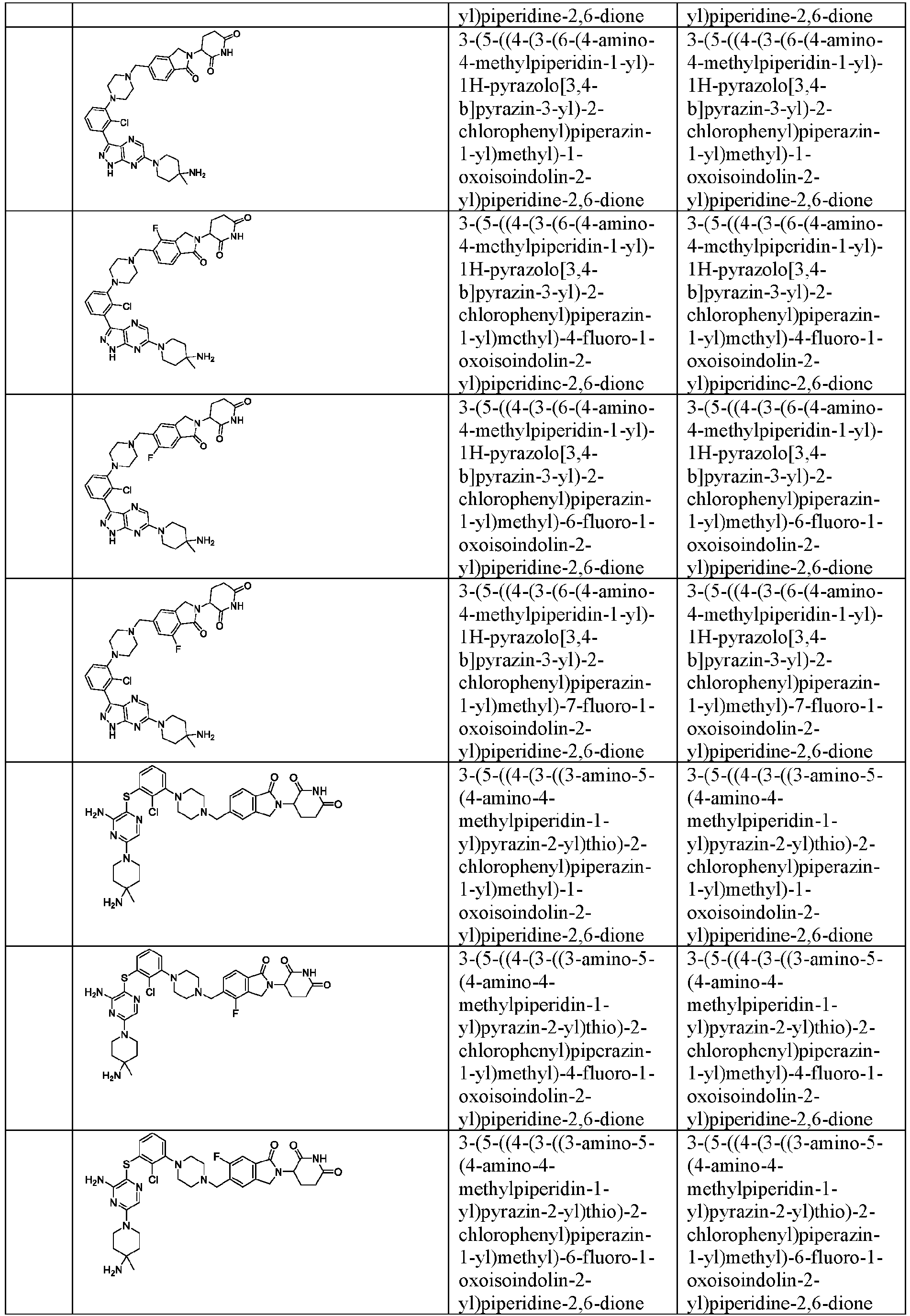

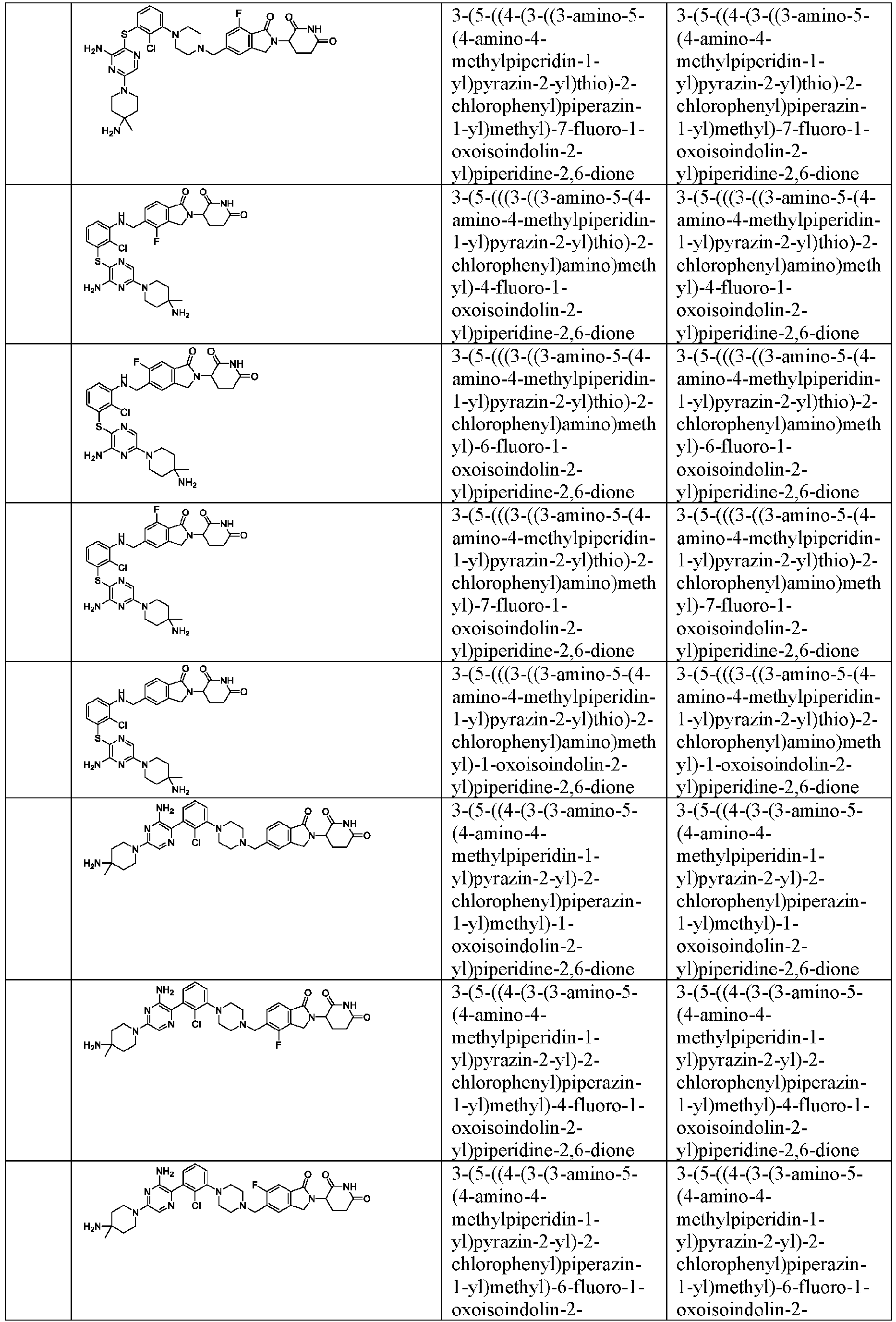

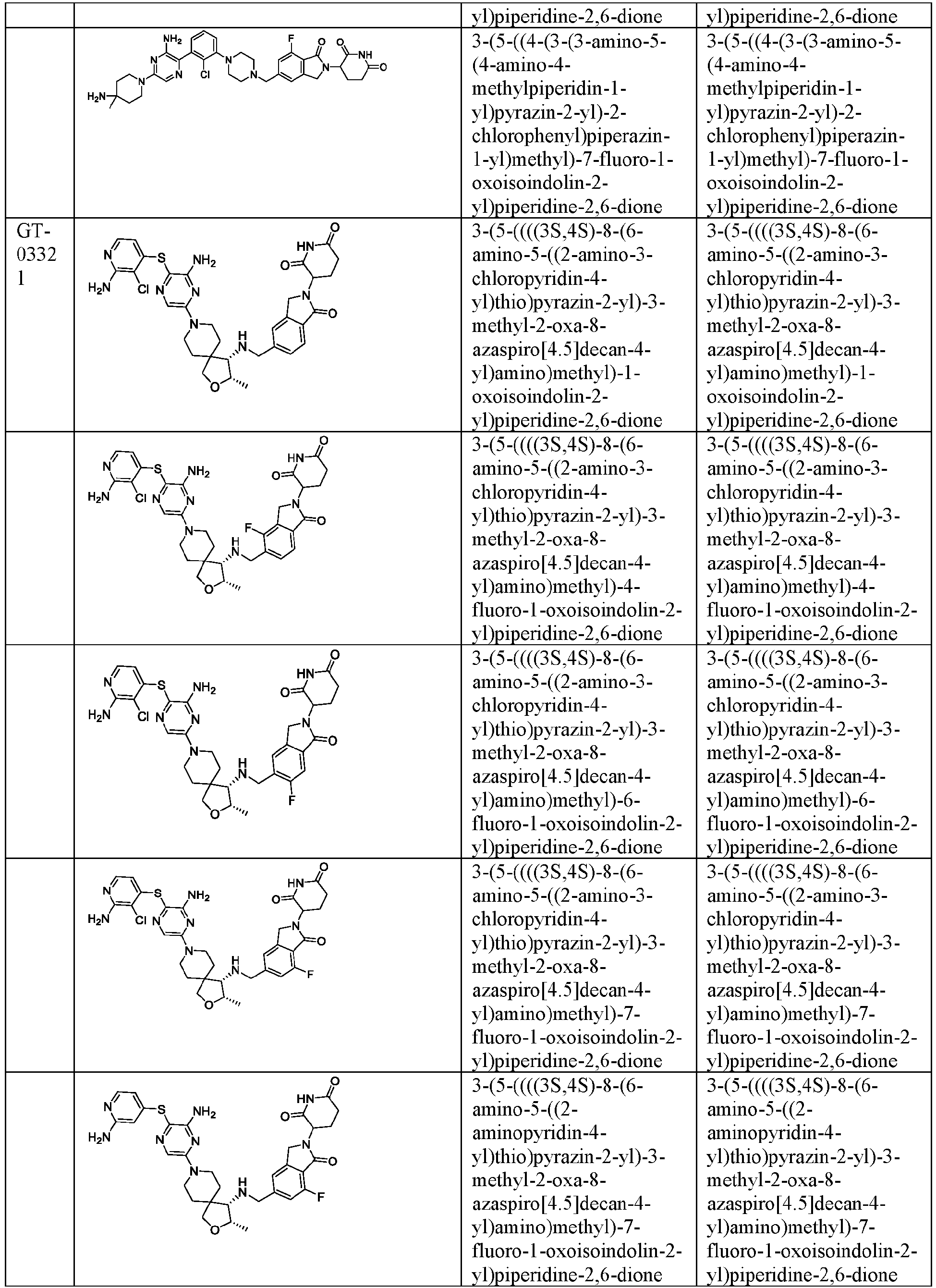

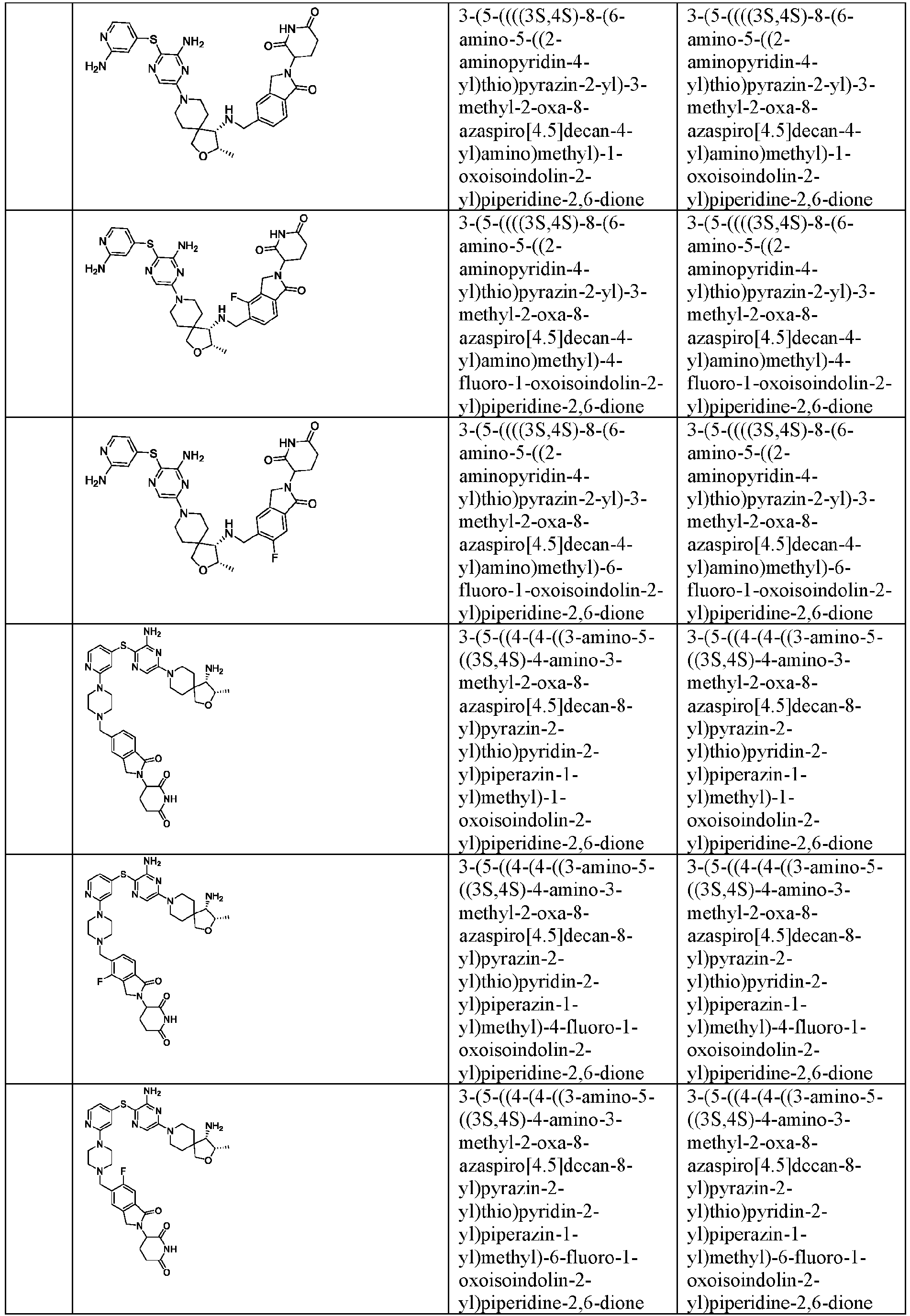

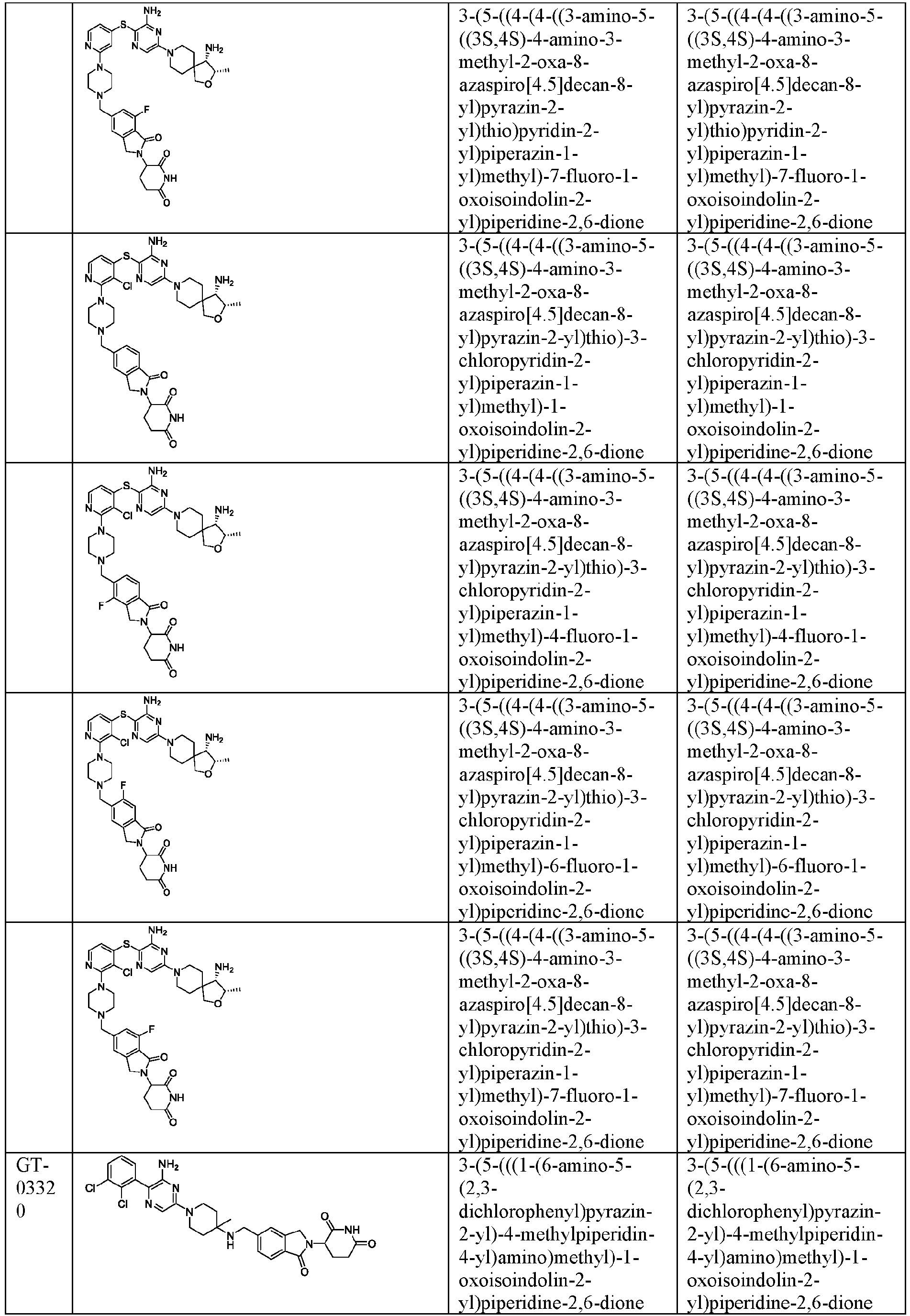

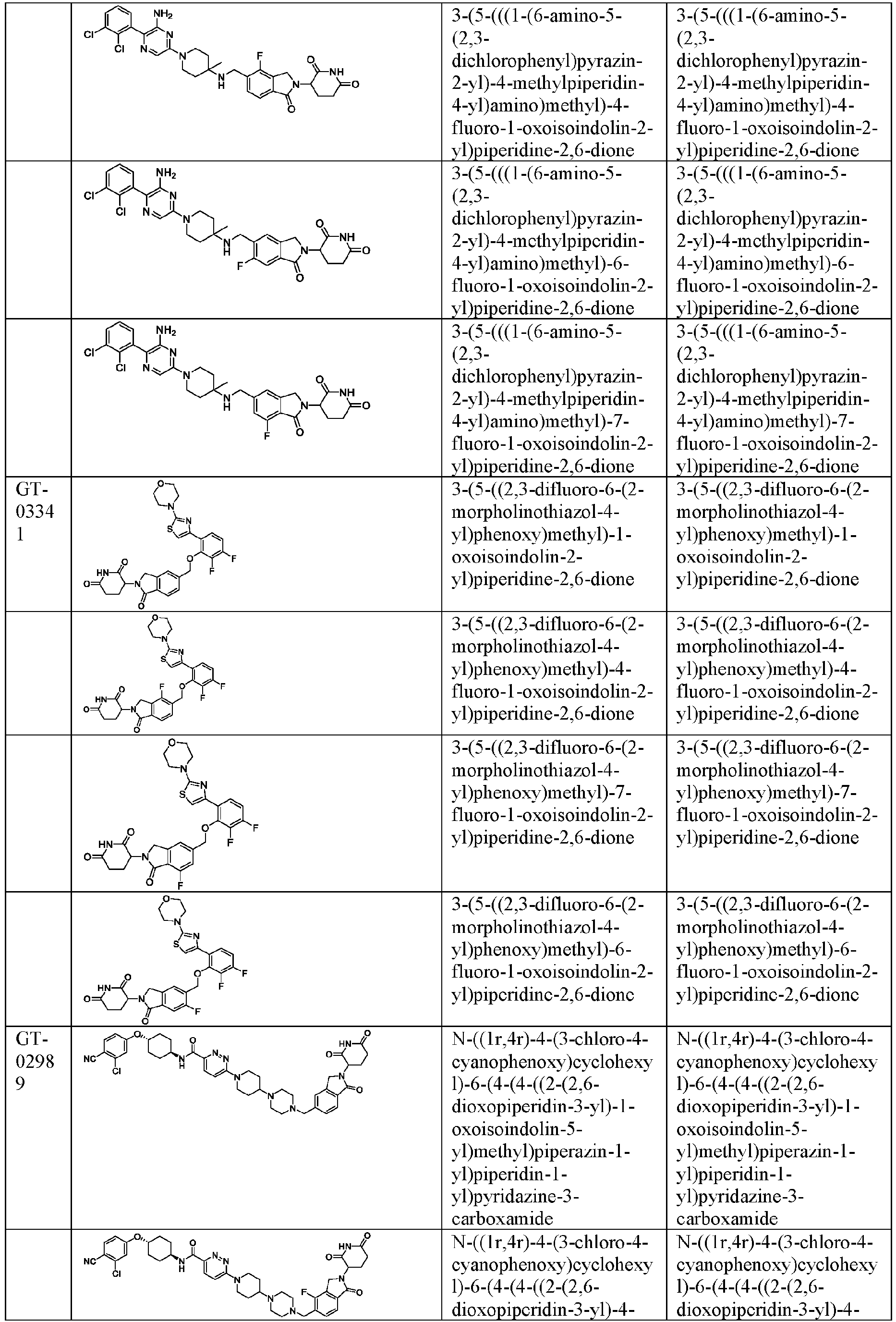

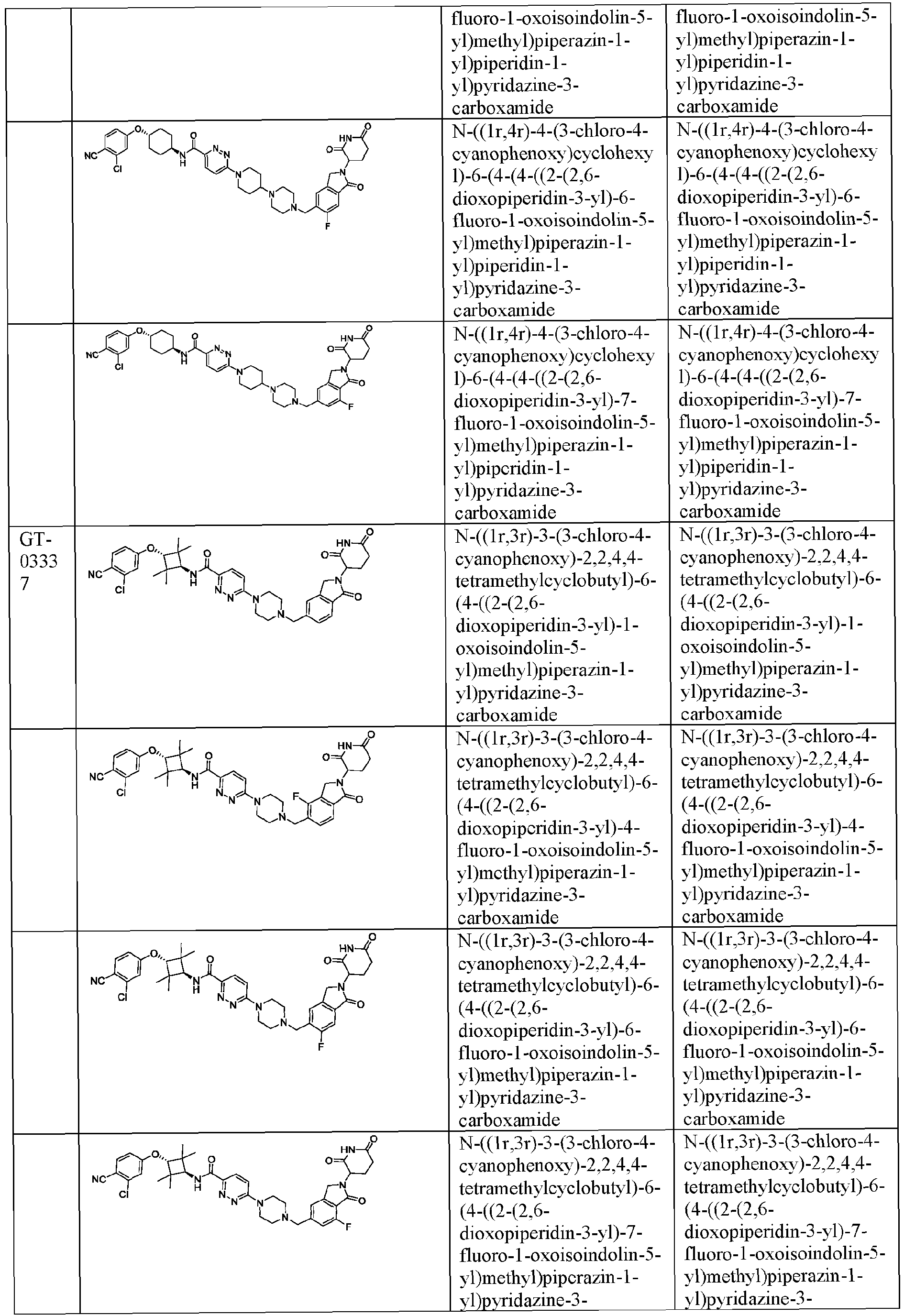

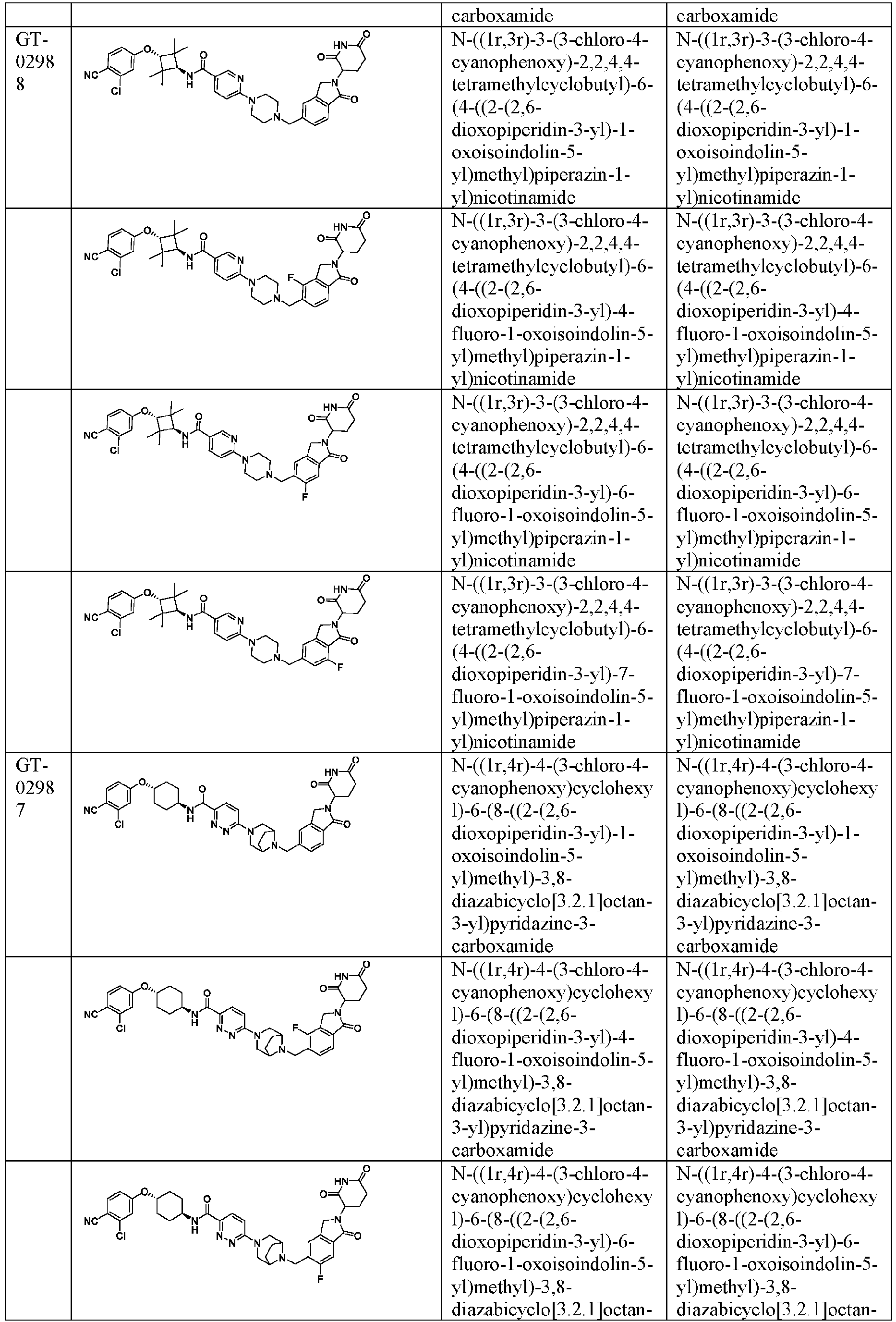

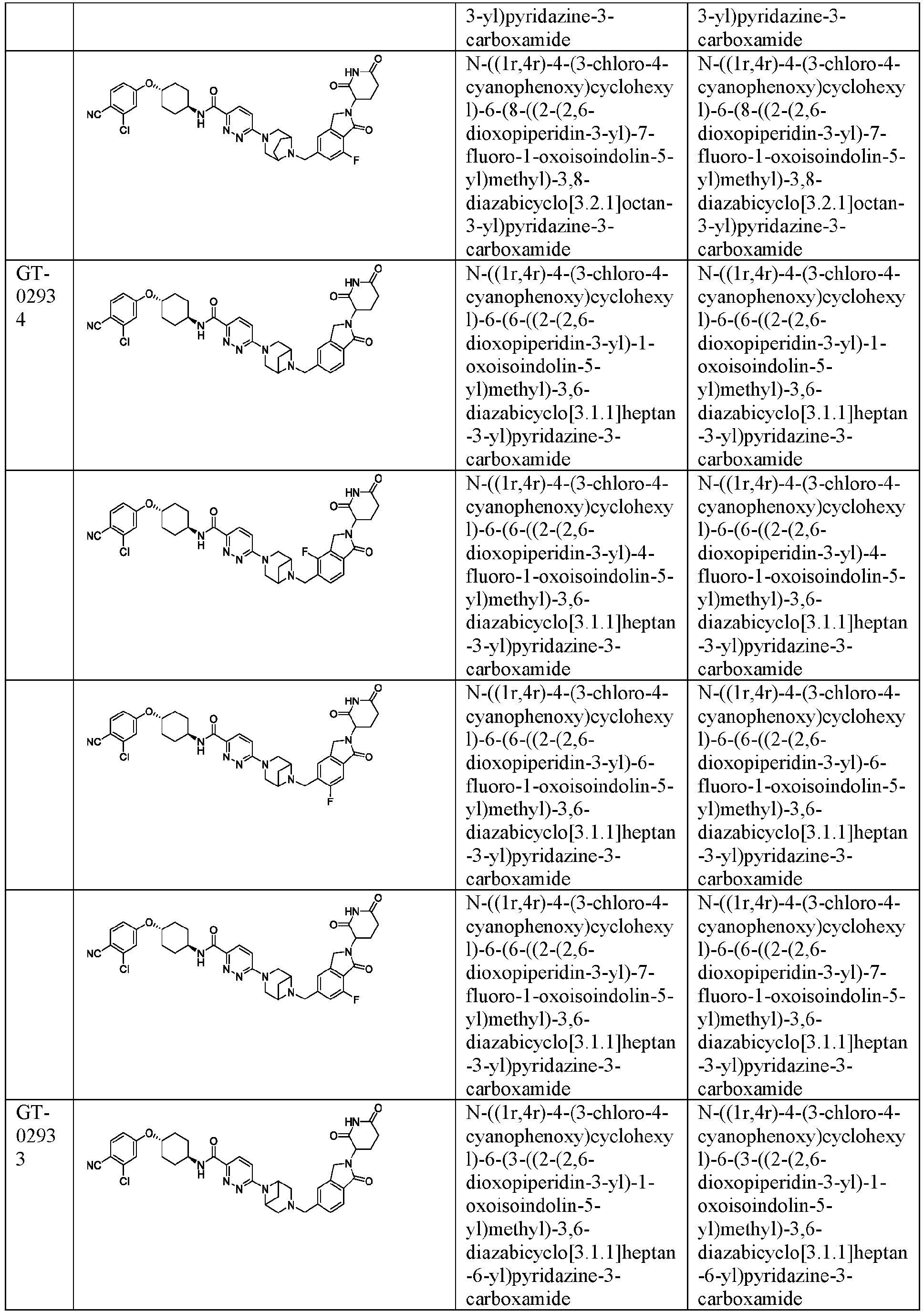

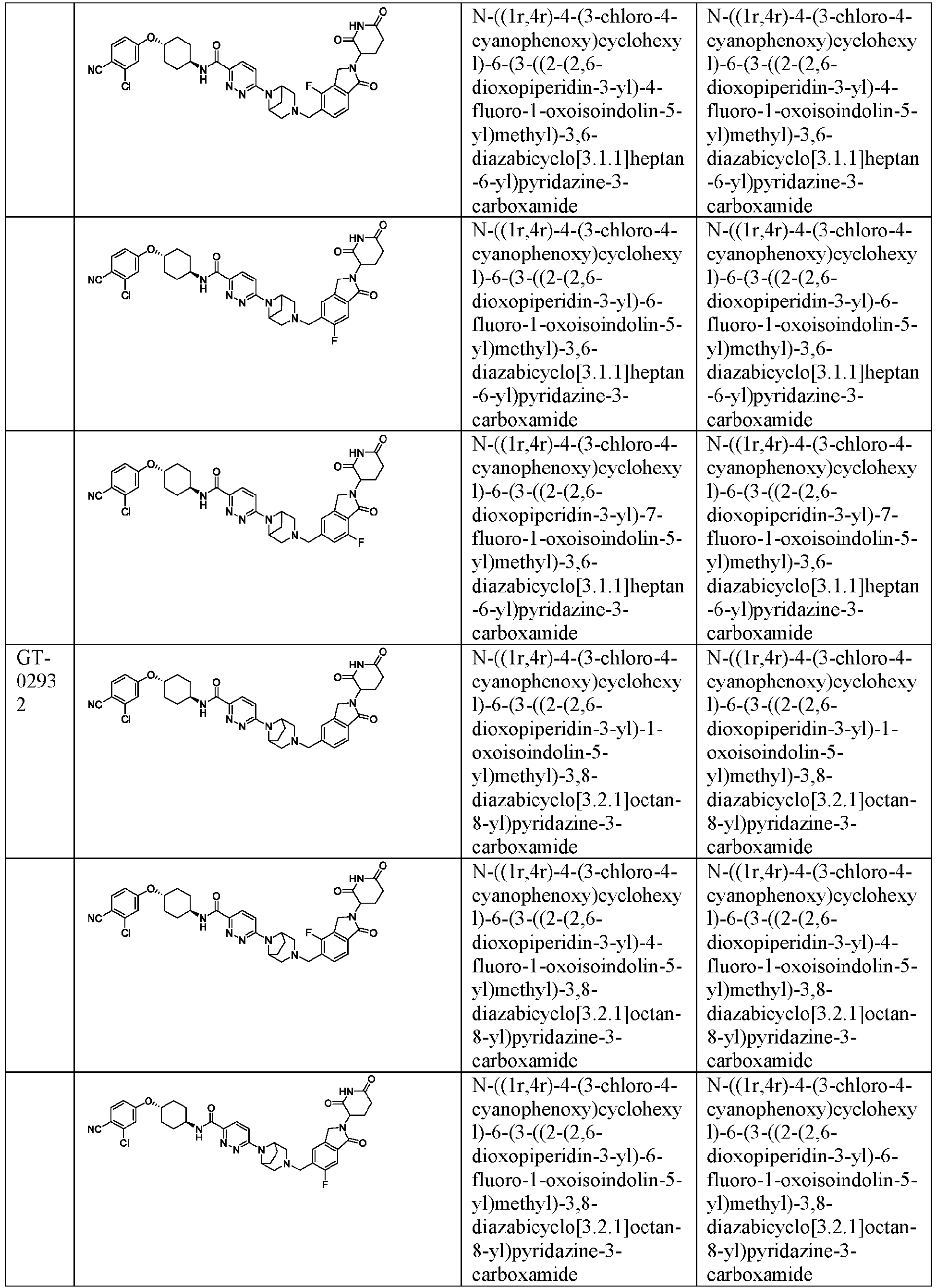

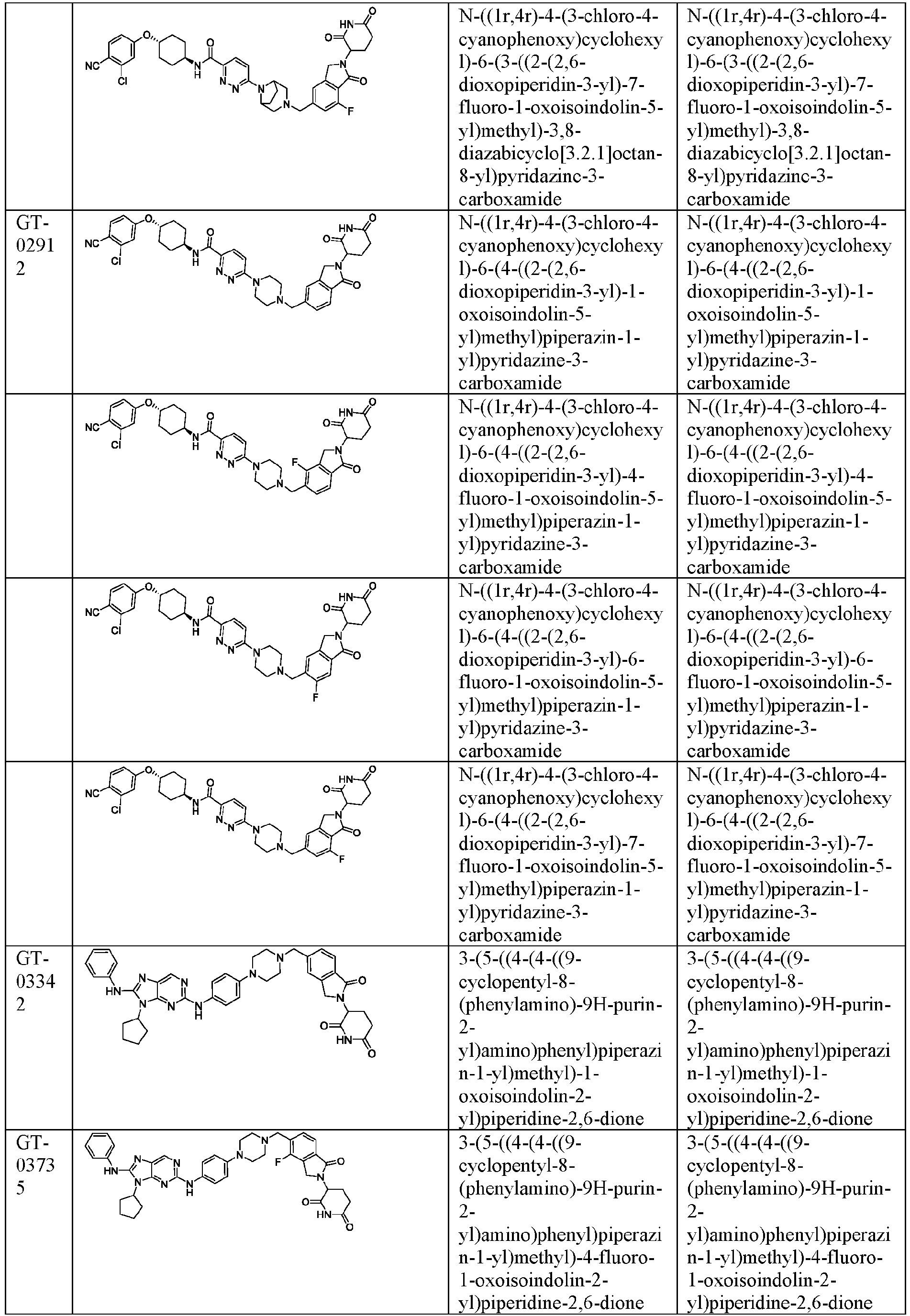

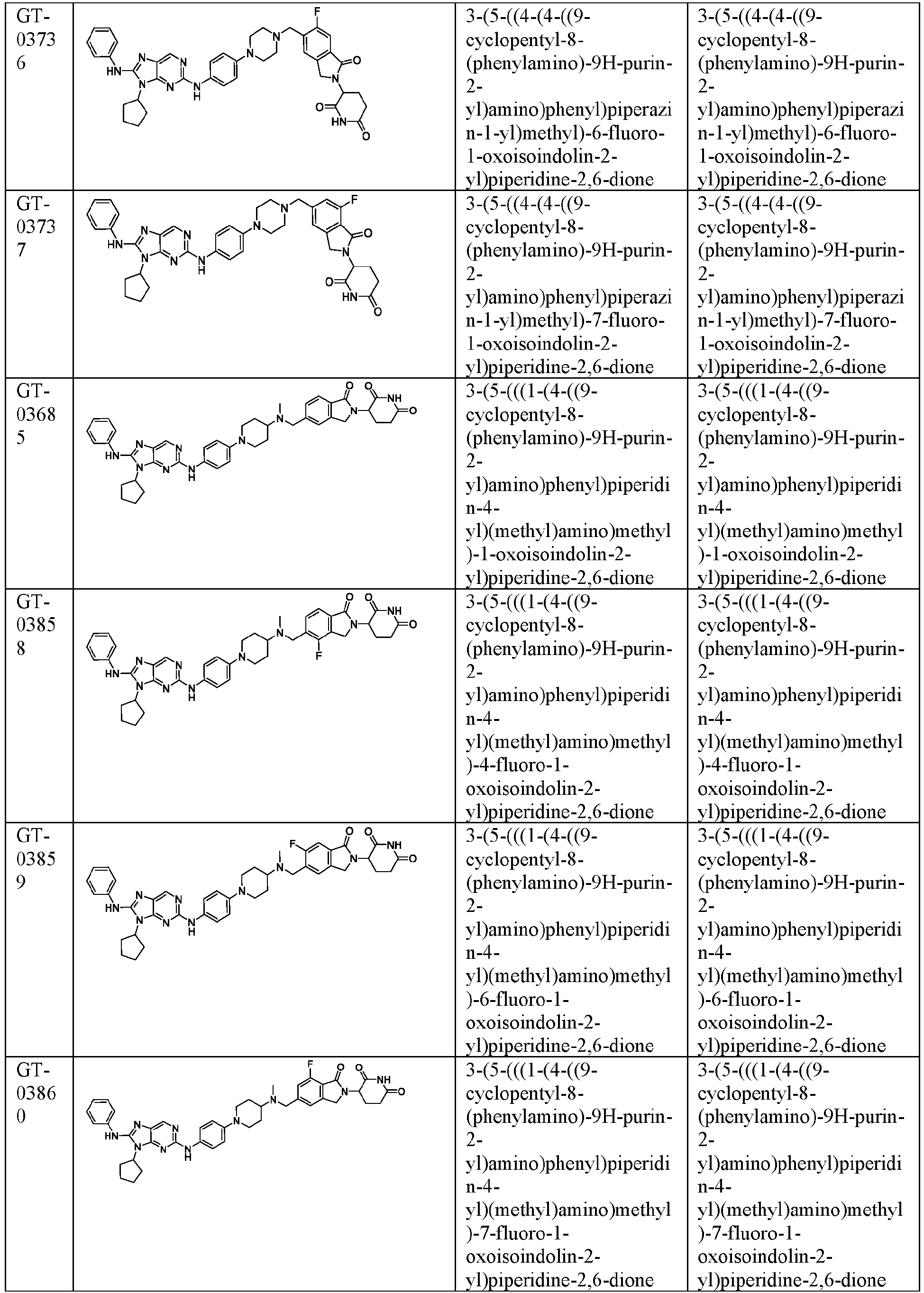

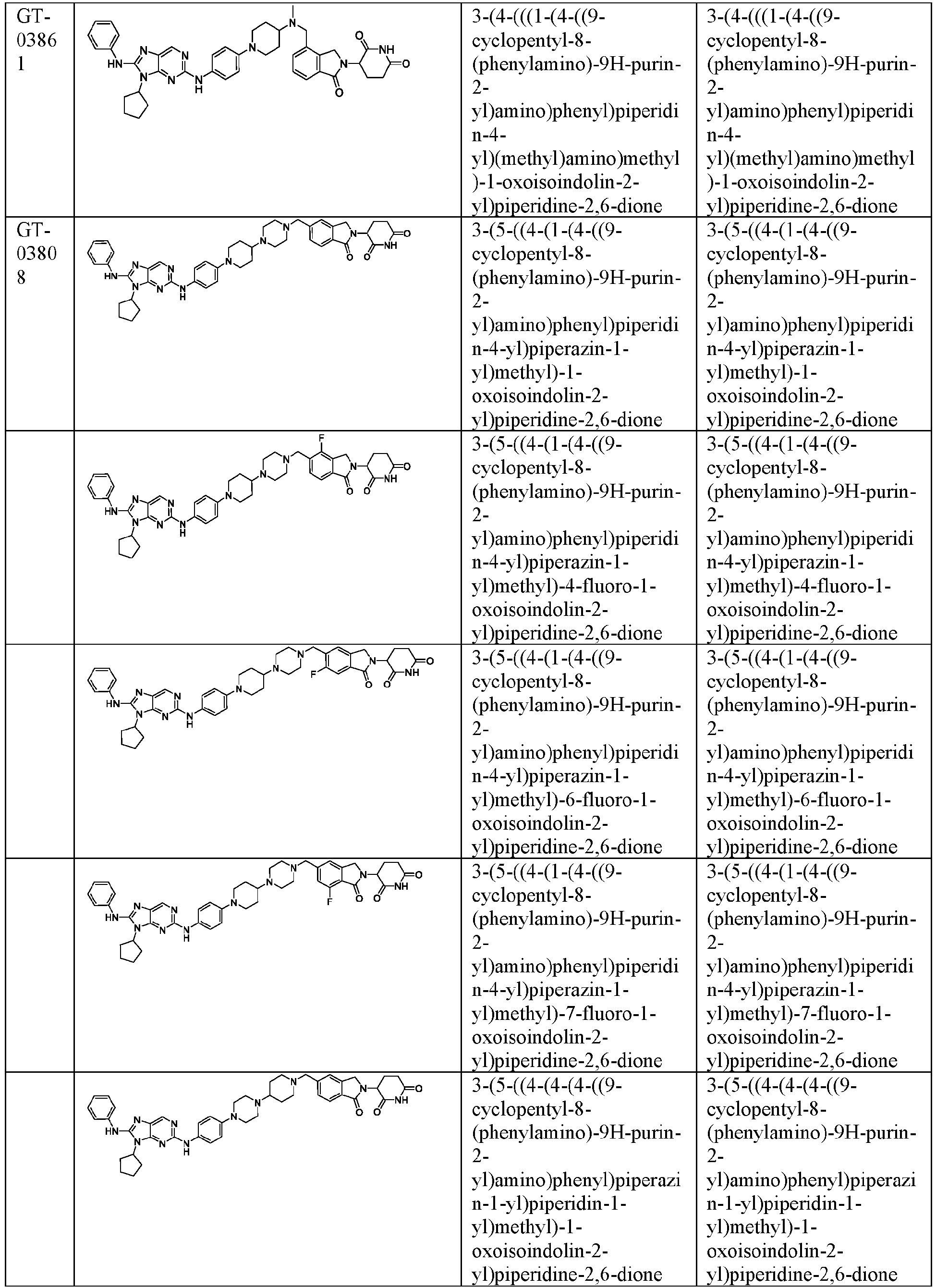

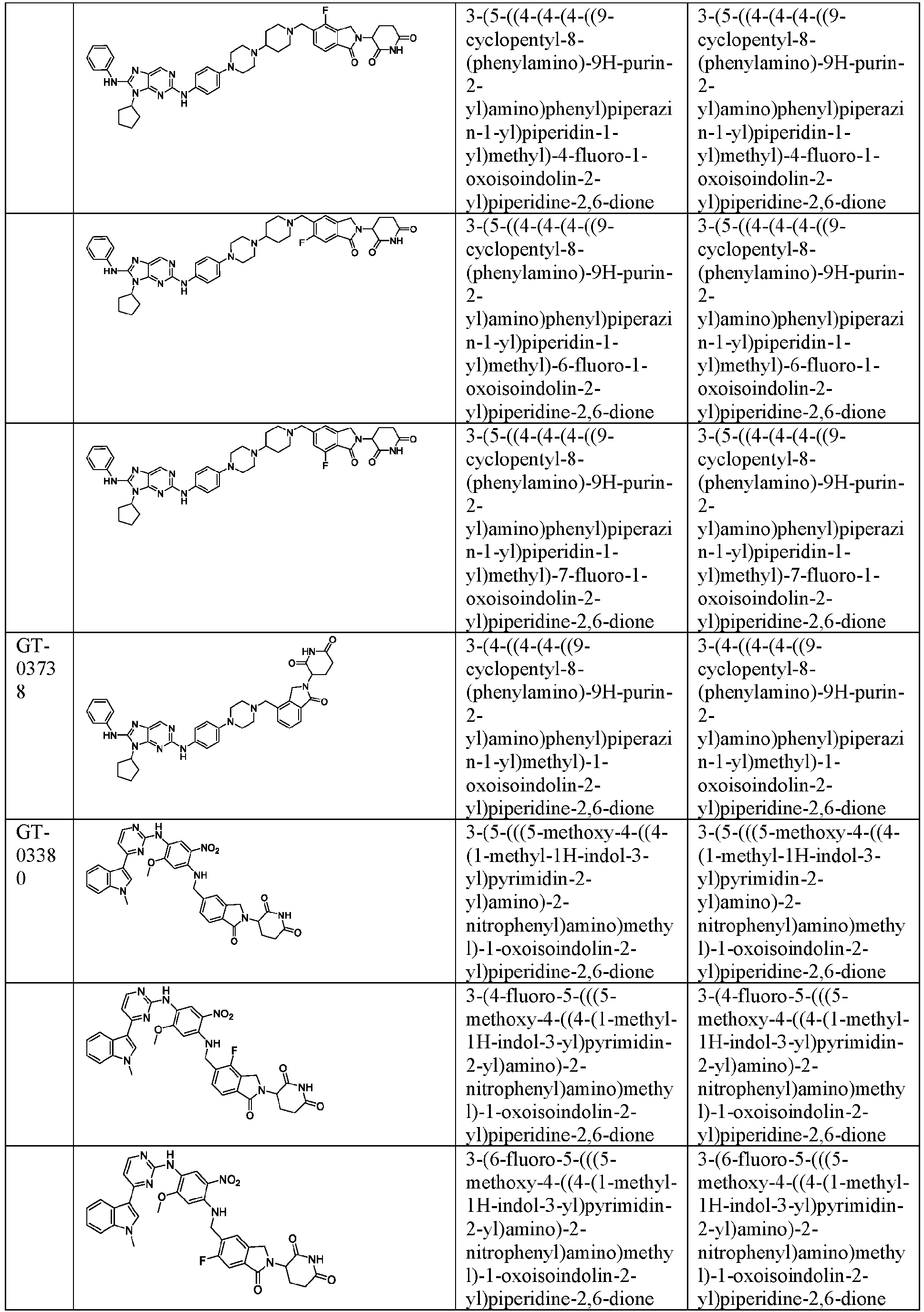

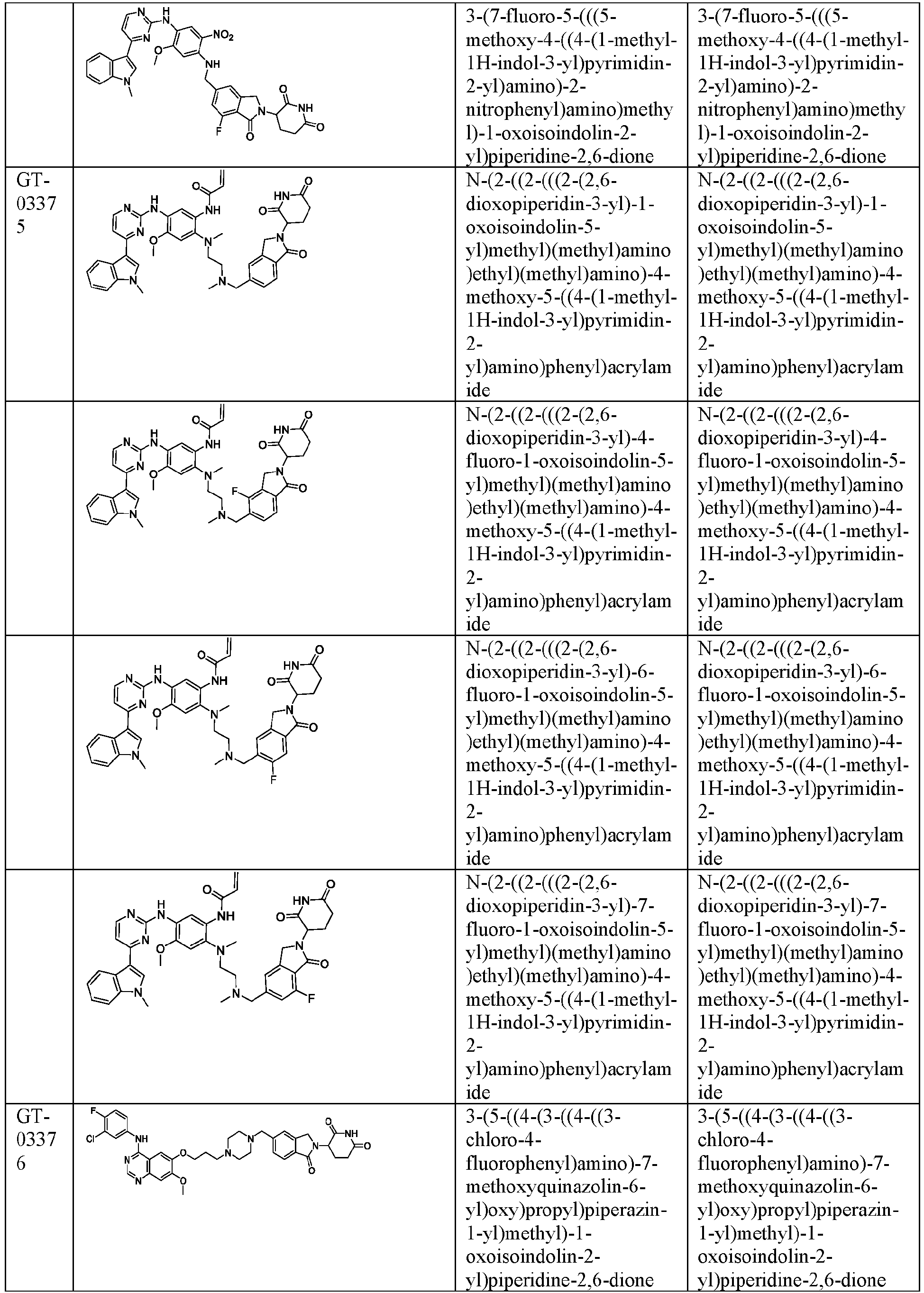

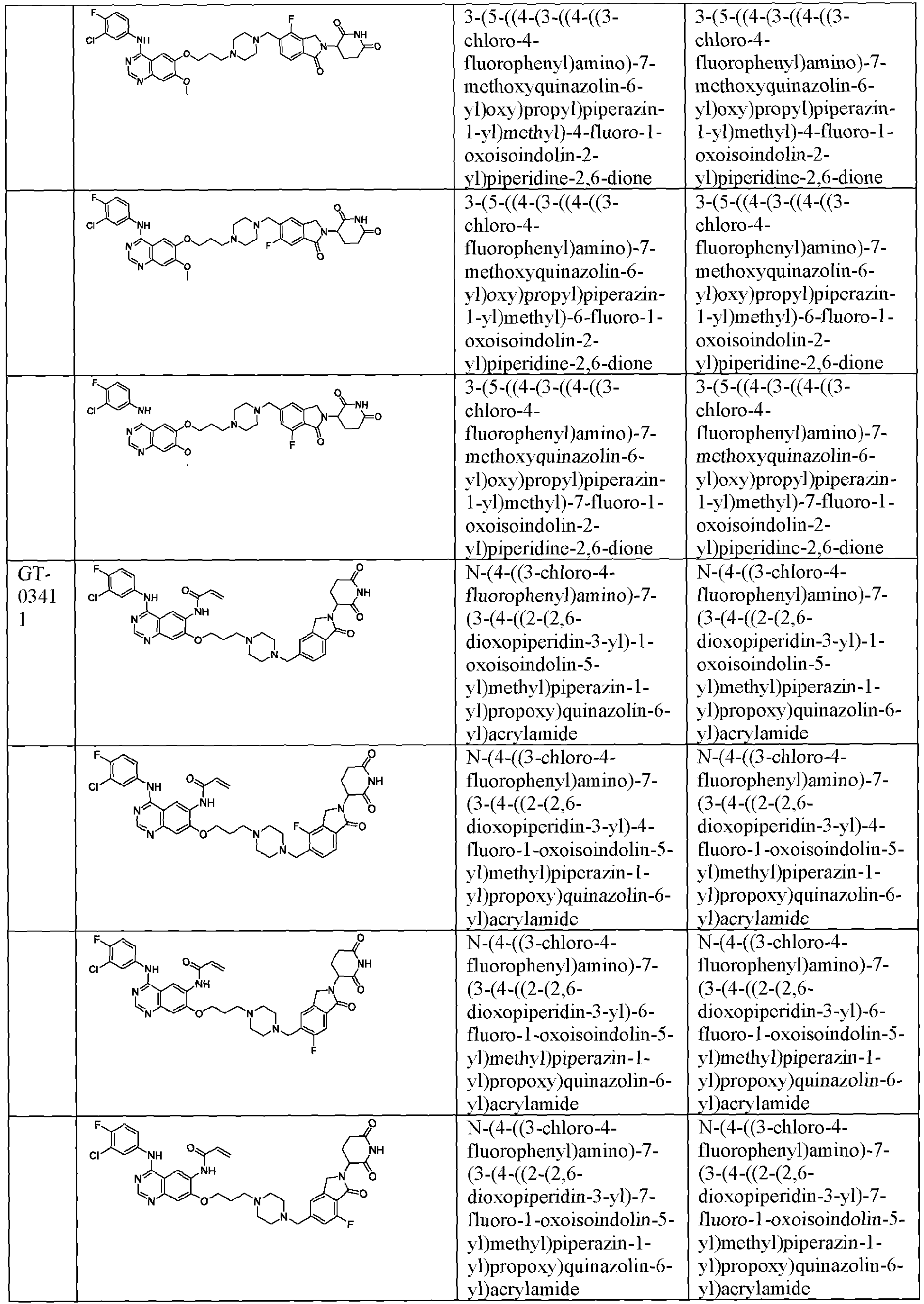

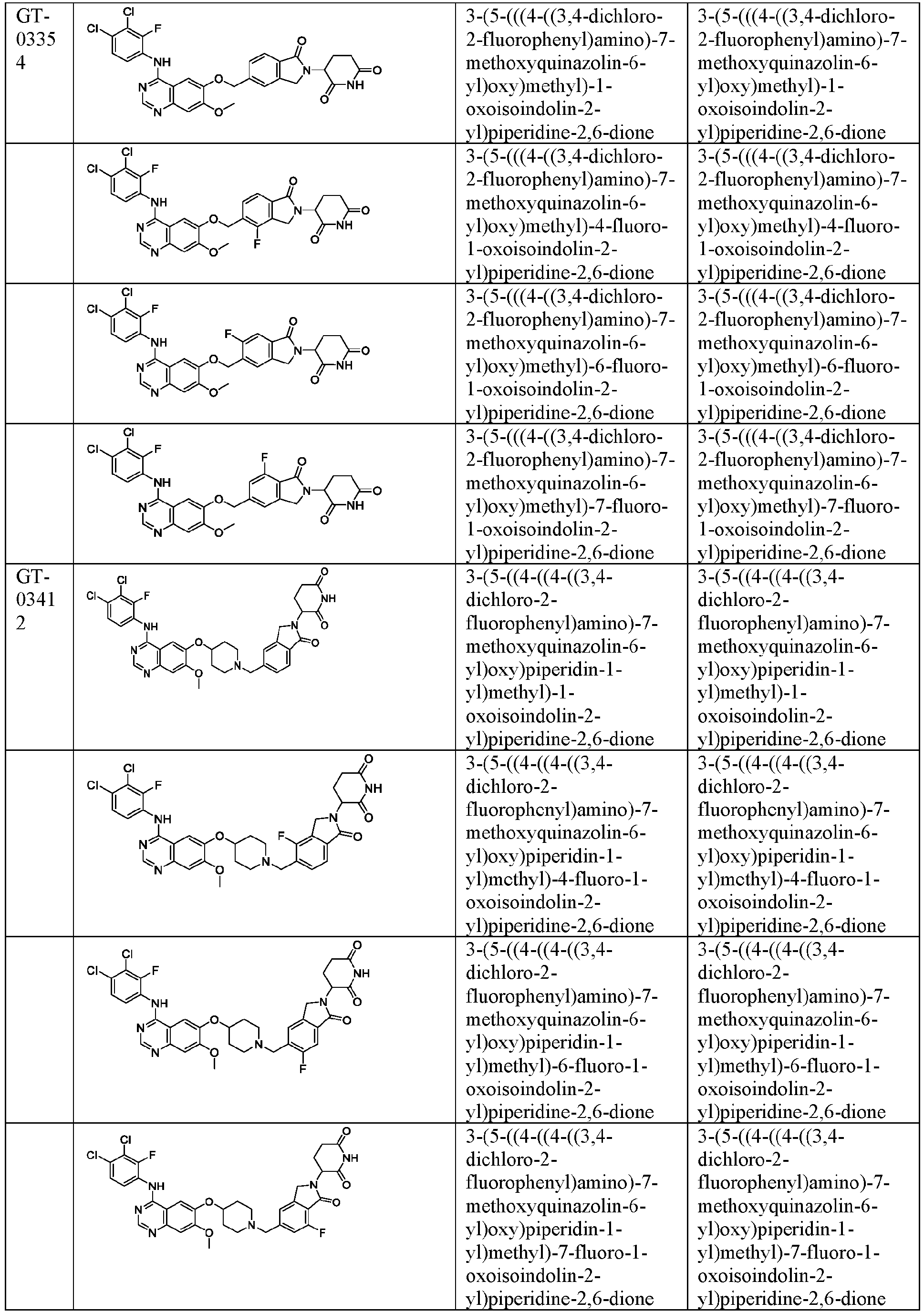

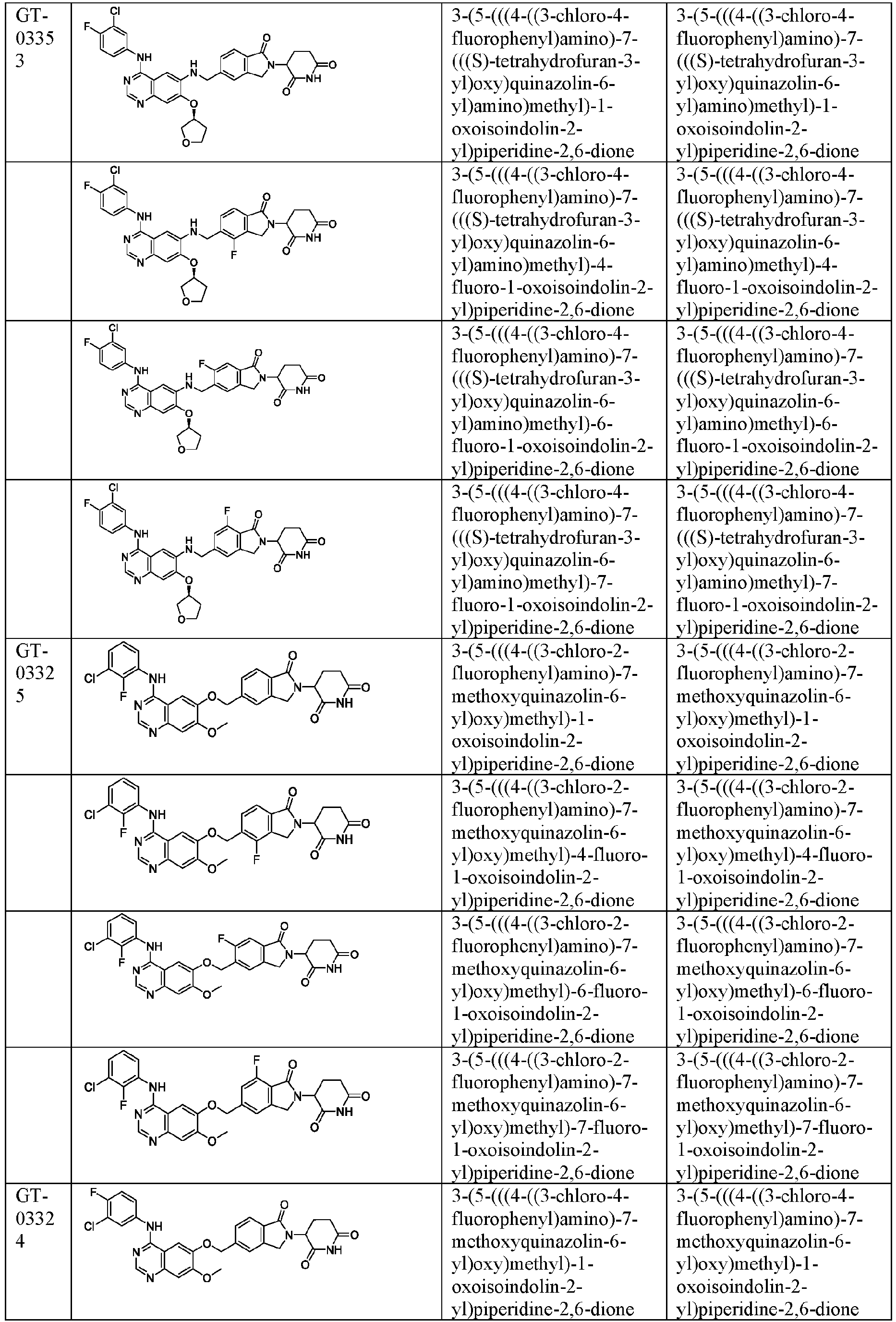

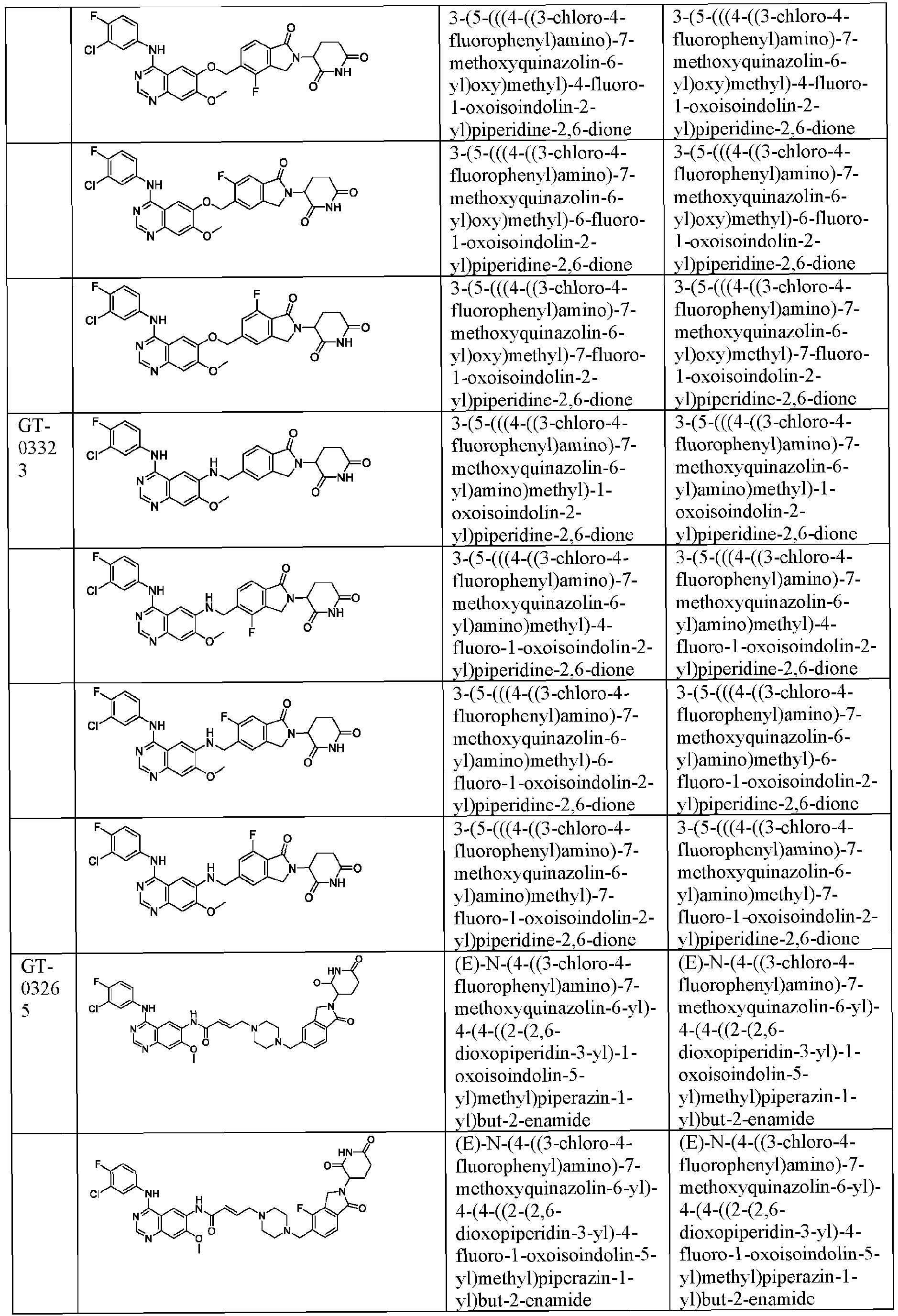

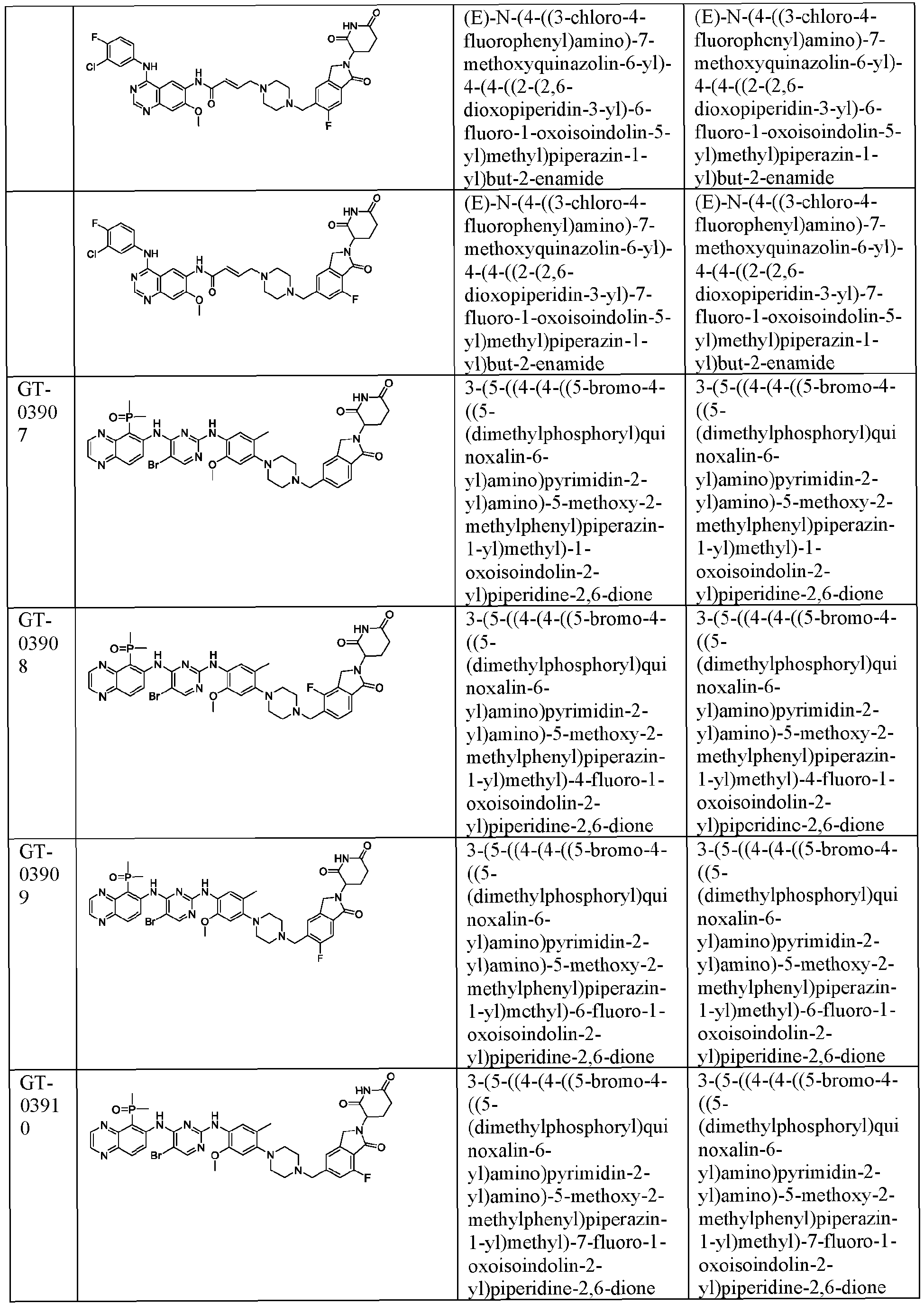

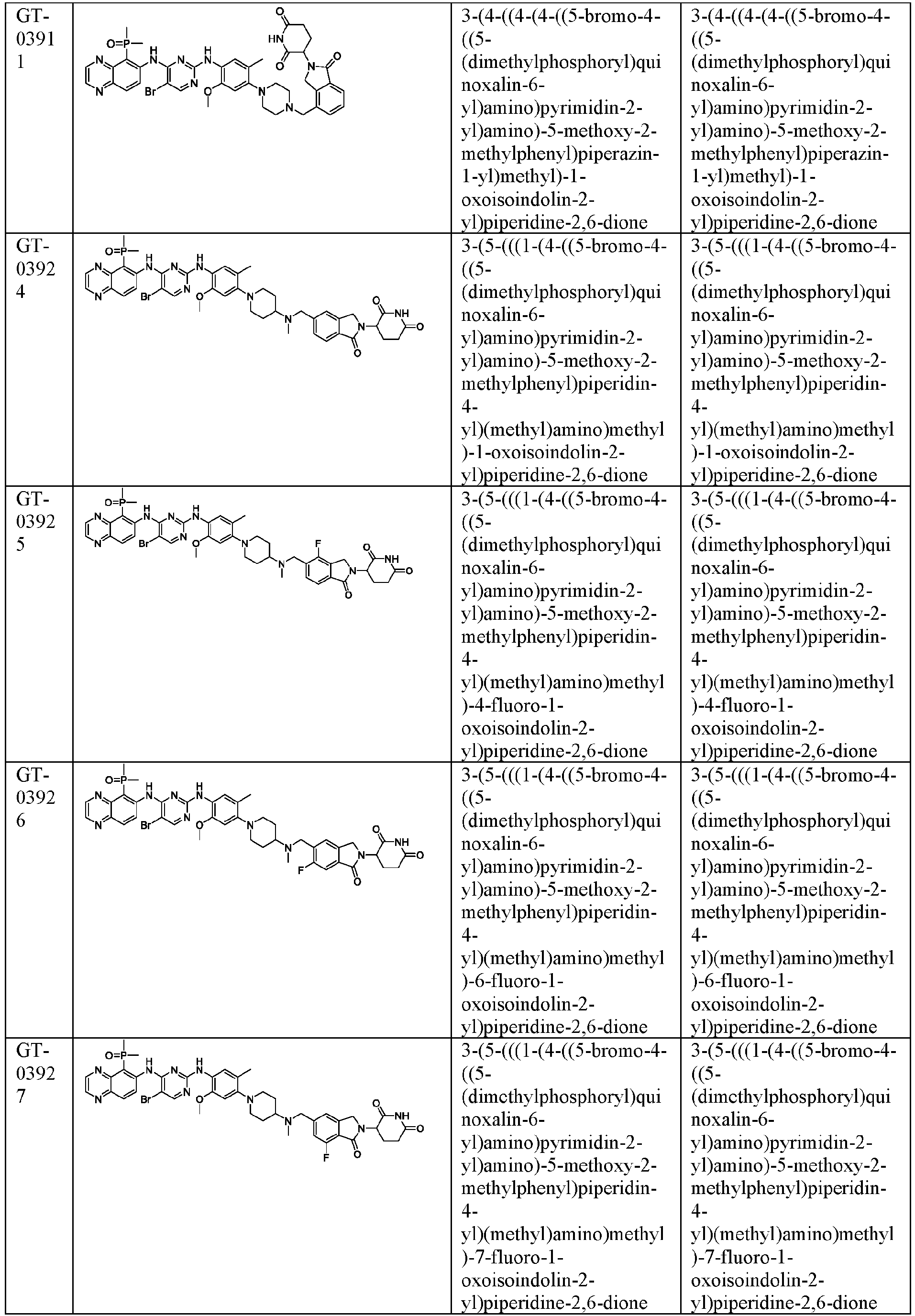

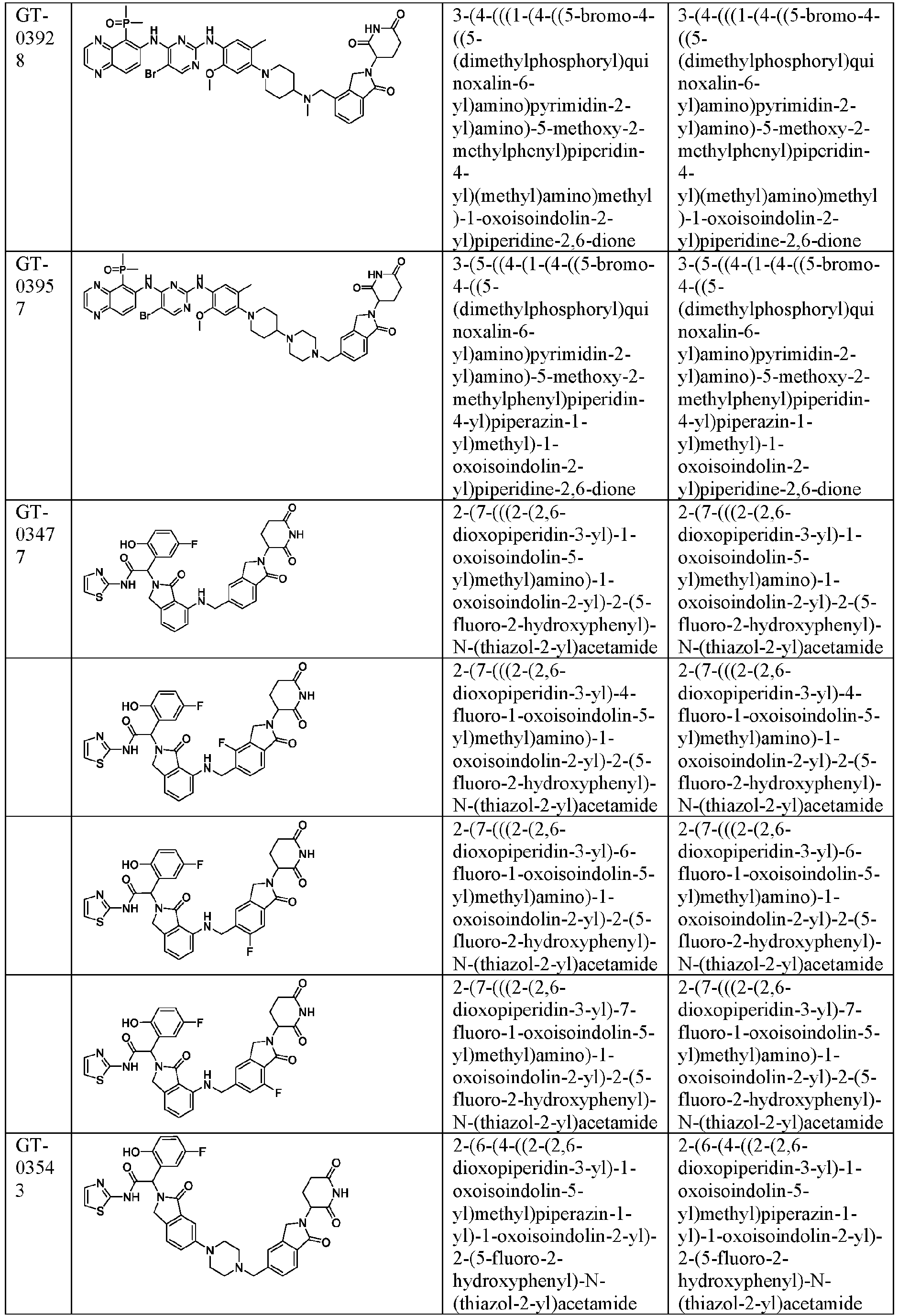

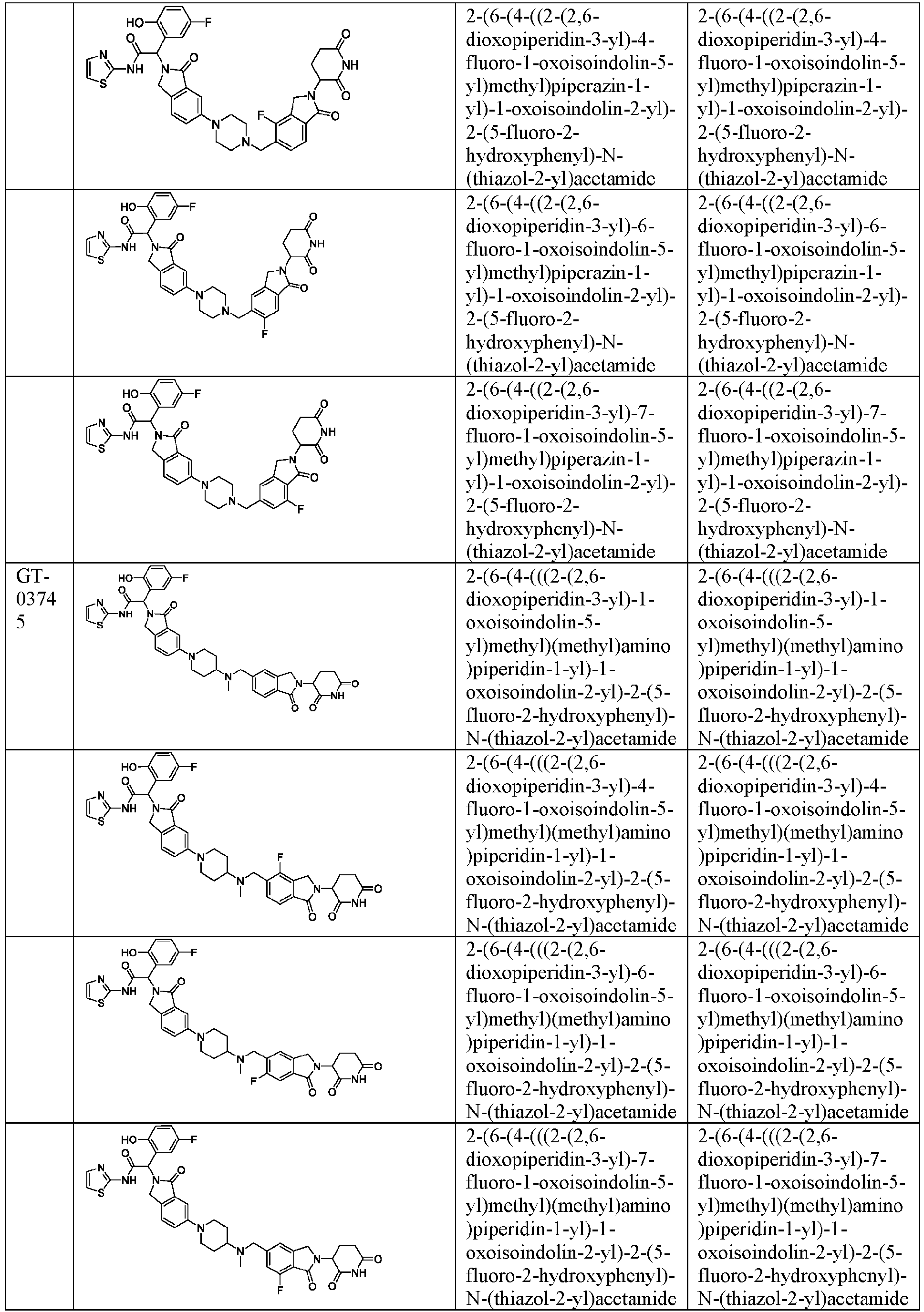

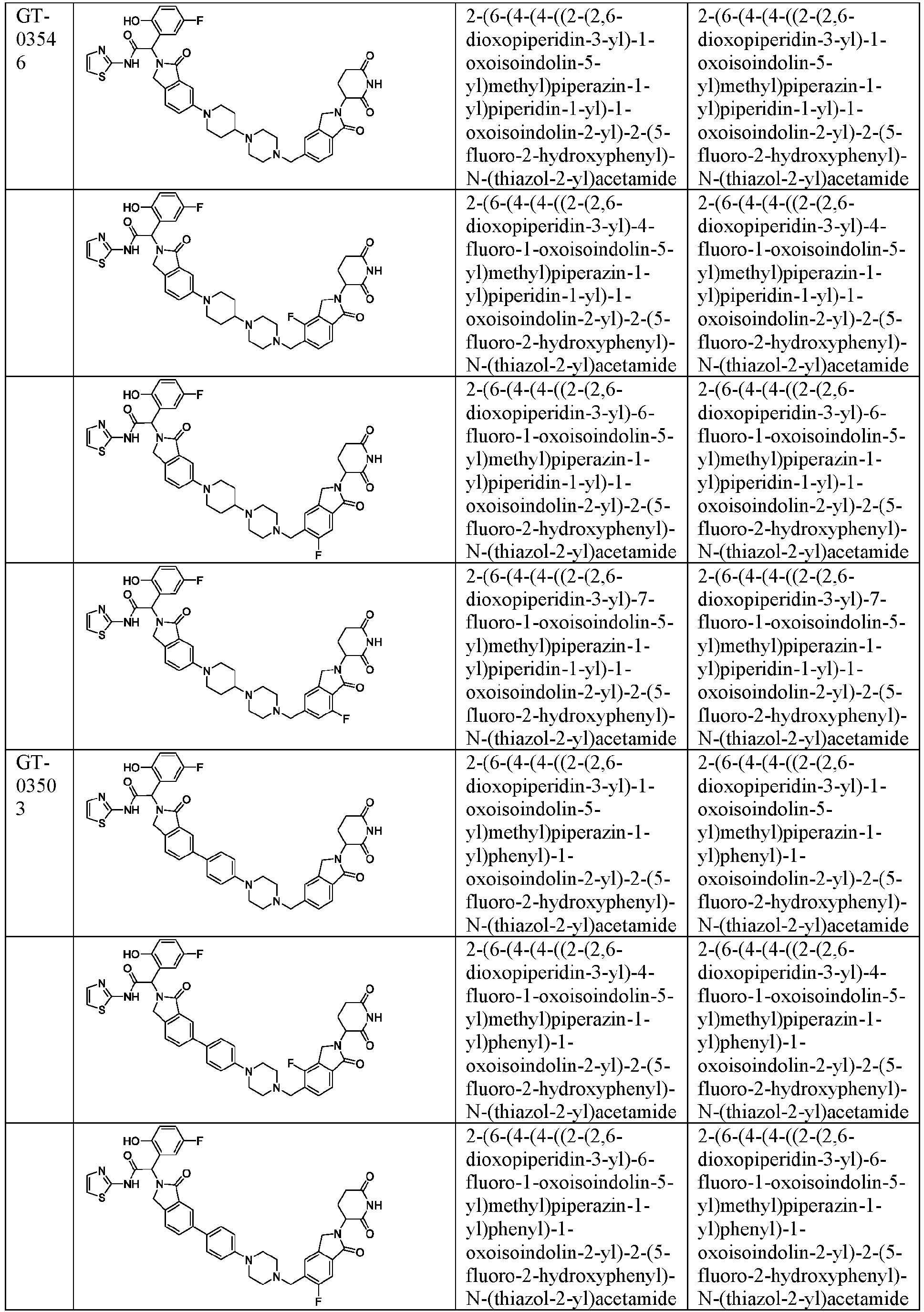

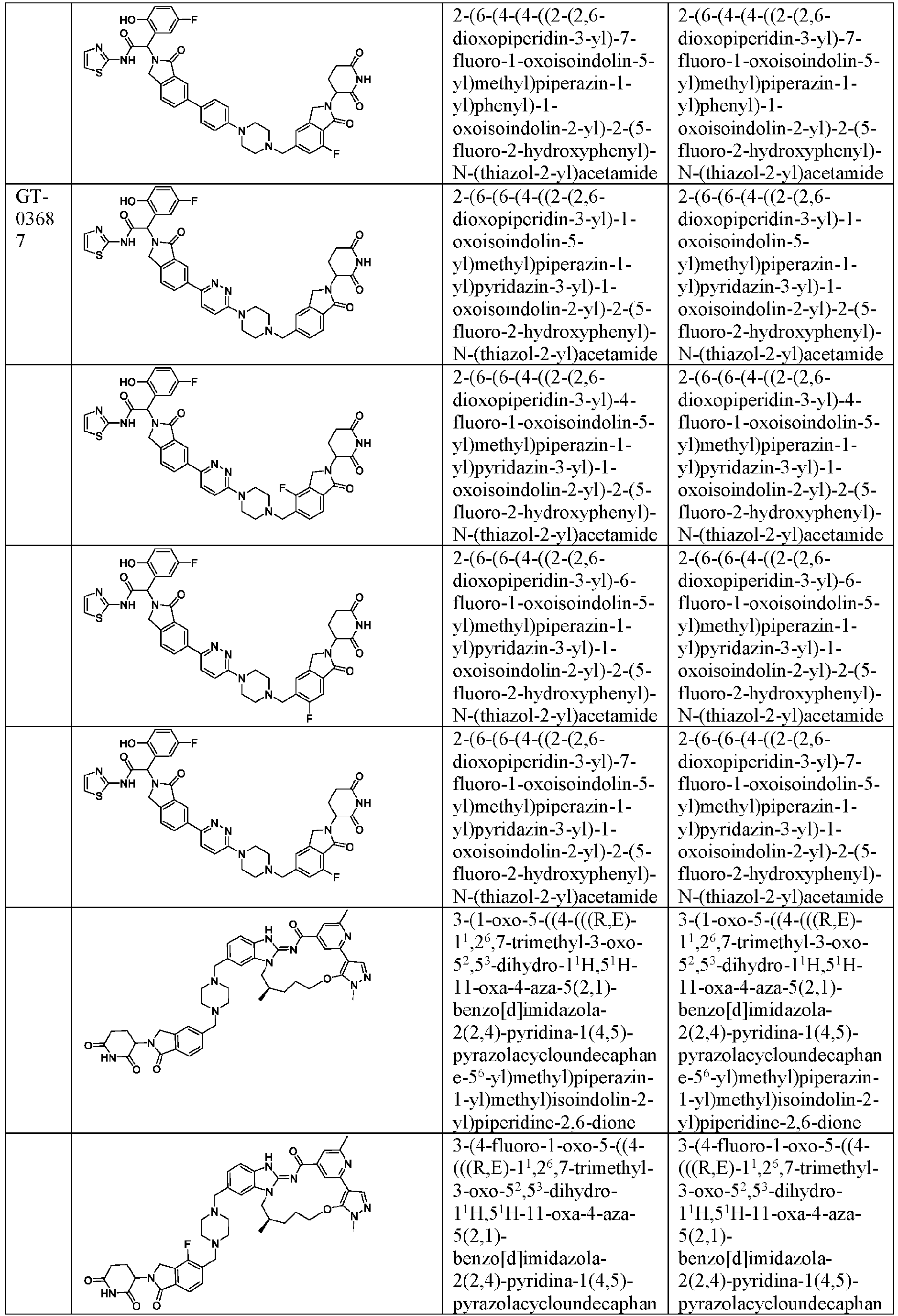

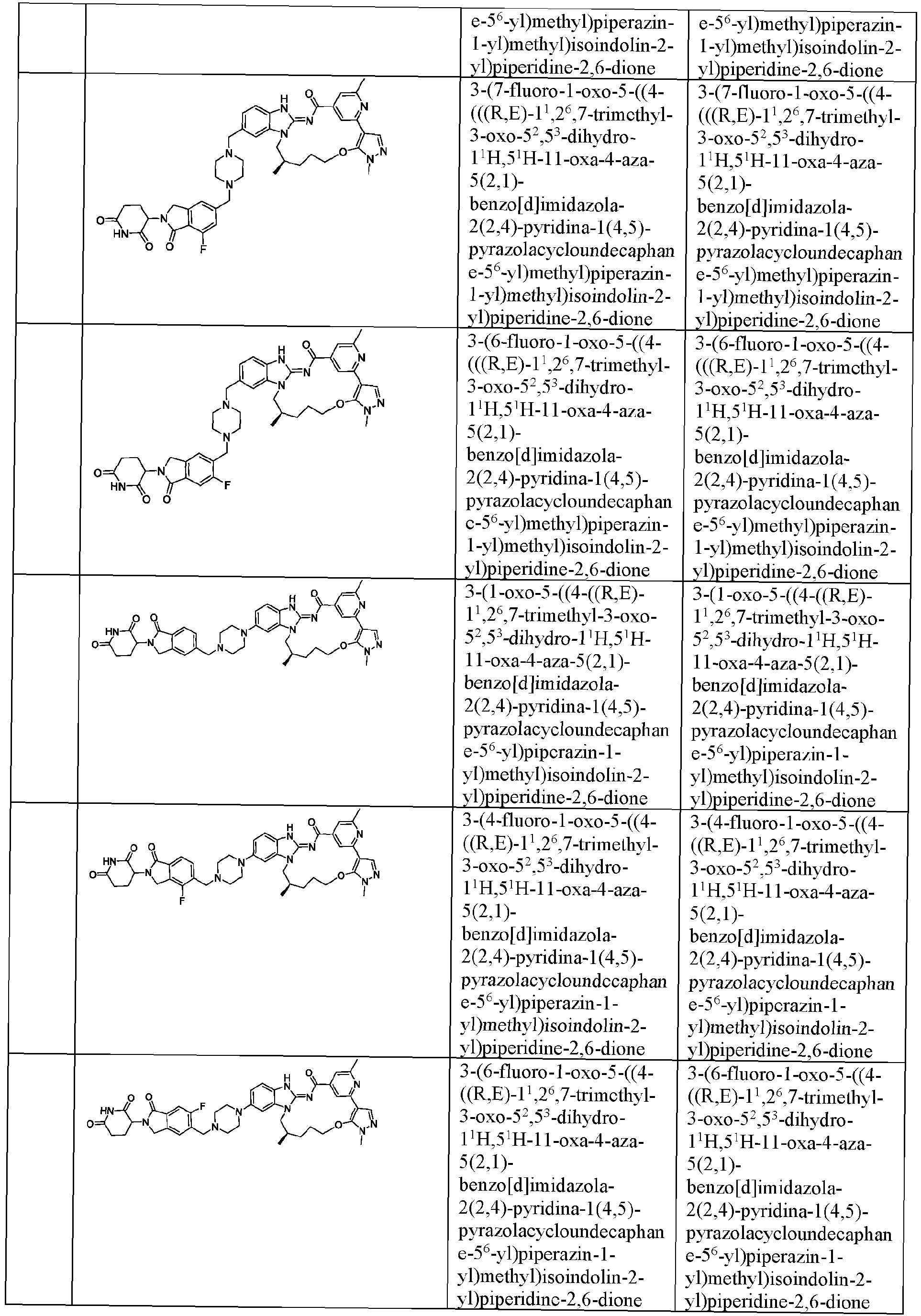

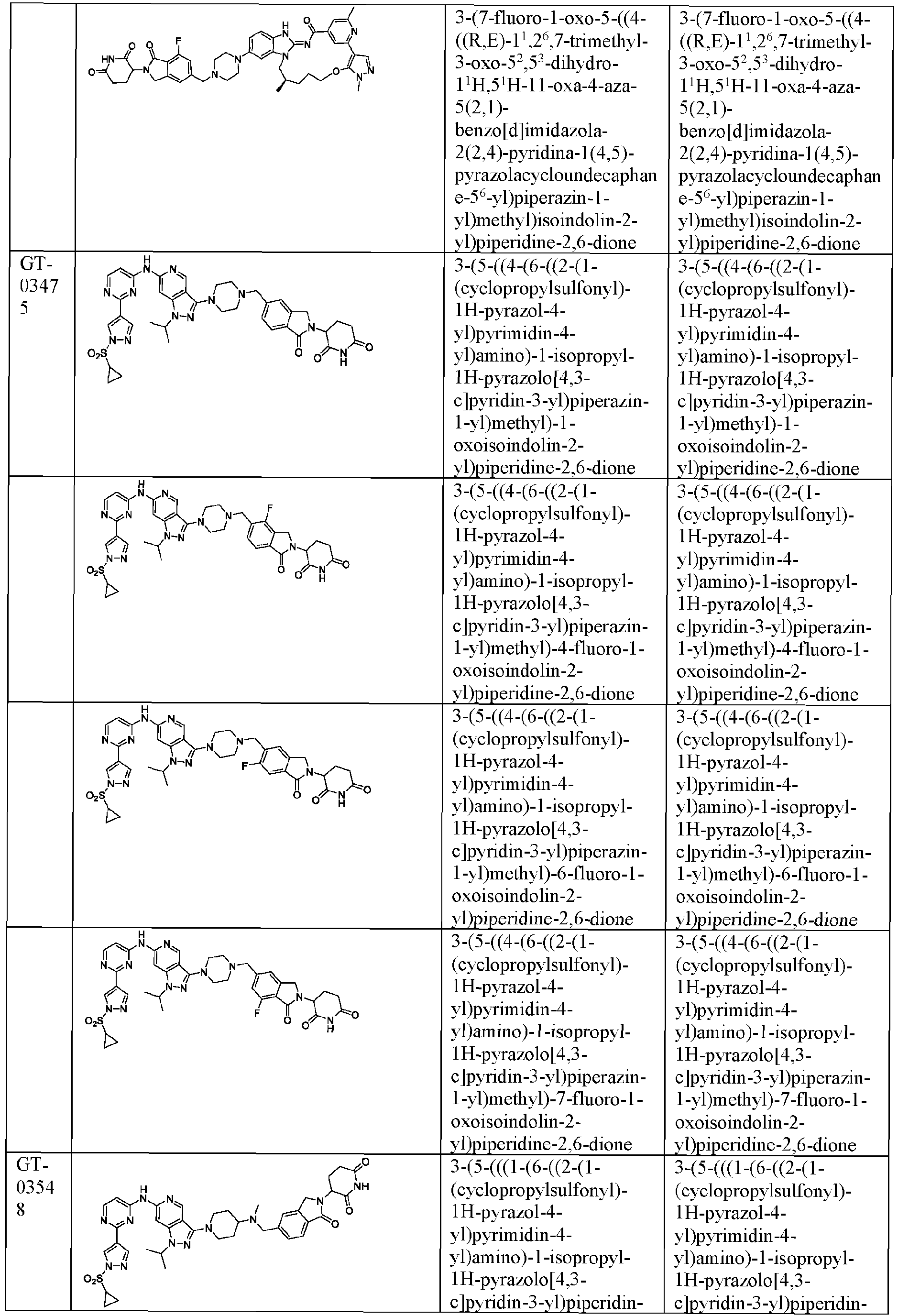

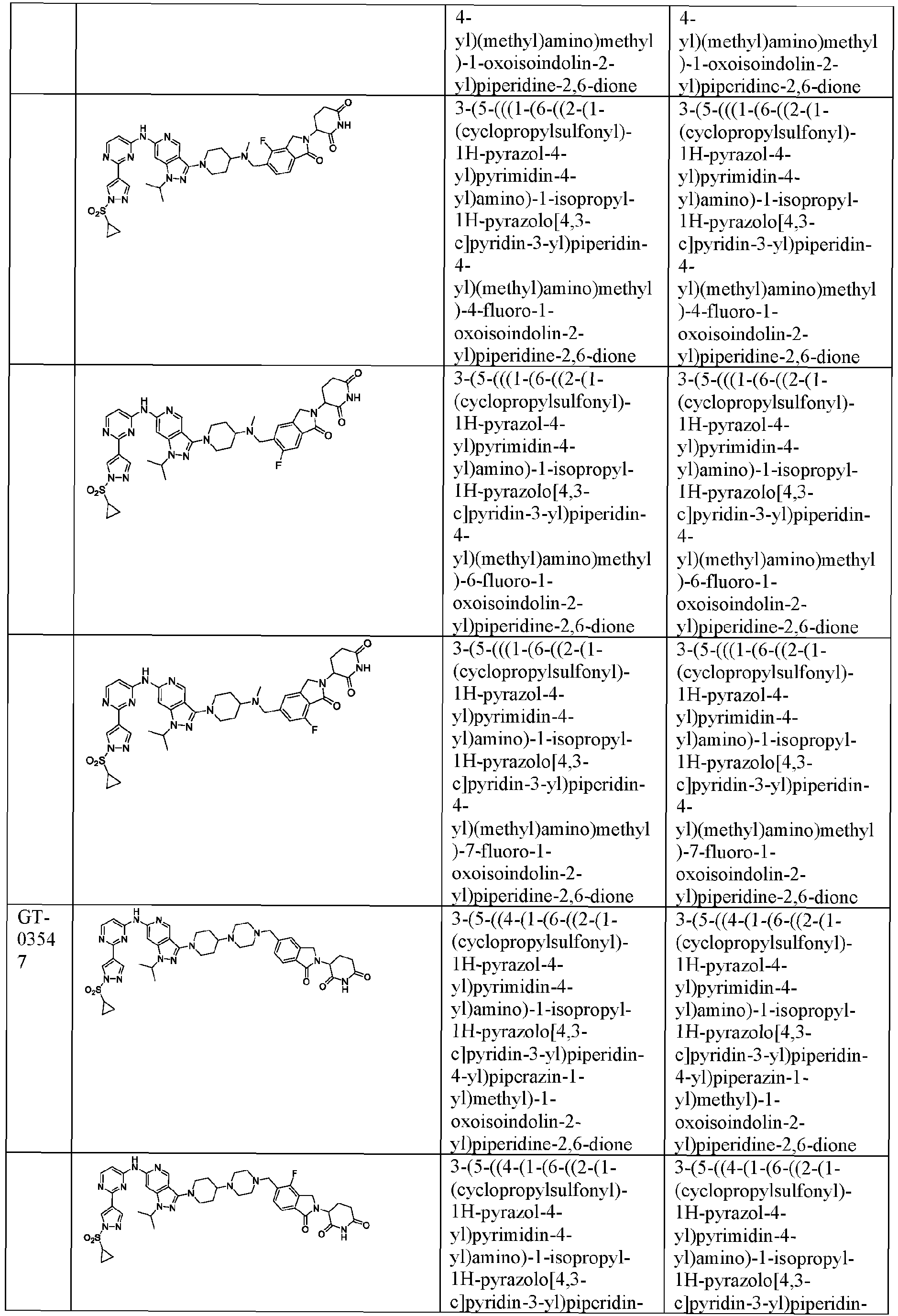

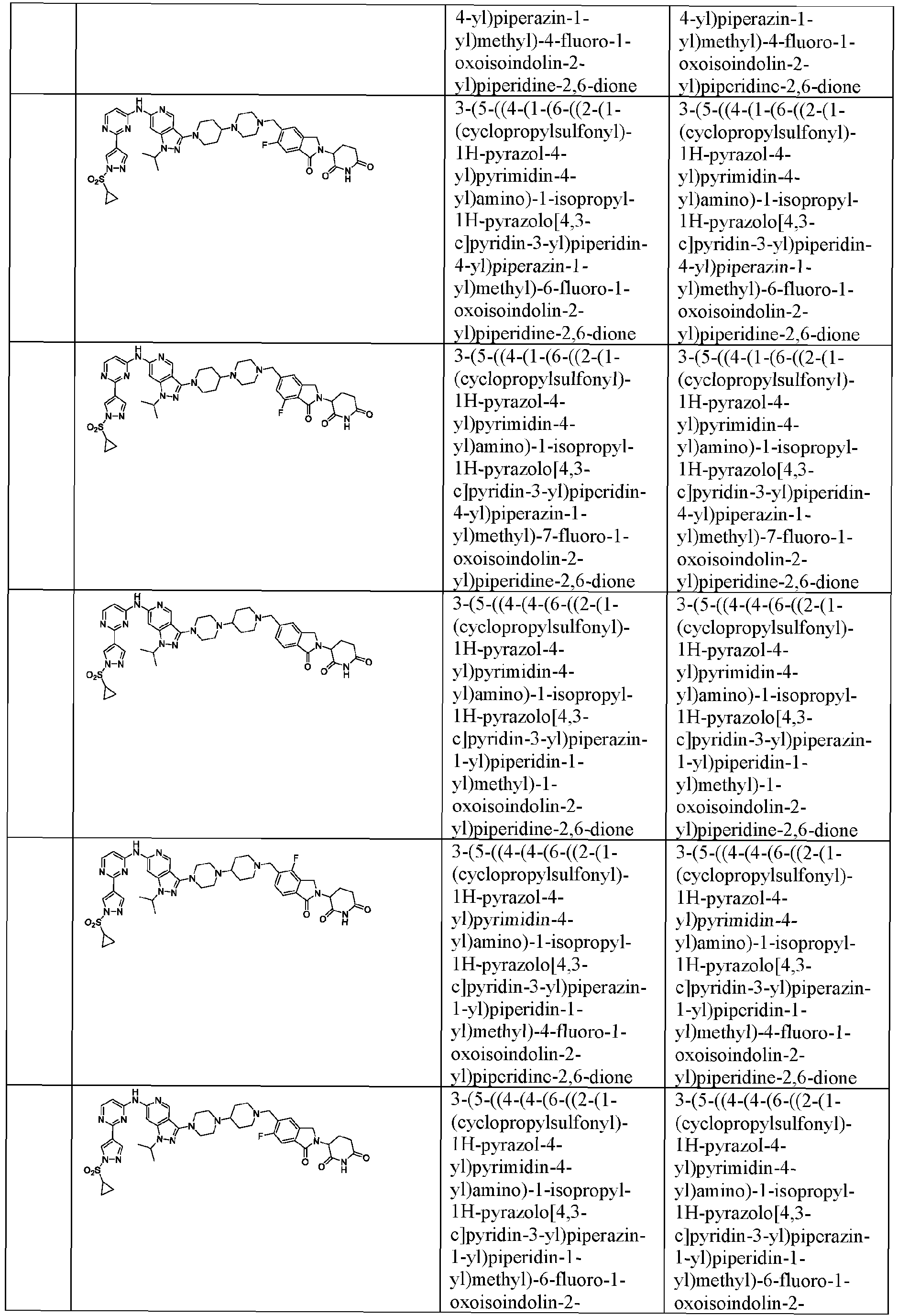

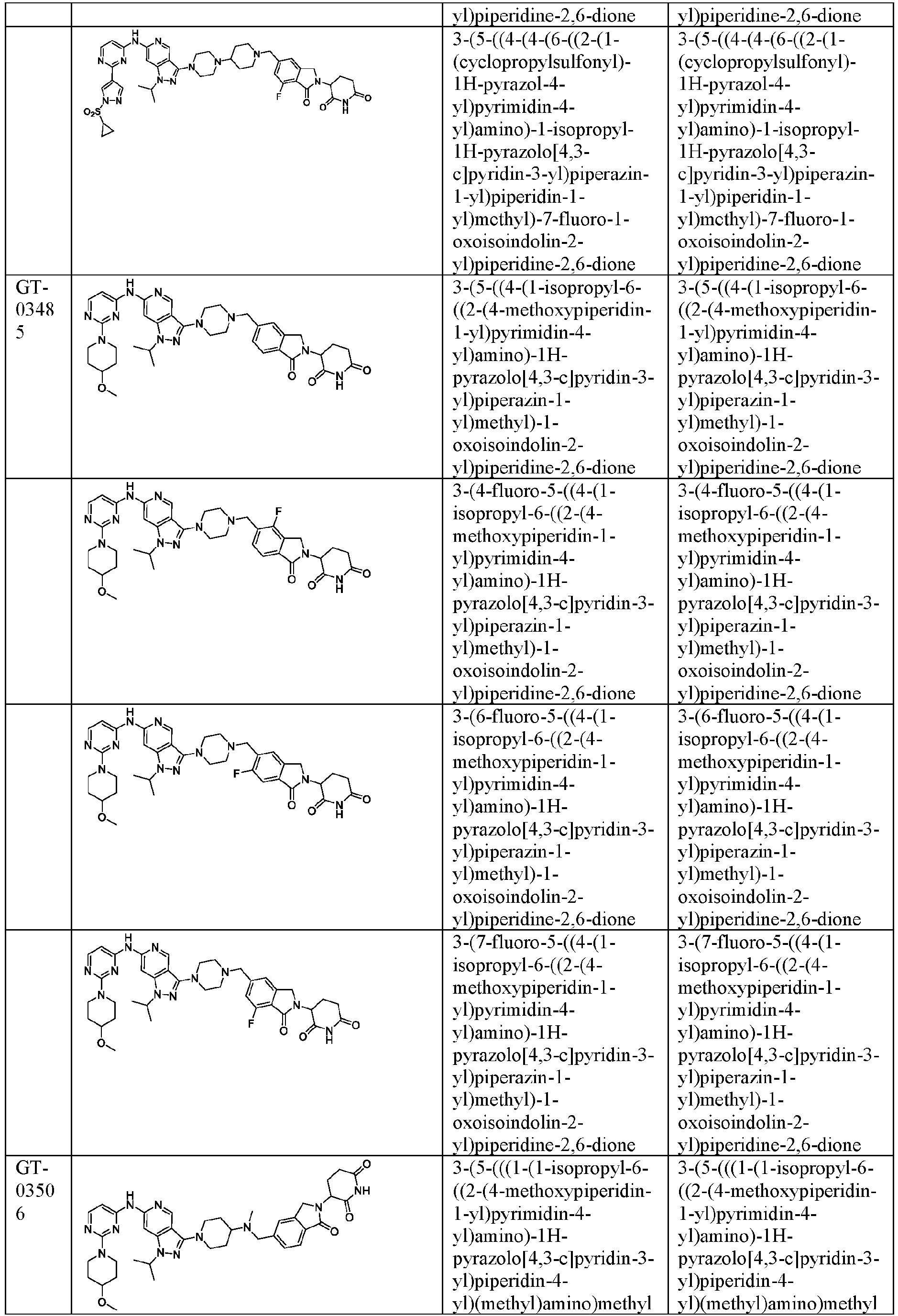

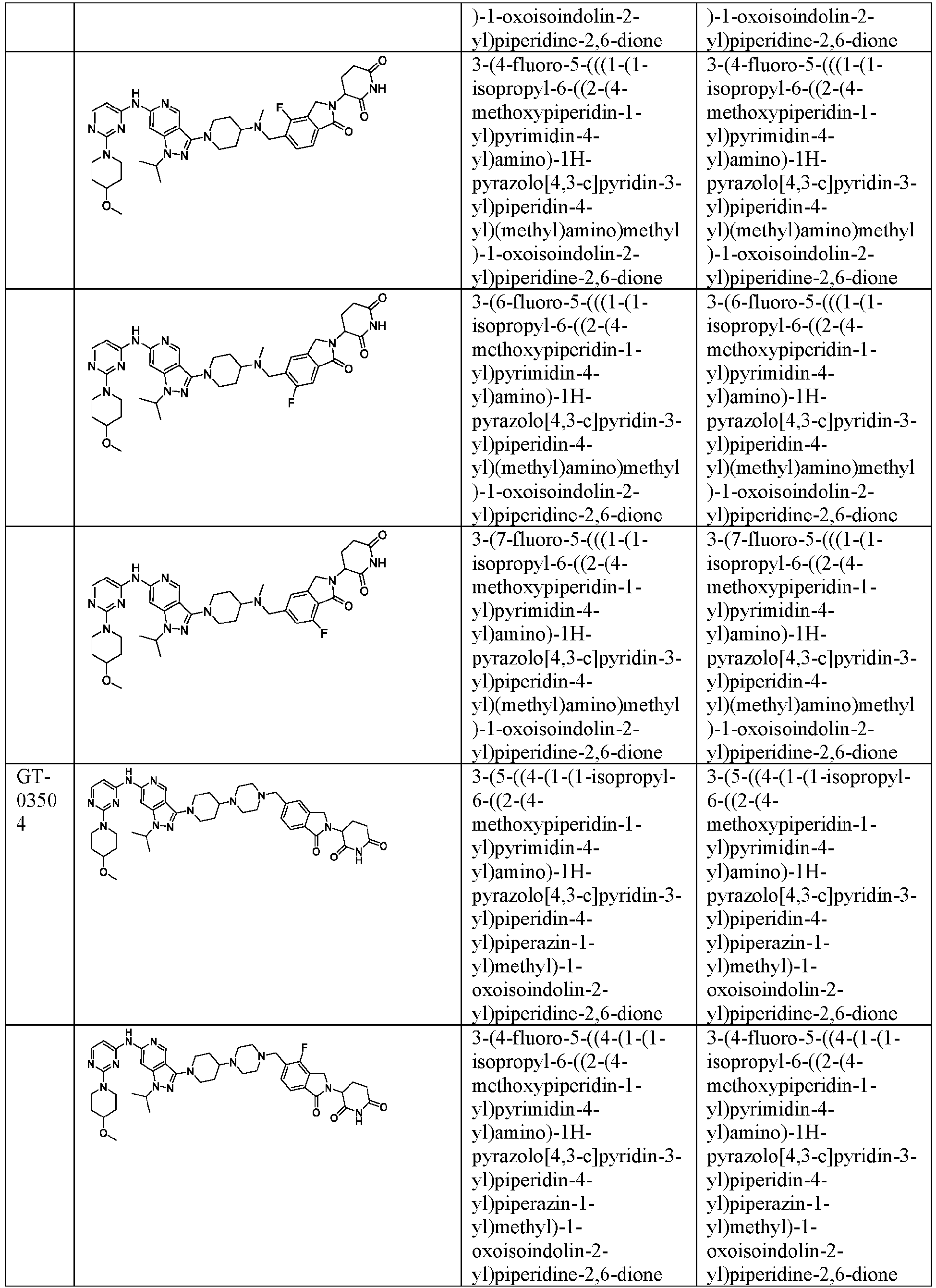

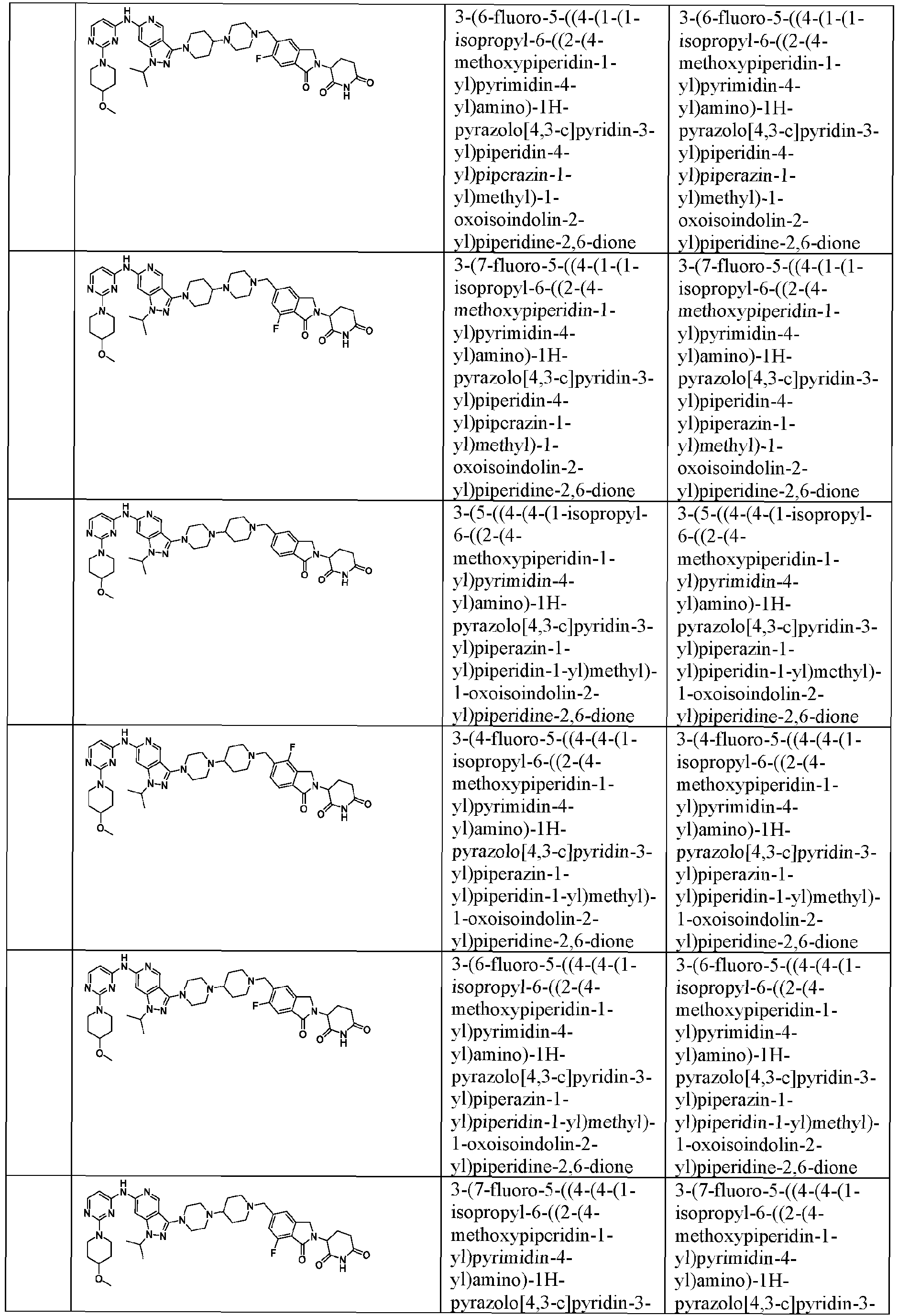

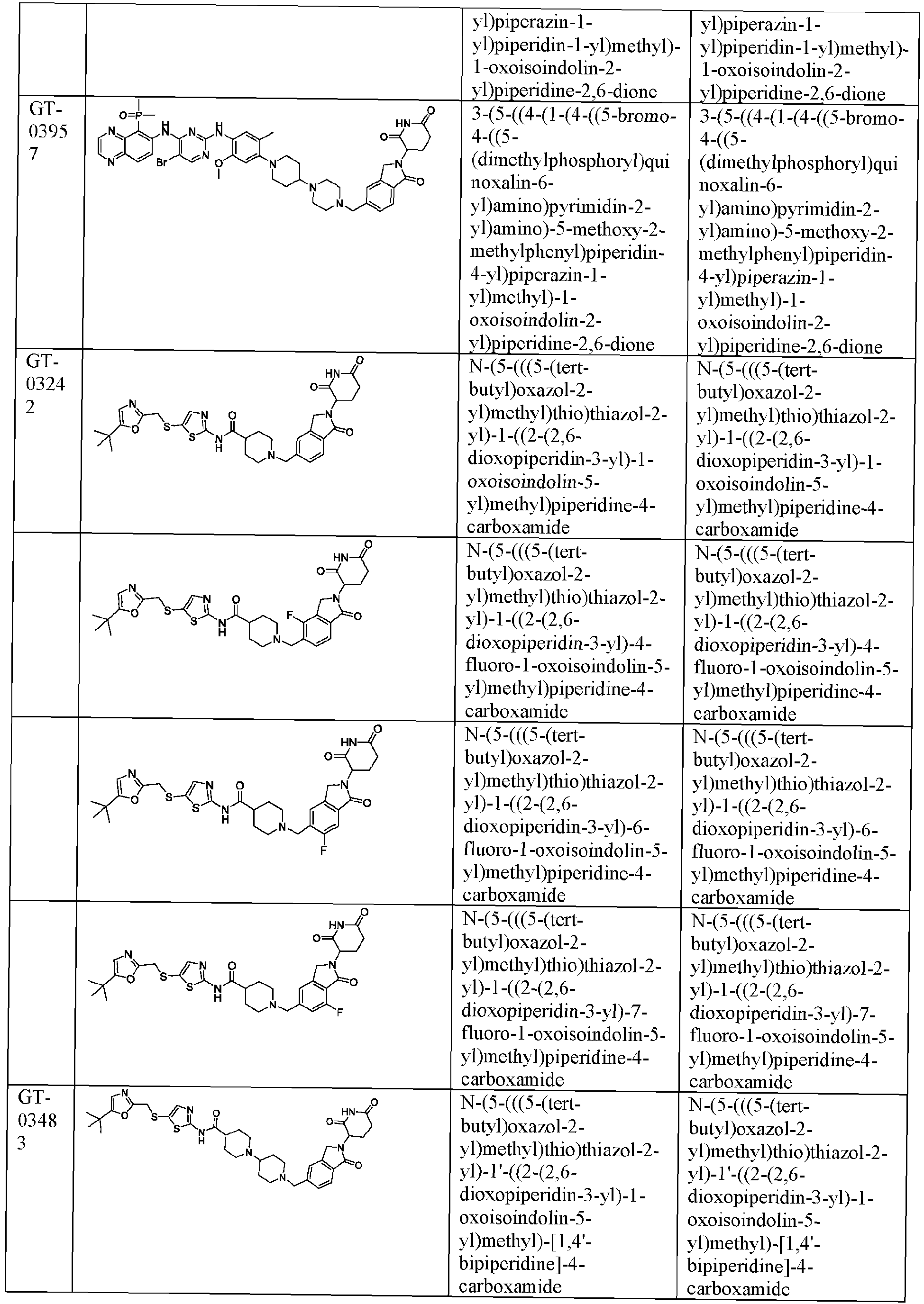

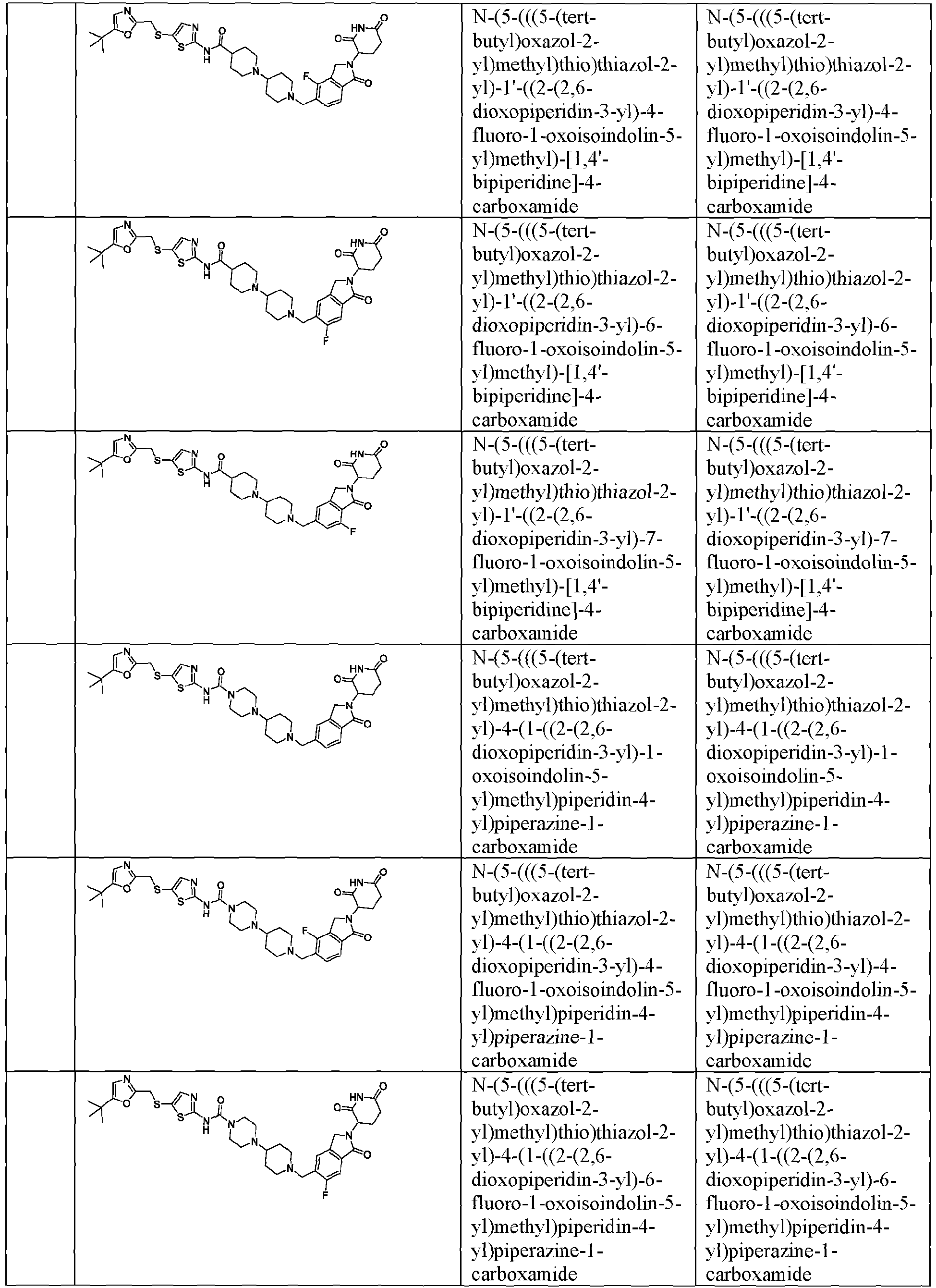

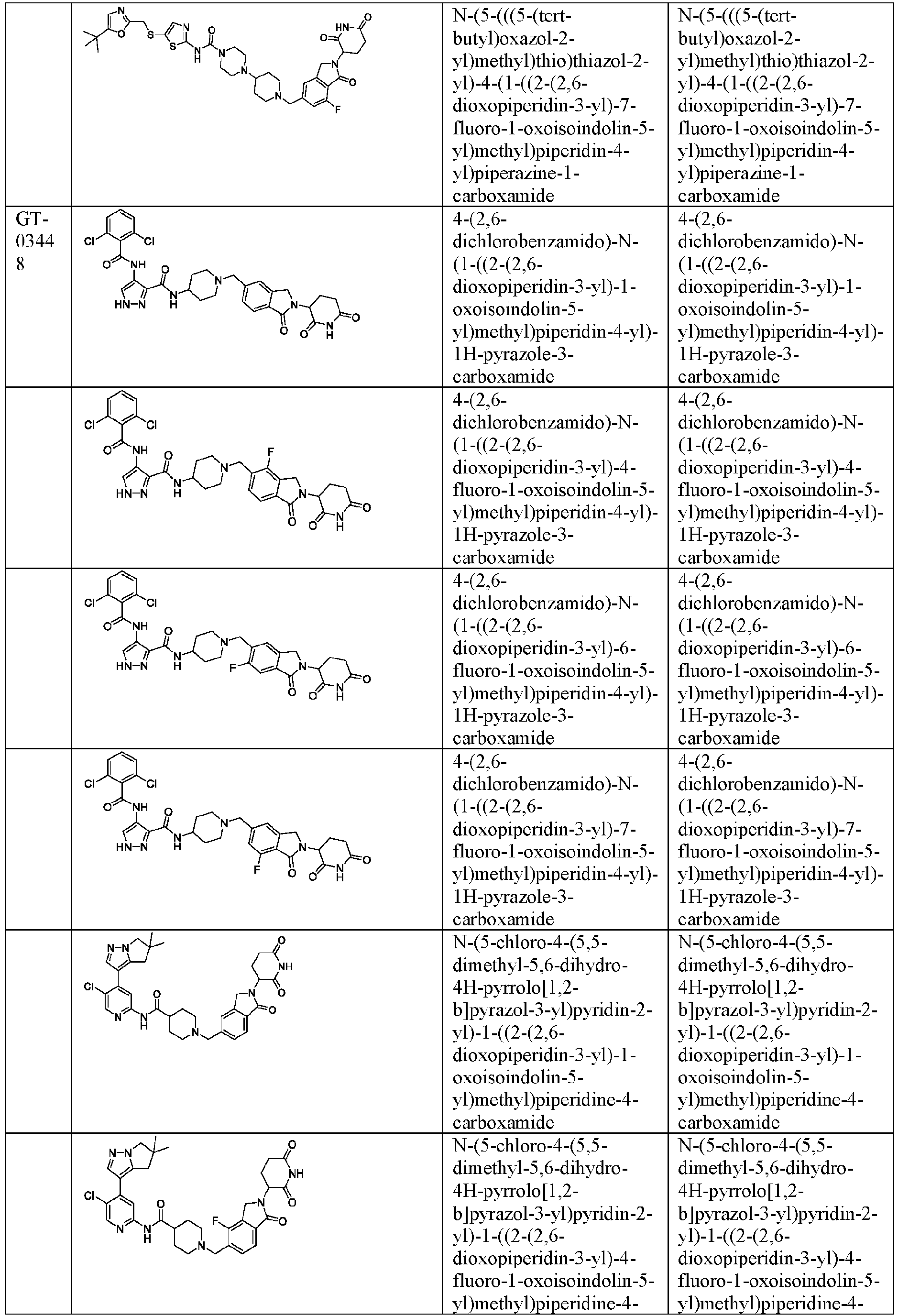

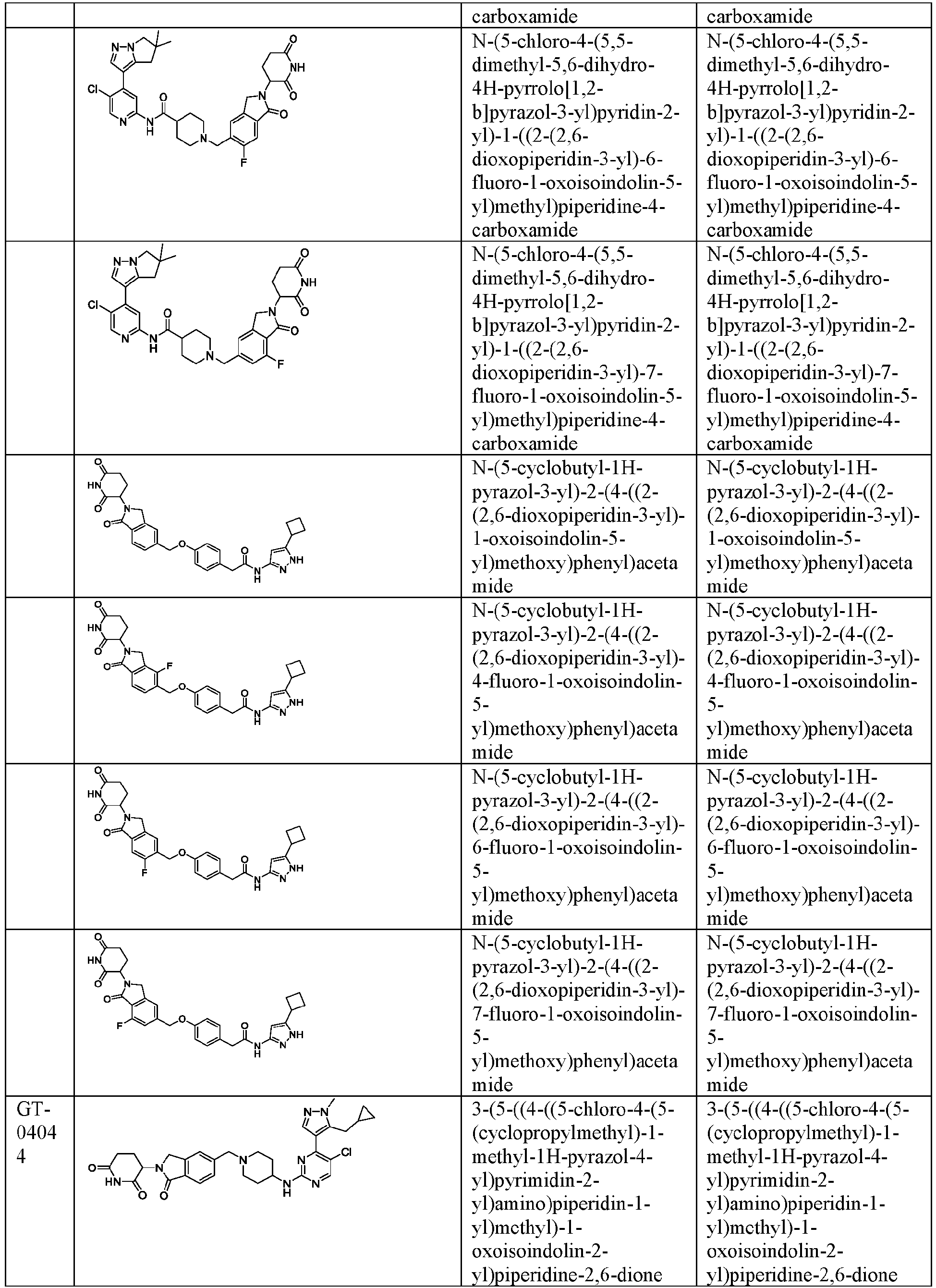

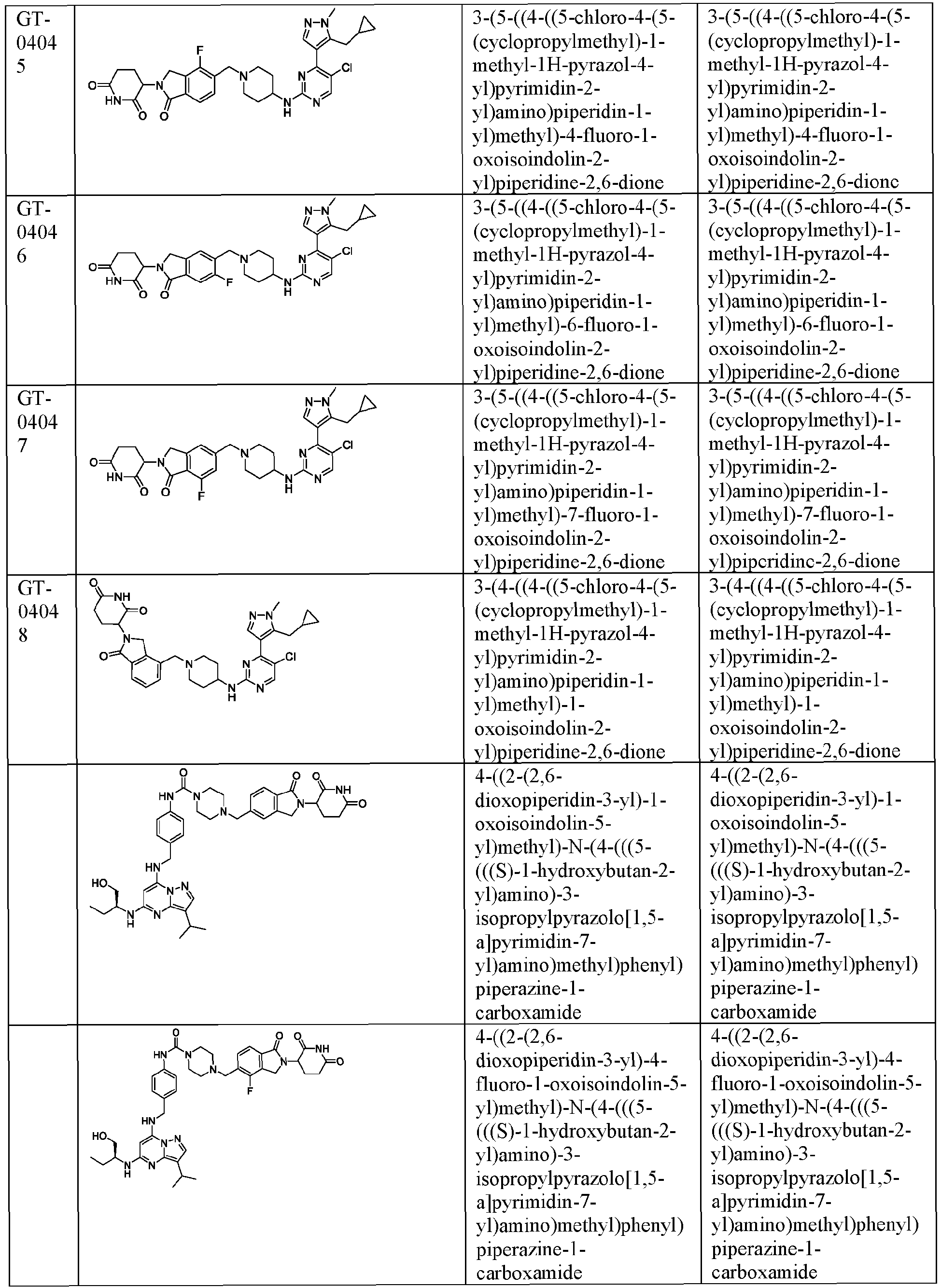

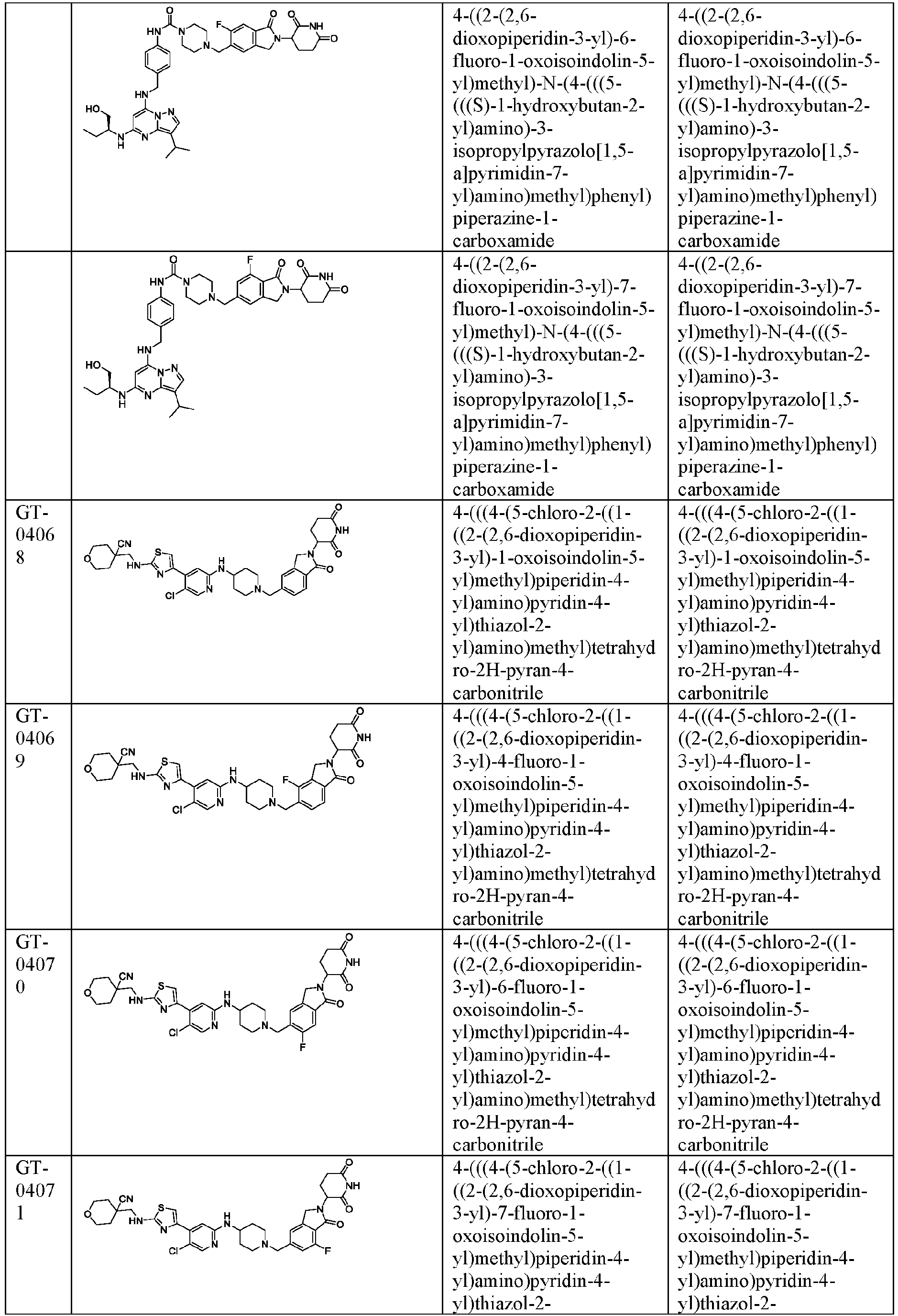

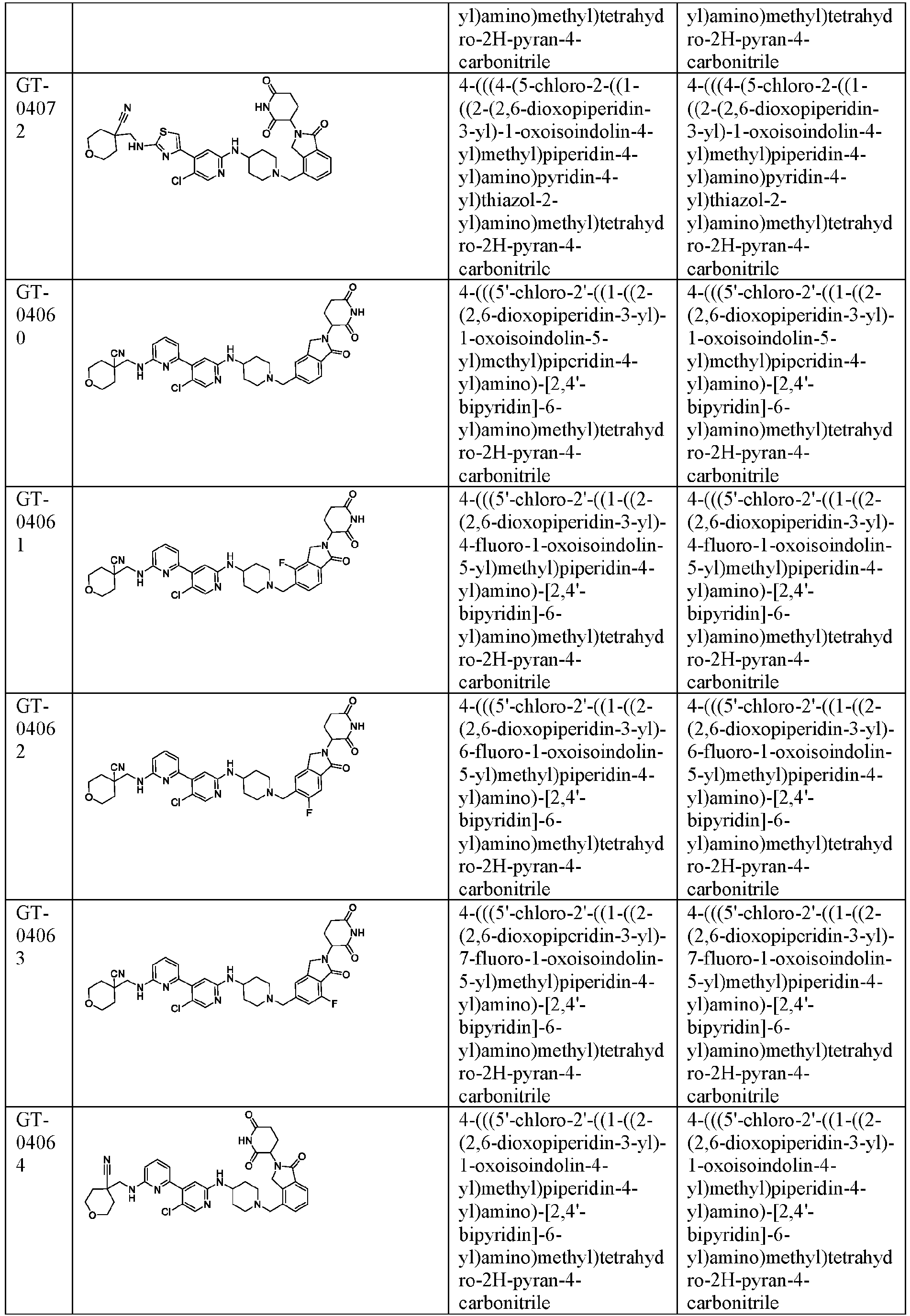

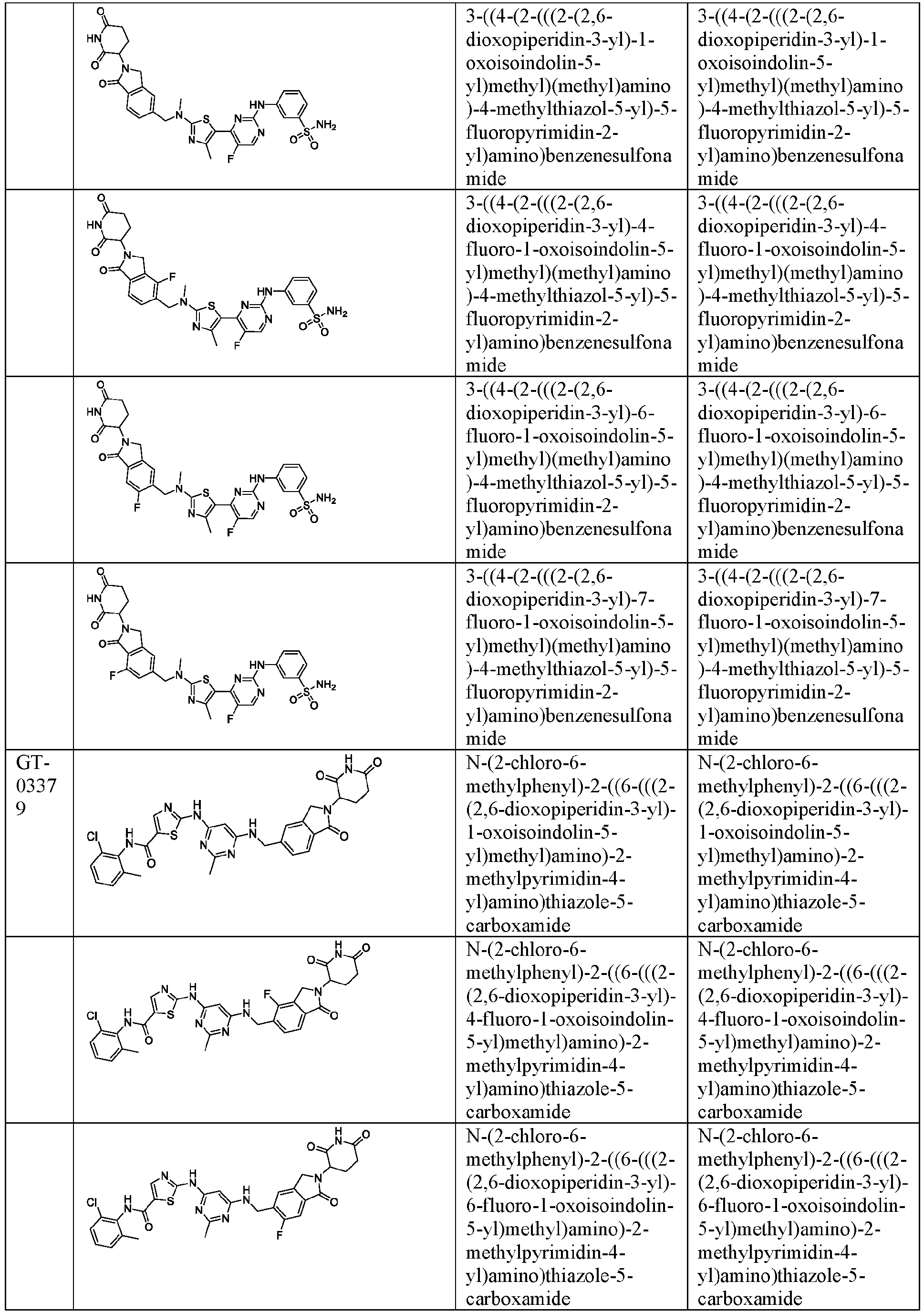

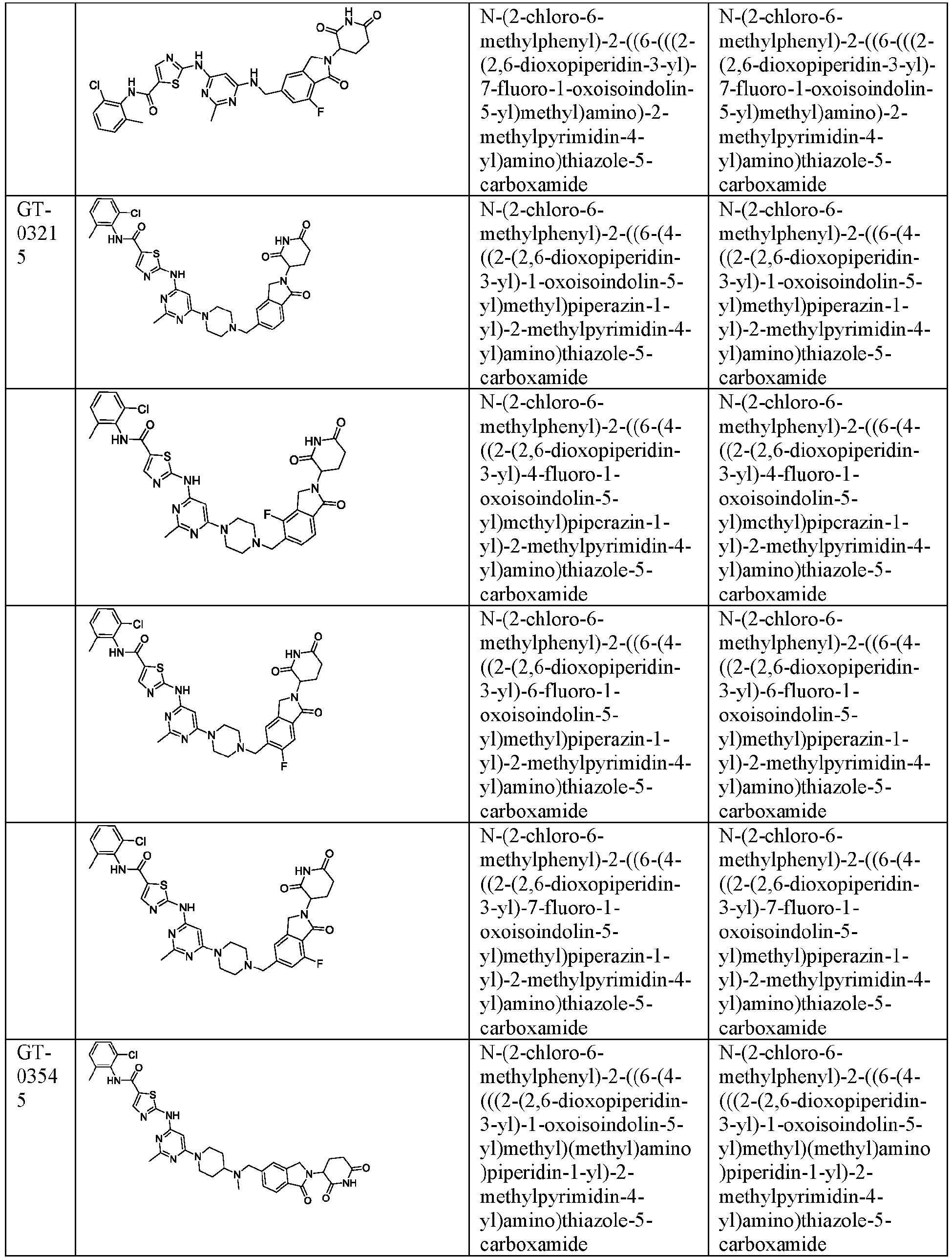

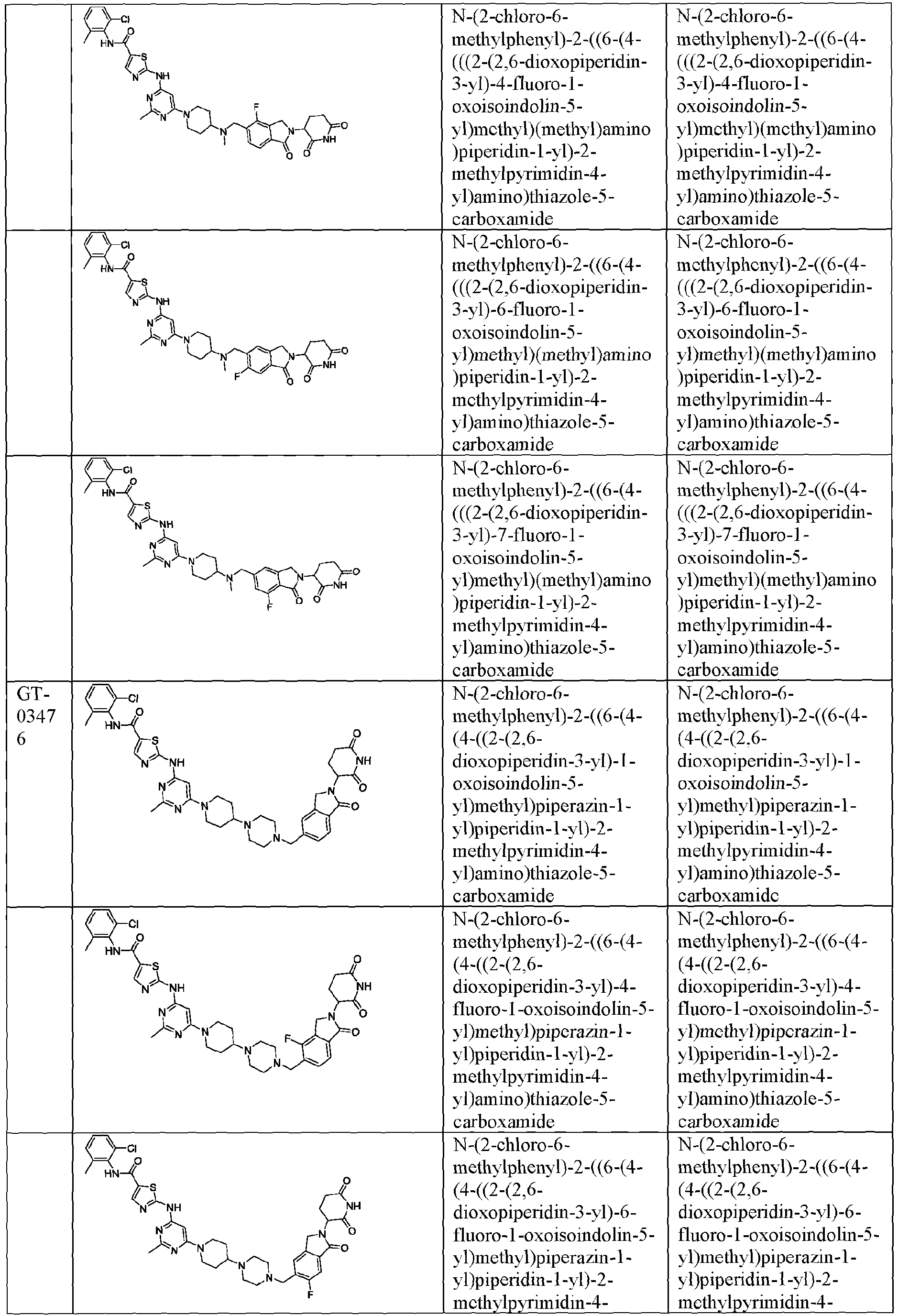

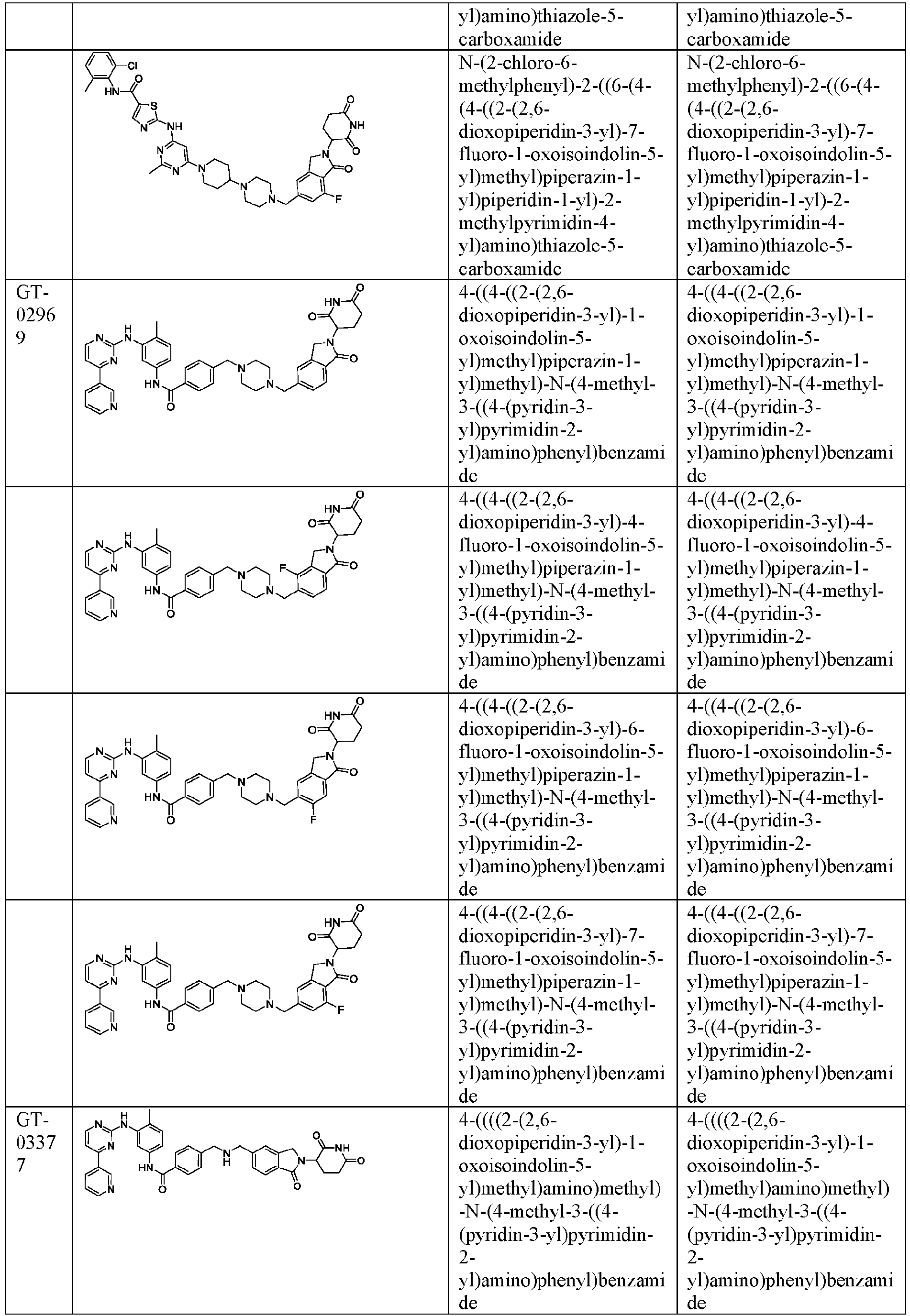

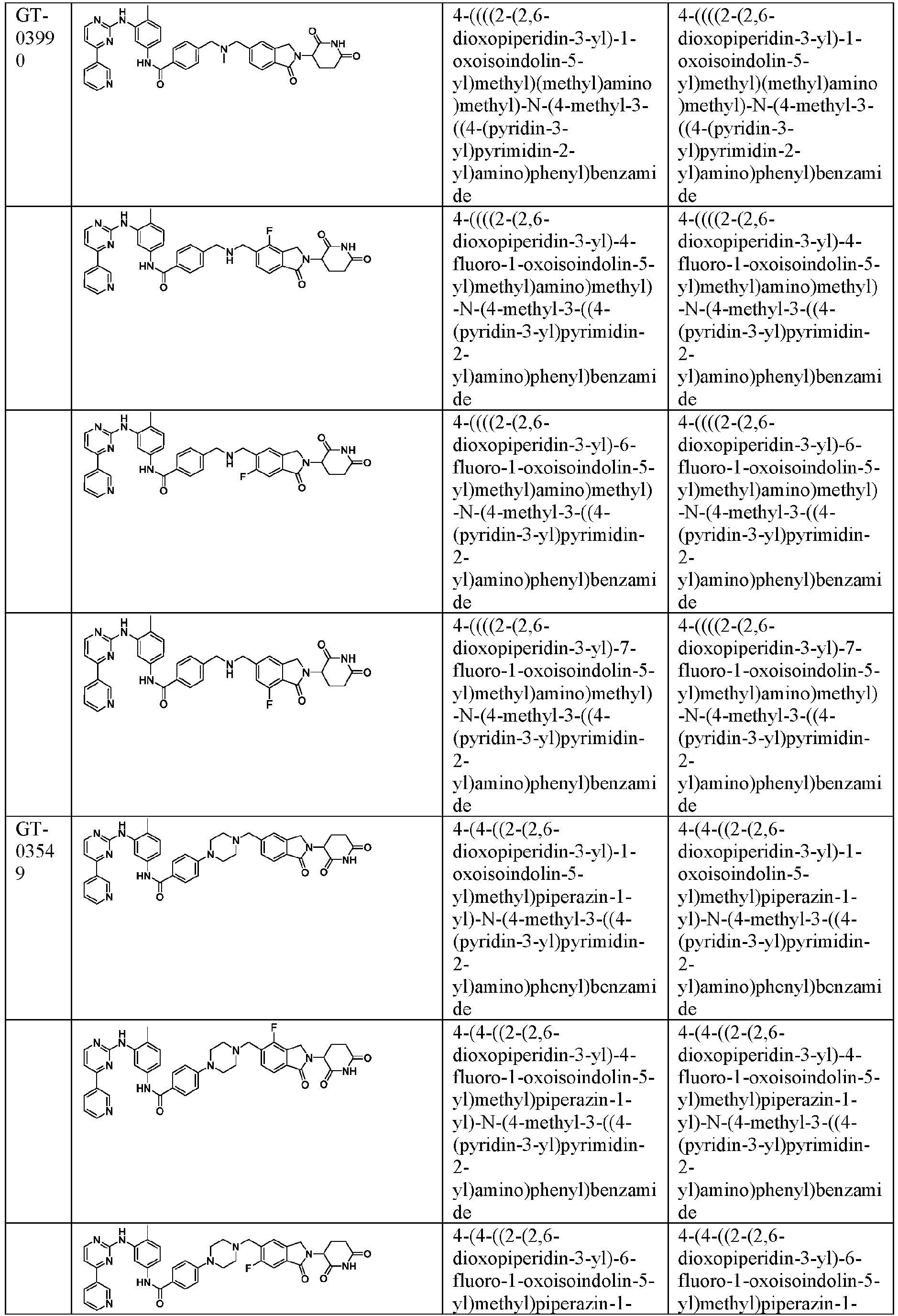

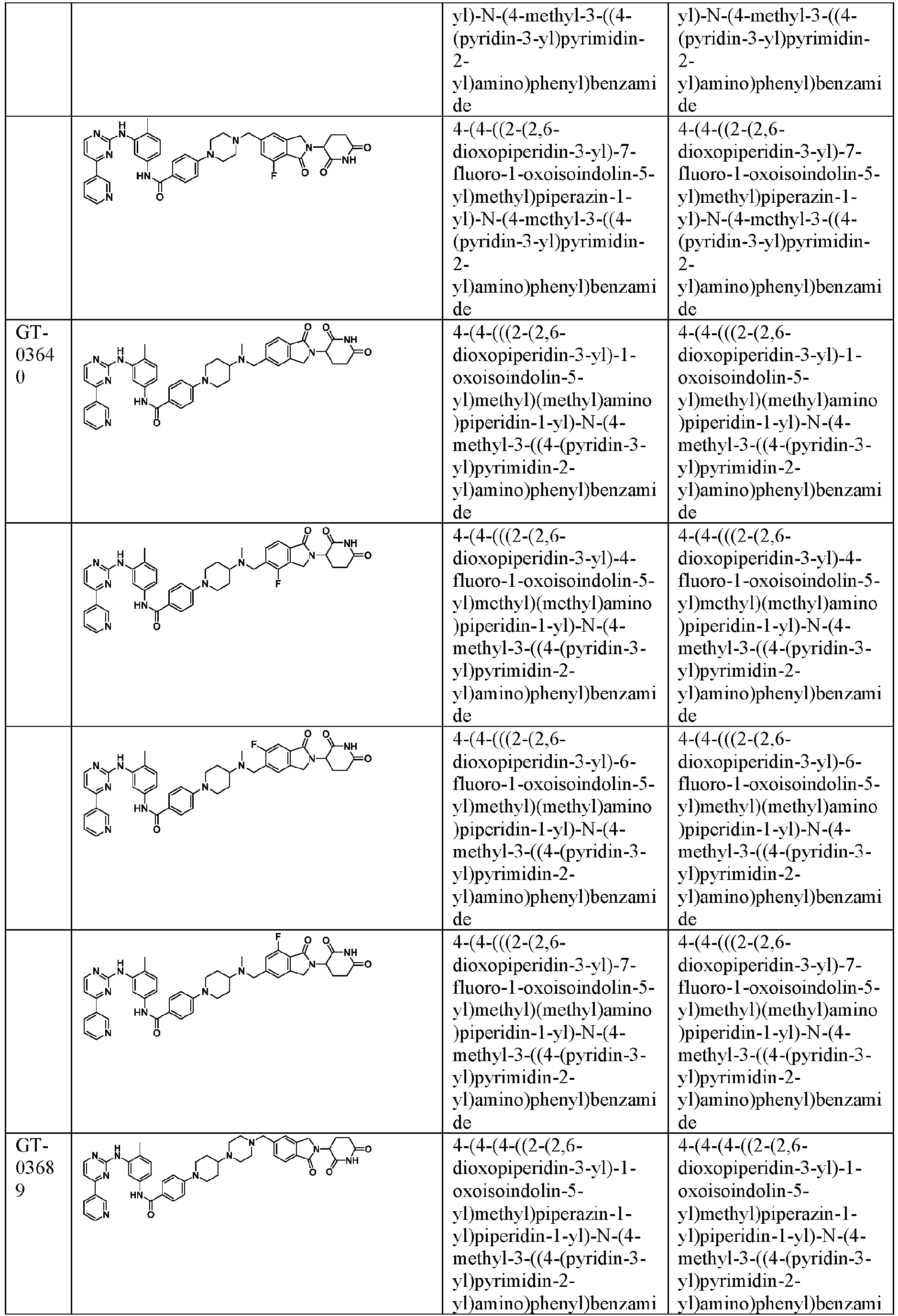

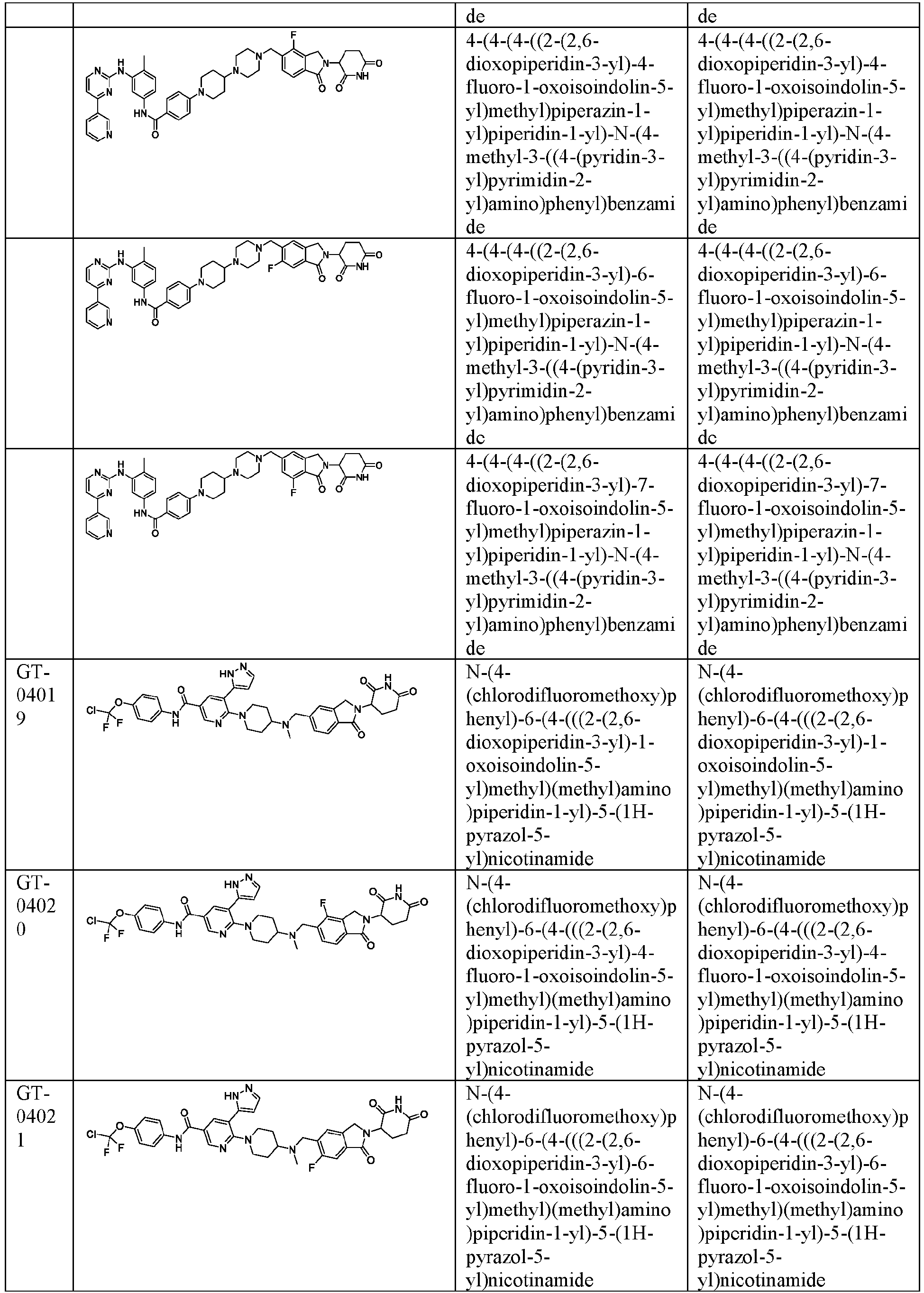

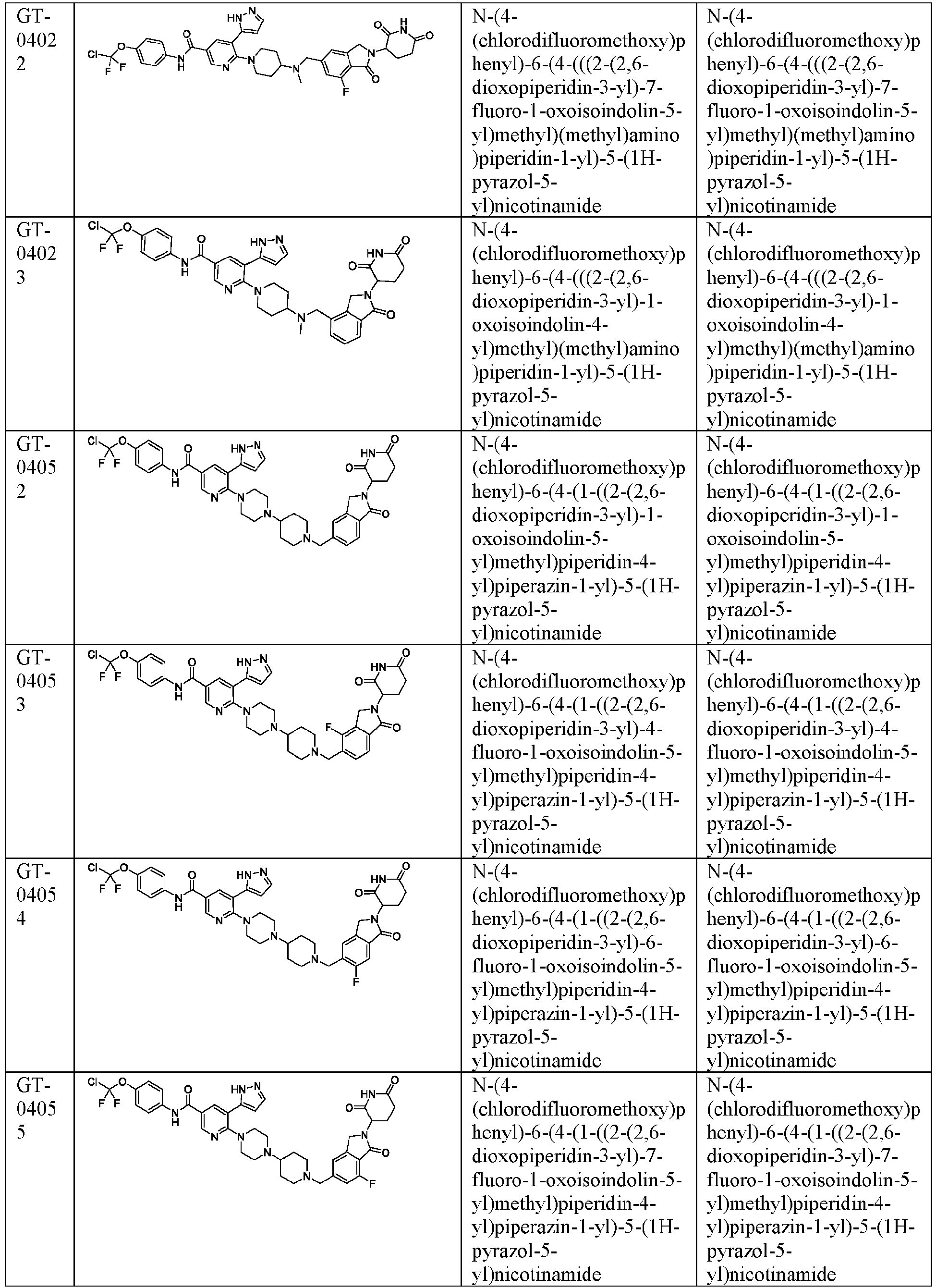

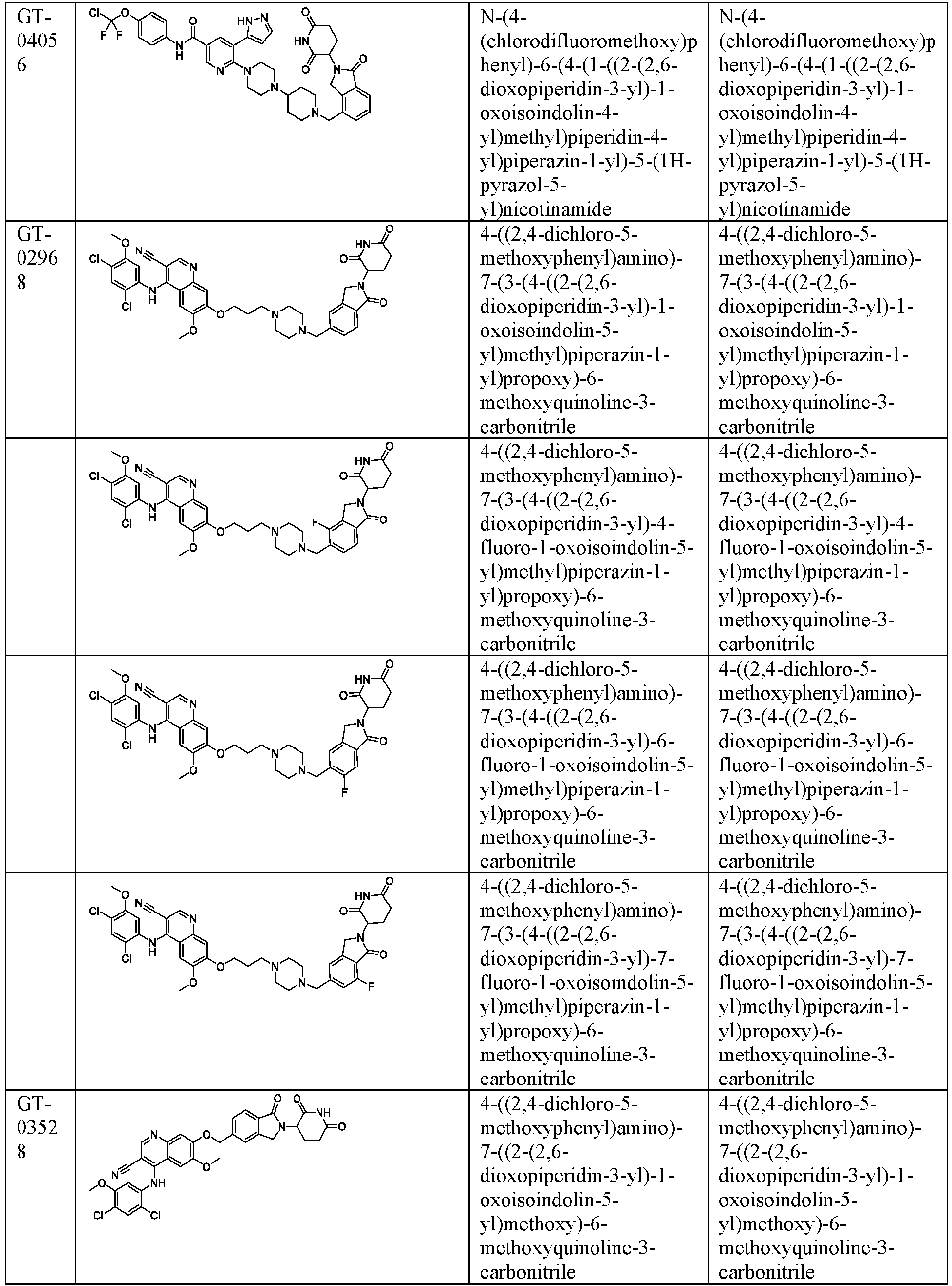

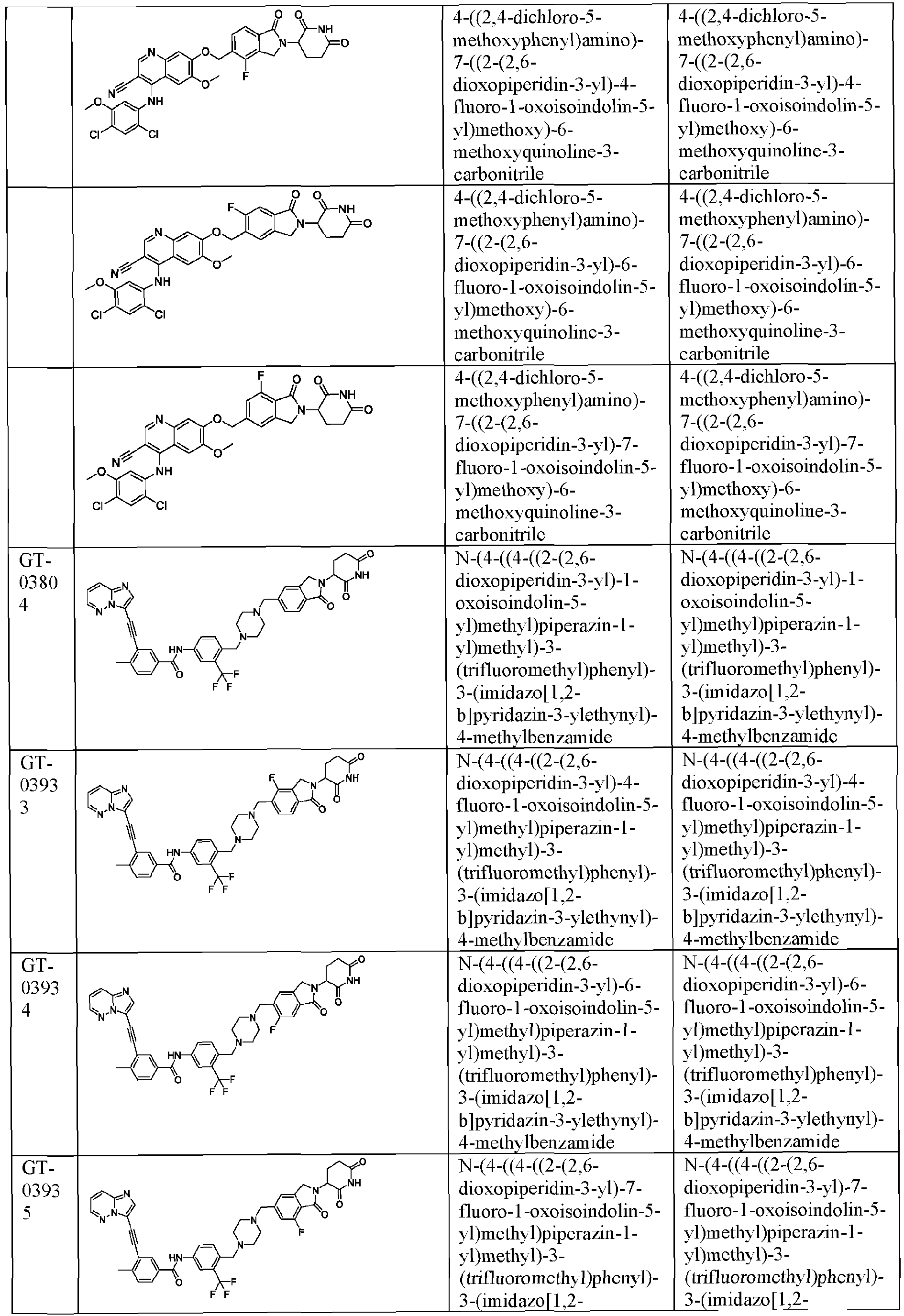

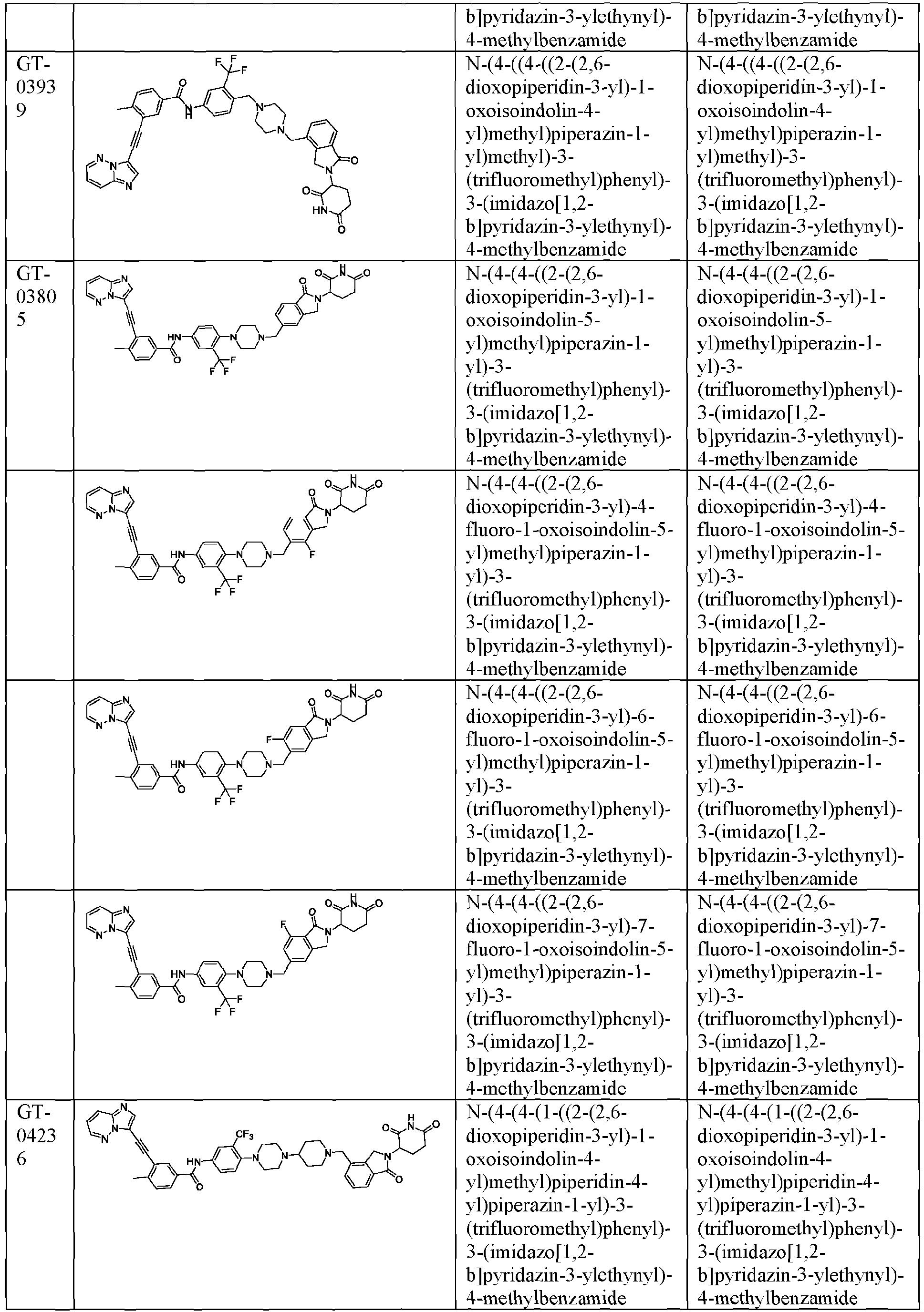

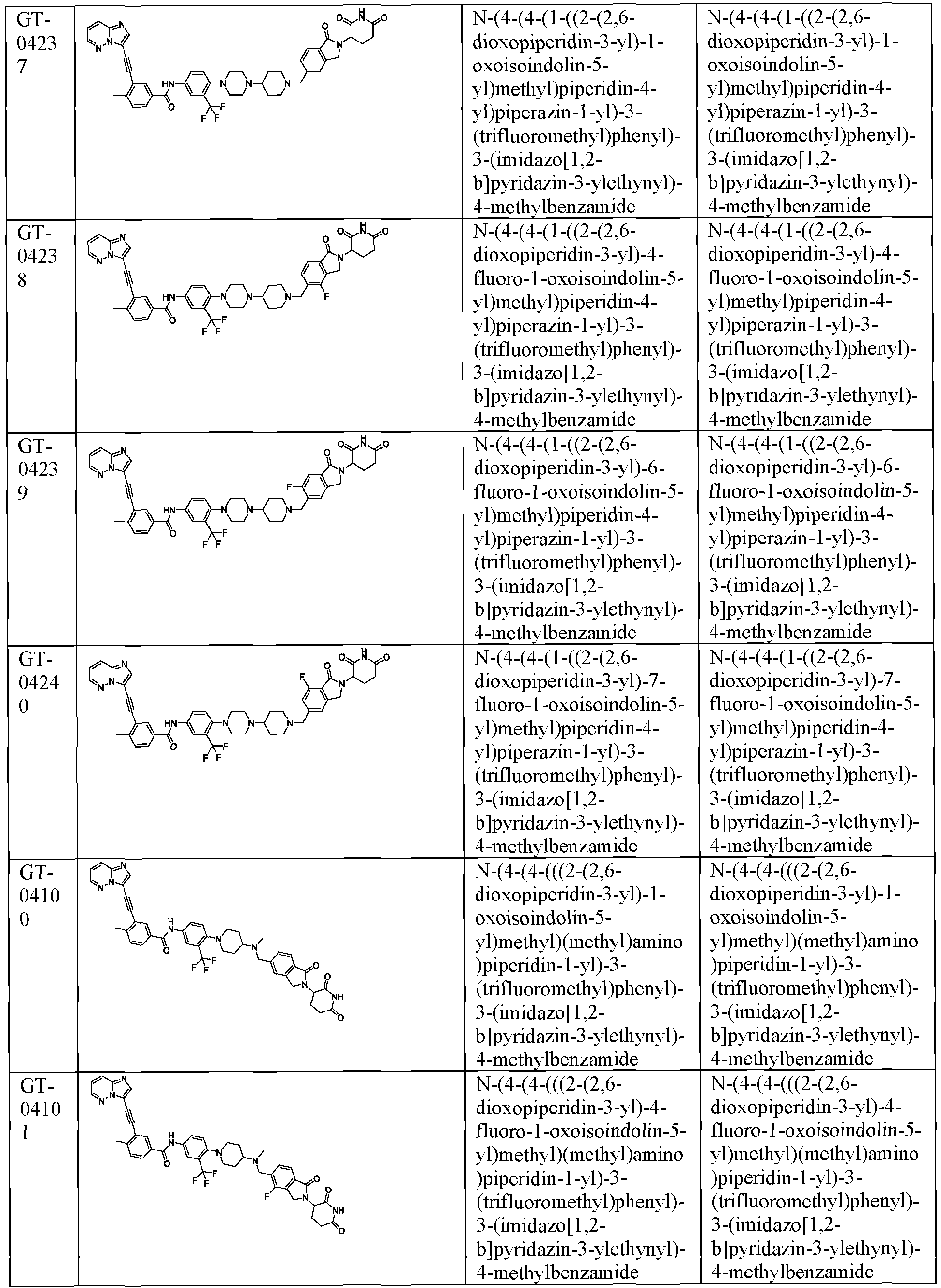

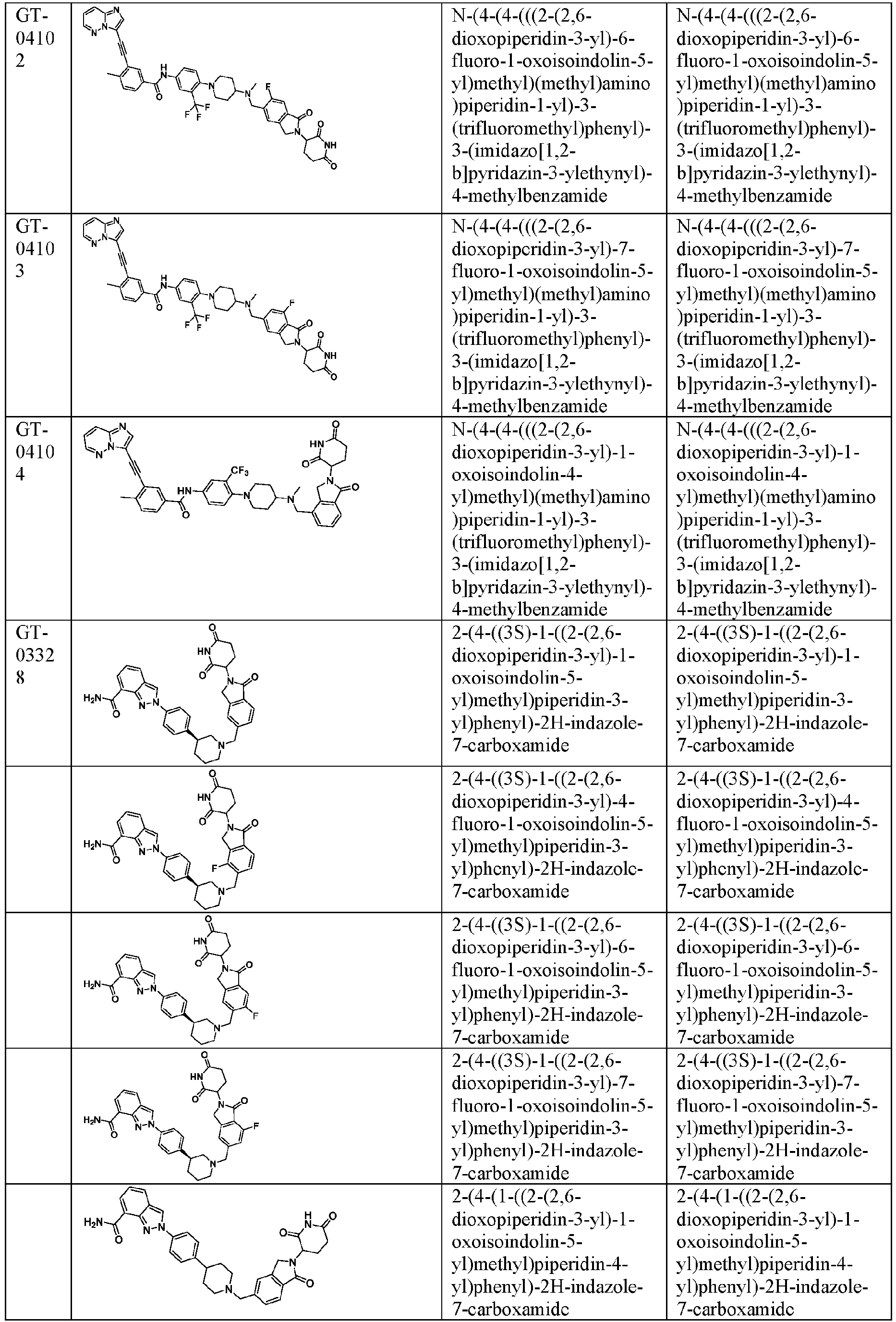

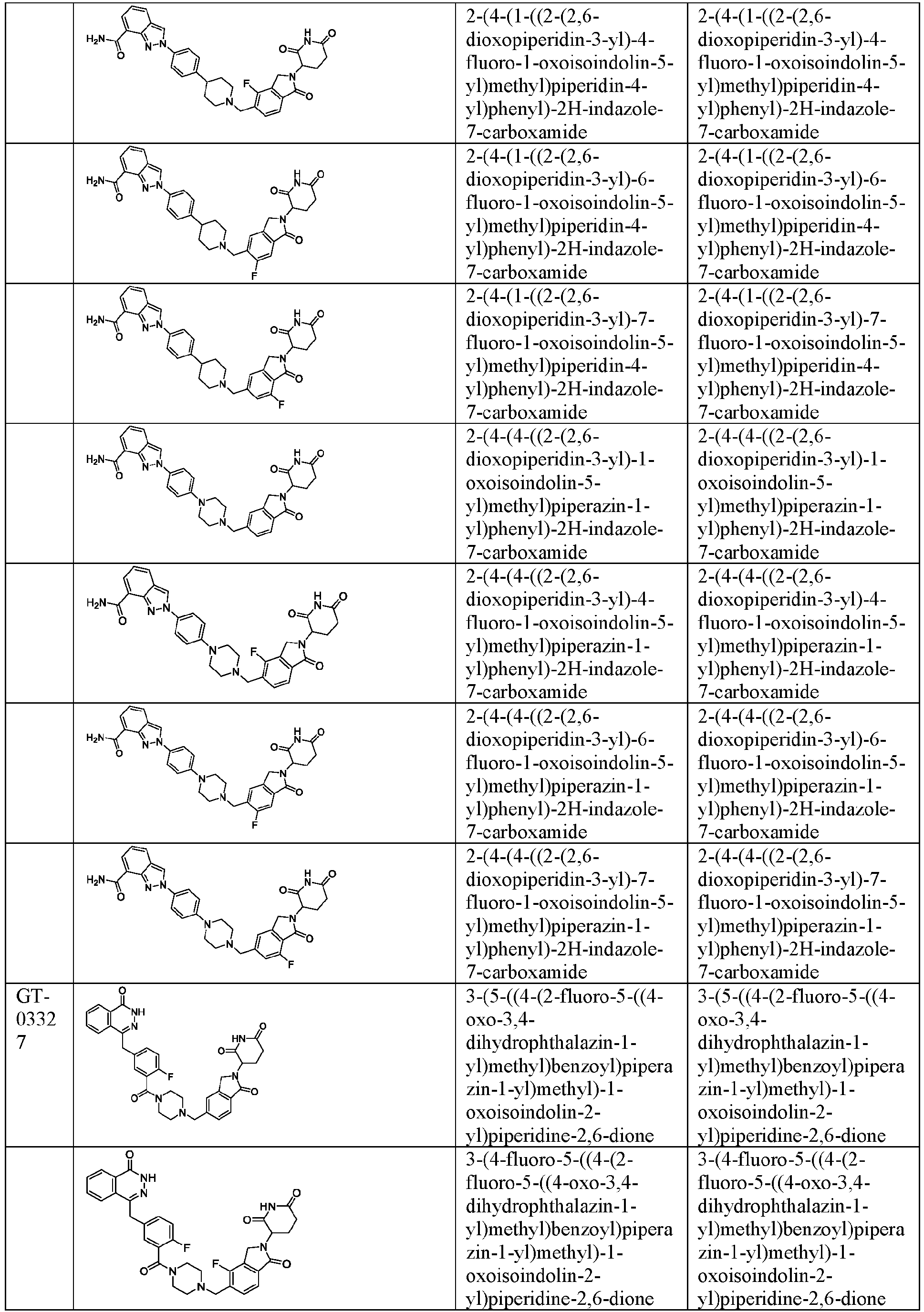

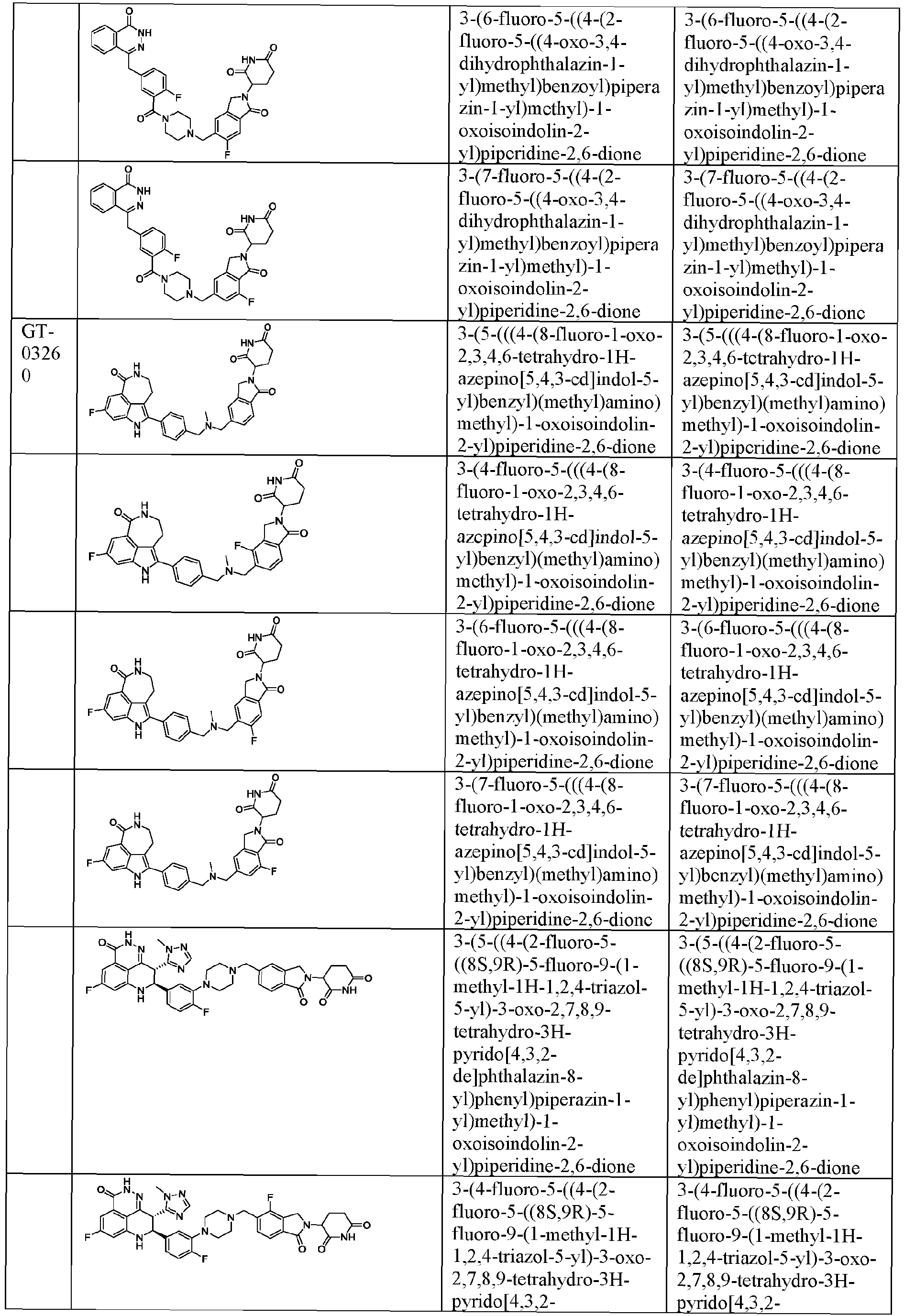

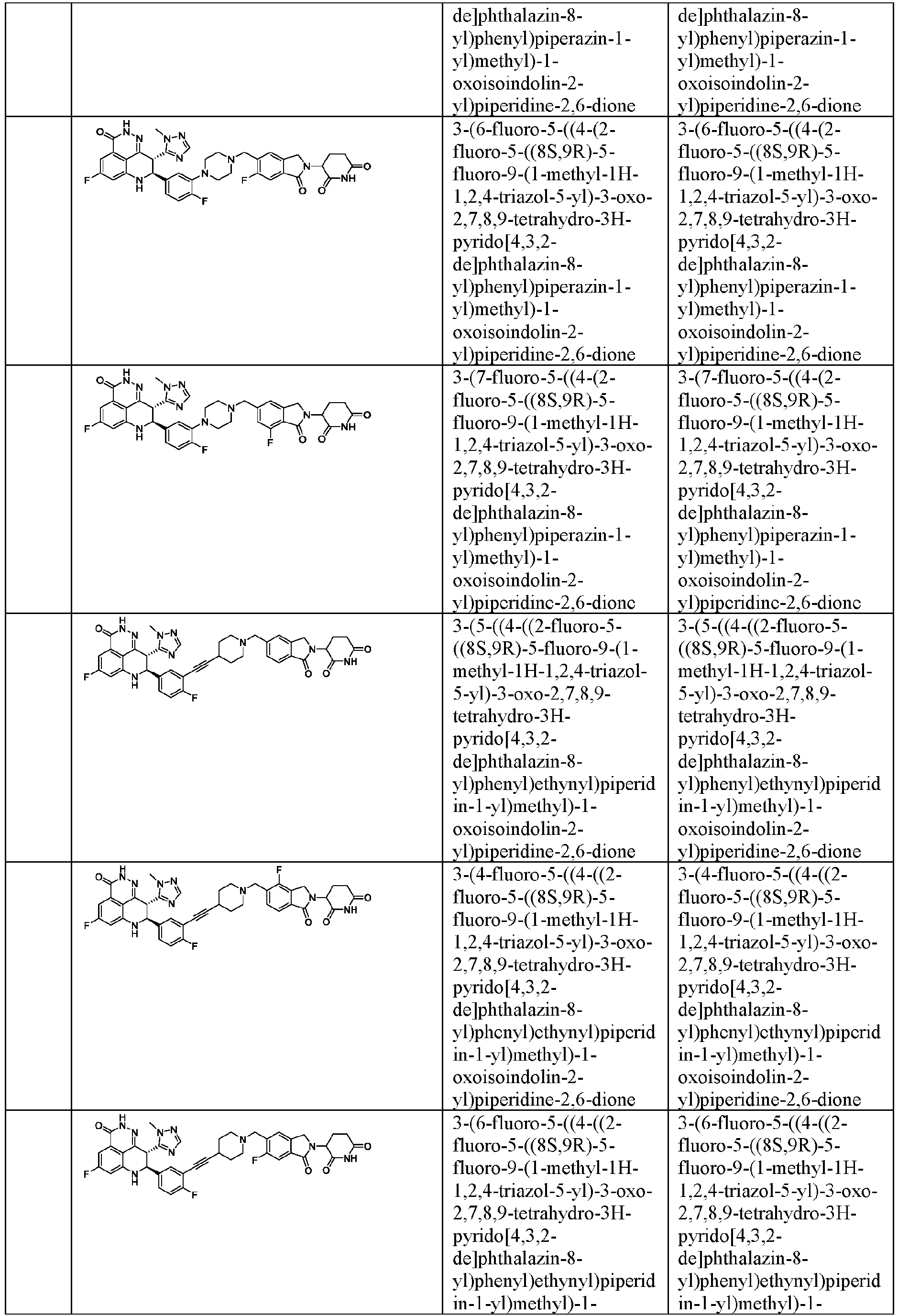

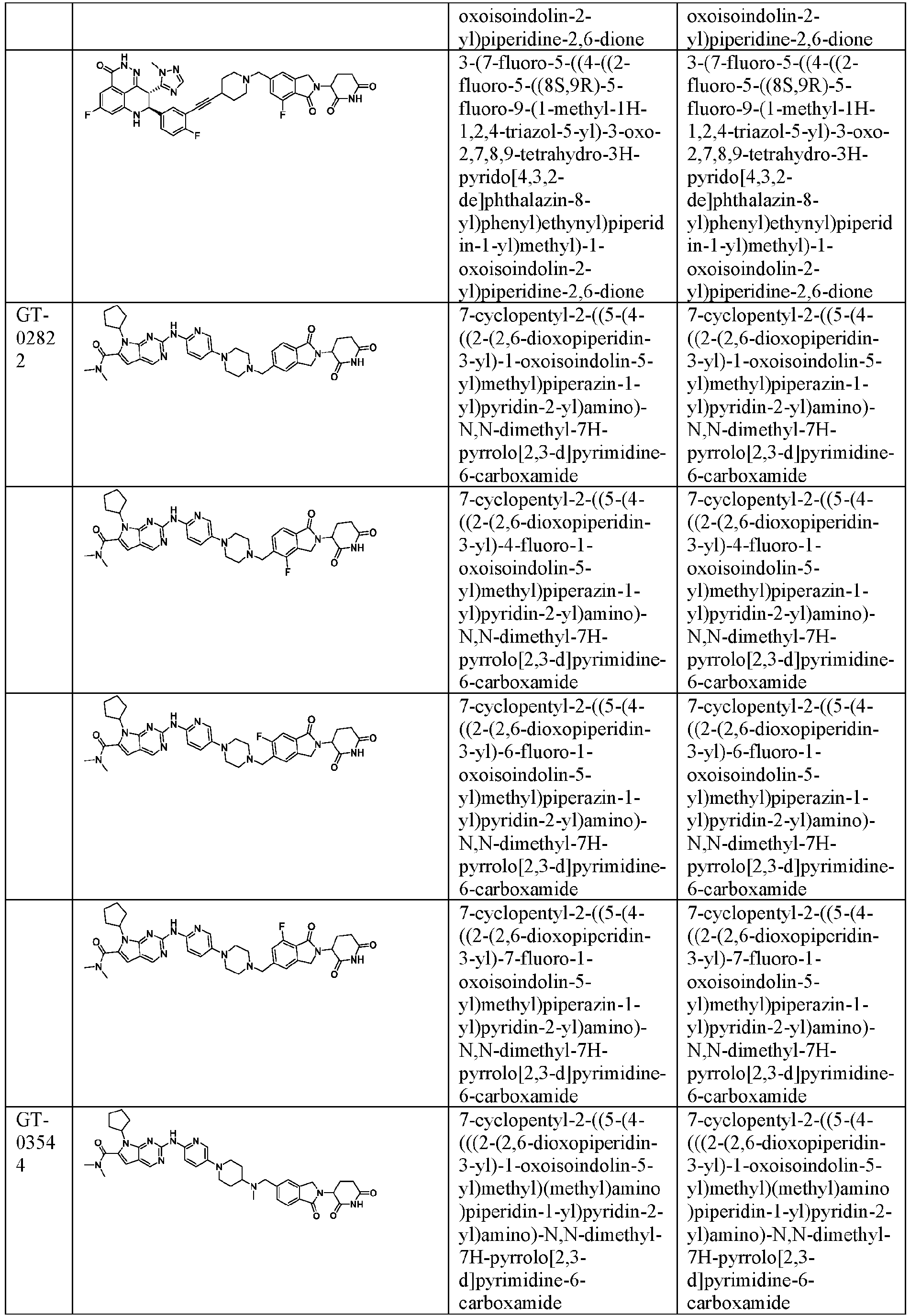

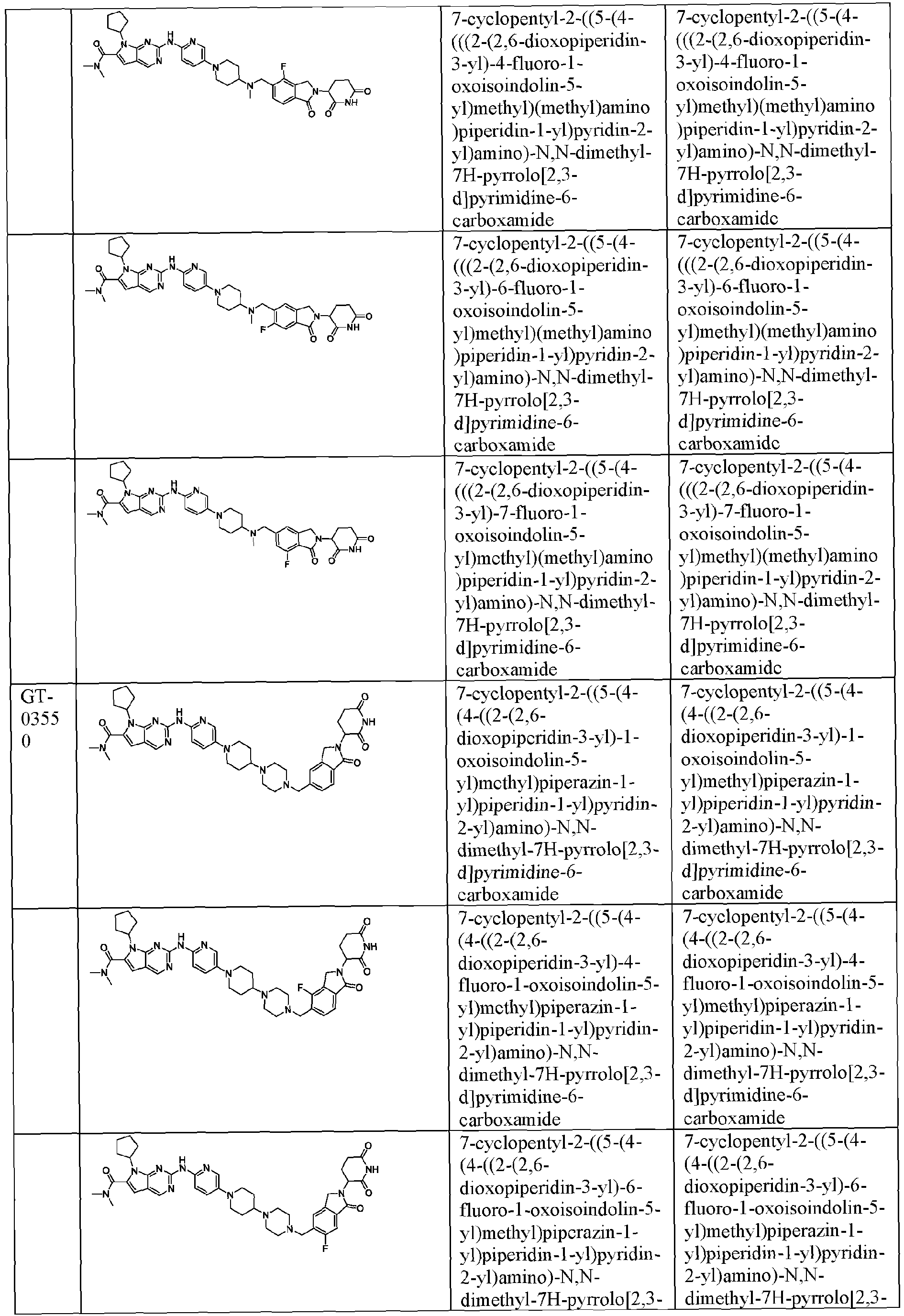

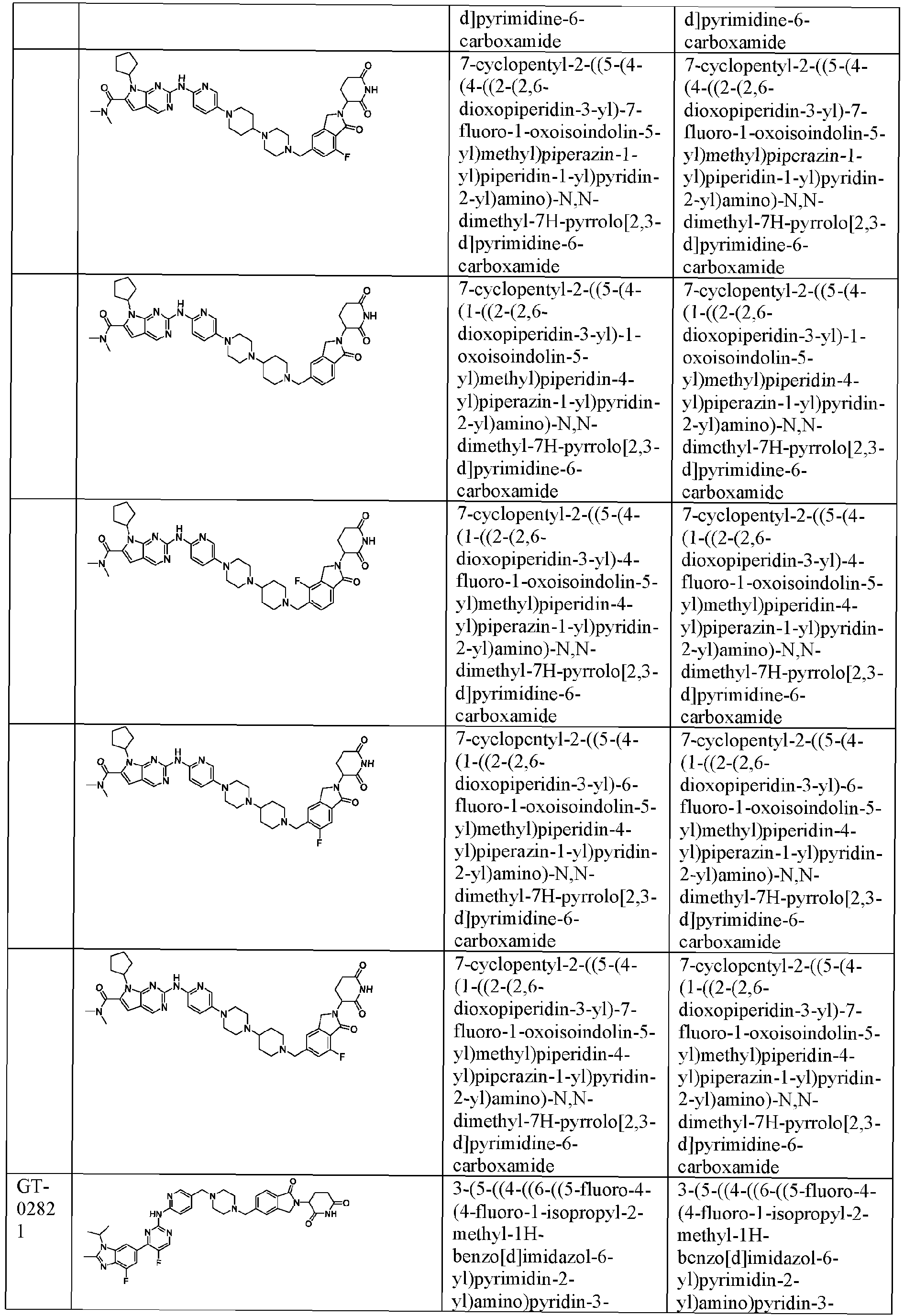

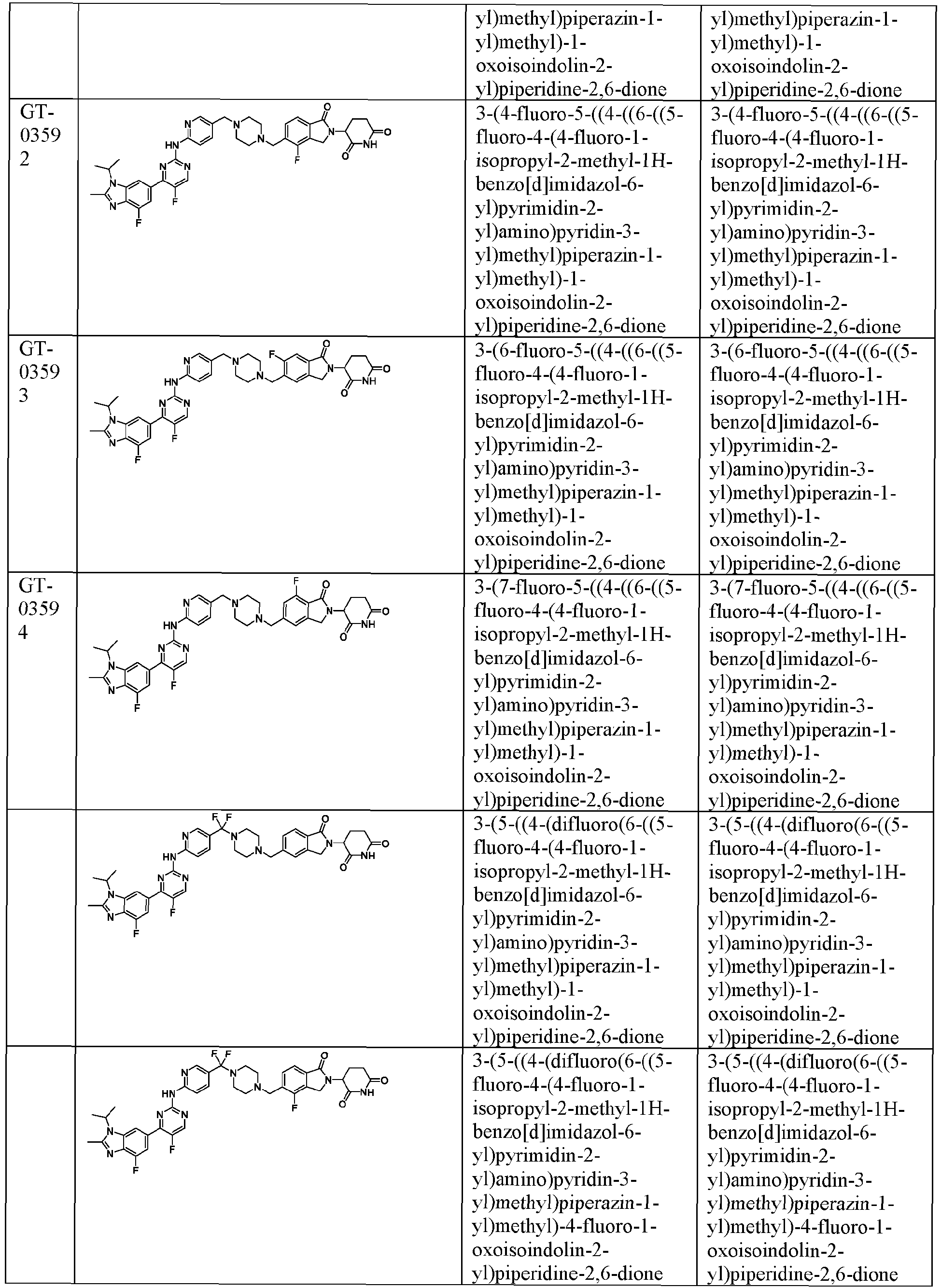

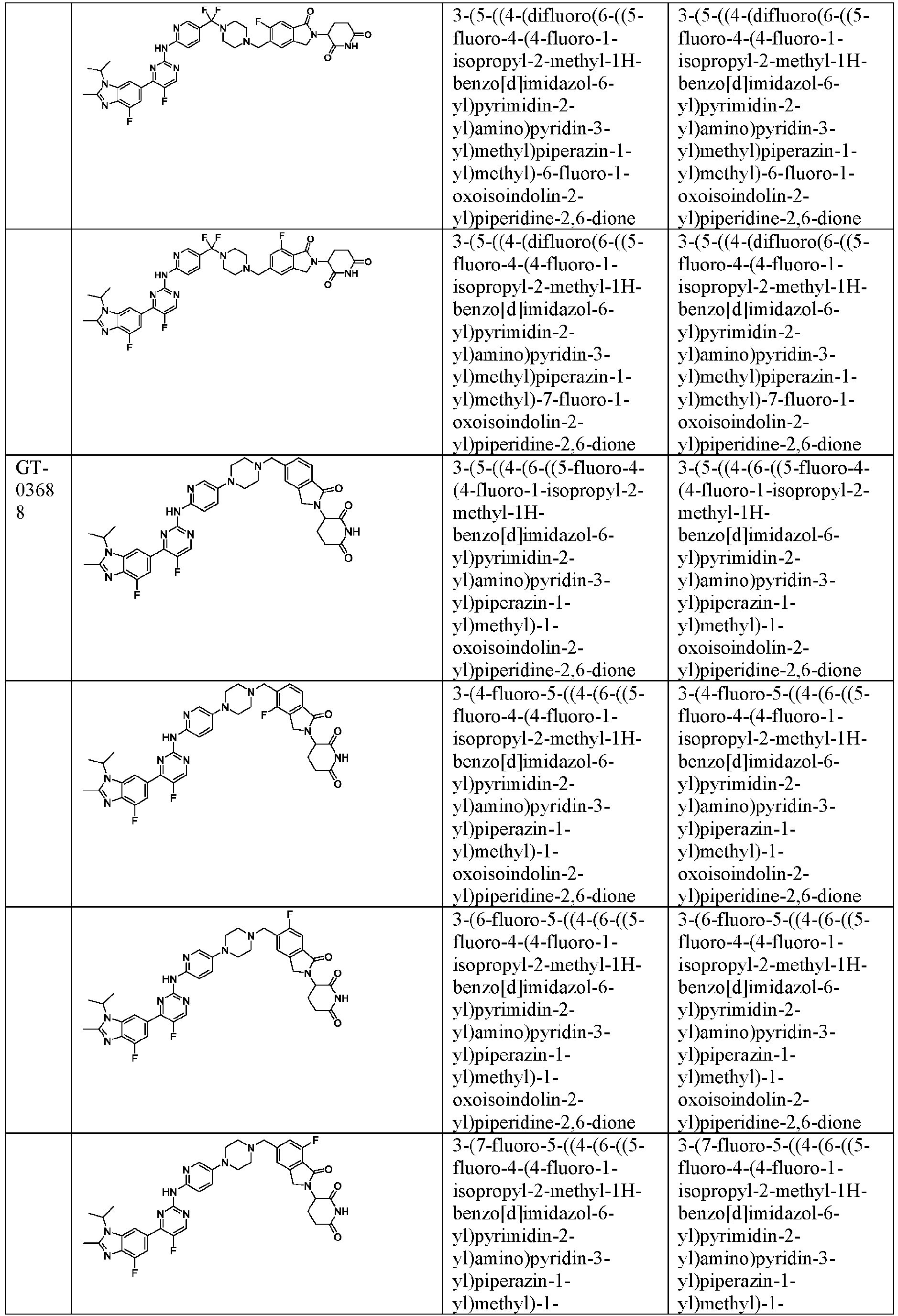

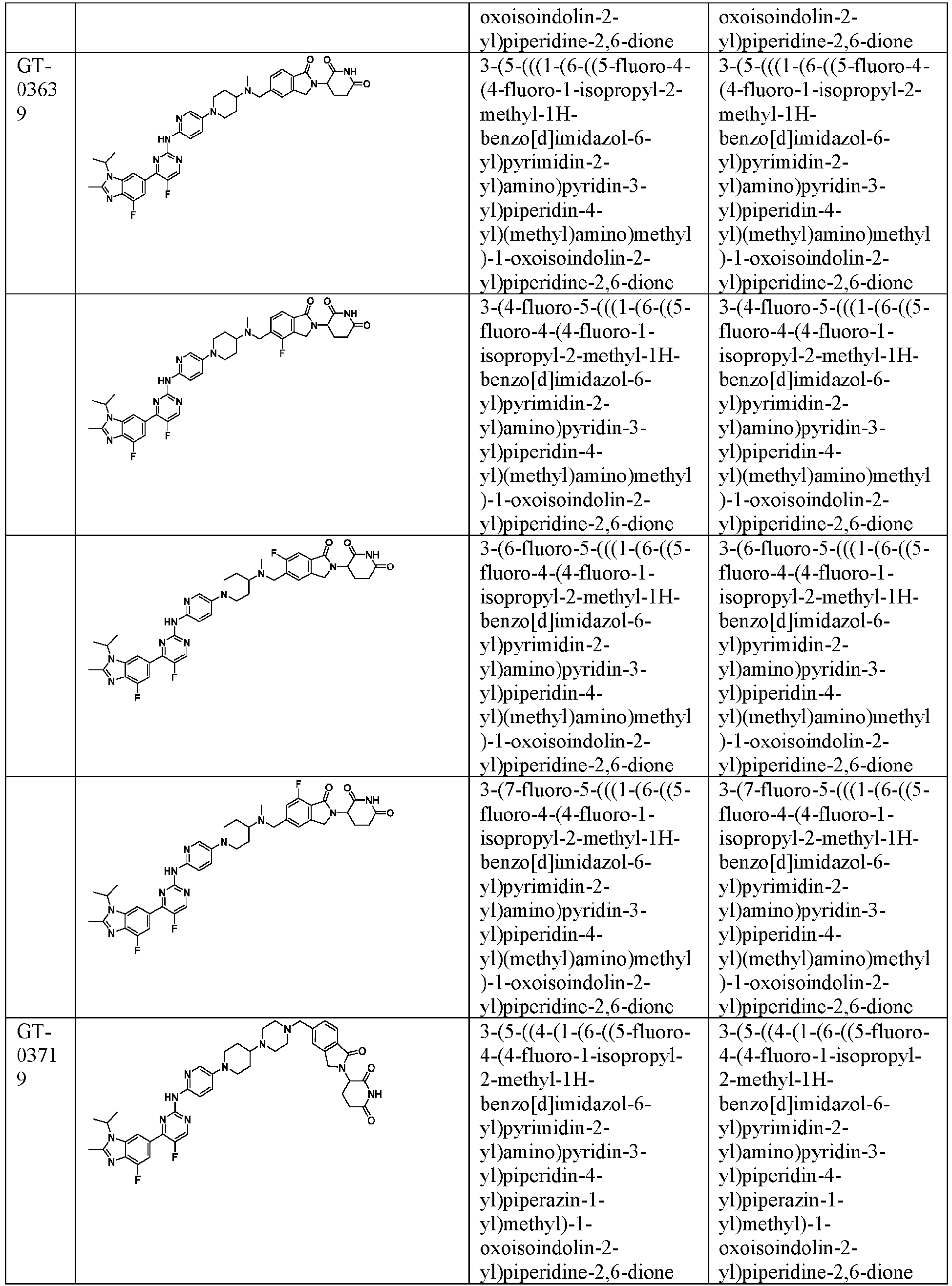

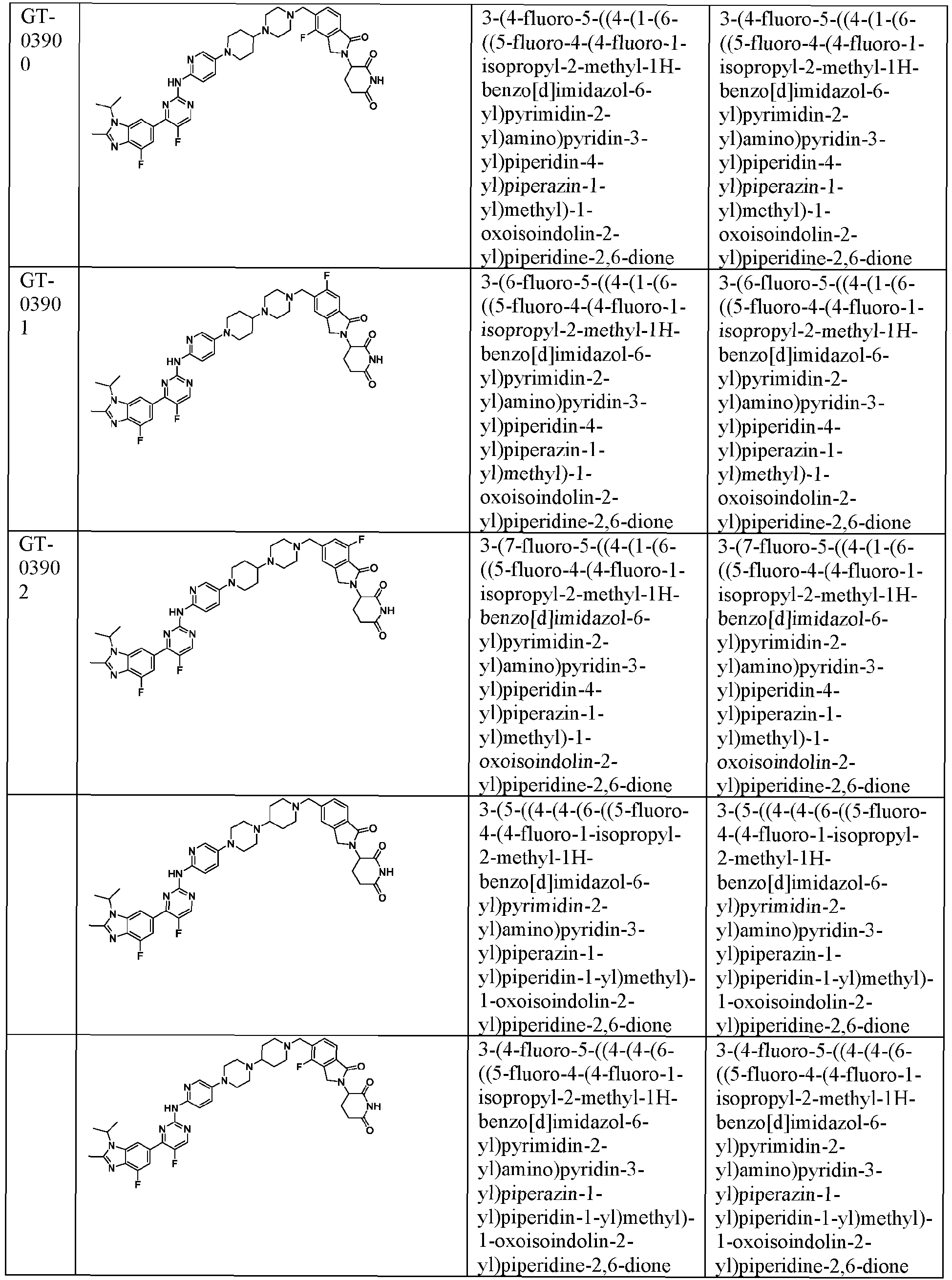

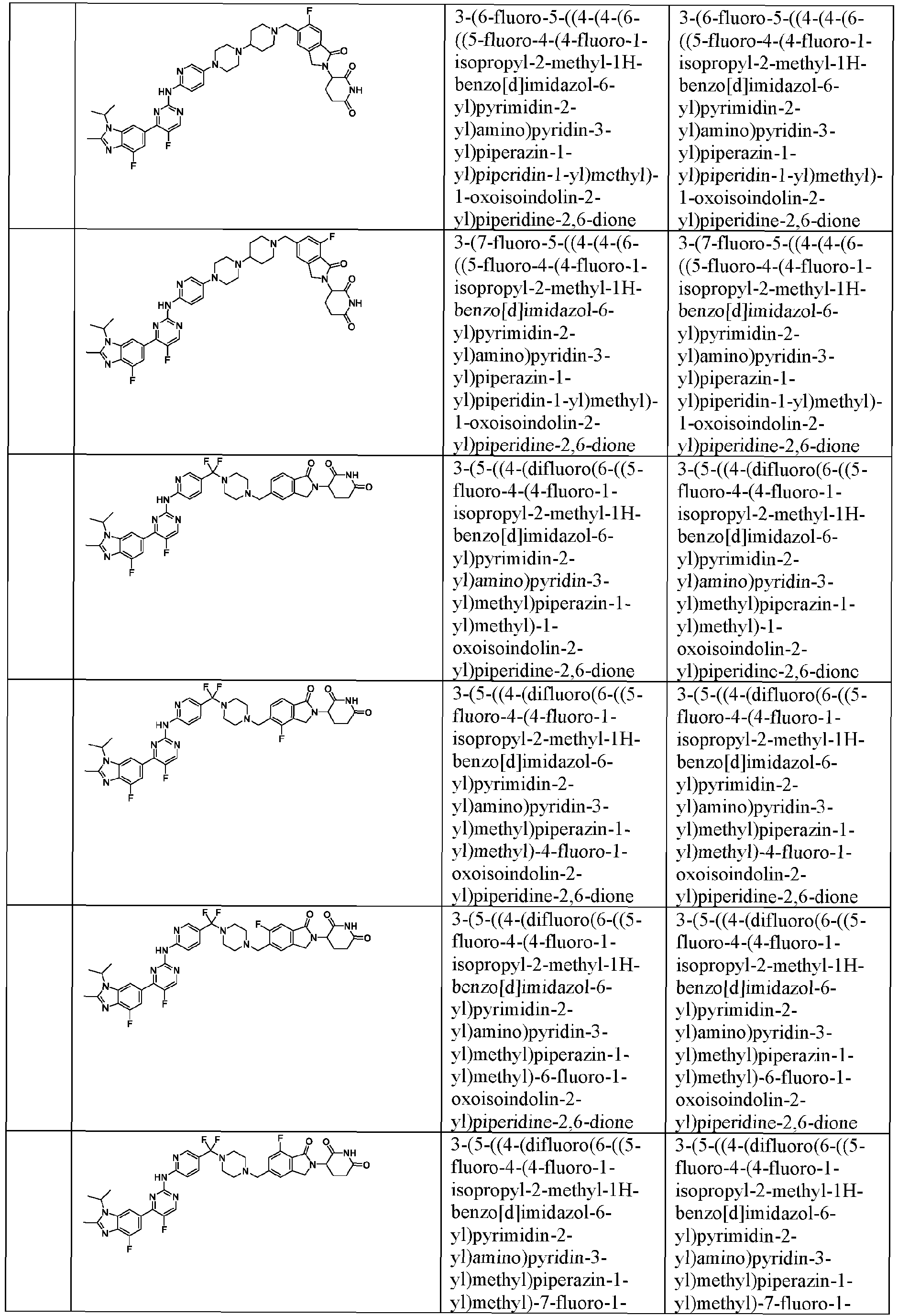

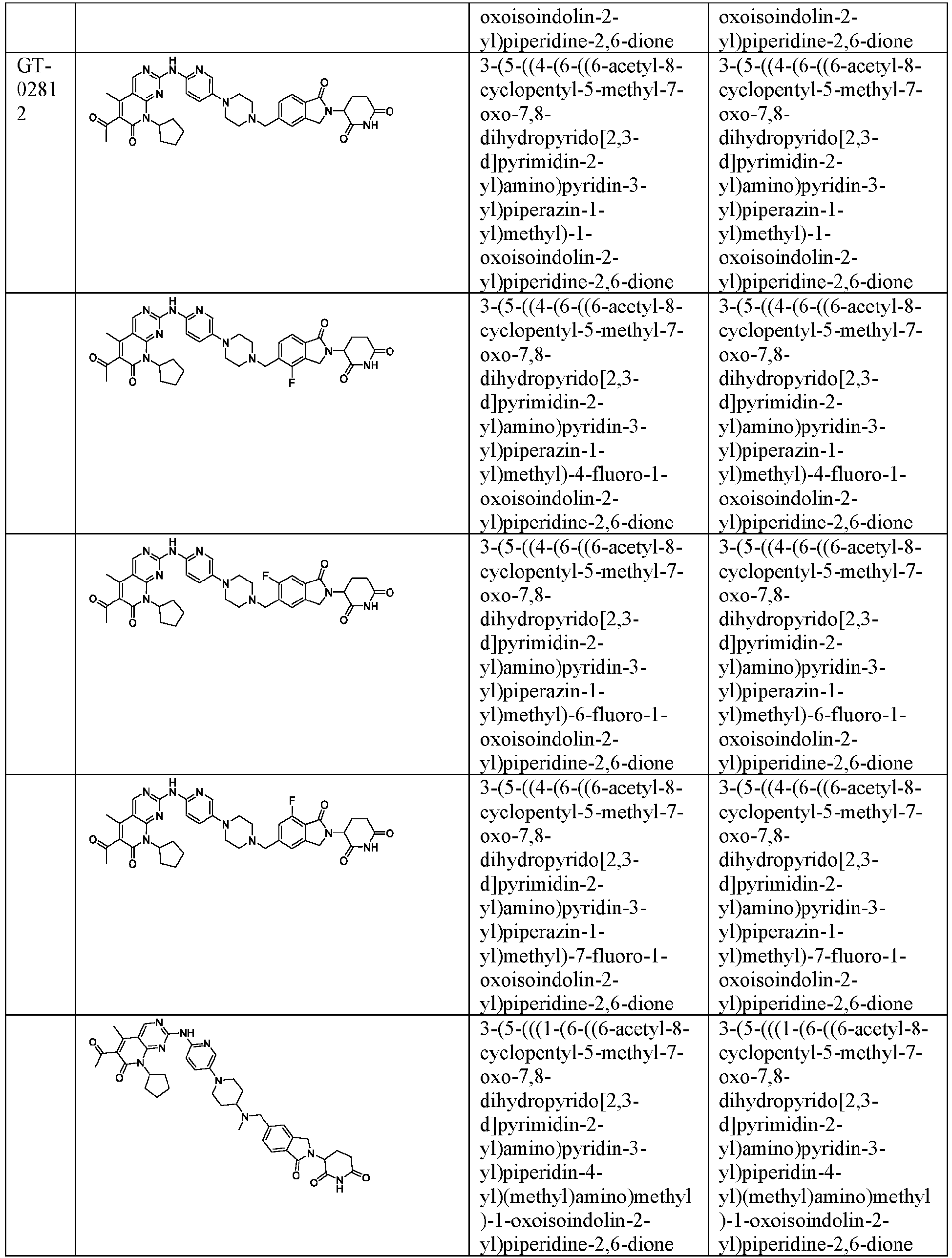

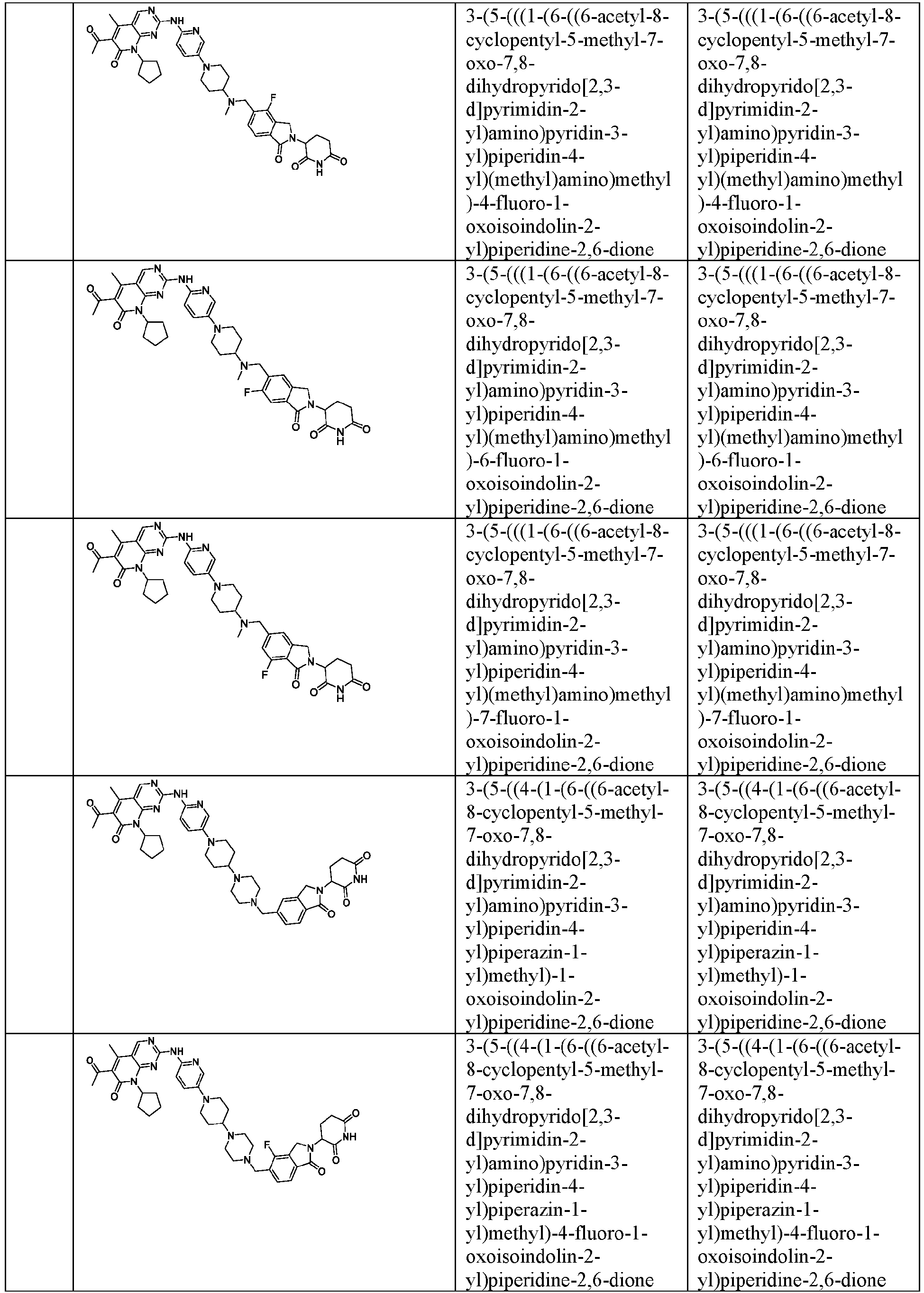

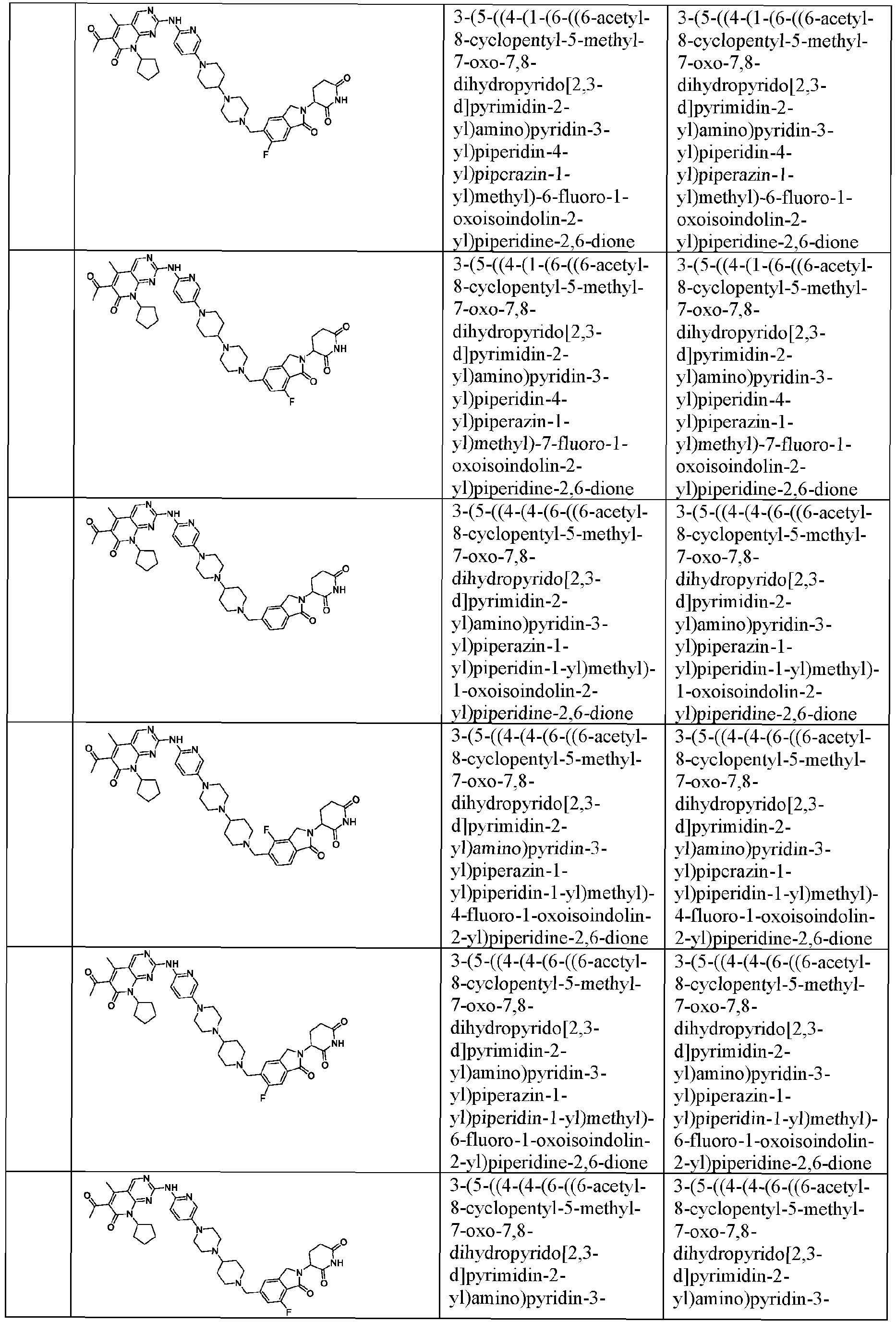

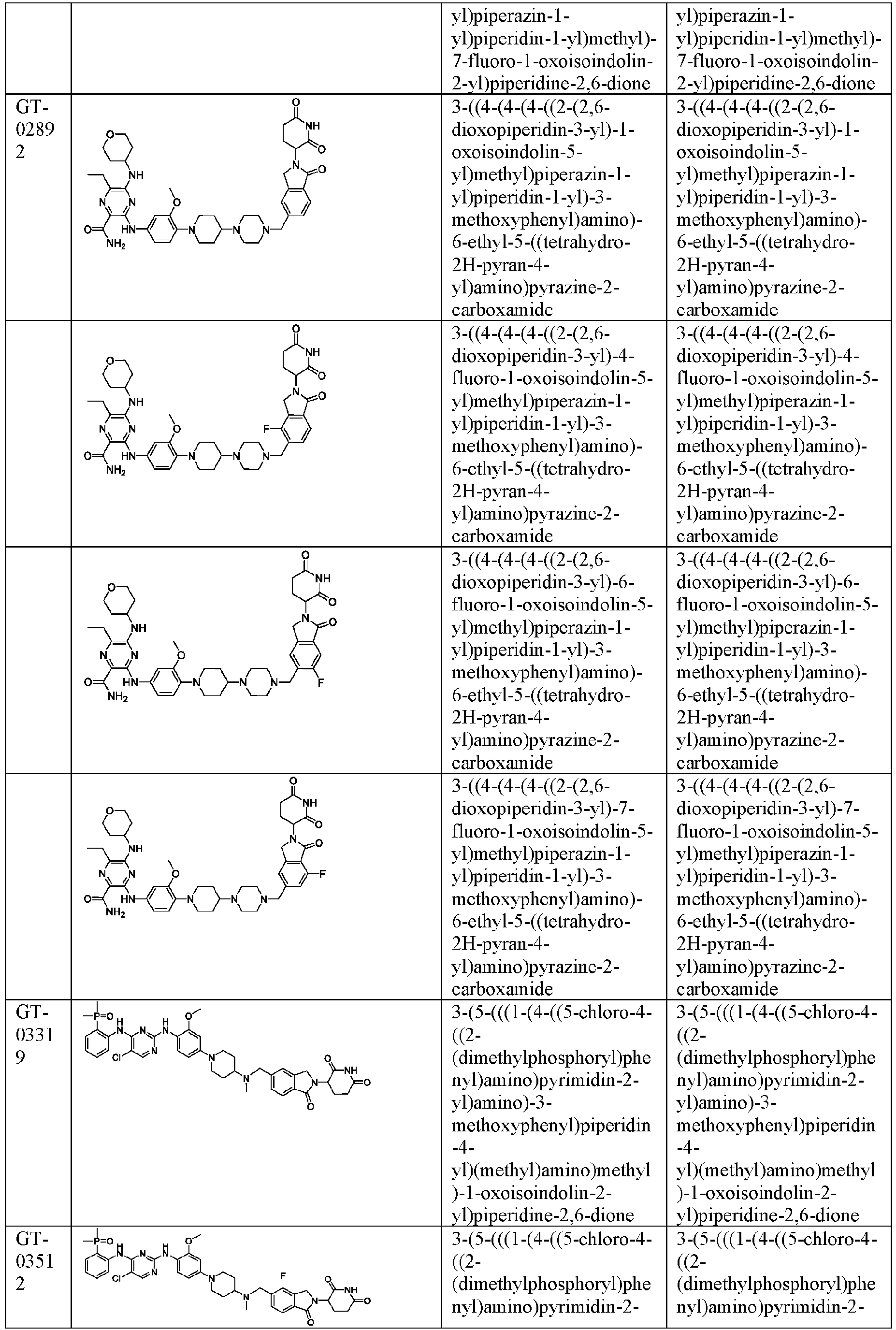

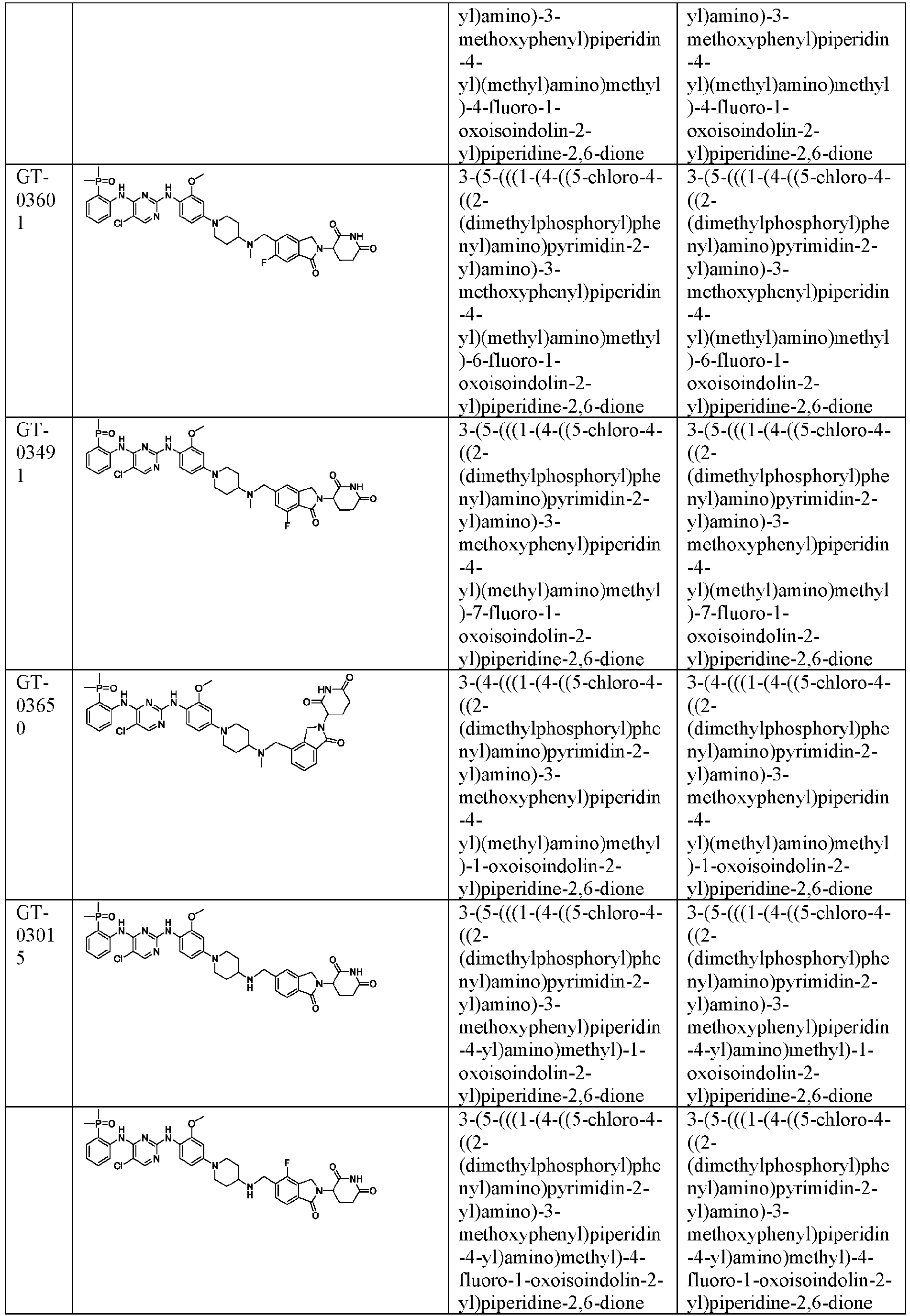

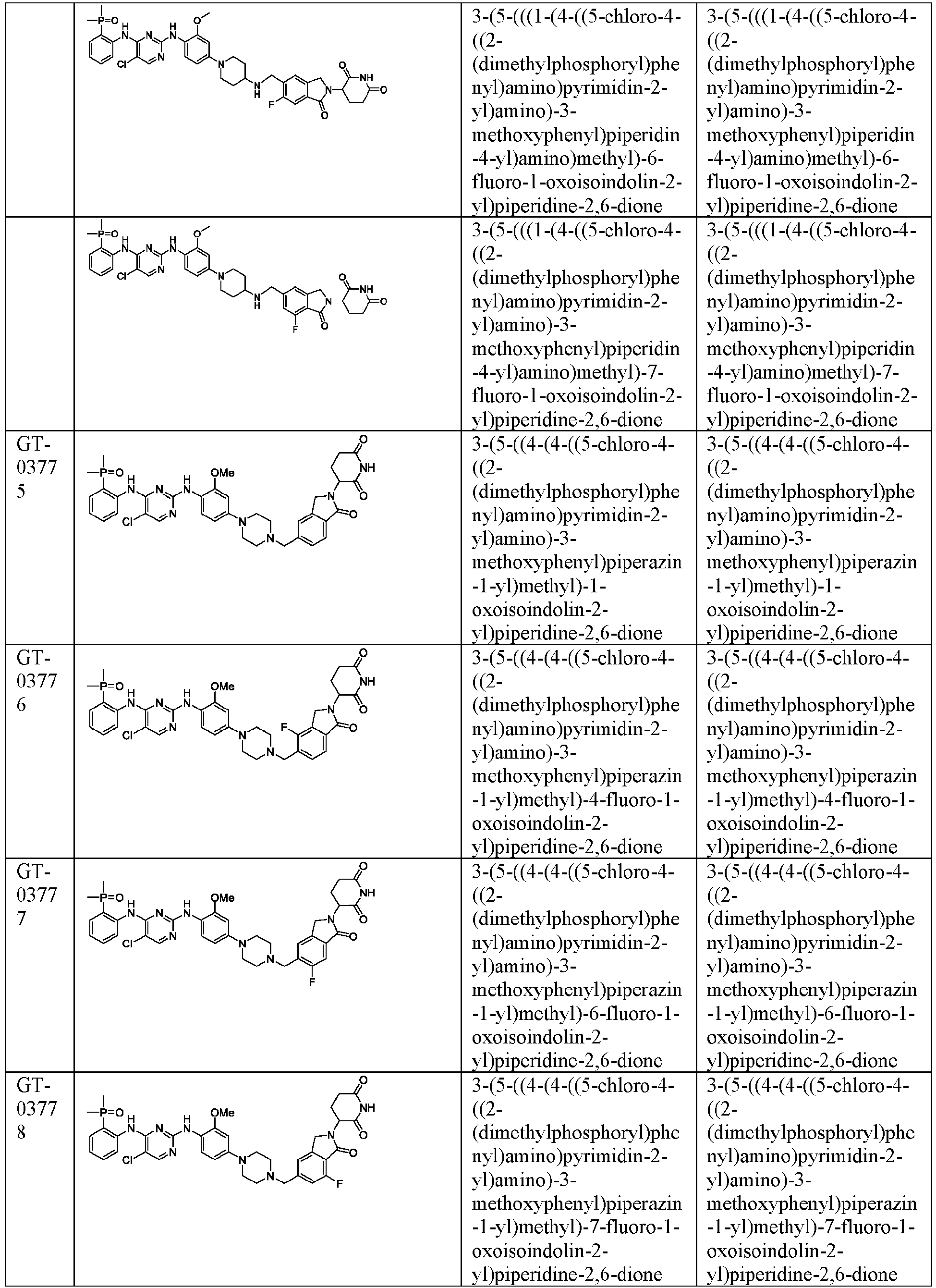

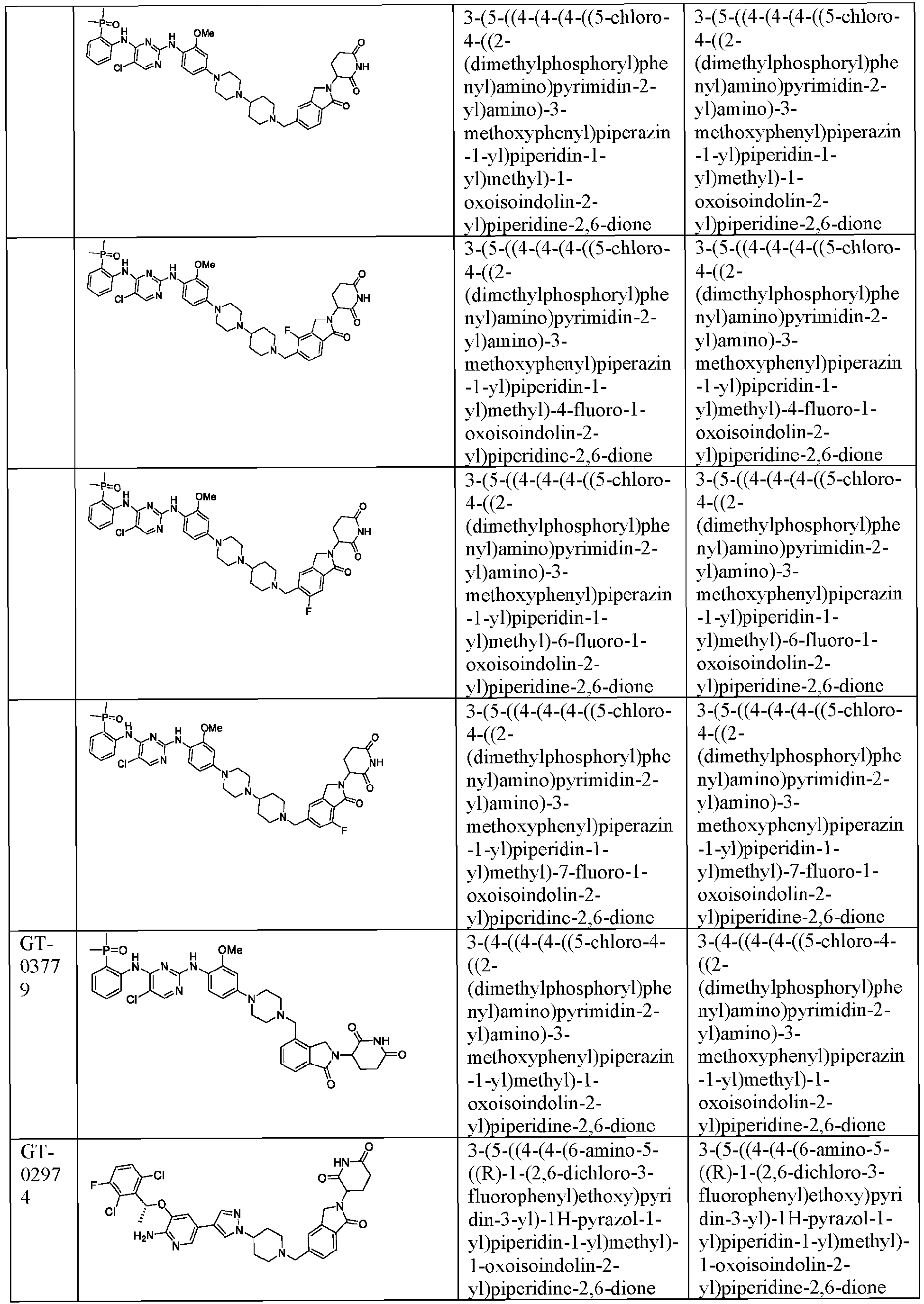

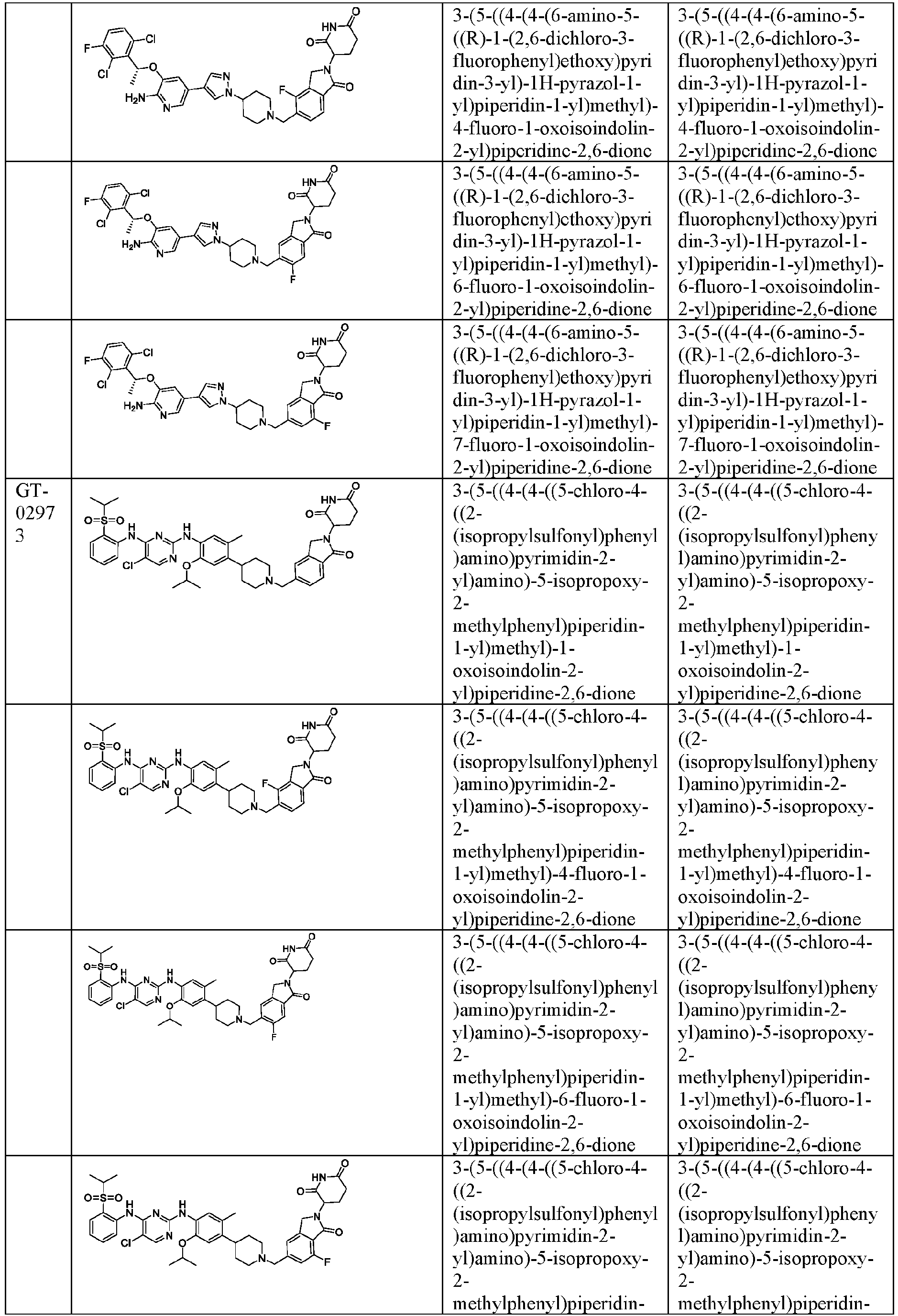

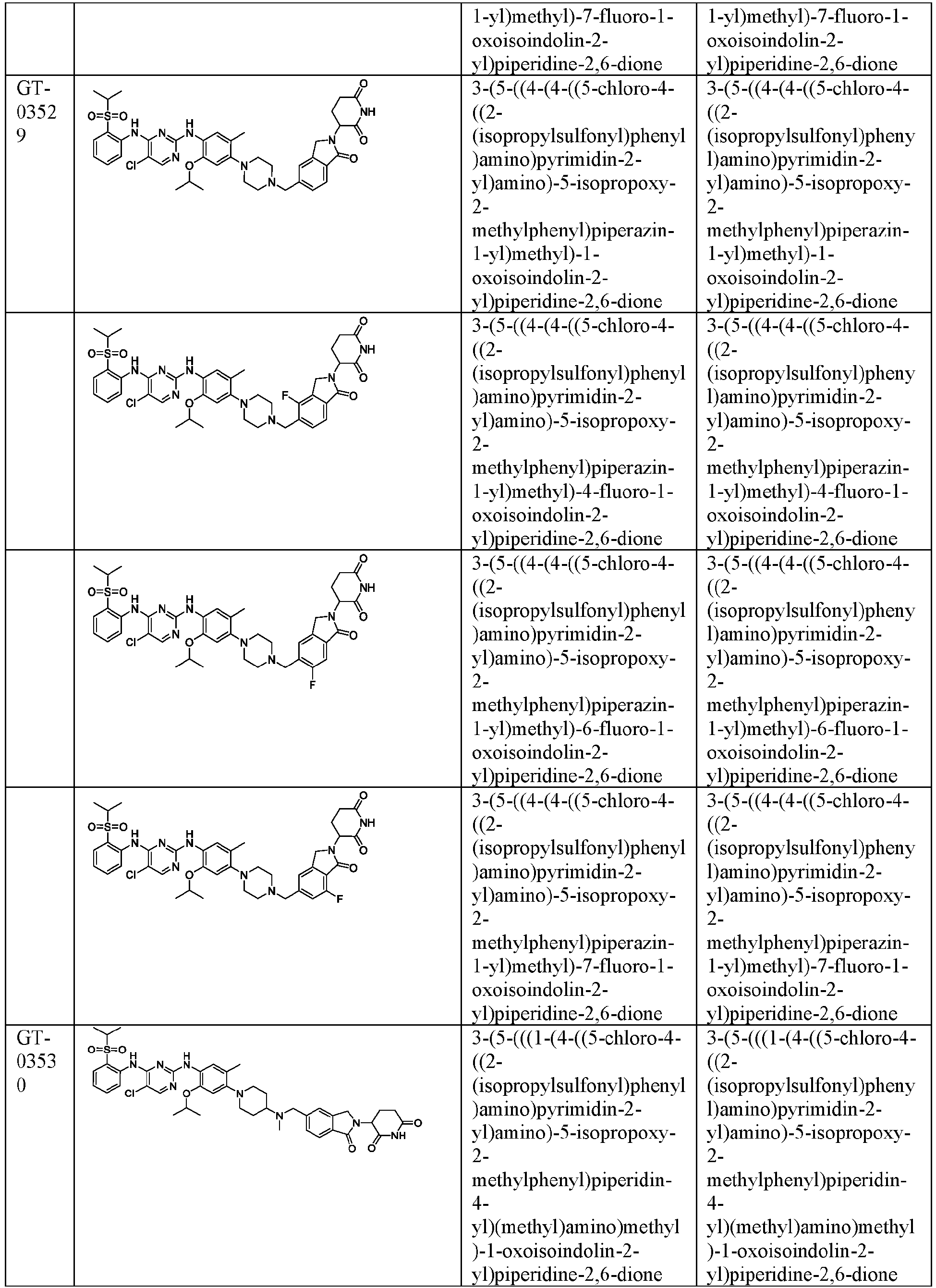

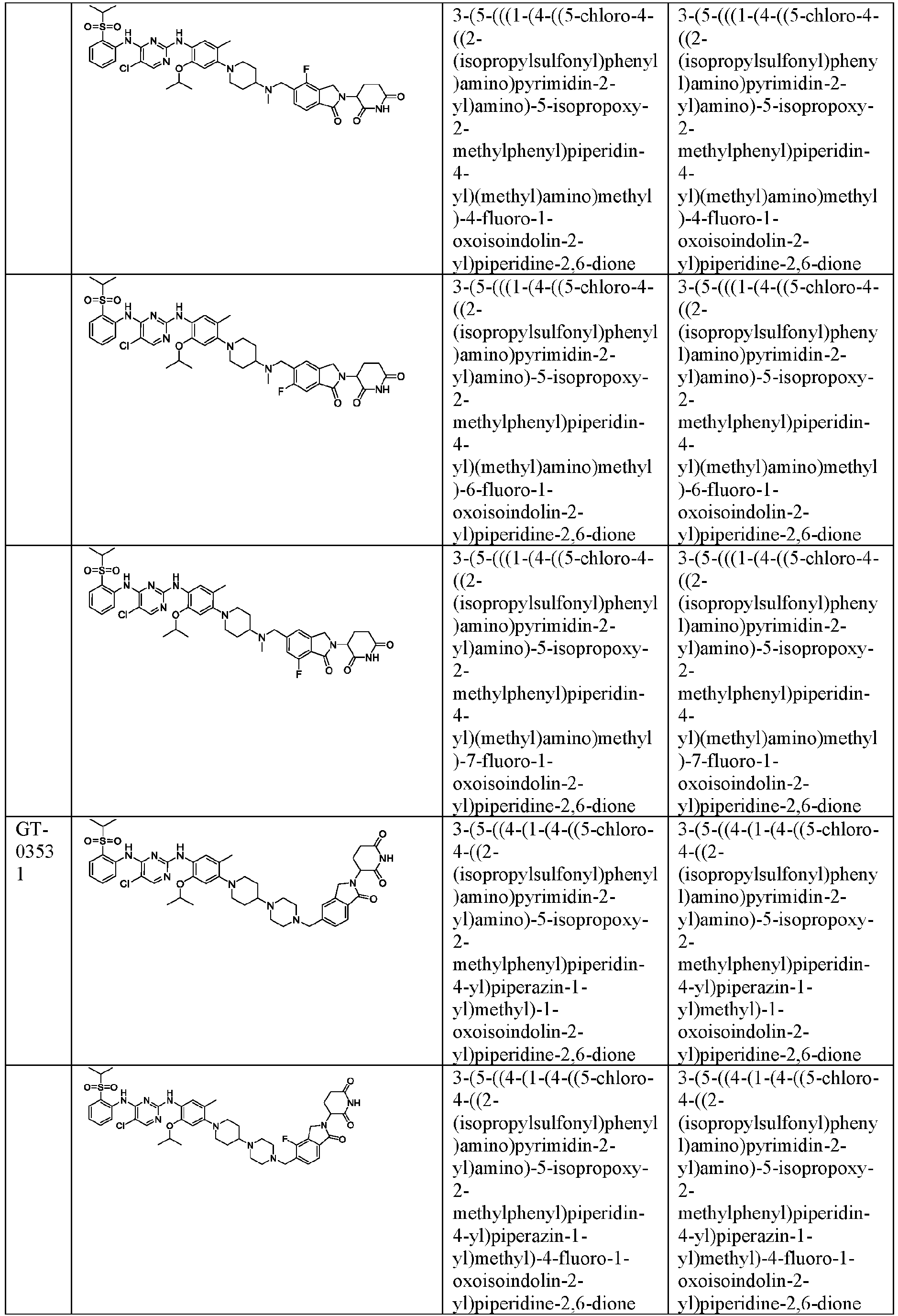

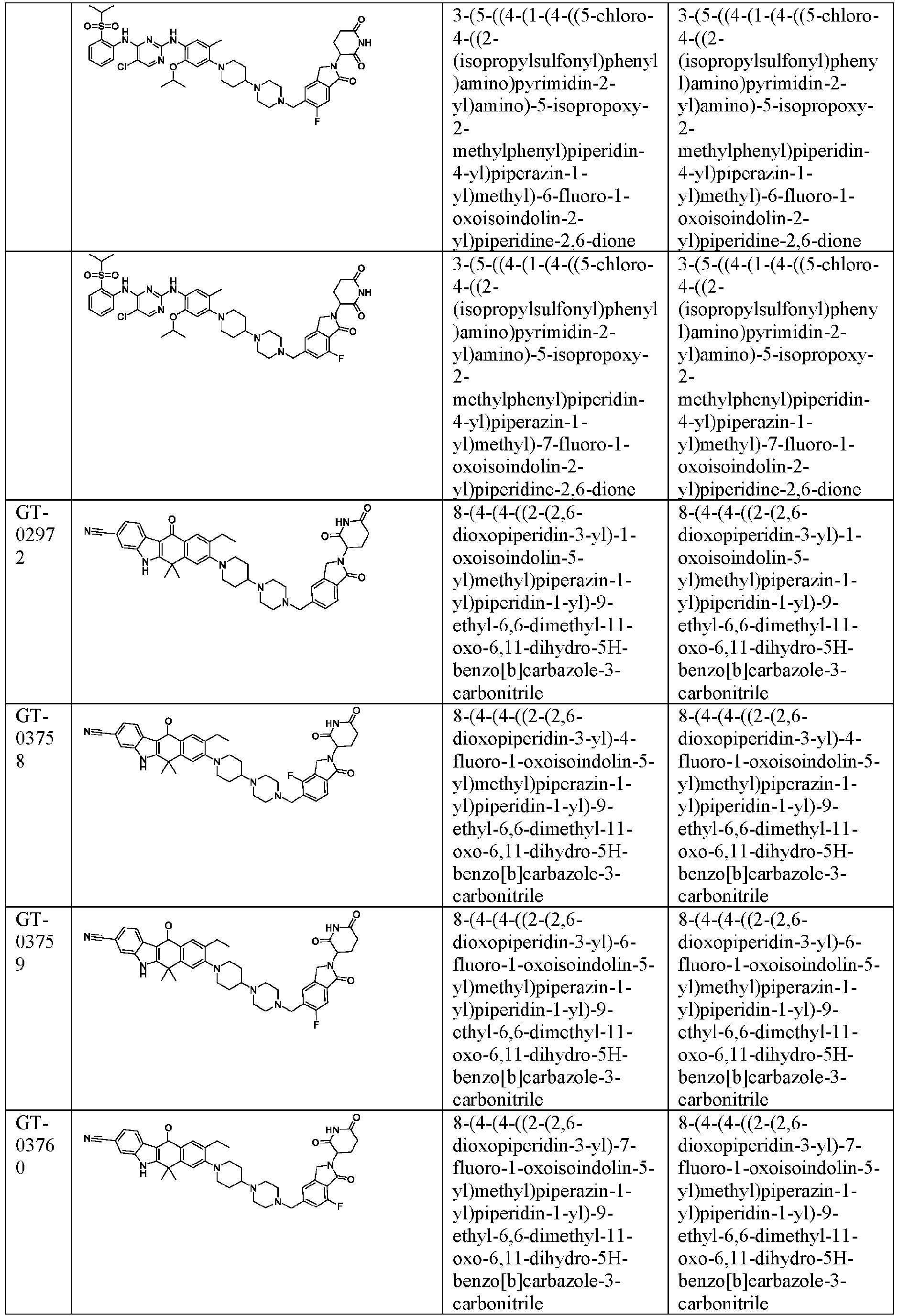

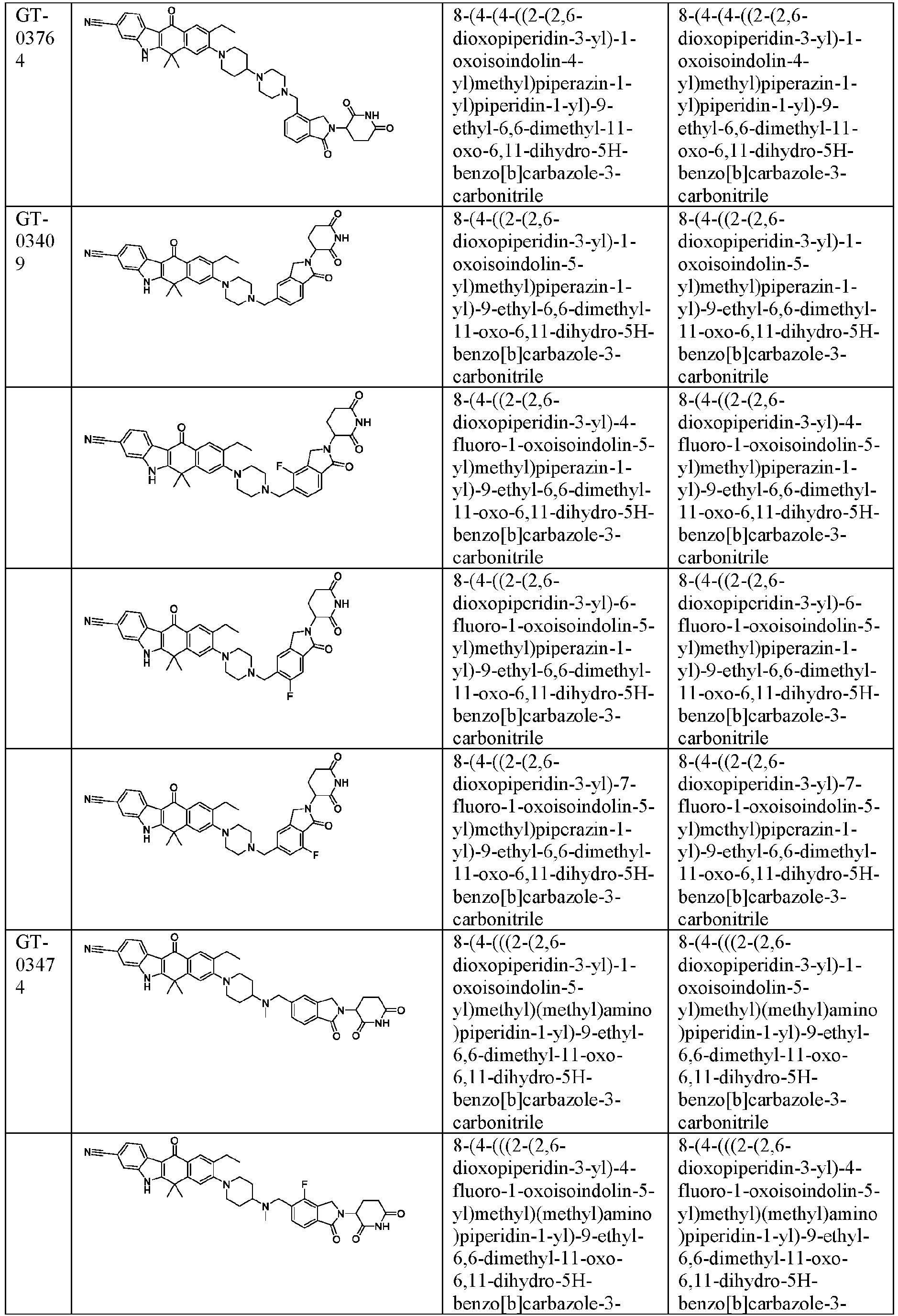

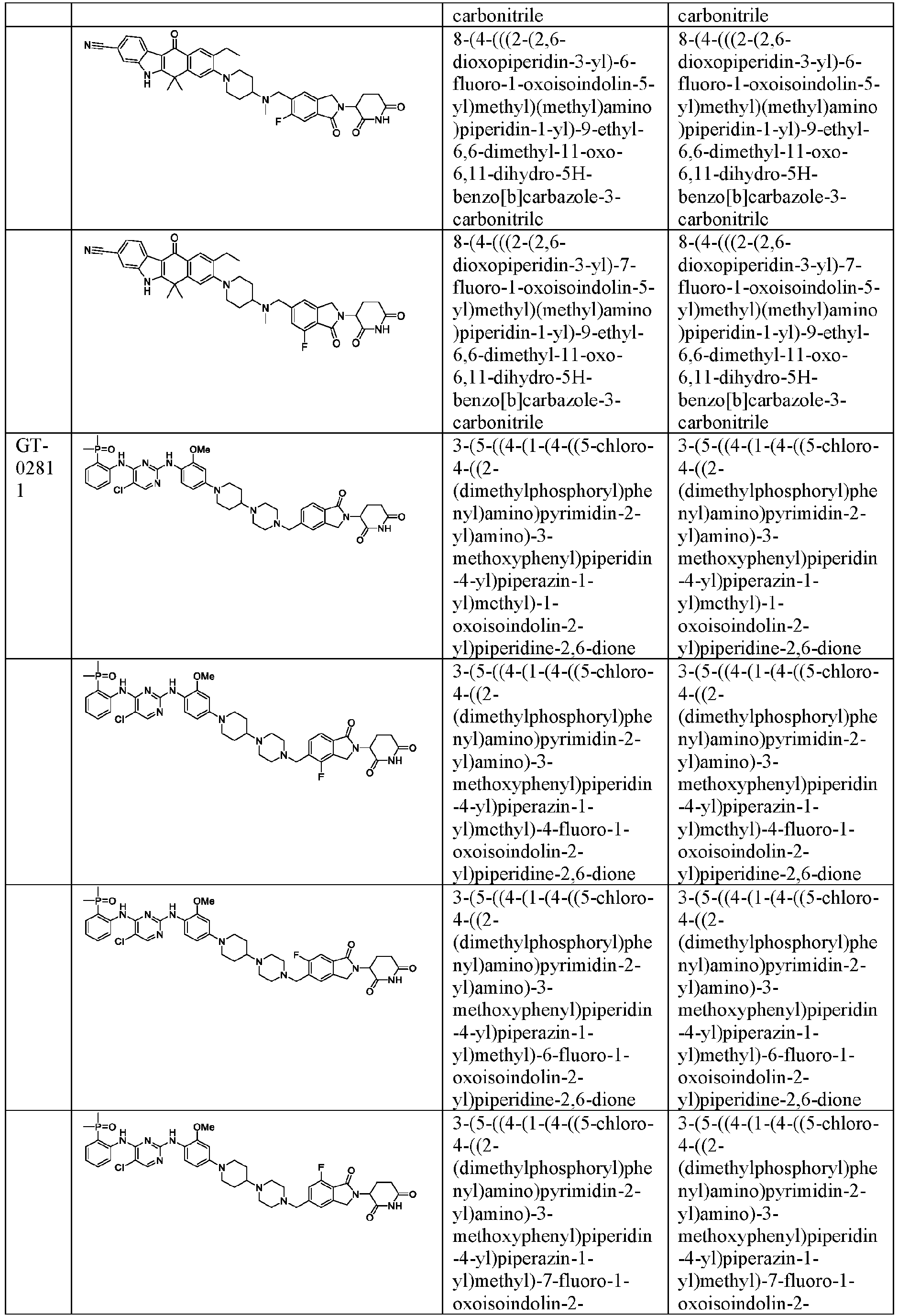

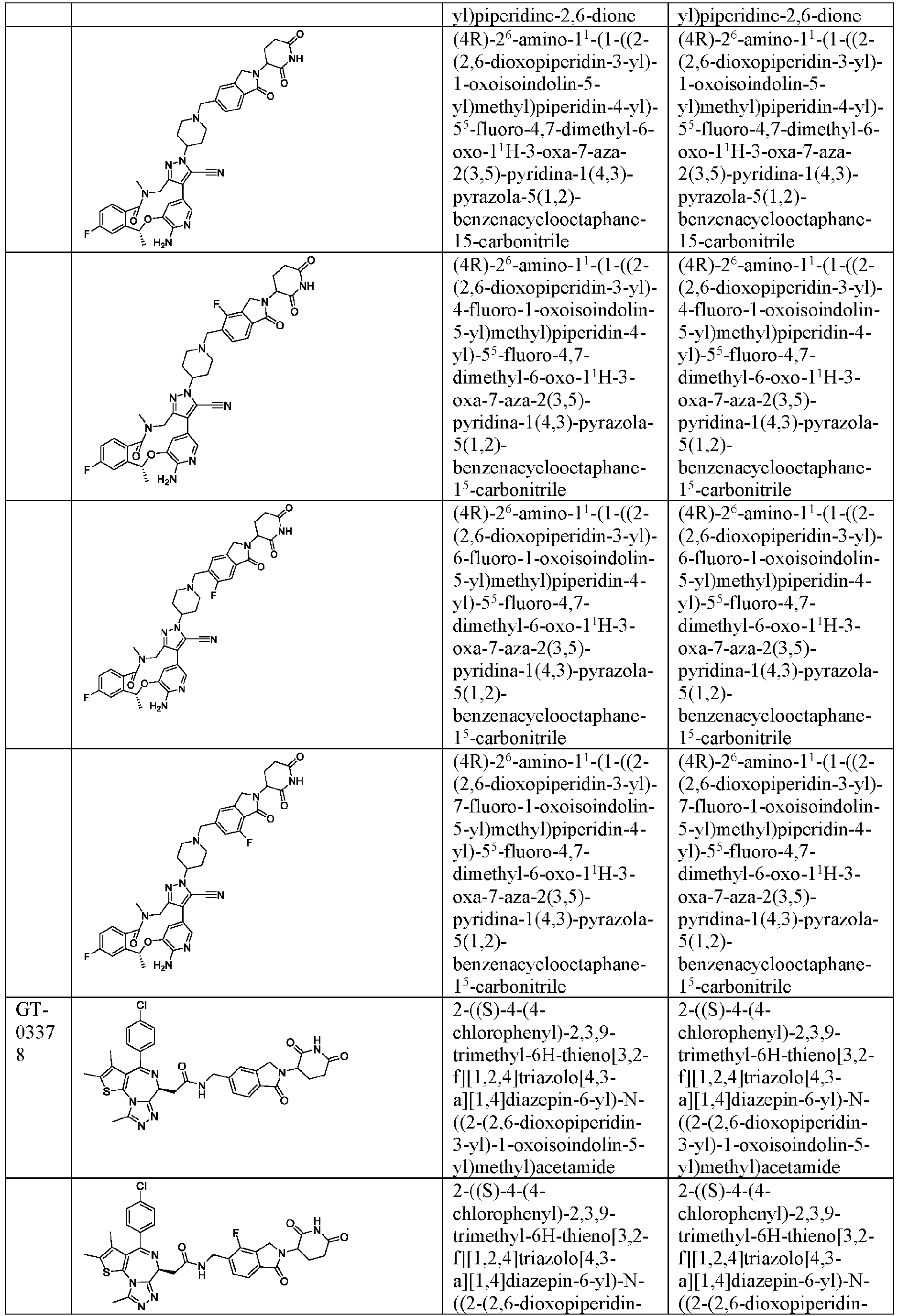

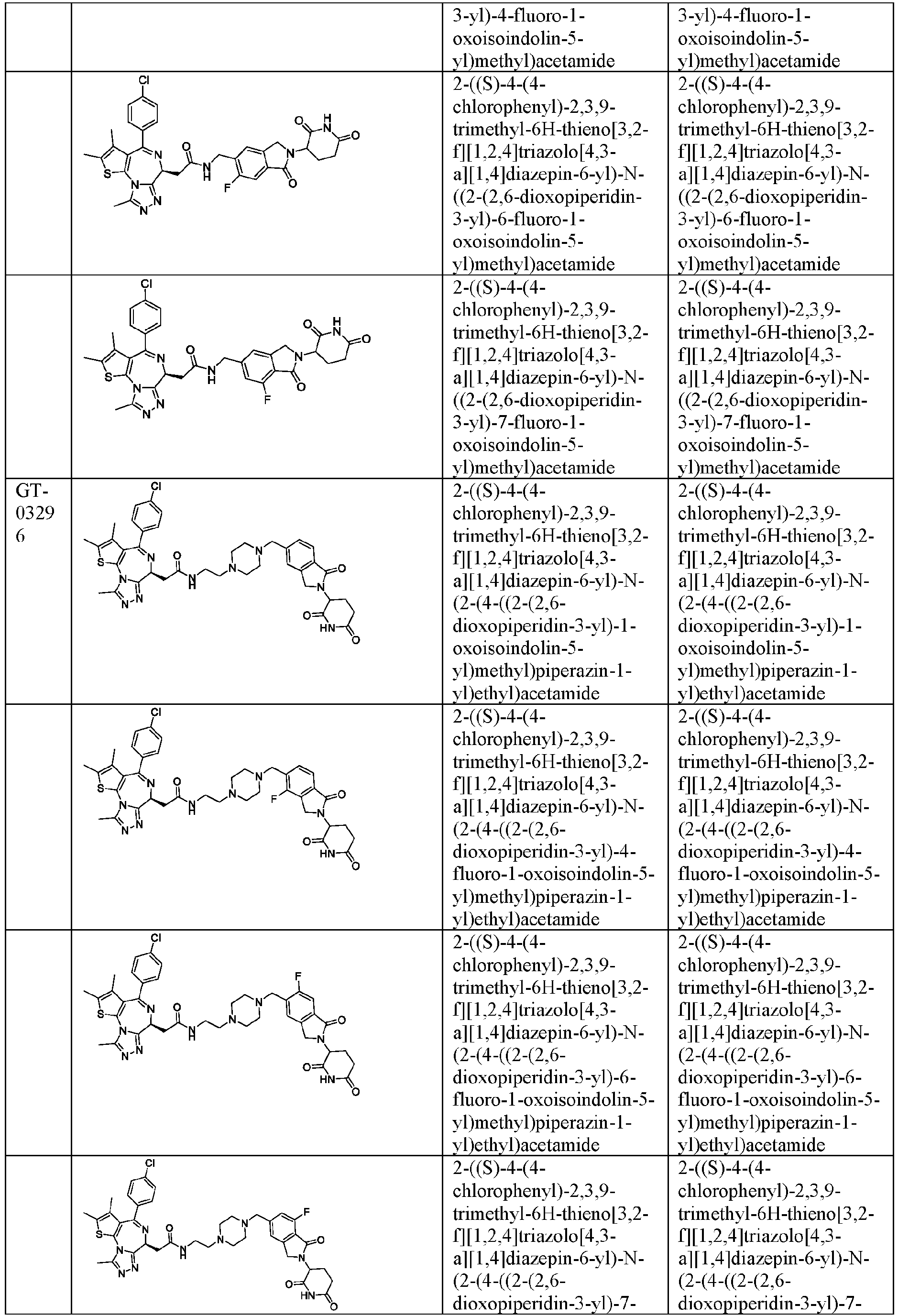

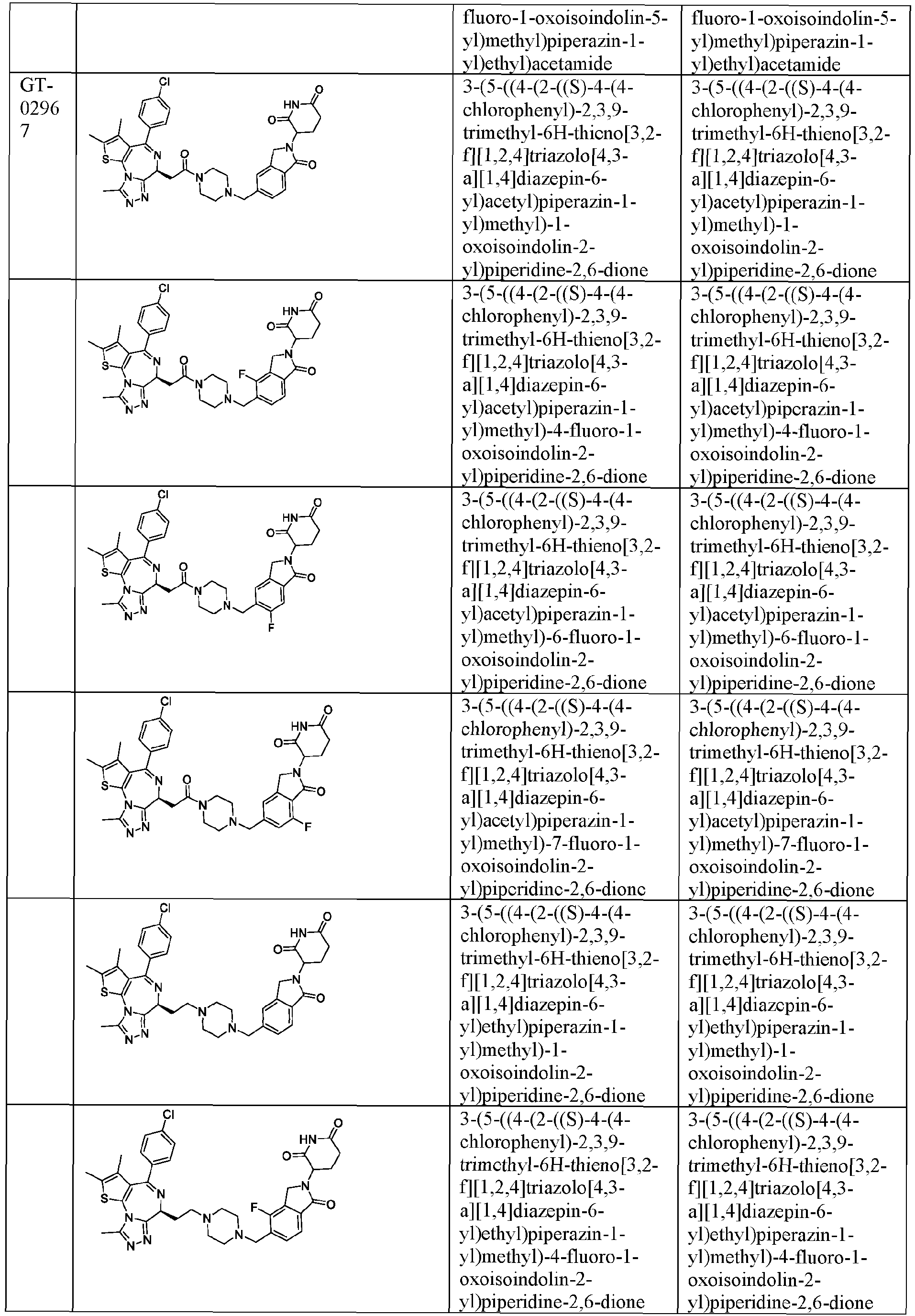

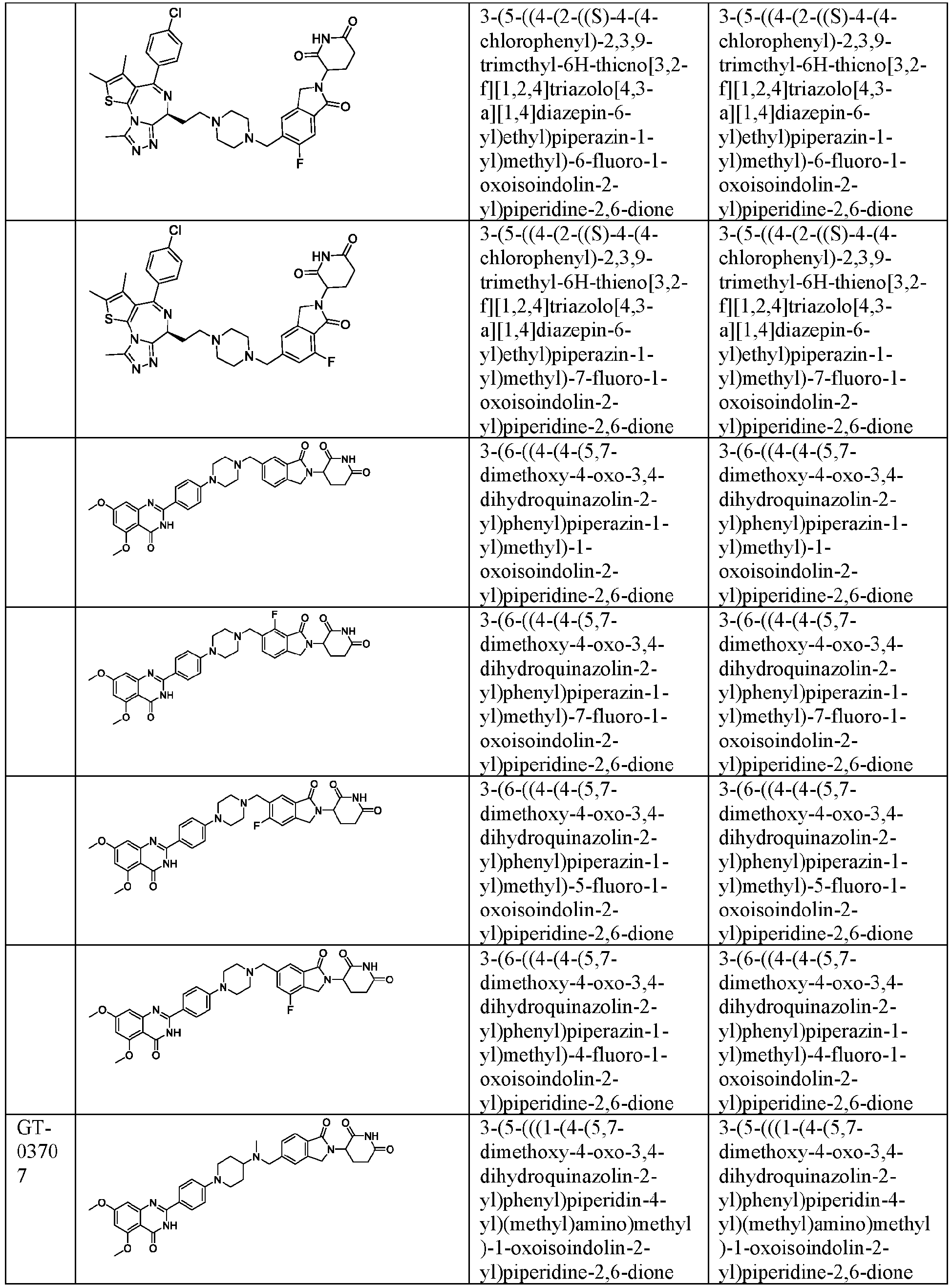

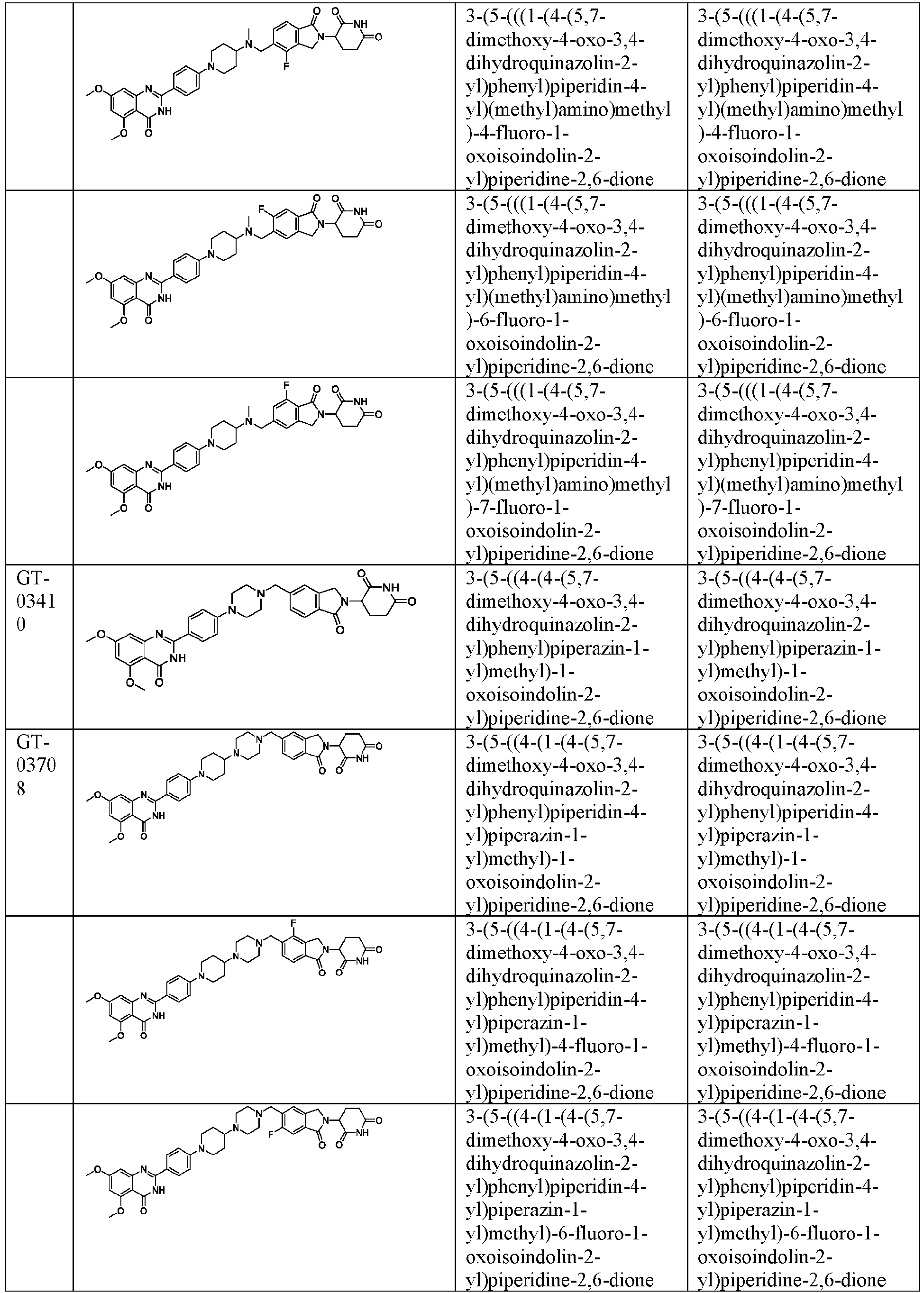

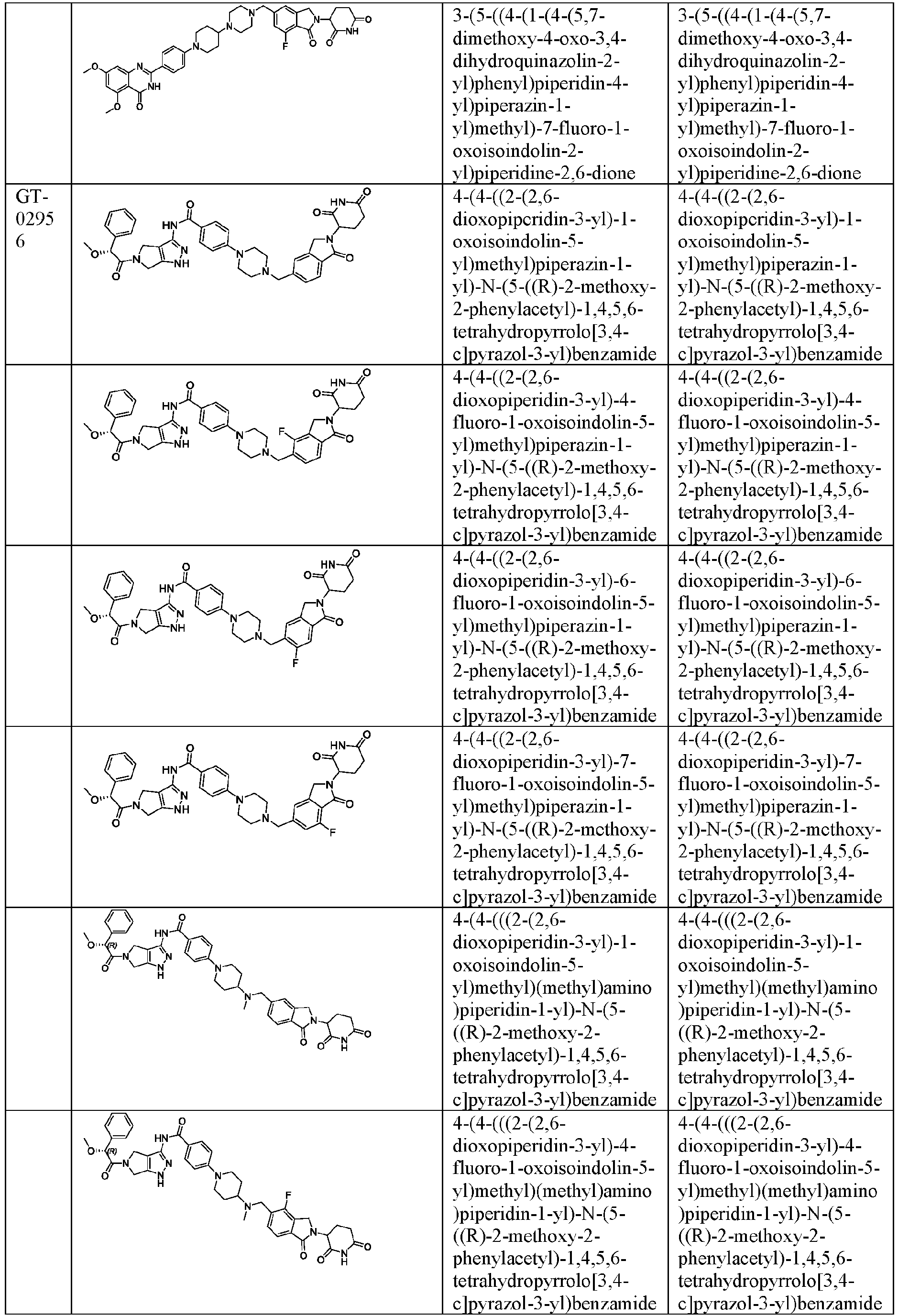

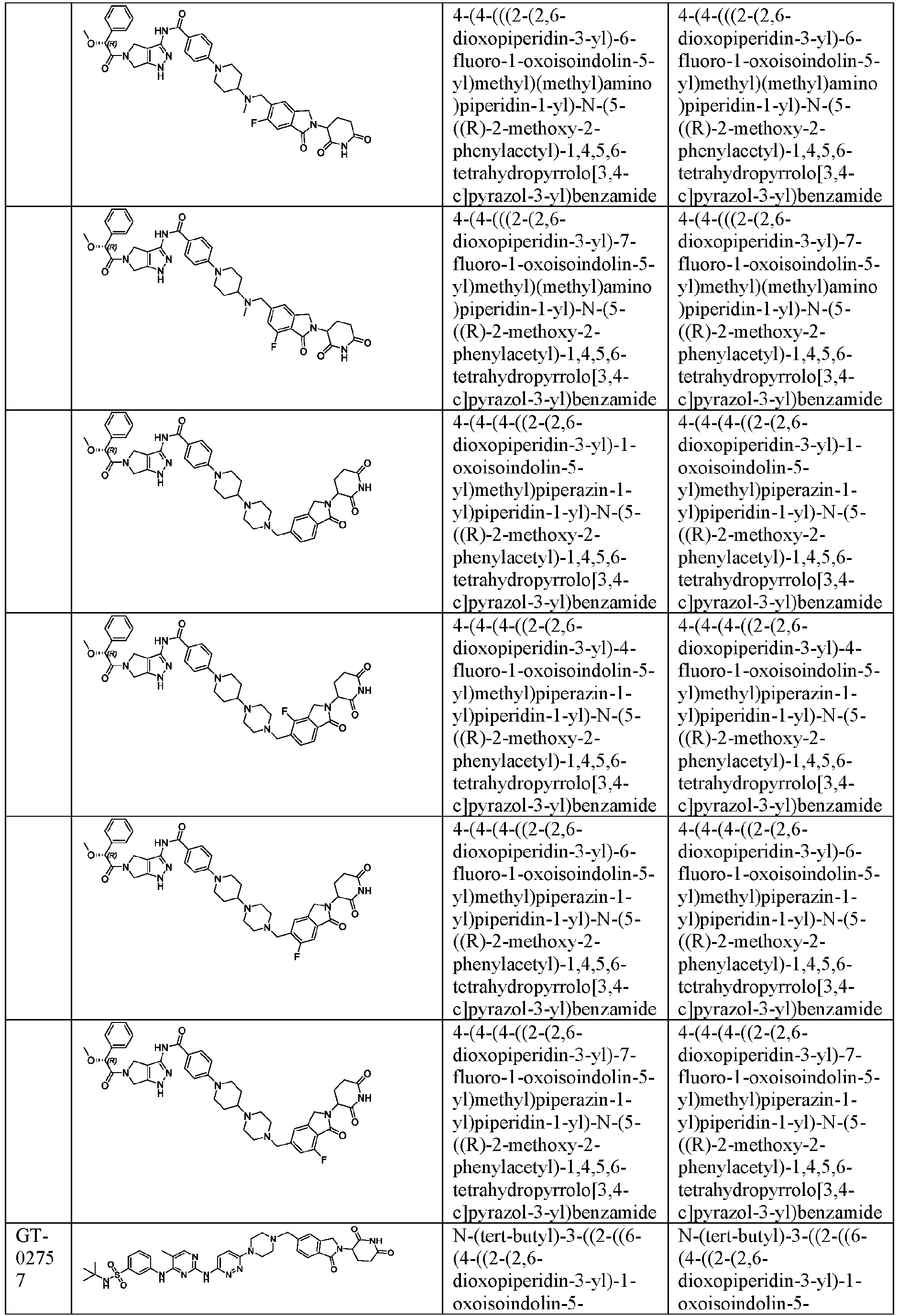

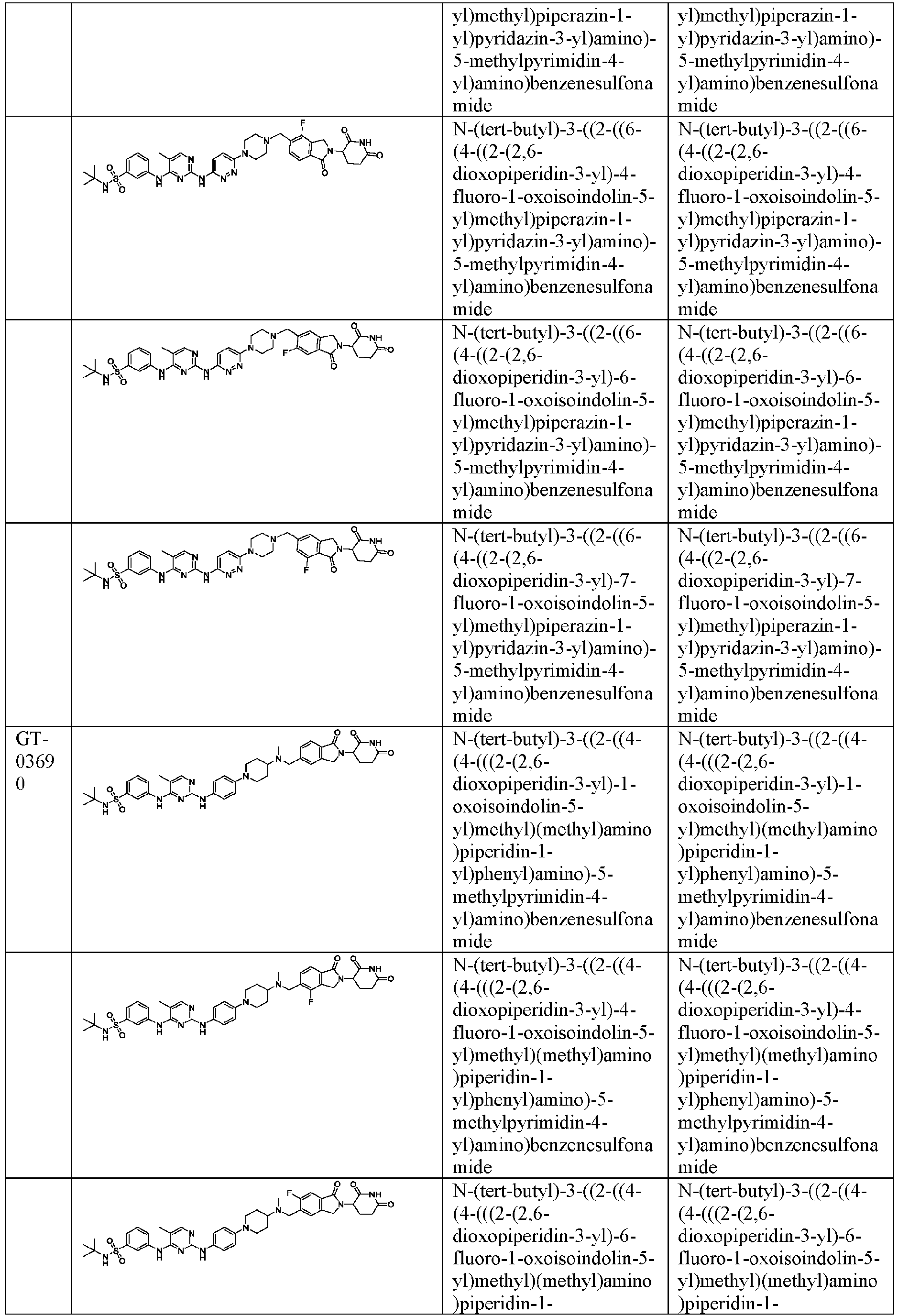

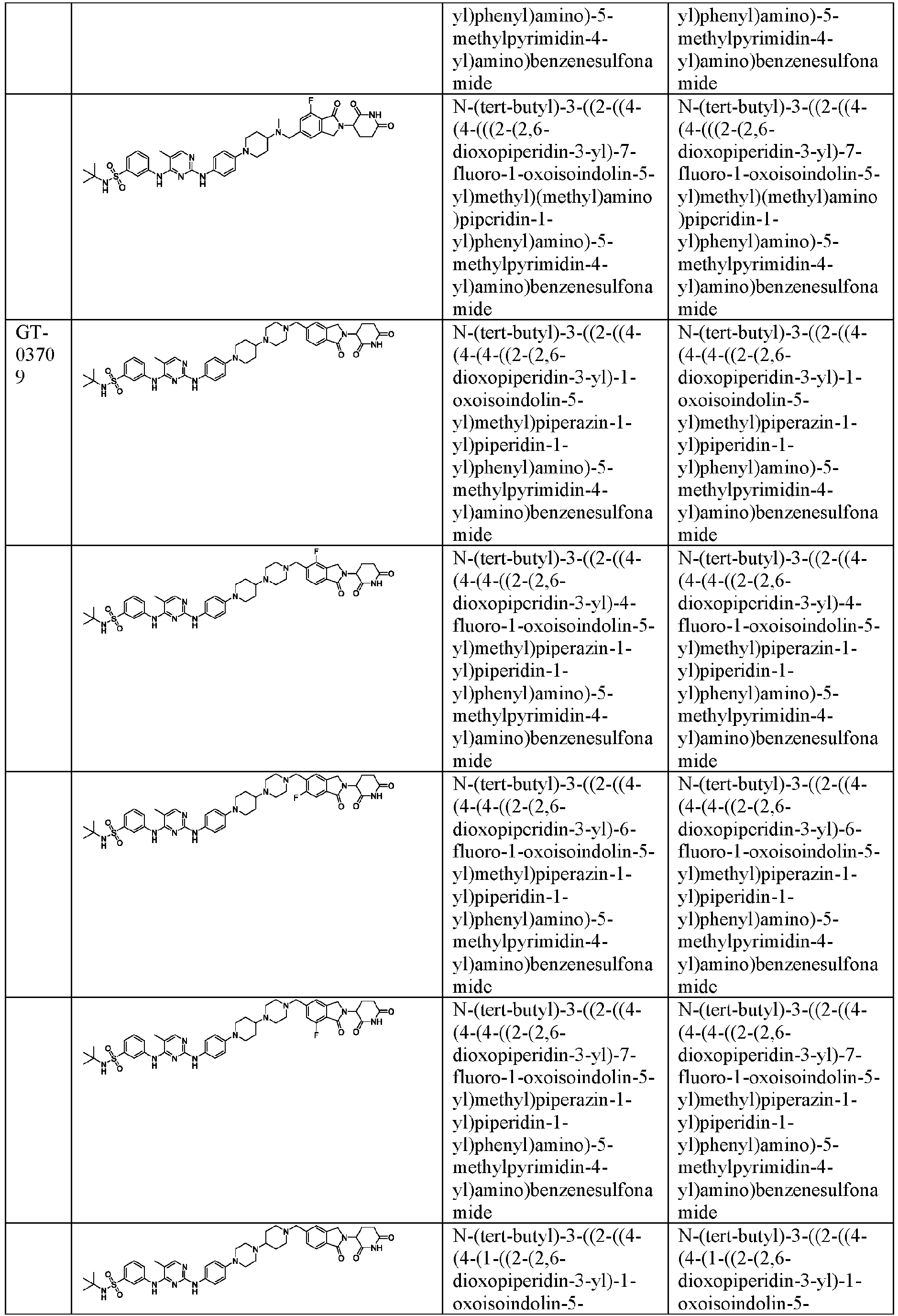

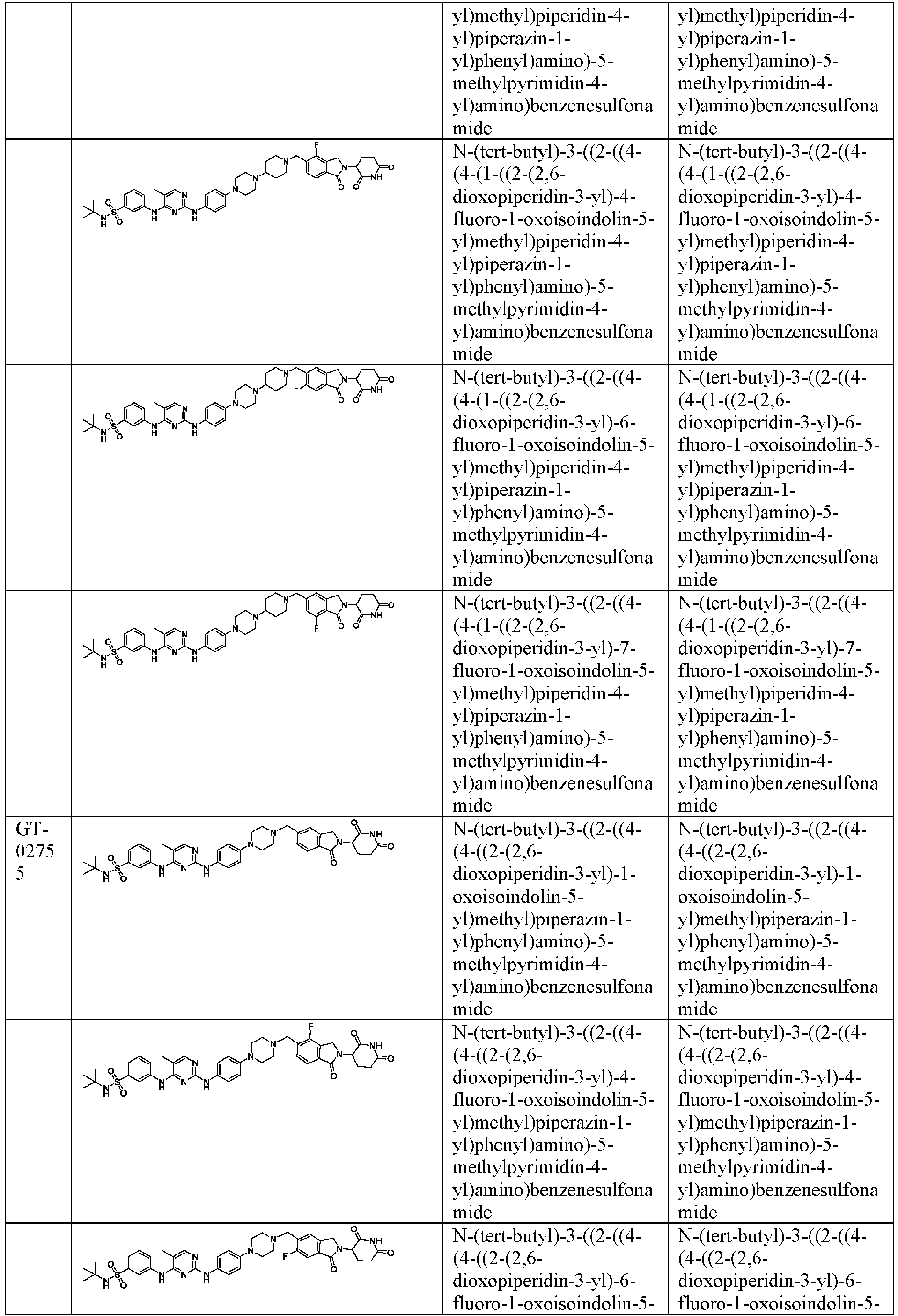

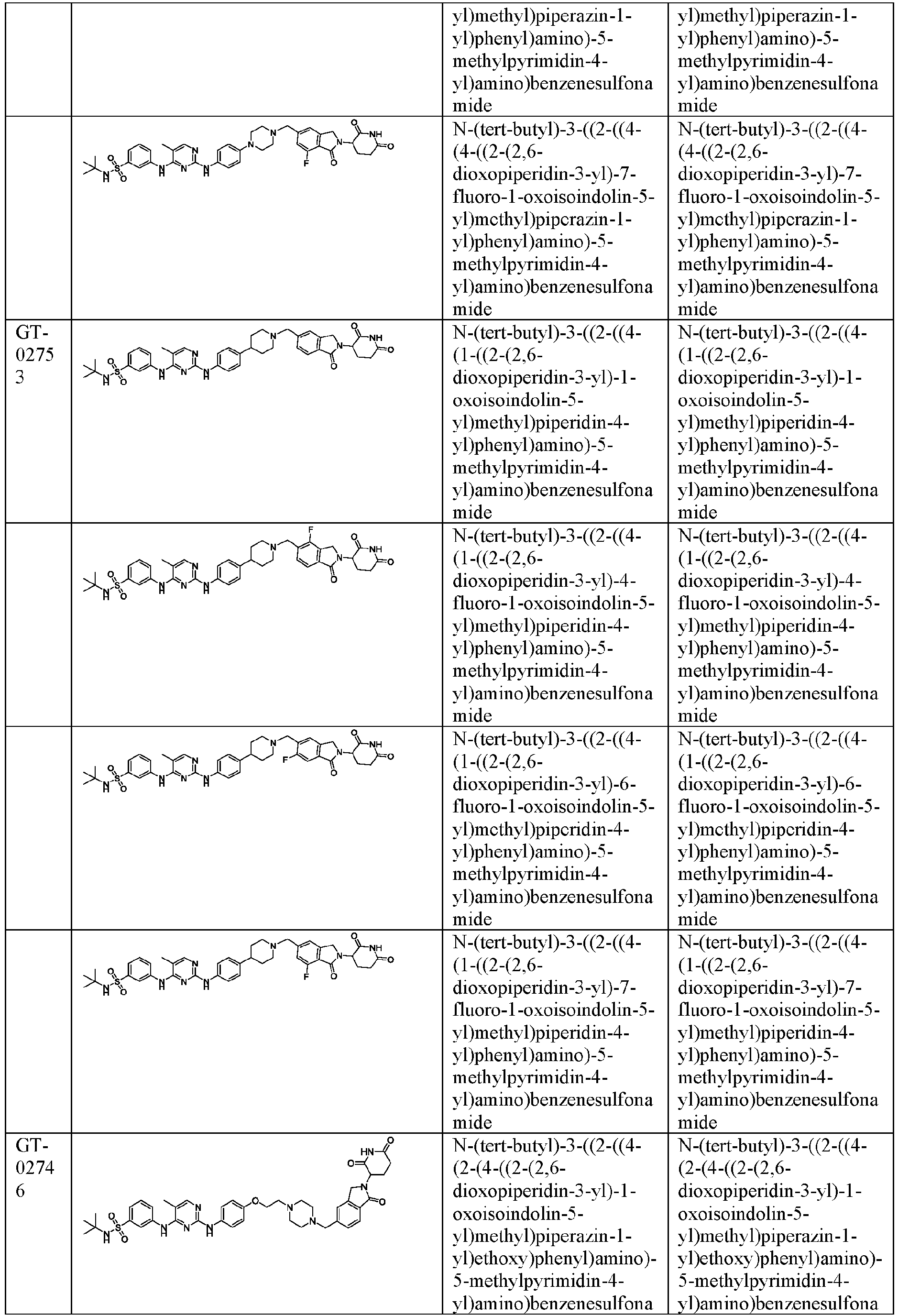

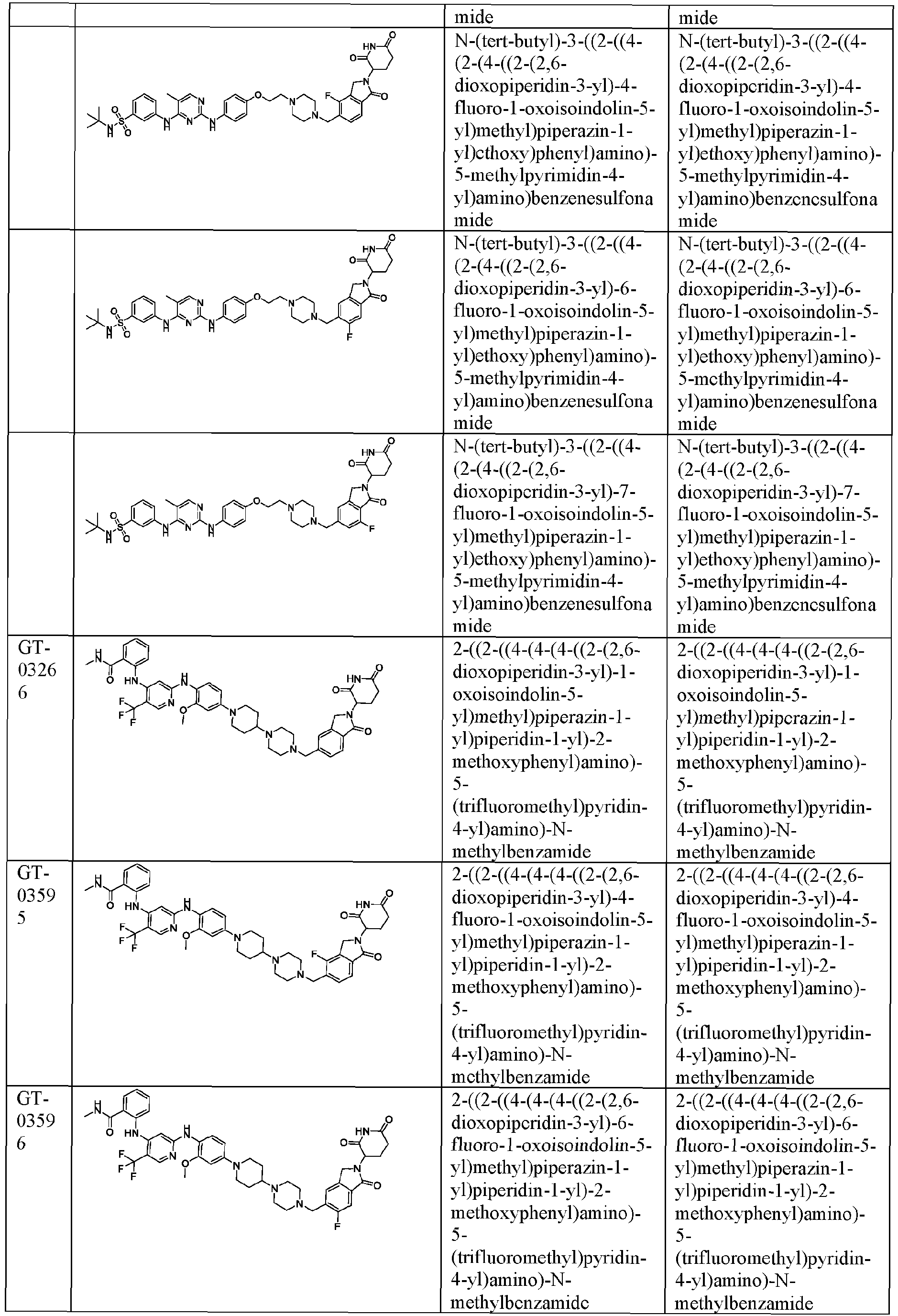

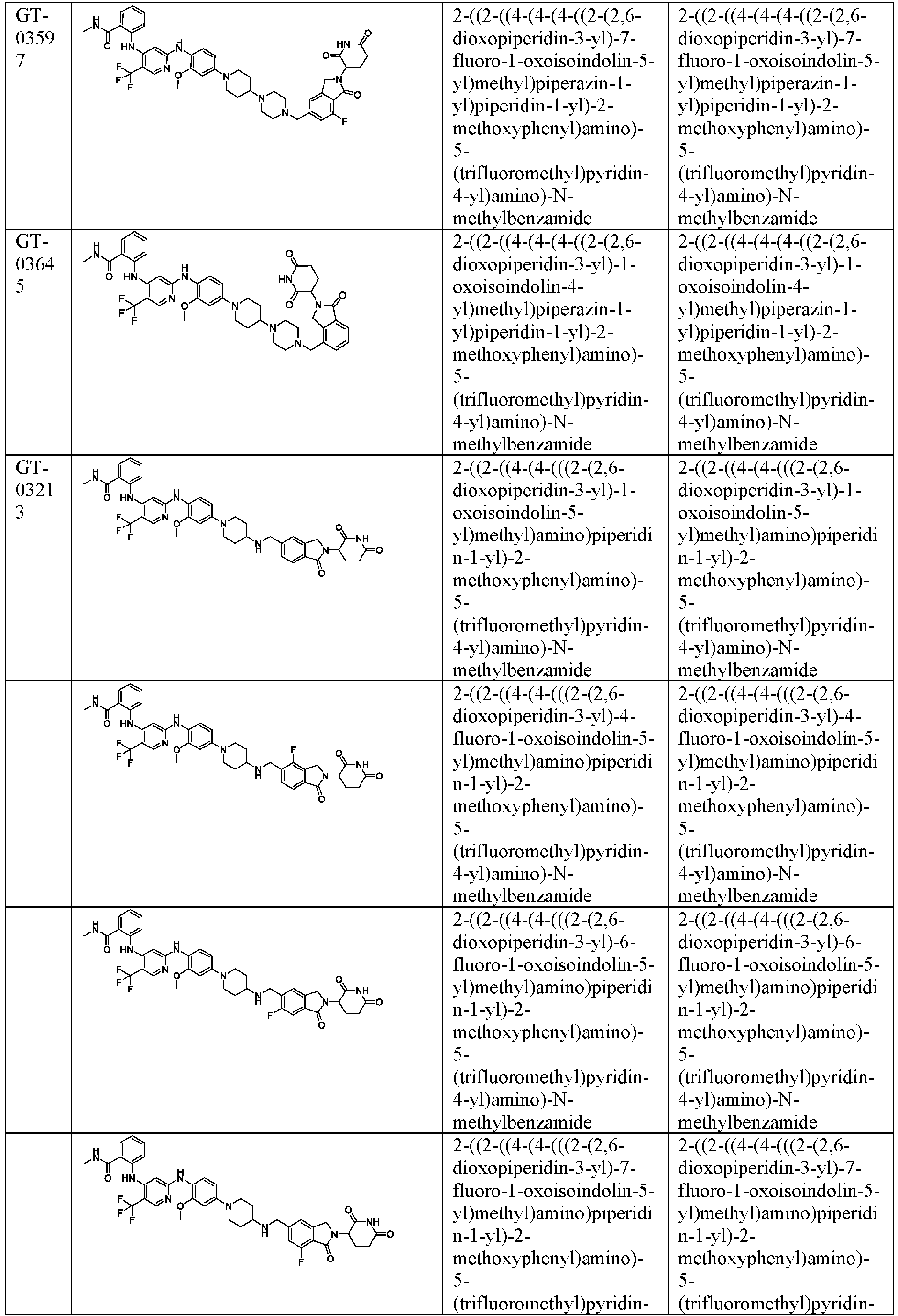

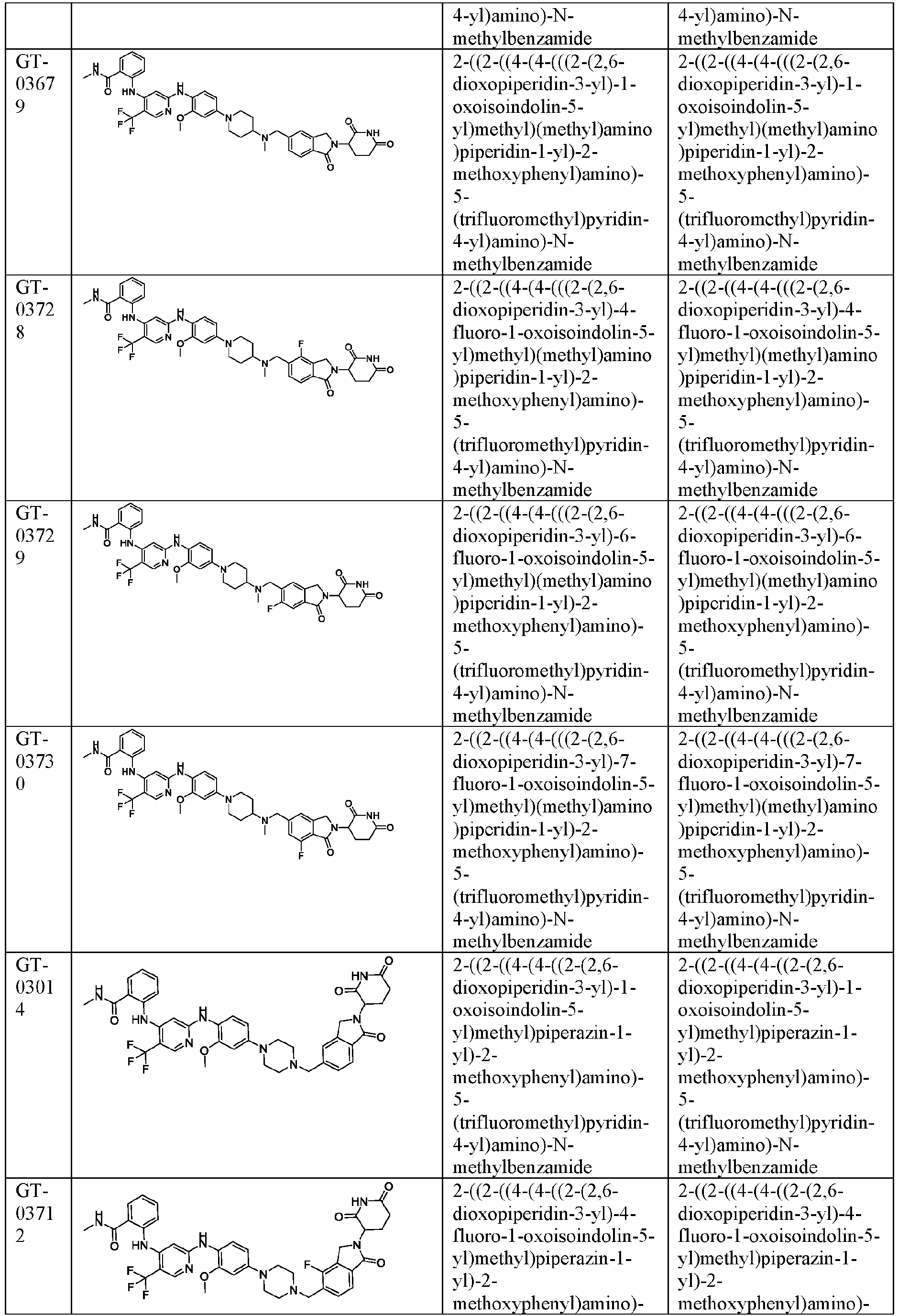

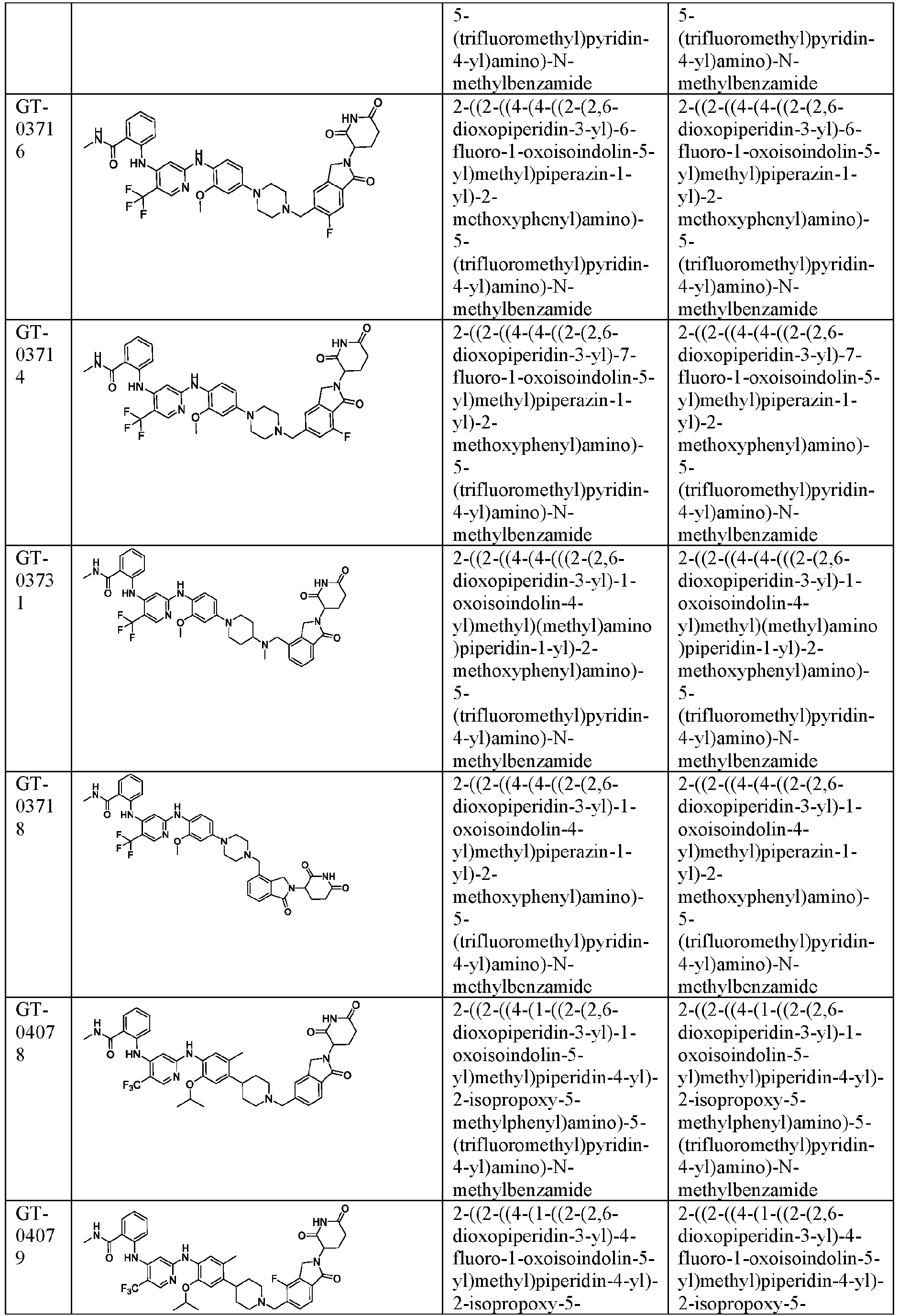

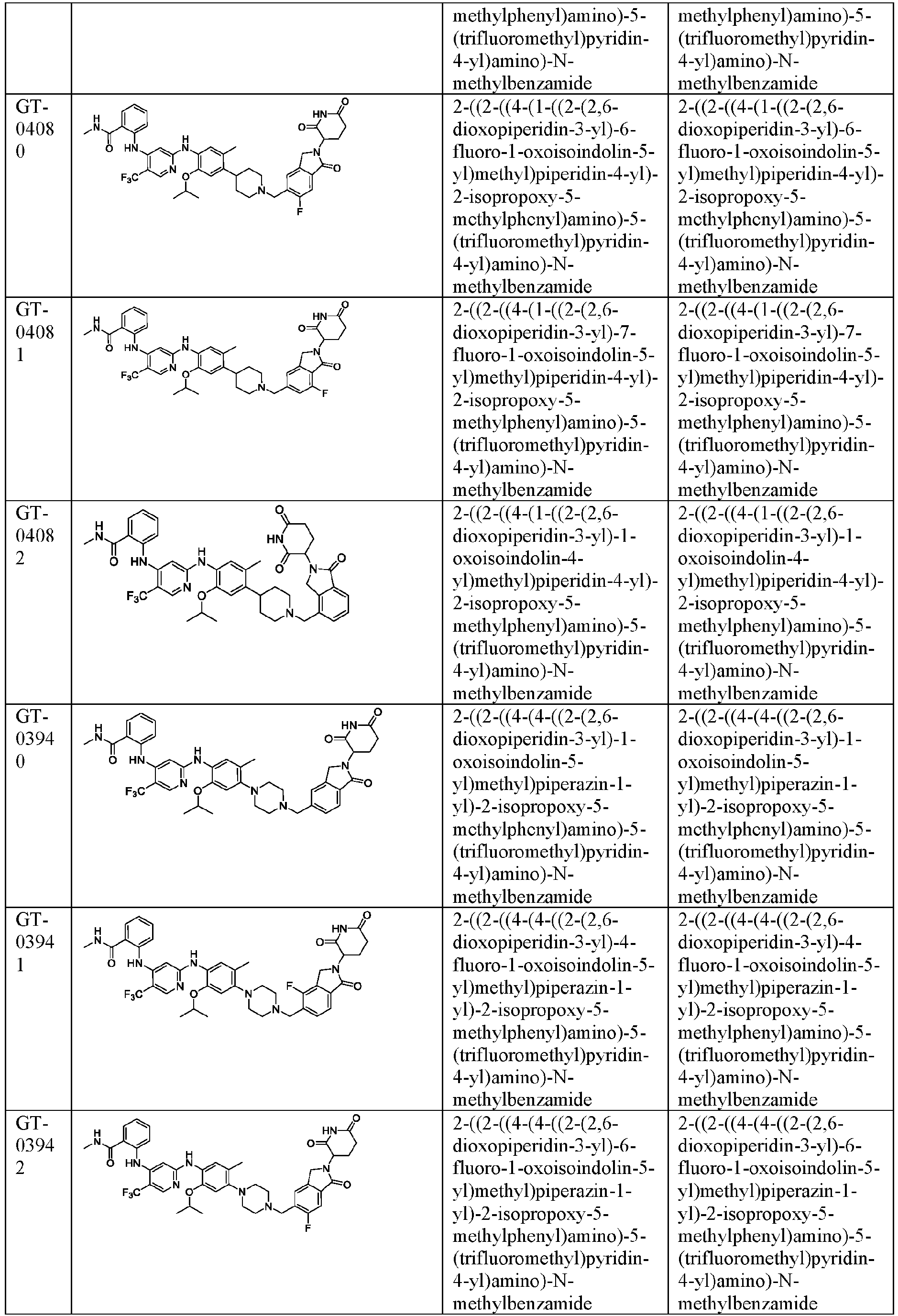

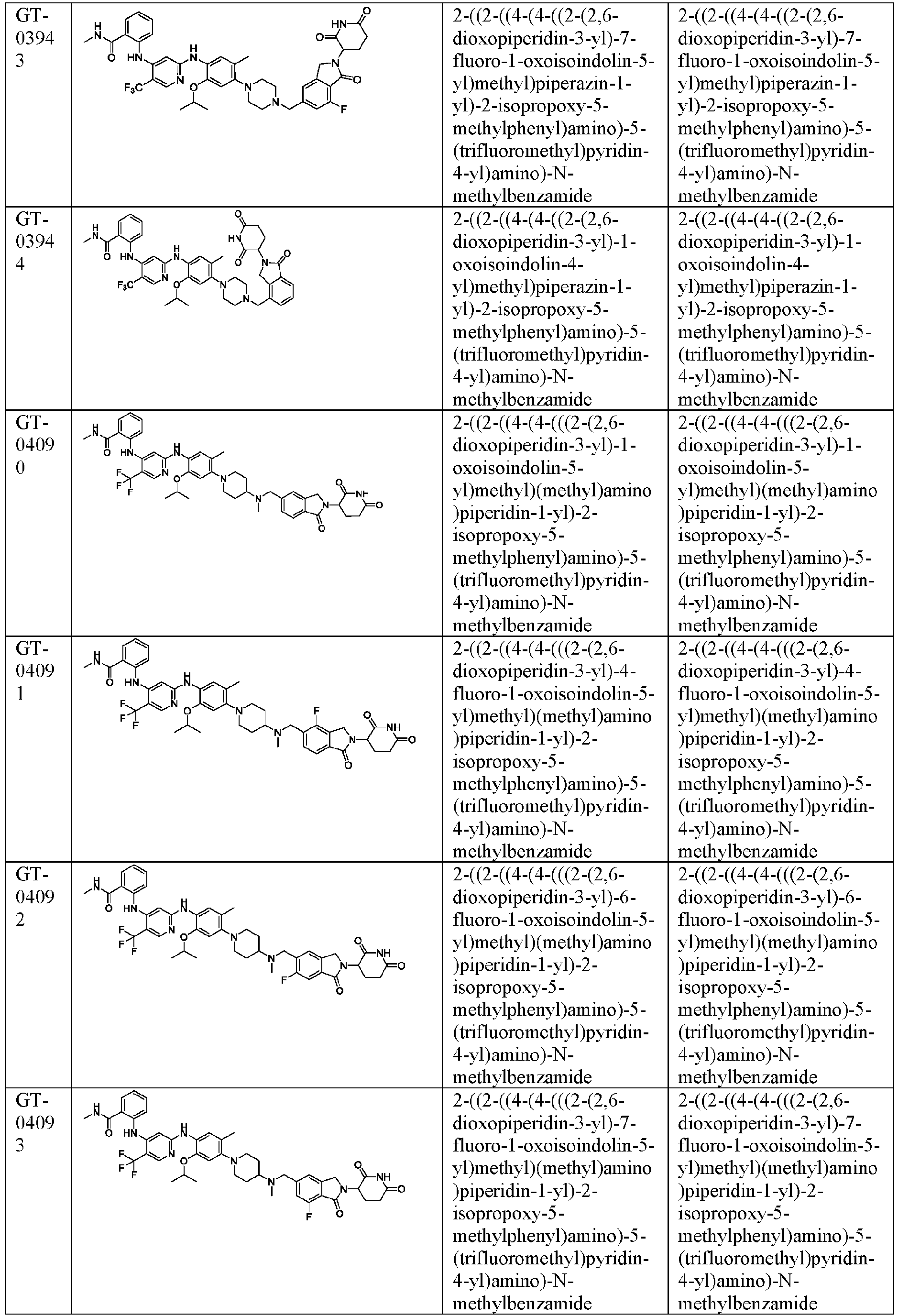

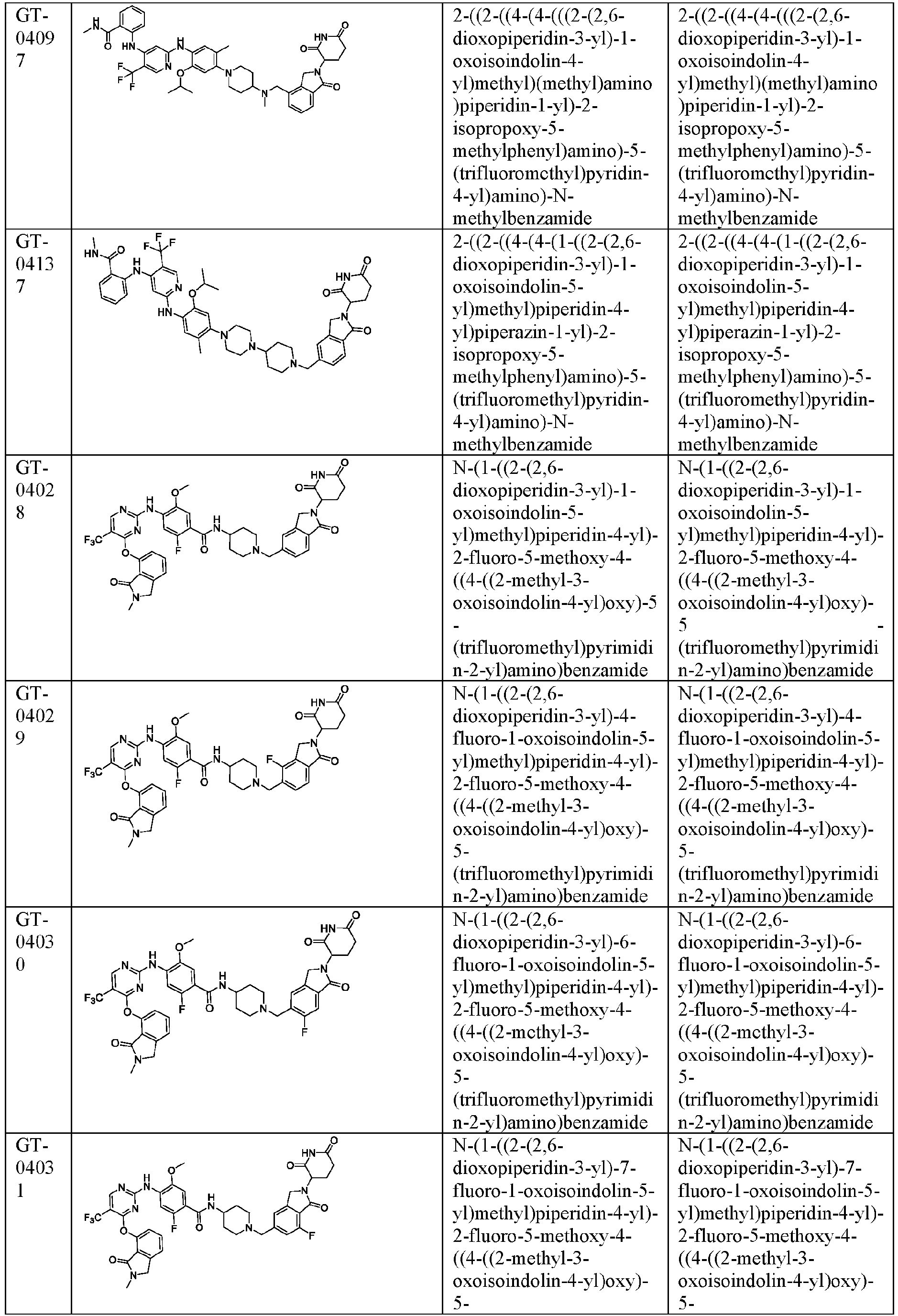

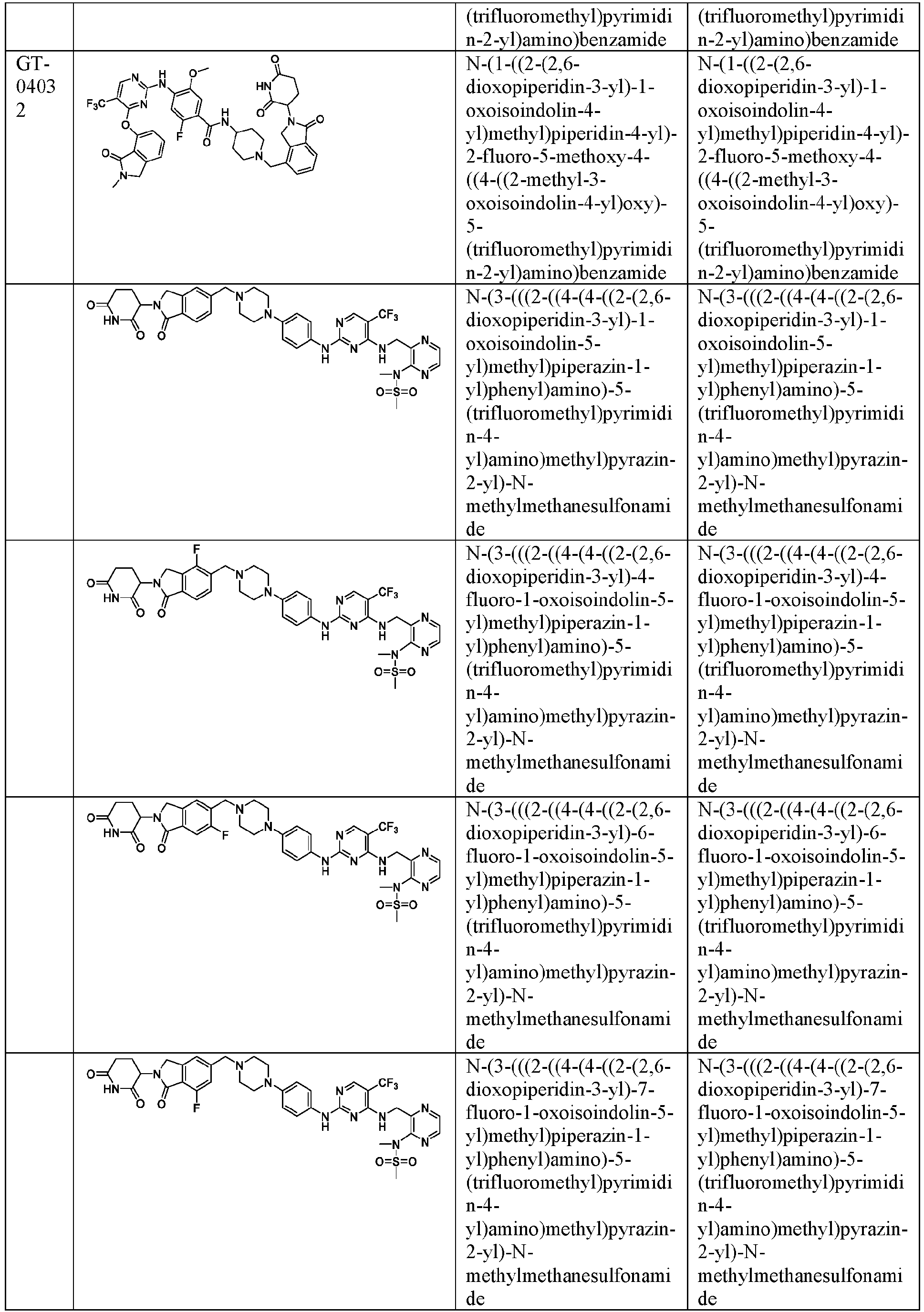

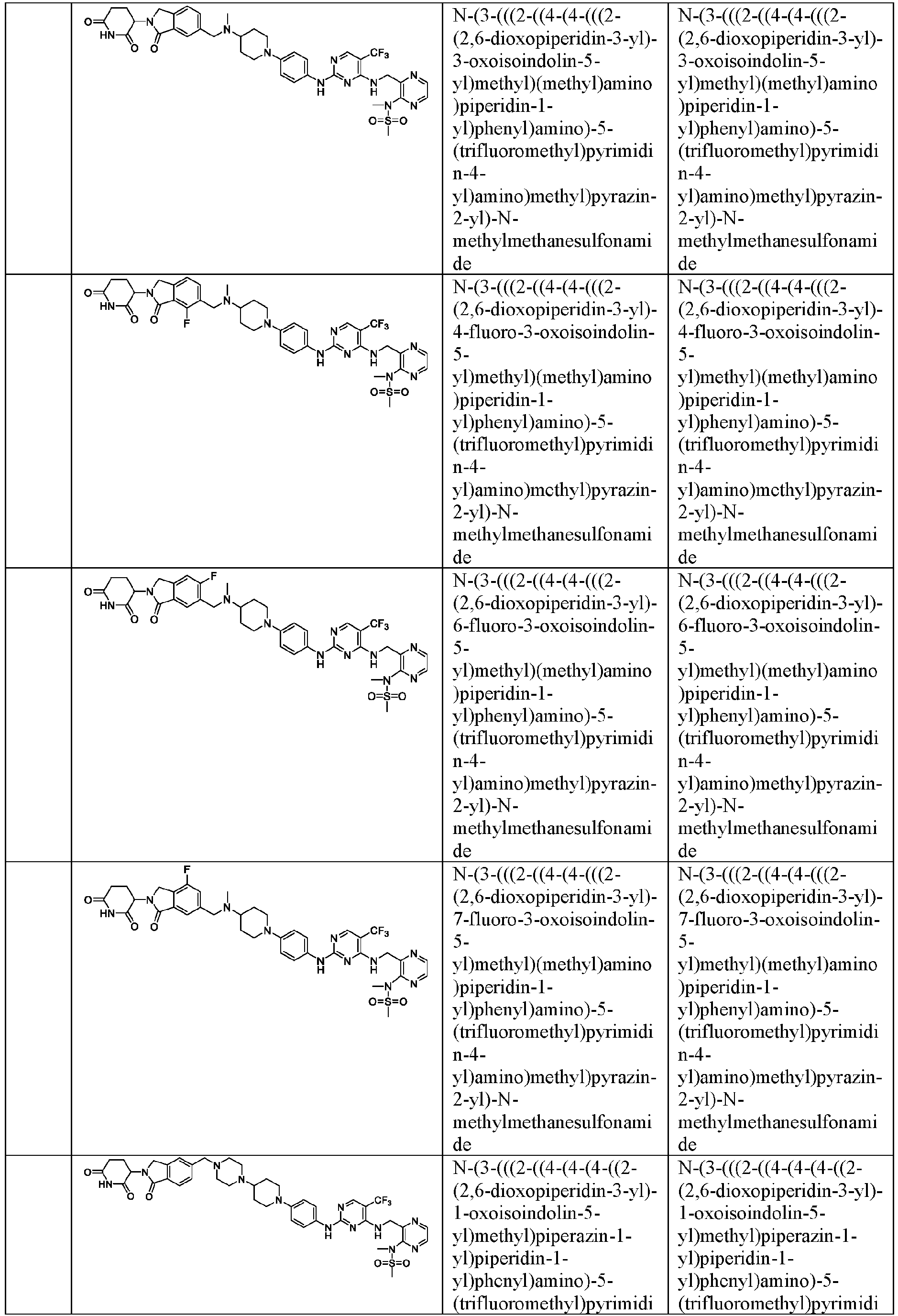

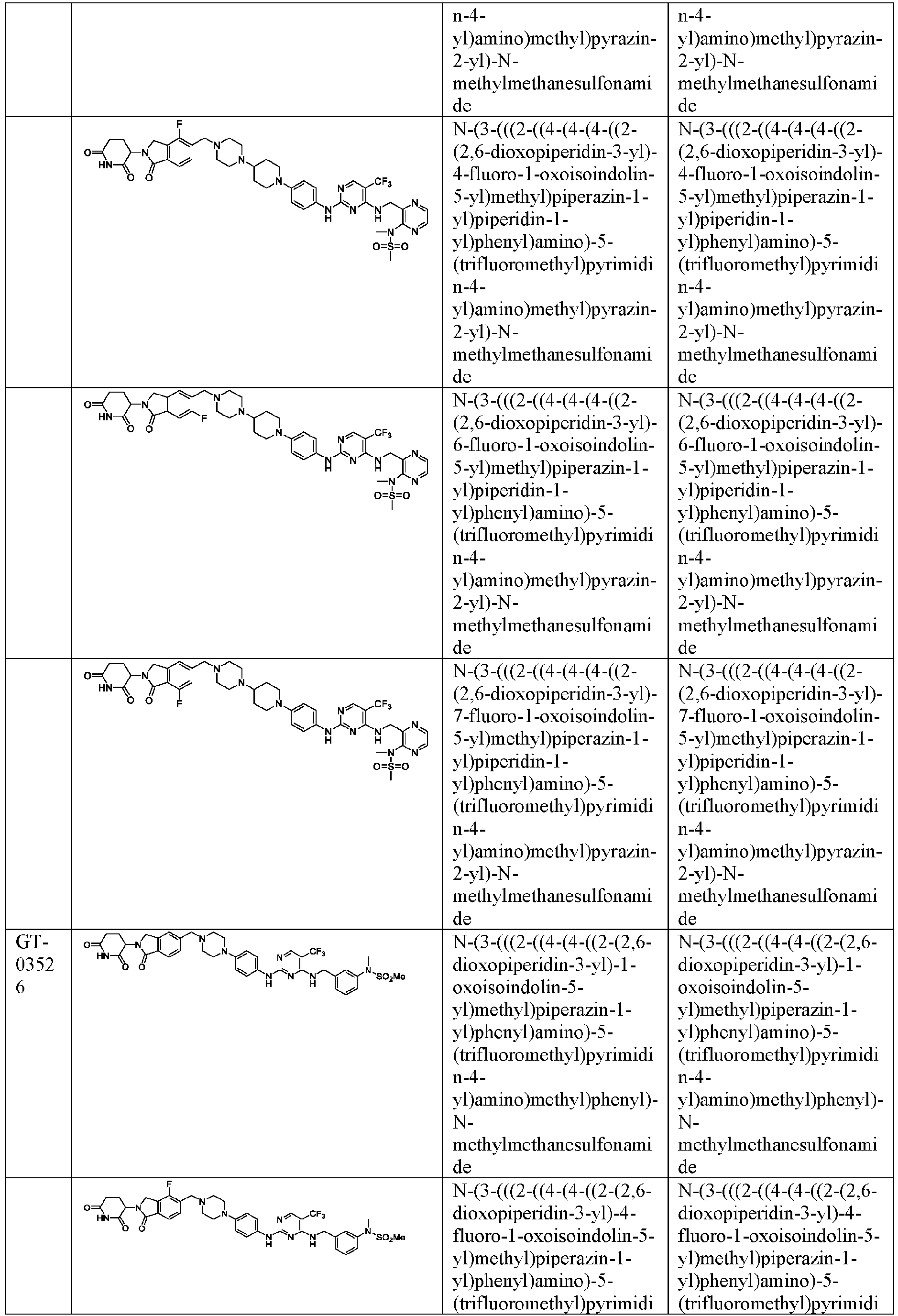

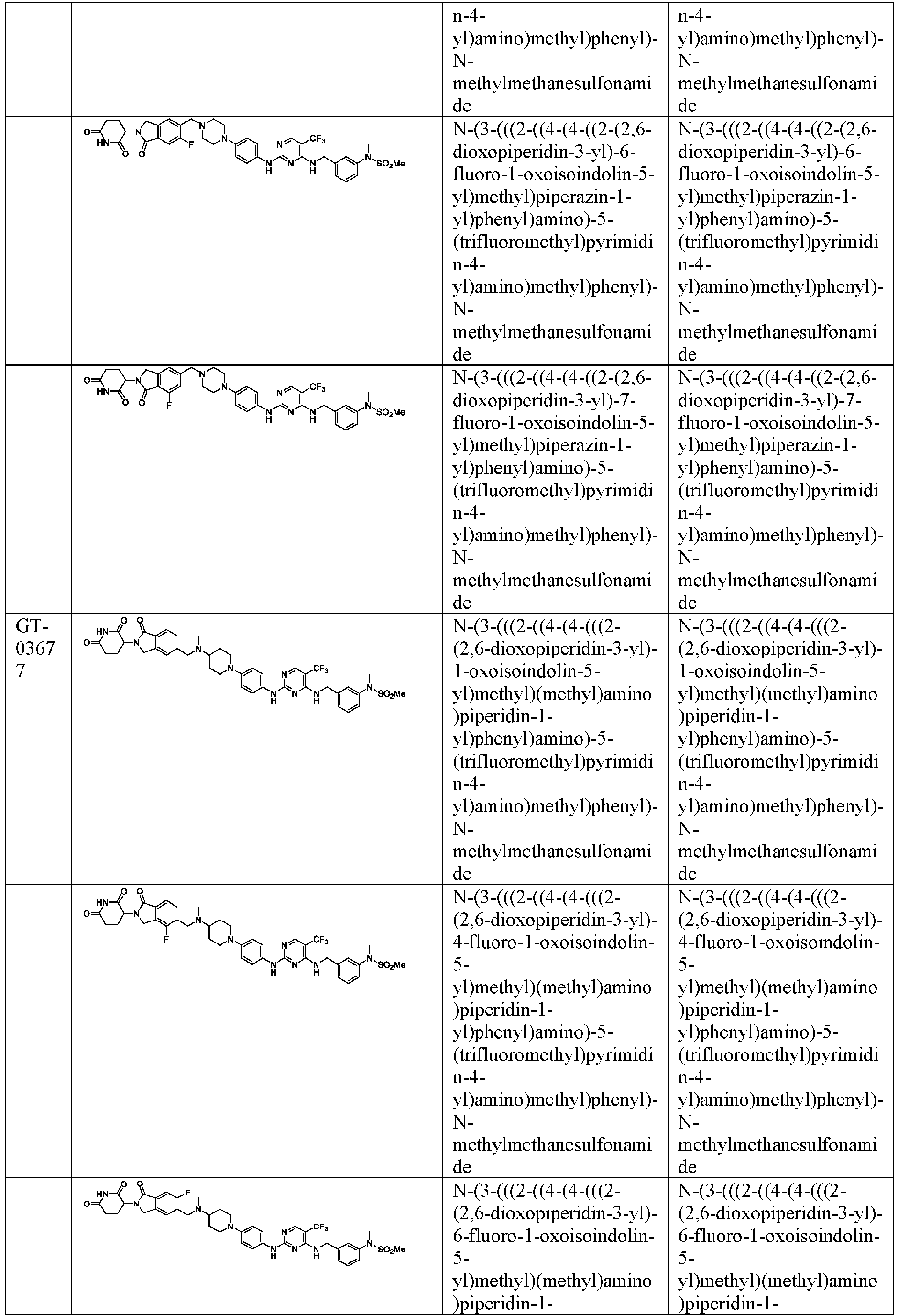

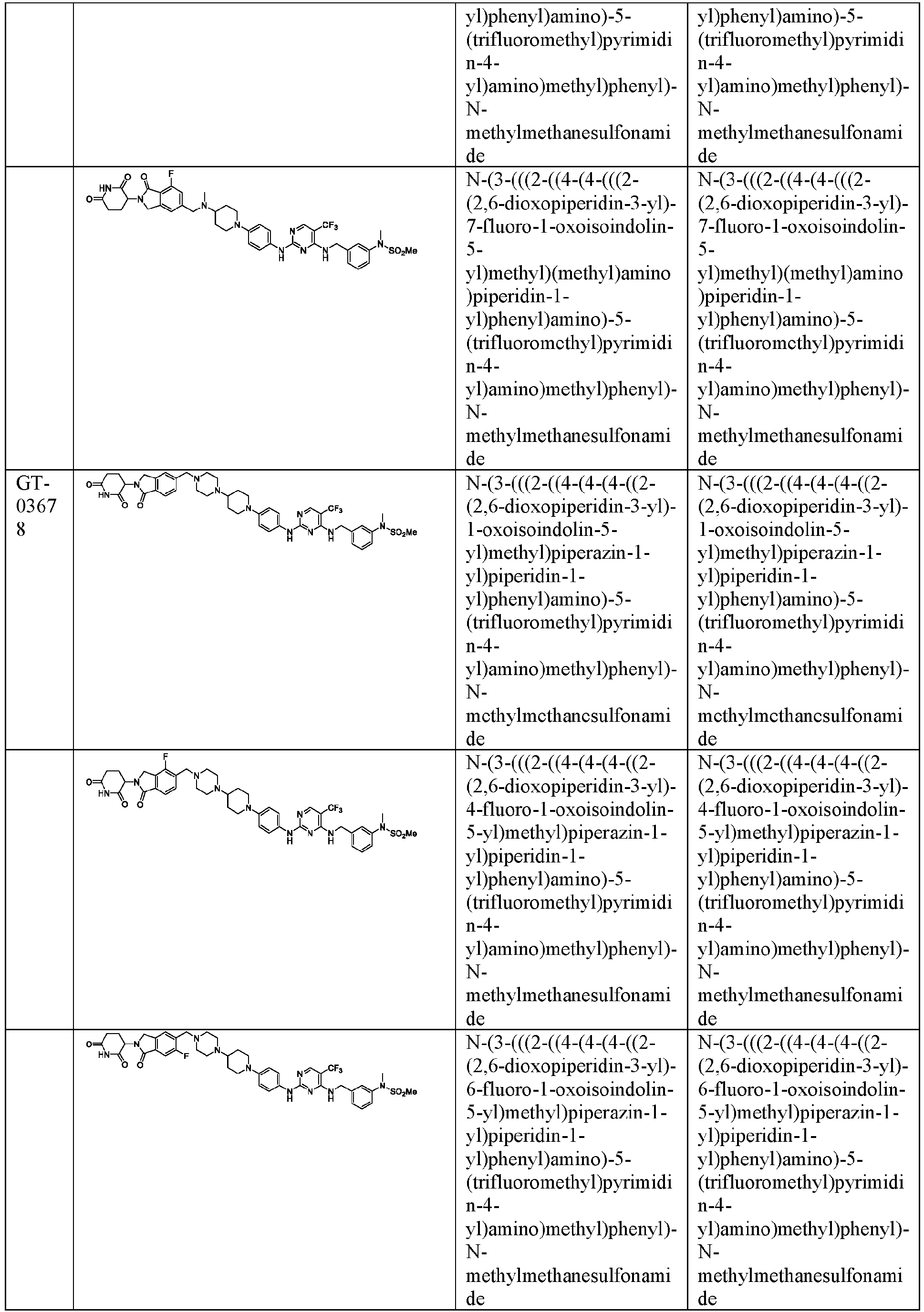

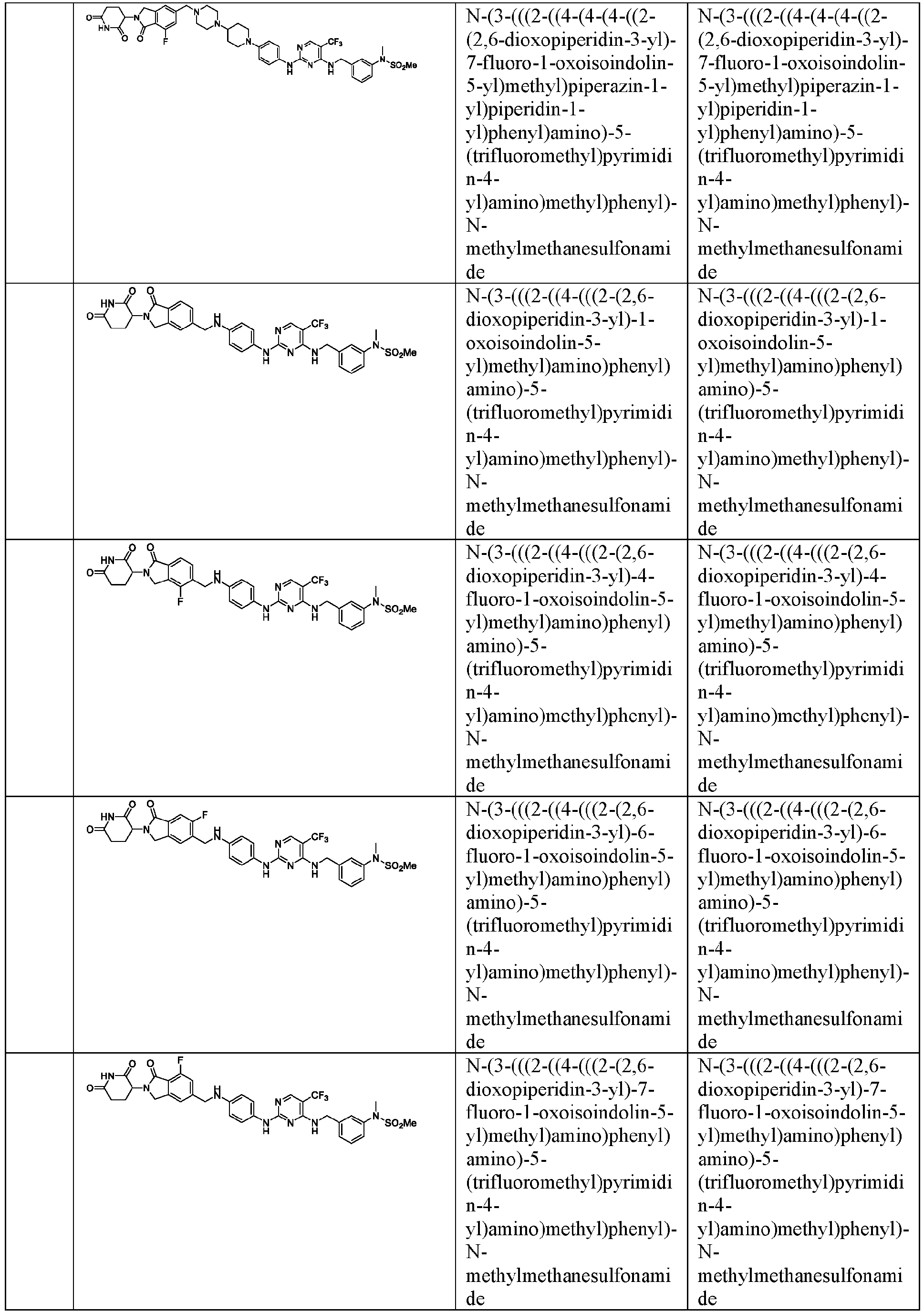

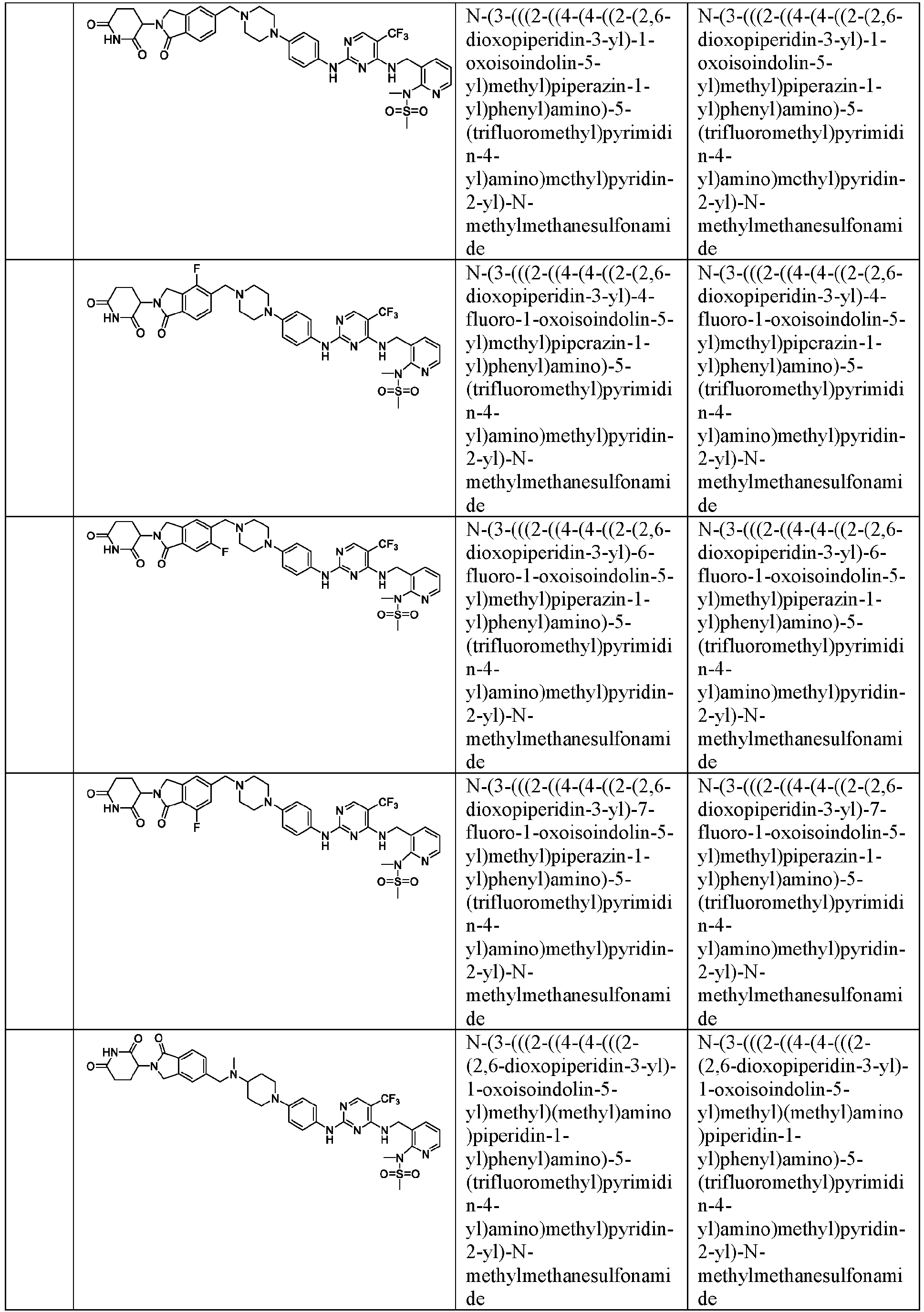

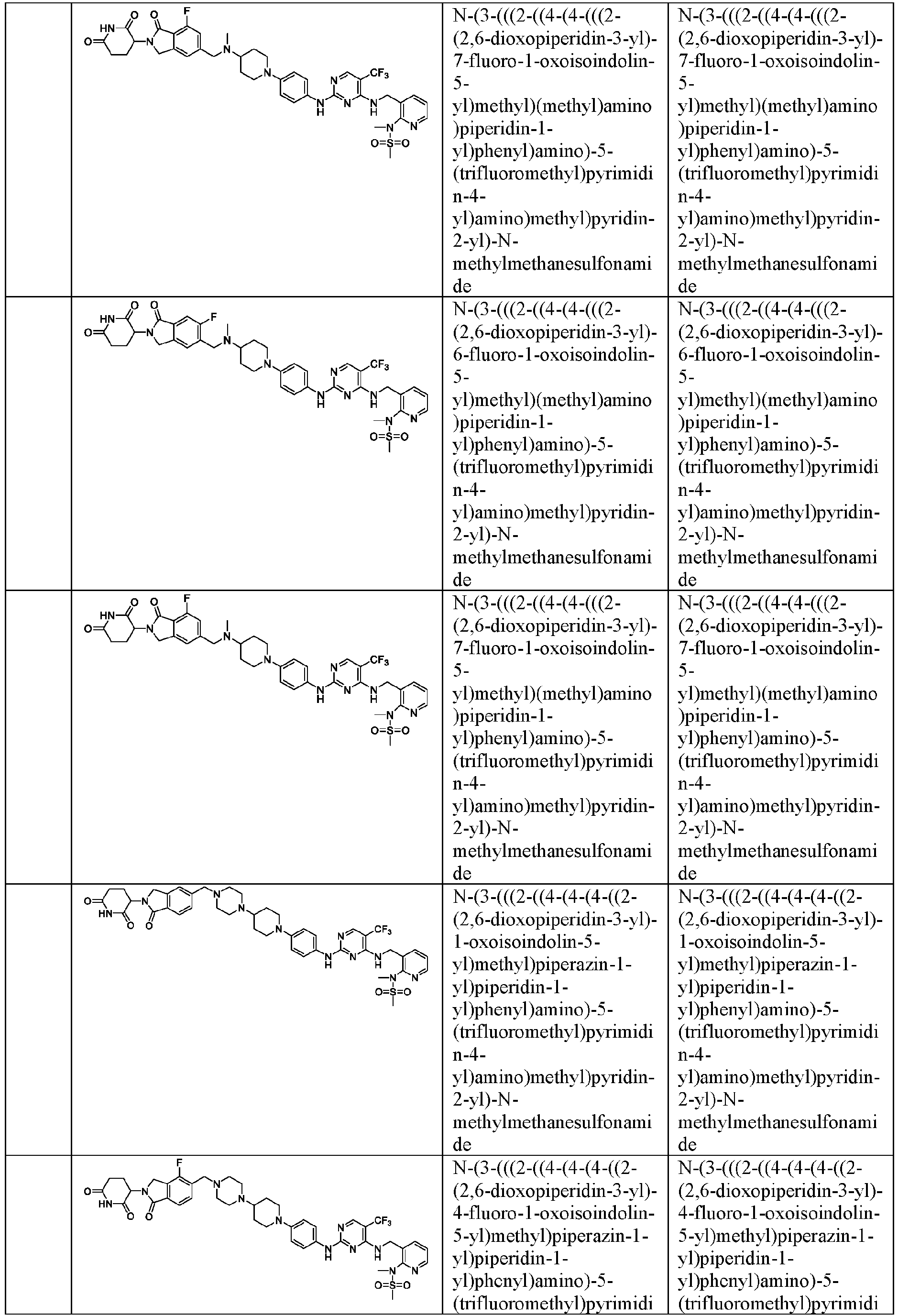

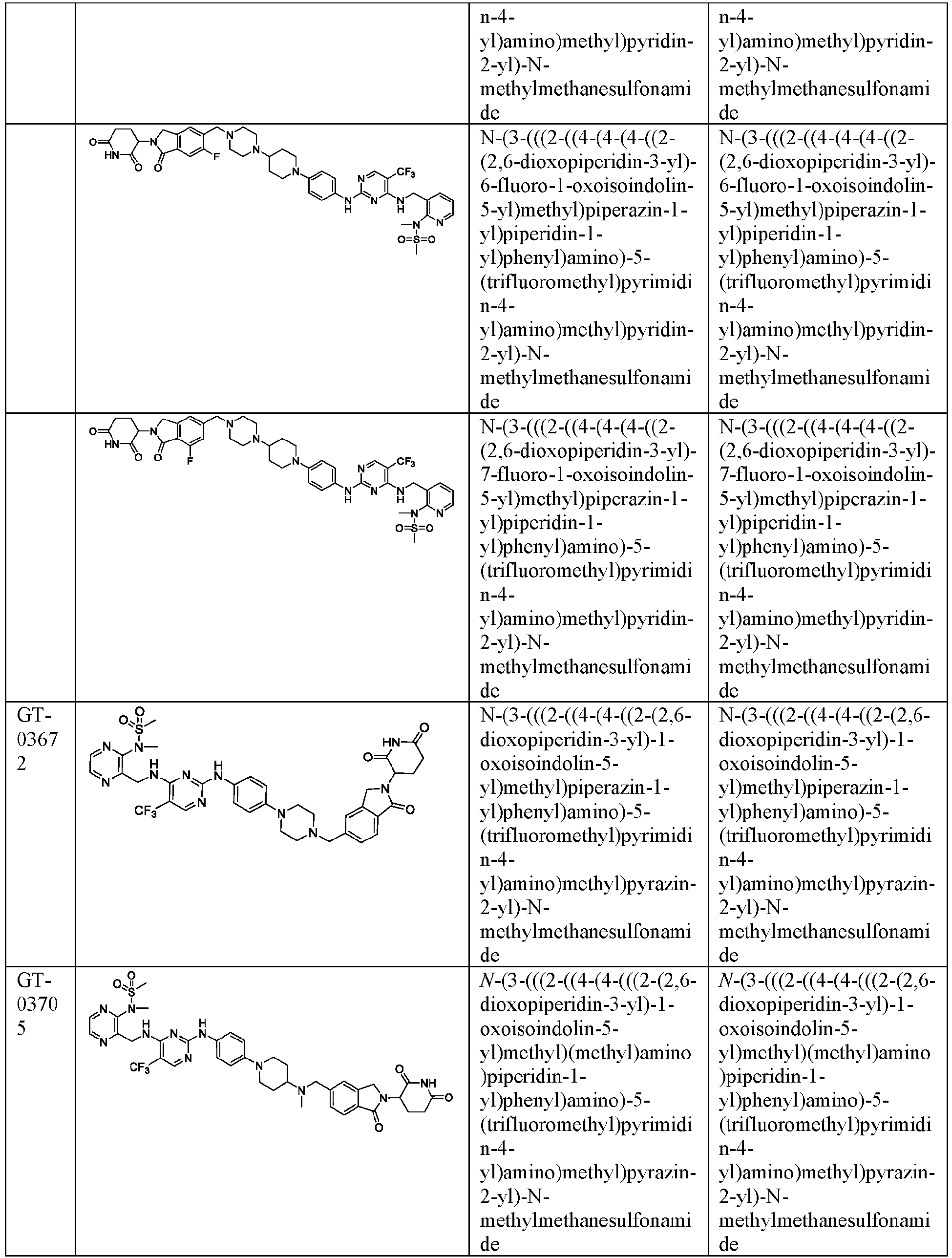

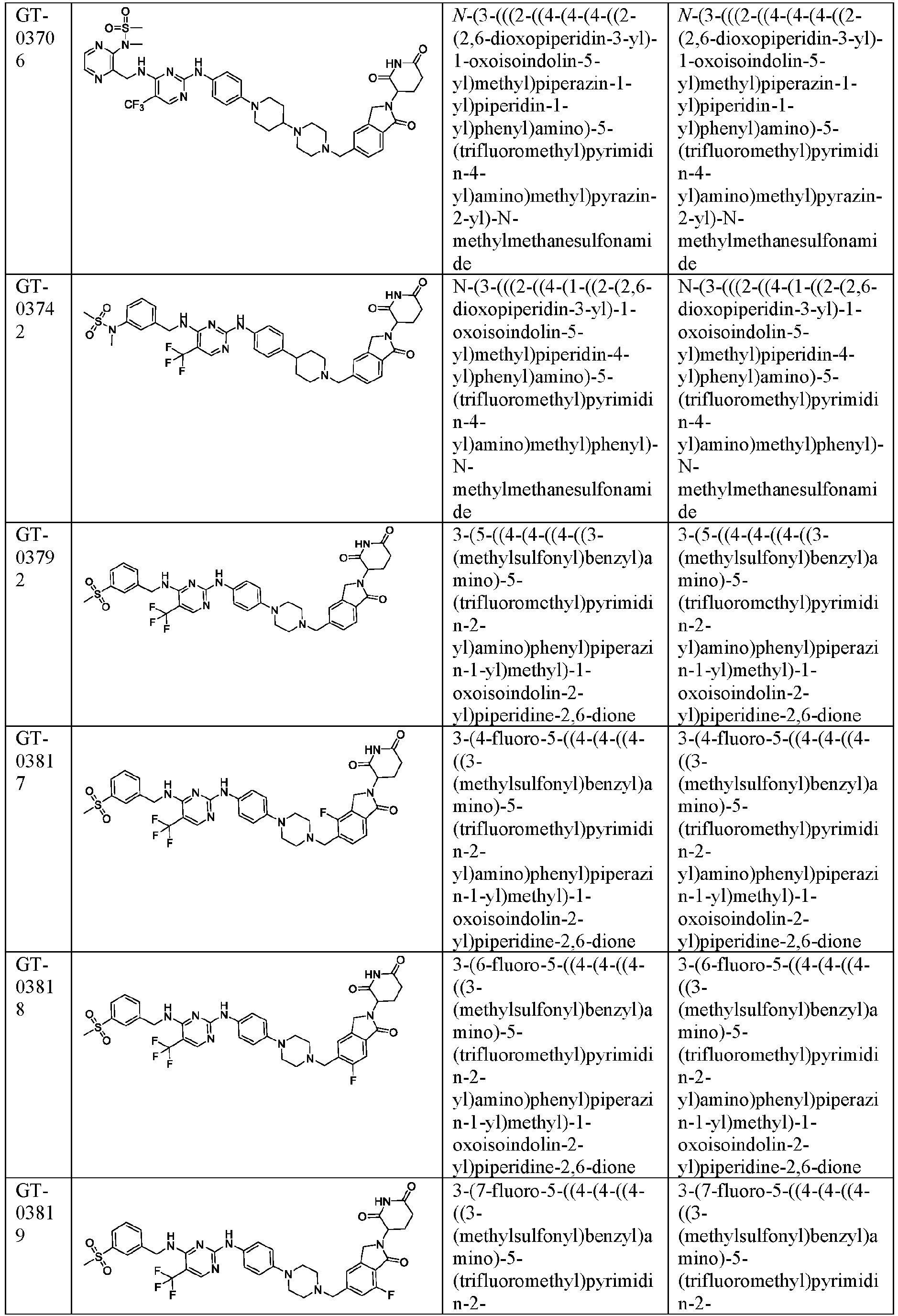

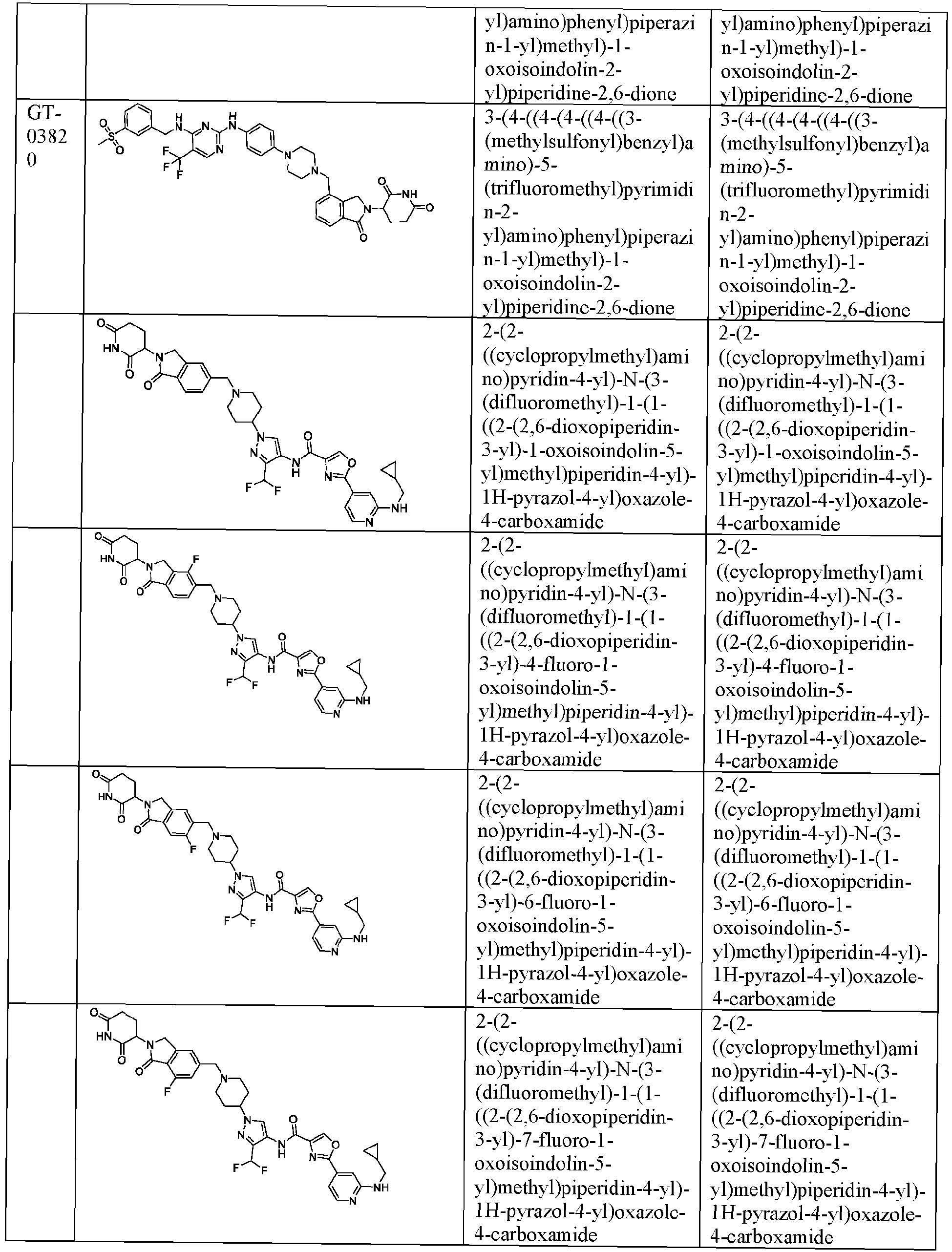

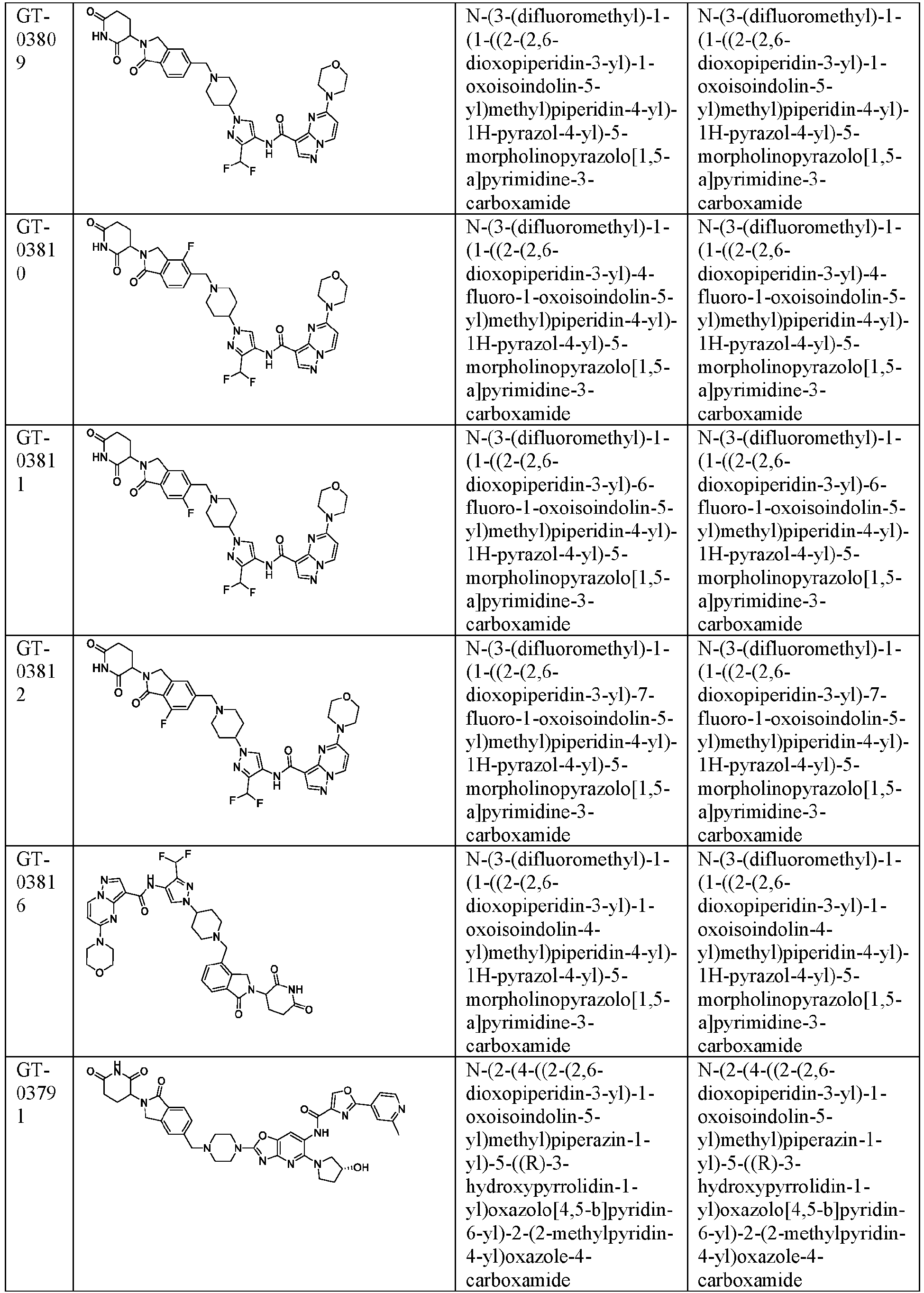

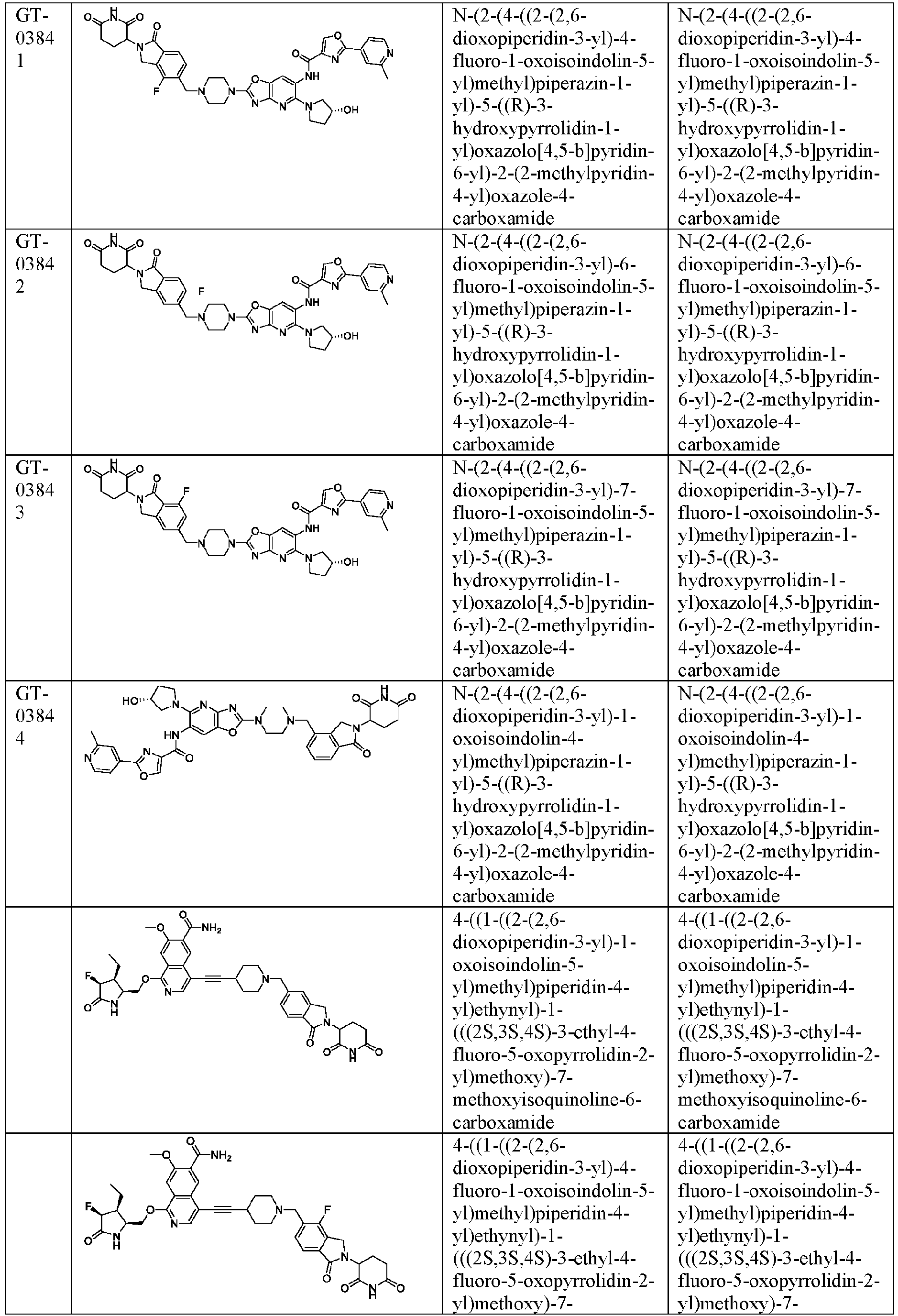

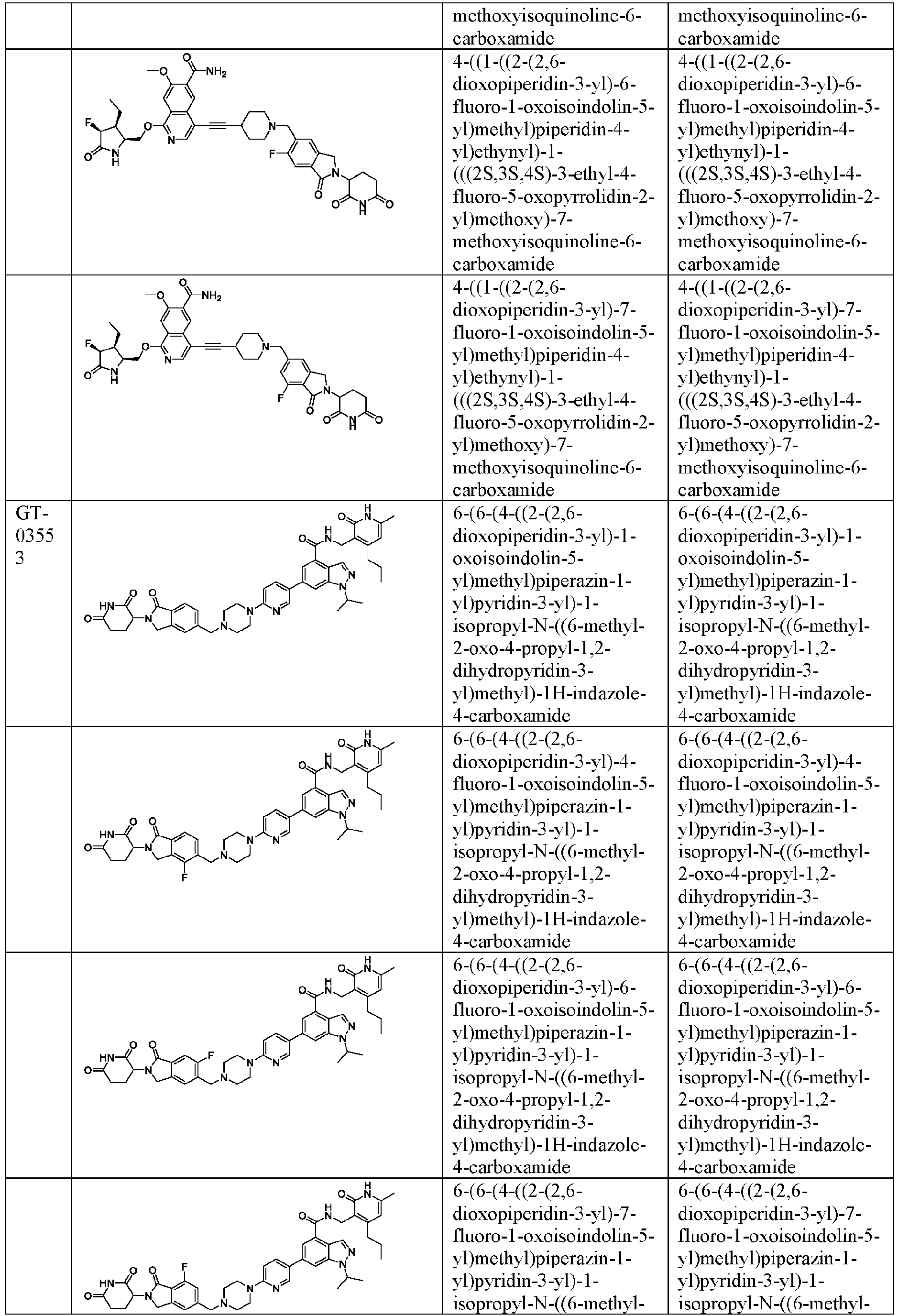

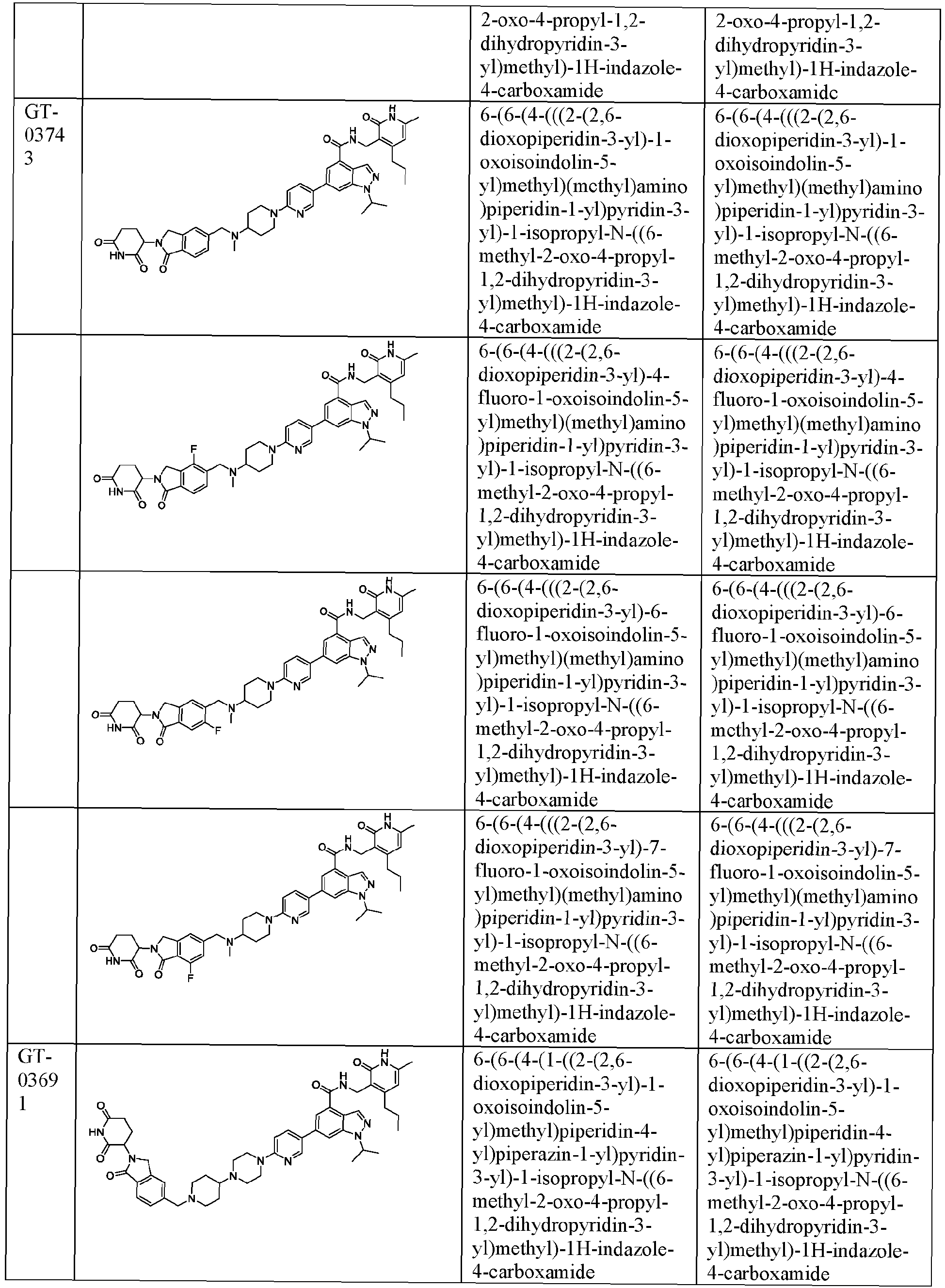

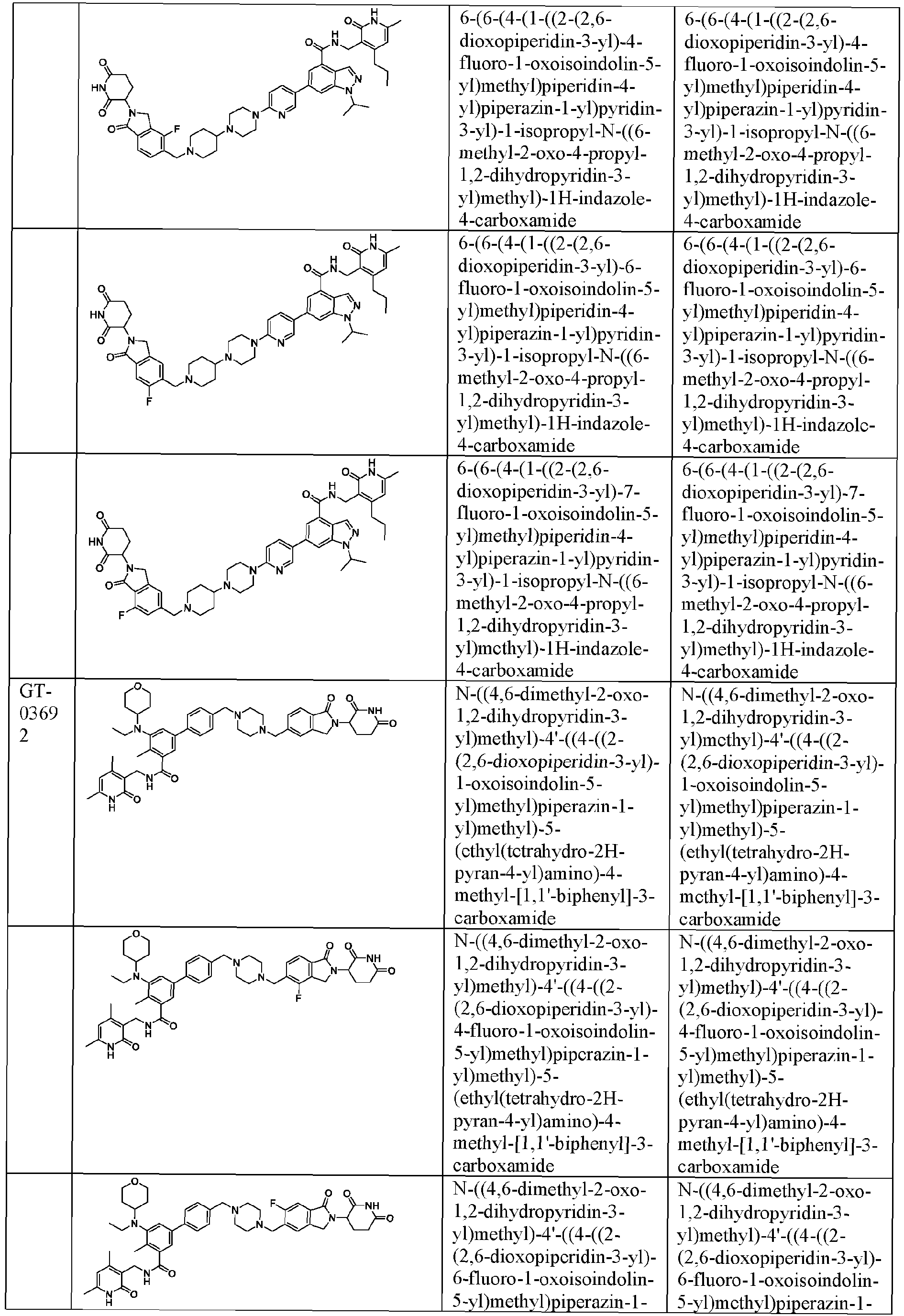

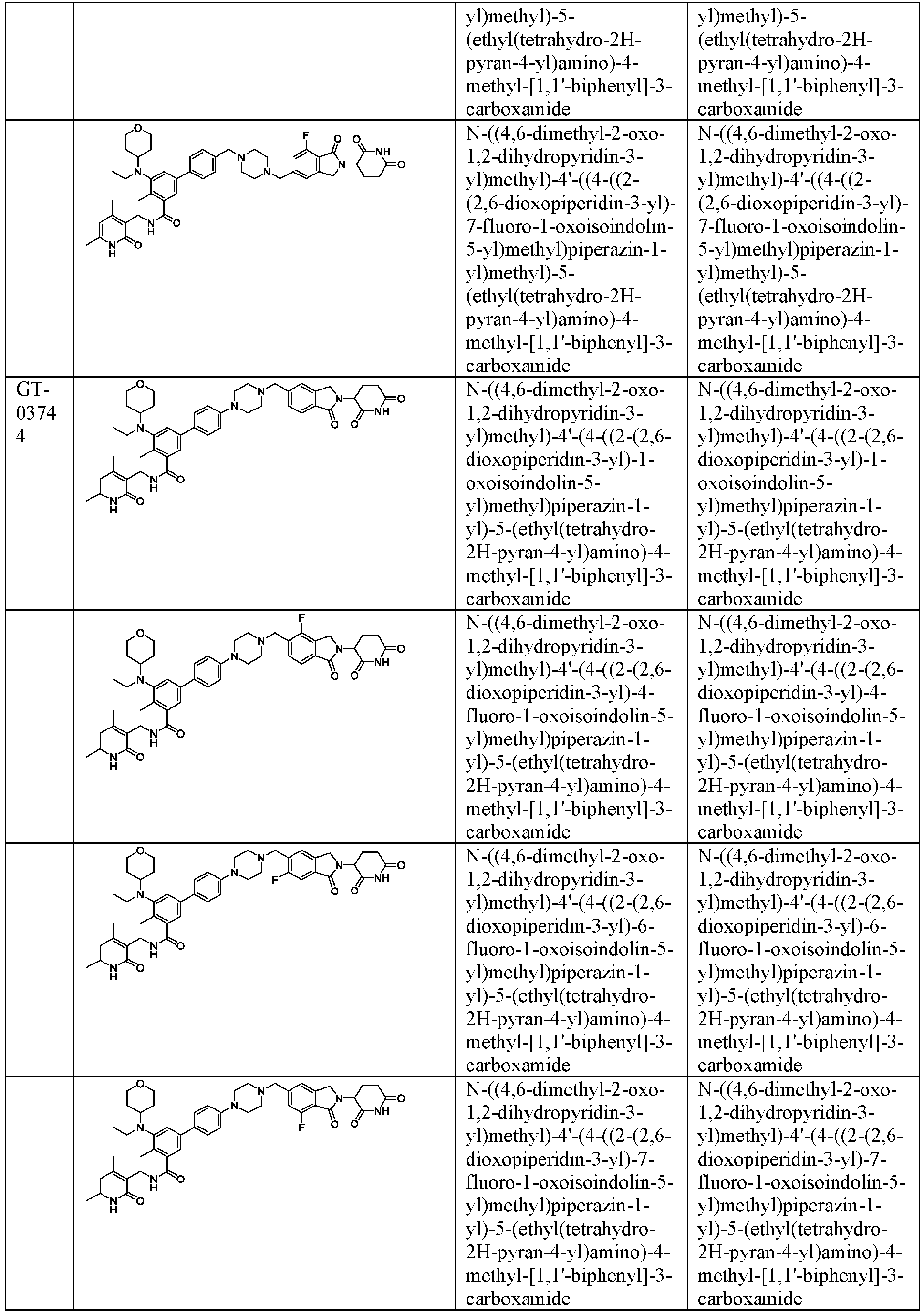

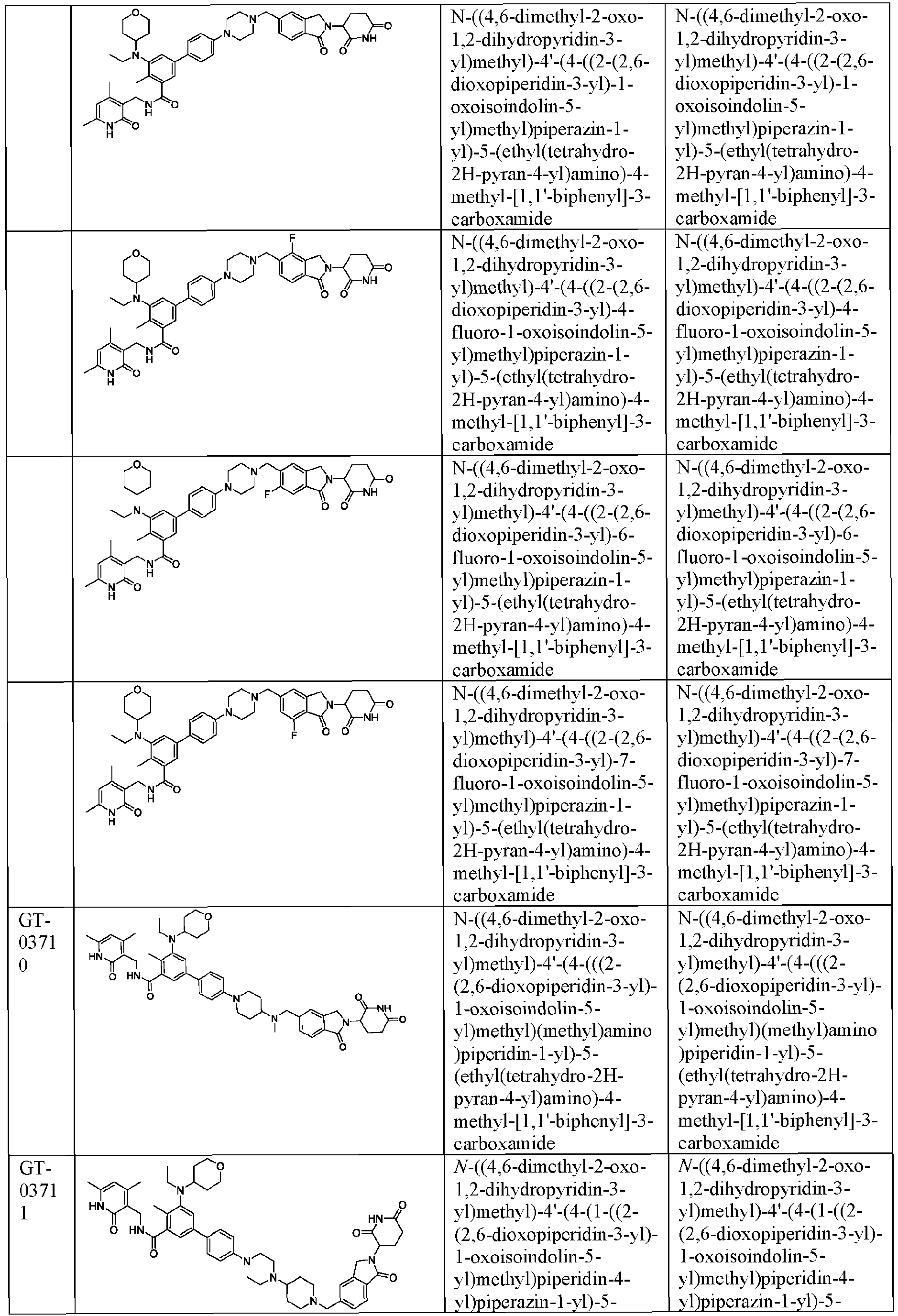

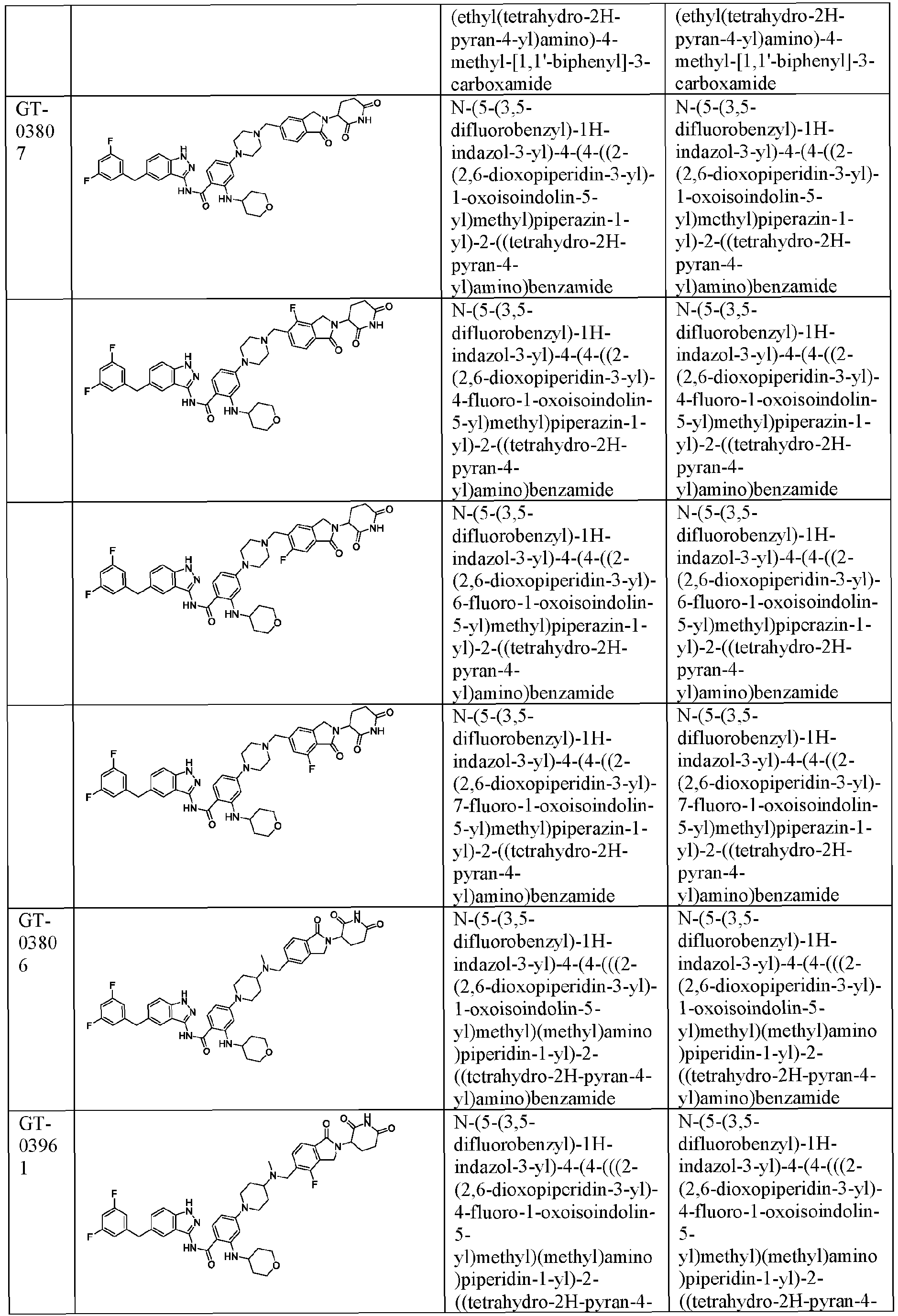

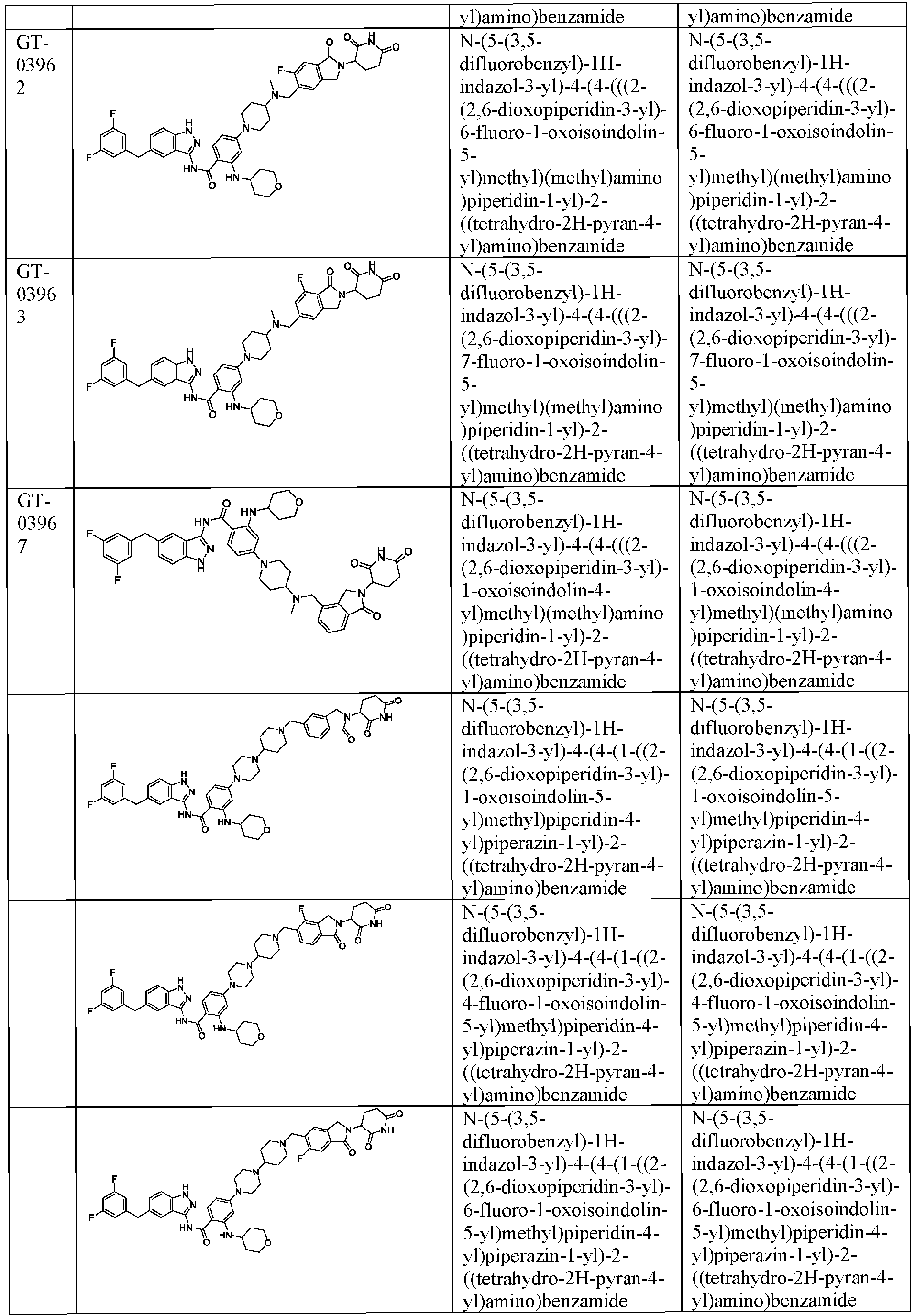

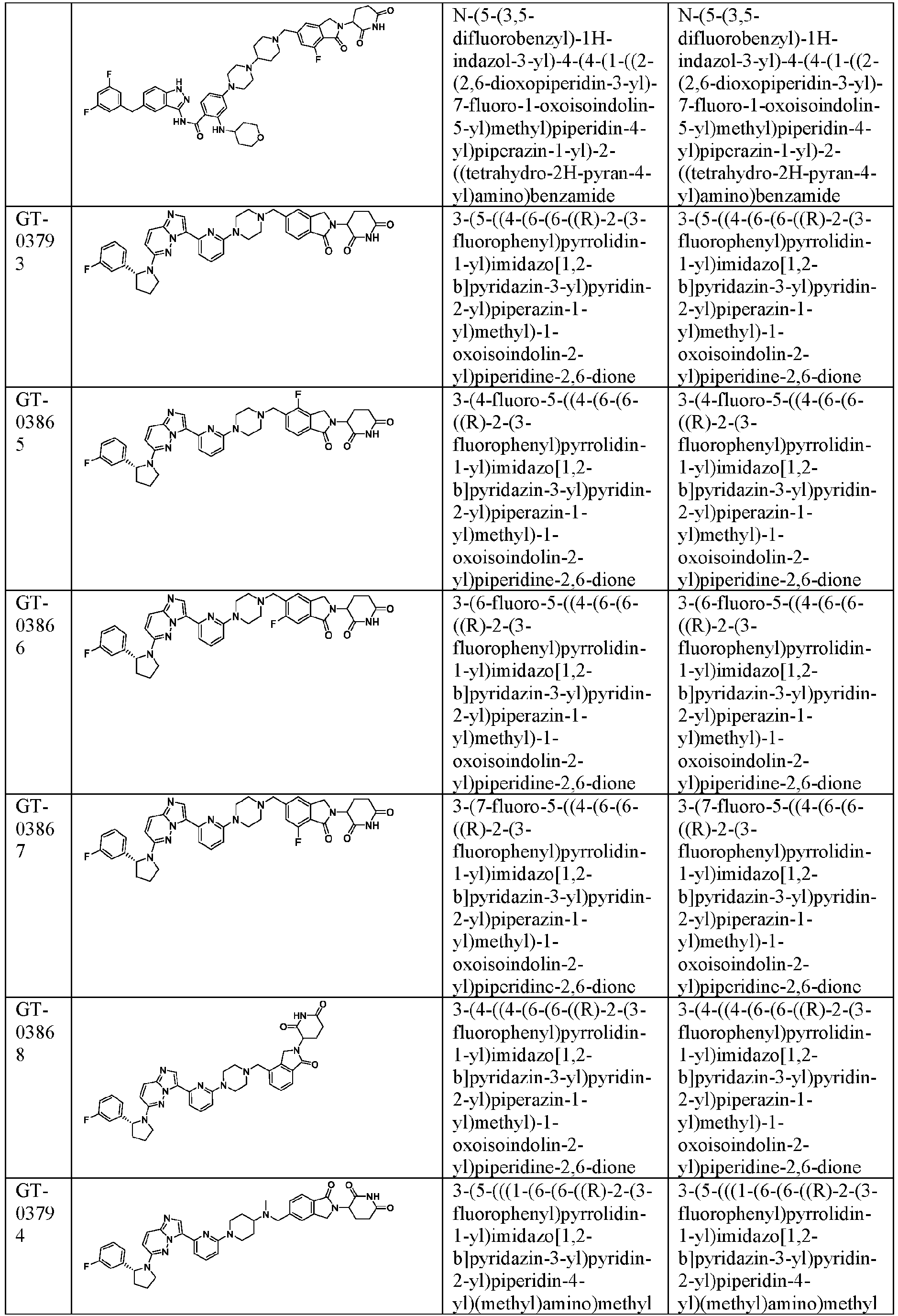

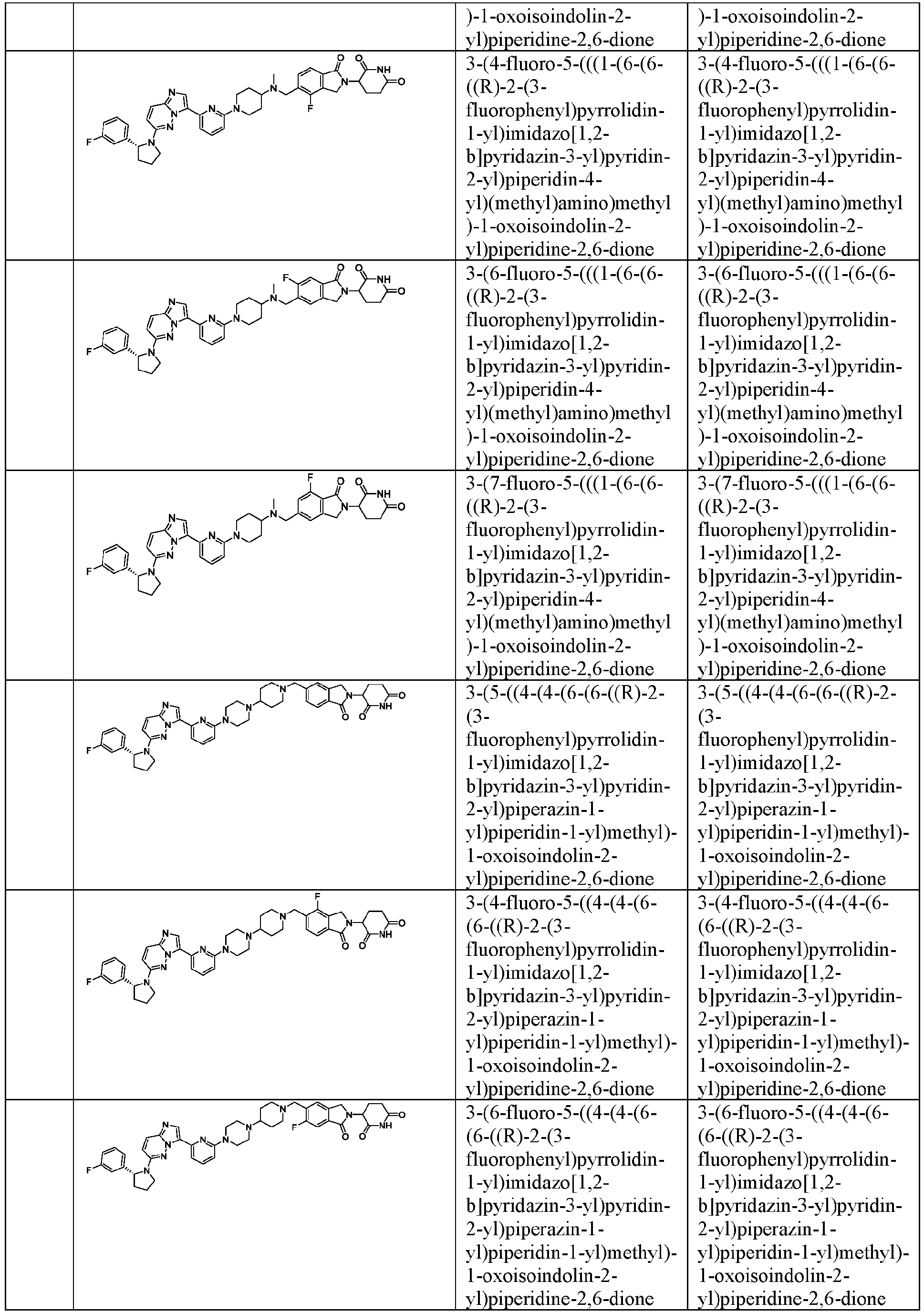

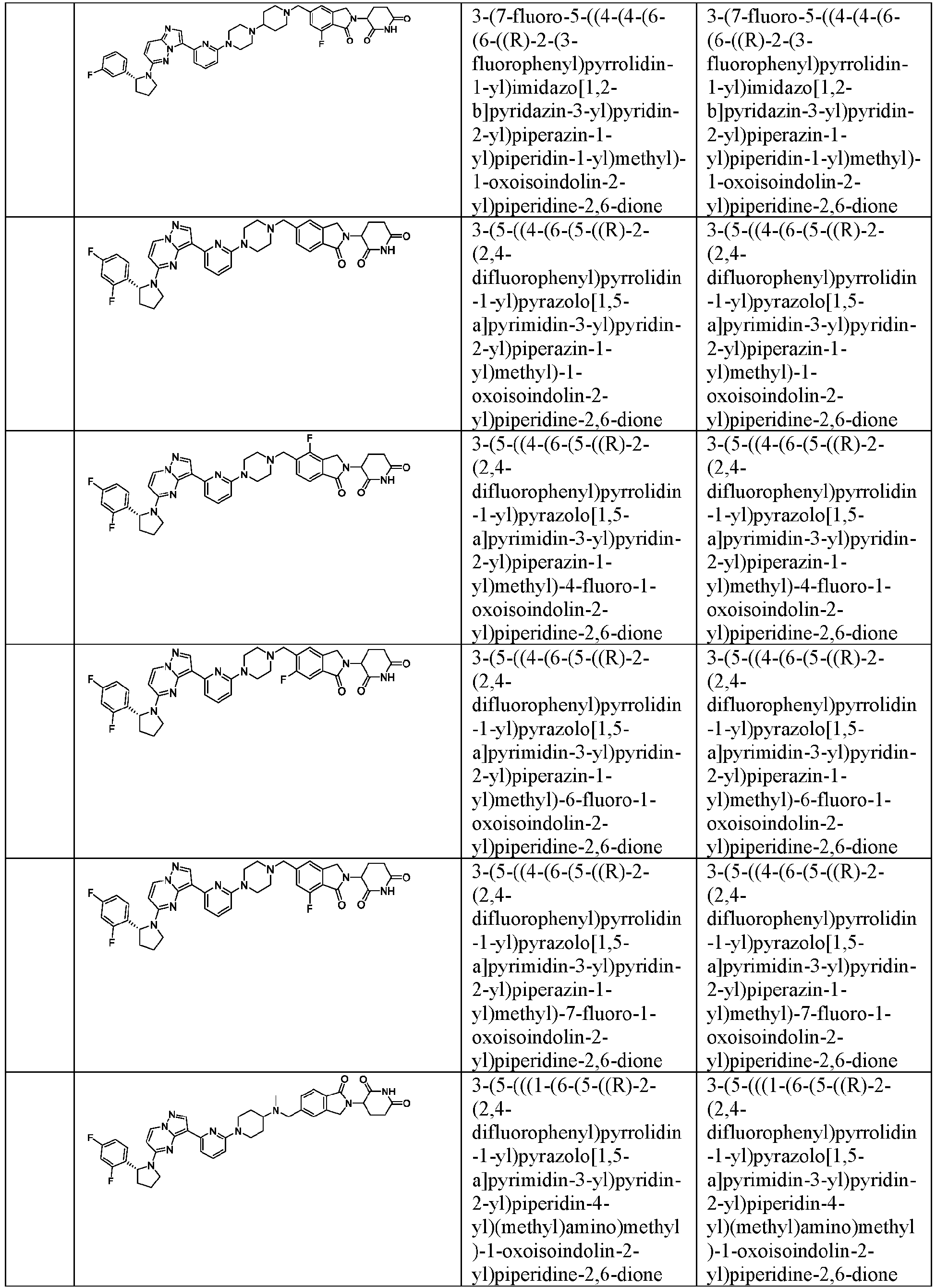

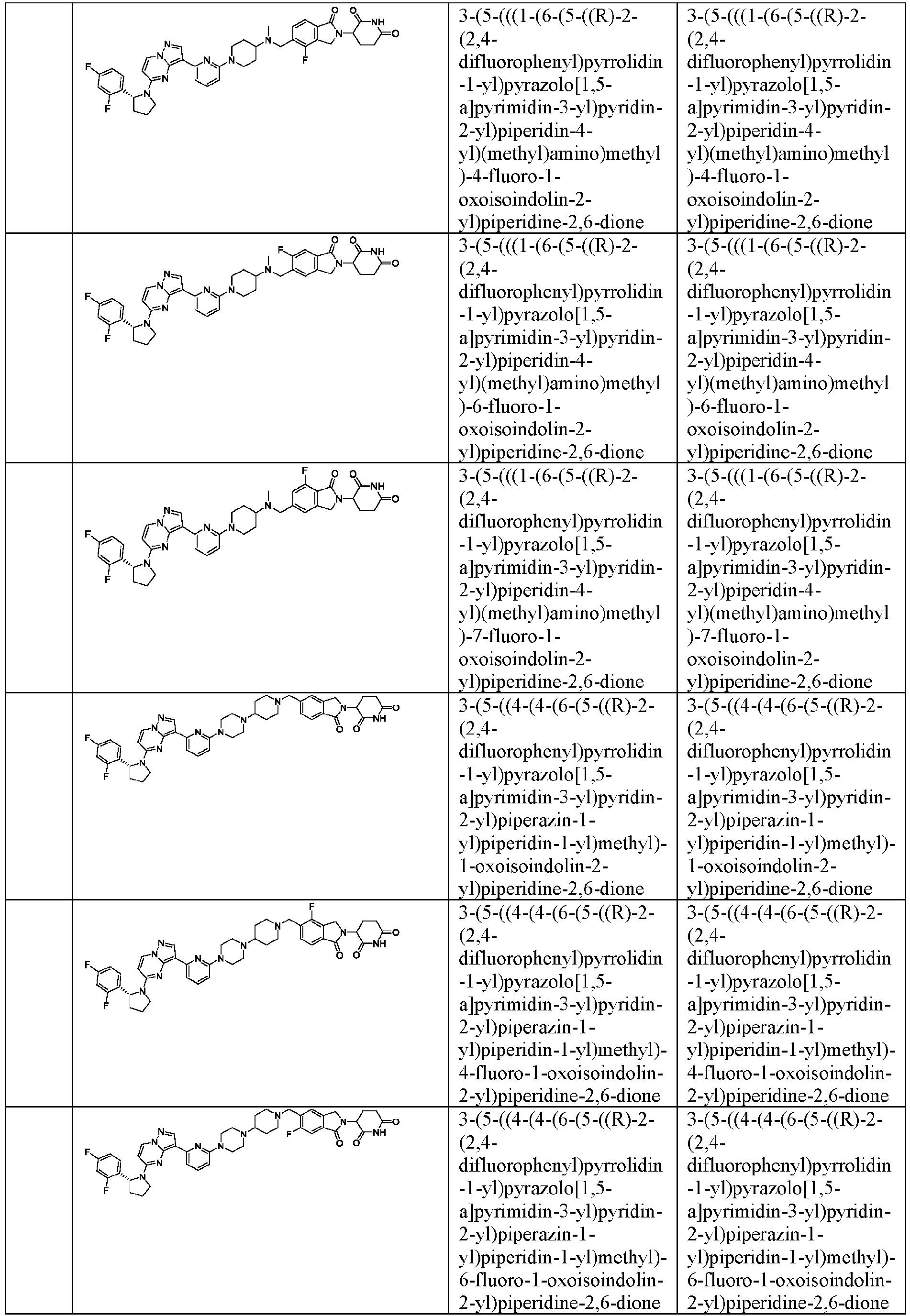

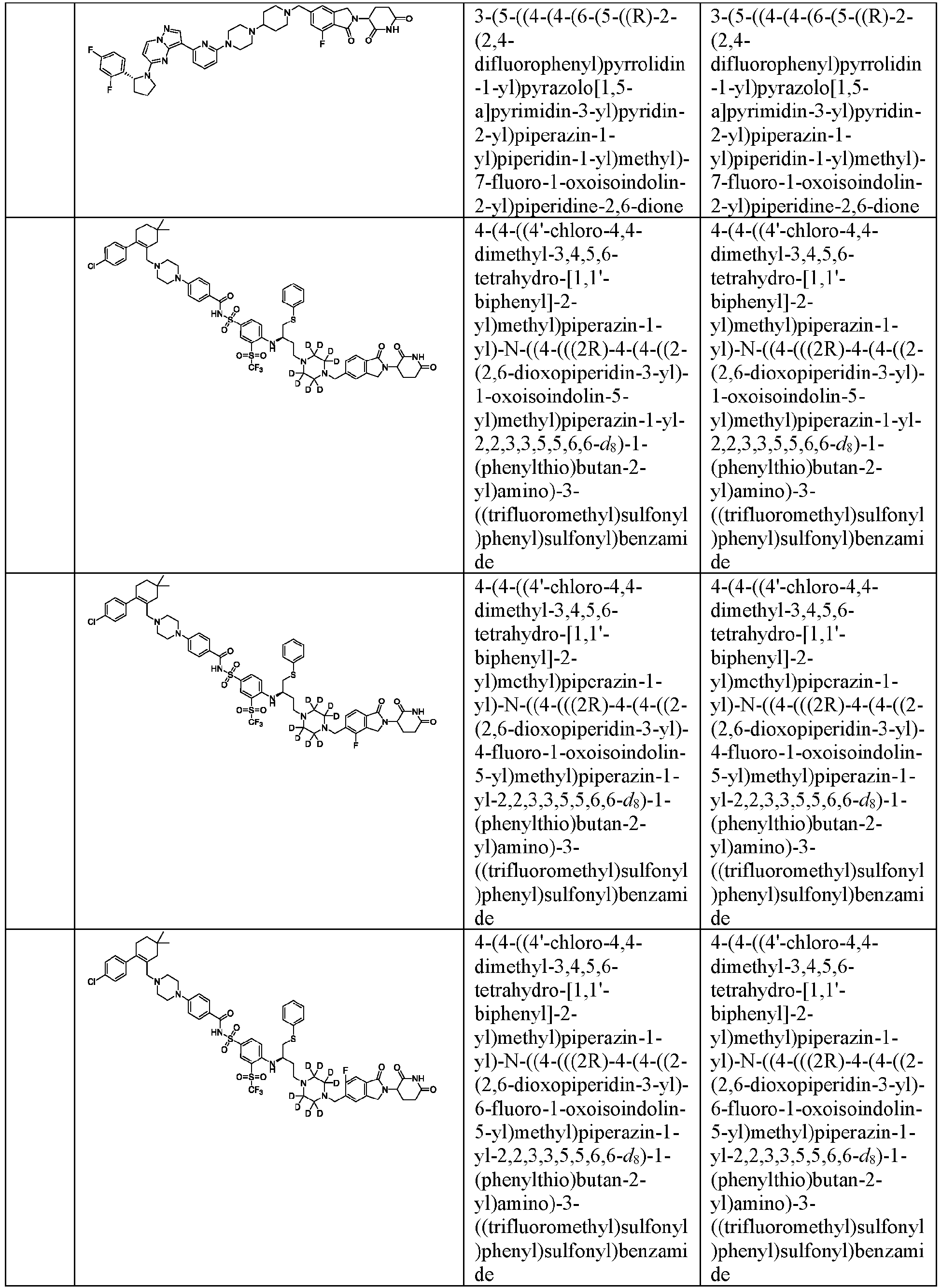

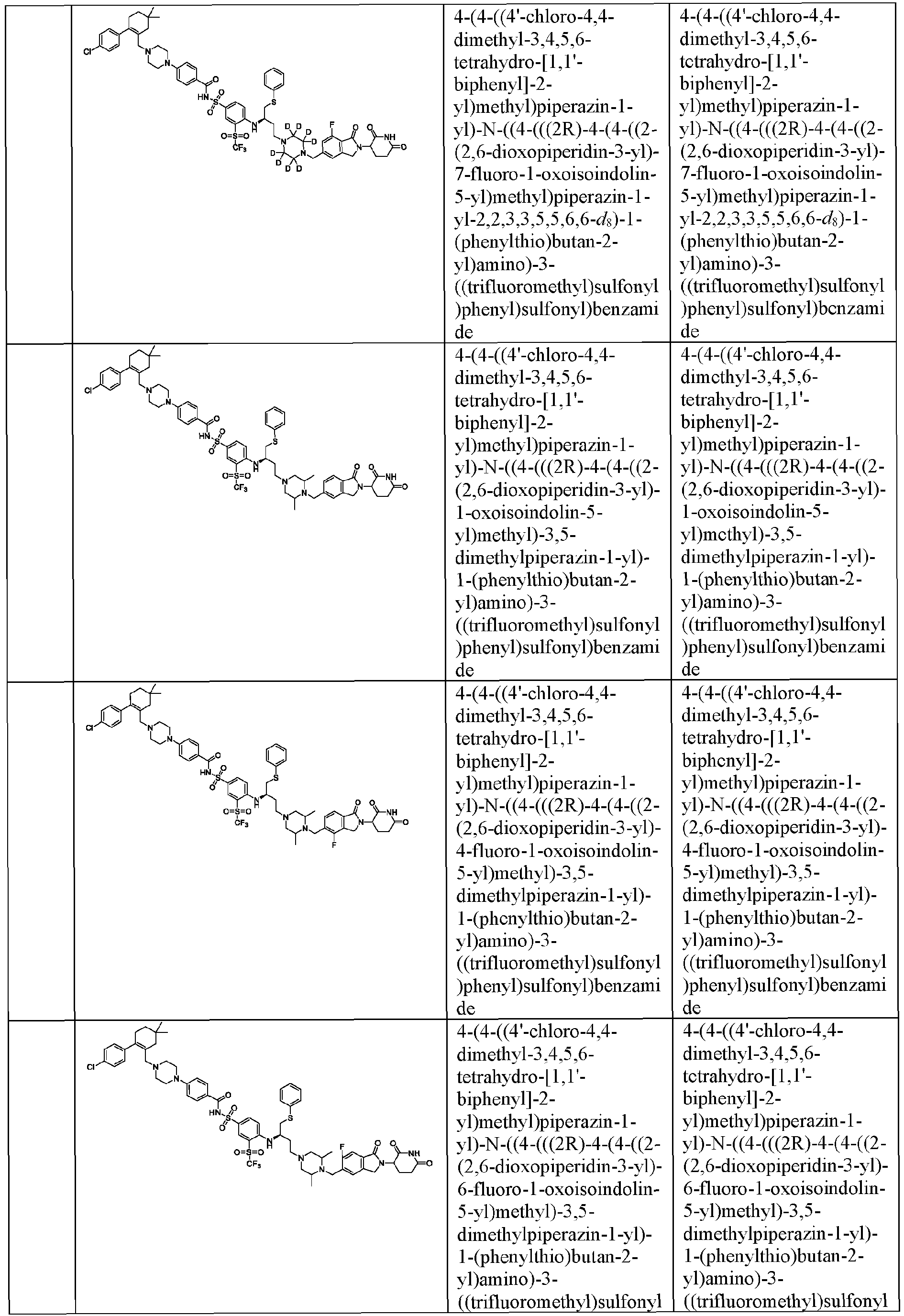

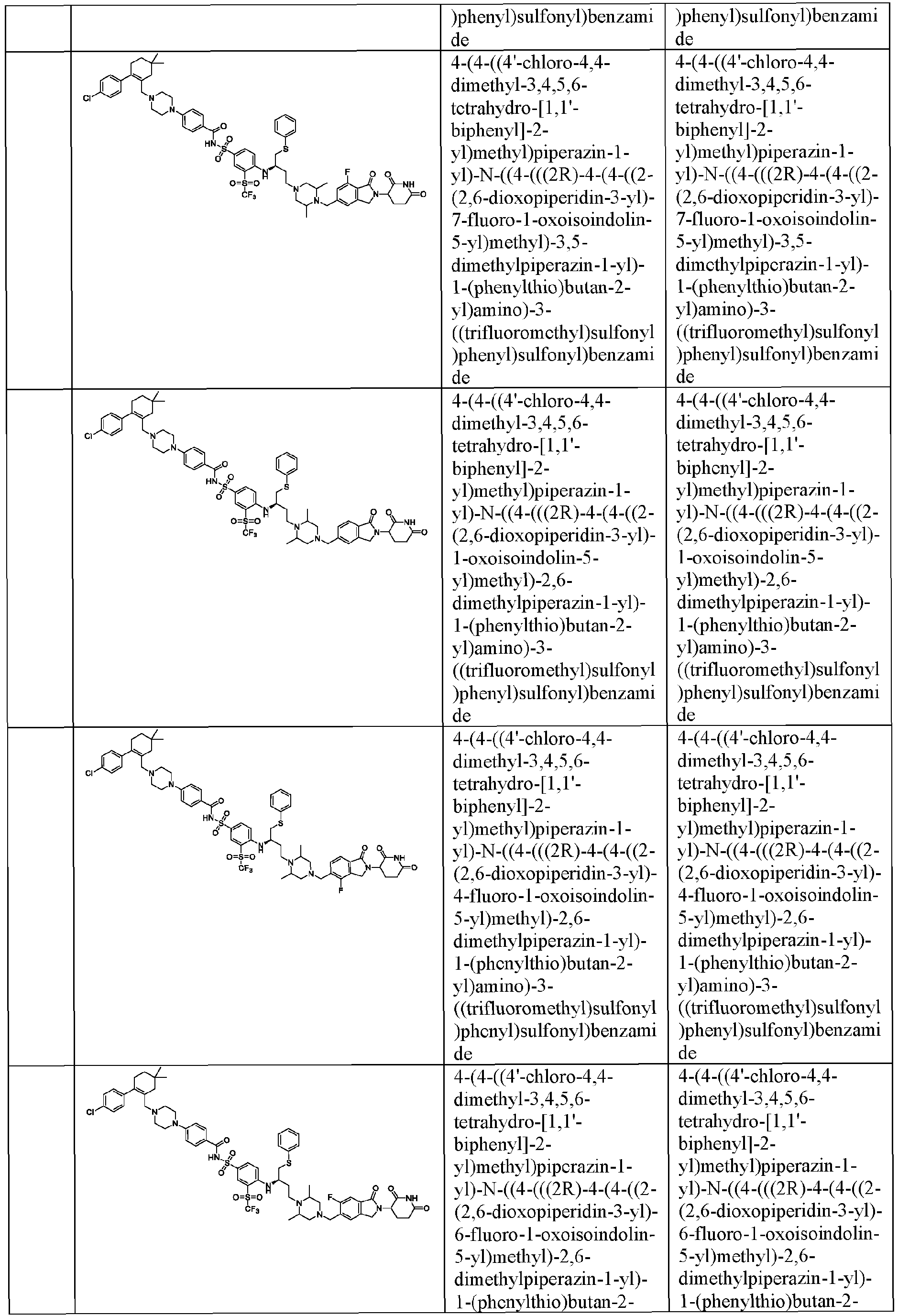

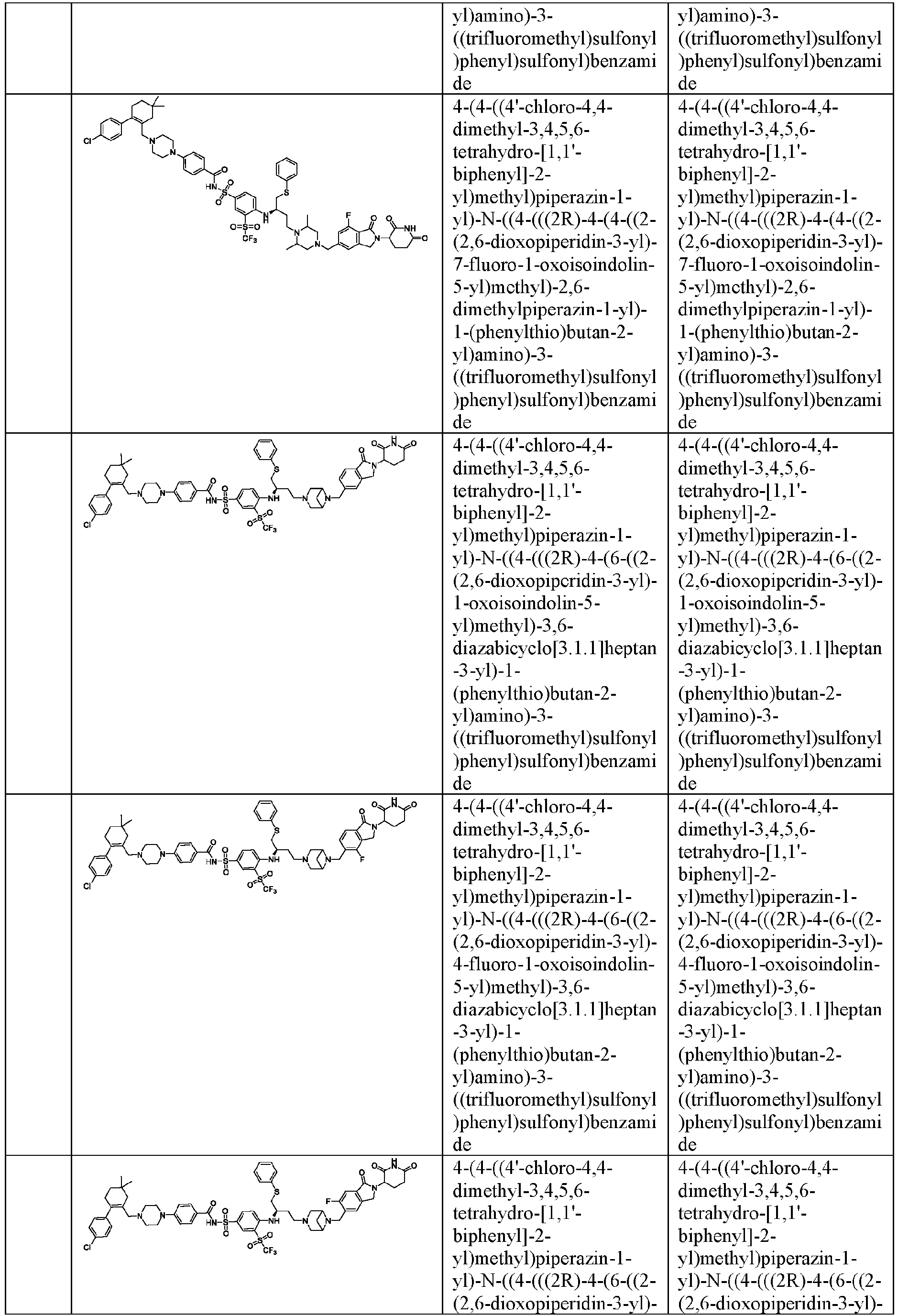

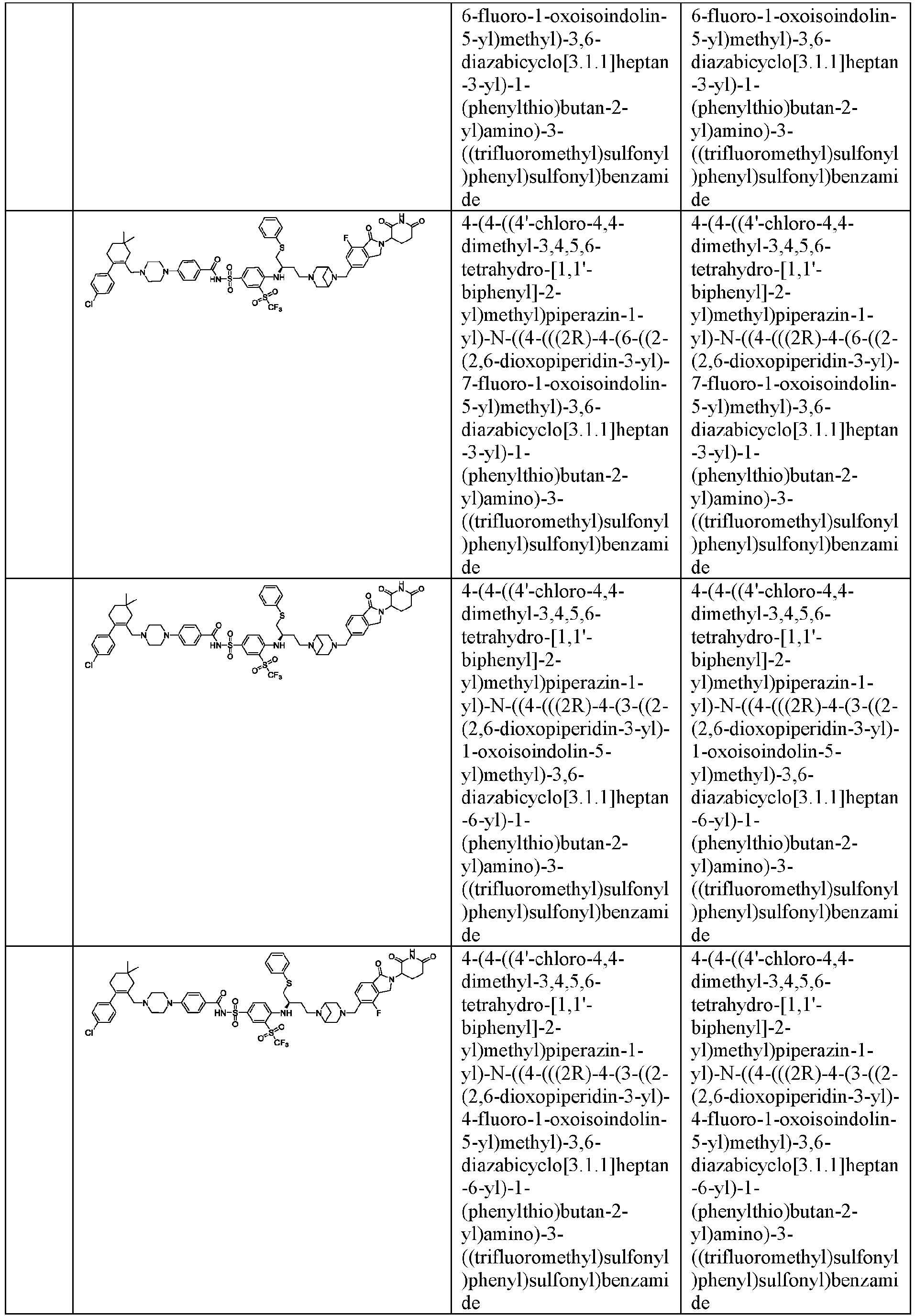

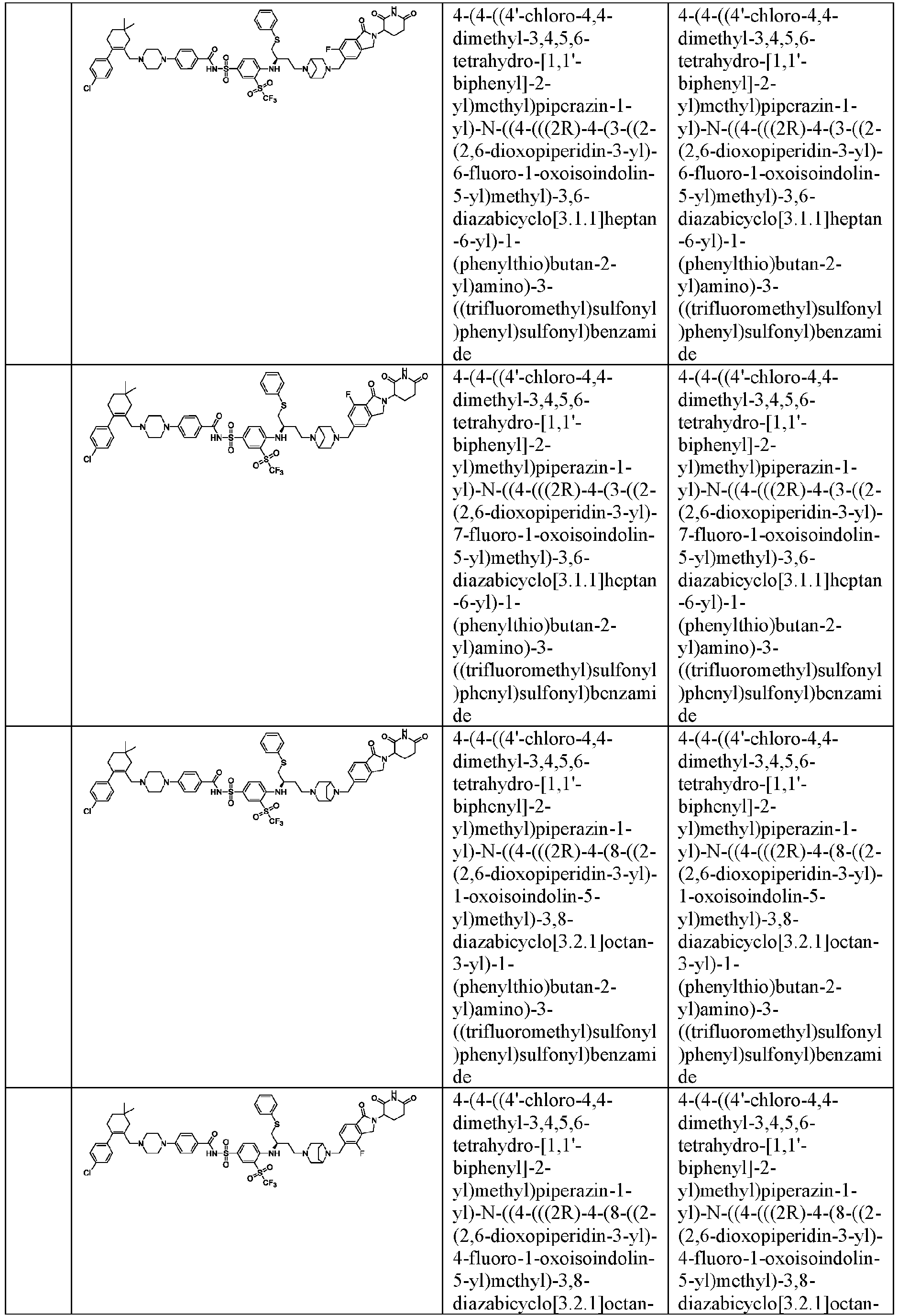

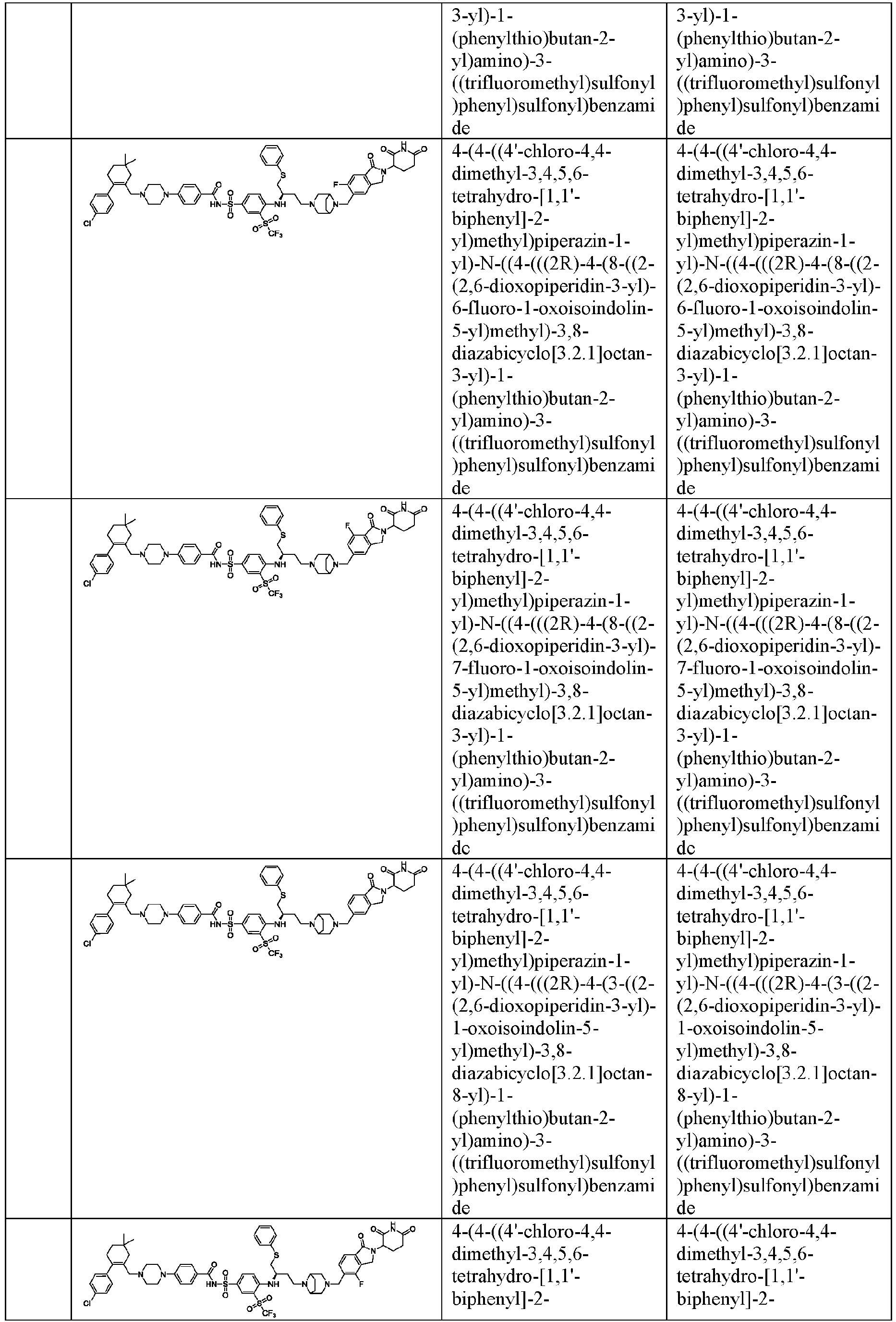

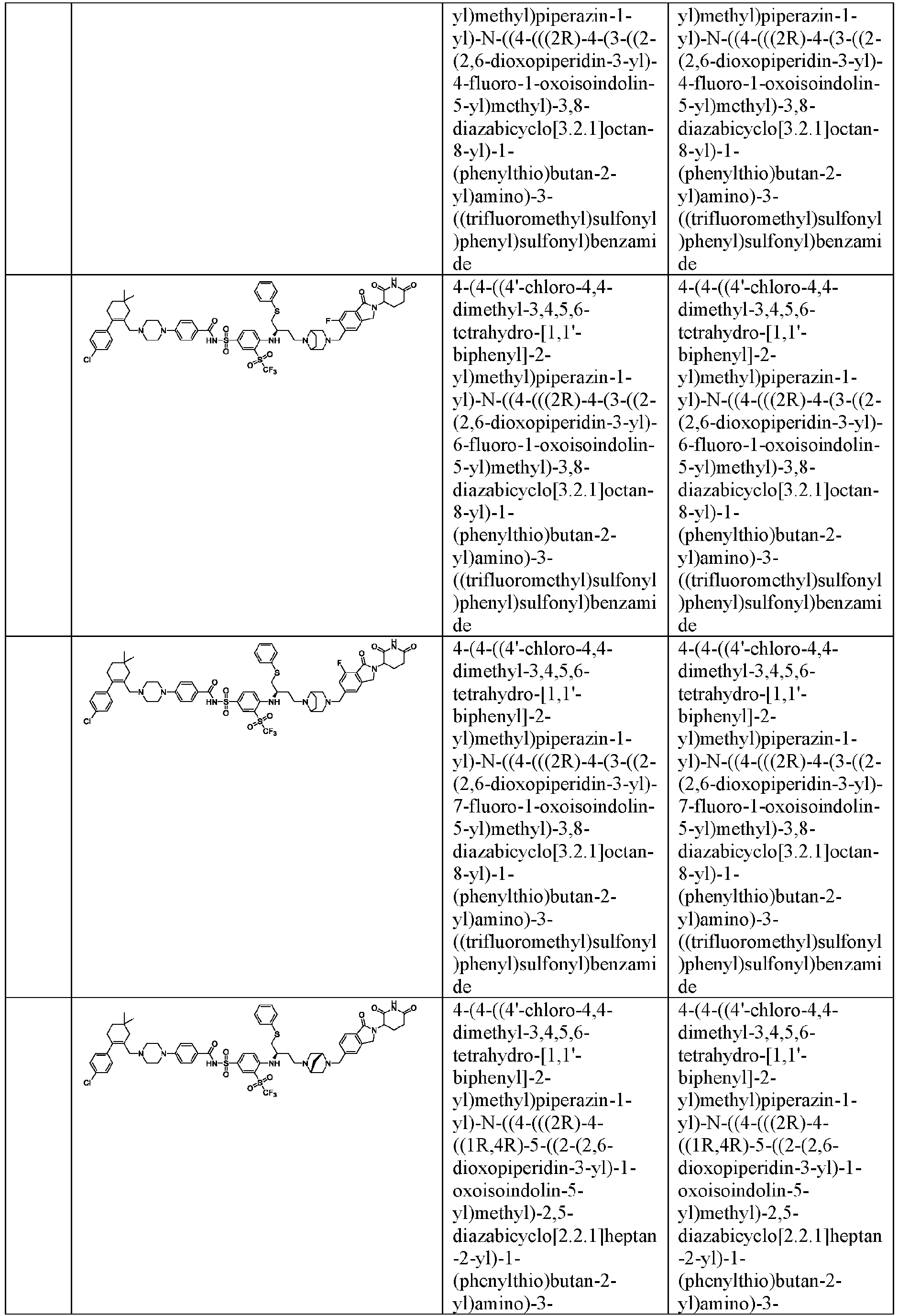

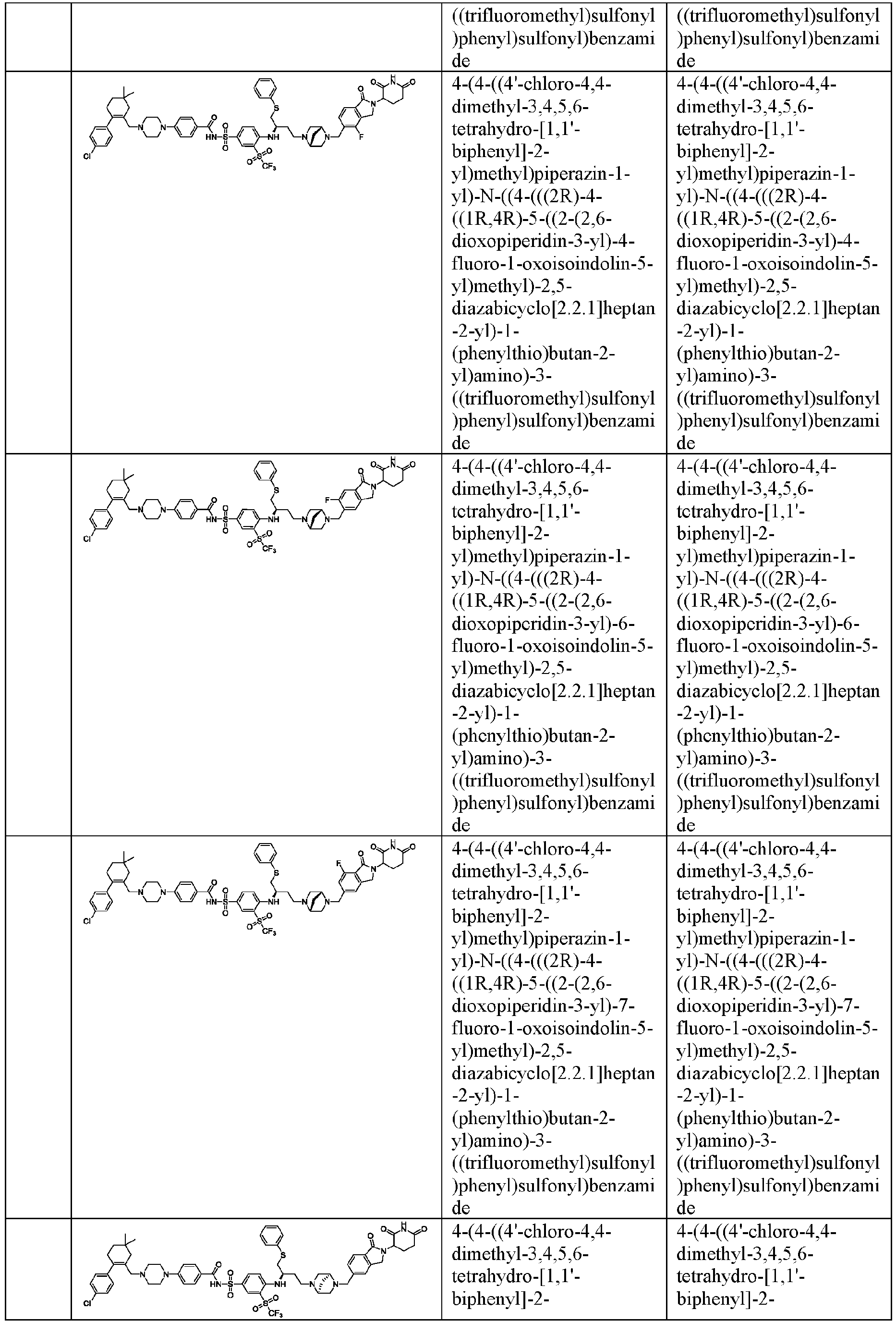

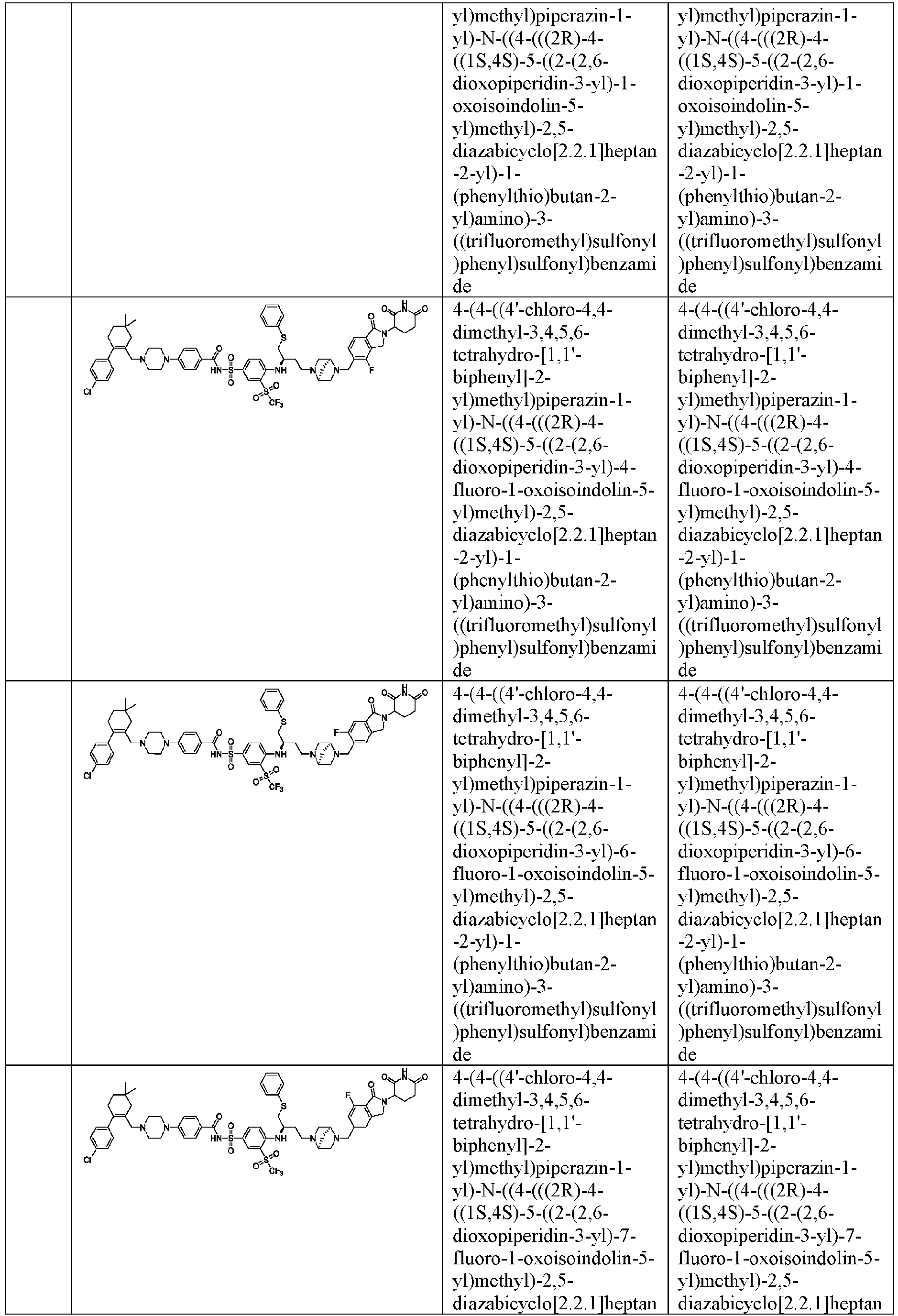

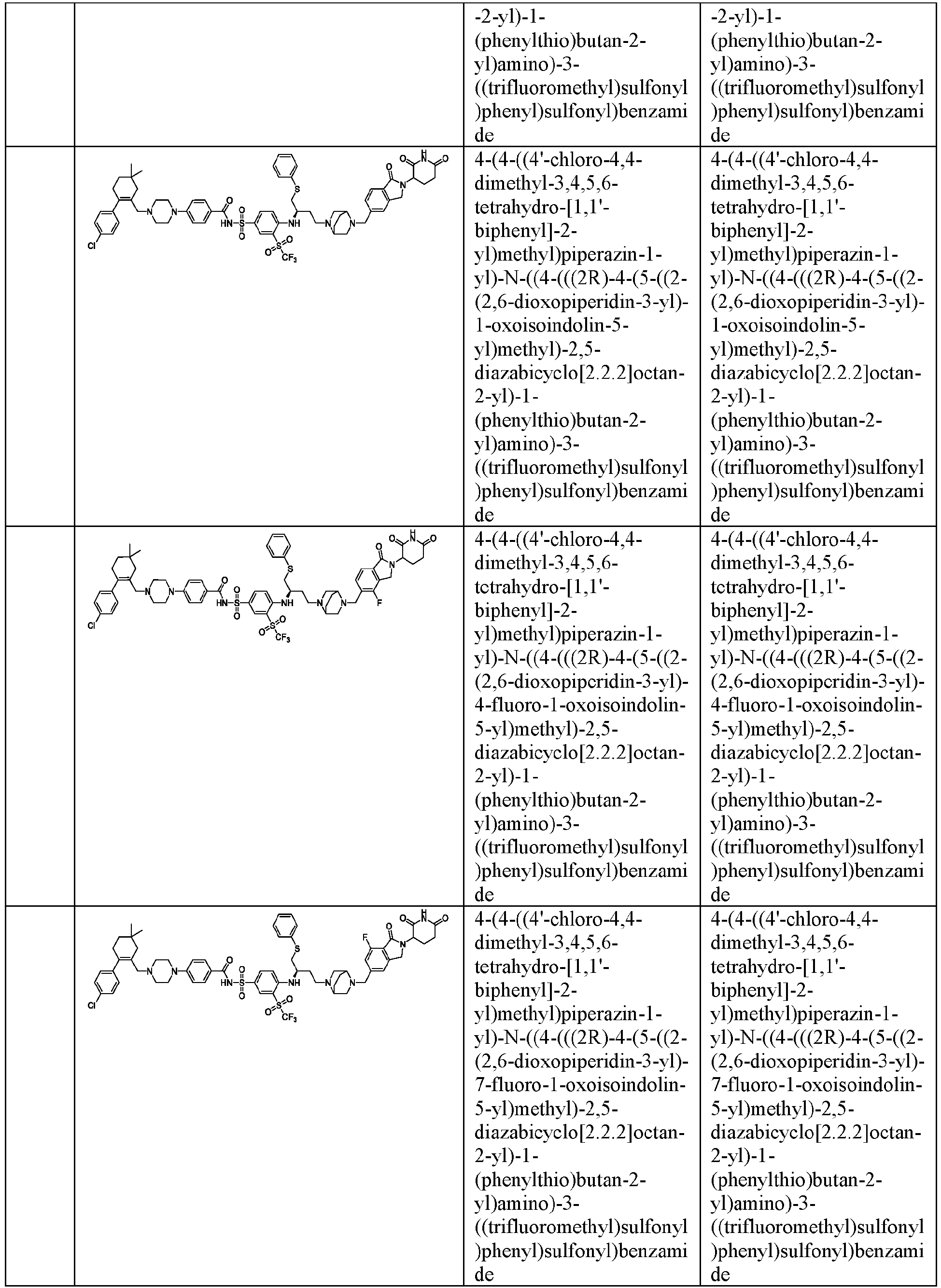

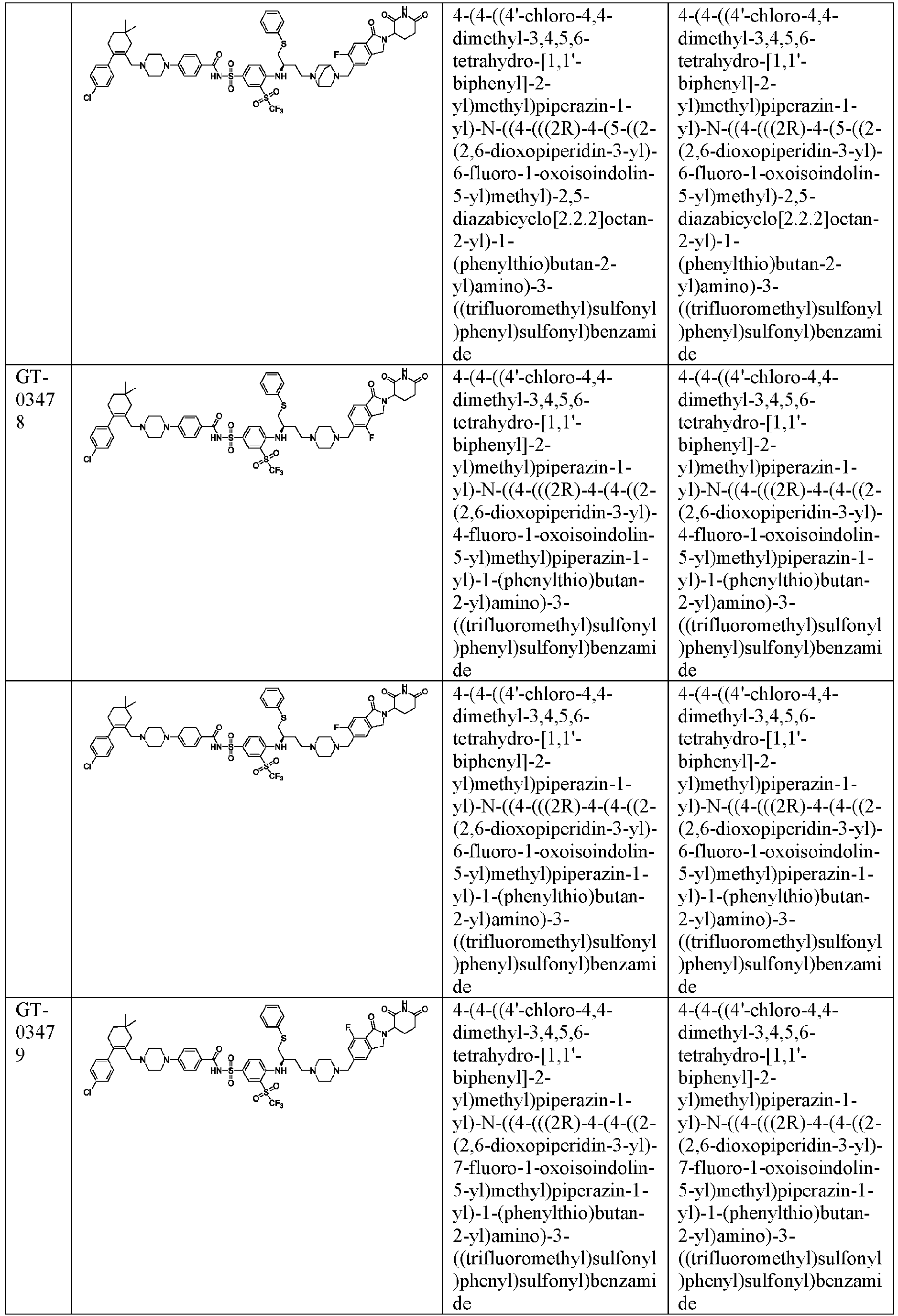

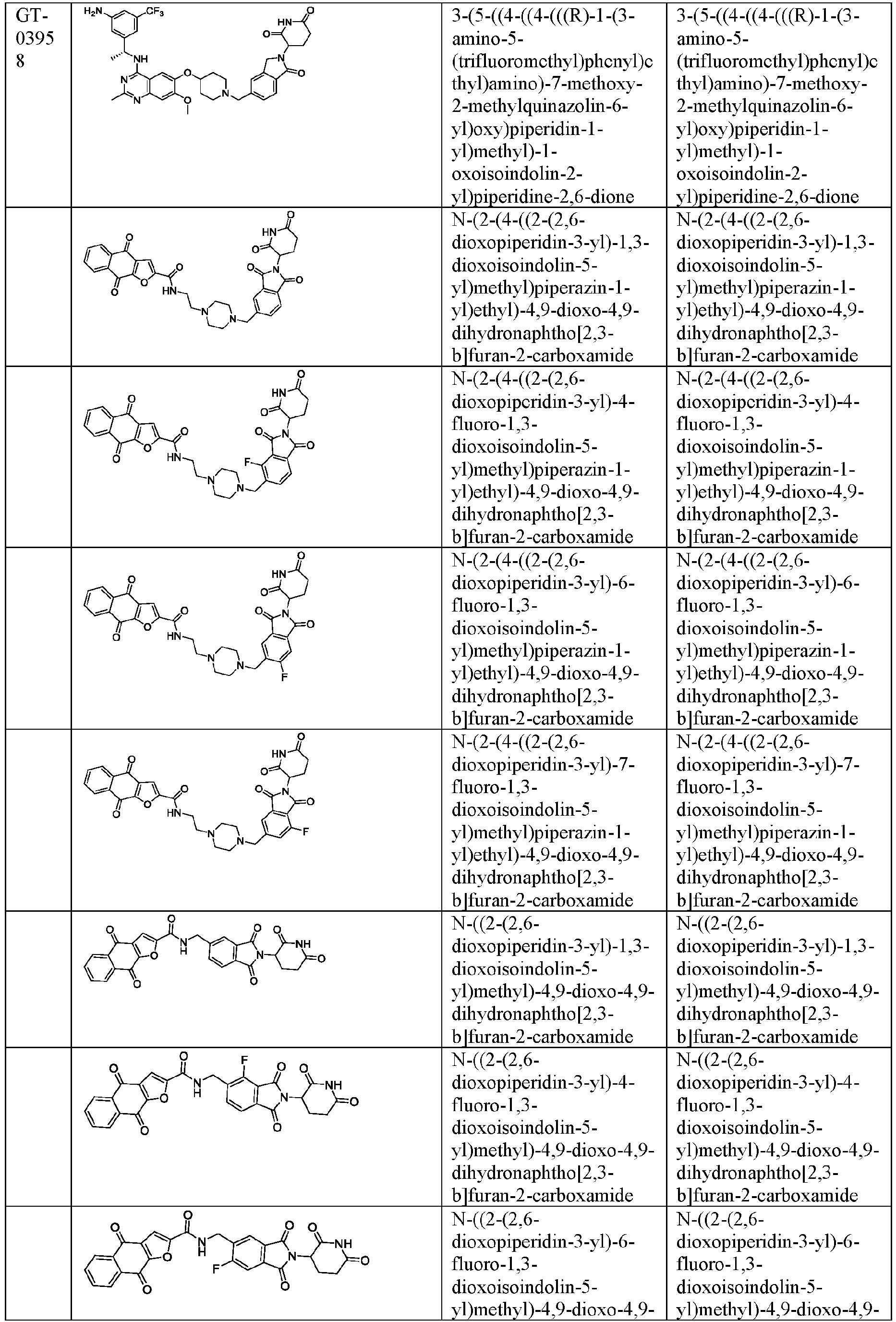

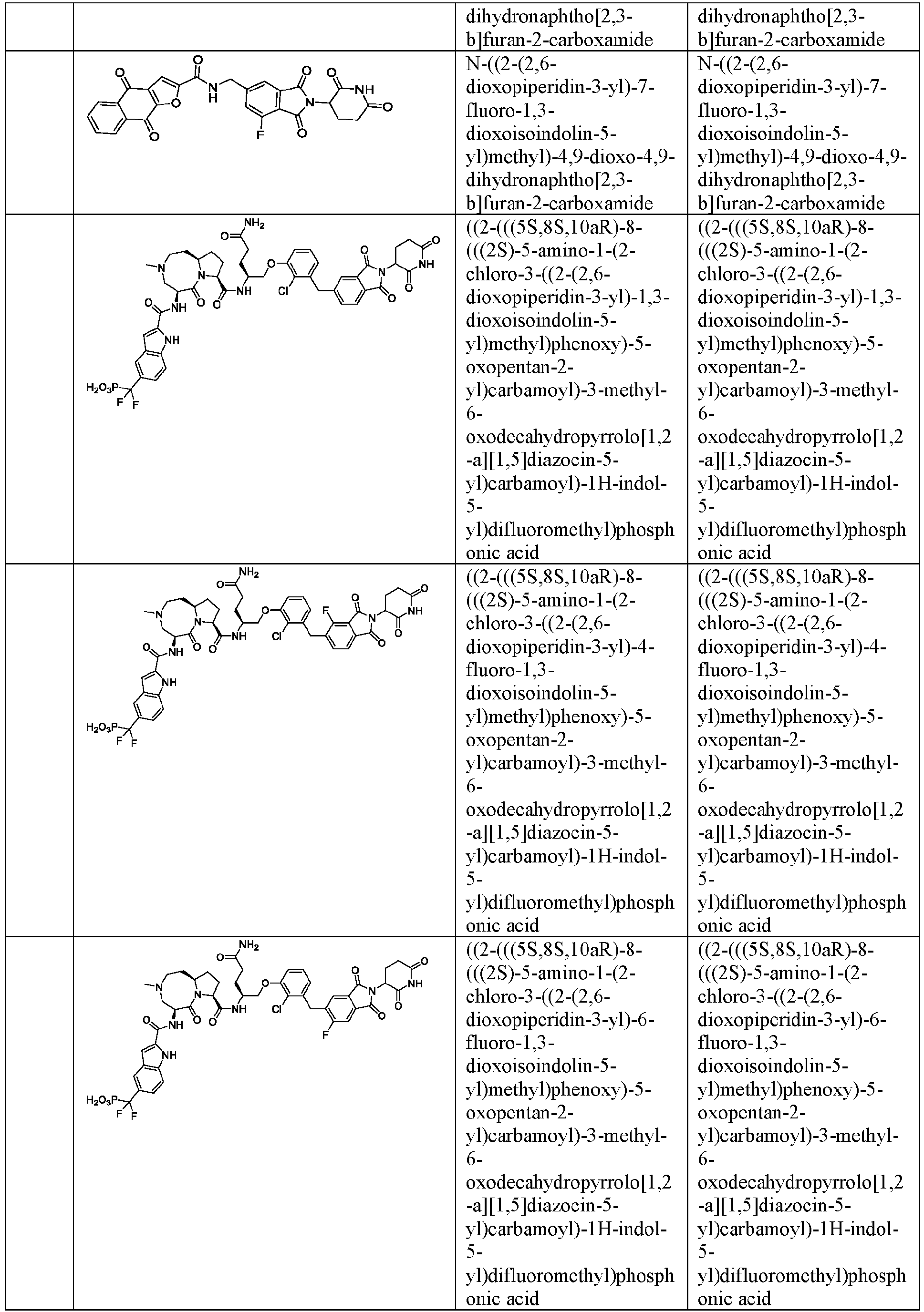

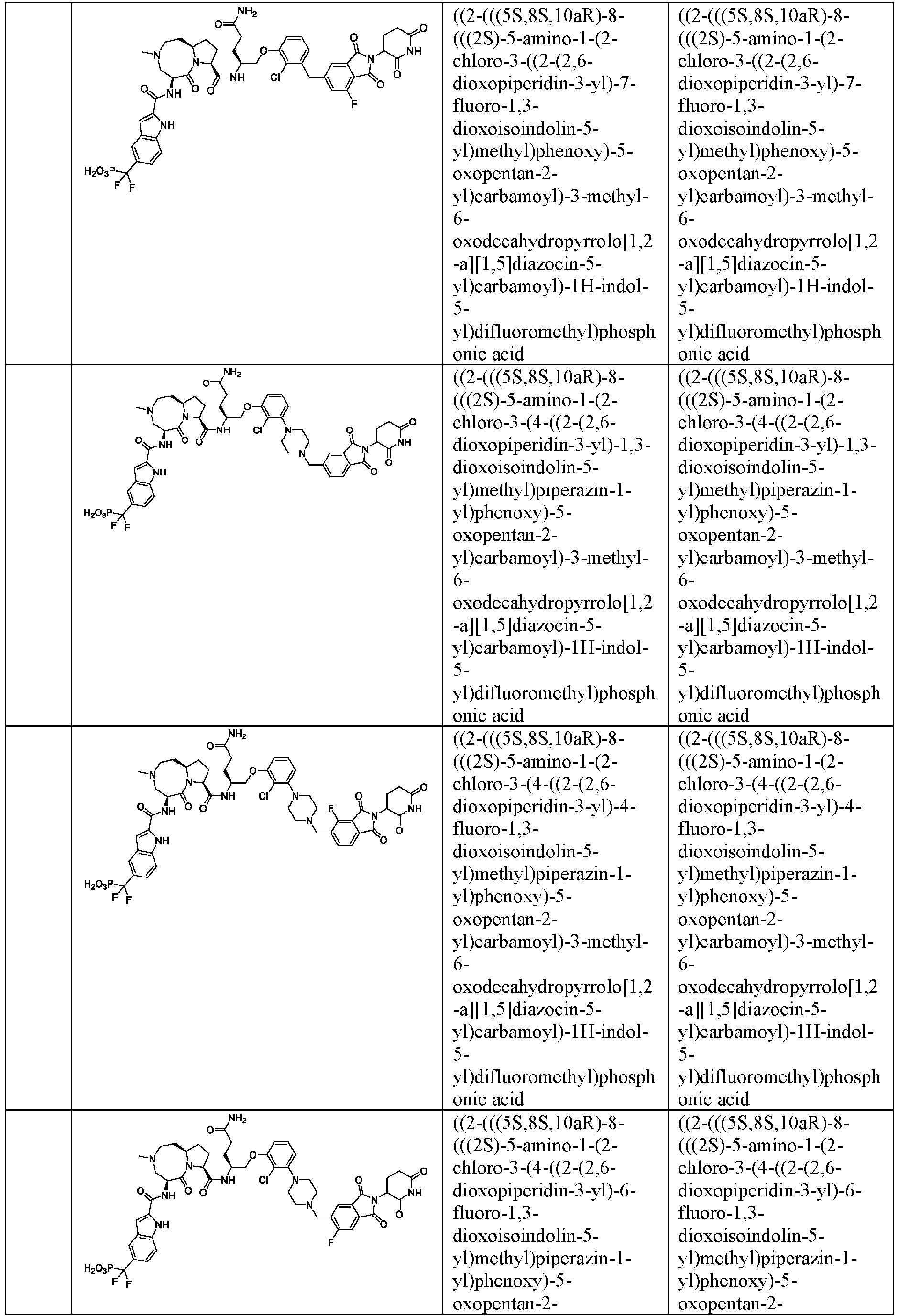

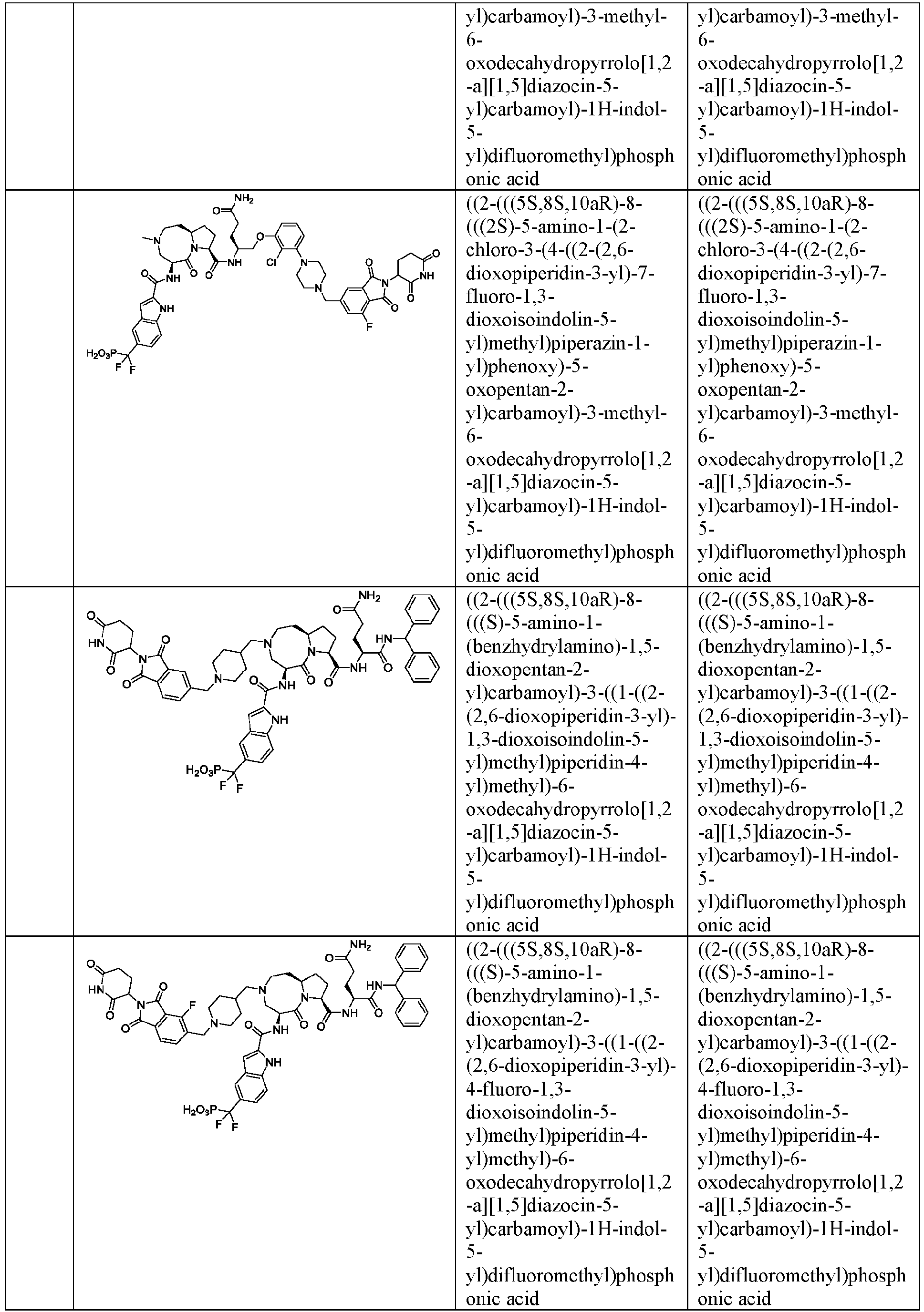

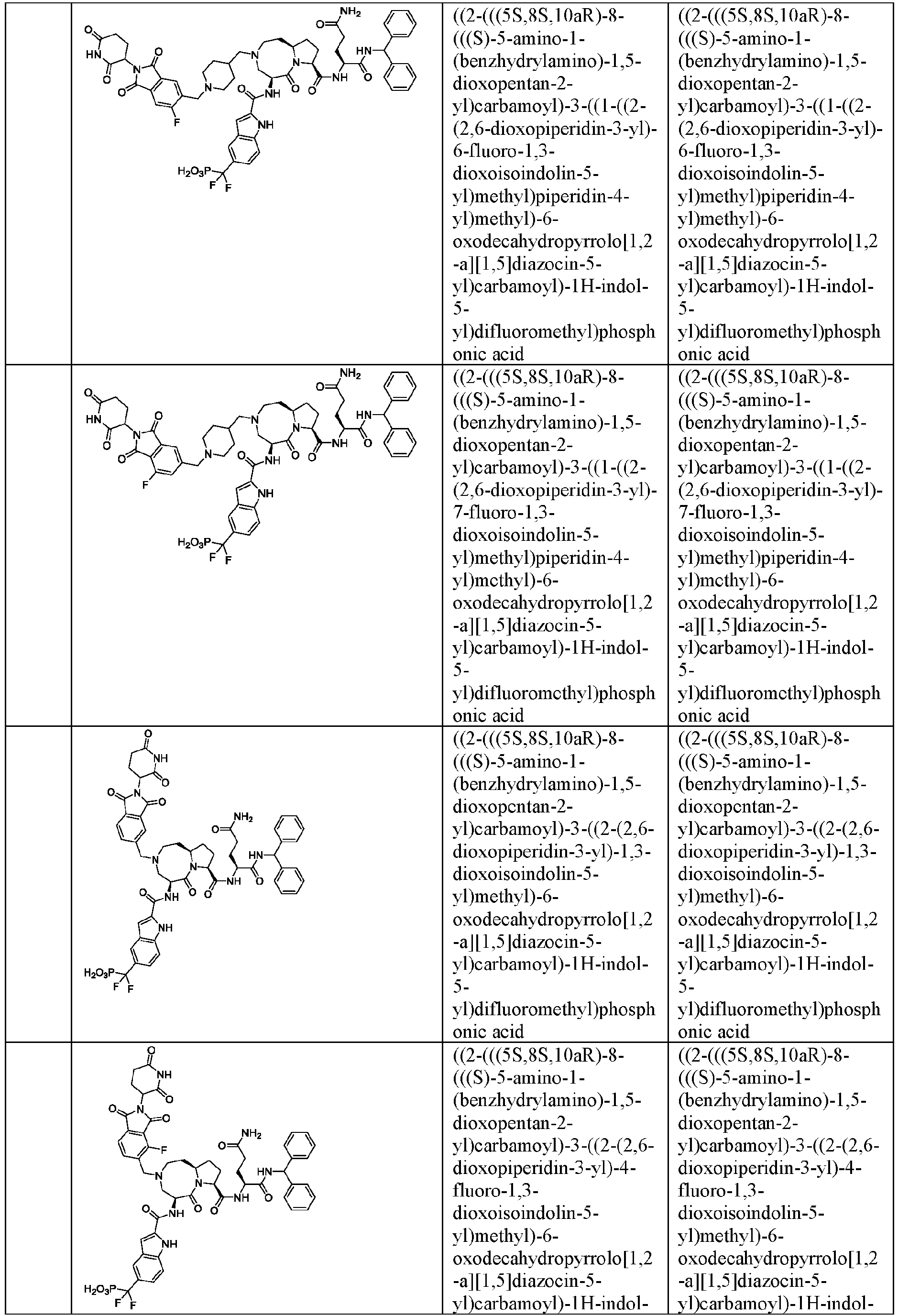

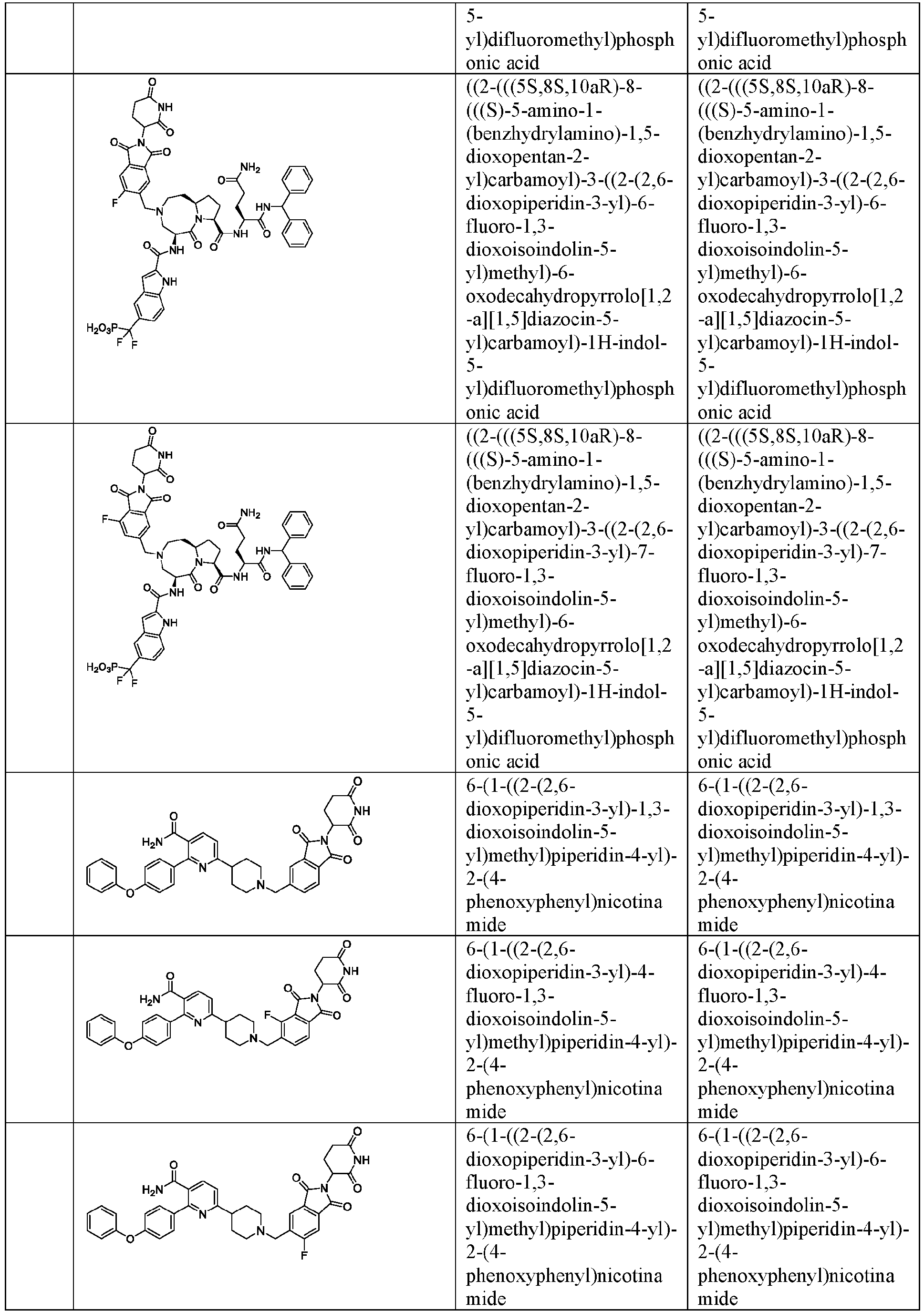

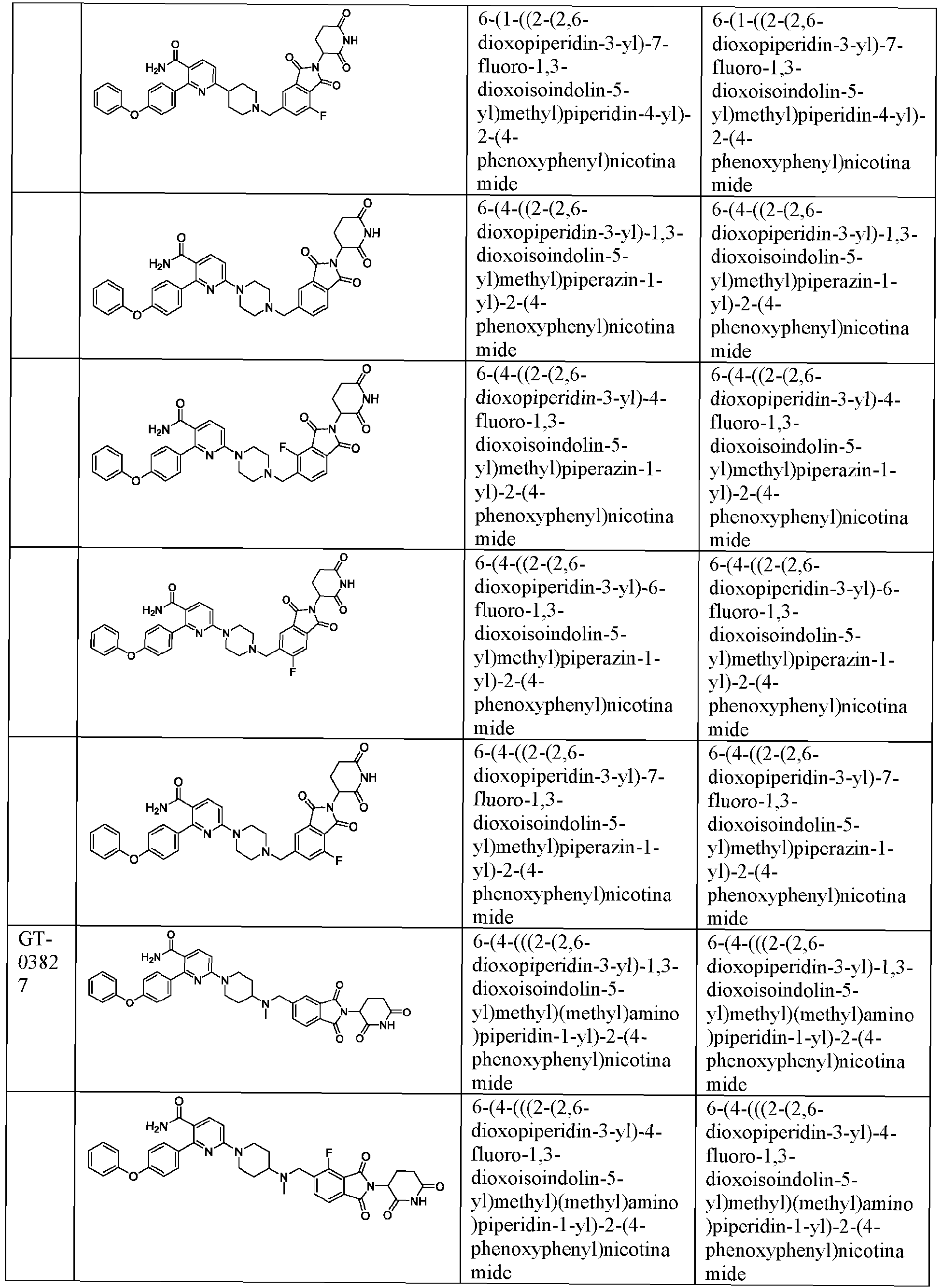

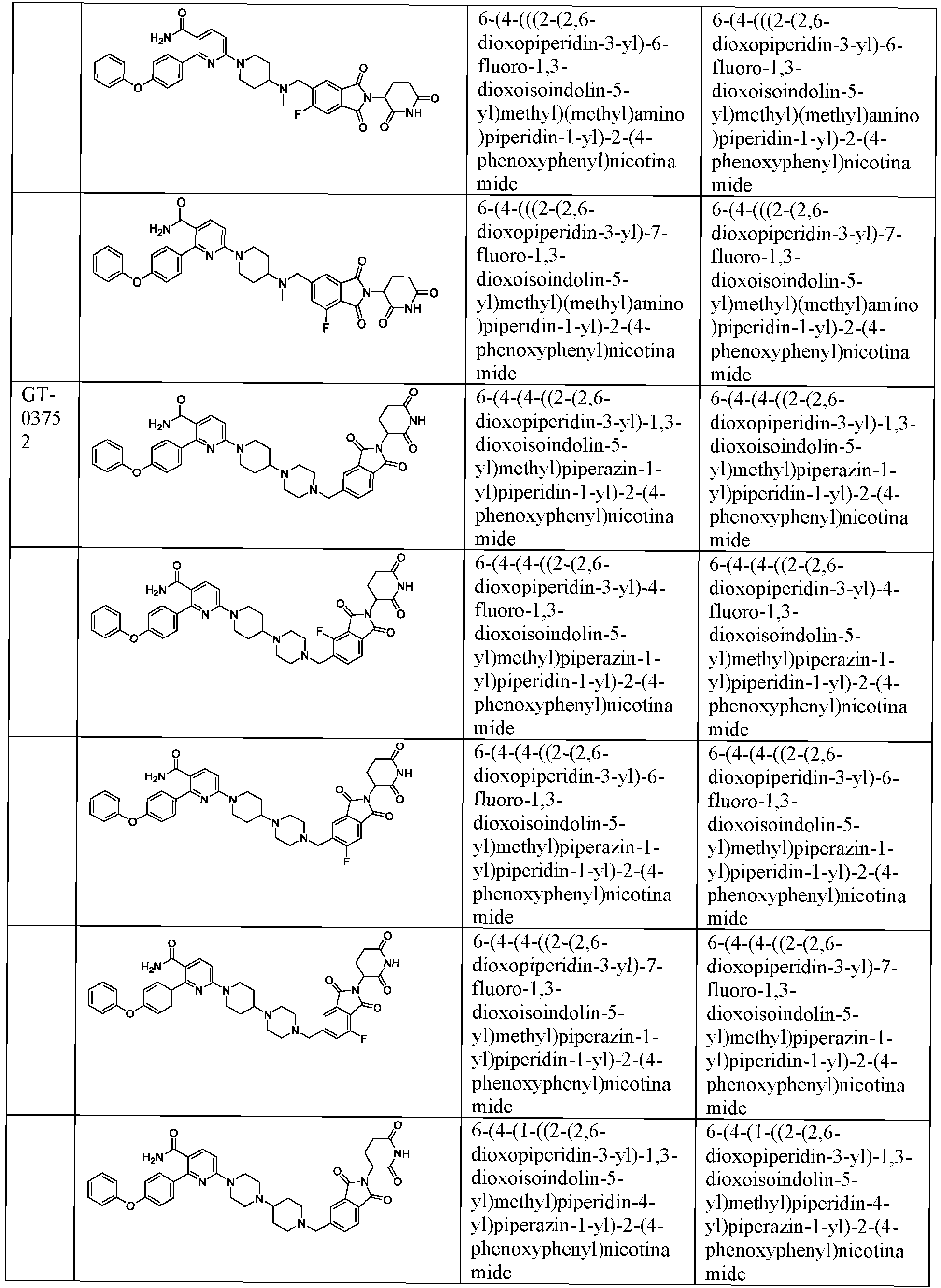

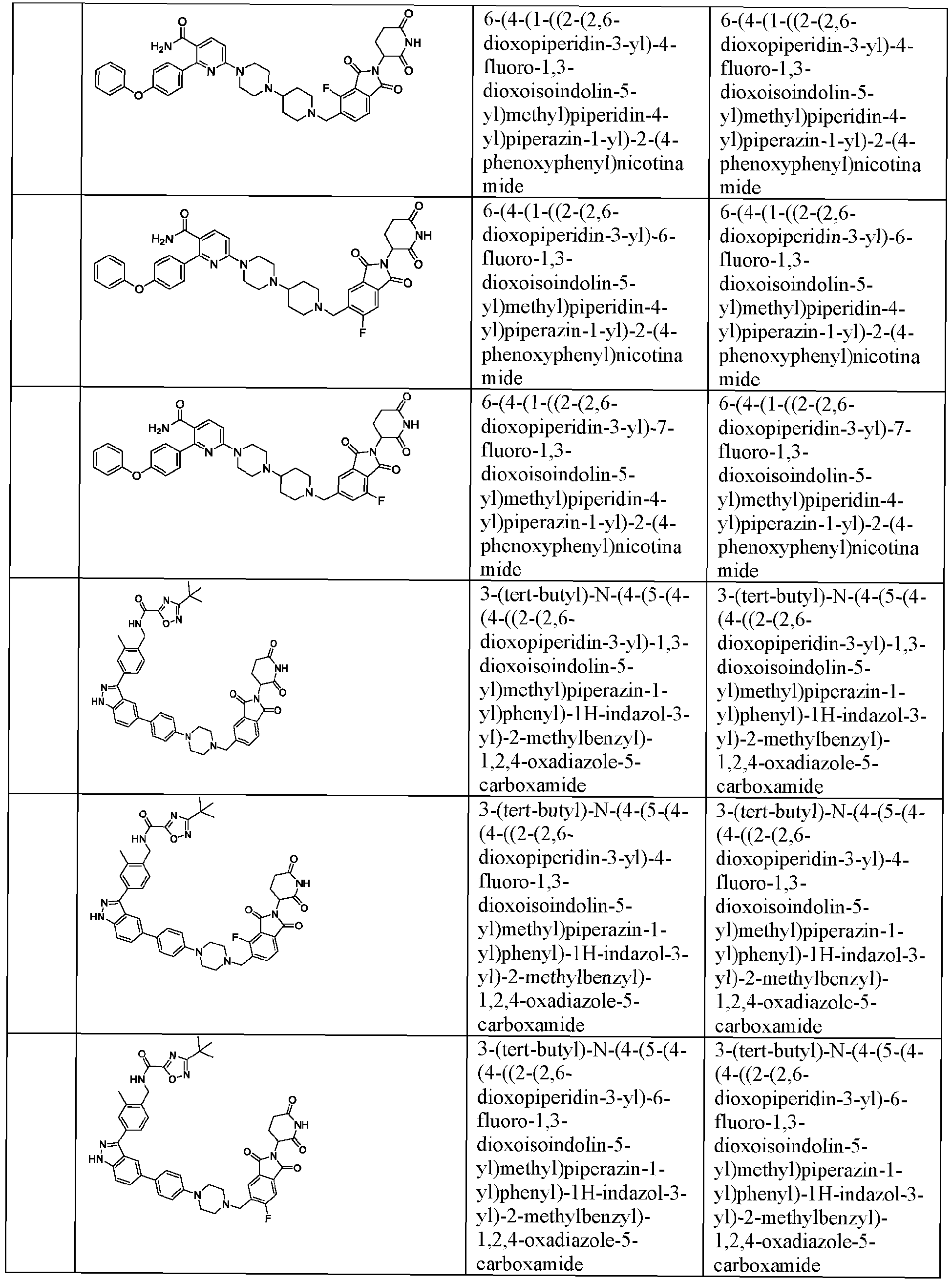

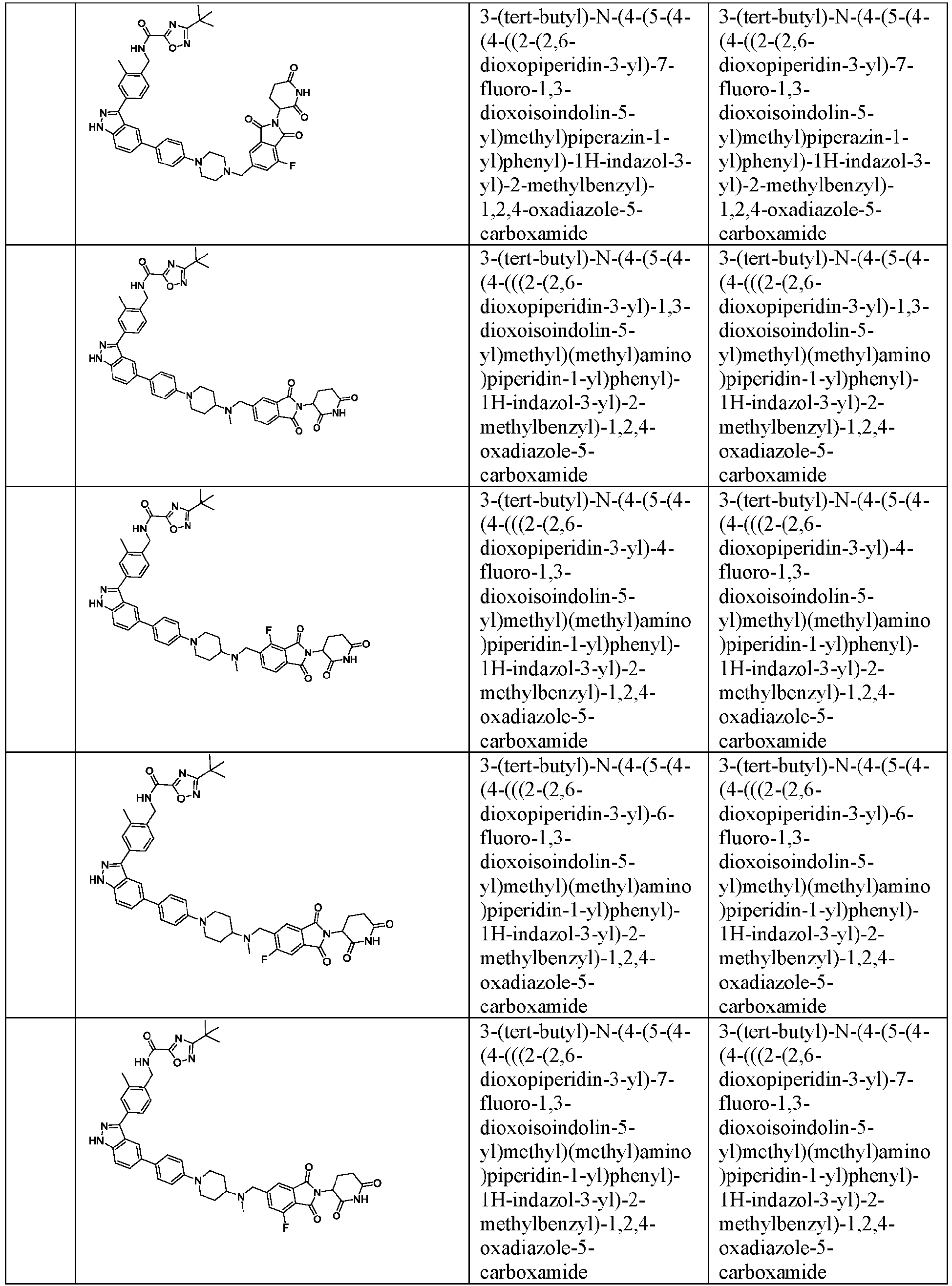

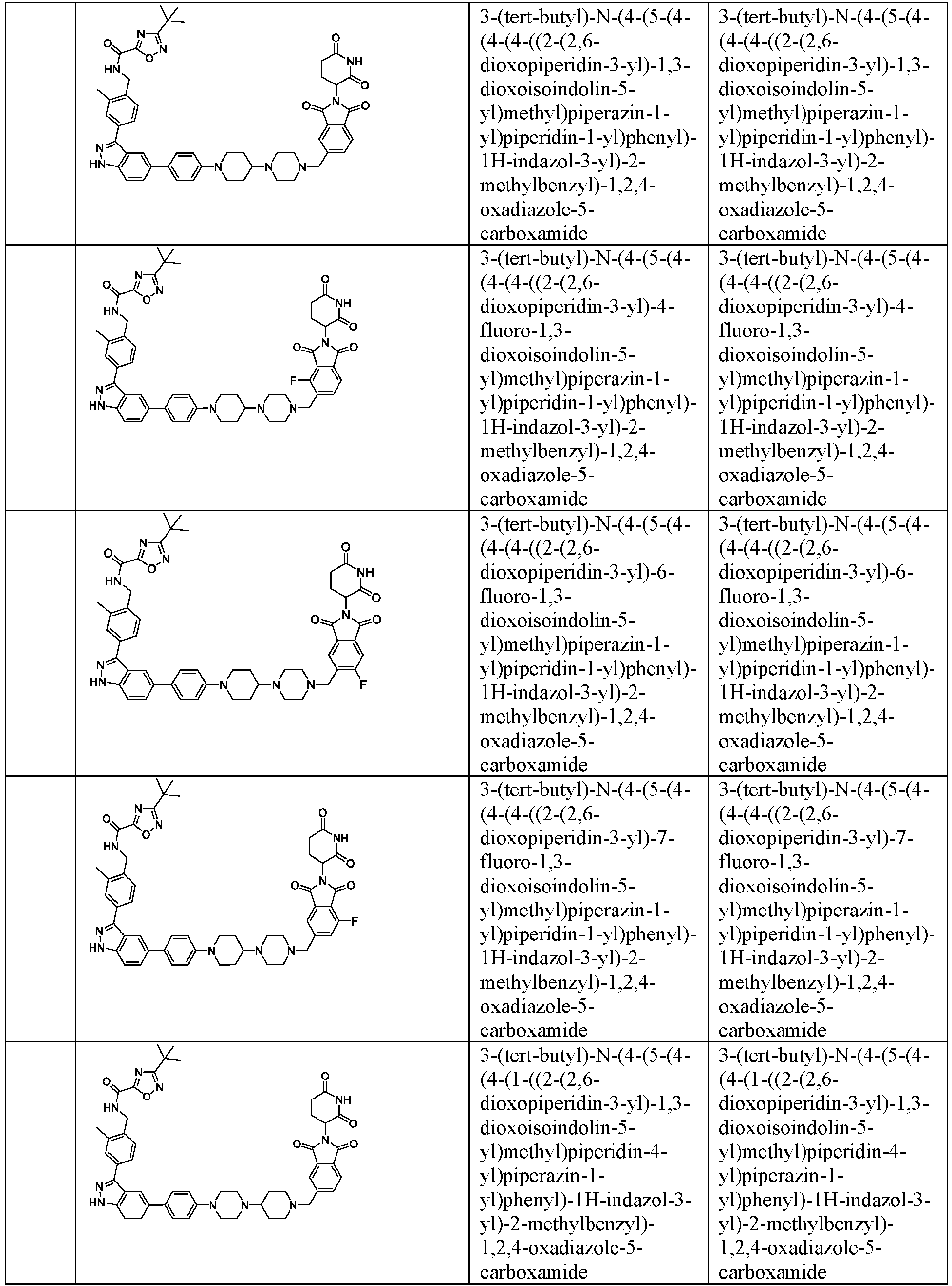

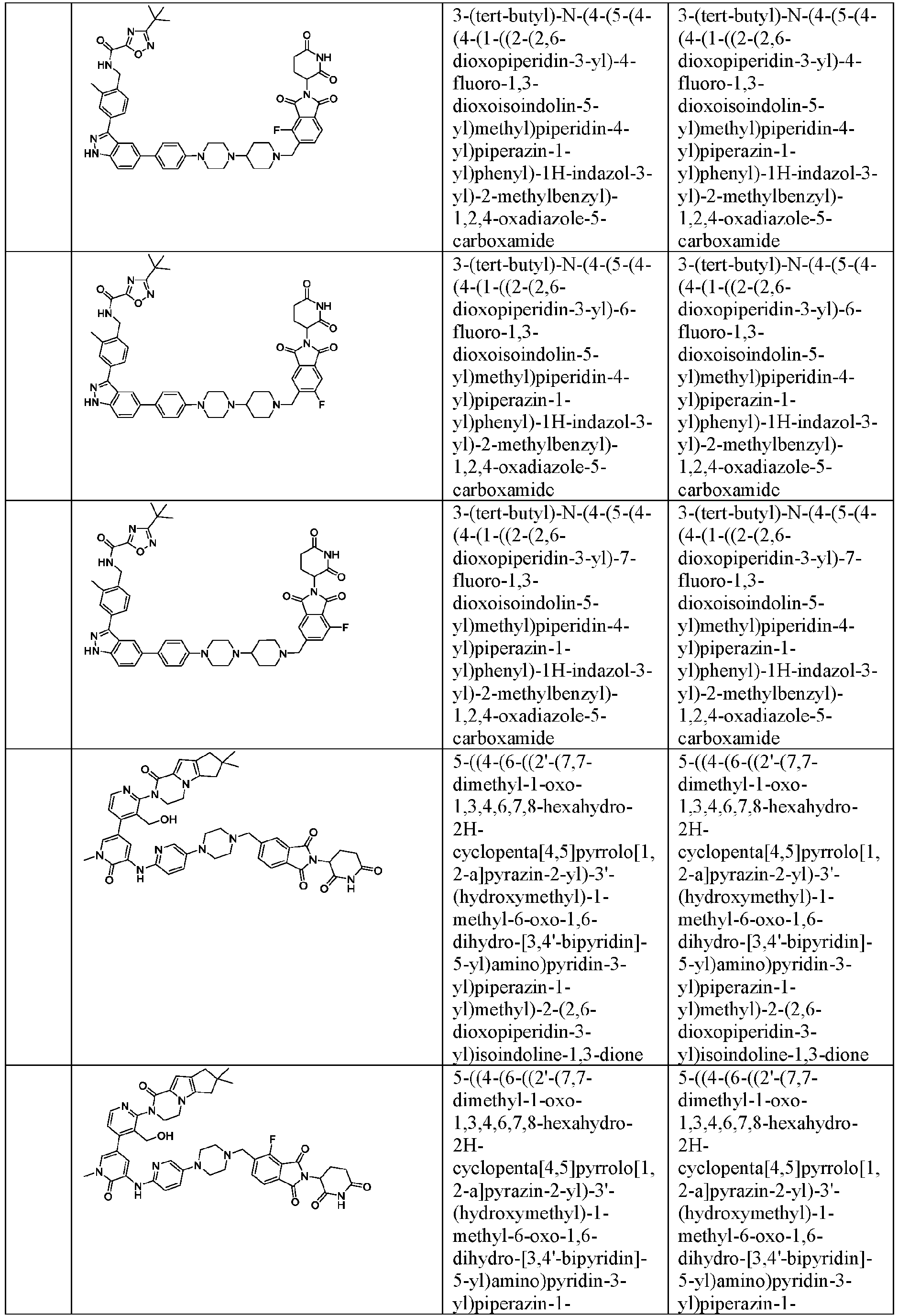

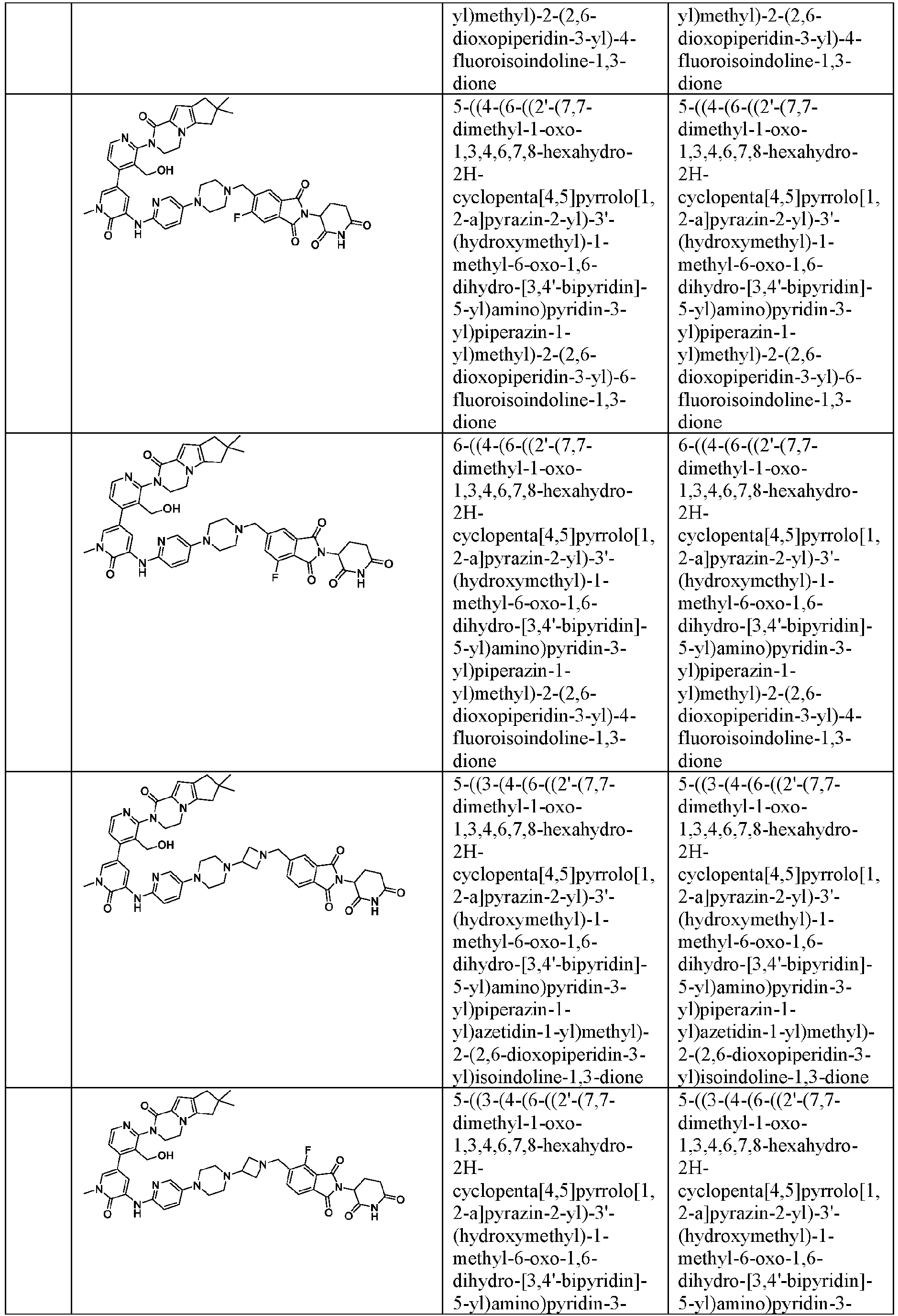

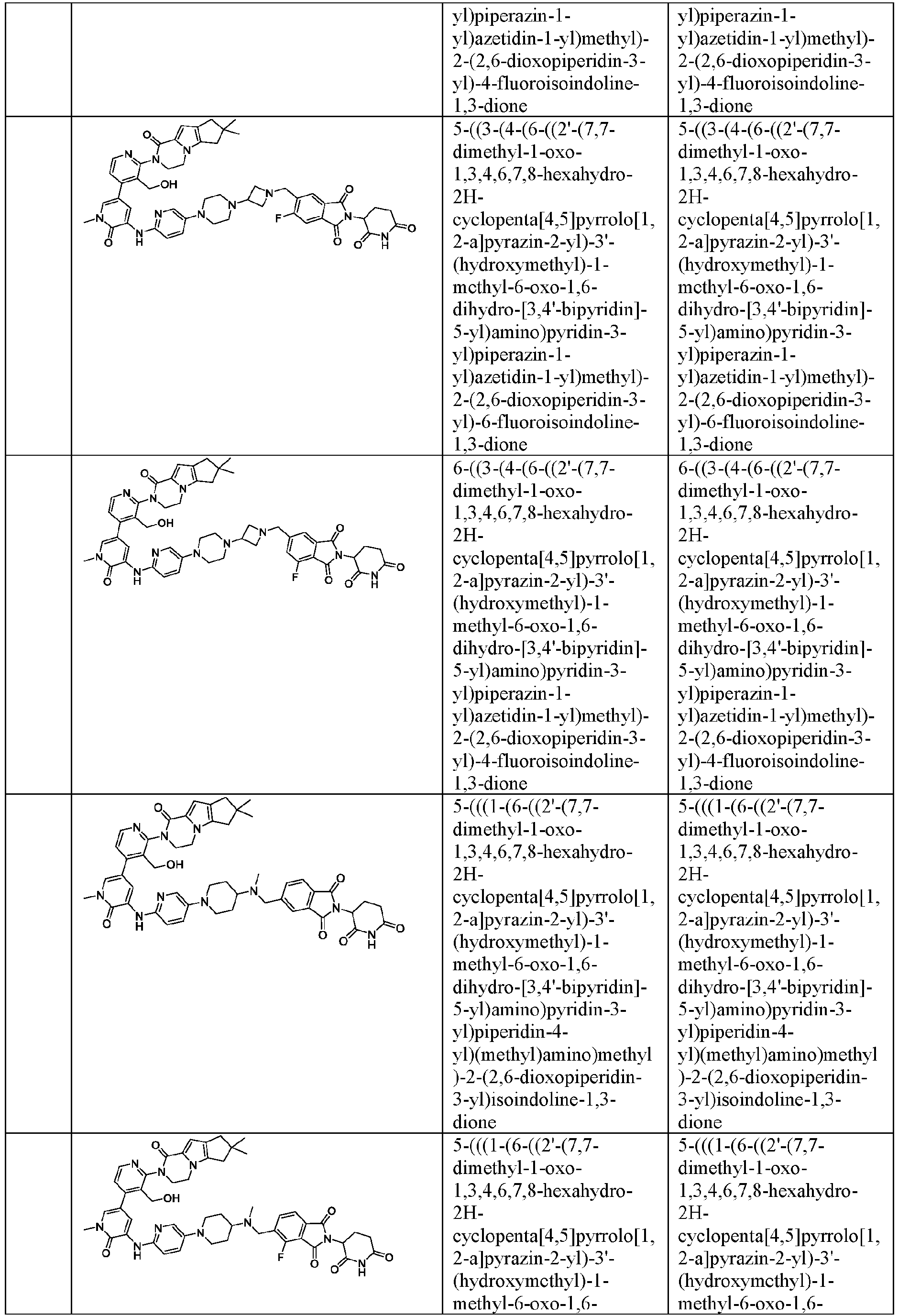

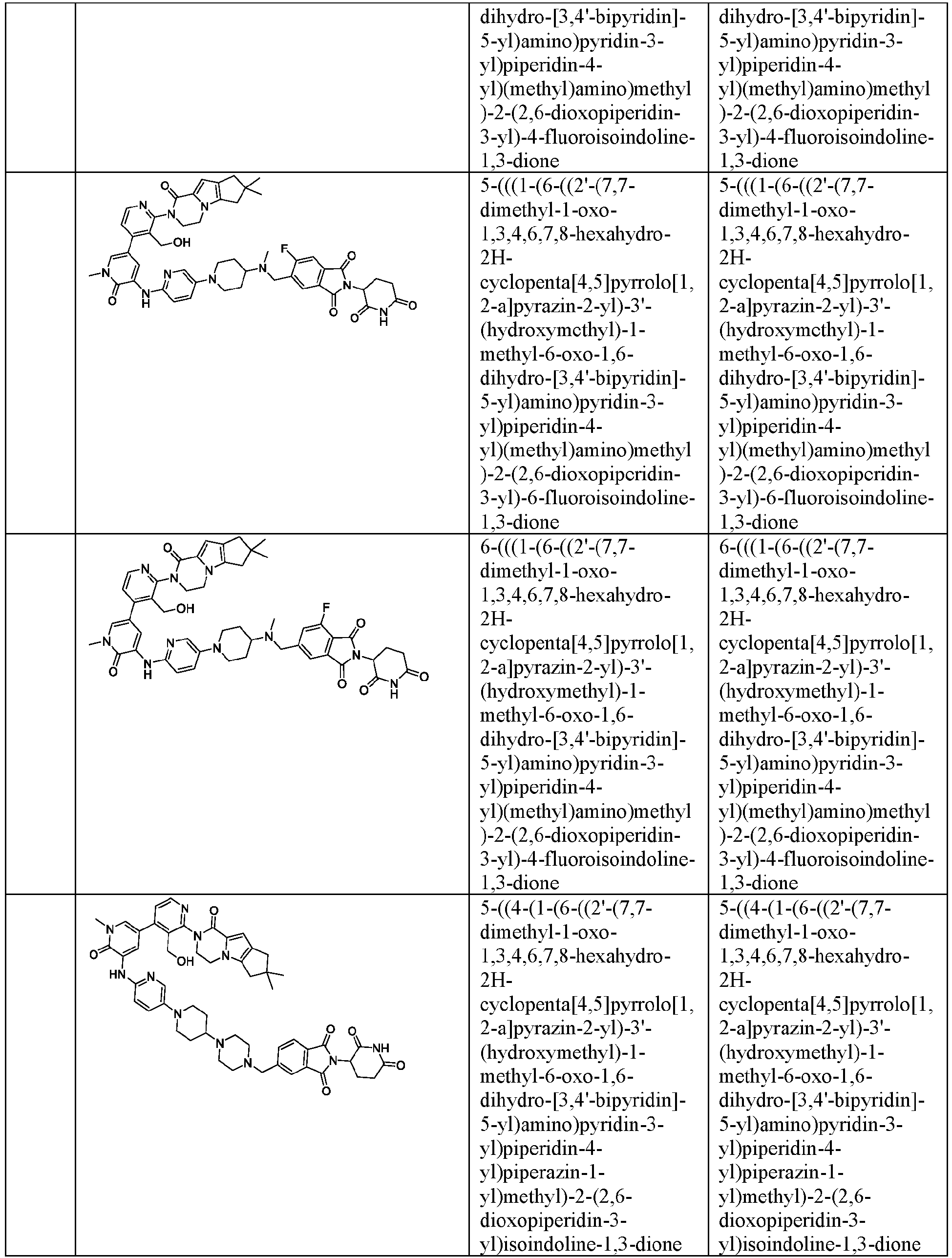

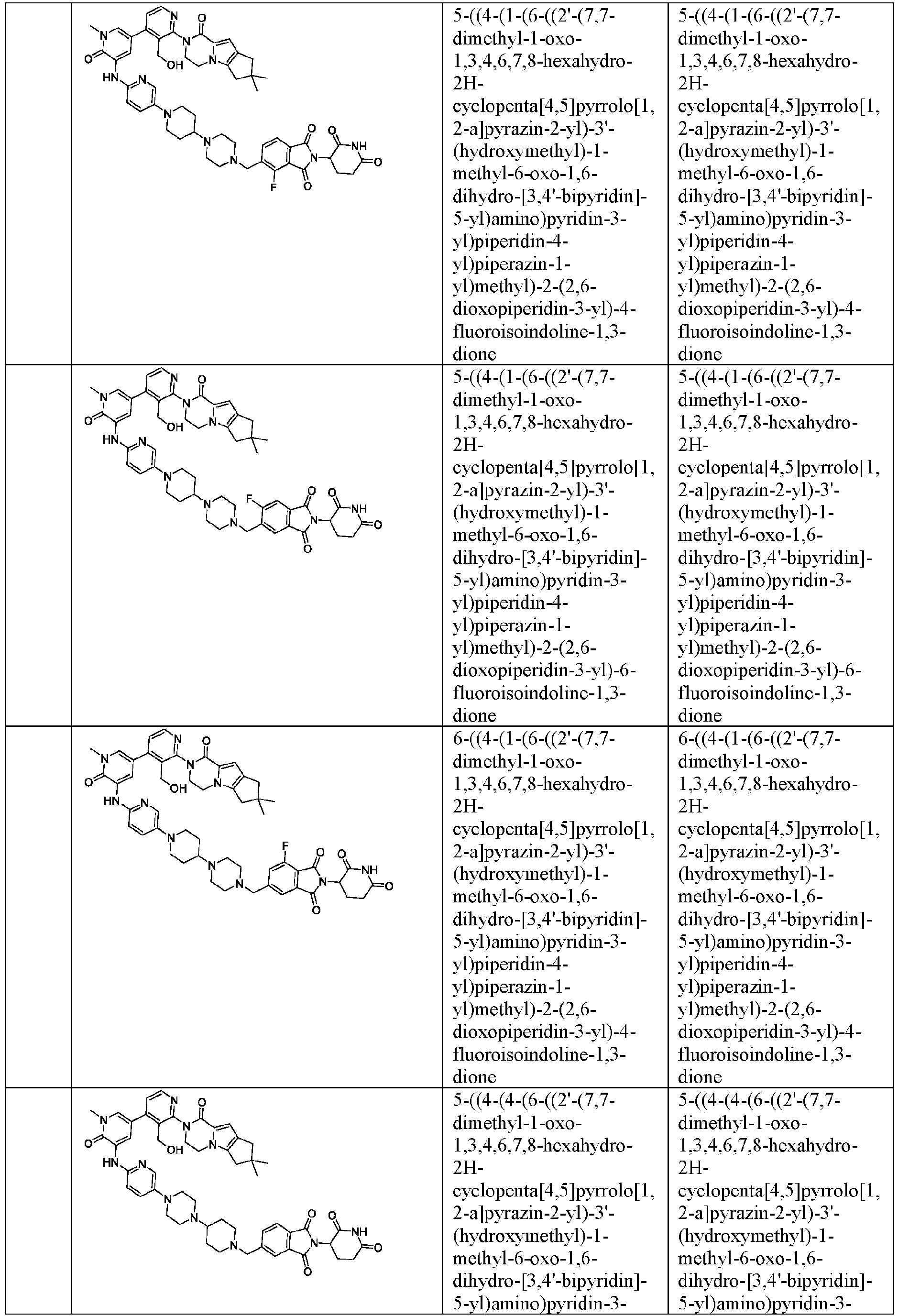

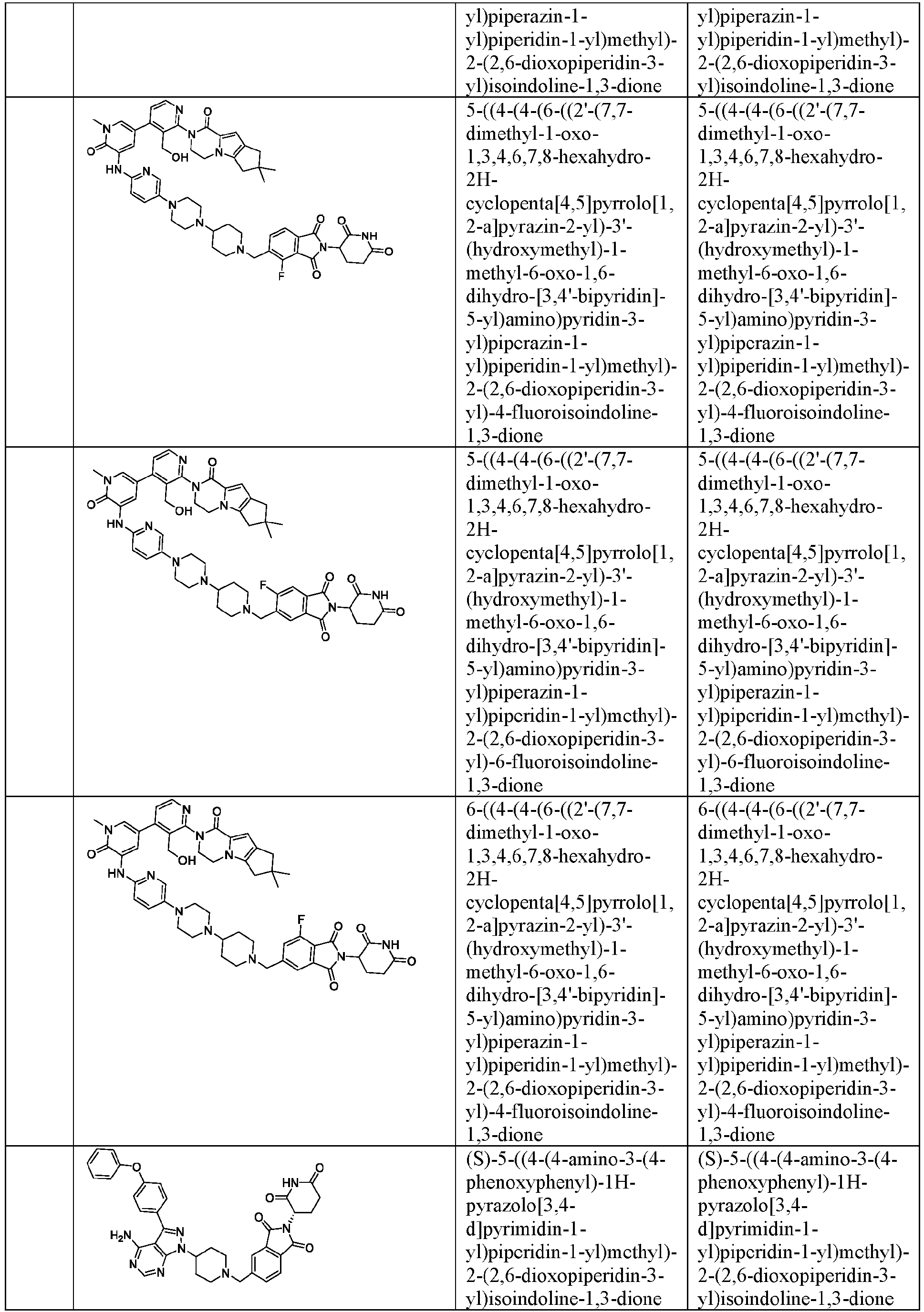

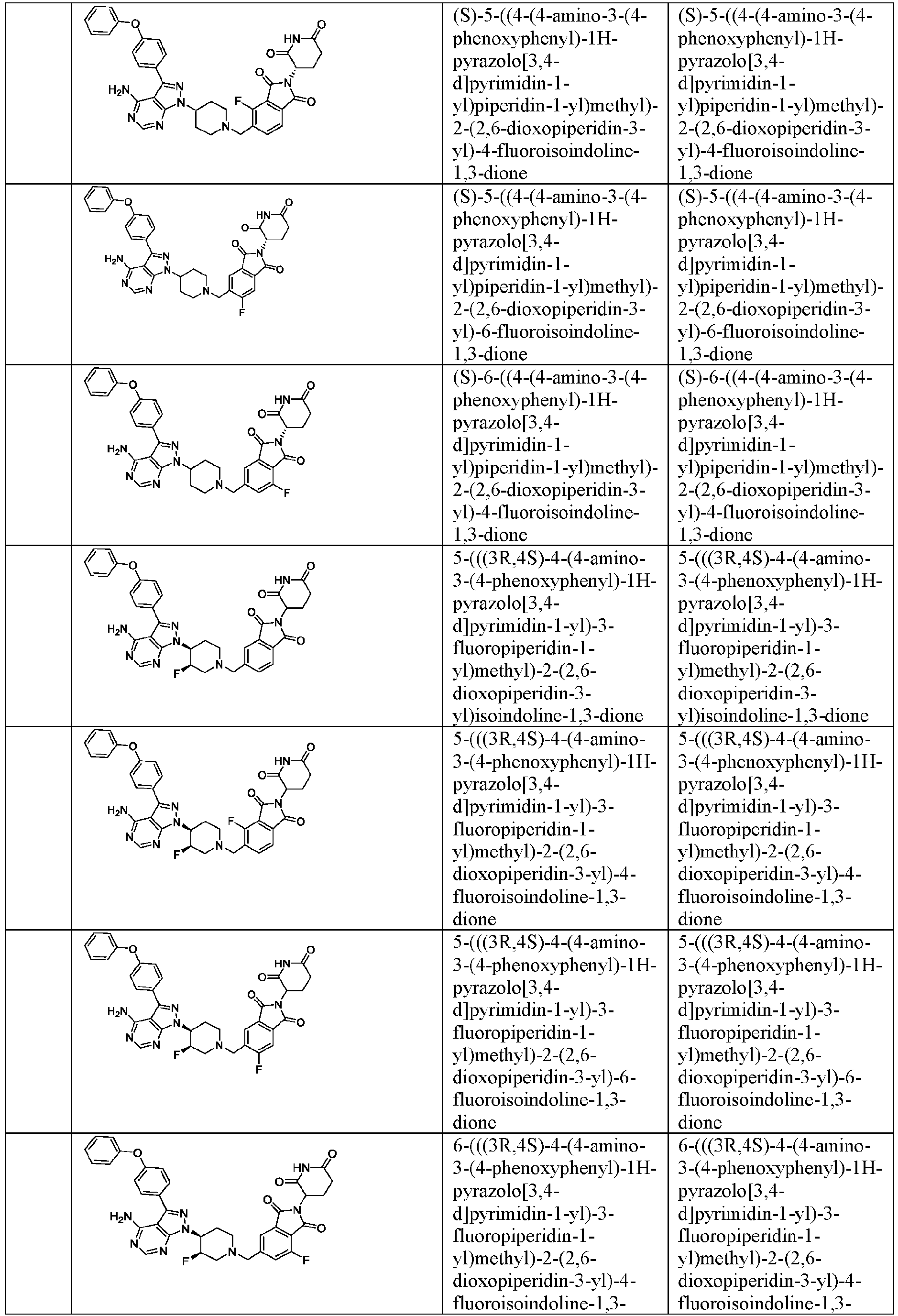

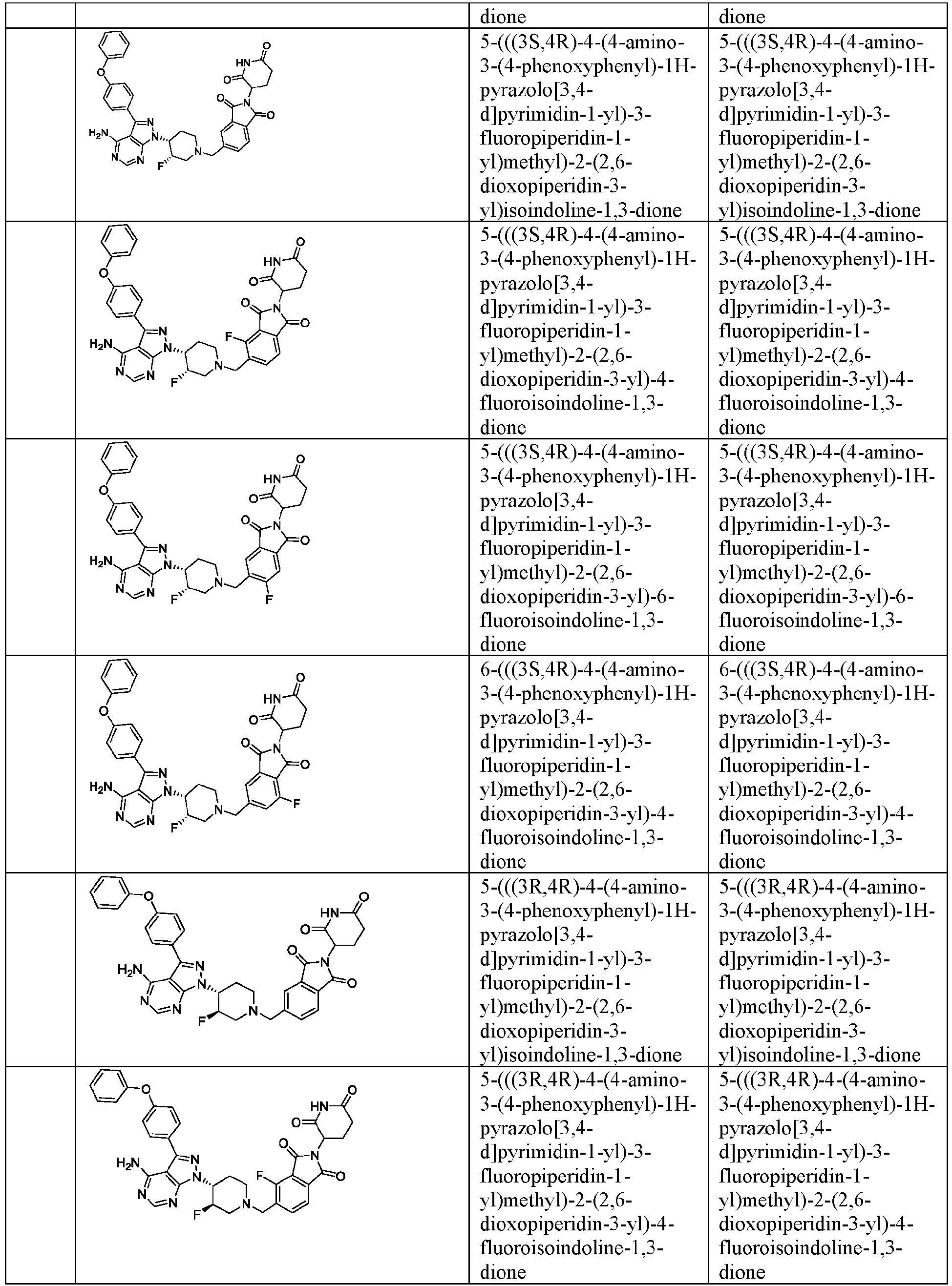

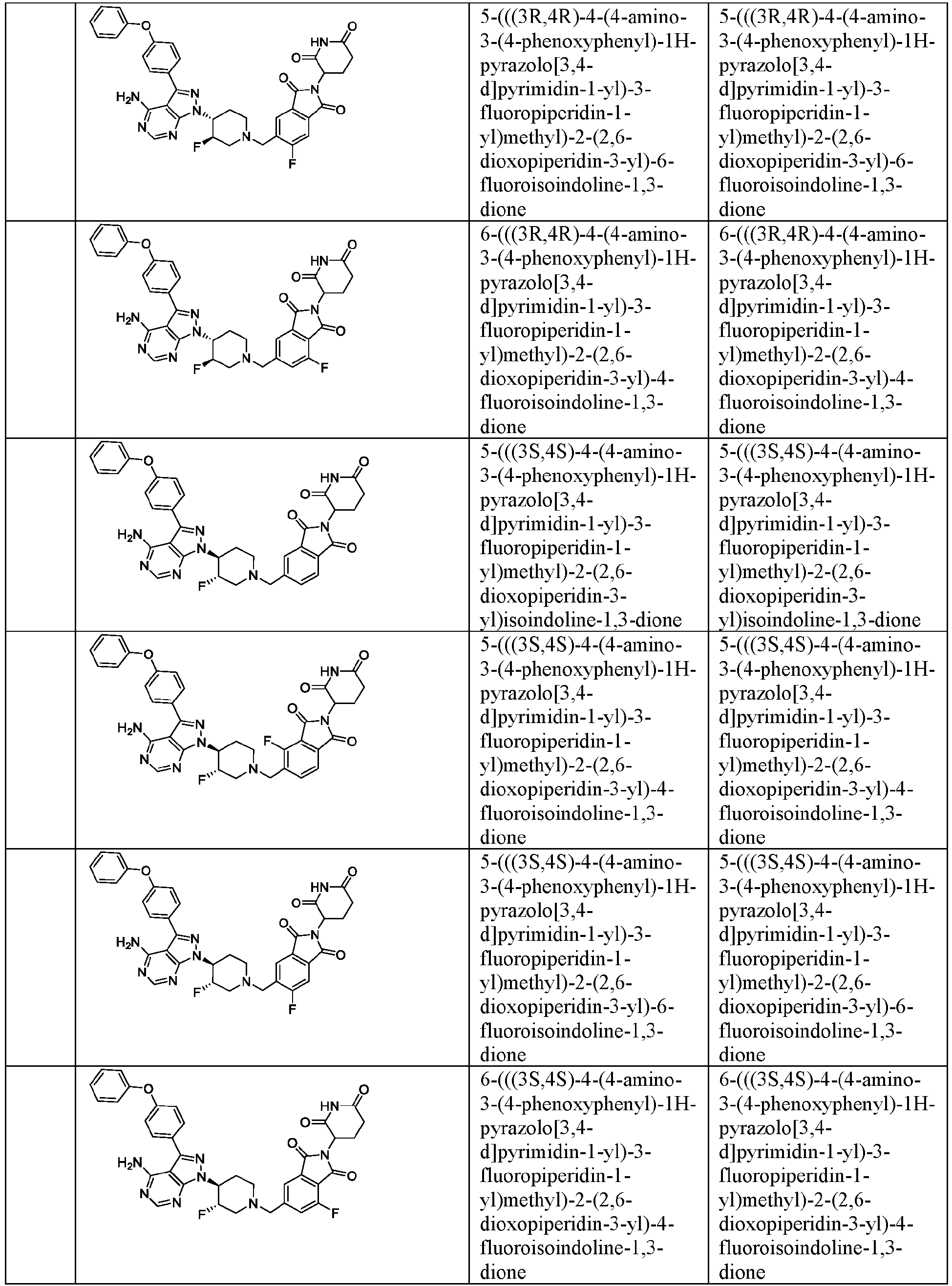

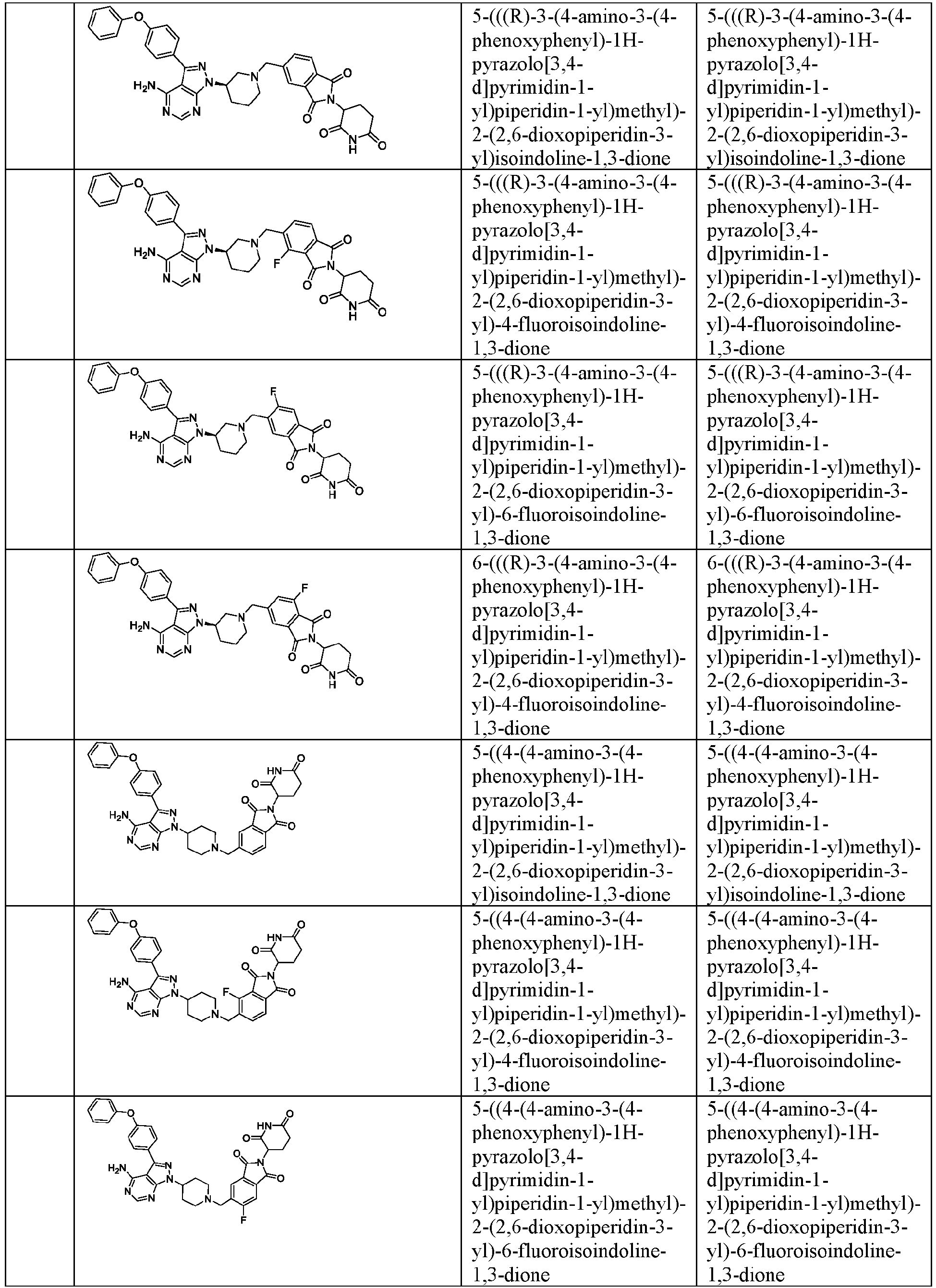

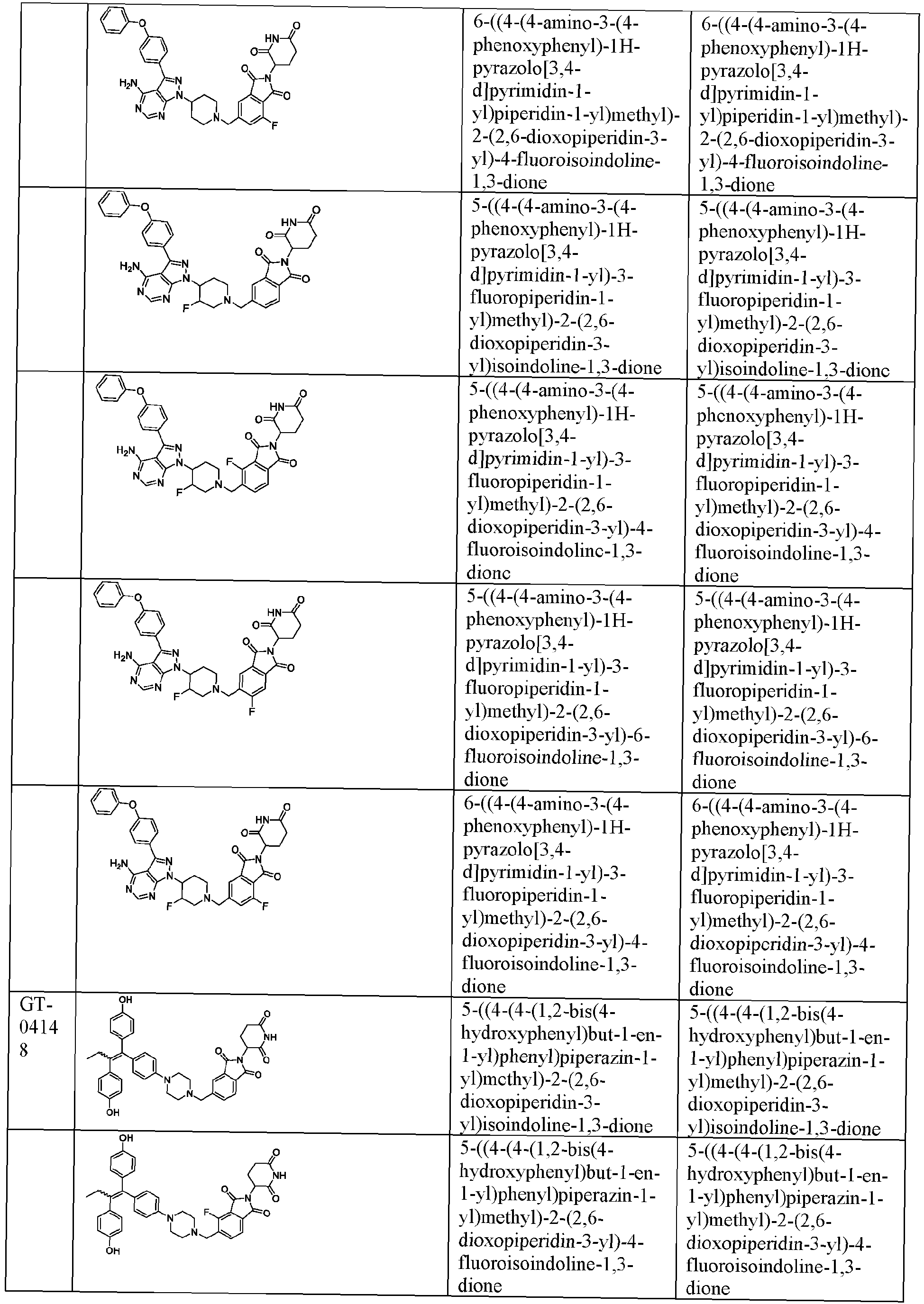

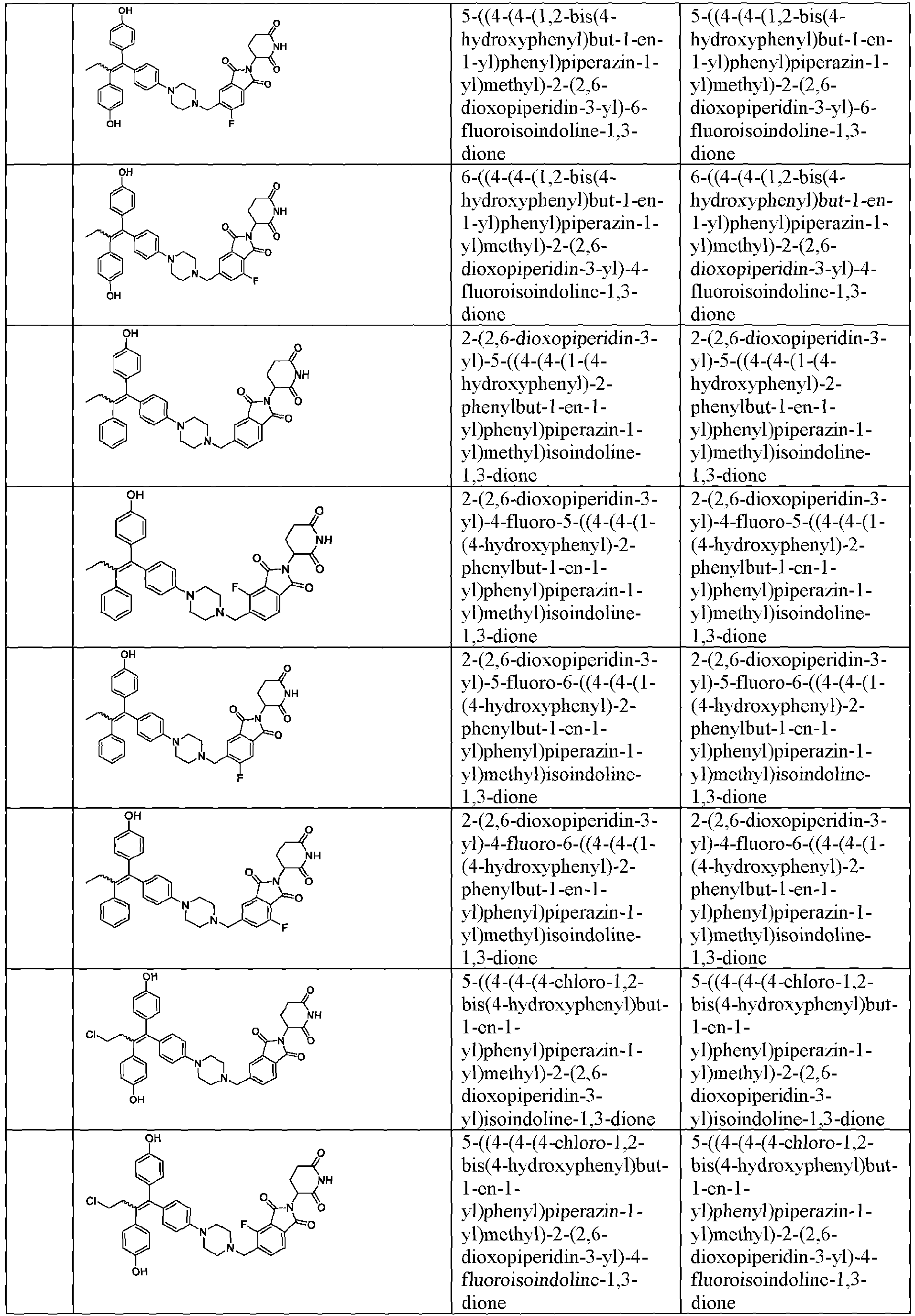

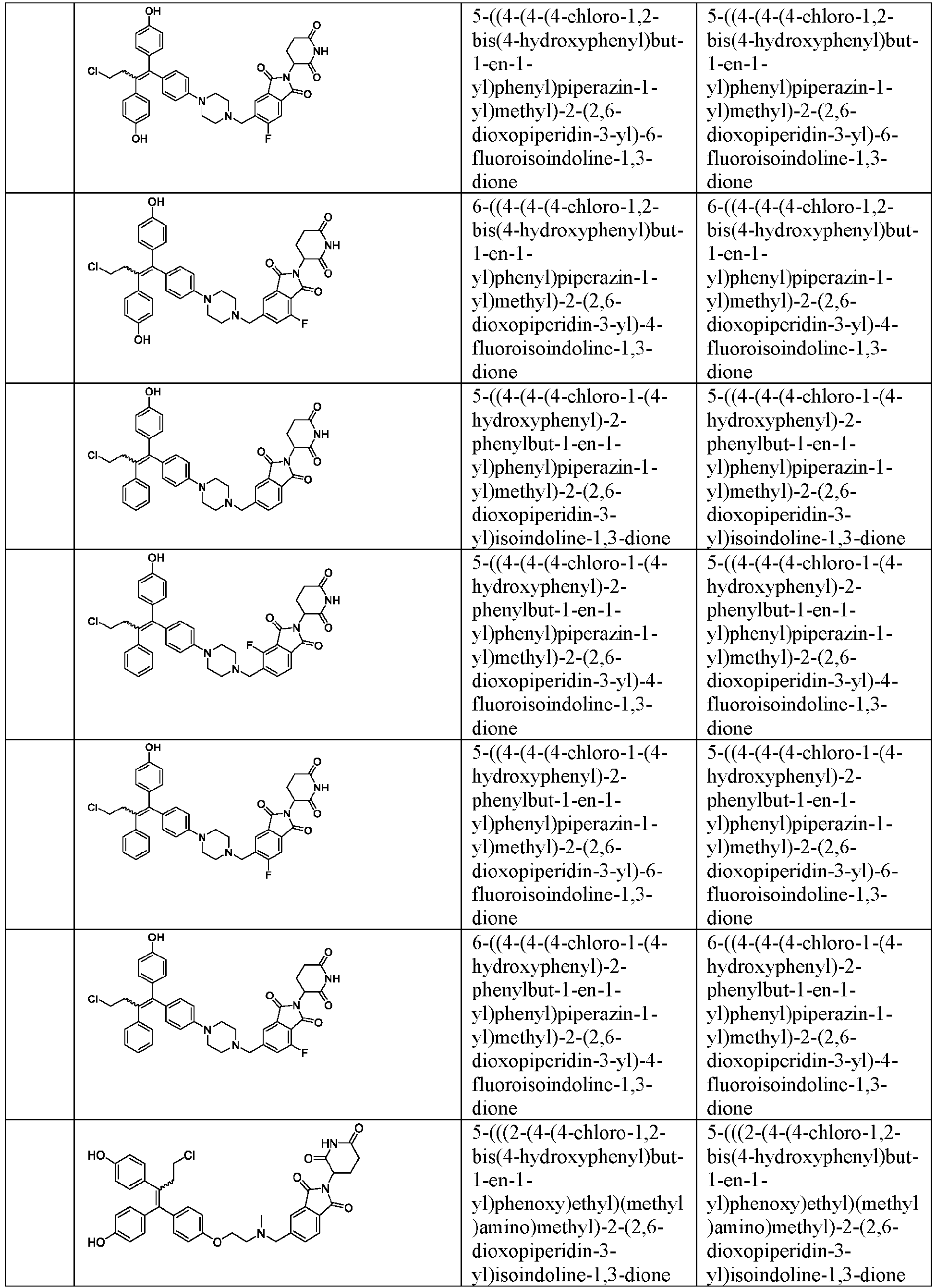

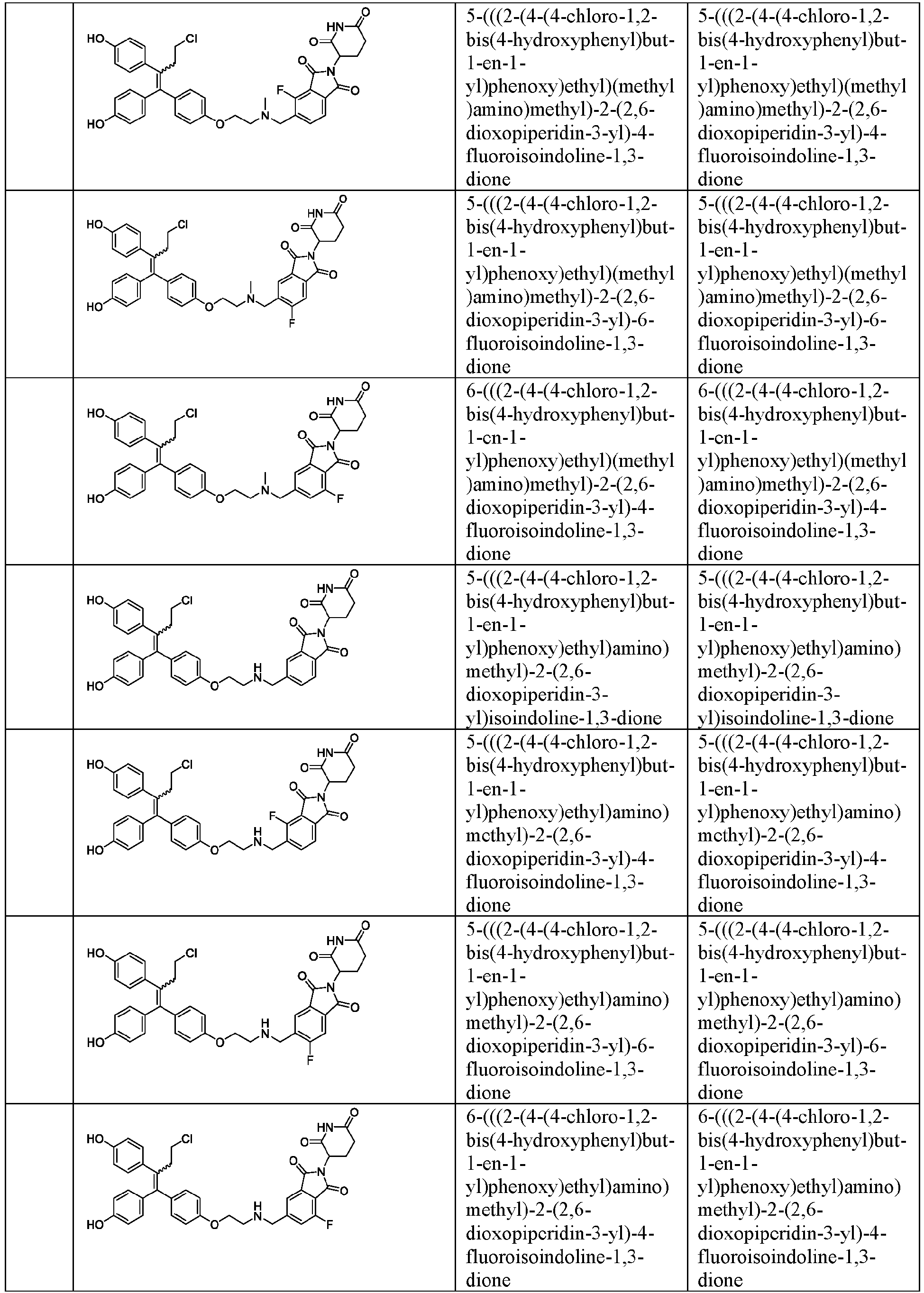

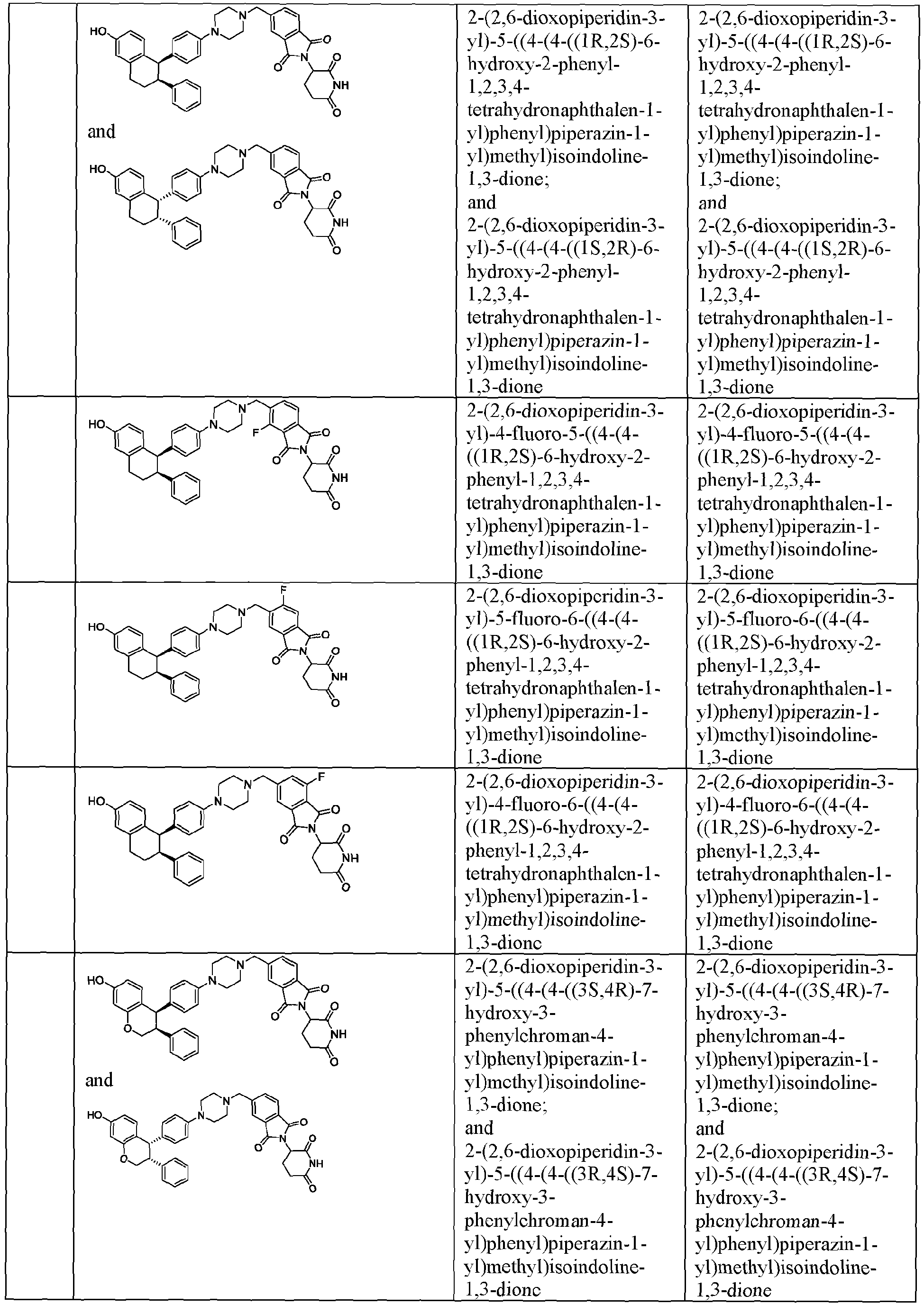

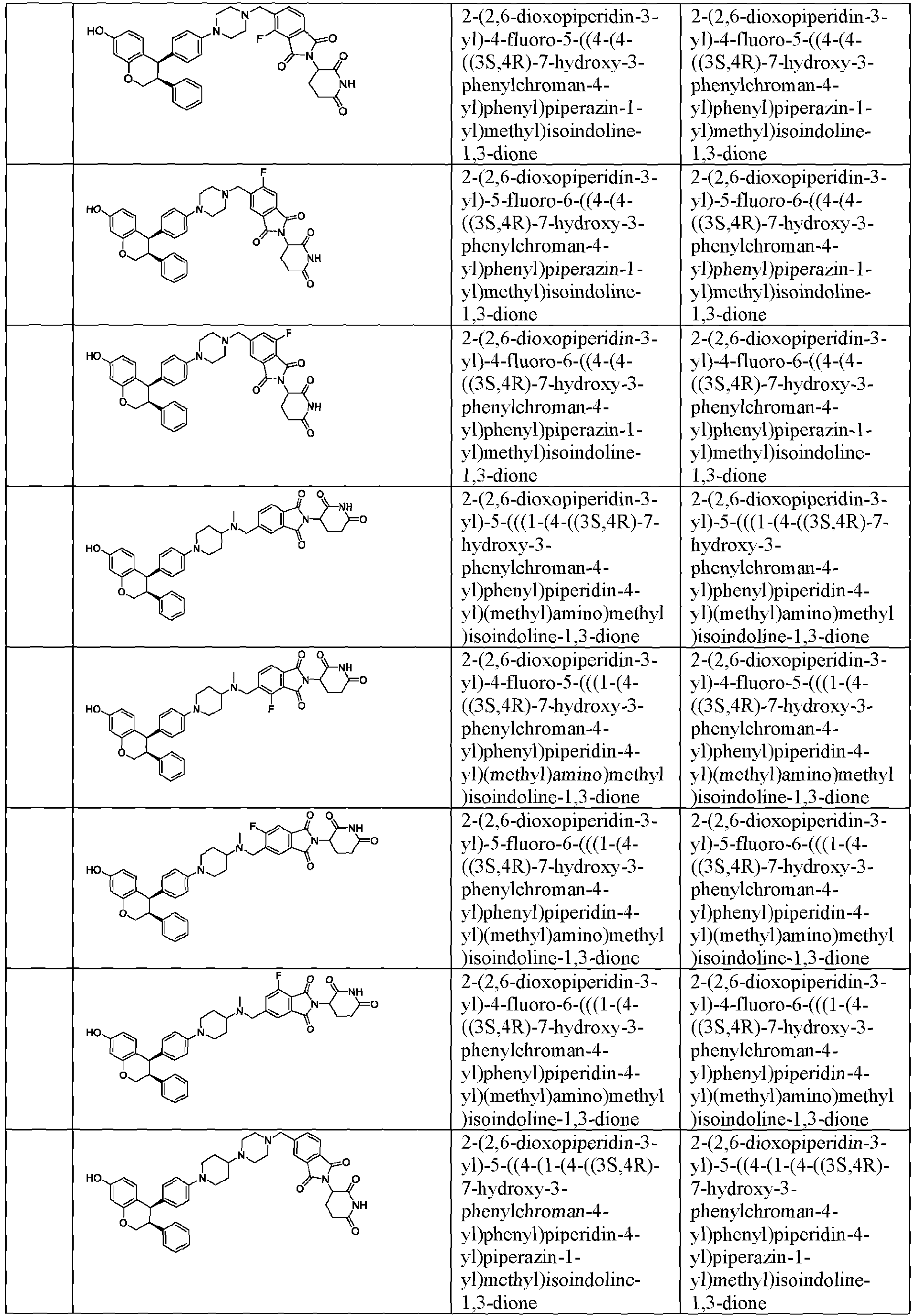

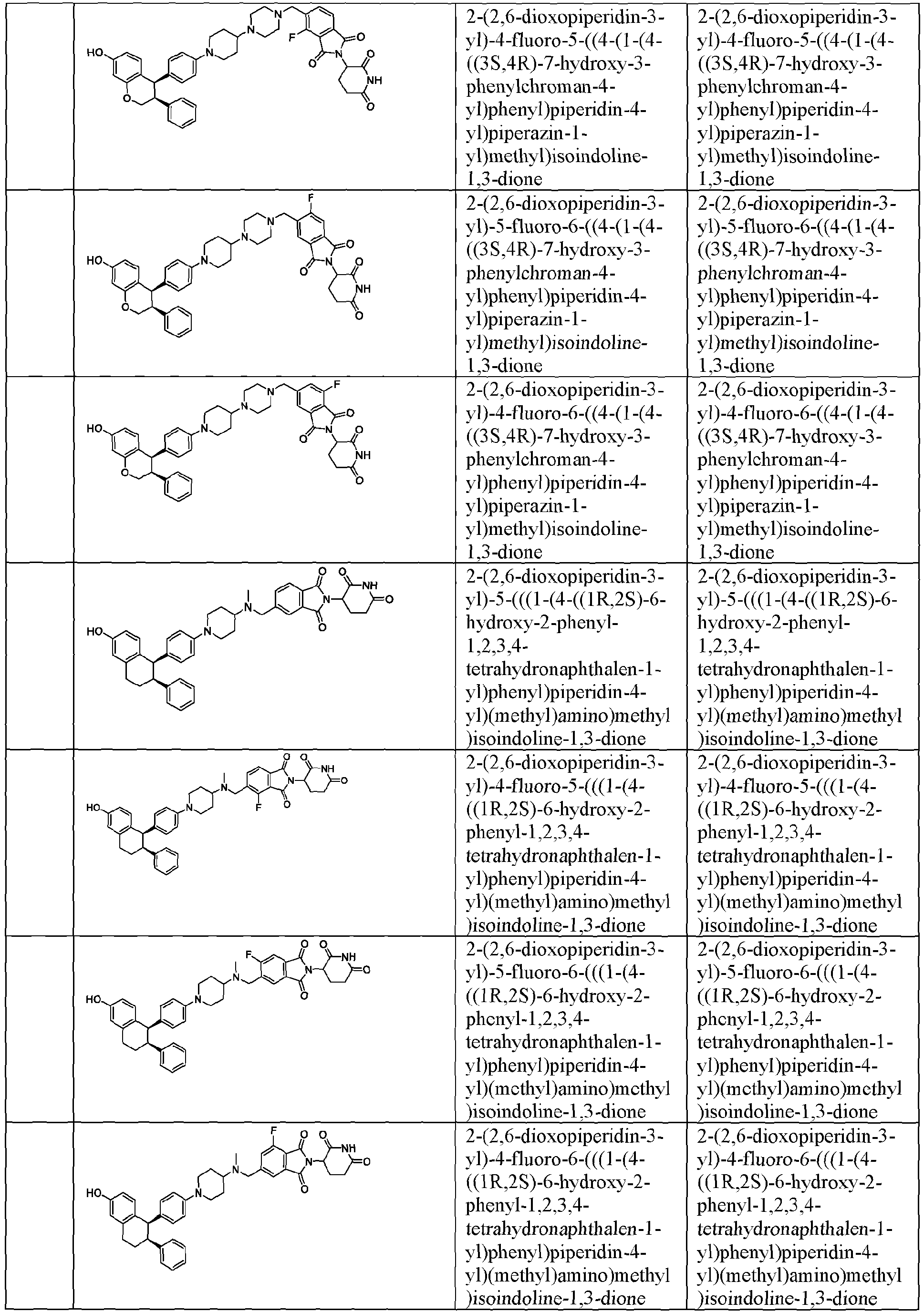

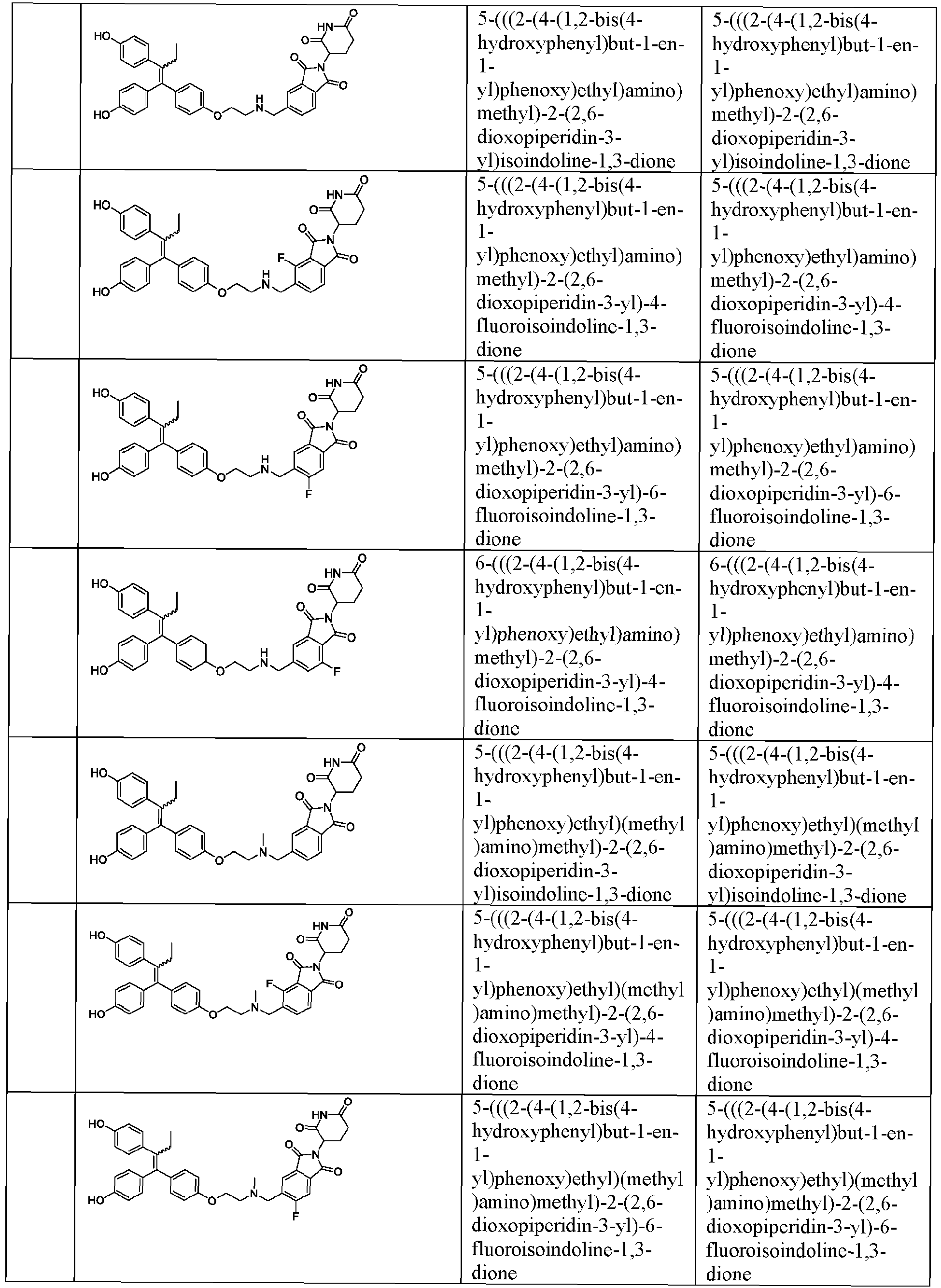

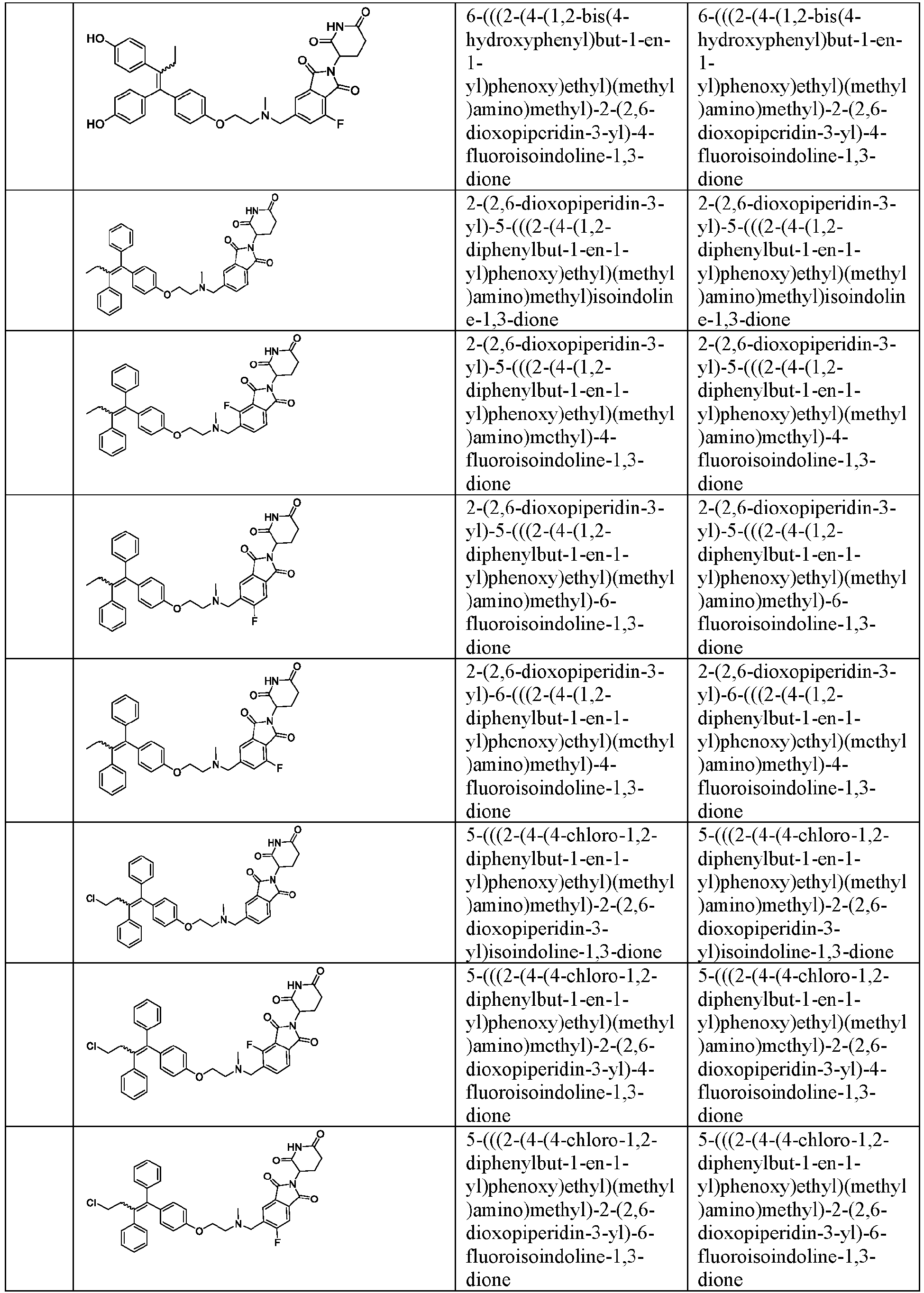

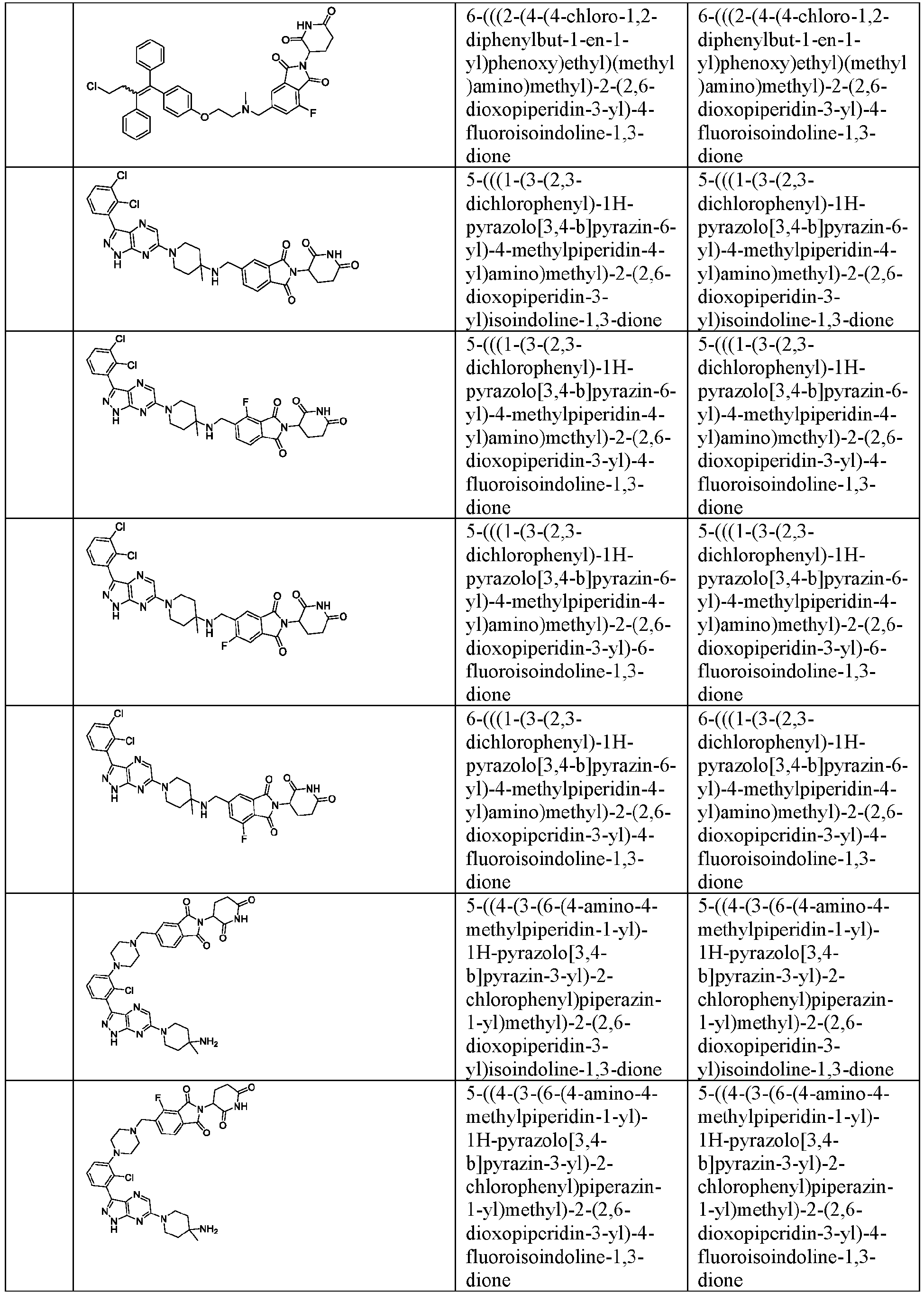

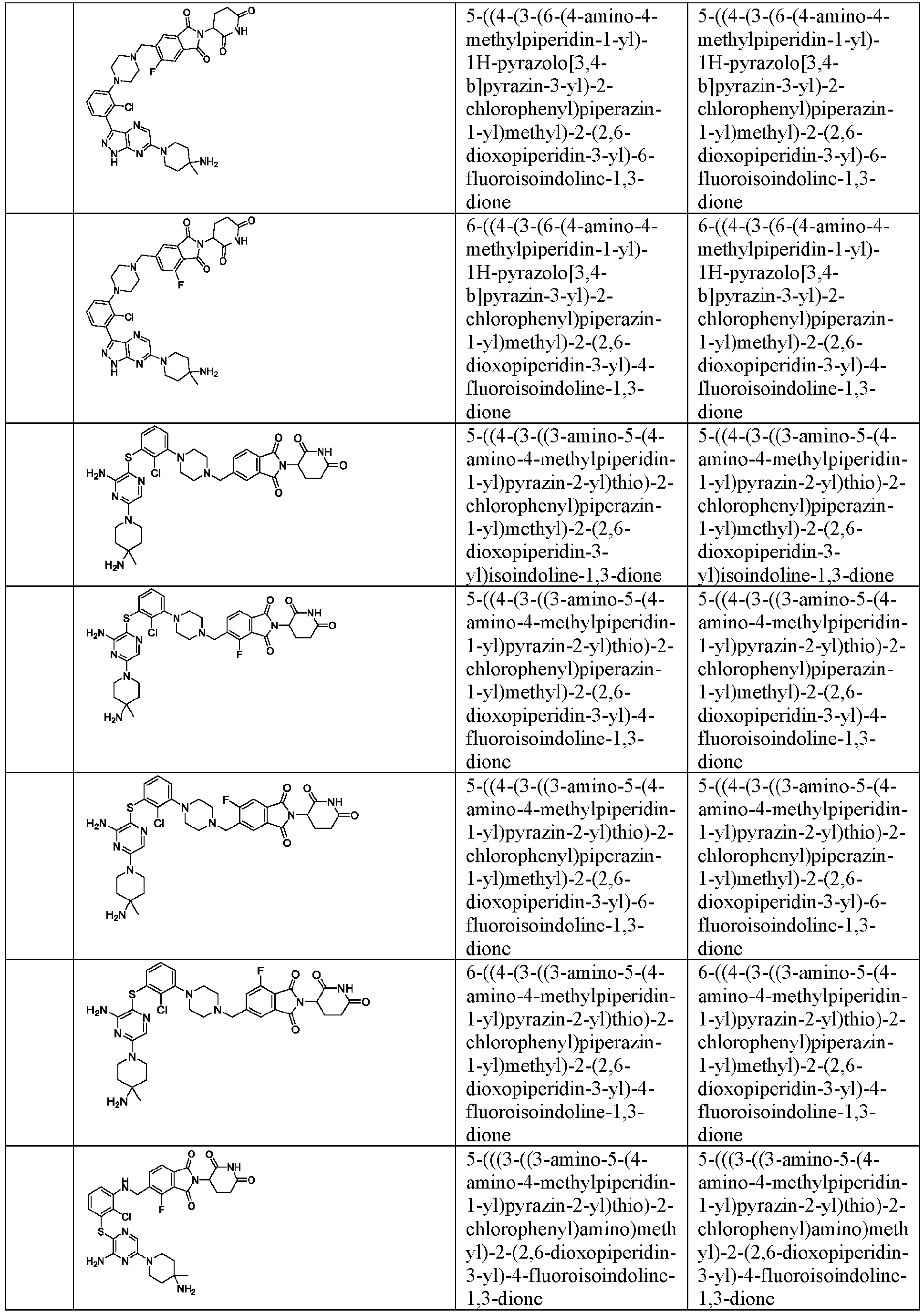

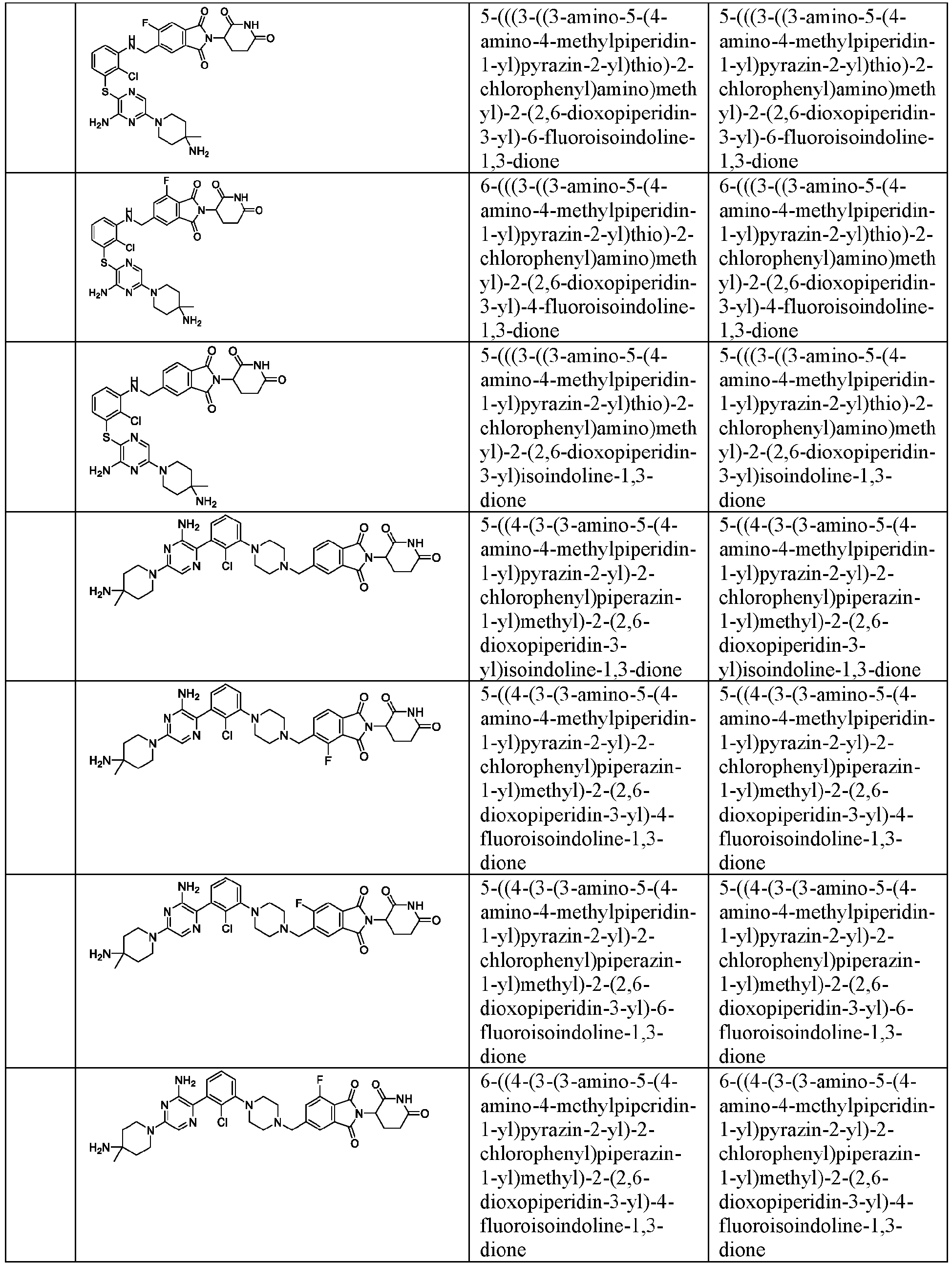

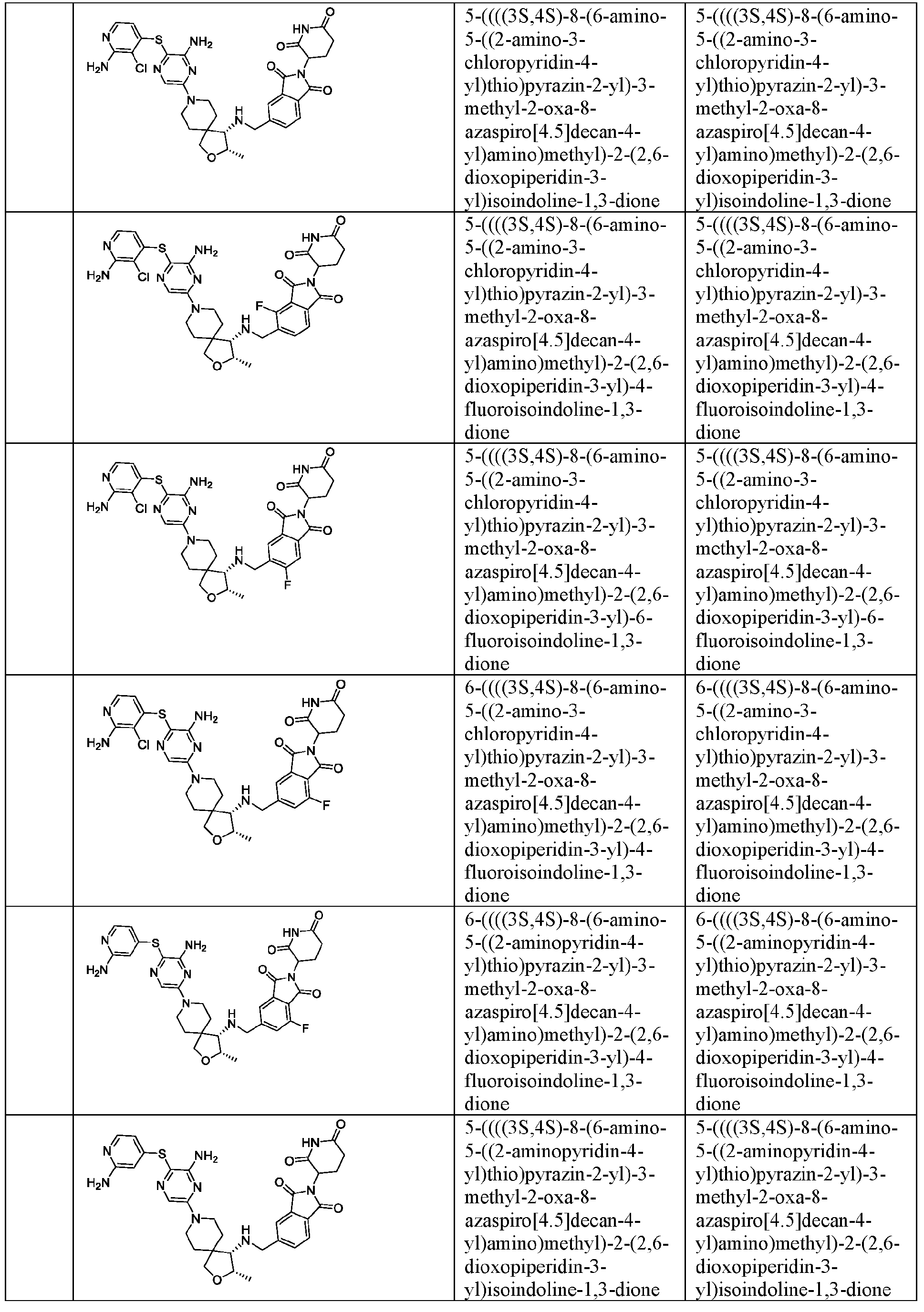

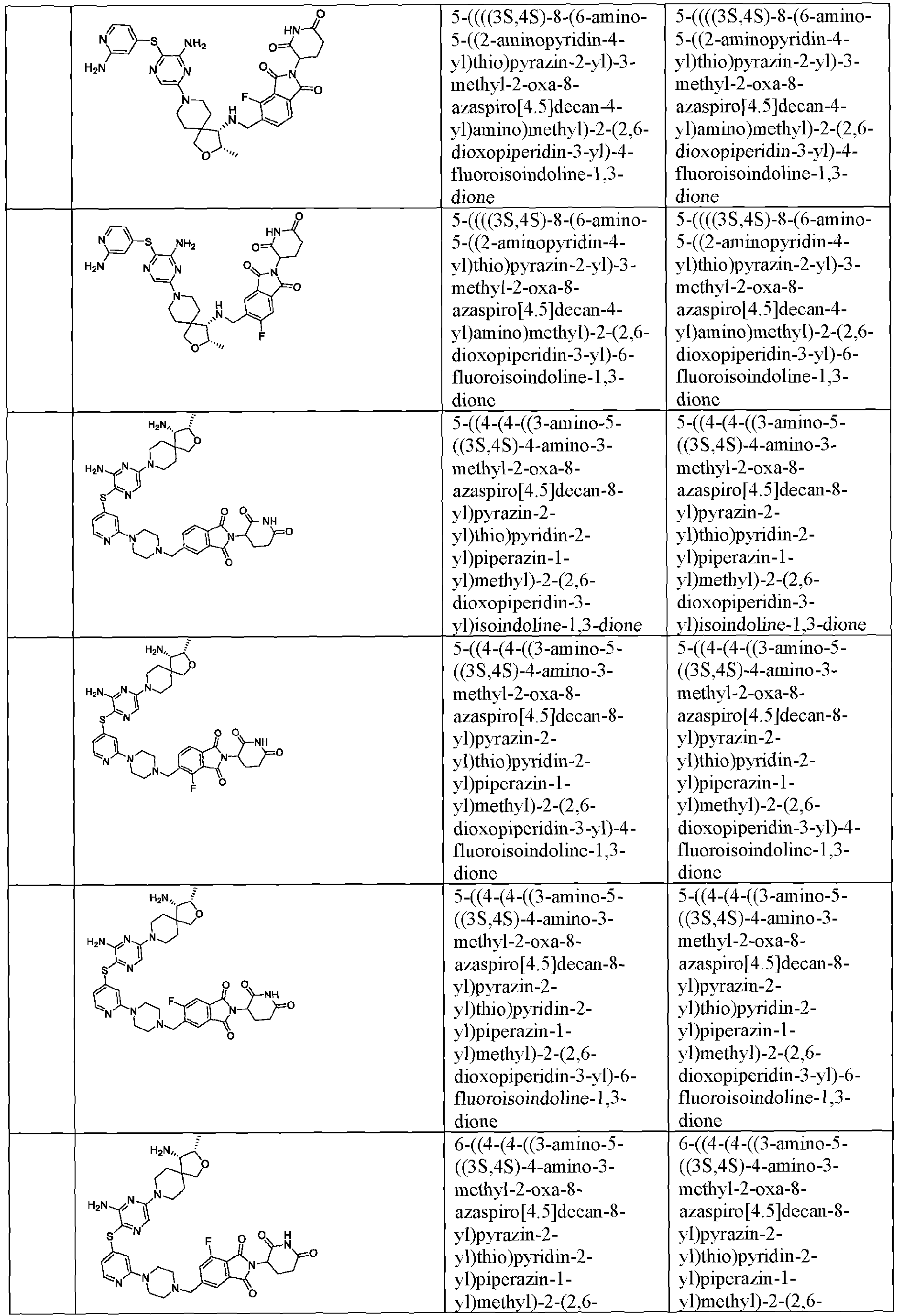

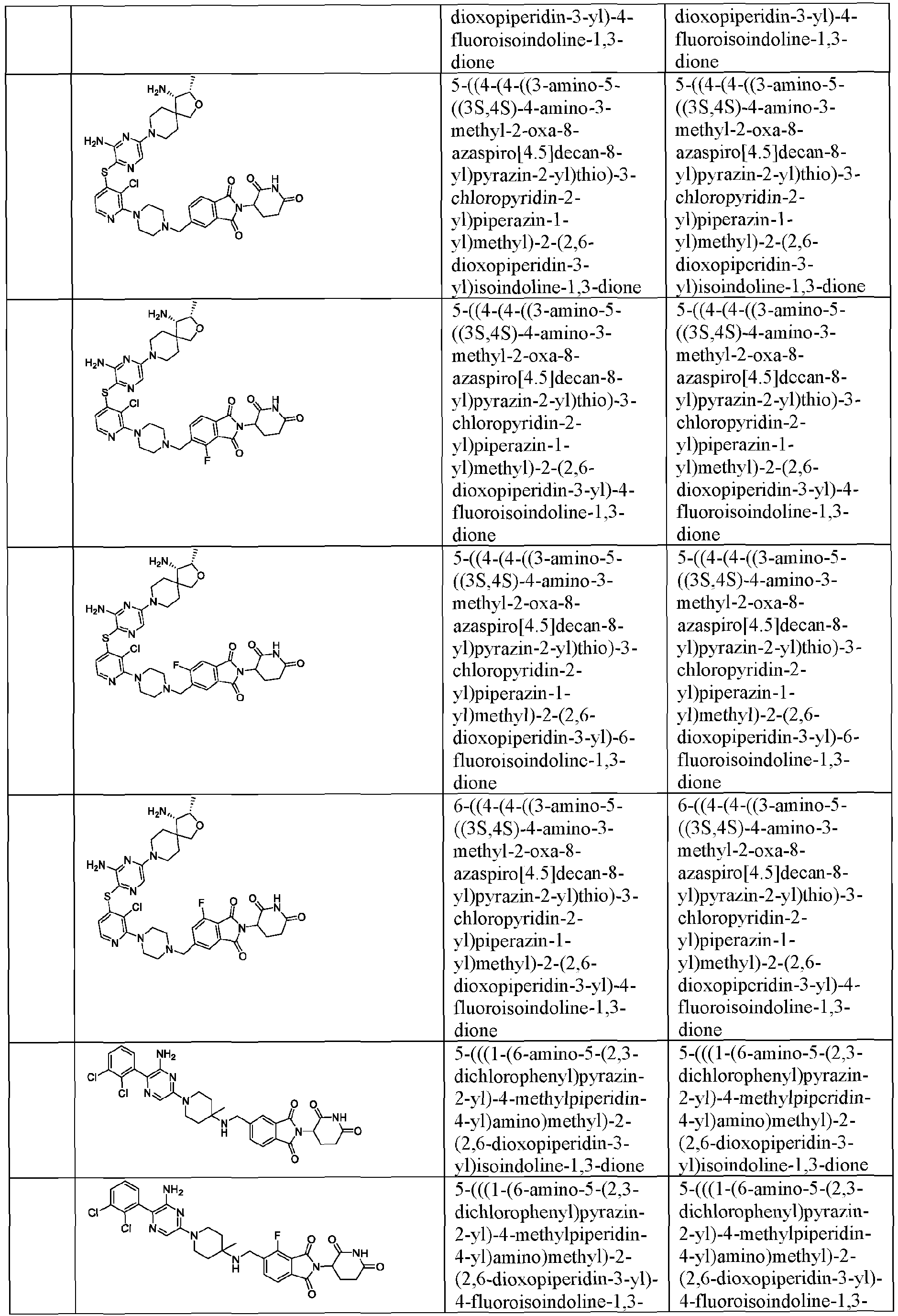

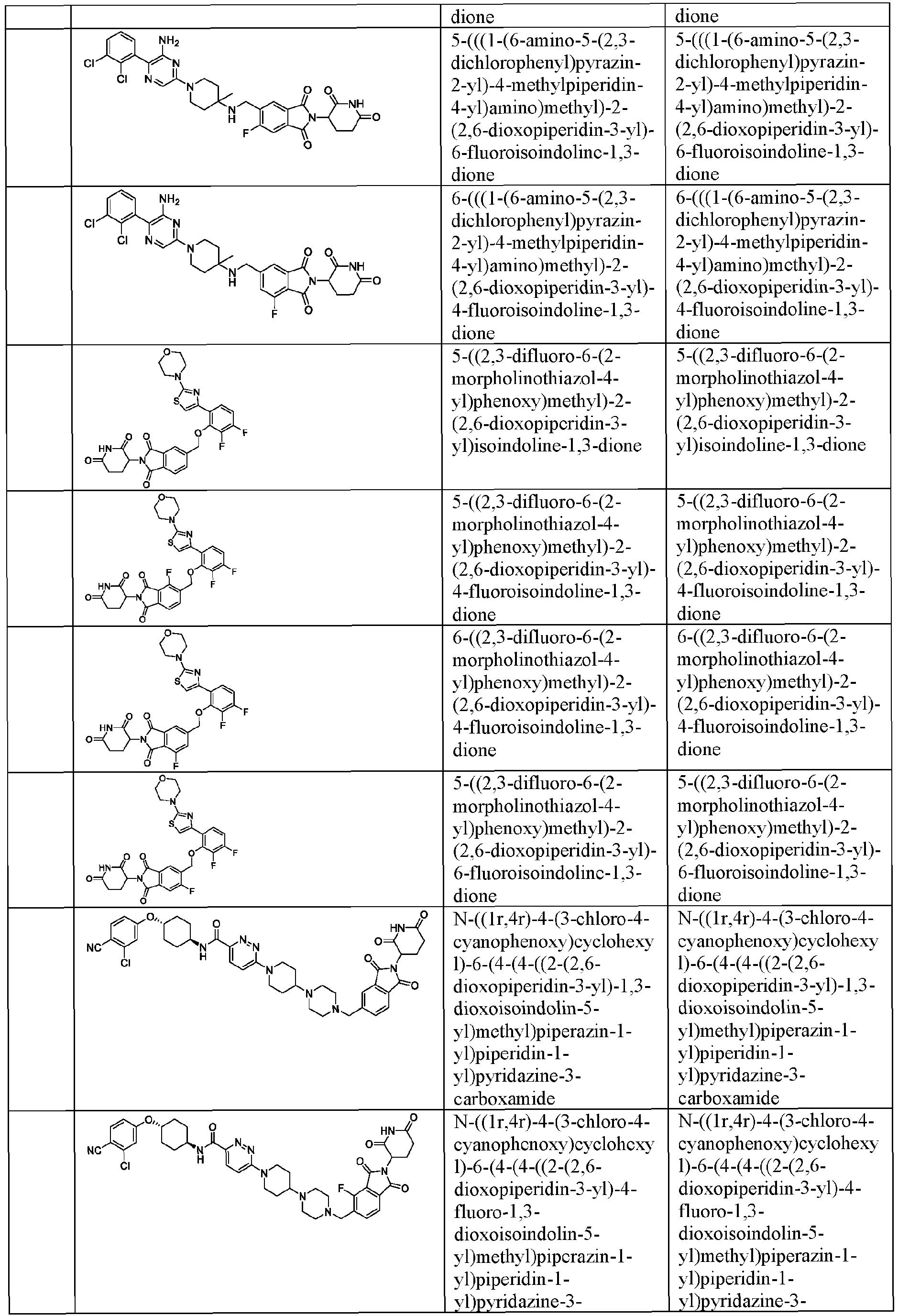

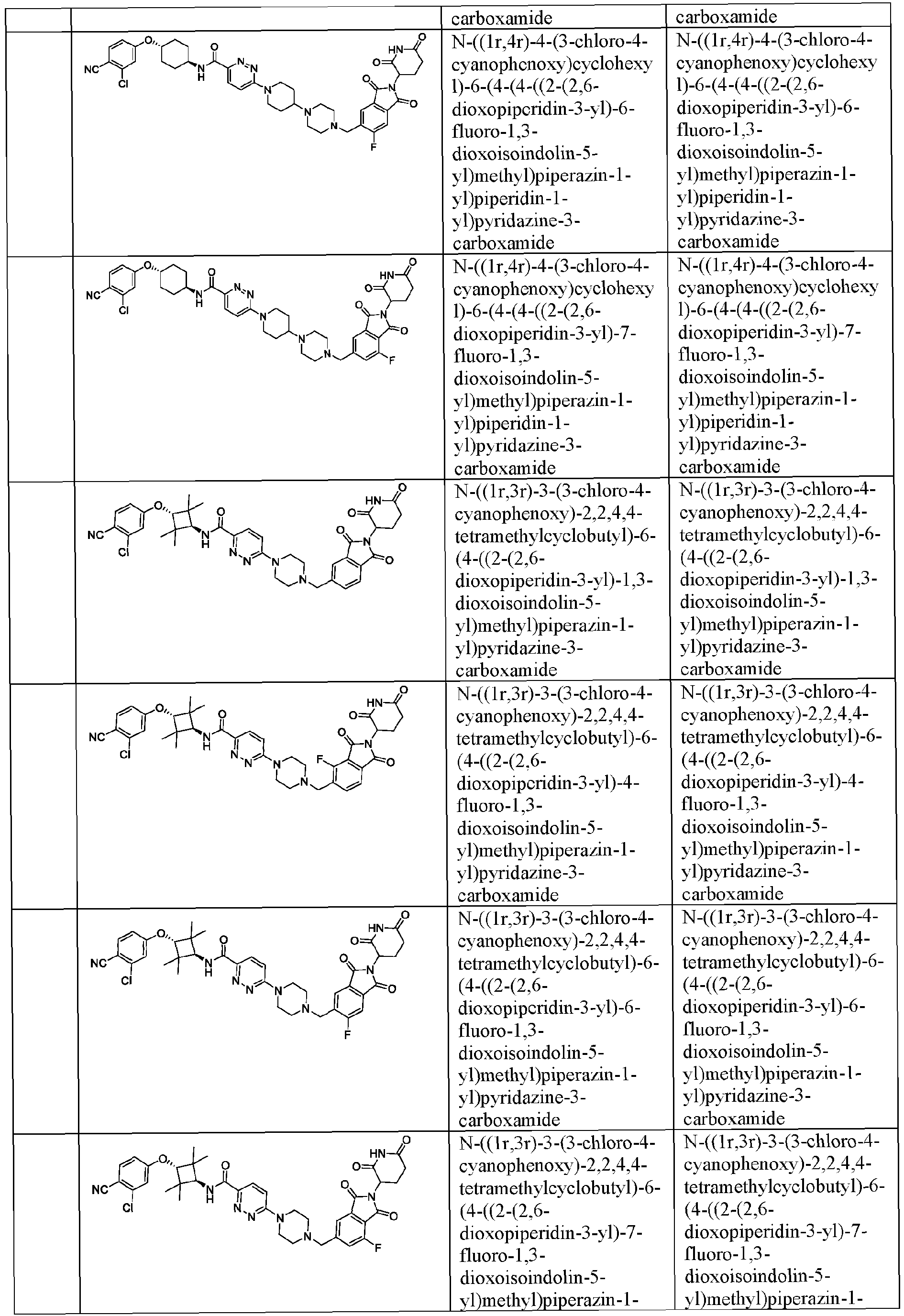

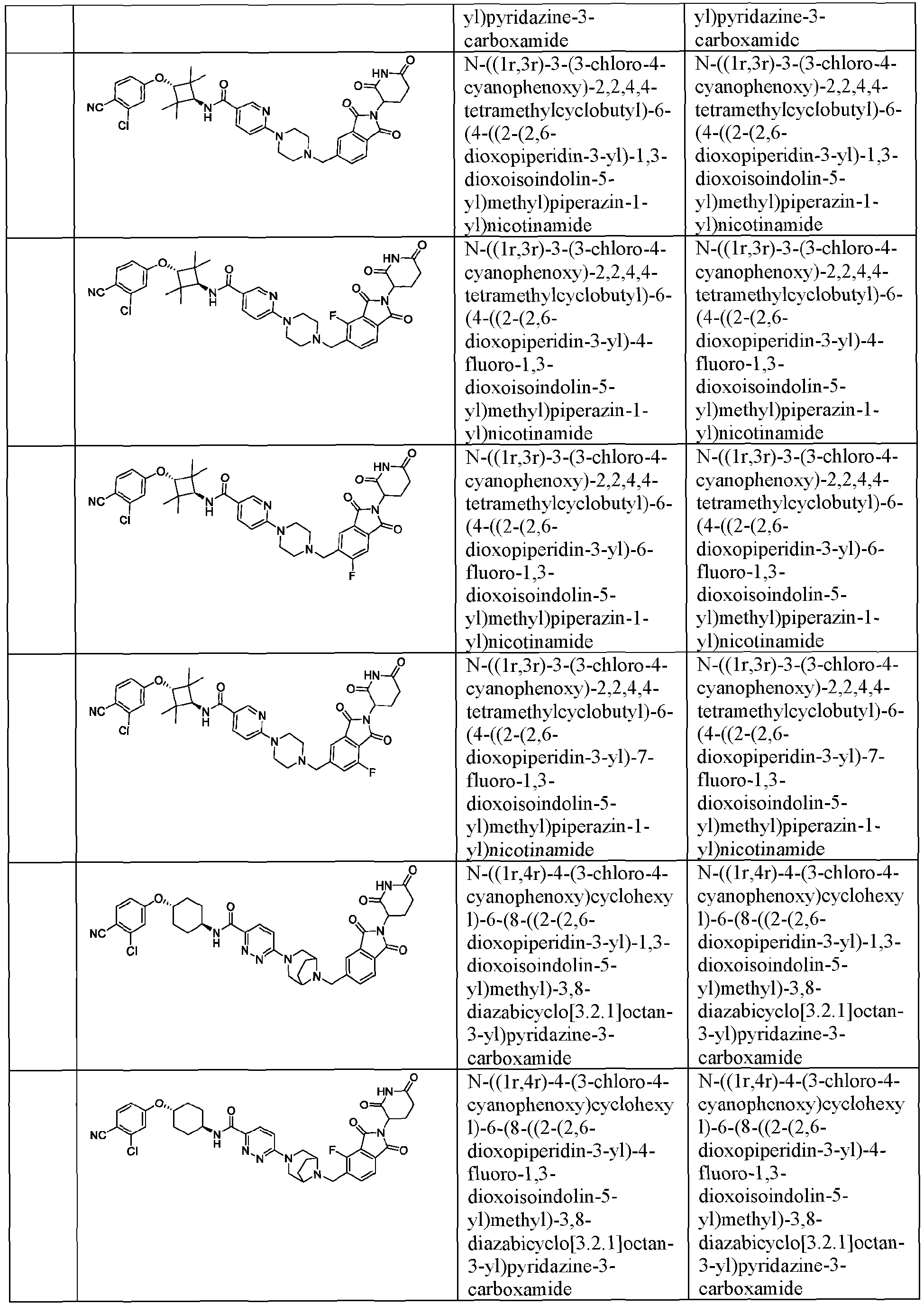

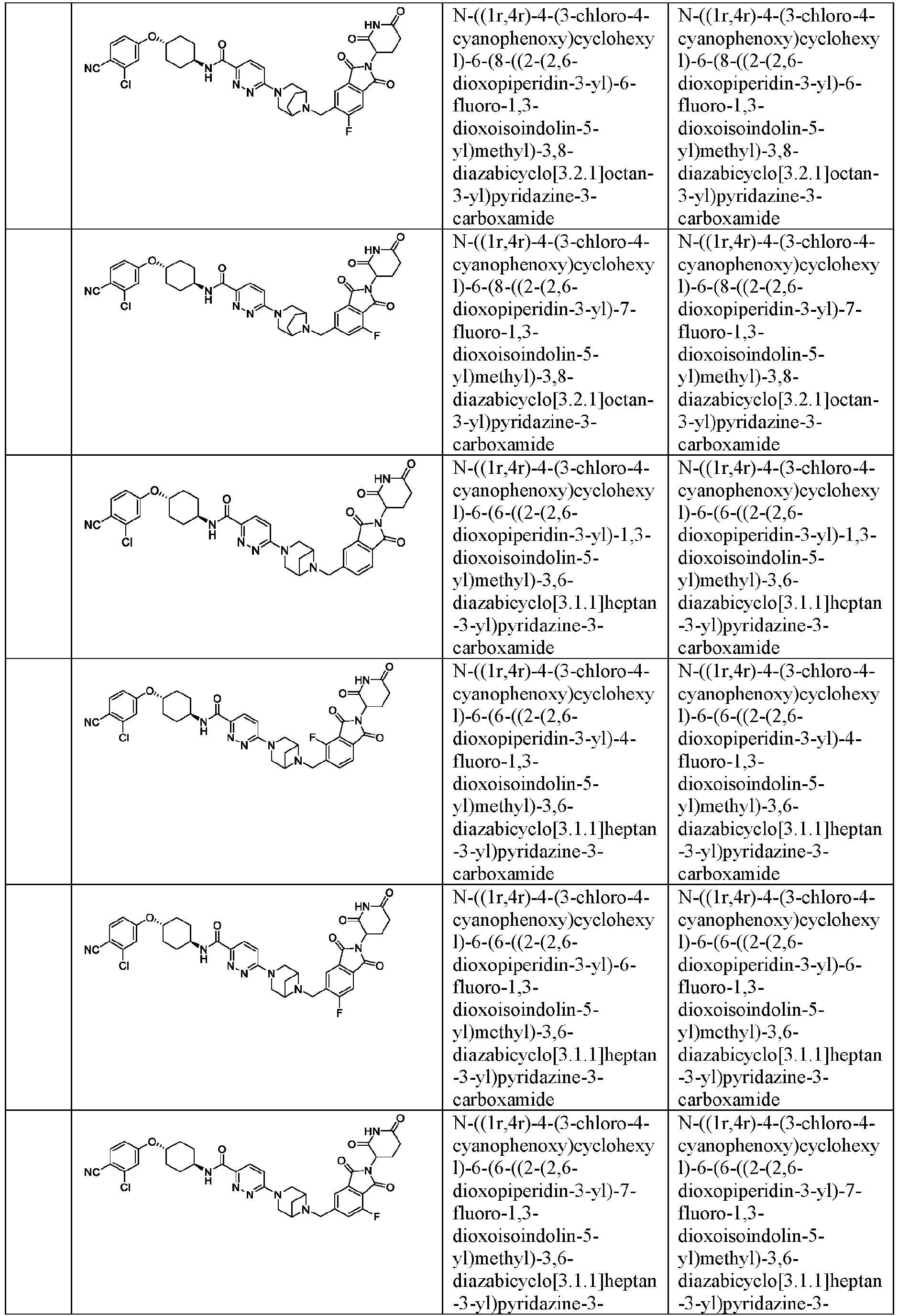

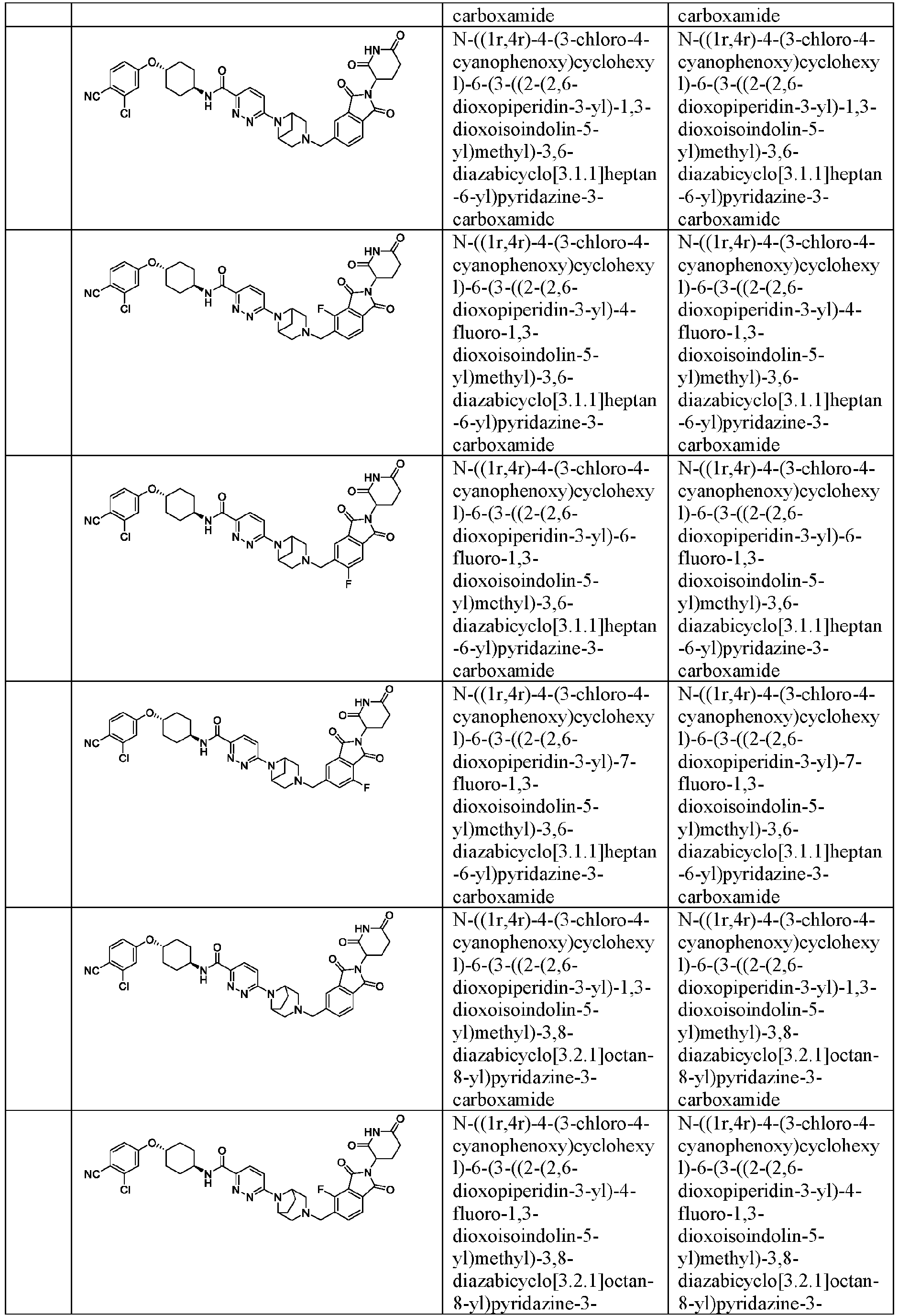

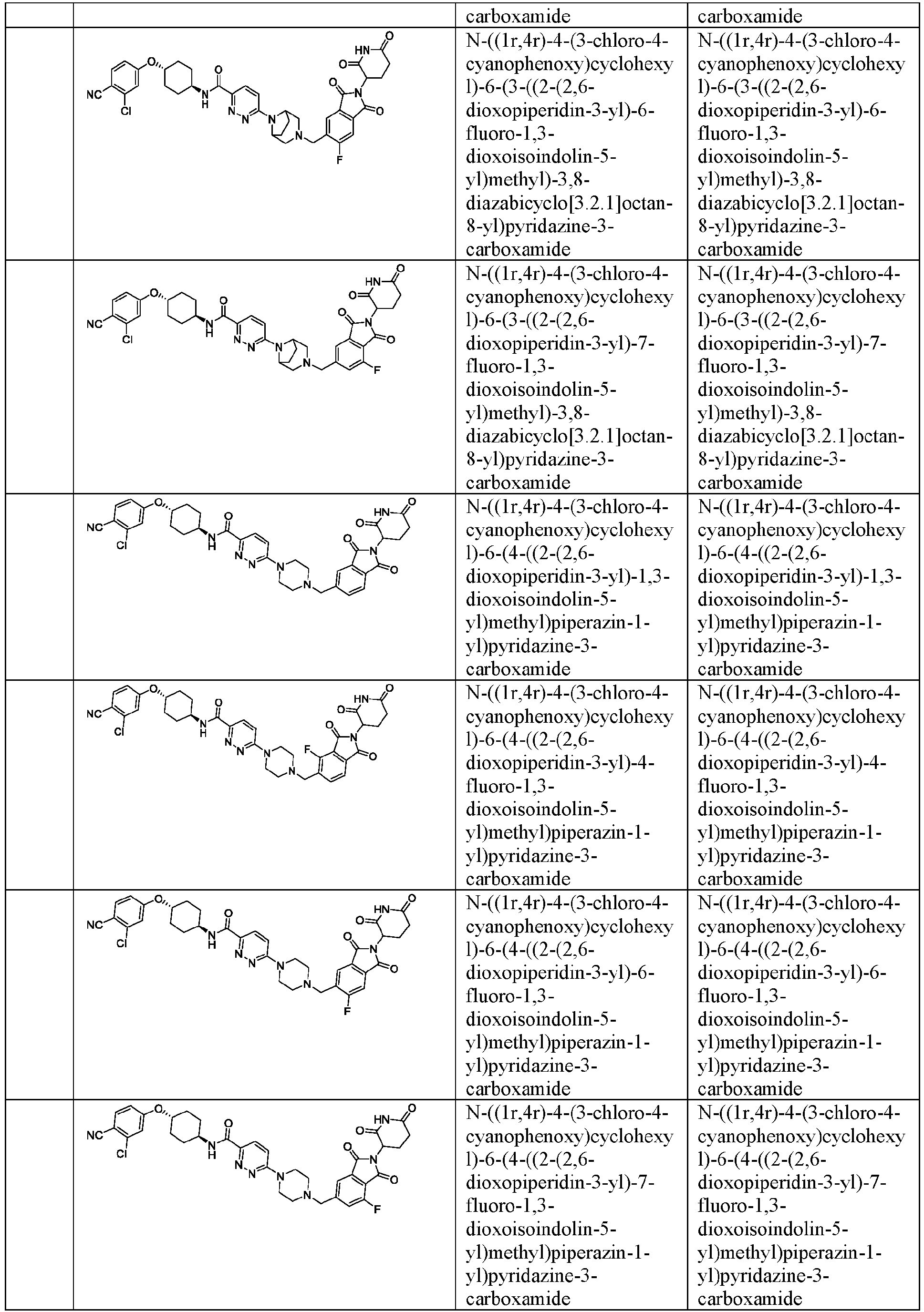

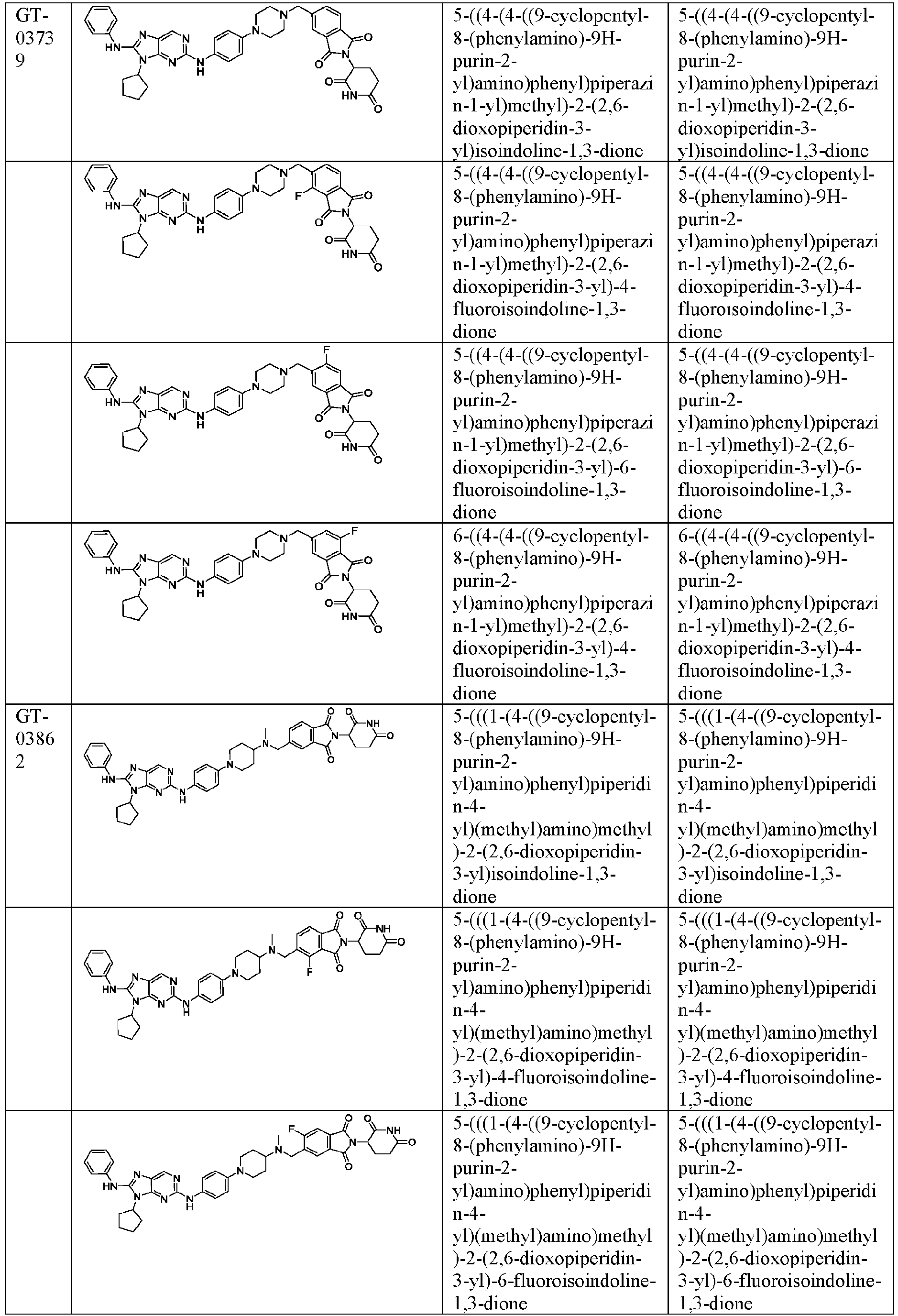

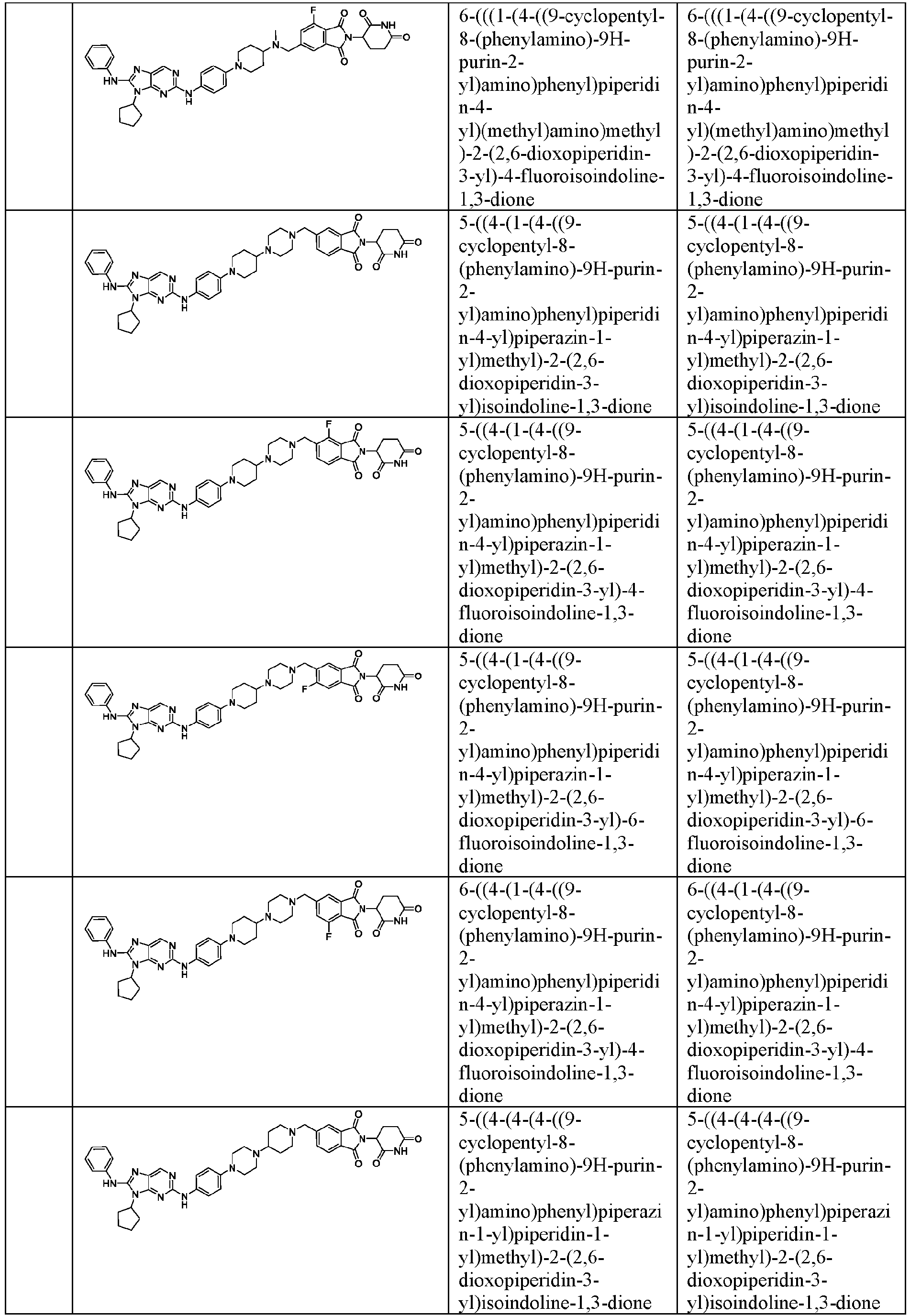

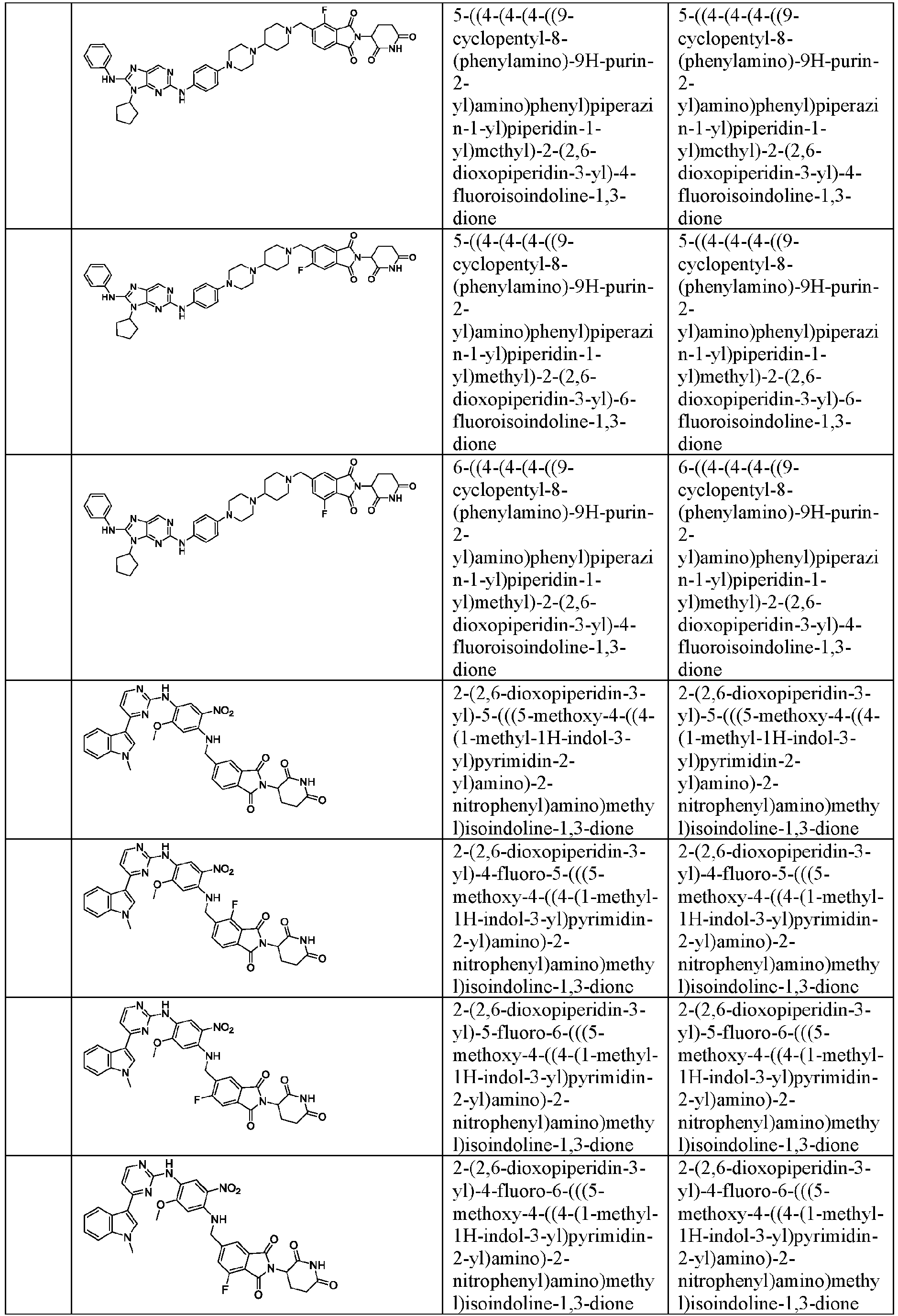

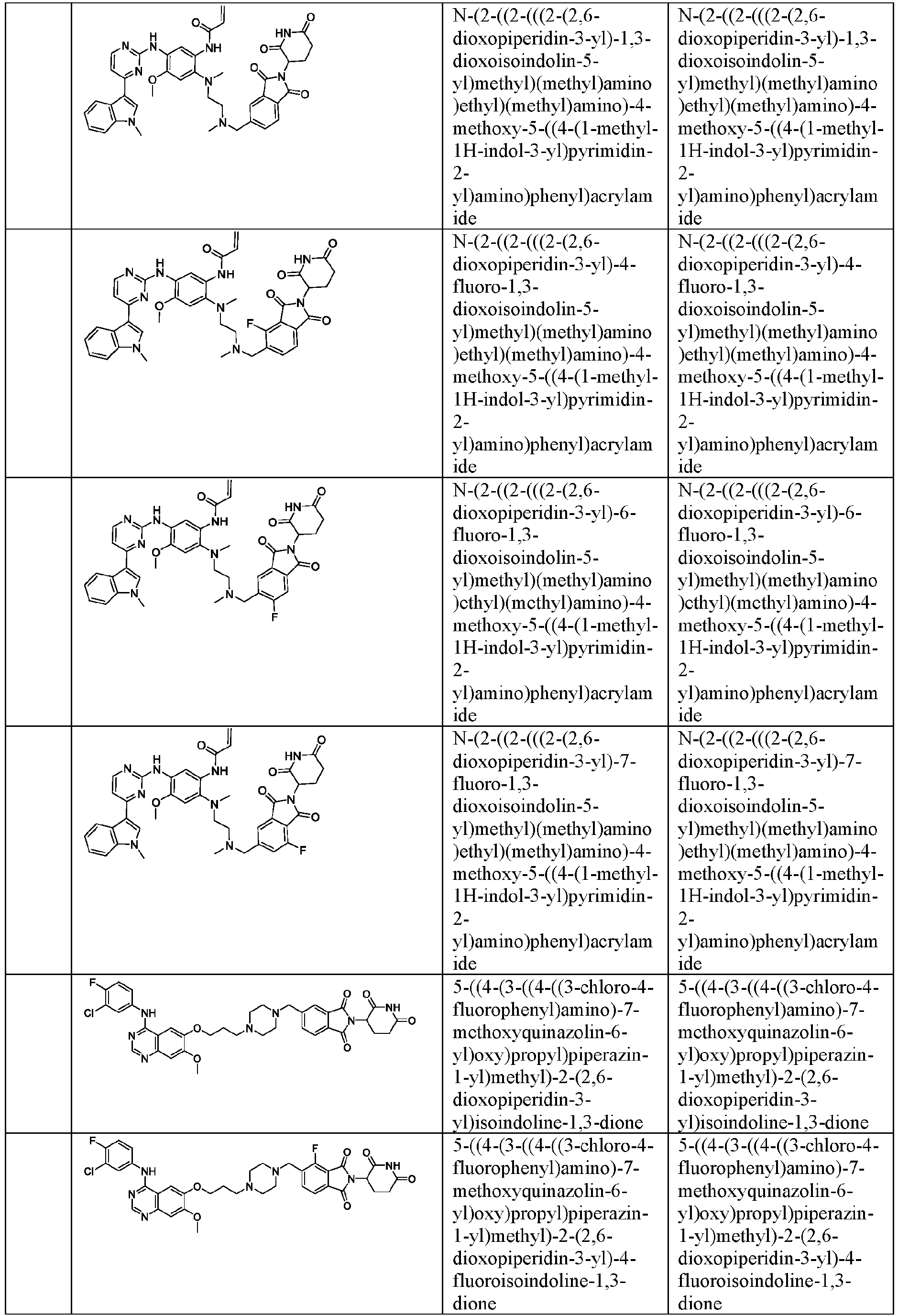

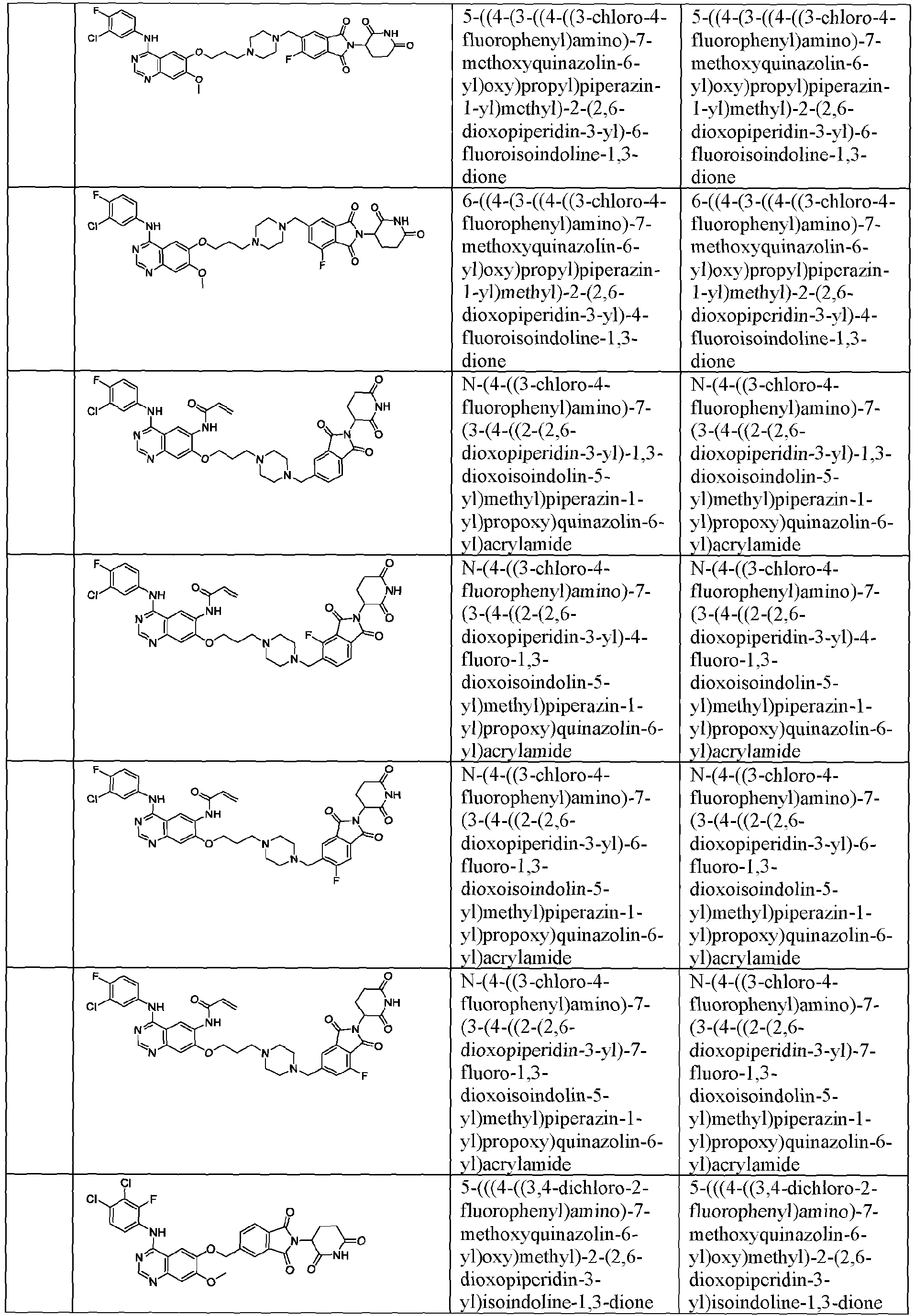

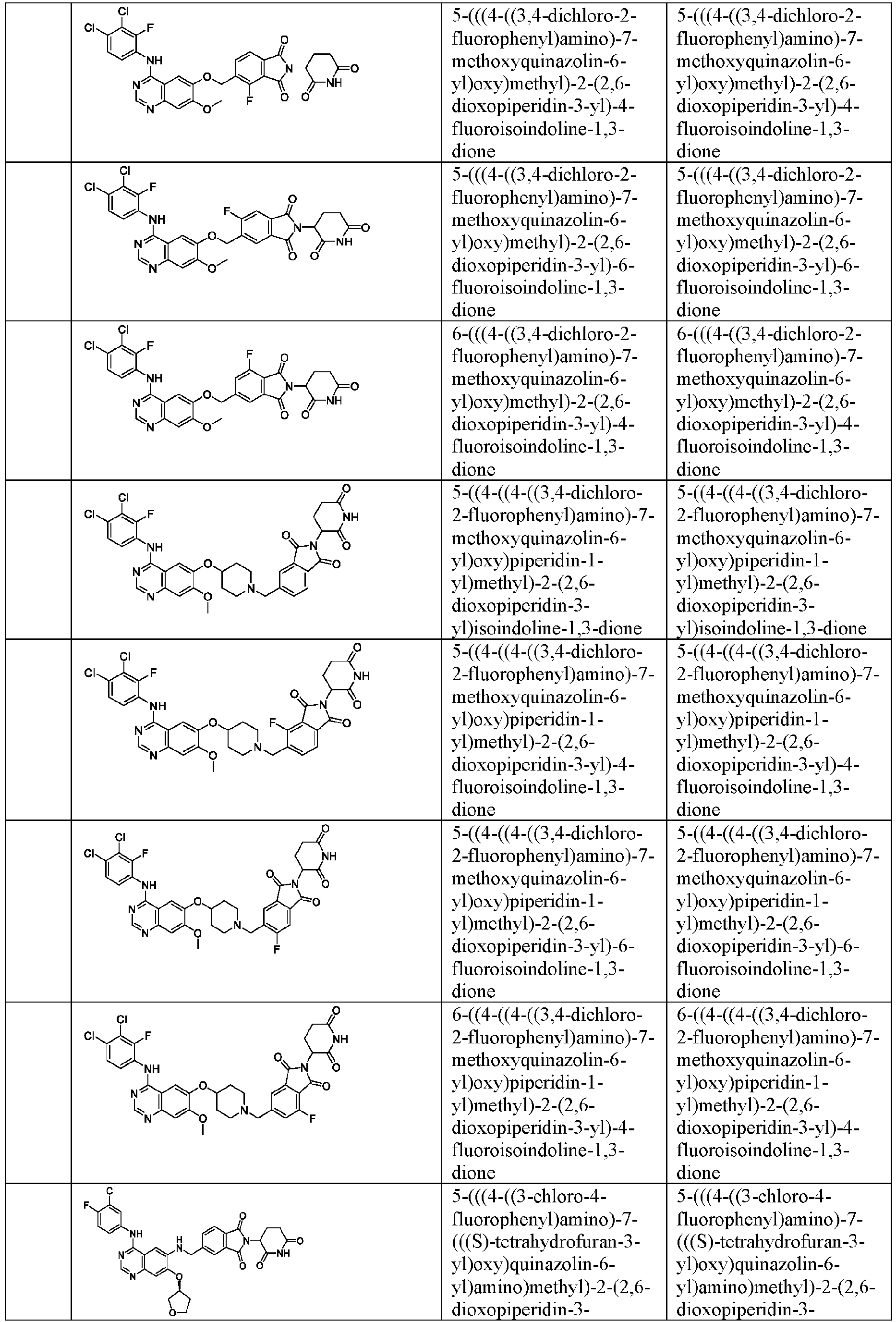

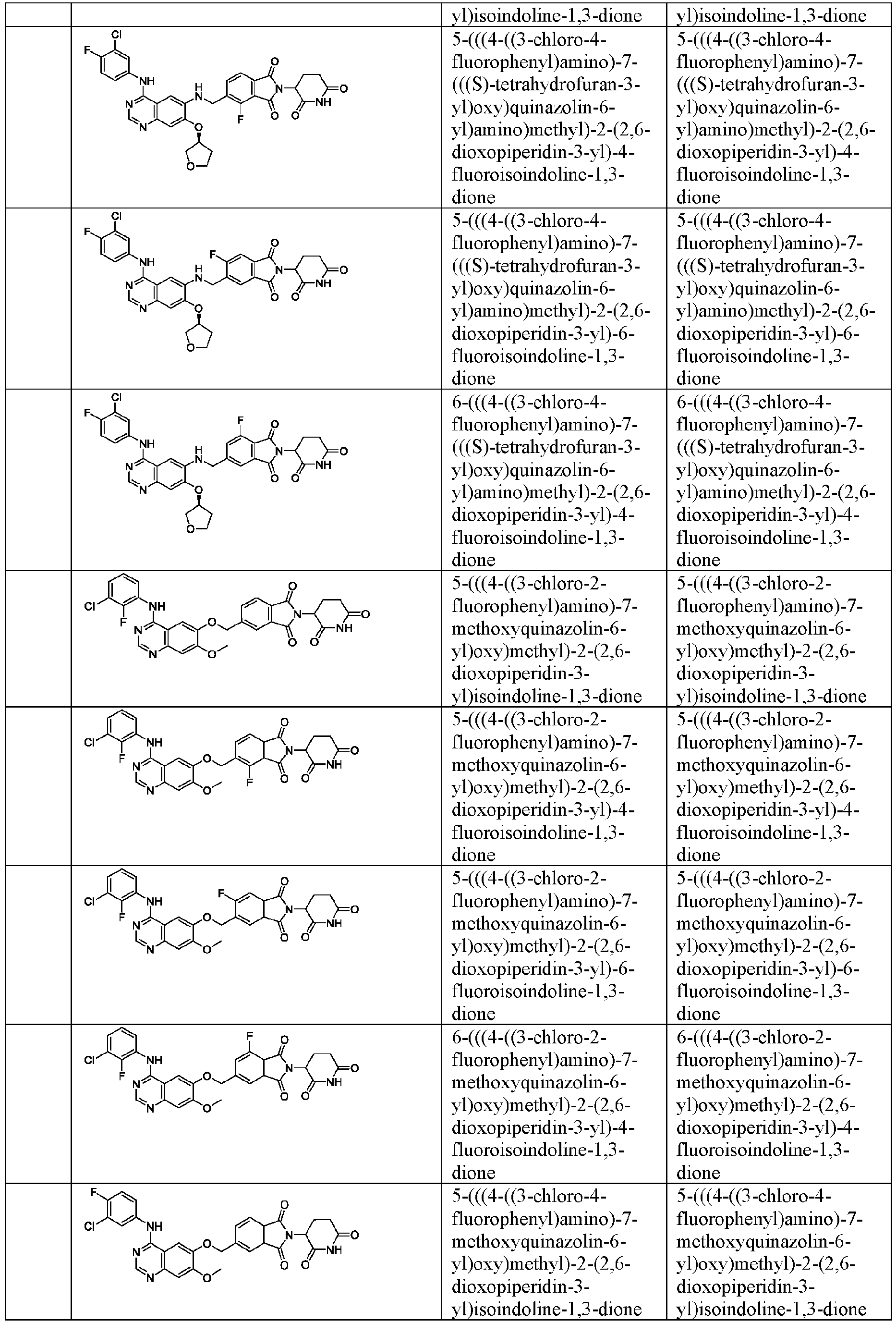

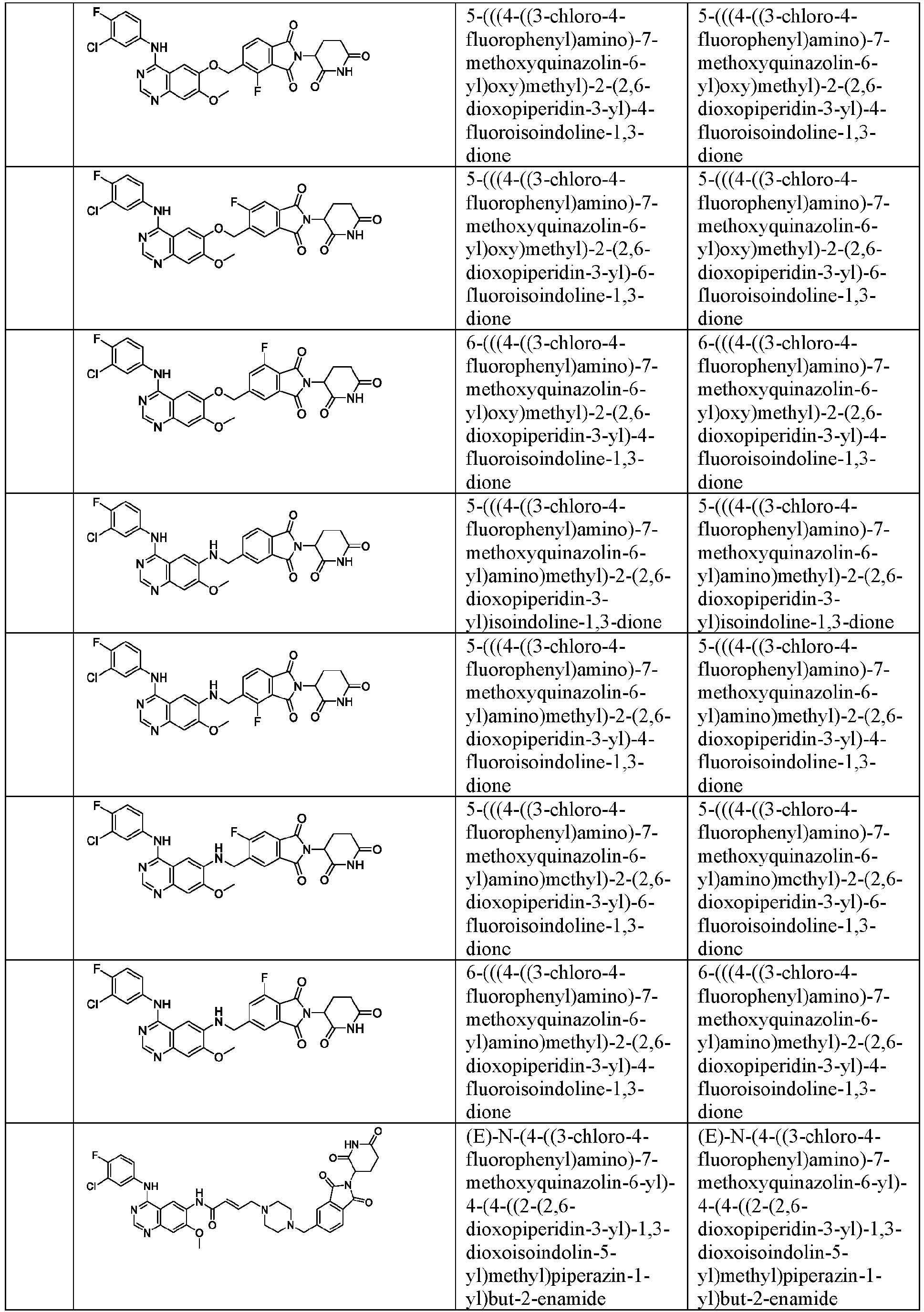

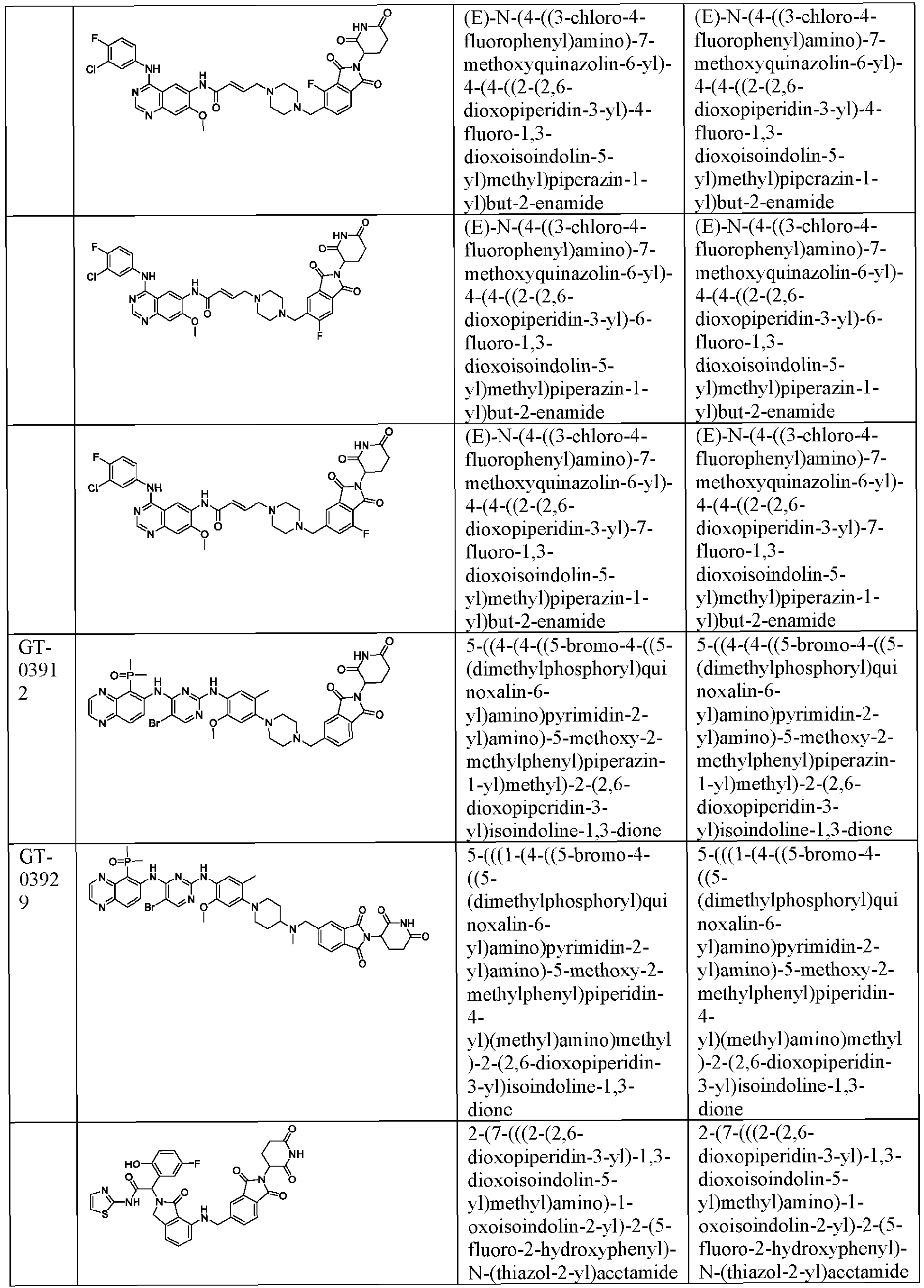

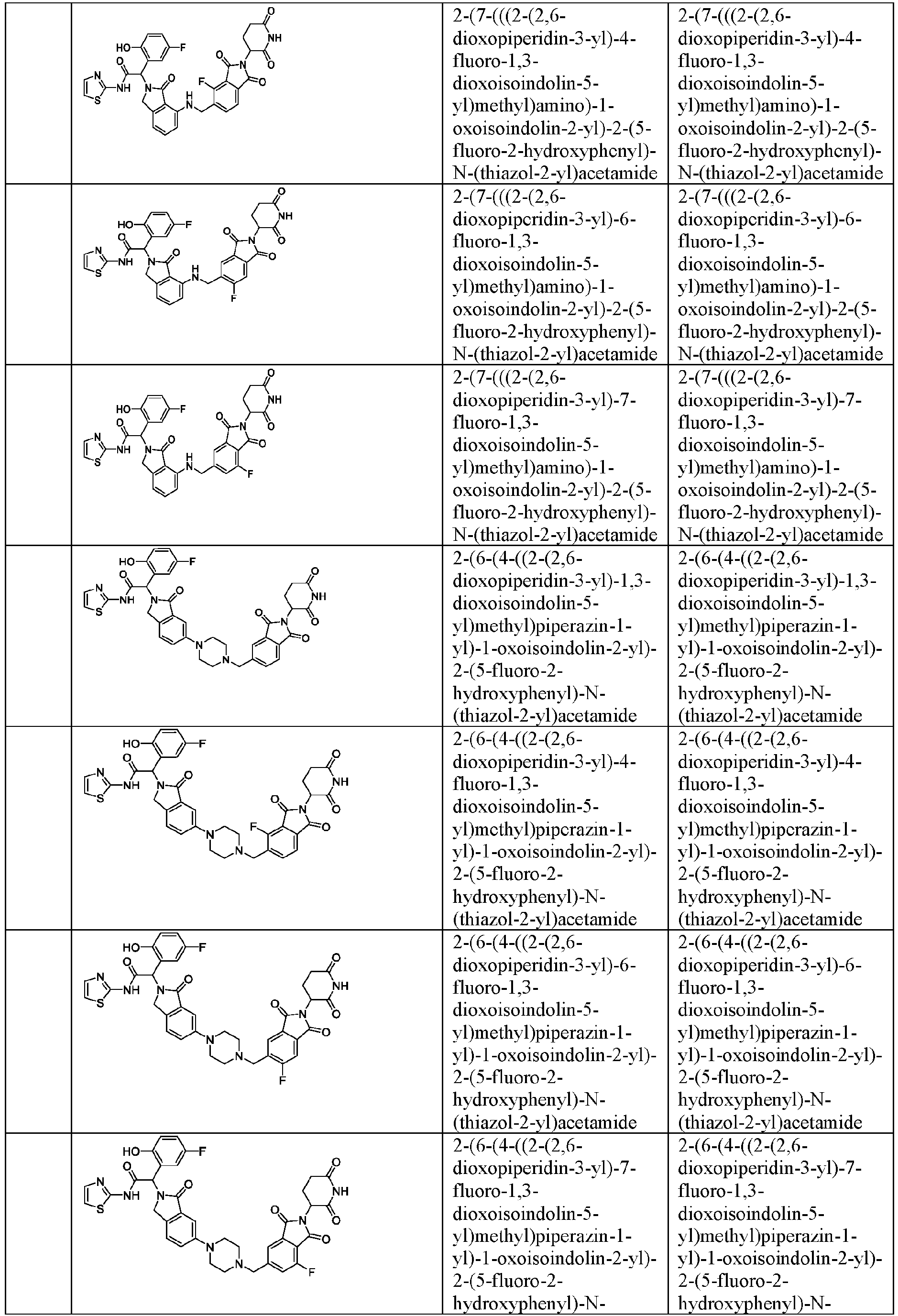

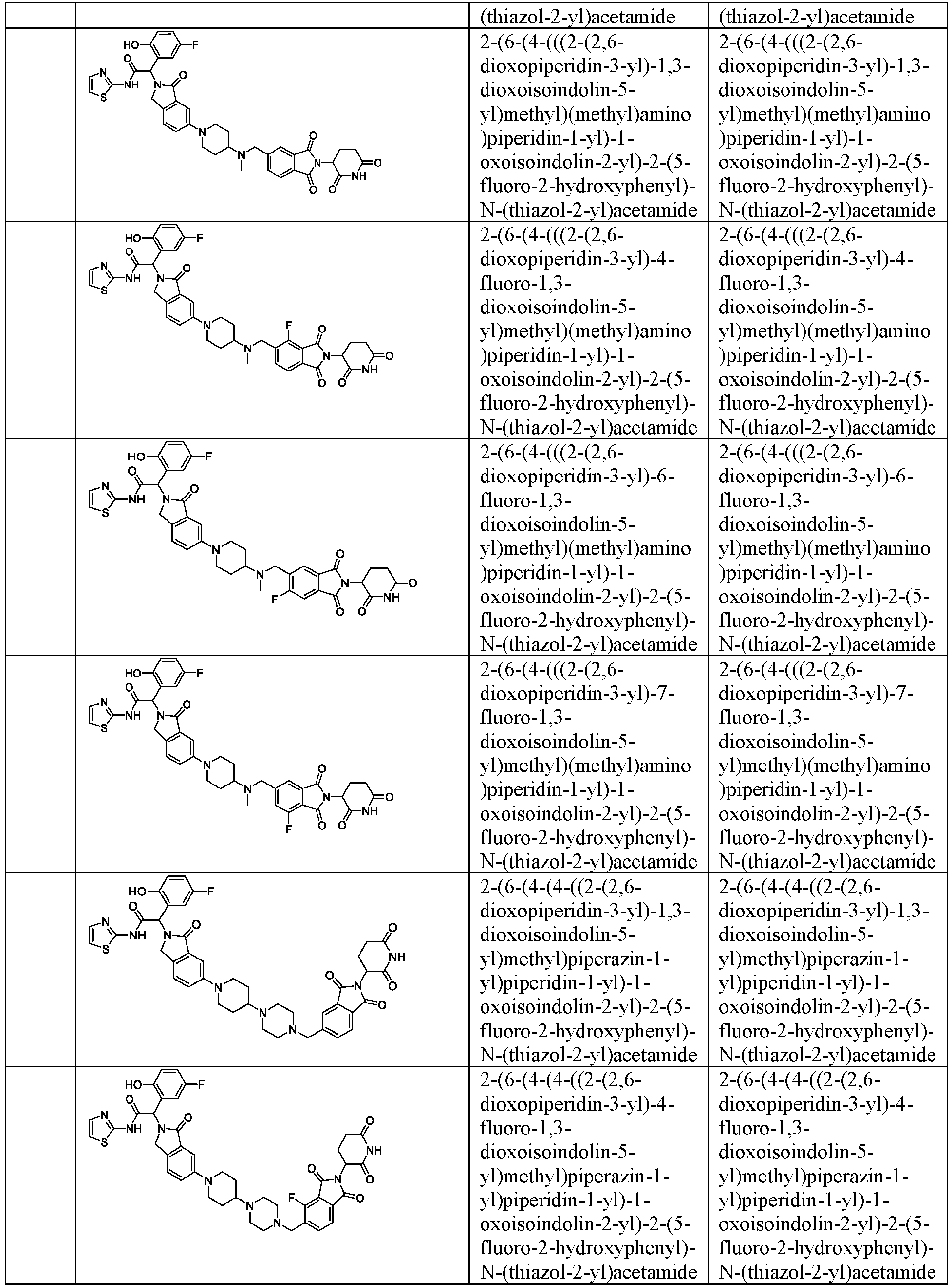

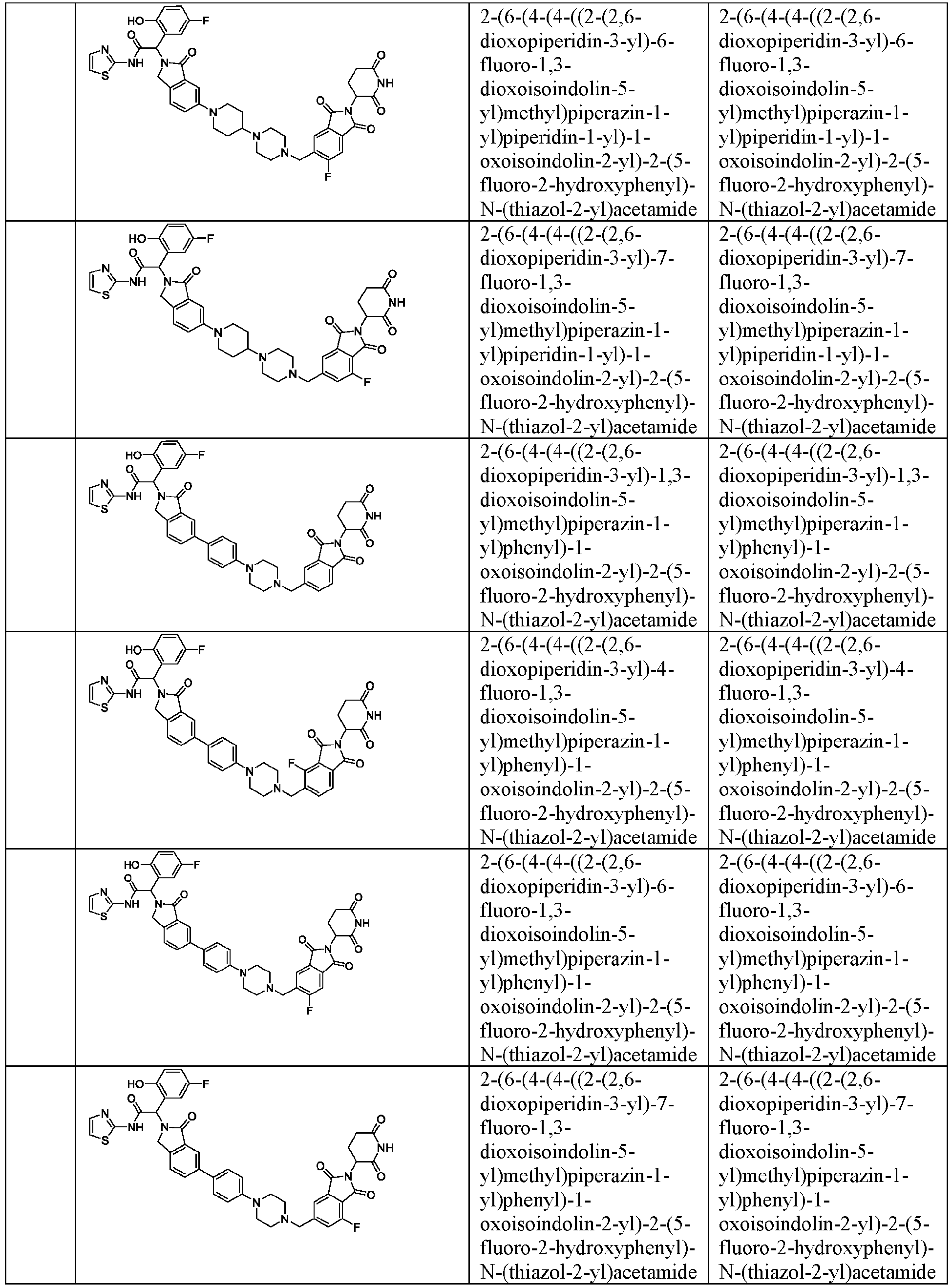

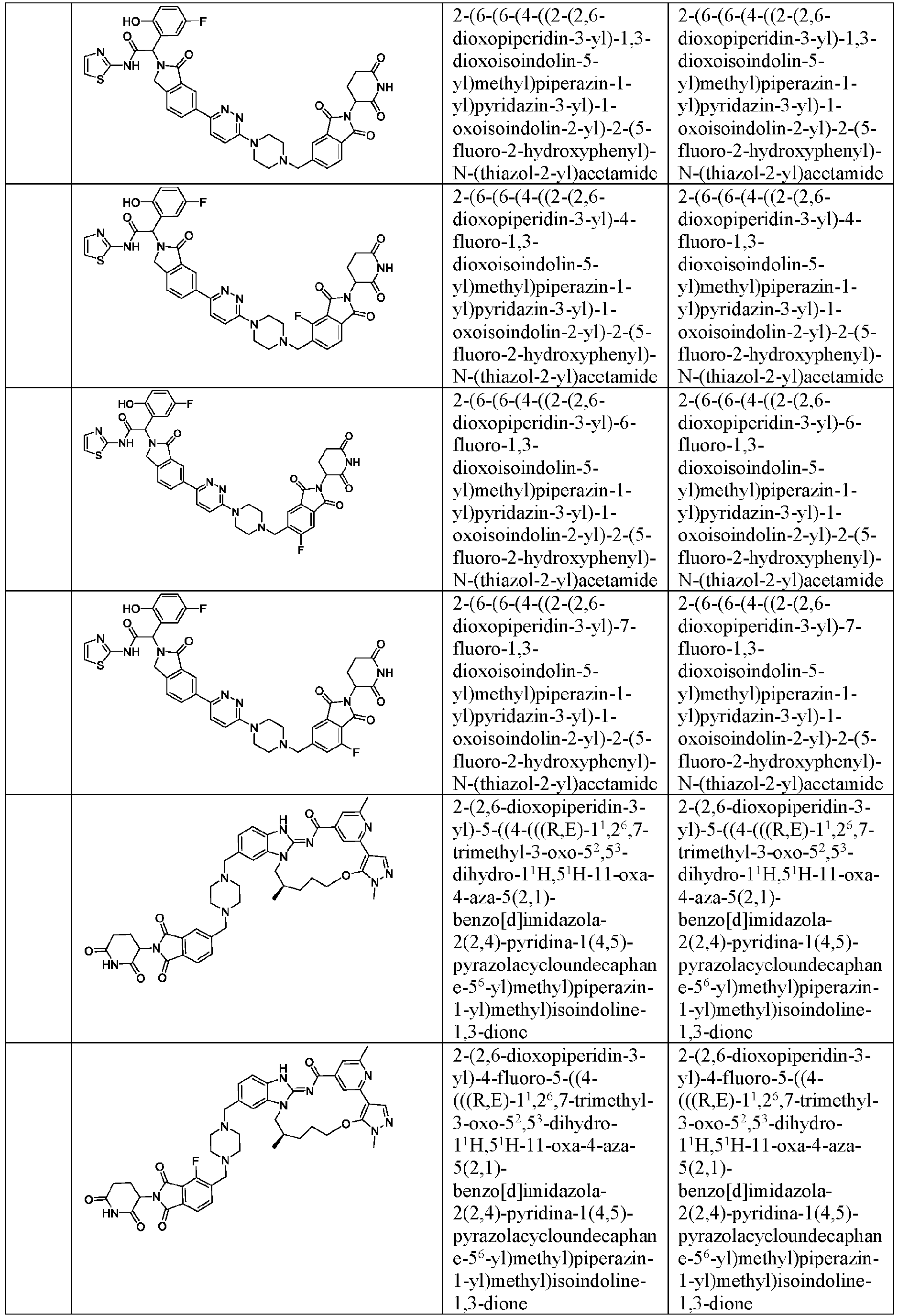

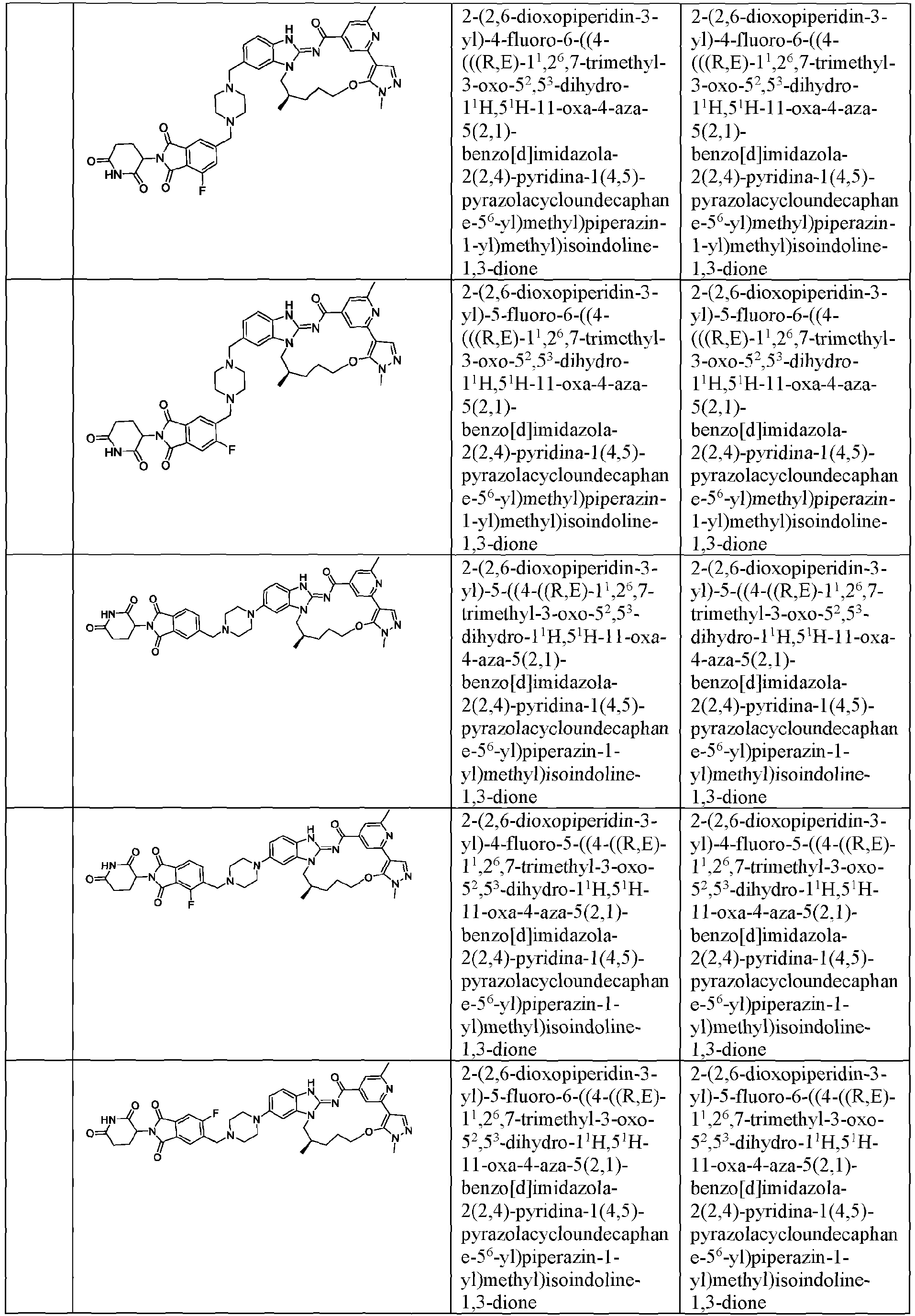

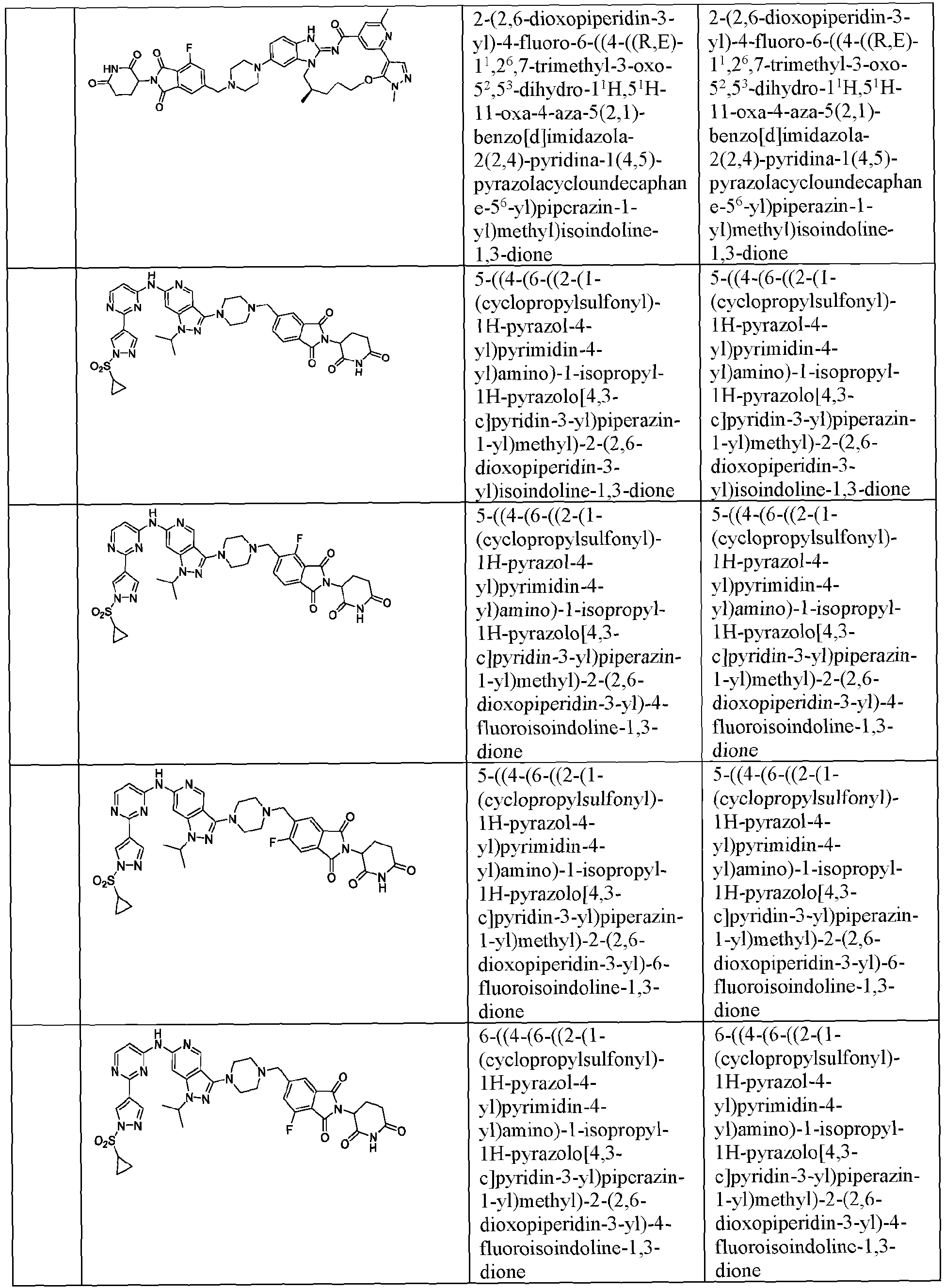

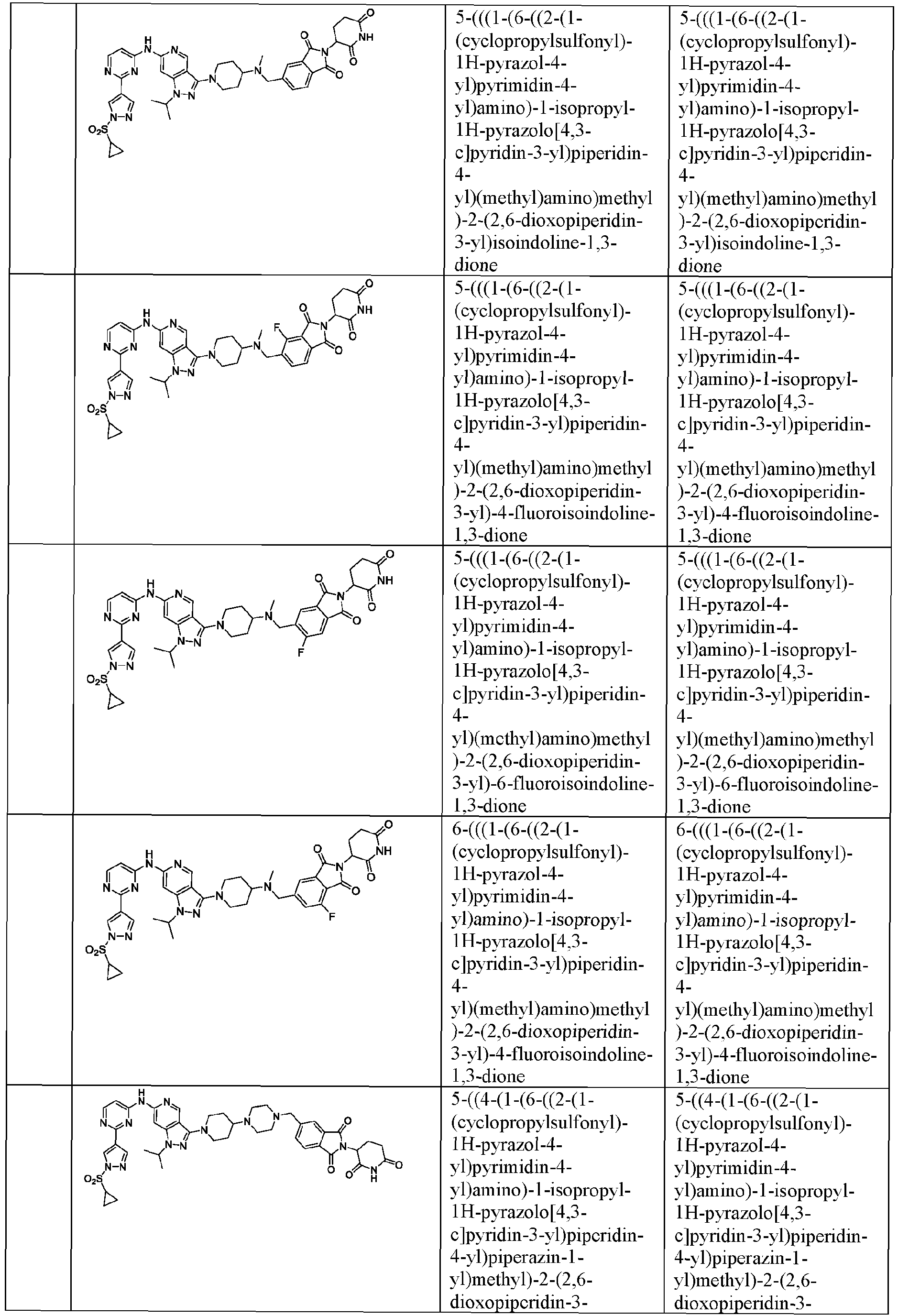

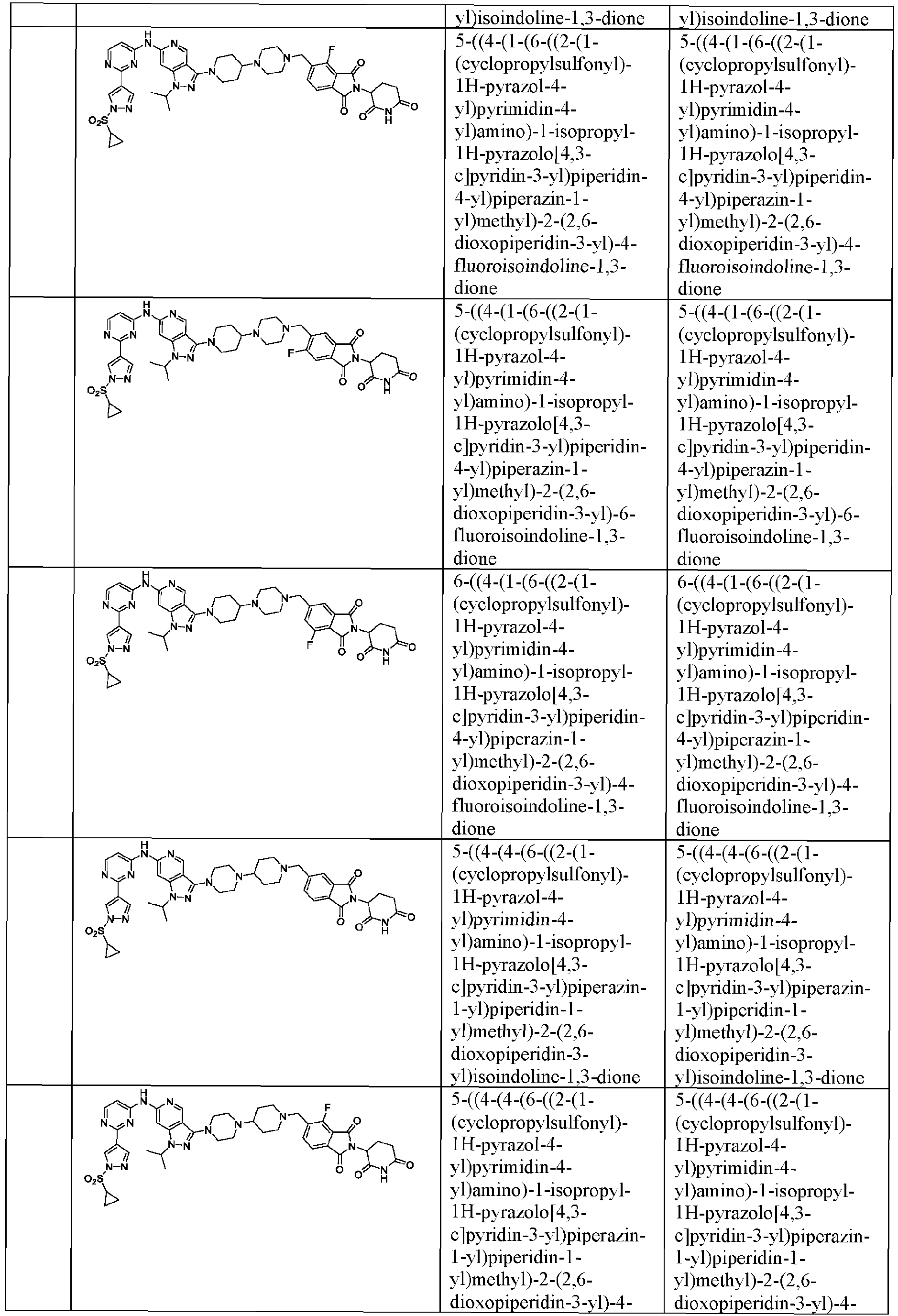

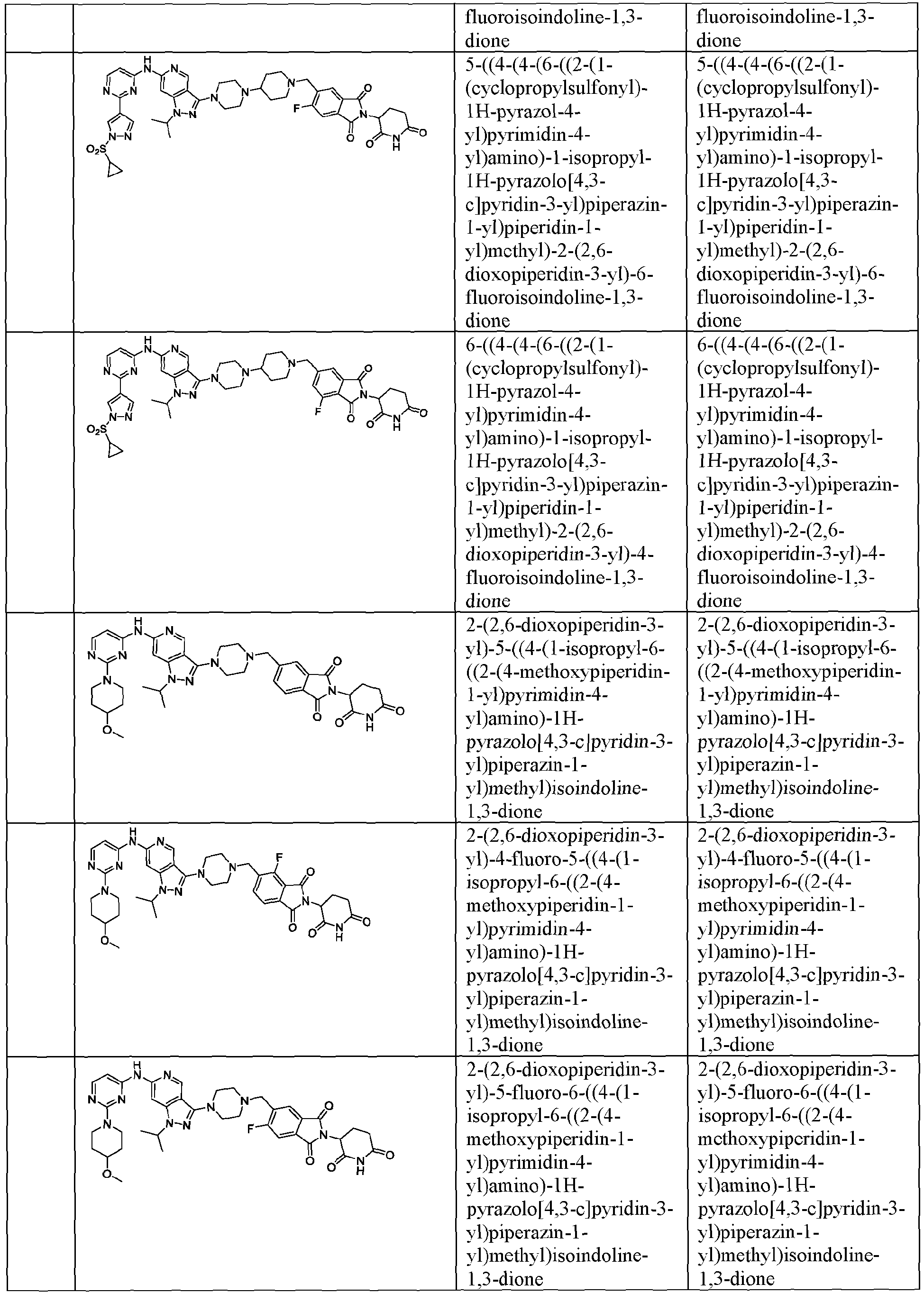

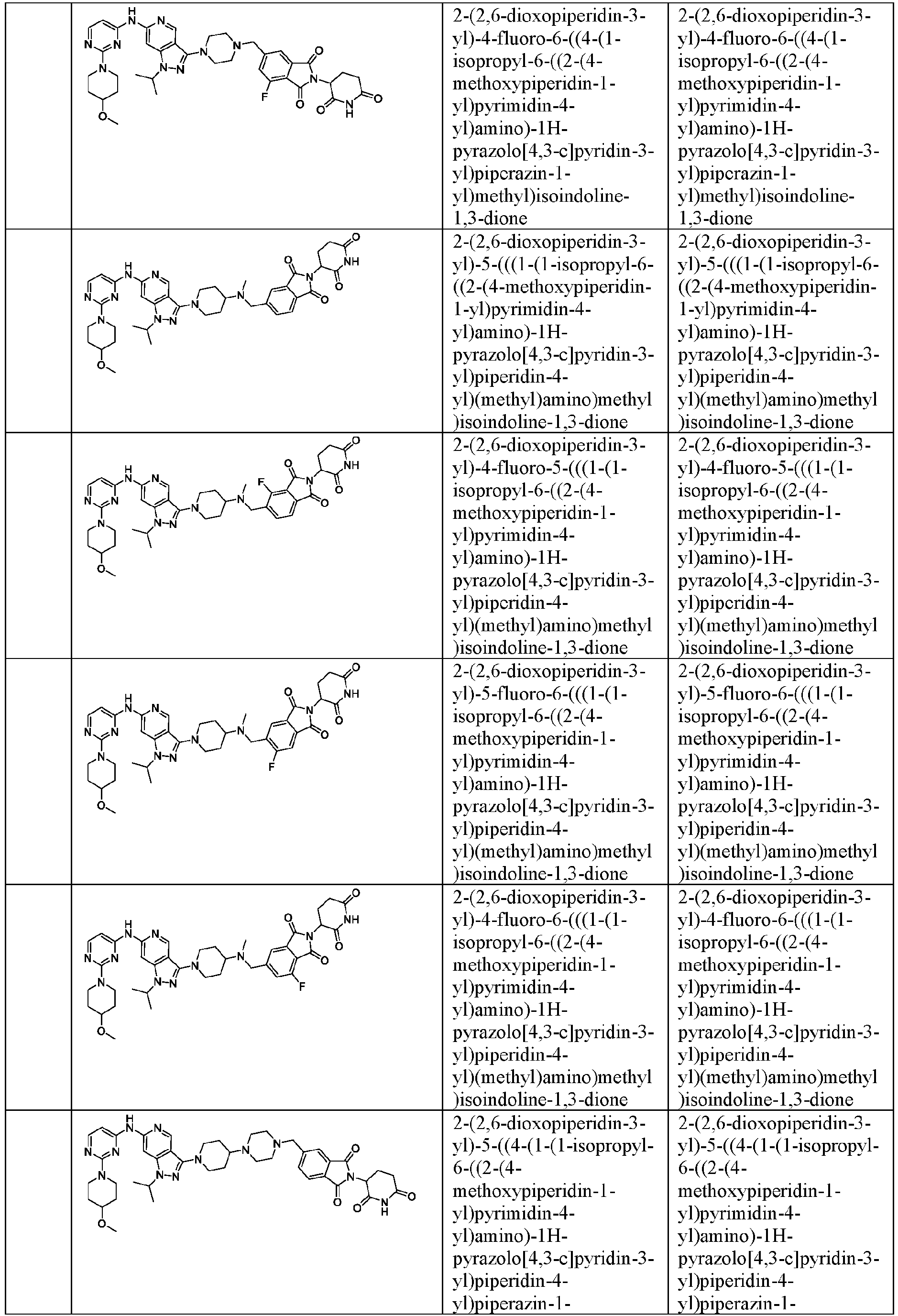

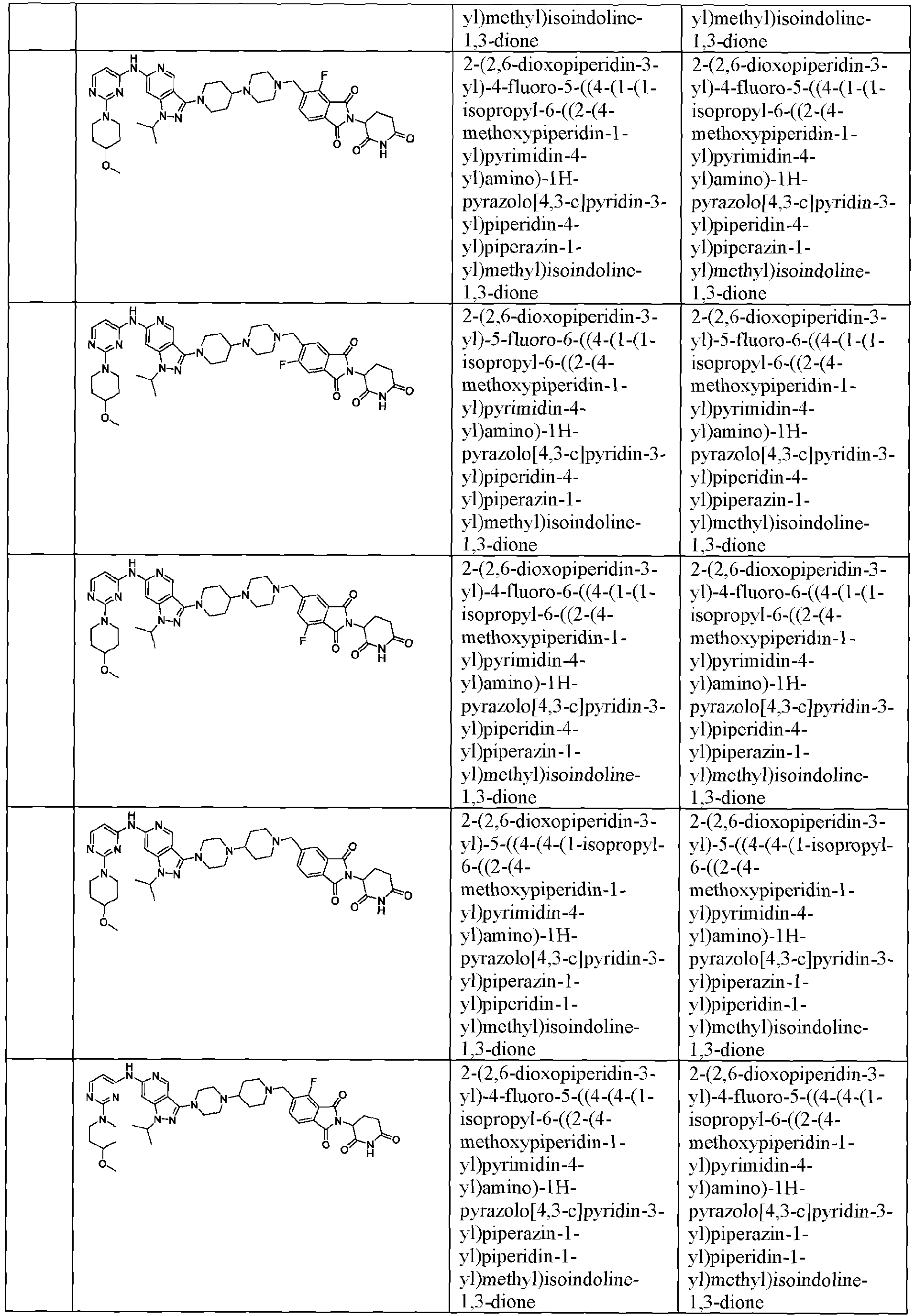

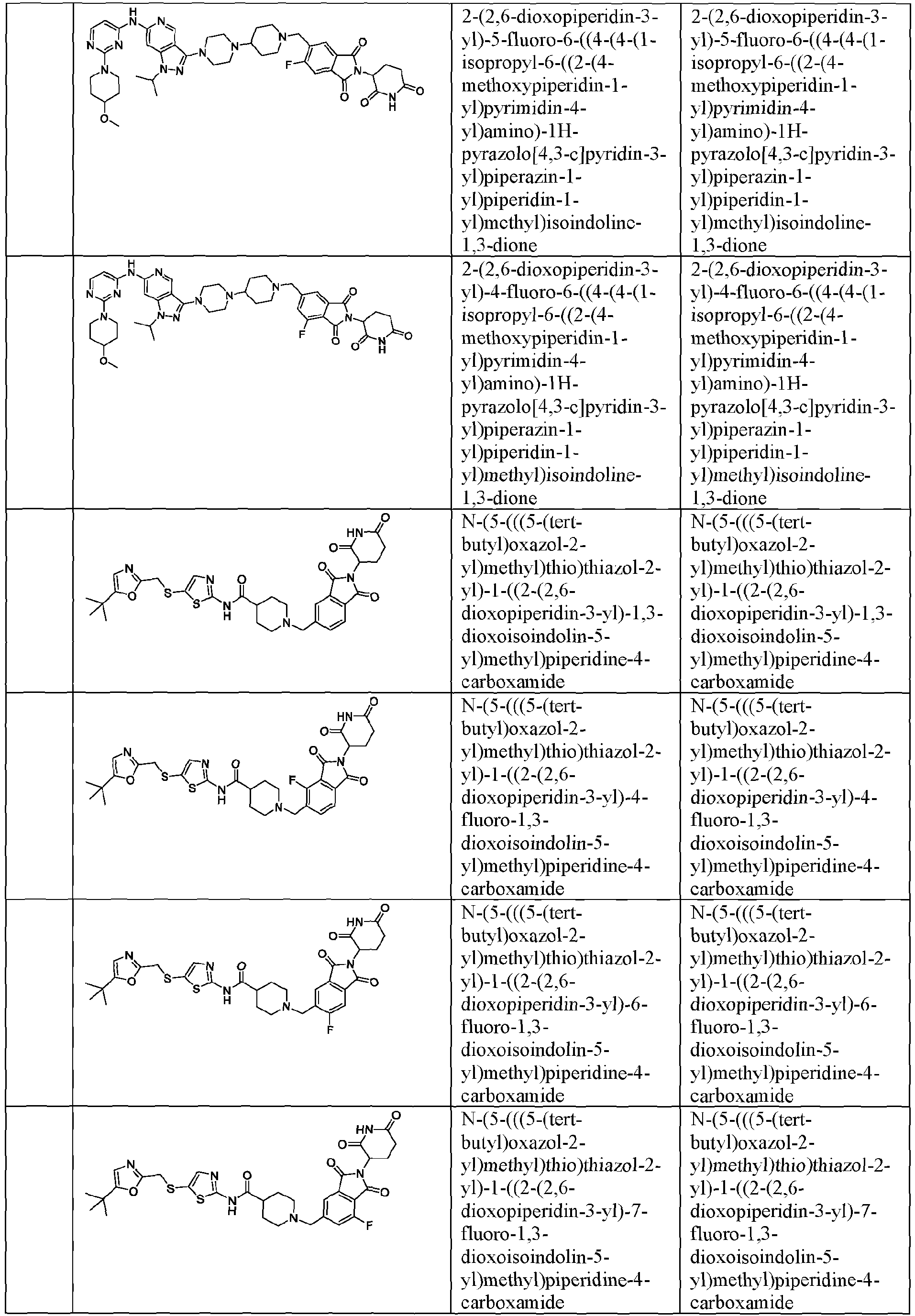

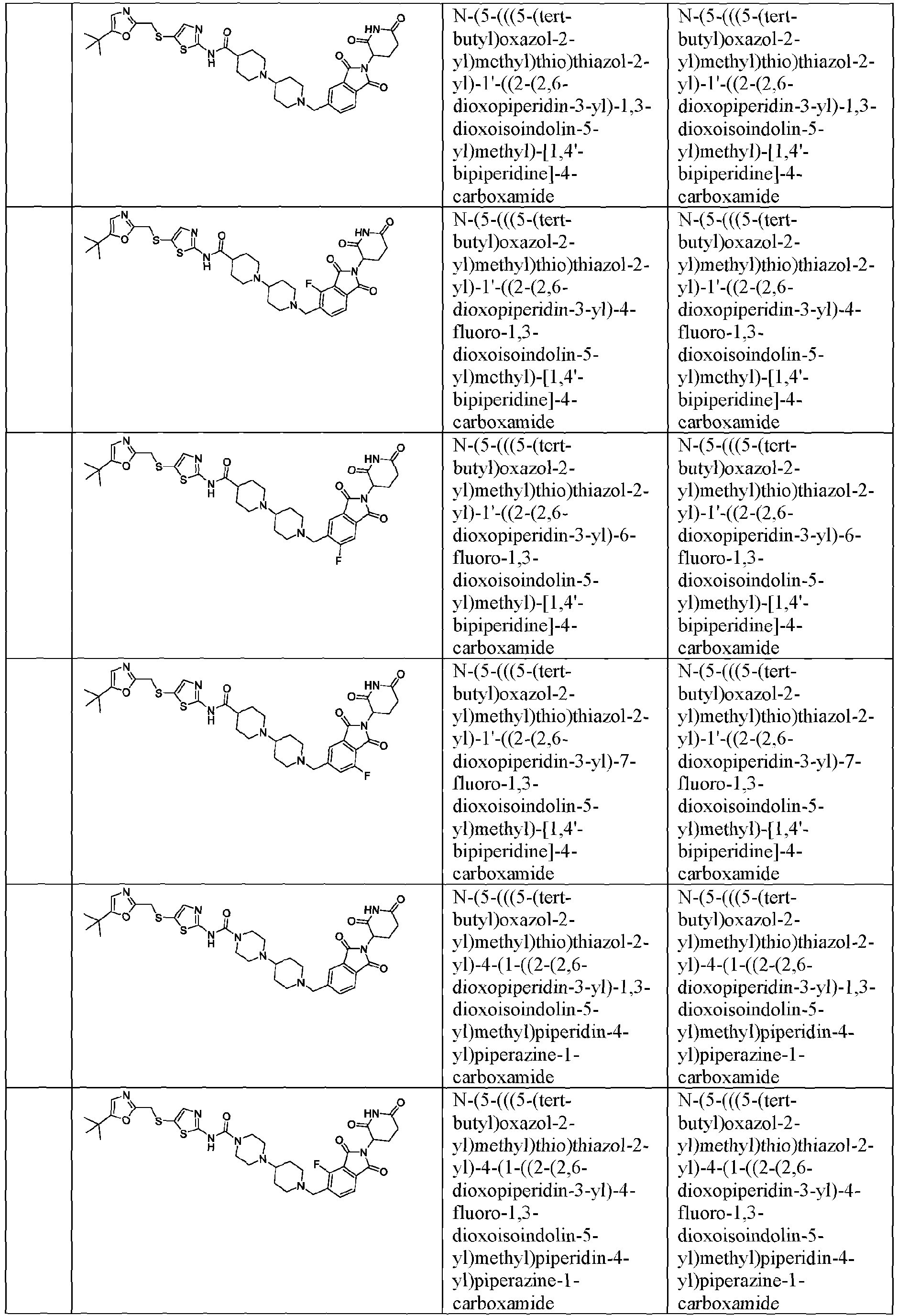

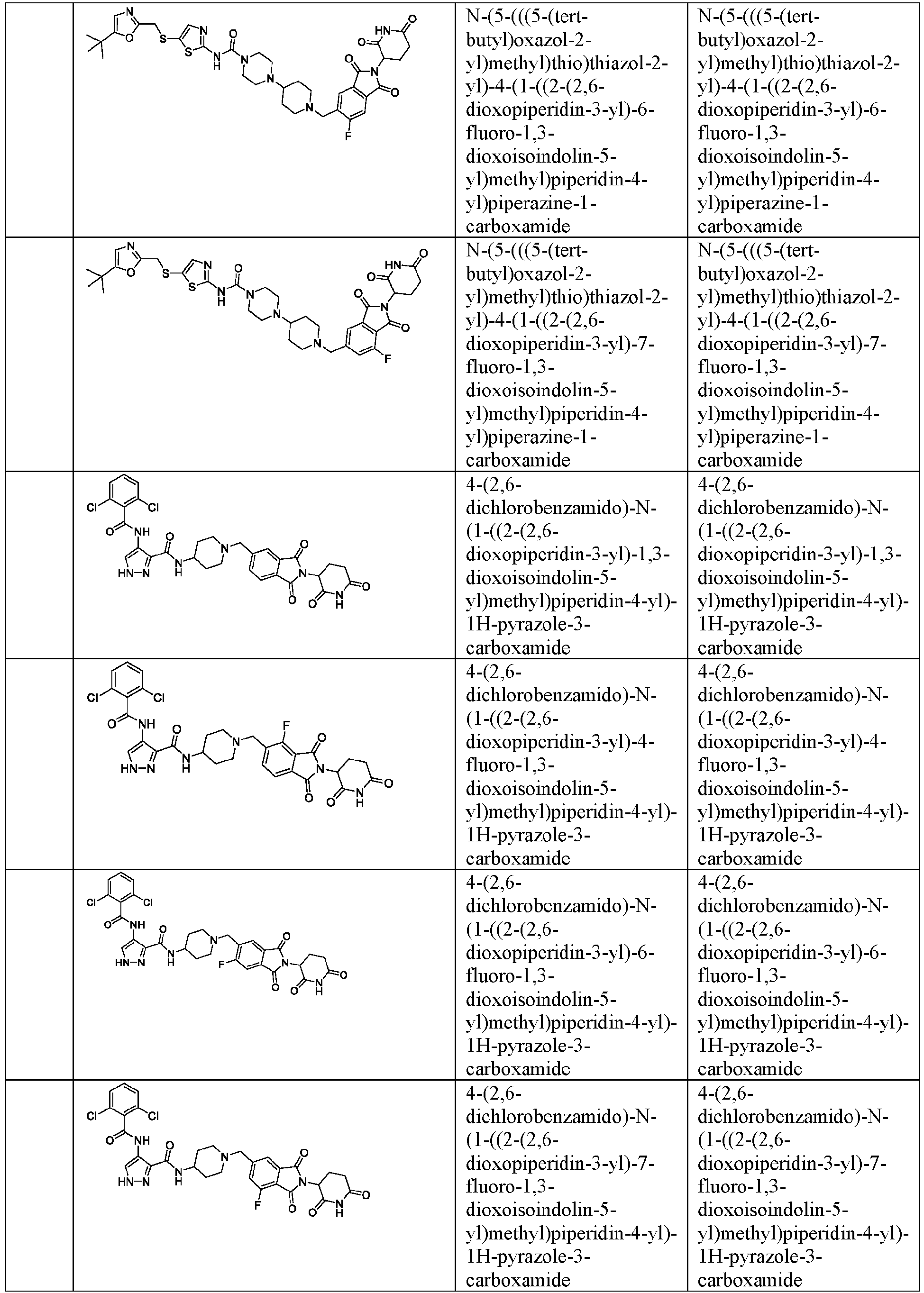

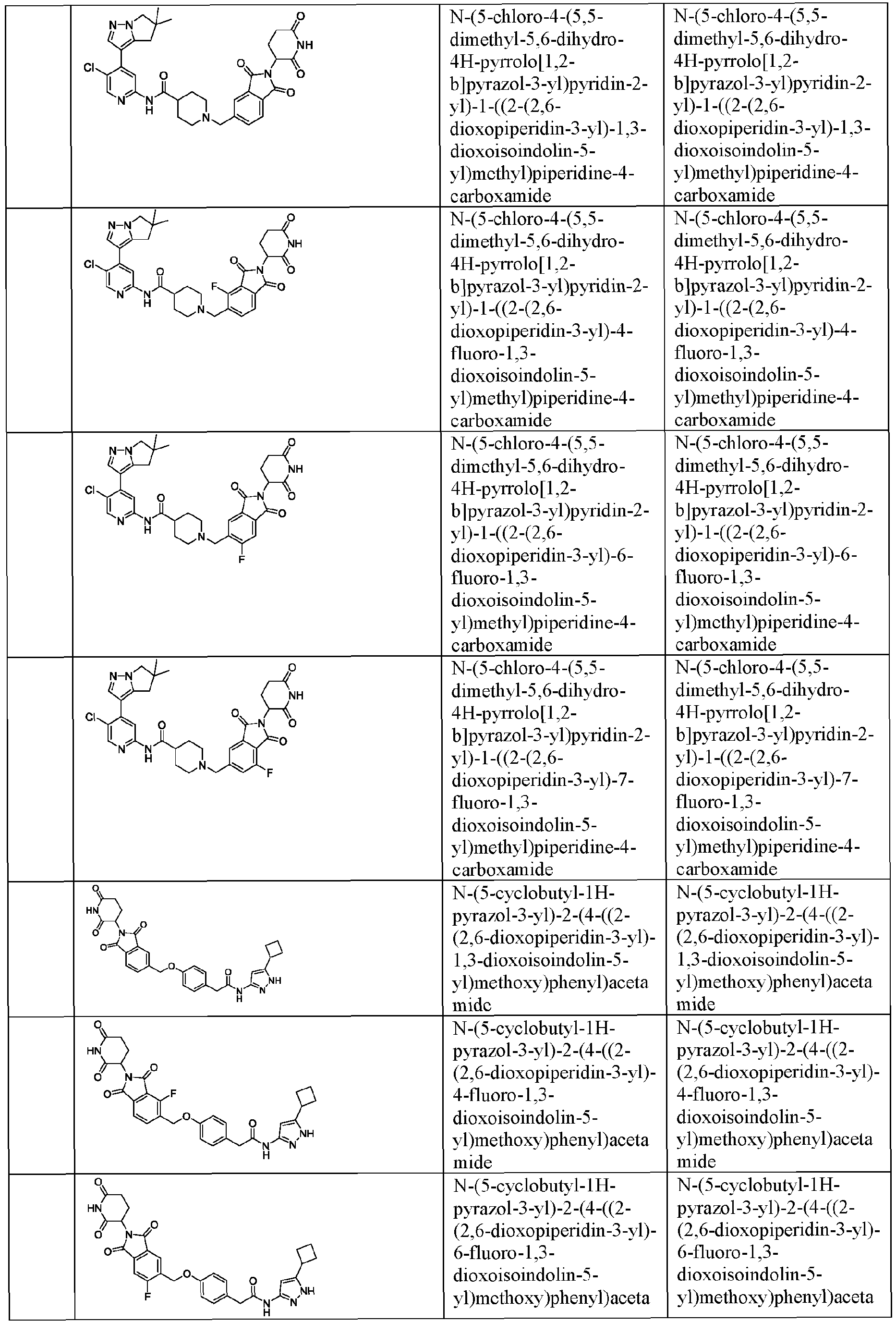

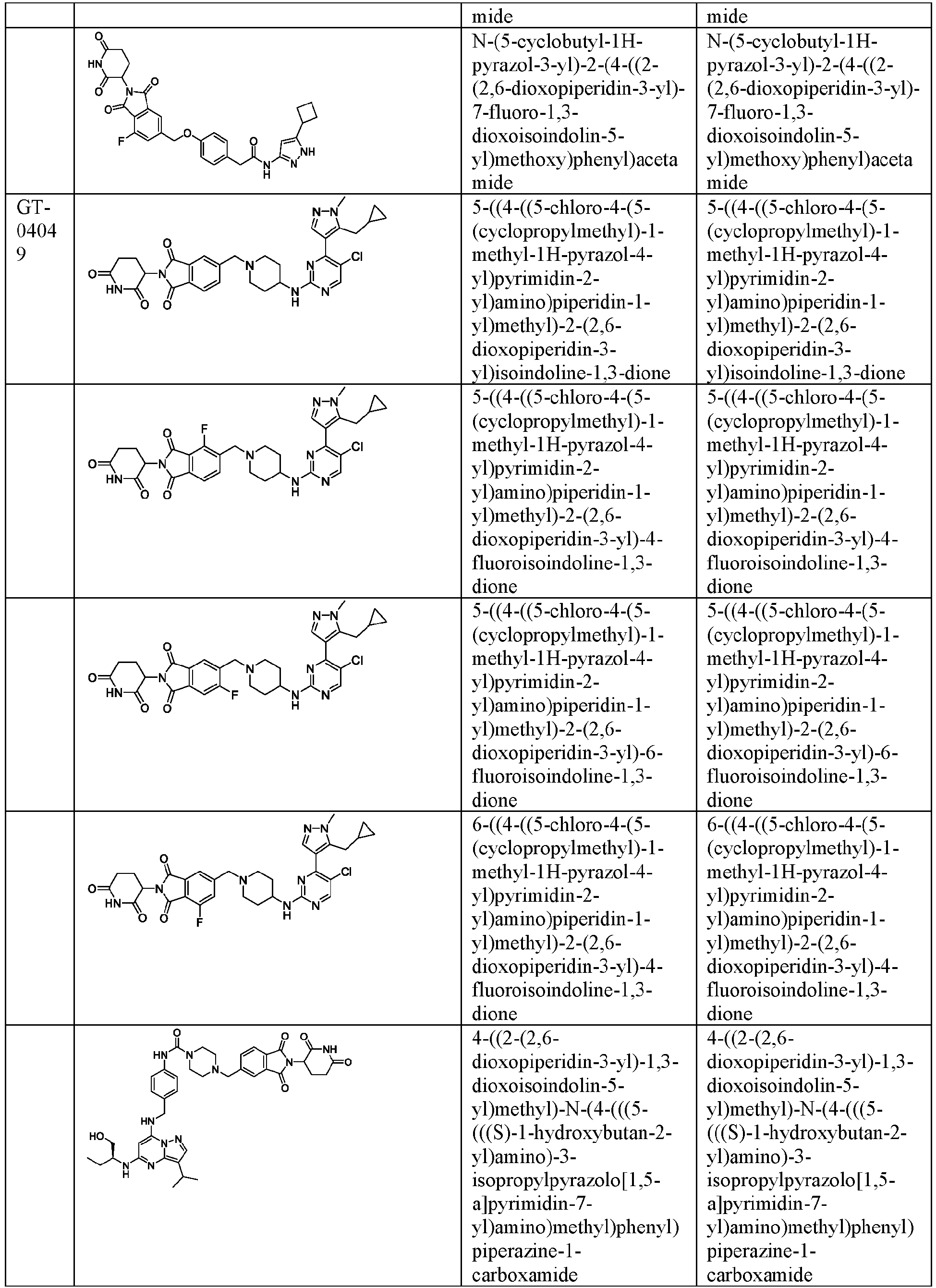

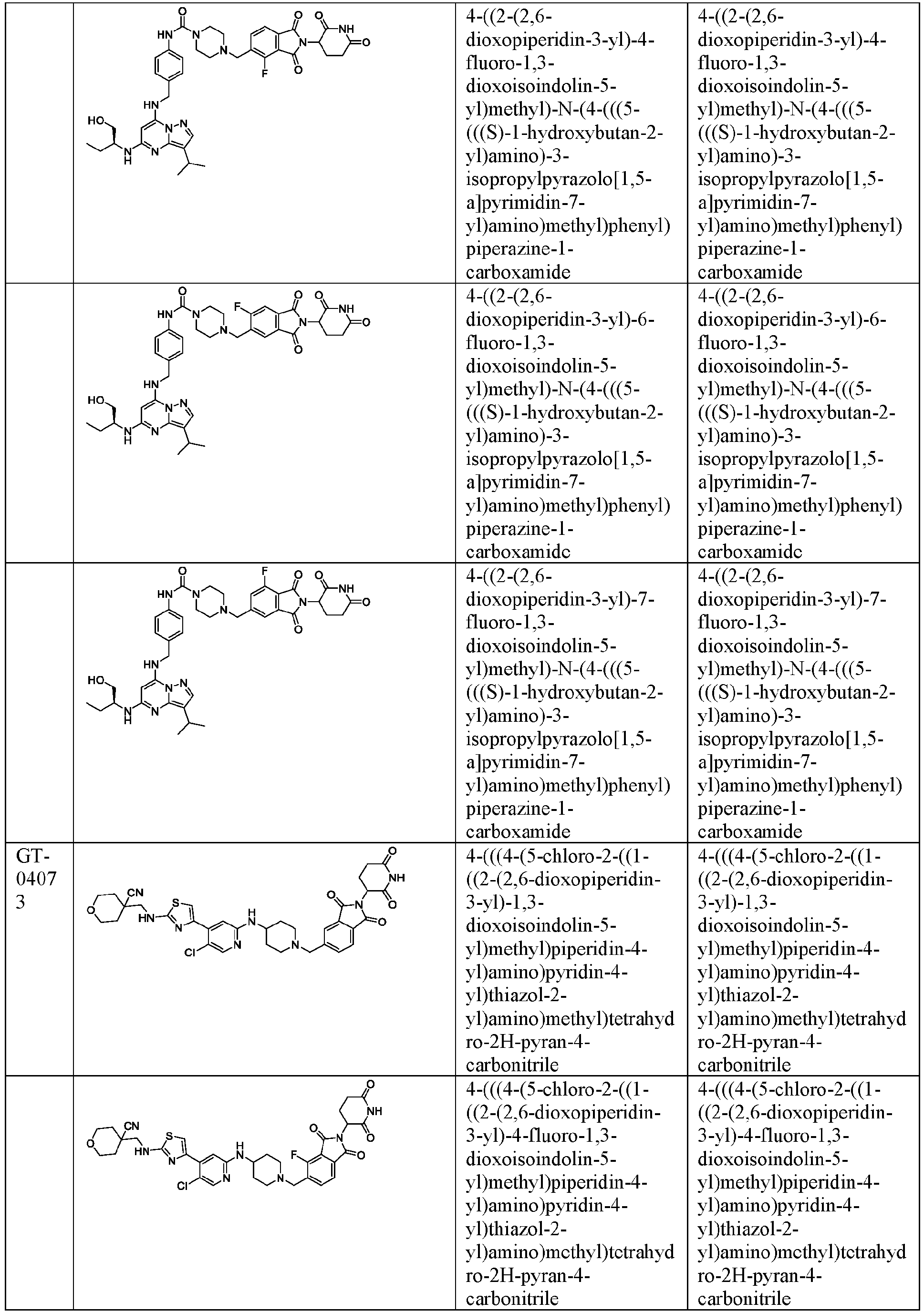

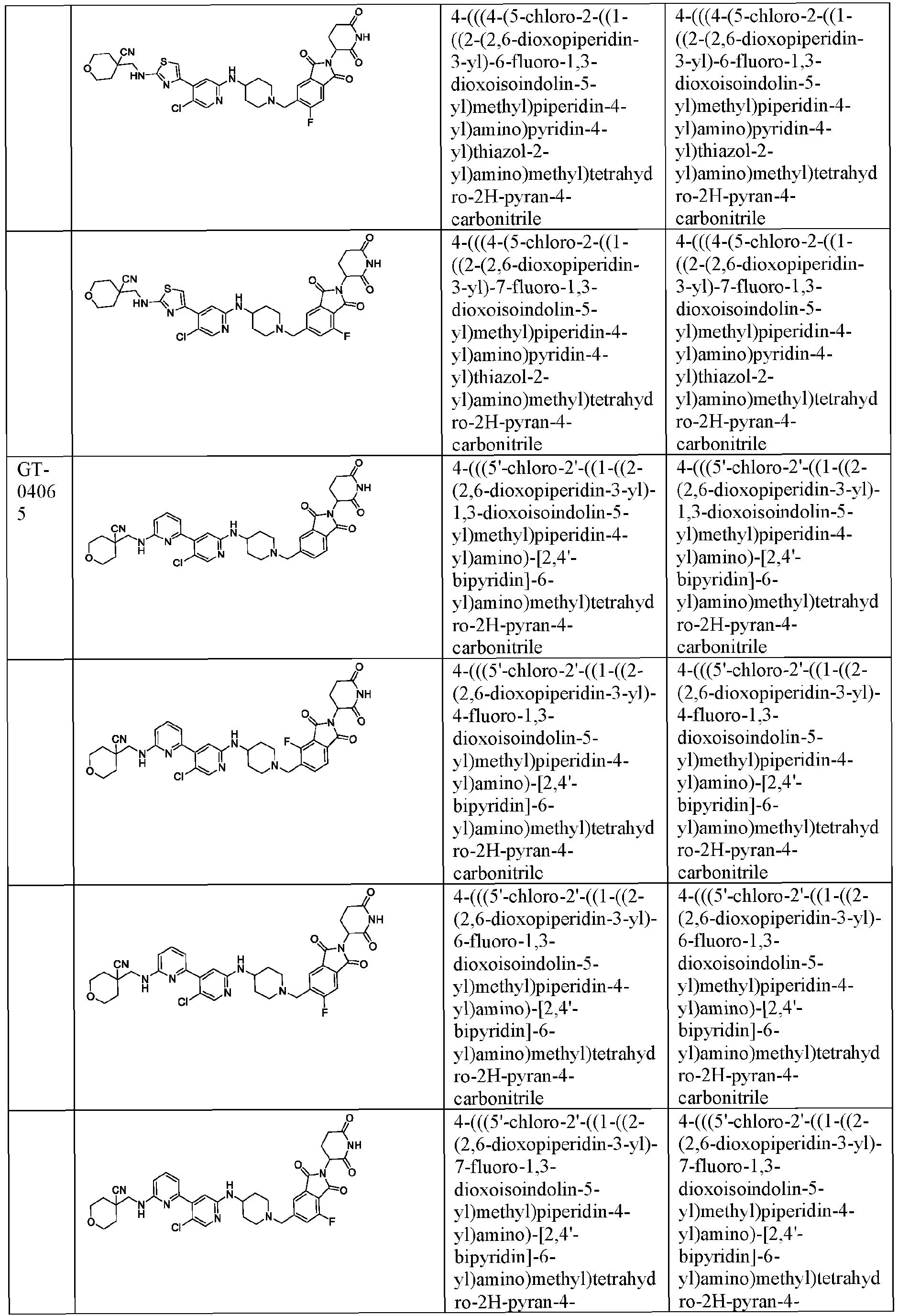

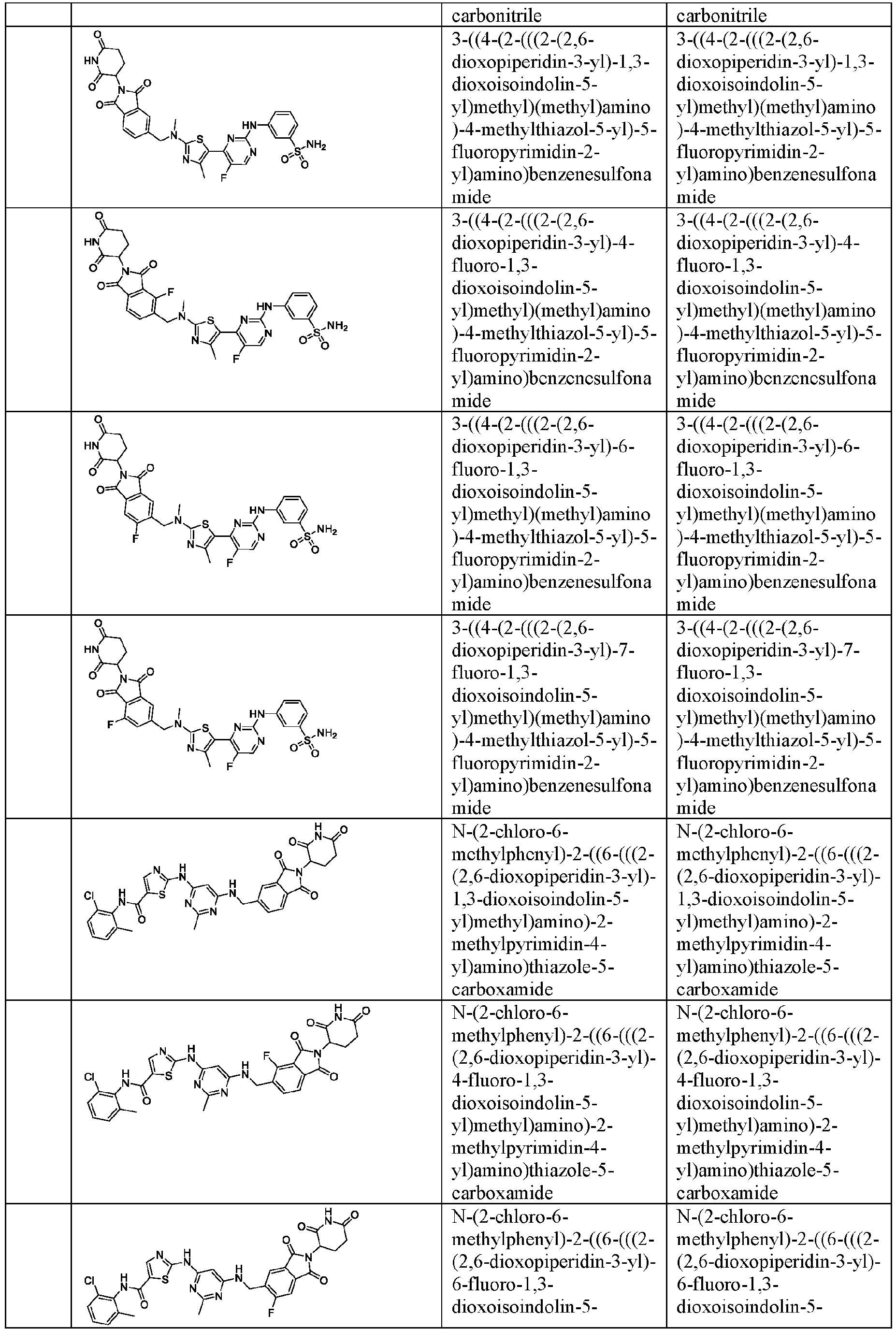

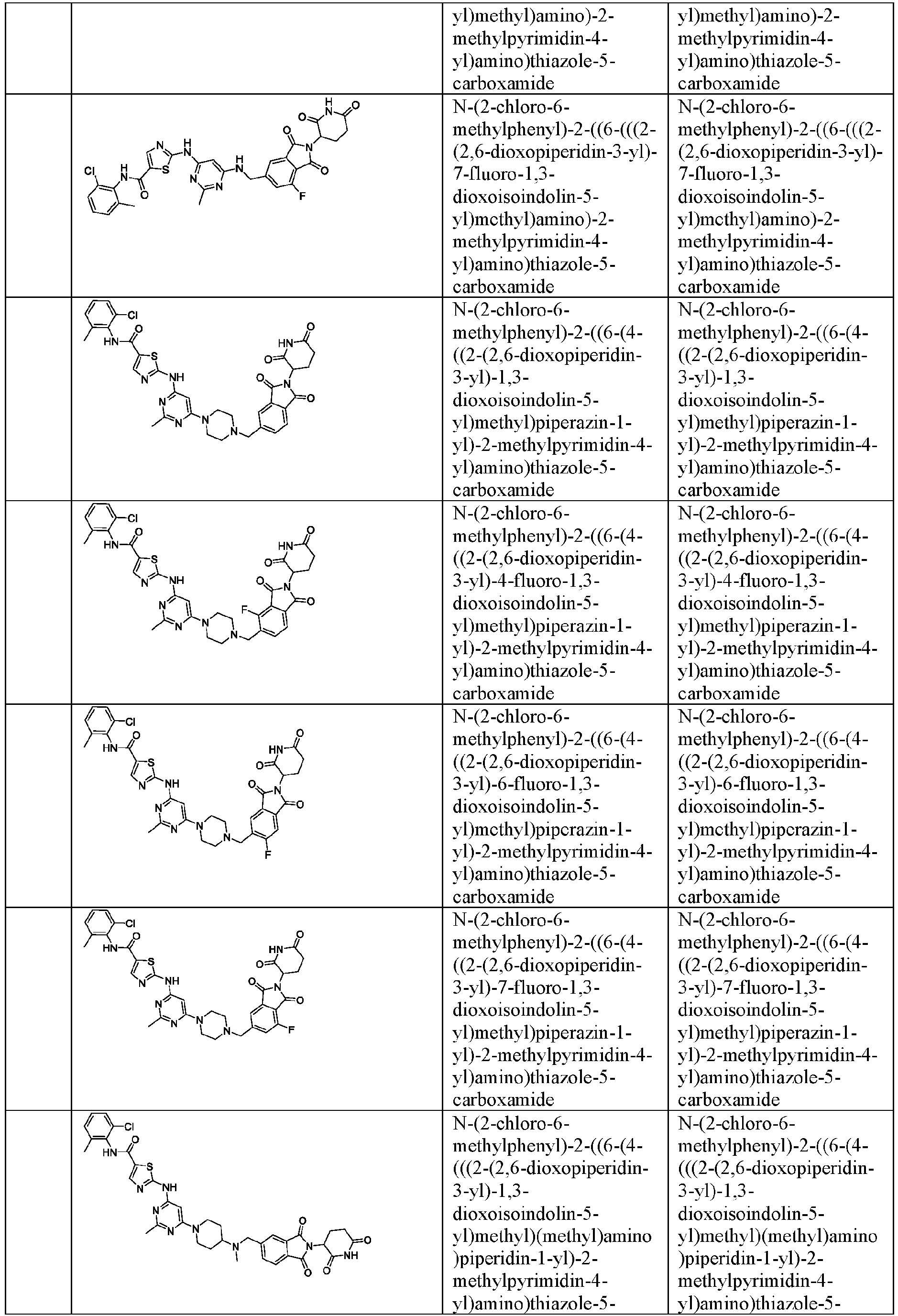

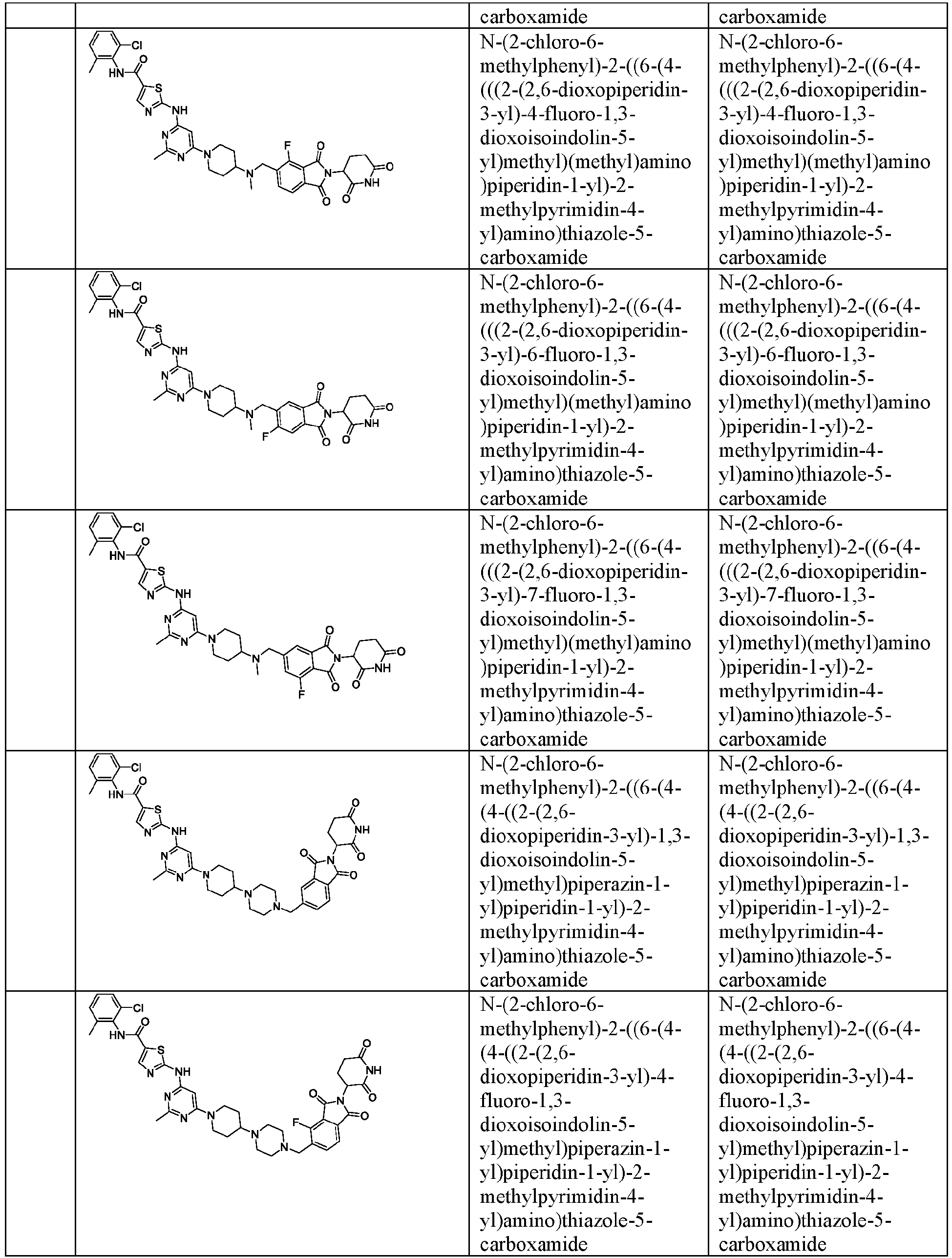

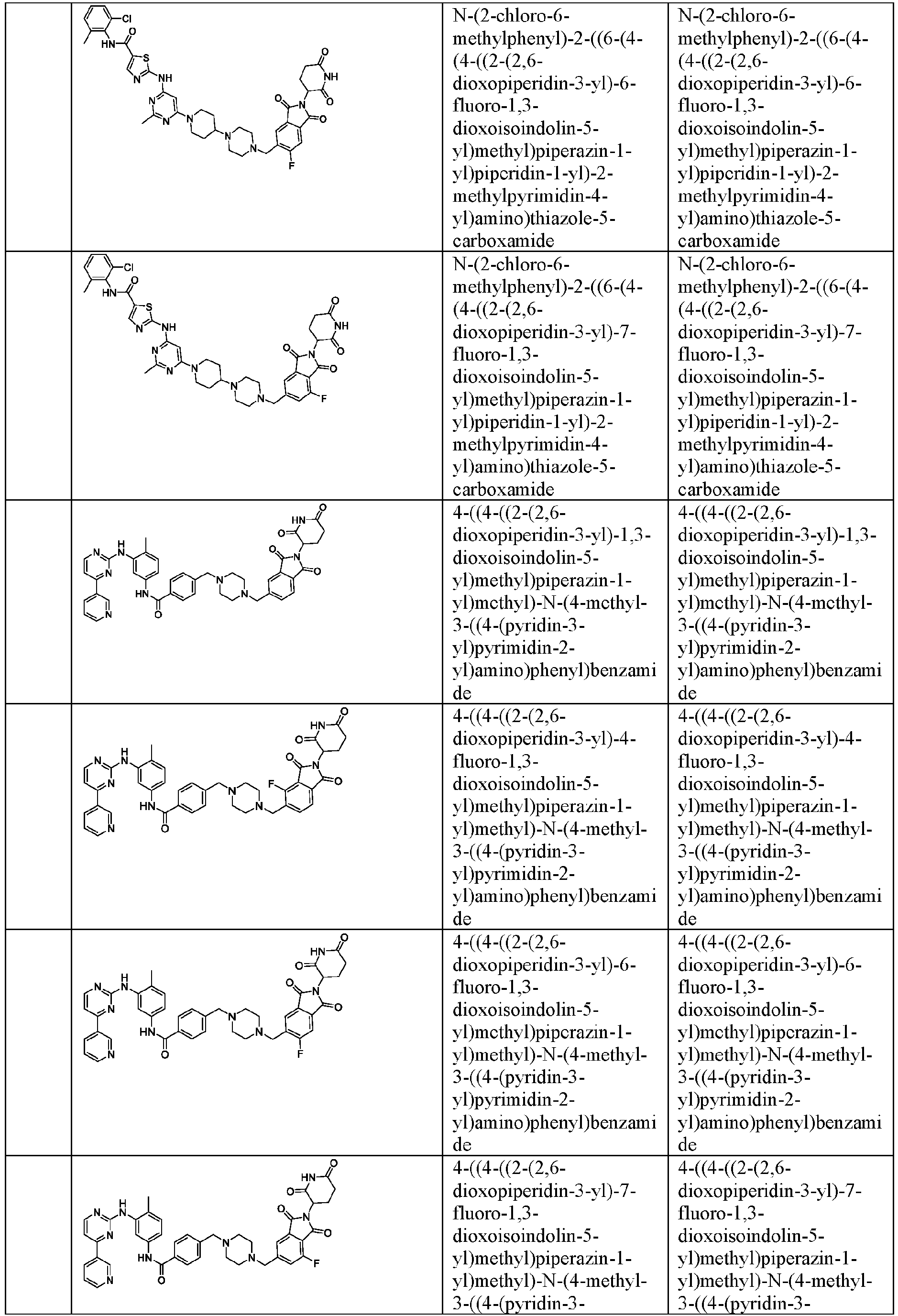

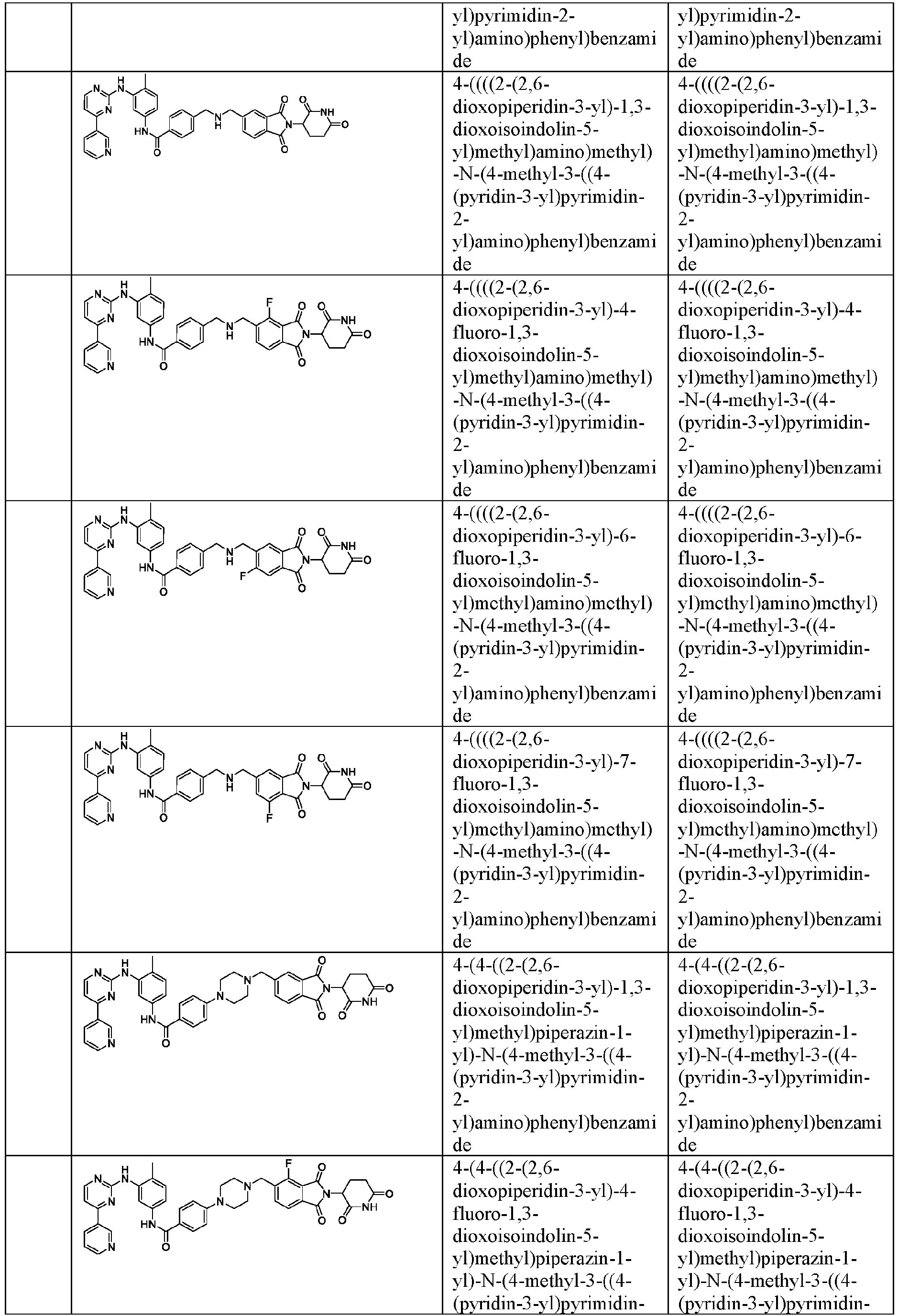

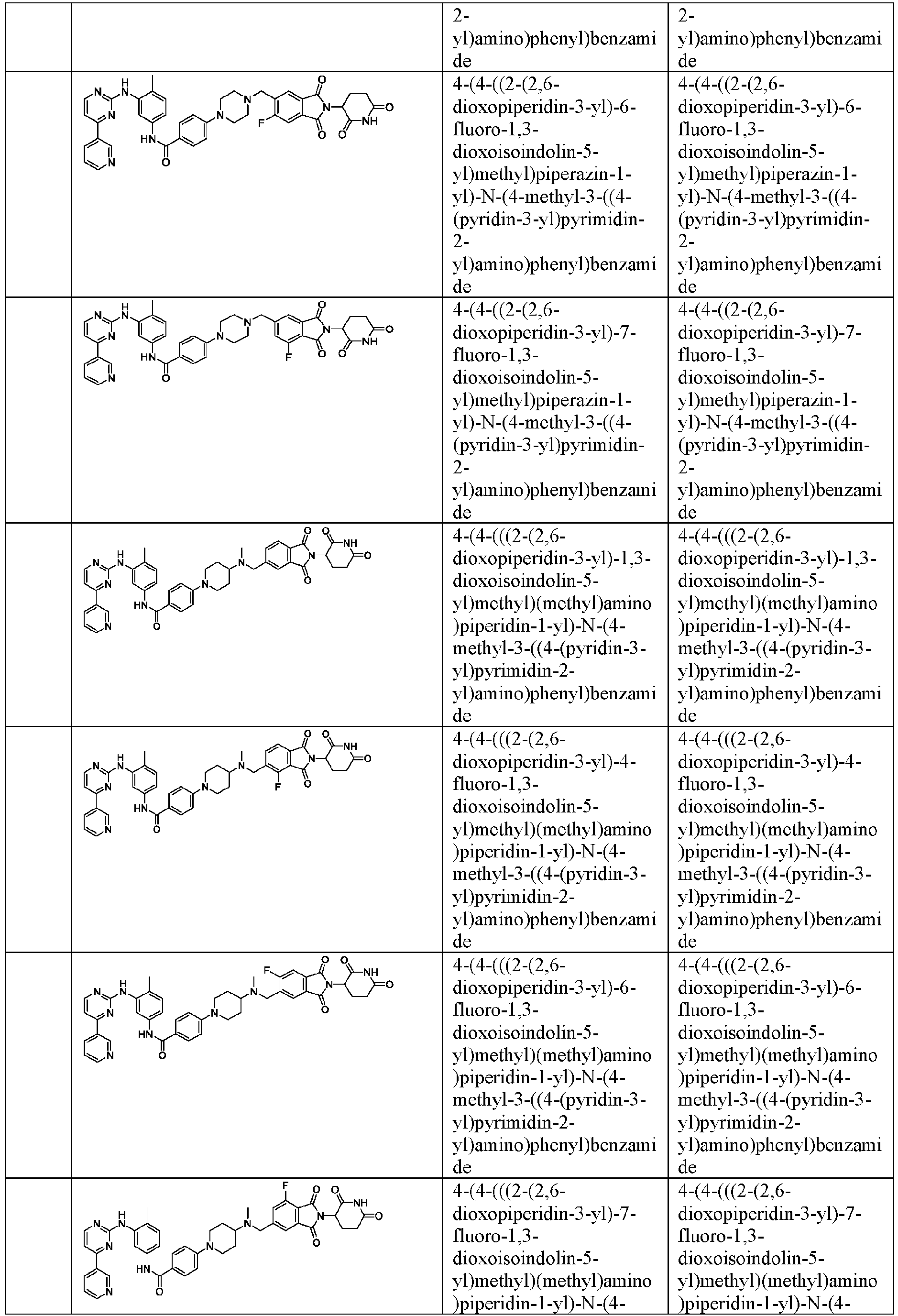

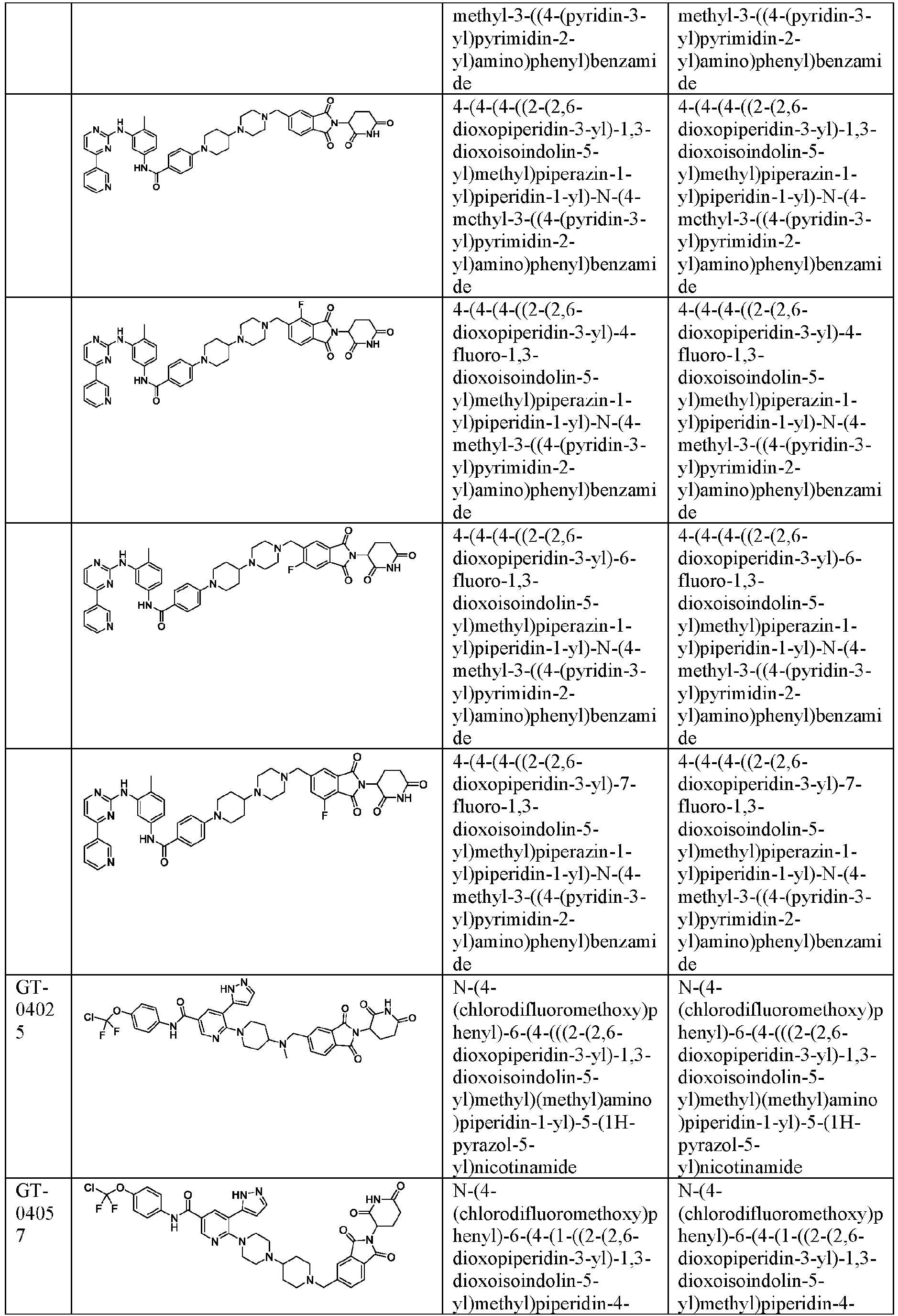

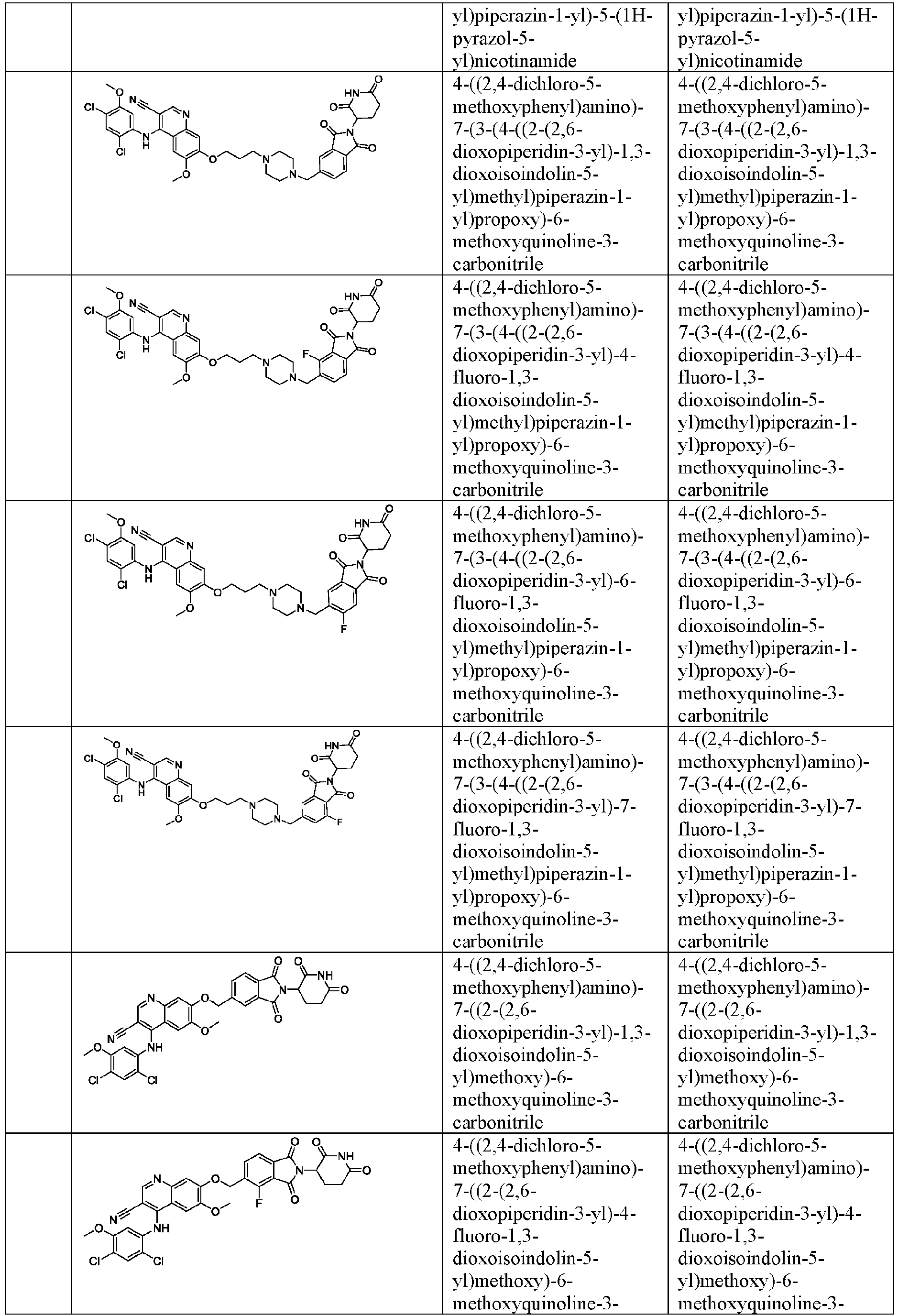

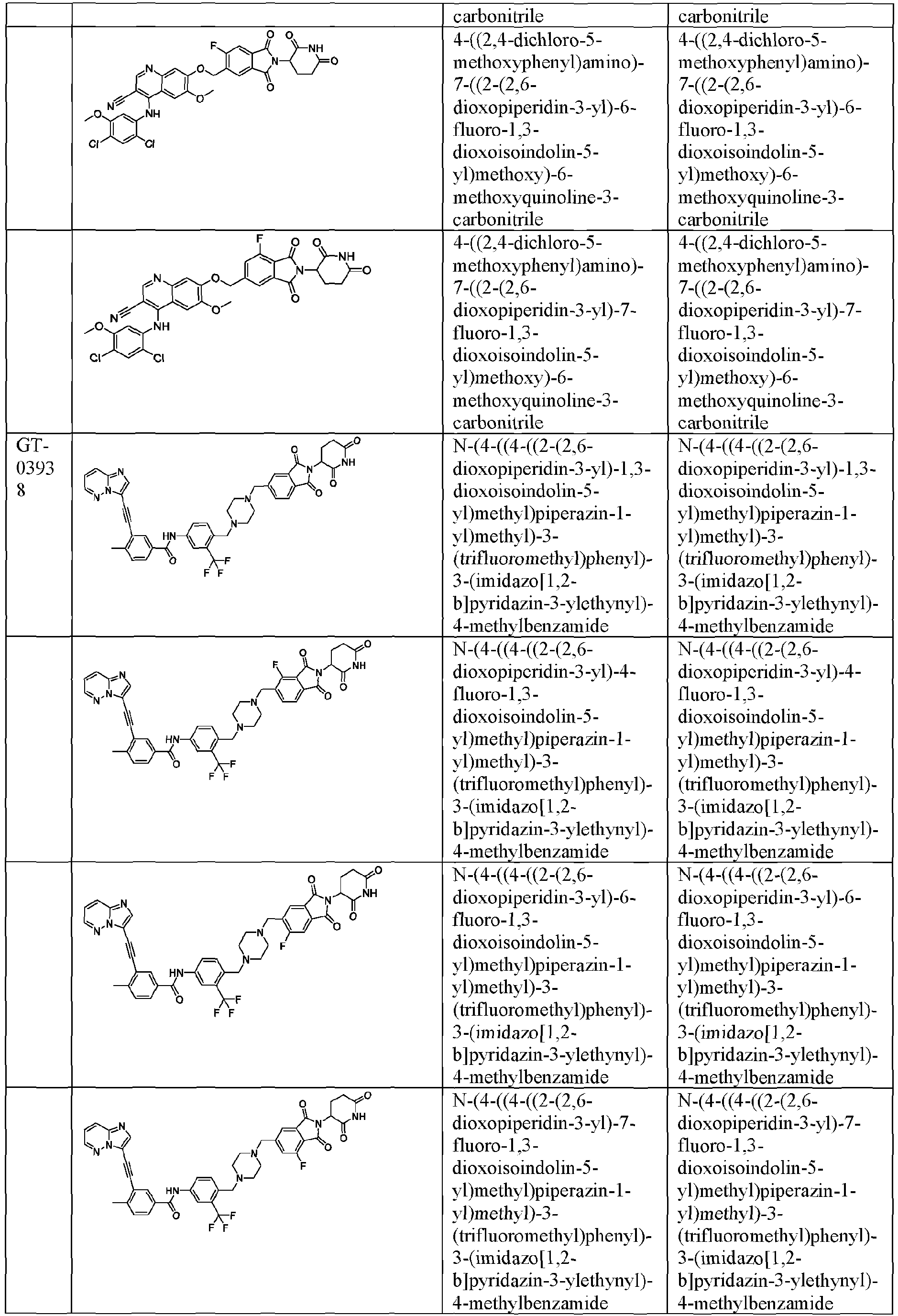

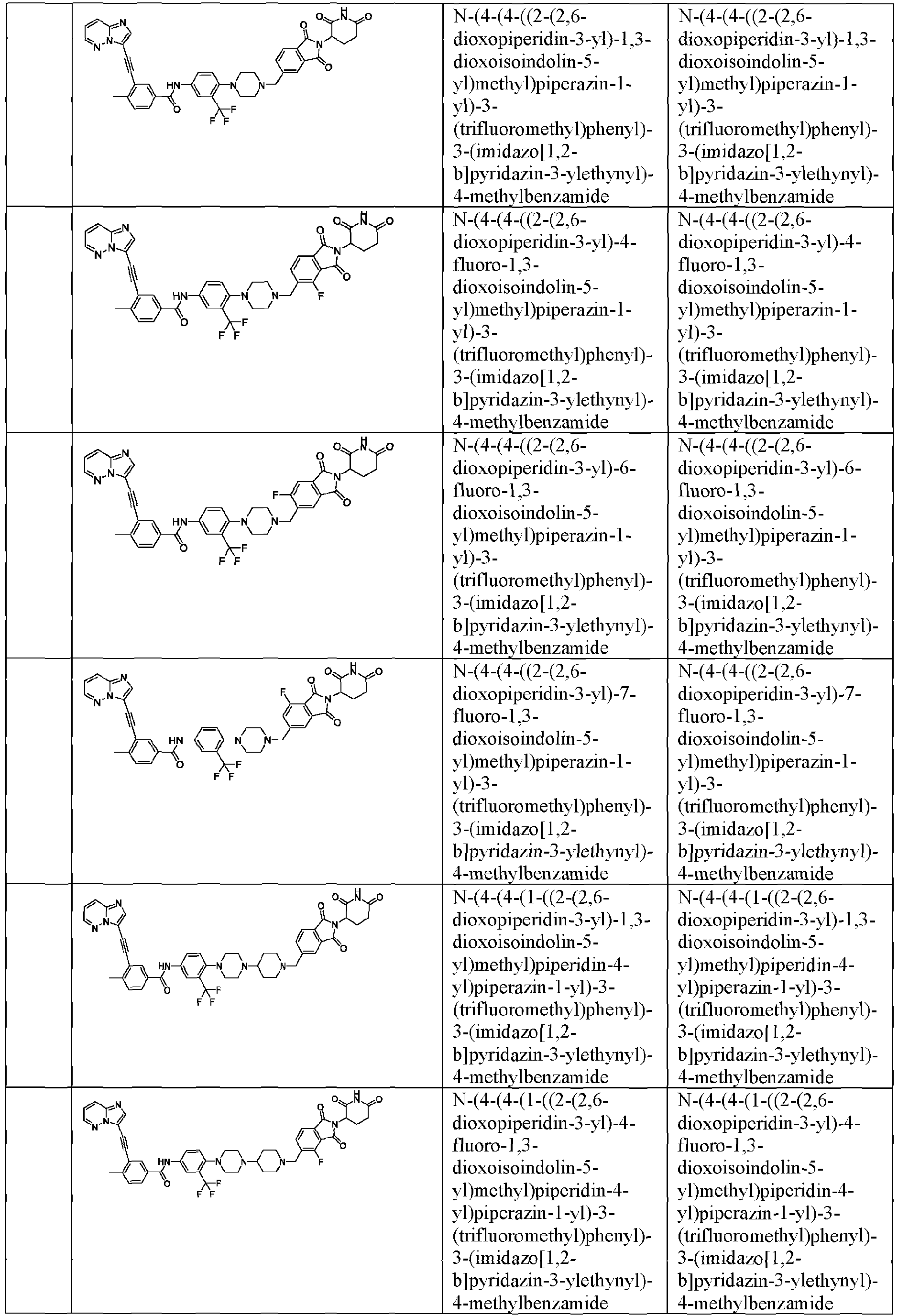

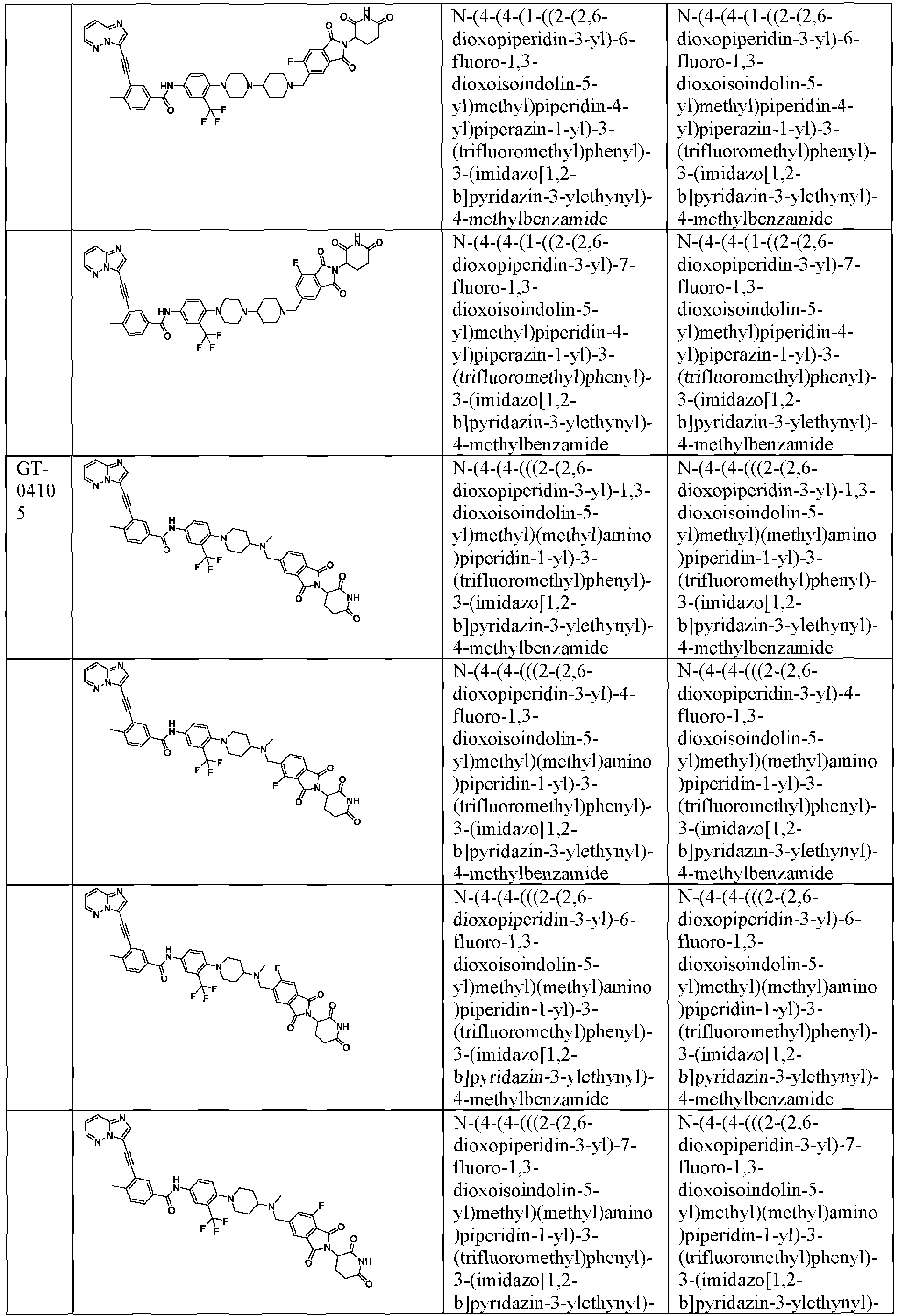

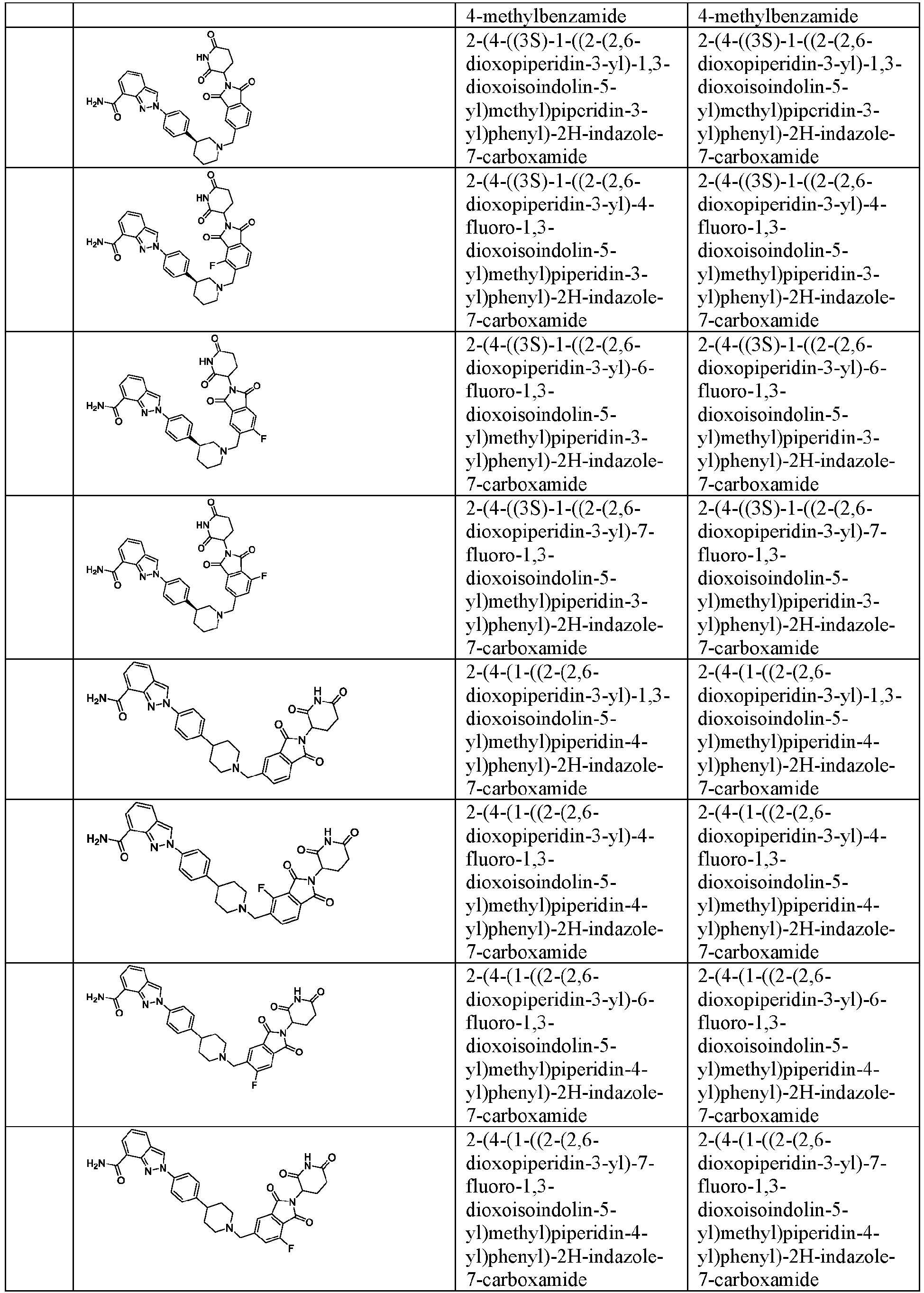

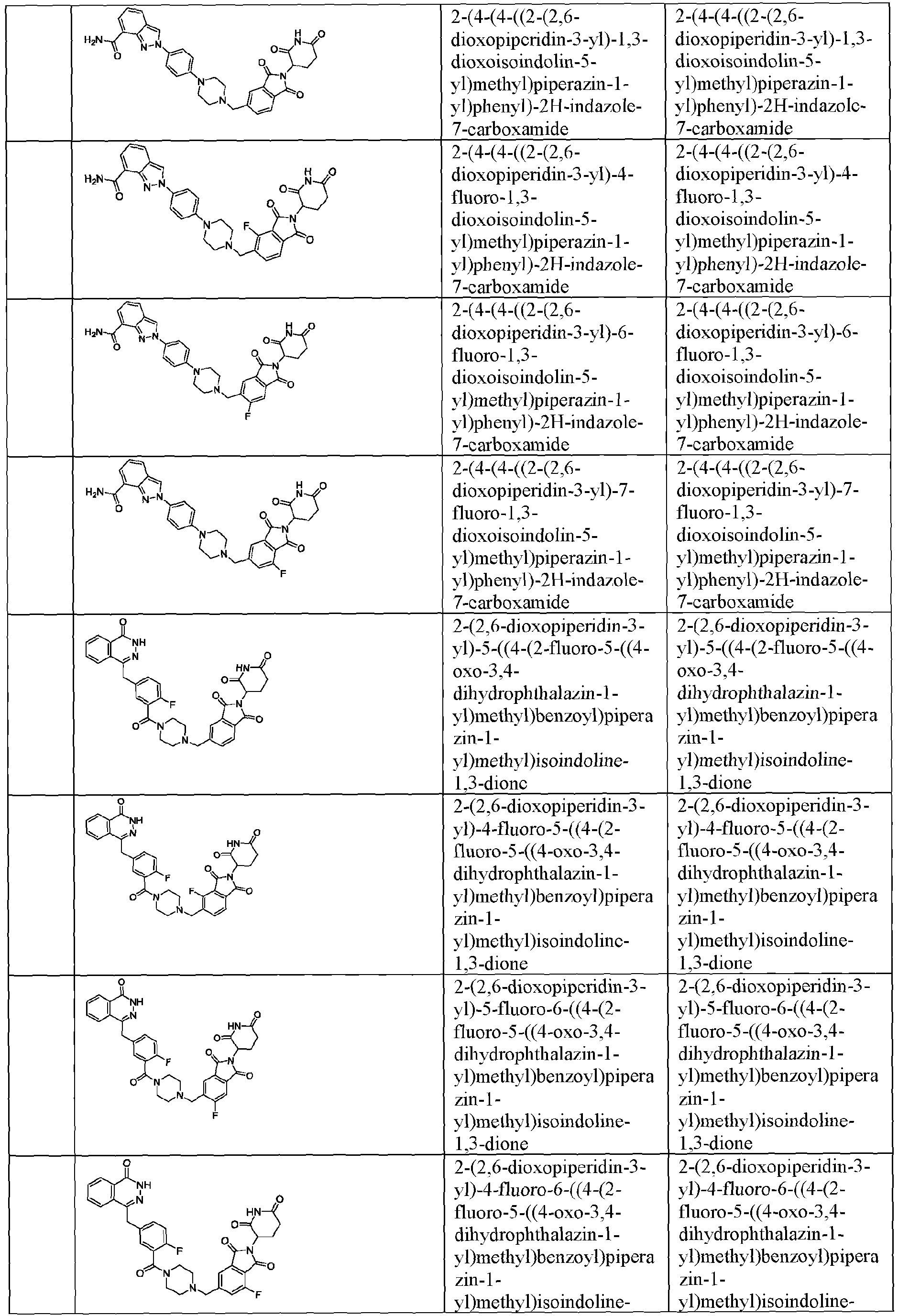

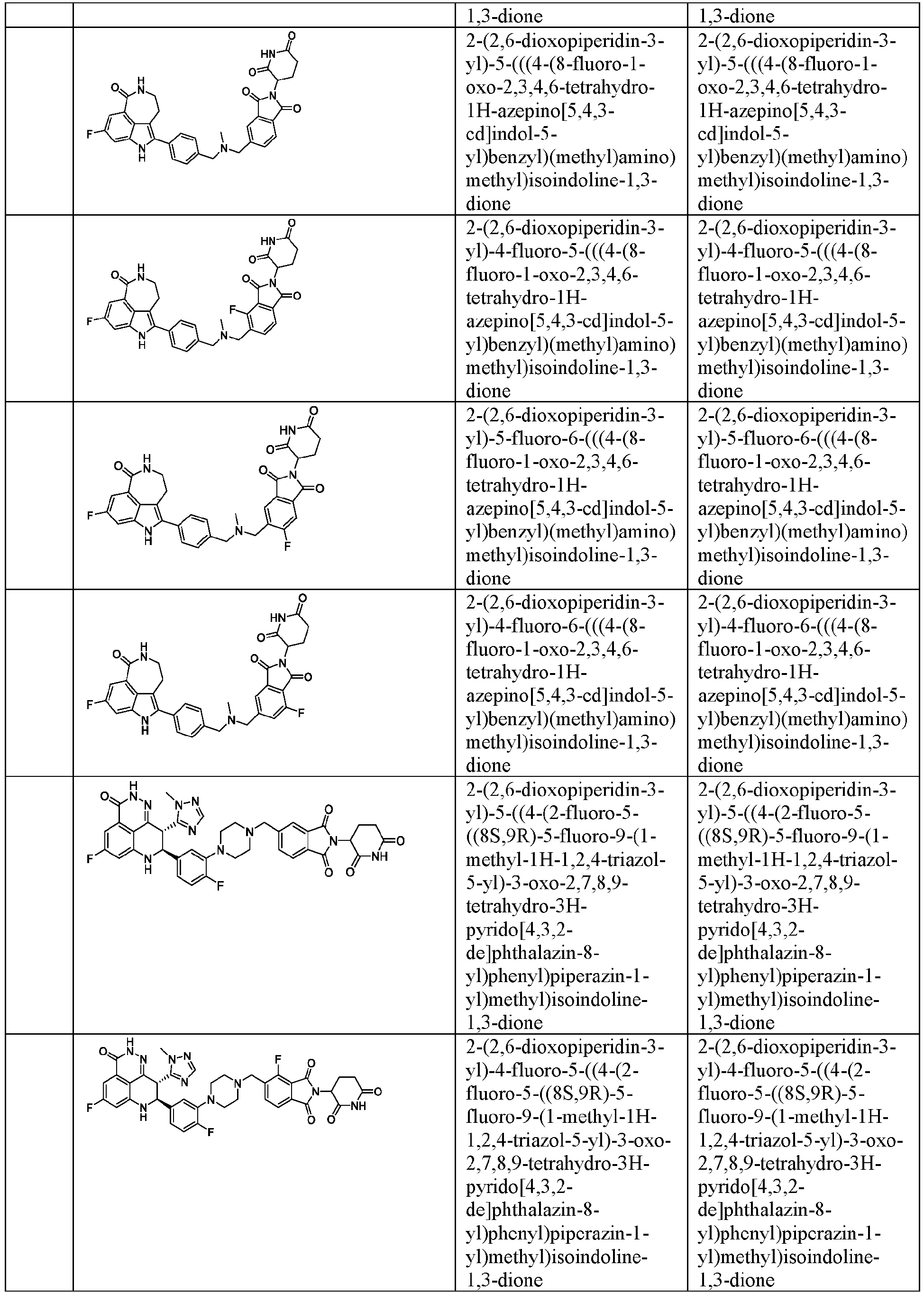

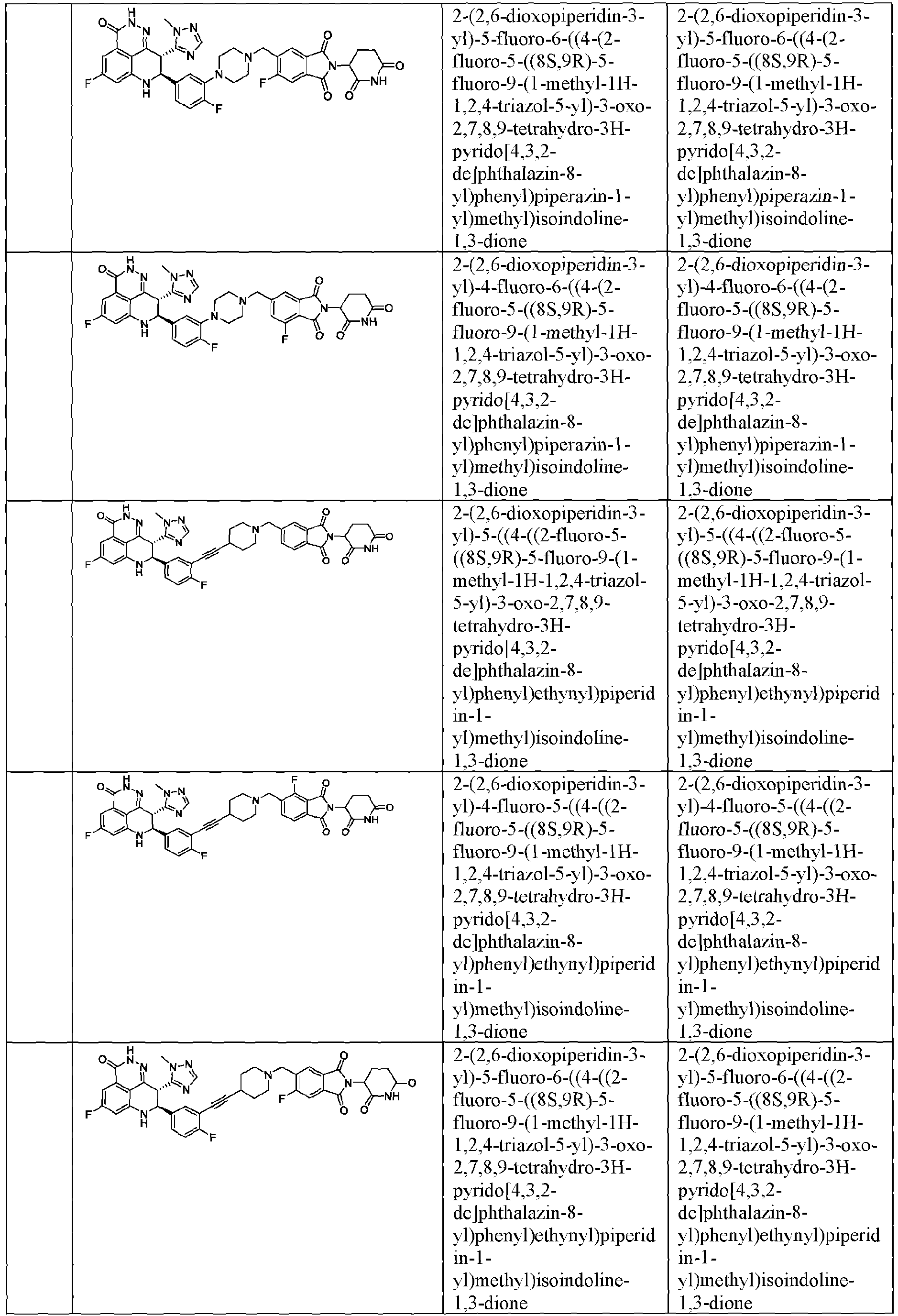

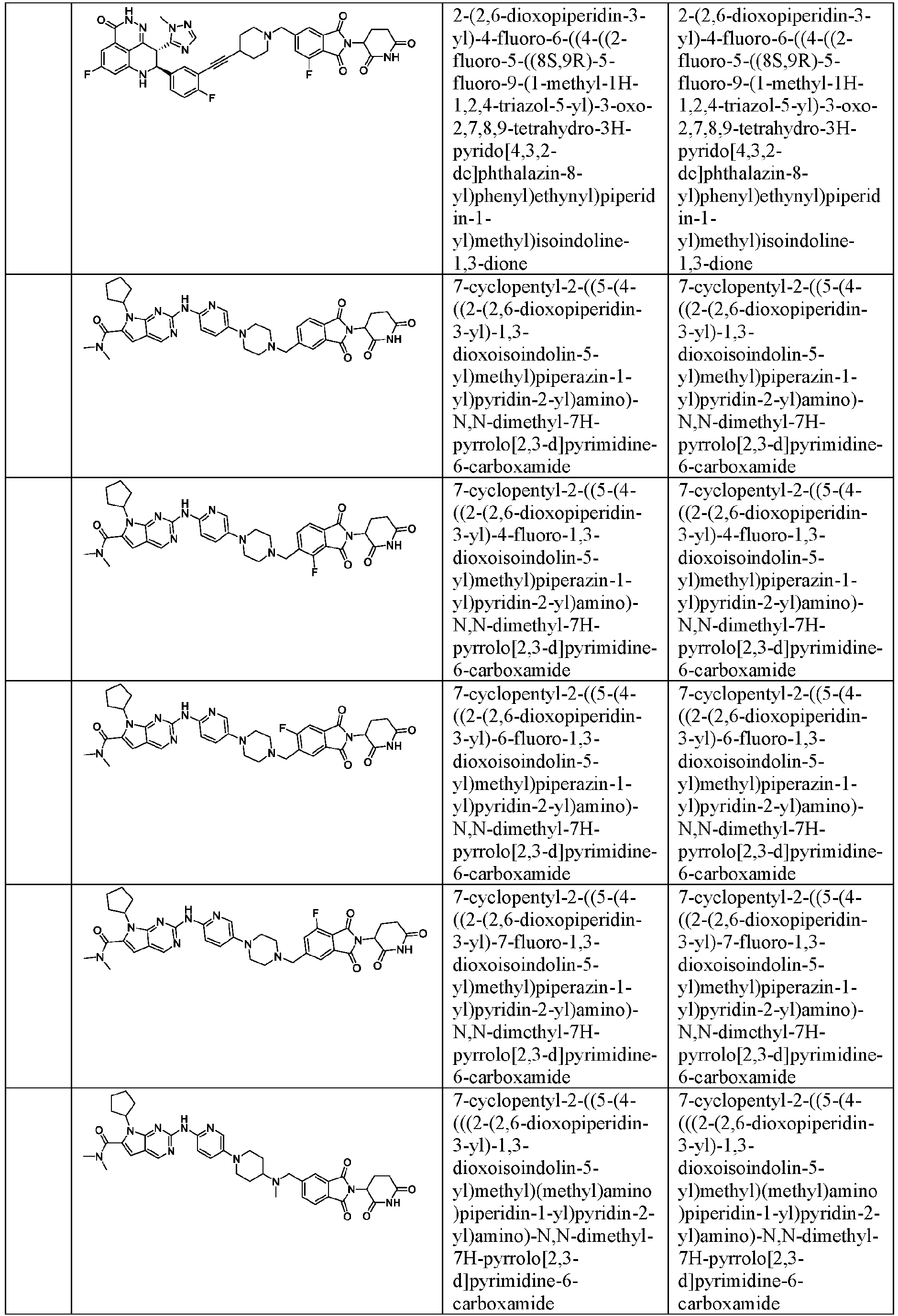

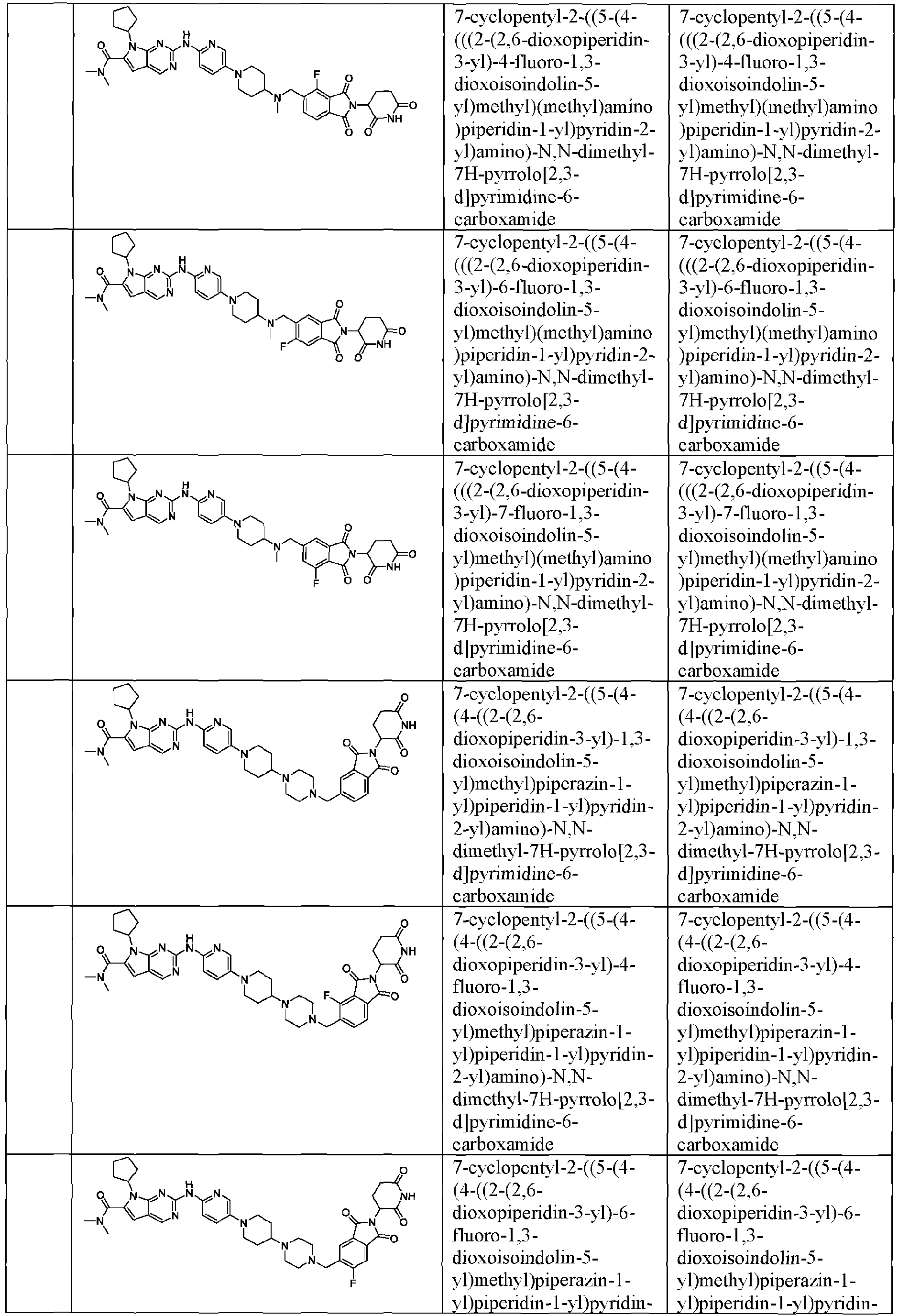

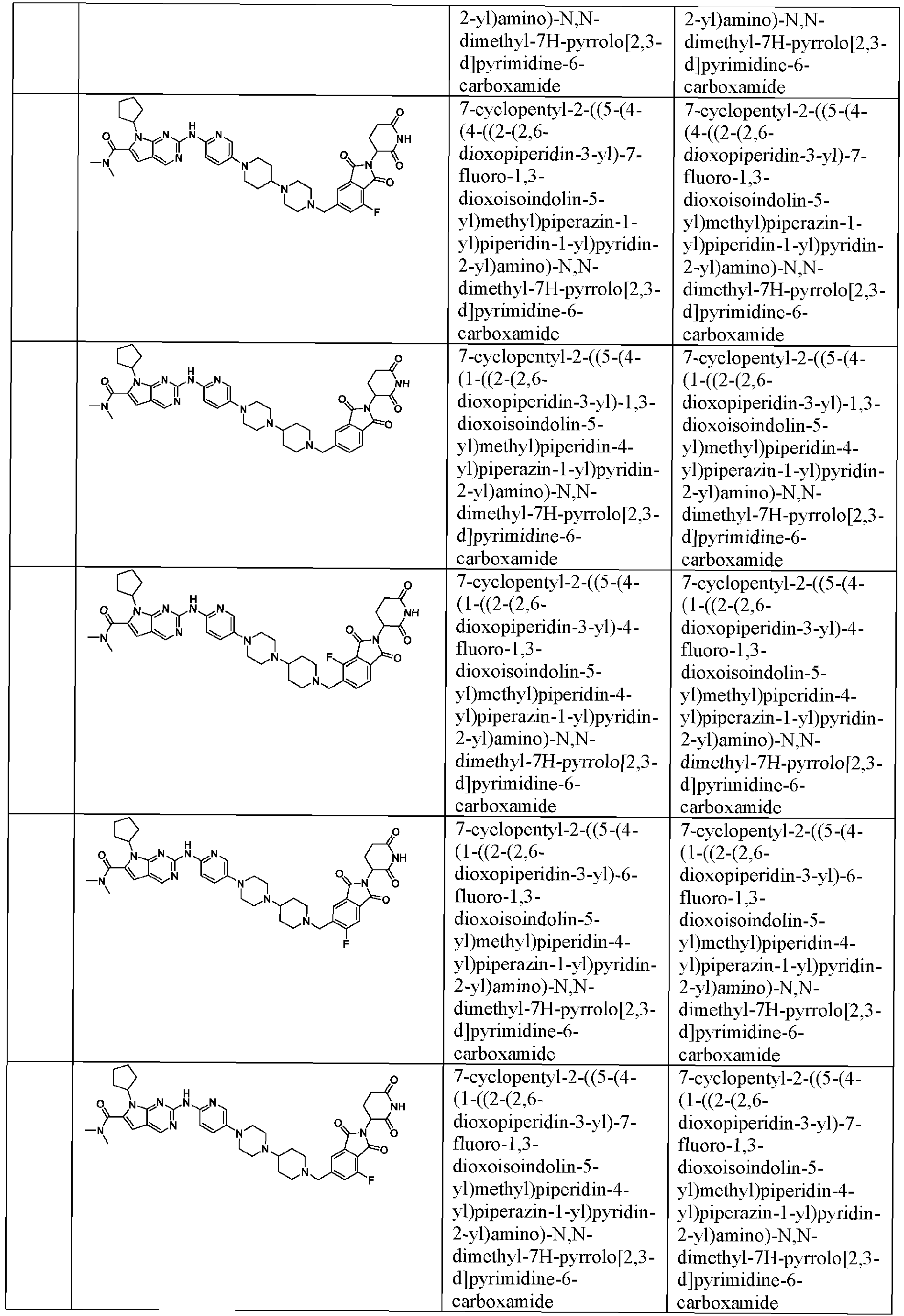

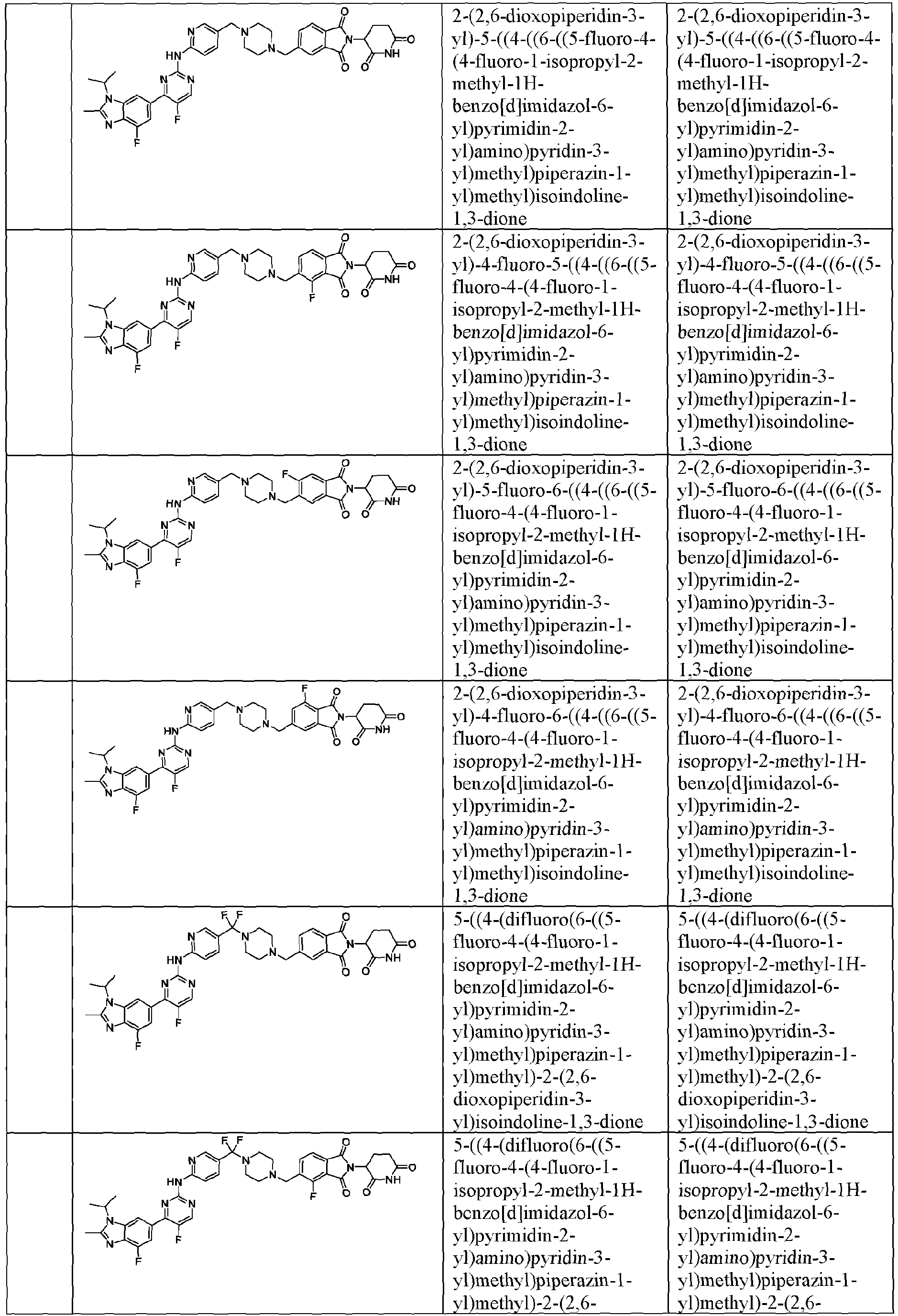

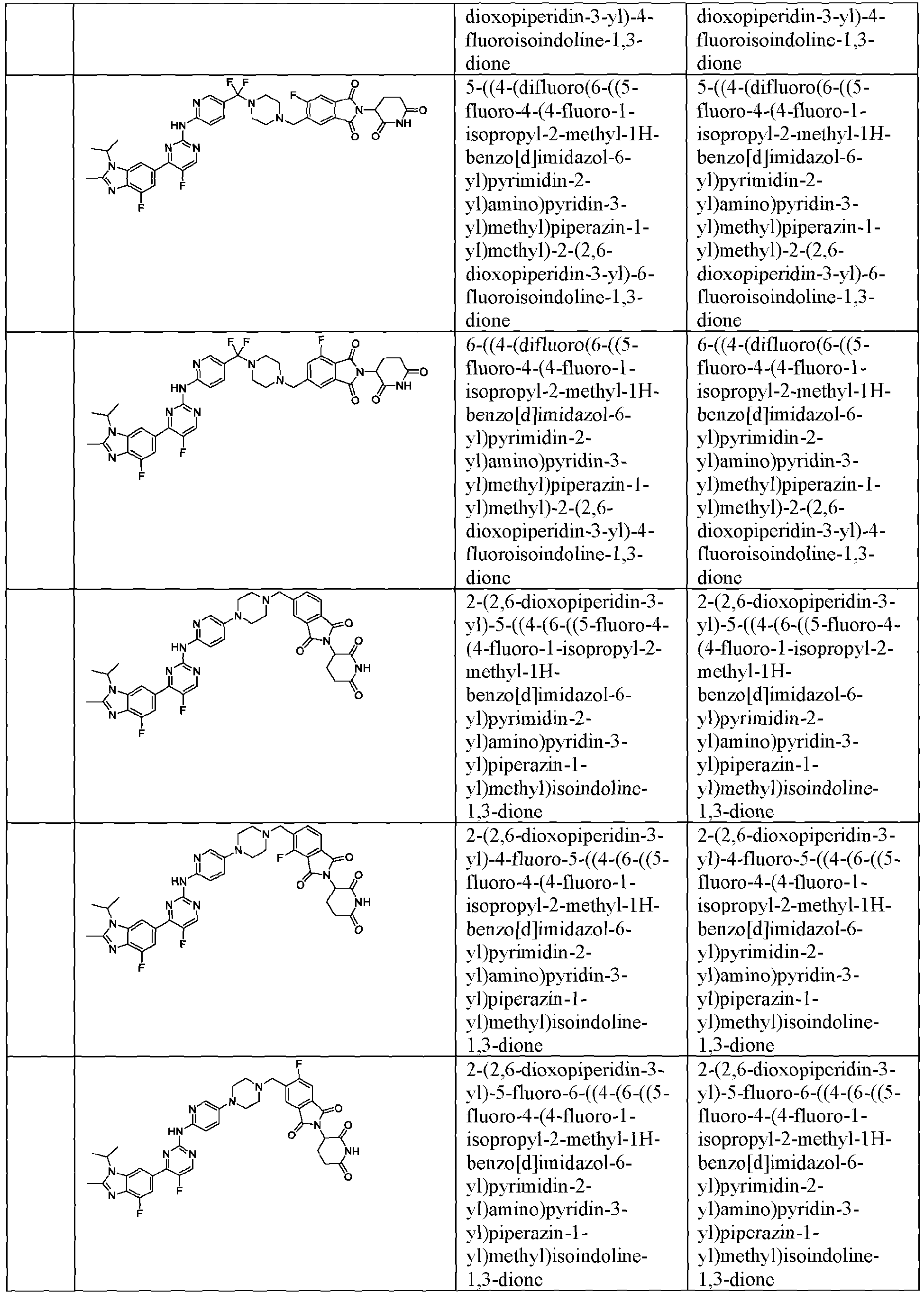

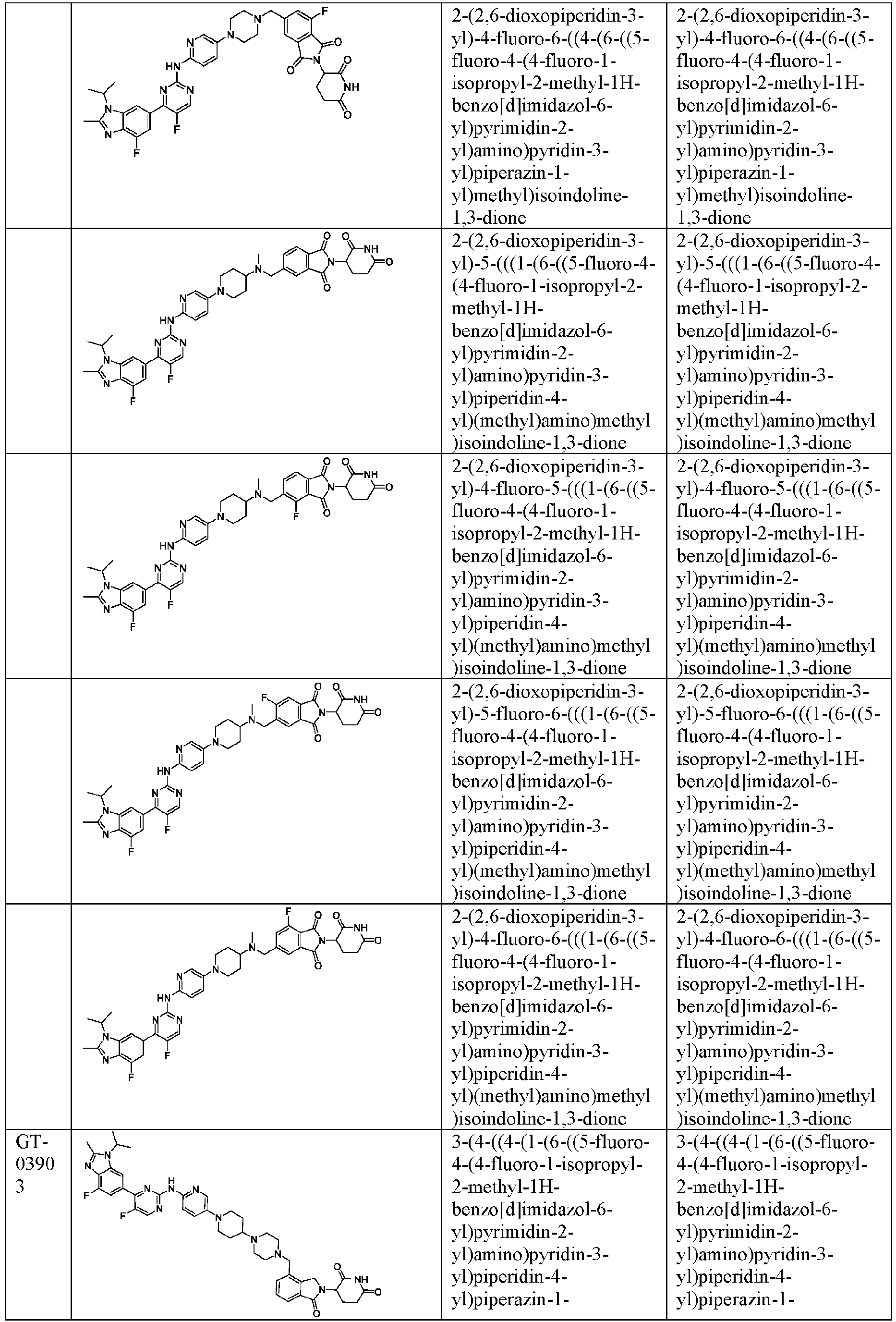

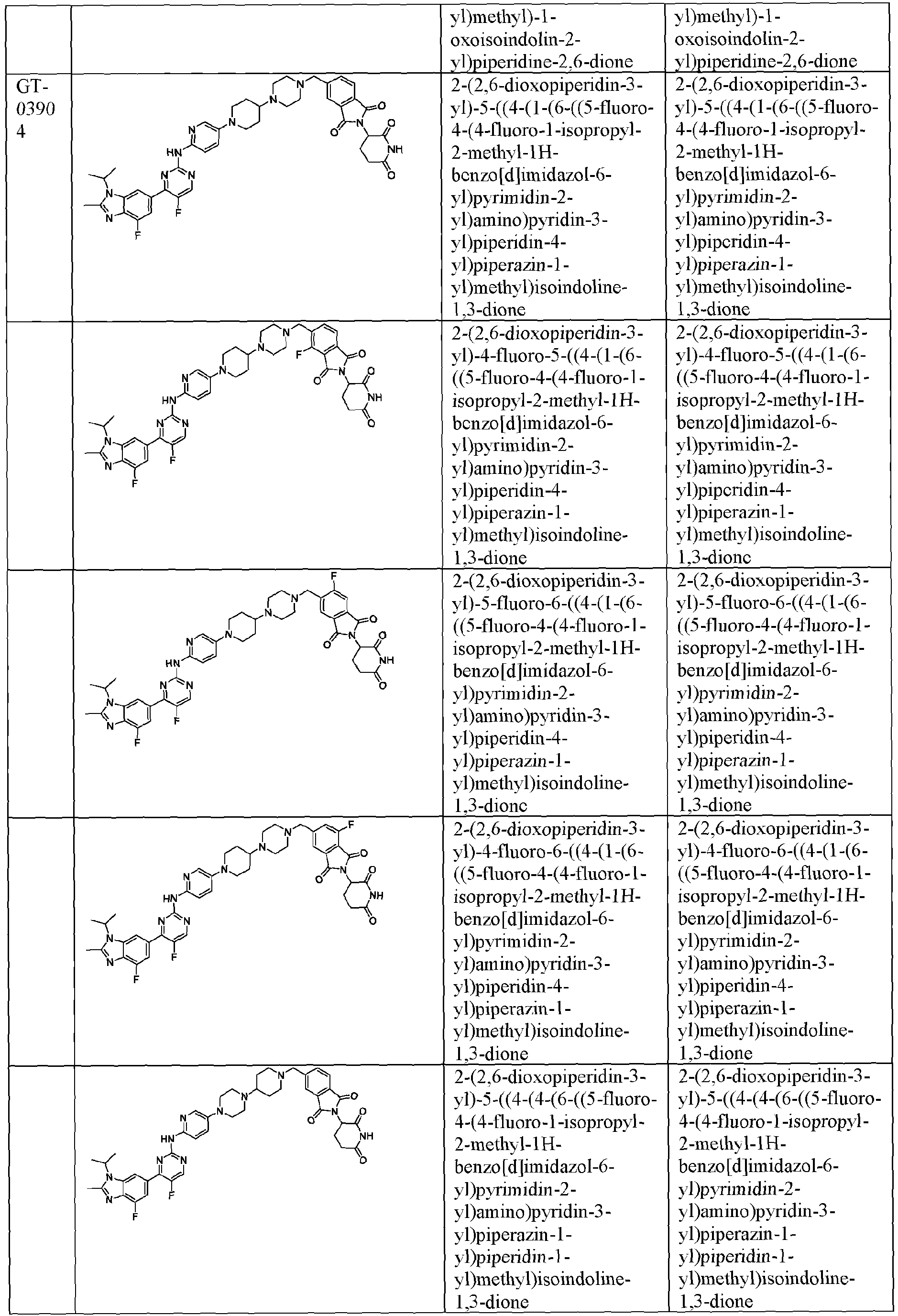

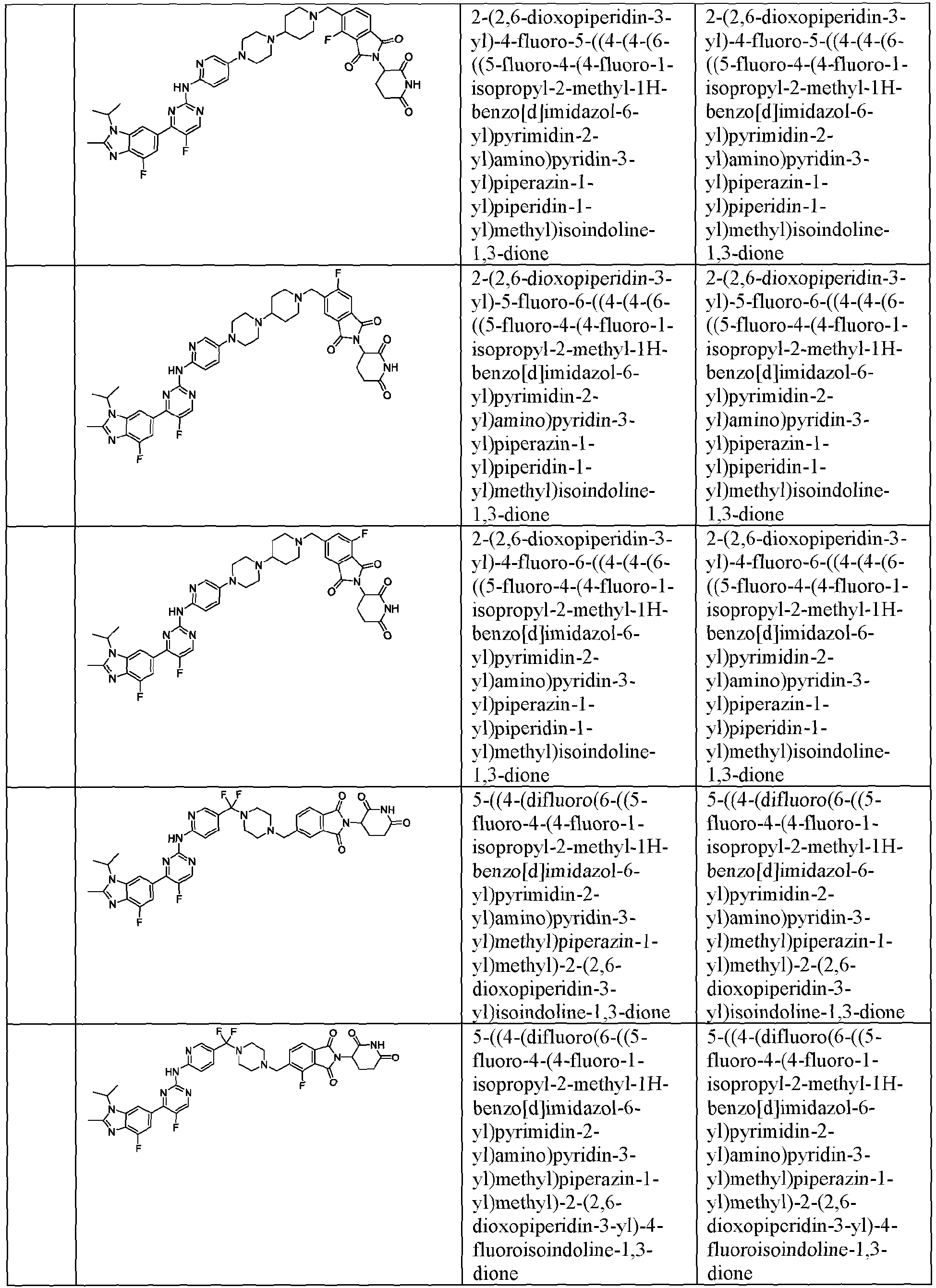

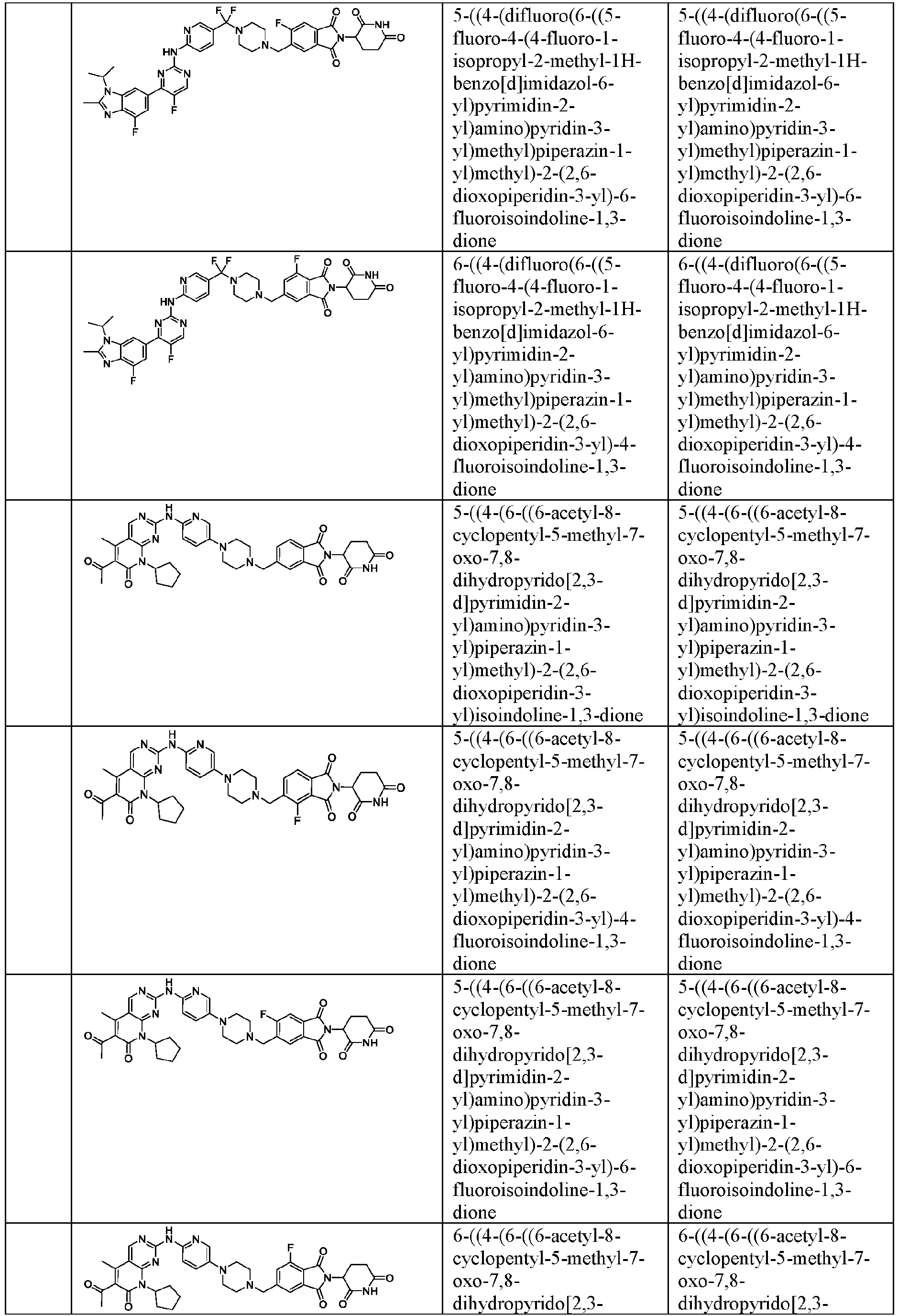

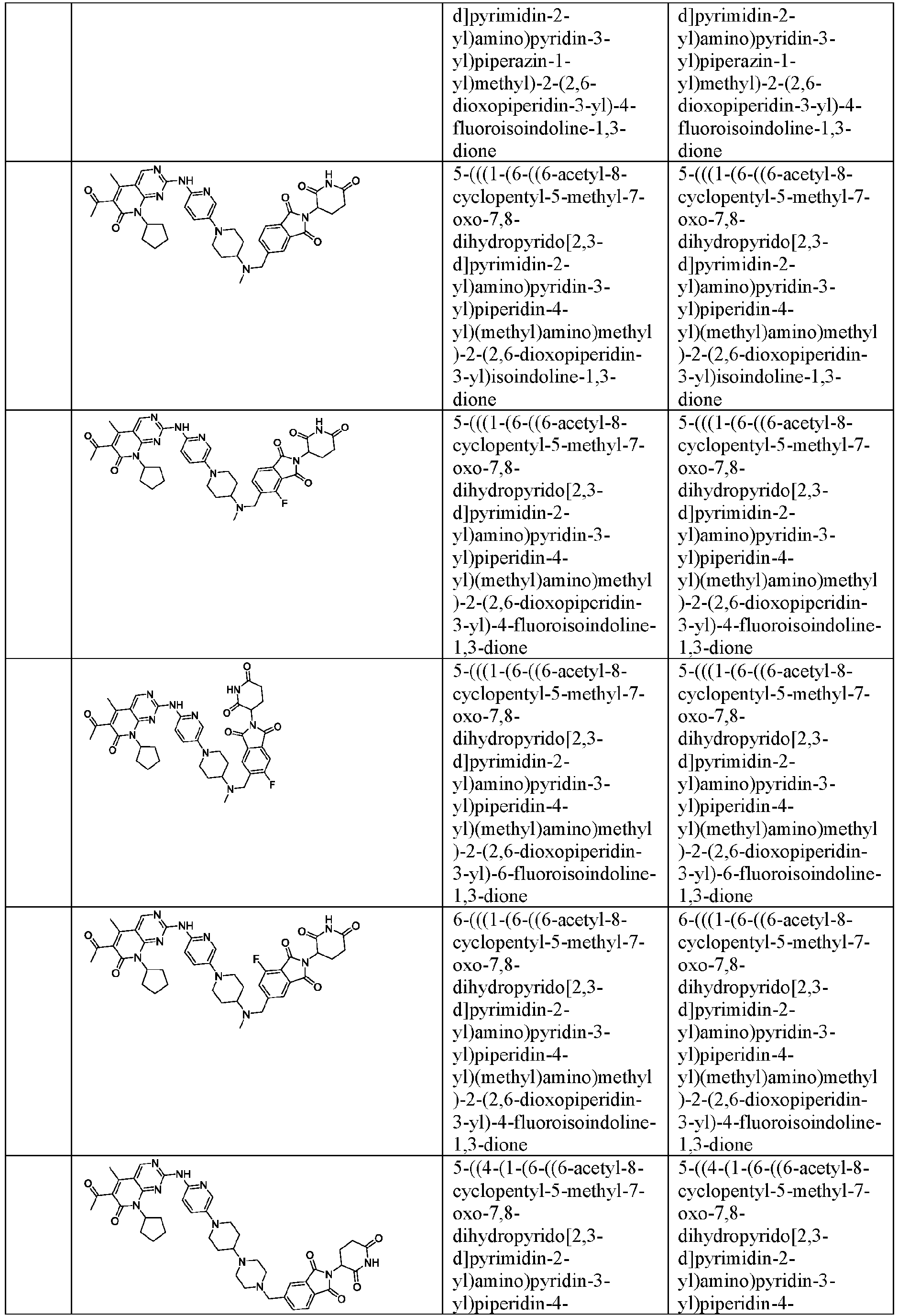

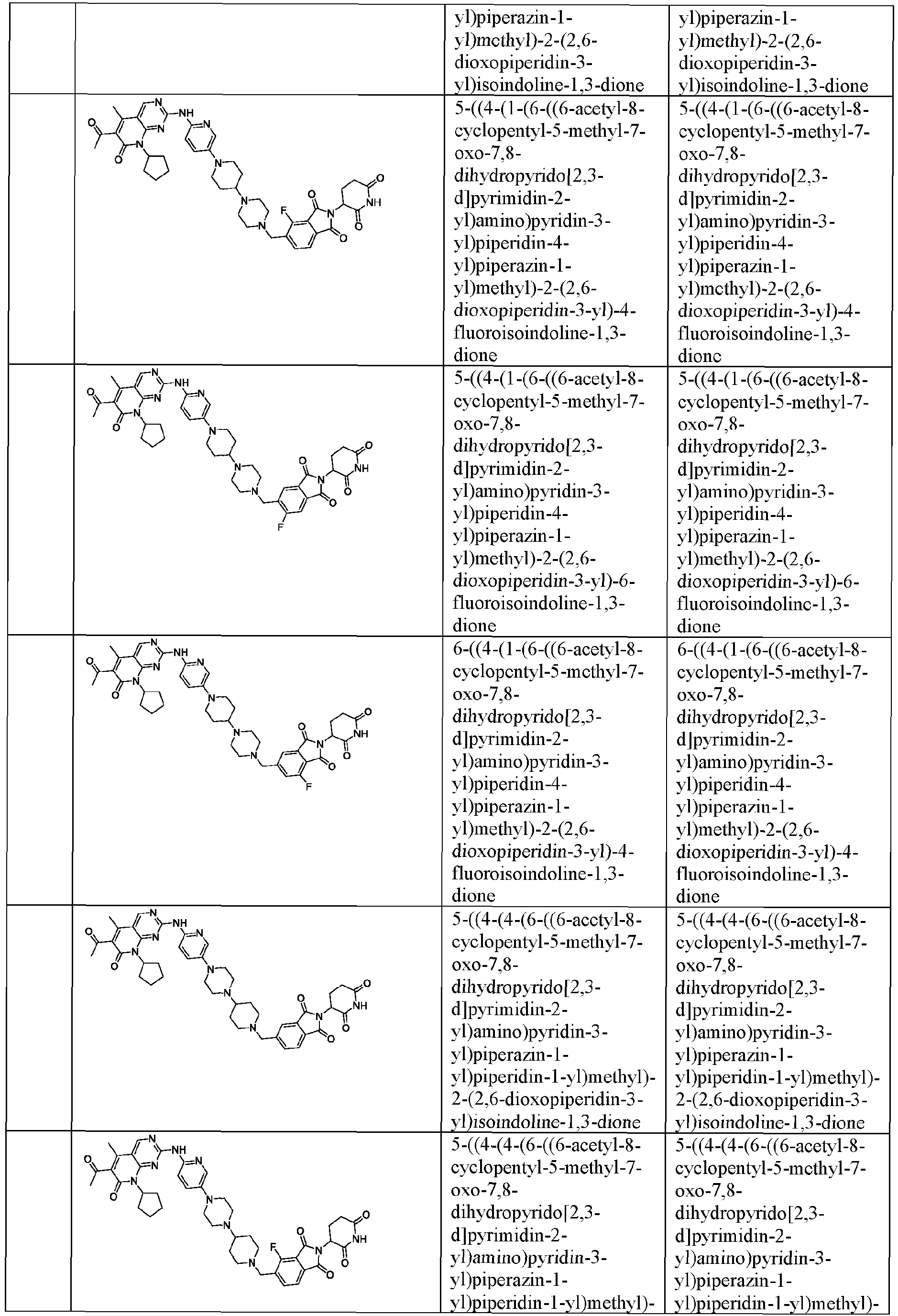

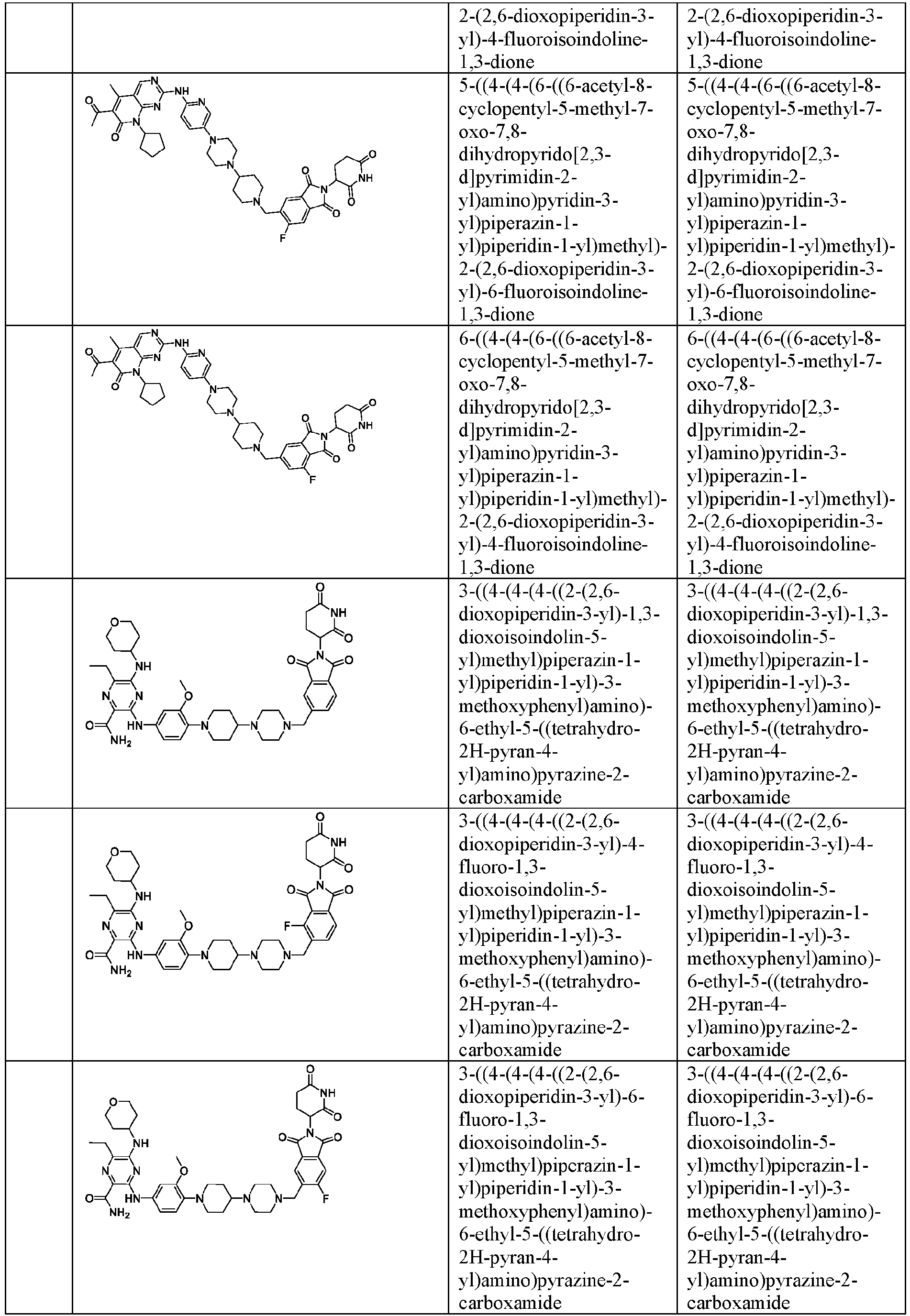

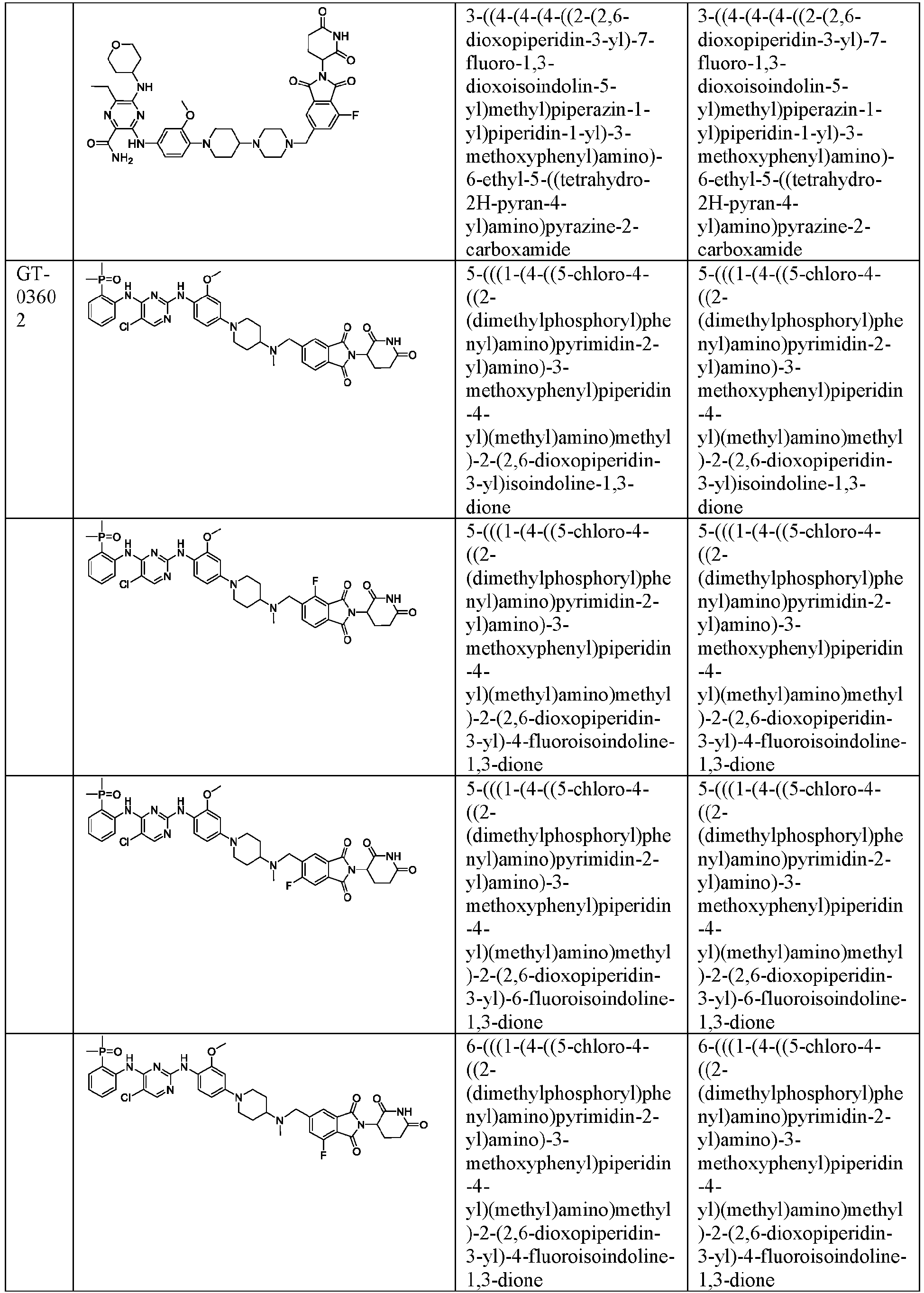

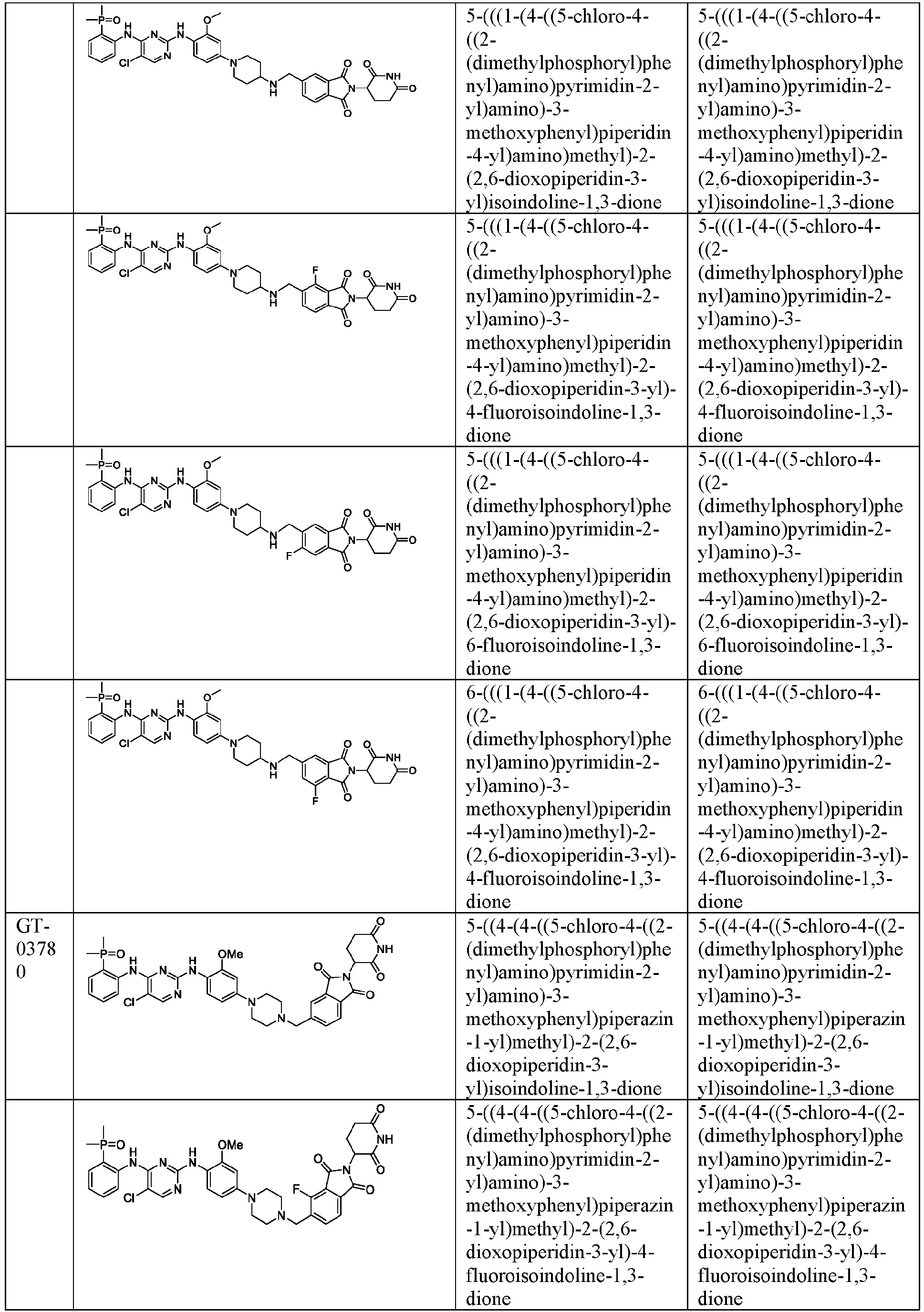

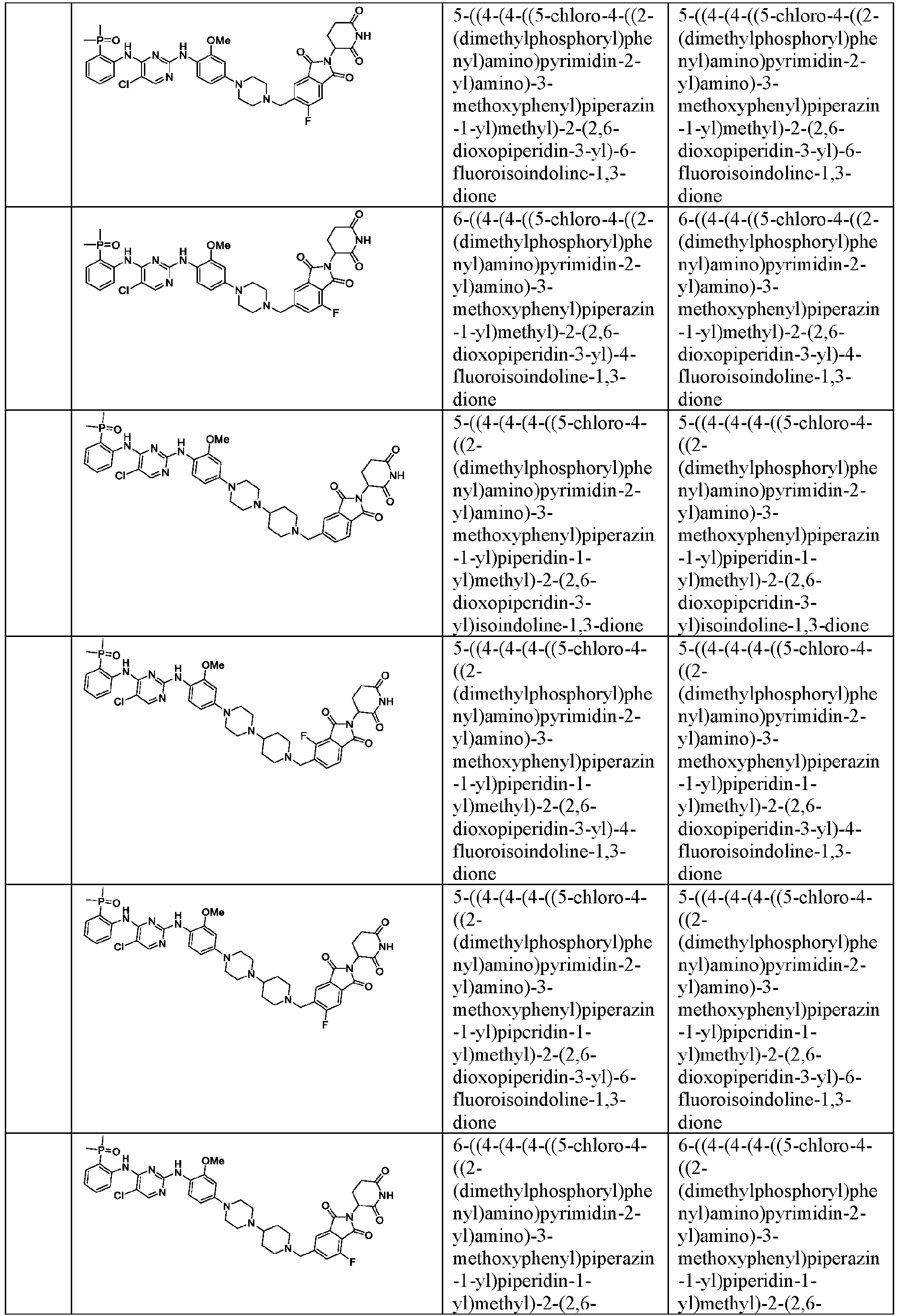

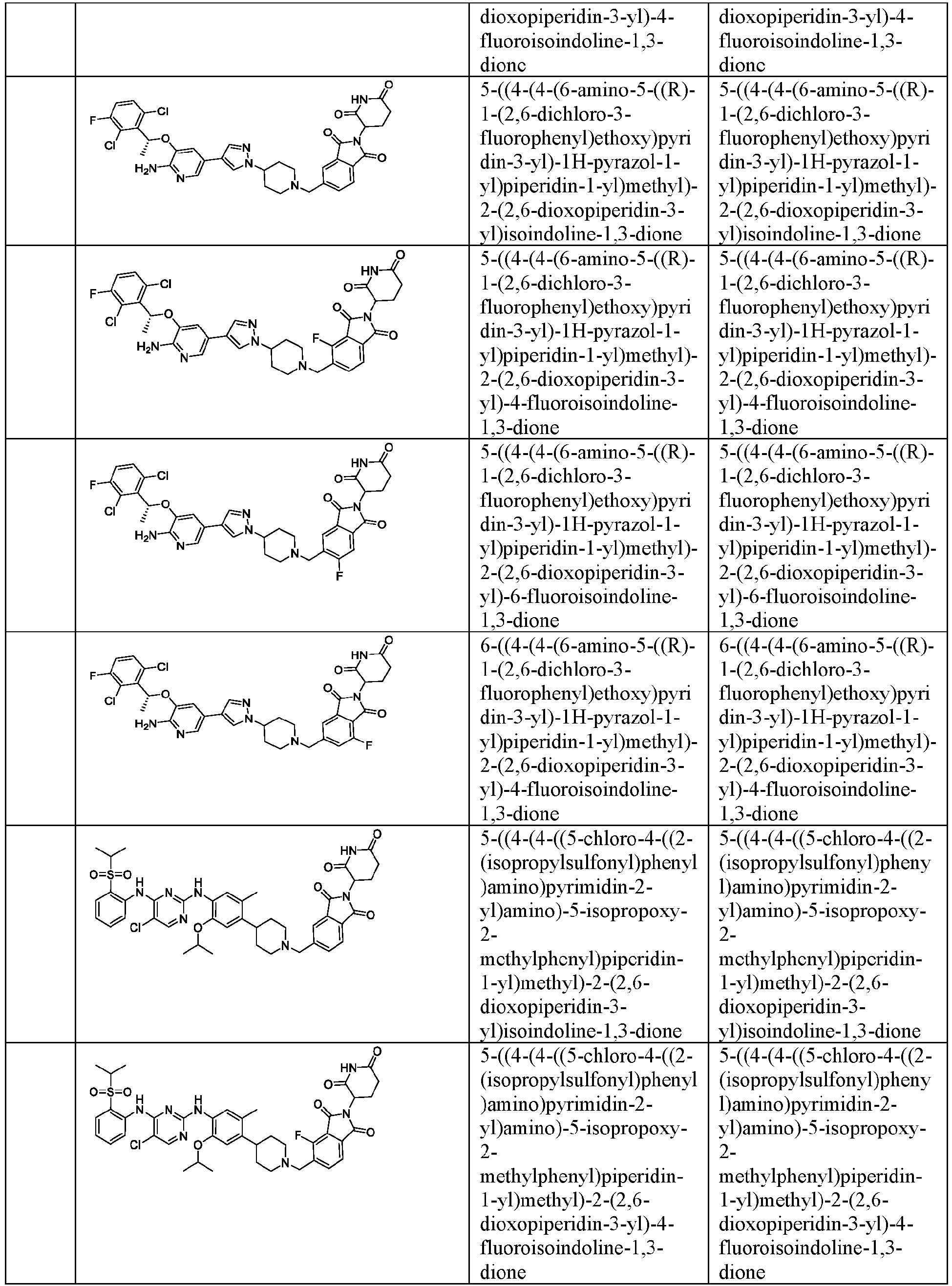

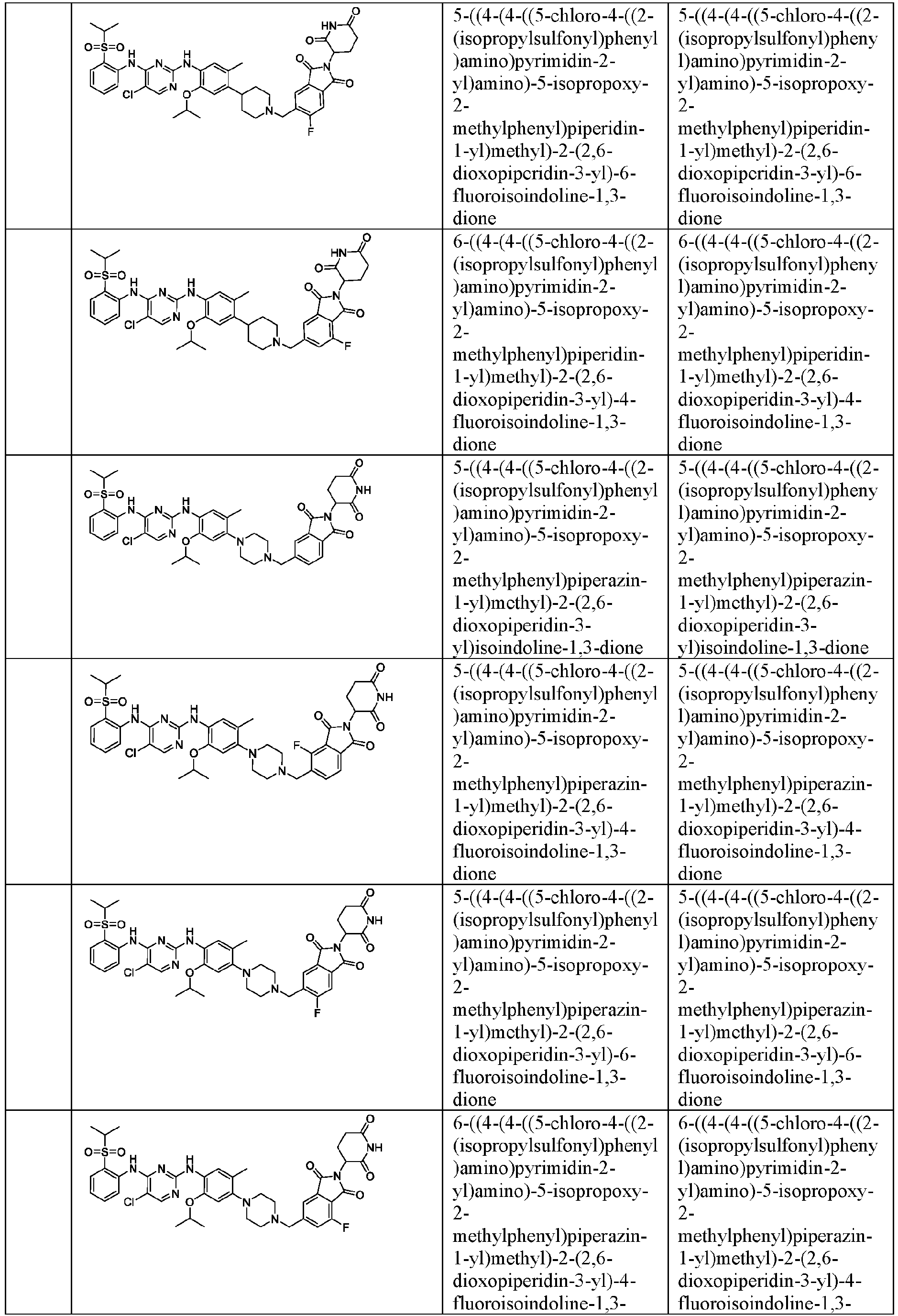

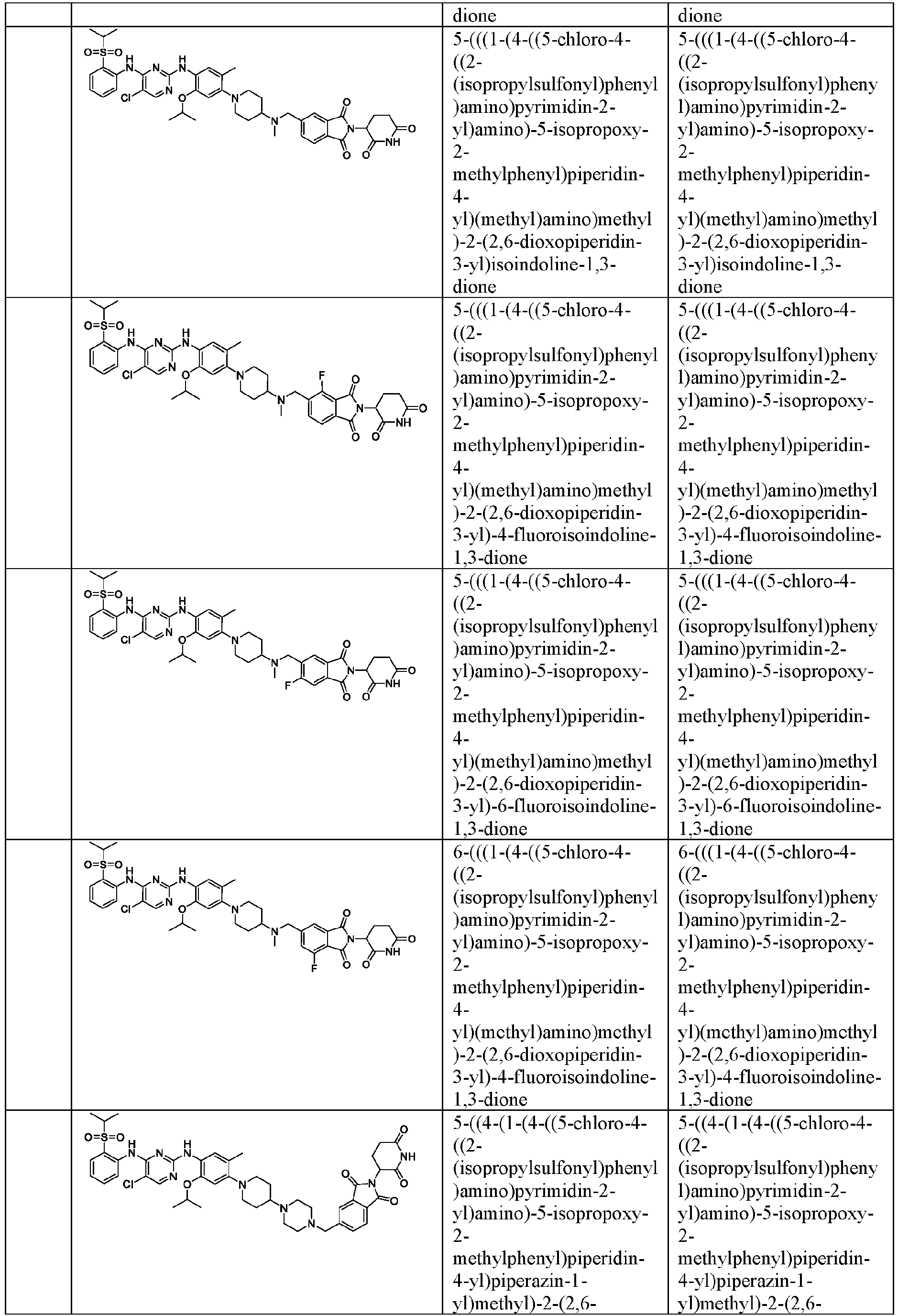

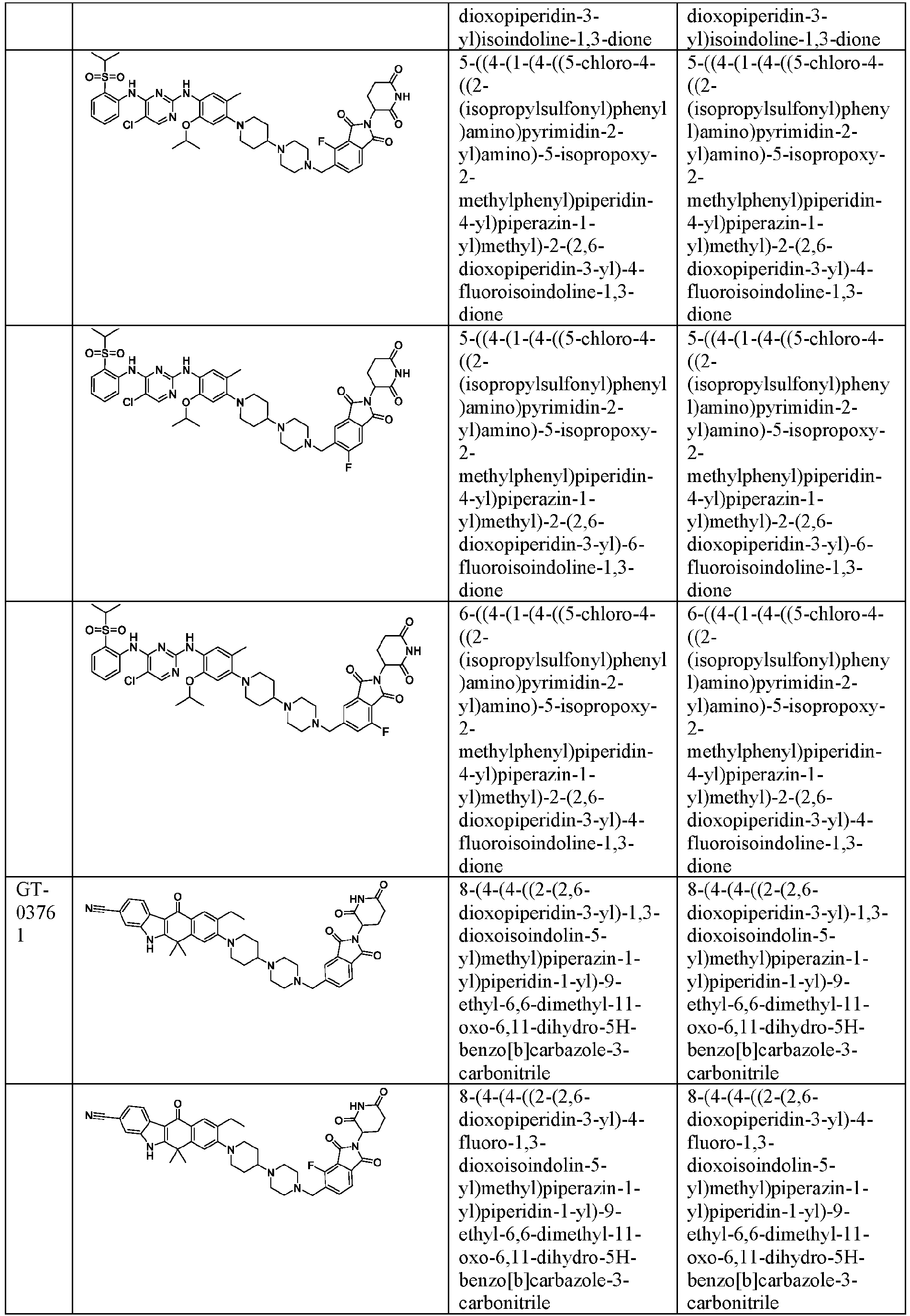

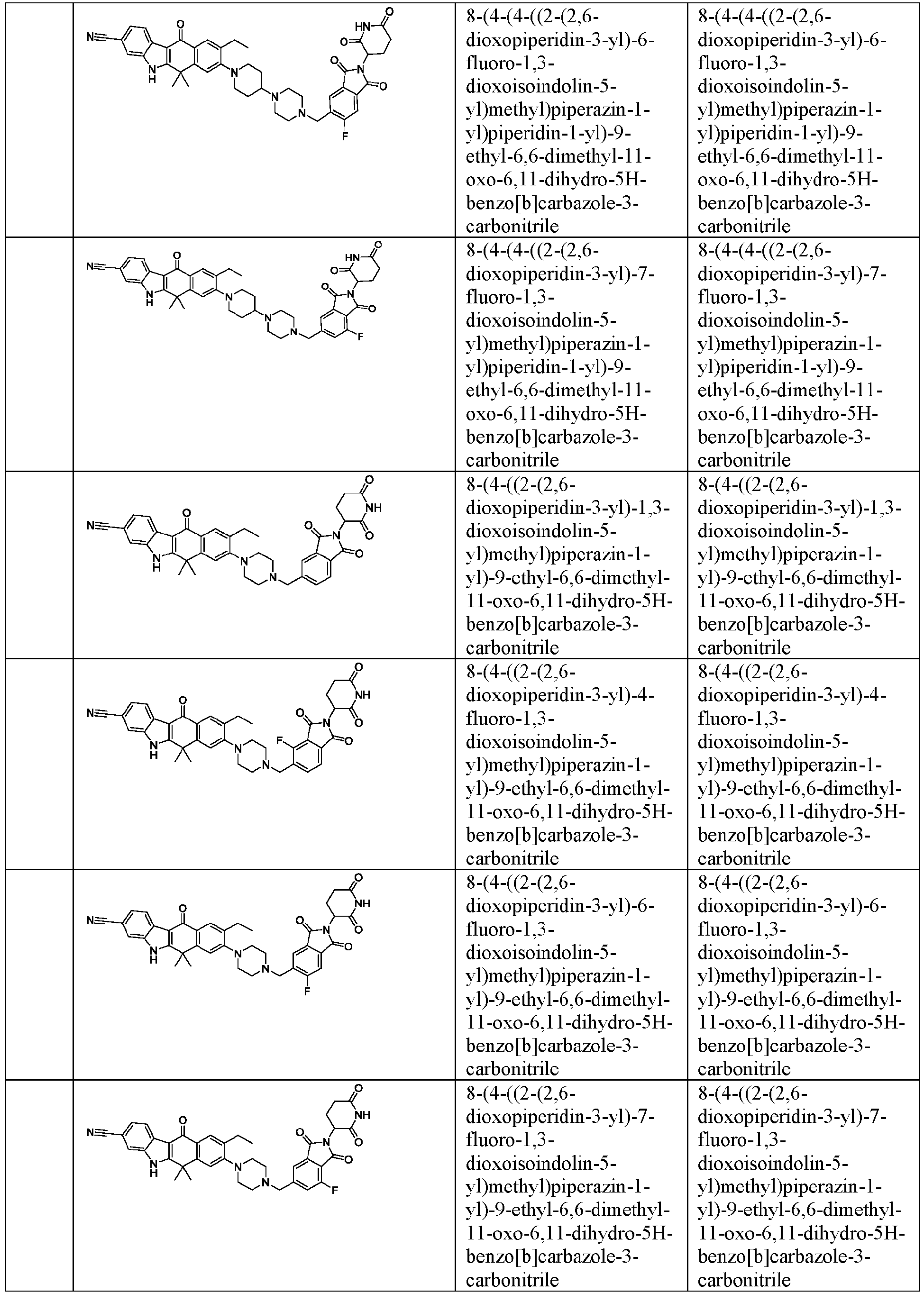

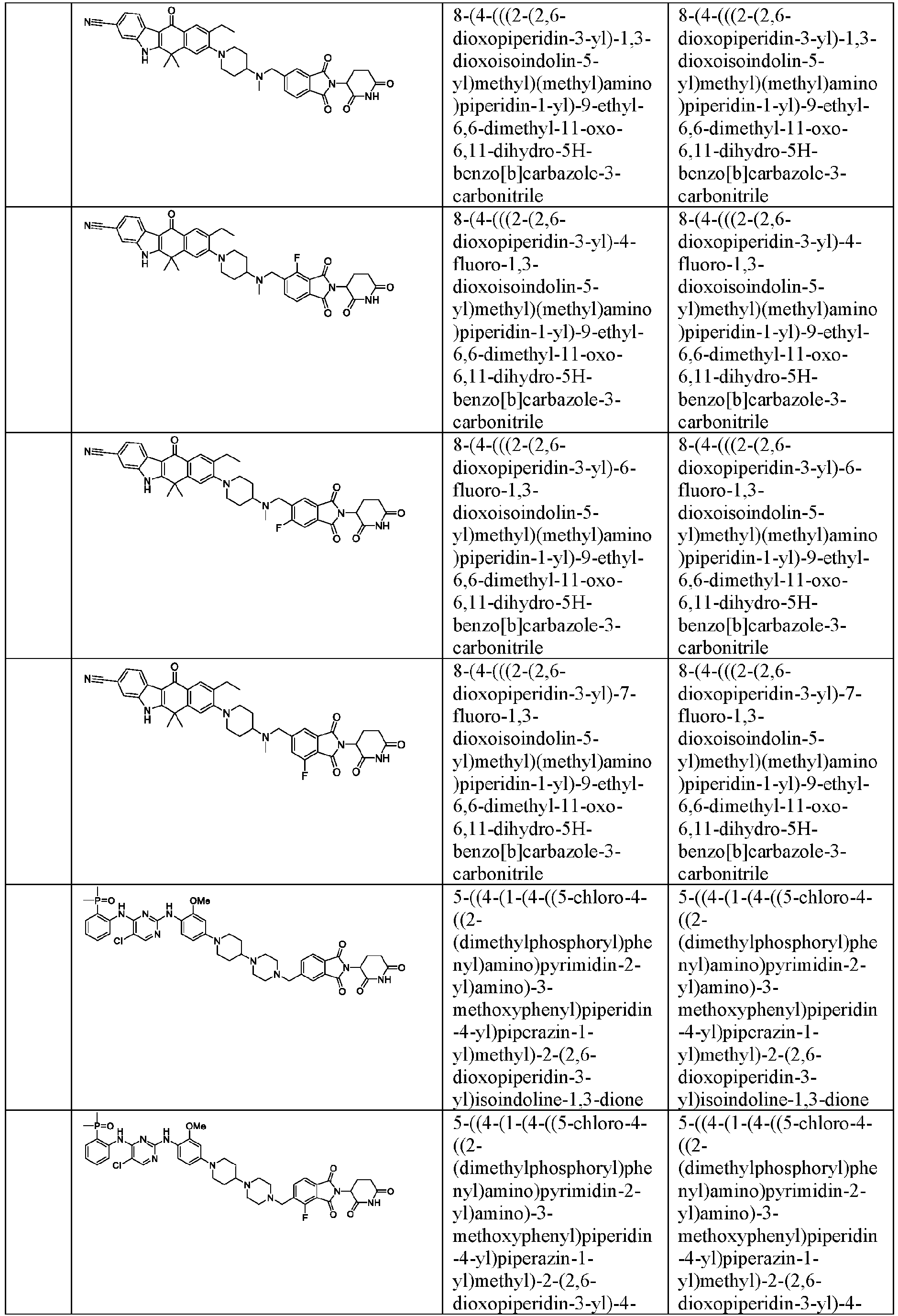

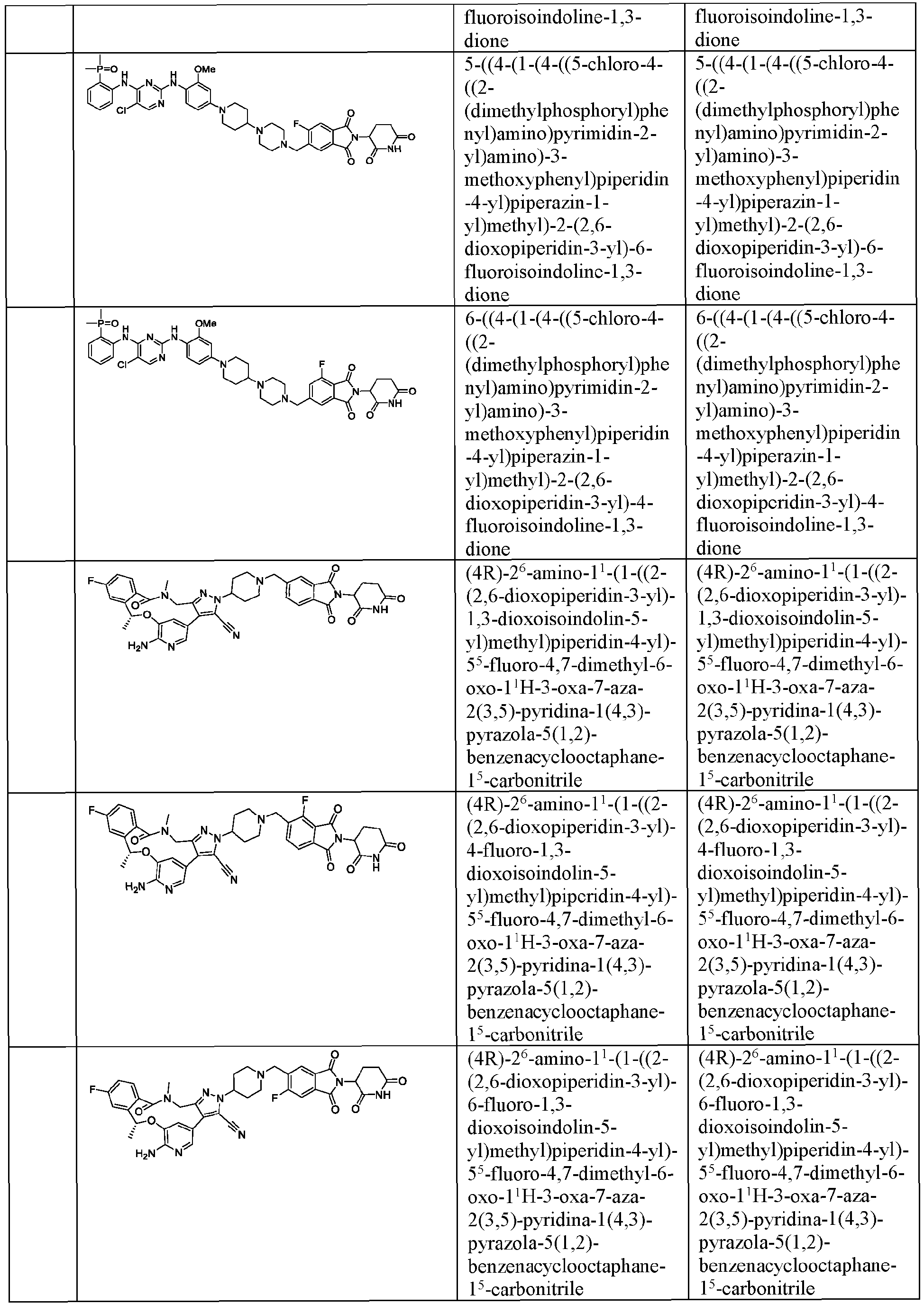

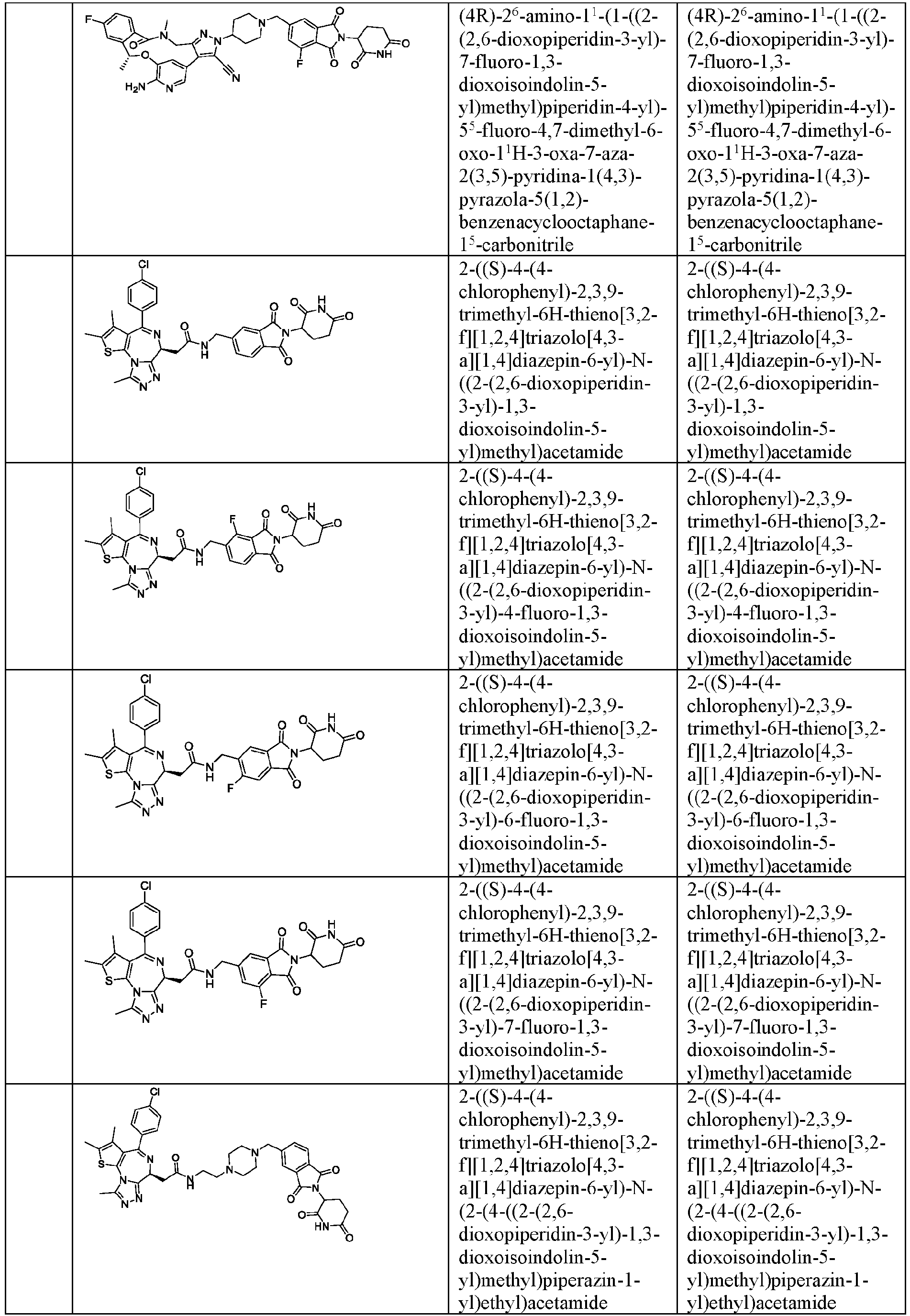

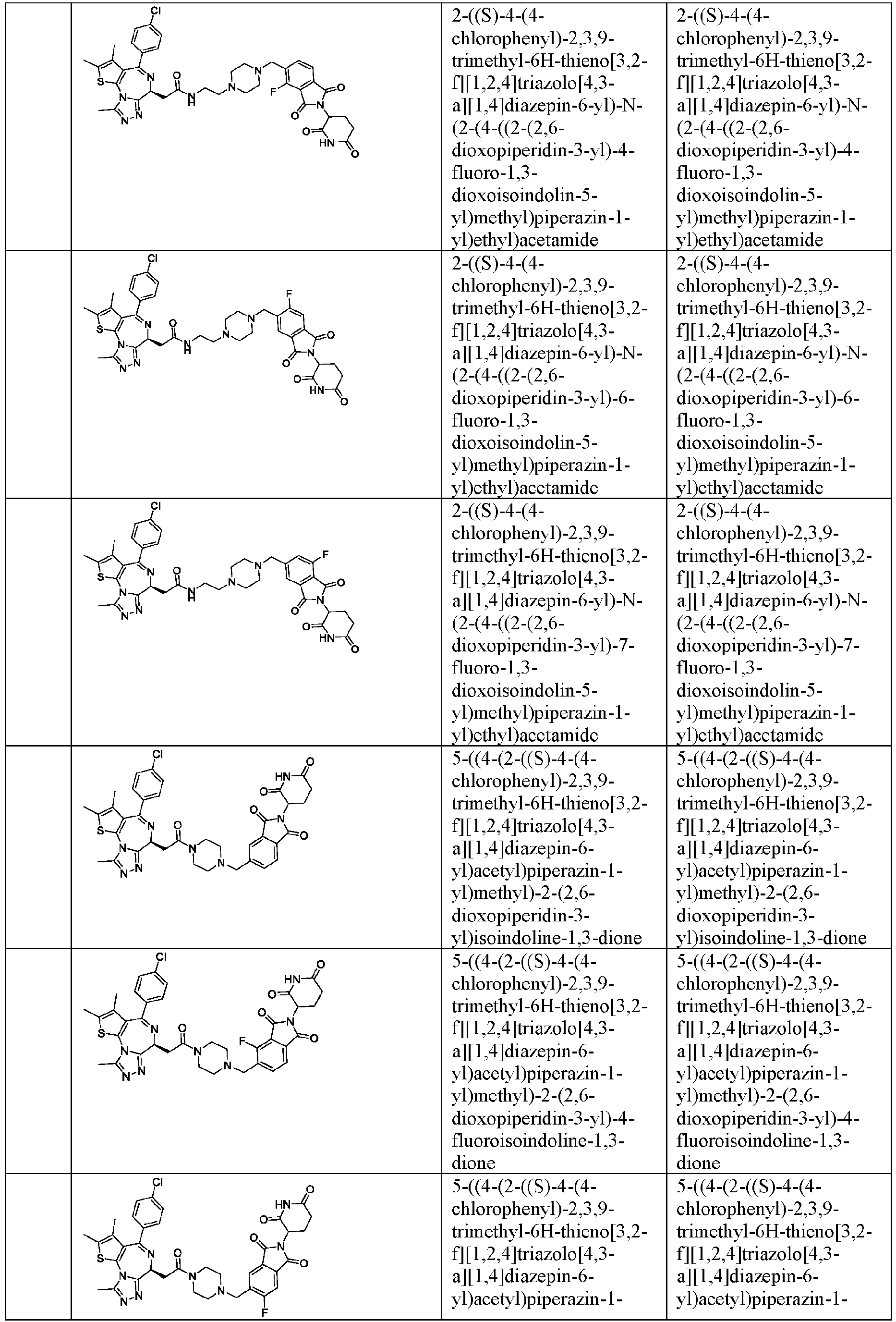

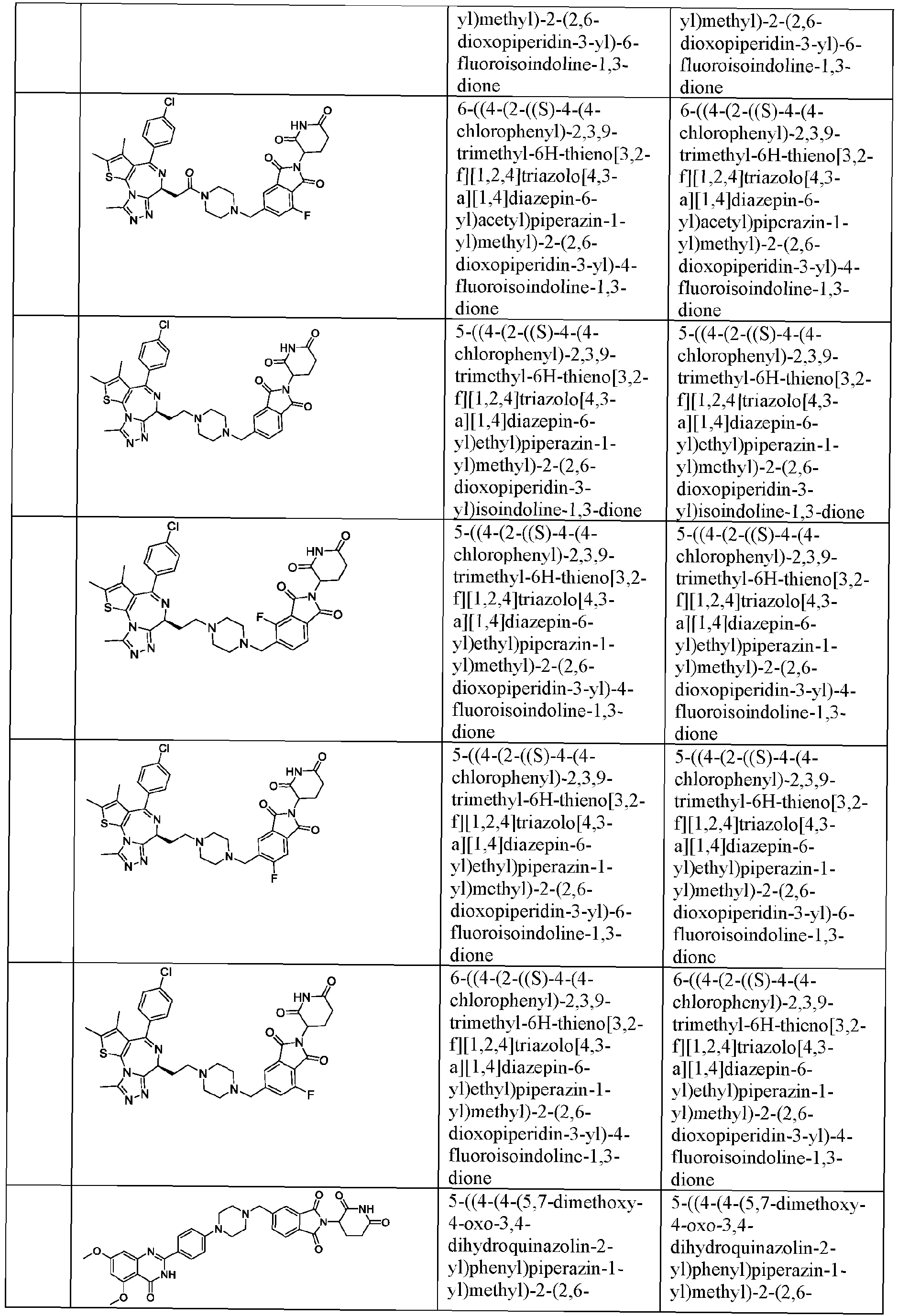

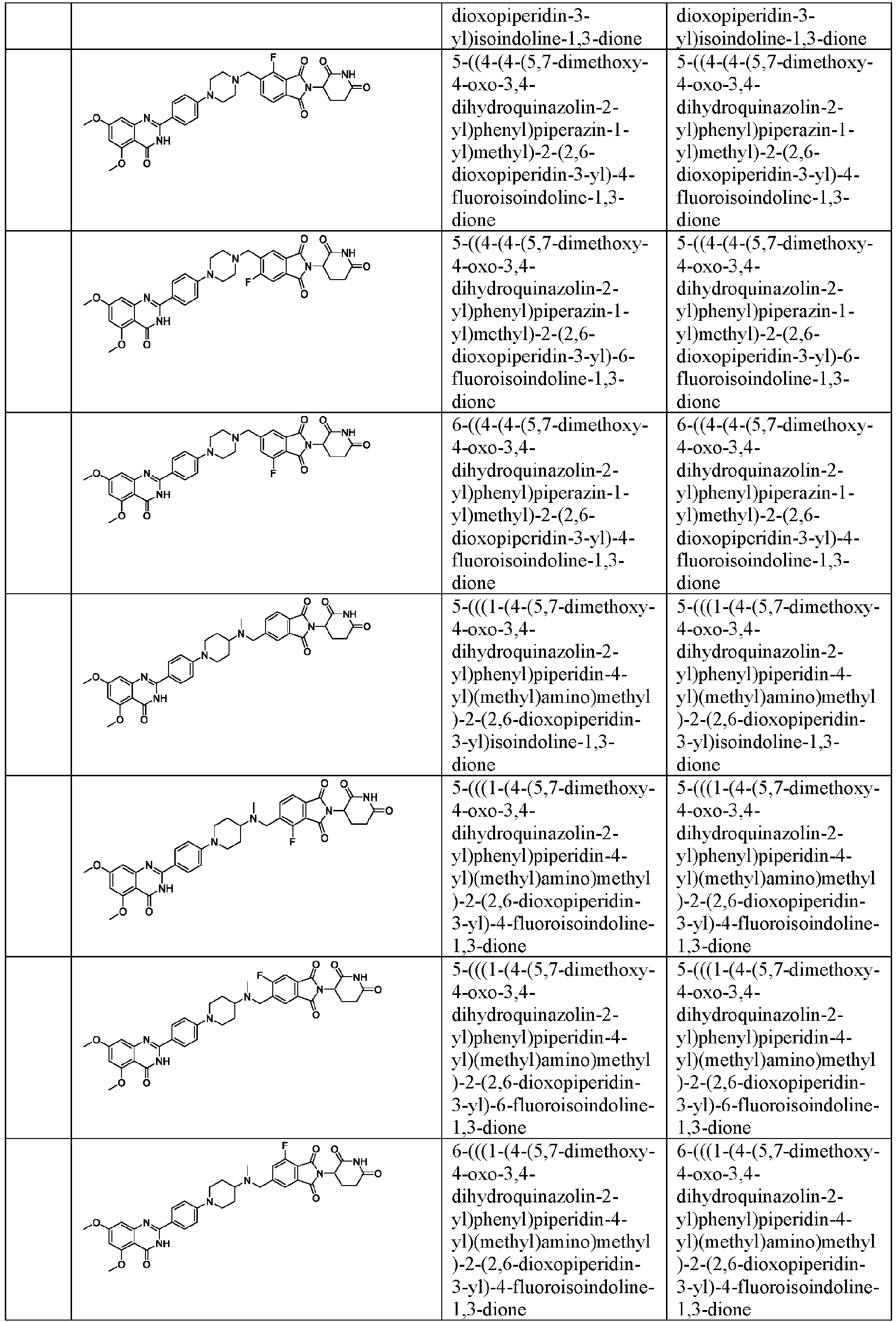

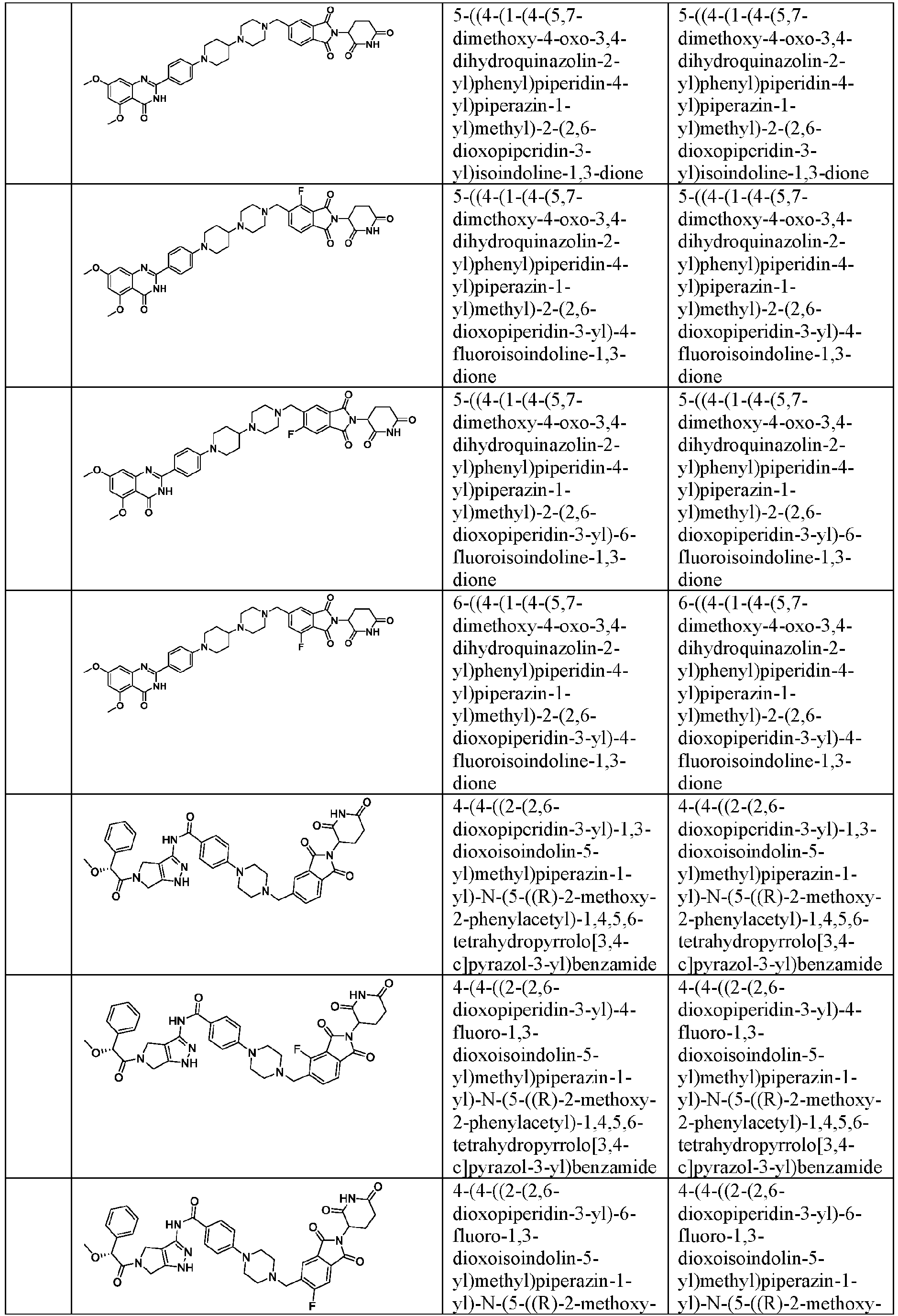

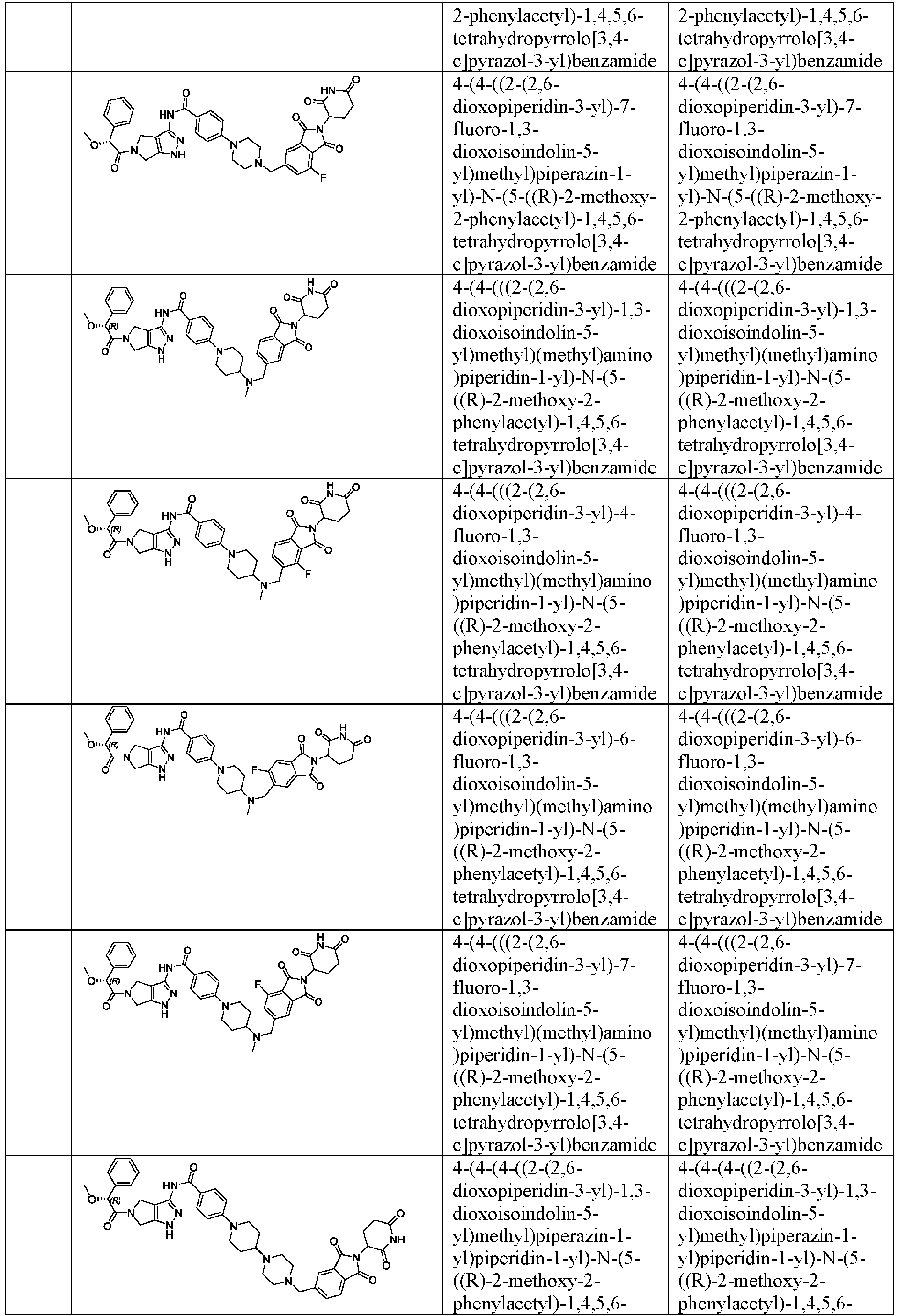

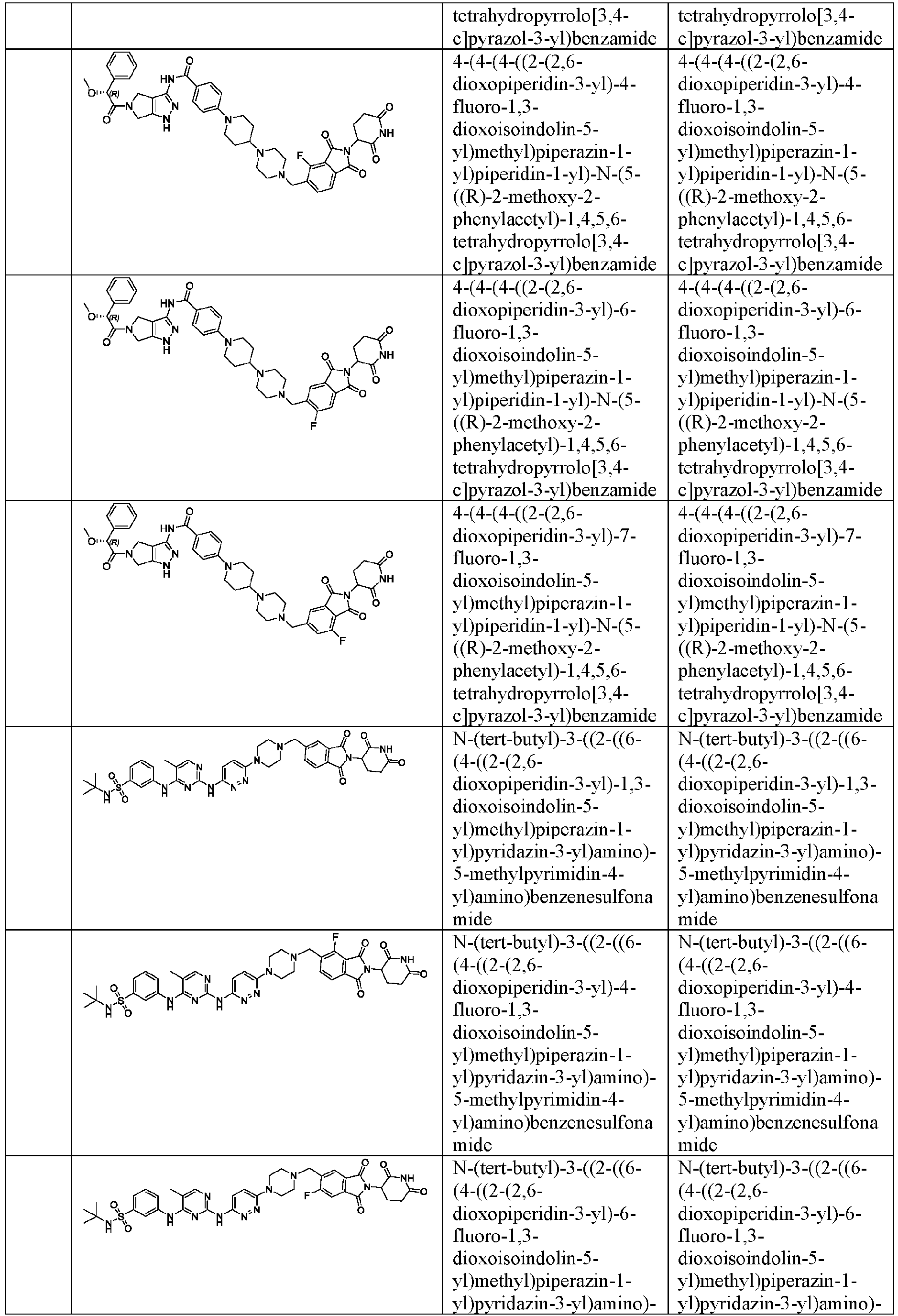

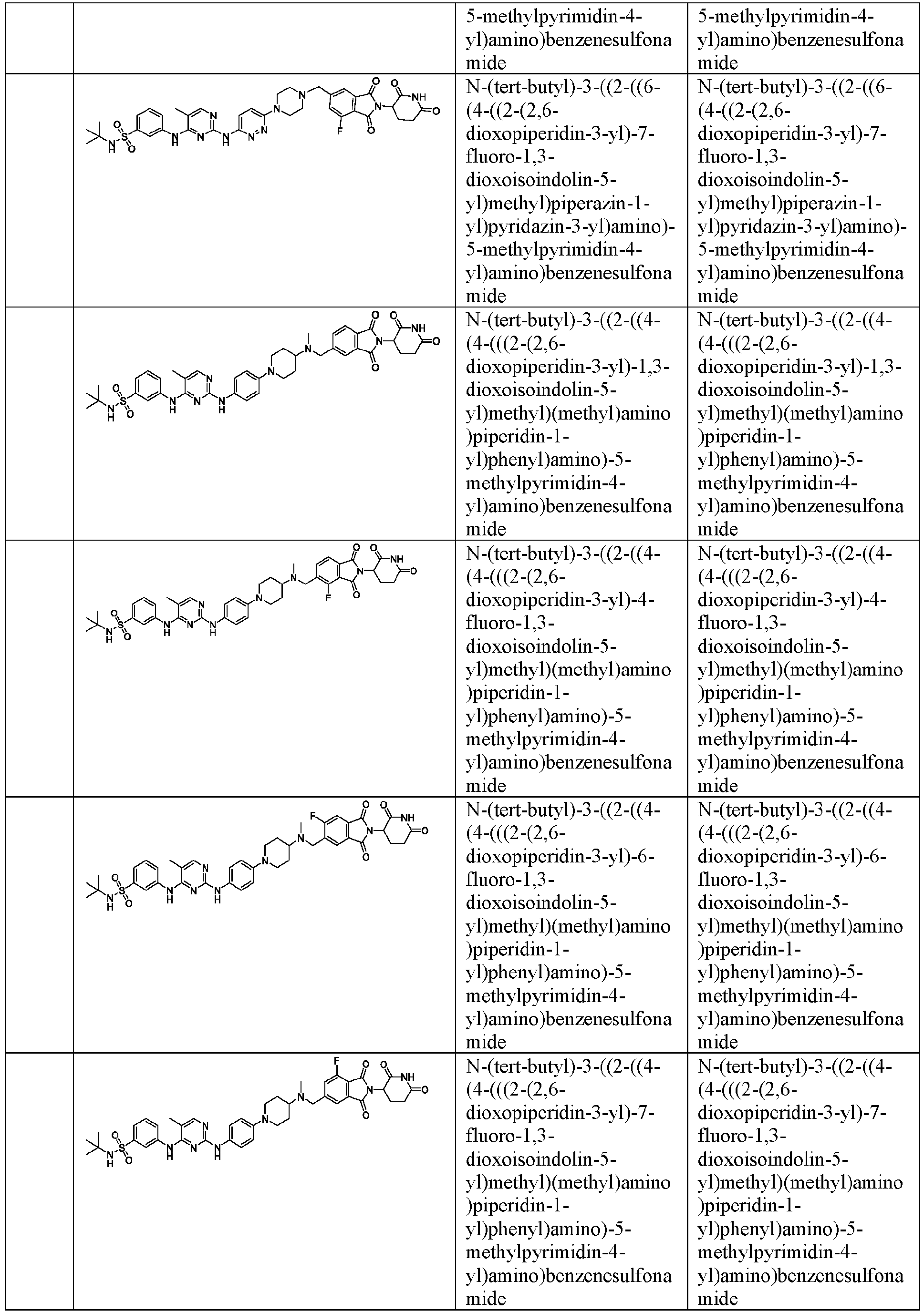

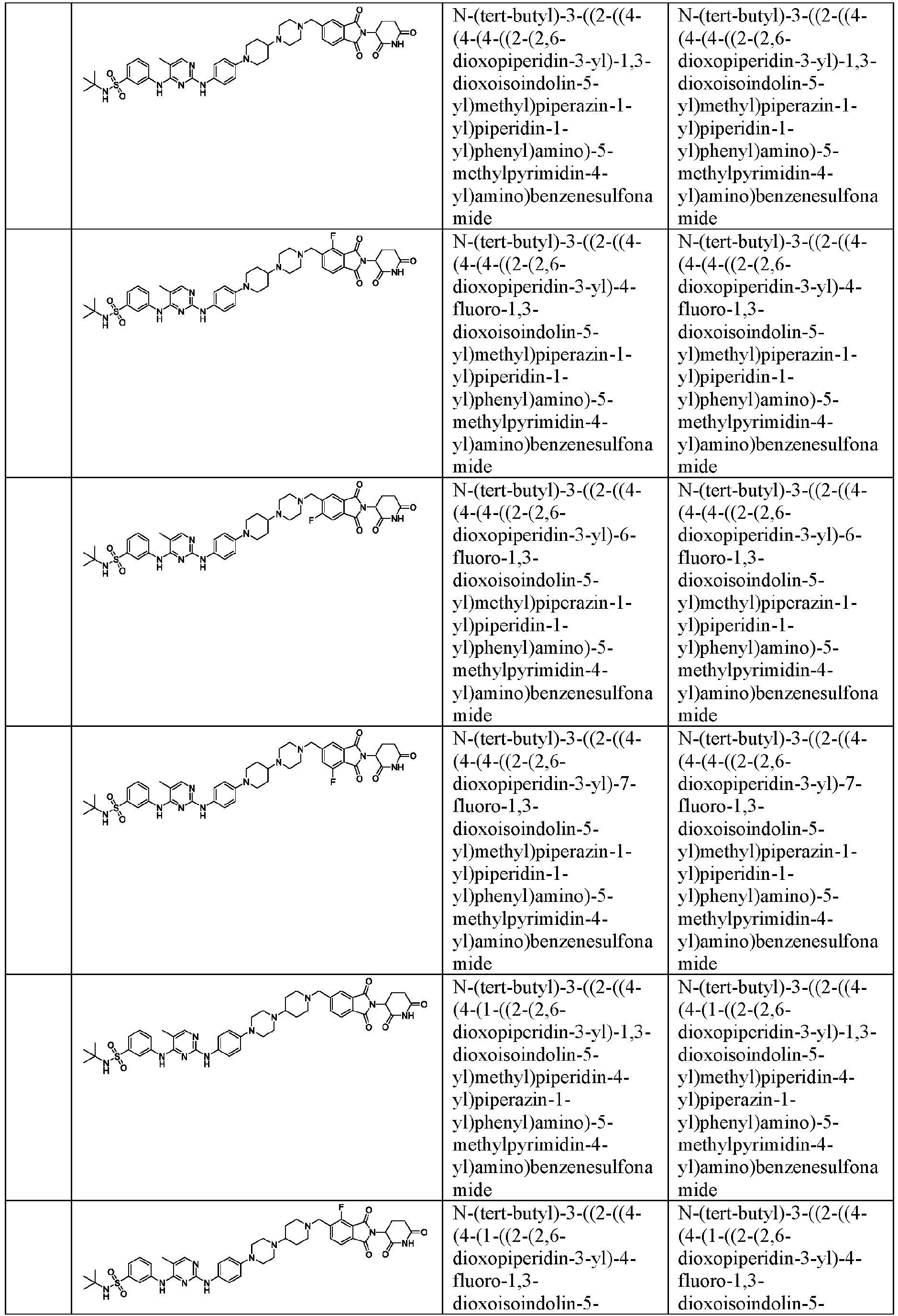

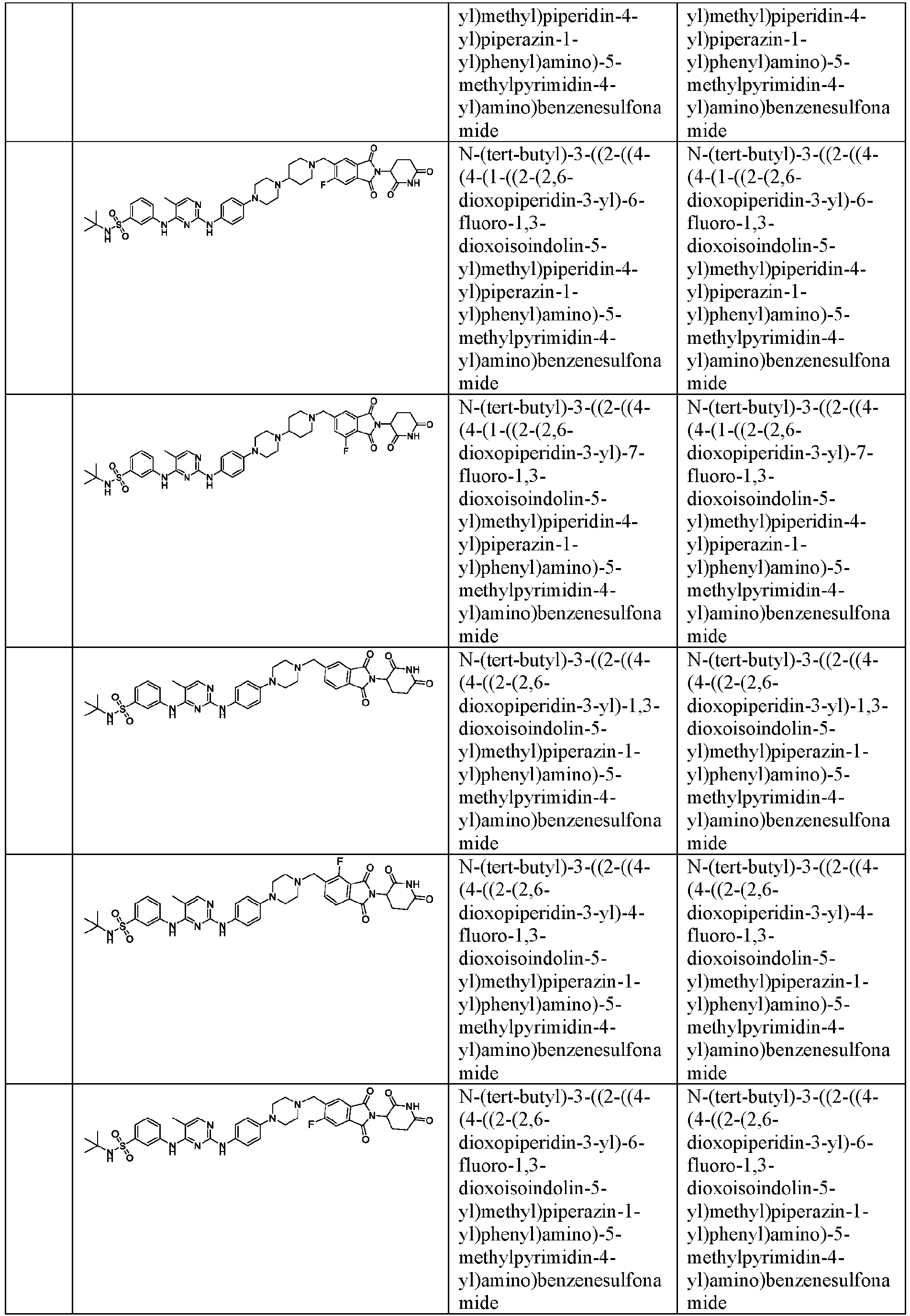

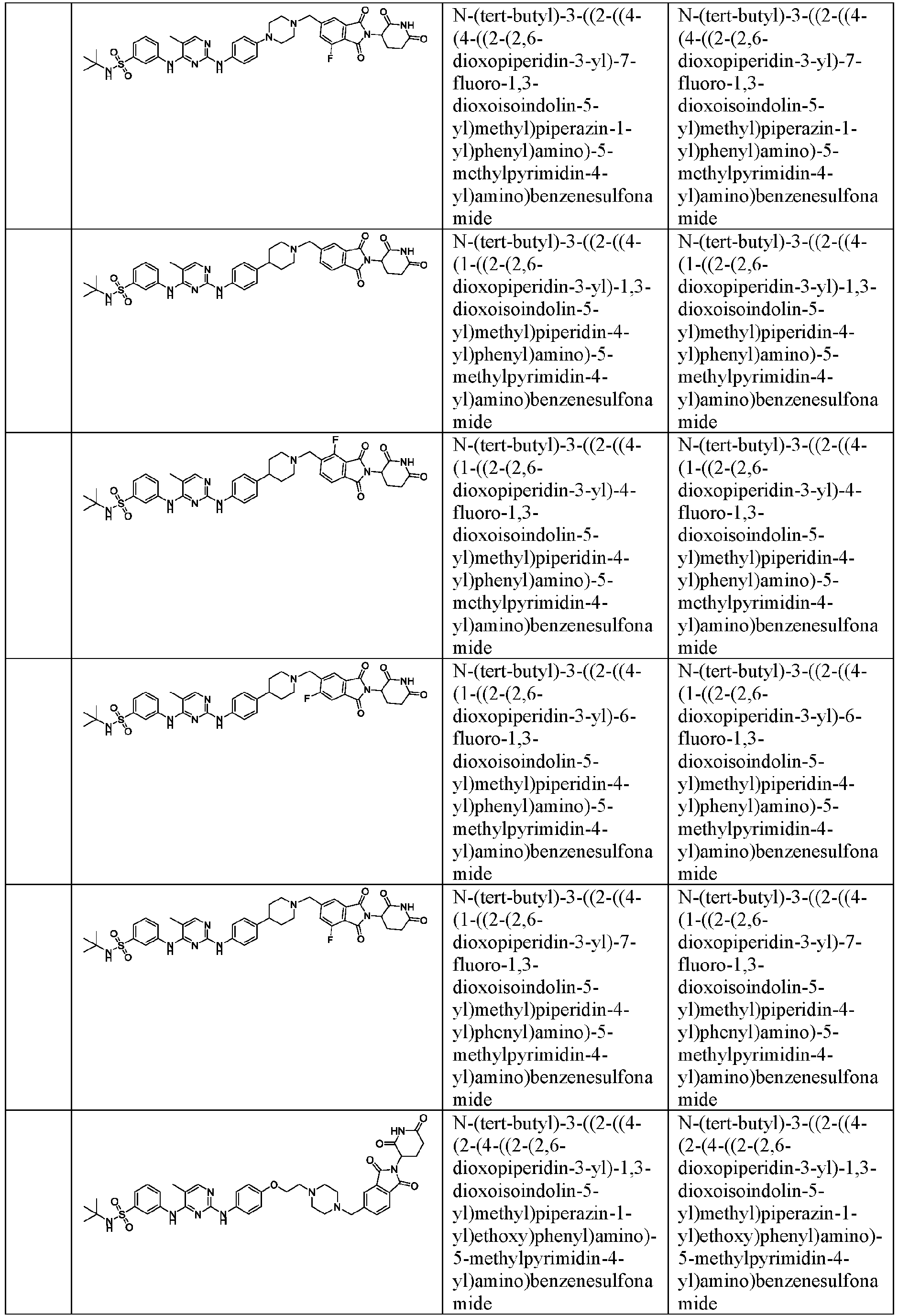

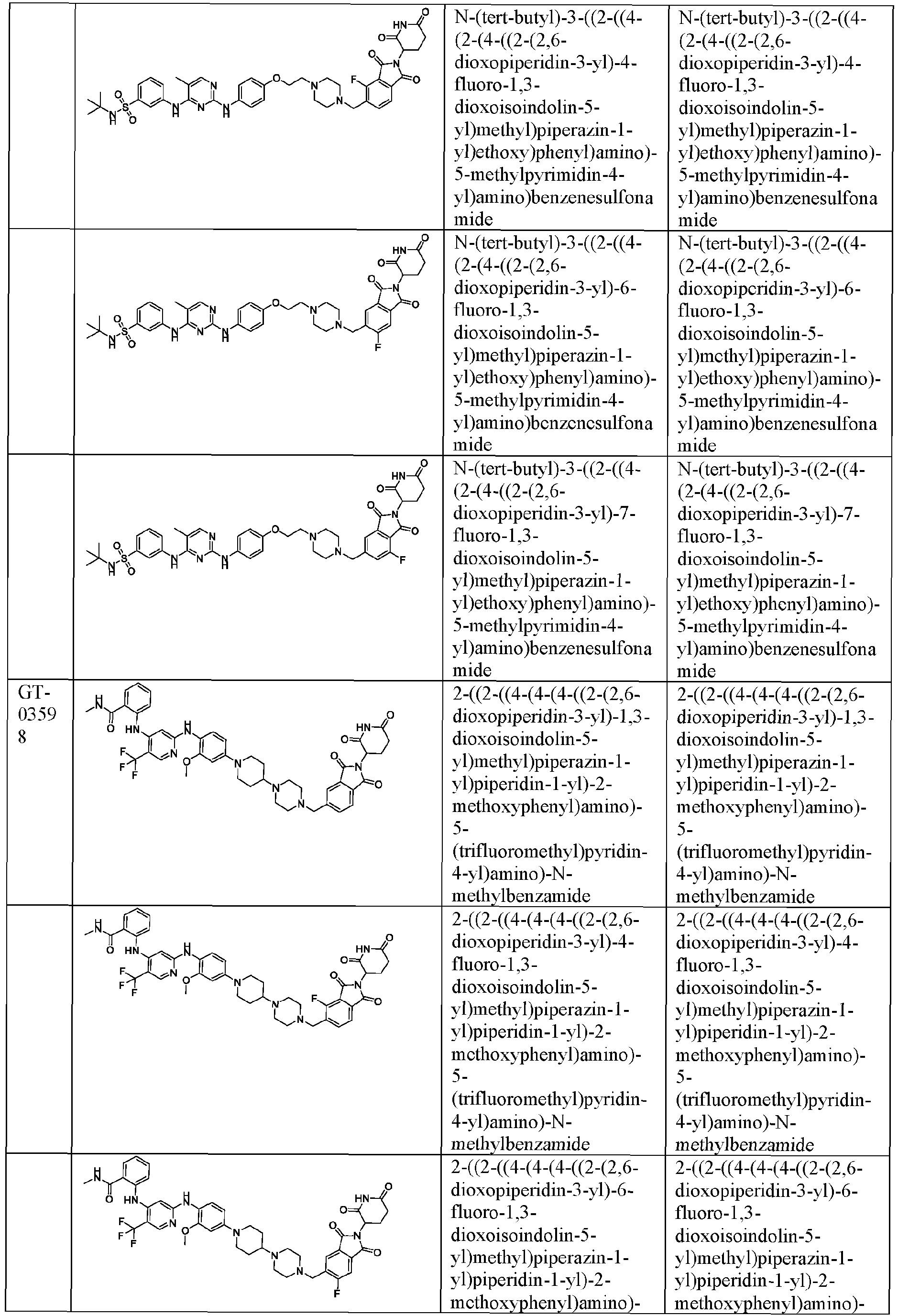

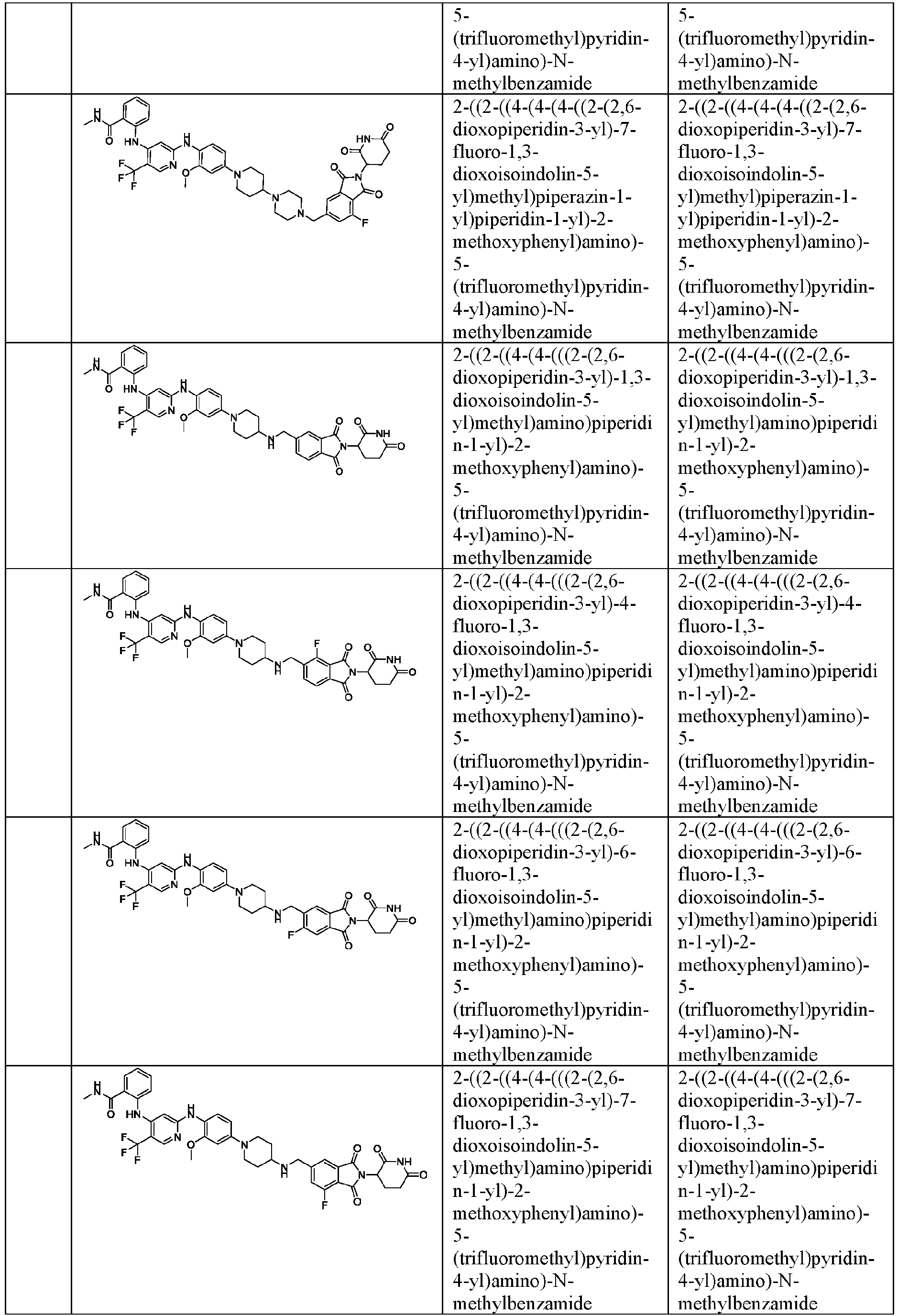

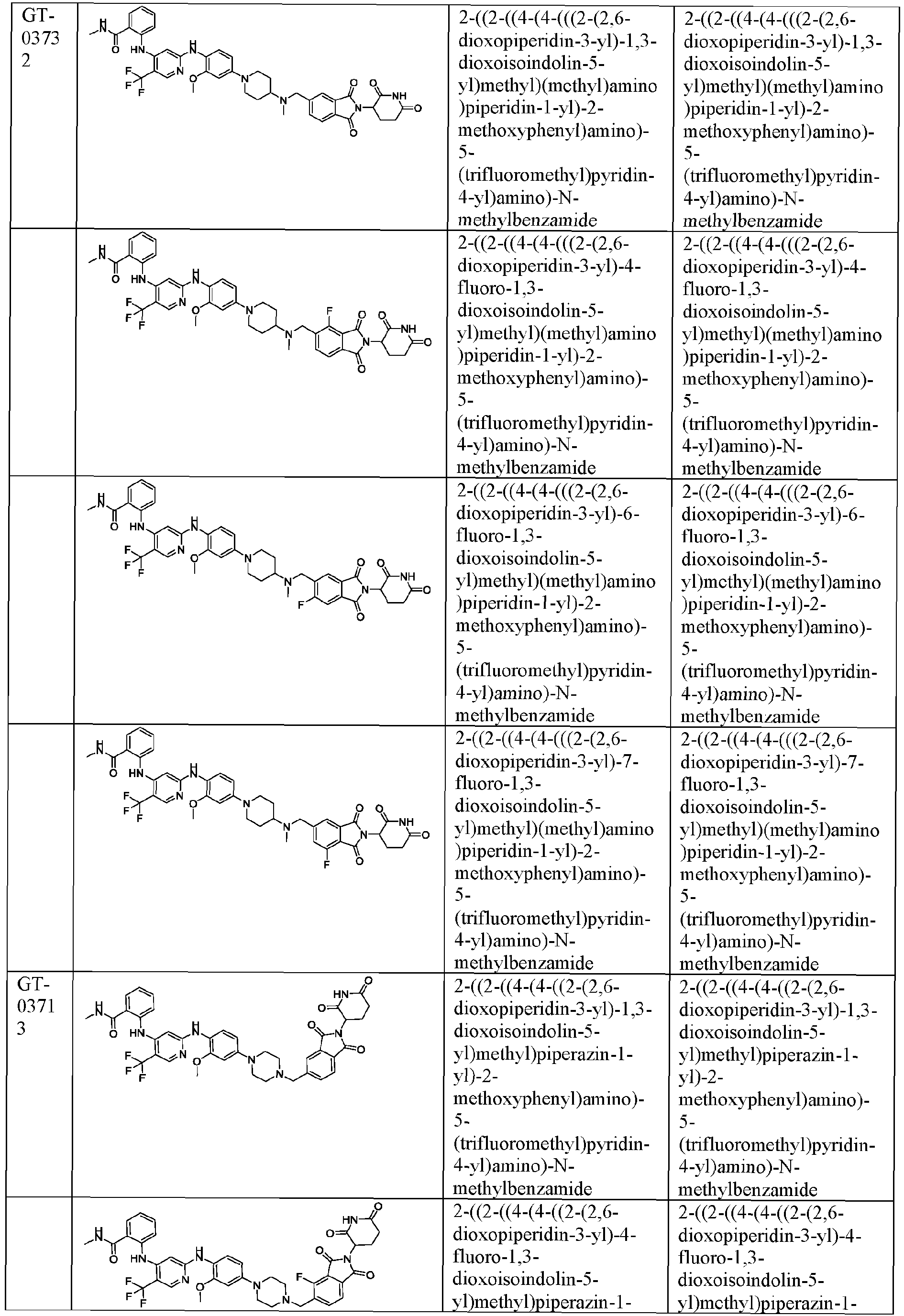

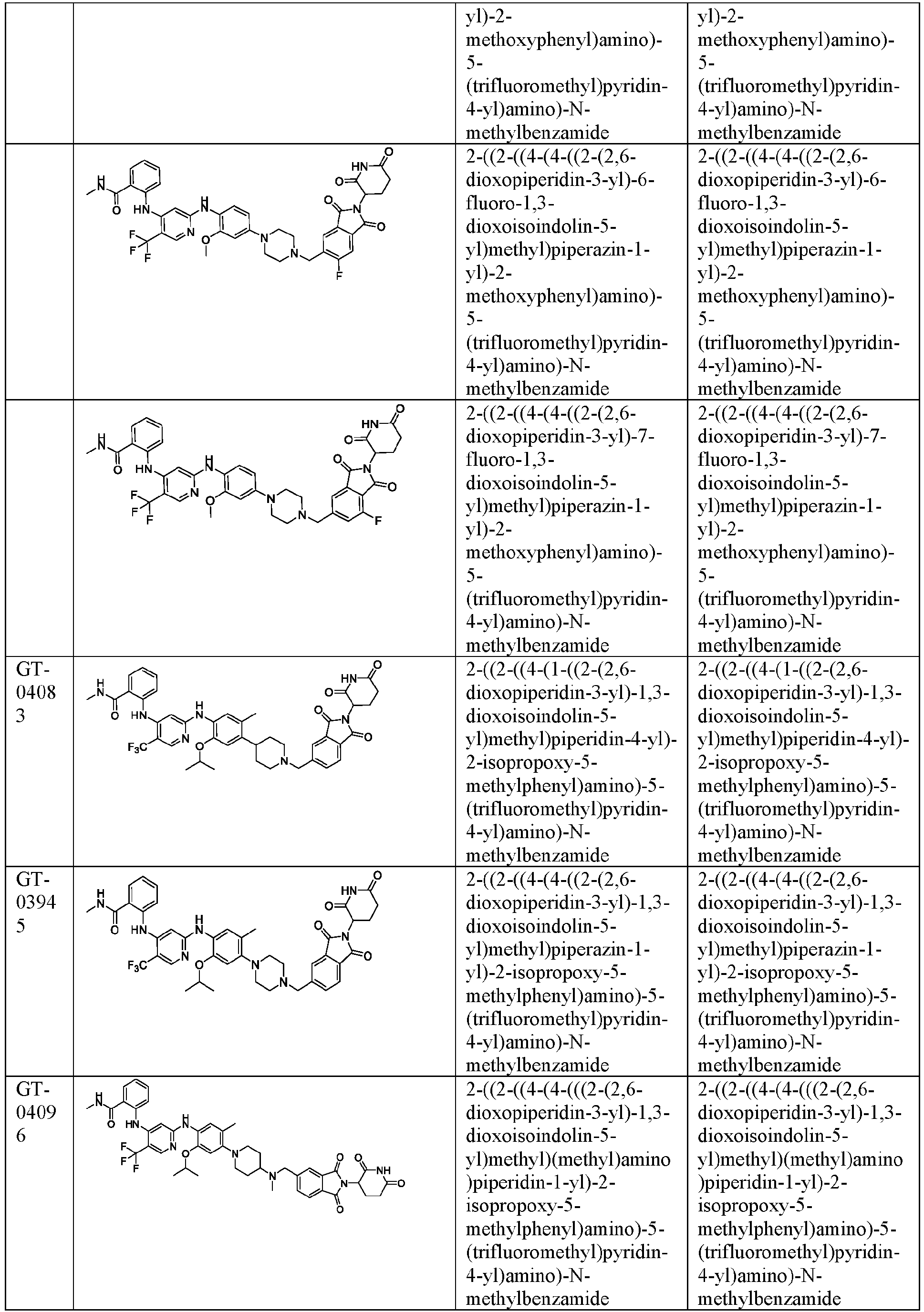

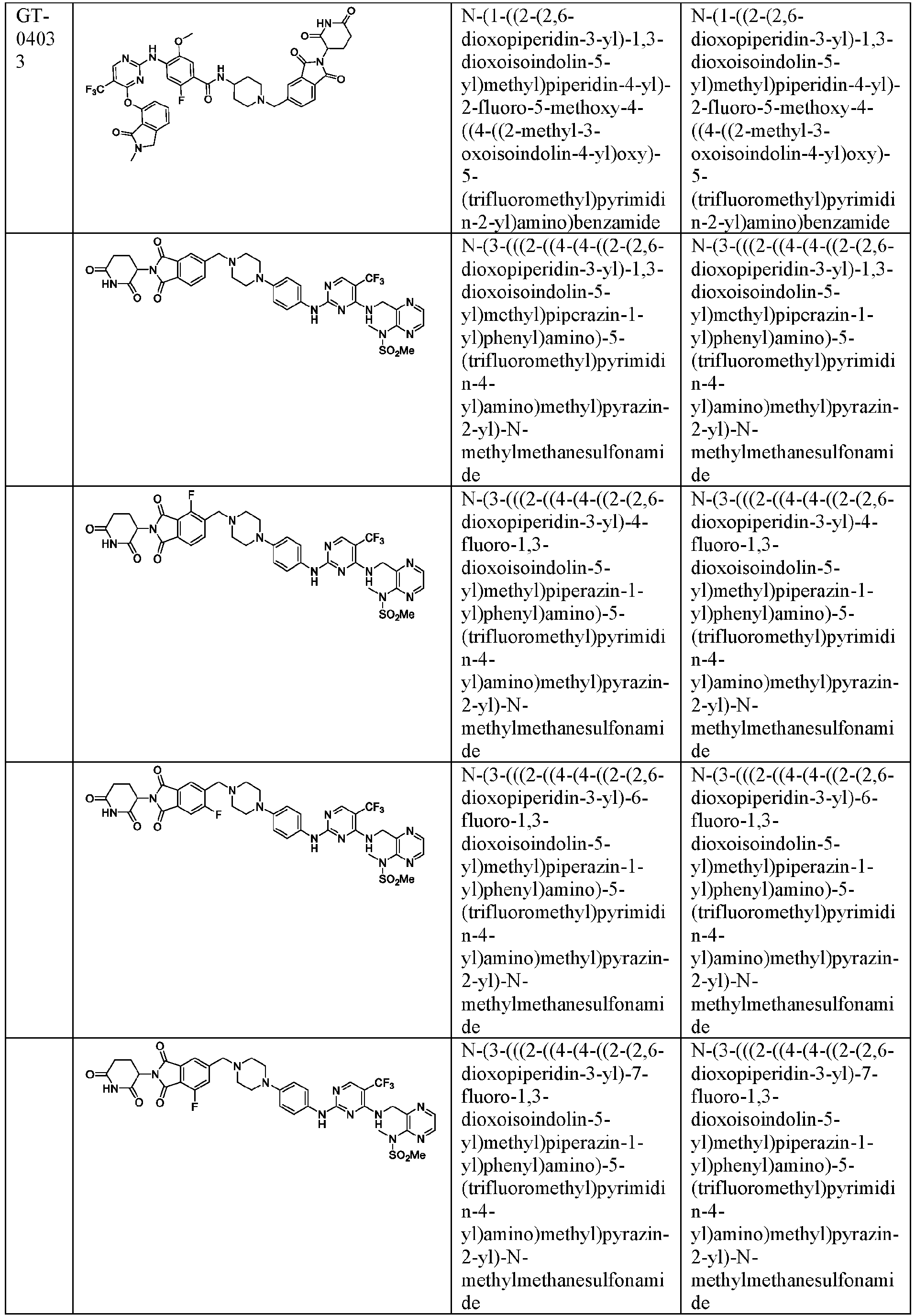

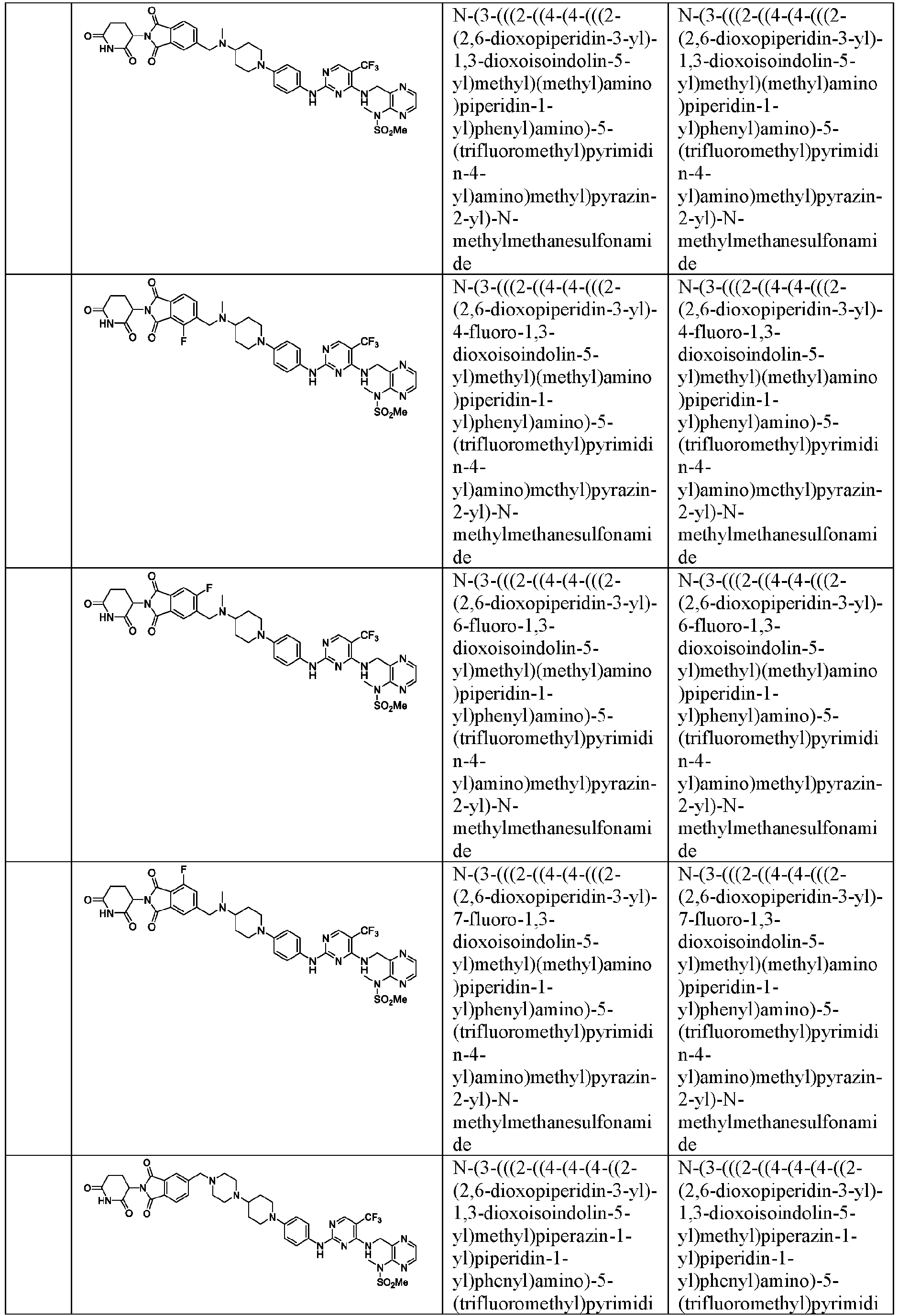

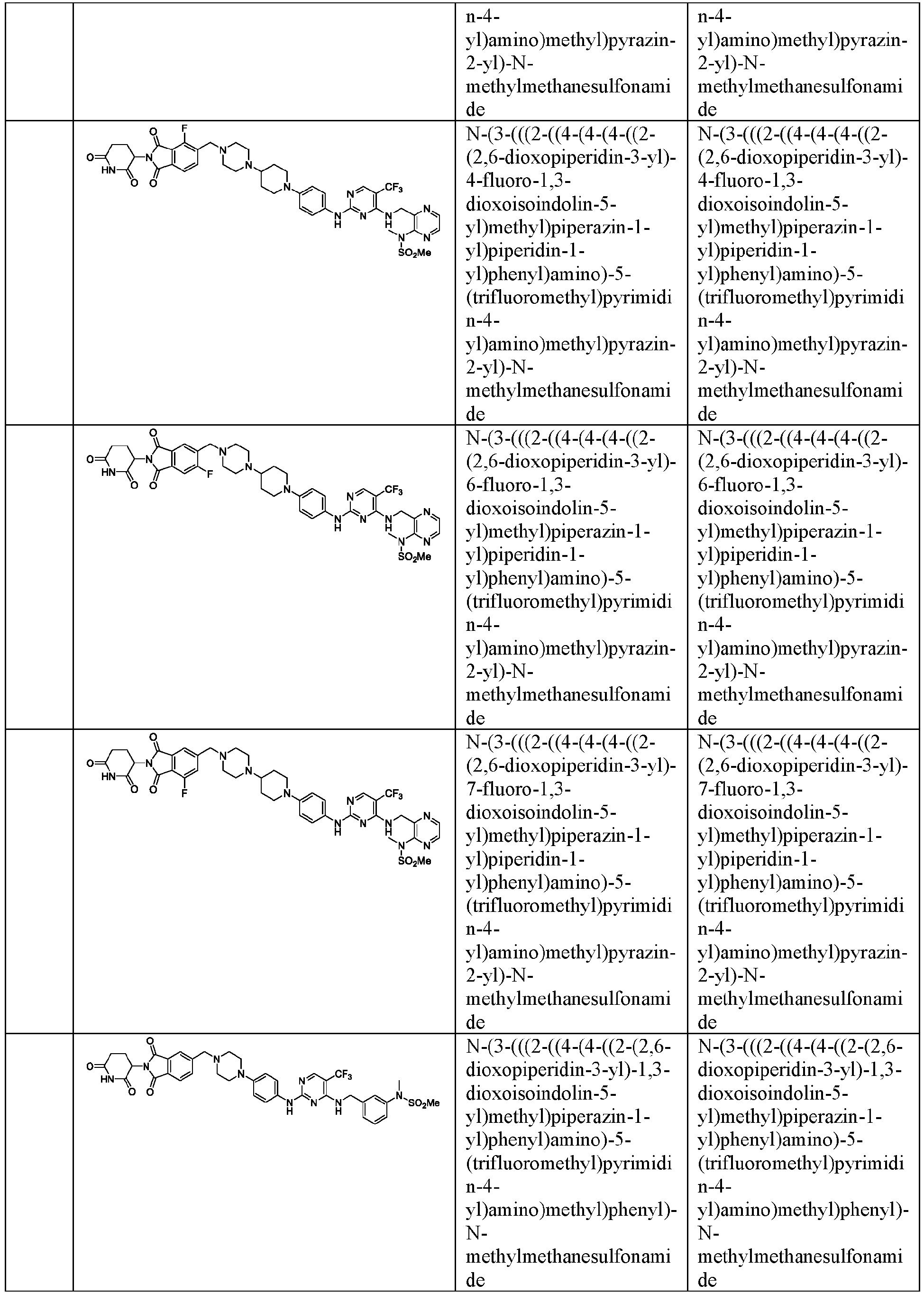

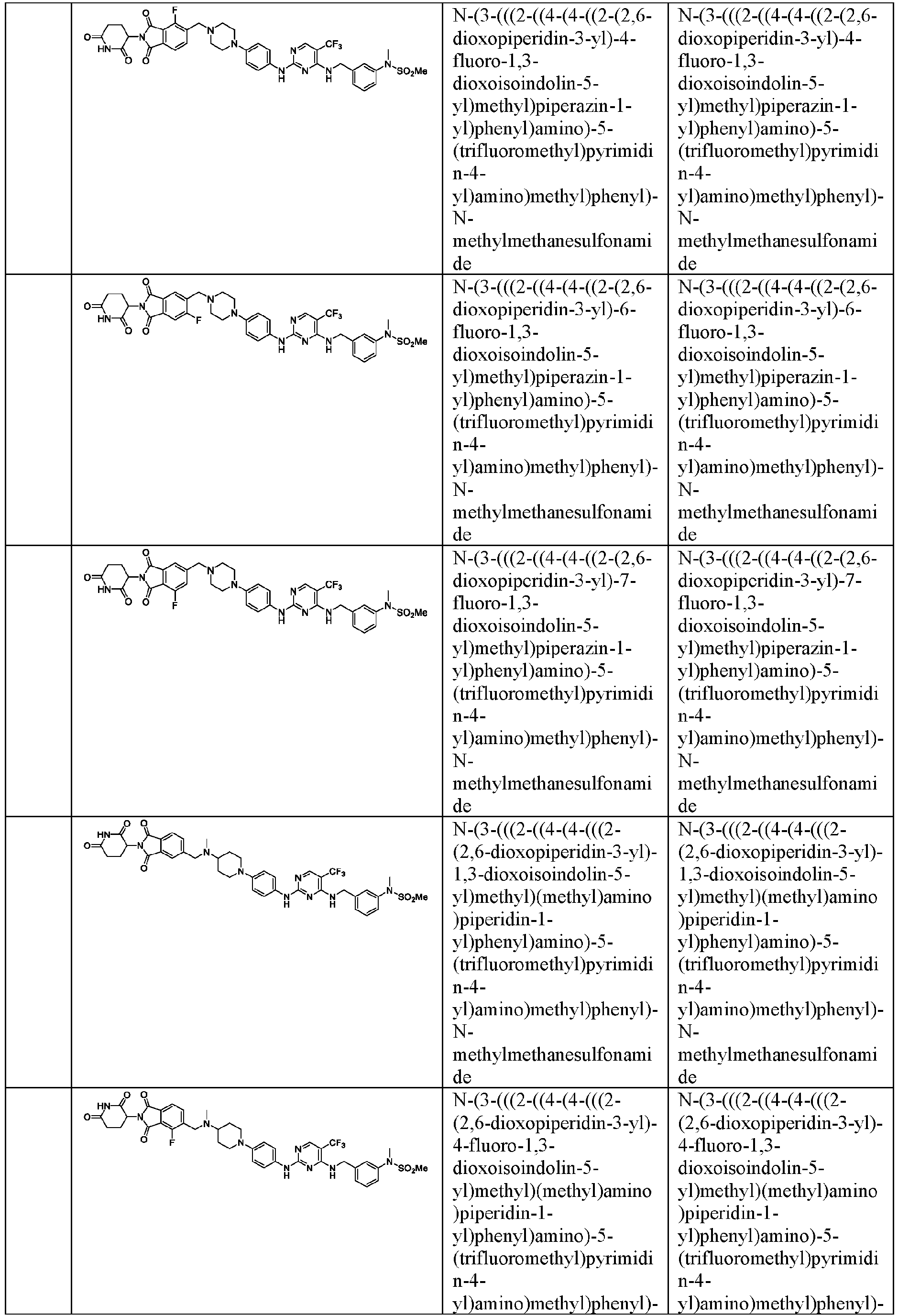

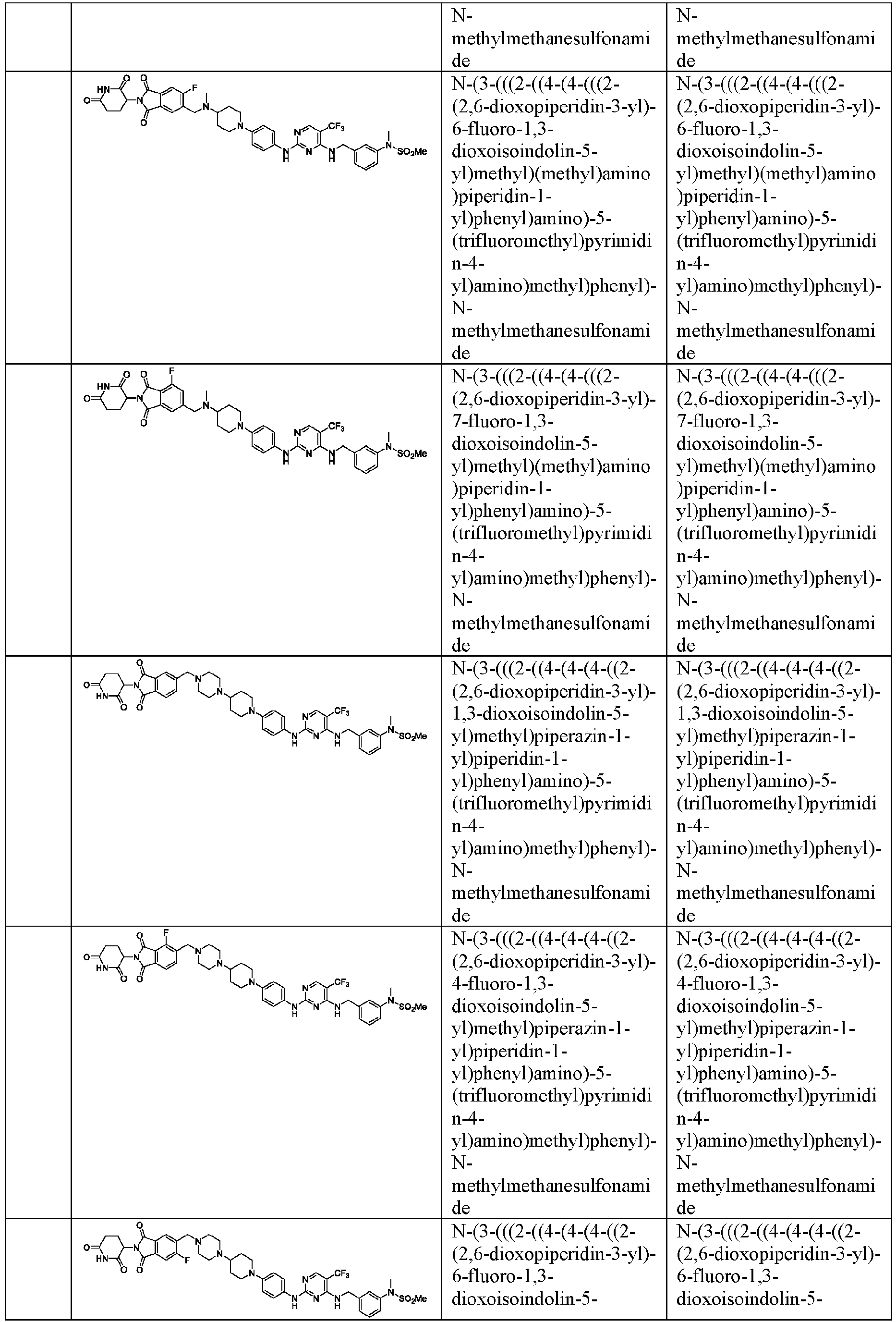

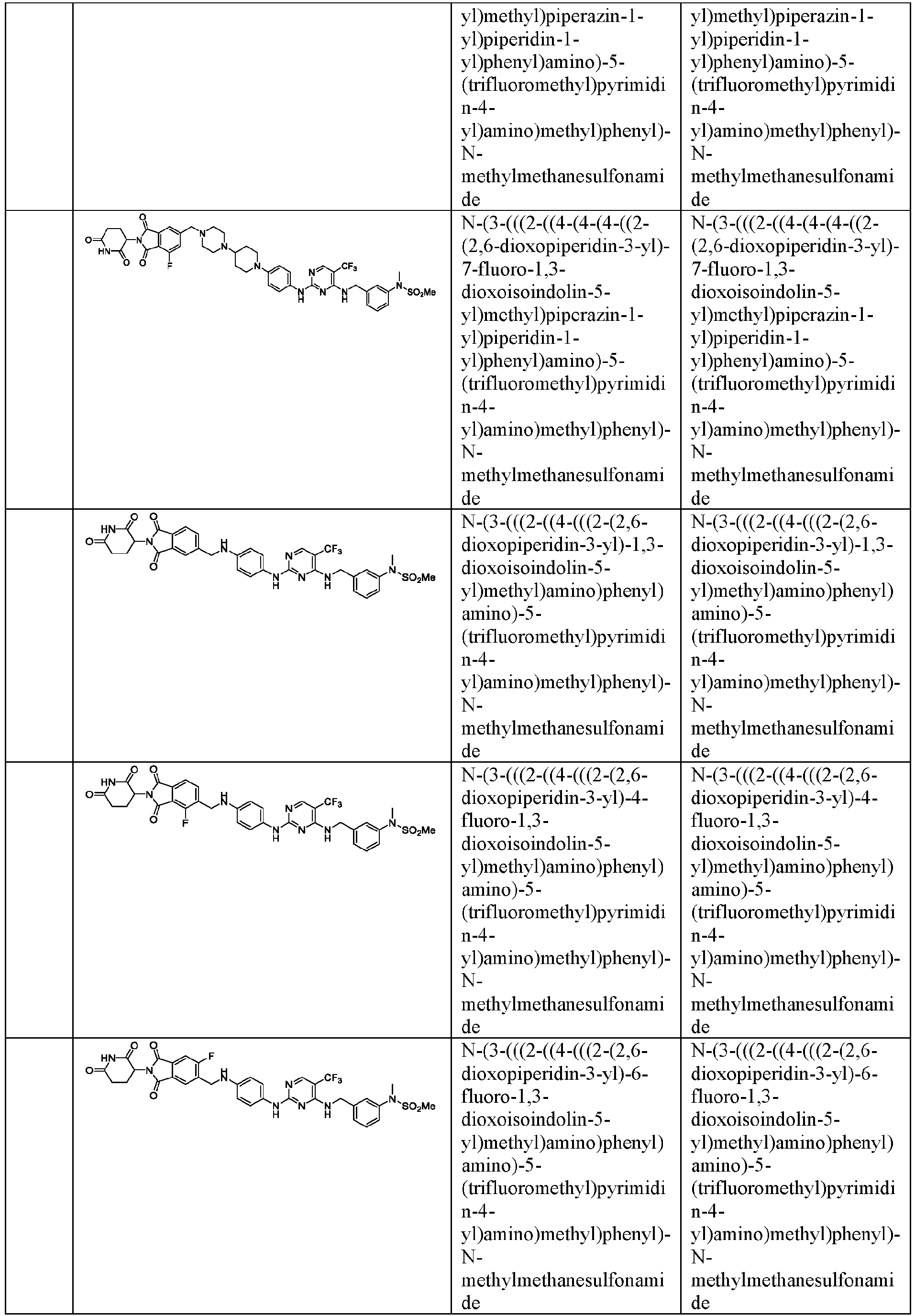

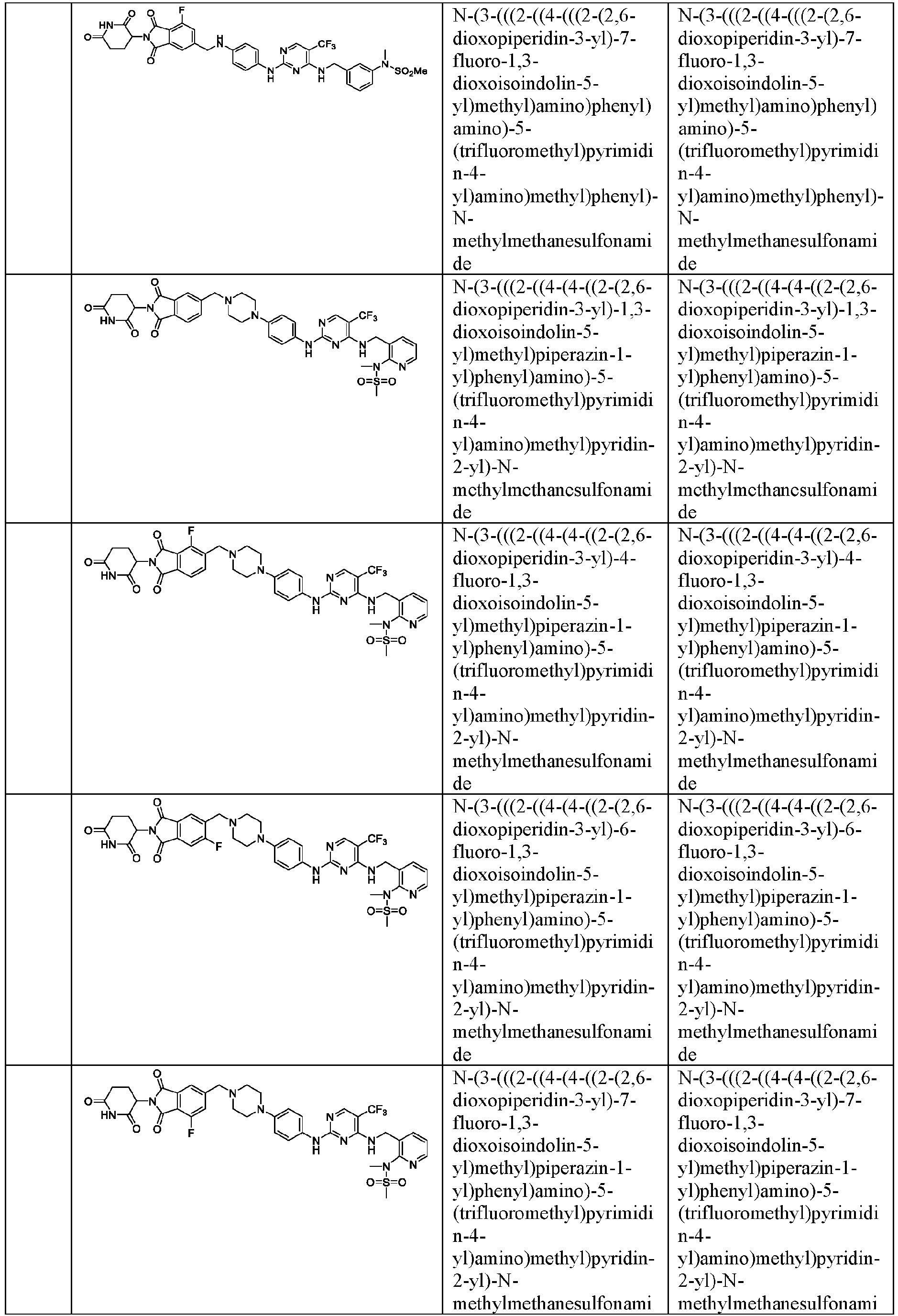

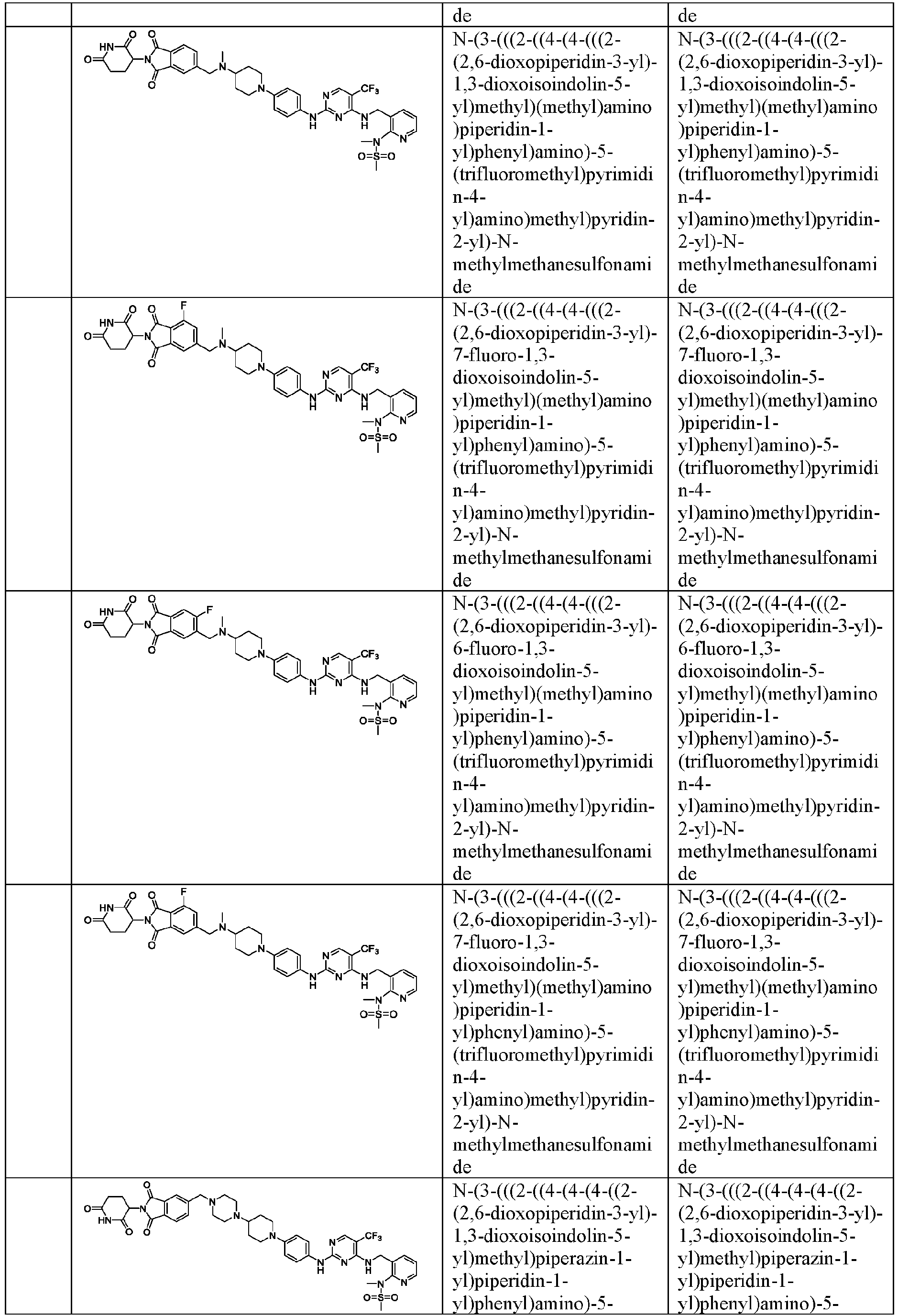

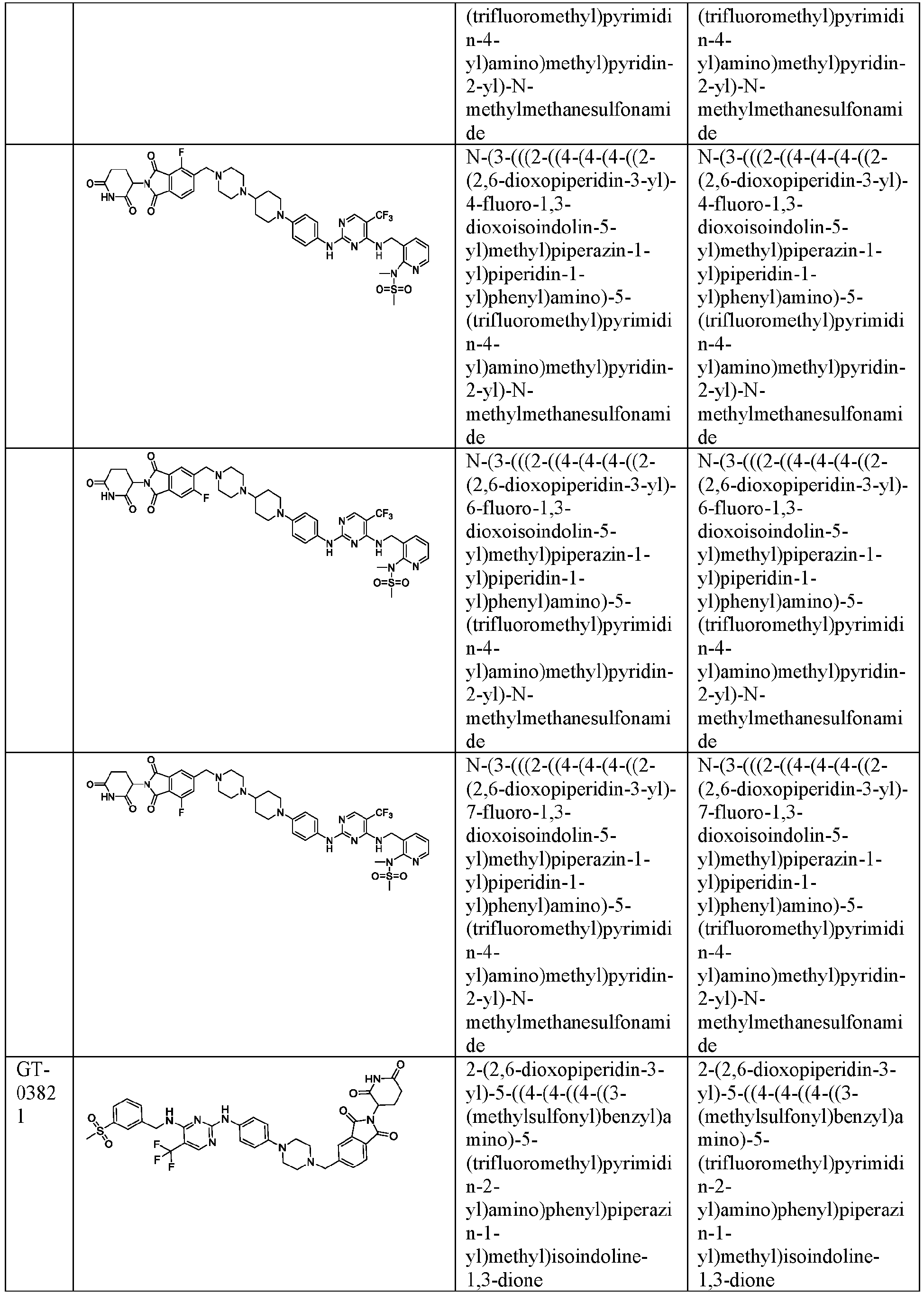

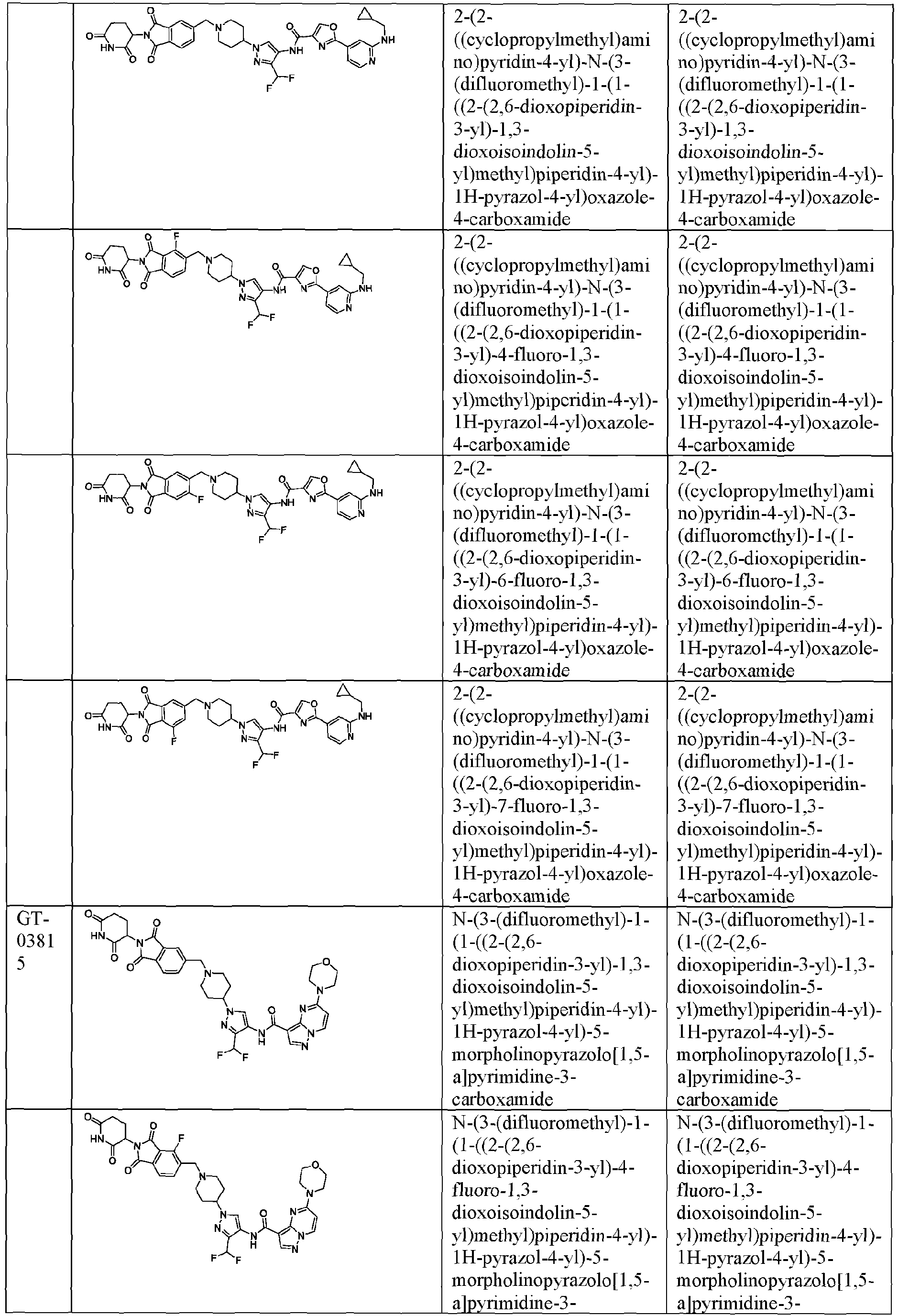

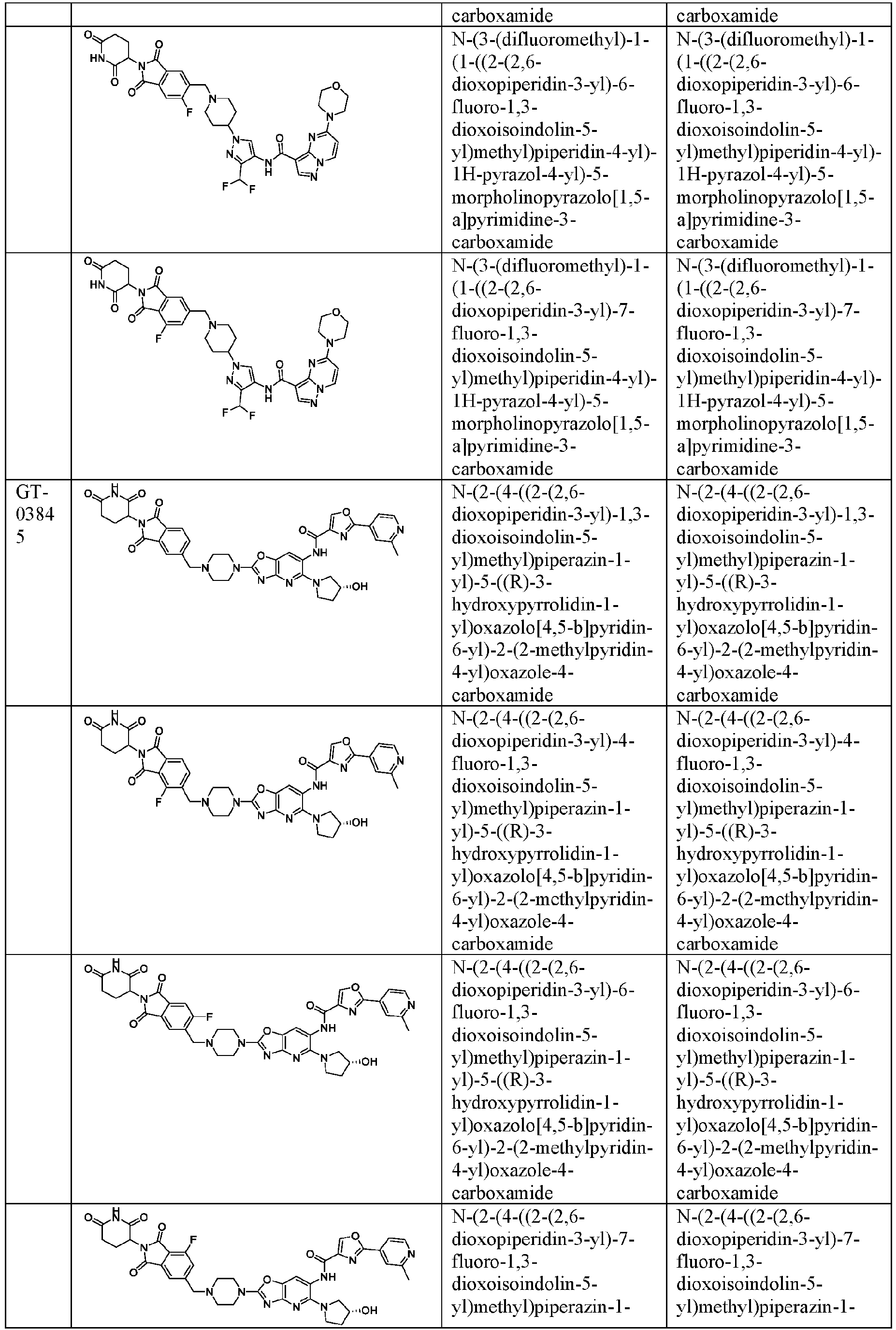

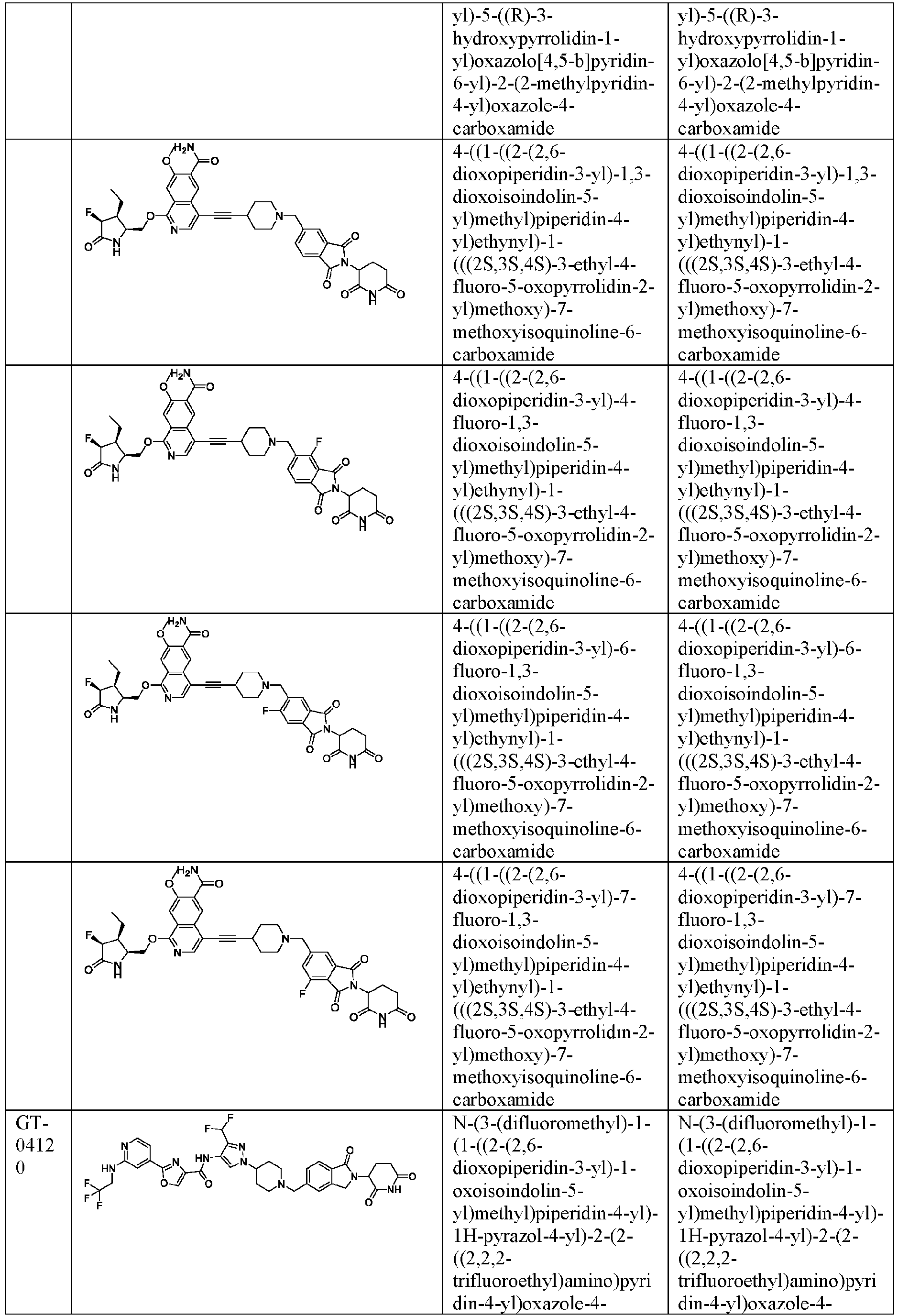

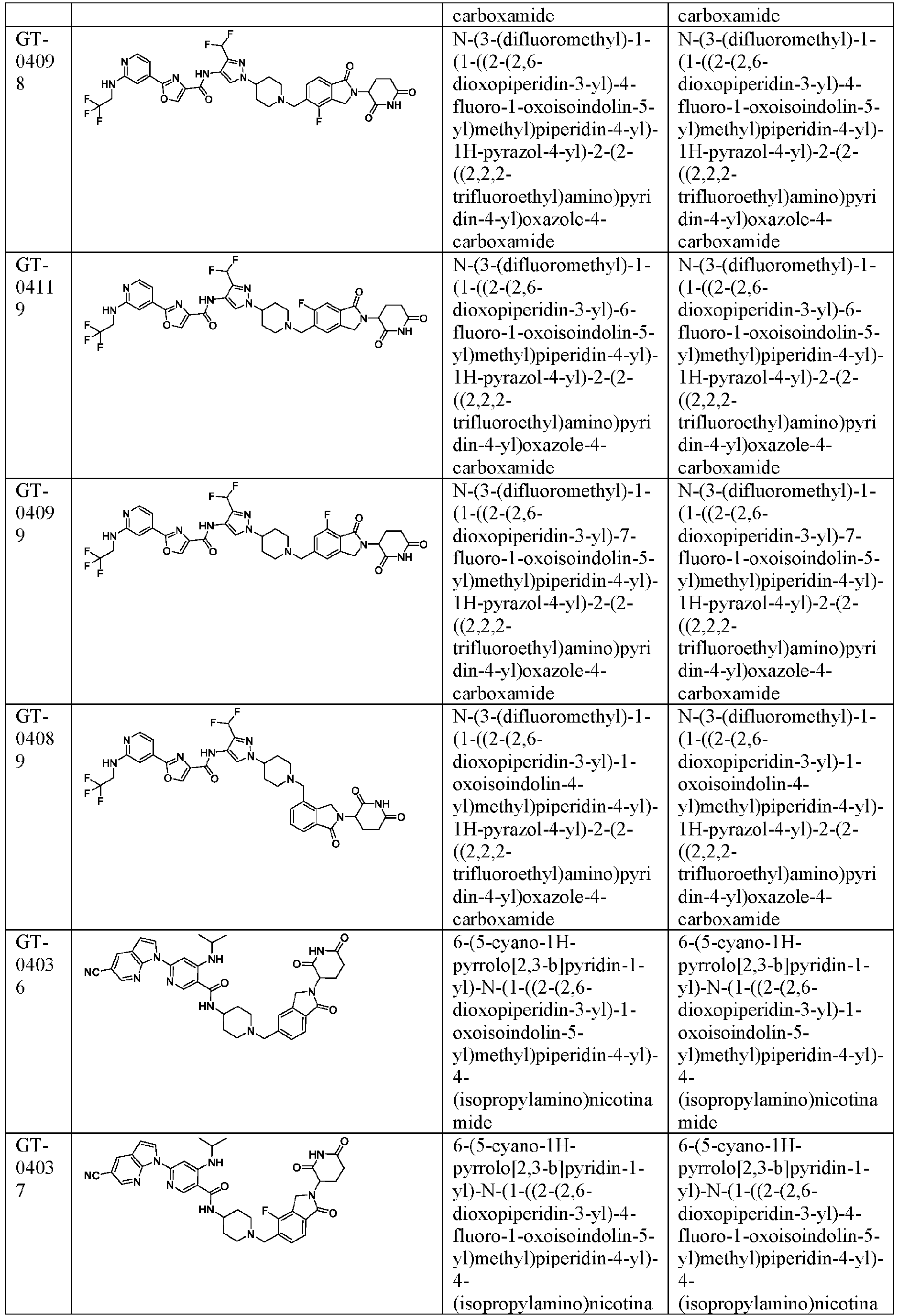

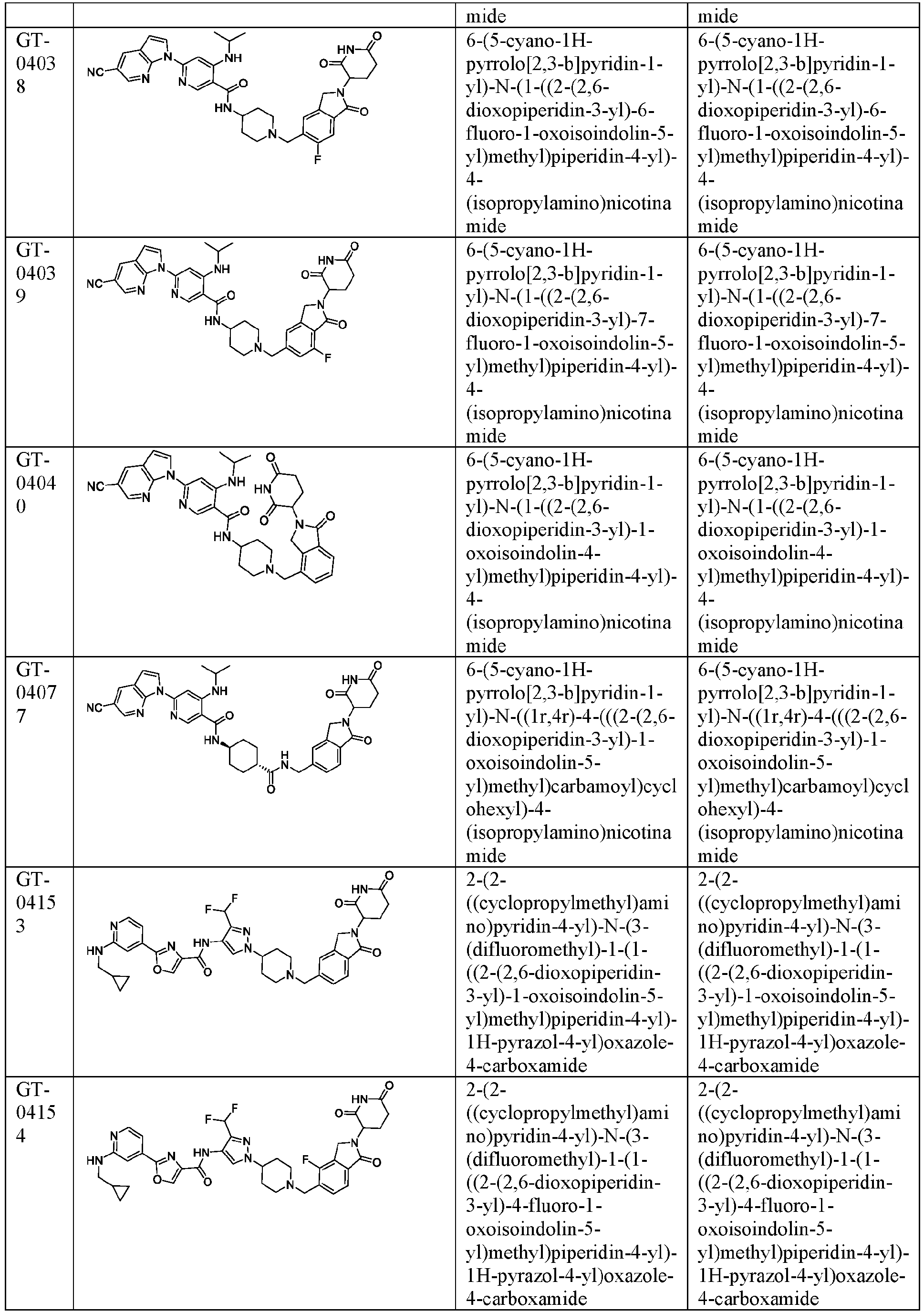

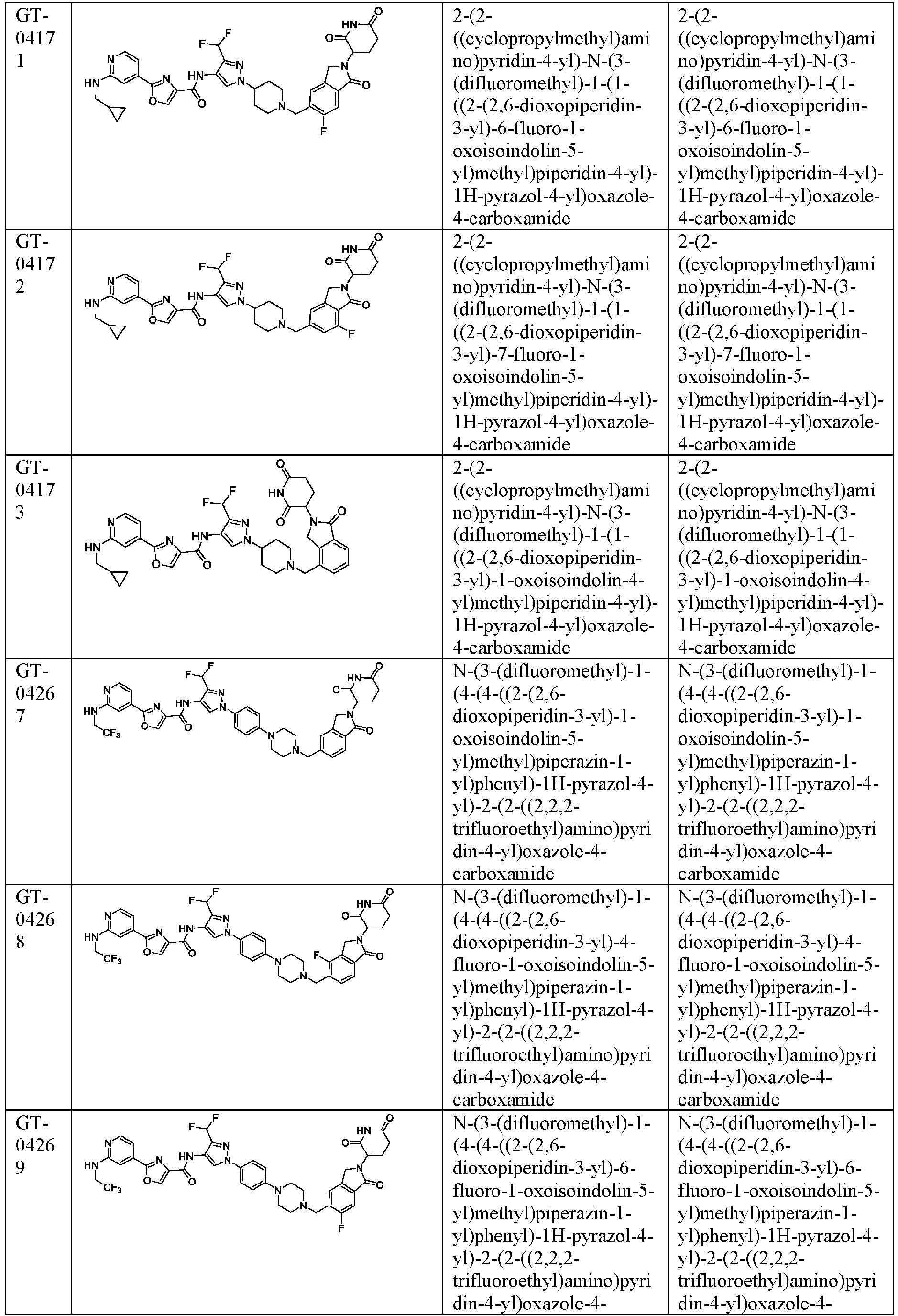

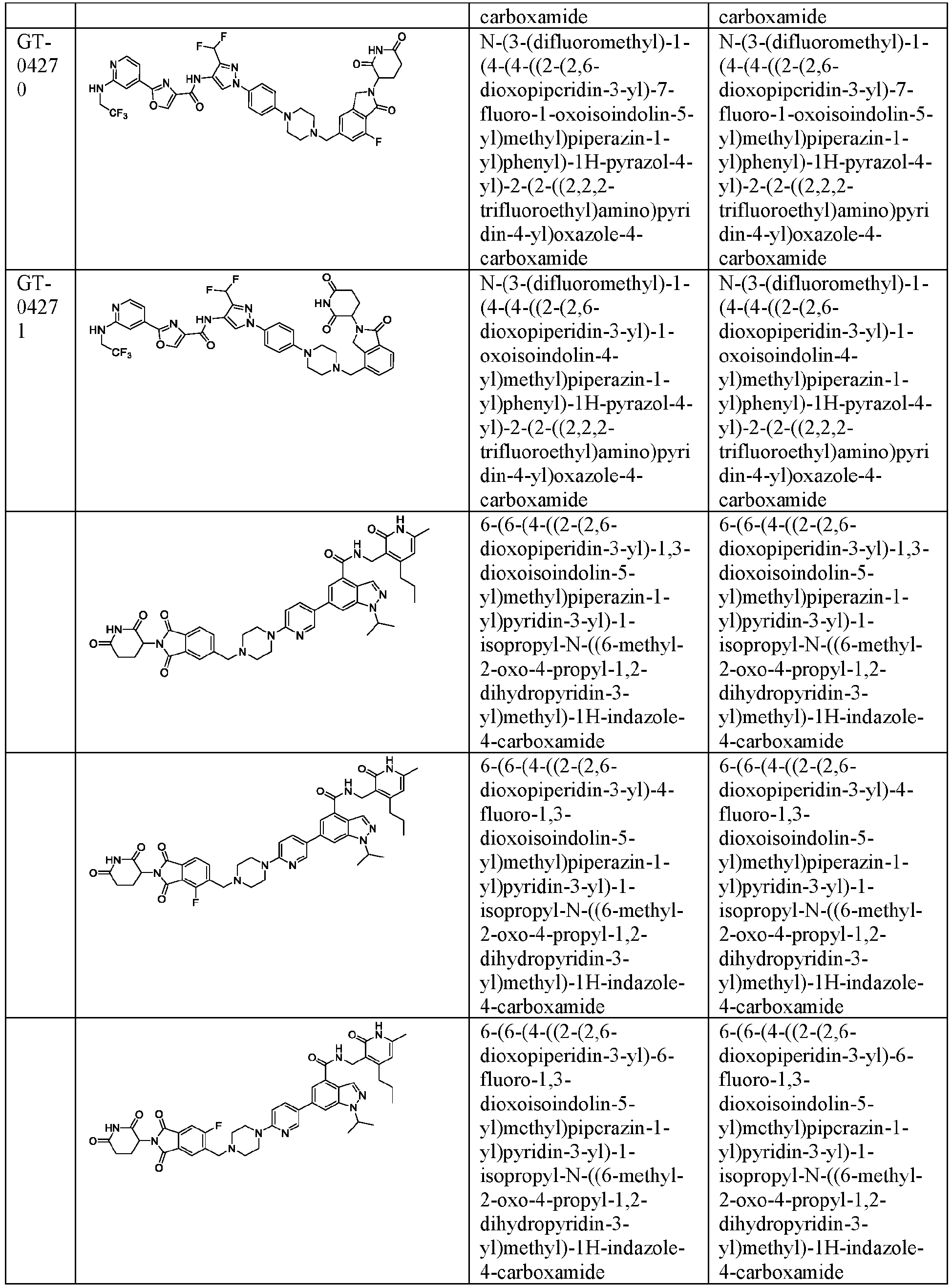

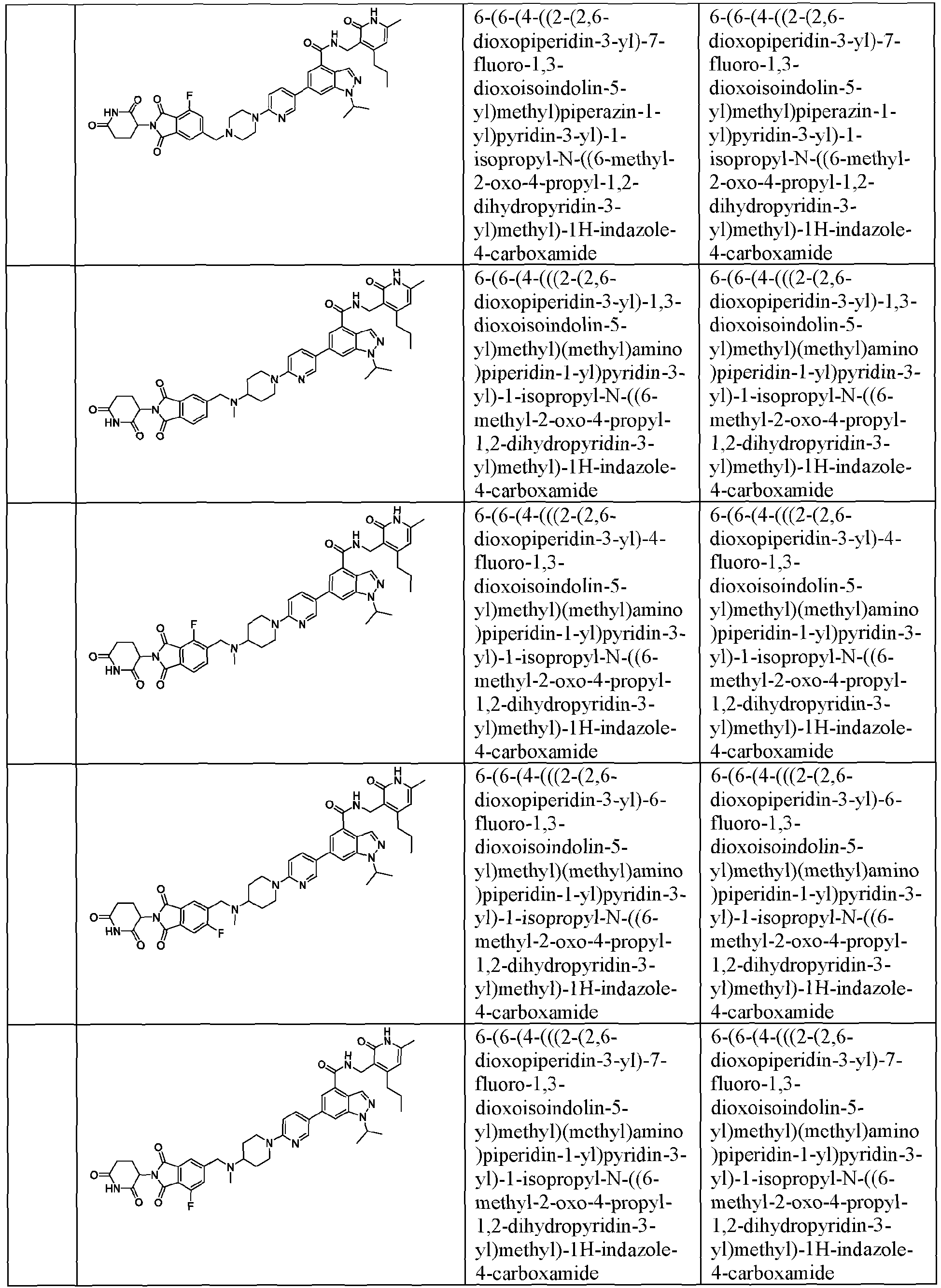

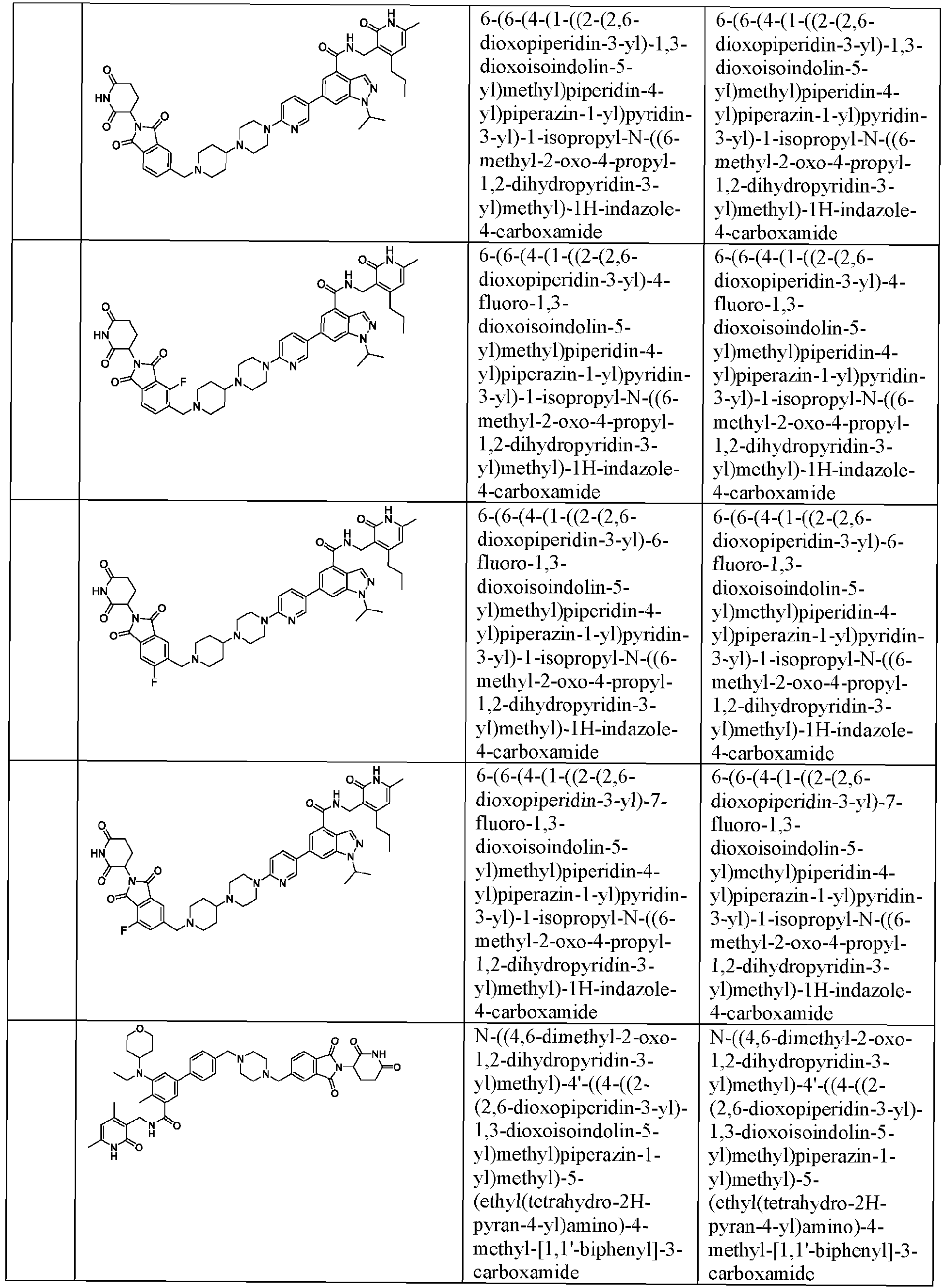

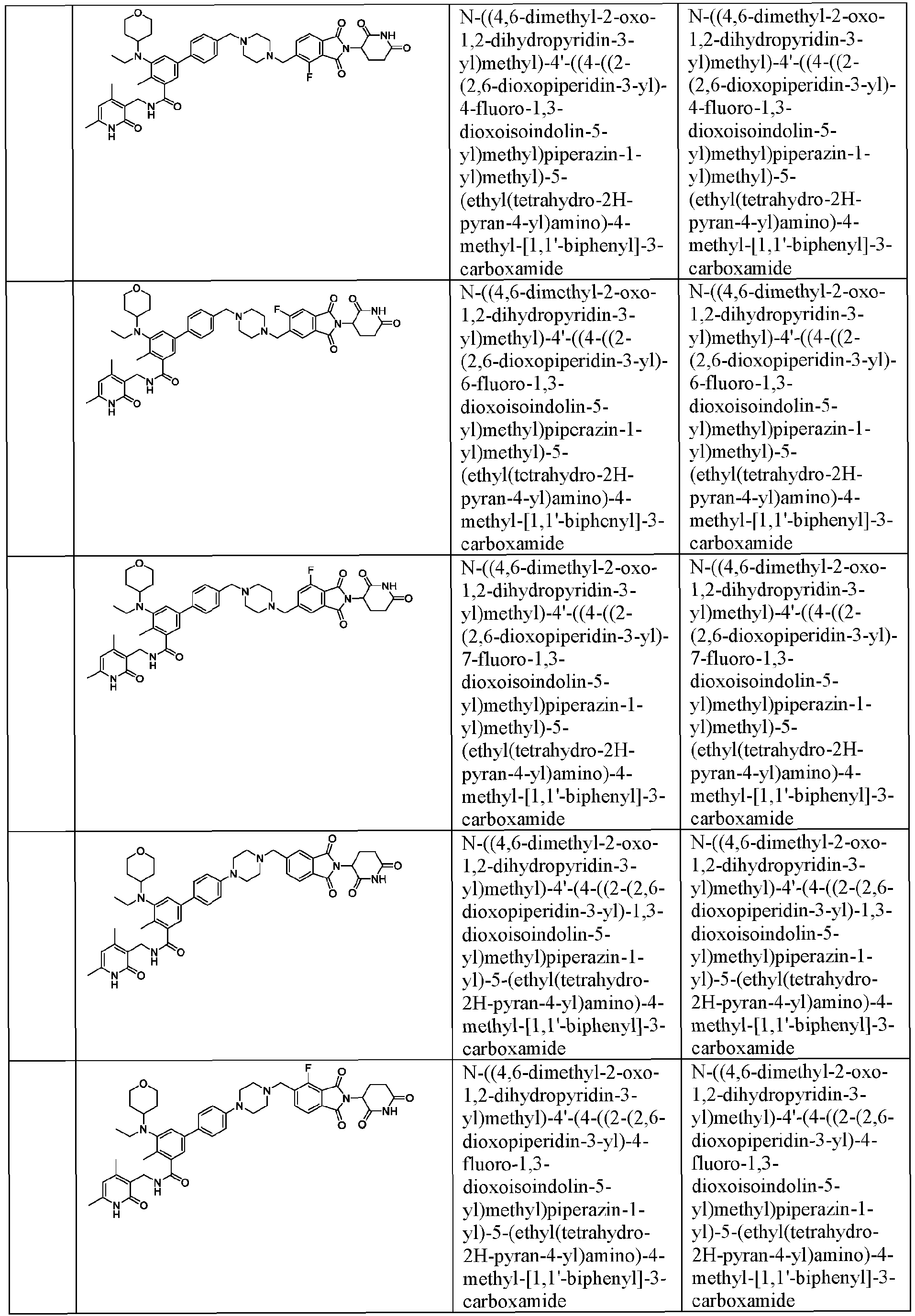

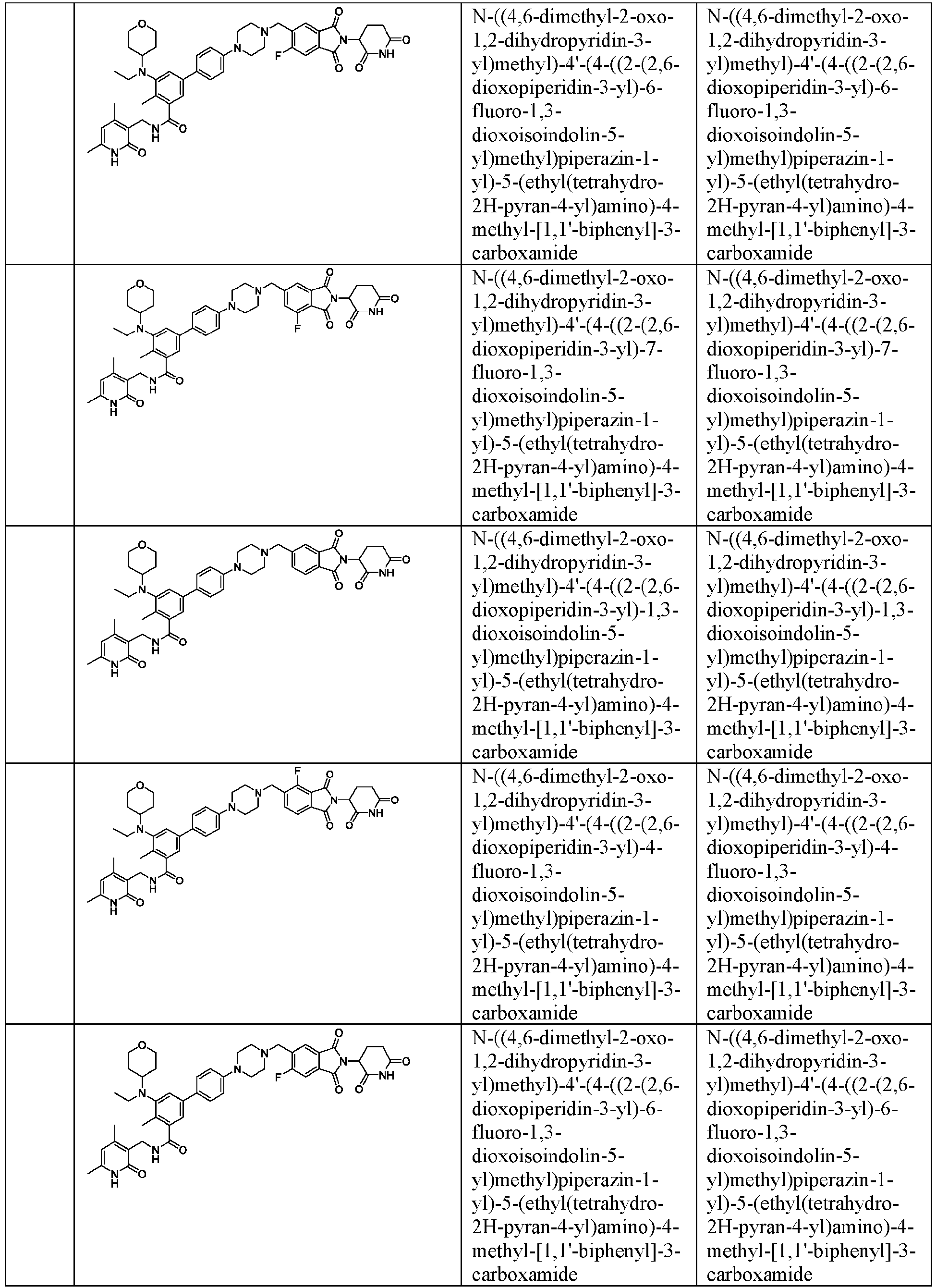

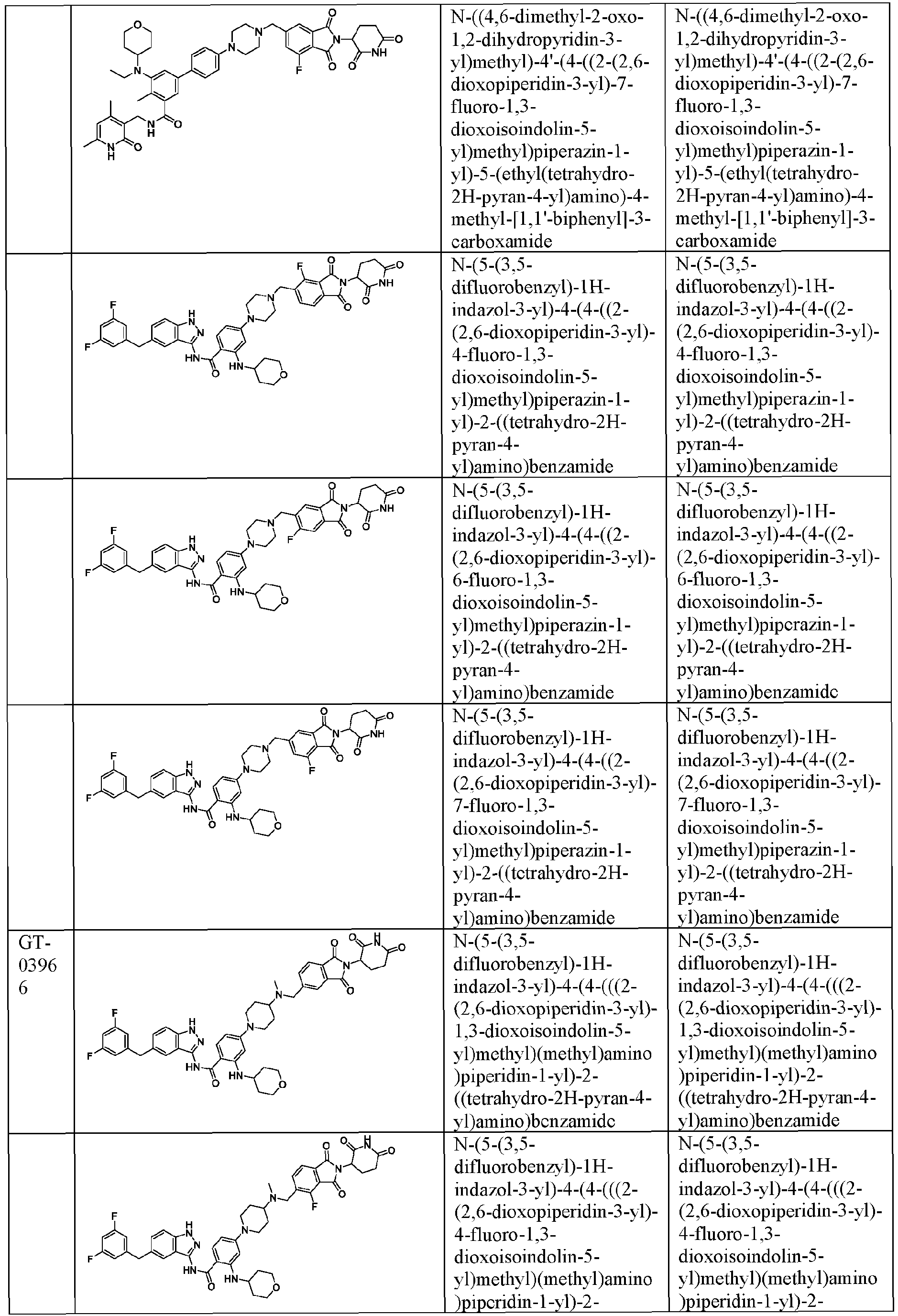

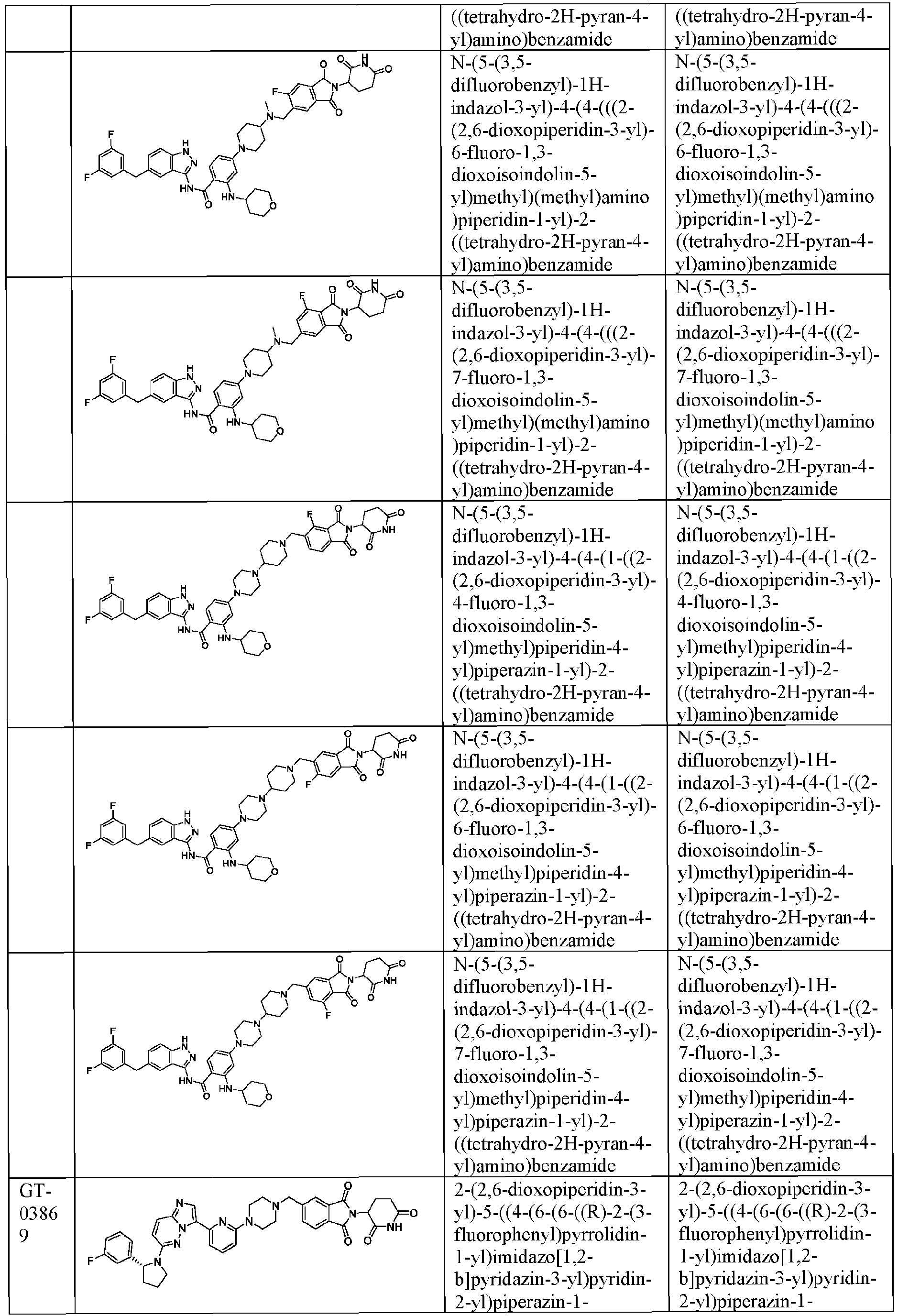

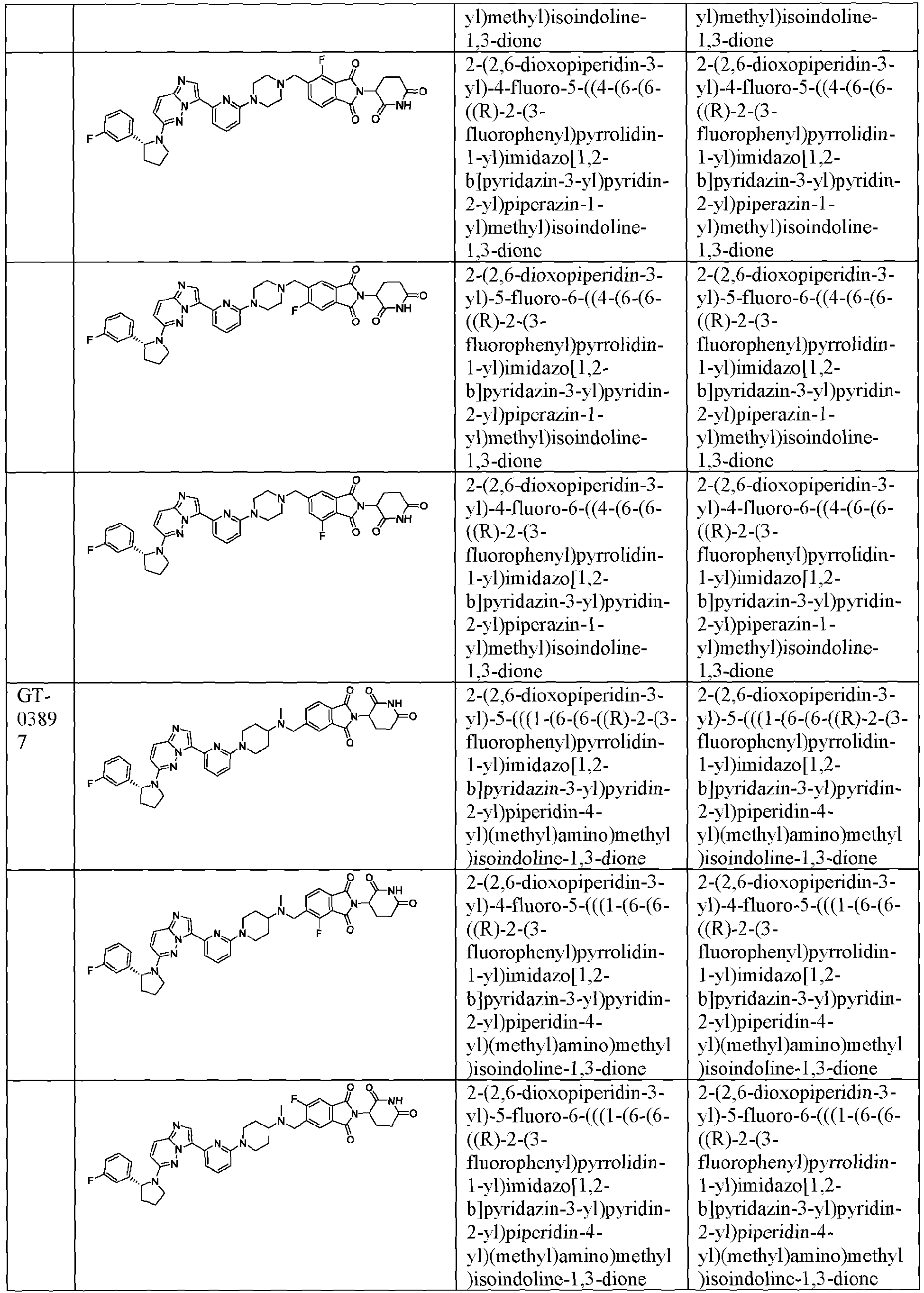

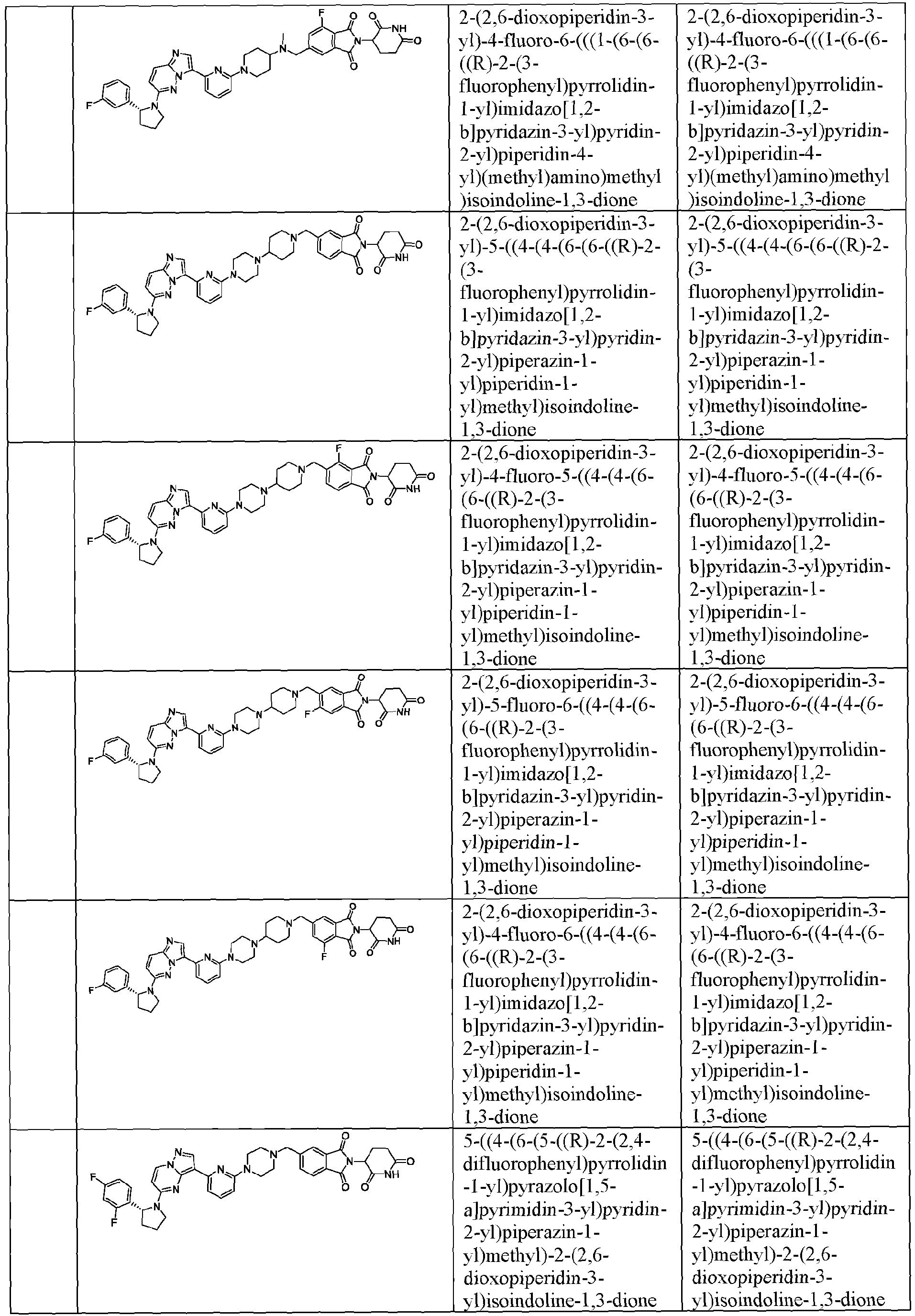

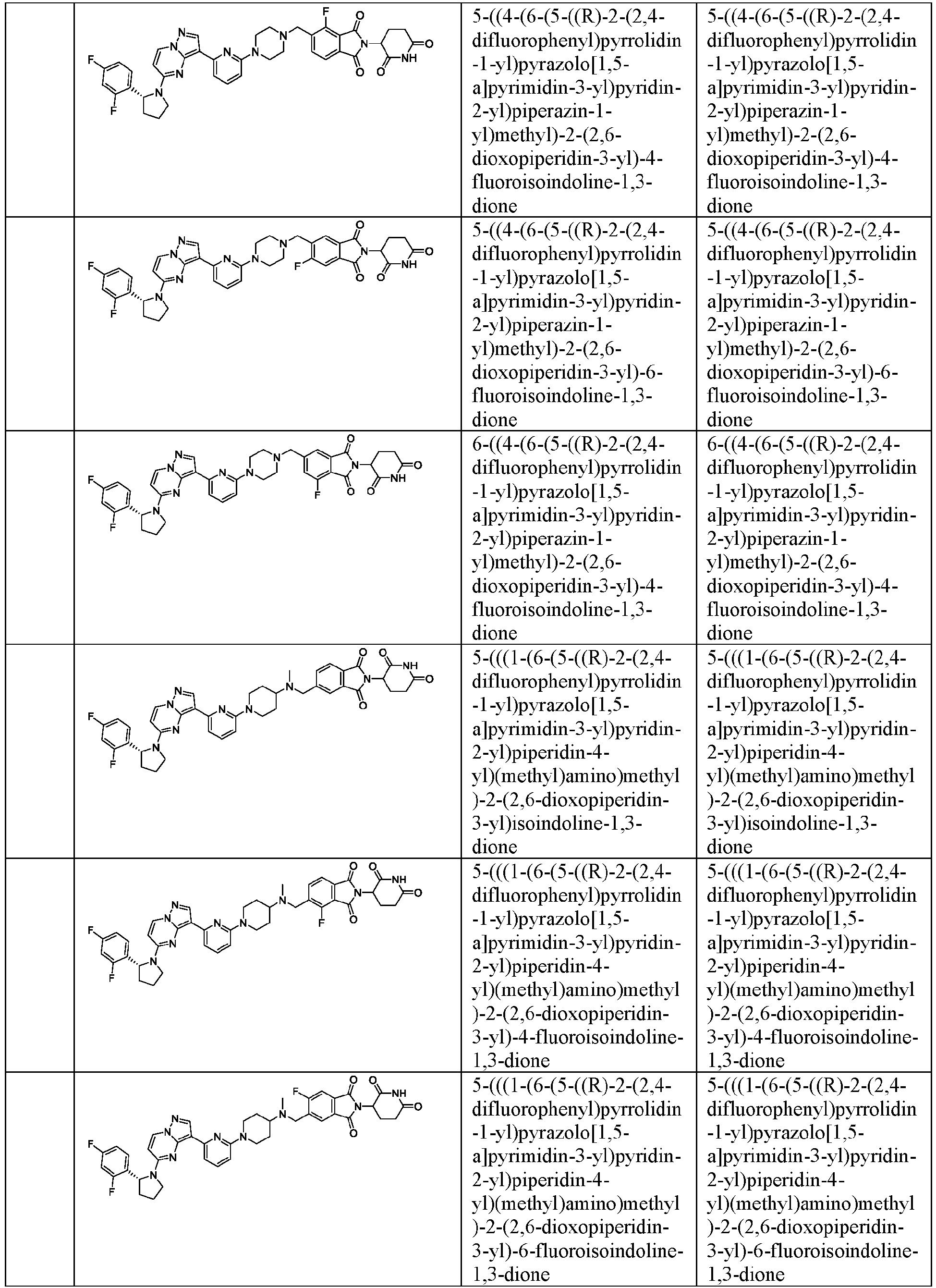

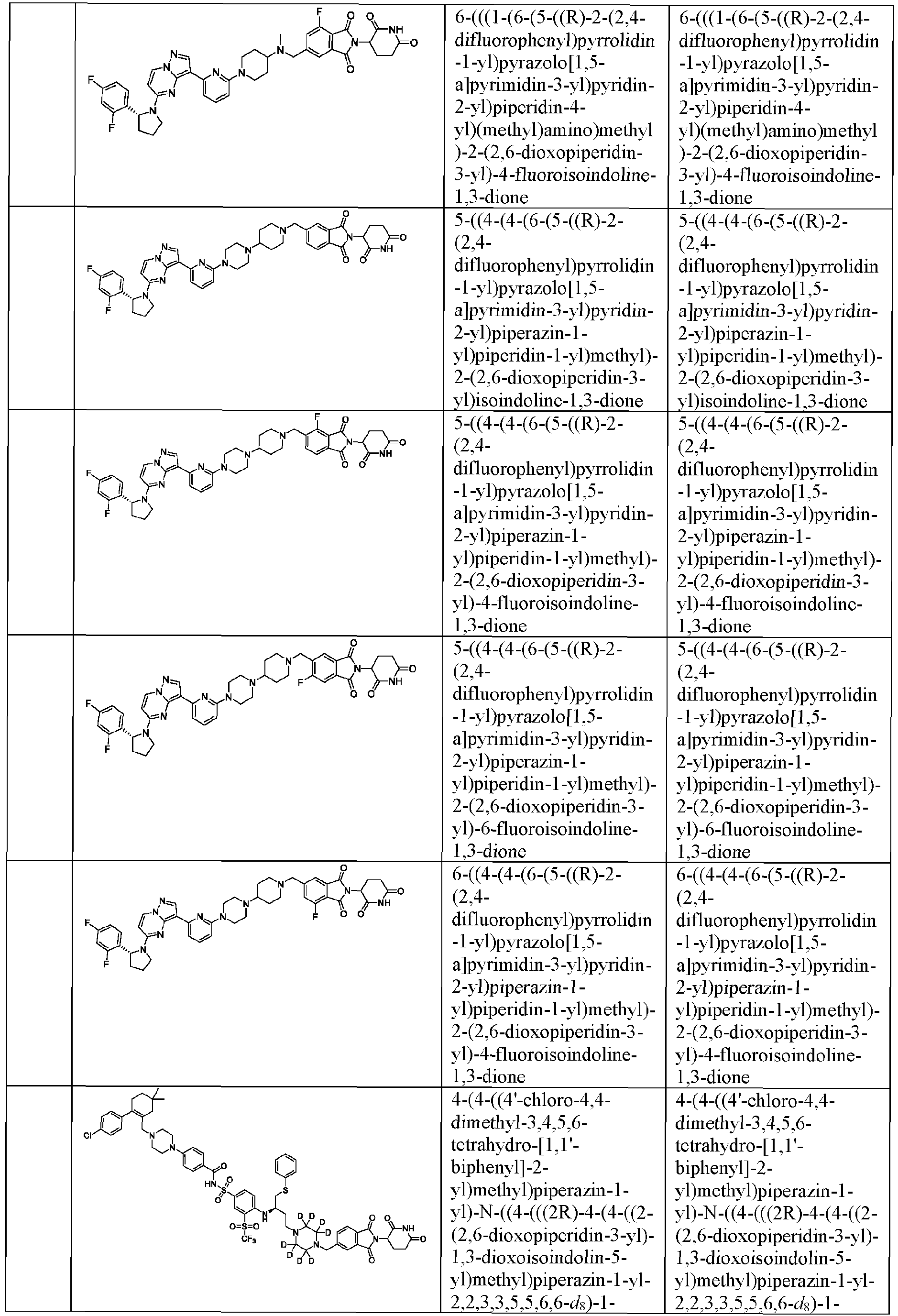

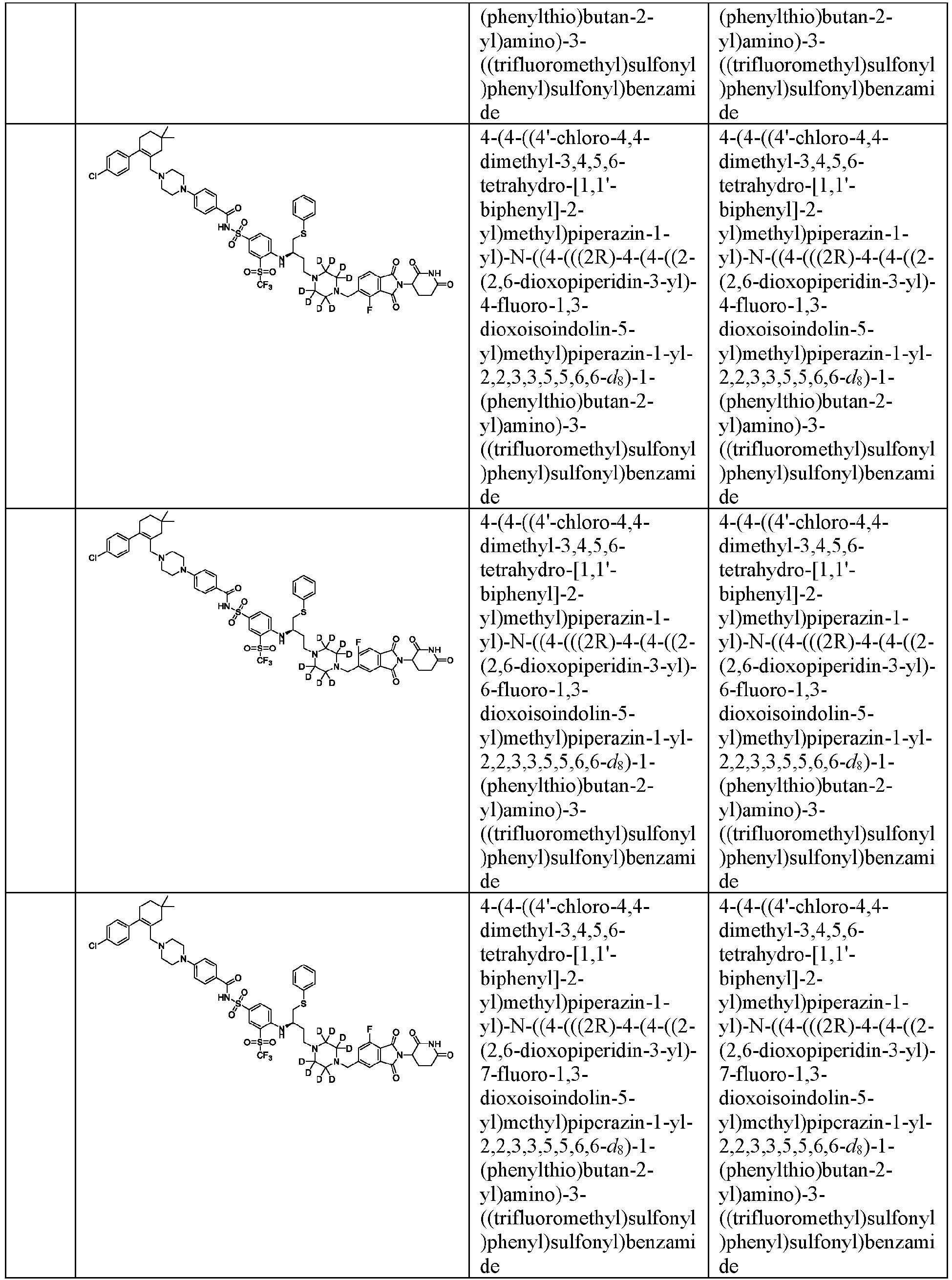

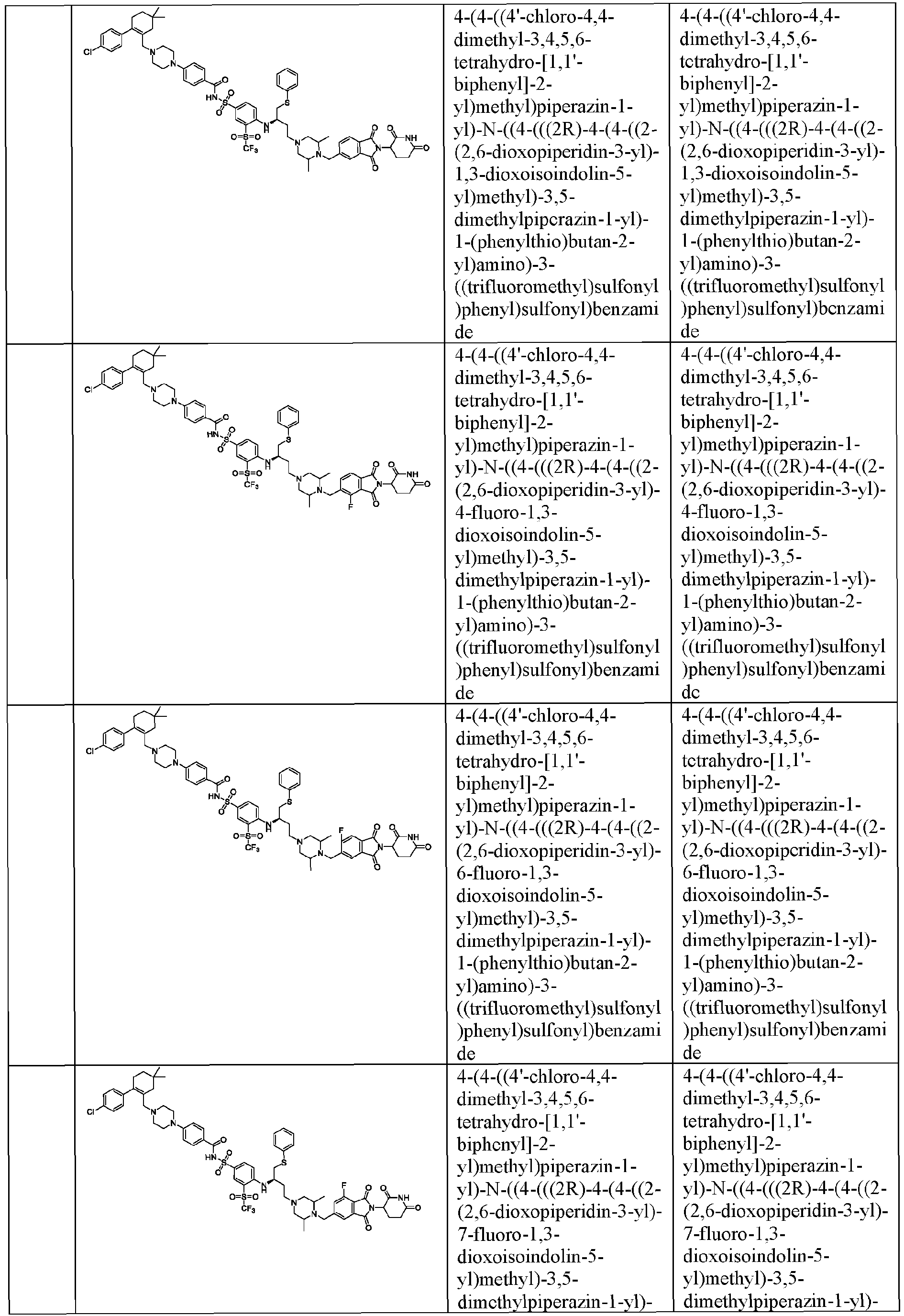

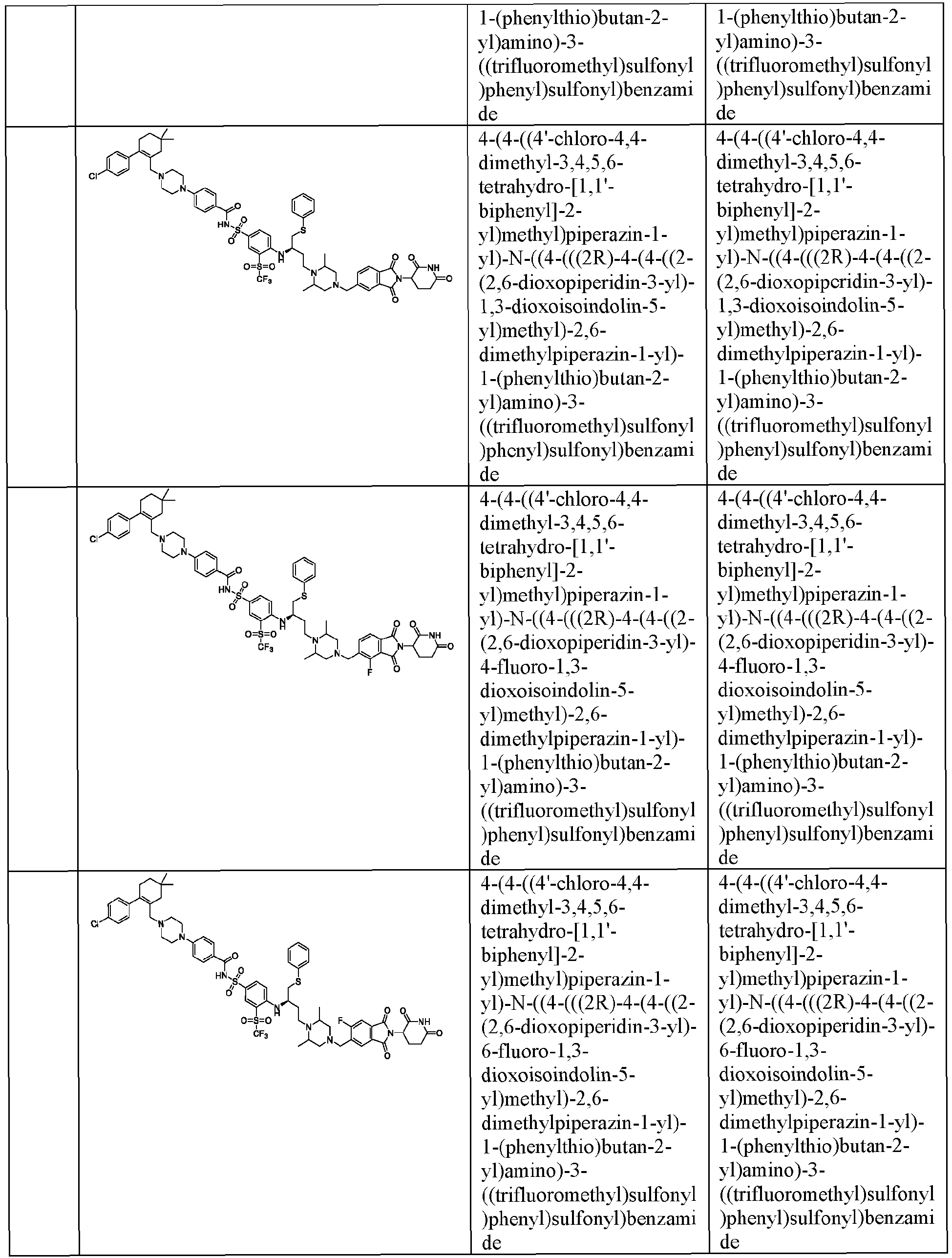

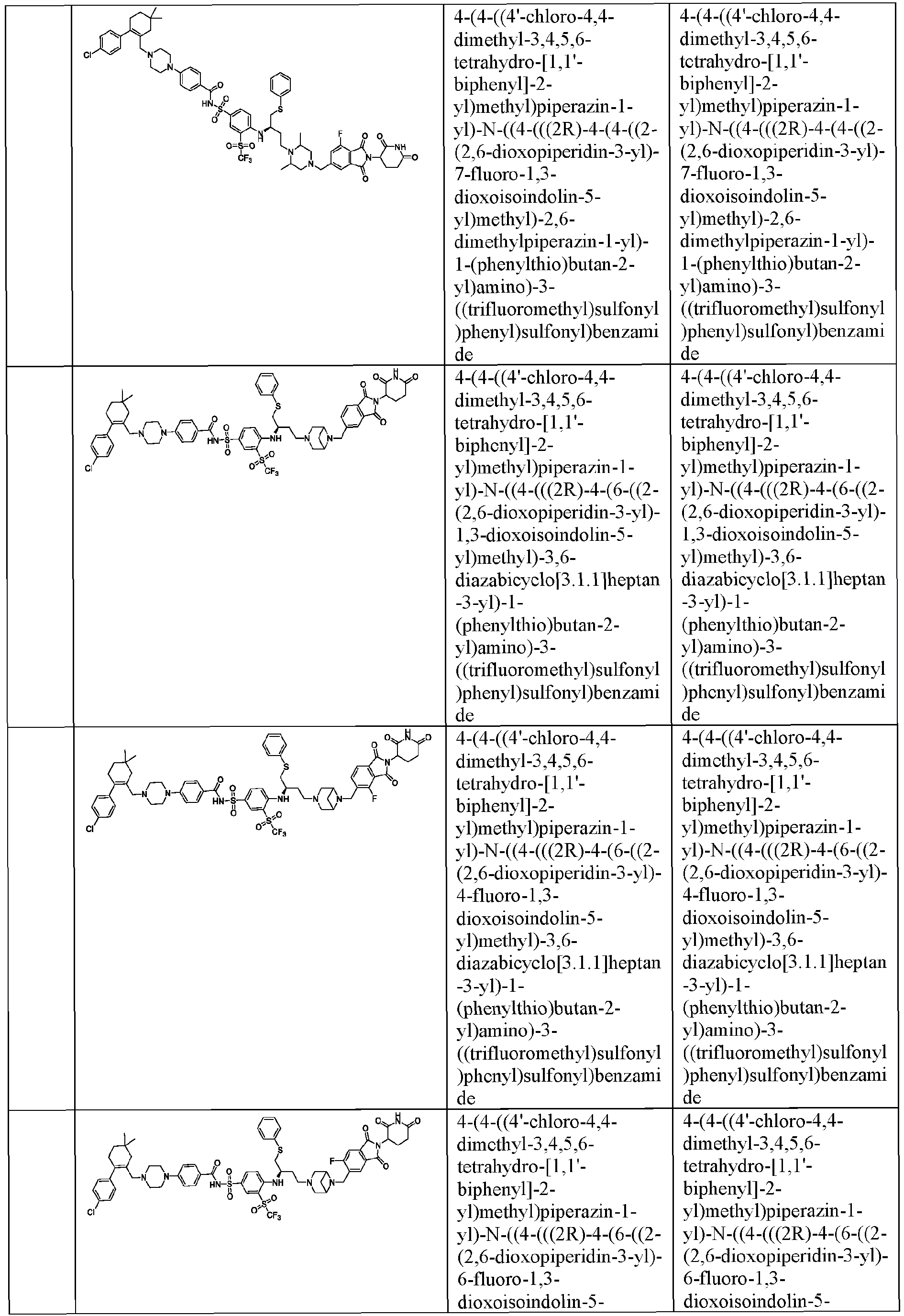

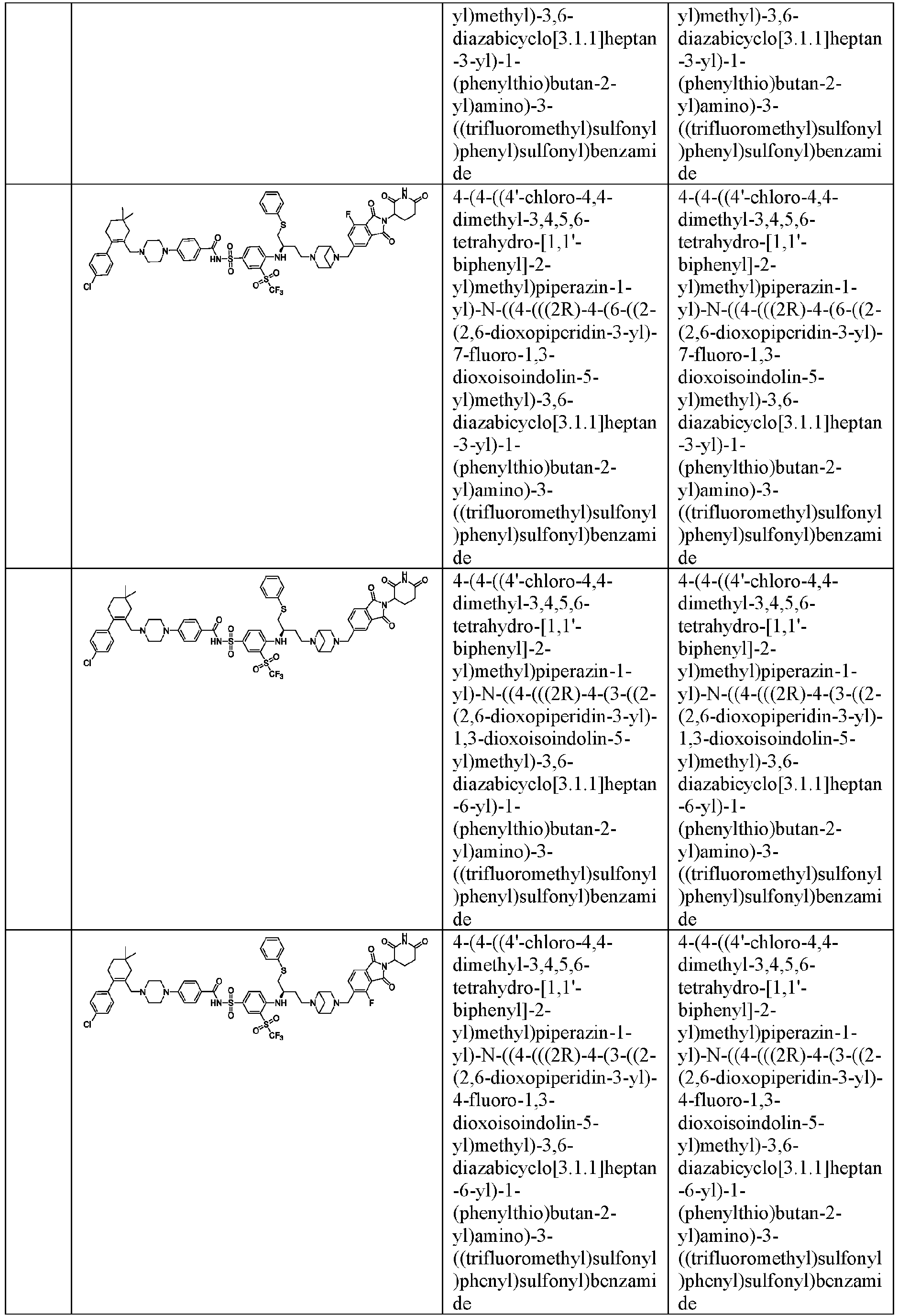

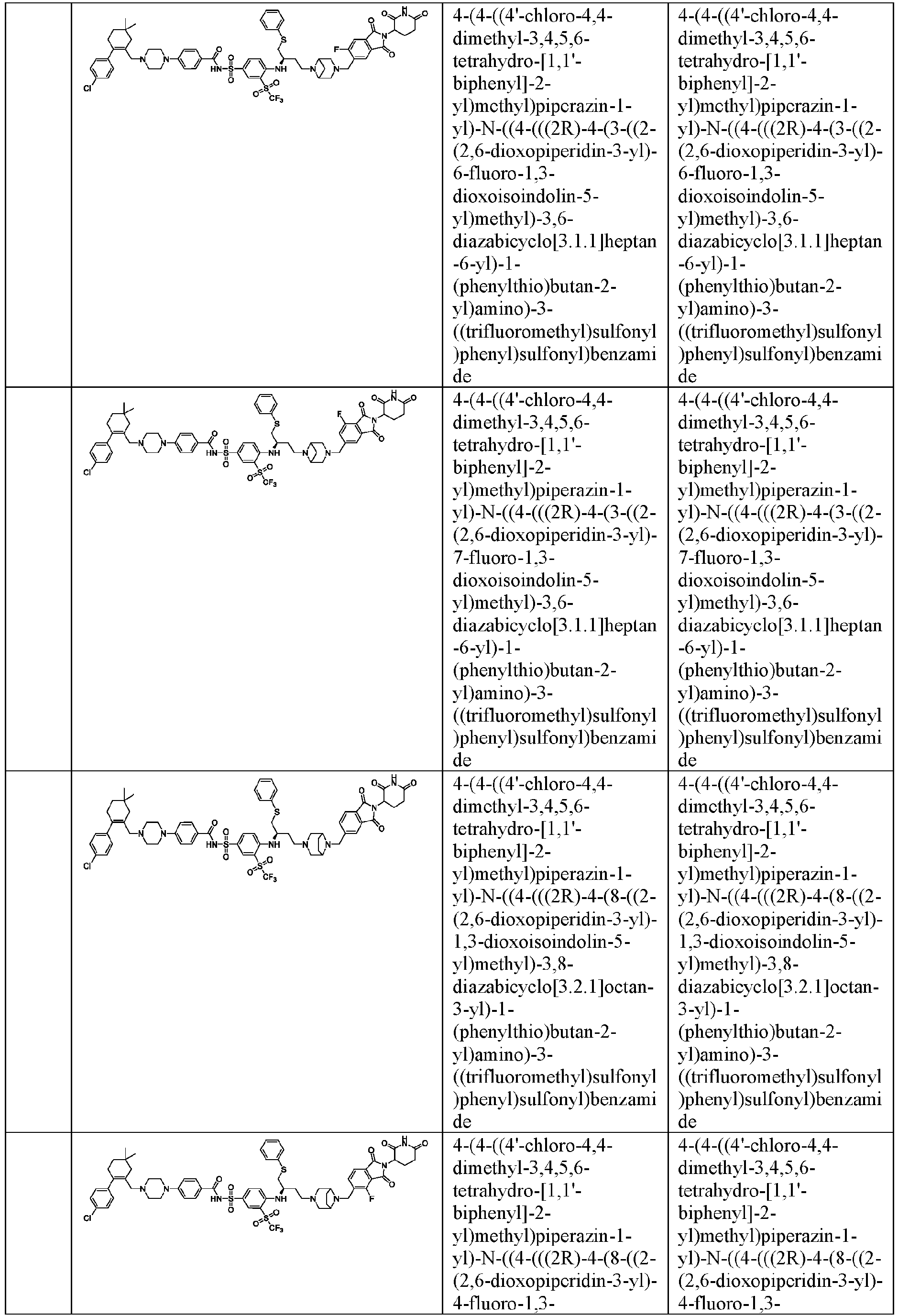

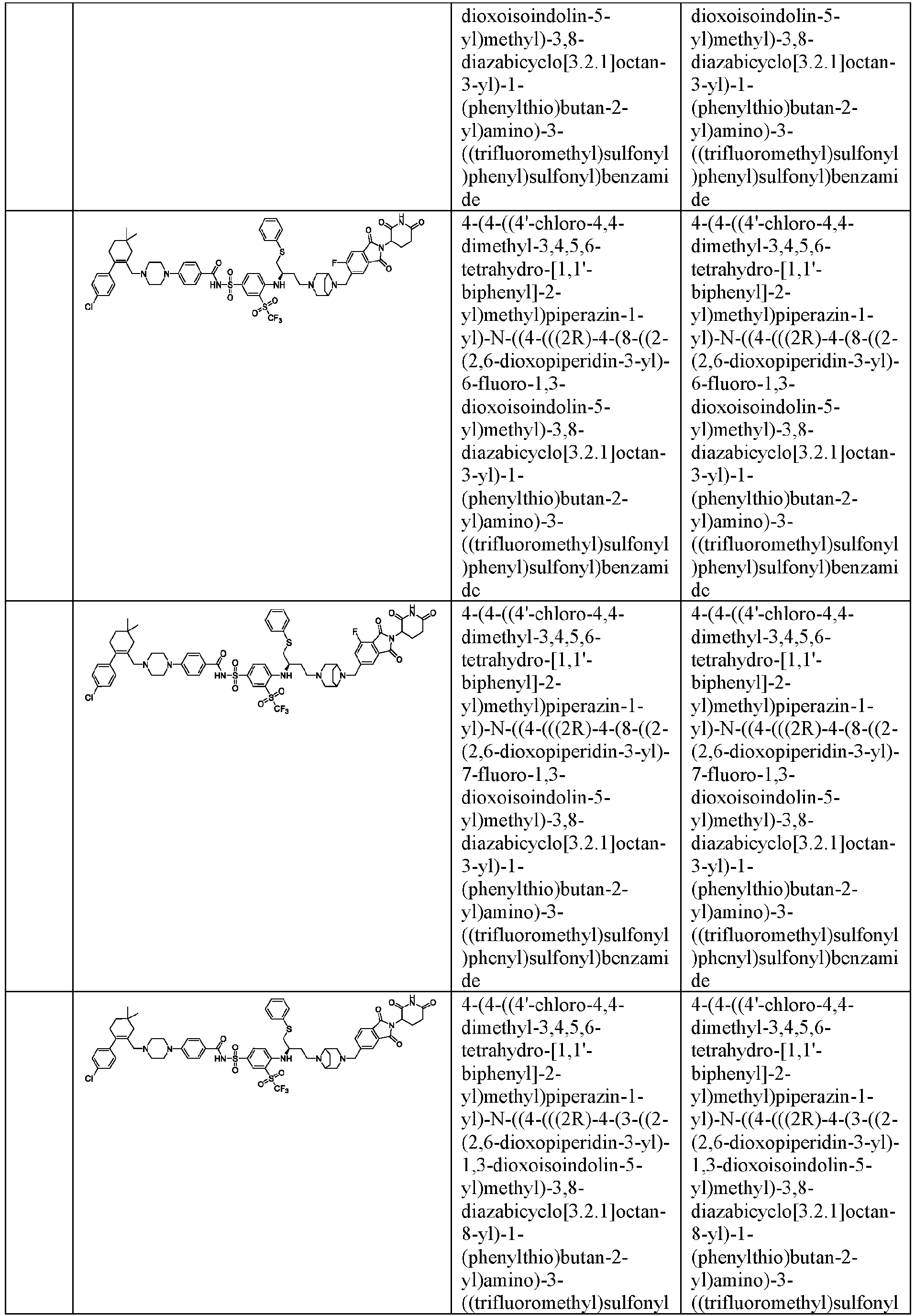

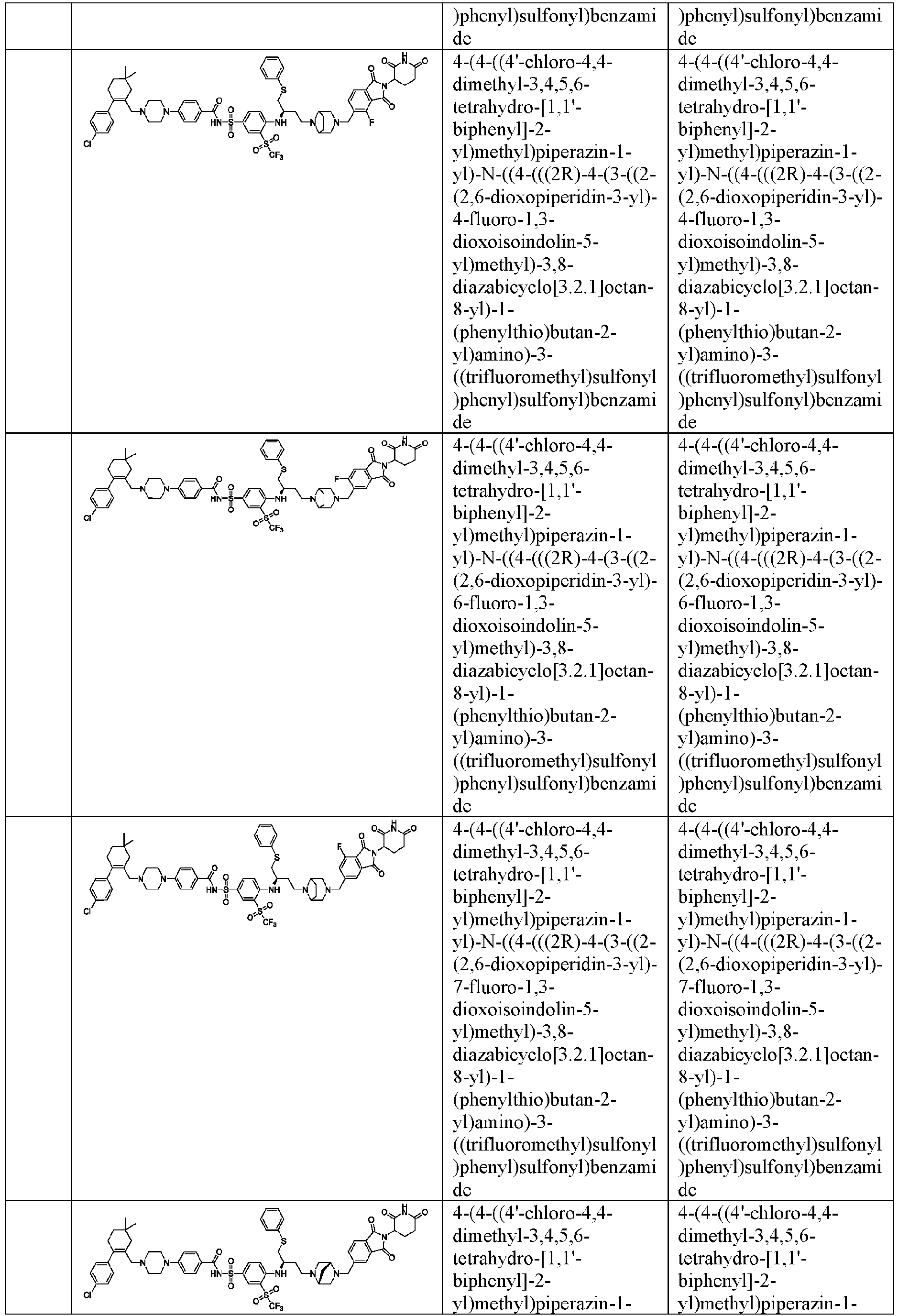

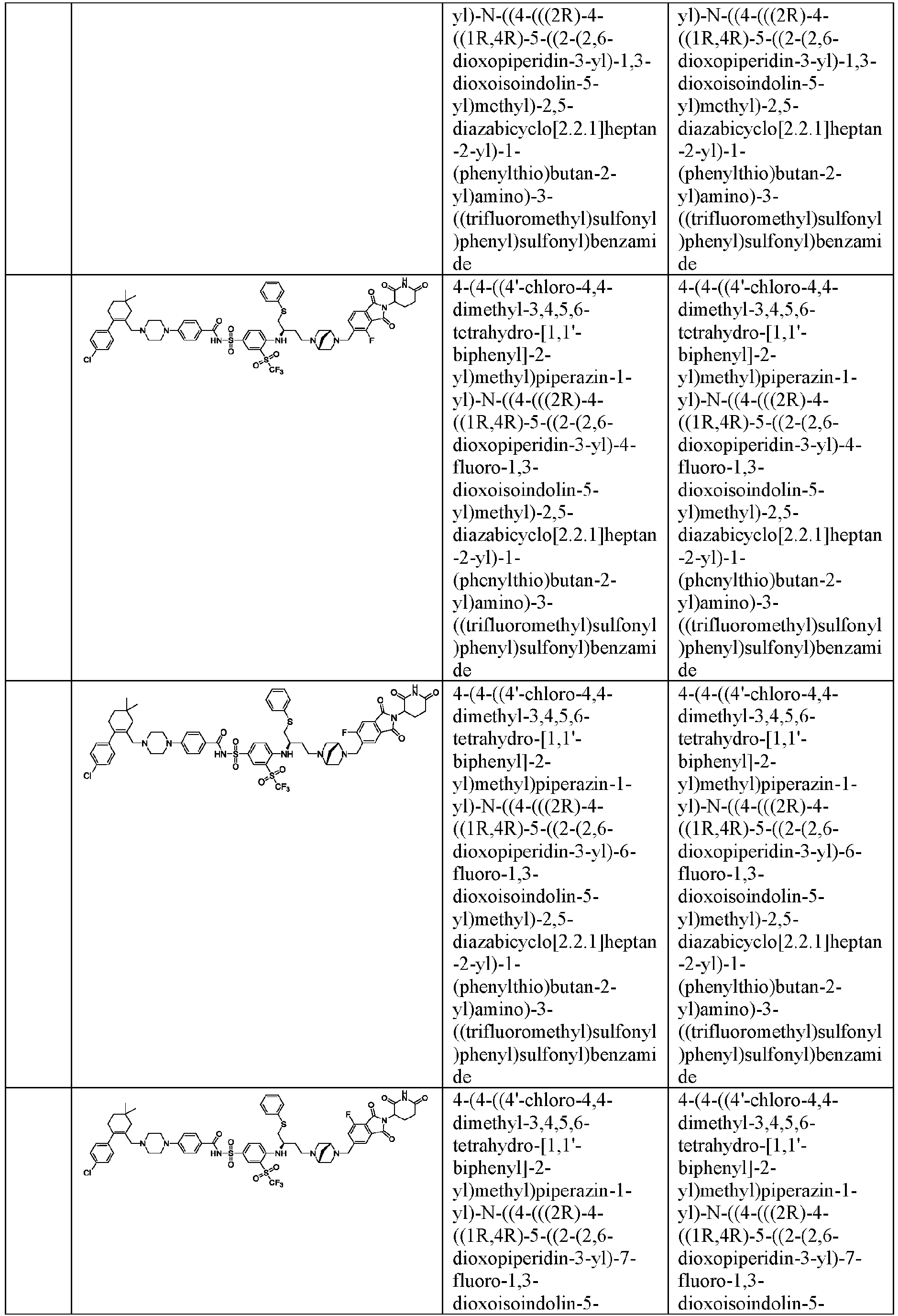

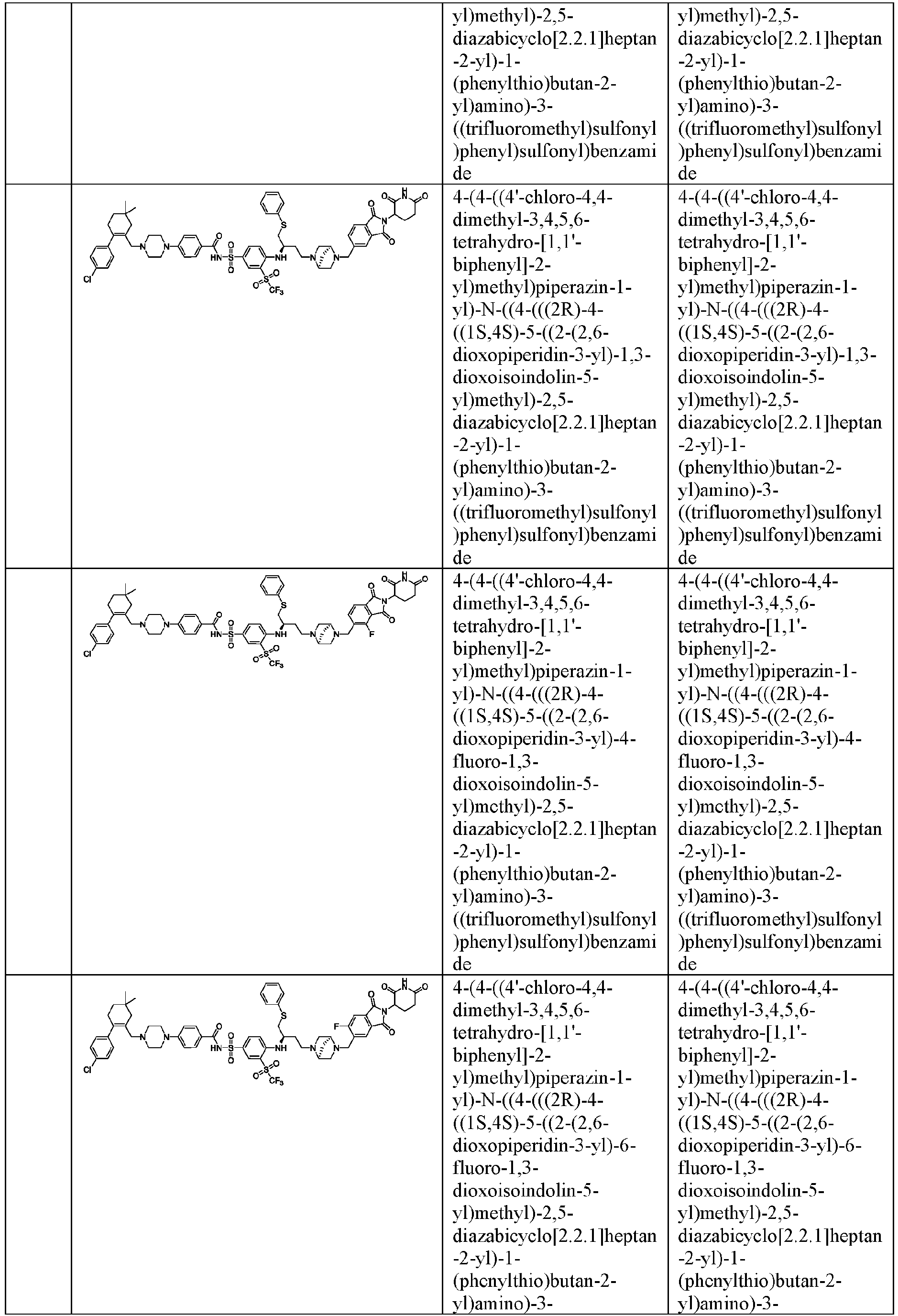

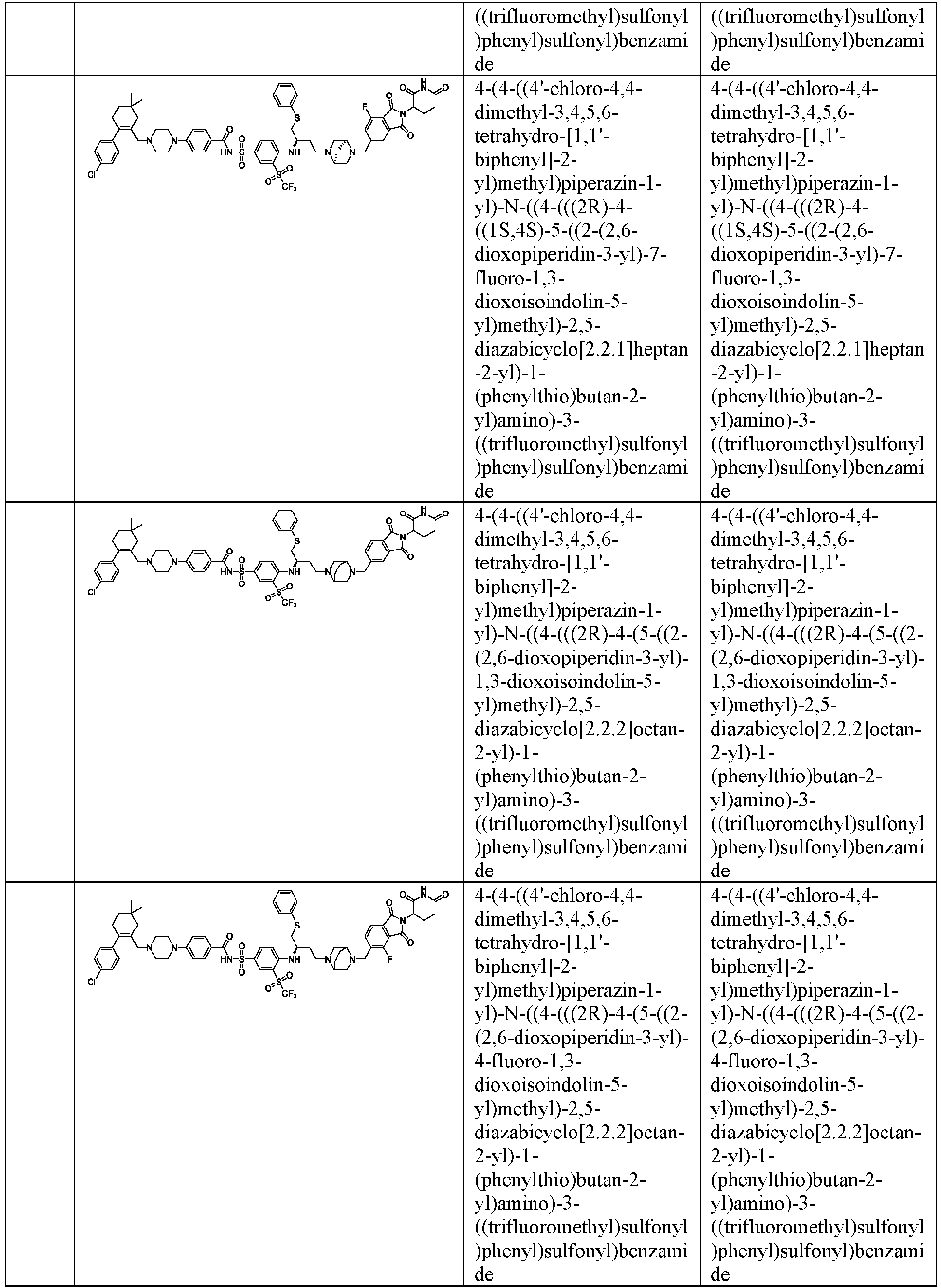

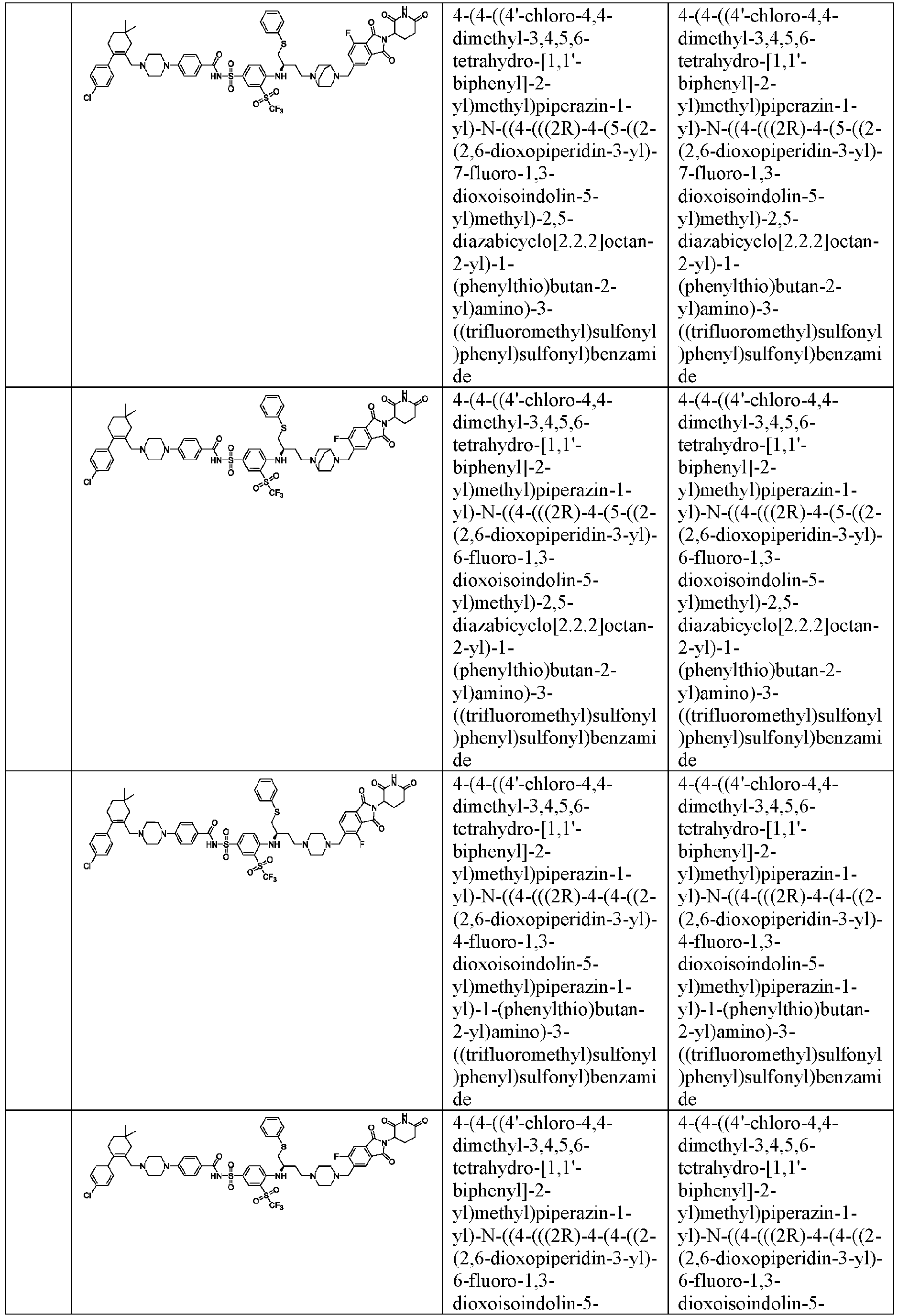

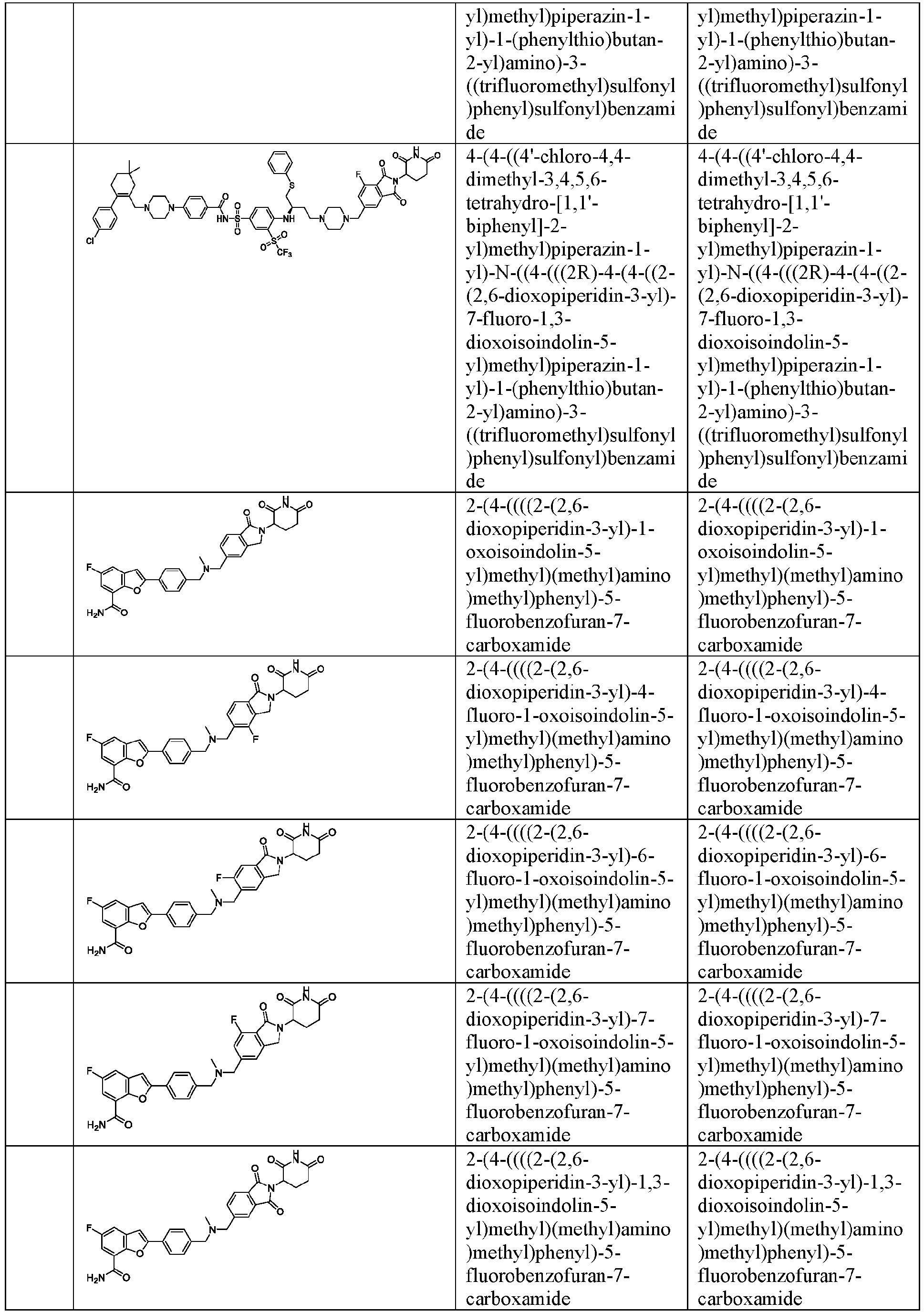

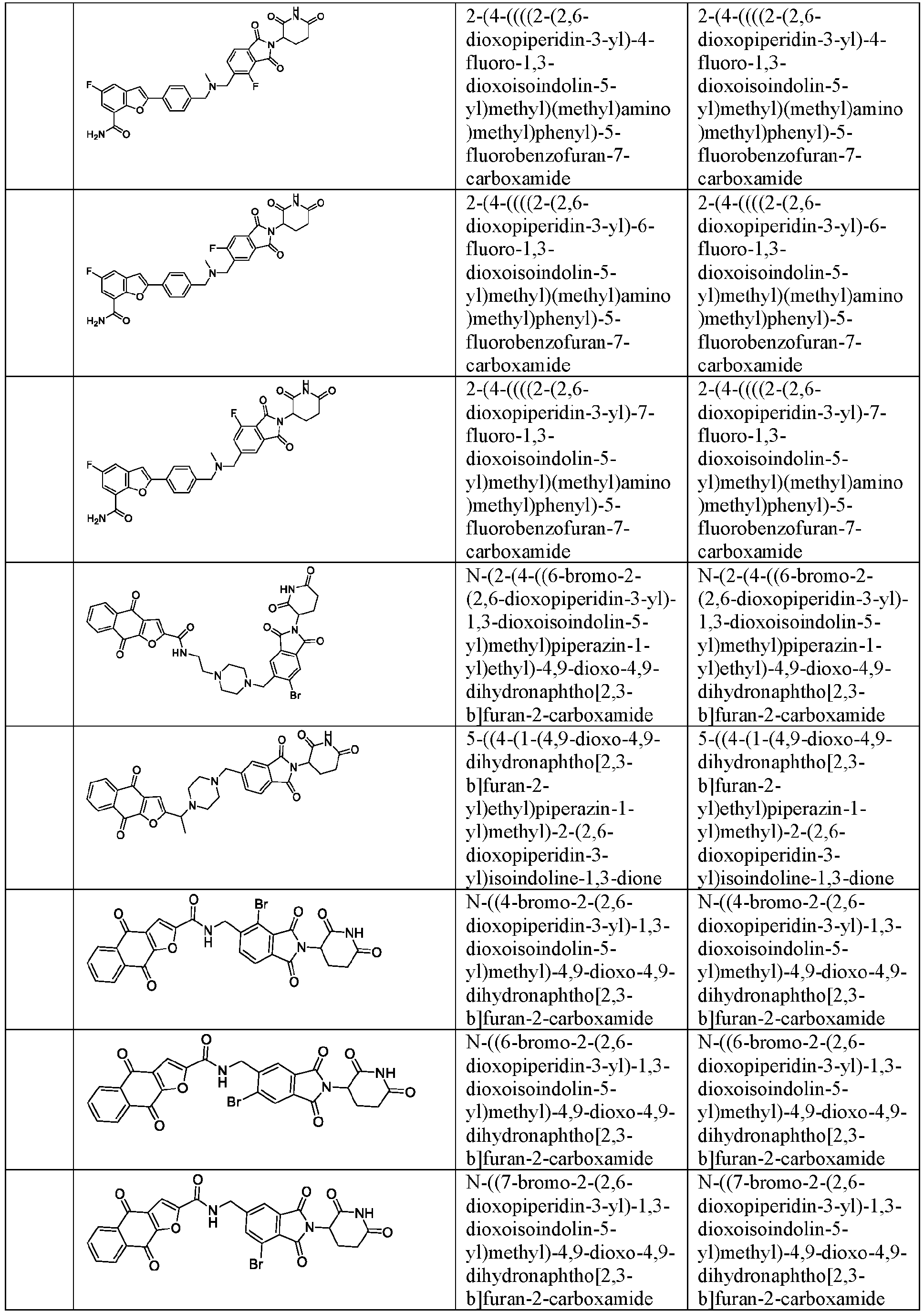

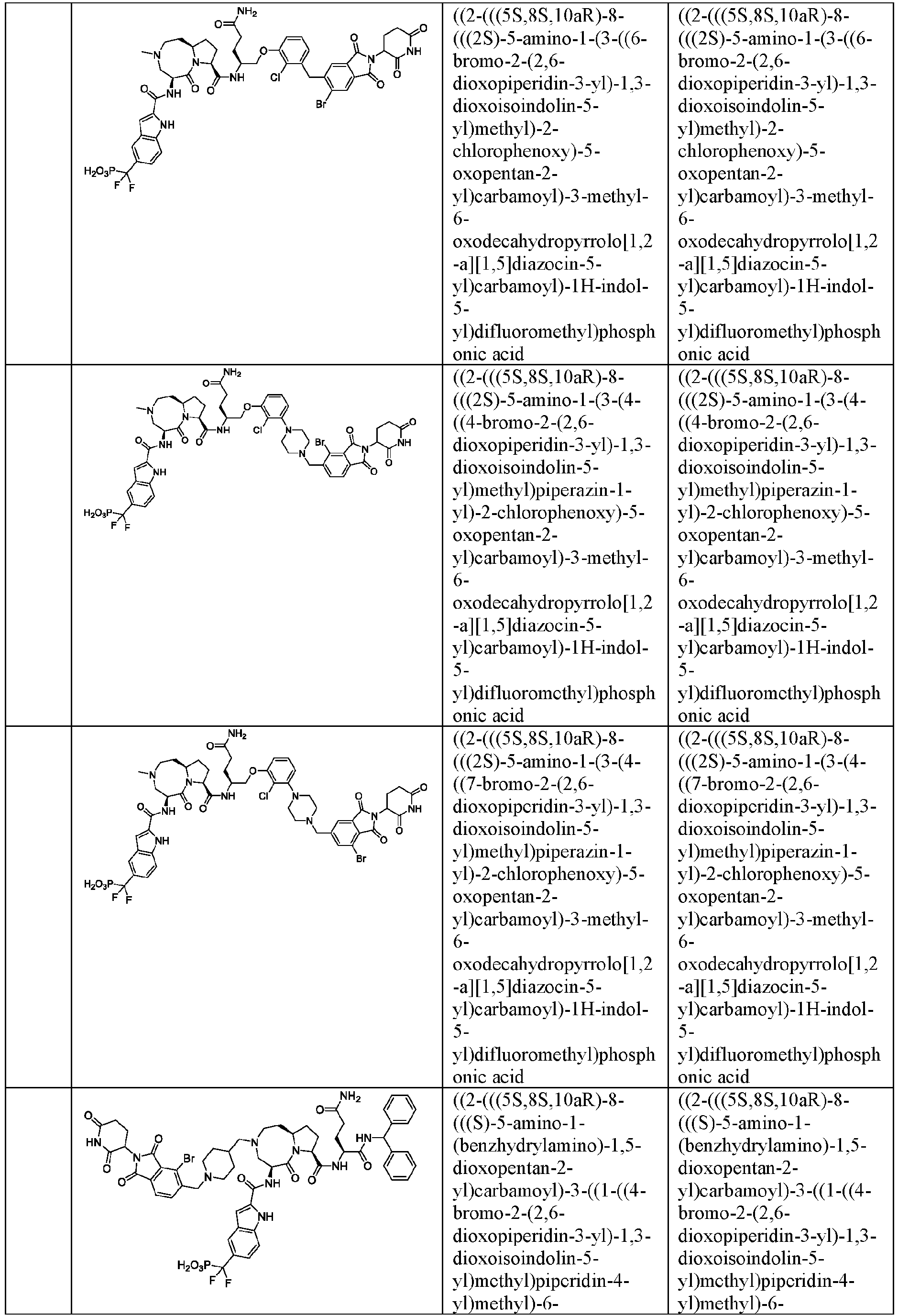

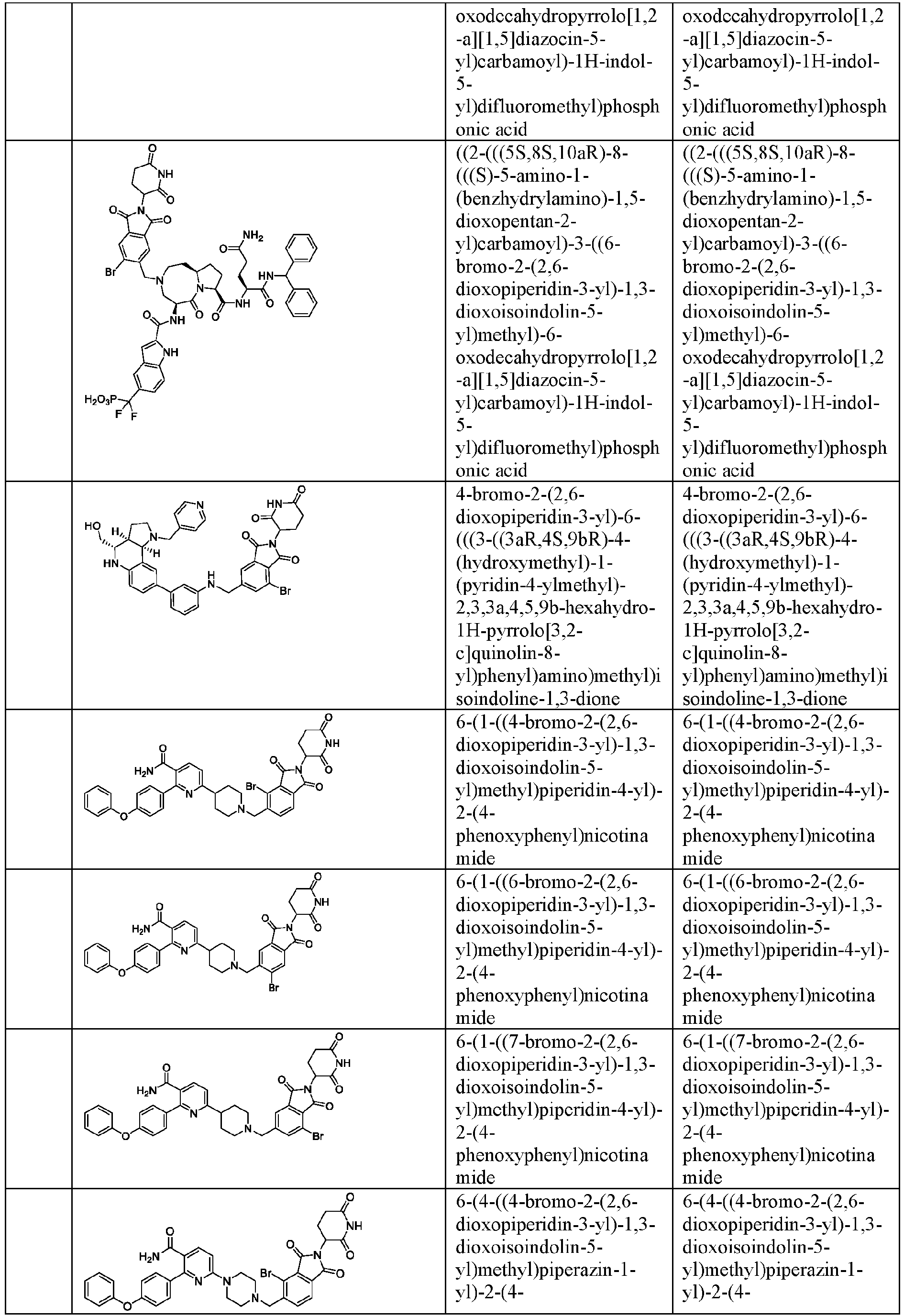

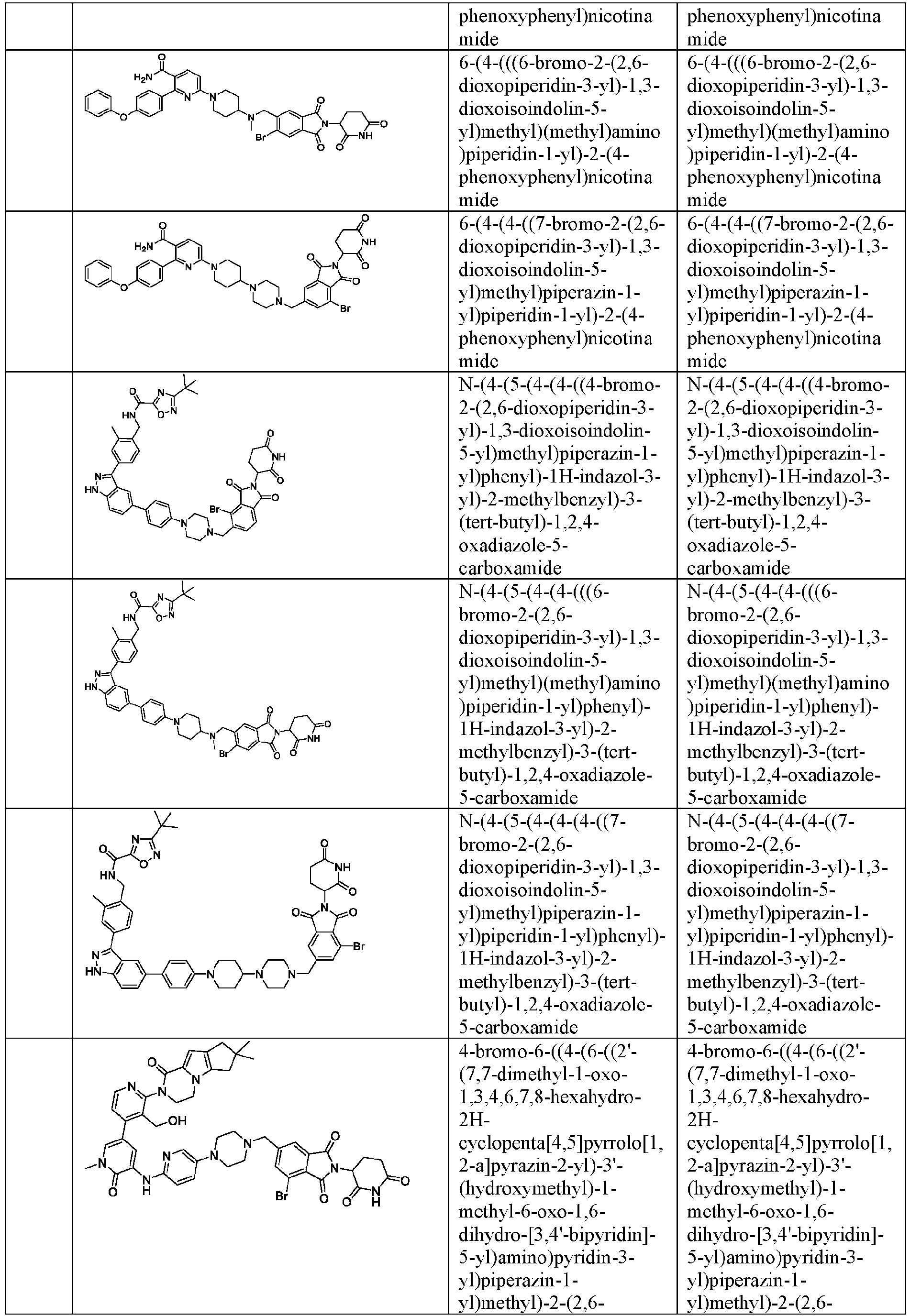

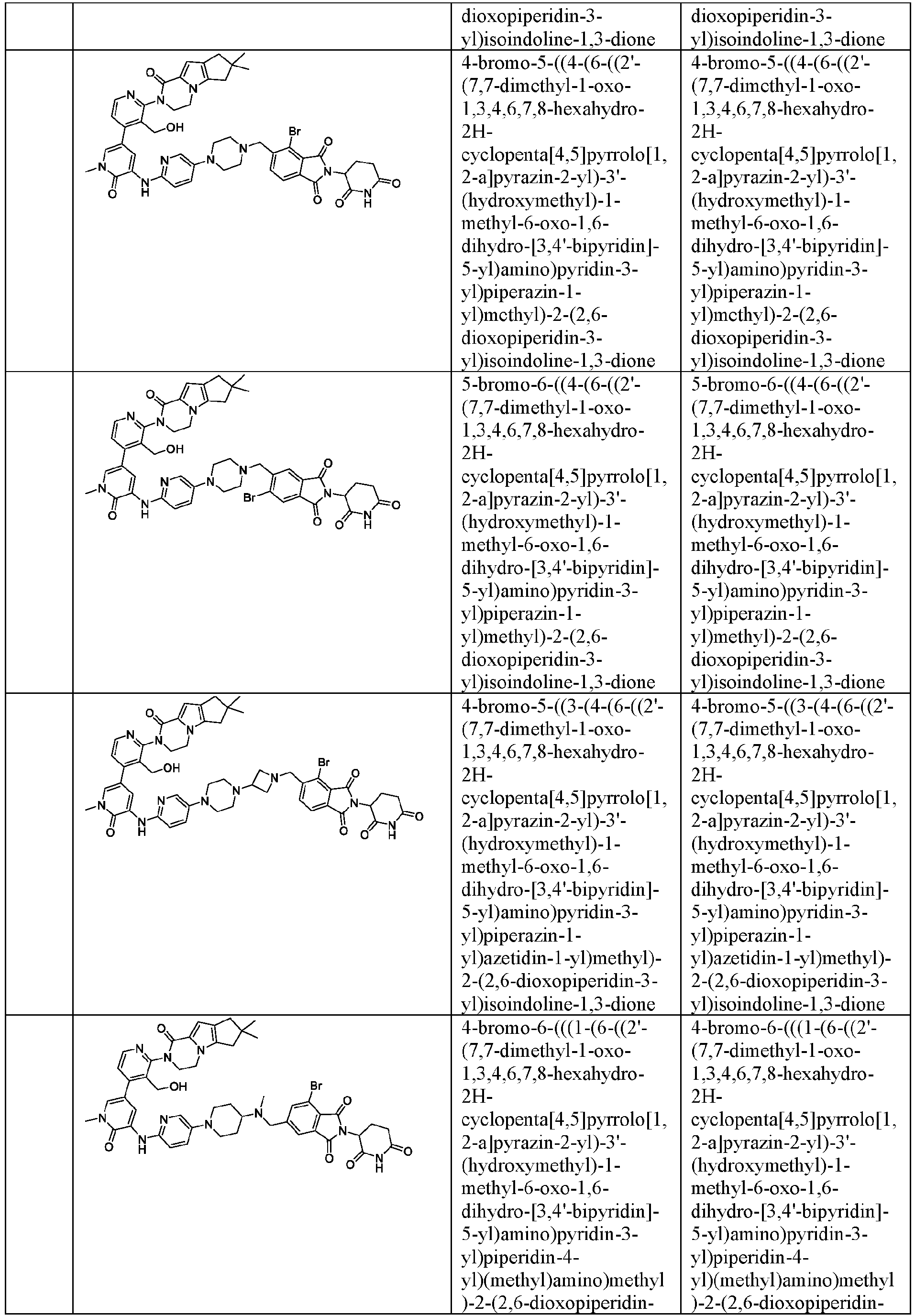

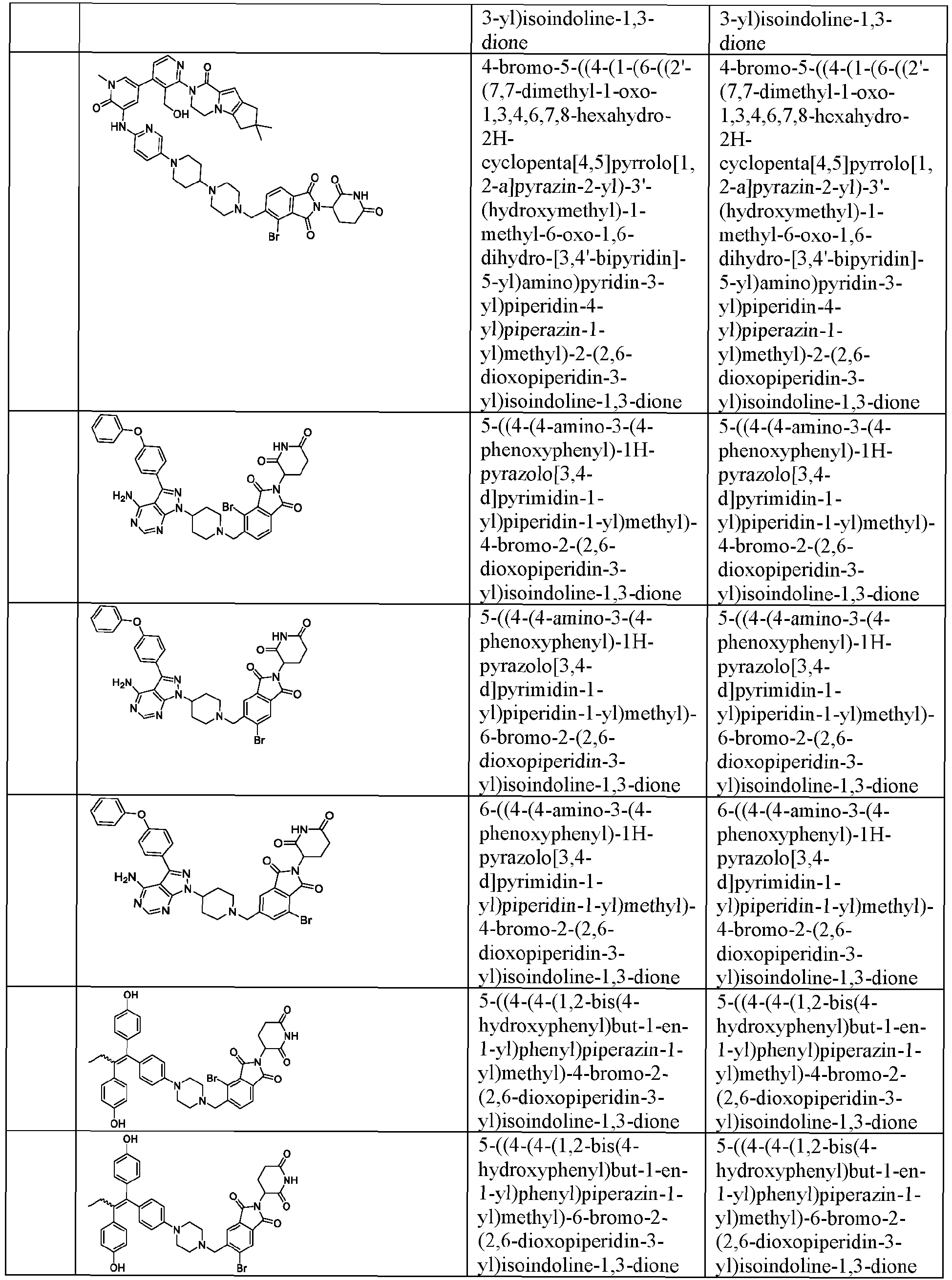

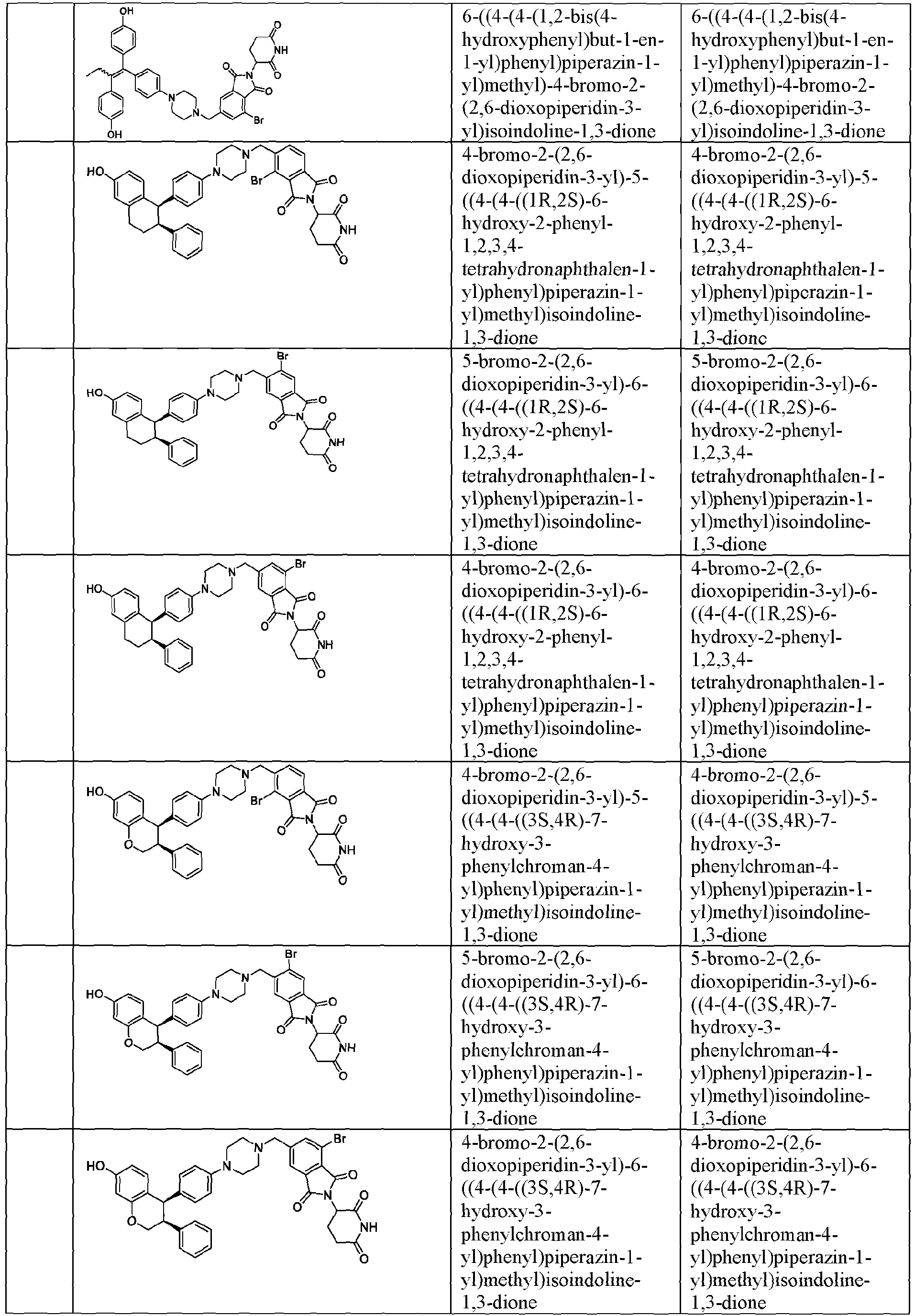

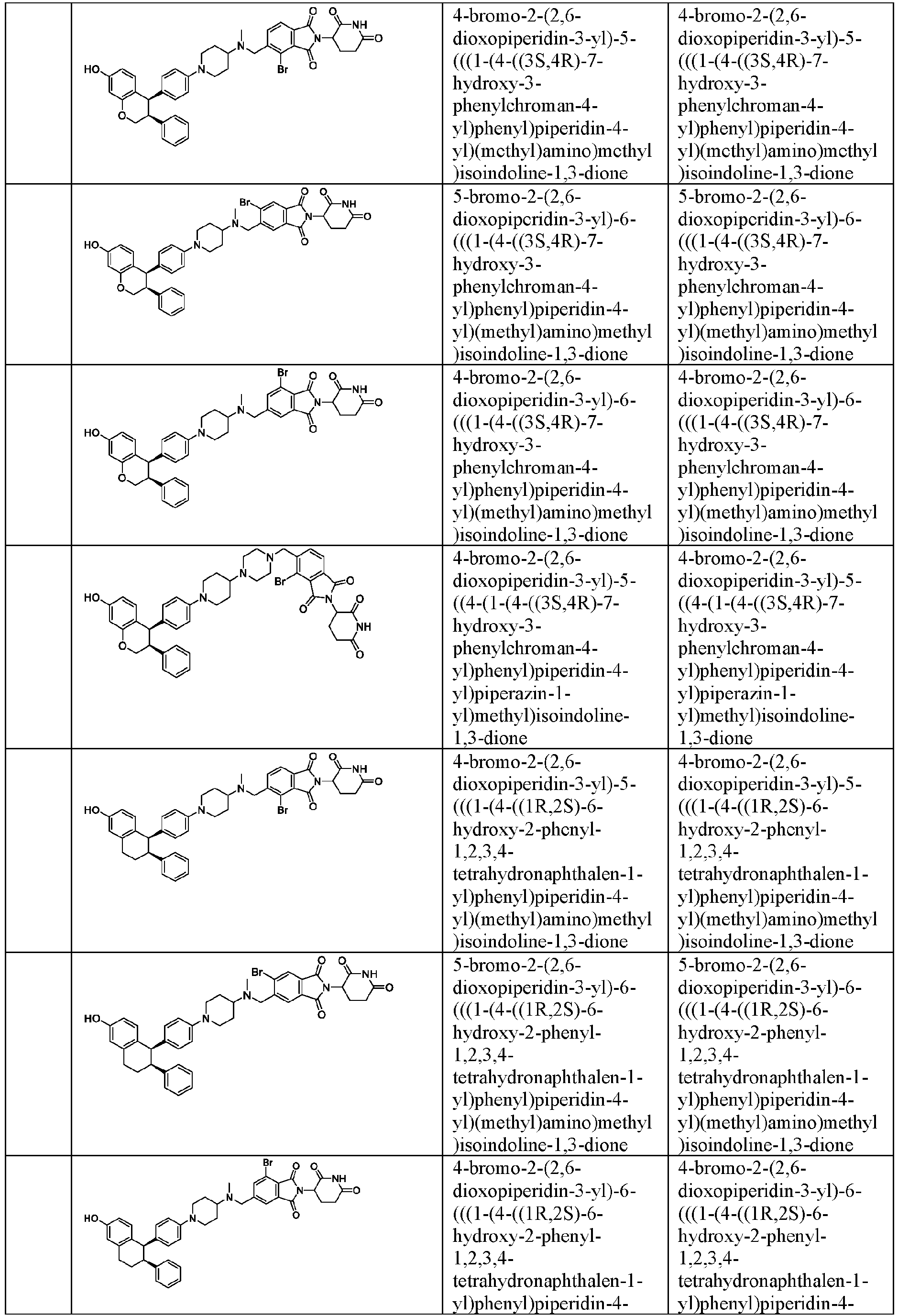

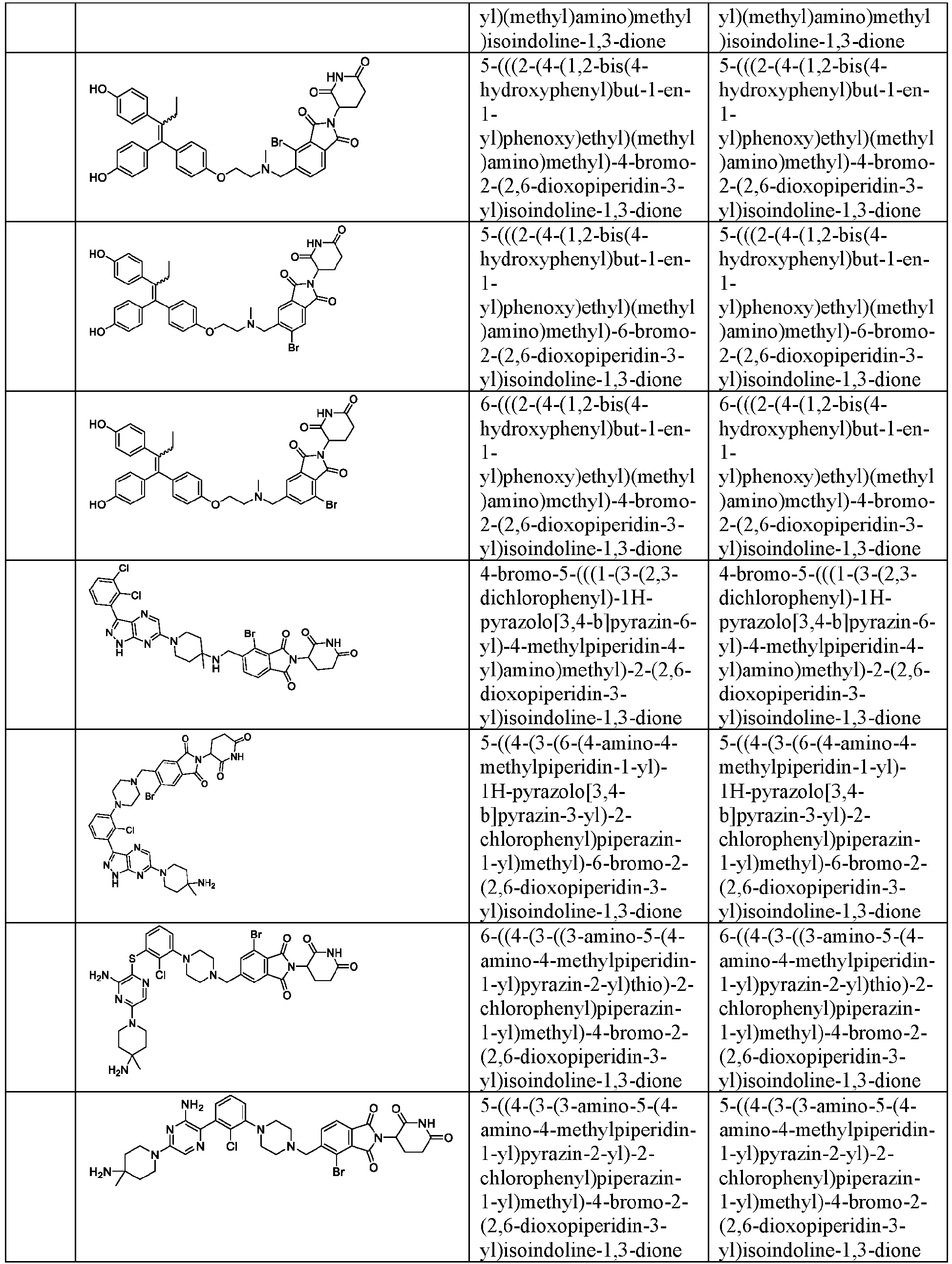

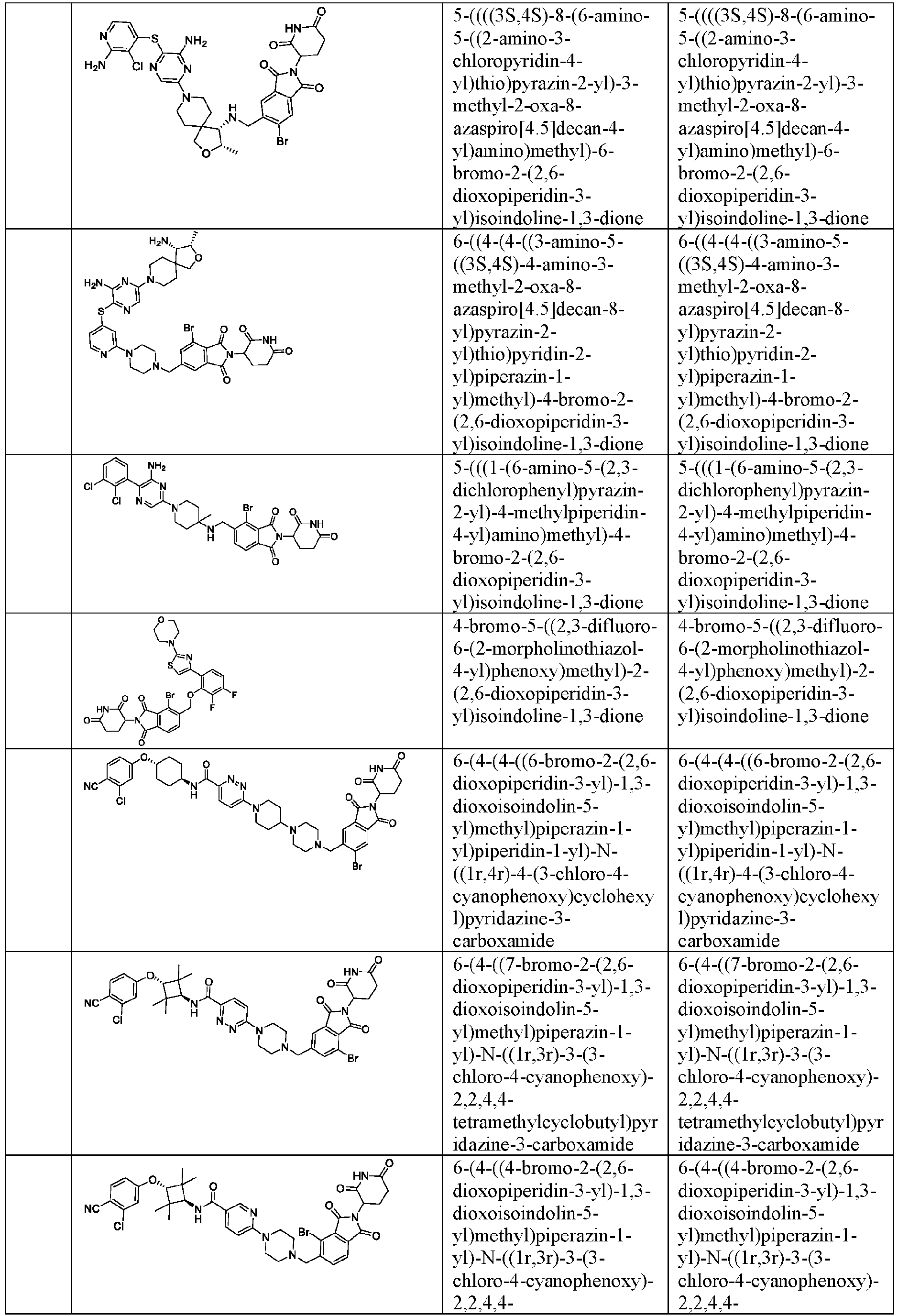

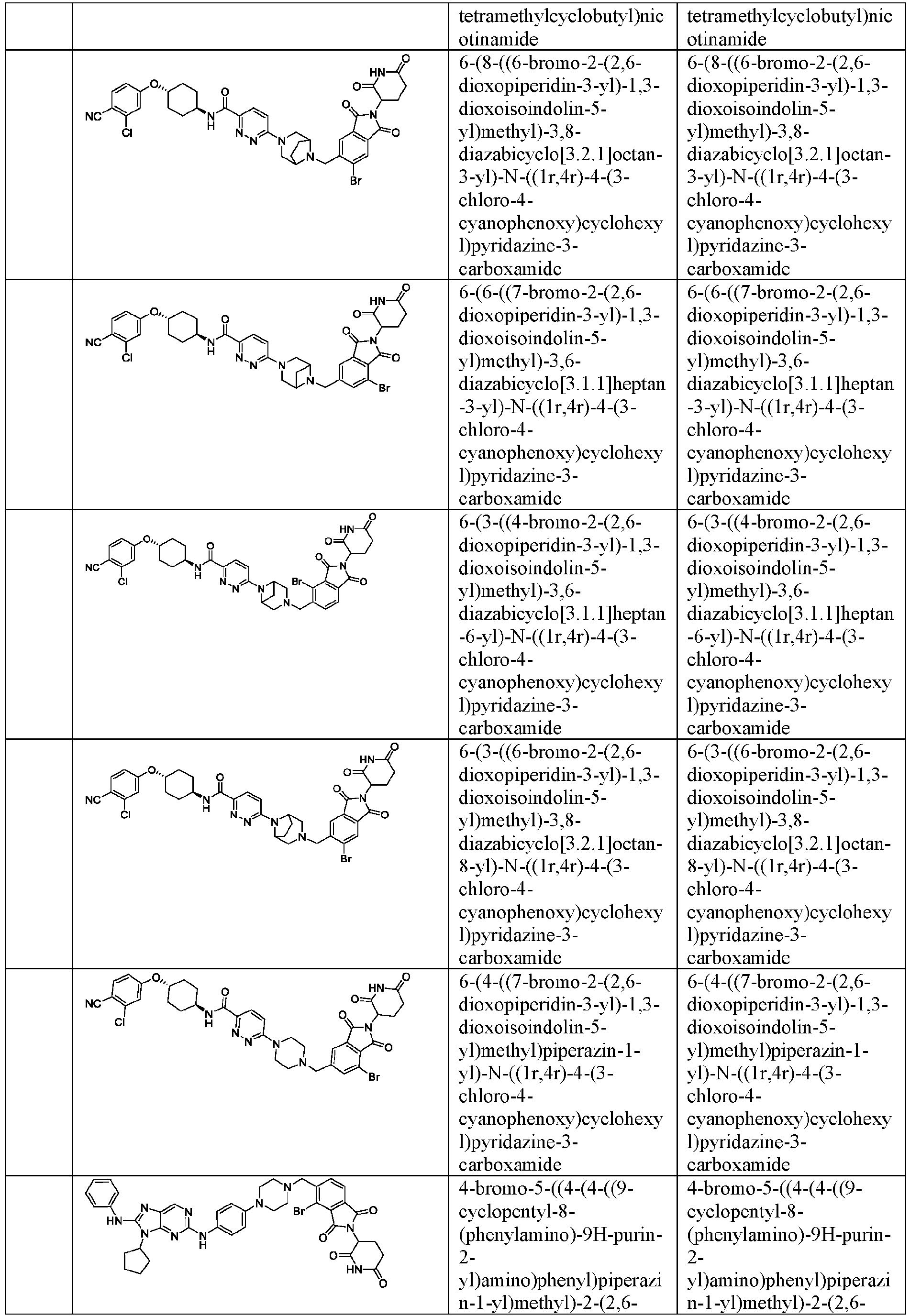

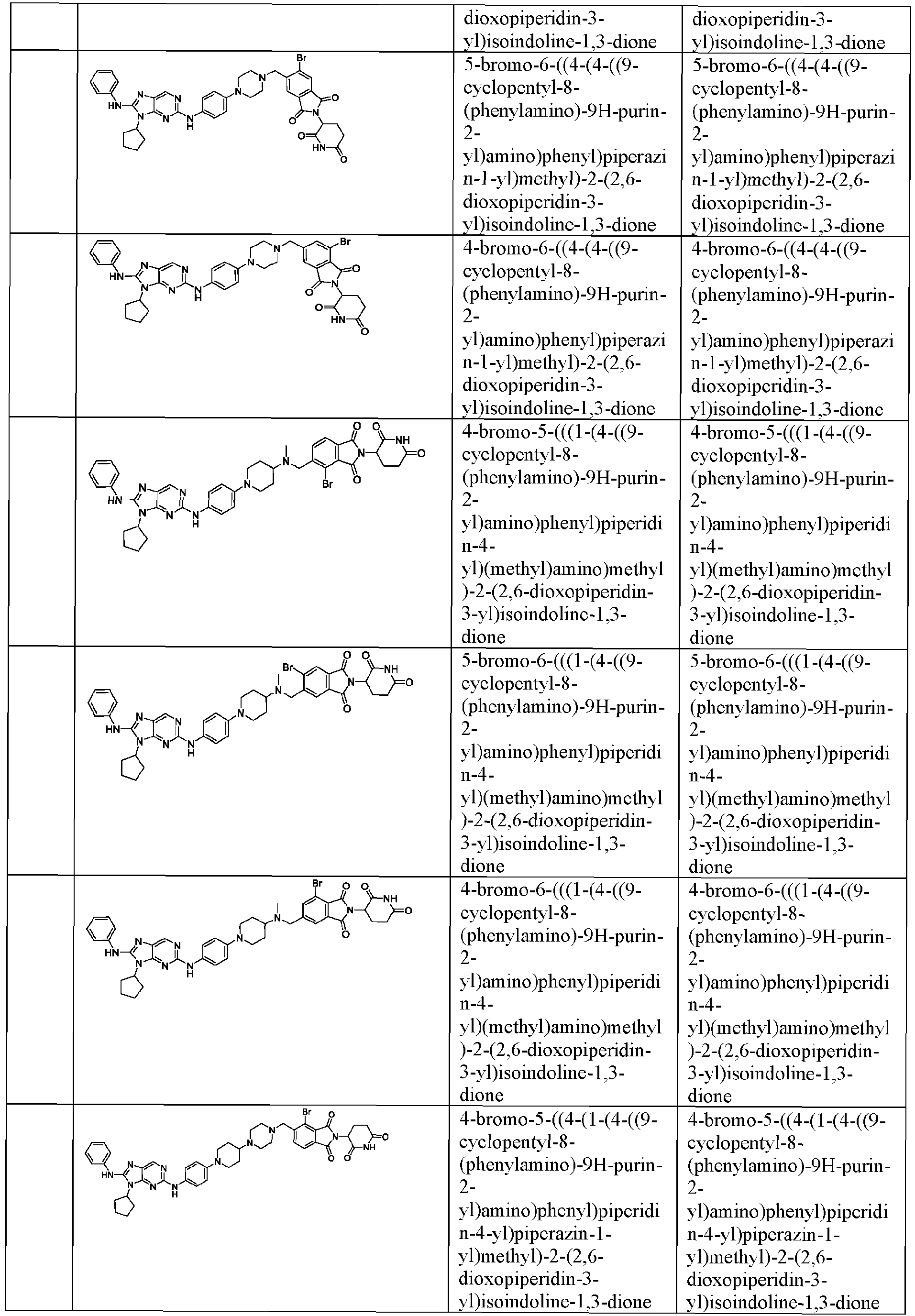

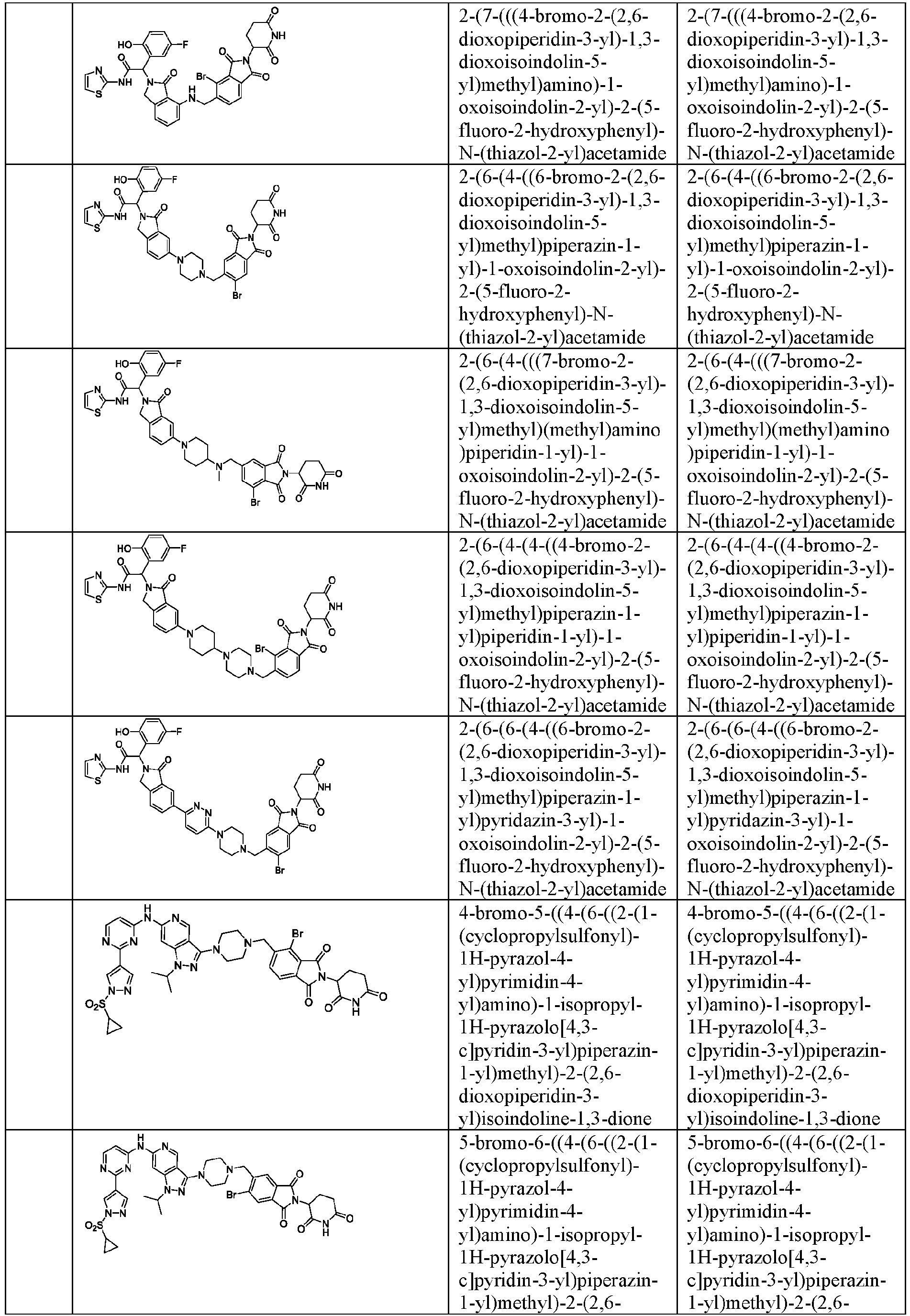

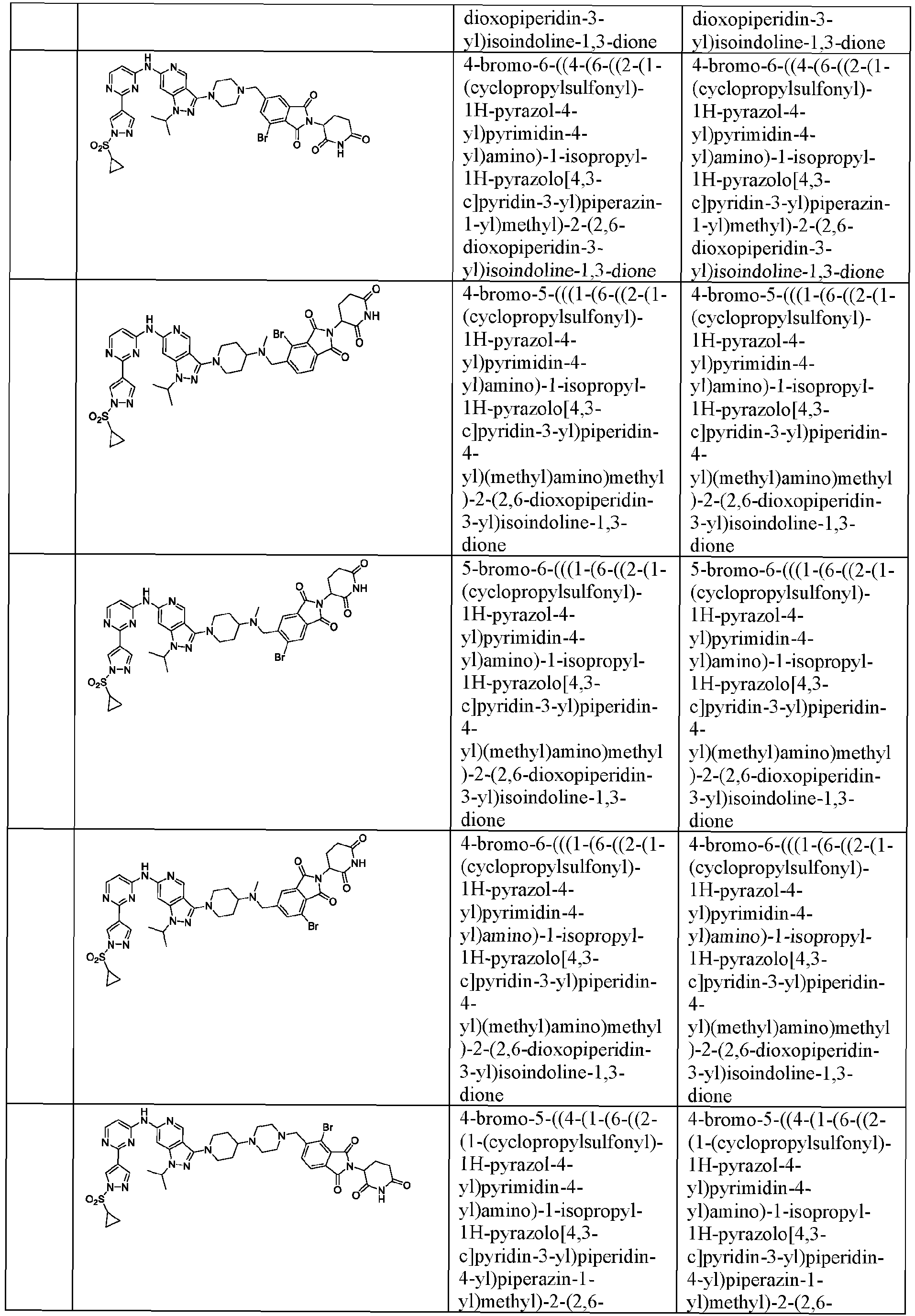

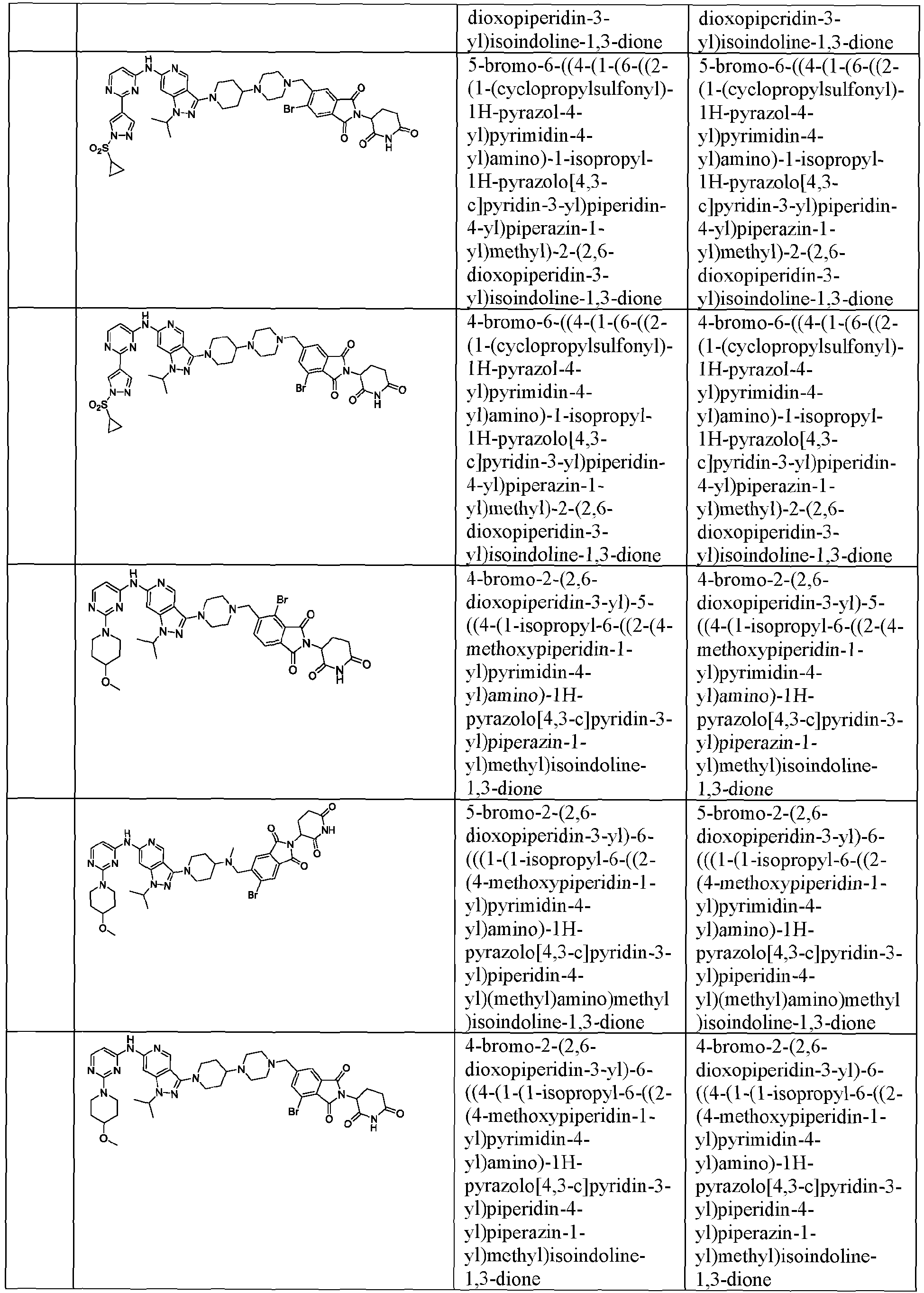

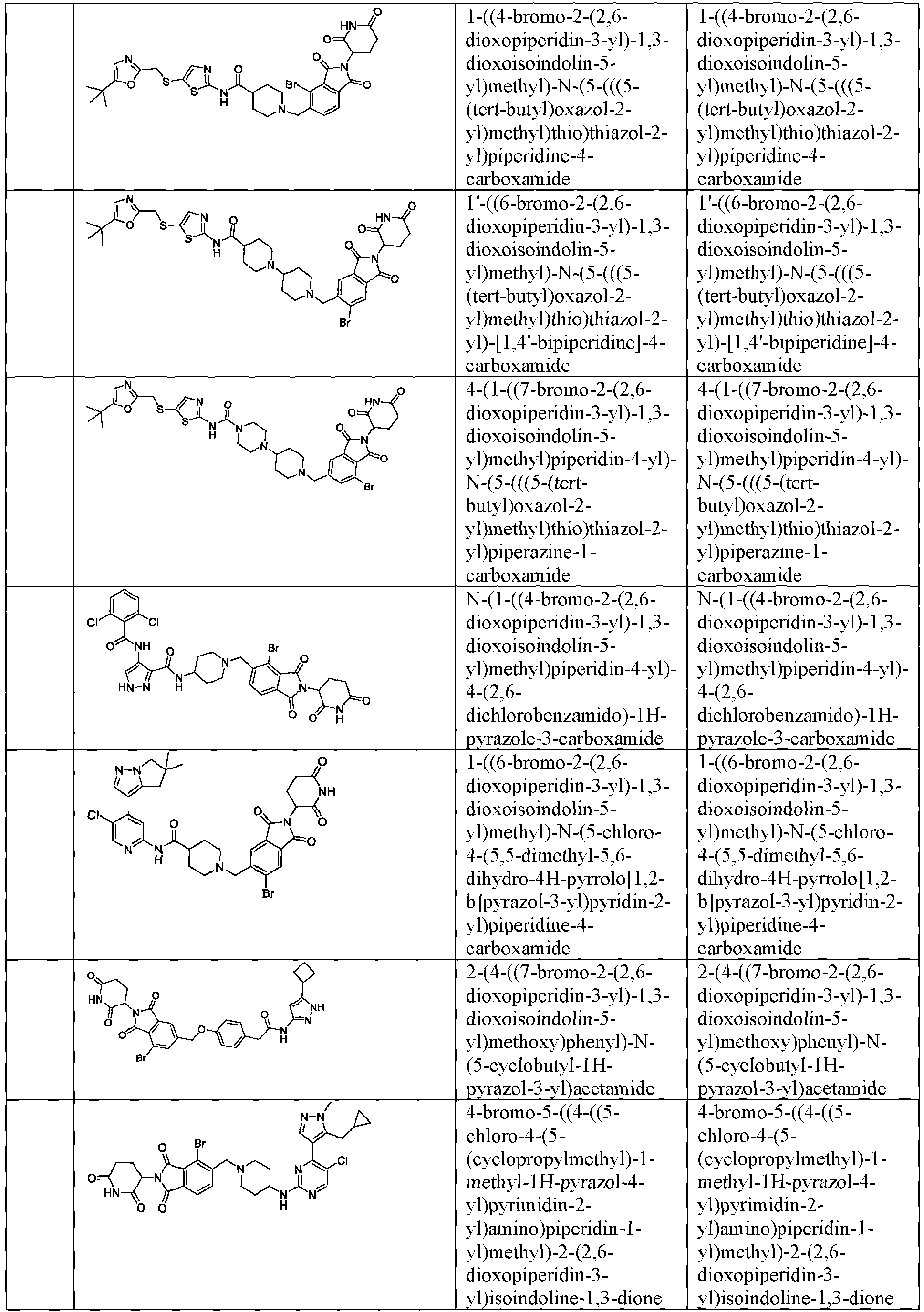

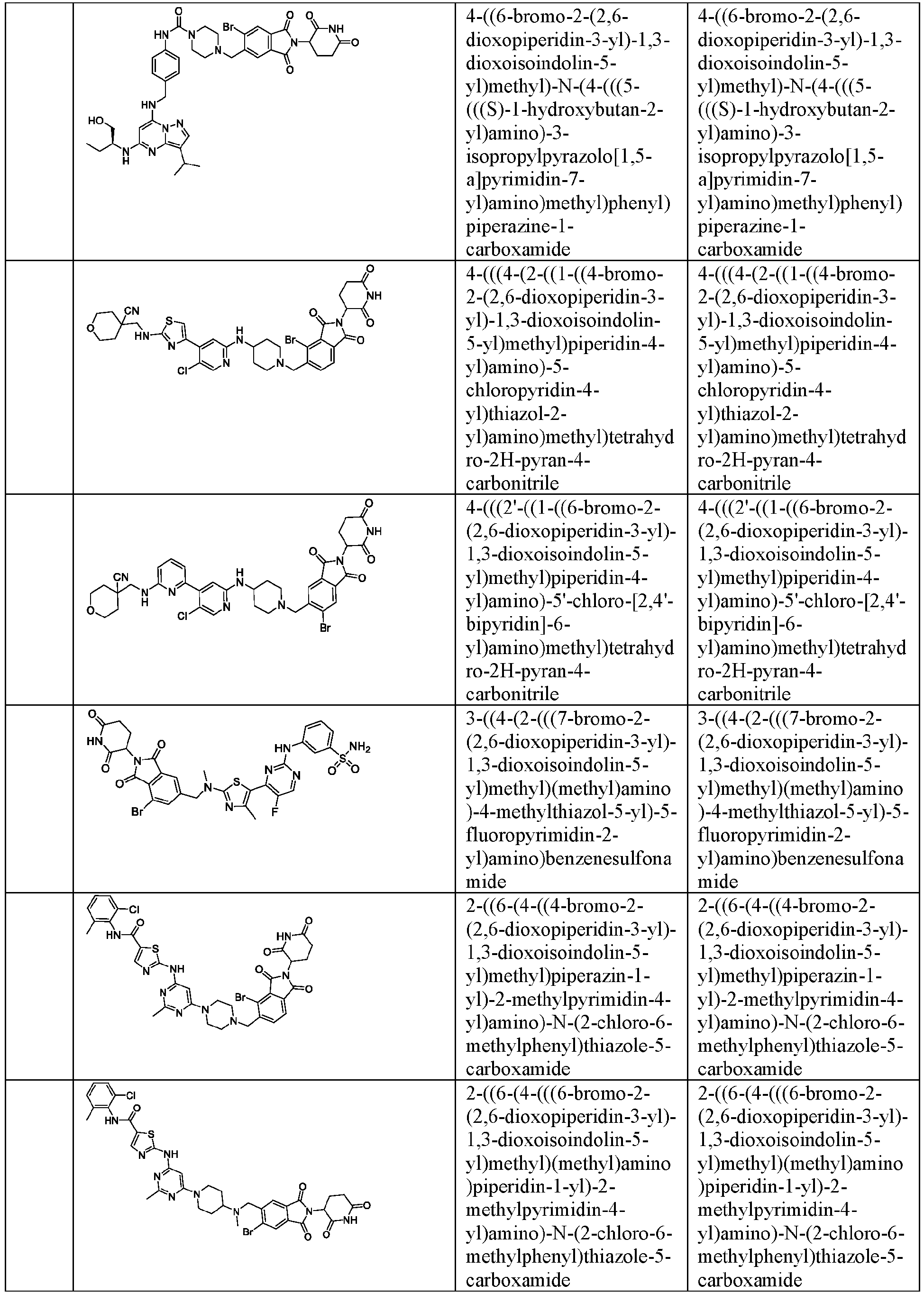

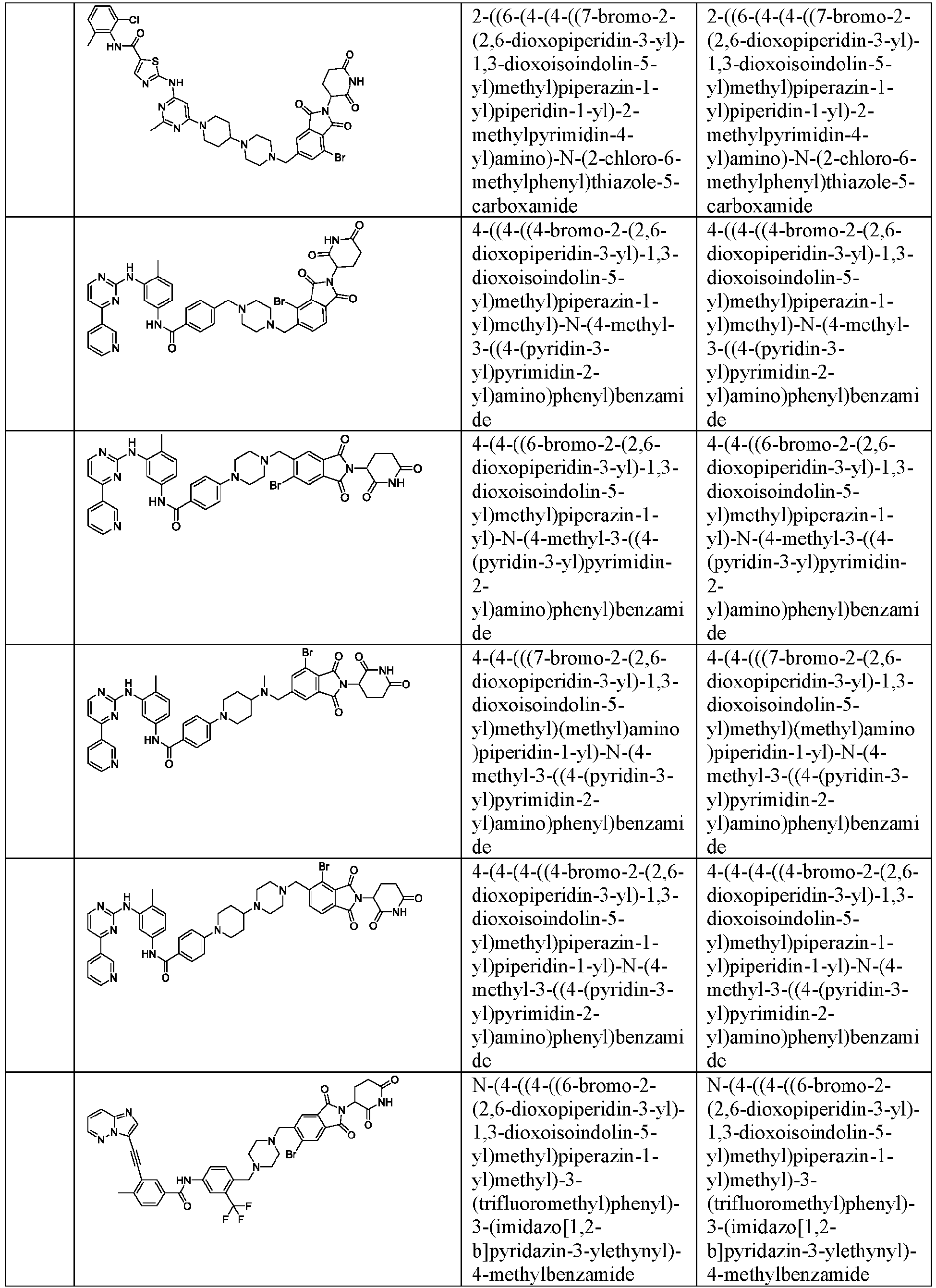

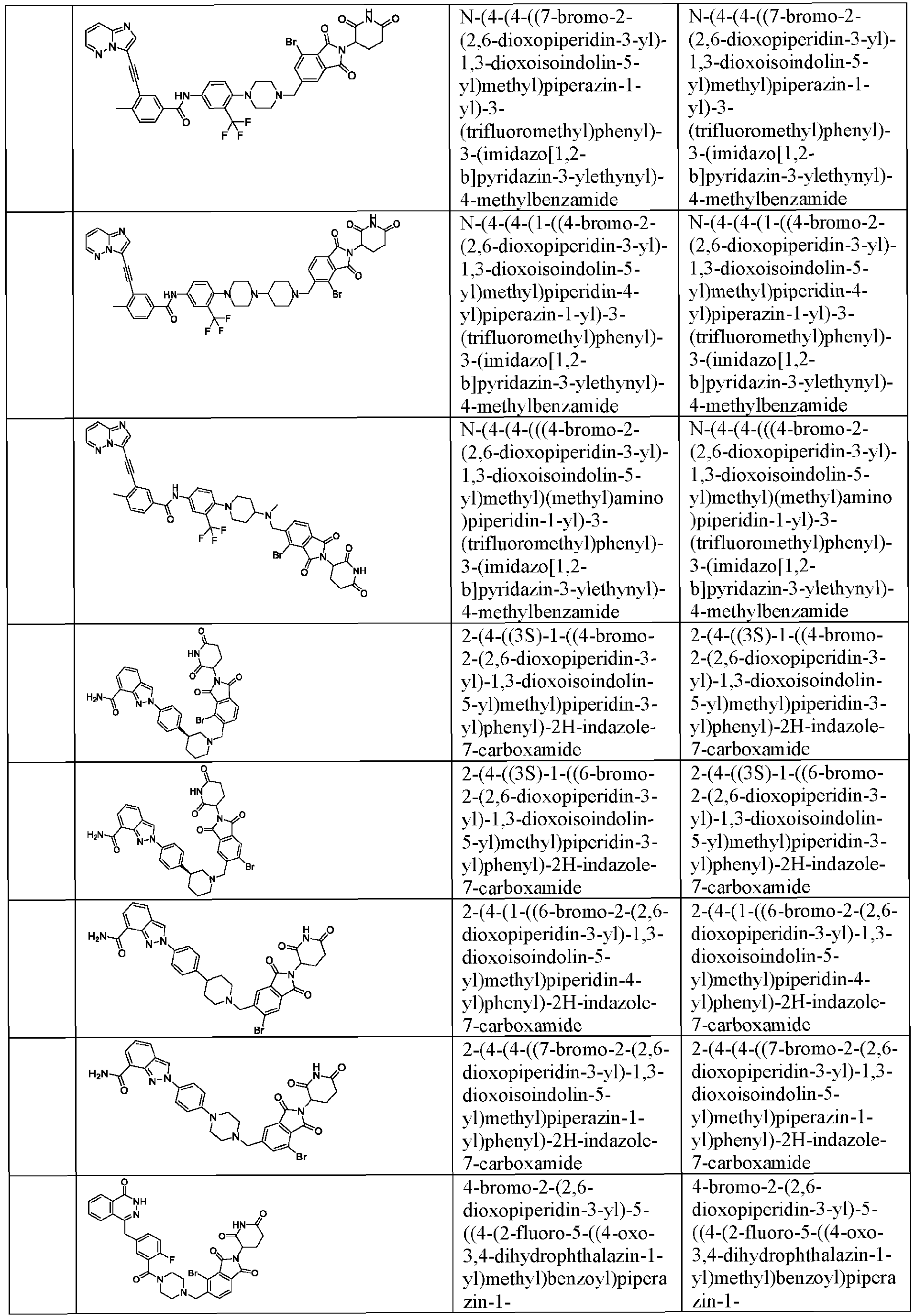

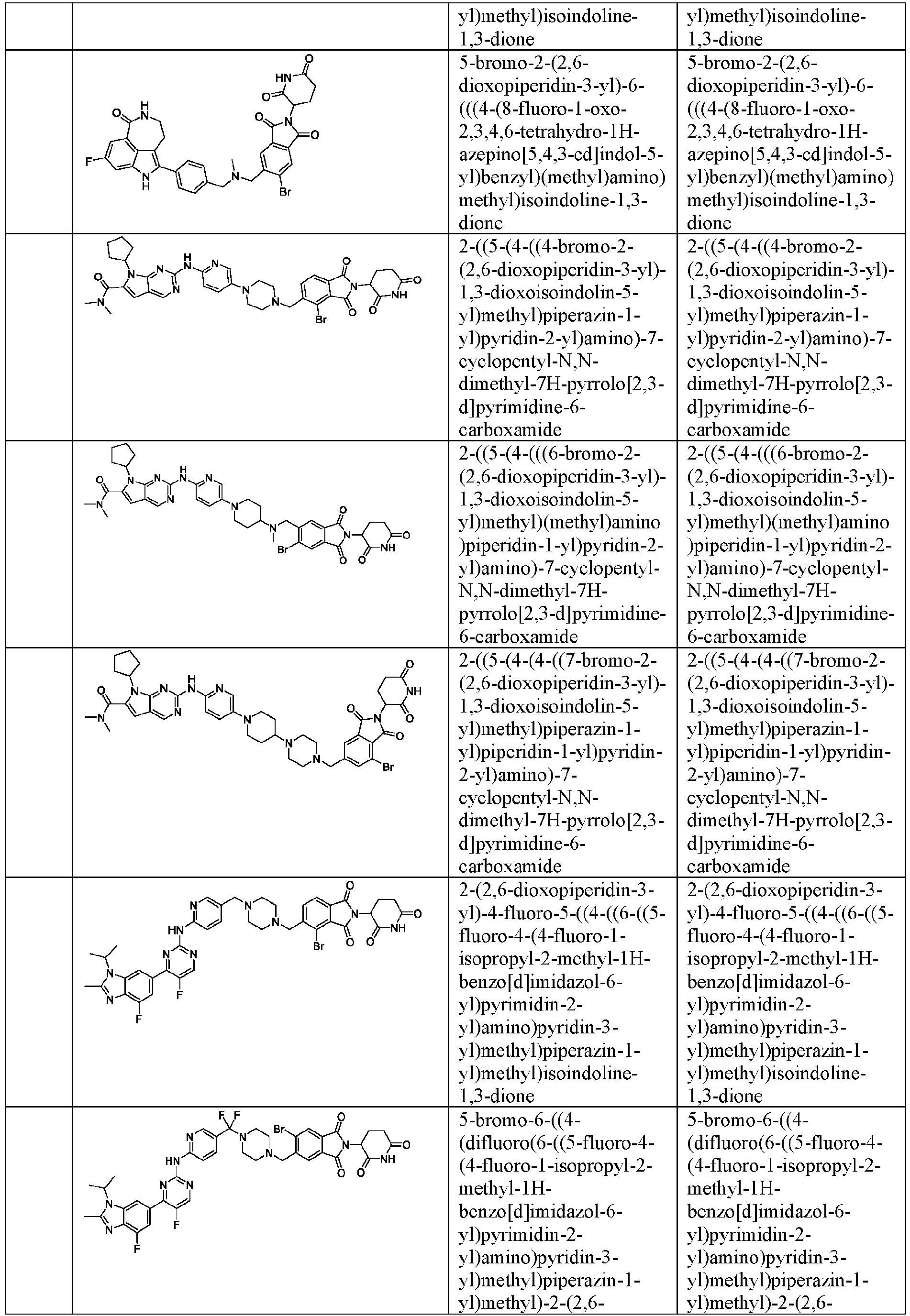

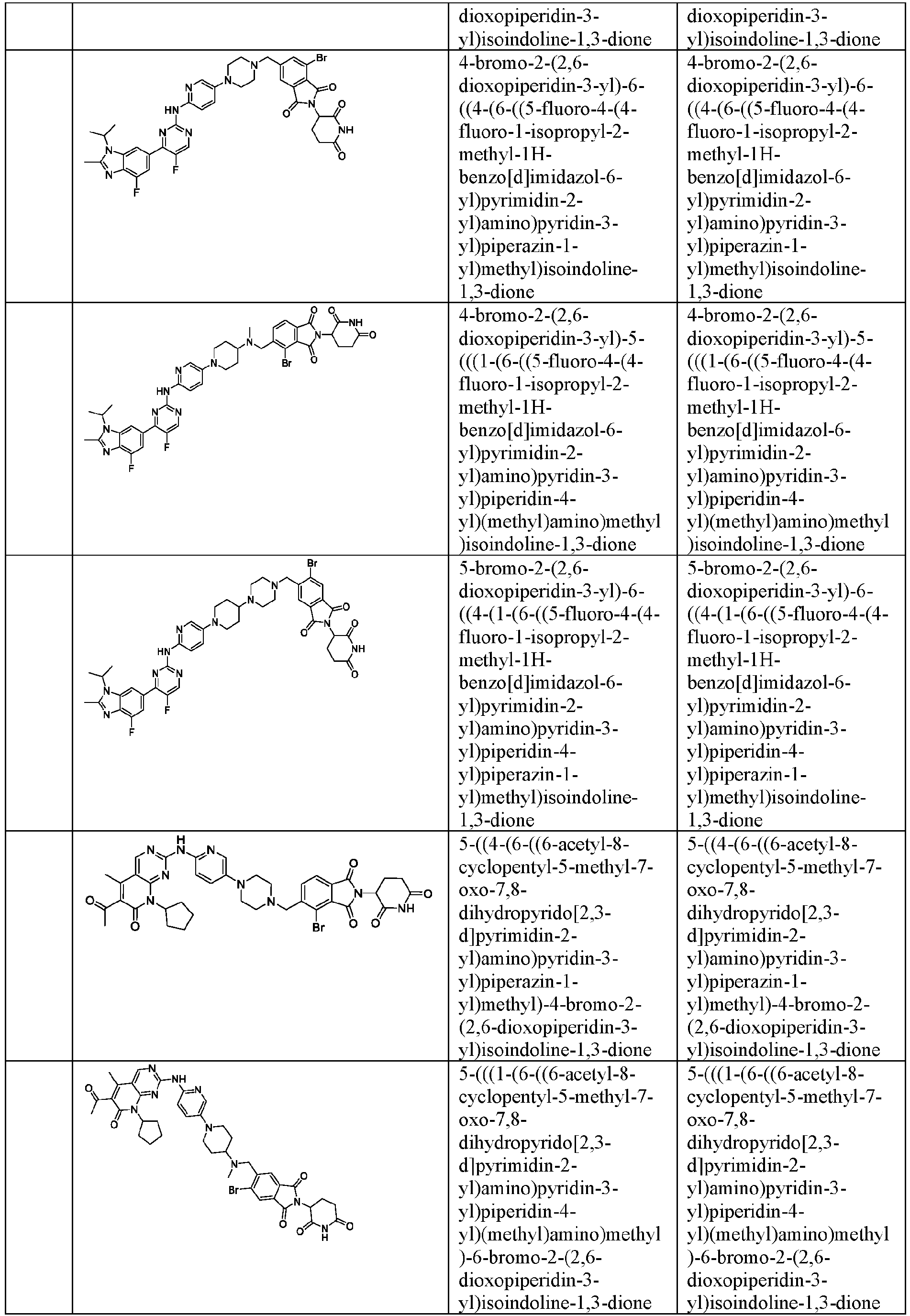

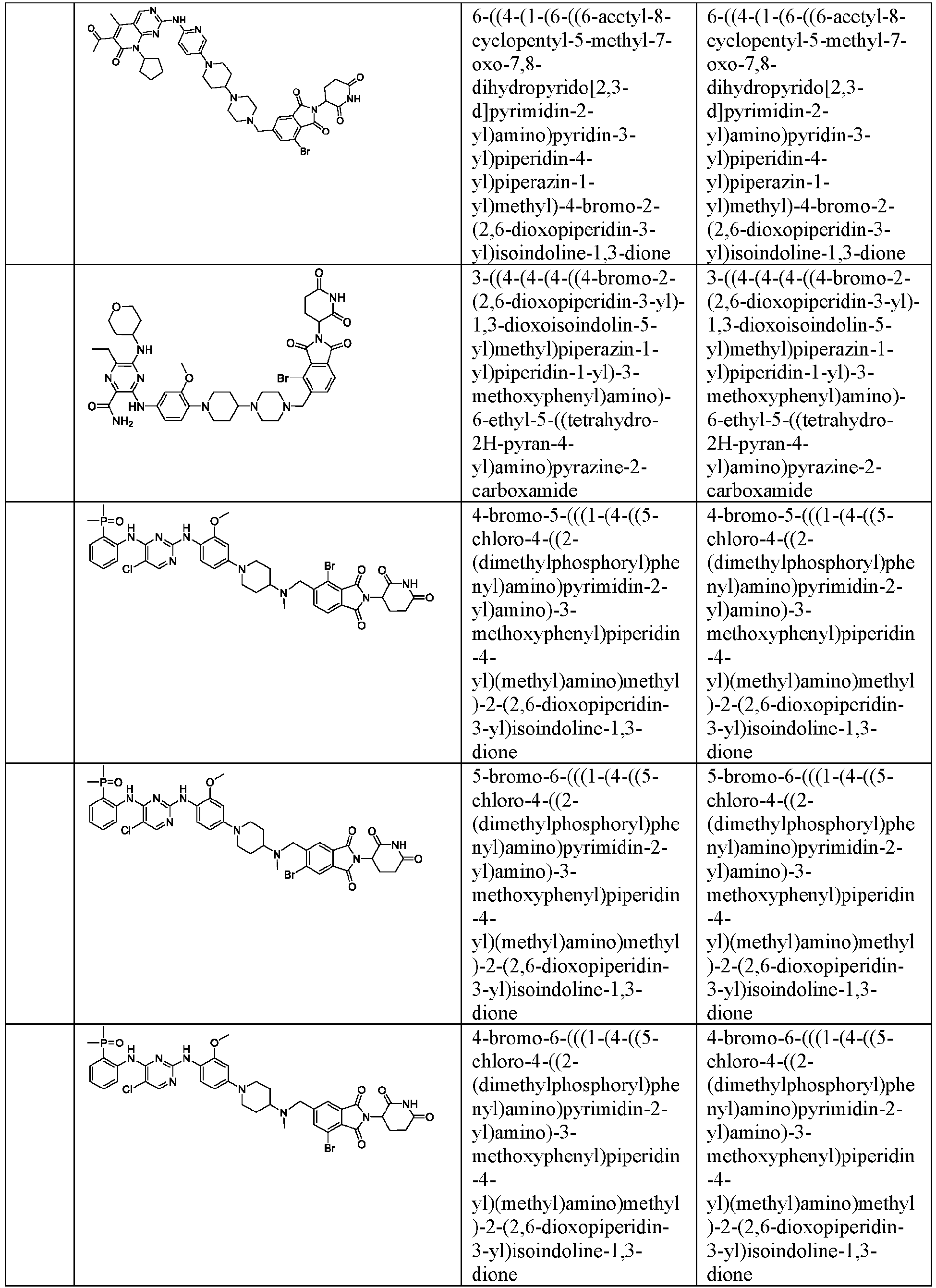

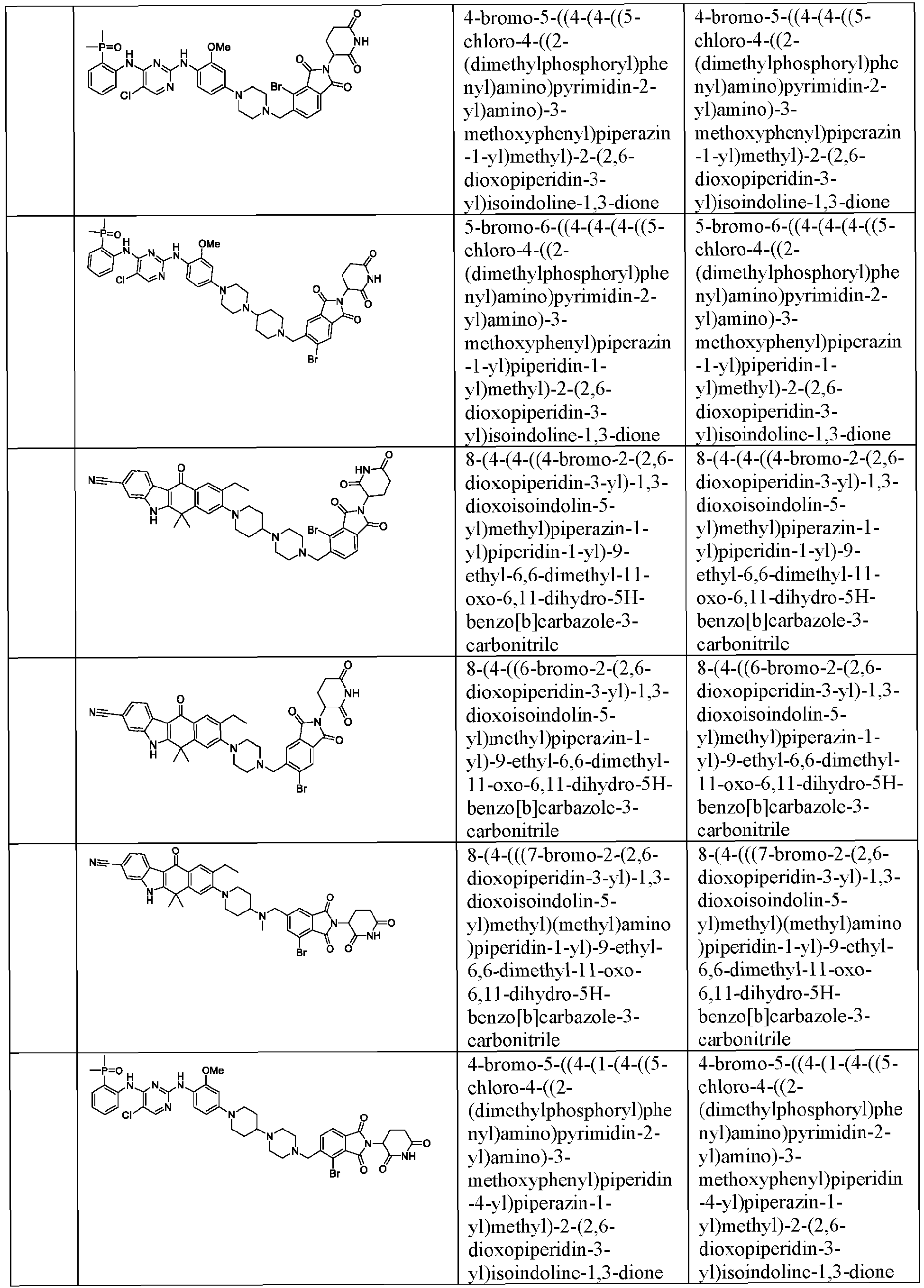

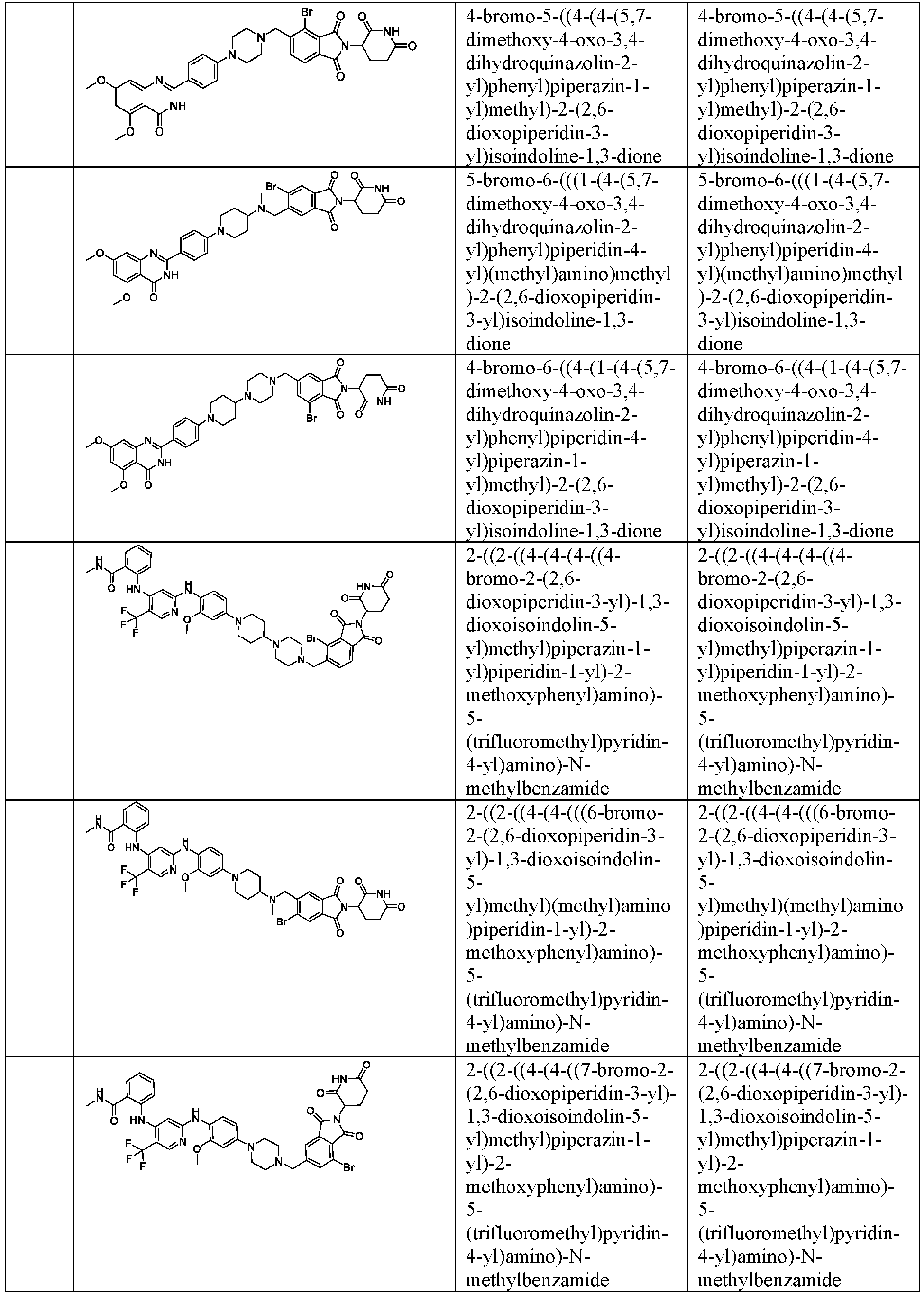

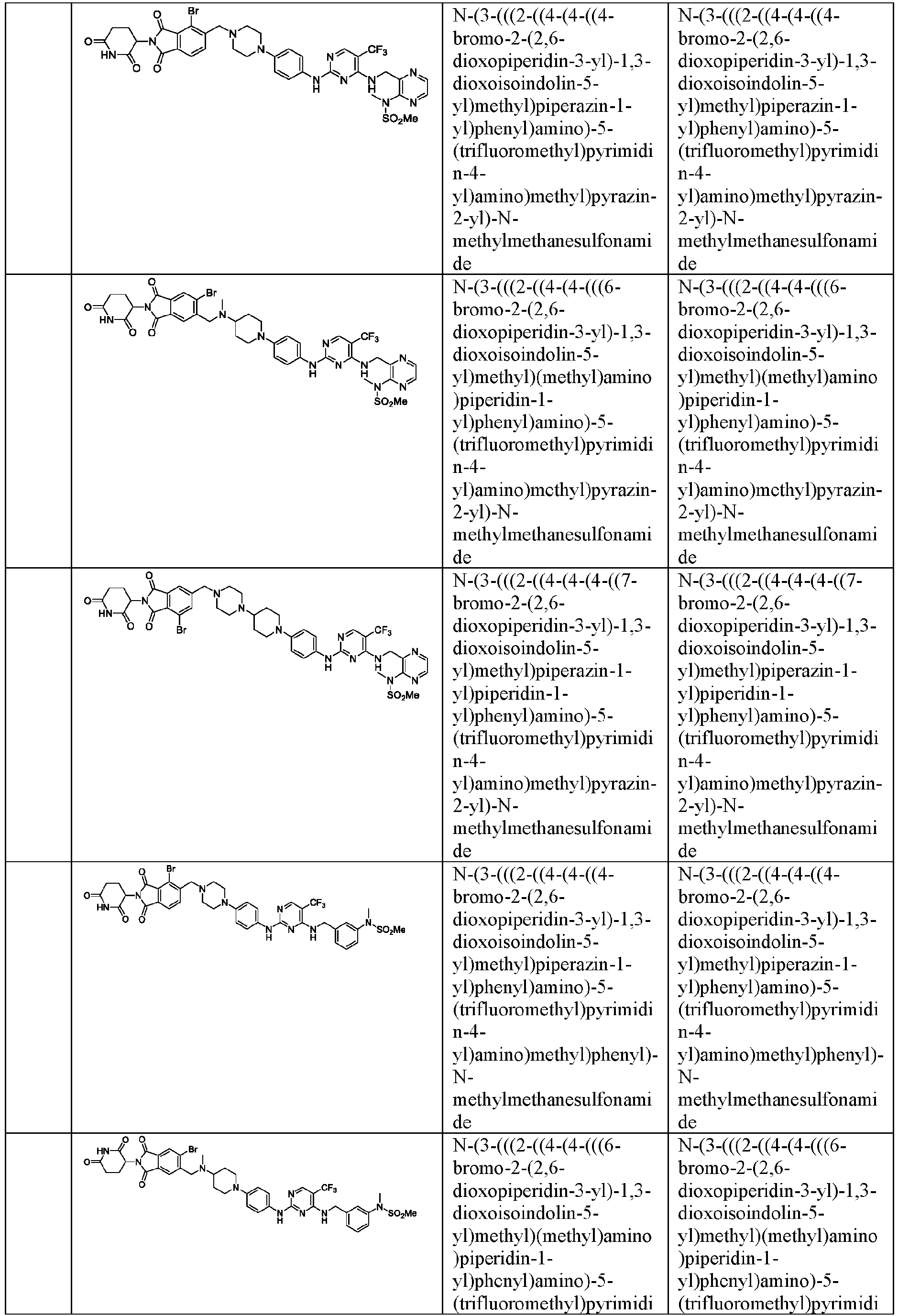

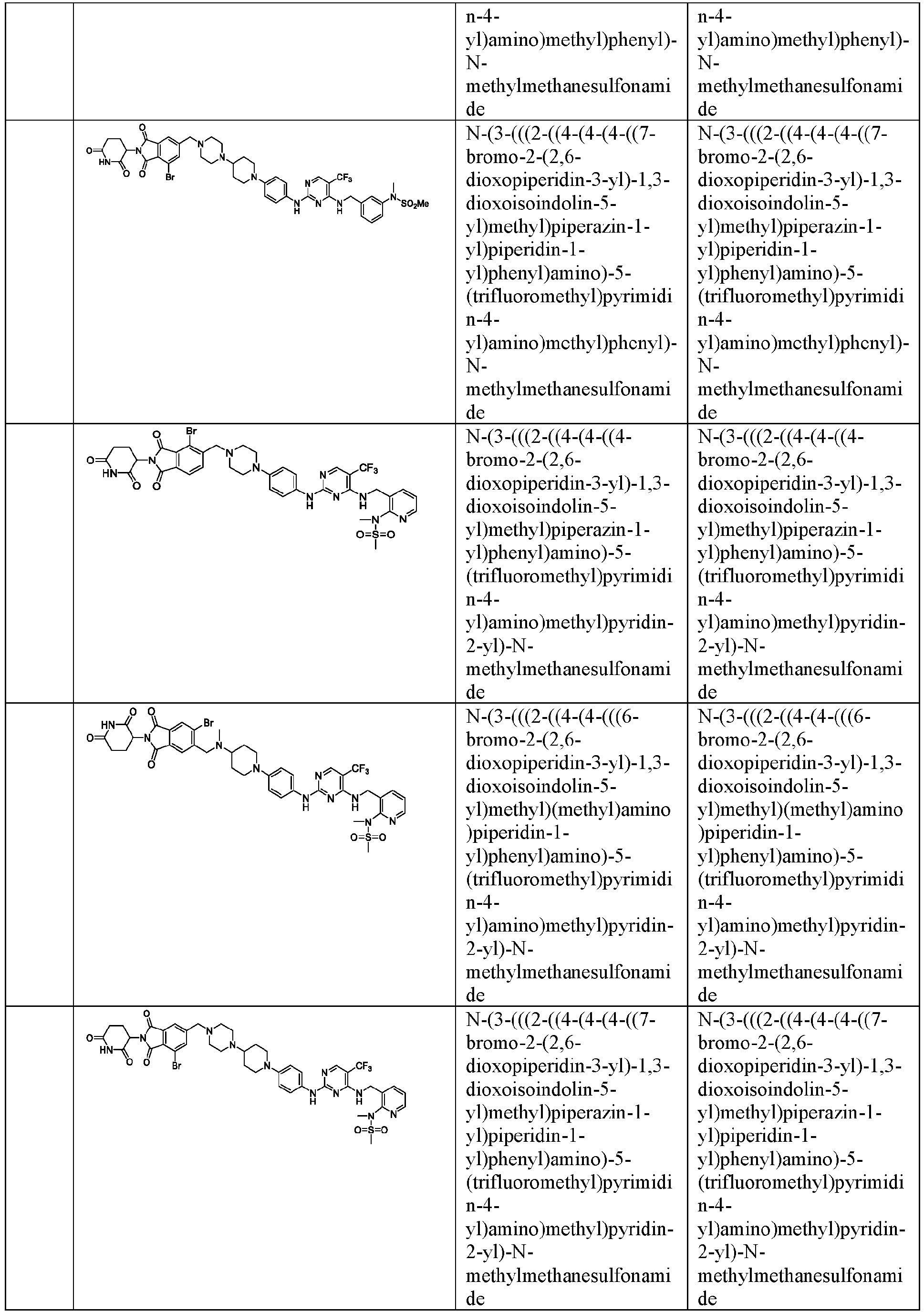

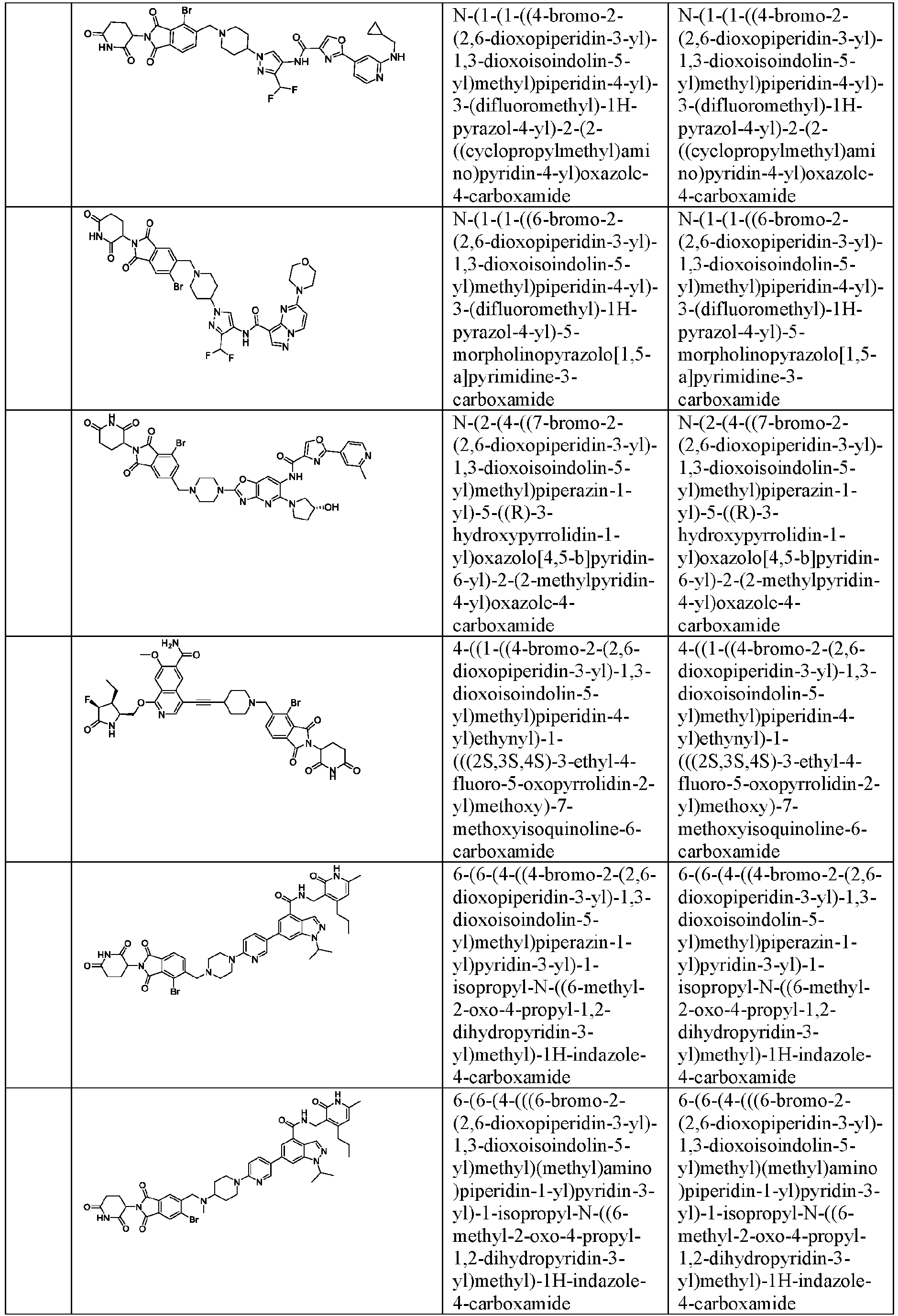

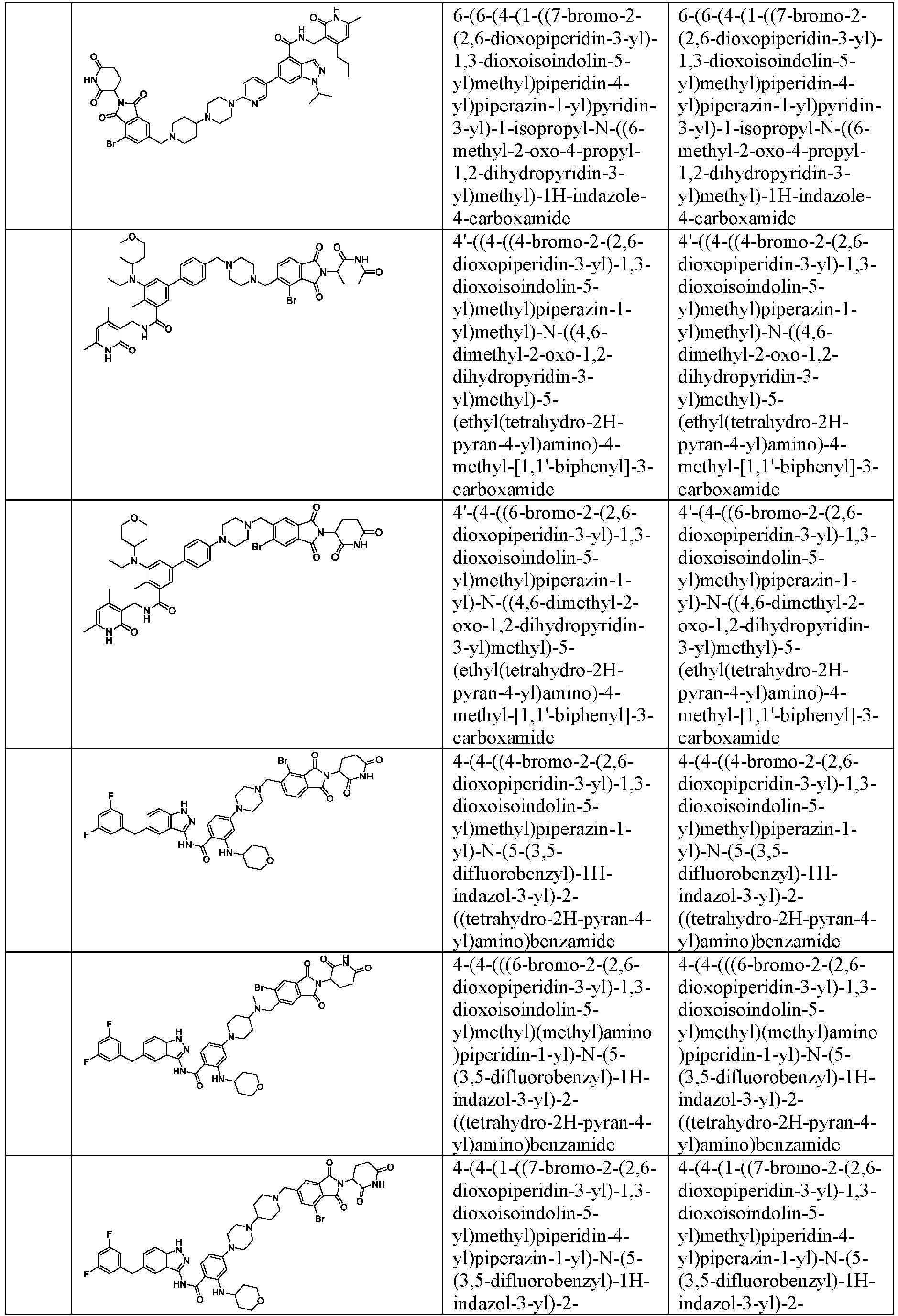

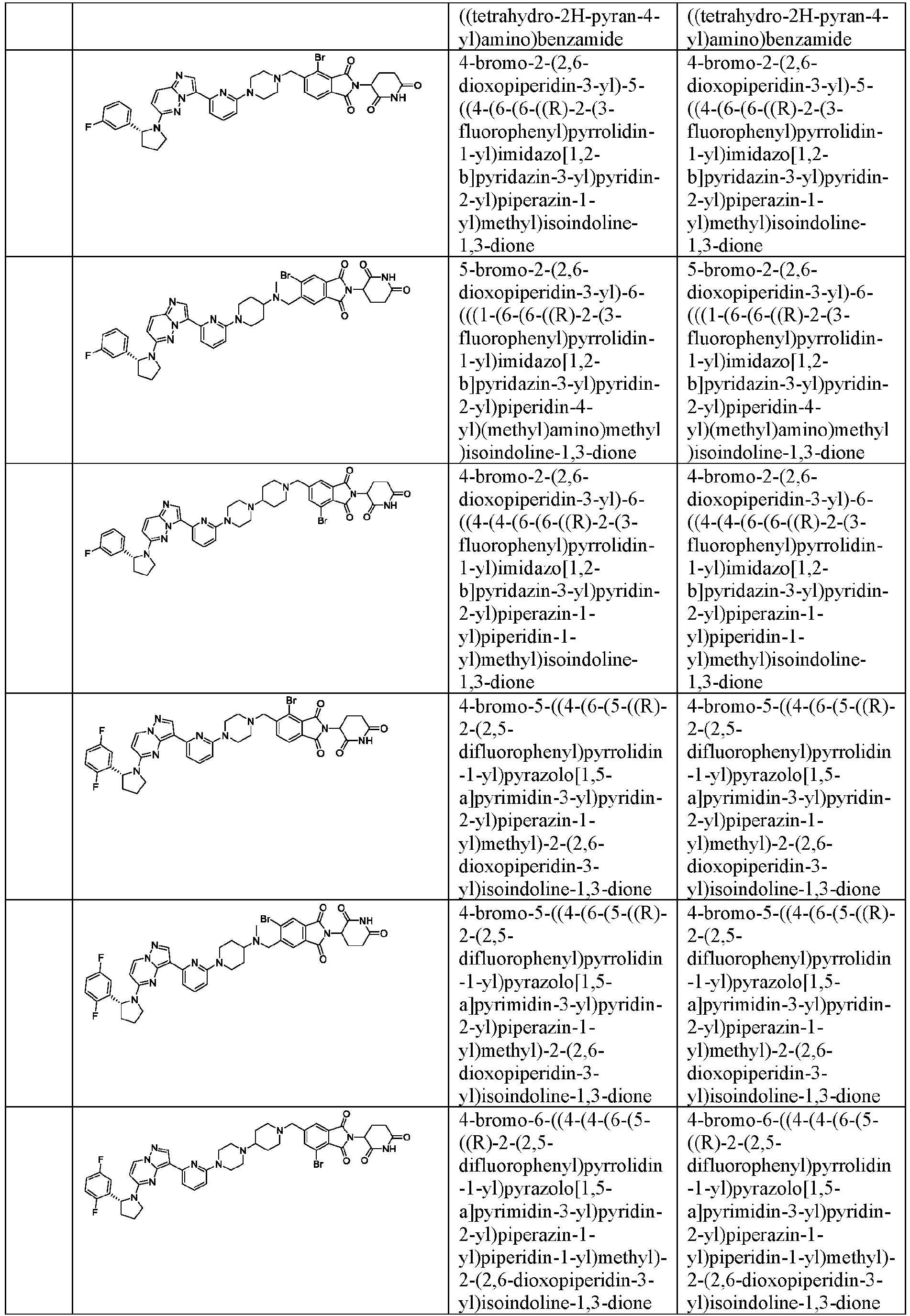

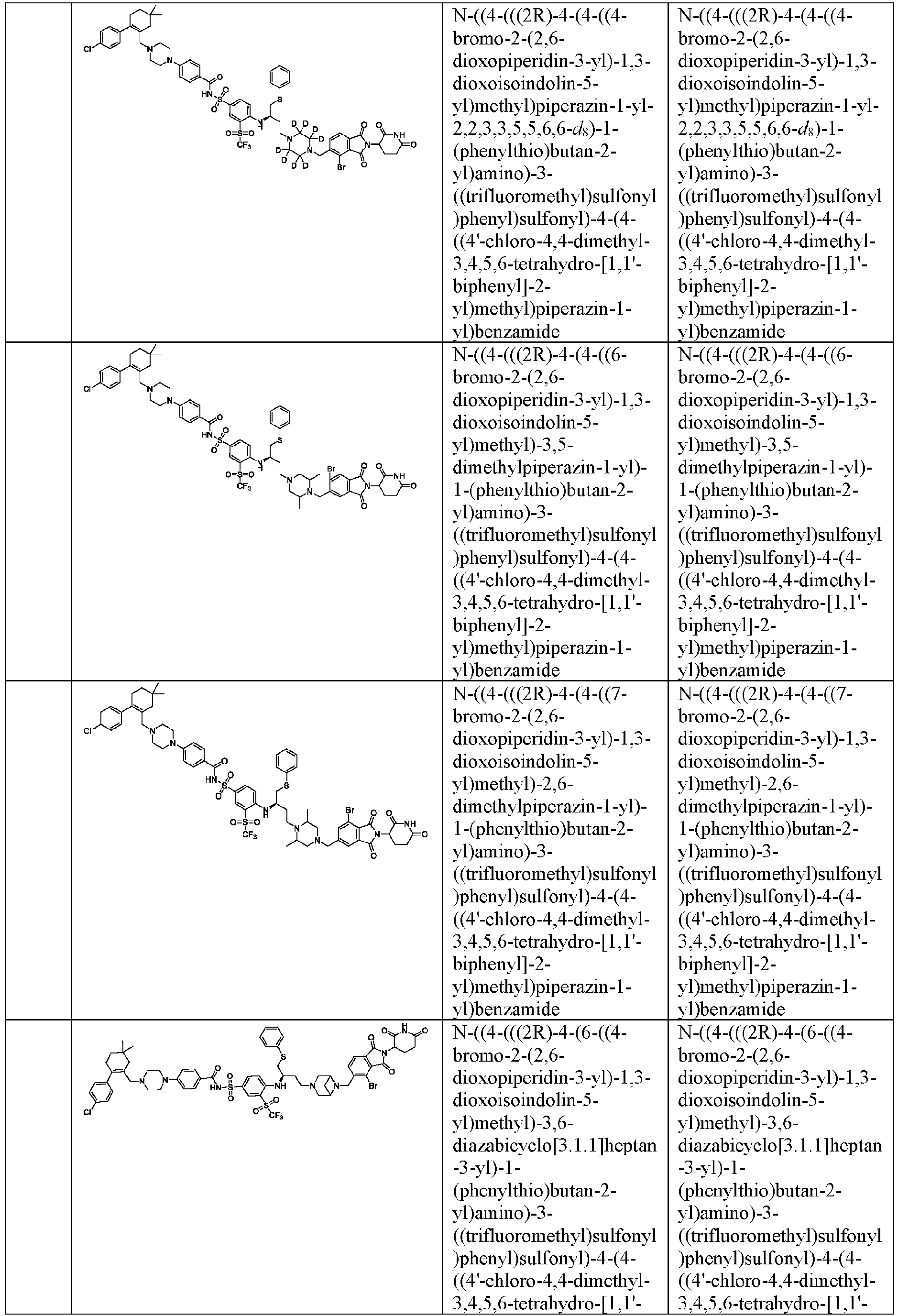

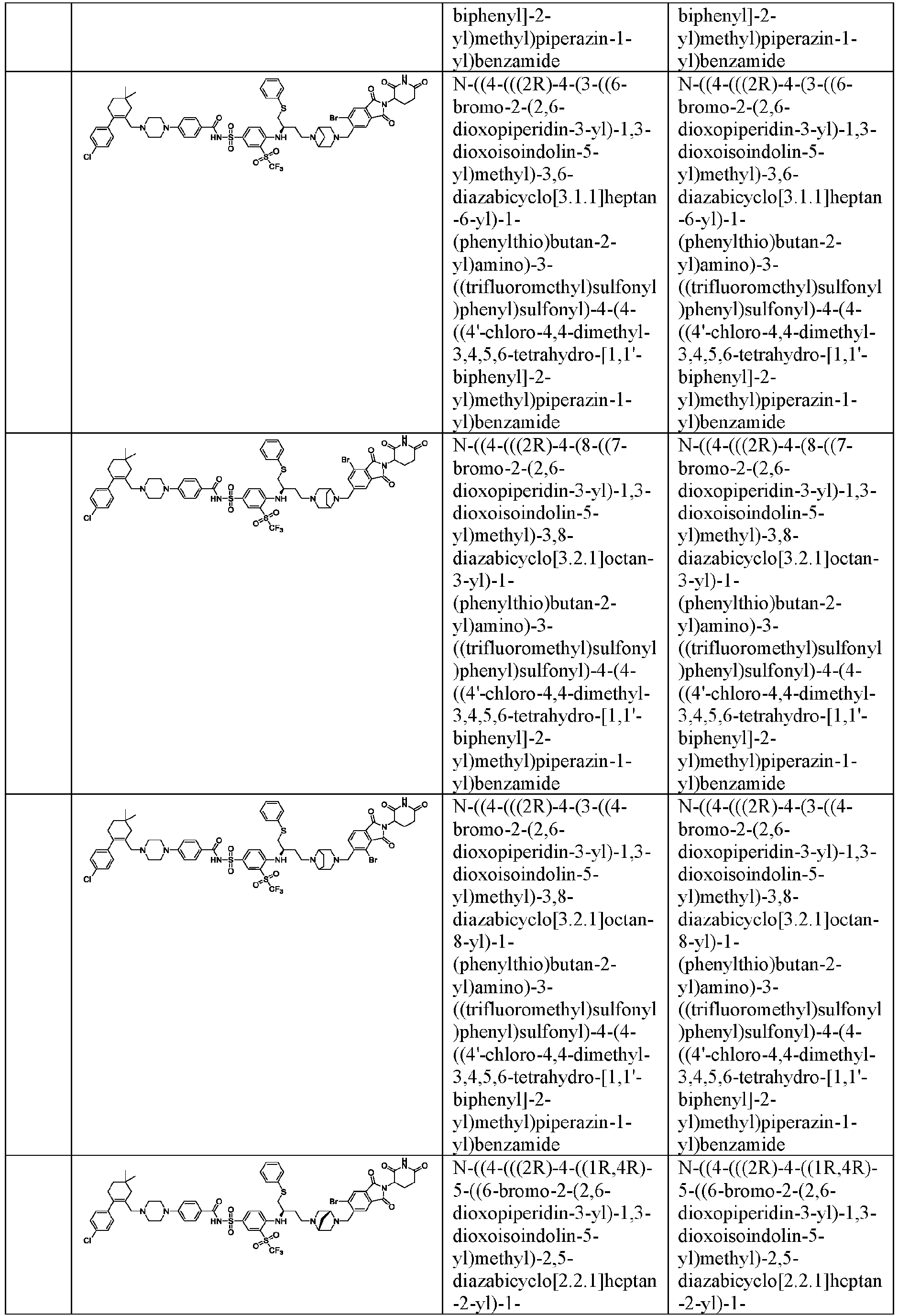

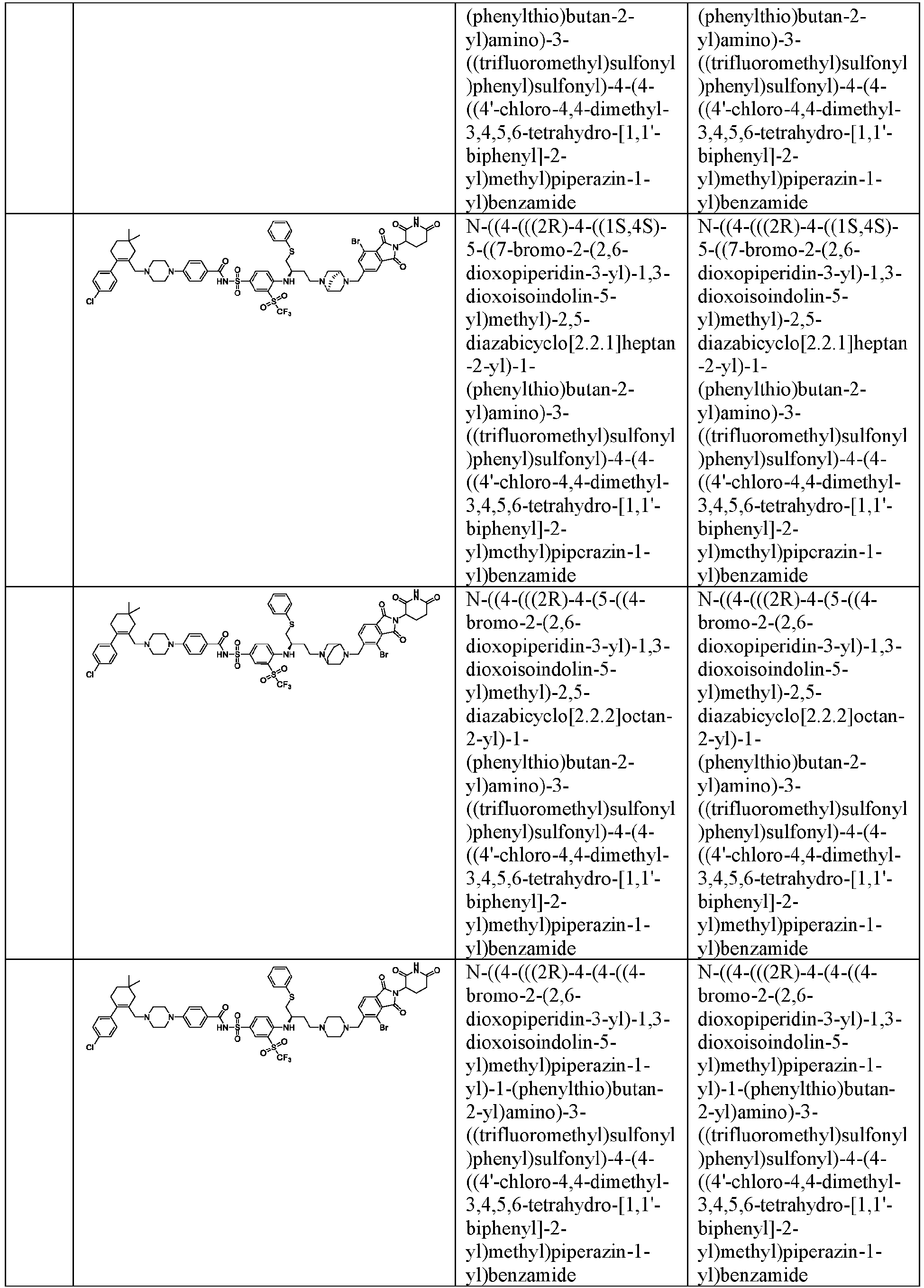

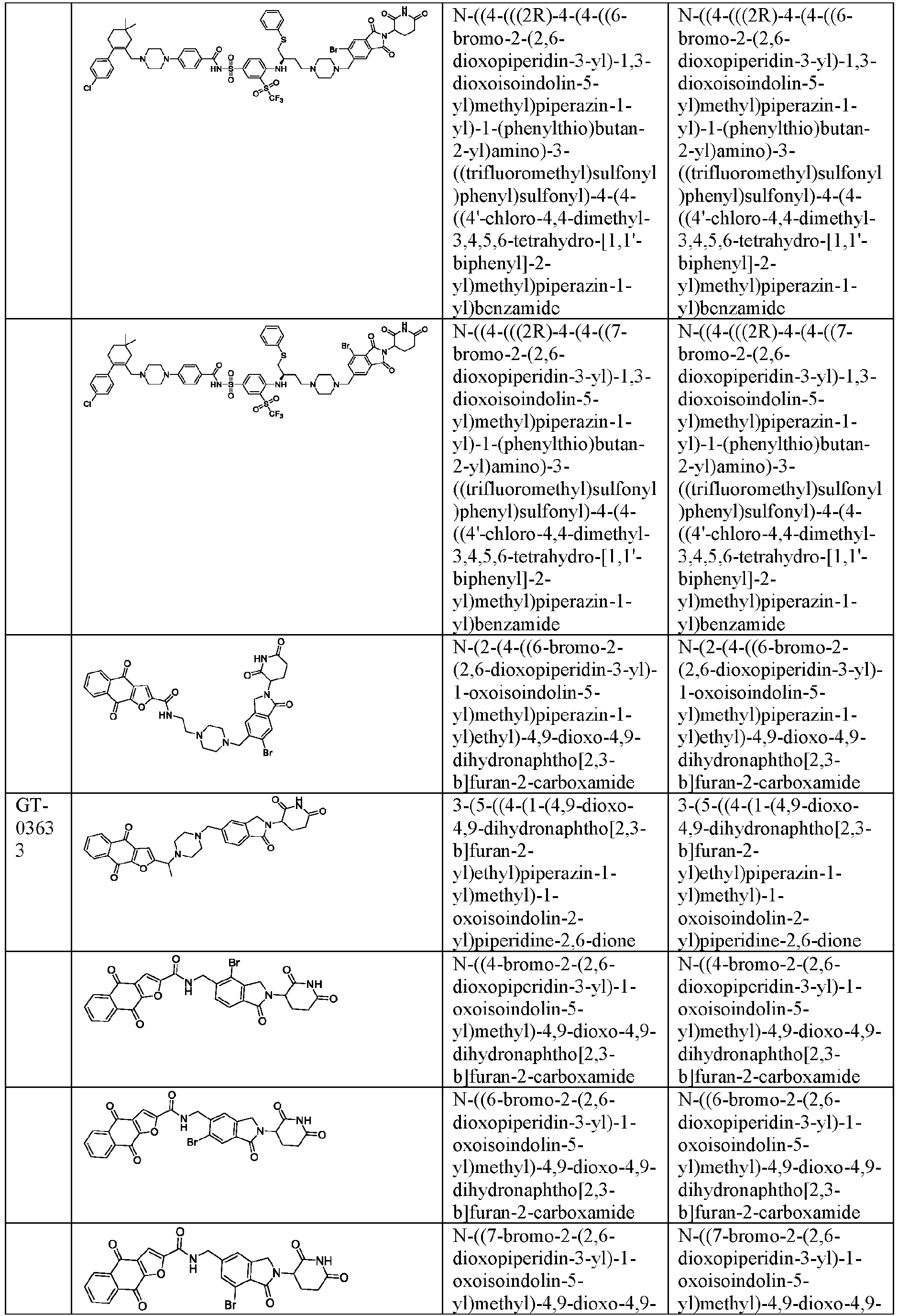

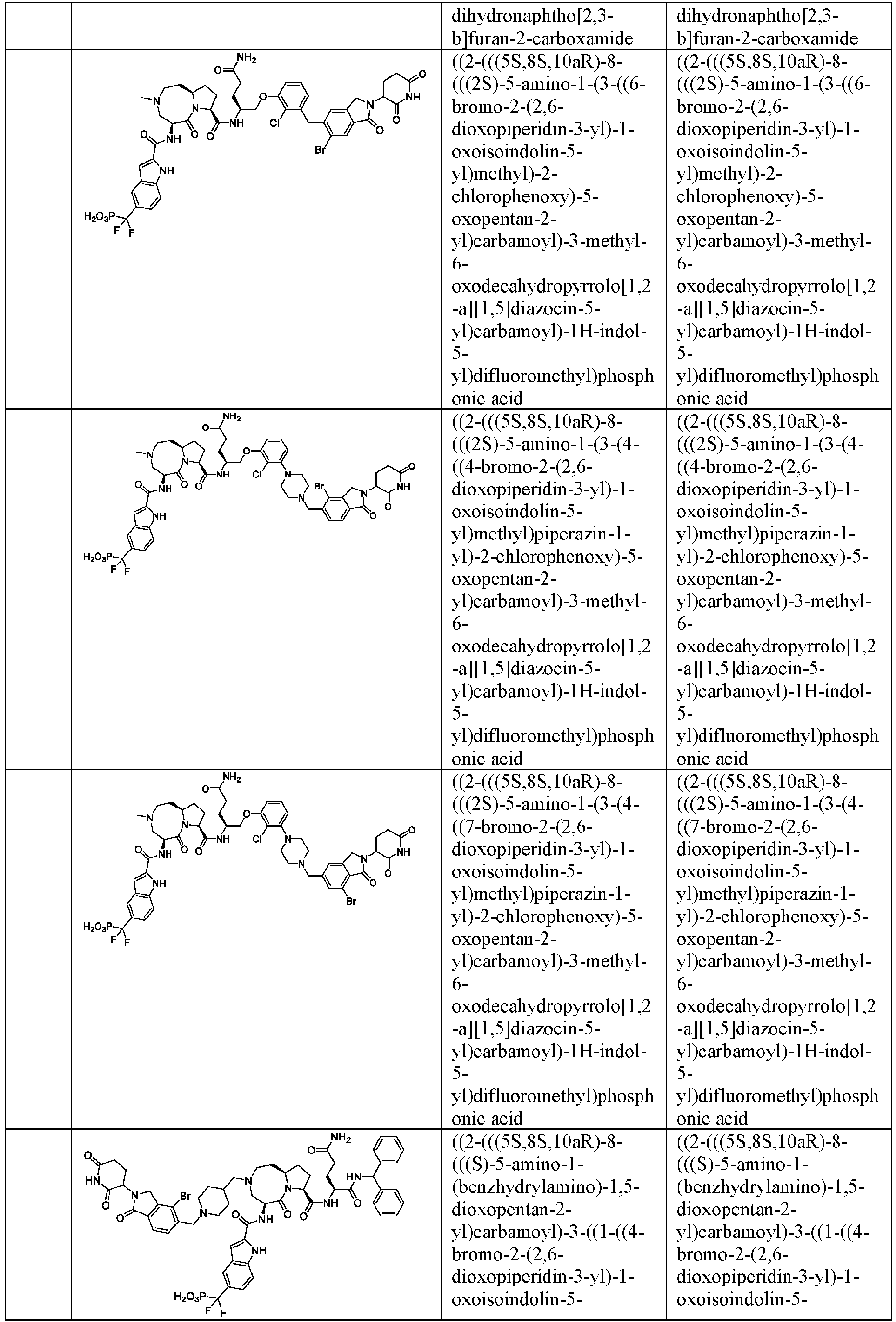

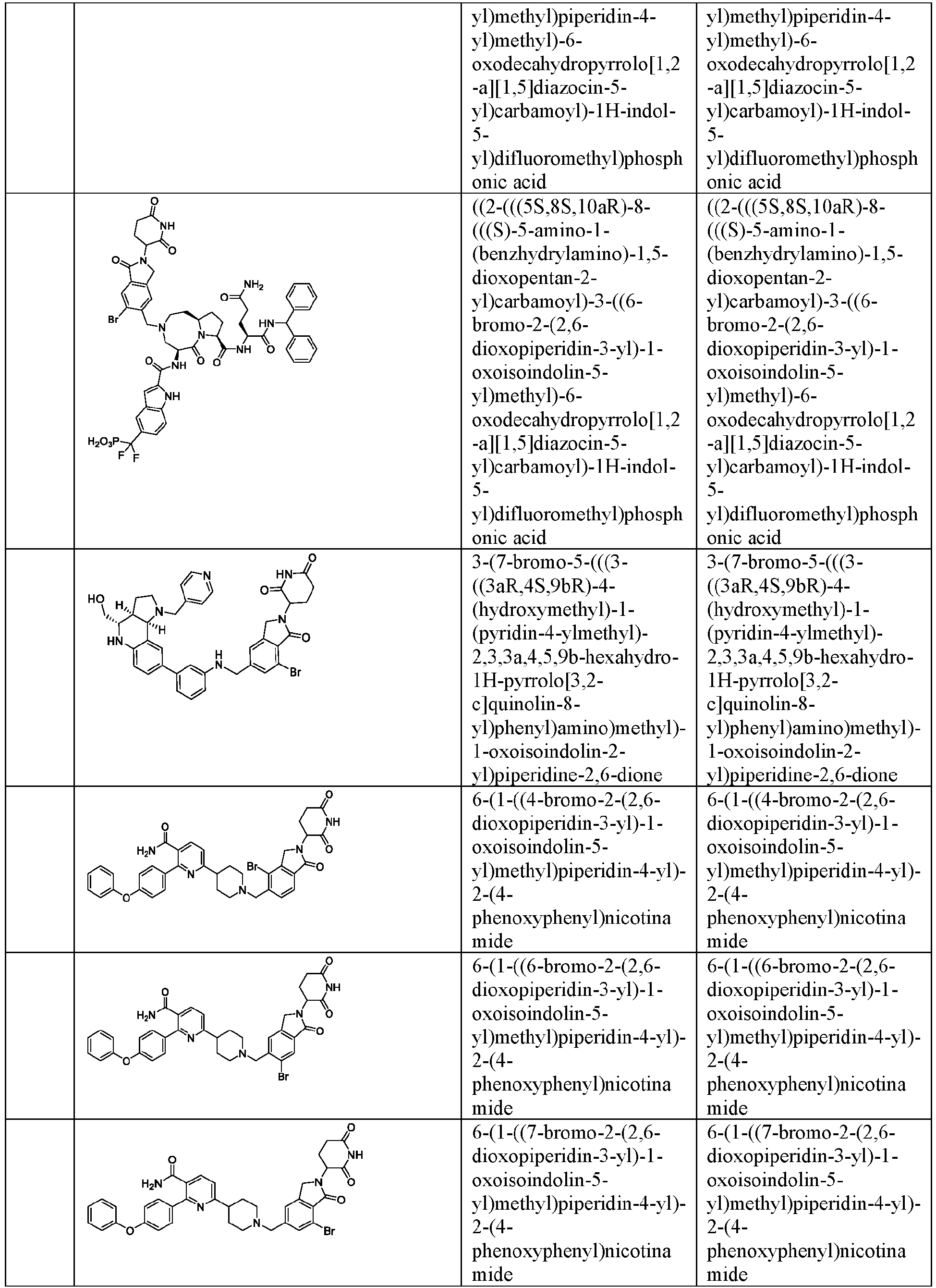

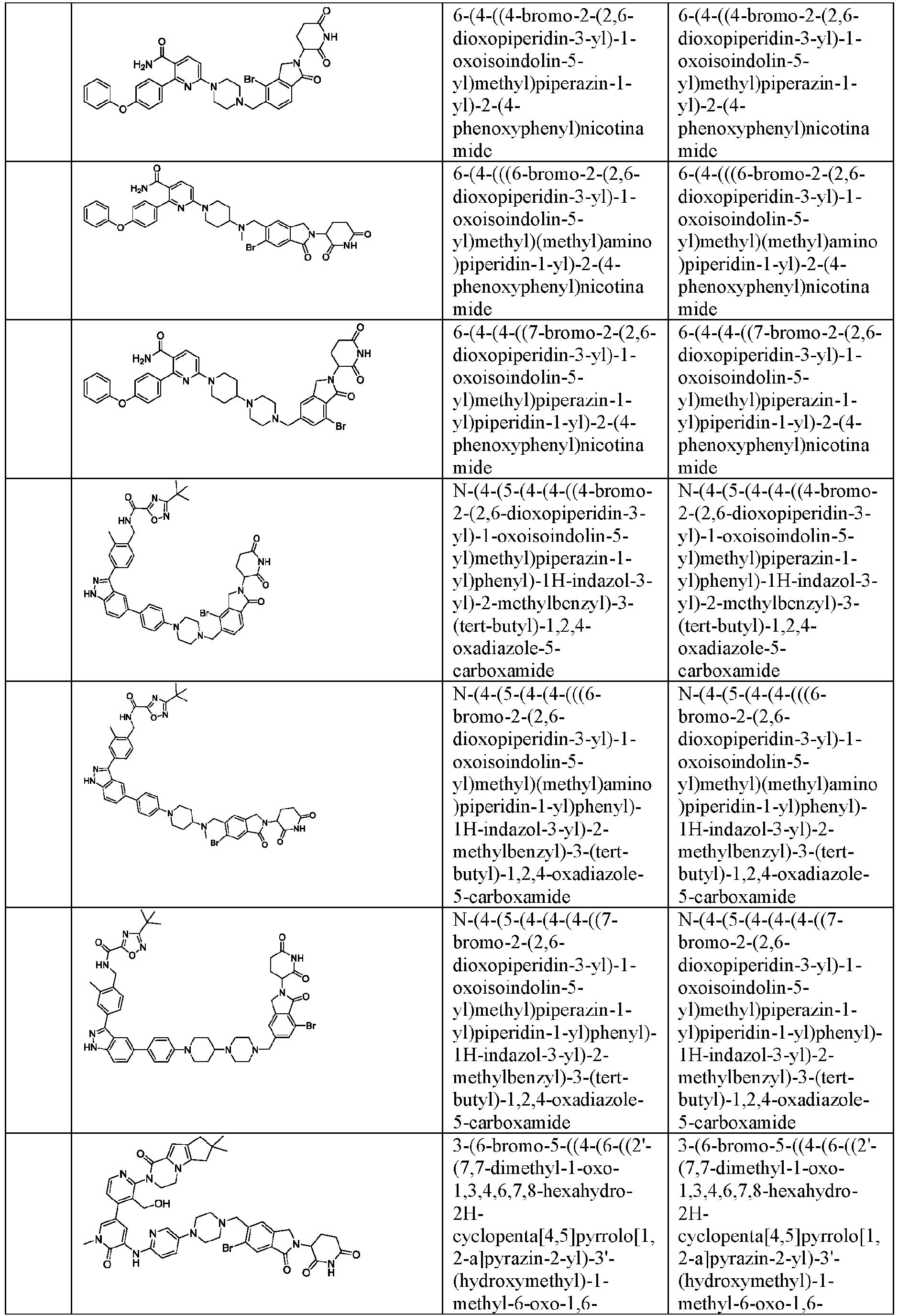

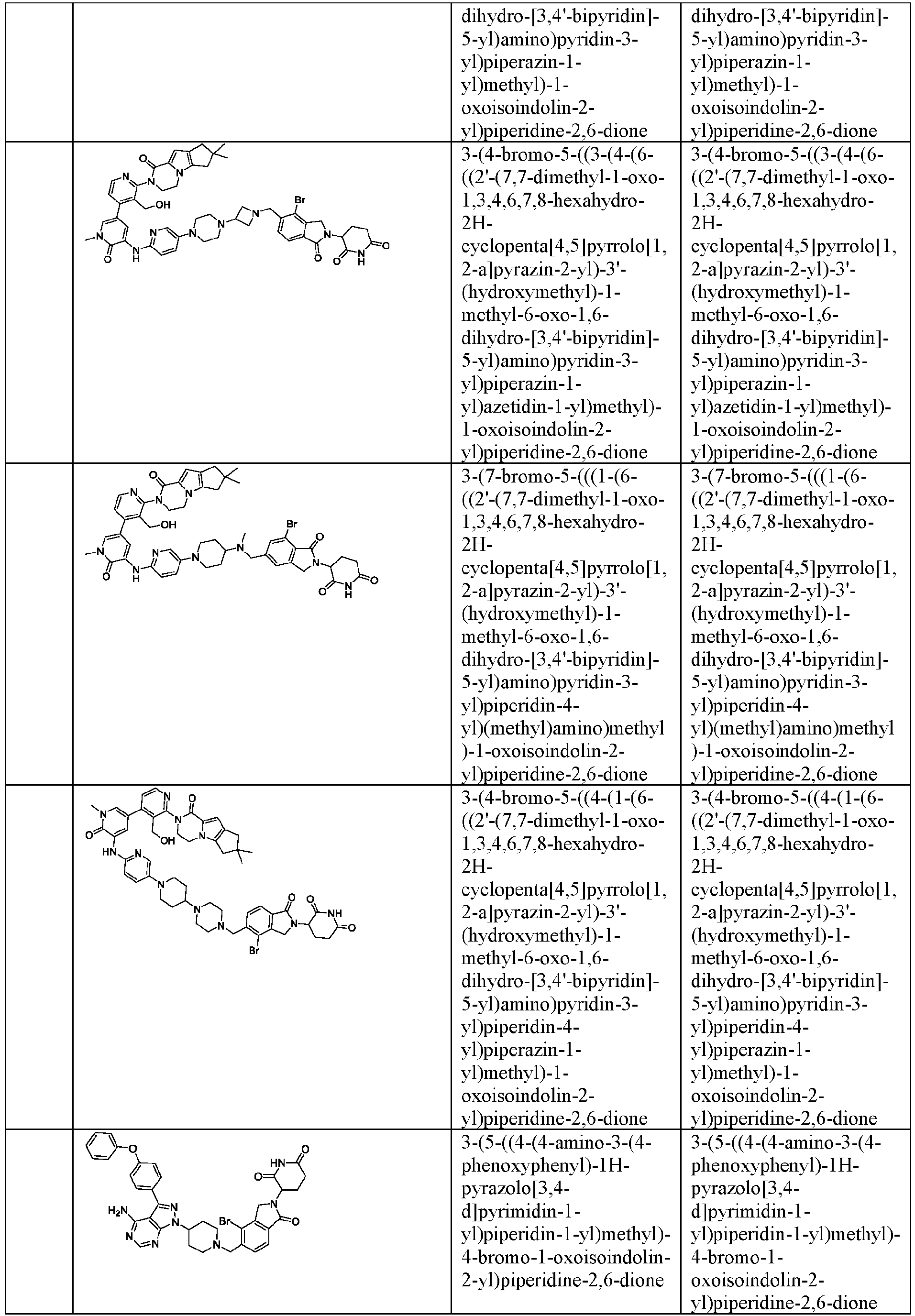

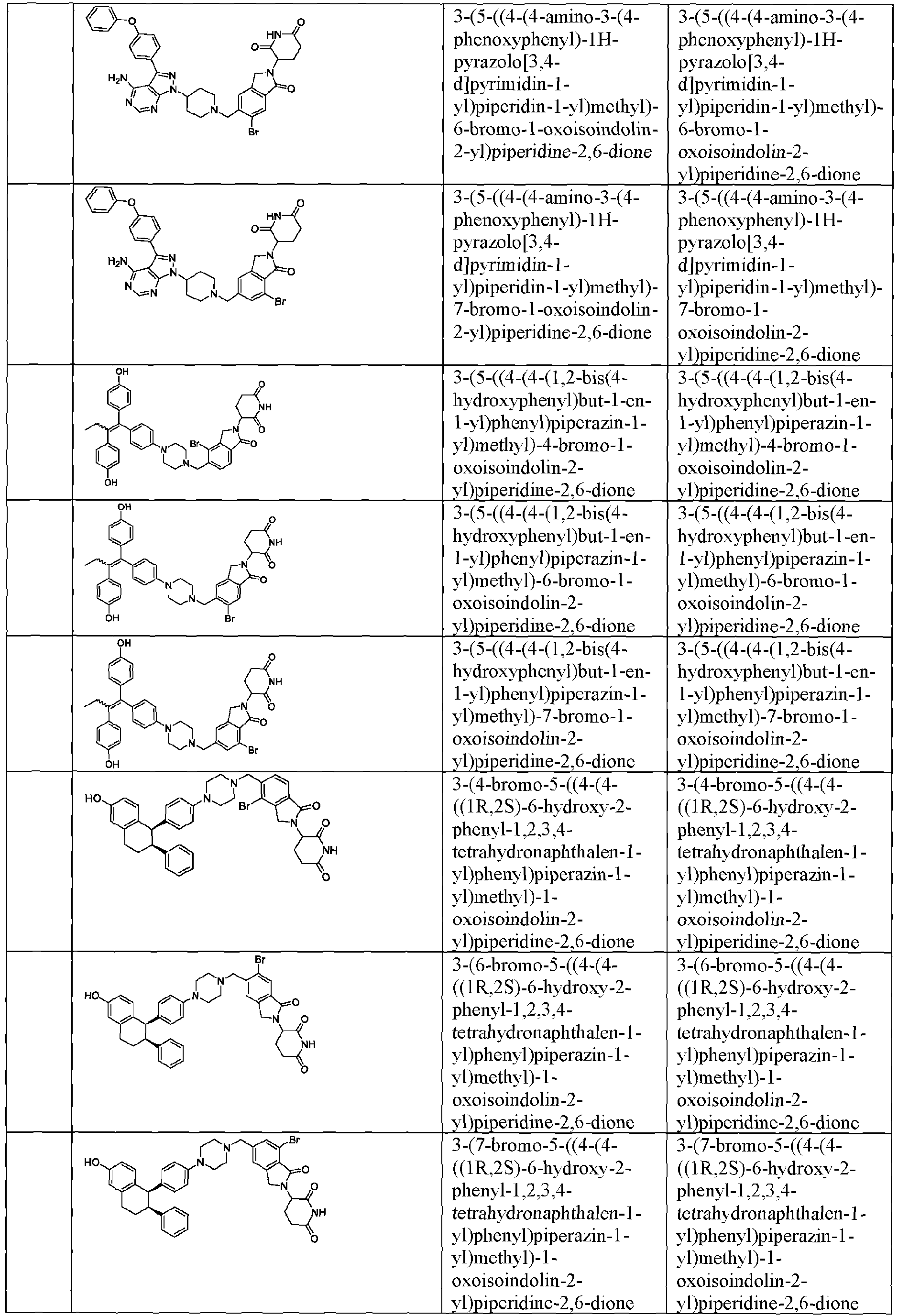

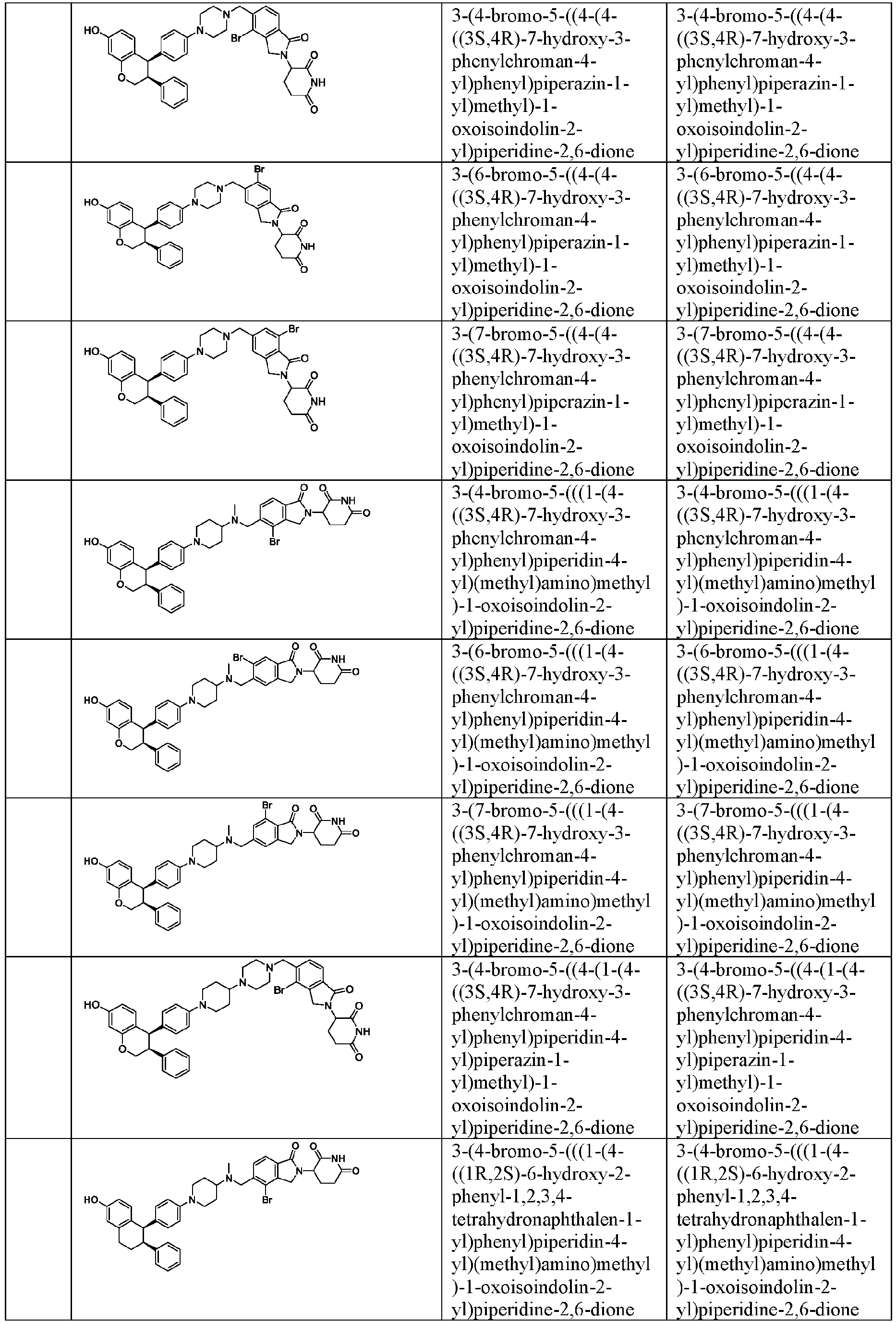

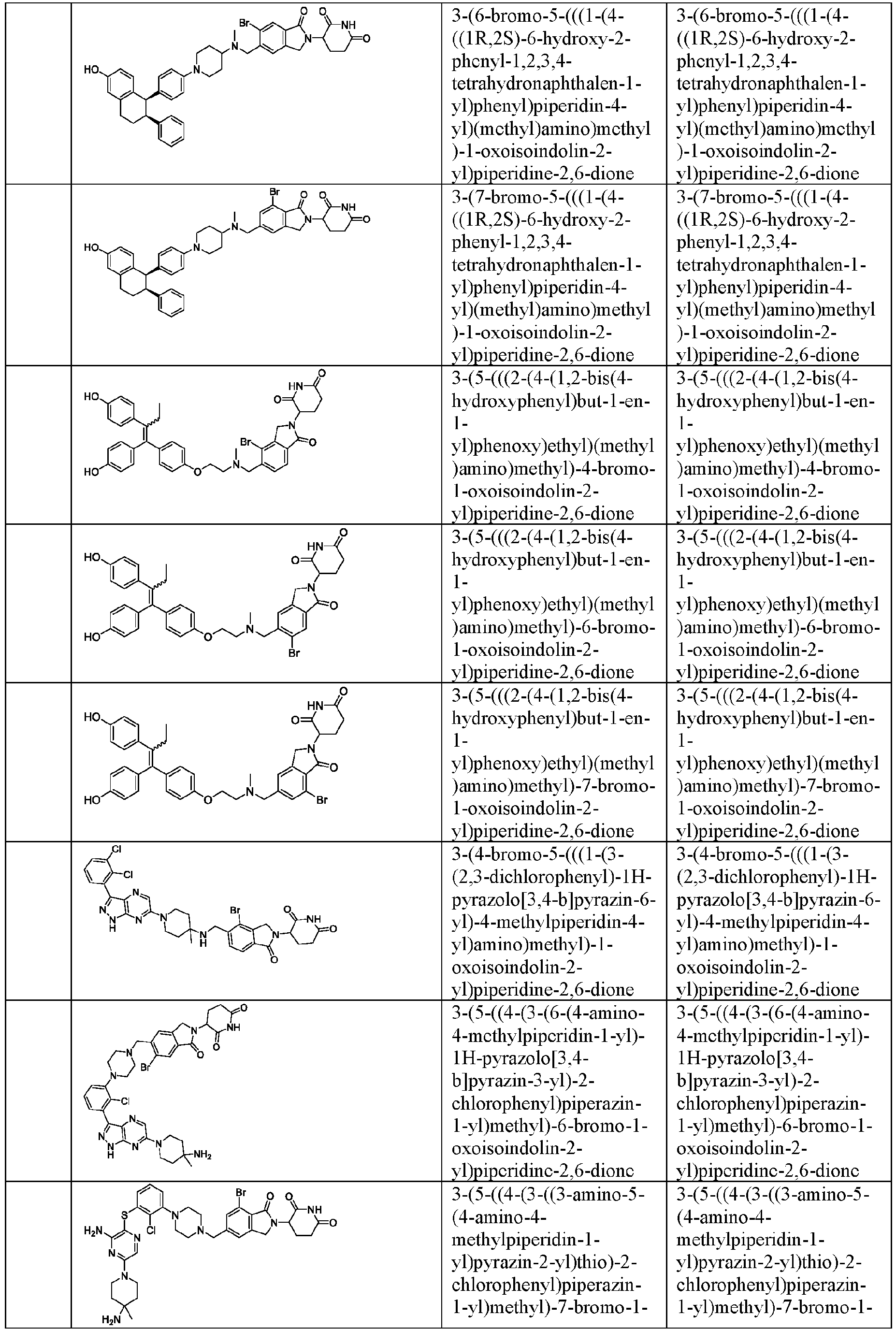

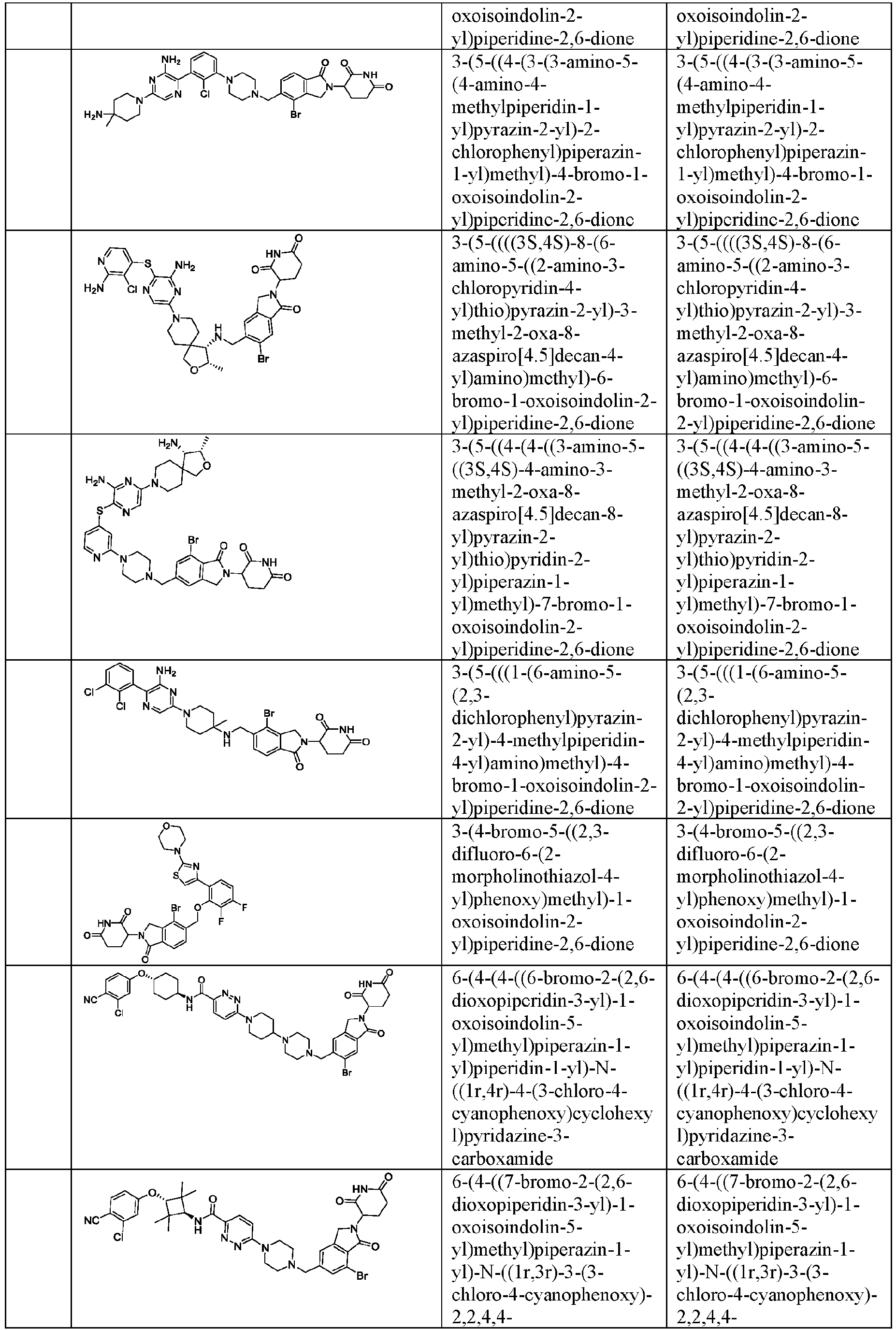

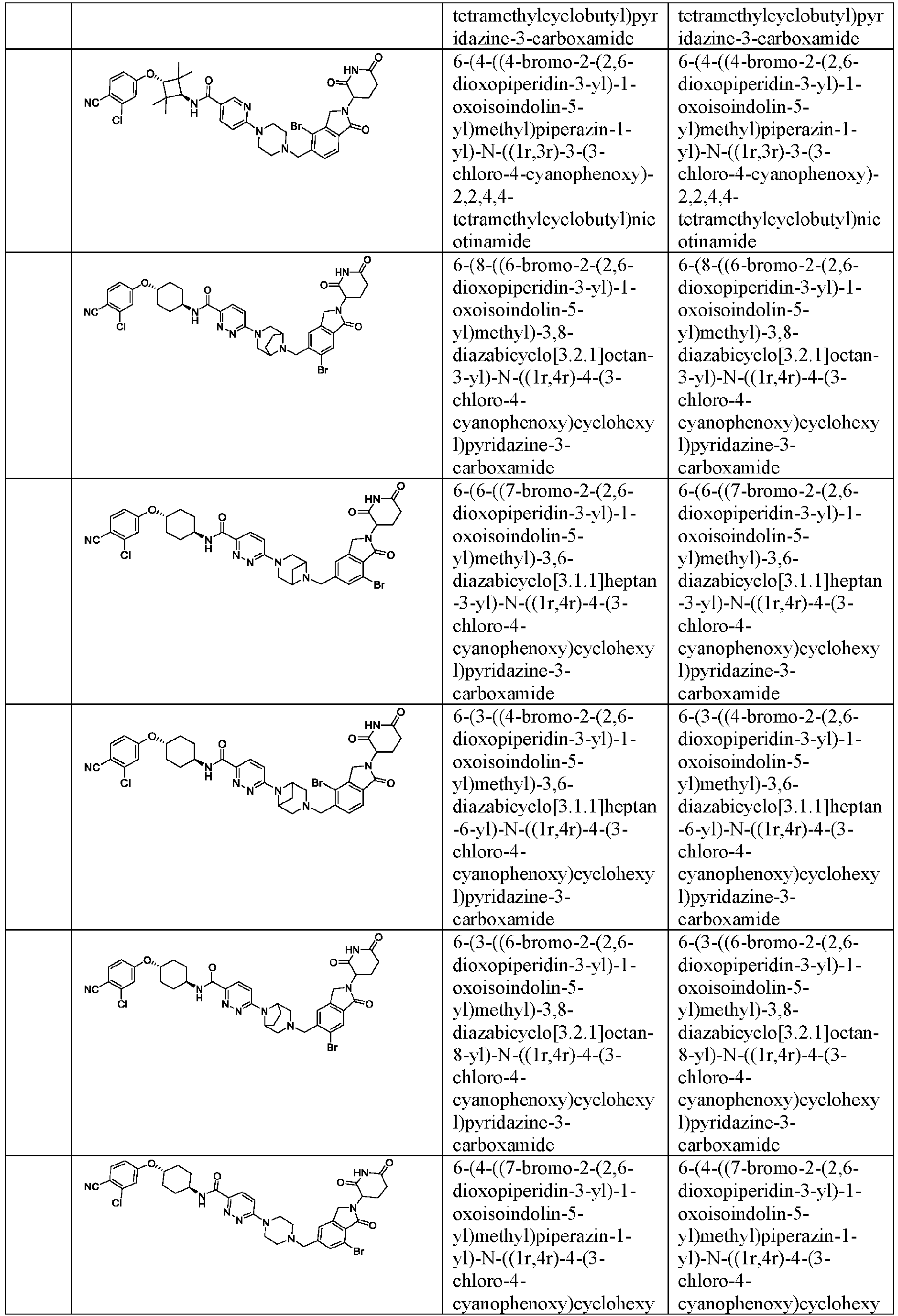

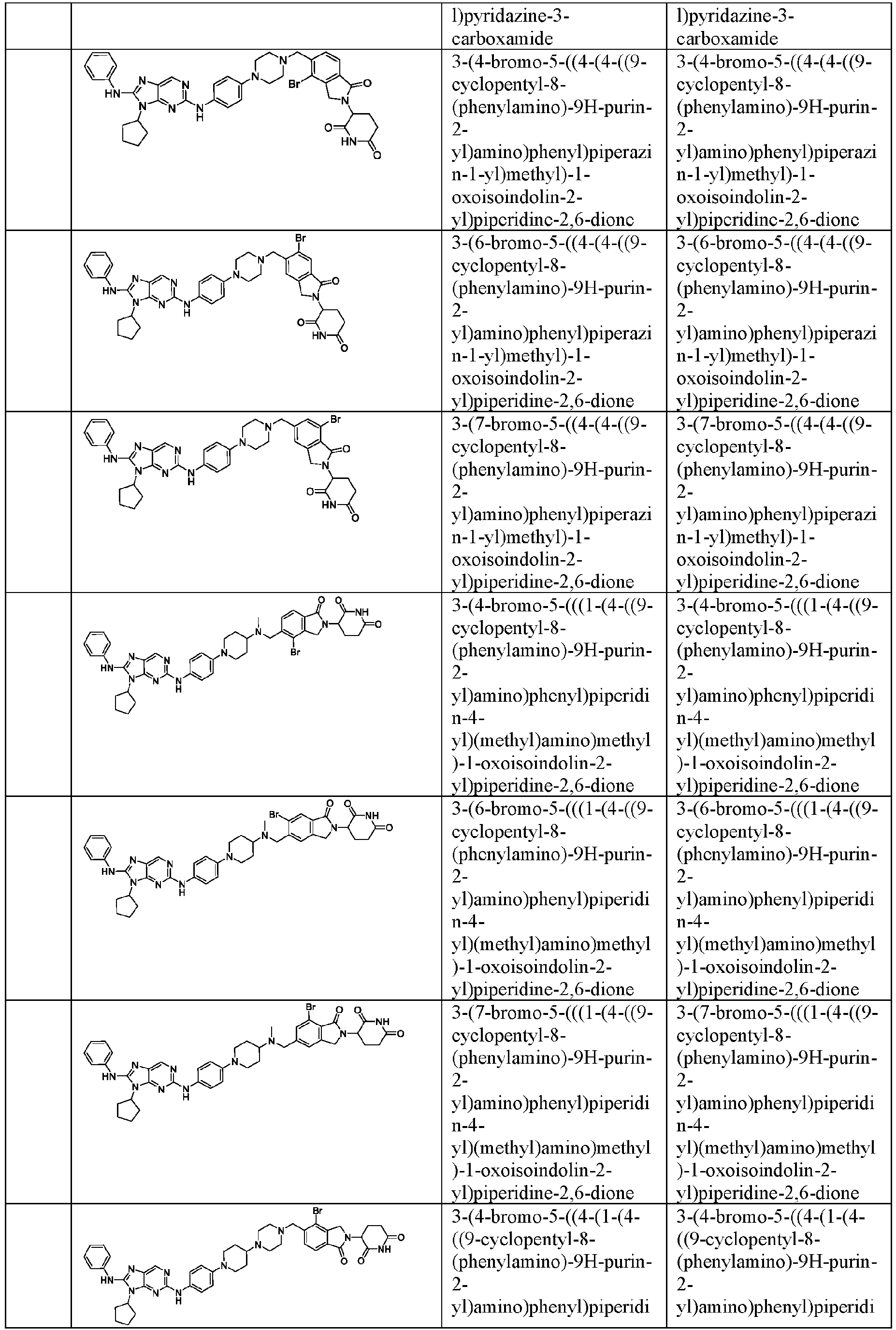

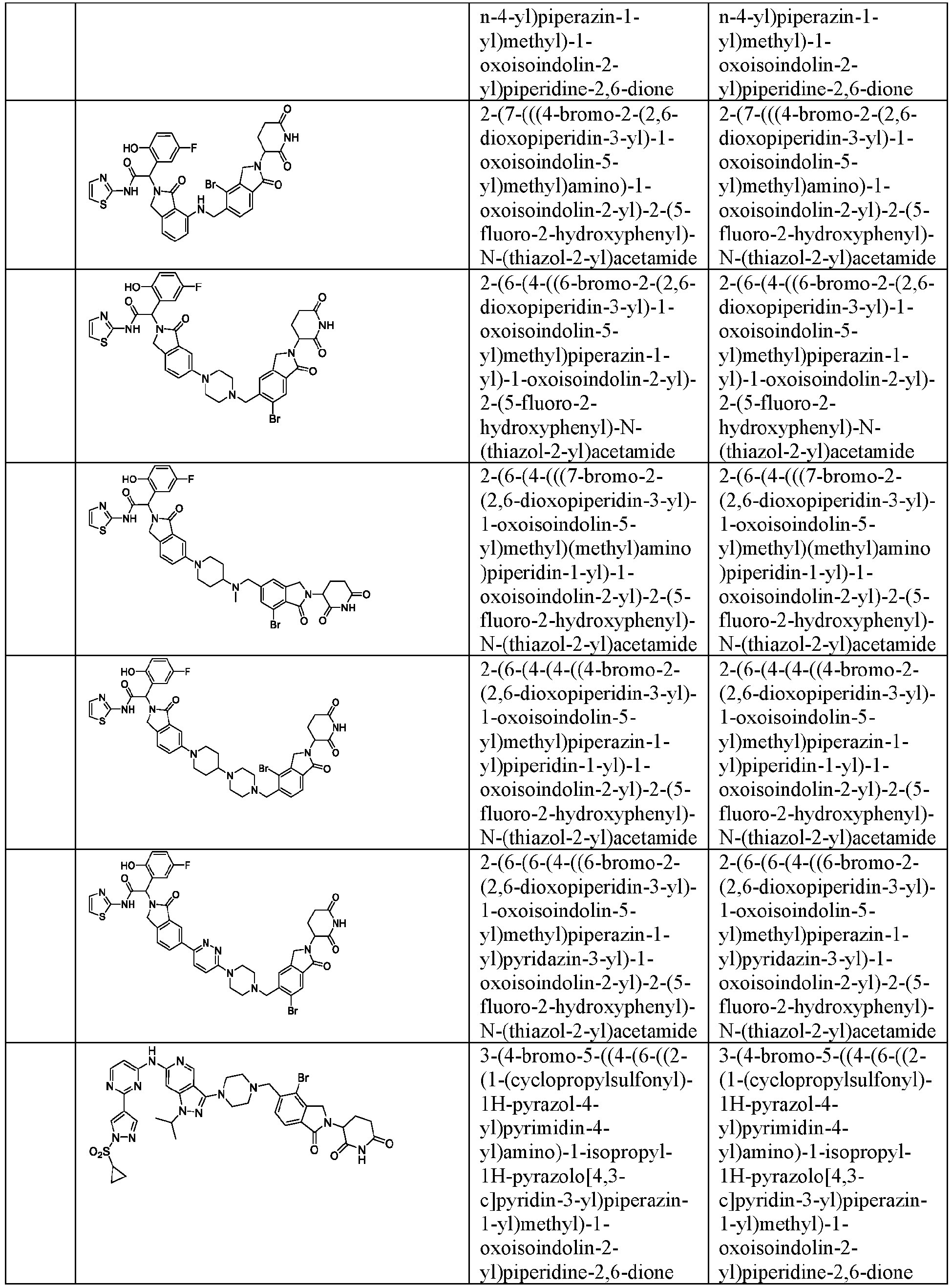

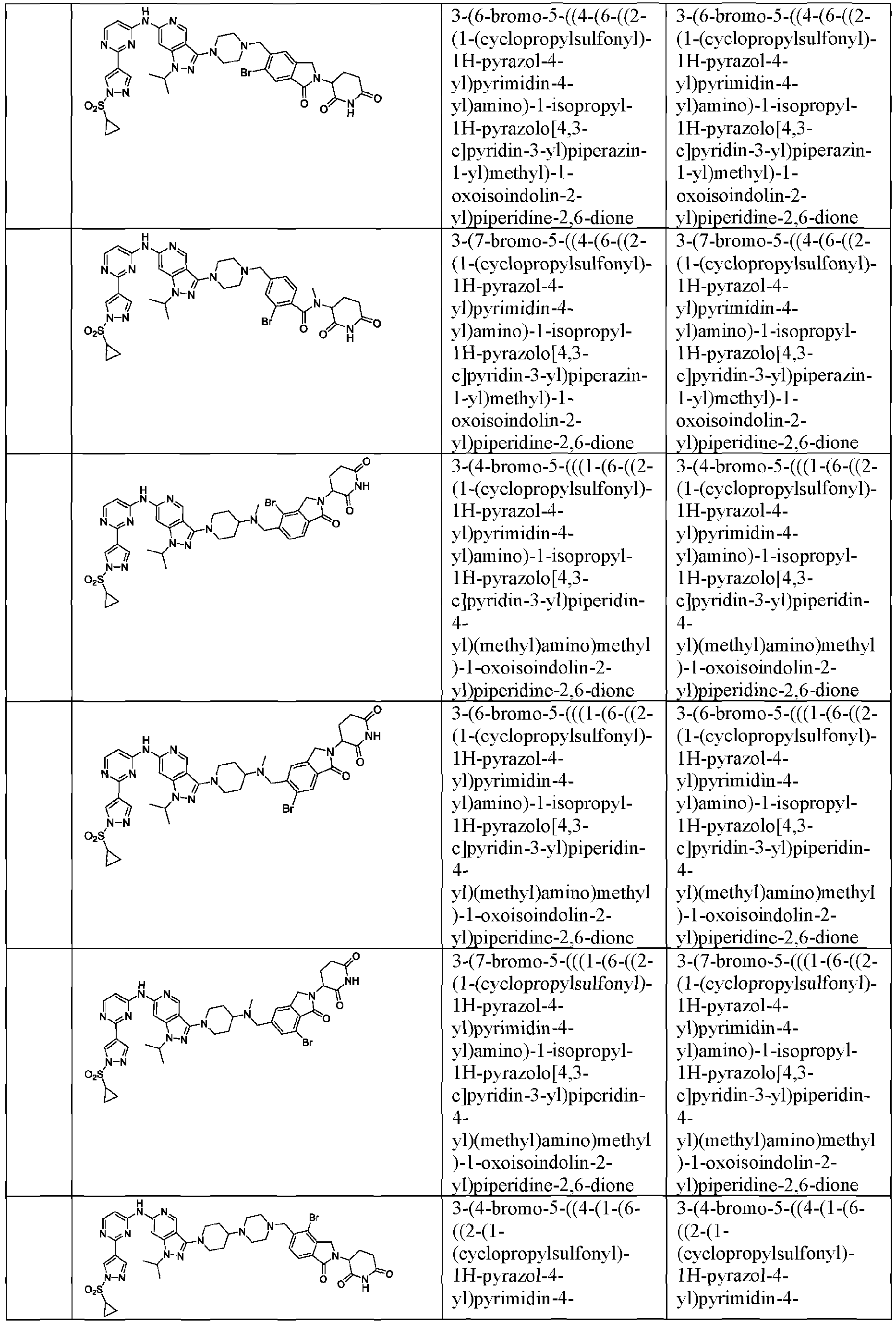

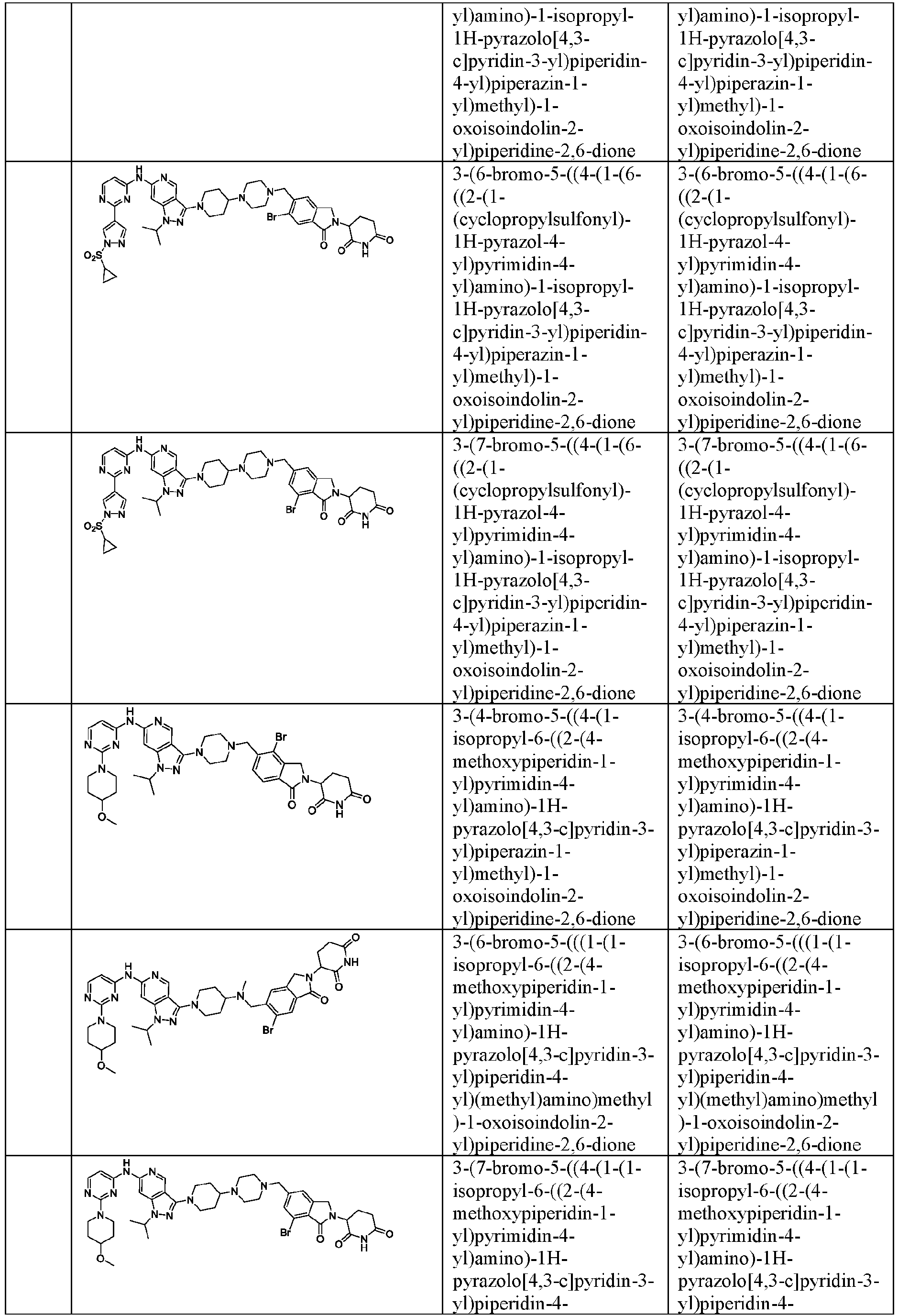

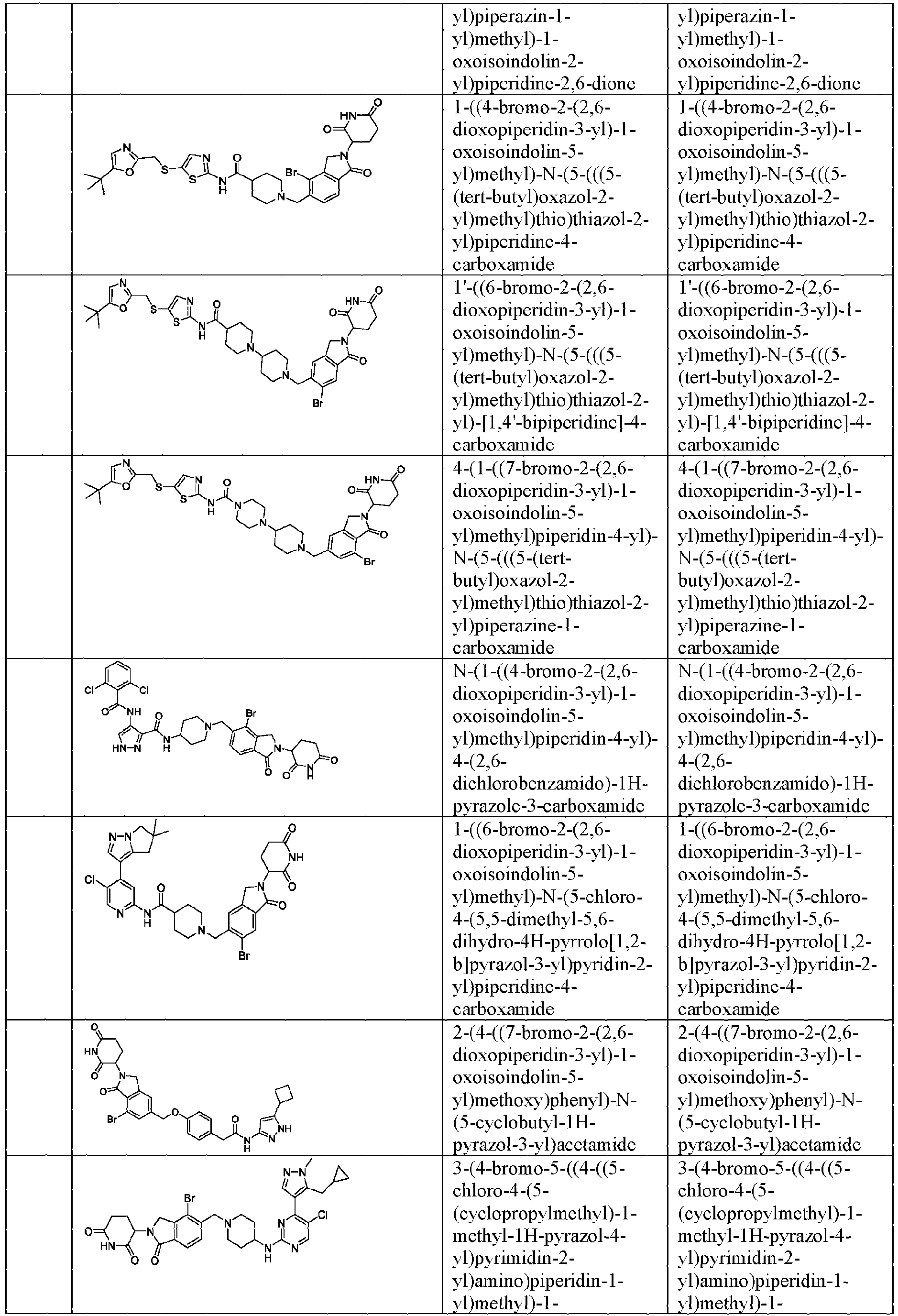

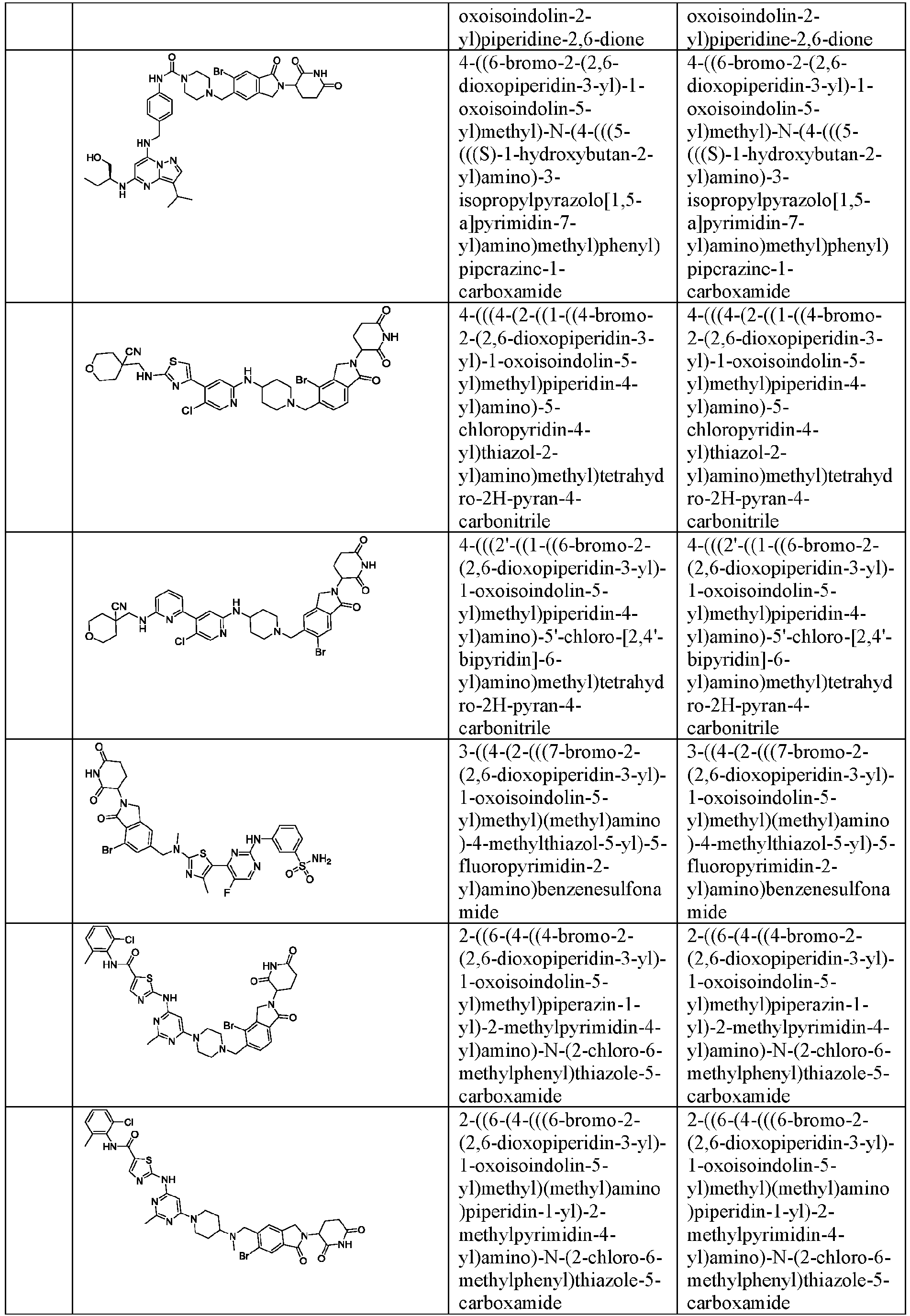

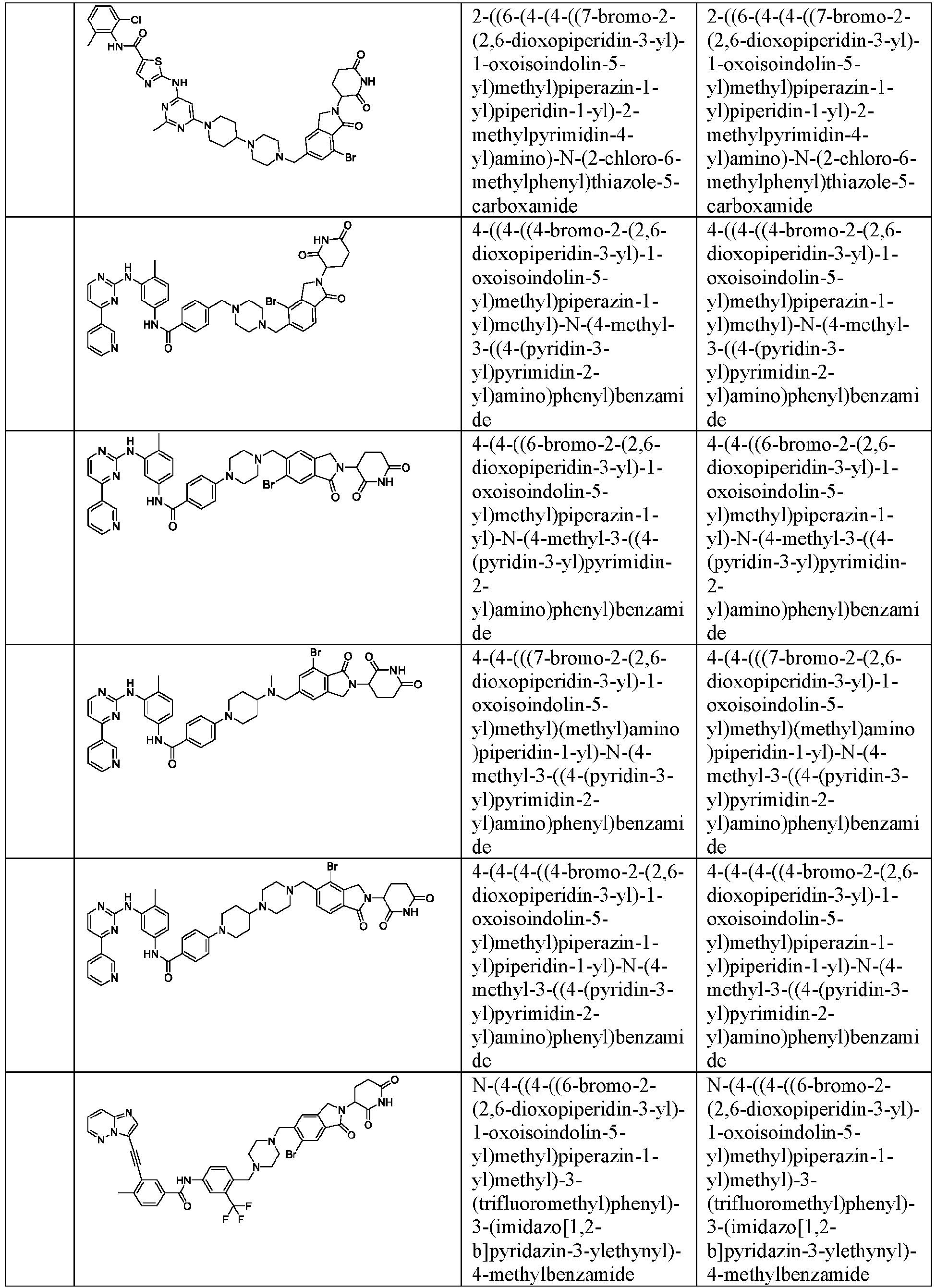

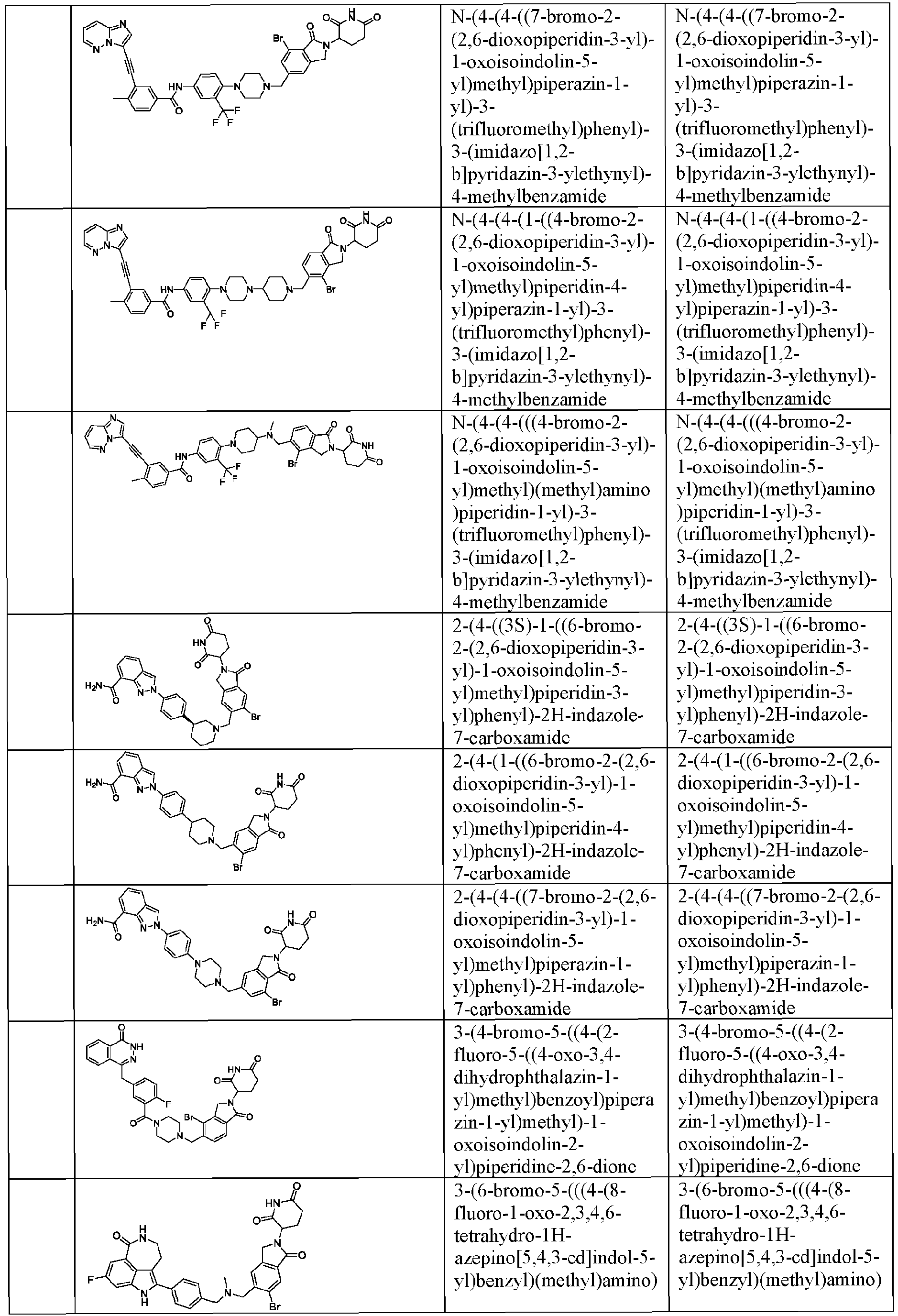

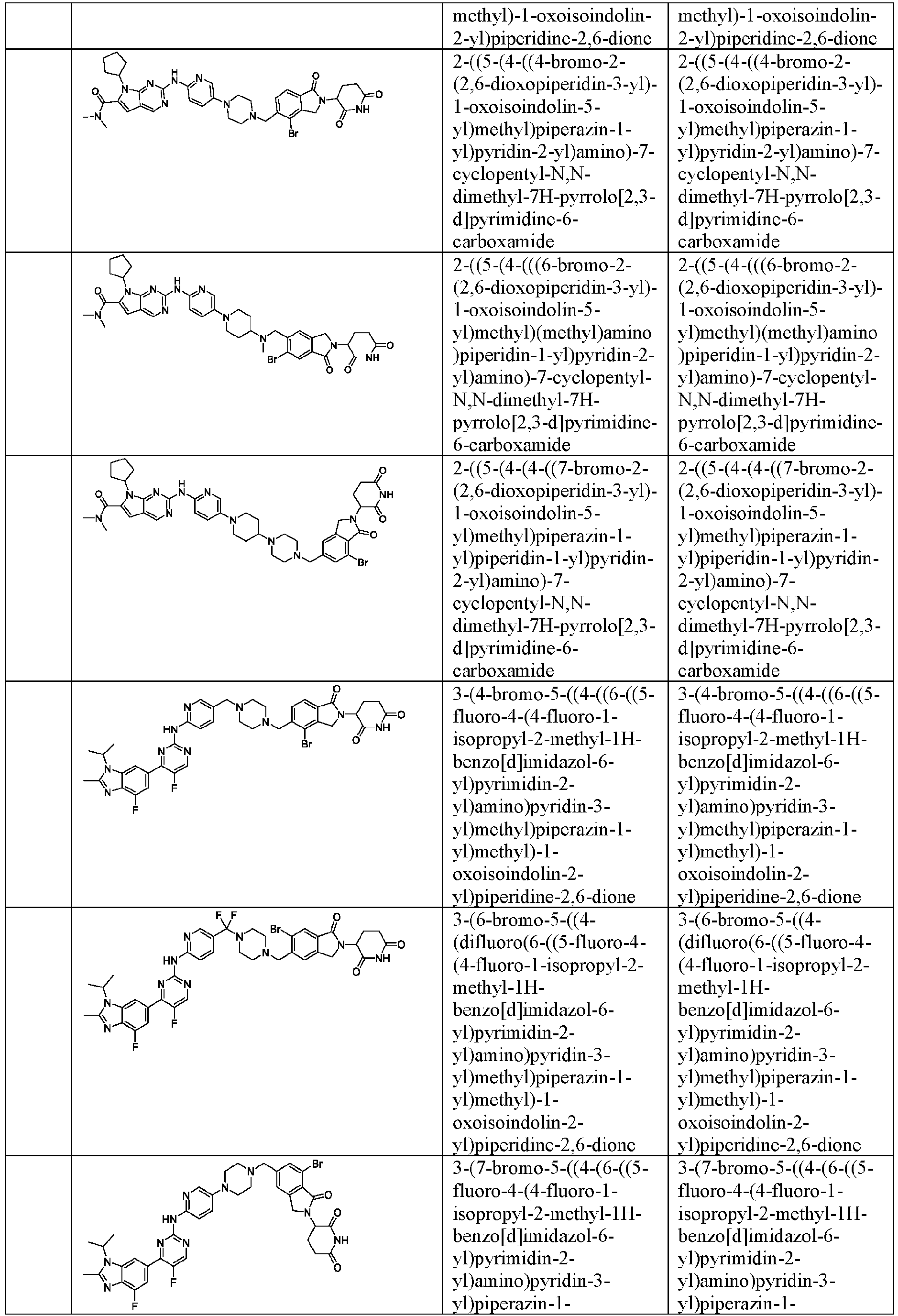

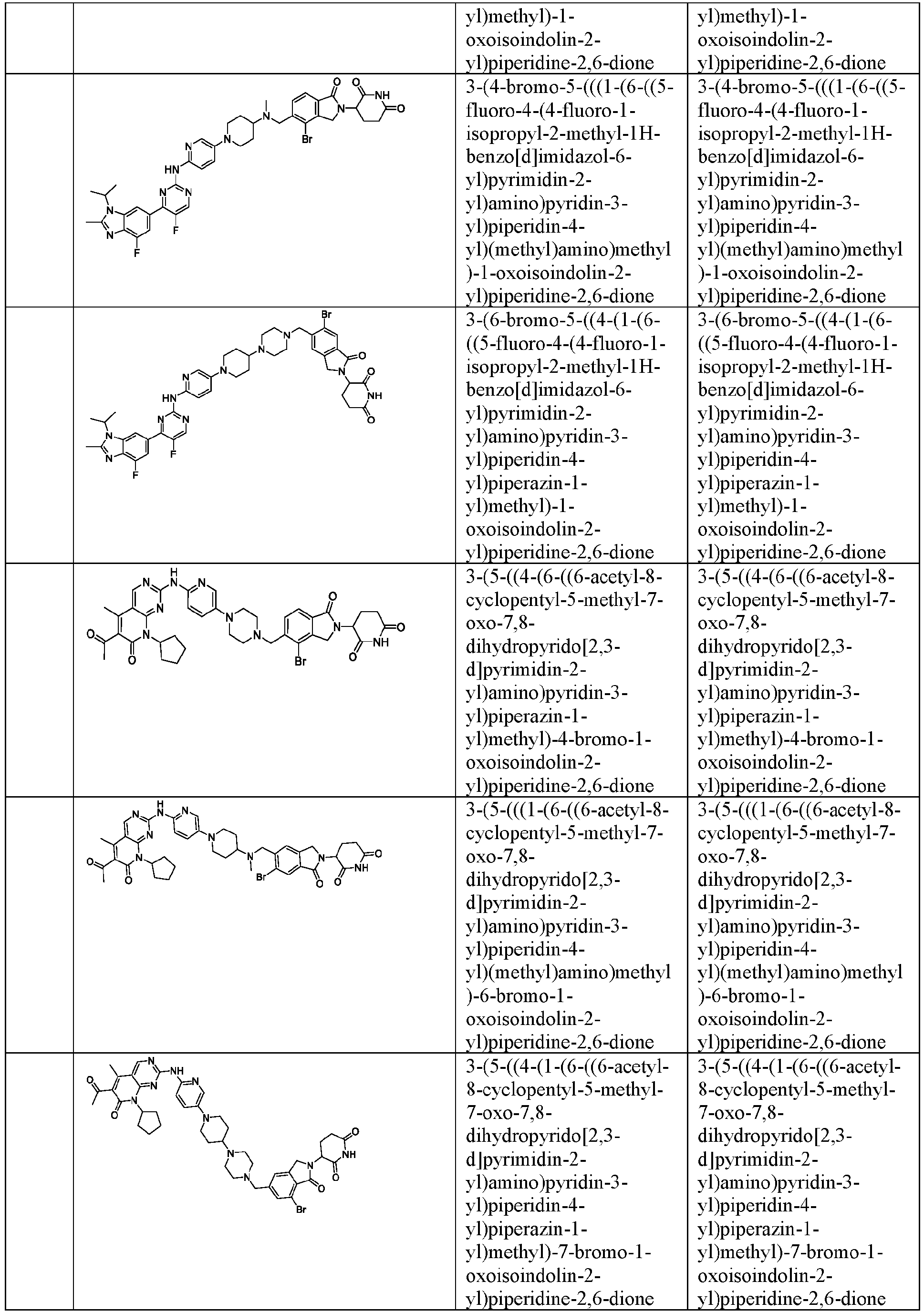

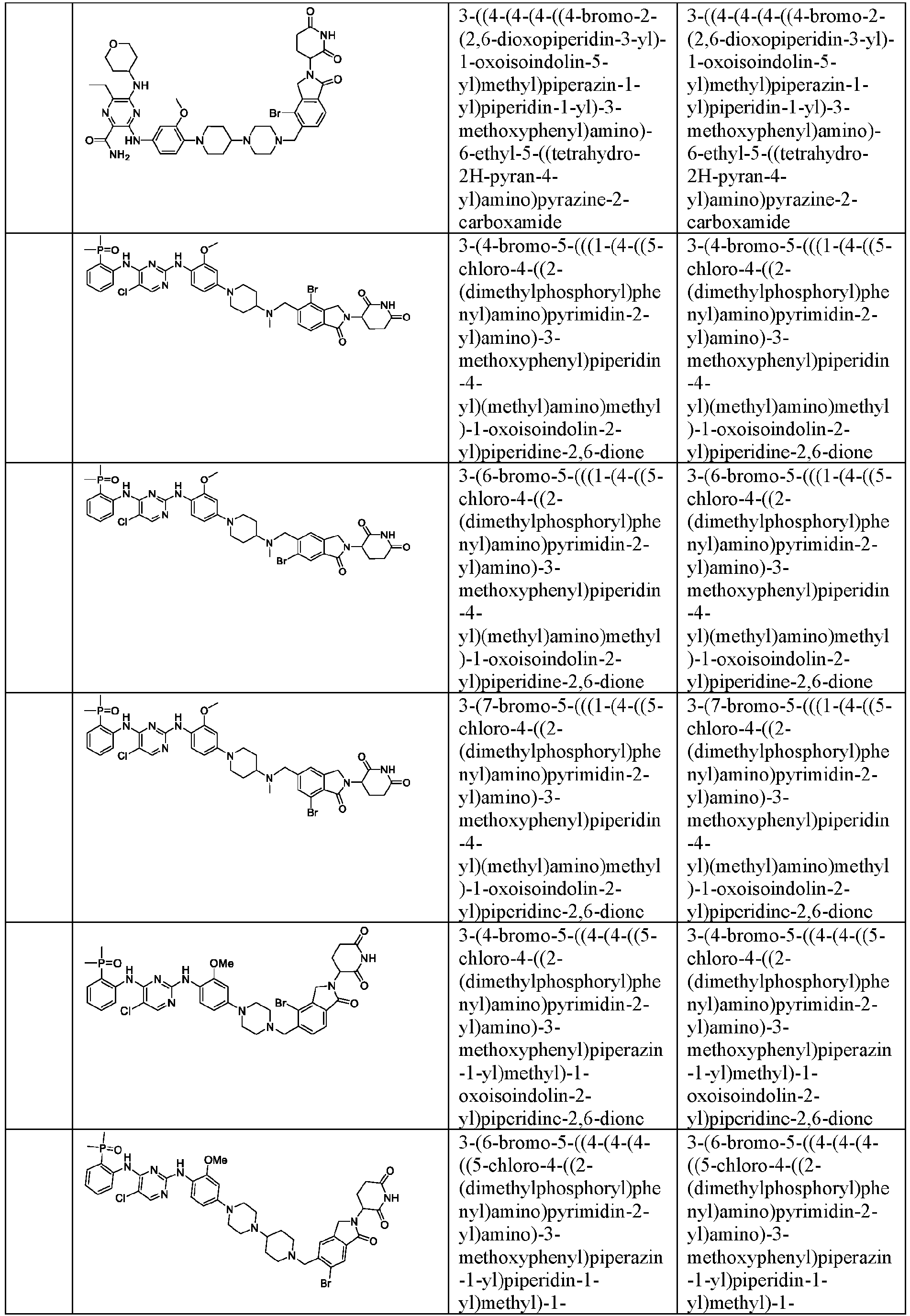

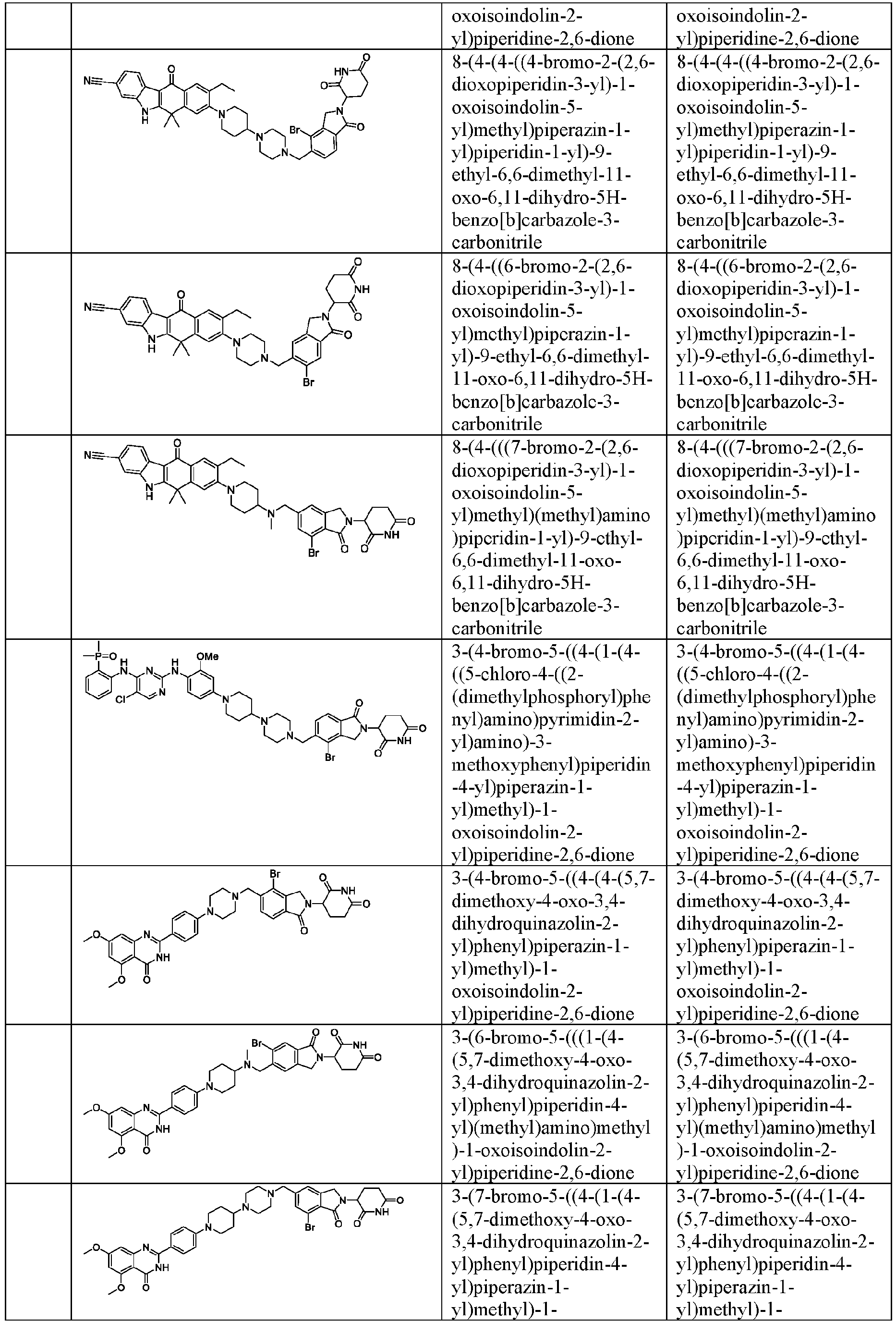

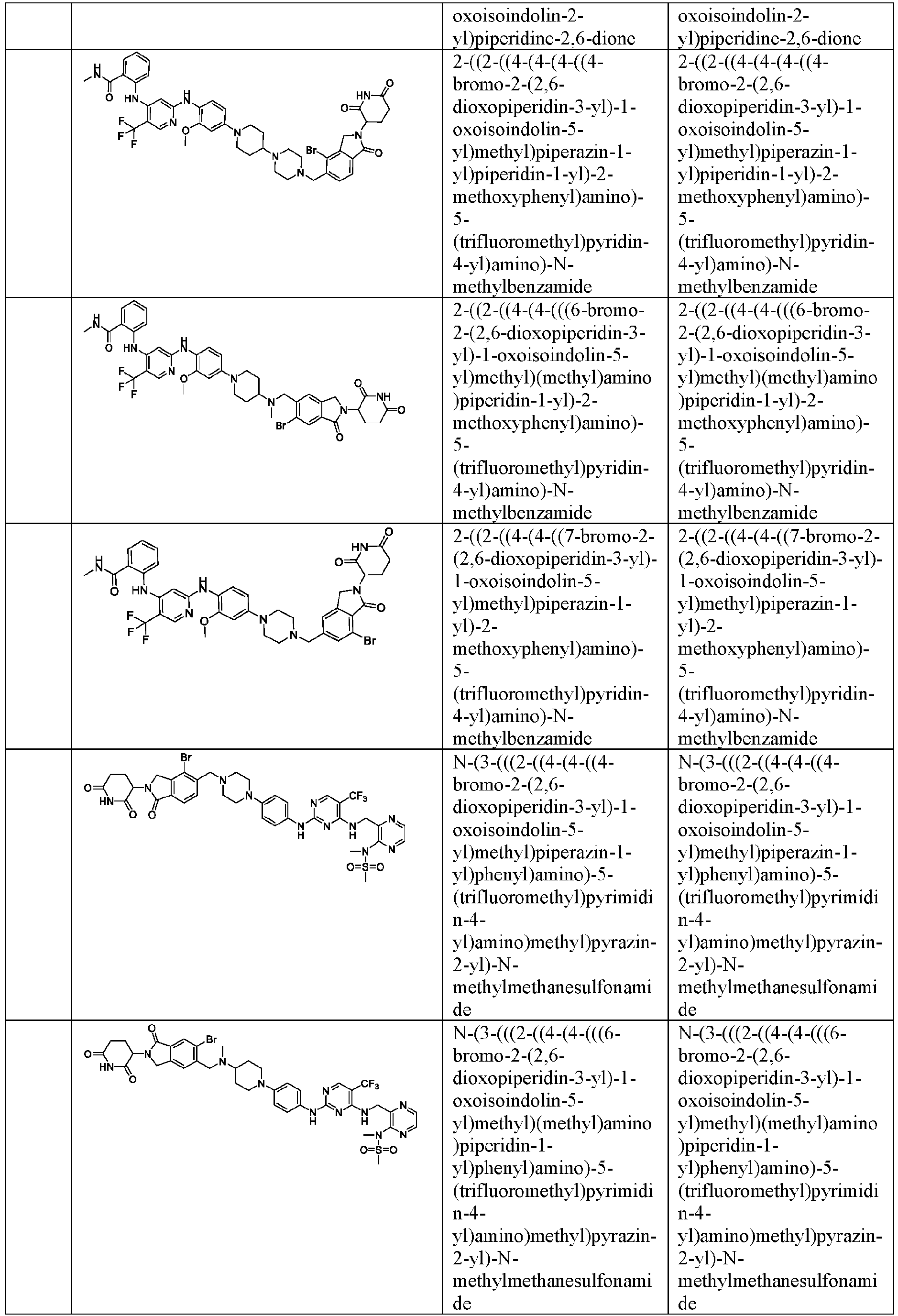

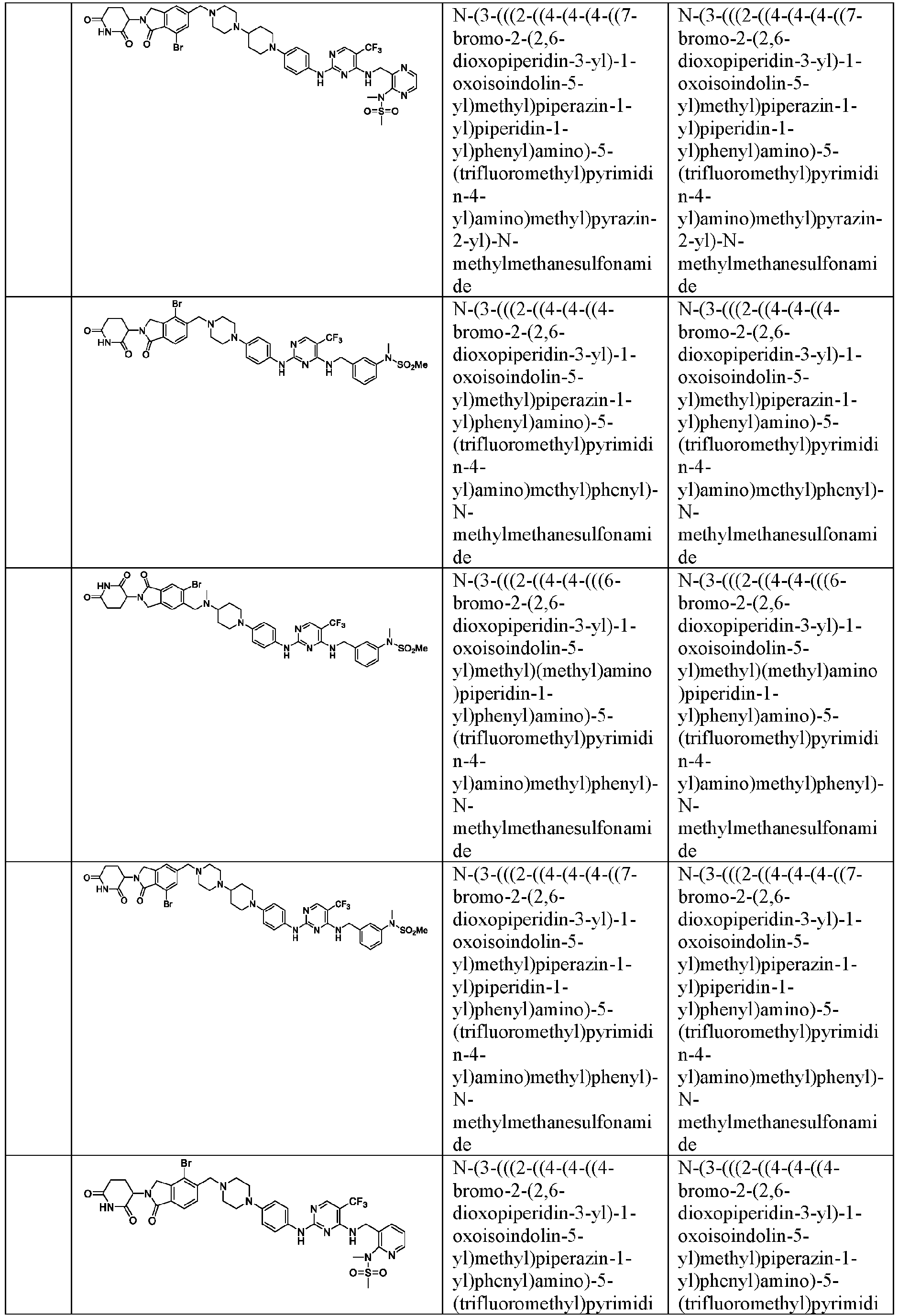

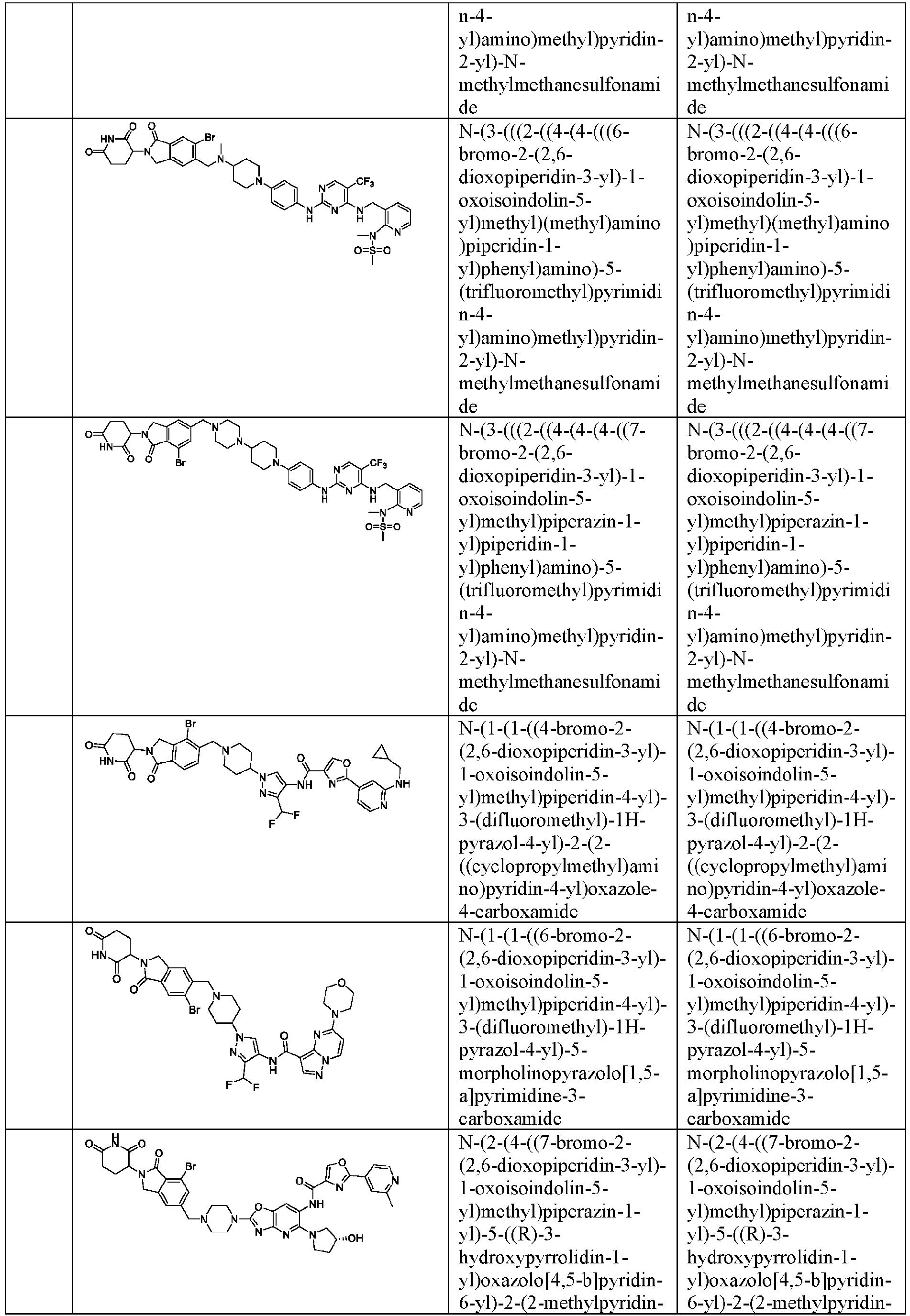

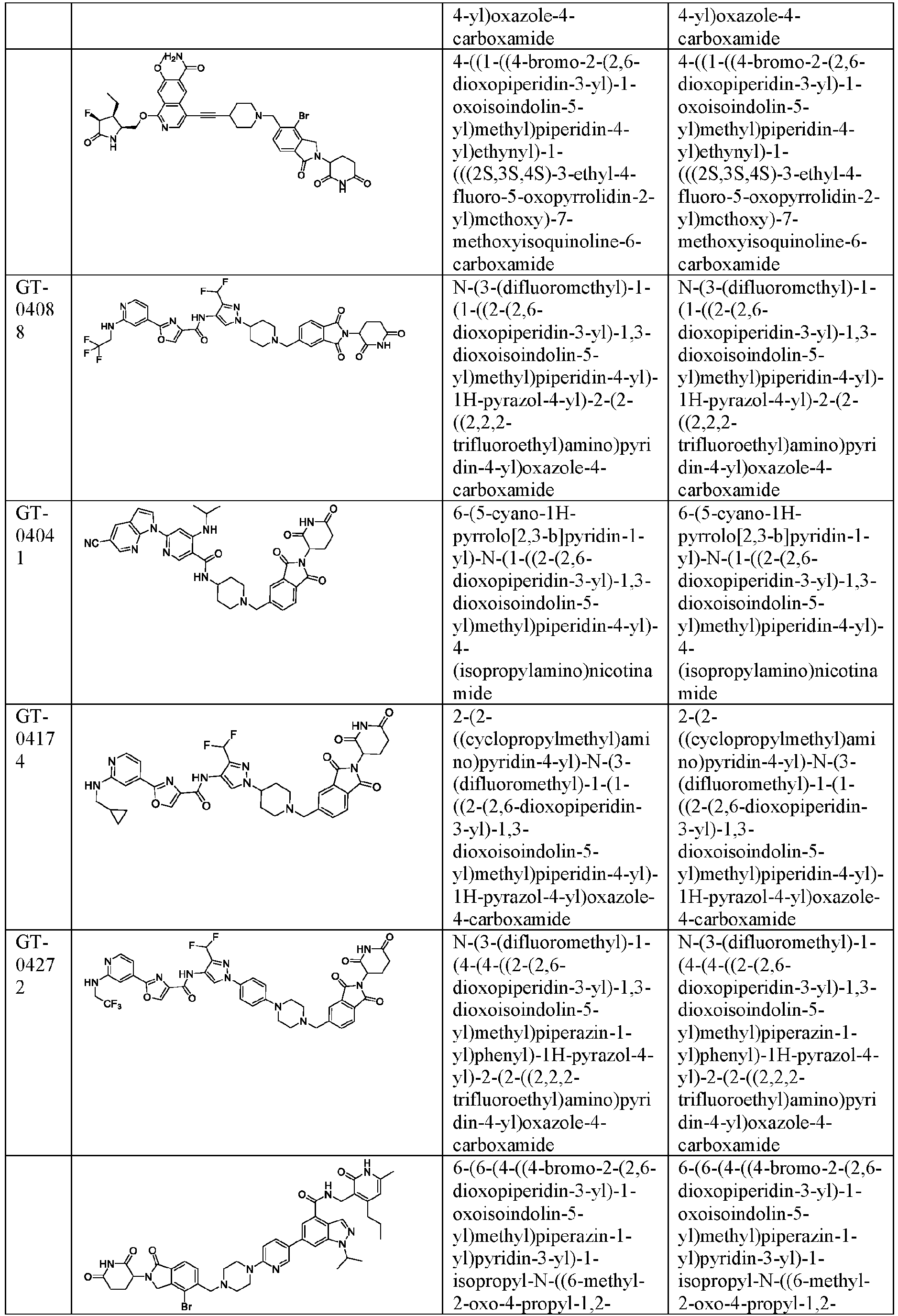

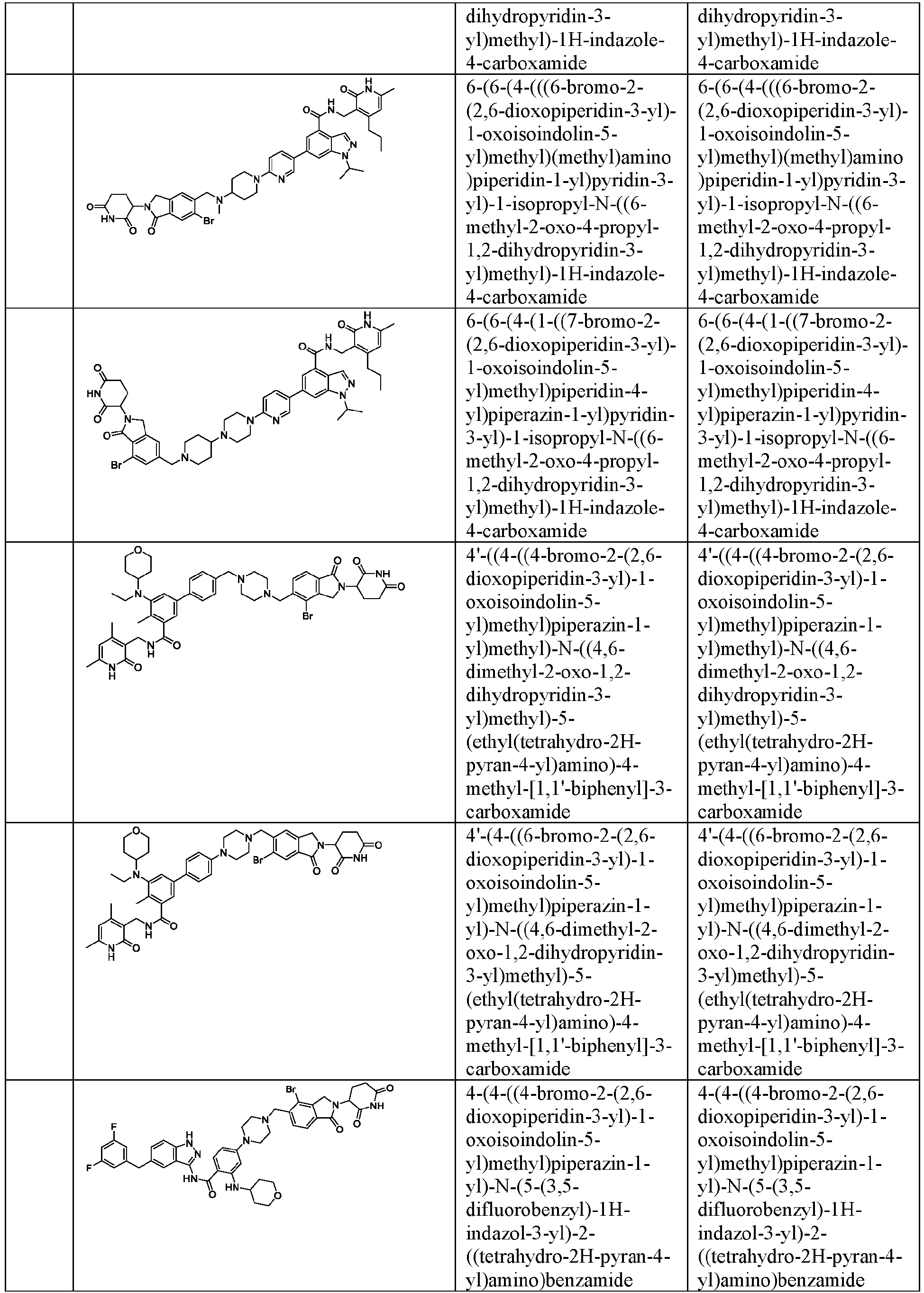

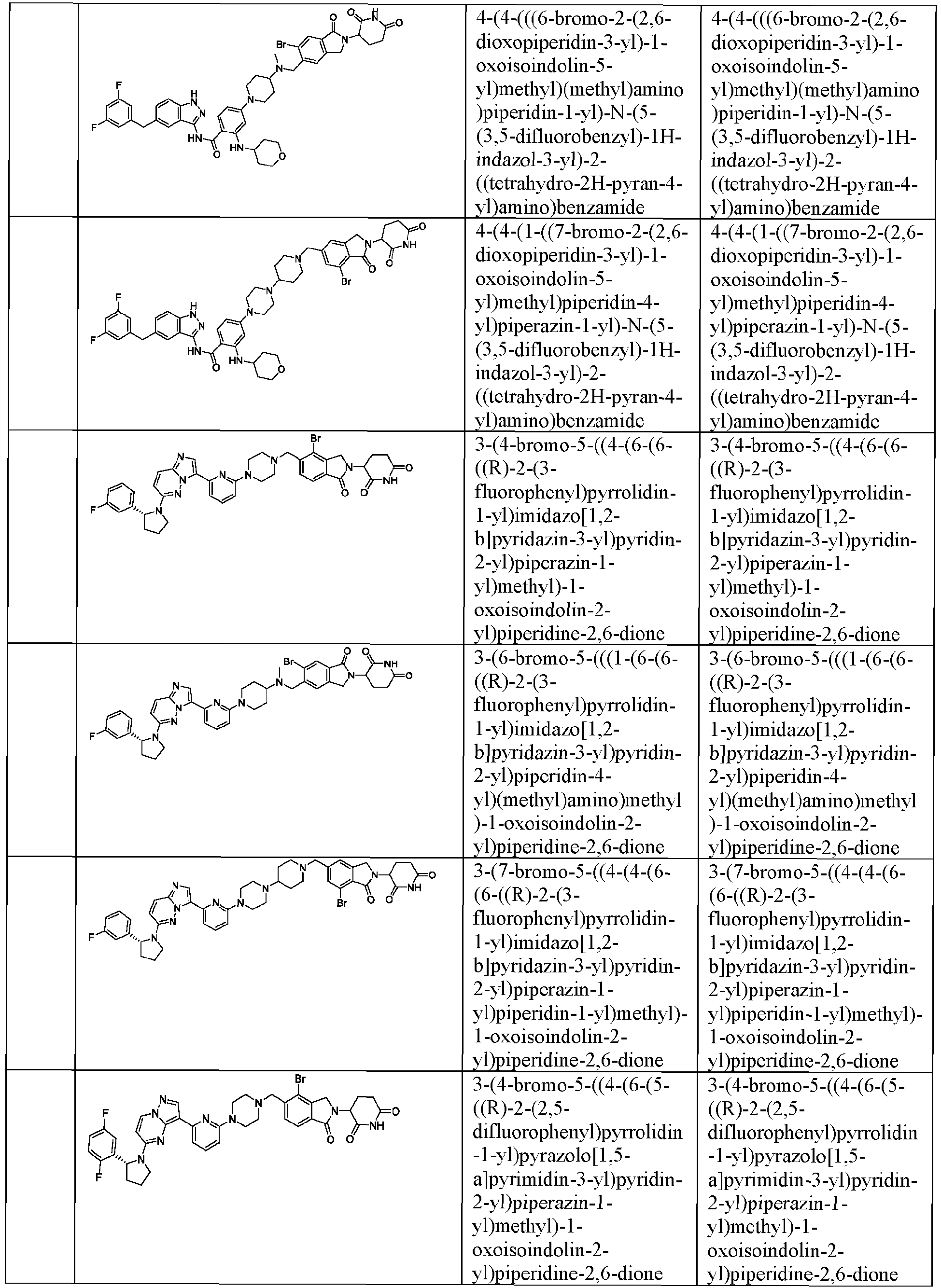

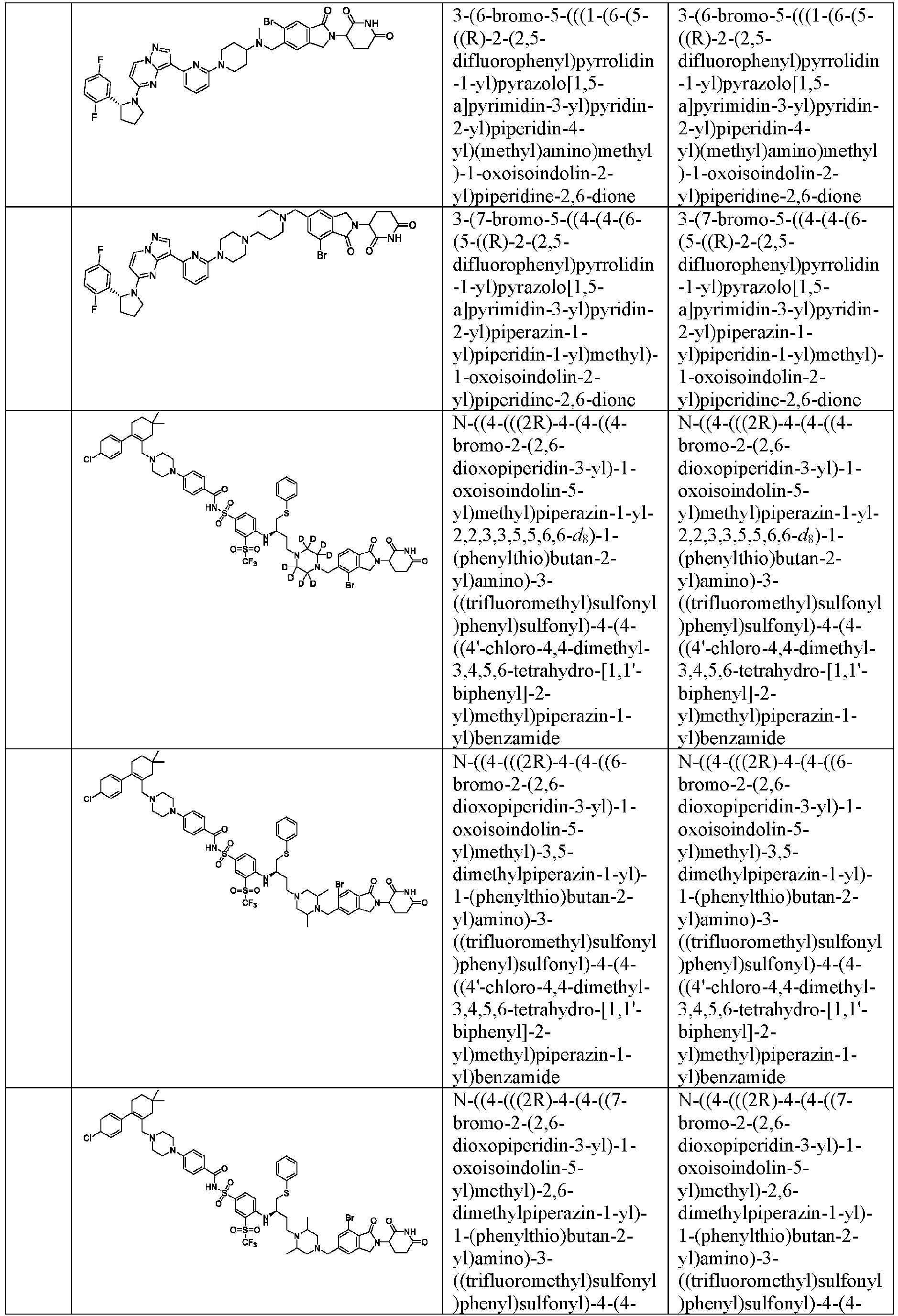

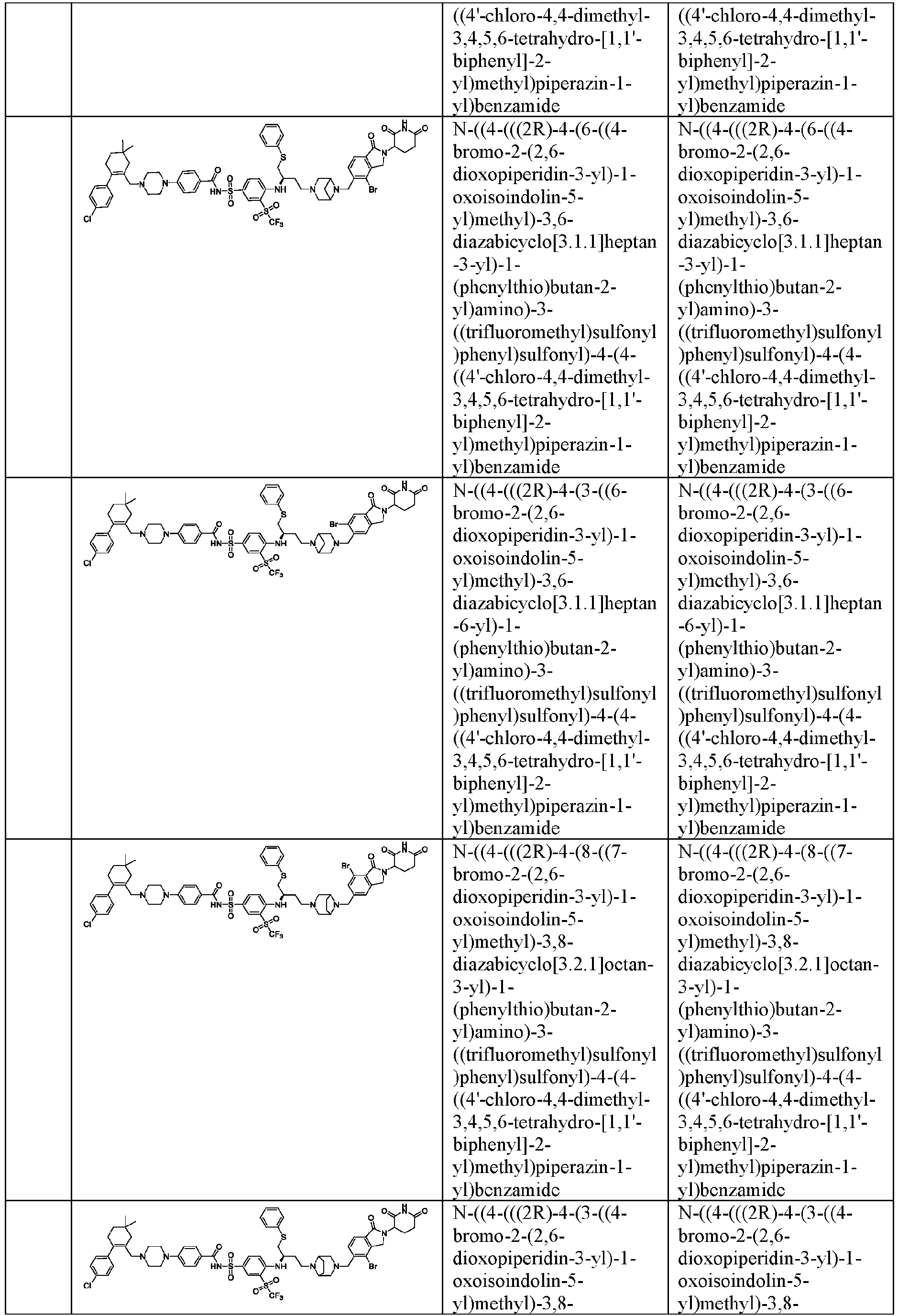

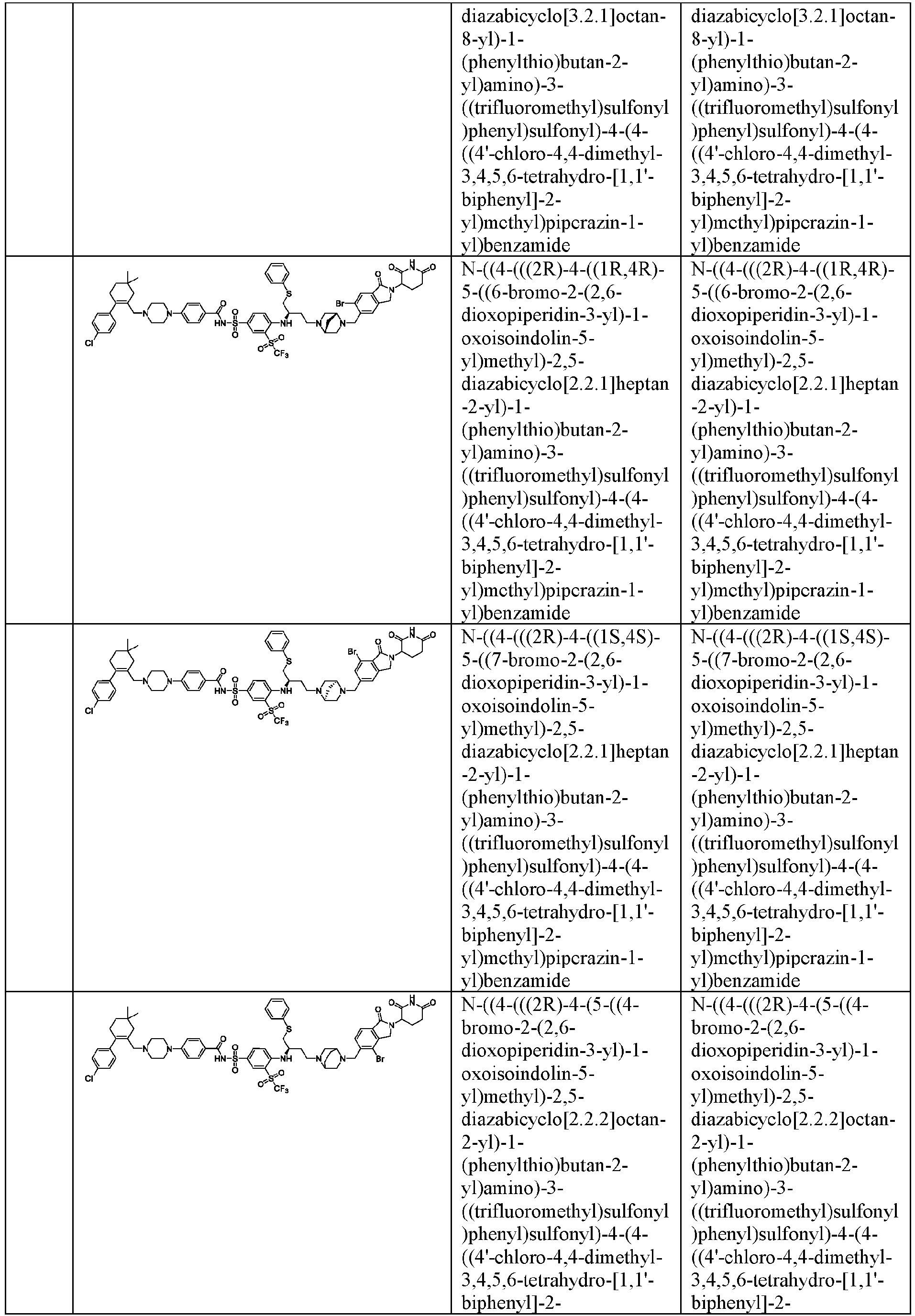

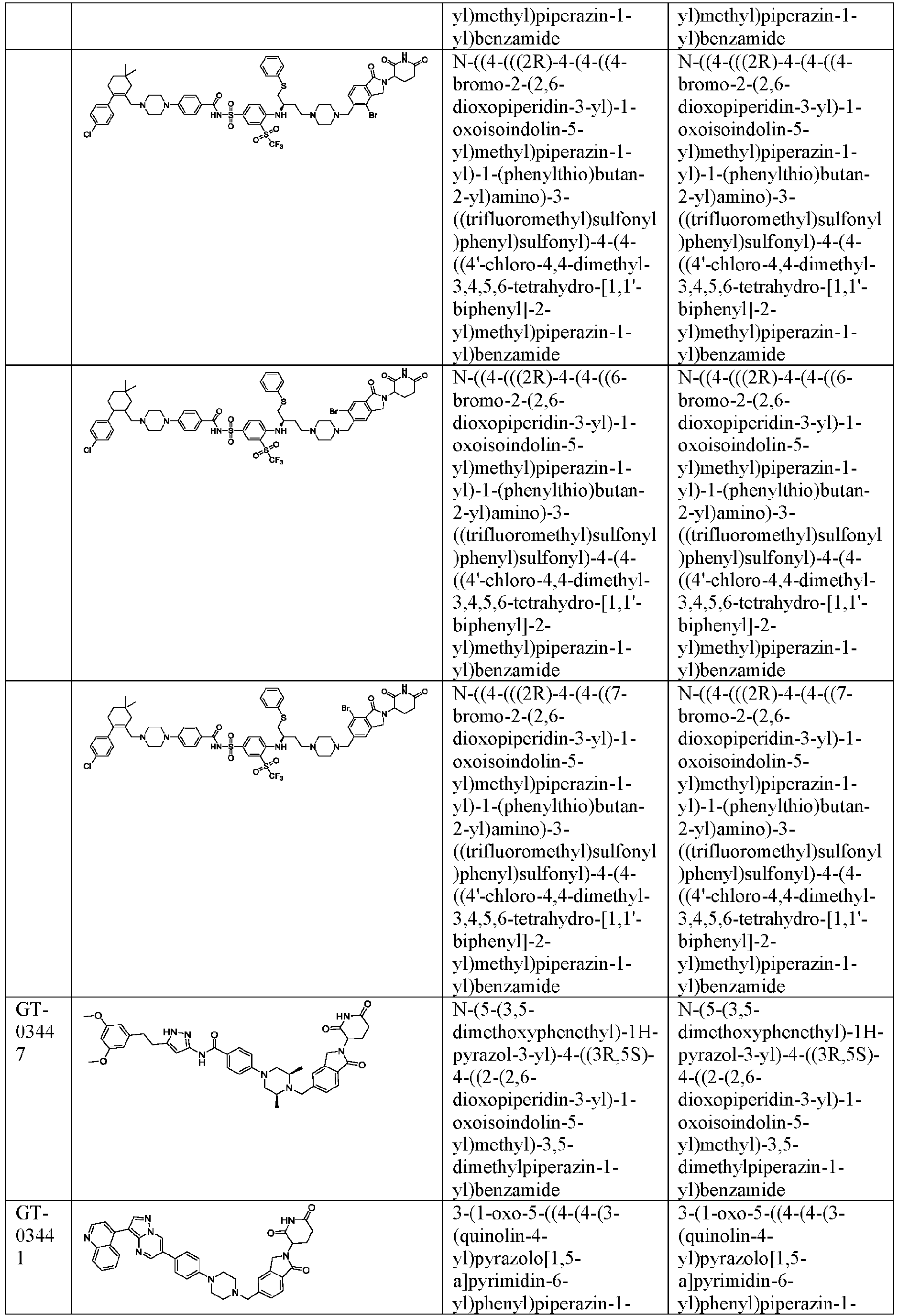

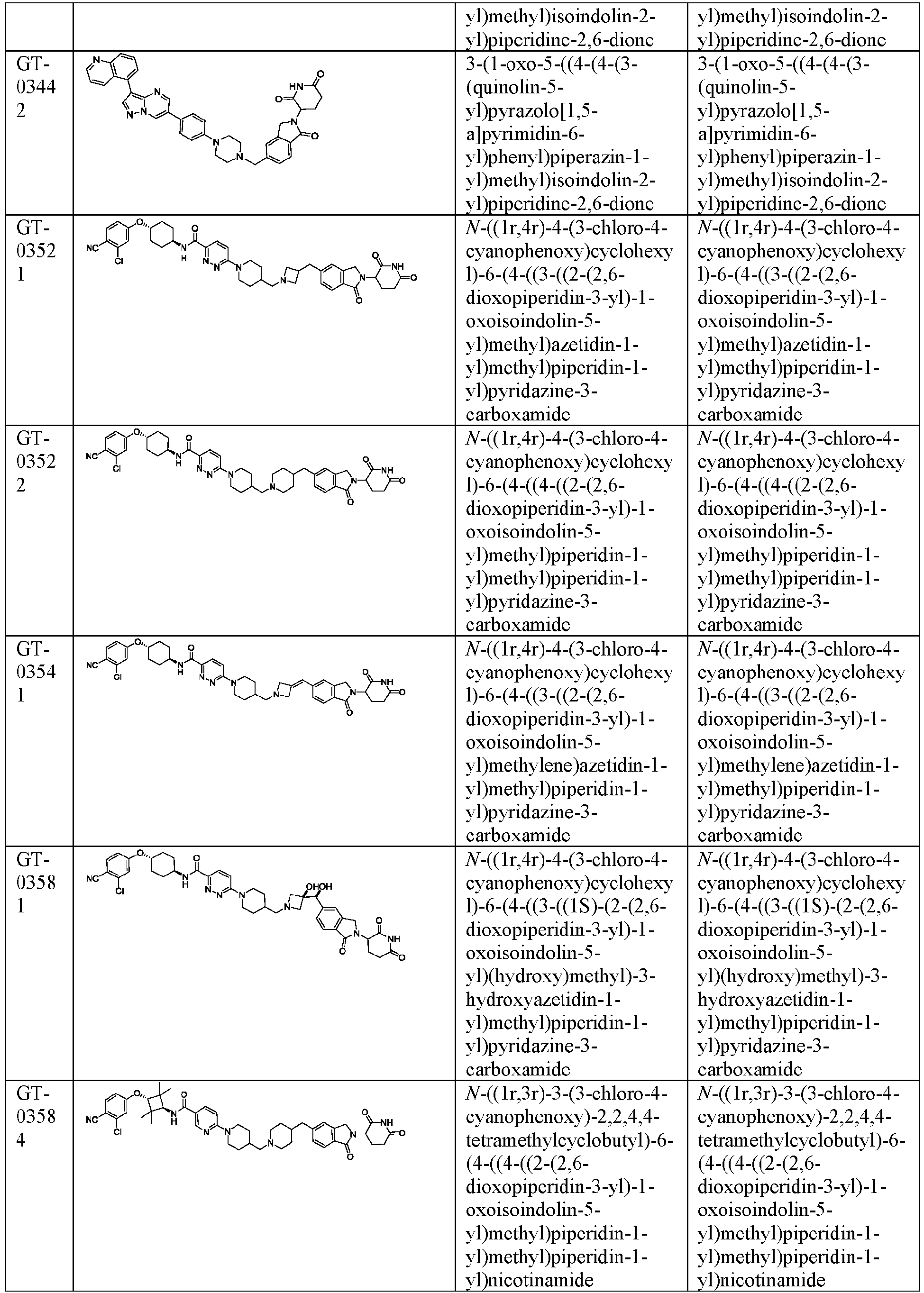

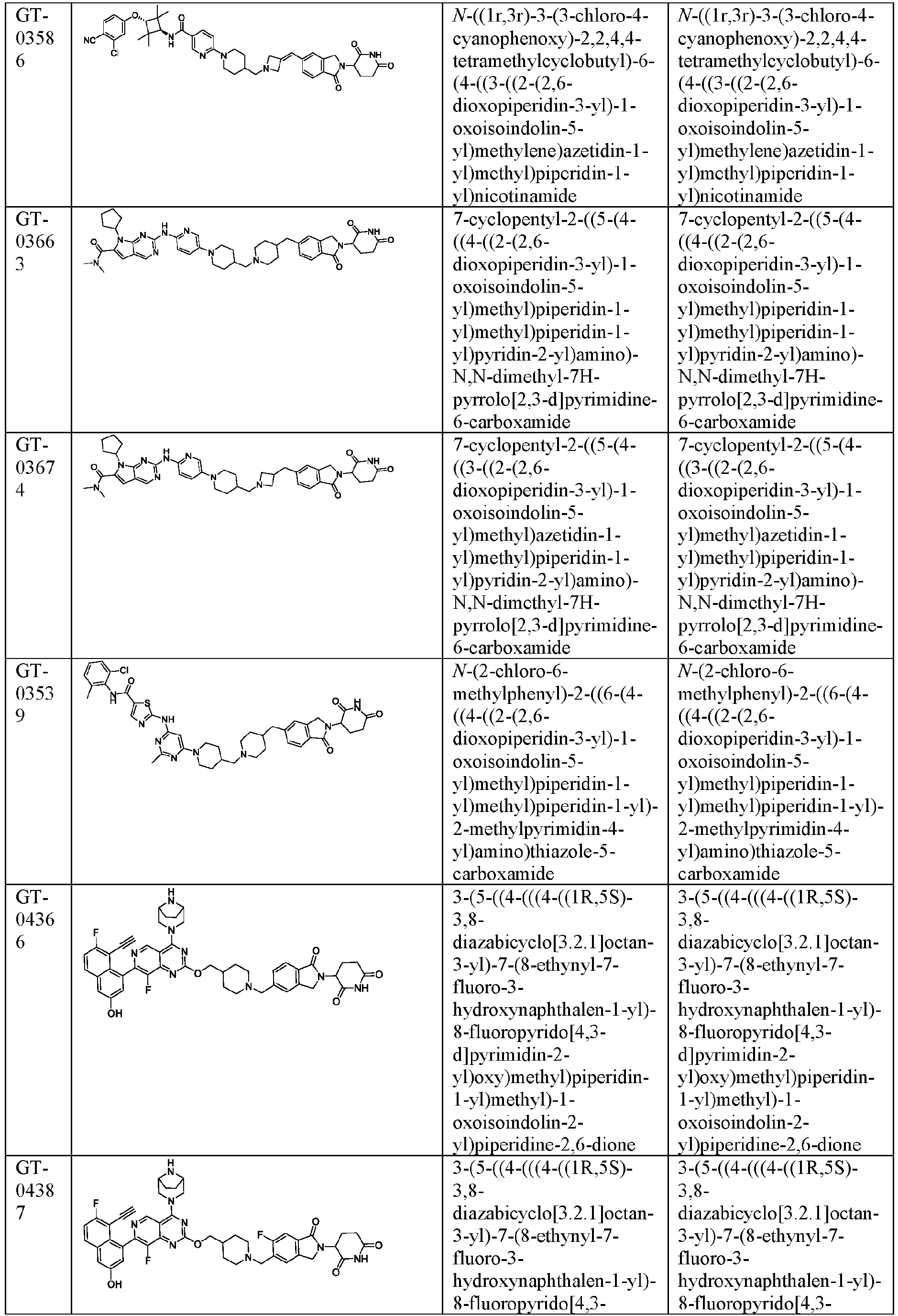

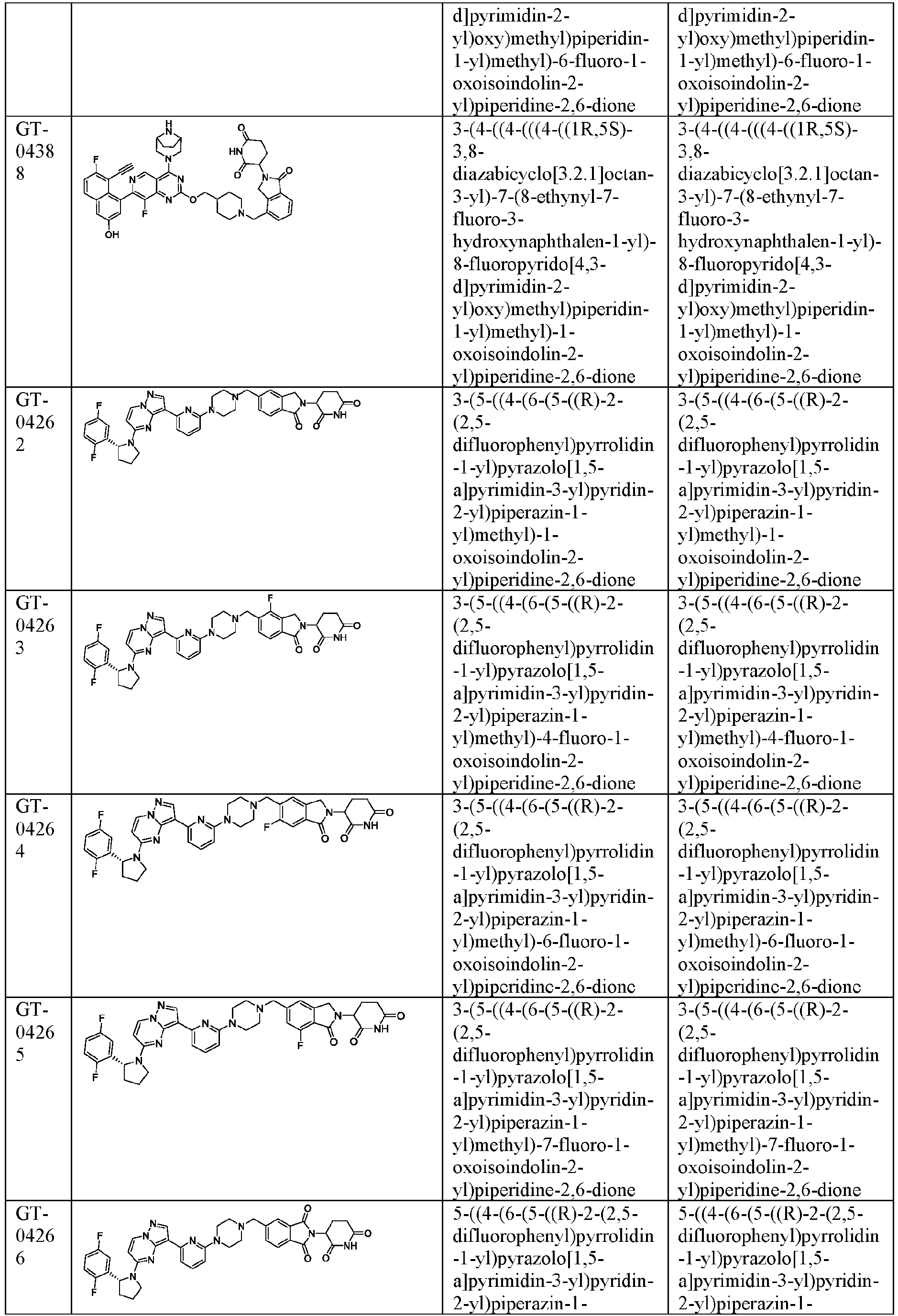

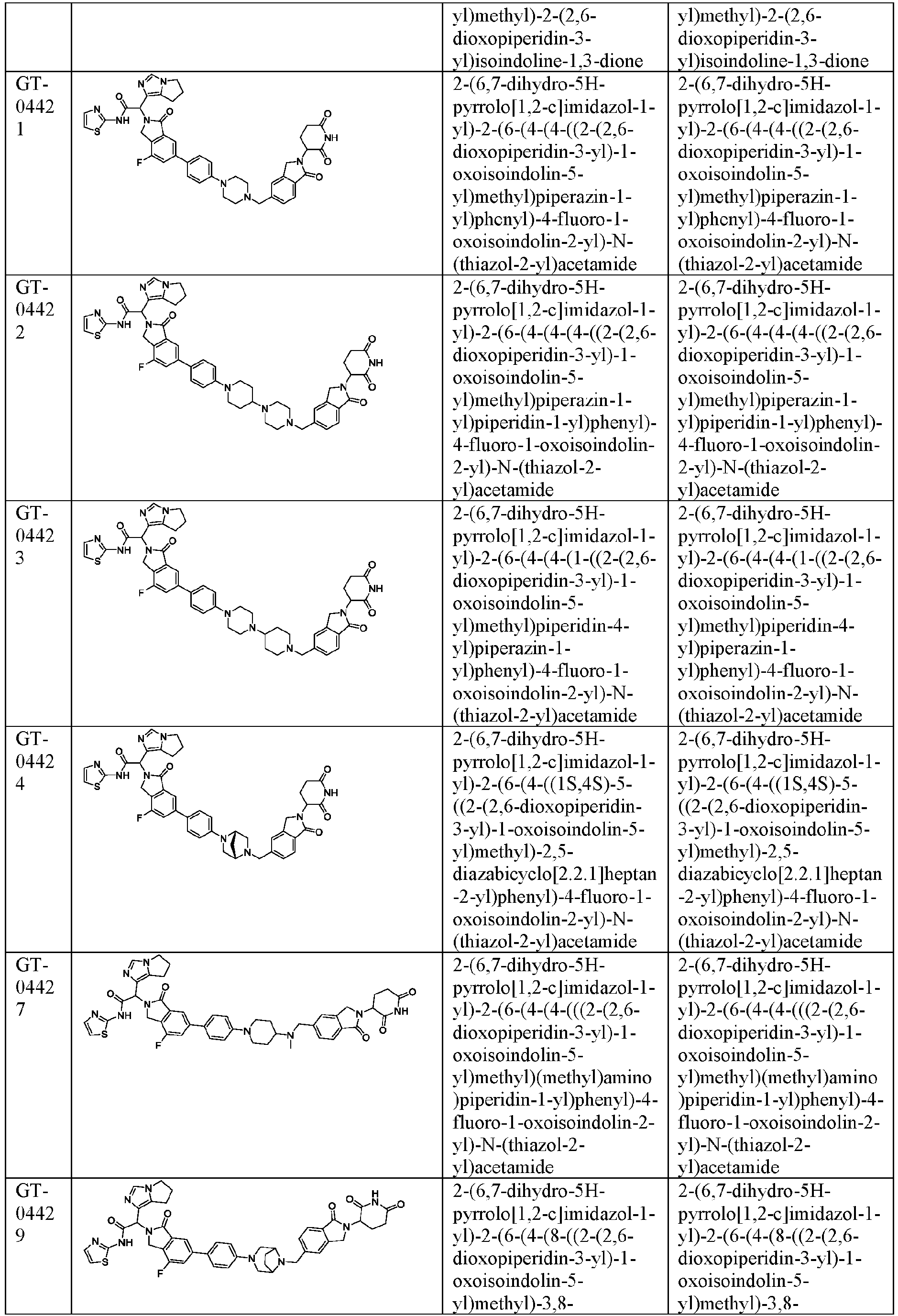

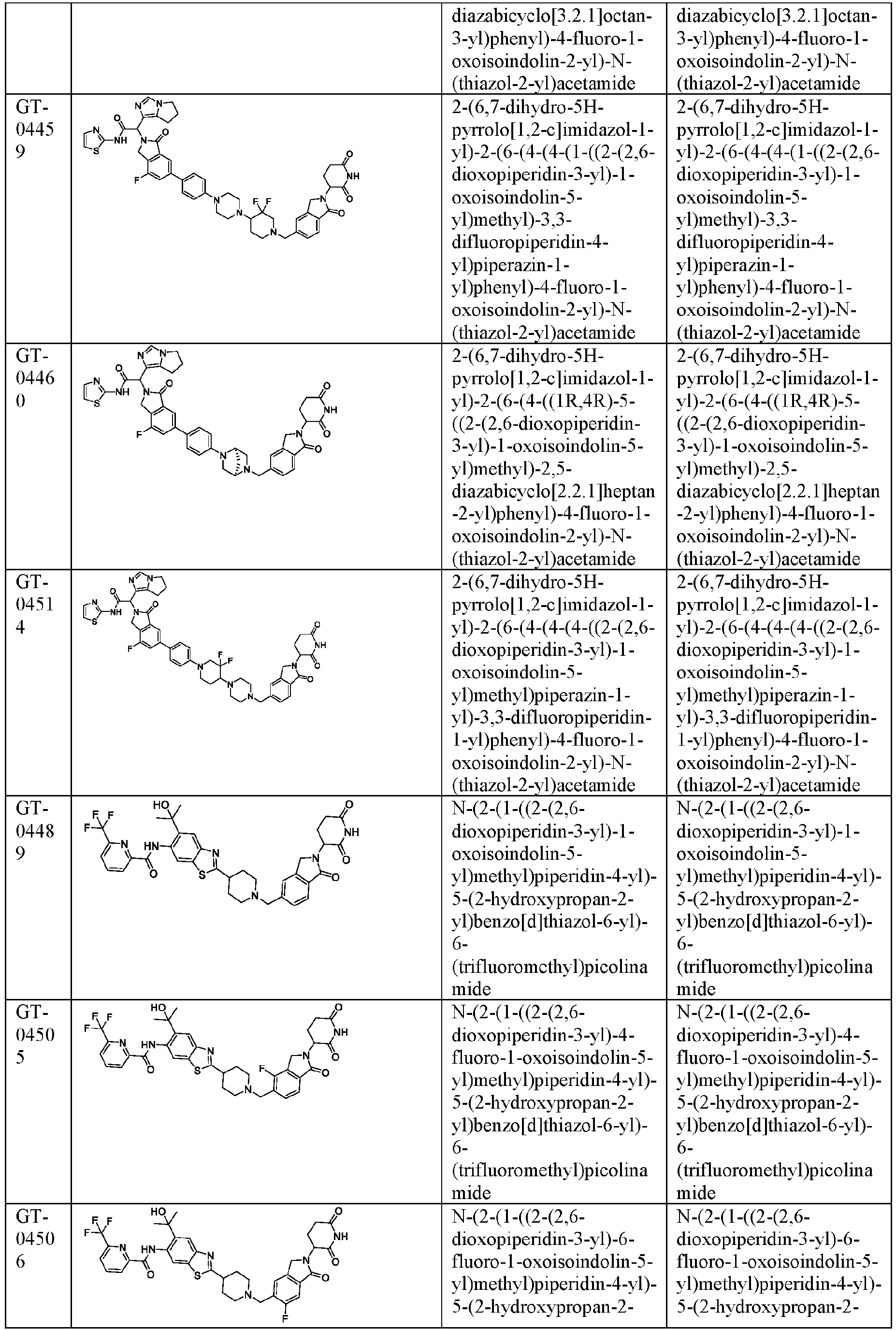

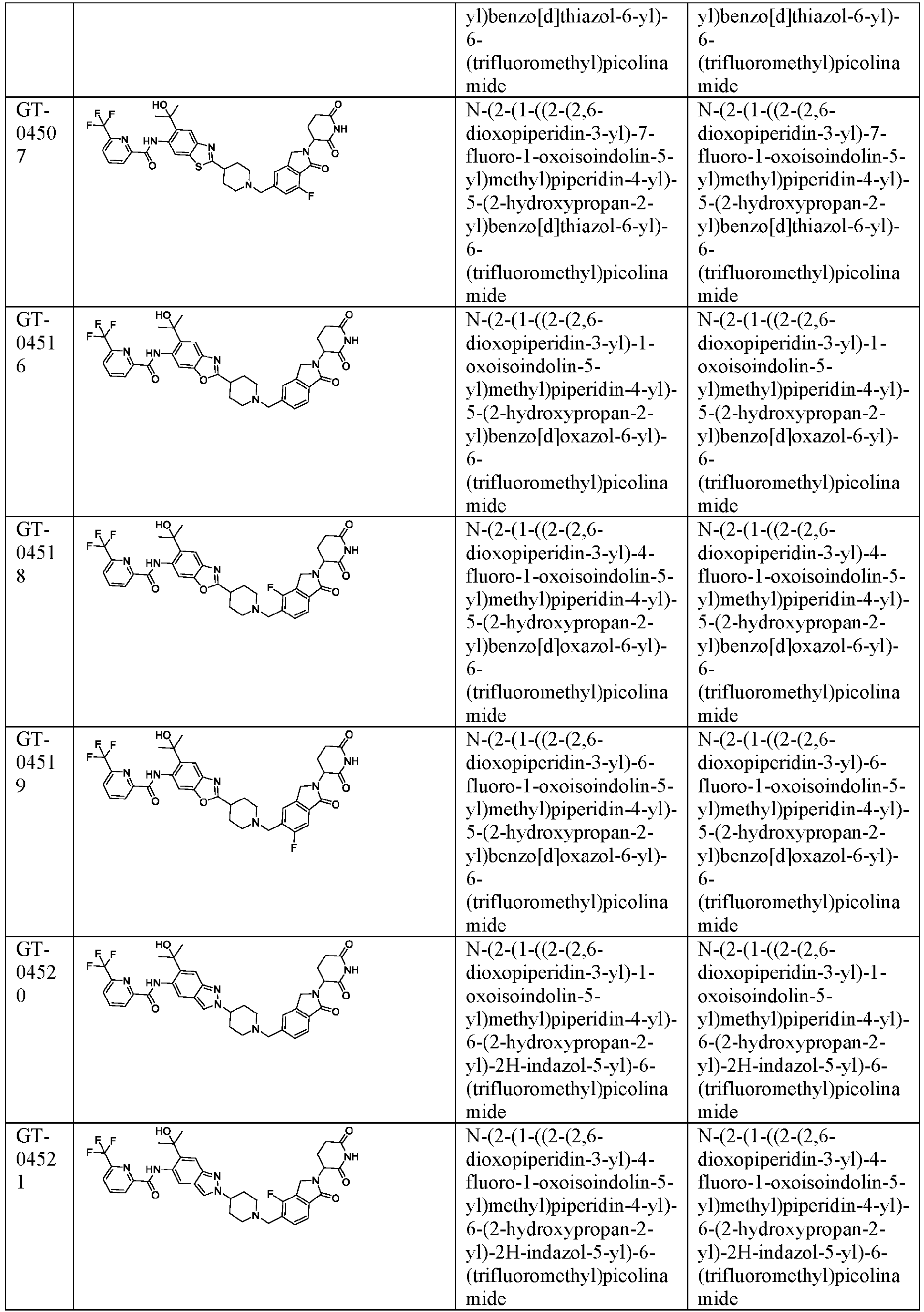

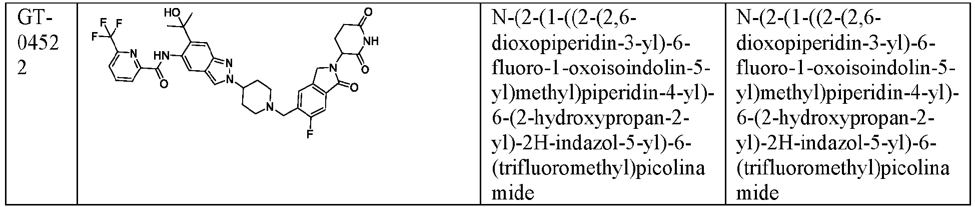

### II. Other Forms of Compounds (including salts, enantiomers, stereoisomers, solvates, isotopically enriched analogs, prodrugs, or polymorphs of compounds)

The compounds of the present disclosure have the structures of any one of Formula (I), Formula (II), Formula (II-1), Formula (II-2), Formula (II-3), Formula (II-4), Formula (II-5), Formula (II-6), Formula (II-7), Formula (II-8), Formula (II-9), Formula (11-10), Formula (II-11), Formula (11-12), Formula (II-13), Formula (11-14), Formula (11-15), Formula (11-16), Formula (11-17), Formula (II-18), Formula (II-19), Formula (II-20), Formula (II-21), Formula (II-22), Formula (II-23), Formula (II-24), Formula (II-25), Formula (II-26), Formula (II-27), Formula (II-28), Formula (II-29), Formula (II-30), Formula (II-31), Formula (II-32), Formula (II-33), Formula (II-34), Formula (II-35), Formula (II-36), Formula (II-37), Formula (11-38), Formula (II-39), Formula (II-40), Formula (II-41), Formula (II-42), Formula (II-43), Formula (II-44), Formula (II-45), Formula (II-46), Formula (II-47), Formula (II-48), Formula (II-49), Formula (II-50), Formula (II-51), Formula (II-52), Formula (II-53), Formula (II-54), Formula (II-55), Formula (II-56), Formula (II-57) , Formula (II-58), Formula (II-59), Formula (II-60), Formula (II-61), Formula (II-62), Formula (II-63), Formula (II-64), Formula (II-65) or Formula (II-66). Unless otherwise specified, all references to the compounds of the present disclosure also include compounds of any one of Formula (I), Formula (II), Formula (II-1), Formula (II-2), Formula (II-3), Formula (II-4), Formula (II-5), Formula (II-6), Formula (II-7), Formula (II-8), Formula (II-9), Formula (II-10), Formula (II-11), Formula (II-12), Formula (II-13), Formula (II-14), Formula (II-15), Formula (II-16), Formula (II-17), Formula (II-18), Formula (II-19), Formula (II-20), Formula (II-21), Formula (II-22), Formula (II-23), Formula (II-24), Formula (II-25), Formula (II-26), Formula (II-27), Formula (II-28), Formula (II-29), Formula (II-30), Formula (II-31), Formula (II-32), Formula (II-33), Formula (II-34), Formula (II-35), Formula (II-36), Formula (II-37), Formula (II-38), Formula (II-39), Formula (II-40), Formula (II-41), Formula (II-42), Formula (II-43), Formula (II-44), Formula (II-45), Formula (II-46), Formula (II-47), Formula (II-48), Formula (II-49), Formula (II-50), Formula (II-51), Formula (II-52), Formula (II-53), Formula (II-54), Formula (II-55), Formula (II-56), Formula (II-57) , Formula (II-58), Formula (II-59), Formula (II-60), Formula (II-61), Formula (II-62), Formula (II-63), Formula (II-64), Formula (II-65) or Formula (II-66) and specific compounds within the scope of these general formulae.

It should be recognized that compounds of the present disclosure (including Formula (I), Formula (II), Formula (II-1), Formula (II-2), Formula (II-3), Formula (II-4), Formula (II-5), Formula (II-6), Formula (II-7), Formula (II-8), Formula (II-9), Formula (11-10), Formula (II-11), Formula (II-12), Formula (11-13), Formula (11-14), Formula (11-15), Formula (11-16), Formula (11-17), Formula (II-18), Formula (II-19), Formula (II-20), Formula (II-21), Formula (II-22), Formula (II-23), Formula (II-24), Formula (II-25), Formula (II-26), Formula (II-27), Formula (II-28), Formula (II-29), Formula (II-30), Formula (II-31), Formula (II-32), Formula (II-33), Formula (II-34), Formula (II-35), Formula (II-36), Formula (II-37), Formula (II-38), Formula (II-39), Formula (II-40), Formula (II-41), Formula (II-42), Formula (II-43), Formula (II-44), Formula (II-45), Formula (II-46), Formula (II-47), Formula (II-48), Formula (II-49), Formula (II-50), Formula (II-51), Formula (II-52), Formula (II-53), Formula (II-54), Formula (II-55), Formula (II-56), Formula (II-57), Formula (II-58) , Formula (II-59), Formula (II-60), Formula (II-61), Formula (II-62), Formula (II-63), Formula (II-64), Formula (II-65) or Formula (II-66)) may have a stereo-configuration and thus can exist in more than one stereoisomeric form. The present disclosure also relates to optically enriched compounds having a stereo-configuration, e.g., greater than about 90% enantiomeric/diastereomeric excess ("ee"), such as about 95% ee or 97% ee, or greater than 99% ee, and mixtures thereof, including racemic mixtures. As used herein, "optically enriched" means that a mixture of enantiomers consists of a significantly greater proportion of one enantiomer, and can be described by enantiomeric excess (ee%). Purification of isomers and separation of mixtures of isomers can be accomplished by standard techniques known in the art (e.g., column chromatography, preparative TLC, preparative HPLC, asymmetric synthesis (e.g., by using chiral intermediates) and/or or chiral resolution, etc.).

In some embodiments, polymorph forms or salts of the compounds of the present disclosure are also provided. Salts of the compounds of the present disclosure can be pharmaceutically acceptable salts including, but not limited to, hydrohalides (including hydrochlorides and hydrobromates), sulfates, citrates, maleates, sulfonates, citrates, lactates, L-tartrates, fumarates, L-malates, phosphates, dihydrophosphates, pyrophosphates, metaphosphates, oxalates, malonates, benzoates, mandelates, succinates, trifluoroacetates, hydroxyacetates, or p-toluenesulfonates, etc. The compounds of the present disclosure can exist as non-solvated or solvated forms in pharmaceutically acceptable solvents such as water, ethanol, and the like. In some embodiments, compounds of the present disclosure can be prepared as prodrugs or precursor drugs. Prodrugs can be converted into parent drugs in the body to play their role. In some embodiments, isotopically-labeled compounds of the present disclosure are also provided, examples of which include deuterium (D or ²H).

### III. Compositions/Formulations

In some embodiments, the present disclosure provides a pharmaceutical composition comprising as an active ingredient the compound of the present disclosure or a pharmaceutically acceptable salt, solvate, isotopically enriched analog, polymorph, prodrug, stereoisomer (including enantiomer), or mixture of stereoisomers thereof, and at least one pharmaceutically acceptable carrier.

In some embodiments, pharmaceutically acceptable carriers include, but are not limited to, fillers, stabilizers, dispersants, suspending agents, diluents, excipients, thickeners, colorants, solvents, or encapsulating materials. Each carrier must be "acceptable" in the sense of being compatible with the other ingredients of the formulation (including the compounds useful in the present disclosure) and not injurious to the patient. Some examples of materials that can be used as pharmaceutically acceptable carriers include: sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; polyethylene oxide, polyvinylpyrrolidone, polyacrylamide, poloxamer; and other common non-toxic compatible substances used in pharmaceutical formulations.

The pharmaceutical composition of the present disclosure can further comprise at least one second therapeutic agent, e.g., an anticancer agent. The second therapeutic agent may be used in combination with the compounds of Formula (I) or Formula (II) described in the present disclosure to treat the diseases or disorders as disclosed herein. The second therapeutic agent includes, but is not limited to, chemotherapeutic agents, immunotherapeutic agents, gene therapy agents, and the like.

The pharmaceutical composition of the present disclosure comprising, as an active ingredient, the compounds of Formula (I) or Formula (II) of the present disclosure or a pharmaceutically acceptable salt thereof can be formulated into any suitable formulations such as sprays, patches, tablets (such as conventional tablets, dispersible tablets, orally disintegrating tablets), capsules (such as soft capsules, hard capsules, enteric-coated capsules), dragees, troches, powders, granules, powder injections, suppositories, or liquid formulations (such as suspensions (e.g., aqueous or oily suspensions), solutions, emulsions, or syrups), or conventional injection dosage forms such as injectable solutions (e.g., sterile injectable solutions formulated according to methods known in the art using water, Ringer's solution, or isotonic sodium chloride solution or the like as a vehicle or solvent) or lyophilized injectable formulation and the like, depending upon a suitable route of administration (including, but not limited to, nasal administration, inhalation administration, topical administration, oral administration, oral mucosal administration, rectal administration, intrapleural administration, intraperitoneal administration, vaginal administration, intramuscular administration, subcutaneous administration, transdermal administration, epidural administration, intrathecal administration, and intravenous administration). Those skilled in the art can also formulate the compounds of Formula (I) or Formula (II) of the present disclosure into conventional, dispersible, chewable, orally disintegrating or rapidly dissolving formulations, or sustained-release capsules or controlled-release capsules as needed.

The compounds of Formula (I) of the present disclosure, as an active ingredient, is contained in the pharmaceutically acceptable carrier or diluent in an amount sufficient to deliver to a subject a therapeutically effective amount for the indication to be treated, without causing serious toxic effects in the subject treated. A dosage of the active compound for all diseases or disorders mentioned herein ranges, for example, from about 5ng/kg to 500mg/kg, from about 10ng/kg to 300mg/kg per day, such as from 0.1 to 100 mg/kg, or from 0.5 to about 25mg per kilogram body weight of the subject per day.

The compounds of Formula (I) of the present disclosure or pharmaceutically acceptable salts thereof can be conveniently administered in any suitable unit dosage form. Suitable unit dosage form specifications include, but are not limited to, less than 1mg, 1mg to 3000mg, 5mg to 1000mg, for example, 5 to 500mg, 25 to 250mg of active ingredient per unit dosage form.

The compounds of Formula (II) of the present disclosure, as an active ingredient, is contained in the pharmaceutically acceptable carrier or diluent in an amount sufficient to deliver to a subject a therapeutically effective amount for the indication to be treated, without causing serious toxic effects in the subject treated. A dosage of the active compound for all diseases or disorders mentioned herein ranges, for example, from about 5ng/kg to 500mg/kg, from about 10ng/kg to 300mg/kg per day, such as from 0.1 to 100 mg/kg, or from 0.5 to about 25mg per kilogram body weight of the subject per day.

The compounds of Formula (II) of the present disclosure or pharmaceutically acceptable salts thereof can be conveniently administered in any suitable unit dosage form. Suitable unit dosage form specifications include, but are not limited to, less than 1mg, 1mg to 3000mg, 5mg to 1000mg, for example, 5 to 500mg, 25 to 250mg of active ingredient per unit dosage form.

### IV. Kits/Packaged Products

The compounds of Formula (I) or Formula (II) or a pharmaceutically acceptable salt, solvate, isotopically enriched analog, polymorph, prodrug, stereoisomer (including enantiomer), or mixture of stereoisomers thereof is used as a medicament. The medicament of the present disclosure or the pharmaceutical composition of the present disclosure may be presented in a kit/packaged product. The kit/packaged product may include a package or container including, but not limited to, ampoules, blister packs, pharmaceutical plastic bottles, vials, pharmaceutical glass bottles, containers, syringes, laminated flexible packaging, co-extruded film infusion containers, test tubes and dispensing devices, and the like. The kit/packaged product may contain instructions for use of the product.

### V. Methods and Uses

The compounds of Formula (I) or a pharmaceutically acceptable salt, solvate, isotopically enriched analog, polymorph, prodrug, stereoisomer (including enantiomer), or mixture of stereoisomers thereof can be used as a medicament. Especially the compounds of Formula (I) or a pharmaceutically acceptable salt, solvate, isotopically enriched analog, polymorph, prodrug, stereoisomer (including enantiomer), or mixture of stereoisomers thereof can be used for the manufacture of a medicament for the prevention and/or treatment of a cereblon protein-mediated disease or disorder.

The present disclosure provides a method for preventing and/or treating a cereblon protein-mediated disease or disorder in a subject, comprising administering to the subject a therapeutically effective amount of the compounds of Formula (I) of the present disclosure or a pharmaceutically acceptable salt thereof, or the pharmaceutical composition of the present disclosure comprising as an active ingredient the compounds of Formula (I) of the present disclosure or a pharmaceutically acceptable salt thereof. In some embodiments, the cereblon protein-mediated disease or disorder comprises: tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, congestive heart failure, myocardial infarction, Unverricht's syndrome, Richter syndrome (RS), acute liver failure, or diabetes. In some embodiments, the cereblon protein-mediated disease or disorder includes, but is not limited to, myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer; inflammatory myofibroblastoma; colorectal cancer; intestinal cancer; brain glioma; astroblastoma; glioma; peripheral neuroepithelioma; ovarian cancer; bronchial cancer; prostate cancer; breast cancer, including triple negative breast cancer, sporadic breast cancer and patients with Cowden syndrome; pancreatic cancer; central nervous system tumor; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout, etc; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; congestive heart failure; myocardial infarction; multiple organ dysfunction caused by cachexia and septic shock; and acute liver failure.

In the method for preventing and/or treating a cereblon protein-mediated disease or disorder in a subject, the compound of Formula (I) of the present disclosure or the pharmaceutical composition comprising as an active ingredient the compound of Formula (I) of the present disclosure of the present disclosure is administered to the subject through at least one mode of administration selected from the group consisting of nasal administration, inhalation administration, topical administration, oral administration, oral mucosal administration, rectal administration, pleural cavity administration, peritoneal administration, vaginal administration, intramuscular administration, subcutaneous, transdermal, epidural, intrathecal, and intravenous administration.

The compound of Formula (II) or a pharmaceutically acceptable salt, solvate, isotopically enriched analog, polymorph, prodrug, stereoisomer (including enantiomer), or mixture of stereoisomers thereof can be used as a medicament. Especially the compound of Formula (II) or a pharmaceutically acceptable salt, solvate, isotopically enriched analog, polymorph, prodrug, stereoisomer (including enantiomer), or mixture of stereoisomers thereof can be used for the manufacture of a medicament for the prevention and/or treatment of a disease or disorder selected from the group consisting of tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, congestive heart failure, myocardial infarction, Unverricht's syndrome, Richter syndrome (RS), acute liver failure, diabetes, Kennedy disease, seborrheic alopecia, hirsutism, skin disease, cardiovascular disease, dysfunctional uterine bleeding, anemia, pediatric aplastic anemia, endometriosis, transplant rejection, polycystic ovary syndrome, and thyroid disease. In some embodiments, the disease or disorder includes, but is not limited to, myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma; inflammatory myofibroblastoma; colorectal cancer; brain glioma; astroblastoma; ovarian cancer; bronchial cancer; prostate cancer; breast cancer, including triple negative breast cancer, sporadic breast cancer and patients with Cowden syndrome; pancreatic cancer; central nervous system tumor; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout, etc; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; multiple organ dysfunction caused by cachexia and septic shock; acute liver failure; dysfunctional uterine bleeding; anemia; pediatric aplastic anemia; endometriosis; polycystic ovary syndrome; thyroid disease; cardiovascular diseases (e.g., coronary heart disease, congestive heart failure, myocardial infarction, atherosclerosis); skin diseases (e.g., acne); Kennedy disease; seborrheic alopecia; and hirsutism.

The present disclosure provides a method for preventing and/or treating a disease or disorder in a subject, comprising administering to the subject a therapeutically effective amount of the compound of Formula (II) of the present disclosure or a pharmaceutically acceptable salt thereof, or the pharmaceutical composition of the present disclosure comprising as an active ingredient the compound of Formula (II) of the present disclosure or a pharmaceutically acceptable salt thereof, wherein the disease or disorder includes, but is not limited to, tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, congestive heart failure, myocardial infarction, Unverricht's syndrome, Richter syndrome (RS), acute liver failure, diabetes, Kennedy disease, seborrheic alopecia, hirsutism, skin disease, cardiovascular disease, dysfunctional uterine bleeding, anemia, pediatric aplastic anemia, endometriosis, transplant rejection, polycystic ovary syndrome, and thyroid disease. In some embodiments, the disease or disorder includes, but is not limited to, myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma; inflammatory myofibroblastoma; colorectal cancer; brain glioma; astroblastoma; ovarian cancer; bronchial cancer; prostate cancer; breast cancer, including triple negative breast cancer, sporadic breast cancer and patients with Cowden syndrome; pancreatic cancer; central nervous system tumor; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout, etc; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; multiple organ dysfunction caused by cachexia and septic shock; acute liver failure; dysfunctional uterine bleeding; anemia; pediatric aplastic anemia; endometriosis; polycystic ovary syndrome; thyroid disease; cardiovascular diseases (e.g., coronary heart disease, congestive heart failure, myocardial infarction, atherosclerosis); skin diseases (e.g., acne); Kennedy disease; seborrheic alopecia; and hirsutism.

The term "treatment" or "treating" refers to the administration of the compound of Formula (I) or Formula (II) or a pharmaceutically acceptable salt thereof according to the present disclosure, or the pharmaceutical composition containing, as an active ingredient, the compound of Formula (I) or Formula (II) or a pharmaceutically acceptable salt thereof, to a subject to mitigate (alleviate) undesirable diseases or conditions, such as the development of a cancer or tumor. The beneficial or desired clinical results of the present disclosure include, but are not limited to: alleviating symptoms, reducing the severity of the disease, stabilizing the state of the disease, slowing down or delaying the progression of the disease, improving or alleviating the condition, and alleviating the disease.

A "therapeutic effective amount" of the compound of the present disclosure depends on a variety of factors, including the activity of the specific compound used, the metabolic stability of the compound and the duration of its action, the age, sex and weight of the patient, the patient's current medical condition, the route and duration of administration, the excretion rate, the combined administration of additional drugs, and the progression of the diseases or conditions of the patient being treated. Those skilled in the art will be able to determine appropriate dosages based on these and other factors.

It is to be understood that the choice of using one or more active compounds and/or compositions and their dosage depends on the basic situations of the individual (which should generally render the individual situation to achieve the best effect). Dosing and dosing regimens should be within the ability of those skilled in the art, and the appropriate dosage depends on many factors including the knowledge and ability of the physicians, veterinarians or researchers (see e.g., Jun Li (chief editor), "Clinical Pharmacology", 4th edition, People's Public Health Press, 2008).

As used herein, the term "patient" or "subject" to be treated refers to animal, for example mammal, including but not limited to primate (such as human being), cow, sheep, goat, horse, dog, cat, rabbit, guinea pig, rat, mice, etc.

The compound of Formula (I) or a pharmaceutically acceptable salt, solvate, isotopically enriched analog, polymorph, prodrug, stereoisomer (including enantiomer), or mixture of stereoisomers thereof of the present disclosure can be used to prepare the compound of Formula (II) of the present disclosure. The compound of Formula (II) of the present disclosure can be used to treat and/or prevent a disease or disorder selected from the group consisting of tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, congestive heart failure, myocardial infarction, Unverricht's syndrome, Richter syndrome (RS), acute liver failure, diabetes, Kennedy disease, seborrheic alopecia, hirsutism, skin disease, cardiovascular disease, dysfunctional uterine bleeding, anemia, pediatric aplastic anemia, endometriosis, transplant rejection, polycystic ovary syndrome, and thyroid disease.

### VI. Definitions

Unless otherwise specified, the following words, phrases and symbols used herein generally have the meanings as described below.

In general, the nomenclature used herein (including the IUPAC nomenclature) and the laboratory procedures described below (including those used in cell culture, organic chemistry, analytical chemistry, and pharmacology, etc.) are those well-known and commonly used in the art. Unless otherwise defined, all scientific and technical terms used herein in connection with the present disclosure described herein have the same meaning as commonly understood by one skill in the art. In addition, the use of the word "a" or "an" when used in conjunction with the term "comprising" or a noun in the claims and/or the specification may mean "one", but it is also consistent with the meaning of "one or more", "at least one", and "one or more than one". Similarly, the terms "another" or "other" can mean at least a second or more.

It should be understood that whenever the term "comprise" or "include" is used herein to describe various aspects, other similar aspects described by "consisting of" and/or "consisting essentially of" are also provided.

As used herein, the term "about" used alone or in combination refers to approximately, roughly, nearly, or around. When the term "about" is used in conjunction with a numerical range, it modifies that range by extending the boundaries above and below the stated numerical value. In general, the term "about" can modify a numerical value above and below the stated value by an upward or downward (increasing or decreasing) variation, e.g., 10%, 5%, 2%, or 1%.

As used herein, the wording "...represents a bond" used alone or in combination means that the referenced group is a bond linker (that is, the referenced group is absent). For example, the wording "R_{f} represents a bond" means that R_{f} is a bond linker. In other words, when R_{f} represents a bond, the group PBM in the structure of Formula (II) is directly connected to LIN in the structure of Formula (II). For example, the wording "R¹⁷ represents a bond" means that R¹⁷ is a bond linker. In other words, when R¹⁷ represents a bond, the carbon atom on the ring in the structure of Formula (PBM-9) is directly connected to R_{f} of the compound of Formula (II).

In this document, the term "optionally substituted" used alone or in combination means that the referenced group may be unsubstituted or substituted with one or more substituents as defined here. Herein, the wording "optionally substituted by..." and "unsubstituted or substituted" can be used interchangeably. The term "substituted" generally indicates that one or more hydrogens in the referenced structure are replaced by the same or different specific substituents. The number of substituents is not theoretically limited in any way, or is automatically limited by the size of the building units (i.e., the total number of replaceable hydrogen atoms in the building units), or as clearly defined herein.

As used herein, the term "inserted" of the expression "one or more groups R_{c} and/or one or more groups R_{d} and/or any combination of one or more groups R_{c} and R_{d} are inserted into the backbone carbon chain of the linear or branched C₂₋₃₀ alkylene group", used alone or in combination, has a known definition in the art, which can mean that carbon-carbon bond between one or more pairs of adjacent carbon atoms in the referenced backbone carbon chain is interrupted by the groups R_{c}, R_{d}, or a combination of R_{c} and R_{d}. Herein, examples of the above-mentioned expression "one or more groups...... are inserted into" may include, but are not limited to, that one or more (1-30, 1-20, or 1-15, 1-10, 1-9, 1-8, 1-7, 1-6, 1-5, 1-4, 1-3, or 1-2, or 1) groups R_{c} as defined herein and/or one or more (1-30, 1-20, or 1-15, 1-10, 1-9, 1-8, 1-7, 1-6, 1-5, 1-4, 1-3, or 1-2, or 1) groups R_{d} as defined herein and/or one or more (1-30, 1-20, or 1-15, 1-10, 1-9, 1-8, 1-7, 1-6, 1-5, 1-4, 1-3, or 1-2 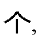 , or 1) combinations of R_{c} with R_{d} are inserted into the backbone carbon chain, and the resulting backbone chain group conforms to the covalent bond theory. For example, the expression "one or more groups R_{c} and/or one or more groups R_{d} and/or any combination of one or more groups R_{c} and R_{d} are inserted into the backbone carbon chain of the linear or branched C₂₋₃₀ alkylene group" can refer to that one or more (e.g., 1-30, 1-20, 1-15, 1-10, 1-8, 1-7, 1-6, 1-5, 1 -4, 1-3, 1-2, or 1) groups R_{c} and/or R_{d} and/or one or more combinations of R_{c} with R_{d} are inserted between one or more pairs of any two adjacent carbon atoms of the backbone carbon chain of the linear or branched C₂₋₃₀ alkylene group, resulting in the formation of a backbone chain group containing one or more (e.g., 1-30, 1-20, 1-15, 1-10, 1-8, 1-7, 1-6, 1-5, 1 -4, 1-3, 1-2, or 1) fragments "-CH₂-R_{c}-CH₂-" and/or one or more (e.g., 1-30, 1-20, 1-15, 1-10, 1-8, 1-7, 1-6, 1-5, 1-4, 1-3, 1-2, or 1) fragments "-CH₂-R_{d}-CH₂-" and/or one or more (e.g., 1-30, 1-20, 1-15, 1-10, 1-8, 1-7, 1-6, 1-5, 1-4, 1-3, 1-2, or 1) fragments "-CH₂-R_{c}-R_{d}-CH₂-", where each R_{c} are the same or different, each R_{d} are the same or different, and are as defined herein.

Herein, it should be understood that the expression "one or more groups R_{c} and/or one or more groups R_{d} or any combination of one or more groups R_{c} and R_{d} are optionally inserted into the backbone carbon chain of the linear or branched C₂₋₃₀ alkylene group" includes embodiments where "one or more groups R_{c} and/or one or more groups R_{d} or any combination of one or more groups R_{c} and R_{d} are inserted into the backbone carbon chain of the linear or branched C₂₋₃₀ alkylene group", as well as embodiments where "no one or more groups R_{c} and/or one or more groups R_{f} or any combination of one or more groups Rₑ and R_{f} are inserted into the backbone carbon chain of the linear or branched C₂₋₃₀ alkylene group".

Herein, a bond interrupted by a wavy line shows the point of attachment of the depicted group to the rest of the molecule. For example, the group of Formula (ULM) depicted below 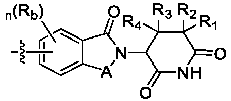 shows the point of attachment of benzene ring in said group to LIN of the compound of Formula (II). For example, the group of LIN depicted below: 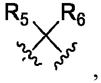 shows the point of attachment of the methylene of the said group to R_{f} and ULM, respectively.

As used herein, the expression "a hydrogen atom of one or more CH₂ of the linear or branched C_{x-y} alkylene is replaced by...", used alone or in combination, means that a hydrogen atom of any one or more CH₂ of the linear or branched C_{x-y} alkylene is replaced by a substituent(s) as defined herein. Herein, the term "one or more" of "a hydrogen atom of one or more CH₂ of groups -CH₂-, -(CH₂)₂-, - (CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, - (CH₂)₁₃-, -(CH₂)₁₄-, -(CH₂)₁₅-, -(CH₂)₁₆-, -(CH₂)₁₇-, -(CH₂)₁₈-, -(CH₂)₁₉-, -(CH₂)₂₀-, -(CH₂)₂₁-, -(CH₂)₂₂-, -(CH₂)₂₅-, or -(CH₂)₃₀-" may refer to part or all hydrogen atoms of each referenced alkylene group, including but not limited to 1-60 hydrogens. In some embodiments, the expression "a hydrogen atom of one or more CH₂" may refer to part or all of the hydrogen atoms of the referenced alkylene group, including but not limited to 1-30, such as 1-25, 1-20, 1-15, 1-10, 1-5, 1-4, 1-3, 1-2 or 1 hydrogen atoms. In some embodiments, the expression "a hydrogen atom of one or more CH₂" may include 1-3 of the plurality of hydrogen atoms of the referenced alkylene group. The number of hydrogens to be replaced is in principle not limited in any way, or is automatically limited by the size of the building unit.

As used herein, the term "oxo" or "oxo group" refers to =O.

As used herein, the term "nicotinoyl" refers to

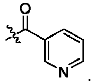

As used herein, the term "halogen atom" or "halogen", used alone or in combination, refers to fluorine, chlorine, bromine, or iodine.

As used herein, the term "alkyl", used alone or in combination, refers to a linear or branched alkyl group. The term "Cₓ-C_{y} alkyl" or "C_{x-y} alkyl" (x and y each being an integer) refers to a linear or branched alkyl group containing from x to y carbon atoms. The term "C₁₋₁₀ alkyl" used alone or in combination in the present disclosure refers to a linear or branched alkyl group containing from 1 to 10 carbon atoms. Non-limiting examples of the C₁₋₁₀ alkyl of the present disclosure may include a C₁₋₉ alkyl, C₁₋₈ alkyl, C₂₋₈ alkyl, C₁₋₇ alkyl, C₁₋₆ alkyl, C₁₋₅ alkyl, C₁₋₄ alkyl, and C₁₋₃ alkyl. Representative examples include methyl, ethyl, n-propyl, isopropyl, n-butyl, isobutyl, sec-butyl, tert-butyl, pentyl, isopentyl, neopentyl, tert-pentyl, hexyl, heptyl, octyl, nonyl, and decyl. The term "C₁₋₃ alkyl" or "C₁-C₃ alkyl" in the present disclosure refers to an alkyl group containing from 1 to 3 carbon atoms, and representative examples thereof include methyl, ethyl, n-propyl, and isopropyl. In the present disclosure, the "alkyl" is optionally substituted with one or more substituents optionally selected from the group consisting of halogen, hydroxyl, cyano, C₁₋₃ alkyl, C₁₋₃ alkoxy, trifluoromethyl, heterocyclyl, or a combination thereof.

As used herein, the term "halogenated alkyl" or "haloalkyl", used alone or in combination, refers to a linear or branched alkyl group substituted with one or more halogens, wherein one or more hydrogen atom(s) of the alkyl group are replaced with one or more halogens. The term "halogenated Cₓ₋c_{y} alkyl" or "halogenated C_{x-y} alkyl" (x and y are each an integer) refers to a linear or branched alkyl containing from x to y carbon atoms substituted with one or more halogens. The term "halogenated C₁₋₁₀ alkyl" used alone or in combination in the present invention refers to a linear or branched alkyl group containing from 1 to 10 carbon atoms substituted with one or more halogens. Examples of the halogenated C₁₋₁₀ alkyl group of the present disclosure include halogenated C₁₋₉ alkyl group, e.g., halogenated C₁₋₈ alkyl group, halogenated C₂₋₈ alkyl group, halogenated C₁₋₇ alkyl group, halogenated C₁₋₆ alkyl, halogenated C₁₋₅ alkyl, or halogenated C₁₋₄ alkyl. Representative examples include halomethyl, haloethyl, halo-n-propyl, haloisopropyl, halo-n-butyl, haloisobutyl, halo-sec-butyl, halo-tert-butyl, halopentyl, haloisoamyl, haloneopentyl, halo-tert-pentyl, halohexyl, haloheptyl, halooctyl, halononyl, and halodecyl. The term "halo-C₁₋₃ alkyl" or "halo-C₁₋C₃ alkyl" of the present disclosure refers to an alkyl group containing from 1 to 3 carbon atoms substituted with one or more halogens, and its representative examples include halomethyl, haloethyl, halo-n-propyl and haloisopropyl.

As used herein, the term "deuterated alkyl", used alone or in combination, refers to a linear or branched alkyl group substituted with one or more deuterium atoms, wherein one or more hydrogen atom(s) of the alkyl group are replaced with one or more deuterium atoms. The term "deuterated Cₓ₋c_{y} alkyl" or "deuterated C_{x-y} alkyl" (x and y are each an integer) refers to a linear or branched alkyl containing from x to y carbon atoms substituted with one or more deuterium atoms. The term "deuterated C₁₋₁₀ alkyl" used alone or in combination in the present invention refers to a linear or branched alkyl group containing from 1 to 10 carbon atoms substituted with one or more deuterium atoms. Examples of the deuterated C₁₋₁₀ alkyl group of the present disclosure include deuterated C₁₋₉ alkyl group, e.g., deuterated C₁₋₈ alkyl group, deuterated C₂₋₈ alkyl group, deuterated C₁₋₇ alkyl group, deuterated C₁₋₆ alkyl, deuterated C₁₋₅ alkyl, or deuterated C₁₋₄ alkyl. Representative examples include perdeuterated methyl (CD₃), perdeuterated ethyl (CD₃CD₂), perdeuterated n-propyl, perdeuterated isopropyl, perdeuterated n-butyl, perdeuterated isobutyl, perdeuterated sec-butyl, perdeuterated tert-butyl, perdeuterated pentyl, perdeuterated isopentyl, perdeuterated neopentyl, perdeuterated tert-pentyl, perdeuterated hexyl, perdeuterated heptyl, perdeuterated octyl, perdeuterated nonyl, and perdeuterated decyl. The term "deuterated C₁₋₃ alkyl" or "deuterated C₁₋C₃ alkyl" of the present disclosure refers to an alkyl group containing from 1 to 3 carbon atoms substituted with one or more deuterium atoms, and its representative examples include perdeuterated methyl (CD₃) and perdeuterated ethyl (CD₃CD₂).

As used herein, the term "alkylene" (which is used interchangeably with "alkylene chain"), used alone or in combination, refers to a linear or branched divalent saturated hydrocarbon group composed of carbon and hydrogen atoms. The term "Cₓ-C_{y} alkylene" or "C_{x-y} alkylene" (x and y each being an integer) refers to a linear or branched alkylene group containing from x to y carbon atoms. Examples of the C₁-C₃₀ alkylene in the present disclosure may include C₁-C₃₀ alkylene, C₁-C₂₉ alkylene, C₁-C₂₈ alkylene, C₁-C₂₇ alkylene, C₁-C₂₆ alkylene, C₁-C₂₅ alkylene, C₁-C₂₄ alkylene, C₁-C₂₃ alkylene, C₁-C₂₂ alkylene, C₁-C₂₁ alkylene, C₁-C₂₀ alkylene, C₁-C₁₉ alkylene, C₁-C₁₈ alkylene, C₁-C₁₇ alkylene, C₁-C₁₆ alkylene, C₁-C₁₅ alkylene, C₁-C₁₄ alkylene, C₁-C₁₃ alkylene, C₁-C₁₂ alkylene, C₁-C₁₁ alkylene, C₁-C₁₀ alkylene, C₁-C₉ alkylene, C₁-C₈ alkylene, C₁-C₇ alkylene, C₁-C₆ alkylene, C₁-C₅ alkylene, C₁-C₄ alkylene, C₁-C₃ alkylene, or C₁-C₂ alkylene. Representative examples include, but are not limited to, methylene, ethylene, propylene, isopropylene, butylene, isobutylene, sec-butylene, tert-butylene, n-pentylene, isopentylene, neopentylidene, tert-pentylene, hexylene, heptylene, octylene, nonylene, decylene, undecylene, dodecylene, tridecylene, tetradecylene, pentadecylene, hexadecylene, heptadecylene, octadecylene, nonadecylene, eicosylene, heneicosylene, docosylene, tricosylene, tetracosylene, pentacosylene, hexacosylene, heptacosylene, octacosylene, nonacosylene, and triacontylene. In the present disclosure, the "alkylene" is optionally substituted with one or more substituents optionally selected from C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "alkoxy", used alone or in combination, refers to a linear or branched alkoxy group having structural formula of -O-alkyl. Optionally, the alkyl portion of the alkoxy group may contain 1-10 carbon atoms. Representative examples of "alkoxy" include, but are not limited to, methoxy, ethoxy, propoxy, isopropoxy, n-butoxy, isobutoxy, tert-butoxy, pentyloxy, 2-pentyloxy, isopentyloxy, neopentyloxy, hexyloxy, 2-hexyloxy, 3-hexyloxy, 3-methylpentyloxy, etc. The term "C₁-C₃ alkoxy" or "C₁₋₃ alkoxy" refers to a linear or branched alkoxy group containing from 1 to 3 carbon atoms. Representative examples of C₁₋₃ alkoxy include, but are not limited to, methoxy, ethoxy, n-propoxy, and isopropoxy.

As used herein, the term "halogenated alkoxy", used alone or in combination, refers to a alkoxy group substituted with one or more halogen atoms. Optionally, the alkyl portion of the alkoxy group may contain 1-10 carbon atoms. Examples of "halogenated alkoxy" include, but are not limited to, halogenated C₁₋₆ alkoxy or halogenated C₁₋₄ alkoxy. Representative examples include, but are not limited to, F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-.

As used herein, the term "alkylamino", used alone or in combination, refers to a linear or branched alkylamino group having structural formula of alkyl-NH-. Optionally, the alkyl portion of the alkylamino group may contain 1-10 carbon atoms. Representative examples of "alkylamino" include, but are not limited to, methyl-NH-, ethyl-NH-, n-propyl-NH-, isopropyl-NH-, n-butyl-NH-, isobutyl-NH-, tert-butyl-NH-, pentyl-NH-, and hexyl-NH-, etc. The term "C₁-C₃ alkyl-NH-" or "C₁₋₃ alkyl-NH-" refers to a linear or branched alkyl-NH- group containing from 1 to 3 carbon atoms. Representative examples of C₁₋₃ alkyl-NH- include, but are not limited to, methyl-NH-, ethyl-NH-, n-propyl-NH-, and isopropyl-NH-.

As used herein, the term "amino-substituted alkylene", used alone or in combination, refers to a linear or branched alkylene group substituted with amino having structural formula of NH₂-alkylene. Optionally, the alkylene portion of the amino-substituted alkylene group may contain 1-10 carbon atoms. The term "amino-substituted C₁₋₃ alkylene" or " NH₂-C₁₋₃ alkyl" refers to a linear or branched alkylene group containing from 1 to 3 carbon atoms which is substituted with amino. Representative examples of amino-substituted C₁₋₃ alkylene include, but are not limited to, NH₂-CH₂-, NH₂-CH₂CH₂-, and NH₂-CH₂CH₂CH₂-..

As used herein, the term "alkyl-NHC(O)-", used alone or in combination, refers to a linear or branched alkyl-NHC(O)- group having structural formula of alkyl-NHC(O)-. Optionally, the alkyl portion of the alkyl-NHC(O)- group may contain 1-10 carbon atoms. The term "C₁₋₃ alkyl-NHC(O)-" or "C₁-C₃ alkyl-NHC(O)-" refers to a linear or branched alkyl-NHC(O)- group containing from 1 to 3 carbon atoms. Representative examples of C₁₋₃ alkyl-NHC(O)- include, but are not limited to, CH₃-NHC(O)-, CH₃CH₂-NHC(O)-, and CH₃CH₂CH₂-NHC(O)-.

As used herein, the term "alkyl-C(O)NH-", used alone or in combination, refers to a linear or branched alkyl-C(O)NH- group having structural formula of alkyl-C(O)NH-. Optionally, the alkyl portion of the alkyl-C(O)NH- group may contain 1-10 carbon atoms. The term "C₁₋₃ alkyl-C(O)NH-" or "C₁-C₃ alkyl-C(O)NH-" refers to a linear or branched alkyl-C(O)NH- group containing from 1 to 3 carbon atoms. Representative examples of C₁₋₃ alkyl-C(O)NH- include, but are not limited to, CH₃-C(O)NH-, CH₃CH₂-C(O)NH-, and CH₃CH₂CH₂-C(O)NH-.

As used herein, the term "heteroaryl", used alone or in combination, refers to a 5- to 20-membered (e.g., 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, or 6- to 9-membered) monocyclic, bicyclic, or polycyclic cyclic hydrocarbon radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroaryl groups include bicyclic, tricyclic or tetracyclic heteroaryl groups, which contain one aromatic ring having one or more heteroatoms independently selected from O, S and N, and the remaining rings which may be a saturated, partially unsaturated or aromatic ring and can be carbocyclic ring or contain one or more heteroatoms independently selected from O, S and N. Examples of monocyclic heteroaryl groups include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, tetrazolyl, and triazinyl. Examples of bicyclic heteroaryl groups include, but are not limited to, indolyl, isoindolyl, isoindolinyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, oxazolopyridyl, furopyridyl, pteridyl, purinyl, pyridopyridyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl and imidazo[2,1-b]thiazolyl. Examples of tricyclic heteroaryl groups include, but are not limited to, acridinyl, benzindolyl, carbazolyl, dibenzofuranyl, and xanthyl. The heteroaryl group may be unsubstituted or substituted. A substituted heteroaryl refers to heteroaryl substituted one or more times (e.g., 1-4, 1-3, or 1-2 times) by a substituent(s) optionally selected from the group consisting of C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "heteroarylene", used alone or in combination, refers to a 5- to 20-membered (e.g., 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, or 6- to 9-membered) bivalent monocyclic, bicyclic, or polycyclic cyclic hydrocarbon radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroarylene groups include bicyclic, tricyclic or tetracyclic heteroarylene groups, which contain one aromatic ring having one or more heteroatoms independently selected from O, S and N, and the remaining ring(s) which may be a saturated, partially unsaturated or aromatic ring and can be carbocyclic ring or contain one or more heteroatoms independently selected from O, S and N. Examples of monocyclic heteroarylene groups include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, tetrazolylene, and triazinylene. Examples of bicyclic heteroarylene groups include, but are not limited to, indolylene, isoindolylene, isoindolinylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, oxazolopyridylene, furopyridylene, pteridylene, purinylene, pyridopyridylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, and imidazo[2,1-b]thiazolylene. Examples of tricyclic heteroarylene groups include, but are not limited to, acridinylene, benzindolylene, carbazolylene, dibenzofuranylene, and xanthylene. The heteroarylene group may be unsubstituted or substituted. A substituted heteroarylene refers to heteroarylene substituted one or more times (e.g., 1-4, 1-3, or 1-2 times) by a substituent(s) optionally selected from the group consisting of C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "aryl", used alone or in combination, refers to a monovalent aromatic hydrocarbon group containing from 5 to 14 carbon atoms and optionally one or more fused rings, such as phenyl group, naphthyl group, or fluorenyl group. As used herein, the "aryl" is optionally substituted. A substituted aryl group refers to an aryl group substituted one or more times (e.g., 1-4, 1-3, or 1-2 times) with a substituent(s). For example, aryl is mono-, di-, or tri-substituted with a substituent(s) optionally selected from e.g., C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "arylene", used alone or in combination, refers to a divalent aromatic hydrocarbon group containing from 5 to 14 carbon atoms and optionally one or more fused rings, such as phenylene, naphthylene, or fluorenylene. As used herein, the "arylene" is optionally substituted. A substituted arylene refers to an arylene group optionally substituted one or more times (e.g., 1-4, 1-3, or 1-2 times) with a substituent(s). For example, arylene is mono-, di-, or tri-substituted with a substituent(s) optionally selected from e.g., C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "cycloalkyl", used alone or in combination, refers to a saturated or partially unsaturated (i.e., containing one or more double bonds, but not having a fully conjugated *π-*electron system) monocyclic or bicyclic or polycyclic cyclic hydrocarbon radical, which in some embodiments contains from 3 to 20 carbon atoms (i.e., C₃₋₂₀ cycloalkyl), or from 3 to 15 carbon atoms (i.e., C₃₋₁₅ cycloalkyl), or from 3 to 12 carbon atoms (i.e., C₃₋₁₂ cycloalkyl), or from 3 to 11 carbon atoms (i.e., C₃₋₁₁ cycloalkyl), or from 3 to 10 carbon atoms (i.e., C₃₋₁₀ cycloalkyl), or from 3 to 8 carbon atoms (i.e., C₃₋₈ cycloalkyl), or from 3 to 7 carbon atoms (i.e., C₃₋₇ cycloalkyl), or from 3 to 6 carbon atoms (i.e., C₃₋₆ cycloalkyl). The term "cycloalkyl" includes monocyclic, bicyclic, or tricyclic cyclic hydrocarbon radical having from 3 to 20 carbon atoms. Representative examples of monocyclic cycloalkyl groups include, but are not limited to, cyclopropyl, cyclobutyl, cyclopentyl, cyclopentenyl, cyclohexyl, cyclohexenyl, cycloheptyl, and cyclooctyl. Bicyclic and tricyclic cycloalkyl groups include bridged cycloalkyl, fused cycloalkyl and spiro-cycloalkyl groups such as, but not limited to, decalinyl, octahydropentalenyl, octahydro-1H-indenyl, spiro-cycloalkyl, adamantanyl, noradamantanyl, bornyl, norbornyl (also named as bicyclo[2.2.1]heptyl by the IUPAC system). As used herein, the "cycloalkyl" is optionally mono- or poly-substituted, such as, but not limited to, 2,2-, 2,3-, 2,4-, 2,5-, or 2,6-disubstituted cyclohexyl. The substituents of the substituted "cycloalkyl" can be optionally one or more (e.g., 1-5, 1-4, 1-3, 1-2, or 1) substituents selected from C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof. Examples of "C₃₋₆ cycloalkyl" include, but are not limited to, cyclopropyl, cyclobutyl, cyclopentyl, cyclopentenyl, and cyclohexyl.

As used herein, the term "cycloalkylene", used alone or in combination, refers to a saturated or partially unsaturated (i.e., containing one or more double bonds, but not having a fully conjugated π-electron system) monocyclic or bicyclic or polycyclic divalent cyclic hydrocarbon radical containing from 3 to 12 carbon atoms (e.g., 3-12, 3-11, 3-10, 3-8, 3-7, 3-6 carbon atoms). The term "cycloalkylene" includes monocyclic, bicyclic or tricyclic cycloalkylene having from 3 to 12 carbon atoms. Representative examples of monocyclic cycloalkylene groups include, but are not limited to, cyclopropylene, cyclobutylene, cyclopentylene, cyclopentenylene, cyclohexylene, cyclohexenylene, cycloheptylene, and cyclooctylene. Bicyclic and tricyclic cycloalkylene groups include bridged cycloalkylene, fused cycloalkylene and spiro-cycloalkylene groups such as, but not limited to, decalinylene, octahydropentalenylene, octahydro-1H-indenylene, 2,3-dihydro-1H-indenylene, spiro-cycloalkylene, adamantanylene, noradamantanylene, and norbomylene (also named as bicyclo[2.2.1]heptylene by the IUPAC system). As used herein, the "cycloalkylene" is optionally mono- or poly-substituted, such as, but not limited to, 2,2-, 2,3-, 2,4-, 2,5-, or 2,6-disubstituted cyclohexylene. The substituents of the substituted "cycloalkylene" can be optionally one or more substituents selected from C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "C_{x-y} spiro-cycloalkylene" (x and y each being an integer), used alone or in combination, refers to a spiro-cycloalkylene group containing from x to y carbon atoms. As used herein, the term "C₇₋₁₁ spiro-cycloalkylene", used alone or in combination, refers to a spiro-cycloalkylene group containing from 7 to 11 carbon atoms (e.g., 7-10, 7-9 carbon atoms). Representative examples of "C₇₋₁₁ spiro-cycloalkylene" include, but are not limited to, spiro[3.3]heptylene, spiro[2.5]octylene, spiro[3.5]nonylene, spiro[4.4]nonylene, spiro[4.5]decylene, or spiro[5.5]undecylene. The "C₇₋₁₁ spiro-cycloalkylene" is optionally further substituted with one or more substituents selected from the group consisting of C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "heterocyclyl" or "heterocyclic group", used alone or in combination, refers to a 3- to 20-membered saturated or partially unsaturated (i.e., containing one or more double bonds, but not having a fully conjugated π-electron system) monocyclic, bicyclic, or tricyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. In some embodiments, "heterocyclyl" may refer to a 3- to 15-membered (e.g., 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated π-electron system) monocyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. Representative examples of the monocyclic heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, and diazacycloheptanyl (e.g., 1,4-diazacycloheptan-1-yl), and diazacyclooctyl. Bicyclic and tricyclic heterocyclyl groups include bridged heterocyclyl, fused heterocyclyl and spiro-heterocyclyl groups such as, but not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, 2,5-diazabicyclo[2.2.2]octan-2-yl, and azaspirocycloalkyl (including 3-azaspiro[5.5]undecan-3-yl). The heterocyclyl may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

As used herein, the term "nitrogen-containing monocyclic heterocyclyl", used alone or in combination, refers to 3- to 20-membered (e.g., 3- to 15-membered, 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated π-electron system) monocyclic monovalent cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Representative examples of the nitrogen-containing monocyclic heterocyclyl include, but are not limited to, azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl (e.g., 1,4-diazacycloheptan-1-yl), and diazacyclooctyl. The nitrogen-containing monocyclic heterocyclyl may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

As used herein, the term "nitrogen-containing bridged heterocyclyl", used alone or in combination, refers to 3- to 20-membered (e.g., 3- to 15-membered, 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated π-electron system) tricyclic monovalent cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Tricyclic heterocyclyl groups include bridged heterocyclyl, such as but not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, and 2,5-diazabicyclo[2.2.2]octan-2-yl. The nitrogen-containing bridged heterocyclyl may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

As used herein, the term "-O-optionally substituted heterocyclyl", used alone or in combination, refers to a group formed by optionally substituted heterocyclyl connected to oxygen. Optionally, the heterocyclyl of "-O-optionally substituted heterocyclyl" is e.g., 4- to 20-membered monocyclic or bicyclic cyclic heterocyclyl containing one or more (e.g., from 1 to 4, or 1, 2, 3 or 4) heteroatoms independently selected from sulfur, oxygen, and nitrogen, including but not limited to azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo [2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). As used herein, the heterocyclyl is optionally substitutedwith a substituent selected from deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

As used herein, the term "heterocyclylene", used alone or in combination, refers to a 3- to 20-membered saturated or partially unsaturated (i.e., containing one or more double bonds, but not having a fully conjugated π-electron system) monocyclic, bicyclic, or tricyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. In some embodiments, "heterocyclylene" may refer to e.g., a 3- to 15-membered (optionally 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated π-electron system) monocyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. Representative examples of the monocyclic heterocyclylene include, but are not limited to, azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, and diazacycloheptanylene (e.g., 1,4-diazacycloheptanylene, 4,5-diazacycloheptanylene, 1,3-diazacycloheptanylene). Bicyclic and tricyclic heterocyclylene groups include bridged heterocyclylene, fused heterocyclylene and spiro-heterocyclylene groups such as, but not limited to, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and azaspirocycloalkylene (e.g., 3-azaspiro[5.5]undecanylene). The heterocyclylene may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

As used herein, the term "nitrogen-containing monocyclic heterocyclylene", used alone or in combination, refers to 3- to 20-membered (e.g., 3- to 15-membered, 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated π-electron system) monocyclic bivalent cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Representative examples of the nitrogen-containing monocyclic heterocyclylene include, but are not limited to, piperidinylene, piperazinylene, morpholinylene, azetidinylene, pyrrolidinylene, imidazolidinylene, pyrazolidylene, oxazolidinylene, thiazolidinylene, thiomorpholinylene, azacycloheptylene, diazacycloheptanylene, azacyclooctylene, and diazacyclooctylene. The nitrogen-containing monocyclic heterocyclylene may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

As used herein, the term "nitrogen-containing bridged heterocyclylene", used alone or in combination, refers to 3- to 20-membered (optionally 3- to 15-membered, 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated π-electron system) tricyclic bivalent cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Tricyclic heterocyclylene groups include bridged heterocyclylene, such as but not limited to, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, and 2,5-diazabicyclo[2.2.2]octanylene. The nitrogen-containing heterocyclylene may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

As used herein, the term "alkynylene", used alone or in combination, refers to a linear or branched bivalent hydrocarbon group containing from 2 to 8 (e.g., from 2 to 6, from 2 to 5, from 2 to 4, or preferably 2) carbon atoms and having one or more (e.g., from 1 to 3, from 1 to 2, or 1) carbon-carbon triple bonds. Examples of alkynylene include, but are not limited to, ethynylene, 1-propynylene, 1-butynylene, and 1,3-diynylene.

As used herein, the term "alkynyl", used alone or in combination, refers to a linear or branched monovalent hydrocarbon group containing from 2 to 8 (e.g., from 2 to 6, from 2 to 5, from 2 to 4, or preferably 2) carbon atoms and having one or more (e.g., from 1 to 3, from 1 to 2, or 1) carbon-carbon triple bonds. Examples of C₂₋₆ alkynylene include, but are not limited to, ethynyl, 1-propynyl, 1-butynyl, and 1,3-diynyl.

As used herein, the term "alkenylene", used alone or in combination, refers to a linear or branched divalent hydrocarbon group containing from 2 to 8 (e.g., from 2 to 6, from 2 to 5, from 2 to 4, from 2 to 3, or 2) carbon atoms and having one or more (e.g., from 1 to 3, from 1 to 2, or 1) carbon-carbon double bonds. Examples of alkenylene groups include, but are not limited to, vinylene (e.g., - CH=CH-), 1-propenylene, allylidene, 1-butenylene, 2-butenylene, 3-butenylene, isobutenylene, pentenylene, n-pent-2,4-dienylene, 1-methyl-but-1-enylene, 2-methyl-but-1-enylene, 3-methyl-but-1-enylene, 1-methyl-but-2-enylene, 2-methyl-but-2-enylene, 3-methyl-but-2-enylene, 1-methyl-but-3-enylene, 2-methyl-but-3-enylene, 3-methyl-but-3-enylene, and hexenylene.

As used herein, the term "alkenyl", used alone or in combination, refers to a linear or branched monovalent hydrocarbon group containing from 2 to 8 (e.g., from 2 to 6, from 2 to 5, from 2 to 4, from 2 to 3, or 2) carbon atoms and having one or more (e.g., from 1 to 3, from 1 to 2, or 1) carbon-carbon double bonds. Examples of C₂₋₆ alkenyl group include, but are not limited to, vinyl (e.g., CH₂=CH-), 1-propenyl, allyl, 1-butenyl, 2-butenyl, 3-butenyl, isobutenyl, pentenyl, n-pent-2,4-dienyl, 1-methyl-but-1-enyl, 2-methyl-but-1-enyl, 3-methyl-but-1-enyl, 1-methyl-but-2-enyl, 2-methyl-but-2-enyl, 3-methyl-but-2-enyl, 1-methyl-but-3-enyl, 2-methyl-but-3-enyl, 3-methyl-but-3-enyl, and hexenyl.

As used herein, "bornylane" or "bornane" (also known as 1,7,7-trimethylbicyclo[2.2.1]heptane; camphane; bornylane) has a definition known to those skilled in the art. As used herein, "camphanyl" or "bornyl" refers to a monovalent group of bornane, i.e., the group remaining after any one of the hydrogens in bornane is removed. Representative examples of "bornyl" include, but are not limited to, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl,

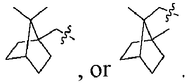

As used herein, "bicyclo[2.2.1]heptane" also known as "norbornane", has a definition known to those skilled in the art. As used herein, "bicyclo[2.2.1]heptyl" or "norbornyl" refers to a monovalent group of bicyclo[2.2.1]heptane, i.e., the group remaining after any hydrogen in bicyclo[2.2.1]heptane is removed. Representative examples of "bicyclo[2.2.1]heptyl" include, but are not limited to, bicyclo[2.2.1]heptan-2-yl, bicyclo[2.2.1]heptan-3-yl, bicyclo[2.2.1]heptan-4-yl, bicyclo[2.2.1]heptan-5-yl, or bicyclo[2.2.1]heptan-6-yl.

As used herein, the term "bicyclo[2.2.1]heptene" has a definition known to those skilled in the art. As used herein, "bicyclo[2.2.1]heptenyl" refers to a monovalent group of bicyclo[2.2.1]heptene, i.e., the group remaining after any hydrogen in bicyclo[2.2.1]heptene is removed. Representative examples of "bicyclo[2.2.1]heptenyl" include, but are not limited to, bicyclo[2.2.1]hept-5-en-2-yl, bicyclo[2.2.1]hept-5-en- 3-yl, or bicyclo[2.2.1]hept-5-en-7-yl. 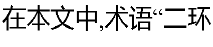 [2.2.1] 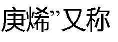 bicyclo[2.2.1]heptene),

As used herein, "adamantane" (also known as tricyclo[3.3.1.1^{3,7}]decane) has a definition known to those skilled in the art, and its structural formula is e.g., as follows: 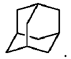 As used herein, "adamantanyl" refers to a monovalent group of adamantane, that is, the group remaining after any hydrogen in adamantane is removed. Representative examples of "adamantanyl" include, but are not limited to, 1-adamantanyl, 2-adamantanyl, 3-adamantanyl, 4-adamantanyl, 5-adamantanyl, 6-adamantanyl, 7-adamantanyl, 8-adamantanyl, 9-adamantanyl, or 10-adamantanyl.

As used herein, "noradamantane" has a definition known to those skilled in the art, and its structural formula is e.g., as follows: 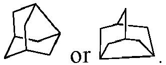 As used herein, "noradamantanyl" refers to a monovalent group of noradamantane, that is, the group remaining after any hydrogen in noradamantane is removed. Representative examples of "noradamantanyl" include, but are not limited to, 1-noradamantanyl, 2-noradamantanyl, 3-noradamantanyl, 4-noradamantanyl, 5-noradamantanyl, 6-noradamantanyl, 7-noradamantanyl, 8-noradamantanyl or 9-noradamantanyl.

As used herein, "adamantanamine" has the definitions known to those skilled in the art, namely referring to an adamantane having an amino substituent, wherein the amino substituent can replace a hydrogen on a carbon at any position in the adamantane. An example of "adamantanamine" can be adamantan-1-amine (corresponding English chemical name is adamantan-1-amine or Tricyclo[3.3.1.1^{3,7}]decan-1-amine; CAS No.: 768-94-5), with the following structural Formula:

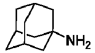

In the present disclosure, the term "leaving group" used alone or in combination is well known to those skilled in the art, which is a leaving molecular fragment (ion or neutral molecule) that carries a pair of electrons from a reactant in chemical reactions, as is a term used in nucleophilic substitution and elimination reactions. Common ionic leaving groups include Cl⁻, Br⁻, I⁻ and sulfonate (such as p-toluenesulfonate, TsO⁻), and neutral molecular leaving groups include water, ammonia and alcohol. In the present disclosure, those skilled in the art can select an appropriate leaving group as needed, such as but not limited to -N₃, halogen, methanesulfonyloxy, trifluoromethanesulfonyloxy or p-toluenesulfonyloxy, etc.

Salts or pharmaceutically acceptable salts, enantiomers, stereoisomers, solvates, polymorphs of the compounds of Formula (I), Formula (II) or Formula (III) of the present disclosure are also encompassed within the scope of the present invention.

In all embodiments of the present disclosure, the salts or pharmaceutically acceptable salts of the compounds of Formula (I), Formula (II) or Formula (III) refer to non-toxic inorganic or organic acid and/or base addition salts. Examples include: sulfates, hydrohalides (including hydrochlorides and hydrobromates), citrates, maleates, methanesulfonates, citrates, lactates, L-tartrates, fumarates, L-malates, phosphates, dihydrophosphates, pyrophosphates, metaphosphates, oxalates, malonates, benzoates, mandelates, succinates, glycolate, methanesulfonates, or p-toluenesulfonates, etc.

"Pharmaceutically acceptable carrier" refers to a pharmaceutically acceptable material, such as a filler, stabilizer, dispersant, suspending agent, diluent, excipient, thickener, solvent, or encapsulating material, with which the useful compounds according to the present disclosure are carried or transported into or administered to a patient so that they can perform their intended function. Generally, such constructs are carried or transported from one organ or part of the body to another organ or part of the body. The carrier is compatible with the other ingredients of the formulation, including the compounds useful in the present disclosure, and is not harmful to the patient, and the carrier must be "acceptable". Some examples of materials that can be used as pharmaceutically acceptable carriers include sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; and other common non-toxic compatible substances used in pharmaceutical formulations.

As used herein, the term "room temperature" refers to the ambient temperature, such as 20-30°C.

As used herein, "stereoisomer" refers to a compound with the same chemical structural formula, but a different arrangement of atoms or groups in space. Stereoisomers include enantiomers, diastereomers, conformational isomers (rotational isomers), geometric isomers (cis/trans isomers), atropisomers, and so on.

As used herein, the term "solvate" refers to an association or complex formed by the interaction between one or more solvent molecules and compounds of the present invention. Examples of solvents include water, isopropanol, ethanol, methanol, DMSO, ethyl acetate, acetic acid and ethanolamine. The term "hydrate" means that a complex formed with water.

As used herein, the term "chiral" refers to a molecule that is nonsuperimposable on its mirror image; whereas "achiral" refers to a molecule that can be superimposed on its mirror image.

As used herein, the term "enantiomers" refers to two isomers of a compound that are nonsuperimposable mirror images.

As used herein, the term "diastereomers" refers to stereoisomers that have two or more chiral centers but are not mirror images of each other. The diastereomers have different physical properties, such as melting point, boiling point, spectral properties and reactivity. The diastereomeric mixture can be separated by high-resolution analytical operations such as electrophoresis and chromatography, such as HPLC.

### Examples

In the following description, numerous specific details are set forth in order to provide a thorough understanding of the present disclosure. The present disclosure may be practiced without some or all of these specific details. In other cases, well-known process operations have not been described in detail in order not to unnecessarily obscure the present disclosure. Although the present disclosure will be described in conjunction with specific embodiments, it should be understood that this is not intended to limit the present disclosure to these embodiments.

The following abbreviations are used throughout the specification and examples:
- ACN: acetonitrile
- AcOH: acetic acid
- AcOK: potassium acetate
- Bn: Benzyl
- Boc: t-Butyloxy carbonyl
- Brettphos: Pd G3 Methanesulfonato(2-dicyclohexylphosphino-3,6-dimethoxy-2',4',6'-tri-i-propyl-1,1'-biphenyl)(2'-amino-1,1'-biphenyl-2-yl)palladium(II)
- Brettphos: Dicyclohexyl[3,6-dimethoxy-2',4',6'-tris(1-methylethyl)[1,1'-biphenyl]-2-yl]phosphine
- BINAP: 2,2'-Bis(diphenylphosphino)-1,1 '-binaphthalene
- BPO: Dibenzoyl peroxide
- CataCXium A Pd: G₃ Methanesulfonatobutyl-bis(1-adamantyl)phosphinepalladium(II)
- Cbz: Carbobenzoxy
- Con.: Concentration
- DCE: 1,2-Dichloroethane
- DCM: dichloromethane
- DEA: Diethylamine
- DIAD: Diisopropyl azodicarboxylate
- DIEA: N, N-diisopropylethylamine
- DMA: N,N-Dimethylacetamide
- DMAP: 4-Dimethylaminopyridine
- DME: Dimethylether
- DMF: N,N-dimethylformamide
- DMSO: dimethyl sulfoxide
- DIPEA: N, N-diisopropylethylamine
- EDCI: 1-(3-dimethylaminopropyl)-3-ethylcarbodiimide hydrochloride
- ESI: electrospray ionization
- equiv: equivalent
- EtOH: ethanol
- EtOAc or EA: Ethyl acetate
- HATU: 2-(7-Azabenzotriazol-1-yl)-N,N,N',N'-tetramethyluronium hexafluorophosphate
- HPLC: high performance liquid chromatography
- HRMS: high resolution mass spectrometry
- IPA: Isopropyl alcohol
- LC-MS: liquid chromatography-mass spectrometry
- LiHMDS: Lithium hexamethyldisilazide
- LRMS: low resolution mass spectrometry
- LC: liquid chromatography
- Me: methyl
- MeCN: acetonitrile
- MeOH: Methanol
- MOMO-: Methoxymethoxy-
- MS: mass spectrometry
- MsO-: Methanesulfonyloxy
- NaHMDS: Sodium bis(trimethylsilyl)amide
- NBS: N-Bromosuccinimide
- NMI: N-Methylimidazole
- ¹H: NMR Proton nuclear magnetic resonance
- HFIP: Hexafluoroisopropanol
- MeO-: Methoxy
- o/n: Overnight
- PCC: Pyridinium chlorochromate
- Pin₂B₂: Pinacolborane
- PyBOP: Benzotriazol-1-yl-oxy-tris(pyrrolidino)phosphonium hexafluorophosphate
- Ruphos: Pd G3 Methanesulfonato(2-dicyclohexylphosphino-2',6'-diisopropoxy-1,1'-biphenyl)(2'-amino-1,1'-biphenyl-2-yl)palladium(II) (CAS No.: 1445085-77-7)
- rt: room temperature
- tBu: tert-butyl
- tBuONa: Sodium tert-butoxide
- TBSCl: tert-Butyldimethylchlorosilane
- TCFH: N,N,N',N'-Tetramethylchloroformamidinium hexafluorophosphate
- TEA: triethylamine
- TFA: trifluoroacetic acid
- T_{f}O-: Trifluoromethanesulfonate
- Tf₂O: Triflic anhydride
- THF: Tetrahydrofuran
- THP: Tetrahydropyranyl
- TLC: thin layer chromatography
- TMS: tetramethylsilane
- TsO-: Tosyloxy
- TsOH: p-Toluenesulfonic acid
- Xantphos: or Xphos 4,5-Bis(diphenylphosphino)-9,9-dimethylxanthene

In the present disclosure, the ¹H NMR spectrum was recorded on a Bruker-500MHz nuclear magnetic resonance instrument, by using, as a solvent, CD₃OD (δ = 3.31 ppm) containing 0.1% TMS (as an internal standard); or using, as a solvent, CDCl₃ (δ = 7.26 ppm) containing 0.1% TMS (as an internal standard); or using, as a solvent, DMSO-*d*₆ (δ = 2.50 ppm) containing 0.03% TMS (as an internal standard). LRMS spectrum was recorded on an AB Triple 4600 mass spectrometer, HPLC preparation was measured on a SHIMADZU LC-20AP type instrument, and HPLC purity was measured on a SHIMADZU LC-30AP or Waters 1525 type instrument. Unless otherwise specified, all reactions were performed in the air atmosphere. The reactions were monitored via TLC or LC-MS.

Solvents and reagents are processed as follows:
the solvents used in the reactions such as DCM, DMF, NMP, anhydrous EtOH, and anhydrous MeOH were commercially available;
Unless otherwise specified, compounds corresponding to the PBM or PBM-R_{f} portion of Formula (II), such as demethylated derivative from imatinib, intermediate of dasatinib N-(2-chloro-6-methylphenyl)-2-[(6-chloro-2-methyl-4-pyrimidinyl)amino]-5-thiazolecarboxamide, intermediate of Bosutinib 7-(3-chloropropoxy)-4-[(2,4-dichloro-5-methoxyphenyl)amino]-6-methoxy-3-cyanoquinoline, and various carbon chain linkers of different lengths (including compounds used to form the groups represented by R₅ or R₆), as well as other reagents and drugs, were commercially available, or synthesized using methods known in the art.
Unless otherwise specified, the materials and reagents used in the following examples are commercially available and used directly, or can be synthesized by using methods known in the art.

### General synthesis methods

Compounds and/or pharmaceutically acceptable salts thereof of the present disclosure can be synthesized using commercially available raw materials by synthetic techniques known in the art. The synthetic schemes described below illustrate the preparation of most compounds. The starting materials or reagents used in each scheme can be purchased from commercial sources or prepared by methods known to those skilled in the art. One skilled in the art can prepare the salts, racemates, enantiomers, phosphates, sulfates, hydrochlorides and prodrug forms of the compounds of Formula (I) and Formula (II) of the present disclosure according to routine techniques in the art.

### General synthesis method of intermediate compounds 1:

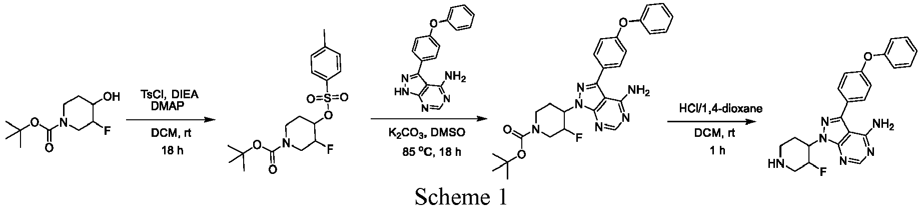

### General synthesis method of intermediate compounds 2:

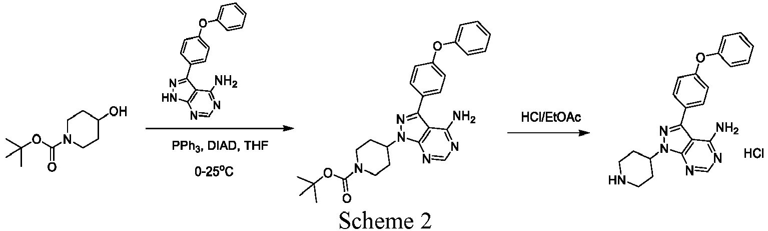

**General synthesis method of intermediate compounds 3:**

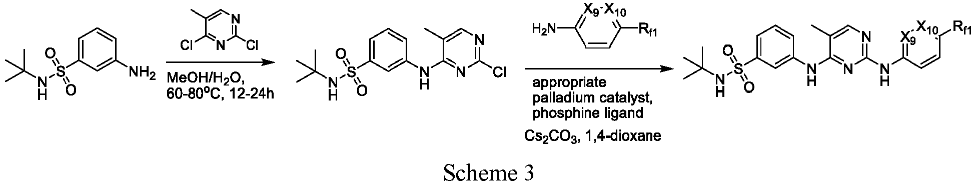

In Scheme 3, X₉ and X₁₀ of the amine substrate used in Step 2 are the same or different and each independently represent N or CH. R_{f1} is a monovalent group corresponding to the divalent group R_{f} of the compound of Formula (II). For example, R_{f1} represents -OH, -NH(R_{g})-, -NHC(O)-Rₕ-W⁵-H, -C(O)NH-Rₕ-W⁵-H, -NHC(O)-W⁵-H, -C(O)NH-W⁵-H, -O-Rₕ-W⁵-H, -O-W⁵-H, -O-Rₕ-NH(Rᵢ), -N(R_{g})-Rₕ-NH(Rᵢ), -W⁵-H, -W⁵-NH(R_{g}), -N(R_{g})-W⁵-H, -N(R_{g})-W⁵-NH(Rᵢ), -Rₕ-W⁵-H, -Rₕ-C(O)-W⁵-H, - C(O)-W⁵-H, -Rₕ-C(O)NH-Rⱼ-W⁵-H, -Rₕ-NHC(O)-Rⱼ-W⁵-H, -Rₕ-NH(Rᵢ), or 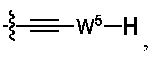 wherein R_{g}, Rₕ, Rᵢ, Rⱼ, and W⁵ are as defined in the compound of Formula (II) and its various embodiments in the present disclosure. It should be understood that when the group R_{f1} contains reactive hydrogen that can participate in the reaction of Step 2, the reactive hydrogen can be protected by techniques and methods known to those skilled in the art, such as using protecting groups. For example, when R_{f1} represents a piperidinyl or piperazinyl group, conventional protecting groups such as Boc can be used to protect the hydrogen on the N. The removal of the protecting group can be achieved by techniques and methods known to those skilled in the art. For instance, the removal of the Boc protecting group can be accomplished under acidic conditions such as hydrochloric acid or trifluoroacetic acid.

The palladium catalyst and phosphine ligand used in Step 2 of Scheme 3 can be conventional ones suitable for coupling reactions. There are many types of conventional palladium catalysts used for coupling reactions, such as palladium acetate (Pd(OAc)₂), tris(dibenzylideneacetone)dipalladium (Pd₂(dba)₃), diphenylphosphinoferrocene palladium dichloride, tetraphenylphosphine palladium, dichlorobis(triphenylphosphine) palladium, palladium on carbon, and so on. The phosphine ligand can be a conventional one like Xantphos or BINAP.

In Step 1 of Scheme 3, to a solution of 3-amino-N-(tert-butyl)benzenesulfonamide (9.50 g, 41.608 mmol) in MeOH/H₂O (400 mL) was added2,4-dichloro-5-methylpyrimidine (6.78 g, 41.608 mmol), and the reaction was stirred at 80°C for 18 hours. After monitoring the reaction via LCMS and confirming its completion, the reaction mixture was cooled to room temperature, resulting in the precipitation of a large amount of white solid. The reaction mixture was filtered, and the filter cake was washed with methanol/water (50 mL, 1: 1), dried under high vacuum at 50°C, yielding the white solid compound N-(tert-butyl)-3-((2-chloro-5-methylpyrimidin-4-yl)amino)benzenesulfonamide (8.00 g, 22.544 mmol, 54.18%). LCMS (ESI): calcd for (M) 354.09, found, (M+H)⁺ 355.10.

In Step 2 of Scheme 3, to a solution of N-(tert-butyl)-3-((2-chloro-5-methylpyrimidin-4-yl)amino)benzenesulfonamide (1 equiv) and the corresponding amine substrate (1.3 equiv) in dioxane (50 mL) were added Cs₂CO₃ and a suitable palladium catalyst/phosphine ligand. The reaction was stirred at 100°C for 4 hours. The reaction mixture was diluted with ethyl acetate and saturated NaCl solution, dried over Na₂SO₄, and concentrated under vacuum to obtain the crude product. The crude product was purified by silica gel flash column chromatography (eluent (v/v): EtOAc/PE = 0/1-1/4) to give the target compound.

### General synthesis method of intermediate compounds 4:

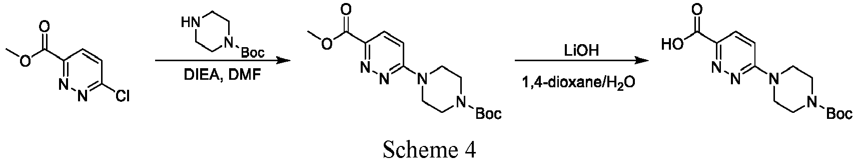

### General synthesis method of intermediate compounds 5:

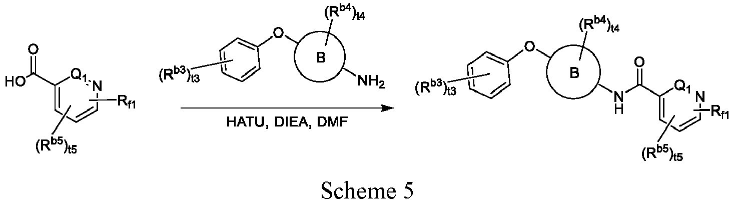

In Scheme 5, the amine substrate (R^{b3})ₜ₃, ring B, (R^{b4})ₜ₄, and the acid substrate Q₁ and (R^{b5})ₜ₅ used are defined in Formula (PBM-7-1A-1) and its various embodiments in the present disclosure. The R_{f1} of the acid substrate used is a monovalent group corresponding to the divalent group R_{f} of the compound of Formula (II). For example, R_{f1} represents -OH, -NH(R_{g})-, -NHC(O)-Rₕ-W⁵-H, -C(O)NH-Rₕ-W⁵-H, -NHC(O)-W⁵-H, -C(O)NH-W⁵-H, -O-Rₕ-W⁵-H, -O-W⁵-H, -O-Rₕ-NH(Rᵢ), -N(R_{g})-Rₕ-NH(Ri), -W⁵-H, -W⁵-NH(R_{g}), -N(R_{g})-W⁵-H, -N(R_{g})-W⁵-NH(Rᵢ), -Rₕ-W⁵-H, -Rₕ-C(O)-W⁵-H, -C(O)-W⁵-H, -Rₕ-C(O)NH-Rⱼ-W⁵-H, -Rₕ-NHC(O)-Rⱼ-W⁵-H, -Rₕ-NH(Rᵢ), or 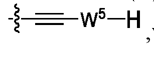 wherein R_{g}, Rₕ, Rᵢ, Rⱼ, and W⁵ are as defined in the compound of Formula (II) and its various embodiments in the present disclosure. It should be understood that when the group R_{f1} contains an active hydrogen or reactive group that can participate in the reaction of Scheme 5, the active hydrogen or reactive group can be protected by techniques and methods well known to those skilled in the art, such as protective groups. For example, when R_{f1} represents a piperidinyl or piperazinyl group, conventional protective groups such as Boc can be used to protect the hydrogen on N. The removal of the protective group can be achieved by techniques and methods well known to those skilled in the art, such as removing the protective group Boc under acidic conditions such as hydrochloric acid or trifluoroacetic acid.

In Scheme 5, to a solution of the acid substrate (1 equiv) and amine substrate (1 equiv) in DMF were added HATU (1.5 equiv) and DIEA (5 equiv). The reaction mixture was then allowed to react at room temperature for 30 minutes. After monitoring the reaction via LCMS and confirming its completion, water was added to the reaction mixture, and the resulting mixture was extracted with ethyl acetate. The organic phase was washed with saturated saline and concentrated to obtain the crude product. The crude product was purified using a chromatography column (eluent (v/v): DCM/MeOH=100/1) to give the target compound.

### General synthesis method of intermediates (JQ-1 derivatives):

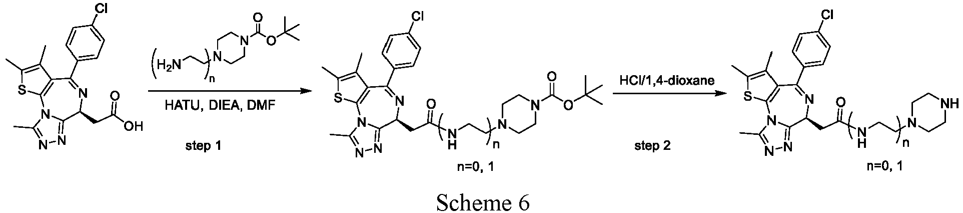

### Step 1:

To a solution of (S)-2-(4-(4-chlorophenyl)-2,3,9-trimethyl-6H-thieno[3,2-f][1,2,4]triazolo[4,3-a][1,4]diazepin-6-yl)acetic acid (1 equiv), the corresponding amine substrate (1.2 equiv) and HATU (2 equiv) in 10 mL of DCM was added DIEA (5 equiv), and the resulting reaction mixture was allowed to react at room temperature for 1 hour. The reaction solution was washed twice with 10 mL of water and concentrated under reduced pressure to remove the solvent. The resulting residue was purified by silica gel column chromatography (eluent (v/v): DCM to DCM/MeOH (10/1)) to give the Boc intermediate compound.

### Step 2:

The Boc intermediate compound obtained in Step 1 was dissolve in HCl/1,4-dioxane, and the resulting reaction mixture was allowed to react at room temperature for 1 hour. The reaction solution was concentrated under reduced pressure to remove the solvent to give the target compound.

### General synthesis method of compounds of Formula (I) 1:

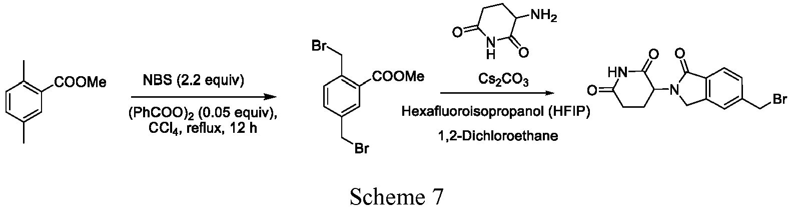

### General synthesis method of compounds of Formula (I) 2:

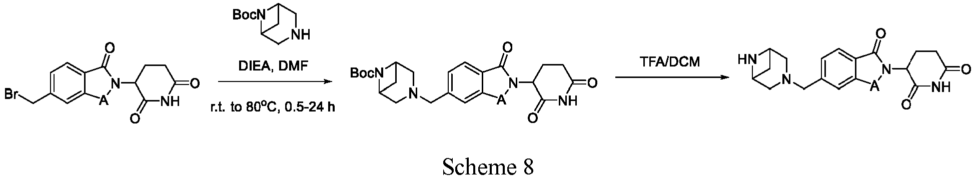

In Scheme 8, A is as defined in the compound of Formula (I) herein.

Step 1: to a solution of 3-(5-(bromomethyl)-1-oxoisoindolin-2-yl)piperidine-2,6-dione (1 equiv) in 4 mL of DMF were added the corresponding amine (1.1 equiv) and N,N-diisopropylethylamine (3 equiv), and the resulting reaction mixture was allowed to react overnight at room temperature. After monitoring the reaction via LCMS and confirming its completion, the reaction system was extracted with ethyl acetate. The organic phases were combined and washed with water, dried over anhydrous sodium sulfate, and filtered. The filtrate was concentrated under reduced pressure. The resulting residue was subjected to a column chromatography (DCM: EtOAc = 2:1 to 1:1) for separation to give the Boc intermediate compound.

Step 2: to a solution of the product from the previous step in 5 mL of DCM was added CF₃CO₂H (1 mL). The reaction mixture was reacted at room temperature for 3h, and concentrated under reduced pressure to give trifluoroacetate salt of the target compound.

Depending upon the target compound, the reaction substrate amine, reaction conditions (including reaction dosage, temperature, time, etc.), and work up, etc. in Scheme 8 can be appropriately modified and adjusted using techniques and methods well known to those skilled in the art to obtain the desired target compound. In some embodiments, the reaction substrate amine can be an aliphatic amine (such as HNR₇R₈) corresponding to the R group of the compound of Formula (I) of the present disclosure or a structure corresponding to the group W¹ (such as 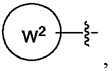 nitrogen-containing monocyclic heterocycles, nitrogen-containing bridged heterocycles, nitrogen-containing spiro heterocycles or nitrogen-containing fused heterocycles; or 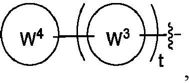 wherein each ring W³ is the same or different and each independently represents an optionally substituted nitrogen-containing heterocyclylene, t represents an integer of 1 or 2, and ring W⁴ represents an optionally substituted aryl, optionally substituted heteroaryl or optionally substituted heterocyclyl). It should be understood that when the reaction substrate amine contains additional reactive hydrogens or reactive groups that can participate in the reaction of Step 1 of Scheme 6, protective groups well known to those skilled in the art can be used to protect the reactive hydrogens or reactive groups. For example, when the reaction substrate amine represents piperazine, conventional protective groups such as Boc can be used to protect the hydrogen on one of the nitrogens of the piperazine. The removal of the protective group can be achieved by techniques and methods well known to those skilled in the art, such as removing the protective group Boc under acidic conditions such as hydrochloric acid or trifluoroacetic acid.

### General synthesis method of compounds of Formula (I) 3:

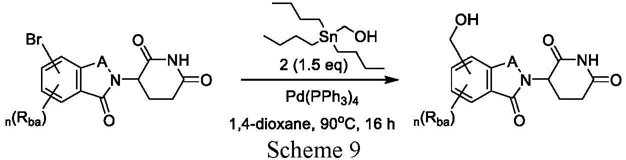

In Scheme 9, Br can be at the 4-, 5-, 6-, or 7-position on the phenyl ring of the isoindolinyl group, and the resulting hydroxymethyl group is correspondingly located at the 4-, 5-, 6-, or 7-position on the phenyl ring of the isoindolinyl group. A is as defined in the compound of Formula (I) herein. (R_{ba})ₙ indicates that the phenyl ring is substituted with n R_{ba} groups, with each R_{ba} being the same or different and each independently representing deuterium, fluorine, protected hydroxy, protected thiol, nitro, protected amino, cyano, optionally deuterated C₁₋₆ alkyl, optionally deuterated C₁₋₆ alkoxy, halo C₁₋₆ alkyl, optionally substituted C₃₋₆ cycloalkyl, C₂₋₆ alkenyl, or C₂₋₆ alkynyl; and n represents an integer of 0, 1, 2, or 3.

### General synthesis method of compounds of Formula (I) 4:

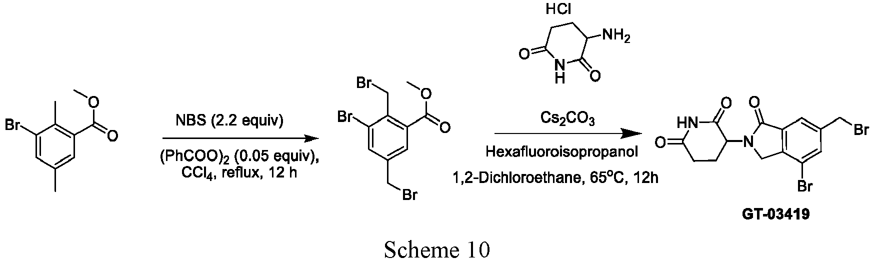

### General synthesis method of compounds of Formula (II) 1:

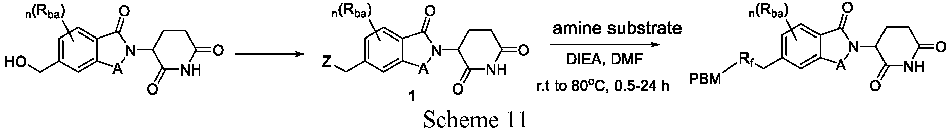

In Scheme 11, the amine substrate is a compound corresponding to the PBM-R_{f} portion of the compound of Formula (II) of the present disclosure, containing aliphatic amine or heterocyclic amine (such as nitrogen-containing monocyclic heterocycle, nitrogen-containing bridged heterocycle, nitrogen-containing spiro heterocycle, or nitrogen-containing fused heterocycle) groups or amino compounds. The other substrate 1 has A as defined in the compound of Formula (II) of the present disclosure. The group Z can be Br, Cl, I, OMs, OTs, OT_{f}, or other similar groups. (R_{ba})ₙ indicates that the benzene ring is substituted by n R_{ba} groups, with each R_{ba} being the same or different and independently representing deuterium, fluorine, protected hydroxy, protected thiol, nitro, protected amino, cyano, optionally deuterated C₁₋₆ alkyl, optionally deuterated C₁₋₆ alkoxy, halo C₁₋₆ alkyl, optionally substituted C₃₋₆ cycloalkyl, C₂₋₆ alkenyl, or C₂₋₆ alkynyl; and n represents an integer of 0, 1, 2, or 3.

Step 1: (1) When the group Z is OMs, OTs, or OTf, the alcohol substrate can undergo esterification with, for example, MsCl (methanesulfonyl chloride), TsCl (p-toluenesulfonyl chloride), or TfCl (trifluoromethanesulfonyl chloride) under basic conditions (such as TEA, DIEA) to obtain the corresponding target intermediate 1. Alternatively, (2) when the group Z is Br, Cl, or I, the alcohol substrate can undergo halogenation with, for example, CBr₄/PPh₃/DCM to obtain the corresponding target intermediate 1.

Step 2: to a solution of the amine substrate (1 equiv) and target intermediate 1 (1.2 equiv) in DMF was added DIEA (5 equiv). The reaction mixture was stirred at room temperature to 80°C for 0.5-24 hours. After monitoring the reaction via LCMS and confirming its completion, the reaction solution was filtered, and the filtrate was separated and purified by preparative HPLC to give the target compound.

### General synthesis method of compounds of Formula (II) 2:

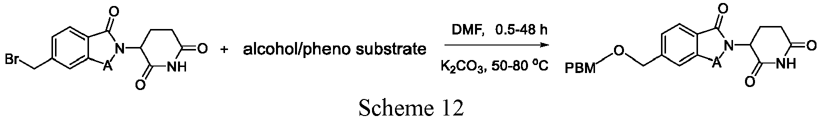

In Scheme 12, the alcohol/pheno substrate has the formula of PBM-OH, where PBM and A are as defined in the compound of Formula (II) of the present disclosure.

To a solution of the alcohol substrate (1 equiv) and 3-(5-(bromomethyl)-1-oxoisoindolin-2-yl)piperidine-2,6-dione (1.2 equiv) in DMF was added K₂CO₃ (5 equiv). The reaction mixture was stirred at 50-80°C for 0.5-48 hours. After monitoring the reaction via LCMS and confirming its completion, the reaction solution was filtered, and the filtrate was separated and purified by preparative HPLC to give the target compound.

### General synthesis method of compounds of Formula (II) 3:

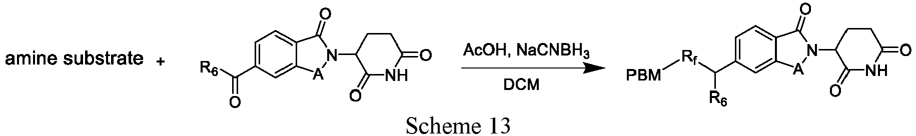

In Scheme 13, the amine substrate is a compound corresponding to the PBM-R_{f} portion of the compound of Formula (II) of the present disclosure, where R_{f} contains aliphatic amine or heterocyclic amine (such as nitrogen-containing monocyclic heterocycle, nitrogen-containing bridged heterocycle, nitrogen-containing spiro heterocycle, or nitrogen-containing fused heterocycle) groups or amino groups. A, PBM, R_{f}, and R₆ are as defined in the compound of Formula (II) of the present disclosure. It should be understood that when the R₆ group contains additional reactive groups (such as -NH or - NH₂) that can participate in the reaction of Scheme 13, the reactive groups can be protected by techniques and methods known to those skilled in the art, such as using protecting groups.

To a solution of the corresponding aldehyde or carbonyl compound (1.0 equiv) and amine substrate (1.0 equiv) in dichloromethane was added an appropriate amount of acetic acid. The resulting reaction mixture was reacted at room temperature for 30 minutes, followed by addition of sodium cyanoborohydride (3.0 equiv), and the reaction was continued at room temperature. After monitoring the reaction via LCMS and confirming its completion, the reaction mixture was separated by using C₁₈ reverse phase column chromatography to give the target compound.

### General synthesis method of intermediate compounds 6:

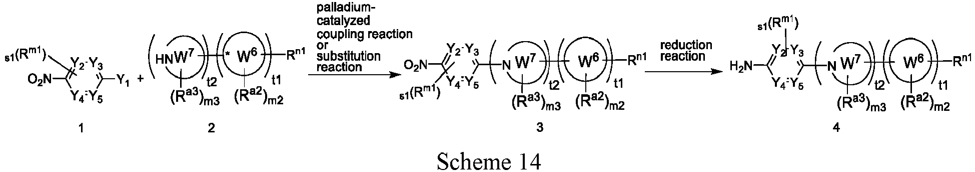

In Scheme 14, (R^{m1})ₛ₁ of compound 1 indicates that the benzene ring is optionally substituted with s1 R^{m1} groups, with each R^{m1} being the same or different and independently representing halogen, hydroxy, C₁₋₆ alkyl (such as methyl or ethyl), halo C₁₋₆ alkyl, deuterated C₁₋₆ alkyl, C₁₋₆ alkoxy (such as methoxy or isopropoxy), halo C₁₋₆ alkoxy, deuterated C₁₋₆ alkoxy, NO₂, NH₂, or cyano. s1 represents an integer of 0, 1, 2, 3, or 4. Y₁ represents halogen (such as fluorine, chlorine, or bromine), and Y₂, Y₃, Y₄, and Y₅ each independently represent N or CH, with the provision that Y₂, Y₃, Y₄, and Y₅ are not all N simultaneously. Rings W⁶, ring W⁷, t1, t2, (R^{a2})ₘ₂, (R^{a3})ₘ₃ are as defined in the compound of Formula (II) of the present disclosure. Rⁿ¹ represents Boc, H, or -NHR_{g}, where R_{g} represents hydrogen or C₁₋₆ alkyl (such as methyl or ethyl). It should be understood that when ring W⁶-Rⁿ¹ contains additional reactive groups (such as ring W⁶-Rⁿ¹ representing -NH- or -NH₂) that can participate in the reaction of Scheme 14, the reactive groups can be protected by techniques and methods known to those skilled in the art, such as using protecting groups.

Depending upon the target compound, the reaction substrate, reaction conditions (including reaction dosage, temperature, time, etc.), and work up, etc. in Scheme 14 can be appropriately modified and adjusted using techniques and methods well known to those skilled in the art to obtain the desired target compound. In Scheme 14, the substitution reaction or palladium-catalyzed coupling reaction of step 1 can be conventional techniques and methods known to those skilled in the art. When step 1 is a substitution reaction, Y₁ represents fluorine, and compounds 1 and 2 react under basic conditions (such as K₂CO₃). When step 1 is a palladium-catalyzed coupling reaction, Y₁ represents bromine, and the palladium catalyst and phosphine ligand used can be conventional ones suitable for coupling reactions. There are many types of conventional palladium catalysts for coupling reactions, such as palladium acetate (Pd(OAc)₂), tris(dibenzylideneacetone)dipalladium (Pd₂(dba)₃), diphenylphosphinoferrocene dichloropalladium, tetraphenylphosphine palladium, dichlorobis(triphenylphosphine)palladium, palladium on carbon, etc. The phosphine ligand can be a conventional ligand such as Xantphos or BINAP. The reduction reaction of step 2 in Scheme 14 can be conventional techniques and methods known to those skilled in the art, such as H₂/Pd/C or Fe/NH₄Cl/MeOH.

### General synthesis method of intermediate compounds 7:

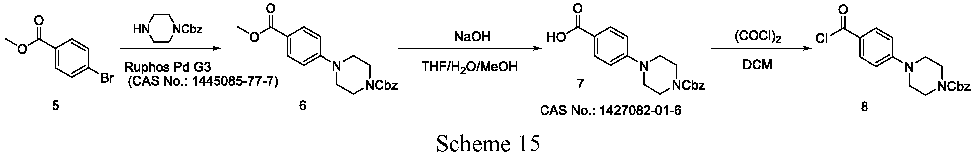

### General synthesis method of intermediate compounds 8:

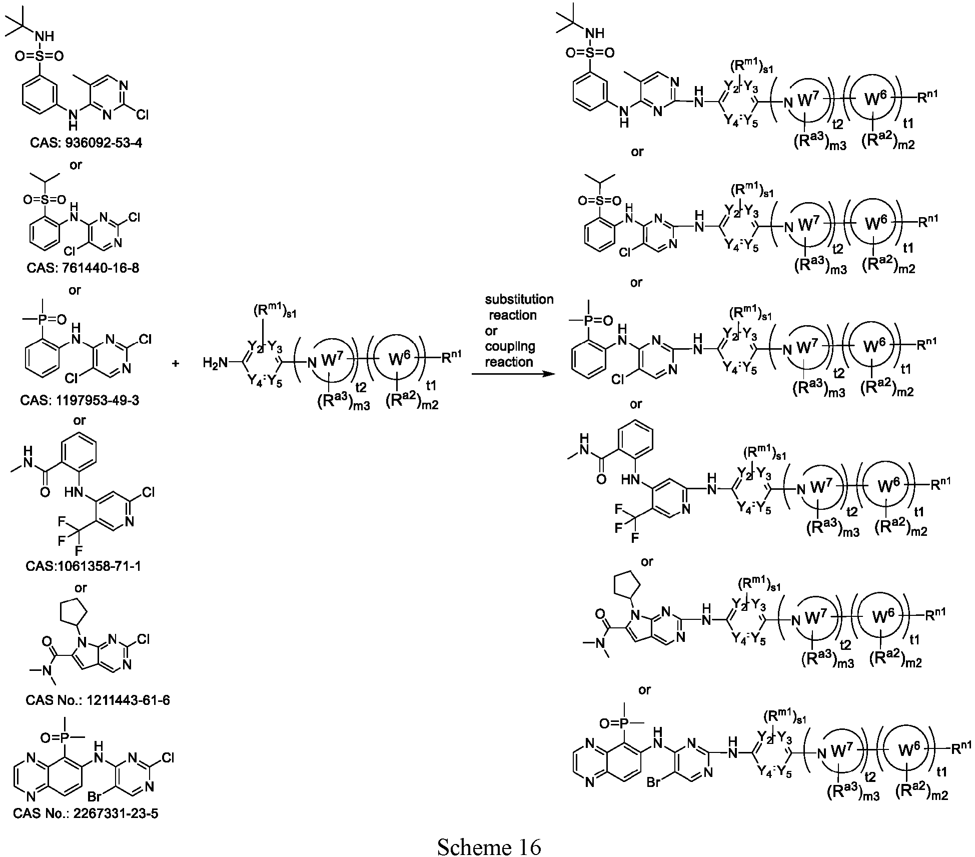

In Scheme 16, (R^{m1})ₛ₁ of the compounds indicates that the benzene ring is optionally substituted with s1 R^{m1} groups, with each R^{m1} being the same or different and independently representing halogen, hydroxy, C₁₋₆ alkyl (such as methyl or ethyl), halo C₁₋₆ alkyl, deuterated C₁₋₆ alkyl, C₁₋₆ alkoxy (such as methoxy or isopropoxy), halo C₁₋₆ alkoxy, deuterated C₁₋₆ alkoxy, NO₂, NH₂, or cyano. Y₁ represents halogen (such as fluorine, chlorine, or bromine), and Y₂, Y₃, Y₄, and Y₅ each independently represent N or CH, with the proviso that Y₂, Y₃, Y₄, and Y₅ are not all N at the same time. Rings W⁶, W⁷, t1, t2, (R^{a2})ₘ₂, (R^{a3})ₘ₃ are as defined in the compound of Formula (II) of the present disclosure. Rⁿ¹ represents Boc, H, or -NHR_{g}, where R_{g} represents hydrogen or C₁₋₆ alkyl (such as methyl or ethyl). It should be understood that when ring W⁶-Rⁿ¹ contains additional reactive groups (such as - NH- or -NH₂) that can participate in the reaction of Scheme 16, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions such as hydrochloric acid or trifluoroacetic acid.

Depending upon the target compound, the reaction substrate, reaction conditions (including reaction dosage, temperature, time, etc.), and work up, etc. in Scheme 16 can be appropriately modified and adjusted using techniques and methods well known to those skilled in the art to obtain the desired target compound. In Scheme 16, the substitution reaction or palladium-catalyzed coupling reaction can be conventional techniques and methods known to those skilled in the art. For example, substitution reactions can be carried out under heating conditions in the presence of triethylamine/isopropanol, p-toluenesulfonic acid/n-butanol, DIEA/ethanol, NaHMDS, or NaI/DIEA/DMF; coupling reactions can be performed under heating conditions in the presence of Brettphos Pd G3/Brettphos ligand/Cs₂CO₃.

### General synthesis method of intermediate compounds 9:

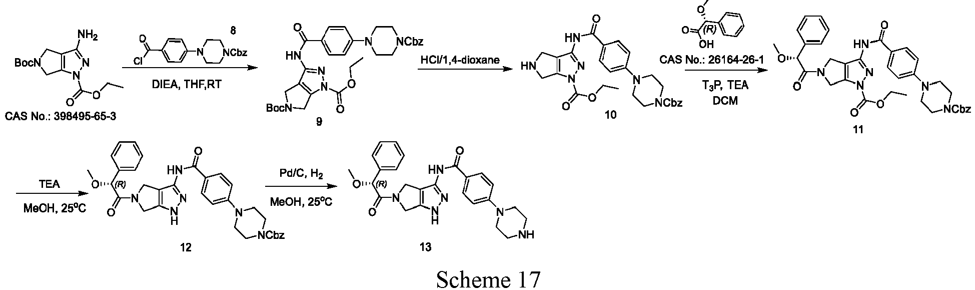

The compound 8 in Step 1 of Scheme 17 can be prepared by referring to Scheme 15.

### General synthesis method of intermediate compounds 10:

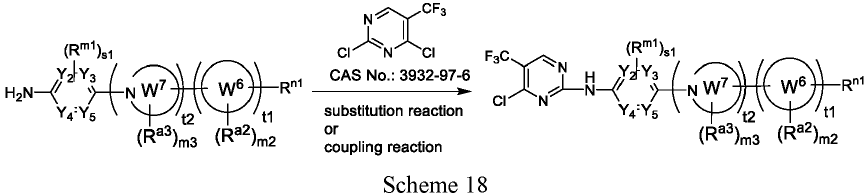

In Scheme 18, (R^{m1})ₛ₁ of the compounds indicates that the benzene ring is optionally substituted with s1 R^{m1} groups, with each R^{m1} being the same or different and independently representing halogen, hydroxy, C₁₋₆ alkyl (such as methyl or ethyl), halo C₁₋₆ alkyl, deuterated C₁₋₆ alkyl, C₁₋₆ alkoxy (such as methoxy or isopropoxy), halo C₁₋₆ alkoxy, deuterated C₁₋₆ alkoxy, NO₂, NH₂, or cyano. s1 represents an integer of 0, 1, 2, 3, or 4. Y₁ represents halogen (such as fluorine, chlorine, or bromine), and Y₂, Y₃, Y₄, and Y₅ each independently represent N or CH, with the proviso that Y₂, Y₃, Y₄, and Y₅ are not all N at the same time. Rings W⁶, W⁷, t1, t2, (R^{a2})ₘ₂, (R^{a3})ₘ₃ are as defined in the compound of Formula (II) of the present disclosure. Rⁿ¹ represents Boc, H, or -NHR_{g}, where R_{g} represents hydrogen or C₁₋₆ alkyl (such as methyl or ethyl). It should be understood that when ring W⁶-Rⁿ¹ contains additional reactive groups (such as -NH- or -NH₂) that can participate in the reaction of Scheme 18, the reactive groups can be protected by techniques and methods known to those skilled in the art, such as using protecting groups.

Depending upon the target compound, the reaction substrate, reaction conditions (including reaction dosage, temperature, time, etc.), and work up, etc. in Scheme 18 can be appropriately modified and adjusted using techniques and methods well known to those skilled in the art to obtain the desired target compound. In Scheme 18, the substitution reaction or palladium-catalyzed coupling reaction can be conventional techniques and methods known to those skilled in the art. For example, substitution reactions can be carried out under heating conditions in the presence of triethylamine/isopropanol, DIEA/ethanol, NaHMDS, or NaI/DIEA/DMF; coupling reactions can be performed under heating conditions in the presence of Brettphos Pd G3/Brettphos ligand/Cs₂CO₃.

### General synthesis method of intermediate compounds 11:

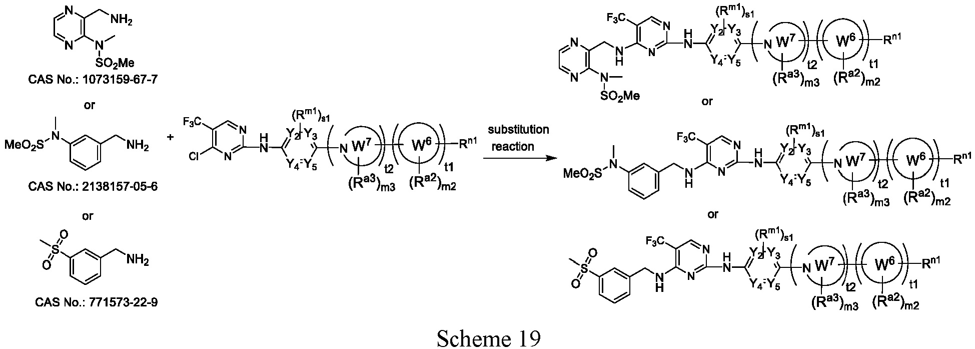

In Scheme 19, (R^{m1})ₛ₁ of the compounds indicates that the benzene ring is optionally substituted with s1 R^{m1} groups, with each R^{m1} being the same or different and independently representing halogen, hydroxy, C₁₋₆ alkyl (such as methyl or ethyl), halo C₁₋₆ alkyl, deuterated C₁₋₆ alkyl, C₁₋₆ alkoxy (such as methoxy or isopropoxy), halo C₁₋₆ alkoxy, deuterated C₁₋₆ alkoxy, NO₂, NH₂, or cyano. Y₁ represents halogen (such as fluorine, chlorine, or bromine), and Y₂, Y₃, Y₄, and Y₅ each independently represent N or CH, with the proviso that Y₂, Y₃, Y₄, and Y₅ are not all N at the same time. Rings W⁶, W⁷, t1, t2, (R^{a2})ₘ₂, (R^{a3})ₘ₃ are as defined in the compound of Formula (II) of the present disclosure. Rⁿ¹ represents Boc, H, or -NHR_{g}, where R_{g} represents hydrogen or C₁₋₆ alkyl (such as methyl or ethyl). It should be understood that when ring W⁶-Rⁿ¹ contains additional reactive groups (such as - NH- or -NH₂) that can participate in the reaction of Scheme 19, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions such as hydrochloric acid or trifluoroacetic acid.

Depending upon the target compound, the reaction substrate, reaction conditions (including reaction dosage, temperature, time, etc.), and work up, etc. in Scheme 19 can be appropriately modified and adjusted using techniques and methods well known to those skilled in the art to obtain the desired target compound. In Scheme 19, the substitution reaction can be conventional techniques and methods known to those skilled in the art. For example, substitution reactions can be carried out under heating conditions in the presence of triethylamine/isopropanol, DIEA/ethanol, or NaI/DIEA/DMF.

### General synthesis method of intermediate compounds 12:

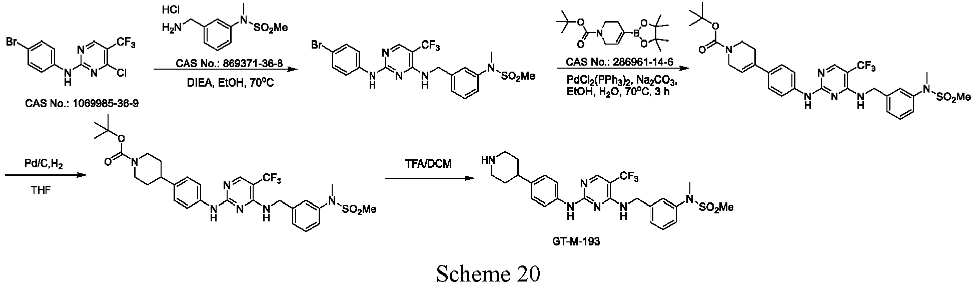

### General synthesis method of intermediate compounds 13:

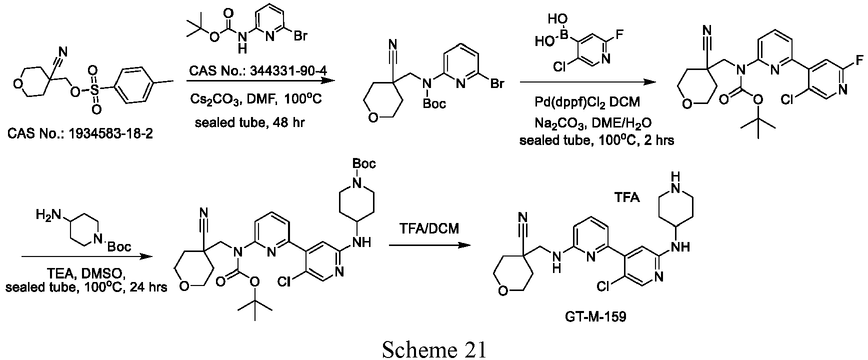

### General synthesis method of intermediate compounds 14:

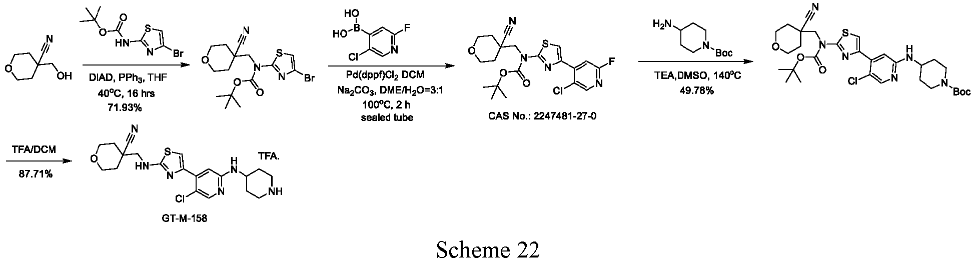

### General synthesis method of intermediate compounds 15:

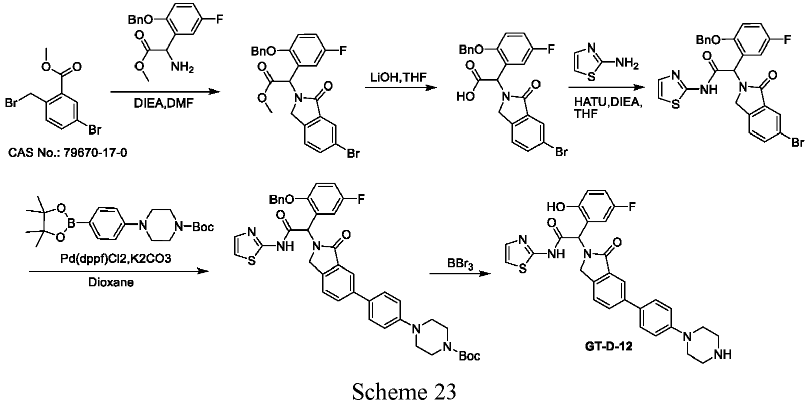

### General synthesis method of intermediate compounds 16:

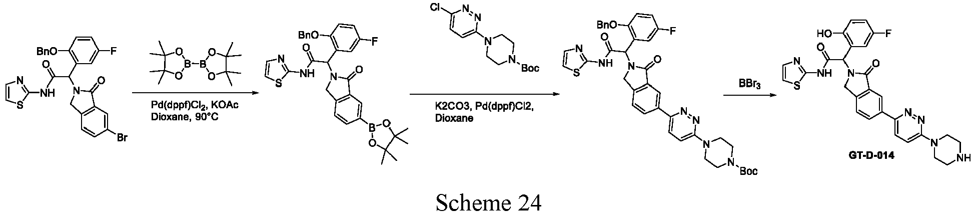

### General synthesis method of intermediate compounds 17:

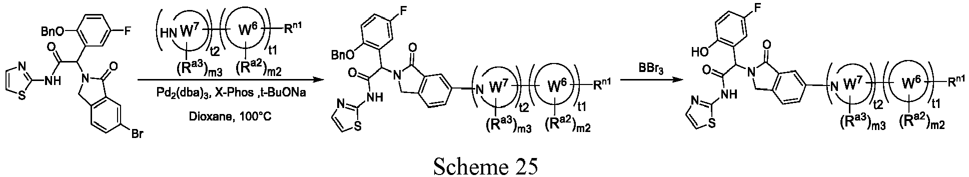

In Scheme 25, the rings W⁶, rings W⁷, t1, t2, (R^{a2})ₘ₂, (R^{a3})ₘ₃ are as defined in the compound of Formula (II) of the present disclosure. Rⁿ¹ represents Boc, H, or -NHR_{g}, where R_{g} represents hydrogen or C₁₋₆ alkyl (such as methyl or ethyl). It should be understood that when ring W⁶-Rⁿ¹ contains additional reactive groups (such as ring W⁶-Rⁿ¹ representing -NH- or -NH₂) that can participate in the reaction of Scheme 25, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions such as hydrochloric acid or trifluoroacetic acid.

Depending upon the target compound, the reaction substrate, reaction conditions (including reaction dosage, temperature, time, etc.), and work up, etc. in Scheme 25 can be appropriately modified and adjusted using techniques and methods well known to those skilled in the art to obtain the desired target compound.

### General synthesis method of intermediate compounds 18:

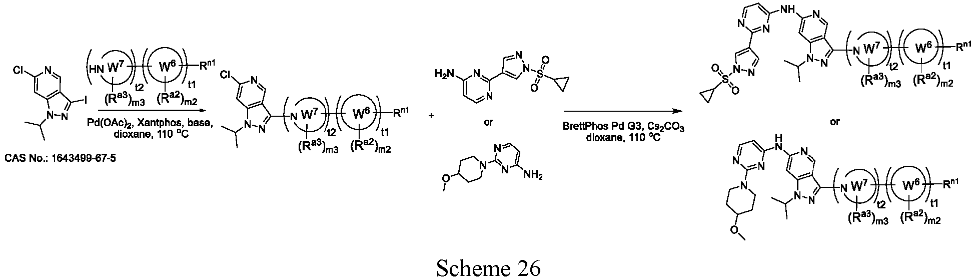

In Scheme 26, the rings W⁶, rings W⁷, t1, t2, (R^{a2})ₘ₂, (R^{a3})ₘ₃ are as defined in the compound of Formula (II) of the present disclosure. Rⁿ¹ represents Boc, H, or -NHR_{g}, where R_{g} represents hydrogen or C₁₋₆ alkyl (such as methyl or ethyl). It should be understood that when ring W⁶-Rⁿ¹ contains additional reactive groups (such as -NH- or -NH₂) that can participate in the reaction of Scheme 26, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions such as hydrochloric acid or trifluoroacetic acid.

### General synthesis method of intermediate compounds 19:

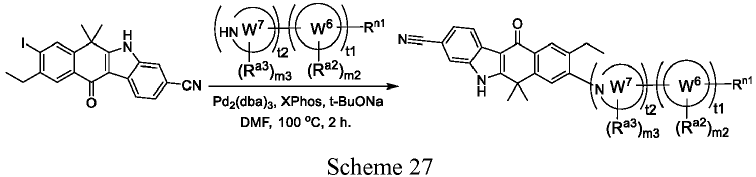

In Scheme 27, the rings W⁶, rings W⁷, t1, t2, (R^{a2})ₘ₂, (R^{a3})ₘ₃ are as defined in the compound of Formula (II) of the present disclosure. Rⁿ¹ represents Boc, H, or -NHR_{g}, where R_{g} represents hydrogen or C₁₋₆ alkyl (such as methyl or ethyl). It should be understood that when ring W⁶-Rⁿ¹ contains additional reactive groups (such as -NH- or -NH₂) that can participate in the reaction of Scheme 27, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions such as hydrochloric acid or trifluoroacetic acid.

### General synthesis method of intermediate compounds 20:

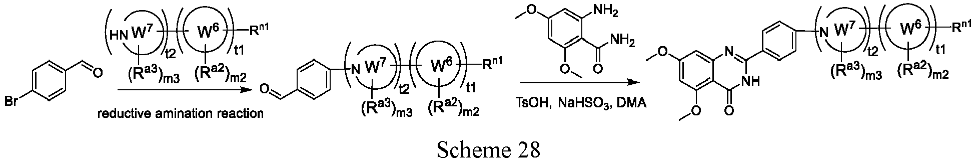

In Scheme 28, the rings W⁶, rings W⁷, t1, t2, (R^{a2})ₘ₂, (R^{a3})ₘ₃ are as defined in the compound of Formula (II) of the present disclosure. Rⁿ¹ represents Boc, H, or -NHR_{g}, where R_{g} represents hydrogen or C₁₋₆ alkyl (such as methyl or ethyl). It should be understood that when ring W⁶-Rⁿ¹ contains additional reactive groups (such as -NH- or -NH₂) that can participate in the reaction of Scheme 28, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions such as hydrochloric acid or trifluoroacetic acid.

Depending upon the target compound, the reaction substrate, reaction conditions (including reaction dosage, temperature, time, etc.), and work up, etc. in Scheme 28 can be appropriately modified and adjusted using techniques and methods well known to those skilled in the art to obtain the desired target compound. In Scheme 28, the reductive amination reaction can follow conventional techniques and methods known to the skilled in the art. For example, the reductive amination reaction can be carried out at room temperature in the presence of sodium triacetoxyborohydride/1,2-dichloroethane, or sodium cyanoborohydride/1,2-dichloroethane.

### General synthesis method of intermediate compounds 21:

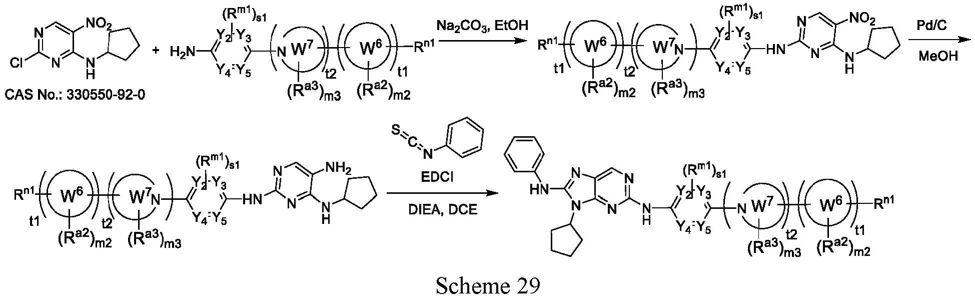

In Scheme 29, (R^{m1})ₛ₁ of compounds indicates that the benzene ring is optionally substituted with s1 R^{m1} groups, with each R^{m1} being the same or different and independently representing halogen, hydroxy, C₁₋₆ alkyl (such as methyl or ethyl), halo C₁₋₆ alkyl, deuterated C₁₋₆ alkyl, C₁₋₆ alkoxy (such as methoxy or isopropoxy), halo C₁₋₆ alkoxy, deuterated C₁₋₆ alkoxy, NO₂, NH₂, or cyano. Y₁ represents halogen (such as fluorine, chlorine, or bromine), and Y₂, Y₃, Y₄, and Y₅ each independently represent N or CH, with the provision that Y₂, Y₃, Y₄, and Y₅ are not all N simultaneously. Rings W⁶, ring W⁷, t1, t2, (R^{a2})ₘ₂, (R^{a3})ₘ₃ are as defined in the compound of Formula (II) of the present disclosure. Rⁿ¹ represents Boc, H, or -NHR_{g}, where R_{g} represents hydrogen or C₁₋₆ alkyl (such as methyl or ethyl). It should be understood that when ring W⁶-Rⁿ¹ contains additional reactive groups (such as ring W⁶-Rⁿ¹ representing -NH- or -NH₂) that can participate in the reaction of Scheme 29, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions such as hydrochloric acid or trifluoroacetic acid.

### General synthesis method of intermediate compounds 22:

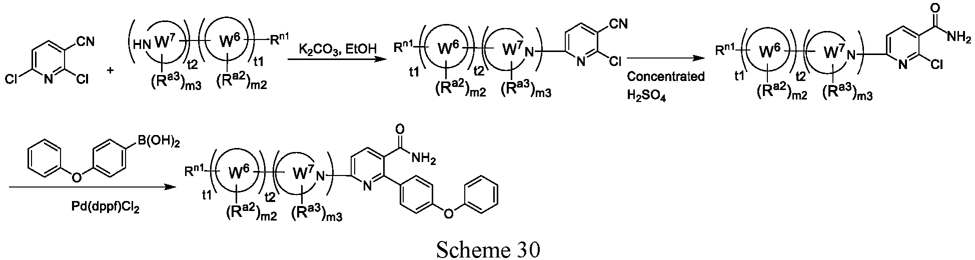

In Scheme 30, the rings W⁶, W⁷, t1, t2, (R^{a2})ₘ₂, (R^{a3})ₘ₃ are as defined in the compound of Formula (II) of the present disclosure. Rⁿ¹ represents Boc, H, or -NHR_{g}, where R_{g} represents hydrogen or C₁₋₆ alkyl (such as methyl or ethyl). It should be understood that when ring W⁶-Rⁿ¹ of the strating material of step 1 of Scheme 30 contains additional reactive groups (such as -NH- or -NH₂) that can participate in the reaction of step 1, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions of step 2 such as hydrochloric acid or trifluoroacetic acid.

### General synthesis method of intermediate compounds 23:

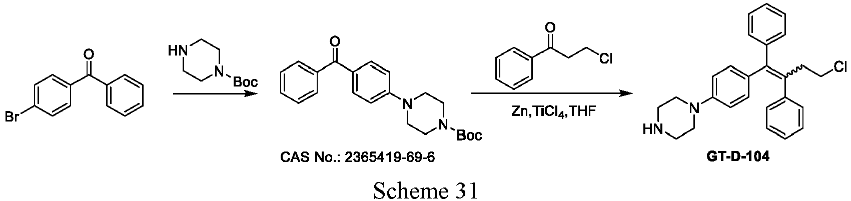

### General synthesis method of intermediate compounds 24:

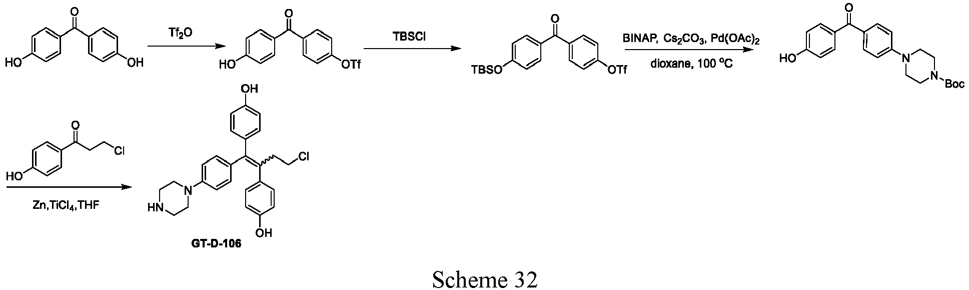

### General synthesis method of intermediate compounds 25:

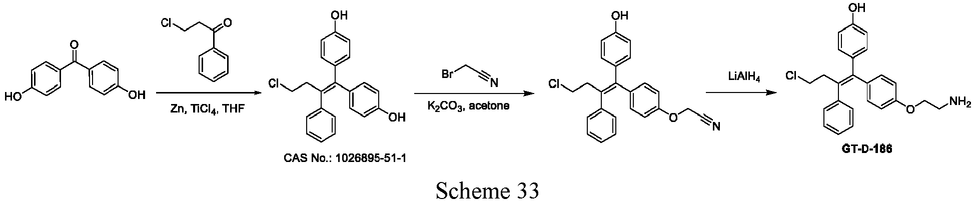

### General synthesis method of intermediate compounds 26:

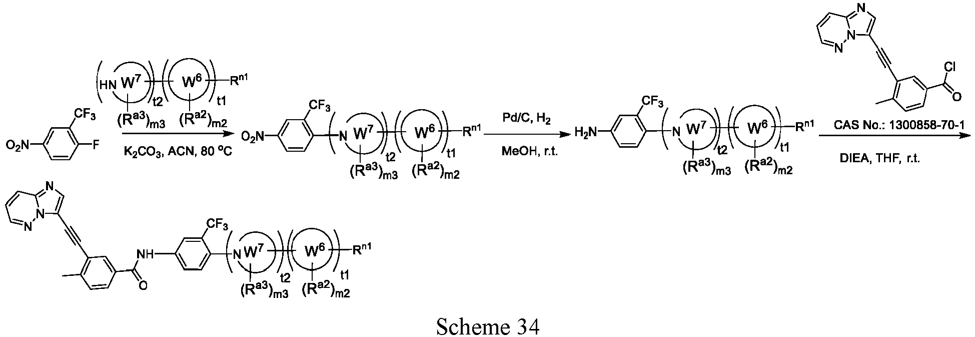

In Scheme 34, the compound's rings W⁶, W⁷, t1, t2, (R^{a2})ₘ₂, (R^{a3})ₘ₃ are as defined in the compound of Formula (II) of the present disclosure. Rⁿ¹ represents Boc, H, or -NHR_{g}, where R_{g} represents hydrogen or C₁₋₆ alkyl (such as methyl or ethyl). It should be understood that when ring W⁶-Rⁿ¹ contains additional reactive groups (such as -NH- or -NH₂) that can participate in the reaction, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions such as concentrated sulfuric acid.

### General synthesis method of intermediate compounds 27:

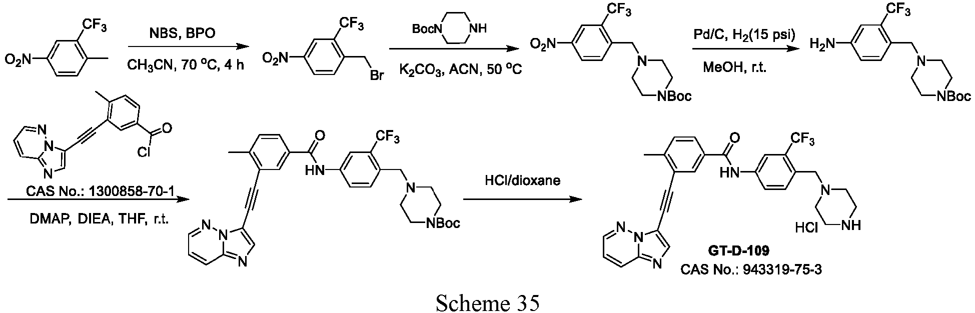

### General synthesis method of intermediate compounds 28:

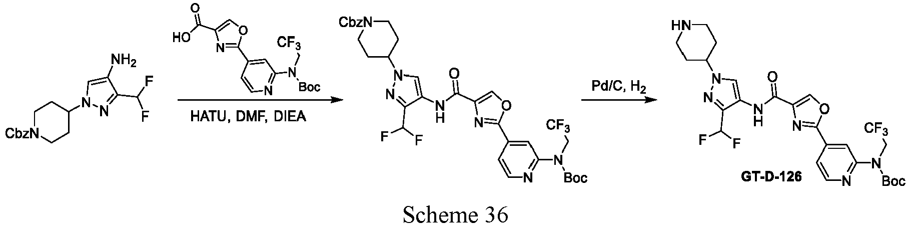

### General synthesis method of intermediate compounds 29:

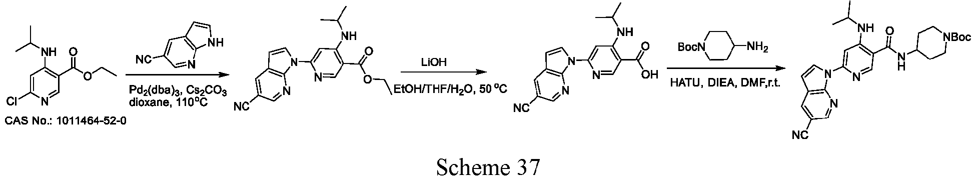

The removal of protecting groups can be achieved through techniques and methods known to those skilled in the art. For example, the removal of the Boc protecting group can be achieved under acidic conditions such as concentrated sulfuric acid.

### General synthesis method of intermediate compounds 30:

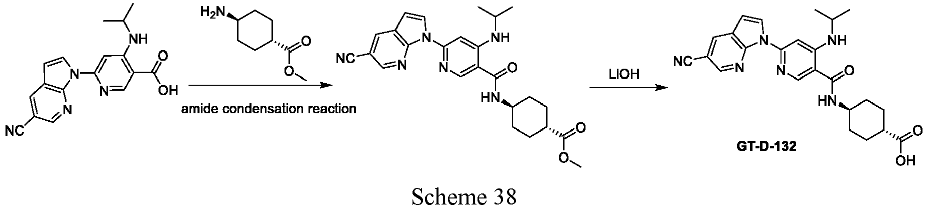

Depending upon the target compound, the reaction substrate, reaction conditions (including reaction dosage, temperature, time, etc.), and work up, etc. in Scheme 38 can be appropriately modified and adjusted using techniques and methods well known to those skilled in the art to obtain the desired target compound. In Scheme 38, the amide condensation reaction can be conventional techniques and methods known to those skilled in the art. For example, the amide condensation reaction can be carried out at room temperature in the presence of HATU/DIEA/DMF or HOAt/EDCI/TEA/DCM.

### General synthesis method of intermediate compounds 31:

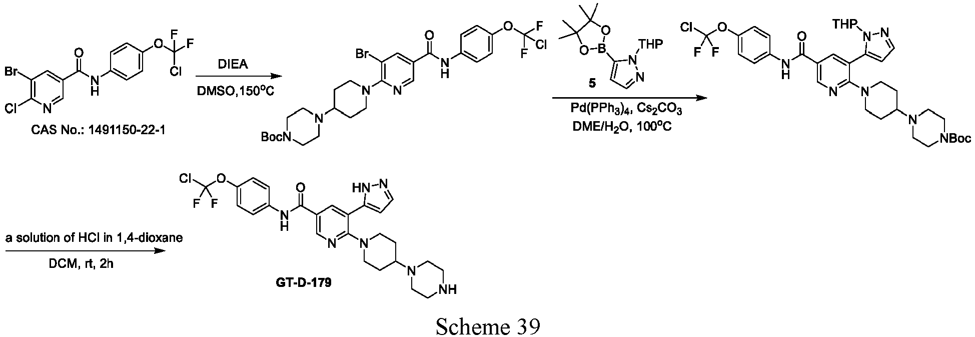

### General synthesis method of intermediate compounds 32:

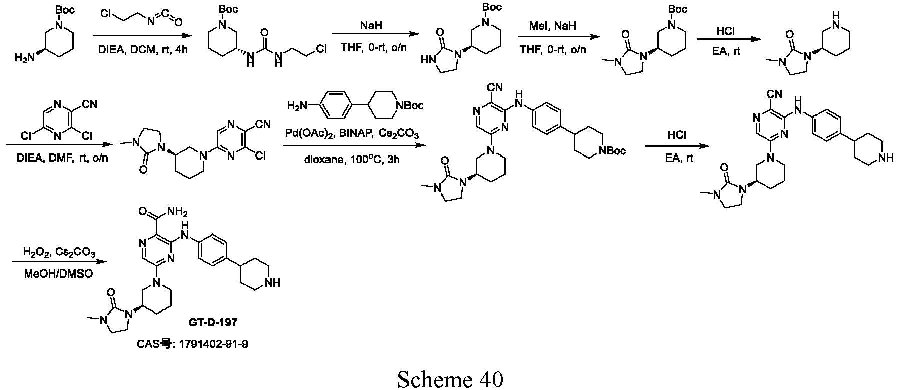

### General synthesis method of intermediate compounds 33:

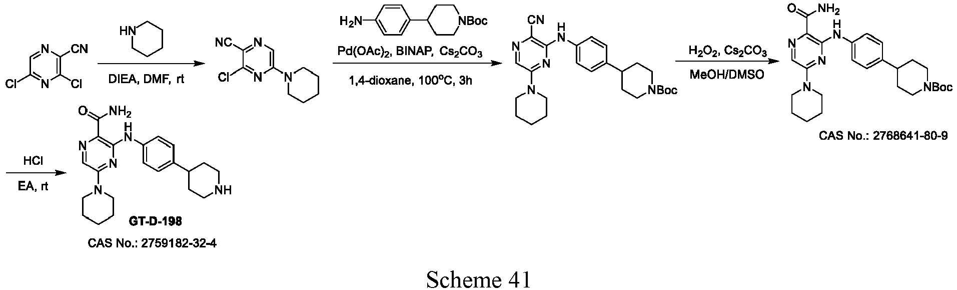

### General synthesis method of intermediate compounds 34:

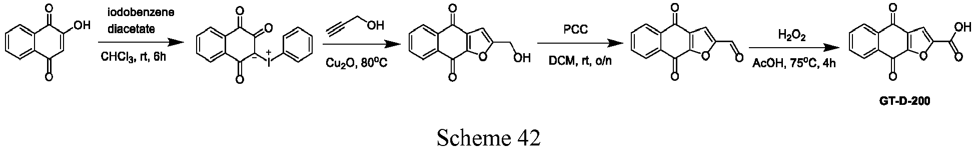

### General synthesis method of intermediate compounds 35:

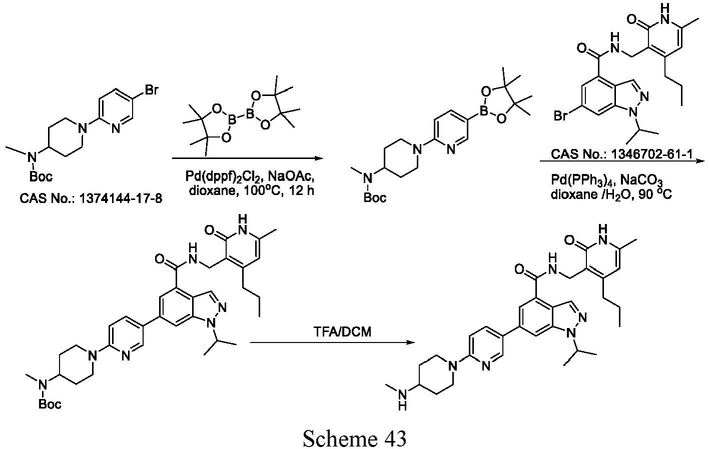

### General synthesis method of intermediate compounds 36:

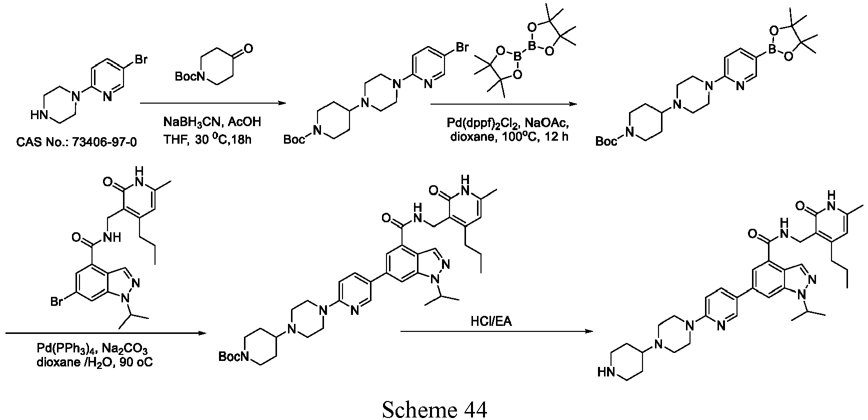

### General synthesis method of intermediate compounds 37:

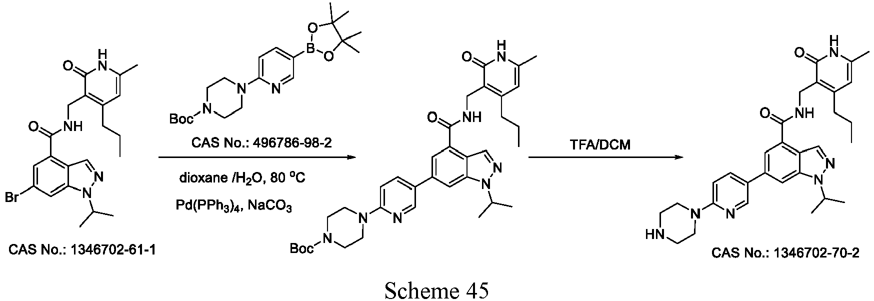

### General synthesis method of intermediate compounds 38:

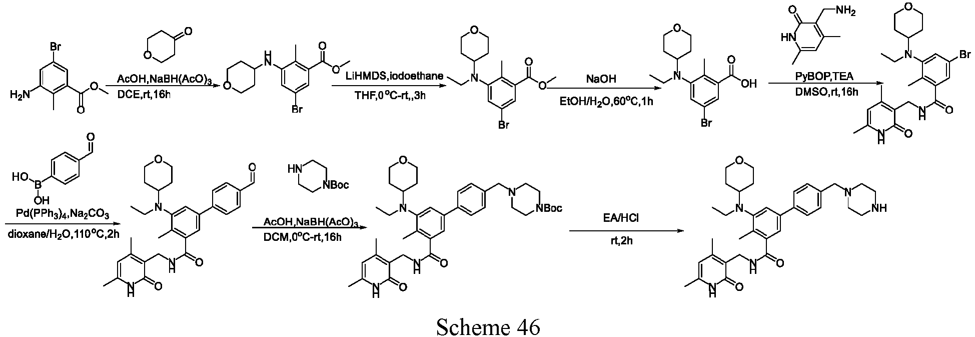

### General synthesis method of intermediate compounds 39:

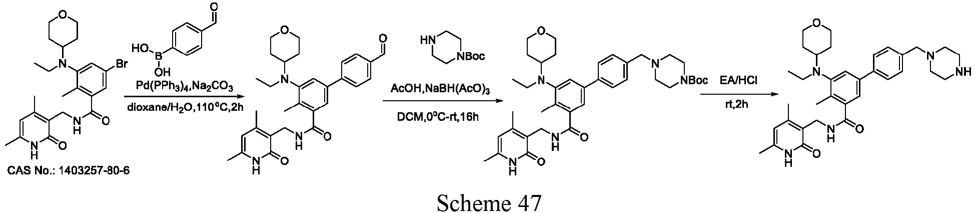

### General synthesis method of intermediate compounds 40:

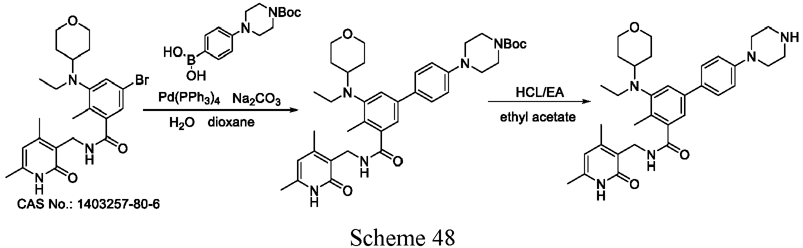

### General synthesis method of intermediate compounds 41:

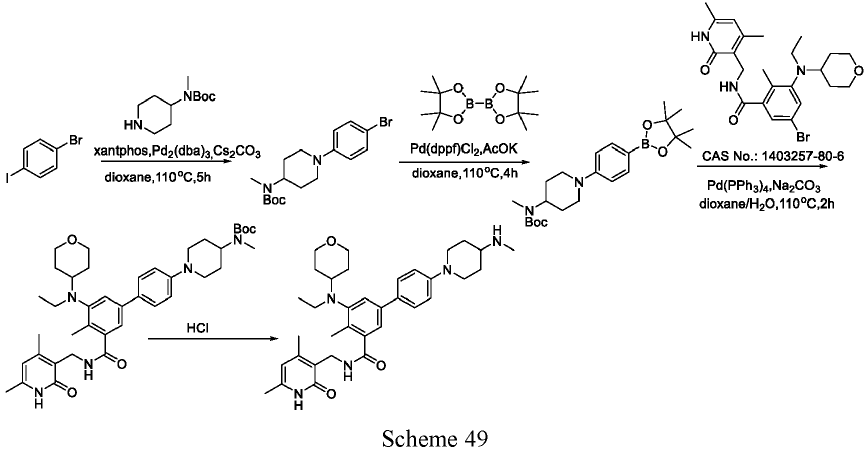

### General synthesis method of intermediate compounds 42:

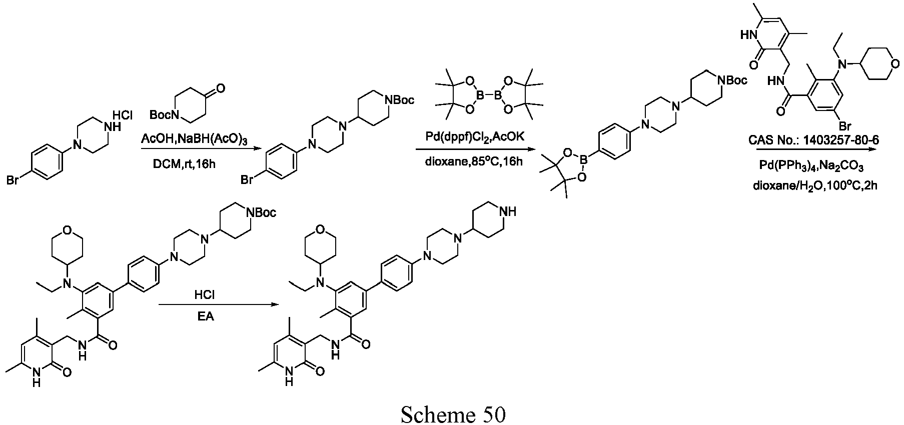

### General synthesis method of intermediate compounds 43:

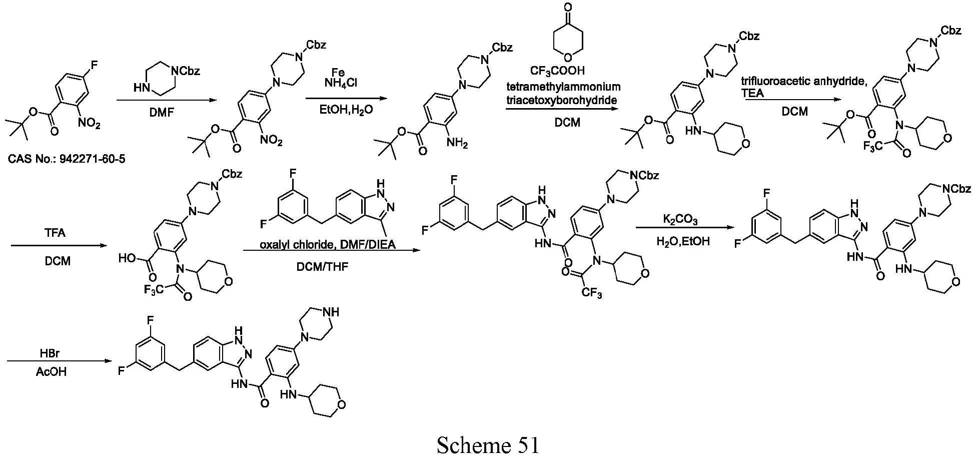

### General synthesis method of intermediate compounds 44:

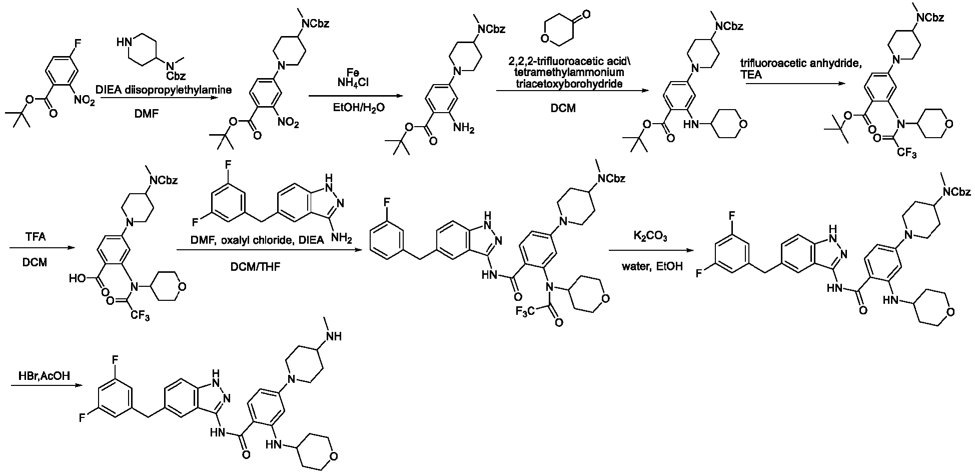

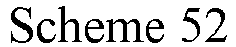

### General synthesis method of intermediate compounds 45:

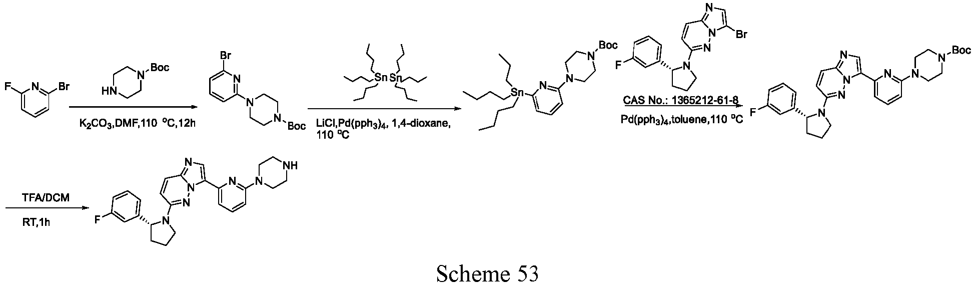

### General synthesis method of intermediate compounds 46:

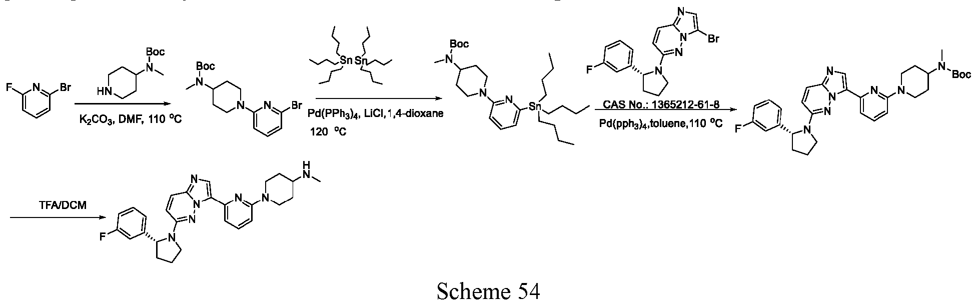

### General synthesis method of intermediate compounds 47:

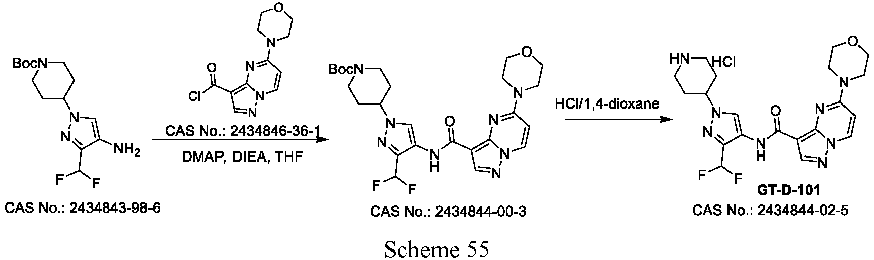

### General synthesis method of compounds of Formula (II) 4:

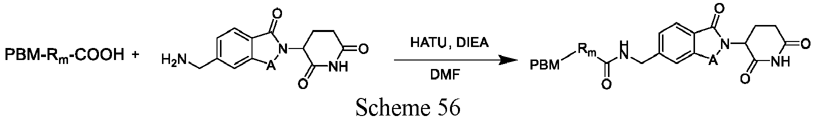

The Rₘ-C(O)NH- portion of the target product in Scheme 56 corresponds to the R_{f} portion of the compound of Formula (II) of the present disclosure, wherein R_{f}, A, and PBM are defined as in the compound of Formula (II). The condensation reaction in Scheme 56 can follow conventional techniques and methods that are well-known to those skilled in the art. For example, it can be carried out using conventional condensing agents such as HOAt/EDCI, T₃P, etc., under alkaline conditions (e.g., in the presence of DMAP) at room temperature or under heating.

### General synthesis method of intermediate compounds 48:

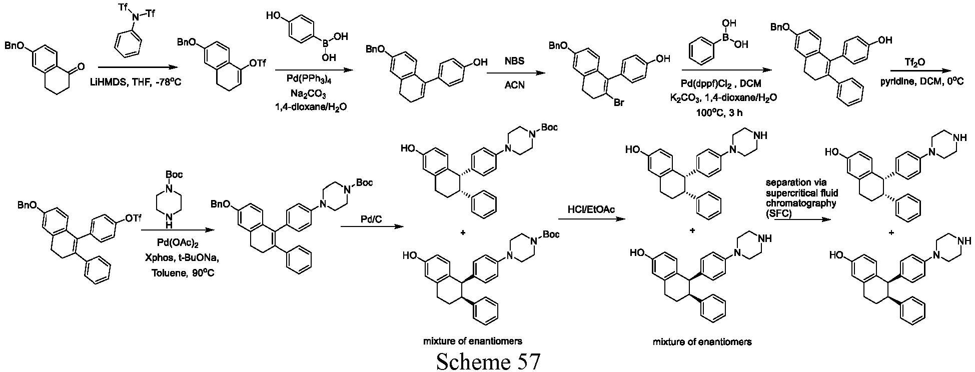

Pure enantiomeric products can be obtained through resolution and separation via supercritical fluid chromatography (SFC) according to Scheme 57. The conditions of supercritical fluid chromatography (SFC) can be appropriately modified and adjusted using techniques and methods well-known to those skilled in the art to obtain the desired target compound.

### General synthesis method of intermediate compounds 49:

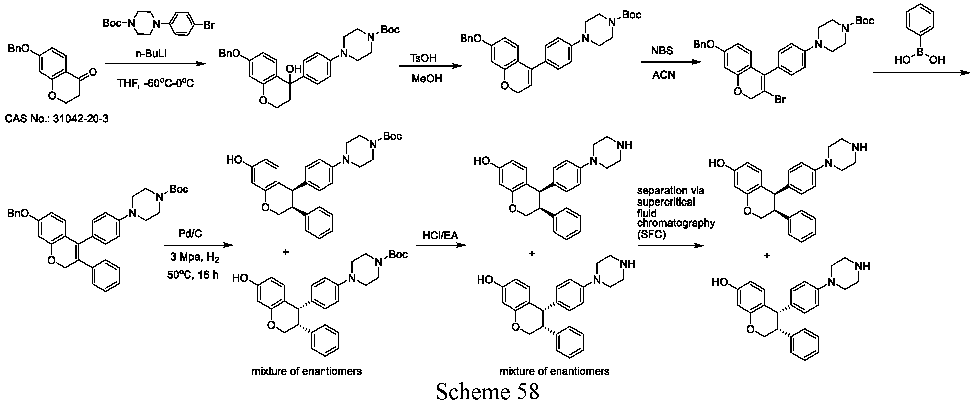

Pure enantiomeric products can be obtained through resolution and separation via supercritical fluid chromatography (SFC) according to Scheme 58. The conditions of supercritical fluid chromatography (SFC) can be appropriately modified and adjusted by techniques and methods familiar to experts in the field to achieve the desired target compound.

### General synthesis method of compounds of Formula (II) 5:

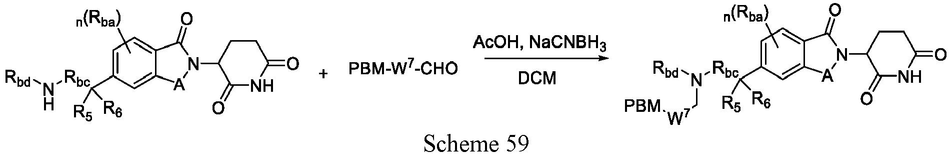

In Scheme 59, for the substrate amine's R_{bd}-NH-R_{bc}-, the group R_{bd} represents hydrogen, and R_{bc} represents a nitrogen-containing heterocyclyl. Alternatively, R_{bd} and R_{bc}, together with the nitrogen atom they are attached to, form a 4- to 15-membered monocyclic, spiro, or bridged heterocyclic group or a nitrogen-containing fused heterocyclic group containing 1-2 nitrogen atoms. A is defined as in the compound of Formula (II) of the present disclosure. R₅ and R₆ are defined as in the compound of Formula (I) of the present disclosure. (R_{ba})ₙ indicates that the benzene ring is substituted with n (R_{ba})ₙ groups, with each(R_{ba})ₙ being the same or different and independently representing deuterium, fluorine, a protected hydroxy group, a protected thiol group, a nitro group, a protected amino group, a cyano group, an optionally deuterated C₁₋₆ alkyl group, an optionally deuterated C₁₋₆ alkoxy group, a halogenated C₁₋₆ alkyl group, an optionally substituted C₃₋₆ cycloalkyl group, a C₂₋₆ alkenyl group, or a C₂₋₆ alkynyl group. n represents an integer of 0, 1, 2, or 3. The groups PBM and W⁷ of the other aldehyde substrate are defined as in the compound of Formula (II) of the present disclosure.

The reductive amination reaction in Scheme 59 can follow conventional techniques and methods that are known to those skilled in the art. For example, the reductive amination reaction can be carried out at temperatures ranging from room temperature to 80°C in the presence of sodium borohydride acetate and 1,2-dichloroethane.

For example, to a solution of the corresponding aldehyde (1.0 equivalent) and amine (1.0 equivalent) in dichloromethane was added an appropriate amount of acetic acid. The resulting reaction mixture was allowed to react at room temperature for 30 minutes, followed by the addition of sodium cyanoborohydride (3.0 equivalents). The reaction was then continued at room temperature until completion, as monitored by LCMS. After the reaction was complete, the product was separated via C₁₈ reverse-phase column chromatography. The collected fractions were rotary evaporated to remove acetonitrile, and the resulting residue was lyophilized to give the target compound.

### General synthesis method of intermediate compounds 50:

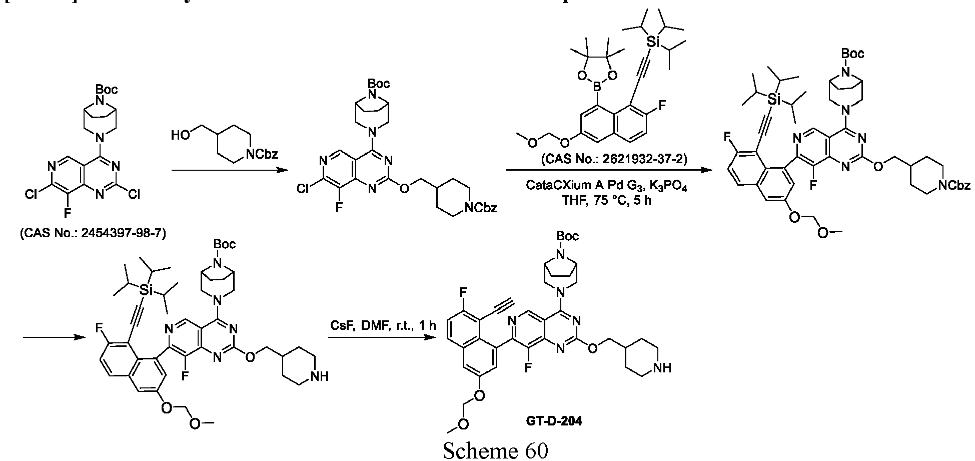

### General synthesis method of intermediate compounds 51:

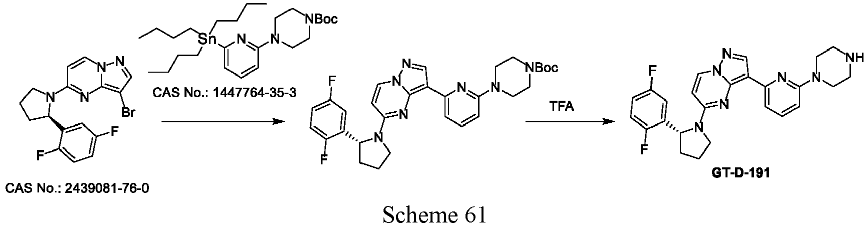

### General synthesis method of intermediate compounds 52:

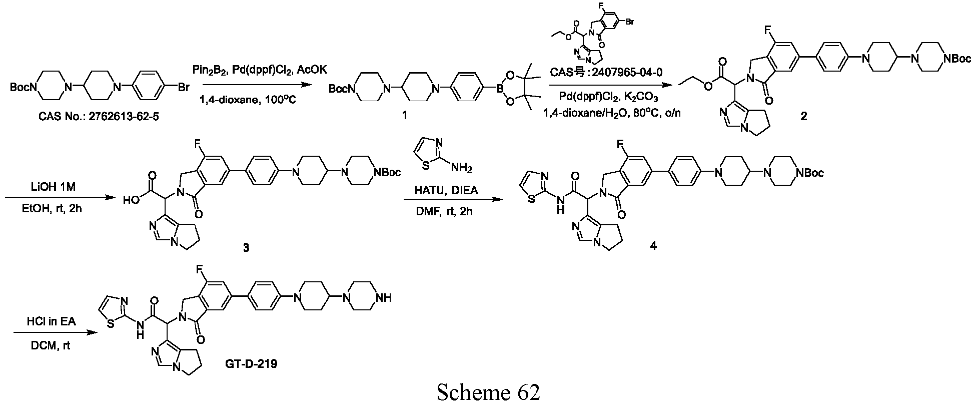

### General synthesis method of intermediate compounds 53:

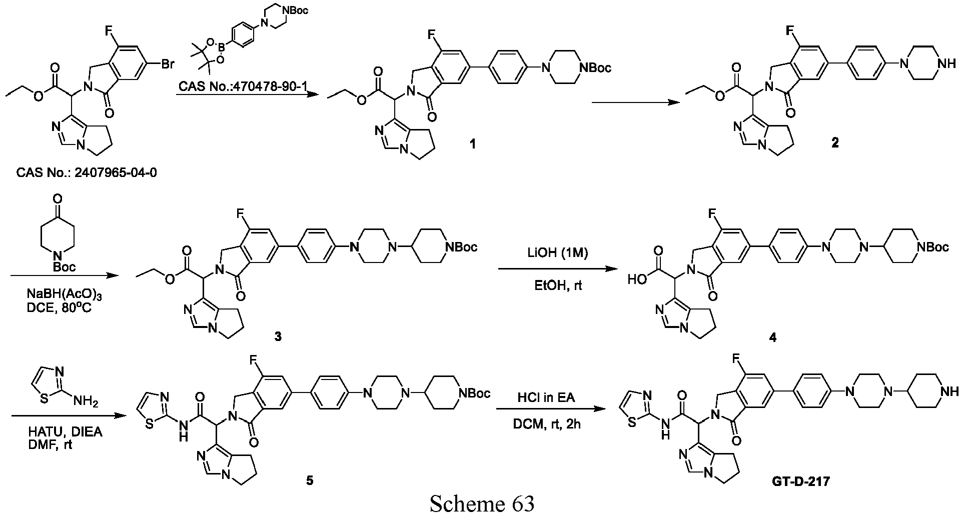

### General synthesis method of intermediate compounds 54:

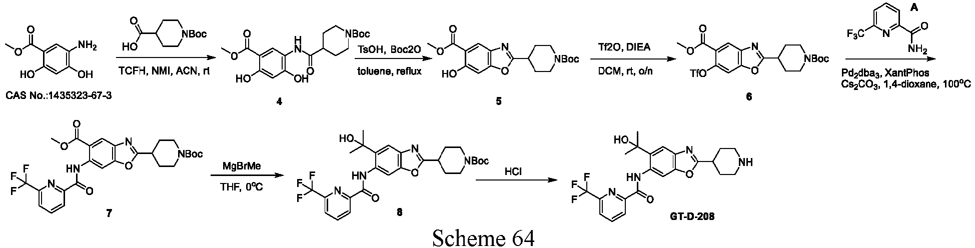

### General synthesis method of intermediate compounds 55:

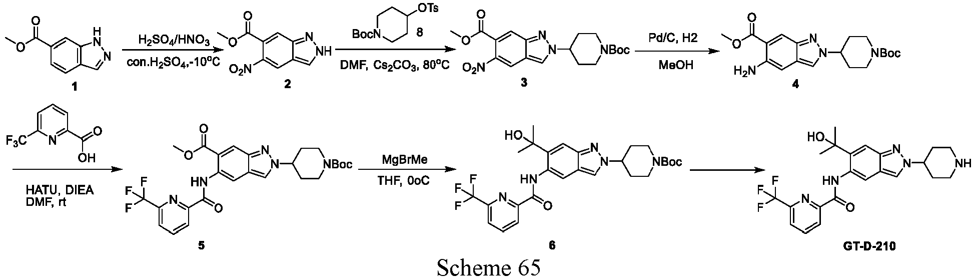

### General synthesis method of intermediate compounds 56:

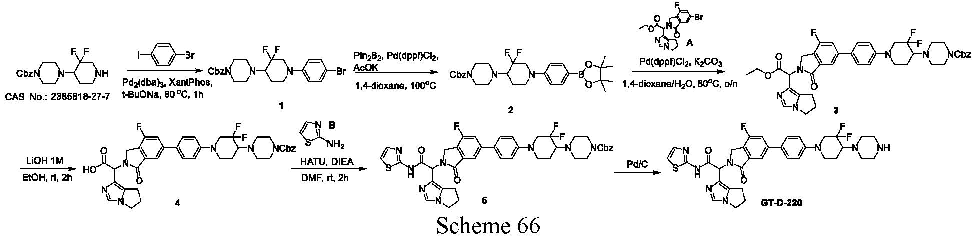

Depending upon the target compounds, the above schemes and their reaction substrates, reaction conditions (including reaction dosage, temperature, duration, etc.), work up, etc. can be appropriately modified and adjusted by techniques and methods well known to those skilled in the art to obtain the desired target compounds. The obtained target compounds can be further modified by the substituents and the like to obtain subsequent target compounds through methods well known to those skilled in the art.

### Examples

### Intermediate Example 1: preparation of 1-((3S,4R)-3-fluoropiperidin-4-yl)-3-(4-phenoxyphenyl)-1H-pyrazolo[3,4-d]pyrimidin-4-amine

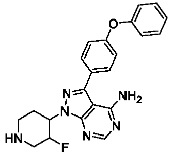

1-((3S,4R)-3-fluoropiperidin-4-yl)-3-(4-phenoxyphenyl)-1H-pyrazolo[3,4-d]pyrimidin-4-amine was prepared by referring to the method of Scheme 1.

**Step 1:** to a solution of tert-butyl (3S,4S)- 3-fluoro-4-hydroxypiperidine-1-carboxylate (370 mg, 1.69 mmol) in anhydrous dichloromethane were added DIEA (0.5 ml, 3.38 mmol), p-toluenesulfonyl chloride (386 mg, 2.03 mmol), and DMAP (206.16 mg, 1.69 mmol). The reaction mixture was stirred at room temperature for 18 hours, and the reaction was completed as detected by TLC. The reaction mixture was washed with H₂O (50 mL) and extracted with DCM (50 mL × 3). The combined organic phases were dried over anhydrous sodium sulfate, filtered, and the filtrate was concentrated under reduced pressure. The residue was purified by column chromatography (eluent (v/v): PE/EtOAc = 1/1) to give the white solid compound tert-butyl (3S,4S)-3-fluoro-4-(tosyloxy)piperidine-1-carboxylate (350 mg, yield 55%).

**Step 2:** to a solution of the compound tert-butyl (3S,4S)-3-fluoro-4-(tosyloxy)piperidine-1-carboxylate (350 mg, 0.94 mmol) in anhydrous DMSO (20 mL) were added 3-(4-phenoxyphenyl)-1H-pyrazolo[3,4-d]pyrimidin-4-amine (284 mg, 0.94 mmol) and K₂CO₃ (388 mg, 2.81 mmol). The reaction mixture was heated to 85°C and stirred for 18 hours, and the reaction was completed as detected by TLC. The reaction mixture was cooled to room temperature, washed with H₂O (100 mL), and extracted with EtOAc (100 mL × 3). The combined organic phases were dried over anhydrous sodium sulfate, filtered, and the filtrate was concentrated under reduced pressure. The residue was purified by column chromatography with eluent (DCM/MeOH = 20/1) to give the white solid compound tert-butyl (3 S,4R)-4-(4-amino-3-(4-phenoxyphenyl)-1H-pyrazolo[3,4-d]pyrimidin-1-yl)-3-fluoropiperidine-1-carboxylate (350 mg, yield 74%). LC/MS (ESI, *m*/*z*) 505.3 [M + H]⁺.

**Step 3:** to a solution of the compound tert-butyl (3S,4R)-4-(4-amino-3-(4-phenoxyphenyl)-1H-pyrazolo[3,4-d]pyrimidin-1-yl)-3-fluoropiperidine-1-carboxylate (350 mg, 0.69 mmol) in anhydrous DCM (15 mL) was added HCl/dioxane (1.7 mL, 4M). The reaction mixture was stirred at room temperature for 1 hour, and the reaction was completed as detected by TLC. The reaction mixture was concentrated under reduced pressure to give the white solid target compound 1-((3S,4R)-3-fluoropiperidin-4-yl)-3-(4-phenoxyphenyl)-1H-pyrazolo[3,4-d]pyrimidin-4-amine (205 mg, yield 67%). ¹H NMR (400 MHz, DMSO) δ 8.24 (d, J = 5.6 Hz, 1H), 7.69 (d, J = 8.6 Hz, 2H), 7.44 (dd, J = 8.4, 7.6 Hz, 2H), 7.28 - 7.04 (m, 5H), 5.15 - 4.76 (m, 2H), 3.34 (d, J = 7.3 Hz, 1H), 2.97 (d, J = 12.3 Hz, 1H), 2.63 (ddd, J = 15.0, 12.2, 3.5 Hz, 2H), 2.12 (dd, J = 12.3, 4.1 Hz, 1H), 1.94 (d, J = 12.7 Hz, 1H). ¹⁹F NMR (377 MHz, DMSO) δ -185.23 (s). LC/MS (ESI, m/z) calcd for [M + H]⁺ ,405.2 found, 405.1.

### Intermediate Example 2: preparation of 3-(4-phenoxyphenyl)-1-(piperidin-4-yl)-1H-pyrazolo [3,4-d] pyrimidin-4-amine hydrochloride

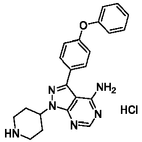

### 3-(4-phenoxyphenyl)-1-(piperidin-4-yl)-1H-pyrazolo[3,4-d]pyrimidin-4-amine hydrochloride was prepared by referring to the method of Scheme 2.

**Step 1:** to a solution of triphenylphosphine (154.59 g, 574.1 mmol) in tetrahydrofuran (3 L) was added DIAD (117.54 g, 581.3 mmol) at 0°C. After addition, the mixture was stirred for 0.5 hours. Then, to the mixture was added 2-methylpropan-2-yl 4-hydroxypiperidine-1-carboxylate (118.6 g, 589.3 mmol) at 0°C, and the mixture was stirred for 0.5 hours. Subsequently, to the reaction solution was added 3-[4-(phenoxy)phenyl]-1H-pyrazolo[3,4-d]pyrimidin-4-amine (90 g, 296.7 mmol), and the temperature was slowly raised to 25°C and stirred for 16 hours. TLC analysis showed the completion of the reaction. The reaction mixture was concentrated under vacuum to obtain the crude product. The crude product was purified by silica gel flash column chromatography (eluent (v/v): PE/EtOAc = 1/0-1/1) to obtain the white solid compound tert-butyl 4-(4-amino-3-(4-phenoxyphenyl)-1H-pyrazolo[3,4-d]pyrimidin-1-yl)piperidine-1-carboxylate (92.0 g, 189.3 mmol, yield 63.8%). LCMS (ESI): calcd. 487.24; found, [M+H]+=487.30.

**Step 2:** tert-butyl 4-(4-amino-3-(4-phenoxyphenyl)-1H-pyrazolo[3,4-d]pyrimidin-1-yl)piperidine-1-carboxylate (92.0 g, 189.3 mmol) was dissolved in HCl/EtOAc (340 mL, 3 M) at 10°C. The mixture was stirred at room temperature for 3 hours. TLC analysis showed the completion of the reaction. A large amount of white solid precipitated, which was filtered and ground with ethyl acetate/petroleum ether (1: 8, 300 ml) and filtered again. The solid was dried under high vacuum to obtain the white solid compound 4-(4-amino-3-(4-phenoxyphenyl)-1H-pyrazolo[3,4-d]pyrimidin-1-yl)piperidine hydrochloride (60 g, 154.957 mmol, yield 52%). ¹H NMR (400 MHz, DMSO) δ 8.25 (s, 1H), 7.69 - 7.61 (m, 2H), 7.47 - 7.39 (m, 2H), 7.23 - 7.09 (m, 6H), 4.98 - 4.89 (m, 1H), 3.35 - 3.26 (m, 3H), 2.99 (t, *J =* 12.1 Hz, 2H), 2.35 - 2.19 (m, 2H), 2.07 - 1.98 (m, 2H). LCMS (ESI): calcd.: 387.19; found, [M+H]⁺=387.20..

### Intermediate Example 3: preparation of (S)-2-(4-(4-chlorophenyl)-2,3,9-trimethyl-6H-thieno[3,2-f][1,2,4]triazolo[4,3-a][1,4]diazepin-6-yl)-N-(2-(piperazin-1-yl)ethyl)acetamide

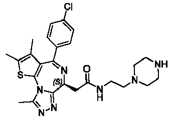

Referring to Scheme 6, in Step 1, to a solution of (S)-2-(4-(4-chlorophenyl)-2,3,9-trimethyl-6H-thieno[3,2-f][1,2,4]triazolo[4,3-a][1,4]diazepin-6-yl)acetic acid (1 equiv), tert-butyl 4-(2-aminoethyl)piperazine-1-carboxylate (1.2 equiv), and HATU (2 equiv) in 10 mL of DCM was added DIEA (5 equiv). The reaction was carried out at room temperature for 1 hour. The reaction mixture was washed with 10 mL of water twice and concentrated under reduced pressure to remove the solvent. The resulting residue was purified by silica gel column chromatography (eluent (v/v): DCM/MeOH = 100/0-10/1) to obtain the corresponding Boc intermediate compound.

**Step 2,** the compound obtained from Step 1 was dissolved in HCl/1,4-dioxane and reacted at room temperature for 1 hour. The reaction mixture was concentrated under reduced pressure to remove the solvent, and the target compound (yellow solid, 72 mg, yield 53%) was obtained. LCMS (ESI) m/z: calcd for C₂₅H₃₁ClN₇OS⁺ [M+H]⁺, 512.20; found, 512.2.

### Intermediate Example 4: preparation of (S)-2-(4-(4-chlorophenyl)-2,3,9-trimethyl-6H-thieno[3,2-f][1,2,4]triazolo[4,3-a][1,4]diazepin-6-yl)-1-(piperazin-1-yl)ethan-1-one

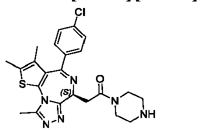

Referring to Scheme 6, in Step 1, to a solution of (S)-2-(4-(4-chlorophenyl)-2,3,9-trimethyl-6H-thieno[3,2-f][1,2,4]triazolo[4,3-a][1,4]diazepin-6-yl)acetic acid (1 equiv), tert-butyl piperazine-1-carboxylate (1.2 equiv), and HATU (2 equiv) in 10 Ml of DCM was added DIEA (5 equiv). The reaction was carried out at room temperature for 1 hour. The reaction mixture was washed with 10 mL of water twice and concentrated under reduced pressure to remove the solvent. The resulting residue was purified by silica gel column chromatography (eluent (v/v): DCM to DCM/MeOH (10/1)) to obtain the corresponding Boc intermediate compound.

**Step 2,** the compound obtained from Step 1 was dissolved in HCl/1,4-dioxane and reacted at room temperature for 1 hour. The reaction mixture was concentrated under reduced pressure to remove the solvent, and the target compound (yellow solid, 260 mg, yield 92%) was obtained. LCMS (ESI) m/z: calcd for C₂₃H₂₆ClN₆OS⁺[M+H]⁺, 469.16; found, 469.1.

### Intermediate Example 5: preparation of tert-butyl 4-(2-(4-aminophenoxy)ethyl)piperazine-1-carboxylate

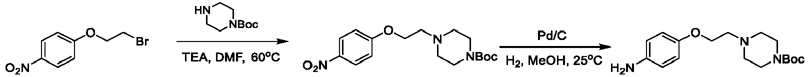

**Step 1:** To a solution of 1-(2-bromoethoxy)-4-nitrobenzene (4.50 g, 18.288 mmol) in N,N-dimethylformamide (100 mL) was added TEA (3.66 g, 36.576 mmol), and the reaction mixture was stirred at 60°C for 30 minutes. TLC analysis showed the completion of the reaction. The reaction mixture was diluted with EtOAc (100 mL) and ice water (100 mL). The organic layer was separated and further washed with saturated brine (100 mL), then concentrated under vacuum. The residue was purified by silica gel column chromatography (eluent (v/v): PE/EtOAc = 1/0-0/1) to obtain the colorless oily compound tert-butyl 4-(2-(4-nitrophenoxy)ethyl)piperazine-1-carboxylate (4 g, 11.383 mmol, yield 62.24%). ¹H NMR (300 MHz, CDCl₃) δ 8.19 (2H, m), 6.97 (2H, m), 4.19 (t, J= 5.6; LCMS (ESI): calcd for (M) 351.18, found, (M+H)⁺ 352.30..

**Step 2**: To a solution of tert-butyl 4-(2-(4-nitrophenoxy)ethyl)piperazine-1-carboxylate (4.00 g, 11.383 mmol) in MeOH (50 mL) was added Pd/C (0.12 g, 1.138 mmol, 10%), and the reaction was stirred under a hydrogen balloon at room temperature for 16 hours. LCMS analysis showed the completion of the reaction. The mixture was filtered through a celite bed and washed with methanol (20 mL). The filtrate was concentrated to obtain the off-white solid tert-butyl 4-(2-(4-aminophenoxy)ethyl)piperazine-1-carboxylate (3.1 g, yield 84.73%). LCMS (ESI): calcd for (M) 321.42, found, (M+H)⁺ 322.20.

### Intermediate Example 6: preparation of tert-butyl 4-(6-aminopyridazin-3-yl)piperazine-1-carboxylate

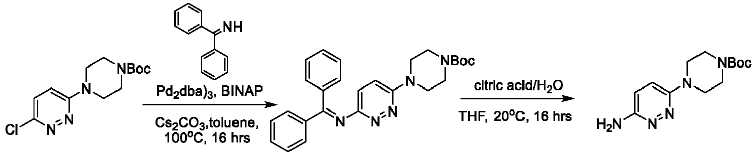

**Step 1**: Under argon protection, to a solution of tert-butyl 4-(6-chloropyridazin-3-yl)piperazine-1-carboxylate (3.00 g, 10.041 mmol) in toluene (60 mL) were added diphenylmethanimine (2.022 mL, 12.049 mmol), BINAP (0.06 g, 0.100 mmol), Cs₂CO₃ (6.54 g, 20.082 mmol) and Pd₂(dba)₃ (0.46 g, 0.502 mmol). The resulting suspension was stirred at 100°C for 16 hours. LCMS analysis showed the completion of the reaction. The reaction mixture was cooled to room temperature, then filtered through celite and washed with ethyl acetate (30 mL). The filtrate was washed with saturated brine (20 mL), dried over anhydrous magnesium sulfate, and filtered to separate the solid. The filtrate was concentrated under reduced pressure, and the resulting residue was purified by silica gel column chromatography to obtain the white solid compound tert-butyl 4-(6-((diphenylmethylene)amino)pyridazin-3-yl)piperazine-1-carboxylate (2.60 g, 5.862 mmol, yield 58.38%). LCMS (ESI): calcd for 443.20; found, [M+H]⁺=444.20.

**Step 2:** To a solution of tert-butyl 4-(6-((diphenylmethylene)amino)pyridazin-3-yl)piperazine-1-carboxylate (2.60 g, 5.862 mmol) in THF (50 mL) was added citric acid/H₂O (20 mL), and the mixture was stirred at 20°C for 16 hours. LCMS analysis showed the completion of the reaction. The reaction mixture was concentrated under vacuum to remove THF. The resulting aqueous phase was adjusted to pH = 8 with saturated NaHCO₃ and extracted with EtOAc (100 mL × 3). The combined organic phases were dried over Na₂SO₄ and concentrated under reduced pressure to obtain the white solid tert-butyl 4-(6-aminopyridazin-3-yl)piperazine-1-carboxylate (1.6 g, yield 97.71%). LCMS (ESI): calcd for M: 279.20; found, [M+H]⁺ =280.20.

### Intermediate Example 7: preparation of N-(tert-butyl)-3-((5-methyl-2-((4-(2-(piperazin-1-yl)ethoxy)phenyl)amino)pyrimidin-4-yl)amino)benzenesulfonamide (GT-M-002)

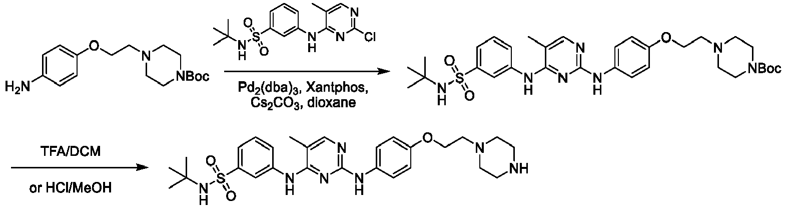

**Step 1**: tert-butyl 4-(2-(4-((4-((3-(N-(tert-butyl)sulfamoyl)phenyl)amino)-5-methylpyrimidin-2-yl)amino)phenoxy)ethyl)piperazine-1-carboxylate was prepared by referring to the method of Step 2 in Scheme 3.

Under argon protection, to a solution of tert-butyl 4-(2-(4-aminophenoxy)ethyl)piperazine-1-carboxylate (2.0 g, 6.222 mmol) and N-(tert-butyl)-3-((2-chloro-5-methylpyrimidin-4-yl)amino)benzenesulfonamide (prepared in Scheme 3, 2.87 g, 8.089 mmol) in dioxane (50 mL) were added Pd₂(dba)₃ (0.36g,0.622mmol), Cs₂CO₃ (4.06g,12.445mmol), and 4,5-bis(diphenylphosphino)-9,9-dimethylxanthene (0.72 g, 1.244 mmol). The reaction mixture was stirred at 100°C for 4 hours. The reaction mixture was diluted with EtOAc (50 mL) and saturated NaCl solution (50 mL). The resulting reaction mixture was dried over Na₂SO₄, concentrated under vacuum to obtain the crude product. The crude product was purified by silica gel flash column chromatography (eluent (v/v): EtOAc/PE = 0/1 - 1/4) to obtain the white solid compound tert-butyl 4-(2-(4-((4-((3-(N-(tert-butyl)sulfamoyl)phenyl)amino)-5-methylpyrimidin-2-yl)amino)phenoxy)ethyl)piperazine-1-carboxylate (2 g, 3.126 mmol, 50.24%). LCMS (ESI): calcd for (M) 639.20, found, (M+H)⁺ 640.20.

**Step 2:** To a solution of tert-butyl 4-(2-(4-((4-((3-(N-(tert-butyl)sulfamoyl)phenyl)amino)-5-methylpyrimidin-2-yl)amino)phenoxy)ethyl)piperazine-1-carboxylate (2 g, 3.126 mmol) in DCM (50 mL) was added TFA (10 mL), and the reaction mixture was stirred at room temperature for 20 minutes. LCMS analysis showed the completion of the reaction. The reaction mixture was concentrated under vacuum to obtain the white solid compound N-(tert-butyl)-3-((5-methyl-2-((4-(2-(piperazin-1-yl)ethoxy)phenyl)amino)pyrimidin-4-yl)amino)benzenesulfonamide (1.20 g, yield 71.13%). ¹H NMR (400 MHz, DMSO) δ 8.78 (s, 1H), 8.54 (s, 1H), 8.11 (s, 2H), 7.90 (s, 1H), 7.62 - 7.40 (m, 5H), 6.79 (d, 1H) J = 9.0 Hz, 2H), 3.99 (t, J = 5.9 Hz, 2H), 2.65 (dt, J = 11.8, 5.3 Hz, 6H), 2.38 (s, 4H), 2.12 (s, 3H), 1.12 ( s, 9H). LCMS (ESI): (M+H)⁺ calcd for (M) 540.3, found, 540.7.

### Intermediate Example 8: preparation of N-(tert-butyl)-3-((5-methyl-2-((6-(piperazin-1-yl)pyridazin-3-yl)amino)pyrimidin-4-yl)amino)benzenesulfonamide (GT-M-17)

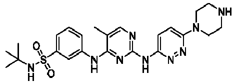

Following the method of Step 2 in Scheme 3 and the method of Intermediate Example 7, under appropriate conditions understood in the field, tert-butyl 4-(6-aminopyridazin-3-yl)piperazine-1-carboxylate prepared from Intermediate Example 6 and N-(tert-butyl)-3-((2-chloro-5-methylpyrimidin-4-yl)amino)benzenesulfonamide were used to prepare the target compound N-(tert-butyl)-3-((5-methyl-2-((6-(piperazin-1-yl)pyridazin-3-yl)amino)pyrimidin-4-yl)amino)benzenesulfonamide (552 mg, yield 66.31%) as a white solid. ¹H NMR (400 MHz, DMSO) δ 9.35 (s, 1H), 8.60 (s, 1H), 8.25 (s, 1H), 8.14 (dd, J = 6.9, 2.4 Hz, 1H), 8.07 (d, J = 9.8 Hz, 1H), 7.94 (d, J = 0.7 Hz, 1H), 7.65 (s, 1H), 7.53 - 7.43 (m, 2H), 7.21 (d, J = 9.9 Hz, 1H), 3.45 - 3.35 (m, 4H), 2.89 - 2.75 (m, 4H), 2.15 (s, 3H), 1.12 (s, 9H). LCMS (ESI): calcd for M: 497.30; found, [M+H]⁺=498.30.

### Intermediate Example 9: preparation of N-(tert-butyl)-3-((5-methyl-2-((4-(piperidin-4-yl)phenyl)amino)pyrimidin-4-yl)amino)benzenesulfonamide (GT-M-003)

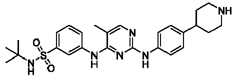

Following the method of Step 2 in Scheme 3 and the method of Intermediate Example 7, under appropriate conditions understood in the field, N-(tert-butyl)-3-((2-chloro-5-methylpyrimidin-4-yl)amino)benzenesulfonamide and tert-butyl 4-(4-aminophenyl)piperidine-1-carboxylate were used to prepare the target compound N-(tert-butyl)-3-((5-methyl-2-((4-(piperidin-4-yl)phenyl)amino)pyrimidin-4-yl)amino)benzenesulfonamide (1.2 g, yield 72.14%) as a white solid. ¹H NMR (400 MHz, DMSO) δ 8.87 (s, 1H), 8.56 (s, 1H), 8.11 (s, 2H), 7.92 (s, 1H), 7.65 - 7.40 (m, 5H), 7.04 (d, J = 8.5 Hz, 2H), 3.17 (s, 3H), 3.00 (d, J = 12.0 Hz, 2H), 2.55 (dd, J = 12.1, 10.3 Hz, 2H), 2.48 (d, J = 15.5 Hz, 3H), 2.13 (s, 3H), 1.64 (d, J = 11.5 Hz, 2H), 1.46 (dd, J = 12.2, 3.6 Hz, 2H), 1.12 (s, 9H). LCMS (ESI): (M+H)⁺ calcd. (M) 495.3, found, 495.2.

### Intermediate Example 10: preparation of N-(tert-butyl)-3-((5-methyl-2-((4-(piperazin-1-yl)phenyl)amino)pyrimidin-4-yl)amino)benzenesulfonamide (GT-M-004)

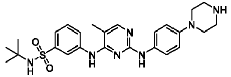

Following the method of Step 2 in Scheme 3 and the method of Intermediate Example 7, under appropriate conditions understood in the field, N-(tert-butyl)-3-((2-chloro-5-methylpyrimidin-4-yl)amino)benzenesulfonamide and tert-butyl 4-(4-aminophenyl)piperazine-1-carboxylate were used to prepare the target compound N-(tert-butyl)-3-((5-methyl-2-((4-(piperazin-1-yl)phenyl)amino)pyrimidin-4-yl)amino)benzenesulfonamide (1.5 g, yield 90.15%) as a white solid. ¹H NMR (400 MHz, DMSO) δ 8.70 (s, 1H), 8.51 (s, 1H), 8.14 (s, 2H), 7.89 (s, 1H), 7.57 (s, 1H), 7.48 (d, J = 6.3 Hz, 4H), 6.80 (d, J = 8.9 Hz, 2H), 3.57 (s, 2H), 3.17 (s, 1H), 3.00 - 2.89 (m, 4H), 2.81 (dd, J = 10.1, 5.8 Hz, 5H), 2.12 (s, 3H), 1.12 (s, 9H). LCMS (ESI): (M+H)⁺ calcd. (M) 496.3, found, 496.2.

### Intermediate Example 11: preparation of 6-(4-(tert-butoxycarbonyl)piperazin-1-yl)pyridazine-3-carboxylic acid

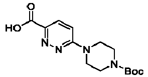

6-(4-(tert-butoxycarbonyl)piperazin-1-yl)pyridazine-3-carboxylic acid was prepared by referring to the method of Scheme 4.

**Step 1:** to a solution of methyl 6-Chloropyridazine-3-carboxylate (500.00 mg, 2.897 mmol) and tert-butyl piperazine-1-carboxylate (809.45 mg, 4.346 mmol) in DMF (10.00 mL) was added DIEA (1.437 mL, 8.692 mmol), and the reaction mixture was stirred at 60°C for 12 h. LC-MS analysis showed completion of the reaction. Water was added to the reaction mixture, which was then extracted with ethyl acetate. The organic phase was separated, washed with saturated brine, and concentrated to obtain a crude product. The crude product was purified by chromatography (eluent (v/v): PE/EA = 5/1) to yield methyl 6-(4-(tert-butoxycarbonyl)piperazin-1-yl)pyridazine-3-carboxylate (900 mg, yield 75.16%) as a white solid. LCMS (ESI): calcd for C₁₅H₂₃N₄O₄⁺ [M+H]⁺: 323.16, found, 323.20..

**Step 2:** to a solution of methyl 6-(4-(tert-butoxycarbonyl)piperazin-1-yl)pyridazine-3-carboxylate (400 mg, 1.241 mmol) in a mixed solvent of 1,4-dioxane and water (1/1; 10 mL) was added Lithium hydroxide (260.32 mg, 6.204 mmol), and the reaction mixture was stirred at 60°C for 12 h. LC-MS analysis showed completion of the reaction. The reaction mixture was concentrated under reduced pressure to remove the organic phase. The remaining aqueous phase was adjusted to pH = 6 with concentrated hydrochloric acid and then extracted with ethyl acetate. The organic phase was separated, concentrated under reduced pressure to obtain 6-(4-(tert-butoxycarbonyl)piperazin-1-yl)pyridazine-3-carboxylic acid (300 mg, yield 76.06%) as a white solid, which can be used for the next step without further purification. LCMS (ESI): calcd for C₁₄H₂₁N₄O₄⁺ [M+H]⁺: 309.15, found, 309.20.

### Intermediate Example 12: preparation of N-((1r,4r)-4-(3-chloro-4-cyanophenoxy)cyclohexyl)-6-(piperazin-1-yl)pyridazine-3-carboxamide

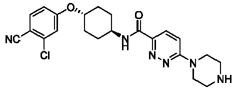

N-((1r,4r)-4-(3-chloro-4-cyanophenoxy)cyclohexyl)-6-(piperazin-1-yl)pyridazine-3-carboxamide was prepared by referring to the method of Scheme 5.

To a solution of 6-(4-(tert-butoxycarbonyl)piperazin-1-yl)pyridazine-3-carboxylic acid (300 mg, 1.135 mmol) prepared from Intermediate Example 11 and 4-(((1r,4r)-4-aminocyclohexyl)oxy)-2-chlorobenzonitrile (326.61 mg, 1.135 mmol) in DMF (5.00 mL) were added HATU (647.40 mg, 1.703 mmol) and DIEA (0.938 mL, 5.676 mmol), and the resulting reaction mixture was stirred at room temperature for 30 minutes. LC-MS analysis showed completion of the reaction. Water was added to the reaction mixture, which was then extracted with ethyl acetate. The organic phase was separated, washed with saturated brine, and concentrated to obtain a crude product. The crude product was purified by chromatography (eluent (v/v): DCM/MeOH = 100/1) to yield the white solid compound tert-butyl 4-(6-(((1r,4r)-4-(3-chloro-4-cyanophenoxy)cyclohexyl)carbamoyl)pyridazin-3-yl)piperazine-1-carboxylate (250 mg, yield 39.49%). LCMS (ESI): calcd for C₂₇H₃₄ClN₆O₄⁺ [M+H]⁺: 541.23, found, 541.30.

tert-butyl 4-(6-(((1r,4r)-4-(3 -chloro-4-cyanophenoxy)cyclohexyl)carbamoyl)pyridazin-3 - yl)piperazine-1-carboxylate (250 mg, 0.462 mmol) was dissolved in a mixed solvent of DMF (5.00 mL) and TFA (1.00 mL). The reaction mixture was stirred at room temperature for 3 h. LC-MS analysis showed completion of the reaction. The reaction mixture was concentrated under reduced pressure to remove the organic phase. The remaining aqueous phase was adjusted to pH = 8 with saturated NaHCO₃ solution and then extracted with ethyl acetate. The organic phase was separated, concentrated under reduced pressure to obtain N-((1r,4r)-4-(3-chloro-4-cyanophenoxy)cyclohexyl)-6-(piperazin-1-yl)pyridazine-3-carboxamide (170 mg, yield 75.10%) as a white solid, which can be used for the next step without further purification. LCMS (ESI): calcd for C₂₂H₂₆ClN₆O₂⁺ [M+H]⁺: 441.17, found, 441.20.

### Intermediate Example 13: preparation of 3-(5-(bromomethyl)-1-oxoisoindolin-2-yl)piperidine-2,6-dione (GT-03234)

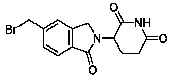

3-(5-(bromomethyl)-1-oxoisoindolin-2-yl)piperidine-2,6-dione was prepared by referring to the method of Scheme 7.

To a solution of methyl 2,5-dimethylbenzoate (3.4 g, 20.6 mmol) in 100 mL of carbon tetrachloride were added sequentially N-bromosuccinimide (8.0 g, 45 mmol) and dibenzoyl peroxide (0.31 g, 1.26 mmol). The reaction mixture was refluxed at 80°C for 12 h, cooled to room temperature, diluted with 100 mL of petroleum ether, washed with water twice, and washed with saturated brine once. The organic phase was dried over anhydrous sodium sulfate, and concentrated under reduced pressure to remove the solvent. The residue was purified by silica gel column chromatography (eluent (v/v): EA/PE = 0/1-1/20) to obtain the intermediate compound methyl 2,5-bis(bromomethyl)benzoate (white solid, 5.0 g, yield: 76%).

Cesium carbonate (1.6 g, 5 mmol) was added to a mixed solvent of 50 mL of 1,2-dichloroethane and 5 mL of hexafluoroisopropanol. The mixture was stirred at 65°C for 15 min. to the mixture were added sequentially 3-amino-2,6-piperidinedione hydrochloride (8.21 g, 5 mmol) and methyl 2,5-bis(bromomethyl)benzoate (1.6 g, 5 mmol), and the resulting mixture was stirred at 65°C for 12 h. After the reaction, the mixture was cooled to room temperature, washed with water. The organic phase was separated, and concentrated under reduced pressure to remove the solvent. The residue was purified by silica gel column chromatography (eluent (v/v): MeOH/DCM = 0/1-1/10). The obtained product was further purified by triturating with acetonitrile to obtain a white powder (455 mg, yield: 27%). ¹H NMR (500 MHz, DMSO-*d*₆) δ 11.00 (s, 1H), 7.78 - 7.66 (m, 2H), 7.59 (dd, *J* = 7.9, 1.5 Hz, 1H), 5.11 (dd, *J* = 13.3, 5.1 Hz, 1H), 4.83 (s, 2H), 4.47 (d, *J* = 17.3 Hz, 1H), 4.34 (d, *J* = 17.4 Hz, 1H), 2.91 (ddd, *J* = 17.3, 13.6, 5.4 Hz, 1H), 2.68 - 2.55 (m, 1H), 2.39 (qd, *J* = 13.3, 4.5 Hz, 1H), 2.01 (dtd, *J* = 12.7, 5.3, 4.7, 1.9 Hz, 1H). LCMS (ESI) m/z: calcd for C₁₄H₁₄BrN₂O₃⁺ [M+H]⁺, 337.0; found, 337.3.

### Intermediate Example 14: preparation of 3-(5-(hydroxymethyl)-1-oxoisoindolin-2-yl)piperidine-2,6-dione (GT-03421)

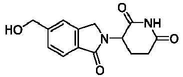

3-(5-(Hydroxymethyl)-1-oxoisoindolin-2-yl)piperidine-2,6-dione was prepared by referring to the method of Scheme 9.

Under argon protection at 25°C, to a solution of 3-(5-bromo-1-oxoisoindolin-2-yl)piperidine-2,6-dione (10 g, 30.945 mmol) in anhydrous dioxane (280 mL) were added (tributylstannyl)methanol (14.90 g, 46.418 mmol) and Pd(PPh₃)₄ (1.32 g, 1.145 mmol). The mixture was heated to 90°C and stirred for 16 hours. TLC analysis showed complete consumption of the starting material. The mixture was cooled to 25°C and concentrated under vacuum to obtain the crude product. The crude product was ground with DCM (200 mL) and filtered to obtain 3-(5-(hydroxymethyl)-1-oxoisoindolin-2-yl)piperidine-2,6-dione (7.5 g, 27.344 mmol, yield 88.36%) as a gray solid. ¹H NMR (400 MHz, DMSO) δ 10.98 (s, 1H), 7.74 - 7.64 (m, 1H), 7.55 (s, 1H), 7.45 (d, J = 7.8 Hz, 1H), 5.40 (t, J = 5.7 Hz, 1H), 5.11 (dd, J = 13.3, 5.1 Hz, 1H), 4.62 (d, J = 5.7 Hz, 2H), 4.38 (dd, J = 53.1, 17.2 Hz, 2H), 3.03 (2.82) m, 1H), 2.60 (d, J = 17.5 Hz, 1H), 2.39 (dd, J = 13.1, 4.4 Hz, 1H), 2.08 - 1.92 (m, 1H). LCMS (ESI): calcd for: [M+H]+=275.10; found, [M+H]⁺=275.0.

### Intermediate Example 15: preparation of 3-(4-fluoro-5-(hydroxymethyl)-1-oxoisoindolin-2-yl)piperidine-2,6-dione (GT-03445)

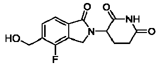

3-(4-fluoro-5-(hydroxymethyl)-1-oxoisoindolin-2-yl)piperidine-2,6-dione was prepared by referring to the method of Scheme 9.

At 25°C, to a solution of 3-(5-bromo-4-fluoro-1-oxoisoindolin-2-yl)piperidine-2,6-dione (2.0 g, 5.863 mmol) in anhydrous dioxane (56 mL) were added (tributylstannyl)methanol (2.82g, 8.794mmol) and Pd(PPh₃)₄ (0.34g, 0.293mmol). The mixture was degassed three times and backfill with N₂. The mixture was heated to 90°C and stir for 16 hours. TLC (DCM/MeOH=10: 1) and LCMS showed complete consumption of the starting material. The mixture was cooled to room temperature and filtered to remove black solids. The filtrate was concentrated under reduced pressure and purify by silica gel flash chromatography (eluent (v/v): DCM/MeOH = 1/0-10/1) to obtain pure white solid 3-(4-fluoro-5-(hydroxymethyl)-1-oxoisoindolin-2-yl)piperidine-2,6-dione (410mg, 1.403mmol, yield 23.93%). ¹H NMR (400 MHz, DMSO) δ 10.99 (s, 1H), 7.80 - 7.42 (m, 2H), 5.46 (t, J = 5.7 Hz, 1H), 5.11 (dd, J = 13.3, 5.0 Hz, 1H), 4.65 (d, J = 5.6 Hz, 2H), 4.46 (dd, J = 69.4, 17.3 Hz, 2H), 3.03 - 2.81 (m, 1H), 2.60 (d, J = 17.6 Hz, 1H), 2.46 - 2.31 (m, 1H), 2.09 - 1.93 (m, 1H). LCMS (ESI): calcd for : [M+H]⁺=293.09; found, [M+H]⁺=293.10.

### Intermediate Example 16: preparation of 3-(7-fluoro-5-(hydroxymethyl)-1-oxoisoindolin-2-yl)piperidine-2,6-dione (GT-03446)

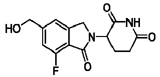

3-(7-fluoro-5-(hydroxymethyl)-1-oxoisoindolin-2-yl)piperidine-2,6-dione was prepared by referring to the method of Scheme 9.

At 25°C, to a solution of 3-(5-bromo-7-fluoro-1-oxoisoindolin-2-yl)piperidine-2,6-dione (2.0 g, 5.863 mmol) in anhydrous dioxane (56 mL) were added (tributylstannyl)methanol (2.82g, 8.794mmol) and Pd(PPh₃)₄ (0.34g, 0.293mmol). The mixture was degassed three times and backfill with N₂. The mixture was heated to 90°C and stir for 16 hours. TLC (DCM/MeOH=10: 1) showed complete consumption of the starting material. The mixture was cooled to room temperature and filtered to remove black solids. The filtrate was concentrated under reduced pressure to obtain the crude product. The crude product was ground with DCM (30mL) and filter to obtain 3-(7-fluoro-5-(hydroxymethyl)-1-oxoisoindolin-2-yl)piperidine-2,6-dione as a gray solid (1.01g, 3.456mmol, yield 58.94%). ¹H NMR (400 MHz, DMSO) δ 10.99 (s, 1H), 7.38 (s, 1H), 7.20 (d, J = 10.6 Hz, 1H), 5.50 (t, J = 5.7 Hz, 1H), 5.08 (dd, J = 13.3, 5.1 Hz, 1H), 4.62 (d, J = 5.7 Hz, 2H), 4.40 (dd, J = 54.3, 17.6 Hz, 2H), 2.99 - 2.82 (m, 1H), 2.60 (d, J = 17.5 Hz, 1H), 2.38 (dd, J = 13.1, 4.4 Hz, 1H), 2.11 - 1.93 (m, 1H). LCMS (ESI): calcd for: [M+H]⁺=293.09; found, [M+H]⁺=293.0.

### Intermediate Example 17: preparation of 3-(4-Bromo-6-(bromomethyl)-1-oxoisoindolin-2-yl)piperidine-2,6-dione (GT-03419)

3-(4-Bromo-6-(bromomethyl)-1-oxoisoindolin-2-yl)piperidine-2,6-dione (GT-03419) was prepared by referring to Scheme 10.

### Step 1:

To a solution of the raw material methyl 2,5-dimethyl-3-bromobenzoate (5.0 g, 20.6 mmol) in 100 mL of carbon tetrachloride were added sequentially N-bromosuccinimide (8.0 g, 45 mmol) and dibenzoyl peroxide (0.31 g, 1.26 mmol). The reaction mixture was refluxed at 80°C for 12 h, cooled to room temperature, and diluted with 100 mL of petroleum ether. The diluted mixture was washed twice with water and once with saturated saline. The organic phase was separated and combined, dried over anhydrous sodium sulfate, filtered, and the filtrate was concentrated under reduced pressure to remove the solvent. The residue was separated by silica gel chromatography (PE/EA =1/0 - 20/1) to obtain the intermediate compound (6.3 g, yield: 76%) as a white solid.

### Step 2:

Cesium carbonate (16.25 g, 49.89 mmol) was added to a mixture of 500 mL of 1,2-dichloroethane and 50 mL of hexafluoroisopropanol. The mixture was stirred at 65°C for 15 min, followed by the sequential addition of 3-amino-2,6-piperidinedione hydrochloride (8.21 g, 49.89 mmol) and the compound 363126 obtained from Step 1 (20 g, 49.89 mmol). The reaction mixture was stirred at 65°C for 12 h. The reaction mixture was filtered hot to remove insolubles, and the filtrate was washed with saturated brine and concentrated under reduced pressure to remove the organic solvent. The residual solid was slurried with 50 mL of dichloromethane and 200 mL of petroleum ether. The resulting mixture was filtered, and the filtered solid was washed with 50 mL of dichloromethane and 50 mL of ether to obtain the product GT-03419 (11 g, yield: 42%) as a gray powder. ¹H NMR (500 MHz, 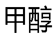 -*d*₄) δ 7.90 (d, *J =* 1.5 Hz, 1H), 7.84 (d, *J =* 1.5 Hz, 1H), 5.17 (dd, *J =* 13.4, 5.2 Hz, 1H), 4.68 (s, 2H), 4.45 (q, *J* = 17.6 Hz, 2H), 2.91 (ddd, *J* = 17.6, 13.6, 5.4 Hz, 1H), 2.79 (ddd, *J* = 17.6, 4.6, 2.4 Hz, 1H), 2.53 (qd, *J* = 13.4, 4.7 Hz, 1H), 2.19 (dtd, *J* = 13.0, 5.4, 2.4 Hz, 1H). MS (ESI) m/z: calcd for C₁₄H₁₃Br₂N₂O₃⁺ [M+H]⁺, 414.9; found, 415.1.

### Intermediate Example 18: preparation of N-(tert-butyl)-3-((5-methyl-2-((4-(4-(methylamino)piperidin-1-yl)phenyl)amino)pyrimidin-4-yl)amino)benzenesulfonamide (GT-D-89)

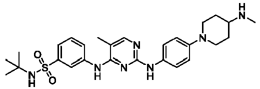

N-(tert-butyl)-3-((5-methyl-2-((4-(4-(methylamino)piperidin-1-yl)phenyl)amino)pyrimidin-4-yl)amino)benzenesulfonamide (GT-D-89) was prepared by referring to Scheme 16 (white solid, 1.1 g). ¹H NMR (400 MHz, MeOD) δ 8.07 (s, 1H), 7.80 (d, *J=* 7.9 Hz, 1H), 7.78 - 7.69 (m, 2H), 7.58 (t, *J=* 8.0 Hz, 1H), 7.34 (d, *J=* 9.0 Hz, 2H), 7.16 (d, *J=* 9.0 Hz, 2H), 3.83 (d, *J=* 12.9 Hz, 2H), 3.33 (d, *J=* 11.0 Hz, 1H), 3.10 (t, *J=* 12.1 Hz, 2H), 2.76 (s, 3H), 2.33 - 2.17 (m, 5H), 1.88 (qd, *J=* 12.5, 3.8 Hz, 2H), 1.16 (d, *J* = 5.5 Hz, 9H). LCMS (ESI): calcd., 524.3; found, 524.4.

### Intermediate Example 19: preparation of N-(tert-butyl)-3-((5-methyl-2-((4-(4-(piperazin-1-yl)piperidin-1-yl)phenyl)amino)pyrimidin-4-yl)amino)benzenesulfonamide (GT-D-90)

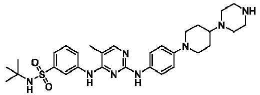

N-(tert-butyl)-3-((5-methyl-2-((4-(4-(piperazin-1-yl)piperidin-1-yl)phenyl)amino)pyrimidin-4-yl)amino)benzenesulfonamide (GT-D-90) was prepared by referring to Scheme 16 (white solid, 0.90 g). ¹H NMR (400 MHz, MeOD) δ 8.07 (s, 1H), 7.82 (d, *J* = 7.9 Hz, 1H), 7.75 (s, 1H), 7.70 (d, *J* = 8.3 Hz, 1H), 7.60 (t, *J=* 7.9 Hz, 1H), 7.43 (d, *J=* 8.9 Hz, 2H), 7.28 (d, *J=* 9.0 Hz, 2H), 3.98 (s, 1H), 3.84 (d, *J* = 12.7 Hz, 2H), 3.59 - 3.47 (m, 4H), 3.40 (s, 4H), 3.25 (d, *J* = 11.6 Hz, 2H), 2.34 - 2.20 (m, 5H), 2.15 - 1.98 (m, 2H), 1.18 (d, *J* = 15.0 Hz, 9H). LCMS (ESI): calcd., 579.3; found, 579.4.

### Intermediate Example 20: preparation of 5-chloro-N²-(2-isopropoxy-5-methyl-4-(piperazin-1-yl)phenyl)-N⁴-(2-(isopropylsulfonyl)phenyl)pyrimidine-2,4-diamine (GT-D-86)

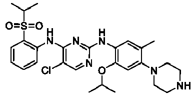

5-chloro-N²-(2-isopropoxy-5-methyl-4-(piperazin-1-yl)phenyl)-N⁴-(2-(isopropylsulfonyl)phenyl)pyrimidine-2,4-diamine (GT-D-86) was prepared by referring to Scheme 16 (brown solid, 2.46 g). ¹H NMR (400 MHz, DMSO) δ 9.51 (s, 1H), 9.14 (s, 2H), 8.43 (d, *J =* 8.3 Hz, 1H), 8.26 (s, 1H), 8.20 (s, 1H), 7.85 (dd, *J =* 7.9, 1.2 Hz, 1H), 7.63 (t, *J =* 7.7 Hz, 1H), 7.51 (s, 1H), 7.37 (t, *J =* 7.5 Hz, 1H), 6.70 (s, 1H), 4.56 (dd, *J* = 12.0, 6.0 Hz, 1H), 3.23 (s, 4H), 3.04 (d, *J* = 4.3 Hz, 4H), 2.09 (s, 3H), 1.19 (dd, *J* = 24.2, 6.4 Hz, 12H). LCMS (ESI): calcd., 559.2; found, 559.1.

### Intermediate Example 21: preparation of 5-chloro-N²-(2-isopropoxy-5-methyl-4-(4-(methylamino)piperidin-1-yl)phenyl)-N⁴-(2-(isopropylsulfonyl)phenyl)pyrimidine-2,4-diamine (GT-D-87)

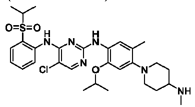

5-chloro-N²-(2-isopropoxy-5-methyl-4-(4-(methylamino)piperidin-1-yl)phenyl)-N⁴-(2-(isopropylsulfonyl)phenyl)pyrimidine-2,4-diamine (GT-D-87) was prepared by referring to Scheme 16 (white solid, 1.2 g). ¹H NMR (400 MHz, DMSO) δ 9.93 (s, 1H), 9.19 (s, 2H), 8.93 (s, 1H), 8.42 (s, 1H), 8.20 (s, 1H), 7.90 (s, 1H), 7.73 (t, *J* = 7.7 Hz, 1H), 7.51 (t, *J* = 7.7 Hz, 1H), 7.39 (s, 1H), 6.81 (s, 1H), 4.71 - 4.36 (m, 1H), 3.48 (dt, *J* = 13.4, 6.7 Hz, 1H), 2.57 (t, *J* = 5.3 Hz, 3H), 2.13 (s, 2H), 2.04 (s, 3H), 1.82 (s, 2H), 1.24 (d, *J* = 6.0 Hz, 6H), 1.14 (d, *J* = 6.8 Hz, 6H). LCMS (ESI): calcd., 587.3; found, 587.3.

### Intermediate Example 22: preparation of 5-chloro-N²-(2-isopropoxy-5-methyl-4-(4-(piperazin-1-yl)piperidin-1-yl)phenyl)-N⁴-(2-(isopropylsulfonyl)phenyl)pyrimidine-2,4-diamine (GT-D-88)

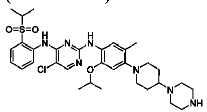

5-chloro-N²-(2-isopropoxy-5-methyl-4-(4-(piperazin-1-yl)piperidin-1-yl)phenyl)-N⁴-(2-(isopropylsulfonyl)phenyl)pyrimidine-2,4-diamine (GT-D-88) was prepared by referring to Scheme 16 (brown solid, 1.2 g). ¹H NMR (400 MHz, DMSO) δ 9.47 (s, 1H), 8.46 (d, *J* = 7.9 Hz, 1H), 8.24 (s, 1H), 8.09 (s, 1H), 7.83 (d, *J* = 7.9 Hz, 1H), 7.61 (t, *J* = 7.8 Hz, 1H), 7.49 (s, 1H), 7.35 (t, *J* = 7.7 Hz, 1H), 6.70 (s, 1H), 4.60 - 4.42 (m, 1H), 3.54 - 3.36 (m, 8H), 3.26 - 3.06 (m, 4H), 2.66 (t, *J* = 11.1 Hz, 2H), 2.15 (d, *J* = 11.0 Hz, 1H), 2.09 (s, 3H), 1.79 (s, 2H), 1.26 (dd, *J* = 13.1, 7.2 Hz, 3H), 1.22 - 1.09 (m, 9H). LCMS (ESI): calcd., 642.3; found, 642.3.

### Intermediate Example 23: preparation of (2-((5-chloro-2-((2-methoxy-4-(piperazin-1-yl)phenyl)amino)pyrimidin-4-yl)amino)phenyl)dimethylphosphine oxide (GT-D-39)

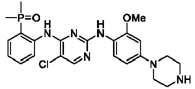

(2-((5-chloro-2-((2-methoxy-4-(piperazin-1-yl)phenyl)amino)pyrimidin-4-yl)amino)phenyl)dimethylphosphine oxide (GT-D-39) was prepared by referring to Scheme 16 (yellow solid, 7.9 g). ¹H NMR (400 MHz, DMSO) δ 11.57 (s, 1H), 9.55 (s, 1H), 9.17 (s, 2H), 8.76 (s, 1H), 8.44 (s, 1H), 8.17 (s, 1H), 7.59 (dd, *J* = 13.3, 7.7 Hz, 1H), 7.50 - 7.30 (m, 2H), 7.17 (t, *J* = 7.3 Hz, 1H), 6.73 (d, *J* = 2.1 Hz, 1H), 6.56 (dd, *J* = 8.6, 2.2 Hz, 1H), 3.79 (s, 3H), 3.49 - 3.36 (m, 4H), 3.26 (m, 4H), 1.80 (s, 3H), 1.77 (s, 3H). LCMS (ESI): calcd., 487.2; found, 487.2.

### Intermediate Example 24: preparation of (R)-N-(5-(2-methoxy-2-phenylacetyl)-1,4,5,6-tetrahydropyrrolo[3,4-c]pyrazol-3-yl)-4-(piperazin-1-yl)benzamide (GT-M-08)

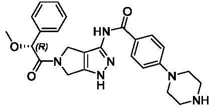

(R)-N-(5-(2-methoxy-2-phenylacetyl)-1,4,5,6-tetrahydropyrrolo[3,4-c]pyrazol-3-yl)-4-(piperazin-1-yl)benzamide (GT-M-08) was prepared by referring to Scheme 17 (white solid,0.165 g). ¹H NMR (400 MHz, DMSO) δ 10.71 (s, 1H), 7.89 (dd, J = 9.0, 2.7 Hz, 2H), 7.59 - 7.20 (m, 5H), 6.97 (dd, J = 9.0, 1.9 Hz, 2H), 5.11 (d, J = 12.1 Hz, 1H), 4.79 (dd, J = 23.9, 12.6 Hz, 1H), 4.62 - 4.26 (m, 4H), 3.34 (d, J = 2.4 Hz, 4H), 3.23 - 3.09 (m, 4H), 2.93 - 2.67 (m, 4H). LCMS (ESI): calcd., 461.2; found, 461.6.

### Intermediate Example 25: preparation of 2-((2-((2-methoxy-4-(piperazin-1-yl)phenyl)amino)-5-(trifluoromethyl)pyridin-4-yl)amino)-N-methylbenzamide (GT-M-10)

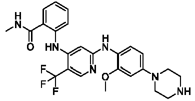

2-((2-((2-methoxy-4-(piperazin-1-yl)phenyl)amino)-5-(trifluoromethyl)pyridin-4-yl)amino)-N-methylbenzamide (GT-M-10) was prepared by referring to Scheme 16 (white solid, 1.87 g). ¹H NMR (400 MHz, MeOD) δ 8.01 (s, 1H), 7.73 (d, J = 7.9 Hz, 1H), 7.59 (d, J = 3.8 Hz, 2H), 7.36 (dt, J = 8.4, 4.1 Hz, 1H), 7.18 (d, J = 8.6 Hz, 1H), 6.79 (d, J = 2.1 Hz, 1H), 6.68 (dd, J = 8.7, 2.2 Hz, 1H), 6.48 (s, 1H), 3.87 (s, 3H), 3.66 (s, 4H), 3.56 - 3.48 (m, 4H), 3.43 - 3.37 (m, 4H), 2.91 (d, J = 13.7 Hz, 3H). LCMS (ESI): calcd., 501.2; found, 501.2.

### Intermediate Example 26: preparation of 2-((2-((4-(4-aminopiperidin-1-yl)-2-methoxyphenyl)amino)-5-(trifluoromethyl)pyridin-4-yl)amino)-N-methylbenzamide (GT-M-11)

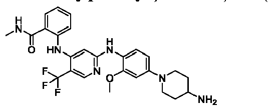

2-((2-((4-(4-aminopiperidin-1-yl)-2-methoxyphenyl)amino)-5-(trifluoromethyl)pyridin-4-yl)amino)-N-methylbenzamide (GT-M-11) was prepared by referring to Scheme 16 (brown solid,0.4 g). ¹H NMR (400 MHz, DMSO) δ 10.13 (s, 1H), 8.63 (d, J = 4.2 Hz, 1H), 8.27 (s, 1H), 8.14 (s, 1H), 7.74 - 7.60 (m, 1H), 7.44 (dd, J = 6.3, 1.3 Hz, 2H), 7.36 (d, J = 8.7 Hz, 1H), 7.08 - 7.00 (m, 1H), 6.57 (d, J = 2.5 Hz, 1H), 6.51 (s, 1H), 6.43 (dd, J = 8.8, 2.5 Hz, 1H), 3.77 (s, 3H), 3.57 (d, J = 12.6 Hz, 3H), 2.75 (d, J = 3.7 Hz, 4H). LCMS (ESI): calcd., 515.2; found, 515.2.

### Intermediate Example 27: preparation of 2-((2-((2-methoxy-4-(4-(piperazin-1-yl)piperidin-1-yl)phenyl)amino)-5-(trifluoromethyl)pyridin-4-yl)amino)-N-methylbenzamide (GT-M-12)

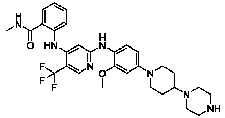

2-((2-((2-methoxy-4-(4-(piperazin-1-yl)piperidin-1-yl)phenyl)amino)-5-(trifluoromethyl)pyridin-4-yl)amino)-N-methylbenzamide (GT-M-12) was prepared by referring to Scheme 16 (purple solid, 0.34 g). ¹H NMR (400 MHz, DMSO) δ 10.60 (s, 1H), 10.10 (s, 1H), 9.99 (s, 1H), 9.75 (s, 1H), 8.82 (d, J = 4.6 Hz, 1H), 8.14 (s, 1H), 7.77 (d, J = 7.7 Hz, 1H), 7.57 (d, J = 3.2 Hz, 2H), 7.33 - 7.26 (m, 1H), 7.19 (s, 1H), 6.81 (s, 1H), 6.68 (s, 1H), 6.54 (s, 1H), 3.78 (d, J = 26.5 Hz, 15H), 3.53 (s, 9H), 2.84 (s, 2H), 2.76 (d, J = 4.5 Hz, 3H), 2.22 (d, J = 10.6 Hz, 2H), 1.87 (d, J = 9.5 Hz, 2H), 1.23 (s, 2H). LCMS (ESI): calcd., 584.3; found, 584.0.

### Intermediate Example 28: preparation of 2-((2-((2-methoxy-4-(piperazin-1-yl)phenyl)amino)-5-(trifluoromethyl)pyridin-4-yl)amino)-N-methylbenzamide (GT-M-171)

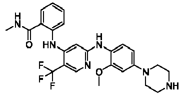

2-((2-((2-methoxy-4-(piperazin-1-yl)phenyl)amino)-5-(trifluoromethyl)pyridin-4-yl)amino)-N-methylbenzamide (GT-M-171) was prepared by referring to Scheme 16 (yellow solid, 20.5 g). ¹H NMR (400 MHz, DMSO) δ 10.39 (s, 1H), 9.20 (s, 1H), 8.80 (s, 2H), 8.69 (d, *J=* 4.6 Hz, 1H), 8.13 (s, 1H), 7.73 (d, *J* = 7.7 Hz, 1H), 7.53 (s, 2H), 7.35 (d, *J* = 8.6 Hz, 1H), 7.20 (s, 1H), 6.70 (d, *J* = 2.2 Hz, 1H), 6.59 - 6.49 (m, 2H), 3.80 (s, 3H), 3.36 (d, *J* = 5.2 Hz, 4H), 3.25 (s, 4H), 2.76 (d, *J* = 4.5 Hz, 3H). LCMS (ESI): calcd., 501.2; found, 501.3.

### Intermediate Example 29: preparation of 2-((2-((2-methoxy-4-(4-(methylamino)piperidin-1-yl)phenyl)amino)-5-(trifluoromethyl)pyridin-4-yl)amino)-N-methylbenzamide (GT-M-172)

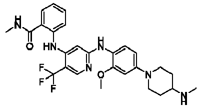

2-((2-((2-methoxy-4-(4-(methylamino)piperidin-1-yl)phenyl)amino)-5-(trifluoromethyl)pyridin-4-yl)amino)-N-methylbenzamide (GT-M-172) was prepared by referring to Scheme 16 (white solid, 1.8 g). ¹H NMR (400 MHz, DMSO) δ 10.60 (s, 1H), 9.98 (s, 1H), 9.38 (s, 2H), 8.83 (d, *J* = 4.5 Hz, 1H), 8.17 (s, 1H), 7.78 (d, *J* = 7.8 Hz, 1H), 7.57 (d, *J* = 3.9 Hz, 2H), 7.34 - 7.15 (m, 2H), 6.87 (d, *J* = 54.9 Hz, 2H), 6.59 (s, 1H), 3.82 (s, 5H), 3.21 (s, 1H), 2.98 (s, 2H), 2.69 (t, *J* = 41.5 Hz, 4H), 2.54 (t, *J =* 5.3 Hz, 3H), 2.17 (s, 2H), 1.84 (s, 2H). LCMS (ESI): calcd., 529.3; found, 529.3.

### Intermediate Example 30: preparation of N-methyl-N-(3-(((2-((4-(piperazin-1-yl)phenyl)amino)-5-(trifluoromethyl)pyrimidin-4-yl)amino)methyl)pyrazin-2-yl)methanesulfonamide (GT-M-151)

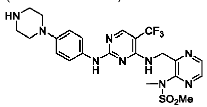

N-methyl-N-(3-(((2-((4-(piperazin-1-yl)phenyl)amino)-5-(trifluoromethyl)pyrimidin-4-yl)amino)methyl)pyrazin-2-yl)methanesulfonamide (GT-M-151) was prepared by referring to Scheme 19 (white solid, 1.7 g). ¹H NMR(400 MHz, MeOD) δ 8.60 (s, 1H), 8.56 (s, 1H), 8.30 (s, 1H), 7.41 (d, *J=* 8.3 Hz, 2H), 7.20 (d, *J* = 8.6 Hz, 2H), 5.13 (s, 2H), 3.59 (d, *J* = 4.7 Hz, 4H), 3.49 (s, 4H), 3.20 (d, *J* = 16.1 Hz, 3H), 3.13 (s, 3H). LCMS (ESI): calcd., 538.20; found, 538.3.

### Intermediate Example 31: preparation of N-methyl-N-(3-(((2-((4-(piperazin-1-yl)phenyl)amino)-5-(trifluoromethyl)pyrimidin-4-yl)amino)methyl)phenyl)methanesulfonamide (GT-M-152)

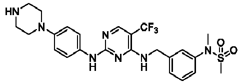

N-methyl-N-(3-(((2-((4-(piperazin-1-yl)phenyl)amino)-5-(trifluoromethyl)pyrimidin-4-yl)amino)methyl)phenyl)methanesulfonamide (GT-M-152) was prepared by referring to Scheme 19 (brown solid, 1.2 g). ¹H NMR (400 MHz, DMSO) δ 10.92 (s, 1H), 9.52 (s, 2H), 9.06 (s, 1H), 8.48 (s, 1H), 7.43 - 7.25 (m, 6H), 7.18 (s, 1H), 6.97 (d, J = 8.3 Hz, 2H), 4.64 (d, J = 5.2 Hz, 2H), 3.37 (s, 4H), 3.20 (s, 4H), 3.15 (s, 3H), 2.87 (s, 3H). LCMS (ESI): calcd., 536.2; found, 536.7.

### Intermediate Example 32: preparation of N-methyl-N-(3-(((2-((4-(4-(methylamino)piperidin-1-yl)phenyl)amino)-5-(trifluoromethyl)pyrimidin-4-yl)amino)methyl)phenyl)methanesulfonamide (GT-M-153)

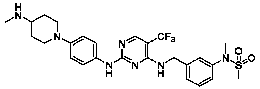

N-methyl-N-(3-(((2-((4-(4-(methylamino)piperidin-1-yl)phenyl)amino)-5-(trifluoromethyl)pyrimidin-4-yl)amino)methyl)phenyl)methanesulfonamide (GT-M-153) was prepared by referring to Scheme 19 (white solid, 1.069 g). ¹H NMR (400 MHz, MeOD) δ 8.36 (d, *J* = 22.7 Hz, 1H), 7.77 (t, *J =* 8.4 Hz, 2H), 7.55 (d, *J =* 8.5 Hz, 2H), 7.47 (t, *J =* 7.9 Hz, 1H), 7.35 (d, *J =* 7.8 Hz, 1H), 7.26 (s, 2H), 7.14 (s, 1H), 4.75 (s, 2H), 3.82 (dd, *J* = 24.4, 12.5 Hz, 4H), 3.63 (d, *J* = 11.4 Hz, 1H), 3.27 (d, *J =* 8.0 Hz, 3H), 2.89 (d, *J* = 18.3 Hz, 3H), 2.81 (s, 3H), 2.50 (d, *J* = 13.0 Hz, 2H), 2.37 (d, *J*= 10.7 Hz, 2H). LCMS (ESI): calcd., 564.2; found, 564.1.

### Intermediate Example 33: preparation of N-methyl-N-(3-(((2-((4-(4-(methylamino)piperidin-1-yl)phenyl)amino)-5-(trifluoromethyl)pyrimidin-4-yl)amino)methyl)pyrazin-2-yl)methanesulfonamide (GT-M-174)

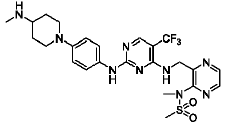

N-methyl-N-(3-(((2-((4-(4-(methylamino)piperidin-1-yl)phenyl)amino)-5-(trifluoromethyl)pyrimidin-4-yl)amino)methyl)pyrazin-2-yl)methanesulfonamide (GT-M-174) was prepared by referring to Scheme 19 (white solid, 707 mg). ¹H NMR (400 MHz, MeOD) δ 8.58 (dd, *J* = 17.7, 2.3 Hz, 2H), 8.39 (s, 1H), 7.76 (dd, *J* = 44.2, 9.0 Hz, 4H), 5.16 (s, 2H), 3.83 (d, *J* = 13.7 Hz, 4H), 3.27 (s, 3H), 3.16 (s, 3H), 2.80 (s, 3H), 2.55 - 2.33 (m, 4H). LCMS (ESI): calcd., 566.2; found, 566.3.

### Intermediate Example 34: preparation of N-methyl-N-(3-(((2-((4-(4-(piperazin-1-yl)piperidin-1-yl)phenyl)amino)-5-(trifluoromethyl)pyrimidin-4-yl)amino)methyl)pyrazin-2-yl)methanesulfonamide (GT-M-175)

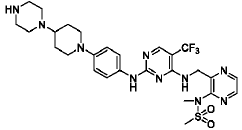

N-methyl-N-(3-(((2-((4-(4-(piperazin-1-yl)piperidin-1-yl)phenyl)amino)-5-(trifluoromethyl)pyrimidin-4-yl)amino)methyl)pyrazin-2-yl)methanesulfonamide (GT-M-175) was prepared by referring to Scheme 19 (brown solid, 560 mg). ¹H NMR (400 MHz, MeOD) δ 8.58 (dd, *J* = 18.6, 2.4 Hz, 1H), 8.37 (s, 1H), 7.66 (s, 2H), 5.15 (s, 1H), 4.00 - 3.60 (m, 6H), 3.26 (s, 2H), 3.16 (s, 2H), 2.54 (dd, *J* = 43.4, 11.6 Hz, 2H). LCMS (ESI): calcd., 621.3; found, 621.4.

### Intermediate Example 35: preparation of N⁴-(3-(methylsulfonyl)benzyl)-N²-(4-(piperazin-1-yl)phenyl)-5-(trifluoromethyl)pyrimidine-2,4-diamine (GT-M-191)

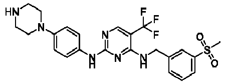

N⁴-(3-(methylsulfonyl)benzyl)-N²-(4-(piperazin-1-yl)phenyl)-5-(trifluoromethyl)pyrimidine-2,4-diamine (GT-M-191) was prepared by referring to Scheme 19 (purple solid, 500 mg). ¹H NMR (400 MHz, DMSO) δ 9.78 (s, 1H), 8.62 (s, 3H), 8.26 (s, 1H), 7.90 (s, 1H), 7.81 (d, *J* = 7.1 Hz, 1H), 7.61 (t, *J* = 7.6 Hz, 2H), 7.31 (d, *J* = 7.9 Hz, 2H), 6.91 (d, *J* = 8.7 Hz, 2H), 4.71 (d, *J* = 5.5 Hz, 2H), 3.35 - 3.20 (m, 8H), 3.16 (d, *J* = 11.3 Hz, 3H). LCMS (ESI): calcd., 507.2; found, 507.4.

### Intermediate Example 36: preparation of N-methyl-N-(3-(((2-((4-(piperidin-4-yl)phenyl)amino)-5-(trifluoromethyl)pyrimidin-4-yl)amino)methyl)phenyl)methanesulfonamide (GT-M-193)

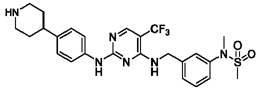

N-methyl-N-(3-(((2-((4-(piperidin-4-yl)phenyl)amino)-5-(trifluoromethyl)pyrimidin-4-yl)amino)methyl)phenyl)methanesulfonamide (GT-M-193) was prepared by referring to Scheme 20 (white solid, 0.87 g). ¹H NMR (400 MHz, DMSO) δ 9.75 (s, 1H), 8.63 (s, 1H), 8.38 (s, 1H), 8.25 (s, 1H), 7.98 (s, 1H), 7.51 (d, *J* = 8.0 Hz, 2H), 7.41 - 7.31 (m, 2H), 7.24 (dd, *J* = 16.3, 7.7 Hz, 2H), 7.09 (d, *J =* 8.4 Hz, 2H), 4.66 (d, *J =* 5.6 Hz, 2H), 3.37 (d, *J* = 12.2 Hz, 2H), 3.15 (s, 3H), 3.00 (q, *J =* 11.9 Hz, 2H), 2.86 (s, 3H), 2.77 (t, *J* = 12.0 Hz, 1H), 1.92 (d, *J* = 13.5 Hz, 2H), 1.73 (dd, *J* = 22.9, 12.5 Hz, 2H). LCMS (ESI): calcd., 535.2; found, 535.3.

### Intermediate Example 37: preparation of 4-(((4-(5-chloro-2-(piperidin-4-ylamino)pyridin-4-yl)thiazol-2-yl)amino)methyl)tetrahydro-2H-pyran-4-carbonitrile (GT-M-158)

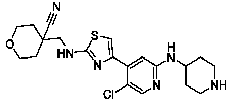

4-(((4-(5-chloro-2-(piperidin-4-ylamino)pyridin-4-yl)thiazol-2-yl)amino)methyl)tetrahydro-2H-pyran-4-carbonitrile (GT-M-158) was prepared by referring to Scheme 22 (yellow solid, 900 mg). ¹H NMR (400 MHz, DMSO) δ 8.52 (s, 1H), 8.34 (s, 1H), 8.12 (t, J = 6.2 Hz, 1H), 8.02 (s, 1H), 7.39 (s, 1H), 7.11 (s, 1H), 6.99 (s, 1H), 3.92 (s, 2H), 3.68 (d, *J* = 6.1 Hz, 2H), 3.49 (t, *J* = 11.3 Hz, 3H), 3.31 (d, J = 12.7 Hz, 2H), 3.03 (d, J = 10.3 Hz, 2H), 2.07 (d, J = 10.8 Hz, 2H), 1.88 (d, J = 13.4 Hz, 2H), 1.77 - 1.59 (m, 4H). LCMS (ESI): calcd for C₂₀H₂₅ClN₆OS⁺ [M+H]⁺: 433.16; found, 433.1.

### Intermediate Example 38: preparation of 4-(((5'-chloro-2'-(piperidin-4-ylamino)-[2,4'-bipyridin]-6-yl)amino)methyl)tetrahydro-2H-pyran-4-carbonitrile (GT-M-159)

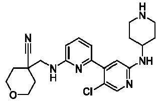

4-(((5'-chloro-2'-(piperidin-4-ylamino)-[2,4'-bipyridin]-6-yl)amino)methyl)tetrahydro-2H-pyran-4-carbonitrile (GT-M-159) was prepared by referring to Scheme 21 (yellow solid, 1.5 g, yield 86.9%). ¹H NMR (400 MHz, DMSO) δ 8.55 (s, 1H), 8.39 (s, 1H), 8.04 (s, 1H), 7.57 - 7.43 (m, 1H), 7.10 (t, *J=* 6.4 Hz, 1H), 6.95 (d, *J=* 7.3 Hz, 1H), 6.76 (d, *J* = 7.2 Hz, 1H), 6.67 (d, *J=* 10.0 Hz, 2H), 4.08 - 3.80 (m, 3H), 3.66 (d, *J=* 6.4 Hz, 2H), 3.46 (dd, *J=* 11.9, 10.5 Hz, 2H), 3.29 (s, 2H), 3.04 (dd, *J* = 18.8, 7.9 Hz, 2H), 2.07 (d, *J* = 10.8 Hz, 2H), 1.84 (d, *J* = 13.2 Hz, 2H), 1.73 - 1.46 (m, 4H). LCMS (ESI): calcd., 427.2; found, 427.2.

### Intermediate Example 39: preparation of 2-(5-fluoro-2-hydroxyphenyl)-2-(1-oxo-6-(4-(piperazin-1-yl)phenyl)isoindolin-2-yl)-N-(thiazol-2-yl)acetamide (GT-D-012)

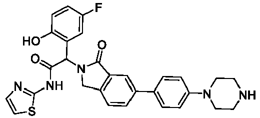

2-(5-fluoro-2-hydroxyphenyl)-2-(1-oxo-6-(4-(piperazin-1-yl)phenyl)isoindolin-2-yl)-N-(thiazol-2-yl)acetamide (GT-D-012) was prepared by referring to Scheme 23 (yellow solid, 110 mg). ¹H NMR (400 MHz, DMSO) δ 10.20 - 8.61 (m, 1H), 7.87 (d, *J* = 8.5 Hz, 2H), 7.63 (dd, *J* = 11.3, 8.4 Hz, 4H), 7.49 (d, *J* = 3.5 Hz, 1H), 7.27 (d, *J* = 3.6 Hz, 1H), 7.12 (td, *J* = 8.6, 3.1 Hz, 1H), 7.07 (d, *J* = 8.8 Hz, 2H), 6.92 (dd, *J* = 8.9, 4.8 Hz, 1H), 6.87 (dd, *J* = 9.2, 3.0 Hz, 1H), 6.33 (s, 1H), 4.64 (d, *J* = 17.5 Hz, 1H), 4.02 (d, *J* = 17.5 Hz, 1H), 3.29 (d, *J* = 4.4 Hz, 5H), 3.12 - 3.00 (m, 6H), 1.24 (s, 1H). LCMS (ESI): calcd., 544.2; found, 544.2.

### Intermediate Example 40: preparation of 2-(5-fluoro-2-hydroxyphenyl)-2-(1-oxo-6-(6-(piperazin-1-yl)pyridazin-3-yl)isoindolin-2-yl)-N-(thiazol-2-yl)acetamide (GT-D-014)

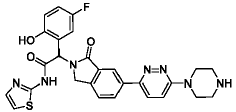

2-(5-fluoro-2-hydroxyphenyl)-2-(1-oxo-6-(6-(piperazin-1-yl)pyridazin-3-yl)isoindolin-2-yl)-N-(thiazol-2-yl)acetamide (GT-D-014) was prepared by referring to Scheme 24 (brown solid, 130 mg). ¹H NMR (400 MHz, DMSO) δ 8.31 (d, *J* = 8.3 Hz, 2H), 8.06 (d, *J* = 9.6 Hz, 1H), 7.68 (d, *J* = 7.9 Hz, 1H), 7.48 (d, *J* = 3.6 Hz, 1H), 7.33 (d, *J* = 9.7 Hz, 1H), 7.25 (d, *J* = 3.5 Hz, 1H), 7.11 (td, *J* = 8.6, 3.1 Hz, 1H), 6.94 - 6.84 (m, 2H), 6.33 (s, 1H), 4.69 (d, *J* = 17.8 Hz, 1H), 4.07 (d, *J* = 17.8 Hz, 1H), 3.66 - 3.54 (m, 4H), 3.17 (s, 1H), 2.91 - 2.78 (m, 4H), 1.23 (s, 1H). LCMS (ESI): calcd., 546.2; found, 546.2.

### Intermediate Example 41: preparation of 2-(5-fluoro-2-hydroxyphenyl)-2-(1-oxo-6-(piperazin-1-yl)isoindolin-2-yl)-N-(thiazol-2-yl)acetamide (GT-D-69)

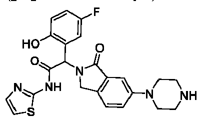

2-(5-fluoro-2-hydroxyphenyl)-2-(1-oxo-6-(piperazin-1-yl)isoindolin-2-yl)-N-(thiazol-2-yl)acetamide (GT-D-69) was prepared by referring to Scheme 25 (yellow solid, 515 mg). ¹H NMR (400 MHz, DMSO) δ 7.48 (d, *J* = 3.5 Hz, 1H), 7.39 (d, *J* = 8.4 Hz, 1H), 7.24 (dd, *J =* 11.9, 2.8 Hz, 2H), 7.15 (d, *J* = 1.8 Hz, 1H), 7.13 - 7.05 (m, 1H), 6.90 (dd, *J* = 8.9, 4.8 Hz, 1H), 6.82 (dd, *J* = 9.2, 2.9 Hz, 1H), 6.29 (s, 1H), 4.50 (d, *J* = 17.0 Hz, 1H), 3.89 (d, *J* = 17.0 Hz, 2H), 3.13 (s, 5H), 2.91 (s, 5H), 1.24 (s, 1H). LCMS (ESI): calcd., 468.2; found, 468.2.

### Intermediate Example 42: preparation of 2-(5-fluoro-2-hydroxyphenyl)-2-(6-(4-(methylamino)piperidin-1-yl)-1-oxoisoindolin-2-yl)-N-(thiazol-2-yl)acetamide (GT-D-70)

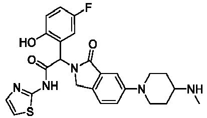

2-(5-fluoro-2-hydroxyphenyl)-2-(6-(4-(methylamino)piperidin-1-yl)-1-oxoisoindolin-2-yl)-N-(thiazol-2-yl)acetamide (GT-D-70) was prepared by referring to Scheme 25 (brown solid, 186 mg). ¹H NMR (400 MHz, DMSO) δ 7.40 (dd, *J* = 29.5, 5.8 Hz, 2H), 7.22 (d, *J* = 7.5 Hz, 1H), 7.16 (d, *J* = 19.7 Hz, 3H), 7.05 (d, *J* = 6.2 Hz, 1H), 6.91 - 6.76 (m, 2H), 6.24 (s, 1H), 4.54 (d, *J* = 17.0 Hz, 1H), 3.94 (d, *J =* 17.0 Hz, 1H), 3.67 (d, *J =* 12.1 Hz, 2H), 3.17 (s, 1H), 2.78 (t, *J* = 11.3 Hz, 2H), 2.31 (d, *J =* 5.8 Hz, 3H), 1.89 (d, *J* = 11.2 Hz, 2H), 1.35 (d, *J* = 11.1 Hz, 2H). LCMS (ESI): calcd., 496.2; found, 496.2.

### Intermediate Example 43: preparation of 2-(5-fluoro-2-hydroxyphenyl)-2-(1-oxo-6-(4-(piperazin-1-yl)piperidin-1-yl)isoindolin-2-yl)-N-(thiazol-2-yl)acetamide (GT-D-71)

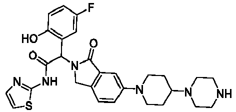

2-(5-fluoro-2-hydroxyphenyl)-2-(1-oxo-6-(4-(piperazin-1-yl)piperidin-1-yl)isoindolin-2-yl)-N-(thiazol-2-yl)acetamide (GT-D-71) was prepared by referring to Scheme 25 (brown solid, 110 mg). ¹H NMR (400 MHz, DMSO) δ 7.45 (s, 1H), 7.36 (d, *J* = 8.4 Hz, 1H), 7.22 (d, *J* = 9.5 Hz, 4H), 7.07 (s, 1H), 6.91 - 6.77 (m, 3H), 6.25 (s, 1H), 4.51 (d, *J =* 17.1 Hz, 1H), 3.91 (d, *J =* 16.9 Hz, 1H), 3.77 (d, *J =* 12.1 Hz, 3H), 3.17 (s, 2H), 2.72 (s, 5H), 2.44 (s, 5H), 1.82 (d, *J* = 10.7 Hz, 2H), 1.51 (d, *J* = 11.4 Hz, 3H), 1.25 (s, 1H). LCMS (ESI): calcd., 551.2; found, 551.2.

### Intermediate Example 44: preparation of 9-ethyl-6,6-dimethyl-11-oxo-8-(piperazin-1-yl)-6,11-dihydro-5H-benzo[b]carbazole-3-carbonitrile (GT-D-42)

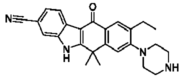

9-ethyl-6,6-dimethyl-11-oxo-8-(piperazin-1-yl)-6,11-dihydro-SH-benzo[b]carbazole-3-carbonitrile (GT-D-42) was prepared by referring to Scheme 27 (white solid,240 mg). ¹H NMR (400 MHz, DMSO) δ 13.03 (s, 1H), 9.39 (s, 2H), 8.32 (d, *J* = 8.1 Hz, 1H), 8.05 (d, *J* = 29.2 Hz, 2H), 7.61 (d, *J* = 9.0 Hz, 1H), 7.38 (s, 1H), 3.24 (d, *J* = 14.9 Hz, 8H), 2.73 (d, *J* = 7.5 Hz, 2H), 1.79 (s, 6H), 1.29 (t, *J=* 7.5 Hz, 3H). LCMS (ESI): calcd., 399.2; found, 399.2.

### Intermediate Example 45: preparation of 9-ethyl-6,6-dimethyl-11-oxo-8-(4-(piperazin-1-yl)piperidin-1-yl)-6,11-dihydro-5H-benzo[b]carbazole-3-carbonitrile (GT-D-113)

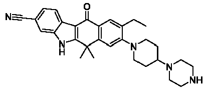

9-ethyl-6,6-dimethyl-11-oxo-8-(4-(piperazin-1-yl)piperidin-1-yl)-6,11-dihydro-5H-benzo[b]carbazole-3-carbonitrile (GT-D-113) was prepared by referring to Scheme 27 (off-white solid, 1.4 g). ¹H NMR (400 MHz, MeOD) δ 8.39 (d, *J* = 8.2 Hz, 1H), 8.23 (s, 1H), 7.86 (s, 1H), 7.62 - 7.48 (m, 2H), 3.75 (s, 9H), 3.53 (d, *J =* 12.0 Hz, 2H), 3.18 (t, *J =* 11.7 Hz, 2H), 2.86 (q, *J =* 7.4 Hz, 2H), 2.43 (d, *J* = 11.4 Hz, 2H), 2.22 (dd, *J* = 20.7, 11.9 Hz, 2H), 1.82 (s, 6H), 1.37 (t, *J* = 7.5 Hz, 3H). LCMS (ESI): calcd., 482.3; found, 482.2.

### Intermediate Example 46: preparation of 9-ethyl-6,6-dimethyl-8-(4-(methylamino)piperidin-1-yl)-11-oxo-6,11-dihydro-SH-benzo[b]carbazole-3-carbonitrile (GT-D-85)

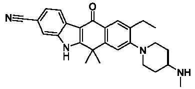

9-ethyl-6,6-dimethyl-8-(4-(methylamino)piperidin-1-yl)-11-oxo-6,11-dihydro-5H-benzo[b]carbazole-3-carbonitrile (GT-D-85) was prepared by referring to Scheme 27 (white solid, 130 mg). ¹H NMR (400 MHz, MeOD) δ 8.31 (d, *J* = 8.2 Hz, 1H), 8.11 (s, 1H), 7.77 (s, 1H), 7.45 (d, *J* = 8.2 Hz, 1H), 7.36 (s, 1H), 3.29 (d, *J* = 11.9 Hz, 2H), 2.91 (t, *J* = 11.7 Hz, 2H), 2.78 - 2.64 (m, 5H), 2.18 (d, *J* = 11.7 Hz, 2H), 1.86 - 1.75 (m, 2H), 1.71 (s, 6H), 1.25 (t, *J* = 7.5 Hz, 3H), 1.22 - 1.18 (m, 1H). LCMS (ESI): calcd., 427.3; found, 427.2.

### Intermediate Example 47: preparation of 9-cyclopentyl-N⁸-phenyl-N²-(4-(piperazin-1-yl)phenyl)-9H-purine-2,8-diamine (GT-D-62)

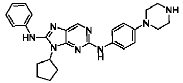

9-cyclopentyl-N⁸-phenyl-N²-(4-(piperazin-1-yl)phenyl)-9H-purine-2,8-diamine (GT-D-62) was prepared by referring to Scheme 29 (white solid, 1.18 g). ¹H NMR (400 MHz, MeOD) δ 8.15 (s, 1H), 7.67 - 7.52 (m, 4H), 7.46 (dd, *J* = 15.9, 8.0 Hz, 3H), 7.25 (d, *J* = 9.0 Hz, 2H), 3.66 (s, 1H), 3.61 - 3.53 (m, 4H), 3.47 (dd, *J* = 6.5, 3.6 Hz, 4H), 2.40 (dd, *J* = 12.8, 7.3 Hz, 2H), 2.20 (d, *J* = 7.9 Hz, 2H), 1.92 (s, 2H), 1.77 - 1.62 (m, 2H). LCMS (ESI): calcd., 455.3; found, 455.0.

### Intermediate Example 48: preparation of 9-cyclopentyl-N²-(4-(4-(methylamino)piperidin-1-yl)phenyl)-N⁸-phenyl-9H-purine-2,8-diamine (GT-D-67)

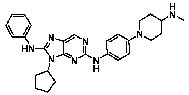

9-cyclopentyl-N²-(4-(4-(methylamino)piperidin-1-yl)phenyl)-N⁸-phenyl-9H-purine-2,8-diamine (GT-D-67) was prepared by referring to Scheme 29 (yellow solid, 4.6 g). ¹H NMR (400 MHz, MeOD) δ 8.34 (s, 1H), 7.94 - 7.82 (m, 4H), 7.57 (dd, *J=* 7.4, 6.3 Hz, 4H), 7.45 (dd, *J=* 7.7, 4.2 Hz, 1H), 3.95 - 3.81 (m, 4H), 3.71 - 3.60 (m, 2H), 2.89 (s, 1H), 2.81 (s, 3H), 2.51 (t, *J=* 15.4 Hz, 4H), 2.43 - 2.37 (m, 2H), 2.25 (d, *J* = 8.3 Hz, 2H), 2.09 - 2.02 (m, 2H), 1.83 -1.71 (m, 2H). LCMS (ESI): calcd., 483.3; found, 483.3.

### Intermediate Example 49: preparation of 9-cyclopentyl-N⁸-phenyl-N²-(4-(4-(piperazin-1-yl)piperidin-1-yl)phenyl)-9H-purine-2,8-diamine (GT-D-68)

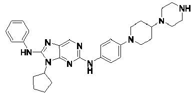

9-cyclopentyl-N⁸-phenyl-N²-(4-(4-(piperazin-1-yl)piperidin-1-yl)phenyl)-9H-purine-2,8-diamine (GT-D-68) was prepared by referring to Scheme 29 (yellow solid, 4.1 g). ¹H NMR(400 MHz, DMSO) δ 7.62 (d, *J=* 8.9 Hz, 2H), 7.56 (t, *J=* 3.7 Hz, 3H), 7.01 - 6.94 (m, 2H), 6.80 (d, *J=* 9.0 Hz, 2H), 6.42 (t, *J* = 7.1 Hz, 1H), 3.53 (d, *J* = 11.9 Hz, 2H), 2.67 (s, 4H), 2.54 (d, *J* = 10.1 Hz, 2H), 2.42 (s, 2H), 2.38 - 2.31 (m, 6H), 2.20 (t, *J* = 11.2 Hz, 1H), 1.99 (s, 2H), 1.92 - 1.87 (m, 1H), 1.81 (d, *J =* 10.0 Hz, 4H), 1.69 - 1.61 (m, 2H), 1.52 (dt, *J* = 11.7, 8.7 Hz, 2H). LCMS (ESI): calcd., 538.3; found, 538.4.

### Intermediate Example 50: preparation of 2-(4-phenoxyphenyl)-6-(piperidin-4-yl)nicotinamide (GT-D-66)

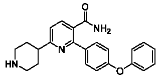

2-(4-phenoxyphenyl)-6-(piperidin-4-yl)nicotinamide (GT-D-66) was prepared by referring to Scheme 30 (white solid, 1.0 g). ¹H NMR (400 MHz, MeOD) δ 8.53 (d, *J* = 8.2 Hz, 1H), 7.93 (d, *J =* 8.2 Hz, 1H), 7.75 (d, *J* = 8.8 Hz, 2H), 7.44 (t, *J* = 8.0 Hz, 2H), 7.23 (t, *J =* 7.4 Hz, 1H), 7.18 - 7.04 (m, 4H), 3.60 (d, *J =* 12.7 Hz, 2H), 3.52 (t, *J =* 12.0 Hz, 1H), 3.24 (t, *J =* 11.8 Hz, 2H), 2.31 (d, *J =* 13.5 Hz, 2H), 2.26 - 2.09 (m, 2H). LCMS (ESI): calcd., 374.2; found, 374.2.

### Intermediate Example 51: preparation of 2-(4-phenoxyphenyl)-6-(piperazin-1-yl)nicotinamide (GT-D-76)

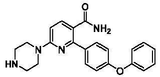

2-(4-phenoxyphenyl)-6-(piperazin-1-yl)nicotinamide (GT-D-76) was prepared by referring to Scheme 30 (yellow solid, 436 mg). ¹H NMR (400 MHz, MeOD) δ 8.14 (d, *J=* 9.2 Hz, 1H), 7.69 (d, *J=* 8.7 Hz, 2H), 7.47 - 7.33 (m, 3H), 7.20 (t, *J* = 7.4 Hz, 1H), 7.09 (t, *J* = 7.8 Hz, 4H), 4.12 - 4.03 (m, 4H), 3.49 - 3.43 (m, 5H). LCMS (ESI): calcd., 375.2; found, 375.2.

### Intermediate Example 52: preparation of 6-(4-(methylamino)piperidin-1-yl)-2-(4-phenoxyphenyl)nicotinamide (GT-D-77)

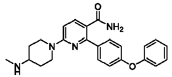

6-(4-(methylamino)piperidin-1-yl)-2-(4-phenoxyphenyl)nicotinamide (GT-D-77) was prepared by referring to Scheme 30 (white solid, 690 m). ¹H NMR (400 MHz, CDCl₃) δ 7.99 (d, *J* = 8.9 Hz, 1H), 7.65 (d, *J* = 8.6 Hz, 2H), 7.39 (t, *J* = 7.9 Hz, 2H), 7.17 (t, *J* = 7.4 Hz, 1H), 7.08 (dd, *J* = 14.0, 8.2 Hz, 4H), 6.65 (d, *J* = 8.9 Hz, 1H), 5.50 - 5.19 (m, 2H), 4.42 (d, *J* = 13.4 Hz, 2H), 3.18 - 2.90 (m, 2H), 2.78 - 2.58 (m, 1H), 2.00 (d, *J=* 10.2 Hz, 2H), 1.52 - 1.25 (m, 4H). LCMS (ESI): calcd., 403.2; found, 403.2.

### Intermediate Example 53: preparation of 2-(4-phenoxyphenyl)-6-(4-(piperazin-1-yl)piperidin-1-yl)nicotinamide (GT-D-78)

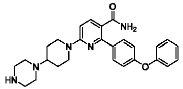

2-(4-phenoxyphenyl)-6-(4-(piperazin-1-yl)piperidin-1-yl)nicotinamide (GT-D-78) was prepared by referring to Scheme 30 (white solid, 355 mg). ¹H NMR (400 MHz, CDCl₃) δ 7.89 (d, *J* = 8.8 Hz, 1H), 7.55 (d, *J* = 8.7 Hz, 2H), 7.30 (t, *J* = 8.0 Hz, 2H), 7.09 (d, *J* = 7.4 Hz, 1H), 7.04 - 6.91 (m, 4H), 6.56 (d, *J* = 8.9 Hz, 1H), 5.41 (s, 1H), 5.21 (d, *J* = 12.3 Hz, 1H), 4.45 (d, *J* = 13.2 Hz, 2H), 2.95 - 2.76 (m, 7H), 2.57 (d, *J* = 4.4 Hz, 4H), 2.44 (dd, *J =* 15.1, 7.5 Hz, 1H), 1.85 (d, *J =* 11.8 Hz, 2H), 1.46 (qd, *J* = 12.4, 3.9 Hz, 2H). LCMS (ESI): calcd., 458.3; found, 458.2.

### Intermediate Example 54: preparation of N-(2-(1-(cyclopropylsulfonyl)-1H-pyrazol-4-yl)pyrimidin-4-yl)-1-isopropyl-3-(piperazin-1-yl)-1H-pyrazolo[4,3-c]pyridin-6-amine (GT-D-18)

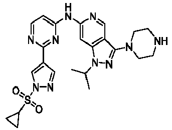

N-(2-(1-(cyclopropylsulfonyl)-1H-pyrazol-4-yl)pyrimidin-4-yl)-1-isopropyl-3-(piperazin-1-yl)-1H-pyrazolo[4,3-c]pyridin-6-amine (GT-D-18) was prepared by referring to Scheme 26 (yellow solid, 0.75 g). ¹H NMR (400 MHz, CDCl3) δ 8.73 (s, 1H), 8.69 (s, 1H), 8.47 (s, 1H), 8.39 (d, *J* = 5.9 Hz, 1H), 8.12 (s, 1H), 7.97 (s, 1H), 6.81 (d, *J* = 5.8 Hz, 1H), 4.65 (dt, *J=* 13.3, 6.6 Hz, 1H), 3.56 - 3.43 (m, 4H), 3.16 - 3.00 (m, 4H), 2.90 - 2.80 (m, 1H), 1.58 (d, *J* = 6.7 Hz, 6H), 1.55 - 1.49 (m, 2H), 1.26 - 1.19 (m, 2H). LCMS (ESI): calcd., 509.2; found, 509.2.

### Intermediate Example 55: preparation of N-(2-(1-(cyclopropylsulfonyl)-1H-pyrazol-4-yl)pyrimidin-4-yl)-1-isopropyl-3-(4-(methylamino)piperidin-1-yl)-1H-pyrazolo[4,3-c]pyridin-6-amine (GT-D-72)

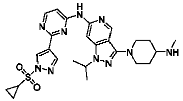

N-(2-(1-(cyclopropylsulfonyl)-1H-pyrazol-4-yl)pyrimidin-4-yl)-1-isopropyl-3-(4-(methylamino)piperidin-1-yl)-1H-pyrazolo[4,3-c]pyridin-6-amine (GT-D-72) was prepared by referring to Scheme 26 (yellow solid, 0.4 g). ¹H NMR (400 MHz, CDCl₃) δ 8.73 (s, 1H), 8.69 (s, 1H), 8.47 (s, 1H), 8.40 (d, *J* = 5.9 Hz, 1H), 8.11 (s, 1H), 7.80 (s, 1H), 6.82 (d, *J* = 5.8 Hz, 1H), 4.65 (dt, *J* = 13.3, 6.6 Hz, 1H), 4.09 (t, *J=* 12.3 Hz, 2H), 3.05 (t, *J=* 11.3 Hz, 2H), 2.85 (ddd, *J* = 12.5, 7.8, 4.7 Hz, 2H), 2.61 (s, 3H), 2.16 (d, *J* = 10.7 Hz, 2H), 1.81 (dd, *J* = 20.3, 11.2 Hz, 2H), 1.58 (d, *J* = 6.7 Hz, 6H), 1.54 (dd, *J* = 5.8, 3.7 Hz, 2H), 1.23 (dt, *J* = 7.0, 3.6 Hz, 2H). LCMS (ESI): calcd., 537.3; found, 537.4.

### Intermediate Example 56: preparation of N-(2-(1-(cyclopropylsulfonyl)-1H-pyrazol-4-yl)pyrimidin-4-yl)-1-isopropyl-3-(4-(piperazin-1-yl)piperidin-1-yl)-1H-pyrazolo[4,3-c]pyridin-6-amine (GT-D-73)

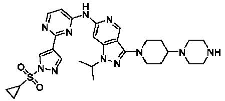

N-(2-(1-(cyclopropylsulfonyl)-1H-pyrazol-4-yl)pyrimidin-4-yl)-1-isopropyl-3-(4-(piperazin-1-yl)piperidin-1-yl)-1H-pyrazolo[4,3-c]pyridin-6-amine (GT-D-73) was prepared by referring to Scheme 26 (yellow solid, 1.19 g). ¹H NMR (400 MHz, DMSO) δ 10.37 (s, 1H), 8.86 (s, 1H), 8.70 (s, 1H), 8.48 (s, 1H), 8.41 (d, *J =* 5.9 Hz, 1H), 8.32 (s, 1H), 7.13 (d, *J =* 5.5 Hz, 1H), 4.72 (s, 1H), 4.02 (d, *J* = 12.0 Hz, 2H), 3.53 (d, *J =* 57.7 Hz, 1H), 3.33 - 3.27 (m, 2H), 2.88 (t, *J =* 11.6 Hz, 2H), 2.76 (s, 4H), 2.35 (d, *J =* 12.0 Hz, 1H), 1.87 (d, *J* = 10.4 Hz, 2H), 1.60 (d, *J =* 10.0 Hz, 2H), 1.47 (d, *J* = 6.6 Hz, 6H), 1.36 (s, 2H), 1.28 (dd, *J* = 7.5, 5.1 Hz, 2H). LCMS (ESI): calcd., 592.3; found, 592.4.

### Intermediate Example 57: preparation of 1-isopropyl-N-(2-(4-methoxypiperidin-1-yl)pyrimidin-4-yl)-3-(4-(methylamino)piperidin-1-yl)-1H-pyrazolo[4,3-c]pyridin-6-amine (GT-D-74)

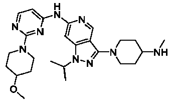

preparation of 1-isopropyl-N-(2-(4-methoxypiperidin-1-yl)pyrimidin-4-yl)-3-(4-(methylamino)piperidin-1-yl)-1H-pyrazolo[4,3-c]pyridin-6-amine (GT-D-74) was prepared by referring to Scheme 26 (yellow solid, 0.97 g). ¹H NMR (400 MHz, MeOD) δ 9.38 (s, 1H), 8.03 (d, *J=* 7.1 Hz, 1H), 7.80 (s, 1H), 6.59 (d, *J* = 7.1 Hz, 1H), 4.89 (s, 1H), 4.30 (d, *J=* 13.0 Hz, 2H), 3.89 (d, *J* = 8.7 Hz, 2H), 3.67 (d, *J =* 15.3 Hz, 2H), 3.59 (s, 1H), 3.39 (s, 3H), 3.37 - 3.32 (m, 1H), 3.20 (s, 2H), 2.77 (s, 3H), 2.27 (d, *J=* 11.6 Hz, 2H), 2.07 -1.94 (m, 2H), 1.83 (ddd, *J=* 33.0, 16.1, 8.8 Hz, 4H), 1.54 (d, *J* = 6.5 Hz, 6H). LCMS (ESI): calcd., 480.3found, 480.4.

### Intermediate Example 58: preparation of 1-isopropyl-N-(2-(4-methoxypiperidin-1-yl)pyrimidin-4-yl)-3-(4-(piperazin-1-yl)piperidin-1-yl)-1H-pyrazolo[4,3-c]pyridin-6-amine (GT-D-75)

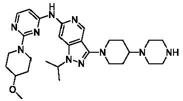

1-isopropyl-N-(2-(4-methoxypiperidin-1-yl)pyrimidin-4-yl)-3-(4-(piperazin-1-yl)piperidin-1-yl)-1H-pyrazolo[4,3-c]pyridin-6-amine (GT-D-75) was prepared by referring to Scheme 26 (yellow solid, 1.1 g). ¹H NMR (400 MHz, MeOD) δ 9.40 (s, 1H), 8.05 (d, *J* = 7.0 Hz, 1H), 7.83 (s, 1H), 6.61 (d, *J* = 7.0 Hz, 1H), 4.39 (d, *J* = 12.7 Hz, 2H), 3.93 (s, 3H), 3.67 (dd, *J* = 33.6, 15.5 Hz, 12H), 3.41 (s, 3H), 3.23 (t, *J* = 12.4 Hz, 2H), 2.41 (d, *J* = 10.4 Hz, 2H), 2.07 (dd, *J* = 23.8, 8.9 Hz, 4H), 1.79 (s, 2H), 1.57 (d, *J* = 6.4 Hz, 6H). LCMS (ESI): calcd., 535.4; found, 535.5.

### Intermediate Example 59: preparation of 7-cyclopentyl-N,N-dimethyl-2-((5-(4-(methylamino)piperidin-1-yl)pyridin-2-yl)amino)-7H-pyrrolo[2,3-d]pyrimidine-6-carboxamide (GT-D-79)

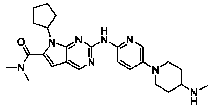

7-cyclopentyl-N,N-dimethyl-2-((5-(4-(methylamino)piperidin-1-yl)pyridin-2-yl)amino)-7H-pyrrolo[2,3-d]pyrimidine-6-carboxamide (GT-D-79) was prepared by referring to Scheme 16 (yellow solid, 800 mg). ¹H NMR (400 MHz, DMSO) δ 9.38 (s, 1H), 8.77 (s, 1H), 8.15 (d, *J* = 9.1 Hz, 1H), 8.03 (d, *J* = 2.9 Hz, 1H), 7.45 (dd, *J =* 9.1, 3.0 Hz, 1H), 6.60 (s, 1H), 4.74 (p, *J* = 8.8 Hz, 1H), 3.67 (d, *J* = 12.5 Hz, 2H), 3.51 (s, 1H), 3.06 (s, 6H), 2.90 - 2.80 (m, 1H), 2.72 (t, *J* = 11.1 Hz, 2H), 2.47 (s, 3H), 2.43 (d, *J* = 11.5 Hz, 2H), 2.05 - 1.94 (m, 6H), 1.67 -1.51 (m, 4H). LCMS (ESI): calcd., 463.3; found, 463.2.

### Intermediate Example 60: preparation of 7-cyclopentyl-N,N-dimethyl-2-((5-(4-(piperazin-1-yl)piperidin-1-yl)pyridin-2-yl)amino)-7H-pyrrolo[2,3-d]pyrimidine-6-carboxamide (GT-D-80)

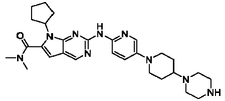

7-cyclopentyl-N,N-dimethyl-2-((5-(4-(piperazin-1-yl)piperidin-1-yl)pyridin-2-yl)amino)-7H-pyrrolo[2,3-d]pyrimidine-6-carboxamide (GT-D-80) was prepared by referring to Scheme 16 (off-white solid, 305 mg). ¹H NMR (400 MHz, DMSO) δ 9.22 (s, 1H), 8.74 (s, 1H), 8.12 (d, *J* = 9.2 Hz, 1H), 7.98 (d, *J* = 2.8 Hz, 1H), 7.42 (dd, *J* = 9.1, 2.9 Hz, 1H), 6.59 (s, 1H), 4.80 - 4.66 (m, 1H), 3.05 (s, 6H), 2.70 - 2.66 (m, 4H), 2.63 (d, *J* = 11.8 Hz, 2H), 2.42 (s, 6H), 2.24 (d, *J* = 11.4 Hz, 1H), 1.97 (s, 6H), 1.84 (d, *J=* 11.2 Hz, 2H), 1.64 (s, 2H), 1.58 - 1.50 (m, 2H), 1.23 (s, 1H). LCMS (ESI): calcd., 518.3; found, 518.4.

### Intermediate Example 61: preparation of 5,7-dimethoxy-2-(4-(piperazin-1-yl)phenyl)quinazolin-4(3H)-one (GT-D-51)

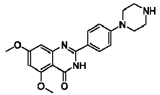

5,7-dimethoxy-2-(4-(piperazin-1-yl)phenyl)quinazolin-4(3H)-one (GT-D-51) was prepared by referring to Scheme 28 (light yellow solid, 1.0 g). ¹H NMR (400 MHz, DMSO) δ 9.40 (s, 2H), 8.14 (d, *J=* 8.8 Hz, 2H), 7.15 (d, *J=* 8.9 Hz, 2H), 7.02 (s, 1H), 6.61 (s, 1H), 3.90 (d, *J=* 7.5 Hz, 6H), 3.87 (s, 1H), 3.65 - 3.25 (m, 8H). LCMS (ESI): calcd., 367.2; found, 367.4.

### Intermediate Example 62: preparation of 5,7-dimethoxy-2-(4-(4-(methylamino)piperidin-1-yl)phenyl)quinazolin-4(3H)-one (GT-D-93)

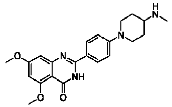

5,7-dimethoxy-2-(4-(4-(methylamino)piperidin-1-yl)phenyl)quinazolin-4(3H)-one (GT-D-93) was prepared by referring to Scheme 28 (yellow solid, 0.64 g). ¹H NMR (400 MHz, MeOD) δ 7.95 (d, *J=* 8.9 Hz, 2H), 7.10 (d, *J* = 9.0 Hz, 2H), 6.77 (d, *J* = 2.1 Hz, 1H), 6.54 (d, *J* = 2.1 Hz, 1H), 4.08 (d, *J* = 13.1 Hz, 2H), 3.92 (d, *J* = 3.2 Hz, 6H), 2.97 (t, *J =* 11.9 Hz, 2H), 2.74 (s, 3H), 2.20 (d, *J* = 10.4 Hz, 2H), 1.70 (tt, *J* = 12.2, 6.2 Hz, 2H). LCMS (ESI): calcd., 395.2; found, 395.3.

### Intermediate Example 63: preparation of 5,7-dimethoxy-2-(4-(4-(piperazin-1-yl)piperidin-1-yl)phenyl)quinazolin-4(3H)-one (GT-D-94)

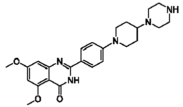

5,7-dimethoxy-2-(4-(4-(piperazin-1-yl)piperidin-1-yl)phenyl)quinazolin-4(3H)-one (GT-D-94)was prepared by referring to Scheme 28 (yellow solid, 0.26 g). ¹H NMR (400 MHz, DMSO) δ 11.74 (s, 1H), 8.90 (s, 1H), 8.09 (d, *J=* 8.8 Hz,2H), 7.03 (t, *J=* 10.9 Hz, 2H), 6.68 (d, *J* = 2.0 Hz, 1H), 6.47 (d, *J=* 1.9 Hz, 1H), 3.96 (d, *J=* 12.5 Hz, 2H), 3.88 (s, 3H), 3.84 (s, 3H), 3.05 (s, 4H), 2.83 (t, *J* = 11.6 Hz, 2H), 2.71 (s, 4H), 1.83 (d, *J* = 11.1 Hz, 2H), 1.47 (d, *J* = 9.3 Hz, 2H). LCMS (ESI): calcd., 450.3; found, 450.3.

### Intermediate Example 64: preparation of N-(3-(difluoromethyl)-1-(piperidin-4-yl)-1H-pyrazol-4-yl)-5-morpholinopyrazolo[1,5-a]pyrimidine-3-carboxamide hydrochloride (GT-D-101)

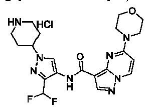

N-(3-(difluoromethyl)-1-(piperidin-4-yl)-1H-pyrazol-4-yl)-5-morpholinopyrazolo[1,5-a]pyrimidine-3-carboxamide hydrochloride (GT-D-101) was prepared by referring to Scheme 55 (yellow solid, 1.3 g). ¹H NMR (400 MHz, MeOD) δ 8.55 (d, *J* = 8.0 Hz, 1H), 8.42 (s, 1H), 8.32 (s, 1H), 7.03 - 6.75 (m, 2H), 4.60 (s, 1H), 3.86 - 3.80 (m, 8H), 3.58 (dd, *J=* 10.0, 6.5 Hz, 2H), 3.27 - 3.17 (m, 2H), 2.39 - 2.24 (m, 4H). LCMS (ESI): calcd., 447.2 ; found, 447.2.

### Intermediate Example 65: preparation of 1-(4-(4-chloro-1,2-diphenylbut-1-en-1-yl)phenyl)piperazine (GT-D-104)

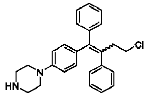

1-(4-(4-chloro-1,2-diphenylbut-1-en-1-yl)phenyl)piperazine (GT-D-104) was prepared by referring to Scheme 31 (yellow solid, 1.6 g). ¹H NMR (400 MHz, DMSO) δ 7.39 (d, *J* = 7.5 Hz, 2H), 7.29 (dd, *J* = 8.4, 7.0 Hz, 3H), 7.26 - 7.21 (m, 2H), 7.20 - 7.16 (m, 3H), 6.70 (d, *J* = 8.9 Hz, 2H), 6.63 (d, *J* = 9.0 Hz, 2H), 3.42 (t, *J* = 7.3 Hz, 2H), 3.21 - 3.14 (m, 4H), 3.08 - 2.99 (m, 4H), 2.84 (s, 2H). LCMS (ESI): calcd., 403.2; found, 403.2.

### Intermediate Example 66: preparation of 4,4'-(4-chloro-1-(4-(piperazin-1-yl)phenyl)but-1-ene-1,2-diyl)diphenol (GT-D-106)

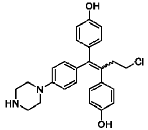

4,4'-(4-chloro-1-(4-(piperazin-1-yl)phenyl)but-1-ene-1,2-diyl)diphenol (GT-D-106) was prepared by referring to Scheme 32 (brown solid, 0.98 g). ¹H NMR (400 MHz, DMSO) δ 9.37 (d, *J* = 50.2 Hz, 2H), 8.70 (s, 1H), 6.97 (dd, *J=* 34.1, 8.4 Hz, 4H), 6.77 - 6.58 (m, 6H), 6.52 - 6.40 (m, 1H), 4.10 (d, *J* = 4.9 Hz, 1H), 3.43 (t, *J* = 6.5 Hz, 2H), 3.36 (s, 1H), 3.22 (d, *J* = 4.3 Hz, 4H), 3.16 (dd, *J =* 10.2, 4.0 Hz, 4H), 2.81 (t, *J* = 7.3 Hz, 2H). LCMS (ESI): calcd., 435.2; found, 435.2.

### Intermediate Example 67: preparation of (Z)-4-(1-(4-(2-aminoethoxy)phenyl)-4-chloro-2-phenylbut-1-en-1-yl)phenol (GT-D-186)

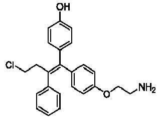

(Z)-4-(1-(4-(2-aminoethoxy)phenyl)-4-chloro-2-phenylbut-1-en-1-yl)phenol (GT-D-186) was prepared by referring to Scheme 33 (light yellow solid, 100 mg). ¹H NMR (400 MHz, DMSO) δ 7.19 (dt, *J* = 14.2, 7.6 Hz, 6H), 7.07 (d, *J* = 8.5 Hz, 1H), 6.96 (d, *J* = 8.7 Hz, 1H), 6.75 (dd, *J =* 17.5, 8.6 Hz, 2H), 6.61 (dd, *J* = 8.7, 6.6 Hz, 2H), 6.42 (d, *J* = 8.6 Hz, 1H), 3.97 (t, *J =* 5.6 Hz, 1H), 3.79 (t, *J =* 5.6 Hz, 1H), 3.44 (t, *J=* 7.4 Hz, 2H), 2.97 - 2.78 (m, 4H). LCMS (ESI): calcd., 394.2; found, 394.2.

### Intermediate Example 68: preparation of 3-(imidazo[1,2-b]pyridazin-3-ylethynyl)-4-methyl-N-(4-(piperazin-1-ylmethyl)-3-(trifluoromethyl)phenyl)benzamide hydrochloride (GT-D-109)

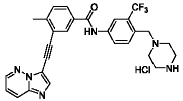

3-(imidazo[1,2-b]pyridazin-3-ylethynyl)-4-methyl-N-(4-(piperazin-1-ylmethyl)-3-(trifluoromethyl)phenyl)benzamide hydrochloride (GT-D-109) was prepared by referring to Scheme 35 (off-white solid, 670 mg). ¹H NMR (400 MHz, MeOD) δ 9.10 (dd, *J* = 4.5, 1.3 Hz, 1H), 8.62 (s, 1H), 8.52 (dd, *J=* 9.3, 1.3 Hz, 1H), 8.39 (d, *J=* 1.8 Hz, 1H), 8.24 (d, *J=* 1.8 Hz, 1H), 8.14 (d, *J=* 1.8 Hz, 1H), 8.09 (d, *J* = 8.6 Hz, 1H), 7.98 (ddd, *J* = 13.9, 8.7, 3.2 Hz, 2H), 7.55 (d, *J* = 8.1 Hz, 1H), 4.53 (s, 2H), 3.60 (dd, *J* = 26.0, 12.5 Hz, 8H), 2.69 (s, 3H). LCMS (ESI): calcd., 519.2; found, 519.4.

### Intermediate Example 69: preparation of 3-(imidazo[1,2-b]pyridazin-3-ylethynyl)-4-methyl-N-(4-(piperazin-1 -yl)-3 -(trifluoromethyl)phenyl)benzamide (GT-D-110)

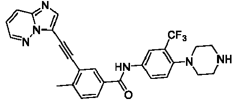

3-(imidazo[1,2-b]pyridazin-3-ylethynyl)-4-methyl-N-(4-(piperazin-1-yl)-3-(trifluoromethyl)phenyl)benzamide (GT-D-110) was prepared by referring to Scheme 34 (off-white solid, 930 mg). ¹H NMR (400 MHz, MeOD) δ 9.10 - 9.00 (m, 1H), 8.55 (s, 1H), 8.47 (dd, J = 9.3, 1.3 Hz, 1H), 8.22 (d, J = 1.8 Hz, 1H), 8.15 (d, J = 2.4 Hz, 1H), 8.05 (dd, J = 8.7, 2.4 Hz, 1H), 7.97 (dd, J = 8.0, 1.9 Hz, 1H), 7.89 (dd, J = 9.3, 4.5 Hz, 1H), 7.65 (t, J = 6.5 Hz, 1H), 7.59 - 7.56 (m, 1H), 3.38 - 3.34 (m, 4H), 3.23 - 3.14 (m, 4H), 2.68 (s, 3H). LCMS (ESI): calcd., 505.2; found, 505.3.

### Intermediate Example 70: preparation of 3-(imidazo[1,2-b]pyridazin-3-ylethynyl)-4-methyl-N-(4-(4-(methylamino)piperidin-1-yl)-3-(trifluoromethyl)phenyl)benzamide (GT-D-111)

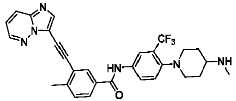

3-(imidazo[1,2-b]pyridazin-3-ylethynyl)-4-methyl-N-(4-(4-(methylamino)piperidin-1-yl)-3-(trifluoromethyl)phenyl)benzamide (GT-D-111) was prepared by referring to Scheme 34 (white solid,1.8 g). ¹H NMR (400 MHz, DMSO) δ 10.63 (s, 1H), 9.21 (s, 2H), 8.86 (dd, *J* = 4.4, 1.1 Hz, 1H), 8.43 (s, 1H), 8.21 (dd, *J=* 14.6, 1.9 Hz, 2H), 8.08 (s, 1H), 8.00 (dd, *J=* 8.0, 1.6 Hz, 1H), 7.59 - 7.53 (m, 3H), 3.00 (d, *J* = 11.7 Hz, 3H), 2.78 (s, 2H), 2.61 (s, 3H), 2.54 (t, *J* = 5.4 Hz, 3H), 2.11 (d, *J* = 10.0 Hz, 2H), 1.70 (dt, *J* = 11.3, 8.1 Hz, 2H). LCMS (ESI): calcd., 533.2; found, 533.2.

### Intermediate Example 71: preparation of 3-(imidazo[1,2-b]pyridazin-3-ylethynyl)-4-methyl-N-(4-(4-(piperidin-4-yl)piperazin-1-yl)-3-(trifluoromethyl)phenyl)benzamide (GT-D-112)

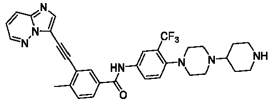

3-(imidazo[1,2-b]pyridazin-3-ylethynyl)-4-methyl-N-(4-(4-(piperidin-4-yl)piperazin-1-yl)-3-(trifluoromethyl)phenyl)benzamide (GT-D-112) was prepared by referring to Scheme 34 (brown solid, 2.1 g). ¹H NMR (400 MHz, DMSO) δ 10.60 (s, 1H), 8.74 (dd, *J =* 4.4, 1.5 Hz, 1H), 8.28 (dd, *J* = 9.2, 1.5 Hz, 1H), 8.25 (s, 1H), 8.22 (d, *J* = 2.1 Hz, 2H), 8.19 - 8.13 (m, 1H), 7.96 (dd, *J* = 8.0, 1.8 Hz, 1H), 7.58 (t, *J =* 9.0 Hz, 2H), 7.42 (dd, *J* = 9.2, 4.5 Hz, 1H), 3.49 (d, *J =* 13.0 Hz, 4H), 3.15 (s, 6H), 2.98 - 2.82 (m, 2H), 2.62 (s, 3H), 2.33 (d, *J* = 10.9 Hz, 2H), 1.80 (d, *J* = 9.7 Hz, 2H). LCMS (ESI): calcd., 588.3; found, 588.2.

### Intermediate Example 72: preparation of tert-butyl (4-(4-((3-(difluoromethyl)-1-(piperidin-4-yl)-1H-pyrazol-4-yl)carbamoyl)oxazol-2-yl)pyridin-2-yl)(2,2,2-trifluoroethyl)carbamate (GT-D-126)

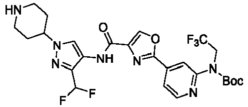

tert-butyl (4-(4-((3-(difluoromethyl)-1-(piperidin-4-yl)-1H-pyrazol-4-yl)carbamoyl)oxazol-2-yl)pyridin-2-yl)(2,2,2-trifluoroethyl)carbamate (GT-D-126) was prepared by referring to Scheme 36 (white solid, 2.2 g). ¹H NMR (400 MHz, CDCl₃) δ 9.06 (s, 1H), 8.53 (d, *J* = 5.1 Hz, 1H), 8.39 (s, 1H), 8.34 (d, *J* = 5.3 Hz, 2H), 7.72 (dd, *J* = 5.1, 1.2 Hz, 1H), 6.84 (t, *J* = 54.7 Hz, 1H), 4.88 (q, *J* = 8.7 Hz, 2H), 4.21 (s, 1H), 3.26 (d, *J=* 12.6 Hz, 2H), 2.77 (td, *J=* 12.5, 2.1 Hz, 2H), 2.16 (d, *J=* 12.2 Hz, 2H), 1.92 (dd, *J* = 12.1, 3.8 Hz, 2H), 1.58 (s, 9H). LCMS (ESI): calcd., 586.2; found, 586.3.

### Intermediate Example 73: preparation of (1r,4r)-4-(6-(5-cyano-1H-pyrrolo[2,3-b]pyridin-1-yl)-4-(isopropylamino)nicotinamido)cyclohexane-1-carboxylic acid (GT-D-132)

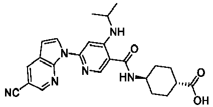

(1r,4r)-4-(6-(5-cyano-1H-pyrrolo[2,3-b]pyridin-1-yl)-4-(isopropylamino)nicotinamido)cyclohexane-1-carboxylic acid (GT-D-132) was prepared by referring to Scheme 38 (white solid, 3.1 g). ¹H NMR (400 MHz, DMSO) δ 12.11 (s, 1H), 8.81 (d, *J* = 1.9 Hz, 1H), 8.67 (d, *J=* 2.0 Hz, 1H), 8.63 - 8.55 (m, 2H), 8.52 (d, *J=* 3.9 Hz, 1H), 8.37 (d, *J=* 7.6 Hz,1H), 8.07 (s, 1H), 6.89 (d, *J* = 3.9 Hz, 1H), 3.77 (dt, *J* = 19.1, 6.3 Hz, 2H), 2.17 (dd, *J* = 11.2, 7.9 Hz, 1H), 2.04 - 1.85 (m, 4H), 1.39 (dd, *J=* 16.9, 8.0 Hz, 4H), 1.30 (s, 3H), 1.29 (s, 3H). LCMS (ESI): calcd., 447.2; found, 447.2.

### Intermediate Example 74: preparation of 6-(5-cyano-1H-pyrrolo[2,3-b]pyridin-1-yl)-4-(isopropylamino)-N-(piperidin-4-yl)nicotinamide (GT-D-133)

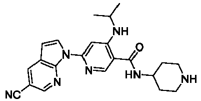

6-(5-cyano-1H-pyrrolo[2,3-b]pyridin-1-yl)-4-(isopropylamino)-N-(piperidin-4-yl)nicotinamide (GT-D-133) was prepared by referring to Scheme 37 (white solid, 2.8 g). ¹H NMR (400 MHz, MeOD) δ 8.80 (d, *J* = 1.3 Hz, 1H), 8.74 (s, 1H), 8.62 (d, *J=* 1.5 Hz, 1H), 8.39 (d, *J* = 4.0 Hz, 1H), 7.44 (s, 1H), 7.09 (d, *J* = 4.0 Hz, 1H), 4.34 - 4.04 (m, 2H), 3.52 (d, *J* = 13.1 Hz, 2H), 3.17 (dd, *J* = 12.4, 10.3 Hz, 2H), 2.23 (d, *J* = 11.3 Hz, 2H), 1.97 (td, *J* = 14.5, 4.2 Hz, 2H), 1.39 (d, *J* = 6.4 Hz, 6H). LCMS (ESI): calcd., 404.2; found, 404.3.

### Intermediate Example 75: preparation of N-(4-(chlorodifluoromethoxy)phenyl)-6-(4-(piperazin-1-yl)piperidin-1-yl)-5-(1H-pyrazol-5-yl)nicotinamide (GT-D-179)

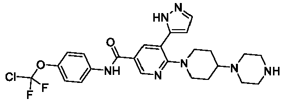

N-(4-(chlorodifluoromethoxy)phenyl)-6-(4-(piperazin-1-yl)piperidin-1-yl)-5-(1H-pyrazol-5-yl)nicotinamide (GT-D-179) was prepared by referring to Scheme 39 (brown solid, 1.03 g). ¹H NMR (400 MHz, MeOD) δ 8.79 (d, *J=* 2.3 Hz, 1H), 8.68 (d, *J=* 2.3 Hz, 1H), 8.00 (d, *J=* 2.3 Hz, 1H), 7.88 - 7.80 (m, 2H), 7.30 (d, *J* = 9.1 Hz, 2H), 6.88 (d, *J* = 2.2 Hz, 1H), 3.90 (dd, *J* = 11.4, 5.8 Hz, 3H), 3.72 (s, 8H), 3.24 (d, *J =* 12.2 Hz, 2H), 2.31 (d, *J =* 10.7 Hz, 2H), 2.09 (dd, *J =* 16.3, 7.7 Hz, 2H). LCMS (ESI): calcd., 532.2; found, 532.2.

### Intermediate Example 76: preparation of (R)-5-(3-(3-methyl-2-oxoimidazolidin-1-yl)piperidin-1-yl)-3 -((4-(piperidin-4-yl)phenyl)amino)pyrazine-2-carboxamide (GT-D-197)

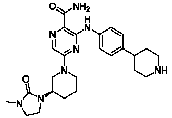

(R)-5-(3-(3-methyl-2-oxoimidazolidin-1-yl)piperidin-1-yl)-3-((4-(piperidin-4-yl)phenyl)amino)pyrazine-2-carboxamide (GT-D-197) was prepared by referring to Scheme 40 (yellow solid, 3.0 g). ¹H NMR (400 MHz, DMSO) δ 11.19 (s, 1H), 7.75 (s, 1H), 7.66 (d, *J* = 7.9 Hz, 1H), 7.48 (t, *J* = 10.8 Hz, 2H), 7.37 - 7.29 (m, 1H), 7.14 (d, *J* = 8.4 Hz, 2H), 4.32 (dd, *J =* 28.6, 12.2 Hz, 2H), 3.60 (d, *J =* 10.7 Hz, 1H), 3.26 (dd, *J =* 13.7, 7.6 Hz, 2H), 3.10 - 2.90 (m, 4H), 2.73 (d, *J =* 14.9 Hz, 3H), 2.63 - 2.54 (m, 2H), 1.76 (ddd, *J* = 33.3, 25.5, 11.2 Hz, 6H), 1.49 (ddd, *J* = 20.7, 19.1, 10.2 Hz, 4H). LCMS (ESI): calcd., 479.3; found, 479.5.

### Intermediate Example 77: preparation of 5-(piperidin-1-yl)-3-((4-(piperidin-4-yl)phenyl)amino)pyrazine-2-carboxamide (GT-D-198)

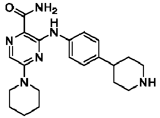

5-(piperidin-1-yl)-3-((4-(piperidin-4-yl)phenyl)amino)pyrazine-2-carboxamide (GT-D-198) was prepared by referring to Scheme 41 (yellow solid, 1.7 g). ¹H NMR (400 MHz, DMSO) δ 11.23 (s, 1H), 7.71 (s, 1H), 7.63 (s, 1H), 7.49 (d, *J* = 8.5 Hz, 2H), 7.28 (s, 1H), 7.14 (d, *J* = 8.5 Hz, 2H), 3.66 (d, *J=* 5.3 Hz, 4H), 3.15 (s, 2H), 3.03 -2.93 (m, 2H), 2.54 (d, *J* = 14.1 Hz, 2H), 1.64 (d, *J* = 11.7 Hz, 4H), 1.57 (d, *J* = 3.8 Hz, 4H), 1.47 (td, *J* = 12.3, 3.6 Hz, 2H). LCMS (ESI): calcd., 381.2; found, 381.2.

### Intermediate Example 78: preparation of 4,9-dioxo-4,9-dihydronaphtho[2,3-b]furan-2-carboxylic acid (GT-D-200)

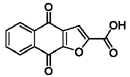

4,9-dioxo-4,9-dihydronaphtho[2,3-b]furan-2-carboxylic acid (GT-D-200) was prepared by referring to Scheme 42 (brown solid, 130 mg). ¹H NMR (400 MHz, DMSO) δ 8.14 (ddd, *J* = 7.1, 4.3, 3.3 Hz, 2H), 8.03 - 7.85 (m, 2H), 7.69 (s, 1H). LCMS (ESI): calcd., 243.0; found, 243.0.

### Intermediate Example 79: preparation of (6-((5-bromo-2-((2-methoxy-5-methyl-4-(piperazin-1-yl)phenyl)amino)pyrimidin-4-yl)amino)quinoxalin-5-yl)dimethylphosphine oxide (GT-S-50)

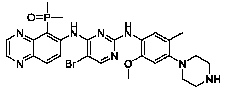

(6-((5-bromo-2-((2-methoxy-5-methyl-4-(piperazin-1-yl)phenyl)amino)pyrimidin-4-yl)amino)quinoxalin-5-yl)dimethylphosphine oxide (GT-S-50) was prepared by referring to Scheme 16 (yellow solid, 950 mg). ¹H NMR (400 MHz, DMSO-d6): δ ppm 12.76 (s, 1H), 9.23 (br, 2H), 8.91(d, 3H), 8.38 - 8.32 (m, 2H), 8.00 (d, 1H), 7.49(s, 1H), 6.77 (s, 1H), 3.83 (s, 3H), 3.32 - 3.24 (m, 4H), 3.12 - 2.97 (m, 4H), 2.13 (s, 3H), 2.10 (s, 3H), 2.03 (s, 3H). LCMS (ESI): calcd., 597.2; found, 597.1.

### Intermediate Example 80: preparation of (6-((5-bromo-2-((2-methoxy-5-methyl-4-(4-(methylamino)piperidin-1-yl)phenyl)amino)pyrimidin-4-yl)amino)quinoxalin-5-yl)dimethylphosphine oxide (GT-S-51)

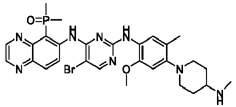

(6-((5-bromo-2-((2-methoxy-5-methyl-4-(4-(methylamino)piperidin-1-yl)phenyl)amino)pyrimidin-4-yl)amino)quinoxalin-5-yl)dimethylphosphine oxide (GT-S-51) was prepared by referring to Scheme 16 (yellow solid, 1.1 g). ¹H NMR (400 MHz, CD₃OD): δ ppm 8.94 - 8.90 (br, 3H), 8.30 (s, 1H), 8.10 - 8.07 (br, 1H) , 7.35 (s, 1H), 6.89 (s, 1H), 3.91 (s, 3H), 3.39 - 3.24 (m, 3H), 2.91 (t, *J* = 15.6 Hz, 2H), 2.82 (s, 3H), 2.30 - 2.19 (m, 11H), 1.98 - 1.84 (m, 11H), 1.98 - 1.84 (m, 2H). LCMS (ESI): calcd., 625.2; found, 625.1.

### Intermediate Example 81: preparation of (6-((5-bromo-2-((2-methoxy-5-methyl-4-(4-(piperazin-1-yl)piperidin-1-yl)phenyl)amino)pyrimidin-4-yl)amino)quinoxalin-5-yl)dimethylphosphine oxide (GT-S-52)

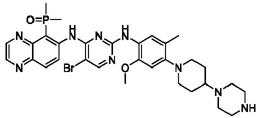

(6-((5-bromo-2-((2-methoxy-5-methyl-4-(4-(piperazin-1-yl)piperidin-1-yl)phenyl)amino)pyrimidin-4-yl)amino)quinoxalin-5-yl)dimethylphosphine oxide (GT-S-52) was prepared by referring to Scheme 16 (yellow solid, 150 mg, yield 108.88%). ¹H NMR (400 MHz, DMSO-d₆) δ ppm 9.10 - 8.80 (m, 2H), 8.41 (s, 1H), 8.05 - 7.95 (m, 1H), 7.60 - 7.50 (m, 2H), 7.48 - 7.36 (m, 3H), 7.20 - 7.10 (m, 2H), 6.85 - 6.80 (m, 1H), 3.80 - 3.62 (m, 6H), 3.60 - 3.40 (m, 6H), 3.35 - 3.30 (m, 2H), 2.85 - 2.60 (m, 2H), 2.38 - 2.25 (m, 2H), 2.20 - 1.90 (m, 11H). LCMS (ESI): calcd., 680.2; found, 680.1.

### Intermediate Example 82: preparation of 1-isopropyl-N-((6-methyl-2-oxo-4-propyl-1,2-dihydropyridin-3-yl)methyl)-6-(6-(piperazin-1-yl)pyridin-3-yl)-1H-indazole-4-carboxamide (GT-S-7)

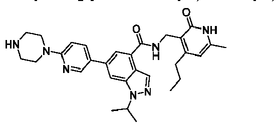

1-isopropyl-N-((6-methyl-2-oxo-4-propyl-1,2-dihydropyridin-3-yl)methyl)-6-(6-(piperazin-1-yl)pyridin-3-yl)-1H-indazole-4-carboxamide (GT-S-7) was prepared by referring to Scheme 45 (yellow solid, 300 mg, yield 21%). ¹H NMR (400 MHz, DMSO-d₆) δ ppm 8.67 - 8.61 (m, 2H), 8.38 (s, 1H), 8.08 - 8.05 (br, 2H), 7.85 (s, 1H), 6.96 (d, *J* = 11.6 Hz, 1H), 5.94 (s, 1H), 5.20 - 5.11 (m, 1H), 4.44 (d, *J* = 6.4 Hz, 2H), 3.52 - 3.49 (m, 4H), 2.86 - 2.78 (m, 4H), 2.57 - 2.53 (m, 2H), 2.11 (s, 3H), 1.58 - 1.51 (m, 8H), 0.90 (t, *J* = 9.6 Hz, 3 H). LCMS (ESI): calcd., 528.3; found, 528.5.

### Intermediate Example 83: preparation of 1-isopropyl-N-((6-methyl-2-oxo-4-propyl-1,2-dihydropyridin-3-yl)methyl)-6-(6-(4-(methylamino)piperidin-1-yl)pyridin-3-yl)-1H-indazole-4-carboxamide (GT-S-8)

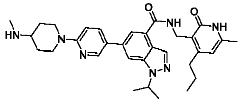

1-isopropyl-N-((6-methyl-2-oxo-4-propyl-1,2-dihydropyridin-3-yl)methyl)-6-(6-(4-(methylamino)piperidin-1-yl)pyridin-3-yl)-1H-indazole-4-carboxamide (GT-S-8) was prepared by referring to Scheme 43 (light yellow solid, 1.8 g). ¹H NMR (400 MHz, DMSO-d₆): δ ppm 8.69 - 8.57 (m, 4H), 8.63 (s, 1H), 8.15 - 8.08 (m, 4H), 7.82 (br, 1H), 7.11 (d, *J* = 8.8 Hz, 1H), 5.91 (s, 1H), 5.17 - 5.10 (m, 1H), 4.48 - 4.41 (m, 4H), 3.30 - 3.28 (br, 1H), 2.96 (t, *J* = 12.6 Hz, 2H), 2.59 (t, *J* = 5.2 Hz, 3H), 2.54 - 2.49 (m, 4H), 2.14 (s, 3H), 2.09 - 2.07 (m, 2H), 1.57 - 1.49 (m, 8H), 0.88 (t, *J* = 7.4 Hz, 3H). LCMS (ESI): calcd., 556.3; found, 556.5.

### Intermediate Example 84: preparation of 1-isopropyl-N-((6-methyl-2-oxo-4-propyl-1,2-dihydropyridin-3-yl)methyl)-6-(6-(4-(piperidin-4-yl)piperazin-1-yl)pyridin-3-yl)-1H-indazole-4-carboxamide (GT-S-9)

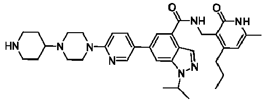

1-isopropyl-N-((6-methyl-2-oxo-4-propyl-1,2-dihydropyridin-3-yl)methyl)-6-(6-(4-(piperidin-4-yl)piperazin-1-yl)pyridin-3-yl)-1H-indazole-4-carboxamide (GT-S-9) was prepared by referring to Scheme 44 (light yellow solid, 2.0 g). ¹H NMR (400 MHz, DMSO-d₆): δ ppm 11.99 (br, 1H), 9.40 - 9.37 (br, 1H), 8.78 (br, 1H), 8.67 (s, 1H), 8.45 - 8.40 (m, 2H), 8.24 (s, 1H), 7.88 (s, 1H), 7.40 (d, *J* = 12 Hz, 1H), 5.99 (s, 1H), 5.24 - 5.15 (m, 1H), 4.66 - 4.62 (m, 2H), 4.45 - 4.44 (br, 2H), 3.77 - 3.18 (m, 7H), 3.25 - 3.18 (m, 2H), 2.97 - 2.90 (m, 2H), 2.58 - 2.52 (m, 4H), 2.37 - 2.33 (br, 2H), 2.17 (s, 3H), 2.10 - 2.02 (m, 2H), 1.57 - 1.51 (m, 8H), 0.89 (t, *J* = 9.6 Hz, 3H). LCMS (ESI): calcd., 611.4; found, 611.7.

### Intermediate Example 85: preparation of N-((4,6-dimethyl-2-oxo-1,2-dihydropyridin-3-yl)methyl)-5-(ethyl(tetrahydro-2H-pyran-4-yl)amino)-4-methyl-4'-(piperazin-1-ylmethyl)-[1,1'-biphenyl]-3-carboxamide (GT-S-10)

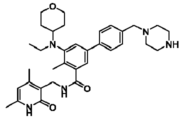

N-((4,6-dimethyl-2-oxo-1,2-dihydropyridin-3-yl)methyl)-5-(ethyl(tetrahydro-2H-pyran-4-yl)amino)-4-methyl-4'-(piperazin-1-ylmethyl)-[1,1'-biphenyl]-3-carboxamide (GT-S-10) was prepared by referring to Scheme 47 (yellow solid, 1.4 g). ¹H NMR (400 MHz, DMSO-d₆): δ ppm 11.99 (br, 1H), 9.40 - 9.37 (br, 1H), 8.78 (br, 1H), 8.67 (s, 1H), 8.45 - 8.40 (m, 2H), 8.24 (s, 1H), 7.88 (s, 1H), 7.40 (d, *J* = 12 Hz, 1H), 5.99 (s, 1H), 5.24 - 5.15 (m, 1H), 4.66 - 4.62 (m, 2H), 4.45 - 4.44 (br, 2H), 3.77 - 3.18 (m, 7H), 3.25 - 3.18 (m, 2H), 2.97 - 2.90 (m, 2H), 2.58 - 2.52 (m, 4H), 2.37 - 2.33 (br, 2H), 2.17 (s, 3H), 2.10 - 2.02 (m, 2H), 1.57 - 1.51 (m, 8H), 0.89 (t, *J* = 9.6 Hz, 3H). LCMS (ESI): calcd., 611.4; found, 611.7.

### Intermediate Example 86: preparation of N-((4,6-dimethyl-2-oxo-1,2-dihydropyridin-3-yl)methyl)-5-(ethyl(tetrahydro-2H-pyran-4-yl)amino)-4-methyl-4'-(piperazin-1-yl)-[1,1'-biphenyl]-3-carboxamide (GT-S-11)

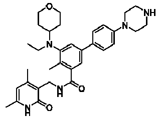

N-((4,6-dimethyl-2-oxo-1,2-dihydropyridin-3-yl)methyl)-5-(ethyl(tetrahydro-2H-pyran-4-yl)amino)-4-methyl-4'-(piperazin-1-yl)-[1,1'-biphenyl]-3-carboxamide (GT-S-11) was prepared by referring to Scheme 48 (white solid, 2.6 g). ¹H NMR (400 MHz, DMSO-*d*₆) δ 8.21 (t, J = 6.0 Hz, 1H), 7.54 (d, J = 11.6 Hz, 2H), 7.3 (s, 1H), 7.20 (s, 1H), 7.07 (d, J = 11.2 Hz, 2H), 4.31 (d, J =6 Hz, 2H), 3.86 (d, J = 13.6 Hz, 2H), 3.35 - 3.12 (m, 15 H), 2.24 (d, J = 6.4 Hz, 6H), 2.14 (s, 2H), 1.71 - 1.48 (m, 4H), 0.86 (t, J = 9.2 Hz, 3H). LCMS (ESI): calcd., 558.3; found, 558.5.

### Intermediate Example 87: preparation of N-((4,6-dimethyl-2-oxo-1,2-dihydropyridin-3-yl)methyl)-5-(ethyl(tetrahydro-2H-pyran-4-yl)amino)-4-methyl-4'-(4-(methylamino)piperidin-1-yl)-[1,1'-biphenyl]-3-carboxamide (GT-S-12)

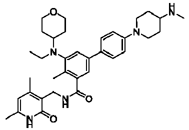

N-((4,6-dimethyl-2-oxo-1,2-dihydropyridin-3-yl)methyl)-5-(ethyl(tetrahydro-2H-pyran-4-yl)amino)-4-methyl-4'-(4-(methylamino)piperidin-1-yl)-[1,1'-biphenyl]-3-carboxamide (GT-S-12) was prepared by referring to Scheme 49 (white solid, 1.8 g). ¹H NMR (400 MHz, DMSO-*d*₆) δ 8.16 (t, J = 4.8 Hz, 1H), 7.45 (d, J = 8.8 Hz, 2H), 7.33 (s, 1H), 7.16 (s, 1H), 6.98 (d, J = 8.8 Hz, 2H), 5.86 (s, 1H), 4.29 (d, J = 4.8 Hz, 2H), 3.82 (d, J = 10.4 Hz, 2H), 3.65 (d, J = 12.4 Hz, 2H), 3.25 (t, J = 11.2 Hz, 3H),3.10-3.01 (m, 4H), 2.76 (t, J = 11.6 Hz, 2H), 2.45-2.38 (m, 1H), 2.29 (s, 3H),2.21( d, J = 6.8 Hz, 6H), 2.11 (s, 3H), 1.89 - 1.85 (m, 2H), 1.66 - 1.64 (m, 2H), 1.55 - 1.49 (m, 2H), 1.32 - 1.28 (m, 2H), 0.83 (t, J = 6.8 Hz, 3H). LCMS (ESI): calcd., 586.4; found, 586.2.

### Intermediate Example 88: preparation of N-((4,6-dimethyl-2-oxo-1,2-dihydropyridin-3-yl)methyl)-5-(ethyl(tetrahydro-2H-pyran-4-yl)amino)-4-methyl-4'-(4-(piperidin-4-yl)piperazin-1-yl)-[1,1'-biphenyl]-3-carboxamide GT-S-13

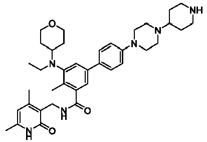

N-((4,6-dimethyl-2-oxo-1,2-dihydropyridin-3-yl)methyl)-5-(ethyl(tetrahydro-2H-pyran-4-yl)amino)-4-methyl-4'-(4-(piperidin-4-yl)piperazin-1-yl)-[1,1'-biphenyl]-3-carboxamide (GT-S-13) was prepared by referring to Scheme 50 (white solid, 1.6 g). ¹H NMR (400 MHz, DMSO-*d*₆) δ 8.18 (t, J = 2.0 Hz, 1H), 7.47 (d, J = 8.4 Hz, 2H), 7.33 (s, 1H), 7.16 (s, 1H), 6.98 (d, J = 8.0 Hz, 2H), 5.86 (s, 1H), 4.29 (d, J = 4.0 Hz, 2H), 4.14 - 4.06 (m, 1H), 3.82 (d, J = 9.2 Hz, 2H), 3.41 - 3.36 (m, 2H), 3.29 (d, J = 12.0 Hz, 3H), 3.14 - 2.98 (m, 9H), 3.62 (s, 4H), 2.28 - 2.21 (m, 7H), 2.11 ( s, 3H), 1.68 - 1.64 (m, 4H), 1.53 - 1.50 (m, 2H), 1.26 - 1.24 (m, 2H), 0.83 (t, J = 6.8 Hz, 3H). LCMS (ESI): calcd., 641.4; found, 642.0.

### Intermediate Example 89: preparation of N-(5-(3,5-difluorobenzyl)-1H-indazol-3-yl)-4-(piperazin-1-yl)-2-((tetrahydro-2H-pyran-4-yl)amino)benzamide (GT-S-14)

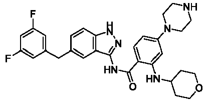

N-(5-(3,5-difluorobenzyl)-1H-indazol-3-yl)-4-(piperazin-1-yl)-2-((tetrahydro-2H-pyran-4-yl)amino)benzamide (GT-S-14) was prepared by referring to Scheme 51 (yellow solid, 341 mg). ¹H NMR (400 MHz, DMSO-*d*₆) δ 10.22 (s, 1H), 9.13 (s, 2H), 7.88 (d, J = 12 Hz, 1H),7.48 (d, J = 19.2 Hz,, 1H), 7.43 (s, 1H), 7.28 (d, J = 11.6 Hz, 1H), 7.03 (t, J = 16 Hz, 3H), 6.30 (t, J = 11.6 Hz, 2H), 4.32 - 4.07 (m, 2H), 3.86 (t, J = 5.2 Hz, 2H), 3.73 (s, 1H), 3.50 (d, J = 14.4 Hz, 6H), 3.26 (s, 4H), 1.97 (d, J =15.6 Hz, 2H), 1.42 - 1.36 (m, 2H). LCMS (ESI): calcd., 547.3; found, 547.0.

### Intermediate Example 90: preparation of N-(5-(3,5-difluorobenzyl)-1H-indazol-3-yl)-4-(4-(methylamino)piperidin-1-yl)-2-((tetrahydro-2H-pyran-4-yl)amino)benzamide (GT-S-15)

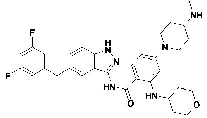

N-(5-(3,5-difluorobenzyl)-1H-indazol-3-yl)-4-(4-(methylamino)piperidin-1-yl)-2-((tetrahydro-2H-pyran-4-yl)amino)benzamide (GT-S-15) was prepared by referring to Scheme 52 (yellow solid, 601 mg). ¹H NMR (400 MHz, DMSO-*d*₆) δ 9.40 (s, 3H), 7.49 (t, J = 11.6 Hz, 2H), 7.11 (d, J = 10.4 Hz, 1H), 6.81 (d, J = 8.4 Hz,, 1H), 6.45 (d, J = 9.2 Hz, 2H), 6.34 (t, J = 11.6 Hz, 1H), 5.94 (d, J = 11.6 Hz, 1H), 5.83 (s, 1H), 3.75 (s, 2H), 3.60 (d, J = 18.4 Hz, 4H), 3.26 (d, J =13.2 Hz, 3H), 2.86 (s, 1H), 2.57 (t, J = 16.4 Hz, 2H), 2.38 (s, 3H), 1.91 (d, J = 13.6 Hz, 1H), 1.67 (t, J = 12.4 Hz, 2H), 1.56 (t, J = 13.6 Hz, 2H), 1.27 (s, 2H). LCMS (ESI): calcd., 575.3; found, 575.0.

### Intermediate Example 91: preparation of (R)-6-(2-(3-fluorophenyl)pyrrolidin-1-yl)-3-(6-(piperazin-1-yl)pyridin-2-yl)imidazo[1,2-b]pyridazine (GT-S-17)

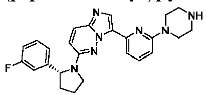

(R)-6-(2-(3-fluorophenyl)pyrrolidin-1-yl)-3-(6-(piperazin-1-yl)pyridin-2-yl)imidazo[1,2-b]pyridazine (GT-S-17) was prepared by referring to Scheme 53 (yellow solid, 800 mg). ¹H NMR (400 MHz, DMSO-d6) δ 8.17 (s, 1H), 7.89 (d, *J* = 6.4 Hz, 1H), 5.57 (s, 2H), 7.54 (d, J = 10.2 Hz, 1H), 7.42 - 7.36 (m, 2H), 7.05 (t, *J* = 8.0 Hz, 1H), 6.82 - 6.74 (m, 2H), 5.15(d, J = 4.0 Hz, 1H), 3.98, 3.95 (dd, J = 6.4, 6.0 Hz, 1H), 3.71 - 3.60 (m, 6H), 2.99 (s, 4H), 2.53 - 2.44 (m, 1H), 2.03 (d, J = 6.8 Hz, 2H), 1.89 - 1.85 (m, 1H), 1.23 (s, 1H). LCMS (ESI): calcd., 444.2; found, 444.3.

### Intermediate Example 92: preparation of (R)-1-(6-(6-(2-(3-fluorophenyl)pyrrolidin-1-yl)imidazo[1,2-b]pyridazin-3-yl)pyridin-2-yl)-N-methylpiperidin-4-amine (GT-S-18)

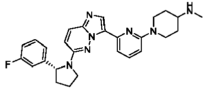

(R)-1-(6-(6-(2-(3-fluorophenyl)pyrrolidin-1-yl)imidazo[1,2-b]pyridazin-3-yl)pyridin-2-yl)-N-methylpiperidin-4-amine (GT-S-18) was prepared by referring to Scheme 54 (yellow solid, 790 mg). ¹H NMR (400 MHz, CD₃OD) δ 8.61 (s, 1H), 8.20 (s, 1H), 7.40 (t, J = 7.6 Hz, 2H), 7.18 - 7.02 (m, 6H), 5.25 (s, 1H), 4.58 (s, 2H), 4.15 (t, J = 9.2 Hz, 1H), 3.68(s, 1H), 3.39 (s, 1H), 3.35 (s, 1H), 2.86 (s, 3H), 2.36 (s, 1H), 2.33 (s, 2H), 2.21 (s, 3H), 2.06 (d, J = 3.6 Hz, 3H), 1.85 - 1.82 (m, 2H). LCMS (ESI): calcd., 472.3; found, 472.3.

### Intermediate Example 93: preparation of the following intermediate compounds

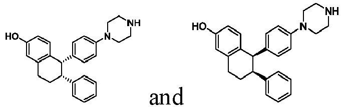

Following the method of Scheme 57, the enantiomeric mixture product obtained according to Step 8 of Scheme 57 was separated by supercritical fluid chromatography (SFC) to obtain the two intermediate compounds as described above (their retention times were 8.232 min and 9.400 min, respectively, in terms of analytical methods). The analytical method, preparation method, and conditions for SFC are shown below.

The analysis method and conditions for SFC are as follows:

| | |
|---|---|
| System: | Shimadzu LC-20AP |
| Column name: | Daicel CHIRALPAK^{®}IE |
| Column size: | 250*4.6mm 5µm |
| Mobile Phase A: | n-Hexane |
| Mobile Phase B: | EtOH(0.2%DEA) |
| Mobile Phase A: Mobile Phase B | 70: 30 |
| Wavelength: | 254 nm |
| Flow | 1mL/min |
| Column temp: | 25°C |
| Injection: | 5µL |
| Solvent: | EtOH: HPLC grade |
| | n-Hexane: HPLC grade |

Preparation method for SFC is as follows:

| | |
|---|---|
| System: | Shimadzu |
| Column name: | DAICELCHIRALPAK^{®}IE |
| Column size: | 250*25 mm 10 µm |
| Mobile Phase A: | n-Hexane |
| Mobile Phase B: | ETOH (+0.1% 7.0mol/l Ammonia in MeOH) |
| A:B: | 70: 30 |
| Wavelength: | 214 nm |
| Flow: | 30ml/min |
| Column temp: | rt |
| Injection: | 1mL |
| Cycle time: | 12min |
| Solvent: | n-Hexane: redistilled grade |
| Preparation of sample solution: | ETOH: redistilled grade |

One of the obtained intermediates (GT-M-160_P1) (white solid, 565 mg): ¹H NMR (400 MHz, DMSO) δ 9.11 (s, 1H), 7.23 - 7.06 (m, 3H), 6.84 (d, *J* = 6.7 Hz, 2H), 6.62 (dd, *J* = 15.5, 5.3 Hz, 2H), 6.55 - 6.43 (m, 3H), 6.21 (d, *J* = 8.6 Hz, 2H), 4.13 (d, *J* = 4.9 Hz, 1H), 3.28 (s, 2H), 3.04 - 2.91 (m, 2H), 2.91 - 2.83 (m, 4H), 2.80 - 2.70 (m, 4H), 2.11 (dd, *J* = 12.3, 6.4 Hz, 1H), 1.71 (d, *J* = 7.5 Hz, 1H). LCMS (ESI): calcd., 385.23; found, 385.30. (retention time was 8.232 min in terms of analytical method).

Another intermediate (GT-M-160_P2) (white solid, 595 mg): ¹H NMR (400 MHz, DMSO) δ 9.10 (s, 1H), 7.14 (dd, *J* = 15.2, 7.8 Hz, 3H), 6.84 (d, *J* = 6.9 Hz, 2H), 6.66 - 6.59 (m, 2H), 6.50 (dd, *J* = 14.1, 8.3 Hz, 3H), 6.22 (d, *J* = 8.4 Hz, 2H), 4.14 (d, *J* = 4.6 Hz, 1H), 3.30 - 3.23 (m, 2H), 2.97 (dd, *J* = 15.6, 9.6 Hz, 2H), 2.90 (d, *J* = 5.0 Hz, 4H), 2.80 (d, *J* = 4.5 Hz, 4H), 2.11 (dd, *J* = 12.2, 6.8 Hz, 1H), 1.72 (s, 1H). LCMS (ESI): calcd., 385.23 found, 385.30. (retention time was 9.400 min in terms of analytical method).

### Intermediate Example 94: preparation of the following intermediate compounds

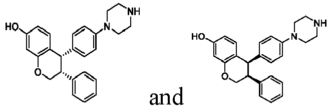

Following the method of Scheme 58, the enantiomeric mixture product obtained according to Step 6 of Scheme 58 was separated by supercritical fluid chromatography (SFC) to obtain the two intermediate compounds as described above (their retention times were 1.763 min and 3.506 min, respectively, in terms of analytical methods). The analytical method, preparation method, and conditions for SFC are shown below.

The analysis method and conditions for SFC are as follows:

| | | | |
|---|---|---|---|
| System: | Waters Ultra Performance Convergence Chromatography (UPCC) (CA-185) | | |
| Column name: | DAICELCHIRALPAK^{®}OD | | |
| Column size: | 100*3.0 mm *3.0 µm | | |
| Mobile Phase A: | Supercritical CO₂ | | |
| Mobile Phase B: | IPA (0.1%DEA) | | |
| Wavelength: | 214nm | | |
| Flow | 1.5 mL/min | | |
| Column temp: | 35°C | | |
| Back Pressure(psi): | 1800 psi | | |
| Injection: | 0.3 µL | | |
| Run time: | 8 min | | |

| Gradient | Time | (min) A(%V/V) | B(%V/V) |
|---|---|---|---|
| | 0.00 | 60 | 40 |
| | 8.00 | 60 | 40 |
| Solvent: | IPA: HPLC grade | | |
| | Supercritical CO₂ : Food grade | | |
| Preparation of mobile phases: | Mobile Phase B: Add 1 mL DEA in 1000 mL IPA , then ultrasonic degassing for 15min. | | |

Preparation method for SFC is as follows:

| | |
|---|---|
| System: | Waters SFC 150 |
| Column name: | DAICELCHIRALCEL^{®}OD |
| Column size: | 250*25 mm 10 µm |
| Mobile Phase A: | Supercritical CO₂, |
| Mobile Phase B: | IPA (+0.1% 7.0mol/l Ammonia in MEOH) |
| A:B: | 45: 55 |
| Wavelength: | 214 nm |
| Flow: | 80ml/min |
| Column temp: | RT |
| Back Pressure: | 100 bar |
| Injection: | 1.5mL |
| Cycle time: | 6min |
| Solvent: | IPA: redistilled grade |
| | Supercritical CO₂: Food grade |

One of the obtained intermediates (GT-M-173_P1) (white solid, 555 mg): ¹H NMR (400 MHz, DMSO) δ 9.33 (s, 1H), 7.22 (dd, *J* = 20.1, 17.2 Hz, 3H), 6.88 (d, *J* = 45.0 Hz, 2H), 6.71 (d, *J* = 8.3 Hz, 1H), 6.64 (d, *J* = 8.6 Hz, 2H), 6.44 (d, *J* = 8.5 Hz, 2H), 6.39 - 6.22 (m, 2H), 4.39 (t, *J* = 11.1 Hz, 1H), 4.30 - 4.06 (m, 2H), 3.55 (dt, *J* = 39.1, 19.2 Hz, 1H), 2.95 (d, *J* = 4.8 Hz, 4H), 2.83 (d, *J* = 4.5 Hz, 4H). LCMS (ESI): calcd., 387.21 found, 387.40. (retention time was 1.763 min in terms of analytical method).

Another intermediate (GT-M-173_P1) (white solid, 495 mg): ¹H NMR (400 MHz, DMSO) δ 9.33 (s, 1H), 7.22 (dd, *J* = 20.1, 17.2 Hz, 3H), 6.88 (d, *J* = 45.0 Hz, 2H), 6.71 (d, *J* = 8.3 Hz, 1H), 6.64 (d, *J* = 8.6 Hz, 2H), 6.44 (d, *J* = 8.5 Hz, 2H), 6.39 - 6.22 (m, 2H), 4.39 (t, *J* = 11.1 Hz, 1H), 4.30 - 4.06 (m, 2H), 3.55 (dt, *J* = 39.1, 19.2 Hz, 1H), 2.95 (d, *J* = 4.8 Hz, 4H), 2.83 (d, *J* = 4.5 Hz, 4H). LCMS (ESI): calcd., 387.21 found, 387.40. (retention time was 3.506 min in terms of analytical method).

### Intermediate Example 95: preparation of N-(3-(difluoromethyl)-1-(1-methylpiperidin-4-yl)-1H-pyrazol-4-yl)-5-morpholinopyrazolo[1,5-a]pyrimidine-3-carboxamide (GT-04349)

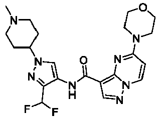

The target compound (GT-04349) was prepared by referring to Scheme 55 (white solid, 15 mg, yield 67.08%). ¹H NMR (400 MHz, MeOD) δ 8.45 (d, *J* = 8.0 Hz, 1H), 8.33 (s, 1H), 8.22 (s, 1H), 6.97 - 6.59 (m, 2H), 4.50 (s, 2H), 3.69 (dd, *J* = 35.3, 32.6 Hz, 10H), 3.16 (s, 1H), 2.85 (s, 3H), 2.29 (s, 4H). LCMS (ESI) calcd for C₂₁H₂₇F₂N₈O₂⁺ [M+H]⁺: 461.22, found, 461.2.

### Intermediate Example 96: preparation of tert-butyl (1R,5S)-3-(7-(8-ethynyl-7-fluoro-3-(methoxymethoxy)naphthalen-1-yl)-8-fluoro-2-(piperidin-4-ylmethoxy)pyrido[4,3-d]pyrimidin-4-yl)-3,8-diazabicyclo[3.2.1]octane-8-carboxylate (GT-D-204)

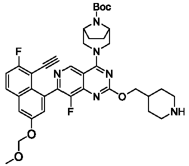

The target compound (GT-D-204) was prepared by referring to Scheme 60 (yellow solid, 0.92 g, yield 86.79%). ¹H NMR (400 MHz, CDCl3) δ 8.99 (s, 1H), 7.84 (dd, J = 9.2, 5.7 Hz, 1H), 7.55 (d, J = 2.5 Hz, 1H), 7.39 (d, J = 2.4 Hz, 1H), 7.29 (dd, J = 14.0, 5.2 Hz, 1H), 5.32 (q, J = 6.9 Hz, 2H), 4.55 (dd, J = 29.6, 12.4 Hz, 2H), 4.42 (s, 2H), 4.33 (d, J = 6.2 Hz, 2H), 3.70 (s, 2H), 3.53 (s, 3H), 3.22 (d, J = 12.2 Hz, 2H), 2.80 (s, 1H), 2.71 (t, J = 11.2 Hz, 2H), 2.04 - 1.91 (m, 9H), 1.53 (s, 9H). LCMS (ESI): calcd., 701.33, found, 701.4.

### Intermediate Example 97: preparation of (R)-5-(2-(2,5-difluorophenyl)pyrrolidin-1-yl)-3-(6-(piperazin-1-yl)pyridin-2-yl)pyrazolo[1,5-a]pyrimidine (GT-D-191)

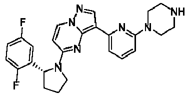

The target compound GT-D-191 was prepared by referring to Scheme 61 (red solid, 262 mg, yield 81.75%). ¹H NMR (400 MHz, DMSO) δ 9.48 (s, 2H), 8.66 (d, J = 8.0 Hz, 1H), 8.49 (s, 1H), 7.79 - 6.24 (m, 7H), 5.44 (d, J = 4.0 Hz, 1H), 4.20 - 3.95 (m, 2H), 3.84-3.77 (m, 5H), 3.20 (s, 4H), 2.10 (s, 2H), 1.93 (d, J = 8.0 Hz, 1H). LCMS (ESI): calcd., 462.22, found, 462.2.

### Intermediate Example 98: preparation of 2-(6,7-dihydro-5H-pyrrolo[1,2-c]imidazol-1-yl)-2-(4-fluoro-1-oxo-6-(4-(4-(piperazin-1-yl)piperidin-1-yl)phenyl)isoindolin-2-yl)-N-(thiazol-2-yl)acetamide (GT-D-219)

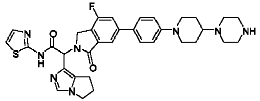

The target compound GT-D-219 was prepared by referring to Scheme 62 (yellow solid, 1.1 g, yield 113.53%). ¹H NMR (400 MHz, MeOD) δ 8.92 (s, 1H), 8.03 - 7.82 (m, 5H), 7.77 (dd, J = 10.0, 1.2 Hz, 1H), 7.58 (d, J = 4.0 Hz, 1H), 7.34 (d, J = 4.0 Hz, 1H), 4.95 (d, J = 17.2 Hz, 2H), 4.65 (d, J = 17.2 Hz, 1H), 4.44 - 4.29 (m, 2H), 4.00 (d, J = 12.0 Hz, 3H), 3.92 - 3.65 (m, 10H), 3.10 - 2.97 (m, 1H), 2.94 - 2.82 (m, 1H), 2.78 - 2.48 (m, 6H). LCMS (ESI): calcd., 641.28, found, 641.4.

### Intermediate Example 99: preparation of 2-(6,7-dihydro-5H-pyrrolo[1,2-c]imidazol-1-yl)-2-(4-fluoro-1-oxo-6-(4-(4-(piperidin-4-yl)piperazin-1-yl)phenyl)isoindolin-2-yl)-N-(thiazol-2-yl)acetamide (GT-D-217)

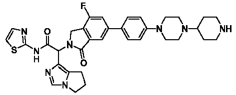

The target compound GT-D-217 was prepared by referring to Scheme 63 (yellow solid, 800 mg, yield 99.45%). ¹H NMR (400 MHz, MeOD) δ 8.90 (s, 1H), 7.88 (s, 1H), 7.66 (d, J = 8.6 Hz, 3H), 7.57 - 7.51 (m, 1H), 7.32 - 7.26 (m, 1H), 7.18-6.89 (m, 3H), 4.91 (d, J = 16.0 Hz, 1H), 4.82-4.75 (m, 1H), 4.58 (d, J = 17.0 Hz, 1H), 4.38-4.33 (m, 2H), 4.01 (s, 2H), 3.78 (s, 2H), 3.64 (d, J = 12.0 Hz, 2H), 3.38 (s, 2H), 3.26 (s, 2H), 3.18-3.12 (m, 2H), 3.07-2.99 (m, 1H), 2.91 - 2.84 (m, 1H), 2.76 - 2.63 (m, 2H), 2.52 (d, J = 12.0 Hz, 2H), 2.18 - 2.05 (m, 2H). LCMS (ESI): calcd., 641.28, found, 641.4.

### Intermediate Example 99: preparation of 2-(6-(4-((1S,4S)-2,5-diazabicyclo[2.2.1]heptan-2-yl)phenyl)-4-fluoro-1-oxoisoindolin-2-yl)-2-(6,7-dihydro-5H-pyrrolo[1,2-c]imidazol-1-yl)-N-(thiazol-2-yl)acetamide (GT-D-222)

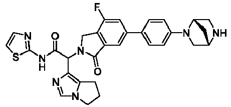

Following steps 2-5 in Scheme 62, the starting material (CAS NO.: 2407965-04-0) and the compound (CAS NO.: 942189-80-2) were used to prepare the target compound GT-D-222 (off-white solid, 0.56 g, yield 109.7%). ¹H NMR (400 MHz, MeOD) δ 8.90 (s, 1H), 7.85 (d,J = 1.2 Hz, 1H), 7.69 - 7.59 (m, 3H), 7.54 (d, J =4.0 Hz, 1H), 7.29 (d, J = 4.0 Hz, 1H), 6.81 (d,J = 8.0 Hz, 2H), 4.91 (d, J = 10.0 Hz, 1H), 4.87 - 4.81 (m,1H), 4.74 (s, 1H), 4.62 - 4.50 (m, 2H), 4.35 (dd, J = 12.0, 7.2 Hz, 2H), 3.79 (dd, J = 10.0, 2.4 Hz, 1H),3.40 (s, 2H), 3.06-2.81 (m, 2H), 2.77 - 2.61 (m, 2H), 2.33 (d, J = 10.0 Hz, 1H), 2.09 (d,J = 12.0 Hz, 1H), 2.00 (d, J = 8.8 Hz, 1H), 1.60 (s, 1H). LCMS (ESI): calcd., 570.21, found, 570.2.

### Intermediate Example 100: preparation of 2-(6,7-dihydro-5H-pyrrolo[1,2-c]imidazol-1-yl)-2-(4-fluoro-6-(4-(4-(methylamino)piperidin-1 -yl)phenyl)-1 -oxoisoindolin-2-yl)-N-(thiazol-2-yl)acetamide (GT-D-221)

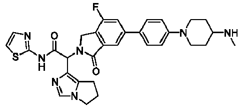

Following steps 2-5 in Scheme 62, the starting material (CAS NO.: 2407965-04-0) and the compound (CAS NO.: 2235416-80-3) were used to prepare the target compound GT-D-221 (yellow solid, 0.72 g, yield 105.4%). ¹H NMR (400 MHz, MeOD) δ 8.91 (s, 1H), 7.96 (d, *J* = 1.2 Hz, 1H), 7.92 (d, *J* = 3.2 Hz, 3H), 7.77 (dd, *J* = 10.0, 1.2 Hz, 1H), 7.55 (d, *J* = 3.6 Hz, 1H), 7.30 (d, *J* = 3.6 Hz, 1H), 6.51 (s, 1H), 4.96 (d, *J* = 17.2 Hz, 1H), 4.88 (s, 1H), 4.83 (s, 1H), 4.63 (d, *J* = 17.2 Hz, 1H), 4.34 (dt, *J* = 12.0, 7.2 Hz, 2H), 3.93 (d, *J* = 12.0 Hz, 2H), 3.85 (t, *J* = 11.2 Hz, 2H), 3.63 (dd, *J* = 20.0, 9.6 Hz, 1H), 3.08 - 2.98 (m, 1H), 2.91 - 2.83 (m, 1H), 2.81 (s, 3H), 2.74 - 2.63 (m, 2H), 2.51 (d, *J* = 12.0 Hz, 2H), 2.44 - 2.31 (m, 2H). LCMS (ESI): calcd., 586.24, found, 586.3.

### Intermediate Example 101: preparation of 2-(6-(4-(3,8-diazabicyclo[3.2.1]octan-3-yl)phenyl)-4-fluoro-1-oxoisoindolin-2-yl)-2-(6,7-dihydro-5H-pyrrolo[1,2-c]imidazol-1-yl)-N-(thiazol-2-yl)acetamide (GT-D-224)

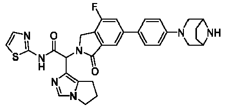

Following Scheme 62, the starting compound (CAS NO.: 1146427-86-2) was used to replace the compound (CAS NO.: 2762613-62-5) to prepare the target compound GT-D-224 (off-white solid, 619 mg, yield 105.0%). ¹H NMR (400 MHz, MeOD) δ 8.90 (s, 1H), 7.88 (d, J = 1.0 Hz, 1H), 7.65 (dd, J = 10.0, 7.2 Hz, 3H), 7.50 (d, J = 4.0 Hz, 1H), 7.24 (d, J = 4.0 Hz, 1H), 7.07 (d, J = 8.0 Hz, 2H), 4.91 (d, J = 16.0 Hz, 1H), 4.55 (d, J = 16.0 Hz, 1H), 4.35 (dd, J = 12.0, 8.0 Hz, 2H), 4.22 (s, 2H), 4.10 (q, J = 7.2 Hz, 1H), 3.80 (d, J = 10.0 Hz, 2H), 3.16 (t, J = 12.0 Hz, 2H), 3.09 - 2.94 (m, 1H), 2.92 - 2.77 (m, 1H), 2.76 - 2.57 (m, 2H), 2.16 (s, 4H). LCMS (ESI): calcd., 584.22, found, 584.2.

### Intermediate Example 102: preparation of 2-(6-(4-((1R,4R)-2,5-diazabicyclo[2.2.1]heptan-2-yl)phenyl)-4-fluoro-1-oxoisoindolin-2-yl)-2-(6,7-dihydro-5H-pyrrolo[1,2-c]imidazol-1-yl)-N-(thiazol-2-yl)acetamide (GT-D-223)

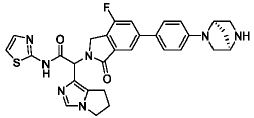

Following steps 2-5 in Scheme 62, the starting material (CAS NO.: 2407965-04-0) and the compound (CAS NO.: 2654825-27-9) were used to prepare the target compound GT-D-223 (off-white solid, 1.3 g, yield 101.7%). ¹H NMR (400 MHz, MeOD) δ 8.90 (s, 1H), 7.84 (d, J = 4.0 Hz, 1H), 7.64-7.58 (m, 4H), 7.35(d, J = 4.0 Hz, 1H), 6.81 (d, J = 8.7 Hz, 2H), 4.92 (s, 1H), 4.87 - 4.84 (m, 1H), 4.74 (s, 1H), 4.67 - 4.48(m, 2H), 4.39-4.32 (m, 2H), 3.80-3.77 (m, 1H), 3.40 (s, 3H), 3.11 - 2.96 (m, 1H), 2.95 -2.82 (m, 1H), 2.72-2.66 (m, 2H), 2.32 (d, J = 8.0 Hz, 1H), 2.10 (d, J = 12.0 Hz,1H). LCMS (ESI): calcd., 570.21, found, 570.2.

### Intermediate Example 103: preparation of 2-(6-(4-(4-(3,3-difluoropiperidin-4-yl)piperazin-1-yl)phenyl)-4-fluoro-1-oxoisoindolin-2-yl)-2-(6,7-dihydro-5H-pyrrolo[1,2-c]imidazol-1-yl)-N-(thiazol-2-yl)acetamide (GT-D-218)

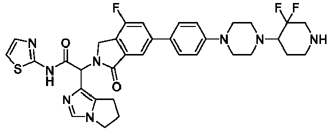

Following steps 3-6 in Scheme 63, the starting material 2 and the compound tert-butyl 3,3-difluoro-4-oxopiperidine-1-carboxylate were used to prepare the target compound GT-D-218 (off-white solid, 645 mg, yield 103.32%). ¹H NMR (400 MHz, MeOD) δ 8.92 (s, 1H), 7.93 (d,J = 1.2 Hz, 1H), 7.84 (d,J = 8.8 Hz, 2H), 7.74 (dd,J = 10.0, 1.2 Hz, 1H), 7.69 - 7.55 (m, 3H), 7.42 (d, J = 4.0 Hz, 1H), 4.95 (s, 2H), 4.68 (d, J = 16.0 Hz,1H), 4.45 - 4.24 (m, 2H), 3.88 (t, J = 8.0 Hz, 2H), 3.76 (s, 4H), 3.67 - 3.49 (m, 6H), 3.33 (d, J = 12.0 Hz,1H), 3.10 - 2.96 (m, 1H), 2.96 - 2.81 (m, 1H), 2.77 - 2.59 (m, 2H), 2.49 (d, J = 12.0 Hz, 1H), 2.37 - 2.18(m, 1H). LCMS (ESI): calcd., 677.26, found, 677.3

### Intermediate Example 104: preparation of N-(5-(2-hydroxypropan-2-yl)-2-(piperidin-4-yl)benzo[d]oxazol-6-yl)-6-(trifluoromethyl)picolinamide (GT-D-208)

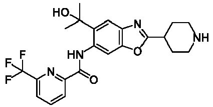

The target compound GT-D-208 was prepared by referring to Scheme 64 (yellow solid, 1.7 g, yield 109%). ¹H NMR (400 MHz, MeOD) δ 8.79 (s, 1H), 8.48 (d, *J* = 8.0 Hz, 1H), 8.30 (t, *J* = 8.0 Hz, 1H), 8.04 (d, *J* = 7.6 Hz, 1H), 7.71 (s, 1H), 3.56 - 3.48 (m, 3H), 3.47 - 3.41 (m, 1H), 3.28 - 3.20 (m, 2H), 2.45 (dd, *J* = 14.4, 3.6 Hz, 2H), 2.23 - 2.11 (m, 2H), 1.71 (s, 6H). LCMS (ESI): calcd., 449.18, found, 449.2.

### Intermediate Example 105: preparation of N-(6-(2-hydroxypropan-2-yl)-2-(piperidin-4-yl)-2H-indazol-5-yl)-6-(trifluoromethyl)picolinamide (GT-D-210)

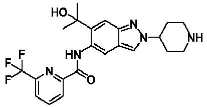

The target compound GT-D-210 was prepared by referring to Scheme 65 (yellow solid, 1.52 g, yield 109.4%). ¹H NMR (400 MHz, D₂O) δ 8.20 (s, 1H), 8.10 (s, 1H), 8.04 (d, *J* = 4.0 Hz, 2H), 7.85 - 7.79 (m, 1H), 7.49 (s, 1H), 4.67 - 4.62 (m, 1H), 3.57 (d, *J* = 13.2 Hz, 2H), 3.20 (dd, *J* = 12.8, 10.0 Hz, 2H), 2.34 - 2.14 (m, 4H), 1.54 (s, 6H). LCMS (ESI): calcd., 448.20, found, 448.2.

### Intermediate Example 106: preparation of 2-(6-(4-(3,3-difluoro-4-(piperazin-1-yl)piperidin-1-yl)phenyl)-4-fluoro-1-oxoisoindolin-2-yl)-2-(6,7-dihydro-5H-pyrrolo[1,2-c]imidazol-1-yl)-N-(thiazol-2-yl)acetamide (GT-D-220)

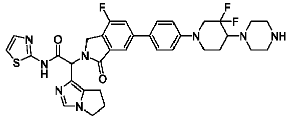

The target compound GT-D-220 was prepared by referring to Scheme 66 (brown solid, 0.5 g, yield 66.6%). ¹H NMR (400 MHz, MeOD) δ 7.83 (s, 1H), 7.65 (s, 1H), 7.60-7.58 (m, 3H), 7.44 (d, J = 3.6 Hz, 1H), 7.15 (d, J = 4.0 Hz, 1H), 7.06 (d, J = 8.0 Hz, 2H), 4.85 (s, 1H), 4.81 (s, 1H), 4.25 (d, J = 16.0 Hz, 1H), 4.12-4.04 (m, 2H), 3.98-3.91 (m, 2H), 3.19 (d, J = 4.0 Hz, 4H), 3.16 - 2.76 (m, 8H), 2.65-2.56 (m, 3H), 2.11-2.06 (m, 1H), 2.00 - 1.86 (m, 1H). LCMS (ESI): calcd., 677.26found, 677.4.

### Example 1: preparation of 3-(5-(((3S,4R)-4-(4-amino-3-(4-phenoxyphenyl)-1H-pyrazolo[3,4-d]pyrimidin-1-yl)-3-fluoropiperidin-1-yl)methyl)-1-oxoisoindolin-2-yl)piperidine-2,6-dione (GT-03297)

Referring to the method of Step 2 in Scheme 11, to a solution of the compound 1-((3S,4R)-3-fluoropiperidin-4-yl)-3-(4-phenoxyphenyl)-1H-pyrazolo[3,4-d]pyrimidin-4-amine (205 mg, 0.51 mmol) prepared from Intermediate Example 1 in anhydrous DMF (10 mL) were added sequentially 3-(5-(bromomethyl)-1-oxoisoindolin-2-yl)piperidine-2,6-dione (171 mg, 0.51 mmol) andDIEA (0.25 ml, 1.52 mmol). The reaction solution was heated to 50°C and stirred for 18 hours. TLC analysis showed completion of the reaction. The reaction solution was cooled to room temperature and purified using preparative high-performance liquid chromatography to obtain the target compound 3-(5-(((3S,4R)-4-(4-amino-3-(4-phenoxyphenyl)-1H-pyrazolo[3,4-d]pyrimidin-1-yl)-3-fluoropiperidin-1-yl)methyl)-1-oxoisoindolin-2-yl)piperidine-2,6-dione (white solid, 182 mg, yield 51%). ¹HNMR (400 MHz, MeOD) *δ* 8.35 (s, 1H), 7.85 (d, *J* = 8.0 Hz, 1H), 7.77 (s, 1H), 7.67 (d, *J* = 8.0 Hz, 1H), 7.57 (d, *J* = 8.0 Hz, 2H), 7.32 (t, *J* = 8.0, 7.5 Hz, 2H), 7.13 - 7.04 (m, 3H), 7.04 - 6.98 (m, 2H), 5.41-5.26 (m, 2H), 5.09 (dd, *J* = 12.0, 4.0 Hz, 1H), 4.57-4.45 (m, 4H), 3.84-3.72 (m, 3H), 3.69-3.63 (m, 1H), 3.48-3.42 (m, 1H), 2.89-2.78 (m, 1H), 2.72-2.67 (m, 1H), 2.45 -2.38 (m, 2H), 2.12 - 2.08 (m, 1H). LC/MS (ESI) m/z: calcd for C₃₆H₃₄FN₈O₄⁺ [M+H]⁺, 661.3; found, 661.3.

### Example 2: preparation of 3-(5-(((3R,4S)-4-(4-amino-3-(4-phenoxyphenyl)-1H-pyrazolo[3,4-d]pyrimidin-1-yl)-3-fluoropiperidin-1-yl)methyl)-1-oxoisoindolin-2-yl)piperidine-2,6-dione (GT-03331)

Referring to the method of Step 2 in Scheme 11 and the method of Example 1, the target compound (GT-03331) was prepared using 1-((3R,4S)-3-fluoropiperidin-4-yl)-3-(4-phenoxyphenyl)-1H-pyrazolo[3,4-d]pyrimidin-4-amine and 3-(5-(bromomethyl)-1-oxoisoindolin-2-yl)piperidine-2,6-dione. The target compound (GT-03331) was obtained as a white solid (175 mg, yield 61.5%). ¹H NMR (400 MHz, MeOD) δ 8.27 (s, 1H), 7.85 (d, *J* = 7.8 Hz, 1H), 7.76 (s, 1H), 7.66 (d, *J* = 7.9 Hz, 1H), 7.56 (d, *J* = 8.6 Hz, 2H), 7.32 (t, *J* = 8.0 Hz, 2H), 7.15 - 6.95 (m, 5H), 5.29 (t, *J* = 33.3 Hz, 2H), 5.09 (dd, *J* = 13.4, 5.1 Hz, 1H), 4.52 (dd, *J* = 14.4, 10.0 Hz, 4H), 3.74 (d, *J* = 11.8 Hz, 2H), 3.66 - 3.53 (m, 1H), 3.42 (t, *J* = 12.4 Hz, 1H), 3.11 (dt, *J* = 3 3.7, 16.9 Hz, 1H), 2.91 - 2.63 (m, 2H), 2.42 (dt, *J* = 18.2, 10.9 Hz, 2H), 2.15 - 2.05 (m, 1H). LCMS (ESI) calcd for C₃₆H₃₄FN₈O₄⁺ [M+H]⁺: 661.27, found, 661.2.

### Example 3: preparation of 3-(5-(((3R,4R)-4-(4-amino-3-(4-phenoxyphenyl)-1H-pyrazolo[3,4-d]pyrimidin-1-yl)-3-fluoropiperidin-1-yl)methyl)-1-oxoisoindolin-2-yl)piperidine-2,6-dione (GT-03287)

Referring to the method of Step 2 in Scheme 11 and the method of Example 1, the target compound (GT-03287) was prepared using 1-((3R,4R)-3-fluoropiperidin-4-yl)-3-(4-phenoxyphenyl)-1H-pyrazolo[3,4-d]pyrimidin-4-amine and 3-(5-(bromomethyl)-1-oxoisoindolin-2-yl)piperidine-2,6-dione. The target compound (GT-03287) was obtained as a white solid (23 mg, yield 46%). ¹H NMR (400 MHz, MeOD) δ 8.31 (s, 1H), 7.82 (d, *J* = 7.8 Hz, 1H), 7.74 (s, 1H), 7.64 (d, *J* = 7.9 Hz, 1H), 7.53 (s, 2H), 7.36 - 7.27 (m, 2H), 7.17 - 6.95 (m, 5H), 5.29 (d, *J* = 45.4 Hz, 2H), 5.07 (dd, *J* = 13.4, 5.1 Hz, 1H), 4.60 - 4.35 (m, 4H), 3.79 (d, *J* = 2.5 Hz, 1H), 3.64 - 3.50 (m, 1H), 3.22 (s, 2H), 2.86 - 2.74 (m, 1H), 2.68 (d, *J* = 15.5 Hz, 1H), 2.42 (ddd, *J* = 22.0, 16.3, 11.7 Hz, 3H), 2.13 - 2.03 (m, 1H). LCMS (ESI) calcd for C₃₆H₃₄FN₈O₄⁺ [M+H]⁺: 661.27, found, 661.3.

### Example 4: preparation of N-((1r,4r)-4-(3-chloro-4-cyanophenoxy)cyclohexyl)-6-(4-((2-(2,6-dioxopiperidin-3-yl)-1-oxoisoindolin-5-yl)methyl)piperazin-1-yl)pyridazine-3-carboxamide (GT-02912)

Referring to the method of Step 2 in Scheme 11 and the method of Example 1, the target compound (GT-02912) was prepared using N-((1r,4r)-4-(3-chloro-4-cyanophenoxy)cyclohexyl)-6-(piperazin-1-yl)pyridazine-3-carboxamide (15.00 mg, 0.034 mmol) prepared from Intermediate Example 12 and 3-(5-(bromomethyl)-1-oxoisoindolin-2-yl)piperidine-2,6-dione (11.47 mg, 0.034 mmol). The target compound (GT-02912) was obtained as a white solid (10 mg, yield 41.32%). ¹H NMR (400 MHz, DMSO) *δ* ppm 11.00 (s, 1H), 8.65 (d, *J* = 8.2 Hz, 1H), 7.96-7.82 (m, 4H), 7.76 (d, *J* = 7.9 Hz, 1H), 7.45 (d, *J* = 9.6 Hz, 1H), 7.37 (d, *J* = 2.4 Hz, 1H), 7.12 (dd, *J* = 8.8, 2.4 Hz, 1H), 5.14 (dd, *J* = 13.2, 5.1 Hz, 1H), 4.48 (m, 7H), 3.92-3.82 (m, 1H), 3.60 (m, 3H), 3.12 (m, 3H), 2.93 (dd, *J* = 15.2, 10.5 Hz, 1H), 2.61 (d, *J* = 16.7 Hz, 2H), 2.10 (dd, *J* = 10.8, 1.1 Hz, 2H), 2.06-1.99 (m, 1H), 1.90 (dd, *J* = 12.9, 2.5 Hz, 2H), 1.64 (dd, *J* = 24.1, 10.8 Hz, 2H), 1.51 (dd, *J* = 22.7, 9.8 Hz, 2H). LCMS (ESI) m/z: calcd for C₃₆H₃₈ClN₈O₅⁺ [M+H]⁺, 697.26; found, 697.30.

### Example 5: preparation of N-((1r,4r)-4-(3-chloro-4-cyanophenoxy)cyclohexyl)-6-(3-((2-(2,6-dioxopiperidin-3-yl)-1-oxoisoindolin-5-yl)methyl)-3,8-diazabicyclo[3.2.1]octan-8-yl)pyridazine-3-carboxamide (GT-02932)

Referring to the method of Step 2 in Scheme 11 and the method of Example 1, the target compound (GT-02932) was prepared using 6-(3,8-diazabicyclo[3.2.1]octan-8-yl)-N-((1r,4r)-4-(3-chloro-4-cyanophenoxy)cyclohexyl)pyridazine-3-carboxamide (15.00 mg, 0.032 mmol) prepared by referring to Scheme 5 and Intermediate Example 12, and 3-(5-(bromomethyl)-1-oxoisoindolin-2-yl)piperidine-2,6-dione hydrochloride (10.83 mg, 0.032 mmol). The target compound (GT-02932) was obtained as a white solid (9.00 mg, yield 37.97%). ¹H NMR (400 MHz, DMSO) *δ* ppm 11.00 (s, 1H), 8.60 (d, *J* = 8.2 Hz, 1H), 7.92 (d, *J* = 9.2 Hz, 1H), 7.86 (d, *J* = 8.8 Hz, 2H), 7.74 (d, *J* = 7.7 Hz, 1H), 7.69 (d, *J* = 7.7 Hz, 1H), 7.39 (d, *J* = 2.4 Hz, 1H), 7.18-7.10 (m, 2H), 5.11 (dd, *J* = 13.3, 5.0 Hz, 1H), 4.64 (s, 2H), 4.57-4.51 (m, 1H), 4.39 (t, *J =* 11.8 Hz, 1H), 4.32 (s, 2H), 3.92-3.84 (m, 1H), 3.64-3.59 (m, 3H), 3.12 (m, 2H), 2.97-2.83 (m, 2H), 2.73-2.60 (m, 2H), 2.42 (dd, *J* = 13.0, 4.9 Hz, 1H), 2.10 (dd, *J* = 19.2, 8.2 Hz, 3H), 1.93 (d, *J* = 13.0 Hz, 2H), 1.71-1.61 (m, 2H), 1.53 (dd, *J* = 22.9, 12.5 Hz, 2H). LCMS (ESI) m/z: calcd for C₃₈H₄₀ClN₈O₅⁺ [M+H]⁺, 723.27; found, 723.20.

### Example 6: preparation of N-((1r,4r)-4-(3-chloro-4-cyanophenoxy)cyclohexyl)-6-(8-((2-(2,6-dioxopiperidin-3-yl)-1-oxoisoindolin-5-yl)methyl)-3,8-diazabicyclo[3.2.1]octan-3-yl)pyridazine-3-carboxamide (GT-02987)

Referring to the method of Step 2 in Scheme 11 and the method of Example 1, the target compound (GT-02987) was prepared by using 6-(3,8-diazabicyclo[3.2.1]octan-3-yl)-N-((1r,4r)-4-(3-chloro-4-cyanophenoxy)cyclohexyl)pyridazine-3-carboxamide (10.00 mg, 0.021 mmol) prepared by referring to Scheme 5 and Intermediate Example 12, and 3-(5-(bromomethyl)-1-oxoisoindolin-2-yl)piperidine-2,6-dione (7.22 mg, 0.021 mmol). The target compound (GT-02987) was obtained as a white solid (6.00 mg, yield 37.96%). ¹H NMR (400 MHz, DMSO) *δ* ppm 10.94 (s, 1H), 9.27 (dd, *J* = 14.3, 6.6 Hz, 2H), 8.63-8.46 (m, 1H), 7.78 (d, *J* = 8.8 Hz, 2H), 7.66 (s, 2H), 7.31 (d, *J* = 2.4 Hz, 1H), 7.06 (dd, *J* = 8.8, 2.4 Hz, 1H), 5.09-5.02 (m, 1H), 4.48-4.39 (m, 2H), 4.30 (m, 2H), 4.00-3.89 (m, 2H), 3.81-3.77 (m, 1H), 3.54-3.52 (m, 3H), 3.06 (dd, *J* = 7.3, 3.2 Hz, 4H), 2.96-2.75 (m, 1H), 2.62-2.53 (m, 1H), 2.31 (dd, *J* = 32.9, 6.7 Hz, 2H), 2.06-1.92 (m, 4H), 1.83 (d, *J* = 11.2 Hz, 2H), 1.60-1.52 (m, 2H), 1.44 (dd, *J* = 22.4, 9.1 Hz, 2H). LCMS (ESI) m/z: calcd for C₃₈H₄₀ClN₈O₅⁺ [M+H]⁺, 723.27; found, 723.30.

### Example 7: preparation of N-((1r,4r)-4-(3-chloro-4-cyanophenoxy)cyclohexyl)-6-(3-((2-(2,6-dioxopiperidin-3-yl)-1-oxoisoindolin-5-yl)methyl)-3,6-diazabicyclo[3.1.1]heptan-6-yl)pyridazine-3-carboxamide (GT-02933)

Referring to the method of Step 2 in Scheme 11 and the method of Example 1, the target compound (GT-02933) was prepared using 6-(3,6-diazabicyclo[3.1.1]heptan-6-yl)-N-((1r,4r)-4-(3-chloro-4-cyanophenoxy)cyclohexyl)pyridazine-3-carboxamide (15.00 mg, 0.033 mmol) prepared by referring to Scheme 5 and Intermediate Example 12, and 3-(5-(bromomethyl)-1-oxoisoindolin-2-yl)piperidine-2,6-dione (11.17 mg, 0.033 mmol). The target compound (GT-02933) was obtained as a white solid (8.00 mg, yield 33.38%). ¹H NMR (400 MHz, DMSO) *δ* ppm 11.00 (s, 1H), 8.60 (d, *J* = 8.2 Hz, 1H), 7.92 (d, *J* = 9.2 Hz, 1H), 7.86 (d, *J* = 8.8 Hz, 2H), 7.74 (d, *J* = 7.7 Hz, 1H), 7.69 (d, *J* = 7.7 Hz, 1H), 7.39 (d, *J* = 2.4 Hz, 1H), 7.18-7.10 (m, 2H), 5.11 (dd, *J* = 13.3, 5.0 Hz, 1H), 4.64 (s, 2H), 4.57-4.51 (m, 1H), 4.39 (t, *J* = 11.8 Hz, 1H), 4.32 (s, 2H), 3.92-3.84 (m, 1H), 3.64-3.59 (m, 3H), 3.12 (qd, *J* = 7.4, 4.3 Hz, 2H), 2.97-2.83 (m, 2H), 2.73-2.60 (m, 2H), 2.42 (dd, *J* = 13.0, 4.9 Hz, 1H), 2.10 (dd, *J* = 19.2, 8.2 Hz, 3H), 1.93 (d, *J* = 13.0 Hz, 2H), 1.71-1.61 (m, 2H), 1.53 (dd, *J* = 22.9, 12.5 Hz, 2H). LCMS (ESI) m/z: calcd for C₃₇H₃₈ClN₈O₅⁺ [M+H]⁺, 709.26; found, 709.30.

### Example 8: preparation of N-((1r,4r)-4-(3-chloro-4-cyanophenoxy)cyclohexyl)-6-(6-((2-(2,6-dioxopiperidin-3-yl)-1-oxoisoindolin-5-yl)methyl)-3,6-diazabicyclo[3.1.1]heptan-3-yl)pyridazine-3-carboxamide (GT-02934)

### Referring to the method of Step 2 in Scheme 11 and the method of Example 1, the target compound (GT-02934) was prepared using 6-(3,6-diazabicyclo[3.1.1]heptan-3-yl)-N-((1r,4r)-4-(3-chloro-4-cyanophenoxy)cyclohexyl)pyridazine-3-carboxamide (15.00 mg, 0.033 mmol) prepared by referring to Scheme 5 and Intermediate Example 12, and 3-(5-(bromomethyl)-1-oxoisoindolin-2-yl)piperidine-2,6-dione (11.17 mg, 0.033 mmol). The target compound (GT-02934) was obtained as a white solid (9.00 mg, yield 37.55%). ¹H NMR (400 MHz, DMSO) δ ppm 11.00 (s, 1H), 8.67 (t, J = 7.8 Hz, 1H), 8.05-7.88 (m, 2H), 7.85 (dt, J = 8.4, 5.3 Hz, 2H), 7.75-7.67 (m, 1H), 7.38 (s, 1H), 7.32-7.10 (m, 2H), 5.12 (dd, J = 11.7, 6.5 Hz, 1H), 4.94-4.74 (m, 2H), 4.54-4.50 (m, 1H), 4.38 (d, J = 11.3 Hz, 1H), 4.10 (d, J = 33.2 Hz, 2H), 3.92-3.84 (m, 1H), 3.61-3.56 (m, 3H), 3.12-3.09 (m, 2H), 3.01-2.84 (m, 2H), 2.68-2.57 (m, 2H), 2.39 (dd, J = 13.1, 4.3 Hz, 1H), 2.10-2.00 (m, 3H), 1.94-1.85 (m, 2H), 1.71-1.60 (m, 2H), 1.56-1.46 (m, 2H). LCMS (ESI) m/z: calcd for C₃₇H₃₈ClN₈O₅⁺ [M+H]⁺, 709.26; found, 709.20.

### Example 9: preparation of N-((1r,4r)-4-(3-chloro-4-cyanophenoxy)cyclohexyl)-6-(4-(4-((2-(2,6-dioxopiperidin-3-yl)-1-oxoisoindolin-5-yl)methyl)piperazin-1-yl)piperidin-1-yl)pyridazine-3-carboxamide (GT-02989)

Referring to the method of Step 2 in Scheme 11 and the method of Example 1, the target compound (GT-02989) was prepared using N-((1r,4r)-4-(3-chloro-4-cyanophenoxy)cyclohexyl)-6-(4-(piperazin-1-yl)piperidin-1-yl)pyridazine-3-carboxamide (20.00 mg, 0.038 mmol) prepared by referring to Scheme 5 and Intermediate Example 12, and 3-(5-(bromomethyl)-1-oxoisoindolin-2-yl)piperidine-2,6-dione (12.87 mg, 0.038 mmol). The target compound (GT-02989) was obtained as a white solid (12.00 mg, yield 39.49%). ¹H NMR (400 MHz, DMSO) *δ* ppm 10.99 (s, 1H), 8.60 (d, *J* = 8.2 Hz, 1H), 7.87-7.77 (m, 4H), 7.72 (d, *J* = 8.2 Hz, 1H), 7.44 (d, *J* = 9.7 Hz, 1H), 7.37 (d, *J* = 2.3 Hz, 1H), 7.13 (dd, *J* = 8.8, 2.3 Hz, 1H), 5.13 (dd, *J* = 13.2, 5.1 Hz, 1H), 4.65 (d, *J* = 12.9 Hz, 2H), 4.56-4.30 (m, 5H), 3.87 (dd, *J* = 11.5, 7.2 Hz, 1H), 3.69-3.55 (m, 5H), 2.95 (m, 5H), 2.71-2.54 (m, 2H), 2.47-2.30 (m, 2H), 2.19 (d, *J* = 9.6 Hz, 2H), 2.10 (d, *J* = 10.6 Hz, 2H), 2.04-1.95 (m, 1H), 1.89 (d, *J* = 9.7 Hz, 2H), 1.66 (dt, *J* = 24.5, 11.4 Hz, 4H), 1.51 (dd, *J* = 22.6, 10.1 Hz, 2H). LCMS (ESI) m/z: calcd for C₄₁H₄₇ClN₉O₅⁺ [M+H]⁺, 780.33; found, 780.30.

### Example 10: preparation of N-((1r,3r)-3-(3-chloro-4-cyanophenoxy)-2,2,4,4-tetramethylcyclobutyl)-6-(4-((2-(2,6-dioxopiperidin-3-yl)-1-oxoisoindolin-5-yl)methyl)piperazin-1-yl)nicotinamide (GT-02988)

Referring to the method of Step 2 in Scheme 11 and the method of Example 1, the target compound (GT-02988) was synthesized using N-((1r,3r)-3-(3-chloro-4-cyanophenoxy)-2,2,4,4-tetramethylcyclobutyl)-6-(piperazin-1-yl)nicotinamide (10.00 mg, 0.021 mmol) prepared by referring to Scheme 5 and Intermediate Example 12, and 3-(5-(bromomethyl)-1-oxoisoindolin-2-yl)piperidine-2,6-dione (7.20 mg, 0.021 mmol). The target compound (GT-02988) was obtained as a white solid (9.00 mg, yield 56.98%). ¹H NMR (400 MHz, DMSO) *δ* ppm 8.62 (d, *J* = 2.3 Hz, 1H), 7.96 (dt, *J* = 9.0, 2.6 Hz, 1H), 7.89 (d, *J* = 8.8 Hz, 1H), 7.65 (t, *J* = 9.4 Hz, 2H), 7.58 (s, 1H), 7.46 (d, *J* = 7.6 Hz, 1H), 7.20 (d, *J* = 2.4 Hz, 1H), 7.00 (dd, *J* = 8.8, 2.4 Hz, 1H), 6.85 (d, *J* = 9.1 Hz, 1H), 4.74 (dd, *J* = 10.3, 4.7 Hz, 1H), 4.69-4.58 (m, 1H), 4.43 (dd, *J* = 27.3, 17.6 Hz, 1H), 4.31 (s, 1H), 4.04 (d, *J* = 9.2 Hz, 1H), 3.63 (d, *J* = 3.9 Hz, 7H), 2.79 (s, 3H), 2.23-2.11 (m, 2H), 2.10-1.76 (m, 2H), 1.11 (s, 12H). LCMS (ESI) m/z: calcd for C₃₉H₄₃ClN₇O₅⁺ [M+H]⁺, 724.29; found, 724.30.

### Example 11: preparation of 3-(5-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-1-oxoisoindolin-2-yl)piperidine-2,6-dione (GT-02821)

Referring to the method of Step 2 in Scheme 11 and the method of Example 1, the target compound (GT-02821) was prepared using 5-fluoro-4-(4-fluoro-1-isopropyl-2-methyl-1H-benzo[d]imidazol-6-yl)-N-(5-(piperazin-1-ylmethyl)pyridin-2-yl)pyrimidin-2-amine (CAS NO.: 1231930-57-6) (20.00 mg, 0.042 mmol) and 3-(5-(bromomethyl)-1-oxoisoindolin-2-yl)piperidine-2,6-dione (14.09 mg, 0.042 mmol). The target compound (GT-02821) was obtained as a white solid (7.00 mg, yield 22.34%). ¹H NMR (400 MHz, DMSO) *δ* ppm 10.97 (s, 1H), 10.06 (s, 1H), 8.69 (d, *J* = 3.8 Hz, 1H), 8.30 (s, 1H), 8.20 (d, *J* = 8.6 Hz, 2H), 7.67 (dd, *J* = 14.3, 6.9 Hz, 3H), 7.53 (s, 1H), 7.43 (d, *J* = 7.9 Hz, 1H), 5.10 (dd, *J* = 13.2, 5.1 Hz, 1H), 4.84 (dt, *J* = 14.0, 7.1 Hz, 1H), 4.44 (d, *J* = 17.3 Hz, 1H), 4.31 (d, *J* = 17.3 Hz, 1H), 3.58 (s, 2H), 3.46 (s, 2H), 2.99-2.81 (m, 3H), 2.73 (s, 1H), 2.6-2.57 (m, 4H), 2.42-2.32 (m, 5H), 2.05-1.94 (m, 2H), 1.62 (d, *J* = 6.9 Hz, 6H). LCMS (ESI) m/z: calcd for C₃₉H₄₁F₂N₁₀O₃⁺ [M+H]⁺, 734.33; found, 735.30.

### Example 12: preparation of 7-cyclopentyl-2-((5-(4-((2-(2,6-dioxopiperidin-3-yl)-1-oxoisoindolin-5-yl)methyl)piperazin-1-yl)pyridin-2-yl)amino)-N,N-dimethyl-7H-pyrrolo [2,3-d]pyrimidine-6-carboxamide (GT-02822)

Referring to the method of Step 2 in Scheme 11 and the method of Example 1, the target compound (GT-02822) was synthesized from 7-cyclopentyl-N,N-dimethyl-2-((5-(piperazin-1-yl)pyridin-2-yl)amino)-7H-pyrrolo[2,3-d]pyrimidine-6-carboxamide (CAS NO.: 1211441-98-3) (20.00 mg, 0.046 mmol) and 3-(5-(bromomethyl)-1-oxoisoindolin-2-yl)piperidine-2,6-dione (15.52 mg, 0.046 mmol). The target compound (GT-02822) was obtained as a white solid (5.00 mg, yield 15.41%). ¹H NMR (400 MHz, DMSO) *δ* ppm 10.99 (s, 1H), 9.37 (s, 1H), 8.77 (s, 1H), 8.15 (d, *J* = 9.1 Hz, 1H), 8.01 (d, *J* = 2.7 Hz, 1H), 7.70 (d, *J* = 7.7 Hz, 1H), 7.58 (s, 1H), 7.49 (d, *J* = 7.8 Hz, 1H), 7.42 (dd, *J* = 9.1, 2.7 Hz, 1H), 6.59 (s, 1H), 5.12 (dd, *J* = 13.2, 5.0 Hz, 1H), 4.80-4.66 (m, 1H), 4.46 (d, *J* = 17.4 Hz, 1H), 4.33 (d, *J* = 17.3 Hz, 1H), 3.66 (s, 2H), 3.10 (d, *J* = 37.3 Hz, 10H), 3.00-2.84 (m, 2H), 2.64-2.54 (m, 5H), 2.46-2.32 (m, 3H), 1.97 (s, 4H), 1.68-1.56 (m, 2H). LCMS (ESI) m/z: calcd for C₃₇H₄₃N₁₀O₄⁺ [M+H]⁺, 691.34; found, 691.30.

### Example 13: preparation of 3-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-1-oxoisoindolin-2-yl)piperidine-2,6-dione (GT-02812)

Referring to the method of Step 2 in Scheme 11 and the method of Example 1, the target compound (GT-02812) was prepared from 6-acetyl-8-cyclopentyl-5-methyl-2-((5-(piperazin-1-yl)pyridin-2-yl)amino)pyrido[2,3-d]pyrimidin-7(8H)-one (CAS NO.: 571190-30-2) (20.00 mg, 0.045 mmol) and 3-(5-(bromomethyl)-1-oxoisoindolin-2-yl)piperidine-2,6-dione (15.07 mg, 0.045 mmol). The target compound (GT-02812) was obtained as a yellow solid (10.00 mg, yield 31.16%). ¹H NMR (400 MHz, DMSO) *δ* ppm 10.98 (s, 1H), 10.07 (s, 1H), 8.95 (s, 1H), 8.04 (s, 1H), 7.84 (d, *J* = 8.0 Hz, 1H), 7.70 (d, *J* = 7.2 Hz, 1H), 7.59 (s, 1H), 7.52-7.43 (m, 2H), 5.90-5.74 (m, 1H), 5.18-5.06 (m, 1H), 4.47 (d, *J* = 14.7 Hz, 1H), 4.34 (d, *J* = 19.7 Hz, 1H), 3.67 (s, 2H), 3.20-3.13 (m, 4H), 2.96-2.85 (m, 3H), 2.75-2.55 (m, 10H), 2.30 (s, 3H), 1.82 (m, 4H), 1.61-1.54 (m, 2H). LCMS (ESI) m/z: calcd for C₃₈H₄₂N₉O₅⁺ [M+H]⁺, 704.32; found, 704.40.

### Example 14: preparation of 3-(5-(((4-(8-fluoro-1-oxo-2,3,4,6-tetrahydro-1H-azepino[5,4,3-cd]indol-5-yl)benzyl)(methyl)amino)methyl)-1-oxoisoindolin-2-yl)piperidine-2,6-dione (GT-03260)

Referring to the method of Step 2 in Scheme 11 and the method of Example 1, the target compound (GT-03260) was synthesized from 8-fluoro-5-(4-((methylamino)methyl)phenyl)-2,3,4,6-tetrahydro-1H-azepino[5,4,3-cd]indol-1-one (CAS NO.: 283173-50-2) (15.00 mg, 0.046 mmol) and 3-(5-(bromomethyl)-1-oxoisoindolin-2-yl)piperidine-2,6-dione (15.64 mg, 0.046 mmol). The target compound (GT-03260) was obtained as a white solid (3.00 mg, yield 10.93%). ¹H NMR (400 MHz, DMSO) δ ppm 11.89 (s, 1H), 11.01 (s, 1H), 8.29 (t, J = 5.7 Hz, 1H), 7.91 (d, J = 5.9 Hz, 1H), 7.81 (dd, J = 10.7, 8.1 Hz, 4H), 7.74 - 7.69 (m, 2H), 7.43 (dd, J = 10.9, 2.4 Hz, 1H), 7.36 (dd, J = 9.1, 2.4 Hz, 1H), 5.14 (dd, J = 13.3, 5.0 Hz, 1H), 4.59 - 4.45 (m, 3H), 4.38 (dd, J = 17.5, 9.4 Hz, 2H), 4.31 (dd, J = 12.6, 6.1 Hz, 1H), 3.61 - 3.57 (m, 1H), 3.13 - 3.06 (m, 3H), 2.98 - 2.85 (m, 1H), 2.59 (t, J = 9.0 Hz, 4H), 2.46 - 2.35 (m, 1H), 2.04 - 1.95 (m, 1H). LCMS (ESI) m/z: calcd for C₃₃H₃₁FN₅O₄⁺ [M+H]⁺, 578.23; found, 578.23.

### Example 17: preparation of 3-(5-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)-1-oxoisoindolin-2-yl)piperidine-2,6-dione (GT-02811)

Referring to the method of Step 2 in Scheme 11 and the method of Example 1, the target compound (GT-02811) was synthesized from (2-((5-chloro-2-((2-methoxy-4-(4-(piperazin-1-yl)piperidin-1-yl)phenyl)amino)pyrimidin-4-yl)amino)phenyl)dimethyl phosphine oxide (20.00 mg, 0.035 mmol) prepared by referring to the method of Scheme 3 and 3-(5-(bromomethyl)-1-oxoisoindolin-2-yl)piperidine-2,6-dione (11.83 mg, 0.035 mmol). The target compound (GT-02811) was obtained as a white solid (10.00 mg, yield 17.25%). ¹H NMR (400 MHz, DMSO) *δ* ppm 11.71 (s, 1H), 11.00 (s, 1H), 9.12 (d, *J* = 131.8 Hz, 1H), 8.42 (s, 1H), 8.20 (s, 1H), 7.87 (s, 1H), 7.79 (t, *J* = 8.5 Hz, 2H), 7.60 (dd, *J* = 13.5, 7.7 Hz, 1H), 7.46-7.37 (m, 2H), 7.21 (t, *J* = 7.2 Hz, 1H), 6.81 (s, 1H), 6.65 (s, 1H), 5.14 (dd, *J* = 13.3, 5.0 Hz, 1H), 4.43 (dd, *J* = 51.4, 17.6 Hz, 4H), 3.88 (d, *J* = 11.4 Hz, 3H), 3.79 (s, 3H), 3.63-3.58 (m, 4H), 3.11 (m, 2H), 2.92 (m, 4H), 2.61 (d, *J* = 16.4 Hz, 1H), 2.45-2.37 (m, 1H), 2.21 (d, *J* = 9.8 Hz, 2H), 2.04-1.99 (m, 1H), 1.90 (dt, *J* = 13.5, 10.1 Hz, 2H), 1.80 (s, 3H), 1.77 (s, 3H). LCMS (ESI) m/z: calcd for C₄₂H₅₀ClN₉O₅P⁺ [M+H]⁺, 826.33; found, 826.30.

### Example 18: preparation of 3-(5-((4-(2-fluoro-5-((4-oxo-3,4-dihydrophthalazin-1-yl)methyl)benzoyl)piperazin-1-yl)methyl)-1-oxoisoindolin-2-yl)piperidine-2,6-dione (GT-03327)

Referring to the method of Step 2 in Scheme 11 and the method of Example 1, the target compound (GT-03327) was synthesized from 4-(4-fluoro-3-(piperazine-1-carbonyl)benzyl)phthalazin-1(2H)-one (CAS NO.: 763111-47-3) (30.00 mg, 0.082 mmol) and 3-(5-(bromomethyl)-1-oxoisoindolin-2-yl)piperidine-2,6-dione (27.61 mg, 0.082 mmol). The target compound (GT-03327) was obtained as a white solid (28.00 mg, yield 53.83%). ¹H NMR (400 MHz, DMSO) δ ppm 12.59 (s, 1H), 11.00 (s, 1H), 8.32-8.18 (m, 1H), 7.96 (d, J = 7.9 Hz, 1H), 7.92-7.85 (m, 2H), 7.85-7.79 (m, 2H), 7.76 (d, J = 7.6 Hz, 1H), 7.46 (dd, J = 7.0, 4.3 Hz, 1H), 7.40 (dd, J = 6.4, 1.9 Hz, 1H), 7.25 (t, J = 9.0 Hz, 1H), 5.14 (dd, J = 13.3, 5.1 Hz, 1H), 4.62-4.27 (m, 7H), 3.59 (m, 3H), 3.11 (m, 3H), 3.03-2.85 (m, 2H), 2.61 (d, J = 17.1 Hz, 1H), 2.42 (m, 1H), 2.08-1.95 (m, 1H). LCMS (ESI) m/z: calcd for C₃₄H₃₂FN₆O₅⁺ [M+H]⁺, 623.23; found, 623.30.

### Example 19: preparation of 2-(4-((3S)-1-((2-(2,6-dioxopiperidin-3-yl)-1-oxoisoindolin-5-yl)methyl)piperidin-3-yl)phenyl)-2H-indazole-7-carboxamide (GT-03328)

Referring to the method of Step 2 in Scheme 11 and the method of Example 1, the target compound (GT-03328) was prepared using (S)-2-(4-(piperidin-3-yl)phenyl)-2H-indazole-7-carboxamide (CAS NO.: 1038915-60-4) (30.00 mg, 0.094 mmol) and 3-(5-(bromomethyl)-1-oxoisoindolin-2-yl)piperidine-2,6-dione (38.03 mg, 0.113 mmol). The target compound (GT-03328) was obtained as a white solid (30.00 mg, yield 54.45%). ¹H NMR (400 MHz, DMSO) *δ* ppm 10.98 (s, 1H), 9.24 (s, 1H), 8.61 (s, 1H), 8.14-7.97 (m, 4H), 7.95-7.83 (m, 2H), 7.78 (s, 2H), 7.50 (d, *J* = 8.4 Hz, 2H), 7.30-7.20 (m, 1H), 5.07 (dd, *J* = 13.1, 5.0 Hz, 1H), 4.49 (d, *J* = 15.9 Hz, 2H), 4.37 (d, *J* = 17.7 Hz, 1H), 3.58-3.54 (m, 2H), 3.40 (d, *J* = 8.3 Hz, 1H), 3.33-3.26 (m, 1H), 3.07 (dd, *J* = 7.4, 4.2 Hz, 2H), 2.94-2.80 (m, 1H), 2.60 (d, *J* = 16.3 Hz, 1H), 2.50 (d, *J* = 1.2 Hz, 1H), 2.45-2.31 (m, 1H), 2.02-1.89 (m, 3H), 1.72 (dt, *J* = 14.8, 6.6 Hz, 1H). LCMS (ESI) m/z: calcd for C₃₃H₃₃N₆O₄⁺ [M+H]⁺, 577.25; found, 577.30.

### Example 20: preparation of N-((1r,3r)-3-(3-chloro-4-cyanophenoxy)-2,2,4,4-tetramethylcyclobutyl)-6-(4-((2-(2,6-dioxopiperidin-3-yl)-1-oxoisoindolin-5-yl)methyl)piperazin-1-yl)pyridazine-3-carboxamide (GT-03337)

Referring to the method of Step 2 in Scheme 11 and the method of Example 1, the target compound (GT-03337) was prepared using N-((1r,3r)-3-(3-chloro-4-cyanophenoxy)-2,2,4,4-tetramethylcyclobutyl)-6-(piperazine-1-yl)pyridazine-3-carboxamide (30.00 mg, 0.064 mmol) prepared according to Scheme 5 and Intermediate Example 12, and 3-(5-(bromomethyl)-1-oxoisoindolin-2-yl)piperidine-2,6-dione (25.88 mg, 0.077 mmol). The target compound (GT-03337) was obtained as a white solid (30.00 mg, yield 63.38%). ¹H NMR (400 MHz, DMSO) *δ* ppm 11.01 (s, 1H), 8.28 (d, *J* = 9.2 Hz, 1H), 7.97-7.89 (m, 3H), 7.81 (q, *J* = 7.9 Hz, 2H), 7.49 (d, *J* = 9.6 Hz, 1H), 7.24 (d, *J* = 2.3 Hz, 1H), 7.03 (dd, *J* = 8.8, 2.4 Hz, 1H), 5.14 (dd, *J* = 13.2, 5.0 Hz, 1H), 4.61 (d, *J* = 12.9 Hz, 2H), 4.54- 4.45 (m, 4H), 4.38 (d, *J* = 17.6 Hz, 1H), 4.01 (d, *J* = 9.2 Hz, 1H), 3.59 (dd, *J* = 6.6, 2.6 Hz, 3H), 3.11 (dd, *J* = 7.4, 4.3 Hz, 3H), 2.97-2.88 (m, 1H), 2.64-2.56 (m, 1H), 2.47-2.37 (m, 1H), 2.05-1.98 (m, 1H), 1.22 (s, 6H), 1.14 (s, 6H). LCMS (ESI) m/z: calcd for C₃₈H₄₂ClN₈O₅⁺ [M+H]⁺, 725.29; found, 725.30.

### Example 21: preparation of 3-(5-((4-(4-amino-3-(4-phenoxyphenyl)-1H-pyrazolo[3,4-d]pyrimidin-1-yl)-3-fluoropiperidin-1-yl)methyl)-1-oxoisoindolin-2-yl)piperidine-2,6-dione (GT-02652)

Referring to the method of Step 2 in Scheme 11 and the method of Example 1, the target compound (GT-02652) was synthesized using 1-(3-fluoropiperidin-4-yl)-3-(4-phenoxyphenyl)-1H-pyrazolo[3,4-d]pyrimidin-4-amine prepared according to Intermediate Example 2, and 3-(5-(bromomethyl)-1-oxoisoindolin-2-yl)piperidine-2,6-dione. The target compound (GT-02652) was obtained as a white solid (501 mg, yield 28%). ¹H NMR (500 MHz, CD₃OD) *δ* 10.99 (s, 1H), 8.27 (s, 1H), 7.75 (d, *J* = 7.7 Hz, 1H), 7.71 - 7.64 (m, 3H), 7.59 (s, 1H), 7.47 - 7.40 (m, 2H), 7.21 - 7.11 (m, 5H), 5.39 - 5.18 (m, 1H), 5.12 (dd, *J* = 13.3, 5.1 Hz, 1H), 5.05 - 4.84 (m, 1H), 4.49 (d, *J* = 17.5 Hz, 1H), 4.36 (d, *J* = 17.4 Hz, 1H), 3.99 (s, 2H), 3.23 - 2.99 (m, 2H), 2.97 - 2.85 (m, 1H), 2.61 (d, *J* = 16.7 Hz, 2H), 2.46 - 2.21 (m, 3H), 2.15 - 1.92 (m, 2H). LCMS (ESI) m/z: calcd for C₃₆H₃₃FN₈O₄⁺ [M+H]⁺: 661.27, found, 661.3.

### Example 22: preparation of 3-(5-((4-(4-amino-3-(4-phenoxyphenyl)-1H-pyrazolo[3,4-d]pyrimidin-1-yl)piperidin-1-yl)methyl)-1-oxoisoindolin-2-yl)piperidine-2,6-dione (GT-02742)

Referring to the method of Step 2 in Scheme 11 and the method of Example 1, the target compound (GT-02742) was prepared using 3-(4-phenoxyphenyl)-1-(piperidin-4-yl)-1H-pyrazolo[3,4-d]pyrimidin-4-amine hydrochloride synthesized according to Intermediate Example 2, and 3-(5-(bromomethyl)-1-oxoisoindolin-2-yl)piperidine-2,6-dione. The target compound (GT-02742) was obtained as a white solid (422 mg, yield 24%). ¹H NMR (500 MHz, DMSO) δ 11.00 (s, 1H), 10.85 (s, 1H), 8.38 (s, 1H), 7.89 (s, 1H), 7.84 (d, *J* = 7.8 Hz, 1H), 7.77 (d, *J* = 8.0 Hz, 1H), 7.64 (d, *J* = 8.6 Hz, 2H), 7.49 - 7.40 (m, 2H), 7.24 - 7.08 (m, 5H), 5.14 (dd, *J* = 13.2, 5.1 Hz, 1H), 5.09 - 4.99 (m, 1H), 4.59 - 4.33 (m, 4H), 3.07 - 2.85 (m, 2H), 2.70 - 2.53 (m, 4H), 2.48 - 2.27 (m, 3H), 2.17 (d, *J* = 11.6 Hz, 2H), 2.08 - 1.95 (m, 1H). LCMS (ESI) m/z: calcd for C₃₆H₃₅N₈O₄⁺ [M+H]⁺: 643.28, found, 643.3.

### Example 23: preparation of 3-(5-(((R)-3-(4-amino-3-(4-phenoxyphenyl)-1H-pyrazolo[3,4-d]pyrimidin-1-yl)piperidin-1-yl)methyl)-1-oxoisoindolin-2-yl)piperidine-2,6-dione (GT-02744)

Referring to the method of Step 2 in Scheme 11 and the method of Example 1, the target compound (GT-02744) was prepared using (R)-3-(4-phenoxyphenyl)-1-(piperidin-3-yl)-1H-pyrazolo[3,4-d]pyrimidin-4-amine (CAS NO.: 1022150-12-4) and 3-(5-(bromomethyl)-1-oxoisoindolin-2-yl)piperidine-2,6-dione. The target compound (GT-02744) was obtained as a white solid (422 mg, yield 24%). LCMS (ESI) m/z: calcd for C₃₆H₃₅N₈O₄⁺ [M+H]⁺: 643.28, found, 643.3.

### Example 24: preparation of N-(tert-butyl)-3-((2-((4-(2-(4-((2-(2,6-dioxopiperidin-3-yl)-1-oxoisoindolin-5-yl)methyl)piperazin-1-yl)ethoxy)phenyl)amino)-5-methylpyrimidin-4-yl)amino)benzenesulfonamide (GT-02746)

Referring to the method of Step 2 in Scheme 11 and the method of Example 1, the target compound (GT-02746) was prepared using N-(tert-butyl)-3-((5-methyl-2-((4-(2-(piperazine-1-yl)ethoxy)phenyl)amino)pyrimidin-4-yl)amino)benzenesulfonamide (GT-M-002) synthesized according to Intermediate Example 7, and 3-(5-(bromomethyl)-1-oxoisoindolin-2-yl)piperidine-2,6-dione. The target compound was obtained as a white solid (5 mg, yield 12%). ¹H NMR (500 MHz, DMSO) δ 11.03 (d, *J* = 10.2 Hz, 1H), 10.50 (s, 1H), 9.97 (s, 1H), 8.00 - 7.69 (m, 7H), 7.60 (dd, *J* = 20.0, 12.1 Hz, 2H), 7.31 (d, *J* = 9.0 Hz, 2H), 6.94 (d, *J* = 8.5 Hz, 2H), 5.18 - 5.10 (m, 1H), 4.54 - 4.32 (m, 5H), 3.46 (s, 11H), 2.98 - 2.89 (m, 1H), 2.61 (d, *J* = 17.0 Hz, 1H), 2.47 - 2.38 (m, 1H), 2.18 (s, 3H), 2.04 - 1.96 (m, 1H), 1.09 (s, 9H). LCMS (ESI) m/z: calcd for C₄₁H₅₀N₉O₆S⁺ [M+H]⁺: 796.36, found, 796.3.

### Example 25: preparation of N-(tert-butyl)-3-((2-((4-(1-((2-(2,6-dioxopiperidin-3-yl)-1-oxoisoindolin-5-yl)methyl)piperidin-4-yl)phenyl)amino)-5-methylpyrimidin-4-yl)amino)benzenesulfonamide (GT-02753)

Referring to the method of Step 2 in Scheme 11 and the method of Example 1, the target compound (GT-02753) was synthesized using N-(tert-butyl)-3-((5-methyl-2-((4-(piperidin-4-yl)phenyl)amino)pyrimidin-4-yl)amino)benzenesulfonamide (GT-M-003) prepared according to Intermediate Example 9, and 3-(5-(bromomethyl)-1-oxoisoindolin-2-yl)piperidine-2,6-dione. The target compound (GT-02753) was obtained as a white solid (5 mg, yield 13%). ¹H NMR (500 MHz, DMSO) δ 11.15 (s, 1H), 11.03 (s, 1H), 10.49 (s, 1H), 9.93 (s, 1H), 8.02 - 7.78 (m, 6H), 7.71 (d, *J* = 7.8 Hz, 1H), 7.66 - 7.51 (m, 2H), 7.36 (t, *J* = 12.9 Hz, 2H), 7.14 (d, *J* = 8.4 Hz, 2H), 5.16 (dd, *J* = 13.3, 5.0 Hz, 1H), 4.68 - 4.29 (m, 4H), 3.44 (d, *J* = 4.6 Hz, 2H), 3.06 (d, *J* = 11.0 Hz, 2H), 2.99 - 2.86 (m, 1H), 2.75 (t, *J* = 12.0 Hz, 1H), 2.62 (d, *J* = 17.3 Hz, 1H), 2.47 - 2.38 (m, 1H), 2.16 (d, *J* = 19.1 Hz, 3H), 2.15 - 1.98 (m, 3H), 1.91 (d, *J* = 12.7 Hz, 2H), 1.09 (d, *J* = 4.8 Hz, 9H). LCMS (ESI) m/z: calcd for C₄₀H₄₇N₈O₅S⁺ [M+H]⁺: 751.34, found, 751.3.

### Example 26: preparation of N-(tert-butyl)-3-((2-((4-(4-((2-(2,6-dioxopiperidin-3-yl)-1-oxoisoindolin-5-yl)methyl)piperazin-1-yl)phenyl)amino)-5-methylpyrimidin-4-yl)amino)benzenesulfonamide (GT-02755)

Referring to the method of Step 2 in Scheme 11 and the method of Example 1, the target compound (GT-02755) was prepared using N-(tert-butyl)-3-((5-methyl-2-((4-(piperazine-1-yl)phenyl)amino)pyrimidin-4-yl)amino)benzenesulfonamide (GT-M-004) synthesized according to Intermediate Example 10, and 3-(5-(bromomethyl)-1-oxoisoindolin-2-yl)piperidine-2,6-dione. The target compound (GT-02755) was obtained as a white solid (5 mg, yield 13%). ¹H NMR (500 MHz, DMSO) δ 11.47 (s, 1H), 11.03 (s, 1H), 10.33 (s, 1H), 9.88 (s, 1H), 7.96 (s, 1H), 7.93 (s, 1H), 7.90 (s, 1H), 7.85 (d, *J* = 7.8 Hz, 1H), 7.81 (d, *J* = 7.6 Hz, 1H), 7.69 (d, *J* = 7.8 Hz, 1H), 7.63 (s, 1H), 7.55 (t, *J* = 8.0 Hz, 1H), 7.27 (d, *J* = 8.8 Hz, 2H), 6.93 (d, *J* = 8.5 Hz, 2H), 5.16 (dd, *J* = 13.3, 5.0 Hz, 1H), 4.57 - 4.47 (m, 3H), 4.41 (t, *J* = 15.7 Hz, 1H), 3.75 (s, 2H), 3.60 (dtd, *J* = 13.2, 6.6, 4.1 Hz, 1H), 3.13 (ddd, *J* = 13.3, 10.4, 8.9 Hz, 5H), 2.98 - 2.86 (m, 1H), 2.67 - 2.59 (m, 1H), 2.47 - 2.39 (m, 1H), 2.17 (s, 3H), 2.07 - 2.00 (m, 1H), 1.09 (s, 9H). LCMS (ESI) m/z: calcd for C₃₉H₄₆N₉O₅S⁺ [M+H]⁺: 752.33, found, 752.4.

### Example 27: preparation of N-(tert-butyl)-3-((2-((6-(4-((2-(2,6-dioxopiperidin-3-yl)-1-oxoisoindolin-5-yl)methyl)piperazin-1-yl)pyridazin-3-yl)amino)-5-methylpyrimidin-4-yl)amino)benzenesulfonamide (GT-02757)

Referring to the method of Step 2 in Scheme 11 and the method of Example 1, the target compound (GT-02757) was prepared by using N-(tert-butyl)-3-((5-methyl-2-((6-(piperazin-1-yl)pyridazin-3-yl)amino)pyrimidin-4-yl)amino)benzenesulfonamide (GT-M-17) prepared by referring to Intermediate Example 8 and 3-(5-(bromomethyl)-1-oxoisoindolin-2-yl)piperidine-2,6-dione. The target compound (GT-02757) was obtained as a white solid (5 mg, yield 13%). ¹H NMR (500 MHz, DMSO) δ 12.07 (s, 1H), 11.03 (s, 1H), 9.88 (s, 1H), 9.48 (s, 3H), 8.16 (d, *J* = 6.5 Hz, 1H), 8.08 (t, *J* = 9.2 Hz, 2H), 7.95 (d, *J* = 11.4 Hz, 1H), 7.82 (s, 2H), 7.73 (s, 1H), 7.70 (d, *J* = 7.8 Hz, 2H), 7.64 (t, *J* = 7.9 Hz, 1H), 5.15 (dd, *J* = 13.2, 5.1 Hz, 1H), 4.61 - 4.45 (m, 3H), 4.36 (dd, *J* = 24.5, 14.7 Hz, 3H), 3.50 (dd, *J* = 34.2, 21.3 Hz, 4H), 3.19 (s, 2H), 2.97 - 2.86 (m, 1H), 2.62 (d, *J* = 17.5 Hz, 1H), 2.46 - 2.37 (m, 1H), 2.26 (s, 3H), 2.06 - 1.97 (m, 1H), 1.13 (s, 9H). LCMS (ESI) m/z: calcd for C₃₇H₄₄N₁₁O₅S⁺ [M+H]⁺: 754.32, found, 754.3.

### Example 28: preparation of 4-(4-((2-(2,6-dioxopiperidin-3-yl)-1-oxoisoindolin-5-yl)methyl)piperazin-1-yl)-N-(5-((R)-2-methoxy-2-phenylacetyl)-1,4,5,6-tetrahydropyrrolo[3,4-c]pyrazol-3-yl)benzamide (GT-02956)

Referring to the method of Step 2 in Scheme 11 and the method of Example 1, the target compound (GT-02956) was prepared using (R)-N-(5-(2-methoxy-2-phenylacetyl)-1,4,5,6-tetrahydropyrrolo[3,4-c]pyrazol-3-yl)-4-(piperazin-1-yl)benzamide (GT-M-08) prepared from Intermediate Example 24 and 3-(5-(bromomethyl)-1-oxoisoindolin-2-yl)piperidine-2,6-dione. The target compound (GT-02956) was obtained as a white solid (5 mg, yield 69%). ¹H NMR (400 MHz, DMSO) δ 11.19 (d, *J* = 27.9 Hz, 1H), 11.02 (s, 1H), 10.72 (d, *J* = 9.0 Hz, 1H), 7.94 (dd, *J* = 8.9, 2.9 Hz, 2H), 7.88 (d, *J* = 4.0 Hz, 1H), 7.85 (d, *J* = 7.8 Hz, 1H), 7.80 - 7.73 (m, 1H), 7.46 - 7.32 (m, 5H), 7.05 (dd, *J* = 9.0, 2.4 Hz, 2H), 5.15 (dd, *J* = 13.2, 5.0 Hz, 1H), 5.10 (d, *J* = 10.3 Hz, 1H), 4.80 (dd, *J* = 18.8, 13.2 Hz, 1H), 4.61 - 4.35 (m, 8H), 4.03 (d, *J* = 13.0 Hz, 2H), 3.35 - 3.30 (m, 8H), 2.98 - 2.88 (m, 1H), 2.65 - 2.56 (m, 1H), 2.43 (dd, *J* = 13.1, 4.4 Hz, 1H), 2.07 - 1.95 (m, 1H). LCMS (ESI) m/z: calcd for C₃₉H₄₁N₈O₆⁺ [M+H]⁺: 717.31, found, 717.3.

### Example 29: preparation of 3-(5-((4-(2-((S)-4-(4-chlorophenyl)-2,3,9-trimethyl-6H-thieno[3,2-f][1,2,4]triazolo[4,3-a][1,4]diazepin-6-yl)acetyl)piperazin-1-yl)methyl)-1-oxoisoindolin-2-yl)piperidine-2,6-dione (GT-02967)

Referring to the method of Step 2 in Scheme 11 and the method of Example 1, the target compound (GT-02967) was prepared using (S)-2-(4-(4-chlorophenyl)-2,3,9-trimethyl-6H-thieno[3,2-f][1,2,4]triazolo[4,3-a][1,4]diazepin-6-yl)-1-(piperazin-1-yl)ethan-1-one prepared from Intermediate Example 4 and 3-(5-(bromomethyl)-1-oxoisoindolin-2-yl)piperidine-2,6-dione. The target compound (GT-02967) was obtained as a white solid (72 mg, yield 22%). ¹H NMR (400 MHz, DMSO) δ 11.45 - 11.12 (m, 1H), 11.01 (s, 1H), 7.92 - 7.80 (m, 2H), 7.79 - 7.70 (m, 1H), 7.50 (d, *J* = 8.5 Hz, 2H), 7.43 (d, *J* = 8.3 Hz, 2H), 5.15 (dd, *J* = 13.2, 5.0 Hz, 1H), 4.69 - 4.22 (m, 8H), 3.77 - 3.60 (m, 3H), 3.20 - 3.13 (m, 1H), 3.02 - 2.84 (m, 2H), 2.68 - 2.56 (m, 5H), 2.47 - 2.44 (m, 1H), 2.42 (s, 3H), 2.06 - 1.96 (m, 1H), 1.63 (s, 3H). LCMS (ESI) m/z: calcd for C₃₇H₃₈ClN₈O₄S⁺ [M+H]⁺: 725.24, found, 725.2.

### Example 30: preparation of 4-((2,4-dichloro-5-methoxyphenyl)amino)-7-(3-(4-((2-(2,6-dioxopiperidin-3-yl)-1-oxoisoindolin-5-yl)methyl)piperazin-1-yl)propoxy)-6-methoxyquinoline-3-carbonitrile (GT-02968)

Referring to the method of Step 2 in Scheme 11 and the method of Example 1, the target compound (GT-02968) was prepared using 4-((2,4-dichloro-5-methoxyphenyl)amino)-6-methoxy-7-(3-(piperazin-1-yl)propoxy)quinoline-3-carbonitrile (CAS NO.: 380843-81-2) and 3-(5-(bromomethyl)-1-oxoisoindolin-2-yl)piperidine-2,6-dione. The target compound (GT-02968) was obtained as a white solid (5 mg, yield 20%). ¹H NMR (400 MHz, MeOD) δ 8.87 (s, 1H), 8.02 (s, 1H), 7.88 (d, *J* = 7.9 Hz, 1H), 7.83 (s, 1H), 7.75 - 7.69 (m, 1H), 7.68 (d, *J* = 9.2 Hz, 1H), 7.40 (d, *J* = 7.2 Hz, 2H), 5.17 (dd, *J* = 13.3, 5.2 Hz, 1H), 4.56 (d, *J* = 8.7 Hz, 2H), 4.46 (t, *J* = 5.4 Hz, 2H), 4.37 (s, 2H), 4.08 (s, 3H), 3.94 (s, 3H), 3.58 - 3.39 (m, 6H), 2.98 - 2.86 (m, 2H), 2.84 - 2.75 (m, 2H), 2.64 - 2.41 (m, 4H), 2.23 - 2.12 (m, 2H). LCMS (ESI) m/z: calcd for C₃₉H₄₀Cl₂N₇O₆⁺ [M+H]⁺: 772.24, found, 772.2.

### Example 31: preparation of 4-((4-((2-(2,6-dioxopiperidin-3-yl)-1-oxoisoindolin-5-yl)methyl)piperazin-1-yl)methyl)-N-(4-methyl-3-((4-(pyridin-3-yl)pyrimidin-2-yl)amino)phenyl)benzamide (GT-02969)

Referring to the method of Step 2 in Scheme 11 and the method of Example 1, the target compound (GT-02969) was prepared using N-(4-methyl-3-((4-(pyridin-3-yl)pyrimidin-2-yl)amino)phenyl)-4-(piperazin-1-ylmethyl)benzamide (CAS NO.: 404844-02-6) and 3-(5-(bromomethyl)-1-oxoisoindolin-2-yl)piperidine-2,6-dione. The target compound (GT-02969) was obtained as a white solid (5 mg, yield 30%). ¹H NMR (500 MHz, DMSO) δ 11.02 (s, 1H), 10.37 (s, 1H), 9.48 (d, *J* = 1.6 Hz, 1H), 9.21 (s, 1H), 9.02 (d, *J* = 8.0 Hz, 1H), 8.93 (d, *J* = 4.5 Hz, 1H), 8.62 (d, *J* = 5.1 Hz, 1H), 8.16 (s, 1H), 8.06 (d, *J* = 8.3 Hz, 2H), 8.02 - 7.95 (m, 1H), 7.92 (s, 1H), 7.86 - 7.73 (m, 4H), 7.59 (d, *J* = 5.2 Hz, 1H), 7.48 (dd, *J* = 8.2, 2.0 Hz, 1H), 7.23 (d, *J* = 8.6 Hz, 1H), 5.14 (dd, *J* = 13.3, 5.1 Hz, 1H), 4.58 - 4.33 (m, 6H), 3.52 (s, 8H), 2.97 - 2.87 (m, 1H), 2.62 (t, *J* = 12.8 Hz, 1H), 2.48 - 2.36 (m, 1H), 2.24 (s, 3H), 2.05 - 1.95 (m, 1H). LCMS (ESI) m/z: calcd for C₄₂H₄₂N₉O₄⁺ [M+H]⁺: 736.34, found, 736.3.

### Example 32: preparation of (Z)-3-(5-(((2-(4-(4-chloro-1,2-diphenylbut-1-en-1-yl)phenoxy)ethyl)(methyl)amino)methyl)-1-oxoisoindolin-2-yl)piperidine-2,6-dione (GT-02970)

Referring to the method of Step 2 in Scheme 11 and the method of Example 1, the target compound (GT-02970) was prepared using (Z)-2-(4-(4-chloro-1,2-diphenylbut-1-en-1-yl)phenoxy)-N-methylethan-1-amine (CAS NO.: 110503-61-2) and 3-(5-(bromomethyl)-1-oxoisoindolin-2-yl)piperidine-2,6-dione. The target compound (GT-02970) was obtained as a white solid (5 mg, yield 28%). ¹H NMR (400 MHz, MeOD) δ 7.90 (d, *J* = 7.8 Hz, 1H), 7.73 (s, 1H), 7.65 (d, *J* = 8.2 Hz, 1H), 7.42 - 7.34 (m, 2H), 7.34 - 7.27 (m, 3H), 7.23 - 7.10 (m, 5H), 6.87 (d, *J* = 8.7 Hz, 2H), 6.68 (d, *J* = 8.8 Hz, 2H), 5.18 (dd, *J* = 13.3, 5.2 Hz, 1H), 4.67 - 4.45 (m, 4H), 4.32 - 4.21 (m, 2H), 3.63 - 3.46 (m, 2H), 3.40 (t, *J* = 7.3 Hz, 2H), 3.00 - 2.86 (m, 6H), 2.83 - 2.75 (m, 1H), 2.50 (qd, *J* = 13.2, 4.6 Hz, 1H), 2.24 - 2.13 (m, 1H). LCMS (ESI) m/z: calcd for C₃₉H₃₈ClN₃O₄⁺ [M+H]⁺: 648.26, found, 648.3.

### Example 33: preparation of (Z)-3-(5-(((2-(4-(1,2-diphenylbut-1-en-1-yl)phenoxy)ethyl)(methyl)amino)methyl)-1-oxoisoindolin-2-yl)piperidine-2,6-dione (GT-02971)

Referring to the method of Step 2 in Scheme 11 and the method of Example 1, the target compound (GT-02971) was prepared using (Z)-2-(4-(1,2-diphenylbut-1-en-1-yl)phenoxy)-N-methylethan-1-amine (CAS NO.: 31750-48-8) and 3-(5-(bromomethyl)-1-oxoisoindolin-2-yl)piperidine-2,6-dione. The target compound (GT-02971) was obtained as a white solid (5 mg, yield 27%). ¹H NMR (400 MHz, MeOD) δ 7.90 (d, *J* = 7.8 Hz, 1H), 7.75 (s, 1H), 7.66 (d, *J* = 7.9 Hz, 1H), 7.39 - 7.32 (m, 2H), 7.30 - 7.24 (m, 1H), 7.23 - 7.19 (m, 2H), 7.18 - 7.05 (m, 5H), 6.86 - 6.80 (m, 2H), 6.71 - 6.63 (m, 2H), 5.17 (dd, *J* = 13.3, 5.2 Hz, 1H), 4.55 (q, *J* = 17.4 Hz, 2H), 4.27 (t, *J* = 4.7 Hz, 1H), 4.17 - 4.10 (m, 1H), 3.56 (s, 1H), 3.41 - 3.31 (m, 3H), 2.98 - 2.86 (m, 3H), 2.83 - 2.76 (m, 1H), 2.74 (s, 1H), 2.57 - 2.49 (m, 1H), 2.45 (q,*J* = 7.5 Hz, 2H), 2.23 - 2.12 (m, 1H), 0.90 (t, *J* = 7.4 Hz, 3H). LCMS (ESI) m/z: calcd for C₃₉H₄₀N₃O₄⁺ [M+H]⁺: 614.30, found, 614.3.

### Example 34: preparation of 8-(4-(4-((2-(2,6-dioxopiperidin-3-yl)-1-oxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile (GT-02972)

Referring to the method of Step 2 in Scheme 11 and the method of Example 1, the target compound (GT-02972) was prepared using 9-ethyl-6,6-dimethyl-11-oxo-8-(4-(piperazin-1-yl)piperidin-1-yl)-6,11-dihydro-5H-benzo[b]carbazole-3-carbonitrile (GT-D-113) prepared from Intermediate Example 45 and 3-(5-(bromomethyl)-1-oxoisoindolin-2-yl)piperidine-2,6-dione. The target compound (GT-02972) was obtained as a white solid (5 mg, yield 20%). ¹H NMR (400 MHz, MeOD) δ 8.41 (d, *J* = 8.3 Hz, 1H), 8.20 (s, 1H), 7.90 - 7.81 (m, 2H), 7.69 (s, 1H), 7.62 (d, *J* = 7.5 Hz, 1H), 7.55 (d, *J* = 8.2 Hz, 1H), 7.39 (s, 1H), 5.18 (dd, *J* = 13.1, 5.3 Hz, 1H), 4.54 - 4.47 (m, 2H), 4.00 (s, 2H), 3.49 - 3.36 (m, 6H), 3.00 - 2.88 (m, 4H), 2.86 - 2.73 (m, 5H), 2.61 - 2.43 (m, 2H), 2.38 - 2.13 (m, 4H), 2.03 - 1.93 (m, 2H), 1.80 (s, 6H), 1.34 (t, *J* = 7.5 Hz, 3H). LCMS (ESI) m/z: calcd for C₄₄H₄₈N₇O₄⁺ [M+H]⁺: 738.38, found, 738.3.

### Example 35: preparation of 3-(5-((4-(4-((5-chloro-4-((2-(isopropylsulfonyl)phenyl)amino)pyrimidin-2-yl)amino)-5-isopropoxy-2-methylphenyl)piperidin-1-yl)methyl)-1-oxoisoindolin-2-yl)piperidine-2,6-dione (GT-02973)

Referring to the method of Step 2 in Scheme 11 and the method of Example 1, the target compound (GT-02973) was prepared using 5-chloro-N²-(2-isopropoxy-5-methyl-4-(piperidin-4-yl)phenyl)-N⁴-(2-(isopropylsulfonyl)phenyl)pyrimidine-2,4-diamine prepared by referring to the method of Scheme 3 and Intermediate Example 9, and 3-(5-(bromomethyl)-1-oxoisoindolin-2-yl)piperidine-2,6-dione. The target compound (GT-02973) was obtained as a white solid (5 mg, yield 65%). ¹H NMR (400 MHz, MeOD) δ 11.02 (s, 1H), 10.92 (s, 1H), 9.61 (s, 1H), 8.45 - 8.34 (m, 2H), 8.30 (s, 1H), 7.91 (s, 1H), 7.88 - 7.82 (m, 2H), 7.79 (d, *J* = 8.0 Hz, 1H), 7.63 (t, *J* = 7.8 Hz, 1H), 7.45 (s, 1H), 7.38 (t, *J* = 7.7 Hz, 1H), 6.82 (s, 1H), 5.15 (dd, *J* = 13.2, 5.1 Hz, 1H), 4.58 - 4.35 (m, 5H), 3.42 - 3.37 (m, 3H), 3.16 - 3.02 (m, 2H), 2.99 - 2.87 (m, 2H), 2.66 - 2.57 (m, 1H), 2.46 - 2.37 (m, 1H), 2.20 - 2.08 (m, 5H), 2.06 - 1.98 (m, 1H), 1.89 - 1.81 (m, 2H), 1.22 (d, *J* = 6.0 Hz, 6H), 1.14 (d, *J* = 6.8 Hz, 6H). LCMS (ESI) m/z: calcd for C₄₂H₄₉ClN₇O₆S⁺ [M+H]⁺: 814.31, found, 814.3.

### Example 36: preparation of 3-(5-((4-(4-(6-amino-5-((R)-1-(2,6-dichloro-3-fluorophenyl)ethoxy)pyridin-3-yl)-1H-pyrazol-1-yl)piperidin-1-yl)methyl)-1-oxoisoindolin-2-yl)piperidine-2,6-dione (GT-02974)

Referring to the method of Step 2 in Scheme 11 and the method of Example 1, the target compound (GT-02974) was prepared using (R)-3-(1-(2,6-dichloro-3-fluorophenyl)ethoxy)-5-(1-(piperidin-4-yl)-1H-pyrazol-4-yl)pyridin-2-amine (CAS NO.: 877399-52-5) and 3-(5-(bromomethyl)-1-oxoisoindolin-2-yl)piperidine-2,6-dione. The target compound (GT-02974) was obtained as a white solid (8.4 mg, yield 49%). ¹H NMR (400 MHz, MeOD) δ 8.01 (s, 1H), 7.92 (d, *J* = 7.9 Hz, 1H), 7.88 (s, 1H), 7.76 (d, *J* = 7.7 Hz, 1H), 7.65 (d, *J* = 7.3 Hz, 2H), 7.51 (dd, *J* = 9.0, 4.8 Hz, 1H), 7.29 (t, *J* = 8.6 Hz, 1H), 7.15 (s, 1H), 6.35 (q, *J* = 6.5 Hz, 1H), 5.18 (dd, *J* = 13.3, 5.1 Hz, 1H), 4.61 - 4.56 (m, 2H), 4.55 (s, 2H), 3.69 - 3.61 (m, 2H), 3.42 - 3.33 (m, 2H), 2.97 - 2.87 (m, 1H), 2.84 - 2.75 (m, 1H), 2.59 - 2.47 (m, 1H), 2.46 - 2.32 (m, 4H), 2.24 - 2.14 (m, 1H), 1.95 (d, *J* = 6.6 Hz, 3H). LCMS (ESI) m/z: calcd for C₃₅H₃₅Cl₂FN₇O₄⁺ [M+H]⁺: 706.21, found, 706.2.

### Example 37: preparation of 2-((2-((4-(4-((2-(2,6-dioxopiperidin-3-yl)-1-oxoisoindolin-5-yl)methyl)piperazin-1-yl)-2-methoxyphenyl)amino)-5-(trifluoromethyl)pyridin-4-yl)amino)-N-methylbenzamide (GT-03014)

Referring to the method of Step 2 in Scheme 11 and the method of Example 1, the target compound (GT-03014) was prepared using 2-((2-((2-methoxy-4-(piperazin-1-yl)phenyl)amino)-5-(trifluoromethyl)pyridin-4-yl)amino)-N-methylbenzamide (GT-M-10) prepared from Intermediate Example 25 and 3-(5-(bromomethyl)-1-oxoisoindolin-2-yl)piperidine-2,6-dione. The target compound (GT-03014) was obtained as a white solid (5 mg, yield 28%). ¹H NMR (400 MHz, MeOD) δ 8.01 (s, 1H), 7.93 (d, J = 7.8 Hz, 1H), 7.89 (s, 1H), 7.77 (d, J = 7.8 Hz, 1H), 7.71 (d, J = 7.7 Hz, 1H), 7.57 (d, J = 3.8 Hz, 2H), 7.38 - 7.31 (m, 1H), 7.18 (d, J = 8.6 Hz, 1H), 6.76 (d, J = 2.4 Hz, 1H), 6.65 (dd, J = 8.7, 2.5 Hz, 1H), 6.47 (s, 1H), 5.19 (dd, J = 13.3, 5.2 Hz, 1H), 4.66 - 4.52 (m, 4H), 3.85 (s, 3H), 3.60 - 3.31 (m, 8H), 2.98 - 2.89 (m, 1H), 2.88 (s, 3H), 2.84 - 2.75 (m, 1H), 2.53 (qd, J = 13.2, 4.7 Hz, 1H), 2.24 - 2.14 (m, 1H). LCMS (ESI) m/z: calcd for C₃₉H₄₀F₃N₈O₅⁺ [M+H]⁺: 757.31, found, 757.3.

### Example 38: preparation of 3-(5-(((1-(4-((5-chloro-4-((2-(dimethylphosphoryl)phenyl)amino)pyrimidin-2-yl)amino)-3-methoxyphenyl)piperidin-4-yl)amino)methyl)-1-oxoisoindolin-2-yl)piperidine-2,6-dione (GT-03015)

Referring to the method of Step 2 in Scheme 11 and the method of Example 1, the target compound (GT-03015) was prepared using (2-((2-((4-(4-aminopiperidin-1-yl)-2-methoxyphenyl)amino)-5-chloropyrimidin-4-yl)amino)phenyl)dimethylphosphine oxide prepared by referring to the method of Scheme 3 and Intermediate Example 9, and 3-(5-(bromomethyl)-1-oxoisoindolin-2-yl)piperidine-2,6-dione. The target compound (GT-03015) was obtained as a white solid (5 mg, yield 30%). ¹H NMR (400 MHz, MeOD) δ 11.75 (s, 1H), 11.00 (s, 1H), 9.45 (s, 2H), 8.43 (s, 1H), 8.20 (s, 1H), 7.87 - 7.80 (m, 2H), 7.75 (d, *J* = 7.8 Hz, 1H), 7.61 (dd, *J* = 13.8, 7.6 Hz, 1H), 7.51 - 7.36 (m, 2H), 7.22 (t, *J* = 7.3 Hz, 1H), 6.93 - 6.79 (m, 1H), 6.76 - 6.59 (m, 1H), 5.13 (dd, *J* = 13.3, 5.1 Hz, 1H), 4.51 (d, *J* = 17.7 Hz, 1H), 4.43 - 4.29 (m, 3H), 3.89 - 3.82 (m, 2H), 3.80 (s, 3H), 3.08 - 2.84 (m, 4H), 2.65 - 2.59 (m, 1H), 2.30 - 2.20 (m, 2H), 2.07 - 1.84 (m, 3H), 1.81 (s, 3H), 1.77 (s, 3H), 1.32 - 1.20 (m, 1H). LCMS (ESI) m/z: calcd for C₃₈H₄₃ClN₈O₅P⁺ [M+H]⁺: 757.28, found, 757.3.

### Example 39: preparation of 2-((2-((4-(4-(((2-(2,6-dioxopiperidin-3-yl)-1-oxoisoindolin-5-yl)methyl)amino)piperidin-1-yl)-2-methoxyphenyl)amino)-5-(trifluoromethyl)pyridin-4-yl)amino)-N-methylbenzamide (GT-03213)

Referring to the method of Step 2 in Scheme 11 and the method of Example 1, the target compound (GT-03213) was prepared using 2-((2-((4-(4-aminopiperidin-1-yl)-2-methoxyphenyl)amino)-5-(trifluoromethyl)pyridin-4-yl)amino)-N-methylbenzamide (GT-M-11) prepared from Intermediate Example 26 and 3-(5-(bromomethyl)-1-oxoisoindolin-2-yl)piperidine-2,6-dione. The target compound (GT-03213) was obtained as a white solid (5 mg, yield 33%). ¹H NMR (400 MHz, MeOD) δ 8.02 (s, 1H), 7.90 (d, *J* = 7.9 Hz, 1H), 7.83 (s, 1H), 7.73 (d, *J* = 7.0 Hz, 2H), 7.60 (d, *J* = 3.2 Hz, 2H), 7.40 - 7.34 (m, 1H), 7.21 (d, *J* = 8.6 Hz, 1H), 6.96 (s, 1H), 6.84 (d, *J* = 8.0 Hz, 1H), 6.49 (s, 1H), 5.18 (dd, *J* = 13.3, 5.1 Hz, 1H), 4.65 - 4.56 (m, 2H), 4.46 (s, 2H), 3.99 - 3.90 (m, 3H), 3.88 (s, 3H), 3.62 - 3.46 (m, 1H), 3.19 - 3.08 (m, 2H), 2.98 - 2.91 (m, 1H), 2.89 (s, 3H), 2.83 - 2.75 (m, 1H), 2.53 (qd, *J* = 13.2, 4.7 Hz, 2H), 2.25 - 2.15 (m, 2H), 2.09 - 1.93 (m, 2H). LCMS (ESI) m/z: calcd for C₄₀H₄₂F₃N₈O₅⁺ [M+H]⁺: 771.32, found, 771.2.

### Example 40: preparation of 3-(5-(((2-(4-(1,2-bis(4-hydroxyphenyl)but-1-en-1-yl)phenoxy)ethyl)amino)methyl)-1-oxoisoindolin-2-yl)piperidine-2,6-dione (GT-03214)

Referring to the method of Step 2 in Scheme 11 and the method of Example 1, the target compound (GT-03214) was prepared using 4,4'-(1-(4-(2-aminoethoxy)phenyl)but-1-ene-1,2-diyl)diphenol (CAS NO.: 1946764-65-3) and 3-(5-(bromomethyl)-1-oxoisoindolin-2-yl)piperidine-2,6-dione. The target compound (GT-03214) was obtained as a white solid (5 mg, yield 17%). ¹H NMR (500 MHz, DMSO) δ 11.01 (s, 1H), 9.23 (d, *J* = 1.5 Hz, 1H), 7.80 (s, 3H), 7.09 (d, *J* = 8.8 Hz, 1H), 6.95 (dd, *J* = 15.4, 8.6 Hz, 2H), 6.90 - 6.83 (m, 2H), 6.74 (t, *J* = 7.1 Hz, 2H), 6.66 (d, *J* = 9.0 Hz, 1H), 6.61 - 6.49 (m, 3H), 6.42 (d, *J* = 8.7 Hz, 1H), 5.14 (d, *J* = 13.4 Hz, 1H), 4.46 (s, 1H), 4.24 (d, *J* = 127.4 Hz, 5H), 3.27 - 3.15 (m, 2H), 2.93 (s, 1H), 2.60 (s, 1H), 2.37 - 2.30 (m, 3H), 2.03 (s, 1H), 0.84 (t, *J* = 7.4 Hz, 3H). LCMS (ESI) m/z: calcd for C₃₈H₃₈N₃O₆⁺ [M+H]⁺: 632.28, found, 632.3.

### Example 41: preparation of N-(2-chloro-6-methylphenyl)-2-((6-(4-((2-(2,6-dioxopiperidin-3-yl)-1-oxoisoindolin-5-yl)methyl)piperazin-1-yl)-2-methylpyrimidin-4-yl)amino)thiazole-5-carboxamide (GT-03215)

Referring to the method of Step 2 in Scheme 11 and the method of Example 1, the target compound (GT-03215) was prepared using N-(2-chloro-6-methylphenyl)-2-((2-methyl-6-(piperazin-1-yl)pyrimidin-4-yl)amino)thiazole-5-carboxamide (CAS NO.: 910297-51-7) and 3-(5-(bromomethyl)-1-oxoisoindolin-2-yl)piperidine-2,6-dione. The target compound (GT-03215) was obtained as a white solid (5 mg, yield 25%). ¹H NMR (400 MHz, MeOD) δ 8.23 (s, 1H), 7.94 (d, *J* = 7.9 Hz, 1H), 7.87 (s, 1H), 7.75 (d, *J* = 7.4 Hz, 1H), 7.39 - 7.34 (m, 1H), 7.28 - 7.24 (m, 2H), 6.45 (s, 1H), 5.19 (dd, *J* = 13.3, 5.2 Hz, 1H), 4.64 - 4.56 (m, 4H), 3.97 - 3.83 (m, 2H), 3.78 - 3.42 (m, 6H), 2.94 - 2.87 (m, 1H), 2.83 - 2.77 (m, 1H), 2.62 (s, 3H), 2.56 - 2.46 (m, 1H), 2.31 (s, 3H), 2.24 - 2.14 (m, 1H). LCMS (ESI) m/z: calcd for C₃₄H₃₅ClN₉O₄⁺ [M+H]⁺: 700.22, found, 700.2.

### Example 42: preparation of 3-(5-(((3S,4S)-4-(4-amino-3-(4-phenoxyphenyl)-1H-pyrazolo[3,4-d]pyrimidin-1-yl)-3-fluoropiperidin-1-yl)methyl)-1-oxoisoindolin-2-yl)piperidine-2,6-dione (GT-03264)

Referring to the method of Step 2 in Scheme 11 and the method of Example 1, the target compound (GT-03264) was prepared using 1-((3S,4S)-3-fluoropiperidin-4-yl)-3-(4-phenoxyphenyl)-1H-pyrazolo[3,4-d]pyrimidin-4-amine and 3-(5-(bromomethyl)-1-oxoisoindolin-2-yl)piperidine-2,6-dione. The target compound (GT-03264) was obtained as a white solid (63.7 mg, yield 18%). ¹H NMR (400 MHz, MeOD) δ 8.41 (s, 1H), 7.93 (d, *J* = 7.8 Hz, 1H), 7.83 (s, 1H), 7.74 (d, *J* = 7.8 Hz, 1H), 7.70 - 7.54 (m, 2H), 7.43 (t, *J* = 8.0 Hz, 2H), 7.24 - 7.08 (m, 5H), 5.50 - 5.25 (m, 2H), 5.17 (dd, *J* = 13.4, 5.1 Hz, 1H), 4.68 - 4.47 (m, 4H), 3.94 - 3.81 (m, 1H), 3.79 - 3.61 (m, 1H), 3.57 - 3.33 (m, 2H), 2.95 - 2.85 (m, 1H), 2.82 - 2.72 (m, 1H), 2.66 - 2.42 (m, 3H), 2.23 - 2.13 (m, 1H). LCMS (ESI) m/z: calcd for C₃₄H₃₅ClN₉O₄ ⁺ [M+H]⁺: 661.27, found, 661.3.

### Example 43: preparation of (E)-N-(4-((3-chloro-4-fluorophenyl)amino)-7-methoxyquinazolin-6-yl)-4-(4-((2-(2,6-dioxopiperidin-3-yl)-1-oxoisoindolin-5-yl)methyl)piperazin-1-yl)but-2-enamide (GT-03265)

Referring to the method of Step 2 in Scheme 11 and the method of Example 1, the target compound (GT-03265) was prepared using (E)-N-(4-((3-chloro-4-fluorophenyl)amino)-7-methoxyquinazolin-6-yl)-4-(piperazin-1-yl)but-2-enamide (CAS NO.: 2600734-22-1) and 3-(5-(bromomethyl)-1-oxoisoindolin-2-yl)piperidine-2,6-dione. The target compound (GT-03265) was obtained as a white solid (9.6 mg, yield 57%). ¹H NMR (400 MHz, MeOD) δ 9.31 - 9.20 (m, 1H), 8.75 (s, 1H), 7.93 (dd, *J* = 6.6, 2.6 Hz, 1H), 7.88 (d, *J* = 7.8 Hz, 1H), 7.80 (s, 1H), 7.70 (d, *J* = 7.8 Hz, 1H), 7.67 - 7.62 (m, 1H), 7.38 (t, *J* = 8.9 Hz, 1H), 7.33 (s, 1H), 7.08 - 6.99 (m, 1H), 6.80 (d, *J* = 15.3 Hz, 1H), 5.18 (dd, *J* = 13.3, 5.1 Hz, 1H), 4.55 (q, *J* = 17.3 Hz, 2H), 4.34 (s, 2H), 4.18 (s, 3H), 3.88 (s, 2H), 3.51 - 3.31 (m, 8H), 2.98 - 2.86 (m, 1H), 2.83 - 2.74 (m, 1H), 2.51 (qd, *J* = 13.2, 4.6 Hz, 1H), 2.22 - 2.13 (m, 1H). LCMS (ESI) m/z: calcd for C₃₇H₃₇ClFN₈O₅ ⁺ [M+H]⁺: 727.26, found, 727.2.

### Example 44: preparation of 2-((2-((4-(4-(4-((2-(2,6-dioxopiperidin-3-yl)-1-oxoisoindolin-5-yl)methyl)piperazin-1-yl)piperidin-1-yl)-2-methoxyphenyl)amino)-5-(trifluoromethyl)pyridin-4-yl)amino)-N-methylbenzamide (GT-03266)

Referring to the method of Step 2 in Scheme 11 and the method of Example 1, 2-((2-((2-methoxy-4-(4-(piperazin-1-yl)piperidin-1-yl)phenyl)amino)-5-(trifluoromethyl)pyridin-4-yl)amino)-N-methylbenzamide (GT-M-12) prepared from Intermediate Example 27 and 3-(5-(bromomethyl)-1-oxoisoindolin-2-yl)piperidine-2,6-dione were used to prepare the target compound (GT-03266) (white solid, 5 mg, yield 33%). ¹H NMR (400 MHz, MeOD) δ 8.01 (s, 1H), 7.90 (d, *J* = 7.8 Hz, 1H), 7.85 (s, 1H), 7.79 - 7.69 (m, 2H), 7.64 - 7.56 (m, 2H), 7.42 - 7.31 (m, 1H), 7.24 - 7.13 (m, 1H), 6.88 (s, 1H), 6.77 (d, *J* = 7.5 Hz, 1H), 6.48 (s, 1H), 5.18 (dd, *J* = 13.3, 5.1 Hz, 1H), 4.57 (q, *J* = 17.4 Hz, 2H), 4.44 (s, 2H), 4.01 - 3.91 (m, 2H), 3.87 (s, 3H), 3.78 - 3.38 (m, 8H), 3.11 - 2.99 (m, 2H), 2.97 - 2.90 (m, 1H), 2.89 (s, 3H), 2.84 - 2.73 (m, 1H), 2.52 (qd, *J* = 13.1, 4.6 Hz, 1H), 2.36 - 2.25 (m, 2H), 2.24 - 2.12 (m, 1H), 2.06 - 1.92 (m, 2H). LCMS (ESI) m/z: calcd for C₄₄H₄₉F₃N₉O₅S ⁺ [M+H]⁺: 840.38, found, 840.3.

### Example 45: preparation of 2-((S)-4-(4-chlorophenyl)-2,3,9-trimethyl-6H-thieno[3,2-f][1,2,4]triazolo[4,3-a][1,4]diazepin-6-yl)-N-(2-(4-((2-(2,6-dioxopiperidin-3-yl)-1-oxoisoindolin-5-yl)methyl)piperazin-1-yl)ethyl)acetamide (GT-03296)

Referring to the method of Step 2 in Scheme 11 and the method of Example 1, (S)-2-(4-(4-chlorophenyl)-2,3,9-trimethyl-6H-thieno[3,2-f][1,2,4]triazolo[4,3-a][1,4]diazepin-6-yl)-N-(2-(piperazin-1-yl)ethyl)acetamide prepared from Intermediate Example 3 and 3-(5-(bromomethyl)-1-oxoisoindolin-2-yl)piperidine-2,6-dione were used to prepare the target compound (GT-03296) (white solid, 5 mg, yield 33%). ¹H NMR (400 MHz, MeOD) δ 7.89 (d, *J* = 7.8 Hz, 1H), 7.83 (s, 1H), 7.71 (d, *J* = 7.8 Hz, 1H), 7.57 - 7.46 (m, 4H), 5.17 (dd, *J* = 13.2, 5.0 Hz, 1H), 4.56 (q, *J* = 17.2 Hz, 2H), 4.43 (s, 2H), 3.85 - 3.46 (m, 13H), 3.38 (t, *J* = 5.6 Hz, 2H), 2.95 - 2.75 (m, 5H), 2.58 - 2.52 (m, 1H), 2.49 (s, 3H), 2.23 -2.13 (m, 1H), 1.72 (s, 3H). LCMS (ESI) m/z: calcd for C₃₉H₄₃ClN₉O₄S ⁺ [M+H]⁺: 768.28, found, 768.3.

### Example 46: preparation of 3-(5-(((1-(6-amino-5-(2,3-dichlorophenyl)pyrazin-2-yl)-4-methylpiperidin-4-yl)amino)methyl)-1-oxoisoindolin-2-yl)piperidine-2,6-dione (GT-03320)

Referring to the method of Step 2 in Scheme 11 and the method of Example 1, 6-(4-amino-4-methylpiperidin-1-yl)-3-(2,3-dichlorophenyl)pyrazin-2-amine (CAS NO.: 1801747-42-1) and 3-(5-(bromomethyl)-1-oxoisoindolin-2-yl)piperidine-2,6-dione were used to prepare the target compound (GT-03320) (white solid, 25 mg, yield 26%). ¹H NMR (400 MHz, MeOD) δ 7.89 (d, *J* = 7.9 Hz, 1H), 7.82 (s, 1H), 7.76 - 7.68 (m, 2H), 7.60 (s, 1H), 7.51 - 7.43 (m, 2H), 5.18 (dd, *J* = 13.3, 5.1 Hz, 1H), 4.62 - 4.47 (m, 4H), 4.42 (s, 2H), 3.29 - 3.22 (m, 2H), 2.97 - 2.86 (m, 1H), 2.84 - 2.75 (m, 1H), 2.52 (qd, *J* = 13.2, 4.8 Hz, 1H), 2.23 - 2.15 (m, 1H), 2.14 - 2.02 (m, 4H), 1.72 (s, 3H). LCMS (ESI) m/z: calcd for C₃₀H₃₂Cl₂N₇O₃ ⁺ [M+H]⁺: 608.19, found, 608.3.

### Example 47: preparation of 3-(5-((((3S,4S)-8-(6-amino-5-((2-amino-3-chloropyridin-4-yl)thio)pyrazin-2-yl)-3-methyl-2-oxa-8-azaspiro[4.5]decan-4-yl)amino)methyl)-1-oxoisoindolin-2-yl)piperidine-2,6-dione (GT-03321)

Referring to the method of Step 2 in Scheme 11 and the method of Example 1, (3S,4S)-8-(6-amino-5-((2-amino-3-chloropyridin-4-yl)thio)pyrazin-2-yl)-3-methyl-2-oxa-8-azaspiro[4.5]decan-4-amine (CAS NO.: 1801765-04-7) and 3-(5-(bromomethyl)-1-oxoisoindolin-2-yl)piperidine-2,6-dione were used to prepare the target compound (GT-03321) (white solid, 5 mg, yield 28%). ¹H NMR (400 MHz, MeOD) δ 7.89 - 7.84 (m, 1H), 7.81 (s, 1H), 7.73 (d, *J* = 7.8 Hz, 1H), 7.67 - 7.56 (m, 3H), 6.15 (dd, *J* = 13.7, 6.5 Hz, 1H), 5.17 (dd, *J* = 13.3, 5.2 Hz, 1H), 4.14 (d, *J* = 9.3 Hz, 1H), 4.00 (d, *J* = 9.3 Hz, 1H), 3.88 (d, *J* = 9.2 Hz, 1H), 3.81 - 3.67 (m, 2H), 3.51 - 3.41 (m, 2H), 3.27 - 3.22 (m, 1H), 3.14 - 3.00 (m, 2H), 2.96 - 2.86 (m, 1H), 2.84 - 2.73 (m, 1H), 2.59 - 2.41 (m, 1H), 2.22 - 2.11 (m, 1H), 1.90 - 1.76 (m, 3H), 1.72 - 1.62 (m, 1H). LCMS (ESI) m/z: calcd for C₃₂H₃₇ClN₉O₄S ⁺ [M+H]⁺: 678.24, found, 678.2.

### Example 48: preparation of 3-(5-(((1-(3-(2,3-dichlorophenyl)-1H-pyrazolo[3,4-b]pyrazin-6-yl)-4-methylpiperidin-4-yl)amino)methyl)-1-oxoisoindolin-2-yl)piperidine-2,6-dione (GT-03322)

Referring to the method of Step 2 in Scheme 11 and the method of Example 1, 1-(3-(2,3-dichlorophenyl)-1H-pyrazolo[3,4-b]pyrazin-6-yl)-4-methylpiperidin-4-amine (CAS NO.: 2160546-07-47) and 3-(5-(bromomethyl)-1-oxoisoindolin-2-yl)piperidine-2,6-dione were used to prepare the target compound (GT-03322) (white solid, 7.6 mg, yield 21%). ¹H NMR (400 MHz, MeOD) δ 8.41 (s, 1H), 7.90 (d, *J* = 7.8 Hz, 1H), 7.75 (s, 1H), 7.67 (dd, *J* = 8.0, 1.6 Hz, 2H), 7.57 (dd, *J* = 7.7, 1.5 Hz, 1H), 7.43 (t, *J* = 7.9 Hz, 1H), 5.17 (dd, *J* = 13.3, 5.1 Hz, 1H), 4.72 - 4.63 (m, 2H), 4.55 (q, *J* = 17.4 Hz, 2H), 4.42 (s, 2H), 3.38 - 3.33 (m, 2H), 2.98 - 2.86 (m, 1H), 2.83 - 2.74 (m, 1H), 2.51 (qd, *J* = 13.4, 4.9 Hz, 1H), 2.22 - 2.16 (m, 1H), 2.14 - 1.97 (m, 4H), 1.74 (s, 3H). LCMS (ESI) m/z: calcd for C₃₁H₃₁Cl₂N₈O₃ ⁺ [M+H]⁺: 633.19, found, 633.2.

### Example 49: preparation of 3-(5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-1-oxoisoindolin-2-yl)piperidine-2,6-dione (GT-03319)

Referring to the method of Step 2 in Scheme 11 and the method of Example 1, (2-((5-chloro-2-((2-methoxy-4-(4-(methylamino)piperidin-1-yl)phenyl)amino)pyrimidin-4-yl)amino)phenyl)dimethylphosphine oxide (CAS NO.: 2353496-90-7) and 3-(5-(bromomethyl)-1-oxoisoindolin-2-yl)piperidine-2,6-dione were used to prepare the target compound (GT-03319) (white solid, 12.8 mg, yield 27%). ¹H NMR (400 MHz, MeOD) δ 8.41 (s, 1H), 7.90 (d, *J* = 7.8 Hz, 1H), 7.75 (s, 1H), 7.67 (dd, *J* = 8.0, 1.6 Hz, 2H), 7.57 (dd, *J* = 7.7, 1.5 Hz, 1H), 7.43 (t, *J* = 7.9 Hz, 1H), 5.17 (dd, *J* = 13.3, 5.1 Hz, 1H), 4.72 - 4.63 (m, 2H), 4.55 (q, *J* = 17.4 Hz, 2H), 4.42 (s, 2H), 3.38 - 3.33 (m, 2H), 2.98 - 2.86 (m, 1H), 2.83 - 2.74 (m, 1H), 2.51 (qd, *J* = 13.4, 4.9 Hz, 1H), 2.22 - 2.16 (m, 1H), 2.14 - 1.97 (m, 4H), 1.74 (s, 3H). LCMS (ESI) m/z: calcd for C₃₁H₃₁Cl₂N₈O₃ ⁺ [M+H]⁺: 633.19, found, 633.2.

### Example 50: preparation of 3-(5-(((4-((3-chloro-4-fluorophenyl)amino)-7-methoxyquinazolin-6-yl)amino)methyl)-1-oxoisoindolin-2-yl)piperidine-2,6-dione (GT-03323)

Referring to the method of Step 2 in Scheme 11 and the method of Example 1, N⁴-(3-chloro-4-fluorophenyl)-7-methoxyquinazoline-4,6-diamine (CAS NO.: 179552-75-1) and 3-(5-(bromomethyl)-1-oxoisoindolin-2-yl)piperidine-2,6-dione were used to prepare the target compound (GT-03323) (white solid, 9.2 mg, yield 21%). ¹H NMR (400 MHz, MeOD) δ 8.91 (s, 1H), 8.54 (s, 1H), 7.90 - 7.81 (m, 1H), 7.80 - 7.71 (m, 1H), 7.65 - 7.44 (m, 3H), 7.41 - 7.25 (m, 1H), 7.15 (d, *J* = 16.5 Hz, 1H), 5.17 - 5.07 (m, 1H), 4.78 (s, 3H), 4.51 - 4.39 (m, 2H), 4.16 (s, 1H), 3.98 (s, 1H), 2.95 - 2.84 (m, 1H), 2.82 - 2.72 (m, 1H), 2.53 - 2.38 (m, 1H), 2.21 - 2.10 (m, 1H). LCMS (ESI) m/z: calcd for C₂₉H₂₅ClFN₆O₄ ⁺ [M+H]⁺: 575.16, found, 575.2.

### Example 51: preparation of 3-(5-(((4-((3-chloro-4-fluorophenyl)amino)-7-methoxyquinazolin-6-yl)oxy)methyl)-1-oxoisoindolin-2-yl)piperidine-2,6-dione (GT-03324)

Referring to the method in Scheme 12, 4-((3-chloro-4-fluorophenyl)amino)-7-methoxyquinazolin-6-ol (CAS NO.: 184475-71-6) and 3-(5-(bromomethyl)-1-oxoisoindolin-2-yl)piperidine-2,6-dione were used to prepare the target compound (GT-03324) (white solid, 7.6 mg, yield 19%). ¹H NMR (400 MHz, MeOD) δ 8.74 (s, 1H), 8.14 (s, 1H), 7.96 (dd, *J* = 6.6, 2.5 Hz, 1H), 7.87 (d, *J* = 7.8 Hz, 1H), 7.78 (s, 1H), 7.72 - 7.64 (m, 2H), 7.37 (t, *J* = 8.9 Hz, 1H), 7.28 (s, 1H), 5.47 (s, 2H), 5.18 (dd, *J* = 13.3, 5.1 Hz, 1H), 4.61 - 4.48 (m, 2H), 4.12 (s, 3H), 2.99 - 2.86 (m, 1H), 2.84 - 2.75 (m, 1H), 2.51 (qd, *J* = 13.1, 4.6 Hz, 1H), 2.26 - 2.14 (m, 1H). LCMS (ESI) m/z: calcd for C₂₉H₂₄ClFN₅O₅ ⁺ [M+H]⁺: 576.14, found, 576.1.

### Example 52: preparation of 3-(5-(((4-((3-chloro-2-fluorophenyl)amino)-7-methoxyquinazolin-6-yl)oxy)methyl)-1-oxoisoindolin-2-yl)piperidine-2,6-dione (GT-03325)

Referring to the method in Scheme 12, 4-((3-chloro-2-fluorophenyl)amino)-7-methoxyquinazolin-6-ol (CAS NO.: 612501-52-7) and 3-(5-(bromomethyl)-1-oxoisoindolin-2-yl)piperidine-2,6-dione were used to prepare the target compound (GT-03324) (white solid, 5 mg, yield 13%). ¹H NMR (400 MHz, MeOD) δ 8.63 (s, 1H), 8.04 (s, 1H), 7.90 - 7.85 (m, 1H), 7.78 (s, 1H), 7.70 (d, *J* = 7.5 Hz, 1H), 7.56 - 7.51 (m, 2H), 7.30 - 7.26 (m, 2H), 5.46 (s, 2H), 5.18 (dd, *J* = 13.2, 5.1 Hz, 1H), 4.59 - 4.53 (m, 2H), 4.11 (s, 3H), 2.93 - 2.88 (m, 1H), 2.82 - 2.78 (m, 1H), 2.54 - 2.47 (m, 1H), 2.22 - 2.18 (m, 1H). LCMS (ESI) m/z: calcd for C₂₉H₂₄ClFN₅O₅ ⁺ [M+H]⁺: 576.14, found, 576.1.

### Example 53: preparation of N-(5-(((5-(tert-butyl)oxazol-2-yl)methyl)thio)thiazol-2-yl)-1-((2-(2,6-dioxopiperidin-3-yl)-1-oxoisoindolin-5-yl)methyl)piperidine-4-carboxamide (GT-03242)

Referring to the method of Step 2 in Scheme 11 and the method of Example 1, N-(5-(((5-(tert-butyl)oxazol-2-yl)methyl)thio)thiazol-2-yl)piperidine-4-carboxamide (CAS NO.: 345627-80-7) and 3-(5-(bromomethyl)-1-oxoisoindolin-2-yl)piperidine-2,6-dione (GT-03234) were used to prepare the target compound (GT-03242) (white solid, 50 mg, yield 54%). ¹H NMR (400 MHz, DMSO) δ 12.44 (s, 1H), 11.01 (s, 1H), 10.74 (s, 1H), 7.87 (s, 1H), 7.82 (t, *J* = 6.3 Hz, 1H), 7.75 (d, *J* = 7.9 Hz, 1H), 7.40 (s, 1H), 6.71 (s, 1H), 5.14 (dd, *J* = 13.3, 5.1 Hz, 1H), 4.55 - 4.46 (m, 2H), 4.46 - 4.33 (m, 3H), 4.05 (s, 2H), 3.44 - 3.38 (m, 2H), 3.16 - 3.08 (m, 1H), 2.79 - 2.70 (m, 1H), 2.65 - 2.57 (m, 1H), 2.45 - 2.37 (m, 1H), 2.16 - 2.07 (m, 1H), 2.04 - 1.97 (m, 4H), 1.17 (s, 9H). LCMS (ESI) m/z: calcd for C₃₁H₃₇N₆O₅S₂ ⁺ [M+H]⁺: 637.23, found, 637.3.

### Example 54: preparation of N-(2-(4-((2-(2,6-dioxopiperidin-3-yl)-1-oxoisoindolin-5-yl)methyl)piperazin-1-yl)ethyl)-4,9-dioxo-4,9-dihydronaphtho[2,3-b]furan-2-carboxamide (GT-03397)

The target compound (GT-03397) was prepared by referring to the method of Step 2 in Scheme 11 and the method of Example 1 (white solid, 19 mg, yield 34.6%). ¹H NMR (400 MHz, DMSO) δ 11.00 (s, 1H), 9.21 (s, 1H), 8.14 (ddt, *J* = 25.0, 23.7, 12.0 Hz, 2H), 7.93 (dd, *J* = 5.2, 3.8 Hz, 2H), 7.85 - 7.41 (m, 4H), 5.13 (dd, *J* = 13.3, 5.2 Hz, 1H), 4.42 (dd, *J* = 51.1, 17.6 Hz, 2H), 3.60 (d, *J* = 40.1 Hz, 8H), 3.17 (s, 6H), 2.95 - 2.88 (m, 1H), 2.61 (d, *J* = 16.6 Hz, 1H), 2.44 - 2.31 (m, 1H), 2.07 - 1.96 (m, 1H). LCMS (ESI) calcd for C₃₃H₃₂N₅O₇⁺ [M+H]⁺: 610.23, found, 610.3.

### Example 55: preparation of 8-(4-((2-(2,6-dioxopiperidin-3-yl)-1-oxoisoindolin-5-yl)methyl)piperazin-1-yl)-9-ethyl-6,6-dimethyl-11-oxo-6,11-dihydro-5H-benzo[b]carbazole-3-carbonitrile (GT-03409)

Referring to the method of Step 2 in Scheme 11 and the method of Example 1, 9-ethyl-6,6-dimethyl-11-oxo-8-(piperazin-1-yl)-6,11-dihydro-5H-benzo[b]carbazole-3-carbonitrile (GT-D-42) prepared from Intermediate Example 44 and 3-(5-(bromomethyl)-1-oxoisoindolin-2-yl)piperidine-2,6-dione were used to prepare the target compound (GT-03409) (white solid, 48 mg, yield 55.5%). ¹H NMR (400 MHz, MeOD) δ 8.40 (d, *J* = 8.3 Hz, 1H), 8.23 (s, 1H), 7.95 (d, *J* = 7.9 Hz, 1H), 7.84 (d, *J* = 12.9 Hz, 2H), 7.76 (t, *J* = 7.8 Hz, 1H), 7.60 - 7.49 (m, 1H), 7.42 (s, 1H), 5.19 (dd, *J* = 13.2, 5.0 Hz, 1H), 4.65 - 4.40 (m, 4H), 3.40 (d, *J* = 63.5 Hz, 8H), 2.98 - 2.75 (m, 4H), 2.52 (dd, *J* = 12.9, 4.5 Hz, 1H), 2.20 (d, *J* = 10.3 Hz, 1H), 1.80 (s, 6H), 1.35 (t, *J* = 7.5 Hz, 3H). LCMS (ESI) calcd for C₃₉H₃₉N₆O₄⁺ [M+H]⁺: 655.30, found, 655.3.

### Example 56: preparation of 3-(5-((4-(4-(5,7-dimethoxy-4-oxo-3,4-dihydroquinazolin-2-yl)phenyl)piperazin-1-yl)methyl)-1-oxoisoindolin-2-yl)piperidine-2,6-dione (GT-03410)

Referring to the method of Step 2 in Scheme 11 and the method of Example 1, 5,7-dimethoxy-2-(4-(piperazin-1-yl)phenyl)quinazolin-4(3H)-one (GT-D-51) prepared from Intermediate Example 61 and 3-(5-(bromomethyl)-1-oxoisoindolin-2-yl)piperidine-2,6-dione were used to prepare the target compound (GT-03410) (white solid, 48 mg, yield 53.5%). ¹H NMR (400 MHz, DMSO) δ 11.02 (s, 2H), 8.13 (d, *J* = 9.0 Hz, 2H), 7.86 (d, *J* = 8.3 Hz, 2H), 7.76 (d, *J* = 7.8 Hz, 1H), 7.11 (d, *J* = 9.1 Hz, 2H), 6.79 (s, 1H), 6.55 (s, 1H), 5.16 (dd, *J* = 13.2, 4.9 Hz, 1H), 4.48 (dt, *J* = 40.8, 12.5 Hz, 4H), 4.09 (d, *J* = 9.9 Hz, 2H), 3.88 (d, *J* = 13.9 Hz, 6H), 3.20 (s, 6H), 2.93 (dd, *J* = 22.0, 8.5 Hz, 1H), 2.68 - 2.60 (m, 1H), 2.44 (dd, *J* = 12.9, 4.1 Hz, 1H), 2.04 (d, *J* = 5.6 Hz, 1H). LCMS (ESI) calcd for C₃₄H₃₅N₆O₆⁺ [M+H]⁺: 623.26, found, 623.3.

### Example 57: preparation of N-(4-((3-chloro-4-fluorophenyl)amino)-7-(3-(4-((2-(2,6-dioxopiperidin-3-yl)-1-oxoisoindolin-5-yl)methyl)piperazin-1-yl)propoxy)quinazolin-6-yl)acrylamide (GT-03411)

Referring to the method of Step 2 in Scheme 11 and the method of Example 1, N-(4-((3-chloro-4-fluorophenyl)amino)-7-(3-(piperazin-1-yl)propoxy)quinazolin-6-yl)acrylamide (CAS NO.: 2600734-25-4) and 3-(5-(bromomethyl)-1-oxoisoindolin-2-yl)piperidine-2,6-dione (GT-03234) were used to prepare the target compound (GT-03411) (white solid, 34 mg, yield 40.5%). ¹H NMR (400 MHz, MeOD) δ 9.05 (s, 1H), 8.66 (s, 1H), 7.84 (dd, *J* = 6.6, 2.5 Hz, 1H), 7.74 (d, *J* = 7.9 Hz, 1H), 7.54 (dd, *J* = 17.3, 11.2 Hz, 3H), 7.27 (dd, *J* = 16.5, 7.6 Hz, 2H), 6.65 (dd, *J* = 16.8, 10.3 Hz, 1H), 6.41 (d, *J* = 16.8 Hz, 1H), 5.80 (d, *J* = 11.6 Hz, 1H), 5.07 (dd, *J* = 13.1, 5.0 Hz, 1H), 4.59 - 4.32 (m, 4H), 3.93 (s, 2H), 2.93 (d, *J* = 83.9 Hz, 11H), 2.69 (d, *J* = 14.1 Hz, 1H), 2.46 - 2.22 (m, 3H), 2.10 (s, 1H). LCMS (ESI) calcd for C₃₈H₃₉ClFN₈O₅⁺ [M+H]⁺: 741.27, found, 741.3.

### Example 58: preparation of 3-(5-((4-((4-((3,4-dichloro-2-fluorophenyl)amino)-7-methoxyquinazolin-6-yl)oxy)piperidin-1-yl)methyl)-1-oxoisoindolin-2-yl)piperidine-2,6-dione (GT-03412)

Referring to the method of Step 2 in Scheme 11 and the method of Example 1, N-(3,4-dichloro-2-fluorophenyl)-7-methoxy-6-(piperidin-4-yloxy)quinazolin-4-amine (CAS NO.: 1429809-12-0) and 3-(5-(bromomethyl)-1-oxoisoindolin-2-yl)piperidine-2,6-dione were used to prepare the target compound (GT-03412) (white solid, 47 mg, yield 56.5%). ¹H NMR (400 MHz, MeOD) δ 8.58 (s, 1H), 8.08 (s, 1H), 7.84 (d, *J* = 7.9 Hz, 1H), 7.76 (s, 1H), 7.66 (s, 1H), 7.50 - 7.37 (m, 2H), 7.19 (s, 1H), 5.13 - 5.08 (m, 1H), 4.55 - 4.39 (m, 4H), 3.99 (d, *J* = 13.6 Hz, 3H), 3.31 (d, *J* = 57.2 Hz, 6H), 2.78 (ddd, *J* = 41.1, 25.9, 14.3 Hz, 2H), 2.47 - 2.07 (m, 5H). LCMS (ESI) calcd for C₃₄H₃₂Cl₂FN₆O₅⁺ [M+H]⁺: 693.18, found, 693.2.

### Example 59: preparation of N-(5-(3,5-dimethoxyphenethyl)-1H-pyrazol-3-yl)-4-((3R,5S)-4-((2-(2,6-dioxopiperidin-3-yl)-1-oxoisoindolin-5-yl)methyl)-3,5-dimethylpiperazin-1-yl)benzamide (GT-03447)

Referring to the method of Step 2 in Scheme 11 and the method of Example 1, N-(5-(3,5-dimethoxyphenethyl)-1H-pyrazol-3-yl)-4-((3R,5S)-3,5-dimethylpiperazin-1-yl)benzamide (CAS NO.: 1615687-07-4) and 3-(5-(bromomethyl)-1-oxoisoindolin-2-yl)piperidine-2,6-dione were used to prepare the target compound (GT-03447) (white solid, 26 mg, yield 52.9%). ¹H NMR (400 MHz, MeOD) δ 8.22 - 7.50 (m, 6H), 7.23 - 6.94 (m, 2H), 6.34 (dd, *J* = 18.8, 2.0 Hz, 3H), 5.17 (dd, *J* = 13.3, 5.1 Hz, 1H), 4.74 - 4.39 (m, 4H), 4.08 (d, *J* = 12.2 Hz, 2H), 3.73 (s, 6H), 3.56 (dd, *J* = 35.7, 17.8 Hz, 2H), 3.15 (s, 1H), 3.06 - 2.72 (m, 7H), 2.56 - 2.42 (m, 1H), 2.24 - 2.11 (m, 1H), 1.68 (s, 5H), 1.46 - 1.33 (m, 1H). LCMS (ESI) calcd for C₄₀H₄₆N₇O₆⁺ [M+H]⁺: 720.35, found, 720.4.

### Example 60: preparation of 3-(1-oxo-5-((4-(4-(3-(quinolin-4-yl)pyrazolo[1,5-a]pyrimidin-6-yl)phenyl)piperazin-1-yl)methyl)isoindolin-2-yl)piperidine-2,6-dione (GT-03441)

Referring to the method of Step 2 in Scheme 11 and the method of Example 1, 4-(6-(4-(piperazin-1-yl)phenyl)pyrazolo[1,5-a]pyrimidin-3-yl)quinoline (CAS NO.: 1062368-24-4) and 3-(5-(bromomethyl)-1-oxoisoindolin-2-yl)piperidine-2,6-dione were used to prepare the target compound (GT-03441) (white solid, 38 mg, yield 60.4%). ¹H NMR (400 MHz, MeOD) δ 9.29 (d, *J* = 2.2 Hz, 1H), 9.04 (d, *J* = 2.2 Hz, 1H), 8.97 (d, *J* = 5.9 Hz, 1H), 8.79 (s, 1H), 8.62 (d, *J* = 8.8 Hz, 1H), 8.45 (d, *J* = 5.9 Hz, 1H), 8.14 (d, *J* = 8.6 Hz, 1H), 8.06 (t, *J* = 7.7 Hz, 1H), 7.87 (t, *J* = 7.8 Hz, 2H), 7.74 (d, *J* = 16.1 Hz, 1H), 7.72 - 7.59 (m, 3H), 7.13 (d, *J* = 8.8 Hz, 2H), 5.10 (dd, *J* = 13.3, 5.1 Hz, 1H), 4.56 - 4.44 (m, 4H), 3.78 - 3.23 (m, 8H), 2.86 - 2.67 (m, 2H), 2.44 (dt, *J* = 13.1, 8.5 Hz, 1H), 2.12 (dd, *J* = 14.8, 9.8 Hz, 1H). LCMS (ESI) calcd for C₃₉H₃₅N₈O₃⁺ [M+H]⁺: 663.28, found, 663.3.

### Example 61: preparation of 3-(1-oxo-5-((4-(4-(3-(quinolin-5-yl)pyrazolo[1,5-a]pyrimidin-6-yl)phenyl)piperazin-1-yl)methyl)isoindolin-2-yl)piperidine-2,6-dione (GT-03442)

Referring to the method of Step 2 in Scheme 11 and the method of Example 1, 5-(6-(4-(piperazin-1-yl)phenyl)pyrazolo[1,5-a]pyrimidin-3-yl)quinoline (CAS NO.: 1432597-26-6) and 3-(5-(bromomethyl)-1-oxoisoindolin-2-yl)piperidine-2,6-dione were used to prepare the target compound (GT-03442) (white solid, 40 mg, yield 58.4%). ¹H NMR (400 MHz, MeOD) δ 9.15 (d, *J* = 2.2 Hz, 1H), 8.98 (s, 1H), 8.80 (d, *J* = 2.3 Hz, 2H), 8.42 (s, 1H), 8.08 (d, *J* = 8.5 Hz, 1H), 8.00 (dd, *J* = 17.4, 8.7 Hz, 2H), 7.87 (d, *J* = 8.0 Hz, 1H), 7.71 (d, *J* = 7.8 Hz, 3H), 7.66 (s, 1H), 7.64 (s, 1H), 7.11 (d, *J* = 8.9 Hz, 2H), 5.10 - 5.08 (m, 1H), 4.49 (s, 4H), 3.33 (d, *J* = 40.3 Hz, 8H), 2.82 - 2.77 (m, 1H), 2.74 - 2.70 (m, 1H), 2.42 (s, 1H), 2.10 (s, 1H). LCMS (ESI) calcd for C₃₉H₃₅N₈O₃⁺ [M+H]⁺: 663.28, found, 663.3.

### Example 62: preparation of 4-(2,6-dichlorobenzamido)-N-(1-((2-(2,6-dioxopiperidin-3-yl)-1-oxoisoindolin-5-yl)methyl)piperidin-4-yl)-1H-pyrazole-3-carboxamide (GT-03448)

Referring to the method of Step 2 in Scheme 11 and the method of Example 1, 4-(2,6-dichlorobenzamido)-N-(piperidin-4-yl)-1H-pyrazole-3-carboxamide (CAS NO.: 844442-38-2) and 3-(5-(bromomethyl)-1-oxoisoindolin-2-yl)piperidine-2,6-dione were used to prepare the target compound (GT-03448) (white solid, 33 mg, yield 62.7%). ¹H NMR (400 MHz, MeOD) δ 8.34 (s, 1H), 7.91 (d, *J* = 7.9 Hz, 1H), 7.77 (s, 1H), 7.69 (d, *J* = 7.9 Hz, 1H), 7.54 - 7.40 (m, 3H), 5.17 (dd, *J* = 13.4, 5.1 Hz, 1H), 4.64 - 4.40 (m, 4H), 4.11 (t, *J* = 57.7 Hz, 1H), 3.70 - 3.44 (m, 2H), 3.26 - 3.11 (m, 2H), 2.86 (dtd, *J* = 15.6, 13.3, 3.9 Hz, 2H), 2.51 (qd, *J* = 13.4, 4.9 Hz, 1H), 2.18 (d, *J* = 10.4 Hz, 4H), 1.92 (d, *J* = 10.0 Hz, 1H). LCMS (ESI) calcd for C₃₀H₃₀Cl₂N₇O₅⁺ [M+H]⁺: 638.17, found, 638.2.

### Example 63: preparation of 3-(5-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)-1-oxoisoindolin-2-yl)piperidine-2,6-dione (GT-03485)

Referring to the method of Step 2 in Scheme 11 and the method of Example 1, the target compound (GT-03485) was prepared (white solid, 44 mg, yield 57.9%). ¹H NMR (400 MHz, MeOD) δ 9.13 (s, 1H), 7.87 - 7.78 (m, 4H), 7.68 (d, *J* = 8.2 Hz, 1H), 6.54 (s, 1H), 5.12 - 5.07 (m, 1H), 4.60 - 4.44 (m, 4H), 4.17 (s, 1H), 3.84 (s, 2H), 3.71 - 3.31 (m, 11H), 3.30 (s, 3H), 2.88 - 2.77 (m, 1H), 2.70 (d, *J* = 15.5 Hz, 1H), 2.51- 2.36 (m, 1H), 2.15 - 2.05 (m, 1H), 1.96 - 1.86 (m, 2H), 1.67 (d, *J* = 6.6 Hz, 2H), 1.43 (d, *J* = 6.6 Hz, 6H). LCMS (ESI) calcd for C₃₇H₄₆N₁₁O₄⁺ [M+H]⁺: 708.37, found, 708.4.

### Example 64: preparation of 6-(1-((2-(2,6-dioxopiperidin-3-yl)-1-oxoisoindolin-5-yl)methyl)piperidin-4-yl)-2-(4-phenoxyphenyl)nicotinamide (GT-03693)

Referring to the method of Step 2 in Scheme 11 and the method of Example 1, 2-(4-phenoxyphenyl)-6-(piperidin-4-yl)nicotinamide (GT-D-66) prepared from Intermediate Example 50 and 3-(5-(bromomethyl)-1-oxoisoindolin-2-yl)piperidine-2,6-dione were used to prepare the target compound (GT-03693) (white solid, 45 mg, yield 52.31%). ¹H NMR (400 MHz, DMSO) δ 11.01 (s, 1H), 9.50 (s, 1H), 7.93 (s, 1H), 7.88 (s, 1H), 7.83 - 7.77 (m, 3H), 7.72 (d, *J* = 8.7 Hz, 2H), 7.52 (s, 1H), 7.45 - 7.40 (m, 2H), 7.29 (d, *J* = 7.9 Hz, 1H), 7.18 (t, *J* = 7.4 Hz, 1H), 7.05 (dd, *J* = 12.2, 8.2 Hz, 4H), 5.14 (dd, *J* = 13.2, 5.1 Hz, 1H), 4.45 (dt, *J* = 38.1, 17.6 Hz, 5H), 3.46 (d, *J* = 10.9 Hz, 2H), 3.11 - 2.98 (m, 3H), 2.97 - 2.88 (m, 1H), 2.64 - 2.57 (m, 1H), 2.46 - 2.38 (m, 1H), 2.27 - 2.17 (m, 2H), 2.12 - 2.05 (m, 2H), 2.02 (dd, *J* = 8.8, 3.5 Hz, 1H). LCMS (ESI) calcd for C₃₇H₃₆N₅O₅⁺ [M+H]⁺: 630.26, found, 630.30.

### Example 65: preparation of 1'-((2-(2,6-dioxopiperidin-3-yl)-1-oxoisoindolin-5-yl)methyl)-6-(4-phenoxyphenyl)-1',2',3',6'-tetrahydro-[2,4'-bipyridine]-5-carboxamide (GT-03552)

Referring to the method of Step 2 in Scheme 11 and the method of Example 1, 6-(4-phenoxyphenyl)-1',2',3',6'-tetrahydro-[2,4'-bipyridine]-5-carboxamide (CAS NO.: 2484693-14-1) and 3-(5-(bromomethyl)-1-oxoisoindolin-2-yl)piperidine-2,6-dione (GT-03234) were used to prepare the target compound (GT-03552) (white solid, 73 mg, yield 40%). ¹H NMR (400 MHz, MeOD) δ 7.97 - 7.90 (m, 2H), 7.83 (s, 1H), 7.78 - 7.72 (m, 3H), 7.63 (d, *J* = 8.1 Hz, 1H), 7.43 - 7.34 (m, 2H), 7.16 (t, *J* = 7.5 Hz, 1H), 7.08 - 6.98 (m, 4H), 6.79 (s, 1H), 5.20 (dd, *J* = 13.4, 5.1 Hz, 1H), 4.67 - 4.52 (m, 4H), 4.05 - 3.96 (m, 2H), 3.91 - 3.77 (m, 1H), 3.53 - 3.38 (m, 1H), 3.25 - 3.10 (m, 1H), 3.07 - 2.86 (m, 2H), 2.84 - 2.72 (m, 1H), 2.60 - 2.45 (m, 1H), 2.24 - 2.13 (m, 1H). LCMS (ESI) calcd for C₃₇H₃₄N₅O₅ ⁺ [M+H]⁺: 628.26, found, 628.23.

### Example 66: preparation of 6-(4-((2-(2,6-dioxopiperidin-3-yl)-1-oxoisoindolin-5-yl)methyl)piperazin-1-yl)-2-(4-phenoxyphenyl)nicotinamide (GT-03636)

Referring to the method of Step 2 in Scheme 11 and the method of Example 1, 2-(4-phenoxyphenyl)-6-(piperazin-1-yl)nicotinamide (GT-D-76) prepared from Intermediate Example 51 and 3-(5-(bromomethyl)-1-oxoisoindolin-2-yl)piperidine-2,6-dione were used to prepare the target compound (GT-03636) (white solid, 32 mg, yield 34%). ¹H NMR (400 MHz, MeOD) δ7.94 (d, *J* = 7.8 Hz, 1H), 7.85 - 7.79 (m, 2H), 7.73 (d, *J* = 7.9 Hz, 1H), 7.70 - 7.65 (m, 2H), 7.40 - 7.35 (m, 2H), 7.15 (t, *J* = 7.4 Hz, 1H), 7.06 - 6.97 (m, 4H), 6.91 (d, *J* = 8.7 Hz, 1H), 5.19 (dd, *J* = 13.3, 5.2 Hz, 1H), 4.61 - 4.51 (m, 4H), 3.72 - 3.32 (m, 8H), 2.98 - 2.87 (m, 1H), 2.84 - 2.75 (m, 1H), 2.52 (qd, *J* = 13.1, 4.4 Hz, 1H), 2.25 - 2.12 (m, 1H). LCMS (ESI) m/z: calcd for C₃₆H₃₅N₆O₅⁺[M+H]⁺, 631.27; found, 631.3.

### Example 67: preparation of 6-(4-(((2-(2,6-dioxopiperidin-3-yl)-1-oxoisoindolin-5-yl)methyl)(methyl)amino)piperidin-1-yl)-2-(4-phenoxyphenyl)nicotinamide (GT-03803)

Referring to the method of Step 2 in Scheme 11 and the method of Example 1, 6-(4-(methylamino)piperidin-1-yl)-2-(4-phenoxyphenyl)nicotinamide (GT-D-77) prepared from Intermediate Example 52 and 3-(5-(bromomethyl)-1-oxoisoindolin-2-yl)piperidine-2,6-dione were used to prepare the target compound (GT-03803) (white solid, 60 mg, yield 68%). ¹H NMR (400 MHz, MeOD) δ 7.91 (d, *J* = 7.8 Hz, 1H), 7.87 (d, *J* = 8.9 Hz, 1H), 7.83 (s, 1H), 7.72 (d, *J* = 7.9 Hz, 1H), 7.69 - 7.64 (m, 2H), 7.42 - 7.36 (m, 2H), 7.19 - 7.14 (m, 1H), 7.08 - 7.00 (m, 5H), 5.18 (dd, *J* = 13.3, 5.1 Hz, 1H), 4.78 - 4.65 (m, 3H), 4.57 (q, *J* = 17.8 Hz, 2H), 4.37 (d, *J* = 13.0 Hz, 1H), 3.80 - 3.70 (m, 1H), 3.19 - 3.06 (m, 2H), 2.97 - 2.86 (m, 1H), 2.83 - 2.78 (m, 1H), 2.77 (s, 3H), 2.51 (qd, *J* = 13.2, 4.6 Hz, 1H), 2.36 - 2.26 (m, 2H), 2.23 - 2.14 (m, 1H), 2.04 - 1.88 (m, 2H). LCMS (ESI) m/z: calcd for C₃₈H₃₉N₆O₅⁺[M+H]⁺, 659.30; found, 659.3.

### Example 68: preparation of 6-(4-(((2-(2,6-dioxopiperidin-3-yl)-4-fluoro-1-oxoisoindolin-5-yl)methyl)(methyl)amino)piperidin-1-yl)-2-(4-phenoxyphenyl)nicotinamide (GT-03824)

Referring to the methods of Scheme 11 and Example 1, 3-(4-fluoro-5-(hydroxymethyl)-1-oxoisoindolin-2-yl)piperidine-2,6-dione (GT-03445) and 2-(4-phenoxyphenyl)-6-(piperidin-4-yl)nicotinamide (GT-D-66) prepared from Intermediate Example 50 were used to prepare the target compound (GT-03824) (white solid, 48 mg, yield 57%). ¹H NMR (400 MHz, DMSO) δ 11.07 (s, 1H), 10.82 (s, 1H), 9.42 (s, 1H), 8.83 (d, *J* = 7.9 Hz, 1H), 8.43 (s, 1H), 8.29 (s, 1H), 7.98 (d, *J* = 7.6 Hz, 1H), 7.85 (d, *J* = 7.4 Hz, 1H), 7.66 (t, *J* = 7.6 Hz, 1H), 7.13 (t, *J* = 53.6 Hz, 1H), 6.91 (d, *J* = 8.0 Hz, 1H), 5.20 (dd, *J* = 13.1, 5.1 Hz, 1H), 4.56 - 4.50 (m, 2H), 4.42 (s, 2H), 3.89 - 3.67 (m, 10H), 3.57 - 3.54 (m, 1H), 3.25 (d, *J* = 10.6 Hz, 2H), 3.02 - 2.91 (m, 1H), 2.72 - 2.63 (m, 1H), 2.40 - 2.23 (m, 5H), 2.12 - 2.03 (m, 1H). LCMS (ESI) m/z: calcd for C₃₄H₃₇F₂N₁₀O₅⁺[M+H]⁺, 703.29; found, 703.3.

### Example 69: preparation of 6-(4-(((2-(2,6-dioxopiperidin-3-yl)-6-fluoro-1-oxoisoindolin-5-yl)methyl)(methyl)amino)piperidin-1-yl)-2-(4-phenoxyphenyl)nicotinamide (GT-03825)

Referring to the methods of Scheme 11 and Example 1, the target compound (GT-03825) was prepared (white solid,54 mg, yield 60%). ¹H NMR (400 MHz, DMSO) δ 11.02 (s, 1H), 10.83 (s, 1H), 8.04 (d, *J* = 6.0 Hz, 1H), 7.70 - 7.62 (m, 4H), 7.55 (s, 1H), 7.46 - 7.39 (m, 2H), 7.24 (s, 1H), 7.18 (t, *J* = 7.4 Hz, 1H), 7.07 (dd, *J* = 8.6, 1.0 Hz, 2H), 7.04 - 6.99 (m, 2H), 6.90 (d, *J* = 8.8 Hz, 1H), 5.14 (dd, *J* = 13.1, 4.8 Hz, 1H), 4.71 - 4.57 (m, 3H), 4.55 - 4.33 (m, 3H), 3.68 - 3.53 (m, 1H), 3.00 - 2.83 (m, 3H), 2.70 - 2.57 (m, 4H), 2.47 - 2.36 (m, 1H), 2.31 (d, *J* = 13.1 Hz, 1H), 2.20 (d, *J* = 10.9 Hz, 1H), 2.07 - 1.97 (m, 1H), 1.84 - 1.68 (m, 2H). LCMS (ESI) m/z: calcd for C₃₈H₃₈FN₆O₅⁺[M+H]⁺, 677.29; found, 677.3.

### Example 70: preparation of 6-(4-(((2-(2,6-dioxopiperidin-3-yl)-7-fluoro-1-oxoisoindolin-5-yl)methyl)(methyl)amino)piperidin-1-yl)-2-(4-phenoxyphenyl)nicotinamide (GT-03826)

Referring to the methods of Scheme 11 and Example 1, the target compound (GT-03826) was prepared using 3-(7-fluoro-5-(hydroxymethyl)-1-oxoisoindolin-2-yl)piperidine-2,6-dione (GT-03446) and 2-(4-phenoxyphenyl)-6-(piperidin-4-yl) nicotinamide (GT-D-66) prepared according to Intermediate Example 50. The target compound (GT-03826) was obtained as a white solid (61 mg, yield 68%). ¹H NMR (400 MHz, DMSO) δ 11.02 (s, 1H), 10.68 (s, 1H), 7.72 - 7.62 (m, 5H), 7.54 (s, 1H), 7.46 - 7.40 (m, 2H), 7.23 (s, 1H), 7.20 - 7.16 (m, 1H), 7.10 - 7.05 (m, 2H), 7.04 - 6.99 (m, 2H), 6.89 (d, *J* = 8.8 Hz, 1H), 5.10 (dd, *J* = 13.1, 5.1 Hz, 1H), 4.70 - 4.55 (m, 3H), 4.54 - 4.47 (m, 1H), 4.45 - 4.27 (m, 2H), 3.64 - 3.51 (m, 1H), 2.98 - 2.84 (m, 3H), 2.65 - 2.56 (m, 4H), 2.46 - 2.35 (m, 1H), 2.29 - 2.16 (m, 2H), 2.05 - 1.96 (m, 1H), 1.83 - 1.64 (m, 2H). LCMS (ESI) m/z: calcd for C₃₈H₃₈FN₆O₅⁺[M+H]⁺, 677.29; found, 677.3.

### Example 71: preparation of 6-(4-(((2-(2,6-dioxopiperidin-3-yl)-1-oxoisoindolin-4-yl)methyl)(methyl)amino)piperidin-1-yl)-2-(4-phenoxyphenyl)nicotinamide (GT-03830)

The target compound (GT-03830) was prepared using the methods described in Scheme 11 and Example 1 (white solid, 55 mg, yield 63%). ¹H NMR (400 MHz, DMSO) δ 11.05 (s, 1H), 10.45 (s, 1H), 7.95 - 7.81 (m, 2H), 7.69 - 7.61 (m, 4H), 7.52 (s, 1H), 7.43 (t, *J* = 7.7 Hz, 2H), 7.23 (s, 1H), 7.18 (t, *J* = 7.1 Hz, 1H), 7.07 (d, *J* = 8.6 Hz, 2H), 7.02 (d, *J* = 8.6 Hz, 2H), 6.90 (d, *J* = 8.8 Hz, 1H), 5.19 (dd, *J* = 13.2, 4.8 Hz, 1H), 4.73 - 4.43 (m, 5H), 4.26 - 4.11 (m, 1H), 3.00 - 2.87 (m, 4H), 2.72 - 2.61 (m, 5H), 2.44 - 2.31 (m, 2H), 2.30 - 2.18 (m, 2H), 2.07 - 1.97 (m, 1H), 1.89 - 1.69 (m, 2H). LCMS (ESI) m/z: calcd for C₃₈H₃₉N₆O₅⁺[M+H]⁺, 659.30; found, 659.3.

### Example 72: preparation of 6-(4-(4-((2-(2,6-dioxopiperidin-3-yl)-1-oxoisoindolin-5-yl)methyl)piperazin-1-yl)piperidin-1-yl)-2-(4-phenoxyphenyl)nicotinamide (GT-03748)

Referring to the method of Step 2 in Scheme 11 and the method of Example 1, the target compound (GT-03748) was prepared using 2-(4-phenoxyphenyl)-6-(4-(piperazin-1-yl)piperidin-1-yl)nicotinamide (GT-D-78) obtained from Intermediate Example 53 and 3-(5-(bromomethyl)-1-oxoisoindolin-2-yl)piperidine-2,6-dione. The target compound (GT-03748) was obtained as a white solid (43 mg, yield 65%). ¹H NMR (400 MHz, DMSO) δ 11.00 (s, 1H), 7.87 - 7.68 (m, 3H), 7.67 - 7.60 (m, 3H), 7.53 (s, 1H), 7.46 - 7.38 (m, 2H), 7.27 - 7.14 (m, 2H), 7.07 (d, *J* = 7.8 Hz, 2H), 7.01 (d, *J* = 8.6 Hz, 2H), 6.88 (d, *J* = 8.8 Hz, 1H), 5.13 (dd, *J* = 13.2, 5.0 Hz, 1H), 4.58 (d, *J* = 12.6 Hz, 2H), 4.49 (d, *J* = 17.5 Hz, 1H), 4.36 (d, *J* = 17.3 Hz, 1H), 3.88 - 3.55 (m, 10H), 2.99 - 2.82 (m, 3H), 2.70 - 2.57 (m, 1H), 2.45 - 2.31 (m, 1H), 2.22 - 2.08 (m, 2H), 2.05 - 1.95 (m, 1H), 1.70 - 1.54 (m, 2H). LCMS (ESI) m/z: calcd for C₄₁H₄₄N₇O₅⁺[M+H]⁺, 714.34; found, 714.3.

### Example 73: preparation of 6-(4-(4-((2-(2,6-dioxopiperidin-3-yl)-4-fluoro-1-oxoisoindolin-5-yl)methyl)piperazin-1-yl)piperidin-1-yl)-2-(4-phenoxyphenyl)nicotinamide (GT-03749)

Referring to the methods of Scheme 11 and Example 1, the target compound (GT-03750) (white solid, 41 mg, yield 60%) was prepared using 3-(4-fluoro-5-(hydroxymethyl)-1-oxoisoindolin-2-yl)piperidine-2,6-dione (GT-03445) and 2-(4-phenoxyphenyl)-6-(4-(piperazin-1-yl)piperidin-1-yl) nicotinamide (GT-D-78) prepared according to Intermediate Example 53. ¹H NMR (400 MHz, MeOD) δ 7.86 (d, *J* = 9.0 Hz, 1H), 7.72 - 7.62 (m, 4H), 7.42 - 7.35 (m, 2H), 7.17 (t, *J* = 7.3 Hz, 1H), 7.08 - 6.96 (m, 5H), 5.20 - 5.10 (m, 2H), 4.68 - 4.50 (m, 4H), 4.08 - 3.96 (m, 2H), 3.82 - 3.32 (m, 8H), 3.14 - 3.02 (m, 2H), 2.95 - 2.87 (m, 1H), 2.82 - 2.76 (m, 1H), 2.59 - 2.44 (m, 1H), 2.33 - 2.13 (m, 3H), 1.90 - 1.68 (m, 2H). LCMS (ESI) m/z: calcd for C₄₁H₄₃FN₇O₅⁺[M+H]⁺, 732.33; found, 732.4.

### Example 74: preparation of 6-(4-(4-((2-(2,6-dioxopiperidin-3-yl)-6-fluoro-1-oxoisoindolin-5-yl)methyl)piperazin-1-yl)piperidin-1-yl)-2-(4-phenoxyphenyl)nicotinamide (GT-03750)

The target compound (GT-03750) was prepared using the methods described in Scheme 11 and Example 1 (white solid,40 mg, yield 59%). ¹H NMR (400 MHz, MeOD) δ 7.91 (d, *J* = 9.0 Hz, 1H), 7.83 (d, *J* = 5.9 Hz, 1H), 7.65 (d, *J* = 8.7 Hz, 2H), 7.59 (d, *J* = 8.6 Hz, 1H), 7.43 - 7.35 (m, 2H), 7.17 (t, *J* = 7.4 Hz, 1H), 7.10 - 7.01 (m, 5H), 5.16 (dd, *J* = 13.3, 5.1 Hz, 1H), 4.94 - 4.88 (m, 1H), 4.66 - 4.44 (m, 4H), 4.22 (s, 2H), 3.70 - 3.32 (m, 8H), 3.19 - 3.10 (m, 2H), 2.96 - 2.86 (m, 1H), 2.82 - 2.73 (m, 1H), 2.50 (qd, *J* = 13.2, 4.7 Hz, 1H), 2.34 - 2.24 (m, 2H), 2.22 - 2.14 (m, 1H), 1.93 - 1.77 (m, 2H). LCMS (ESI) m/z: calcd for C₄₁H₄₃FN₇O₅⁺[M+H]⁺, 732.33; found, 732.3.

### Example 75: preparation of 6-(4-(4-((2-(2,6-dioxopiperidin-3-yl)-7-fluoro-1-oxoisoindolin-5-yl)methyl)piperazin-1-yl)piperidin-1-yl)-2-(4-phenoxyphenyl)nicotinamide (GT-03751)

Referring to the methods of Scheme 11 and Example 1, the target compound (GT-03751) was prepared using 3-(7-fluoro-5-(hydroxymethyl)-1-oxoisoindolin-2-yl)piperidine-2,6-dione (GT-03446) and 2-(4-phenoxyphenyl)-6-(4-(piperazin-1-yl)piperidin-1-yl) nicotinamide (GT-D-78) prepared according to Intermediate Example 53. The target compound (GT-03751) was obtained as a white solid (35 mg, yield 51%). ¹H NMR (400 MHz, MeOD) δ 7.91 (d, *J* = 9.0 Hz, 1H), 7.68 - 7.61 (m, 2H), 7.54 (s, 1H), 7.44 - 7.35 (m, 3H), 7.17 (t, *J* = 7.4 Hz, 1H), 7.12 - 7.02 (m, 5H), 5.13 (dd, *J* = 13.3, 5.2 Hz, 1H), 4.94 - 4.87 (m, 1H), 4.64 - 4.46 (m, 4H), 4.15 (s, 2H), 3.87 - 3.32 (m, 8H), 3.21 - 3.10 (m, 3H), 2.97 - 2.85 (m, 1H), 2.82 - 2.72 (m, 1H), 2.49 (qd, *J* = 13.2, 4.7 Hz, 1H), 2.33 - 2.26 (m, 2H), 2.23 - 2.12 (m, 1H), 1.92 - 1.79 (m, 2H). LCMS (ESI) m/z: calcd for C₄₁H₄₃FN₇O₅⁺[M+H]⁺, 732.33; found, 732.4.

### Example 76: preparation of 6-(4-(4-((2-(2,6-dioxopiperidin-3-yl)-1-oxoisoindolin-4-yl)methyl)piperazin-1-yl)piperidin-1-yl)-2-(4-phenoxyphenyl)nicotinamide (GT-03755)

The target compound (GT-03755) was prepared using the methods described in Scheme 11 and Example 1 (white solid, 31 mg, yield 46%). ¹H NMR (400 MHz, MeOD) δ 7.91 (d, *J* = 9.0 Hz, 1H), 7.84 (d, *J* = 7.5 Hz, 1H), 7.75 (d, *J* = 7.5 Hz, 1H), 7.68 - 7.63 (m, 2H), 7.59 (t, *J* = 7.6 Hz, 1H), 7.43 - 7.36 (m, 2H), 7.21 - 7.14 (m, 1H), 7.13 - 7.01 (m, 5H), 5.19 (dd, *J* = 13.3, 5.2 Hz, 1H), 4.81 - 4.57 (m, 4H), 4.26 - 3.99 (m, 2H), 3.72 - 3.32 (m, 7H), 3.30 - 3.06 (m, 4H), 3.00 - 2.87 (m, 1H), 2.84 - 2.73 (m, 1H), 2.55 (qd, *J* = 13.3, 4.7 Hz, 1H), 2.36 - 2.26 (m, 2H), 2.24 - 2.16 (m, 1H), 1.92 - 1.77 (m, 2H). LCMS (ESI) m/z: calcd for C₄₁H₄₄N₇O₅⁺[M+H]⁺, 714.34; found, 714.3.

### Example 77: preparation of 3-(5-((4-(4-amino-3-(4-phenoxyphenyl)-1H-pyrazolo[3,4-d]pyrimidin-1-yl)piperidin-1-yl)methyl)-4-fluoro-1-oxoisoindolin-2-yl)piperidine-2,6-dione (GT-03472)

Referring to the methods of Scheme 11 and Example 1, the target compound (GT-03472) was prepared using 3-(4-fluoro-5-(hydroxymethyl)-1-oxoisoindolin-2-yl)piperidine-2,6-dione (GT-03445) and 3-(4-phenoxyphenyl)-1-(piperidin-4-yl)-1H-pyrazolo[3,4-d]pyrimidine-4-amine hydrochloride prepared according to Intermediate Example 2. The target compound (GT-03472) was obtained as a white solid (5 mg, yield 25%). ¹H NMR (400 MHz, MeOD) δ 8.42 (s, 1H), 7.90 - 7.83 (m, 1H), 7.78 (d, *J* = 7.7 Hz, 1H), 7.67 (d, *J* = 7.9 Hz, 2H), 7.42 (t, *J* = 7.9 Hz, 2H), 7.23 - 7.08 (m, 5H), 5.28 - 5.11 (m, 2H), 4.66 - 4.63 (m, 2H), 3.85 - 3.78 (m, 2H), 3.57 - 3.43 (m, 2H), 2.97 - 2.74 (m, 2H), 2.69 - 2.50 (m, 3H), 2.44 - 2.14 (m, 3H). LCMS (ESI) calcd for C₃₆H₃₄FN₈O₄⁺ [M+H]⁺: 661.27, found, 661.3.

### Example 78: preparation of 3-(5-((4-(4-amino-3-(4-phenoxyphenyl)-1H-pyrazolo[3,4-d]pyrimidin-1-yl)piperidin-1-yl)methyl)-6-fluoro-1-oxoisoindolin-2-yl)piperidine-2,6-dione (GT-03605)

The target compound (GT-03605) was prepared using the methods described in Scheme 11 and Example 1 (white solid,34 mg, yield 38%). ¹H NMR (400 MHz, MeOD) δ8.42 (s, 1H), 7.92 (d, *J* = 6.0 Hz, 1H), 7.75 - 7.61 (m, 3H), 7.42 (t, *J* = 7.9 Hz, 2H), 7.22 - 7.09 (m, 5H), 5.26 - 5.13 (m, 2H), 4.70 - 4.56 (m, 4H), 3.84 - 3.72 (m, 2H), 3.58 - 3.45 (m, 2H), 2.97 - 2.35 (m, 8H). LCMS (ESI) m/z: calcd for C₃₆H₃₄FN₈O₄⁺[M+H]⁺, 661.27; found, 661.3.

### Example 79: preparation of 3-(5-((4-(4-amino-3-(4-phenoxyphenyl)-1H-pyrazolo[3,4-d]pyrimidin-1-yl)piperidin-1-yl)methyl)-7-fluoro-1-oxoisoindolin-2-yl)piperidine-2,6-dione (GT-03473)

Referring to the methods of Scheme 11 and Example 1, the target compound (GT-03473) was prepared using 3-(7-fluoro-5-(hydroxymethyl)-1-oxoisoindolin-2-yl)piperidine-2,6-dione (GT-03446) and 3-(4-phenoxyphenyl)-1-(piperidin-4-yl)-1H-pyrazolo[3,4-d]pyrimidine-4-amine hydrochloride prepared according to Intermediate Example 2. The target compound (GT-03473) was obtained as a white solid (5 mg, yield 27%). ¹H NMR (400 MHz, MeOD) δ 8.39 (s, 1H), 7.73 - 7.62 (m, 3H), 7.51 (d, *J* = 9.7 Hz, 1H), 7.42 (t, *J* = 8.0 Hz, 2H), 7.22 - 7.06 (m, 5H), 5.30 - 5.19 (m, 1H), 5.14 (dd, *J* = 13.3, 5.1 Hz, 1H), 4.66 - 4.51 (m, 4H), 3.75 - 3.71 (m, 2H), 3.50 - 3.37 (m, 2H), 2.96 - 2.85 (m, 1H), 2.84 - 2.75 (m, 1H), 2.74 - 2.58 (m, 2H), 2.57 - 2.47 (m, 1H), 2.43 - 2.29 (m, 2H), 2.25 - 2.14 (m, 1H). LCMS (ESI) calcd for C₃₆H₃₄FN₈O₄⁺ [M+H]⁺: 661.27, found, 661.3.

### Example 80: preparation of 3-(4-((4-(4-amino-3-(4-phenoxyphenyl)-1H-pyrazolo[3,4-d]pyrimidin-1-yl)piperidin-1-yl)methyl)-1-oxoisoindolin-2-yl)piperidine-2,6-dione (GT-03641)

The target compound (GT-03641) was prepared using the methods described in Scheme 11 and Example 1 (white solid, 51 mg, yield 57%). ¹H NMR (400 MHz, MeOD) δ 8.43 (s, 1H), 7.96 (d, *J* = 7.4 Hz, 1H), 7.91 (d, *J* = 7.5 Hz, 1H), 7.72 (t, *J* = 7.6 Hz, 1H), 7.69 - 7.63 (m, 2H), 7.42 (t, *J* = 7.8 Hz, 2H), 7.23 - 7.13 (m, 3H), 7.10 (d, *J* = 7.9 Hz, 2H), 5.30 - 5.19 (m, 2H), 4.80 - 4.65 (m, 2H), 4.56 (s, 2H), 3.84 - 3.73 (m, 2H), 3.58 - 3.44 (m, 2H), 3.01 - 2.89 (m, 1H), 2.86 - 2.77 (m, 1H), 2.74 - 2.53 (m, 3H), 2.48 - 2.33 (m, 2H), 2.28 - 2.16 (m, 1H). LCMS (ESI) m/z: calcd for C₃₆H₃₅N₈O₄⁺[M+H]⁺, 634.28; found, 634.3.

### Example 81: preparation of 3-(5-((4-(4-(1,2-bis(4-hydroxyphenyl)but-1-en-1-yl)phenyl)piperazin-1-yl)methyl)-1-oxoisoindolin-2-yl)piperidine-2,6-dione (GT-03551)

The target compound (GT-03551) was prepared using the methods described in Scheme 11 and Example 1 (white solid, 92 mg, yield 52%). ¹H NMR (500 MHz, DMSO) δ 11.26 (d, *J* = 40.9 Hz, 1H), 11.03 (d, *J* = 3.7 Hz, 1H), 9.23 (s, 1H), 7.93 - 7.75 (m, 3H), 7.04 - 6.84 (m, 5H), 6.76 - 6.37 (m, 7H), 5.15 (dt, *J* = 13.1, 5.4 Hz, 1H), 4.56 - 4.45 (m, 3H), 4.38 (dd, *J* = 17.6, 9.6 Hz, 1H), 3.74 (dd, *J* = 62.6, 8.2 Hz, 2H), 3.41 - 3.32 (m, 2H), 3.10 (ddd, *J* = 35.3, 28.5, 19.8 Hz, 4H), 2.98 - 2.85 (m, 1H), 2.59 (t, *J* = 22.4 Hz, 1H), 2.48 - 2.40 (m, 1H), 2.39 - 2.28 (m, 2H), 2.01 (dd, *J* = 10.4, 5.1 Hz, 1H), 0.91 - 0.70 (m, 3H). LCMS (ESI) calcd for C₄₀H₄₁N₄O₅ ⁺ [M+H]⁺: 657.31, found, 657.3.

### Example 82: preparation of 3-(5-((4-(4-(4-chloro-1,2-bis(4-hydroxyphenyl)but-1-en-1-yl)phenyl)piperazin-1-yl)methyl)-1-oxoisoindolin-2-yl)piperidine-2,6-dione (GT-03638)

Referring to the method of Step 2 in Scheme 11 and the method of Example 1, the target compound (GT-03638) was prepared using 4,4'-(4-chloro-1-(4-(piperazin-1-yl)phenyl)but-1-ene-1,2-diyl)diphenol (GT-D-106) prepared according to Intermediate Example 66 and 3-(5-(bromomethyl)-1-oxoisoindolin-2-yl)piperidine-2,6-dione. The target compound (GT-03638) was obtained as a white solid (31 mg, yield 36%). ¹H NMR (500 MHz, DMSO) δ 11.02 (t, *J* = 8.8 Hz, 2H), 7.93 - 7.71 (m, 3H), 7.16 - 6.84 (m, 5H), 6.79 - 6.39 (m, 7H), 5.20 - 5.08 (m, 1H), 4.52 (dd, *J* = 17.7, 10.2 Hz, 3H), 4.43 - 4.30 (m, 1H), 3.90 - 3.76 (m, 1H), 3.70 (d, *J* = 12.2 Hz, 1H), 3.43 (dd, *J* = 14.5, 7.3 Hz, 3H), 3.29 - 2.86 (m, 6H), 2.87 - 2.73 (m, 2H), 2.61 (d, *J* = 15.7 Hz, 1H), 2.45 - 2.36 (m, 1H), 2.01 (dd, *J* = 10.7, 5.0 Hz, 1H). LCMS (ESI) m/z: calcd for C₄₀H₄₀ClN₄O₅⁺[M+H]⁺, 691.27; found, 691.3.

### Example 83: preparation of 3-(5-((4-(4-(4-chloro-1,2-diphenylbut-1-en-1-yl)phenyl)piperazin-1-yl)methyl)-1-oxoisoindolin-2-yl)piperidine-2,6-dione (GT-03637)

Referring to the method of Step 2 in Scheme 11 and the method of Example 1, the target compound (GT-03637) was prepared using 1-(4-(4-chloro-1,2-diphenylbut-1-en-1-yl)phenyl)piperazine (GT-D-104) prepared according to Intermediate Example 65 and 3-(5-(bromomethyl)-1-oxoisoindolin-2-yl)piperidine-2,6-dione. The target compound (GT-03637) was obtained as a white solid (50 mg, yield 55%). ¹H NMR (400 MHz, MeOD) δ 7.92 (d, *J* = 7.8 Hz, 1H), 7.79 (s, 1H), 7.70 (d, *J* = 7.6 Hz, 1H), 7.41 - 7.33 (m, 2H), 7.32 - 7.25 (m, 3H), 7.20 - 7.13 (m, 5H), 6.81 (d, *J* = 8.7 Hz, 2H), 6.67 (d, *J* = 8.8 Hz, 2H), 5.18 (dd, *J* = 13.3, 5.1 Hz, 1H), 4.59 - 4.54 (m, 2H), 4.52 (s, 2H), 3.77 - 3.67 (m, 2H), 3.55 - 3.47 (m, 2H), 3.39 (t, *J* = 7.3 Hz, 2H), 3.29 - 3.19 (m, 2H), 3.04 - 2.94 (m, 2H), 2.89 (t, *J* = 7.3 Hz, 2H), 2.83 - 2.75 (m, 1H), 2.51 (qd, *J* = 13.3, 4.9 Hz, 1H), 2.25 - 2.13 (m, 1H). LCMS (ESI) m/z: calcd for C₄₀H₄₀ClN₄O₃⁺[M+H]⁺, 659.28; found, 659.3.

### Example 84: preparation of 3-(5-((4-(4-((9-cyclopentyl-8-(phenylamino)-9H-purin-2-yl)amino)phenyl)piperazin-1-yl)methyl)-4-fluoro-1-oxoisoindolin-2-yl)piperidine-2,6-dione (GT-03735)

Referring to the methods of Scheme 11 and Example 1, the target compound (GT-03735) was prepared using 3-(4-fluoro-5-(hydroxymethyl)-1-oxoisoindolin-2-yl)piperidine-2,6-dione (GT-03445) prepared according to Intermediate Example 15 and 9-cyclopentyl-N⁸-phenyl-N²-(4-(piperazin-1-yl)phenyl)-9H-purine-2,8-diamine (GT-D-62) prepared according to Intermediate Example 47. The target compound (GT-03735) was obtained as a white solid (43 mg, yield 57.94%). ^{1 1}H NMR (400 MHz, DMSO) δ 11.58 (s, 1H), 11.03 (s, 1H), 10.28 (s, 1H), 10.10 (s, 1H), 8.42 (s, 1H), 8.02 (t, *J* = 6.9 Hz, 1H), 7.84 (d, *J* = 8.0 Hz, 2H), 7.71 (d, *J* = 7.7 Hz, 1H), 7.47 (d, *J* = 8.8 Hz, 2H), 7.39 (t, *J* = 7.9 Hz, 2H), 7.12 (t, *J* = 7.4 Hz, 1H), 7.03 (d, *J* = 8.9 Hz, 2H), 5.24 - 5.13 (m, 2H), 4.64 - 4.56 (m, 3H), 4.47 (d, *J* = 17.6 Hz, 1H), 3.83 - 3.75 (m, 3H), 3.56 - 3.50 (m, 2H), 3.24 (ddd, *J* = 14.0, 12.6, 5.9 Hz, 3H), 2.92 (dd, *J* = 10.6, 6.6 Hz, 1H), 2.61 (d, *J* = 17.3 Hz, 1H), 2.44 (dd, *J* = 13.1, 4.3 Hz, 1H), 2.33 - 2.24 (m, 2H), 2.05 (ddd, *J* = 12.7, 9.5, 4.3 Hz, 3H), 1.92 - 1.84 (m, 2H), 1.65 - 1.57 (m, 2H). LCMS (ESI) calcd for C₄₀H₄₂FN₁₀O₃⁺ [M+H]⁺: 729.33, found, 729.30.

### Example 85: preparation of 3-(5-((4-(4-((9-cyclopentyl-8-(phenylamino)-9H-purin-2-yl)amino)phenyl)piperazin-1-yl)methyl)-6-fluoro-1-oxoisoindolin-2-yl)piperidine-2,6-dione (GT-03736)

The target compound (GT-03736) was prepared using the methods described in Scheme 11 and Example 1 (white solid, 39 mg, yield 52.55%). ¹H NMR (400 MHz, DMSO) δ 11.55 (s, 1H), 11.02 (s, 1H), 10.20 (s, 1H), 10.02 (s, 1H), 8.42 (s, 1H), 8.11 (d, *J* = 6.0 Hz, 1H), 7.83 (d, *J* = 7.9 Hz, 2H), 7.69 (d, *J* = 8.5 Hz, 1H), 7.47 (d, *J* = 8.8 Hz, 2H), 7.39 (t, *J* = 7.9 Hz, 2H), 7.12 (t, *J* = 7.4 Hz, 1H), 7.02 (d, *J* = 8.9 Hz, 2H), 5.17 (td, *J* = 13.4, 6.9 Hz, 2H), 4.56 - 4.48 (m, 3H), 4.38 (d, *J* = 17.4 Hz, 1H), 3.82 - 3.74 (m, 3H), 3.53 - 3.48 (m, 2H), 3.26 (ddd, *J* = 20.5, 9.2, 5.4 Hz, 3H), 2.92 (dd, *J* = 10.6, 6.8 Hz, 1H), 2.61 (d, *J* = 16.5 Hz, 1H), 2.47 - 2.39 (m, 1H), 2.29 (dt, *J* = 15.7, 8.1 Hz, 2H), 2.10 - 2.00 (m, 3H), 1.95 -1.85 (m, 2H), 1.62 (dd, *J* = 12.8, 6.6 Hz, 2H). LCMS (ESI) calcd for C₄₀H₄₂FN₁₀O₃⁺ [M+H]⁺: 729.33, found, 729.30.

### Example 86: preparation of 3-(5-((4-(4-((9-cyclopentyl-8-(phenylamino)-9H-purin-2-yl)amino)phenyl)piperazin-1-yl)methyl)-7-fluoro-1-oxoisoindolin-2-yl)piperidine-2,6-dione (GT-03737)

Referring to the methods of Scheme 11 and Example 1, the target compound (GT-03737) was prepared using 3-(7-fluoro-5-(hydroxymethyl)-1-oxoisoindolin-2-yl)piperidine-2,6-dione (GT-03446) and 9-cyclopentyl-N⁸-phenyl-N²-(4-(piperazin-1-yl)phenyl)-9H-purine-2,8-diamine (GT-D-62) prepared according to Intermediate Example 47. The target compound (GT-03737) was obtained as a white solid (40 mg, yield 53.90%). ¹H NMR (400 MHz, DMSO) δ 11.79 (s, 1H), 11.03 (s, 1H), 10.26 (s, 1H), 10.08 (s, 1H), 8.42 (s, 1H), 7.84 (d, *J* = 8.0 Hz, 2H), 7.74 (d, *J* = 9.7 Hz, 2H), 7.47 (d, *J* = 8.9 Hz, 2H), 7.39 (t, *J* = 7.9 Hz, 2H), 7.12 (t, *J* = 7.4 Hz, 1H), 7.02 (d, *J* = 8.9 Hz, 2H), 5.21 (dd, *J* = 16.8, 8.4 Hz, 1H), 5.11 (dd, *J* = 13.3, 5.0 Hz, 1H), 4.54 (d, *J* = 17.7 Hz, 3H), 4.41 (d, *J* = 18.2 Hz, 1H), 3.77 (d, *J* = 4.9 Hz, 3H), 3.43 (d, *J* = 4.1 Hz, 2H), 3.25 - 3.19 (m, 3H), 2.96 - 2.88 (m, 1H), 2.61 (d, *J* = 16.4 Hz, 1H), 2.44 - 2.36 (m, 1H), 2.33 - 2.23 (m, 2H), 2.04 (ddd, *J* = 12.5, 11.2, 6.3 Hz, 3H), 1.93 - 1.84 (m, 2H), 1.62 (dd, *J* = 12.6, 6.4 Hz, 2H). LCMS (ESI) calcd for C₄₀H₄₂FN₁₀O₃⁺ [M+H]⁺: 729.33, found, 729.30.

### Example 87: preparation of 3-(4-((4-(4-((9-cyclopentyl-8-(phenylamino)-9H-purin-2-yl)amino)phenyl)piperazin-1-yl)methyl)-1-oxoisoindolin-2-yl)piperidine-2,6-dione (GT-03738)

The target compound (GT-03738) was prepared using the methods described in Scheme 11 and Example 1 (white solid, 33 mg, yield 45.59%). ¹H NMR (400 MHz, DMSO) δ 11.57 (s, 1H), 11.07 (s, 1H), 10.23 (s, 1H), 10.05 (s, 1H), 8.42 (s, 1H), 8.07 (d, *J* = 7.6 Hz, 1H), 7.84 (dd, *J* = 7.4, 4.5 Hz, 3H), 7.65 (t, *J* = 7.6 Hz, 1H), 7.47 (d, *J* = 8.8 Hz, 2H), 7.39 (t, *J* = 7.9 Hz, 2H), 7.12 (t, *J* = 7.4 Hz, 1H), 7.02 (d, *J* = 8.9 Hz, 2H), 5.23 - 5.15 (m, 2H), 4.95 (d, *J* = 17.7 Hz, 1H), 4.52 (dd, *J* = 26.4, 15.8 Hz, 3H), 3.78 (d, *J* = 9.9 Hz, 2H), 3.45 (d, *J* = 18.1 Hz, 2H), 3.35 - 3.23 (m, 4H), 3.00 - 2.91 (m, 1H), 2.69 - 2.61 (m, 1H), 2.31 (ddd, *J* = 29.0, 14.6, 6.1 Hz, 3H), 2.10 - 2.01 (m, 3H), 1.90 (dd, *J* = 14.6, 6.4 Hz, 2H), 1.62 (dd, *J* = 12.8, 6.6 Hz, 2H). LCMS (ESI) calcd for C₄₀H₄₃H₁₀O₃⁺ [M+H]⁺: 711.34, found, 711.30.

### Example 88: preparation of 2-(7-(((2-(2,6-dioxopiperidin-3-yl)-1-oxoisoindolin-5-yl)methyl)amino)-1-oxoisoindolin-2-yl)-2-(5-fluoro-2-hydroxyphenyl)-N-(thiazol-2-yl)acetamide (GT-03477)

The target compound (GT-03477) was prepared using the methods described in Scheme 11 and Example 1 (white solid, 5 mg, yield 6%). ¹H NMR (400 MHz, MeOD) δ 7.59 (dd, *J* = 10.6, 8.2 Hz, 1H), 7.50 (d, *J* = 4.8 Hz, 1H), 7.19 - 7.12 (m, 2H), 7.05 - 6.98 (m, 1H), 6.97 - 6.89 (m, 1H), 6.81 (dd, *J* = 8.6, 4.9 Hz, 2H), 6.61 (t, *J* = 7.8 Hz, 1H), 6.58 - 6.52 (m, 1H), 6.32 (s, 1H), 5.51 - 5.39 (m, 1H), 5.25 - 5.10 (m, 2H), 4.69 - 4.62 (m, 3H), 4.38 - 4.30 (m, 2H), 2.97 - 2.86 (m, 1H), 2.83 - 2.74 (m, 1H), 2.49 (dt, *J* = 13.1, 8.5 Hz, 1H), 2.22 - 2.11 (m, 1H). LCMS (ESI) calcd for C₃₃H₂₈FN₆O₆S⁺ [M+H]⁺: 655.18, found, 655.2.

### Example 89: preparation of 2-(6-(4-((2-(2,6-dioxopiperidin-3-yl)-1-oxoisoindolin-5-yl)methyl)piperazin-1-yl)-1-oxoisoindolin-2-yl)-2-(5-fluoro-2-hydroxyphenyl)-N-(thiazol-2-yl)acetamide (GT-03543)

Referring to the method of Step 2 in Scheme 11 and the method of Example 1, the target compound (GT-03543) was prepared using 2-(5-fluoro-2-hydroxyphenyl)-2-(1-oxo-6-(piperazin-1-yl)isoindolin-2-yl)-N-(thiazol-2-yl)acetamide (GT-D-69) prepared according to Intermediate Example 41 and 3-(5-(bromomethyl)-1-oxoisoindolin-2-yl)piperidine-2,6-dione. The target compound (GT-03543) was obtained as a white solid (35 mg, yield 20%). ¹H NMR (400 MHz, MeOD) δ 7.95 (d, *J* = 7.8 Hz, 1H), 7.81 (s, 1H), 7.74 (d, *J* = 7.9 Hz, 1H), 7.47 - 7.41 (m, 2H), 7.38 (d, *J* = 2.2 Hz, 1H), 7.32 (dd, *J* = 8.4, 2.3 Hz, 1H), 7.17 (d, *J* = 3.7 Hz, 1H), 7.07 -7.00 (m, 1H), 6.95 (dd, *J* = 8.9, 3.1 Hz, 1H), 6.91 - 6.86 (m, 1H), 6.45 (s, 1H), 5.19 (dd, *J* = 13.4, 5.1 Hz, 1H), 4.71 - 4.65 (m, 1H), 4.60 - 4.56 (m, 3H), 3.96 - 3.84 (m, 2H), 3.68 - 3.53 (m, 2H), 3.21 - 3.05 (m, 2H), 2.96 - 2.87 (m, 1H), 2.84 - 2.76 (m, 1H), 2.52 (qd, *J* = 13.2, 4.8 Hz, 1H), 2.25 - 2.15 (m, 1H). LCMS (ESI) calcd for C₃₇H₃₅FN₇O₆S ⁺ [M+H]⁺: 724.23, found, 724.2.

### Example 90: preparation of 2-(6-(4-(((2-(2,6-dioxopiperidin-3-yl)-1-oxoisoindolin-5-yl)methyl)(methyl)amino)piperidin-1-yl)-1-oxoisoindolin-2-yl)-2-(5-fluoro-2-hydroxyphenyl)-N-(thiazol-2-yl)acetamide (GT-03745)

Referring to the method of Step 2 in Scheme 11 and the method of Example 1, the target compound (GT-03745) was prepared using 2-(5-fluoro-2-hydroxyphenyl)-2-(6-(4-(methylamino)piperidin-1-yl)-1-oxoisoindolin-2-yl)-N-(thiazol-2-yl)acetamide (GT-D-70) prepared according to Intermediate Example 42 and 3-(5-(bromomethyl)-1-oxoisoindolin-2-yl)piperidine-2,6-dione. The target compound (GT-03745) was obtained as a white solid (67 mg, yield 40%). ¹H NMR (400 MHz, MeOD) δ 7.93 (d, *J* = 7.9 Hz, 1H), 7.85 (s, 1H), 7.75 (d, *J* = 7.9 Hz, 1H), 7.69 (s, 1H), 7.65 - 7.58 (m, 1H), 7.58 - 7.52 (m, 2H), 7.31 (d, *J* = 3.9 Hz, 1H), 7.10 - 6.99 (m, 2H), 6.94 - 6.88 (m, 1H), 6.48 (s, 1H), 5.19 (dd, *J* = 13.3, 5.2 Hz, 1H), 4.79 - 4.70 (m, 2H), 4.59 (q, *J* = 17.6 Hz, 2H), 4.47 - 4.35 (m, 1H), 4.09 - 4.02 (m, 1H), 4.02 - 3.94 (m, 2H), 3.81 - 3.71 (m, 1H), 3.36 - 3.32 (m, 2H), 2.99 - 2.88 (m, 1H), 2.84 (s, 3H), 2.82 - 2.76 (m, 1H), 2.52 (ddd, *J* = 26.5, 13.2, 4.6 Hz, 1H), 2.45 - 2.38 (m, 2H), 2.36 - 2.25 (m, 2H), 2.24 - 2.15 (m, 1H). LCMS (ESI) m/z: calcd for C₃₉H₃₉FN₇O₆S⁺[M+H]⁺, 752.27; found, 752.3.

### Example 91: preparation of 2-(6-(4-(4-((2-(2,6-dioxopiperidin-3-yl)-1-oxoisoindolin-5-yl)methyl)piperazin-1-yl)piperidin-1-yl)-1-oxoisoindolin-2-yl)-2-(5-fluoro-2-hydroxyphenyl)-N-(thiazol-2-yl)acetamide (GT-03546)

Referring to the method of Step 2 in Scheme 11 and the method of Example 1, the target compound (GT-03546) was prepared using 2-(5-fluoro-2-hydroxyphenyl)-2-(1-oxo-6-(4-(piperazin-1-yl)piperidin-1-yl)isoindolin-2-yl)-N-(thiazol-2-yl)acetamide (GT-D-71) prepared according to Intermediate Example 43 and 3-(5-(bromomethyl)-1-oxoisoindolin-2-yl)piperidine-2,6-dione. The target compound (GT-03546) was obtained as a white solid (28 mg, yield 18%). ¹H NMR (400 MHz, MeOD) δ 7.90 (d, *J* = 7.8 Hz, 1H), 7.80 (s, 1H), 7.70 (d, *J* = 7.1 Hz, 1H), 7.57 (s, 1H), 7.52 - 7.44 (m, 3H), 7.22 (d, *J* = 3.7 Hz, 1H), 7.08 - 7.01 (m, 1H), 6.98 (dd, *J* = 8.8, 3.2 Hz, 1H), 6.93 - 6.87 (m, 1H), 6.46 (s, 1H), 5.18 (dd, *J* = 13.4, 5.1 Hz, 1H), 4.72 (d, *J* = 17.5 Hz, 1H), 4.62 - 4.50 (m, 2H), 4.37 (s, 2H), 3.98 - 3.89 (m, 2H), 3.69 - 3.34 (m, 8H), 3.18 - 3.09 (m, 2H), 2.98 - 2.87 (m, 1H), 2.84 - 2.74 (m, 1H), 2.52 (qd, *J* = 13.3, 4.7 Hz, 1H), 2.34 - 2.25 (m, 2H), 2.23 - 2.14 (m, 1H), 2.08 - 1.93 (m, 2H). LCMS (ESI) calcd for C₄₂H₄₄FN₈O₆S ⁺ [M+H]⁺: 807.31, found, 807.3.

### Example 92: preparation of 2-(6-(4-(4-((2-(2,6-dioxopiperidin-3-yl)-1-oxoisoindolin-5-yl)methyl)piperazin-1-yl)phenyl)-1-oxoisoindolin-2-yl)-2-(5-fluoro-2-hydroxyphenyl)-N-(thiazol-2-yl)acetamide (GT-03503)

Referring to the method of Step 2 in Scheme 11 and the method of Example 1, the target compound (GT-03503) was prepared using 2-(5-fluoro-2-hydroxyphenyl)-2-(1-oxo-6-(4-(piperazin-1-yl)phenyl)isoindolin-2-yl)-N-(thiazol-2-yl)acetamide (GT-D-012) prepared according to Intermediate Example 39 and 3-(5-(bromomethyl)-1-oxoisoindolin-2-yl)piperidine-2,6-dione. The target compound (GT-03503) was obtained as a white solid (27 mg, yield 28%). ¹H NMR (400 MHz, MeOD) δ 8.00 - 7.93 (m, 2H), 7.86 - 7.80 (m, 2H), 7.74 (d, *J* = 6.8 Hz, 1H), 7.66 - 7.61 (m, 2H), 7.57 (d, *J* = 8.2 Hz, 1H), 7.46 (d, *J* = 3.7 Hz, 1H), 7.20 - 7.17 (m, 1H), 7.13 (d, *J* = 8.9 Hz, 2H), 7.08 - 6.97 (m, 2H), 6.94 - 6.87 (m, 1H), 6.50 (s, 1H), 5.20 (dd, *J* = 13.4, 5.1 Hz, 1H), 4.65 - 4.51 (m, 4H), 4.01 - 3.87 (m, 2H), 3.45 - 3.36 (m, 2H), 3.21 - 3.08 (m, 2H), 2.99 - 2.87 (m, 1H), 2.85 - 2.75 (m, 1H), 2.53 (qd, *J* = 13.4, 4.9 Hz, 1H), 2.25 - 2.15 (m, 1H). LCMS (ESI) calcd for C₄₃H₃₉FN₇O₆S ⁺ [M+H]⁺: 800.27, found, 800.3.

### Example 93: preparation of 2-(6-(6-(4-((2-(2,6-dioxopiperidin-3-yl)-1-oxoisoindolin-5-yl)methyl)piperazin-1-yl)pyridazin-3-yl)-1-oxoisoindolin-2-yl)-2-(5-fluoro-2-hydroxyphenyl)-N-(thiazol-2-yl)acetamide (GT-03687)

Referring to the method of Step 2 in Scheme 11 and the method of Example 1, the target compound (GT-03687) was prepared using 2-(5-fluoro-2-hydroxyphenyl)-2-(1-oxo-6-(6-(piperazin-1-yl)pyridazin-3-yl)isoindolin-2-yl)-N-(thiazol-2-yl)acetamide (GT-D-014) prepared according to Intermediate Example 40 and 3-(5-(bromomethyl)-1-oxoisoindolin-2-yl)piperidine-2,6-dione. The target compound (GT-03687) was obtained as a white solid (21 mg, yield 28.01%). ¹H NMR (400 MHz, DMSO) δ 12.63 (s, 1H), 11.48 (s, 1H), 11.01 (s, 1H), 10.05 (s, 1H), 8.36 - 8.27 (m, 3H), 7.89 (s, 1H), 7.84 (d, *J* = 7.8 Hz, 1H), 7.77 (d, *J* = 7.6 Hz, 1H), 7.71 (d, *J* = 8.0 Hz, 1H), 7.58 (d, *J* = 9.5 Hz, 1H), 7.49 (d, *J* = 3.6 Hz, 1H), 7.27 (d, *J* = 3.6 Hz, 1H), 7.15 - 7.09 (m, 1H), 6.97 - 6.92 (m, 1H), 6.91 - 6.85 (m, 1H), 6.34 (s, 1H), 5.15 (dd, *J* = 13.3, 5.1 Hz, 1H), 4.68 (d, *J* = 17.9 Hz, 1H), 4.53 (dd, *J* = 19.6, 5.0 Hz, 5H), 4.37 (s, 1H), 4.08 (s, 1H), 3.49 (dd, *J* = 24.7, 11.4 Hz, 4H), 3.20 (ddd, *J* = 7.9, 6.2, 2.6 Hz, 2H), 2.97 - 2.88 (m, 1H), 2.64 - 2.58 (m, 1H), 2.46 - 2.38 (m, 1H), 2.02 (dd, *J* = 12.0, 6.2 Hz, 1H). LCMS (ESI) calcd for C₄₁H₃₇FN₉O₆S⁺ [M+H]⁺: 802.25, found, 802.30.

### Example 94: preparation of 3-(5-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)-1-oxoisoindolin-2-yl)piperidine-2,6-dione (GT-03475)

Referring to the method of Step 2 in Scheme 11 and the method of Example 1, the target compound (GT-03475) was prepared using N-(2-(1-(cyclopropylsulfonyl)-1H-pyrazol-4-yl)pyrimidin-4-yl)-1-isopropyl-3-(piperazin-1-yl)-1H-pyrazolo[4,3-c]pyridin-6-amine (GT-D-18) prepared according to Intermediate Example 54 and 3-(5-(bromomethyl)-1-oxoisoindolin-2-yl)piperidine-2,6-dione. The target compound (GT-03475) was obtained as a white solid (32.8 mg, yield 40%). ¹H NMR (400 MHz, MeOD) δ 9.38 (s, 1H), 8.99 (s, 1H), 8.63 - 8.53 (m, 2H), 7.97 - 7.89 (m, 2H), 7.80 (d, *J* = 8.1 Hz, 1H), 7.62 (s, 1H), 7.23 (d, *J* = 5.8 Hz, 1H), 5.19 (dd, *J* = 13.3, 5.1 Hz, 1H), 4.68 - 4.53 (m, 4H), 4.34 - 4.26 (m, 1H), 3.92 - 3.34 (m, 8H), 3.14 - 3.05 (m, 1H), 2.98 - 2.87 (m, 1H), 2.84 - 2.76 (m, 1H), 2.54 (qd, *J* = 13.2, 4.6 Hz, 1H), 2.25 - 2.16 (m, 1H), 1.55 (d, *J* = 6.6 Hz, 6H), 1.49 - 1.43 (m, 2H), 1.31 - 1.24 (m, 2H). LCMS (ESI) calcd for C₃₇H₄₁N₁₂O₅⁺ [M+H]⁺: 765.30, found, 765.3.

### Example 95: preparation of 3-(5-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)-1-oxoisoindolin-2-yl)piperidine-2,6-dione (GT-03548)

Referring to the method of Step 2 in Scheme 11 and the method of Example 1, the target compound (GT-03548) was prepared using N-(2-(1-(cyclopropylsulfonyl)-1H-pyrazol-4-yl)pyrimidin-4-yl)-1-isopropyl-3-(4-(methylamino)piperidin-1-yl)-1H-pyrazolo[4,3-c]pyridin-6-amine (GT-D-72) prepared according to Intermediate Example 55 and 3-(5-(bromomethyl)-1-oxoisoindolin-2-yl)piperidine-2,6-dione. The target compound (GT-03548) was obtained as a white solid (116 mg, yield 74%). ¹H NMR (400 MHz, MeOD) δ 9.40 (s, 1H), 9.01 (s, 1H), 8.62 - 8.57 (m, 2H), 7.92 (d, *J* = 7.9 Hz, 1H), 7.89 (s, 1H), 7.77 (d, *J* = 7.7 Hz, 1H), 7.57 (s, 1H), 7.16 (d, *J* = 6.2 Hz, 1H), 5.19 (dd, *J* = 13.3, 5.1 Hz, 1H), 4.66 - 4.53 (m, 2H), 4.46 - 4.34 (m, 3H), 3.79 - 3.69 (m, 1H), 3.27 - 3.18 (m, 2H), 3.14 - 3.07 (m, 1H), 2.98 - 2.87 (m, 1H), 2.83 (s, 3H), 2.82 - 2.76 (m, 1H), 2.53 (qd, *J* = 13.2, 4.6 Hz, 1H), 2.44 - 2.35 (m, 2H), 2.25 - 2.11 (m, 3H), 1.56 (d, *J* = 6.6 Hz, 6H), 1.50 - 1.44 (m, 2H), 1.32 - 1.25 (m, 2H). LCMS (ESI) calcd for C₃₉H₄₅N₁₂O₅S ⁺ [M+H]⁺: 793.34, found, 793.4.

### Example 96: preparation of 3-(5-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)-1-oxoisoindolin-2-yl)piperidine-2,6-dione (GT-03547)

Referring to the method of Step 2 in Scheme 11 and the method of Example 1, the target compound (GT-03547) was prepared using N-(2-(1-(cyclopropylsulfonyl)-1H-pyrazol-4-yl)pyrimidin-4-yl)-1-isopropyl-3-(4-(piperazin-1-yl)piperidin-1-yl)-1H-pyrazolo[4,3-c]pyridin-6-amine (GT-D-73) prepared according to Intermediate Example 56 and 3-(5-(bromomethyl)-1-oxoisoindolin-2-yl)piperidine-2,6-dione. The target compound (GT-03547) was obtained as a white solid (28 mg, yield 18%). ¹H NMR (400 MHz, MeOD) δ 9.33 (s, 1H), 8.98 (s, 1H), 8.66 - 8.56 (m, 2H), 7.96 - 7.84 (m, 2H), 7.76 (s, 1H), 7.49 (s, 1H), 7.13 (s, 1H), 5.23 - 5.15 (m, 1H), 4.57 (d, *J* = 7.9 Hz, 1H), 4.53 - 4.42 (m, 2H), 4.34 (d, *J* = 11.4 Hz, 2H), 3.99 - 3.32 (m, 12H), 3.22 - 3.17 (m, 1H), 3.13 - 3.01 (m, 1H), 2.97 - 2.86 (m, 1H), 2.85 - 2.75 (m, 1H), 2.57 - 2.46 (m, 1H), 2.41 - 2.31 (m, 2H), 2.24 - 2.14 (m, 1H), 2.12 - 1.99 (m, 2H), 1.55 (d, *J* = 6.3 Hz, 4H), 1.50 - 1.43 (m, 2H), 1.40 - 1.33 (m, 2H), 1.31 - 1.24 (m, 2H). LCMS (ESI) calcd for C₄₂H₅₀N₁₃O₅S ⁺ [M+H]⁺: 848.38, found, 848.4.

### Example 97: preparation of 3-(5-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)-1-oxoisoindolin-2-yl)piperidine-2,6-dione (GT-03506)

Referring to the method of Step 2 in Scheme 11 and the method of Example 1, the target compound (GT-03506) was prepared using 1-isopropyl-N-(2-(4-methoxypiperidin-1-yl)pyrimidin-4-yl)-3-(4-(methylamino)piperidin-1-yl)-1H-pyrazolo[4,3-c]pyridin-6-amine (GT-D-74) prepared according to Intermediate Example 57 and 3-(5-(bromomethyl)-1-oxoisoindolin-2-yl)piperidine-2,6-dione. The target compound (GT-03506) was obtained as a white solid (76 mg, yield 45%). ¹H NMR (400 MHz, MeOD) δ 9.44 (s, 1H), 8.04 (d, *J* = 7.1 Hz, 1H), 7.94 - 7.89 (m, 2H), 7.81 (s, 1H), 7.79 - 7.75 (m, 1H), 6.59 (d, *J* = 7.1 Hz, 1H), 5.18 (dd, *J* = 13.3, 5.1 Hz, 1H), 4.80 - 4.77 (m, 1H), 4.61- 4.51 (m, 2H), 4.46 - 4.35 (m, 3H), 3.96 - 3.85 (m, 2H), 3.83 - 3.67 (m, 4H), 3.65 - 3.57 (m, 1H), 3.39 (s, 3H), 3.27 - 3.19 (m, 2H), 2.94 - 2.86 (m, 1H), 2.82 (s, 3H), 2.80 - 2.75 (m, 1H), 2.59 - 2.46 (m, 1H), 2.43 - 2.35 (m, 2H), 2.23 - 2.13 (m, 3H), 2.05 - 1.95 (m, 2H), 1.82 - 1.73 (m, 2H), 1.56 (d, *J* = 6.6 Hz, 6H). LCMS (ESI) calcd for C₃₉H₅₀N₁₁O₄⁺ [M+H]⁺: 736.40, found, 736.4.

### Example 98: preparation of 3-(5-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)-1-oxoisoindolin-2-yl)piperidine-2,6-dione (GT-03504)

Referring to the method of Step 2 in Scheme 11 and the method of Example 1, the target compound (GT-03504) was prepared using 1-isopropyl-N-(2-(4-methoxypiperidin-1-yl)pyrimidin-4-yl)-3-(4-(piperazin-1-yl)piperidin-1-yl)-1H-pyrazolo[4,3-c]pyridin-6-amine (GT-D-75) prepared according to Intermediate Example 58 and 3-(5-(bromomethyl)-1-oxoisoindolin-2-yl)piperidine-2,6-dione. The target compound (GT-03504) was obtained as a white solid (73 mg, yield 47%). ¹H NMR (400 MHz, MeOD) δ 9.37 (s, 1H), 8.03 (d, *J* = 7.1 Hz, 1H), 7.96 - 7.89 (m, 2H), 7.84 - 7.75 (m, 2H), 6.58 (d, *J* = 7.1 Hz, 1H), 5.18 (dd, *J* = 13.3, 5.1 Hz, 1H), 4.66 - 4.52 (m, 4H), 4.35 (d, *J* = 13.0 Hz, 2H), 4.14 - 3.53 (m, 16H), 3.39 (s, 3H), 3.22 - 3.18 (m, 1H), 2.99 - 2.86 (m, 1H), 2.83 - 2.74 (m, 1H), 2.53 (qd, *J* = 13.3, 4.7 Hz, 1H), 2.40 - 2.31 (m, 2H), 2.24 - 2.15 (m, 1H), 2.13 - 1.94 (m, 4H), 1.83 - 1.68 (m, 2H), 1.55 (d, *J* = 6.5 Hz, 6H). LCMS (ESI) calcd for C₄₂H₅₅N₁₂O₄⁺ [M+H]⁺: 791.45, found, 791.4.

### Example 99: preparation of 3-(5-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)-1-oxoisoindolin-2-yl)piperidine-2,6-dione (GT-03957)

Referring to the method of Step 2 in Scheme 11 and the method of Example 1, the target compound (GT-03957) was prepared using (6-((5-bromo-2-((2-methoxy-5-methyl-4-(4-(piperazin-1-yl)piperidin-1-yl)phenyl)amino)pyrimidin-4-yl)amino)quinoxalin-5-yl)dimethylphosphine oxide (GT-S-52) prepared according to Intermediate Example 81 and 3-(5-(bromomethyl)-1-oxoisoindolin-2-yl)piperidine-2,6-dione. The target compound (GT-03957) was obtained as a white solid (10 mg, yield 14%). ¹H NMR (500 MHz, DMSO)δ 12.92 (s, 1H), 11.01 (d, *J* = 11.7 Hz, 1H), 8.85 (dt, *J* = 90.9, 31.0 Hz, 4H), 8.33 (s, 1H), 7.83 (ddd, *J* = 43.8, 32.1, 8.1 Hz, 4H), 7.38 (s, 1H), 6.79 (s, 1H), 5.17 - 5.11 (m, 1H), 4.61 - 4.29 (m, 4H), 3.78 (d, *J* = 13.7 Hz, 3H), 3.75 - 3.59 (m, 6H), 3.21 (t, *J* = 22.5 Hz, 6H), 2.97 - 2.90 (m, 1H), 2.75 (s, 1H), 2.64 - 2.59 (m, 1H), 2.42 (dd, *J* = 13.4, 8.3 Hz, 1H), 2.19 (s, 2H), 2.13 - 2.08 (m, 3H), 2.05 (t, *J* = 12.7 Hz, 7H), 1.92 (d, *J* = 8.8 Hz, 2H). LCMS (ESI) m/z: calcd for C₄₅H₅₂BrN₁₁O₅P⁺[M+H]⁺, 936.31; found, 936.3.

### Example 100: preparation of N-(2-chloro-6-methylphenyl)-2-((6-(4-(((2-(2,6-dioxopiperidin-3-yl)-1-oxoisoindolin-5-yl)methyl)(methyl)amino)piperidin-1-yl)-2-methylpyrimidin-4-yl)amino)thiazole-5-carboxamide (GT-03545)

The target compound (GT-03545) was prepared using the methods described in Scheme 11 and Example 1 (white solid, 100 mg, yield 51%). ¹H NMR (400 MHz, MeOD) δ 8.23 (s, 1H), 7.93 (d, *J* = 7.9 Hz, 1H), 7.86 (s, 1H), 7.74 (d, *J* = 7.8 Hz, 1H), 7.37 (dd, *J* = 7.0, 2.3 Hz, 1H), 7.29 - 7.20 (m, 2H), 6.44 (s, 1H), 5.19 (dd, *J* = 13.3, 5.1 Hz, 1H), 4.75 (d, *J* = 13.7 Hz, 1H), 4.61 - 4.52 (m, 2H), 4.39 (d, *J* = 12.9 Hz, 1H), 3.87 - 3.76 (m, 1H), 3.30 - 3.11 (m, 4H), 2.99 - 2.87 (m, 1H), 2.83 - 2.80 (m, 1H), 2.79 (s, 3H), 2.64 (s, 3H), 2.52 (ddd, *J* = 26.2, 13.2, 4.7 Hz, 1H), 2.43 - 2.35 (m, 2H), 2.32 (s, 3H), 2.24 - 2.14 (m, 1H), 2.08 - 1.92 (m, 2H). LCMS (ESI) calcd for C₃₆H₃₉ClN₉O₄S⁺ [M+H]⁺: 728.25, found, 728.3.

### Example 101: preparation of N-(2-chloro-6-methylphenyl)-2-((6-(4-(4-((2-(2,6-dioxopiperidin-3-yl)-1-oxoisoindolin-5-yl)methyl)piperazin-1-yl)piperidin-1-yl)-2-methylpyrimidin-4-yl)amino)thiazole-5-carboxamide (GT-03476)

The target compound (GT-03476) was prepared using the methods described in Scheme 11 and Example 1 (white solid, 32.1 mg, yield 39%). ¹H NMR (400 MHz, MeOD) δ 8.22 (s, 1H), 7.87 (d, *J* = 7.8 Hz, 1H), 7.75 (s, 1H), 7.66 (d, *J* = 7.9 Hz, 1H), 7.41 - 7.33 (m, 1H), 7.26 (d, *J* = 7.2 Hz, 2H), 6.43 (s, 1H), 5.17 (dd, *J* = 13.4, 5.3 Hz, 1H), 4.60 - 4.47 (m, 3H), 4.23 (s, 2H), 3.90 - 3.38 (m, 8H), 3.26 - 3.14 (m, 4H), 2.94 - 2.85 (m, 1H), 2.84 - 2.75 (m, 1H), 2.63 (s, 3H), 2.55 - 2.46 (m, 1H), 2.37 - 2.27 (m, 5H), 2.22 - 2.15 (m, 1H), 1.89 - 1.76 (m, 2H). LCMS (ESI) calcd for C₃₉H₄₄ClN₁₀O₄S⁺ [M+H]⁺: 783.30, found, 783.3.

### Example 102: preparation of 4-((((2-(2,6-dioxopiperidin-3-yl)-1-oxoisoindolin-5-yl)methyl)(methyl)amino)methyl)-N-(4-methyl-3-((4-(pyridin-3-yl)pyrimidin-2-yl)amino)phenyl)benzamide (GT-03990)

The target compound (GT-03990) was prepared using the methods described in Scheme 11 and Example 1 (white solid,15 mg, yield 29.9%). ¹H NMR (400 MHz, MeOD) δ 9.55 (s, 1H), 9.19 (d, *J* = 8.2 Hz, 1H), 8.83 (d, *J* = 5.4 Hz, 1H), 8.49 (d, *J* = 5.3 Hz, 1H), 8.20 (s, 1H), 8.05 (dd, *J* = 8.2, 5.7 Hz, 1H), 7.97 (d, *J* = 8.2 Hz, 2H), 7.82 (d, *J* = 7.8 Hz, 1H), 7.67 (s, 1H), 7.59 (d, *J* = 8.1 Hz, 3H), 7.46 (d, *J* = 5.4 Hz, 1H), 7.29 - 7.17 (m, 2H), 5.07 (dd, *J* = 13.3, 5.1 Hz, 1H), 4.46 (q, *J* = 17.4 Hz, 6H), 2.86 - 2.62 (m, 5H), 2.40 (dt, *J* = 13.7, 8.7 Hz, 1H), 2.23 (s, 3H), 2.13 - 2.02 (m, 1H). LCMS (ESI) calcd for C₃₉H₃₇N₈O₄⁺ [M+H]⁺: 681.29, found, 681.3.

### Example 103: preparation of 4-(4-((2-(2,6-dioxopiperidin-3-yl)-1-oxoisoindolin-5-yl)methyl)piperazin-1-yl)-N-(4-methyl-3-((4-(pyridin-3-yl)pyrimidin-2-yl)amino)phenyl)benzamide (GT-03549)

Referring to the method of Step 2 in Scheme 11 and the method of Example 1, the target compound (GT-03549) was prepared using N-(4-methyl-3-((4-(pyridin-3-yl)pyrimidin-2-yl)amino)phenyl)-4-(piperazin-1-yl)benzamide (CAS NO.: 2296714-70-8) and 3-(5-(bromomethyl)-1-oxoisoindolin-2-yl)piperidine-2,6-dione. The target compound (GT-03549) was obtained as a white solid (91 mg, yield 55%). ¹H NMR (400 MHz, MeOD) δ 9.68 (d, *J* = 1.7 Hz, 1H), 9.35 (d, *J* = 8.2 Hz, 1H), 8.98 (d, *J* = 5.7 Hz, 1H), 8.59 (d, *J* = 5.7 Hz, 1H), 8.25 - 8.16 (m, 2H), 7.98 - 7.90 (m, 3H), 7.88 (s, 1H), 7.76 (d, *J* = 7.9 Hz, 1H), 7.65 (d, *J* = 5.7 Hz, 1H), 7.39 - 7.29 (m, 2H), 7.11 (d, *J* = 9.0 Hz, 2H), 5.19 (dd, *J* = 13.3, 5.2 Hz, 1H), 4.65 - 4.53 (m, 2H), 4.14 - 3.99 (m, 2H), 3.68 - 3.53 (m, 2H), 3.39 - 3.31 (m, 4H), 3.29 - 3.14 (m, 2H), 2.98 - 2.87 (m, 1H), 2.84 - 2.75 (m, 1H), 2.53 (qd, *J* = 13.3, 4.9 Hz, 1H), 2.33 (s, 3H), 2.24 - 2.15 (m, 1H). LCMS (ESI) calcd for C₄₁H₄₀N₉O₄⁺ [M+H]⁺: 722.32, found, 722.3.

### Example 104: preparation of 4-(4-(((2-(2,6-dioxopiperidin-3-yl)-1-oxoisoindolin-5-yl)methyl)(methyl)amino)piperidin-1-yl)-N-(4-methyl-3-((4-(pyridin-3-yl)pyrimidin-2-yl)amino)phenyl)benzamide (GT-03640)

The target compound (GT-03640) was prepared using the methods described in Scheme 11 and Example 1 (white solid, 16 mg, yield 18%). ¹H NMR (400 MHz, MeOD) δ 9.72 (s, 1H), 9.41 (d, *J* = 8.3 Hz, 1H), 9.05 (d, *J* = 5.5 Hz, 1H), 8.60 (d, *J* = 6.0 Hz, 1H), 8.27 (dd, *J* = 8.2, 5.9 Hz, 1H), 8.19 (s, 1H), 7.99 (d, *J* = 8.8 Hz, 2H), 7.92 (d, *J* = 7.8 Hz, 1H), 7.87 (s, 1H), 7.78 - 7.74 (m, 2H), 7.44 (d, *J* = 8.3 Hz, 1H), 7.40 - 7.29 (m, 3H), 5.18 (dd, *J* = 13.4, 5.1 Hz, 1H), 4.76 - 4.71 (m, 1H), 4.63 - 4.55 (m, 2H), 4.42 (d, *J* = 11.7 Hz, 1H), 4.13 - 4.03 (m, 2H), 3.80 - 3.71 (m, 1H), 3.27 - 3.19 (m, 2H), 2.96 - 2.87 (m, 1H), 2.82 (s, 3H), 2.79 - 2.73 (m, 1H), 2.57 - 2.49 (m, 1H), 2.43 - 2.37 (m, 2H), 2.36 (s, 3H), 2.25 - 2.16 (m, 3H). LCMS (ESI) m/z: calcd for C₄₃H₄₄N₉O₄⁺[M+H]⁺, 750.35; found, 750.4.

### Example 105: preparation of 4-(4-(4-((2-(2,6-dioxopiperidin-3-yl)-1-oxoisoindolin-5-yl)methyl)piperazin-1-yl)piperidin-1-yl)-N-(4-methyl-3-((4-(pyridin-3-yl)pyrimidin-2-yl)amino)phenyl)benzamide (GT-03689)

The target compound (GT-03689) was prepared using the methods described in Scheme 11 and Example 1 (white solid, 21 mg, yield 28.06%). ¹H NMR (400 MHz, DMSO) δ 11.00 (s, 1H), 9.96 (s, 1H), 9.44 (s, 1H), 9.14 (s, 1H), 8.94 (d, *J* = 8.2 Hz, 1H), 8.89 (d, *J* = 5.0 Hz, 1H), 8.59 (d, *J* = 5.1 Hz, 1H), 8.11 (s, 1H), 7.94 - 7.87 (m, 4H), 7.81 (s, 2H), 7.55 (d, *J* = 5.2 Hz, 1H), 7.46 (dd, *J* = 8.3, 1.9 Hz, 1H), 7.19 (d, *J* = 8.6 Hz, 1H), 7.05 (d, *J* = 8.9 Hz, 2H), 5.16 - 5.11 (m, 1H), 4.48 (s, 2H), 4.39 (s, 1H), 4.07 (d, *J* = 11.6 Hz, 3H), 3.75 - 3.66 (m, 3H), 3.49 (ddd, *J* = 17.8, 12.4, 7.8 Hz, 3H), 2.99 - 2.78 (m, 4H), 2.60 (dd, *J* = 16.1, 1.8 Hz, 1H), 2.48 - 2.37 (m, 2H), 2.25 - 2.12 (m, 6H), 2.01 (dd, *J* = 9.0, 3.4 Hz, 1H), 1.82 - 1.72 (m, 2H). LCMS (ESI) calcd for C₄₆H₄₉N₁₀O₄⁺ [M+H]⁺: 805.39, found, 805.40.

### Example 106: preparation of 4-((2,4-dichloro-5-methoxyphenyl)amino)-7-((2-(2,6-dioxopiperidin-3-yl)-1-oxoisoindolin-5-yl)methoxy)-6-methoxyquinoline-3-carbonitrile (GT-03528)

According to the method of Scheme 12, the target compound (GT-03528) was prepared using 4-((2,4-dichloro-5-methoxyphenyl)amino)-7-hydroxy-6-methoxyquinoline-3-carbonitrile (CAS NO.: 1460227-29-5) and 3-(5-(bromomethyl)-1-oxoisoindolin-2-yl)piperidine-2,6-dione. The target compound (GT-03528) was obtained as a white solid (53 mg, yield 30%). ¹H NMR (400 MHz, MeOD) δ 8.83 (s, 1H), 8.00 (s, 1H), 7.88 (d, *J* = 7.9 Hz, 1H), 7.77 (s, 1H), 7.70 (d, *J* = 7.2 Hz, 1H), 7.67 (s, 1H), 7.40 (d, *J* = 8.3 Hz, 2H), 5.55 (s, 2H), 5.18 (dd, *J* = 13.3, 5.2 Hz, 1H), 4.54 (q, *J* = 17.2 Hz, 2H), 4.10 (s, 3H), 3.93 (s, 3H), 2.98 - 2.86 (m, 1H), 2.84 - 2.74 (m, 1H), 2.57 - 2.43 (m, 1H), 2.24 - 2.14 (m, 1H). LCMS (ESI) calcd for C₃₂H₂₆Cl₂N₅O₆⁺ [M+H]⁺: 646.13, found, 646.2.

### Example 107: preparation of N-(4-((4-((2-(2,6-dioxopiperidin-3-yl)-1-oxoisoindolin-5-yl)methyl)piperazin-1-yl)methyl)-3-(trifluoromethyl)phenyl)-3-(imidazo[1,2-b]pyridazin-3-ylethynyl)-4-methylbenzamide (GT-03804)

Referring to the method of Step 2 in Scheme 11 and the method of Example 1, the target compound (GT-03804) was prepared using 3-(imidazo[1,2-b]pyridazin-3-ylethynyl)-4-methyl-N-(4-(piperazin-1-ylmethyl)-3-(trifluoromethyl)phenyl)benzamide hydrochloride (GT-D-109) prepared from Intermediate Example 68 and 3-(5-(bromomethyl)-1-oxoisoindolin-2-yl)piperidine-2,6-dione. The target compound (GT-03804) was obtained as a white solid (26 mg, yield 31%). ¹H NMR (400 MHz, MeOD) δ 8.95 (dd, *J* = 4.5, 1.4 Hz, 1H), 8.44 (s, 1H), 8.38 (dd, *J* = 9.3, 1.4 Hz, 1H), 8.25 - 8.17 (m, 2H), 8.05 (d, *J* = 7.8 Hz, 1H), 7.96 (dd, *J* = 8.0, 1.9 Hz, 1H), 7.91 (d, *J* = 7.8 Hz, 1H), 7.83 - 7.75 (m, 3H), 7.70 (d, *J* = 7.7 Hz, 1H), 7.53 (d, *J* = 8.1 Hz, 1H), 5.18 (dd, *J* = 13.3, 5.2 Hz, 1H), 4.56 (q, *J* = 17.5 Hz, 2H), 4.45 (s, 2H), 3.95 (s, 2H), 3.75 - 3.32 (m, 8H), 2.93 - 2.87 (m, 1H), 2.83 - 2.76 (m, 1H), 2.68 (s, 3H), 2.58 - 2.45 (m, 1H), 2.23 - 2.15 (m, 1H). LCMS (ESI) m/z: calcd for C₄₂H₃₈F₃N₈O₄⁺[M+H]⁺, 775.30; found, 775.3.

### Example 108: preparation of N-(4-((4-((2-(2,6-dioxopiperidin-3-yl)-4-fluoro-1-oxoisoindolin-5-yl)methyl)piperazin-1-yl)methyl)-3-(trifluoromethyl)phenyl)-3-(imidazo[1,2-b]pyridazin-3-ylethynyl)-4-methylbenzamide (GT-03933)

Referring to the methods of Scheme 11 and Example 1, the target compound (GT-03933) was prepared using 3-(imidazo[1,2-b]pyridazin-3-ylethynyl)-4-methyl-N-(4-(piperazin-1-ylmethyl)-3-(trifluoromethyl)phenyl)benzamide hydrochloride (GT-D-109) prepared from Intermediate Example 68 and 3-(4-fluoro-5-(hydroxymethyl)-1-oxoisoindolin-2-yl)piperidine-2,6-dione (GT-03445). The target compound (GT-03933) was obtained as a white solid (53 mg, yield 65%). ¹H NMR (400 MHz, DMSO) δ 11.03 (s, 1H), 10.71 (s, 1H), 8.75 (dd, *J* = 4.4, 1.3 Hz, 1H), 8.33 - 8.26 (m, 3H), 8.23 (d, *J* = 1.5 Hz, 1H), 8.20 - 8.13 (m, 1H), 8.01 - 7.94 (m, 1H), 7.86 (s, 1H), 7.74 - 7.67 (m, 1H), 7.64 - 7.60 (m, 1H), 7.57 (d, *J* = 8.2 Hz, 1H), 7.43 (dd, *J* = 9.2, 4.5 Hz, 1H), 5.19 - 5.09 (m, 2H), 4.92 (s, 1H), 4.65 - 4.49 (m, 4H), 3.48 - 3.06 (m, 6H), 2.97 - 2.86 (m, 2H), 2.69 - 2.64 (m, 1H), 2.61 (s, 3H), 2.60 - 2.57 (m, 1H), 2.46 - 2.40 (m, 1H), 2.04 - 1.97 (m, 1H). LCMS (ESI) m/z: calcd for C₄₂H₃₇F₄N₈O₄⁺[M+H]⁺, 793.29; found, 793.3.

### Example 109: preparation of N-(4-((4-((2-(2,6-dioxopiperidin-3-yl)-6-fluoro-1-oxoisoindolin-5-yl)methyl)piperazin-1-yl)methyl)-3-(trifluoromethyl)phenyl)-3-(imidazo[1,2-b]pyridazin-3-ylethynyl)-4-methylbenzamide (GT-03934)

The target compound (GT-03934) was prepared using the methods described in Scheme 11 and Example 1 (white solid,44 mg, yield 54%). ¹H NMR (400 MHz, DMSO) δ 11.01 (s, 1H), 10.70 (s, 1H), 8.75 (dd, *J* = 4.4, 1.4 Hz, 1H), 8.33 - 8.26 (m, 3H), 8.23 (d, *J* = 1.7 Hz, 1H), 8.17 (d, *J* = 7.9 Hz, 1H), 8.01 - 7.91 (m, 3H), 7.66 (d, *J* = 8.4 Hz, 1H), 7.57 (d, *J* = 8.4 Hz, 1H), 7.43 (dd, *J* = 9.2, 4.5 Hz, 1H), 5.14 (dd, *J* = 13.3, 5.1 Hz, 1H), 4.90 (s, 1H), 4.58 - 4.43 (m, 5H), 3.43 - 3.11 (m, 6H), 2.97 - 2.86 (m, 2H), 2.71 - 2.64 (m, 1H), 2.62 (s, 3H), 2.59 - 2.55 (m, 1H), 2.45 - 2.37 (m, 1H), 2.06 - 1.97 (m, 1H). LCMS (ESI) m/z: calcd for C₄₂H₃₇F₄N₈O₄⁺[M+H]⁺, 793.29; found, 793.3.

### Example 110: preparation of N-(4-((4-((2-(2,6-dioxopiperidin-3-yl)-7-fluoro-1-oxoisoindolin-5-yl)methyl)piperazin-1-yl)methyl)-3-(trifluoromethyl)phenyl)-3-(imidazo[1,2-b]pyridazin-3-ylethynyl)-4-methylbenzamide (GT-03935)

Referring to the methods of Scheme 11 and Example 1, the target compound (GT-03935) was prepared using 3-(imidazo[1,2-b]pyridazin-3-ylethynyl)-4-methyl-N-(4-(piperazin-1-ylmethyl)-3-(trifluoromethyl)phenyl)benzamide hydrochloride (GT-D-109) prepared from Intermediate Example 68 and 3-(7-fluoro-5-(hydroxymethyl)-1-oxoisoindolin-2-yl) piperidine-2,6-dione (GT-03446). The target compound (GT-03935) was obtained as a white solid (62 mg, yield 76%). ¹H NMR (400 MHz, DMSO) δ 11.01 (s, 1H), 10.68 (s, 1H), 8.75 (d, *J* = 4.2 Hz, 1H), 8.33 - 8.26 (m, 3H), 8.23 (s, 1H), 8.16 (d, *J* = 7.8 Hz, 1H), 7.97 (d, *J* = 8.0 Hz, 1H), 7.70 - 7.51 (m, 4H), 7.46 - 7.37 (m, 2H), 3.39 - 3.02 (m, 6H), 2.97 - 2.87 (m, 2H), 2.65 - 2.60 (m, 4H), 2.60 - 2.57 (m, 1H), 2.41 - 2.34 (m, 1H), 2.04 - 1.98 (m, 1H). LCMS (ESI) m/z: calcd for C₄₂H₃₇F₄N₈O₄⁺[M+H]⁺, 793.29; found, 793.3.

### Example 111: preparation of N-(4-((4-((2-(2,6-dioxopiperidin-3-yl)-1-oxoisoindolin-4-yl)methyl)piperazin-1-yl)methyl)-3-(trifluoromethyl)phenyl)-3-(imidazo[1,2-b]pyridazin-3-ylethynyl)-4-methylbenzamide (GT-03939)

The target compound (GT-03939) was prepared using the methods described in Scheme 11 and Example 1 (white solid, 40 mg, yield 50%). ¹H NMR (400 MHz, DMSO) δ 11.05 (s, 1H), 10.68 (s, 1H), 8.77 - 8.73 (m, 1H), 8.31 - 8.26 (m, 3H), 8.23 (d, *J* = 1.7 Hz, 1H), 8.17 (d, *J* = 8.3 Hz, 1H), 7.97 (d, *J* = 8.0 Hz, 1H), 7.92 (s, 1H), 7.81 (d, *J* = 6.9 Hz, 1H), 7.75 - 7.68 (m, 1H), 7.62 (t, *J* = 7.6 Hz, 1H), 7.57 (d, *J =* 8.2 Hz, 1H), 7.45 - 7.39 (m, 1H), 5.18 (dd, *J* = 13.2, 5.1 Hz, 1H), 4.86 - 4.71 (m, 1H), 4.58 - 4.45 (m, 3H), 3.51 - 3.01 (m, 6H), 3.01 - 2.90 (m, 2H), 2.68 - 2.66 (m, 1H), 2.62 (s, 3H), 2.58 - 2.53 (m, 1H), 2.39 - 2.31 (m, 1H), 2.10 - 2.01 (m, 1H). LCMS (ESI) m/z: calcd for C₄₂H₃₈F₃N₈O₄⁺[M+H]⁺, 775.30; found, 775.3.

### Example 112: preparation of N-(4-(4-((2-(2,6-dioxopiperidin-3-yl)-1-oxoisoindolin-5-yl)methyl)piperazin-1-yl)-3-(trifluoromethyl)phenyl)-3-(imidazo[1,2-b]pyridazin-3-ylethynyl)-4-methylbenzamide (GT-03805)

Referring to the method of Step 2 in Scheme 11 and the method of Example 1, the target compound (GT-03805) was prepared using 3-(imidazo[1,2-b]pyridazin-3-ylethynyl)-4-methyl-N-(4-(piperazin-1-yl)-3-(trifluoromethyl)phenyl)benzamide (GT-D-110) prepared from Intermediate Example 69 and 3-(5-(bromomethyl)-1-oxoisoindolin-2-yl)piperidine-2,6-dione. The target compound (GT-03805) was obtained as a white solid (48 mg, yield 60%). ¹H NMR (400 MHz, DMSO) δ 11.02 (s, 1H), 10.86 (s, 1H), 10.60 (s, 1H), 8.73 (dd, *J* = 4.4, 1.5 Hz, 1H), 8.27 (dd, *J* = 9.2, 1.5 Hz, 1H), 8.25 (s, 1H), 8.23 - 8.20 (m, 2H), 8.16 - 8.10 (m, 1H), 7.96 (dd, *J* = 8.0, 1.8 Hz, 1H), 7.90 (s, 1H), 7.85 (d, *J* = 7.8 Hz, 1H), 7.80 (d, *J* = 7.8 Hz, 1H), 7.56 (dd, *J* = 8.6, 3.0 Hz, 2H), 7.41 (dd, *J* = 9.2, 4.5 Hz, 1H), 5.15 (dd, *J* = 13.3, 5.0 Hz, 1H), 4.62 - 4.56 (m, 2H), 4.46 (dd, *J* = 52.1, 17.7 Hz, 2H), 3.44 - 3.40 (m, 2H), 3.30 - 3.16 (m, 4H), 3.11 - 3.01 (m, 2H), 3.00 - 2.87 (m, 1H), 2.68 - 2.63 (m, 1H), 2.61 (s, 3H), 2.46 - 2.37 (m, 1H), 2.06 - 1.98 (m, 1H). LCMS (ESI) m/z: calcd for C₄₁H₃₆F₃N₈O₄⁺[M+H]⁺, 761.28; found, 761.3.

### Example 113: preparation of 7-cyclopentyl-2-((5-(4-(((2-(2,6-dioxopiperidin-3-yl)-1-oxoisoindolin-5-yl)methyl)(methyl)amino)piperidin-1-yl)pyridin-2-yl)amino)-N,N-dimethyl-7H-pyrrolo[2,3-d]pyrimidine-6-carboxamide (GT-03544)

Referring to the method of Step 2 in Scheme 11 and the method of Example 1, the target compound (GT-03544) was prepared using 7-cyclopentyl-N,N-dimethyl-2-((5-(4-(methylamino)piperidin-1-yl)pyridin-2-yl)amino)-7H-pyrrolo[2,3-d]pyrimidine-6-carboxamide (GT-D-79) prepared from Intermediate Example 59 and 3-(5-(bromomethyl)-1-oxoisoindolin-2-yl)piperidine-2,6-dione. The target compound (GT-03544) was obtained as a white solid (100 mg, yield 60%). ¹H NMR (400 MHz, MeOD) δ 8.98 (s, 1H), 8.10 (dd, *J* = 9.6, 2.9 Hz, 1H), 7.96 - 7.90 (m, 2H), 7.87 (s, 1H), 7.75 (d, *J* = 7.9 Hz, 1H), 7.45 (d, *J* = 9.5 Hz, 1H), 6.81 (s, 1H), 5.19 (dd, *J* = 13.3, 5.1 Hz, 1H), 4.80 - 4.75 (m, 2H), 4.65 - 4.53 (m, 2H), 4.41 (d, *J* = 13.1 Hz, 1H), 4.00 - 3.90 (m, 2H), 3.68 - 3.59 (m, 1H), 3.16 (d, *J* = 7.7 Hz, 6H), 3.00 - 2.87 (m, 3H), 2.82 (s, 3H), 2.81 - 2.73 (m, 1H), 2.60 - 2.42 (m, 3H), 2.40 - 2.31 (m, 2H), 2.25 - 2.17 (m, 1H), 2.16 - 2.02 (m, 6H), 1.81 - 1.68 (m, 2H). LCMS (ESI) calcd for C₃₉H₄₇N₁₀O₄⁺ [M+H]⁺: 719.38, found, 719.4.

### Example 114: preparation of 7-cyclopentyl-2-((5-(4-(4-((2-(2,6-dioxopiperidin-3-yl)-1-oxoisoindolin-5-yl)methyl)piperazin-1-yl)piperidin-1-yl)pyridin-2-yl)amino)-N,N-dimethyl-7H-pyrrolo[2,3-d]pyrimidine-6-carboxamide (GT-03550)

Referring to the method of Step 2 in Scheme 11 and the method of Example 1, the target compound (GT-03550) was prepared using 7-cyclopentyl-N,N-dimethyl-2-((5-(4-(piperazin-1-yl)piperidin-1-yl)pyridin-2-yl)amino)-7H-pyrrolo[2,3-d]pyrimidine-6-carboxamide (GT-D-80) prepared from Intermediate Example 60 and 3-(5-(bromomethyl)-1-oxoisoindolin-2-yl)piperidine-2,6-dione. The target compound (GT-03550) was obtained as a white solid (107 mg, yield 67%). ¹H NMR (400 MHz, MeOD) δ 8.98 (s, 1H), 8.09 (d, *J* = 7.8 Hz, 1H), 8.02 - 7.88 (m, 3H), 7.81 (d, *J* = 7.7 Hz, 1H), 7.44 (d, *J* = 9.5 Hz, 1H), 6.81 (s, 1H), 5.18 (dd, *J* = 13.7, 4.9 Hz, 1H), 4.82 - 4.78 (m, 1H), 4.70 - 4.65 (m, 1H), 4.65 - 4.52 (m, 2H), 4.01 - 3.60 (m, 9H), 3.16 (d, *J* = 6.3 Hz, 6H), 3.04 - 2.74 (m, 4H), 2.65 - 2.31 (m, 5H), 2.24 - 1.95 (m, 6H), 1.81 - 1.63 (m, 2H), 1.42 - 1.33 (m, 2H). LCMS (ESI) calcd for C₄₂H₅₂N₁₁O₄⁺ [M+H]⁺: 744.42, found, 744.4.

### Example 115: preparation of 3-(5-((4-(6-((5-fluoro-4-(4-fluoro-1-isopropyl-2-methyl-1H-benzo [d] imidazol-6-yl)pyrimidin-2-yl)amino)pyridin-3-yl)piperazin-1-yl)methyl)-1-oxoisoindolin-2-yl)piperidine-2,6-dione (GT-03688)

Referring to the method of Step 2 in Scheme 11 and the method of Example 1, the target compound (GT-03688) was prepared using 5-Fluoro-4-(4-fluoro-1-isopropyl-2-methyl-1H-benzo[d]imidazol-6-yl)-N-(5-(piperazin-1-yl)pyridin-2-yl)pyrimidin-2-amine (CAS NO.: 1873303-25-3) and 3-(5-(bromomethyl)-1-oxoisoindolin-2-yl)piperidine-2,6-dione. The target compound (GT-03688) was obtained as a white solid (18 mg, yield 22.74%). ¹H NMR (400 MHz, DMSO) δ 12.00 (s, 1H), 11.54 (s, 1H), 11.02 (s, 1H), 9.42 (s, 1H), 8.83 (d, *J* = 3.4 Hz, 1H), 8.30 (s, 1H), 8.06 (d, *J* = 9.4 Hz, 1H), 7.97 (s, 2H), 7.88 (d, *J* = 9.5 Hz, 1H), 7.83 (s, 2H), 7.78 (d, *J* = 11.7 Hz, 1H), 5.15 (dd, *J* = 13.2, 5.1 Hz, 1H), 4.89 (dd, *J* = 13.9, 7.0 Hz, 1H), 4.52 (d, *J* = 17.5 Hz, 3H), 4.36 (s, 1H), 3.81 (s, 1H), 3.44 (d, *J* = 6.7 Hz, 2H), 3.34 (t, *J* = 12.6 Hz, 2H), 3.23 (dd, *J* = 18.6, 10.7 Hz, 2H), 2.98 - 2.89 (m, 1H), 2.73 (s, 3H), 2.61 (dd, *J* = 15.9, 1.4 Hz, 1H), 2.44 (dd, *J* = 13.1, 4.5 Hz, 1H), 2.05 - 1.98 (m, 1H), 1.64 (d, *J* = 6.9 Hz, 6H). LCMS (ESI) calcd for C₃₈H₃₉F₂N₁₀O₃⁺ [M+H]⁺: 721.31, found, 721.30.

### Example 116: preparation of 3-(5-(((1-(6-((5-fluoro-4-(4-fluoro-1-isopropyl-2-methyl-1H-benzo [d] imidazol-6-yl)pyrimidin-2-yl)amino)pyridin-3-yl)piperidin-4-yl)(methyl)amino)methyl)-1-oxoisoindolin-2-yl)piperidine-2,6-dione (GT-03639)

Referring to the method of Step 2 in Scheme 11 and the method of Example 1, the target compound (GT-03639) was prepared using 5-fluoro-4-(4-fluoro-1-isopropyl-2-methyl-1H-benzo[d]imidazol-6-yl)-N-(5-(4-(methylamino)piperidin-1-yl)pyridin-2-yl)pyrimidin-2-amine (CAS NO.: 2756101-77-4) and 3-(5-(bromomethyl)-1-oxoisoindolin-2-yl)piperidine-2,6-dione. The target compound (GT-03639) was obtained as a white solid (24 mg, yield 29%). ¹H NMR (400 MHz, MeOD) δ8.83 (d, *J* = 3.4 Hz, 1H), 8.52 (s, 1H), 8.27 (dd, *J* = 9.7, 2.8 Hz, 1H), 8.10 (d, *J* = 11.1 Hz, 1H), 7.94 (d, *J* = 7.9 Hz, 1H), 7.90 - 7.85 (m, 2H), 7.75 (d, *J* = 8.2 Hz, 1H), 7.55 (d, *J* = 9.7 Hz, 1H), 5.19 (dd, *J* = 13.2, 5.1 Hz, 1H), 5.09 (dt, *J* = 14.0, 7.0 Hz, 1H), 4.78 (d, *J* = 12.6 Hz, 1H), 4.59 (q, *J* = 17.6 Hz, 2H), 4.42 (d, *J* = 12.6 Hz, 1H), 4.05 - 3.94 (m, 2H), 3.71 - 3.61 (m, 1H), 3.05 - 2.92 (m, 3H), 2.90 (s, 3H), 2.82 (s, 3H), 2.81 - 2.75 (m, 1H), 2.53 (qd, *J* = 13.2, 4.7 Hz, 1H), 2.42 - 2.34 (m, 2H), 2.25 - 2.07 (m, 3H), 1.79 (d, *J* = 6.9 Hz, 6H). LCMS (ESI) m/z: calcd for C₄₀H₄₃F₂N₁₀O₃⁺[M+H]⁺, 749.35; found, 749.3.

### Example 117: preparation of 3-(5-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)-1-oxoisoindolin-2-yl)piperidine-2,6-dione (GT-03719)

Referring to the method of Step 2 in Scheme 11 and the method of Example 1, the target compound (GT-03719) was prepared using 5-Fluoro-4-(4-fluoro-1-isopropyl-2-methyl-1H-benzo[d]imidazol-6-yl)-N-(5-(4-(piperazin-1-yl)piperidin-1-yl)pyridin-2-yl)pyrimidin-2-amine (CAS NO.: 1873300-67-4) and 3-(5-(bromomethyl)-1-oxoisoindolin-2-yl)piperidine-2,6-dione. The target compound (GT-03719) was obtained as a white solid (22 mg, yield 31.33%). ¹H NMR (400 MHz, DMSO) δ 11.62 (s, 1H), 11.01 (s, 1H), 8.84 (d, *J* = 3.4 Hz, 1H), 8.27 (s, 1H), 8.16 (d, *J* = 9.5 Hz, 1H), 7.93 (dd, *J* = 8.5, 3.5 Hz, 2H), 7.77 (dd, *J* = 13.3, 11.8 Hz, 4H), 5.14 (dd, *J* = 13.0, 5.3 Hz, 1H), 4.90 (dd, *J* = 13.8, 6.8 Hz, 1H), 4.45 (td, *J* = 33.7, 16.7 Hz, 6H), 3.87 (d, *J* = 11.1 Hz, 3H), 3.74 - 3.65 (m, 3H), 3.49 - 3.41 (m, 2H), 2.96 - 2.89 (m, 1H), 2.81 (t, *J* = 12.0 Hz, 2H), 2.71 (s, 3H), 2.61 (d, *J* = 17.2 Hz, 1H), 2.47 - 2.35 (m, 2H), 2.25 - 2.15 (m, 2H), 2.04 - 1.99 (m, 1H), 1.90 - 1.79 (m, 2H), 1.63 (d, *J* = 6.9 Hz, 6H). LCMS (ESI) calcd for C₄₃H₄₈F₂N₁₁O₃⁺ [M+H]⁺: 804.38, found, 804.40.

### Example 118: preparation of 3-(5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-6-fluoro-1-oxoisoindolin-2-yl)piperidine-2,6-dione (GT-03601)

The target compound (GT-03601) was prepared using the method of Step 2 in Scheme 11 and the method of Example 1 (white solid, 40 mg, yield 49%). ¹H NMR (400 MHz, MeOD) δ8.29 (s, 1H), 8.13 (s, 1H), 7.94 (d, *J* = 6.1 Hz, 1H), 7.75 - 7.68 (m, 2H), 7.62 (t, *J* = 7.5 Hz, 1H), 7.52 - 7.40 (m, 2H), 7.09 (s, 1H), 6.89 (d, *J* = 8.7 Hz, 1H), 5.19 (dd, *J* = 13.3, 5.1 Hz, 1H), 4.66 - 4.57 (m, 2H), 4.57 - 4.34 (m, 2H), 4.04 - 3.95 (m, 2H), 3.92 (s, 3H), 3.88 - 3.77 (m, 1H), 3.39 - 3.32 (m, 2H), 3.03 - 2.94 (m, 1H), 2.92 (s, 3H), 2.84 - 2.76 (m, 1H), 2.59 - 2.28 (m, 5H), 2.24 - 2.16 (m, 1H), 1.90 (s, 3H), 1.87 (s, 3H). LCMS (ESI) calcd for C₃₉H₄₄ClFN₈O₅P⁺ [M+H]⁺: 789.28, found, 789.3.

### Example 119: preparation of 3-(5-((4-(4-((5-chloro-4-((2-(dimethylphosphoryl)phenyl)amino)pyrimidin-2-yl)amino)-3-methoxyphenyl)piperazin-1-yl)methyl)-1-oxoisoindolin-2-yl)piperidine-2,6-dione (GT-03775)

Referring to the method of Step 2 in Scheme 11 and the method of Example 1, the target compound (GT-03775) was prepared using (2-((5-chloro-2-((2-methoxy-4-(piperazin-1-yl)phenyl)amino)pyrimidin-4-yl)amino)phenyl)dimethylphosphine oxide (GT-D-39) prepared from Intermediate Example 23 and 3-(5-(bromomethyl)-1-oxoisoindolin-2-yl)piperidine-2,6-dione. The target compound (GT-03775) was obtained as a white solid (39 mg, yield 63.07%). ¹H NMR (400 MHz, DMSO) δ 11.96 (d, *J* = 41.2 Hz, 2H), 11.01 (s, 1H), 9.48 (s, 1H), 8.31 (d, *J* = 27.9 Hz, 2H), 7.98 (s, 1H), 7.88 - 7.79 (m, 2H), 7.63 (dd, *J* = 13.8, 7.5 Hz, 1H), 7.36 (d, *J* = 8.6 Hz, 2H), 7.24 (t, *J* = 7.3 Hz, 1H), 6.73 (d, *J* = 2.1 Hz, 1H), 6.56 (dd, *J* = 8.7, 2.1 Hz, 1H), 5.15 (dd, *J* = 13.2, 5.1 Hz, 1H), 4.52 (d, *J* = 17.2 Hz, 3H), 4.39 (d, *J* = 17.7 Hz, 1H), 3.87 (d, *J* = 11.9 Hz, 2H), 3.78 (s, 3H), 3.41 (d, *J* = 10.6 Hz, 2H), 3.34 - 3.27 (m, 2H), 3.25 - 3.17 (m, 2H), 2.98 - 2.89 (m, 1H), 2.61 (d, *J* = 17.0 Hz, 1H), 2.46 - 2.35 (m, 1H), 2.07 - 1.98 (m, 1H), 1.81 (s, 3H), 1.78 (s, 3H). LCMS (ESI) calcd for C₃₇H₄₁ClN₈O₅P⁺ [M+H]⁺: 743.25, found, 743.30.

### Example 120: preparation of 3-(5-((4-(4-((5-chloro-4-((2-(dimethylphosphoryl)phenyl)amino)pyrimidin-2-yl)amino)-3-methoxyphenyl)piperazin-1-yl)methyl)-4-fluoro-1-oxoisoindolin-2-yl)piperidine-2,6-dione (GT-03776)

Referring to the methods of Scheme 11 and Example 1, the target compound (GT-03776) was prepared using (2-((5-chloro-2-((2-methoxy-4-(piperazin-1-yl)phenyl)amino)pyrimidin-4-yl)amino)phenyl)dimethylphosphine oxide (GT-D-39) prepared from Intermediate Example 23 and 3-(4-fluoro-5-(hydroxymethyl)-1-oxoisoindolin-2-yl)piperidine-2,6-dione (GT-03445). The target compound (GT-03776) was obtained as a white solid (34 mg, yield 53.69%). ¹H NMR (400 MHz, DMSO) δ 11.93 (s, 1H), 11.74 (s, 1H), 11.03 (s, 1H), 9.33 (s, 1H), 8.31 (d, *J =* 41.8 Hz, 2H), 8.07 - 8.00 (m, 1H), 7.71 (d, *J* = 7.7 Hz, 1H), 7.62 (dd, *J* = 13.7, 7.5 Hz, 1H), 7.37 (d, *J* = 8.6 Hz, 2H), 7.22 (t, *J* = 7.3 Hz, 1H), 6.74 (d, *J* = 2.0 Hz, 1H), 6.56 (dd, *J* = 8.7, 2.0 Hz, 1H), 5.16 (dd, *J* = 13.2, 5.1 Hz, 1H), 4.64 - 4.55 (m, 3H), 4.47 (d, *J* = 17.6 Hz, 1H), 3.91 - 3.85 (m, 2H), 3.78 (s, 3H), 3.58 - 3.50 (m, 2H), 3.25 (d, *J* = 20.0 Hz, 4H), 2.98 - 2.89 (m, 1H), 2.62 (d, *J* = 17.1 Hz, 1H), 2.44 (dd, *J* = 13.5, 4.4 Hz, 1H), 2.03 (ddd, *J* = 9.9, 4.9, 3.0 Hz, 1H), 1.81 (s, 3H), 1.77 (s, 3H). LCMS (ESI) calcd for C₃₇H₄₀ClFN₈O₅P⁺ [M+H]⁺: 761.25, found, 761.30.

### Example 121: preparation of 3-(5-((4-(4-((5-chloro-4-((2-(dimethylphosphoryl)phenyl)amino)pyrimidin-2-yl)amino)-3-methoxyphenyl)piperazin-1-yl)methyl)-6-fluoro-1-oxoisoindolin-2-yl)piperidine-2,6-dione (GT-03777)

The target compound (GT-03777) was prepared using the methods described in Scheme 11 and Example 1 (white solid, 35 mg, yield 55.27%). ¹H NMR (400 MHz, DMSO) δ 11.93 (s, 2H), 11.03 (s, 1H), 9.35 (s, 1H), 8.35 (s, 1H), 8.26 (s, 1H), 8.16 (d, *J* = 6.1 Hz, 1H), 7.69 (d, *J* = 8.5 Hz, 1H), 7.62 (dd, *J* = 13.3, 7.7 Hz, 1H), 7.38 (d, *J* = 8.6 Hz, 2H), 7.22 (t, *J* = 7.2 Hz, 1H), 6.74 (d, *J* = 1.9 Hz, 1H), 6.55 (dd, *J* = 8.7, 2.0 Hz, 1H), 5.15 (dd, *J* = 13.2, 5.0 Hz, 1H), 4.59 - 4.48 (m, 3H), 4.38 (d, *J* = 17.5 Hz, 1H), 3.87 (s, 2H), 3.78 (s, 3H), 3.50 (s, 2H), 3.29 (s, 4H), 2.98 - 2.88 (m, 1H), 2.61 (d, *J* = 16.9 Hz, 1H), 2.47 - 2.37 (m, 1H), 2.04 (dd, *J* = 13.8, 8.4 Hz, 1H), 1.81 (s, 3H), 1.77 (s, 3H). LCMS (ESI) calcd for C₃₇H₄₀ClFN₈O₅P⁺ [M+H]⁺: 761.25, found, 761.30.

### Example 122: preparation of 3-(5-((4-(4-((5-chloro-4-((2-(dimethylphosphoryl)phenyl)amino)pyrimidin-2-yl)amino)-3-methoxyphenyl)piperazin-1-yl)methyl)-7-fluoro-1-oxoisoindolin-2-yl)piperidine-2,6-dione (GT-03778)

Referring to the method of Step 2 in Scheme 11 and the method of Example 1, the target compound (GT-03778) was prepared using (2-((5-chloro-2-((2-methoxy-4-(piperazin-1-yl)phenyl)amino)pyrimidin-4-yl)amino)phenyl)dimethylphosphine oxide (GT-D-39) prepared from Intermediate Example 23 and 3-(7-fluoro-5-(hydroxymethyl)-1-oxoisoindolin-2-yl)piperidine-2,6-dione (GT-03446). The target compound (GT-03778) was obtained as a white solid (42 mg, yield 66.32%). ¹H NMR (400 MHz, DMSO) δ 11.99 (s, 2H), 11.02 (s, 1H), 9.45 (s, 1H), 8.35 (s, 1H), 8.27 (s, 1H), 7.76 (d, *J* = 8.1 Hz, 2H), 7.62 (dd, *J* = 13.3, 7.7 Hz, 1H), 7.37 (d, *J* = 8.6 Hz, 2H), 7.24 (t, *J* = 7.3 Hz, 1H), 6.74 (d, *J* = 2.0 Hz, 1H), 6.56 (dd, *J* = 8.7, 2.1 Hz, 1H), 5.11 (dd, *J* = 13.2, 5.0 Hz, 1H), 4.57 - 4.50 (m, 3H), 4.41 (d, *J* = 18.1 Hz, 1H), 3.87 (d, *J* = 8.7 Hz, 2H), 3.79 (s, 3H), 3.43 (d, *J* = 10.1 Hz, 2H), 3.35 - 3.27 (m, 2H), 3.25 - 3.17 (m, 2H), 2.97 - 2.88 (m, 1H), 2.61 (d, *J* = 16.4 Hz, 1H), 2.45 - 2.37 (m, 1H), 2.05 -1.96 (m, 1H), 1.81 (s, 3H), 1.78 (s, 3H). LCMS (ESI) calcd for C₃₇H₄₀ClFN₈O₅P⁺ [M+H]⁺: 761.25, found, 761.30.

### Example 123: preparation of 3-(4-((4-(4-((5-chloro-4-((2-(dimethylphosphoryl)phenyl)amino)pyrimidin-2-yl)amino)-3-methoxyphenyl)piperazin-1-yl)methyl)-1-oxoisoindolin-2-yl)piperidine-2,6-dione (GT-03779)

The target compound (GT-03779) was prepared using the methods described in Scheme 11 and Example 1 (white solid, 28 mg, yield 45.28%). ¹H NMR (400 MHz, DMSO) δ 11.98 (s, 1H), 11.81 (s, 1H), 11.07 (s, 1H), 9.43 (s, 1H), 8.35 (s, 1H), 8.27 (s, 1H), 8.10 (d, *J* = 7.4 Hz, 1H), 7.85 (d, *J* = 7.6 Hz, 1H), 7.68 - 7.58 (m, 2H), 7.37 (d, *J* = 8.8 Hz, 2H), 7.23 (t, *J* = 7.1 Hz, 1H), 6.74 (s, 1H), 6.56 (d, *J* = 8.8 Hz, 1H), 5.20 (dd, *J* = 12.9, 4.8 Hz, 1H), 5.00 (d, *J* = 17.5 Hz, 1H), 4.53 (dd, *J* = 25.2, 14.8 Hz, 3H), 3.89 (d, *J* = 7.3 Hz, 2H), 3.79 (s, 3H), 3.50 (d, *J* = 7.9 Hz, 1H), 3.36 (dd, *J* = 21.2, 9.4 Hz, 5H), 2.95 (dd, *J* = 21.8, 8.8 Hz, 1H), 2.65 (d, *J* = 17.0 Hz, 1H), 2.35 (dt, *J* = 13.2, 9.6 Hz, 1H), 2.09 - 2.01 (m, 1H), 1.81 (s, 3H), 1.77 (s, 3H). LCMS (ESI) calcd for C₃₇H₄₁ClN₈O₅P⁺ [M+H]⁺: 743.25, found, 743.30.

### Example 124: preparation of 3-(5-((4-(4-((5-chloro-4-((2-(isopropylsulfonyl)phenyl)amino)pyrimidin-2-yl)amino)-5-isopropoxy-2-methylphenyl)piperazin-1-yl)methyl)-1-oxoisoindolin-2-yl)piperidine-2,6-dione (GT-03529)

Referring to the method of Step 2 in Scheme 11 and the method of Example 1, the target compound (GT-03529) was prepared using 5-chloro-N²-(2-isopropoxy-5-methyl-4-(piperazin-1-yl)phenyl)-N⁴-(2-(isopropylsulfonyl)phenyl)pyrimidine-2,4-diamine (GT-D-86) prepared from Intermediate Example 20 and 3-(5-(bromomethyl)-1-oxoisoindolin-2-yl)piperidine-2,6-dione. The target compound (GT-03529) was obtained as a white solid (77 mg, yield 49%). ¹H NMR (400 MHz, MeOD) δ 8.35 (d, *J* = 8.0 Hz, 1H), 8.19 (s, 1H), 8.00 - 7.92 (m, 2H), 7.88 (s, 1H), 7.78 (d, *J* = 7.8 Hz, 1H), 7.70 (t, *J* = 7.7 Hz, 1H), 7.56 (s, 1H), 7.46 (t, *J* = 7.7 Hz, 1H), 6.80 (s, 1H), 5.19 (dd, *J* = 13.3, 5.1 Hz, 1H), 4.65 - 4.53 (m, 5H), 3.62 - 3.53 (m, 2H), 3.45 - 3.34 (m, 3H), 3.29 - 3.22 (m, 2H), 3.19 - 3.10 (m, 2H), 2.98 - 2.87 (m, 1H), 2.84 - 2.76 (m, 1H), 2.59 - 2.46 (m, 1H), 2.24 - 2.18 (m, 1H), 2.16 (s, 3H), 1.30 (d,*J* = 6.0 Hz, 6H), 1.25 (d, *J* = 6.8 Hz, 6H). LCMS (ESI) calcd for C₄₁H₄₈ClN₈O₆S⁺ [M+H]⁺: 815.31, found, 815.3.

### Example 125: preparation of 3-(5-(((1-(4-((5-chloro-4-((2-(isopropylsulfonyl)phenyl)amino)pyrimidin-2-yl)amino)-5-isopropoxy-2-methylphenyl)piperidin-4-yl)(methyl)amino)methyl)-1-oxoisoindolin-2-yl)piperidine-2,6-dione (GT-03530)

Referring to the method of Step 2 in Scheme 11 and the method of Example 1, the target compound (GT-03530) was prepared using 5-chloro-N²-(2-isopropoxy-5-methyl-4-(4-(methylamino)piperidin-1-yl)phenyl)-N⁴-(2-(isopropylsulfonyl)phenyl)pyrimidine-2,4-diamine (GT-D-87) prepared from Intermediate Example 21 and 3-(5-(bromomethyl)-1-oxoisoindolin-2-yl)piperidine-2,6-dione. The target compound (GT-03530) was obtained as a white solid (92 mg, yield 61%). ¹H NMR (400 MHz, MeOD) δ 8.33 - 8.19 (m, 2H), 8.03 (dd, *J* = 7.9, 1.4 Hz, 1H), 7.94 (d, *J* = 7.8 Hz, 1H), 7.89 (s, 1H), 7.82 - 7.71 (m, 2H), 7.62 - 7.53 (m, 1H), 7.32 (s, 1H), 6.93 (s, 1H), 5.19 (dd, *J* = 13.3, 5.2 Hz, 1H), 4.79 (d, *J* = 13.3 Hz, 1H), 4.75 - 4.65 (m, 2H), 4.63 - 4.54 (m, 2H), 4.44 (d, *J* = 13.1 Hz, 1H), 3.69 - 3.59 (m, 1H), 3.46 - 3.33 (m, 4H), 2.95 - 2.88 (m, 1H), 2.85 (s, 3H), 2.83 - 2.77 (m, 1H), 2.53 (qd, *J* = 13.2, 4.6 Hz, 1H), 2.40 - 2.33 (m, 2H), 2.29 - 2.15 (m, 6H), 1.29 (d, *J* = 6.0 Hz, 6H), 1.26 (d, *J* = 6.8 Hz, 6H). LCMS (ESI) calcd for C₄₃H₅₂ClN₈O₆S⁺ [M+H]⁺: 843.34, found, 843.3.

### Example 126: preparation of 3-(5-((4-(1-(4-((5-chloro-4-((2-(isopropylsulfonyl)phenyl)amino)pyrimidin-2-yl)amino)-5-isopropoxy-2-methylphenyl)piperidin-4-yl)piperazin-1-yl)methyl)-1-oxoisoindolin-2-yl)piperidine-2,6-dione (GT-03531)

Referring to the method of Step 2 in Scheme 11 and the method of Example 1, the target compound (GT-03531) was prepared using 5-chloro-N²-(2-isopropoxy-5-methyl-4-(4-(piperazin-1-yl)piperidin-1-yl)phenyl)-N⁴-(2-(isopropylsulfonyl)phenyl)pyrimidine-2,4-diamine (GT-D-88) prepared from Intermediate Example 22 and 3-(5-(bromomethyl)-1-oxoisoindolin-2-yl)piperidine-2,6-dione. The target compound (GT-03531) was obtained as a white solid (58 mg, yield 39%). ¹H NMR (400 MHz, MeOD) δ 8.36 - 8.14 (m, 2H), 8.02 (d, *J* = 7.9 Hz, 1H), 7.87 (d, *J* = 7.8 Hz, 1H), 7.82 - 7.72 (m, 2H), 7.69 (d, *J* = 7.8 Hz, 1H), 7.56 (t, *J* = 7.6 Hz, 1H), 7.37 - 7.22 (m, 1H), 6.83 (s, 1H), 5.18 (dd, *J=* 13.3, 5.2 Hz, 1H), 4.69 - 4.61 (m, 1H), 4.55 (q, *J* = 17.4 Hz, 2H), 4.24 (s, 2H), 3.93 - 3.36 (m, 8H), 3.13 - 2.70 (m, 5H), 2.59 - 2.44 (m, 1H), 2.34 - 2.25 (m, 2H), 2.24 - 2.14 (m, 4H), 2.08 - 1.94 (m, 2H), 1.28 (d, *J* = 6.0 Hz, 6H), 1.25 (d, *J* = 6.8 Hz, 6H). LCMS (ESI) calcd for C₄₆H₅₇ClN₉O₆S⁺ [M+H]⁺: 898.38, found, 898.4.

### Example 127: preparation of 8-(4-(4-((2-(2,6-dioxopiperidin-3-yl)-4-fluoro-1-oxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile (GT-03758)

Referring to the methods of Scheme 11 and Example 1, the target compound (GT-03758) was prepared using 9-ethyl-6,6-dimethyl-11-oxo-8-(4-(piperazin-1-yl)piperidin-1-yl)-6,11-dihydro-5H-benzo[b]carbazole-3-carbonitrile (GT-D-113) prepared from Intermediate Example 45 and 3-(4-fluoro-5-(hydroxymethyl)-1-oxoisoindolin-2-yl)piperidine-2,6-dione (GT-03445). The target compound (GT-03758) was obtained as a white solid (22 mg, yield 26%). ¹H NMR (400 MHz, DMSO) δ 12.85 (s, 1H), 11.02 (s, 1H), 8.31 (d, *J* = 8.2 Hz, 1H), 8.06 (s, 1H), 8.00 (s, 1H), 7.77 (s, 1H), 7.66 (d, *J* = 7.6 Hz, 1H), 7.60 (dd, *J* = 8.2, 1.2 Hz, 1H), 7.36 (s, 1H), 5.14 (dd, *J* = 13.3, 5.1 Hz, 1H), 4.60 (d, *J* = 17.5 Hz, 1H), 4.43 (d, *J* = 17.5 Hz, 1H), 4.15 (s, 2H), 3.47 - 3.22 (m, 10H), 3.00 - 2.87 (m, 2H), 2.86 - 2.76 (m, 2H), 2.74 - 2.68 (m, 2H), 2.64 - 2.58 (m, 1H), 2.47 - 2.41 (m, 1H), 2.27 - 2.18 (m, 2H), 2.07 - 1.99 (m, 1H), 1.96 - 1.86 (m, 2H), 1.76 (s, 6H), 1.28 (t, *J* = 7.5 Hz, 3H). LCMS (ESI) m/z: calcd for C₄₄H₄₇FN₇O₄⁺[M+H]⁺, 756.37; found, 756.4

### Example 128: preparation of 8-(4-(4-((2-(2,6-dioxopiperidin-3-yl)-6-fluoro-1-oxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile (GT-03759)

The target compound (GT-03759) was prepared using the method of Step 2 in Scheme 11 and the method of Example 1 (white solid, 32 mg, yield 38%). ¹H NMR (400 MHz, DMSO) δ 12.83 (s, 1H), 11.01 (s, 1H), 8.31 (d, *J* = 8.2 Hz, 1H), 8.06 (s, 1H), 8.00 (s, 1H), 7.84 (s, 1H), 7.65 - 7.56 (m, 2H), 7.36 (s, 1H), 5.14 (dd, *J* = 13.2, 5.1 Hz, 1H), 4.48 (d, *J* = 17.5 Hz, 1H), 4.36 (d, *J* = 17.1 Hz, 1H), 4.08 (s, 2H), 3.46 - 3.18 (m, 10H), 2.98 - 2.87 (m, 2H), 2.82 (t, *J* = 11.5 Hz, 2H), 2.71 (q, *J* = 7.4 Hz, 2H), 2.64 - 2.59 (m, 1H), 2.46 - 2.39 (m, 1H), 2.25 - 2.17 (m, 2H), 2.07 - 2.00 (m, 1H), 1.95 - 1.86 (m, 2H), 1.76 (s, 6H), 1.28 (t, *J* = 7.5 Hz, 3H). LCMS (ESI) m/z: calcd for C₄₄H₄₇FN₇O₄⁺[M+H]⁺, 756.37; found, 756.4.

### Example 129: preparation of 8-(4-(4-((2-(2,6-dioxopiperidin-3-yl)-7-fluoro-1-oxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile (GT-03760)

Referring to the methods of Scheme 11 and Example 1, the target compound (GT-03760) was prepared using 9-ethyl-6,6-dimethyl-11-oxo-8-(4-(piperazin-1-yl)piperidin-1-yl)-6,11-dihydro-5H-benzo[b]carbazole-3-carbonitrile (GT-D-113) prepared from Intermediate Example 45 and 3-(7-fluoro-5-(hydroxymethyl)-1-oxoisoindolin-2-yl)piperidine-2,6-dione (GT-03446). The target compound (GT-03760) was obtained as a white solid (40 mg, yield 47%). ¹H NMR (400 MHz, DMSO) δ 12.83 (s, 1H), 11.02 (s, 1H), 8.31 (d, *J* = 8.2 Hz, 1H), 8.06 (s, 1H), 8.00 (s, 1H), 7.67 - 7.47 (m, 3H), 7.36 (s, 1H), 5.10 (dd, *J* = 13.3, 5.0 Hz, 1H), 4.51 (d, *J* = 18.2 Hz, 1H), 4.38 (d, *J* = 17.9 Hz, 1H), 4.20 (s, 2H), 3.50 - 3.23 (m, 10H), 2.97 - 2.87 (m, 2H), 2.86 - 2.78 (m, 2H), 2.71 (q, *J* = 7.5 Hz, 2H), 2.65 - 2.58 (m, 1H), 2.46 - 2.35 (m, 1H), 2.27 - 2.18 (m, 2H), 2.05 - 1.98 (m, 1H), 1.95 - 1.86 (m, 2H), 1.76 (s, 6H), 1.28 (t, *J* = 7.5 Hz, 3H). LCMS (ESI) m/z: calcd for C₄₄H₄₇FN₇O₄⁺[M+H]⁺, 756.37; found, 756.4.

### Example 130: preparation of 8-(4-(4-((2-(2,6-dioxopiperidin-3-yl)-1-oxoisoindolin-4-yl)methyl)piperazin-1-yl)piperidin-1-yl)-9-ethyl-6,6-dimethyl-11-oxo-6,11-dihydro-5H-benzo[b]carbazole-3-carbonitrile (GT-03764)

The target compound (GT-03764) was prepared using the method of Step 2 in Scheme 11 and the method of Example 1 (white solid, 44 mg, yield 54%). ¹H NMR (400 MHz, DMSO) δ 12.83 (s, 1H), 11.05 (s, 1H), 8.31 (d, *J* = 8.2 Hz, 1H), 8.06 (s, 1H), 8.00 (s, 1H), 7.85 - 7.71 (m, 2H), 7.60 (dd, *J* = 8.2, 1.3 Hz, 2H), 7.36 (s, 1H), 5.17 (dd, *J* = 12.9, 5.2 Hz, 1H), 4.70 - 4.41 (m, 2H), 3.54 - 3.14 (m, 11H), 3.00 - 2.94 (m, 1H), 2.82 (t, *J* = 11.5 Hz, 2H), 2.71 (q, *J* = 7.6 Hz, 2H), 2.67 - 2.61 (m, 1H), 2.45 - 2.31 (m, 1H), 2.28 - 2.18 (m, 2H), 2.09 - 2.01 (m, 1H), 1.98 - 1.87 (m, 2H), 1.76 (s, 6H), 1.28 (t, *J* = 7.5 Hz, 3H). LCMS (ESI) m/z: calcd for C₄₄H₄₈N₇O₄⁺[M+H]⁺, 738.38; found, 738.4

### Example 131: preparation of 8-(4-(((2-(2,6-dioxopiperidin-3-yl)-1-oxoisoindolin-5-yl)methyl)(methyl)amino)piperidin-1-yl)-9-ethyl-6,6-dimethyl-11-oxo-6,11-dihydro-5H-benzo[b]carbazole-3-carbonitrile (GT-03474)

Referring to the method of Step 2 in Scheme 11 and the method of Example 1, the target compound (GT-03474) was prepared using 9-ethyl-6,6-dimethyl-8-(4-(methylamino)piperidin-1-yl)-11-oxo-6,11-dihydro-5H-benzo[b]carbazole-3-carbonitrile (GT-D-85) prepared from Intermediate Example 46 and 3-(5-(bromomethyl)-1-oxoisoindolin-2-yl)piperidine-2,6-dione. The target compound (GT-03474) was obtained as a white solid (23.5 mg, yield 29%). ¹H NMR (400 MHz, MeOD) δ 8.41 (d, *J* = 8.1 Hz, 1H), 8.20 (s, 1H), 7.96 (d, *J* = 7.8 Hz, 1H), 7.86 (d, *J* = 9.8 Hz, 2H), 7.76 (d, *J* = 7.9 Hz, 1H), 7.55 (dd, *J* = 8.2, 1.2 Hz, 1H), 7.41 (s, 1H), 5.20 (dd, *J* = 13.3, 5.1 Hz, 1H), 4.60 (q, *J* = 17.6 Hz, 2H), 4.10 (q, *J* = 7.1 Hz, 2H), 3.49 - 3.36 (m, 2H), 2.99 - 2.92 (m, 2H), 2.88 - 2.80 (m, 5H), 2.53 (qd, *J* = 13.2, 4.6 Hz, 1H), 2.38 - 2.30 (m, 2H), 2.25 - 2.09 (m, 3H), 2.01 (s, 3H), 1.81 (s, 6H), 1.24 (t,*J* = 7.1 Hz, 3H). LCMS (ESI) calcd for C₄₁H₄₃ClN₆O₄⁺ [M+H]⁺: 683.33, found, 683.3.

### Example 132: preparation of 3-(5-(((1-(4-(5,7-dimethoxy-4-oxo-3,4-dihydroquinazolin-2-yl)phenyl)piperidin-4-yl)(methyl)amino)methyl)-1-oxoisoindolin-2-yl)piperidine-2,6-dione (GT-03707)

Referring to the method of Step 2 in Scheme 11 and the method of Example 1, the target compound (GT-03707) was prepared using 5,7-dimethoxy-2-(4-(4-(methylamino)piperidin-1-yl)phenyl)quinazolin-4(3H)-one (GT-D-93) prepared from Intermediate Example 62 and 3-(5-(bromomethyl)-1-oxoisoindolin-2-yl)piperidine-2,6-dione. The target compound (GT-03707) was obtained as a white solid (32 mg, yield 38.02%). ¹H NMR (400 MHz, DMSO) δ 10.98 (s, 1H), 8.08 (d, *J* = 8.9 Hz, 2H), 7.95 (s, 1H), 7.67 (d, *J* = 7.8 Hz, 1H), 7.55 (s, 1H), 7.46 (d, *J* = 7.9 Hz, 1H), 7.01 (d, *J* = 9.0 Hz, 2H), 6.68 (d, *J* = 2.1 Hz, 1H), 6.46 (d, *J* = 2.1 Hz, 1H), 5.10 (dd, *J* = 13.3, 5.1 Hz, 1H), 4.44 (d, *J* = 17.3 Hz, 1H), 4.31 (d, *J* = 17.3 Hz, 1H), 3.99 (d, *J* = 12.3 Hz, 2H), 3.88 (s, 3H), 3.83 (s, 3H), 3.69 (s, 2H), 2.93 (dd, *J* = 13.9, 5.4 Hz, 1H), 2.83 (t, *J* = 12.5 Hz, 2H), 2.68 - 2.57 (m, 2H), 2.40 (dd, *J* = 13.0, 8.8 Hz, 1H), 2.13 (s, 3H), 2.03 - 1.98 (m, 1H), 1.88 (d, *J* = 10.7 Hz, 2H), 1.59 (dd, *J* = 20.8, 10.7 Hz, 2H). LCMS (ESI) calcd for C₃₆H₃₉N₆O₆⁺ [M+H]⁺: 651.29, found, 651.30.

### Example 133: preparation of 3-(5-((4-(1-(4-(5,7-dimethoxy-4-oxo-3,4-dihydroquinazolin-2-yl)phenyl)piperidin-4-yl)piperazin-1-yl)methyl)-1-oxoisoindolin-2-yl)piperidine-2,6-dione (GT-03708)

Referring to the method of Step 2 in Scheme 11 and the method of Example 1, the target compound (GT-03708) was prepared using 5,7-dimethoxy-2-(4-(4-(piperazin-1-yl)piperidin-1-yl)phenyl)quinazolin-4(3H)-one (GT-D-94) prepared from Intermediate Example 63 and 3-(5-(bromomethyl)-1-oxoisoindolin-2-yl)piperidine-2,6-dione. The target compound (GT-03708) was obtained as a white solid (29 mg, yield 36.20%). ¹H NMR (400 MHz, DMSO) δ 11.72 (s, 1H), 10.98 (s, 1H), 8.07 (d, *J* = 8.9 Hz, 2H), 7.95 (s, 1H), 7.67 (d, *J* = 7.7 Hz, 1H), 7.53 (s, 1H), 7.43 (d, *J* = 7.8 Hz, 1H), 6.99 (d, *J* = 9.0 Hz, 2H), 6.67 (d, *J* = 2.1 Hz, 1H), 6.46 (d, *J* = 2.0 Hz, 1H), 5.10 (dd, *J* = 13.3, 5.1 Hz, 1H), 4.44 (d, *J* = 17.5 Hz, 1H), 4.31 (d, *J* = 17.3 Hz, 1H), 3.92 (d, *J* = 12.9 Hz, 2H), 3.88 (s, 3H), 3.83 (s, 3H), 3.56 (s, 2H), 2.93 (dd, *J* = 13.7, 4.9 Hz, 1H), 2.81 (t, *J* = 11.7 Hz, 2H), 2.70 (d, *J* = 24.0 Hz, 3H), 2.64 - 2.54 (m, 2H), 2.46 - 2.34 (m, 6H), 1.99 (ddd, *J* = 13.2, 5.7, 3.1 Hz, 1H), 1.83 (d, *J* = 11.5 Hz, 2H), 1.44 (tt, *J* = 12.8, 6.6 Hz, 2H). LCMS (ESI) calcd for C₃₉H₄₄N₇O₆⁺ [M+H]⁺: 706.33, found, 706.40.

### Example 134: preparation of N-(tert-butyl)-3-((2-((4-(4-(((2-(2,6-dioxopiperidin-3-yl)-1-oxoisoindolin-5-yl)methyl)(methyl)amino)piperidin-1-yl)phenyl)amino)-5-methylpyrimidin-4-yl)amino)benzenesulfonamide (GT-03690)

Referring to the method of Step 2 in Scheme 11 and the method of Example 1, the target compound (GT-03690) was prepared using N-(tert-butyl)-3-((5-methyl-2-((4-(4-(methylamino)piperidin-1-yl)phenyl)amino)pyrimidin-4-yl)amino)benzenesulfonamide (GT-D-89) prepared from Intermediate Example 18 and 3-(5-(bromomethyl)-1-oxoisoindolin-2-yl)piperidine-2,6-dione. The target compound (GT-03690) was obtained as a white solid (16 mg, yield 21.06%). ¹H NMR (400 MHz, DMSO) δ 11.01 (s, 1H), 9.98 (s, 1H), 7.99 - 7.92 (m, 3H), 7.89 - 7.80 (m, 3H), 7.72 (d, *J* = 7.9 Hz, 1H), 7.60 (dd, *J* = 17.1, 9.1 Hz, 3H), 7.29 (d, *J* = 8.1 Hz, 2H), 7.19 - 6.96 (m, 2H), 5.14 (dd, *J* = 13.2, 5.0 Hz, 1H), 4.64 - 4.58 (m, 1H), 4.50 (t, *J* = 6.7 Hz, 1H), 4.41 - 4.34 (m, 2H), 3.45 (ddd, *J* = 18.1, 10.6, 6.3 Hz, 2H), 2.96 - 2.79 (m, 3H), 2.70 - 2.56 (m, 5H), 2.44 - 2.27 (m, 3H), 2.18 (s, 3H), 2.02 (dd, *J =* 10.8, 5.7 Hz, 3H), 1.09 (s, 9H). LCMS (ESI) calcd for C₄₁H₅₀N₉O₅S⁺ [M+H]⁺: 780.36, found, 780.40.

### Example 135: preparation of N-(tert-butyl)-3-((2-((4-(4-(4-((2-(2,6-dioxopiperidin-3-yl)-1-oxoisoindolin-5-yl)methyl)piperazin-1-yl)piperidin-1-yl)phenyl)amino)-5-methylpyrimidin-4-yl)amino)benzenesulfonamide (GT-03709)

Referring to the method of Step 2 in Scheme 11 and the method of Example 1, the target compound (GT-03709) was prepared using N-(tert-butyl)-3-((5-methyl-2-((4-(4-(piperazin-1-yl)piperidin-1-yl)phenyl)amino)pyrimidin-4-yl)amino)benzenesulfonamide (GT-D-90) prepared from Intermediate Example 19 and 3-(5-(bromomethyl)-1-oxoisoindolin-2-yl)piperidine-2,6-dione. The target compound (GT-03709) was obtained as a white solid (23 mg, yield 36.54%). ¹H NMR (400 MHz, DMSO) δ 11.01 (s, 1H), 10.48 (s, 1H), 9.96 (s, 1H), 9.40 (s, 1H), 7.96 - 7.86 (m, 4H), 7.80 (s, 2H), 7.72 (d, *J* = 7.9 Hz, 1H), 7.60 (dd, *J* = 18.9, 10.9 Hz, 2H), 7.26 (d, *J* = 8.2 Hz, 2H), 7.02 (s, 2H), 5.14 (dd, *J* = 13.1, 5.2 Hz, 1H), 4.62 - 4.33 (m, 5H), 3.78 (d, *J* = 10.5 Hz, 5H), 3.51 - 3.35 (m, 4H), 3.03 - 2.86 (m, 2H), 2.84 - 2.69 (m, 2H), 2.63 (t, *J* = 16.4 Hz, 2H), 2.48 - 2.34 (m, 2H), 2.18 (s, 3H), 2.02 (dd, *J* = 9.8, 4.7 Hz, 1H), 1.93 - 1.80 (m, 2H), 1.09 (s, 9H). LCMS (ESI) calcd for C₄₄H₅₅N₁₀O₅S⁺ [M+H]⁺: 835.40, found, 835.40.

### Example 136: preparation of 2-((2-((4-(4-(4-((2-(2,6-dioxopiperidin-3-yl)-4-fluoro-1-oxoisoindolin-5-yl)methyl)piperazin-1-yl)piperidin-1-yl)-2-methoxyphenyl)amino)-5-(trifluoromethyl)pyridin-4-yl)amino)-N-methylbenzamide (GT-03595)

Referring to the methods of Scheme 11 and Example 1, the target compound (GT-03595) was prepared using 2-((2-((2-methoxy-4-(4-(piperazin-1-yl)piperidin-1-yl)phenyl)amino)-5-(trifluoromethyl)pyridin-4-yl)amino)-N-methylbenzamide (GT-M-12) prepared from Intermediate Example 27 and 3-(4-fluoro-5-(hydroxymethyl)-1-oxoisoindolin-2-yl)piperidine-2,6-dione (GT-03445). The target compound (GT-03595) was obtained as a white solid (37 mg, yield 59%). ¹H NMR (400 MHz, MeOD) δ 8.03 (s, 1H), 7.89 - 7.81 (m, 1H), 7.78 - 7.70 (m, 2H), 7.66 - 7.55 (m, 2H), 7.42 - 7.33 (m, 1H), 7.31 - 7.22 (m, 1H), 7.08 (s, 1H), 6.94 (d, *J* = 8.8 Hz, 1H), 6.48 (s, 1H), 5.18 (dd, *J* = 13.4, 5.1 Hz, 1H), 4.70 - 4.61 (m, 2H), 4.53 (s, 2H), 4.02 - 3.92 (m, 2H), 3.89 (s, 3H), 3.81 - 3.48 (m, 10H), 3.25 - 3.22 (m, 1H), 3.00 - 2.90 (m, 1H), 2.89 (s, 3H), 2.80 (d, *J* = 15.5 Hz, 1H), 2.54 (qd, *J* = 13.2, 4.8 Hz, 1H), 2.43 - 2.33 (m, 2H), 2.24 - 2.06 (m, 3H). LCMS (ESI) calcd for C₄₄H₄₈F₄N₉O₅⁺ [M+H]⁺: 858.37, found, 858.4.

### Example 137: preparation of 2-((2-((4-(4-(4-((2-(2,6-dioxopiperidin-3-yl)-6-fluoro-1-oxoisoindolin-5-yl)methyl)piperazin-1-yl)piperidin-1-yl)-2-methoxyphenyl)amino)-5-(trifluoromethyl)pyridin-4-yl)amino)-N-methylbenzamide (GT-03596)

The target compound (GT-03596) was prepared using the methods described in Scheme 11 and Example 1 (white solid, 37 mg, yield 60%). ¹H NMR (400 MHz, MeOD) δ8.02 (s, 1H), 7.92 (d, *J* = 6.0 Hz, 1H), 7.72 (d, *J* = 7.7 Hz, 1H), 7.65 (d, *J* = 8.5 Hz, 1H), 7.63 - 7.55 (m, 2H), 7.40 - 7.33 (m, 1H), 7.28 - 7.20 (m, 1H), 7.02 (s, 1H), 6.89 (d, *J* = 7.4 Hz, 1H), 6.48 (s, 1H), 5.17 (dd, *J* = 13.3, 5.1 Hz, 1H), 4.62 - 4.53 (m, 2H), 4.52 - 4.44 (m, 2H), 4.00 - 3.92 (m, 2H), 3.89 (s, 3H), 3.82 - 3.53 (m, 9H), 3.26 - 3.18 (m, 2H), 3.00 - 2.90 (m, 1H), 2.89 (s, 3H), 2.84 - 2.75 (m, 1H), 2.52 (qd, *J* = 13.2, 4.6 Hz, 1H), 2.40 - 2.30 (m, 2H), 2.23 - 2.16 (m, 1H), 2.16 - 2.04 (m, 2H). LCMS (ESI) calcd for C₄₄H₄₈F₄N₉O₅⁺ [M+H]⁺: 858.37, found, 858.4.

### Example 138: preparation of 2-((2-((4-(4-(4-((2-(2,6-dioxopiperidin-3-yl)-7-fluoro-1-oxoisoindolin-5-yl)methyl)piperazin-1-yl)piperidin-1-yl)-2-methoxyphenyl)amino)-5-(trifluoromethyl)pyridin-4-yl)amino)-N-methylbenzamide (GT-03597)

Referring to the methods of Scheme 11 and Example 1, the target compound (GT-03597) was prepared using 2-((2-((2-methoxy-4-(4-(piperazin-1-yl)piperidin-1-yl)phenyl)amino)-5-(trifluoromethyl)pyridin-4-yl)amino)-N-methylbenzamide (GT-M-12) prepared from Intermediate Example 27 and 3-(7-fluoro-5-(hydroxymethyl)-1-oxoisoindolin-2-yl)piperidine-2,6-dione (GT-03446). The target compound (GT-03597) was obtained as a white solid (29 mg, yield 45%). ¹H NMR (400 MHz, MeOD) δ8.02 (s, 1H), 7.73 (d, *J* = 7.5 Hz, 1H), 7.63 (s, 1H), 7.61 - 7.55 (m, 2H), 7.47 (d, *J* = 9.6 Hz, 1H), 7.40 - 7.33 (m, 1H), 7.22 (d, *J* = 8.6 Hz, 1H), 6.97 (s, 1H), 6.84 (d, *J* = 8.5 Hz, 1H), 6.48 (s, 1H), 5.15 (dd, *J* = 13.2, 5.2 Hz, 1H), 4.63 - 4.54 (m, 2H), 4.38 (s, 2H), 4.00 - 3.93 (m, 2H), 3.88 (s, 3H), 3.77 - 3.40 (m, 9H), 3.18 - 3.08 (m, 2H), 2.97 - 2.90 (m, 1H), 2.89 (s, 3H), 2.82 - 2.75 (m, 1H), 2.55 - 2.45 (m, 1H), 2.38 - 2.30 (m, 2H), 2.23 - 2.14 (m, 1H), 2.12 - 1.99 (m, 2H). LCMS (ESI) calcd for C₄₄H₄₈F₄N₉O₅⁺ [M+H]⁺: 858.37, found, 858.4.

### Example 139: preparation of 2-((2-((4-(4-(((2-(2,6-dioxopiperidin-3-yl)-1-oxoisoindolin-5-yl)methyl)(methyl)amino)piperidin-1-yl)-2-methoxyphenyl)amino)-5-(trifluoromethyl)pyridin-4-yl)amino)-N-methylbenzamide (GT-03679)

Referring to the method of Step 2 in Scheme 11 and the method of Example 1, the target compound (GT-03679) was prepared using 2-((2-((2-methoxy-4-(4-(methylamino)piperidin-1-yl)phenyl)amino)-5-(trifluoromethyl)pyridin-4-yl)amino)-N-methylbenzamide (GT-M-172) prepared from Intermediate Example 29 and 3-(5-(bromomethyl)-1-oxoisoindolin-2-yl)piperidine-2,6-dione. The target compound (GT-03679) was obtained as a white solid (103 mg, yield 63%). ¹H NMR (400 MHz, MeOD) δ 8.05 (s, 1H), 7.95 - 7.87 (m, 2H), 7.80 - 7.70 (m, 2H), 7.63 - 7.57 (m, 2H), 7.41 - 7.33 (m, 1H), 7.31 - 7.24 (m, 1H), 7.10 (s, 1H), 6.97 (d, *J* = 7.8 Hz, 1H), 6.50 (s, 1H), 5.19 (dd, *J* = 13.3, 5.1 Hz, 1H), 4.78 (d, *J* = 13.9 Hz, 1H), 4.60 (q, *J* = 17.5 Hz, 2H), 4.44 (d, *J* = 13.5 Hz, 1H), 4.02 - 3.93 (m, 2H), 3.90 (s, 3H), 3.83 - 3.74 (m, 1H), 3.36 - 3.32 (m, 2H), 2.99 - 2.90 (m, 1H), 2.89 (s, 3H), 2.83 (s, 3H), 2.82 - 2.75 (m, 1H), 2.53 (qd, *J* = 13.1, 4.4 Hz, 1H), 2.46 - 2.38 (m, 2H), 2.35 - 2.26 (m, 2H), 2.23 - 2.16 (m, 1H). LCMS (ESI) m/z: calcd for C₄₁H₄₄F₃N₈O₅⁺[M+H]⁺, 785.34; found, 785.3.

### Example 140: preparation of 2-((2-((4-(4-(((2-(2,6-dioxopiperidin-3-yl)-6-fluoro-1-oxoisoindolin-5-yl)methyl)(methyl)amino)piperidin-1-yl)-2-methoxyphenyl)amino)-5-(trifluoromethyl)pyridin-4-yl)amino)-N-methylbenzamide (GT-03729)

The target compound (GT-03729) was prepared using the methods described in Scheme 11 and Example 1 (white solid, 49 mg, yield 68.98%). ¹H NMR (400 MHz, DMSO) δ 11.42 (s, 1H), 11.02 (s, 1H), 10.61 (s, 1H), 10.02 (s, 1H), 8.83 (dd, *J* = 9.6, 5.1 Hz, 1H), 8.24 - 8.09 (m, 2H), 7.78 (d, *J* = 7.8 Hz, 1H), 7.68 (d, *J* = 8.5 Hz, 1H), 7.56 (d, *J* = 3.9 Hz, 2H), 7.34 - 7.18 (m, 2H), 6.90 - 6.44 (m, 3H), 5.15 (dd, *J* = 13.2, 4.9 Hz, 1H), 4.64 (d, *J* = 12.9 Hz, 1H), 4.51 (dd, *J* = 17.3, 11.2 Hz, 1H), 4.38 (dd, *J* = 17.2, 9.8 Hz, 2H), 3.94 (d, *J* = 10.0 Hz, 2H), 3.82 (s, 3H), 3.56 - 3.52 (m, 1H), 2.99 - 2.85 (m, 3H), 2.76 (d, *J* = 4.5 Hz, 3H), 2.69 (s, 3H), 2.61 (d, *J* = 16.9 Hz, 1H), 2.47 - 2.35 (m, 2H), 2.26 (d, *J* = 11.6 Hz, 1H), 2.10 - 1.94 (m, 3H). LCMS (ESI) calcd for C₄₁H₄₃F₄N₈O₅⁺ [M+H]⁺: 803.32, found, 803.30.

### Example 141: preparation of 2-((2-((4-(4-(((2-(2,6-dioxopiperidin-3-yl)-7-fluoro-1-oxoisoindolin-5-yl)methyl)(methyl)amino)piperidin-1-yl)-2-methoxyphenyl)amino)-5-(trifluoromethyl)pyridin-4-yl)amino)-N-methylbenzamide (GT-03730)

Referring to the methods of Scheme 11 and Example 1, the target compound (GT-03730) was prepared using 2-((2-((2-methoxy-4-(4-(methylamino)piperidin-1-yl)phenyl)amino)-5-(trifluoromethyl)pyridin-4-yl)amino)-N-methylbenzamide (GT-M-172) prepared from Intermediate Example 29 and 3-(7-fluoro-5-(hydroxymethyl)-1-oxoisoindolin-2-yl)piperidine-2,6-dione (GT-03446). The target compound (GT-03730) was obtained as a white solid (55 mg, yield 77.42%). ¹H NMR (400 MHz, DMSO) δ 11.66 (s, 1H), 11.02 (s, 1H), 10.61 (s, 1H), 10.03 (s, 1H), 8.90 - 8.78 (m, 1H), 8.18 (s, 1H), 7.84 - 7.75 (m, 3H), 7.56 (d, *J* = 3.9 Hz, 2H), 7.33 - 7.18 (m, 2H), 6.92 - 6.49 (m, 3H), 5.11 (dd, *J* = 13.2, 5.2 Hz, 1H), 4.62 - 4.51 (m, 2H), 4.44 - 4.35 (m, 2H), 3.90 (d, *J* = 10.6 Hz, 2H), 3.82 (s, 3H), 3.49 (s, 1H), 2.99 - 2.86 (m, 3H), 2.75 (d, *J* = 4.5 Hz, 3H), 2.62 (s, 3H), 2.59 (s, 1H), 2.44 - 2.36 (m, 2H), 2.29 (d, *J* = 11.2 Hz, 1H), 2.02 (dd, *J* = 8.9, 3.6 Hz, 3H). LCMS (ESI) calcd for C₄₁H₄₃F₄N₈O₅⁺ [M+H]⁺: 803.32, found, 803.30.

### Example 142: preparation of 2-((2-((4-(4-((2-(2,6-dioxopiperidin-3-yl)-4-fluoro-1-oxoisoindolin-5-yl)methyl)piperazin-1-yl)-2-methoxyphenyl)amino)-5-(trifluoromethyl)pyridin-4-yl)amino)-N-methylbenzamide (GT-03712)

Referring to the methods of Scheme 11 and Example 1, the target compound (GT-03712) was prepared using 2-((2-((2-methoxy-4-(piperazin-1-yl)phenyl)amino)-5-(trifluoromethyl)pyridin-4-yl)amino)-N-methylbenzamide (GT-M-171) prepared from Intermediate Example 28 and 3-(4-fluoro-5-(hydroxymethyl)-1-oxoisoindolin-2-yl)piperidine-2,6-dione (GT-03445). The target compound (GT-03712) was obtained as a white solid (36 mg, yield 55.96%). ¹H NMR (400 MHz, DMSO) δ 12.01 (s, 1H), 11.04 (s, 1H), 10.64 (s, 1H), 10.20 (s, 1H), 9.69 (s, 1H), 8.88 (q, *J* = 4.0 Hz, 1H), 8.17 (s, 1H), 8.06 (t, *J* = 7.0 Hz, 1H), 7.78 (d, *J* = 7.8 Hz, 1H), 7.69 (d, *J* = 7.7 Hz, 1H), 7.55 (d, *J* = 3.9 Hz, 2H), 7.28 (dt, *J* = 8.2, 4.2 Hz, 1H), 7.15 (d, *J* = 8.6 Hz, 1H), 6.73 (d, *J* = 1.8 Hz, 1H), 6.58 (dd, *J* = 8.7, 1.8 Hz, 1H), 6.50 (s, 1H), 5.16 (dd, *J* = 13.2, 5.0 Hz, 1H), 4.64 - 4.54 (m, 3H), 4.46 (d, *J* = 17.6 Hz, 1H), 3.89 (d, *J* = 9.0 Hz, 2H), 3.80 (s, 3H), 3.55 - 3.46 (m, 2H), 3.36 - 3.17 (m, 4H), 2.93 (td, *J* = 13.5, 6.8 Hz, 1H), 2.75 (d, *J* = 4.5 Hz, 3H), 2.61 (d, *J* = 16.8 Hz, 1H), 2.44 (dd, *J* = 13.1, 4.1 Hz, 1H), 2.06 - 1.99 (m, 1H). LCMS (ESI) calcd for C₃₉H₃₉F₄N₈O₅⁺ [M+H]⁺: 775.29, found, 775.30.

### Example 143: preparation of 2-((2-((4-(4-((2-(2,6-dioxopiperidin-3-yl)-6-fluoro-1-oxoisoindolin-5-yl)methyl)piperazin-1-yl)-2-methoxyphenyl)amino)-5-(trifluoromethyl)pyridin-4-yl)amino)-N-methylbenzamide (GT-03716)

The target compound (GT-03716) was prepared using the methods described in Scheme 11 and Example 1 (white solid, 37 mg, yield 57.52%). ¹H NMR (400 MHz, DMSO) δ 11.66 (s, 1H), 11.03 (s, 1H), 10.52 (s, 1H), 8.83 - 8.71 (m, 1H), 8.11 (d, *J* = 6.2 Hz, 2H), 7.73 (dd, *J* = 22.0, 8.1 Hz, 2H), 7.54 (d, *J* = 3.9 Hz, 2H), 7.31 - 7.17 (m, 2H), 6.72 (d, *J* = 2.0 Hz, 1H), 6.61 - 6.42 (m, 2H), 5.16 (dd, *J* = 13.3, 5.1 Hz, 1H), 4.59 - 4.47 (m, 3H), 4.38 (d, *J* = 17.4 Hz, 1H), 3.91 - 3.84 (m, 2H), 3.80 (s, 3H), 3.52 - 3.46 (m, 2H), 3.24 (t, *J* = 11.1 Hz, 4H), 2.98 - 2.88 (m, 1H), 2.75 (d, *J* = 4.6 Hz, 3H), 2.61 (d, *J* = 16.7 Hz, 1H), 2.47 - 2.40 (m, 1H), 2.07 -1.98 (m, 1H). LCMS (ESI) calcd for C₃₉H₃₉F₄N₈O₅⁺ [M+H]⁺: 775.29, found, 775.30.

### Example 144: preparation of 2-((2-((4-(4-((2-(2,6-dioxopiperidin-3-yl)-7-fluoro-1-oxoisoindolin-5-yl)methyl)piperazin-1-yl)-2-methoxyphenyl)amino)-5-(trifluoromethyl)pyridin-4-yl)amino)-N-methylbenzamide (GT-03714)

Referring to the methods of Scheme 11 and Example 1, the target compound (GT-03714) was prepared using 2-((2-((2-methoxy-4-(piperazin-1-yl)phenyl)amino)-5-(trifluoromethyl)pyridin-4-yl)amino)-N-methylbenzamide (GT-M-171) prepared from Intermediate Example 28 and 3-(7-fluoro-5-(hydroxymethyl)-1-oxoisoindolin-2-yl)piperidine-2,6-dione (GT-03446). The target compound (GT-03714) was obtained as a white solid (17 mg, yield 26.97%). ¹H NMR (400 MHz, DMSO) δ 11.97 (s, 1H), 11.03 (s, 1H), 10.51 (s, 1H), 9.34 (s, 2H), 8.87 - 8.70 (m, 1H), 8.14 (s, 1H), 7.75 (t, *J* = 6.1 Hz, 3H), 7.54 (d, *J* = 3.9 Hz, 2H), 7.28 - 7.21 (m, 2H), 6.71 (s, 1H), 6.61 - 6.48 (m, 2H), 5.11 (dd, *J* = 13.2, 5.0 Hz, 1H), 4.54 (d, *J* = 18.1 Hz, 3H), 4.40 (d, *J* = 18.2 Hz, 1H), 3.86 (d, *J* = 11.9 Hz, 2H), 3.80 (s, 3H), 3.41 (d, *J* = 10.2 Hz, 2H), 3.31 - 3.25 (m, 2H), 3.19 (dd, *J* = 11.1, 6.0 Hz, 2H), 2.96 - 2.89 (m, 1H), 2.75 (d, *J* = 4.5 Hz, 3H), 2.64 - 2.58 (m, 1H), 2.42 (dd, *J* = 13.4, 4.5 Hz, 1H), 2.05 - 1.98 (m, 1H). LCMS (ESI) calcd for C₃₉H₃₈F₃N₈O₆⁺ [M+H]⁺: 771.28, found, 771.30.

### Example 145: preparation of 2-((2-((4-(4-(((2-(2,6-dioxopiperidin-3-yl)-1-oxoisoindolin-4-yl)methyl)(methyl)amino)piperidin-1-yl)-2-methoxyphenyl)amino)-5-(trifluoromethyl)pyridin-4-yl)amino)-N-methylbenzamide (GT-03731)

The target compound (GT-03731) was prepared using the methods described in Scheme 11 and Example 1 (white solid, 48 mg, yield 69.11%). ¹H NMR (400 MHz, DMSO) δ 11.38 (s, 1H), 11.05 (s, 1H), 10.62 (s, 1H), 10.10 (s, 1H), 8.86 (d, *J* = 4.5 Hz, 1H), 8.19 (s, 1H), 7.81 (dd, *J* = 17.1, 7.7 Hz, 2H), 7.63 (t, *J* = 6.8 Hz, 1H), 7.56 (d, *J* = 3.9 Hz, 2H), 7.34 - 7.20 (m, 2H), 6.93 - 6.51 (m, 3H), 5.18 (ddd, *J* = 30.8, 17.7, 11.2 Hz, 2H), 4.79 (t, *J* = 17.5 Hz, 1H), 4.63 (dd, *J* = 18.4, 5.9 Hz, 2H), 4.51 (d, *J* = 17.4 Hz, 1H), 4.25 (dd, *J* = 14.6, 6.1 Hz, 1H), 3.93 (d, *J* = 9.0 Hz, 2H), 3.83 (s, 3H), 3.03 - 2.89 (m, 3H), 2.75 (d, *J* = 4.5 Hz, 3H), 2.65 (dd, *J* = 9.6, 4.2 Hz, 3H), 2.44 - 2.29 (m, 3H), 2.15 - 1.96 (m, 3H). LCMS (ESI) calcd for C₄₁H₄₄F₃N₈O₅⁺ [M+H]⁺: 785.33, found, 785.30.

### Example 146: preparation of 2-((2-((4-(4-((2-(2,6-dioxopiperidin-3-yl)-1-oxoisoindolin-4-yl)methyl)piperazin-1-yl)-2-methoxyphenyl)amino)-5-(trifluoromethyl)pyridin-4-yl)amino)-N-methylbenzamide (GT-03718)

The target compound (GT-03718) was prepared using the methods described in Scheme 11 and Example 1 (white solid, 34 mg, yield 54.12%). ¹H NMR (400 MHz, DMSO) δ 11.73 (s, 1H), 11.07 (s, 1H), 10.53 (s, 1H), 8.79 (d, *J* = 3.5 Hz, 1H), 8.19 - 8.02 (m, 2H), 7.85 (d, *J* = 7.4 Hz, 1H), 7.76 (d, *J* = 7.8 Hz, 1H), 7.65 (t, *J* = 7.6 Hz, 1H), 7.55 (d, *J* = 3.9 Hz, 2H), 7.32 - 7.17 (m, 2H), 6.72 (d, *J* = 1.9 Hz, 1H), 6.64 - 6.40 (m, 2H), 5.20 (dd, *J* = 13.2, 5.1 Hz, 1H), 4.98 (d, *J* = 17.4 Hz, 1H), 4.58 - 4.42 (m, 3H), 3.88 (d, *J* = 9.0 Hz, 2H), 3.80 (s, 3H), 3.48 (d, *J* = 8.8 Hz, 2H), 3.37 - 3.28 (m, 4H), 3.01 - 2.91 (m, 1H), 2.75 (d, *J* = 4.5 Hz, 3H), 2.65 (d, *J* = 16.7 Hz, 1H), 2.39 - 2.29 (m, 1H), 2.08 - 2.01 (m, 1H). LCMS (ESI) calcd for C₃₉H₄₀F₃N₈O₅⁺ [M+H]⁺: 756.30, found, 756.30.

### Example 147: preparation of N-(3-(((2-((4-(4-((2-(2,6-dioxopiperidin-3-yl)-1-oxoisoindolin-5-yl)methyl)piperazin-1-yl)phenyl)amino)-5-(trifluoromethyl)pyrimidin-4-yl)amino)methyl)phenyl)-N-methylmethanesulfonamide (GT-03526)

Referring to the method of Step 2 in Scheme 11 and the method of Example 1, the target compound (GT-03526) was prepared using N-methyl-N-(3-(((2-((4-(piperazin-1-yl)phenyl)amino)-5-(trifluoromethyl)pyrimidin-4-yl)amino)methyl)phenyl)methanesulfonamide (GT-M-152) prepared from Intermediate Example 31 and 3-(5-(bromomethyl)-1-oxoisoindolin-2-yl)piperidine-2,6-dione. The target compound (GT-03526) was obtained as a white solid (74 mg, yield 48%). ¹H NMR (400 MHz, MeOD) δ 8.23 (s, 1H), 7.93 (d, *J* = 7.8 Hz, 1H), 7.88 (s, 1H), 7.77 (d, *J* = 7.8 Hz, 1H), 7.41 - 7.15 (m, 6H), 7.11 - 7.00 (m, 2H), 5.19 (dd, *J* = 13.3, 5.1 Hz, 1H), 4.74 - 4.53 (m, 7H), 3.98 - 3.84 (m, 2H), 3.66 - 3.54 (m, 2H), 3.44 - 3.36 (m, 2H), 3.24 (s, 3H), 3.23 - 3.12 (m, 2H), 2.94 - 2.86 (m, 1H), 2.84 (s, 2H), 2.83 - 2.74 (m, 1H), 2.53 (qd, *J* = 13.1, 4.6 Hz, 1H), 2.25 - 2.15 (m, 1H). LCMS (ESI) calcd for C₃₈H₄₁FN₉O₅S⁺ [M+H]⁺: 792.29, found, 792.3.

### Example 148: preparation of N-(3-(((2-((4-(4-(((2-(2,6-dioxopiperidin-3-yl)-1-oxoisoindolin-5-yl)methyl)(methyl)amino)piperidin-1-yl)phenyl)amino)-5-(trifluoromethyl)pyrimidin-4-yl)amino)methyl)pyrazin-2-yl)-N-methylmethanesulfonamide (GT-03705)

Referring to the method of Step 2 in Scheme 11 and the method of Example 1, the target compound (GT-03705) was prepared using N-methyl-N-(3-(((2-((4-(4-(methylamino)piperidin-1-yl)phenyl)amino)-5-(trifluoromethyl)pyrimidin-4-yl)amino)methyl)pyrazin-2-yl)methanesulfonamide (GT-M-174) prepared from Intermediate Example 33 and 3-(5-(bromomethyl)-1-oxoisoindolin-2-yl)piperidine-2,6-dione. The target compound (GT-03705) was obtained as a white solid (27 mg, yield 38.77%). ¹H NMR (400 MHz, DMSO) δ 11.58 (s, 1H), 11.02 (s, 1H), 10.38 (s, 1H), 9.52 (s, 1H), 8.68 (d, *J* = 2.5 Hz, 1H), 8.60 (d, *J* = 2.3 Hz, 1H), 8.40 (s, 1H), 8.01 (d, *J* = 3.6 Hz, 1H), 7.88 (d, *J* = 7.9 Hz, 1H), 7.81 (d, *J* = 7.8 Hz, 1H), 7.56 - 7.43 (m, 2H), 7.40 - 7.31 (m, 1H), 5.15 (dd, *J* = 13.2, 5.0 Hz, 2H), 5.00 (d, *J* = 5.0 Hz, 3H), 4.49 (dddd, *J* = 27.9, 22.8, 12.8, 4.5 Hz, 5H), 3.69 (dd, *J* = 16.1, 6.8 Hz, 2H), 3.19 (d, *J* = 10.1 Hz, 7H), 2.97 - 2.89 (m, 1H), 2.62 (d, *J* = 22.0 Hz, 4H), 2.41 (ddd, *J* = 15.5, 11.7, 4.9 Hz, 5H), 2.04 - 1.98 (m, 1H). LCMS (ESI) calcd for C₃₈H₄₃F₃N₁₁O₅S⁺ [M+H]⁺: 822.30, found, 822.30.

### Example 149: preparation of N-(3-(((2-((4-(4-(4-((2-(2,6-dioxopiperidin-3-yl)-1-oxoisoindolin-5-yl)methyl)piperazin-1-yl)piperidin-1-yl)phenyl)amino)-5-(trifluoromethyl)pyrimidin-4-yl)amino)methyl)pyrazin-2-yl)-N-methylmethanesulfonamide (GT-03706)

Referring to the method of Step 2 in Scheme 11 and the method of Example 1, the target compound (GT-03706) was prepared using N-methyl-N-(3-(((2-((4-(4-(piperazin-1-yl)piperidin-1-yl)phenyl)amino)-5-(trifluoromethyl)pyrimidin-4-yl)amino)methyl)pyrazin-2-yl)methanesulfonamide (GT-M-175) prepared from Intermediate Example 34 and 3-(5-(bromomethyl)-1-oxoisoindolin-2-yl)piperidine-2,6-dione. The target compound (GT-03706) was obtained as a white solid (44 mg, yield 64.63%). ¹H NMR (400 MHz, DMSO) δ 11.00 (s, 1H), 10.31 (dd, *J* = 52.7, 34.8 Hz, 1H), 8.69 (dd, *J* = 7.0, 2.4 Hz, 1H), 8.59 (d, *J* = 2.3 Hz, 1H), 8.39 (d, *J* = 7.7 Hz, 1H), 8.04 - 7.87 (m, 2H), 7.85 - 7.77 (m, 2H), 7.51 - 7.36 (m, 2H), 7.26 - 7.19 (m, 1H), 5.14 (dd, *J* = 13.2, 5.0 Hz, 1H), 4.99 (d, *J* = 4.9 Hz, 2H), 4.65 - 4.31 (m, 8H), 3.79 - 3.65 (m, 6H), 3.22 - 3.14 (m, 7H), 3.01 - 2.80 (m, 2H), 2.63 - 2.58 (m, 1H), 2.47 - 2.34 (m, 2H), 2.28 (dd, *J* = 9.8, 1.9 Hz, 2H), 2.21 - 2.06 (m, 2H), 2.05 - 1.81 (m, 2H). LCMS (ESI) calcd for C₄₁H₄₈F₃N₁₂O₅S⁺ [M+H]⁺: 877.35, found, 877.40.

### Example 150: preparation of N-(3-(((2-((4-(1-((2-(2,6-dioxopiperidin-3-yl)-1-oxoisoindolin-5-yl)methyl)piperidin-4-yl)phenyl)amino)-5-(trifluoromethyl)pyrimidin-4-yl)amino)methyl)phenyl)-N-methylmethanesulfonamide (GT-03742)

Referring to the method of Step 2 in Scheme 11 and the method of Example 1, the target compound (GT-03742) was prepared using N-methyl-N-(3-(((2-((4-(piperidin-4-yl)phenyl)amino)-5-(trifluoromethyl)pyrimidin-4-yl)amino)methyl)phenyl)methanesulfonamide (GT-M-193) prepared from Intermediate Example 36 and 3-(5-(bromomethyl)-1-oxoisoindolin-2-yl)piperidine-2,6-dione. The target compound (GT-03742) was obtained as a white solid (53 mg, yield 34%). ¹H NMR (400 MHz, MeOD) δ 8.20 (s, 1H), 7.94 (d, *J* = 7.8 Hz, 1H), 7.86 (s, 1H), 7.75 (d, *J* = 8.0 Hz, 1H), 7.41 - 7.26 (m, 7H), 7.19 (d, *J* = 7.0 Hz, 1H), 5.19 (dd, *J =* 13.3, 5.2 Hz, 1H), 4.70 (s, 2H), 4.59 (q, *J* = 17.5 Hz, 2H), 4.52 (s, 2H), 3.67 - 3.58 (m, 2H), 3.27 - 3.19 (m, 5H), 2.99 - 2.87 (m, 2H), 2.83 - 2.77 (m, 4H), 2.53 (qd, *J* = 13.2, 4.5 Hz, 1H), 2.22 - 2.02 (m, 5H). LCMS (ESI) m/z: calcd for C₃₉H₄₂F₃N₈O₅S⁺[M+H]⁺, 791.29; found, 791.3.

### Example 151: preparation of N-(3-(difluoromethyl)-1-(1-((2-(2,6-dioxopiperidin-3-yl)-1-oxoisoindolin-5-yl)methyl)piperidin-4-yl)-1H-pyrazol-4-yl)-5-morpholinopyrazolo [1,5-a]pyrimidine-3-carboxamide (GT-03809)

Referring to the method of Step 2 in Scheme 11 and the method of Example 1, the target compound (GT-03809) was prepared using N-(3-(difluoromethyl)-1-(piperidin-4-yl)-1H-pyrazol-4-yl)-5-morpholinopyrazolo[1,5-a]pyrimidine-3-carboxamide hydrochloride (GT-D-101) prepared from Intermediate Example 64 and 3-(5-(bromomethyl)-1-oxoisoindolin-2-yl)piperidine-2,6-dione. The target compound (GT-03809) was obtained as a white solid (38 mg, yield 45%). ¹H NMR (400 MHz, MeOD) δ 8.54 (d, *J* = 7.9 Hz, 1H), 8.41 (s, 1H), 8.34 - 8.29 (m, 1H), 7.95 (d, *J* = 7.8 Hz, 1H), 7.82 (s, 1H), 7.73 (d, *J* = 8.0 Hz, 1H), 7.01-6.74 (m, 1H), 6.81 (d, *J* = 8.0 Hz, 1H), 5.19 (dd, *J* = 13.3, 5.1 Hz, 1H), 4.61 - 4.53 (m, 4H), 3.92 - 3.77 (m, 8H), 3.74 - 3.66 (m, 2H), 3.54 - 3.46 (m, 1H), 3.39 - 3.33 (m, 2H), 3.00 - 2.86 (m, 1H), 2.85 - 2.75 (m, 1H), 2.53 (dt, *J* = 13.0, 9.0 Hz, 1H), 2.47 - 2.33 (m, 4H), 2.25 - 2.15 (m, 1H). LCMS (ESI) m/z: calcd for C₃₄H₃₇F₂N₁₀O₅⁺[M+H]⁺, 703.29; found, 703.3.

### Example 152: preparation of N-(3-(difluoromethyl)-1-(1-((2-(2,6-dioxopiperidin-3-yl)-4-fluoro-1-oxoisoindolin-5-yl)methyl)piperidin-4-yl)-1H-pyrazol-4-yl)-5-morpholinopyrazolo[1,5-a]pyrimidine-3-carboxamide (GT-03810)

Referring to the method of Step 2 in Scheme 11 and the method of Example 1, the target compound (GT-03810) was prepared using N-(3-(difluoromethyl)-1-(piperidin-4-yl)-1H-pyrazol-4-yl)-5-morpholinopyrazolo[1,5-a]pyrimidine-3-carboxamide hydrochloride (GT-D-101) prepared from Intermediate Example 64 and 3-(4-fluoro-5-(hydroxymethyl)-1-oxoisoindolin-2-yl)piperidine-2,6-dione (GT-03445). The target compound (GT-03810) was obtained as a white solid (46 mg, yield 53%). ¹H NMR (400 MHz, MeOD) δ 8.54 (d, *J* = 7.9 Hz, 1H), 8.41 (s, 1H), 8.31 (s, 1H), 7.86 - 7.76 (m, 2H), 7.01 (s, 1H), 6.87 (s, 1H), 6.80 (d, *J* = 8.0 Hz, 1H), 6.74 (s, 1H), 5.20 (dd, *J* = 13.4, 5.1 Hz, 1H), 4.73 - 4.60 (m, 4H), 3.93 - 3.71 (m, 10H), 3.63 - 3.53 (m, 1H), 3.45 - 3.34 (m, 2H), 2.98 - 2.88 (m, 1H), 2.85 - 2.75 (m, 1H), 2.60 - 2.49 (m, 1H), 2.47 - 2.30 (m, 4H), 2.26 - 2.15 (m, 1H). LCMS (ESI) m/z: calcd for C₃₄H₃₆F₃N₁₀O₅⁺[M+H]⁺, 721.28; found, 721.3.

### Example 153: preparation of N-(3-(difluoromethyl)-1-(1-((2-(2,6-dioxopiperidin-3-yl)-6-fluoro-1-oxoisoindolin-5-yl)methyl)piperidin-4-yl)-1H-pyrazol-4-yl)-5-morpholinopyrazolo[1,5-a]pyrimidine-3-carboxamide (GT-03811)

The target compound (GT-03811) was prepared using the methods described in Scheme 11 and Example 1 (white solid, 50 mg, yield 58%). ¹H NMR (400 MHz, MeOD) δ 8.54 (d, *J* = 7.9 Hz, 1H), 8.42 (s, 1H), 8.31 (s, 1H), 7.88 (d, *J* = 5.9 Hz, 1H), 7.71 (d, *J* = 8.5 Hz, 1H), 7.01 - 6.74 (s, 1H), 6.81 (d, *J* = 8.0 Hz, 1H), , 5.19 (dd, *J* = 13.4, 5.1 Hz, 1H), 4.64 - 4.55 (m, 4H), 3.94 - 3.70 (m, 10H), 3.66 - 3.52 (m, 1H), 3.50 - 3.33 (m, 2H), 2.99 - 2.87 (m, 1H), 2.83 - 2.75 (m, 1H), 2.59 - 2.46 (m, 1H), 2.44 - 2.28 (m, 4H), 2.26 - 2.14 (m, 1H). LCMS (ESI) m/z: calcd for C₃₄H₃₆F₃N₁₀O₅⁺[M+H]⁺, 721.28; found, 721.3.

### Example 154: preparation of N-(3-(difluoromethyl)-1-(1-((2-(2,6-dioxopiperidin-3-yl)-7-fluoro-1-oxoisoindolin-5-yl)methyl)piperidin-4-yl)-1H-pyrazol-4-yl)-5-morpholinopyrazolo[1,5-a]pyrimidine-3-carboxamide (GT-03812)

Referring to the methods of Scheme 11 and Example 1, the target compound (GT-03812) was prepared using N-(3-(difluoromethyl)-1-(piperidin-4-yl)-1H-pyrazol-4-yl)-5-morpholinopyrazolo[1,5-a]pyrimidine-3-carboxamide hydrochloride (GT-D-101) prepared from Intermediate Example 64 and 3-(7-fluoro-5-(hydroxymethyl)-1-oxoisoindolin-2-yl)piperidine-2,6-dione (GT-03446). The target compound (GT-03812) was obtained as a white solid (45 mg, yield 52%). ¹H NMR (400 MHz, MeOD) δ 8.53 (d, *J* = 7.9 Hz, 1H), 8.41 (s, 1H), 8.30 (s, 1H), 7.63 (s, 1H), 7.47 (d, *J* = 9.6 Hz, 1H), 7.00 - 6.74 (m, 1H), 6.80 (d, *J* = 7.9 Hz, 1H), 5.16 (dd, *J* = 13.3, 5.1 Hz, 1H), 4.61 - 4.52 (m, 4H), 3.96 - 3.77 (m, 9H), 3.75 - 3.65 (m, 2H), 3.56 - 3.46 (m, 1H), 3.40 - 3.32 (m, 1H), 2.99 - 2.87 (m, 1H), 2.84 - 2.75 (m, 1H), 2.61 - 2.47 (m, 1H), 2.47 - 2.34 (m, 4H), 2.24 - 2.13 (m, 1H). LCMS (ESI) m/z: calcd for C₃₄H₃₆F₃N₁₀O₅⁺[M+H]⁺, 721.28; found, 721.3.

### Example 155: preparation of N-(3-(difluoromethyl)-1-(1-((2-(2,6-dioxopiperidin-3-yl)-1-oxoisoindolin-4-yl)methyl)piperidin-4-yl)-1H-pyrazol-4-yl)-5-morpholinopyrazolo [1,5-a]pyrimidine-3-carboxamide (GT-03816)

The target compound (GT-03816) was prepared using the methods described in Scheme 11 and Example 1 (white solid, 48 mg, yield 57%). ¹H NMR (400 MHz, DMSO) δ 11.07 (s, 1H), 10.82 (s, 1H), 9.42 (s, 1H), 8.83 (d, *J* = 7.9 Hz, 1H), 8.43 (s, 1H), 8.29 (s, 1H), 7.98 (d, *J* = 7.6 Hz, 1H), 7.85 (d, *J* = 7.4 Hz, 1H), 7.66 (t, *J* = 7.6 Hz, 1H), 7.13 (t, *J* = 53.6 Hz, 1H), 6.91 (d, *J* = 8.0 Hz, 1H), 5.20 (dd, *J* = 13.1, 5.1 Hz, 1H), 4.56 - 4.50 (m, 2H), 4.42 (s, 2H), 3.89 - 3.67 (m, 10H), 3.57 - 3.54 (m, 1H), 3.25 (d, *J* = 10.6 Hz, 2H), 3.02 - 2.91 (m, 1H), 2.72 - 2.63 (m, 1H), 2.40 - 2.23 (m, 5H), 2.12 - 2.03 (m, 1H). LCMS (ESI) m/z: calcd for C₃₄H₃₇F₂N₁₀O₅⁺[M+H]⁺, 703.29; found, 703.3.

### Example 156: preparation of 6-(6-(4-((2-(2,6-dioxopiperidin-3-yl)-1-oxoisoindolin-5-yl)methyl)piperazin-1-yl)pyridin-3-yl)-1-isopropyl-N-((6-methyl-2-oxo-4-propyl-1,2-dihydropyridin-3-yl)methyl)-1H-indazole-4-carboxamide (GT-03553)

Referring to the method of Step 2 in Scheme 11 and the method of Example 1, the target compound (GT-03553) was prepared using 1-isopropyl-N-((6-methyl-2-oxo-4-propyl-1,2-dihydropyridin-3-yl)methyl)-6-(6-(piperazin-1-yl)pyridin-3-yl)-1H-indazole-4-carboxamide (GT-S-7) prepared from Intermediate Example 82 and 3-(5-(bromomethyl)-1-oxoisoindolin-2-yl)piperidine-2,6-dione. The target compound (GT-03553) was obtained as a white solid (116 mg, yield 73%). ¹H NMR (400 MHz, MeOD) δ 8.56 (d, *J* = 2.1 Hz, 1H), 8.44 (d, *J* = 9.4 Hz, 1H), 8.40 (s, 1H), 8.09 (s, 1H), 7.95 (d, *J* = 7.9 Hz, 1H), 7.87 (d, *J* = 11.2 Hz, 2H), 7.77 (d, *J* = 7.6 Hz, 1H), 7.41 (d, *J* = 9.3 Hz, 1H), 6.41 (s, 1H), 5.20 (dd, *J* = 13.5, 5.1 Hz, 1H), 5.16 - 5.08 (m, 1H), 4.59 (n, *J* = 24.0, 12.0 Hz, 6H), 3.86 - 3.32 (m, 8H), 2.99 - 2.87 (m, 1H), 2.85 - 2.74 (m, 3H), 2.60 - 2.46 (m, 1H), 2.34 (s, 3H), 2.24 - 2.15 (m, 1H), 1.73 - 1.63 (m, 2H), 1.59 (d, *J* = 6.6 Hz, 6H), 1.05 (t, *J* = 7.3 Hz, 3H). LCMS (ESI) calcd for C₃₇H₃₄N₅O₅⁺ [M+H]⁺: 784.39, found, 784.4.

### Example 157: preparation of 6-(6-(4-(((2-(2,6-dioxopiperidin-3-yl)-1-oxoisoindolin-5-yl)methyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide (GT-03743)

Referring to the method of Step 2 in Scheme 11 and the method of Example 1, the target compound (GT-03743) was prepared using 1-isopropyl-N-((6-methyl-2-oxo-4-propyl-1,2-dihydropyridin-3-yl)methyl)-6-(6-(4-(methylamino)piperidin-1-yl)pyridin-3-yl)-1H-indazole-4-carboxamide (GT-S-8) prepared from Intermediate Example 83 and 3-(5-(bromomethyl)-1-oxoisoindolin-2-yl)piperidine-2,6-dione. The target compound (GT-03743) was obtained as a white solid (37 mg, yield 59%). ¹H NMR (400 MHz, MeOD) δ 8.59 (dd, *J* = 9.6, 2.3 Hz, 1H), 8.46 (d, *J* = 2.2 Hz, 1H), 8.42 (s, 1H), 8.16 (s, 1H), 8.00 - 7.87 (m, 3H), 7.77 (d, *J* = 7.7 Hz, 1H), 7.61 (d, *J* = 9.6 Hz, 1H), 6.53 (s, 1H), 5.21 - 5.10 (m, 2H), 4.80 - 4.73 (m, 1H), 4.66 (s, 2H), 4.59 (d, *J* = 9.2 Hz, 2H), 4.57 - 4.48 (m, 2H), 4.44 (d, *J* = 12.1 Hz, 1H), 3.94 - 3.83 (m, 1H), 3.52 - 3.42 (m, 2H), 2.96 - 2.84 (m, 3H), 2.82 (s, 3H), 2.81 - 2.75 (m, 1H), 2.59 - 2.44 (m, 3H), 2.38 (s, 3H), 2.24 - 2.10 (m, 3H), 1.70 (dq, *J* = 15.0, 7.4 Hz, 2H), 1.60 (d, *J* = 6.6 Hz, 6H), 1.06 (t, *J* = 7.3 Hz, 3H). LCMS (ESI) m/z: calcd for C₄₆H₅₄N₉O₅⁺[M+H]⁺, 812.42; found, 812.4.

### Example 158: preparation of 6-(6-(4-(1-((2-(2,6-dioxopiperidin-3-yl)-1-oxoisoindolin-5-yl)methyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide (GT-03691)

Referring to the method of Step 2 in Scheme 11 and the method of Example 1, the target compound (GT-03691) was prepared using 1-isopropyl-N-((6-methyl-2-oxo-4-propyl-1,2-dihydropyridin-3-yl)methyl)-6-(6-(4-(piperidin-4-yl)piperazin-1-yl)pyridin-3-yl)-1H-indazole-4-carboxamide (GT-S-9) prepared from Intermediate Example 84 and 3-(5-(bromomethyl)-1-oxoisoindolin-2-yl)piperidine-2,6-dione. The target compound (GT-03691) was obtained as a white solid (35 mg, yield 48.32%). ¹H NMR (400 MHz, DMSO) δ 11.89 (s, 1H), 11.01 (s, 1H), 9.58 (s, 3H), 8.71 - 8.61 (m, 2H), 8.37 (s, 1H), 8.27 (d, *J* = 8.2 Hz, 1H), 8.15 (s, 1H), 7.91 - 7.74 (m, 4H), 7.22 (d, *J* = 8.9 Hz, 1H), 5.93 (s, 1H), 5.14 (dd, *J* = 12.8, 5.5 Hz, 2H), 4.63 - 4.31 (m, 9H), 3.63 - 3.58 (m, 4H), 3.25 - 3.15 (m, 2H), 3.05 - 2.88 (m, 3H), 2.57 (dd, *J* = 28.4, 12.2 Hz, 3H), 2.48 - 2.19 (m, 6H), 2.14 (s, 3H), 2.00 (dd, *J* = 11.7, 6.0 Hz, 1H), 1.51 (dd, *J* = 17.0, 7.1 Hz, 9H), 0.87 (t, *J* = 7.3 Hz, 3H). LCMS (ESI) calcd for C₄₉H₅₉N₁₀O₅⁺ [M+H]⁺: 867.46, found, 867.50.

### Example 159: preparation of N-((4,6-dimethyl-2-oxo-1,2-dihydropyridin-3-yl)methyl)-4'-(4-((2-(2,6-dioxopiperidin-3-yl)-1-oxoisoindolin-5-yl)methyl)piperazin-1-yl)-5-(ethyl(tetrahydro-2H-pyran-4-yl)amino)-4-methyl-[1,1'-biphenyl]-3-carboxamide (GT-03744)

Referring to the method of Step 2 in Scheme 11 and the method of Example 1, the target compound (GT-03744) was prepared using N-((4,6-dimethyl-2-oxo-1,2-dihydropyridin-3-yl)methyl)-5-(ethyl(tetrahydro-2H-pyran-4-yl)amino)-4-methyl-4'-(piperazin-1-yl)-[1,1'-biphenyl]-3-carboxamide (GT-S-11) prepared from Intermediate Example 86 and 3-(5-(bromomethyl)-1-oxoisoindolin-2-yl)piperidine-2,6-dione. The target compound (GT-03744) was obtained as a white solid (92 mg, yield 59%). ¹H NMR (400 MHz, MeOD) δ 8.59 (dd, *J* = 9.6, 2.3 Hz, 1H), 8.46 (d, *J* = 2.2 Hz, 1H), 8.42 (s, 1H), 8.16 (s, 1H), 8.00 - 7.87 (m, 3H), 7.77 (d, *J* = 7.7 Hz, 1H), 7.61 (d, *J* = 9.6 Hz, 1H), 6.53 (s, 1H), 5.21 - 5.10 (m, 2H), 4.80 - 4.73 (m, 1H), 4.66 (s, 2H), 4.59 (d, *J* = 9.2 Hz, 2H), 4.57 - 4.48 (m, 2H), 4.44 (d, *J* = 12.1 Hz, 1H), 3.94 - 3.83 (m, 1H), 3.52 - 3.42 (m, 2H), 2.96 - 2.84 (m, 3H), 2.82 (s, 3H), 2.81 - 2.75 (m, 1H), 2.59 - 2.44 (m, 3H), 2.38 (s, 3H), 2.24 - 2.10 (m, 3H), 1.70 (dq, *J* = 15.0, 7.4 Hz, 2H), 1.60 (d, *J* = 6.6 Hz, 6H), 1.06 (t, *J* = 7.3 Hz, 3H). LCMS (ESI) m/z: calcd for C₄₆H₅₄N₉O₅⁺[M+H]⁺, 812.42; found, 812.4.

### Example 160: preparation of N-((4,6-dimethyl-2-oxo-1,2-dihydropyridin-3-yl)methyl)-4'-(4-(((2-(2,6-dioxopiperidin-3-yl)-1-oxoisoindolin-5-yl)methyl)(methyl)amino)piperidin-1-yl)-5-(ethyl(tetrahydro-2H-pyran-4-yl)amino)-4-methyl-[1,1'-biphenyl]-3-carboxamide (GT-03710)

Referring to the method of Step 2 in Scheme 11 and the method of Example 1, the target compound (GT-03710) was prepared using N-((4,6-dimethyl-2-oxo-1,2-dihydropyridin-3-yl)methyl)-5-(ethyl(tetrahydro-2H-pyran-4-yl)amino)-4-methyl-4'-(4-(methylamino)piperidin-1-yl)-[1,1'-biphenyl]-3-carboxamide (GT-S-12) prepared from Intermediate Example 87 and 3-(5-(bromomethyl)-1-oxoisoindolin-2-yl)piperidine-2,6-dione. The target compound (GT-03710) was obtained as a white solid (45 mg, yield 61.36%). ¹H NMR (400 MHz, DMSO) δ 11.66 (s, 1H), 11.01 (s, 1H), 10.77 (s, 1H), 9.72 (s, 1H), 8.48 (s, 1H), 8.02 (s, 2H), 7.84 (dd, *J* = 33.8, 7.6 Hz, 4H), 7.62 (dd, *J* = 9.1, 4.2 Hz, 1H), 7.46 - 7.25 (m, 2H), 5.92 (s, 1H), 5.14 (dd, *J* = 12.8, 4.5 Hz, 1H), 4.60 (d, *J* = 12.5 Hz, 2H), 4.54 - 4.47 (m, 2H), 4.46 - 4.36 (m, 3H), 4.31 (d, *J* = 3.7 Hz, 2H), 4.07 - 3.73 (m, 7H), 3.63 - 3.49 (m, 3H), 3.24 (s, 2H), 3.14 - 3.05 (m, 3H), 2.92 (t, *J* = 13.1 Hz, 1H), 2.63 (s, 3H), 2.50 (s, 3H), 2.46 - 2.37 (m, 3H), 2.23 (s, 3H), 2.13 (s, 3H), 2.03 (dd, *J* = 17.3, 11.5 Hz, 2H), 1.32 (s, 3H). LCMS (ESI) calcd for C₄₉H₆₀N₇O₆⁺ [M+H]⁺: 842.45, found, 842.50.

### Example 161: preparation of N-((4,6-dimethyl-2-oxo-1,2-dihydropyridin-3-yl)methyl)-4'-(4-(1-((2-(2,6-dioxopiperidin-3-yl)-1-oxoisoindolin-5-yl)methyl)piperidin-4-yl)piperazin-1-yl)-5-(ethyl(tetrahydro-2H-pyran-4-yl)amino)-4-methyl-[1,1'-biphenyl]-3-carboxamide (GT-03711)

Referring to the method of Step 2 in Scheme 11 and the method of Example 1, the target compound (GT-03711) was prepared using N-((4,6-dimethyl-2-oxo-1,2-dihydropyridin-3-yl)methyl)-5-(ethyl(tetrahydro-2H-pyran-4-yl)amino)-4-methyl-4'-(4-(piperidin-4-yl)piperazin-1-yl)-[1,1'-biphenyl]-3-carboxamide (GT-S-13) prepared from Intermediate Example 88 and 3-(5-(bromomethyl)-1-oxoisoindolin-2-yl)piperidine-2,6-dione. The target compound (GT-03711) was obtained as a white solid (30 mg, yield 42.01%). ¹H NMR (400 MHz, DMSO) δ 11.78 (s, 1H), 11.44 (s, 1H), 11.01 (s, 1H), 9.53 (s, 1H), 8.47 (s, 1H), 8.11 - 7.91 (m, 1H), 7.89 (s, 1H), 7.82 (d, *J* = 7.9 Hz, 1H), 7.75 (dd, *J* = 14.3, 6.0 Hz, 2H), 7.59 (d, *J* = 7.1 Hz, 1H), 7.10 (t, *J* = 7.3 Hz, 2H), 5.90 (s, 1H), 5.15 (dd, *J =* 13.1, 5.0 Hz, 1H), 4.55 - 4.34 (m, 7H), 3.94 (d, *J* = 11.9 Hz, 4H), 3.87 - 3.73 (m, 3H), 3.56 - 3.48 (m, 5H), 3.26 (dd, *J* = 26.8, 13.8 Hz, 7H), 3.04 - 2.88 (m, 3H), 2.61 (d, *J* = 18.8 Hz, 1H), 2.41 - 2.34 (m, 3H), 2.29 (d, *J* = 11.7 Hz, 2H), 2.23 (s, 3H), 2.12 (s, 3H), 2.03 - 1.99 (m, 1H), 1.65 (dd, *J* = 42.8, 19.4 Hz, 2H), 1.31 (d, *J* = 6.6 Hz, 6H). LCMS (ESI) calcd for C₅₂H₆₅N₈O₆⁺ [M+H]⁺: 897.49, found, 897.50.

### Example 162: preparation of N-(5-(3,5-difluorobenzyl)-1H-indazol-3-yl)-4-(4-((2-(2,6-dioxopiperidin-3-yl)-1-oxoisoindolin-5-yl)methyl)piperazin-1-yl)-2-((tetrahydro-2H-pyran-4-yl)amino)benzamide (GT-03807)

Referring to the method of Step 2 in Scheme 11 and the method of Example 1, the target compound (GT-03807) was prepared using N-(5-(3,5-difluorobenzyl)-1H-indazol-3-yl)-4-(piperazin-1-yl)-2-((tetrahydro-2H-pyran-4-yl)amino)benzamide (GT-S-14) prepared from Intermediate Example 89 and 3-(5-(bromomethyl)-1-oxoisoindolin-2-yl)piperidine-2,6-dione. The target compound (GT-03807) was obtained as a white solid (35 mg, yield 45%). ¹H NMR (400 MHz, MeOD) δ 7.95 (d, *J* = 7.8 Hz, 1H), 7.91 (t, *J* = 4.4 Hz, 1H), 7.85 (s, 1H), 7.75 (d, *J* = 7.7 Hz, 1H), 7.65 (s, 1H), 7.46 (d, *J* = 8.6 Hz, 1H), 7.31 (d, *J* = 8.7 Hz, 1H), 6.88 - 6.79 (m, 2H), 6.78 - 6.70 (m, 1H), 5.20 (dd, *J* = 13.2, 5.1 Hz, 1H), 4.67 - 4.53 (m, 4H), 4.20 - 4.02 (m, 4H), 4.01 - 3.89 (m, 2H), 3.84 - 3.72 (m, 1H), 3.70 - 3.32 (m, 10H), 2.99 - 2.87 (m, 1H), 2.84 - 2.75 (m, 1H), 2.62 - 2.44 (m, 1H), 2.27 - 2.15 (m, 1H), 2.07 - 1.97 (m, 2H), 1.66 - 1.53 (m, 2H). LCMS (ESI) m/z: calcd for C₄₄H₄₅F₂N₈O₅⁺[M+H]⁺, 803.35; found, 803.3.

### Example 163: preparation of N-(5-(3,5-difluorobenzyl)-1H-indazol-3-yl)-4-(4-(((2-(2,6-dioxopiperidin-3-yl)-1-oxoisoindolin-5-yl)methyl)(methyl)amino)piperidin-1-yl)-2-((tetrahydro-2H-pyran-4-yl)amino)benzamide (GT-03806)

Referring to the method of Step 2 in Scheme 11 and the method of Example 1, the target compound (GT-03806) was prepared using N-(5-(3,5-difluorobenzyl)-1H-indazol-3-yl)-4-(4-(methylamino)piperidin-1-yl)-2-((tetrahydro-2H-pyran-4-yl)amino)benzamide (GT-S-15) prepared from Intermediate Example 90 and 3-(5-(bromomethyl)-1-oxoisoindolin-2-yl)piperidine-2,6-dione. The target compound (GT-03806) was obtained as a white solid (25 mg, yield 32%). ¹H NMR (400 MHz, MeOD) δ 7.98 - 7.91 (m, 2H), 7.82 (s, 1H), 7.73 (d, *J* = 7.9 Hz, 1H), 7.65 (s, 1H), 7.47 (d, *J* = 8.7 Hz, 1H), 7.31 (d, *J* = 8.7 Hz, 1H), 6.88 - 6.80 (m, 2H), 6.78 - 6.70 (m, 1H), 5.19 (dd, *J* = 13.3, 5.1 Hz, 1H), 4.63 - 4.55 (m, 2H), 4.39 (d, *J* = 12.5 Hz, 1H), 4.25 - 4.15 (m, 2H), 4.09 (s, 2H), 4.03 - 3.92 (m, 2H), 3.82 - 3.75 (m, 1H), 3.73 - 3.63 (m, 1H), 3.55 - 3.48 (m, 2H), 3.08 - 3.00 (m, 2H), 2.95 - 2.87 (m, 1H), 2.81 (s, 3H), 2.59 - 2.46 (m, 1H), 2.36 - 2.25 (m, 2H), 2.25 - 2.15 (m, 1H), 2.09 - 1.94 (m, 5H), 1.74 -1.62 (m, 2H). LCMS (ESI) m/z: calcd for C₄₆H₄₉F₂N₈O₅⁺[M+H]⁺, 831.38; found, 831.4.

### Example 164: preparation of N-(5-(3,5-difluorobenzyl)-1H-indazol-3-yl)-4-(4-(((2-(2,6-dioxopiperidin-3-yl)-4-fluoro-1-oxoisoindolin-5-yl)methyl)(methyl)amino)piperidin-1-yl)-2-((tetrahydro-2H-pyran-4-yl)amino)benzamide (GT-03961)

Referring to the methods of Scheme 11 and Example 1, the target compound (GT-03961) was prepared using N-(5-(3,5-difluorobenzyl)-1H-indazol-3-yl)-4-(4-(methylamino)piperidin-1-yl)-2-((tetrahydro-2H-pyran-4-yl)amino)benzamide (GT-S-15) prepared from Intermediate Example 90 and 3-(4-fluoro-5-(hydroxymethyl)-1-oxoisoindolin-2-yl)piperidine-2,6-dione (GT-03445). The target compound (GT-03961) was obtained as a white solid (27 mg, yield 34%). ¹H NMR (400 MHz, DMSO) δ 12.68 (s, 1H), 11.04 (s, 1H), 10.48 (d, *J* = 26.7 Hz, 1H), 10.15 (s, 1H), 7.97 - 7.89 (m, 1H), 7.83 (d, *J* = 9.1 Hz, 1H), 7.73 (d, *J* = 7.7 Hz, 1H), 7.48 (s, 1H), 7.41 (d, *J* = 8.6 Hz, 1H), 7.26 (d, *J* = 8.6 Hz, 1H), 7.07 - 6.94 (m, 3H), 6.32 (d, *J* = 9.0 Hz, 1H), 6.22 (s, 1H), 5.16 (dd, *J* = 13.1, 5.0 Hz, 1H), 4.75 - 4.56 (m, 2H), 4.53 - 4.32 (m, 2H), 4.13 - 4.01 (m, 4H), 3.83 (d, *J* = 11.6 Hz, 2H), 3.53 - 3.46 (m, 2H), 2.98 - 2.79 (m, 3H), 2.71 (s, 3H), 2.64 - 2.59 (m, 1H), 2.46 - 2.41 (m, 1H), 2.31 - 2.14 (m, 2H), 2.08 - 1.78 (m, 6H), 1.43 - 1.30 (m, 2H). LCMS (ESI) m/z: calcd for C₄₆H₄₈F₃N₈O₅⁺[M+H]⁺, 849.37; found, 849.4.

### Example 165: preparation of N-(5-(3,5-difluorobenzyl)-1H-indazol-3-yl)-4-(4-(((2-(2,6-dioxopiperidin-3-yl)-6-fluoro-1-oxoisoindolin-5-yl)methyl)(methyl)amino)piperidin-1-yl)-2-((tetrahydro-2H-pyran-4-yl)amino)benzamide (GT-03962)

The target compound (GT-03962) was prepared using the methods described in Scheme 11 and Example 1 (white solid, 30 mg, yield 38%). ¹H NMR (400 MHz, DMSO) δ 12.69 (s, 1H), 11.04 (s, 1H), 10.58 (s, 1H), 10.16 (s, 1H), 8.02 (d, *J* = 5.6 Hz, 1H), 7.84 (d, *J* = 9.0 Hz, 1H), 7.71 (d, *J* = 8.5 Hz, 1H), 7.48 (s, 1H), 7.41 (d, *J* = 8.6 Hz, 1H), 7.26 (d, *J* = 9.7 Hz, 1H), 7.04 - 6.93 (m, 3H), 6.34 (d, *J* = 8.4 Hz, 1H), 6.24 (s, 1H), 5.16 (dd, *J* = 12.8, 4.6 Hz, 1H), 4.67 (d, *J* = 12.6 Hz, 1H), 4.58 - 4.47 (m, 1H), 4.44 - 4.28 (m, 2H), 4.15 - 3.99 (m, 4H), 3.87 - 3.79 (m, 3H), 3.54 - 3.42 (m, 3H), 3.00 - 2.79 (m, 3H), 2.70 (s, 3H), 2.65 - 2.57 (m, 1H), 2.46 - 2.39 (m, 1H), 2.34 - 2.14 (m, 2H), 2.07 - 1.81 (m, 5H), 1.44 - 1.3 (m, 2H). LCMS (ESI) m/z: calcd for C₄₆H₄₈F₃N₈O₅⁺[M+H]⁺, 849.37; found, 849.4.

### Example 166: preparation of N-(5-(3,5-difluorobenzyl)-1H-indazol-3-yl)-4-(4-(((2-(2,6-dioxopiperidin-3-yl)-7-fluoro-1-oxoisoindolin-5-yl)methyl)(methyl)amino)piperidin-1-yl)-2-((tetrahydro-2H-pyran-4-yl)amino)benzamide (GT-03963)

Referring to the methods of Scheme 11 and Example 1, the target compound (GT-03963) was prepared using N-(5-(3,5-difluorobenzyl)-1H-indazol-3-yl)-4-(4-(methylamino)piperidin-1-yl)-2-((tetrahydro-2H-pyran-4-yl)amino)benzamide (GT-S-15) prepared from Intermediate Example 90 and 3-(7-fluoro-5-(hydroxymethyl)-1-oxoisoindolin-2-yl)piperidine-2,6-dione (GT-03446). The target compound (GT-03963) was obtained as a white solid (28 mg, yield 36%). ¹H NMR (400 MHz, DMSO) δ 12.67 (s, 1H), 11.03 (s, 1H), 10.62 (s, 1H), 10.14 (s, 1H), 7.83 (d, *J* = 9.0 Hz, 1H), 7.77 - 7.64 (m, 2H), 7.48 (s, 1H), 7.41 (d, *J* = 8.6 Hz, 1H), 7.26 (d, *J* = 8.7 Hz, 1H), 7.05 - 6.96 (m, 3H), 6.31 (d, *J* = 9.0 Hz, 1H), 6.20 (s, 1H), 5.12 (dd, *J* = 13.1, 5.2 Hz, 1H), 4.67 - 4.27 (m, 5H), 4.13 - 3.99 (m, 5H), 3.88 - 3.79 (m, 3H), 3.54 - 3.45 (m, 4H), 2.99 - 2.76 (m, 4H), 2.64 (s, 3H), 2.60 - 2.56 (m, 1H), 2.44 - 2.38 (m, 1H), 2.29 - 2.15 (m, 2H), 2.07 - 1.73 (m, 6H), 1.42 - 1.30 (m, 2H). LCMS (ESI) m/z: calcd for C₄₆H₄₈F₃N₈O₅⁺[M+H]⁺, 849.37; found, 849.4.

### Example 167: preparation of N-(5-(3,5-difluorobenzyl)-1H-indazol-3-yl)-4-(4-(((2-(2,6-dioxopiperidin-3-yl)-1-oxoisoindolin-4-yl)methyl)(methyl)amino)piperidin-1-yl)-2-((tetrahydro-2H-pyran-4-yl)amino)benzamide (GT-03967)

The target compound (GT-03967) was prepared using the methods described in Scheme 11 and Example 1 (white solid,30 mg, yield 39%). ¹H NMR (400 MHz, DMSO) δ 12.67 (s, 1H), 11.06 (s, 1H), 10.49 (d, *J* = 60.6 Hz, 1H), 10.14 (s, 1H), 7.94 (dd, *J* = 13.8, 7.4 Hz, 1H), 7.89 - 7.82 (m, 2H), 7.70 - 7.62 (m, 1H), 7.48 (s, 1H), 7.41 (d, *J* = 8.6 Hz, 1H), 7.29 - 7.24 (m, 1H), 7.02 - 6.94 (m, 3H), 6.33 (d, *J* = 8.5 Hz, 1H), 6.23 (s, 1H), 5.28 - 5.13 (m, 1H), 4.91 (d, *J* = 17.3 Hz, 1H), 4.74 - 4.51 (m, 3H), 4.25 - 4.16 (m, 1H), 4.16 - 4.01 (m, 5H), 3.86 - 3.79 (m, 3H), 3.49 (t, *J* = 9.9 Hz, 3H), 3.03 - 2.81 (m, 4H), 2.73 - 2.61 (m, 5H), 2.42 - 2.20 (m, 5H), 2.08 - 1.99 (m, 2H), 1.98 - 1.82 (m, 5H), 1.44 - 1.35 (m, 2H). LCMS (ESI) m/z: calcd for C₄₆H₄₉F₂N₈O₅⁺[M+H]⁺, 831.38; found, 831.4.

### Example 168: preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-4-fluoro-1-oxoisoindolin-5-yl)methyl)piperazin-1-yl)-1-(phenylthio)butan-2-yl)amino)-3-((trifluoromethyl)sulfonyl)phenyl)sulfonyl)benzamide (GT-03478)

Referring to the methods of Scheme 11 and Example 1, the target compound (GT-03478) was prepared using (R)-4-(4-((4'-chloro-4,4-dimethyl-3,4,5,6-tetrahydro-[1,1'-biphenyl]-2-yl)methyl)piperazin-1-yl)-N-((4-((1-(phenylthio)-4-(piperazin-1-yl)butan-2-yl)amino)-3-((trifluoromethyl)sulfonyl)phenyl)sulfonyl)benzamide (CAS NO.: 2143096-93-7) and 3-(4-fluoro-5-(hydroxymethyl)-1-oxoisoindolin-2-yl)piperidine-2,6-dione (GT-03445). The target compound (GT-03478) was obtained as a white solid (5 mg, yield 38%). ¹H NMR (400 MHz, MeOD) δ 8.30 (d, *J* = 2.0 Hz, 1H), 8.11 - 8.02 (m, 1H), 7.75 (dd, *J* = 9.0, 2.6 Hz, 2H), 7.71 - 7.62 (m, 2H), 7.40 (d, *J* = 8.4 Hz, 2H), 7.33 (d, *J* = 7.4 Hz, 2H), 7.22 - 7.09 (m, 5H), 7.04 - 6.92 (m, 3H), 5.15 (dd, *J* = 13.3, 3.0 Hz, 1H), 4.65 - 4.51 (m, 2H), 4.19 - 4.02 (m, 3H), 3.89 (d, *J* = 14.4 Hz, 2H), 3.71 (s, 2H), 3.53 (d, *J* = 11.4 Hz, 2H), 3.26 - 2.76 (m, 16H), 2.56 - 2.45 (m, 1H), 2.44 - 2.37 (m, 2H), 2.31 - 2.15 (m, 3H), 2.11 (s, 2H), 1.58 (t, *J* = 6.4 Hz, 2H), 1.41 - 1.35 (m, 1H), 1.30 (t, *J* = 7.2 Hz, 1H), 1.08 (s, 6H). LCMS (ESI) calcd for C₆₁H₅₈ClF₄N₈O₈S₃⁺ [M+H]⁺: 1247.39, found, 1247.4.

### Example 169: preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-7-fluoro-1-oxoisoindolin-5-yl)methyl)piperazin-1-yl)-1-(phenylthio)butan-2-yl)amino)-3-((trifluoromethyl)sulfonyl)phenyl)sulfonyl)benzamide (GT-03479)

Referring to the methods of Scheme 11 and Example 1, the target compound (GT-03479) was prepared using (R)-4-(4-((4'-chloro-4,4-dimethyl-3,4,5,6-tetrahydro-[1,1'-biphenyl]-2-yl)methyl)piperazin-1-yl)-N-((4-((1-(phenylthio)-4-(piperazin-1-yl)butan-2-yl)amino)-3-((trifluoromethyl)sulfonyl)phenyl)sulfonyl)benzamide (CAS NO.: 2143096-93-7) and 3-(7-fluoro-5-(hydroxymethyl)-1-oxoisoindolin-2-yl)piperidine-2,6-dione (GT-03446). The target compound (GT-03479) was obtained as a white solid (5 mg, yield 38%). ¹H NMR (400 MHz, MeOD) δ 8.31 (s, 1H), 8.10 - 8.04 (m, 1H), 7.75 (dd, *J* = 9.0, 2.6 Hz, 2H), 7.48 (s, 1H), 7.39 (d, *J* = 8.4 Hz, 2H), 7.36 - 7.29 (m, 3H), 7.22 - 7.12 (m, 5H), 6.99 (d, *J* = 8.5 Hz, 3H), 5.11 (dd, *J* = 13.3, 5.1 Hz, 1H), 4.55 - 4.46 (m, 2H), 4.20 - 4.09 (m, 1H), 4.05 - 3.95 (m, 2H), 3.89 (d, *J* = 13.5 Hz, 2H), 3.53 (d, *J* = 11.7 Hz, 3H), 3.42 - 3.34 (m, 3H), 3.23 - 2.86 (m, 16H), 2.81 - 2.74 (m, 1H), 2.54 - 2.44 (m, 1H), 2.43 - 2.37 (m, 2H), 2.35 - 2.22 (m, 1H), 2.22 - 2.09 (m, 4H), 1.58 (t, *J* = 6.5 Hz, 2H), 1.31 - 1.18 (m, 2H), 1.08 (s, 6H). LCMS (ESI) calcd for C₆₁H₆₈ClF₄N₈O₈S₃⁺ [M+H]⁺: 1247.39, found, 1247.4.

### Example 170: preparation of 3-(5-((4-((4-(((R)-1-(3-amino-5-(trifluoromethyl)phenyl)ethyl)amino)-7-methoxy-2-methylquinazolin-6-yl)oxy)piperidin-1-yl)methyl)-1-oxoisoindolin-2-yl)piperidine-2,6-dione (GT-03958)

The target compound (GT-03958) was prepared using the methods described in Scheme 11 and Example 1 (white solid, 8 mg, yield 10%). ¹H NMR (400 MHz, DMSO) δ 11.00 (s, 1H), 10.64 (s, 1H), 7.91 (d, *J* = 10.3 Hz, 1H), 7.87 - 7.76 (m, 2H), 7.18 (d, *J* = 18.7 Hz, 1H), 7.00 - 6.89 (m, 2H), 6.76 (s, 1H), 5.78 - 5.64 (m, 1H), 5.15 (dd, *J* = 12.8, 5.0 Hz, 1H), 4.55 - 4.48 (m, 2H), 3.95 - 3.89 (m, 3H), 3.24 - 3.09 (m, 3H), 2.97 - 2.86 (m, 1H), 2.69 - 2.61 (m, 2H), 2.34 - 2.23 (m, 3H), 2.06 - 1.98 (m, 2H), 1.75 - 1.64 (m, 3H). LCMS (ESI) m/z: calcd for C₃₈H₄₀F₃N₇O₅⁺[M+H]⁺, 731.30; found, 731.3.

### Example 171: preparation of 6-(4-(((2-(2,6-dioxopiperidin-3-yl)-1,3-dioxoisoindolin-5-yl)methyl)(methyl)amino)piperidin-1-yl)-2-(4-phenoxyphenyl)nicotinamide (GT-03827)

Referring to the method of Step 2 in Scheme 11 and the method of Example 1, the target compound (GT-03827) was prepared using 6-(4-(methylamino)piperidin-1-yl)-2-(4-phenoxyphenyl)nicotinamide (GT-D-77) prepared from Intermediate Example 52 and 5-(bromomethyl)-2-(2,6-dioxo-piperidin-3-yl)isoindoline-1,3-dione (CAS NO.: 1312023-72-5). The target compound (GT-03827) was obtained as a white solid (54 mg, yield 60%). ¹H NMR (400 MHz, DMSO) δ 11.15 (s, 1H), 10.86 (s, 1H), 8.28 (s, 1H), 8.16 (d, *J* = 7.7 Hz, 1H), 8.03 (d, *J* = 7.7 Hz, 1H), 7.69 - 7.62 (m, 3H), 7.54 (s, 1H), 7.47 - 7.39 (m, 2H), 7.23 (s, 1H), 7.18 (t, *J* = 7.4 Hz, 1H), 7.07 (dd, *J* = 8.6, 1.0 Hz, 2H), 7.05 - 6.99 (m, 2H), 6.90 (d, *J* = 8.8 Hz, 1H), 5.18 (dd, *J* = 12.8, 5.4 Hz, 1H), 4.75 - 4.56 (m, 3H), 4.47 - 4.36 (m, 1H), 3.66 - 3.54 (m, 1H), 2.96 - 2.82 (m, 3H), 2.66 - 2.62 (m, 1H), 2.59 (d, *J* = 4.4 Hz, 3H), 2.57 - 2.52 (m, 1H), 2.31 - 2.19 (m, 2H), 2.13 - 2.03 (m, 1H), 1.83 - 1.68 (m, 2H). LCMS (ESI) m/z: calcd for C₄₆H₅₅N₉O₁₀S₂⁺[M+H]⁺, 958.33; found, 958.3.

### Example 172: preparation of 6-(4-(4-((2-(2,6-dioxopiperidin-3-yl)-1,3-dioxoisoindolin-5-yl)methyl)piperazin-1-yl)piperidin-1-yl)-2-(4-phenoxyphenyl)nicotinamide (GT-03752)

Referring to the method of Step 2 in Scheme 11 and the method of Example 1, the target compound (GT-03752) was prepared using 2-(4-phenoxyphenyl)-6-(4-(piperazin-1-yl)piperidin-1-yl)nicotinamide (GT-D-78) prepared from Intermediate Example 53 and 5-(bromomethyl)-2-(2,6-dioxo-piperidin-3-yl)isoindoline-1,3-dione (CAS NO.: 1312023-72-5). The target compound (GT-03752) was obtained as a white solid (31 mg, yield 46%). ¹H NMR (400 MHz, MeOD) δ 7.97 (s, 1H), 7.92 - 7.81 (m, 3H), 7.65 (d, *J* = 8.7 Hz, 2H), 7.43 -7.36 (m, 2H), 7.16 (t, *J* = 7.5 Hz, 1H), 7.07 - 6.95 (m, 5H), 5.15 (dd, *J* = 12.6, 5.5 Hz, 1H), 4.70 - 4.59 (m, 2H), 3.95 (s, 1H), 3.65 - 3.32 (m, 8H), 3.17 - 3.03 (m, 3H), 2.95 - 2.81 (m, 1H), 2.80 - 2.62 (m, 3H), 2.31 - 2.20 (m, 2H), 2.17 - 2.11 (m, 1H), 1.86 - 1.70 (m, 2H). LCMS (ESI) m/z: calcd for C₄₁H₄₂N₇O₆⁺[M+H]⁺, 728.32; found, 728.3.

### Example 173: preparation of 5-((4-(4-((9-cyclopentyl-8-(phenylamino)-9H-purin-2-yl)amino)phenyl)piperazin-1-yl)methyl)-2-(2,6-dioxopiperidin-3-yl)isoindoline-1,3-dione (GT-03739)

Referring to the method of Step 2 in Scheme 11 and the method of Example 1, the target compound (GT-03739) was prepared using 9-cyclopentyl-N⁸-phenyl-N²-(4-(piperazin-1-yl)phenyl)-9H-purine-2,8-diamine (GT-D-62) prepared according to Intermediate Example 47 and 5-(bromomethyl)-2-(2,6-dioxo-piperidin-3-yl)isoindoline-1,3-dione (CAS NO.: 1312023-72-5). The target compound (GT-03739) was obtained as a white solid (26 mg, yield 35.23%). ¹H NMR (400 MHz, DMSO) δ 11.69 (s, 1H), 11.15 (s, 1H), 10.12 (s, 1H), 9.95 (s, 1H), 8.41 (s, 1H), 8.30 (s, 1H), 8.18 (dd, *J* = 7.7, 0.8 Hz, 1H), 8.05 (d, *J* = 7.6 Hz, 1H), 7.82 (d, *J* = 8.0 Hz, 2H), 7.48 (d, *J* = 8.9 Hz, 2H), 7.39 (t, *J* = 8.0 Hz, 2H), 7.11 (t, *J* = 7.4 Hz, 1H), 7.01 (d, *J* = 8.9 Hz, 2H), 5.22 - 5.12 (m, 2H), 4.61 (s, 2H), 3.77 (d, *J =* 8.9 Hz, 2H), 3.43 (d, *J =* 10.7 Hz, 2H), 3.20 (s, 4H), 2.95 - 2.87 (m, 1H), 2.67 - 2.53 (m, 2H), 2.29 (dt, *J* = 16.0, 7.9 Hz, 2H), 2.07 (dd, *J* = 8.4, 3.7 Hz, 3H), 1.94 - 1.86 (m, 2H), 1.66 - 1.58 (m, 2H). LCMS (ESI) calcd for C₄₀H₄₁N₁₀O₄⁺ [M+H]⁺: 725.32, found, 725.30.

### Example 174: preparation of N-(4-((4-((2-(2,6-dioxopiperidin-3-yl)-1,3-dioxoisoindolin-5-yl)methyl)piperazin-1-yl)methyl)-3-(trifluoromethyl)phenyl)-3-(imidazo[1,2-b]pyridazin-3-ylethynyl)-4-methylbenzamide (GT-03938)

Referring to the method of Step 2 in Scheme 11 and the method of Example 1, the target compound (GT-03938) was prepared using 3-(imidazo[1,2-b]pyridazin-3-ylethynyl)-4-methyl-N-(4-(piperazin-1-ylmethyl)-3-(trifluoromethyl)phenyl)benzamide hydrochloride (GT-D-109) prepared according to Intermediate Example 68 and 5-(bromomethyl)-2-(2,6-dioxo-piperidin-3-yl)isoindoline-1,3-dione (CAS NO.: 1312023-72-5). The target compound (GT-03938) was obtained as a white solid (42 mg, yield 52%). ¹H NMR (400 MHz, DMSO) δ 11.13 (d, *J* = 12.2 Hz, 2H), 10.67 (s, 1H), 8.75 (dd, *J* = 4.5, 1.5 Hz, 1H), 8.28 (dd, *J* = 9.2, 1.5 Hz, 1H), 8.26 (s, 1H), 8.23 (d, *J* = 1.7 Hz, 1H), 8.21 - 8.13 (m, 2H), 8.11 - 8.00 (m, 2H), 7.97 (dd, *J* = 8.0, 1.8 Hz, 1H), 7.81 (d, *J =* 7.6 Hz, 1H), 7.76 (s, 1H), 7.70 (d, *J* = 7.6 Hz, 1H), 7.57 (d, *J* = 8.2 Hz, 1H), 7.42 (dd, *J* = 9.2, 4.5 Hz, 1H), 5.23 - 5.07 (m, 2H), 4.64 - 4.36 (m, 3H), 3.43 - 2.98 (m, 5H), 2.96 - 2.83 (m, 3H), 2.69 - 2.65 (m, 1H), 2.62 (s, 3H), 2.59 - 2.54 (m, 2H), 2.08 - 2.04 (m, 1H). LCMS (ESI) m/z: calcd for C₄₂H₃₆F₃N₈O₅⁺[M+H]⁺, 789.28; found, 789.3.

### Example 175: preparation of 5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-2-(2,6-dioxopiperidin-3-yl)isoindoline-1,3-dione (GT-03602)

Referring to the method of Step 2 in Scheme 11 and the method of Example 1, the target compound (GT-03602) was prepared using (2-((5-chloro-2-((2-methoxy-4-(4-(methylamino)piperidin-1-yl)phenyl)amino)pyrimidin-4-yl)amino)phenyl)dimethylphosphine oxide (CAS NO.: 2353496-90-7) and 5-(bromomethyl)-2-(2,6-dioxo-piperidin-3-yl)isoindoline-1,3-dione (CAS NO.: 1312023-72-5). The target compound (GT-03602) was obtained as a white solid (29 mg, yield 36%). ¹H NMR (400 MHz, MeOD) δ8.31 (s, 1H), 8.17 (s, 1H), 8.15 - 8.01 (m, 3H), 7.71 (dd, *J* = 14.5, 7.1 Hz, 1H), 7.61 (t, *J* = 7.4 Hz, 1H), 7.51 - 7.37 (m, 2H), 7.02 (s, 1H), 6.83 (d, *J* = 8.5 Hz, 1H), 5.20 (dd, *J* = 12.6, 5.5 Hz, 1H), 4.77 - 4.46 (m, 2H), 4.05 - 3.95 (m, 2H), 3.91 (s, 3H), 3.83 - 3.71 (m, 1H), 3.28 - 3.17 (m, 2H), 2.95 - 2.88 (m, 1H), 2.84 (s, 3H), 2.82 - 2.72 (m, 2H), 2.45 - 2.37 (m, 2H), 2.35 - 2.22 (m, 2H), 2.21- 2.14 (m, 1H), 1.90 (s, 3H), 1.87 (s, 3H). LCMS (ESI) calcd for C₃₉H₄₃ClN₈O₆P⁺ [M+H]⁺: 785.27, found, 785.3.

### Example 176: preparation of 5-((4-(4-((5-chloro-4-((2-(dimethylphosphoryl)phenyl)amino)pyrimidin-2-yl)amino)-3-methoxyphenyl)piperazin-1-yl)methyl)-2-(2,6-dioxopiperidin-3-yl)isoindoline-1,3-dione (GT-03780)

Referring to the method of Step 2 in Scheme 11 and the method of Example 1, the target compound (GT-03780) was prepared using (2-((5-chloro-2-((2-methoxy-4-(piperazin-1-yl)phenyl)amino)pyrimidin-4-yl)amino)phenyl)dimethylphosphine oxide (CAS NO.: 1197958-90-9) and 5-(bromomethyl)-2-(2,6-dioxo-piperidin-3-yl)isoindoline-1,3-dione (CAS NO.: 1312023-72-5). The target compound (GT-03780) was obtained as a white solid (16 mg, yield 25.40%). ¹H NMR (400 MHz, DMSO) δ 11.83 (s, 2H), 11.15 (s, 1H), 9.15 (s, 1H), 8.39 (s, 1H), 8.31 (s, 1H), 8.27 - 8.15 (m, 2H), 8.06 (d, *J* = 7.6 Hz, 1H), 7.61 (dd, *J* = 13.7, 7.9 Hz, 1H), 7.39 (d, *J* = 8.4 Hz, 2H), 7.21 (t, *J* = 7.2 Hz, 1H), 6.73 (d, *J* = 1.6 Hz, 1H), 6.55 (dd, *J* = 8.7, 1.7 Hz, 1H), 5.20 (dd, *J* = 12.9, 5.3 Hz, 1H), 4.62 (s, 2H), 3.87 (d, *J* = 10.9 Hz, 2H), 3.78 (s, 3H), 3.44 (d, *J* = 5.8 Hz, 2H), 3.24 (d, *J* = 9.5 Hz, 4H), 2.95 - 2.87 (m, 1H), 2.65 - 2.56 (m, 2H), 2.11 - 2.03 (m, 1H), 1.80 (s, 3H), 1.77 (s, 3H). LCMS (ESI) calcd for C₃₇H₃₉ClN₈O₆P⁺ [M+H]⁺: 757.23, found, 757.20.

### Example 177: preparation of 8-(4-(4-((2-(2,6-dioxopiperidin-3-yl)-1,3-dioxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile (GT-03761)

Referring to the method of Step 2 in Scheme 11 and the method of Example 1, the target compound (GT-03761) was prepared using 9-ethyl-6,6-dimethyl-11-oxo-8-(4-(piperazin-1-yl)piperidin-1-yl)-6,11-dihydro-5H-benzo[b]carbazole-3-carbonitrile (GT-D-113) prepared from Intermediate Example 45 and 5-(bromomethyl)-2-(2,6-dioxo-piperidin-3-yl)isoindoline-1,3-dione (CAS NO.: 1312023-72-5). The target compound (GT-03761) was obtained as a white solid (60 mg, yield 72%). ¹H NMR (400 MHz, DMSO) δ 12.80 (s, 1H), 11.13 (s, 1H), 8.31 (d, *J* = 8.2 Hz, 1H), 8.06 (s, 1H), 8.00 (s, 1H), 7.82 (s, 1H), 7.76 (s, 2H), 7.36 (s, 1H), 5.17 (dd, *J* = 12.8, 5.2 Hz, 1H), 4.09 (s, 2H), 3.45 - 3.19 (m, 10H), 2.90 - 2.80 (m, 4H), 2.74 - 2.67 (m, 3H), 2.26 - 2.17 (m, 2H), 2.07 - 2.03 (m, 2H), 1.93 - 1.86 (m, 2H), 1.76 (s, 6H), 1.28 (t, *J* = 7.5 Hz, 3H). LCMS (ESI) m/z: calcd for C₄₄H₄₆N₇O₅⁺[M+H]⁺, 752.36; found, 752.4.

### Example 178: preparation of 2-((2-((4-(4-(((2-(2,6-dioxopiperidin-3-yl)-1,3-dioxoisoindolin-5-yl)methyl)(methyl)amino)piperidin-1-yl)-2-methoxyphenyl)amino)-5-(trifluoromethyl)pyridin-4-yl)amino)-N-methylbenzamide (GT-03732)

Referring to the method of Step 2 in Scheme 11 and the method of Example 1, the target compound (GT-03732) was prepared using 2-((2-((2-methoxy-4-(4-(methylamino)piperidin-1-yl)phenyl)amino)-5-(trifluoromethyl)pyridin-4-yl)amino)-N-methylbenzamide (GT-M-172) prepared from Intermediate Example 29 and 5-(bromomethyl)-2-(2,6-dioxo-piperidin-3-yl)isoindoline-1,3-dione (CAS NO.: 1312023-72-5). The target compound (GT-03732) was obtained as a white solid (28 mg, yield 39.61%). ¹H NMR (400 MHz, DMSO) δ 11.65 (s, 1H), 11.15 (s, 1H), 10.61 (s, 1H), 10.03 (s, 1H), 8.83 (d, *J* = 3.7 Hz, 1H), 8.37 (s, 1H), 8.26 (d, *J* = 7.8 Hz, 1H), 8.18 (s, 1H), 8.03 (d, *J* = 7.7 Hz, 1H), 7.78 (d, *J* = 7.8 Hz, 1H), 7.57 (d, *J* = 3.9 Hz, 2H), 7.32 - 7.20 (m, 2H), 6.91 - 6.53 (m, 3H), 5.19 (dd, *J* = 12.8, 5.3 Hz, 1H), 4.70 (d, *J* = 12.2 Hz, 1H), 4.47 (dd, *J* = 12.6, 7.1 Hz, 1H), 3.92 (d, *J* = 8.9 Hz, 2H), 3.82 (s, 3H), 3.52 (dd, *J* = 15.7, 9.6 Hz, 2H), 3.00 - 2.85 (m, 3H), 2.76 (d, *J* = 4.5 Hz, 3H), 2.63 - 2.59 (m, 3H), 2.54 (d, *J* = 8.2 Hz, 1H), 2.39 - 2.26 (m, 2H), 2.14 - 1.98 (m, 3H). LCMS (ESI) calcd for C₄₁H₄₂F₃N₈O₆⁺ [M+H]⁺: 798.31, found, 798.30.

### Example 179: preparation of 2-((2-((4-(4-((2-(2,6-dioxopiperidin-3-yl)-1,3-dioxoisoindolin-5-yl)methyl)piperazin-1-yl)-2-methoxyphenyl)amino)-5-(trifluoromethyl)pyridin-4-yl)amino)-N-methylbenzamide (GT-03713)

Referring to the method of Step 2 in Scheme 11 and the method of Example 1, the target compound (GT-03713) was prepared using 2-((2-((2-methoxy-4-(piperazin-1-yl)phenyl)amino)-5-(trifluoromethyl)pyridin-4-yl)amino)-N-methylbenzamide (GT-M-171) prepared from Intermediate Example 28 and 5-(bromomethyl)-2-(2,6-dioxo-piperidin-3-yl)isoindoline-1,3-dione (CAS NO.: 1312023-72-5). The target compound (GT-03713) was obtained as a white solid (20 mg, yield 31.25%). ¹H NMR (400 MHz, DMSO) δ 12.01 (s, 1H), 11.15 (s, 1H), 10.52 (s, 1H), 9.37 (s, 1H), 8.83 - 8.76 (m, 1H), 8.31 (s, 1H), 8.22 - 8.10 (m, 2H), 8.05 (d, *J* = 7.7 Hz, 1H), 7.76 (d, *J* = 7.8 Hz, 1H), 7.54 (d, *J* = 3.8 Hz, 2H), 7.25 (dd, *J* = 10.4, 6.1 Hz, 2H), 6.71 (d, *J* = 1.6 Hz, 1H), 6.60 - 6.49 (m, 2H), 5.19 (dd, *J* = 12.9, 5.3 Hz, 1H), 4.61 (s, 2H), 3.88 (s, 2H), 3.80 (s, 3H), 3.41 (dd, *J* = 8.9, 6.6 Hz, 3H), 3.25 (dd, *J* = 23.3, 10.3 Hz, 4H), 2.90 (td, *J* = 14.0, 7.4 Hz, 1H), 2.75 (d, *J* = 4.5 Hz, 3H), 2.62 (d, *J =* 17.8 Hz, 1H), 2.12 - 2.04 (m, 1H). LCMS (ESI) calcd for C₃₉H₃₈F₃N₈O₆⁺ [M+H]⁺: 771.28, found, 771.30.

### Example 180: preparation of N-(3-(difluoromethyl)-1-(1-((2-(2,6-dioxopiperidin-3-yl)-1,3-dioxoisoindolin-5-yl)methyl)piperidin-4-yl)-1H-pyrazol-4-yl)-5-morpholinopyrazolo[1,5-a]pyrimidine-3-carboxamide (GT-03815)

Referring to the method of Step 2 in Scheme 11 and the method of Example 1, the target compound (GT-03815) was prepared using N-(3-(difluoromethyl)-1-(piperidin-4-yl)-1H-pyrazol-4-yl)-5-morpholinopyrazolo[1,5-a]pyrimidine-3-carboxamide hydrochloride (GT-D-101) prepared from Intermediate Example 64 and 5-(bromomethyl)-2-(2,6-dioxo-piperidin-3-yl)isoindoline-1,3-dione (CAS NO.: 1312023-72-5). The target compound (GT-03815) was obtained as a white solid (50 mg, yield 58%). ¹H NMR (400 MHz, MeOD) δ 8.55 (d, *J* = 7.9 Hz, 1H), 8.43 (s, 1H), 8.32 (s, 1H), 8.11 (s, 1H), 8.07 - 8.00 (m, 2H), 7.02 - 6.75 (m, 1H), 6.82 (d, *J* = 8.0 Hz, 1H), 5.19 (dd, *J* = 12.6, 5.4 Hz, 1H), 4.83 - 4.76 (m, 2H), 4.57 (s, 2H), 3.93 - 3.77 (m, 9H), 3.74 - 3.58 (m, 2H), 3.43 - 3.33 (m, 2H), 2.80 - 2.75 (m, 3H), 2.47 - 2.29 (m, 4H), 2.21 - 2.13 (m, 1H). LCMS (ESI) m/z: calcd for C₃₄H₃₅F₂N₁₀O₆⁺[M+H]⁺, 717.27; found, 717.3.

### Example 181: preparation of N-(5-(3,5-difluorobenzyl)-1H-indazol-3-yl)-4-(4-(((2-(2,6-dioxopiperidin-3-yl)-1,3-dioxoisoindolin-5-yl)methyl)(methyl)amino)piperidin-1-yl)-2-((tetrahydro-2H-pyran-4-yl)amino)benzamide (GT-03966)

Referring to the method of Step 2 in Scheme 11 and the method of Example 1, the target compound (GT-03966) was prepared using N-(5-(3,5-difluorobenzyl)-1H-indazol-3-yl)-4-(4-(methylamino)piperidin-1-yl)-2-((tetrahydro-2H-pyran-4-yl)amino)benzamide (GT-S-15) prepared from Intermediate Example 90 and 5-(bromomethyl)-2-(2,6-dioxo-piperidin-3-yl)isoindoline-1,3-dione (CAS NO.: 1312023-72-5). The target compound (GT-03966) was obtained as a white solid (23 mg, yield 29%). ¹H NMR (400 MHz, DMSO) δ 12.65 (s, 1H), 11.15 (s, 1H), 10.39 (s, 1H), 10.12 (s, 1H), 8.26 (s, 1H), 8.13 (d, *J* = 7.6 Hz, 1H), 8.06 (d, *J* = 7.8 Hz, 1H), 7.87 - 7.82 (m, 1H), 7.47 (s, 1H), 7.41 (d, *J* = 8.6 Hz, 1H), 7.26 (d,*J* = 8.6 Hz, 1H), 7.06 - 6.95 (m, 3H), 6.31 (d, *J* = 9.1 Hz, 1H), 6.20 (s, 1H), 5.19 (dd, *J* = 12.8, 5.4 Hz, 1H), 4.71 (d, *J* = 10.9 Hz, 1H), 4.58 - 4.39 (m, 1H), 4.15 - 3.98 (m, 4H), 3.87 - 3.79 (m, 2H), 3.73 - 3.68 (m, 1H), 2.97 - 2.78 (m, 5H), 2.20 (s, 2H), 2.32 - 2.04 (m, 5H), 2.04 - 1.90 (m, 3H), 1.86 - 1.76 (m, 1H), 1.44 - 1.30 (m, 2H). LCMS (ESI) m/z: calcd for C₄₆H₄₇F₂N₈O₆⁺[M+H]⁺, 845.36; found, 845.4.

### Example 182: preparation of 3-(5-((4-(1-(4,9-dioxo-4,9-dihydronaphtho[2,3-b]furan-2-yl)ethyl)piperazin-1-yl)methyl)-1-oxoisoindolin-2-yl)piperidine-2,6-dione (GT-03633)

Referring to the method of Step 2 in Scheme 11 and the method of Example 1, the target compound (GT-03633) was prepared using 2-(1-(piperazin-1-yl)ethyl)naphtho[2,3-b]furan-4,9-dione (CAS NO.: 2226349-69-3) and 3-(5-(bromomethyl)-1-oxoisoindolin-2-yl)piperidine-2,6-dione (GT-03234). The target compound (GT-03633) was obtained as a white solid (53 mg, yield 72.57%). ¹H NMR (400 MHz, DMSO) δ 10.99 (s, 1H), 8.11 (ddd, *J* = 6.1, 4.3, 2.6 Hz, 2H), 7.92 - 7.85 (m, 3H), 7.76 (q, *J* = 7.8 Hz, 2H), 7.31 (s, 1H), 5.12 (dd, *J* = 13.2, 5.1 Hz, 1H), 4.70 (s, 1H), 4.50 - 4.43 (m, 3H), 4.33 (d, *J* = 17.4 Hz, 2H), 3.49 - 3.23 (m, 7H), 2.95 - 2.87 (m, 1H), 2.62 - 2.57 (m, 1H), 2.41 (dd, *J* = 13.1, 4.4 Hz, 1H), 2.02 - 1.96 (m, 1H), 1.63 (d, *J* = 6.6 Hz, 3H). LCMS (ESI) calcd for C₃₂H₃₁N₄O₆⁺ [M+H]⁺: 567.61, found, 567.20.

### Example 183: preparation of N-((1r,4r)-4-(3-chloro-4-cyanophenoxy)cyclohexyl)-6-(4-((3-((2-(2,6-dioxopiperidin-3-yl)-1-oxoisoindolin-5-yl)methyl)azetidin-1-yl)methyl)piperidin-1-yl)pyridazine-3-carboxamide (GT-03521)

The target compound (GT-03521) was prepared using the method described in Scheme 59 (yellow solid, 25 mg, yield 30.57%). ¹H NMR (400 MHz, DMSO) δ 10.98 (s, 1H), 8.60 (d, *J* = 8.2 Hz, 1H), 7.90 - 7.79 (m, 2H), 7.67 (dd, *J* = 7.8, 3.6 Hz, 1H), 7.46 (d, *J* = 13.1 Hz, 2H), 7.41 - 7.31 (m, 2H), 7.13 (dd, *J* = 8.8, 2.4 Hz, 1H), 5.10 (dd, *J* = 13.2, 5.1 Hz, 1H), 4.61 - 4.39 (m, 4H), 4.30 (d, *J* = 17.4 Hz, 1H), 4.16 - 4.10 (m, 1H), 4.08 - 3.95 (m, 3H), 3.28 (d, *J* = 8.2 Hz, 1H), 3.22 - 3.11 (m, 1H), 3.06 (dt, *J* = 23.8, 8.0 Hz, 5H), 2.95 - 2.86 (m, 1H), 2.66 - 2.55 (m, 1H), 2.45 - 2.32 (m, 1H), 2.10 (d, *J* = 10.1 Hz, 2H), 2.06 - 1.94 (m, 2H), 1.87 (t, *J* = 13.4 Hz, 4H), 1.69 - 1.46 (m, 5H), 1.33 - 1.19 (m, 2H). LCMS (ESI) calcd for C₄₁H₄₆ClN₈O₅⁺ [M+H]⁺: 765.32, found, 765.40.

### Example 184: preparation of 2-((2-((4-(4-(4-((2-(2,6-dioxopiperidin-3-yl)-1,3-dioxoisoindolin-5-yl)methyl)piperazin-1-yl)piperidin-1-yl)-2-methoxyphenyl)amino)-5-(trifluoromethyl)pyridin-4-yl)amino)-N-methylbenzamide (GT-03598)

Referring to the method of Step 2 in Scheme 11 and the method of Example 1, the target compound (GT-03598) was prepared using 2-((2-((2-methoxy-4-(4-(piperazin-1-yl)piperidin-1-yl)phenyl)amino)-5-(trifluoromethyl)pyridin-4-yl)amino)-N-methylbenzamide (GT-M-12) prepared from Intermediate Example 27 and 3-(5-(bromomethyl)-1-oxoisoindolin-2-yl)piperidine-2,6-dione (GT-03234). The target compound (GT-03598) was obtained as a white solid (25 mg, yield 39%). ¹H NMR (400 MHz, MeOD) δ8.16 (s, 1H), 8.08 (d, *J* = 7.8 Hz, 1H), 8.04 (s, 1H), 7.99 (d, *J* = 7.7 Hz, 1H), 7.73 (d, *J* = 7.6 Hz, 1H), 7.64 - 7.56 (m, 2H), 7.40 - 7.34 (m, 1H), 7.30 - 7.23 (m, 1H), 7.08 (s, 1H), 6.94 (d, *J* = 7.4 Hz, 1H), 6.49 (s, 1H), 5.18 (dd, *J* = 12.6, 5.4 Hz, 1H), 4.51 (s, 2H), 3.98 - 3.92 (m, 2H), 3.89 (s, 3H), 3.77 - 3.47 (m, 9H), 3.29 - 3.20 (m, 2H), 2.89 (s, 3H), 2.87 - 2.82 (m, 1H), 2.80 - 2.68 (m, 2H), 2.45 - 2.32 (m, 2H), 2.23 - 2.08 (m, 3H). LCMS (ESI) calcd for C₄₄H₄₇F₃N₉O₆⁺ [M+H]⁺: 854.36, found, 854.4.

### Example 185: preparation of 3-(5-((4-(4-(1,2-bis(4-hydroxyphenyl)but-1-en-1-yl)phenyl)piperazin-1-yl)methyl)-4-fluoro-1-oxoisoindolin-2-yl)piperidine-2,6-dione (GT-04144)

The target compound (GT-04144) was prepared by referring to the methods of Scheme 11 and Example 1 (white solid, 45 mg, yield 52.35%). ¹H NMR (400 MHz, DMSO-*d₆*) δ 11.45 (d, *J* = 29.8 Hz, 1H), 11.05 (d, *J* = 2.7 Hz, 1H), 8.11 - 7.95 (m, 1H), 7.77 - 7.67 (m, 1H), 7.04 (t, *J* = 9.2 Hz, 1H), 6.99 - 6.92 (m, 2H), 6.89 (dd, *J* = 8.5, 4.0 Hz, 2H), 6.76 (d, *J* = 8.5 Hz, 1H), 6.69 (t, *J* = 9.1 Hz, 2H), 6.61 - 6.53 (m, 3H), 6.44 (d, *J* = 8.6 Hz, 1H), 5.17 (ddd, *J* = 14.2, 9.6, 4.8 Hz, 1H), 4.64 - 4.46 (m, 4H), 3.51 - 3.06 (m, 8H), 2.98 - 2.90 (m, 1H), 2.63 (d, *J* = 16.0 Hz, 1H), 2.48 - 2.41 (m, 1H), 2.36 (d, *J* = 7.2 Hz, 2H), 2.03 (dd, *J* = 6.2, 3.3 Hz, 1H), 0.86 (td, *J* = 7.3, 4.6 Hz, 3H). LCMS (ESI) calcd for C₄₀H₄₀FN₄O₅⁺ [M+H]⁺: 675.29, found, 675.30.

### Example 186: preparation of 3-(5-((4-(4-(1,2-bis(4-hydroxyphenyl)but-1-en-1-yl)phenyl)piperazin-1-yl)methyl)-6-fluoro-1-oxoisoindolin-2-yl)piperidine-2,6-dione (GT-04145)

The target compound (GT-04145) was prepared using the methods described in Scheme 11 and Example 1 (white solid, 46 mg, yield 53.51%). ¹H NMR (400 MHz, DMSO) δ 11.32 (d, *J =* 34.1 Hz, 1H), 10.94 (d, *J* = 2.7 Hz, 1H), 8.00 (dd, *J* = 15.9, 6.1 Hz, 1H), 7.60 (t, *J =* 8.8 Hz, 1H), 6.94 (t, *J* = 8.7 Hz, 1H), 6.89 - 6.82 (m, 2H), 6.78 (dd, *J* = 8.5, 4.3 Hz, 2H), 6.65 (d, *J* = 8.5 Hz, 1H), 6.57 (dd, *J* = 19.2, 8.9 Hz, 2H), 6.52 - 6.43 (m, 3H), 6.33 (d, *J* = 8.6 Hz, 1H), 5.06 (dt, *J* = 13.1, 4.7 Hz, 1H), 4.52 - 4.34 (m, 3H), 4.29 (dd, *J* = 17.4, 7.8 Hz, 1H), 3.42 - 2.91 (m, 8H), 2.83 (dd, *J* = 19.9, 8.6 Hz, 1H), 2.54 (t, *J* = 16.5 Hz, 1H), 2.38 - 2.29 (m, 1H), 2.25 (d, *J* = 7.1 Hz, 2H), 1.94 (d, *J* = 4.4 Hz, 1H), 0.75 (td, *J* = 7.3, 4.7 Hz, 3H). LCMS (ESI) calcd for C₄₀H₄₀FN₄O₅⁺ [M+H]⁺: 675.29, found, 675.30.

### Example 187: preparation of 3-(5-((4-(4-(1,2-bis(4-hydroxyphenyl)but-1-en-1-yl)phenyl)piperazin-1-yl)methyl)-7-fluoro-1-oxoisoindolin-2-yl)piperidine-2,6-dione (GT-04146)

The target compound (GT-04146) was prepared by referring to the methods of Scheme 11 and Example 1 (white solid, 56 mg, yield 65.15%). ¹H NMR (400 MHz, DMSO) δ 11.43 (d, *J =* 38.3 Hz, 1H), 11.00 -10.86 (m, 1H), 9.26 (d, *J* = 98.2 Hz, 1H), 7.67 - 7.56 (m, 2H), 6.95 (t, *J* = 9.5 Hz, 1H), 6.87 (dd, *J* = 8.4, 6.2 Hz, 2H), 6.80 (dd, *J* = 8.5, 4.9 Hz, 2H), 6.67 (d, *J* = 8.5 Hz, 1H), 6.59 (dd, *J* = 19.6, 8.9 Hz, 2H), 6.54 - 6.44 (m, 3H), 6.35 (d, *J* = 8.6 Hz, 1H), 5.09 - 4.99 (m, 1H), 4.50 - 4.34 (m, 4H), 3.39 - 2.95 (m, 8H), 2.90 - 2.80 (m, 1H), 2.53 (d, *J* = 16.5 Hz, 1H), 2.34 - 2.20 (m, 3H), 1.98 - 1.91 (m, 1H), 0.77 (td, *J* = 7.3, 4.9 Hz, 3H). LCMS (ESI) calcd for C₄₀H₄₀FN₄O₅⁺ [M+H]⁺: 675.29, found, 675.30.

### Example 188: preparation of 3-(4-((4-(4-(1,2-bis(4-hydroxyphenyl)but-1-en-1-yl)phenyl)piperazin-1-yl)methyl)-1-oxoisoindolin-2-yl)piperidine-2,6-dione (GT-04147)

The target compound (GT-04147) was prepared using the methods described in Scheme 11 and Example 1 (white solid, 27 mg, yield 32.27%). ¹H NMR (400 MHz, DMSO) δ 11.23 (d, *J* = 37.7 Hz, 1H), 10.96 (dd, *J* = 24.7, 7.3 Hz, 1H), 9.11 (s, 2H), 7.95 (dd, *J* = 17.0, 7.6 Hz, 1H), 7.77 (t, *J* = 7.4 Hz, 1H), 7.57 (dd, *J* = 16.8, 7.7 Hz, 1H), 6.95 (t, *J* = 9.9 Hz, 1H), 6.91 - 6.84 (m, 2H), 6.80 (dd, *J* = 8.4, 5.1 Hz, 2H), 6.67 (d, *J* = 8.5 Hz, 1H), 6.64 - 6.56 (m, 2H), 6.54 - 6.46 (m, 3H), 6.35 (d, *J* = 8.6 Hz, 1H), 5.15 - 5.08 (m, 1H), 4.83 (dd, *J=* 20.2, 13.4 Hz, 1H), 4.53 - 4.45 (m, 1H), 4.41 - 4.30 (m, 2H), 3.80 - 3.63 (m, 2H), 3.27 - 2.95 (m, 6H), 2.86 (dd, *J* = 12.9, 4.5 Hz, 1H), 2.58 (d, *J =* 15.2 Hz, 1H), 2.27 (dd, *J* = 7.1, 3.5 Hz, 3H), 2.01 - 1.93 (m, 1H), 0.77 (dd, *J =* 12.5, 7.3 Hz, 3H). LCMS (ESI) calcd for C₄₀H₄₁N₄O₅⁺ [M+H]⁺: 657.30, found, 657.30.

### Example 189: preparation of 5-((4-(4-(1,2-bis(4-hydroxyphenyl)but-1-en-1-yl)phenyl)piperazin-1-yl)methyl)-2-(2,6-dioxopiperidin-3-yl)isoindoline-1,3-dione (GT-04148)

The target compound (GT-04148) was prepared using the methods described in Scheme 11 and Example 1 (white solid, 67 mg, yield 78.41%). ¹H NMR (400 MHz, DMSO) δ 11.27 (d, *J* = 33.2 Hz, 1H), 11.04 (s, 1H), 9.15 (s, 2H), 8.19 (d, *J* = 14.0 Hz, 1H), 8.07 (dd, *J* = 13.9, 7.9 Hz, 1H), 8.01 - 7.92 (m, 1H), 6.95 (t, *J* = 9.2 Hz, 1H), 6.88 (dd, *J* = 15.1, 7.6 Hz, 2H), 6.80 (dd, *J* = 8.5, 5.1 Hz, 2H), 6.66 (d, *J* = 8.5 Hz, 1H), 6.59 (q, *J* = 8.9 Hz, 2H), 6.53 - 6.45 (m, 3H), 6.35 (d, *J* = 8.6 Hz, 1H), 5.16 - 5.09 (m, 1H), 4.52 (d, *J* = 18.6 Hz, 2H), 3.68 (dd, *J* = 51.4, 10.4 Hz, 2H), 3.26 - 2.92 (m, 6H), 2.83 (dd, *J* = 8.6, 5.5 Hz, 1H), 2.59 - 2.51 (m, 2H), 2.34 - 2.20 (m, 2H), 2.05 - 2.00 (m, 1H), 0.77 (dt, *J* = 12.1, 6.1 Hz, 3H). LCMS (ESI) calcd for C₄₀H₃₉N₄O₆⁺ [M+H]⁺: 671.28, found, 671.30.

### Example 190: preparation of 3-(5-((2,3-difluoro-6-(2-morpholinothiazol-4-yl)phenoxy)methyl)-1-oxoisoindolin-2-yl)piperidine-2,6-dione (GT-03341)

Referring to the method of Scheme 12, the target compound (GT-03341) was prepared using 2,3-difluoro-6-(2-morpholinothiazol-4-yl)phenol (CAS NO.: 1621375-40-3) and 3-(5-(bromomethyl)-1-oxoisoindolin-2-yl)piperidine-2,6-dione (GT-03234). The target compound (GT-03341) was obtained as a white solid (54.00 mg, yield 36.31%). ¹H NMR (400 MHz, DMSO) δ 11.00 (s, 1H), 7.75 (ddd, *J* = 11.5, 5.7, 2.4 Hz, 2H), 7.66 (s, 1H), 7.55 (d, *J* = 7.9 Hz, 1H), 7.30 - 7.22 (m, 2H), 5.26 (s, 2H), 5.16 - 5.11 (m, 1H), 4.47 (d, *J* = 17.5 Hz, 1H), 4.34 (d, *J* = 17.4 Hz, 1H), 3.74 - 3.66 (m, 4H), 3.44 - 3.36 (m, 4H), 2.98 - 2.86 (m, 1H), 2.60 (d, *J* = 17.8 Hz, 1H), 2.40 (qd, *J* = 13.3, 4.3 Hz, 1H), 2.05 - 1.98 (m, 1H). LCMS (ESI) calcd for C₂₇H₂₅F₂N₄O₅S⁺ [M+H]⁺: 555.14, found, 555.20.

### Example 191: preparation of 3-(5-((4-(1-(4-((9-cyclopentyl-8-(phenylamino)-9H-purin-2-yl)amino)phenyl)piperidin-4-yl)piperazin-1-yl)methyl)-1-oxoisoindolin-2-yl)piperidine-2,6-dione (GT-03808)

Referring to the method of Step 2 in Scheme 11 and the method of Example 1, the target compound (GT-03808) was prepared using 9-cyclopentyl-N⁸-phenyl-N²-(4-(4-(piperazin-1-yl)piperidin-1-yl)phenyl)-9H-purine-2,8-diamine (GT-D-68) prepared from Intermediate Example 49 and 3-(5-(bromomethyl)-1-oxoisoindolin-2-yl)piperidine-2,6-dione. The target compound (GT-03808) was obtained as a white solid (14 mg, yield 18%). ¹H NMR (400 MHz, DMSO) δ 11.01 (s, 1H), 10.22 (s, 1H), 8.44 (s, 1H), 7.93 (s, 1H), 7.84 - 7.73 (m, 4H), 7.68 - 7.58 (m, 2H), 7.45 - 7.35 (m, 4H), 7.15 (t, *J* = 7.4 Hz, 1H), 5.24 - 5.09 (m, 2H), 4.59 - 4.33 (m, 5H), 3.23 - 2.82 (m, 8H), 2.68 - 2.57 (m, 2H), 2.40 - 2.23 (m, 5H), 2.21 - 1.87 (m, 8H), 1.71 - 1.57 (m, 2H). LCMS (ESI) m/z: calcd for C₄₅H₅₂N₁₁O₃⁺[M+H]⁺, 793.42; found, 793.4.

### Example 192: preparation of 3-(5-(((4-((3,4-dichloro-2-fluorophenyl)amino)-7-methoxyquinazolin-6-yl)oxy)methyl)-1-oxoisoindolin-2-yl)piperidine-2,6-dione (GT-03354)

Referring to the method of Scheme 12, the target compound (GT-03354) was prepared using 4-((3,4-Dichloro-2-fluorophenyl)amino)-7-methoxyquinazolin-6-ol (CAS NO.: 1429757-65-2) and 3-(5-(bromomethyl)-1-oxoisoindolin-2-yl)piperidine-2,6-dione. The target compound (GT-03354) was obtained as a white solid (5 mg, yield 5%). ¹H NMR (400 MHz, MeOD) δ 8.67 (s, 1H), 8.05 (s, 1H), 7.87 (d, *J* = 7.9 Hz, 1H), 7.78 (s, 1H), 7.70 (d, *J* = 7.8 Hz, 1H), 7.61 - 7.56 (m, 1H), 7.53 (d, *J* = 9.9 Hz, 1H), 7.30 (s, 1H), 5.46 (s, 2H), 5.18 (dd, *J* = 13.3, 5.2 Hz, 1H), 4.62 - 4.51 (m, 2H), 4.12 (s, 3H), 2.97 - 2.87 (m, 1H), 2.84 - 2.76 (m, 1H), 2.51 (qd, *J* = 13.5, 4.9 Hz, 1H), 2.24 - 2.15 (m, 1H). LCMS (ESI) calcd for C₂₉H₂₃Cl₂FN₅O₅⁺ [M+H]⁺: 610.11, found, 610.1.

### Example 193: preparation of 3-(5-(((4-((3-chloro-4-fluorophenyl)amino)-7-(((S)-tetrahydrofuran-3-yl)oxy)quinazolin-6-yl)amino)methyl)-1-oxoisoindolin-2-yl)piperidine-2,6-dione (GT-03353)

Referring to the method of Step 2 in Scheme 11 and the method of Example 1, the target compound (GT-03353) was prepared using (S)-N⁴-(3-chloro-4-fluorophenyl)-7-((tetrahydrofuran-3-yl)oxy)quinazolin-4,6-diamine (CAS NO.: 314771-76-1) and 3-(5-(bromomethyl)-1-oxoisoindolin-2-yl)piperidine-2,6-dione. The target compound (GT-03353) was obtained as a white solid (5 mg, yield 5%). ¹H NMR (400 MHz, MeOD) δ 8.55 (s, 1H), 7.83 (dd, *J* = 6.6, 2.6 Hz, 1H), 7.76 (d, *J* = 7.8 Hz, 1H), 7.64 - 7.52 (m, 3H), 7.37 - 7.27 (m, 2H), 7.13 (s, 1H), 5.39 - 5.34 (m, 1H), 5.13 (dd, *J* = 13.3, 5.1 Hz, 1H), 4.79 (s, 2H), 4.54 - 4.38 (m, 2H), 4.19 (d, *J* = 10.6 Hz, 1H), 4.14 - 4.04 (m, 2H), 4.00 - 3.91 (m, 1H), 2.95 - 2.83 (m, 1H), 2.82 - 2.71 (m, 1H), 2.53 - 2.38 (m, 2H), 2.36 - 2.27 (m, 1H), 2.21 - 2.10 (m, 1H). LCMS (ESI) calcd for C₃₂H₂₉ClFN₆O₅⁺ [M+H]⁺: 631.39, found, 631.2.

### Example 194: preparation of 3-(5-(((1-(4-((9-cyclopentyl-8-(phenylamino)-9H-purin-2-yl)amino)phenyl)piperidin-4-yl)(methyl)amino)methyl)-1-oxoisoindolin-2-yl)piperidine-2,6-dione (GT-03685)

Referring to the method of Step 2 in Scheme 11 and the method of Example 1, the target compound (GT-03685) was prepared using (9-cyclopentyl-N²-(4-(4-(methylamino)piperidin-1-yl)phenyl)-N⁸-phenyl-9H-purine-2,8-diamine (GT-D-67) prepared from Intermediate Example 48 and 3-(5-(bromomethyl)-1-oxoisoindolin-2-yl)piperidine-2,6-dione. The target compound (GT-03685) was obtained as a white solid (16.00 mg, yield 22.48%). ¹H NMR (400 MHz, DMSO) δ 11.61 (s, 1H), 11.03 (s, 1H), 10.44 (s, 1H), 8.48 (s, 1H), 8.06 (t, *J* = 6.9 Hz, 1H), 7.82 (d, *J* = 7.9 Hz, 2H), 7.70 (d, *J* = 7.7 Hz, 3H), 7.66 - 7.56 (m, 2H), 7.41 (t, *J* = 7.9 Hz, 2H), 7.16 (t, *J* = 7.4 Hz, 1H), 5.31 (dd, *J* = 16.3, 7.8 Hz, 1H), 5.16 (dd, *J* = 13.3, 5.0 Hz, 1H), 4.62 (d, *J* = 17.8 Hz, 2H), 4.50 - 4.44 (m, 2H), 3.80 - 3.65 (m, 4H), 3.43 (s, 2H), 2.97 - 2.89 (m, 1H), 2.74 (s, 3H), 2.61 (d, *J* = 16.9 Hz, 1H), 2.36 (ddd, *J* = 19.1, 16.2, 6.1 Hz, 5H), 2.04 (ddd, *J* = 35.3, 18.6, 4.7 Hz, 7H), 1.67 (dd, *J* = 12.1, 6.2 Hz, 2H). LCMS (ESI) calcd for C₄₂H₄₇N₁₀O₃⁺ [M+H]⁺: 739.38, found, 739.40.

### Example 195: preparation of 3-(5-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)-1-oxoisoindolin-2-yl)piperidine-2,6-dione (GT-03924)

Referring to the method of Step 2 in Scheme 11 and the method of Example 1, the target compound (GT-03924) was prepared using (6-((5-bromo-2-((2-methoxy-5-methyl-4-(4-(methylamino)piperidin-1-yl)phenyl)amino)pyrimidin-4-yl)amino)quinoxalin-5-yl)dimethylphosphine oxide (GT-S-51) prepared from Intermediate Example 80 and 3-(5-(bromomethyl)-1-oxoisoindolin-2-yl)piperidine-2,6-dione. The target compound (GT-03924) was obtained as a white solid (22 mg, yield 36.90%). ¹H NMR (400 MHz, DMSO) δ 13.21 (s, 1H), 11.31 (s, 1H), 11.03 (s, 1H), 9.29 (s, 1H), 8.93 (s, 2H), 8.76 (s, 1H), 8.43 (s, 1H), 8.00 (t, *J* = 9.7 Hz, 2H), 7.86 (dd, *J* = 17.8, 7.9 Hz, 2H), 7.37 (s, 1H), 6.87 (s, 1H), 5.15 (dd, *J* = 13.3, 4.9 Hz, 1H), 4.69 - 4.61 (m, 1H), 4.56 - 4.49 (m, 1H), 4.49 - 4.32 (m, 3H), 3.80 (s, 3H), 3.40 - 3.33 (m, 1H), 3.26 (ddd, *J* = 15.8, 8.2, 4.4 Hz, 2H), 2.98 - 2.78 (m, 3H), 2.68 (s, 3H), 2.64 - 2.58 (m, 1H), 2.45 (d, *J* = 13.6 Hz, 2H), 2.42 - 2.23 (m, 3H), 2.15 (s, 3H), 2.06 (s, 3H), 2.03 (s, 3H). LCMS (ESI) calcd for C₄₂H₄₇BrN₁₀O₅P⁺ [M+H]⁺: 881.26, found, 881.30.

### Example 196: preparation of 3-(5-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)-4-fluoro-1-oxoisoindolin-2-yl)piperidine-2,6-dione (GT-03925)

Referring to the method of Scheme 11, the target compound (GT-03925) was prepared using (6-((5-bromo-2-((2-methoxy-5-methyl-4-(4-(methylamino)piperidin-1-yl)phenyl)amino)pyrimidin-4-yl)amino)quinoxalin-5-yl)dimethylphosphine oxide (GT-S-51) prepared from Intermediate Example 80 and 3-(4-fluoro-5-(hydroxymethyl)-1-oxoisoindolin-2-yl)piperidine-2,6-dione (GT-03445) prepared from Intermediate Example 15. The target compound (GT-03925) was obtained as a white solid (22 mg, yield 36.17%). ¹H NMR (400 MHz, DMSO) δ 13.24 (s, 1H), 11.28 (s, 1H), 11.03 (s, 1H), 9.35 (s, 1H), 8.93 (s, 2H), 8.77 (s, 1H), 8.44 (s, 1H), 8.16 - 7.91 (m, 2H), 7.71 (d, *J* = 7.7 Hz, 1H), 7.39 (s, 1H), 6.87 (s, 1H), 5.16 (dd, *J* = 12.8, 4.3 Hz, 1H), 4.85 - 4.53 (m, 3H), 4.51 - 4.30 (m, 3H), 3.81 (s, 3H), 3.52 - 3.43 (m, 1H), 3.35 - 3.23 (m, 2H), 2.98 - 2.81 (m, 3H), 2.75 (d, *J* = 3.5 Hz, 3H), 2.67 - 2.56 (m, 2H), 2.47 - 2.35 (m, 2H), 2.27 (d, *J* = 12.5 Hz, 1H), 2.15 (s, 3H), 2.07 (s, 3H), 2.03 (s, 3H). LCMS (ESI) calcd for C₄₂H₄₆BrFN₁₀O₅P⁺ [M+H]⁺: 899.25, found, 899.30.

### Example 197: preparation of 3-(5-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)-6-fluoro-1-oxoisoindolin-2-yl)piperidine-2,6-dione (GT-03926)

The target compound (GT-03926) was prepared by referring to the methods of Scheme 11 and Example 1 (white solid, 22 mg, yield 36.16%). ¹H NMR (400 MHz, DMSO) δ 13.11 (s, 1H), 11.08 (d, *J* = 28.9 Hz, 2H), 9.36 - 8.95 (m, 1H), 8.92 (s, 2H), 8.87 - 8.68 (m, 1H), 8.40 (s, 1H), 8.13 (dd, *J* = 5.6, 3.1 Hz, 1H), 7.98 (d, *J* = 9.1 Hz, 1H), 7.71 (d, *J* = 7.7 Hz, 1H), 7.38 (s, 1H), 6.84 (s, 1H), 5.16 (ddd, *J* = 12.5, 3.7, 1.8 Hz, 1H), 4.70 (dd, *J* = 13.1, 2.8 Hz, 1H), 4.52 (dd, *J* = 17.7, 9.6 Hz, 1H), 4.43 - 4.36 (m, 2H), 3.81 (s, 3H), 3.59 - 3.37 (m, 3H), 3.25 (dd, *J* = 15.8, 6.6 Hz, 2H), 2.99 - 2.79 (m, 3H), 2.75 (s, 3H), 2.63 (ddd, *J* = 15.1, 4.1, 2.4 Hz, 2H), 2.45 (dd, *J* = 13.5, 4.5 Hz, 1H), 2.38 - 2.32 (m, 1H), 2.28 - 2.22 (m, 1H), 2.14 (s, 3H), 2.06 (s, 3H), 2.02 (s, 3H). LCMS (ESI) calcd for C₄₂H₄₆BrFN₁₀O₅P⁺ [M+H]⁺: 899.25, found, 899.30.

### Example 198: preparation of 3-(5-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)-7-fluoro-1-oxoisoindolin-2-yl)piperidine-2,6-dione (GT-03927)

Referring to the method of Scheme 11, the target compound (GT-03927) was prepared using (6-((5-bromo-2-((2-methoxy-5-methyl-4-(4-(methylamino)piperidin-1-yl)phenyl)amino)pyrimidin-4-yl)amino)quinoxalin-5-yl)dimethylphosphine oxide (GT-S-51) prepared from Intermediate Example 80 and 3-(7-fluoro-5-(hydroxymethyl)-1-oxoisoindolin-2-yl)piperidine-2,6-dione (GT-03446) prepared from Intermediate Example 15. The target compound (GT-03927) was obtained as a white solid (26.00 mg, yield 42.74%). ¹H NMR (400 MHz, DMSO) δ 13.13 (s, 1H), 11.44 (s, 1H), 11.02 (s, 1H), 9.11 (t, *J* = 11.9 Hz, 1H), 8.92 (s, 2H), 8.78 (d, *J* = 7.6 Hz, 1H), 8.40 (s, 1H), 7.98 (d, *J* = 9.4 Hz, 1H), 7.81 (d, *J* = 10.6 Hz, 2H), 7.38 (s, 1H), 6.85 (s, 1H), 5.11 (dd, *J* = 13.3, 5.0 Hz, 1H), 4.59 (ddd, *J* = 25.9, 13.3, 6.4 Hz, 2H), 4.46 - 4.38 (m, 2H), 3.80 (s, 3H), 3.50 - 3.33 (m, 2H), 3.24 (dd, *J* = 13.5, 7.7 Hz, 2H), 2.91 (ddd, *J* = 33.4, 19.5, 12.5 Hz, 3H), 2.69 (s, 3H), 2.65 - 2.55 (m, 2H), 2.45 - 2.24 (m, 4H), 2.14 (s, 3H), 2.06 (s, 3H), 2.03 (s, 3H). LCMS (ESI) calcd for C₄₂H₄₆BrFN₁₀O₅P⁺ [M+H]⁺: 899.25, found, 899.30.

### Example 199: preparation of 3-(4-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)-1-oxoisoindolin-2-yl)piperidine-2,6-dione (GT-03928)

The target compound (GT-03928) was prepared using the methods described in Scheme 11 and Example 1 (white solid, 20.00 mg, yield 33.55%). ¹H NMR (400 MHz, DMSO) δ 13.24 (s, 1H), 11.16 (d, *J* = 29.2 Hz, 1H), 11.07 (s, 1H), 9.35 (dt, *J* = 14.9, 11.4 Hz, 1H), 8.93 (s, 2H), 8.77 (dd, *J* = 12.9, 6.6 Hz, 1H), 8.44 (s, 1H), 8.20 - 7.89 (m, 2H), 7.85 (d, *J* = 7.6 Hz, 1H), 7.68 - 7.62 (m, 1H), 7.39 (s, 1H), 6.88 (s, 1H), 5.24 - 5.16 (m, 1H), 4.81 - 4.52 (m, 3H), 4.30 - 4.20 (m, 2H), 3.82 (s, 3H), 3.65 - 3.39 (m, 2H), 3.34 - 3.23 (m, 2H), 3.01 - 2.80 (m, 3H), 2.70 (dd, *J* = 12.3, 4.7 Hz, 3H), 2.64 - 2.53 (m, 1H), 2.46 - 2.20 (m, 4H), 2.15 (s, 3H), 2.07 (s, 3H), 2.03 (s, 3H). LCMS (ESI) calcd for C₄₂H₄₇BrN₁₀O₅P⁺ [M+H]⁺: 881.26, found, 881.30.

### Example 200: preparation of 5-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)-2-(2,6-dioxopiperidin-3-yl)isoindoline-1,3-dione (GT-03929)

Referring to the method of Step 2 in Scheme 11 and the method of Example 1, the target compound (GT-03929) was prepared using (6-((5-bromo-2-((2-methoxy-5-methyl-4-(4-(methylamino)piperidin-1-yl)phenyl)amino)pyrimidin-4-yl)amino)quinoxalin-5-yl)dimethylphosphine oxide (GT-S-51) prepared from Intermediate Example 80 and 5-(bromomethyl)-2-(2,6-dioxo-piperidin-3-yl)isoindoline-1,3-dione (CAS NO.: 1312023-72-5). The target compound (GT-03929) was obtained as a white solid (20.00 mg, yield 33.03%). ¹H NMR (400 MHz, DMSO) δ 13.05 (s, 1H), 11.31 (s, 1H), 11.15 (s, 1H), 9.02 - 8.87 (m, 3H), 8.86 - 8.73 (m, 1H), 8.37 (d, *J* = 4.6 Hz, 2H), 8.25 (d, *J* = 7.7 Hz, 1H), 8.05 (d, *J* = 7.6 Hz, 1H), 7.97 (d, *J* = 9.1 Hz, 1H), 7.38 (s, 1H), 6.83 (s, 1H), 5.20 (dd, *J* = 12.9, 5.3 Hz, 1H), 4.80 - 4.68 (m, 1H), 4.50 (ddd, *J* = 8.1, 5.8, 1.3 Hz, 1H), 3.80 (s, 3H), 3.41 (dd, *J* = 18.5, 10.1 Hz, 2H), 3.24 (s, 2H), 2.96 - 2.78 (m, 3H), 2.68 (d, *J* = 4.2 Hz, 3H), 2.59 (ddd, *J* = 13.0, 5.6, 4.7 Hz, 4H), 2.35 - 2.28 (m, 2H), 2.14 (s, 3H), 2.06 (s, 3H), 2.02 (s, 3H). LCMS (ESI) calcd for C₄₂H₄₅BrN₁₀O₆P⁺ [M+H]⁺: 895.24, found, 895.20.

### Example 201: preparation of 3-(5-(((1-(4-((9-cyclopentyl-8-(phenylamino)-9H-purin-2-yl)amino)phenyl)piperidin-4-yl)(methyl)amino)methyl)-4-fluoro-1-oxoisoindolin-2-yl)piperidine-2,6-dione (GT-03858)

Referring to the method of Scheme 11, the target compound (GT-03858) was prepared using (9-cyclopentyl-N²-(4-(4-(methylamino)piperidin-1-yl)phenyl)-N⁸-phenyl-9H-purine-2,8-diamine (GT-D-67) prepared from Intermediate Example 48 and 3-(4-fluoro-5-(hydroxymethyl)-1-oxoisoindolin-2-yl)piperidine-2,6-dione (GT-03445) prepared from Intermediate Example 15. The target compound (GT-03858) was obtained as a white solid (28 mg, yield 38.40%). ¹H NMR (400 MHz, DMSO) δ 11.61 (s, 1H), 11.03 (s, 1H), 10.44 (s, 1H), 8.48 (s, 1H), 8.06 (t, *J* = 6.9 Hz, 1H), 7.82 (d, *J* = 7.9 Hz, 2H), 7.70 (d, *J* = 7.7 Hz, 3H), 7.66 - 7.56 (m, 2H), 7.41 (t, *J* = 7.9 Hz, 2H), 7.16 (t, *J* = 7.4 Hz, 1H), 5.31 (dd, *J* = 16.3, 7.8 Hz, 1H), 5.16 (dd, *J* = 13.3, 5.0 Hz, 1H), 4.62 (d, *J* = 17.8 Hz, 2H), 4.50 - 4.44 (m, 2H), 3.80 - 3.65 (m, 4H), 3.43 (s, 2H), 2.97 - 2.89 (m, 1H), 2.74 (s, 3H), 2.61 (d, *J* = 16.9 Hz, 1H), 2.36 (ddd, *J* = 19.1, 16.2, 6.1 Hz, 5H), 2.04 (ddd, *J* = 35.3, 18.6, 4.7 Hz, 6H), 1.67 (dd, *J* = 12.1, 6.2 Hz, 2H). LCMS (ESI) calcd for C₄₂H₄₆FN₁₀O₃⁺ [M+H]⁺: 757.37, found, 757.40.

### Example 202: preparation of 3-(5-(((1-(4-((9-cyclopentyl-8-(phenylamino)-9H-purin-2-yl)amino)phenyl)piperidin-4-yl)(methyl)amino)methyl)-6-fluoro-1-oxoisoindolin-2-yl)piperidine-2,6-dione (GT-03859)

The target compound (GT-03859) was prepared using the methods described in Scheme 11 and Example 1 (white solid, 20.00 mg, yield 27.43%). ¹H NMR (400 MHz, DMSO) δ 11.49 (s, 1H), 11.03 (s, 1H), 10.39 (s, 1H), 8.47 (s, 1H), 8.14 (d, *J* = 5.4 Hz, 1H), 7.81 (d, *J* = 7.9 Hz, 2H), 7.69 (d, *J* = 8.5 Hz, 3H), 7.62 - 7.46 (m, 2H), 7.41 (t, *J* = 7.8 Hz, 2H), 7.15 (t, *J* = 7.4 Hz, 1H), 5.29 (dd, *J* = 16.6, 8.2 Hz, 1H), 5.15 (dd, *J* = 13.0, 4.9 Hz, 1H), 4.65 (d, *J =* 11.9 Hz, 1H), 4.45 - 4.34 (m, 3H), 3.79 (d, *J* = 7.9 Hz, 2H), 3.66 (s, 2H), 3.39 (s, 2H), 2.95 - 2.88 (m, 1H), 2.74 (s, 3H), 2.61 (dd, *J* = 15.8, 1.0 Hz, 1H), 2.45 - 2.31 (m, 5H), 2.15 - 1.87 (m, 6H), 1.71 - 1.62 (m, 2H). LCMS (ESI) calcd for C₄₂H₄₆FN₁₀O₃⁺ [M+H]⁺: 757.37, found, 757.40.

### Example 203: preparation of 3-(5-(((1-(4-((9-cyclopentyl-8-(phenylamino)-9H-purin-2-yl)amino)phenyl)piperidin-4-yl)(methyl)amino)methyl)-7-fluoro-1-oxoisoindolin-2-yl)piperidine-2,6-dione (GT-03860)

Referring to the method of Scheme 11, the target compound (GT-03860) was prepared using (9-cyclopentyl-N²-(4-(4-(methylamino)piperidin-1-yl)phenyl)-N⁸-phenyl-9H-purine-2,8-diamine (GT-D-67) prepared from Intermediate Example 48 and 3-(7-fluoro-5-(hydroxymethyl)-1-oxoisoindolin-2-yl)piperidine-2,6-dione (GT-03446) prepared from Intermediate Example 16. The target compound (GT-03860) was obtained as a white solid (20 mg, yield 27.43%). ¹H NMR (400 MHz, DMSO) δ 11.75 (s, 1H), 11.03 (s, 1H), 10.40 (s, 1H), 8.48 (s, 1H), 7.85 - 7.78 (m, 4H), 7.71 (d, *J* = 7.9 Hz, 2H), 7.63 (s, 2H), 7.42 (t, *J* = 7.7 Hz, 2H), 7.17 (t, *J* = 7.5 Hz, 1H), 5.27 (dd, *J* = 17.3, 8.8 Hz, 1H), 5.11 (dd, *J* = 13.1, 5.0 Hz, 1H), 4.57 - 4.44 (m, 4H), 3.75 (d, *J* = 8.7 Hz, 2H), 3.66 - 3.57 (m, 2H), 3.50 - 3.37 (m, 2H), 2.95 - 2.88 (m, 1H), 2.69 - 2.58 (m, 4H), 2.44 - 2.31 (m, 5H), 2.14 - 1.91 (m, 6H), 1.67 (dd, *J* = 12.6, 6.6 Hz, 2H). LCMS (ESI) calcd for C₄₂H₄₆FN₁₀O₃⁺ [M+H]⁺: 757.37, found, 757.40.

### Example 204: preparation of 3-(4-(((1-(4-((9-cyclopentyl-8-(phenylamino)-9H-purin-2-yl)amino)phenyl)piperidin-4-yl)(methyl)amino)methyl)-1-oxoisoindolin-2-yl)piperidine-2,6-dione (GT-03861)

The target compound (GT-03861) was prepared using the methods described in Scheme 11 and Example 1 (white solid, 23 mg, yield 32.32%). ¹H NMR (400 MHz, DMSO) δ 11.62 (s, 1H), 11.06 (s, 1H), 10.58 (s, 1H), 8.50 (s, 1H), 8.09 (dd, *J* = 28.8, 7.4 Hz, 1H), 7.87 - 7.71 (m, 7H), 7.64 (t, *J* = 7.5 Hz, 1H), 7.42 (t, *J* = 7.7 Hz, 2H), 7.18 (t, *J* = 7.3 Hz, 1H), 5.39 - 5.32 (m, 1H), 5.19 (dd, *J* = 12.6, 8.0 Hz, 1H), 4.63 - 4.57 (m, 3H), 4.36 (dd, *J* = 11.0, 5.1 Hz, 2H), 3.86 - 3.73 (m, 3H), 3.61 (s, 2H), 2.95 (t, *J* = 15.0 Hz, 1H), 2.63 (dd, *J* = 34.2, 16.1 Hz, 6H), 2.39 - 2.26 (m, 3H), 2.16 - 1.83 (m, 6H), 1.70 - 1.59 (m, 2H). LCMS (ESI) calcd for C₄₂H₄₇N₁₀O₃⁺ [M+H]⁺: 739.38, found, 739.40.

### Example 205: preparation of 5-(((1-(4-((9-cyclopentyl-8-(phenylamino)-9H-purin-2-yl)amino)phenyl)piperidin-4-yl)(methyl)amino)methyl)-2-(2,6-dioxopiperidin-3-yl)isoindoline-1,3-dione (GT-03862)

Referring to the method of Step 2 in Scheme 11 and the method of Example 1, the target compound (GT-03862) was prepared using (9-cyclopentyl-N²-(4-(4-(methylamino)piperidin-1-yl)phenyl)-N⁸-phenyl-9H-purine-2,8-diamine (GT-D-67) prepared from Intermediate Example 48 and 5-(bromomethyl)-2-(2,6-dioxo-piperidin-3-yl)isoindoline-1,3-dione (CAS NO.: 1312023-72-5). The target compound (GT-03862) was obtained as a white solid (25 mg, yield 34.47%). ¹H NMR (400 MHz, DMSO) δ 11.74 (s, 1H), 11.16 (s, 1H), 10.35 (s, 1H), 8.47 (s, 1H), 8.39 (s, 1H), 8.28 (d, *J* = 7.6 Hz, 1H), 8.03 (d, *J* = 7.7 Hz, 1H), 7.82 (d, *J* = 7.9 Hz, 2H), 7.68 (d, *J* = 7.3 Hz, 2H), 7.55 (s, 2H), 7.40 (t, *J* = 7.8 Hz, 2H), 7.14 (t, *J* = 7.3 Hz, 1H), 5.29 - 5.17 (m, 2H), 4.69 (d, *J* = 12.3 Hz, 1H), 4.53 (d, *J* = 10.9 Hz, 1H), 3.77 (s, 3H), 3.64 (d, *J* = 3.9 Hz, 1H), 3.35 (s, 1H), 2.93 - 2.86 (m, 1H), 2.66 (s, 3H), 2.58 (d, *J* = 10.5 Hz, 1H), 2.34 (dd, *J* = 11.2, 7.3 Hz, 3H), 2.13 - 2.06 (m, 3H), 1.96 (d, *J* = 14.1 Hz, 2H), 1.69 - 1.62 (m, 2H). LCMS (ESI) calcd for C₄₂H₄₅N₁₀O₄⁺ [M+H]⁺: 753.35, found, 753.40.

### Example 206: preparation of 3-(5-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)-1-oxoisoindolin-2-yl)piperidine-2,6-dione (GT-03907)

Referring to the method of Step 2 in Scheme 11 and the method of Example 1, the target compound (GT-03907) was prepared using (6-((5-bromo-2-((2-methoxy-5-methyl-4-(piperazin-1-yl)phenyl)amino)pyrimidin-4-yl)amino)quinoxalin-5-yl)dimethylphosphine oxide (GT-S-50) prepared from Intermediate Example 79 and 3-(5-(bromomethyl)-1-oxoisoindolin-2-yl)piperidine-2,6-dione. The target compound (GT-03907) was obtained as a white solid (40.00 mg, yield 66.67%). ¹H NMR (400 MHz, DMSO) δ 12.86 (s, 1H), 11.27 (s, 1H), 11.03 (s, 1H), 8.88 (dd, *J* = 10.2, 1.7 Hz, 3H), 8.57 (s, 1H), 8.32 (s, 1H), 7.96 (d, *J* = 10.0 Hz, 2H), 7.88 - 7.80 (m, 2H), 7.46 (s, 1H), 6.72 (s, 1H), 5.16 (dd, *J* = 13.2, 5.1 Hz, 1H), 4.53 (d, *J* = 17.7 Hz, 3H), 4.40 (d, *J* = 17.7 Hz, 1H), 3.80 (s, 3H), 3.41 (d, *J* = 10.9 Hz, 3H), 3.31 - 3.16 (m, 6H), 2.98 - 2.88 (m, 1H), 2.61 (d, *J* = 17.1 Hz, 1H), 2.47 - 2.39 (m, 1H), 2.11 (s, 3H), 2.05 (s, 3H), 2.01 (s, 3H). LCMS (ESI) calcd for C₄₀H₄₃BrN₁₀O₅P⁺ [M+H]⁺: 853.23, found, 853.20.

### Example 207: preparation of 3-(5-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)-4-fluoro-1-oxoisoindolin-2-yl)piperidine-2,6-dione (GT-03908)

Referring to the method of Scheme 11, the target compound (GT-03908) was prepared using (6-((5-bromo-2-((2-methoxy-5-methyl-4-(piperazin-1-yl)phenyl)amino)pyrimidin-4-yl)amino)quinoxalin-5-yl)dimethylphosphine oxide (GT-S-50) prepared from Intermediate Example 79 and 3-(4-fluoro-5-(hydroxymethyl)-1-oxoisoindolin-2-yl)piperidine-2,6-dione (GT-03445). The target compound (GT-03908) was obtained as a white solid (40.00 mg, yield 65.30%). ¹H NMR (400 MHz, DMSO) δ 12.85 (s, 1H), 11.20 (s, 1H), 11.03 (s, 1H), 8.93 - 8.79 (m, 3H), 8.54 (s, 1H), 8.32 (s, 1H), 8.04 - 7.90 (m, 2H), 7.73 (d, *J* = 7.7 Hz, 1H), 7.47 (s, 1H), 6.72 (s, 1H), 5.16 (dd, *J* = 13.3, 5.0 Hz, 1H), 4.63 (d, *J* = 17.8 Hz, 3H), 4.48 (d, *J* = 17.6 Hz, 1H), 3.80 (s, 3H), 3.43 - 3.27 (m, 5H), 3.20 (s, 4H), 2.98 - 2.89 (m, 1H), 2.62 (d, *J* = 17.7 Hz, 1H), 2.44 (dd, *J* = 13.7, 5.1 Hz, 1H), 2.11 (s, 3H), 2.05 (s, 3H), 2.01 (s, 3H). LCMS (ESI) calcd for C₄₀H₄₂BrFN₁₀O₅P⁺ [M+H]⁺: 871.22, found, 871.20.

### Example 208: preparation of 3-(5-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)-6-fluoro-1-oxoisoindolin-2-yl)piperidine-2,6-dione (GT-03909)

The target compound (GT-03909) was prepared using the methods described in Scheme 11 and Example 1 (white solid, 40.00 mg, yield 65.30%). ¹H NMR (400 MHz, DMSO) δ 12.84 (s, 1H), 11.15 (s, 1H), 11.04 (s, 1H), 8.87 (dt, *J* = 10.8, 5.4 Hz, 3H), 8.53 (s, 1H), 8.32 (s, 1H), 8.09 (d, *J*= 5.9 Hz, 1H), 7.96 (d, *J* = 9.4 Hz, 1H), 7.72 (d, *J =* 8.5 Hz, 1H), 7.46 (s, 1H), 6.71 (s, 1H), 5.16 (dd, *J =* 13.4, 4.9 Hz, 1H), 4.63 - 4.47 (m, 3H), 4.39 (d, *J* = 17.4 Hz, 1H), 3.80 (s, 3H), 3.34 (dd, *J* = 15.5, 6.9 Hz, 4H), 3.20 (d, *J* = 0.9 Hz, 5H), 2.97 - 2.89 (m, 1H), 2.67 - 2.58 (m, 1H), 2.47 - 2.43 (m, 1H), 2.11 (s, 3H), 2.05 (s, 3H), 2.01 (s, 3H). LCMS (ESI) calcd for C₄₀H₄₂BrFN₁₀O₅P⁺ [M+H]⁺: 871.22, found, 871.20.

### Example 209: preparation of 3-(5-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)-7-fluoro-1-oxoisoindolin-2-yl)piperidine-2,6-dione (GT-03910)

Referring to the method of Scheme 11, the target compound (GT-03910) was prepared using (6-((5-bromo-2-((2-methoxy-5-methyl-4-(piperazin-1-yl)phenyl)amino)pyrimidin-4-yl)amino)quinoxalin-5-yl)dimethylphosphine oxide (GT-S-50) prepared from Intermediate Example 79 and 3-(7-fluoro-5-(hydroxymethyl)-1-oxoisoindolin-2-yl)piperidine-2,6-dione (GT-03446). The target compound (GT-03910) was obtained as a white solid (40.00 mg, yield 65.30%). ¹H NMR (400 MHz, DMSO) δ 12.87 (s, 1H), 11.42 (s, 1H), 11.02 (s, 1H), 8.91 - 8.75 (m, 3H), 8.58 (s, 1H), 8.33 (s, 1H), 7.97 (d, *J* = 9.4 Hz, 1H), 7.73 (d, *J* = 13.7 Hz, 2H), 7.47 (s, 1H), 6.73 (s, 1H), 5.12 (dd, *J* = 13.2, 5.0 Hz, 1H), 4.64 - 4.51 (m, 3H), 4.42 (d, *J* = 18.1 Hz, 1H), 3.81 (s, 3H), 3.43 (d, *J* = 10.4 Hz, 3H), 3.29 - 3.15 (m, 6H), 2.97 - 2.88 (m, 1H), 2.61 (dd, *J* = 16.7, 1.7 Hz, 1H), 2.42 (dd, *J* = 13.0, 4.8 Hz, 1H), 2.11 (s, 3H), 2.05 (s, 3H), 2.02 (s, 3H). LCMS (ESI) calcd for C₄₀H₄₂BrFN₁₀O₅P⁺ [M+H]⁺: 871.22, found, 871.20.

### Example 210: preparation of 3-(4-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)-1-oxoisoindolin-2-yl)piperidine-2,6-dione (GT-03911)

The target compound (GT-03911) was prepared using the methods described in Scheme 11 and Example 1 (white solid, 40.00 mg, yield 66.67%). ¹H NMR (400 MHz, DMSO) δ 12.97 (s, 1H), 11.43 (s, 1H), 11.09 (s, 1H), 8.86 (ddd, *J* = 19.8, 11.9, 5.7 Hz, 4H), 8.36 (s, 1H), 8.12 (d, *J* = 7.6 Hz, 1H), 7.97 (d, *J* = 9.3 Hz, 1H), 7.85 (d, *J* = 7.5 Hz, 1H), 7.66 (t, *J* = 7.6 Hz, 1H), 7.46 (s, 1H), 6.72 (s, 1H), 5.21 (dd, *J* = 13.0, 5.0 Hz, 1H), 4.93 (d, *J* = 17.7 Hz, 1H), 4.61 - 4.45 (m, 3H), 3.80 (s, 3H), 3.50 - 3.32 (m, 5H), 3.24 (dd, *J* = 28.3, 11.8 Hz, 4H), 3.02 - 2.91 (m, 1H), 2.66 (dd, *J* = 15.5, 1.5 Hz, 1H), 2.41 - 2.30 (m, 1H), 2.13 (s, 3H), 2.05 (s, 3H), 2.02 (s, 3H). LCMS (ESI) calcd for C₄₀H₄₃BrN₁₀O₅P⁺ [M+H]⁺: 853.23, found, 853.20.

### Example 211: preparation of 5-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)-2-(2,6-dioxopiperidin-3-yl)isoindoline-1,3-dione (GT-03912)

Referring to the method of Step 2 in Scheme 11 and the method of Example 1, the target compound (GT-03912) was prepared using (6-((5-bromo-2-((2-methoxy-5-methyl-4-(piperazin-1-yl)phenyl)amino)pyrimidin-4-yl)amino)quinoxalin-5-yl)dimethylphosphine oxide (GT-S-50) prepared from Intermediate Example 79 and 5-(bromomethyl)-2-(2,6-dioxo-piperidin-3-yl)isoindoline-1,3-dione (CAS NO.: 1312023-72-5). The target compound (GT-03912) was obtained as a white solid (40.00 mg, yield 65.60%). ¹H NMR (400 MHz, DMSO) δ 12.83 (s, 1H), 11.42 (s, 1H), 11.15 (s, 1H), 9.04 - 8.80 (m, 3H), 8.50 (s, 1H), 8.31 (s, 2H), 8.19 (d, *J* = 7.8 Hz, 1H), 8.06 (d, *J* = 7.6 Hz, 1H), 7.96 (d, *J* = 9.5 Hz, 1H), 7.47 (s, 1H), 6.73 (s, 1H), 5.20 (dd, *J* = 12.9, 5.3 Hz, 1H), 4.64 (s, 2H), 3.80 (s, 3H), 3.38 - 3.10 (m, 8H), 2.96 - 2.84 (m, 1H), 2.66 - 2.53 (m, 3H), 2.11 (s, 3H), 2.05 (s, 3H), 2.01 (s, 3H). LCMS (ESI) calcd for C₄₀H₄₁BrN₁₀O₆P⁺ [M+H]⁺: 867.21, found, 867.20.

### Example 212: preparation of N-(5-(((5-(tert-butyl)oxazol-2-yl)methyl)thio)thiazol-2-yl)-1'-((2-(2,6-dioxopiperidin-3-yl)-1-oxoisoindolin-5-yl)methyl)-[1,4'-bipiperidine]-4-carboxamide (GT-03483)

Referring to the method of Step 2 in Scheme 11 and the method of Example 1, the target compound (GT-03483) was prepared using N-(5-(((5-(tert-Butyl)oxazol-2-yl)methyl)thio)thiazol-2-yl)-[1,4'-bipiperidine]-4-carboxamide (CAS NO.: 2791384-17-1) and 3-(5-(bromomethyl)-1-oxoisoindolin-2-yl)piperidine-2,6-dione (GT-03234). The target compound (GT-03483) was obtained as a white solid (20.00 mg, yield 64.41%). ¹H NMR (400 MHz, DMSO) δ 12.45 (s, 1H), 9.22 (s, 1H), 7.88 - 7.81 (m, 2H), 7.78 - 7.73 (m, 1H), 7.40 - 7.37 (m, 1H), 7.25 - 7.12 (m, 1H), 6.76 - 6.71 (m, 1H), 5.14 (dd, *J* = 13.2, 5.1 Hz, 1H), 4.51 (d, *J* = 17.3 Hz, 1H), 4.44 - 4.35 (m, 3H), 4.07 (d, *J* = 11.9 Hz, 2H), 2.98 (td, *J* = 13.3, 7.1 Hz, 5H), 2.81 - 2.74 (m, 1H), 2.68 - 2.57 (m, 2H), 2.45 - 2.39 (m, 1H), 2.34 - 2.15 (m, 7H), 2.09 -1.94 (m, 6H), 1.18 (d, *J* = 2.4 Hz, 9H). LCMS (ESI) calcd for C₃₆H₄₆N₇O₅S₂⁺ [M+H]⁺: 720.29, found, 720.30.

### Example 213: preparation of 3-(5-((4-((5-chloro-4-(5-(cyclopropylmethyl)-1-methyl-1H-pyrazol-4-yl)pyrimidin-2-yl)amino)piperidin-1-yl)methyl)-1-oxoisoindolin-2-yl)piperidine-2,6-dione (GT-04044)

The target compound (GT-04044) was prepared using the methods described in Scheme 11 and Example 1 (white solid, 51 mg, yield 63.53%). ¹H NMR (400 MHz, DMSO) δ 11.17 (d, *J* = 53.3 Hz, 1H), 11.00 (s, 1H), 8.37 - 8.28 (m, 1H), 7.96 (d, *J* = 7.4 Hz, 1H), 7.90 (s, 1H), 7.80 (q, *J* = 7.8 Hz, 2H), 7.72 - 7.48 (m, 1H), 5.14 (dd, *J* = 13.2, 5.0 Hz, 1H), 4.57 - 4.27 (m, 4H), 3.85 (s, 3H), 3.49 - 3.19 (m, 3H), 3.13 - 2.85 (m, 5H), 2.61 (d, *J* = 17.3 Hz, 1H), 2.46 -2.35 (m, 1H), 2.16 - 1.85 (m, 5H), 0.92 (td, *J* = 12.7, 7.7 Hz, 1H), 0.40 - 0.29 (m, 2H), 0.09 (dt, *J* = 9.2, 4.2 Hz, 2H). LCMS (ESI) calcd for C₃₁H₃₆ClN₈O₃⁺ [M+H]⁺: 603.25, found, 603.30.

### Example 214: preparation of 3-(5-((4-((5-chloro-4-(5-(cyclopropylmethyl)-1-methyl-1H-pyrazol-4-yl)pyrimidin-2-yl)amino)piperidin-1-yl)methyl)-4-fluoro-1-oxoisoindolin-2-yl)piperidine-2,6-dione (GT-04045)

The target compound (GT-04044) was prepared using the methods described in Scheme 11 and Example 1 (white solid, 62 mg, yield 74.99%). ¹H NMR (400 MHz, DMSO) δ 11.34 (d, *J* = 65.2 Hz, 1H), 11.02 (s, 1H), 8.39 - 8.27 (m, 1H), 8.10 - 7.83 (m, 2H), 7.68 (d, *J* = 7.7 Hz, 1H), 5.15 (dd, *J* = 13.2, 5.0 Hz, 1H), 4.64 - 4.54 (m, 1H), 4.51 - 4.36 (m, 3H), 3.85 (s, 3H), 3.65 - 3.20 (m, 3H), 3.28 - 2.75 (m, 5H), 2.61 (d, *J* = 17.0 Hz, 1H), 2.49 - 2.37 (m, 1H), 2.21 - 1.85 (m, 5H), 0.92 (td, *J* = 12.8, 7.0 Hz, 1H), 0.40 - 0.27 (m, 2H), 0.09 (dd, *J* = 12.5, 7.7 Hz, 2H). LCMS (ESI) calcd for C₃₁H₃₅FClN₈O₃⁺ [M+H]⁺: 621.24, found, 621.30.

### Example 215: preparation of 3-(5-((4-((5-chloro-4-(5-(cyclopropylmethyl)-1-methyl-1H-pyrazol-4-yl)pyrimidin-2-yl)amino)piperidin-1-yl)methyl)-6-fluoro-1-oxoisoindolin-2-yl)piperidine-2,6-dione (GT-04046)

The target compound (GT-04046) was prepared using the methods described in Scheme 11 and Example 1 (white solid, 57 mg, yield 68.95%). ¹H NMR (400 MHz, DMSO) δ 11.43 (d, *J* = 54.9 Hz, 1H), 11.02 (s, 1H), 8.39 - 8.28 (m, 1H), 8.16 - 8.07 (m, 1H), 7.97 (d, *J* = 8.4 Hz, 1H), 7.66 (d, *J* = 8.5 Hz, 1H), 5.14 (dd, *J* = 13.2, 5.0 Hz, 1H), 4.53 - 4.32 (m, 4H), 3.90 (dd, *J* = 13.9, 7.1 Hz, 1H), 3.85 (s, 3H), 3.66 - 3.16 (m, 3H), 3.13 - 2.98 (m, 3H), 2.95 - 2.84 (m, 1H), 2.61 (d, *J* = 17.3 Hz, 1H), 2.47 - 2.32 (m, 1H), 2.23 - 1.86 (m, 5H), 1.02 - 0.81 (m, 1H), 0.41 - 0.27 (m, 2H), 0.09 (dd, *J* = 12.0, 7.2 Hz, 2H). LCMS (ESI) calcd for C₃₁H₃₅FClN₈O₃⁺ [M+H]⁺: 621.24, found, 621.30.

### Example 216: preparation of 3-(5-((4-((5-chloro-4-(5-(cyclopropylmethyl)-1-methyl-1H-pyrazol-4-yl)pyrimidin-2-yl)amino)piperidin-1-yl)methyl)-7-fluoro-1-oxoisoindolin-2-yl)piperidine-2,6-dione (GT-04047)

The target compound (GT-04047) was prepared using the methods described in Scheme 11 and Example 1 (white solid, 64 mg, yield 77.41%). ¹H NMR (400 MHz, DMSO) δ 11.47 (d, *J* = 48.5 Hz, 1H), 11.02 (s, 1H), 8.33 (d, *J* = 16.9 Hz, 1H), 7.96 (s, 1H), 7.72 (t, *J* = 4.8 Hz, 2H), 5.10 (dd, *J* = 13.2, 5.0 Hz, 1H), 4.46 (q, *J* = 17.9 Hz, 4H), 3.85 (s, 4H), 3.55 - 3.08 (m, 3H), 3.05 - 2.86 (m, 4H), 2.60 (d, *J* = 17.0 Hz, 1H), 2.41 (qd, *J* = 13.1, 4.3 Hz, 1H), 2.21 - 1.87 (m, 5H), 1.02 - 0.81 (m, 1H), 0.34 (tt, *J* = 5.8, 5.3 Hz, 2H), 0.15 - 0.02 (m, 2H). LCMS (ESI) calcd for C₃₁H₃₅FClN₈O₃⁺ [M+H]⁺: 621.24, found, 621.30.

### Example 217: preparation of 3-(4-((4-((5-chloro-4-(5-(cyclopropylmethyl)-1-methyl-1H-pyrazol-4-yl)pyrimidin-2-yl)amino)piperidin-1-yl)methyl)-1-oxoisoindolin-2-yl)piperidine-2,6-dione (GT-04048)

The target compound (GT-04048) was prepared using the methods described in Scheme 11 and Example 1 (white solid, 63 mg, yield 78.48%). ¹H NMR (500 MHz, DMSO) δ 11.03 (dd, *J* = 50.2, 14.8 Hz, 2H), 8.39 - 8.29 (m, 1H), 7.99 (t, *J* = 14.1 Hz, 1H), 7.84 (d, *J* = 7.5 Hz, 1H), 7.72 - 7.50 (m, 2H), 5.24 - 5.13 (m, 1H), 4.85 (dd, *J =* 17.4, 8.8 Hz, 1H), 4.59 - 4.47 (m, 1H), 4.44 - 4.27 (m, 2H), 3.86 (s, 4H), 3.48 - 2.90 (m, 7H), 2.71 - 2.60 (m, 1H), 2.34 (tt, *J* = 13.2, 6.5 Hz, 1H), 2.06 (ddd, *J* = 71.1, 48.4, 13.3 Hz, 5H), 0.91 (tdt, *J* = 20.5, 13.8, 6.8 Hz, 1H), 0.47 - 0.27 (m, 2H), 0.09 (t, *J* = 14.4 Hz, 2H). LCMS (ESI) calcd for C₃₁H₃₆ClN₈O₃⁺ [M+H]⁺: 603.25, found, 603.30.

### Example 218: preparation of 5-((4-((5-chloro-4-(5-(cyclopropylmethyl)-1-methyl-1H-pyrazol-4-yl)pyrimidin-2-yl)amino)piperidin-1-yl)methyl)-2-(2,6-dioxopiperidin-3-yl)isoindoline-1,3-dione (GT-04049)

The target compound (GT-04049) was prepared using the methods described in Scheme 11 and Example 1 (white solid, 55 mg, yield 66.96%). ¹H NMR (400 MHz, DMSO) δ 11.35 (s, 1H), 11.14 (s, 1H), 8.31 (t, *J* = 14.5 Hz, 2H), 8.16 (d, *J* = 7.7 Hz, 1H), 8.02 (d, *J* = 7.7 Hz, 1H), 7.94 (d, *J* = 8.1 Hz, 1H), 5.19 (dd, *J* = 12.8, 5.4 Hz, 1H), 4.51 (d, *J* = 4.0 Hz, 2H), 3.85 (s, 3H), 3.62 - 3.11 (m, 3H), 3.06-2.87 (m, 4H), 2.70 - 2.50 (m, 3H), 2.10 (dd, *J* = 28.4, 23.1 Hz, 3H), 1.93 (dd, *J* = 23.9, 10.5 Hz, 2H), 1.02 - 0.86 (m, 1H), 0.35 (dt, *J* = 12.0, 6.6 Hz, 2H), 0.09 (dd, *J* = 10.2, 5.8 Hz, 2H). LCMS (ESI) calcd for C₃₁H₃₄ClN₈O₄⁺ [M+H]⁺: 617.23, found, 617.30.

### Example 219: preparation of 4-(((5'-chloro-2'-((1-((2-(2,6-dioxopiperidin-3-yl)-1-oxoisoindolin-5-yl)methyl)piperidin-4-yl)amino)-[2,4'-bipyridin]-6-yl)amino)methyl)tetrahydro-2H-pyran-4-carbonitrile (GT-04060)

Referring to the method of Step 2 in Scheme 11 and the method of Example 1, the target compound (GT-04060) was prepared using 4-(((5'-chloro-2'-(piperidin-4-ylamino)-[2,4'-bipyridin]-6-yl)amino)methyl)tetrahydro-2H-pyran-4-carbonitrile (GT-M-159) prepared from Intermediate Example 38 and 3-(5-(bromomethyl)-1-oxoisoindolin-2-yl)piperidine-2,6-dione (GT-03234). The target compound (GT-04060) was obtained as a white solid (56 mg, yield 86.90%). ¹H NMR (400 MHz, DMSO) δ 11.32 (d, *J* = 52.7 Hz, 1H), 11.02 (s, 1H), 8.13 (d, *J* = 17.6 Hz, 1H), 7.92 (d, *J* = 6.1 Hz, 1H), 7.85 - 7.75 (m, 2H), 7.69 (d, *J* = 6.9 Hz, 1H), 7.34 - 6.61 (m, 3H), 5.15 (dd, *J* = 13.2, 5.0 Hz, 1H), 4.55 - 4.39 (m, 4H), 3.97 - 3.87 (m, 3H), 3.72 (d, *J* = 7.8 Hz, 2H), 3.45 (dd, *J* = 13.9, 8.7 Hz, 4H), 3.12 (dt, *J* = 21.8, 10.3 Hz, 2H), 2.98 - 2.86 (m, 1H), 2.63 (t, *J* = 15.7 Hz, 1H), 2.47 - 2.35 (m, 1H), 2.15 (d, *J* = 11.6 Hz, 2H), 2.07 - 1.92 (m, 3H), 1.87 (d, *J* = 13.1 Hz, 2H), 1.69 (tt, *J* = 13.6, 6.8 Hz, 2H). LCMS (ESI) calcd for C₃₆H₄₀ClN₈O₄⁺ [M+H]⁺: 683.28, found, 683.30.

### Example 220: preparation of 4-(((5'-chloro-2'-((1-((2-(2,6-dioxopiperidin-3-yl)-4-fluoro-1-oxoisoindolin-5-yl)methyl)piperidin-4-yl)amino)-[2,4'-bipyridin]-6-yl)amino)methyl)tetrahydro-2H-pyran-4-carbonitrile (GT-04061)

Referring to the method of Scheme 11, the target compound (GT-04061) was prepared using 4-(((5'-chloro-2'-(piperidin-4-ylamino)-[2,4'-bipyridin]-6-yl)amino)methyl)tetrahydro-2H-pyran-4-carbonitrile (GT-M-159) prepared from Intermediate Example 38 and 3-(4-fluoro-5-(hydroxymethyl)-1-oxoisoindolin-2-yl)piperidine-2,6-dione (GT-03445). The target compound (GT-04061) was obtained as a white solid (55 mg, yield 83.17%). ¹H NMR (400 MHz, DMSO) δ 11.37 (d, *J* = 53.9 Hz, 1H), 11.04 (s, 1H), 8.13 (d, *J* = 16.2 Hz, 1H), 8.05 - 7.95 (m, 1H), 7.76 - 7.60 (m, 2H), 7.33 - 6.74 (m, 3H), 5.15 (dt, *J* = 16.0, 7.9 Hz, 1H), 4.60 - 4.45 (m, 4H), 3.99 - 3.83 (m, 3H), 3.73 (s, 2H), 3.58 - 3.39 (m, 4H), 3.24 (d, *J* = 89.1 Hz, 2H), 2.99 - 2.85 (m, 1H), 2.62 (d, *J* = 16.8 Hz, 1H), 2.48 - 2.31 (m, 1H), 2.26 - 2.08 (m, 2H), 2.08 - 1.92 (m, 3H), 1.87 (d, *J* = 13.3 Hz, 2H), 1.69 (td, *J* = 13.5, 4.3 Hz, 2H). LCMS (ESI) calcd for C₃₆H₃₉FClN₈O₄⁺ [M+H]⁺: 701.27, found, 701.30.

### Example 221: preparation of 4-(((5'-chloro-2'-((1-((2-(2,6-dioxopiperidin-3-yl)-6-fluoro-1-oxoisoindolin-5-yl)methyl)piperidin-4-yl)amino)-[2,4'-bipyridin]-6-yl)amino)methyl)tetrahydro-2H-pyran-4-carbonitrile (GT-04062)

The target compound (GT-04062) was prepared using the methods described in Scheme 11 and Example 1 (white solid, 54 mg, yield 81.65%). ¹H NMR (400 MHz, DMSO) δ 11.41 (d, *J* = 54.2 Hz, 1H), 11.08 (d, *J* = 8.4 Hz, 1H), 8.18 (t, *J =* 11.8 Hz, 2H), 7.73 (dd, *J* = 8.4, 3.5 Hz, 2H), 7.32 - 6.66 (m, 3H), 5.20 (dt, *J* = 15.1, 7.6 Hz, 1H), 4.53 (dd, *J* = 31.5, 20.0 Hz, 4H), 4.00 - 3.89 (m, 3H), 3.78 (s, 2H), 3.52 (dd, *J* = 22.7, 11.8 Hz, 4H), 3.20 (d, *J* = 9.2 Hz, 2H), 3.04 - 2.92 (m, 1H), 2.70 (dd, *J* = 24.1, 9.3 Hz, 1H), 2.47 (ddd, *J* = 26.0, 17.5, 6.6 Hz, 1H), 2.33 - 2.14 (m, 2H), 2.06 (dd, *J* = 21.0, 9.0 Hz, 3H), 1.93 (d, *J* = 13.2 Hz, 2H), 1.74 (td, *J* = 13.6, 4.4 Hz, 2H). LCMS (ESI) calcd for C₃₆H₃₉FClN₈O₄⁺ [M+H]⁺: 701.27, found, 701.30.

### Example 222: preparation of 4-(((5'-chloro-2'-((1-((2-(2,6-dioxopiperidin-3-yl)-7-fluoro-1-oxoisoindolin-5-yl)methyl)piperidin-4-yl)amino)-[2,4'-bipyridin]-6-yl)amino)methyl)tetrahydro-2H-pyran-4-carbonitrile (GT-04063)

Referring to the method of Scheme 11, the target compound (GT-04063) was prepared using 4-(((5'-chloro-2'-(piperidin-4-ylamino)-[2,4'-bipyridin]-6-yl)amino)methyl)tetrahydro-2H-pyran-4-carbonitrile (GT-M-159) prepared from Intermediate Example 38 and 3-(7-fluoro-5-(hydroxymethyl)-1-oxoisoindolin-2-yl)piperidine-2,6-dione (GT-03446). The target compound (GT-04063) was obtained as a white solid (57 mg, yield 86.19%). ¹H NMR (400 MHz, DMSO) δ 11.56 (d, *J* = 39.7 Hz, 1H), 11.09 (s, 1H), 8.19 (d, *J* = 18.5 Hz, 1H), 7.86 - 7.67 (m, 3H), 7.36 - 6.82 (m, 3H), 5.17 (dd, *J* = 13.2, 5.0 Hz, 1H), 4.62 - 4.49 (m, 4H), 3.96 (dd, *J* = 10.5, 3.7 Hz, 3H), 3.79 (d, *J* = 6.8 Hz, 2H), 3.51 (t, *J* = 11.2 Hz, 4H), 3.19 (dd, *J* = 46.8, 36.5 Hz, 2H), 3.04 - 2.85 (m, 1H), 2.69 (t, *J* = 17.8 Hz, 1H), 2.53 - 2.38 (m, 1H), 2.30 - 2.15 (m, 2H), 2.13 - 1.99 (m, 3H), 1.98 - 1.86 (m, 2H), 1.74 (td, *J =* 13.5, 4.3 Hz, 2H). LCMS (ESI) calcd for C₃₆H₃₉FClN₈O₄⁺ [M+H]⁺: 701.27, found, 701.30.

### Example 223: preparation of 4-(((5'-chloro-2'-((1-((2-(2,6-dioxopiperidin-3-yl)-1-oxoisoindolin-4-yl)methyl)piperidin-4-yl)amino)-[2,4'-bipyridin]-6-yl)amino)methyl)tetrahydro-2H-pyran-4-carbonitrile (GT-04064)

The target compound (GT-04064) was prepared using the methods described in Scheme 11 and Example 1 (white solid, 56 mg, yield 86.90%). ¹H NMR (400 MHz, DMSO) δ 11.37 (d, *J* = 32.1 Hz, 1H), 11.12 (d, *J* = 10.0 Hz, 1H), 8.15 (dd, *J* = 34.4, 12.0 Hz, 2H), 7.88 (dd, *J* = 7.2, 3.8 Hz, 1H), 7.71 (dd, *J* = 21.0, 13.4 Hz, 2H), 7.08 (dd, *J* = 108.1, 79.7 Hz, 3H), 5.24 (dt, *J* = 13.2, 6.6 Hz, 1H), 5.06 - 4.95 (m, 1H), 4.59 (d, *J* = 17.5 Hz, 1H), 4.46 (t, *J =* 9.1 Hz, 2H), 4.04 - 3.93 (m, 3H), 3.78 (s, 2H), 3.51 (t, *J* = 11.2 Hz, 4H), 3.28 (t, *J* = 18.6 Hz, 2H), 3.09 - 2.95 (m, 1H), 2.71 (d, *J* = 17.3 Hz, 1H), 2.47 - 2.33 (m, 1H), 2.32 - 2.16 (m, 2H), 2.15 - 1.98 (m, 3H), 1.93 (d, *J* = 13.2 Hz, 2H), 1.74 (td, *J =* 13.6, 4.2 Hz, 2H). LCMS (ESI) calcd for C₃₆H₄₀ClN₈O₄⁺ [M+H]⁺: 683.28, found, 683.30.

### Example 224: preparation of 4-(((5'-chloro-2'-((1-((2-(2,6-dioxopiperidin-3-yl)-1,3-dioxoisoindolin-5-yl)methyl)piperidin-4-yl)amino)-[2,4'-bipyridin]-6-yl)amino)methyl)tetrahydro-2H-pyran-4-carbonitrile (GT-04065)

Referring to the method of Step 2 in Scheme 11 and the method of Example 1, the target compound (GT-04065) was prepared using 4-(((5'-chloro-2'-(piperidin-4-ylamino)-[2,4'-bipyridin]-6-yl)amino)methyl)tetrahydro-2H-pyran-4-carbonitrile (GT-M-159) prepared from Intermediate Example 38 and 5-(bromomethyl)-2-(2,6-dioxo-piperidin-3-yl)isoindoline-1,3-dione (CAS NO.: 1312023-72-5). The target compound (GT-04065) was obtained as a white solid (59 mg, yield 89.71%). ¹H NMR (400 MHz, DMSO) δ 11.57 (s, 1H), 11.14 (s, 1H), 8.28 (d, *J* = 13.0 Hz, 1H), 8.21 - 8.11 (m, 1H), 8.09 (s, 1H), 8.00 (dd, *J* = 7.6, 3.1 Hz, 1H), 7.71 (dd, *J* = 23.1, 15.7 Hz, 1H), 7.32 - 6.80 (m, 3H), 5.17 (dd, *J* = 12.8, 5.4 Hz, 1H), 4.54 (s, 2H), 3.91 (dd, *J =* 31.9, 22.2 Hz, 3H), 3.72 (d, *J* = 8.5 Hz, 2H), 3.44 (dt, *J* = 17.5, 8.8 Hz, 4H), 3.15 (dd, *J* = 64.2, 10.5 Hz, 2H), 2.97 - 2.82 (m, 1H), 2.65 - 2.50 (m, 2H), 2.13 (t*, J* = 13.4 Hz, 2H), 2.09 - 1.91 (m, 3H), 1.85 (d, *J* = 13.1 Hz, 2H), 1.66 (td, *J* = 13.6, 4.1 Hz, 2H). LCMS (ESI) calcd for C₃₆H₃₈ClN₈O₅⁺ [M+H]⁺: 697.26, found, 697.30.

### Example 225: preparation of 4-(((4-(5-chloro-2-((1-((2-(2,6-dioxopiperidin-3-yl)-1-oxoisoindolin-5-yl)methyl)piperidin-4-yl)amino)pyridin-4-yl)thiazol-2-yl)amino)methyl)tetrahydro-2H-pyran-4-carbonitrile (GT-04068)

Referring to the method of Step 2 in Scheme 11 and the method of Example 1, the target compound (GT-04068) was prepared using 4-(((4-(5-chloro-2-(piperidin-4-ylamino)pyridin-4-yl)thiazol-2-yl)amino)methyl)tetrahydro-2H-pyran-4-carbonitrile (GT-M-158) prepared from Intermediate Example 37 and 3-(5-(bromomethyl)-1-oxoisoindolin-2-yl)piperidine-2,6-dione (GT-03234). The target compound (GT-04068) was obtained as a white solid (41 mg, yield 63.78%). ¹H NMR (400 MHz, DMSO) δ 11.39 (d, *J* = 75.1 Hz, 1H), 11.02 (s, 1H), 8.40 (d, *J* = 5.9 Hz, 1H), 8.05 (d, *J* = 21.7 Hz, 1H), 7.94 (d, *J* = 8.9 Hz, 1H), 7.85 - 7.78 (m, 2H), 7.66 (d, *J* = 22.4 Hz, 1H), 7.56 (s, 1H), 5.15 (dd, *J* = 13.2, 5.0 Hz, 1H), 4.53 - 4.38 (m, 4H), 3.93 (dd, *J* = 11.6, 3.1 Hz, 3H), 3.72 (dd, *J* = 16.3, 5.6 Hz, 2H), 3.47 (dd, *J* = 12.0, 10.5 Hz, 4H), 3.07 (dd, *J* = 22.0, 10.4 Hz, 2H), 3.00 - 2.86 (m, 1H), 2.64 (t, *J* = 16.8 Hz, 1H), 2.47 - 2.34 (m, 1H), 2.14 (t, *J* = 15.5 Hz, 2H), 2.10 - 1.96 (m, 3H), 1.90 (dd, *J=* 15.0, 7.3 Hz, 2H), 1.79-1.64 (m, 2H). LCMS (ESI) calcd for C₃₄H₃₈ClN₈O₄S⁺ [M+H]⁺: 689.23, found, 689.30.

### Example 226: preparation of 4-(((4-(5-chloro-2-((1-((2-(2,6-dioxopiperidin-3-yl)-4-fluoro-1-oxoisoindolin-5-yl)methyl)piperidin-4-yl)amino)pyridin-4-yl)thiazol-2-yl)amino)methyl)tetrahydro-2H-pyran-4-carbonitrile (GT-04069)

Referring to the method of Scheme 11, the target compound (GT-04069) was prepared using 4-(((4-(5-chloro-2-(piperidin-4-ylamino)pyridin-4-yl)thiazol-2-yl)amino)methyl)tetrahydro-2H-pyran-4-carbonitrile (GT-M-158) prepared from Intermediate Example 37 and 3-(4-fluoro-5-(hydroxymethyl)-1-oxoisoindolin-2-yl)piperidine-2,6-dione (GT-03445). The target compound (GT-04069) was obtained as a white solid (40 mg, yield 60.63%). ¹H NMR (400 MHz, DMSO) δ 11.37 (d, *J* = 87.8 Hz, 1H), 11.04 (s, 1H), 8.41 (s, 1H), 8.13 - 7.94 (m, 2H), 7.73 -7.46 (m, 3H), 5.15 (dt, *J* = 16.1, 8.1 Hz, 1H), 4.74 - 4.53 (m, 2H), 4.49 (s, 2H), 4.03 (s, 1H), 3.93 (d, *J =* 9.5 Hz, 2H), 3.71 (t, *J* = 9.0 Hz, 2H), 3.49 (dd, *J* = 24.8, 12.6 Hz, 4H), 3.15 (s, 2H), 2.98 - 2.85 (m, 1H), 2.64 (t, *J* = 16.5 Hz, 1H), 2.42 (dd, *J* = 28.9, 19.7 Hz, 1H), 2.17 (d, *J* = 12.1 Hz, 2H), 2.09 - 1.93 (m, 3H), 1.88 (d, *J* = 13.1 Hz, 2H), 1.77 -1.61 (m, 2H). LCMS (ESI) calcd for C₃₄H₃₇FClN₈O₄S⁺ [M+H]⁺: 707.23, found, 707.30.

### Example 227: preparation of 4-(((4-(5-chloro-2-((1-((2-(2,6-dioxopiperidin-3-yl)-6-fluoro-1-oxoisoindolin-5-yl)methyl)piperidin-4-yl)amino)pyridin-4-yl)thiazol-2-yl)amino)methyl)tetrahydro-2H-pyran-4-carbonitrile (GT-04070)

The target compound (GT-04070) was prepared using the methods described in Scheme 11 and Example 1 (white solid, 40 mg, yield 60.63%). ¹H NMR (400 MHz, DMSO) δ 11.39 (d, *J* = 81.7 Hz, 1H), 11.02 (d, *J* = 8.9 Hz, 1H), 8.41 (s, 1H), 8.08 (dd, *J* = 22.6, 12.0 Hz, 2H), 7.74 - 7.46 (m, 3H), 5.14 (dt, *J* = 15.4, 7.7 Hz, 1H), 4.50 - 4.36 (m, 4H), 4.03 (s, 1H), 3.95 - 3.89 (m, 2H), 3.71 (t, *J* = 8.9 Hz, 2H), 3.49 (dd, *J* = 22.3, 11.5 Hz, 4H), 3.15 (d, *J* = 9.1 Hz, 2H), 2.99 - 2.87 (m, 1H), 2.61 (d, *J* = 16.9 Hz, 1H), 2.41 (ddd, *J* = 26.0, 17.5, 6.6 Hz, 1H), 2.17 (d, *J* = 11.8 Hz, 2H), 2.07 - 1.94 (m, 3H), 1.92 - 1.83 (m, 2H), 1.78 - 1.65 (m, 2H). LCMS (ESI) calcd for C₃₄H₃₇FClN₈O₄S⁺ [M+H]⁺: 707.23, found, 707.30.

### Example 228: preparation of 4-(((4-(5-chloro-2-((1-((2-(2,6-dioxopiperidin-3-yl)-7-fluoro-1-oxoisoindolin-5-yl)methyl)piperidin-4-yl)amino)pyridin-4-yl)thiazol-2-yl)amino)methyl)tetrahydro-2H-pyran-4-carbonitrile (GT-04071)

Referring to the method of Scheme 11, the target compound (GT-04071) was prepared using 4-(((4-(5-chloro-2-(piperidin-4-ylamino)pyridin-4-yl)thiazol-2-yl)amino)methyl)tetrahydro-2H-pyran-4-carbonitrile (GT-M-158) prepared from Intermediate Example 37 and 3-(7-fluoro-5-(hydroxymethyl)-1-oxoisoindolin-2-yl)piperidine-2,6-dione (GT-03446). The target compound (GT-04071) was obtained as a white solid (40 mg, yield 60.63%). ¹H NMR (400 MHz, DMSO) δ 11.54 (d, *J* = 66.7 Hz, 1H), 11.03 (s, 1H), 8.41 (d, *J* = 5.6 Hz, 1H), 8.05 (d, *J* = 23.1 Hz, 1H), 7.88 - 7.48 (m, 4H), 5.11 (dd, *J* = 13.2, 5.0 Hz, 1H), 4.58 - 4.42 (m, 4H), 4.06 (s, 1H), 3.94 -3.88 (m, 2H), 3.72 (dd, *J* = 15.3, 5.7 Hz, 2H), 3.47 (t, *J* = 11.3 Hz, 4H), 3.28 - 3.01 (m, 2H), 2.98 - 2.84 (m, 1H), 2.61 (d, *J* = 16.8 Hz, 1H), 2.46 - 2.32 (m, 1H), 2.17 (d, *J=* 11.5 Hz, 2H), 2.10 - 1.95 (m, 3H), 1.93 - 1.82 (m, 2H), 1.80 - 1.63 (m, 2H). LCMS (ESI) calcd for C₃₄H₃₇FClN₈O₄S⁺ [M+H]⁺: 707.23, found, 707.30.

### Example 229: preparation of 4-(((4-(5-chloro-2-((1-((2-(2,6-dioxopiperidin-3-yl)-1-oxoisoindolin-4-yl)methyl)piperidin-4-yl)amino)pyridin-4-yl)thiazol-2-yl)amino)methyl)tetrahydro-2H-pyran-4-carbonitrile (GT-04072)

The target compound (GT-04072) was prepared using the methods described in Scheme 11 and Example 1 (white solid, 41 mg, yield 63.78%). ¹H NMR (400 MHz, DMSO) δ 11.37 (d, *J* = 56.1 Hz, 1H), 11.11 - 10.99 (m, 1H), 8.41 (d, *J* = 5.7 Hz, 1H), 8.09 - 7.92 (m, 2H), 7.81 (dd, *J* = 16.8, 7.4 Hz, 1H), 7.74 - 7.44 (m, 3H), 5.24 - 5.12 (m, 1H), 4.99 (t, *J* = 16.7 Hz, 1H), 4.54 (d, *J* = 17.6 Hz, 1H), 4.45 - 4.39 (m, 2H), 4.05 (s, 1H), 3.92 (dd, *J* = 11.6, 3.0 Hz, 2H), 3.72 (dd, *J =* 17.0, 5.5 Hz, 2H), 3.56 - 3.37 (m, 4H), 3.20 (s, 2H), 3.02 - 2.87 (m, 1H), 2.65 (d, *J* = 17.4 Hz, 1H), 2.36 (ddd, *J* = 26.8, 13.5, 4.4 Hz, 1H), 2.25 - 1.95 (m, 5H), 1.92 - 1.82 (m, 2H), 1.82 - 1.64 (m, 2H). LCMS (ESI) calcd for C₃₄H₃₈ClN₈O₄S⁺ [M+H]⁺: 689.23, found, 689.30.

### Example 230: preparation of 4-(((4-(5-chloro-2-((1-((2-(2,6-dioxopiperidin-3-yl)-1,3-dioxoisoindolin-5-yl)methyl)piperidin-4-yl)amino)pyridin-4-yl)thiazol-2-yl)amino)methyl)tetrahydro-2H-pyran-4-carbonitrile (GT-04073)

Referring to the method of Step 2 in Scheme 11 and the method of Example 1, the target compound (GT-04073) was prepared using 4-(((4-(5-chloro-2-(piperidin-4-ylamino)pyridin-4-yl)thiazol-2-yl)amino)methyl)tetrahydro-2H-pyran-4-carbonitrile (GT-M-158) prepared from Intermediate Example 37 and 5-(bromomethyl)-2-(2,6-dioxo-piperidin-3-yl)isoindoline-1,3-dione (CAS NO.: 1312023-72-5). The target compound (GT-04073) was obtained as a white solid (40 mg, yield 60.98%). ¹H NMR (400 MHz, DMSO) δ 11.56 (d, *J* = 67.5 Hz, 1H), 11.16 (s, 1H), 8.40 (d, *J* = 5.7 Hz, 1H), 8.30 (d, *J* = 15.2 Hz, 1H), 8.18 (t, *J* = 9.3 Hz, 1H), 8.10 -7.98 (m, 2H), 7.66 (dd, *J* = 56.7, 32.7 Hz, 2H), 5.19 (dd, *J* = 12.8, 5.4 Hz, 1H), 4.56 (s, 2H), 4.05 (s, 1H), 3.93 (dd, *J* = 11.6, 2.9 Hz, 2H), 3.71 (t, *J* = 7.9 Hz, 2H), 3.47 (t, *J* = 11.3 Hz, 4H), 3.14 (t, *J* = 34.4 Hz, 2H), 2.97 - 2.82 (m, 1H), 2.60 (ddd, *J* = 21.7, 17.4, 9.8 Hz, 2H), 2.17 (d, *J* = 11.6 Hz, 2H), 2.10 - 1.94 (m, 3H), 1.93 - 1.82 (m, 2H), 1.80 - 1.65 (m, 2H). LCMS (ESI) calcd for C₃₄H₃₆ClN₈O₅S⁺ [M+H]⁺: 703.21, found, 703.30.

### Example 231: preparation of 4-((((2-(2,6-dioxopiperidin-3-yl)-1-oxoisoindolin-5-yl)methyl)amino)methyl)-N-(4-methyl-3-((4-(pyridin-3-yl)pyrimidin-2-yl)amino)phenyl)benzamide (GT-03377)

Referring to the method of Step 2 in Scheme 11 and the method of Example 1, the target compound (GT-03377) was prepared using 4-(aminomethyl)-N-(4-methyl-3-((4-(pyridin-3-yl)pyrimidin-2-yl)amino)phenyl)benzamide (CAS NO.: 791609-83-1) and 3-(5-(bromomethyl)-1-oxoisoindolin-2-yl)piperidine-2,6-dione (GT-03234). The target compound (GT-03377) was obtained as a white solid (38 mg, yield 48.03%). ¹H NMR (400 MHz, DMSO) δ 11.01 (s, 1H), 10.34 (s, 1H), 10.11 - 9.95 (m, 2H), 9.45 (d, *J* = 1.5 Hz, 1H), 9.18 (s, 1H), 8.94 (d, *J* = 8.3 Hz, 1H), 8.91 - 8.88 (m, 1H), 8.60 (d, *J* = 5.2 Hz, 1H), 8.15 (d, *J* = 1.3 Hz, 1H), 8.04 (d, *J* = 8.3 Hz, 2H), 7.92 (dd, *J* = 8.0, 5.3 Hz, 1H), 7.84 - 7.78 (m, 2H), 7.73 (d, *J* = 8.4 Hz, 3H), 7.56 (d, *J* = 5.2 Hz, 1H), 7.49 (dd, *J* = 8.2, 1.9 Hz, 1H), 7.23 (d, *J* = 8.5 Hz, 1H), 5.13 (dd, *J* = 13.2, 5.1 Hz, 1H), 4.47 (s, 1H), 4.38 (s, 1H), 4.30 (d, *J* = 5.1 Hz, 2H), 4.26 - 4.23 (m, 2H), 2.96 - 2.86 (m, 1H), 2.64 - 2.56 (m, 1H), 2.42 (dd, *J* = 13.2, 4.6 Hz, 1H), 2.23 (s, 3H), 2.05 - 1.98 (m, 1H). LCMS (ESI) calcd for C₃₈H₃₅N₈O₄⁺ [M+H]⁺: 667.27, found, 667.30.

### Example 232: preparation of N-(4-(chlorodifluoromethoxy)phenyl)-6-(4-(((2-(2,6-dioxopiperidin-3-yl)-4-fluoro-1-oxoisoindolin-5-yl)methyl)(methyl)amino)piperidin-1-yl)-5-(1H-pyrazol-5-yl)nicotinamide (GT-04020)

The target compound (GT-04020) was prepared using the methods described in Scheme 11 and Example 1 (white solid, 26 mg, yield 40.09%). ¹H NMR (400 MHz, DMSO) δ 11.14 - 10.76 (m, 2H), 10.56 (s, 1H), 8.81 (d, *J* = 2.3 Hz, 1H), 8.41 (d, *J* = 2.4 Hz, 1H), 8.03 - 7.89 (m, 3H), 7.84 (d, *J* = 2.1 Hz, 1H), 7.69 (d, *J* = 7.7 Hz, 1H), 7.35 (d, *J* = 9.0 Hz, 2H), 6.75 (d, *J* = 1.7 Hz, 1H), 5.15 (dd, *J =* 13.2, 5.0 Hz, 1H), 4.67 - 4.56 (m, 3H), 4.45 (dd, *J* = 17.6, 7.0 Hz, 2H), 3.81 (t, *J* = 10.2 Hz, 2H), 3.49 (dd, *J* = 12.3, 7.2 Hz, 1H), 2.96 - 2.78 (m, 3H), 2.70 - 2.57 (m, 4H), 2.49 - 2.37 (m, 1H), 2.22 (d, *J* = 10.3 Hz, 1H), 2.12 (d, *J* = 10.1 Hz, 1H), 2.01 (dd, *J* = 9.8, 5.2 Hz, 1H), 1.90 (d, *J* = 11.6 Hz, 1H). LCMS (ESI) calcd for C₃₆H₃₅ClF₃N₈O₅⁺ [M+H]⁺: 751.23, found, 751.30.

### Example 233: preparation of N-(4-(chlorodifluoromethoxy)phenyl)-6-(4-(((2-(2,6-dioxopiperidin-3-yl)-6-fluoro-1-oxoisoindolin-5-yl)methyl)(methyl)amino)piperidin-1-yl)-5-(1H-pyrazol-5-yl)nicotinamide (GT-04021)

The target compound (GT-04021) was prepared using the methods described in Scheme 11 and Example 1 (white solid, 25 mg, yield 38.55%). ¹H NMR (400 MHz, DMSO) δ 11.03 (s, 1H), 10.92 (s, 1H), 10.56 (s, 1H), 8.81 (d, *J* = 2.4 Hz, 1H), 8.41 (d, *J* = 2.4 Hz, 1H), 8.05 (d, *J* = 4.8 Hz, 1H), 7.95 - 7.89 (m, 2H), 7.84 (d, *J* = 2.1 Hz, 1H), 7.68 (d, *J* = 8.6 Hz, 1H), 7.35 (d, *J* = 9.1 Hz, 2H), 6.75 (d, *J* = 2.1 Hz, 1H), 5.14 (dd, *J* = 13.3, 5.0 Hz, 1H), 4.64 (d, *J* = 12.8 Hz, 1H), 4.50 (d, *J* = 5.6 Hz, 1H), 4.41-4.32 (m, 2H), 3.81 (s, 2H), 3.48 (d, *J* = 13.5 Hz, 1H), 2.96 - 2.78 (m, 3H), 2.70 - 2.58 (m, 4H), 2.47-2.34 (m, 1H), 2.22 (d, *J* = 10.3 Hz, 1H), 2.12 (d, *J* = 10.6 Hz, 1H), 2.08 - 1.99 (m, 1H), 1.90 (dd, *J* = 22.5, 10.5 Hz, 2H). LCMS (ESI) calcd for C₃₆H₃₅ClF₃N₈O₅⁺ [M+H]⁺: 751.23, found, 751.30.

### Example 234: preparation of N-(4-(chlorodifluoromethoxy)phenyl)-6-(4-(((2-(2,6-dioxopiperidin-3-yl)-7-fluoro-1-oxoisoindolin-5-yl)methyl)(methyl)amino)piperidin-1-yl)-5-(1H-pyrazol-5-yl)nicotinamide (GT-04022)

The target compound (GT-04022) was prepared using the methods described in Scheme 11 and Example 1 (white solid, 31 mg, yield 47.80%). ¹H NMR (500 MHz, DMSO) δ 11.04 (s, 1H), 10.86 (s, 1H), 10.50 (s, 1H), 8.78 (dd, *J* = 9.9, 2.4 Hz, 1H), 8.39 (d, *J* = 2.2 Hz, 1H), 7.91 (d, *J* = 9.1 Hz, 2H), 7.83 (d, *J* = 1.8 Hz, 1H), 7.76 - 7.61 (m, 2H), 7.36 (d, *J* = 8.9 Hz, 2H), 6.73 (d, *J* = 1.5 Hz, 1H), 5.15-5.04 (m, 1H), 4.44 (dddd, *J* = 36.0, 25.5, 14.5, 5.8 Hz, 5H), 3.80 (d, *J* = 9.6 Hz, 2H), 2.97 - 2.88 (m, 1H), 2.80 (q, *J* = 11.9 Hz, 2H), 2.61 (d, *J* = 12.8 Hz, 4H), 2.41 (ddd, *J* = 26.7, 13.4, 4.6 Hz, 1H), 2.15 (dd, *J* = 22.8, 11.1 Hz, 2H), 2.05 - 1.96 (m, 1H), 1.93 - 1.79 (m, 2H). LCMS (ESI) calcd for C₃₆H₃₅ClF₃N₈O₅⁺ [M+H]⁺: 751.23, found, 751.30.

### Example 235: preparation of N-(4-(chlorodifluoromethoxy)phenyl)-6-(4-(((2-(2,6-dioxopiperidin-3-yl)-1-oxoisoindolin-4-yl)methyl)(methyl)amino)piperidin-1-yl)-5-(1H-pyrazol-5-yl)nicotinamide (GT-04023)

The target compound (GT-04023) was prepared using the methods described in Scheme 11 and Example 1 (white solid, 27 mg, yield 42.66%). ¹H NMR (400 MHz, DMSO) δ 11.05 (s, 1H), 10.89 (t, *J* = 34.1 Hz, 1H), 10.54 (s, 1H), 8.81 (d, *J* = 2.4 Hz, 1H), 8.41 (d, *J* = 2.4 Hz, 1H), 7.97 - 7.89 (m, 3H), 7.86 - 7.78 (m, 2H), 7.64 (dt, *J* = 7.6, 3.8 Hz, 1H), 7.35 (d, *J* = 9.1 Hz, 2H), 6.81 - 6.69 (m, 1H), 5.22 - 5.15 (m, 1H), 4.60 (d, *J* = 4.0 Hz, 1H), 4.49 (d, *J* = 17.3 Hz, 1H), 4.22 - 4.17 (m, 2H), 3.82 (d, *J* = 10.7 Hz, 2H), 3.59 - 3.53 (m, 1H), 2.94 (dd, *J* = 21.5, 9.2 Hz, 1H), 2.83 (t, *J* = 13.0 Hz, 2H), 2.64 (dd, *J* = 10.7, 4.7 Hz, 4H), 2.40 - 2.33 (m, 1H), 2.25 - 2.12 (m, 2H), 2.03 - 1.88 (m, 3H). LCMS (ESI) calcd for C₃₆H₃₆ClF₂N₈O₅⁺ [M+H]⁺: 733.24, found, 733.30.

### Example 236: preparation of N-(4-(chlorodifluoromethoxy)phenyl)-6-(4-(((2-(2,6-dioxopiperidin-3-yl)-1,3-dioxoisoindolin-5-yl)methyl)(methyl)amino)piperidin-1-yl)-5-(1H-pyrazol-5-yl)nicotinamide (GT-04025)

The target compound (GT-04025)was prepared using the methods described in Scheme 11 and Example 1 (white solid, 34 mg, yield 52.70%). ¹H NMR (500 MHz, DMSO) δ 11.15 (d, *J* = 18.1 Hz, 2H), 10.73 (s, 1H), 10.49 (s, 1H), 8.84 - 8.76 (m, 1H), 8.38 (dd, *J* = 9.2, 2.4 Hz, 1H), 8.04 (d, *J* = 7.7 Hz, 1H), 7.95 - 7.87 (m, 2H), 7.83 (dd, *J* = 9.4, 4.6 Hz, 2H), 7.77 (s, 1H), 7.70 (d, *J* = 7.6 Hz, 1H), 7.36 (d, *J* = 8.9 Hz, 2H), 6.70 (dd, *J* = 40.2, 2.1 Hz, 1H), 5.14 (dd, *J* = 12.8, 5.4 Hz, 1H), 4.68 (d, *J* = 10.1 Hz, 1H), 4.42 (dd, *J* = 12.8, 7.4 Hz, 1H), 3.81 (d, *J* = 10.5 Hz, 2H), 2.95 - 2.85 (m, 2H), 2.80 (dd, *J* = 19.5, 11.9 Hz, 2H), 2.66 - 2.58 (m, 5H), 2.23 - 1.96 (m, 4H), 1.94 - 1.85 (m, 1H). LCMS (ESI) calcd for C₃₆H₃₄ClF₂N₈O₆⁺ [M+H]⁺: 747.22, found, 747.30.

### Example 237: preparation of N-(4-(chlorodifluoromethoxy)phenyl)-6-(4-(1-((2-(2,6-dioxopiperidin-3-yl)-1-oxoisoindolin-5-yl)methyl)piperidin-4-yl)piperazin-1-yl)-5-(1H-pyrazol-5-yl)nicotinamide (GT-04052)

Referring to the method of Step 2 in Scheme 11 and the method of Example 1, the target compound (GT-04052) was prepared using N-(4-(chlorodifluoromethoxy)phenyl)-6-(4-(piperidin-4-yl)piperazin-1-yl)-5-(1H-pyrazol-5-yl)nicotinamide (CAS NO.: 2699130-63-5) and 3-(5-(bromomethyl)-1-oxoisoindolin-2-yl)piperidine-2,6-dione (GT-03234). The target compound (GT-04052) was obtained as a white solid (37 mg, yield 52.30%). ¹H NMR (400 MHz, DMSO) δ 11.56 (s, 1H), 11.29 (s, 1H), 11.01 (s, 1H), 10.60 (s, 1H), 8.83 (d, *J* = 2.3 Hz, 1H), 8.46 (d, *J* = 2.3 Hz, 1H), 7.95 - 7.90 (m, 2H), 7.87 (s, 1H), 7.82 (dd, *J* = 7.7, 5.8 Hz, 2H), 7.76 (d, *J* = 8.0 Hz, 1H), 7.35 (d, *J* = 8.9 Hz, 2H), 6.82 (d, *J* = 2.1 Hz, 1H), 5.14 (dd, *J* = 13.2, 5.1 Hz, 1H), 4.53 - 4.35 (m, 5H), 3.70 (d, *J* = 12.4 Hz, 2H), 3.53 - 3.45 (m, 4H), 3.36 (t, *J* = 12.3 Hz, 2H), 3.19 - 3.11 (m, 2H), 3.04 - 2.88 (m, 3H), 2.66 - 2.58 (m, 1H), 2.43 (dd, *J* = 13.1, 4.5 Hz, 1H), 2.36 (dd, *J* = 12.0, 6.8 Hz, 2H), 2.24 - 2.13 (m, 2H), 2.04 - 1.98 (m, 1H). LCMS (ESI) calcd for C₃₉H₄₁ClF₂N₉O₅⁺ [M+H]⁺: 788.28, found, 788.30.

### Example 238: preparation of N-(4-(chlorodifluoromethoxy)phenyl)-6-(4-(1-((2-(2,6-dioxopiperidin-3-yl)-4-fluoro-1-oxoisoindolin-5-yl)methyl)piperidin-4-yl)piperazin-1-yl)-5-(1H-pyrazol-5-yl)nicotinamide (GT-04053)

Referring to the method of Scheme 11, the target compound (GT-04053) was prepared using N-(4-(chlorodifluoromethoxy)phenyl)-6-(4-(piperidin-4-yl)piperazin-1-yl)-5-(1H-pyrazol-5-yl)nicotinamide (CAS NO.: 2699130-63-5) and 3-(4-fluoro-5-(hydroxymethyl)-1-oxoisoindolin-2-yl)piperidine-2,6-dione (GT-03445). The target compound (GT-04053) was obtained as a white solid (43 mg, yield 59.42%). ¹H NMR (400 MHz, DMSO) δ 11.63 (s, 1H), 11.03 (s, 1H), 10.59 (s, 1H), 8.83 (d, *J* = 2.3 Hz, 1H), 8.46 (d, *J* = 2.3 Hz, 1H), 7.93 (dd, *J* = 7.4, 5.3 Hz, 3H), 7.82 (d, *J* = 2.0 Hz, 1H), 7.69 (d, *J* = 7.7 Hz, 1H), 7.35 (d, *J* = 8.9 Hz, 2H), 6.82 (d, *J* = 2.1 Hz, 1H), 5.15 (dd, *J* = 13.2, 5.0 Hz, 1H), 4.69 - 4.37 (m, 5H), 3.69 (s, 2H), 3.61 (s, 3H), 3.47 (d, *J* = 10.5 Hz, 2H), 3.35 (d, *J* = 12.4 Hz, 2H), 3.16 - 3.07 (m, 3H), 2.96 - 2.89 (m, 1H), 2.61 (d, *J* = 17.0 Hz, 1H), 2.47 - 2.31 (m, 3H), 2.19 (d, *J* = 11.4 Hz, 2H), 2.04 - 1.97 (m, 1H). LCMS (ESI) calcd for C₃₉H₄₀ClF₃N₉O₅⁺ [M+H]⁺: 806.27, found, 806.30.

### Example 239: preparation of N-(4-(chlorodifluoromethoxy)phenyl)-6-(4-(1-((2-(2,6-dioxopiperidin-3-yl)-6-fluoro-1-oxoisoindolin-5-yl)methyl)piperidin-4-yl)piperazin-1-yl)-5-(1H-pyrazol-5-yl)nicotinamide (GT-04054)

The target compound (GT-04054) was prepared using the methods described in Scheme 11 and Example 1 (white solid, 40 mg, yield 55.27%). ¹H NMR (400 MHz, DMSO) δ 11.60 (s, 1H), 11.02 (s, 1H), 10.59 (s, 1H), 8.83 (d, *J* = 2.2 Hz, 1H), 8.46 (d, *J* = 2.3 Hz, 1H), 8.02 (d, *J =* 6.0 Hz, 1H), 7.97 - 7.85 (m, 2H), 7.82 (d, *J* = 1.9 Hz, 1H), 7.67 (d, *J* = 8.5 Hz, 1H), 7.35 (d, *J* = 8.9 Hz, 2H), 6.82 (d, *J* = 2.1 Hz, 1H), 5.14 (dd, *J* = 13.2, 5.1 Hz, 1H), 4.67 - 4.32 (m, 5H), 3.72 (s, 2H), 3.59 (d, *J* = 10.6 Hz, 2H), 3.46 (d, *J* = 9.1 Hz, 3H), 3.40 - 3.33 (m, 2H), 3.17 - 3.08 (m, 3H), 2.95 - 2.87 (m, 1H), 2.65-2.58 (m, 1H), 2.44 (dd, *J* = 13.2, 4.6 Hz, 1H), 2.35 (d, *J* = 9.1 Hz, 2H), 2.19 (dd, *J* = 23.5, 12.1 Hz, 2H), 2.05 - 1.98 (m, 1H). LCMS (ESI) calcd for C₃₉H₄₀ClF₃N₉O₅⁺ [M+H]⁺: 806.27, found, 806.30.

### Example 240: preparation of N-(4-(chlorodifluoromethoxy)phenyl)-6-(4-(1-((2-(2,6-dioxopiperidin-3-yl)-7-fluoro-1-oxoisoindolin-5-yl)methyl)piperidin-4-yl)piperazin-1-yl)-5-(1H-pyrazol-5-yl)nicotinamide (GT-04055)

Referring to the method of Scheme 11, the target compound (GT-04055) was prepared using N-(4-(chlorodifluoromethoxy)phenyl)-6-(4-(piperidin-4-yl)piperazin-1-yl)-5-(1H-pyrazol-5-yl)nicotinamide (CAS NO.: 2699130-63-5) and 3-(7-fluoro-5-(hydroxymethyl)-1-oxoisoindolin-2-yl)piperidine-2,6-dione (GT-03446). The target compound (GT-04055) was obtained as a white solid (44 mg, yield 60.80%). ¹H NMR (400 MHz, DMSO) δ 11.54 (s, 1H), 11.06 (s, 1H), 10.59 (s, 1H), 8.83 (d, *J* = 2.3 Hz, 1H), 8.46 (d, *J* = 2.3 Hz, 1H), 7.98 (d, *J* = 7.6 Hz, 1H), 7.92 (d, *J* = 9.1 Hz, 2H), 7.83 (d, *J* = 7.8 Hz, 2H), 7.64 (t, *J* = 7.6 Hz, 1H), 7.35 (d, *J* = 8.8 Hz, 2H), 6.82 (d, *J* = 2.1 Hz, 1H), 5.21 - 5.16 (m, 1H), 4.84 (d, *J* = 17.6 Hz, 1H), 4.46 (dd, *J* = 41.0, 14.2 Hz, 4H), 3.71 (d, *J* = 12.8 Hz, 2H), 3.57-3.45 (m, 5H), 3.36 (t, *J* = 12.3 Hz, 2H), 3.16 (d, *J* = 7.0 Hz, 3H), 2.99 - 2.90 (m, 1H), 2.65 (d, *J* = 16.9 Hz, 1H), 2.43 - 2.31 (m, 3H), 2.30 - 2.17 (m, 2H), 2.08 - 2.02 (m, 1H). LCMS (ESI) calcd for C₃₉H₄₀ClF₃N₉O₅⁺ [M+H]⁺: 806.27, found, 806.30.

### Example 241: preparation of N-(4-(chlorodifluoromethoxy)phenyl)-6-(4-(1-((2-(2,6-dioxopiperidin-3-yl)-1-oxoisoindolin-4-yl)methyl)piperidin-4-yl)piperazin-1-yl)-5-(1H-pyrazol-5-yl)nicotinamide (GT-04056)

The target compound (GT-04056) was prepared using the methods described in Scheme 11 and Example 1 (white solid, 44 mg, yield 62.20%). ¹H NMR (400 MHz, DMSO) δ 11.48 (s, 1H), 11.02 (s, 1H), 10.59 (s, 1H), 8.83 (d, *J* = 2.2 Hz, 1H), 8.46 (d, *J* = 2.2 Hz, 1H), 7.92 (d, *J* = 9.1 Hz, 2H), 7.83 (d, *J* = 2.0 Hz, 1H), 7.66 (d, *J* = 12.7 Hz, 2H), 7.35 (d, *J* = 8.9 Hz, 2H), 6.82 (d, *J* = 2.0 Hz, 1H), 5.10 (dd, *J* = 13.2, 5.0 Hz, 1H), 4.58 - 4.35 (m, 5H), 3.70 (s, 2H), 3.50 (d, *J* = 15.1 Hz, 4H), 3.40 - 3.31 (m, 2H), 3.20 - 3.12 (m, 2H), 3.04 - 2.85 (m, 3H), 2.61 (d, *J* = 16.8 Hz, 1H), 2.45 - 2.30 (m, 3H), 2.20 (dd, *J* = 23.2, 11.6 Hz, 2H), 2.05 - 1.93 (m, 1H). LCMS (ESI) calcd for C₃₉H₄₁ClF₂N₉O₅⁺ [M+H]⁺: 788.28, found, 788.30.

### Example 242: preparation of N-(4-(chlorodifluoromethoxy)phenyl)-6-(4-(1-((2-(2,6-dioxopiperidin-3-yl)-1,3-dioxoisoindolin-5-yl)methyl)piperidin-4-yl)piperazin-1-yl)-5-(1H-pyrazol-5-yl)nicotinamide (GT-04057)

Referring to the method of Step 2 in Scheme 11 and the method of Example 1, the target compound (GT-04057) was prepared using N-(4-(chlorodifluoromethoxy)phenyl)-6-(4-(piperidin-4-yl)piperazin-1-yl)-5-(1H-pyrazol-5-yl)nicotinamide (CAS NO.: 2699130-63-5) and 5-(bromomethyl)-2-(2,6-dioxo-piperidin-3-yl)isoindoline-1,3-dione (CAS NO.: 1312023-72-5). The target compound (GT-04057) was obtained as a white solid (44 mg, yield 61.11%). ¹H NMR (400 MHz, DMSO) δ 11.54 (s, 1H), 11.15 (s, 1H), 10.59 (s, 1H), 8.83 (d, *J* = 2.2 Hz, 1H), 8.46 (d, *J* = 2.2 Hz, 1H), 8.24 (s, 1H), 8.13 (d, *J* = 7.8 Hz, 1H), 8.03 (d, *J* = 7.6 Hz, 1H), 7.92 (d, *J* = 9.1 Hz, 2H), 7.82 (d, *J* = 1.9 Hz, 1H), 7.35 (d, *J* = 8.9 Hz, 2H), 6.82 (d, *J* = 2.0 Hz, 1H), 5.19 (dd, *J* = 12.8, 5.4 Hz, 1H), 4.54 (s, 2H), 3.72 (s, 2H), 3.55 - 3.46 (m, 5H), 3.39 - 3.31 (m, 2H), 3.15 (d, *J* = 8.2 Hz, 2H), 3.09 - 2.98 (m, 2H), 2.95-2.86 (m, 1H), 2.66 - 2.53 (m, 2H), 2.35 (d, *J* = 11.2 Hz, 2H), 2.28 - 2.14 (m, 2H), 2.08 (dd, *J* = 9.0, 3.6 Hz, 1H). LCMS (ESI) calcd for C₃₉H₃₉ClF₂N₉O₆⁺ [M+H]⁺: 802.26, found, 802.30.

### Example 243: preparation of N-(4-(4-(((2-(2,6-dioxopiperidin-3-yl)-1-oxoisoindolin-5-yl)methyl)(methyl)amino)piperidin-1-yl)-3-(trifluoromethyl)phenyl)-3-(imidazo [1,2-b]pyridazin-3-ylethynyl)-4-methylbenzamide (GT-04100)

Referring to the method of Step 2 in Scheme 11 and the method of Example 1, the target compound (GT-04100) was prepared using 3-(imidazo[1,2-b]pyridazin-3-ylethynyl)-4-methyl-N-(4-(4-(methylamino)piperidin-1-yl)-3-(trifluoromethyl)phenyl)benzamide (GT-D-111) prepared from Intermediate Example 70 and 3-(5-(bromomethyl)-1-oxoisoindolin-2-yl)piperidine-2,6-dione (GT-03234). The target compound (GT-04100) was obtained as a white solid (27 mg, yield 38.18%). LCMS (ESI) calcd for C₄₃H₄₀F₃N₈O₄⁺ [M+H]⁺: 789.30, found, 789.30.

### Example 244: preparation of N-(4-(4-(((2-(2,6-dioxopiperidin-3-yl)-4-fluoro-1-oxoisoindolin-5-yl)methyl)(methyl)amino)piperidin-1-yl)-3-(trifluoromethyl)phenyl)-3-(imidazo[1,2-b]pyridazin-3-ylethynyl)-4-methylbenzamide (GT-04101)

Referring to the method of Scheme 11, the target compound (GT-04101) was prepared using 3-(imidazo[1,2-b]pyridazin-3-ylethynyl)-4-methyl-N-(4-(4-(methylamino)piperidin-1-yl)-3-(trifluoromethyl)phenyl)benzamide (GT-D-111) prepared from Intermediate Example 70 and 3-(4-fluoro-5-(hydroxymethyl)-1-oxoisoindolin-2-yl)piperidine-2,6-dione (GT-03445). The target compound (GT-04101) was obtained as a white solid (55 mg, yield 76.03%). ¹H NMR (400 MHz, DMSO) δ 11.03 (s, 1H), 10.60 (s, 1H), 8.77 (dd, *J* = 4.4, 1.4 Hz, 1H), 8.33 - 8.26 (m, 2H), 8.21 (dd, *J* = 11.0, 2.0 Hz, 2H), 8.10 (d, *J* = 6.1 Hz, 2H), 7.98 (dd, *J* = 8.0, 1.7 Hz, 1H), 7.70 (d, *J* = 8.4 Hz, 1H), 7.57 (dd, *J* = 13.8, 7.3 Hz, 2H), 7.45 (dd, *J* = 9.2, 4.5 Hz, 1H), 5.16 (dd, *J* = 13.8, 4.9 Hz, 1H), 4.67 (d, *J* = 15.1 Hz, 1H), 4.57 - 4.37 (m, 3H), 3.42 (s, 1H), 3.07 (s, 2H), 2.92 - 2.85 (m, 2H), 2.70 (d, *J* = 18.0 Hz, 3H), 2.62 (d, *J* = 9.1 Hz, 4H), 2.39 (ddd, *J* = 22.4, 12.8, 7.1 Hz, 2H), 2.23 (d, *J* = 9.9 Hz, 1H), 2.03 (dd, *J* = 11.3, 6.0 Hz, 2H), 1.92 (d, *J* = 11.5 Hz, 2H). LCMS (ESI) calcd for C₄₃H₃₉F₄N₈O₄⁺ [M+H]⁺: 807.30, found, 807.30.

### Example 245: preparation of N-(4-(4-(((2-(2,6-dioxopiperidin-3-yl)-6-fluoro-1-oxoisoindolin-5-yl)methyl)(methyl)amino)piperidin-1-yl)-3-(trifluoromethyl)phenyl)-3-(imidazo [1,2-b] pyridazin-3-ylethynyl)-4-methylbenzamide (GT-04102)

The target compound (GT-04102) was prepared using the methods described in Scheme 11 and Example 1 (white solid, 40 mg, yield 55.30%). ¹H NMR (500 MHz, DMSO) δ 11.05 (d, *J* = 7.4 Hz, 1H), 10.73 (s, 1H), 10.60 (s, 1H), 8.76 (d, *J* = 4.3 Hz, 1H), 8.30 (dd, *J* = 9.3, 1.4 Hz, 2H), 8.21 (dd, *J* = 14.2, 2.1 Hz, 2H), 8.09 (dd, *J* = 20.3, 6.4 Hz, 2H), 7.98 (dd, *J* = 8.0, 1.6 Hz, 1H), 7.70 (t, *J* = 12.7 Hz, 1H), 7.58 (dd, *J* = 19.4, 8.6 Hz, 2H), 7.44 (ddd, *J* = 9.2, 4.5, 0.9 Hz, 1H), 5.21 - 5.10 (m, 1H), 4.69 (d, *J* = 13.0 Hz, 1H), 4.51 (dt, *J* = 20.1, 10.2 Hz, 1H), 4.45 - 4.32 (m, 2H), 3.43 (s, 1H), 3.07 (s, 2H), 3.00 - 2.80 (m, 3H), 2.73 (t, *J* = 4.5 Hz, 3H), 2.63 (d, *J* = 11.7 Hz, 4H), 2.47 - 2.38 (m, 1H), 2.35 (dd, *J* = 16.1, 6.4 Hz, 1H), 2.23 (d, *J* = 10.4 Hz, 1H), 2.09 - 2.00 (m, 1H), 1.97 - 1.82 (m, 2H). LCMS (ESI) calcd for C₄₃H₃₉F₄N₈O₄⁺ [M+H]⁺: 807.30, found, 807.30.

### Example 246: preparation of N-(4-(4-(((2-(2,6-dioxopiperidin-3-yl)-7-fluoro-1-oxoisoindolin-5-yl)methyl)(methyl)amino)piperidin-1-yl)-3-(trifluoromethyl)phenyl)-3-(imidazo[1,2-b]pyridazin-3-ylethynyl)-4-methylbenzamide (GT-04103)

Referring to the method of Scheme 11, the target compound (GT-04103) was prepared using 3-(imidazo[1,2-b]pyridazin-3-ylethynyl)-4-methyl-N-(4-(4-(methylamino)piperidin-1-yl)-3-(trifluoromethyl)phenyl)benzamide (GT-D-111) prepared from Intermediate Example 70 and 3-(7-fluoro-5-(hydroxymethyl)-1-oxoisoindolin-2-yl)piperidine-2,6-dione (GT-03446). The target compound (GT-04103) was obtained as a white solid (46 mg, yield 63.59%). ¹H NMR (400 MHz, DMSO) δ 11.24 (s, 1H), 11.03 (s, 1H), 10.59 (s, 1H), 8.76 (dd, *J* = 4.4, 1.4 Hz, 1H), 8.34 - 8.26 (m, 2H), 8.21 (dd, *J* = 12.3, 2.0 Hz, 2H), 8.08 (t, *J* = 10.7 Hz, 1H), 7.98 (dd, *J* = 8.0, 1.7 Hz, 1H), 7.77 (d, *J* = 11.2 Hz, 2H), 7.62 - 7.50 (m, 2H), 7.44 (dt, *J* = 10.7, 5.3 Hz, 1H), 5.11 (dt, *J* = 13.0, 6.5 Hz, 1H), 4.70 - 4.49 (m, 2H), 4.41 (dt, *J* = 18.2, 9.3 Hz, 2H), 3.36 (s, 1H), 3.05 (d, *J* = 8.0 Hz, 2H), 2.98-2.77 (m, 3H), 2.70 - 2.57 (m, 7H), 2.47 - 2.36 (m, 1H), 2.36 - 2.22 (m, 2H), 2.09 - 1.99 (m, 1H), 1.97 - 1.77 (m, 2H). LCMS (ESI) calcd for C₄₃H₃₉F₄N₈O₄⁺ [M+H]⁺: 807.30, found, 807.30.

### Example 247: preparation of N-(4-(4-(((2-(2,6-dioxopiperidin-3-yl)-1-oxoisoindolin-4-yl)methyl)(methyl)amino)piperidin-1-yl)-3-(trifluoromethyl)phenyl)-3-(imidazo [1,2-b]pyridazin-3-ylethynyl)-4-methylbenzamide (GT-04104)

The target compound (GT-04104) was prepared using the methods described in Scheme 11 and Example 1 (white solid, 45 mg, yield 63.63%). ¹H NMR (400 MHz, DMSO) δ 10.98 (d, *J* = 10.0 Hz, 1H), 10.83 (d, *J* = 50.8 Hz, 1H), 10.53 (s, 1H), 8.69 (dd, *J* = 4.4, 1.4 Hz, 1H), 8.23 (dd, *J* = 8.9, 1.8 Hz, 2H), 8.15 (dd, *J* = 10.3, 2.0 Hz, 2H), 8.04 (d, *J* = 8.5 Hz, 1H), 7.99 - 7.86 (m, 2H), 7.79 (t, *J* = 7.6 Hz, 1H), 7.64 - 7.57 (m, 1H), 7.51 (dd, *J* = 18.3, 8.6 Hz, 2H), 7.37 (dd, *J* = 9.2, 4.5 Hz, 1H), 5.18-5.06 (m, 1H), 5.00 - 4.65 (m, 1H), 4.63 - 4.41 (m, 2H), 4.17 (dt, *J* = 18.5, 8.9 Hz, 1H), 3.43 (d, *J* = 10.6 Hz, 1H), 3.03 (d, *J* = 8.1 Hz, 2H), 2.94 - 2.77 (m, 3H), 2.66 - 2.52 (m, 7H), 2.37 - 2.18 (m, 3H), 2.04 - 1.78 (m, 3H). LCMS (ESI) calcd for C₄₃H₄₀F₃N₈O₄⁺ [M+H]⁺: 789.30, found, 789.30.

### Example 248: preparation of N-(4-(4-(((2-(2,6-dioxopiperidin-3-yl)-1,3-dioxoisoindolin-5-yl)methyl)(methyl)amino)piperidin-1-yl)-3-(trifluoromethyl)phenyl)-3-(imidazo [1,2-b]pyridazin-3-ylethynyl)-4-methylbenzamide (GT-04105)

Referring to the method of Step 2 in Scheme 11 and the method of Example 1, the target compound (GT-04105) was prepared using 3-(imidazo[1,2-b]pyridazin-3-ylethynyl)-4-methyl-N-(4-(4-(methylamino)piperidin-1-yl)-3-(trifluoromethyl)phenyl)benzamide (GT-D-111) and 5-(bromomethyl)-2-(2,6-dioxo-piperidin-3-yl)isoindoline-1,3-dione (CAS NO.: 1312023-72-5). The target compound (GT-04105) was obtained as a white solid (57 mg, yield 79.19%). ¹H NMR (500 MHz, DMSO) δ 10.85 (s, 1H), 10.58 (s, 1H), 8.75 (dd, *J* = 4.4, 1.5 Hz, 1H), 8.32 - 8.29 (m, 1H), 8.29 - 8.27 (m, 1H), 8.22 (d, *J* = 1.7 Hz, 1H), 8.19 (d, *J* = 2.3 Hz, 1H), 8.09 (dd, *J* = 9.2, 7.5 Hz, 1H), 8.06 (d, *J* = 7.7 Hz, 1H), 8.03 - 8.00 (m, 1H), 7.99 - 7.93 (m, 3H), 7.64 - 7.52 (m, 2H), 7.43 (dd, *J* = 9.2, 4.4 Hz, 1H), 5.20 - 5.17 (m, 1H), 4.97 (s, 2H), 3.41 (s, 1H), 3.07 (s, 1H), 2.89 (ddd, *J* = 11.7, 6.2, 2.9 Hz, 2H), 2.67 - 2.58 (m, 8H), 2.27 (s, 2H), 2.07 (ddd, *J* = 10.7, 5.4, 2.8 Hz, 3H), 1.90 (dd, *J* = 19.3, 11.4 Hz, 2H). LCMS (ESI) calcd for C₄₃H₃₈F₃N₈O₅⁺ [M+H]⁺: 803.28, found, 803.30.

### Example 249: preparation of 3-(4-fluoro-5-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)-1-oxoisoindolin-2-yl)piperidine-2,6-dione (GT-03900)

Referring to the method of Scheme 11, the target compound (GT-03900) was prepared using 5-fluoro-4-(4-fluoro-1-isopropyl-2-methyl-1H-benzo[d]imidazol-6-yl)-N-(5-(4-(piperazin-1-yl)piperidin-1-yl)pyridin-2-yl)pyrimidin-2-amine (CAS NO.: 1873300-67-4) and 3-(4-fluoro-5-(hydroxymethyl)-1-oxoisoindolin-2-yl)piperidine-2,6-dione (GT-03445). The target compound (GT-03900) was obtained as a white solid (50.00 mg, yield 71.07%). ¹H NMR (400 MHz, DMSO) δ 11.67 (s, 1H), 11.02 (s, 1H), 8.84 (d, *J* = 3.4 Hz, 1H), 8.28 (s, 1H), 8.21 - 8.14 (m, 1H), 7.95 (s, 1H), 7.89-7.74 (m, 3H), 7.67 (d, *J* = 7.7 Hz, 1H), 5.14 (dd, *J* = 13.2, 5.0 Hz, 1H), 4.89 (dd, *J* = 13.8, 6.9 Hz, 1H), 4.59 (d, *J* = 17.6 Hz, 1H), 4.46 (s, 1H), 4.38 - 4.33 (m, 1H), 3.87 (d, *J* = 11.4 Hz, 2H), 3.71 - 3.65 (m, 3H), 3.54 - 3.42 (m, 6H), 2.96 - 2.89 (m, 1H), 2.81 (t, *J* = 11.6 Hz, 2H), 2.72 (s, 3H), 2.61 (dd, *J* = 16.9, 1.0 Hz, 2H), 2.43 (dd, *J* = 9.4, 4.2 Hz, 1H), 2.23 (dd, *J* = 8.1, 5.0 Hz, 2H), 2.05 - 1.98 (m, 1H), 1.92 - 1.82 (m, 2H), 1.64 (d, *J* = 6.9 Hz, 6H). LCMS (ESI) calcd for C₄₃H₄₇F₃N₁₁O₃⁺ [M+H]⁺: 822.37, found, 822.40.

### Example 250: preparation of 3-(6-fluoro-5-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)-1-oxoisoindolin-2-yl)piperidine-2,6-dione (GT-03901)

The target compound (GT-03901) was prepared using the method described in Scheme 11 (white solid, 50.00 mg, yield 71.07%). ¹H NMR (400 MHz, DMSO) δ 11.75 (s, 1H), 11.01 (s, 1H), 8.86 (d, *J* = 3.0 Hz, 1H), 8.30 (s, 1H), 8.21 (d, *J* = 9.9 Hz, 1H), 8.04 - 7.91 (m, 2H), 7.80 (t, *J* = 9.5 Hz, 2H), 7.65 (d, *J* = 8.4 Hz, 1H), 5.14 (dd, *J* = 13.2, 4.7 Hz, 1H), 4.96 - 4.88 (m, 1H), 4.42 (d, *J* = 32.6 Hz, 3H), 3.89 (s, 2H), 3.69 (d, *J* = 6.6 Hz, 3H), 3.57 - 3.45 (m, 6H), 2.96 - 2.88 (m, 1H), 2.82 (t, *J* = 12.5 Hz, 2H), 2.74 (s, 3H), 2.61 (dd, *J* = 17.4, 2.8 Hz, 1H), 2.47 - 2.32 (m, 2H), 2.23 (d, *J* = 11.1 Hz, 2H), 2.06 - 1.99 (m, 1H), 1.91 - 1.79 (m, 2H), 1.64 (d, *J* = 6.8 Hz, 6H). LCMS (ESI) calcd for C₄₃H₄₇F₃N₁₁O₃⁺ [M+H]⁺: 822.37, found, 822.40.

### Example 251: preparation of 3-(7-fluoro-5-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)-1-oxoisoindolin-2-yl)piperidine-2,6-dione (GT-03902)

Referring to the method of Scheme 11, the target compound (GT-03902) was prepared using 5-fluoro-4-(4-fluoro-1-isopropyl-2-methyl-1H-benzo[d]imidazol-6-yl)-N-(5-(4-(piperazin-1-yl)piperidin-1-yl)pyridin-2-yl)pyrimidin-2-amine (CAS NO.: 1873300-67-4) and 3-(7-fluoro-5-(hydroxymethyl)-1-oxoisoindolin-2-yl)piperidine-2,6-dione (GT-03446). The target compound (GT-03902) was obtained as a white solid (50.00 mg, yield 71.07%). ¹H NMR (400 MHz, DMSO) δ 11.70 (s, 1H), 11.01 (s, 1H), 8.85 (d, *J* = 3.4 Hz, 1H), 8.29 (s, 1H), 8.19 (d, *J* = 8.1 Hz, 1H), 7.95 (s, 1H), 7.83 - 7.67 (m, 4H), 5.10 (dd, *J* = 13.2, 5.1 Hz, 1H), 4.91 (dt, *J* = 13.8, 6.8 Hz, 1H), 4.51 - 4.41 (m, 3H), 3.89 (s, 2H), 3.68 (s, 3H), 3.57 - 3.45 (m, 6H), 2.93 - 2.79 (m, 3H), 2.73 (s, 3H), 2.60 (dd, *J* = 16.2, 2.2 Hz, 2H), 2.44 - 2.38 (m, 1H), 2.24 (d, *J* = 10.9 Hz, 2H), 2.04 - 1.98 (m, 1H), 1.87 (ddd, *J* = 20.3, 12.6, 7.4 Hz, 2H), 1.64 (d, *J* = 6.9 Hz, 6H). LCMS (ESI) calcd for C₄₃H₄₇F₃N₁₁O₃⁺ [M+H]⁺: 822.37, found, 822.40.

### Example 252: preparation of 3-(4-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)-1-oxoisoindolin-2-yl)piperidine-2,6-dione (GT-03903)

The target compound (GT-03903) was prepared using the method described in Scheme 11 (white solid, 50.00 mg, yield 72.66%). ¹H NMR (400 MHz, DMSO) δ 11.66 (s, 1H), 11.04 (s, 1H), 8.84 (d, *J* = 3.3 Hz, 1H), 8.28 (s, 1H), 8.18 (dd, *J* = 9.9, 1.7 Hz, 1H), 7.95 (d, *J* = 1.6 Hz, 2H), 7.78 (dd, *J* = 15.0, 10.5 Hz, 3H), 7.61 (t, *J* = 7.5 Hz, 1H), 5.17 (dd, *J* = 13.0, 5.2 Hz, 1H), 4.90 (dt, *J* = 14.0, 6.8 Hz, 2H), 4.51 (d, *J* = 17.6 Hz, 2H), 3.88 (s, 2H), 3.70 - 3.66 (m, 3H), 3.53 - 3.38 (m, 6H), 2.94 (dd, J = 10.8, 6.2 Hz, 1H), 2.80 (d, *J* = 12.0 Hz, 2H), 2.72 (s, 3H), 2.67 - 2.58 (m, 2H), 2.40 - 2.34 (m, 1H), 2.26 - 2.17 (m, 2H), 2.06 - 2.01 (m, 1H), 1.87 (dt, *J* = 12.1, 8.7 Hz, 2H), 1.64 (d, *J* = 6.9 Hz, 6H). LCMS (ESI) calcd for C₄₃H₄₈F₂N₁₁O₃⁺ [M+H]⁺: 804.38, found, 804.40.

### Example 253: preparation of 2-(2,6-dioxopiperidin-3-yl)-5-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)isoindoline-1,3-dione (GT-03904)

Referring to the method of Step 2 in Scheme 11 and the method of Example 1, the target compound (GT-03904) was prepared using 5-fluoro-4-(4-fluoro-1-isopropyl-2-methyl-1H-benzo[d]imidazol-6-yl)-N-(5-(4-(piperazin-1-yl)piperidin-1-yl)pyridin-2-yl)pyrimidin-2-amine (CAS NO.: 1873300-67-4) and 5-(bromomethyl)-2-(2,6-dioxo-piperidin-3-yl)isoindoline-1,3-dione (CAS NO.: 1312023-72-5). The target compound (GT-03904) was obtained as a white solid (50.00 mg, yield 71.42%). ¹H NMR (400 MHz, DMSO) δ 11.68 (s, 1H), 11.14 (s, 1H), 8.84 (d, *J* = 3.4 Hz, 1H), 8.27 (d, *J* = 8.9 Hz, 2H), 8.20 - 8.12 (m, 2H), 8.01 (d, *J* = 7.6 Hz, 1H), 7.95 (d, *J* = 1.8 Hz, 1H), 7.79 (dd, *J* = 20.3, 10.7 Hz, 2H), 5.18 (dd, *J* = 12.9, 5.3 Hz, 1H), 4.91 (dd, *J* = 13.9, 7.0 Hz, 1H), 4.51 (tdd, *J* = 23.8, 15.9, 8.0 Hz, 3H), 3.87 (d, *J* = 11.7 Hz, 2H), 3.72 - 3.66 (m, 3H), 3.56 (ddd, *J* = 13.5, 8.6, 4.1 Hz, 5H), 2.90 - 2.79 (m, 3H), 2.72 (s, 3H), 2.60 (dd, *J* = 21.8, 6.6 Hz, 2H), 2.23 (d, *J* = 10.1 Hz, 2H), 2.11 - 2.04 (m, 1H), 1.92 - 1.83 (m, 2H), 1.64 (d, *J* = 6.9 Hz, 6H). LCMS (ESI) calcd for C₄₃H₄₆F₂N₁₁O₄⁺ [M+H]⁺: 818.36, found, 818.40.

### Example 254: preparation of 2-((S)-4-(4-chlorophenyl)-2,3,9-trimethyl-6H-thieno[3,2-f][1,2,4]triazolo[4,3-a][1,4]diazepin-6-yl)-N-((2-(2,6-dioxopiperidin-3-yl)-1-oxoisoindolin-5-yl)methyl)acetamide (GT-03378)

Referring to the method of Scheme 56, the target compound (GT-03378) was prepared using (S)-2-(4-(4-chlorophenyl)-2,3,9-trimethyl-6H-thieno[3,2-f][1,2,4]triazolo[4,3-a][1,4]diazepin-6-yl)acetic acid (CAS NO.: 202592-23-2) and 3-(5-(aminomethyl)-1-oxoisoindolin-2-yl)piperidine-2,6-dione (CAS NO.: 1010100-28-3). The target compound (GT-03378) was obtained as a white solid (49 mg, yield 58.68%). ¹H NMR (400 MHz, DMSO) δ 10.99 (s, 1H), 8.90 (t, *J* = 5.6 Hz, 1H), 7.67 (dd, *J* = 7.8, 3.0 Hz, 1H), 7.56 (s, 1H), 7.46 (d, *J=* 8.6 Hz, 3H), 7.40 - 7.34 (m, 2H), 5.12 (dd, *J* = 13.1, 5.0 Hz, 1H), 4.58 (td, *J* = 7.1, 2.6 Hz, 1H), 4.46 (d, *J =* 6.1 Hz, 2H), 4.43 - 4.36 (m, 1H), 4.34 - 4.26 (m, 1H), 3.35 (d, *J* = 7.3 Hz, 2H), 2.92 (ddd, *J* = 17.7, 13.7, 5.4 Hz, 1H), 2.62 (s, 3H), 2.62 - 2.54 (m, 1H), 2.41 (s, 3H), 2.39 - 2.30 (m, 1H), 2.03 - 1.94 (m, 1H), 1.61 (d, *J* = 3.3 Hz, 3H). LCMS (ESI) calcd for C₃₃H₃₁ClN₇O₄S⁺ [M+H]⁺: 656.18, found, 656.20.

### Example 255: preparation of 2-((2-((4-(4-(((2-(2,6-dioxopiperidin-3-yl)-4-fluoro-1-oxoisoindolin-5-yl)methyl)(methyl)amino)piperidin-1-yl)-2-methoxyphenyl)amino)-5-(trifluoromethyl)pyridin-4-yl)amino)-N-methylbenzamide (GT-03728)

Referring to the method of Scheme 11, the target compound (GT-03728) was prepared using 2-((2-((2-methoxy-4-(4-(methylamino)piperidin-1-yl)phenyl)amino)-5-(trifluoromethyl)pyridin-4-yl)amino)-N-methylbenzamide (GT-M-172) prepared from Intermediate Example 29 and 3-(4-fluoro-5-(hydroxymethyl)-1-oxoisoindolin-2-yl)piperidine-2,6-dione (GT-03445). The target compound (GT-03728) was obtained as a white solid (51 mg, yield 71.79%). ¹H NMR (400 MHz, DMSO) δ 11.44 (d, *J* = 15.0 Hz, 1H), 11.04 (s, 1H), 10.62 (s, 1H), 10.05 (s, 1H), 8.86 (d, *J* = 4.6 Hz, 1H), 8.18 (s, 1H), 8.03 (t, *J* = 6.3 Hz, 1H), 7.78 (d, *J* = 7.8 Hz, 1H), 7.69 (d, *J* = 7.7 Hz, 1H), 7.56 (d, *J* = 4.0 Hz, 2H), 7.27 (dt, *J* = 13.8, 5.1 Hz, 2H), 6.86 (s, 1H), 6.73 (s, 1H), 6.57 (s, 1H), 5.18 - 5.14 (m, 1H), 4.66 - 4.57 (m, 2H), 4.43 (dd, *J* = 19.6, 10.6 Hz, 2H), 3.93 (d, *J* = 11.8 Hz, 2H), 3.82 (s, 3H), 3.60 - 3.54 (m, 2H), 2.97 - 2.88 (m, 3H), 2.75 (d, *J* = 4.5 Hz, 3H), 2.68 (d, *J* = 1.8 Hz, 3H), 2.61 (d, *J* = 16.1 Hz, 1H), 2.43 - 2.37 (m, 1H), 2.25 (d, *J* = 13.3 Hz, 1H), 2.03 (dd, *J* = 10.6, 5.1 Hz, 3H). LCMS (ESI) calcd for C₄₁H₄₃F₄N₈O₅⁺ [M+H]⁺: 803.32, found, 803.30.

### Example 256: preparation of 3-(5-((4-(4-((4-((3-(methylsulfonyl)benzyl)amino)-5-(trifluoromethyl)pyrimidin-2-yl)amino)phenyl)piperazin-1-yl)methyl)-1-oxoisoindolin-2-yl)piperidine-2,6-dione (GT-03792)

Referring to the method of Step 2 in Scheme 11 and the method of Example 1, the target compound (GT-03792) was prepared using N⁴-(3-(methylsulfonyl)benzyl)-N²-(4-(piperazin-1-yl)phenyl)-5-(trifluoromethyl)pyrimidine-2,4-diamine (GT-M-191) prepared from Intermediate Example 35 and 3-(5-(bromomethyl)-1-oxoisoindolin-2-yl)piperidine-2,6-dione (GT-03234). The target compound (GT-03792) was obtained as a white solid (18 mg, yield 29.29%). ¹H NMR (400 MHz, DMSO) δ 11.55 (s, 1H), 11.02 (s, 1H), 8.36 (s, 1H), 7.92 (d, *J* = 16.2 Hz, 2H), 7.81 (d, *J* = 12.3 Hz, 3H), 7.59 (dd, *J* = 19.6, 12.0 Hz, 2H), 7.28 (d, *J* = 6.1 Hz, 2H), 6.89 (d, *J* = 8.5 Hz, 2H), 5.15 (dd, *J* = 13.2, 4.8 Hz, 1H), 4.71 (d, *J* = 5.0 Hz, 2H), 4.52 (t, *J* = 8.6 Hz, 3H), 4.39 (d, *J* = 17.7 Hz, 1H), 3.39 (d, *J* = 5.2 Hz, 3H), 3.19 (dd, *J* = 22.2, 14.5 Hz, 9H), 2.92 (t, *J* = 8.7 Hz, 1H), 2.61 (d, *J* = 16.8 Hz, 1H), 2.43 (dd, *J* = 13.2, 4.0 Hz, 1H), 2.04 - 1.99 (m, 1H). LCMS (ESI) calcd for C₃₇H₃₈F₃N₈O₅S⁺ [M+H]⁺: 763.26, found, 763.30.

### Example 257: preparation of 3-(4-fluoro-5-((4-(4-((4-((3-(methylsulfonyl)benzyl)amino)-5-(trifluoromethyl)pyrimidin-2-yl)amino)phenyl)piperazin-1-yl)methyl)-1-oxoisoindolin-2-yl)piperidine-2,6-dione (GT-03817)

Referring to the method of Scheme 11, the target compound (GT-03817) was prepared using N⁴-(3-(methylsulfonyl)benzyl)-N²-(4-(piperazin-1-yl)phenyl)-5-(trifluoromethyl)pyrimidine-2,4-diamine (GT-M-191) prepared from Intermediate Example 35 and 3-(4-fluoro-5-(hydroxymethyl)-1-oxoisoindolin-2-yl)piperidine-2,6-dione (GT-03445). The target compound (GT-03817) was obtained as a white solid (26.00 mg, yield 33.74%). ¹H NMR (400 MHz, DMSO) δ 11.66 (s, 1H), 11.04 (s, 1H), 10.68 (s, 1H), 8.96 (s, 1H), 8.44 (s, 1H), 8.06 - 8.00 (m, 1H), 7.90 (s, 1H), 7.81 (d, *J* = 7.4 Hz, 1H), 7.70 (d, *J* = 7.7 Hz, 1H), 7.61 (t, *J* = 7.7 Hz, 1H), 7.56 - 7.40 (m, 1H), 7.27 (d, *J* = 7.9 Hz, 2H), 6.91 (d, *J* = 8.7 Hz, 2H), 5.16 (dd, *J* = 13.2, 5.0 Hz, 1H), 4.72 (d, *J* = 5.4 Hz, 2H), 4.65 - 4.56 (m, 3H), 4.46 (d, *J* = 17.6 Hz, 1H), 3.77 (d, *J* = 7.9 Hz, 3H), 3.51 (s, 2H), 3.30 - 3.17 (m, 4H), 3.15 (s, 3H), 2.97-2.89 (m, 1H), 2.61 (d, *J* = 16.8 Hz, 1H), 2.49 - 2.39 (m, 1H), 2.06 - 1.99 (m, 1H). LCMS (ESI) calcd for C₃₇H₃₇F₄N₈O₅S⁺ [M+H]⁺: 781.25, found, 781.30.

### Example 258: preparation of 3-(6-fluoro-5-((4-(4-((4-((3-(methylsulfonyl)benzyl)amino)-5-(trifluoromethyl)pyrimidin-2-yl)amino)phenyl)piperazin-1-yl)methyl)-1-oxoisoindolin-2-yl)piperidine-2,6-dione (GT-03818)

The target compound (GT-03818) was prepared using the method described in Scheme 11 (white solid, 19.00 mg, yield 24.65%). ¹H NMR (400 MHz, DMSO) δ 11.44 (s, 1H), 11.03 (s, 1H), 10.25 (s, 1H), 8.61 (s, 1H), 8.34 (s, 1H), 8.10 (d, *J* = 6.0 Hz, 1H), 7.90 (s, 1H), 7.80 (d, *J* = 7.2 Hz, 1H), 7.69 (d, *J* = 8.4 Hz, 1H), 7.60 (d, *J* = 7.6 Hz, 1H), 7.57 - 7.47 (m, 1H), 7.30 (d, *J* = 7.3 Hz, 2H), 6.89 (d, *J* = 8.8 Hz, 2H), 5.15 (dd, *J* = 13.2, 5.1 Hz, 1H), 4.71 (d, *J* = 5.4 Hz, 2H), 4.58 - 4.46 (m, 4H), 4.38 (d, *J* = 17.5 Hz, 1H), 3.49 (d, *J* = 7.9 Hz, 4H), 3.24 (d, *J* = 26.6 Hz, 4H), 3.14 (s, 3H), 2.96 - 2.89 (m, 1H), 2.64 - 2.59 (m, 1H), 2.44 (dd, *J* = 13.1, 4.6 Hz, 1H), 2.06 - 2.00 (m, 1H). LCMS (ESI) calcd for C₃₇H₃₇F₄N₈O₅S⁺ [M+H]⁺: 781.25, found, 781.30.

### Example 259: preparation of 3-(7-fluoro-5-((4-(4-((4-((3-(methylsulfonyl)benzyl)amino)-5-(trifluoromethyl)pyrimidin-2-yl)amino)phenyl)piperazin-1-yl)methyl)-1-oxoisoindolin-2-yl)piperidine-2,6-dione (GT-03819)

Referring to the method of Scheme 11, the target compound (GT-03819) was prepared using N⁴-(3-(methylsulfonyl)benzyl)-N²-(4-(piperazin-1-yl)phenyl)-5-(trifluoromethyl)pyrimidine-2,4-diamine (GT-M-191) prepared from Intermediate Example 35 and 3-(7-fluoro-5-(hydroxymethyl)-1-oxoisoindolin-2-yl)piperidine-2,6-dione (GT-03446). The target compound (GT-03819) was obtained as a white solid (15.00 mg, yield 19.46%). ¹H NMR (400 MHz, DMSO) δ 11.62 (s, 1H), 11.03 (s, 1H), 10.21 (s, 1H), 8.60 (s, 1H), 8.34 (s, 1H), 7.90 (s, 1H), 7.80 (d, *J* = 7.3 Hz, 1H), 7.72 (d, *J* = 11.6 Hz, 2H), 7.61 (t, *J* = 7.6 Hz, 1H), 7.58 - 7.45 (m, 1H), 7.30 (d, *J* = 7.5 Hz, 2H), 6.88 (d, *J* = 8.4 Hz, 2H), 5.11 (dd, *J* = 13.2, 5.0 Hz, 1H), 4.71 (d, J = 5.6 Hz, 2H), 4.62 - 4.30 (m, 5H), 3.41 (d, *J* = 6.8 Hz, 3H), 3.27 - 3.07 (m, 8H), 2.96 - 2.87 (m, 1H), 2.67 - 2.58 (m, 1H), 2.44 - 2.33 (m, 1H), 2.01 (dt, *J* = 7.8, 3.2 Hz, 1H). LCMS (ESI) calcd for C₃₇H₃₇F₄N₈O₅S⁺ [M+H]⁺: 781.25, found, 781.30.

### Example 260: preparation of 3-(4-((4-(4-((4-((3-(methylsulfonyl)benzyl)amino)-5-(trifluoromethyl)pyrimidin-2-yl)amino)phenyl)piperazin-1-yl)methyl)-1-oxoisoindolin-2-yl)piperidine-2,6-dione (GT-03820)

The target compound (GT-03820) was prepared using the method described in Scheme 11 (white solid, 22.00 mg, yield 29.22%). ¹H NMR (400 MHz, DMSO) δ 11.51 (s, 1H), 11.07 (s, 1H), 10.33 (s, 1H), 8.68 (s, 1H), 8.36 (s, 1H), 8.06 (d, *J* = 7.6 Hz, 1H), 7.90 (s, 1H), 7.84 (d, *J* = 7.5 Hz, 1H), 7.80 (d, *J* = 7.4 Hz, 1H), 7.67 - 7.59 (m, 2H), 7.58 - 7.49 (m, 1H), 7.29 (d, *J* = 6.3 Hz, 2H), 6.89 (d, *J* = 8.5 Hz, 2H), 5.19 (dd, *J* = 13.1, 5.1 Hz, 1H), 4.94 (d, *J* = 17.6 Hz, 1H), 4.72 (d, *J* = 5.3 Hz, 2H), 4.54 (d, *J* = 17.6 Hz, 1H), 4.46 (s, 2H), 3.76 (d, *J* = 10.1 Hz, 3H), 3.47 (d, *J* = 8.8 Hz, 1H), 3.40 (d, *J* = 10.4 Hz, 1H), 3.31 - 3.20 (m, 4H), 3.15 (s, 3H), 2.99 - 2.91 (m, 1H), 2.68 - 2.61 (m, 1H), 2.34 (td, *J* = 13.4, 8.9 Hz, 1H), 2.08 - 2.01 (m, 1H). LCMS (ESI) calcd for C₃₇H₃₈F₃N₈O₅S⁺ [M+H]⁺: 763.26, found, 763.30.

### Example 261: preparation of 2-(2,6-dioxopiperidin-3-yl)-5-((4-(4-((4-((3-(methylsulfonyl)benzyl)amino)-5-(trifluoromethyl)pyrimidin-2-yl)amino)phenyl)piperazin-1-yl)methyl)isoindoline-1,3-dione (GT-03821)

Referring to the method of Step 2 in Scheme 11 and the method of Example 1, the target compound (GT-03821) was prepared using N⁴-(3-(methylsulfonyl)benzyl)-N²-(4-(piperazin-1-yl)phenyl)-5-(trifluoromethyl)pyrimidine-2,4-diamine (GT-M-191) prepared from Intermediate Example 35 and 5-(bromomethyl)-2-(2,6-dioxo-piperidin-3-yl)isoindoline-1,3-dione (CAS NO.: 1312023-72-5). The target compound (GT-03821) was obtained as a white solid (25.00 mg, yield 32.60%). ¹H NMR (400 MHz, DMSO) δ 11.73 (s, 1H), 11.16 (s, 1H), 10.32 (s, 1H), 8.67 (s, 1H), 8.33 (d, *J* = 21.0 Hz, 2H), 8.18 (d, *J* = 7.9 Hz, 1H), 8.05 (d, *J* = 7.6 Hz, 1H), 7.90 (s, 1H), 7.80 (d, *J* = 7.3 Hz, 1H), 7.64 - 7.51 (m, 2H), 7.29 (d, *J* = 8.1 Hz, 2H), 6.89 (d, *J* = 8.6 Hz, 2H), 5.19 (dd, *J* = 12.8, 5.3 Hz, 1H), 4.71 (d, *J* = 5.4 Hz, 2H), 4.60 (s, 2H), 3.76 (d, *J* = 7.8 Hz, 3H), 3.40 (d, *J* = 8.1 Hz, 2H), 3.21 - 3.12 (m, 7H), 2.95 - 2.86 (m, 1H), 2.66 - 2.54 (m, 2H), 2.11 - 2.05 (m, 1H). LCMS (ESI) calcd for C₃₇H₃₆F₃N₈O₆S⁺ [M+H]⁺: 777.24, found, 777.30.

### Example 262: preparation of 2-((2-((4-(4-((2-(2,6-dioxopiperidin-3-yl)-1-oxoisoindolin-5-yl)methyl)piperazin-1-yl)-2-isopropoxy-5-methylphenyl)amino)-5-(trifluoromethyl)pyridin-4-yl)amino)-N-methylbenzamide (GT-03940)

The target compound (GT-03940) was prepared using the methods described in Scheme 11 and Example 1 (white solid, 30.00 mg, yield 49.32%). ¹H NMR (400 MHz, DMSO) δ 11.70 (s, 1H), 11.01 (s, 1H), 10.56 (s, 1H), 9.66 (s, 1H), 8.78 (d, *J* = 4.6 Hz, 1H), 8.23 (s, 1H), 7.98 (s, 1H), 7.85 (q, *J* = 7.9 Hz, 2H), 7.76 (d, *J* = 6.9 Hz, 1H), 7.60 (d, *J* = 7.8 Hz, 1H), 7.52 (t, *J* = 7.4 Hz, 1H), 7.29 - 7.19 (m, 2H), 6.70 (s, 1H), 6.60 (s, 1H), 5.15 (dd, *J* = 13.2, 5.0 Hz, 1H), 4.60 - 4.50 (m, 4H), 4.39 (d, *J* = 17.6 Hz, 1H), 3.37 (d, *J* = 8.1 Hz, 2H), 3.21 (d, *J* = 11.4 Hz, 5H), 2.92 (dd, *J* = 10.9, 6.8 Hz, 1H), 2.75 (d, *J* = 4.5 Hz, 3H), 2.62 (d, *J* = 16.6 Hz, 2H), 2.44 (dd, *J* = 13.3, 4.6 Hz, 1H), 2.18 (s, 3H), 2.06 - 2.00 (m, 1H), 1.18 (s, 3H), 1.17 (s, 3H). LCMS (ESI) calcd for C₄₂H₄₆F₃N₈O₅⁺ [M+H]⁺: 799.35, found, 799.40.

### Example 263: preparation of 2-((2-((4-(4-((2-(2,6-dioxopiperidin-3-yl)-4-fluoro-1-oxoisoindolin-5-yl)methyl)piperazin-1-yl)-2-isopropoxy-5-methylphenyl)amino)-5-(trifluoromethyl)pyridin-4-yl)amino)-N-methylbenzamide (GT-03941)

The target compound (GT-03941) was prepared using the methods described in Scheme 11 and Example 1 (white solid, 30.00 mg, yield 48.23%). ¹H NMR (400 MHz, DMSO) δ 11.64 (s, 1H), 11.05 (s, 1H), 10.58 (s, 1H), 9.69 (s, 1H), 8.80 (q, *J =* 4.0 Hz, 1H), 8.23 (s, 1H), 8.05 (t, *J* = 6.9 Hz, 1H), 7.76 (d, *J* = 7.4 Hz, 1H), 7.71 (d, *J* = 7.7 Hz, 1H), 7.60 (d, *J* = 7.8 Hz, 1H), 7.52 (t, *J* = 7.6 Hz, 1H), 7.28 - 7.20 (m, 2H), 6.70 (s, 1H), 6.61 (s, 1H), 5.16 (dd, *J* = 13.2, 5.1 Hz, 1H), 4.56 (ddd, *J* = 27.4, 21.7, 16.6 Hz, 5H), 3.30 (dd, *J* = 10.9, 5.2 Hz, 3H), 3.19 (s, 5H), 2.97 - 2.89 (m, 1H), 2.75 (d, *J* = 4.5 Hz, 3H), 2.61 (d, *J* = 16.1 Hz, 1H), 2.44 (dd, *J* = 11.5, 3.5 Hz, 1H), 2.18 (s, 3H), 2.04 - 1.98 (m, 1H), 1.18 (s, 3H), 1.17 (s, 3H). LCMS (ESI) calcd for C₄₂H₄₅F₄N₈O₅⁺ [M+H]⁺: 817.34, found, 817.30.

### Example 264: preparation of 2-((2-((4-(4-((2-(2,6-dioxopiperidin-3-yl)-6-fluoro-1-oxoisoindolin-5-yl)methyl)piperazin-1-yl)-2-isopropoxy-5-methylphenyl)amino)-5-(trifluoromethyl)pyridin-4-yl)amino)-N-methylbenzamide (GT-03942)

The target compound (GT-03942) was prepared using the methods described in Scheme 11 and Example 1 (white solid, 30.00 mg, yield 48.23%). ¹H NMR (400 MHz, DMSO) δ 11.69 (s, 1H), 11.04 (s, 1H), 10.60 (s, 1H), 9.75 (s, 1H), 8.81 (d, *J* = 4.6 Hz, 1H), 8.24 (s, 1H), 8.16 (d, *J* = 6.0 Hz, 1H), 7.76 (d, *J* = 7.4 Hz, 1H), 7.70 (d, *J* = 8.4 Hz, 1H), 7.60 (d, *J* = 8.1 Hz, 1H), 7.53 (t, *J* = 7.5 Hz, 1H), 7.24 (dd, *J* = 14.6, 6.9 Hz, 2H), 6.69 (s, 1H), 6.61 (s, 1H), 5.16 (dd, *J* = 13.2, 5.1 Hz, 1H), 4.58-4.35 (m, 5H), 3.36 - 3.28 (m, 3H), 3.19 (d, *J* = 0.7 Hz, 5H), 2.96 - 2.88 (m, 1H), 2.75 (d, *J* = 4.5 Hz, 3H), 2.61 (dd, *J* = 14.9, 2.0 Hz, 1H), 2.48 - 2.39 (m, 1H), 2.18 (s, 3H), 2.02 (dd, *J* = 11.1, 5.8 Hz, 1H), 1.18 (s, 3H), 1.17 (s, 3H). LCMS (ESI) calcd for C₄₂H₄₅F₄N₈O₅⁺ [M+H]⁺: 817.34, found, 817.30.

### Example 265: preparation of 2-((2-((4-(4-((2-(2,6-dioxopiperidin-3-yl)-7-fluoro-1-oxoisoindolin-5-yl)methyl)piperazin-1-yl)-2-isopropoxy-5-methylphenyl)amino)-5-(trifluoromethyl)pyridin-4-yl)amino)-N-methylbenzamide (GT-03943)

The target compound (GT-03943) was prepared using the methods described in Scheme 11 and Example 1 (white solid, 30.00 mg, yield 48.23%). ¹H NMR (400 MHz, DMSO) δ 11.87 (s, 1H), 11.02 (s, 1H), 10.57 (s, 1H), 9.67 (s, 1H), 8.78 (d, *J* = 4.5 Hz, 1H), 8.23 (s, 1H), 7.77 (dd, *J* = 10.6, 6.2 Hz, 3H), 7.60 (d, *J* = 8.0 Hz, 1H), 7.52 (t, *J* = 7.7 Hz, 1H), 7.29 - 7.18 (m, 2H), 6.71 (s, 1H), 6.60 (s, 1H), 5.11 (dd, *J* = 13.2, 5.1 Hz, 1H), 4.59 - 4.50 (m, 4H), 4.41 (d, *J* = 18.1 Hz, 1H), 3.29 - 3.13 (m, 8H), 2.96 - 2.89 (m, 1H), 2.75 (d, *J* = 4.5 Hz, 3H), 2.61 (d, *J* = 16.4 Hz, 1H), 2.40 (dd, *J* = 13.2, 8.8 Hz, 1H), 2.18 (s, 3H), 2.02 (dd, *J* = 10.7, 5.4 Hz, 1H), 1.19 (s, 3H), 1.17 (s, 3H). LCMS (ESI) calcd for C₄₂H₄₅F₄N₈O₅⁺ [M+H]⁺: 817.34, found, 817.30.

### Example 266: preparation of 2-((2-((4-(4-((2-(2,6-dioxopiperidin-3-yl)-1-oxoisoindolin-4-yl)methyl)piperazin-1-yl)-2-isopropoxy-5-methylphenyl)amino)-5-(trifluoromethyl)pyridin-4-yl)amino)-N-methylbenzamide (GT-03944)

The target compound (GT-03944) was prepared using the methods described in Scheme 11 and Example 1 (white solid, 30.00 mg, yield 49.32%). ¹H NMR (400 MHz, DMSO) δ 11.60 (s, 1H), 11.07 (s, 1H), 10.57 (s, 1H), 9.69 (s, 1H), 8.77 (dd, *J* = 8.2, 3.7 Hz, 1H), 8.23 (s, 1H), 8.12 (d, *J* = 7.6 Hz, 1H), 7.84 (d, *J* = 7.5 Hz, 1H), 7.76 (d, *J* = 7.8 Hz, 1H), 7.65 (t, *J* = 7.6 Hz, 1H), 7.60 (d, *J* = 7.9 Hz, 1H), 7.53 (t, *J* = 7.6 Hz, 1H), 7.25 (dd, *J* = 15.1, 7.2 Hz, 2H), 6.70 (s, 1H), 6.60 (s, 1H), 5.19 (dd, *J* = 13.2, 5.1 Hz, 1H), 4.96 *(d, J=* 17.6 Hz, 1H), 4.60 - 4.43 (m, 4H), 3.32 - 3.13 (m, 8H), 3.00 - 2.92 (m, 1H), 2.75 (d, *J* = 4.5 Hz, 3H), 2.69 - 2.62 (m, 1H), 2.40 - 2.31 (m, 1H), 2.20 (s, 3H), 2.09 - 2.01 (m, 1H), 1.18 (s, 3H), 1.17 (s, 3H). LCMS (ESI) calcd for C₄₂H₄₆F₃N₈O₅⁺ [M+H]⁺: 799.35, found, 799.40.

### Example 267: preparation of 2-((2-((4-(4-((2-(2,6-dioxopiperidin-3-yl)-1,3-dioxoisoindolin-5-yl)methyl)piperazin-1-yl)-2-isopropoxy-5-methylphenyl)amino)-5-(trifluoromethyl)pyridin-4-yl)amino)-N-methylbenzamide (GT-03945)

The target compound (GT-03945) was prepared using the methods described in Scheme 11 and Example 1 (white solid, 30.00 mg, yield 48.47%). ¹H NMR (400 MHz, DMSO) δ 11.88 (s, 1H), 11.15 (s, 1H), 10.56 (s, 1H), 9.63 (s, 1H), 8.78 (q, *J* = 4.2 Hz, 1H), 8.34 (s, 1H), 8.22 (d, *J* = 6.4 Hz, 2H), 8.05 (d, *J* = 7.7 Hz, 1H), 7.76 (d, *J* = 7.0 Hz, 1H), 7.59 (d, *J* = 8.1 Hz, 1H), 7.52 (t, *J* = 7.6 Hz, 1H), 7.31 - 7.17 (m, 2H), 6.70 (s, 1H), 6.60 (s, 1H), 5.19 (dd, *J* = 12.8, 5.3 Hz, 1H), 4.68 - 4.51 (m, 3H), 3.22 (d, *J* = 28.8 Hz, 7H), 2.96 - 2.87 (m, 1H), 2.75 (d, *J* = 4.5 Hz, 3H), 2.66 - 2.52 (m, 3H), 2.18 (s, 3H), 2.08 (dd, *J* = 9.0, 3.6 Hz, 1H), 1.18 (s, 3H), 1.17 (s, 3H). LCMS (ESI) calcd for C₄₂H₄₄F₃N₈O₆⁺ [M+H]⁺: 813.33, found, 813.30.

### Example 268: preparation of N-(1-((2-(2,6-dioxopiperidin-3-yl)-1-oxoisoindolin-5-yl)methyl)piperidin-4-yl)-2-fluoro-5-methoxy-4-((4-((2-methyl-3-oxoisoindolin-4-yl)oxy)-5 - (trifluoromethyl)pyrimidin-2-yl)amino)benzamide (GT-04028)

The target compound (GT-04028) was prepared using the methods described in Scheme 11 and Example 1 (white solid, 27 mg, yield 36.60%). ¹H NMR (400 MHz, DMSO) δ 11.02 (s, 1H), 10.89 (s, 1H), 8.63 (t, *J* = 2.7 Hz, 1H), 8.48 - 8.40 (m, 1H), 8.35 (d, *J* = 9.5 Hz, 1H), 7.87 (d, *J* = 5.4 Hz, 1H), 7.83 (d, *J* = 7.8 Hz, 1H), 7.77 (d, *J* = 7.8 Hz, 1H), 7.65 (t, *J* = 7.8 Hz, 1H), 7.49 (d, *J* = 7.5 Hz, 1H), 7.36 (dd, *J* = 13.6, 4.8 Hz, 1H), 7.25 (d, *J* = 7.9 Hz, 1H), 7.13 (d, *J* = 6.4 Hz, 1H), 5.15 (dd, *J* = 13.2, 5.0 Hz, 1H), 4.54 - 4.36 (m, 8H), 3.95 (dd, *J* = 11.1, 7.3 Hz, 1H), 3.85 (d, *J* = 7.3 Hz, 3H), 3.13 - 3.05 (m, 2H), 2.92 (d, *J* = 4.5 Hz, 3H), 2.64 - 2.59 (m, 1H), 2.45 - 2.38 (m, 1H), 2.05 - 1.89 (m, 6H). LCMS (ESI) calcd for C₄₁H₃₉F₄N₈O₇⁺ [M+H]⁺: 831.28, found, 831.30.

### Example 269: preparation of N-(1-((2-(2,6-dioxopiperidin-3-yl)-4-fluoro-1-oxoisoindolin-5-yl)methyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide (GT-04029)

The target compound (GT-04029) was prepared using the methods described in Scheme 11 and Example 1 (white solid, 43 mg, yield 57.05%). ¹H NMR (400 MHz, DMSO) δ 11.04 (s, 1H), 8.63 (s, 1H), 7.96 (dd, *J* = 14.1, 7.5 Hz, 2H), 7.71 - 7.63 (m, 3H), 7.49 (d, *J* = 7.6 Hz, 1H), 7.36 (dd, *J* = 13.7, 5.7 Hz, 2H), 7.22 (dd, *J* = 16.2, 7.2 Hz, 2H), 7.13 (d, *J* = 6.4 Hz, 1H), 6.96 (dd, *J* = 7.4, 0.7 Hz, 1H), 6.80 (dd, *J* = 8.1, 0.6 Hz, 1H), 5.15 (d, *J* = 8.2 Hz, 1H), 4.59 (s, 1H), 4.41 (d, *J* = 28.7 Hz, 7H), 3.86 (d, *J* = 12.2 Hz, 4H), 3.18 (s, 2H), 2.92 (s, 3H), 2.61 (d, *J* = 14.6 Hz, 1H), 2.43 (dd, *J* = 13.0, 4.5 Hz, 1H), 1.98 (d, *J =* 24.2 Hz, 6H). LCMS (ESI) calcd for C₄₁H₃₈F₅N₈O₇⁺ [M+H]⁺: 849.27, found, 849.30.

### Example 270: preparation of N-(1-((2-(2,6-dioxopiperidin-3-yl)-6-fluoro-1-oxoisoindolin-5-yl)methyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide (GT-04030)

The target compound (GT-04030) was prepared using the methods described in Scheme 11 and Example 1 (white solid, 36 mg, yield 47.76%). ¹H NMR (400 MHz, DMSO) δ 11.03 (s, 1H), 8.63 (s, 1H), 8.40 (dd, *J* = 26.9, 15.0 Hz, 2H), 8.05 (d, *J* = 8.3 Hz, 1H), 7.66 (dd, *J* = 17.0, 8.2 Hz, 2H), 7.49 (d, *J* = 7.5 Hz, 1H), 7.36 (dd, *J* = 13.6, 5.4 Hz, 1H), 7.25 (d, *J* = 7.9 Hz, 1H), 7.15 (t, *J* = 10.2 Hz, 1H), 6.88 (ddd, *J* = 63.8, 7.8, 0.7 Hz, 1H), 5.18 - 5.13 (m, 1H), 4.50 - 4.37 (m, 8H), 3.89 (d, *J* = 2.2 Hz, 1H), 3.85 (s, 3H), 3.44 (s, 1H), 3.21 - 3.18 (m, 1H), 2.92 (d, *J* = 4.3 Hz, 3H), 2.61 (d, *J* = 16.9 Hz, 1H), 2.46 - 2.40 (m, 1H), 1.98 (dd, *J* = 31.3, 8.6 Hz, 6H). LCMS (ESI) calcd for C₄₁H₃₈F₅N₈O₇⁺ [M+H]⁺: 849.27, found, 849.30.

### Example 271: preparation of N-(1-((2-(2,6-dioxopiperidin-3-yl)-7-fluoro-1-oxoisoindolin-5-yl)methyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide (GT-04031)

The target compound (GT-04031) was prepared using the methods described in Scheme 11 and Example 1 (white solid, 30 mg, yield 39.80%). ¹H NMR (400 MHz, DMSO) δ 11.12 (s, 1H), 11.03 (s, 1H), 8.63 (d, *J* = 7.0 Hz, 1H), 8.47 - 8.31 (m, 2H), 7.67 (dt, *J* = 15.7, 6.4 Hz, 4H), 7.49 (d, *J* = 7.6 Hz, 1H), 7.35 (t, *J* = 8.8 Hz, 1H), 7.25 (d, *J* = 7.9 Hz, 1H), 7.13 (d, *J* = 6.4 Hz, 1H), 5.13 - 5.09 (m, 1H), 4.54 - 4.34 (m, 8H), 3.90 (s, 1H), 3.85 (d, *J* = 6.9 Hz, 3H), 3.09 (dd, *J* = 21.3, 11.1 Hz, 2H), 2.92 (s, 3H), 2.61 (d, *J* = 16.5 Hz, 1H), 2.40 (dd, *J* = 13.1, 8.9 Hz, 1H), 1.98 (dd, *J* = 23.6, 8.1 Hz, 6H). LCMS (ESI) calcd for C₄₁H₃₈F₅N₈O₇⁺ [M+H]⁺: 849.27, found, 849.30.

### Example 272: preparation of N-(1-((2-(2,6-dioxopiperidin-3-yl)-1-oxoisoindolin-4-yl)methyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide (GT-04032)

The target compound (GT-04032) was prepared using the methods described in Scheme 11 and Example 1 (white solid, 20 mg, yield 27.11%). ¹H NMR (400 MHz, DMSO) δ 11.07 (s, 1H), 10.90 (s, 1H), 8.63 (s, 1H), 8.42 (dd, *J* = 38.5, 14.5 Hz, 2H), 8.00 (t, *J* = 7.7 Hz, 1H), 7.83 (d, *J* = 7.5 Hz, 1H), 7.64 (d, *J* = 7.7 Hz, 2H), 7.49 (d, *J* = 7.5 Hz, 1H), 7.36 (dd, *J* = 13.6, 5.7 Hz, 1H), 7.24 (d, *J* = 7.9 Hz, 1H), 7.13 (d, *J* = 6.3 Hz, 1H), 5.19 (dd, *J* = 13.1, 5.2 Hz, 1H), 4.85 (dd, *J* = 16.0, 7.7 Hz, 1H), 4.43 (ddd, *J* = 20.1, 18.6, 10.3 Hz, 7H), 3.91 - 3.87 (m, 1H), 3.84 (s, 3H), 3.20 (d, *J* = 12.3 Hz, 2H), 2.92 (s, 3H), 2.68 - 2.63 (m, 1H), 2.35 (dd, *J* = 13.2, 4.4 Hz, 1H), 2.00 (td, *J* = 16.4, 8.2 Hz, 6H). LCMS (ESI) calcd for C₄₁H₃₉F₄N₈O₇⁺ [M+H]⁺: 831.28, found, 831.30.

### Example 273: preparation of N-(1-((2-(2,6-dioxopiperidin-3-yl)-1,3-dioxoisoindolin-5-yl)methyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide (GT-04033)

The target compound (GT-04033) was prepared using the methods described in Scheme 11 and Example 1 (white solid, 29 mg, yield 38.66%). ¹H NMR (400 MHz, DMSO) δ 11.15 (s, 1H), 8.63 (s, 1H), 8.46 (d, *J* = 7.1 Hz, 1H), 8.34 (s, 1H), 8.26 (s, 2H), 8.12 (t, *J* = 6.1 Hz, 1H), 8.03 (d, *J* = 7.6 Hz, 1H), 7.65 (t, *J* = 7.8 Hz, 1H), 7.49 (d, *J* = 7.6 Hz, 1H), 7.35 (dd, *J* = 9.5, 7.5 Hz, 1H), 7.24 (d, *J* = 7.9 Hz, 1H), 7.13 (d, *J* = 6.4 Hz, 1H), 5.21 - 5.17 (m, 1H), 4.46 (dd, *J* = 40.9, 22.0 Hz, 5H), 3.94 (ddd, *J* = 19.3, 11.5, 5.5 Hz, 2H), 3.84 (s, 3H), 3.10 (dd, *J* = 21.3, 10.4 Hz, 2H), 2.92 (s, 3H), 2.59 (dd, *J* = 21.6, 10.8 Hz, 2H), 2.10 - 1.92 (m, 6H). LCMS (ESI) calcd for C₄₁H₃₇F₄N₈O₈⁺ [M+H]⁺: 845.26, found, 845.30.

### Example 274: preparation of 2-((2-((4-(1-((2-(2,6-dioxopiperidin-3-yl)-1-oxoisoindolin-5-yl)methyl)piperidin-4-yl)-2-isopropoxy-5-methylphenyl)amino)-5-(trifluoromethyl)pyridin-4-yl)amino)-N-methylbenzamide (GT-04078)

Referring to the method of Step 2 in Scheme 11 and the method of Example 1, the target compound (GT-04078) was prepared using 2-((2-((2-isopropoxy-5-methyl-4-(piperidin-4-yl)phenyl)amino)-5-(trifluoromethyl)pyridin-4-yl)amino)-N-methylbenzamide (CAS NO.: 2414478-49-0) and 3-(5-(bromomethyl)-1-oxoisoindolin-2-yl)piperidine-2,6-dione (GT-03234). The target compound (GT-04078) was obtained as a white solid (45 mg, yield 72.47%). ¹H NMR (400 MHz, DMSO) δ 11.49 (s, 1H), 11.02 (s, 1H), 10.59 (s, 1H), 9.73 (s, 1H), 8.80 (d, *J* = 4.6 Hz, 1H), 8.29 (d, *J* = 20.4 Hz, 1H), 8.06 - 7.90 (m, 1H), 7.89 - 7.70 (m, 3H), 7.67 - 7.55 (m, 1H), 7.52 (t, *J* = 7.7 Hz, 1H), 7.23 (dd, *J* = 14.8, 7.1 Hz, 2H), 6.92 (s, 1H), 6.66 (d, *J* = 18.5 Hz, 1H), 5.15 (dd, *J* = 13.2, 5.0 Hz, 1H), 4.59 - 4.35 (m, 5H), 3.42 (s, 2H), 3.15 - 3.05 (m, 2H), 2.96 (dt, *J* = 17.2, 12.3 Hz, 2H), 2.75 (t, *J* = 5.6 Hz, 3H), 2.62 (d, *J* = 16.8 Hz, 1H), 2.47 - 2.37 (m, 1H), 2.32 - 2.17 (m, 5H), 2.08 - 1.97 (m, 1H), 1.82 (d, *J* = 12.5 Hz, 2H), 1.21 (dd, *J* = 16.2, 6.0 Hz, 6H). LCMS (ESI) calcd for C₄₃H₄₇F₃N₇O₅⁺ [M+H]⁺: 798.35, found, 798.30.

### Example 275: preparation of 2-((2-((4-(1-((2-(2,6-dioxopiperidin-3-yl)-4-fluoro-1-oxoisoindolin-5-yl)methyl)piperidin-4-yl)-2-isopropoxy-5-methylphenyl)amino)-5-(trifluoromethyl)pyridin-4-yl)amino)-N-methylbenzamide (GT-04079)

Referring to the method of Scheme 11, the target compound (GT-04079) was prepared using 2-((2-((2-isopropoxy-5-methyl-4-(piperidin-4-yl)phenyl)amino)-5-(trifluoromethyl)pyridin-4-yl)amino)-N-methylbenzamide (CAS NO.: 2414478-49-0) and 3-(4-fluoro-5-(hydroxymethyl)-1-oxoisoindolin-2-yl)piperidine-2,6-dione (GT-03445). The target compound (GT-04079) was obtained as a white solid (41 mg, yield 64.58%). ¹H NMR (400 MHz, DMSO) δ 11.50 (s, 1H), 11.02 (d, *J* = 10.2 Hz, 1H), 10.58 (s, 1H), 9.70 (s, 1H), 8.79 (d, *J* = 4.6 Hz, 1H), 8.26 (s, 1H), 8.09 - 7.96 (m, 1H), 7.76 (d, *J* = 7.1 Hz, 1H), 7.70 (d, *J* = 7.7 Hz, 1H), 7.60 (d, *J* = 8.1 Hz, 1H), 7.50 (dd, *J* = 15.3, 7.5 Hz, 1H), 7.23 (dd, *J* = 13.7, 5.7 Hz, 2H), 6.89 (s, 1H), 6.63 (s, 1H), 5.16 (dd, *J =* 13.3, 5.1 Hz, 1H), 4.61 (d, *J* = 17.6 Hz, 1H), 4.52 - 4.42 (m, 4H), 3.49 (s, 2H), 3.14 (d, *J* = 19.6 Hz, 2H), 3.01 - 2.89 (m, 2H), 2.74 (t, *J* = 5.5 Hz, 3H), 2.61 (d, *J* = 17.4 Hz, 1H), 2.47 - 2.35 (m, 1H), 2.23 (s, 5H), 2.06 - 1.99 (m, 1H), 1.82 (d, *J* = 12.9 Hz, 2H), 1.19 (d,J= 6.0 Hz, 6H). LCMS (ESI) calcd for C₄₃H₄₆F₄N₇O₅⁺ [M+H]⁺: 816.34, found, 816.30.

### Example 276: preparation of 2-((2-((4-(1-((2-(2,6-dioxopiperidin-3-yl)-6-fluoro-1-oxoisoindolin-5-yl)methyl)piperidin-4-yl)-2-isopropoxy-5-methylphenyl)amino)-5-(trifluoromethyl)pyridin-4-yl)amino)-N-methylbenzamide (GT-04080)

The target compound (GT-04080) was prepared using the method described in Scheme 11 (white solid, 22 mg, yield 34.65%). ¹H NMR (400 MHz, DMSO) δ 11.43 (s, 1H), 10.96 (s, 1H), 10.51 (s, 1H), 9.50 (d, *J* = 87.1 Hz, 1H), 8.73 (d, *J* = 4.6 Hz, 1H), 8.21 (d, *J* = 17.1 Hz, 1H), 8.07 (d, *J* = 6.1 Hz, 1H), 7.76 - 7.57 (m, 2H), 7.53 (d, *J* = 8.0 Hz, 1H), 7.43 (dd, *J* = 16.2, 8.9 Hz, 1H), 7.23 - 7.08 (m, 2H), 6.83 (s, 1H), 6.58 (d, *J* = 14.6 Hz, 1H), 5.09 (dd, *J* = 13.2, 5.0 Hz, 1H), 4.47 - 4.29 (m, 5H), 3.40 (s, 2H), 3.13 - 3.04 (m, 2H), 2.95 - 2.82 (m, 2H), 2.68 (d, *J* = 4.5 Hz, 3H), 2.55 (d, *J* = 16.8 Hz, 1H), 2.41 - 2.31 (m, 1H), 2.20 - 2.11 (m, 5H), 2.00 - 1.90 (m, 1H), 1.76 (d, *J* = 12.6 Hz, 2H), 1.12 (d, *J* = 6.0 Hz, 6H). LCMS (ESI) calcd for C₄₃H₄₆F₄N₇O₅⁺ [M+H]⁺: 816.34, found, 816.30.

### Example 277: preparation of 2-((2-((4-(1-((2-(2,6-dioxopiperidin-3-yl)-7-fluoro-1-oxoisoindolin-5-yl)methyl)piperidin-4-yl)-2-isopropoxy-5-methylphenyl)amino)-5-(trifluoromethyl)pyridin-4-yl)amino)-N-methylbenzamide (GT-04081)

Referring to the method of Scheme 11, the target compound (GT-04081) was prepared using 2-((2-((2-isopropoxy-5-methyl-4-(piperidin-4-yl)phenyl)amino)-5-(trifluoromethyl)pyridin-4-yl)amino)-N-methylbenzamide (CAS NO.: 2414478-49-0) and 3-(7-fluoro-5-(hydroxymethyl)-1-oxoisoindolin-2-yl)piperidine-2,6-dione (GT-03446). The target compound (GT-04081) was obtained as a white solid (19 mg, yield 29.93%). ¹H NMR (400 MHz, DMSO) δ 11.56 (s, 1H), 11.03 (s, 1H), 10.56 (s, 1H), 9.61 (s, 1H), 8.78 (d, *J* = 4.6 Hz, 1H), 8.28 (d, *J* = 18.9 Hz, 1H), 7.75 (t, *J* = 5.4 Hz, 3H), 7.59 (t, *J* = 9.5 Hz, 1H), 7.52 (t, *J* = 7.7 Hz, 1H), 7.28 - 7.13 (m, 2H), 6.92 (s, 1H), 6.66 (d, *J* = 18.0 Hz, 1H), 5.19 - 5.02 (m, 1H), 4.58 - 4.36 (m, 5H), 3.44 (s, 2H), 3.15 - 3.06 (m, 2H), 3.00 - 2.88 (m, 2H), 2.76 (d, *J* = 4.5 Hz, 3H), 2.61 (d, *J* = 16.6 Hz, 1H), 2.46 - 2.34 (m, 1H), 2.31 - 2.18 (m, 5H), 2.08 -1.94 (m, 1H), 1.80 (t, *J* = 16.4 Hz, 2H), 1.21 (t, *J* = 6.7Hz, 6H). LCMS (ESI) calcd for C₄₃H₄₆F₄N₇O₅⁺ [M+H]⁺: 816.34, found, 816.30.

### Example 278: preparation of 2-((2-((4-(1-((2-(2,6-dioxopiperidin-3-yl)-1-oxoisoindolin-4-yl)methyl)piperidin-4-yl)-2-isopropoxy-5-methylphenyl)amino)-5-(trifluoromethyl)pyridin-4-yl)amino)-N-methylbenzamide (GT-04082)

The target compound (GT-04082) was prepared using the methods described in Scheme 11 and Example 1 (white solid, 46 mg, yield 74.08%). ¹H NMR (400 MHz, DMSO) δ 11.44 (s, 1H), 11.06 (d, *J* = 18.1 Hz, 1H), 10.62 (s, 1H), 9.84 (s, 1H), 8.82 (d, *J* = 4.6 Hz, 1H), 8.30 (d, *J* = 21.0 Hz, 1H), 8.09 (d, *J* = 7.5 Hz, 1H), 7.83 (t, *J* = 9.8 Hz, 1H), 7.77 (d, *J* = 7.0 Hz, 1H), 7.68 - 7.59 (m, 2H), 7.54 (q, *J* = 7.7 Hz, 1H), 7.26 (t, *J* = 7.5 Hz, 1H), 7.20 (s, 1H), 6.95 (d, *J* = 6.3 Hz, 1H), 6.64 (s, 1H), 5.19 (dt, *J* = 14.7, 7.2 Hz, 1H), 5.00 (d, *J* = 17.6 Hz, 1H), 4.63 - 4.34 (m, 4H), 3.50 (d, *J* = 10.8 Hz, 2H), 3.25 - 3.13 (m, 2H), 3.04 - 2.91 (m, 2H), 2.75 (t, *J =* 5.6 Hz, 3H), 2.66 (d, *J =* 17.1 Hz, 1H), 2.42-2.20 (m, 6H), 2.09 - 1.98 (m, 1H), 1.80 (t, *J* = 14.7 Hz, 2H), 1.26 - 1.08 (m, 6H). LCMS (ESI) calcd for C₄₃H₄₇F₃N₇O₅⁺ [M+H]⁺: 798.35, found, 798.30.

### Example 279: preparation of 2-((2-((4-(1-((2-(2,6-dioxopiperidin-3-yl)-1,3-dioxoisoindolin-5-yl)methyl)piperidin-4-yl)-2-isopropoxy-5-methylphenyl)amino)-5-(trifluoromethyl)pyridin-4-yl)amino)-N-methylbenzamide (GT-04083)

Referring to the method of Step 2 in Scheme 11 and the method of Example 1, the target compound (GT-04083) was prepared using 2-((2-((2-isopropoxy-5-methyl-4-(piperidin-4-yl)phenyl)amino)-5-(trifluoromethyl)pyridin-4-yl)amino)-N-methylbenzamide (CAS NO.: 2414478-49-0) and 5-(bromomethyl)-2-(2,6-dioxo-piperidin-3-yl)isoindoline-1,3-dione (CAS NO.: 1312023-72-5). The target compound (GT-04083) was obtained as a white solid (42 mg, yield 66.48%). ¹H NMR (500 MHz, DMSO) δ 11.50 (d, *J* = 41.0 Hz, 1H), 11.16 (d, *J* = 16.8 Hz, 1H), 10.58 (s, 1H), 8.79 (d, J = 4.6 Hz, 1H), 8.31 (s, 1H), 8.25 (s, 1H), 8.18 (dd, *J* = 13.0, 12.1 Hz, 1H), 8.07 (t, J = 10.1 Hz, 1H), 7.76 (dd, *J* = 7.9, 1.1 Hz, 1H), 7.65 - 7.56 (m, 1H), 7.53 (q, *J* = 7.3 Hz, 1H), 7.30 - 7.15 (m, 2H), 6.92 (d, *J* = 15.3 Hz, 1H), 6.66 (d, *J* = 24.2 Hz, 1H), 5.20 (dd, *J* = 12.8, 5.4 Hz, 1H), 4.55 (d, *J* = 4.0 Hz, 2H), 4.48 (dq, *J* = 12.1, 6.0 Hz, 1H), 3.20 (d, *J=* 10.7 Hz, 2H), 3.14 - 3.06 (m, 2H), 3.01 - 2.85 (m, 2H), 2.75 (t, *J* = 6.6 Hz, 3H), 2.62 (d, *J* = 18.3 Hz, 1H), 2.58 - 2.52 (m, 1H), 2.29 - 2.15 (m, 5H), 2.13 - 2.04 (m, 1H), 1.84 (d, *J* = 12.7 Hz, 2H), 1.20 (d, *J* = 6.0 Hz, 6H). LCMS (ESI) calcd for C₄₃H₄₅F₃N₇O₆⁺ [M+H]⁺: 812.33, found, 812.40.

### Example 280: preparation of N-(2-(4-((2-(2,6-dioxopiperidin-3-yl)-4-fluoro-1-oxoisoindolin-5-yl)methyl)piperazin-1-yl)-5-((R)-3-hydroxypyrrolidin-1-yl)oxazolo [4,5-b]pyridin-6-yl)-2-(2-methylpyridin-4-yl)oxazole-4-carboxamide (GT-03841)

The target compound (GT-03841) was prepared using the method described in Scheme 11 (white solid, 11 mg, yield 14.11%). ¹H NMR (400 MHz, DMSO) δ 11.03 (s, 1H), 10.03 (s, 1H), 9.16 (s, 1H), 8.90 (d, *J* = 5.9 Hz, 1H), 8.33 (s, 1H), 8.21 (d, *J* = 5.9 Hz, 1H), 8.00 (t, *J* = 6.8 Hz, 1H), 7.70 (d, *J* = 4.6 Hz, 2H), 5.15 (dd, *J* = 13.2, 4.7 Hz, 1H), 4.64 (d, *J* = 10.0 Hz, 1H), 4.56 (d, *J* = 15.1 Hz, 3H), 4.48 - 4.39 (m, 2H), 4.28 (s, 2H), 3.59 (dd, *J* = 11.8, 5.3 Hz, 3H), 3.48 - 3.40 (m, 3H), 3.26 (d, *J* = 10.2 Hz, 1H), 2.90 (dd, *J* = 12.7, 4.4 Hz, 1H), 2.80 (s, 3H), 2.61 (d, *J* = 16.9 Hz, 1H), 2.43 (dd, *J* = 13.3, 3.9 Hz, 1H), 2.03 - 1.99 (m, 1H), 1.90 (dt, *J* = 14.1, 5.2 Hz, 1H), 1.82 - 1.72 (m, 1H). LCMS (ESI) calcd for C₃₈H₃₈FN₁₀O₇⁺ [M+H]⁺: 765.28, found, 765.30.

### Example 281: preparation of N-(2-(4-((2-(2,6-dioxopiperidin-3-yl)-6-fluoro-1-oxoisoindolin-5-yl)methyl)piperazin-1-yl)-5-((R)-3-hydroxypyrrolidin-1-yl)oxazolo [4,5-b]pyridin-6-yl)-2-(2-methylpyridin-4-yl)oxazole-4-carboxamide (GT-03842)

The target compound (GT-03842) was prepared using the method described in Scheme 11 (white solid, 11 mg, yield 14.11%). ¹H NMR (400 MHz, DMSO) δ 11.03 (s, 1H), 10.02 (s, 1H), 9.15 (s, 1H), 8.89 (d, *J* = 5.9 Hz, 1H), 8.32 (s, 1H), 8.20 (d, *J* = 5.7 Hz, 1H), 8.10 (d, *J* = 5.8 Hz, 1H), 7.72 - 7.66 (m, 2H), 5.15 (dd, *J* = 13.3, 4.9 Hz, 1H), 4.50 (d, *J* = 15.7 Hz, 4H), 4.37 (dd, *J* = 17.9, 6.6 Hz, 2H), 4.28 (s, 2H), 3.60 (dd, *J* = 9.0, 4.7 Hz, 4H), 3.48 - 3.40 (m, 4H), 3.26 (d, *J* = 9.8 Hz, 2H), 2.91 (dd, *J* = 11.1, 6.4 Hz, 1H), 2.80 (s, 3H), 2.61 (d, *J* = 16.7 Hz, 1H), 2.46 - 2.37 (m, 1H), 2.05 - 2.00 (m, 1H), 1.97 - 1.87 (m, 1H), 1.82 - 1.70 (m, 1H). LCMS (ESI) calcd for C₃₈H₃₈FN₁₀O₇⁺ [M+H]⁺: 765.28, found, 765.30.

### Example 282: preparation of N-(2-(4-((2-(2,6-dioxopiperidin-3-yl)-7-fluoro-1-oxoisoindolin-5-yl)methyl)piperazin-1-yl)-5-((R)-3-hydroxypyrrolidin-1-yl)oxazolo [4,5-b]pyridin-6-yl)-2-(2-methylpyridin-4-yl)oxazole-4-carboxamide (GT-03843)

The target compound (GT-03843) was prepared using the method described in Scheme 11 (white solid, 17 mg, yield 21.81%). ¹H NMR (400 MHz, DMSO) δ 11.02 (s, 1H), 10.07 (s, 1H), 9.18 (s, 1H), 8.91 (d, *J* = 6.0 Hz, 1H), 8.37 (s, 1H), 8.25 (d, *J* = 5.7 Hz, 1H), 7.72 (d, *J* = 4.1 Hz, 3H), 5.10 (dd, *J* = 13.1, 5.0 Hz, 1H), 4.53 (d, *J* = 17.2 Hz, 4H), 4.40 (d, *J* = 18.2 Hz, 3H), 4.31 - 4.25 (m, 4H), 3.63 - 3.57 (m, 2H), 3.49 - 3.40 (m, 3H), 3.27 (d, *J =* 10.1 Hz, 2H), 2.95 - 2.87 (m, 1H), 2.82 (s, 3H), 2.60 (d, *J* = 16.2 Hz, 1H), 2.45 - 2.35 (m, 1H), 2.04 - 1.98 (m, 1H), 1.90 (dt, *J* = 12.2, 6.2 Hz, 1H), 1.78 (dd, *J* = 8.0, 4.3 Hz, 1H). LCMS (ESI) calcd for C₃₈H₃₈FN₁₀O₇⁺ [M+H]⁺: 765.28, found, 765.30.

### Example 283: preparation of N-(2-(4-((2-(2,6-dioxopiperidin-3-yl)-1-oxoisoindolin-4-yl)methyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide (GT-03844)

The target compound (GT-03844) was prepared using the methods described in Scheme 11 and Example 1 (white solid, 15 mg, yield 19.71%). ¹H NMR (400 MHz, DMSO) δ 11.07 (s, 1H), 10.09 (s, 1H), 9.19 (d, *J* = 3.9 Hz, 1H), 8.93 (d, *J* = 6.0 Hz, 1H), 8.39 (s, 1H), 8.27 (d, *J* = 5.9 Hz, 1H), 8.06 (t, *J* = 6.7 Hz, 1H), 7.84 (dd, *J* = 7.3, 3.9 Hz, 1H), 7.77 (d, *J* = 34.3 Hz, 1H), 7.65 (t, *J* = 7.7 Hz, 1H), 5.19 (dd, *J* = 13.1, 5.1 Hz, 1H), 4.96 - 4.90 (m, 1H), 4.58 - 4.43 (m, 6H), 3.66 - 3.57 (m, 3H), 3.54-3.33 (m, 7H), 3.28 (d, *J* = 9.9 Hz, 1H), 3.00 - 2.91 (m, 1H), 2.84 (s, 3H), 2.65 (d, *J* = 18.4 Hz, 1H), 2.37 - 2.29 (m, 1H), 2.04 (dd, *J* = 11.1, 5.6 Hz, 1H), 1.91 (ddd, *J* = 12.6, 7.9, 3.8 Hz, 1H), 1.79 (dt, *J* = 11.6, 5.4 Hz, 1H). LCMS (ESI) calcd for C₃₈H₃₉N₁₀O₇⁺ [M+H]⁺: 747.29, found, 747.30.

### Example 284: preparation of N-(2-(4-((2-(2,6-dioxopiperidin-3-yl)-1,3-dioxoisoindolin-5-yl)methyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide (GT-03845)

The target compound (GT-03845) was prepared using the methods described in Scheme 11 and Example 1 (white solid, 53 mg, yield 68.34%). ¹H NMR (400 MHz, DMSO) δ 11.15 (s, 1H), 10.10 (s, 1H), 9.20 (s, 1H), 8.92 (d, *J* = 6.1 Hz, 1H), 8.39 (s, 1H), 8.31 - 8.26 (m, 2H), 8.17 (d, *J* = 7.8 Hz, 1H), 8.02 (d, *J* = 7.6 Hz, 1H), 7.71 (s, 1H), 5.21 - 5.16 (m, 1H), 4.63 (s, 4H), 4.28 (s, 3H), 3.76 (s, 1H), 3.60 (dd, *J* = 12.5, 6.2 Hz, 2H), 3.51 - 3.36 (m, 4H), 3.27 (d, *J* = 10.2 Hz, 2H), 2.97 - 2.88 (m, 1H), 2.84 (s, 3H), 2.64 - 2.52 (m, 2H), 2.11 - 2.04 (m, 1H), 1.90 (dt, *J* = 16.4, 6.2 Hz, 1H), 1.78 (dd, *J* = 7.7, 4.3 Hz, 1H). LCMS (ESI) calcd for C₃₈H₃₇N₁₀O₈⁺ [M+H]⁺: 761.27, found, 761.30.

### Example 285: preparation of 6-(5-cyano-1H-pyrrolo[2,3-b]pyridin-1-yl)-N-(1-((2-(2,6-dioxopiperidin-3-yl)-1-oxoisoindolin-5-yl)methyl)piperidin-4-yl)-4-(isopropylamino)nicotinamide (GT-04036)

Referring to the method of Step 2 in Scheme 11 and the method of Example 1, the target compound (GT-04036) was prepared using 6-(5-cyano-1H-pyrrolo[2,3-b]pyridin-1-yl)-4-(isopropylamino)-N-(piperidin-4-yl)nicotinamide (GT-D-133) prepared from Intermediate Example 74 and 3-(5-(bromomethyl)-1-oxoisoindolin-2-yl)piperidine-2,6-dione (GT-03234). The target compound (GT-04036) was obtained as a white solid (42 mg, yield 54.89%). ¹H NMR (400 MHz, DMSO) δ 11.27 (s, 1H), 11.02 (s, 1H), 8.90 (d, *J* = 6.8 Hz, 2H), 8.81 (dd, *J=* 5.3, 1.9 Hz, 1H), 8.69 (t, *J* = 3.3 Hz, 2H), 8.50 (d, *J* = 3.9 Hz, 1H), 7.95 (t, *J* = 7.2 Hz, 2H), 7.81 (s, 2H), 6.93 (dd, *J* = 7.9, 3.9 Hz, 1H), 5.14 (dd, *J* = 13.1, 5.0 Hz, 1H), 4.53 - 4.36 (m, 4H), 4.00 (dt, *J* = 15.8, 8.2 Hz, 2H), 3.39 (d, *J* = 11.1 Hz, 2H), 3.22 - 3.04 (m, 2H), 2.97 - 2.88 (m, 1H), 2.64 - 2.57 (m, 1H), 2.46 - 2.36 (m, 1H), 2.06 (dd, *J* = 25.8, 8.5 Hz, 5H), 1.29 (t, *J* = 7.4 Hz, 7H). LCMS (ESI) calcd for C₃₆H₃₈N₉O₄⁺ [M+H]⁺: 660.30, found, 660.30.

### Example 286: preparation of 6-(5-cyano-1H-pyrrolo[2,3-b]pyridin-1-yl)-N-(1-((2-(2,6-dioxopiperidin-3-yl)-4-fluoro-1-oxoisoindolin-5-yl)methyl)piperidin-4-yl)-4-(isopropylamino)nicotinamide (GT-04037)

Referring to the method of Scheme 11, the target compound (GT-04037) was prepared using 6-(5-cyano-1H-pyrrolo[2,3-b]pyridin-1-yl)-4-(isopropylamino)-N-(piperidin-4-yl)nicotinamide (GT-D-133) prepared from Intermediate Example 74 and 3-(4-fluoro-5-(hydroxymethyl)-1-oxoisoindolin-2-yl)piperidine-2,6-dione (GT-03445). The target compound (GT-04037) was obtained as a white solid (50 mg, yield 63.62%). ¹H NMR (400 MHz, DMSO) δ 11.34 (s, 1H), 11.04 (s, 1H), 8.91 (d, *J* = 7.0 Hz, 1H), 8.81 (dd, *J* = 4.3, 1.9 Hz, 1H), 8.69 (dd, *J* = 4.9, 2.7 Hz, 2H), 8.50 (d, *J* = 3.9 Hz, 1H), 8.04 (dd, *J* = 15.9, 8.8 Hz, 1H), 7.96 (d, *J* = 6.6 Hz, 1H), 7.69 (d, *J* = 7.7 Hz, 1H), 6.93 (dd, *J* = 6.6, 3.9 Hz, 1H), 5.16 (dd, *J* = 13.2, 5.0 Hz, 1H), 4.63 - 4.41 (m, 4H), 3.85 (d, *J* = 5.0 Hz, 2H), 3.48 (d, *J* = 9.4 Hz, 2H), 3.34 - 3.14 (m, 2H), 2.97 - 2.85 (m, 1H), 2.67 - 2.56 (m, 1H), 2.49 - 2.37 (m, 1H), 2.19 - 1.98 (m, 5H), 1.29 (t, *J* = 7.8 Hz, 7H). LCMS (ESI) calcd for C₃₆H₃₇FN₉O₄⁺ [M+H]⁺: 678.29, found, 678.30.

### Example 287: preparation of 6-(5-cyano-1H-pyrrolo[2,3-b]pyridin-1-yl)-N-(1-((2-(2,6-dioxopiperidin-3-yl)-6-fluoro-1-oxoisoindolin-5-yl)methyl)piperidin-4-yl)-4-(isopropylamino)nicotinamide (GT-04038)

The target compound (GT-04038) was prepared using the method described in Scheme 11 (white solid, 52 mg, yield 66.16%). ¹H NMR (400 MHz, DMSO) δ 11.22 (d, *J* = 32.6 Hz, 1H), 11.03 (s, 1H), 9.03 - 8.70 (m, 3H), 8.68 (d, *J* = 2.0 Hz, 2H), 8.50 (d, *J* = 3.9 Hz, 1H), 8.13 (dd, *J* = 12.7, 6.1 Hz, 1H), 7.98 (d, *J* = 9.1 Hz, 1H), 7.67 (d, *J* = 8.5 Hz, 1H), 6.93 (dd, *J* = 7.5, 3.9 Hz, 1H), 5.15 (dd, J = 13.2, 4.9 Hz, 1H), 4.44 (dt, *J* = 28.7, 17.4 Hz, 4H), 4.03 (dt, *J* = 16.3, 9.1 Hz, 2H), 3.46 (d, *J* = 11.2 Hz, 2H), 3.32 - 3.14 (m, 2H), 2.97 - 2.87 (m, 1H), 2.61 (d, *J* = 17.0 Hz, 1H), 2.48 - 2.38 (m, 1H), 2.21 - 1.99 (m, 5H), 1.28 (d, *J* = 6.3 Hz, 6H). LCMS (ESI) calcd for C₃₆H₃₇FN₉O₄⁺ [M+H]⁺: 678.29, found, 678.30.

### Example 288: preparation of 6-(5-cyano-1H-pyrrolo[2,3-b]pyridin-1-yl)-N-(1-((2-(2,6-dioxopiperidin-3-yl)-7-fluoro-1-oxoisoindolin-5-yl)methyl)piperidin-4-yl)-4-(isopropylamino)nicotinamide (GT-04039)

Referring to the method of Scheme 11, the target compound (GT-04039) was prepared using 6-(5-cyano-1H-pyrrolo[2,3-b]pyridin-1-yl)-4-(isopropylamino)-N-(piperidin-4-yl)nicotinamide (GT-D-133) prepared from Intermediate Example 74 and 3-(7-fluoro-5-(hydroxymethyl)-1-oxoisoindolin-2-yl)piperidine-2,6-dione (GT-03446). The target compound (GT-04039) was obtained as a white solid (49 mg, yield 62.35%). ¹H NMR (400 MHz, DMSO) δ 11.38 (s, 1H), 11.03 (s, 1H), 8.98 - 8.70 (m, 3H), 8.69 (dd, *J* = 4.3, 2.1 Hz, 2H), 8.50 (d, *J* = 3.9 Hz, 1H), 7.99 (d, *J* = 7.7 Hz, 1H), 7.73 (d, *J* = 10.8 Hz, 2H), 6.93 (dd, *J* = 7.5, 3.9 Hz, 1H), 5.11 (dd, *J* = 13.2, 5.0 Hz, 1H), 4.57 - 4.38 (m, 4H), 4.01 (dd, *J=* 16.6, 10.7 Hz, 2H), 3.41 (d, *J* = 11.2 Hz, 2H), 3.26 - 3.04 (m, 2H), 2.97 - 2.86 (m, 1H), 2.65 - 2.55 (m, 1H), 2.46 - 2.33 (m, 1H), 2.09 (dd, *J* = 30.6, 19.2 Hz, 5H), 1.28 (d, *J* = 6.3 Hz, 6H). LCMS (ESI) calcd for C₃₆H₃₇FN₉O₄⁺ [M+H]⁺: 678.29, found, 678.30.

### Example 289: preparation of 6-(5-cyano-1H-pyrrolo[2,3-b]pyridin-1-yl)-N-(1-((2-(2,6-dioxopiperidin-3-yl)-1-oxoisoindolin-4-yl)methyl)piperidin-4-yl)-4-(isopropylamino)nicotinamide (GT-04040)

The target compound (GT-04040) was prepared by referring to the method of Step 2 in Scheme 11 and the method of Example 1 (white solid, 56 mg, yield 73.19%). ¹H NMR (400 MHz, DMSO) δ 11.23 (s, 1H), 11.07 (s, 1H), 8.89 (d, *J* = 7.0 Hz, 1H), 8.81 (dd, *J* = 6.1, 2.0 Hz, 1H), 8.69 (dd, *J* = 5.7, 1.9 Hz, 2H), 8.50 (d, *J* = 3.9 Hz, 1H), 8.06 (d, *J* = 7.6 Hz, 1H), 7.97 (s, 1H), 7.83 (d, *J* = 7.5 Hz, 1H), 7.64 (t, *J* = 7.6 Hz, 1H), 6.93 (dd, *J =* 9.0, 3.9 Hz, 1H), 5.18 (dt, *J* = 12.7, 4.3 Hz, 1H), 5.01 - 4.85 (m, 1H), 4.54 (dd, *J* = 17.5, 7.6 Hz, 1H), 4.40 (d, *J* = 31.8 Hz, 2H), 4.08 - 3.88 (m, 2H), 3.52 - 3.36 (m, 2H), 3.21 (dd, *J* = 21.6, 10.4 Hz, 2H), 3.01 - 2.91 (m, 1H), 2.69 - 2.61 (m, 1H), 2.41-2.31 (m, 1H), 2.06 (dd, *J* = 10.5, 5.2 Hz, 5H), 1.29 (d, *J* = 6.3 Hz, 6H). LCMS (ESI) calcd for C₃₆H₃₈N₉O₄⁺ [M+H]⁺: 660.30, found, 660.30.

### Example 290: preparation of 6-(5-cyano-1H-pyrrolo[2,3-b]pyridin-1-yl)-N-(1-((2-(2,6-dioxopiperidin-3-yl)-1,3-dioxoisoindolin-5-yl)methyl)piperidin-4-yl)-4-(isopropylamino)nicotinamide (GT-04041)

Referring to the method of Step 2 in Scheme 11 and the method of Example 1, the target compound (GT-04041) was prepared using 6-(5-cyano-1H-pyrrolo[2,3-b]pyridin-1-yl)-4-(isopropylamino)-N-(piperidin-4-yl)nicotinamide (GT-D-133) prepared from Intermediate Example 74 and 5-(bromomethyl)-2-(2,6-dioxo-piperidin-3-yl)isoindoline-1,3-dione (CAS NO.: 1312023-72-5). The target compound (GT-04041) was obtained as a white solid (22 mg, yield 28.16%). ¹H NMR (400 MHz, DMSO) δ 11.24 (s, 1H), 11.15 (s, 1H), 8.85 - 8.76 (m, 2H), 8.71 - 8.63 (m, 2H), 8.51 (t, *J* = 4.2 Hz, 1H), 8.28 (s, 1H), 8.21 - 8.11 (m, 1H), 8.07 - 7.98 (m, 2H), 6.92 (dd, *J* = 9.5, 3.9 Hz, 1H), 5.19 (dd, *J* = 12.9, 5.3 Hz, 1H), 4.54 (dd, *J* = 28.0, 4.6 Hz, 2H), 4.12 - 3.92 (m, 1H), 3.81 (d, *J* = 4.6 Hz, 1H), 3.41 (d, *J* = 11.7 Hz, 2H), 3.25 - 3.04 (m, 2H), 2.90 (ddd, *J* = 12.8, 10.5, 6.4 Hz, 1H), 2.67 - 2.52 (m, 2H), 2.14 - 1.96 (m, 5H), 1.28 (d, *J* = 6.3 Hz, 6H). LCMS (ESI) calcd for C₃₆H₃₆N₉O₅⁺ [M+H]⁺: 674.28, found, 674.30.

### Example 291: preparation of 6-(5-cyano-1H-pyrrolo[2,3-b]pyridin-1-yl)-N-((1r,4r)-4-(((2-(2,6-dioxopiperidin-3-yl)-1-oxoisoindolin-5-yl)methyl)carbamoyl)cyclohexyl)-4-(isopropylamino)nicotinamide (GT-04077)

Referring to the method of Scheme 56, the target compound (GT-04077) was prepared using (1r,4r)-4-(6-(5-cyano-1H-pyrrolo[2,3-b]pyridin-1-yl)-4-(isopropylamino)nicotinamido)cyclohexane-1-carboxylic acid (GT-D-132) prepared from Intermediate Example 73 and 3-(5-(aminomethyl)-1-oxoisoindolin-2-yl)piperidine-2,6-dione (CAS NO.: 1010100-28-3). The target compound (GT-04077) was obtained as a white solid (97 mg, yield 60.48%). ¹H NMR (500 MHz, DMSO) δ 11.00 (s, 1H), 8.91 (s, 1H), 8.83 (d, *J* = 2.0 Hz, 1H), 8.70 (d, *J* = 2.0 Hz, 1H), 8.64 (d, *J* = 2.1 Hz, 1H), 8.58 - 8.43 (m, 3H), 7.97 (d, *J* = 3.6 Hz, 1H), 7.68 (t, *J* = 11.1 Hz, 1H), 7.45 (d, *J* = 11.4 Hz, 1H), 7.39 (d, *J* = 7.9 Hz, 1H), 6.94 (d, *J* = 3.8 Hz, 1H), 5.12 (dd, *J* = 13.3, 5.1 Hz, 1H), 4.38 (dt, *J* = 40.4, 17.4 Hz, 4H), 3.78 - 3.70 (m, 2H), 2.92 (ddd, J = 17.4, 13.7, 5.4 Hz, 1H), 2.64 - 2.57 (m, 1H), 2.44 - 2.33 (m, 1H), 2.20 (td, *J* = 11.8, 3.3 Hz, 1H), 2.01 (ddd, *J* = 10.3, 5.2, 3.1 Hz, 1H), 1.96 - 1.78 (m, 4H), 1.50 (dd, *J* = 25.4, 10.6 Hz, 2H), 1.39 (dd, *J* = 22.2, 12.1 Hz, 2H), 1.30 (d, *J* = 6.3 Hz, 6H). LCMS (ESI) calcd for C₃₈H₄₀N₉O₅⁺ [M+H]⁺: 702.31, found, 702.30.

### Example 292: preparation of 2-(2-((cyclopropylmethyl)amino)pyridin-4-yl)-N-(3-(difluoromethyl)-1-(1-((2-(2,6-dioxopiperidin-3-yl)-1-oxoisoindolin-5-yl)methyl)piperidin-4-yl)-1H-pyrazol-4-yl)oxazole-4-carboxamide (GT-04153)

Referring to the method of Step 2 in Scheme 11 and the method of Example 1, the target compound (GT-04153) was prepared using 2-(2-((cyclopropylmethyl)amino)pyridin-4-yl)-N-(3-(difluoromethyl)-1-(piperidin-4-yl)-1H-pyrazol-4-yl)oxazol-4-carboxamide (CAS NO.: 2434842-21-2) and 3-(5-(bromomethyl)-1-oxoisoindolin-2-yl)piperidine-2,6-dione (GT-03234). The target compound (GT-04153) was obtained as a white solid (43 mg, yield 54.87%). ¹H NMR (400 MHz, DMSO) δ 11.52 (d, *J* = 62.1 Hz, 1H), 11.01 (s, 1H), 10.01 (d, *J* = 13.6 Hz, 1H), 9.18 - 9.08 (m, 1H), 8.46 - 8.13 (m, 1H), 8.08 (d, *J=* 6.4 Hz, 1H), 8.00 - 7.89 (m, 1H), 7.87 - 7.76 (m, 2H), 7.66 (d, *J* = 16.6 Hz, 1H), 7.40 - 7.05 (m, 2H), 5.15 (dd, *J* = 13.2, 5.0 Hz, 1H), 4.61 - 4.40 (m, 4H), 3.36 (d, *J* = 6.7 Hz, 4H), 3.17 (t, *J* = 45.5 Hz, 3H), 2.98 - 2.86 (m, 1H), 2.68 - 2.55 (m, 1H), 2.50 - 2.23 (m, 5H), 2.04 (dd, *J* = 19.6, 14.2 Hz, 1H), 1.15 (ddd, *J* = 12.3, 7.4, 5.0 Hz, 1H), 0.61 - 0.45 (m, 2H), 0.40 - 0.25 (m, 2H). LCMS (ESI) calcd for C₃₆H₃₈F₂N₉O₅⁺ [M+H]⁺: 714.29, found, 714.30.

### Example 293: preparation of 2-(2-((cyclopropylmethyl)amino)pyridin-4-yl)-N-(3-(difluoromethyl)-1-(1-((2-(2,6-dioxopiperidin-3-yl)-4-fluoro-1-oxoisoindolin-5-yl)methyl)piperidin-4-yl)-1H-pyrazol-4-yl)oxazole-4-carboxamide (GT-04154)

Referring to the method of Scheme 11, the target compound (GT-04154) was prepared using 2-(2-((cyclopropylmethyl)amino)pyridin-4-yl)-N-(3-(difluoromethyl)-1-(piperidin-4-yl)-1H-pyrazol-4-yl)oxazol-4-carboxamide (CAS NO.: 2434842-21-2) and 3-(4-fluoro-5-(hydroxymethyl)-1-oxoisoindolin-2-yl)piperidine-2,6-dione (GT-03445). The target compound (GT-04154) was obtained as a white solid (47 mg, yield 58.50%). ¹H NMR (400 MHz, DMSO) δ 11.48 (s, 1H), 11.02 (d, *J* = 14.0 Hz, 1H), 10.01 (s, 1H), 9.34 (s, 1H), 9.14 (d, *J* = 6.1 Hz, 1H), 8.17 (d, *J* = 16.1 Hz, 1H), 8.12 - 7.95 (m, 2H), 7.76 - 7.64 (m, 2H), 7.39 - 7.03 (m, 2H), 5.15 (dt, *J* = 18.5, 9.2 Hz, 1H), 4.64 - 4.44 (m, 4H), 3.59 (s, 2H), 3.37 - 3.08 (m, 5H), 3.01 - 2.85 (m, 1H), 2.62 (d, *J* = 16.9 Hz, 1H), 2.38 (ddd, *J* = 53.9, 23.4, 10.5 Hz, 5H), 2.04 (dd, *J* = 16.0, 10.5 Hz, 1H), 1.23 - 1.07 (m, 1H), 0.64 - 0.47 (m, 2H), 0.39-0.28 (m, 2H). LCMS (ESI) calcd for C₃₆H₃₇F₃N₉O₅⁺ [M+H]⁺: 732.28, found, 732.30.

### Example 294: preparation of 2-(2-((cyclopropylmethyl)amino)pyridin-4-yl)-N-(3-(difluoromethyl)-1-(1-((2-(2,6-dioxopiperidin-3-yl)-6-fluoro-1-oxoisoindolin-5-yl)methyl)piperidin-4-yl)-1H-pyrazol-4-yl)oxazole-4-carboxamide (GT-04171)

The target compound (GT-04171) was prepared using the method described in Scheme 11 (white solid, 49 mg, yield 60.99%). ¹H NMR (400 MHz, DMSO) δ 11.51 (s, 1H), 11.02 (d, *J* = 10.7 Hz, 1H), 10.01 (s, 1H), 9.41 (s, 1H), 9.14 (s, 1H), 8.30 - 8.14 (m, 1H), 8.09 (dd, *J* = 15.6, 6.3 Hz, 2H), 7.77 - 7.57 (m, 2H), 7.43 - 7.04 (m, 2H), 5.21 - 5.10 (m, 1H), 4.56 - 4.31 (m, 4H), 3.58 (d, *J* = 5.3 Hz, 2H), 3.31 (d, *J* = 46.5 Hz, 5H), 2.97 - 2.89 (m, 1H), 2.62 (d, *J* = 16.8 Hz, 1H), 2.45 (dd, *J* = 15.9, 7.7 Hz, 3H), 2.32 - 2.19 (m, 2H), 2.09 - 1.96 (m, 1H), 1.22 - 1.06 (m, 1H), 0.61 - 0.48 (m, 2H), 0.36 (dq, *J* = 9.8, 4.9 Hz, 2H). LCMS (ESI) calcd for C₃₆H₃₇F₃N₉O₅⁺ [M+H]⁺: 732.28, found, 732.30.

### Example 295: preparation of 2-(2-((cyclopropylmethyl)amino)pyridin-4-yl)-N-(3-(difluoromethyl)-1-(1-((2-(2,6-dioxopiperidin-3-yl)-7-fluoro-1-oxoisoindolin-5-yl)methyl)piperidin-4-yl)-1H-pyrazol-4-yl)oxazole-4-carboxamide (GT-04172)

Referring to the method of Scheme 11, the target compound (GT-04172) was prepared using 2-(2-((cyclopropylmethyl)amino)pyridin-4-yl)-N-(3-(difluoromethyl)-1-(piperidin-4-yl)-1H-pyrazol-4-yl)oxazol-4-carboxamide (CAS NO.: 2434842-21-2) and 3-(7-fluoro-5-(hydroxymethyl)-1-oxoisoindolin-2-yl)piperidine-2,6-dione (GT-03446). The target compound (GT-04172) was obtained as a white solid (53 mg, yield 65.96%). ¹H NMR (400 MHz, DMSO) δ 11.69 (d, *J* = 61.5 Hz, 1H), 11.01 (d, *J* = 10.3 Hz, 1H), 10.02 (d*, J* = 19.1 Hz, 1H), 9.33 (s, 1H), 9.14 (d*, J* = 6.1 Hz, 1H), 8.45-8.13 (m, 1H), 8.03 (dd, *J* = 39.5, 12.6 Hz, 1H), 7.73 (dd, *J* = 35.1, 24.9 Hz, 3H), 7.43 - 7.04 (m, 2H), 5.10 (dt, *J* = 15.4, 7.7 Hz, 1H), 4.50 (dd, *J* = 34.9, 17.3 Hz, 4H), 3.37 - 3.10 (m, 7H), 2.90 (t, *J* = 8.5 Hz, 1H), 2.61 (d, *J* = 16.8 Hz, 1H), 2.40 (dd, *J* = 28.2, 16.1 Hz, 3H), 2.26 (d, *J* = 11.6 Hz, 2H), 2.06-1.95 (m, 1H), 1.15 (dt, *J* = 7.4, 5.1 Hz, 1H), 0.62 - 0.48 (m, 2H), 0.39 - 0.26 (m, 2H). LCMS (ESI) calcd for C₃₆H₃₇F₃N₉O₅⁺ [M+H]⁺: 732.28, found, 732.30.

### Example 296: preparation of 2-(2-((cyclopropylmethyl)amino)pyridin-4-yl)-N-(3-(difluoromethyl)-1-(1-((2-(2,6-dioxopiperidin-3-yl)-1-oxoisoindolin-4-yl)methyl)piperidin-4-yl)-1H-pyrazol-4-yl)oxazole-4-carboxamide (GT-04173)

The target compound (GT-04173) was prepared using the method described in Scheme 11 (white solid, 52 mg, yield 66.35%). ¹H NMR (400 MHz, DMSO) δ 11.30 (s, 1H), 10.97 (d, *J* = 22.7 Hz, 1H), 9.92 (s, 1H), 9.17 (s, 1H), 9.11 - 9.03 (m, 1H), 8.16 (d, *J* = 12.6 Hz, 1H), 8.05 - 7.93 (m, 2H), 7.77 (d, *J* = 7.5 Hz, 1H), 7.57 (q, *J* = 7.8 Hz, 2H), 7.32 - 7.02 (m, 2H), 5.17 - 5.06 (m, 1H), 4.83 (d, *J* = 17.6 Hz, 1H), 4.52 (d, *J* = 22.2 Hz, 1H), 4.41 - 4.26 (m, 2H), 3.30 - 3.16 (m, 7H), 2.86 (dd, *J* = 16.6, 11.7 Hz, 1H), 2.59 (d, *J* = 17.4 Hz, 1H), 2.31 (ddd, *J* = 21.3, 19.4, 10.7 Hz, 3H), 2.24 - 2.10 (m, 2H), 2.00 (dd, *J* = 10.9, 5.1 Hz, 1H), 1.14 - 1.02 (m, 1H), 0.48 (q, *J* = 5.4 Hz, 2H), 0.26 (q, *J* = 4.8 Hz, 2H). LCMS (ESI) calcd for C₃₆H₃₈F₂N₉O₅⁺ [M+H]⁺: 714.29, found, 714.30.

### Example 297: preparation of 2-(2-((cyclopropylmethyl)amino)pyridin-4-yl)-N-(3-(difluoromethyl)-1-(1-((2-(2,6-dioxopiperidin-3-yl)-1,3-dioxoisoindolin-5-yl)methyl)piperidin-4-yl)-1H-pyrazol-4-yl)oxazole-4-carboxamide (GT-04174)

Referring to the method of Step 2 in Scheme 11 and the method of Example 1, the target compound (GT-04174) was prepared using 2-(2-((cyclopropylmethyl)amino)pyridin-4-yl)-N-(3-(difluoromethyl)-1-(piperidin-4-yl)-1H-pyrazol-4-yl)oxazol-4-carboxamide (CAS NO.: 2434842-21-2) and 5-(bromomethyl)-2-(2,6-dioxo-piperidin-3-yl)isoindoline-1,3-dione (CAS NO.: 1312023-72-5). The target compound (GT-04174) was obtained as a white solid (56 mg, yield 70.08%). ¹H NMR (400 MHz, DMSO) δ 11.49 (s, 1H), 11.14 (d, *J* = 12.9 Hz, 1H), 9.97 (d, *J* = 13.0 Hz, 1H), 9.10 (s, 1H), 8.32 (d, *J* = 18.8 Hz, 1H), 8.24 - 8.14 (m, 2H), 8.07 (dd, *J* = 17.4, 7.0 Hz, 2H), 7.54 (s, 1H), 7.23 (dd, *J* = 57.2, 51.3 Hz, 2H), 5.20 (dd, *J* = 12.9, 5.3 Hz, 1H), 4.57 (s, 2H), 3.35 - 3.04 (m, 7H), 2.99 - 2.84 (m, 1H), 2.60 (dt, *J* = 11.0, 9.8 Hz, 2H), 2.47 - 2.19 (m, 4H), 2.14 - 2.01 (m, 1H), 1.13 (dd, *J* = 12.2, 7.4 Hz, 1H), 0.54 (q, *J* = 5.6 Hz, 2H), 0.32 (q, *J* = 4.8 Hz, 2H). LCMS (ESI) calcd for C₃₆H₃₆F₂N₉O₆⁺ [M+H]⁺: 728.27, found, 728.30.

### Example 298: preparation of N-(3-(difluoromethyl)-1-(4-(4-((2-(2,6-dioxopiperidin-3-yl)-1-oxoisoindolin-5-yl)methyl)piperazin-1-yl)phenyl)-1H-pyrazol-4-yl)-2-(2-((2,2,2-trifluoroethyl)amino)pyridin-4-yl)oxazole-4-carboxamide (GT-04267)

The target compound (GT-04267) was prepared using the methods described in Scheme 11 and Example 1 (white solid, 52 mg, yield 82.49%). ¹H NMR (400 MHz, DMSO) δ 11.09 - 10.84 (m, 2H), 9.93 (s, 1H), 8.94 (s, 1H), 8.63 (s, 1H), 8.18 (d, *J* = 5.4 Hz, 1H), 7.85 - 7.76 (m, 2H), 7.69 (dd, *J* = 17.6, 8.7 Hz, 4H), 7.60 - 7.34 (m, 1H), 7.22 (d, *J* = 5.1 Hz, 1H), 7.16 (dd, *J* = 5.4, 1.4 Hz, 1H), 7.06 (d, *J* = 9.4 Hz, 2H), 5.09 (dd, *J* = 13.3, 5.1 Hz, 1H), 4.50 - 4.19 (m, 6H), 3.83 (d, *J* = 8.1 Hz, 2H), 3.16 (s, 6H), 2.85 (dd, *J* = 17.2, 5.8 Hz, 1H), 2.57 (dd, *J* = 21.4, 7.6 Hz, 1H), 2.35 (dd, *J* = 15.0, 10.7 Hz, 1H), 2.00 - 1.91 (m, 1H). LCMS (ESI) calcd for C₃₉H₃₆F₅N₁₀O₅⁺ [M+H]⁺: 819.27, found, 819.30.

### Example 299: preparation of N-(3-(difluoromethyl)-1-(4-(4-((2-(2,6-dioxopiperidin-3-yl)-4-fluoro-1-oxoisoindolin-5-yl)methyl)piperazin-1-yl)phenyl)-1H-pyrazol-4-yl)-2-(2-((2,2,2-trifluoroethyl)amino)pyridin-4-yl)oxazole-4-carboxamide (GT-04268)

The target compound (GT-04268) was prepared using the method described in Scheme 11 (white solid, 50 mg, yield 77.61%). ¹H NMR (400 MHz, DMSO) δ 10.96 (d, *J* = 9.5 Hz, 2H), 9.94 (s, 1H), 8.94 (s, 1H), 8.63 (s, 1H), 8.18 (d, *J* = 5.3 Hz, 1H), 7.95 - 7.82 (m, 1H), 7.76 - 7.62 (m, 4H), 7.57 - 7.20 (m, 2H), 7.16 (dd, *J* = 5.4, 1.4 Hz, 1H), 7.06 (d, *J* = 9.2 Hz, 2H), 5.08 (dt, *J* = 15.7, 7.8 Hz, 1H), 4.57 - 4.33 (m, 4H), 4.25 - 4.15 (m, 2H), 3.83 (s, 2H), 3.35 - 3.01 (m, 6H), 2.91 - 2.81 (m, 1H), 2.54 (d, *J* = 15.8 Hz, 1H), 2.37 (dd, *J* = 13.5, 4.6 Hz, 1H), 1.99 - 1.90 (m, 1H). LCMS (ESI) calcd for C₃₉H₃₅F₆N₁₀O₅⁺ [M+H]⁺: 837.26, found, 837.30.

### Example 300: preparation of N-(3-(difluoromethyl)-1-(4-(4-((2-(2,6-dioxopiperidin-3-yl)-6-fluoro-1-oxoisoindolin-5-yl)methyl)piperazin-1-yl)phenyl)-1H-pyrazol-4-yl)-2-(2-((2,2,2-trifluoroethyl)amino)pyridin-4-yl)oxazole-4-carboxamide (GT-04269)

The target compound (GT-04269) was prepared using the method described in Scheme 11 (white solid, 47 mg, yield 72.95%). ¹H NMR (400 MHz, DMSO) δ 11.01 - 10.73 (m, 2H), 9.92 (s, 1H), 8.94 (s, 1H), 8.63 (s, 1H), 8.18 (d, *J* = 5.3 Hz, 1H), 7.95 (d, *J* = 6.0 Hz, 1H), 7.66 (t, *J* = 8.2 Hz, 4H), 7.16 (ddd, *J* = 54.9, 36.6, 31.6 Hz, 5H), 5.08 (dt, *J* = 14.8, 7.3 Hz, 1H), 4.54 - 4.27 (m, 4H), 4.24 - 4.13 (m, 2H), 3.83 (s, 2H), 3.48 (d, *J* = 50.4 Hz, 3H), 3.09 (d, *J* = 22.3 Hz, 3H), 2.92 - 2.81 (m, 1H), 2.58 (dd, *J* = 19.7, 9.3 Hz, 1H), 2.39 - 2.27 (m, 1H), 2.01 - 1.92 (m, 1H). LCMS (ESI) calcd for C₃₉H₃₅F₆N₁₀O₅⁺ [M+H]⁺: 837.26, found, 837.30.

### Example 301: preparation of N-(3-(difluoromethyl)-1-(4-(4-((2-(2,6-dioxopiperidin-3-yl)-7-fluoro-1-oxoisoindolin-5-yl)methyl)piperazin-1-yl)phenyl)-1H-pyrazol-4-yl)-2-(2-((2,2,2-trifluoroethyl)amino)pyridin-4-yl)oxazole-4-carboxamide (GT-04270)

The target compound (GT-04270) was prepared using the method described in Scheme 11 (white solid, 53 mg, yield 82.26%). ¹H NMR (400 MHz, DMSO) δ 11.19 - 10.98 (m, 2H), 10.00 (s, 1H), 9.01 (s, 1H), 8.71 (s, 1H), 8.26 (d, *J* = 5.3 Hz, 1H), 7.79 - 7.60 (m, 5H), 7.38 - 7.12 (m, 5H), 5.12 (dd, *J* = 13.5, 5.2 Hz, 1H), 4.63 - 4.45 (m, 4H), 4.32 - 4.21 (m, 2H), 3.90 (s, 2H), 3.37 - 3.13 (m, 6H), 2.92 (dd, *J* = 17.7, 6.0 Hz, 1H), 2.61 (d, *J* = 15.6 Hz, 1H), 2.43 - 2.34 (m, 1H), 2.02 (d, *J* = 4.7 Hz, 1H). LCMS (ESI) calcd for C₃₉H₃₅F₆N₁₀O₅⁺ [M+H]⁺: 837.26, found, 837.30.

### Example 302: preparation of N-(3-(difluoromethyl)-1-(4-(4-((2-(2,6-dioxopiperidin-3-yl)-1-oxoisoindolin-4-yl)methyl)piperazin-1-yl)phenyl)-1H-pyrazol-4-yl)-2-(2-((2,2,2-trifluoroethyl)amino)pyridin-4-yl)oxazole-4-carboxamide (GT-04271)

The target compound (GT-04271) was prepared using the method described in Scheme 11 (white solid, 28 mg, yield 44.42%). ¹H NMR (400 MHz, DMSO) δ 10.97 (d, *J* = 32.4 Hz, 2H), 9.93 (s, 1H), 8.94 (s, 1H), 8.63 (s, 1H), 8.18 (d, *J* = 5.3 Hz, 1H), 7.93 (d, *J* = 7.7 Hz, 1H), 7.79 (d, *J* = 7.3 Hz, 1H), 7.67 (d, *J* = 9.1 Hz, 3H), 7.63 - 7.51 (m, 2H), 7.36 - 7.04 (m, 5H), 5.19 - 5.08 (m, 1H), 4.79 (d, *J* = 17.5 Hz, 1H), 4.52 - 4.39 (m, 3H), 4.19 (dt, *J =* 16.5, 8.1 Hz, 2H), 3.84 (s, 2H), 3.23 (s, 6H), 2.87 (d, *J* = 12.7 Hz, 1H), 2.63 - 2.56 (m, 1H), 2.34 - 2.26 (m, 1H), 2.02 - 1.95 (m, 1H). LCMS (ESI) calcd for C₃₉H₃₆F₅N₁₀O₅⁺ [M+H]⁺: 819.27, found, 819.30.

### Example 303: preparation of N-(3-(difluoromethyl)-1-(4-(4-((2-(2,6-dioxopiperidin-3-yl)-1,3-dioxoisoindolin-5-yl)methyl)piperazin-1-yl)phenyl)-1H-pyrazol-4-yl)-2-(2-((2,2,2-trifluoroethyl)amino)pyridin-4-yl)oxazole-4-carboxamide (GT-04272)

The target compound was prepared using the method described in Scheme 11 (white solid, 58 mg, yield 90.45%). ¹H NMR (400 MHz, DMSO) δ 11.20 - 10.97 (m, 2H), 10.00 (s, 1H), 9.01 (s, 1H), 8.71 (s, 1H), 8.25 (d, *J* = 4.8 Hz, 2H), 8.10 (dd, *J* = 21.3, 7.7 Hz, 2H), 7.72 (dd, *J* = 17.4, 7.9 Hz, 3H), 7.44 - 7.11 (m, 5H), 5.20 (dd, *J* = 12.8, 5.3 Hz, 1H), 4.62 (s, 2H), 4.26 (dt, *J* = 16.5, 8.1 Hz, 2H), 3.93 (s, 2H), 3.22 (s, 6H), 2.99 - 2.85 (m, 1H), 2.70 - 2.56 (m, 2H), 2.13 - 2.04 (m, 1H). LCMS (ESI) calcd for C₃₉H₃₄F₅N₁₀O₆⁺ [M+H]⁺: 833.25, found, 833.30.

### Example 304: preparation of N-((4,6-dimethyl-2-oxo-1,2-dihydropyridin-3-yl)methyl)-4'-((4-((2-(2,6-dioxopiperidin-3-yl)-1-oxoisoindolin-5-yl)methyl)piperazin-1-yl)methyl)-5-(ethyl(tetrahydro-2H-pyran-4-yl)amino)-4-methyl-[1,1'-biphenyl]-3-carboxamide (GT-03692)

Referring to the method of Step 2 in Scheme 11 and the method of Example 1, the target compound (GT-03692) was prepared using N-((4,6-dimethyl-2-oxo-1,2-dihydropyridin-3-yl)methyl)-5-(ethyl(tetrahydro-2H-pyran-4-yl)amino)-4-methyl-4'-(piperazin-1-ylmethyl)-[1,1'-biphenyl]-3-carboxamide (GT-S-10) prepared from Intermediate Example 85 and 3-(5-(bromomethyl)-1-oxoisoindolin-2-yl)piperidine-2,6-dione (GT-03234). The target compound (GT-03692) was obtained as a white solid (59.00 mg, yield 79.85%). ¹H NMR (400 MHz, DMSO) δ 11.66 (s, 1H), 11.01 (s, 1H), 10.77 (s, 1H), 9.72 (s, 1H), 8.48 (s, 1H), 8.02 (s, 2H), 7.84 (dd, *J* = 33.8, 7.6 Hz, 4H), 7.62 (dd, *J* = 9.1, 4.2 Hz, 1H), 7.46 - 7.25 (m, 2H), 5.92 (s, 1H), 5.14 (dd, *J* = 12.8, 4.5 Hz, 1H), 4.60 (d, *J* = 12.5 Hz, 2H), 4.54 - 4.47 (m, 2H), 4.46 - 4.36 (m, 3H), 4.31 (d, *J* = 3.7 Hz, 2H), 4.07 - 3.73 (m, 7H), 3.63 - 3.49 (m, 3H), 3.24 (s, 2H), 3.14 - 3.05 (m, 3H), 2.92 (t, *J* = 13.1 Hz, 1H), 2.63 (s, 3H), 2.50 (s, 3H), 2.46 - 2.37 (m, 3H), 2.23 (s, 3H), 2.13 (s, 3H), 2.03 (dd, *J* = 17.3, 11.5 Hz, 2H), 1.32 (s, 3H). LCMS (ESI) calcd for C₄₈H₅₈N₇O₆⁺ [M+H]⁺: 828.44, found, 828.50.

### Example 305: preparation of 3-(5-((4-(6-(6-((R)-2-(3-fluorophenyl)pyrrolidin-1-yl)imidazo[1,2-b]pyridazin-3-yl)pyridin-2-yl)piperazin-1-yl)methyl)-1-oxoisoindolin-2-yl)piperidine-2,6-dione (GT-03793)

Referring to the method of Step 2 in Scheme 11 and the method of Example 1, the target compound (GT-03793) was prepared using (R)-6-(2-(3-fluorophenyl)pyrrolidin-1-yl)-3-(6-(piperazin-1-yl)pyridin-2-yl)imidazo[1,2-b]pyridazine (GT-S-17) prepared from Intermediate Example 91 and 3-(5-(bromomethyl)-1-oxoisoindolin-2-yl)piperidine-2,6-dione (GT-03234). The target compound (GT-03793) was obtained as a white solid (43 mg, yield 54.51%). ¹H NMR (400 MHz, DMSO) δ 11.01 (s, 1H), 8.12 (s, 1H), 7.89 (d, *J* = 9.9 Hz, 2H), 7.78 (dd, *J* = 16.6, 7.5 Hz, 2H), 7.64 - 7.48 (m, 2H), 7.38 (d, *J*= 6.3 Hz, 1H), 7.16 (d, *J* = 7.6 Hz, 2H), 7.03 (t, *J* = 8.2 Hz, 1H), 6.82 (t, *J* = 10.3 Hz, 2H), 5.18 - 5.11 (m, 2H), 4.44 (dd, *J* = 52.7, 17.6 Hz, 6H), 3.98 (dd, *J* = 9.7, 4.9 Hz, 1H), 3.66 (dd, *J* = 17.1, 7.9 Hz, 2H), 3.16 - 3.03 (m, 2H), 3.01 - 2.85 (m, 2H), 2.61 (d, *J* = 17.2 Hz, 1H), 2.49 - 2.35 (m, 3H), 2.27 - 1.91 (m, 4H), 1.89 - 1.81 (m, 1H). LCMS (ESI) calcd for C₃₉H₃₉FN₉O₃⁺ [M+H]⁺: 700.31, found, 700.30.

### Example 306: preparation of 3-(5-(((1-(6-(6-((R)-2-(3-fluorophenyl)pyrrolidin-1-yl)imidazo[1,2-b]pyridazin-3-yl)pyridin-2-yl)piperidin-4-yl)(methyl)amino)methyl)-1-oxoisoindolin-2-yl)piperidine-2,6-dione (GT-03794)

Referring to the method of Step 2 in Scheme 11 and the method of Example 1, the target compound (GT-03794) was prepared using (R)-1-(6-(6-(2-(3-fluorophenyl)pyrrolidin-1-yl)imidazo[1,2-b]pyridazin-3-yl)pyridin-2-yl)-N-methylpiperidin-4-amine (GT-S-18) prepared from Intermediate Example 92 and 3-(5-(bromomethyl)-1-oxoisoindolin-2-yl)piperidine-2,6-dione (GT-03234). The target compound (GT-03794) was obtained as a white solid (20 mg, yield 28.53%). ¹H NMR (400 MHz, DMSO) δ 11.47 (s, 1H), 11.02 (s, 1H), 8.59 (s, 1H), 8.21 (d, *J*= 5.8 Hz, 1H), 7.99 (s, 1H), 7.85 (s, 1H), 7.79 (d, *J* = 7.9 Hz, 1H), 7.41 (dd, *J* = 13.8, 7.7 Hz, 2H), 7.19 (d, *J* = 7.6 Hz, 2H), 7.13 - 6.94 (m, 3H), 5.23 (d, *J* = 4.7 Hz, 1H), 5.13 (dd, *J* = 13.1, 4.9 Hz, 1H), 4.60 (d, *J* = 10.7 Hz, 3H), 4.53 - 4.47 (m, 2H), 4.41 - 4.33 (m, 4H), 3.72 (dd, *J* = 16.6, 8.2 Hz, 2H), 3.53 (s, 1H), 2.96 - 2.80 (m, 3H), 2.63 (s, 2H), 2.46 - 2.34 (m, 2H), 2.26 (d, *J* = 11.8 Hz, 1H), 2.03 (dd, *J* = 17.8, 8.5 Hz, 3H), 1.92 (d, *J* = 7.5 Hz, 1H), 1.84 - 1.71 (m, 2H). LCMS (ESI) calcd for C₄₁H₄₃FN₉O₃⁺ [M+H]⁺: 728.34, found, 728.40.

### Example 307: preparation of 3-(4-fluoro-5-((4-(6-(6-((R)-2-(3-fluorophenyl)pyrrolidin-1-yl)imidazo[1,2-b]pyridazin-3-yl)pyridin-2-yl)piperazin-1-yl)methyl)-1-oxoisoindolin-2-yl)piperidine-2,6-dione (GT-03865)

Referring to the method of Scheme 11, the target compound (GT-03865) was prepared using (R)-6-(2-(3-fluorophenyl)pyrrolidin-1-yl)-3-(6-(piperazin-1-yl)pyridin-2-yl)lmldazo[1,2-b]pyridazine (GT-S-17) prepared from Intermediate Example 91 and 3-(4-fluoro-5-(hydroxymethyl)-1-oxoisoindolin-2-yl)piperidine-2,6-dione (GT-03445). The target compound (GT-03865) was obtained as a white solid (67 mg, yield 82.80%). ¹H NMR (400 MHz, DMSO) δ 11.04 (s, 1H), 8.44 (s, 1H), 8.08 (d, *J* = 9.9 Hz, 1H), 8.00 (t, *J* = 6.7 Hz, 1H), 7.70 (d, *J* = 7.7 Hz, 1H), 7.68 - 7.43 (m, 2H), 7.42 - 7.33 (m, 1H), 7.18 (d, *J* = 8.0 Hz, 3H), 7.04 (t, *J* = 8.3 Hz, 1H), 6.93 (d, *J* = 8.3 Hz, 1H), 5.23 - 5.13 (m, 2H), 4.65 - 4.40 (m, 6H), 4.06 - 4.00 (m, 1H), 3.70 (dd, *J* = 17.1, 8.4 Hz, 2H), 3.26 - 3.12 (m, 4H), 3.03 - 2.84 (m, 2H), 2.61 (d, *J* = 17.2 Hz, 1H), 2.48 - 2.38 (m, 2H), 2.09 - 1.99 (m, 3H), 1.94 - 1.86 (m, 1H). LCMS (ESI) calcd for C₃₉H₃₈F₂N₉O₃⁺ [M+H]⁺: 718.30, found, 718.30.

### Example 308: preparation of 3-(6-fluoro-5-((4-(6-(6-((R)-2-(3-fluorophenyl)pyrrolidin-1-yl)imidazo[1,2-b]pyridazin-3-yl)pyridin-2-yl)piperazin-1-yl)methyl)-1-oxoisoindolin-2-yl)piperidine-2,6-dione (GT-03866)

The target compound (GT-03866) was prepared using the method described in Scheme 11 (white solid, 30.00 mg, yield 37.07%). ¹H NMR (400 MHz, DMSO) δ 11.03 (s, 1H), 8.53 (s, 1H), 8.14 (d, *J* = 6.3 Hz, 2H), 7.75 - 7.43 (m, 3H), 7.40 (d, *J* = 6.6 Hz, 1H), 7.18 (d, *J* = 8.3 Hz, 3H), 7.05 (t, *J* = 8.0 Hz, 1H), 6.95 (d, *J* = 8.4 Hz, 1H), 5.24 - 5.12 (m, 2H), 4.44 (dd, *J* = 52.6, 18.6 Hz, 6H), 4.06 - 4.01 (m, 1H), 3.71 (d, *J* = 8.7 Hz, 2H), 3.20 (ddd, *J* = 41.4, 26.6, 14.6 Hz, 4H), 3.06 - 2.79 (m, 2H), 2.61 (d, *J* = 16.8 Hz, 1H), 2.49 - 2.32 (m, 2H), 2.10 - 1.99 (m, 3H), 1.97 - 1.83 (m, 1H). LCMS (ESI) calcd for C₃₉H₃₈F₂N₉O₃⁺ [M+H]⁺: 718.30, found, 718.30.

### Example 309: preparation of 3-(7-fluoro-5-((4-(6-(6-((R)-2-(3-fluorophenyl)pyrrolidin-1-yl)imidazo[1,2-b]pyridazin-3-yl)pyridin-2-yl)piperazin-1-yl)methyl)-1-oxoisoindolin-2-yl)piperidine-2,6-dione (GT-03867)

Referring to the method of Scheme 11, the target compound (GT-03867) was prepared using (R)-6-(2-(3-fluorophenyl)pyrrolidin-1-yl)-3-(6-(piperazin-1-yl)pyridin-2-yl)imidazo[1,2-b]pyridazine (GT-S-17) prepared from Intermediate Example 91 and 3-(7-fluoro-5-(hydroxymethyl)-1-oxoisoindolin-2-yl)piperidine-2,6-dione (GT-03446). The target compound (GT-03867) was obtained as a white solid (78 mg, yield 96.39%). ¹H NMR (400 MHz, DMSO) δ 11.98 (s, 1H), 11.03 (s, 1H), 8.54 (s, 1H), 8.14 (d, *J* = 9.6 Hz, 1H), 7.82 - 7.47 (m, 4H), 7.40 (d, *J* = 6.4 Hz, 1H), 7.18 (d, *J* = 8.2 Hz, 3H), 7.04 (d, *J* = 8.1 Hz, 1H), 6.96 (d, *J* = 8.5 Hz, 1H), 5.22 (d, *J* = 6.8 Hz, 1H), 5.11 (dd, *J* = 13.2, 5.0 Hz, 1H), 4.49 (t, *J* = 26.5 Hz, 6H), 4.07 - 4.02 (m, 1H), 3.74 - 3.68 (m, 2H), 3.25 - 3.04 (m, 4H), 3.01 - 2.85 (m, 2H), 2.60 (d, *J* = 16.8 Hz, 1H), 2.48 - 2.33 (m, 2H), 2.09 - 1.99 (m, 3H), 1.94 - 1.87 (m, 1H). LCMS (ESI) calcd for C₃₉H₃₈F₂N₉O₃⁺ [M+H]⁺: 718.30, found, 718.30.

### Example 310: preparation of 3-(4-((4-(6-(6-((R)-2-(3-fluorophenyl)pyrrolidin-1-yl)imidazo[1,2-b]pyridazin-3-yl)pyridin-2-yl)piperazin-1-yl)methyl)-1-oxoisoindolin-2-yl)piperidine-2,6-dione (GT-03868)

The target compound (GT-03868) was prepared using the method described in Scheme 11 (white solid, 72 mg, yield 91.27%). ¹H NMR (400 MHz, DMSO) δ 11.70 (s, 1H), 11.04 (s, 1H), 8.38 (s, 1H), 8.05 (t, *J* = 7.5 Hz, 2H), 7.83 (d, *J* = 7.6 Hz, 1H), 7.70 - 7.50 (m, 3H), 7.39 (td, *J* = 8.0, 6.5 Hz, 1H), 7.24 - 7.09 (m, 3H), 7.06 - 7.00 (m, 1H), 6.95 - 6.87 (m, 1H), 5.22 - 5.15 (m, 2H), 4.93 (d, *J* = 17.7 Hz, 1H), 4.65 - 4.30 (m, 6H), 4.05 - 3.99 (m, 1H), 3.70 (dd, *J* = 17.5, 8.2 Hz, 2H), 3.25 - 3.16 (m, 3H), 3.03 - 2.82 (m, 2H), 2.63 (d, *J* = 18.1 Hz, 2H), 2.35 - 2.26 (m, 1H), 2.09 - 2.01 (m, 3H), 1.94 - 1.83 (m, 1H). LCMS (ESI) calcd for C₃₉H₃₉FN₉O₃⁺ [M+H]⁺: 700.31, found, 700.30.

### Example 311: preparation of 2-(2,6-dioxopiperidin-3-yl)-5-((4-(6-(6-((R)-2-(3-fluorophenyl)pyrrolidin-1-yl)imidazo[1,2-b]pyridazin-3-yl)pyridin-2-yl)piperazin-1-yl)methyl)isoindoline-1,3-dione (GT-03869)

Referring to the method of Scheme 11, the target compound (GT-03869) was prepared using (R)-6-(2-(3-fluorophenyl)pyrrolidin-1-yl)-3-(6-(piperazin-1-yl)pyridin-2-yl)imidazo[1,2-b]pyridazine (GT-S-17) prepared from Intermediate Example 91 and 5-(bromomethyl)-2-(2,6-dioxo-piperidin-3-yl)isoindoline-1,3-dione (CAS NO.: 1312023-72-5). The target compound (GT-03869) was obtained as a white solid (66 mg, yield 82.03%). ¹H NMR (400 MHz, DMSO) δ 11.97 (s, 1H), 11.15 (s, 1H), 8.52 (s, 1H), 8.29 (s, 1H), 8.15 (dd, *J* = 17.0, 9.0 Hz, 2H), 8.03 (d, *J* = 7.6 Hz, 1H), 7.74 - 7.45 (m, 2H), 7.44 - 7.25 (m, 2H), 7.18 (d, *J* = 8.0 Hz, 2H), 7.05 (t, *J* = 8.0 Hz, 1H), 6.96 (d, *J* = 8.6 Hz, 1H), 5.27 - 5.16 (m, 2H), 4.55 (d, *J* = 32.2 Hz, 5H), 4.15 - 3.96 (m, 2H), 3.72 (dd, *J* = 16.2, 6.8 Hz, 2H), 3.19 - 3.03 (m, 3H), 2.92 (dd, *J* = 9.6, 6.5 Hz, 1H), 2.59 (dd, *J* = 21.9, 10.9 Hz, 3H), 2.12 - 2.02 (m, 3H), 1.90 (dd, *J* = 11.8, 4.8 Hz, 1H). LCMS (ESI) calcd for C₃₉H₃₇FN₉O₄⁺ [M+H]⁺: 714.29, found, 714.30.

### Example 312: preparation of 2-(2,6-dioxopiperidin-3-yl)-5-(((1-(6-(6-((R)-2-(3-fluorophenyl)pyrrolidin-1-yl)imidazo[1,2-b]pyridazin-3-yl)pyridin-2-yl)piperidin-4-yl)(methyl)amino)methyl)isoindoline-1,3-dione (GT-03897)

Referring to the method of Step 2 in Scheme 11 and the method of Example 1, the target compound (GT-03897) was prepared using (R)-1-(6-(6-(2-(3-fluorophenyl)pyrrolidin-1-yl)imidazo[1,2-b]pyridazin-3-yl)pyridin-2-yl)-N-methylpiperidin-4-amine (GT-S-18) prepared from Intermediate Example 92 and 5-(bromomethyl)-2-(2,6-dioxo-piperidin-3-yl)isoindoline-1,3-dione (CAS NO.: 1312023-72-5). The target compound (GT-03897) was obtained as a white solid (30.00 mg, yield 41.09%). ¹H NMR (400 MHz, DMSO) δ 11.14 (s, 1H), 8.57 (s, 1H), 8.34 (s, 1H), 8.24 (d, *J* = 7.6 Hz, 1H), 8.18 (d, *J*= 10.1 Hz, 1H), 8.00 (d, *J* = 7.7 Hz, 1H), 7.54 (s, 1H), 7.40 (dd, *J*= 14.2, 7.9 Hz, 2H), 7.18 (d, *J* = 8.2 Hz, 2H), 7.07 (dd, *J* = 18.0, 10.0 Hz, 2H), 6.99 (d, *J* = 8.4 Hz, 1H), 5.25 - 5.16 (m, 2H), 4.64 (dd, *J* = 21.0, 10.0 Hz, 3H), 4.11 - 4.01 (m, 2H), 3.72 (d, *J* = 9.4 Hz, 2H), 3.56 (s, 2H), 2.91 - 2.82 (m, 3H), 2.58 (d, *J* = 3.7 Hz, 4H), 2.32 (dd, *J* = 13.6, 9.2 Hz, 2H), 2.07 (dd, *J* = 5.8, 3.5 Hz, 3H), 1.92 (dd, *J* = 11.8,4.2 Hz, 1H), 1.83 - 1.72 (m, 2H). LCMS (ESI) calcd for C₄₁H₄₁FN₉O₄⁺ [M+H]⁺: 742.32, found, 742.30.

### Example 313: preparation of 4-((((2-(2,6-dioxopiperidin-3-yl)-1-oxoisoindolin-5-yl)methyl)(methyl)amino)methyl)-N-(4-methyl-3-((4-(pyridin-3-yl)pyrimidin-2-yl)amino)phenyl)benzamide (GT-03990)

The target compound (GT-03990) was prepared using the methods described in Scheme 11 and Example 1 (white solid, 15 mg, yield 29.9%). ¹H NMR (400 MHz, MeOD) δ 9.55 (s, 1H), 9.19 (d, *J* = 8.2 Hz, 1H), 8.83 (d, *J* = 5.4 Hz, 1H), 8.49 (d, *J* = 5.3 Hz, 1H), 8.20 (s, 1H), 8.05 (dd, *J* = 8.2, 5.7 Hz, 1H), 7.97 (d, *J* = 8.2 Hz, 2H), 7.82 (d, *J* = 7.8 Hz, 1H), 7.67 (s, 1H), 7.59 (d, *J* = 8.1 Hz, 3H), 7.46 (d, *J* = 5.4 Hz, 1H), 7.29 - 7.17 (m, 2H), 5.07 (dd, *J* = 13.3, 5.1 Hz, 1H), 4.46 (q, *J* = 17.4 Hz, 6H), 2.86 - 2.62 (m, 5H), 2.40 (dt, *J* = 13.7, 8.7 Hz, 1H), 2.23 (s, 3H), 2.13 - 2.02 (m, 1H). LCMS (ESI) calcd for C₃₉H₃₇N₈O₄⁺ [M+H]⁺: 681.29, found, 681.3.

### Example 314: preparation of N-(4-(4-(1-((2-(2,6-dioxopiperidin-3-yl)-1-oxoisoindolin-4-yl)methyl)piperidin-4-yl)piperazin-1-yl)-3-(trifluoromethyl)phenyl)-3-(imidazo[1,2-b]pyridazin-3-ylethynyl)-4-methylbenzamide (GT-04236)

The target compound (GT-04236) was prepared using the methods described in Scheme 11 and Example 1 (light yellow solid, 35 mg, yield 44.91%). ¹H NMR (400 MHz, MeOD) δ 8.89 (d, *J* = 4.4 Hz, 1H), 8.40 - 8.29 (m, 2H), 8.25 - 8.15 (m, 2H), 8.10 - 8.02 (m, 1H), 7.96 (dd, *J* = 17.8, 7.8 Hz, 3H), 7.77 - 7.66 (m, 2H), 7.62 (d, *J* = 8.8 Hz, 1H), 7.54 (d, *J* = 8.3 Hz, 1H), 5.25 (dd, *J* = 13.2, 5.1 Hz, 1H), 4.90 (s, 1H), 4.71 (d, *J* = 17.2 Hz, 1H), 4.55 (t, *J* = 11.0 Hz, 2H), 3.88 - 3.64 (m, 5H), 3.37 (d, *J* = 15.4 Hz, 4H), 3.28 (s, 4H), 3.05 - 2.91 (m, 1H), 2.84 (d, *J* = 16.0 Hz, 1H), 2.69 (s, 3H), 2.57 (s, 3H), 2.28 (s, 3H). LCMS (ESI) calcd for C₄₆H₄₅F₃N₉O₄⁺ [M+H]⁺: 844.35, found, 844.4.

### Example 315: preparation of N-(4-(4-(1-((2-(2,6-dioxopiperidin-3-yl)-1-oxoisoindolin-5-yl)methyl)piperidin-4-yl)piperazin-1-yl)-3-(trifluoromethyl)phenyl)-3-(imidazo[1,2-b]pyridazin-3-ylethynyl)-4-methylbenzamide (GT-04237)

Referring to the method of Step 2 in Scheme 11 and the method of Example 1, the target compound (GT-04237) was prepared using 3-(imidazo[1,2-b]pyridazin-3-ylethynyl)-4-methyl-N-(4-(4-(piperidin-4-yl)piperazin-1-yl)-3-(trifluoromethyl)phenyl)benzamide (GT-D-112) prepared from Intermediate Example 71 and 3-(5-(bromomethyl)-1-oxoisoindolin-2-yl)piperidine-2,6-dione (GT-03234). The target compound (GT-04237) was obtained as a light yellow solid (18 mg, yield 23.10%). ¹H NMR (400 MHz, MeOD) δ 9.01 (d, *J* = 4.5 Hz, 1H), 8.51 (s, 1H), 8.44 (d, *J* = 9.6 Hz, 1H), 8.21 (d, *J* = 16.8 Hz, 2H), 8.06 (d, *J* = 8.8 Hz, 1H), 7.97 (t, *J* = 8.1 Hz, 2H), 7.90 - 7.81 (m, 2H), 7.77 (d, *J* = 7.3 Hz, 1H), 7.63 (d, *J* = 8.3 Hz, 1H), 7.55 (d, *J* = 8.1 Hz, 1H), 5.20 (d, *J* = 13.7 Hz, 1H), 4.61 (dd, *J* = 25.2, 18.1 Hz, 4H), 3.76 (dd, *J* = 13.1, 6.6 Hz, 6H), 3.25 (dd, *J* = 14.8, 7.4 Hz, 7H), 2.95 (s, 1H), 2.82 (d, *J* = 15.9 Hz, 1H), 2.70 (s, 3H), 2.56 (s, 3H), 2.24 (s, 3H). LCMS (ESI) calcd for C₄₆H₄₅F₃N₉O₄⁺ [M+H]⁺: 844.35, found, 844.3.

### Example 316: preparation of N-(4-(4-(1-((2-(2,6-dioxopiperidin-3-yl)-4-fluoro-1-oxoisoindolin-5-yl)methyl)piperidin-4-yl)piperazin-1-yl)-3-(trifluoromethyl)phenyl)-3-(imidazo[1,2-b]pyridazin-3-ylethynyl)-4-methylbenzamide (GT-04238)

Referring to the method of Scheme 11, the target compound (GT-04238) was prepared using 3-(imidazo[1,2-b]pyridazin-3-ylethynyl)-4-methyl-N-(4-(4-(piperidin-4-yl)piperazin-1-yl)-3-(trifluoromethyl)phenyl)benzamide (GT-D-112) prepared from Intermediate Example 71 and 3-(4-fluoro-5-(hydroxymethyl)-1-oxoisoindolin-2-yl)piperidine-2,6-dione (GT-03445). The target compound (GT-0423 8) was obtained as a light yellow solid (34 mg, yield 42.79%). ¹H NMR (400 MHz, MeOD) δ 9.05 (d, *J* = 3.8 Hz, 1H), 8.55 (s, 1H), 8.47 (d, *J* = 9.4 Hz, 1H), 8.23 (s, 1H), 8.20 (s, 1H), 8.06 (d, *J* = 8.3 Hz, 1H), 7.99 (d, *J* = 9.6 Hz, 1H), 7.92 - 7.84 (m, 2H), 7.81 (d, *J* = 7.8 Hz, 1H), 7.63 (d, *J* = 8.7 Hz, 1H), 7.56 (d, *J* = 8.1 Hz, 1H), 5.25 - 5.18 (m, 1H), 4.68 (dd, *J* = 29.2, 17.2 Hz, 4H), 3.74 (dd, *J* = 13.1, 6.8 Hz, 6H), 3.31 - 3.14 (m, 7H), 2.93 (d, *J* = 13.6 Hz, 1H), 2.84 (s, 1H), 2.70 (s, 3H), 2.57 (s, 3H), 2.24 (s, 3H). LCMS (ESI) calcd for C₄₆H₄₄F₄N₉O₄⁺ [M+H]⁺: 862.34, found, 862.3.

### Example 317: preparation of N-(4-(4-(1-((2-(2,6-dioxopiperidin-3-yl)-6-fluoro-1-oxoisoindolin-5-yl)methyl)piperidin-4-yl)piperazin-1-yl)-3-(trifluoromethyl)phenyl)-3-(imidazo[1,2-b]pyridazin-3-ylethynyl)-4-methylbenzamide (GT-04239)

The target compound (GT-04239) was prepared using the method described in Scheme 11 (light yellow solid, 36 mg, yield 45.31%). ¹H NMR (400 MHz, MeOD) δ 8.98 (d, *J* = 3.4 Hz, 1H), 8.47 (s, 1H), 8.41 (d, *J* = 9.4 Hz, 1H), 8.21 (d, *J* = 14.8 Hz, 2H), 8.10 - 8.02 (m, 1H), 7.97 (t, *J* = 5.9 Hz, 2H), 7.81 (dd, *J* = 9.3, 4.5 Hz, 1H), 7.72 (d, *J* = 8.6 Hz, 1H), 7.63 (d, *J* = 8.6 Hz, 1H), 7.55 (d, *J* = 8.2 Hz, 1H), 5.20 (dd, *J* = 13.4, 5.1 Hz, 1H), 4.67 - 4.49 (m, 4H), 3.77 (t, *J* = 25.9 Hz, 5H), 3.34 (s, 4H), 3.27 (d, *J* = 11.9 Hz, 4H), 3.01 -2.88 (m, 1H), 2.82 (d, *J* = 15.6 Hz, 1H), 2.70 (s, 3H), 2.55 (dd, *J* = 19.6, 6.7 Hz, 3H), 2.23 (s, 3H). LCMS (ESI) calcd for C₄₆H₄₄F₄N₉O₄⁺ [M+H]⁺: 862.34, found, 862.4.

### Example 318: preparation of N-(4-(4-(1-((2-(2,6-dioxopiperidin-3-yl)-7-fluoro-1-oxoisoindolin-5-yl)methyl)piperidin-4-yl)piperazin-1-yl)-3-(trifluoromethyl)phenyl)-3-(imidazo[1,2-b]pyridazin-3-ylethynyl)-4-methylbenzamide (GT-04240)

Referring to the method of Scheme 11, the target compound (GT-04240) was prepared using 3-(imidazo[1,2-b]pyridazin-3-ylethynyl)-4-methyl-N-(4-(4-(piperidin-4-yl)piperazin-1-yl)-3-(trifluoromethyl)phenyl)benzamide (GT-D-112) prepared from Intermediate Example 71 and 3-(7-fluoro-5-(hydroxymethyl)-1-oxoisoindolin-2-yl)piperidine-2,6-dione (GT-03446). The target compound (GT-04240) was obtained as a light yellow solid (34 mg, yield 42.79%). ¹H NMR (400 MHz, MeOD) δ 9.06 (d, *J* = 4.7 Hz, 1H), 8.57 (s, 1H), 8.48 (d, *J* = 8.1 Hz, 1H), 8.21 (d, *J* = 18.7 Hz, 2H), 8.06 (d, J = 6.2 Hz, 1H), 8.00 (d, *J* = 8.4 Hz, 1H), 7.91 (dd, *J* = 9.4, 4.5 Hz, 1H), 7.69 (s, 1H), 7.63 (d, *J* = 8.5 Hz, 1H), 7.54 (dd, *J* = 16.5, 8.8 Hz, 2H), 5.18 (dd, *J* = 13.3, 5.0 Hz, 1H), 4.61 (dd, *J* = 29.6, 21.3 Hz, 4H), 3.75 (d, *J* = 12.0 Hz, 5H), 3.35 (s, 4H), 3.29 (s, 4H), 3.02 - 2.89 (m, 1H), 2.81 (d, *J* = 15.5 Hz, 1H), 2.70 (s, 3H), 2.55 (s, 3H), 2.23 (s, 3H). LCMS (ESI) calcd for C₄₆H₄₄F₄N₉O₄⁺ [M+H]⁺: 862.34, found, 862.3.

### Example 319: preparation of N-(4-(chlorodifluoromethoxy)phenyl)-6-(4-(((2-(2,6-dioxopiperidin-3-yl)-1-oxoisoindolin-5-yl)methyl)(methyl)amino)piperidin-1-yl)-5-(1H-pyrazol-5-yl)nicotinamide (GT-04019)

The target compound (GT-04019) was prepared using the methods described in Scheme 11 and Example 1 (white solid,27 mg, yield 42.66%). ¹H NMR (400 MHz, DMSO) δ 11.01 (s, 1H), 10.89 (s, 1H), 10.53 (s, 1H), 8.80 (d, *J* = 2.4 Hz, 1H), 8.40 (d, *J* = 2.4 Hz, 1H), 7.91 (dd, *J* = 6.9, 2.2 Hz, 3H), 7.84 - 7.77 (m, 3H), 7.35 (d, *J* = 9.1 Hz, 2H), 6.73 (dd, *J* = 2.0, 1.0 Hz, 1H), 5.14 (dd, *J* = 13.2, 5.0 Hz, 1H), 4.60 - 4.47 (m, 2H), 4.44 - 4.29 (m, 3H), 3.48 - 3.37 (m, 2H), 2.96 - 2.89 (m, 1H), 2.79 (dd, *J* = 21.7, 11.1 Hz, 2H), 2.64 - 2.57 (m, 4H), 2.47 - 2.34 (m, 1H), 2.17 (dd, *J* = 24.0, 10.7 Hz, 2H), 2.05 - 1.98 (m, 1H), 1.95 - 1.79 (m, 2H). LCMS (ESI) calcd for C₃₆H₃₆ClF₂N₈O₅⁺ [M+H]⁺: 733.24, found, 733.30.

### Example 320: preparation of the following compounds

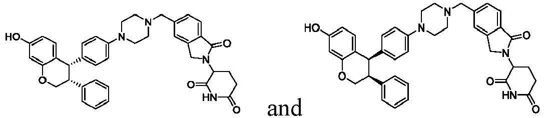

Referring to the method of Step 2 in Scheme 11 and the method of Example 1, one of the intermediates prepared in Intermediate Example 94 was reacted with 3-(5-(bromomethyl)-1-oxoisoindolin-2-yl)piperidine-2,6-dione (GT-03234) to obtain the corresponding target compound.

The structural characterization data of the target compound prepared are as follows:
One of the two target compounds (GT-03795) (white solid, 30 mg, yield 33%): ¹H NMR (400 MHz, MeOD) δ 7.93 (d, *J* = 7.8 Hz, 1H), 7.80 (s, 1H), 7.72 (d, *J* = 7.9 Hz, 1H), 7.17 - 7.10 (m, 3H), 6.74 - 6.65 (m, 5H), 6.56 - 6.52 (m, 2H), 6.35 (d, *J* = 2.1 Hz, 1H), 6.30 (dd, *J* = 8.3, 2.4 Hz, 1H), 5.19 (dd, *J* = 13.3, 5.1 Hz, 1H), 4.88 - 4.88 (m, 1H), 4.61 - 4.50 (m, 4H), 4.40 (t, *J* = 11.1 Hz, 1H), 4.23 (d, *J* = 5.1 Hz, 1H), 4.19 (d, *J* = 7.8 Hz, 1H), 3.78 - 3.68 (m, 2H), 3.58 - 3.51 (m, 2H), 3.38 - 3.32 (m, 2H), 3.05 - 2.87 (m, 3H), 2.84 - 2.76 (m, 1H), 2.52 (qd, *J*= 13.1, 4.7 Hz, 1H), 2.23 - 2.15 (m, 1H). LCMS (ESI) m/z: calcd for C₃₉H₃₉N₄O₅⁺[M+H]⁺, 643.29; found, 643.3.
another target compound (GT-03774) (white solid, 54 mg, yield 64.94%): ¹H NMR (400 MHz, DMSO) δ 11.35 (s, 1H), 11.00 (s, 1H), 7.90 (s, 1H), 7.80 (dd, *J* = 16.7, 7.8 Hz, 2H), 7.18 - 7.08 (m, 3H), 6.76 (dd, *J* = 6.2, 3.1 Hz, 2H), 6.65 (dd, *J* = 8.4, 5.3 Hz, 3H), 6.43 (d, *J* = 8.6 Hz, 2H), 6.35 - 6.24 (m, 2H), 5.14 (dd, *J* = 13.3, 5.1 Hz, 1H), 4.50 (d, *J* = 17.7 Hz, 3H), 4.40 - 4.29 (m, 2H), 4.19 (dd, *J* = 10.3, 3.8 Hz, 2H), 3.65 (d, *J* = 10.7 Hz, 2H), 3.54 (d, *J* = 4.7 Hz, 1H), 3.34 (d, *J* = 9.4 Hz, 2H), 3.16 - 3.02 (m, 4H), 2.97 - 2.88 (m, 1H), 2.61 (d, *J* = 16.9 Hz, 1H), 2.46 - 2.35 (m, 1H), 2.04 - 1.93 (m, 1H). LCMS (ESI) calcd for C₃₉H₃₉N₄O₅⁺ [M+H]⁺: 643.28, found, 643.30.

### Example 321: preparation of the following compounds

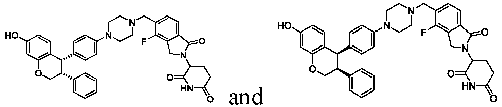

Referring to the method of Scheme 11, one of the intermediates prepared in Intermediate Example 94 was reacted with 3-(4-fluoro-5-(hydroxymethyl)-1-oxoisoindolin-2-yl)piperidine-2,6-dione (GT-03445) to obtain the corresponding target compound.

The structural characterization data of the target compound prepared are as follows:
One of the two target compounds (GT-03796) (white solid, 39 mg, yield 42%): ¹H NMR (400 MHz, MeOD) δ 7.84 - 7.74 (m, 2H), 7.15 - 7.07 (m, 3H), 6.77 - 6.65 (m, 5H), 6.54 (d, *J* = 8.4 Hz, 2H), 6.35 (d, *J* = 2.1 Hz, 1H), 6.30 (dd, *J* = 8.3, 2.2 Hz, 1H), 5.18 (dd, *J* = 13.3, 5.1 Hz, 1H), 4.87 - 4.85 (m, 1H), 4.71 - 4.55 (m, 4H), 4.40 (t, *J* = 11.1 Hz, 1H), 4.23 (d, *J* = 5.2 Hz, 1H), 4.19 (d, *J* = 8.0 Hz, 1H), 3.81 - 3.38 (m, 6H), 3.11 - 2.86 (m, 3H), 2.84 - 2.73 (m, 1H), 2.53 (qd, *J* = 13.2, 4.7 Hz, 1H), 2.25 - 2.15 (m, 1H). LCMS (ESI) m/z: calcd for C₃₉H₃₈FN₄O₅⁺[M+H]⁺, 661.28; found, 661.3.
another target compound (GT-03784) (white solid, 68 mg, yield 79.55%): ¹H NMR (400 MHz, DMSO) δ 11.35 (s, 1H), 11.04 (s, 1H), 7.96 (t, *J* = 6.9 Hz, 1H), 7.69 (d, *J* = 7.7 Hz, 1H), 7.18 - 7.08 (m, 3H), 6.76 (dd, *J* = 6.1, 3.0 Hz, 2H), 6.65 (t, *J* = 8.0 Hz, 3H), 6.43 (d, *J* = 8.6 Hz, 2H), 6.34 - 6.21 (m, 2H), 5.15 (dd, *J* = 13.2, 5.0 Hz, 1H), 4.62 - 4.41 (m, 4H), 4.32 (t, *J* = 11.1 Hz, 1H), 4.19 (dd, *J* = 10.5, 3.7 Hz, 2H), 3.66 (d, *J* = 11.4 Hz, 2H), 3.56 - 3.48 (m, 3H), 3.18 (s, 2H), 3.05 (t, *J* = 12.7 Hz, 2H), 2.97 - 2.85 (m, 1H), 2.61 (dd, *J* = 15.8, 1.6 Hz, 1H), 2.48 - 2.38 (m, 1H), 2.01 (dt, *J =* 10.2, 3.7 Hz, 1H). LCMS (ESI) calcd for C₃₉H₃₈FN₄O₅⁺ [M+H]⁺: 661.27, found, 661.30.

### Example 322: preparation of the following compounds

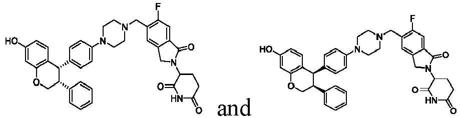

Referring to the method of Scheme 11, one of the intermediates prepared in Intermediate Example 94 was reacted with 3-(6-fluoro-5-(hydroxymethyl)-1-oxoisoindolin-2-yl)piperidine-2,6-dione to obtain the corresponding target compound.

The structural characterization data of the target compound prepared are as follows:
One of the two target compounds (GT-03797) (white solid,53 mg, yield 58%): ¹H NMR (400 MHz, MeOD) δ 7.86 (d, *J* = 6.0 Hz, 1H), 7.69 (d, *J* = 8.5 Hz, 1H), 7.14 - 7.07 (m, 3H), 6.75 - 6.65 (m, 5H), 6.54 (d, *J* = 8.7 Hz, 2H), 6.35 (d, *J* = 2.4 Hz, 1H), 6.30 (dd, *J* = 8.3, 2.5 Hz, 1H), 5.18 (dd, *J* = 13.3, 5.2 Hz, 1H), 4.66 - 4.46 (m, 5H), 4.40 (t, *J* = 11.1 Hz, 1H), 4.23 (d, *J* = 5.2 Hz, 1H), 4.19 (dd, *J* = 10.1, 2.8 Hz, 1H), 3.76 - 3.36 (m, 6H), 3.09 - 2.85 (m, 3H), 2.83 - 2.75 (m, 1H), 2.51 (qd, *J* = 13.4, 4.9 Hz, 1H), 2.24 - 2.15 (m, 1H). LCMS (ESI) m/z: calcd for C₃₉H₅₅FN₄O₅⁺[M+H]⁺, 661.28; found, 661.3.
another target compound (GT-03785) (white solid, 69 mg, yield 80.72%): ¹H NMR (400 MHz, DMSO) δ 11.46 (s, 1H), 11.03 (s, 1H), 8.08 (d, *J* = 6.1 Hz, 1H), 7.67 (d, *J* = 8.4 Hz, 1H), 7.19 - 7.08 (m, 3H), 6.77 (d, *J* = 3.2 Hz, 2H), 6.65 (dd, *J* = 8.2, 6.5 Hz, 3H), 6.43 (d, *J* = 8.5 Hz, 2H), 6.35 - 6.24 (m, 2H), 5.14 (dd, *J* = 13.2, 5.0 Hz, 1H), 4.49 (d, *J* = 17.4 Hz, 3H), 4.33 (dd, *J* = 23.8, 14.0 Hz, 2H), 4.19 (dd, *J* = 10.6, 3.5 Hz, 2H), 3.66 (d, *J* = 12.4 Hz, 2H), 3.44 (d, *J* = 13.1 Hz, 3H), 3.19 (s, 2H), 3.07 (t, *J* = 11.9 Hz, 2H), 2.98 - 2.85 (m, 1H), 2.60 (d, *J* = 16.4 Hz, 1H), 2.48 - 2.35 (m, 1H), 2.07 - 1.96 (m, 1H). LCMS (ESI) calcd for C₃₉H₃₈FN₄O₅⁺ [M+H]⁺: 661.27, found, 661.30.

### Example 323: preparation of the following compounds

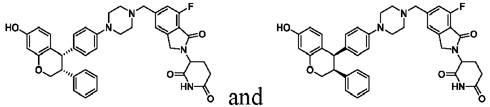

Referring to the method of Scheme 11, one of the intermediates prepared in Intermediate Example 94 was reacted with 3-(7-fluoro-5-(hydroxymethyl)-1-oxoisoindolin-2-yl)piperidine-2,6-dione (GT-03446) to obtain the corresponding target compound.

The structural characterization data of the target compound prepared are as follows:
One of the two target compounds (GT-03798) (white solid,42 mg, yield 46%): ¹H NMR (400 MHz, MeOD) δ 7.60 (s, 1H), 7.45 (d, *J* = 9.5 Hz, 1H), 7.11 (dd, *J* = 5.1, 1.9 Hz, 3H), 6.75 - 6.65 (m, 5H), 6.54 (d, *J* = 8.7 Hz, 2H), 6.35 (d, *J* = 2.4 Hz, 1H), 6.30 (dd, *J* = 8.3, 2.5 Hz, 1H), 5.15 (dd, *J* = 13.3, 5.1 Hz, 1H), 4.84 - 4.77 (m, 1H), 4.65 - 4.56 (m, 2H), 4.52 (s, 2H), 4.41 (t, *J* = 11.1 Hz, 1H), 4.24 (d, *J* = 5.1 Hz, 1H), 4.19 (dd, *J* = 10.6, 2.2 Hz, 1H), 3.86 - 3.66 (m, 2H), 3.57 - 3.52 (m, 2H), 3.30 - 3.22 (m, 2H), 3.08 - 2.86 (m, 3H), 2.83 - 2.74 (m, 1H), 2.50 (qd, *J* = 13.1, 4.5 Hz, 1H), 2.24 - 2.14 (m, 1H). LCMS (ESI) m/z: calcd for C₃₉H₃₈FN₄O₅⁺[M+H]⁺, 661.28; found, 661.3.
another target compound (GT-03786) (white solid, 68 mg, yield 79.55%): ¹H NMR (400 MHz, DMSO) δ 11.56 (s, 1H), 11.03 (s, 1H), 7.69 (d, *J* = 11.8 Hz, 2H), 7.17 - 7.11 (m, 3H), 6.76 (dd, *J* = 6.3, 2.8 Hz, 2H), 6.65 (dd, *J* = 8.2, 6.6 Hz, 3H), 6.43 (d, *J* = 8.6 Hz, 2H), 6.33 - 6.25 (m, 2H), 5.10 (dd, *J* = 13.3, 5.1 Hz, 1H), 4.57 - 4.40 (m, 4H), 4.37 - 4.30 (m, 1H), 4.19 (dd, *J* = 10.4, 3.6 Hz, 2H), 3.65 (d, *J* = 8.5 Hz, 2H), 3.56 - 3.48 (m, 2H), 3.36 (d, *J* = 8.0 Hz, 2H), 3.09 (d, *J* = 8.2 Hz, 3H), 2.96 - 2.87 (m, 1H), 2.60 (d, *J* = 17.2 Hz, 1H), 2.44 - 2.31 (m, 1H), 2.00 (dd, *J=* 12.1, 6.8 Hz, 1H). LCMS (ESI) calcd for C₃₉H₃₈FN₄O₅⁺ [M+H]⁺: 661.27, found, 661.30.

### Example 324: preparation of the following compounds

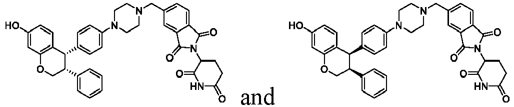

Referring to the method of Scheme 11, one of the intermediates prepared in Intermediate Example 94 was reacted with 5-(bromomethyl)-2-(2,6-dioxo-piperidin-3-yl)isoindoline-1,3-dione (CAS NO.: 1312023-72-5) to obtain the corresponding target compound.

The structural characterization data of the target compound prepared are as follows:
One of the two target compounds (GT-03801) (white solid,40 mg, yield 44%): ¹H NMR (400 MHz, DMSO) δ 11.14 (s, 1H), 9.30 (s, 1H), 8.21 (s, 1H), 8.09 (d, *J* = 7.7 Hz, 1H), 8.04 (d, *J* = 7.7 Hz, 1H), 7.17 - 7.10 (m, 3H), 6.77 (d, *J* = 2.7 Hz, 2H), 6.65 (t, *J* = 9.0 Hz, 3H), 6.44 (d, *J* = 8.6 Hz, 2H), 6.31 (d, *J* = 2.3 Hz, 1H), 6.27 (dd, *J* = 8.3, 2.4 Hz, 1H), 5.21 - 5.15 (m, 1H), 4.57 (s, 2H), 4.32 (t, *J* = 11.2 Hz, 1H), 4.21 (d, *J* = 4.3 Hz, 2H), 3.74 - 3.62 (m, 3H), 3.56 - 3.49 (m, 2H), 3.18 - 3.08 (m, 2H), 3.01 - 2.81 (m, 5H), 2.07 - 2.03 (m, 2H). LCMS (ESI) m/z: calcd for C₃₉H₃₇N₄O₆⁺[M+H]⁺, 657.27; found, 657.3.
another target compound (GT-03788) (white solid, 17 mg, yield 20.01%): ¹H NMR (400 MHz, DMSO) δ 11.35 (s, 1H), 11.15 (s, 1H), 8.25 (s, 1H), 8.13 (d, *J* = 7.8 Hz, 1H), 8.03 (d, *J* = 7.6 Hz, 1H), 7.86 - 7.74 (m, 1H), 7.18 - 7.11 (m, 3H), 6.76 (dd, *J* = 6.0, 2.8 Hz, 2H), 6.65 (t, *J* = 8.3 Hz, 3H), 6.43 (d, *J* = 8.5 Hz, 2H), 6.33 - 6.24 (m, 2H), 5.18 (dd, *J* = 12.8, 5.4 Hz, 1H), 4.56 (s, 2H), 4.32 (t, *J* = 11.2 Hz, 1H), 4.19 (dd, *J*= 10.1, 3.7 Hz, 2H), 3.67 (d, *J* = 11.7 Hz, 2H), 3.57 - 3.49 (m, 2H), 3.17 - 2.98 (m, 4H), 2.92 - 2.86 (m, 1H), 2.67 - 2.52 (m, 3H), 2.06 (dd, *J* = 10.4, 5.3 Hz, 1H). LCMS (ESI) calcd for C₃₉H₃₇N₄O₆⁺ [M+H]⁺: 657.26, found, 657.30.

### Example 325: preparation of the following compounds

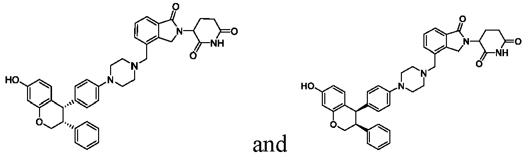

Referring to the method of Scheme 11, one of the intermediates prepared in Intermediate Example 94 was reacted with 3-(4-(bromomethyl)-1-oxoisoindolin-2-yl)piperidine-2,6-dione to obtain the corresponding target compound.

The structural characterization data of the target compound prepared are as follows:
One of the two target compounds (GT-03802) (white solid,49 mg, yield 54%): ¹H NMR (400 MHz, MeOD) δ 7.95 (d, *J* = 7.4 Hz, 1H), 7.88 (d, *J* = 7.4 Hz, 1H), 7.70 (t, *J* = 7.7 Hz, 1H), 7.14 - 7.09 (m, 3H), 6.76 - 6.70 (m, 4H), 6.67 (d, *J* = 8.3 Hz, 1H), 6.54 (d, *J* = 8.6 Hz, 2H), 6.36 (d, *J* = 2.4 Hz, 1H), 6.30 (dd, J = 8.3, 2.4 Hz, 1H), 5.22 (dd, *J* = 13.3, 5.2 Hz, 1H), 4.92 - 4.87 (m, 1H), 4.77 (d, *J* = 17.3 Hz, 1H), 4.66 (d, *J* = 17.4 Hz, 1H), 4.51 (s, 2H), 4.40 (t, *J* = 11.1 Hz, 1H), 4.23 (d, *J* = 5.3 Hz, 1H), 4.18 (dd, *J* = 10.7, 2.5 Hz, 1H), 3.73 - 3.42 (m, 6H), 3.17 - 3.00 (m, 2H), 2.98 - 2.88 (m, 1H), 2.85 - 2.75 (m, 1H), 2.52 (qd, *J* = 13.2, 4.7 Hz, 1H), 2.27 - 2.16 (m, 1H). LCMS (ESI) m/z: calcd for C₃₉H₃₉N₄O₅⁺[M+H]⁺, 643.29; found, 643.3.
another target compound (GT-03787) (white solid, 55 mg, yield 66.15%): ¹H NMR (400 MHz, DMSO) δ 11.38 (s, 1H), 11.07 (s, 1H), 8.02 (d, *J* = 7.5 Hz, 1H), 7.83 (d, *J* = 7.5 Hz, 1H), 7.63 (t, *J* = 7.6 Hz, 1H), 7.20 - 7.09 (m, 3H), 6.82 - 6.72 (m, 2H), 6.65 (dd, *J* = 8.2, 6.5 Hz, 3H), 6.43 (d, *J* = 8.5 Hz, 2H), 6.37 - 6.24 (m, 2H), 5.18 (dd, *J* = 13.2, 5.1 Hz, 1H), 4.89 (d, *J* = 17.5 Hz, 1H), 4.52 (d, *J*= 17.6 Hz, 1H), 4.42 (s, 2H), 4.32 (t, *J* = 11.1 Hz, 1H), 4.19 (dd, *J =* 10.5, 3.6 Hz, 2H), 3.66 (d, *J* = 11.8 Hz, 2H), 3.56 - 3.49 (m, 3H), 3.25 - 3.05 (m, 4H), 3.00 - 2.88 (m, 1H), 2.64 (d, *J* = 16.9 Hz, 1H), 2.41 - 2.23 (m, 1H), 2.10 - 1.98 (m, 1H). LCMS (ESI) calcd for C₃₉H₃₉N₄O₅⁺ [M+H]⁺: 643.28, found, 643.30.

### Example 326: preparation of the following compounds

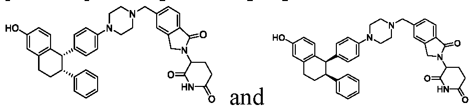

Referring to the method of Step 2 in Scheme 11 and the method of Example 1, one of the intermediates prepared in Intermediate Example 93 was reacted with 3-(5-(bromomethyl)-1-oxoisoindolin-2-yl)piperidine-2,6-dione (GT-03234) to obtain the corresponding target compound.

The structural characterization data of the target compound prepared are as follows:
One of the two target compounds (GT-03949) (white solid,46 mg, yield 51%): ¹H NMR (400 MHz, DMSO) δ 11.00 (s, 1H), 10.71 (s, 1H), 9.12 (s, 1H), 7.89 -7.81 (m, 2H), 7.73 (d, *J* = 8.2 Hz, 1H), 7.19 - 7.07 (m, 3H), 6.83 (d, *J* = 6.6 Hz, 2H), 6.66 - 6.53 (m, 4H), 6.48 (dd, *J* = 8.3, 2.4 Hz, 1H), 6.25 (d, *J* = 8.6 Hz, 2H), 5.14 (dd, *J* = 13.4, 5.1 Hz, 1H), 4.56 - 4.45 (m, 3H), 4.38 (d, *J* = 17.7 Hz, 1H), 4.16 (d, *J* = 4.9 Hz, 1H), 3.68 - 3.60 (m, 2H), 3.35 - 3.32 (m, 1H), 3.27 - 3.05 (m, 3H), 3.00 - 2.84 (m, 5H), 2.64 - 2.58 (m, 1H), 2.45 - 2.36 (m, 1H), 2.12 - 1.96 (m, 2H), 1.77 - 1.66 (m, 1H). LCMS (ESI) m/z: calcd for C₄₀H₄₁N₄O₄⁺[M+H]⁺, 641.31; found, 641.3.
another target compound (GT-03850) (white solid, 63.00 mg, yield 75.61%): ¹H NMR (400 MHz, DMSO) δ 11.29 (s, 1H), 11.00 (s, 1H), 7.89 (s, 1H), 7.79 (dd, *J =* 17.8, 7.8 Hz, 2H), 7.17 - 7.08 (m, 3H), 6.83 (d, *J* = 6.7 Hz, 2H), 6.60 (dd, *J* = 20.5, 8.5 Hz, 4H), 6.48 (dd, *J* = 8.2, 2.4 Hz, 1H), 6.25 (d, *J* = 8.6 Hz, 2H), 5.14 (dd, *J* = 13.2, 5.1 Hz, 1H), 4.44 (q, *J* = 17.6 Hz, 4H), 4.16 (d, *J* = 4.7 Hz, 1H), 3.62 (d, *J* = 11.8 Hz, 2H), 3.36 - 3.27 (m, 3H), 3.13 - 2.88 (m, 7H), 2.61 (d, *J* = 16.7 Hz, 1H), 2.45 - 2.35 (m, 1H), 2.11 - 1.96 (m, 2H), 1.70 (d, *J=* 7.0 Hz, 1H). LCMS (ESI) calcd for C₄₀H₄₁N₄O₄⁺ [M+H]⁺: 641.30, found, 641.30.

### Example 327: preparation of the following compounds

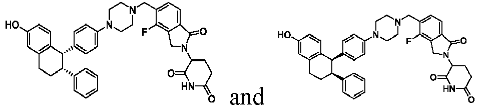

Referring to the method of Scheme 11, one of the intermediates prepared in Intermediate Example 93 was reacted with 3-(4-fluoro-5-(hydroxymethyl)-1-oxoisoindolin-2-yl)piperidine-2,6-dione (GT-03445) to obtain the corresponding target compound.

The structural characterization data of the target compound prepared are as follows:
One of the two target compounds (GT-03950) (white solid,57 mg, yield 62%): ¹H NMR (400 MHz, DMSO) δ 11.02 (s, 1H), 10.66 (s, 1H), 9.12 (s, 1H), 7.87 (t, *J* = 6.9 Hz, 1H), 7.71 (d, *J* = 7.7 Hz, 1H), 7.20 - 7.07 (m, 3H), 6.83 (d, *J* = 6.5 Hz, 2H), 6.66 - 6.54 (m, 4H), 6.48 (dd, *J* = 8.3, 2.5 Hz, 1H), 6.25 (d, *J* = 8.6 Hz, 2H), 5.15 (dd, *J* = 13.2, 5.2 Hz, 1H), 4.60 (d, *J* = 17.7 Hz, 1H), 4.49 - 4.39 (m, 1H), 4.16 (d, *J* = 4.8 Hz, 1H), 3.71 - 3.60 (m, 2H), 3.33 - 3.12 (m, 4H), 3.06 - 2.85 (m, 6H), 2.70 - 2.58 (m, 2H), 2.17 - 1.94 (m, 3H), 1.77 - 1.65 (m, 1H). LCMS (ESI) m/z: calcd for C₄₀H₃₉FN₄O₄⁺[M+H]⁺, 658.30; found, 658.3.
another target compound (GT-03851) (white solid, 66.00 mg, yield 77.05%): ¹H NMR (400 MHz, DMSO) δ 11.22 (s, 1H), 11.02 (s, 1H), 7.95 (t, *J* = 6.9 Hz, 1H), 7.69 (d, *J* = 7.7 Hz, 1H), 7.19 - 7.05 (m, 3H), 6.83 (d, *J* = 6.7 Hz, 2H), 6.60 (dd, *J* = 18.2, 8.5 Hz, 4H), 6.48 (dd, *J* = 8.2, 2.5 Hz, 1H), 6.25 (d, *J* = 8.6 Hz, 2H), 5.15 (dd, *J* = 13.2, 5.1 Hz, 1H), 4.63 - 4.41 (m, 4H), 4.16 (d, *J* = 4.8 Hz, 1H), 3.63 (d, *J* = 13.7 Hz, 2H), 3.32 - 3.27 (m, 1H), 3.18 (s, 2H), 3.06 - 2.86 (m, 5H), 2.61 (dd, *J* = 16.5, 1.7 Hz, 1H), 2.47 - 2.38 (m, 1H), 2.12 - 1.97 (m, 2H), 1.70 (dd, *J* = 11.0, 4.5 Hz, 1H). LCMS (ESI) calcd for C₄₀H₄₀FN₄O₄⁺ [M+H]⁺: 659.30, found, 659.30.

### Example 328: preparation of the following compounds

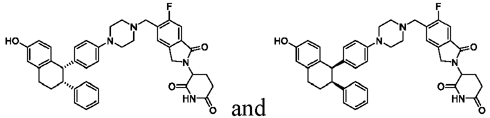

Referring to the method of Scheme 11, one of the intermediates prepared in Intermediate Example 93 was reacted with 3-(6-fluoro-5-(hydroxymethyl)-1-oxoisoindolin-2-yl)piperidine-2,6-dione to obtain the corresponding target compound.

The structural characterization data of the target compound prepared are as follows:
One of the two target compounds (GT-03951) (white solid,53 mg, yield 57%): ¹H NMR (400 MHz, DMSO) δ 11.02 (s, 1H), 10.72 (s, 1H), 9.12 (s, 1H), 7.97 (d, *J* = 5.3 Hz, 1H), 7.69 (d, *J* = 8.3 Hz, 1H), 7.18 - 7.08 (m, 3H), 6.83 (d, *J* = 6.6 Hz, 2H), 6.66 - 6.56 (m, 4H), 6.48 (dd, *J* = 8.3, 2.4 Hz, 1H), 6.25 (d, *J* = 8.6 Hz, 2H), 5.14 (dd, *J* = 13.2, 5.1 Hz, 1H), 4.49 - 4.33 (m, 2H), 4.16 (d, *J* = 4.9 Hz, 1H), 3.71 - 3.58 (m, 2H), 3.35 - 3.13 (m, 4H), 3.04 - 2.85 (m, 5H), 2.66 - 2.55 (m, 2H), 2.44 - 2.35 (m, 1H), 2.14 - 1.97 (m, 2H), 1.77 - 1.65 (m, 1H). LCMS (ESI) m/z: calcd for C₄₀H₃₉FN₄O⁺[M+H]⁺, 658.30; found, 658.3.
another target compound (GT-03852) (white solid, 67.00 mg, yield 78.22%): ¹H NMR (400 MHz, DMSO) δ 11.26 (s, 1H), 11.02 (s, 1H), 8.05 (d, *J* = 6.0 Hz, 1H), 7.67 (d, *J* = 8.5 Hz, 1H), 7.20 - 7.07 (m, 3H), 6.83 (d, *J* = 6.7 Hz, 2H), 6.60 (dd, *J* = 19.6, 8.5 Hz, 4H), 6.48 (dd, *J* = 8.2, 2.4 Hz, 1H), 6.25 (d, *J* = 8.6 Hz, 2H), 5.14 (dd, *J* = 13.2, 5.0 Hz, 1H), 4.43 (dd, *J* = 49.3, 17.0 Hz, 4H), 4.16 (d, *J* = 4.8 Hz, 1H), 3.63 (d, *J* = 11.0 Hz, 2H), 3.30 (dd, *J* = 13.4, 4.0 Hz, 3H), 3.18 (s, 2H), 3.06 - 2.85 (m, 5H), 2.61 (d, *J* = 16.9 Hz, 1H), 2.46 - 2.34 (m, 1H), 2.12 - 1.94 (m, 2H), 1.70 (dd, *J* = 11.4, 4.0 Hz, 1H). LCMS (ESI) calcd for C₄₀H₄₀FN₄O₄⁺ [M+H]⁺: 659.30, found, 659.30.

### Example 329: preparation of the following compounds

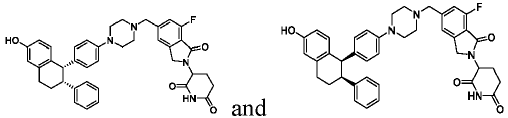

Referring to the method of Scheme 11, one of the intermediates prepared in Intermediate Example 93 was reacted with 3-(7-fluoro-5-(hydroxymethyl)-1-oxoisoindolin-2-yl)piperidine-2,6-dione (GT-03446) to obtain the corresponding target compound.

The structural characterization data of the target compound prepared are as follows:
One of the two target compounds (GT-03952) (white solid,46 mg, yield 50%): ¹H NMR (400 MHz, DMSO) δ 11.02 (s, 1H), 10.90 (s, 1H), 9.13 (s, 1H), 7.68 - 7.53 (m, 2H), 7.19 - 7.07 (m, 3H), 6.84 (d, *J* = 6.8 Hz, 2H), 6.65 - 6.55 (m, 4H), 6.48 (dd, *J* = 8.3, 2.4 Hz, 1H), 6.25 (d, *J* = 8.4 Hz, 2H), 5.11 (dd, *J* = 13.3, 5.0 Hz, 1H), 4.53 (d, *J* = 18.2 Hz, 1H), 4.40 (d, *J* = 18.1 Hz, 1H), 4.16 (d, *J* = 4.7 Hz, 1H), 3.71 - 3.58 (m, 2H), 3.36 - 3.26 (m, 2H), 3.18 - 3.05 (m, 2H), 3.04 - 2.85 (m, 5H), 2.65 - 2.58 (m, 1H), 2.44 - 2.34 (m, 1H), 2.17 - 1.97 (m, 2H), 1.76 - 1.68 (m, 1H). LCMS (ESI) m/z: calcd for C₄₀H₃₉FN₄O₄⁺[M+H]⁺, 658.30; found, 658.3.
another target compound (GT-03853) (white solid, 65.00 mg, yield 75.88%): ¹H NMR (400 MHz, DMSO) δ 11.42 (s, 1H), 11.01 (s, 1H), 7.77 - 7.61 (m, 2H), 7.20 - 7.05 (m, 3H), 6.84 (d, *J* = 6.8 Hz, 2H), 6.60 (dd, *J* = 19.3, 8.5 Hz, 4H), 6.48 (dd, *J* = 8.3, 2.4 Hz, 1H), 6.25 (d, *J* = 8.6 Hz, 2H), 5.10 (dd, *J* = 13.2, 5.1 Hz, 1H), 4.56 - 4.35 (m, 4H), 4.16 (d, *J* = 4.8 Hz, 1H), 3.62 (d, *J* = 11.3 Hz, 2H), 3.36 - 3.24 (m, 3H), 3.15 - 3.00 (m, 4H), 2.98 - 2.83 (m, 3H), 2.60 (d, *J* = 16.5 Hz, 1H), 2.43 - 2.33 (m, 1H), 2.12 - 1.95 (m, 2H), 1.71 (dd, *J* = 11.4, 4.6 Hz, 1H). LCMS (ESI) calcd for C₄₀H₄₀FN₄O₄⁺ [M+H]⁺: 659.30, found, 659.30.

### Example 330: preparation of the following compounds

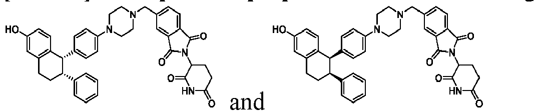

Referring to the method of Step 2 in Scheme 11 and the method of Example 1, one of the intermediates prepared in Intermediate Example 93 was reacted with 5-(bromomethyl)-2-(2,6-dioxo-piperidin-3-yl)isoindoline-1,3-dione (CAS NO.: 1312023-72-5) to obtain the corresponding target compound.

The structural characterization data of the target compound prepared are as follows:
One of the two target compounds (GT-03955) (white solid,16 mg, yield 17%): ¹H NMR (400 MHz, DMSO) δ 11.15 (s, 1H), 11.11 (s, 1H), 9.12 (s, 1H), 8.18 (s, 1H), 8.05 (s, 2H), 7.18 - 7.07 (m, 3H), 6.84 (d, *J* = 6.6 Hz, 2H), 6.65 - 6.54 (m, 4H), 6.48 (dd, *J* = 8.3, 2.6 Hz, 1H), 6.25 (d, *J* = 8.5 Hz, 2H), 5.18 (dd, *J* = 12.7, 5.4 Hz, 1H), 4.58 (s, 2H), 4.16 (d, *J* = 4.9 Hz, 1H), 3.73 - 3.59 (m, 2H), 3.35 - 3.26 (m, 3H), 3.20 - 3.07 (m, 2H), 3.02 - 2.82 (m, 6H), 2.65 - 2.53 (m, 2H), 2.13 - 2.00 (m, 2H), 1.76 - 1.67 (m, 1H). LCMS (ESI) m/z: calcd for C₄₀H₃₉N₄O₅⁺[M+H]⁺, 655.29; found, 655.3.
another target compound (GT-03855) (white solid, 54.00 mg, yield 63.42%): ¹H NMR (400 MHz, DMSO) δ 11.48 (s, 1H), 11.14 (s, 1H), 8.26 (s, 1H), 8.14 (d, *J* = 7.8 Hz, 1H), 8.02 (d, *J* = 7.6 Hz, 1H), 7.21 - 7.04 (m, 3H), 6.83 (d, *J* = 6.8 Hz, 2H), 6.60 (dd, *J* = 18.3, 8.5 Hz, 4H), 6.48 (dd, *J* = 8.2, 2.4 Hz, 1H), 6.25 (d, *J* = 8.6 Hz, 2H), 5.18 (dd, *J* = 12.8, 5.4 Hz, 1H), 4.56 (s, 2H), 4.16 (d, *J* = 4.9 Hz, 1H), 3.63 (d, *J* = 11.8 Hz, 2H), 3.31 (ddd, *J* = 14.8, 10.4, 7.8 Hz, 3H), 3.14 - 2.83 (m, 7H), 2.61 (d, *J* = 15.3 Hz, 1H), 2.14 - 2.01 (m, 2H), 1.71 (dd, *J =* 11.6, 4.9 Hz, 1H). LCMS (ESI) calcd for C₄₀H₃₉N₄O₅⁺ [M+H]⁺: 655.28, found, 655.30.

### Example 331: preparation of the following compounds

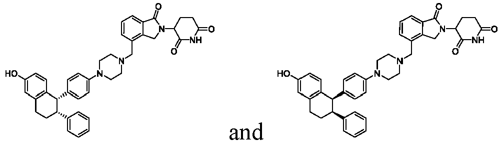

Referring to the method of Step 2 in Scheme 11 and the method of Example 1, one of the intermediates prepared in Intermediate Example 93 was reacted with 3-(4-(bromomethyl)-1-oxoisoindolin-2-yl)piperidine-2,6-dione to obtain the corresponding target compound.

The structural characterization data of the target compound prepared are as follows:
One of the two target compounds (GT-03956) (white solid,51 mg, yield 57%): ¹H NMR (400 MHz, DMSO) δ 11.05 (s, 1H), 10.61 (s, 1H), 9.12 (s, 1H), 7.92 (d, *J* = 6.9 Hz, 1H), 7.84 (d, *J* = 7.6 Hz, 1H), 7.65 (t, *J* = 7.5 Hz, 1H), 7.19 - 7.06 (m, 3H), 6.83 (d, *J* = 6.4 Hz, 2H), 6.65 - 6.56 (m, 4H), 6.48 (dd, *J* = 8.3, 2.5 Hz, 1H), 6.25 (d, *J* = 8.5 Hz, 2H), 5.18 (dd, *J* = 13.2, 5.1 Hz, 1H), 4.77 (d, *J* = 17.2 Hz, 1H), 4.51 (d, *J* = 17.4 Hz, 1H), 4.46 - 4.36 (m, 2H), 4.16 (d, *J* = 4.7 Hz, 1H), 3.74 - 3.60 (m, 2H), 3.35 - 3.17 (m, 4H), 3.02 - 2.87 (m, 5H), 2.68 - 2.62 (m, 1H), 2.36 - 2.26 (m, 1H), 2.15 - 2.01 (m, 2H), 1.76 - 1.67 (m, 1H). LCMS (ESI) m/z: calcd for C₄₀H₄₁N₄O₄⁺[M+H]⁺, 641.31; found, 641.3.
another target compound (GT-03854) (white solid, 62.00 mg, yield 74.41%): ¹H NMR (400 MHz, DMSO) δ 11.26 (s, 1H), 11.05 (s, 1H), 8.00 (d, *J* = 7.6 Hz, 1H), 7.83 (d, *J* = 7.5 Hz, 1H), 7.63 (t, *J* = 7.6 Hz, 1H), 7.19 - 7.05 (m, 3H), 6.83 (d, *J* = 6.7 Hz, 2H), 6.66 - 6.54 (m, 4H), 6.48 (dd, *J* = 8.3, 2.4 Hz, 1H), 6.25 (d, *J* = 8.6 Hz, 2H), 5.18 (dd, *J* = 13.2, 5.1 Hz, 1H), 4.87 (d, *J* = 17.5 Hz, 1H), 4.52 (d, *J* = 17.6 Hz, 1H), 4.41 (s, 2H), 4.16 (d, *J* = 4.8 Hz, 1H), 3.63 (d, *J* = 11.8 Hz, 2H), 3.38 - 3.27 (m, 3H), 3.24 - 3.15 (m, 2H), 3.13 - 3.04 (m, 2H), 3.01 - 2.86 (m, 3H), 2.64 (d, *J* = 17.1 Hz, 1H), 2.38 - 2.27 (m, 1H), 2.13 - 1.96 (m, 2H), 1.71 (dd, *J* = 11.4, 4.6 Hz, 1H). LCMS (ESI) calcd for C₄₀H₄₁N₄O₄⁺ [M+H]⁺: 641.30, found, 641.30.

### Example 332: preparation of 3-(4-fluoro-5-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-1-oxoisoindolin-2-yl)piperidine-2,6-dione (GT-03592)

Referring to the method of Scheme 11, the target compound (GT-03592) was prepared using 5-fluoro-4-(4-fluoro-1-isopropyl-2-methyl-1H-benzo[d]imidazol-6-yl)-N-(5-(piperazin-1-ylmethyl)pyridin-2-yl)pyrimidin-2-amine (CAS NO.: 1231930-57-6) and 3-(4-fluoro-5-(hydroxymethyl)-1-oxoisoindolin-2-yl)piperidine-2,6-dione (GT-03445). The target compound (GT-03592) was obtained as a white solid (55 mg, yield 63.7%). ¹H NMR (400 MHz, MeOD) δ 8.81 (d, *J* = 3.2 Hz, 1H), 8.50 (s, 1H), 8.42 - 8.30 (m, 2H), 8.10 (d, *J* = 11.0 Hz, 1H), 7.77 - 7.69 (m, 1H), 7.65 (d, *J* = 7.7 Hz, 1H), 7.55 (d, *J* = 9.0 Hz, 1H), 5.06 (dt, *J* = 14.2, 6.1 Hz, 2H), 4.66 - 4.32 (m, 4H), 3.95 (s, 2H), 3.36 (s, 4H), 3.19 - 2.84 (m, 7H), 2.84 - 2.75 (m, 1H), 2.69 (d, *J* = 15.7 Hz, 1H), 2.50 - 2.36 (m, 1H), 2.15 - 2.04 (m, 1H), 1.72 (d, *J* = 6.9 Hz, 6H). LCMS (ESI) calcd for C₃₉H₄₀F₃N₁₀O₃⁺ [M+H]⁺: 753.32, found, 753.3.

### Example 333: preparation of 3-(6-fluoro-5-((4-((6-((5-fluoro-4-(4-fluoro-1-isopropyl-2-methyl-1H-benzo[d] imidazol-6-yl)pyrimidin-2-yl)amino)pyridin-3-yl)methyl)piperazin-1-yl)methyl)-1-oxoisoindolin-2-yl)piperidine-2,6-dione (GT-03593)

The target compound (GT-03593) was prepared using the method described in Scheme 11 (white solid, 45 mg, yield 63.6%). ¹H NMR (400 MHz, MeOD) δ 8.80 (d, *J* = 3.0 Hz, 1H), 8.48 (s, 1H), 8.40 - 8.26 (m, 2H), 8.08 (d, *J* = 11.2 Hz, 1H), 7.80 (d, *J* = 6.0 Hz, 1H), 7.55 (d, *J* = 8.6 Hz, 2H), 5.13 - 5.00 (m, 2H), 4.45 (dd, *J* = 29.9, 20.4 Hz, 4H), 3.85 (s, 2H), 3.27 (d, *J* = 19.8 Hz, 4H), 3.12 - 2.62 (m, 9H), 2.42 (tt, *J* = 13.5, 6.8 Hz, 1H), 2.15 - 2.01 (m, 1H), 1.72 (d, *J* = 6.9 Hz, 6H). LCMS (ESI) calcd for C₃₉H₄₀F₃N₁₀O₃⁺ [M+H]⁺: 753.32, found, 753.3.

### Example 334: preparation of 3-(7-fluoro-5-((4-((6-((5-fluoro-4-(4-fluoro-1-isopropyl-2-methyl-1H-benzo[d] imidazol-6-yl)pyrimidin-2-yl)amino)pyridin-3-yl)methyl)piperazin-1-yl)methyl)-1-oxoisoindolin-2-yl)piperidine-2,6-dione (GT-03594)

Referring to the method of Scheme 11, the target compound (GT-03594) was prepared using 5-fluoro-4-(4-fluoro-1-isopropyl-2-methyl-1H-benzo[d]imidazol-6-yl)-N-(5-(piperazin-1-ylmethyl)pyridin-2-yl)pyrimidin-2-amine (CAS NO.: 1231930-57-6) and 3-(7-fluoro-5-(hydroxymethyl)-1-oxoisoindolin-2-yl)piperidine-2,6-dione (GT-03446). The target compound (GT-03594) was obtained as a white solid (35 mg, yield 50.5%). ¹H NMR (400 MHz, MeOD) δ 8.81 (d, *J* = 2.9 Hz, 1H), 8.50 (s, 1H), 8.40 - 8.27 (m, 2H), 8.10 (d, *J* = 11.2 Hz, 1H), 7.55 (d, *J* = 8.9 Hz, 2H), 7.37 (d, *J* = 9.8 Hz, 1H), 5.11 - 5.00 (m, 2H), 4.54 - 4.42 (m, 2H), 4.34 (s, 2H), 3.93 (s, 2H), 3.33 (d, *J* = 39.0 Hz, 4H), 3.11 - 2.63 (m, 9H), 2.40 (dt, *J* = 13.3, 8.8 Hz, 1H), 2.16 - 2.00 (m, 1H), 1.72 (d, *J* = 6.9 Hz, 6H). LCMS (ESI) calcd for C₃₉H₄₀F₃N₁₀O₃⁺ [M+H]⁺: 753.32, found, 753.4.

### Example 335: preparation of 3-(5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-7-fluoro-1-oxoisoindolin-2-yl)piperidine-2,6-dione (GT-03491)

Referring to the method of Scheme 11, the target compound (GT-03491) was prepared using (2-((5-chloro-2-((2-methoxy-4-(4-(methylamino)piperidin-1-yl)phenyl)amino)pyrimidin-4-yl)amino)phenyl)dimethylphosphine oxide (CAS NO.: 2353496-90-7) and 3-(7-fluoro-5-(hydroxymethyl)-1-oxoisoindolin-2-yl)piperidine-2,6-dione (GT-03446). The target compound (GT-03491) was obtained as a white solid (40 mg, yield 49.9%). ¹H NMR (400 MHz, MeOD) δ 8.41 - 8.11 (m, 1H), 7.97 (s, 1H), 7.57 (dt, *J* = 33.6, 17.8 Hz, 3H), 7.39 (d, *J* = 9.5 Hz, 1H), 7.31 (t, *J* = 7.5 Hz, 1H), 7.21 (s, 1H), 6.71 (s, 1H), 6.57 (d, *J* = 7.2 Hz, 1H), 5.09 - 5.02 (m, 1H), 4.66 - 4.59 (m, 1H), 4.48 (t, *J* = 13.0 Hz, 2H), 4.31 (s, 1H), 3.91 (d, *J*= 13.1 Hz, 2H), 3.77 (s, 3H), 3.54 (s, 1H), 2.95 - 2.78 (m, 3H), 2.70 (d, *J* = 22.0 Hz, 4H), 2.41 (dd, *J* = 13.4, 4.4 Hz, 1H), 2.23 (d, *J* = 11.3 Hz, 2H), 2.10 (s, 1H), 2.00 (d, *J* = 9.6 Hz, 2H), 1.79 (d, *J* = 13.6 Hz, 6H). LCMS (ESI) calcd for C₃₉H₄₄ClFN₈O₅P⁺ [M+H]⁺: 789.28, found, 789.3.

### Example 336: preparation of 3-(5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-4-fluoro-1-oxoisoindolin-2-yl)piperidine-2,6-dione (GT-03512)

Referring to the method of Scheme 11, the target compound (GT-03512) was prepared using (2-((5-chloro-2-((2-methoxy-4-(4-(methylamino)piperidin-1-yl)phenyl)amino)pyrimidin-4-yl)amino)phenyl)dimethylphosphine oxide (CAS NO.: 2353496-90-7) and 3-(4-fluoro-5-(hydroxymethyl)-1-oxoisoindolin-2-yl)piperidine-2,6-dione (GT-03445). The target compound (GT-03512) was obtained as a white solid (50 mg, yield 62.4%). ¹H NMR (400 MHz, MeOD) δ 8.19 (s, 1H), 8.05 (s, 1H), 7.79 - 7.73 (m, 1H), 7.69 (d, *J* = 7.7 Hz, 1H), 7.62 (dd, *J* = 14.0, 7.8 Hz, 1H), 7.53 (t, *J* = 7.9 Hz, 1H), 7.45 - 7.31 (m, 2H), 7.05 (s, 1H), 6.84 (d, *J* = 9.0 Hz, 1H), 5.10 (dd, *J* = 13.3, 5.2 Hz, 1H), 4.72 - 4.65 (m, 1H), 4.56 (t, *J* = 15.3 Hz, 2H), 4.40 (s, 1H), 3.88 (d, *J* = 12.2 Hz, 2H), 3.83 (s, 3H), 3.77 (s, 1H), 3.27 (d, *J* = 31.1 Hz, 2H), 2.88 - 2.79 (m, 4H), 2.74 - 2.66 (m, 1H), 2.51 - 2.42 (m, 1H), 2.32 (d, *J* = 28.8 Hz, 4H), 2.15 - 2.07 (m, 1H), 1.79 (d, *J* = 13.6 Hz, 6H). LCMS (ESI) calcd for C₃₉H₄₄ClFN₈O₅P⁺ [M+H]⁺: 789.28, found, 789.3.

### Example 337: preparation of 3-(4-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-1-oxoisoindolin-2-yl)piperidine-2,6-dione (GT-03650)

The target compound (GT-03650) was prepared using the method described in Scheme 11 (white solid, 40 mg, yield 63.5%). ¹H NMR (400 MHz, MeOD) δ 8.35 (s, 1H), 8.13 (s, 1H), 7.99 (d, *J* = 7.5 Hz, 1H), 7.92 (d, *J* = 7.5 Hz, 1H), 7.80 - 7.69 (m, 2H), 7.62 (d, *J* = 7.2 Hz, 1H), 7.45 (t, *J* = 7.1 Hz, 2H), 7.02 (s, 1H), 6.83 (d, *J* = 8.0 Hz, 1H), 5.27 (dd, *J* = 13.3, 5.1 Hz, 1H), 4.70 (d, *J* = 17.3 Hz, 2H), 4.39 (s, 1H), 4.02 (d, *J* = 11.7 Hz, 2H), 3.92 (s, 3H), 3.85 (s, 1H), 3.24 (s, 2H), 3.07 - 2.73 (m, 6H), 2.59 (qd, *J* = 13.3, 4.9 Hz, 1H), 2.42 (s, 2H), 2.37 - 2.22 (m, 3H), 1.91 (d, *J* = 13.6 Hz, 6H). LCMS (ESI) calcd for C₃₉H₄₅ClN₈O₅P⁺ [M+H]⁺: 771.29, found, 771.3.

### Example 338: preparation of 2-((2-((4-(4-(4-((2-(2,6-dioxopiperidin-3-yl)-1-oxoisoindolin-4-yl)methyl)piperazin-1-yl)piperidin-1-yl)-2-methoxyphenyl)amino)-5-(trifluoromethyl)pyridin-4-yl)amino)-N-methylbenzamide (GT-03645)

The target compound (GT-03645) was prepared using the method described in Scheme 11 (white solid, 35 mg, yield 55.6%). ¹H NMR (400 MHz, MeOD) δ 8.02 (s, 1H), 7.88 (d, *J* = 7.5 Hz, 1H), 7.77 (dd, *J* = 22.1, 7.4 Hz, 2H), 7.63 (dd, *J* = 14.4, 5.7 Hz, 3H), 7.43 - 7.32 (m, 1H), 7.20 (d, *J* = 8.7 Hz, 1H), 6.89 (s, 1H), 6.78 (d, *J* = 7.6 Hz, 1H), 6.49 (s, 1H), 5.23 (dd, *J* = 13.2, 5.2 Hz, 1H), 4.70 (t, *J*= 24.2 Hz, 2H), 4.19 (s, 2H), 3.98 (d, *J* = 12.9 Hz, 2H), 3.88 (s, 3H), 3.64 - 3.34 (m, 9H), 3.03 (d, J= 12.5 Hz, 2H), 2.96 - 2.78 (m, 5H), 2.57 (dd, *J* = 13.0, 4.5 Hz, 1H), 2.32 (d, *J* = 11.7 Hz, 2H), 2.24 (d, *J* = 5.4 Hz, 1H), 2.00 (d, *J*= 9.6 Hz, 2H). LCMS (ESI) calcd for C₄₄H₄₉F₃N₉O₅⁺ [M+H]⁺: 840.38, found, 840.4.

### Example 339: preparation of N-(3-(((2-((4-(4-(((2-(2,6-dioxopiperidin-3-yl)-1-oxoisoindolin-5-yl)methyl)(methyl)amino)piperidin-1-yl)phenyl)amino)-5-(trifluoromethyl)pyrimidin-4-yl)amino)methyl)phenyl)-N-methylmethanesulfonamide (GT-03677)

Referring to the method of Step 2 in Scheme 11 and the method of Example 1, the target compound (GT-03677) was prepared using N-methyl-N-(3-(((2-((4-(4-(methylamino)piperidin-1-yl)phenyl)amino)-5-(trifluoromethyl)pyrimidin-4-yl)amino)methyl)phenyl)methanesulfonamide (GT-M-153) prepared from Intermediate Example 32 and 3-(5-(bromomethyl)-1-oxoisoindolin-2-yl)piperidine-2,6-dione (GT-03234). The target compound (GT-03677) was obtained as a white solid (16 mg, yield 26.3%). ¹H NMR (400 MHz, MeOD) δ 8.11 (s, 1H), 7.84 (d, *J* = 7.8 Hz, 1H), 7.76 (s, 1H), 7.65 (d, *J* = 7.7 Hz, 1H), 7.23 (ddd, *J* = 42.0, 23.6, 8.3 Hz, 8H), 5.09 (dd, *J* = 13.3, 5.1 Hz, 1H), 4.61 (s, 2H), 4.48 (t, *J* = 12.7 Hz, 2H), 4.35 (s, 1H), 3.85 (d, *J* = 11.6 Hz, 2H), 3.58 (s, 1H), 3.27 (s, 1H), 3.16 (s, 3H), 3.03 (s, 2H), 2.86 - 2.67 (m, 8H), 2.43 (dt, *J* = 12.7, 8.3 Hz, 1H), 2.28 (d, *J* = 12.0 Hz, 2H), 2.18 - 2.03 (m, 3H). LCMS (ESI) calcd for C₄₀H₄₅F₃N₉O₅S⁺ [M+H]⁺: 820.32, found, 820.3.

### Example 340: preparation of N-(3-(((2-((4-(4-(4-((2-(2,6-dioxopiperidin-3-yl)-1-oxoisoindolin-5-yl)methyl)piperazin-1-yl)piperidin-1-yl)phenyl)amino)-5-(trifluoromethyl)pyrimidin-4-yl)amino)methyl)phenyl)-N-methylmethanesulfonamide (GT-03678)

The target compound (GT-03678) was prepared using the method described in Scheme 11 (white solid, 25 mg, yield 42.2%). ¹H NMR (400 MHz, MeOD) δ 8.11 (s, 1H), 7.79 (d, *J* = 7.8 Hz, 1H), 7.71 (s, 1H), 7.61 (d, *J* = 8.1 Hz, 1H), 7.34 - 7.08 (m, 8H), 5.08 (dd, *J* = 13.2, 5.2 Hz, 1H), 4.62 (s, 2H), 4.46 (q, *J* = 17.5 Hz, 2H), 4.25 (s, 2H), 3.81 (d, *J* = 12.3 Hz, 2H), 3.31 (d, *J* = 59.8 Hz, 9H), 3.16 (s, 3H), 3.08 (s, 2H), 2.86 - 2.66 (m, 5H), 2.49 - 2.35 (m, 1H), 2.21 (d, *J* = 12.1 Hz, 2H), 2.14 - 2.05 (m, 1H), 1.96 (d, *J* = 10.7 Hz, 2H). LCMS (ESI) calcd for C₄₃H₅₀F₃N₁₀O₅S⁺ [M+H]⁺: 875.36, found, 875.4.

### Example 341: preparation of N-(3-(((2-((4-(4-((2-(2,6-dioxopiperidin-3-yl)-1-oxoisoindolin-5-yl)methyl)piperazin-1-yl)phenyl)amino)-5-(trifluoromethyl)pyrimidin-4-yl)amino)methyl)pyrazin-2-yl)-N-methylmethanesulfonamide (GT-03672)

Referring to the method of Step 2 in Scheme 11 and the method of Example 1, the target compound (GT-03672) was prepared using N-methyl-N-(3-(((2-((4-(piperazin-1-yl)phenyl)amino)-5-(trifluoromethyl)pyrimidin-4-yl)amino)methyl)pyrazin-2-yl)methanesulfonamide (GT-M-151) prepared from Intermediate Example 30 and 3-(5-(bromomethyl)-1-oxoisoindolin-2-yl)piperidine-2,6-dione (GT-03234). The target compound (GT-03672) was obtained as a white solid (30 mg, yield 48.8%). ¹H NMR (400 MHz, MeOD) δ 8.50 (d, *J* = 2.5 Hz, 1H), 8.45 (d, *J* = 2.4 Hz, 1H), 8.16 (s, 1H), 7.88 - 7.75 (m, 2H), 7.67 (d, *J* = 7.9 Hz, 1H), 7.22 (d, *J* = 8.8 Hz, 2H), 6.94 (s, 2H), 5.09 (dd, *J* = 13.3, 5.1 Hz, 1H), 5.01 (s, 2H), 4.57 - 4.42 (m, 4H), 3.79 (d, *J* = 13.0 Hz, 2H), 3.51 (dd, *J* = 14.0, 6.9 Hz, 2H), 3.33 - 3.23 (m, 2H), 3.05 (d, *J* = 32.0 Hz, 8H), 2.87 - 2.75 (m, 1H), 2.75 - 2.62 (m, 1H), 2.43 (qd, *J* = 13.1, 4.6 Hz, 1H), 2.17 - 2.03 (m, 1H). LCMS (ESI) calcd for C₃₆H₃₉F₃N₁₁O₅S⁺ [M+H]⁺: 794.28, found, 794.3.

### Example 342: preparation of 2-((2-((4-(4-(1-((2-(2,6-dioxopiperidin-3-yl)-1-oxoisoindolin-5-yl)methyl)piperidin-4-yl)piperazin-1-yl)-2-isopropoxy-5-methylphenyl)amino)-5-(trifluoromethyl)pyridin-4-yl)amino)-N-methylbenzamide (GT-04137)

The target compound (GT-04137) was prepared using the methods described in Scheme 11 and Example 1 (white solid, 12 mg, yield 27.08%). ¹H NMR (400 MHz, MeOD) δ 7.94 (s, 1H), 7.84 (d, *J* = 7.9 Hz, 1H), 7.78 (s, 1H), 7.64 (dd, *J* = 12.3, 7.8 Hz, 2H), 7.49 (d, *J* = 3.8 Hz, 2H), 7.32 - 7.22 (m, 1H), 7.04 (s, 1H), 6.77 (s, 1H), 6.40 (s, 1H), 5.08 (dt, *J* = 11.6, 5.8 Hz, 1H), 4.59 - 4.40 (m, 5H), 3.62 (d, *J* = 9.2 Hz, 5H), 3.22 (s, 4H), 3.17 (s, 4H), 2.94 - 2.81 (m, 1H), 2.79 (s, 3H), 2.70 (dd, *J =* 15.3, 2.4 Hz, 1H), 2.50 - 2.36 (m, 3H), 2.26 - 2.0 5 (m, 6H), 1.15 (d, *J* = 6.0 Hz, 6H). LCMS (ESI) calcd for C₄₇H₅₅F₃N₉O₅⁺ [M+H]⁺: 882.43, found, 882.4.

### Example 343: preparation of 2-((2-((4-(4-(((2-(2,6-dioxopiperidin-3-yl)-1-oxoisoindolin-5-yl)methyl)(methyl)amino)piperidin-1-yl)-2-isopropoxy-5-methylphenyl)amino)-5-(trifluoromethyl)pyridin-4-yl)amino)-N-methylbenzamide (GT-04090)

The target compound (GT-04090) was prepared using the methods described in Scheme 11 and Example 1 (yellow solid, 30 mg, yield 38.7%). ¹H NMR (400 MHz, MeOD) δ 7.94 (s, 1H), 7.84 (d, *J* = 7.8 Hz, 1H), 7.76 (s, 1H), 7.69 - 7.57 (m, 2H), 7.53 - 7.41 (m, 2H), 7.30 - 7.18 (m, 1H), 7.02 (s, 1H), 6.71 (s, 1H), 6.42 (s, 1H), 5.14 - 5.04 (m, 1H), 4.73 - 4.59 (m, 1H), 4.58 - 4.41 (m, 3H), 4.38 (d, *J* = 47.0 Hz, 1H), 3.52 - 3.37 (m, 1H), 3.20 (s, 2H), 2.88 - 2.63 (m, 10H), 2.43 (qd, *J* = 13.2, 4.6 Hz, 1H), 2.25 - 1.95 (m, 8H), 1.14 (d, *J* = 6.0 Hz, 6H). LCMS (ESI) calcd for C₄₄H₅₀F₃N₈O₅⁺ [M+H]⁺: 827.39, found, 827.4.

### Example 344: preparation of 2-((2-((4-(4-(((2-(2,6-dioxopiperidin-3-yl)-4-fluoro-1-oxoisoindolin-5-yl)methyl)(methyl)amino)piperidin-1-yl)-2-isopropoxy-5-methylphenyl)amino)-5-(trifluoromethyl)pyridin-4-yl)amino)-N-methylbenzamide (GT-04091)

The target compound (GT-04091) was prepared using the method described in Scheme 11 (yellow solid, 30 mg, yield 37.9%). ¹H NMR (400 MHz, MeOD) δ 7.95 (s, 1H), 7.78 - 7.66 (m, 2H), 7.66 - 7.58 (m, 1H), 7.51 - 7.41 (m, 2H), 7.28 - 7.21 (m, 1H), 7.02 (s, 1H), 6.72 (s, 1H), 6.43 (s, 1H), 5.17 - 5.03 (m, 1H), 4.72 - 4.65 (m, 1H), 4.64 - 4.48 (m, 3H), 4.46 - 4.23 (m, 1H), 3.50 (t, *J* = 12.1 Hz, 1H), 3.23 (d, *J* = 6.2 Hz, 2H), 2.91 - 2.66 (m, 10H), 2.44 (qd, *J* = 13.2, 4.9 Hz, 1H), 2.30 - 1.95 (m, 8H), 1.15 (d, *J* = 6.0 Hz, 6H). LCMS (ESI) calcd for C₄₄H₄₉F₄N₈O₅⁺ [M+H]⁺: 845.38, found, 845.4.

### Example 345: preparation of 2-((2-((4-(4-(((2-(2,6-dioxopiperidin-3-yl)-6-fluoro-1-oxoisoindolin-5-yl)methyl)(methyl)amino)piperidin-1-yl)-2-isopropoxy-5-methylphenyl)amino)-5-(trifluoromethyl)pyridin-4-yl)amino)-N-methylbenzamide (GT-04092)

The target compound (GT-04092) was prepared using the method described in Scheme 11 (yellow solid, 30 mg, yield 37.9%). ¹H NMR (400 MHz, MeOD) δ 7.95 (s, 1H), 7.83 (d, *J* = 6.1 Hz, 1H), 7.61 (dd, *J*= 8.5, 7.0 Hz, 2H), 7.53 - 7.40 (m, 2H), 7.27 - 7.18 (m, 1H), 7.03 (s, 1H), 6.71 (d, *J* = 7.8 Hz, 1H), 6.43 (s, 1H), 5.13 - 5.03 (m, 1H), 4.71 - 4.23 (m, 5H), 3.50 (t, *J* = 12.2 Hz, 1H), 3.23 (d, *J* = 6.1 Hz, 2H), 2.83 - 2.65 (m, 10H), 2.43 (qd, *J* = 13.2, 4.7 Hz, 1H), 2.28 - 1.97 (m, 8H), 1.19 - 1.09 (m, 6H). LCMS (ESI) calcd for C₄₄H₄₉F₄N₈O₅ ⁺ [M+H]⁺: 845.38, found, 845.4.

### Example 346: preparation of 2-((2-((4-(4-(((2-(2,6-dioxopiperidin-3-yl)-7-fluoro-1-oxoisoindolin-5-yl)methyl)(methyl)amino)piperidin-1-yl)-2-isopropoxy-5-methylphenyl)amino)-5-(trifluoromethyl)pyridin-4-yl)amino)-N-methylbenzamide (GT-04093)

The target compound (GT-04093) was prepared using the method described in Scheme 11 (yellow solid, 30 mg, yield 37.9%). ¹H NMR (400 MHz, MeOD) δ 7.94 (s, 1H), 7.61 (dd, *J* = 9.4, 8.3 Hz, 2H), 7.52 - 7.45 (m, 2H), 7.41 (d, *J* = 9.7 Hz, 1H), 7.28 - 7.19 (m, 1H), 7.02 (s, 1H), 6.71 (s, 1H), 6.42 (s, 1H), 5.06 (dd, *J* = 13.3, 5.2 Hz, 1H), 4.61 (s, 1H), 4.57 - 4.44 (m, 3H), 4.28 (d, *J* = 44.9 Hz, 1H), 3.46 (t, *J* = 12.1 Hz, 1H), 3.20 (d, *J* = 4.7 Hz, 2H), 2.88 - 2.66 (m, 10H), 2.41 (qd, *J* = 13.2, 4.6 Hz, 1H), 2.25 - 1.95 (m, 8H), 1.14 (d, *J* = 6.0 Hz, 6H). LCMS (ESI) calcd for C₄₄H₄₉F₄N₈O₅ ⁺ [M+H]⁺: 845.38, found, 845.4.

### Example 347: preparation of 2-((2-((4-(4-(((2-(2,6-dioxopiperidin-3-yl)-1-oxoisoindolin-4-yl)methyl)(methyl)amino)piperidin-1-yl)-2-isopropoxy-5-methylphenyl)amino)-5-(trifluoromethyl)pyridin-4-yl)amino)-N-methylbenzamide (GT-04097)

The target compound (GT-04097) was prepared using the methods described in Scheme 11 and Example 1 (yellow solid, 30 mg, yield 38.7%). ¹H NMR (400 MHz, MeOD) δ 7.94 (s, 1H), 7.88 (d, *J* = 7.6 Hz, 1H), 7.79 (d, *J* = 7.7 Hz, 1H), 7.63 (dt, *J* = 7.5, 3.7 Hz, 2H), 7.49 (d, *J* = 3.8 Hz, 2H), 7.33 - 7.22 (m, 1H), 7.00 (s, 1H), 6.72 (d, *J* = 9.0 Hz, 1H), 6.40 (s, 1H), 5.20 - 5.09 (m, 1H), 4.65 - 4.49 (m, 4H), 4.25 (s, 1H), 3.54 (s, 1H), 3.24 (s, 1H), 2.97 - 2.61 (m, 11H), 2.47 (dt, *J* = 13.2, 11.0 Hz, 1H), 2.29 - 2.00 (m, 8H), 1.14 (d, *J* = 6.0 Hz, 6H). LCMS (ESI) calcd for C₄₄H₅₀F₃N₈O₅⁺ [M+H]⁺: 827.39, found, 827.4.

### Example 348: preparation of 2-((2-((4-(4-(((2-(2,6-dioxopiperidin-3-yl)-1,3-dioxoisoindolin-5-yl)methyl)(methyl)amino)piperidin-1-yl)-2-isopropoxy-5-methylphenyl)amino)-5-(trifluoromethyl)pyridin-4-yl)amino)-N-methylbenzamide (GT-04096)

The target compound (GT-04096) was prepared using the methods described in Scheme 11 and Example 1 (yellow solid, 30 mg, yield 38.1%). ¹H NMR (400 MHz, MeOD) δ 8.05 (s, 1H), 8.01 - 7.87 (m, 3H), 7.61 (d, *J* = 8.1 Hz, 1H), 7.51 - 7.38 (m, 2H), 7.23 (dd, *J* = 11.2, 5.0 Hz, 1H), 7.03 (s, 1H), 6.70 (s, 1H), 6.41 (s, 1H), 5.10 (dd, *J* = 12.7, 5.4 Hz, 1H), 4.60 - 4.28 (m, 4H), 3.46 (s, 1H), 2.88 - 2.54 (m, 12H), 2.19 (d, *J* = 18.8 Hz, 5H), 2.12 - 1.94 (m, 3H), 1.14 (d, *J* = 6.0 Hz, 6H). LCMS (ESI) calcd for C₄₄H₄₈F₃N₈O₆⁺ [M+H]⁺: 841.36, found, 841.4.

### Example 349: preparation of N-(3-(difluoromethyl)-1-(1-((2-(2,6-dioxopiperidin-3-yl)-1-oxoisoindolin-5-yl)methyl)piperidin-4-yl)-1H-pyrazol-4-yl)-2-(2-((2,2,2-trifluoroethyl)amino)pyridin-4-yl)oxazole-4-carboxamide (GT-04120)

Referring to the method of Step 2 in Scheme 11 and the method of Example 1, the target compound (GT-04120) was prepared using tert-butyl (4-(4-((3-(difluoromethyl)-1-(piperidin-4-yl)-1H-pyrazol-4-yl)carbamoyl)oxazol-2-yl)pyridin-2-yl)(2,2,2-trifluoroethyl)carbamate (GT-D-126) prepared from Intermediate Example 72 and 3-(5-(bromomethyl)-1-oxoisoindolin-2-yl)piperidine-2,6-dione (GT-03234). The target compound (GT-04120) was obtained as a white solid (25 mg, yield 37.0%). ¹H NMR (400 MHz, MeOD) δ 8.72 (s, 1H), 8.33 (s, 1H), 8.20 (d, *J* = 6.0 Hz, 1H), 7.97 (d, *J* = 8.0 Hz, 1H), 7.82 (s, 1H), 7.75 (d, *J* = 8.0 Hz, 1H), 7.47 (s, 1H), 7.42 (d, *J* = 4.5 Hz, 1H), 6.89 (d, *J* = 54.0 Hz, 1H), 5.22 (dd, *J*= 13.4, 5.2 Hz, 1H), 4.65 - 4.52 (m, 5H), 4.32 - 4.21 (m, 2H), 3.71 (s, 2H), 3.50 (s, 1H), 3.06 (d, *J* = 7.7 Hz, 1H), 2.94 (s, 1H), 2.84 (s, 1H), 2.54 (d, *J* = 17.4 Hz, 1H), 2.42 (s, 4H), 2.23 (s, 1H). LCMS (ESI) calcd for C₃₄H₃₃F₅N₉O₅ [M+H]⁺: 742.25, found, 742.3.

### Example 350: preparation of N-(3-(difluoromethyl)-1-(1-((2-(2,6-dioxopiperidin-3-yl)-4-fluoro-1-oxoisoindolin-5-yl)methyl)piperidin-4-yl)-1H-pyrazol-4-yl)-2-(2-((2,2,2-trifluoroethyl)amino)pyridin-4-yl)oxazole-4-carboxamide (GT-04098)

Referring to the method of Scheme 11, the target compound (GT-04098) was prepared using tert-butyl (4-(4-((3-(difluoromethyl)-1-(piperidin-4-yl)-1H-pyrazol-4-yl)carbamoyl)oxazol-2-yl)pyridin-2-yl)(2,2,2-trifluoroethyl)carbamate (GT-D-126) prepared from Intermediate Example 72 and 3-(4-fluoro-5-(hydroxymethyl)-1-oxoisoindolin-2-yl)piperidine-2,6-dione (GT-03445). The target compound (GT-04098) was obtained as a white solid (25 mg, yield 26.2%). ¹H NMR (400 MHz, MeOD) δ 8.83 (s, 1H), 8.36 (s, 1H), 8.23 (d, *J* = 6.3 Hz, 1H), 7.94 - 7.80 (m, 2H), 7.72 (s, 1H), 7.60 (d, *J* = 6.3 Hz, 1H), 6.99 (t, *J* = 54.4 Hz, 1H), 5.25 (dd, *J* = 13.4, 5.2 Hz, 1H), 4.80 - 4.59 (m, 5H), 4.37 (q, *J* = 8.9 Hz, 2H), 3.76 (t, *J* = 42.7 Hz, 2H), 3.41 (d, *J* = 29.0 Hz, 2H), 2.98 (ddd, *J* = 18.5, 13.4, 5.3 Hz, 1H), 2.91 - 2.82 (m, 1H), 2.70 - 2.36 (m, 5H), 2.31 - 2.20 (m, 1H). LCMS (ESI) calcd for C₃₄H₃₂F₆N₉O₅ ⁺ [M+H]⁺: 760.24, found, 760.3.

### Example 351: preparation of N-(3-(difluoromethyl)-1-(1-((2-(2,6-dioxopiperidin-3-yl)-6-fluoro-1-oxoisoindolin-5-yl)methyl)piperidin-4-yl)-1H-pyrazol-4-yl)-2-(2-((2,2,2-trifluoroethyl)amino)pyridin-4-yl)oxazole-4-carboxamide (GT-04119)

The target compound (GT-04119) was prepared using the method described in Scheme 11 (white solid, 25 mg, yield 26.2%). ¹H NMR (400 MHz, MeOD) δ 8.98 (s, 1H), 8.51 (s, 1H), 8.37 (d, *J* = 6.3 Hz, 1H), 8.13 (d, *J* = 6.1 Hz, 1H), 7.90 (d, *J* = 12.3 Hz, 2H), 7.76 (d, *J* = 6.1 Hz, 1H), 7.14 (t, *J* = 54.3 Hz, 1H), 5.39 (dd, *J* = 13.2, 5.2 Hz, 1H), 4.94 - 4.65 (m, 5H), 4.52 (q, *J* = 9.0 Hz, 2H), 3.91 (t, *J* = 40.4 Hz, 2H), 3.58 (d, *J* = 34.7 Hz, 2H), 3.20 - 3.05 (m, 1H), 3.00 (d, *J* = 15.7 Hz, 1H), 2.85 - 2.52 (m, 5H), 2.41 (d, *J* = 13.1 Hz, 1H). LCMS (ESI) calcd for C₃₄H₃₂F₆N₉O₅ ⁺ [M+H]⁺: 760.24, found, 760.3.

### Example 352: preparation of N-(3-(difluoromethyl)-1-(1-((2-(2,6-dioxopiperidin-3-yl)-7-fluoro-1-oxoisoindolin-5-yl)methyl)piperidin-4-yl)-1H-pyrazol-4-yl)-2-(2-((2,2,2-trifluoroethyl)amino)pyridin-4-yl)oxazole-4-carboxamide (GT-04099)

Referring to the method of Scheme 11, the target compound (GT-04099) was prepared using tert-butyl (4-(4-((3-(difluoromethyl)-1-(piperidin-4-yl)-1H-pyrazol-4-yl)carbamoyl)oxazol-2-yl)pyridin-2-yl)(2,2,2-trifluoroethyl)carbamate (GT-D-126) prepared from Intermediate Example 72 and 3-(7-fluoro-5-(hydroxymethyl)-1-oxoisoindolin-2-yl)piperidine-2,6-dione (GT-03446). The target compound (GT-04099) was obtained as a white solid (25 mg, yield 26.2%). ¹H NMR (400 MHz, MeOD) δ 8.75 (s, 1H), 8.33 (s, 1H), 8.19 (d, *J* = 6.0 Hz, 1H), 7.66 (s, 1H), 7.57 (s, 1H), 7.54 - 7.44 (m, 2H), 6.95 (t, *J* = 54.6 Hz, 1H), 5.17 (dd, *J* = 13.3, 5.2 Hz, 1H), 4.61 (dd, *J* = 26.9, 18.4 Hz, 5H), 4.29 (q, *J* = 9.1 Hz, 2H), 3.67 (t, *J* = 38.8 Hz, 2H), 3.42 (d, *J* = 64.8 Hz, 2H), 2.94 (ddd, *J* = 18.5, 13.4, 5.3 Hz, 1H), 2.87 - 2.77 (m, 1H), 2.63 - 2.36 (m, 5H), 2.28 - 2.16 (m, 1H). LCMS (ESI) calcd for C₃₄H₃₂F₆N₉O₅ ⁺ [M+H]⁺: 760.24, found, 760.3.

### Example 353: preparation of N-(3-(difluoromethyl)-1-(1-((2-(2,6-dioxopiperidin-3-yl)-1-oxoisoindolin-4-yl)methyl)piperidin-4-yl)-1H-pyrazol-4-yl)-2-(2-((2,2,2-trifluoroethyl)amino)pyridin-4-yl)oxazole-4-carboxamide (GT-04089)

The target compound (GT-04089) was prepared using the method described in Scheme 11 (white solid, 25 mg, yield 37.0%). ¹H NMR (400 MHz, MeOD) δ 8.61 (s, 1H), 8.21 (s, 1H), 8.08 (d, *J* = 5.9 Hz, 1H), 7.86 (t, *J* = 7.5 Hz, 1H), 7.82 - 7.75 (m, 1H), 7.62 (t, *J* = 7.6 Hz, 1H), 7.40 (s, 1H), 7.33 (d, *J* = 5.9 Hz, 1H), 6.83 (t, *J* = 54.3 Hz, 1H), 5.14 (dd, *J* = 13.3, 5.1 Hz, 1H), 4.64 (dd, *J* = 42.9, 17.4 Hz, 3H), 4.43 (s, 2H), 4.16 (q, *J* = 9.2 Hz, 2H), 3.56 (d, *J* = 70.4 Hz, 2H), 3.37 (dd, *J* = 13.4, 11.8 Hz, 2H), 2.94 - 2.79 (m, 1H), 2.72 (d, *J* = 15.7 Hz, 1H), 2.53 - 2.22 (m, 5H), 2.19 - 2.08 (m, 1H). LCMS (ESI) calcd for C₃₄H₃₃F₅N₉O₅⁺ [M+H]⁺: 742.25, found, 742.3.

### Example 354: preparation of N-(3-(difluoromethyl)-1-(1-((2-(2,6-dioxopiperidin-3-yl)-1,3-dioxoisoindolin-5-yl)methyl)piperidin-4-yl)-1H-pyrazol-4-yl)-2-(2-((2,2,2-trifluoroethyl)amino)pyridin-4-yl)oxazole-4-carboxamide (GT-04088)

Referring to the method of Scheme 11, the target compound (GT-04088) was prepared using tert-butyl (4-(4-((3-(difluoromethyl)-1-(piperidin-4-yl)-1H-pyrazol-4-yl)carbamoyl)oxazol-2-yl)pyridin-2-yl)(2,2,2-trifluoroethyl)carbamate (GT-D-126) prepared from Intermediate Example 72 and 5-(bromomethyl)-2-(2,6-dioxo-piperidin-3-yl)isoindoline-1,3-dione (CAS NO.: 1312023-72-5). The target compound (GT-04088) was obtained as a white solid (25 mg, yield 36.4%). ¹H NMR (400 MHz, MeOD) δ 8.60 (s, 1H), 8.21 (s, 1H), 8.09 (d, *J* = 5.8 Hz, 1H), 8.03 (s, 1H), 7.95 (s, 2H), 7.36 (s, 1H), 7.30 (d, *J* = 5.8 Hz, 1H), 6.84 (t, *J* = 54.8 Hz, 1H), 5.10 (dd, *J* = 12.6, 5.5 Hz, 1H), 4.51 (s, 3H), 4.14 (q, *J* = 9.2 Hz, 2H), 3.56 (t, *J* = 38.6 Hz, 2H), 3.39 - 3.22 (m, 2H), 2.80 (ddd, *J* = 17.8, 14.4, 5.1 Hz, 1H), 2.67 (ddd, *J* = 13.4, 7.8, 4.6 Hz, 2H), 2.31 (s, 4H), 2.11 - 2.03 (m, 1H). LCMS (ESI) calcd for C₃₄H₃₁F₅N₉O₆⁺ [M+H]⁺: 756.23, found, 756.2.

### Example 355: preparation of N-((2-(2,6-dioxopiperidin-3-yl)-1-oxoisoindolin-5-yl)methyl)-4,9-dioxo-4,9-dihydronaphtho[2,3-b]furan-2-carboxamide (GT-03383)

Referring to the method of Scheme 56, the target compound (GT-03383) was prepared using 4,9-dioxo-4,9-dihydronaphtho[2,3-b]furan-2-carboxylic acid (GT-D-200) prepared from Intermediate Example 78 and 3-(5-(aminomethyl)-1-oxoisoindolin-2-yl)piperidine-2,6-dione (CAS NO.: 1010100-28-3). The target compound (GT-03383) was obtained as a grey solid (32 mg, yield 51.9%). ¹H NMR (400 MHz, DMSO) δ 10.97 (s, 1H), 9.62 (t, *J* = 6.1 Hz, 1H), 8.20 - 8.06 (m, 2H), 7.99 - 7.86 (m, 2H), 7.78 - 7.66 (m, 2H), 7.58 (s, 1H), 7.51 (t, *J* = 8.6 Hz, 1H), 5.11 (dd, *J* = 13.3, 5.1 Hz, 1H), 4.62 (d, *J* = 6.0 Hz, 2H), 4.39 (dd, *J* = 54.6, 17.4 Hz, 2H), 2.97 - 2.85 (m, 1H), 2.63 (t, *J* = 18.2 Hz, 1H), 2.44 - 2.33 (m, 1H), 2.07 -1.95 (m, 1H). LCMS (ESI) calcd for C₂₇H₂₀N₃O₇⁺ [M+H]⁺: 498.13, found, 498.1.

### Example 356: preparation of N-((1r,4r)-4-(3-chloro-4-cyanophenoxy)cyclohexyl)-6-(4-((4-((2-(2,6-dioxopiperidin-3-yl)-1-oxoisoindolin-5-yl)methyl)piperidin-1-yl)methyl)piperidin-1-yl)pyridazine-3-carboxamide (GT-03522)

The target compound (GT-03522) was prepared using the method described in Scheme 59 (yellow solid, 53 mg, yield 62.52%). ¹H NMR (400 MHz, DMSO) δ 10.99 (s, 1H), 8.61 - 8.58 (m, 1H), 7.85 (d, *J* = 8.7 Hz, 2H), 7.67 (d, *J* = 7.7 Hz, 1H), 7.45 (d, *J* = 10.6 Hz, 2H), 7.37 (t, *J* = 6.0 Hz, 2H), 7.13 (d, *J* = 6.5 Hz, 1H), 5.11 (dd, *J* = 13.2, 5.0 Hz, 1H), 4.50 - 4.44 (m, 3H), 4.30 (d, *J* = 17.4 Hz, 1H), 3.51 - 3.43 (m, 2H), 3.17 (d, *J* = 8.1 Hz, 1H), 3.06 (t, *J* = 11.5 Hz, 3H), 2.93 - 2.88 (m, 2H), 2.68 - 2.58 (m, 2H), 2.40 (dd, *J* = 13.3, 4.4 Hz, 1H), 2.10 (d, *J* = 9.9 Hz, 4H), 2.01 - 1.86 (m, 7H), 1.72 - 1.50 (m, 9H), 1.31 - 1.22 (m, 3H). LCMS (ESI) calcd for C₄₃H₅₀ClN₈O₅⁺ [M+H]⁺: 793.35, found, 793.30.

### Example 357: preparation of N-((1r,4r)-4-(3-chloro-4-cyanophenoxy)cyclohexyl)-6-(4-((3-((2-(2,6-dioxopiperidin-3-yl)-1-oxoisoindolin-5-yl)methylene)azetidin-1-yl)methyl)piperidin-1-yl)pyridazine-3-carboxamide (GT-03541)

The target compound (GT-03541) was prepared using the method described in Scheme 59 (yellow solid, 44 mg, yield 53.95%). ¹H NMR (400 MHz, DMSO) δ 11.00 (s, 1H), 8.61 (d, *J* = 8.1 Hz, 1H), 7.85 (dd, *J* = 9.2, 2.4 Hz, 2H), 7.72 (d, *J* = 7.9 Hz, 1H), 7.44 (d, *J* = 5.2 Hz, 2H), 7.38 (d, *J* = 2.1 Hz, 1H), 7.33 (d, *J* = 7.8 Hz, 1H), 7.13 (dd, *J* = 8.8, 2.1 Hz, 1H), 6.62 (s, 1H), 5.33 - 5.21 (m, 1H), 5.19 - 5.09 (m, 2H), 5.07 - 4.98 (m, 1H), 4.84 (d, *J* = 15.9 Hz, 1H), 4.54 - 4.42 (m, 4H), 4.34 (dd, *J* = 17.4, 3.9 Hz, 1H), 3.28 (t, *J* = 5.2 Hz, 2H), 3.03 (t, *J* = 12.1 Hz, 2H), 2.93 (dd, *J* = 22.1, 8.8 Hz, 1H), 2.60 (d, *J* = 16.5 Hz, 1H), 2.43 - 2.31 (m, 1H), 2.07 (dd, *J* = 28.2, 7.3 Hz, 4H), 1.90 (d, *J* = 12.3 Hz, 4H), 1.65 - 1.49 (m, 4H), 1.35 - 1.16 (m, 3H). LCMS (ESI) calcd for C₄₁H₄₄ClN₈O₅⁺ [M+H]⁺: 763.30, found, 763.30.

### Example 358: preparation of N-((1r,4r)-4-(3-chloro-4-cyanophenoxy)cyclohexyl)-6-(4-((3-((1S)-(2-(2,6-dioxopiperidin-3-yl)-1-oxoisoindolin-5-yl)(hydroxy)methyl)-3-hydroxyazetidin-1-yl)methyl)piperidin-1-yl)pyridazine-3-carboxamide (GT-03581)

The target compound (GT-03581) was prepared using the method described in Scheme 59 (yellow solid, 44 mg, yield 51.65%). ¹H NMR (400 MHz, DMSO) δ 10.99 (s, 1H), 8.60 (d, *J* = 8.2 Hz, 1H), 7.86 (dd, *J* = 9.2, 4.5 Hz, 2H), 7.68 (dd, *J* = 8.5, 4.6 Hz, 2H), 7.61 (t, *J* = 6.8 Hz, 1H), 7.48 (d, *J* = 9.7 Hz, 1H), 7.38 (d, *J* = 2.2 Hz, 1H), 7.13 (dd, *J* = 8.8, 2.2 Hz, 1H), 5.20 - 5.08 (m, 2H), 4.49 (dd, *J* = 9.9, 3.5 Hz, 4H), 4.33 (dd, *J* = 16.4, 9.9 Hz, 3H), 4.21 (dd, *J* = 10.5, 4.9 Hz, 1H), 4.11 - 4.04 (m, 1H), 3.92 - 3.79 (m, 3H), 3.20 - 3.13 (m, 2H), 3.04 (t, *J* = 12.4 Hz, 2H), 2.96 - 2.85 (m, 1H), 2.60 (d, *J* = 16.7 Hz, 1H), 2.47 - 2.26 (m, 1H), 2.06 (dd, *J* = 36.8, 8.2 Hz, 4H), 1.88 (t, *J* = 10.6 Hz, 4H), 1.68 - 1.50 (m, 4H), 1.32 - 1.22 (m, 2H). LCMS (ESI) calcd for C41H46ClN8O7⁺ [M+H]⁺: 797.31, found, 797.30.

### Example 359: preparation of N-((1r,3r)-3-(3-chloro-4-cyanophenoxy)-2,2,4,4-tetramethylcyclobutyl)-6-(4-((4-((2-(2,6-dioxopiperidin-3-yl)-1-oxoisoindolin-5-yl)methyl)piperidin-1-yl)methyl)piperidin-1-yl)nicotinamide (GT-03584)

The target compound (GT-03584) was prepared using the method described in Scheme 59 (yellow solid, 36 mg, yield 43.44%). ¹H NMR (400 MHz, DMSO) δ 10.99 (s, 1H), 8.56 (s, 1H), 8.24 (d, *J*= 8.8 Hz, 1H), 8.06 (d, *J* = 8.0 Hz, 1H), 7.90 (d, *J*= 8.7 Hz, 1H), 7.67 (dd, *J* = 7.6, 4.3 Hz, 1H), 7.44 (s, 1H), 7.34 (dd, *J* = 17.5, 9.6 Hz, 2H), 7.21 (d, *J* = 2.1 Hz, 1H), 7.02 - 6.99 (m, 1H), 5.11 (dd, *J* = 13.2, 5.0 Hz, 1H), 4.45 (d, *J* = 7.4 Hz, 3H), 4.34 (d, *J* = 13.1 Hz, 2H), 4.07 (d, *J* = 9.1 Hz, 2H), 3.52 - 3.43 (m, 2H), 3.23 - 3.13 (m, 3H), 2.95 - 2.79 (m, 4H), 2.64 (dd, *J* = 28.3, 11.0 Hz, 2H), 2.44 - 2.34 (m, 1H), 2.22 (dd, *J* = 11.4, 6.8 Hz, 1H), 1.98 (dd, *J* = 14.1, 9.9 Hz, 4H), 1.77 (dt, *J* = 46.5, 15.2 Hz, 4H), 1.23 (s, 8H), 1.12 (s, 6H). LCMS (ESI) calcd for C₄₆H₅₅ClN₇O₅⁺ [M+H]⁺: 820.39, found, 820.40.

### Example 360: preparation of N-((1r,3r)-3-(3-chloro-4-cyanophenoxy)-2,2,4,4-tetramethylcyclobutyl)-6-(4-((3-((2-(2,6-dioxopiperidin-3-yl)-1-oxoisoindolin-5-yl)methylene)azetidin-1-yl)methyl)piperidin-1-yl)nicotinamide (GT-03586)

The target compound (GT-03586) was prepared using the method described in Scheme 59 (yellow solid, 47 mg, yield 58.88%). ¹H NMR (400 MHz, DMSO) δ 11.50 (s, 1H), 11.00 (s, 1H), 8.59 (d, *J* = 1.6 Hz, 1H), 8.18 (d, *J* = 8.4 Hz, 1H), 7.93 (dd, *J* = 20.9, 8.0 Hz, 2H), 7.73 (d, *J* = 7.9 Hz, 1H), 7.44 (s, 1H), 7.33 (d, *J* = 7.7 Hz, 1H), 7.21 (d, *J* = 2.2 Hz, 2H), 7.01 (dd, *J* = 8.8, 2.1 Hz, 1H), 6.63 (s, 1H), 5.27 (d, *J* = 15.1 Hz, 1H), 5.22 - 5.08 (m, 2H), 5.03 (d, *J* = 14.6 Hz, 1H), 4.84 (d, *J* = 14.7 Hz, 1H), 4.52 - 4.41 (m, 3H), 4.34 (dd, *J* = 16.2, 5.0 Hz, 2H), 4.07 (d, *J* = 9.1 Hz, 1H), 3.27 (d, *J* = 5.6 Hz, 2H), 3.10 (t, *J*= 11.8 Hz, 2H), 2.96 - 2.87 (m, 1H), 2.60 (d, *J* = 16.5 Hz, 1H), 2.46 - 2.34 (m, 1H), 2.00 (ddd, *J* = 34.8, 23.3, 8.8 Hz, 4H), 1.33 - 1.20 (m, 8H), 1.13 (s, 6H). LCMS (ESI) calcd for C₄₄H₄₉ClN₇O₅⁺ [M+H]⁺: 790.34, found, 790.30.

### Example 361: preparation of 7-cyclopentyl-2-((5-(4-((4-((2-(2,6-dioxopiperidin-3-yl)-1-oxoisoindolin-5-yl)methyl)piperidin-1-yl)methyl)piperidin-1-yl)pyridin-2-yl)amino)-N,N-dimethyl-7H-pyrrolo[2,3-d]pyrimidine-6-carboxamide (GT-03663)

The target compound (GT-03663) was prepared using the method described in Scheme 59 (yellow solid, 36 mg, yield 42.23%). ¹H NMR (400 MHz, DMSO) δ 11.64 (s, 1H), 10.99 (s, 1H), 10.52 (s, 1H), 9.02 (s, 1H), 8.21 (dd, *J* = 9.4, 2.4 Hz, 1H), 8.08 (s, 1H), 7.66 (dd, *J* = 8.5, 5.4 Hz, 2H), 7.50-7.41 (m, 1H), 7.36 (dd, *J* = 14.7, 7.9 Hz, 1H), 6.84 (s, 1H), 5.11 (dd, *J* = 13.3, 5.0 Hz, 1H), 4.80 (s, 1H), 4.43 (d, *J* = 11.3 Hz, 2H), 4.32 (s, 2H), 3.72 - 3.63 (m, 3H), 3.57 - 3.37 (m, 3H), 3.05 (s, 7H), 2.91 (dd, *J* = 10.9, 6.6 Hz, 3H), 2.67 (d, *J* = 4.2 Hz, 1H), 2.34 - 2.24 (m, 3H), 2.17 - 1.91 (m, 12H), 1.85 - 1.77 (m, 2H), 1.64 (d, *J* = 5.9 Hz, 2H), 1.49 - 1.41 (m, 2H). LCMS (ESI) calcd for C₄₄H₅₅N₁₀O₄⁺ [M+H]⁺: 787.43, found, 787.50.

### Example 362: preparation of 7-cyclopentyl-2-((5-(4-((3-((2-(2,6-dioxopiperidin-3-yl)-1-oxoisoindolin-5-yl)methyl)azetidin-1-yl)methyl)piperidin-1-yl)pyridin-2-yl)amino)-N,N-dimethyl-7H-pyrrolo[2,3-d]pyrimidine-6-carboxamide (GT-03674)

The target compound (GT-03674) was prepared using the method described in Scheme 59 (yellow solid, 10 mg, yield 45.84%). ¹H NMR (400 MHz, DMSO) δ 11.56 (s, 1H), 10.99 (s, 1H), 9.00 (s, 1H), 8.15 (dd, *J* = 9.1, 1.9 Hz, 1H), 7.94 (s, 1H), 7.68 (dd, *J* = 7.7, 3.3 Hz, 1H), 7.60 (d, *J* = 9.5 Hz, 1H), 7.47 (d, *J* = 12.9 Hz, 1H), 7.38 (dd, *J* = 15.5, 8.0 Hz, 1H), 6.83 (s, 1H), 5.11 (dd, *J* = 13.2, 4.9 Hz, 1H), 4.84 - 4.77 (m, 1H), 4.44 (d, *J* = 17.4 Hz, 1H), 4.31 (d, *J* = 17.3 Hz, 1H), 4.12 (dd, *J* = 8.9, 5.2 Hz, 2H), 4.06 - 4.01 (m, 2H), 3.67 (d, *J* = 11.5 Hz, 3H), 3.29 (d, *J* = 8.3 Hz, 1H), 3.16 - 3.10 (m, 2H), 3.06 (s, 6H), 2.96 - 2.88 (m, 1H), 2.75 (dd, *J* = 18.0, 7.3 Hz, 2H), 2.60 (dd, *J* = 16.0, 3.1 Hz, 1H), 2.34 (ddd, *J* = 19.4, 12.6, 8.5 Hz, 3H), 2.09 - 1.81 (m, 9H), 1.68 - 1.61 (m, 2H), 1.35 (dd, *J* = 23.5, 12.5 Hz, 2H). LCMS (ESI) calcd for C₄₂H₅₁N₁₀O₄⁺ [M+H]⁺: 759.40, found, 759.50.

### Example 363: preparation of N-(2-chloro-6-methylphenyl)-2-((6-(4-((4-((2-(2,6-dioxopiperidin-3-yl)-1-oxoisoindolin-5-yl)methyl)piperidin-1-yl)methyl)piperidin-1-yl)-2-methylpyrimidin-4-yl)amino)thiazole-5-carboxamide (GT-03539)

The target compound (GT-03539) was prepared using the method described in Scheme 59 (light yellow solid, 10 mg, yield 11.86%). ¹H NMR (400 MHz, DMSO) δ 11.45 (s, 1H), 11.00 (s, 1H), 9.91 (s, 1H), 8.22 (s, 1H), 7.68 (d, *J* = 7.8 Hz, 1H), 7.38 - 7.35 (m, 2H), 7.34 - 7.27 (m, 3H), 6.05 (s, 1H), 5.14 (dd, *J* = 13.2, 5.0 Hz, 1H), 4.39 (dd, *J* = 42.6, 17.3 Hz, 4H), 2.96 - 2.90 (m, 6H), 2.90 - 2.80 (m, 2H), 2.45 - 2.30 (m, 6H), 2.25 (s, 3H), 2.10 (s, 4H), 2.05 - 2.01 (m, 1H), 1.97 - 1.87 (m, 4H), 1.57 - 1.50 (m, 2H), 1.17 - 1.07 (m, 2H). LCMS (ESI) calcd for C₄₁H₄₇ClN₉O₄S⁺ [M+H]⁺: 795.3, found, 796.2.

### Example 364: preparation of 3-(5-((4-(((4-((1R,SS)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)-1-oxoisoindolin-2-yl)piperidine-2,6-dione (GT-04366)

The target compound (GT-04366) was prepared using the method described in Scheme 11 (white solid, 15 mg, yield 56.34%). ¹H NMR (400 MHz, MeOD) δ 9.07 - 8.91 (m, 1H), 7.81 (dd, *J* = 15.7, 8.2 Hz, 2H), 7.70 (d, *J* = 16.1 Hz, 1H), 7.63 (d, *J* = 7.9 Hz, 1H), 7.27 (dd, *J* = 18.7, 9.9 Hz, 2H), 7.14 (s, 1H), 5.08 (dd, *J* = 13.2, 5.0 Hz, 1H), 4.41 (dd, *J* = 33.8, 15.7 Hz, 5H), 4.21 (s, 2H), 3.98 - 3.86 (m, 2H), 3.49 (d, *J* = 14.0 Hz, 2H), 3.36 (dd, *J* = 17.6, 12.1 Hz, 2H), 3.03 (dd, *J* = 28.8, 16.6 Hz, 2H), 2.78 (dt, *J* = 38.5, 14.8 Hz, 2H), 2.43 (d, *J* = 13.0 Hz, 1H), 2.21 - 1.88 (m, 9H), 1.68 (d, *J* = 12.2 Hz, 2H). LCMS (ESI) calcd for C₄₅H₄₃F₂N₈O₅⁺ [M+H]⁺: 813.33, found, 407.3/813.3.

### Example 365: preparation of 3-(5-((4-(((4-((1R,SS)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)-6-fluoro-1-oxoisoindolin-2-yl)piperidine-2,6-dione (GT-04387)

The target compound (GT-04387) was prepared using the method described in Scheme 11 (white solid, 28 mg, yield 99.94%). ¹H NMR (400 MHz, MeOD) δ 9.22 - 8.88 (m, 1H), 7.94 - 7.73 (m, 2H), 7.58 (d, *J* = 8.5 Hz, 1H), 7.42 - 7.15 (m, 3H), 5.08 (dd, *J* = 13.3, 5.0 Hz, 1H), 4.46 (dd, *J* = 26.6, 17.6 Hz, 5H), 4.22 (s, 2H), 4.16 - 3.96 (m, 1H), 3.51 (d, *J* = 10.6 Hz, 4H), 3.43 (d, *J* = 9.4 Hz, 1H), 3.36 (t, *J* = 8.4 Hz, 1H), 3.19 - 2.96 (m, 2H), 2.88 - 2.63 (m, 2H), 2.49 - 2.36 (m, 1H), 2.28 - 2.01 (m, 7H), 1.91 (d, *J*= 14.3 Hz, 1H), 1.76 - 1.38 (m, 2H). LCMS (ESI) calcd for C₄₅H₄₂F₃N₈O₅⁺ [M+H]⁺: 831.32, found, 416.3/831.3.

### Example 366: preparation of 33-(4-((4-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)-1-oxoisoindolin-2-yl)piperidine-2,6-dione (GT-04388)

The target compound (GT-04388) was prepared using the method described in Scheme 11 (white solid, 27 mg, yield 98.33%). ¹H NMR (400 MHz, MeOD) δ 9.19 - 8.99 (m, 1H), 7.92 - 7.73 (m, 3H), 7.60 (t, *J* = 7.6 Hz, 1H), 7.40 - 7.14 (m, 3H), 5.13 (dd, *J* = 13.2, 5.1 Hz, 1H), 4.58 (dd, *J* = 19.3, 7.2 Hz, 1H), 4.37 (t, *J* = 19.7 Hz, 4H), 4.22 (s, 2H), 4.05 - 3.93 (m, 1H), 3.56 (d, *J* = 20.5 Hz, 2H), 3.40 (ddd, *J* = 24.1, 14.0, 6.3 Hz, 3H), 3.04 (s, 2H), 2.80 (dt, *J* = 42.9, 14.6 Hz, 2H), 2.44 (s, 1H), 2.12 (ddd, *J* = 91.2, 37.1, 28.6 Hz, 9H), 1.74 (s, 2H). LCMS (ESI) calcd for C₄₅H₄₃F₂N₈O₅⁺ [M+H]⁺: 813.33, found, 407.3/813.3.

### Example 367: preparation of 3-(5-((4-(6-(5-((R)-2-(2,5-difluorophenyl)pyrrolidin-1-yl)pyrazolo[1,5-a]pyrimidin-3-yl)pyridin-2-yl)piperazin-1-yl)methyl)-1-oxoisoindolin-2-yl)piperidine-2,6-dione (GT-04262)

The target compound (GT-04262) was prepared using the method described in Scheme 11 (white solid, 71 mg, yield 96.55%). ¹H NMR (400 MHz, DMSO-*d₆*) δ 11.30 (s, 1H), 11.01 (d, *J* = 6.9 Hz, 1H), 8.79 - 8.38 (m, 2H), 7.90 (s, 1H), 7.84 (d, *J* = 7.7 Hz, 1H), 7.78 (d, *J* = 7.8 Hz, 1H), 7.47 - 7.31 (m, 2H), 7.09 (d, *J* = 33.7 Hz, 3H), 6.62 (d, *J* = 6.3 Hz, 2H), 5.45 (s, 1H), 5.20 - 5.08 (m, 1H), 4.51 - 4.38 (m, 5H), 4.02 (s, 1H), 3.67 (s, 2H), 3.37 (s, 4H), 3.12 (s, 2H), 2.97 - 2.89 (m, 1H), 2.62 (d, *J* = 17.1 Hz, 1H), 2.47 - 2.39 (m, 1H), 2.17 - 1.81 (m, 5H). LCMS (ESI) calcd for C₃₉H₃₈F₂N₉O₃⁺ [M+H]⁺: 718.30, found, 718.40.

### Example 368: preparation of 3-(5-((4-(6-(5-((R)-2-(2,5-difluorophenyl)pyrrolidin-1-yl)pyrazolo[1,5-a]pyrimidin-3-yl)pyridin-2-yl)piperazin-1-yl)methyl)-4-fluoro-1-oxoisoindolin-2-yl)piperidine-2,6-dione (GT-04263)

The target compound (GT-04263) was prepared using the method described in Scheme 11 (white solid, 73 mg, yield 96.83%). ¹H NMR (400 MHz, DMSO-*d₆*) δ 11.08 - 10.82 (m, 2H), 8.58 (d, *J* = 132.9 Hz, 2H), 7.93 (t, *J* = 6.9 Hz, 1H), 7.79 - 7.59 (m, 2H), 7.35 (td, *J* = 9.6, 4.4 Hz, 2H), 7.09 (d, *J* = 31.8 Hz, 2H), 6.62 (s, 1H), 5.45 (s, 1H), 5.16 (dd, *J* = 13.3, 5.1 Hz, 1H), 4.92 (s, 1H), 4.63 - 4.41 (m, 6H), 4.03 (s, 1H), 3.64 (d, *J* = 38.3 Hz, 2H), 3.30 - 3.06 (m, 4H), 2.91 (dd, *J* = 12.7, 4.6 Hz, 1H), 2.60 (s, 1H), 2.47 - 2.42 (m, 1H), 2.27 - 1.58 (m, 5H). LCMS (ESI) calcd for C₃₉H₃₇F₃N₉O₃⁺ [M+H]⁺: calcd for 736.29, found, 736.30.

### Example 369: preparation of 3-(5-((4-(6-(5-((R)-2-(2,5-difluorophenyl)pyrrolidin-1-yl)pyrazolo[1,5-a]pyrimidin-3-yl)pyridin-2-yl)piperazin-1-yl)methyl)-6-fluoro-1-oxoisoindolin-2-yl)piperidine-2,6-dione (GT-04264)

The target compound (GT-04264) was prepared using the method described in Scheme 11 (white solid, 25 mg, yield 31.73%). ¹H NMR (400 MHz, MeOD) δ 8.43 (d, *J* = 39.5 Hz, 2H), 7.87 (t, *J* = 62.3 Hz, 2H), 7.63 - 7.27 (m, 2H), 7.06 (d, *J* = 53.2 Hz, 2H), 6.90 - 6.06 (m, 3H), 5.40 (d, *J* = 47.3 Hz, 1H), 5.08 (dd, *J*= 13.3, 5.1 Hz, 1H), 4.61 - 4.35 (m, 4H), 4.33 - 3.24 (m, 10H), 2.91 - 2.63 (m, 2H), 2.59 - 2.35 (m, 2H), 2.06 (dd, *J* = 36.9, 31.5 Hz, 4H). LCMS (ESI) calcd for C₃₉H₃₇F₃N₉O₃⁺ [M+H]⁺: 736.29, found, 736.30.

### Example 370: preparation of 3-(5-((4-(6-(5-((R)-2-(2,5-difluorophenyl)pyrrolidin-1-yl)pyrazolo[1,5-a]pyrimidin-3-yl)pyridin-2-yl)piperazin-1-yl)methyl)-7-fluoro-1-oxoisoindolin-2-yl)piperidine-2,6-dione (GT-04265)

The target compound (GT-04265) was prepared using the method described in Scheme 11 (white solid, 67 mg, yield 88.87%). ¹H NMR (400 MHz, DMSO) δ 10.95 (d, *J* = 8.2 Hz, 1H), 10.76 (s, 1H), 8.67 (s, 1H), 8.32 (s, 1H), 7.81 - 6.81 (m, 7H), 6.53 (s, 1H), 5.05 (dd, *J* = 13.0, 5.2 Hz, 1H), 4.59 - 4.26 (m, 6H), 3.95 (s, 1H), 3.60 (s, 1H), 3.24 - 2.76 (m, 8H), 2.54 (d, *J*= 18.9 Hz, 1H), 2.33 (s, 1H), 1.99 (d, *J* = 22.9 Hz, 5H). LCMS (ESI) calcd for C₃₉H₃₇F₃N₉O₃⁺ [M+H]⁺: 736.29, found, 736.30.

### Example 371: preparation of 5-((4-(6-(5-((R)-2-(2,5-difluorophenyl)pyrrolidin-1-yl)pyrazolo[1,5-a]pyrimidin-3-yl)pyridin-2-yl)piperazin-1-yl)methyl)-2-(2,6-dioxopiperidin-3-yl)isoindoline-1,3-dione (GT-04266)

The target compound (GT-04266) was prepared using the method described in Scheme 11 (white solid, 70 mg, yield 93.37%). ¹H NMR (400 MHz, DMSO) δ 11.46 (s, 1H), 11.15 (d, *J* = 6.8 Hz, 1H), 9.15 (s, 1H), 8.76 - 8.38 (m, 1H), 7.94 (dddd, *J* = 54.2, 46.5, 41.5, 27.3 Hz, 5H), 7.43 - 7.28 (m, 1H), 7.09 (d, *J* = 29.8 Hz, 2H), 6.61 (d, *J* = 7.1 Hz, 1H), 5.53 - 5.10 (m, 2H), 5.00 - 3.97 (m, 4H), 3.61 (ddt, *J* = 9.2, 6.6, 4.6 Hz, 4H), 3.25 (d, *J* = 66.7 Hz, 4H), 2.92 - 2.84 (m, 1H), 2.64 - 2.54 (m, 2H), 2.34 - 1.74 (m, 5H). LCMS (ESI) calcd for C₃₉H₃₆F₂N₉O₄⁺ [M+H]⁺: 732.28, found, 732.30.

### Example 372: preparation of 2-(6,7-dihydro-5H-pyrrolo[1,2-c]imidazol-1-yl)-2-(6-(4-(4-((2-(2,6-dioxopiperidin-3-yl)-1-oxoisoindolin-5-yl)methyl)piperazin-1-yl)phenyl)-4-fluoro-1-oxoisoindolin-2-yl)-N-(thiazol-2-yl)acetamide (GT-04421)

The target compound (GT-04421) was prepared using the method described in Scheme 11 (white solid, 52 mg, yield 67.94%). ¹H NMR (400 MHz, MeOD) δ 8.85 (d, *J* = 17.0 Hz, 1H), 7.92 (dd, *J* = 20.7, 10.3 Hz, 3H), 7.80 (t, *J* = 11.2 Hz, 1H), 7.68 - 7.59 (m, 3H), 7.51 (d, *J* = 3.4 Hz, 1H), 7.24 (d, *J* = 3.8 Hz, 1H), 7.15 (d, *J* = 8.6 Hz, 2H), 5.16 (dt, *J* = 16.1, 8.1 Hz, 1H), 4.92 (d, *J* = 17.0 Hz, 1H), 4.59 (t, *J* = 10.2 Hz, 6H), 4.37 (dt, *J* = 19.6, 9.8 Hz, 2H), 4.12 - 3.34 (m, 8H), 3.04 (dd, *J* = 15.9, 8.2 Hz, 1H), 2.96 - 2.77 (m, 3H), 2.77 - 2.67 (m, 2H), 2.54 (dt, *J* = 13.3, 8.6 Hz, 1H), 2.27 - 2.16 (m, 1H). LCMS (ESI) C₄₃H₄₁FN₉O₅S⁺ [M+H]⁺: 814.29, found, 814.3.

### Example 373: preparation of 2-(6,7-dihydro-5H-pyrrolo[1,2-c]imidazol-1-yl)-2-(6-(4-(4-(4-((2-(2,6-dioxopiperidin-3-yl)-1-oxoisoindolin-5-yl)methyl)piperazin-1-yl)piperidin-1-yl)phenyl)-4-fluoro-1-oxoisoindolin-2-yl)-N-(thiazol-2-yl)acetamide (GT-04422)

The target compound (GT-04422) was prepared using the method described in Scheme 11 (white solid, 52 mg, yield 68.73%). ¹H NMR (400 MHz, DMSO) δ 10.85 (s, 1H), 9.03 (s, 1H), 7.90 (s, 1H), 7.85 - 7.61 (m, 6H), 7.52 (d, *J* = 3.6 Hz, 1H), 7.30 (d, *J* = 3.6 Hz, 1H), 7.12 (d, *J* = 8.7 Hz, 2H), 6.40 (s, 1H), 5.12 (dd, *J* = 13.1, 5.2 Hz, 1H), 4.81 (d, *J* = 17.1 Hz, 1H), 4.54 - 4.47 (m, 2H), 4.40 (d, *J* = 15.7 Hz, 2H), 4.28 (t, *J* = 7.2 Hz, 2H), 3.97 (d, *J* = 12.8 Hz, 4H), 3.48 (s, 8H), 2.89 (dt, *J* = 22.7, 10.1 Hz, 4H), 2.72 - 2.53 (m, 5H), 2.47 - 2.36 (m, 1H), 2.21 (d, *J* = 10.7 Hz, 2H), 2.09 - 2.02 (m, 1H), 1.86 (d, *J* = 9.7 Hz, 2H). LCMS (ESI) calcd for C₄₈H₅₀FN₁₀O₅S⁺ [M+H]⁺: 897.37, found, 897.4.

### Example 374: preparation of 2-(6,7-dihydro-5H-pyrrolo[1,2-c]imidazol-1-yl)-2-(6-(4-(4-(1-((2-(2,6-dioxopiperidin-3-yl)-1-oxoisoindolin-5-yl)methyl)piperidin-4-yl)piperazin-1-yl)phenyl)-4-fluoro-1-oxoisoindolin-2-yl)-N-(thiazol-2-yl)acetamide (GT-04423)

The target compound (GT-04423) was prepared using the method described in Scheme 11 (white solid, 34 mg, yield 44.94%). ¹H NMR (400 MHz, DMSO) δ 10.86 (s, 1H), 9.02 (s, 1H), 7.90 (s, 1H), 7.80 (dd, *J*= 18.5, 8.7 Hz, 4H), 7.72 (d, *J* = 8.5 Hz, 2H), 7.52 (d, *J* = 3.5 Hz, 1H), 7.30 (d, *J* = 3.6 Hz, 1H), 7.11 (d, *J* = 8.2 Hz, 2H), 6.40 (s, 1H), 5.12 (dd, *J* = 13.1, 5.1 Hz, 1H), 4.82 (d, *J* = 17.0 Hz, 1H), 4.52 (d, *J* = 14.6 Hz, 2H), 4.42 (d, *J* = 17.3 Hz, 3H), 4.26 (d, *J* = 7.1 Hz, 2H), 3.88 (d, *J* = 49.4 Hz, 4H), 3.21 (s, 8H), 3.03 (s, 2H), 2.96 - 2.88 (m, 2H), 2.67 (t, *J =* 10.6 Hz, 2H), 2.58 (dd, *J* = 13.9, 6.8 Hz, 2H), 2.47 - 2.29 (m, 5H), 2.08 - 2.02 (m, 1H). LCMS (ESI) calcd for C₄₈H₅₀FN₁₀O₅S⁺ [M+H]⁺: 897.37, found, 897.4.

### Example 375: preparation of 2-(6,7-dihydro-SH-pyrrolo[1,2-c]imidazol-1-yl)-2-(6-(4-((1S,4S)-5-((2-(2,6-dioxopiperidin-3-yl)-1-oxoisoindolin-5-yl)methyl)-2,5-diazabicyclo[2.2.1]heptan-2-yl)phenyl)-4-fluoro-1-oxoisoindolin-2-yl)-N-(thiazol-2-yl)acetamide (GT-04424)

The target compound (GT-04424) was prepared using the method described in Scheme 11 (white solid, 42 mg, yield 55.34%). ¹H NMR (400 MHz, DMSO) δ 11.03 (s, 1H), 10.85 (s, 1H), 8.94 (s, 1H), 8.00 (s, 1H), 7.88 (d, *J* = 7.8 Hz, 1H), 7.82 - 7.76 (m, 2H), 7.69 (dd, *J* = 33.7, 9.5 Hz, 3H), 7.52 (d, *J* = 3.5 Hz, 1H), 7.30 (d, *J* = 3.5 Hz, 1H), 6.78 (t, *J* = 22.3 Hz, 2H), 6.38 (s, 1H), 5.11 (dd, *J* = 13.2, 4.9 Hz, 1H), 4.84 - 4.72 (m, 2H), 4.66 (s, 1H), 4.47 (dd, *J* = 26.7, 10.7 Hz, 5H), 4.26 (t, *J* = 7.1 Hz, 2H), 3.85 (d, *J* = 11.0 Hz, 1H), 3.64 (d, *J* = 9.6 Hz, 1H), 3.09 - 2.84 (m, 4H), 2.71 - 2.55 (m, 6H), 2.43 (s, 1H), 2.20 (d, *J* = 9.9 Hz, 1H), 2.06 (s, 1H). LCMS (ESI) calcd for C₄₄H₄₁FN₉O₅S⁺ [M+H]⁺: 826.29, found, 826.3.

### Example 376: preparation of 2-(6,7-dihydro-5H-pyrrolo[1,2-c]imidazol-1-yl)-2-(6-(4-(4-(((2-(2,6-dioxopiperidin-3-yl)-1-oxoisoindolin-5-yl)methyl)(methyl)amino)piperidin-1-yl)phenyl)-4-fluoro-1-oxoisoindolin-2-yl)-N-(thiazol-2-yl)acetamide (GT-04427)

The target compound (GT-04427) was prepared using the method described in Scheme 11 (white solid, 46 mg, yield 61.61%). ¹H NMR (400 MHz, DMSO) δ 11.18 (s, 1H), 11.01 (d, *J* = 8.2 Hz, 1H), 9.10 (s, 1H), 7.96 (s, 1H), 7.82 (d, *J* = 5.9 Hz, 4H), 7.73 (d, *J* = 8.7 Hz, 2H), 7.53 (d, *J* = 3.6 Hz, 1H), 7.33 (d, *J* = 3.6 Hz, 1H), 7.18 (d, *J* = 7.7 Hz, 2H), 6.40 (s, 1H), 5.15 (dd, *J* = 13.2, 5.0 Hz, 1H), 4.80 (d, *J* = 17.1 Hz, 1H), 4.63 (d, *J* = 12.4 Hz, 1H), 4.51 (dd, *J* = 16.4, 6.3 Hz, 2H), 4.33 (ddd, *J* = 27.9, 15.9, 8.2 Hz, 5H), 3.97 (s, 2H), 3.48 (s, 2H), 2.95 - 2.84 (m, 4H), 2.66 - 2.58 (m, 6H), 2.44 (dd, *J* = 13.3, 4.7 Hz, 1H), 2.33 (s, 1H), 2.26 (s, 1H), 2.02 (d, *J* = 5.1 Hz, 3H). LCMS (ESI) calcd for C₄₅H₄₅FN₉O₅S⁺ [M+H]⁺: 842.32, found, 842.3.

### Example 377: preparation of 2-(6,7-dihydro-5H-pyrrolo[1,2-c]imidazol-1-yl)-2-(6-(4-(8-((2-(2,6-dioxopiperidin-3-yl)-1-oxoisoindolin-5-yl)methyl)-3,8-diazabicyclo[3.2.1]octan-3-yl)phenyl)-4-fluoro-1-oxoisoindolin-2-yl)-N-(thiazol-2-yl)acetamide (GT-04429)

The target compound (GT-04429) was prepared using the method described in Scheme 11 (white solid, 42 mg, yield 55.73%). ¹H NMR (400 MHz, DMSO) δ 11.66 (s, 1H), 11.02 (s, 1H), 9.04 (s, 1H), 8.10 (s, 1H), 7.97 (d, *J* = 7.8 Hz, 1H), 7.82 (dd, *J* = 19.0, 8.8 Hz, 3H), 7.70 (d, *J* = 8.8 Hz, 2H), 7.53 (d, *J* = 3.6 Hz, 1H), 7.33 (d, *J* = 3.6 Hz, 1H), 6.98 (d, *J* = 8.9 Hz, 2H), 6.39 (s, 1H), 5.16 (dd, *J* = 13.3, 5.1 Hz, 1H), 4.79 (d, *J* = 17.3 Hz, 1H), 4.52 (dd, *J* = 17.1, 12.5 Hz, 2H), 4.45 -4.37 (m, 3H), 4.25 (t, *J*= 7.1 Hz, 2H), 3.99 (s, 2H), 3.73 (d, *J* = 11.7 Hz, 2H), 3.56 - 3.49 (m, 3H), 3.01 - 2.85 (m, 2H), 2.70 - 2.54 (m, 4H), 2.40 (dd, *J* = 29.9, 12.8 Hz, 3H), 2.03 (d, *J* = 8.7 Hz, 3H). LCMS (ESI) calcd for C₄₅H₄₃FN₉O₅S⁺ [M+H]⁺: 840.31, found, 840.3.

### Example 378: preparation of 2-(6,7-dihydro-5H-pyrrolo[1,2-c]imidazol-1-yl)-2-(6-(4-(4-(1-((2-(2,6-dioxopiperidin-3-yl)-1-oxoisoindolin-5-yl)methyl)-3,3-difluoropiperidin-4-yl)piperazin-1-yl)phenyl)-4-fluoro-1-oxoisoindolin-2-yl)-N-(thiazol-2-yl)acetamide (GT-04459)

The target compound (GT-04459) was prepared using the method described in Scheme 11 (white solid, 42 mg, yield 58.54%). ¹H NMR (400 MHz, DMSO) δ 11.01 (s, 1H), 9.10 (s, 1H), 7.81 (dd, *J* = 13.9, 7.6 Hz, 3H), 7.73 (d, *J* = 8.6 Hz, 3H), 7.64 (s, 1H), 7.53 (d, *J* = 3.6 Hz, 1H), 7.33 (d, *J* = 3.6 Hz, 1H), 7.13 (d, *J* = 7.9 Hz, 2H), 6.40 (s, 1H), 5.14 (dd, *J* = 13.2, 5.1 Hz, 1H), 4.80 (d, *J* = 17.2 Hz, 1H), 4.51 (dd, *J* = 17.5, 6.1 Hz, 2H), 4.38 (d, *J* = 14.1 Hz, 1H), 4.26 (t, *J* = 7.1 Hz, 2H), 4.15 (s, 3H), 3.53 (s, 6H), 3.30 (s, 6H), 2.89 (dd, *J* = 18.1, 11.6 Hz, 2H), 2.66 - 2.54 (m, 4H), 2.45 - 2.33 (m, 3H), 2.22 (s, 1H), 2.05 - 1.96 (m, 1H). LCMS (ESI) calcd for C₄₈H₄₈F₃N₁₀O₅S⁺ [M+H]⁺: 933.35, found, 933.4.

### Example 379: preparation of 2-(6,7-dihydro-SH-pyrrolo[1,2-c]imidazol-1-yl)-2-(6-(4-((1R,4R)-5-((2-(2,6-dioxopiperidin-3-yl)-1-oxoisoindolin-5-yl)methyl)-2,5-diazabicyclo[2.2.1]heptan-2-yl)phenyl)-4-fluoro-1-oxoisoindolin-2-yl)-N-(thiazol-2-yl)acetamide (GT-04460)

The target compound (GT-04460) was prepared using the method described in Scheme 11 (white solid, 32 mg, yield 42.17%). ¹H NMR (400 MHz, DMSO) δ 11.01 (s, 1H), 10.74 (s, 1H), 9.05 (s, 1H), 7.97 (s, 1H), 7.88 - 7.75 (m, 4H), 7.66 (d, *J* = 8.6 Hz, 2H), 7.53 (d, J = 3.5 Hz, 1H), 7.33 (d, *J* = 3.5 Hz, 1H), 6.74 (d, *J* = 8.5 Hz, 2H), 6.38 (s, 1H), 5.14 (dd, *J* = 13.3, 4.9 Hz, 1H), 4.84 - 4.65 (m, 3H), 4.55 - 4.43 (m, 4H), 4.37 (d, *J* = 16.8 Hz, 1H), 4.26 (t, *J* = 7.0 Hz, 2H), 3.80 - 3.53 (m, 4H), 3.25 (s, 1H), 2.97 - 2.83 (m, 2H), 2.61 (dd, *J* = 28.5, 16.4 Hz, 5H), 2.40 (dd, *J* = 31.1, 13.4 Hz, 1H), 2.20 (d, *J* = 10.1 Hz, 1H), 2.03 (s, 1H). LCMS (ESI) calcd for C₄₄H₄₁FN₉O₅S⁺ [M+H]⁺: 826.29, found, 826.3.

### Example 380: preparation of N-(2-(1-((2-(2,6-dioxopiperidin-3-yl)-1-oxoisoindolin-5-yl)methyl)piperidin-4-yl)-5-(2-hydroxypropan-2-yl)benzo[d] thiazol-6-yl)-6-(trifluoromethyl)picolinamide (GT-04489)

The target compound (GT-04489) was prepared using the method described in Scheme 11 (white solid, 47 mg, yield 57.47%). ¹H NMR (400 MHz, DMSO) δ 12.58 (d, *J* = 8.1 Hz, 1H), 11.02 (s, 1H), 10.63 (s, 1H), 9.11 (d, *J* = 10.0 Hz, 1H), 8.52 - 8.42 (m, 1H), 8.39 (dd, *J* = 9.9, 5.7 Hz, 1H), 8.20 (dt, *J* = 5.5, 2.3 Hz, 1H), 7.93 (d, *J* = 11.7 Hz, 1H), 7.88 - 7.85 (m, 1H), 7.83 - 7.72 (m, 1H), 6.10 (s, 1H), 5.16 (dd, *J* = 13.3, 5.0 Hz, 1H), 4.62 - 4.31 (m, 4H), 3.47 (dd, *J* = 43.5, 10.7 Hz, 3H), 3.15 (d, *J* = 11.9 Hz, 2H), 2.92 (dd, *J* = 21.4, 9.3 Hz, 1H), 2.62 (d, *J* = 17.3 Hz, 1H), 2.44 (dd, *J* = 13.2, 4.9 Hz, 1H), 2.41 - 2.09 (m, 4H), 2.08 - 1.98 (m, 1H), 1.64 (d, *J* = 9.5 Hz, 6H). LCMS (ESI) calcd for C₃₆H₃₆F₃N₆O₅S⁺ [M+H]⁺: 721.24, found, 721.3.

### Example 381: preparation of N-(2-(1-((2-(2,6-dioxopiperidin-3-yl)-4-fluoro-1-oxoisoindolin-5-yl)methyl)piperidin-4-yl)-5-(2-hydroxypropan-2-yl)benzo[d]thiazol-6-yl)-6-(trifluoromethyl)picolinamide (GT-04505)

The target compound (GT-04505) was prepared using the method described in Scheme 11 (white solid, 55 mg, yield 65.69%). ¹H NMR (400 MHz, DMSO) δ 12.58 (d, *J* = 6.9 Hz, 1H), 11.03 (d, *J* = 10.5 Hz, 1H), 10.73 (d, *J* = 65.4 Hz, 1H), 9.11 (d, *J* = 9.8 Hz, 1H), 8.55 - 8.32 (m, 2H), 8.19 (dd, *J* = 7.8, 0.9 Hz, 1H), 7.99 - 7.87 (m, 2H), 7.72 (dd, *J* = 11.5, 7.9 Hz, 1H), 6.11 (s, 1H), 5.17 (dd, *J* = 13.3, 5.1 Hz, 1H), 4.66 - 4.45 (m, 4H), 3.64 (d, *J* = 35.5 Hz, 2H), 3.44 (s, 1H), 3.23 (s, 2H), 2.92 (dd, *J* = 17.5, 5.5 Hz, 1H), 2.62 (d, *J* = 15.9 Hz, 1H), 2.48 - 2.41 (m, 1H), 2.40 - 2.12 (m, 4H), 2.07 - 1.98 (m, 1H), 1.64 (d, *J* = 8.3 Hz, 6H). LCMS (ESI) calcd for C₃₆H₃₅F₄N₆O₅S⁺ [M+H]⁺: 739.23, found, 739.3.

### Example 382: preparation of N-(2-(1-((2-(2,6-dioxopiperidin-3-yl)-6-fluoro-1-oxoisoindolin-5-yl)methyl)piperidin-4-yl)-5-(2-hydroxypropan-2-yl)benzo[d]thiazol-6-yl)-6-(trifluoromethyl)picolinamide (GT-04506)

The target compound (GT-04506) was prepared using the method described in Scheme 11 (white solid, 55 mg, yield 65.69%). ¹H NMR (400 MHz, DMSO) δ 12.58 (d, *J* = 6.7 Hz, 1H), 11.17 (s, 1H), 11.03 (d, *J* = 9.7 Hz, 1H), 9.12 (d, *J* = 10.0 Hz, 1H), 8.49 - 8.43 (m, 1H), 8.39 (dd, *J* = 10.0, 5.6 Hz, 1H), 8.23 - 8.15 (m, 1H), 8.08 (t, *J* = 7.3 Hz, 1H), 7.94 (d, *J* = 7.3 Hz, 1H), 7.70 (t, *J* = 7.8 Hz, 1H), 6.11 (s, 1H), 5.16 (dd, *J* = 13.3, 5.0 Hz, 1H), 4.55 - 4.37 (m, 4H), 3.58 (dd, *J* = 48.1, 36.9 Hz, 3H), 3.24 (d, *J* = 9.7 Hz, 2H), 3.00 - 2.88 (m, 1H), 2.62 (d, *J* = 17.0 Hz, 1H), 2.47 - 2.42 (m, 1H), 2.40 - 2.15 (m, 4H), 2.05 (dd, *J* = 13.6, 8.5 Hz, 1H), 1.65 (d, *J* = 8.9 Hz, 6H). LCMS (ESI) calcd for C₃₆H₃₅F₄N₆O₅S⁺ [M+H]⁺: 739.23, found, 739.3.

### Example 383: preparation of N-(2-(1-((2-(2,6-dioxopiperidin-3-yl)-7-fluoro-1-oxoisoindolin-5-yl)methyl)piperidin-4-yl)-5-(2-hydroxypropan-2-yl)benzo[d]thiazol-6-yl)-6-(trifluoromethyl)picolinamide (GT-04507)

The target compound (GT-04507) was prepared using the method described in Scheme 11 (white solid, 55 mg, yield 65.69%). ¹H NMR (400 MHz, DMSO) δ 12.58 (d, *J* = 6.8 Hz, 1H), 11.02 (d, *J* = 9.6 Hz, 1H), 10.92 (s, 1H), 9.12 (d, *J* = 9.0 Hz, 1H), 8.47 (dd, *J* = 7.4, 4.0 Hz, 1H), 8.42 - 8.34 (m, 1H), 8.20 (dt, *J* = 5.2, 2.1 Hz, 1H), 7.94 (d, *J* = 10.2 Hz, 1H), 7.67 (dd, *J* = 19.3, 9.4 Hz, 2H), 6.11 (s, 1H), 5.12 (dd, *J* = 13.3, 5.1 Hz, 1H), 4.58 - 4.39 (m, 4H), 3.75 - 3.49 (m, 2H), 3.42 (d, *J* = 10.8 Hz, 1H), 3.14 (d, *J* = 11.7 Hz, 2H), 2.99 - 2.86 (m, 1H), 2.61 (d, *J* = 15.1 Hz, 1H), 2.44 - 2.40 (m, 1H), 2.37 - 2.09 (m, 4H), 2.05 - 1.97 (m, 1H), 1.64 (d, *J* = 8.4 Hz, 6H). LCMS (ESI) calcd for C₃₆H₃₅F₄N₆O₅S⁺ [M+H]⁺: 739.23, found, 739.3.

### Example 384: preparation of 2-(6,7-dihydro-5H-pyrrolo[1,2-c]imidazol-1-yl)-2-(6-(4-(4-(4-((2-(2,6-dioxopiperidin-3-yl)-1-oxoisoindolin-5-yl)methyl)piperazin-1-yl)-3,3-difluoropiperidin-1-yl)phenyl)-4-fluoro-1-oxoisoindolin-2-yl)-N-(thiazol-2-yl)acetamide (GT-04514)

The target compound (GT-04514) was prepared using the method described in Scheme 11 (white solid, 53 mg, yield 71.20%). ¹H NMR (400 MHz, DMSO) δ 11.14 (s, 1H), 11.01 (s, 1H), 9.07 (s, 1H), 7.89 (s, 1H), 7.79 (q, *J* = 8.1 Hz, 4H), 7.65 (t, *J* = 9.5 Hz, 2H), 7.53 (d, *J* = 3.6 Hz, 1H), 7.33 (d, *J* = 3.6 Hz, 1H), 7.08 (d, *J* = 8.7 Hz, 2H), 6.39 (s, 1H), 5.14 (dd, *J* = 13.2, 5.0 Hz, 1H), 4.79 (d, *J* = 17.1 Hz, 1H), 4.53 - 4.34 (m, 5H), 4.26 (t, *J* = 7.0 Hz, 2H), 4.05 (s, 1H), 3.95 (d, *J* = 11.1 Hz, 1H), 3.28 (s, 3H), 3.11 (dd, *J* = 33.8, 10.9 Hz, 8H), 2.96 - 2.84 (m, 3H), 2.67 - 2.53 (m, 4H), 2.47 - 2.35 (m, 1H), 2.03 (dd, *J* = 17.7, 12.2 Hz, 1H), 1.87 (s, 2H). LCMS (ESI) calcd for C₄₈H₄₈F₃N₁₀O₅S⁺ [M+H]⁺: 933.35, found, 933.4.

### Example 385: preparation of N-(2-(1-((2-(2,6-dioxopiperidin-3-yl)-1-oxoisoindolin-5-yl)methyl)piperidin-4-yl)-5-(2-hydroxypropan-2-yl)benzo[d] oxazol-6-yl)-6-(trifluoromethyl)picolinamide (GT-04516)

The target compound (GT-04516) was prepared using the method described in Scheme 11 (white solid, 58 mg, yield 70.50%). ¹H NMR (400 MHz, DMSO) δ 12.63 (d, *J* = 9.7 Hz, 1H), 11.01 (d, *J* = 9.1 Hz, 1H), 10.36 (s, 1H), 8.82 - 8.70 (m, 1H), 8.51 - 8.43 (m, 1H), 8.38 (t, *J* = 7.9 Hz, 1H), 8.24 - 8.14 (m, 1H), 7.84 (dd, *J* = 15.8, 8.2 Hz, 2H), 7.79 - 7.65 (m, 3H), 6.08 (d, *J* = 8.6 Hz, 1H), 5.15 (dd, *J* = 14.0, 4.9 Hz, 1H), 4.57 - 4.41 (m, 4H), 3.54 (d, *J* = 10.2 Hz, 1H), 3.15 (d, *J* = 11.5 Hz, 2H), 3.05 - 2.80 (m, 2H), 2.71 - 2.56 (m, 2H), 2.37 (dd, *J* = 26.8, 9.8 Hz, 3H), 2.18 - 1.98 (m, 3H), 1.62 (d, *J* = 11.4 Hz, 6H). LCMS (ESI) calcd for C₃₆H₃₆F₃N₆O₆⁺ [M+H]⁺: 705.26, found, 705.3.

### Example 386: preparation of N-(2-(1-((2-(2,6-dioxopiperidin-3-yl)-4-fluoro-1-oxoisoindolin-5-yl)methyl)piperidin-4-yl)-5-(2-hydroxypropan-2-yl)benzo[d]oxazol-6-yl)-6-(trifluoromethyl)picolinamide (GT-04518)

The target compound (GT-04518) was prepared using the method described in Scheme 11 (white solid, 50 mg, yield 59.34%). ¹H NMR (400 MHz, DMSO) δ 12.63 (d, *J* = 8.7 Hz, 1H), 11.03 (s, 1H), 10.57 (d, *J* = 63.4 Hz, 1H), 8.76 (d, *J* = 12.9 Hz, 1H), 8.41 (ddd, *J* = 25.9, 16.4, 8.9 Hz, 3H), 8.23 - 8.17 (m, 1H), 7.90 (s, 1H), 7.71 (d, *J* = 15.5 Hz, 2H), 6.07 (s, 1H), 5.18 - 5.12 (m, 1H), 4.55 (dd, *J* = 31.9, 10.3 Hz, 4H), 3.61 (s, 2H), 3.08 (s, 3H), 2.92 (d, *J* = 11.9 Hz, 1H), 2.62 (d, *J* = 17.0 Hz, 1H), 2.35 (d, *J* = 13.4 Hz, 3H), 2.15 (d, *J* = 12.1 Hz, 1H), 2.02 (d, *J* = 5.2 Hz, 2H), 1.62 (d, *J* = 10.3 Hz, 6H). LCMS (ESI) calcd for C₃₆H₃₅F₄N₆O₆⁺ [M+H]⁺: 723.25, found, 723.3.

### Example 387: preparation of N-(2-(1-((2-(2,6-dioxopiperidin-3-yl)-6-fluoro-1-oxoisoindolin-5-yl)methyl)piperidin-4-yl)-5-(2-hydroxypropan-2-yl)benzo[d]oxazol-6-yl)-6-(trifluoromethyl)picolinamide (GT-04519)

The target compound (GT-04519) was prepared using the method described in Scheme 11 (white solid, 44 mg, yield 52.22%). ¹H NMR (400 MHz, DMSO) δ 12.63 (d, *J* = 9.4 Hz, 1H), 11.03 (s, 1H), 10.48 (s, 1H), 8.82 - 8.65 (m, 1H), 8.53 - 8.32 (m, 2H), 8.19 (d, *J* = 7.7 Hz, 1H), 7.98 (s, 1H), 7.78 - 7.61 (m, 2H), 6.07 (s, 1H), 5.15 (d, *J* = 13.4 Hz, 1H), 4.63 - 4.28 (m, 4H), 3.80 - 3.43 (m, 2H), 3.29 - 3.04 (m, 3H), 2.93 (t, *J* = 12.9 Hz, 1H), 2.62 (d, *J* = 16.1 Hz, 1H), 2.33 (s, 3H), 2.10 (dd, *J* = 24.5, 14.4 Hz, 3H), 1.73 -1.48 (m, 6H). LCMS (ESI) calcd for C₃₆H₃₅F₄N₆O₆⁺ [M+H]⁺: 723.25, found, 723.3.

### Example 388: preparation of N-(2-(1-((2-(2,6-dioxopiperidin-3-yl)-1-oxoisoindolin-5-yl)methyl)piperidin-4-yl)-6-(2-hydroxypropan-2-yl)-2H-indazol-5-yl)-6-(trifluoromethyl)picolinamide (GT-04520)

The target compound (GT-04520) was prepared using the method described in Scheme 11 (white solid, 45 mg, yield 54.28%). ¹H NMR (400 MHz, DMSO) δ 12.39 (d, *J* = 7.4 Hz, 1H), 11.00 (d, *J* = 10.5 Hz, 1H), 10.77 (s, 1H), 8.75 (d, *J* = 11.8 Hz, 1H), 8.56 - 8.41 (m, 1H), 8.40 - 8.29 (m, 2H), 8.16 (d, *J* = 7.7 Hz, 1H), 7.81 (ddd, *J* = 21.4, 13.4, 6.8 Hz, 3H), 7.60 (d, *J* = 9.1 Hz, 1H), 5.97 (s, 1H), 5.22 - 5.06 (m, 1H), 4.57 - 4.35 (m, 4H), 3.57 (d, *J* = 11.0 Hz, 1H), 3.24 - 3.14 (m, 4H), 2.92 (dd, *J* = 17.5, 5.5 Hz, 1H), 2.62 (d, *J* = 16.5 Hz, 1H), 2.45 - 2.22 (m, 5H), 2.06 - 1.98 (m, 1H), 1.63 (d, *J* = 8.5 Hz, 6H). LCMS (ESI) calcd for C₃₆H₃₇F₃N₇O₅⁺ [M+H]⁺: 704.28, found, 704.3.

### Example 389: preparation of N-(2-(1-((2-(2,6-dioxopiperidin-3-yl)-4-fluoro-1-oxoisoindolin-5-yl)methyl)piperidin-4-yl)-6-(2-hydroxypropan-2-yl)-2H-indazol-5-yl)-6-(trifluoromethyl)picolinamide (GT-04521)

The target compound (GT-04521) was prepared using the method described in Scheme 11 (white solid, 46 mg, yield 54.17%). ¹H NMR (400 MHz, DMSO) δ 12.38 (s, 1H), 11.04 (s, 2H), 8.75 (d, *J* = 11.0 Hz, 1H), 8.56 - 8.30 (m, 3H), 8.16 (d, *J* = 7.8 Hz, 1H), 7.97 (t, *J* = 6.9 Hz, 1H), 7.74 (d, *J* = 7.7 Hz, 1H), 7.59 (s, 1H), 5.98 (s, 1H), 5.17 (dd, *J* = 13.2, 4.9 Hz, 1H), 4.79 (t, *J* = 11.6 Hz, 1H), 4.69 - 4.39 (m, 4H), 3.64 (s, 1H), 3.35 - 3.21 (m, 4H), 3.02 - 2.86 (m, 1H), 2.56 (d, *J* = 32.6 Hz, 2H), 2.40 (dd, *J* = 40.3, 12.9 Hz, 4H), 2.04 (dd, *J* = 15.9, 10.7 Hz, 1H), 1.62 (s, 6H). LCMS (ESI) calcd for C₃₆H₃₆F₄N₇O₅⁺ [M+H]⁺: 722.27, found, 722.3.

### Example 390: preparation of N-(2-(1-((2-(2,6-dioxopiperidin-3-yl)-6-fluoro-1-oxoisoindolin-5-yl)methyl)piperidin-4-yl)-6-(2-hydroxypropan-2-yl)-2H-indazol-5-yl)-6-(trifluoromethyl)picolinamide (GT-04522)

The target compound (GT-04522) was prepared using the method described in Scheme 11 (white solid, 41 mg, yield 48.28%). ¹H NMR (400 MHz, DMSO) δ 12.38 (s, 1H), 11.03 (s, 1H), 10.86 (s, 1H), 8.75 (d, *J* = 12.2 Hz, 1H), 8.57 - 8.27 (m, 3H), 8.16 (d, *J* = 7.9 Hz, 1H), 8.02 (s, 1H), 7.72 (d, *J* = 8.4 Hz, 1H), 7.59 (s, 1H), 5.97 (s, 1H), 5.16 (dd, *J* = 13.2, 5.0 Hz, 1H), 4.86 (d, *J* = 51.4 Hz, 1H), 4.63 - 4.26 (m, 4H), 3.61 (s, 1H), 3.35 (s, 4H), 2.92 (dd, *J* = 21.6, 9.4 Hz, 1H), 2.62 (d, *J* = 16.7 Hz, 1H), 2.44 - 2.10 (m, 5H), 2.08 - 1.95 (m, 1H), 1.62 (s, 6H). LCMS (ESI) calcd for C₃₆H₃₆F₄N₇O₅⁺ [M+H]⁺: 722.27, found, 722.3.

### Comparative Example 1: preparation of 3-(5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-1-oxoisoindolin-2-yl)-1-methylpiperidine-2,6-dione (GT-04414)

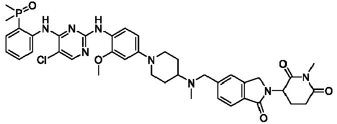

The target compound (GT-04414) was prepared using the method described in Scheme 11 (white solid, 65 mg, yield 81.55%). ¹H NMR (400 MHz, MeOD) δ 8.30 (d, *J* = 27.6 Hz, 1H), 8.16 (s, 1H), 7.99 - 7.87 (m, 2H), 7.82 - 7.68 (m, 2H), 7.64 (t, *J* = 7.9 Hz, 1H), 7.56 - 7.40 (m, 2H), 7.19 (s, 1H), 6.97 (d, *J* = 8.5 Hz, 1H), 5.21 (dd, *J* = 13.4, 5.1 Hz, 1H), 4.78 (s, 1H), 4.60 (q, *J* = 17.5 Hz, 2H), 4.48 (s, 1H), 3.95 (d, *J* = 13.7 Hz, 5H), 3.85 (s, 1H), 3.44 (dd, *J* = 33.4, 21.9 Hz, 2H), 3.14 (d, *J* = 7.2 Hz, 3H), 3.02 - 2.90 (m, 2H), 2.87 (d, *J* = 13.0 Hz, 3H), 2.58 - 2.32 (m, 5H), 2.24 - 2.11 (m, 1H), 1.88 (d, *J* = 13.6 Hz, 6H). LCMS (ESI) calcd for C₄₀H₄₇ClN₈O₅P⁺ [M+H]⁺: 785.31, found, 785.4.

### Comparative Example 2: preparation of 9-ethyl-6,6-dimethyl-8-(4-(4-((2-(1-methyl-2,6-dioxopiperidin-3-yl)-1-oxoisoindolin-5-yl)methyl)piperazin-1-yl)piperidin-1-yl)-11-oxo-6,11-dihydro-5H-benzo[b]carbazole-3-carbonitrile (GT-04415)

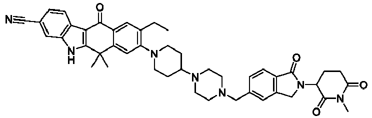

The target compound (GT-04415) was prepared using the method described in Scheme 11 (white solid, 20 mg, yield 23.31%). ¹H NMR (400 MHz, DMSO) δ 12.84 (s, 1H), 8.32 (d, *J* = 8.2 Hz, 1H), 8.04 (d, *J* = 22.1 Hz, 2H), 7.80 (t, *J* = 19.8 Hz, 3H), 7.61 (dd, *J* = 8.2, 1.4 Hz, 1H), 7.36 (s, 1H), 5.22 (dd, *J* = 13.4, 5.1 Hz, 1H), 4.44 (dd, *J* = 54.9, 17.5 Hz, 4H), 3.64 (d, *J* = 38.9 Hz, 4H), 3.32 (d, *J* = 11.5 Hz, 6H), 3.06 - 2.97 (m, 4H), 2.89 - 2.78 (m, 3H), 2.78 - 2.65 (m, 3H), 2.48 - 2.36 (m, 1H), 2.21 (s, 2H), 2.11 - 1.99 (m, 1H), 1.91 (d, *J* = 10.0 Hz, 2H), 1.77 (s, 6H), 1.29 (t, *J* = 7.5 Hz, 3H). LCMS (ESI) calcd for C₄₅H₅₀N₇O₄⁺ [M+H]⁺: 752.39, found, 752.4.

### Comparative Example 3: preparation of 3-(5-(3-((1-(4-((5-chloro-4-((2-(dimethylphosphoryl)phenyl)amino)pyrimidin-2-yl)amino)-3-methoxyphenyl)piperidin-4-yl)(methyl)amino)propyl)-1-oxoisoindolin-2-yl)piperidine-2,6-dione (GT-04458)

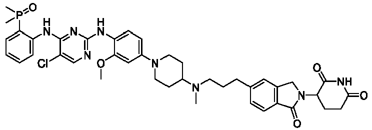

The target compound (GT-04458) was prepared using the method described in Scheme 11 (white solid, 23 mg, yield 28.37%). ¹H NMR (400 MHz, DMSO) δ 12.00 (s, 1H), 10.96 (d, *J* = 18.9 Hz, 2H), 9.47 (s, 1H), 8.34 (d, *J* = 35.0 Hz, 2H), 7.72 - 7.59 (m, 2H), 7.55 - 7.39 (m, 4H), 7.26 (t, *J* = 7.5 Hz, 1H), 6.97 (s, 1H), 6.80 (s, 1H), 5.11 (dd, *J* = 13.2, 5.0 Hz, 1H), 4.39 (dd, *J* = 52.5, 17.2 Hz, 2H), 3.86 (s, 2H), 3.82 (s, 3H), 3.21 (s, 2H), 3.07 (s, 3H), 2.91 (d, *J* = 12.8 Hz, 1H), 2.81 (s, 2H), 2.74 (d, *J* = 4.6 Hz, 3H), 2.61 (d, *J* = 16.8 Hz, 1H), 2.44 - 2.36 (m, 1H), 2.25 (d, *J* = 12.2 Hz, 1H), 2.12 (d, *J* = 31.5 Hz, 3H), 2.04 -1.90 (m, 3H), 1.80 (d, *J* = 13.6 Hz, 6H). LCMS (ESI) calcd for C₄₁H₄₉ClN₈O₅P⁺ [M+H]⁺: 799.32, found, 799.3.

### Comparative Example 4: preparation of 8-(4-(4-(3-(2-(2,6-dioxopiperidin-3-yl)-1-oxoisoindolin-5-yl)propyl)piperazin-1-yl)piperidin-1-yl)-9-ethyl-6,6-dimethyl-11-oxo-6,11-dihydro-5H-benzo[b]carbazole-3-carbonitrile (GT-04457)

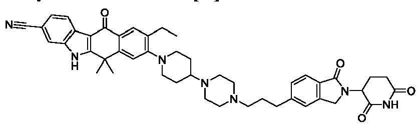

The target compound (GT-04457) was prepared using the method described in Scheme 11 (white solid, 41 mg, yield 49.13%). ¹H NMR (400 MHz, DMSO) δ 12.82 (s, 1H), 11.79 (s, 1H), 10.99 (s, 1H), 8.32 (d, *J* = 8.2 Hz, 1H), 8.07 (s, 1H), 8.01 (s, 1H), 7.70 (d, *J* = 7.7 Hz, 1H), 7.61 (d, *J* = 8.2 Hz, 1H), 7.51 (s, 1H), 7.43 (d, *J* = 7.7 Hz, 1H), 7.36 (s, 1H), 5.12 (dd, *J* = 13.2, 5.1 Hz, 1H), 4.39 (dd, *J* = 51.1, 17.3 Hz, 2H), 3.65 (d, *J* = 119.1 Hz, 7H), 3.33 (d, *J* = 11.0 Hz, 4H), 3.18 (s, 2H), 2.99 - 2.79 (m, 5H), 2.73 (q, *J* = 7.5 Hz, 2H), 2.61 (d, *J* = 16.6 Hz, 1H), 2.44 - 2.34 (m, 1H), 2.22 (s, 2H), 2.09 (s, 2H), 2.04 - 1.99 (m, 1H), 1.93 (s, 2H), 1.77 (s, 6H), 1.30 (t, *J* = 7.5 Hz, 3H). LCMS (ESI) calcd for C₄₆H₅₂N₇O₄⁺ [M+H]⁺: 766.41, found, 766.4.

### Comparative Example 5: preparation of 8-(4-(4-(2-(2-(2,6-dioxopiperidin-3-yl)-1-oxoisoindolin-5-yl)ethyl)piperazin-1-yl)piperidin-1-yl)-9-ethyl-6,6-dimethyl-11-oxo-6,11-dihydro-5H-benzo[b]carbazole-3-carbonitrile (GT-04512)

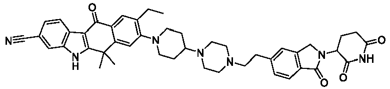

The target compound (GT-04512) was prepared using the method described in Scheme 11 (white solid, 37 mg, yield 43.13%). ¹H NMR (400 MHz, DMSO) δ 12.85 (s, 1H), 12.05 (s, 1H), 10.99 (s, 1H), 8.32 (d, *J* = 8.2 Hz, 1H), 8.07 (s, 1H), 8.01 (s, 1H), 7.72 (d, *J* = 7.8 Hz, 1H), 7.64 - 7.54 (m, 2H), 7.48 (d, *J* = 7.9 Hz, 1H), 7.37 (s, 1H), 5.12 (dd, *J* = 13.2, 5.1 Hz, 1H), 4.40 (dd, *J* = 51.6, 17.5 Hz, 2H), 3.66 (d, *J* = 130.3 Hz, 8H), 3.34 (d, *J* = 11.9 Hz, 5H), 3.22 (s, 2H), 2.94 (dd, *J* = 22.2, 9.0 Hz, 1H), 2.84 (t, *J* = 11.7 Hz, 2H), 2.73 (q, *J* = 7.4 Hz, 2H), 2.61 (d, *J* = 16.6 Hz, 1H), 2.45 - 2.34 (m, 1H), 2.24 (s, 2H), 2.06 -1.86 (m, 3H), 1.77 (s, 6H), 1.30 (t, *J* = 7.5 Hz, 3H). LCMS (ESI) calcd for C₄₅H₅₀N₇O₄⁺ [M+H]⁺: 752.39, found, 752.5.

### Comparative Example 6: preparation of 3-(5-(2-((1-(4-((5-chloro-4-((2-(dimethylphosphoryl)phenyl)amino)pyrimidin-2-yl)amino)-3-methoxyphenyl)piperidin-4-yl)(methyl)amino)ethyl)-1-oxoisoindolin-2-yl)piperidine-2,6-dione (GT-04513)

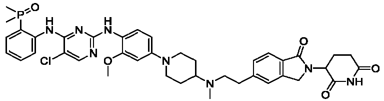

The target compound (GT-04513) was prepared using the method described in Scheme 11 (white solid, 26 mg, yield 32.62%). ¹H NMR (400 MHz, DMSO) δ 11.96 (s, 1H), 11.10 (s, 1H), 10.99 (s, 1H), 9.35 (s, 1H), 8.40 (s, 1H), 8.27 (s, 1H), 7.73 (d, *J* = 7.8 Hz, 1H), 7.68 - 7.53 (m, 2H), 7.55 - 7.33 (m, 3H), 7.24 (t, *J* = 7.2 Hz, 1H), 6.82 (d, *J* = 58.5 Hz, 2H), 5.12 (dd, *J* = 13.3, 5.2 Hz, 1H), 4.39 (dd, *J* = 51.8, 17.4 Hz, 2H), 3.89 (s, 2H), 3.81 (s, 3H), 3.39 - 3.18 (m, 5H), 3.08 - 2.88 (m, 3H), 2.82 (d, *J* = 4.7 Hz, 3H), 2.61 (d, *J* = 17.2 Hz, 1H), 2.45 - 2.36 (m, 1H), 2.27 (s, 1H), 2.19 (s, 1H), 2.08 - 1.87 (m, 3H), 1.80 (d, *J* = 13.6 Hz, 6H). LCMS (ESI) calcd for C₄₀H₄₇ClN₈O₅P⁺ [M+H]⁺: 785.31, found, 785.3.

### Comparative Example 7: preparation of 3-(5-((4-(4-((1R,2S)-6-hydroxy-2-phenyl-1,2,3,4-tetrahydronaphthalen-1-yl)phenyl)piperazin-1-yl)methyl)-1-oxoisoindolin-2-yl)-1-methylpiperidine-2,6-dione (GT-04561)

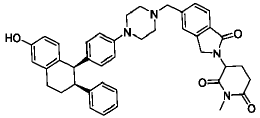

The target compound (GT-04561) was prepared using the method described in Scheme 11 (white solid, 16 mg, yield 17.80%). ¹H NMR (400 MHz, DMSO) δ 10.91 (s, 1H), 9.12 (s, 1H), 7.92 - 7.79 (m, 2H), 7.74 (d, *J* = 7.4 Hz, 1H), 7.18 - 7.07 (m, 3H), 6.84 (d, *J* = 6.9 Hz, 2H), 6.65 - 6.53 (m, 4H), 6.48 (dd, *J* = 8.3, 2.3 Hz, 1H), 6.25 (d, *J* = 8.5 Hz, 2H), 5.21 (dd, *J* = 13.4, 4.9 Hz, 1H), 4.55 - 4.33 (m, 4H), 4.16 (d, *J* = 5.0 Hz, 1H), 3.63 (d, *J* = 12.2 Hz, 2H), 3.29 (s, 2H), 3.11 (d, *J* = 10.2 Hz, 2H), 3.05 - 2.87 (m, 9H), 2.77 (d, *J* = 17.9 Hz, 1H), 2.41 (dd, *J* = 13.0, 8.8 Hz, 1H), 2.06 (ddd, *J* = 15.8, 15.1, 9.0 Hz, 2H), 1.71 (d, *J* = 7.2 Hz, 1H). LCMS (ESI) calcd for C₄₁H₄₃N₄O₄⁺ [M+H]⁺: 655.33, found, 655.4.

### Comparative Example 8: preparation of 3-(5-(2-(4-(4-((1R,2S)-6-hydroxy-2-phenyl-1,2,3,4-tetrahydronaphthalen-1-yl)phenyl)piperazin-1-yl)ethyl)-1-oxoisoindolin-2-yl)piperidine-2,6-dione (GT-04562)

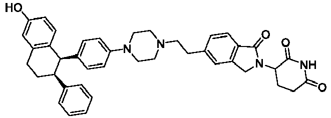

The target compound (GT-04562) was prepared using the method described in Scheme 11 (white solid, 32 mg, yield 35.61%). ¹H NMR (400 MHz, DMSO) δ 10.98 (s, 1H), 10.74 (s, 1H), 9.13 (s, 1H), 7.72 (d, *J* = 7.8 Hz, 1H), 7.53 (s, 1H), 7.44 (d, *J* = 7.8 Hz, 1H), 7.14 (dq, *J* = 14.0, 6.9 Hz, 3H), 6.85 (d, *J* = 6.9 Hz, 2H), 6.66 - 6.55 (m, 4H), 6.49 (dd, *J* = 8.3, 2.3 Hz, 1H), 6.27 (d, *J* = 8.5 Hz, 2H), 5.11 (dd, *J* = 13.2, 5.1 Hz, 1H), 4.38 (dd, *J* = 51.6, 17.5 Hz, 2H), 4.17 (d, *J* = 4.9 Hz, 1H), 3.64 (dd, *J* = 38.3, 11.6 Hz, 4H), 3.38 (s, 2H), 3.30 (s, 1H), 3.23 - 3.07 (m, 4H), 3.03 - 2.87 (m, 5H), 2.60 (d, *J* = 16.9 Hz, 1H), 2.46 - 2.36 (m, 1H), 2.15 - 1.93 (m, 2H), 1.72 (d, *J* = 7.1 Hz, 1H). LCMS (ESI) calcd for C₄₁H₄₃N₄O₄⁺ [M+H]⁺: 655.33, found, 655.3.

### Comparative Example 9: preparation of 3-(5-(2-(4-(4-amino-3-(4-phenoxyphenyl)-1H-pyrazolo[3,4-d]pyrimidin-1-yl)piperidin-1-yl)ethyl)-1-oxoisoindolin-2-yl)piperidine-2,6-dione (GT-04563)

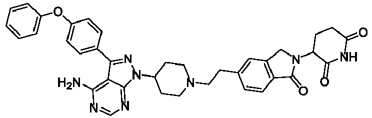

The target compound (GT-04563) was prepared using the method described in Scheme 11 (white solid, 67 mg, yield 74.92%). ¹H NMR (400 MHz, DMSO) δ 10.99 (s, 2H), 8.49 (s, 1H), 7.70 (dd, *J* = 21.5, 8.3 Hz, 3H), 7.57 (s, 1H), 7.52 - 7.40 (m, 3H), 7.17 (tt, *J* = 14.3, 7.0 Hz, 5H), 5.11 (dt, *J* = 12.8, 6.5 Hz, 2H), 4.51 - 4.30 (m, 2H), 3.76 (s, 2H), 3.41 - 3.25 (m, 6H), 2.91 (dd, *J* = 21.6, 9.3 Hz, 1H), 2.61 (d, *J* = 14.7 Hz, 3H), 2.46 - 2.39 (m, 1H), 2.26 (t, *J* = 23.4 Hz, 2H), 2.06 - 1.95 (m, 1H). LCMS (ESI) calcd for C₃₇H₃₇N₈O₄⁺ [M+H]⁺: 657.29, found, 657.3.

### Comparative Example 10: preparation of 3-(5-(3-(4-(4-((1R,2S)-6-hydroxy-2-phenyl-1,2,3,4-tetrahydronaphthalen-1-yl)phenyl)piperazin-1-yl)propyl)-1-oxoisoindolin-2-yl)piperidine-2,6-dione (GT-04564)

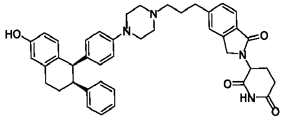

The target compound (GT-04564) was prepared using the method described in Scheme 11 (white solid, 47 mg, yield 51.26%). ¹H NMR (400 MHz, DMSO) δ 10.98 (s, 1H), 10.64 (s, 1H), 9.12 (s, 1H), 7.67 (d, *J* = 7.8 Hz, 1H), 7.49 (s, 1H), 7.40 (d, *J* = 7.9 Hz, 1H), 7.19 - 7.05 (m, 3H), 6.84 (d, *J* = 6.9 Hz, 2H), 6.61 (dd, *J* = 11.8, 8.8 Hz, 4H), 6.48 (dd, *J* = 8.3, 2.3 Hz, 1H), 6.25 (d, *J* = 8.5 Hz, 2H), 5.11 (dd, *J* = 13.3, 5.1 Hz, 1H), 4.37 (dd, *J* = 50.7, 17.3 Hz, 2H), 4.16 (d, *J* = 4.9 Hz, 1H), 3.62 (d, *J* = 11.7 Hz, 2H), 3.50 (d, *J* = 10.5 Hz, 2H), 3.29 (s, 1H), 3.16 - 2.86 (m, 9H), 2.78 (t, *J* = 7.3 Hz, 2H), 2.60 (d, *J* = 17.0 Hz, 1H), 2.44 - 2.31 (m, 1H), 2.15 - 1.94 (m, 4H), 1.71 (d, *J* = 7.3 Hz, 1H). LCMS (ESI) calcd for C₄₂H₄₅N₄O₄⁺ [M+H]⁺: 669.34, found, 669.4.

### Comparative Example 11: preparation of 3-(5-(3-(4-(4-amino-3-(4-phenoxyphenyl)-1H-pyrazolo[3,4-d]pyrimidin-1-yl)piperidin-1-yl)propyl)-1-oxoisoindolin-2-yl)piperidine-2,6-dione (GT-04565)

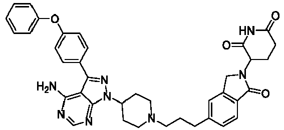

The target compound (GT-04565) was prepared using the method described in Scheme 11 (white solid, 67 mg, yield 73.44%). ¹H NMR (400 MHz, DMSO) δ 10.98 (s, 1H), 10.72 (s, 1H), 8.47 (s, 1H), 7.76 - 7.60 (m, 3H), 7.56 - 7.35 (m, 4H), 7.25 - 7.07 (m, 5H), 5.19 - 4.95 (m, 2H), 4.38 (ddd, *J* = 23.4, 17.3, 6.7 Hz, 2H), 3.42 (s, 2H), 3.26 - 3.16 (m, 2H), 3.09 (s, 2H), 2.96 - 2.87 (m, 1H), 2.81 (t, *J* = 7.4 Hz, 2H), 2.70 - 2.52 (m, 3H), 2.41 (dd, *J* = 13.2, 4.5 Hz, 1H), 2.15 (dd, *J* = 18.0, 10.5 Hz, 4H), 2.05 - 1.96 (m, 1H). LCMS (ESI) calcd for C₃₈H₃₉N₈O₄⁺ [M+H]⁺: 671.31, found, 671.3.

### Biological activity assay

### Reagents and Materials

| | |
|---|---|
| Reagents and materials | Suppliers or Source |
| Human B lymphoma cell: SU-DHL-4 | ATCC (American Type CultureCollection) |
| Human diffuse large B-cell lymphoma cell line: SU-DHL-6 | ATCC |
| Human acute lymphocytic leukemia T lymphocytes: CCRF-CEM | Dalian Meilun Biotech Co., Ltd. |
| Human Burkitt's lymphoma cell line: Daudi | Dalian Meilun Biotech Co., Ltd. |
| Human non-small cell lung cancer cell: HCC827 | ATCC |
| Human liver cancer cell line: HEP3B2.7-1 | Dalian Meilun Biotech Co., Ltd. |
| Human gastric cancer cell: HGC-27 | Dalian Meilun Biotech Co., Ltd. |
| Human acute lymphoblastic leukemia cell line: Kasumi-1 | ATCC |
| Human breast cancer cells: MDA-MB-231 | Dalian Meilun Biotech Co., Ltd. |
| Multiple myeloma cells: MM.1S | ATCC |
| Human B-lymphoma cell: Ramos | Dalian Meilun Biotech Co., Ltd. |
| Human immunoblastic lymphoblastic lymphoma cells: SR | Dalian Meilun Biotech Co., Ltd. |
| Human immunoblastic lymphoblastic lymphoma cells: SR | Dalian Meilun Biotech Co., Ltd. |
| Human breast ductal carcinoma cell: T47D | ATCC |
| Human diffuse large B-cell lymphoma cell line: TMD8 | Kyinno Biotechnology Co., Ltd (test) |
| Human prostate cancer cell: 22RV1 | Dalian Meilun Biotech Co., Ltd. |
| Human non-small cell lung cancer cell: NCI-H1975 | ATCC |
| Human Malignant Meningioma Cell Line: JEKO-1 | ATCC |
| Human M-cell lymphoma cells: Mino | ATCC |
| Human monocytic leukemia cells: THP-1 | ATCC |
| Human myeloid monocytic leukemia cells: MV-4-11 | ATCC |
| dexamethasone-resistant multiple myeloma cell line: MM.1R | ATCC |
| Human prostate cancer cells: Vcap | ATCC |
| Human colon cancer cell: SW620 | ATCC |
| Human prostate cancer cell: DU-145 | ATCC |
| Human ovarian cancer cell: PC-3 | ATCC |
| Human lung adenocarcinoma cell line: PC-9 | Dalian Meilun Biotech Co., Ltd. |
| Human prostate cancer cell: NCI-H358 ATCC | ATCC |
| Human non-small cell lung cancer cell: NCI-H2228 ATCC | ATCC |
| Human bronchioloalveolar adenocarcinoma cell: NCI- ATCC H1666 | ATCC |
| Human prostate cancer cell: LNcap ATCC | ATCC |
| Human chronic myelogenous leukemia cells: K562 ATCC | ATCC |
| Human ovarian cancer cell: NIH: OVCAR3 | Dalian Meilun Biotech Co., Ltd. |
| Human T-lymphocyte leukemia cells: Jurkat | Dalian Meilun Biotech Co., Ltd. |
| Human thyroid cancer cells (undifferentiated): CAL-62 | Dalian Meilun Biotech Co., Ltd. |
| Human gastric cancer cell: MGC-803 | Dalian Meilun Biotech Co., Ltd. |
| RPMI-1640 Medium Dalian Meilun | Biotech Co., Ltd. |
| MEM Medium | Dalian Meilun Biotech Co., Ltd. |
| IMDM Medium | Gibco Company |
| DMEM Medium | Dalian Meilun Biotech Co., Ltd. |
| DMEM medium, high glucose | ATCC |
| L-15 Medium | ATCC |
| F-12K Medium | ATCC |
| EMEM Medium | Dalian Meilun Biotech Co., Ltd. |
| Meilun Cell Counting Kit-8 | Dalian Meilun Biotech Co., Ltd. |
| Fetal bovine serum (FBS) | Sunrise Science Products 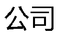 |
| Penicillin-Streptomycin | Beijing TransGen Biotech Co., Ltd. |
| Mycoplasma detection kit | Beijing TransGen Biotech Co., Ltd. |
| STR genotype detection Shanghai Biowing Applied | Biotechnology Co. Ltd. |

### Methods:

### Cell cultures

The tumor cells used herein were cultured in a cell culture medium listed in table 4 at 37°C in an incubator with 5% CO₂. Prior to experimentation, all cells used were correctly identified by STR profiling, and tested negative for mycoplasma through routine screenings.

**Table 4. Tumor cells and cell culture medium used in the present disclosure**

| Cell Line | Cell culture medium used |
|---|---|
| SU-DHL-4 | RPMI 1640 culture medium supplemented with 10% FBS, as well as penicillin at a final concentration of 100 U/mL and streptomycin at a final concentration of 100 µg/mL |
| SU-DHL-6 | |
| CCRF-CEM | |
| Daudi | |
| HCC827 | |
| Hep3B2.7-1 | |
| HGC-27 | |
| Kasumi-1 | |
| MM.1S | |
| Ramos | |
| SNU-182 | |
| SR | |
| T47D | |
| TMD8 | |
| 22RV1 | |
| NCI-H1975 | |
| JEKO-1 | |
| Mino | |
| THP-1 | |
| MM.1R | |
| PC-9 | |
| NCI-H358 | |
| NCI-H2228 | |
| NCI-H1666 | |
| NIH: OVCAR3 | |
| Jurkat | |
| MGC-803 | |
| MDA-MB-231 | DMEM culture medium supplemented with 10% FBS, as well as penicillin at a final concentration of 100 U/mL and streptomycin at a final concentration of 100 µg/mL |
| CAL-62 | |
| MV-4-11 | IMDM culture medium supplemented with 10% FBS, as well as penicillin at a final concentration of 100 U/mL and streptomycin at a final concentration of 100 µg/mL |
| K562 | |
| Vcap | DMEM medium (high glucose) supplemented with 10% FBS, as well as penicillin at a final concentration of 100 U/mL and streptomycin at a final concentration of 100 µg/mL |
| SW620 | L-15 culture medium supplemented with 10% FBS, as well as penicillin at a final concentration of 100 U/mL and streptomycin at a final concentration of 100 µg/mL |
| DU-145 | EMEM culture medium supplemented with 10% FBS, as well as penicillin at a final concentration of 100 U/mL and streptomycin at a final concentration of 100 µg/mL |
| PC-3 | F-12K culture medium supplemented with 10% FBS, as well as penicillin at a final concentration of 100 U/mL and streptomycin at a final concentration of 100 µg/mL |
| LNcap | |

### Determination of half inhibitory concentration (IC₅₀) of the compounds against tumor cells:

IC₅₀ values of the compounds of the present disclosure (including the compounds in Tables 1 and 3, and compounds of examples) were measured using the Meilun Cell Counting Kit-8 kit from Dalian Meilun Biotech Co., Ltd. Assay details are as follows: tumor cells were seeded in a 96-well plate at a density listed in Table 5. After 24h, 100 µL of the inoculated cells were treated with 0.5µL of the compounds of the present disclosure (including the compounds in Tables 1 and 3, and compounds of examples) at 10 different successively decreasing concentrations (starting at the highest concentration of 10µM; 5-fold serial dilutions). After the cells were treated with the compounds of the present disclosure for a period of time, the cell viability detection reagent was added to the culture medium for cell viability assessment according to the instructions provided with the CCK-8 kit. DMSO served as the negative control, and corresponding commercial inhibitors (including Napabucasin ((BBI608), CAS NO.: 83280-65-3); Ibrutinib (CAS NO.: 936563-96-1); compound GT-03308 (CAS NO.: 1679327-21-9); compound GT-03335 (CAS NO.: 1801765-04-7); compound GT-03336 (CAS NO.: 2160546-07-4); compound GT-03334 (CAS NO.: 1801747-42-1); enzalutamide (CAS NO.: 915087-33-1); afatinib (CAS NO.: 439081-18-2); Osimertinib (CAS NO.: 1421373-65-0); AT7519 (CAS NO.: 844442-38-2); Dasatinib (CAS NO.: 302962-49-8); abemaciclib (CAS NO.: 1231929-97-7); Ribociclib (CAS NO.: 1211443-58-1); Palbociclib (CAS NO.: 571190-30-2); brigatinib (CAS NO.: 1197953-54-0); alectmib (CAS NO.: 1256580-46-7); JQ-1 (CAS NO.: 1268524-70-4); Apabetalone (CAS NO.: 1044870-39-4); Fedratinib (CAS NO.: 936091-26-8); Defactinib (CAS NO.: 1073154-85-4); PND-1186 (CAS NO.: 1061353-68-1); PF06650833 (CAS NO.: 1817626-54-2); emavusertib (CAS NO.: 1801344-14-8); Venetoclax (ABT-199, CAS NO.: 1257044-40-8); ABT-263 (Navitoclax, CAS NO.: 923564-51-6); and LDN-212854 (CAS NO.: 1432597-26-6)), were used as the positive control, both of which treated the cells using the same protocol as that of the compounds of the present disclosure. The growth inhibition of the compounds of the present disclosure on cells was plotted by Prism Graphpad software, and the IC₅₀ values of the compounds of the present disclosure were calculated therefrom. Results were shown in Tables 6-21 below.

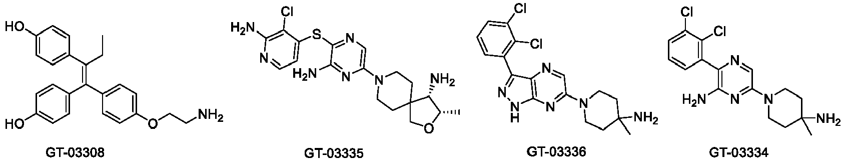

**Table 5. Seeding protocol and treatment time for tumor cells**

| Cells | Cells seeding protocol | The times for treating cells with the compounds of the present disclosure (hours) |
|---|---|---|
| SU-DHL-4 | cells were seeded in 100 µL RPMI1640 medium containing serum at a density of 10,000 cells/well | 72h |
| SU-DHL-6 | | |
| Kasumi-1 | | |
| MM.1S | | |
| Ramos | | |
| SR | | |
| TMD8 | | |
| Mino | | |
| THP-1 | | |
| MM.1R | | |
| MGC-803 | | |
| CAL-62 | | |
| CCRF-CEM | cells were seeded in 100 µL RPMI1640 | 96h |
| Daudi | medium containing serum at a density of 4000 cells/well | |
| HCC827 | | |
| Hep3B2.7-1 | | |
| HGC-27 | | |
| SNU-182 | | |
| T47D | | |
| 22RV1 | | |
| NCI-H1975 | | |
| JEKO-1 | | |
| PC-9 | | |
| NCI-H358 | | |
| NCI-H2228 | | |
| NCI-H1666 | | |
| NIH: OVCAR3 | | |
| Jurkat | | |
| MDA-MB-231 | cells were seeded in 100 µL DMEM medium containing serum at a density of 4000 cells/well | 96h |
| MV-4-11 | cells were seeded in 100 µL IMDM medium containing serum at a density of 10,000 cells/well | 72h |
| K562 | | |
| Vcap | cells were seeded in 100 µL DMEM (high glucose) medium containing serum at a density of 4000 cells/well | 96h |
| SW620 | cells were seeded in 100 µL L-15 medium containing serum at a density of 4000 cells/well | 96h |
| DU-145 | cells were seeded in 100 µL EMEM medium containing serum at a density of 4000 cells/well | 96h |
| PC-3 | cells were seeded in 100 µL F-12K medium containing serum at a density of 4000 cells/well | 96h |
| LNcap | | |

**Table 6. IC₅₀ values (in nM) of the compounds of examples of the present disclosure, designed based on the STAT3 inhibitors, for inhibiting the proliferation of tumor cells**

| Comp. No. /names | CCR F-CEM | Daud i | HCC 827 | Hep 3B2 .7-1 | Kas umi-1 | MDA -MB-231 | MM.1 S | Ra mos | SNU -182 | SR | T47 D | TMD 8 |
|---|---|---|---|---|---|---|---|---|---|---|---|---|
| Napabuc asin | D | C | B | B | C | C | C | B | C | B | B | B |
| GT-03397 | D | D | D | D | - | C | C | D | C | B | B | C |
| GT-03383 | A+ | B | B | B | A+ | A | A+ | B | A+ | A | A+ | A |
| GT-03633 | - | C | D | D | - | D | B | C | - | C | D | - |

**Table 7. IC₅₀ values (in nM) of the compounds of examples of the present disclosure, designed based on the BTK inhibitors, for inhibiting the proliferation of tumor cells**

| Comp. No. /names | 22R V1 | CCRF-CEM | NCI-H1975 | JEK O-1 | Mi no | Kasu mi-1 | TH P-1 | MV-4-11 | MDA-MB-231 | MM .1S | Vca p |
|---|---|---|---|---|---|---|---|---|---|---|---|
| ibrutinib | >10 000 | >10000 | D | D | E | D | >10 000 | C | >10000 | >10 000 | >10 000 |
| GT-03693 | - | A+ | - | - | A+ | - | - | A | B | A+ | A |
| GT-03552 | - | - | - | - | - | - | - | - | - | - | C |
| GT-03636 | - | - | - | - | - | - | A+ | - | - | - | - |
| GT-03326 | A+ | - | A+ | A+ | A+ | - | - | - | A+ | A+ | - |
| GT-03472 | - | A+ | A+ | - | - | A+ | A+ | - | A+ | A+ | A+ |
| GT-03331 | A+ | A+ | A+ | A+ | A+ | A+ | A+ | A+ | A+ | A+ | A+ |
| GT-03297 | A+ | A+ | A+ | A+ | - | A+ | A+ | - | A+ | A+ | A+ |
| GT-03287 | A+ | - | A | A+ | A+ | - | - | - | A+ | A+ | - |
| GT-03264 | A+ | - | A+ | A+ | A+ | - | - | - | A+ | A+ | - |
| GT-02744 | - | - | - | - | - | - | - | B | - | - | - |
| GT-02742 | A+ | - | A+ | A+ | A+ | A+ | A+ | A+ | A+ | A+ | A+ |
| GT-03605 | - | A+ | - | - | - | D | D | - | A+ | A+ | - |
| GT-03473 | - | A+ | A+ | - | A | A | - | - | A | B | A+ |
| GT-02652 | A+ | - | A | A | A+ | A | B | A+ | A+ | A+ | - |
| | | | | | | | | | | | |

**Table 8. IC₅₀ values (in nM) of the compounds of examples of the present disclosure, designed based on the ER inhibitors, for inhibiting the proliferation of tumor cells**

| Comp. No. /names | T47 D | Mi no | SW6 20 | CCR F-CEM | HCC8 27 | Hep3B 2.7-1 | Kasu mi-1 | SU-DH L-6 | MM. 1S | MD A-MB-231 | Vc ap | CA L-62 |
|---|---|---|---|---|---|---|---|---|---|---|---|---|
| GT-03308 | >100 00 | - | - | >100 00 | >1000 0 | >10000 | D | E | >100 00 | A | | |
| GT-03551 | A+ | A+ | A+ | A+ | A+ | B | A+ | A+ | A+ | - | | |
| GT-03638 | A+ | A+ | A+ | A+ | A+ | A+ | A+ | A+ | A+ | A+ | | |
| GT-03214 | A+ | - | A+ | A+ | A+ | A+ | A+ | A+ | A+ | A+ | | |
| GT-02971 | A | - | - | - | - | - | - | - | - | - | | |
| GT-02970 | A+ | - | - | - | - | - | - | - | - | - | | |
| GT-03637 | A | A+ | A | A+ | A | B | A+ | B | A | A | | |
| GT-03797 | - | A+ | - | - | - | - | - | - | - | - | | |
| GT-03774 | - | A+ | A+ | - | - | - | - | - | - | - | | |
| GT-03784 | - | A+ | A+ | - | - | - | - | - | - | - | | |
| GT-03785 | - | A+ | A+ | - | - | - | - | - | - | - | | |
| GT-03786 | - | - | B | - | - | - | - | - | - | - | | |
| GT-03787 | - | B | B | - | - | - | - | - | - | - | | |
| GT-04144 | | | A+ | A+ | | | | | | | A+ | A+ |
| GT-04145 | | | A+ | A+ | | | | | | | A+ | A+ |
| GT-04146 | | | A+ | A+ | | | | | | | A+ | A+ |
| GT-04147 | | | A | A+ | | | | | | | A+ | A+ |
| GT-04148 | | | B | A+ | | | | | | | A+ | A+ |
| GT-03850 | | | A+ | A+ | | | | | | | A+ | A+ |
| GT-03851 | | | A+ | A+ | | | | | | | A+ | A+ |
| GT-03852 | | | A+ | A+ | | | | | | | A+ | A+ |
| GT-03853 | | | B | A+ | | | | | | | A+ | A+ |
| GT-03854 | | | - | A | | | | | | | B | A |
| GT-03855 | | | A | A+ | | | | | | | A+ | A+ |
| GT-03774 | A+ | A+ | | | | | | | A+ | A+ | | A+ |
| GT-03784 | A+ | A+ | | | | | | | A+ | A+ | | A+ |
| GT-03786 | A | | | | | | | | A+ | | | A+ |
| GT-03787 | B | | | | | | | | B | | | B |
| GT-03797 | | | | A+ | | | | | A+ | B | | |
| GT-03785 | | | A+ | A+ | | | | | | | | A+ |
| GT-03786 | | | B | A+ | | | | | | | | A+ |
| GT-03788 | | | C | A+ | | | | | | | | B |

**Table 9. IC₅₀ values (in nM) of the compounds of examples of the present disclosure, designed based on the SHP2 inhibitors, for inhibiting the proliferation of tumor cells**

| Comp. No. /names | HCC827 | Ramos |
|---|---|---|
| GT-03335 | C | >10000 |
| GT-03322 | C | E |
| GT-03336 | D | >10000 |
| GT-03321 | D | - |
| GT-03334 | E | >10000 |
| GT-03320 | - | C |

**Table 10. IC₅₀ values (in nM) of the compounds of examples of the present disclosure, designed based on the AR inhibitors, for inhibiting the proliferation of tumor cells**

| Comp. No. /names | DU-145 | PC-3 |
|---|---|---|
| Enzalutamide | - | - |
| GT-02989 | C | A+ |

**Table 11. IC₅₀ values (in nM) of the compounds of examples of the present disclosure, designed based on the EGFR inhibitors, for inhibiting the proliferation of tumor cells**

| Comp. No. /names | NCI-H358 | HCC8 27 | PC-9 | Min o | THP -1 | CCRF-CEM | SR | Vca p | H19 75 | CAL-62 | CFP AC-1 |
|---|---|---|---|---|---|---|---|---|---|---|---|
| afatinib | D | A+ | A+ | - | - | - | - | - | - | - | - |
| osimertinib | D | A+ | A | - | - | - | - | - | - | - | - |
| GT-03342 | A | B | A+ | D | - | D | - | - | - | - | - |
| GT-03735 | - | - | A | - | - | - | - | - | - | - | - |
| GT-03736 | - | - | A+ | - | - | - | - | - | - | - | - |
| GT-03737 | - | - | A | - | - | - | - | - | - | - | - |
| GT-03808 | - | - | A+ | - | - | - | - | - | - | - | - |
| GT-03738 | - | - | A | - | - | - | - | - | - | - | - |
| GT-03380 | B | - | D | - | - | - | - | - | - | - | - |
| GT-03375 | C | - | A+ | - | - | - | - | - | - | - | - |
| GT-03376 | D | - | B | - | - | - | - | - | - | - | - |
| GT-03411 | A+ | - | A+ | - | - | - | - | - | - | - | - |
| GT-03354 | A+ | - | B | - | - | - | - | - | - | - | - |
| GT-03412 | A+ | - | A+ | - | - | - | - | - | - | - | - |
| GT-03353 | D | - | B | - | - | - | - | - | - | - | - |
| GT-03325 | D | A+ | - | - | - | - | - | - | - | - | - |
| GT-03324 | - | B | - | - | - | - | - | - | - | - | - |
| GT-03323 | D | A | B | - | - | - | - | - | - | - | - |
| GT-03265 | A+ | A+ | A+ | D | - | - | - | - | - | - | - |
| GT-03475 | - | - | B | D | - | - | - | - | - | - | - |
| GT-03548 | - | A | B | B | - | - | - | - | - | - | - |
| GT-03547 | - | - | B | B | - | - | - | - | - | - | - |
| GT-03685 | - | - | A+ | A | - | - | - | - | - | - | - |
| GT-04459 | - | - | - | - | A+ | A+ | A | A+ | - | - | - |
| GT-03924 | - | - | - | - | - | - | - | - | A+ | A+ | B |
| GT-03925 | - | - | - | - | - | - | - | - | A+ | A+ | C |
| GT-03926 | - | - | - | - | - | - | - | - | A+ | B | C |
| GT-03927 | - | - | - | - | - | - | - | - | A+ | A+ | C |
| GT-03928 | - | - | - | - | - | - | - | - | B | D | C |
| GT-03929 | - | - | - | - | - | - | - | - | B | C | D |
| GT-03858 | - | - | - | - | - | - | - | - | A+ | - | B |
| GT-03859 | - | - | - | - | - | - | - | - | A | - | B |
| GT-03860 | - | - | - | - | - | - | - | - | A | - | B |
| GT-03861 | - | - | - | - | - | - | - | - | C | - | B |
| GT-03907 | - | - | - | - | - | - | - | - | B | - | B |
| GT-03908 | - | - | - | - | - | - | - | - | B | - | C |
| GT-03909 | - | - | - | - | - | - | - | - | B | - | D |
| GT-03910 | - | - | - | - | - | - | - | - | B | - | C |
| GT-03911 | - | - | - | - | - | - | - | - | B | - | C |
| GT-03924 | | | | | | | | | A+ | C | B |
| GT-03503 | | | | | | | | | A+ | | |
| GT-03808 | | | A+ | | | B | | | | | |
| GT-03685 | | | A+ | A | | A+ | | | B | | |

**Table 12. IC₅₀ values (in nM) of the compounds of examples of the present disclosure, designed based on the CDK9 inhibitors, for inhibiting the proliferation of tumor cells**

| Comp. No. /names | 22RV 1 | CCRF -CEM | HCC82 7 | JEKO -1 | Lnca p | MDA -MB-231 | Min o | MM.1 S | Ramo s | SR | CAL -62 |
|---|---|---|---|---|---|---|---|---|---|---|---|
| AT751 9 | - | C | C | - | c | B | - | B | B | B | |
| GT-03242 | B | - | B | B | B | B | B | A+ | A | A+ | |
| GT-03448 | - | B | B | - | D | D | - | D | C | C | |
| GT-04044 | | A+ | | | D | | | | | | A |
| GT-04045 | | A+ | | | A | | | | | | A+ |
| GT-04046 | | A+ | | | A | | | | | | A+ |
| GT-04048 | | A | | | D | | | | | | B |
| GT-04061 | | B | | | | | | | | | |

**Table 13. IC₅₀ values (in nM) of the compounds of examples of the present disclosure, designed based on the BCR-ABL inhibitors, for inhibiting the proliferation of tumor cells**

| Comp. No. /names | Mino | K562 | Kasumi -1 | Daudi | TMD8 | CCRF-CEM | CAL-62 | PC-9 |
|---|---|---|---|---|---|---|---|---|
| Dasatinib | A+ | A+ | A+ | B | | | | |
| GT-03215 | A+ | A+ | A+ | B | | | | |
| GT-03545 | A+ | - | A | A+ | | | | |
| GT-03476 | A+ | - | B | A | | | | |
| GT-02969 | D | B | D | A+ | | | | |
| GT-03377 | A+ | D | A+ | A+ | | | | |
| GT-02968 | - | A | D | D | | | A+ | |
| GT-03804 | A+ | - | - | - | | | C | |
| GT-03990 | | | | | A+ | | | |
| GT-04236 | | | | | A | | | |
| GT-04021 | | | | | B | | | |
| GT-04023 | | | | | B | | | |
| GT-03804 | A+ | A+ | A+ | A+ | | A+ | A+ | C |
| GT-03805 | | A+ | B | C | | A+ | C | - |

**Table 14. IC₅₀ values (in nM) of the compounds of examples of the present disclosure, designed based on the CDK4/6 inhibitors, for inhibiting the proliferation of tumor cells**

| Comp. No. /names | Mino | SW620 | SU-DHL-6 | SK-OV-3 | SR | THP-1 | CCRF-CEM | DU-145 | HGC-27 |
|---|---|---|---|---|---|---|---|---|---|
| abemaciclib | A | B | A+ | - | - | - | - | - | |
| Ribociclib | E | >10000 | A | - | - | - | - | - | |
| Palbociclib | D | B | A+ | - | - | - | - | - | |
| GT-03544 | - | - | A+ | - | - | - | - | - | |
| GT-03550 | - | - | A | - | - | - | - | - | |
| GT-02821 | A | C | A | - | - | - | - | - | |
| GT-03688 | B | B | - | - | - | - | - | - | |
| GT-03639 | A+ | A | A+ | - | - | - | - | - | |
| GT-03719 | B | A+ | - | - | - | - | - | - | |
| GT-02812 | - | B | B | - | - | - | - | - | |
| GT-03900 | - | B | A+ | A+ | A+ | A | A | A | A+ |
| GT-03901 | - | B | A+ | A+ | A+ | A | B | A | A+ |
| GT-03902 | - | B | A+ | A+ | A+ | B | A | B | A+ |
| GT-03903 | - | A | A+ | A+ | A+ | B | A+ | B | A+ |
| GT-03904 | - | D | A | B | A | B | B | D | B |
| GT-03663 | | | | | | | A | | |
| GT-03666 | | | | | D | | A | | |
| GT-03688 | | B | | | A+ | | B | | |
| GT-03719 | | A+ | | | A+ | | A+ | | |

**Table 15. IC₅₀ values (in nM) of the compounds of examples of the present disclosure, designed based on the ALK inhibitors, for inhibiting the proliferation of tumor cells**

| Comp. No. /names | NCI-H1975 | NCI-H2228 | HCC827 | MDA-MB-231 | PC-9 | SR | Mino | MV-4-11 |
|---|---|---|---|---|---|---|---|---|
| brigatinib | C | B | B | C | D | A+ | E | |
| alectinib | D | D | - | D | B | A+ | E | |
| GT-03319 | A+ | A | A | A | A | A+ | D | |
| GT-03601 | - | - | B | B | B | A+ | E | |
| GT-03015 | A | B | - | - | - | - | - | |
| GT-02974 | D | - | - | D | D | A | - | |
| GT-02973 | - | B | - | C | C | A+ | - | |
| GT-03529 | - | - | - | B | B | A+ | - | |
| GT-03530 | D | - | - | C | C | A+ | - | |
| GT-03531 | - | - | - | B | D | A+ | - | |
| GT-02972 | C | C | D | C | C | A+ | C | |
| GT-03759 | - | - | - | - | B | - | C | |
| GT-03760 | A | B | - | - | - | - | B | |
| GT-03764 | - | - | - | - | B | - | A | |
| GT-03409 | - | D | D | D | D | A+ | - | |
| GT-03474 | - | - | D | D | C | A+ | - | |
| GT-02811 | B | D | - | - | - | - | - | |
| GT-03602 | - | - | - | - | - | A | - | |
| GT-03775 | | | | | | A+ | | B |
| GT-03776 | | | | | | A+ | | B |
| GT-03777 | | | | | | A+ | | A |
| GT-03778 | | | | | | A+ | | B |
| GT-03779 | | | | | | A+ | | B |
| GT-03780 | | | | | | B | | D |
| GT-03758 | | | | | | A+ | | B |
| GT-03759 | | | | | | A+ | | B |
| GT-03760 | | | | | | A+ | | C |
| GT-03761 | | | | | | A+ | | D |
| GT-03764 | | | | A | | A+ | A | A |

**Table 16. IC₅₀ values (in nM) of the compounds of examples of the present disclosure, designed based on the BET inhibitors, for inhibiting the proliferation of tumor cells**

| Comp. No. /names | 22RV 1 | CCRF -CEM | Hep3B2.7 -1 | Kasumi -1 | Min o | NIH: OVCAR 3 | PC-3 | SNU-182 | SU-DHL -4 |
|---|---|---|---|---|---|---|---|---|---|
| JQ-1 | B | - | - | - | B | - | >1000 0 | - | B |
| GT-03378 | - | A+ | B | B | - | - | - | B | - |
| GT-03296 | B | A+ | B | A+ | A+ | - | A+ | B | B |
| GT-02967 | B | A+ | A | A+ | - | - | - | A | - |
| Apabetalon e | - | - | - | - | - | - | - | - | - |
| GT-03708 | D | A+ | - | - | A+ | B | A+ | - | A |

**Table 17. IC₅₀ values (in nM) of the compounds of examples of the present disclosure, designed based on the RET inhibitors, for inhibiting the proliferation of tumor cells**

| Comp. No. /names | MV-4-11 |
|---|---|
| Fedratinib | A |
| GT-02755 | B |
| GT-02753 | A |
| GT-02746 | B |

**Table 18. IC₅₀ values (in nM) of the compounds of examples of the present disclosure, designed based on the FAK inhibitors, for inhibiting the proliferation of tumor cells**

| Comp. No. /names | CCR F-CEM | Mino | PC-9 | SW 620 | SR | MD A-MB-231 | HGC -27 | MM. 1S | HEP 3B2. 7-1 | Vcap | CAL -62 | MG C80 3 |
|---|---|---|---|---|---|---|---|---|---|---|---|---|
| Defactini b | - | C | D | D | - | - | - | - | - | - | - | - |
| PND-1186 | C | C | C | A+ | B | D | D | - | - | - | - | - |
| GT-03266 | B | D | B | B | A | B | C | - | - | - | - | - |
| GT-03595 | B | - | C | - | A+ | B | B | - | - | - | - | - |
| GT-03596 | B | - | D | - | A | C | C | - | - | - | - | - |
| GT-03597 | B | - | C | - | A | C | C | - | - | - | - | - |
| GT-03213 | B | - | C | C | A+ | A+ | C | - | - | - | - | - |
| GT-03014 | A+ | A+ | D | A+ | B | A | D | - | - | - | - | - |
| GT-03712 | - | B | D | A+ | - | - | - | - | - | - | - | - |
| GT-03716 | A+ | A+ | D | A+ | - | A+ | - | A+ | - | A+ | - | - |
| GT-03705 | A | A+ | D | B | - | A+ | - | - | - | B | - | - |
| GT-03706 | B | B | D | C | - | A+ | - | - | - | B | - | - |
| GT-03732 | - | - | A | D | - | - | - | - | - | - | - | - |
| GT-03672 | - | C | D | D | - | - | - | - | - | - | - | - |
| GT-03728 | - | D | D | B | - | - | - | - | - | - | - | - |
| GT-03792 | - | B | D | A+ | - | - | - | - | - | - | - | - |
| GT-03817 | - | A | - | A+ | - | - | - | - | - | - | - | - |
| GT-03818 | - | A+ | - | A+ | - | - | - | - | - | - | - | - |
| GT-04078 | A+ | - | - | - | - | - | - | A+ | A+ | B | - | - |
| GT-04079 | A+ | - | - | - | - | - | - | A+ | A+ | A+ | - | - |
| GT-04080 | A+ | - | - | - | - | - | - | A+ | A+ | A+ | - | - |
| GT-04090 | - | - | - | - | - | - | - | - | - | - | A | A+ |
| GT-04091 | - | - | - | - | - | - | - | - | - | - | A+ | A |
| GT-04092 | - | - | - | - | - | - | - | - | - | - | B | A+ |
| GT-04093 | - | - | - | - | - | - | - | - | - | - | A | A+ |
| GT-03940 | - | - | - | A | - | - | - | - | - | C | A+ | |
| GT-03941 | - | - | - | A+ | - | - | - | - | - | B | A+ | |
| GT-03942 | - | - | - | A+ | - | - | - | - | - | A+ | A+ | A+ |
| GT-04078 | A+ | - | - | B | - | - | - | - | - | D | A+ | - |
| GT-04079 | A+ | - | - | A+ | - | - | - | - | - | A+ | A+ | B |
| GT-04080 | A+ | - | - | A+ | - | - | - | - | - | A+ | A+ | A |
| GT-04083 | B | - | - | - | - | - | - | - | - | - | - | - |
| GT-03677 | | | | | | A+ | | | | | | |
| GT-03678 | | | | | | A+ | | | | | | |
| GT-03690 | | | | | | A+ | | | | | | |
| GT-03709 | | | | | | D | | | | | | |
| GT-03712 | | | | | | A+ | | | | | | |
| GT-03718 | | | | | B | D | | | | | B | |
| GT-03672 | | | | | C | C | | | | | c | |
| GT-03728 | | | | | A+ | A+ | | | | | A | |
| GT-03729 | | | | | A+ | A | | | | | A | |
| GT-03730 | | | | | A+ | A | | | | | A+ | |
| GT-03731 | | | | | A | D | | | | | C | |
| GT-03732 | | | | | B | | | | | | | |
| GT-03679 | C | | | B | | A+ | | B | | | A | |
| GT-03742 | | | | | | D | | D | | | D | |
| GT-03792 | A+ | | | A+ | | A+ | | A+ | | | A+ | |
| GT-03817 | A+ | | | A+ | | A+ | | A+ | | | A+ | |
| GT-03818 | A+ | | | A+ | | A+ | | A+ | | | A+ | |

**Table 19. IC₅₀ values (in nM) of the compounds of examples of the present disclosure, designed based on the IRAK4 inhibitors, for inhibiting the proliferation of tumor cells**

| Comp. No. /names | NCI-H16 66 | Mino | SU-DHL -4 | SW6 20 | Jurka t | CCR F-CEM | Lnc ap | MM.1 R | CAL -62 | Kasu mi-1 | Hep3 B2.7 -1 | MV-4-11 |
|---|---|---|---|---|---|---|---|---|---|---|---|---|
| PF066 | >100 | E | >100 | >100 | - | - | - | - | - | - | - | |
| 50833 | 00 | | 00 | 00 | | | | | | | | |
| emavu sertib | >100 00 | >100 00 | >100 00 | >100 00 | - | - | - | - | - | - | - | |
| GT-03809 | C | A+ | C | A+ | A+ | A+ | - | A+ | A+ | A+ | - | A+ |
| GT-03810 | B | A+ | B | A+ | A+ | A+ | - | A+ | A+ | A+ | - | A+ |
| GT-03811 | A | A+ | B | A+ | A+ | A+ | - | A+ | A+ | A+ | - | A+ |
| GT-03812 | - | B | D | D | - | - | - | - | - | - | - | B |
| GT-03815 | - | D | E | - | - | - | - | - | - | - | - | |
| GT-04506 | - | - | - | - | A | B | B | - | - | - | - | |
| GT-04518 | - | - | - | - | A+ | - | - | A | B | - | - | |
| GT-04519 | - | - | - | - | A+ | - | - | A+ | A+ | - | - | |
| GT-04267 | - | - | - | - | C | - | - | - | - | B | B | |
| GT-04268 | - | - | - | - | C | - | - | - | - | B | C | |
| GT-04269 | - | - | - | - | A | B | B | B | - | A | A+ | |
| GT-04270 | - | - | - | - | B | - | - | - | - | B | C | |
| GT-04271 | - | - | - | - | A+ | A+ | A | A+ | - | A+ | A+ | |
| GT-04272 | - | - | - | - | D | - | B | C | - | A | C | |
| GT-04088 | - | - | - | B | A+ | A+ | B | - | A+ | - | - | |
| GT-04120 | - | - | A+ | A+ | A+ | A+ | A+ | - | A+ | - | - | |
| GT-04098 | - | - | A+ | A+ | A+ | A+ | A+ | - | A+ | - | - | |
| GT-04099 | - | - | B | A+ | A+ | A+ | A+ | - | A+ | - | - | |
| GT-04154 | - | - | A+ | A+ | A+ | A+ | A+ | - | A+ | - | - | |
| GT-04171 | - | - | A+ | A+ | A+ | A+ | A+ | - | A+ | - | - | |
| GT-04172 | - | - | B | A+ | A+ | A+ | A+ | - | A+ | - | - | |
| GT-04173 | - | - | - | D | C | D | B | - | D | - | - | |
| GT-04174 | - | - | - | B | A | A+ | C | - | A+ | - | - | |
| GT-04077 | - | - | C | B | A+ | A+ | B | - | A+ | - | - | |
| GT- | - | - | A+ | A+ | A+ | A+ | A+ | - | A+ | - | - | |
| 04153 | | | | | | | | | | | | |
| GT-03841 | | | | | | | | | | | | B |
| GT-03842 | | | | | | | | | | | | B |
| GT-03843 | | | | | | A+ | | | | | | A |
| GT-03844 | | | | | | | | | | | | B |
| GT-03845 | | | | | | | | | | | | C |

**Table 20. IC₅₀ values (in nM) of the compounds of examples of the present disclosure, designed based on the BCL-2 inhibitors, for inhibiting the proliferation of tumor cells**

| Comp. No. /names | CCRF-CEM | HCC827 | LNcap | MDA-MB-231 | MM.1R | MM.1S | SR |
|---|---|---|---|---|---|---|---|
| ABT-199 | >10000 | E | D | D | - | >10000 | C |
| ABT-263 | - | - | - | - | - | - | - |
| GT-03478 | A+ | A+ | A+ | A+ | A+ | A+ | A+ |
| GT-03479 | A+ | B | A+ | A+ | A+ | A+ | A+ |

**Table 21. IC₅₀ values (in nM) of the compounds of examples of the present disclosure, designed based on the ALK2 inhibitors, for inhibiting the proliferation of tumor cells**

| Comp. No. /names | Vcap |
|---|---|
| LDN-212854(CAS NO.: 1432597-26-6) | B |
| GT-03442 | B |

**Table 22. IC₅₀ values (in nM) of the compounds of examples of the present disclosure, designed based on the NTRK inhibitors, for inhibiting the proliferation of tumor cells**

| Comp. No. /names | Kasumi -1 | Jurkat | HEP3B2.7 -1 | MM.1 R | T47D | MM.1S | CCRF-CEM | Vcap |
|---|---|---|---|---|---|---|---|---|
| larotrectini b | >10000 | >1000 0 | >10000 | >10000 | >10000 | >10000 | >10000 | |
| GT-03867 | A+ | A+ | A+ | A+ | B | A | A+ | |
| GT-04262 | B | B | A+ | A+ | - | - | A+ | |
| GT-04263 | C | B | A+ | A+ | - | - | A | |
| GT-04264 | A+ | A+ | A+ | A+ | A+ | A+ | A+ | |
| GT-03866 | | A+ | | | | | A+ | A |

| Table 23. Inhibitory activity (IC₅₀, nM) of the examples compounds of the present disclosure against tumor cell proliferation compared to Comparative Example compounds | | | |
|---|---|---|---|
| Comp. No. /names | MM.1R | CAL-62 | Jurkat |
| GT-03338 | C | D | B |
| GT-04512(Comparative Example ) | D | D | B |
| GT-04457(Comparative Example ) | E | E | B |
| GT-03319 | B | A+ | D |
| GT-04513(Comparative Example ) | B | D | D |
| GT-02742 | A+ | A+ | A+ |
| GT-04563 (Comparative Example ) | A+ | E | D |
| GT-04565(Comparative Example ) | >10000 | >10000 | E |
| GT-03850 | A+ | A+ | A+ |
| GT-04562(Comparative Example ) | E | D | B |
| GT-04564(Comparative Example ) | D | D | B |

| | | | |
|---|---|---|---|
| Note: In Tables 6-23, the symbols A+, A, B, C, D, and E are defined as follows: A+ ≤ 50 nM; 50 nM < A ≤ 100 nM; 100 nM < B ≤ 500 nM; 500 nM < C ≤ 1000 nM; 1000 nM < D ≤ 5000 nM; 5000 nM < E ≤ 10000 nM. | | | |

The results indicate that the compounds of the present invention (including the compounds in Tables 1 and 3, and the compounds of Examples) can inhibit the proliferation of tumor cells (as shown in Tables 6-23), with inhibitory effects superior to or comparable with the corresponding positive control drugs, as well as degradation effect better than that of the corresponding positive control drugs.

The basic principles, main features and advantages of the present disclosure are shown and described above. Those skilled in the art should understand that the present disclosure is not limited by the foregoing embodiments, and they can make various changes, substitutions and alterations herein without departing from the spirit and scope of the present disclosure. These changes, substitutions and alterations fall within the scope of the present disclosure. The claimed scope of the present disclosure is defined by the appended claims and their equivalents.

## Claims

1. A compound of Formula (I) 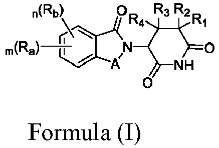
or salts, enantiomers, stereoisomers, solvates, isotopically enriched analogs, prodrugs, or polymorphs thereof,
wherein A represents CO, CH₂ or CD₂;
R₁, R₂, R₃ and R₄ are the same or different and independently represent hydrogen, deuterium, halogen, optionally deuterated C₁₋₆ alkyl, optionally deuterated C₁₋₆ alkoxy, or halogenated C₁₋₆ alkyl;
(Rₐ)ₘ indicates that benzene ring is substituted with m Rₐ substituents, with each Rₐ being the same or different and independently representing the structure of the following formula: 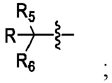
wherein R represents chlorine, bromine, iodine, hydroxy, leaving group, NR₇R₈ or a group W¹,
wherein R₇ and R₈ are the same or different and independently represent hydrogen, C₁₋₆ alkyl, C₂₋₆ alkynyl-C₁₋₆ alkylene-, C₂₋₆ alkenyl-C₁₋₆ alkylene-, optionally substituted 4- to 15-membered nitrogen-containing heterocyclyl, or R^{2a}-R^{1a}-, where R^{1a} is optionally substituted C₁₋₆ alkylene or optionally substituted C₂₋₆ alkenylene, R^{2a} represents halogen, amino, hydroxy, carboxyl, C₂₋₆ alkynyl, C₂₋₆ alkenyl or a leaving group; and
W¹ represents the structure of the following formula: 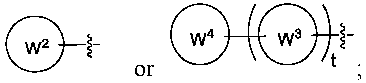 wherein ring W² represents optionally substituted nitrogen-containing heterocyclyl, each ring W³ is the same or different and independently represents optionally substituted nitrogen-containing heterocyclylene, t represents an integer of 1 or 2, and ring W⁴ represents optionally substituted aryl, optionally substituted heteroaryl or optionally substituted heterocyclyl;
R₅ and R₆ are the same or different and independently represent hydrogen, fluorine, chlorine, bromine, iodine, optionally substituted methyl, or optionally substituted linear or branched C₂₋₃₀ alkyl, wherein one or more groups Rₑ and/or one or more groups R_{d} and/or any combination of one or more groups R_{c} and R_{d} are optionally inserted into the backbone carbon chain of the linear or branched C₂₋₃₀ alkyl group, wherein carbon-carbon bond between one or more pairs of adjacent carbon atoms in the backbone carbon chain is interrupted by the group R_{c}, R_{d}, or a combination of R_{c} and R_{d}; wherein each R_{c} is selected from the group consisting of O, N(Rₑ), C(O), C(O)O, S(O), S(O)₂, S(O)₂NH, NHS(O)₂, OC(O), C(O)N(Rₑ), N(Rₑ)C(O), or N(Rₑ)C(O)N(Rₑ), where each Rₑ independently represents H or C₁₋₆ alkyl, and in case that two or more groups R_{c} are inserted into the backbone carbon chain of the linear or branched C₂₋₃₀ alkyl group, the two or more groups R_{c} are not directly connected to each other; and wherein each R_{d} is selected from the group consisting of cycloalkylene, arylene, heterocyclylene, heteroarylene, alkynylene, alkenylene, or any combination thereof, wherein the cycloalkylene, arylene, heterocyclylene, and heteroarylene are independently optionally substituted with a substituent selected from the group consisting of deuterium, optionally deuterated C₁₋₆ alkyl, optionally deuterated C₃₋₆ cycloalkyl, hydroxy, amino, mercapto, halogen, cyano, optionally deuterated C₁₋₆ alkoxy, optionally deuterated C₁₋₆ alkyl-NH-, halogenated C₁₋₆ alkyl, NH₂-C₁₋₆ alkylene, optionally deuterated C₁₋₆ alkyl-NHC(O)-, optionally deuterated C₁₋₆ alkyl-C(O)NH-, or any combination thereof;
or
R and R₅, together with the carbon atom to which they are connected, form carbonyl group, R₆ represents hydrogen, optionally substituted methyl, or optionally substituted linear or branched C₂₋₃₀ alkyl, wherein one or more groups R_{c} and/or one or more groups R_{d} and/or any combination of one or more groups R_{c} and R_{d} are optionally inserted into the backbone carbon chain of the linear or branched C₂₋₃₀ alkyl group, wherein carbon-carbon bond between one or more pairs of adjacent carbon atoms in the backbone carbon chain is interrupted by the group R_{c}, R_{d}, or a combination of R_{c} and R_{d}; wherein each R_{c} is selected from the group consisting of O, N(Rₑ), C(O), C(O)O, S(O), S(O)2, S(O)₂NH, NHS(O)₂, OC(O), C(O)N(Rₑ), N(Rₑ)C(O), or N(Rₑ)C(O)N(Rₑ), where each Rₑ independently represents H or C₁₋₆ alkyl, and in case that two or more groups R_{c} are inserted into the backbone carbon chain of the linear or branched C₂₋₃₀ alkyl group, the two or more groups R_{c} are not directly connected to each other; and wherein each R_{d} is selected from the group consisting of cycloalkylene, arylene, heterocyclylene, heteroarylene, alkynylene, alkenylene, or any combination thereof, wherein the cycloalkylene, arylene, heterocyclylene, and heteroarylene are independently optionally substituted with a substituent selected from the group consisting of deuterium, optionally deuterated C₁₋₆ alkyl, optionally deuterated C₃₋₆ cycloalkyl, hydroxy, amino, mercapto, halogen, optionally deuterated C₁₋₆ alkoxy, optionally deuterated C₁₋₆ alkyl-NH-, halogenated C₁₋₆ alkyl, NH₂-C₁₋₆ alkylene, optionally deuterated C₁₋₆ alkyl-NHC(O)-, optionally deuterated C₁₋₆ alkyl-C(O)NH-, cyano, or any combination thereof;
(R_{b})ₙ indicates that the benzene ring is substituted with n R_{b} substituents, with each R_{b} being the same or different and independently representing deuterium, halogen, hydroxy, mercapto, nitro, amino, cyano, optionally deuterated C₁₋₆ alkyl, optionally deuterated C₁₋₆ alkoxy, halogenated C₁₋₆ alkyl, optionally substituted C₃₋₆ cycloalkyl, C₂₋₆ alkenyl or C₂₋₆ alkynyl; and
m represents an integer of 1, 2, 3, or 4, n represents an integer of 0, 1, 2, or 3, and the sum of m and n is an integer of 1, 2, 3, or 4;
wherein when R and R₅ together with the carbon atom to which they are connected form a carbonyl group and R₆ represents hydrogen, m represents an integer of 1, 2, or 3, n represents an integer of 1, 2, or 3, and the sum of m and n is an integer of 2, 3, or 4;
with the proviso that the following compounds and compounds of Formula (I') are excluded: 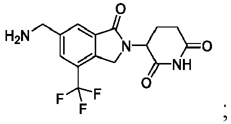 and
when A represents CH₂ or C(O) and R represents unsubstituted piperazinyl, m represents an integer of 1 and n represents an integer of 0;
when A represents CH₂ or C(O) and Rₐ represents H₂N-CH₂-, m represents an integer of 1 and n represents an integer of 0;
when A represents C(O) and R represents bromine, m represents an integer of 1 and n represents an integer of 0; and
the compounds of Formula (I'): 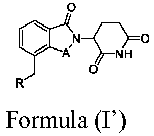
wherein in Formula (I'), A represents CH₂ or C(O), and R represents unsubstituted piperidinyl or methylamino substituted piperidinyl.

2. The compound of Formula (I) or salts, enantiomers, stereoisomers, solvates, isotopically enriched analogs, prodrugs, or polymorphs thereof as claimed in claim 1, wherein the compound of Formula (I) is also of Formula (I-1) or Formula (I-2): 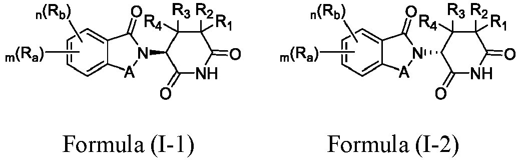 wherein A, R₁, R₂, R₃, R₄, Rₐ, R_{b}, m, and n are as defined in claim 1.

3. The compound of Formula (I) or salts, enantiomers, stereoisomers, solvates, isotopically enriched analogs, prodrugs, or polymorphs thereof as claimed in claim 1 or 2, wherein R represents:
chlorine, bromine, iodine, hydroxy, or leaving group; or
NR₇R₈, wherein R₇ and R₈ are the same or different and each independently represent hydrogen, C₁₋₆ alkyl, C₂₋₆ alkynyl-C₁₋₆ alkylene, C₂₋₆ alkenyl-C₁₋₆ alkylene, optionally substituted 4- to 15-membered nitrogen-containing monocyclic heterocyclyl, optionally substituted 4- to 15-membered nitrogen-containing bridged heterocyclyl, optionally substituted 4- to 15-membered nitrogen-containing spiro-heterocyclyl, or R^{2a}-R^{1a}-, wherein R^{1a} represents optionally substituted C₁₋₆ alkylene or optionally substituted C₂₋₆ alkenylene, R^{2a} represents halogen, amino, hydroxy, carboxyl, CHO, C₂₋₆ alkynyl, C₂₋₆ alkenyl or leaving group, and the 4- to 15-membered nitrogen-containing monocyclic heterocyclyl, the 4- to 15-membered nitrogen-containing bridged heterocyclyl, and the 4- to 15-membered nitrogen-containing spiro-heterocyclyl are each optionally substituted with one or more substituents selected from the group consisting of deuterium, C₁₋₆ alkyl, C₁₋₆ alkoxy, halogen, leaving group, amino, hydroxy, carboxyl, CHO, C₂₋₆ alkynyl, C₂₋₆ alkenyl, R^{4a}-R^{3a}-, or any combination thereof, where R^{3a} represents optionally substituted C₁₋₆ alkylene or optionally substituted C₂₋₆ alkenylene, R^{4a} represents halogen, R^{5a}R^{6a}N-, hydroxy, carboxyl, CHO, ethynyl, vinyl, or leaving group, wherein R^{5a} and R^{6a} are the same or different and each independently represent hydrogen or C₁₋₃ alkyl; or
mercapto, cyano, optionally deuterated C₁₋₆ alkyl-NH-, halogenated C₁₋₆ alkyl, NH₂-C₁₋₆ alkylene, optionally deuterated C₁₋₆ alkyl-NHC(O)-, optionally deuterated C₁₋₆ alkyl-C(O)NH-, or any combination thereof;
W¹ represented by the structure of the following formula: 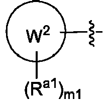
wherein ring W² represents 4- to 20-membered nitrogen-containing heterocyclyl, (R^{a1})ₘ₁ indicates that ring W² is optionally substituted with m1 R^{a1} substituents, with each R^{a1} independently representing deuterium, optionally deuterated C₁₋₆ alkyl, C₁₋₆ alkoxy, halogen, leaving group, amino, hydroxy, mercapto, cyano, carboxyl, CHO, C₂₋₆ alkynyl, C₂₋₆ alkenyl, optionally deuterated C₃₋₆ cycloalkyl, or R^{8a}-R^{7a}-, where R^{7a} is optionally substituted C₁₋₆ alkylene or optionally substituted C₂₋₆ alkenylene, and R^{8a} represents halogen, R^{9a}R^{10a}N-, hydroxy, carboxyl, CHO, ethynyl, vinyl, or leaving group, wherein R^{9a} and R^{10a} are the same or different and each independently represent hydrogen or C₁₋₃ alkyl; or 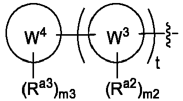
wherein each ring W³ is the same or different and independently represents 4- to 20-membered nitrogen-containing heterocyclylene, t represents an integer of 1 or 2, (R^{a2})ₘ₂ indicates that each ring W³ is optionally substituted with m2 R^{a2} substituents, with each R^{a2} independently representing deuterium, optionally deuterated C₁₋₆ alkyl, C₁₋₆ alkoxy, halogen, leaving group, amino, hydroxy, mercapto, cyano, carboxyl, CHO, C₂₋₆ alkynyl, C₂₋₆ alkenyl, optionally deuterated C₃₋₆ cycloalkyl, or R^{12a}-R^{11a}-, where R^{11a} is optionally substituted C₁₋₆ alkylene or optionally substituted C₂₋₆ alkenylene, and R^{12a} represents halogen, R^{13a}R^{14a}N-, hydroxy, carboxyl, CHO, ethynyl, vinyl, or leaving group, wherein R^{13a} and R^{14a} are the same or different and each independently represent hydrogen or C₁₋₃ alkyl; and
ring W⁴ represents 4- to 15-membered nitrogen-containing heterocyclyl, 6- to 10-membered aryl, or 5- to 10-membered heteroaryl, (R^{a3})ₘ₃ indicates that ring W⁴ is optionally substituted with m3 R^{a3} substituents, and each R^{a3} independently represents deuterium, optionally deuterated C₁₋₆ alkyl, C₁₋₆ alkoxy, halogen, leaving group, amino, hydroxy, mercapto, cyano, carboxyl, CHO, C₂₋₆ alkynyl, C₂₋₆ alkenyl, optionally deuterated C₃₋₆ cycloalkyl, or R^{16a}-R^{15a}-, where R^{15a} is optionally substituted C₁₋₆ alkylene or optionally substituted C₂₋₆ alkenylene, and R^{16a} represents halogen, R^{17a}R^{18a}N-, hydroxy, carboxyl, CHO, ethynyl, vinyl, or leaving group, wherein R^{17a} and R^{18a} are the same or different and each independently represent hydrogen or C₁₋₃ alkyl;
wherein m1, m2, and m3 independently represent an integer of 0-20.

4. The compound of Formula (I) or salts, enantiomers, stereoisomers, solvates, isotopically enriched analogs, prodrugs, or polymorphs thereof as claimed in claim 1 or 2, wherein
R represents NR₇R₈, where R₇ and R₈ are the same or different and each independently represent:
hydrogen, C₁₋₆ alkyl, C₂₋₆ alkynyl-C₁₋₆ alkylene, C₂₋₆ alkenyl-C₁₋₆ alkylene; or
optionally substituted 4- to 15-membered nitrogen-containing monocyclic heterocyclyl, optionally substituted 4- to 15-membered nitrogen-containing bridged heterocyclyl, or optionally substituted 4- to 15-membered nitrogen-containing spiro-heterocyclyl, e.g.,
piperidinyl, piperazinyl, morpholinyl, azetidinyl, pyrrolidinyl, imidazolidinyl, pyrazolidyl, oxazolidinyl, thiazolidinyl, thiomorpholinyl, azepanyl, diazacycloheptanyl, azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl, diazabicyclo[2.2.2]octanyl, 3-azaspiro[5.5]undecan-3-yl, 8-azaspiro[4.5]decan-4-yl, 2-oxa-8-azaspiro[4.5]decan-4-yl, or 3-methyl-2-oxa-8-azaspiro[4.5]decan-4-yl, or 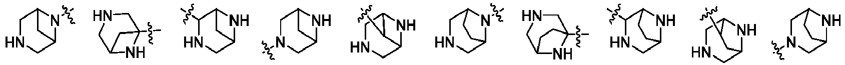 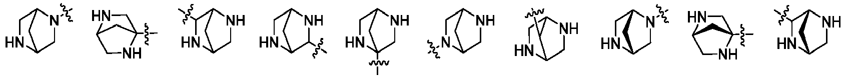 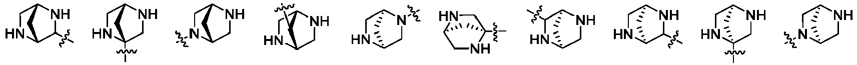 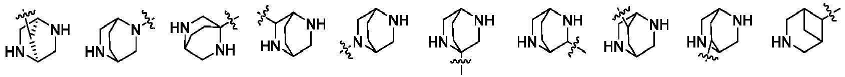 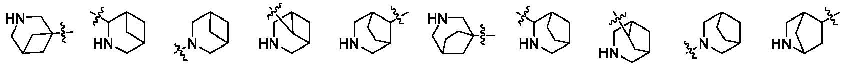 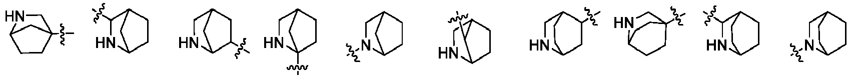 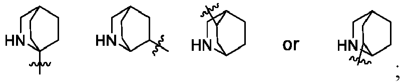 or
R^{2a}-R^{1a}-, where R^{1a} represents optionally substituted C₁₋₆ alkylene or optionally substituted C₂₋₆ alkenylene, and R^{2a} represents halogen, amino, hydroxy, carboxyl, CHO, C₂₋₆ alkynyl, C₂₋₆ alkenyl, or leaving group,
wherein the 4- to 15-membered nitrogen-containing monocyclic heterocyclyl, the 4- to 15-membered nitrogen-containing bridged heterocyclyl and the 4- to 15-membered nitrogen-containing spiro-heterocyclyl are each optionally substituted with one or more substituents selected from the group consisting of deuterium, C₁₋₆ alkyl, C₁₋₆ alkoxy, halogen, leaving group, amino, hydroxy, carboxyl, CHO, C₂₋₆ alkynyl, C₂₋₆ alkenyl, R^{4a}-R^{3a}-, or any combination thereof, where R^{3a} represents optionally substituted C₁₋₆ alkylene or optionally substituted C₂₋₆ alkenylene, R^{4a} represents halogen, R^{5a}R^{6a}N-, hydroxy, carboxyl, CHO, ethynyl, vinyl, or leaving group, wherein R^{5a} and R^{6a} are the same or different and each independently represent hydrogen or C₁₋₃ alkyl; or R represents W¹ represented by the structure of the following formula: 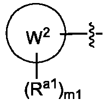
wherein ring W² represents 4- to 20-membered nitrogen-containing heterocyclyl, e.g.,
piperidinyl, piperazinyl, morpholinyl, azetidinyl, pyrrolidinyl, imidazolidinyl, pyrazolidyl, oxazolidinyl, thiazolidinyl, thiomorpholinyl, azepanyl, diazacycloheptanyl, azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl, diazabicyclo[2.2.2]octanyl, 3-azaspiro[5.5]undecan-3-yl, 8-azaspiro[4.5]decan-4-yl, 2-oxa-8-azaspiro[4.5]decan-4-yl, or 3-methyl-2-oxa-8-azaspiro[4.5]decan-4-yl, or 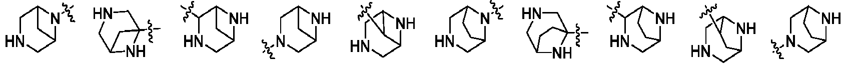 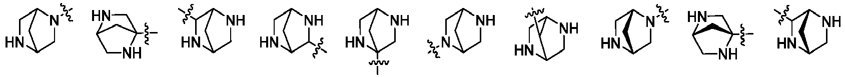 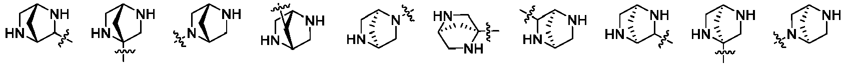 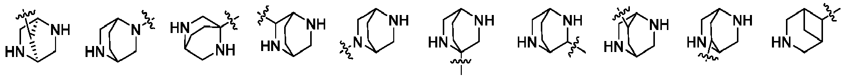 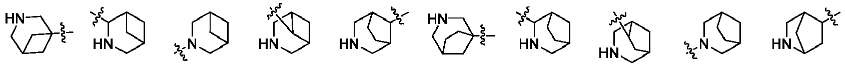 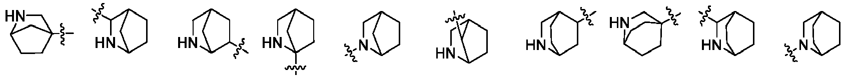 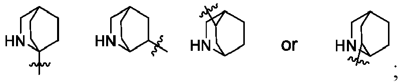
(R^{a1})ₘ₁ indicates that ring W² is optionally substituted with m1 R^{a1} substituents, with each R^{a1} independently representing deuterium, optionally deuterated C₁₋₆ alkyl, C₁₋₆ alkoxy, halogen, leaving group, amino, hydroxy, mercapto, cyano, carboxyl, CHO, C₂₋₆ alkynyl, C₂₋₆ alkenyl, optionally deuterated C₃₋₆ cycloalkyl, or R^{8a}-R^{7a}-, where R^{7a} is optionally substituted C₁₋₆ alkylene or optionally substituted C₂₋₆ alkenylene, and R^{8a} represents halogen, R^{9a}R^{10a}N-, hydroxy, carboxyl, CHO, ethynyl, vinyl, or leaving group, wherein R^{9a} and R^{10a} are the same or different and each independently represent hydrogen or C₁₋₃ alkyl; or
W¹ represented by the structure of the following formula: 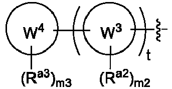
wherein each ring W³ is the same or different and independently represents 4- to 20-membered nitrogen-containing heterocyclylene, e.g.,
piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, 3-azaspiro[5.5]undecan-3-ylene, 8-azaspiro[4.5]decan-4-ylene, 2-oxa-8-azaspiro[4.5]decan-4-ylene, or 3-methyl-2-oxa-8-azaspiro[4.5]decan-4-ylene, or 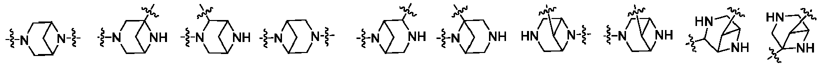 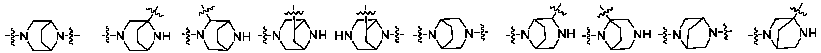 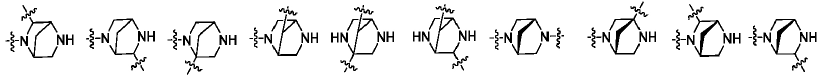 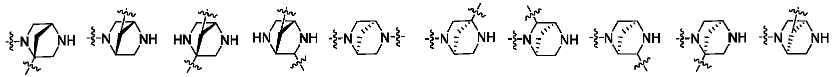 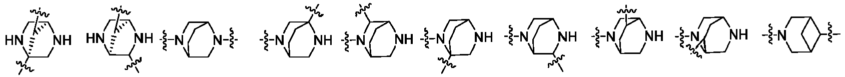 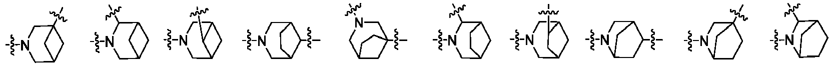 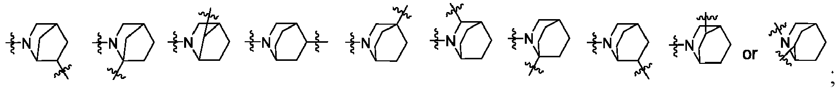
t represents an integer of 1 or 2, (R^{a2})ₘ₂ indicates that each ring W³ is optionally substituted with m2 R^{a2} substituents, with each R^{a2} independently representing deuterium, optionally deuterated C₁₋₆ alkyl, C₁₋₆ alkoxy, halogen, leaving group, amino, hydroxy, mercapto, cyano, carboxyl, CHO, C₂₋₆ alkynyl, C₂₋₆ alkenyl, optionally deuterated C₃₋₆ cycloalkyl, or R^{12a}-R^{11a}-, where R^{11a} is optionally substituted C₁₋₆ alkylene or optionally substituted C₂₋₆ alkenylene, and R^{12a} represents halogen, R^{13a}R^{14a}N-, hydroxy, carboxyl, CHO, ethynyl, vinyl, or leaving group, wherein R^{13a} and R^{14a} are the same or different and each independently represent hydrogen or C₁₋₃ alkyl; and
ring W⁴ represents 4- to 15-membered nitrogen-containing heterocyclyl, 6- to 10-membered aryl, or 5- to 10-membered heteroaryl, e.g.,
piperidinyl, piperazinyl, morpholinyl, azetidinyl, pyrrolidinyl, imidazolidinyl, pyrazolidyl, oxazolidinyl, thiazolidinyl, thiomorpholinyl, azepanyl, diazacycloheptanyl, azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl, diazabicyclo[2.2.2]octanyl, 3-azaspiro[5.5]undecan-3-yl, 8-azaspiro[4.5]decan-4-yl, 2-oxa-8-azaspiro[4.5]decan-4-yl, 3-methyl-2-oxa-8-azaspiro[4.5]decan-4-yl, phenyl, naphthyl, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, or imidazo[2,1-b]thiazolyl; or 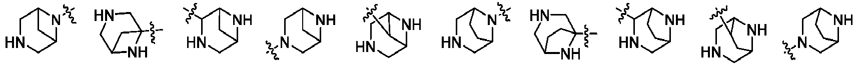 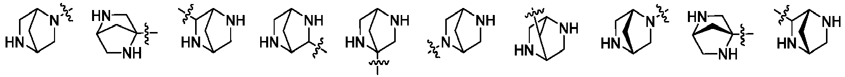 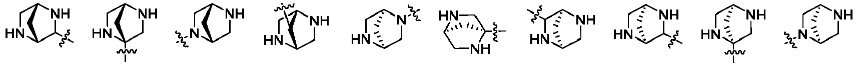 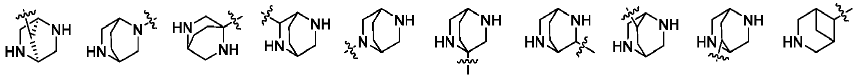 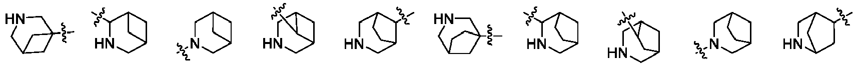 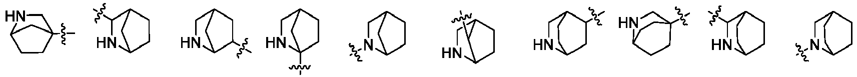 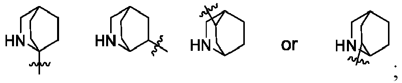
(R^{a3})ₘ₃ indicates that ring W⁴ is optionally substituted with m3 R^{a3} substituents, with each R^{a3} independently representing deuterium, optionally deuterated C₁₋₆ alkyl, C₁₋₆ alkoxy, halogen, leaving group, amino, hydroxy, mercapto, cyano, carboxyl, CHO, C₂₋₆ alkynyl, C₂₋₆ alkenyl, optionally deuterated C₃₋₆ cycloalkyl, or R^{16a}-R^{15a}-, where R^{15a} is optionally substituted C₁₋₆ alkylene or optionally substituted C₂₋₆ alkenylene, and R^{16a} represents halogen, R^{17a}R^{18a}N-, hydroxy, carboxyl, CHO, ethynyl, vinyl, or leaving group, wherein R^{17a} and R^{18a} are the same or different and each independently represent hydrogen or C₁₋₃ alkyl;
wherein m1, m2, and m3 each independently represent an integer of 0-20.

5. The compound of Formula (I) or salts, enantiomers, stereoisomers, solvates, isotopically enriched analogs, prodrugs, or polymorphs thereof as claimed in claim 1, wherein R represents the following groups:
chlorine, bromine, iodine, hydroxy, -N₃, sulfonate, e.g., methanesulfonyloxy (MsO-), trifluoromethanesulfonyloxy (TfO-), p-toluenesulfonyloxy (TsO-), NH₂, or 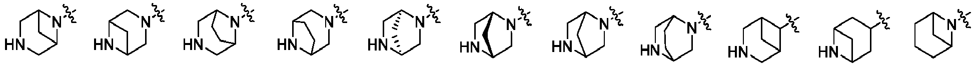 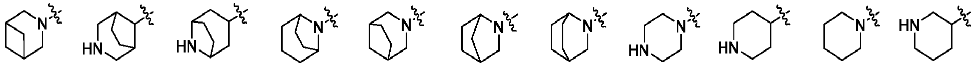 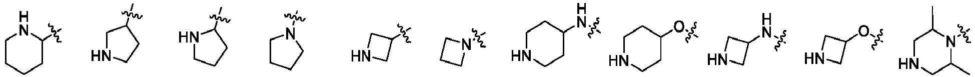 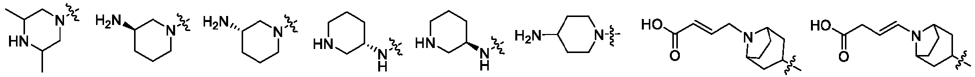 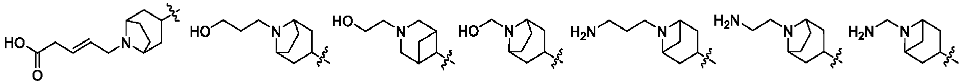 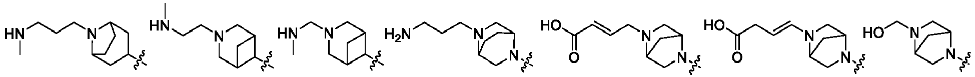 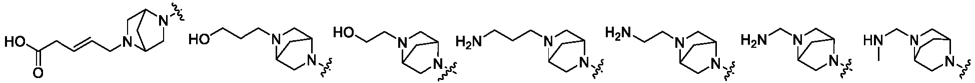 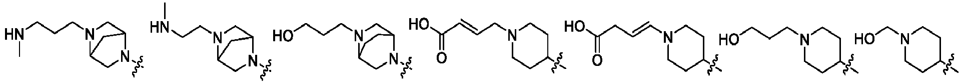 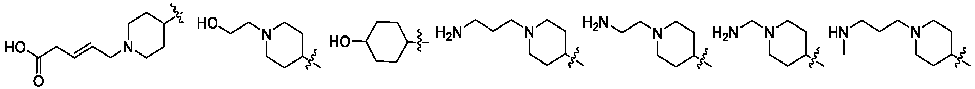 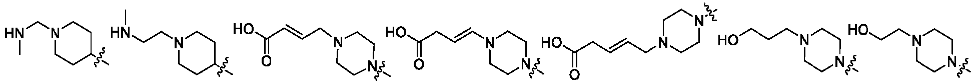 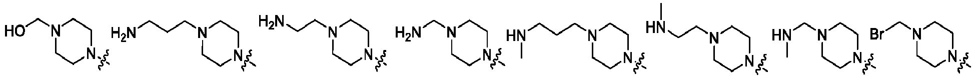 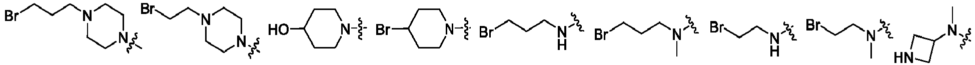 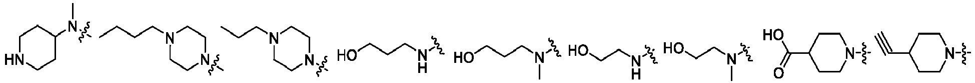 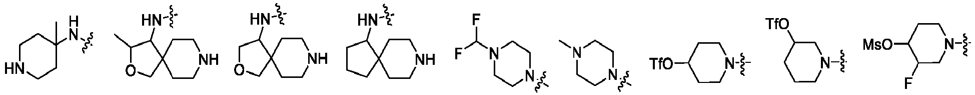 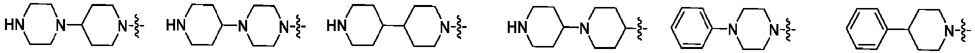 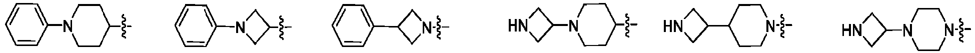 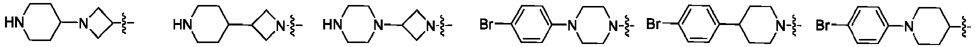 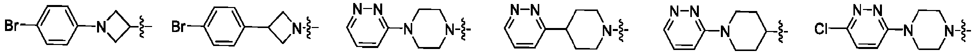 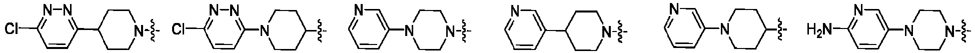 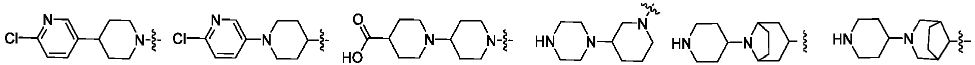 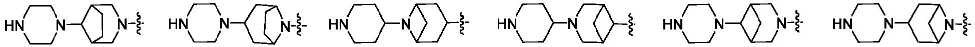 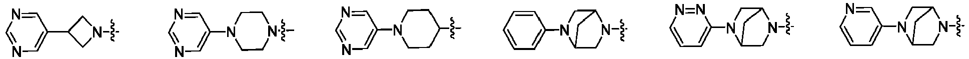 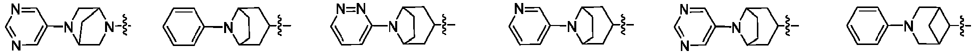 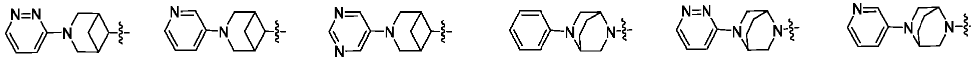 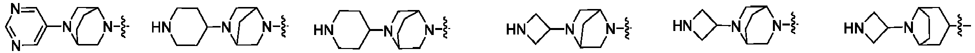 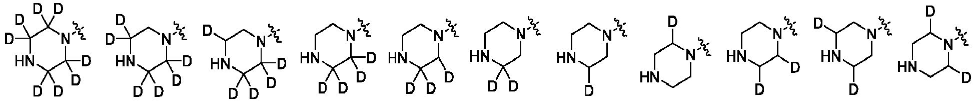 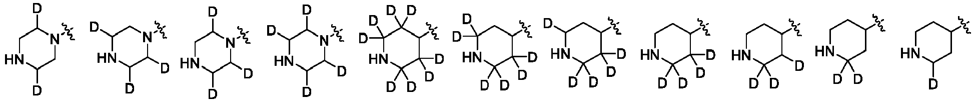 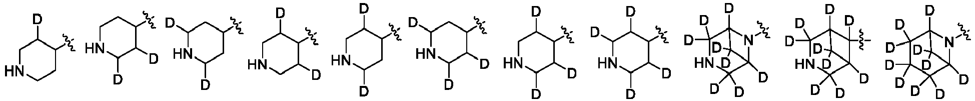 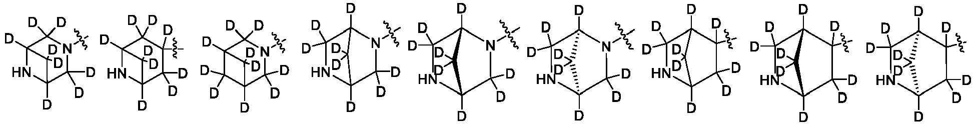 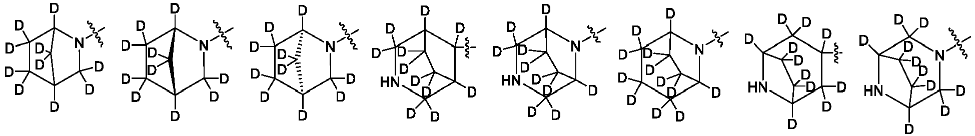 or

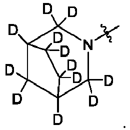

6. The compound of Formula (I) or salts, enantiomers, stereoisomers, solvates, isotopically enriched analogs, prodrugs, or polymorphs thereof as claimed in claim 1 or 2, wherein R₅ and R₆ each independently represent:
hydrogen, fluorine, chlorine, bromine, iodine, optionally substituted methyl, or optionally substituted linear or branched C₂₋₃₀ alkyl,
wherein one or more groups R_{c} and/or one or more groups R_{d} and/or any combination of one or more groups R_{c} and R_{d} are optionally inserted into the backbone carbon chain of the linear or branched C₂₋₃₀ alkyl group, wherein carbon-carbon bond between one or more pairs of adjacent carbon atoms in the backbone carbon chain is interrupted by the group R_{c}, R_{d}, or a combination of R_{c} and R_{d}; wherein each R_{c} is selected from the group consisting of O, N(Rₑ), C(O), C(O)O, S(O), S(O)₂, S(O)₂NH, NHS(O)₂, OC(O), C(O)N(Rₑ), N(Rₑ)C(O), or N(Rₑ)C(O)N(Rₑ), where each Rₑ independently represents H or C₁₋₆ alkyl, and in case that two or more groups R_{c} are inserted into the backbone carbon chain of the linear or branched C₂₋₃₀ alkyl group, the two or more groups R_{c} are not directly connected to each other; and wherein each R_{d} is selected from the group consisting of cycloalkylene (e.g., C₃₋₂₀ cycloalkylene), arylene (e.g., C₃₋₂₀ arylene), heterocyclylene (e.g., 4- to 20-membered heterocyclylene), heteroarylene (e.g., 4- to 20-membered heteroarylene), alkynylene (e.g., C₂₋₆ alkynylene), alkenylene (e.g., C₂₋₆ alkenylene), or any combination thereof, wherein the cycloalkylene, arylene, heterocyclylene, and heteroarylene are each independently optionally substituted with a substituent selected from the group consisting of deuterium, optionally deuterated C₁₋₆ alkyl, optionally deuterated C₃₋₆ cycloalkyl, hydroxy, amino, mercapto, halogen, optionally deuterated C₁₋₆ alkoxy, optionally deuterated C₁₋₆ alkyl-NH-, halogenated C₁₋₆ alkyl, NH₂-C₁₋₆ alkylene, optionally deuterated C₁₋₆ alkyl-NHC(O)-, optionally deuterated C₁₋₆ alkyl-C(O)NH-, cyano, or any combination thereof; and
a hydrogen atom of methyl and hydrogen atom of one or more CH₂ of C₂₋₃₀ alkyl are optionally substituted with a substituent selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, or any combination thereof.

7. The compound of Formula (I) or salts, enantiomers, stereoisomers, solvates, isotopically enriched analogs, prodrugs, or polymorphs thereof as claimed in claim 1, wherein R and R₅ together with the carbon atom to which they are connected form carbonyl group.

8. The compound of Formula (I) or salts, enantiomers, stereoisomers, solvates, isotopically enriched analogs, prodrugs, or polymorphs thereof as claimed in any one of claims 1-2 and 6-7, wherein R₆ represents the structure of the following formula:
-C₁₋₃₀ alkyl;
-(C(R^{a4})(R^{a5}))ₙ₁-(R_{c}-(C(R^{a6})(R^{a7}))ₙ₂)ₘ₄-H;
-(C(Ra⁴)(R^{a5}))ₙ₁-(R_{c}-(C(R^{a6})(R^{a7}))ₙ₂)ₘ₄-(R_{c}-(C(R^{a8})(R^{a9}))ₙ₃)ₘ₅-H;
-(C(R^{a4})(R^{a5}))ₙ₁-(R_{c}-(C(R^{a6})(R^{a7}))ₙ₂)ₘ₄-(R_{c}-(C(R^{a8})(R^{a9}))ₙ₃)ₘ₅-(R_{c}-(C(R^{a10})(R^{a11}))ₙ₄)ₘ₆-H;
-(C(R^{a4})(R^{a5}))ₙ₁-(R_{d}-(C(R^{a6})(R^{a7}))ₙ₂)ₘ₄-H;
-(C(R^{a4})(R^{a5}))ₙ₁-(R_{d}-(C(R^{a6})(R^{a7}))ₙ₂)ₘ₄-(R_{d}-(C(R^{a8})(R^{a9}))ₙ₃)ₘ₅-H;
-(C(R^{a4})(R^{a5}))ₙ₁-(R_{d}-(C(R^{a6})(R^{a7}))ₙ₂)ₘ₄-(R_{d}-(C(R^{a8})(R^{a9}))ₙ₃)ₘ₅-(R_{d}-(C(R^{a10})(R^{a11}))ₙ₄)ₘ₆-H;
-(C(R^{a4})(R^{a5}))ₙ₁-(R_{c}-R_{d}-(C(R^{a6})(R^{a7}))ₙ₂)ₘ₄-H;
-(C(R^{a4})(R^{a5}))ₙ₁-(R_{c}-(C(R^{a6})(R^{a7}))ₙ₂)ₘ₄-(R_{d}-(C(R^{a8})(R^{a9}))ₙ₃)ₘ₅-H;
-(C(R^{a4})(R^{a5}))ₙ₁-(R_{d}-R_{c}-(C(R^{a6})(R^{a7}))ₙ₂)ₘ₄-H; or
-(C(R^{a4})(R^{a5}))ₙ₁-(R_{d}-(C(R^{a6})(R^{a7}))ₙ₂)ₘ₄-(R_{c}-(C(R^{a8})(R^{a9}))ₙ₃)ₘ₅-H;
wherein each R_{c} is selected from the group consisting of O, N(Rₑ), C(O), C(O)O, OC(O), S(O), S(O)₂, S(O)₂NH, NHS(O)₂, C(O)N(Rₑ), N(Rₑ)C(O), or N(Rₑ)C(O)N(Rₑ), where each Rₑ independently represents H or C₁₋₆ alkyl; and wherein each R_{d} is selected from the group consisting of cycloalkylene (e.g., C₃₋₂₀ cycloalkylene), arylene (e.g., C₅₋₂₀ arylene), heterocyclylene (e.g., 4- to 20-membered heterocyclylene), heteroarylene (e.g., 5- to 20-membered heteroarylene), alkynylene (e.g., C₂₋₆ alkynylene), alkenylene (e.g., C₂₋₆ alkenylene), or any combination thereof, wherein the cycloalkylene, arylene, heterocyclylene, and heteroarylene are each independently optionally substituted with a substituent selected from the group consisting of deuterium, optionally deuterated C₁₋₆ alkyl, optionally deuterated C₃₋₆ cycloalkyl, hydroxy, amino, mercapto, halogen, optionally deuterated C₁₋₆ alkoxy, optionally deuterated C₁₋₆ alkyl-NH-, halogenated C₁₋₆ alkyl, NH₂-C₁₋₆ alkylene, optionally deuterated C₁₋₆ alkyl-NHC(O)-, optionally deuterated C₁₋₆ alkyl-C(O)NH-, cyano, or any combination thereof;
a hydrogen atom of one or more CH₂ of C₁₋₃₀ alkyl is optionally substituted with a substituent selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, or any combination thereof;
R^{a4}, R^{a5}, R^{a6}, R^{a7}, R^{a8}, R^{a9}, R^{a10} and R^{a11} each independently represent H, deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, or optionally deuterated C₁₋₆ alkyl-C(O)NH-; and
n1, n2, n3, n4, m4, m5, and m6 each independently represent an integer of 1, 2, 3, 4, 5, 6, 7, 8, 9, 10, 11, 12, 13, 14, or 15.

9. The compound of Formula (I) or salts, enantiomers, stereoisomers, solvates, isotopically enriched analogs, prodrugs, or polymorphs thereof as claimed in claim 8, wherein R₆ represents the structure of the following formula:
-(C(R^{a4})(R^{a5}))ₙ₁-(O-(C(R^{a6})(R^{a7}))ₙ₂)ₘ₄-H;
-(C(R^{a4})(R^{a5}))ₙ₁-(O-(C(R^{a6})(R^{a7}))ₙ₂)ₘ₄-(O-(C(R^{a8})(R^{a9}))ₙ₃)ₘ₅-H;
-(C(R^{a4})(R^{a5}))ₙ₁-(O-(C(R^{a6})(R^{a7}))ₙ₂)ₘ₄-(O-(C(R^{a8})(R^{a9}))ₙ₃)ₘ₅-(O-(C(R^{a10})(R^{a11}))ₙ₄)ₘ₆-H;
-(C(R^{a4})(R^{a5}))ₙ₁-(N(Rₑ)-(C(R^{a6})(R^{a7}))ₙ₂)ₘ₄-H;
-(C(R^{a4})(R^{a5}))ₙ₁-(N(Rₑ)-(C(R^{a6})(R^{a7}))ₙ₂)ₘ₄-(N(Rₑ)-(C(R^{a8})(R^{a9}))ₙ₃)ₘ₅-H;
-(C(R^{a4})(R^{a5}))ₙ₁-(N(Rₑ)-(C(R^{a6})(R^{a7}))ₙ₂)ₘ₄-(N(Rₑ)-(C(R^{a8})(R^{a9}))ₙ₃)ₘ₅-(N(Rₑ)-(C(R^{a10})(R^{a11}))ₙ₄)ₘ₆-H;
-(C(R^{a4})(R^{a5}))ₙ₁-(C(O)N(Rₑ)-(C(R^{a6})(R^{a7}))ₙ₂)ₘ₄-H;
-(C(R^{a4})(R^{a5}))ₙ₁-(C(O)N(Rₑ)-(C(R^{a6})(R^{a7}))ₙ₂)ₘ₄-(C(O)N(Rₑ)-(C(R^{a8})(R^{a9}))ₙ₃)ₘ₅-H;
-(C(R^{a4})(R^{a5}))ₙ₁-(C(O)N(Rₑ)-(C(R^{a6})(R^{a7}))ₙ₂)ₘ₄-(C(O)N(Rₑ)-(C(R^{a8})(R^{a9}))ₙ₃)ₘ₅-(C(O)N(Rₑ)-(C(R^{a10})(R^{a11}))ₙ₄)ₘ₆-H;
-(C(R^{a4})(R^{a5}))ₙ₁-(C(O)N(Rₑ)-(C(R^{a6})(R^{a7}))ₙ₂)ₘ₄-(O-(C(R^{a8})(R^{a9}))ₙ₃)ₘ₅-H;
-(C(R^{a4})(R^{a5}))ₙ₁-C(O)N(Rₑ)-(C(R^{a6})(R^{a7}))ₙ₂-(O-(C(R^{a8})(R^{a9}))ₙ₃)ₘ₄-H;
-(C(R^{a4})(R^{a5}))ₙ₁-(N(Rₑ)C(O)-(C(R^{a6})(R^{a7}))ₙ₂)ₘ₄-H;
-(C(R^{a4})(R^{a5}))ₙ₁-(N(Rₑ)C(O)-(C(R^{a6})(R^{a7}))ₙ₂)ₘ₄-(O-(C(R^{a8})(R^{a9}))ₙ₃)ₘ₅-H;
-(C(R^{a4})(R^{a5}))ₙ₁-(N(Rₑ)C(O)-(C(R^{a6})(R^{a7}))ₙ₂)ₘ₄-(O-(C(R^{a8})(R^{a9}))ₙ₃)ₘ₅-(O-(C(R^{a10})(R^{a11}))ₙ₄)ₘ₆-H;
-(C(R^{a4})(R^{a5}))ₙ₁-N(Rₑ)C(O)-(C(R^{a6})(R^{a7}))ₙ₂-(O-(C(R^{a8})(R^{a9}))ₙ₃)ₘ₄-H;
-(C(R^{a4})(R^{a5}))ₙ₁-N(Rₑ)C(O)N(Rₑ)-(C(R^{a6})(R^{a7}))ₙ₂-H;
-(C(R^{a4})(R^{a5}))ₙ₁-C(O)-(C(R^{a6})(R^{a7}))ₙ₂-H;
-(C(R^{a4})(R^{a5}))ₙ₁-arylene-(C(R^{a6})(R^{a7}))ₙ₂)ₘ₄-H;
-(C(R^{a4})(R^{a5}))ₙ₁-(arylene(C(R^{a6})(R^{a7}))ₙ₂)ₘ₄-arylene-(C(R^{a8})(R^{a9}))ₙ₃-H;
-(C(R^{a4})(R^{a5}))ₙ₁-(heterocyclylene-(C(R^{a6})(R^{a7}))ₙ₂)ₘ₄-H;
-(C(R^{a4})(R^{a5}))ₙ₁-(heterocyclylene-(C(R^{a6})(R^{a7}))ₙ₂)ₘ₄-(heterocyclylene-(C(R^{a8})(R^{a9}))ₙ₃)ₘ₅-H;
-(C(R^{a4})(R^{a5}))ₙ₁-(heteroarylene-(C(R^{a6})(R^{a7}))ₙ₂)ₘ₄-H;
-(C(R^{a4})(R^{a5}))ₙ₁-(heteroarylene-(C(R^{a6})(R^{a7}))ₙ₂)ₘ₄-(heteroarylene-(C(R^{a8})(R^{a9}))ₙ₃)ₘ₅-H;
-(C(R^{a4})(R^{a5}))ₙ₁-(cycloalkylene-(C(R^{a6})(R^{a7}))ₙ₂)ₘ₄-H; or
-(C(R^{a4})(R^{a5}))ₙ₁-(cycloalkylene-(C(R^{a6})(R^{a7}))ₙ₂)ₘ₄-(cycloalkylene-(C(R^{a8})(R^{a9}))ₙ₃)ₘ₅-H;
wherein each Rₑ independently represents H or C₁₋₆ alkyl;
the cycloalkylene, arylene, heterocyclylene and heteroarylene are each independently optionally substituted with a substituent selected from the group consisting of deuterium, optionally deuterated C₁₋₆ alkyl, optionally deuterated C₃₋₆ cycloalkyl, hydroxy, amino, mercapto, halogen, optionally deuterated C₁₋₆ alkoxy, optionally deuterated C₁₋₆ alkyl-NH-, halogenated C₁₋₆ alkyl, NH₂-C₁₋₆ alkylene, optionally deuterated C₁₋₆ alkyl-NHC(O)-, optionally deuterated C₁₋₆ alkyl-C(O)NH-, cyano, or any combination thereof;
R^{a4}, R^{a5}, R^{a6}, R^{a7}, R^{a8}, R^{a9}, R^{a10} and R^{a11} each independently represent H, deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, or optionally deuterated C₁₋₆ alkyl-C(O)NH-; and
n1, n2, n3, n4, m4, m5, and m6 each independently represent an integer of 1, 2, 3, 4, 5, 6, 7, 8, 9, 10, 11, 12, 13, 14, or 15.

10. The compound of Formula (I) or salts, enantiomers, stereoisomers, solvates, isotopically enriched analogs, prodrugs, or polymorphs thereof as claimed in claim 1, wherein R₆ represents the following group:
H, fluorine, chlorine, bromine, iodine, -CH₃, -CH₂-CH₃, -(CH₂)₂-CH₃, -(CH₂)₃-CH₃, -(CH₂)₄-CH₃, -(CH₂)₅-CH₃, -(CH₂)₆-CH₃, -(CH₂)₇-CH₃, -(CH₂)₈-CH₃, -(CH₂)₉-CH₃, -(CH₂)₁₀-CH₃, -(CH₂)₁₁-CH₃, - (CH₂)₁₂-CH₃, -(CH₂)₁₃-CH₃, -(CH₂)₁₄-CH₃, -(CH₂)₁₅-CH₃, -(CH₂)₁₆-CH₃, -(CH₂)₁₇-CH₃, -(CH₂)₁₈-CH₃, -(CH₂)₁₉-CH₃, -(CH₂)₂₀-CH₃, -(CH₂)₂₁-CH₃, -(CH₂)₂₂-CH₃, -(CH₂)₂₅-CH₃, -(CH₂)₂₉-CH₃;-CH₂-O-CH₃, -CH₂-O-CH₂-CH₃, -CH₂-O-(CH₂)₂-CH₃, -(CH₂)₁-O-(CH₂)₃-CH₃, -(CH₂)₁-O-(CH₂)₄-CH₃, -(CH₂)₁-O- (CH₂)₅-CH₃, -(CH₂)₁-O-(CH₂)₆-CH₃, -(CH₂)₁-O-(CH₂)₇-CH₃, -(CH₂)₁-O-(CH₂)₈-CH₃, -(CH₂)₁-O- (CH₂)₉-CH₃, -(CH₂)₁-O-(CH₂)₁₀-CH₃, -(CH₂)₂-O-CH₃, -(CH₂)₂-O-CH₂-CH₃, -(CH₂)₂-O-(CH₂)₂-CH₃, - (CH₂)₂-O-(CH₂)₃-CH₃, -(CH₂)₂-O-(CH₂)₄-CH₃, -(CH₂)₂-O-(CH₂)₅-CH₃, -(CH₂)₂-O-(CH₂)₆-CH₃, - (CH₂)₂-O-(CH₂)₇-CH₃, -(CH₂)₂-O-(CH₂)₈-CH₃, -(CH₂)₂-O-(CH₂)₉-CH₃, -(CH₂)₂-O-(CH₂)₁₀-CH₃, - (CH₂)₂-O-(CH₂)₁₁-CH₃, -(CH₂)₂-O-(CH₂)₁₂-CH₃, -(CH₂)₃-O-CH₃, -(CH₂)₃-O-CH₂-CH₃, -(CH₂)₃-O-(CH₂)₂-CH₃, -(CH₂)₃-O-(CH₂)₃-CH₃, -(CH₂)₃-O-(CH₂)₄-CH₃, -(CH₂)₃-0-(CH₂)₅-CH₃, -(CH₂)₃-O-(CH₂)₆-CH₃, -(CH₂)₃-O-(CH₂)₇-CH₃, -(CH₂)₄-O-CH₃, -(CH₂)₄-O-CH₂-CH₃, -(CH₂)₄-O-(CH₂)₂-CH₃, - (CH₂)₄-O-(CH₂)₃-CH₃, -(CH₂)₄-O-(CH₂)₄-CH₃, -(CH₂)₄-O-(CH₂)₅-CH₃, -(CH₂)₄-O-(CH₂)₆-CH₃, - (CH₂)₅-O-CH₃, -(CH₂)₅-O-CH₂-CH₃, -(CH₂)₅-O-(CH₂)₂-CH₃, -(CH₂)₅-O-(CH₂)₃-CH₃, -(CH₂)₅-O-(CH₂)₄-CH₃, -(CH₂)₅-O-(CH₂)₅-CH₃, -(CH₂)₆-O-CH₃, -(CH₂)₆-O-CH₂-CH₃, -(CH₂)₆-O-(CH₂)₂-CH₃, - (CH₂)₆-O-(CH₂)₃-CH₃, -(CH₂)₆-O-(CH₂)₄-CH₃, -(CH₂)₇-O-CH₃, -(CH₂)₇-O-CH₂-CH₃, -(CH₂)₇-O-(CH₂)₂-CH₃, -(CH₂)₇-O-(CH₂)₃-CH₃, -(CH₂)₈-O-CH₃, -(CH₂)₈-O-CH₂-CH₃, -(CH₂)₈-O-(CH₂)₂-CH₃, - CH(CH₃)-O-CH₃, -CH(CH₃)-O-CH₂-CH₃, -CH(CH₃)-O-(CH₂)₂-CH₃, -CH(CH₃)-O-(CH₂)₃-CH₃, - CH(CH₃)-O-(CH₂)₄-CH₃, -CH(CH₃)-O-(CH₂)₅-CH₃, -CH(CH₃)-O-(CH₂)₆-CH₃, -CH(CH₃)-O-(CH₂)₇-CH₃, -CH(CH₃)-O-(CH₂)₈-CH₃, -CH(CH₃)-O-(CH₂)₉-CH₃, -CH(CH₃)-O-(CH₂)₁₀-CH₃, -CH₂-(O(CH₂)₂)₁-OCH₂CH₃, -CH₂-(O(CH₂)₂)₂-OCH₂CH₃, -CH₂-(O(CH₂)₂)₃-OCH₂CH₃, -CH₂-(O(CH₂)₂)₄-OCH₂CH₃, -CH₂-(O(CH₂)₂)₅-OCH₂CH₃, -CH₂-(O(CH₂)₂)₆-OCH₂CH₃, -CH₂-(O(CH₂)₂)₇-OCH₂CH₃, - CH₂-(O(CH₂)₂)₈-OCH₂CH₃, -CH₂-(O(CH₂)₂)₉-OCH₂CH₃, -CH₂-(O(CH₂)₂)₁₀-OCH₂CH₃, -CH₂-(O(CH₂)₂)₁-OCH₃, -CH₂-(O(CH₂)₂)₂-OCH₃, -CH₂-(O(CH₂)₂)₃-OCH₃, -CH₂-(O(CH₂)₂)₄-OCH₃, -CH₂-(O(CH₂)₂)₅-OCH₃, -CH₂-(O(CH₂)₂)₆-OCH₃, -CH₂-(O(CH₂)₂)₇-OCH₃, -CH₂-(O(CH₂)₂)₈-OCH₃, -CH₂-(O(CH₂)₂)₉-OCH₃, -CH₂-(O(CH₂)₂)₁₀-OCH₃, -(CH₂)₂-(O(CH₂)₂)₁-OCH₂CH₃, -(CH₂)₂-(O(CH₂)₂)₂-OCH₂CH₃, -(CH₂)₂-(O(CH₂)₂)₃-OCH₂CH₃, -(CH₂)₂-(O(CH₂)₂)₄-OCH₂CH₃, -(CH₂)₂-(O(CH₂)₂)₅-OCH₂CH₃, -(CH₂)₂-(O(CH₂)₂)₆-OCH₂CH₃, -(CH₂)₂-(O(CH₂)₂)₇-OCH₂CH₃, -(CH₂)₂-(O(CH₂)₂)₈-OCH₂CH₃, -(CH₂)₂-(O(CH₂)₂)₉-OCH₂CH₃, -(CH₂)₂-(O(CH₂)₂)₁₀-OCH₂CH₃, -(CH₂)₃-(O(CH₂)₂)₁-OCH₂CH₃, -(CH₂)₃-(O(CH₂)₂)₂-OCH₂CH₃, -(CH₂)₃-(O(CH₂)₂)₃-OCH₂CH₃, -(CH₂)₃-(O(CH₂)₂)₄-OCH₂CH₃, -(CH₂)₃-(O(CH₂)₂)₅-OCH₂CH₃, -(CH₂)₃-(O(CH₂)₂)₆-OCH₂CH₃, -(CH₂)₃-(O(CH₂)₂)₇-OCH₂CH₃, -(CH₂)₃-(O(CH₂)₂)₈-OCH₂CH₃, -(CH₂)₃-(O(CH₂)₂)₉-OCH₂CH₃, -(CH₂)₃-(O(CH₂)₂)₁₀-OCH₂CH₃, -(CH₂)₄-(O(CH₂)₂)₁-OCH₂CH₃, -(CH₂)₄-(O(CH₂)₂)₂-OCH₂CH₃, -(CH₂)₄-(O(CH₂)₂)₃-OCH₂CH₃, -(CH₂)₄-(O(CH₂)₂)₄-OCH₂CH₃, -(CH₂)₄-(O(CH₂)₂)₅-OCH₂CH₃, -(CH₂)₄-(O(CH₂)₂)₆-OCH₂CH₃, -(CH₂)₄-(O(CH₂)₂)₇-OCH₂CH₃, -(CH₂)₄-(O(CH₂)₂)₈-OCH₂CH₃, -(CH₂)₄-(O(CH₂)₂)₉-OCH₂CH₃, -(CH₂)₄-(0(CH₂)₂)_{lO}-OCH₂CH₃, -CH₂-(O(CH₂)₃)₁-OCH₂CH₂CH₃, -CH₂-(O(CH₂)₃)₂-OCH₂CH₂CH₃, -CH₂-(O(CH₂)₃)₃-OCH₂CH₂CH₃, -CH₂-(O(CH₂)₃)₄-OCH₂CH₂CH₃, -CH₂-(O(CH₂)₃)₅-OCH₂CH₂CH₃, -CH₂-(O(CH₂)₃)₆-OCH₂CH₂CH₃, -CH₂-(O(CH₂)₃)₇-OCH₂CH₂CH₃, -CH₂-(O(CH₂)₃)₈-OCH₂CH₂CH₃, -CH₂-(O(CH₂)₃)₉-OCH₂CH₂CH₃, -CH₂-(O(CH₂)₃)₁₀-OCH₂CH₂CH₃, -(CH₂)₂-(O(CH₂)₃)₁-OCH₂CH₂CH₃, -(CH₂)₂-(O(CH₂)₃)₂-OCH₂CH₂CH₃, -(CH₂)₂-(O(CH₂)₃)₃-OCH₂CH₂CH₃, - (CH₂)₂-(O(CH₂)₃)₄-OCH₂CH₂CH₃, -(CH₂)₂-(O(CH₂)₃)₅-OCH₂CH₂CH₃, -(CH₂)₂-(O(CH₂)₃)₆-OCH₂CH₂CH₃, -(CH₂)₂-(O(CH₂)₃)₇-OCH₂CH₂CH₃, -(CH₂)₂-(O(CH₂)₃)₈-OCH₂CH₂CH₃, -(CH₂)₃-(O(CH₂)₃)₁-OCH₂CH₂CH₃, -(CH₂)₃-(O(CH₂)₃)₂-OCH₂CH₂CH₃, -(CH₂)₃-(O(CH₂)₃)₃-OCH₂CH₂CH₃, - (CH₂)₃-(O(CH₂)₃)₄-OCH₂CH₂CH₃, -(CH₂)₃-(O(CH₂)₃)₅-OCH₂CH₂CH₃, -(CH₂)₃-(O(CH₂)₃)₆-OCH₂CH₂CH₃, -(CH₂)₃-(O(CH₂)₃)₇-OCH₂CH₂CH₃, -(CH₂)₃-(O(CH₂)₃)₈-OCH₂CH₂CH₃, -CH₂-O-(CH₂)₂-O-(CH₂)₂-CH₃, -CH₂-(O(CH₂)₂)₂-(O(CH₂)₃)₁-OCH₂CH₂CH₃, -CH₂-(O(CH₂)₂)₃-(O(CH₂)₃)₂-OCH₂CH₂CH₃, -CH₂-(O(CH₂)₂)₄-(O(CH₂)₃)₃-OCH₂CH₂CH₃, -CH₂-(O(CH₂)₂)₅-(O(CH₂)₃)₄-OCH₂CH₂CH₃, -(CH₂)₂-O-(CH₂)₂-O-(CH₂)₂CH₃, -(CH₂)₂-(O(CH₂)₂)₂-(O(CH₂)₃)₁-OCH₂CH₂CH₃, - (CH₂)₂-(O(CH₂)₂)₃-(O(CH₂)₃)₂-OCH₂CH₂CH₃, -(CH₂)₂-(O(CH₂)₂)₄-(O(CH₂)₃)₃-OCH₂CH₂CH₃, - (CH₂)₂-(O(CH₂)₂)₅-(O(CH₂)₃)₄-OCH₂CH₂CH₃, -(CH₂)₃-O-(CH₂)₂-O-(CH₂)₂CH₃, -(CH₂)₃-(O(CH₂)₂)₂-(O(CH₂)₃)₁-OCH₂CH₂CH₃, -(CH₂)₃-(O(CH₂)₂)₃-(O(CH₂)₃)₂-OCH₂CH₂CH₃, -(CH₂)₃-(O(CH₂)₂)₄-(O(CH₂)₃)₃-OCH₂CH₂CH₃, -(CH₂)₃-(O(CH₂)₂)₅-(O(CH₂)₃)₄-OCH₂CH₂CH₃, -CH₂-O-(CH₂)₃-O-CH₂CH₃, -CH₂-(O(CH₂)₃)₂-(O(CH₂)₂)₁-O-CH₂CH₃, -CH₂-(O(CH₂)₃)₃-(O(CH₂)₂)₂-O-CH₂CH₃, -CH₂-(O(CH₂)₃)₄-(O(CH₂)₂)₃-O-CH₂CH₃, -CH₂-(O(CH₂)₃)₅-(O(CH₂)₂)₄-O-CH₂CH₃, -(CH₂)₂-O-(CH₂)₃-O-CH₂CH₃, -(CH₂)₂-(O(CH₂)₃)₂-(O(CH₂)₂)₁-O-CH₂CH₃, -(CH₂)₂-(O(CH₂)₃)₃-(O(CH₂)₂)₂-O-CH₂CH₃, - (CH₂)₂-(O(CH₂)₃)₄-(O(CH₂)₂)₃-O-CH₂CH₃, -(CH₂)₂-(O(CH₂)₃)₅-(O(CH₂)₂)₄-O-CH₂CH₃, -(CH₂)₃-O-(CH₂)₃-O-CH₂CH₃, -(CH₂)₃-(O(CH₂)₃)₂-(O(CH₂)₂)₁-O-CH₂CH₃, -(CH₂)₃-(O(CH₂)₃)₃-(O(CH₂)₂)₂-O-CH₂CH₃, -(CH₂)₃-(O(CH₂)₃)₄-(O(CH₂)₂)₃-O-CH₂CH₃, -(CH₂)₃-(O(CH₂)₃)₅-(O(CH₂)₂)₄-O-CH₂CH₃, - CH₂-O-(CH₂)₂-O-CH₃, -(CH₂)₂-O-(CH₂)₂-O-CH₃, -(CH₂)₂-(O(CH₂)₂)₂-O-(CH₂)₂CH₃, -(CH₂)₂-(O(CH₂)₂)₃-O-(CH₂)₂CH₃, -(CH₂)₂-(O(CH₂)₂)₄-O-(CH₂)₂CH₃, -(CH₂)₅-(O(CH₂)₂)₂-O-(CH₂)₄CH₃, - (CH₂)₅-(O(CH₂)₂)₂-O-(CH₂)₅CH₃, -(CH₂)₁-N(Rₑ)-CH₃, -(CH₂)₁-N(Rₑ)-(CH₂)₁-CH₃, -(CH₂)₁-N(Rₑ)-(CH₂)₂-CH₃, -(CH₂)₁-N(Rₑ)-(CH₂)₃-CH₃, -(CH₂)₁-N(Rₑ)-(CH₂)₄-CH₃, -(CH₂)₁-N(Rₑ)-(CH₂)₅-CH₃, - (CH₂)₁-N(Rₑ)-(CH₂)₆-CH₃, -(CH₂)₁-N(Rₑ)-(CH₂)₇-CH₃, -(CH₂)₁-N(Rₑ)-(CH₂)₈-CH₃, -(CH₂)₁-N(Rₑ)-(CH₂)₉-CH₃, -(CH₂)₂-N(Rₑ)-CH₃, -(CH₂)₂-N(Rₑ)-(CH₂)₁-CH₃, -(CH₂)₂-N(Rₑ)-(CH₂)₂-CH₃, -(CH₂)₂-N(Rₑ)-(CH₂)₃-CH₃, -(CH₂)₂-N(Rₑ)-(CH₂)₄-CH₃, -(CH₂)₂-N(Rₑ)-(CH₂)₅-CH₃, -(CH₂)₂-N(Rₑ)-(CH₂)₆-CH₃, -(CH₂)₂-N(Rₑ)-(CH₂)₇-CH₃, -(CH₂)₂-N(Rₑ)-(CH₂)₈-CH₃, -(CH₂)₂-N(Rₑ)-(CH₂)₉-CH₃, -(CH₂)₂-N(Rₑ)-(CH₂)₁₀-CH₃, -(CH₂)₂-N(Rₑ)-(CH₂)₁₁-CH₃, -(CH₂)₃-N(Rₑ)-CH₃, -(CH₂)₃-N(Rₑ)-(CH₂)₁-CH₃, - (CH₂)₃-N(Rₑ)-(CH₂)₂-CH₃, -(CH₂)₄-N(Rₑ)-CH₃, -(CH₂)₄-N(Rₑ)-(CH₂)₁-CH₃, -(CH₂)₄-N(Rₑ)-(CH₂)₂-CH₃, -(CH₂)₄-N(Rₑ)-(CH₂)₃-CH₃, -(CH₂)₅-N(Rₑ)-CH₃, -(CH₂)₅-N(Rₑ)-(CH₂)₁-CH₃, -(CH₂)₅-N(Rₑ)-(CH₂)₂-CH₃, -(CH₂)₅-N(Rₑ)-(CH₂)₃-CH₃, -(CH₂)₅-N(Rₑ)-(CH₂)₄-CH₃, -(CH₂)₆-N(Rₑ)-CH₃, -(CH₂)₆-N(Rₑ)-(CH₂)₁-CH₃, -(CH₂)₆-N(Rₑ)-(CH₂)₂-CH₃, -(CH₂)₇-N(Rₑ)-CH₃, -(CH₂)₇-N(Rₑ)-(CH₂)₁-CH₃, - (CH₂)₇-N(Rₑ)-(CH₂)₂-CH₃, -(CH₂)₈-N(Rₑ)-CH₃, -(CH₂)₈-N(Rₑ)-(CH₂)₁-CH₃, -(CH₂)₈-N(Rₑ)-(CH₂)₂-CH₃, -CH(CH₃)-N(Rₑ)-CH₃, -CH(CH₃)-N(Rₑ)-(CH₂)₁-CH₃, -CH(CH₃)-N(Rₑ)-(CH₂)₂-CH₃, -CH(CH₃)-N(Rₑ)-(CH₂)₃-CH₃, -CH(CH₃)-N(Rₑ)-(CH₂)₄-CH₃, -CH(CH₃)-N(Rₑ)-(CH₂)₅-CH₃, -CH(CH₃)-N(Rₑ)-(CH₂)₆-CH₃, -CH(CH₃)-N(Rₑ)-(CH₂)₇-CH₃, -CH(CH₃)-N(Rₑ)-(CH₂)₈-CH₃, -CH(CH₃)-N(Rₑ)-(CH₂)₉-CH₃, -CH₂C(O)NHCH₃, -(CH₂)₂C(O)NHCH₂CH₃, -(CH₂)₂C(O)NH(CH₂)₂-CH₃, - (CH₂)₂C(O)NH(CH₂)₃-CH₃, -(CH₂)₂C(O)NH(CH₂)₄-CH₃, -(CH₂)₃C(O)NH(CH₂)₂-CH₃, - (CH₂)₃C(O)NH(CH₂)₃-CH₃, -(CH₂)₄C(O)NH(CH₂)₃-CH₃, -(CH₂)₅C(O)NH(CH₂)₄-CH₃, - (CH₂)₆C(O)NH(CH₂)₆-CH₃, -(CH₂)₆C(O)NH(CH₂)₅-CH₃, -(CH₂)₇C(O)NH(CH₂)₆-CH₃, - (CH₂)₈C(O)NH(CH₂)₇-CH₃, U-(CH₂)₉C(O)NH(CH₂)₈-CH₃, -(CH₂)₁₀C(O)NH(CH₂)₉-CH₃, - (CH₂)₂C(O)NH(CH₂)₂-O-CH₂-CH₃, -CH₂NHC(O)CH₃, -(CH₂)₂NHC(O)CH₂CH₃, - (CH₂)₂NHC(O)(CH₂)₂-CH₃, -(CH₂)₂NHC(O)(CH₂)₃-CH₃, -(CH₂)₂NHC(O)(CH₂)₄-CH₃, - (CH₂)₃NHC(O)(CH₂)₂-CH₃, -(CH₂)₃NHC(O)(CH₂)₃-CH₃, -(CH₂)₄NHC(O)(CH₂)₃-CH₃, - (CH₂)₅NHC(O)(CH₂)₄-CH₃, -(CH₂)₆NHC(O)(CH₂)₆-CH₃, -(CH₂)₆NHC(O)(CH₂)₅-CH₃, - (CH₂)₇NHC(O)(CH₂)₆-CH₃, -(CH₂)₈NHC(O)(CH₂)₇-CH₃, -(CH₂)₉NHC(O)(CH₂)₈-CH₃, - (CH₂)₁₀NHC(O)(CH₂)₉-CH₃, -(CH₂)₄NHC(O)(CH₂)₇-CH₃, -(CH₂)₂NHC(O)(CH₂)₂-O-CH₂-CH₃, - (CH₂)₄NHC(O)CH₃, -CH₂-piperidinylene-CH₃, -CH₂-piperidinylene-CH₂-CH₃, -CH₂-piperidinylene-(CH₂)₂-CH₃, -CH₂-piperidinylene-(CH₂)₃-CH₃, -CH₂-piperidinylene-(CH₂)₄-CH₃, -CH₂-piperidinylene-(CH₂)₅-CH₃, -CH₂-piperidinylene-(CH₂)₆-CH₃, -CH₂-piperidinylene-(CH₂)₇-CH₃, - (CH₂)₂-piperidinylene-CH₃, -(CH₂)₂-piperidinylene-CH₂-CH₃, -(CH₂)₂-piperidinylene-(CH₂)₂-CH₃, - (CH₂)₂-piperidinylene-(CH₂)₃-CH₃, -(CH₂)₂-piperidinylene-(CH₂)₄-CH₃, -(CH₂)₂-piperidinylene-(CH₂)₅-CH₃, -(CH₂)₂-piperidinylene-(CH₂)₆-CH₃, -(CH₂)₂-piperidinylene-(CH₂)₇-CH₃, -(CH₂)₃-piperidinylene-CH₃, -(CH₂)₃-piperidinylene-CH₂-CH₃, -(CH₂)₃-piperidinylene-(CH₂)₂-CH₃, -(CH₂)₃-piperidinylene-(CH₂)₃-CH₃, -(CH₂)₃-piperidinylene-(CH₂)₄-CH₃, -(CH₂)₃-piperidinylene-(CH₂)₅-CH₃, -(CH₂)₃-piperidinylene-(CH₂)₆-CH₃, -(CH₂)₃-piperidinylene-(CH₂)₇-CH₃, -(CH₂)₄-piperidinylene-CH₃, -(CH₂)₄-piperidinylene-CH₂-CH₃, -(CH₂)₄-piperidinylene-(CH₂)₂-CH₃, -(CH₂)₄-piperidinylene-(CH₂)₃-CH₃, -(CH₂)₄-piperidinylene-(CH₂)₄-CH₃, -(CH₂)₄-piperidinylene-(CH₂)₅-CH₃, -(CH₂)₄-piperidinylene-(CH₂)₆-CH₃, -(CH₂)₄-piperidinylene-(CH₂)₇-CH₃, -(CH₂)₅-piperidinylene-CH₃, - (CH₂)₅-piperidinylene-CH₂-CH₃, -(CH₂)₅-piperidinylene-(CH₂)₂-CH₃, -(CH₂)₅-piperidinylene-(CH₂)₃-CH₃, -(CH₂)₅-piperidinylene-(CH₂)₄-CH₃, -(CH₂)₅-piperidinylene-(CH₂)₅-CH₃, -(CH₂)₅-piperidinylene-(CH₂)₆-CH₃, -(CH₂)₅-piperidinylene-(CH₂)₇-CH₃, -(CH₂)₆-piperidinylene-CH₃, - (CH₂)₆-piperidinylene-CH₂-CH₃, -(CH₂)₆-piperidinylene-(CH₂)₂-CH₃, -(CH₂)₆-piperidinylene-(CH₂)₃-CH₃, -(CH₂)₆-piperidinylene-(CH₂)₄-CH₃, -(CH₂)₆-piperidinylene-(CH₂)₅-CH₃, -(CH₂)₆-piperidinylene-(CH₂)₆-CH₃, -(CH₂)₆-piperidinylene-(CH₂)₇-CH₃, -(CH₂)₇-piperidinylene-CH₃, - (CH₂)₇-piperidinylene-CH₂-CH₃, -(CH₂)₇-piperidinylene-(CH₂)₂-CH₃, -(CH₂)₇-piperidinylene-(CH₂)₃-CH₃, -(CH₂)₇-piperidinylene-(CH₂)₇-CH₃, -(CH₂)₈-piperidinylene-CH₃, -(CH₂)₈-piperidinylene-CH₂-CH₃, -(CH₂)₈-piperidinylene-(CH₂)₂-CH₃, -(CH₂)₈-piperidinylene-(CH₂)₃-CH₃, -(CH₂)₈-piperidinylene-(CH₂)₄-CH₃, -(CH₂)₈-piperidinylene-(CH₂)₅-CH₃, -(CH₂)₈-piperidinylene-(CH₂)₆-CH₃, -(CH₂)₈-piperidinylene-(CH₂)₇-CH₃, -CH₂-N(Rₑ)-CH₂-piperidinylene-CH₃, -CH₂-N(Rₑ)-CH₂-piperidinylene-CH₃, -CH₂-N(Rₑ)-CH₂-piperidinylene-(CH₂)₂-CH₃, -CH₂-N(Rₑ)-CH₂-piperidinylene-(CH₂)₃-CH₃, -CH₂-N(Rₑ)-CH₂-piperidinylene-(CH₂)₄-CH₃, -CH₂-N(Rₑ)-CH₂-piperidinylene-(CH₂)₅-CH₃, -CH₂-N(Rₑ)-CH₂-piperidinylene-(CH₂)₆-CH₃, -CH₂-N(Rₑ)-CH₂-piperidinylene-(CH₂)₇-CH₃, - CH₂-N(Rₑ)-(CH₂)₂-piperidinylene-CH₃, -CH₂-N(Rₑ)-(CH₂)₂-piperidinylene-CH₂-CH₃, -CH₂-N(Rₑ)-(CH₂)₂-piperidinylene-(CH₂)₂-CH₃, -CH₂-N(Rₑ)-(CH₂)₂-piperidinylene-(CH₂)₃-CH₃, -CH₂-N(Rₑ)-(CH₂)₂-piperidinylene-(CH₂)₄-CH₃, -CH₂-N(Rₑ)-(CH₂)₂-pipendinylene-(CH₂)₅-CH₃, -CH₂-N(Rₑ)-(CH₂)₂-piperidinylene-(CH₂)₆-CH₃, -CH₂-N(Rₑ)-(CH₂)₂-piperidinylene-(CH₂)₇-CH₃, -(CH₂)₂-N(Rₑ)-CH₂-piperidinylene-CH₃, -(CH₂)₂-N(Rₑ)-CH₂-piperidinylene-CH₂-CH₃, -(CH₂)₂-N(Rₑ)-CH₂-piperidinylene-(CH₂)₂-CH₃, -(CH₂)₂-N(Rₑ)-CH₂-piperidinylene-(CH₂)₃-CH₃, -(CH₂)₂-N(Rₑ)-CH₂-piperidinylene-(CH₂)₄-CH₃, -(CH₂)₂-N(Rₑ)-CH₂-piperidinylene-(CH₂)₅-CH₃, -(CH₂)₂-N(Rₑ)-CH₂-piperidinylene-(CH₂)₆-CH₃, -(CH₂)₂-N(Rₑ)-CH₂-piperidinylene-(CH₂)₇-CH₃, -(CH₂)₃-N(Rₑ)-CH₂-piperidinylene-CH₃, -(CH₂)₃-N(Rₑ)-CH₂-piperidinylene-CH₂-CH₃, -(CH₂)₃-N(Rₑ)-CH₂-piperidinylene-(CH₂)₂-CH₃, -(CH₂)₃-N(Rₑ)-CH₂-piperidinylene-(CH₂)₇-CH₃, -(CH₂)₄-N(Rₑ)-CH₂-piperidinylene-CH₃, -(CH₂)₄-N(Rₑ)-CH₂-piperidinylene-CH₂-CH₃, -(CH₂)₄-N(Rₑ)-CH₂-piperidinylene-(CH₂)₂-CH₃, - (CH₂)₄-N(Rₑ)-CH₂-piperidinylene-(CH₂)₇-CH₃, -(CH₂)₅-N(Rₑ)-CH₂-piperidinylene-(CH₂)₂-CH₃, - (CH₂)₅-N(Rₑ)-CH₂-piperidinylene-(CH₂)₇-CH₃, -(CH₂)₆-N(Rₑ)-CH₂-piperidinylene-(CH₂)₂-CH₃, - (CH₂)₆-N(Rₑ)-CH₂-piperidinylene-(CH₂)₇-CH₃, -(CH₂)₇-N(Rₑ)-CH₂-piperidinylene-(CH₂)₂-CH₃, - (CH₂)₇-N(Rₑ)-CH₂-piperidinylene-(CH₂)₇-CH₃, -(CH₂)₈-N(Rₑ)-CH₂-piperidinylene-CH₃, -(CH₂)₈-N(Rₑ)-CH₂-piperidinylene-CH₂-CH₃, -(CH₂)₈-N(Rₑ)-CH₂-piperidinylene-(CH₂)₂-CH₃, -(CH₂)₈-N(Rₑ)-CH₂-piperidinylene-(CH₂)₃-CH₃, -(CH₂)₈-N(Rₑ)-CH₂-piperidinylene-(CH₂)₄-CH₃, -(CH₂)₈-N(Rₑ)-CH₂-piperidinylene-(CH₂)₅-CH₃, -(CH₂)₈-N(Rₑ)-CH₂-piperidinylene-(CH₂)₆-CH₃, -(CH₂)₈-N(Rₑ)-CH₂-piperidinylene-(CH₂)₇-CH₃, -CH₂-piperazinylene-CH₃, -CH₂-piperazinylene-CH₂-CH₃, -CH₂-piperazinylene-(CH₂)₂-CH₃, -CH₂-piperazinylene-(CH₂)₃-CH₃, -CH₂-piperazinylene-(CH₂)₄-CH₃, - CH₂-piperazinylene-(CH₂)₅-CH₃, -CH₂-piperazinylene-(CH₂)₆-CH₃, -CH₂-piperazinylene-(CH₂)₇-CH₃, -(CH₂)₂-piperazinylene-CH₃, -(CH₂)₂-piperazinylene-CH₂-CH₃, -(CH₂)₂-piperazinylene-(CH₂)₂-CH₃, - (CH₂)₂-piperazinylene-(CH₂)₃-CH₃, -(CH₂)₂-piperazinylene-(CH₂)₄-CH₃, -(CH₂)₂-piperazinylene-(CH₂)₅-CH₃, -(CH₂)₂-piperazinylene-(CH₂)₆-CH₃, -(CH₂)₂-piperazinylene-(CH₂)₇-CH₃, -(CH₂)₃-piperazinylene-CH₃, -(CH₂)₃-piperazinylene-CH₂-CH₃, -(CH₂)₃-piperazinylene-(CH₂)₂-CH₃, -(CH₂)₃-piperazinylene-(CH₂)₃-CH₃, -(CH₂)₃-piperazinylene-(CH₂)₄-CH₃, -(CH₂)₃-piperazinylene-(CH₂)₅-CH₃, -(CH₂)₃-piperazinylene-(CH₂)₆-CH₃, -(CH₂)₃-piperazinylene-(CH₂)₇-CH₃, -(CH₂)₄-piperazinylene-CH₃, -(CH₂)₄-piperazinylene-CH₂-CH₃, -(CH₂)₄-piperazinylene-(CH₂)₂-CH₃, -(CH₂)₄-piperazinylene-(CH₂)₃-CH₃, -(CH₂)₄-piperazinylene-(CH₂)₄-CH₃, -(CH₂)₄-piperazinylene-(CH₂)₅-CH₃, -(CH₂)₄-piperazinylene-(CH₂)₆-CH₃, -(CH₂)₄-piperazinylene-(CH₂)₇-CH₃, -(CH₂)₅-piperazinylene-CH₃, - (CH₂)₅-piperazinylene-CH₂-CH₃, -(CH₂)₅-piperazinylene-(CH₂)₂-CH₃, -(CH₂)₅-piperazinylene-(CH₂)₃-CH₃, -(CH₂)₅-piperazinylene-(CH₂)₄-CH₃, -(CH₂)₅-piperazinylene-(CH₂)₅-CH₃, -(CH₂)₅-piperazinylene-(CH₂)₆-CH₃, -(CH₂)₅-piperazinylene-(CH₂)₇-CH₃, -(CH₂)₆-piperazinylene-CH₃, - (CH₂)₆-piperazinylene-CH₂-CH₃, -(CH₂)₆-piperazinylene-(CH₂)₂-CH₃, -(CH₂)₆-piperazinylene-(CH₂)₃-CH₃, -(CH₂)₆-piperazinylene-(CH₂)₄-CH₃, -(CH₂)₆-piperazinylene-(CH₂)₅-CH₃, -(CH₂)₆-piperazinylene-(CH₂)₆-CH₃, -(CH₂)₆-piperazinylene-(CH₂)₇-CH₃, -(CH₂)₇-piperazinylene-CH₃, - (CH₂)₇-piperazinylene-CH₂-CH₃, -(CH₂)₇-piperazinylene-(CH₂)₂-CH₃, -(CH₂)₇-piperazinylene-(CH₂)₃-CH₃, -(CH₂)₇-piperazinylene-(CH₂)₇-CH₃, -(CH₂)₈-piperazinylene-CH₃, -(CH₂)₈-piperazinylene-CH₂-CH₃, -(CH₂)₈-piperazinylene-(CH₂)₂-CH₃, -(CH₂)₈-piperazinylene-(CH₂)₃-CH₃, -(CH₂)₈-piperazinylene-(CH₂)₄-CH₃, -(CH₂)₈-piperazinylene-(CH₂)₅-CH₃, -(CH₂)₈-piperazinylene-(CH₂)₆-CH₃, or -(CH₂)₈-piperazinylene-(CH₂)₇-CH₃;
wherein the piperidinylene and piperazinylene are each independently optionally substituted with a substituent selected from the group consisting of deuterium, optionally deuterated C₁₋₆ alkyl, optionally deuterated C₃₋₆ cycloalkyl, hydroxy, amino, mercapto, nitro, halogen, optionally deuterated C₁₋₆ alkoxy, optionally deuterated C₁₋₆ alkyl-NH-, halogenated C₁₋₆ alkyl, NH₂-C₁₋₆ alkylene, optionally deuterated C₁₋₆ alkyl-NHC(O)-, optionally deuterated C₁₋₆ alkyl-C(O)NH-, cyano, or any combination thereof;
a hydrogen atom of CH₃ of the groups and a hydrogen atom of one or more CH₂ of the groups are optionally substituted with a substituent selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, or any combination thereof;
each Rₑ independently represents H or C₁₋₆ alkyl; and
n1, n2, n3, n4, m4, m5, and m6 each independently represent an integer of 1, 2, 3, 4, 5, 6, 7, 8, 9, 10, 11, 12, 13, 14, or 15.

11. The compound of Formula (I) or salts, enantiomers, stereoisomers, solvates, isotopically enriched analogs, prodrugs, or polymorphs thereof as claimed in claim 9, which is selected from:
3-(5-(bromomethyl)-1-oxoisoindolin-2-yl)piperidine-2,6-dione;
3-(5-(chloromethyl)-1-oxoisoindolin-2-yl)piperidine-2,6-dione;
3-(5-(iodomethyl)-1-oxoisoindolin-2-yl)piperidine-2,6-dione;
3-(5-(hydroxymethyl)-1-oxoisoindolin-2-yl)piperidine-2,6-dione;
3-(6-fluoro-5-(hydroxymethyl)-1-oxoisoindolin-2-yl)piperidine-2,6-dione;
3-(4-fluoro-5-(hydroxymethyl)-1-oxoisoindolin-2-yl)piperidine-2,6-dione;
3-(7-fluoro-5-(hydroxymethyl)-1-oxoisoindolin-2-yl)piperidine-2,6-dione;
3-(5-(aminomethyl)-7-fluoro-1-oxoisoindolin-2-yl)piperidine-2,6-dione;
3-(5-(aminomethyl)-6-fluoro-1-oxoisoindolin-2-yl)piperidine-2,6-dione;
3-(5-(aminomethyl)-4-fluoro-1-oxoisoindolin-2-yl)piperidine-2,6-dione;
(2-(2,6-dioxopiperidin-3-yl)-1-oxoisoindolin-5-yl)methyl 4-methylbenzenesulfonate;
(2-(2,6-dioxopiperidin-3-yl)-1-oxoisoindolin-5-yl)methyl methanesulfonate;
(2-(2,6-dioxopiperidin-3-yl)-1-oxoisoindolin-5-yl)methyl trifluoromethanesulfonate;
3-(5-(bromomethyl)-7-fluoro-1-oxoisoindolin-2-yl)piperidine-2,6-dione;
3-(5-(bromomethyl)-4-fluoro-1-oxoisoindolin-2-yl)piperidine-2,6-dione;
3-(5-(bromomethyl)-6-fluoro-1-oxoisoindolin-2-yl)piperidine-2,6-dione;
3-(7-bromo-5-(bromomethyl)-1-oxoisoindolin-2-yl)piperidine-2,6-dione;
3-(6-bromo-5-(bromomethyl)-1-oxoisoindolin-2-yl)piperidine-2,6-dione;
3-(4-bromo-5-(bromomethyl)-1-oxoisoindolin-2-yl)piperidine-2,6-dione;
3-(5-((3,6-diazabicyclo[3.1.1]heptan-3-yl)methyl)-1-oxoisoindolin-2-yl)piperidine-2,6-dione;
3-(5-((3,6-diazabicyclo[3.1.1]heptan-6-yl)methyl)-1-oxoisoindolin-2-yl)piperidine-2,6-dione;
3-(5-((3,8-diazabicyclo[3.2.1]octan-8-yl)methyl)-1-oxoisoindolin-2-yl)piperidine-2,6-dione;
3-(5-((3,8-diazabicyclo[3.2.1]octan-3-yl)methyl)-1-oxoisoindolin-2-yl)piperidine-2,6-dione;
3-(5-((2,S-diazabicyclo[2.2.1]heptan-2-yl)methyl)-1-oxoisoindolin-2-yl)piperidine-2,6-dione;
3-(5-((2,5-diazabicyclo[2.2.2]octan-2-yl)methyl)-1-oxoisoindolin-2-yl)piperidine-2,6-dione;
3-(1-oxo-5-((piperazin-1-yl-2,2,3,3,5,5,6,6-d8)methyl)isoindolin-2-yl)piperidine-2,6-dione;
3-(5-((2,6-dimethylpiperazin-1-yl)methyl)-1-oxoisoindolin-2-yl)piperidine-2,6-dione;
3-(5-((3,5-dimethylpiperazin-1-yl)methyl)-1-oxoisoindolin-2-yl)piperidine-2,6-dione;
3-(7-fluoro-1-oxo-5-(piperazin-1-ylmethyl)isoindolin-2-yl)piperidine-2,6-dione;
3-(7-chloro-1-oxo-5-(piperazin-1-ylmethyl)isoindolin-2-yl)piperidine-2,6-dione;
3-(7-bromo-1-oxo-5-(piperazin-1-ylmethyl)isoindolin-2-yl)piperidine-2,6-dione;
3-(4-fluoro-1-oxo-5-(piperazin-1-ylmethyl)isoindolin-2-yl)piperidine-2,6-dione;
3-(4-chloro-1-oxo-5-(piperazin-1-ylmethyl)isoindolin-2-yl)piperidine-2,6-dione;
3-(4-bromo-1-oxo-5-(piperazin-1-ylmethyl)isoindolin-2-yl)piperidine-2,6-dione;
3-(6-fluoro-1-oxo-5-(piperazin-1-ylmethyl)isoindolin-2-yl)piperidine-2,6-dione;
3-(6-chloro-1-oxo-5-(piperazin-1-ylmethyl)isoindolin-2-yl)piperidine-2,6-dione;
3-(6-bromo-1-oxo-5-(piperazin-1-ylmethyl)isoindolin-2-yl)piperidine-2,6-dione;
3-(4-(bromomethyl)-1-oxoisoindolin-2-yl)piperidine-2,6-dione;
3-(4-(chloromethyl)-1-oxoisoindolin-2-yl)piperidine-2,6-dione;
3-(4-(iodomethyl)-1-oxoisoindolin-2-yl)piperidine-2,6-dione;
3-(4-(hydroxymethyl)-1-oxoisoindolin-2-yl)piperidine-2,6-dione;
3-(5-fluoro-4-(hydroxymethyl)-1-oxoisoindolin-2-yl)piperidine-2,6-dione;
3-(6-fluoro-4-(hydroxymethyl)-1-oxoisoindolin-2-yl)piperidine-2,6-dione;
3-(7-fluoro-4-(hydroxymethyl)-1-oxoisoindolin-2-yl)piperidine-2,6-dione;
3-(4-(aminomethyl)-5-fluoro-1-oxoisoindolin-2-yl)piperidine-2,6-dione;
3-(4-(aminomethyl)-6-fluoro-1-oxoisoindolin-2-yl)piperidine-2,6-dione;
3-(4-(aminomethyl)-7-fluoro-1-oxoisoindolin-2-yl)piperidine-2,6-dione;
(2-(2,6-dioxopiperidin-3-yl)-1-oxoisoindolin-4-yl)methyl 4-methylbenzenesulfonate;
(2-(2,6-dioxopiperidin-3-yl)-1-oxoisoindolin-4-yl)methyl methanesulfonate;
(2-(2,6-dioxopiperidin-3-yl)-1-oxoisoindolin-4-yl)methyl trifluoromethanesulfonate;
3-(4-(bromomethyl)-7-fluoro-1-oxoisoindolin-2-yl)piperidine-2,6-dione;
3-(4-(bromomethyl)-5-fluoro-1-oxoisoindolin-2-yl)piperidine-2,6-dione;
3-(4-(bromomethyl)-6-fluoro-1-oxoisoindolin-2-yl)piperidine-2,6-dione;
3-(7-bromo-4-(bromomethyl)-1-oxoisoindolin-2-yl)piperidine-2,6-dione;
3-(6-bromo-4-(bromomethyl)-1-oxoisoindolin-2-yl)piperidine-2,6-dione;
3-(5-bromo-4-(bromomethyl)-1-oxoisoindolin-2-yl)piperidine-2,6-dione;
3-(4-((3,6-diazabicyclo[3.1.1]heptan-3-yl)methyl)-1-oxoisoindolin-2-yl)piperidine-2,6-dione;
3-(4-((3,6-diazabicyclo[3.1.1]heptan-6-yl)methyl)-1-oxoisoindolin-2-yl)piperidine-2,6-dione;
3-(4-((3,8-diazabicyclo[3.2.1]octan-8-yl)methyl)-1-oxoisoindolin-2-yl)piperidine-2,6-dione;
3-(4-((3,8-diazabicyclo[3.2.1]octan-3-yl)methyl)-1-oxoisoindolin-2-yl)piperidine-2,6-dione;
3-(4-((2,5-diazabicyclo[2.2.1]heptan-2-yl)methyl)-1-oxoisoindolin-2-yl)piperidine-2,6-dione;
3-(4-((2,5-diazabicyclo[2.2.2]octan-2-yl)methyl)-1-oxoisoindolin-2-yl)piperidine-2,6-dione;
3-(1-oxo-4-((piperazin-1-yl-2,2,3,3,5,5,6,6- d8)methyl)isoindolin-2-yl)piperidine-2,6-dione;
3-(4-((2,6-dimethylpiperazin-1-yl)methyl)-1-oxoisoindolin-2-yl)piperidine-2,6-dione;
3-(4-((3,5-dimethylpiperazin-1-yl)methyl)-1-oxoisoindolin-2-yl)piperidine-2,6-dione;
3-(7-fluoro-1-oxo-4-(piperazin-1-ylmethyl)isoindolin-2-yl)piperidine-2,6-dione;
3-(7-chloro-1-oxo-4-(piperazin-1-ylmethyl)isoindolin-2-yl)piperidine-2,6-dione;
3-(7-bromo-1-oxo-4-(piperazin-1-ylmethyl)isoindolin-2-yl)piperidine-2,6-dione;
3-(5-fluoro-1-oxo-4-(piperazin-1-ylmethyl)isoindolin-2-yl)piperidine-2,6-dione;
3-(5-chloro-1-oxo-4-(piperazin-1-ylmethyl)isoindolin-2-yl)piperidine-2,6-dione;
3-(5-bromo-1-oxo-4-(piperazin-1-ylmethyl)isoindolin-2-yl)piperidine-2,6-dione;
3-(6-fluoro-1-oxo-4-(piperazin-1-ylmethyl)isoindolin-2-yl)piperidine-2,6-dione;
3-(6-chloro-1-oxo-4-(piperazin-1-ylmethyl)isoindolin-2-yl)piperidine-2,6-dione;
3-(6-bromo-1-oxo-4-(piperazin-1-ylmethyl)isoindolin-2-yl)piperidine-2,6-dione;
3-(6-(bromomethyl)-1-oxoisoindolin-2-yl)piperidine-2,6-dione;
3-(7-(bromomethyl)-1-oxoisoindolin-2-yl)piperidine-2,6-dione;
3-(5-(((R)-3-aminopiperidin-1-yl)methyl)-1-oxoisoindolin-2-yl)piperidine-2,6-dione;
3-(5-(((S)-3-aminopiperidin-1-yl)methyl)-1-oxoisoindolin-2-yl)piperidine-2,6-dione;
3-(5-((4-aminopiperidin-1-yl)methyl)-1-oxoisoindolin-2-yl)piperidine-2,6-dione;
3-(1-oxo-5-(piperidin-4-ylmethyl)isoindolin-2-yl)piperidine-2,6-dione;
3-(5-(azetidin-3-ylmethyl)-1-oxoisoindolin-2-yl)piperidine-2,6-dione;
3-(4-(((R)-3-aminopiperidin-1-yl)methyl)-1-oxoisoindolin-2-yl)piperidine-2,6-dione;
3-(4-(((S)-3-aminopiperidin-1-yl)methyl)-1-oxoisoindolin-2-yl)piperidine-2,6-dione;
3-(4-((4-aminopiperidin-1-yl)methyl)-1-oxoisoindolin-2-yl)piperidine-2,6-dione3-(1-oxo-4-(piperazin-1-ylmethyl)isoindolin-2-yl)piperidine-2,6-dione;
3-(4-(azetidin-3-ylmethyl)-1-oxoisoindolin-2-yl)piperidine-2,6-dione;
5-(chloromethyl)-2-(2,6-dioxopiperidin-3-yl)isoindoline-1,3-dione;
2-(2,6-dioxopiperidin-3-yl)-5-(iodomethyl)isoindoline-1,3-dione;
2-(2,6-dioxopiperidin-3-yl)-5-(hydroxymethyl)isoindoline-1,3-dione;
2-(2,6-dioxopiperidin-3-yl)-4-fluoro-5-(hydroxymethyl)isoindoline-1,3-dione;
2-(2,6-dioxopiperidin-3-yl)-5-fluoro-6-(hydroxymethyl)isoindoline-1,3-dione;
2-(2,6-dioxopiperidin-3-yl)-4-fluoro-6-(hydroxymethyl)isoindoline-1,3-dione;
5-(aminomethyl)-2-(2,6-dioxopiperidin-3-yl)-4-fluoroisoindoline-1,3-dione;
5-(aminomethyl)-2-(2,6-dioxopiperidin-3-yl)-6-fluoroisoindoline-1,3-dione;
6-(aminomethyl)-2-(2,6-dioxopiperidin-3-yl)-4-fluoroisoindoline-1,3-dione;
(2-(2,6-dioxopiperidin-3-yl)-1,3-dioxoisoindolin-5-yl)methyl 4-methylbenzenesulfonate;
(2-(2,6-dioxopiperidin-3-yl)-1,3-dioxoisoindolin-5-yl)methyl methanesulfonate;
(2-(2,6-dioxopiperidin-3-yl)-1,3-dioxoisoindolin-5-yl)methyl trifluoromethanesulfonate;
6-(bromomethyl)-2-(2,6-dioxopiperidin-3-yl)-4-fluoroisoindoline-1,3-dione;
5-(bromomethyl)-2-(2,6-dioxopiperidin-3-yl)-4-fluoroisoindoline-1,3-dione;
5-(bromomethyl)-2-(2,6-dioxopiperidin-3-yl)-6-fluoroisoindoline-1,3-dione;
4- bromo-6-(bromomethyl)-2-(2,6-dioxopiperidin-3-yl)isoindoline-1,3-dione;
5-bromo-6-(bromomethyl)-2-(2,6-dioxopiperidin-3-yl)isoindoline-1,3-dione;
4-bromo-5-(bromomethyl)-2-(2,6-dioxopiperidin-3-yl)isoindoline-1,3-dione;
5-((3,6-diazabicyclo[3.1.1]heptan-3-yl)methyl)-2-(2,6-dioxopiperidin-3-yl)isoindoline-1,3-dione;
5-((3,6-diazabicyclo[3.1.1]heptan-6-yl)methyl)-2-(2,6-dioxopiperidin-3-yl)isoindoline-1,3-dione;
5-((3,8-diazabicyclo[3.2.1]octan-8-yl)methyl)-2-(2,6-dioxopiperidin-3-yl)isoindoline-1,3-dione;
5-((3,8-diazabicyclo[3.2.1]octan-3-yl)methyl)-2-(2,6-dioxopiperidin-3-yl)isoindoline-1,3-dione;
5-((2,5-diazabicyclo[2.2.1]heptan-2-yl)methyl)-2-(2,6-dioxopiperidin-3-yl)isoindoline-1,3-dione;
5-((2,5-diazabicyclo[2.2.2]octan-2-yl)methyl)-2-(2,6-dioxopiperidin-3-yl)isoindoline-1,3-dione;
2-(2,6-dioxopiperidin-3-yl)-5-((piperazin-1-yl-2,2,3,3,5,5,6,6- d8)methyl)isoindoline-1,3-dione;
5-((2,6-dimethylpiperazin-1-yl)methyl)-2-(2,6-dioxopiperidin-3-yl)isoindoline-1,3-dione;
5-((3,5-dimethylpiperazin-1-yl)methyl)-2-(2,6-dioxopiperidin-3-yl)isoindoline-1,3-dione;
2-(2,6-dioxopiperidin-3-yl)-4-fluoro-6-(piperazin-1-ylmethyl)isoindoline-1,3-dione;
4-chloro-2-(2,6-dioxopiperidin-3-yl)-6-(piperazin-1-ylmethyl)isoindoline-1,3-dione;
4-bromo-2-(2,6-dioxopiperidin-3-yl)-6-(piperazin-1-ylmethyl)isoindoline-1,3-dione;
2-(2,6-dioxopiperidin-3-yl)-4-fluoro-5-(piperazin-1-ylmethyl)isoindoline-1,3-dione;
4-chloro-2-(2,6-dioxopiperidin-3-yl)-5-(piperazin-1-ylmethyl)isoindoline-1,3-dione;
4-bromo-2-(2,6-dioxopiperidin-3-yl)-5-(piperazin-1-ylmethyl)isoindoline-1,3-dione;
2-(2,6-dioxopiperidin-3-yl)-5-fluoro-6-(piperazin-1-ylmethyl)isoindoline-1,3-dione;
5-chloro-2-(2,6-dioxopiperidin-3-yl)-6-(piperazin-1-ylmethyl)isoindoline-1,3-dione;
5-bromo-2-(2,6-dioxopiperidin-3-yl)-6-(piperazin-1-ylmethyl)isoindoline-1,3-dione;
4-(chloromethyl)-2-(2,6-dioxopiperidin-3-yl)isoindoline-1,3-dione;
2-(2,6-dioxopiperidin-3-yl)-4-(iodomethyl)isoindoline-1,3-dione;
2-(2,6-dioxopiperidin-3-yl)-4-(hydroxymethyl)isoindoline-1,3-dione;
2-(2,6-dioxopiperidin-3-yl)-5-fluoro-4-(hydroxymethyl)isoindoline-1,3-dione;
2-(2,6-dioxopiperidin-3-yl)-6-fluoro-4-(hydroxymethyl)isoindoline-1,3-dione;
2-(2,6-dioxopiperidin-3-yl)-4-fluoro-7-(hydroxymethyl)isoindoline-1,3-dione;
4-(aminomethyl)-2-(2,6-dioxopiperidin-3-yl)-5-fluoroisoindoline-1,3-dione;
4-(aminomethyl)-2-(2,6-dioxopiperidin-3-yl)-6-fluoroisoindoline-1,3-dione;
4-(aminomethyl)-2-(2,6-dioxopiperidin-3-yl)-7-fluoroisoindoline-1,3-dione;
(2-(2,6-dioxopiperidin-3-yl)-1,3-dioxoisoindolin-4-yl)methyl 4-methylbenzenesulfonate;
(2-(2,6-dioxopiperidin-3-yl)-1,3-dioxoisoindolin-4-yl)methyl methanesulfonate;
(2-(2,6-dioxopiperidin-3-yl)-1,3-dioxoisoindolin-4-yl)methyl trifluoromethanesulfonate;
4-(bromomethyl)-2-(2,6-dioxopiperidin-3-yl)-7-fluoroisoindoline-1,3-dione;
4-(bromomethyl)-2-(2,6-dioxopiperidin-3-yl)-5-fluoroisoindoline-1,3-dione;
4-(bromomethyl)-2-(2,6-dioxopiperidin-3-yl)-6-fluoroisoindoline-1,3-dione;
4-bromo-7-(bromomethyl)-2-(2,6-dioxopiperidin-3-yl)isoindoline-1,3-dione;
6-bromo-4-(bromomethyl)-2-(2,6-dioxopiperidin-3-yl)isoindoline-1,3-dione;
5-bromo-4-(bromomethyl)-2-(2,6-dioxopiperidin-3-yl)isoindoline-1,3-dione;
4-((3,6-diazabicyclo[3.1.1]heptan-3-yl)methyl)-2-(2,6-dioxopiperidin-3-yl)isoindoline-1,3-dione;
4-((3,6-diazabicyclo[3.1.1]heptan-6-yl)methyl)-2-(2,6-dioxopiperidin-3-yl)isoindoline-1,3-dione;
4-((3,8-diazabicyclo[3.2.1]octan-8-yl)methyl)-2-(2,6-dioxopiperidin-3-yl)isoindoline-1,3-dione;
4-((3,8-diazabicyclo[3.2.1]octan-3-yl)methyl)-2-(2,6-dioxopiperidin-3-yl)isoindoline-1,3-dione;
4-((2,5-diazabicyclo[2.2.1]heptan-2-yl)methyl)-2-(2,6-dioxopiperidin-3-yl)isoindoline-1,3-dione;
4-((2,5 -di azabicyclo [2.2.2]octan-2-yl)methyl)-2-(2,6-dioxopiperi din-3 -yl)isoindoline-1,3 -dione;
2-(2,6-dioxopiperidin-3-yl)-4-((piperazin-1-yl-2,2,3,3,5,5,6,6- d8)methyl)isoindoline-1,3-dione;
4-((2,6-dimethylpiperazin-1-yl)methyl)-2-(2,6-dioxopiperidin-3-yl)isoindoline-1,3-dione;
4-((3,5-dimethylpiperazin-1-yl)methyl)-2-(2,6-dioxopiperidin-3-yl)isoindoline-1,3-dione;
2-(2,6-dioxopiperidin-3-yl)-4-fluoro-7-(piperazin-1-ylmethyl)isoindoline-1,3-dione;
4-chloro-2-(2,6-dioxopiperidin-3-yl)-7-(piperazin-1-ylmethyl)isoindoline-1,3-dione;
4-bromo-2-(2,6-dioxopiperidin-3-yl)-7-(piperazin-1-ylmethyl)isoindoline-1,3-dione;
2-(2,6-dioxopiperidin-3-yl)-5-fluoro-4-(piperazin-1-ylmethyl)isoindoline-1,3-dione;
5-chloro-2-(2,6-dioxopiperidin-3-yl)-4-(piperazin-1-ylmethyl)isoindoline-1,3-dione;
5-bromo-2-(2,6-dioxopiperidin-3-yl)-4-(piperazin-1-ylmethyl)isoindoline-1,3-dione;
2-(2,6-dioxopiperidin-3-yl)-6-fluoro-4-(piperazin-1-ylmethyl)isoindoline-1,3-dione;
6-chloro-2-(2,6-dioxopiperidin-3-yl)-4-(piperazin-1-ylmethyl)isoindoline-1,3-dione;
6-bromo-2-(2,6-dioxopiperidin-3-yl)-4-(piperazin-1-ylmethyl)isoindoline-1,3-dione;
5-(((R)-3-aminopiperidin-1-yl)methyl)-2-(2,6-dioxopiperidin-3-yl)isoindoline-1,3-dione;
5-(((S)-3-aminopiperidin-1-yl)methyl)-2-(2,6-dioxopiperidin-3-yl)isoindoline-1,3-dione;
5-((4-aminopiperidin-1-yl)methyl)-2-(2,6-dioxopiperidin-3-yl)isoindoline-1,3-dione;
2-(2,6-dioxopiperidin-3-yl)-5-(piperazin-1-ylmethyl)isoindoline-1,3-dione;
2-(2,6-dioxopiperidin-3-yl)-5-(piperidin-4-ylmethyl)isoindoline-1,3-dione;
5-(azetidin-3-ylmethyl)-2-(2,6-dioxopiperidin-3-yl)isoindoline-1,3-dione;
4-(((R)-3-aminopiperidin-1-yl)methyl)-2-(2,6-dioxopiperidin-3-yl)isoindoline-1,3-dione;
4-(((S)-3-aminopiperidin-1-yl)methyl)-2-(2,6-dioxopiperidin-3-yl)isoindoline-1,3-dione;
4-((4-aminopiperidin-1-yl)methyl)-2-(2,6-dioxopiperidin-3-yl)isoindoline-1,3-dione;
4-(azetidin-3-ylmethyl)-2-(2,6-dioxopiperidin-3-yl)isoindoline-1,3-dione;
(E)-4-(4-((2-(2,6-dioxopiperidin-3-yl)-1-oxoisoindolin-5-yl)methyl)piperazin-1-yl)but-2-enoic acid;
(E)-4-(4-((2-(2,6-dioxopiperidin-3-yl)-1-oxoisoindolin-4-yl)methyl)piperazin-1-yl)but-2-enoic acid;
(E)-4-(4-((2-(2,6-dioxopiperidin-3-yl)-1,3-dioxoisoindolin-5-yl)methyl)piperazin-1-yl)but-2-enoic acid;
(E)-4-(4-((2-(2,6-dioxopiperidin-3-yl)-1,3-dioxoisoindolin-4-yl)methyl)piperazin-1-yl)but-2-enoic acid;
3-(5-((4-(3-hydroxypropyl)piperazin-1-yl)methyl)-1-oxoisoindolin-2-yl)piperidine-2,6-dione;
3-(4-((4-(3-hydroxypropyl)piperazin-1-yl)methyl)-1-oxoisoindolin-2-yl)piperidine-2,6-dione;
2-(2,6-dioxopiperidin-3-yl)-5-((4-(3-hydroxypropyl)piperazin-1-yl)methyl)isoindoline-1,3-dione;
2-(2,6-dioxopiperidin-3-yl)-4-((4-(3-hydroxypropyl)piperazin-1-yl)methyl)isoindoline-1,3-dione;
3-(5-((4-(3-bromopropyl)piperazin-1-yl)methyl)-1-oxoisoindolin-2-yl)piperidine-2,6-dione;
3-(4-((4-(3-bromopropyl)piperazin-1-yl)methyl)-1-oxoisoindolin-2-yl)piperidine-2,6-dione;
5-((4-(3-bromopropyl)piperazin-1-yl)methyl)-2-(2,6-dioxopiperidin-3-yl)isoindoline-1,3-dione;
4-((4-(3-bromopropyl)piperazin-1-yl)methyl)-2-(2,6-dioxopiperidin-3-yl)isoindoline-1,3-dione;
3-(5-((4-hydroxypiperidin-1-yl)methyl)-1-oxoisoindolin-2-yl)piperidine-2,6-dione;
3-(4-((4-hydroxypiperidin-1-yl)methyl)-1-oxoisoindolin-2-yl)piperidine-2,6-dione;
2-(2,6-dioxopiperidin-3-yl)-5-((4-hydroxypiperidin-1-yl)methyl)isoindoline-1,3-dione;
2-(2,6-dioxopiperidin-3-yl)-4-((4-hydroxypiperidin-1-yl)methyl)isoindoline-1,3-dione;
3-(5-((4-bromopiperidin-1-yl)methyl)-1-oxoisoindolin-2-yl)piperidine-2,6-dione;
3-(4-((4-bromopiperidin-1-yl)methyl)-1-oxoisoindolin-2-yl)piperidine-2,6-dione;
5-((4-bromopiperidin-1-yl)methyl)-2-(2,6-dioxopiperidin-3-yl)isoindoline-1,3-dione;
4-((4-bromopiperidin-1-yl)methyl)-2-(2,6-dioxopiperidin-3-yl)isoindoline-1,3-dione;
3-(5-(((2-bromoethyl)(methyl)amino)methyl)-1-oxoisoindolin-2-yl)piperidine-2,6-dione;
3-(4-(((2-bromoethyl)(methyl)amino)methyl)-1-oxoisoindolin-2-yl)piperidine-2,6-dione;
5-(((2-bromoethyl)(methyl)amino)methyl)-2-(2,6-dioxopiperidin-3-yl)isoindoline-1,3-dione;
4-(((2-bromoethyl)(methyl)amino)methyl)-2-(2,6-dioxopiperidin-3-yl)isoindoline-1,3-dione;
3-(5-((methyl(piperidin-4-yl)amino)methyl)-1-oxoisoindolin-2-yl)piperidine-2,6-dione;
3-(4-((methyl(piperidin-4-yl)amino)methyl)-1-oxoisoindolin-2-yl)piperidine-2,6-dione;
2-(2,6-dioxopiperidin-3-yl)-5-((methyl(piperidin-4-yl)amino)methyl)isoindoline-1,3-dione;
2-(2,6-dioxopiperidin-3-yl)-4-((methyl(piperidin-4-yl)amino)methyl)isoindoline-1,3-dione;
3-(1-oxo-5-((4-(piperazin-1-yl)piperidin-1-yl)methyl)isoindolin-2-yl)piperidine-2,6-dione;
3-(1-oxo-4-((4-(piperazin-1-yl)piperidin-1-yl)methyl)isoindolin-2-yl)piperidine-2,6-dione;
2-(2,6-dioxopiperidin-3-yl)-5-((4-(piperazin-1-yl)piperidin-1-yl)methyl)isoindoline-1,3-dione;
2-(2,6-dioxopiperidin-3-yl)-4-((4-(piperazin-1-yl)piperidin-1-yl)methyl)isoindoline-1,3-dione;
3-(1-oxo-5-((4-(piperidin-4-yl)piperazin-1-yl)methyl)isoindolin-2-yl)piperidine-2,6-dione;
3-(1-oxo-4-((4-(piperidin-4-yl)piperazin-1-yl)methyl)isoindolin-2-yl)piperidine-2,6-dione;
2-(2,6-dioxopiperidin-3-yl)-5-((4-(piperidin-4-yl)piperazin-1-yl)methyl)isoindoline-1,3-dione;
2-(2,6-dioxopiperidin-3-yl)-4-((4-(piperidin-4-yl)piperazin-1-yl)methyl)isoindoline-1,3-dione;
3-(1-oxo-5-((4-phenylpiperazin-1-yl)methyl)isoindolin-2-yl)piperidine-2,6-dione;
3-(1-oxo-4-((4-phenylpiperazin-1-yl)methyl)isoindolin-2-yl)piperidine-2,6-dione;
2-(2,6-dioxopiperidin-3-yl)-5-((4-phenylpiperazin-1-yl)methyl)isoindoline-1,3-dione;
2-(2,6-dioxopiperidin-3-yl)-4-((4-phenylpiperazin-1-yl)methyl)isoindoline-1,3-dione;
3-(5-((4-(4-bromophenyl)piperazin-1-yl)methyl)-1-oxoisoindolin-2-yl)piperidine-2,6-dione;
3-(4-((4-(4-bromophenyl)piperazin-1-yl)methyl)-1-oxoisoindolin-2-yl)piperidine-2,6-dione;
5-((4-(4-bromophenyl)piperazin-1-yl)methyl)-2-(2,6-dioxopiperidin-3-yl)isoindoline-1,3-dione;
4-((4-(4-bromophenyl)piperazin-1-yl)methyl)-2-(2,6-dioxopiperidin-3-yl)isoindoline-1,3-dione;
3-(1-oxo-5-((4-(pyridazin-3-yl)piperazin-1-yl)methyl)isoindolin-2-yl)piperidine-2,6-dione;
3-(1-oxo-4-((4-(pyridazin-3-yl)piperazin-1-yl)methyl)isoindolin-2-yl)piperidine-2,6-dione;
2-(2,6-dioxopiperidin-3-yl)-5-((4-(pyridazin-3-yl)piperazin-1-yl)methyl)isoindoline-1,3-dione;
2-(2,6-dioxopiperidin-3-yl)-4-((4-(pyridazin-3-yl)piperazin-1-yl)methyl)isoindoline-1,3-dione;
3-(5-((4-(6-chloropyridazin-3-yl)piperazin-1-yl)methyl)-1-oxoisoindolin-2-yl)piperidine-2,6-dione;
3-(4-((4-(6-chloropyridazin-3-yl)piperazin-1-yl)methyl)-1-oxoisoindolin-2-yl)piperidine-2,6-dione;
5-((4-(6-chloropyridazin-3-yl)piperazin-1-yl)methyl)-2-(2,6-dioxopiperidin-3-yl)isoindoline-1,3-dione;
4-((4-(6-chloropyridazin-3-yl)piperazin-1-yl)methyl)-2-(2,6-dioxopiperidin-3-yl)isoindoline-1,3-dione;
1-((2-(2,6-dioxopiperidin-3-yl)-1-oxoisoindolin-5-yl)methyl)-3-fluoropiperidin-4-yl methanesulfonate;
1-((2-(2,6-dioxopiperidin-3-yl)-1-oxoisoindolin-4-yl)methyl)-3-fluoropiperidin-4-yl methanesulfonate;
1-((2-(2,6-dioxopiperidin-3-yl)-1,3-dioxoisoindolin-5-yl)methyl)-3-fluoropiperidin-4-yl methanesulfonate;
1-((2-(2,6-dioxopiperidin-3-yl)-1,3-dioxoisoindolin-4-yl)methyl)-3-fluoropiperidin-4-yl methanesulfonate;
1-((2-(2,6-dioxopiperidin-3-yl)-1-oxoisoindolin-5-yl)methyl)piperidin-3-yl trifluoromethanesulfonate;
1-((2-(2,6-dioxopiperidin-3-yl)-1-oxoisoindolin-4-yl)methyl)piperidin-3-yl trifluoromethanesulfonate;
1-((2-(2,6-dioxopiperidin-3-yl)-1,3-dioxoisoindolin-5-yl)methyl)piperidin-3-yl trifluoromethanesulfonate;
1-((2-(2,6-dioxopiperidin-3-yl)-1,3-dioxoisoindolin-4-yl)methyl)piperidin-3-yl trifluoromethanesulfonate;
3-(5-((4-(6-aminopyridin-3-yl)piperazin-1-yl)methyl)-1-oxoisoindolin-2-yl)piperidine-2,6-dione;
3-(4-((4-(6-aminopyridin-3-yl)piperazin-1-yl)methyl)-1-oxoisoindolin-2-yl)piperidine-2,6-dione;
5 -((4-(6-aminopyridin-3 -yl)pip erazin-1-yl)methyl)-2-(2, 6-dioxopip eridin-3 -yl)i soindoline-1, 3 - dione;
4-((4-(6-aminopyridin-3 -yl)pip erazin-1-yl)methyl)-2-(2, 6-dioxopip eridin-3 -yl)i soindoline-1, 3 - dione;
3-(5-((4-(azetidin-3-yl)piperazin-1-yl)methyl)-1-oxoisoindolin-2-yl)piperidine-2,6-dione;
3-(4-((4-(azetidin-3-yl)piperazin-1-yl)methyl)-1-oxoisoindolin-2-yl)piperidine-2,6-dione;
5-((4-(azetidin-3-yl)piperazin-1-yl)methyl)-2-(2,6-dioxopiperidin-3-yl)isoindoline-1,3-dione;
4-((4-(azetidin-3-yl)piperazin-1-yl)methyl)-2-(2,6-dioxopiperidin-3-yl)isoindoline-1,3-dione;
3-(1-oxo-5-((3-(piperazin-1-yl)azetidin-1-yl)methyl)isoindolin-2-yl)piperidine-2,6-dione;
3-(1-oxo-4-((3-(piperazin-1-yl)azetidin-1-yl)methyl)isoindolin-2-yl)piperidine-2,6-dione;
2-(2,6-dioxopiperidin-3-yl)-5-((3-(piperazin-1-yl)azetidin-1-yl)methyl)isoindoline-1,3-dione;
2-(2,6-dioxopiperidin-3-yl)-4-((3-(piperazin-1-yl)azetidin-1-yl)methyl)isoindoline-1,3-dione;
3-(5-((4-methylpiperazin-1-yl)methyl)-1-oxoisoindolin-2-yl)piperidine-2,6-dione;
3-(4-((4-methylpiperazin-1-yl)methyl)-1-oxoisoindolin-2-yl)piperidine-2,6-dione;
2-(2,6-dioxopiperidin-3-yl)-5-((4-methylpiperazin-1-yl)methyl)isoindoline-1,3-dione;
2-(2,6-dioxopiperidin-3-yl)-4-((4-methylpiperazin-1-yl)methyl)isoindoline-1,3-dione;
3-(5-((4-(difluoromethyl)piperazin-1-yl)methyl)-1-oxoisoindolin-2-yl)piperidine-2,6-dione;
3 -(4-((4-(difluoromethyl)pip erazin-1-yl)methyl)-1-oxoisoindolin-2-yl)pip eri dine-2, 6-dione;
5-((4-(difluoromethyl)piperazin-1-yl)methyl)-2-(2,6-dioxopiperidin-3-yl)isoindoline-1,3-dione;
4-((4-(difluoromethyl)piperazin-1-yl)methyl)-2-(2,6-dioxopiperidin-3-yl)isoindoline-1,3-dione;
3-(1-oxo-5-((piperidin-4-ylamino)methyl)isoindolin-2-yl)piperidine-2,6-dione;
3-(1-oxo-4-((piperidin-4-ylamino)methyl)isoindolin-2-yl)piperidine-2,6-dione;
2-(2,6-dioxopiperidin-3-yl)-5-((piperidin-4-ylamino)methyl)isoindoline-1,3-dione;
2-(2,6-dioxopiperidin-3-yl)-4-((piperidin-4-ylamino)methyl)isoindoline-1,3-dione;
3-(5-((4-(2-aminoethyl)piperazin-1-yl)methyl)-1-oxoisoindolin-2-yl)piperidine-2,6-dione;
3-(4-((4-(2-aminoethyl)piperazin-1-yl)methyl)-1-oxoisoindolin-2-yl)piperidine-2,6-dione;
5-((4-(2-aminoethyl)piperazin-1-yl)methyl)-2-(2,6-dioxopiperidin-3-yl)isoindoline-1,3-dione;
4-((4-(2-aminoethyl)piperazin-1-yl)methyl)-2-(2,6-dioxopiperidin-3-yl)isoindoline-1,3-dione;
3-(1-oxo-5-((4-propylpiperazin-1-yl)methyl)isoindolin-2-yl)piperidine-2,6-dione;
3-(1-oxo-4-((4-propylpiperazin-1-yl)methyl)isoindolin-2-yl)piperidine-2,6-dione;
2-(2,6-dioxopiperidin-3-yl)-5-((4-propylpiperazin-1-yl)methyl)isoindoline-1,3-dione;
2-(2,6-dioxopiperidin-3-yl)-4-((4-propylpiperazin-1-yl)methyl)isoindoline-1,3-dione;
3-(5-(((2-hydroxyethyl)amino)methyl)-1-oxoisoindolin-2-yl)piperidine-2,6-dione;
3-(4-(((2-hydroxyethyl)amino)methyl)-1-oxoisoindolin-2-yl)piperidine-2,6-dione;
2-(2,6-dioxopiperidin-3-yl)-5-(((2-hydroxyethyl)amino)methyl)isoindoline-1,3-dione;
2-(2,6-dioxopiperidin-3-yl)-4-(((2-hydroxyethyl)amino)methyl)isoindoline-1,3-dione;
1-((2-(2,6-dioxopiperidin-3-yl)-1-oxoisoindolin-5-yl)methyl)piperidine-4-carboxylic acid;
1-((2-(2,6-dioxopiperidin-3-yl)-1-oxoisoindolin-4-yl)methyl)piperidine-4-carboxylic acid;
1-((2-(2,6-dioxopiperidin-3-yl)-1,3-dioxoisoindolin-5-yl)methyl)piperidine-4-carboxylic acid;
1-((2-(2,6-dioxopiperidin-3-yl)-1,3-dioxoisoindolin-4-yl)methyl)piperidine-4-carboxylic acid;
1'-((2-(2,6-dioxopiperidin-3-yl)-1-oxoisoindolin-5-yl)methyl)-[1,4'-bipiperidine]-4-carboxylic acid;
1'-((2-(2,6-dioxopiperidin-3-yl)-1-oxoisoindolin-4-yl)methyl)-[1,4'-bipiperidine]-4-carboxylic acid;
1'-((2-(2,6-dioxopiperidin-3-yl)-1,3-dioxoisoindolin-5-yl)methyl)-[1,4'-bipiperidine]-4-carboxylic acid;
1'-((2-(2,6-dioxopiperidin-3-yl)-1,3-dioxoisoindolin-4-yl)methyl)-[1,4'-bipiperidine]-4-carboxylic acid;
3-(5-((4-ethynylpiperidin-1-yl)methyl)-1-oxoisoindolin-2-yl)piperidine-2,6-dione;
3 -(4-((4-ethynylpip eridin-1-yl)methyl)-1-oxoisoindolin-2-yl)pip eri dine-2, 6-dione;
2-(2,6-dioxopiperidin-3-yl)-5-((4-ethynylpiperidin-1-yl)methyl)isoindoline-1,3-dione;
2-(2,6-dioxopiperidin-3-yl)-4-((4-ethynylpiperidin-1-yl)methyl)isoindoline-1,3-dione;
3 -(1 -oxo-5 -((3 -(piperazin-1 -yl)piperidin-1 -yl)methyl)isoindolin-2-yl)piperidine-2,6-dione;
3 -(1 -oxo-4-((3 -(piperazin-1-yl)piperidin-1-yl)methyl)isoindolin-2-yl)piperidine-2,6-dione;
2-(2,6-dioxopiperidin-3-yl)-5-((3-(piperazin-1-yl)piperidin-1-yl)methyl)isoindoline-1,3-dione;
2-(2,6-dioxopiperidin-3-yl)-4-((3-(piperazin-1-yl)piperidin-1-yl)methyl)isoindoline-1,3-dione;
3-(5-(((4-methylpiperidin-4-yl)amino)methyl)-1-oxoisoindolin-2-yl)piperidine-2,6-dione;
3-(4-(((4-methylpiperidin-4-yl)amino)methyl)-1-oxoisoindolin-2-yl)piperidine-2,6-dione;
2-(2,6-dioxopiperidin-3-yl)-5-(((4-methylpiperidin-4-yl)amino)methyl)isoindoline-1,3-dione;
2-(2,6-dioxopiperidin-3-yl)-4-(((4-methylpiperidin-4-yl)amino)methyl)isoindoline-1,3-dione;
3-(5-(((3-methyl-2-oxa-8-azaspiro[4.5]decan-4-yl)amino)methyl)-1-oxoisoindolin-2-yl)piperidine-2,6-dione;
3-(4-(((3-methyl-2-oxa-8-azaspiro[4.5]decan-4-yl)amino)methyl)-1-oxoisoindolin-2-yl)piperidine-2,6-dione;
2-(2,6-dioxopiperidin-3-yl)-5-(((3-methyl-2-oxa-8-azaspiro[4.5]decan-4-yl)amino)methyl)isoindoline-1,3-dione;
2-(2,6-dioxopiperidin-3-yl)-4-(((3-methyl-2-oxa-8-azaspiro[4.5]decan-4-yl)amino)methyl)isoindoline-1,3-dione;
2-(2,6-dioxopiperidin-3-yl)-6-fluoro-1-oxoisoindoline-5-carbaldehyde;
2-(2,6-dioxopiperidin-3-yl)-4-fluoro-1-oxoisoindoline-5-carbaldehyde;
2-(2,6-dioxopiperidin-3-yl)-7-fluoro-1-oxoisoindoline-5-carbaldehyde;
2-(2,6-dioxopiperidin-3-yl)-5-fluoro-1-oxoisoindoline-4-carbaldehyde;
2-(2,6-dioxopiperidin-3-yl)-6-fluoro-1-oxoisoindoline-4-carbaldehyde;
2-(2,6-dioxopiperidin-3-yl)-7-fluoro-1-oxoisoindoline-4-carbaldehyde;
2-(2,6-dioxopiperidin-3-yl)-4-fluoro-1,3-dioxoisoindoline-5-carbaldehyde;
2-(2,6-dioxopiperidin-3-yl)-6-fluoro-1,3-dioxoisoindoline-5-carbaldehyde;
2-(2,6-dioxopiperidin-3-yl)-7-fluoro-1,3-dioxoisoindoline-5-carbaldehyde;
2-(2,6-dioxopiperidin-3-yl)-5-fluoro-1,3-dioxoisoindoline-4-carbaldehyde;
2-(2,6-dioxopiperidin-3-yl)-6-fluoro-1,3-dioxoisoindoline-4-carbaldehyde;
2-(2,6-dioxopiperidin-3-yl)-7-fluoro-1,3-dioxoisoindoline-4-carbaldehyde;
3-(4-bromo-6-(bromomethyl)-1-oxoisoindolin-2-yl)piperidine-2,6-dione;
3-(5-bromo-6-(bromomethyl)-1-oxoisoindolin-2-yl)piperidine-2,6-dione;
3-(7-bromo-6-(bromomethyl)-1-oxoisoindolin-2-yl)piperidine-2,6-dione;
3-(6-(bromomethyl)-4-fluoro-1-oxoisoindolin-2-yl)piperidine-2,6-dione;
3-(6-(bromomethyl)-5-fluoro-1-oxoisoindolin-2-yl)piperidine-2,6-dione;
3-(6-(bromomethyl)-7-fluoro-1-oxoisoindolin-2-yl)piperidine-2,6-dione;
3-(7-(bromomethyl)-1-oxoisoindolin-2-yl)piperidine-2,6-dione;
3-(4-bromo-7-(bromomethyl)-1-oxoisoindolin-2-yl)piperidine-2,6-dione;
3-(5-bromo-7-(bromomethyl)-1-oxoisoindolin-2-yl)piperidine-2,6-dione;
3-(6-bromo-7-(bromomethyl)-1-oxoisoindolin-2-yl)piperidine-2,6-dione;
3-(7-(bromomethyl)-4-fluoro-1-oxoisoindolin-2-yl)piperidine-2,6-dione;
3-(7-(bromomethyl)-5-fluoro-1-oxoisoindolin-2-yl)piperidine-2,6-dione;
3-(7-(bromomethyl)-6-fluoro-1-oxoisoindolin-2-yl)piperidine-2,6-dione;
4-bromo-6-(bromomethyl)-2-(2,6-dioxopiperidin-3-yl)isoindoline-1,3-dione;
5-bromo-6-(bromomethyl)-2-(2,6-dioxopiperidin-3-yl)isoindoline-1,3-dione;
4-bromo-5-(bromomethyl)-2-(2,6-dioxopiperidin-3-yl)isoindoline-1,3-dione;
6-(bromomethyl)-2-(2,6-dioxopiperidin-3-yl)-4-fluoroisoindoline-1,3-dione;
5-(bromomethyl)-2-(2,6-dioxopiperidin-3-yl)-6-fluoroisoindoline-1,3-dione;
5-(bromomethyl)-2-(2,6-dioxopiperidin-3-yl)-4-fluoroisoindoline-1,3-dione;
4-(bromomethyl)-2-(2,6-dioxopiperidin-3-yl)isoindoline-1,3-dione;
4-bromo-7-(bromomethyl)-2-(2,6-dioxopiperidin-3-yl)isoindoline-1,3-dione;
6-bromo-4-(bromomethyl)-2-(2,6-dioxopiperidin-3-yl)isoindoline-1,3-dione;
5-bromo-4-(bromomethyl)-2-(2,6-dioxopiperidin-3-yl)isoindoline-1,3-dione;
4-(bromomethyl)-2-(2,6-dioxopiperidin-3-yl)-7-fluoroisoindoline-1,3-dione;
4-(bromomethyl)-2-(2,6-dioxopiperidin-3-yl)-6-fluoroisoindoline-1,3-dione; and
4-(bromomethyl)-2-(2,6-dioxopiperidin-3-yl)-5-fluoroisoindoline-1,3-dione.

12. A compound of Formula (II)
PBM-R_{f}-LIN-ULM Formula (II)
or salts, enantiomers, stereoisomers, solvates, isotopically enriched analogs, prodrugs, or polymorphs thereof,
wherein PBM-R_{f}- represents a targeting moiety capable of binding protein, ULM represents a E3 ubiquitin ligase ligand moiety, LIN is a linker moiety, and PBM-R_{f}- is covalently bonded to ULM through LIN;
ULM represents the structure of Formula (ULM): 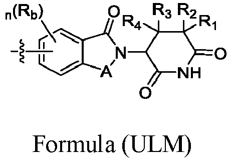
wherein A represents CO, CH₂ or CD₂;
R₁, R₂, R₃ and R₄ are the same or different and each independently represent hydrogen, deuterium, halogen, optionally deuterated C₁₋₆ alkyl, optionally deuterated C₁₋₆ alkoxy, or halogenated C₁₋₆ alkyl;
(R_{b})ₙ indicates that the benzene ring is optionally substituted with n R_{b} substituents, with each R_{b} being the same or different and independently representing deuterium, halogen, hydroxy, mercapto, nitro, amino, cyano, optionally deuterated C₁₋₆ alkyl, optionally deuterated C₁₋₆ alkoxy, halogenated C₁₋₆ alkyl, optionally substituted C₃₋₆ cycloalkyl, C₂₋₆ alkenyl or C₂₋₆ alkynyl;
n represents an integer of 0, 1, 2 or 3;
LIN represents the structure of the following formula: 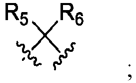
wherein R₅ and R₆ are the same or different and each independently represent hydrogen, fluorine, chlorine, bromine, iodine, optionally substituted methyl, or optionally substituted linear or branched C₂₋₃₀ alkyl, wherein one or more groups Rₑ and/or one or more groups R_{d} and/or any combination of one or more groups R_{c} and R_{d} are optionally inserted into the backbone carbon chain of the linear or branched C₂₋₃₀ alkyl group, wherein carbon-carbon bond between one or more pairs of adj acent carbon atoms in the backbone carbon chain is interrupted by the group R_{c}, R_{d}, or a combination of R_{c} and R_{d}; wherein each R_{c} is selected from the group consisting of O, N(Rₑ), C(O), C(O)O, S(O), S(O)₂, S(O)₂NH, NHS(O)₂, OC(O), C(O)N(Rₑ), N(Rₑ)C(O), or N(Rₑ)C(O)N(Rₑ), where each Rₑ independently represents H or C₁₋₆ alkyl, and in case that two or more groups Rₑ are inserted into the backbone carbon chain of the linear or branched C₂₋₃₀ alkyl group, the two or more groups Rₑ are not directly connected to each other; and wherein each R_{d} is selected from the group consisting of cycloalkylene, arylene, heterocyclylene, heteroarylene, alkynylene, alkenylene, or any combination thereof, wherein the cycloalkylene, arylene, heterocyclylene, and heteroarylene are each independently optionally substituted with a substituent selected from the group consisting of deuterium, optionally deuterated C₁₋₆ alkyl, optionally deuterated C₃₋₆ cycloalkyl, hydroxy, amino, mercapto, halogen, cyano, optionally deuterated C₁₋₆ alkoxy, optionally deuterated C₁₋₆ alkyl-NH-, halogenated C₁₋₆ alkyl, NH₂-C₁₋₆ alkylene, optionally deuterated C₁₋₆ alkyl-NHC(O)-, optionally deuterated C₁₋₆ alkyl-C(O)NH-, or any combination thereof;
R_{f} represents a bond, -O-, -N(R_{g})-, -NHC(O)-Rₕ-W⁵-*, -C(O)NH-Rₕ-W⁵-*, -NHC(O)-W⁵-*, - C(O)NH-W⁵-*, -NHC(O)-*, -C(O)NH-*, -O-Rₕ-W⁵-*, -O-W⁵-*, -O-Rₕ-N(Rᵢ)-*, -N(R_{g})-Rₕ-N(Rᵢ)-*, - W⁵-, -W⁵-N(R_{g})-*, -N(R_{g})-W⁵-*, -N(R_{g})-W⁵-N(Rᵢ)-*, -Rₕ-W⁵-*, -Rₕ-C(O)-W⁵-*, -C(O)-W⁵-*, -Rₕ-C(O)NH-Rⱼ-W⁵-*, -Rₕ-NHC(O)-Rⱼ-W⁵-*, -Rₕ-C(O)NH-*, -Rₕ-NHC(O)-*, -Rₕ-, -Rₕ-N(Rᵢ)-*, or 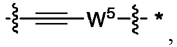
wherein W⁵ represents the structure of the following formula: 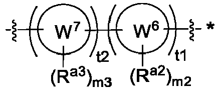
wherein each ring W⁶ is the same or different and each independently represents nitrogen-containing heterocyclylene, t1 represents an integer of 1 or 2, (R^{a2})ₘ₂ indicates that each ring W⁶ is optionally substituted with m2 R^{a2} substituents, with each R^{a2} independently representing deuterium, optionally deuterated C₁₋₆ alkyl, optionally halogenated C₁₋₆ alkyl, C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{12a}-R^{11a}-, where R^{11a} is optionally substituted C₁₋₆ alkylene or optionally substituted C₂₋₆ alkenylene, and R^{12a} represents halogen, R^{13a}R^{14a}N-, hydroxy, carboxyl, ethynyl, or vinyl, wherein R^{13a} and R^{14a} are the same or different and each independently represent hydrogen or C₁₋₃ alkyl, and m2 represents an integer of 0-20;
each ring W⁷ is the same or different and each independently represents nitrogen-containing heterocyclylene, arylene, or heteroarylene, t2 represents an integer of 0 or 1, (R^{a3})ₘ₃ indicates that each ring W⁷ is independently optionally substituted with m3 R^{a3} substituents, with each R^{a3} independently representing deuterium, optionally deuterated C₁₋₆ alkyl, C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{16a}-R^{15a}-, where R^{15a} is optionally substituted C₁₋₆ alkylene or optionally substituted C₂₋₆ alkenylene, and R^{16a} represents halogen, R^{17a}R^{18a}N-, hydroxy, carboxyl, ethynyl, or vinyl, wherein R^{17a} and R^{18a} are the same or different and each independently represent hydrogen or C₁₋₃ alkyl, and m3 represents an integer of 0-20; and
R_{g} and Rᵢ each independently represent hydrogen or C₁₋₆ alkyl, Rₕ and Rⱼ each independently represent optionally substituted C₁₋₆ alkylene or optionally substituted C₂₋₆ alkenylene, and symbol * indicates the point of attachment to LIN;
wherein when PBM represents the structure of the following formula: 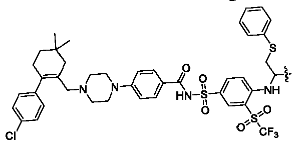 and simultaneously -R_{f}-LIN- represents 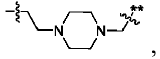 where symbol ** indicates the point of attachment to ULM, (R_{b})ₙ indicates that benzene ring of Formula (ULM) is substituted with 1, 2, or 3 R_{b}, with each R_{b} being the same or different and each independently representing deuterium, halogen, hydroxy, mercapto, nitro, amino, cyano, optionally deuterated C₁₋₆ alkyl, optionally deuterated C₁₋₆ alkoxy, halogenated C₁₋₆ alkyl, optionally substituted C₃₋₆ cycloalkyl, C₂₋₆ alkenyl, or C₂₋₆ alkynyl;
with the proviso that the following compounds are excluded:
3-(4-((3-(4-amino-3-(4-phenoxyphenyl)-1H-pyrazolo[3,4-*d*]pyrimidin-1-yl)piperidin-1- yl)methyl)-1-oxoisoindolin-2-yl)piperidine-2,6-dione;
N-(5-(3,5-difluorobenzyl)-1H-indazol-3-yl)-4-(4-(1-((2-(2,6-dioxopiperidin-3-yl)-1-oxoisoindolin-5-yl)methyl)piperidin-3-yl)piperazin-1-yl)-2-((tetrahydro-2H-pyran-4-yl)amino)benzamide;
N-(5-(3,5-difluorobenzyl)-1H-indazol-3-yl)-4-(4-((2-(2,6-dioxopiperidin-3-yl)-1,3-dioxoisoindolin-5-yl)methyl)piperazin-1-yl)-2-((tetrahydro-2H-pyran-4-yl)amino)benzamide;
N-(5-(3,5-difluorobenzyl)-1H-indazol-3-yl)-4-(4-(1-((2-(2,6-dioxopiperidin-3-yl)-3-oxoisoindolin-5-yl)methyl)piperidin-3-yl)piperazin-1-yl)-2-((tetrahydro-2H-pyran-4-yl)amino)benzamide;
N-(5-(3,5-difluorobenzyl)-1H-indazol-3-yl)-4-(4-(1-((2-(2,6-dioxopiperidin-3-yl)-1,3-dioxoisoindolin-5-yl)methyl)piperidin-4-yl)piperazin-1-yl)-2-((tetrahydro-2H-pyran-4-yl)amino)benzamide;
N-(5-(3,5-difluorobenzyl)-1H-indazol-3-yl)-4-(4-(1-((2-(2,6-dioxopiperidin-3-yl)-1,3-dioxoisoindolin-5-yl)methyl)pyrrolidin-3-yl)piperazin-1-yl)-2-((tetrahydro-2H-pyran-4-yl)amino)benzamide;
N-(5-(3,5-difluorobenzyl)-1H-indazol-3-yl)-4-(4-(1-((2-(2,6-dioxopiperidin-3-yl)-1,3-dioxoisoindolin-5-yl)methyl)piperidin-3-yl)piperazin-1-yl)-2-((tetrahydro-2H-pyran-4-yl)amino)benzamide;
N-(5-(3,5-difluorobenzyl)-1H-indazol-3-yl)-4-(4-(1-((2-(2,6-dioxopiperidin-3-yl)-1,3-dioxoisoindolin-5-yl)methyl)azetidin-3-yl)piperazin-1-yl)-2-((tetrahydro-2H-pyran-4-yl)amino)benzamide;
2-(2,6-dioxopiperidin-3-yl)-5-((4-(6-(6-(2-(3-fluorophenyl)pyrrolidin-1-yl)imidazo[1,2-b]pyridazin-3-yl)pyridin-2-yl)piperazin-1-yl)methyl)isoindoline-1,3-dione; and
2-(2,6-dioxopiperidin-3-yl)-5-((3-(4-(6-(6-(2-(3-fluorophenyl)pyrrolidin-1-yl)imidazo[1,2-b]pyridazin-3 -yl)pyridin-2-yl)piperazin-1-yl)azetidin-1-yl)methyl)isoindoline-1,3 -dione.

13. The compound of Formula (II) or salts, enantiomers, stereoisomers, solvates, isotopically enriched analogs, prodrugs, or polymorphs thereof as claimed in claim 12, wherein the PBM-R_{f}- represents a small molecule ligand targeting epidermal growth factor receptor (EGFR), Cyclin-dependent kinase 4/6 (CDK4/6), anaplastic lymphoma kinase (ALK), activin receptor-like kinase 2 (ALK2), fibroblast growth factor receptors (FGFRs), proto-oncogene tyrosine-protein kinase receptor RET (RET), Focal adhesion kinase (FAK), breakpoint cluster region (BCR)-Abelson leukemia virus (ABL) (BCR-ABL) tyrosine kinase, Bruton tyrosine kinase (BTK), Androgen receptor (AR), Estrogen receptor (ER), Bromodomain and extra-terminal domain protein (BET), Interleukin-1 receptorassociated kinase 4 (IRAK4), Cyclin-dependent kinase 9 (CDK9), Histone-lysine N-methyltransferase EZH2 (EZH2), neurotrophic receptor tyrosine kinase (NTRK), Src homology 2 domain containing protein tyrosine phosphatase (SHP2), Poly (ADP-ribose) polymerase (PARP), signal transducers and activators of transcription 3 (STAT3), FMS-like tyrosine kinase 3 (FLT3), B cell lymphoma-2 (BCL-2) family proteins, SOS Ras/Rac guanine nucleotide exchange factor 1 (SOS1), or GTPase Kras (KRAS).

14. The compound of Formula (III) or salts, enantiomers, stereoisomers, solvates, isotopically enriched analogs, prodrugs, or polymorphs thereof as claimed in claim 12, wherein PBM comprises the structure of formula selected from: 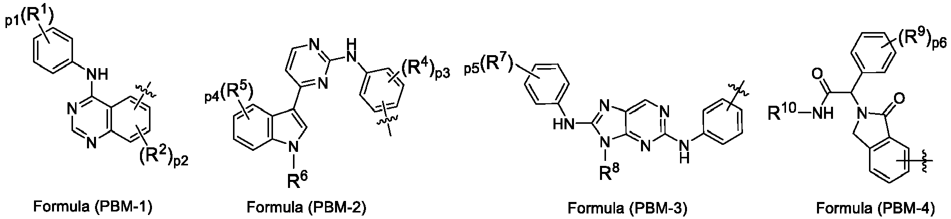 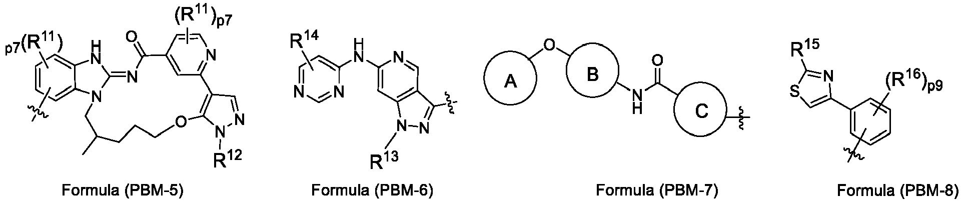 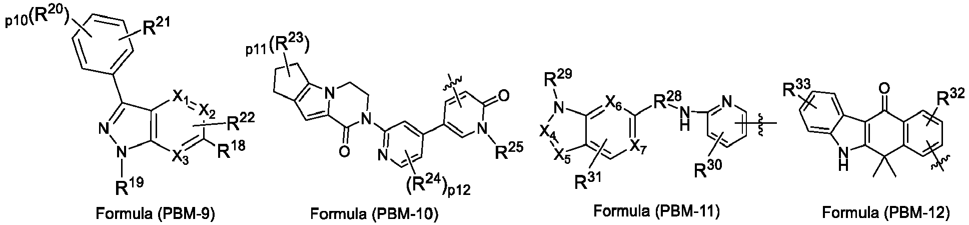 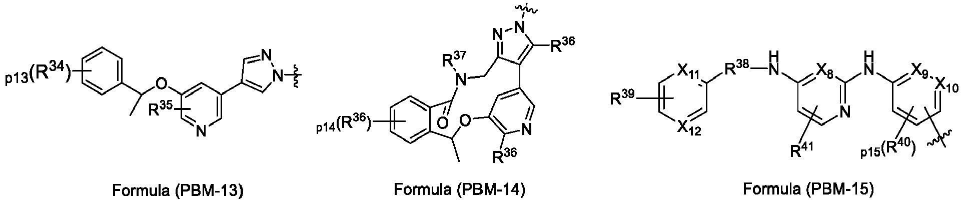 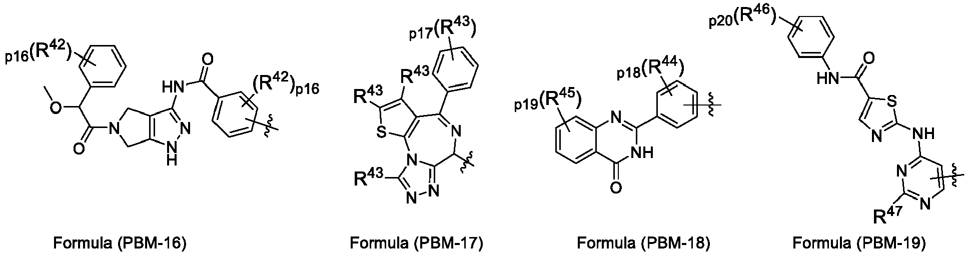 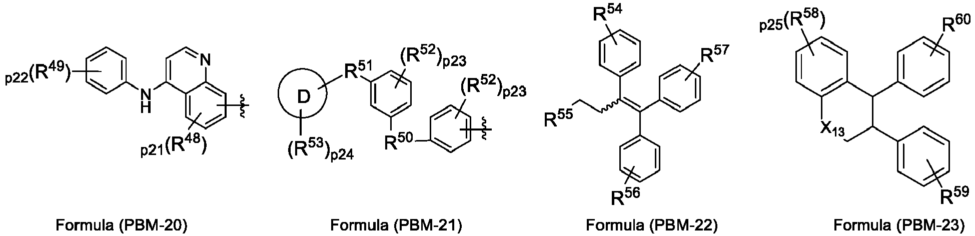 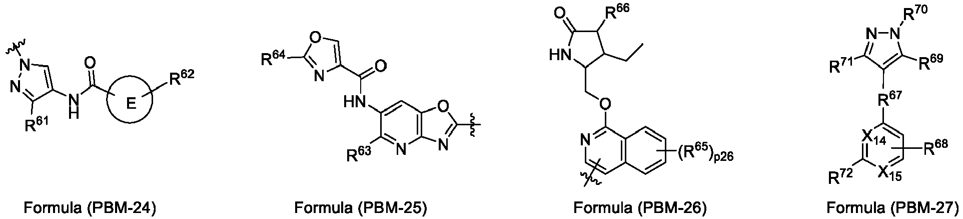 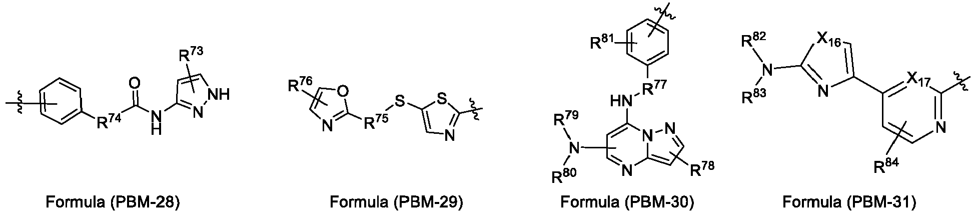 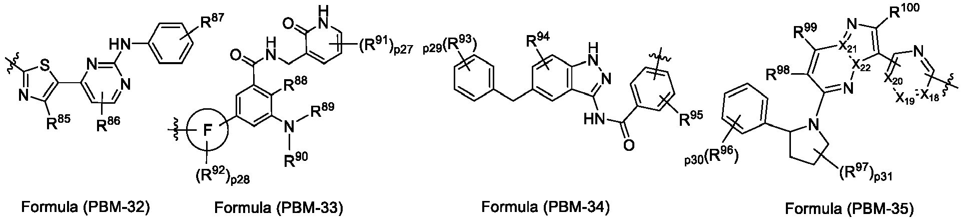 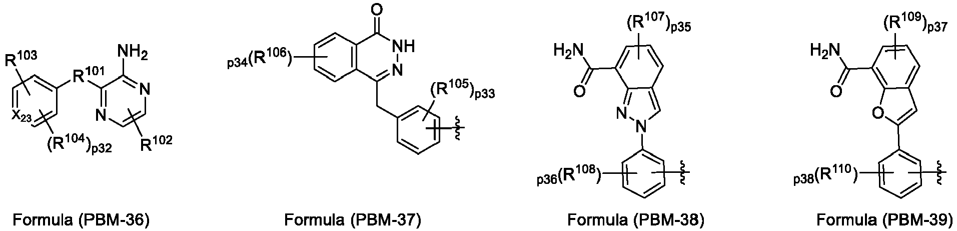 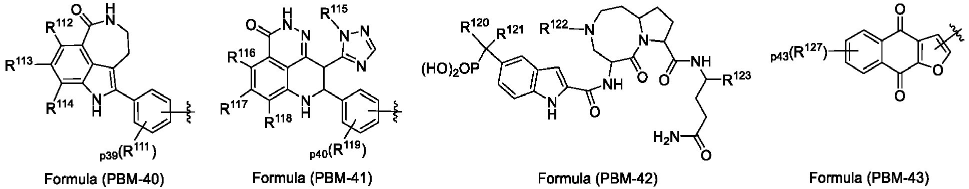 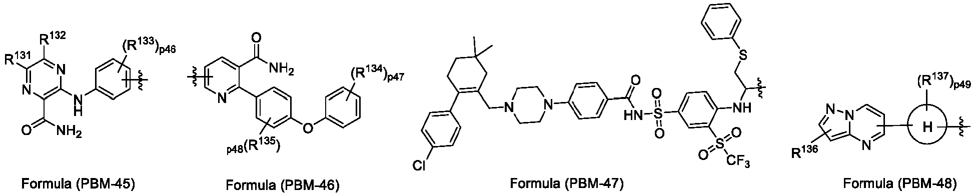 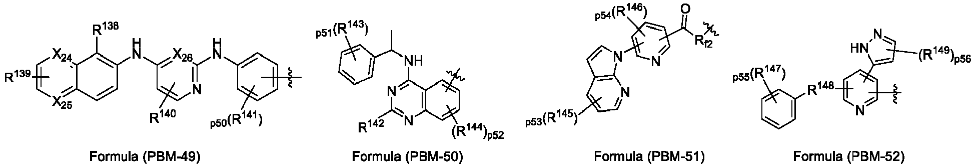 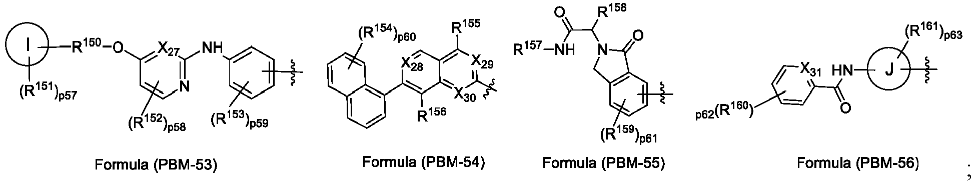 wherein
(R¹)ₚ₁ indicates that the benzene ring in Formula (PBM-1) is substituted with p1 R¹, with each R¹ being the same or different and each independently representing halogen, hydroxy, NH₂, NO₂, cyano, C₁₋₆ alkyl, halogenated C₁₋₆ alkyl, deuterated C₁₋₆ alkyl, C₁₋₆ alkoxy, or halogenated C₁₋₆ alkoxy;
(R²)ₚ₂ indicates that the quinazoline ring in Formula (PBM-1) is substituted with p2 R², with each R² being the same or different and each independently representing halogen, NO₂, cyano, halogenated C₁₋₆ alkyl, C₁₋₁₀ alkyl, deuterated C₁₋₁₀ alkyl, C₁₋₁₀ alkoxy, halogenated C₁₋₁₀ alkoxy, -O-optionally substituted heterocyclyl, or -NHC(O)R³, wherein R³ is C₁₋₁₀ alkyl, deuterated C₁₋₁₀ alkyl, or C₂₋₁₀ alkenyl, e.g., R² represents methyl, methoxy, 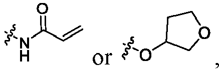 such as 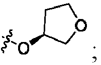
p1 represents an integer of 1, 2, 3, 4 or 5;
p2 represents an integer of 1, 2, or 3;
(R⁴)ₚ₃ indicates that the benzene ring in Formula (PBM-2) is substituted with p3 R⁴, with each R⁴ being the same or different and each independently representing halogen, NO₂, cyano, halogenated C₁₋₆ alkyl, C₁₋₁₀ alkyl, deuterated C₁₋₁₀ alkyl, C₁₋₁₀ alkoxy, or -NHC(O)R^{15a}, where R^{15a} is C₁₋₁₀ alkyl, deuterated C₁₋₁₀ alkyl, or C₂₋₁₀ alkenyl, e.g., R⁴ represents methyl, methoxy, NO₂, or 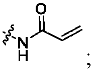
p3 represents an integer of 1, 2, 3 or 4;
(R⁵)ₚ₄ indicates that the 1H-indole ring in Formula (PBM-2) is substituted with p4 R⁵, with each R⁵ being the same or different and each independently representing C₁₋₁₀ alkyl or deuterated C₁₋₁₀ alkyl, and p4 represents an integer of 0, 1, 2 or 3;
R⁶ represents hydrogen, C₁₋₁₀ alkyl, or deuterated C₁₋₁₀ alkyl;
(R⁷)ₚ₅ indicates that the benzene ring in Formula (PBM-3) is substituted with p5 R⁷, with each R⁷ being the same or different and each independently representing halogen, hydroxy, C₁₋₆ alkyl, halogenated C₁₋₆ alkyl, deuterated C₁₋₆ alkyl, NO₂, NH₂, or cyano;
p5 represents an integer of 0, 1, 2, 3, 4 or 5;
R⁸ represents substituted or unsubstituted C₃₋₁₅ cycloalkyl, e.g., cyclopentyl;
(R⁹)ₚ₆ indicates that the benzene ring in Formula (PBM-4) is substituted with p6 R⁹, with each R⁹ being the same or different and each independently representing halogen, hydroxy, C₁₋₆ alkyl, halogenated C₁₋₆ alkyl, deuterated C₁₋₆ alkyl, NO₂, NH₂, C₁₋₆ alkoxy, halogenated C₁₋₆ alkoxy, or cyano;
p6 represents an integer of 2, 3, 4 or 5;
R¹⁰ represents C₆₋₁₀ aryl, or heteroaryl containing one or two 5- to 7-membered rings and 1 to 4 heteroatoms selected from nitrogen, oxygen, and sulfur, wherein the C₆₋₁₀ aryl and heteroaryl are each optionally substituted with one or more R^{b1}, with each R^{b1} being the same or different and independently representing halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
(R¹¹)ₚ₇ indicates that the benzene ring and the pyridine ring in Formula (PBM-5) are each optionally substituted with p7 R¹¹, with each p7 being the same or different and each independently representing an integer of 0, 1, 2 or 3, and each R¹¹ in Formula (PBM-5) is the same or different and each independently represents halogen, C₁₋₆ alkyl, halogenated C₁₋₆ alkyl, or deuterated C₁₋₆ alkyl;
R¹² represents hydrogen, C₁₋₆ alkyl, halogenated C₁₋₆ alkyl, or deuterated C₁₋₆ alkyl;
R¹³ in Formula (PBM-6) represents hydrogen, C₁₋₁₀ alkyl (e.g., 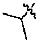 ), deuterated C₁₋₁₀ alkyl or halogenated C₁₋₁₀ alkyl;
R¹⁴ represents 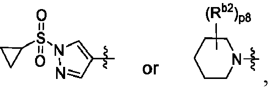 wherein (R^{b2})ₚ₈ indicates that the piperidine ring is optionally substituted with p8 R^{b2}, with each R^{b2} being the same or different and each independently representing deuterium, halogen, hydroxy, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, NO₂, NH₂, or cyano, and p8 represents an integer of 1, 2, 3, 4 or 5;
ring A in Formula (PBM-7) represents C₆₋₁₀ aryl or 5- to 20-membered monocyclic or bicyclic heteroaryl containing from 1 to 4 heteroatoms selected from the group consisting of oxygen, nitrogen, and sulfur, and the ring A is optionally substituted with one or more R^{b3}, with each R^{b3} being the same or different and each independently representing deuterium, halogen, hydroxy, cyano, amino, nitro, C₁₋₆ alkyl, halogenated C₁₋₆ alkyl, deuterated C₁₋₆ alkyl, C₁₋₆ alkoxy, halogenated C₁₋₆ alkoxy, C₂₋₆ alkenyl, or C₂₋₆ alkynyl;
ring B in Formula (PBM-7) represents C₃₋₁₅ cycloalkyl or 3- to 20-membered heterocyclyl containing from 1 to 4 heteroatoms selected from the group consisting of oxygen, nitrogen, and sulfur, and the ring B is optionally substituted with one or more R^{b4}, with each R^{b4} being the same or different and each independently representing deuterium, halogen, C₁₋₆ alkyl, halogenated C₁₋₆ alkyl, deuterated C₁₋₆ alkyl, C₁₋₆ alkoxy, or halogenated C₁₋₆ alkoxy;
ring C in Formula (PBM-7) represents 5- to 20-membered monocyclic or bicyclic heteroaryl containing from 1 to 4 heteroatoms selected from the group consisting of oxygen, nitrogen, and sulfur, and the ring C is optionally substituted with one or more R^{b5}, with each R^{b5} being the same or different and each independently representing deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
R¹⁵ in Formula (PBM-8) represents 4- to 20-membered heterocyclyl containing from 1 to 4 heteroatoms selected from the group consisting of oxygen, nitrogen, and sulfur, and the heterocyclyl is optionally substituted with one or more R^{b6}, with each R^{b6} being the same or different and each independently representing halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
(R¹⁶)ₚ₉ indicates that the benzene ring in Formula (PBM-8) is substituted with p9 R¹⁶, with each R¹⁶ being the same or different and each independently representing halogen, C₁₋₆ alkyl, halogenated C₁₋₆ alkyl, or deuterated C₁₋₆ alkyl, and p9 represents an integer of 1, 2, 3 or 4;
X₁ in Formula (PBM-9) represents N or CR^{b7}, wherein R^{b7} is hydrogen or amino, and X₂ represents N or CR¹⁷, wherein R¹⁷ is hydrogen or represents a bond, and X₃ represents CH or N;
R¹⁸ and R¹⁹ each independently represent hydrogen or a bond;
(R²⁰)ₚ₁₀ indicates that the benzene ring in Formula (PBM-9) is substituted with p10 R²⁰, with each R²⁰ being the same or different and each independently representing -O-aryl, -O-heteroaryl, -C₁₋₃ alkylene-NHC(O)-heteroaryl, -C₁₋₃ alkylene-C(O)NH-heteroaryl or halogen, wherein the aryl and heteroaryl are each independently optionally substituted with one or more R^{b8}, with each R^{b8} being the same or different and each independently representing deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
p10 represents an integer of 1, 2, 3 or 4;
R²¹ represents a bond, deuterium, hydrogen, halogen, C₁₋₆ alkyl, halogenated C₁₋₆ alkyl, deuterated C₁₋₆ alkyl, or C₁₋₆ alkoxy;
R²² represents hydrogen, halogen, cyano, or amino;
wherein R¹⁷, R¹⁸, R¹⁹, and R²¹ are not simultaneously hydrogen, and R¹⁷, R¹⁸, R¹⁹, and R²¹ do not simultaneously represent a bond, and only one of R¹⁷, R¹⁸, R¹⁹, and R²¹ represents a bond, wherein
when R¹⁸ represents a bond, the carbon atom on the ring connected to R¹⁸ is directly connected to R_{f}, X₂ represents CH or N, and R¹⁹ is hydrogen, and R²¹ is not a bond;
when X₂ represents CR¹⁷, where R¹⁷ represents a bond, the carbon atom on the ring connected to R¹⁷ is directly connected to R_{f}, and R¹⁸ and R¹⁹ are hydrogen, and R²¹ is not a bond;
when R¹⁹ represents a bond, the nitrogen atom on the ring connected to R¹⁹ is directly connected to R_{f}, X₂ represents N or CH, and R¹⁸ is hydrogen, and R²¹ is not a bond; and
when R²¹ represents a bond, the carbon atom on the ring connected to R²¹ is directly connected to R_{f}, and X₂ represents N or CH, and R¹⁸ and R¹⁹ are hydrogen;
(R²³)ₚ₁₁ indicates that the cyclopentene ring in Formula (PBM-10) is substituted with p11 R²³, with each R²³ being the same or different and each independently representing halogen, C₁₋₆ alkyl, halogenated C₁₋₆ alkyl, or deuterated C₁₋₆ alkyl, and p11 represents an integer of 1, 2, 3, or 4;
(R²⁴)ₚ₁₂ indicates that the pyridine ring in Formula (PBM-10) is substituted with p12 R²⁴, with each R²⁴ being the same or different and each independently representing halogen, -C₁₋₃ alkylene -OH, C₁₋₆ alkyl, halogenated C₁₋₆ alkyl, or deuterated C₁₋₆ alkyl, and p12 represents an integer of 1, 2, or 3;
R²⁵ represents hydrogen, C₁₋₆ alkyl, halogenated C₁₋₆ alkyl, or deuterated C₁₋₆ alkyl;
X₄ in Formula (PBM-11) represents CR²⁶ or a fragment 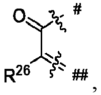 wherein symbol # indicates the point of attachment to N atom adjacent to X₄, symbol ## indicates the point of attachment to X₅, and R²⁶ represents deuterium, hydrogen, halogen, C₁₋₆ alkyl, halogenated C₁₋₆ alkyl, deuterated C₁₋₆ alkyl, - C(O)-C₁₋₆ alkyl, -C(O)-deuterated C₁₋₆ alkyl, or -C(O)NR^{b9}R^{b10}, where R^{b9} and R^{b10} are the same or different and each independently represent hydrogen, C₁₋₆ alkyl or deuterated C₁₋₆ alkyl;
X₅ in Formula (PBM-11) represents N or CR²⁷, where R²⁷ represents hydrogen, deuterium, halogen, C₁₋₆ alkyl, deuterated C₁₋₆ alkyl, or halogenated C₁₋₆ alkyl;
X₆ and X₇ each independently represent CH or N;
R²⁸ represents a bond or optionally substituted heteroarylene (e.g., halogenated heteroarylene);
R²⁹ represents hydrogen, C₁₋₆ alkyl, halogenated C₁₋₆ alkyl, deuterated C₁₋₆ alkyl, or optionally substituted C₃₋₇ cycloalkyl;
R³⁰ and R³¹ are the same or different and each independently represent hydrogen, halogen, C₁₋₆ alkyl, halogenated C₁₋₆ alkyl, or deuterated C₁₋₆ alkyl;
R³² in Formula (PBM-12) represents hydrogen, halogen, C₁₋₆ alkyl, halogenated C₁₋₆ alkyl, or deuterated C₁₋₆ alkyl;
R³³ represents hydrogen, cyano, halogen, C₁₋₆ alkyl, halogenated C₁₋₆ alkyl, or deuterated C₁₋₆ alkyl;
(R³⁴)ₚ₁₃ indicates that the benzene ring in Formula (PBM-13) is substituted with p13 R³⁴, with each R³⁴ being the same or different and independently representing halogen, hydroxy, amino, cyano, C₁₋₆ alkyl, halogenated C₁₋₆ alkyl, or deuterated C₁₋₆ alkyl;
p13 represents an integer of 1, 2, 3, 4 or 5;
R³⁵ represents NR^{b11}R^{b12}, where R^{b11} and R^{b12} are the same or different and independently represent hydrogen, C₁₋₆ alkyl, halogenated C₁₋₆ alkyl, or deuterated C₁₋₆ alkyl;
(R³⁶)ₚ₁₄ indicates that the benzene ring in Formula (PBM-14) is optionally substituted with p14 R³⁶, with each R³⁶ in Formula (PBM-14) being the same or different and each independently representing halogen, amino, cyano, C₁₋₆ alkyl, halogenated C₁₋₆ alkyl, or deuterated C₁₋₆ alkyl, and p14 represents an integer of 1, 2 or 3;
R³⁷ represents hydrogen, C₁₋₆ alkyl, halogenated C₁₋₆ alkyl, or deuterated C₁₋₆ alkyl;
X₈, X₉, X₁₀, X₁₁ and X₁₂ in Formula (PBM-15) are the same or different and each independently represent N or CH;
R³⁸ represents a bond or optionally substituted methylene (e.g., halogenated methylene);
R³⁹ represents -C(O)NHR^{b13}, -NHC(O)-R^{b13}, -S(O)₂NHR^{b13}, -N(R^{b14})S(O)₂Rb¹³, -S(O)₂R^{b13} or - P(O)(R^{b13})₂, wherein each R^{b13} is the same or different and each independently represents C₁₋₆ alkyl or deuterated C₁₋₆ alkyl, and R^{b14} represents hydrogen or C₁₋₆ alkyl;
(R⁴⁰)ₚ₁₅ indicates that the 6-membered ring containing X₉ and X₁₀ in Formula (PBM-15) is optionally substituted with p15 R⁴⁰, with each R⁴⁰ being the same or different and independently representing halogen, C₁₋₆ alkoxy, C₁₋₆ alkyl, deuterated C₁₋₆ alkyl or halogenated C₁₋₆ alkyl;
p15 represents an integer of 0, 1, 2, 3 or 4;
R⁴¹ represents halogen, C₁₋₆ alkoxy, C₁₋₆ alkyl, deuterated C₁₋₆ alkyl or halogenated C₁₋₆ alkyl;
(R⁴²)ₚ₁₆ indicates that the benzene ring in Formula (PBM-16) is optionally substituted with p16 R⁴², with each R⁴² being the same or different and independently representing halogen, C₁₋₆ alkoxy, C₁₋₆ alkyl, deuterated C₁₋₆ alkyl or halogenated C₁₋₆ alkyl;
each p16 is the same or different and each independently represents an integer of 0, 1, 2, 3 or 4;
(R⁴³)ₚ₁₇ indicates that the benzene ring in Formula (PBM-17) is substituted with p17 R⁴³, with each R⁴³ being the same or different and each independently representing halogen, C₁₋₆ alkoxy, C₁₋₆ alkyl, deuterated C₁₋₆ alkyl or halogenated C₁₋₆ alkyl, and p17 represents an integer of 0, 1, 2, 3 or 4;
(R⁴⁴)ₚ₁₈ indicates that the benzene ring in Formula (PBM-18) is optionally substituted with p18 R⁴⁴, with each R⁴⁴ being the same or different and each independently representing halogen, C₁₋₆ alkoxy, C₁₋₆ alkyl, deuterated C₁₋₆ alkyl or halogenated C₁₋₆ alkyl;
p18 represents an integer of 0, 1, 2, 3 or 4;
(R⁴⁵)ₚ₁₉ indicates that the 4-oxo-3,4-dihydroquinazoline ring in Formula (PBM-18) is substituted with p19 R⁴⁵, with each R⁴⁵ being the same or different and each independently representing halogen, C₁₋₆ alkoxy, C₁₋₆ alkyl, deuterated C₁₋₆ alkyl or halogenated C₁₋₆ alkyl;
p19 represents an integer of 1, 2, 3 or 4;
(R⁴⁶)ₚ₂₀ indicates that the benzene ring in Formula (PBM-19) is substituted with p20 R⁴⁶, with each R⁴⁶ being the same or different and each independently representing halogen, C₁₋₆ alkoxy, C₁₋₆ alkyl, deuterated C₁₋₆ alkyl, or halogenated C₁₋₆ alkyl;
p20 represents an integer of 1, 2, 3, 4 or 5;
R⁴⁷ represents halogen, C₁₋₆ alkyl, deuterated C₁₋₆ alkyl, or halogenated C₁₋₆ alkyl;
(R⁴⁸)ₚ₂₁ indicates that the quinoline ring in Formula (PBM-20) is substituted with p21 R⁴⁸, with each R⁴⁸ being the same or different and each independently representing halogen, cyano, C₁₋₆ alkoxy, C₁₋₆ alkyl, deuterated C₁₋₆ alkyl, or halogenated C₁₋₆ alkyl;
p21 represents an integer of 1, 2, 3 or 4;
(R⁴⁹)ₚ₂₂ indicates that the benzene ring in Formula (PBM-20) is substituted with p22 R⁴⁹, with each R⁴⁹ being the same or different and each independently representing halogen, C₁₋₆ alkoxy, C₁₋₆ alkyl, deuterated C₁₋₆ alkyl, or halogenated C₁₋₆ alkyl;
p22 represents an integer of 2, 3 or 4;
R⁵⁰ in Formula (PBM-21) represents -NHC(O)- or -C(O)NH-;
R⁵¹ represents -NH- or ethynylene;
(R⁵²)ₚ₂₃ indicates that the benzene rings in Formula (PBM-21) are each optionally substituted with p23 R⁵², with each R⁵² being the same or different and each independently representing halogen, C₁₋₆ alkoxy, C₁₋₆ alkyl, deuterated C₁₋₆ alkyl, or halogenated C₁₋₆ alkyl;
each p23 is the same or different and each independently represents an integer of 0, 1, 2, 3 or 4;
ring D in Formula (PBM-21) represents 5- to 20-membered monocyclic or bicyclic heteroaryl containing from 1 to 4 heteroatoms selected from the group consisting of oxygen, nitrogen, and sulfur, and (R⁵³)ₚ₂₄ indicates that the ring D is optionally substituted with p24 R⁵³, with each R⁵³ being the same or different and each independently representing optionally substituted 5- to 15-membered heteroaryl, deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
p24 represents an integer of 0, 1, 2, or 3;
only one of R⁵⁴, R⁵⁵, R⁵⁶, and R⁵⁷ in Formula (PBM-22) represents a bond, and the remaining are the same or different and each independently represent hydrogen, halogen, or hydroxy, wherein when one of R⁵⁴, R⁵⁵, R⁵⁶ and R⁵⁷ represents a bond, the benzene ring or methylene in Formula (PBM-22) connected to the bond is directly connected to R_{f};
symbol " 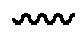 " connected to double bond in Formula (PBM-22) represents a bond in stereo-configuration (cis or trans configuration, or E- or Z-configuration);
X₁₃ in Formula (PBM-23) represents -O- or -CH₂-;
(R⁵⁸)ₚ₂₅ indicates that 1,2,3,4-tetrahydronaphthalene ring in Formula (PBM-23) is substituted with p25 R⁵⁸, with each R⁵⁸ being the same or different and each independently representing hydrogen, hydroxy, halogen, C₁₋₆ alkyl, halogenated C₁₋₆ alkyl, or deuterated C₁₋₆ alkyl;
p25 represents an integer of 1, 2, 3 or 4;
one of R⁵⁹ and R⁶⁰ represents a bond, and another represents hydrogen, halogen, C₁₋₆ alkyl, deuterated C₁₋₆ alkyl or halogenated C₁₋₆ alkyl, wherein when one of R⁵⁹ and R⁶⁰ represents a bond, the carbon atom on the ring in Formula (PBM-23) connected to the bond is directly connected to R_{f};
ring E in Formula (PBM-24) represents 5- to 20-membered monocyclic or bicyclic heteroarylene containing from 1 to 4 heteroatoms selected from the group consisting of oxygen, nitrogen, and sulfur;
R⁶² represents optionally substituted 5- to 15-membered heteroaryl, optionally substituted 5- to 15-membered heterocyclyl, deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
R⁶¹ represents halogen, C₁₋₆ alkyl, halogenated C₁₋₆ alkyl, or deuterated C₁₋₆ alkyl;
R⁶³ in Formula (PBM-25) represents optionally substituted 5- to 15-membered heterocyclyl, deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
R⁶⁴ represents 5- to 20-membered monocyclic or bicyclic heteroaryl containing from 1 to 4 heteroatoms selected from the group consisting of oxygen, nitrogen, and sulfur, and the heteroaryl is optionally substituted with one or more substituents selected from the group consisting of deuterium, halogen, hydroxy, cyano, amino, C₁₋₆ alkyl, halogenated C₁₋₆ alkyl, deuterated C₁₋₆ alkyl, C₁₋₆ alkoxy, halogenated C₁₋₆ alkoxy, or any combination thereof;
(R⁶⁵)ₚ₂₆ indicates that the isoquinoline ring in Formula (PBM-26) is substituted with p26 R⁶⁵, with each R⁶⁵ being the same or different and each independently representing hydrogen, hydroxy, halogen, C₁₋₆ alkyl, C₁₋₆ alkoxy, -C(O)NH₂, deuterated C₁₋₆ alkyl, or halogenated C₁₋₆ alkyl;
p26 represents an integer of 1, 2, 3 or 4;
groups R⁶⁶ are the same or different and each independently represent hydrogen, hydroxy, halogen, C₁₋₆ alkyl, C₁₋₆ alkoxy, deuterated C₁₋₆ alkyl, or halogenated C₁₋₆ alkyl;
X₁₄ in Formula (PBM-27) represents N or CR^{b23}, where R^{b23} represents hydrogen or halogen, and X₁₅ represents N or CH;
R⁶⁷ represents a bond, -C(O)NH- or -NHC(O)-;
R⁶⁸ represents halogen, C₁₋₆ alkyl, deuterated C₁₋₆ alkyl, or halogenated C₁₋₆ alkyl;
R⁶⁹ represents hydrogen, C₁₋₆ alkyl, deuterated C₁₋₆ alkyl, halogenated C₁₋₆ alkyl, or -C₁₋₃ alkylene-C₃₋₆ cycloalkyl;
R⁷⁰ represents hydrogen, C₁₋₆ alkyl, deuterated C₁₋₆ alkyl, or halogenated C₁₋₆ alkyl; or
R⁶⁹ and R⁷⁰ together with the corresponding nitrogen and carbon atoms to which they are connected form an optionally substituted 5- to 7-membered nitrogen-containing heterocycle;
R⁷¹ represents hydrogen or a bond, R⁷² represents hydrogen or a bond, wherein R⁷¹ and R⁷² are not simultaneously hydrogen, and only one of R⁷¹ and R⁷² represents a bond, and another represents hydrogen, wherein when R⁷¹ represents a bond, the carbon atom on the ring connected to R⁷¹ is directly connected to R_{f}, and when R⁷² represents a bond, the carbon atom on the ring connected to R⁷² is directly connected to R_{f};
R⁷⁴ in Formula (PBM-28) represents a bond, C₁₋₃ alkylene or halogenated C₁₋₃ alkylene;
R⁷³ represents optionally substituted C₃₋₆cycloalkyl, halogenated C₃₋₆ cycloalkyl, or optionally substituted C₁₋₆ alkyl;
R⁷⁵ in Formula (PBM-29) represents C₁₋₃ alkylene or halogenated C₁₋₃ alkylene;
R⁷⁶ represents C₁₋₆ alkyl, halogenated C₁₋₆ alkyl, deuterated C₁₋₆ alkyl, or optionally substituted C₃₋₆ cycloalkyl;
R⁷⁷ in Formula (PBM-30) represents C₁₋₃ alkylene or halogenated C₁₋₃ alkylene;
R⁷⁸ represents hydroxy, halogen, C₁₋₆ alkyl, C₁₋₆ alkoxy, deuterated C₁₋₆ alkyl, or halogenated C₁₋₆ alkyl;
R⁷⁹ and R⁸⁰ are the same or different and each independently represent hydrogen, optionally substituted C₁₋₁₀ alkyl or optionally substituted C₃₋₆ cycloalkyl;
R⁸¹ represents hydrogen, halogen, C₁₋₆ alkyl, deuterated C₁₋₆ alkyl, or halogenated C₁₋₆ alkyl;
X₁₆ in Formula (PBM-31) represents -S- or a fragment 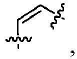 wherein when X₁₆ represents -S-, heteroaryl containing X₁₆ and nitrogen atom in Formula (PBM-31) is thiazolyl, and when X₁₆ represents the fragment 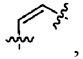 the heteroaryl containing X₁₆ and nitrogen atom in Formula (PBM-31) is pyridyl;
X₁₇ represents N or CH;
R⁸² represents hydrogen or optionally substituted C₁₋₁₀ alkyl;
R⁸³ represents hydrogen, -C₁₋₃ alkylene-optionally substituted 4- to 10-membered heterocyclyl, or optionally substituted 4- to 10-membered heterocyclyl;
R⁸⁴ represents hydroxy, halogen, C₁₋₆ alkyl, C₁₋₆ alkoxy, deuterated C₁₋₆ alkyl, or halogenated C₁₋₆ alkyl;
R⁸⁵ and R⁸⁶ in Formula (PBM-32) are the same or different and each independently represent hydroxy, halogen, C₁₋₆ alkyl, C₁₋₆ alkoxy, deuterated C₁₋₆ alkyl, or halogenated C₁₋₆ alkyl;
R⁸⁷ represents -S(O)₂NH₂ or -C(O)NH₂;
R⁸⁸ in Formula (PBM-33) represents hydrogen, hydroxy, halogen, C₁₋₆ alkyl, deuterated C₁₋₆ alkyl, or halogenated C₁₋₆ alkyl;
R⁸⁹ represents C₁₋₆ alkyl, deuterated C₁₋₆ alkyl, or halogenated C₁₋₆ alkyl; or
R⁸⁸ and R⁸⁹ together with the corresponding carbon and nitrogen atoms to which they are connected form an optionally substituted 4- to 6-membered heteroaromatic ring or optionally substituted 4- to 6-membered heterocycle;
R⁹⁰ represents optionally substituted C₃₋₆ cycloalkyl, C₁₋₆ alkyl, deuterated C₁₋₆ alkyl, halogenated C₁₋₆ alkyl or optionally substituted 4- to 10-membered heterocyclyl;
(R⁹¹)ₚ₂₇ incicates that pyridin-2(1H)-one ring in Formula (PBM-33) is substituted with p27 R⁹¹, with each R⁹¹ being the same or different and each independently representing hydroxy, halogen, C₁₋₆ alkyl, C₁₋₆ alkoxy, deuterated C₁₋₆ alkyl, or halogenated C₁₋₆ alkyl;
p27 represents an integer of 1, 2 or 3;
ring F in Formula (PBM-33) represents arylene or 5- to 20-membered monocyclic or bicyclic heteroarylene containing from 1 to 4 heteroatoms selected from the group consisting of oxygen, nitrogen, and sulfur; (R⁹²)ₚ₂₈ incicates that the ring F is optionally substituted with p28 R⁹², with each R⁹² being the same or different and each independently representing deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
p28 represents an integer of 0, 1, 2, 3 or 4;
(R⁹³)ₚ₂₉ indicates that the benzene ring in Formula (PBM-34) is substituted with p29 R⁹³, with each R⁹³ being the same or different and each independently representing hydroxy, amino, cyano, halogen, C₁₋₆ alkyl, C₁₋₆ alkoxy, deuterated C₁₋₆ alkyl, or halogenated C₁₋₆ alkyl;
p29 represents an integer of 1, 2, 3, 4 or 5;
R⁹⁴ represents hydrogen, hydroxy, amino, cyano, halogen, C₁₋₆ alkyl, C₁₋₆ alkoxy, deuterated C₁₋₆ alkyl, or halogenated C₁₋₆ alkyl;
R⁹⁵ represents NR^{b15}R^{b16}, where R^{b15} and R^{b16} are the same or different and each independently represent hydrogen, C₁₋₆ alkyl, deuterated C₁₋₆ alkyl, halogenated C₁₋₆ alkyl, optionally substituted C₃₋₆ cycloalkyl, or optionally substituted 4- to 8-membered heterocyclyl ;
X₁₈, X₁₉ and X₂₀ in Formula (PBM-35) are the same or different and each independently represent CH or N, wherein X₁₈, X₁₉ and X₂₀ are not simultaneously N, and the 6-membered ring containing X₁₈, X₁₉, X₂₀ and nitrogen atom is optionally substituted with 1 or 2 substituents selected from the group consisting of halogen, cyano, C₁₋₆ alkyl, deuterated C₁₋₆ alkyl, halogenated C₁₋₆ alkyl, or any combination thereof;
X₂₁ and X₂₂ in Formula (PBM-35) are the same or different and each independently represent C or N, wherein X₂₁ and X₂₂ are not simultaneously N;
(R⁹⁶)ₚ₃₀ indicates that the benzene ring in Formula (PBM-35) is substituted with p30 R⁹⁶, with each R⁹⁶ being the same or different and each independently representing hydroxy, amino, cyano, halogen, C₁₋₆ alkyl, C₁₋₆ alkoxy, deuterated C₁₋₆ alkyl, or halogenated C₁₋₆ alkyl;
p30 represents an integer of 1, 2, 3, 4 or 5;
(R⁹⁷)ₚ₃₁ indicates that the pyrrolidine ring in Formula (PBM-35) is optionally substituted with p31 R⁹⁷, with each R⁹⁷ being the same or different and each independently representing hydrogen, hydroxy, amino, cyano, halogen, C₁₋₆ alkyl, C₁₋₆ alkoxy, deuterated C₁₋₆ alkyl, or halogenated C₁₋₆ alkyl;
p31 represents an integer of 0, 1, 2, 3, 4, 5 or 6;
R⁹⁸, R⁹⁹, and R¹⁰⁰ are the same or different and each independently represent hydrogen, halogen, C₁₋₆ alkyl, deuterated C₁₋₆ alkyl, or halogenated C₁₋₆ alkyl;
X₂₃ in Formula (PBM-36) represents CH or N;
R¹⁰¹ represents a bond or -S-;
R¹⁰² represents a bond or optionally substituted 4- to 15-membered nitrogen-containing heterocyclyl;
R¹⁰³ represents a bond, halogen, amino, C₁₋₆ alkyl, deuterated C₁₋₆ alkyl, halogenated C₁₋₆ alkyl, or optionally substituted 4- to 15-membered nitrogen-containing heterocyclyl;
wherein R¹⁰² and R¹⁰³ are not simultaneously a bond, and only one of R¹⁰² and R¹⁰³ is a bond, wherein when R¹⁰² represents a bond, the carbon atom on the ring connected to R¹⁰² is directly connected to R_{f}, and when R¹⁰³ represents a bond, the carbon atom on the ring connected to R¹⁰³ is directly connected to R_{f};
(R¹⁰⁴)ₚ₃₂ indicates that the 6-membered ring in Formula (PBM-36) is optionally substituted with p32 R¹⁰⁴, with each R¹⁰⁴ being the same or different and each independently representing hydroxy, amino, cyano, halogen, C₁₋₆ alkyl, C₁₋₆ alkoxy, deuterated C₁₋₆ alkyl, or halogenated C₁₋₆ alkyl;
p32 represents an integer of 0, 1, 2, 3 or 4;
(R¹⁰⁵)ₚ₃₃ indicates that the benzene ring in Formula (PBM-37) is optionally substituted with p33 R¹⁰⁵, with each R¹⁰⁵ being the same or different and each independently representing hydroxy, amino, cyano, halogen, C₁₋₆ alkyl, C₁₋₆ alkoxy, deuterated C₁₋₆ alkyl, or halogenated C₁₋₆ alkyl;
p33 represents an integer of 1, 2, 3 or 4;
(R¹⁰⁶)ₚ₃₄ indicates that the phthalazinone ring in Formula (PBM-37) is optionally substituted with p34 R¹⁰⁶, with each R¹⁰⁶ being the same or different and each independently representing hydroxy, amino, cyano, halogen, C₁₋₆ alkyl, C₁₋₆ alkoxy, deuterated C₁₋₆ alkyl or halogenated C₁₋₆ alkyl;
p34 represents an integer of 0, 1, 2, 3 or 4;
(R¹⁰⁷)ₚ₃₅ indicates that the 2H-indazole ring in Formula (PBM-38) is optionally substituted with p35 R¹⁰⁷, with each R¹⁰⁷ being the same or different and each independently representing hydroxy, amino, cyano, halogen, C₁₋₆ alkyl, C₁₋₆ alkoxy, deuterated C₁₋₆ alkyl or halogenated C₁₋₆ alkyl;
p35 represents an integer of 0, 1, 2 or 3;
(R¹⁰⁸)ₚ₃₆ indicates that the benzene ring in Formula (PBM-38) is optionally substituted with p36 R¹⁰⁸, with each R¹⁰⁸ being the same or different and each independently representing hydroxy, amino, cyano, halogen, C₁₋₆ alkyl, C₁₋₆ alkoxy, deuterated C₁₋₆ alkyl or halogenated C₁₋₆ alkyl;
p36 represents an integer of 0, 1, 2 or 3;
(R¹⁰⁹)ₚ₃₇ indicates that the 2H-indazole ring in Formula (PBM-39) is optionally substituted with p37 R¹⁰⁹, with each R¹⁰⁹ being the same or different and each independently representing hydroxy, amino, cyano, halogen, C₁₋₆ alkyl, C₁₋₆ alkoxy, deuterated C₁₋₆ alkyl or halogenated C₁₋₆ alkyl;
p37 represents an integer of 0, 1, 2 or 3;
(R¹¹⁰)ₚ₃₈ indicates that the benzene ring in Formula (PBM-39) is optionally substituted with p38 R¹¹⁰, with each R¹¹⁰ being the same or different and each independently representing hydroxy, amino, cyano, halogen, C₁₋₆ alkyl, C₁₋₆ alkoxy, deuterated C₁₋₆ alkyl or halogenated C₁₋₆ alkyl;
p38 represents an integer of 0, 1, 2 or 3;
(R¹¹¹)ₚ₃₉ indicates that the benzene ring in Formula (PBM-40) is optionally substituted with p39 R¹¹¹, with each R¹¹¹ being the same or different and each independently representing hydroxy, amino, cyano, halogen, C₁₋₆ alkyl, C₁₋₆ alkoxy, deuterated C₁₋₆ alkyl or halogenated C₁₋₆ alkyl;
p39 represents an integer of 0, 1, 2, 3 or 4;
R¹¹², R¹¹³, and R¹¹⁴ are the same or different and each independently represent hydrogen, halogen, C₁₋₆ alkyl, deuterated C₁₋₆ alkyl or halogenated C₁₋₆ alkyl;
R¹¹⁵ in Formula (PBM-41) represents hydrogen, C₁₋₆ alkyl, deuterated C₁₋₆ alkyl or halogenated C₁₋₆ alkyl;
R¹¹⁶, R¹¹⁷, and R¹¹⁸ are the same or different and each independently represent hydrogen, halogen, C₁₋₆ alkyl, deuterated C₁₋₆ alkyl or halogenated C₁₋₆ alkyl;
(R¹¹⁹)ₚ₄₀ indicates that the benzene ring in Formula (PBM-41) is optionally substituted with p40 R¹¹⁹, with each R¹¹⁹ being the same or different and each independently representing hydroxy, amino, cyano, halogen, C₁₋₆ alkyl, C₁₋₆ alkoxy, deuterated C₁₋₆ alkyl or halogenated C₁₋₆ alkyl;
p40 represents an integer of 0, 1, 2, 3 or 4;
R¹²⁰ and R¹²¹ in Formula (PBM-42) are the same or different and each independently represent hydrogen, halogen, C₁₋₃ alkyl or halogenated C₁₋₃ alkyl;
R¹²² represents a bond, hydrogen, C₁₋₆ alkyl, deuterated C₁₋₆ alkyl or halogenated C₁₋₆ alkyl;
R¹²³ represents the structure of the following formula: 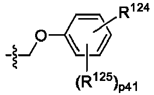
wherein R¹²⁴ represents a bond or hydrogen; (R¹²⁵)ₚ₄₁ indicates that the benzene ring is optionally substituted with p41 R¹²⁵, with each R¹²⁵ being the same or different and each independently representing hydroxy, amino, cyano, halogen, C₁₋₆ alkyl, C₁₋₆ alkoxy, deuterated C₁₋₆ alkyl or halogenated C₁₋₆ alkyl, and p41 represents an integer of 0, 1, 2, 3 or 4; or
R¹²³ represents the structure of the following formula: 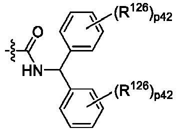
wherein (R¹²⁶)ₚ₄₂ indicates that the benzene ring is optionally substituted with p42 R¹²⁶, with each R¹²⁶ being the same or different and each independently representing hydroxy, amino, cyano, halogen, C₁₋₆ alkyl, C₁₋₆ alkoxy, deuterated C₁₋₆ alkyl or halogenated C₁₋₆ alkyl, and p42 represents an integer of 0, 1, 2, 3, 4 or 5;
wherein R¹²² and R¹²⁴ are not simultaneously a bond, and only one of R¹²² and R¹²⁴ represents a bond, wherein when R¹²² represents a bond, the nitrogen atom on the ring connected to R¹²² is directly connected to R_{f}, and when R¹²⁴ represents a bond, the carbon atom on the ring connected to R¹²⁴ is directly connected to R_{f};
(R¹²⁷)ₚ₄₃ indicates that the benzene ring in Formula (PBM-43) is optionally substituted with p43 R¹²⁷, with each R¹²⁷ being the same or different and each independently representing hydroxy, amino, cyano, halogen, C₁₋₆ alkyl, C₁₋₆ alkoxy, deuterated C₁₋₆ alkyl or halogenated C₁₋₆ alkyl;
p43 represents an integer of 0, 1, 2, 3 or 4;
R¹³¹ in Formula (PBM-45) represents C₁₋₆ alkyl, deuterated C₁₋₆ alkyl, halogenated C₁₋₆ alkyl or hydrogen;
R¹³² represents C₁₋₆ alkyl, deuterated C₁₋₆ alkyl, halogenated C₁₋₆ alkyl, optionally substituted C₃₋₆ cycloalkyl, optionally substituted 4- to 8-membered nitrogen-containing heterocyclyl or NR^{b17}R^{b18}, where R^{b17} and R^{b18} are the same or different and each independently represent hydrogen, C₁₋₆ alkyl, deuterated C₁₋₆ alkyl or optionally substituted 4- to 8-membered heterocyclyl;
(R¹³³)ₚ₄₆ indicates that the benzene ring in Formula (PBM-45) is optionally substituted with p46 R¹³³, with each R¹³³ being the same or different and each independently representing hydroxy, amino, cyano, halogen, C₁₋₆ alkyl, C₁₋₆ alkoxy, deuterated C₁₋₆ alkyl or halogenated C₁₋₆ alkyl;
p46 represents an integer of 0, 1, 2, 3 or 4;
(R¹³⁴)ₚ₄₇ indicates that the benzene ring in Formula (PBM-46) is optionally substituted with p47 R¹³⁴, with each R¹³⁴ being the same or different and each independently representing hydroxy, amino, cyano, halogen, C₁₋₆ alkyl, C₁₋₆ alkoxy, deuterated C₁₋₆ alkyl or halogenated C₁₋₆ alkyl;
p47 represents an integer of 0, 1, 2, 3 or 4;
(R¹³⁵)ₚ₄₈ indicates that the benzene ring in Formula (PBM-46) is optionally substituted with p48 R¹³⁵, with each R¹³⁵ being the same or different and each independently representing hydroxy, amino, cyano, halogen, C₁₋₆ alkyl, C₁₋₆ alkoxy, deuterated C₁₋₆ alkyl or halogenated C₁₋₆ alkyl;
p48 represents an integer of 0, 1, 2, 3 or 4;
ring H in Formula (PBM-48) represents C₆₋₁₀ arylene or C₅₋₁₀ heteroarylene, and (R¹³⁷)ₚ₄₉ indicates that the ring H is optionally substituted with p49 R¹³⁷, with each R¹³⁷ being the same or different and each independently representing deuterium, halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
p49 represents an integer of 0, 1, 2, 3 or 4;
R¹³⁶ represents optionally substituted 5- to 20-membered monocyclic or bicyclic heteroaryl containing from 1 to 4 heteroatoms selected from the group consisting of oxygen, nitrogen, and sulfur, which is optionally substituted with a substituent selected from the group consisting of hydroxy, amino, cyano, halogen, C₁₋₆ alkyl, C₁₋₆ alkoxy, deuterated C₁₋₆ alkyl or halogenated C₁₋₆ alkyl;
X₂₄, X₂₅, and X₂₆ in Formula (PBM-49) each independently represents CH or N;
R¹³⁸ represents -C(O)NHR^{b19}, -NHC(O)-R^{b19}, -S(O)₂NHR^{b19}, -NHS(O)₂R^{b19}, -S(O)₂R^{b19} or - P(O)(R^{b19})₂, wherein each R^{b19} is the same or different and each independently represents C₁₋₆ alkyl or deuterated C₁₋₆ alkyl;
R¹³⁹ and R¹⁴⁰ each independently represent halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
(R¹⁴¹)ₚ₅₀ indicates that the benzene ring is optionally substituted with p50 R¹⁴¹, with each R¹⁴¹ being independently halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
p50 represents an integer of 0, 1, 2, 3 or 4; and
(R¹⁴³)ₚ₅₁ in Formula (PBM-50) indicates that the benzene ring is optionally substituted with p51 R¹⁴³, with each R¹⁴³ being independently halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
p51 represents an integer of 0, 1, 2, 3, 4 or 5;
R¹⁴² represents H, C₁₋₆ alkyl or halogenated C₁₋₆ alkyl;
(R¹⁴⁴)ₚ₅₂ indicates that the quinazoline ring in Formula (PBM-50) is optionally substituted with p52 R¹⁴⁴, with each R¹⁴⁴ being independently halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p52 represents an integer of 1, 2, or 3;
R_{f2} in Formula (PBM-51) represents a bond, or R_{f2} represents -NH-R_{f3}-C(O)-***, wherein symbol *** indicates the point of attachment to R_{f1}, and R_{f3} represents optionally substituted C₃₋₈ cycloalkyl;
(R¹⁴⁵)ₚ₅₃ indicates that the 1H-pyrrolo[2,3-b]pyridine ring in Formula (PBM-51) is optionally substituted with p53 R¹⁴⁵, with each R¹⁴⁵ being independently cyano, halogen, hydroxy, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p53 represents an integer of 0, 1, 2, 3, 4 or 5;
(R¹⁴⁶)ₚ₅₄ indicates that the pyridine ring in Formula (PBM-51) is optionally substituted with p54 R¹⁴⁶, with each R¹⁴⁶ being independently cyano, halogen, hydroxy, nitro, C₁₋₆ alkyl, halogenated C₁₋₆ alkyl, deuterated C₁₋₆ alkyl, C₁₋₆ alkoxy, or halogenated C₁₋₆ alkoxy or NR^{b25}R^{b26}, wherein R^{b25} and R^{b26} are the same or different and each independently represent hydrogen, C₁₋₆ alkyl, deuterated C₁₋₆ alkyl, halogenated C₁₋₆ alkyl, optionally substituted C₃₋₆ cycloalkyl or optionally substituted 4- to 8-membered heterocyclyl, and p54 represents an integer of 0, 1, 2 or 3; and
(R¹⁴⁷)ₚ₅₅ in Formula (PBM-52) indicates that the benzene ring is optionally substituted with p55 R¹⁴⁷, with each R¹⁴⁷ being independently halogen, hydroxy, cyano, amino, nitro, C₁₋₆ alkyl, halogenated C₁₋₆ alkyl, deuterated C₁₋₆ alkyl, C₁₋₆ alkoxy or halogenated C₁₋₆ alkoxy, and p55 represents an integer of 0, 1, 2, 3, 4 or 5;
R¹⁴⁸ represents NHC(O) or C(O)NH;
(R¹⁴⁹)ₚ₅₆ indicates that the 1H-pyrazole ring in Formula (PBM-52) is optionally substituted with p56 R¹⁴⁹, with each R¹⁴⁹ being independently halogen, hydroxy, cyano, amino, nitro, C₁₋₆ alkyl, halogenated C₁₋₆ alkyl, deuterated C₁₋₆ alkyl, C₁₋₆ alkoxy or halogenated C₁₋₆ alkoxy, and p56 represents an integer of 0, 1 or 2; and
X₂₇ in Formula (PBM-53) represents N or CH;
R¹⁵⁰ represents a bond or optionally substituted methylene (e.g., halogenated methylene);
ring I represents C₆₋₁₀ arylene or 5- to 15-membered heteroarylene, and (R¹⁵¹)ₚ₅₇ indicates that the ring I is optionally substituted with p57 R¹⁵¹, with each R¹⁵¹ being the same or different and each independently representing deuterium, halogen, hydroxy, cyano, amino, nitro, oxo, C₁₋₆ alkyl, halogenated C₁₋₆ alkyl, deuterated C₁₋₆ alkyl, C₁₋₆ alkoxy, or halogenated C₁₋₆ alkoxy, and p57 represents an integer of 0, 1, 2, 3 or 4;
(R¹⁵²)ₚ₅₈ indicates that the 6-membered ring containing X₂₇ in Formula (PBM-53) is optionally substituted with p58 R¹⁵², with each R¹⁵² independently representing halogen, C₁₋₆ alkoxy, C₁₋₆ alkyl, deuterated C₁₋₆ alkyl or halogenated C₁₋₆ alkyl, and p58 represents an integer of 0, 1 or 2;
(R¹⁵³)ₚ₅₉ indicates that the benzene ring in Formula (PBM-53) is optionally substituted with p59 R¹⁵³, with each R¹⁵³ being the same or different and each independently representing halogen, C₁₋₆ alkoxy, C₁₋₆ alkyl, deuterated C₁₋₆ alkyl or halogenated C₁₋₆ alkyl, and p15 represents an integer of 0, 1, 2, 3 or 4;
X₂₈, X₂₉ and X₃₀ in Formula (PBM-54) independently represent N or CH;
(R¹⁵⁴)ₚ₆₀ indicates that the naphthyl in Formula (PBM-54) is optionally substituted with p60 R¹⁵⁴, with each R¹⁵⁴ independently representing halogen, hydroxy, amino, mercapto, nitro, cyano, C₂₋₆ alkynyl, C₂₋₆ alkenyl, C₁₋₆ alkoxy, C₁₋₆ alkyl or halogenated C₁₋₆ alkyl, and p60 represents an integer of 0, 1, 2, 3, 4, 5, 6 or 7;
R¹⁵⁵ represents optionally substituted C₃₋₁₁ cycloalkyl or optionally substituted 4- to 15-membered heterocyclyl containing from 1 to 4 heteroatoms selected from the group consisting of oxygen, nitrogen, and sulfur;
R¹⁵⁶ represents hydrogen, halogen, hydroxy, cyano, amino, mercapto, nitro, C₂₋₆ alkynyl, C₂₋₆ alkenyl, C₁₋₆ alkoxy, C₁₋₆ alkyl or halogenated C₁₋₆ alkyl;
R¹⁵⁷ in Formula (PBM-55) represents C₆₋₁₀ aryl, or heteroaryl containing one or two 5- to 7-membered rings and 1 to 4 heteroatoms selected from nitrogen, oxygen, and sulfur, wherein the C₆₋₁₀ aryl and heteroaryl are each optionally substituted with one or more substituents selected from the group consisting of halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
R¹⁵⁸ represents 5- to 15-membered heteroaryl containing 1 to 4 heteroatoms selected from nitrogen, oxygen, and sulfur, wherein the 5- to 15-membered heteroaryl is optionally substituted with one or more substituents selected from the group consisting of halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
(R¹⁵⁹)ₚ₆₁ indicates that the isoindolinone ring in Formula (PBM-55) is substituted with p61 R¹⁵⁹, with each R¹⁵⁹ being the same or different and each independently representing halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p61 represents an integer of 0, 1, 2 or 3;
X₃₁ in Formula (PBM-56) represents N or CH;
(R¹⁶⁰)ₚ₆₂ indicates that the aromatic ring containing X₃₁ in Formula (PBM-56) is substituted with p62 R¹⁶⁰, with each R¹⁶⁰ being the same or different and each independently representing halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p62 represents an integer of 0, 1, 2, 3 or 4; and
ring J represents 5- to 15-membered heteroarylene, and (R¹⁶¹)ₚ₆₃ indicates that the ring J is optionally substituted with p63 R¹⁶¹, with each R¹⁶¹ being the same or different and each independently representing halogen, hydroxy, mercapto, cyano, amino, nitro, C₁₋₆ alkyl, halogenated C₁₋₆ alkyl, deuterated C₁₋₆ alkyl, hydroxy substituted C₁₋₆ alkyl, C₁₋₆ alkoxy, or halogenated C₁₋₆ alkoxy, and p63 represents an integer of 0, 1, 2 or 3.

15. The compound of Formula (II) or salts, enantiomers, stereoisomers, solvates, isotopically enriched analogs, prodrugs, or polymorphs thereof as claimed in claim 12, wherein PBM comprises the structure of formula selected from:

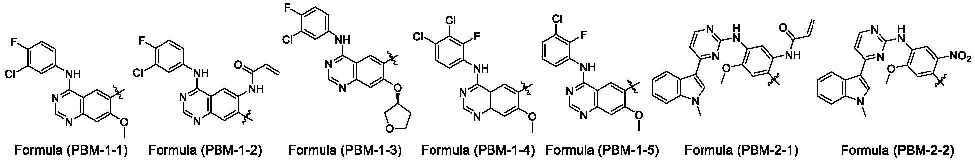

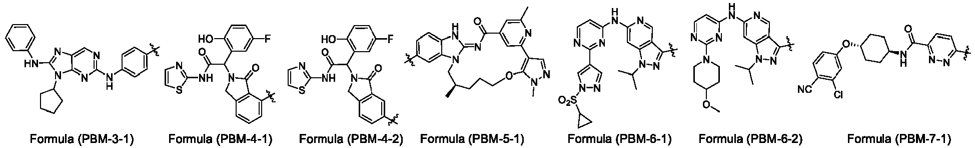

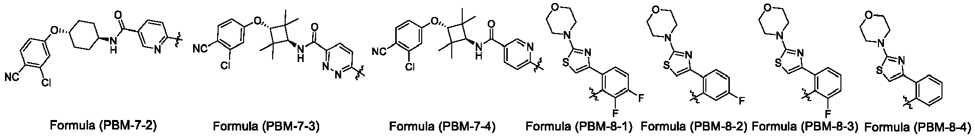

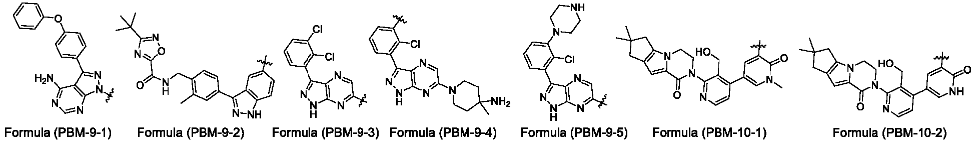

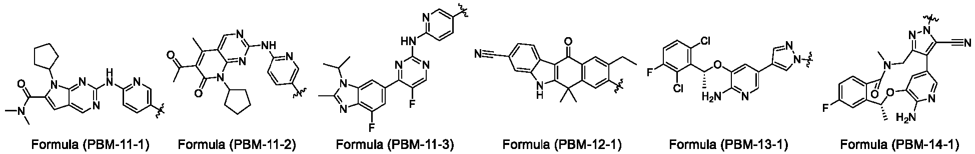

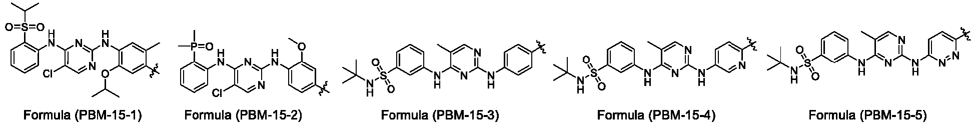

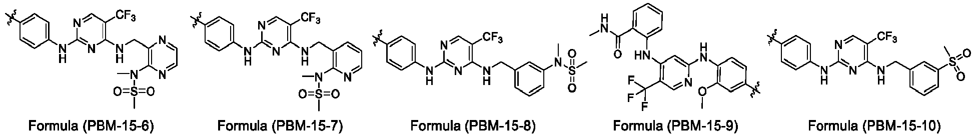

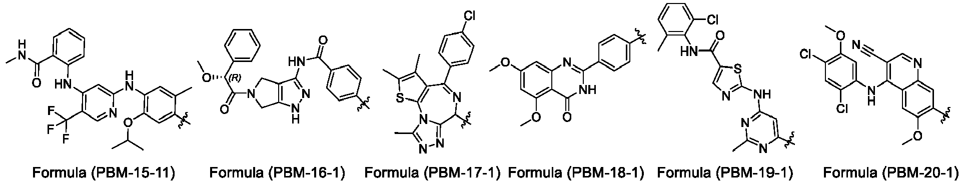

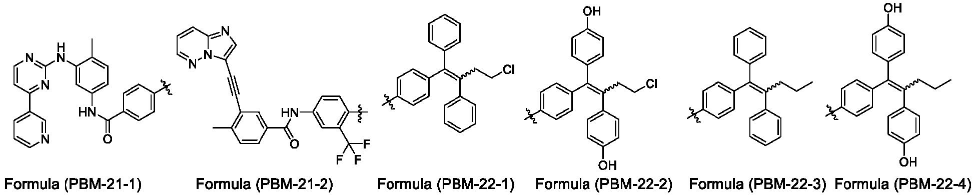

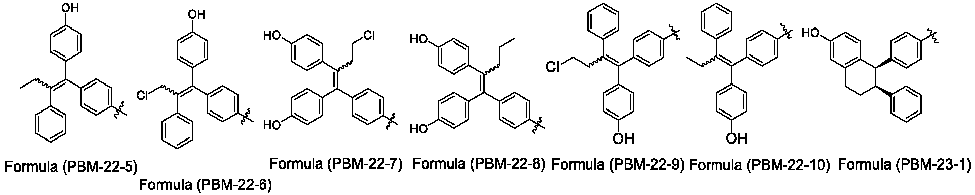

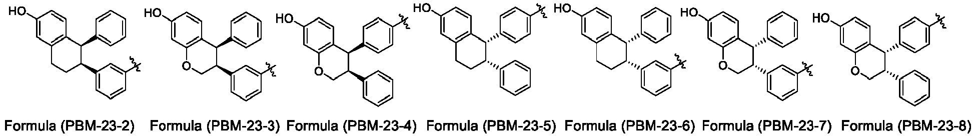

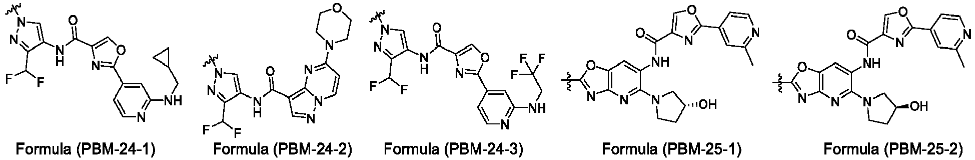

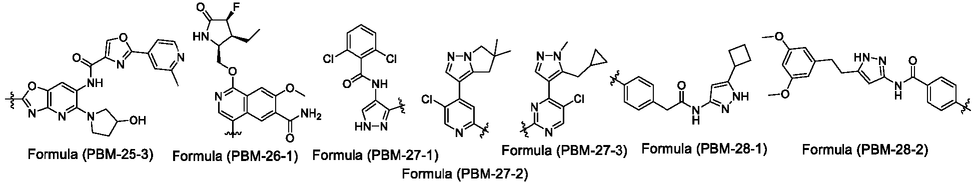

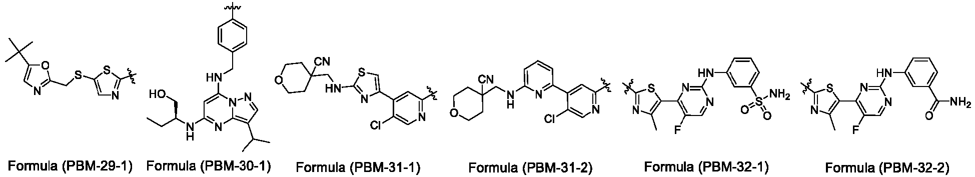

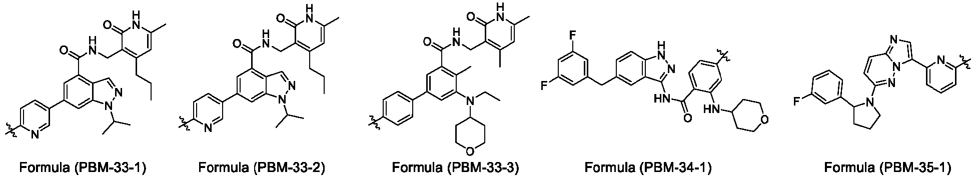

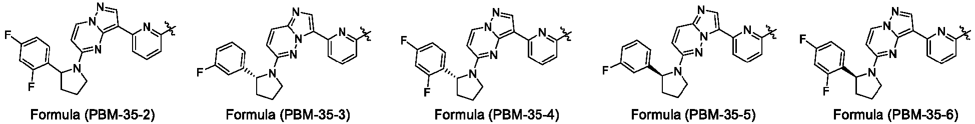

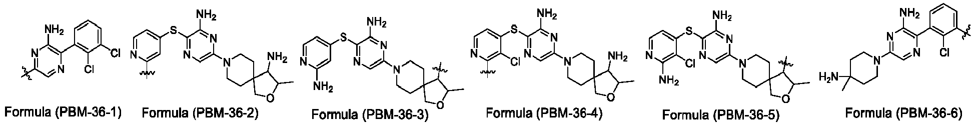

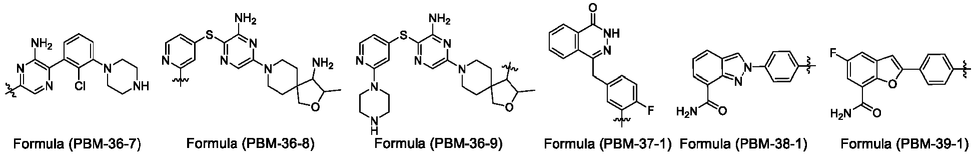

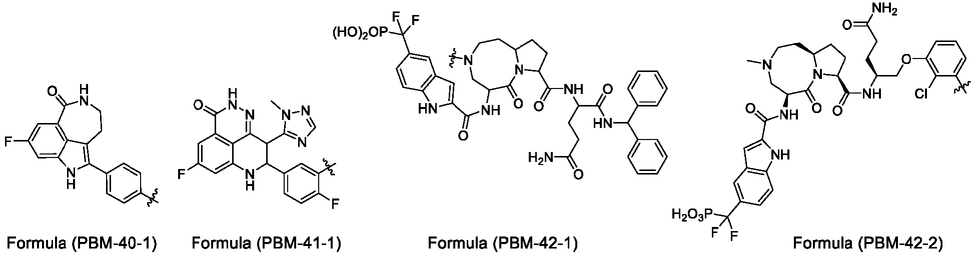

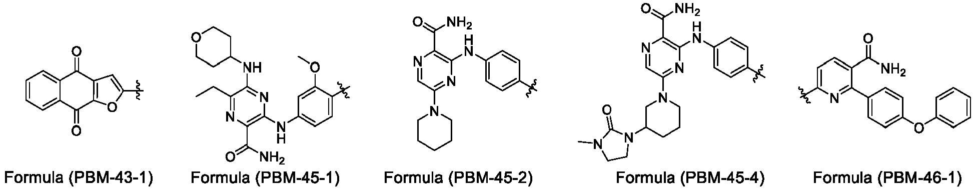

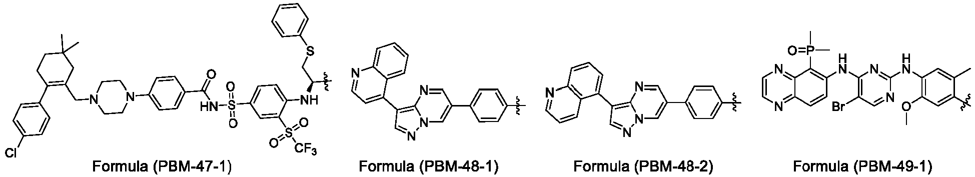

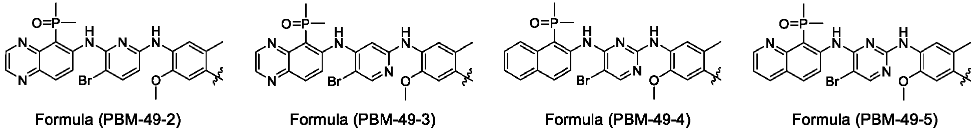

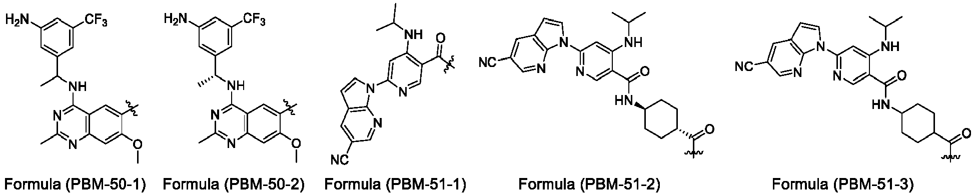

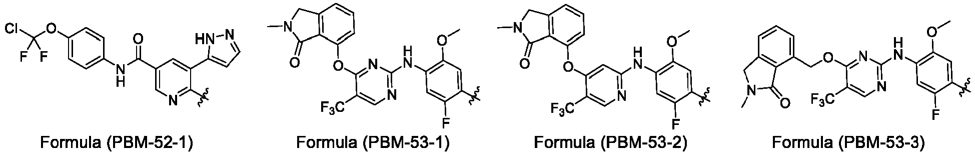

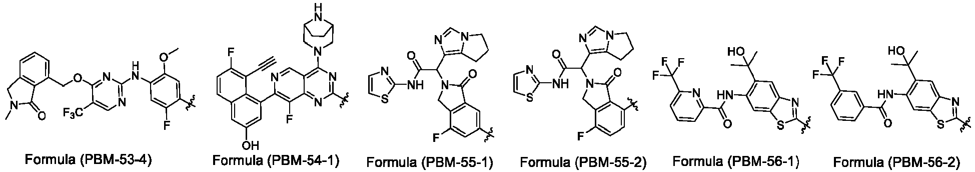

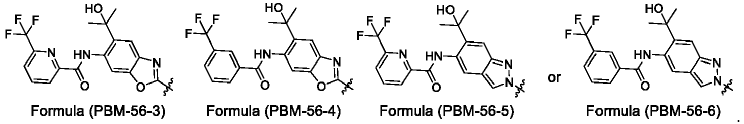

16. The compound of Formula (II) or salts, enantiomers, stereoisomers, solvates, isotopically enriched analogs, prodrugs, or polymorphs thereof as claimed in claim 12, wherein
R_{f} represents a bond, -O-, -NHC(O)-*, -C(O)NH-*, or -NH-, or
R_{f} represents the structure of the following formula:
-N(R_{g})-, -NHC(O)-C₁₋₆ alkylene-W⁵-*, -NHC(O)-C₂₋₆ alkenylene-W⁵-*, -C(O)NH-C₁₋₆ alkylene-W⁵-*, -C(O)NH-C₂₋₆ alkenylene-W⁵-*, -NHC(O)-W⁵-*, -C(O)NH-W⁵-*, -O-C₁₋₆ alkylene-W⁵-*, -O-C₂₋₆ alkenylene-W⁵-*, -O-W⁵-*, -O-C₁₋₆ alkylene-N(Rᵢ)-*, -O-C₂₋₆ alkenylene-N(Rᵢ)-*, -N(R_{g})-C₁₋₆ alkylene-N(Rᵢ)-*, -N(R_{g})-C₂₋₆ alkenylene-N(Rᵢ)-*, -W⁵-, -W⁵-N(R_{g})-*, -N(R_{g})-W⁵-*, -N(R_{g})-W⁵-N(Rᵢ)-*, -C₁₋₆ alkylene-W⁵-*, -C₂₋₆ alkenylene-W⁵-*, -C₁₋₆ alkylene -C(O)-W⁵-*, -C₂₋₆ alkenylene-C(O)-W⁵-*, -C(O)-W⁵-*, -C₁₋₆ alkylene-C(O)NH-C₁₋₆ alkylene-W⁵-*, -C₁₋₆ alkylene-C(O)NH-C₂₋₆ alkenylene-W⁵-*, -C₂₋₆ alkenylene-C(O)NH-C₁₋₆ alkylene-W⁵-*, -C₂₋₆ alkenylene-C(O)NH-C₂₋₆ alkenylene-W⁵-*, -C₁₋₆ alkylene-NHC(O)-C₁₋₆ alkylene-W⁵-*, -C₁₋₆ alkylene-NHC(O)-C₂₋₆ alkenylene-W⁵-*, -C₂₋₆ alkenylene-NHC(O)-C₁₋₆ alkylene-W⁵-*, -C₂₋₆ alkenylene-NHC(O)-C₂₋₆ alkenylene-W⁵-*, -C₁₋₆ alkylene-C(O)NH-*, -C₂₋₆ alkenylene-C(O)NH-*, -C₁₋₆ alkylene-NHC(O)-*, -C₂₋₆ alkenylene-NHC(O)-*, -C₁₋₆ alkylene-, -C₂₋₆ alkenylene-, -C₁₋₆ alkylene-N(Rᵢ)-*, -C₂₋₆ alkenylene-N(Rᵢ)-*, 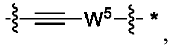
wherein R_{g} and Rᵢ each independently represent hydrogen or C₁₋₆ alkyl, and the C₁₋₆ alkylene and C₂₋₆ alkenylene are optionally substituted with one or more substituents selected from the group consisting of halogen, hydroxy, mercapto, cyano, amino, nitro, C₁₋₆ alkyl, halogenated C₁₋₃ alkyl, C₁₋₃ alkoxy, halogenated C₁₋₃ alkoxy or any combination thereof;
wherein W⁵ represents the structure of the following formula: 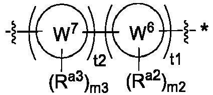
wherein each rings W⁶ is the same or different and each independently represents nitrogen-containing heterocyclylene, t1 represents an integer of 1 or 2 , and (R^{a2})ₘ₂ indicates that each ring W⁶ is optionally substituted with m2 R^{a2} substituents, with each R^{a2} being independently deuterium, optionally deuterated C₁₋₆ alkyl, optionally halogenated C₁₋₆ alkyl, C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{12a}-R^{11a}-, wherein R^{11a} is optionally substituted C₁₋₆ alkylene or optionally substituted C₂₋₆ alkenylene, and R^{12a} represents halogen, R^{13a}R^{14a}N-, hydroxy, carboxyl, ethynyl, or vinyl, wherein R^{13a} and R^{14a} are the same or different and each independently represent hydrogen or C₁₋₃ alkyl, and the C₁₋₆ alkylene and C₂₋₆ alkenylene is optionally substituted with one or more substituents selected from the group consisting of halogen, hydroxy, mercapto, cyano, amino, nitro, C₁₋₆ alkyl, halogenated C₁₋₃ alkyl, C₁₋₃ alkoxy, halogenated C₁₋₃ alkoxy or any combination thereof, and m2 represents an integer of 0-20;
each ring W⁷ is the same or different and each independently represents nitrogen-containing heterocyclylene, arylene, or heteroarylene, t2 represents an integer of 0 or 1, and (R^{a3})ₘ₃ indicates that each ring W⁷ is independently optionally substituted with m3 R^{a3} substituents, with each R^{a3} being independently deuterium, optionally deuterated C₁₋₆ alkyl, C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{16a}-R^{15a}-, wherein R^{15a} is optionally substituted C₁₋₆ alkylene or optionally substituted C₂₋₆ alkenylene, and R^{16a} represents halogen, R^{17a}R^{18a}N-, hydroxy, carboxyl, ethynyl, or vinyl, wherein R^{17a} and R^{18a} are the same or different and each independently represent hydrogen or C₁₋₃ alkyl, and m3 represents an integer of 0-20; and
symbol * indicates the point of attachment to LIN.

17. The compound of Formula (II) or salts, enantiomers, stereoisomers, solvates, isotopically enriched analogs, prodrugs, or polymorphs thereof as claimed in claim 12, wherein R_{f} represents -W⁷-Rₕ-W⁵-*, wherein Rₕ represents optionally substituted C₁₋₆ alkylene or optionally substituted C₂₋₆ alkenylene, W⁵ and W⁷ are as defined in claim 12 or 16.

18. The compound of Formula (II) or salts, enantiomers, stereoisomers, solvates, isotopically enriched analogs, prodrugs, or polymorphs thereof as claimed in any one of claims 12 or 16-17, wherein W⁵ represents the structure of the following formula: 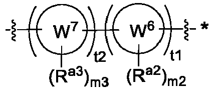
wherein each ring W⁶ is the same or different and each independently represents 4- to 20-membered nitrogen-containing heterocyclylene, e.g.,
piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, 3-azaspiro[5.5]undecan-3-ylene, 8-azaspiro[4.5]decan-4-ylene, 2-oxa-8-azaspiro[4.5]decan-4-ylene, or 3-methyl-2-oxa-8-azaspiro[4.5]decan-4-ylene, or 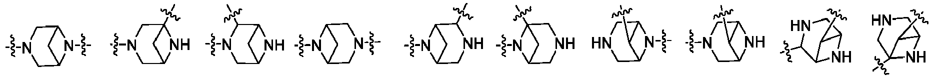 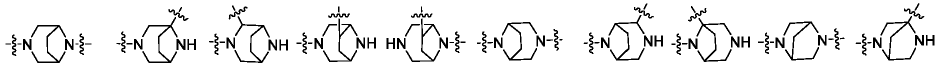 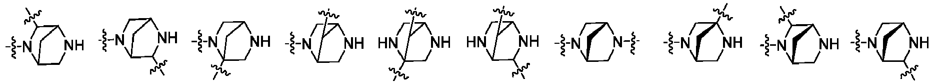 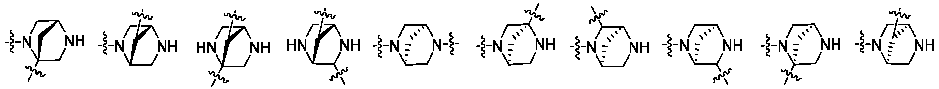 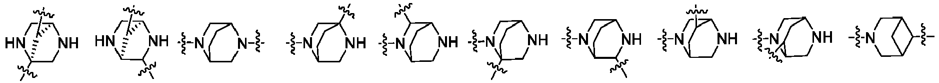 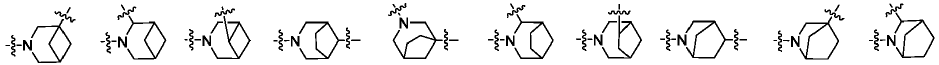 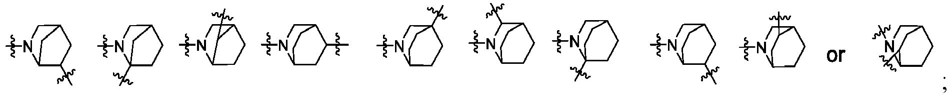
t1 represents an integer of 1 or 2, (R^{a2})ₘ₂ indicates that each ring W⁶ is optionally substituted with m2 R^{a2} substituents, with each R^{a2} being independently deuterium, optionally deuterated C₁₋₆ alkyl, optionally halogenated C₁₋₆ alkyl, C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{12a}-R^{11a}-, wherein R^{11a} is optionally substituted C₁₋₆ alkylene or optionally substituted C₂₋₆ alkenylene, and R^{12a} represents halogen, R^{13a}R^{14a}N-, hydroxy, carboxyl, ethynyl, vinyl, wherein R^{13a} and R^{14a} are the same or different and each independently represent hydrogen or C₁₋₃ alkyl, and the C₁₋₆ alkylene and C₂₋₆ alkenylene are optionally substituted with one or more substituents selected from the group consisting of halogen, hydroxy, mercapto, cyano, amino, nitro, C₁₋₆ alkyl, halogenated C₁₋₃ alkyl, C₁₋₃ alkoxy, halogenated C₁₋₃ alkoxy or any combination thereof, and m2 represents an integer of 0-20;
each ring W⁷ is the same or different and each independently represents 4- to 15-membered nitrogen-containing heterocyclylene, 6- to 10-membered arylene, or 5- to 10-membered heteroarylene, e.g.,
piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene, diazacyclooctylene, azabicyclo[3.1. 1]heptanylene, azabicyclo[2.2. 1]heptanylene, azabicyclo[3.2. 1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, 3-azaspiro[5.5]undecan-3-ylene, 8-azaspiro[4.5]decan-4-ylene, 2-oxa-8-azaspiro[4.5]decan-4-ylene, or 3-methyl-2-oxa-8-azaspiro[4.5]decan-4-ylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, and imidazo[2,1-b]thiazolylene, or 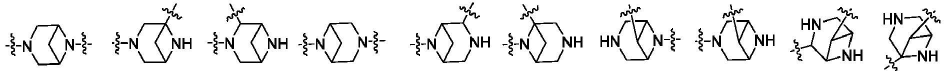 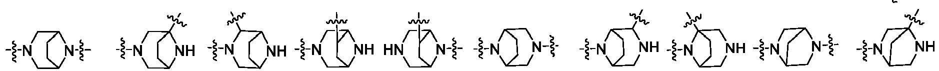 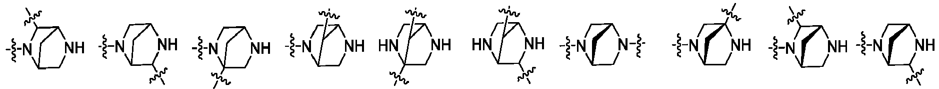 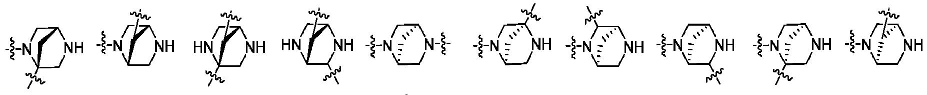 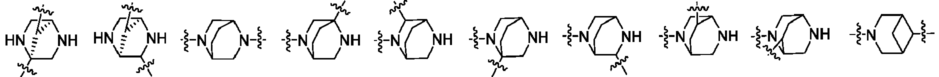 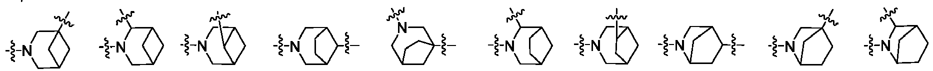 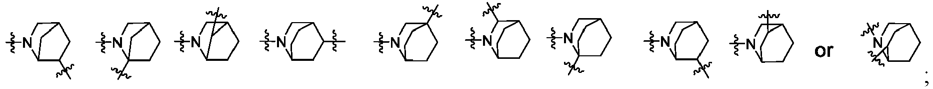
t2 represents an integer of 0 or 1, (R^{a3})ₘ₃ indicates that each ring W⁷ is optionally substituted with m3 R^{a3} substituents, with each R^{a3} being independently deuterium, optionally deuterated C₁₋₆ alkyl, C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{16a}-R^{15a}-, wherein R^{15a} is optionally substituted C₁₋₆ alkylene or optionally substituted C₂₋₆ alkenylene, and R^{16a} represents halogen, R^{17a}R^{18a}N-, hydroxy, carboxyl, ethynyl, or vinyl, wherein R^{17a} and R^{18a} are the same or different and each independently represent hydrogen or C₁₋₃ alkyl, and m3 represents an integer of 0-20; and
symbol * indicates the point of attachment to LIN.

19. The compound of Formula (II) or salts, enantiomers, stereoisomers, solvates, isotopically enriched analogs, prodrugs, or polymorphs thereof as claimed in claim 16 or 17, wherein W⁵ represents the following groups: 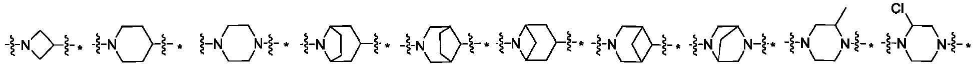 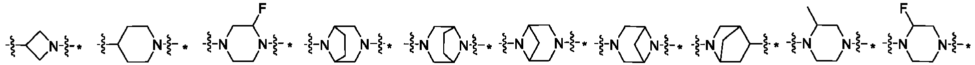 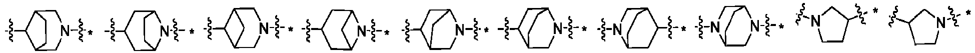 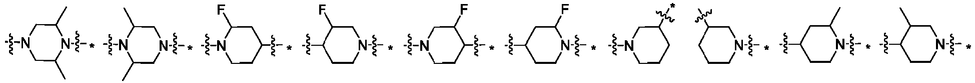 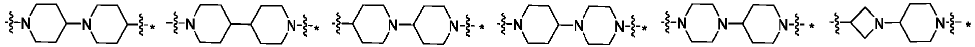 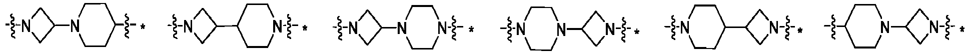 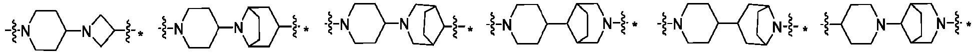 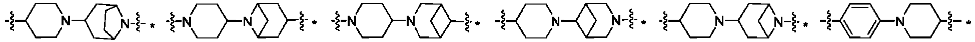 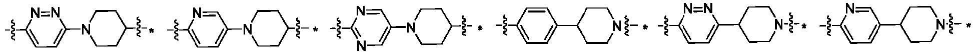 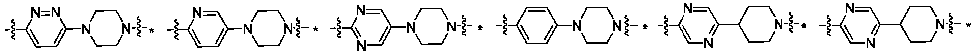 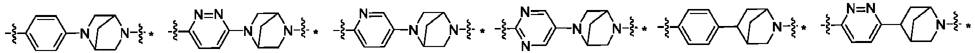 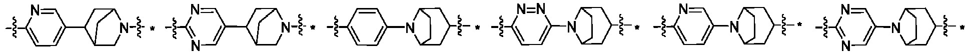 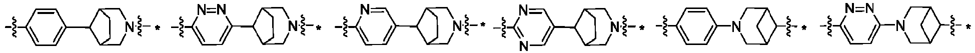 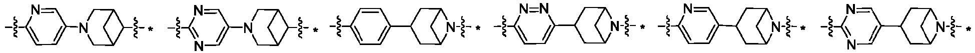 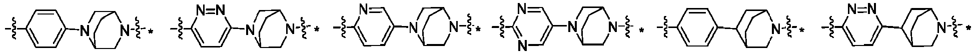 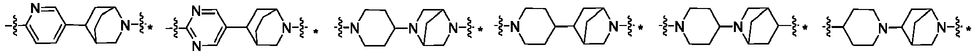 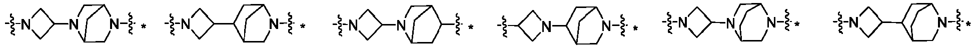 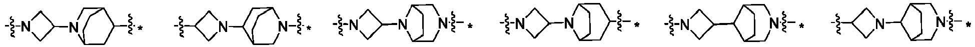 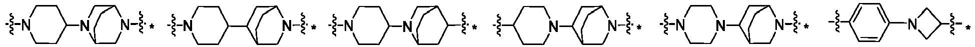 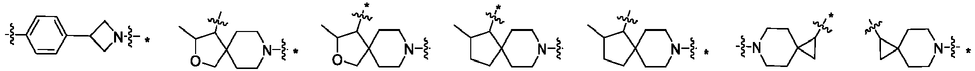 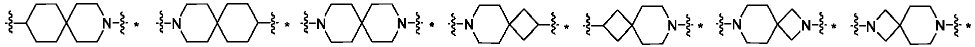 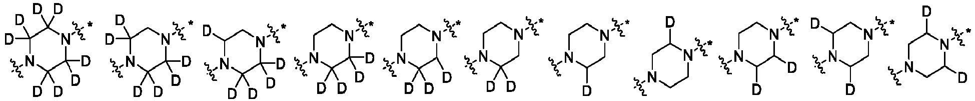 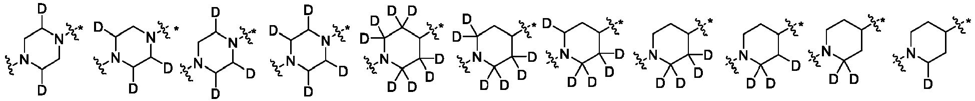 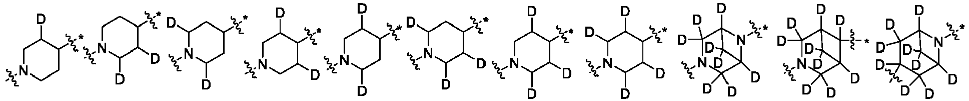 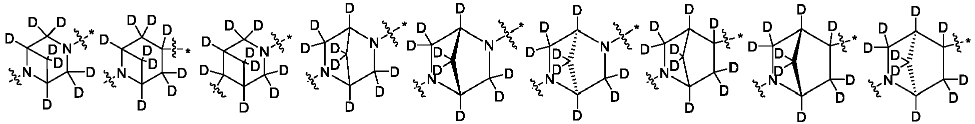 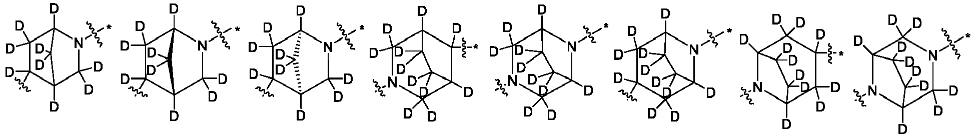 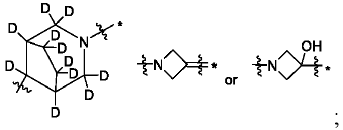
wherein the groups are optionally substituted with one or more substituents selected from the group consisting of deuterium, optionally deuterated C₁₋₆ alkyl, optionally halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, R^{12a}-R^{11a}-, or any combination thereof, wherein R^{11a} is optionally substituted C₁₋₆ alkylene or optionally substituted C₂₋₆ alkenylene, R^{12a} represents halogen, R^{13a}R^{14a}N-, hydroxy, carboxyl, ethynyl, or vinyl, wherein R^{13a} and R^{14a} are the same or different and each independently represent hydrogen or C₁₋₃ alkyl; and
symbol * indicates the point of attachment to LIN.

20. The compound of Formula (II) or salts, enantiomers, stereoisomers, solvates, isotopically enriched analogs, prodrugs, or polymorphs thereof as claimed in any one of claims 12 or 16-17, wherein R_{f} represents the following groups:
a bond, -O-, -NHC(O)-*, -C(O)NH-*, -NH-, -N(CH₃)-, -N(CH₃)-(CH₂)₂-N(CH₃)-*, -N(CH₃)-(CH₂)₃-N(CH₃)-*, -NH-(CH₂)₂-N(CH₃)-*, -N(CH₃)-(CH₂)₃-NH-*, -CH₂-NHC(O)-*, -CH₂-C(O)NH-*, -CH₂-NH-*, -O-(CH₂)₂-N(CH₃)-*, -O-(CH₂)₂-NH-*, or -CH₂-N(CH₃)-*, or 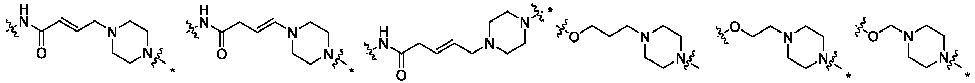 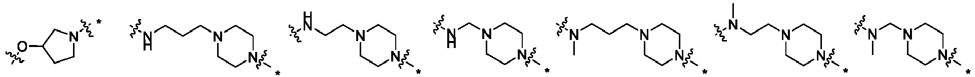 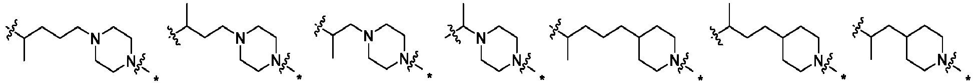 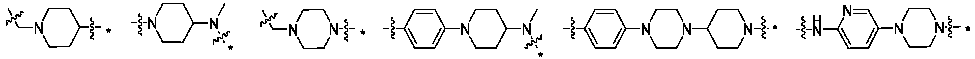 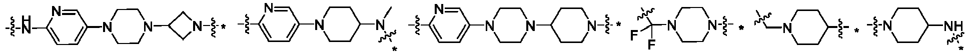 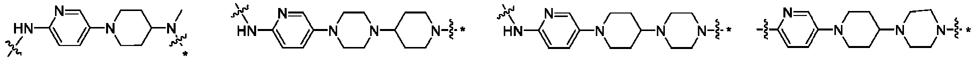 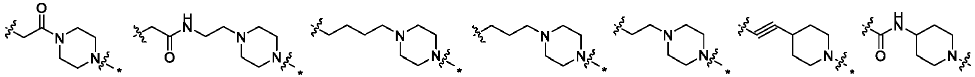 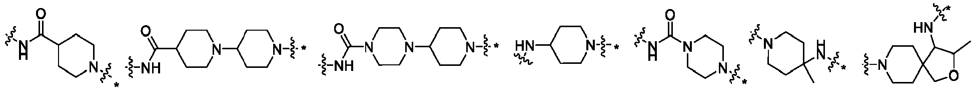 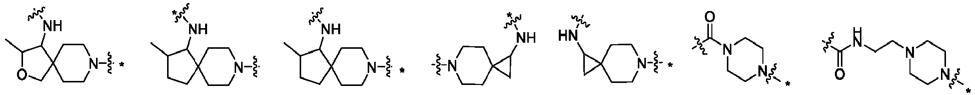 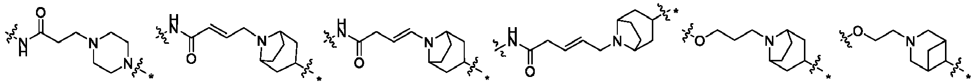 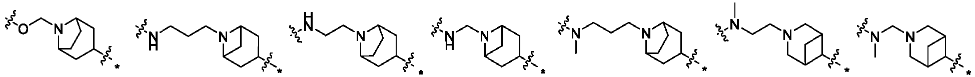 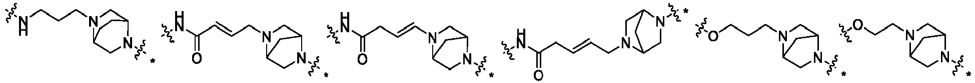 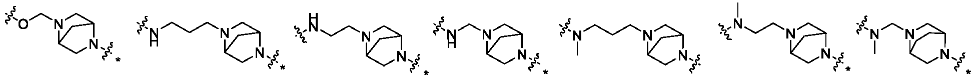 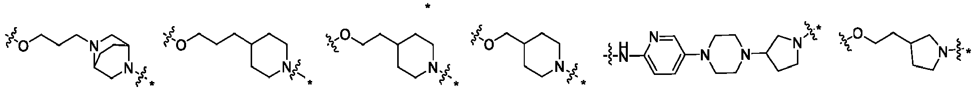 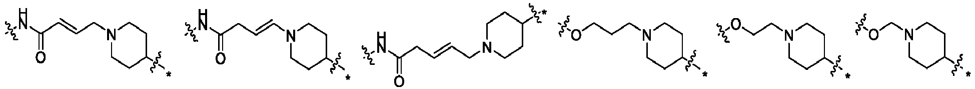 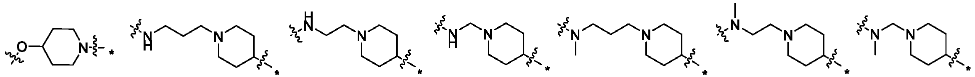 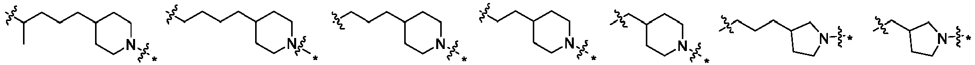 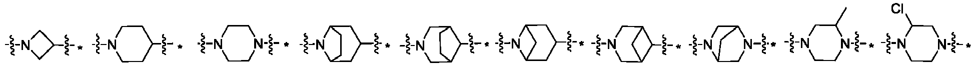 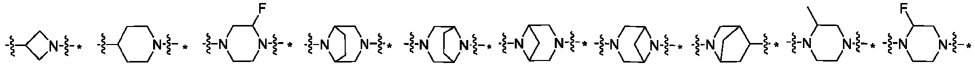 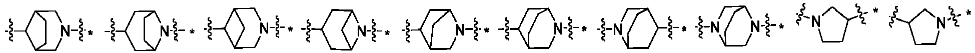 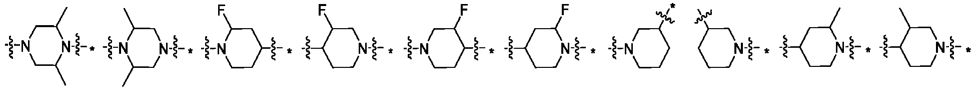 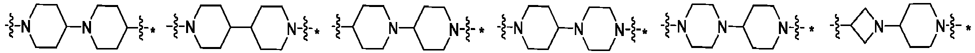 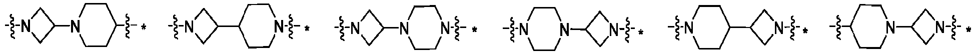 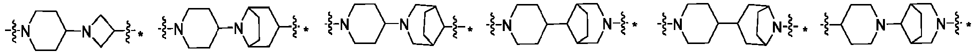 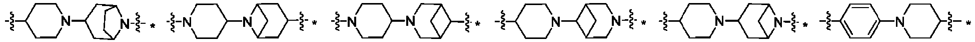 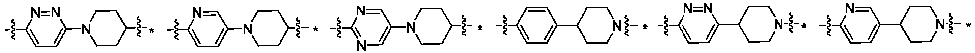 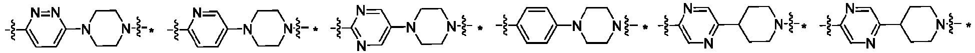 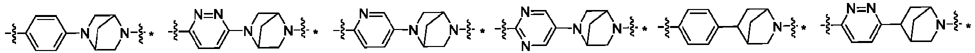 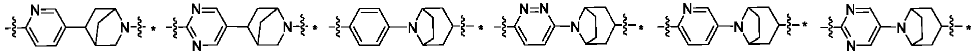 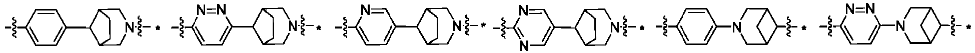 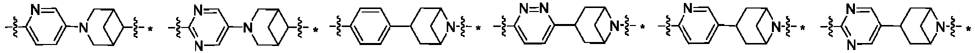 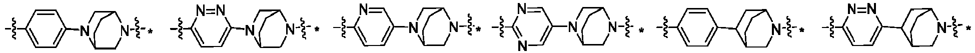 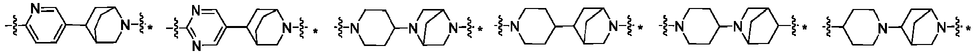 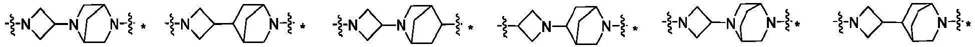 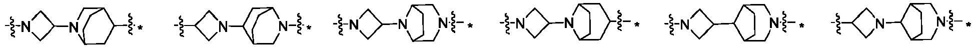 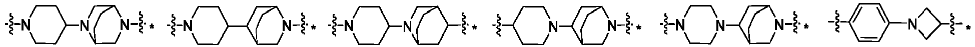 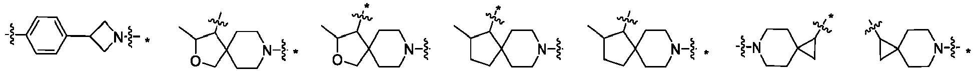 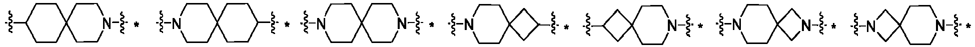 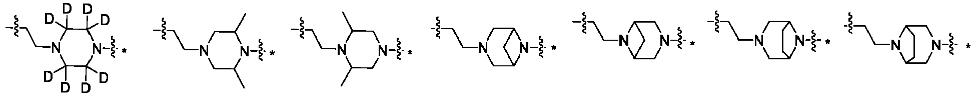 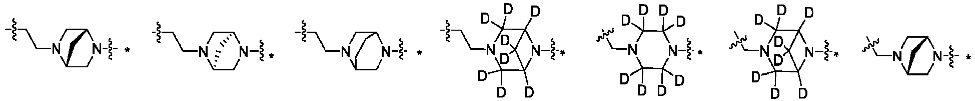 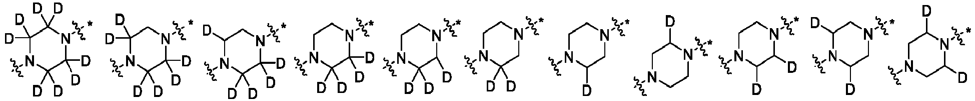 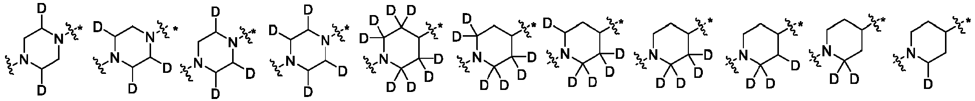 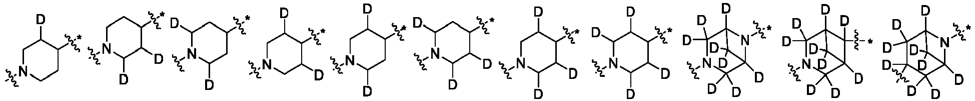 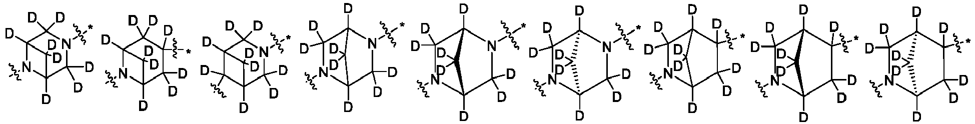 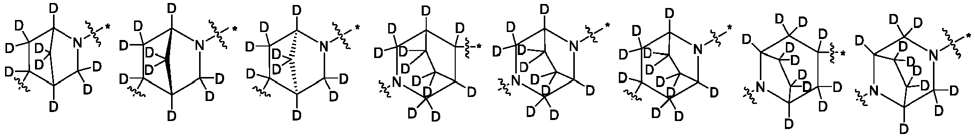 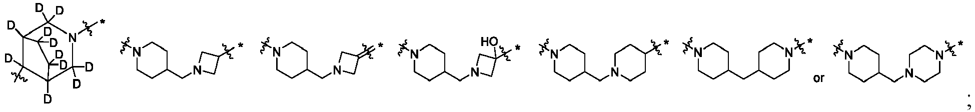 wherein the groups are optionally substituted with one or more substituents selected from the group consisting of deuterium, optionally deuterated C₁₋₆ alkyl, optionally halogenated C₁₋₆ alkyl, C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, R^{12a}-R^{11a}-, or any combination thereof, wherein R^{11a} is optionally substituted C₁₋₆ alkylene or optionally substituted C₂₋₆ alkenylene, R^{12a} represents halogen, R^{13a}R^{14a}N-, hydroxy, carboxyl, ethynyl, or vinyl, wherein R^{13a} and R^{14a} are the same or different and each independently represent hydrogen or C₁₋₃ alkyl; and
symbol * indicates the point of attachment to LIN.

21. The compound of Formula (II) or salts, enantiomers, stereoisomers, solvates, isotopically enriched analogs, prodrugs, or polymorphs thereof as claimed in claim 12, wherein ULM represents the structure of the following Formula (ULM-1), Formula (ULM-2), Formula (ULM-3), Formula (ULM-4), Formula (ULM-5), Formula (ULM-6), Formula (ULM-7), Formula (ULM-8), Formula (ULM-9), Formula (ULM-10) , Formula (ULM-11), or Formula (ULM-12): 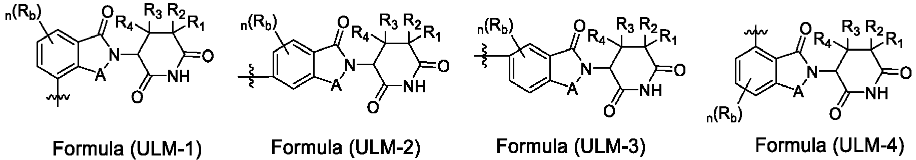 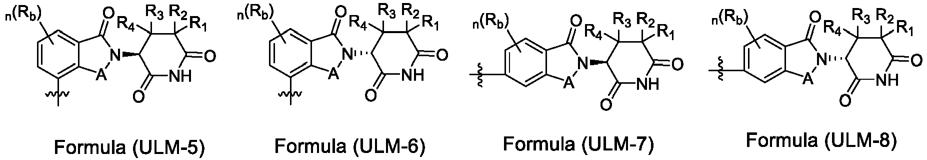 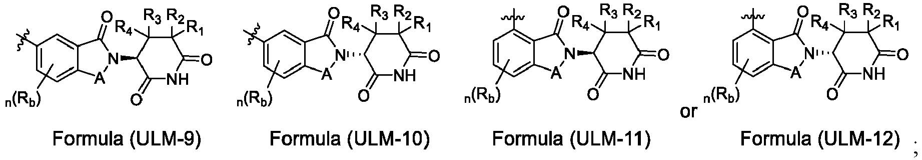 wherein
A represents CO, CH₂ or CD₂;
R₁, R₂, R₃ and R₄ are the same or different and each independently represent hydrogen, deuterium, halogen, optionally deuterated C₁₋₆ alkyl, optionally deuterated C₁₋₆ alkoxy, or halogenated C₁₋₆ alkyl;
(R_{b})ₙ indicates that benzene ring is optionally substituted with n R_{b} substituents, with each R_{b} being the same or different and independently representing deuterium, halogen, hydroxy, mercapto, nitro, amino, cyano, optionally deuterated C₁₋₆ alkyl, optionally deuterated C₁₋₆ alkoxy, halogenated C₁₋₆ alkyl, optionally substituted C₃₋₆ cycloalkyl, C₂₋₆ alkynyl, or C₂₋₆ alkenyl; and
N represents an integer of 0, 1, 2 or 3.

22. The compound of Formula (II) or salts, enantiomers, stereoisomers, solvates, isotopically enriched analogs, prodrugs, or polymorphs thereof as claimed in claim 20, wherein ULM represents the structure of the following Formula (ULM-13), Formula (ULM-14), Formula (ULM-15), Formula (ULM-16), Formula (ULM-17), Formula (ULM-18), Formula (ULM-19), Formula (ULM-20), Formula (ULM-21), Formula (ULM-22) , Formula (ULM-23), or Formula (ULM-24): 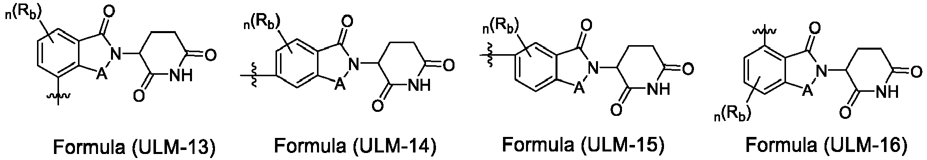 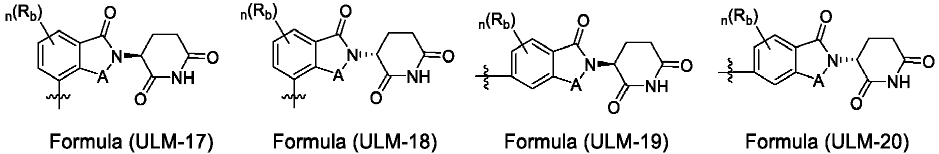 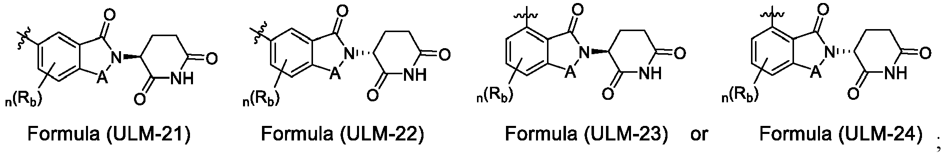 wherein A, (R_{b})ₙ, and R_{b} are as defined in claim 19.

23. The compound of Formula (II) or salts, enantiomers, stereoisomers, solvates, isotopically enriched analogs, prodrugs, or polymorphs thereof as claimed in claim 20, wherein ULM represents the structure of the following formula:

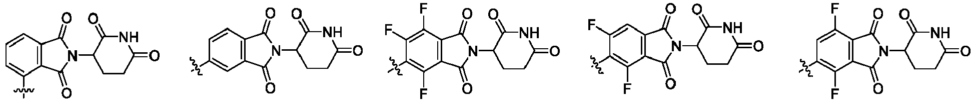

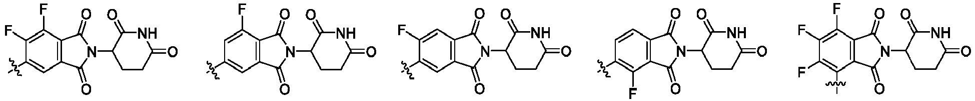

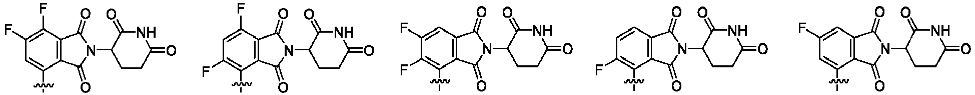

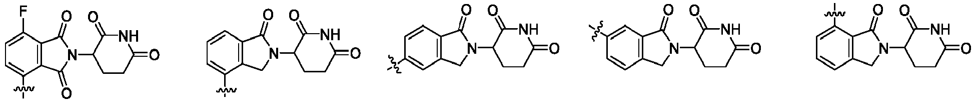

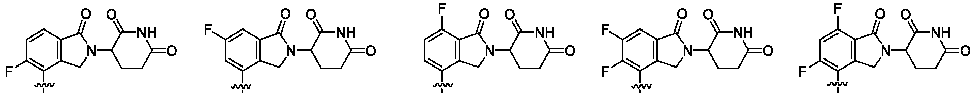

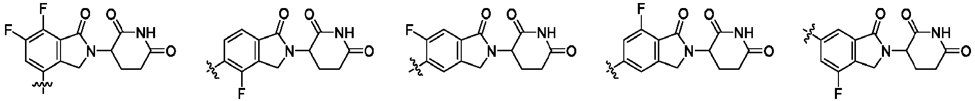

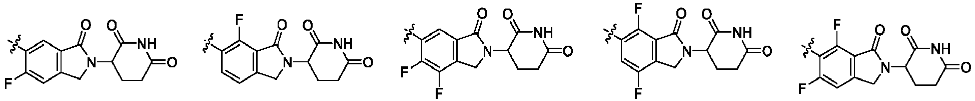

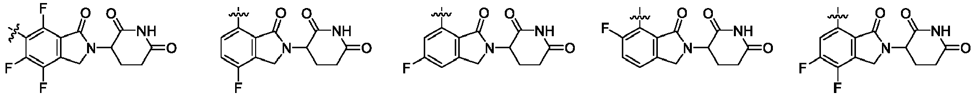

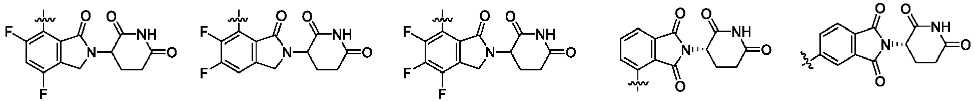

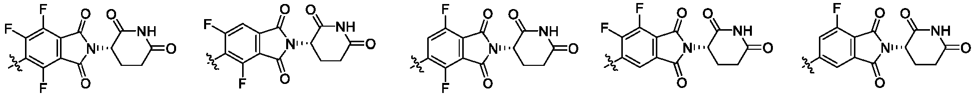

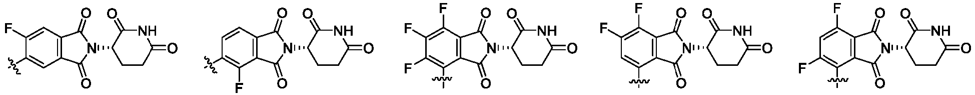

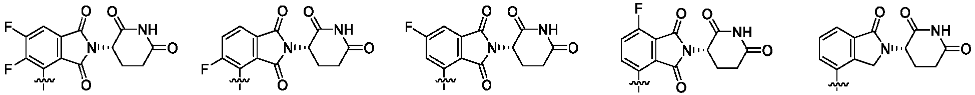

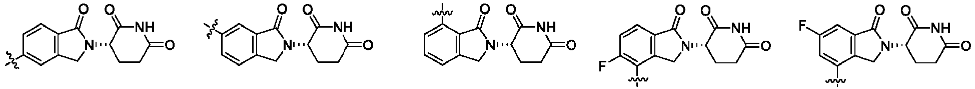

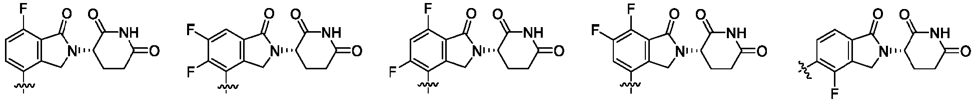

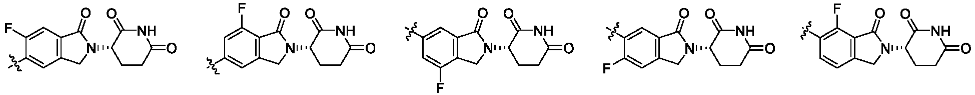

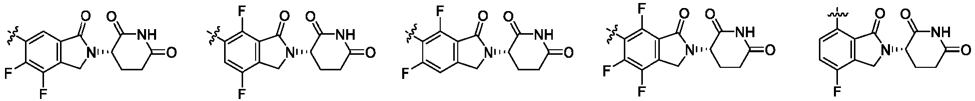

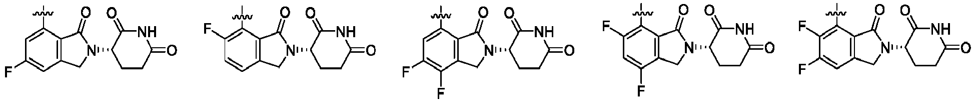

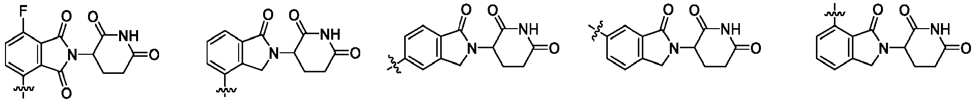

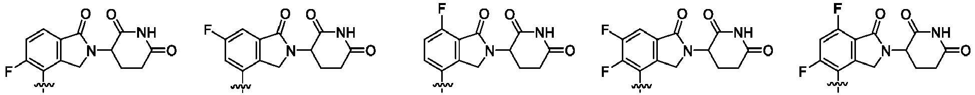

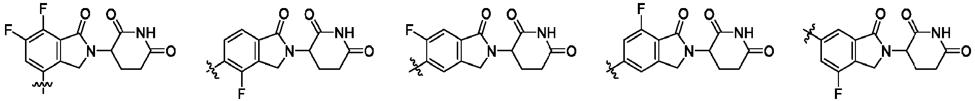

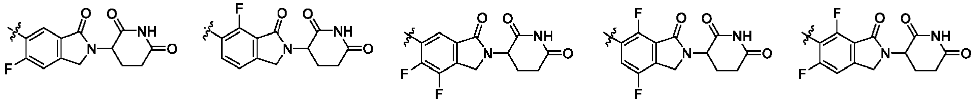

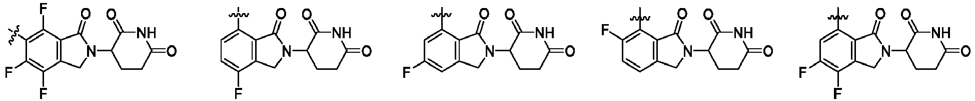

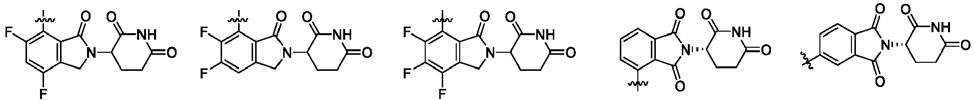

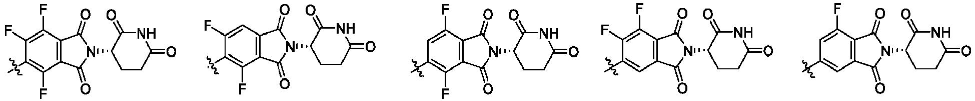

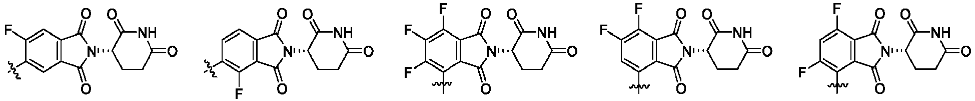

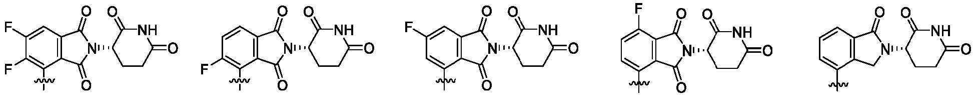

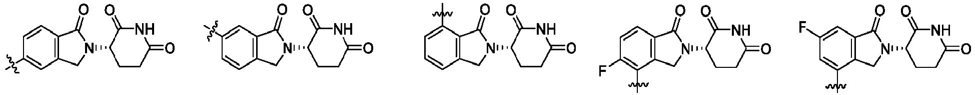

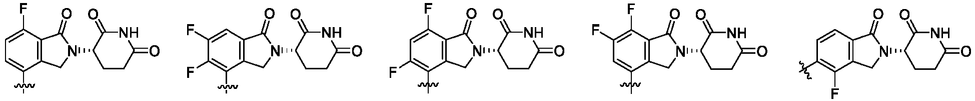

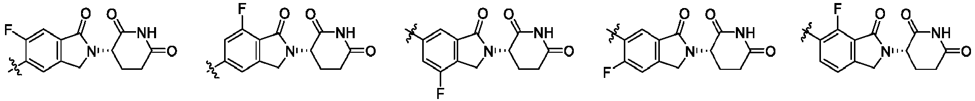

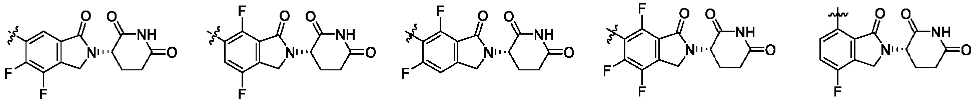

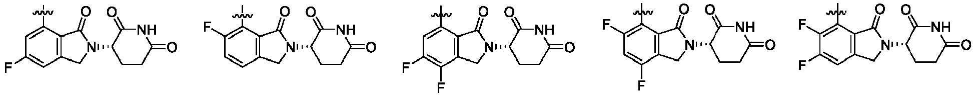

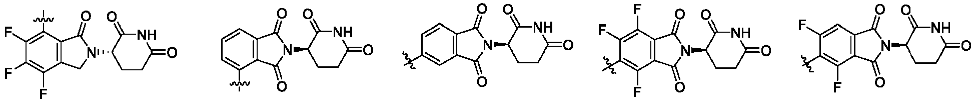

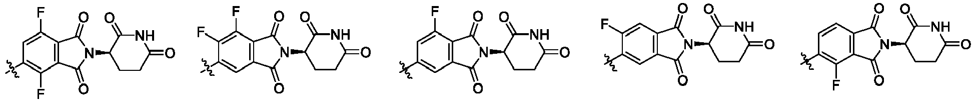

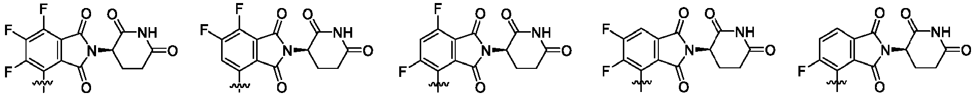

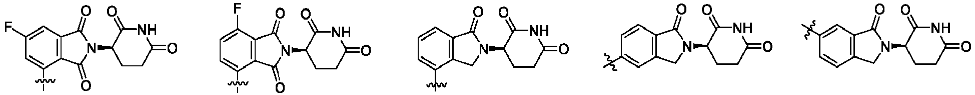

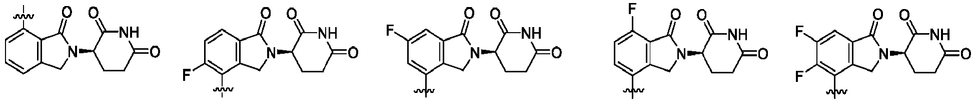

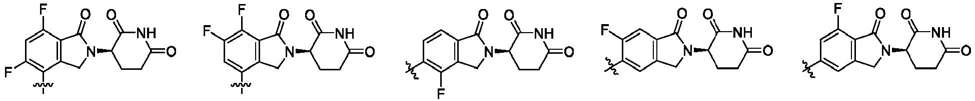

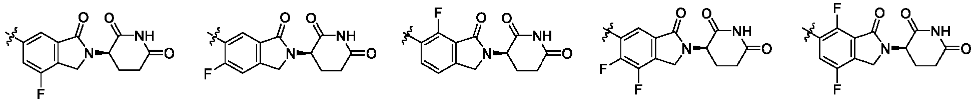

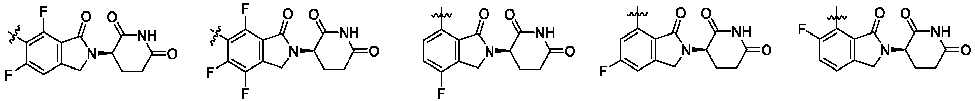

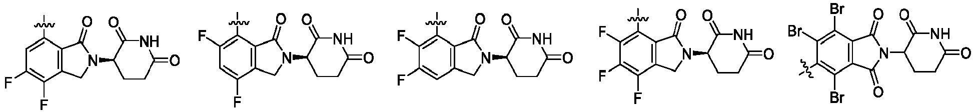

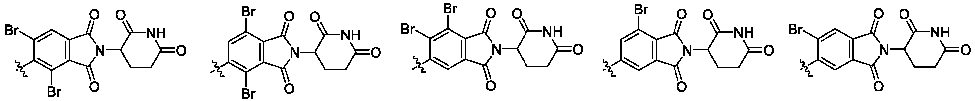

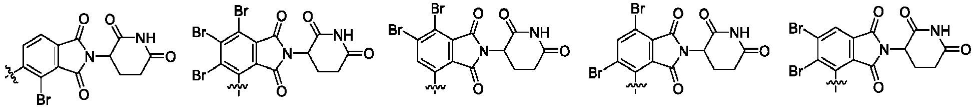

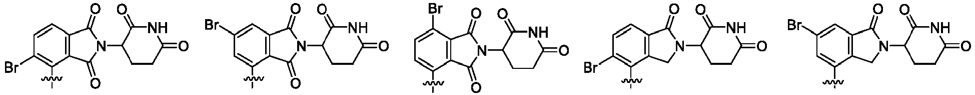

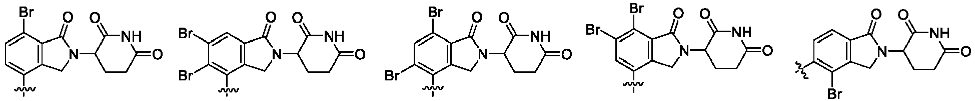

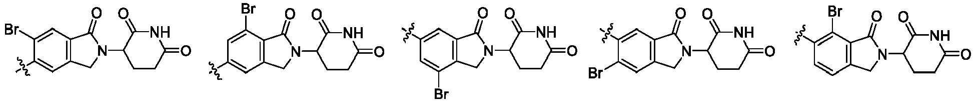

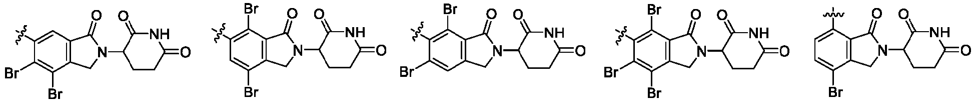

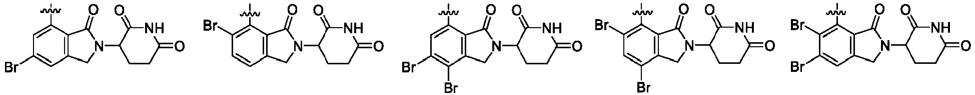

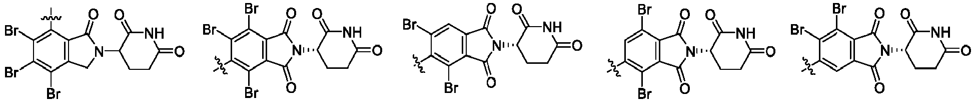

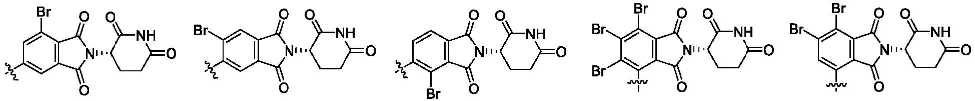

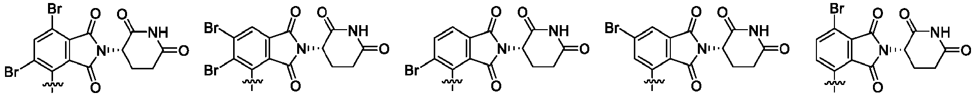

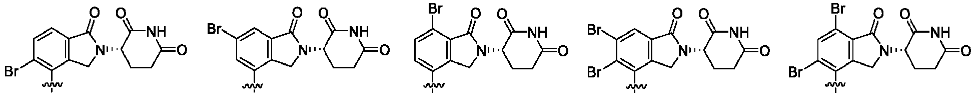

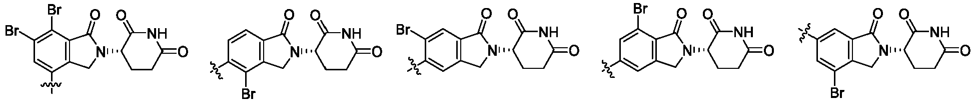

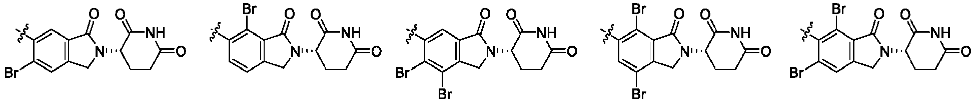

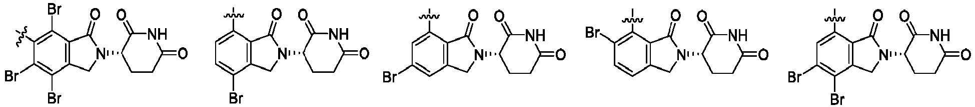

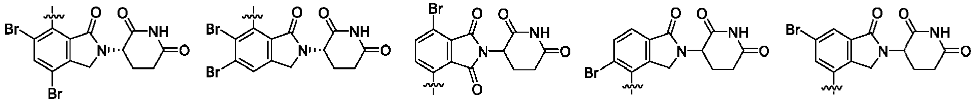

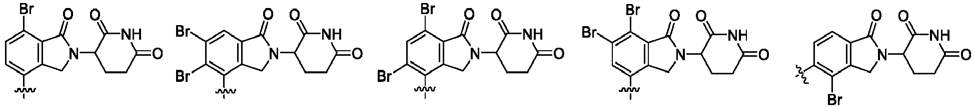

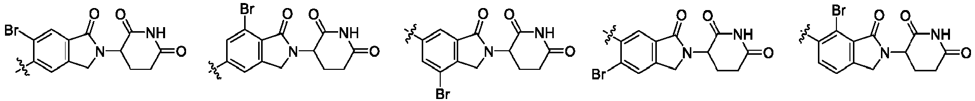

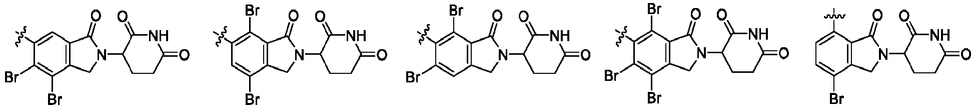

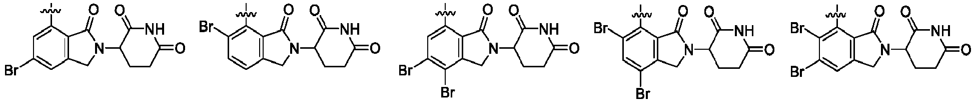

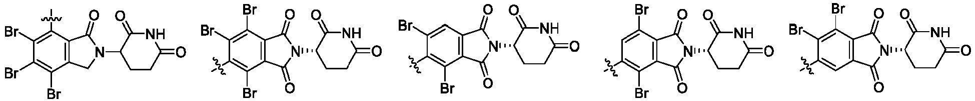

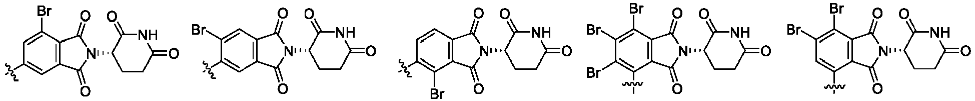

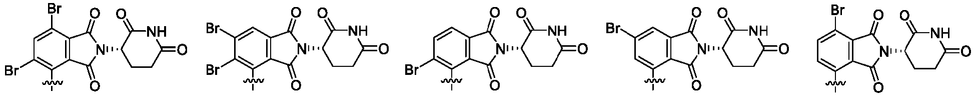

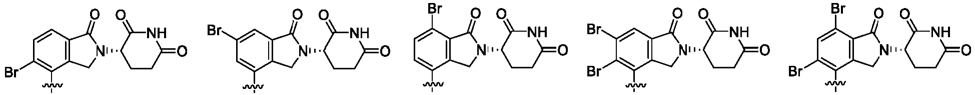

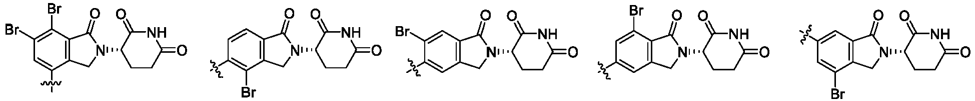

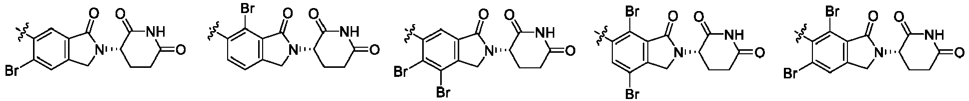

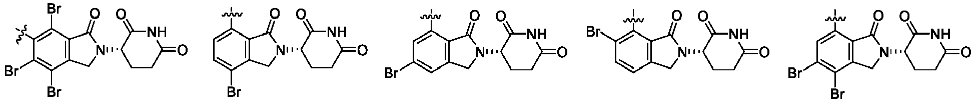

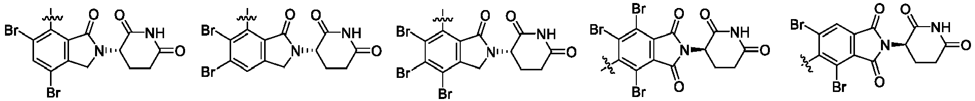

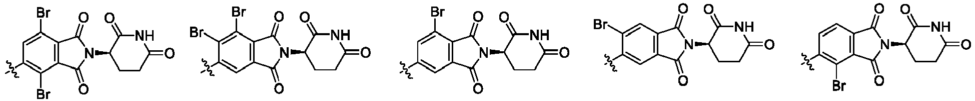

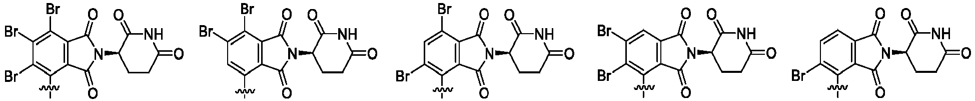

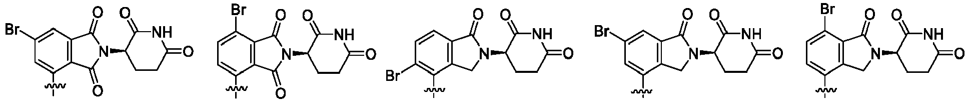

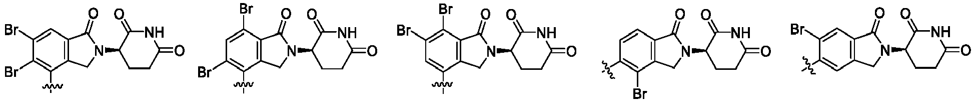

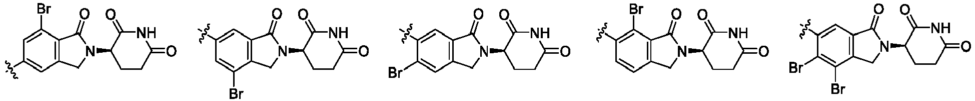

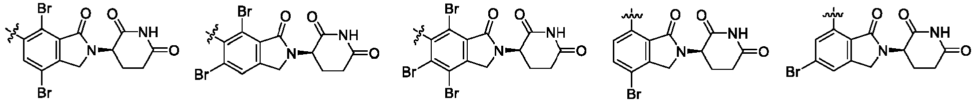

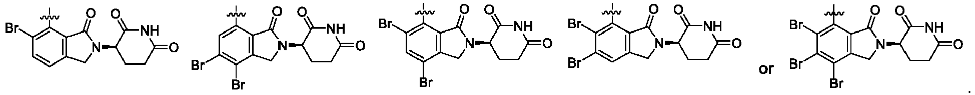

24. The compound of Formula (II) or salts, enantiomers, stereoisomers, solvates, isotopically enriched analogs, prodrugs, or polymorphs thereof as claimed in any one of claims 12-22, wherein LIN represents the structure of the following formula: 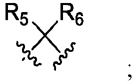 wherein R₅ and R₆ are the same or different and each independently represent:
hydrogen, fluorine, chlorine, bromine, iodine, optionally substituted methyl, or optionally substituted linear or branched C₂₋₃₀ alkyl,
wherein one or more groups R_{c} and/or one or more groups R_{d} and/or any combination of one or more groups R_{c} and R_{d} are optionally inserted into the backbone carbon chain of the linear or branched C₂₋₃₀ alkyl, wherein carbon-carbon bond between one or more pairs of adjacent carbon atoms in the backbone carbon chain is interrupted by the group R_{c}, R_{d}, or a combination of R_{c} and R_{d}; wherein each R_{c} is selected from the group consisting of O, N(Rₑ), C(O), C(O)O, S(O), S(O)₂, S(O)₂NH, NHS(O)₂, OC(O), C(O)N(Rₑ), N(Rₑ)C(O), or N(Rₑ)C(O)N(Rₑ), where each Rₑ independently represents H or C₁₋₆ alkyl, and in case that two or more groups R_{c} are inserted into the backbone carbon chain of the linear or branched C₂₋₃₀ alkyl group, the two or more groups R_{c} are not directly connected to each other; and wherein each R_{d} is selected from the group consisting of cycloalkylene (e.g., C₃₋₂₀ cycloalkylene), arylene (e.g., C₃₋₂₀ arylene), heterocyclylene (e.g., 4- to 20-membered heterocyclylene), heteroarylene (e.g., 4- to 20-membered heteroarylene), alkynylene (e.g., C₂₋₆ alkynylene), alkenylene (e.g., C₂₋₆ alkenylene), or any combination thereof, wherein the cycloalkylene, arylene, heterocyclylene, and heteroarylene are independently optionally substituted with a substituent selected from the group consisting of deuterium, optionally deuterated C₁₋₆ alkyl, optionally deuterated C₃₋₆ cycloalkyl, hydroxy, amino, mercapto, halogen, optionally deuterated C₁₋₆ alkoxy, optionally deuterated C₁₋₆ alkyl-NH-, halogenated C₁₋₆ alkyl, NH₂-C₁₋₆ alkylene, optionally deuterated C₁₋₆ alkyl-NHC(O)-, optionally deuterated C₁₋₆ alkyl-C(O)NH-, cyano, or any combination thereof; and
a hydrogen atom of methyl and hydrogen atom of one or more CH₂ of C₂₋₃₀ alkyl are optionally substituted with a substituent selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, or any combination thereof.

25. The compound of Formula (II) or salts, enantiomers, stereoisomers, solvates, isotopically enriched analogs, prodrugs, or polymorphs thereof as claimed in any one of claims 12-22, wherein R₅ and R₆ are the same or different and each independently represent hydrogen, fluorine, chlorine, bromine, or iodine, or represent the structure of the following formula:
-C₁₋₃₀ alkyl;
-(C(R^{a4})(R^{a5}))ₙ₁-(R_{c}-(C(R^{a6})(R^{a7}))ₙ₂)ₘ₄-H;
-(C(R^{a4})(R^{a5}))ₙ₁-(R_{c}-(C(R^{a6})(R^{a7}))ₙ₂)ₘ₄-(R_{c}-(C(R^{a8})(R^{a9}))ₙ₃)ₘ₅-H;
-(C(R^{a4})(R^{a5}))ₙ₁-(R_{c}-(C(R^{a6})(R^{a7}))ₙ₂)ₘ₄-(R_{c}-(C(R^{a8})(R^{a9}))ₙ₃)ₘ₅-(R_{c}-(C(R^{a10})(R^{a11}))ₙ₄)ₘ₆-H;
-(C(R^{a4})(R^{a5}))ₙ₁-(R_{d}-(C(R^{a6})(R^{a7}))ₙ₂)ₘ₄-H;
-(C(Ra⁴)(R^{a5}))ₙ₁-(R_{d}-(C(Ra⁶)(Ra⁷))ₙ₂)ₘ₄-(R_{d}-(C(R^{a8})(R^{a9}))ₙ₃)ₘ₅-H;
-(C(R^{a4})(R^{a5}))ₙ₁-(R_{d}-(C(R^{a6})(R^{a7}))ₙ₂)ₘ₄-(R_{d}-(C(R^{a8})(R^{a9}))ₙ₃)ₘ₅-(R_{d}-(C(R¹⁰)(R^{a11}))ₙ₄)ₘ₆-H;
-(C(R^{a4})(R^{a5}))ₙ₁-(R_{c}-R_{d}-(C(R^{a6})(R^{a7}))ₙ₂)ₘ₄-CH₃;
-(C(R^{a4})(R^{a5}))ₙ₁-(R_{c}-(C(R^{a6})(R^{a7}))ₙ₂)ₘ₄-(R_{d}-(C(R^{a8})(R^{a9}))ₙ₃)ₘ₅-H;
-(C(R^{a4})(R^{a5}))ₙ₁-(R_{d}-R_{c}-(C(R^{a6})(R^{a7}))ₙ₂)ₘ₄-H; or
-(C(R^{a4})(R^{a5}))ₙ₁-(R_{d}-(C(R^{a6})(R^{a7}))ₙ₂)ₘ₄-(R_{c}-(C(R^{a8})(R^{a9}))ₙ₃)ₘ₅-H;
wherein each R_{c} is selected from the group consisting of O, N(Rₑ), C(O), C(O)O, OC(O), S(O), S(O)₂, S(O)₂NH, NHS(O)₂, C(O)N(Rₑ), N(Rₑ)C(O), or N(Rₑ)C(O)N(Rₑ), wherein each Rₑ independently represents H or C₁₋₆ alkyl; and each R_{d} is selected from the group consisting of cycloalkylene (e.g., C₃₋₂₀cycloalkylene), arylene (e.g., C₃₋₂₀arylene), heterocyclylene (e.g., 4- to 20-membered heterocyclylene), heteroarylene (e.g., 4- to 20-membered heteroarylene), alkynylene (e.g., C₂₋₆ alkynylene), alkenylene (e.g., C₂₋₆ alkenylene ) or any combination thereof, wherein the cycloalkylene, arylene, heterocyclylene and heteroarylene are each independently optionally substituted with a substituent selected from the group consisting of deuterium, optionally deuterated C₁₋₆ alkyl, optionally deuterated C₃₋₆ cycloalkyl, hydroxy, amino, mercapto, halogen, optionally deuterated C₁₋₆ alkoxy, optionally deuterated C₁₋₆ alkyl-NH-, halogenated C₁₋₆ alkyl, NH₂-C₁₋₆ alkylene, optionally deuterated C₁₋₆ alkyl-NHC(O)-, optionally deuterated C₁₋₆ alkyl-C(O)NH-, cyano or any combination thereof;
a hydrogen atom of one or more CH₂ of C₁₋₃₀ alkyl is optionally substituted with a substituent selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, or any combination thereof;
R^{a4}, R^{a5}, R^{a6}, R^{a7}, R^{a8}, R^{a9}, R^{a10} and R^{a11} each independently represent H, deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, or optionally deuterated C₁₋₆ alkyl-C(O)NH-; and
n1, n2, n3, n4, m4, m5, and m6 each independently represent an integer of 1, 2, 3, 4, 5, 6, 7, 8, 9, 10, 11, 12, 13, 14, or 15.

26. The compound of Formula (II) or salts, enantiomers, stereoisomers, solvates, isotopically enriched analogs, prodrugs, or polymorphs thereof as claimed in claim 12, wherein R₅ and R₆ are the same or different and each independently represent the structure of the following formula:
- (C(R^{a4})(R^{a5}))ₙ₁-(O-(C(R^{a6})(R^{a7}))ₙ₂)ₘ₄-H;
- (C(R^{a4})(R^{a5}))ₙ₁-(O-(C(R^{a6})(R^{a7}))ₙ₂)ₘ₄-(O-(C(R^{a8})(R^{a9}))ₙ₃)ₘ₅-H;
- (C(R^{a4})(R^{a5}))ₙ₁-(O-(C(R^{a6})(R^{a7}))ₙ₂)ₘ₄-(O-(C(R^{a8})(R^{a9}))ₙ₃)ₘ₅-(O(C(R^{a10})(R^{a11}))ₙ₄)ₘ₆-H;
- (C(R^{a4})(R^{a5}))ₙ₁-(N(Rₑ)-(C(R^{a6})(R^{a7}))ₙ₂)ₘ₄-H;
- (C(R^{a4})(R^{a5}))ₙ₁-(N(Rₑ)-(C(R^{a6})(R^{a7}))ₙ₂)ₘ₄-(N(Rₑ)-(C(R^{a8})(R^{a9}))ₙ₃)ₘ₅-H;
- (C(R^{a4})(R^{a5}))ₙ₁-(N(Rₑ)-(C(R^{a6})(R^{a7}))ₙ₂)ₘ₄-(N(Rₑ)-(C(R^{a8})(R^{a9}))ₙ₃)ₘ₅-(N(Rₑ)-(C(R^{a10})(R^{a11}))ₙ₄)ₘ₆-H;
- (C(R^{a4})(R^{a5}))ₙ₁-(C(O)N(Rₑ)-(C(R^{a6})(R^{a7}))ₙ₂)ₘ₄-H;
- (C(R^{a4})(R^{a5}))ₙ₁-(C(O)N(Rₑ)-(C(R^{a6})(R^{a7}))ₙ₂)ₘ₄-(C(O)N(Rₑ)-(C(R^{a8})(R^{a9}))ₙ₃)ₘ₅-H;
- (C(R^{a4})(R^{a5}))ₙ₁-(C(O)N(Rₑ)-(C(R^{a6})(R^{a7}))ₙ₂)ₘ₄-(C(O)N(Rₑ)-(C(R^{a8})(R^{a9}))ₙ₃)ₘ₅-(C(O)N(Rₑ)-(C(R^{a10})(R^{a11}))ₙ₄)ₘ₆-H;
- (C(R^{a4})(R^{a5}))ₙ₁-(C(O)N(Rₑ)-(C(R^{a6})(R^{a7}))ₙ₂)ₘ₄-(O-(C(R^{a8})(R^{a9}))ₙ₃)ₘ₅-H;
- (C(R^{a4})(R^{a5}))ₙ₁-C(O)N(Rₑ)-(C(R^{a6})(R^{a7}))ₙ₂-(O-(C(R^{a8})(R^{a9}))ₙ₃)ₘ₄-H;
- (C(R^{a4})(R^{a5}))ₙ₁-(N(Rₑ)C(O)-(C(R^{a6})(R^{a7}))ₙ₂)ₘ₄-H;
- (C(R^{a4})(R^{a5}))ₙ₁-(N(Rₑ)C(O)-(C(R^{a6})(R^{a7}))ₙ₂)ₘ₄-(O-(C(R^{a8})(R^{a9}))ₙ₃)ₘ₅-H;
- (C(R^{a4})(R^{a5}))ₙ₁-(N(Rₑ)C(O)-(C(R^{a6})(R^{a7}))ₙ₂)ₘ₄-(O-(C(R^{a8})(R^{a9}))ₙ₃)ₘ₅-(O-(C(R^{a10})(R^{a11}))ₙ₄)ₘ₆-H;
- (C(R^{a4})(R^{a5}))ₙ₁-N(Rₑ)C(O)-(C(R^{a6})(R^{a7}))ₙ₂-(O-(C(R^{a8})(R^{a9}))ₙ₃)ₘ₄-H;
- (C(R^{a4})(R^{a5}))ₙ₁-N(Rₑ)C(O)N(Rₑ)-(C(R^{a6})(R^{a7}))ₙ₂-H;
- (C(R^{a4})(R^{a5}))ₙ₁-C(O)-(C(R^{a6})(R^{a7}))ₙ₂-H;
- (C(R^{a4})(R^{a5}))ₙ₁-(arylene-(C(R^{a6})(R^{a7}))ₙ₂)ₘ₄-H;
- (C(R^{a4})(R^{a5}))ₙ₁-(arylene-(C(R^{a6})(R^{a7}))ₙ₂)ₘ₄-arylene-(C(R^{a8})(R^{a9}))ₙ₃-H;
- (C(R^{a4})(R^{a5}))ₙ₁-(heterocyclylene-(C(R^{a6})(R^{a7}))ₙ₂)ₘ₄-H;
- (C(R^{a4})(R^{a5}))ₙ₁-(heterocyclylene-(C(R^{a6})(R^{a7}))ₙ₂)ₘ₄-(heterocyclylene-(C(R^{a8})(R^{a9}))ₙ₃)ₘ₅-H;
- (C(R^{a4})(R^{a5}))ₙ₁-(heteroarylene-(C(R^{a6})(R^{a7}))ₙ₂)ₘ₄-H;
- (C(R^{a4})(R^{as}))ₙ₁-(heteroarylene-(C(R^{a6})(R^{a7}))ₙ₂)ₘ₄-(heteroarylene-(C(R^{a8})(R^{a9}))ₙ₃)ₘ₅-H;
- (C(R^{a4})(R^{a5}))ₙ₁-(cycloalkylene-(C(R^{a6})(R^{a7}))ₙ₂)ₘ₄-H; or
- (C(R^{a4})(R^{a5}))ₙ₁-(cycloalkylene-(C(R^{a6})(R^{a7}))ₙ₂)ₘ₄-(cycloalkylene-(C(R^{a8})(R^{a9}))ₙ₃)ₘ₅-H;
wherein each Rₑ independently represents H or C₁₋₆ alkyl;
the cycloalkylene, arylene, heterocyclylene and heteroarylene are each independently optionally substituted with one or more substituents selected from the group consisting of deuterium, optionally deuterated C₁₋₆ alkyl, optionally deuterated C₃₋₆ cycloalkyl, hydroxy, amino, mercapto, halogen, optionally deuterated C₁₋₆ alkoxy, optionally deuterated C₁₋₆ alkyl-NH-, halogenated C₁₋₆ alkyl, NH₂-C₁₋₆ alkylene, optionally deuterated C₁₋₆ alkyl-NHC(O)-, optionally deuterated C₁₋₆ alkyl-C(O)NH-, cyano or any combination thereof;
R^{a4}, R^{a5}, R^{a6}, R^{a7}, R^{a8}, R^{a9}, R^{a10} and R^{a11} each independently represent H, deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, or optionally deuterated C₁₋₆ alkyl-C(O)NH-; and
n1, n2, n3, n4, m4, m5, and m6 each independently represent an integer of 1, 2, 3, 4, 5, 6, 7, 8, 9, 10, 11, 12, 13, 14, or 15.

27. The compound of Formula (II) or salts, enantiomers, stereoisomers, solvates, isotopically enriched analogs, prodrugs, or polymorphs thereof as claimed in claim 12, wherein R₅ and R₆ are the same or different and each independently represent the following groups:
H, fluorine, chlorine, bromine, iodine, -CH₃, -CH₂-CH₃, -(CH₂)₂-CH₃, -(CH₂)₃-CH₃, -(CH₂)₄-CH₃, -(CH₂)₅-CH₃, -(CH₂)₆-CH₃, -(CH₂)₇-CH₃, -(CH₂)₈-CH₃, -(CH₂)₉-CH₃, -(CH₂)₁₀-CH₃, -(CH₂)₁₁-CH₃,-(CH₂)₁₂-CH₃, -(CH₂)₁₃-CH₃, -(CH₂)₁₄-CH₃, -(CH₂)₁₅-CH₃, -(CH₂)₁₆-CH₃, -(CH₂)₁₇-CH₃, -(CH₂)₁₈-CH₃, -(CH₂)₁₉-CH₃, -(CH₂)₂₀-CH₃, -(CH₂)₂₁-CH₃, -(CH₂)₂₂-CH₃, -(CH₂)₂₅-CH₃, -(CH₂)₂₉-CH₃;-CH₂-O-CH₃, -CH₂-O-CH₂-CH₃, -CH₂-O-(CH₂)₂-CH₃, -(CH₂)₁-O-(CH₂)₃-CH₃, -(CH₂)₁-O-(CH₂)₄-CH₃, -(CH₂)₁-O-(CH₂)₅-CH₃, -(CH₂)₁-O-(CH₂)₆-CH₃, -(CH₂)₁-O-(CH₂)₇-CH₃, -(CH₂)₁-O-(CH₂)₈-CH₃, -(CH₂)₁-O-(CH₂)₉-CH₃, -(CH₂)₁-O-(CH₂)₁₀-CH₃, -(CH₂)₂-O-CH₃, -(CH₂)₂-O-CH₂-CH₃, -(CH₂)₂-O-(CH₂)₂-CH₃,-(CH₂)₂-O-(CH₂)₃-CH₃, -(CH₂)₂-O-(CH₂)₄-CH₃, -(CH₂)₂-O-(CH₂)₅-CH₃, -(CH₂)₂-O-(CH₂)₆-CH₃,-(CH₂)₂-O-(CH₂)₇-CH₃, -(CH₂)₂-O-(CH₂)₈-CH₃, -(CH₂)₂-O-(CH₂)₉-CH₃, -(CH₂)₂-O-(CH₂)₁₀-CH₃,-(CH₂)₂-O-(CH₂)₁₁-CH₃, -(CH₂)₂-O-(CH₂)₁₂-CH₃, -(CH₂)₃-O-CH₃, -(CH₂)₃-O-CH₂-CH₃, -(CH₂)₃-O-(CH₂)₂-CH₃, -(CH₂)₃-O-(CH₂)₃-CH₃, -(CH₂)₃-O-(CH₂)₄-CH₃, -(CH₂)₃-O-(CH₂)₅-CH₃, -(CH₂)₃-O-(CH₂)₆-CH₃, -(CH₂)₃-O-(CH₂)₇-CH₃, -(CH₂)₄-O-CH₃, -(CH₂)₄-O-CH₂-CH₃, -(CH₂)₄-O-(CH₂)₂-CH₃,-(CH₂)₄-O-(CH₂)₃-CH₃, -(CH₂)₄-O-(CH₂)₄-CH₃, -(CH₂)₄-O-(CH₂)₅-CH₃, -(CH₂)₄-O-(CH₂)₆-CH₃,-(CH₂)₅-O-CH₃, -(CH₂)₅-O-CH₂-CH₃, -(CH₂)₅-O-(CH₂)₂-CH₃, -(CH₂)₅-O-(CH₂)₃-CH₃, -(CH₂)₅-O-(CH₂)₄-CH₃, -(CH₂)₅-O-(CH₂)₅-CH₃, -(CH₂)₆-O-CH₃, -(CH₂)₆-O-CH₂-CH₃, -(CH₂)₆-O-(CH₂)₂-CH₃,-(CH₂)₆-O-(CH₂)₃-CH₃, -(CH₂)₆-O-(CH₂)₄-CH₃, -(CH₂)₇-O-CH₃, -(CH₂)₇-O-CH₂-CH₃, -(CH₂)₇-O-(CH₂)₂-CH₃, -(CH₂)₇-O-(CH₂)₃-CH₃, -(CH₂)₈-O-CH₃, -(CH₂)₈-O-CH₂-CH₃, -(CH₂)₈-O-(CH₂)₂-CH₃,-CH(CH₃)-O-CH₃, -CH(CH₃)-O-CH₂-CH₃, -CH(CH₃)-O-(CH₂)₂-CH₃, -CH(CH₃)-O-(CH₂)₃-CH₃,-CH(CH₃)-O-(CH₂)₄-CH₃, -CH(CH₃)-O-(CH₂)₅-CH₃, -CH(CH₃)-O-(CH₂)₆-CH₃, -CH(CH₃)-O-(CH₂)₇-CH₃, -CH(CH₃)-O-(CH₂)₈-CH₃, -CH(CH₃)-O-(CH₂)₉-CH₃, -CH(CH₃)-O-(CH₂)₁₀-CH₃, -CH₂-(O(CH₂)₂)₁-OCH₂CH₃, -CH₂-(O(CH₂)₂)₂-OCH₂CH₃, -CH₂-(O(CH₂)₂)₃-OCH₂CH₃, -CH₂-(O(CH₂)₂)₄-OCH₂CH₃, -CH₂-(O(CH₂)₂)₅-OCH₂CH₃, -CH₂-(O(CH₂)₂)₆-OCH₂CH₃, -CH₂-(O(CH₂)₂)₇-OCH₂CH₃,-CH₂-(O(CH₂)₂)₈-OCH₂CH₃, -CH₂-(O(CH₂)₂)₉-OCH₂CH₃, -CH₂-(O(CH₂)₂)₁₀-OCH₂CH₃, -CH₂-(O(CH₂)₂)₁-OCH₃, -CH₂-(O(CH₂)₂)₂-OCH₃, -CH₂-(O(CH₂)₂)₃-OCH₃, -CH₂-(O(CH₂)₂)₄-OCH₃, -CH₂-(O(CH₂)₂)₅-OCH₃, -CH₂-(O(CH₂)₂)₆-OCH₃, -CH₂-(O(CH₂)₂)₇-OCH₃, -CH₂-(O(CH₂)₂)₈-OCH₃, -CH₂-(O(CH₂)₂)₉-OCH₃, -CH₂-(O(CH₂)₂)₁₀-OCH₃, -(CH₂)₂-(O(CH₂)₂)₁-OCH₂CH₃, -(CH₂)₂-(O(CH₂)₂)₂-OCH₂CH₃, -(CH₂)₂-(O(CH₂)₂)₃-OCH₂CH₃, -(CH₂)₂-(O(CH₂)₂)₄-OCH₂CH₃, -(CH₂)₂-(O(CH₂)₂)₅-OCH₂CH₃, -(CH₂)₂-(O(CH₂)₂)₆-OCH₂CH₃, -(CH₂)₂-(O(CH₂)₂)₇-OCH₂CH₃, -(CH₂)₂-(O(CH₂)₂)₈-OCH₂CH₃, -(CH₂)₂-(O(CH₂)₂)₉-OCH₂CH₃, -(CH₂)₂-(O(CH₂)₂)₁₀-OCH₂CH₃, -(CH₂)₃-(O(CH₂)₂)₁-OCH₂CH₃, -(CH₂)₃-(O(CH₂)₂)₂-OCH₂CH₃, -(CH₂)₃-(O(CH₂)₂)₃-OCH₂CH₃, -(CH₂)₃-(O(CH₂)₂)₄-OCH₂CH₃, -(CH₂)₃-(O(CH₂)₂)₅-OCH₂CH₃, -(CH₂)₃-(O(CH₂)₂)₆-OCH₂CH₃, -(CH₂)₃-(O(CH₂)₂)₇-OCH₂CH₃, -(CH₂)₃-(O(CH₂)₂)₈-OCH₂CH₃, -(CH₂)₃-(O(CH₂)₂)₉-OCH₂CH₃, -(CH₂)₃-(O(CH₂)₂)₁₀-OCH₂CH₃, -(CH₂)₄-(O(CH₂)₂)₁-OCH₂CH₃, -(CH₂)₄-(O(CH₂)₂)₂-OCH₂CH₃, -(CH₂)₄-(O(CH₂)₂)₃-OCH₂CH₃, -(CH₂)₄-(O(CH₂)₂)₄-OCH₂CH₃, -(CH₂)₄-(O(CH₂)₂)₅-OCH₂CH₃, -(CH₂)₄-(O(CH₂)₂)₆-OCH₂CH₃, -(CH₂)₄-(O(CH₂)₂)₇-OCH₂CH₃, -(CH₂)₄-(O(CH₂)₂)₈-OCH₂CH₃, -(CH₂)₄-(O(CH₂)₂)₉-OCH₂CH₃, -(CH₂)₄-(O(CH₂)₂)₁₀-OCH₂CH₃, -CH₂-(O(CH₂)₃)₁-OCH₂CH₂CH₃, -CH₂-(O(CH₂)₃)₂-OCH₂CH₂CH₃, -CH₂-(O(CH₂)₃)₃-OCH₂CH₂CH₃, -CH₂-(O(CH₂)₃)₄-OCH₂CH₂CH₃, -CH₂-(O(CH₂)₃)₅-OCH₂CH₂CH₃, -CH₂-(O(CH₂)₃)₆-OCH₂CH₂CH₃, -CH₂-(O(CH₂)₃)₇-OCH₂CH₂CH₃, -CH₂-(O(CH₂)₃)₈-OCH₂CH₂CH₃, -CH₂-(O(CH₂)₃)₉-OCH₂CH₂CH₃, -CH₂-(O(CH₂)₃)₁₀-OCH₂CH₂CH₃, -(CH₂)₂-(O(CH₂)₃)₁-OCH₂CH₂CH₃, -(CH₂)₂-(O(CH₂)₃)₂-OCH₂CH₂CH₃, -(CH₂)₂-(O(CH₂)₃)₃-OCH₂CH₂CH₃,-(CH₂)₂-(O(CH₂)₃)₄-OCH₂CH₂CH₃, -(CH₂)₂-(O(CH₂)₃)₅-OCH₂CH₂CH₃, -(CH₂)₂-(O(CH₂)₃)₆-OCH₂CH₂CH₃, -(CH₂)₂-(O(CH₂)₃)₇-OCH₂CH₂CH₃, -(CH₂)₂-(O(CH₂)₃)₈-OCH₂CH₂CH₃, -(CH₂)₃-(O(CH₂)₃)₁-OCH₂CH₂CH₃, -(CH₂)₃-(O(CH₂)₃)₂-OCH₂CH₂CH₃, -(CH₂)₃-(O(CH₂)₃)₃-OCH₂CH₂CH₃,-(CH₂)₃-(O(CH₂)₃)₄-OCH₂CH₂CH₃, -(CH₂)₃-(O(CH₂)₃)₅-OCH₂CH₂CH₃, -(CH₂)₃-(O(CH₂)₃)₆-OCH₂CH₂CH₃, -(CH₂)₃-(O(CH₂)₃)₇-OCH₂CH₂CH₃, -(CH₂)₃-(O(CH₂)₃)₈-OCH₂CH₂CH₃, -CH₂-O-(CH₂)₂-O-(CH₂)₂-CH₃, -CH₂-(O(CH₂)₂)₂-(O(CH₂)₃)₁-OCH₂CH₂CH₃, -CH₂-(O(CH₂)₂)₃-(O(CH₂)₃)₂-OCH₂CH₂CH₃, -CH₂-(O(CH₂)₂)₄-(O(CH₂)₃)₃-OCH₂CH₂CH₃, -CH₂-(O(CH₂)₂)₅-(O(CH₂)₃)₄-OCH₂CH₂CH₃, -(CH₂)₂-O-(CH₂)₂-O-(CH₂)₂CH₃, -(CH₂)₂-(O(CH₂)₂)₂-(O(CH₂)₃)₁-OCH₂CH₂CH₃,-(CH₂)₂-(O(CH₂)₂)₃-(O(CH₂)₃)₂-OCH₂CH₂CH₃, -(CH₂)₂-(O(CH₂)₂)₄-(O(CH₂)₃)₃-OCH₂CH₂CH₃,-(CH₂)₂-(O(CH₂)₂)₅-(O(CH₂)₃)₄-OCH₂CH₂CH₃, -(CH₂)₃-O-(CH₂)₂-O-(CH₂)₂CH₃, -(CH₂)₃-(O(CH₂)₂)₂-(O(CH₂)₃)₁-OCH₂CH₂CH₃, -(CH₂)₃-(O(CH₂)₂)₃-(O(CH₂)₃)₂-OCH₂CH₂CH₃, -(CH₂)₃-(O(CH₂)₂)₄-(O(CH₂)₃)₃-OCH₂CH₂CH₃, -(CH₂)₃-(O(CH₂)₂)₅-(O(CH₂)₃)₄-OCH₂CH₂CH₃, -CH₂-O-(CH₂)₃-O-CH₂CH₃, -CH₂-(O(CH₂)₃)₂-(O(CH₂)₂)₁-O-CH₂CH₃, -CH₂-(O(CH₂)₃)₃-(O(CH₂)₂)₂-O-CH₂CH₃, -CH₂-(O(CH₂)₃)₄-(O(CH₂)₂)₃-O-CH₂CH₃, -CH₂-(O(CH₂)₃)₅-(O(CH₂)₂)₄-O-CH₂CH₃, -(CH₂)₂-O-(CH₂)₃-O-CH₂CH₃, -(CH₂)₂-(O(CH₂)₃)₂-(O(CH₂)₂)₁-O-CH₂CH₃, -(CH₂)₂-(O(CH₂)₃)₃-(O(CH₂)₂)₂-O-CH₂CH₃,-(CH₂)₂-(O(CH₂)₃)₄-(O(CH₂)₂)₃-O-CH₂CH₃, -(CH₂)₂-(O(CH₂)₃)₅-(O(CH₂)₂)₄-O-CH₂CH₃, -(CH₂)₃-O-(CH₂)₃-O-CH₂CH₃, -(CH₂)₃-(O(CH₂)₃)₂-(O(CH₂)₂)₁-O-CH₂CH₃, -(CH₂)₃-(O(CH₂)₃)₃-(O(CH₂)₂)₂-O-CH₂CH₃, -(CH₂)₃-(O(CH₂)₃)₄-(O(CH₂)₂)₃-O-CH₂CH₃, -(CH₂)₃-(O(CH₂)₃)₅-(O(CH₂)₂)₄-O-CH₂CH₃,-CH₂-O-(CH₂)₂-O-CH₃, -(CH₂)₂-O-(CH₂)₂-O-CH₃, -(CH₂)₂-(O(CH₂)₂)₂-O-(CH₂)₂CH₃, -(CH₂)₂-(O(CH₂)₂)₃-O-(CH₂)₂CH₃, -(CH₂)₂-(O(CH₂)₂)₄-O-(CH₂)₂CH₃, -(CH₂)₅-(O(CH₂)₂)₂-O-(CH₂)₄CH₃,-(CH₂)₅-(O(CH₂)₂)₂-O-(CH₂)₅CH₃, -(CH₂)₁-N(Rₑ)-CH₃, -(CH₂)₁-N(Rₑ)-(CH₂)₁-CH₃, -(CH₂)₁-N(Rₑ)-(CH₂)₂-CH₃, -(CH₂)₁-N(Rₑ)-(CH₂)₃-CH₃, -(CH₂)₁-N(Rₑ)-(CH₂)₄-CH₃, -(CH₂)₁-N(Rₑ)-(CH₂)₅-CH₃,-(CH₂)₁-N(Rₑ)-(CH₂)₆-CH₃, -(CH₂)₁-N(Rₑ)-(CH₂)₇-CH₃, -(CH₂)₁-N(Rₑ)-(CH₂)₈-CH₃, -(CH₂)₁-N(Rₑ)-(CH₂)₉-CH₃, -(CH₂)₂-N(Rₑ)-CH₃, -(CH₂)₂-N(Rₑ)-(CH₂)₁-CH₃, -(CH₂)₂-N(Rₑ)-(CH₂)₂-CH₃, -(CH₂)₂-N(Rₑ)-(CH₂)₃-CH₃, -(CH₂)₂-N(Rₑ)-(CH₂)₄-CH₃, -(CH₂)₂-N(Rₑ)-(CH₂)₅-CH₃, -(CH₂)₂-N(Rₑ)-(CH₂)₆-CH₃, -(CH₂)₂-N(Rₑ)-(CH₂)₇-CH₃, -(CH₂)₂-N(Rₑ)-(CH₂)₈-CH₃, -(CH₂)₂-N(Rₑ)-(CH₂)₉-CH₃, -(CH₂)₂-N(Rₑ)-(CH₂)₁₀-CH₃, -(CH₂)₂-N(Rₑ)-(CH₂)₁₁-CH₃, -(CH₂)₃-N(Rₑ)-CH₃, -(CH₂)₃-N(Rₑ)-(CH₂)₁-CH₃,-(CH₂)₃-N(Rₑ)-(CH₂)₂-CH₃, -(CH₂)₄-N(Rₑ)-CH₃, -(CH₂)₄-N(Rₑ)-(CH₂)₁-CH₃, -(CH₂)₄-N(Rₑ)-(CH₂)₂-CH₃, -(CH₂)₄-N(Rₑ)-(CH₂)₃-CH₃, -(CH₂)₅-N(Rₑ)-CH₃, -(CH₂)₅-N(Rₑ)-(CH₂)₁-CH₃, -(CH₂)₅-N(Rₑ)-(CH₂)₂-CH₃, -(CH₂)₅-N(Rₑ)-(CH₂)₃-CH₃, -(CH₂)₅-N(Rₑ)-(CH₂)₄-CH₃, -(CH₂)₆-N(Rₑ)-CH₃, -(CH₂)₆-N(Rₑ)-(CH₂)₁-CH₃, -(CH₂)₆-N(Rₑ)-(CH₂)₂-CH₃, -(CH₂)₇-N(Rₑ)-CH₃, -(CH₂)₇-N(Rₑ)-(CH₂)₁-CH₃,-(CH₂)₇-N(Rₑ)-(CH₂)₂-CH₃, -(CH₂)₈-N(Rₑ)-CH₃, -(CH₂)₈-N(Rₑ)-(CH₂)₁-CH₃, -(CH₂)₈-N(Rₑ)-(CH₂)₂-CH₃, -CH(CH₃)-N(Rₑ)-CH₃, -CH(CH₃)-N(Rₑ)-(CH₂)₁-CH₃, -CH(CH₃)-N(Rₑ)-(CH₂)₂-CH₃, -CH(CH₃)-N(Rₑ)-(CH₂)₃-CH₃, -CH(CH₃)-N(Rₑ)-(CH₂)₄-CH₃, -CH(CH₃)-N(Rₑ)-(CH₂)₅-CH₃, -CH(CH₃)-N(Rₑ)-(CH₂)₆-CH₃, -CH(CH₃)-N(Rₑ)-(CH₂)₇-CH₃, -CH(CH₃)-N(Rₑ)-(CH₂)₈-CH₃, -CH(CH₃)-N(Rₑ)-(CH₂)₉-CH₃, -CH₂C(O)NHCH₃, -(CH₂)₂C(O)NHCH₂CH₃, -(CH₂)₂C(O)NH(CH₂)₂-CH₃,-(CH₂)₂C(O)NH(CH₂)₃-CH₃, -(CH₂)₂C(O)NH(CH₂)₄-CH₃, -(CH₂)₃C(O)NH(CH₂)₂-CH₃,-(CH₂)₃C(O)NH(CH₂)₃-CH₃, -(CH₂)₄C(O)NH(CH₂)₃-CH₃, -(CH₂)₅C(O)NH(CH₂)₄-CH₃,-(CH₂)₆C(O)NH(CH₂)₆-CH₃, -(CH₂)₆C(O)NH(CH₂)₅-CH₃, -(CH₂)₇C(O)NH(CH₂)₆-CH₃,-(CH₂)₈C(O)NH(CH₂)₇-CH₃, U-(CH₂)₉C(O)NH(CH₂)₈-CH₃, -(CH₂)₁₀C(O)NH(CH₂)₉-CH₃,-(CH₂)₂C(O)NH(CH₂)₂-O-CH₂-CH₃, -CH₂NHC(O)CH₃, -(CH₂)₂NHC(O)CH₂CH₃,-(CH₂)₂NHC(O)(CH₂)₂-CH₃, -(CH₂)₂NHC(O)(CH₂)₃-CH₃, -(CH₂)₂NHC(O)(CH₂)₄-CH₃,-(CH₂)₃NHC(O)(CH₂)₂-CH₃, -(CH₂)₃NHC(O)(CH₂)₃-CH₃, -(CH₂)₄NHC(O)(CH₂)₃-CH₃,-(CH₂)₅NHC(O)(CH₂)₄-CH₃, -(CH₂)₆NHC(O)(CH₂)₆-CH₃, -(CH₂)₆NHC(O)(CH₂)₅-CH₃,-(CH₂)₇NHC(O)(CH₂)₆-CH₃, -(CH₂)₈NHC(O)(CH₂)₇-CH₃, -(CH₂)₉NHC(O)(CH₂)₈-CH₃,-(CH₂)₁₀NHC(O)(CH₂)₉-CH₃, -(CH₂)₄NHC(O)(CH₂)₇-CH₃, -(CH₂)₂NHC(O)(CH₂)₂-O-CH₂-CH₃,-(CH₂)₄NHC(O)CH₃, -CH₂-piperidinylene-CH₃, -CH₂-piperidinylene-CH₂-CH₃, -CH₂-piperidinylene-(CH₂)₂-CH₃, -CH₂-piperidinylene-(CH₂)₃-CH₃, -CH₂-piperidinylene-(CH₂)₄-CH₃, -CH₂-piperidinylene-(CH₂)₅-CH₃, -CH₂-piperidinylene-(CH₂)₆-CH₃, -CH₂-piperidinylene-(CH₂)₇-CH₃,-(CH₂)₂-piperidinylene-CH₃, -(CH₂)₂-piperidinylene-CH₂-CH₃, -(CH₂)₂-piperidinylene-(CH₂)₂-CH₃,-(CH₂)₂-piperidinylene-(CH₂)₃-CH₃, -(CH₂)₂-piperidinylene-(CH₂)₄-CH₃, -(CH₂)₂-piperidinylene-(CH₂)₅-CH₃, -(CH₂)₂-piperidinylene-(CH₂)₆-CH₃, -(CH₂)₂-piperidinylene-(CH₂)₇-CH₃, -(CH₂)₃-piperidinylene-CH₃, -(CH₂)₃-piperidinylene-CH₂-CH₃, -(CH₂)₃-piperidinylene-(CH₂)₂-CH₃, -(CH₂)₃-piperidinylene-(CH₂)₃-CH₃, -(CH₂)₃-piperidinylene-(CH₂)₄-CH₃, -(CH₂)₃-piperidinylene-(CH₂)₅-CH₃, -(CH₂)₃-piperidinylene-(CH₂)₆-CH₃, -(CH₂)₃-piperidinylene-(CH₂)₇-CH₃, -(CH₂)₄-piperidinylene-CH₃, -(CH₂)₄-piperidinylene-CH₂-CH₃, -(CH₂)₄-piperidinylene-(CH₂)₂-CH₃, -(CH₂)₄-piperidinylene-(CH₂)₃-CH₃, -(CH₂)₄-piperidinylene-(CH₂)₄-CH₃, -(CH₂)₄-piperidinylene-(CH₂)₅-CH₃, -(CH₂)₄-piperidinylene-(CH₂)₆-CH₃, -(CH₂)₄-piperidinylene-(CH₂)₇-CH₃, -(CH₂)₅-piperidinylene-CH₃,-(CH₂)₅-piperidinylene-CH₂-CH₃, -(CH₂)₅-piperidinylene-(CH₂)₂-CH₃, -(CH₂)₅-piperidinylene-(CH₂)₃-CH₃, -(CH₂)₅-piperidinylene-(CH₂)₄-CH₃, -(CH₂)₅-piperidinylene-(CH₂)₅-CH₃, -(CH₂)₅-piperidinylene-(CH₂)₆-CH₃, -(CH₂)₅-piperidinylene-(CH₂)₇-CH₃, -(CH₂)₆-piperidinylene-CH₃,-(CH₂)₆-piperidinylene-CH₂-CH₃, -(CH₂)₆-piperidinylene-(CH₂)₂-CH₃, -(CH₂)₆-piperidinylene-(CH₂)₃-CH₃, -(CH₂)₆-piperidinylene-(CH₂)₄-CH₃, -(CH₂)₆-piperidinylene-(CH₂)₅-CH₃, -(CH₂)₆-piperidinylene-(CH₂)₆-CH₃, -(CH₂)₆-piperidinylene-(CH₂)₇-CH₃, -(CH₂)₇-piperidinylene-CH₃,-(CH₂)₇-piperidinylene-CH₂-CH₃, -(CH₂)₇-piperidinylene-(CH₂)₂-CH₃, -(CH₂)₇-piperidinylene-(CH₂)₃-CH₃, -(CH₂)₇-piperidinylene-(CH₂)₇-CH₃, -(CH₂)₈-piperidinylene-CH₃, -(CH₂)₈-piperidinylene-CH₂-CH₃, -(CH₂)₈-piperidinylene-(CH₂)₂-CH₃, -(CH₂)₈-piperidinylene-(CH₂)₃-CH₃, -(CH₂)₈-piperidinylene-(CH₂)₄-CH₃, -(CH₂)₈-piperidinylene-(CH₂)₅-CH₃, -(CH₂)₈-piperidinylene-(CH₂)₆-CH₃, -(CH₂)₈-piperidinylene-(CH₂)₇-CH₃, -CH₂-N(Rₑ)-CH₂-piperidinylene-CH₃, -CH₂-N(Rₑ)-CH₂-piperidinylene-CH₃, -CH₂-N(Rₑ)-CH₂-piperidinylene-(CH₂)₂-CH₃, -CH₂-N(Rₑ)-CH₂-piperidinylene-(CH₂)₃-CH₃, -CH₂-N(Rₑ)-CH₂-piperidinylene-(CH₂)₄-CH₃, -CH₂-N(Rₑ)-CH₂-piperidinylene-(CH₂)₅-CH₃, -CH₂-N(Rₑ)-CH₂-piperidinylene-(CH₂)₆-CH₃, -CH₂-N(Rₑ)-CH₂-piperidinylene-(CH₂)₇-CH₃,-CH₂-N(Rₑ)-(CH₂)₂-piperidinylene-CH₃, -CH₂-N(Rₑ)-(CH₂)₂-piperidinylene-CH₂-CH₃, -CH₂-N(Rₑ)-(CH₂)₂-piperidinylene-(CH₂)₂-CH₃, -CH₂-N(Rₑ)-(CH₂)₂-piperidinylene-(CH₂)₃-CH₃, -CH₂-N(Rₑ)-(CH₂)₂-piperidinylene-(CH₂)₄-CH₃, -CH₂-N(Rₑ)-(CH₂)₂-pipendinylene-(CH₂)₅-CH₃, -CH₂-N(Rₑ)-(CH₂)₂-piperidinylene-(CH₂)₆-CH₃, -CH₂-N(Rₑ)-(CH₂)₂-piperidinylene-(CH₂)₇-CH₃, -(CH₂)₂-N(Rₑ)-CH₂-piperidinylene-CH₃, -(CH₂)₂-N(Rₑ)-CH₂-piperidinylene-CH₂-CH₃, -(CH₂)₂-N(Rₑ)-CH₂-piperidinylene-(CH₂)₂-CH₃, -(CH₂)₂-N(Rₑ)-CH₂-piperidinylene-(CH₂)₃-CH₃, -(CH₂)₂-N(Rₑ)-CH₂-piperidinylene-(CH₂)₄-CH₃, -(CH₂)₂-N(Rₑ)-CH₂-piperidinylene-(CH₂)₅-CH₃, -(CH₂)₂-N(Rₑ)-CH₂-piperidinylene-(CH₂)₆-CH₃, -(CH₂)₂-N(Rₑ)-CH₂-piperidinylene-(CH₂)₇-CH₃, -(CH₂)₃-N(Rₑ)-CH₂-piperidinylene-CH₃, -(CH₂)₃-N(Rₑ)-CH₂-piperidinylene-CH₂-CH₃, -(CH₂)₃-N(Rₑ)-CH₂-piperidinylene-(CH₂)₂-CH₃, -(CH₂)₃-N(Rₑ)-CH₂-piperidinylene-(CH₂)₇-CH₃, -(CH₂)₄-N(Rₑ)-CH₂-piperidinylene-CH₃, -(CH₂)₄-N(Rₑ)-CH₂-piperidinylene-CH₂-CH₃, -(CH₂)₄-N(Rₑ)-CH₂-piperidinylene-(CH₂)₂-CH₃,-(CH₂)₄-N(Rₑ)-CH₂-piperidinylene-(CH₂)₇-CH₃, -(CH₂)₅-N(Rₑ)-CH₂-piperidinylene-(CH₂)₂-CH₃,-(CH₂)₅-N(Rₑ)-CH₂-piperidinylene-(CH₂)₇-CH₃, -(CH₂)₆-N(Rₑ)-CH₂-piperidinylene-(CH₂)₂-CH₃,-(CH₂)₆-N(Rₑ)-CH₂-piperidinylene-(CH₂)₇-CH₃, -(CH₂)₇-N(Rₑ)-CH₂-piperidinylene-(CH₂)₂-CH₃,-(CH₂)₇-N(Rₑ)-CH₂-piperidinylene-(CH₂)₇-CH₃, -(CH₂)₈-N(Rₑ)-CH₂-piperidinylene-CH₃, -(CH₂)₈-N(Rₑ)-CH₂-piperidinylene-CH₂-CH₃, -(CH₂)₈-N(Rₑ)-CH₂-piperidinylene-(CH₂)₂-CH₃, -(CH₂)₈-N(Rₑ)-CH₂-piperidinylene-(CH₂)₃-CH₃, -(CH₂)₈-N(Rₑ)-CH₂-piperidinylene-(CH₂)₄-CH₃, -(CH₂)₈-N(Rₑ)-CH₂-piperidinylene-(CH₂)₅-CH₃, -(CH₂)₈-N(Rₑ)-CH₂-piperidinylene-(CH₂)₆-CH₃, -(CH₂)₈-N(Rₑ)-CH₂-piperidinylene-(CH₂)₇-CH₃, -CH₂-piperazinylene-CH₃, -CH₂-piperazinylene-CH₂-CH₃, -CH₂-piperazinylene-(CH₂)₂-CH₃, -CH₂-piperazinylene-(CH₂)₃-CH₃, -CH₂-piperazinylene-(CH₂)₄-CH₃,-CH₂-piperazinylene-(CH₂)₅-CH₃, -CH₂-piperazinylene-(CH₂)₆-CH₃, -CH₂-piperazinylene-(CH₂)₇-CH₃, -(CH₂)₂-piperazinylene-CH₃, -(CH₂)₂-piperazinylene-CH₂-CH₃, -(CH₂)₂-piperazinylene-(CH₂)₂-CH₃,-(CH₂)₂-piperazinylene-(CH₂)₃-CH₃, -(CH₂)₂-piperazinylene-(CH₂)₄-CH₃, -(CH₂)₂-piperazinylene-(CH₂)₅-CH₃, -(CH₂)₂-piperazinylene-(CH₂)₆-CH₃, -(CH₂)₂-piperazinylene-(CH₂)₇-CH₃, -(CH₂)₃-piperazinylene-CH₃, -(CH₂)₃-piperazinylene-CH₂-CH₃, -(CH₂)₃-piperazinylene-(CH₂)₂-CH₃, -(CH₂)₃-piperazinylene-(CH₂)₃-CH₃, -(CH₂)₃-piperazinylene-(CH₂)₄-CH₃, -(CH₂)₃-piperazinylene-(CH₂)₅-CH₃, -(CH₂)₃-piperazinylene-(CH₂)₆-CH₃, -(CH₂)₃-piperazinylene-(CH₂)₇-CH₃, -(CH₂)₄-piperazinylene-CH₃, -(CH₂)₄-piperazinylene-CH₂-CH₃, -(CH₂)₄-piperazinylene-(CH₂)₂-CH₃, -(CH₂)₄-piperazinylene-(CH₂)₃-CH₃, -(CH₂)₄-piperazinylene-(CH₂)₄-CH₃, -(CH₂)₄-piperazinylene-(CH₂)₅-CH₃, -(CH₂)₄-piperazinylene-(CH₂)₆-CH₃, -(CH₂)₄-piperazinylene-(CH₂)₇-CH₃, -(CH₂)₅-piperazinylene-CH₃,-(CH₂)₅-piperazinylene-CH₂-CH₃, -(CH₂)₅-piperazinylene-(CH₂)₂-CH₃, -(CH₂)₅-piperazinylene-(CH₂)₃-CH₃, -(CH₂)₅-piperazinylene-(CH₂)₄-CH₃, -(CH₂)₅-piperazinylene-(CH₂)₅-CH₃, -(CH₂)₅-piperazinylene-(CH₂)₆-CH₃, -(CH₂)₅-piperazinylene-(CH₂)₇-CH₃, -(CH₂)₆-piperazinylene-CH₃,-(CH₂)₆-piperazinylene-CH₂-CH₃, -(CH₂)₆-piperazinylene-(CH₂)₂-CH₃, -(CH₂)₆-piperazinylene-(CH₂)₃-CH₃, -(CH₂)₆-piperazinylene-(CH₂)₄-CH₃, -(CH₂)₆-piperazinylene-(CH₂)₅-CH₃, -(CH₂)₆-piperazinylene-(CH₂)₆-CH₃, -(CH₂)₆-piperazinylene-(CH₂)₇-CH₃, -(CH₂)₇-piperazinylene-CH₃,-(CH₂)₇-piperazinylene-CH₂-CH₃, -(CH₂)₇-piperazinylene-(CH₂)₂-CH₃, -(CH₂)₇-piperazinylene-(CH₂)₃-CH₃, -(CH₂)₇-piperazinylene-(CH₂)₇-CH₃, -(CH₂)₈-piperazinylene-CH₃, -(CH₂)₈-piperazinylene-CH₂-CH₃, -(CH₂)₈-piperazinylene-(CH₂)₂-CH₃, -(CH₂)₈-piperazinylene-(CH₂)₃-CH₃, -(CH₂)₈-piperazinylene-(CH₂)₄-CH₃, -(CH₂)₈-piperazinylene-(CH₂)₅-CH₃, -(CH₂)₈-piperazinylene-(CH₂)₆-CH₃, or -(CH₂)₈-piperazinylene-(CH₂)₇-CH₃;
wherein the piperidinylene and piperazinylene are each independently optionally substituted with a substituent selected from the group consisting of deuterium, optionally deuterated C₁₋₆ alkyl, optionally deuterated C₃₋₆ cycloalkyl, hydroxy, amino, mercapto, nitro, halogen, optionally deuterated C₁₋₆ alkoxy, optionally deuterated C₁₋₆ alkyl-NH-, halogenated C₁₋₆ alkyl, NH₂-C₁₋₆ alkylene, optionally deuterated C₁₋₆ alkyl-NHC(O)-, optionally deuterated C₁₋₆ alkyl-C(O)NH-, cyano or any combination thereof;
a hydrogen atom of CH₃ of the groups and a hydrogen atom of one or more CH₂ of the groups are optionally substituted with a substituent selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, or any combination thereof;
each Rₑ independently represents H or C₁₋₆ alkyl; and
n1, n2, n3, n4, m4, m5, and m6 each independently represent an integer of 1, 2, 3, 4, 5, 6, 7, 8, 9, 10, 11, 12, 13, 14, or 15.

28. The compound of Formula (II) or salts, enantiomers, stereoisomers, solvates, isotopically enriched analogs, prodrugs, or polymorphs thereof as claimed in any one of claims 12-27, wherein -R_{f}-LIN-represents the following groups:
-CH₂-, -O-CH₂-**, -NHC(O)-CH₂-**, -C(O)NH-CH₂-**, -NH-CH₂-**, -N(CH₃)-CH₂-**,-N(CH₃)-(CH₂)₂-N(CH₃)-CH₂-**, -N(CH₃)-(CH₂)₃-N(CH₃)-CH₂-**, -NH-(CH₂)₂-N(CH₃)-CH₂-**,-N(CH₃)-(CH₂)₃-NH-CH₂-**, -CH₂-NHC(O)-CH₂-**, -CH₂-C(O)NH-CH₂-**, -CH₂-NH-CH₂-**, -O-(CH₂)₂-N(CH₃)-CH₂-**, -O-(CH₂)₂-NH-CH₂-**, or -CH₂-N(CH₃) -CH₂-**, or 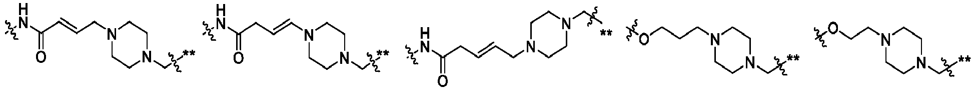 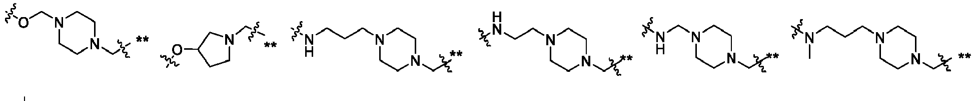 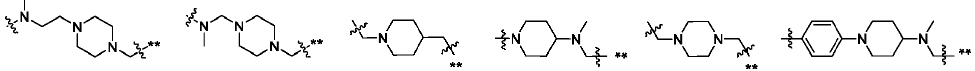 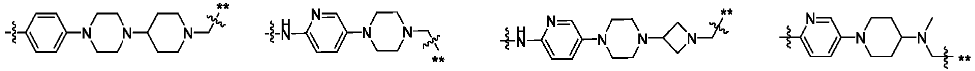 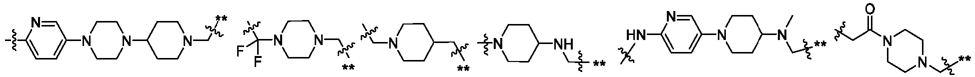 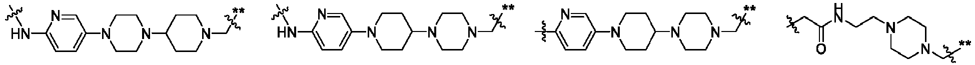 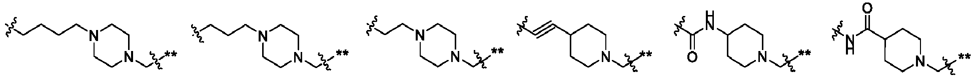 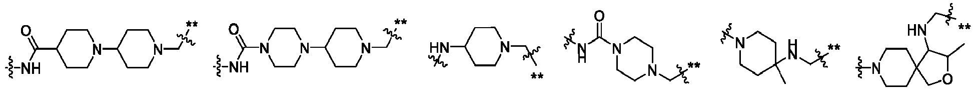 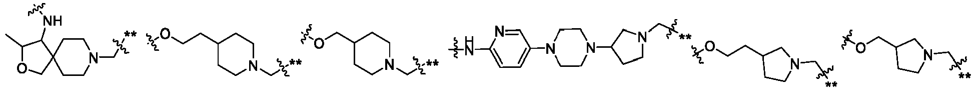 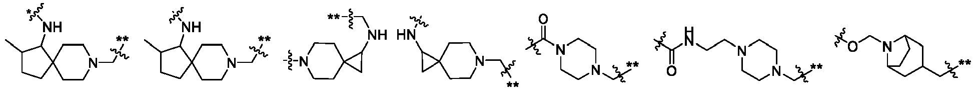 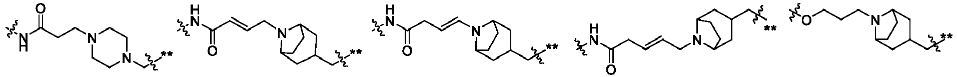 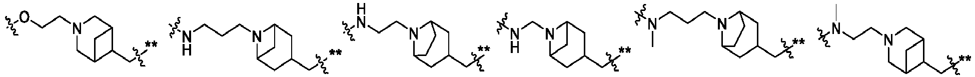 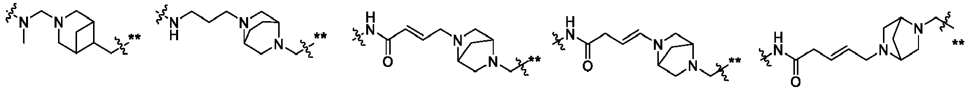 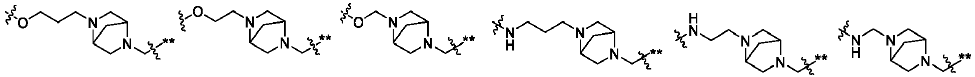 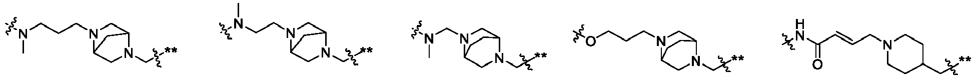 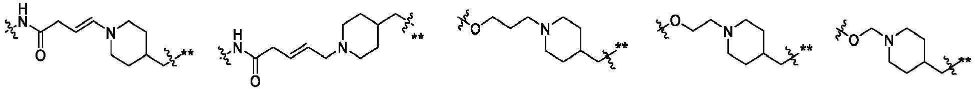 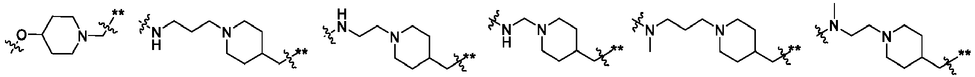 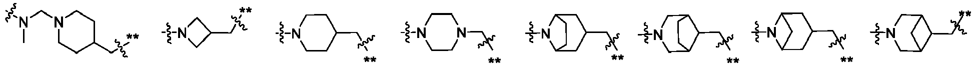 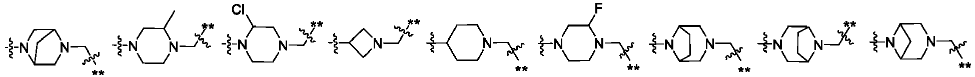 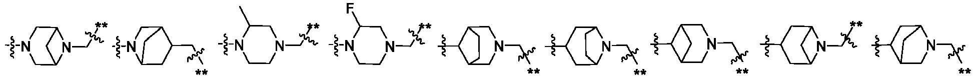 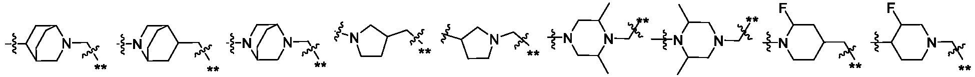 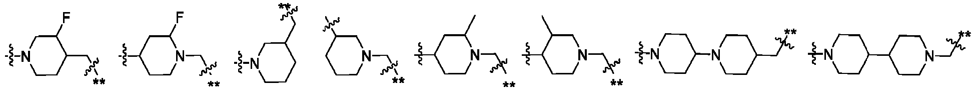 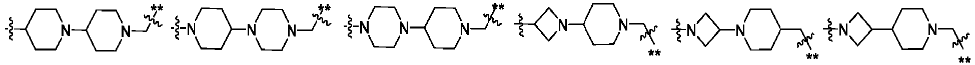 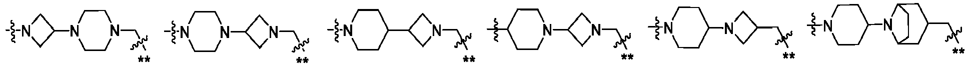 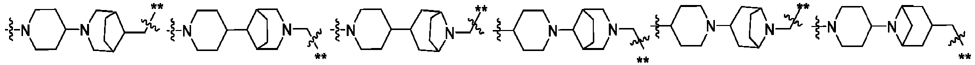 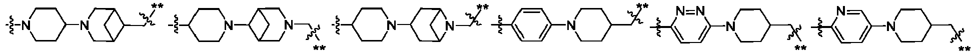 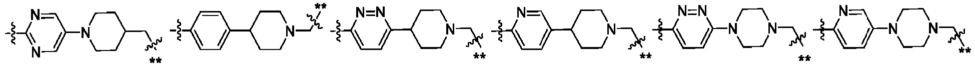 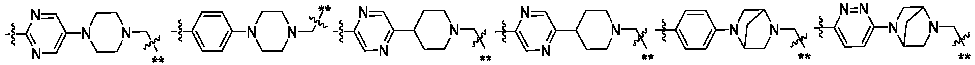 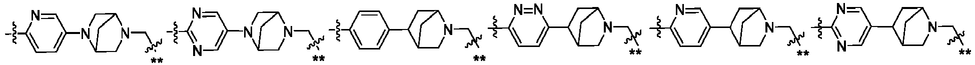 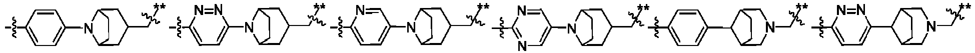 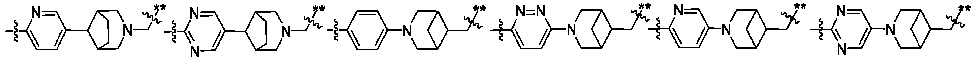 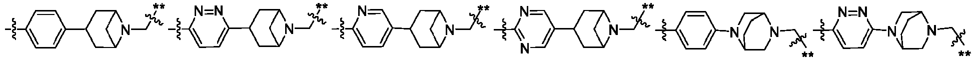 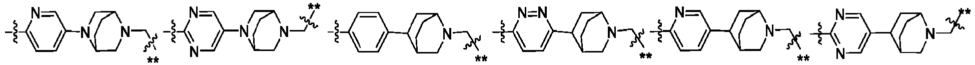 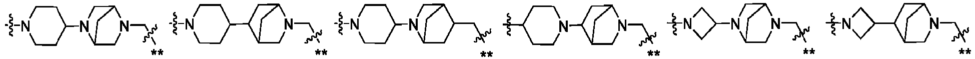 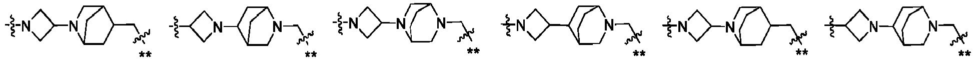 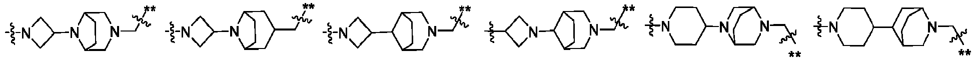 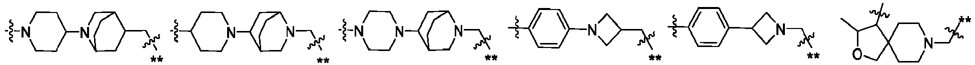 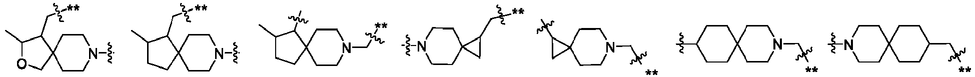 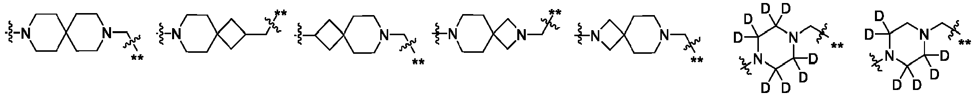 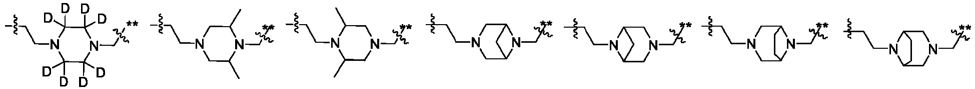 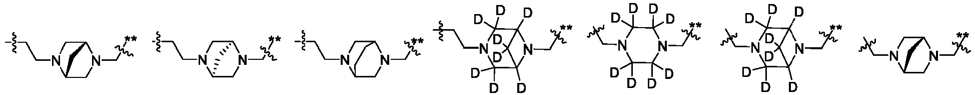 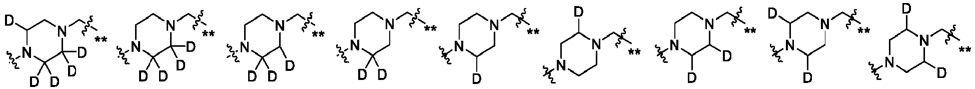 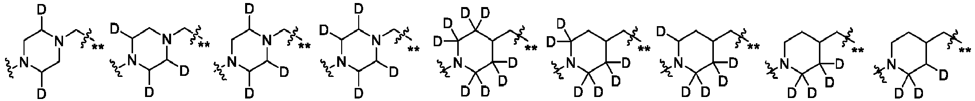 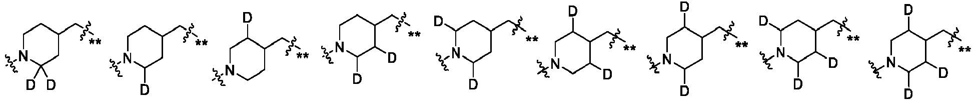 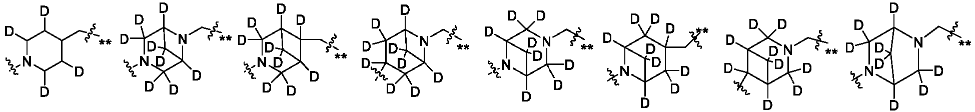 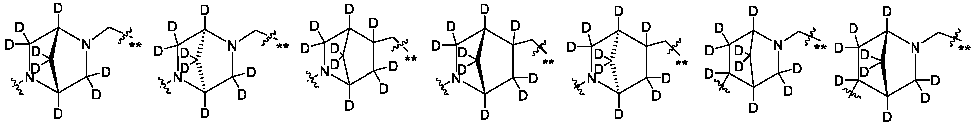 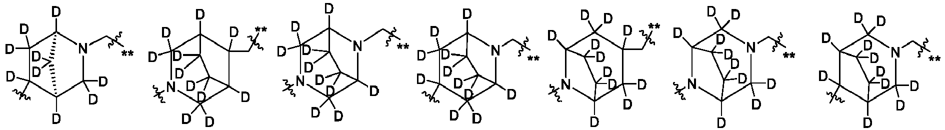 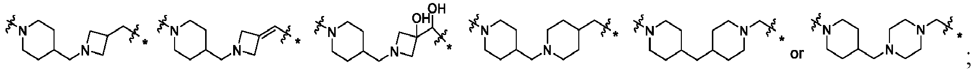 wherein the groups are optionally substituted with one or more substituents selected from the group consisting of deuterium, optionally deuterated C₁₋₆ alkyl, optionally halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or any combination thereof; and symbol ** indicates the point of attachment to ULM.

29. The compound of Formula (II) or salts, enantiomers, stereoisomers, solvates, isotopically enriched analogs, prodrugs, or polymorphs thereof as claimed in claim 12, which is also of Formula (II-1), Formula (II-2), Formula (II-3), Formula (II-4), Formula (II-5), Formula (II-6), Formula (II-7), Formula (II-8), Formula (II-9), Formula (II-10), Formula (II-11), Formula (II-12), Formula (II-13), Formula (II-14), Formula (II-15), Formula (II-16), Formula (II-17), Formula (II-18), Formula (II-19), Formula (II-20), Formula (II-21), Formula (II-22), Formula (II-23), Formula (II-24), Formula (II-25), Formula (II-26), Formula (II-27), Formula (II-28), Formula (II-29), Formula (II-30), Formula (II-31), Formula (II-32), Formula (II-33), Formula (II-34), Formula (II-35), Formula (II-36), Formula (II-37), Formula (II-38), Formula (II-39), Formula (II-40), Formula (II-41), Formula (II-42), Formula (II-43), Formula (II-44), Formula (II-45), Formula (II-46), Formula (II-47), Formula (II-48), Formula (II-49), Formula (II-50), Formula (II-51), Formula (II-52), Formula (II-53), Formula (II-55), Formula (II-56), Formula (II-57), Formula (II-58), Formula (II-59), Formula (II-60), Formula (II-61), Formula (II-62), Formula (II-63), Formula (II-64), Formula (II-65), or Formula (II-66): 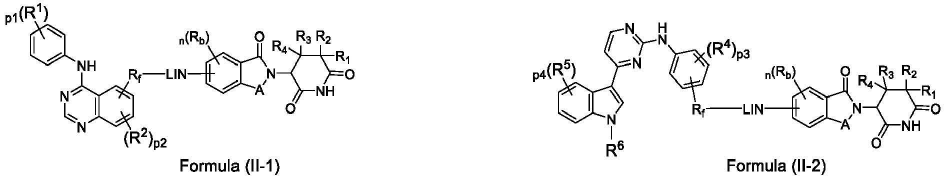 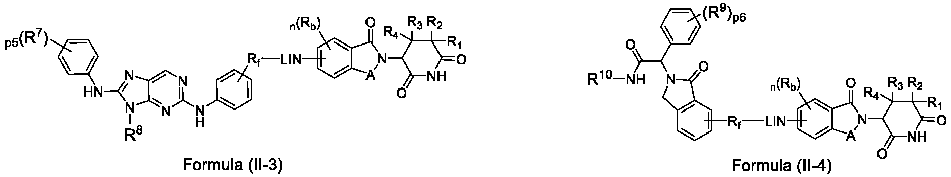 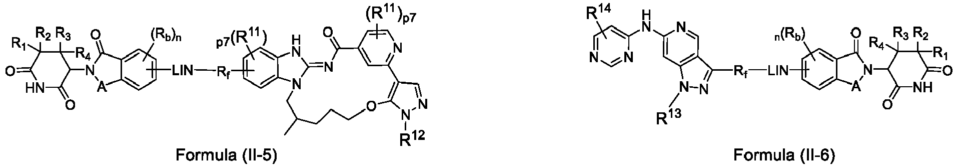 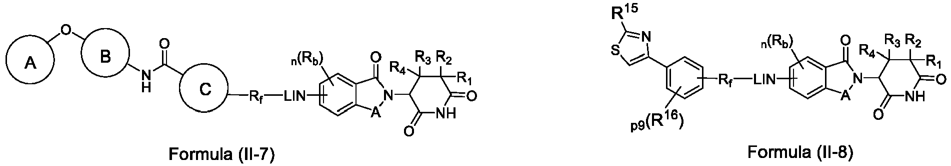 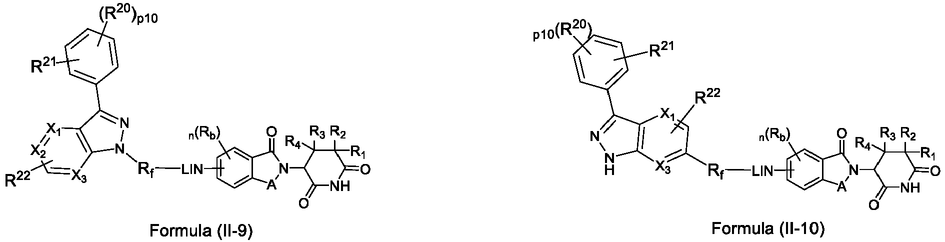 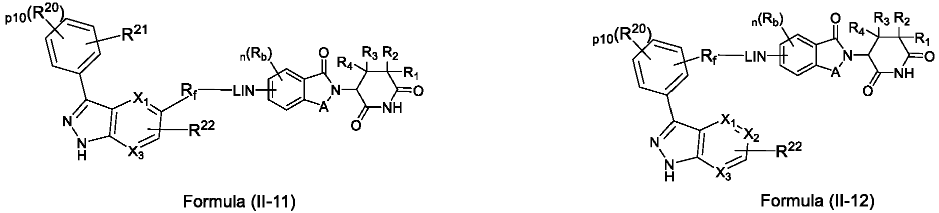 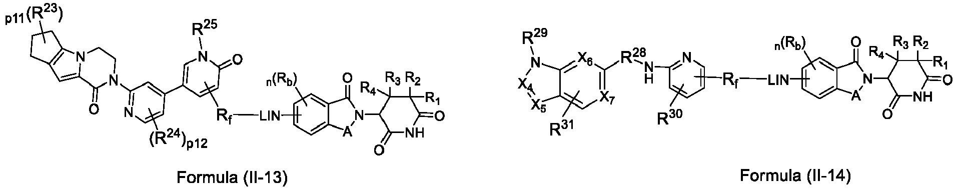 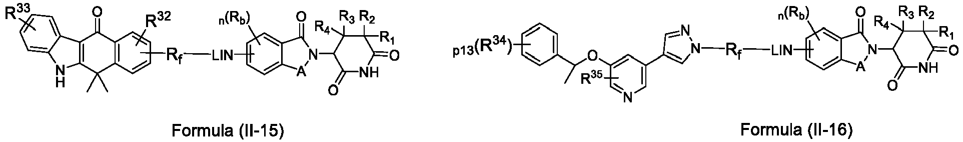 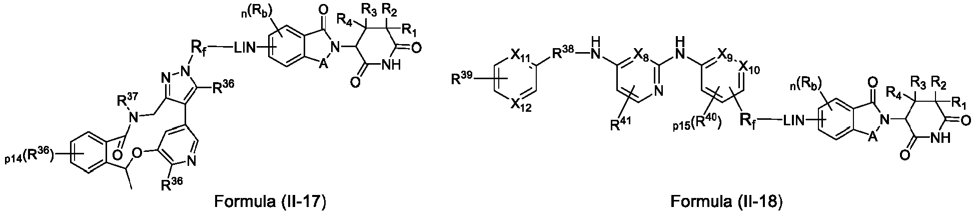 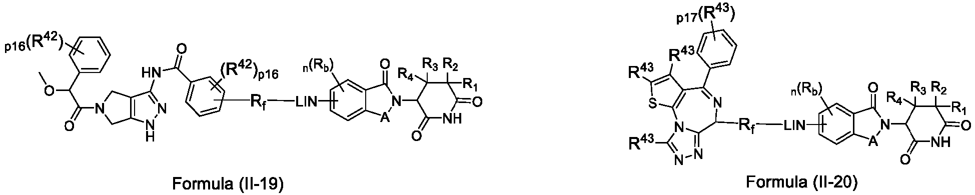 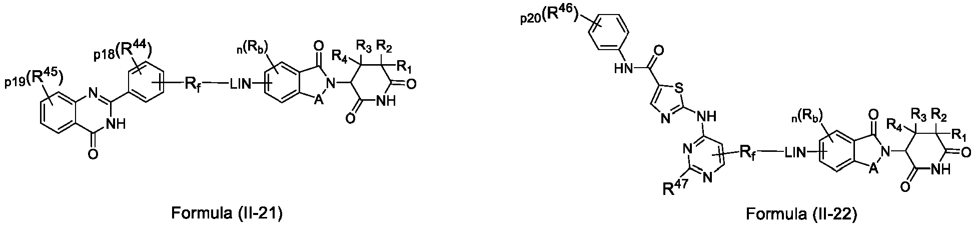 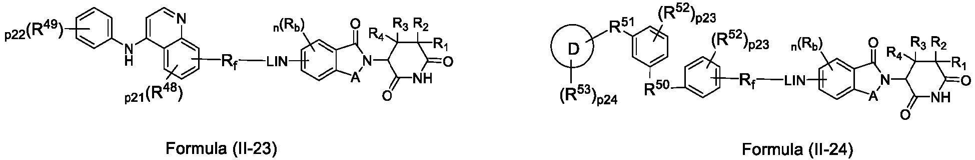 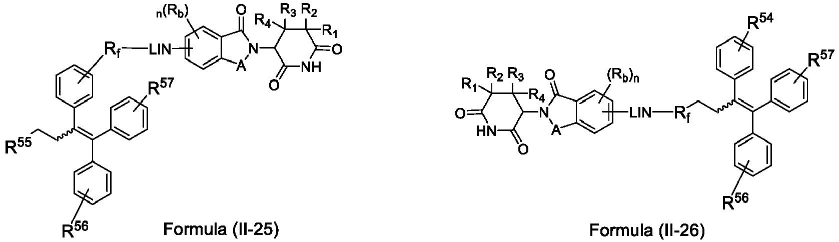 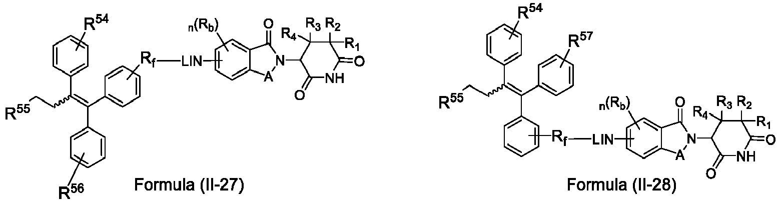 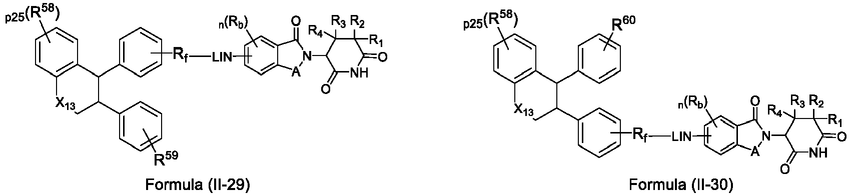 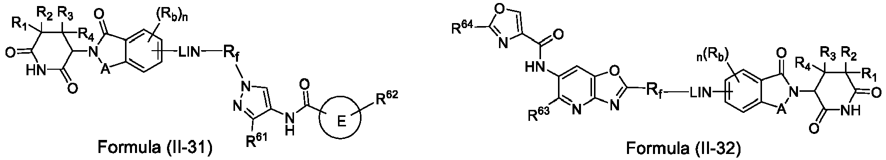 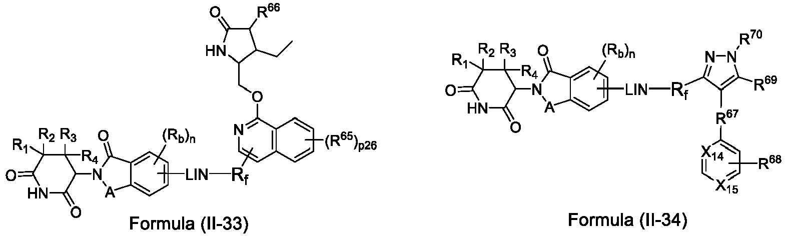 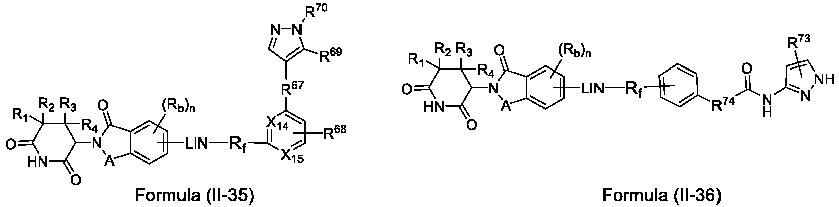 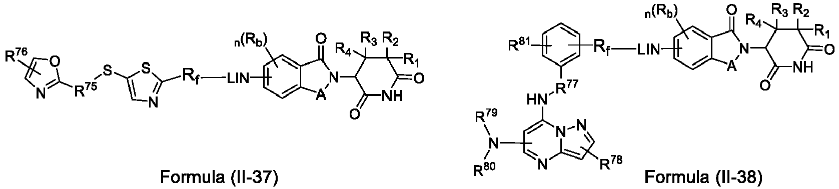 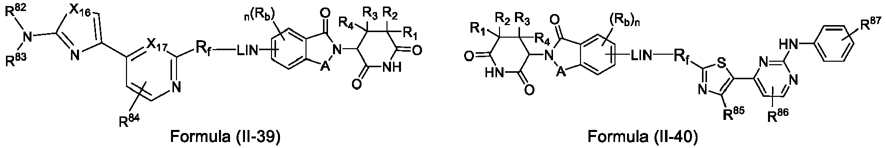 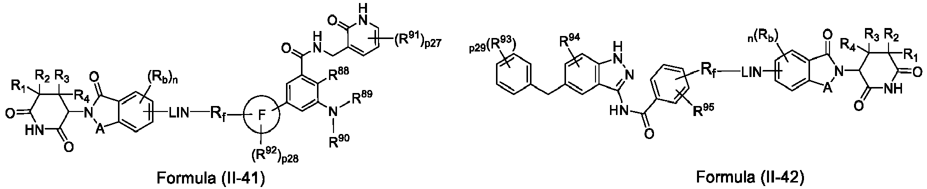 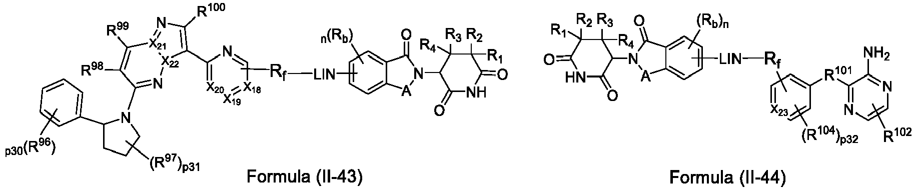 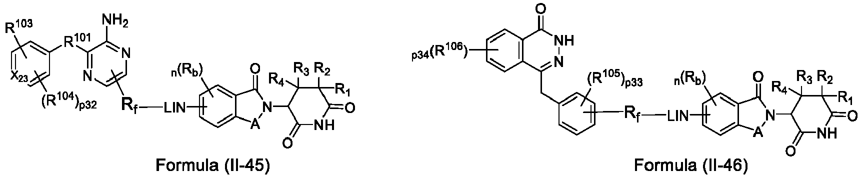 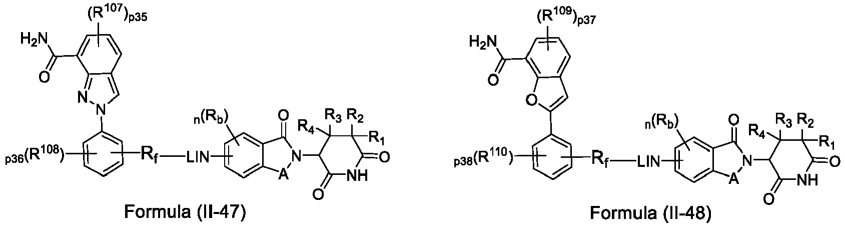 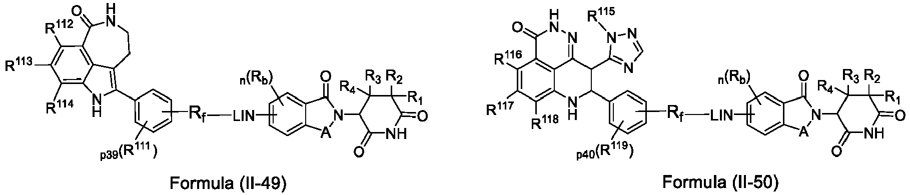 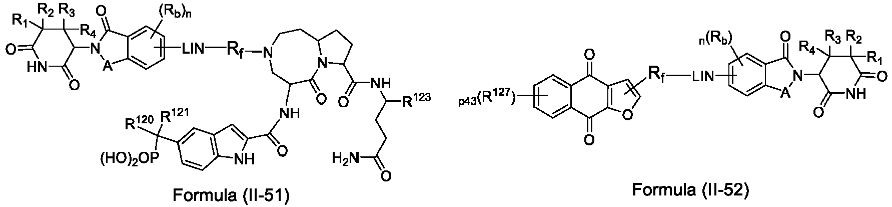 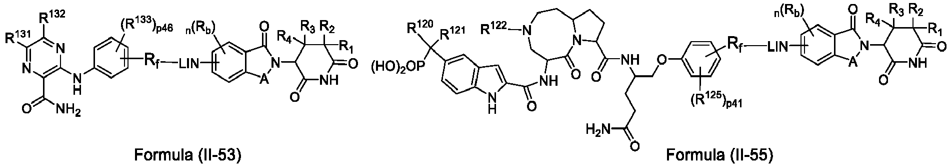 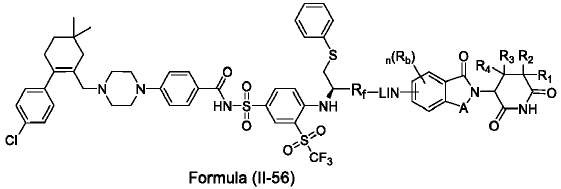 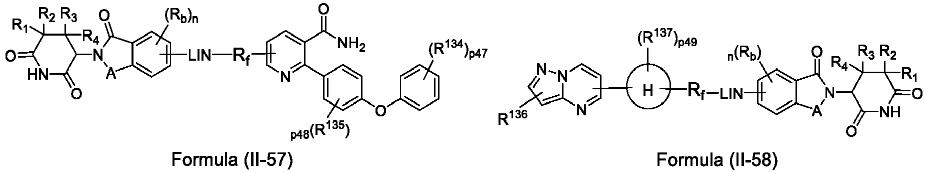 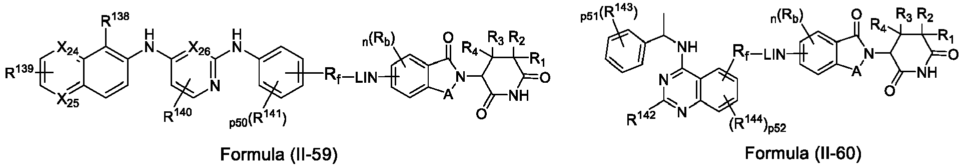 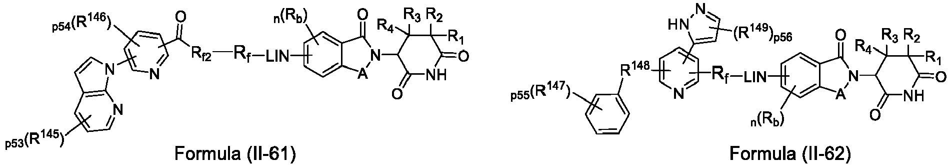 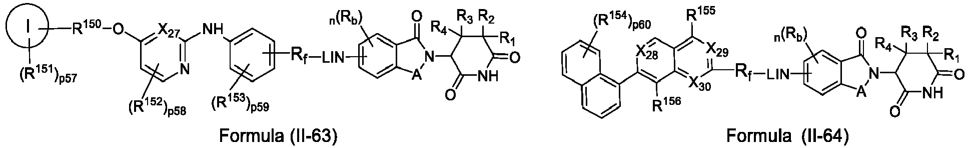 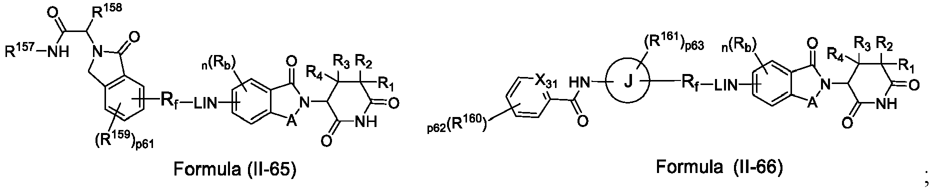 wherein A, R₁, R₂, R₃, R₄, (R_{b})ₙ, LIN, R_{f}, (R¹)_{p1,} (R²)ₚ₂, (R⁴)ₚ₃, (R⁵)ₚ₄, (R⁷)ₚ₅, R⁸, (R⁹)ₚ₆, R¹⁰, (R¹¹)ₚ₇, R¹², R¹³, R¹⁴, ring A, ring B, ring C, R¹⁵, (R¹⁶)ₚ₉, X₁, X₂, X₃, (R²⁰)ₚ₁₀, R²¹, R²², (R²³)ₚ₁₁, (R²⁴)ₚ₁₂, R²⁵, X₄, X₅, X₆, X₇, R²⁸, R²⁹, R³⁰, R³¹, R³², R³³, (R³⁴)ₚ₁₃, R³⁵, (R³⁶)ₚ₁₄, R³⁷, X₈, X₉, X₁₀, X₁₁, X₁₂, R³⁸, R³⁹, (R⁴⁰)ₚ₁₅, (R⁴²)ₚ₁₆, (R⁴³)ₚ₁₇, (R⁴⁴)ₚ₁₈, (R⁴⁵)ₚ₁₉, (R⁴⁶)ₚ₂₀, (R⁴⁸)ₚ₂₁, (R⁴⁹)ₚ₂₂, R⁵⁰, R⁵¹, (R⁵²)ₚ₂₃, (R⁵³)ₚ₂₄, R⁵⁴, R⁵⁵, R⁵⁶, R⁵⁷, (R⁵⁸)ₚ₂₅, X₁₃, R⁵⁹, R⁶⁰, R⁶¹, R⁶², R⁶³, R⁶⁴, (R⁶⁵)ₚ₂₆, R⁶⁶, R⁶⁷, R⁶⁸, R⁶⁹, R⁷⁰, X₁₄, X₁₅, R⁷³, R⁷⁴, R⁷⁵, R⁷⁶, R⁷⁷, R⁷⁸, R⁷⁹, R⁸⁰, R⁸¹, X₁₆, X₁₇, R⁸², R⁸³, R⁸⁴, R⁸⁵, R⁸⁶, R⁸⁷, R⁸⁸, R⁸⁹, R⁹⁰, (R⁹¹)ₚ₂₇, (R⁹²)ₚ₂₈, (R⁹³)ₚ₂₉, R⁹⁴, R⁹⁵, (R⁹⁶)ₚ₃₀, (R⁹⁷)ₚ₃₁, X₁₈, X₁₉, X₂₀, X₂₁, X₂₂, R⁹⁸, R⁹⁹, R¹⁰⁰, R¹⁰¹, R¹⁰², R¹⁰³, X₂₃, (R¹⁰⁴)ₚ₃₂, (R¹⁰⁵)ₚ₃₃, (R¹⁰⁶)_{p34,} (R¹⁰⁷)ₚ₃₅, (R¹⁰⁸)ₚ₃₆, (R¹⁰⁹)ₚ₃₇, (R¹¹⁰)ₚ₃₈, (R¹¹¹)ₚ₃₉, R¹¹², R¹¹³, R¹¹⁴, R¹¹⁵, R¹¹⁶, R¹¹⁷, R¹¹⁸, (R¹¹⁹)ₚ₄₀, R¹²⁰, R¹²¹, R¹²², R¹²³, (R¹²⁵)ₚ₄₁, (R¹²⁷)ₚ₄₃, R¹³¹, R¹³², (R¹³³)ₚ₄₆, (R¹³⁴)ₚ₄₇, (R¹³⁵)ₚ₄₈, R¹³⁶, (R¹³⁷)ₚ₄₉, X₂₄, X₂₅, X₂₆, R¹³⁸, R¹³⁹, R¹⁴⁰, (R¹⁴¹)ₚ₅₀, R¹⁴², (R¹⁴³)ₚ₅₁, (R¹⁴⁴)ₚ₅₂, (R¹⁴⁵)ₚ₅₃, (R¹⁴⁶)ₚ₅₄, R_{f2}, (R¹⁴⁷)ₚ₅₅, R¹⁴⁸, (R¹⁴⁹)ₚ₅₆, X₂₇, ring I, R¹⁵⁰, (R¹⁵¹)ₚ₅₇, (R¹⁵²)ₚ₅₈, (R¹⁵³)ₚ₅₉, X₂₈, X₂₉, X₃₀, (R¹⁵⁴)ₚ₆₀, R¹⁵⁵, R¹⁵⁶, R¹⁵⁷, R¹⁵⁸, (R¹⁵⁹)ₚ₆₁, X₃₁, (R¹⁶⁰)ₚ₆₂, ring J and (R¹⁶¹)ₚ₆₃ are as defined in claim 13.

30. The compound of Formula (II) or salts, enantiomers, stereoisomers, solvates, isotopically enriched analogs, prodrugs, or polymorphs thereof as claimed in claim 12, which is selected from:
N-(2-(4-((2-(2,6-dioxopiperidin-3-yl)-1-oxoisoindolin-5-yl)methyl)piperazin-1-yl)ethyl)-4,9-dioxo-4,9-dihydronaphtho[2,3-b]furan-2-carboxamide;
N-(2-(4-((2-(2,6-dioxopiperidin-3-yl)-4-fluoro-1-oxoisoindolin-5-yl)methyl)piperazin-1-yl)ethyl)-4,9-dioxo-4,9-dihydronaphtho[2,3-b]furan-2-carboxamide;
N-(2-(4-((2-(2,6-dioxopiperidin-3-yl)-6-fluoro-1-oxoisoindolin-5-yl)methyl)piperazin-1-yl)ethyl)-4,9-dioxo-4,9-dihydronaphtho[2,3-b]furan-2-carboxamide;
N-(2-(4-((2-(2,6-dioxopiperidin-3-yl)-7-fluoro-1-oxoisoindolin-5-yl)methyl)piperazin-1-yl)ethyl)-4,9-dioxo-4,9-dihydronaphtho[2,3-b]furan-2-carboxamide;
N-((2-(2,6-dioxopiperidin-3-yl)-1-oxoisoindolin-5-yl)methyl)-4,9-dioxo-4,9-dihydronaphtho[2,3-b]furan-2-carboxamide;
N-((2-(2,6-dioxopiperidin-3-yl)-4-fluoro-1-oxoisoindolin-5-yl)methyl)-4,9-dioxo-4,9-dihydronaphtho[2,3-b]furan-2-carboxamide;
N-((2-(2,6-dioxopiperidin-3-yl)-6-fluoro-1-oxoisoindolin-5-yl)methyl)-4,9-dioxo-4,9-dihydronaphtho[2,3-b]furan-2-carboxamide;
N-((2-(2,6-dioxopiperidin-3-yl)-7-fluoro-1-oxoisoindolin-5-yl)methyl)-4,9-dioxo-4,9-dihydronaphtho[2,3-b]furan-2-carboxamide;
((2-(((5S,8S,10aR)-8-(((2S)-5-amino-1-(2-chloro-3-((2-(2,6-dioxopiperidin-3-yl)-1-oxoisoindolin-5-yl)methyl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(2-chloro-3-((2-(2,6-dioxopiperidin-3-yl)-4-fluoro-1-oxoisoindolin-5-yl)methyl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(2-chloro-3-((2-(2,6-dioxopiperidin-3-yl)-6-fluoro-1-oxoisoindolin-5-yl)methyl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(2-chloro-3-((2-(2,6-dioxopiperidin-3-yl)-7-fluoro-1-oxoisoindolin-5-yl)methyl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(2-chloro-3-(4-((2-(2,6-dioxopiperidin-3-yl)-1-oxoisoindolin-5-yl)methyl)piperazin-1-yl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(2-chloro-3-(4-((2-(2,6-dioxopiperidin-3-yl)-4-fluoro-1-oxoisoindolin-5-yl)methyl)piperazin-1-yl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(2-chloro-3-(4-((2-(2,6-dioxopiperidin-3-yl)-6-fluoro-1-oxoisoindolin-5-yl)methyl)piperazin-1-yl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(2-chloro-3-(4-((2-(2,6-dioxopiperidin-3-yl)-7-fluoro-1-oxoisoindolin-5-yl)methyl)piperazin-1-yl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((1-((2-(2,6-dioxopiperidin-3-yl)-1-oxoisoindolin-5-yl)methyl)piperidin-4-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((1-((2-(2,6-dioxopiperidin-3-yl)-4-fluoro-1-oxoisoindolin-5-yl)methyl)piperidin-4-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((1-((2-(2,6-dioxopiperidin-3-yl)-6-fluoro-1-oxoisoindolin-5-yl)methyl)piperidin-4-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((1-((2-(2,6-dioxopiperidin-3-yl)-7-fluoro-1-oxoisoindolin-5-yl)methyl)piperidin-4-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((2-(2,6-dioxopiperidin-3-yl)-3-oxoisoindolin-5-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((2-(2,6-dioxopiperidin-3-yl)-4-fluoro-3-oxoisoindolin-5-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-ammo-1-(benzhydrylammo)-1,5-dioxopentan-2-yl)carbamoyl)-3-((2-(2,6-dioxopiperidin-3-yl)-6-fluoro-3-oxoisoindolin-5-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((2-(2,6-dioxopiperidin-3-yl)-7-fluoro-3-oxoisoindolin-5-yl)methyl)-6-oxodecahydropyrrolo[1,2-a][1,5]diazocin-5-yl)carbamoyl)-1H-indol-5-yl)difluoromethyl)phosphonic acid;
6-(1-((2-(2,6-dioxopiperidin-3-yl)-1-oxoisoindolin-5-yl)methyl)piperidin-4-yl)-2-(4-phenoxyphenyl)nicotinamide;
6-(1-((2-(2,6-dioxopiperidin-3-yl)-4-fluoro-1-oxoisoindolin-5-yl)methyl)piperidin-4-yl)-2-(4-phenoxyphenyl)nicotinamide;
6-(1-((2-(2,6-dioxopiperidin-3-yl)-6-fluoro-1-oxoisoindolin-5-yl)methyl)piperidin-4-yl)-2-(4-phenoxyphenyl)nicotinamide;
6-(1-((2-(2,6-dioxopiperidin-3-yl)-7-fluoro-1-oxoisoindolin-5-yl)methyl)piperidin-4-yl)-2-(4-phenoxyphenyl)nicotinamide;
6-(4-((2-(2,6-dioxopiperidin-3-yl)-1-oxoisoindolin-5-yl)methyl)piperazin-1-yl)-2-(4-phenoxyphenyl)nicotinamide;
6-(4-((2-(2,6-dioxopiperidin-3-yl)-4-fluoro-1-oxoisoindolin-5-yl)methyl)piperazin-1-yl)-2-(4-phenoxyphenyl)nicotinamide;
6-(4-((2-(2,6-dioxopiperidin-3-yl)-6-fluoro-1-oxoisoindolin-5-yl)methyl)piperazin-1-yl)-2-(4-phenoxyphenyl)nicotinamide;
6-(4-((2-(2,6-dioxopiperidin-3-yl)-7-fluoro-1-oxoisoindolin-5-yl)methyl)piperazin-1-yl)-2-(4-phenoxyphenyl)nicotinamide;
6-(4-(((2-(2,6-dioxopiperidin-3-yl)-1-oxoisoindolin-5-yl)methyl)(methyl)amino)piperidin-1-yl)-2-(4-phenoxyphenyl)nicotinamide;
6-(4-(((2-(2,6-dioxopiperidin-3-yl)-4-fluoro-1-oxoisoindolin-5-yl)methyl)(methyl)amino)piperidin-1-yl)-2-(4-phenoxyphenyl)nicotinamide;
6-(4-(((2-(2,6-dioxopiperidin-3-yl)-6-fluoro-1-oxoisoindolin-5-yl)methyl)(methyl)amino)piperidin-1-yl)-2-(4-phenoxyphenyl)nicotinamide;
6-(4-(((2-(2,6-dioxopiperidin-3-yl)-7-fluoro-1-oxoisoindolin-5-yl)methyl)(methyl)amino)piperidin-1-yl)-2-(4-phenoxyphenyl)nicotinamide;
6-(4-(4-((2-(2,6-dioxopiperidin-3-yl)-1-oxoisoindolin-5-yl)methyl)piperazin-1-yl)piperidin-1-yl)-2-(4-phenoxyphenyl)nicotinamide;
6-(4-(4-((2-(2,6-dioxopiperidin-3-yl)-4-fluoro-1-oxoisoindolin-5-yl)methyl)piperazin-1-yl)piperidin-1-yl)-2-(4-phenoxyphenyl)nicotinamide;
6-(4-(4-((2-(2,6-dioxopiperidin-3-yl)-6-fluoro-1-oxoisoindolin-5-yl)methyl)piperazin-1-yl)piperidin-1-yl)-2-(4-phenoxyphenyl)nicotinamide;
6-(4-(4-((2-(2,6-dioxopiperidin-3-yl)-7-fluoro-1-oxoisoindolin-5-yl)methyl)piperazin-1-yl)piperidin-1-yl)-2-(4-phenoxyphenyl)nicotinamide;
6-(4-(1-((2-(2,6-dioxopiperidin-3-yl)-1-oxoisoindolin-5-yl)methyl)piperidin-4-yl)piperazin-1-yl)-2-(4-phenoxyphenyl)nicotinamide;
6-(4-(1-((2-(2,6-dioxopiperidin-3-yl)-4-fluoro-1-oxoisoindolin-5-yl)methyl)piperidin-4-yl)piperazin-1-yl)-2-(4-phenoxyphenyl)nicotinamide;
6-(4-(1-((2-(2,6-dioxopiperidin-3-yl)-6-fluoro-1-oxoisoindolin-5-yl)methyl)piperidin-4-yl)piperazin-1-yl)-2-(4-phenoxyphenyl)nicotinamide;
6-(4-(1-((2-(2,6-dioxopiperidin-3-yl)-7-fluoro-1-oxoisoindolin-5-yl)methyl)piperidin-4-yl)piperazin-1-yl)-2-(4-phenoxyphenyl)nicotinamide;
3-(tert-butyl)-N-(4-(5-(4-(4-((2-(2,6-dioxopiperidin-3-yl)-1-oxoisoindolin-5-yl)methyl)piperazin-1-yl)phenyl)-1H-indazol-3-yl)-2-methylbenzyl)-1,2,4-oxadiazole-5-carboxamide;
3-(tert-butyl)-N-(4-(5-(4-(4-((2-(2,6-dioxopiperidin-3-yl)-4-fluoro-1-oxoisoindolin-5-yl)methyl)piperazin-1-yl)phenyl)-1H-indazol-3-yl)-2-methylbenzyl)-1,2,4-oxadiazole-5-carboxamide;
3-(tert-butyl)-N-(4-(5-(4-(4-((2-(2,6-dioxopiperidin-3-yl)-6-fluoro-1-oxoisoindolin-5-yl)methyl)piperazin-1-yl)phenyl)-1H-indazol-3-yl)-2-methylbenzyl)-1,2,4-oxadiazole-5-carboxamide;
3-(tert-butyl)-N-(4-(5-(4-(4-((2-(2,6-dioxopiperidin-3-yl)-7-fluoro-1-oxoisoindolin-5-yl)methyl)piperazin-1-yl)phenyl)-1H-indazol-3-yl)-2-methylbenzyl)-1,2,4-oxadiazole-5-carboxamide;
3-(tert-butyl)-N-(4-(5-(4-(4-(((2-(2,6-dioxopiperidin-3-yl)-1-oxoisoindolin-5-yl)methyl)(methyl)amino)piperidin-1-yl)phenyl)-1H-indazol-3-yl)-2-methylbenzyl)-1,2,4-oxadiazole-5-carboxamide;
3-(tert-butyl)-N-(4-(5-(4-(4-(((2-(2,6-dioxopiperidin-3-yl)-4-fluoro-1-oxoisoindolin-5-yl)methyl)(methyl)amino)piperidin-1-yl)phenyl)-1H-indazol-3-yl)-2-methylbenzyl)-1,2,4-oxadiazole-5-carboxamide;
3-(tert-butyl)-N-(4-(5-(4-(4-(((2-(2,6-dioxopiperidin-3-yl)-6-fluoro-1-oxoisoindolin-5-yl)methyl)(methyl)amino)piperidin-1-yl)phenyl)-1H-indazol-3-yl)-2-methylbenzyl)-1,2,4-oxadiazole-5-carboxamide;
3-(tert-butyl)-N-(4-(5-(4-(4-(((2-(2,6-dioxopiperidin-3-yl)-7-fluoro-1-oxoisoindolin-5-yl)methyl)(methyl)amino)piperidin-1-yl)phenyl)-1H-indazol-3-yl)-2-methylbenzyl)-1,2,4-oxadiazole-5-carboxamide;
3-(tert-butyl)-N-(4-(5-(4-(4-(4-((2-(2,6-dioxopiperidin-3-yl)-1-oxoisoindolin-5-yl)methyl)piperazin-1-yl)piperidin-1-yl)phenyl)-1H-indazol-3-yl)-2-methylbenzyl)-1,2,4-oxadiazole-5-carboxamide;
3-(tert-butyl)-N-(4-(5-(4-(4-(4-((2-(2,6-dioxopiperidin-3-yl)-4-fluoro-1-oxoisoindolin-5-yl)methyl)piperazin-1-yl)piperidin-1-yl)phenyl)-1H-indazol-3-yl)-2-methylbenzyl)-1,2,4-oxadiazole-5-carboxamide;
3-(tert-butyl)-N-(4-(5-(4-(4-(4-((2-(2,6-dioxopiperidin-3-yl)-6-fluoro-1-oxoisoindolin-5-yl)methyl)piperazin-1-yl)piperidin-1-yl)phenyl)-1H-indazol-3-yl)-2-methylbenzyl)-1,2,4-oxadiazole-5-carboxamide;
3-(tert-butyl)-N-(4-(5-(4-(4-(4-((2-(2,6-dioxopiperidin-3-yl)-7-fluoro-1-oxoisoindolin-5-yl)methyl)piperazin-1-yl)piperidin-1-yl)phenyl)-1H-indazol-3-yl)-2-methylbenzyl)-1,2,4-oxadiazole-5-carboxamide;
3-(tert-butyl)-N-(4-(5-(4-(4-(1-((2-(2,6-dioxopiperidin-3-yl)-1-oxoisoindolin-5-yl)methyl)piperidin-4-yl)piperazin-1-yl)phenyl)-1H-indazol-3-yl)-2-methylbenzyl)-1,2,4-oxadiazole-5-carboxamide;
3-(tert-butyl)-N-(4-(5-(4-(4-(1-((2-(2,6-dioxopiperidin-3-yl)-4-fluoro-1-oxoisoindolin-5-yl)methyl)piperidin-4-yl)piperazin-1-yl)phenyl)-1H-indazol-3-yl)-2-methylbenzyl)-1,2,4-oxadiazole-5-carboxamide;
3-(tert-butyl)-N-(4-(5-(4-(4-(1-((2-(2,6-dioxopiperidin-3-yl)-6-fluoro-1-oxoisoindolin-5-yl)methyl)piperidin-4-yl)piperazin-1-yl)phenyl)-1H-indazol-3-yl)-2-methylbenzyl)-1,2,4-oxadiazole-5-carboxamide;
3-(tert-butyl)-N-(4-(5-(4-(4-(1-((2-(2,6-dioxopiperidin-3-yl)-7-fluoro-1-oxoisoindolin-5-yl)methyl)piperidin-4-yl)piperazin-1-yl)phenyl)-1H-indazol-3-yl)-2-methylbenzyl)-1,2,4-oxadiazole-5-carboxamide;
3-(5-((4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)methyl)-1-oxoisoindolin-2-yl)piperidine-2,6-dione;
3-(5-((4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)methyl)-4-fluoro-1-oxoisoindolin-2-yl)piperidine-2,6-dione;
3-(5-((4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)methyl)-6-fluoro-1-oxoisoindolin-2-yl)piperidine-2,6-dione;
3-(5-((4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)methyl)-7-fluoro-1-oxoisoindolin-2-yl)piperidine-2,6-dione;
3-(5-((3-(4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)azetidin-1-yl)methyl)-1-oxoisoindolin-2-yl)piperidine-2,6-dione;
3-(5-((3-(4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)azetidin-1-yl)methyl)-4-fluoro-1-oxoisoindolin-2-yl)piperidine-2,6-dione;
3-(5-((3-(4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)azetidin-1-yl)methyl)-6-fluoro-1-oxoisoindolin-2-yl)piperidine-2,6-dione;
3-(5-((3-(4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)azetidin-1-yl)methyl)-7-fluoro-1-oxoisoindolin-2-yl)piperidine-2,6-dione;
3-(6-(((1-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperidin-4-yl)(methyl)amino)methyl)-1-oxoisoindolin-2-yl)piperidine-2,6-dione;
3-(6-(((1-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperidin-4-yl)(methyl)amino)methyl)-7-fluoro-1-oxoisoindolin-2-yl)piperidine-2,6-dione;
3-(6-(((1-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperidin-4-yl)(methyl)amino)methyl)-5-fluoro-1-oxoisoindolin-2-yl)piperidine-2,6-dione;
3-(6-(((1-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperidin-4-yl)(methyl)amino)methyl)-4-fluoro-1-oxoisoindolin-2-yl)piperidine-2,6-dione;
3-(5-((4-(1-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperidin-4-yl)piperazin-1-yl)methyl)-1-oxoisoindolin-2-yl)piperidine-2,6-dione;
3-(5-((4-(1-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3 -yl)pip eridin-4-yl)pip erazin-1-yl)methyl)-4-fl uoro-1-oxoisoindolin-2-yl)pip eridine-2, 6-dione;
3-(5-((4-(1-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3 -yl)piperidin-4-yl)piperazin-1 -yl)methyl)-6-fluoro-1 -oxoisoindolin-2-yl)piperidine-2,6-dione;
3-(5-((4-(1-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperidin-4-yl)piperazin-1-yl)methyl)-7-fluoro-1-oxoisoindolin-2-yl)piperidine-2,6-dione;
3-(5-((4-(4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)piperidin-1-yl)methyl)-1-oxoisoindolin-2-yl)piperidine-2,6-dione;
3-(5-((4-(4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)piperidin-1-yl)methyl)-4-fluoro-1-oxoisoindolin-2-yl)piperidine-2,6-dione;
3-(5-((4-(4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)piperidin-1-yl)methyl)-6-fluoro-1-oxoisoindolin-2-yl)piperidine-2,6-dione;
3-(5-((4-(4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)piperidin-1-yl)methyl)-7-fluoro-1-oxoisoindolin-2-yl)piperidine-2,6-dione;
(S)-3-(5-((4-(4-amino-3-(4-phenoxyphenyl)-1H-pyrazolo[3,4-d]pyrimidin-1-yl)piperidin-1-yl)methyl)-1-oxoisoindolin-2-yl)piperidine-2,6-dione;
(S)-3-(5-((4-(4-amino-3-(4-phenoxyphenyl)-1H-pyrazolo[3,4-d]pyrimidin-1-yl)piperidin-1-yl)methyl)-4-fluoro-1-oxoisoindolin-2-yl)piperidine-2,6-dione;
(S)-3-(5-((4-(4-amino-3-(4-phenoxyphenyl)-1H-pyrazolo[3,4-d]pyrimidin-1-yl)piperidin-1-yl)methyl)-6-fluoro-1-oxoisoindolin-2-yl)piperidine-2,6-dione;
(S)-3-(5-((4-(4-amino-3-(4-phenoxyphenyl)-1H-pyrazolo[3,4-d]pyrimidin-1-yl)piperidin-1-yl)methyl)-7-fluoro-1-oxoisoindolin-2-yl)piperidine-2,6-dione;
3-(5-(((3R,4S)-4-(4-amino-3-(4-phenoxyphenyl)-1H-pyrazolo[3,4-d]pyrimidin-1-yl)-3-fluoropip eridin-1-yl)methyl)-1-oxoi soindolin-2-yl)pip eridine-2, 6-dione;
3-(5-(((3R,4S)-4-(4-amino-3-(4-phenoxyphenyl)-1H-pyrazolo[3,4-d]pyrimidin-1-yl)-3-fluoropiperidin-1-yl)methyl)-4-fluoro-1-oxoisoindolin-2-yl)piperidine-2,6-dione;
3-(5-(((3R,4S)-4-(4-amino-3-(4-phenoxyphenyl)-1H-pyrazolo[3,4-d]pyrimidin-1-yl)-3-fluoropiperidin-1-yl)methyl)-6-fluoro-1-oxoisoindolin-2-yl)piperidine-2,6-dione;
3-(5-(((3R,4S)-4-(4-amino-3-(4-phenoxyphenyl)-1H-pyrazolo[3,4-d]pyrimidin-1-yl)-3-fluoropiperidin-1-yl)methyl)-7-fluoro-1-oxoisoindolin-2-yl)piperidine-2,6-dione;
3-(5-(((3S,4R)-4-(4-amino-3-(4-phenoxyphenyl)-1H-pyrazolo[3,4-d]pyrimidin-1-yl)-3-fluoropiperidin-1-yl)methyl)-1-oxoisoindolin-2-yl)piperidine-2,6-dione;
3-(5-(((3S,4R)-4-(4-amino-3-(4-phenoxyphenyl)-1H-pyrazolo[3,4-d]pyrimidin-1-yl)-3-fluoropiperidin-1-yl)methyl)-4-fluoro-1-oxoisoindolin-2-yl)piperidine-2,6-dione;
3-(5-(((3S,4R)-4-(4-amino-3-(4-phenoxyphenyl)-1H-pyrazolo[3,4-d]pyrimidin-1-yl)-3-fluoropiperidin-1-yl)methyl)-6-fluoro-1-oxoisoindolin-2-yl)piperidine-2,6-dione;
3-(5-(((3S,4R)-4-(4-amino-3-(4-phenoxyphenyl)-1H-pyrazolo[3,4-d]pyrimidin-1-yl)-3-fluoropiperidin-1-yl)methyl)-7-fluoro-1-oxoisoindolin-2-yl)piperidine-2,6-dione;
3-(5-(((3R,4R)-4-(4-amino-3-(4-phenoxyphenyl)-1H-pyrazolo[3,4-d]pyrimidin-1-yl)-3-fluoropiperidin-1-yl)methyl)-1-oxoisoindolin-2-yl)piperidine-2,6-dione;
3-(5-(((3R,4R)-4-(4-amino-3-(4-phenoxyphenyl)-1H-pyrazolo[3,4-d]pyrimidin-1-yl)-3-fluoropiperidin-1-yl)methyl)-4-fluoro-1-oxoisoindolin-2-yl)piperidine-2,6-dione;
3-(5-(((3R,4R)-4-(4-amino-3-(4-phenoxyphenyl)-1H-pyrazolo[3,4-d]pyrimidin-1-yl)-3-fluoropiperidin-1-yl)methyl)-6-fluoro-1-oxoisoindolin-2-yl)piperidine-2,6-dione;
3-(5-(((3R,4R)-4-(4-amino-3-(4-phenoxyphenyl)-1H-pyrazolo[3,4-d]pyrimidin-1-yl)-3-fluoropiperidin-1-yl)methyl)-7-fluoro-1-oxoisoindolin-2-yl)piperidine-2,6-dione;
3-(5-(((3S,4S)-4-(4-amino-3-(4-phenoxyphenyl)-1H-pyrazolo[3,4-d]pyrimidin-1-yl)-3-fluoropiperidin-1-yl)methyl)-1-oxoisoindolin-2-yl)piperidine-2,6-dione;
3-(5-(((3S,4S)-4-(4-amino-3-(4-phenoxyphenyl)-1H-pyrazolo[3,4-d]pyrimidin-1-yl)-3-fluoropiperidin-1-yl)methyl)-4-fluoro-1-oxoisoindolin-2-yl)piperidine-2,6-dione;
3-(5-(((3S,4S)-4-(4-amino-3-(4-phenoxyphenyl)-1H-pyrazolo[3,4-d]pyrimidin-1-yl)-3-fluoropiperidin-1-yl)methyl)-6-fluoro-1-oxoisoindolin-2-yl)piperidine-2,6-dione;
3-(5-(((3S,4S)-4-(4-amino-3-(4-phenoxyphenyl)-1H-pyrazolo[3,4-d]pyrimidin-1-yl)-3-fluoropiperidin-1-yl)methyl)-7-fluoro-1-oxoisoindolin-2-yl)piperidine-2,6-dione;
3-(5-(((R)-3-(4-amino-3-(4-phenoxyphenyl)-1H-pyrazolo[3,4-d]pyrimidin-1-yl)piperidin-1-yl)methyl)-1-oxoisoindolin-2-yl)piperidine-2,6-dione;
3-(5-(((R)-3-(4-amino-3-(4-phenoxyphenyl)-1H-pyrazolo[3,4-d]pyrimidin-1-yl)piperidin-1-yl)methyl)-4-fluoro-1-oxoisoindolin-2-yl)piperidine-2,6-dione;
3-(5-(((R)-3-(4-amino-3-(4-phenoxyphenyl)-1H-pyrazolo[3,4-d]pyrimidin-1-yl)piperidin-1-yl)methyl)-6-fluoro-1-oxoisoindolin-2-yl)piperidine-2,6-dione;
3-(5-(((R)-3-(4-amino-3-(4-phenoxyphenyl)-1H-pyrazolo[3,4-d]pyrimidin-1-yl)piperidin-1-yl)methyl)-7-fluoro-1-oxoisoindolin-2-yl)piperidine-2,6-dione;
3-(5-((4-(4-amino-3-(4-phenoxyphenyl)-1H-pyrazolo[3,4-d]pyrimidin-1-yl)piperidin-1-yl)methyl)-1-oxoisoindolin-2-yl)piperidine-2,6-dione;
3-(5-((4-(4-amino-3-(4-phenoxyphenyl)-1H-pyrazolo[3,4-d]pyrimidin-1-yl)piperidin-1-yl)methyl)-4-fluoro-1-oxoisoindolin-2-yl)piperidine-2,6-dione;
3-(5-((4-(4-amino-3-(4-phenoxyphenyl)-1H-pyrazolo[3,4-d]pyrimidin-1-yl)piperidin-1-yl)methyl)-6-fluoro-1-oxoisoindolin-2-yl)piperidine-2,6-dione;
3-(5-((4-(4-amino-3-(4-phenoxyphenyl)-1H-pyrazolo[3,4-d]pyrimidin-1-yl)piperidin-1-yl)methyl)-7-fluoro-1-oxoisoindolin-2-yl)piperidine-2,6-dione;
3-(5-((4-(4-amino-3-(4-phenoxyphenyl)-1H-pyrazolo[3,4-d]pyrimidin-1-yl)-3-fluoropiperidin-1-yl)methyl)-1-oxoisoindolin-2-yl)piperidine-2,6-dione;
3-(5-((4-(4-amino-3-(4-phenoxyphenyl)-1H-pyrazolo[3,4-d]pyrimidin-1-yl)-3-fluoropiperidin-1-yl)methyl)-4-fluoro-1-oxoisoindolin-2-yl)piperidine-2,6-dione;
3-(5-((4-(4-amino-3-(4-phenoxyphenyl)-1H-pyrazolo[3,4-d]pyrimidin-1-yl)-3-fluoropiperidin-1-yl)methyl)-6-fluoro-1-oxoisoindolin-2-yl)piperidine-2,6-dione;
3-(5-((4-(4-amino-3-(4-phenoxyphenyl)-1H-pyrazolo[3,4-d]pyrimidin-1-yl)-3-fluoropiperidin-1-yl)methyl)-7-fluoro-1-oxoisoindolin-2-yl)piperidine-2,6-dione;
3-(5-((4-(4-(1,2-bis(4-hydroxyphenyl)but-1-en-1-yl)phenyl)piperazin-1-yl)methyl)-1-oxoisoindolin-2-yl)piperidine-2,6-dione;
3-(5-((4-(4-(1,2-bis(4-hydroxyphenyl)but-1-en-1-yl)phenyl)piperazin-1-yl)methyl)-4-fluoro-1-oxoisoindolin-2-yl)piperidine-2,6-dione;
3-(5-((4-(4-(1,2-bis(4-hydroxyphenyl)but-1-en-1-yl)phenyl)piperazin-1-yl)methyl)-6-fluoro-1-oxoisoindolin-2-yl)piperidine-2,6-dione;
3-(5-((4-(4-(1,2-bis(4-hydroxyphenyl)but-1-en-1-yl)phenyl)piperazin-1-yl)methyl)-7-fluoro-1-oxoisoindolin-2-yl)piperidine-2,6-dione;
3-(5-((4-(4-(1-(4-hydroxyphenyl)-2-phenylbut-1-en-1-yl)phenyl)piperazin-1-yl)methyl)-1-oxoisoindolin-2-yl)piperidine-2,6-dione;
3-(4-fluoro-5-((4-(4-(1-(4-hydroxyphenyl)-2-phenylbut-1-en-1-yl)phenyl)piperazin-1-yl)methyl)-1-oxoisoindolin-2-yl)piperidine-2,6-dione;
3-(6-fluoro-5-((4-(4-(1-(4-hydroxyphenyl)-2-phenylbut-1-en-1-yl)phenyl)piperazin-1-yl)methyl)-1-oxoisoindolin-2-yl)piperidine-2,6-dione;
3-(7-fluoro-5-((4-(4-(1-(4-hydroxyphenyl)-2-phenylbut-1-en-1-yl)phenyl)piperazin-1-yl)methyl)-1-oxoisoindolin-2-yl)piperidine-2,6-dione;
3-(5-((4-(4-(4-chloro-1,2-bis(4-hydroxyphenyl)but-1-en-1-yl)phenyl)piperazin-1-yl)methyl)-1-oxoisoindolin-2-yl)piperidine-2,6-dione;
3-(5-((4-(4-(4-chloro-1,2-bis(4-hydroxyphenyl)but-1-en-1-yl)phenyl)piperazin-1-yl)methyl)-4-fluoro-1-oxoisoindolin-2-yl)piperidine-2,6-dione;
3 -(5 -((4-(4-(4-chloro-1,2-bis(4-hydroxyphenyl)but-1 -en-1 -yl)phenyl)piperazin-1 -yl)methyl)-6-fluoro-1-oxoisoindolin-2-yl)piperidine-2,6-dione;
3 -(5 -((4-(4-(4-chloro-1,2-bis(4-hydroxyphenyl)but-1 -en-1 -yl)phenyl)piperazin-1 -yl)methyl)-7-fluoro-1-oxoisoindolin-2-yl)piperidine-2,6-dione;
3-(5-((4-(4-(4-chloro-1-(4-hydroxyphenyl)-2-phenylbut-1-en-1-yl)phenyl)piperazin-1-yl)methyl)-1-oxoisoindolin-2-yl)piperidine-2,6-dione;
3-(5-((4-(4-(4-chloro-1-(4-hydroxyphenyl)-2-phenylbut-1-en-1-yl)phenyl)piperazin-1-yl)methyl)-4-fluoro-1-oxoisoindolin-2-yl)piperidine-2,6-dione;
3-(5-((4-(4-(4-chloro-1-(4-hydroxyphenyl)-2-phenylbut-1-en-1-yl)phenyl)piperazin-1-yl)methyl)-6-fluoro-1-oxoisoindolin-2-yl)piperidine-2,6-dione;
3-(5-((4-(4-(4-chloro-1-(4-hydroxyphenyl)-2-phenylbut-1-en-1-yl)phenyl)piperazin-1-yl)methyl)-7-fluoro-1-oxoisoindolin-2-yl)piperidine-2,6-dione;
3-(5-(((2-(4-(4-chloro-1,2-bis(4-hydroxyphenyl)but-1-en-1-yl)phenoxy)ethyl)(methyl)amino)methyl)-1-oxoisoindolin-2-yl)piperidine-2,6-dione;
3-(5-(((2-(4-(4-chloro-1,2-bis(4-hydroxyphenyl)but-1-en-1-yl)phenoxy)ethyl)(methyl)amino)methyl)-4-fluoro-1-oxoisoindolin-2-yl)piperidine-2,6-dione;
3-(5-(((2-(4-(4-chloro-1,2-bis(4-hydroxyphenyl)but-1-en-1-yl)phenoxy)ethyl)(methyl)amino)methyl)-6-fluoro-1-oxoisoindolin-2-yl)piperidine-2,6-dione;
3-(5-(((2-(4-(4-chloro-1,2-bis(4-hydroxyphenyl)but-1-en-1-yl)phenoxy)ethyl)(methyl)amino)methyl)-7-fluoro-1-oxoisoindolin-2-yl)piperidine-2,6-dione;
3-(5-(((2-(4-(4-chloro-1,2-bis(4-hydroxyphenyl)but-1-en-1-yl)phenoxy)ethyl)amino)methyl)-1-oxoisoindolin-2-yl)piperidine-2,6-dione;
3-(5-(((2-(4-(4-chloro-1,2-bis(4-hydroxyphenyl)but-1-en-1-yl)phenoxy)ethyl)amino)methyl)-4-fluoro-1-oxoisoindolin-2-yl)piperidine-2,6-dione;
3-(5-(((2-(4-(4-chloro-1,2-bis(4-hydroxyphenyl)but-1-en-1-yl)phenoxy)ethyl)amino)methyl)-6-fluoro-1-oxoisoindolin-2-yl)piperidine-2,6-dione;
3-(5-(((2-(4-(4-chloro-1,2-bis(4-hydroxyphenyl)but-1-en-1-yl)phenoxy)ethyl)amino)methyl)-7-fluoro-1-oxoisoindolin-2-yl)piperidine-2,6-dione;
3-(5-((4-(4-((1R,2S)-6-hydroxy-2-phenyl-1,2,3,4-tetrahydronaphthalen-1-yl)phenyl)piperazin-1-yl)methyl)-1-oxoisoindolin-2-yl)piperidine-2,6-dione;
3-(4-fluoro-5-((4-(4-((1R,2S)-6-hydroxy-2-phenyl-1,2,3,4-tetrahydronaphthalen-1-yl)phenyl)piperazin-1-yl)methyl)-1-oxoisoindolin-2-yl)piperidine-2,6-dione;
3-(6-fluoro-5-((4-(4-((1R,2S)-6-hydroxy-2-phenyl-1,2,3,4-tetrahydronaphthalen-1-yl)phenyl)piperazin-1-yl)methyl)-1-oxoisoindolin-2-yl)piperidine-2,6-dione;
3-(7-fluoro-5-((4-(4-((1R,2S)-6-hydroxy-2-phenyl-1,2,3,4-tetrahydronaphthalen-1-yl)phenyl)piperazin-1-yl)methyl)-1-oxoisoindolin-2-yl)piperidine-2,6-dione;
3-(5-((4-(4-((3S,4R)-7-hydroxy-3-phenylchroman-4-yl)phenyl)piperazin-1-yl)methyl)-1-oxoisoindolin-2-yl)piperidine-2,6-dione;
3-(4-fluoro-5-((4-(4-((3S,4R)-7-hydroxy-3-phenylchroman-4-yl)phenyl)piperazin-1-yl)methyl)-1-oxoisoindolin-2-yl)piperidine-2,6-dione;
3-(6-fluoro-5-((4-(4-((3S,4R)-7-hydroxy-3-phenylchroman-4-yl)phenyl)piperazin-1-yl)methyl)-1-oxoisoindolin-2-yl)piperidine-2,6-dione;
3-(7-fluoro-5-((4-(4-((3S,4R)-7-hydroxy-3-phenylchroman-4-yl)phenyl)piperazin-1-yl)methyl)-1-oxoisoindolin-2-yl)piperidine-2,6-dione;
3-(5-(((1-(4-((3S,4R)-7-hydroxy-3-phenylchroman-4-yl)phenyl)piperidin-4-yl)(methyl)amino)methyl)-1-oxoisoindolin-2-yl)piperidine-2,6-dione;
3-(4-fluoro-5-(((1-(4-((3S,4R)-7-hydroxy-3-phenylchroman-4-yl)phenyl)piperidin-4-yl)(methyl)amino)methyl)-1-oxoisoindolin-2-yl)piperidine-2,6-dione;
3-(6-fluoro-5-(((1-(4-((3S,4R)-7-hydroxy-3-phenylchroman-4-yl)phenyl)piperidin-4-yl)(methyl)amino)methyl)-1-oxoisoindolin-2-yl)piperidine-2,6-dione;
3-(7-fluoro-5-(((1-(4-((3S,4R)-7-hydroxy-3-phenylchroman-4-yl)phenyl)piperidin-4-yl)(methyl)amino)methyl)-1-oxoisoindolin-2-yl)piperidine-2,6-dione;
3-(5-((4-(1-(4-((3S,4R)-7-hydroxy-3-phenylchroman-4-yl)phenyl)piperidin-4-yl)piperazin-1-yl)methyl)-1-oxoisoindolin-2-yl)piperidine-2,6-dione;
3-(4-fluoro-5-((4-(1-(4-((3S,4R)-7-hydroxy-3-phenylchroman-4-yl)phenyl)piperidin-4-yl)piperazin-1-yl)methyl)-1-oxoisoindolin-2-yl)piperidine-2,6-dione;
3-(6-fluoro-5-((4-(1-(4-((3S,4R)-7-hydroxy-3-phenylchroman-4-yl)phenyl)piperidin-4-yl)piperazin-1-yl)methyl)-1-oxoisoindolin-2-yl)piperidine-2,6-dione;
3-(7-fluoro-5-((4-(1-(4-((3S,4R)-7-hydroxy-3-phenylchroman-4-yl)phenyl)piperidin-4-yl)piperazin-1-yl)methyl)-1-oxoisoindolin-2-yl)piperidine-2,6-dione;
3-(5-(((1-(4-((1R,2S)-6-hydroxy-2-phenyl-1,2,3,4-tetrahydronaphthalen-1-yl)phenyl)piperidin-4-yl)(methyl)amino)methyl)-1-oxoisoindolin-2-yl)piperidine-2,6-dione;
3-(4-fluoro-5-(((1-(4-((1R,2S)-6-hydroxy-2-phenyl-1,2,3,4-tetrahydronaphthalen-1-yl)phenyl)piperidin-4-yl)(methyl)amino)methyl)-1-oxoisoindolin-2-yl)piperidine-2,6-dione;
3-(6-fluoro-5-(((1-(4-((1R,2S)-6-hydroxy-2-phenyl-1,2,3,4-tetrahydronaphthalen-1-yl)phenyl)piperidin-4-yl)(methyl)amino)methyl)-1-oxoisoindolin-2-yl)piperidine-2,6-dione;
3-(7-fluoro-5-(((1-(4-((1R,2S)-6-hydroxy-2-phenyl-1,2,3,4-tetrahydronaphthalen-1-yl)phenyl)piperidin-4-yl)(methyl)amino)methyl)-1-oxoisoindolin-2-yl)piperidine-2,6-dione;
3-(5-(((2-(4-(1,2-bis(4-hydroxyphenyl)but-1-en-1-yl)phenoxy)ethyl)amino)methyl)-1-oxoisoindolin-2-yl)piperidine-2,6-dione;
3-(5-(((2-(4-(1,2-bis(4-hydroxyphenyl)but-1-en-1-yl)phenoxy)ethyl)amino)methyl)-4-fluoro-1-oxoisoindolin-2-yl)piperidine-2,6-dione;
3-(5-(((2-(4-(1,2-bis(4-hydroxyphenyl)but-1-en-1-yl)phenoxy)ethyl)amino)methyl)-6-fluoro-1-oxoisoindolin-2-yl)piperidine-2,6-dione;
3-(5-(((2-(4-(1,2-bis(4-hydroxyphenyl)but-1-en-1-yl)phenoxy)ethyl)amino)methyl)-7-fluoro-1-oxoisoindolin-2-yl)piperidine-2,6-dione;
3-(5-(((2-(4-(1,2-bis(4-hydroxyphenyl)but-1-en-1-yl)phenoxy)ethyl)(methyl)amino)methyl)-1-oxoisoindolin-2-yl)piperidine-2,6-dione;
3-(5-(((2-(4-(1,2-bis(4-hydroxyphenyl)but-1-en-1-yl)phenoxy)ethyl)(methyl)amino)methyl)-4-fluoro-1-oxoisoindolin-2-yl)piperidine-2,6-dione;
3-(5-(((2-(4-(1,2-bis(4-hydroxyphenyl)but-1-en-1-yl)phenoxy)ethyl)(methyl)amino)methyl)-6-fluoro-1-oxoisoindolin-2-yl)piperidine-2,6-dione;
3-(5-(((2-(4-(1,2-bis(4-hydroxyphenyl)but-1-en-1-yl)phenoxy)ethyl)(methyl)amino)methyl)-7-fluoro-1-oxoisoindolin-2-yl)piperidine-2,6-dione;
3-(5-(((2-(4-(1,2-diphenylbut-1-en-1-yl)phenoxy)ethyl)(methyl)amino)methyl)-1-oxoisoindolin-2-yl)piperidine-2,6-dione;
3-(5-(((2-(4-(1,2-diphenylbut-1-en-1-yl)phenoxy)ethyl)(methyl)amino)methyl)-4-fluoro-1-oxoisoindolin-2-yl)piperidine-2,6-dione;
3-(5-(((2-(4-(1,2-diphenylbut-1-en-1-yl)phenoxy)ethyl)(methyl)amino)methyl)-6-fluoro-1-oxoisoindolin-2-yl)piperidine-2,6-dione;
3-(5-(((2-(4-(1,2-diphenylbut-1-en-1-yl)phenoxy)ethyl)(methyl)amino)methyl)-7-fluoro-1-oxoisoindolin-2-yl)piperidine-2,6-dione;
3 -(5 -(((2-(4-(4-chloro-1,2-diphenylbut-1 -en-1 -yl)phenoxy)ethyl)(methyl)amino)methyl)-1 - oxoisoindolin-2-yl)piperidine-2,6-dione;
3 -(5 -(((2-(4-(4-chloro-1,2-diphenylbut-1 -en-1 -yl)phenoxy)ethyl)(methyl)amino)methyl)-4-fluoro-1-oxoisoindolin-2-yl)piperidine-2,6-dione;
3-(5-(((2-(4-(4-chloro-1,2-diphenylbut-1-en-1-yl)phenoxy)ethyl)(methyl)amino)methyl)-6-fluoro-1-oxoisoindolin-2-yl)piperidine-2,6-dione;
3-(5-(((2-(4-(4-chloro-1,2-diphenylbut-1-en-1-yl)phenoxy)ethyl)(methyl)amino)methyl)-7-fluoro-1-oxoisoindolin-2-yl)piperidine-2,6-dione;
3-(5-(((1-(3-(2,3-dichlorophenyl)-1H-pyrazolo[3,4-b]pyrazin-6-yl)-4-methylpiperidin-4-yl)amino)methyl)-1-oxoisoindolin-2-yl)piperidine-2,6-dione;
3-(5-(((1-(3-(2,3-dichlorophenyl)-1H-pyrazolo[3,4-b]pyrazin-6-yl)-4-methylpiperidin-4-yl)amino)methyl)-4-fluoro-1-oxoisoindolin-2-yl)piperidine-2,6-dione;
3-(5-(((1-(3-(2,3-dichlorophenyl)-1H-pyrazolo[3,4-b]pyrazin-6-yl)-4-methylpiperidin-4-yl)amino)methyl)-6-fluoro-1-oxoisoindolin-2-yl)piperidine-2,6-dione;
3-(5-(((1-(3-(2,3-dichlorophenyl)-1H-pyrazolo[3,4-b]pyrazin-6-yl)-4-methylpiperidin-4-yl)amino)methyl)-7-fluoro-1-oxoisoindolin-2-yl)piperidine-2,6-dione;
3-(5-((4-(3-(6-(4-amino-4-methylpiperidin-1-yl)-1H-pyrazolo[3,4-b]pyrazin-3-yl)-2-chlorophenyl)piperazin-1-yl)methyl)-1-oxoisoindolin-2-yl)piperidine-2,6-dione;
3-(5-((4-(3-(6-(4-amino-4-methylpiperidin-1-yl)-1H-pyrazolo[3,4-b]pyrazin-3-yl)-2-chlorophenyl)piperazin-1-yl)methyl)-4-fluoro-1-oxoisoindolin-2-yl)piperidine-2,6-dione;
3-(5-((4-(3-(6-(4-amino-4-methylpiperidin-1-yl)-1H-pyrazolo[3,4-b]pyrazin-3-yl)-2-chlorophenyl)piperazin-1-yl)methyl)-6-fluoro-1-oxoisoindolin-2-yl)piperidine-2,6-dione;
3-(5-((4-(3-(6-(4-amino-4-methylpiperidin-1-yl)-1H-pyrazolo[3,4-b]pyrazin-3-yl)-2-chlorophenyl)piperazin-1-yl)methyl)-7-fluoro-1-oxoisoindolin-2-yl)piperidine-2,6-dione;
3-(5-((4-(3-((3-amino-5-(4-amino-4-methylpiperidin-1-yl)pyrazin-2-yl)thio)-2-chlorophenyl)piperazin-1-yl)methyl)-1-oxoisoindolin-2-yl)piperidine-2,6-dione;
3-(5-((4-(3-((3-amino-5-(4-amino-4-methylpiperidin-1-yl)pyrazin-2-yl)thio)-2-chlorophenyl)piperazin-1-yl)methyl)-4-fluoro-1-oxoisoindolin-2-yl)piperidine-2,6-dione;
3-(5-((4-(3-((3-amino-5-(4-amino-4-methylpiperidin-1-yl)pyrazin-2-yl)thio)-2-chlorophenyl)piperazin-1-yl)methyl)-6-fluoro-1-oxoisoindolin-2-yl)piperidine-2,6-dione;
3-(5-((4-(3-((3-amino-5-(4-amino-4-methylpiperidin-1-yl)pyrazin-2-yl)thio)-2-chlorophenyl)piperazin-1-yl)methyl)-7-fluoro-1-oxoisoindolin-2-yl)piperidine-2,6-dione;
3-(5-(((3-((3-amino-5-(4-amino-4-methylpiperidin-1-yl)pyrazin-2-yl)thio)-2-chlorophenyl)amino)methyl)-4-fluoro-1-oxoisoindolin-2-yl)piperidine-2,6-dione;
3-(5-(((3-((3-amino-5-(4-amino-4-methylpiperidin-1-yl)pyrazin-2-yl)thio)-2-chlorophenyl)amino)methyl)-6-fluoro-1-oxoisoindolin-2-yl)piperidine-2,6-dione;
3-(5-(((3-((3-amino-5-(4-amino-4-methylpiperidin-1-yl)pyrazin-2-yl)thio)-2-chlorophenyl)amino)methyl)-7-fluoro-1-oxoisoindolin-2-yl)piperidine-2,6-dione;
3-(5-(((3-((3-amino-5-(4-amino-4-methylpiperidin-1-yl)pyrazin-2-yl)thio)-2-chlorophenyl)amino)methyl)-1-oxoisoindolin-2-yl)piperidine-2,6-dione;
3-(5-((4-(3-(3-amino-5-(4-amino-4-methylpiperidin-1-yl)pyrazin-2-yl)-2-chlorophenyl)piperazin-1-yl)methyl)-1-oxoisoindolin-2-yl)piperidine-2,6-dione;
3-(5-((4-(3-(3-amino-5-(4-amino-4-methylpiperidin-1-yl)pyrazin-2-yl)-2-chlorophenyl)piperazin-1-yl)methyl)-4-fluoro-1-oxoisoindolin-2-yl)piperidine-2,6-dione;
3-(5-((4-(3-(3-amino-5-(4-amino-4-methylpiperidin-1-yl)pyrazin-2-yl)-2-chlorophenyl)piperazin-1-yl)methyl)-6-fluoro-1-oxoisoindolin-2-yl)piperidine-2,6-dione;
3-(5-((4-(3-(3-amino-5-(4-amino-4-methylpiperidin-1-yl)pyrazin-2-yl)-2-chlorophenyl)piperazin-1-yl)methyl)-7-fluoro-1-oxoisoindolin-2-yl)piperidine-2,6-dione;
3-(5-((((3S,4S)-8-(6-amino-5-((2-amino-3-chloropyridin-4-yl)thio)pyrazin-2-yl)-3-methyl-2-oxa-8-azaspiro[4.5]decan-4-yl)amino)methyl)-1-oxoisoindolin-2-yl)piperidine-2,6-dione;
3-(5-((((3S,4S)-8-(6-amino-5-((2-amino-3-chloropyridin-4-yl)thio)pyrazin-2-yl)-3-methyl-2-oxa-8-azaspiro[4.5]decan-4-yl)amino)methyl)-4-fluoro-1-oxoisoindolin-2-yl)piperidine-2,6-dione;
3-(5-((((3S,4S)-8-(6-amino-5-((2-amino-3-chloropyridin-4-yl)thio)pyrazin-2-yl)-3-methyl-2-oxa-8-azaspiro[4.5]decan-4-yl)amino)methyl)-6-fluoro-1-oxoisoindolin-2-yl)piperidine-2,6-dione;
3-(5-((((3S,4S)-8-(6-amino-5-((2-amino-3-chloropyridin-4-yl)thio)pyrazin-2-yl)-3-methyl-2-oxa-8-azaspiro[4.5]decan-4-yl)amino)methyl)-7-fluoro-1-oxoisoindolin-2-yl)piperidine-2,6-dione;
3-(5-((((3S,4S)-8-(6-amino-5-((2-aminopyridin-4-yl)thio)pyrazin-2-yl)-3-methyl-2-oxa-8-azaspiro[4.5]decan-4-yl)amino)methyl)-7-fluoro-1-oxoisoindolin-2-yl)piperidine-2,6-dione;
3-(5-((((3S,4S)-8-(6-amino-5-((2-aminopyridin-4-yl)thio)pyrazin-2-yl)-3-methyl-2-oxa-8-azaspiro[4.5]decan-4-yl)amino)methyl)-1-oxoisoindolin-2-yl)piperidine-2,6-dione;
3-(5-((((3S,4S)-8-(6-amino-5-((2-aminopyridin-4-yl)thio)pyrazin-2-yl)-3-methyl-2-oxa-8-azaspiro[4.5]decan-4-yl)amino)methyl)-4-fluoro-1-oxoisoindolin-2-yl)piperidine-2,6-dione;
3-(5-((((3S,4S)-8-(6-amino-5-((2-aminopyridin-4-yl)thio)pyrazin-2-yl)-3-methyl-2-oxa-8-azaspiro[4.5]decan-4-yl)amino)methyl)-6-fluoro-1-oxoisoindolin-2-yl)piperidine-2,6-dione;
3-(5-((4-(4-((3-amino-5-((3S,4S)-4-amino-3-methyl-2-oxa-8-azaspiro[4.5]decan-8-yl)pyrazin-2-yl)thio)pyridin-2-yl)piperazin-1-yl)methyl)-1-oxoisoindolin-2-yl)piperidine-2,6-dione;
3-(5-((4-(4-((3-amino-5-((3S,4S)-4-amino-3-methyl-2-oxa-8-azaspiro[4.5]decan-8-yl)pyrazin-2-yl)thio)pyridin-2-yl)pip erazin-1-yl)methyl)-4-fl uoro-1-oxoisoindolin-2-yl)pip eridine-2, 6-dione;
3-(5-((4-(4-((3-amino-5-((3S,4S)-4-amino-3-methyl-2-oxa-8-azaspiro[4.5]decan-8-yl)pyrazin-2-yl)thio)pyridin-2-yl)pip erazin-1-yl)methyl)-6-fl uoro-1-oxoisoindolin-2-yl)pip eridine-2, 6-dione;
3-(5-((4-(4-((3-amino-5-((3S,4S)-4-amino-3-methyl-2-oxa-8-azaspiro[4.5]decan-8-yl)pyrazin-2-yl)thio)pyridin-2-yl)pip erazin-1-yl)methyl)-7-fl uoro-1-oxoisoindolin-2-yl)pip eridine-2, 6-dione;
3-(5-((4-(4-((3-amino-5-((3S,4S)-4-amino-3-methyl-2-oxa-8-azaspiro[4.5]decan-8-yl)pyrazin-2-yl)thio)-3-chloropyridin-2-yl)piperazin-1-yl)methyl)-1-oxoisoindolin-2-yl)piperidine-2,6-dione;
3-(5-((4-(4-((3-amino-5-((3S,4S)-4-amino-3-methyl-2-oxa-8-azaspiro[4.5]decan-8-yl)pyrazin-2-yl)thio)-3-chloropyridin-2-yl)piperazin-1-yl)methyl)-4-fluoro-1-oxoisoindolin-2-yl)piperidine-2,6-dione;
3-(5-((4-(4-((3-amino-5-((3S,4S)-4-amino-3-methyl-2-oxa-8-azaspiro[4.5]decan-8-yl)pyrazin-2-yl)thio)-3-chloropyridin-2-yl)piperazin-1-yl)methyl)-6-fluoro-1-oxoisoindolin-2-yl)piperidine-2,6-dione;
3-(5-((4-(4-((3-amino-5-((3S,4S)-4-amino-3-methyl-2-oxa-8-azaspiro[4.5]decan-8-yl)pyrazin-2-yl)thio)-3-chloropyridin-2-yl)piperazin-1-yl)methyl)-7-fluoro-1-oxoisoindolin-2-yl)piperidine-2,6-dione;
3-(5-(((1-(6-amino-5-(2,3-dichlorophenyl)pyrazin-2-yl)-4-methylpiperidin-4-yl)amino)methyl)-1-oxoisoindolin-2-yl)piperidine-2,6-dione;
3-(5-(((1-(6-amino-5-(2,3-dichlorophenyl)pyrazin-2-yl)-4-methylpiperidin-4-yl)amino)methyl)-4-fluoro-1-oxoisoindolin-2-yl)piperidine-2,6-dione;
3-(5-(((1-(6-amino-5-(2,3-dichlorophenyl)pyrazin-2-yl)-4-methylpiperidin-4-yl)amino)methyl)-6-fluoro-1-oxoisoindolin-2-yl)piperidine-2,6-dione;
3-(5-(((1-(6-amino-5-(2,3-dichlorophenyl)pyrazin-2-yl)-4-methylpiperidin-4-yl)amino)methyl)-7-fluoro-1-oxoisoindolin-2-yl)piperidine-2,6-dione;
3-(5-((2,3-difluoro-6-(2-morpholinothiazol-4-yl)phenoxy)methyl)-1-oxoisoindolin-2-yl)piperidine-2,6-dione;
3-(5-((2,3-difluoro-6-(2-morpholinothiazol-4-yl)phenoxy)methyl)-4-fluoro-1-oxoisoindolin-2-yl)piperidine-2,6-dione;
3-(5-((2,3-difluoro-6-(2-morpholinothiazol-4-yl)phenoxy)methyl)-7-fluoro-1-oxoisoindolin-2-yl)piperidine-2,6-dione;
3-(5-((2,3-difluoro-6-(2-morpholinothiazol-4-yl)phenoxy)methyl)-6-fluoro-1-oxoisoindolin-2-yl)piperidine-2,6-dione;
N-((1r,4r)-4-(3-chloro-4-cyanophenoxy)cyclohexyl)-6-(4-(4-((2-(2,6-dioxopiperidin-3-yl)-1-oxoisoindolin-5-yl)methyl)piperazin-1-yl)piperidin-1-yl)pyridazine-3-carboxamide;
N-((1r,4r)-4-(3-chloro-4-cyanophenoxy)cyclohexyl)-6-(4-(4-((2-(2,6-dioxopiperidin-3-yl)-4-fluoro-1-oxoisoindolin-5-yl)methyl)piperazin-1-yl)piperidin-1-yl)pyridazine-3-carboxamide;
N-((1r,4r)-4-(3-chloro-4-cyanophenoxy)cyclohexyl)-6-(4-(4-((2-(2,6-dioxopiperidin-3-yl)-6-fluoro-1-oxoisoindolin-5-yl)methyl)piperazin-1-yl)piperidin-1-yl)pyridazine-3-carboxamide;
N-((1r,4r)-4-(3-chloro-4-cyanophenoxy)cyclohexyl)-6-(4-(4-((2-(2,6-dioxopiperidin-3-yl)-7-fluoro-1-oxoisoindolin-5-yl)methyl)piperazin-1-yl)piperidin-1-yl)pyridazine-3-carboxamide;
N-((1r,3r)-3-(3-chloro-4-cyanophenoxy)-2,2,4,4-tetramethylcyclobutyl)-6-(4-((2-(2,6-dioxopiperidin-3-yl)-1-oxoisoindolin-5-yl)methyl)piperazin-1-yl)pyridazine-3-carboxamide;
N-((1r,3r)-3-(3-chloro-4-cyanophenoxy)-2,2,4,4-tetramethylcyclobutyl)-6-(4-((2-(2,6-dioxopiperidin-3-yl)-4-fluoro-1-oxoisoindolin-5-yl)methyl)piperazin-1-yl)pyridazine-3-carboxamide;
N-((1r,3r)-3-(3-chloro-4-cyanophenoxy)-2,2,4,4-tetramethylcyclobutyl)-6-(4-((2-(2,6-dioxopiperidin-3-yl)-6-fluoro-1-oxoisoindolin-5-yl)methyl)piperazin-1-yl)pyridazine-3-carboxamide;
N-((1r,3r)-3-(3-chloro-4-cyanophenoxy)-2,2,4,4-tetramethylcyclobutyl)-6-(4-((2-(2,6-dioxopiperidin-3-yl)-7-fluoro-1-oxoisoindolin-5-yl)methyl)piperazin-1-yl)pyridazine-3-carboxamide;
N-((1r,3r)-3-(3-chloro-4-cyanophenoxy)-2,2,4,4-tetramethylcyclobutyl)-6-(4-((2-(2,6-dioxopiperidin-3-yl)-1-oxoisoindolin-5-yl)methyl)piperazin-1-yl)nicotinamide;
N-((1r,3r)-3-(3-chloro-4-cyanophenoxy)-2,2,4,4-tetramethylcyclobutyl)-6-(4-((2-(2,6-dioxopip eridin-3 -yl)-4-fluoro-1-oxoisoindolin-5-yl)methyl)pip erazin-1-yl)ni cotinami de;
N-((1r,3r)-3-(3-chloro-4-cyanophenoxy)-2,2,4,4-tetramethylcyclobutyl)-6-(4-((2-(2,6-dioxopip eridin-3 -yl)-6-fluoro-1-oxoisoindolin-5-yl)methyl)pip erazin-1-yl)ni cotinami de;
N-((1r,3r)-3-(3-chloro-4-cyanophenoxy)-2,2,4,4-tetramethylcyclobutyl)-6-(4-((2-(2,6-dioxopip eridin-3 -yl)-7-fluoro-1-oxoisoindolin-5-yl)methyl)pip erazin-1-yl)ni cotinami de;
N-((1r,4r)-4-(3-chloro-4-cyanophenoxy)cyclohexyl)-6-(8-((2-(2,6-dioxopiperidin-3-yl)-1-oxoisoindolin-5-yl)methyl)-3,8-diazabicyclo[3.2.1]octan-3-yl)pyridazine-3-carboxamide;
N-((1r,4r)-4-(3-chloro-4-cyanophenoxy)cyclohexyl)-6-(8-((2-(2,6-dioxopiperidin-3-yl)-4-fluoro-1-oxoisoindolin-5-yl)methyl)-3,8-diazabicyclo[3.2.1]octan-3-yl)pyridazine-3-carboxamide;
N-((1r,4r)-4-(3-chloro-4-cyanophenoxy)cyclohexyl)-6-(8-((2-(2,6-dioxopiperidin-3-yl)-6-fluoro-1-oxoisoindolin-5-yl)methyl)-3,8-diazabicyclo[3.2.1]octan-3-yl)pyridazine-3-carboxamide;
N-((1r,4r)-4-(3-chloro-4-cyanophenoxy)cyclohexyl)-6-(8-((2-(2,6-dioxopiperidin-3-yl)-7-fluoro-1-oxoisoindolin-5-yl)methyl)-3,8-diazabicyclo[3.2.1]octan-3-yl)pyridazine-3-carboxamide;
N-((1r,4r)-4-(3-chloro-4-cyanophenoxy)cyclohexyl)-6-(6-((2-(2,6-dioxopiperidin-3-yl)-1-oxoisoindolin-5-yl)methyl)-3,6-di azabi cyclo[3.1.1]heptan-3-yl)pyridazine-3-carboxami de;
N-((1r,4r)-4-(3-chloro-4-cyanophenoxy)cyclohexyl)-6-(6-((2-(2,6-dioxopiperidin-3-yl)-4-fluoro-1-oxoisoindolin-5-yl)methyl)-3,6-diazabicyclo[3.1.1]heptan-3-yl)pyridazine-3-carboxamide;
N-((1r,4r)-4-(3-chloro-4-cyanophenoxy)cyclohexyl)-6-(6-((2-(2,6-dioxopiperidin-3-yl)-6-fluoro-1-oxoisoindolin-5-yl)methyl)-3,6-diazabicyclo[3.1.1]heptan-3-yl)pyridazine-3-carboxamide;
N-((1r,4r)-4-(3-chloro-4-cyanophenoxy)cyclohexyl)-6-(6-((2-(2,6-dioxopiperidin-3-yl)-7-fluoro-1-oxoisoindolin-5-yl)methyl)-3,6-diazabicyclo[3.1.1]heptan-3-yl)pyridazine-3-carboxamide;
N-((1r,4r)-4-(3-chloro-4-cyanophenoxy)cyclohexyl)-6-(3-((2-(2,6-dioxopiperidin-3-yl)-1-oxoisoindolin-5-yl)methyl)-3,6-di azabi cyclo[3.1.1]heptan-6-yl)pyridazine-3-carboxami de;
N-((1r,4r)-4-(3-chloro-4-cyanophenoxy)cyclohexyl)-6-(3-((2-(2,6-dioxopiperidin-3-yl)-4-fluoro-1-oxoisoindolin-5-yl)methyl)-3,6-diazabicyclo[3.1.1]heptan-6-yl)pyridazine-3-carboxamide;
N-((1r,4r)-4-(3-chloro-4-cyanophenoxy)cyclohexyl)-6-(3-((2-(2,6-dioxopiperidin-3-yl)-6-fluoro-1-oxoisoindolin-5-yl)methyl)-3,6-diazabicyclo[3.1.1]heptan-6-yl)pyridazine-3-carboxamide;
N-((1r,4r)-4-(3-chloro-4-cyanophenoxy)cyclohexyl)-6-(3-((2-(2,6-dioxopiperidin-3-yl)-7-fluoro-1-oxoisoindolin-5-yl)methyl)-3,6-diazabicyclo[3.1.1]heptan-6-yl)pyridazine-3-carboxamide;
N-((1r,4r)-4-(3-chloro-4-cyanophenoxy)cyclohexyl)-6-(3-((2-(2,6-dioxopiperidin-3-yl)-1-oxoisoindolin-5-yl)methyl)-3,8-diazabicyclo[3.2.1]octan-8-yl)pyridazine-3-carboxamide;
N-((1r,4r)-4-(3-chloro-4-cyanophenoxy)cyclohexyl)-6-(3-((2-(2,6-dioxopiperidin-3-yl)-4-fluoro-1-oxoisoindolin-5-yl)methyl)-3,8-diazabicyclo[3.2.1]octan-8-yl)pyridazine-3-carboxamide;
N-((1r,4r)-4-(3-chloro-4-cyanophenoxy)cyclohexyl)-6-(3-((2-(2,6-dioxopiperidin-3-yl)-6-fluoro-1-oxoisoindolin-5-yl)methyl)-3,8-diazabicyclo[3.2.1]octan-8-yl)pyridazine-3-carboxamide;
N-((1r,4r)-4-(3-chloro-4-cyanophenoxy)cyclohexyl)-6-(3-((2-(2,6-dioxopiperidin-3-yl)-7-fluoro-1-oxoisoindolin-5-yl)methyl)-3,8-diazabicyclo[3.2.1]octan-8-yl)pyridazine-3-carboxamide;
N-((1r,4r)-4-(3-chloro-4-cyanophenoxy)cyclohexyl)-6-(4-((2-(2,6-dioxopiperidin-3-yl)-1-oxoisoindolin-5-yl)methyl)piperazin-1-yl)pyridazine-3-carboxamide;
N-((1r,4r)-4-(3-chloro-4-cyanophenoxy)cyclohexyl)-6-(4-((2-(2,6-dioxopiperidin-3-yl)-4-fluoro-1-oxoisoindolin-5-yl)methyl)piperazin-1-yl)pyridazine-3-carboxamide;
N-((1r,4r)-4-(3-chloro-4-cyanophenoxy)cyclohexyl)-6-(4-((2-(2,6-dioxopiperidin-3-yl)-6-fluoro-1-oxoisoindolin-5-yl)methyl)piperazin-1-yl)pyridazine-3-carboxamide;
N-((1r,4r)-4-(3-chloro-4-cyanophenoxy)cyclohexyl)-6-(4-((2-(2,6-dioxopiperidin-3-yl)-7-fluoro-1-oxoisoindolin-5-yl)methyl)piperazin-1-yl)pyridazine-3-carboxamide;
3-(5-((4-(4-((9-cyclopentyl-8-(phenylamino)-9H-purin-2-yl)amino)phenyl)piperazin-1-yl)methyl)-1-oxoisoindolin-2-yl)piperidine-2,6-dione;
3-(5-((4-(4-((9-cyclopentyl-8-(phenylamino)-9H-purin-2-yl)amino)phenyl)piperazin-1-yl)methyl)-4-fluoro-1-oxoisoindolin-2-yl)piperidine-2,6-dione;
3-(5-((4-(4-((9-cyclopentyl-8-(phenylamino)-9H-purin-2-yl)amino)phenyl)piperazin-1-yl)methyl)-6-fluoro-1-oxoisoindolin-2-yl)piperidine-2,6-dione;
3-(5-((4-(4-((9-cyclopentyl-8-(phenylamino)-9H-purin-2-yl)amino)phenyl)piperazin-1-yl)methyl)-7-fluoro-1-oxoisoindolin-2-yl)piperidine-2,6-dione;
3-(5-(((1-(4-((9-cyclopentyl-8-(phenylamino)-9H-purin-2-yl)amino)phenyl)piperidin-4-yl)(methyl)amino)methyl)-1-oxoisoindolin-2-yl)piperidine-2,6-dione;
3-(5-(((1-(4-((9-cyclopentyl-8-(phenylamino)-9H-purin-2-yl)amino)phenyl)piperidin-4-yl)(methyl)amino)methyl)-4-fluoro-1-oxoisoindolin-2-yl)piperidine-2,6-dione;
3-(5-(((1-(4-((9-cyclopentyl-8-(phenylamino)-9H-purin-2-yl)amino)phenyl)piperidin-4-yl)(methyl)amino)methyl)-6-fluoro-1-oxoisoindolin-2-yl)piperidine-2,6-dione;
3-(5-(((1-(4-((9-cyclopentyl-8-(phenylamino)-9H-purin-2-yl)amino)phenyl)piperidin-4-yl)(methyl)amino)methyl)-7-fluoro-1-oxoisoindolin-2-yl)piperidine-2,6-dione;
3-(5-((4-(1-(4-((9-cyclopentyl-8-(phenylamino)-9H-purin-2-yl)amino)phenyl)piperidin-4-yl)piperazin-1-yl)methyl)-1-oxoisoindolin-2-yl)piperidine-2,6-dione;
3-(5-((4-(1-(4-((9-cyclopentyl-8-(phenylamino)-9H-purin-2-yl)amino)phenyl)piperidin-4-yl)piperazin-1-yl)methyl)-4-fluoro-1-oxoisoindolin-2-yl)piperidine-2,6-dione;
3-(5-((4-(1-(4-((9-cyclopentyl-8-(phenylamino)-9H-purin-2-yl)amino)phenyl)piperidin-4-yl)piperazin-1-yl)methyl)-6-fluoro-1-oxoisoindolin-2-yl)piperidine-2,6-dione;
3-(5-((4-(1-(4-((9-cyclopentyl-8-(phenylamino)-9H-purin-2-yl)amino)phenyl)piperidin-4-yl)piperazin-1-yl)methyl)-7-fluoro-1-oxoisoindolin-2-yl)piperidine-2,6-dione;
3-(5-((4-(4-(4-((9-cyclopentyl-8-(phenylamino)-9H-purin-2-yl)amino)phenyl)piperazin-1-yl)piperidin-1 -yl)methyl)-1 -oxoisoindolin-2-yl)piperidine-2,6-dione;
3-(5-((4-(4-(4-((9-cyclopentyl-8-(phenylamino)-9H-purin-2-yl)amino)phenyl)piperazin-1-yl)piperidin-1-yl)methyl)-4-fluoro-1-oxoisoindolin-2-yl)piperidine-2,6-dione;
3-(5-((4-(4-(4-((9-cyclopentyl-8-(phenylamino)-9H-purin-2-yl)amino)phenyl)piperazin-1-yl)pip eridin-1-yl)methyl)-6-fl uoro-1-oxoisoindolin-2-yl)pip eri dine-2, 6-dione;
3-(5-((4-(4-(4-((9-cyclopentyl-8-(phenylamino)-9H-purin-2-yl)amino)phenyl)piperazin-1-yl)piperidin-1-yl)methyl)-7-fluoro-1-oxoisoindolin-2-yl)piperidine-2,6-dione;
3-(5-(((5-methoxy-4-((4-(1-methyl-1H-indol-3-yl)pyrimidin-2-yl)amino)-2-nitrophenyl)amino)methyl)-1-oxoisoindolin-2-yl)piperidine-2,6-dione;
3-(4-fluoro-5-(((5-methoxy-4-((4-(1-methyl-1H-indol-3-yl)pyrimidin-2-yl)amino)-2-nitrophenyl)amino)methyl)-1-oxoisoindolin-2-yl)piperidine-2,6-dione;
3-(6-fluoro-5-(((5-methoxy-4-((4-(1-methyl-1H-indol-3-yl)pyrimidin-2-yl)amino)-2-nitrophenyl)amino)methyl)-1-oxoisoindolin-2-yl)piperidine-2,6-dione;
3-(7-fluoro-5-(((5-methoxy-4-((4-(1-methyl-1H-indol-3-yl)pyrimidin-2-yl)amino)-2-nitrophenyl)amino)methyl)-1-oxoisoindolin-2-yl)piperidine-2,6-dione;
N-(2-((2-(((2-(2,6-dioxopiperidin-3-yl)-1-oxoisoindolin-5-yl)methyl)(methyl)amino)ethyl)(methyl)amino)-4-methoxy-5-((4-(1-methyl-1H-indol-3-yl)pyrimidin-2-yl)amino)phenyl)acrylamide;
N-(2-((2-(((2-(2,6-dioxopiperidin-3-yl)-4-fluoro-1-oxoisoindolin-5-yl)methyl)(methyl)amino)ethyl)(methyl)amino)-4-methoxy-5-((4-(1-methyl-1H-indol-3-yl)pyrimidin-2-yl)amino)phenyl)acrylamide;
N-(2-((2-(((2-(2,6-dioxopiperidin-3-yl)-6-fluoro-1-oxoisoindolin-5-yl)methyl)(methyl)amino)ethyl)(methyl)amino)-4-methoxy-5-((4-(1-methyl-1H-indol-3-yl)pyrimidin-2-yl)amino)phenyl)acrylamide;
N-(2-((2-(((2-(2,6-dioxopiperidin-3-yl)-7-fluoro-1-oxoisoindolin-5-yl)methyl)(methyl)amino)ethyl)(methyl)amino)-4-methoxy-5-((4-(1-methyl-1H-indol-3-yl)pyrimidin-2-yl)amino)phenyl)acrylamide;
3-(5-((4-(3-((4-((3-chloro-4-fluorophenyl)amino)-7-methoxyquinazolin-6-yl)oxy)propyl)piperazin-1-yl)methyl)-1-oxoisoindolin-2-yl)piperidine-2,6-dione;
3-(5-((4-(3-((4-((3-chloro-4-fluorophenyl)amino)-7-methoxyquinazolin-6-yl)oxy)propyl)piperazin-1-yl)methyl)-4-fluoro-1-oxoisoindolin-2-yl)piperidine-2,6-dione;
3-(5-((4-(3-((4-((3-chloro-4-fluorophenyl)amino)-7-methoxyquinazolin-6-yl)oxy)propyl)piperazin-1-yl)methyl)-6-fluoro-1-oxoisoindolin-2-yl)piperidine-2,6-dione;
3-(5-((4-(3-((4-((3-chloro-4-fluorophenyl)amino)-7-methoxyquinazolin-6-yl)oxy)propyl)piperazin-1-yl)methyl)-7-fluoro-1-oxoisoindolin-2-yl)piperidine-2,6-dione;
N-(4-((3-chloro-4-fluorophenyl)amino)-7-(3-(4-((2-(2,6-dioxopiperidin-3-yl)-1-oxoisoindolin-5-yl)methyl)piperazin-1-yl)propoxy)quinazolin-6-yl)acrylamide;
N-(4-((3-chloro-4-fluorophenyl)amino)-7-(3-(4-((2-(2,6-dioxopiperidin-3-yl)-4-fluoro-1-oxoisoindolin-5-yl)methyl)piperazin-1-yl)propoxy)quinazolin-6-yl)acrylamide;
N-(4-((3-chloro-4-fluorophenyl)amino)-7-(3-(4-((2-(2,6-dioxopiperidin-3-yl)-6-fluoro-1-oxoisoindolin-5-yl)methyl)piperazin-1-yl)propoxy)quinazolin-6-yl)acrylamide;
N-(4-((3-chloro-4-fluorophenyl)amino)-7-(3-(4-((2-(2,6-dioxopiperidin-3-yl)-7-fluoro-1-oxoisoindolin-5-yl)methyl)piperazin-1-yl)propoxy)quinazolin-6-yl)acrylamide;
3-(5-(((4-((3,4-dichloro-2-fluorophenyl)amino)-7-methoxyquinazolin-6-yl)oxy)methyl)-1-oxoisoindolin-2-yl)piperidine-2,6-dione;
3-(5-(((4-((3,4-dichloro-2-fluorophenyl)amino)-7-methoxyquinazolin-6-yl)oxy)methyl)-4-fluoro-1-oxoisoindolin-2-yl)piperidine-2,6-dione;
3-(5-(((4-((3,4-dichloro-2-fluorophenyl)amino)-7-methoxyquinazolin-6-yl)oxy)methyl)-6-fluoro-1-oxoisoindolin-2-yl)piperidine-2,6-dione;
3-(5-(((4-((3,4-dichloro-2-fluorophenyl)amino)-7-methoxyquinazolin-6-yl)oxy)methyl)-7-fluoro-1-oxoisoindolin-2-yl)piperidine-2,6-dione;
3-(5-((4-((4-((3,4-dichloro-2-fluorophenyl)amino)-7-methoxyquinazolin-6-yl)oxy)piperidin-1-yl)methyl)-1-oxoisoindolin-2-yl)piperidine-2,6-dione;
3-(5-((4-((4-((3,4-dichloro-2-fluorophenyl)amino)-7-methoxyquinazolin-6-yl)oxy)piperidin-1-yl)methyl)-4-fluoro-1-oxoisoindolin-2-yl)piperidine-2,6-dione;
3-(5-((4-((4-((3,4-dichloro-2-fluorophenyl)amino)-7-methoxyquinazolin-6-yl)oxy)piperidin-1-yl)methyl)-6-fluoro-1-oxoisoindolin-2-yl)piperidine-2,6-dione;
3-(5-((4-((4-((3,4-dichloro-2-fluorophenyl)amino)-7-methoxyquinazolin-6-yl)oxy)piperidin-1-yl)methyl)-7-fluoro-1-oxoisoindolin-2-yl)piperidine-2,6-dione;
3-(5-(((4-((3-chloro-4-fluorophenyl)amino)-7-(((S)-tetrahydrofuran-3-yl)oxy)quinazolin-6-yl)amino)methyl)-1-oxoisoindolin-2-yl)piperidine-2,6-dione;
3-(5-(((4-((3-chloro-4-fluorophenyl)amino)-7-(((S)-tetrahydrofuran-3-yl)oxy)quinazolin-6-yl)amino)methyl)-4-fluoro-1-oxoisoindolin-2-yl)piperidine-2,6-dione;
3-(5-(((4-((3-chloro-4-fluorophenyl)amino)-7-(((S)-tetrahydrofuran-3-yl)oxy)quinazolin-6-yl)amino)methyl)-6-fluoro-1-oxoisoindolin-2-yl)piperidine-2,6-dione;
3-(5-(((4-((3-chloro-4-fluorophenyl)amino)-7-(((S)-tetrahydrofuran-3-yl)oxy)quinazolin-6-yl)amino)methyl)-7-fluoro-1-oxoisoindolin-2-yl)piperidine-2,6-dione;
3-(5-(((4-((3-chloro-2-fluorophenyl)amino)-7-methoxyquinazolin-6-yl)oxy)methyl)-1-oxoisoindolin-2-yl)piperidine-2,6-dione;
3-(5-(((4-((3-chloro-2-fluorophenyl)amino)-7-methoxyquinazolin-6-yl)oxy)methyl)-4-fluoro-1-oxoisoindolin-2-yl)piperidine-2,6-dione;
3-(5-(((4-((3-chloro-2-fluorophenyl)amino)-7-methoxyquinazolin-6-yl)oxy)methyl)-6-fluoro-1-oxoisoindolin-2-yl)piperidine-2,6-dione;
3-(5-(((4-((3-chloro-2-fluorophenyl)amino)-7-methoxyquinazolin-6-yl)oxy)methyl)-7-fluoro-1-oxoisoindolin-2-yl)piperidine-2,6-dione;
3-(5-(((4-((3-chloro-4-fluorophenyl)amino)-7-methoxyquinazolin-6-yl)oxy)methyl)-1-oxoisoindolin-2-yl)piperidine-2,6-dione;
3-(5-(((4-((3-chloro-4-fluorophenyl)amino)-7-methoxyquinazolin-6-yl)oxy)methyl)-4-fluoro-1-oxoisoindolin-2-yl)piperidine-2,6-dione;
3-(5-(((4-((3-chloro-4-fluorophenyl)amino)-7-methoxyquinazolin-6-yl)oxy)methyl)-6-fluoro-1-oxoisoindolin-2-yl)piperidine-2,6-dione;
3-(5-(((4-((3-chloro-4-fluorophenyl)amino)-7-methoxyquinazolin-6-yl)oxy)methyl)-7-fluoro-1-oxoisoindolin-2-yl)piperidine-2,6-dione;
3-(5-(((4-((3-chloro-4-fluorophenyl)amino)-7-methoxyquinazolin-6-yl)amino)methyl)-1-oxoisoindolin-2-yl)piperidine-2,6-dione;
3-(5-(((4-((3-chloro-4-fluorophenyl)amino)-7-methoxyquinazolin-6-yl)amino)methyl)-4-fluoro-1-oxoisoindolin-2-yl)piperidine-2,6-dione;
3-(5-(((4-((3-chloro-4-fluorophenyl)amino)-7-methoxyquinazolin-6-yl)amino)methyl)-6-fluoro-1-oxoisoindolin-2-yl)piperidine-2,6-dione;
3-(5-(((4-((3-chloro-4-fluorophenyl)amino)-7-methoxyquinazolin-6-yl)amino)methyl)-7-fluoro-1-oxoisoindolin-2-yl)piperidine-2,6-dione;
(E)-N-(4-((3-chloro-4-fluorophenyl)amino)-7-methoxyquinazolin-6-yl)-4-(4-((2-(2,6-dioxopiperidin-3-yl)-1-oxoisoindolin-5-yl)methyl)piperazin-1-yl)but-2-enamide;
(E)-N-(4-((3-chloro-4-fluorophenyl)amino)-7-methoxyquinazolin-6-yl)-4-(4-((2-(2,6-dioxopiperidin-3-yl)-4-fluoro-1-oxoisoindolin-5-yl)methyl)piperazin-1-yl)but-2-enamide;
(E)-N-(4-((3-chloro-4-fluorophenyl)amino)-7-methoxyquinazolin-6-yl)-4-(4-((2-(2,6-dioxopiperidin-3-yl)-6-fluoro-1-oxoisoindolin-5-yl)methyl)piperazin-1-yl)but-2-enamide;
(E)-N-(4-((3-chloro-4-fluorophenyl)amino)-7-methoxyquinazolin-6-yl)-4-(4-((2-(2,6-dioxopiperidin-3-yl)-7-fluoro-1-oxoisoindolin-5-yl)methyl)piperazin-1-yl)but-2-enamide;
2-(7-(((2-(2,6-dioxopiperidin-3-yl)-1-oxoisoindolin-5-yl)methyl)amino)-1-oxoisoindolin-2-yl)-2-(5-fluoro-2-hydroxyphenyl)-N-(thiazol-2-yl)acetamide;
2-(7-(((2-(2,6-dioxopiperidin-3-yl)-4-fluoro-1-oxoisoindolin-5-yl)methyl)amino)-1-oxoisoindolin-2-yl)-2-(5-fluoro-2-hydroxyphenyl)-N-(thiazol-2-yl)acetamide;
2-(7-(((2-(2,6-dioxopiperidin-3-yl)-6-fluoro-1-oxoisoindolin-5-yl)methyl)amino)-1-oxoisoindolin-2-yl)-2-(5-fluoro-2-hydroxyphenyl)-N-(thiazol-2-yl)acetamide;
2-(7-(((2-(2,6-dioxopiperidin-3-yl)-7-fluoro-1-oxoisoindolin-5-yl)methyl)amino)-1-oxoisoindolin-2-yl)-2-(5-fluoro-2-hydroxyphenyl)-N-(thiazol-2-yl)acetamide;
2-(6-(4-((2-(2,6-dioxopiperidin-3-yl)-1-oxoisoindolin-5-yl)methyl)piperazin-1-yl)-1-oxoisoindolin-2-yl)-2-(5-fluoro-2-hydroxyphenyl)-N-(thiazol-2-yl)acetamide;
2-(6-(4-((2-(2,6-dioxopiperidin-3-yl)-4-fluoro-1-oxoisoindolin-5-yl)methyl)piperazin-1-yl)-1-oxoisoindolin-2-yl)-2-(5-fluoro-2-hydroxyphenyl)-N-(thiazol-2-yl)acetamide;
2-(6-(4-((2-(2,6-dioxopiperidin-3-yl)-6-fluoro-1-oxoisoindolin-5-yl)methyl)piperazin-1-yl)-1-oxoisoindolin-2-yl)-2-(5-fluoro-2-hydroxyphenyl)-N-(thiazol-2-yl)acetamide;
2-(6-(4-((2-(2,6-dioxopiperidin-3-yl)-7-fluoro-1-oxoisoindolin-5-yl)methyl)piperazin-1-yl)-1-oxoisoindolin-2-yl)-2-(5-fluoro-2-hydroxyphenyl)-N-(thiazol-2-yl)acetamide;
2-(6-(4-(((2-(2,6-dioxopiperidin-3-yl)-1-oxoisoindolin-5-yl)methyl)(methyl)amino)piperidin-1-yl)-1-oxoisoindolin-2-yl)-2-(5-fluoro-2-hydroxyphenyl)-N-(thiazol-2-yl)acetamide;
2-(6-(4-(((2-(2,6-dioxopiperidin-3-yl)-4-fluoro-1-oxoisoindolin-5-yl)methyl)(methyl)amino)piperidin-1-yl)-1-oxoisoindolin-2-yl)-2-(5-fluoro-2-hydroxyphenyl)-N-(thiazol-2-yl)acetamide;
2-(6-(4-(((2-(2,6-dioxopiperidin-3-yl)-6-fluoro-1-oxoisoindolin-5-yl)methyl)(methyl)amino)piperidin-1-yl)-1-oxoisoindolin-2-yl)-2-(5-fluoro-2-hydroxyphenyl)-N-(thiazol-2-yl)acetamide;
2-(6-(4-(((2-(2,6-dioxopiperidin-3-yl)-7-fluoro-1-oxoisoindolin-5-yl)methyl)(methyl)amino)piperidin-1-yl)-1-oxoisoindolin-2-yl)-2-(5-fluoro-2-hydroxyphenyl)-N-(thiazol-2-yl)acetamide;
2-(6-(4-(4-((2-(2,6-dioxopiperidin-3-yl)-1-oxoisoindolin-5-yl)methyl)piperazin-1-yl)piperidin-1-yl)-1-oxoisoindolin-2-yl)-2-(5-fluoro-2-hydroxyphenyl)-N-(thiazol-2-yl)acetamide;
2-(6-(4-(4-((2-(2,6-dioxopiperidin-3-yl)-4-fluoro-1-oxoisoindolin-5-yl)methyl)piperazin-1-yl)piperidin-1-yl)-1-oxoisoindolin-2-yl)-2-(5-fluoro-2-hydroxyphenyl)-N-(thiazol-2-yl)acetamide;
2-(6-(4-(4-((2-(2,6-dioxopiperidin-3-yl)-6-fluoro-1-oxoisoindolin-5-yl)methyl)piperazin-1-yl)piperidin-1-yl)-1-oxoisoindolin-2-yl)-2-(5-fluoro-2-hydroxyphenyl)-N-(thiazol-2-yl)acetamide;
2-(6-(4-(4-((2-(2,6-dioxopiperidin-3-yl)-7-fluoro-1-oxoisoindolin-5-yl)methyl)piperazin-1-yl)piperidin-1-yl)-1-oxoisoindolin-2-yl)-2-(5-fluoro-2-hydroxyphenyl)-N-(thiazol-2-yl)acetamide;
2-(6-(4-(4-((2-(2,6-dioxopiperidin-3-yl)-1-oxoisoindolin-5-yl)methyl)piperazin-1-yl)phenyl)-1-oxoisoindolin-2-yl)-2-(5-fluoro-2-hydroxyphenyl)-N-(thiazol-2-yl)acetamide;
2-(6-(4-(4-((2-(2,6-dioxopiperidin-3-yl)-4-fluoro-1-oxoisoindolin-5-yl)methyl)piperazin-1-yl)phenyl)-1-oxoisoindolin-2-yl)-2-(5-fluoro-2-hydroxyphenyl)-N-(thiazol-2-yl)acetamide;
2-(6-(4-(4-((2-(2,6-dioxopiperidin-3-yl)-6-fluoro-1-oxoisoindolin-5-yl)methyl)piperazin-1-yl)phenyl)-1-oxoisoindolin-2-yl)-2-(5-fluoro-2-hydroxyphenyl)-N-(thiazol-2-yl)acetamide;
2-(6-(4-(4-((2-(2,6-dioxopiperidin-3-yl)-7-fluoro-1-oxoisoindolin-5-yl)methyl)piperazin-1-yl)phenyl)-1-oxoisoindolin-2-yl)-2-(5-fluoro-2-hydroxyphenyl)-N-(thiazol-2-yl)acetamide;
2-(6-(6-(4-((2-(2,6-dioxopiperidin-3-yl)-1-oxoisoindolin-5-yl)methyl)piperazin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((2-(2,6-dioxopiperidin-3-yl)-4-fluoro-1-oxoisoindolin-5-yl)methyl)piperazin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((2-(2,6-dioxopiperidin-3-yl)-6-fluoro-1-oxoisoindolin-5-yl)methyl)piperazin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((2-(2,6-dioxopiperidin-3-yl)-7-fluoro-1-oxoisoindolin-5-yl)methyl)piperazin-1-yl)pyridazin-3-yl)-1-oxoisoindolin-2-yl)-2-(5-fluoro-2-hydroxyphenyl)-N-(thiazol-2-yl)acetamide;
3-(1-oxo-5-((4-(((R,E)-1¹,2⁶,7-trimethyl-3-oxo-5²,5³-dihydro-1¹H,5¹H-11-oxa-4-aza-5(2,1)-benzo[d]imidazola-2(2,4)-pyridina-1(4,5)-pyrazolacycloundecaphane-5⁶-yl)methyl)piperazin-1-yl)methyl)isoindolin-2-yl)piperidine-2,6-dione;
3-(4-fluoro-1-oxo-5-((4-(((R,E)-1¹,2⁶,7-trimethyl-3-oxo-5²,5³-dihydro-1¹H,5¹H-11-oxa-4-aza-5(2,1)-benzo[d]imidazola-2(2,4)-pyridina-1(4,5)-pyrazolacycloundecaphane-5⁶-yl)methyl)piperazin-1-yl)methyl)isoindolin-2-yl)piperidine-2,6-dione;
3-(7-fluoro-1-oxo-5-((4-(((R,E)-1¹,2⁶,7-trimethyl-3-oxo-5²,5³-dihydro-1¹H,5¹H-11-oxa-4-aza-5(2,1)-benzo[d]imidazola-2(2,4)-pyridina-1(4,5)-pyrazolacycloundecaphane-5⁶-yl)methyl)piperazin-1-yl)methyl)isoindolin-2-yl)piperidine-2,6-dione;
3-(6-fluoro-1-oxo-5-((4-(((R,E)-1¹,2⁶,7-trimethyl-3-oxo-5²,5³-dihydro-1¹H,5¹H-11-oxa-4-aza-5(2,1)-benzo[d]imidazola-2(2,4)-pyridina-1(4,5)-pyrazolacycloundecaphane-5⁶-yl)methyl)piperazin-1-yl)methyl)isoindolin-2-yl)piperidine-2,6-dione;
3-(1-oxo-5-((4-((R,E)-1¹,2⁶,7-trimethyl-3-oxo-5²,5³-dihydro-1¹H,5¹H-11-oxa-4-aza-5(2,1)-benzo[d]imidazola-2(2,4)-pyridina-1(4,5)-pyrazolacycloundecaphane-5⁶-yl)piperazin-1-yl)methyl)isoindolin-2-yl)piperidine-2,6-dione;
3-(4-fluoro-1-oxo-5-((4-((R,E)-1¹,2⁶,7-trimethyl-3-oxo-5²,5³-dihydro-1¹H,5¹H-11-oxa-4-aza-5(2,1)-benzo[d]imidazola-2(2,4)-pyridina-1(4,5)-pyrazolacycloundecaphane-5⁶-yl)piperazin-1-yl)methyl)isoindolin-2-yl)piperidine-2,6-dione;
3-(6-fluoro-1-oxo-5-((4-((R,E)-1¹,2⁶,7-trimethyl-3-oxo-5²,5³-dihydro-1¹H,5¹H-11-oxa-4-aza-5(2,1)-benzo[d]imidazola-2(2,4)-pyridina-1(4,5)-pyrazolacycloundecaphane-5⁶-yl)piperazin-1-yl)methyl)isoindolin-2-yl)piperidine-2,6-dione;
3-(7-fluoro-1-oxo-5-((4-((R,E)-1¹,2⁶,7-trimethyl-3-oxo-5²,5³-dihydro-1¹H,5¹H-11-oxa-4-aza-5(2,1)-benzo[d]imidazola-2(2,4)-pyridina-1(4,5)-pyrazolacycloundecaphane-5⁶-yl)piperazin-1-yl)methyl)isoindolin-2-yl)piperidine-2,6-dione;
3-(5-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)-1-oxoisoindolin-2-yl)piperidine-2,6-dione;
3-(5-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)-4-fluoro-1-oxoisoindolin-2-yl)piperidine-2,6-dione;
3-(5-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)-6-fluoro-1-oxoisoindolin-2-yl)piperidine-2,6-dione;
3-(5-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)-7-fluoro-1-oxoisoindolin-2-yl)piperidine-2,6-dione;
3-(5-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)-1-oxoisoindolin-2-yl)piperidine-2,6-dione;
3-(5-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)-4-fluoro-1-oxoisoindolin-2-yl)piperidine-2,6-dione;
3-(5-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)-6-fluoro-1-oxoisoindolin-2-yl)piperidine-2,6-dione;
3-(5-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)-7-fluoro-1-oxoisoindolin-2-yl)piperidine-2,6-dione;
3-(5-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)-1-oxoisoindolin-2-yl)piperidine-2,6-dione;
3-(5-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)-4-fluoro-1-oxoisoindolin-2-yl)piperidine-2,6-dione;
3-(5-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)-6-fluoro-1-oxoisoindolin-2-yl)piperidine-2,6-dione;
3-(5-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)-7-fluoro-1-oxoisoindolin-2-yl)piperidine-2,6-dione;
3-(5-((4-(4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)piperidin-1-yl)methyl)-1-oxoisoindolin-2-yl)piperidine-2,6-dione;
3-(5-((4-(4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)piperidin-1-yl)methyl)-4-fluoro-1-oxoisoindolin-2-yl)piperidine-2,6-dione;
3-(5-((4-(4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)piperidin-1-yl)methyl)-6-fluoro-1-oxoisoindolin-2-yl)piperidine-2,6-dione;
3-(5-((4-(4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)piperidin-1-yl)methyl)-7-fluoro-1-oxoisoindolin-2-yl)piperidine-2,6-dione;
3-(5-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)-1-oxoisoindolin-2-yl)piperidine-2,6-dione;
3-(4-fluoro-5-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)-1-oxoisoindolin-2-yl)piperidine-2,6-dione;
3-(6-fluoro-5-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)-1-oxoisoindolin-2-yl)piperidine-2,6-dione;
3-(7-fluoro-5-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)-1-oxoisoindolin-2-yl)piperidine-2,6-dione;
3-(5-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)-1-oxoisoindolin-2-yl)piperidine-2,6-dione;
3-(4-fluoro-5-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)-1-oxoisoindolin-2-yl)piperidine-2,6-dione;
3-(6-fluoro-5-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)-1-oxoisoindolin-2-yl)piperidine-2,6-dione;
3-(7-fluoro-5-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)-1-oxoisoindolin-2-yl)piperidine-2,6-dione;
3-(5-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)-1-oxoisoindolin-2-yl)piperidine-2,6-dione;
3-(4-fluoro-5-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)-1-oxoisoindolin-2-yl)piperidine-2,6-dione;
3-(6-fluoro-5-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)-1-oxoisoindolin-2-yl)piperidine-2,6-dione;
3-(7-fluoro-5-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)-1-oxoisoindolin-2-yl)piperidine-2,6-dione;
3-(5-((4-(4-(1-isopropyl-6-((2-(4-methoxypiperidin-1-yl)pyrimidin-4-yl)amino)-1H-pyrazolo[4,3-c]pyridin-3-yl)piperazin-1-yl)piperidin-1-yl)methyl)-1-oxoisoindolin-2-yl)piperidine-2,6-dione;
3-(4-fluoro-5-((4-(4-(1-isopropyl-6-((2-(4-methoxypiperidin-1-yl)pyrimidin-4-yl)amino)-1H-pyrazolo[4,3-c]pyridin-3-yl)piperazin-1-yl)piperidin-1-yl)methyl)-1-oxoisoindolin-2-yl)piperidine-2,6-dione;
3-(6-fluoro-5-((4-(4-(1-isopropyl-6-((2-(4-methoxypiperidin-1-yl)pyrimidin-4-yl)amino)-1H-pyrazolo[4,3-c]pyridin-3-yl)piperazin-1-yl)piperidin-1-yl)methyl)-1-oxoisoindolin-2-yl)piperidine-2,6-dione;
3-(7-fluoro-5-((4-(4-(1-isopropyl-6-((2-(4-methoxypiperidin-1-yl)pyrimidin-4-yl)amino)-1H-pyrazolo[4,3-c]pyridin-3-yl)piperazin-1-yl)piperidin-1-yl)methyl)-1-oxoisoindolin-2-yl)piperidine-2,6-dione;
N-(5-(((5-(tert-butyl)oxazol-2-yl)methyl)thio)thiazol-2-yl)-1-((2-(2,6-dioxopiperidin-3-yl)-1-oxoisoindolin-5-yl)methyl)piperidine-4-carboxamide;
N-(5-(((5-(tert-butyl)oxazol-2-yl)methyl)thio)thiazol-2-yl)-1-((2-(2,6-dioxopiperidin-3-yl)-4-fluoro-1-oxoisoindolin-5-yl)methyl)piperidine-4-carboxamide;
N-(5-(((5-(tert-butyl)oxazol-2-yl)methyl)thio)thiazol-2-yl)-1-((2-(2,6-dioxopiperidin-3-yl)-6-fluoro-1-oxoisoindolin-5-yl)methyl)piperidine-4-carboxamide;
N-(5-(((5-(tert-butyl)oxazol-2-yl)methyl)thio)thiazol-2-yl)-1-((2-(2,6-dioxopiperidin-3-yl)-7-fluoro-1-oxoisoindolin-5-yl)methyl)piperidine-4-carboxamide;
N-(5-(((5-(tert-butyl)oxazol-2-yl)methyl)thio)thiazol-2-yl)-1'-((2-(2,6-dioxopiperidin-3-yl)-1-oxoisoindolin-5-yl)methyl)-[1,4'-bipiperidine]-4-carboxamide;
N-(5-(((5-(tert-butyl)oxazol-2-yl)methyl)thio)thiazol-2-yl)-1'-((2-(2,6-dioxopiperidin-3-yl)-4-fluoro-1-oxoisoindolin-5-yl)methyl)-[1,4'-bipiperidine]-4-carboxamide;
N-(5-(((5-(tert-butyl)oxazol-2-yl)methyl)thio)thiazol-2-yl)-1'-((2-(2,6-dioxopiperidin-3-yl)-6-fluoro-1-oxoisoindolin-5-yl)methyl)-[1,4'-bipiperidine]-4-carboxamide;
N-(5-(((5-(tert-butyl)oxazol-2-yl)methyl)thio)thiazol-2-yl)-1'-((2-(2,6-dioxopiperidin-3-yl)-7-fluoro-1-oxoisoindolin-5-yl)methyl)-[1,4'-bipiperidine]-4-carboxamide;
N-(5-(((5-(tert-butyl)oxazol-2-yl)methyl)thio)thiazol-2-yl)-4-(1-((2-(2,6-dioxopiperidin-3-yl)-1-oxoisoindolin-5-yl)methyl)piperidin-4-yl)piperazine-1-carboxamide;
N-(5-(((5-(tert-butyl)oxazol-2-yl)methyl)thio)thiazol-2-yl)-4-(1-((2-(2,6-dioxopiperidin-3-yl)-4-fluoro-1-oxoisoindolin-5-yl)methyl)piperidin-4-yl)piperazine-1-carboxamide;
N-(5-(((5-(tert-butyl)oxazol-2-yl)methyl)thio)thiazol-2-yl)-4-(1-((2-(2,6-dioxopiperidin-3-yl)-6-fluoro-1-oxoisoindolin-5-yl)methyl)piperidin-4-yl)piperazine-1-carboxamide;
N-(5-(((5-(tert-butyl)oxazol-2-yl)methyl)thio)thiazol-2-yl)-4-(1-((2-(2,6-dioxopiperidin-3-yl)-7-fluoro-1-oxoisoindolin-5-yl)methyl)piperidin-4-yl)piperazine-1-carboxamide;
4-(2,6-dichlorobenzamido)-N-(1-((2-(2,6-dioxopiperidin-3-yl)-1-oxoisoindolin-5-yl)methyl)piperidin-4-yl)-1H-pyrazole-3-carboxamide;
4-(2,6-dichlorobenzamido)-N-(1-((2-(2,6-dioxopiperidin-3-yl)-4-fluoro-1-oxoisoindolin-5-yl)methyl)piperidin-4-yl)-1H-pyrazole-3-carboxamide;
4-(2,6-dichlorobenzamido)-N-(1-((2-(2,6-dioxopiperidin-3-yl)-6-fluoro-1-oxoisoindolin-5-yl)methyl)piperidin-4-yl)-1H-pyrazole-3-carboxamide;
4-(2,6-dichlorobenzamido)-N-(1-((2-(2,6-dioxopiperidin-3-yl)-7-fluoro-1-oxoisoindolin-5-yl)methyl)piperidin-4-yl)-1H-pyrazole-3-carboxamide;
N-(5-chloro-4-(5,5-dimethyl-5,6-dihydro-4H-pyrrolo[1,2-b]pyrazol-3-yl)pyridin-2-yl)-1-((2-(2,6-dioxopiperidin-3-yl)-1-oxoisoindolin-5-yl)methyl)piperidine-4-carboxamide;
N-(5-chloro-4-(5,5-dimethyl-5,6-dihydro-4H-pyrrolo[1,2-b]pyrazol-3-yl)pyridin-2-yl)-1-((2-(2,6-dioxopiperidin-3-yl)-4-fluoro-1-oxoisoindolin-5-yl)methyl)piperidine-4-carboxamide;
N-(5-chloro-4-(5,5-dimethyl-5,6-dihydro-4H-pyrrolo[1,2-b]pyrazol-3-yl)pyridin-2-yl)-1-((2-(2,6-dioxopiperidin-3-yl)-6-fluoro-1-oxoisoindolin-5-yl)methyl)piperidine-4-carboxamide;
N-(5-chloro-4-(5,5-dimethyl-5,6-dihydro-4H-pyrrolo[1,2-b]pyrazol-3-yl)pyridin-2-yl)-1-((2-(2,6-dioxopiperidin-3-yl)-7-fluoro-1-oxoisoindolin-5-yl)methyl)piperidine-4-carboxamide;
N-(5-cyclobutyl-1H-pyrazol-3-yl)-2-(4-((2-(2,6-dioxopiperidin-3-yl)-1-oxoisoindolin-5-yl)methoxy)phenyl)acetamide;
N-(5-cyclobutyl-1H-pyrazol-3-yl)-2-(4-((2-(2,6-dioxopiperidin-3-yl)-4-fluoro-1-oxoisoindolin-5-yl)methoxy)phenyl)acetamide;
N-(5-cyclobutyl-1H-pyrazol-3-yl)-2-(4-((2-(2,6-dioxopiperidin-3-yl)-6-fluoro-1-oxoisoindolin-5-yl)methoxy)phenyl)acetamide;
N-(5-cyclobutyl-1H-pyrazol-3-yl)-2-(4-((2-(2,6-dioxopiperidin-3-yl)-7-fluoro-1-oxoisoindolin-5-yl)methoxy)phenyl)acetamide;
3-(5-((4-((5-chloro-4-(5-(cyclopropylmethyl)-1-methyl-1H-pyrazol-4-yl)pyrimidin-2-yl)amino)piperidin-1-yl)methyl)-1-oxoisoindolin-2-yl)piperidine-2,6-dione;
3-(5-((4-((5-chloro-4-(5-(cyclopropylmethyl)-1-methyl-1H-pyrazol-4-yl)pyrimidin-2-yl)amino)piperidin-1-yl)methyl)-4-fluoro-1-oxoisoindolin-2-yl)piperidine-2,6-dione;
3-(5-((4-((5-chloro-4-(5-(cyclopropylmethyl)-1-methyl-1H-pyrazol-4-yl)pyrimidin-2-yl)amino)piperidin-1-yl)methyl)-6-fluoro-1-oxoisoindolin-2-yl)piperidine-2,6-dione;
3-(5-((4-((5-chloro-4-(5-(cyclopropylmethyl)-1-methyl-1H-pyrazol-4-yl)pyrimidin-2-yl)amino)piperidin-1-yl)methyl)-7-fluoro-1-oxoisoindolin-2-yl)piperidine-2,6-dione;
4-((2-(2,6-dioxopiperidin-3-yl)-1-oxoisoindolin-5-yl)methyl)-N-(4-(((5-(((S)-1-hydroxybutan-2-yl)amino)-3-isopropylpyrazolo[1,5-a]pyrimidin-7-yl)amino)methyl)phenyl)piperazine-1-carboxamide;
4-((2-(2,6-dioxopiperidin-3-yl)-4-fluoro-1-oxoisoindolin-5-yl)methyl)-N-(4-(((5-(((S)-1-hydroxybutan-2-yl)amino)-3-isopropylpyrazolo[1,5-a]pyrimidin-7-yl)amino)methyl)phenyl)piperazine-1 -carboxamide;
4-((2-(2,6-dioxopiperidin-3-yl)-6-fluoro-1-oxoisoindolin-5-yl)methyl)-N-(4-(((5-(((S)-1-hydroxybutan-2-yl)amino)-3-isopropylpyrazolo[1,5-a]pyrimidin-7-yl)amino)methyl)phenyl)piperazine-1 -carboxamide;
4-((2-(2,6-dioxopiperidin-3-yl)-7-fluoro-1-oxoisoindolin-5-yl)methyl)-N-(4-(((5-(((S)-1-hydroxybutan-2-yl)amino)-3-isopropylpyrazolo[1,5-a]pyrimidin-7-yl)amino)methyl)phenyl)piperazine-1 -carboxamide;
4-(((4-(5-chloro-2-((1-((2-(2,6-dioxopiperidin-3-yl)-1-oxoisoindolin-5-yl)methyl)piperidin-4-yl)amino)pyridin-4-yl)thiazol-2-yl)amino)methyl)tetrahydro-2H-pyran-4-carbonitrile;
4-(((4-(5-chloro-2-((1-((2-(2,6-dioxopiperidin-3-yl)-4-fluoro-1-oxoisoindolin-5-yl)methyl)piperidin-4-yl)amino)pyridin-4-yl)thiazol-2-yl)amino)methyl)tetrahydro-2H-pyran-4-carbonitrile;
4-(((4-(5-chloro-2-((1-((2-(2,6-dioxopiperidin-3-yl)-6-fluoro-1-oxoisoindolin-5-yl)methyl)piperidin-4-yl)amino)pyridin-4-yl)thiazol-2-yl)amino)methyl)tetrahydro-2H-pyran-4-carbonitrile;
4-(((4-(5-chloro-2-((1-((2-(2,6-dioxopiperidin-3-yl)-7-fluoro-1-oxoisoindolin-5-yl)methyl)piperidin-4-yl)amino)pyridin-4-yl)thiazol-2-yl)amino)methyl)tetrahydro-2H-pyran-4-carbonitrile;
4-(((5'-chloro-2'-((1-((2-(2,6-dioxopiperidin-3-yl)-1-oxoisoindolin-5-yl)methyl)piperidin-4-yl)amino)-[2,4'-bipyridin]-6-yl)amino)methyl)tetrahydro-2H-pyran-4-carbonitrile;
4-(((5'-chloro-2'-((1-((2-(2,6-dioxopiperidin-3-yl)-4-fluoro-1-oxoisoindolin-5-yl)methyl)piperidin-4-yl)amino)-[2,4'-bipyridin]-6-yl)amino)methyl)tetrahydro-2H-pyran-4-carbonitrile;
4-(((5'-chloro-2'-((1-((2-(2,6-dioxopiperidin-3-yl)-6-fluoro-1-oxoisoindolin-5-yl)methyl)piperidin-4-yl)amino)-[2,4'-bipyridin]-6-yl)amino)methyl)tetrahydro-2H-pyran-4-carbonitrile;
4-(((5'-chloro-2'-((1-((2-(2,6-dioxopiperidin-3-yl)-7-fluoro-1-oxoisoindolin-5-yl)methyl)piperidin-4-yl)amino)-[2,4'-bipyridin]-6-yl)amino)methyl)tetrahydro-2H-pyran-4-carbonitrile;
3-((4-(2-(((2-(2,6-dioxopiperidin-3-yl)-1-oxoisoindolin-5-yl)methyl)(methyl)amino)-4-methylthiazol-5-yl)-5-fluoropyrimidin-2-yl)amino)benzenesulfonamide;
3-((4-(2-(((2-(2,6-dioxopiperidin-3-yl)-4-fluoro-1-oxoisoindolin-5-yl)methyl)(methyl)amino)-4-methylthiazol-5-yl)-5-fluoropyrimidin-2-yl)amino)benzenesulfonamide;
3-((4-(2-(((2-(2,6-dioxopiperidin-3-yl)-6-fluoro-1-oxoisoindolin-5-yl)methyl)(methyl)amino)-4-methylthiazol-5-yl)-5-fluoropyrimidin-2-yl)amino)benzenesulfonamide;
3-((4-(2-(((2-(2,6-dioxopiperidin-3-yl)-7-fluoro-1-oxoisoindolin-5-yl)methyl)(methyl)amino)-4-methylthiazol-5-yl)-5-fluoropyrimidin-2-yl)amino)benzenesulfonamide;
N-(2-chloro-6-methylphenyl)-2-((6-(((2-(2,6-dioxopiperidin-3-yl)-1-oxoisoindolin-5-yl)methyl)amino)-2-methylpyrimidin-4-yl)amino)thiazole-5-carboxamide;
N-(2-chloro-6-methylphenyl)-2-((6-(((2-(2,6-dioxopiperidin-3-yl)-4-fluoro-1-oxoisoindolin-5-yl)methyl)amino)-2-methylpyrimidin-4-yl)amino)thiazole-5-carboxamide;
N-(2-chloro-6-methylphenyl)-2-((6-(((2-(2,6-dioxopiperidin-3-yl)-6-fluoro-1-oxoisoindolin-5-yl)methyl)amino)-2-methylpyrimidin-4-yl)amino)thiazole-5-carboxamide;
N-(2-chloro-6-methylphenyl)-2-((6-(((2-(2,6-dioxopiperidin-3-yl)-7-fluoro-1-oxoisoindolin-5-yl)methyl)amino)-2-methylpyrimidin-4-yl)amino)thiazole-5-carboxamide;
N-(2-chloro-6-methylphenyl)-2-((6-(4-((2-(2,6-dioxopiperidin-3-yl)-1-oxoisoindolin-5-yl)methyl)piperazin-1-yl)-2-methylpyrimidin-4-yl)amino)thiazole-5-carboxamide;
N-(2-chloro-6-methylphenyl)-2-((6-(4-((2-(2,6-dioxopiperidin-3-yl)-4-fluoro-1-oxoisoindolin-5-yl)methyl)piperazin-1-yl)-2-methylpyrimidin-4-yl)amino)thiazole-5-carboxamide;
N-(2-chloro-6-methylphenyl)-2-((6-(4-((2-(2, 6-dioxopiperidin-3-yl)-6-fluoro-1-oxoisoindolin-5-yl)methyl)piperazin-1-yl)-2-methylpyrimidin-4-yl)amino)thiazole-5-carboxamide;
N-(2-chloro-6-methylphenyl)-2-((6-(4-((2-(2,6-dioxopiperidin-3-yl)-7-fluoro-1-oxoisoindolin-5-yl)methyl)piperazin-1-yl)-2-methylpyrimidin-4-yl)amino)thiazole-5-carboxamide;
N-(2-chloro-6-methylphenyl)-2-((6-(4-(((2-(2,6-dioxopiperidin-3-yl)-1-oxoisoindolin-5-yl)methyl)(methyl)amino)piperidin-1-yl)-2-methylpyrimidin-4-yl)amino)thiazole-5-carboxamide;
N-(2-chloro-6-methylphenyl)-2-((6-(4-(((2-(2,6-dioxopiperidin-3-yl)-4-fluoro-1-oxoisoindolin-5-yl)methyl)(methyl)amino)piperidin-1-yl)-2-methylpyrimidin-4-yl)amino)thiazole-5-carboxamide;
N-(2-chloro-6-methylphenyl)-2-((6-(4-(((2-(2,6-dioxopiperidin-3-yl)-6-fluoro-1-oxoisoindolin-5-yl)methyl)(methyl)amino)piperidin-1-yl)-2-methylpyrimidin-4-yl)amino)thiazole-5-carboxamide;
N-(2-chloro-6-methylphenyl)-2-((6-(4-(((2-(2,6-dioxopiperidin-3-yl)-7-fluoro-1-oxoisoindolin-5-yl)methyl)(methyl)amino)piperidin-1-yl)-2-methylpyrimidin-4-yl)amino)thiazole-5-carboxamide;
N-(2-chloro-6-methylphenyl)-2-((6-(4-(4-((2-(2,6-dioxopiperidin-3-yl)-1-oxoisoindolin-5-yl)methyl)piperazin-1-yl)piperidin-1-yl)-2-methylpyrimidin-4-yl)amino)thiazole-5-carboxamide;
N-(2-chloro-6-methylphenyl)-2-((6-(4-(4-((2-(2,6-dioxopiperidin-3-yl)-4-fluoro-1-oxoisoindolin-5-yl)methyl)piperazin-1-yl)piperidin-1-yl)-2-methylpyrimidin-4-yl)amino)thiazole-5-carboxamide;
N-(2-chloro-6-methylphenyl)-2-((6-(4-(4-((2-(2,6-dioxopiperidin-3-yl)-6-fluoro-1-oxoisoindolin-5-yl)methyl)piperazin-1-yl)piperidin-1-yl)-2-methylpyrimidin-4-yl)amino)thiazole-5-carboxamide;
N-(2-chloro-6-methylphenyl)-2-((6-(4-(4-((2-(2,6-dioxopiperidin-3-yl)-7-fluoro-1-oxoisoindolin-5-yl)methyl)piperazin-1-yl)piperidin-1-yl)-2-methylpyrimidin-4-yl)amino)thiazole-5-carboxamide;
4-((4-((2-(2,6-dioxopiperidin-3-yl)-1-oxoisoindolin-5-yl)methyl)piperazin-1-yl)methyl)-N-(4-methyl-3-((4-(pyridin-3-yl)pyrimidin-2-yl)amino)phenyl)benzamide;
4-((4-((2-(2,6-dioxopiperidin-3-yl)-4-fluoro-1-oxoisoindolin-5-yl)methyl)piperazin-1-yl)methyl)-N-(4-methyl-3-((4-(pyridin-3-yl)pyrimidin-2-yl)amino)phenyl)benzamide;
4-((4-((2-(2,6-dioxopiperidin-3-yl)-6-fluoro-1-oxoisoindolin-5-yl)methyl)piperazin-1-yl)methyl)-N-(4-methyl-3-((4-(pyridin-3-yl)pyrimidin-2-yl)amino)phenyl)benzamide;
4-((4-((2-(2,6-dioxopiperidin-3-yl)-7-fluoro-1-oxoisoindolin-5-yl)methyl)piperazin-1-yl)methyl)-N-(4-methyl-3-((4-(pyridin-3-yl)pyrimidin-2-yl)amino)phenyl)benzamide;
4-((((2-(2,6-dioxopiperidin-3-yl)-1-oxoisoindolin-5-yl)methyl)amino)methyl)-N-(4-methyl-3-((4-(pyridin-3-yl)pyrimidin-2-yl)amino)phenyl)benzamide;
4-((((2-(2,6-dioxopiperidin-3-yl)-4-fluoro-1-oxoisoindolin-5-yl)methyl)amino)methyl)-N-(4-methyl-3-((4-(pyridin-3-yl)pyrimidin-2-yl)amino)phenyl)benzamide;
4-((((2-(2,6-dioxopiperidin-3-yl)-6-fluoro-1-oxoisoindolin-5-yl)methyl)amino)methyl)-N-(4-methyl-3-((4-(pyridin-3-yl)pyrimidin-2-yl)amino)phenyl)benzamide;
4-((((2-(2,6-dioxopiperidin-3-yl)-7-fluoro-1-oxoisoindolin-5-yl)methyl)amino)methyl)-N-(4-methyl-3-((4-(pyridin-3-yl)pyrimidin-2-yl)amino)phenyl)benzamide;
4-(4-((2-(2,6-dioxopiperidin-3-yl)-1-oxoisoindolin-5-yl)methyl)piperazin-1-yl)-N-(4-methyl-3-((4-(pyridin-3-yl)pyrimidin-2-yl)amino)phenyl)benzamide;
4-(4-((2-(2,6-dioxopiperidin-3-yl)-4-fluoro-1-oxoisoindolin-5-yl)methyl)piperazin-1-yl)-N-(4-methyl-3-((4-(pyridin-3-yl)pyrimidin-2-yl)amino)phenyl)benzamide;
4-(4-((2-(2,6-dioxopiperidin-3-yl)-6-fluoro-1-oxoisoindolin-5-yl)methyl)piperazin-1-yl)-N-(4-methyl-3-((4-(pyridin-3-yl)pyrimidin-2-yl)amino)phenyl)benzamide;
4-(4-((2-(2,6-dioxopiperidin-3-yl)-7-fluoro-1-oxoisoindolin-5-yl)methyl)piperazin-1-yl)-N-(4-methyl-3-((4-(pyridin-3-yl)pyrimidin-2-yl)amino)phenyl)benzamide;
4-(4-(((2-(2,6-dioxopiperidin-3-yl)-1-oxoisoindolin-5-yl)methyl)(methyl)amino)piperidin-1-yl)-N-(4-methyl-3-((4-(pyridin-3-yl)pyrimidin-2-yl)amino)phenyl)benzamide;
4-(4-(((2-(2,6-dioxopiperidin-3-yl)-4-fluoro-1-oxoisoindolin-5-yl)methyl)(methyl)amino)piperidin-1-yl)-N-(4-methyl-3-((4-(pyridin-3-yl)pyrimidin-2-yl)amino)phenyl)benzamide;
4-(4-(((2-(2,6-dioxopiperidin-3-yl)-6-fluoro-1-oxoisoindolin-5-yl)methyl)(methyl)amino)piperidin-1-yl)-N-(4-methyl-3-((4-(pyridin-3-yl)pyrimidin-2-yl)amino)phenyl)benzamide;
4-(4-(((2-(2,6-dioxopiperidin-3-yl)-7-fluoro-1-oxoisoindolin-5-yl)methyl)(methyl)amino)piperidin-1-yl)-N-(4-methyl-3-((4-(pyridin-3-yl)pyrimidin-2-yl)amino)phenyl)benzamide;
4-(4-(4-((2-(2,6-dioxopiperidin-3-yl)-1-oxoisoindolin-5-yl)methyl)piperazin-1-yl)piperidin-1-yl)-N-(4-methyl-3-((4-(pyridin-3-yl)pyrimidin-2-yl)amino)phenyl)benzamide;
4-(4-(4-((2-(2,6-dioxopiperidin-3-yl)-4-fluoro-1-oxoisoindolin-5-yl)methyl)piperazin-1-yl)piperidin-1-yl)-N-(4-methyl-3-((4-(pyridin-3-yl)pyrimidin-2-yl)amino)phenyl)benzamide;
4-(4-(4-((2-(2,6-dioxopiperidin-3-yl)-6-fluoro-1-oxoisoindolin-5-yl)methyl)piperazin-1-yl)piperidin-1-yl)-N-(4-methyl-3-((4-(pyridin-3-yl)pyrimidin-2-yl)amino)phenyl)benzamide;
4-(4-(4-((2-(2,6-dioxopiperidin-3-yl)-7-fluoro-1-oxoisoindolin-5-yl)methyl)piperazin-1-yl)piperidin-1-yl)-N-(4-methyl-3-((4-(pyridin-3-yl)pyrimidin-2-yl)amino)phenyl)benzamide;
4-((2,4-dichloro-5-methoxyphenyl)amino)-7-(3-(4-((2-(2,6-dioxopiperidin-3-yl)-1-oxoisoindolin-5-yl)methyl)piperazin-1-yl)propoxy)-6-methoxyquinoline-3-carbonitrile;
4-((2,4-dichloro-5-methoxyphenyl)amino)-7-(3-(4-((2-(2,6-dioxopiperidin-3-yl)-4-fluoro-1-oxoisoindolin-5-yl)methyl)piperazin-1-yl)propoxy)-6-methoxyquinoline-3-carbonitrile;
4-((2,4-dichloro-5-methoxyphenyl)amino)-7-(3-(4-((2-(2,6-dioxopiperidin-3-yl)-6-fluoro-1-oxoisoindolin-5-yl)methyl)piperazin-1-yl)propoxy)-6-methoxyquinoline-3-carbonitrile;
4-((2,4-dichloro-5-methoxyphenyl)amino)-7-(3-(4-((2-(2,6-dioxopiperidin-3-yl)-7-fluoro-1-oxoisoindolin-5-yl)methyl)piperazin-1-yl)propoxy)-6-methoxyquinoline-3-carbonitrile;
4-((2,4-dichloro-5-methoxyphenyl)amino)-7-((2-(2,6-dioxopiperidin-3-yl)-1-oxoisoindolin-5-yl)methoxy)-6-methoxyquinoline-3-carbonitrile;
4-((2,4-dichloro-5-methoxyphenyl)amino)-7-((2-(2,6-dioxopiperidin-3-yl)-4-fluoro-1-oxoisoindolin-5-yl)methoxy)-6-methoxyquinoline-3-carbonitrile;
4-((2,4-dichloro-5-methoxyphenyl)amino)-7-((2-(2,6-dioxopiperidin-3-yl)-6-fluoro-1-oxoisoindolin-5-yl)methoxy)-6-methoxyquinoline-3-carbonitrile;
4-((2,4-dichloro-5-methoxyphenyl)amino)-7-((2-(2,6-dioxopiperidin-3-yl)-7-fluoro-1-oxoisoindolin-5-yl)methoxy)-6-methoxyquinoline-3-carbonitrile;
N-(4-((4-((2-(2,6-dioxopiperidin-3-yl)-1-oxoisoindolin-5-yl)methyl)piperazin-1-yl)methyl)-3-(trifluoromethyl)phenyl)-3-(imidazo[1,2-b]pyridazin-3-ylethynyl)-4-methylbenzamide;
N-(4-((4-((2-(2,6-dioxopiperidin-3-yl)-4-fluoro-1-oxoisoindolin-5-yl)methyl)piperazin-1-yl)methyl)-3-(trifluoromethyl)phenyl)-3-(imidazo[1,2-b]pyridazin-3-ylethynyl)-4-methylbenzamide;
N-(4-((4-((2-(2,6-dioxopiperidin-3-yl)-6-fluoro-1-oxoisoindolin-5-yl)methyl)piperazin-1-yl)methyl)-3-(trifluoromethyl)phenyl)-3-(imidazo[1,2-b]pyridazin-3-ylethynyl)-4-methylbenzamide;
N-(4-((4-((2-(2,6-dioxopiperidin-3-yl)-7-fluoro-1-oxoisoindolin-5-yl)methyl)piperazin-1-yl)methyl)-3-(trifluoromethyl)phenyl)-3-(imidazo[1,2-b]pyridazin-3-ylethynyl)-4-methylbenzamide;
N-(4-(4-((2-(2,6-dioxopiperidin-3-yl)-1-oxoisoindolin-5-yl)methyl)piperazin-1-yl)-3-(trifluoromethyl)phenyl)-3-(imidazo[1,2-b]pyridazin-3-ylethynyl)-4-methylbenzamide;
N-(4-(4-((2-(2,6-dioxopiperidin-3-yl)-4-fluoro-1-oxoisoindolin-5-yl)methyl)piperazin-1-yl)-3-(trifluoromethyl)phenyl)-3-(imidazo[1,2-b]pyridazin-3-ylethynyl)-4-methylbenzamide;
N-(4-(4-((2-(2,6-dioxopiperidin-3-yl)-6-fluoro-1-oxoisoindolin-5-yl)methyl)piperazin-1-yl)-3-(trifluoromethyl)phenyl)-3-(imidazo[1,2-b]pyridazin-3-ylethynyl)-4-methylbenzamide;
N-(4-(4-((2-(2,6-dioxopiperidin-3-yl)-7-fluoro-1-oxoisoindolin-5-yl)methyl)piperazin-1-yl)-3-(trifluoromethyl)phenyl)-3-(imidazo[1,2-b]pyridazin-3-ylethynyl)-4-methylbenzamide;
N-(4-(4-(1-((2-(2,6-dioxopiperidin-3-yl)-1-oxoisoindolin-5-yl)methyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(1-((2-(2,6-dioxopiperidin-3-yl)-4-fluoro-1-oxoisoindolin-5-yl)methyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(1-((2-(2,6-dioxopiperidin-3-yl)-6-fluoro-1-oxoisoindolin-5-yl)methyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(1-((2-(2,6-dioxopiperidin-3-yl)-7-fluoro-1-oxoisoindolin-5-yl)methyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(((2-(2,6-dioxopiperidin-3-yl)-1-oxoisoindolin-5-yl)methyl)(methyl)amino)piperidin-1-yl)-3-(trifluoromethyl)phenyl)-3-(imidazo[1,2-b]pyridazin-3-ylethynyl)-4-methylbenzamide;
N-(4-(4-(((2-(2,6-dioxopiperidin-3-yl)-4-fluoro-1-oxoisoindolin-5-yl)methyl)(methyl)amino)piperidin-1-yl)-3-(trifluoromethyl)phenyl)-3-(imidazo[1,2-b]pyridazin-3-ylethynyl)-4-methylbenzamide;
N-(4-(4-(((2-(2,6-dioxopiperidin-3-yl)-6-fluoro-1-oxoisoindolin-5-yl)methyl)(methyl)amino)piperidin-1-yl)-3-(trifluoromethyl)phenyl)-3-(imidazo[1,2-b]pyridazin-3-ylethynyl)-4-methylbenzamide;
N-(4-(4-(((2-(2,6-dioxopiperidin-3-yl)-7-fluoro-1-oxoisoindolin-5-yl)methyl)(methyl)amino)piperidin-1-yl)-3-(trifluoromethyl)phenyl)-3-(imidazo[1,2-b]pyridazin-3-ylethynyl)-4-methylbenzamide;
2-(4-((3 S)-1-((2-(2,6-dioxopiperidin-3-yl)-1-oxoisoindolin-5-yl)methyl)piperidin-3-yl)phenyl)-2H-indazole-7-carboxamide;
2-(4-((3S)-1-((2-(2,6-dioxopiperidin-3-yl)-4-fluoro-1-oxoisoindolin-5-yl)methyl)piperidin-3-yl)phenyl)-2H-indazole-7-carboxamide;
2-(4-((3 S)-1-((2-(2,6-dioxopiperidin-3-yl)-6-fluoro-1-oxoisoindolin-5-yl)methyl)piperidin-3-yl)phenyl)-2H-indazole-7-carboxamide;
2-(4-((3 S)-1-((2-(2,6-dioxopiperidin-3-yl)-7-fluoro-1-oxoisoindolin-5-yl)methyl)piperidin-3-yl)phenyl)-2H-indazole-7-carboxamide;
2-(4-(1-((2-(2,6-dioxopiperidin-3-yl)-1-oxoisoindolin-5-yl)methyl)piperidin-4-yl)phenyl)-2H-indazole-7-carboxamide;
2-(4-(1-((2-(2,6-dioxopiperidin-3-yl)-4-fluoro-1-oxoisoindolin-5-yl)methyl)piperidin-4-yl)phenyl)-2H-indazole-7-carboxamide;
2-(4-(1-((2-(2,6-dioxopiperidin-3-yl)-6-fluoro-1-oxoisoindolin-5-yl)methyl)piperidin-4-yl)phenyl)-2H-indazole-7-carboxamide;
2-(4-(1-((2-(2,6-dioxopiperidin-3-yl)-7-fluoro-1-oxoisoindolin-5-yl)methyl)piperidin-4-yl)phenyl)-2H-indazole-7-carboxamide;
2-(4-(4-((2-(2,6-dioxopiperidin-3-yl)-1-oxoisoindolin-5-yl)methyl)piperazin-1-yl)phenyl)-2H-indazole-7-carboxamide;
2-(4-(4-((2-(2,6-dioxopiperidin-3-yl)-4-fluoro-1-oxoisoindolin-5-yl)methyl)piperazin-1-yl)phenyl)-2H-indazole-7-carboxamide;
2-(4-(4-((2-(2,6-dioxopiperidin-3-yl)-6-fluoro-1-oxoisoindolin-5-yl)methyl)piperazin-1-yl)phenyl)-2H-indazole-7-carboxamide;
2-(4-(4-((2-(2,6-dioxopiperidin-3-yl)-7-fluoro-1-oxoisoindolin-5-yl)methyl)piperazin-1-yl)phenyl)-2H-indazole-7-carboxamide;
3-(5-((4-(2-fluoro-5-((4-oxo-3,4-dihydrophthalazin-1-yl)methyl)benzoyl)piperazin-1-yl)methyl)-1-oxoisoindolin-2-yl)piperidine-2,6-dione;
3-(4-fluoro-5-((4-(2-fluoro-5-((4-oxo-3,4-dihydrophthalazin-1-yl)methyl)benzoyl)piperazin-1-yl)methyl)-1-oxoisoindolin-2-yl)piperidine-2,6-dione;
3-(6-fluoro-5-((4-(2-fluoro-5-((4-oxo-3,4-dihydrophthalazin-1-yl)methyl)benzoyl)piperazin-1-yl)methyl)-1-oxoisoindolin-2-yl)piperidine-2,6-dione;
3-(7-fluoro-5-((4-(2-fluoro-5-((4-oxo-3,4-dihydrophthalazin-1-yl)methyl)benzoyl)piperazin-1-yl)methyl)-1-oxoisoindolin-2-yl)piperidine-2,6-dione;
3-(5-(((4-(8-fluoro-1-oxo-2,3,4,6-tetrahydro-1H-azepino[5,4,3-cd]indol-5-yl)benzyl)(methyl)amino)methyl)-1-oxoisoindolin-2-yl)piperidine-2,6-dione;
3-(4-fluoro-5-(((4-(8-fluoro-1-oxo-2,3,4,6-tetrahydro-1H-azepino[5,4,3-cd]indol-5-yl)benzyl)(methyl)amino)methyl)-1-oxoisoindolin-2-yl)piperidine-2,6-dione;
3-(6-fluoro-5-(((4-(8-fluoro-1-oxo-2,3,4,6-tetrahydro-1H-azepino[5,4,3-cd]indol-5-yl)benzyl)(methyl)amino)methyl)-1-oxoisoindolin-2-yl)piperidine-2,6-dione;
3-(7-fluoro-5-(((4-(8-fluoro-1-oxo-2,3,4,6-tetrahydro-1H-azepino[5,4,3-cd]indol-5-yl)benzyl)(methyl)amino)methyl)-1-oxoisoindolin-2-yl)piperidine-2,6-dione;
3-(5-((4-(2-fluoro-5-((8S,9R)-5-fluoro-9-(1-methyl-1H-1,2,4-triazol-5-yl)-3-oxo-2,7,8,9-tetrahydro-3H-pyrido[4,3,2-de]phthalazin-8-yl)phenyl)piperazin-1-yl)methyl)-1-oxoisoindolin-2-yl)piperidine-2,6-dione;
3-(4-fluoro-5-((4-(2-fluoro-5-((8S,9R)-5-fluoro-9-(1-methyl-1H-1,2,4-triazol-5-yl)-3-oxo-2,7,8,9-tetrahydro-3H-pyrido[4,3,2-de]phthalazin-8-yl)phenyl)piperazin-1-yl)methyl)-1-oxoisoindolin-2-yl)piperidine-2,6-dione;
3-(6-fluoro-5-((4-(2-fluoro-5-((8S,9R)-5-fluoro-9-(1-methyl-1H-1,2,4-triazol-5-yl)-3-oxo-2,7,8,9-tetrahydro-3H-pyrido[4,3,2-de]phthalazin-8-yl)phenyl)piperazin-1-yl)methyl)-1-oxoisoindolin-2-yl)piperidine-2,6-dione;
3-(7-fluoro-5-((4-(2-fluoro-5-((8S,9R)-5-fluoro-9-(1-methyl-1H-1,2,4-triazol-5-yl)-3-oxo-2,7,8,9-tetrahydro-3H-pyrido[4,3,2-de]phthalazin-8-yl)phenyl)piperazin-1-yl)methyl)-1-oxoisoindolin-2-yl)piperidine-2,6-dione;
3-(5-((4-((2-fluoro-5-((8S,9R)-5-fluoro-9-(1-methyl-1H-1,2,4-triazol-5-yl)-3-oxo-2,7,8,9-tetrahydro-3H-pyrido[4,3,2-de]phthalazin-8-yl)phenyl)ethynyl)piperidin-1-yl)methyl)-1-oxoisoindolin-2-yl)piperidine-2,6-dione;
3-(4-fluoro-5-((4-((2-fluoro-5-((8S,9R)-5-fluoro-9-(1-methyl-1H-1,2,4-triazol-5-yl)-3-oxo-2,7,8,9-tetrahydro-3H-pyrido[4,3,2-de]phthalazin-8-yl)phenyl)ethynyl)piperidin-1-yl)methyl)-1-oxoisoindolin-2-yl)piperidine-2,6-dione;
3-(6-fluoro-5-((4-((2-fluoro-5-((8S,9R)-5-fluoro-9-(1-methyl-1H-1,2,4-triazol-5-yl)-3-oxo-2,7,8,9-tetrahydro-3H-pyrido[4,3,2-de]phthalazin-8-yl)phenyl)ethynyl)piperidin-1-yl)methyl)-1-oxoisoindolin-2-yl)piperidine-2,6-dione;
3-(7-fluoro-5-((4-((2-fluoro-5-((8S,9R)-5-fluoro-9-(1-methyl-1H-1,2,4-triazol-5-yl)-3-oxo-2,7,8,9-tetrahydro-3H-pyrido[4,3,2-de]phthalazin-8-yl)phenyl)ethynyl)piperidin-1-yl)methyl)-1-oxoisoindolin-2-yl)piperidine-2,6-dione;
7-cyclopentyl-2-((5-(4-((2-(2,6-dioxopiperidin-3-yl)-1-oxoisoindolin-5-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-((2-(2,6-dioxopiperidin-3-yl)-4-fluoro-1-oxoisoindolin-5-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-((2-(2,6-dioxopiperidin-3-yl)-6-fluoro-1-oxoisoindolin-5-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-((2-(2,6-dioxopiperidin-3-yl)-7-fluoro-1-oxoisoindolin-5-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-(((2-(2,6-dioxopiperidin-3-yl)-1-oxoisoindolin-5-yl)methyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-(((2-(2,6-dioxopiperidin-3-yl)-4-fluoro-1-oxoisoindolin-5-yl)methyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-(((2-(2,6-dioxopiperidin-3-yl)-6-fluoro-1-oxoisoindolin-5-yl)methyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-(((2-(2,6-dioxopiperidin-3-yl)-7-fluoro-1-oxoisoindolin-5-yl)methyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-(4-((2-(2,6-dioxopiperidin-3-yl)-1-oxoisoindolin-5-yl)methyl)piperazin-1-yl)piperidin-1-yl)pyridin-2-yl)amino)-N,N-dimethyl-7H-pyrrolo[2,3-d]pyrimidine-6-carboxamide;
7-cyclopentyl-2-((5-(4-(4-((2-(2,6-dioxopiperidin-3-yl)-4-fluoro-1-oxoisoindolin-5-yl)methyl)piperazin-1-yl)piperidin-1-yl)pyridin-2-yl)amino)- N,N-dimethyl-7H-pyrrolo[2,3-d]pyrimidine-6-carboxamide;
7-cyclopentyl-2-((5-(4-(4-((2-(2,6-dioxopiperidin-3-yl)-6-fluoro-1-oxoisoindolin-5-yl)methyl)piperazin-1-yl)piperidin-1-yl)pyridin-2-yl)amino)- N,N-dimethyl-7H-pyrrolo[2,3-d]pyrimidine-6-carboxamide;
7-cyclopentyl-2-((5-(4-(4-((2-(2,6-dioxopiperidin-3-yl)-7-fluoro-1-oxoisoindolin-5-yl)methyl)piperazin-1-yl)piperidin-1-yl)pyridin-2-yl)amino)- N,N-dimethyl-7H-pyrrolo[2,3-d]pyrimidine-6-carboxamide;
7-cyclopentyl-2-((5-(4-(1-((2-(2,6-dioxopiperidin-3-yl)-1-oxoisoindolin-5-yl)methyl)piperidin-4-yl)piperazin-1-yl)pyridin-2-yl)amino)-N,N-dimethyl-7H-pyrrolo[2,3-d]pyrimidine-6-carboxamide;
7-cyclopentyl-2-((5-(4-(1-((2-(2,6-dioxopiperidin-3-yl)-4-fluoro-1-oxoisoindolin-5-yl)methyl)piperidin-4-yl)piperazin-1-yl)pyridin-2-yl)amino)-N,N-dimethyl-7H-pyrrolo[2,3-d]pyrimidine-6-carboxamide;
7-cyclopentyl-2-((5-(4-(1-((2-(2,6-dioxopiperidin-3-yl)-6-fluoro-1-oxoisoindolin-5-yl)methyl)piperidin-4-yl)piperazin-1-yl)pyridin-2-yl)amino)-N,N-dimethyl-7H-pyrrolo[2,3-d]pyrimidine-6-carboxamide;
7-cyclopentyl-2-((5-(4-(1-((2-(2,6-dioxopiperidin-3-yl)-7-fluoro-1-oxoisoindolin-5-yl)methyl)piperidin-4-yl)piperazin-1-yl)pyridin-2-yl)amino)-N,N-dimethyl-7H-pyrrolo[2,3-d]pyrimidine-6-carboxamide;
3-(5-((4-((6-((5-fluoro-4-(4-fluoro-1-isopropyl-2-methyl-1H-benzo[d]imidazol-6-yl)pyrimidin-2-yl)amino)pyri din-3 -yl)methyl)piperazin-l-yl)methyl)-l-oxoisoindolin-2-yl)piperidine-2,6-dione;
3-(4-fluoro-5-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-1-oxoisoindolin-2-yl)piperidine-2,6-dione;
3-(6-fluoro-5-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-1-oxoisoindolin-2-yl)piperidine-2,6-dione;
3-(7-fluoro-5-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-1-oxoisoindolin-2-yl)piperidine-2,6-dione;
3-(5-((4-(difluoro(6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-1-oxoisoindolin-2-yl)piperidine-2,6-dione;
3-(5-((4-(difluoro(6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-4-fluoro-1-oxoisoindolin-2-yl)piperidine-2,6-dione;
3-(5-((4-(difluoro(6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-6-fluoro-1-oxoisoindolin-2-yl)piperidine-2,6-dione;
3-(5-((4-(difluoro(6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-7-fluoro-1-oxoisoindolin-2-yl)piperidine-2,6-dione;
3-(5-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)-1-oxoisoindolin-2-yl)piperidine-2,6-dione;
3-(4-fluoro-5-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)-1-oxoisoindolin-2-yl)piperidine-2,6-dione;
3-(6-fluoro-5-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)-1-oxoisoindolin-2-yl)piperidine-2,6-dione;
3-(7-fluoro-5-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)-1-oxoisoindolin-2-yl)piperidine-2,6-dione;
3-(5-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)-1-oxoisoindolin-2-yl)piperidine-2,6-dione;
3-(4-fluoro-5-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)-1-oxoisoindolin-2-yl)piperidine-2,6-dione;
3-(6-fluoro-5-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)-1-oxoisoindolin-2-yl)piperidine-2,6-dione;
3-(7-fluoro-5-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)-1-oxoisoindolin-2-yl)piperidine-2,6-dione;
3-(5-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)-1-oxoisoindolin-2-yl)piperidine-2,6-dione;
3-(4-fluoro-5-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)-1-oxoisoindolin-2-yl)piperidine-2,6-dione;
3-(6-fluoro-5-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)-1-oxoisoindolin-2-yl)piperidine-2,6-dione;
3-(7-fluoro-5-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)-1-oxoisoindolin-2-yl)piperidine-2,6-dione;
3-(5-((4-(4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)piperidin-1-yl)methyl)-1-oxoisoindolin-2-yl)piperidine-2,6-dione;
3-(4-fluoro-5-((4-(4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)piperidin-1-yl)methyl)-1-oxoisoindolin-2-yl)piperidine-2,6-dione;
3-(6-fluoro-5-((4-(4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)piperidin-1-yl)methyl)-1-oxoisoindolin-2-yl)piperidine-2,6-dione;
3-(7-fluoro-5-((4-(4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)piperidin-1-yl)methyl)-1-oxoisoindolin-2-yl)piperidine-2,6-dione;
3-(5-((4-(difluoro(6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-1-oxoisoindolin-2-yl)piperidine-2,6-dione;
3-(5-((4-(difluoro(6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-4-fluoro-1-oxoisoindolin-2-yl)piperidine-2,6-dione;
3-(5-((4-(difluoro(6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-6-fluoro-1-oxoisoindolin-2-yl)piperidine-2,6-dione;
3-(5-((4-(difluoro(6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-7-fluoro-1-oxoisoindolin-2-yl)piperidine-2,6-dione;
3-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-1-oxoisoindolin-2-yl)piperidine-2,6-dione;
3-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-4-fluoro-1-oxoisoindolin-2-yl)piperidine-2,6-dione;
3-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-6-fluoro-1-oxoisoindolin-2-yl)piperidine-2,6-dione;
3-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-7-fluoro-1-oxoisoindolin-2-yl)piperidine-2,6-dione;
3-(5-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)-1-oxoisoindolin-2-yl)piperidine-2,6-dione;
3-(5-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)-4-fluoro-1-oxoisoindolin-2-yl)piperidine-2,6-dione;
3-(5-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)-6-fluoro-1-oxoisoindolin-2-yl)piperidine-2,6-dione;
3-(5-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)-7-fluoro-1-oxoisoindolin-2-yl)piperidine-2,6-dione;
3-(5-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)-1-oxoisoindolin-2-yl)piperidine-2,6-dione;
3-(5-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)-4-fluoro-1-oxoisoindolin-2-yl)piperidine-2,6-dione;
3-(5-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)-6-fluoro-1-oxoisoindolin-2-yl)piperidine-2,6-dione;
3-(5-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)-7-fluoro-1-oxoisoindolin-2-yl)piperidine-2,6-dione;
3-(5-((4-(4-(6-((6-acetyl-8-cyclopentyl-5-methyl-7-oxo-7,8-dihydropyrido[2,3-d]pyrimidin-2-yl)amino)pyridin-3-yl)piperazin-1-yl)piperidin-1-yl)methyl)-1-oxoisoindolin-2-yl)piperidine-2,6-dione;
3-(5-((4-(4-(6-((6-acetyl-8-cyclopentyl-5-methyl-7-oxo-7,8-dihydropyrido[2,3-d]pyrimidin-2-yl)amino)pyridin-3-yl)piperazin-1-yl)piperidin-1-yl)methyl)-4-fluoro-1-oxoisoindolin-2-yl)piperidine-2,6-dione;
3-(5-((4-(4-(6-((6-acetyl-8-cyclopentyl-5-methyl-7-oxo-7,8-dihydropyrido[2,3-d]pyrimidin-2-yl)amino)pyridin-3-yl)piperazin-1-yl)piperidin-1-yl)methyl)-6-fluoro-1-oxoisoindolin-2-yl)piperidine-2,6-dione;
3-(5-((4-(4-(6-((6-acetyl-8-cyclopentyl-5-methyl-7-oxo-7,8-dihydropyrido[2,3-d]pyrimidin-2-yl)amino)pyridin-3-yl)piperazin-1-yl)piperidin-1-yl)methyl)-7-fluoro-1-oxoisoindolin-2-yl)piperidine-2,6-dione;
3-((4-(4-(4-((2-(2,6-dioxopiperidin-3-yl)-1-oxoisoindolin-5-yl)methyl)piperazin-1-yl)piperidin-1-yl)-3-methoxyphenyl)amino)-6-ethyl-5-((tetrahydro-2H-pyran-4-yl)amino)pyrazine-2-carboxamide;
3-((4-(4-(4-((2-(2,6-dioxopiperidin-3-yl)-4-fluoro-1-oxoisoindolin-5-yl)methyl)piperazin-1-yl)piperidin-1-yl)-3-methoxyphenyl)amino)-6-ethyl-5-((tetrahydro-2H-pyran-4-yl)amino)pyrazine-2-carboxamide;
3-((4-(4-(4-((2-(2,6-dioxopiperidin-3-yl)-6-fluoro-1-oxoisoindolin-5-yl)methyl)piperazin-1-yl)piperidin-1-yl)-3-methoxyphenyl)amino)-6-ethyl-5-((tetrahydro-2H-pyran-4-yl)amino)pyrazine-2-carboxamide;
3-((4-(4-(4-((2-(2,6-dioxopiperidin-3-yl)-7-fluoro-1-oxoisoindolin-5-yl)methyl)piperazin-1-yl)piperidin-1-yl)-3-methoxyphenyl)amino)-6-ethyl-5-((tetrahydro-2H-pyran-4-yl)amino)pyrazine-2-carboxamide;
3-(5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-l-oxoisoindolin-2-yl)piperidine-2,6-dione,'
3-(5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-4-fluoro-l-oxoisoindolin-2-yl)piperidine-2,6-dione;
3-(5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-6-fluoro-l-oxoisoindolin-2-yl)piperidine-2,6-dione;
3-(5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-7-fluoro-l-oxoisoindolin-2-yl)piperidine-2,6-dione;
3-(5-(((1-(4-((5-chloro-4-((2-(dimethylphosphoryl)phenyl)amino)pyrimidin-2-yl)amino)-3-methoxyphenyl)piperidin-4-yl)amino)methyl)-1-oxoisoindolin-2-yl)piperidine-2,6-dione;
3-(5-(((1-(4-((5-chloro-4-((2-(dimethylphosphoryl)phenyl)amino)pyrimidin-2-yl)amino)-3-methoxyphenyl)piperidin-4-yl)amino)methyl)-4-fluoro-l-oxoisoindolin-2-yl)piperidine-2,6-dione,'
3-(5-(((1-(4-((5-chloro-4-((2-(dimethylphosphoryl)phenyl)amino)pyrimidin-2-yl)amino)-3-methoxyphenyl)piperidin-4-yl)amino)methyl)-6-fluoro-l-oxoisoindolin-2-yl)piperidine-2,6-dione,'
3-(5-(((1-(4-((5-chloro-4-((2-(dimethylphosphoryl)phenyl)amino)pyrimidin-2-yl)amino)-3-methoxyphenyl)piperidin-4-yl)amino)methyl)-7-fluoro-l-oxoisoindolin-2-yl)piperidine-2,6-dione,'
3-(5-((4-(4-((5-chloro-4-((2-(dimethylphosphoryl)phenyl)amino)pyrimidin-2-yl)amino)-3-methoxyphenyl)piperazin-l-yl)methyl)-l-oxoisoindolin-2-yl)piperidine-2,6-dione,'
3-(5-((4-(4-((5-chloro-4-((2-(dimethylphosphoryl)phenyl)amino)pyrimidin-2-yl)amino)-3-methoxyphenyl)piperazin-1-yl)methyl)-4-fluoro-1-oxoisoindolin-2-yl)piperidine-2,6-dione ;
3-(5-((4-(4-((5-chloro-4-((2-(dimethylphosphoryl)phenyl)amino)pyrimidin-2-yl)amino)-3-methoxyphenyl)piperazin-l-yl)methyl)-6-fluoro-l-oxoisoindolin-2-yl)piperidine-2,6-dione,'
3-(5-((4-(4-((5-chloro-4-((2-(dimethylphosphoryl)phenyl)amino)pyrimidin-2-yl)amino)-3-methoxyphenyl)piperazin-1-yl)methyl)-7-fluoro-1-oxoisoindolin-2-yl)piperidine-2,6-dione;
3-(5-((4-(4-(4-((5-chloro-4-((2-(dimethylphosphoryl)phenyl)amino)pyrimidin-2-yl)amino)-3-methoxyphenyl)piperazin-1-yl)piperidin-1-yl)methyl)-1-oxoisoindolin-2-yl)piperidine-2,6-dione;
3-(5-((4-(4-(4-((5-chloro-4-((2-(dimethylphosphoryl)phenyl)amino)pyrimidin-2-yl)amino)-3-methoxyphenyl)piperazin-1 -yl)piperidin-1 -yl)methyl)-4-fluoro-1 -oxoisoindolin-2-yl)piperidine-2,6-dione;
3-(5-((4-(4-(4-((5-chloro-4-((2-(dimethylphosphoryl)phenyl)amino)pyrimidin-2-yl)amino)-3-methoxyphenyl)piperazin-1 -yl)piperidin-1 -yl)methyl)-6-fluoro-1 -oxoisoindolin-2-yl)piperidine-2,6-dione;
3-(5-((4-(4-(4-((5-chloro-4-((2-(dimethylphosphoryl)phenyl)amino)pyrimidin-2-yl)amino)-3-methoxyphenyl)piperazin-1-yl)piperidin-1-yl)methyl)-7-fluoro-1-oxoisoindolin-2-yl)piperidine-2,6-dione;
3-(5-((4-(4-(6-amino-5-((R)-1-(2,6-dichloro-3-fluorophenyl)ethoxy)pyridin-3-yl)-1H-pyrazol-1-yl)piperidin-1-yl)methyl)-1-oxoisoindolin-2-yl)piperidine-2,6-dione ;
3-(5-((4-(4-(6-amino-5-((R)-1-(2,6-dichloro-3-fluorophenyl)ethoxy)pyridin-3-yl)-1H-pyrazol-1-yl)piperidin-1-yl)methyl)-4-fluoro-1-oxoisoindolin-2-yl)piperidine-2,6-dione;
3-(5-((4-(4-(6-amino-5-((R)-1-(2,6-dichloro-3-fluorophenyl)ethoxy)pyridin-3-yl)-1H-pyrazol-1-yl)piperidin-1-yl)methyl)-6-fluoro-1-oxoisoindolin-2-yl)piperidine-2,6-dione ;
3-(5-((4-(4-(6-amino-5-((R)-1-(2,6-dichloro-3-fluorophenyl)ethoxy)pyridin-3-yl)-1H-pyrazol-1-yl)piperidin-1-yl)methyl)-7-fluoro-1-oxoisoindolin-2-yl)piperidine-2,6-dione ;
3-(5-((4-(4-((5-chloro-4-((2-(isopropylsulfonyl)phenyl)amino)pyrimidin-2-yl)amino)-5-isopropoxy-2-methylphenyl)piperidin-1-yl)methyl)-1-oxoisoindolin-2-yl)piperidine-2,6-dione ;
3-(5-((4-(4-((5-chloro-4-((2-(isopropylsulfonyl)phenyl)amino)pyrimidin-2-yl)amino)-5-isopropoxy-2-methylphenyl)piperidin-1-yl)methyl)-4-fluoro-1-oxoisoindolin-2-yl)piperidine-2,6-dione;
3-(5-((4-(4-((5-chloro-4-((2-(isopropylsulfonyl)phenyl)amino)pyrimidin-2-yl)amino)-5-isopropoxy-2-methylphenyl)piperidin-1-yl)methyl)-6-fluoro-1-oxoisoindolin-2-yl)piperidine-2,6-dione;
3-(5-((4-(4-((5-chloro-4-((2-(isopropylsulfonyl)phenyl)amino)pyrimidin-2-yl)amino)-5-isopropoxy-2-methylphenyl)piperidin-1-yl)methyl)-7-fluoro-1-oxoisoindolin-2-yl)piperidine-2,6-dione;
3-(5-((4-(4-((5-chloro-4-((2-(isopropylsulfonyl)phenyl)amino)pyrimidin-2-yl)amino)-5-isopropoxy-2-methylphenyl)piperazin-1-yl)methyl)-1-oxoisoindolin-2-yl)piperidine-2,6-dione ;
3-(5-((4-(4-((5-chloro-4-((2-(isopropylsulfonyl)phenyl)amino)pyrimidin-2-yl)amino)-5-isopropoxy-2-methylphenyl)piperazin-l-yl)methyl)-4-fluoro-l-oxoisoindolin-2-yl)piperidine-2,6-dione;
3-(5-((4-(4-((5-chloro-4-((2-(isopropylsulfonyl)phenyl)amino)pyrimidin-2-yl)amino)-5-isopropoxy-2-methylphenyl)piperazin-l-yl)methyl)-6-fluoro-l-oxoisoindolin-2-yl)piperidine-2,6-dione;
3-(5-((4-(4-((5-chloro-4-((2-(isopropylsulfonyl)phenyl)amino)pyrimidin-2-yl)amino)-5-isopropoxy-2-methylphenyl)piperazin-1-yl)methyl)-7-fluoro-1-oxoisoindolin-2-yl)piperidine-2,6-dione;
3-(5-(((1-(4-((5-chloro-4-((2-(isopropylsulfonyl)phenyl)amino)pyrimidin-2-yl)amino)-5-isopropoxy-2-methylphenyl)piperidin-4-yl)(methyl)amino)methyl)-l-oxoisoindolin-2-yl)piperidine-2,6-dione;
3-(5-(((1-(4-((5-chloro-4-((2-(isopropylsulfonyl)phenyl)amino)pyrimidin-2-yl)amino)-5-isopropoxy-2-methylphenyl)piperidin-4-yl)(methyl)amino)methyl)-4-fluoro-l-oxoisoindolin-2-yl)piperidine-2,6-dione;
3-(5-(((1-(4-((5-chloro-4-((2-(isopropylsulfonyl)phenyl)amino)pyrimidin-2-yl)amino)-5-isopropoxy-2-methylphenyl)piperidin-4-yl)(methyl)amino)methyl)-6-fluoro-l-oxoisoindolin-2-yl)piperidine-2,6-dione;
3-(5-(((1-(4-((5-chloro-4-((2-(isopropylsulfonyl)phenyl)amino)pyrimidin-2-yl)amino)-5-isopropoxy-2-methylphenyl)piperidin-4-yl)(methyl)amino)methyl)-7-fluoro-l-oxoisoindolin-2-yl)piperidine-2,6-dione;
3-(5-((4-(1-(4-((5-chloro-4-((2-(isopropylsulfonyl)phenyl)amino)pyrimidin-2-yl)amino)-5-isopropoxy-2-methylphenyl)piperidin-4-yl)piperazin-l-yl)methyl)-l-oxoisoindolin-2-yl)piperidine-2,6-dione;
3-(5-((4-(1-(4-((5-chloro-4-((2-(isopropylsulfonyl)phenyl)amino)pyrimidin-2-yl)amino)-5-isopropoxy-2-methylphenyl)piperidin-4-yl)piperazin-l-yl)methyl)-4-fluoro-l-oxoisoindolin-2-yl)piperidine-2,6-dione;
3-(5-((4-(1-(4-((5-chloro-4-((2-(isopropylsulfonyl)phenyl)amino)pyrimidin-2-yl)amino)-5-isopropoxy-2-methylphenyl)piperidin-4-yl)piperazin-l-yl)methyl)-6-fluoro-l-oxoisoindolin-2-yl)piperidine-2,6-dione;
3-(5-((4-(1-(4-((5-chloro-4-((2-(isopropylsulfonyl)phenyl)amino)pyrimidin-2-yl)amino)-5-isopropoxy-2-methylphenyl)piperidin-4-yl)piperazin-1-yl)methyl)-7-fluoro-1-oxoisoindolin-2-yl)piperidine-2,6-dione;
8-(4-(4-((2-(2,6-dioxopiperidin-3-yl)-1-oxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-(4-((2-(2,6-dioxopiperidin-3-yl)-4-fluoro-1-oxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-(4-((2-(2,6-dioxopiperidin-3-yl)-6-fluoro-1-oxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-(4-((2-(2,6-dioxopiperidin-3-yl)-7-fluoro-1-oxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-((2-(2,6-dioxopiperidin-3-yl)-1-oxoisoindolin-5-yl)methyl)piperazin-1-yl)-9-ethyl-6,6-dimethyl-11-oxo-6,11-dihydro-SH-benzo[b]carbazole-3-carbonitrile;
8-(4-((2-(2,6-dioxopiperidin-3-yl)-4-fluoro-1-oxoisoindolin-5-yl)methyl)piperazin-1-yl)-9-ethyl-6,6-dimethyl-11-oxo-6,11-dihydro-5H-benzo[b]carbazole-3-carbonitrile;
8-(4-((2-(2,6-dioxopiperidin-3-yl)-6-fluoro-1-oxoisoindolin-5-yl)methyl)piperazin-1-yl)-9-ethyl-6,6-dimethyl-11-oxo-6,11-dihydro-5H-benzo[b]carbazole-3-carbonitrile;
8-(4-((2-(2,6-dioxopiperidin-3-yl)-7-fluoro-1-oxoisoindolin-5-yl)methyl)piperazin-1-yl)-9-ethyl-6,6-dimethyl-11-oxo-6,11-dihydro-5H-benzo[b]carbazole-3-carbonitrile;
8-(4-(((2-(2,6-dioxopiperidin-3-yl)-1-oxoisoindolin-5-yl)methyl)(methyl)amino)piperidin-1-yl)-9-ethyl-6,6-dimethyl-11-oxo-6,11-dihydro-5H-benzo[b]carbazole-3-carbonitrile;
8-(4-(((2-(2,6-dioxopiperidin-3-yl)-4-fluoro-1-oxoisoindolin-5-yl)methyl)(methyl)amino)piperidin-1-yl)-9-ethyl-6,6-dimethyl-11-oxo-6,11-dihydro-5H-benzo[b]carbazole-3-carbonitrile;
8-(4-(((2-(2,6-dioxopiperidin-3-yl)-6-fluoro-1-oxoisoindolin-5-yl)methyl)(methyl)amino)piperidin-1-yl)-9-ethyl-6,6-dimethyl-11-oxo-6,11-dihydro-5H-benzo[b]carbazole-3-carbonitrile;
8-(4-(((2-(2,6-dioxopiperidin-3-yl)-7-fluoro-1-oxoisoindolin-5-yl)methyl)(methyl)amino)piperidin-1-yl)-9-ethyl-6,6-dimethyl-11-oxo-6,11-dihydro-5H-benzo[b]carbazole-3-carbonitrile;
3-(5-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)-1-oxoisoindolin-2-yl)piperidine-2,6-dione;
3-(5-((4-(1-(4-((5-chloro-4-((2-(dimethylphosphoryl)phenyl)amino)pyrimidin-2-yl)amino)-3-methoxyphenyl)piperidin-4-yl)piperazin-l-yl)methyl)-4-fluoro-l-oxoisoindolin-2-yl)piperidine-2,6-dione;
3-(5-((4-(1-(4-((5-chloro-4-((2-(dimethylphosphoryl)phenyl)amino)pyrimidin-2-yl)amino)-3-methoxyphenyl)piperidin-4-yl)piperazin-l-yl)methyl)-6-fluoro-l-oxoisoindolin-2-yl)piperidine-2,6-dione;
3-(5-((4-(1-(4-((5-chloro-4-((2-(dimethylphosphoryl)phenyl)amino)pyrimidin-2-yl)amino)-3-methoxyphenyl)piperidin-4-yl)piperazin-l-yl)methyl)-7-fluoro-l-oxoisoindolin-2-yl)piperidine-2,6-dione;
(4R)-2⁶-amino-1¹-(1-((2-(2,6-dioxopiperidin-3-yl)-1-oxoisoindolin-5-yl)methyl)piperidin-4-yl)-5⁵-fluoro-4,7-dimethyl-6-oxo-1¹H-3-oxa-7-aza-2(3,5)-pyridina-1(4,3)-pyrazola-5(1,2)-benzenacyclooctaphane-15-carbonitrile;
(4R)-2⁶-amino-1¹-(1-((2-(2,6-dioxopiperidin-3-yl)-4-fluoro-1-oxoisoindolin-5-yl)methyl)piperidin-4-yl)-5⁵-fluoro-4,7-dimethyl-6-oxo-1¹H-3-oxa-7-aza-2(3,5)-pyridina-1(4,3)-pyrazola-5(1,2)-benzenacyclooctaphane-1⁵-carbonitrile;
(4R)-2⁶-amino-1¹-(1-((2-(2,6-dioxopiperidin-3-yl)-6-fluoro-1-oxoisoindolin-5-yl)methyl)piperidin-4-yl)-5⁵-fluoro-4,7-dimethyl-6-oxo-1¹H-3-oxa-7-aza-2(3,5)-pyridina-1(4,3)-pyrazola-5(1,2)-benzenacyclooctaphane-1⁵-carbonitrile;
(4R)-2⁶-amino-1¹-(1-((2-(2,6-dioxopiperidin-3-yl)-7-fluoro-1-oxoisoindolin-5-yl)methyl)piperidin-4-yl)-5⁵-fluoro-4,7-dimethyl-6-oxo-1¹H-3-oxa-7-aza-2(3,5)-pyridina-1(4,3)-pyrazola-5(1,2)-benzenacyclooctaphane-1⁵-carbonitrile;
2-((S)-4-(4-chlorophenyl)-2,3,9-trimethyl-6H-thieno[3,2-f][1,2,4]triazolo[4,3-a][1,4]diazepin-6-yl)-N-((2-(2,6-dioxopiperidin-3-yl)-1-oxoisoindolin-5-yl)methyl)acetamide;
2-((S)-4-(4-chlorophenyl)-2,3,9-trimethyl-6H-thieno[3,2-f][1,2,4]triazolo[4,3-a][1,4]diazepin-6-yl)-N-((2-(2,6-dioxopiperidin-3-yl)-4-fluoro-1-oxoisoindolin-5-yl)methyl)acetamide;
2-((S)-4-(4-chlorophenyl)-2,3,9-trimethyl-6H-thieno[3,2-f][1,2,4]triazolo[4,3-a][1,4]diazepin-6-yl)-N-((2-(2,6-dioxopiperidin-3-yl)-6-fluoro-1-oxoisoindolin-5-yl)methyl)acetamide;
2-((S)-4-(4-chlorophenyl)-2,3,9-trimethyl-6H-thieno[3,2-f][1,2,4]triazolo[4,3-a][1,4]diazepin-6-yl)-N-((2-(2,6-dioxopiperidin-3-yl)-7-fluoro-1-oxoisoindolin-5-yl)methyl)acetamide;
2-((S)-4-(4-chlorophenyl)-2,3,9-trimethyl-6H-thieno[3,2-f][1,2,4]triazolo[4,3-a][1,4]diazepin-6-yl)-N-(2-(4-((2-(2,6-dioxopiperidin-3-yl)-1-oxoisoindolin-5-yl)methyl)piperazin-1-yl)ethyl)acetamide ;
2-((S)-4-(4-chlorophenyl)-2,3,9-trimethyl-6H-thieno[3,2-f][1,2,4]triazolo[4,3-a][1,4]diazepin-6-yl)-N-(2-(4-((2-(2,6-dioxopiperidin-3-yl)-4-fluoro-1-oxoisoindolin-5-yl)methyl)piperazin-1-yl)ethyl)acetamide;
2-((S)-4-(4-chlorophenyl)-2,3,9-trimethyl-6H-thieno[3,2-f][1,2,4]triazolo[4,3-a][1,4]diazepin-6-yl)-N-(2-(4-((2-(2,6-dioxopiperidin-3-yl)-6-fluoro-1-oxoisoindolin-5-yl)methyl)piperazin-1-yl)ethyl)acetamide;
2-((S)-4-(4-chlorophenyl)-2,3,9-trimethyl-6H-thieno[3,2-f][1,2,4]triazolo[4,3-a][1,4]diazepin-6-yl)-N-(2-(4-((2-(2,6-dioxopiperidin-3-yl)-7-fluoro-1-oxoisoindolin-5-yl)methyl)piperazin-1-yl)ethyl)acetamide;
3-(5-((4-(2-((S)-4-(4-chlorophenyl)-2,3,9-trimethyl-6H-thieno[3,2-f][1,2,4]triazolo[4,3-a][1,4]diazepin-6-yl)acetyl)piperazin-1-yl)methyl)-1-oxoisoindolin-2-yl)piperidine-2,6-dione;
3-(5-((4-(2-((S)-4-(4-chlorophenyl)-2,3,9-trimethyl-6H-thieno[3,2-f][1,2,4]triazolo[4,3-a][1,4]diazepin-6-yl)acetyl)piperazin-1-yl)methyl)-4-fluoro-1-oxoisoindolin-2-yl)piperidine-2,6-dione;
3-(5-((4-(2-((S)-4-(4-chlorophenyl)-2,3,9-trimethyl-6H-thieno[3,2-f][1,2,4]triazolo[4,3-a][1,4]diazepin-6-yl)acetyl)piperazin-1-yl)methyl)-6-fluoro-1-oxoisoindolin-2-yl)piperidine-2,6-dione;
3-(5-((4-(2-((S)-4-(4-chlorophenyl)-2,3,9-trimethyl-6H-thieno[3,2-f][1,2,4]triazolo[4,3-a][1,4]diazepin-6-yl)acetyl)piperazin-1-yl)methyl)-7-fluoro-1-oxoisoindolin-2-yl)piperidine-2,6-dione;
3-(5-((4-(2-((S)-4-(4-chlorophenyl)-2,3,9-trimethyl-6H-thieno[3,2-f][1,2,4]triazolo[4,3-a][1,4]diazepin-6-yl)ethyl)piperazin-1-yl)methyl)-1-oxoisoindolin-2-yl)piperidine-2,6-dione;
3-(5-((4-(2-((S)-4-(4-chlorophenyl)-2,3,9-trimethyl-6H-thieno[3,2-f][1,2,4]triazolo[4,3-a][1,4]diazepin-6-yl)ethyl)piperazin-1-yl)methyl)-4-fluoro-1-oxoisoindolin-2-yl)piperidine-2,6-dione ;
3-(5-((4-(2-((S)-4-(4-chlorophenyl)-2,3,9-trimethyl-6H-thieno[3,2-f][1,2,4]triazolo[4,3-a][1,4]diazepin-6-yl)ethyl)piperazin-1-yl)methyl)-6-fluoro-1-oxoisoindolin-2-yl)piperidine-2,6-dione ;
3-(5-((4-(2-((S)-4-(4-chlorophenyl)-2,3,9-trimethyl-6H-thieno[3,2-f][1,2,4]triazolo[4,3-a][1,4]diazepin-6-yl)ethyl)piperazin-1-yl)methyl)-7-fluoro-1-oxoisoindolin-2-yl)piperidine-2,6-dione ;
3-(6-((4-(4-(5,7-dimethoxy-4-oxo-3,4-dihydroquinazolin-2-yl)phenyl)piperazin-1-yl)methyl)-1-oxoisoindolin-2-yl)piperidine-2,6-dione;
3-(6-((4-(4-(5,7-dimethoxy-4-oxo-3,4-dihydroquinazolin-2-yl)phenyl)piperazin-1-yl)methyl)-7-fluoro-1-oxoisoindolin-2-yl)piperidine-2,6-dione;
3-(6-((4-(4-(5,7-dimethoxy-4-oxo-3,4-dihydroquinazolin-2-yl)phenyl)piperazin-1-yl)methyl)-5-fluoro-1-oxoisoindolin-2-yl)piperidine-2,6-dione;
3-(6-((4-(4-(5,7-dimethoxy-4-oxo-3,4-dihydroquinazolin-2-yl)phenyl)piperazin-1-yl)methyl)-4-fluoro-1-oxoisoindolin-2-yl)piperidine-2,6-dione;
3-(5-(((1-(4-(5,7-dimethoxy-4-oxo-3,4-dihydroquinazolin-2-yl)phenyl)piperidin-4-yl)(methyl)amino)methyl)-1-oxoisoindolin-2-yl)piperidine-2,6-dione;
3-(5-(((1-(4-(5,7-dimethoxy-4-oxo-3,4-dihydroquinazolin-2-yl)phenyl)piperidin-4-yl)(methyl)amino)methyl)-4-fluoro-1-oxoisoindolin-2-yl)piperidine-2,6-dione;
3-(5-(((1-(4-(5,7-dimethoxy-4-oxo-3,4-dihydroquinazolin-2-yl)phenyl)piperidin-4-yl)(methyl)amino)methyl)-6-fluoro-1-oxoisoindolin-2-yl)piperidine-2,6-dione;
3-(5-(((1-(4-(5,7-dimethoxy-4-oxo-3,4-dihydroquinazolin-2-yl)phenyl)piperidin-4-yl)(methyl)amino)methyl)-7-fluoro-1-oxoisoindolin-2-yl)piperidine-2,6-dione;
3-(5-((4-(1-(4-(5,7-dimethoxy-4-oxo-3,4-dihydroquinazolin-2-yl)phenyl)piperidin-4-yl)piperazin-1-yl)methyl)-1-oxoisoindolin-2-yl)piperidine-2,6-dione;
3-(5-((4-(1-(4-(5,7-dimethoxy-4-oxo-3,4-dihydroquinazolin-2-yl)phenyl)piperidin-4-yl)piperazin-1-yl)methyl)-4-fluoro-1-oxoisoindolin-2-yl)piperidine-2,6-dione ;
3-(5-((4-(1-(4-(5,7-dimethoxy-4-oxo-3,4-dihydroquinazolin-2-yl)phenyl)piperidin-4-yl)piperazin-l-yl)methyl)-6-fluoro-l-oxoisoindolin-2-yl)piperidine-2,6-dione,'
3-(5-((4-(1-(4-(5,7-dimethoxy-4-oxo-3,4-dihydroquinazolin-2-yl)phenyl)piperidin-4-yl)piperazin-1-yl)methyl)-7-fluoro-1-oxoisoindolin-2-yl)piperidine-2,6-dione;
4-(4-((2-(2,6-dioxopiperidin-3-yl)-1-oxoisoindolin-5-yl)methyl)piperazin-1-yl)-N-(5-((R)-2-methoxy-2-phenylacetyl)-1,4,5,6-tetrahydropyrrolo[3,4-c]pyrazol-3-yl)benzamide;
4-(4-((2-(2,6-dioxopiperidin-3-yl)-4-fluoro-1-oxoisoindolin-5-yl)methyl)piperazin-1-yl)-N-(5-((R)-2-methoxy-2-phenylacetyl)-1,4,5,6-tetrahydropyrrolo[3,4-c]pyrazol-3-yl)benzamide;
4-(4-((2-(2,6-dioxopiperidin-3-yl)-6-fluoro-1-oxoisoindolin-5-yl)methyl)piperazin-1-yl)-N-(5-((R)-2-methoxy-2-phenylacetyl)-1,4,5,6-tetrahydropyrrolo[3,4-c]pyrazol-3-yl)benzamide;
4-(4-((2-(2,6-dioxopiperidin-3-yl)-7-fluoro-1-oxoisoindolin-5-yl)methyl)piperazin-1-yl)-N-(5-((R)-2-methoxy-2-phenylacetyl)-1,4,5,6-tetrahydropyrrolo[3,4-c]pyrazol-3-yl)benzamide;
4-(4-(((2-(2,6-dioxopiperidin-3-yl)-1-oxoisoindolin-5-yl)methyl)(methyl)amino)piperidin-1-yl)-N-(5-((R)-2-methoxy-2-phenylacetyl)-1,4,5,6-tetrahydropyrrolo[3,4-c]pyrazol-3-yl)benzamide;
4-(4-(((2-(2,6-dioxopiperidin-3-yl)-4-fluoro-1-oxoisoindolin-5-yl)methyl)(methyl)amino)piperidin-1-yl)-N-(5-((R)-2-methoxy-2-phenylacetyl)-1,4,5,6-tetrahydropyrrolo[3,4-c]pyrazol-3-yl)benzamide;
4-(4-(((2-(2,6-dioxopiperidin-3-yl)-6-fluoro-1-oxoisoindolin-5-yl)methyl)(methyl)amino)piperidin-l-yl)-N-(5-((R)-2-methoxy-2-phenylacetyl)-l,4,5,6-tetrahydropyrrolo[3,4-c]pyrazol-3-yl)benzamide;
4-(4-(((2-(2,6-dioxopiperidin-3-yl)-7-fluoro-1-oxoisoindolin-5-yl)methyl)(methyl)amino)piperidin-l-yl)-N-(5-((R)-2-methoxy-2-phenylacetyl)-l,4,5,6-tetrahydropyrrolo[3,4-c]pyrazol-3-yl)benzamide;
4-(4-(4-((2-(2,6-dioxopiperidin-3-yl)-1-oxoisoindolin-5-yl)methyl)piperazin-1-yl)piperidin-1-yl)-N-(5-((R)-2-methoxy-2-phenylacetyl)-1,4,5,6-tetrahydropyrrolo[3,4-c]pyrazol-3-yl)benzamide;
4-(4-(4-((2-(2,6-dioxopiperidin-3-yl)-4-fluoro-1-oxoisoindolin-5-yl)methyl)piperazin-1-yl)piperidin-l-yl)-N-(5-((R)-2-methoxy-2-phenylacetyl)-l,4,5,6-tetrahydropyrrolo[3,4-c]pyrazol-3-yl)benzamide;
4-(4-(4-((2-(2,6-dioxopiperidin-3-yl)-6-fluoro-1-oxoisoindolin-5-yl)methyl)piperazin-1-yl)piperidin-l-yl)-N-(5-((R)-2-methoxy-2-phenylacetyl)-l,4,5,6-tetrahydropyrrolo[3,4-c]pyrazol-3-yl)benzamide;
4-(4-(4-((2-(2,6-dioxopiperidin-3-yl)-7-fluoro-1-oxoisoindolin-5-yl)methyl)piperazin-1-yl)piperidin-l-yl)-N-(5-((R)-2-methoxy-2-phenylacetyl)-l,4,5,6-tetrahydropyrrolo[3,4-c]pyrazol-3-yl)benzamide;
N-(tert-butyl)-3-((2-((6-(4-((2-(2,6-dioxopiperidin-3-yl)-1-oxoisoindolin-5-yl)methyl)piperazin-1-yl)pyridazin-3-yl)amino)-5-methylpyrimidin-4-yl)amino)benzenesulfonamide;
N-(tert-butyl)-3-((2-((6-(4-((2-(2,6-dioxopiperidin-3-yl)-4-fluoro-1-oxoisoindolin-5-yl)methyl)piperazin-1-yl)pyridazin-3-yl)amino)-5-methylpyrimidin-4-yl)amino)benzenesulfonamide;
N-(tert-butyl)-3-((2-((6-(4-((2-(2,6-dioxopiperidin-3-yl)-6-fluoro-1-oxoisoindolin-5-yl)methyl)piperazin-1-yl)pyridazin-3-yl)amino)-5-methylpyrimidin-4-yl)amino)benzenesulfonamide;
N-(tert-butyl)-3-((2-((6-(4-((2-(2,6-dioxopiperidin-3-yl)-7-fluoro-1-oxoisoindolin-5-yl)methyl)piperazin-1-yl)pyridazin-3-yl)amino)-5-methylpyrimidin-4-yl)amino)benzenesulfonamide;
N-(tert-butyl)-3-((2-((4-(4-(((2-(2,6-dioxopiperidin-3-yl)-1-oxoisoindolin-5-yl)methyl)(methyl)amino)piperidin-1-yl)phenyl)amino)-5-methylpyrimidin-4-yl)amino)benzenesulfonamide;
N-(tert-butyl)-3-((2-((4-(4-(((2-(2,6-dioxopiperidin-3-yl)-4-fluoro-1-oxoisoindolin-5-yl)methyl)(methyl)amino)piperidin-1-yl)phenyl)amino)-5-methylpyrimidin-4-yl)amino)benzenesulfonamide;
N-(tert-butyl)-3-((2-((4-(4-(((2-(2,6-dioxopiperidin-3-yl)-6-fluoro-1-oxoisoindolin-5-yl)methyl)(methyl)amino)piperidin-1-yl)phenyl)amino)-5-methylpyrimidin-4-yl)amino)benzenesulfonamide;
N-(tert-butyl)-3-((2-((4-(4-(((2-(2,6-dioxopiperidin-3-yl)-7-fluoro-1-oxoisoindolin-5-yl)methyl)(methyl)amino)piperidin-1-yl)phenyl)amino)-5-methylpyrimidin-4-yl)amino)benzenesulfonamide;
N-(tert-butyl)-3-((2-((4-(4-(4-((2-(2,6-dioxopiperidin-3-yl)-1-oxoisoindolin-5-yl)methyl)piperazin-1-yl)piperidin-1-yl)phenyl)amino)-5-methylpyrimidin-4-yl)amino)benzenesulfonamide;
N-(tert-butyl)-3-((2-((4-(4-(4-((2-(2,6-dioxopiperidin-3-yl)-4-fluoro-1-oxoisoindolin-5-yl)methyl)piperazin-1-yl)piperidin-1-yl)phenyl)amino)-5-methylpyrimidin-4-yl)amino)benzenesulfonamide;
N-(tert-butyl)-3-((2-((4-(4-(4-((2-(2,6-dioxopiperidin-3-yl)-6-fluoro-1-oxolsoindolin-5-yl)methyl)piperazin-1-yl)piperidin-1-yl)phenyl)amino)-5-methylpyrimidin-4-yl)amino)benzenesulfonamide;
N-(tert-butyl)-3-((2-((4-(4-(4-((2-(2,6-dioxopiperidin-3-yl)-7-fluoro-1-oxolsoindolin-5-yl)methyl)piperazin-1-yl)piperidin-1-yl)phenyl)amino)-5-methylpyrimidin-4-yl)amino)benzenesulfonamide;
N-(tert-butyl)-3-((2-((4-(4-(1-((2-(2,6-dioxopiperidin-3-yl)-1-oxoisoindolin-5-yl)methyl)piperidin-4-yl)piperazin-1-yl)phenyl)amino)-5-methylpyrimidin-4-yl)amino)benzenesulfonamide;
N-(tert-butyl)-3-((2-((4-(4-(1-((2-(2,6-dioxopiperidin-3-yl)-4-fluoro-1-oxoisoindolin-5-yl)methyl)piperidin-4-yl)piperazin-1-yl)phenyl)amino)-5-methylpyrimidin-4-yl)amino)benzenesulfonamide;
N-(tert-butyl)-3-((2-((4-(4-(1-((2-(2,6-dioxopiperidin-3-yl)-6-fluoro-1-oxoisoindolin-5-yl)methyl)piperidin-4-yl)piperazin-1-yl)phenyl)amino)-5-methylpyrimidin-4-yl)amino)benzenesulfonamide;
N-(tert-butyl)-3-((2-((4-(4-(1-((2-(2,6-dioxopiperidin-3-yl)-7-fluoro-1-oxoisoindolin-5-yl)methyl)piperidin-4-yl)piperazin-1-yl)phenyl)amino)-5-methylpyrimidin-4-yl)amino)benzenesulfonamide;
N-(tert-butyl)-3-((2-((4-(4-((2-(2,6-dioxopiperidin-3-yl)-1-oxoisoindolin-5-yl)methyl)piperazin-1-yl)phenyl)amino)-5-methylpyrimidin-4-yl)amino)benzenesulfonamide;
N-(tert-butyl)-3-((2-((4-(4-((2-(2,6-dioxopiperidin-3-yl)-4-fluoro-1-oxoisoindolin-5-yl)methyl)piperazin-1-yl)phenyl)amino)-5-methylpyrimidin-4-yl)amino)benzenesulfonamide;
N-(tert-butyl)-3-((2-((4-(4-((2-(2,6-dioxopiperidin-3-yl)-6-fluoro-1-oxoisoindolin-5-yl)methyl)piperazin-1-yl)phenyl)amino)-5-methylpyrimidin-4-yl)amino)benzenesulfonamide;
N-(tert-butyl)-3-((2-((4-(4-((2-(2,6-dioxopiperidin-3-yl)-7-fluoro-1-oxoisoindolin-5-yl)methyl)piperazin-1-yl)phenyl)amino)-5-methylpyrimidin-4-yl)amino)benzenesulfonamide;
N-(tert-butyl)-3-((2-((4-(1-((2-(2,6-dioxopiperidin-3-yl)-1-oxoisoindolin-5-yl)methyl)piperidin-4-yl)phenyl)amino)-5-methylpyrimidin-4-yl)amino)benzenesulfonamide;
N-(tert-butyl)-3-((2-((4-(1-((2-(2,6-dioxopiperidin-3-yl)-4-fluoro-1-oxoisoindolin-5-yl)methyl)piperidin-4-yl)phenyl)amino)-5-methylpyrimidin-4-yl)amino)benzenesulfonamide;
N-(tert-butyl)-3-((2-((4-(1-((2-(2,6-dioxopiperidin-3-yl)-6-fluoro-1-oxoisoindolin-5-yl)methyl)piperidin-4-yl)phenyl)amino)-5-methylpyrimidin-4-yl)amino)benzenesulfonamide;
N-(tert-butyl)-3-((2-((4-(1-((2-(2,6-dioxopiperidin-3-yl)-7-fluoro-1-oxoisoindolin-5-yl)methyl)piperidin-4-yl)phenyl)amino)-5-methylpyrimidin-4-yl)amino)benzenesulfonamide;
N-(tert-butyl)-3-((2-((4-(2-(4-((2-(2,6-dioxopiperidin-3-yl)-1-oxoisoindolin-5-yl)methyl)piperazin-l-yl)ethoxy)phenyl)amino)-5-methylpyrimidin-4-yl)amino)benzenesulfonamide;
N-(tert-butyl)-3-((2-((4-(2-(4-((2-(2,6-dioxopiperidin-3-yl)-4-fluoro-1-oxoisoindolin-5-yl)methyl)piperazin-l-yl)ethoxy)phenyl)amino)-5-methylpyrimidin-4-yl)amino)benzenesulfonamide;
N-(tert-butyl)-3-((2-((4-(2-(4-((2-(2,6-dioxopiperidin-3-yl)-6-fluoro-1-oxoisoindolin-5-yl)methyl)piperazin-l-yl)ethoxy)phenyl)amino)-5-methylpyrimidin-4-yl)amino)benzenesulfonamide;
N-(tert-butyl)-3-((2-((4-(2-(4-((2-(2,6-dioxopiperidin-3-yl)-7-fluoro-1-oxoisoindolin-5-yl)methyl)piperazin-l-yl)ethoxy)phenyl)amino)-5-methylpyrimidin-4-yl)amino)benzenesulfonamide;
2-((2-((4-(4-(4-((2-(2,6-dioxopiperidin-3-yl)-1-oxoisoindolin-5-yl)methyl)piperazin-1-yl)piperidin-l-yl)-2-methoxyphenyl)amino)-5-(trifluoromethyl)pyridin-4-yl)amino)-N-methylbenzamide;
2-((2-((4-(4-(4-((2-(2,6-dioxopiperidin-3-yl)-4-fluoro-1-oxoisoindolin-5-yl)methyl)piperazin-1-yl)piperidin-l-yl)-2-methoxyphenyl)amino)-5-(trifluoromethyl)pyridin-4-yl)amino)-N-methylbenzamide;
2-((2-((4-(4-(4-((2-(2,6-dioxopiperidin-3-yl)-6-fluoro-1-oxoisoindolin-5-yl)methyl)piperazin-1-yl)piperidin-l-yl)-2-methoxyphenyl)amino)-5-(trifluoromethyl)pyridin-4-yl)amino)-N-methylbenzamide;
2-((2-((4-(4-(4-((2-(2,6-dioxopiperidin-3-yl)-7-fluoro-1-oxoisoindolin-5-yl)methyl)piperazin-1-yl)piperidin-l-yl)-2-methoxyphenyl)amino)-5-(trifluoromethyl)pyridin-4-yl)amino)-N-methylbenzamide;
2-((2-((4-(4-(((2-(2,6-dioxopiperidin-3-yl)-1-oxoisoindolin-5-yl)methyl)amino)piperidin-1-yl)-2-methoxyphenyl)amino)-5-(trifluoromethyl)pyridin-4-yl)amino)-N-methylbenzamide;
2-((2-((4-(4-(((2-(2,6-dioxopiperidin-3-yl)-4-fluoro-1-oxoisoindolin-5-yl)methyl)amino)piperidin-l-yl)-2-methoxyphenyl)amino)-5-(trifluoromethyl)pyridin-4-yl)amino)-N-methylbenzamide;
2-((2-((4-(4-(((2-(2,6-dioxopiperidin-3-yl)-6-fluoro-1-oxoisoindolin-5-yl)methyl)amino)piperidin-l-yl)-2-methoxyphenyl)amino)-5-(trifluoromethyl)pyridin-4-yl)amino)-N-methylbenzamide;
2-((2-((4-(4-(((2-(2,6-dioxopiperidin-3-yl)-7-fluoro-1-oxoisoindolin-5-yl)methyl)amino)piperidin-l-yl)-2-methoxyphenyl)amino)-5-(trifluoromethyl)pyridin-4-yl)amino)-N-methylbenzamide;
2-((2-((4-(4-(((2-(2,6-dioxopiperidin-3-yl)-1-oxoisoindolin-5-yl)methyl)(methyl)amino)piperidin-1-yl)-2-methoxyphenyl)amino)-5-(trifluoromethyl)pyridin-4-yl)amino)-N-methylbenzamide;
2-((2-((4-(4-(((2-(2,6-dioxopiperidin-3-yl)-4-fluoro-1-oxoisoindolin-5-yl)methyl)(methyl)amino)piperidin-1-yl)-2-methoxyphenyl)amino)-5-(trifluoromethyl)pyridin-4-yl)amino)-N-methylbenzamide;
2-((2-((4-(4-(((2-(2,6-dioxopiperidin-3-yl)-6-fluoro-1-oxoisoindolin-5-yl)methyl)(methyl)amino)piperidin-1-yl)-2-methoxyphenyl)amino)-5-(trifluoromethyl)pyridin-4-yl)amino)-N-methylbenzamide;
2-((2-((4-(4-(((2-(2,6-dioxopiperidin-3-yl)-7-fluoro-1-oxoisoindolin-5-yl)methyl)(methyl)amino)piperidin-1-yl)-2-methoxyphenyl)amino)-5-(trifluoromethyl)pyridin-4-yl)amino)-N-methylbenzamide;
2-((2-((4-(4-((2-(2,6-dioxopiperidin-3-yl)-1-oxoisoindolin-5-yl)methyl)piperazin-1-yl)-2-methoxyphenyl)amino)-5-(trifluoromethyl)pyridin-4-yl)amino)-N-methylbenzamide;
2-((2-((4-(4-((2-(2,6-dioxopiperidin-3-yl)-4-fluoro-1-oxoisoindolin-5-yl)methyl)piperazin-1-yl)-2-methoxyphenyl)amino)-5-(trifluoromethyl)pyridin-4-yl)amino)-N-methylbenzamide;
2-((2-((4-(4-((2-(2,6-dioxopiperidin-3-yl)-6-fluoro-1-oxoisoindolin-5-yl)methyl)piperazin-1-yl)-2-methoxyphenyl)amino)-5-(trifluoromethyl)pyridin-4-yl)amino)-N-methylbenzamide;
2-((2-((4-(4-((2-(2,6-dioxopiperidin-3-yl)-7-fluoro-1-oxoisoindolin-5-yl)methyl)piperazin-1-yl)-2-methoxyphenyl)amino)-5-(trifluoromethyl)pyridin-4-yl)amino)-N-methylbenzamide;
N-(3-(((2-((4-(4-((2-(2,6-dioxopiperidin-3-yl)-1-oxoisoindolin-5-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((2-(2,6-dioxopiperidin-3-yl)-4-fluoro-1-oxoisoindolin-5-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((2-(2,6-dioxopiperidin-3-yl)-6-fluoro-1-oxoisoindolin-5-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((2-(2,6-dioxopiperidin-3-yl)-7-fluoro-1-oxoisoindolin-5-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(((2-(2,6-dioxopiperidin-3-yl)-3-oxoisoindolin-5-yl)methyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(((2-(2,6-dioxopiperidin-3-yl)-4-fluoro-3-oxoisoindolin-5-yl)methyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(((2-(2,6-dioxopiperidin-3-yl)-6-fluoro-3-oxoisoindolin-5-yl)methyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(((2-(2,6-dioxopiperidin-3-yl)-7-fluoro-3-oxoisoindolin-5-yl)methyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-((2-(2,6-dioxopiperidin-3-yl)-1-oxoisoindolin-5-yl)methyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-((2-(2,6-dioxopiperidin-3-yl)-4-fluoro-1-oxoisoindolin-5-yl)methyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-((2-(2,6-dioxopiperidin-3-yl)-6-fluoro-1-oxoisoindolin-5-yl)methyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-((2-(2,6-dioxopiperidin-3-yl)-7-fluoro-1-oxoisoindolin-5-yl)methyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((2-(2,6-dioxopiperidin-3-yl)-1-oxoisoindolin-5-yl)methyl)piperazin-1-yl)phenyl)amino)-5-(trifluoromethyl)pyrimidin-4-yl)amino)methyl)phenyl)-N-methylmethanesulfonamide;
N-(3-(((2-((4-(4-((2-(2,6-dioxopiperidin-3-yl)-4-fluoro-1-oxoisoindolin-5-yl)methyl)piperazin-1-yl)phenyl)amino)-5-(trifluoromethyl)pyrimidin-4-yl)amino)methyl)phenyl)-N-methylmethanesulfonamide;
N-(3-(((2-((4-(4-((2-(2,6-dioxopiperidin-3-yl)-6-fluoro-1-oxoisoindolin-5-yl)methyl)piperazin-1-yl)phenyl)amino)-5-(trifluoromethyl)pyrimidin-4-yl)amino)methyl)phenyl)-N-methylmethanesulfonamide;
N-(3-(((2-((4-(4-((2-(2,6-dioxopiperidin-3-yl)-7-fluoro-1-oxoisoindolin-5-yl)methyl)piperazin-1-yl)phenyl)amino)-5-(trifluoromethyl)pyrimidin-4-yl)amino)methyl)phenyl)-N-methylmethanesulfonamide;
N-(3-(((2-((4-(4-(((2-(2,6-dioxopiperidin-3-yl)-1-oxoisoindolin-5-yl)methyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-(((2-(2,6-dioxopiperidin-3-yl)-4-fluoro-1-oxoisoindolin-5-yl)methyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-(((2-(2,6-dioxopiperidin-3-yl)-6-fluoro-1-oxoisoindolin-5-yl)methyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-(((2-(2,6-dioxopiperidin-3-yl)-7-fluoro-1-oxoisoindolin-5-yl)methyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-((2-(2,6-dioxopiperidin-3-yl)-1-oxoisoindolin-5-yl)methyl)piperazin-1-yl)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-((2-(2,6-dioxopiperidin-3-yl)-4-fluoro-1-oxoisoindolin-5-yl)methyl)piperazin-1-yl)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-((2-(2,6-dioxopiperidin-3-yl)-6-fluoro-1-oxoisoindolin-5-yl)methyl)piperazin-1-yl)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-((2-(2,6-dioxopiperidin-3-yl)-7-fluoro-1-oxoisoindolin-5-yl)methyl)piperazin-1-yl)piperidin-1-yl)phenyl)amino)-5-(trifluoromethyl)pyrimidin-4-yl)amino)methyl)phenyl)-N-methylmethanesulfonamide;
N-(3-(((2-((4-(((2-(2,6-dioxopiperidin-3-yl)-1-oxoisoindolin-5-yl)methyl)amino)phenyl)amino)-5-(trifluoromethyl)pyrimidin-4-yl)amino)methyl)phenyl)-N-methylmethanesulfonamide;
N-(3-(((2-((4-(((2-(2,6-dioxopiperidin-3-yl)-4-fluoro-1-oxoisoindolin-5-yl)methyl)amino)phenyl)amino)-5-(trifluoromethyl)pyrimidin-4-yl)amino)methyl)phenyl)-N-methylmethanesulfonamide;
N-(3-(((2-((4-(((2-(2,6-dioxopiperidin-3-yl)-6-fluoro-1-oxoisoindolin-5-yl)methyl)amino)phenyl)amino)-5-(trifluoromethyl)pyrimidin-4-yl)amino)methyl)phenyl)-N-methylmethanesulfonamide;
N-(3-(((2-((4-(((2-(2,6-dioxopiperidin-3-yl)-7-fluoro-1-oxoisoindolin-5-yl)methyl)amino)phenyl)amino)-5-(trifluoromethyl)pyrimidin-4-yl)amino)methyl)phenyl)-N-methylmethanesulfonamide;
N-(3-(((2-((4-(4-((2-(2,6-dioxopiperidin-3-yl)-1-oxoisoindolin-5-yl)methyl)piperazin-1-yl)phenyl)amino)-5-(trifluoromethyl)pyrimidin-4-yl)amino)methyl)pyridin-2-yl)-N-methylmethanesulfonamide;
N-(3-(((2-((4-(4-((2-(2,6-dioxopiperidin-3-yl)-4-fluoro-1-oxoisoindolin-5-yl)methyl)piperazin-1-yl)phenyl)amino)-5-(trifluoromethyl)pyrimidin-4-yl)amino)methyl)pyridin-2-yl)-N-methylmethanesulfonamide;
N-(3-(((2-((4-(4-((2-(2,6-dioxopiperidin-3-yl)-6-fluoro-1-oxoisoindolin-5-yl)methyl)piperazin-1-yl)phenyl)amino)-5-(trifluoromethyl)pyrimidin-4-yl)amino)methyl)pyridin-2-yl)-N-methylmethanesulfonamide;
N-(3-(((2-((4-(4-((2-(2,6-dioxopiperidin-3-yl)-7-fluoro-1-oxoisoindolin-5-yl)methyl)piperazin-1-yl)phenyl)amino)-5-(trifluoromethyl)pyrimidin-4-yl)amino)methyl)pyridin-2-yl)-N-methylmethanesulfonamide;
N-(3-(((2-((4-(4-(((2-(2,6-dioxopiperidin-3-yl)-1-oxoisoindolin-5-yl)methyl)(methyl)amino)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(((2-(2,6-dioxopiperidin-3-yl)-7-fluoro-1-oxoisoindolin-5-yl)methyl)(methyl)amino)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(((2-(2,6-dioxopiperidin-3-yl)-6-fluoro-1-oxoisoindolin-5-yl)methyl)(methyl)amino)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(((2-(2,6-dioxopiperidin-3-yl)-7-fluoro-1-oxoisoindolin-5-yl)methyl)(methyl)amino)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-((2-(2,6-dioxopiperidin-3-yl)-1-oxoisoindolin-5-yl)methyl)piperazin-1-yl)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-((2-(2,6-dioxopiperidin-3-yl)-4-fluoro-1-oxoisoindolin-5-yl)methyl)piperazin-1-yl)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-((2-(2,6-dioxopiperidin-3-yl)-6-fluoro-1-oxoisoindolin-5-yl)methyl)piperazin-1-yl)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-((2-(2,6-dioxopiperidin-3-yl)-7-fluoro-1-oxoisoindolin-5-yl)methyl)piperazin-1-yl)piperidin-1-yl)phenyl)amino)-5-(trifluoromethyl)pyrimidin-4-yl)amino)methyl)pyridin-2-yl)-N-methylmethanesulfonamide;
2-(2-((cyclopropylmethyl)amino)pyridin-4-yl)-N-(3-(difluoromethyl)-1-(1-((2-(2,6-dioxopiperidin-3-yl)-1-oxoisoindolin-5-yl)methyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-((2-(2,6-dioxopiperidin-3-yl)-4-fluoro-1-oxoisoindolin-5-yl)methyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-((2-(2,6-dioxopiperidin-3-yl)-6-fluoro-1-oxoisoindolin-5-yl)methyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-((2-(2,6-dioxopiperidin-3-yl)-7-fluoro-1-oxoisoindolin-5-yl)methyl)piperidin-4-yl)-1H-pyrazol-4-yl)oxazole-4-carboxamide;
N-(3-(difluoromethyl)-1-(1-((2-(2,6-dioxopiperidin-3-yl)-1-oxoisoindolin-5-yl)methyl)piperidin-4-yl)-1H-pyrazol-4-yl)-5-morpholinopyrazolo[1,5-a]pyrimidine-3-carboxamide;
N-(3-(difluoromethyl)-1-(1-((2-(2,6-dioxopiperidin-3-yl)-4-fluoro-1-oxoisoindolin-5-yl)methyl)piperidin-4-yl)-1H-pyrazol-4-yl)-5-morpholinopyrazolo[1,5-a]pyrimidine-3-carboxamide;
N-(3-(difluoromethyl)-1-(1-((2-(2,6-dioxopiperidin-3-yl)-6-fluoro-1-oxoisoindolin-5-yl)methyl)piperidin-4-yl)-1H-pyrazol-4-yl)-5-morpholinopyrazolo[1,5-a]pyrimidine-3-carboxamide;
N-(3-(difluoromethyl)-1-(1-((2-(2,6-dioxopiperidin-3-yl)-7-fluoro-1-oxoisoindolin-5-yl)methyl)piperidin-4-yl)-1H-pyrazol-4-yl)-5-morpholinopyrazolo[1,5-a]pyrimidine-3-carboxamide;
N-(2-(4-((2-(2,6-dioxopiperidin-3-yl)-1-oxoisoindolin-5-yl)methyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide;
N-(2-(4-((2-(2,6-dioxopiperidin-3-yl)-4-fluoro-1-oxoisoindolin-5-yl)methyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide;
N-(2-(4-((2-(2,6-dioxopiperidin-3-yl)-6-fluoro-1-oxoisoindolin-5-yl)methyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide;
N-(2-(4-((2-(2,6-dioxopiperidin-3-yl)-7-fluoro-1-oxoisoindolin-5-yl)methyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide;
4-((1-((2-(2,6-dioxopiperidin-3-yl)-1-oxoisoindolin-5-yl)methyl)piperidin-4-yl)ethynyl)-1-(((2S,3S,4S)-3-ethyl-4-fluoro-5-oxopyrrolidin-2-yl)methoxy)-7-methoxyisoquinoline-6-carboxamide;
4-((1-((2-(2,6-dioxopiperidin-3-yl)-4-fluoro-1-oxoisoindolin-5-yl)methyl)piperidin-4-yl)ethynyl)-1-(((2S,3S,4S)-3-ethyl-4-fluoro-5-oxopyrrolidin-2-yl)methoxy)-7-methoxyisoquinoline-6-carboxamide;
4-((1-((2-(2,6-dioxopiperidin-3-yl)-6-fluoro-1-oxoisoindolin-5-yl)methyl)piperidin-4-yl)ethynyl)-1-(((2S,3S,4S)-3-ethyl-4-fluoro-5-oxopyrrolidin-2-yl)methoxy)-7-methoxyisoquinoline-6-carboxamide;
4-((1-((2-(2,6-dioxopiperidin-3-yl)-7-fluoro-1-oxoisoindolin-5-yl)methyl)piperidin-4-yl)ethynyl)-1-(((2S,3S,4S)-3-ethyl-4-fluoro-5-oxopyrrolidin-2-yl)methoxy)-7-methoxyisoquinoline-6-carboxamide;
6-(6-(4-((2-(2,6-dioxopiperidin-3-yl)-1-oxoisoindolin-5-yl)methyl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-((2-(2,6-dioxopiperidin-3-yl)-4-fluoro-1-oxoisoindolin-5-yl)methyl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-((2-(2,6-dioxopiperidin-3-yl)-6-fluoro-1-oxoisoindolin-5-yl)methyl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-((2-(2,6-dioxopiperidin-3-yl)-7-fluoro-1-oxoisoindolin-5-yl)methyl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(((2-(2,6-dioxopiperidin-3-yl)-1-oxoisoindolin-5-yl)methyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(((2-(2,6-dioxopiperidin-3-yl)-4-fluoro-1-oxoisoindolin-5-yl)methyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(((2-(2,6-dioxopiperidin-3-yl)-6-fluoro-1-oxoisoindolin-5-yl)methyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(((2-(2,6-dioxopiperidin-3-yl)-7-fluoro-1-oxoisoindolin-5-yl)methyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-((2-(2,6-dioxopiperidin-3-yl)-1-oxoisoindolin-5-yl)methyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-((2-(2,6-dioxopiperidin-3-yl)-4-fluoro-1-oxoisoindolin-5-yl)methyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-((2-(2,6-dioxopiperidin-3-yl)-6-fluoro-1-oxoisoindolin-5-yl)methyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-((2-(2,6-dioxopiperidin-3-yl)-7-fluoro-1-oxoisoindolin-5-yl)methyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
N-((4,6-dimethyl-2-oxo-1,2-dihydropyridin-3-yl)methyl)-4'-((4-((2-(2,6-dioxopiperidin-3-yl)-1-oxoisoindolin-5-yl)methyl)piperazin-1-yl)methyl)-5-(ethyl(tetrahydro-2H-pyran-4-yl)amino)-4-methyl-[1,1'-biphenyl]-3 -carboxamide;
N-((4,6-dimethyl-2-oxo-1,2-dihydropyridin-3-yl)methyl)-4'-((4-((2-(2,6-dioxopiperidin-3-yl)-4-fluoro-1-oxoisoindolin-5-yl)methyl)piperazin-1-yl)methyl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-((4-((2-(2,6-dioxopiperidin-3-yl)-6-fluoro-1-oxoisoindolin-5-yl)methyl)piperazin-1-yl)methyl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-((4-((2-(2,6-dioxopiperidin-3-yl)-7-fluoro-1-oxoisoindolin-5-yl)methyl)piperazin-1-yl)methyl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-((2-(2,6-dioxopiperidin-3-yl)-1-oxoisoindolin-5-yl)methyl)piperazin-1-yl)-5-(ethyl(tetrahydro-2H-pyran-4-yl)amino)-4-methyl-[1,1'-biphenyl] -3 -carboxamide;
N-((4,6-dimethyl-2-oxo-1,2-dihydropyridin-3-yl)methyl)-4'-(4-((2-(2,6-dioxopiperidin-3-yl)-4-fluoro-1-oxoisoindolin-5-yl)methyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-((2-(2,6-dioxopiperidin-3-yl)-6-fluoro-1-oxoisoindolin-5-yl)methyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-((2-(2,6-dioxopiperidin-3-yl)-7-fluoro-1-oxoisoindolin-5-yl)methyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-((2-(2,6-dioxopiperidin-3-yl)-1-oxoisoindolin-5-yl)methyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-((2-(2,6-dioxopiperidin-3-yl)-4-fluoro-1-oxoisoindolin-5-yl)methyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-((2-(2,6-dioxopiperidin-3-yl)-6-fluoro-1-oxoisoindolin-5-yl)methyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-((2-(2,6-dioxopiperidin-3-yl)-7-fluoro-1-oxoisoindolin-5-yl)methyl)piperazin-1-yl)-5-(ethyl(tetrahydro-2H-pyran-4-yl)amino)-4-methyl-[1,1'-biphenyl]-3-carboxamide;
N-(5-(3,5-difluorobenzyl)-1H-indazol-3-yl)-4-(4-((2-(2,6-dioxopiperidin-3-yl)-1-oxoisoindolin-5-yl)methyl)piperazin-1-yl)-2-((tetrahydro-2H-pyran-4-yl)amino)benzamide;
N-(5-(3,5-difluorobenzyl)-1H-indazol-3-yl)-4-(4-((2-(2,6-dioxopiperidin-3-yl)-4-fluoro-1-oxoisoindolin-5-yl)methyl)piperazin-1-yl)-2-((tetrahydro-2H-pyran-4-yl)amino)benzamide;
N-(5-(3,5-difluorobenzyl)-1H-indazol-3-yl)-4-(4-((2-(2,6-dioxopiperidin-3-yl)-6-fluoro-1-oxoisoindolin-5-yl)methyl)piperazin-1-yl)-2-((tetrahydro-2H-pyran-4-yl)amino)benzamide;
N-(5-(3,5-difluorobenzyl)-1H-indazol-3-yl)-4-(4-((2-(2,6-dioxopiperidin-3-yl)-7-fluoro-1-oxoisoindolin-5-yl)methyl)piperazin-1-yl)-2-((tetrahydro-2H-pyran-4-yl)amino)benzamide;
N-(5-(3,5-difluorobenzyl)-1H-indazol-3-yl)-4-(4-(((2-(2,6-dioxopiperidin-3-yl)-1-oxoisoindolin-5-yl)methyl)(methyl)amino)piperidin-1-yl)-2-((tetrahydro-2H-pyran-4-yl)amino)benzamide;
N-(5-(3,5-difluorobenzyl)-1H-indazol-3-yl)-4-(4-(((2-(2,6-dioxopiperidin-3-yl)-4-fluoro-1-oxoisoindolin-5-yl)methyl)(methyl)amino)piperidin-1-yl)-2-((tetrahydro-2H-pyran-4-yl)amino)benzamide;
N-(5-(3,5-difluorobenzyl)-1H-indazol-3-yl)-4-(4-(((2-(2,6-dioxopiperidin-3-yl)-6-fluoro-1-oxoisoindolin-5-yl)methyl)(methyl)amino)piperidin-1-yl)-2-((tetrahydro-2H-pyran-4-yl)amino)benzamide;
N-(5-(3,5-difluorobenzyl)-1H-indazol-3-yl)-4-(4-(((2-(2,6-dioxopiperidin-3-yl)-7-fluoro-1-oxoisoindolin-5-yl)methyl)(methyl)amino)piperidin-1-yl)-2-((tetrahydro-2H-pyran-4-yl)amino)benzamide;
N-(5-(3,5-difluorobenzyl)-1H-indazol-3-yl)-4-(4-(1-((2-(2,6-dioxopiperidin-3-yl)-1-oxoisoindolin-5-yl)methyl)piperidin-4-yl)piperazin-1-yl)-2-((tetrahydro-2H-pyran-4-yl)amino)benzamide;
N-(5-(3,5-difluorobenzyl)-1H-indazol-3-yl)-4-(4-(1-((2-(2,6-dioxopiperidin-3-yl)-4-fluoro-1-oxoisoindolin-5-yl)methyl)piperidin-4-yl)piperazin-1-yl)-2-((tetrahydro-2H-pyran-4-yl)amino)benzamide;
N-(5-(3,5-difluorobenzyl)-1H-indazol-3-yl)-4-(4-(1-((2-(2,6-dioxopiperidin-3-yl)-6-fluoro-1-oxoisoindolin-5-yl)methyl)piperidin-4-yl)piperazin-1-yl)-2-((tetrahydro-2H-pyran-4-yl)amino)benzamide;
N-(5-(3,5-difluorobenzyl)-1H-indazol-3-yl)-4-(4-(1-((2-(2,6-dioxopiperidin-3-yl)-7-fluoro-1-oxoisoindolin-5-yl)methyl)piperidin-4-yl)piperazin-1-yl)-2-((tetrahydro-2H-pyran-4-yl)amino)benzamide;
3-(5-((4-(6-(6-((R)-2-(3-fluorophenyl)pyrrolidin-1-yl)imidazo[1,2-b]pyridazin-3-yl)pyridin-2-yl)piperazin-l-yl)methyl)-l-oxoisoindolin-2-yl)piperidine-2,6-dione;
3-(4-fluoro-5-((4-(6-(6-((R)-2-(3-fluorophenyl)pyrrolidin-1-yl)imidazo[1,2-b]pyridazin-3-yl)pyridin-2-yl)piperazin-1-yl)methyl)-1-oxoisoindolin-2-yl)piperidine-2,6-dione;
3-(6-fluoro-5-((4-(6-(6-((R)-2-(3-fluorophenyl)pyrrolidin-1-yl)imidazo[1,2-b]pyridazin-3-yl)pyridin-2-yl)piperazin-1-yl)methyl)-1-oxoisoindolin-2-yl)piperidine-2,6-dione;
3-(7-fluoro-5-((4-(6-(6-((R)-2-(3-fluorophenyl)pyrrolidin-1-yl)imidazo[1,2-b]pyridazin-3-yl)pyridin-2-yl)piperazin-1-yl)methyl)-1-oxoisoindolin-2-yl)piperidine-2,6-dione;
3-(5-(((1-(6-(6-((R)-2-(3-fluorophenyl)pyrrolidin-1-yl)imidazo[1,2-b]pyridazin-3-yl)pyridin-2-yl)piperidin-4-yl)(methyl)amino)methyl)-1-oxoisoindolin-2-yl)piperidine-2,6-dione;
3-(4-fluoro-5-(((1-(6-(6-((R)-2-(3-fluorophenyl)pyrrolidin-1-yl)imidazo[1,2-b]pyridazin-3-yl)pyridin-2-yl)piperidin-4-yl)(methyl)amino)methyl)-1-oxoisoindolin-2-yl)piperidine-2,6-dione;
3-(6-fluoro-5-(((1-(6-(6-((R)-2-(3-fluorophenyl)pyrrolidin-1-yl)imidazo[1,2-b]pyridazin-3-yl)pyridin-2-yl)piperidin-4-yl)(methyl)amino)methyl)-1-oxoisoindolin-2-yl)piperidine-2,6-dione;
3-(7-fluoro-5-(((1-(6-(6-((R)-2-(3-fluorophenyl)pyrrolidin-1-yl)imidazo[1,2-b]pyridazin-3-yl)pyridin-2-yl)piperidin-4-yl)(methyl)amino)methyl)-1-oxoisoindolin-2-yl)piperidine-2,6-dione;
3-(5-((4-(4-(6-(6-((R)-2-(3-fluorophenyl)pyrrolidin-1-yl)imidazo[1,2-b]pyridazin-3-yl)pyridin-2-yl)piperazin-1-yl)piperidin-1-yl)methyl)-1-oxoisoindolin-2-yl)piperidine-2,6-dione;
3-(4-fluoro-5-((4-(4-(6-(6-((R)-2-(3-fluorophenyl)pyrrolidin-1-yl)imidazo[1,2-b]pyridazin-3-yl)pyridin-2-yl)piperazin-1-yl)piperidin-1-yl)methyl)-1-oxoisoindolin-2-yl)piperidine-2,6-dione;
3-(6-fluoro-5-((4-(4-(6-(6-((R)-2-(3-fluorophenyl)pyrrolidin-1-yl)imidazo[1,2-b]pyridazin-3-yl)pyridin-2-yl)piperazin-1-yl)piperidin-1-yl)methyl)-1-oxoisoindolin-2-yl)piperidine-2,6-dione;
3-(7-fluoro-5-((4-(4-(6-(6-((R)-2-(3-fluorophenyl)pyrrolidin-1-yl)imidazo[1,2-b]pyridazin-3-yl)pyridin-2-yl)piperazin-1-yl)piperidin-1-yl)methyl)-1-oxoisoindolin-2-yl)piperidine-2,6-dione;
3-(5-((4-(6-(5-((R)-2-(2,4-difluorophenyl)pyrrolidin-1-yl)pyrazolo[1,5-a]pyrimidin-3-yl)pyridin-2-yl)piperazin-1-yl)methyl)-1-oxoisoindolin-2-yl)piperidine-2,6-dione;
3-(5-((4-(6-(5-((R)-2-(2,4-difluorophenyl)pyrrolidin-1-yl)pyrazolo[1,5-a]pyrimidin-3-yl)pyridin-2-yl)piperazin-1-yl)methyl)-4-fluoro-1-oxoisoindolin-2-yl)piperidine-2,6-dione;
3-(5-((4-(6-(5-((R)-2-(2,4-difluorophenyl)pyrrolidin-1-yl)pyrazolo[1,5-a]pyrimidin-3-yl)pyridin-2-yl)piperazin-1-yl)methyl)-6-fluoro-1-oxoisoindolin-2-yl)piperidine-2,6-dione;
3-(5-((4-(6-(5-((R)-2-(2,4-difluorophenyl)pyrrolidin-1-yl)pyrazolo[1,5-a]pyrimidin-3-yl)pyridin-2-yl)piperazin-1-yl)methyl)-7-fluoro-1-oxoisoindolin-2-yl)piperidine-2,6-dione;
3-(5-(((1-(6-(5-((R)-2-(2,4-difluorophenyl)pyrrolidin-1-yl)pyrazolo[1,5-a]pyrimidin-3-yl)pyridin-2-yl)piperidin-4-yl)(methyl)amino)methyl)-1-oxoisoindolin-2-yl)piperidine-2,6-dione;
3-(5-(((1-(6-(5-((R)-2-(2,4-difluorophenyl)pyrrolidin-1-yl)pyrazolo[1,5-a]pyrimidin-3-yl)pyridin-2-yl)piperidin-4-yl)(methyl)amino)methyl)-4-fluoro-1-oxoisoindolin-2-yl)piperidine-2,6-dione;
3-(5-(((1-(6-(5-((R)-2-(2,4-difluorophenyl)pyrrolidin-1-yl)pyrazolo[1,5-a]pyrimidin-3-yl)pyridin-2-yl)piperidin-4-yl)(methyl)amino)methyl)-6-fluoro-1-oxoisoindolin-2-yl)piperidine-2,6-dione;
3-(5-(((1-(6-(5-((R)-2-(2,4-difluorophenyl)pyrrolidin-1-yl)pyrazolo[1,5-a]pyrimidin-3-yl)pyridin-2-yl)piperidin-4-yl)(methyl)amino)methyl)-7-fluoro-1-oxoisoindolin-2-yl)piperidine-2,6-dione;
3-(5-((4-(4-(6-(5-((R)-2-(2,4-difluorophenyl)pyrrolidin-1-yl)pyrazolo[1,5-a]pyrimidin-3-yl)pyridin-2-yl)piperazin-1-yl)piperidin-1-yl)methyl)-1-oxoisoindolin-2-yl)piperidine-2,6-dione;
3-(5-((4-(4-(6-(5-((R)-2-(2,4-difluorophenyl)pyrrolidin-1-yl)pyrazolo[1,5-a]pyrimidin-3-yl)pyridin-2-yl)piperazin-1-yl)piperidin-1-yl)methyl)-4-fluoro-1-oxoisoindolin-2-yl)piperidine-2,6-dione;
3-(5-((4-(4-(6-(5-((R)-2-(2,4-difluorophenyl)pyrrolidin-1-yl)pyrazolo[1,5-a]pyrimidin-3-yl)pyridin-2-yl)piperazin-1-yl)piperidin-1-yl)methyl)-6-fluoro-1-oxoisoindolin-2-yl)piperidine-2,6-dione;
3-(5-((4-(4-(6-(5-((R)-2-(2,4-difluorophenyl)pyrrolidin-1-yl)pyrazolo[1,5-a]pyrimidin-3-yl)pyridin-2-yl)piperazin-1-yl)piperidin-1-yl)methyl)-7-fluoro-1-oxoisoindolin-2-yl)piperidine-2,6-dione;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-oxoisoindolin-5-yl)methyl)piperazin-1-yl-2,2,3,3,5,5,6,6-*d*₈)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-4-fluoro-1-oxoisoindolin-5-yl)methyl)piperazin-1-yl-2,2,3,3,5,5,6,6-*d*₈)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-6-fluoro-1-oxoisoindolin-5-yl)methyl)piperazin-1-yl-2,2,3,3,5,5,6,6-*d*₈)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-7-fluoro-1-oxoisoindolin-5-yl)methyl)piperazin-1-yl-2,2,3,3,5,5,6,6-*d*₈)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-oxoisoindolin-5-yl)methyl)-3,5-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-4-fluoro-1-oxoisoindolin-5-yl)methyl)-3,5-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-6-fluoro-1-oxoisoindolin-5-yl)methyl)-3,5-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-7-fluoro-1-oxoisoindolin-5-yl)methyl)-3,5-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-oxoisoindolin-5-yl)methyl)-2,6-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-4-fluoro-1-oxoisoindolin-5-yl)methyl)-2,6-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-6-fluoro-1-oxoisoindolin-5-yl)methyl)-2,6-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-7-fluoro-1-oxoisoindolin-5-yl)methyl)-2,6-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(6-((2-(2,6-dioxopiperidin-3-yl)-1-oxoisoindolin-5-yl)methyl)-3,6-diazabicyclo[3.1.1]heptan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(6-((2-(2,6-dioxopiperidin-3-yl)-4-fluoro-1-oxoisoindolin-5-yl)methyl)-3,6-diazabicyclo[3.1.1]heptan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(6-((2-(2,6-dioxopiperidin-3-yl)-6-fluoro-1-oxoisoindolin-5-yl)methyl)-3,6-diazabicyclo[3.1.1]heptan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(6-((2-(2,6-dioxopiperidin-3-yl)-7-fluoro-1-oxoisoindolin-5-yl)methyl)-3,6-diazabicyclo[3.1.1]heptan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-1-oxoisoindolin-5-yl)methyl)-3,6-diazabicyclo[3.1.1]heptan-6-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-4-fluoro-1-oxoisoindolin-5-yl)methyl)-3,6-diazabicyclo[3.1.1]heptan-6-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-6-fluoro-1-oxoisoindolin-5-yl)methyl)-3,6-diazabicyclo[3.1.1]heptan-6-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-7-fluoro-1-oxoisoindolin-5-yl)methyl)-3,6-diazabicyclo[3.1.1]heptan-6-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-((2-(2,6-dioxopiperidin-3-yl)-1-oxoisoindolin-5-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-((2-(2,6-dioxopiperidin-3-yl)-4-fluoro-1-oxoisoindolin-5-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-((2-(2,6-dioxopiperidin-3-yl)-6-fluoro-1-oxoisoindolin-5-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-((2-(2,6-dioxopiperidin-3-yl)-7-fluoro-1-oxoisoindolin-5-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-1-oxoisoindolin-5-yl)methyl)-3,8-diazabicyclo[3.2.1]octan-8-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-4-fluoro-1-oxoisoindolin-5-yl)methyl)-3,8-diazabicyclo[3.2.1]octan-8-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-6-fluoro-1-oxoisoindolin-5-yl)methyl)-3,8-diazabicyclo[3.2.1]octan-8-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-7-fluoro-1-oxoisoindolin-5-yl)methyl)-3,8-diazabicyclo[3.2.1]octan-8-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1R,4R)-5-((2-(2,6-dioxopiperidin-3-yl)-1-oxolsoindolin-5-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1R,4R)-5-((2-(2,6-dioxopiperidin-3-yl)-4-fluoro-1-oxolsoindolin-5-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1R,4R)-5-((2-(2,6-dioxopiperidin-3-yl)-6-fluoro-1-oxoisoindolin-5-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1R,4R)-5-((2-(2,6-dioxopiperidin-3-yl)-7-fluoro-1-oxoisoindolin-5-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1S,4S)-5-((2-(2,6-dioxopiperidin-3-yl)-1-oxoisoindolin-5-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1S,4S)-5-((2-(2,6-dioxopiperidin-3-yl)-4-fluoro-1-oxoisoindolin-5-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1S,4S)-5-((2-(2,6-dioxopiperidin-3-yl)-6-fluoro-1-oxoisoindolin-5-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1S,4S)-5-((2-(2,6-dioxopiperidin-3-yl)-7-fluoro-1-oxoisoindolin-5-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(5-((2-(2,6-dioxopiperidin-3-yl)-1-oxoisoindolin-5-yl)methyl)-2,5-diazabicyclo[2.2.2]octan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(5-((2-(2,6-dioxopiperidin-3-yl)-4-fluoro-1-oxoisoindolin-5-yl)methyl)-2,5-diazabicyclo[2.2.2]octan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(5-((2-(2,6-dioxopiperidin-3-yl)-7-fluoro-1-oxoisoindolin-5-yl)methyl)-2,5-diazabicyclo[2.2.2]octan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(5-((2-(2,6-dioxopiperidin-3-yl)-6-fluoro-1-oxoisoindolin-5-yl)methyl)-2,5-diazabicyclo[2.2.2]octan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-4-fluoro-1-oxoisoindolin-5-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-6-fluoro-1-oxoisoindolin-5-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-7-fluoro-1-oxoisoindolin-5-yl)methyl)piperazin-1-yl)-1-(phenylthio)butan-2-yl)amino)-3-((trifluoromethyl)sulfonyl)phenyl)sulfonyl)benzamide;
N-(2-(4-((2-(2,6-dioxopiperidin-3-yl)-1,3-dioxoisoindolin-5-yl)methyl)piperazin-1-yl)ethyl)-4,9-dioxo-4,9-dihydronaphtho[2,3-b]furan-2-carboxamide;
N-(2-(4-((2-(2,6-dioxopiperidin-3-yl)-4-fluoro-1,3-dioxoisoindolin-5-yl)methyl)piperazin-1-yl)ethyl)-4,9-dioxo-4,9-dihydronaphtho[2,3-b]furan-2-carboxamide;
N-(2-(4-((2-(2,6-dioxopiperidin-3-yl)-6-fluoro-1,3-dioxoisoindolin-5-yl)methyl)piperazin-1-yl)ethyl)-4,9-dioxo-4,9-dihydronaphtho[2,3-b]furan-2-carboxamide;
N-(2-(4-((2-(2,6-dioxopiperidin-3-yl)-7-fluoro-1,3-dioxoisoindolin-5-yl)methyl)piperazin-1-yl)ethyl)-4,9-dioxo-4,9-dihydronaphtho[2,3-b]furan-2-carboxamide;
N-((2-(2,6-dioxopiperidin-3-yl)-1,3-dioxoisoindolin-5-yl)methyl)-4,9-dioxo-4,9-dihydronaphtho[2,3 -b]furan-2-carboxamide;
N-((2-(2,6-dioxopiperidin-3-yl)-4-fluoro-1,3-dioxoisoindolin-5-yl)methyl)-4,9-dioxo-4,9-dihydronaphtho[2,3 -b]furan-2-carboxamide;
N-((2-(2,6-dioxopiperidin-3-yl)-6-fluoro-1,3-dioxoisoindolin-5-yl)methyl)-4,9-dioxo-4,9-dihydronaphtho[2,3 -b]furan-2-carboxamide;
N-((2-(2,6-dioxopiperidin-3-yl)-7-fluoro-1,3-dioxoisoindolin-5-yl)methyl)-4,9-dioxo-4,9-dihydronaphtho[2,3 -b]furan-2-carboxamide;
((2-(((5S,8S,10aR)-8-(((2S)-5-amino-1-(2-chloro-3-((2-(2,6-dioxopiperidin-3-yl)-1,3-dioxoisoindolin-5-yl)methyl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(2-chloro-3-((2-(2,6-dioxopiperidin-3-yl)-4-fluoro-1,3-dioxoisoindolin-5-yl)methyl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(2-chloro-3-((2-(2,6-dioxopiperidin-3-yl)-6-fluoro-1,3-dioxoisoindolin-5-yl)methyl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(2-chloro-3-((2-(2,6-dioxopiperidin-3-yl)-7-fluoro-1,3-dioxoisoindolin-5-yl)methyl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(2-chloro-3-(4-((2-(2,6-dioxopiperidin-3-yl)-1,3-dioxoisoindolin-5-yl)methyl)piperazin-1-yl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5 S, 8 S, lOaR)-8-(((2S)-5-amino-1-(2-chloro-3-(4-((2-(2,6-dioxopiperidin-3-yl)-4-fluoro-1,3-dioxoisoindolin-5-yl)methyl)piperazin-1-yl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5 S, 8 S, lOaR)-8-(((2S)-5-amino-1-(2-chloro-3-(4-((2-(2,6-dioxopiperidin-3-yl)-6-fluoro-1,3-dioxoisoindolin-5-yl)methyl)piperazin-1-yl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5 S, 8 S, lOaR)-8-(((2S)-5-amino-1-(2-chloro-3-(4-((2-(2,6-dioxopiperidin-3-yl)-7-fluoro-1,3-dioxoisoindolin-5-yl)methyl)piperazin-1-yl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((1-((2-(2,6-dioxopiperidin-3-yl)-1,3-dioxoisoindolin-5-yl)methyl)piperidin-4-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((1-((2-(2,6-dioxopiperidin-3-yl)-4-fluoro-1,3-dioxoisoindolin-5-yl)methyl)piperidin-4-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((1-((2-(2,6-dioxopiperidin-3-yl)-6-fluoro-1,3-dioxoisoindolin-5-yl)methyl)piperidin-4-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((1-((2-(2,6-dioxopiperidin-3-yl)-7-fluoro-1,3-dioxoisoindolin-5-yl)methyl)piperidin-4-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((2-(2,6-dioxopiperidin-3-yl)-1,3-dioxoisoindolin-5-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((2-(2,6-dioxopiperidin-3-yl)-4-fluoro-1,3-dioxoisoindolin-5-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((2-(2,6-dioxopiperidin-3-yl)-6-fluoro-1,3-dioxoisoindolin-5-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((2-(2,6-dioxopiperidin-3-yl)-7-fluoro-1,3-dioxoisoindolin-5-yl)methyl)-6-oxodecahydropyrrolo[1,2-a][1,5]diazocin-5-yl)carbamoyl)-1H-indol-5-yl)difluoromethyl)phosphonic acid;
6-(1-((2-(2,6-dioxopiperidin-3-yl)-1,3-dioxoisoindolin-5-yl)methyl)piperidin-4-yl)-2-(4-phenoxyphenyl)nicotinamide;
6-(1-((2-(2,6-dioxopiperidin-3-yl)-4-fluoro-1,3-dioxoisoindolin-5-yl)methyl)piperidin-4-yl)-2-(4-phenoxyphenyl)nicotinamide;
6-(1-((2-(2,6-dioxopiperidin-3-yl)-6-fluoro-1,3-dioxoisoindolin-5-yl)methyl)piperidin-4-yl)-2-(4-phenoxyphenyl)nicotinamide;
6-(1-((2-(2,6-dioxopiperidin-3-yl)-7-fluoro-1,3-dioxoisoindolin-5-yl)methyl)piperidin-4-yl)-2-(4-phenoxyphenyl)nicotinamide;
6-(4-((2-(2,6-dioxopiperidin-3-yl)-1,3-dioxoisoindolin-5-yl)methyl)piperazin-1-yl)-2-(4-phenoxyphenyl)nicotinamide;
6-(4-((2-(2,6-dioxopiperidin-3-yl)-4-fluoro-1,3-dioxoisoindolin-5-yl)methyl)piperazin-1-yl)-2-(4-phenoxyphenyl)nicotinamide;
6-(4-((2-(2,6-dioxopiperidin-3-yl)-6-fluoro-1,3-dioxoisoindolin-5-yl)methyl)piperazin-1-yl)-2-(4-phenoxyphenyl)nicotinamide;
6-(4-((2-(2,6-dioxopiperidin-3-yl)-7-fluoro-1,3-dioxoisoindolin-5-yl)methyl)piperazin-1-yl)-2-(4-phenoxyphenyl)nicotinamide;
6-(4-(((2-(2,6-dioxopiperidin-3-yl)-1,3-dioxoisoindolin-5-yl)methyl)(methyl)amino)piperidin-1-yl)-2-(4-phenoxyphenyl)nicotinamide;
6-(4-(((2-(2,6-dioxopiperidin-3-yl)-4-fluoro-1,3-dioxoisoindolin-5-yl)methyl)(methyl)amino)piperidin-1-yl)-2-(4-phenoxyphenyl)nicotinamide;
6-(4-(((2-(2,6-dioxopiperidin-3-yl)-6-fluoro-1,3-dioxoisoindolin-5-yl)methyl)(methyl)amino)piperidin-1-yl)-2-(4-phenoxyphenyl)nicotinamide;
6-(4-(((2-(2,6-dioxopiperidin-3-yl)-7-fluoro-1,3-dioxoisoindolin-5-yl)methyl)(methyl)amino)piperidin-1-yl)-2-(4-phenoxyphenyl)nicotinamide;
6-(4-(4-((2-(2,6-dioxopiperidin-3-yl)-1,3-dioxoisoindolin-5-yl)methyl)piperazin-1-yl)piperidin-1-yl)-2-(4-phenoxyphenyl)nicotinamide;
6-(4-(4-((2-(2,6-dioxopiperidin-3-yl)-4-fluoro-1,3-dioxoisoindolin-5-yl)methyl)piperazin-1-yl)piperidin-1-yl)-2-(4-phenoxyphenyl)nicotinamide;
6-(4-(4-((2-(2,6-dioxopiperidin-3-yl)-6-fluoro-1,3-dioxoisoindolin-5-yl)methyl)piperazin-1-yl)piperidin-1-yl)-2-(4-phenoxyphenyl)nicotinamide;
6-(4-(4-((2-(2,6-dioxopiperidin-3-yl)-7-fluoro-1,3-dioxoisoindolin-5-yl)methyl)piperazin-1-yl)piperidin-1-yl)-2-(4-phenoxyphenyl)nicotinamide;
6-(4-(1-((2-(2,6-dioxopiperidin-3-yl)-1,3-dioxoisoindolin-5-yl)methyl)piperidin-4-yl)piperazin-1-yl)-2-(4-phenoxyphenyl)nicotinamide;
6-(4-(1-((2-(2,6-dioxopiperidin-3-yl)-4-fluoro-1,3-dioxoisoindolin-5-yl)methyl)piperidin-4-yl)piperazin-1-yl)-2-(4-phenoxyphenyl)nicotinamide;
6-(4-(1-((2-(2,6-dioxopiperidin-3-yl)-6-fluoro-1,3-dioxoisoindolin-5-yl)methyl)piperidin-4-yl)piperazin-1-yl)-2-(4-phenoxyphenyl)nicotinamide;
6-(4-(1-((2-(2,6-dioxopiperidin-3-yl)-7-fluoro-1,3-dioxoisoindolin-5-yl)methyl)piperidin-4-yl)piperazin-1-yl)-2-(4-phenoxyphenyl)nicotinamide;
3-(tert-butyl)-N-(4-(5-(4-(4-((2-(2,6-dioxopiperidin-3-yl)-1,3-dioxoisoindolin-5-yl)methyl)piperazin-1-yl)phenyl)-1H-indazol-3-yl)-2-methylbenzyl)-1,2,4-oxadiazole-5-carboxamide;
3-(tert-butyl)-N-(4-(5-(4-(4-((2-(2,6-dioxopiperidin-3-yl)-4-fluoro-1,3-dioxoisoindolin-5-yl)methyl)piperazin-1-yl)phenyl)-1H-indazol-3-yl)-2-methylbenzyl)-1,2,4-oxadiazole-5-carboxamide;
3-(tert-butyl)-N-(4-(5-(4-(4-((2-(2,6-dioxopiperidin-3-yl)-6-fluoro-1,3-dioxoisoindolin-5-yl)methyl)piperazin-1-yl)phenyl)-1H-indazol-3-yl)-2-methylbenzyl)-1,2,4-oxadiazole-5-carboxamide;
3-(tert-butyl)-N-(4-(5-(4-(4-((2-(2,6-dioxopiperidin-3-yl)-7-fluoro-1,3-dioxoisoindolin-5-yl)methyl)piperazin-1-yl)phenyl)-1H-indazol-3-yl)-2-methylbenzyl)-1,2,4-oxadiazole-5-carboxamide;
3-(tert-butyl)-N-(4-(5-(4-(4-(((2-(2,6-dioxopiperidin-3-yl)-1,3-dioxoisoindolin-5-yl)methyl)(methyl)amino)piperidin-1-yl)phenyl)-1H-indazol-3-yl)-2-methylbenzyl)-1,2,4-oxadiazole-5-carboxamide;
3-(tert-butyl)-N-(4-(5-(4-(4-(((2-(2,6-dioxopiperidin-3-yl)-4-fluoro-1,3-dioxoisoindolin-5-yl)methyl)(methyl)amino)piperidin-1-yl)phenyl)-1H-indazol-3-yl)-2-methylbenzyl)-1,2,4-oxadiazole-5-carboxamide;
3-(tert-butyl)-N-(4-(5-(4-(4-(((2-(2,6-dioxopiperidin-3-yl)-6-fluoro-1,3-dioxoisoindolin-5-yl)methyl)(methyl)amino)piperidin-1-yl)phenyl)-1H-indazol-3-yl)-2-methylbenzyl)-1,2,4-oxadiazole-5-carboxamide;
3-(tert-butyl)-N-(4-(5-(4-(4-(((2-(2,6-dioxopiperidin-3-yl)-7-fluoro-1,3-dioxoisoindolin-5-yl)methyl)(methyl)amino)piperidin-1-yl)phenyl)-1H-indazol-3-yl)-2-methylbenzyl)-1,2,4-oxadiazole-5-carboxamide;
3-(tert-butyl)-N-(4-(5-(4-(4-(4-((2-(2,6-dioxopiperidin-3-yl)-1,3-dioxoisoindolin-5-yl)methyl)piperazin-1-yl)piperidin-1-yl)phenyl)-1H-indazol-3-yl)-2-methylbenzyl)-1,2,4-oxadiazole-5-carboxamide;
3-(tert-butyl)-N-(4-(5-(4-(4-(4-((2-(2,6-dioxopiperidin-3-yl)-4-fluoro-1,3-dioxoisoindolin-5-yl)methyl)piperazin-1 -yl)piperidin-1 -yl)phenyl)-1 H-indazol-3 -yl)-2-methylbenzyl)-1,2,4-oxadiazole-5-carboxamide;
3-(tert-butyl)-N-(4-(5-(4-(4-(4-((2-(2,6-dioxopiperidin-3-yl)-6-fluoro-1,3-dioxoisoindolin-5-yl)methyl)piperazin-1-yl)piperidin-1-yl)phenyl)-1H-indazol-3-yl)-2-methylbenzyl)-1,2,4-oxadiazole-5-carboxamide;
3-(tert-butyl)-N-(4-(5-(4-(4-(4-((2-(2,6-dioxopiperidin-3-yl)-7-fluoro-1,3-dioxoisoindolin-5-yl)methyl)piperazin-1-yl)piperidin-1-yl)phenyl)-1H-indazol-3-yl)-2-methylbenzyl)-1,2,4-oxadiazole-5-carboxamide;
3-(tert-butyl)-N-(4-(5-(4-(4-(1-((2-(2,6-dioxopiperidin-3-yl)-1,3-dioxoisoindolin-5-yl)methyl)piperidin-4-yl)piperazin-1-yl)phenyl)-1H-indazol-3-yl)-2-methylbenzyl)-1,2,4-oxadiazole-5-carboxamide;
3-(tert-butyl)-N-(4-(5-(4-(4-(1-((2-(2,6-dioxopiperidin-3-yl)-4-fluoro-1,3-dioxoisoindolin-5-yl)methyl)piperidin-4-yl)piperazin-1-yl)phenyl)-1H-indazol-3-yl)-2-methylbenzyl)-1,2,4-oxadiazole-5-carboxamide;
3-(tert-butyl)-N-(4-(5-(4-(4-(1-((2-(2,6-dioxopiperidin-3-yl)-6-fluoro-1,3-dioxoisoindolin-5-yl)methyl)piperidin-4-yl)piperazin-1-yl)phenyl)-1H-indazol-3-yl)-2-methylbenzyl)-1,2,4-oxadiazole-5-carboxamide;
3-(tert-butyl)-N-(4-(5-(4-(4-(1-((2-(2,6-dioxopiperidin-3-yl)-7-fluoro-1,3-dioxoisoindolin-5-yl)methyl)piperidin-4-yl)piperazin-1-yl)phenyl)-1H-indazol-3-yl)-2-methylbenzyl)-1,2,4-oxadiazole-5-carboxamide;
5-((4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)methyl)-2-(2,6-dioxopiperidin-3-yl)isoindoline-1,3-dione;
5-((4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)methyl)-2-(2,6-dioxopiperidin-3 -yl)-4-fluoroisoindoline-1,3 -dione;
5-((4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3 -yl)piperazin-1-yl)methyl)-2-(2,6-dioxopiperidin-3 -yl)-6-fluoroisoindoline-1,3 -dione;
6-((4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3 -yl)piperazin-1-yl)methyl)-2-(2,6-dioxopiperidin-3 -yl)-4-fluoroisoindoline-1,3 -dione;
5-((3-(4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3 -yl)piperazin-1 -yl)azetidin-1 -yl)methyl)-2-(2,6-dioxopiperi din-3 -yl)isoindoline-1,3 -dione;
5-((3-(4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)azetidin-1-yl)methyl)-2-(2,6-dioxopiperidin-3-yl)-4-fluoroisoindoline-1,3-dione;
5-((3-(4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)azetidin-1-yl)methyl)-2-(2,6-dioxopiperidin-3-yl)-6-fluoroisoindoline-1,3-dione;
6-((3-(4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)azetidin-1-yl)methyl)-2-(2,6-dioxopiperidin-3-yl)-4-fluoroisoindoline-1,3-dione;
5-(((1-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperidin-4-yl)(methyl)amino)methyl)-2-(2,6-dioxopiperidin-3-yl)isoindoline-1,3-dione;
5-(((1-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperidin-4-yl)(methyl)amino)methyl)-2-(2,6-dioxopiperidin-3-yl)-4-fluoroisoindoline-1,3-dione;
5-(((1-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperidin-4-yl)(methyl)amino)methyl)-2-(2,6-dioxopiperidin-3-yl)-6-fluoroisoindoline-1,3-dione;
6-(((1-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperidin-4-yl)(methyl)amino)methyl)-2-(2,6-dioxopiperidin-3-yl)-4-fluoroisoindoline-1,3-dione;
5-((4-(1-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperidin-4-yl)piperazin-1-yl)methyl)-2-(2,6-dioxopiperidin-3-yl)isoindoline-1,3-dione;
5-((4-(1-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3 -yl)pip eridin-4-yl)pip erazin-1-yl)methyl)-2-(2, 6-dioxopip eri din-3 -yl)-4-fl uoroisoindoline-1, 3 -dione;
5-((4-(1-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3 -yl)pip eridin-4-yl)pip erazin-1-yl)methyl)-2-(2, 6-dioxopip eri din-3 -yl)-6-fl uoroisoindoline-1, 3 -dione;
6-((4-(1-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3 -yl)pip eridin-4-yl)pip erazin-1-yl)methyl)-2-(2, 6-dioxopip eri din-3 -yl)-4-fl uoroisoindoline-1, 3 -dione;
5-((4-(4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)piperidin-1-yl)methyl)-2-(2,6-dioxopiperidin-3-yl)isoindoline-1,3-dione;
5-((4-(4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)piperidin-1-yl)methyl)-2-(2,6-dioxopiperidin-3-yl)-4-fluoroisoindoline-1,3-dione;
5-((4-(4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3 -yl)pip erazin-1-yl)pip eridin-1-yl)methyl)-2-(2, 6-dioxopip eridin-3 -yl)-6-fluoroi soindoline-1, 3 -dione;
6-((4-(4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)piperidin-1-yl)methyl)-2-(2,6-dioxopiperidin-3-yl)-4-fluoroisoindoline-1,3-dione;
(S)-5-((4-(4-amino-3-(4-phenoxyphenyl)-1H-pyrazolo[3,4-d]pyrimidin-1-yl)piperidin-1-yl)methyl)-2-(2,6-dioxopiperidin-3-yl)isoindoline-1,3-dione;
(S)-5-((4-(4-amino-3-(4-phenoxyphenyl)-1H-pyrazolo[3,4-d]pyrimidin-1-yl)piperidin-1-yl)methyl)-2-(2,6-dioxopiperidin-3-yl)-4-fluoroisoindoline-1,3-dione;
(S)-5-((4-(4-amino-3-(4-phenoxyphenyl)-1H-pyrazolo[3,4-d]pyrimidin-1-yl)piperidin-1-yl)methyl)-2-(2,6-dioxopiperidin-3-yl)-6-fluoroisoindoline-1,3-dione;
(S)-6-((4-(4-amino-3-(4-phenoxyphenyl)-1H-pyrazolo [3,4-d]pyrimidin-1 -yl)piperidin-1 - yl)methyl)-2-(2,6-dioxopiperidin-3-yl)-4-fluoroisoindoline-1,3-dione;
5-(((3R,4S)-4-(4-amino-3-(4-phenoxyphenyl)-1H-pyrazolo[3,4-d]pyrimidin-1-yl)-3-fluoropiperidin-1-yl)methyl)-2-(2,6-dioxopiperidin-3-yl)isoindoline-1,3-dione;
5-(((3R,4S)-4-(4-amino-3-(4-phenoxyphenyl)-1H-pyrazolo[3,4-d]pyrimidin-1-yl)-3-fluoropiperidin-1-yl)methyl)-2-(2,6-dioxopiperidin-3-yl)-4-fluoroisoindoline-1,3-dione;
5-(((3R,4S)-4-(4-amino-3-(4-phenoxyphenyl)-1H-pyrazolo[3,4-d]pyrimidin-1-yl)-3-fluoropiperidin-1-yl)methyl)-2-(2,6-dioxopiperidin-3-yl)-6-fluoroisoindoline-1,3-dione;
6-(((3R,4S)-4-(4-amino-3 -(4-phenoxyphenyl)-1H-pyrazolo [3,4-d]pyrimidin-1 -yl)-3 - fluoropiperidin-1-yl)methyl)-2-(2,6-dioxopiperidin-3-yl)-4-fluoroisoindoline-1,3-dione;
5-(((3S,4R)-4-(4-amino-3-(4-phenoxyphenyl)-1H-pyrazolo[3,4-d]pyrimidin-1-yl)-3-fluoropiperidin-1-yl)methyl)-2-(2,6-dioxopiperidin-3-yl)isoindoline-1,3-dione;
5-(((3S,4R)-4-(4-amino-3-(4-phenoxyphenyl)-1H-pyrazolo[3,4-d]pyrimidin-1-yl)-3-fluoropiperidin-1-yl)methyl)-2-(2,6-dioxopiperidin-3-yl)-4-fluoroisoindoline-1,3-dione;
5-(((3S,4R)-4-(4-amino-3-(4-phenoxyphenyl)-1H-pyrazolo[3,4-d]pyrimidin-1-yl)-3-fluoropiperidin-1-yl)methyl)-2-(2,6-dioxopiperidin-3-yl)-6-fluoroisoindoline-1,3-dione;
6-(((3S,4R)-4-(4-amino-3-(4-phenoxyphenyl)-1H-pyrazolo[3,4-d]pyrimidin-1-yl)-3-fluoropiperidin-1-yl)methyl)-2-(2,6-dioxopiperidin-3-yl)-4-fluoroisoindoline-1,3-dione;
5-(((3R,4R)-4-(4-amino-3-(4-phenoxyphenyl)-1H-pyrazolo[3,4-d]pyrimidin-1-yl)-3-fluoropiperidin-1-yl)methyl)-2-(2,6-dioxopiperidin-3-yl)isoindoline-1,3-dione;
5-(((3R,4R)-4-(4-amino-3-(4-phenoxyphenyl)-1H-pyrazolo[3,4-d]pyrimidin-1-yl)-3-fluoropiperidin-1-yl)methyl)-2-(2,6-dioxopiperidin-3-yl)-4-fluoroisoindoline-1,3-dione;
5-(((3R,4R)-4-(4-amino-3-(4-phenoxyphenyl)-1H-pyrazolo[3,4-d]pyrimidin-1-yl)-3-fluoropiperidin-1-yl)methyl)-2-(2,6-dioxopiperidin-3-yl)-6-fluoroisoindoline-1,3-dione;
6-(((3R,4R)-4-(4-amino-3-(4-phenoxyphenyl)-1H-pyrazolo[3,4-d]pyrimidin-1-yl)-3-fluoropiperidin-1-yl)methyl)-2-(2,6-dioxopiperidin-3-yl)-4-fluoroisoindoline-1,3-dione;
5-(((3S,4S)-4-(4-amino-3-(4-phenoxyphenyl)-1H-pyrazolo[3,4-d]pyrimidin-1-yl)-3-fluoropiperidin-1-yl)methyl)-2-(2,6-dioxopiperidin-3-yl)isoindoline-1,3-dione;
5-(((3S,4S)-4-(4-amino-3-(4-phenoxyphenyl)-1H-pyrazolo[3,4-d]pyrimidin-1-yl)-3-fluoropiperidin-1-yl)methyl)-2-(2,6-dioxopiperidin-3-yl)-4-fluoroisoindoline-1,3-dione;
5-(((3S,4S)-4-(4-amino-3-(4-phenoxyphenyl)-1H-pyrazolo[3,4-d]pyrimidin-1-yl)-3-fluoropiperidin-1-yl)methyl)-2-(2,6-dioxopiperidin-3-yl)-6-fluoroisoindoline-1,3-dione;
6-(((3S,4S)-4-(4-amino-3-(4-phenoxyphenyl)-1H-pyrazolo[3,4-d]pyrimidin-1-yl)-3-fluoropiperidin-1-yl)methyl)-2-(2,6-dioxopiperidin-3-yl)-4-fluoroisoindoline-1,3-dione;
5-(((R)-3-(4-amino-3-(4-phenoxyphenyl)-1H-pyrazolo[3,4-d]pyrimidin-1-yl)piperidin-1-yl)methyl)-2-(2,6-dioxopiperidin-3-yl)isoindoline-1,3-dione;
5-(((R)-3-(4-amino-3-(4-phenoxyphenyl)-1H-pyrazolo[3,4-d]pyrimidin-1-yl)piperidin-1-yl)methyl)-2-(2,6-dioxopiperidin-3-yl)-4-fluoroisoindoline-1,3-dione;
5-(((R)-3-(4-amino-3-(4-phenoxyphenyl)-1H-pyrazolo[3,4-d]pyrimidin-1-yl)piperidin-1-yl)methyl)-2-(2,6-dioxopiperidin-3-yl)-6-fluoroisoindoline-1,3-dione;
6-(((R)-3-(4-amino-3-(4-phenoxyphenyl)-1H-pyrazolo[3,4-d]pyrimidin-1-yl)piperidin-1-yl)methyl)-2-(2,6-dioxopiperidin-3-yl)-4-fluoroisoindoline-1,3-dione;
5-((4-(4-amino-3-(4-phenoxyphenyl)-1H-pyrazolo[3,4-d]pyrimidin-1-yl)piperidin-1-yl)methyl)-2-(2,6-dioxopiperidin-3-yl)isoindoline-1,3-dione;
5-((4-(4-amino-3-(4-phenoxyphenyl)-1H-pyrazolo[3,4-d]pyrimidin-1-yl)piperidin-1-yl)methyl)-2-(2,6-dioxopiperidin-3-yl)-4-fluoroisoindoline-1,3-dione;
5-((4-(4-amino-3-(4-phenoxyphenyl)-1H-pyrazolo[3,4-d]pyrimidin-1-yl)piperidin-1-yl)methyl)-2-(2,6-dioxopiperidin-3-yl)-6-fluoroisoindoline-1,3-dione;
6-((4-(4-amino-3-(4-phenoxyphenyl)-1H-pyrazolo[3,4-d]pyrimidin-1-yl)piperidin-1-yl)methyl)-2-(2,6-dioxopiperidin-3-yl)-4-fluoroisoindoline-1,3-dione;
5-((4-(4-amino-3-(4-phenoxyphenyl)-1H-pyrazolo[3,4-d]pyrimidin-1-yl)-3-fluoropiperidin-1-yl)methyl)-2-(2,6-dioxopiperidin-3-yl)isoindoline-1,3-dione;
5-((4-(4-amino-3-(4-phenoxyphenyl)-1H-pyrazolo[3,4-d]pyrimidin-1-yl)-3-fluoropiperidin-1-yl)methyl)-2-(2,6-dioxopiperidin-3-yl)-4-fluoroisoindoline-1,3-dione;
5-((4-(4-amino-3-(4-phenoxyphenyl)-1H-pyrazolo[3,4-d]pyrimidin-1-yl)-3-fluoropiperidin-1-yl)methyl)-2-(2,6-dioxopiperidin-3-yl)-6-fluoroisoindoline-1,3-dione;
6-((4-(4-amino-3-(4-phenoxyphenyl)-1H-pyrazolo[3,4-d]pyrimidin-1-yl)-3-fluoropiperidin-1-yl)methyl)-2-(2,6-dioxopiperidin-3-yl)-4-fluoroisoindoline-1,3-dione;
5-((4-(4-(1,2-bis(4-hydroxyphenyl)but-1-en-1-yl)phenyl)piperazin-1-yl)methyl)-2-(2,6-dioxopiperidin-3 -yl)isoindoline-1,3 -dione;
5-((4-(4-(1,2-bis(4-hydroxyphenyl)but-1-en-1-yl)phenyl)piperazin-1-yl)methyl)-2-(2,6-dioxopiperidin-3-yl)-4-fluoroisoindoline-1,3-dione;
5-((4-(4-(1,2-bis(4-hydroxyphenyl)but-1-en-1-yl)phenyl)piperazin-1-yl)methyl)-2-(2,6-dioxopiperidin-3-yl)-6-fluoroisoindoline-1,3-dione;
6-((4-(4-(1,2-bis(4-hydroxyphenyl)but-1-en-1-yl)phenyl)piperazin-1-yl)methyl)-2-(2,6-dioxopiperidin-3-yl)-4-fluoroisoindoline-1,3-dione;
2-(2,6-dioxopiperidin-3-yl)-5-((4-(4-(1-(4-hydroxyyphenyl)-2-phenylbut-1-en-1-yl)phenyl)piperazin-1-yl)methyl)isoindoline-1,3-dione;
2-(2,6-dioxopiperidin-3-yl)-4-fluoro-5-((4-(4-(1-(4-hydroxyphenyl)-2-phenylbut-1-en-1-yl)phenyl)piperazin-1-yl)methyl)isoindoline-1,3-dione;
2-(2,6-dioxopiperidin-3-yl)-5-fluoro-6-((4-(4-(1-(4-hydroxyphenyl)-2-phenylbut-1-en-1-yl)phenyl)piperazin-1-yl)methyl)isoindoline-1,3-dione;
2-(2,6-dioxopiperidin-3-yl)-4-fluoro-6-((4-(4-(1-(4-hydroxyphenyl)-2-phenylbut-1-en-1-yl)phenyl)piperazin-1-yl)methyl)isoindoline-1,3-dione;
5-((4-(4-(4-chloro-1,2-bis(4-hydroxyphenyl)but-1-en-1-yl)phenyl)piperazin-1-yl)methyl)-2-(2,6-dioxopiperidin-3 -yl)isoindoline-1,3 -dione;
5-((4-(4-(4-chloro-1,2-bis(4-hydroxyphenyl)but-1-en-1-yl)phenyl)piperazin-1-yl)methyl)-2-(2,6-dioxopiperidin-3-yl)-4-fluoroisoindoline-1,3-dione;
5-((4-(4-(4-chloro-1,2-bis(4-hydroxyphenyl)but-1-en-1-yl)phenyl)piperazin-1-yl)methyl)-2-(2,6-dioxopiperidin-3-yl)-6-fluoroisoindoline-1,3-dione;
6-((4-(4-(4-chloro-1,2-bis(4-hydroxyphenyl)but-1-en-1-yl)phenyl)piperazin-1-yl)methyl)-2-(2,6-dioxopiperidin-3-yl)-4-fluoroisoindoline-1,3-dione;
5-((4-(4-(4-chloro-1-(4-hydroxyphenyl)-2-phenylbut-1-en-1-yl)phenyl)piperazin-1-yl)methyl)-2-(2,6-dioxopiperidin-3-yl)isoindoline-1,3-dione;
5-((4-(4-(4-chloro-1-(4-hydroxyphenyl)-2-phenylbut-1-en-1-yl)phenyl)piperazin-1-yl)methyl)-2-(2,6-dioxopiperidin-3-yl)-4-fluoroisoindoline-1,3-dione;
5-((4-(4-(4-chloro-1-(4-hydroxyphenyl)-2-phenylbut-1-en-1-yl)phenyl)piperazin-1-yl)methyl)-2-(2,6-dioxopiperidin-3-yl)-6-fluoroisoindoline-1,3-dione;
6-((4-(4-(4-chloro-1-(4-hydroxyphenyl)-2-phenylbut-1-en-1-yl)phenyl)piperazin-1-yl)methyl)-2-(2,6-dioxopiperidin-3-yl)-4-fluoroisoindoline-1,3-dione;
5-(((2-(4-(4-chloro-1,2-bis(4-hydroxyphenyl)but-1-en-1-yl)phenoxy)ethyl)(methyl)amino)methyl)-2-(2,6-dioxopiperidin-3-yl)isoindoline-1,3-dione;
5-(((2-(4-(4-chloro-1,2-bis(4-hydroxyphenyl)but-1-en-1-yl)phenoxy)ethyl)(methyl)amino)methyl)-2-(2,6-dioxopiperidin-3-yl)-4-fluoroisoindoline-1,3-dione;
5-(((2-(4-(4-chloro-1,2-bis(4-hydroxyphenyl)but-1-en-1-yl)phenoxy)ethyl)(methyl)amino)methyl)-2-(2,6-dioxopiperidin-3-yl)-6-fluoroisoindoline-1,3-dione;
6-(((2-(4-(4-chloro-1,2-bis(4-hydroxyphenyl)but-1-en-1-yl)phenoxy)ethyl)(methyl)amino)methyl)-2-(2,6-dioxopiperidin-3-yl)-4-fluoroisoindoline-1,3-dione;
5-(((2-(4-(4-chloro-l,2-bis(4-hydroxyphenyl)but-1-en-1-yl)phenoxy)ethyl)amino)methyl)-2-(2,6-dioxopiperidin-3-yl)isoindoline-1,3-dione;
5-(((2-(4-(4-chloro-1,2-bis(4-hydroxyphenyl)but-1-en-1-yl)phenoxy)ethyl)amino)methyl)-2-(2,6-dioxopiperidin-3-yl)-4-fluoroisoindoline-1,3-dione;
5-(((2-(4-(4-chloro-1,2-bis(4-hydroxyphenyl)but-1-en-1-yl)phenoxy)ethyl)amino)methyl)-2-(2,6-dioxopiperidin-3-yl)-6-fluoroisoindoline-1,3-dione;
6-(((2-(4-(4-chloro-1,2-bis(4-hydroxyphenyl)but-1-en-1-yl)phenoxy)ethyl)amino)methyl)-2-(2,6-dioxopiperidin-3-yl)-4-fluoroisoindoline-1,3-dione;
2-(2,6-dioxopiperidin-3-yl)-5-((4-(4-((1R,2S)-6-hydroxy-2-phenyl-1,2,3,4-tetrahydronaphthalen-1-yl)phenyl)piperazin-1-yl)methyl)isoindoline-1,3-dione;
2-(2,6-dioxopiperidin-3-yl)-4-fluoro-5-((4-(4-((1R,2S)-6-hydroxy-2-phenyl-1,2,3,4-tetrahydronaphthalen-1-yl)phenyl)piperazin-1-yl)methyl)isoindoline-1,3-dione;
2-(2,6-dioxopiperidin-3-yl)-5-fluoro-6-((4-(4-((1R,2S)-6-hydroxy-2-phenyl-1,2,3,4-tetrahydronaphthalen-1-yl)phenyl)piperazin-1-yl)methyl)isoindoline-1,3-dione;
2-(2,6-dioxopiperidin-3-yl)-4-fluoro-6-((4-(4-((1R,2S)-6-hydroxy-2-phenyl-1,2,3,4-tetrahydronaphthalen-1-yl)phenyl)piperazin-1-yl)methyl)isoindoline-1,3-dione;
2-(2,6-dioxopiperidin-3-yl)-5-((4-(4-((3S,4R)-7-hydroxy-3-phenylchroman-4-yl)phenyl)piperazin-1-yl)methyl)isoindoline-1,3-dione;
2-(2,6-dioxopiperidin-3-yl)-4-fluoro-5-((4-(4-((3S,4R)-7-hydroxy-3-phenylchroman-4-yl)phenyl)piperazin-1-yl)methyl)isoindoline-1,3-dione;
2-(2,6-dioxopiperidin-3-yl)-5-fluoro-6-((4-(4-((3S,4R)-7-hydroxy-3-phenylchroman-4-yl)phenyl)piperazin-1-yl)methyl)isoindoline-1,3-dione;
2-(2,6-dioxopiperidin-3-yl)-4-fluoro-6-((4-(4-((3S,4R)-7-hydroxy-3-phenyl chroman-4-yl)phenyl)piperazin-1-yl)methyl)isoindoline-1,3-dione;
2-(2,6-dioxopiperidin-3-yl)-5-(((1-(4-((3S,4R)-7-hydroxy-3-phenylchroman-4-yl)phenyl)piperidin-4-yl)(methyl)amino)methyl)isoindoline-1,3-dione;
2-(2,6-dioxopiperidin-3-yl)-4-fluoro-5-(((1-(4-((3S,4R)-7-hydroxy-3-phenylchroman-4-yl)phenyl)piperidin-4-yl)(methyl)amino)methyl)isoindoline-1,3-dione;
2-(2,6-dioxopiperidin-3-yl)-5-fluoro-6-(((1-(4-((3S,4R)-7-hydroxy-3-phenylchroman-4-yl)phenyl)piperidin-4-yl)(methyl)amino)methyl)isoindoline-1,3-dione;
2-(2,6-dioxopiperidin-3-yl)-4-fluoro-6-(((1-(4-((3S,4R)-7-hydroxy-3-phenylchroman-4-yl)phenyl)piperidin-4-yl)(methyl)amino)methyl)isoindoline-1,3-dione;
2-(2,6-dioxopiperidin-3-yl)-5-((4-(1-(4-((3S,4R)-7-hydroxy-3-phenylchroman-4-yl)phenyl)piperidin-4-yl)piperazin-1-yl)methyl)isoindoline-1,3-dione;
2-(2,6-dioxopiperidin-3-yl)-4-fluoro-5-((4-(1-(4-((3S,4R)-7-hydroxy-3-phenylchroman-4-yl)phenyl)piperidin-4-yl)piperazin-1-yl)methyl)isoindoline-1,3-dione;
2-(2,6-dioxopiperidin-3-yl)-5-fluoro-6-((4-(1-(4-((3S,4R)-7-hydroxy-3-phenylchroman-4-yl)phenyl)piperidin-4-yl)piperazin-1-yl)methyl)isoindoline-1,3-dione;
2-(2,6-dioxopiperidin-3-yl)-4-fluoro-6-((4-(1-(4-((3S,4R)-7-hydroxy-3-phenylchroman-4-yl)phenyl)piperidin-4-yl)piperazin-1-yl)methyl)isoindoline-1,3-dione;
2-(2,6-dioxopiperidin-3-yl)-5-(((1-(4-((1R,2S)-6-hydroxy-2-phenyl-1,2,3,4-tetrahydronaphthalen-1-yl)phenyl)piperidin-4-yl)(methyl)amino)methyl)isoindoline-1,3-dione;
2-(2,6-dioxopiperidin-3-yl)-4-fluoro-5-(((1-(4-((1R,2S)-6-hydroxy-2-phenyl-1,2,3,4-tetrahydronaphthalen-1-yl)phenyl)piperidin-4-yl)(methyl)amino)methyl)isoindoline-1,3 -dione;
2-(2,6-dioxopiperidin-3-yl)-5-fluoro-6-(((1-(4-((1R,2S)-6-hydroxy-2-phenyl-1,2,3,4-tetrahydronaphthalen-1-yl)phenyl)piperidin-4-yl)(methyl)amino)methyl)isoindoline-1,3-dione;
2-(2,6-dioxopiperidin-3-yl)-4-fluoro-6-(((1-(4-((1R,2S)-6-hydroxy-2-phenyl-1,2,3,4-tetrahydronaphthalen-1-yl)phenyl)piperidin-4-yl)(methyl)amino)methyl)isoindoline-1,3-dione;
5-(((2-(4-(1,2-bis(4-hydroxyphenyl)but-1-en-1-yl)phenoxy)ethyl)amino)methyl)-2-(2,6-dioxopiperidin-3 -yl)isoindoline-1,3 -dione;
5-(((2-(4-(1,2-bis(4-hydroxyphenyl)but-1-en-1-yl)phenoxy)ethyl)amino)methyl)-2-(2,6-dioxopiperidin-3-yl)-4-fluoroisoindoline-1,3-dione;
5-(((2-(4-(1,2-bis(4-hydroxyphenyl)but-1-en-1-yl)phenoxy)ethyl)amino)methyl)-2-(2,6-dioxopiperidin-3-yl)-6-fluoroisoindoline-1,3-dione;
6-(((2-(4-(1,2-bis(4-hydroxyphenyl)but-1-en-1-yl)phenoxy)ethyl)amino)methyl)-2-(2,6-dioxopiperidin-3-yl)-4-fluoroisoindoline-1,3-dione;
5-(((2-(4-(1,2-bis(4-hydroxyphenyl)but-1-en-1-yl)phenoxy)ethyl)(methyl)amino)methyl)-2-(2,6-dioxopiperidin-3-yl)isoindoline-1,3-dione;
5-(((2-(4-(1,2-bis(4-hydroxyphenyl)but-1-en-1-yl)phenoxy)ethyl)(methyl)amino)methyl)-2-(2,6-dioxopiperidin-3-yl)-4-fluoroisoindoline-1,3-dione;
5-(((2-(4-(1,2-bis(4-hydroxyphenyl)but-1-en-1-yl)phenoxy)ethyl)(methyl)amino)methyl)-2-(2,6-dioxopiperidin-3-yl)-6-fluoroisoindoline-1,3-dione;
6-(((2-(4-(1,2-bis(4-hydroxyphenyl)but-1-en-1-yl)phenoxy)ethyl)(methyl)amino)methyl)-2-(2,6-dioxopiperidin-3-yl)-4-fluoroisoindoline-1,3-dione;
2-(2,6-dioxopiperidin-3-yl)-5-(((2-(4-(1,2-diphenylbut-1-en-1-yl)phenoxy)ethyl)(methyl)amino)methyl)isoindoline-1,3-dione;
2-(2,6-dioxopiperidin-3-yl)-5-(((2-(4-(1,2-diphenylbut-1-en-1-yl)phenoxy)ethyl)(methyl)amino)methyl)-4-fluoroisoindoline-1,3-dione;
2-(2,6-dioxopiperidin-3-yl)-5-(((2-(4-(1,2-diphenylbut-1-en-1-yl)phenoxy)ethyl)(methyl)amino)methyl)-6-fluoroisoindoline-1,3-dione;
2-(2,6-dioxopiperidin-3 -yl)-6-(((2-(4-(1,2-diphenylbut-1-en-1-yl)phenoxy)ethyl)(methyl)amino)methyl)-4-fluorolsoindoline-1,3-dione;
5-(((2-(4-(4-chloro-l,2-diphenylbut-l-en-l-yl)phenoxy)ethyl)(methyl)amino)methyl)-2-(2,6-dioxopiperidin-3 -yl)isoindoline-1,3 -dione;
5-(((2-(4-(4-chloro-1,2-diphenylbut-1-en-1-yl)phenoxy)ethyl)(methyl)amino)methyl)-2-(2,6-dioxopiperidin-3-yl)-4-fluoroisoindoline-1,3-dione;
5-(((2-(4-(4-chloro-1,2-diphenylbut-1-en-1-yl)phenoxy)ethyl)(methyl)amino)methyl)-2-(2,6-dioxopiperidin-3-yl)-6-fluoroisoindoline-1,3-dione;
6-(((2-(4-(4-chloro-1,2-diphenylbut-1-en-1-yl)phenoxy)ethyl)(methyl)amino)methyl)-2-(2,6-dioxopiperidin-3-yl)-4-fluoroisoindoline-1,3-dione;
5-(((1-(3-(2,3-dichlorophenyl)-1H-pyrazolo[3,4-b]pyrazin-6-yl)-4-methylpiperidin-4-yl)amino)methyl)-2-(2,6-dioxopiperidin-3-yl)isoindoline-1,3-dione;
5-(((1-(3-(2,3-dichlorophenyl)-1H-pyrazolo[3,4-b]pyrazin-6-yl)-4-methylpiperidin-4-yl)amino)methyl)-2-(2,6-dioxopiperidin-3-yl)-4-fluoroisoindoline-1,3-dione;
5-(((1-(3-(2,3-dichlorophenyl)-1H-pyrazolo[3,4-b]pyrazin-6-yl)-4-methylpiperidin-4-yl)amino)methyl)-2-(2,6-dioxopiperidin-3-yl)-6-fluoroisoindoline-1,3-dione;
6-(((1-(3-(2,3-dichlorophenyl)-1H-pyrazolo[3,4-b]pyrazin-6-yl)-4-methylpiperidin-4-yl)amino)methyl)-2-(2,6-dioxopiperidin-3-yl)-4-fluoroisoindoline-1,3-dione;
5-((4-(3-(6-(4-amino-4-methylpiperidin-1-yl)-1H-pyrazolo[3,4-b]pyrazin-3-yl)-2-chlorophenyl)pip erazin-1-yl)methyl)-2-(2, 6-dioxopip eri din-3 -yl)i soindoline-1,3 -dione;
5-((4-(3-(6-(4-amino-4-methylpiperidin-1-yl)-1H-pyrazolo[3,4-b]pyrazin-3-yl)-2-chlorophenyl)piperazin-1-yl)methyl)-2-(2,6-dioxopiperidin-3-yl)-4-fluoroisoindoline-1,3-dione;
5-((4-(3-(6-(4-amino-4-methylpiperidin-1-yl)-1H-pyrazolo[3,4-b]pyrazin-3-yl)-2-chlorophenyl)piperazin-1-yl)methyl)-2-(2,6-dioxopiperidin-3-yl)-6-fluoroisoindoline-1,3-dione;
6-((4-(3-(6-(4-amino-4-methylpiperidin-1-yl)-1H-pyrazolo[3,4-b]pyrazin-3-yl)-2-chlorophenyl)piperazin-1-yl)methyl)-2-(2,6-dioxopiperidin-3-yl)-4-fluoroisoindoline-1,3-dione;
5-((4-(3-((3-amino-5-(4-amino-4-methylpiperidin-1-yl)pyrazin-2-yl)thio)-2-chlorophenyl)pip erazin-1-yl)methyl)-2-(2, 6-dioxopip eri din-3 -yl)i soindoline-1,3 -dione;
5-((4-(3-((3-amino-5-(4-amino-4-methylpiperidin-1-yl)pyrazin-2-yl)thio)-2-chlorophenyl)piperazin-1-yl)methyl)-2-(2,6-dioxopiperidin-3-yl)-4-fluoroisoindoline-1,3-dione;
5-((4-(3-((3-amino-5-(4-amino-4-methylpiperidin-1-yl)pyrazin-2-yl)thio)-2-chlorophenyl)piperazin-1-yl)methyl)-2-(2,6-dioxopiperidin-3-yl)-6-fluoroisoindoline-1,3-dione;
6-((4-(3-((3-amino-5-(4-amino-4-methylpiperidin-1-yl)pyrazin-2-yl)thio)-2-chlorophenyl)piperazin-1-yl)methyl)-2-(2,6-dioxopiperidin-3-yl)-4-fluoroisoindoline-1,3-dione;
5-(((3-((3-amino-5-(4-amino-4-methylpiperidin-1-yl)pyrazin-2-yl)thio)-2-chlorophenyl)amino)methyl)-2-(2,6-dioxopiperidin-3-yl)-4-fluoroisoindoline-1,3-dione;
5-(((3-((3-amino-5-(4-amino-4-methylpiperidin-1-yl)pyrazin-2-yl)thio)-2-chlorophenyl)amino)methyl)-2-(2,6-dioxopiperidin-3-yl)-6-fluoroisoindoline-1,3-dione;
6-(((3-((3-amino-5-(4-amino-4-methylpiperidin-1-yl)pyrazin-2-yl)thio)-2-chlorophenyl)amino)methyl)-2-(2,6-dioxopiperidin-3-yl)-4-fluoroisoindoline-1,3-dione;
5-(((3-((3-amino-5-(4-amino-4-methylpiperidin-1-yl)pyrazin-2-yl)thio)-2-chlorophenyl)amino)methyl)-2-(2,6-dioxopiperidin-3-yl)isoindoline-1,3-dione;
5-((4-(3-(3-amino-5-(4-amino-4-methylpiperidin-1-yl)pyrazin-2-yl)-2-chlorophenyl)piperazin-1-yl)methyl)-2-(2,6-dioxopiperidin-3-yl)isoindoline-1,3-dione;
5-((4-(3-(3-amino-5-(4-amino-4-methylpiperidin-1-yl)pyrazin-2-yl)-2-chlorophenyl)piperazin-1-yl)methyl)-2-(2,6-dioxopiperidin-3-yl)-4-fluoroisoindoline-1,3-dione;
5-((4-(3-(3-amino-5-(4-amino-4-methylpiperidin-1-yl)pyrazin-2-yl)-2-chlorophenyl)piperazin-1-yl)methyl)-2-(2,6-dioxopiperidin-3-yl)-6-fluoroisoindoline-1,3-dione;
6-((4-(3 -(3 -amino-5 -(4-amino-4-methylpip eri din-1-yl)pyrazin-2-yl)-2-chlorophenyl)pip erazin-1-yl)methyl)-2-(2,6-dioxopiperidin-3-yl)-4-fluoroisoindoline-1,3-dione;
5-((((3S,4S)-8-(6-amino-5-((2-amino-3-chloropyridin-4-yl)thio)pyrazin-2-yl)-3-methyl-2-oxa-8-azaspiro[4.5]decan-4-yl)amino)methyl)-2-(2,6-dioxopiperidin-3-yl)isoindoline-1,3-dione;
5-((((3S,4S)-8-(6-amino-5-((2-amino-3-chloropyridin-4-yl)thio)pyrazin-2-yl)-3-methyl-2-oxa-8-azaspiro[4.5]decan-4-yl)amino)methyl)-2-(2,6-dioxopiperidin-3-yl)-4-fluoroisoindoline-1,3-dione;
5-((((3S,4S)-8-(6-amino-5-((2-amino-3-chloropyridin-4-yl)thio)pyrazin-2-yl)-3-methyl-2-oxa-8-azaspiro[4.5]decan-4-yl)amino)methyl)-2-(2,6-dioxopiperidin-3-yl)-6-fluoroisoindoline-1,3-dione;
6-((((3S,4S)-8-(6-amino-5-((2-amino-3-chloropyridin-4-yl)thio)pyrazin-2-yl)-3-methyl-2-oxa-8-azaspiro[4.5]decan-4-yl)amino)methyl)-2-(2,6-dioxopiperidin-3-yl)-4-fluoroisoindoline-1,3-dione;
6-((((3S,4S)-8-(6-amino-5-((2-aminopyridin-4-yl)thio)pyrazin-2-yl)-3-methyl-2-oxa-8-azaspiro[4.5]decan-4-yl)amino)methyl)-2-(2,6-dioxopiperidin-3-yl)-4-fluoroisoindoline-1,3-dione;
5-((((3S,4S)-8-(6-amino-5-((2-aminopyridin-4-yl)thio)pyrazin-2-yl)-3-methyl-2-oxa-8-azaspiro[4.5]decan-4-yl)amino)methyl)-2-(2,6-dioxopiperidin-3-yl)isoindoline-1,3-dione;
5-((((3S,4S)-8-(6-amino-5-((2-aminopyridin-4-yl)thio)pyrazin-2-yl)-3-methyl-2-oxa-8-azaspiro[4.5]decan-4-yl)amino)methyl)-2-(2,6-dioxopiperidin-3-yl)-4-fluoroisoindoline-1,3-dione;
5-((((3S,4S)-8-(6-amino-5-((2-aminopyridin-4-yl)thio)pyrazin-2-yl)-3-methyl-2-oxa-8-azaspiro[4.5]decan-4-yl)amino)methyl)-2-(2,6-dioxopiperidin-3-yl)-6-fluoroisoindoline-1,3-dione;
5-((4-(4-((3-amino-5-((3S,4S)-4-amino-3-methyl-2-oxa-8-azaspiro[4.5]decan-8-yl)pyrazin-2-yl)thio)pyridin-2-yl)piperazin-1-yl)methyl)-2-(2,6-dioxopiperidin-3-yl)isoindoline-1,3-dione;
5-((4-(4-((3-amino-5-((3S,4S)-4-amino-3-methyl-2-oxa-8-azaspiro[4.5]decan-8-yl)pyrazin-2-yl)thio)pyridin-2-yl)piperazin-1-yl)methyl)-2-(2,6-dioxopiperidin-3-yl)-4-fluoroisoindoline-1,3-dione;
5-((4-(4-((3-amino-5-((3S,4S)-4-amino-3-methyl-2-oxa-8-azaspiro[4.5]decan-8-yl)pyrazin-2-yl)thio)pyridin-2-yl)piperazin-1-yl)methyl)-2-(2,6-dioxopiperidin-3-yl)-6-fluoroisoindoline-1,3 -dione;
6-((4-(4-((3-amino-5-((3S,4S)-4-amino-3-methyl-2-oxa-8-azaspiro[4.5]decan-8-yl)pyrazin-2-yl)thio)pyridin-2-yl)piperazin-1-yl)methyl)-2-(2,6-dioxopiperidin-3-yl)-4-fluoroisoindoline-1,3-dione;
5-((4-(4-((3-amino-5-((3S,4S)-4-amino-3-methyl-2-oxa-8-azaspiro[4.5]decan-8-yl)pyrazin-2-yl)thio)-3-chloropyridin-2-yl)piperazin-1-yl)methyl)-2-(2,6-dioxopiperidin-3-yl)isoindoline-1,3-dione;
5-((4-(4-((3-amino-5-((3S,4S)-4-amino-3-methyl-2-oxa-8-azaspiro[4.5]decan-8-yl)pyrazin-2-yl)thio)-3-chloropyridin-2-yl)piperazin-l-yl)methyl)-2-(2,6-dioxopiperidin-3-yl)-4-fluoroisoindoline-1,3-dione;
5-((4-(4-((3-amino-5-((3S,4S)-4-amino-3-methyl-2-oxa-8-azaspiro[4.5]decan-8-yl)pyrazin-2-yl)thio)-3-chloropyridin-2-yl)piperazin-l-yl)methyl)-2-(2,6-dioxopiperidin-3-yl)-6-fluoroisoindoline-1,3-dione;
6-((4-(4-((3-amino-5-((3S,4S)-4-amino-3-methyl-2-oxa-8-azaspiro[4.5]decan-8-yl)pyrazin-2-yl)thio)-3-chloropyridin-2-yl)piperazin-l-yl)methyl)-2-(2,6-dioxopiperidin-3-yl)-4-fluoroisoindoline-1,3-dione;
5-(((1-(6-amino-5-(2,3-dichlorophenyl)pyrazin-2-yl)-4-methylpiperidin-4-yl)amino)methyl)-2-(2,6-dioxopiperidin-3 -yl)isoindoline-1,3-dione;
5-(((1-(6-amino-5-(2,3-dichlorophenyl)pyrazin-2-yl)-4-methylpiperidin-4-yl)amino)methyl)-2-(2,6-dioxopiperidin-3-yl)-4-fluoroisoindoline-1,3-dione;
5-(((1-(6-amino-5-(2,3-dichlorophenyl)pyrazin-2-yl)-4-methylpiperidin-4-yl)amino)methyl)-2-(2,6-dioxopiperidin-3-yl)-6-fluoroisoindoline-1,3-dione;
6-(((1-(6-amino-5-(2,3-dichlorophenyl)pyrazin-2-yl)-4-methylpiperidin-4-yl)amino)methyl)-2-(2,6-dioxopiperidin-3-yl)-4-fluoroisoindoline-1,3-dione;
5-((2,3-difluoro-6-(2-morpholinothiazol-4-yl)phenoxy)methyl)-2-(2,6-dioxopiperidin-3-yl)isoindoline-1,3-dione;
5-((2,3-difluoro-6-(2-morpholinothiazol-4-yl)phenoxy)methyl)-2-(2,6-dioxopiperidin-3-yl)-4-fluoroisoindoline-1,3-dione;
6-((2,3-difluoro-6-(2-morpholinothiazol-4-yl)phenoxy)methyl)-2-(2,6-dioxopiperidin-3-yl)-4-fluoroisoindoline-1,3-dione;
5-((2,3-difluoro-6-(2-morpholinothiazol-4-yl)phenoxy)methyl)-2-(2,6-dioxopiperidin-3-yl)-6-fluoroisoindoline-1,3-dione;
N-((1r,4r)-4-(3-chloro-4-cyanophenoxy)cyclohexyl)-6-(4-(4-((2-(2,6-dioxopiperidin-3-yl)-1,3-dioxoisoindolin-5-yl)methyl)piperazin-1-yl)piperidin-1-yl)pyridazine-3-carboxamide;
N-((1r,4r)-4-(3-chloro-4-cyanophenoxy)cyclohexyl)-6-(4-(4-((2-(2,6-dioxopiperidin-3-yl)-4-fluoro-1,3-dioxoisoindolin-5-yl)methyl)piperazin-1-yl)piperidin-1-yl)pyridazine-3-carboxamide;
N-((1r,4r)-4-(3-chloro-4-cyanophenoxy)cyclohexyl)-6-(4-(4-((2-(2,6-dioxopiperidin-3-yl)-6-fluoro-1,3-dioxoisoindolin-5-yl)methyl)piperazin-1-yl)piperidin-1-yl)pyridazine-3-carboxamide;
N-((1r,4r)-4-(3-chloro-4-cyanophenoxy)cyclohexyl)-6-(4-(4-((2-(2,6-dioxopiperidin-3-yl)-7-fluoro-1,3-dioxoisoindolin-5-yl)methyl)piperazin-1-yl)piperidin-1-yl)pyridazine-3-carboxamide;
N-((1r,3r)-3-(3-chloro-4-cyanophenoxy)-2,2,4,4-tetramethylcyclobutyl)-6-(4-((2-(2,6-dioxopiperidin-3-yl)-1,3-dioxoisoindolin-5-yl)methyl)piperazin-1-yl)pyridazine-3-carboxamide;
N-((1r,3r)-3-(3-chloro-4-cyanophenoxy)-2,2,4,4-tetramethylcyclobutyl)-6-(4-((2-(2,6-dioxopiperidin-3-yl)-4-fluoro-1,3-dioxoisoindolin-5-yl)methyl)piperazin-1-yl)pyridazine-3-carboxamide;
N-((1r,3r)-3-(3-chloro-4-cyanophenoxy)-2,2,4,4-tetramethylcyclobutyl)-6-(4-((2-(2,6-dioxopiperidin-3-yl)-6-fluoro-1,3-dioxoisoindolin-5-yl)methyl)piperazin-1-yl)pyridazine-3-carboxamide;
N-((1r,3r)-3-(3-chloro-4-cyanophenoxy)-2,2,4,4-tetramethylcyclobutyl)-6-(4-((2-(2,6-dioxopiperidin-3-yl)-7-fluoro-1,3-dioxoisoindolin-5-yl)methyl)piperazin-1-yl)pyridazine-3-carboxamide;
N-((lr,3r)-3-(3-chloro-4-cyanophenoxy)-2,2,4,4-tetramethylcyclobutyl)-6-(4-((2-(2,6-dioxopiperidin-3-yl)-1,3-dioxoisoindolin-5-yl)methyl)piperazin-1-yl)nicotinamide;
N-((1r,3r)-3-(3-chloro-4-cyanophenoxy)-2,2,4,4-tetramethylcyclobutyl)-6-(4-((2-(2,6-dioxopiperidin-3-yl)-4-fluoro-1,3-dioxoisoindolin-5-yl)methyl)piperazin-1-yl)nicotinamide;
N-((1r,3r)-3-(3-chloro-4-cyanophenoxy)-2,2,4,4-tetramethylcyclobutyl)-6-(4-((2-(2,6-dioxopiperidin-3-yl)-6-fluoro-1,3-dioxoisoindolin-5-yl)methyl)piperazin-1-yl)nicotinamide;
N-((1r,3r)-3-(3-chloro-4-cyanophenoxy)-2,2,4,4-tetramethylcyclobutyl)-6-(4-((2-(2,6-dioxopip eridin-3 -yl)-7-fluoro-1, 3 -dioxoisoindolin-5-yl)methyl)pip erazin-1-yl)nicotinamide;
N-((1r,4r)-4-(3-chloro-4-cyanophenoxy)cyclohexyl)-6-(8-((2-(2,6-dioxopiperidin-3-yl)-1,3-dioxoisoindolin-5-yl)methyl)-3,8-diazabicyclo[3.2.1]octan-3-yl)pyridazine-3-carboxamide;
N-((1r,4r)-4-(3-chloro-4-cyanophenoxy)cyclohexyl)-6-(8-((2-(2,6-dioxopiperidin-3-yl)-4-fluoro-1,3-dioxoisoindolin-5-yl)methyl)-3,8-diazabicyclo[3.2.1]octan-3-yl)pyridazine-3-carboxamide;
N-((1r,4r)-4-(3-chloro-4-cyanophenoxy)cyclohexyl)-6-(8-((2-(2,6-dioxopiperidin-3-yl)-6-fluoro-1,3-dioxoisoindolin-5-yl)methyl)-3,8-diazabicyclo[3.2.1]octan-3-yl)pyridazine-3-carboxamide;
N-((1r,4r)-4-(3-chloro-4-cyanophenoxy)cyclohexyl)-6-(8-((2-(2,6-dioxopiperidin-3-yl)-7-fluoro-1,3-dioxoisoindolin-5-yl)methyl)-3,8-diazabicyclo[3.2.1]octan-3-yl)pyridazine-3-carboxamide;
N-((1r,4r)-4-(3-chloro-4-cyanophenoxy)cyclohexyl)-6-(6-((2-(2,6-dioxopiperidin-3-yl)-1,3-dioxoisoindolin-5-yl)methyl)-3,6-diazabicyclo[3.1.1]heptan-3-yl)pyridazine-3-carboxamide;
N-((1r,4r)-4-(3-chloro-4-cyanophenoxy)cyclohexyl)-6-(6-((2-(2,6-dioxopiperidin-3-yl)-4-fluoro-1,3-dioxoisoindolin-5-yl)methyl)-3,6-diazabicyclo[3.1.1]heptan-3-yl)pyridazine-3-carboxamide;
N-((1r,4r)-4-(3-chloro-4-cyanophenoxy)cyclohexyl)-6-(6-((2-(2,6-dioxopiperidin-3-yl)-6-fluoro-1,3-dioxoisoindolin-5-yl)methyl)-3,6-diazabicyclo[3.1.1]heptan-3-yl)pyridazine-3-carboxamide;
N-((1r,4r)-4-(3-chloro-4-cyanophenoxy)cyclohexyl)-6-(6-((2-(2,6-dioxopiperidin-3-yl)-7-fluoro-1,3-dioxoisoindolin-5-yl)methyl)-3,6-diazabicyclo[3.1.1]heptan-3-yl)pyridazine-3-carboxamide;
N-((1r,4r)-4-(3-chloro-4-cyanophenoxy)cyclohexyl)-6-(3-((2-(2,6-dioxopiperidin-3-yl)-1,3-dioxoisoindolin-5-yl)methyl)-3,6-diazabicyclo[3.1.1]heptan-6-yl)pyridazine-3-carboxamide;
N-((1r,4r)-4-(3-chloro-4-cyanophenoxy)cyclohexyl)-6-(3-((2-(2,6-dioxopiperidin-3-yl)-4-fluoro-1,3-dioxoisoindolin-5-yl)methyl)-3,6-diazabicyclo[3.1.1]heptan-6-yl)pyridazine-3-carboxamide;
N-((1r,4r)-4-(3-chloro-4-cyanophenoxy)cyclohexyl)-6-(3-((2-(2,6-dioxopiperidin-3-yl)-6-fluoro-1,3-dioxoisoindolin-5-yl)methyl)-3,6-diazabicyclo[3.1.1]heptan-6-yl)pyridazine-3-carboxamide;
N-((1r,4r)-4-(3-chloro-4-cyanophenoxy)cyclohexyl)-6-(3-((2-(2,6-dioxopiperidin-3-yl)-7-fluoro-1,3-dioxoisoindolin-5-yl)methyl)-3,6-diazabicyclo[3.1.1]heptan-6-yl)pyridazine-3-carboxamide;
N-((1r,4r)-4-(3-chloro-4-cyanophenoxy)cyclohexyl)-6-(3-((2-(2,6-dioxopiperidin-3-yl)-1,3-dioxoisoindolin-5-yl)methyl)-3,8-diazabicyclo[3.2.1]octan-8-yl)pyridazine-3-carboxamide;
N-((1r,4r)-4-(3-chloro-4-cyanophenoxy)cyclohexyl)-6-(3-((2-(2,6-dioxopiperidin-3-yl)-4-fluoro-1,3-dioxoisoindolin-5-yl)methyl)-3,8-diazabicyclo[3.2.1]octan-8-yl)pyridazine-3-carboxamide;
N-((1r,4r)-4-(3-chloro-4-cyanophenoxy)cyclohexyl)-6-(3-((2-(2,6-dioxopiperidin-3-yl)-6-fluoro-1,3-dioxoisoindolin-5-yl)methyl)-3,8-diazabicyclo[3.2.1]octan-8-yl)pyridazine-3-carboxamide;
N-((1r,4r)-4-(3-chloro-4-cyanophenoxy)cyclohexyl)-6-(3-((2-(2,6-dioxopiperidin-3-yl)-7-fluoro-1,3-dioxoisoindolin-5-yl)methyl)-3,8-diazabicyclo[3.2.1]octan-8-yl)pyridazine-3-carboxamide;
N-((1r,4r)-4-(3-chloro-4-cyanophenoxy)cyclohexyl)-6-(4-((2-(2,6-dioxopiperidin-3-yl)-1,3-dioxoisoindolin-5-yl)methyl)piperazin-1-yl)pyridazine-3-carboxamide;
N-((1r,4r)-4-(3-chloro-4-cyanophenoxy)cyclohexyl)-6-(4-((2-(2,6-dioxopiperidin-3-yl)-4-fluoro-1,3-dioxoisoindolin-5-yl)methyl)piperazin-1-yl)pyridazine-3-carboxamide;
N-((1r,4r)-4-(3-chloro-4-cyanophenoxy)cyclohexyl)-6-(4-((2-(2,6-dioxopiperidin-3-yl)-6-fluoro-1,3-dioxoisoindolin-5-yl)methyl)piperazin-1-yl)pyridazine-3-carboxamide;
N-((1r,4r)-4-(3-chloro-4-cyanophenoxy)cyclohexyl)-6-(4-((2-(2,6-dioxopiperidin-3-yl)-7-fluoro-1,3-dioxoisoindolin-5-yl)methyl)piperazin-1-yl)pyridazine-3-carboxamide;
5-((4-(4-((9-cyclopentyl-8-(phenylamino)-9H-purin-2-yl)amino)phenyl)piperazin-1-yl)methyl)-2-(2,6-dioxopiperidin-3-yl)isoindoline-1,3-dione;
5-((4-(4-((9-cyclopentyl-8-(phenylamino)-9H-purin-2-yl)amino)phenyl)piperazin-1-yl)methyl)-2-(2,6-dioxopiperidin-3-yl)-4-fluoroisoindoline-1,3-dione;
5-((4-(4-((9-cyclopentyl-8-(phenylamino)-9H-purin-2-yl)amino)phenyl)piperazin-1-yl)methyl)-2-(2,6-dioxopiperidin-3-yl)-6-fluoroisoindoline-1,3-dione;
6-((4-(4-((9-cyclopentyl-8-(phenylamino)-9H-purin-2-yl)amino)phenyl)piperazin-1-yl)methyl)-2-(2,6-dioxopiperidin-3-yl)-4-fluoroisoindoline-1,3-dione;
5-(((1-(4-((9-cyclopentyl-8-(phenylamino)-9H-purin-2-yl)amino)phenyl)piperidin-4-yl)(methyl)amino)methyl)-2-(2,6-dioxopiperidin-3-yl)isoindoline-1,3-dione;
5-(((1-(4-((9-cyclopentyl-8-(phenylamino)-9H-purin-2-yl)amino)phenyl)piperidin-4-yl)(methyl)amino)methyl)-2-(2,6-dioxopiperidin-3-yl)-4-fluoroisoindoline-1,3-dione;
5-(((1-(4-((9-cyclopentyl-8-(phenylamino)-9H-purin-2-yl)amino)phenyl)piperidin-4-yl)(methyl)amino)methyl)-2-(2,6-dioxopiperidin-3-yl)-6-fluoroisoindoline-1,3-dione;
6-(((1-(4-((9-cyclopentyl-8-(phenylamino)-9H-purin-2-yl)amino)phenyl)piperidin-4-yl)(methyl)amino)methyl)-2-(2,6-dioxopiperidin-3-yl)-4-fluoroisoindoline-1,3-dione;
5-((4-(1-(4-((9-cyclopentyl-8-(phenylamino)-9H-purin-2-yl)amino)phenyl)piperidin-4-yl)piperazin-1-yl)methyl)-2-(2,6-dioxopiperidin-3-yl)isoindoline-1,3-dione;
5-((4-(1-(4-((9-cyclopentyl-8-(phenylamino)-9H-purin-2-yl)amino)phenyl)piperidin-4-yl)pip erazin-1-yl)methyl)-2-(2, 6-dioxopip eri din-3 -yl)-4-fl uoroisoindoline-1, 3 -dione;
5-((4-(1-(4-((9-cyclopentyl-8-(phenylamino)-9H-purin-2-yl)amino)phenyl)piperidin-4-yl)piperazin-1-yl)methyl)-2-(2,6-dioxopiperidin-3-yl)-6-fluoroisoindoline-1,3-dione;
6-((4-(1-(4-((9-cyclopentyl-8-(phenylamino)-9H-purin-2-yl)amino)phenyl)piperidin-4-yl)pip erazin-1-yl)methyl)-2-(2, 6-dioxopip eri din-3 -yl)-4-fl uoroisoindoline-1, 3 -dione;
5-((4-(4-(4-((9-cyclopentyl-8-(phenylamino)-9H-purin-2-yl)amino)phenyl)piperazin-1-yl)piperidin-1-yl)methyl)-2-(2,6-dioxopiperidin-3-yl)isoindoline-1,3-dione;
5-((4-(4-(4-((9-cyclopentyl-8-(phenylamino)-9H-purin-2-yl)amino)phenyl)piperazin-1-yl)piperidin-1-yl)methyl)-2-(2,6-dioxopiperidin-3-yl)-4-fluoroisoindoline-1,3-dione;
5-((4-(4-(4-((9-cyclopentyl-8-(phenylamino)-9H-purin-2-yl)amino)phenyl)piperazin-1-yl)piperidin-1-yl)methyl)-2-(2,6-dioxopiperidin-3-yl)-6-fluoroisoindoline-1,3-dione;
6-((4-(4-(4-((9-cyclopentyl-8-(phenylamino)-9H-purin-2-yl)amino)phenyl)piperazin-1-yl)piperidin-1-yl)methyl)-2-(2,6-dioxopiperidin-3-yl)-4-fluoroisoindoline-1,3-dione;
2-(2,6-dioxopiperidin-3-yl)-5-(((5-methoxy-4-((4-(1-methyl-1H-indol-3-yl)pyrimidin-2-yl)amino)-2-nitrophenyl)amino)methyl)isoindoline-1,3-dione;
2-(2,6-dioxopiperidin-3-yl)-4-fluoro-5-(((5-methoxy-4-((4-(1-methyl-1H-indol-3-yl)pyrimidin-2-yl)amino)-2-nitrophenyl)amino)methyl)isoindoline-1,3-dione;
2-(2,6-dioxopiperidin-3-yl)-5-fluoro-6-(((5-methoxy-4-((4-(1-methyl-1H-indol-3-yl)pyrimidin-2-yl)amino)-2-nitrophenyl)amino)methyl)isoindoline-1,3-dione;
2-(2,6-dioxopiperidin-3-yl)-4-fluoro-6-(((5-methoxy-4-((4-(1-methyl-1H-indol-3-yl)pyrimidin-2-yl)amino)-2-nitrophenyl)amino)methyl)isoindoline-1,3-dione;
N-(2-((2-(((2-(2,6-dioxopiperidin-3-yl)-1,3-dioxoisoindolin-5-yl)methyl)(methyl)amino)ethyl)(methyl)amino)-4-methoxy-5-((4-(1-methyl-1H-indol-3-yl)pyrimidin-2-yl)amino)phenyl)acrylamide;
N-(2-((2-(((2-(2,6-dioxopiperidin-3-yl)-4-fluoro-1,3-dioxoisoindolin-5-yl)methyl)(methyl)amino)ethyl)(methyl)amino)-4-methoxy-5-((4-(1-methyl-1H-indol-3-yl)pyrimidin-2-yl)amino)phenyl)acrylamide;
N-(2-((2-(((2-(2,6-dioxopiperidin-3-yl)-6-fluoro-1,3-dioxoisoindolin-5-yl)methyl)(methyl)amino)ethyl)(methyl)amino)-4-methoxy-5-((4-(1-methyl-1H-indol-3-yl)pyrimidin-2-yl)amino)phenyl)acrylamide;
N-(2-((2-(((2-(2,6-dioxopiperidin-3-yl)-7-fluoro-1,3-dioxoisoindolin-5-yl)methyl)(methyl)amino)ethyl)(methyl)amino)-4-methoxy-5-((4-(1-methyl-1H-indol-3-yl)pyrimidin-2-yl)amino)phenyl)acrylamide;
5-((4-(3-((4-((3-chloro-4-fluorophenyl)amino)-7-methoxyquinazolin-6-yl)oxy)propyl)piperazin-1-yl)methyl)-2-(2,6-dioxopiperidin-3-yl)isoindoline-1,3-dione;
5-((4-(3-((4-((3-chloro-4-fluorophenyl)amino)-7-methoxyquinazolin-6-yl)oxy)propyl)piperazin-1-yl)methyl)-2-(2,6-dioxopiperidin-3-yl)-4-fluoroisoindoline-1,3-dione;
5-((4-(3-((4-((3-chloro-4-fluorophenyl)amino)-7-methoxyquinazolin-6-yl)oxy)propyl)piperazin-1-yl)methyl)-2-(2,6-dioxopiperidin-3-yl)-6-fluoroisoindoline-1,3-dione;
6-((4-(3-((4-((3-chloro-4-fluorophenyl)amino)-7-methoxyquinazolin-6-yl)oxy)propyl)piperazin-1-yl)methyl)-2-(2,6-dioxopiperidin-3-yl)-4-fluoroisoindoline-1,3-dione;
N-(4-((3-chloro-4-fluorophenyl)amino)-7-(3-(4-((2-(2,6-dioxopiperidin-3-yl)-1,3-dioxoisoindolin-5-yl)methyl)piperazin-1-yl)propoxy)quinazolin-6-yl)acrylamide;
N-(4-((3-chloro-4-fluorophenyl)amino)-7-(3-(4-((2-(2,6-dioxopiperidin-3-yl)-4-fluoro-1,3-dioxoisoindolin-5-yl)methyl)piperazin-1-yl)propoxy)quinazolin-6-yl)acrylamide;
N-(4-((3-chloro-4-fluorophenyl)amino)-7-(3-(4-((2-(2,6-dioxopiperidin-3-yl)-6-fluoro-1,3-dioxoisoindolin-5-yl)methyl)piperazin-1-yl)propoxy)quinazolin-6-yl)acrylamide;
N-(4-((3-chloro-4-fluorophenyl)amino)-7-(3-(4-((2-(2,6-dioxopiperidin-3-yl)-7-fluoro-1,3-dioxoisoindolin-5-yl)methyl)piperazin-1-yl)propoxy)quinazolin-6-yl)acrylamide;
5-(((4-((3,4-dichloro-2-fluorophenyl)amino)-7-methoxyquinazolin-6-yl)oxy)methyl)-2-(2,6-dioxopiperidin-3 -yl)isoindoline-1,3 -dione;
5-(((4-((3,4-dichloro-2-fluorophenyl)amino)-7-methoxyquinazolin-6-yl)oxy)methyl)-2-(2,6-dioxopiperidin-3-yl)-4-fluoroisoindoline-1,3-dione;
5- (((4-((3,4-dichloro-2-fluorophenyl)amino)-7-methoxyquinazolin-6-yl)oxy)methyl)-2-(2,6-dioxopiperidin-3-yl)-6-fluoroisoindoline-1,3-dione;
6-(((4-((3,4-dichloro-2-fluorophenyl)amino)-7-methoxyquinazolin-6-yl)oxy)methyl)-2-(2,6-dioxopiperidin-3-yl)-4-fluoroisoindoline-1,3-dione;
5-((4-((4-((3,4-dichloro-2-fluorophenyl)amino)-7-methoxyquinazolin-6-yl)oxy)piperidin-1-yl)methyl)-2-(2,6-dioxopiperidin-3-yl)isoindoline-1,3-dione;
5-((4-((4-((3,4-dichloro-2-fluorophenyl)amino)-7-methoxyquinazolin-6-yl)oxy)piperidin-1-yl)methyl)-2-(2,6-dioxopiperidin-3-yl)-4-fluoroisoindoline-1,3-dione;
5-((4-((4-((3,4-dichloro-2-fluorophenyl)amino)-7-methoxyquinazolin-6-yl)oxy)piperidin-1-yl)methyl)-2-(2,6-dioxopiperidin-3-yl)-6-fluoroisoindoline-1,3-dione;
6-((4-((4-((3,4-dichloro-2-fluorophenyl)amino)-7-methoxyquinazolin-6-yl)oxy)piperidin-1-yl)methyl)-2-(2,6-dioxopiperidin-3-yl)-4-fluoroisoindoline-1,3-dione;
5-(((4-((3-chloro-4-fluorophenyl)amino)-7-(((S)-tetrahydrofuran-3-yl)oxy)quinazolin-6-yl)amino)methyl)-2-(2,6-dioxopiperidin-3-yl)isoindoline-1,3-dione;
5-(((4-((3-chloro-4-fluorophenyl)amino)-7-(((S)-tetrahydrofuran-3-yl)oxy)quinazolin-6-yl)amino)methyl)-2-(2,6-dioxopiperidin-3-yl)-4-fluoroisoindoline-1,3-dione;
5-(((4-((3-chloro-4-fluorophenyl)amino)-7-(((S)-tetrahydrofuran-3-yl)oxy)quinazolin-6-yl)amino)methyl)-2-(2,6-dioxopiperidin-3-yl)-6-fluoroisoindoline-1,3-dione;
6-(((4-((3-chloro-4-fluorophenyl)amino)-7-(((S)-tetrahydrofuran-3-yl)oxy)quinazolin-6-yl)amino)methyl)-2-(2,6-dioxopiperidin-3-yl)-4-fluoroisoindoline-1,3-dione;
5-(((4-((3-chloro-2-fluorophenyl)amino)-7-methoxyquinazolin-6-yl)oxy)methyl)-2-(2,6-dioxopiperidin-3 -yl)isoindoline-1,3 -dione;
5-(((4-((3-chloro-2-fluorophenyl)amino)-7-methoxyquinazolin-6-yl)oxy)methyl)-2-(2,6-dioxopiperidin-3-yl)-4-fluoroisoindoline-1,3-dione;
5-(((4-((3-chloro-2-fluorophenyl)amino)-7-methoxyquinazolin-6-yl)oxy)methyl)-2-(2,6-dioxopiperidin-3-yl)-6-fluoroisoindoline-1,3-dione;
6-(((4-((3-chloro-2-fluorophenyl)amino)-7-methoxyquinazolin-6-yl)oxy)methyl)-2-(2,6-dioxopiperidin-3-yl)-4-fluoroisoindoline-1,3-dione;
5-(((4-((3-chloro-4-fluorophenyl)amino)-7-methoxyquinazolin-6-yl)oxy)methyl)-2-(2,6-dioxopiperidin-3 -yl)isoindoline-1,3 -dione;
5-(((4-((3-chloro-4-fluorophenyl)amino)-7-methoxyquinazolin-6-yl)oxy)methyl)-2-(2,6-dioxopiperidin-3-yl)-4-fluoroisoindoline-1,3-dione;
5-(((4-((3-chloro-4-fluorophenyl)amino)-7-methoxyquinazolin-6-yl)oxy)methyl)-2-(2,6-dioxopiperidin-3-yl)-6-fluoroisoindoline-1,3-dione;
6-(((4-((3-chloro-4-fluorophenyl)amino)-7-methoxyquinazolin-6-yl)oxy)methyl)-2-(2,6-dioxopiperidin-3-yl)-4-fluoroisoindoline-1,3-dione;
5-(((4-((3-chloro-4-fluorophenyl)amino)-7-methoxyquinazolin-6-yl)amino)methyl)-2-(2,6-dioxopiperidin-3 -yl)isoindoline-1,3 -dione;
5-(((4-((3-chloro-4-fluorophenyl)amino)-7-methoxyquinazolin-6-yl)amino)methyl)-2-(2,6-dioxopiperidin-3-yl)-4-fluoroisoindoline-1,3-dione;
5-(((4-((3-chloro-4-fluorophenyl)amino)-7-methoxyquinazolin-6-yl)amino)methyl)-2-(2,6-dioxopiperidin-3-yl)-6-fluoroisoindoline-1,3-dione;
6-(((4-((3-chloro-4-fluorophenyl)amino)-7-methoxyquinazolin-6-yl)amino)methyl)-2-(2,6-dioxopiperidin-3-yl)-4-fluoroisoindoline-1,3-dione;
(E)-N-(4-((3-chloro-4-fluorophenyl)amino)-7-methoxyquinazolin-6-yl)-4-(4-((2-(2,6-dioxopiperidin-3-yl)-1,3-dioxoisoindolin-5-yl)methyl)piperazin-1-yl)but-2-enamide;
(E)-N-(4-((3-chloro-4-fluorophenyl)amino)-7-methoxyquinazolin-6-yl)-4-(4-((2-(2,6-dioxopiperidin-3-yl)-4-fluoro-1,3-dioxoisoindolin-5-yl)methyl)piperazin-1-yl)but-2-enamide;
(E)-N-(4-((3-chloro-4-fluorophenyl)amino)-7-methoxyquinazolin-6-yl)-4-(4-((2-(2,6-dioxopiperidin-3-yl)-6-fluoro-1,3-dioxoisoindolin-5-yl)methyl)piperazin-1-yl)but-2-enamide;
(E)-N-(4-((3-chloro-4-fluorophenyl)amino)-7-methoxyquinazolin-6-yl)-4-(4-((2-(2,6-dioxopiperidin-3-yl)-7-fluoro-1,3-dioxoisoindolin-5-yl)methyl)piperazin-1-yl)but-2-enamide;
2-(7-(((2-(2,6-dioxopiperidin-3-yl)-1,3-dioxoisoindolin-5-yl)methyl)amino)-1-oxoisoindolin-2-yl)-2-(5-fluoro-2-hydroxyphenyl)-N-(thiazol-2-yl)acetamide;
2-(7-(((2-(2,6-dioxopiperidin-3-yl)-4-fluoro-1,3-dioxoisoindolin-5-yl)methyl)amino)-1-oxoisoindolin-2-yl)-2-(5-fluoro-2-hydroxyphenyl)-N-(thiazol-2-yl)acetamide;
2-(7-(((2-(2,6-dioxopiperidin-3-yl)-6-fluoro-1,3-dioxoisoindolin-5-yl)methyl)amino)-1-oxoisoindolin-2-yl)-2-(5-fluoro-2-hydroxyphenyl)-N-(thiazol-2-yl)acetamide;
2-(7-(((2-(2,6-dioxopiperidin-3-yl)-7-fluoro-1,3-dioxoisoindolin-5-yl)methyl)amino)-1-oxoisoindolin-2-yl)-2-(5-fluoro-2-hydroxyphenyl)-N-(thiazol-2-yl)acetamide;
2-(6-(4-((2-(2,6-dioxopiperidin-3-yl)-1,3-dioxoisoindolin-5-yl)methyl)piperazin-1-yl)-1-oxoisoindolin-2-yl)-2-(5-fluoro-2-hydroxyphenyl)-N-(thiazol-2-yl)acetamide;
2-(6-(4-((2-(2,6-dioxopiperidin-3-yl)-4-fluoro-1,3-dioxoisoindolin-5-yl)methyl)piperazin-1-yl)-1-oxoisoindolin-2-yl)-2-(5-fluoro-2-hydroxyphenyl)-N-(thiazol-2-yl)acetamide;
2-(6-(4-((2-(2,6-dioxopiperidin-3-yl)-6-fluoro-1,3-dioxoisoindolin-5-yl)methyl)piperazin-1-yl)-1-oxoisoindolin-2-yl)-2-(5-fluoro-2-hydroxyphenyl)-N-(thiazol-2-yl)acetamide;
2-(6-(4-((2-(2,6-dioxopiperidin-3-yl)-7-fluoro-1,3-dioxoisoindolin-5-yl)methyl)piperazin-1-yl)-1-oxoisoindolin-2-yl)-2-(5-fluoro-2-hydroxyphenyl)-N-(thiazol-2-yl)acetamide;
2-(6-(4-(((2-(2,6-dioxopiperidin-3-yl)-1,3-dioxoisoindolin-5-yl)methyl)(methyl)amino)piperidin-1-yl)-1-oxoisoindolin-2-yl)-2-(5-fluoro-2-hydroxyphenyl)-N-(thiazol-2-yl)acetamide;
2-(6-(4-(((2-(2,6-dioxopiperidin-3-yl)-4-fluoro-1,3-dioxoisoindolin-5-yl)methyl)(methyl)amino)piperidin-1-yl)-1-oxoisoindolin-2-yl)-2-(5-fluoro-2-hydroxyphenyl)-N-(thiazol-2-yl)acetamide;
2-(6-(4-(((2-(2,6-dioxopiperidin-3-yl)-6-fluoro-1,3-dioxoisoindolin-5-yl)methyl)(methyl)amino)piperidin-1-yl)-1-oxoisoindolin-2-yl)-2-(5-fluoro-2-hydroxyphenyl)-N-(thiazol-2-yl)acetamide;
2-(6-(4-(((2-(2,6-dioxopiperidin-3-yl)-7-fluoro-1,3-dioxoisoindolin-5-yl)methyl)(methyl)amino)piperidin-1-yl)-1-oxoisoindolin-2-yl)-2-(5-fluoro-2-hydroxyphenyl)-N-(thiazol-2-yl)acetamide;
2-(6-(4-(4-((2-(2,6-dioxopiperidin-3-yl)-1,3-dioxoisoindolin-5-yl)methyl)piperazin-1-yl)piperidin-1-yl)-1-oxoisoindolin-2-yl)-2-(5-fluoro-2-hydroxyphenyl)-N-(thiazol-2-yl)acetamide;
2-(6-(4-(4-((2-(2,6-dioxopiperidin-3-yl)-4-fluoro-1,3-dioxoisoindolin-5-yl)methyl)piperazin-1-yl)piperidin-1-yl)-1-oxoisoindolin-2-yl)-2-(5-fluoro-2-hydroxyphenyl)-N-(thiazol-2-yl)acetamide;
2-(6-(4-(4-((2-(2,6-dioxopiperidin-3-yl)-6-fluoro-1,3-dioxoisoindolin-5-yl)methyl)piperazin-1-yl)piperidin-1-yl)-1-oxoisoindolin-2-yl)-2-(5-fluoro-2-hydroxyphenyl)-N-(thiazol-2-yl)acetamide;
2-(6-(4-(4-((2-(2,6-dioxopiperidin-3-yl)-7-fluoro-1,3-dioxoisoindolin-5-yl)methyl)piperazin-1-yl)piperidin-1-yl)-1-oxoisoindolin-2-yl)-2-(5-fluoro-2-hydroxyphenyl)-N-(thiazol-2-yl)acetamide;
2-(6-(4-(4-((2-(2,6-dioxopiperidin-3-yl)-1,3-dioxoisoindolin-5-yl)methyl)piperazin-1-yl)phenyl)-1-oxoisoindolin-2-yl)-2-(5-fluoro-2-hydroxyphenyl)-N-(thiazol-2-yl)acetamide;
2-(6-(4-(4-((2-(2,6-dioxopiperidin-3-yl)-4-fluoro-1,3-dioxoisoindolin-5-yl)methyl)piperazin-1-yl)phenyl)-1-oxoisoindolin-2-yl)-2-(5-fluoro-2-hydroxyphenyl)-N-(thiazol-2-yl)acetamide;
2-(6-(4-(4-((2-(2,6-dioxopiperidin-3-yl)-6-fluoro-1,3-dioxoisoindolin-5-yl)methyl)piperazin-1-yl)phenyl)-1-oxoisoindolin-2-yl)-2-(5-fluoro-2-hydroxyphenyl)-N-(thiazol-2-yl)acetamide;
2-(6-(4-(4-((2-(2,6-dioxopiperidin-3-yl)-7-fluoro-1,3-dioxoisoindolin-5-yl)methyl)piperazin-1-yl)phenyl)-1-oxoisoindolin-2-yl)-2-(5-fluoro-2-hydroxyphenyl)-N-(thiazol-2-yl)acetamide;
2-(6-(6-(4-((2-(2,6-dioxopiperidin-3-yl)-1,3-dioxoisoindolin-5-yl)methyl)piperazin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((2-(2,6-dioxopiperidin-3-yl)-4-fluoro-1,3-dioxoisoindolin-5-yl)methyl)piperazin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((2-(2,6-dioxopiperidin-3-yl)-6-fluoro-1,3-dioxoisoindolin-5-yl)methyl)piperazin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((2-(2,6-dioxopiperidin-3-yl)-7-fluoro-1,3-dioxoisoindolin-5-yl)methyl)piperazin-1-yl)pyridazin-3-yl)-1-oxoisoindolin-2-yl)-2-(5-fluoro-2-hydroxyphenyl)-N-(thiazol-2-yl)acetamide;
2-(2,6-dioxopiperidin-3-yl)-5-((4-(((R,E)-1¹,2⁶,7-trimethyl-3-oxo-5²,5³-dihydro-1¹H,5¹H-11-oxa-4-aza-5(2,1)-benzo[d]imidazola-2(2,4)-pyridina-1(4,5)-pyrazolacycloundecaphane-5⁶-yl)methyl)piperazin-1-yl)methyl)isoindoline-1,3-dione;
2-(2,6-dioxopiperidin-3-yl)-4-fluoro-5-((4-(((R,E)-1¹,2⁶,7-trimethyl-3-oxo-5²,5³-dihydro-1¹H,5¹H-11-oxa-4-aza-5(2,1)-benzo[d]imidazola-2(2,4)-pyridina-1(4,5)-pyrazolacycloundecaphane-5⁶-yl)methyl)piperazin-1-yl)methyl)isoindoline-1,3-dione;
2-(2,6-dioxopiperidin-3-yl)-4-fluoro-6-((4-(((R,E)-1¹,2⁶,7-trimethyl-3-oxo-5²,5³-dihydro-1¹H,5¹H-11-oxa-4-aza-5(2,1)-benzo[d]imidazola-2(2,4)-pyridina-1(4,5)-pyrazolacycloundecaphane-5⁶-yl)methyl)piperazin-1-yl)methyl)isoindoline-1,3-dione;
2-(2,6-dioxopiperidin-3-yl)-5-fluoro-6-((4-(((R,E)-1¹,2⁶,7-trimethyl-3-oxo-5²,5³-dihydro-1¹H,5¹H-11-oxa-4-aza-5(2,1)-benzo[d]imidazola-2(2,4)-pyridina-1(4,5)-pyrazolacycloundecaphane-5⁶-yl)methyl)piperazin-1-yl)methyl)isoindoline-1,3-dione;
2-(2,6-dioxopiperidin-3-yl)-5-((4-((R,E)-1¹,2⁶,7-trimethyl-3-oxo-5²,5³-dihydro-1¹H,5¹H-11-oxa-4-aza-5(2,1)-benzo[d]imidazola-2(2,4)-pyridina-1(4,5)-pyrazolacycloundecaphane-5⁶-yl)piperazin-1-yl)methyl)isoindoline-1,3-dione;
2-(2,6-dioxopiperidin-3-yl)-4-fluoro-5-((4-((R,E)-1¹,2⁶,7-trimethyl-3-oxo-5²,5³-dihydro-1¹H,5¹H-11-oxa-4-aza-5(2,1)-benzo[d]imidazola-2(2,4)-pyridina-1(4,5)-pyrazolacycloundecaphane-5⁶-yl)piperazin-1-yl)methyl)isoindoline-1,3-dione;
2-(2,6-dioxopiperidin-3-yl)-5-fluoro-6-((4-((R,E)-1¹,2⁶,7-trimethyl-3-oxo-5²,5³-dihydro-1¹H,5¹H-11-oxa-4-aza-5(2,1)-benzo[d]imidazola-2(2,4)-pyridina-1(4,5)-pyrazolacycloundecaphane-5⁶-yl)piperazin-1-yl)methyl)isoindoline-1,3-dione;
2-(2,6-dioxopiperidin-3-yl)-4-fluoro-6-((4-((R,E)-1¹,2⁶,7-trimethyl-3-oxo-5²,5³-dihydro-1¹H,5¹H-11-oxa-4-aza-5(2,1)-benzo[d]imidazola-2(2,4)-pyridina-1(4,5)-pyrazolacycloundecaphane-5⁶-yl)piperazin-1-yl)methyl)isoindoline-1,3-dione;
5-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)-2-(2,6-dioxopiperidin-3-yl)isoindoline-1,3-dione;
5-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-l-yl)methyl)-2-(2,6-dioxopiperidin-3-yl)-4-fluoroisoindoline-1,3-dione;
5-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-l-yl)methyl)-2-(2,6-dioxopiperidin-3-yl)-6-fluoroisoindoline-1,3-dione;
6-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)-2-(2,6-dioxopiperidin-3-yl)-4-fluoroisoindoline-1,3-dione;
5-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)-2-(2,6-dioxopiperidin-3-yl)isoindoline-1,3-dione;
5-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)-2-(2,6-dioxopiperidin-3-yl)-4-fluoroisoindoline-1,3-dione;
5-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)-2-(2,6-dioxopiperidin-3-yl)-6-fluoroisoindoline-1,3-dione;
6-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)-2-(2,6-dioxopiperidin-3-yl)-4-fluoroisoindoline-1,3-dione;
5-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)-2-(2,6-dioxopiperidin-3-yl)isoindoline-1,3-dione;
5-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)-2-(2,6-dioxopiperidin-3-yl)-4-fluoroisoindoline-1,3-dione;
5-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)-2-(2,6-dioxopiperidin-3-yl)-6-fluoroisoindoline-1,3-dione;
6-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)-2-(2,6-dioxopiperidin-3-yl)-4-fluoroisoindoline-1,3-dione;
5-((4-(4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)piperidin-1-yl)methyl)-2-(2,6-dioxopiperidin-3-yl)isoindoline-1,3-dione;
5-((4-(4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)piperidin-1-yl)methyl)-2-(2,6-dioxopiperidin-3-yl)-4-fluoroisoindoline-1,3-dione;
5-((4-(4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)piperidin-1-yl)methyl)-2-(2,6-dioxopiperidin-3-yl)-6-fluoroisoindoline-1,3-dione;
6-((4-(4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)piperidin-1-yl)methyl)-2-(2,6-dioxopiperidin-3-yl)-4-fluoroisoindoline-1,3-dione;
2-(2,6-dioxopiperidin-3-yl)-5-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)isoindoline-1,3-dione;
2-(2,6-dioxopiperidin-3-yl)-4-fluoro-5-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)isoindoline-1,3-dione;
2-(2,6-dioxopiperidin-3-yl)-5-fluoro-6-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)isoindoline-1,3-dione;
2-(2,6-dioxopiperidin-3-yl)-4-fluoro-6-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)isoindoline-1,3-dione;
2-(2,6-dioxopiperidin-3-yl)-5-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)isoindoline-1,3-dione;
2-(2,6-dioxopiperidin-3-yl)-4-fluoro-5-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)isoindoline-1,3-dione;
2-(2,6-dioxopiperidin-3-yl)-5-fluoro-6-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)isoindoline-1,3-dione;
2-(2,6-dioxopiperidin-3-yl)-4-fluoro-6-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)isoindoline-1,3-dione;
2-(2,6-dioxopiperidin-3-yl)-5-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)isoindoline-1,3-dione;
2-(2,6-dioxopiperidin-3-yl)-4-fluoro-5-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)isoindoline-1,3-dione;
2-(2,6-dioxopiperidin-3-yl)-5-fluoro-6-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)isoindoline-1,3-dione;
2-(2,6-dioxopiperidin-3-yl)-4-fluoro-6-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)isoindoline-1,3-dione;
2-(2,6-dioxopiperidin-3-yl)-5-((4-(4-(1-isopropyl-6-((2-(4-methoxypiperidin-1-yl)pyrimidin-4-yl)amino)-1H-pyrazolo[4,3-c]pyridin-3-yl)piperazin-1-yl)piperidin-1-yl)methyl)isoindoline-1,3-dione;
2-(2,6-dioxopiperidin-3-yl)-4-fluoro-5-((4-(4-(1-isopropyl-6-((2-(4-methoxypiperidin-1-yl)pyrimidin-4-yl)amino)-1H-pyrazolo[4,3-c]pyridin-3-yl)piperazin-1-yl)piperidin-1-yl)methyl)isoindoline-1,3-dione;
2-(2,6-dioxopiperidin-3-yl)-5-fluoro-6-((4-(4-(1-isopropyl-6-((2-(4-methoxypiperidin-1-yl)pyrimidin-4-yl)amino)-1H-pyrazolo[4,3-c]pyridin-3-yl)piperazin-1-yl)piperidin-1-yl)methyl)isoindoline-1,3-dione;
2-(2,6-dioxopiperidin-3-yl)-4-fluoro-6-((4-(4-(1-isopropyl-6-((2-(4-methoxypiperidin-1-yl)pyrimidin-4-yl)amino)-1H-pyrazolo[4,3-c]pyridin-3-yl)piperazin-1-yl)piperidin-1-yl)methyl)isoindoline-1,3-dione;
N-(5-(((5-(tert-butyl)oxazol-2-yl)methyl)thio)thiazol-2-yl)-1-((2-(2,6-dioxopiperidin-3-yl)-1,3-dioxoisoindolin-5-yl)methyl)piperidine-4-carboxamide;
N-(5-(((5-(tert-butyl)oxazol-2-yl)methyl)thio)thiazol-2-yl)-1-((2-(2,6-dioxopiperidin-3-yl)-4-fluoro-1,3-dioxoisoindolin-5-yl)methyl)piperidine-4-carboxamide;
N-(5-(((5-(tert-butyl)oxazol-2-yl)methyl)thio)thiazol-2-yl)-1-((2-(2,6-dioxopiperidin-3-yl)-6-fluoro-1,3-dioxoisoindolin-5-yl)methyl)piperidine-4-carboxamide;
N-(5-(((5-(tert-butyl)oxazol-2-yl)methyl)thio)thiazol-2-yl)-1-((2-(2,6-dioxopiperidin-3-yl)-7-fluoro-1,3-dioxoisoindolin-5-yl)methyl)piperidine-4-carboxamide;
N-(5-(((5-(tert-butyl)oxazol-2-yl)methyl)thio)thiazol-2-yl)-1'-((2-(2,6-dioxopiperidin-3-yl)-1,3-dioxoisoindolin-5-yl)methyl)-[1,4'-bipiperidine]-4-carboxamide;
N-(5-(((5-(tert-butyl)oxazol-2-yl)methyl)thio)thiazol-2-yl)-1'-((2-(2,6-dioxopiperidin-3-yl)-4-fluoro-1,3-dioxoisoindolin-5-yl)methyl)-[1,4'-bipiperidine]-4-carboxamide;
N-(5-(((5-(tert-butyl)oxazol-2-yl)methyl)thio)thiazol-2-yl)-1'-((2-(2,6-dioxopiperidin-3-yl)-6-fluoro-1,3-dioxoisoindolin-5-yl)methyl)-[1,4'-bipiperidine]-4-carboxamide;
N-(5-(((5-(tert-butyl)oxazol-2-yl)methyl)thio)thiazol-2-yl)-1'-((2-(2,6-dioxopiperidin-3-yl)-7-fluoro-1,3-dioxoisoindolin-5-yl)methyl)-[1,4'-bipiperidine]-4-carboxamide;
N-(5-(((5-(tert-butyl)oxazol-2-yl)methyl)thio)thiazol-2-yl)-4-(1-((2-(2,6-dioxopiperidin-3-yl)-1,3-dioxoisoindolin-5-yl)methyl)piperidin-4-yl)piperazine-1-carboxamide;
N-(5-(((5-(tert-butyl)oxazol-2-yl)methyl)thio)thiazol-2-yl)-4-(1-((2-(2,6-dioxopiperidin-3-yl)-4-fluoro-1,3-dioxoisoindolin-5-yl)methyl)piperidin-4-yl)piperazine-1-carboxamide;
N-(5-(((5-(tert-butyl)oxazol-2-yl)methyl)thio)thiazol-2-yl)-4-(1-((2-(2,6-dioxopiperidin-3-yl)-6-fluoro-1,3-dioxoisoindolin-5-yl)methyl)piperidin-4-yl)piperazine-1-carboxamide;
N-(5-(((5-(tert-butyl)oxazol-2-yl)methyl)thio)thiazol-2-yl)-4-(1-((2-(2,6-dioxopiperidin-3-yl)-7-fluoro-1,3-dioxoisoindolin-5-yl)methyl)piperidin-4-yl)piperazine-1-carboxamide;
4-(2,6-dichlorobenzamido)-N-(1-((2-(2,6-dioxopiperidin-3-yl)-1,3-dioxoisoindolin-5-yl)methyl)piperidin-4-yl)-1H-pyrazole-3-carboxamide;
4-(2,6-dichlorobenzamido)-N-(1-((2-(2,6-dioxopiperidin-3-yl)-4-fluoro-1,3-dioxoisoindolin-5-yl)methyl)piperidin-4-yl)-1H-pyrazole-3-carboxamide;
4-(2,6-dichlorobenzamido)-N-(1-((2-(2,6-dioxopiperidin-3-yl)-6-fluoro-1,3-dioxoisoindolin-5-yl)methyl)piperidin-4-yl)-1H-pyrazole-3-carboxamide;
4-(2,6-dichlorobenzamido)-N-(1-((2-(2,6-dioxopiperidin-3-yl)-7-fluoro-1,3-dioxoisoindolin-5-yl)methyl)piperidin-4-yl)-1H-pyrazole-3-carboxamide;
N-(5-chloro-4-(5,5-dimethyl-5,6-dihydro-4H-pyrrolo[1,2-b]pyrazol-3-yl)pyridin-2-yl)-1-((2-(2,6-dioxopiperidin-3-yl)-1,3-dioxoisoindolin-5-yl)methyl)piperidine-4-carboxamide;
N-(5-chloro-4-(5,5-dimethyl-5,6-dihydro-4H-pyrrolo[1,2-b]pyrazol-3-yl)pyridin-2-yl)-1-((2-(2,6-dioxopiperidin-3-yl)-4-fluoro-1,3-dioxoisoindolin-5-yl)methyl)piperidine-4-carboxamide;
N-(5-chloro-4-(5,5-dimethyl-5,6-dihydro-4H-pyrrolo[1,2-b]pyrazol-3-yl)pyridin-2-yl)-1-((2-(2,6-dioxopiperidin-3-yl)-6-fluoro-1,3-dioxoisoindolin-5-yl)methyl)piperidine-4-carboxamide;
N-(5-chloro-4-(5,5-dimethyl-5,6-dihydro-4H-pyrrolo[1,2-b]pyrazol-3-yl)pyridin-2-yl)-1-((2-(2,6-dioxopiperidin-3-yl)-7-fluoro-1,3-dioxoisoindolin-5-yl)methyl)piperidine-4-carboxamide;
N-(5-cyclobutyl-1H-pyrazol-3-yl)-2-(4-((2-(2,6-dioxopiperidin-3-yl)-1,3-dioxoisoindolin-5-yl)methoxy)phenyl)acetamide;
N-(5-cyclobutyl-1H-pyrazol-3-yl)-2-(4-((2-(2,6-dioxopiperidin-3-yl)-4-fluoro-1,3-dioxoisoindolin-5-yl)methoxy)phenyl)acetamide;
N-(5-cyclobutyl-1H-pyrazol-3-yl)-2-(4-((2-(2,6-dioxopiperidin-3-yl)-6-fluoro-1,3-dioxoisoindolin-5-yl)methoxy)phenyl)acetamide;
N-(5-cyclobutyl-1H-pyrazol-3-yl)-2-(4-((2-(2,6-dioxopiperidin-3-yl)-7-fluoro-1,3-dioxoisoindolin-5-yl)methoxy)phenyl)acetamide;
5-((4-((5-chloro-4-(5-(cyclopropylmethyl)-1-methyl-1H-pyrazol-4-yl)pyrimidin-2-yl)amino)piperidin-1-yl)methyl)-2-(2,6-dioxopiperidin-3-yl)isoindoline-1,3-dione;
5-((4-((5-chloro-4-(5-(cyclopropylmethyl)-1-methyl-1H-pyrazol-4-yl)pyrimidin-2-yl)amino)piperidin-1-yl)methyl)-2-(2,6-dioxopiperidin-3-yl)-4-fluoroisoindoline-1,3-dione;
5-((4-((5-chloro-4-(5-(cyclopropylmethyl)-1-methyl-1H-pyrazol-4-yl)pyrimidin-2-yl)amino)piperidin-1-yl)methyl)-2-(2,6-dioxopiperidin-3-yl)-6-fluoroisoindoline-1,3-dione;
6-((4-((5-chloro-4-(5-(cyclopropylmethyl)-1-methyl-1H-pyrazol-4-yl)pyrimidin-2-yl)amino)piperidin-1-yl)methyl)-2-(2,6-dioxopiperidin-3-yl)-4-fluoroisoindoline-1,3-dione;
4-((2-(2,6-dioxopiperidin-3-yl)-1,3-dioxoisoindolin-5-yl)methyl)-N-(4-(((5-(((S)-1-hydroxybutan-2-yl)amino)-3-isopropylpyrazolo[1,5-a]pyrimidin-7-yl)amino)methyl)phenyl)piperazine-1-carboxamide;
4-((2-(2,6-dioxopiperidin-3-yl)-4-fluoro-1,3-dioxoisoindolin-5-yl)methyl)-N-(4-(((5-(((S)-1-hydroxybutan-2-yl)amino)-3-isopropylpyrazolo[1,5-a]pyrimidin-7-yl)amino)methyl)phenyl)piperazine-1 -carboxamide;
4-((2-(2,6-dioxopiperidin-3-yl)-6-fluoro-1,3-dioxoisoindolin-5-yl)methyl)-N-(4-(((5-(((S)-1-hydroxybutan-2-yl)amino)-3-isopropylpyrazolo[1,5-a]pyrimidin-7-yl)amino)methyl)phenyl)piperazine-1 -carboxamide;
4-((2-(2,6-dioxopiperidin-3-yl)-7-fluoro-1,3-dioxoisoindolin-5-yl)methyl)-N-(4-(((5-(((S)-1-hydroxybutan-2-yl)amino)-3-isopropylpyrazolo[1,5-a]pyrimidin-7-yl)amino)methyl)phenyl)piperazine-1 -carboxamide;
4-(((4-(5-chloro-2-((1-((2-(2,6-dioxopiperidin-3-yl)-1,3-dioxoisoindolin-5-yl)methyl)piperidin-4-yl)amino)pyridin-4-yl)thiazol-2-yl)amino)methyl)tetrahydro-2H-pyran-4-carbonitrile;
4-(((4-(5-chloro-2-((1-((2-(2,6-dioxopiperidin-3-yl)-4-fluoro-1,3-dioxoisoindolin-5-yl)methyl)piperidin-4-yl)amino)pyridin-4-yl)thiazol-2-yl)amino)methyl)tetrahydro-2H-pyran-4-carbonitrile;
4-(((4-(5-chloro-2-((1-((2-(2,6-dioxopiperidin-3-yl)-6-fluoro-1,3-dioxoisoindolin-5-yl)methyl)piperidin-4-yl)amino)pyridin-4-yl)thiazol-2-yl)amino)methyl)tetrahydro-2H-pyran-4-carbonitrile;
4-(((4-(5-chloro-2-((1-((2-(2,6-dioxopiperidin-3-yl)-7-fluoro-1,3-dioxoisoindolin-5-yl)methyl)piperidin-4-yl)amino)pyridin-4-yl)thiazol-2-yl)amino)methyl)tetrahydro-2H-pyran-4-carbonitrile;
4-(((5'-chloro-2'-((1-((2-(2,6-dioxopiperidin-3-yl)-1,3-dioxoisoindolin-5-yl)methyl)piperidin-4-yl)amino)-[2,4'-bipyridin]-6-yl)amino)methyl)tetrahydro-2H-pyran-4-carbonitrile;
4-(((5'-chloro-2'-((1-((2-(2,6-dioxopiperidin-3-yl)-4-fluoro-1,3-dioxoisoindolin-5-yl)methyl)piperidin-4-yl)amino)-[2,4'-bipyridin]-6-yl)amino)methyl)tetrahydro-2H-pyran-4-carbonitrile;
4-(((5'-chloro-2'-((1-((2-(2,6-dioxopiperidin-3-yl)-6-fluoro-1,3-dioxoisoindolin-5-yl)methyl)piperidin-4-yl)amino)-[2,4'-bipyridin]-6-yl)amino)methyl)tetrahydro-2H-pyran-4-carbonitrile;
4-(((5'-chloro-2'-((1-((2-(2,6-dioxopiperidin-3-yl)-7-fluoro-1,3-dioxoisoindolin-5-yl)methyl)piperidin-4-yl)amino)-[2,4'-bipyridin]-6-yl)amino)methyl)tetrahydro-2H-pyran-4-carbonitrile;
3-((4-(2-(((2-(2,6-dioxopiperidin-3-yl)-1,3-dioxoisoindolin-5-yl)methyl)(methyl)amino)-4-methylthiazol-5-yl)-5-fluoropyrimidin-2-yl)amino)benzenesulfonamide;
3-((4-(2-(((2-(2,6-dioxopiperidin-3-yl)-4-fluoro-1,3-dioxoisoindolin-5-yl)methyl)(methyl)amino)-4-methylthiazol-5-yl)-5-fluoropyrimidin-2-yl)amino)benzenesulfonamide;
3-((4-(2-(((2-(2,6-dioxopiperidin-3-yl)-6-fluoro-1,3-dioxoisoindolin-5-yl)methyl)(methyl)amino)-4-methylthiazol-5-yl)-5-fluoropyrimidin-2-yl)amino)benzenesulfonamide;
3-((4-(2-(((2-(2,6-dioxopiperidin-3-yl)-7-fluoro-1,3-dioxoisoindolin-5-yl)methyl)(methyl)amino)-4-methylthiazol-5-yl)-5-fluoropyrimidin-2-yl)amino)benzenesulfonamide;
N-(2-chloro-6-methylphenyl)-2-((6-(((2-(2,6-dioxopiperidin-3-yl)-1,3-dioxoisoindolin-5-yl)methyl)amino)-2-methylpyrimidin-4-yl)amino)thiazole-5-carboxamide;
N-(2-chloro-6-methylphenyl)-2-((6-(((2-(2,6-dioxopiperidin-3-yl)-4-fluoro-1,3-dioxoisoindolin-5-yl)methyl)amino)-2-methylpyrimidin-4-yl)amino)thiazole-5-carboxamide;
N-(2-chloro-6-methylphenyl)-2-((6-(((2-(2,6-dioxopiperidin-3-yl)-6-fluoro-1,3-dioxoisoindolin-5-yl)methyl)amino)-2-methylpyrimidin-4-yl)amino)thiazole-5-carboxamide;
N-(2-chloro-6-methylphenyl)-2-((6-(((2-(2,6-dioxopiperidin-3-yl)-7-fluoro-1,3-dioxoisoindolin-5-yl)methyl)amino)-2-methylpyrimidin-4-yl)amino)thiazole-5-carboxamide;
N-(2-chloro-6-methylphenyl)-2-((6-(4-((2-(2,6-dioxopiperidin-3-yl)-1,3-dioxoisoindolin-5-yl)methyl)piperazin-1-yl)-2-methylpyrimidin-4-yl)amino)thiazole-5-carboxamide;
N-(2-chloro-6-methylphenyl)-2-((6-(4-((2-(2,6-dioxopiperidin-3-yl)-4-fluoro-1,3-dioxoisoindolin-5-yl)methyl)piperazin-1-yl)-2-methylpyrimidin-4-yl)amino)thiazole-5-carboxamide;
N-(2-chloro-6-methylphenyl)-2-((6-(4-((2-(2,6-dioxopiperidin-3-yl)-6-fluoro-1,3-dioxoisoindolin-5-yl)methyl)piperazin-1-yl)-2-methylpyrimidin-4-yl)amino)thiazole-5-carboxamide;
N-(2-chloro-6-methylphenyl)-2-((6-(4-((2-(2,6-dioxopiperidin-3-yl)-7-fluoro-1,3-dioxoisoindolin-5-yl)methyl)piperazin-1-yl)-2-methylpyrimidin-4-yl)amino)thiazole-5-carboxamide;
N-(2-chloro-6-methylphenyl)-2-((6-(4-(((2-(2,6-dioxopiperidin-3-yl)-1,3-dioxoisoindolin-5-yl)methyl)(methyl)amino)piperidin-1-yl)-2-methylpyrimidin-4-yl)amino)thiazole-5-carboxamide;
N-(2-chloro-6-methylphenyl)-2-((6-(4-(((2-(2,6-dioxopiperidin-3-yl)-4-fluoro-1,3-dioxoisoindolin-5-yl)methyl)(methyl)amino)piperidin-1-yl)-2-methylpyrimidin-4-yl)amino)thiazole-5-carboxamide;
N-(2-chloro-6-methylphenyl)-2-((6-(4-(((2-(2,6-dioxopiperidin-3-yl)-6-fluoro-1,3-dioxoisoindolin-5-yl)methyl)(methyl)amino)piperidin-1-yl)-2-methylpyrimidin-4-yl)amino)thiazole-5-carboxamide;
N-(2-chloro-6-methylphenyl)-2-((6-(4-(((2-(2,6-dioxopiperidin-3-yl)-7-fluoro-1,3-dioxoisoindolin-5-yl)methyl)(methyl)amino)piperidin-1-yl)-2-methylpyrimidin-4-yl)amino)thiazole-5-carboxamide;
N-(2-chloro-6-methylphenyl)-2-((6-(4-(4-((2-(2,6-dioxopiperidin-3-yl)-1,3-dioxoisoindolin-5-yl)methyl)piperazin-1-yl)piperidin-1-yl)-2-methylpyrimidin-4-yl)amino)thiazole-5-carboxamide;
N-(2-chloro-6-methylphenyl)-2-((6-(4-(4-((2-(2,6-dioxopiperidin-3-yl)-4-fluoro-1,3-dioxoisoindolin-5-yl)methyl)piperazin-1-yl)piperidin-1-yl)-2-methylpyrimidin-4-yl)amino)thiazole-5-carboxamide;
N-(2-chloro-6-methylphenyl)-2-((6-(4-(4-((2-(2,6-dioxopiperidin-3-yl)-6-fluoro-1,3-dioxoisoindolin-5-yl)methyl)piperazin-1-yl)piperidin-1-yl)-2-methylpyrimidin-4-yl)amino)thiazole-5-carboxamide;
N-(2-chloro-6-methylphenyl)-2-((6-(4-(4-((2-(2,6-dioxopiperidin-3-yl)-7-fluoro-1,3-dioxoisoindolin-5-yl)methyl)piperazin-1-yl)piperidin-1-yl)-2-methylpyrimidin-4-yl)amino)thiazole-5-carboxamide;
4-((4-((2-(2,6-dioxopiperidin-3-yl)-1,3-dioxoisoindolin-5-yl)methyl)piperazin-1-yl)methyl)-N-(4-methyl-3-((4-(pyridin-3-yl)pyrimidin-2-yl)amino)phenyl)benzamide;
4-((4-((2-(2,6-dioxopiperidin-3-yl)-4-fluoro-1,3-dioxoisoindolin-5-yl)methyl)piperazin-1-yl)methyl)-N-(4-methyl-3-((4-(pyridin-3-yl)pyrimidin-2-yl)amino)phenyl)benzamide;
4-((4-((2-(2,6-dioxopiperidin-3-yl)-6-fluoro-1,3-dioxoisoindolin-5-yl)methyl)piperazin-1-yl)methyl)-N-(4-methyl-3-((4-(pyridin-3-yl)pyrimidin-2-yl)amino)phenyl)benzamide;
4-((4-((2-(2,6-dioxopiperidin-3-yl)-7-fluoro-1,3-dioxoisoindolin-5-yl)methyl)piperazin-1-yl)methyl)-N-(4-methyl-3-((4-(pyridin-3-yl)pyrimidin-2-yl)amino)phenyl)benzamide;
4-((((2-(2,6-dioxopiperidin-3-yl)-1,3-dioxoisoindolin-5-yl)methyl)amino)methyl)-N-(4-methyl-3-((4-(pyridin-3-yl)pyrimidin-2-yl)amino)phenyl)benzamide;
4-((((2-(2,6-dioxopiperidin-3-yl)-4-fluoro-1,3-dioxoisoindolin-5-yl)methyl)amino)methyl)-N-(4-methyl-3-((4-(pyridin-3-yl)pyrimidin-2-yl)amino)phenyl)benzamide;
4-((((2-(2, 6-dioxopiperidin-3-yl)-6-fluoro-1, 3-dioxoisoindolin-5-yl)methyl)amino)methyl)-N-(4-methyl-3-((4-(pyridin-3-yl)pyrimidin-2-yl)amino)phenyl)benzamide;
4-((((2-(2,6-dioxopiperidin-3-yl)-7-fluoro-1,3-dioxoisoindolin-5-yl)methyl)amino)methyl)-N-(4-methyl-3-((4-(pyridin-3-yl)pyrimidin-2-yl)amino)phenyl)benzamide;
4-(4-((2-(2,6-dioxopiperidin-3-yl)-1,3-dioxoisoindolin-5-yl)methyl)piperazin-1-yl)-N-(4-methyl-3-((4-(pyridin-3-yl)pyrimidin-2-yl)amino)phenyl)benzamide;
4-(4-((2-(2,6-dioxopiperidin-3-yl)-4-fluoro-1,3-dioxoisoindolin-5-yl)methyl)piperazin-1-yl)-N-(4-methyl-3-((4-(pyridin-3-yl)pyrimidin-2-yl)amino)phenyl)benzamide;
4-(4-((2-(2,6-dioxopiperidin-3-yl)-6-fluoro-1,3-dioxoisoindolin-5-yl)methyl)piperazin-1-yl)-N-(4-methyl-3-((4-(pyridin-3-yl)pyrimidin-2-yl)amino)phenyl)benzamide;
4-(4-((2-(2,6-dioxopiperidin-3-yl)-7-fluoro-1,3-dioxoisoindolin-5-yl)methyl)piperazin-1-yl)-N-(4-methyl-3-((4-(pyridin-3-yl)pyrimidin-2-yl)amino)phenyl)benzamide;
4-(4-(((2-(2,6-dioxopiperidin-3-yl)-1,3-dioxoisoindolin-5-yl)methyl)(methyl)amino)piperidin-1-yl)-N-(4-methyl-3-((4-(pyridin-3-yl)pyrimidin-2-yl)amino)phenyl)benzamide;
4-(4-(((2-(2,6-dioxopiperidin-3-yl)-4-fluoro-1,3-dioxoisoindolin-5-yl)methyl)(methyl)amino)piperidin-1-yl)-N-(4-methyl-3-((4-(pyridin-3-yl)pyrimidin-2-yl)amino)phenyl)benzamide;
4-(4-(((2-(2,6-dioxopiperidin-3-yl)-6-fluoro-1,3-dioxoisoindolin-5-yl)methyl)(methyl)amino)piperidin-1-yl)-N-(4-methyl-3-((4-(pyridin-3-yl)pyrimidin-2-yl)amino)phenyl)benzamide;
4-(4-(((2-(2,6-dioxopiperidin-3-yl)-7-fluoro-1,3-dioxoisoindolin-5-yl)methyl)(methyl)amino)piperidin-1-yl)-N-(4-methyl-3-((4-(pyridin-3-yl)pyrimidin-2-yl)amino)phenyl)benzamide;
4-(4-(4-((2-(2,6-dioxopiperidin-3-yl)-1,3-dioxoisoindolin-5-yl)methyl)piperazin-1-yl)piperidin-1-yl)-N-(4-methyl-3-((4-(pyridin-3-yl)pyrimidin-2-yl)amino)phenyl)benzamide;
4-(4-(4-((2-(2,6-dioxopiperidin-3-yl)-4-fluoro-1,3-dioxoisoindolin-5-yl)methyl)piperazin-1-yl)piperidin-1-yl)-N-(4-methyl-3-((4-(pyridin-3-yl)pyrimidin-2-yl)amino)phenyl)benzamide;
4-(4-(4-((2-(2,6-dioxopiperidin-3-yl)-6-fluoro-1,3-dioxoisoindolin-5-yl)methyl)piperazin-1-yl)piperidin-1-yl)-N-(4-methyl-3-((4-(pyridin-3-yl)pyrimidin-2-yl)amino)phenyl)benzamide;
4-(4-(4-((2-(2,6-dioxopiperidin-3-yl)-7-fluoro-1,3-dioxoisoindolin-5-yl)methyl)piperazin-1-yl)piperidin-1-yl)-N-(4-methyl-3-((4-(pyridin-3-yl)pyrimidin-2-yl)amino)phenyl)benzamide;
4-((2,4-dichloro-5-methoxyphenyl)amino)-7-(3-(4-((2-(2,6-dioxopiperidin-3-yl)-1,3-dioxoisoindolin-5-yl)methyl)piperazin-1-yl)propoxy)-6-methoxyquinoline-3-carbonitrile;
4-((2,4-dichloro-5-methoxyphenyl)amino)-7-(3-(4-((2-(2,6-dioxopiperidin-3-yl)-4-fluoro-1,3-dioxoisoindolin-5-yl)methyl)piperazin-1-yl)propoxy)-6-methoxyquinoline-3-carbonitrile;
4-((2,4-dichloro-5-methoxyphenyl)amino)-7-(3-(4-((2-(2,6-dioxopiperidin-3-yl)-6-fluoro-1,3-dioxoisoindolin-5-yl)methyl)piperazin-1-yl)propoxy)-6-methoxyquinoline-3-carbonitrile;
4-((2,4-dichloro-5-methoxyphenyl)amino)-7-(3-(4-((2-(2,6-dioxopiperidin-3-yl)-7-fluoro-1,3-dioxoisoindolin-5-yl)methyl)piperazin-1-yl)propoxy)-6-methoxyquinoline-3-carbonitrile;
4-((2,4-dichloro-5-methoxyphenyl)amino)-7-((2-(2,6-dioxopiperidin-3-yl)-1,3-dioxoisoindolin-5-yl)methoxy)-6-methoxyquinoline-3-carbonitrile;
4-((2,4-dichloro-5-methoxyphenyl)amino)-7-((2-(2,6-dioxopiperidin-3-yl)-4-fluoro-1,3-dioxoisoindolin-5-yl)methoxy)-6-methoxyquinoline-3-carbonitrile;
4-((2,4-dichloro-5-methoxyphenyl)amino)-7-((2-(2,6-dioxopiperidin-3-yl)-6-fluoro-1,3-dioxoisoindolin-5-yl)methoxy)-6-methoxyquinoline-3-carbonitrile;
4-((2,4-dichloro-5-methoxyphenyl)amino)-7-((2-(2,6-dioxopiperidin-3-yl)-7-fluoro-1,3-dioxoisoindolin-5-yl)methoxy)-6-methoxyquinoline-3-carbonitrile;
N-(4-((4-((2-(2,6-dioxopiperidin-3-yl)-1,3-dioxoisoindolin-5-yl)methyl)piperazin-1-yl)methyl)-3-(trifluoromethyl)phenyl)-3-(imidazo[1,2-b]pyridazin-3-ylethynyl)-4-methylbenzamide;
N-(4-((4-((2-(2,6-dioxopiperidin-3-yl)-4-fluoro-1,3-dioxoisoindolin-5-yl)methyl)piperazin-1-yl)methyl)-3-(trifluoromethyl)phenyl)-3-(imidazo[1,2-b]pyridazin-3-ylethynyl)-4-methylbenzamide;
N-(4-((4-((2-(2,6-dioxopiperidin-3-yl)-6-fluoro-1,3-dioxoisoindolin-5-yl)methyl)piperazin-1-yl)methyl)-3-(trifluoromethyl)phenyl)-3-(imidazo[1,2-b]pyridazin-3-ylethynyl)-4-methylbenzamide;
N-(4-((4-((2-(2,6-dioxopiperidin-3-yl)-7-fluoro-1,3-dioxoisoindolin-5-yl)methyl)piperazin-1-yl)methyl)-3-(trifluoromethyl)phenyl)-3-(imidazo[1,2-b]pyridazin-3-ylethynyl)-4-methylbenzamide;
N-(4-(4-((2-(2,6-dioxopiperidin-3-yl)-1,3-dioxoisoindolin-5-yl)methyl)piperazin-1-yl)-3-(trifluoromethyl)phenyl)-3-(imidazo[1,2-b]pyridazin-3-ylethynyl)-4-methylbenzamide;
N-(4-(4-((2-(2,6-dioxopiperidin-3-yl)-4-fluoro-1,3-dioxoisoindolin-5-yl)methyl)piperazin-1-yl)-3-(trifluoromethyl)phenyl)-3-(imidazo[1,2-b]pyridazin-3-ylethynyl)-4-methylbenzamide;
N-(4-(4-((2-(2,6-dioxopiperidin-3-yl)-6-fluoro-1,3-dioxoisoindolin-5-yl)methyl)piperazin-1-yl)-3-(trifluoromethyl)phenyl)-3-(imidazo[1,2-b]pyridazin-3-ylethynyl)-4-methylbenzamide;
N-(4-(4-((2-(2,6-dioxopiperidin-3-yl)-7-fluoro-1,3-dioxoisoindolin-5-yl)methyl)piperazin-1-yl)-3-(trifluoromethyl)phenyl)-3-(imidazo[1,2-b]pyridazin-3-ylethynyl)-4-methylbenzamide;
N-(4-(4-(1-((2-(2,6-dioxopiperidin-3-yl)-1,3-dioxoisoindolin-5-yl)methyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(1-((2-(2,6-dioxopiperidin-3-yl)-4-fluoro-1,3-dioxoisoindolin-5-yl)methyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(1-((2-(2,6-dioxopiperidin-3-yl)-6-fluoro-1,3-dioxoisoindolin-5-yl)methyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(1-((2-(2,6-dioxopiperidin-3-yl)-7-fluoro-1,3-dioxoisoindolin-5-yl)methyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(((2-(2,6-dioxopiperidin-3-yl)-1,3-dioxoisoindolin-5-yl)methyl)(methyl)amino)piperidin-1-yl)-3-(trifluoromethyl)phenyl)-3-(imidazo[1,2-b]pyridazin-3-ylethynyl)-4-methylbenzamide;
N-(4-(4-(((2-(2,6-dioxopiperidin-3-yl)-4-fluoro-1,3-dioxoisoindolin-5-yl)methyl)(methyl)amino)piperidin-1-yl)-3-(trifluoromethyl)phenyl)-3-(imidazo[1,2-b]pyridazin-3-ylethynyl)-4-methylbenzamide;
N-(4-(4-(((2-(2,6-dioxopiperidin-3-yl)-6-fluoro-1,3-dioxoisoindolin-5-yl)methyl)(methyl)amino)piperidin-1-yl)-3-(trifluoromethyl)phenyl)-3-(imidazo[1,2-b]pyridazin-3-ylethynyl)-4-methylbenzamide;
N-(4-(4-(((2-(2,6-dioxopiperidin-3-yl)-7-fluoro-1,3-dioxoisoindolin-5-yl)methyl)(methyl)amino)piperidin-1-yl)-3-(trifluoromethyl)phenyl)-3-(imidazo[1,2-b]pyridazin-3-ylethynyl)-4-methylbenzamide;
2-(4-((3S)-1-((2-(2,6-dioxopiperidin-3-yl)-1,3-dioxoisoindolin-5-yl)methyl)piperidin-3-yl)phenyl)-2H-indazole-7-carboxamide;
2-(4-((3S)-1-((2-(2,6-dioxopiperidin-3-yl)-4-fluoro-1,3-dioxoisoindolin-5-yl)methyl)piperidin-3-yl)phenyl)-2H-indazole-7-carboxamide;
2-(4-((3S)-1-((2-(2,6-dioxopiperidin-3-yl)-6-fluoro-1,3-dioxoisoindolin-5-yl)methyl)piperidin-3-yl)phenyl)-2H-indazole-7-carboxamide;
2-(4-((3S)-1-((2-(2,6-dioxopiperidin-3-yl)-7-fluoro-1,3-dioxoisoindolin-5-yl)methyl)piperidin-3-yl)phenyl)-2H-indazole-7-carboxamide;
2-(4-(1-((2-(2,6-dioxopiperidin-3-yl)-1,3-dioxoisoindolin-5-yl)methyl)piperidin-4-yl)phenyl)-2H-indazole-7-carboxamide;
2-(4-(1-((2-(2,6-dioxopiperidin-3-yl)-4-fluoro-1,3-dioxoisoindolin-5-yl)methyl)piperidin-4-yl)phenyl)-2H-indazole-7-carboxamide;
2-(4-(1-((2-(2,6-dioxopiperidin-3-yl)-6-fluoro-1,3-dioxoisoindolin-5-yl)methyl)piperidin-4-yl)phenyl)-2H-indazole-7-carboxamide;
2-(4-(1-((2-(2,6-dioxopiperidin-3-yl)-7-fluoro-1,3-dioxoisoindolin-5-yl)methyl)piperidin-4-yl)phenyl)-2H-indazole-7-carboxamide;
2-(4-(4-((2-(2,6-dioxopiperidin-3-yl)-1,3-dioxoisoindolin-5-yl)methyl)piperazin-1-yl)phenyl)-2H-indazole-7-carboxamide;
2-(4-(4-((2-(2,6-dioxopiperidin-3-yl)-4-fluoro-1,3-dioxoisoindolin-5-yl)methyl)piperazin-1-yl)phenyl)-2H-indazole-7-carboxamide;
2-(4-(4-((2-(2,6-dioxopiperidin-3-yl)-6-fluoro-1,3-dioxoisoindolin-5-yl)methyl)piperazin-1-yl)phenyl)-2H-indazole-7-carboxamide;
2-(4-(4-((2-(2,6-dioxopiperidin-3-yl)-7-fluoro-1,3-dioxoisoindolin-5-yl)methyl)piperazin-1-yl)phenyl)-2H-indazole-7-carboxamide;
2-(2,6-dioxopiperidin-3-yl)-5-((4-(2-fluoro-5-((4-oxo-3,4-dihydrophthalazin-1-yl)methyl)benzoyl)piperazin-1-yl)methyl)isoindoline-1,3-dione;
2-(2,6-dioxopiperidin-3-yl)-4-fluoro-5-((4-(2-fluoro-5-((4-oxo-3,4-dihydrophthalazin-1-yl)methyl)benzoyl)piperazin-1-yl)methyl)isoindoline-1,3-dione;
2-(2,6-dioxopiperidin-3-yl)-5-fluoro-6-((4-(2-fluoro-5-((4-oxo-3,4-dihydrophthalazin-1-yl)methyl)benzoyl)piperazin-1-yl)methyl)isoindoline-1,3-dione;
2-(2,6-dioxopiperidin-3-yl)-4-fluoro-6-((4-(2-fluoro-5-((4-oxo-3,4-dihydrophthalazin-1-yl)methyl)benzoyl)piperazin-1-yl)methyl)isoindoline-1,3-dione;
2-(2,6-dioxopiperidin-3-yl)-5-(((4-(8-fluoro-1-oxo-2,3,4,6-tetrahydro-1H-azepino[5,4,3-cd]indol-5-yl)benzyl)(methyl)amino)methyl)isoindoline-1,3-dione;
2-(2,6-dioxopiperidin-3-yl)-4-fluoro-5-(((4-(8-fluoro-1-oxo-2,3,4,6-tetrahydro-1H-azepino[5,4,3-cd]indol-5-yl)benzyl)(methyl)amino)methyl)isoindoline-1,3-dione;
2-(2,6-dioxopiperidin-3-yl)-5-fluoro-6-(((4-(8-fluoro-1-oxo-2,3,4,6-tetrahydro-1H-azepino[5,4,3-cd]indol-5-yl)benzyl)(methyl)amino)methyl)isoindoline-1,3-dione;
2-(2,6-dioxopiperidin-3-yl)-4-fluoro-6-(((4-(8-fluoro-1-oxo-2,3,4,6-tetrahydro-1H-azepino[5,4,3-cd]indol-5-yl)benzyl)(methyl)amino)methyl)isoindoline-1,3-dione;
2-(2,6-dioxopiperidin-3-yl)-5-((4-(2-fluoro-5-((8S,9R)-5-fluoro-9-(1-methyl-1H-1,2,4-triazol-5-yl)-3-oxo-2,7,8,9-tetrahydro-3H-pyrido[4,3,2-de]phthalazin-8-yl)phenyl)piperazin-1-yl)methyl)isoindoline-1,3-dione;
2-(2,6-dioxopiperidin-3-yl)-4-fluoro-5-((4-(2-fluoro-5-((8S,9R)-5-fluoro-9-(1-methyl-1H-1,2,4-triazol-5-yl)-3-oxo-2,7,8,9-tetrahydro-3H-pyrido[4,3,2-de]phthalazin-8-yl)phenyl)piperazin-1-yl)methyl)isoindoline-1,3-dione;
2-(2,6-dioxopiperidin-3-yl)-5-fluoro-6-((4-(2-fluoro-5-((8S,9R)-5-fluoro-9-(1-methyl-1H-1,2,4-triazol-5-yl)-3-oxo-2,7,8,9-tetrahydro-3H-pyrido[4,3,2-de]phthalazin-8-yl)phenyl)piperazin-1-yl)methyl)isoindoline-1,3-dione;
2-(2,6-dioxopiperidin-3-yl)-4-fluoro-6-((4-(2-fluoro-5-((8S,9R)-5-fluoro-9-(1-methyl-1H-1,2,4-triazol-5-yl)-3-oxo-2,7,8,9-tetrahydro-3H-pyrido[4,3,2-de]phthalazin-8-yl)phenyl)piperazin-1-yl)methyl)isoindoline-1,3-dione;
2-(2,6-dioxopiperidin-3-yl)-5-((4-((2-fluoro-5-((8S,9R)-5-fluoro-9-(1-methyl-1H-1,2,4-triazol-5-yl)-3-oxo-2,7,8,9-tetrahydro-3H-pyrido[4,3,2-de]phthalazin-8-yl)phenyl)ethynyl)piperidin-1-yl)methyl)isoindoline-1,3-dione;
2-(2,6-dioxopiperidin-3-yl)-4-fluoro-5-((4-((2-fluoro-5-((8S,9R)-5-fluoro-9-(1-methyl-1H-1,2,4-triazol-5-yl)-3-oxo-2,7,8,9-tetrahydro-3H-pyrido[4,3,2-de]phthalazin-8-yl)phenyl)ethynyl)piperidin-1 -yl)methyl)isoindoline-1,3 -dione;
2-(2,6-dioxopiperidin-3-yl)-5-fluoro-6-((4-((2-fluoro-5-((8S,9R)-5-fluoro-9-(1-methyl-1H-1,2,4-triazol-5-yl)-3-oxo-2,7,8,9-tetrahydro-3H-pyrido[4,3,2-de]phthalazin-8-yl)phenyl)ethynyl)piperidin-1 -yl)methyl)isoindoline-1,3 -dione;
2-(2,6-dioxopiperidin-3-yl)-4-fluoro-6-((4-((2-fluoro-5-((8S,9R)-5-fluoro-9-(1-methyl-1H-1,2,4-triazol-5-yl)-3-oxo-2,7,8,9-tetrahydro-3H-pyrido[4,3,2-de]phthalazin-8-yl)phenyl)ethynyl)piperidin-1 -yl)methyl)isoindoline-1,3 -dione;
7-cyclopentyl-2-((5-(4-((2-(2,6-dioxopiperidin-3-yl)-1,3-dioxoisoindolin-5-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-((2-(2,6-dioxopiperidin-3-yl)-4-fluoro-1,3-dioxoisoindolin-5-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-((2-(2,6-dioxopiperidin-3-yl)-6-fluoro-1,3-dioxoisoindolin-5-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-((2-(2,6-dioxopiperidin-3-yl)-7-fluoro-1,3-dioxoisoindolin-5-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-(((2-(2,6-dioxopiperidin-3-yl)-1,3-dioxoisoindolin-5-yl)methyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-(((2-(2,6-dioxopiperidin-3-yl)-4-fluoro-1,3-dioxoisoindolin-5-yl)methyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-(((2-(2,6-dioxopiperidin-3-yl)-6-fluoro-1,3-dioxoisoindolin-5-yl)methyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-(((2-(2,6-dioxopiperidin-3-yl)-7-fluoro-1,3-dioxoisoindolin-5-yl)methyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-(4-((2-(2,6-dioxopiperidin-3-yl)-1,3-dioxoisoindolin-5-yl)methyl)piperazin-1-yl)piperidin-1-yl)pyridin-2-yl)amino)-N,N-dimethyl-7H-pyrrolo[2,3-d]pyrimidine-6-carboxamide;
7-cyclopentyl-2-((5-(4-(4-((2-(2,6-dioxopiperidin-3-yl)-4-fluoro-1,3-dioxoisoindolin-5-yl)methyl)piperazin-1-yl)piperidin-1-yl)pyridin-2-yl)amino)-N,N-dimethyl-7H-pyrrolo[2,3-d]pyrimidine-6-carboxamide;
7-cyclopentyl-2-((5-(4-(4-((2-(2,6-dioxopiperidin-3-yl)-6-fluoro-1,3-dioxoisoindolin-5-yl)methyl)piperazin-1-yl)piperidin-1-yl)pyridin-2-yl)amino)-N,N-dimethyl-7H-pyrrolo[2,3-d]pyrimidine-6-carboxamide;
7-cyclopentyl-2-((5-(4-(4-((2-(2,6-dioxopiperidin-3-yl)-7-fluoro-1,3-dioxoisoindolin-5-yl)methyl)piperazin-1-yl)piperidin-1-yl)pyridin-2-yl)amino)-N,N-dimethyl-7H-pyrrolo[2,3-d]pyrimidine-6-carboxamide;
7-cyclopentyl-2-((5-(4-(1-((2-(2,6-dioxopiperidin-3-yl)-1,3-dioxoisoindolin-5-yl)methyl)piperidin-4-yl)piperazin-1-yl)pyridin-2-yl)amino)-N,N-dimethyl-7H-pyrrolo[2,3-d]pyrimidine-6-carboxamide;
7-cyclopentyl-2-((5-(4-(1-((2-(2,6-dioxopiperidin-3-yl)-4-fluoro-1,3-dioxoisoindolin-5-yl)methyl)piperidin-4-yl)piperazin-1-yl)pyridin-2-yl)amino)-N,N-dimethyl-7H-pyrrolo[2,3-d]pyrimidine-6-carboxamide;
7-cyclopentyl-2-((5-(4-(1-((2-(2,6-dioxopiperidin-3-yl)-6-fluoro-1,3-dioxoisoindolin-5-yl)methyl)piperidin-4-yl)piperazin-1-yl)pyridin-2-yl)amino)-N,N-dimethyl-7H-pyrrolo[2,3-d]pyrimidine-6-carboxamide;
7-cyclopentyl-2-((5-(4-(1-((2-(2,6-dioxopiperidin-3-yl)-7-fluoro-1,3-dioxoisoindolin-5-yl)methyl)piperidin-4-yl)piperazin-1-yl)pyridin-2-yl)amino)-N,N-dimethyl-7H-pyrrolo[2,3-d]pyrimidine-6-carboxamide;
2-(2,6-dioxopiperidin-3-yl)-5-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)isoindoline-1,3-dione;
2-(2,6-dioxopiperidin-3-yl)-4-fluoro-5-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)isoindoline-1,3-dione;
2-(2,6-dioxopiperidin-3-yl)-5-fluoro-6-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)isoindoline-1,3-dione;
2-(2,6-dioxopiperidin-3-yl)-4-fluoro-6-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)isoindoline-1,3-dione;
5-((4-(difluoro(6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-2-(2,6-dioxopiperidin-3-yl)isoindoline-1,3-dione;
5-((4-(difluoro(6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-2-(2,6-dioxopiperidin-3-yl)-4-fluoroisoindoline-1,3-dione;
5-((4-(difluoro(6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-2-(2,6-dioxopiperidin-3-yl)-6-fluoroisoindoline-1,3-dione;
6-((4-(difluoro(6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-2-(2,6-dioxopiperidin-3-yl)-4-fluoroisoindoline-1,3-dione;
2-(2,6-dioxopiperidin-3-yl)-5-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)isoindoline-1,3-dione;
2-(2,6-dioxopiperidin-3-yl)-4-fluoro-5-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)isoindoline-1,3-dione;
2-(2,6-dioxopiperidin-3-yl)-5-fluoro-6-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)isoindoline-1,3-dione;
2-(2,6-dioxopiperidin-3-yl)-4-fluoro-6-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)isoindoline-1,3-dione;
2-(2,6-dioxopiperidin-3-yl)-5-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)isoindoline-1,3-dione;
2-(2,6-dioxopiperidin-3-yl)-4-fluoro-5-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)isoindoline-1,3-dione;
2-(2,6-dioxopiperidin-3-yl)-5-fluoro-6-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)isoindoline-1,3-dione;
2-(2,6-dioxopiperidin-3-yl)-4-fluoro-6-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)isoindoline-1,3-dione;
2-(2,6-dioxopiperidin-3-yl)-5-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)isoindoline-1,3-dione;
2-(2,6-dioxopiperidin-3-yl)-4-fluoro-5-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)isoindoline-1,3-dione;
2-(2,6-dioxopiperidin-3-yl)-5-fluoro-6-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)isoindoline-1,3-dione;
2-(2,6-dioxopiperidin-3-yl)-4-fluoro-6-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)isoindoline-1,3-dione;
2-(2,6-dioxopiperidin-3-yl)-5-((4-(4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)piperidin-1-yl)methyl)isoindoline-1,3-dione;
2-(2,6-dioxopiperidin-3-yl)-4-fluoro-5-((4-(4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)piperidin-1-yl)methyl)isoindoline-1,3-dione;
2-(2,6-dioxopiperidin-3-yl)-5-fluoro-6-((4-(4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)piperidin-1-yl)methyl)isoindoline-1,3-dione;
2-(2,6-dioxopiperidin-3-yl)-4-fluoro-6-((4-(4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)piperidin-1-yl)methyl)isoindoline-1,3-dione;
5-((4-(difluoro(6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-2-(2,6-dioxopiperidin-3-yl)isoindoline-1,3-dione;
5-((4-(difluoro(6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-2-(2,6-dioxopiperidin-3-yl)-4-fluoroisoindoline-1,3-dione;
5-((4-(difluoro(6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-2-(2,6-dioxopiperidin-3-yl)-6-fluoroisoindoline-1,3-dione;
6-((4-(difluoro(6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-2-(2,6-dioxopiperidin-3-yl)-4-fluoroisoindoline-1,3-dione;
5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-2-(2,6-dioxopiperidin-3-yl)isoindoline-1,3-dione;
5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-2-(2,6-dioxopiperidin-3-yl)-4-fluoroisoindoline-1,3-dione;
5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-2-(2,6-dioxopiperidin-3-yl)-6-fluoroisoindoline-1,3-dione;
6-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-2-(2,6-dioxopiperidin-3-yl)-4-fluoroisoindoline-1,3-dione;
5-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)-2-(2,6-dioxopiperidin-3-yl)isoindoline-1,3-dione;
5-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)-2-(2,6-dioxopiperidin-3-yl)-4-fluoroisoindoline-1,3-dione;
5-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)-2-(2,6-dioxopiperidin-3-yl)-6-fluoroisoindoline-1,3-dione;
6-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)-2-(2,6-dioxopiperidin-3-yl)-4-fluoroisoindoline-1,3-dione;
5-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)-2-(2,6-dioxopiperidin-3-yl)isoindoline-1,3-dione;
5-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)-2-(2,6-dioxopiperidin-3-yl)-4-fluoroisoindoline-1,3-dione;
5-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)-2-(2,6-dioxopiperidin-3-yl)-6-fluoroisoindoline-1,3-dione;
6-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)-2-(2,6-dioxopiperidin-3-yl)-4-fluoroisoindoline-1,3-dione;
5-((4-(4-(6-((6-acetyl-8-cyclopentyl-5-methyl-7-oxo-7,8-dihydropyrido[2,3-d]pyrimidin-2-yl)amino)pyridin-3-yl)piperazin-1-yl)piperidin-1-yl)methyl)-2-(2,6-dioxopiperidin-3-yl)isoindoline-1,3-dione;
5-((4-(4-(6-((6-acetyl-8-cyclopentyl-5-methyl-7-oxo-7,8-dihydropyrido[2,3-d]pyrimidin-2-yl)amino)pyridin-3-yl)piperazin-1-yl)piperidin-1-yl)methyl)-2-(2,6-dioxopiperidin-3-yl)-4-fluoroisoindoline-1,3-dione;
5-((4-(4-(6-((6-acetyl-8-cyclopentyl-5-methyl-7-oxo-7,8-dihydropyrido[2,3-d]pyrimidin-2-yl)amino)pyridin-3-yl)piperazin-1-yl)piperidin-1-yl)methyl)-2-(2,6-dioxopiperidin-3-yl)-6-fluoroisoindoline-1,3-dione;
6-((4-(4-(6-((6-acetyl-8-cyclopentyl-5-methyl-7-oxo-7,8-dihydropyrido[2,3-d]pyrimidin-2-yl)amino)pyridin-3-yl)piperazin-1-yl)piperidin-1-yl)methyl)-2-(2,6-dioxopiperidin-3-yl)-4-fluoroisoindoline-1,3-dione;
3-((4-(4-(4-((2-(2,6-dioxopiperidin-3-yl)-1,3-dioxoisoindolin-5-yl)methyl)piperazin-1-yl)piperidin-1-yl)-3-methoxyphenyl)amino)-6-ethyl-5-((tetrahydro-2H-pyran-4-yl)amino)pyrazine-2-carboxamide;
3-((4-(4-(4-((2-(2,6-dioxopiperidin-3-yl)-4-fluoro-1,3-dioxoisoindolin-5-yl)methyl)piperazin-1-yl)piperidin-1-yl)-3-methoxyphenyl)amino)-6-ethyl-5-((tetrahydro-2H-pyran-4-yl)amino)pyrazine-2-carboxamide;
3-((4-(4-(4-((2-(2,6-dioxopiperidin-3-yl)-6-fluoro-1,3-dioxoisoindolin-5-yl)methyl)piperazin-1-yl)piperidin-1-yl)-3-methoxyphenyl)amino)-6-ethyl-5-((tetrahydro-2H-pyran-4-yl)amino)pyrazine-2-carboxamide;
3-((4-(4-(4-((2-(2,6-dioxopiperidin-3-yl)-7-fluoro-1,3-dioxoisoindolin-5-yl)methyl)piperazin-1-yl)piperidin-1-yl)-3-methoxyphenyl)amino)-6-ethyl-5-((tetrahydro-2H-pyran-4-yl)amino)pyrazine-2-carboxamide;
5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-2-(2,6-dioxopiperidin-3-yl)isoindoline-1,3-dione;
5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-2-(2,6-dioxopiperidin-3-yl)-4-fluoroisoindoline-1,3-dione;
5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-2-(2,6-dioxopiperidin-3-yl)-6-fluoroisoindoline-1,3-dione;
6-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-2-(2,6-dioxopiperidin-3-yl)-4-fluoroisoindoline-1,3-dione;
5-(((1-(4-((5-chloro-4-((2-(dimethylphosphoryl)phenyl)amino)pyrimidin-2-yl)amino)-3-methoxyphenyl)piperidin-4-yl)amino)methyl)-2-(2,6-dioxopiperidin-3-yl)isoindoline-1,3-dione;
5-(((1-(4-((5-chloro-4-((2-(dimethylphosphoryl)phenyl)amino)pyrimidin-2-yl)amino)-3-methoxyphenyl)piperidin-4-yl)amino)methyl)-2-(2,6-dioxopiperidin-3-yl)-4-fluoroisoindoline-1,3-dione;
5-(((1-(4-((5-chloro-4-((2-(dimethylphosphoryl)phenyl)amino)pyrimidin-2-yl)amino)-3-methoxyphenyl)piperidin-4-yl)amino)methyl)-2-(2,6-dioxopiperidin-3-yl)-6-fluoroisoindoline-1,3-dione;
6-(((1-(4-((5-chloro-4-((2-(dimethylphosphoryl)phenyl)amino)pyrimidin-2-yl)amino)-3-methoxyphenyl)piperidin-4-yl)amino)methyl)-2-(2,6-dioxopiperidin-3-yl)-4-fluoroisoindoline-1,3-dione;
5-((4-(4-((5-chloro-4-((2-(dimethylphosphoryl)phenyl)amino)pyrimidin-2-yl)amino)-3-methoxyphenyl)piperazin-1-yl)methyl)-2-(2,6-dioxopiperidin-3-yl)isoindoline-1,3-dione;
5-((4-(4-((5-chloro-4-((2-(dimethylphosphoryl)phenyl)amino)pyrimidin-2-yl)amino)-3-methoxyphenyl)piperazin-1-yl)methyl)-2-(2,6-dioxopiperidin-3-yl)-4-fluoroisoindoline-1,3-dione;
5-((4-(4-((5-chloro-4-((2-(dimethylphosphoryl)phenyl)amino)pyrimidin-2-yl)amino)-3-methoxyphenyl)piperazin-1-yl)methyl)-2-(2,6-dioxopiperidin-3-yl)-6-fluoroisoindoline-1,3-dione;
6-((4-(4-((5-chloro-4-((2-(dimethylphosphoryl)phenyl)amino)pyrimidin-2-yl)amino)-3-methoxyphenyl)piperazin-1-yl)methyl)-2-(2,6-dioxopiperidin-3-yl)-4-fluoroisoindoline-1,3-dione;
5-((4-(4-(4-((5-chloro-4-((2-(dimethylphosphoryl)phenyl)amino)pyrimidin-2-yl)amino)-3-methoxyphenyl)piperazin-1-yl)piperidin-1-yl)methyl)-2-(2,6-dioxopiperidin-3-yl)isoindoline-1,3-dione;
5-((4-(4-(4-((5-chloro-4-((2-(dimethylphosphoryl)phenyl)amino)pyrimidin-2-yl)amino)-3-methoxyphenyl)piperazin-1-yl)piperidin-1 -yl)methyl)-2-(2,6-dioxopiperidin-3 -yl)-4-fluoroisoindoline-1,3-dione;
5-((4-(4-(4-((5-chloro-4-((2-(dimethylphosphoryl)phenyl)amino)pyrimidin-2-yl)amino)-3-methoxyphenyl)piperazin-1-yl)piperidin-1 -yl)methyl)-2-(2,6-dioxopiperidin-3 -yl)-6-fluoroisoindoline-1,3-dione;
6-((4-(4-(4-((5-chloro-4-((2-(dimethylphosphoryl)phenyl)amino)pyrimidin-2-yl)amino)-3-methoxyphenyl)piperazin-1-yl)piperidin-1 -yl)methyl)-2-(2,6-dioxopiperidin-3 -yl)-4-fluoroisoindoline-1,3-dione;
5-((4-(4-(6-amino-5-((R)-1-(2,6-dichloro-3-fluorophenyl)ethoxy)pyridin-3-yl)-1H-pyrazol-1-yl)piperidin-1-yl)methyl)-2-(2,6-dioxopiperidin-3-yl)isoindoline-1,3-dione;
5-((4-(4-(6-amino-5-((R)-1-(2,6-dichloro-3-fluorophenyl)ethoxy)pyridin-3-yl)-1H-pyrazol-1-yl)piperidin-1-yl)methyl)-2-(2,6-dioxopiperidin-3-yl)-4-fluoroisoindoline-1,3-dione;
5-((4-(4-(6-amino-5-((R)-1-(2,6-dichloro-3-fluorophenyl)ethoxy)pyridin-3-yl)-1H-pyrazol-1-yl)piperidin-1-yl)methyl)-2-(2,6-dioxopiperidin-3-yl)-6-fluoroisoindoline-1,3-dione;
6-((4-(4-(6-amino-5-((R)-1-(2,6-dichloro-3-fluorophenyl)ethoxy)pyridin-3-yl)-1H-pyrazol-1-yl)piperidin-1-yl)methyl)-2-(2,6-dioxopiperidin-3-yl)-4-fluoroisoindoline-1,3-dione;
5-((4-(4-((5-chloro-4-((2-(isopropylsulfonyl)phenyl)amino)pyrimidin-2-yl)amino)-5-isopropoxy-2-methylphenyl)piperidin-1-yl)methyl)-2-(2,6-dioxopiperidin-3-yl)isoindoline-1,3-dione;
5-((4-(4-((5-chloro-4-((2-(isopropylsulfonyl)phenyl)amino)pyrimidin-2-yl)amino)-5-isopropoxy-2-methylphenyl)piperidin-1-yl)methyl)-2-(2,6-dioxopiperidin-3-yl)-4-fluoroisoindoline-1,3-dione;
5- ((4-(4-((5-chloro-4-((2-(isopropylsulfonyl)phenyl)amino)pyrimidin-2-yl)amino)-5-isopropoxy-2-methylphenyl)piperidin-1-yl)methyl)-2-(2,6-dioxopiperidin-3-yl)-6-fluoroisoindoline-1,3-dione;
6- ((4-(4-((5-chloro-4-((2-(isopropylsulfonyl)phenyl)amino)pyrimidin-2-yl)amino)-5-isopropoxy-2-methylphenyl)piperidin-1-yl)methyl)-2-(2,6-dioxopiperidin-3-yl)-4-fluoroisoindoline-1,3-dione;
5-((4-(4-((5-chloro-4-((2-(isopropylsulfonyl)phenyl)amino)pyrimidin-2-yl)amino)-5-isopropoxy-2-methylphenyl)piperazin-1-yl)methyl)-2-(2,6-dioxopiperidin-3-yl)isoindoline-1,3-dione;
5-((4-(4-((5-chloro-4-((2-(isopropylsulfonyl)phenyl)amino)pyrimidin-2-yl)amino)-5-isopropoxy-2-methylphenyl)piperazin-1-yl)methyl)-2-(2,6-dioxopiperidin-3-yl)-4-fluoroisoindoline-1,3-dione;
5-((4-(4-((5-chloro-4-((2-(isopropylsulfonyl)phenyl)amino)pyrimidin-2-yl)amino)-5-isopropoxy-2-methylphenyl)piperazin-1-yl)methyl)-2-(2,6-dioxopiperidin-3-yl)-6-fluoroisoindoline-1,3-dione;
6- ((4-(4-((5-chloro-4-((2-(isopropylsulfonyl)phenyl)amino)pyrimidin-2-yl)amino)-5-isopropoxy-2-methylphenyl)piperazin-1-yl)methyl)-2-(2,6-dioxopiperidin-3-yl)-4-fluoroisoindoline-1,3-dione;
5-(((1-(4-((5-chloro-4-((2-(isopropylsulfonyl)phenyl)amino)pyrimidin-2-yl)amino)-5-isopropoxy-2-methylphenyl)piperidin-4-yl)(methyl)amino)methyl)-2-(2,6-dioxopiperi din-3-yl)isoindoline-1,3-dione;
5-(((1-(4-((5-chloro-4-((2-(isopropylsulfonyl)phenyl)amino)pyrimidin-2-yl)amino)-5-isopropoxy-2-methylphenyl)piperidin-4-yl)(methyl)amino)methyl)-2-(2,6-dioxopiperidin-3-yl)-4-fluoroisoindoline-1,3-dione;
5-(((1-(4-((5-chloro-4-((2-(isopropylsulfonyl)phenyl)amino)pyrimidin-2-yl)amino)-5-isopropoxy-2-methylphenyl)piperidin-4-yl)(methyl)amino)methyl)-2-(2,6-dioxopiperidin-3-yl)-6-fluoroisoindoline-1,3-dione;
6-(((1-(4-((5-chloro-4-((2-(isopropylsulfonyl)phenyl)amino)pyrimidin-2-yl)amino)-5-isopropoxy-2-methylphenyl)piperidin-4-yl)(methyl)amino)methyl)-2-(2,6-dioxopiperidin-3-yl)-4-fluoroisoindoline-1,3-dione;
5-((4-(1-(4-((5-chloro-4-((2-(isopropylsulfonyl)phenyl)amino)pyrimidin-2-yl)amino)-5-isopropoxy-2-methylphenyl)piperidin-4-yl)piperazin-1-yl)methyl)-2-(2,6-dioxopiperidin-3-yl)isoindoline-1,3-dione;
5-((4-(1-(4-((5-chloro-4-((2-(isopropylsulfonyl)phenyl)amino)pyrimidin-2-yl)amino)-5-isopropoxy-2-methylphenyl)piperidin-4-yl)piperazin-1-yl)methyl)-2-(2,6-dioxopiperidin-3-yl)-4-fluoroisoindoline-1,3-dione;
5-((4-(1-(4-((5-chloro-4-((2-(isopropylsulfonyl)phenyl)amino)pyrimidin-2-yl)amino)-5-isopropoxy-2-methylphenyl)piperidin-4-yl)piperazin-1-yl)methyl)-2-(2,6-dioxopiperidin-3-yl)-6-fluoroisoindoline-1,3-dione;
6-((4-(1-(4-((5-chloro-4-((2-(isopropylsulfonyl)phenyl)amino)pyrimidin-2-yl)amino)-5-isopropoxy-2-methylphenyl)piperidin-4-yl)piperazin-1-yl)methyl)-2-(2,6-dioxopiperidin-3-yl)-4-fluoroisoindoline-1,3-dione;
8-(4-(4-((2-(2,6-dioxopiperidin-3-yl)-1,3-dioxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-(4-((2-(2,6-dioxopiperidin-3-yl)-4-fluoro-1,3-dioxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-(4-((2-(2,6-dioxopiperidin-3-yl)-6-fluoro-1,3-dioxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-(4-((2-(2,6-dioxopiperidin-3-yl)-7-fluoro-1,3-dioxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-((2-(2,6-dioxopiperidin-3-yl)-1,3-dioxoisoindolin-5-yl)methyl)piperazin-1-yl)-9-ethyl-6,6-dimethyl-11-oxo-6,11-dihydro-5H-benzo[b]carbazole-3-carbonitrile;
8-(4-((2-(2,6-dioxopiperidin-3-yl)-4-fluoro-1,3-dioxoisoindolin-5-yl)methyl)piperazin-1-yl)-9-ethyl-6,6-dimethyl-11-oxo-6,11-dihydro-SH-benzo[b]carbazole-3-carbonitrile;
8-(4-((2-(2,6-dioxopiperidin-3-yl)-6-fluoro-1,3-dioxoisoindolin-5-yl)methyl)piperazin-1-yl)-9-ethyl-6,6-dimethyl-11-oxo-6,11-dihydro-SH-benzo[b]carbazole-3-carbonitrile;
8-(4-((2-(2,6-dioxopiperidin-3-yl)-7-fluoro-1,3-dioxoisoindolin-5-yl)methyl)piperazin-1-yl)-9-ethyl-6,6-dimethyl-11-oxo-6,11-dihydro-SH-benzo[b]carbazole-3-carbonitrile;
8-(4-(((2-(2,6-dioxopiperidin-3-yl)-1,3-dioxoisoindolin-5-yl)methyl)(methyl)amino)piperidin-1-yl)-9-ethyl-6,6-dimethyl-11-oxo-6,11-dihydro-5H-benzo[b]carbazole-3-carbonitrile;
8-(4-(((2-(2,6-dioxopiperidin-3-yl)-4-fluoro-1,3-dioxoisoindolin-5-yl)methyl)(methyl)amino)piperidin-1-yl)-9-ethyl-6,6-dimethyl-11-oxo-6,11-dihydro-5H-benzo[b]carbazole-3-carbonitrile;
8-(4-(((2-(2,6-dioxopiperidin-3-yl)-6-fluoro-1,3-dioxoisoindolin-5-yl)methyl)(methyl)amino)piperidin-1-yl)-9-ethyl-6,6-dimethyl-11-oxo-6,11-dihydro-5 H-benzo[b]carbazole-3-carbonitrile;
8-(4-(((2-(2,6-dioxopiperidin-3-yl)-7-fluoro-1,3-dioxoisoindolin-5-yl)methyl)(methyl)amino)piperidin-1-yl)-9-ethyl-6,6-dimethyl-11-oxo-6,11-dihydro-5 H-benzo[b]carbazole-3-carbonitrile;
5-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)-2-(2,6-dioxopiperidin-3-yl)isoindoline-1,3-dione;
5-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)-2-(2,6-dioxopiperidin-3-yl)-4-fluoroisoindoline-1,3-dione;
5-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)-2-(2,6-dioxopiperidin-3-yl)-6-fluoroisoindoline-1,3-dione;
6-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)-2-(2,6-dioxopiperidin-3-yl)-4-fluoroisoindoline-1,3-dione;
(4R)-2⁶-amino-1¹-(1-((2-(2,6-dioxopiperidin-3-yl)-1,3-dioxoisoindolin-5-yl)methyl)piperidin-4-yl)-5⁵-fluoro-4,7-dimethyl-6-oxo-1¹H-3-oxa-7-aza-2(3,5)-pyridina-1(4,3)-pyrazola-5(1,2)-benzenacyclooctaphane-1⁵-carbonitrile;
(4R)-2⁶-amino-1¹-(1-((2-(2,6-dioxopiperidin-3-yl)-4-fluoro-1,3-dioxoisoindolin-5-yl)methyl)piperidin-4-yl)-5⁵-fluoro-4,7-dimethyl-6-oxo-1¹H-3-oxa-7-aza-2(3,5)-pyridina-1(4,3)-pyrazola-5(1,2)-benzenacyclooctaphane-1⁵-carbonitrile;
(4R)-2⁶-amino-1¹-(1-((2-(2,6-dioxopiperidin-3-yl)-6-fluoro-1,3-dioxoisoindolin-5-yl)methyl)piperidin-4-yl)-5⁵-fluoro-4,7-dimethyl-6-oxo-1¹H-3-oxa-7-aza-2(3,5)-pyridina-1(4,3)-pyrazola-5(1,2)-benzenacyclooctaphane-1⁵-carbonitrile;
(4R)-2⁶-amino-1¹-(1-((2-(2,6-dioxopiperidin-3-yl)-7-fluoro-1,3-dioxoisoindolin-5-yl)methyl)piperidin-4-yl)-5⁵-fluoro-4,7-dimethyl-6-oxo-1¹H-3-oxa-7-aza-2(3,5)-pyridina-1(4,3)-pyrazola-5(1,2)-benzenacyclooctaphane-1⁵-carbonitrile;
2-((S)-4-(4-chlorophenyl)-2,3,9-trimethyl-6H-thieno[3,2-f][1,2,4]triazolo[4,3-a][1,4]diazepin-6-yl)-N-((2-(2,6-dioxopiperidin-3-yl)-1,3-dioxoisoindolin-5-yl)methyl)acetamide;
2-((S)-4-(4-chlorophenyl)-2,3,9-trimethyl-6H-thieno[3,2-f][1,2,4]triazolo[4,3-a][1,4]diazepin-6-yl)-N-((2-(2,6-dioxopiperidin-3-yl)-4-fluoro-1,3-dioxoisoindolin-5-yl)methyl)acetamide;
2-((S)-4-(4-chlorophenyl)-2,3,9-trimethyl-6H-thieno[3,2-f][1,2,4]triazolo[4,3-a][1,4]diazepin-6-yl)-N-((2-(2,6-dioxopiperidin-3-yl)-6-fluoro-1,3-dioxoisoindolin-5-yl)methyl)acetamide;
2-((S)-4-(4-chlorophenyl)-2,3,9-trimethyl-6H-thieno[3,2-f][1,2,4]triazolo[4,3-a][1,4]diazepin-6-yl)-N-((2-(2,6-dioxopiperidin-3-yl)-7-fluoro-1,3-dioxoisoindolin-5-yl)methyl)acetamide;
2-((S)-4-(4-chlorophenyl)-2,3,9-trimethyl-6H-thieno[3,2-f][1,2,4]triazolo[4,3-a][1,4]diazepin-6-yl)-N-(2-(4-((2-(2,6-dioxopiperidin-3-yl)-1,3-dioxoisoindolin-5-yl)methyl)piperazin-1-yl)ethyl)acetamide;
2-((S)-4-(4-chlorophenyl)-2,3,9-trimethyl-6H-thieno[3,2-f][1,2,4]triazolo[4,3-a][1,4]diazepin-6-yl)-N-(2-(4-((2-(2,6-dioxopiperidin-3-yl)-4-fluoro-1,3-dioxoisoindolin-5-yl)methyl)piperazin-1-yl)ethyl)acetamide;
2-((S)-4-(4-chlorophenyl)-2,3,9-trimethyl-6H-thieno[3,2-f][1,2,4]triazolo[4,3-a][1,4]diazepin-6-yl)-N-(2-(4-((2-(2,6-dioxopiperidin-3-yl)-6-fluoro-1,3-dioxoisoindolin-5-yl)methyl)piperazin-1-yl)ethyl)acetamide;
2-((S)-4-(4-chlorophenyl)-2,3,9-trimethyl-6H-thieno[3,2-f][1,2,4]triazolo[4,3-a][1,4]diazepin-6-yl)-N-(2-(4-((2-(2,6-dioxopiperidin-3-yl)-7-fluoro-1,3-dioxoisoindolin-5-yl)methyl)piperazin-1-yl)ethyl)acetamide;
5-((4-(2-((S)-4-(4-chlorophenyl)-2,3,9-trimethyl-6H-thieno[3,2-f][1,2,4]triazolo[4,3-a][1,4]diazepin-6-yl)acetyl)piperazin-1-yl)methyl)-2-(2,6-dioxopiperidin-3-yl)isoindoline-1,3-dione;
5-((4-(2-((S)-4-(4-chlorophenyl)-2,3,9-trimethyl-6H-thieno[3,2-f][1,2,4]triazolo[4,3-a][1,4]diazepin-6-yl)acetyl)piperazin-1-yl)methyl)-2-(2,6-dioxopiperidin-3-yl)-4-fluoroisoindoline-1,3-dione;
5-((4-(2-((S)-4-(4-chlorophenyl)-2,3,9-trimethyl-6H-thieno[3,2-f][1,2,4]triazolo[4,3-a][1,4]diazepin-6-yl)acetyl)piperazin-1-yl)methyl)-2-(2,6-dioxopiperidin-3-yl)-6-fluoroisoindoline-1,3-dione;
6-((4-(2-((S)-4-(4-chlorophenyl)-2,3,9-trimethyl-6H-thieno[3,2-f][1,2,4]triazolo[4,3-a][1,4]diazepin-6-yl)acetyl)piperazin-1-yl)methyl)-2-(2,6-dioxopiperidin-3-yl)-4-fluoroisoindoline-1,3-dione;
5-((4-(2-((S)-4-(4-chlorophenyl)-2,3,9-trimethyl-6H-thieno[3,2-f][1,2,4]triazolo[4,3-a][1,4]diazepin-6-yl)ethyl)piperazin-1-yl)methyl)-2-(2,6-dioxopiperidin-3-yl)isoindoline-1,3-dione;
5-((4-(2-((S)-4-(4-chlorophenyl)-2,3,9-trimethyl-6H-thieno[3,2-f][1,2,4]triazolo[4,3-a][1,4]diazepin-6-yl)ethyl)piperazin-1-yl)methyl)-2-(2,6-dioxopiperidin-3-yl)-4-fluoroisoindoline-1,3-dione;
5-((4-(2-((S)-4-(4-chlorophenyl)-2,3,9-trimethyl-6H-thieno[3,2-f][1,2,4]triazolo[4,3-a][1,4]diazepin-6-yl)ethyl)piperazin-1-yl)methyl)-2-(2,6-dioxopiperidin-3-yl)-6-fluoroisoindoline-1,3-dione;
6-((4-(2-((S)-4-(4-chlorophenyl)-2,3,9-trimethyl-6H-thieno[3,2-f][1,2,4]triazolo[4,3-a][1,4]diazepin-6-yl)ethyl)piperazin-1-yl)methyl)-2-(2,6-dioxopiperidin-3-yl)-4-fluoroisoindoline-1,3-dione;
5-((4-(4-(5,7-dimethoxy-4-oxo-3,4-dihydroquinazolin-2-yl)phenyl)piperazin-1-yl)methyl)-2-(2,6-dioxopipendin-3 -yl)isoindoline-1,3-dione;
5-((4-(4-(5,7-dimethoxy-4-oxo-3,4-dihydroquinazolin-2-yl)phenyl)piperazin-1-yl)methyl)-2-(2,6-dioxopiperidin-3-yl)-4-fluoroisoindoline-1,3-dione;
5-((4-(4-(5,7-dimethoxy-4-oxo-3,4-dihydroquinazolin-2-yl)phenyl)piperazin-1-yl)methyl)-2-(2,6-dioxopiperidin-3-yl)-6-fluoroisoindoline-1,3-dione;
6-((4-(4-(5,7-dimethoxy-4-oxo-3,4-dihydroquinazolin-2-yl)phenyl)piperazin-1-yl)methyl)-2-(2,6-dioxopiperidin-3-yl)-4-fluoroisoindoline-1,3-dione;
5-(((1-(4-(5,7-dimethoxy-4-oxo-3,4-dihydroquinazolin-2-yl)phenyl)piperidin-4-yl)(methyl)amino)methyl)-2-(2,6-dioxopiperidin-3-yl)isoindoline-1,3-dione;
5-(((l-(4-(5,7-dimethoxy-4-oxo-3,4-dihydroquinazolin-2-yl)phenyl)piperidin-4-yl)(methyl)amino)methyl)-2-(2,6-dioxopiperidin-3-yl)-4-fluoroisoindoline-1,3-dione;
5-(((1-(4-(5,7-dimethoxy-4-oxo-3,4-dihydroquinazolin-2-yl)phenyl)piperidin-4-yl)(methyl)amino)methyl)-2-(2,6-dioxopiperidin-3-yl)-6-fluoroisoindoline-1,3-dione;
6-(((1-(4-(5,7-dimethoxy-4-oxo-3,4-dihydroquinazolin-2-yl)phenyl)piperidin-4-yl)(methyl)amino)methyl)-2-(2,6-dioxopiperidin-3-yl)-4-fluoroisoindoline-1,3-dione;
5-((4-(1-(4-(5,7-dimethoxy-4-oxo-3,4-dihydroquinazolin-2-yl)phenyl)piperidin-4-yl)piperazin-1-yl)methyl)-2-(2,6-dioxopiperidin-3-yl)isoindoline-1,3-dione;
5-((4-(1-(4-(5,7-dimethoxy-4-oxo-3,4-dihydroquinazolin-2-yl)phenyl)piperidin-4-yl)piperazin-1-yl)methyl)-2-(2,6-dioxopiperidin-3-yl)-4-fluoroisoindoline-1,3-dione;
5-((4-(1-(4-(5,7-dimethoxy-4-oxo-3,4-dihydroquinazolin-2-yl)phenyl)piperidin-4-yl)piperazin-1-yl)methyl)-2-(2,6-dioxopiperidin-3-yl)-6-fluoroisoindoline-1,3-dione;
6-((4-(1-(4-(5,7-dimethoxy-4-oxo-3,4-dihydroquinazolin-2-yl)phenyl)piperidin-4-yl)piperazin-1-yl)methyl)-2-(2,6-dioxopiperidin-3-yl)-4-fluoroisoindoline-1,3-dione;
4-(4-((2-(2,6-dioxopiperidin-3-yl)-1,3-dioxoisoindolin-5-yl)methyl)piperazin-1-yl)-N-(5-((R)-2-methoxy-2-phenylacetyl)-1,4,5,6-tetrahydropyrrolo[3,4-c]pyrazol-3-yl)benzamide;
4-(4-((2-(2,6-dioxopiperidin-3-yl)-4-fluoro-1,3-dioxoisoindolin-5-yl)methyl)piperazin-1-yl)-N-(5-((R)-2-methoxy-2-phenylacetyl)-1,4,5,6-tetrahydropyrrolo[3,4-c]pyrazol-3-yl)benzamide;
4-(4-((2-(2,6-dioxopiperidin-3-yl)-6-fluoro-1,3-dioxoisoindolin-5-yl)methyl)piperazin-1-yl)-N-(5-((R)-2-methoxy-2-phenylacetyl)-1,4,5,6-tetrahydropyrrolo[3,4-c]pyrazol-3-yl)benzamide;
4-(4-((2-(2,6-dioxopiperidin-3-yl)-7-fluoro-1,3-dioxoisoindolin-5-yl)methyl)piperazin-1-yl)-N-(5-((R)-2-methoxy-2-phenylacetyl)-1,4,5,6-tetrahydropyrrolo[3,4-c]pyrazol-3-yl)benzamide;
4-(4-(((2-(2,6-dioxopiperidin-3-yl)-1,3-dioxoisoindolin-5-yl)methyl)(methyl)amino)piperidin-1-yl)-N-(5-((R)-2-methoxy-2-phenylacetyl)-1,4,5,6-tetrahydropyrrolo[3,4-c]pyrazol-3-yl)benzamide;
4-(4-(((2-(2,6-dioxopiperidin-3-yl)-4-fluoro-1,3-dioxoisoindolin-5-yl)methyl)(methyl)amino)piperidin-1-yl)-N-(5-((R)-2-methoxy-2-phenylacetyl)-1,4,5,6-tetrahydropyrrolo[3,4-c]pyrazol-3-yl)benzamide;
4-(4-(((2-(2,6-dioxopiperidin-3-yl)-6-fluoro-1,3-dioxoisoindolin-5-yl)methyl)(methyl)amino)piperidin-1-yl)-N-(5-((R)-2-methoxy-2-phenylacetyl)-1,4,5,6-tetrahydropyrrolo[3,4-c]pyrazol-3-yl)benzamide;
4-(4-(((2-(2,6-dioxopiperidin-3-yl)-7-fluoro-1,3-dioxoisoindolin-5-yl)methyl)(methyl)amino)piperidin-1-yl)-N-(5-((R)-2-methoxy-2-phenylacetyl)-1,4,5,6-tetrahydropyrrolo[3,4-c]pyrazol-3-yl)benzamide;
4-(4-(4-((2-(2,6-dioxopiperidin-3-yl)-1,3-dioxoisoindolin-5-yl)methyl)piperazin-1-yl)piperidin-1-yl)-N-(5-((R)-2-methoxy-2-phenylacetyl)-1,4,5,6-tetrahydropyrrolo[3,4-c]pyrazol-3-yl)benzamide;
4-(4-(4-((2-(2,6-dioxopiperidin-3-yl)-4-fluoro-1,3-dioxoisoindolin-5-yl)methyl)piperazin-1-yl)piperidin-1-yl)-N-(5-((R)-2-methoxy-2-phenylacetyl)-1,4,5,6-tetrahydropyrrolo[3,4-c]pyrazol-3-yl)benzamide;
4-(4-(4-((2-(2,6-dioxopiperidin-3-yl)-6-fluoro-1,3-dioxoisoindolin-5-yl)methyl)piperazin-1-yl)piperidin-1-yl)-N-(5-((R)-2-methoxy-2-phenylacetyl)-1,4,5,6-tetrahydropyrrolo[3,4-c]pyrazol-3-yl)benzamide;
4-(4-(4-((2-(2,6-dioxopiperidin-3-yl)-7-fluoro-1,3-dioxoisoindolin-5-yl)methyl)piperazin-1-yl)piperidin-1-yl)-N-(5-((R)-2-methoxy-2-phenylacetyl)-1,4,5,6-tetrahydropyrrolo[3,4-c]pyrazol-3-yl)benzamide;
N-(tert-butyl)-3-((2-((6-(4-((2-(2,6-dioxopiperidin-3-yl)-1,3-dioxoisoindolin-5-yl)methyl)piperazin-1-yl)pyridazin-3-yl)amino)-5-methylpyrimidin-4-yl)amino)benzenesulfonamide;
N-(tert-butyl)-3-((2-((6-(4-((2-(2,6-dioxopiperidin-3-yl)-4-fluoro-1,3-dioxoisoindolin-5-yl)methyl)piperazin-1-yl)pyridazin-3-yl)amino)-5-methylpyrimidin-4-yl)amino)benzenesulfonamide;
N-(tert-butyl)-3-((2-((6-(4-((2-(2,6-dioxopiperidin-3-yl)-6-fluoro-1,3-dioxoisoindolin-5-yl)methyl)piperazin-1-yl)pyridazin-3-yl)amino)-5-methylpyrimidin-4-yl)amino)benzenesulfonamide;
N-(tert-butyl)-3-((2-((6-(4-((2-(2,6-dioxopiperidin-3-yl)-7-fluoro-1,3-dioxoisoindolin-5-yl)methyl)piperazin-1-yl)pyridazin-3-yl)amino)-5-methylpyrimidin-4-yl)amino)benzenesulfonamide;
N-(tert-butyl)-3-((2-((4-(4-(((2-(2,6-dioxopiperidin-3-yl)-1,3-dioxoisoindolin-5-yl)methyl)(methyl)amino)piperidin-1-yl)phenyl)amino)-5-methylpyrimidin-4-yl)amino)benzenesulfonamide;
N-(tert-butyl)-3-((2-((4-(4-(((2-(2,6-dioxopiperidin-3-yl)-4-fluoro-1,3-dioxoisoindolin-5-yl)methyl)(methyl)amino)piperidin-1-yl)phenyl)amino)-5-methylpyrimidin-4-yl)amino)benzenesulfonamide;
N-(tert-butyl)-3-((2-((4-(4-(((2-(2,6-dioxopiperidin-3-yl)-6-fluoro-1,3-dioxoisoindolin-5-yl)methyl)(methyl)amino)piperidin-1-yl)phenyl)amino)-5-methylpyrimidin-4-yl)amino)benzenesulfonamide;
N-(tert-butyl)-3-((2-((4-(4-(((2-(2,6-dioxopiperidin-3-yl)-7-fluoro-1,3-dioxoisoindolin-5-yl)methyl)(methyl)amino)piperidin-1-yl)phenyl)amino)-5-methylpyrimidin-4-yl)amino)benzenesulfonamide;
N-(tert-butyl)-3-((2-((4-(4-(4-((2-(2,6-dioxopiperidin-3-yl)-1,3-dioxoisoindolin-5-yl)methyl)piperazin-1-yl)piperidin-1-yl)phenyl)amino)-5-methylpyrimidin-4-yl)amino)benzenesulfonamide;
N-(tert-butyl)-3-((2-((4-(4-(4-((2-(2,6-dioxopiperidin-3-yl)-4-fluoro-1,3-dioxoisoindolin-5-yl)methyl)piperazin-1-yl)piperidin-1-yl)phenyl)amino)-5-methylpyrimidin-4-yl)amino)benzenesulfonamide;
N-(tert-butyl)-3-((2-((4-(4-(4-((2-(2,6-dioxopiperidin-3-yl)-6-fluoro-1,3-dioxoisoindolin-5-yl)methyl)piperazin-1-yl)piperidin-1-yl)phenyl)amino)-5-methylpyrimidin-4-yl)amino)benzenesulfonamide;
N-(tert-butyl)-3-((2-((4-(4-(4-((2-(2,6-dioxopiperidin-3-yl)-7-fluoro-1,3-dioxoisoindolin-5-yl)methyl)piperazin-1-yl)piperidin-1-yl)phenyl)amino)-5-methylpyrimidin-4-yl)amino)benzenesulfonamide;
N-(tert-butyl)-3-((2-((4-(4-(1-((2-(2,6-dioxopiperidin-3-yl)-1,3-dioxoisoindolin-5-yl)methyl)piperidin-4-yl)piperazin-1-yl)phenyl)amino)-5-methylpyrimidin-4-yl)amino)benzenesulfonamide;
N-(tert-butyl)-3-((2-((4-(4-(1-((2-(2,6-dioxopiperidin-3-yl)-4-fluoro-1,3-dioxoisoindolin-5-yl)methyl)piperidin-4-yl)piperazin-1-yl)phenyl)amino)-5-methylpyrimidin-4-yl)amino)benzenesulfonamide;
N-(tert-butyl)-3-((2-((4-(4-(1-((2-(2,6-dioxopiperidin-3-yl)-6-fluoro-1,3-dioxoisoindolin-5-yl)methyl)piperidin-4-yl)piperazin-1-yl)phenyl)amino)-5-methylpyrimidin-4-yl)amino)benzenesulfonamide;
N-(tert-butyl)-3-((2-((4-(4-(1-((2-(2,6-dioxopiperidin-3-yl)-7-fluoro-1,3-dioxoisoindolin-5-yl)methyl)piperidin-4-yl)piperazin-1-yl)phenyl)amino)-5-methylpyrimidin-4-yl)amino)benzenesulfonamide;
N-(tert-butyl)-3-((2-((4-(4-((2-(2,6-dioxopiperidin-3-yl)-1,3-dioxoisoindolin-5-yl)methyl)piperazin-1-yl)phenyl)amino)-5-methylpyrimidin-4-yl)amino)benzenesulfonamide;
N-(tert-butyl)-3-((2-((4-(4-((2-(2,6-dioxopiperidin-3-yl)-4-fluoro-1,3-dioxoisoindolin-5-yl)methyl)piperazin-1-yl)phenyl)amino)-5-methylpyrimidin-4-yl)amino)benzenesulfonamide;
N-(tert-butyl)-3-((2-((4-(4-((2-(2,6-dioxopiperidin-3-yl)-6-fluoro-1,3-dioxoisoindolin-5-yl)methyl)piperazin-1-yl)phenyl)amino)-5-methylpyrimidin-4-yl)amino)benzenesulfonamide;
N-(tert-butyl)-3-((2-((4-(4-((2-(2,6-dioxopiperidin-3-yl)-7-fluoro-1,3-dioxoisoindolin-5-yl)methyl)piperazin-1-yl)phenyl)amino)-5-methylpyrimidin-4-yl)amino)benzenesulfonamide;
N-(tert-butyl)-3-((2-((4-(1-((2-(2,6-dioxopiperidin-3-yl)-1,3-dioxoisoindolin-5-yl)methyl)piperidin-4-yl)phenyl)amino)-5-methylpyrimidin-4-yl)amino)benzenesulfonamide;
N-(tert-butyl)-3-((2-((4-(1-((2-(2,6-dioxopiperidin-3-yl)-4-fluoro-1,3-dioxoisoindolin-5-yl)methyl)piperidin-4-yl)phenyl)amino)-5-methylpyrimidin-4-yl)amino)benzenesulfonamide;
N-(tert-butyl)-3-((2-((4-(1-((2-(2,6-dioxopiperidin-3-yl)-6-fluoro-1,3-dioxoisoindolin-5-yl)methyl)piperidin-4-yl)phenyl)amino)-5-methylpyrimidin-4-yl)amino)benzenesulfonamide;
N-(tert-butyl)-3-((2-((4-(1-((2-(2,6-dioxopiperidin-3-yl)-7-fluoro-1,3-dioxoisoindolin-5-yl)methyl)piperidin-4-yl)phenyl)amino)-5-methylpyrimidin-4-yl)amino)benzenesulfonamide;
N-(tert-butyl)-3-((2-((4-(2-(4-((2-(2,6-dioxopiperidin-3-yl)-1,3-dioxoisoindolin-5-yl)methyl)piperazin-1-yl)ethoxy)phenyl)amino)-5-methylpyrimidin-4-yl)amino)benzenesulfonamide;
N-(tert-butyl)-3-((2-((4-(2-(4-((2-(2,6-dioxopiperidin-3-yl)-4-fluoro-1,3-dioxoisoindolin-5-yl)methyl)piperazin-1-yl)ethoxy)phenyl)amino)-5-methylpyrimidin-4-yl)amino)benzenesulfonamide;
N-(tert-butyl)-3-((2-((4-(2-(4-((2-(2,6-dioxopiperidin-3-yl)-6-fluoro-1,3-dioxoisoindolin-5-yl)methyl)piperazin-1-yl)ethoxy)phenyl)amino)-5-methylpyrimidin-4-yl)amino)benzenesulfonamide;
N-(tert-butyl)-3-((2-((4-(2-(4-((2-(2,6-dioxopiperidin-3-yl)-7-fluoro-1,3-dioxoisoindolin-5-yl)methyl)piperazin-1-yl)ethoxy)phenyl)amino)-5-methylpyrimidin-4-yl)amino)benzenesulfonamide;
2-((2-((4-(4-(4-((2-(2,6-dioxopiperidin-3-yl)-1,3-dioxoisoindolin-5-yl)methyl)piperazin-1-yl)piperidin-1-yl)-2-methoxyphenyl)amino)-5-(trifluoromethyl)pyridin-4-yl)amino)-N-methylbenzamide;
2-((2-((4-(4-(4-((2-(2,6-dioxopiperidin-3-yl)-4-fluoro-1,3-dioxoisoindolin-5-yl)methyl)piperazin-1-yl)piperidin-1-yl)-2-methoxyphenyl)amino)-5-(trifluoromethyl)pyridin-4-yl)amino)-N-methylbenzamide;
2-((2-((4-(4-(4-((2-(2,6-dioxopiperidin-3-yl)-6-fluoro-1,3-dioxoisoindolin-5-yl)methyl)piperazin-1-yl)piperidin-1-yl)-2-methoxyphenyl)amino)-5-(trifluoromethyl)pyridin-4-yl)amino)-N-methylbenzamide;
2-((2-((4-(4-(4-((2-(2,6-dioxopiperidin-3-yl)-7-fluoro-1,3-dioxoisoindolin-5-yl)methyl)piperazin-1-yl)piperidin-1-yl)-2-methoxyphenyl)amino)-5-(trifluoromethyl)pyridin-4-yl)amino)-N-methylbenzamide;
2-((2-((4-(4-(((2-(2,6-dioxopiperidin-3-yl)-1,3-dioxoisoindolin-5-yl)methyl)amino)piperidin-1-yl)-2-methoxyphenyl)amino)-5-(trifluoromethyl)pyridin-4-yl)amino)-N-methylbenzamide;
2-((2-((4-(4-(((2-(2,6-dioxopiperidin-3-yl)-4-fluoro-1,3-dioxoisoindolin-5-yl)methyl)amino)piperidin-1-yl)-2-methoxyphenyl)amino)-5-(trifluoromethyl)pyridin-4-yl)amino)-N-methylbenzamide;
2-((2-((4-(4-(((2-(2,6-dioxopiperidin-3-yl)-6-fluoro-1,3-dioxoisoindolin-5-yl)methyl)amino)piperidin-1-yl)-2-methoxyphenyl)amino)-5-(trifluoromethyl)pyridin-4-yl)amino)-N-methylbenzamide;
2-((2-((4-(4-(((2-(2,6-dioxopiperidin-3-yl)-7-fluoro-1,3-dioxoisoindolin-5-yl)methyl)amino)piperidin-1-yl)-2-methoxyphenyl)amino)-5-(trifluoromethyl)pyridin-4-yl)amino)-N-methylbenzamide;
2-((2-((4-(4-(((2-(2,6-dioxopiperidin-3-yl)-1,3-dioxoisoindolin-5-yl)methyl)(methyl)amino)piperidin-1-yl)-2-methoxyphenyl)amino)-5-(trifluoromethyl)pyridin-4-yl)amino)-N-methylbenzamide;
2-((2-((4-(4-(((2-(2,6-dioxopiperidin-3-yl)-4-fluoro-1,3-dioxoisoindolin-5-yl)methyl)(methyl)amino)piperidin-1-yl)-2-methoxyphenyl)amino)-5-(trifluoromethyl)pyridin-4-yl)amino)-N-methylbenzamide;
2-((2-((4-(4-(((2-(2,6-dioxopiperidin-3-yl)-6-fluoro-1,3-dioxoisoindolin-5-yl)methyl)(methyl)amino)piperidin-1-yl)-2-methoxyphenyl)amino)-5-(trifluoromethyl)pyridin-4-yl)amino)-N-methylbenzamide;
2-((2-((4-(4-(((2-(2,6-dioxopiperidin-3-yl)-7-fluoro-1,3-dioxoisoindolin-5-yl)methyl)(methyl)amino)piperidin-1-yl)-2-methoxyphenyl)amino)-5-(trifluoromethyl)pyridin-4-yl)amino)-N-methylbenzamide;
2-((2-((4-(4-((2-(2,6-dioxopiperidin-3-yl)-1,3-dioxoisoindolin-5-yl)methyl)piperazin-1-yl)-2-methoxyphenyl)amino)-5-(trifluoromethyl)pyridin-4-yl)amino)-N-methylbenzamide;
2-((2-((4-(4-((2-(2,6-dioxopiperidin-3-yl)-4-fluoro-1,3-dioxoisoindolin-5-yl)methyl)piperazin-1-yl)-2-methoxyphenyl)amino)-5-(trifluoromethyl)pyridin-4-yl)amino)-N-methylbenzamide;
2-((2-((4-(4-((2-(2,6-dioxopiperidin-3-yl)-6-fluoro-1,3-dioxoisoindolin-5-yl)methyl)piperazin-1-yl)-2-methoxyphenyl)amino)-5-(trifluoromethyl)pyridin-4-yl)amino)-N-methylbenzamide;
2-((2-((4-(4-((2-(2,6-dioxopiperidin-3-yl)-7-fluoro-1,3-dioxoisoindolin-5-yl)methyl)piperazin-1-yl)-2-methoxyphenyl)amino)-5-(trifluoromethyl)pyridin-4-yl)amino)-N-methylbenzamide;
N-(3-(((2-((4-(4-((2-(2,6-dioxopiperidin-3-yl)-1,3-dioxoisoindolin-5-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((2-(2,6-dioxopiperidin-3-yl)-4-fluoro-1,3-dioxoisoindolin-5-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((2-(2,6-dioxopiperidin-3-yl)-6-fluoro-1,3-dioxoisoindolin-5-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((2-(2,6-dioxopiperidin-3-yl)-7-fluoro-1,3-dioxoisoindolin-5-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(((2-(2,6-dioxopiperidin-3-yl)-1,3-dioxoisoindolin-5-yl)methyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(((2-(2,6-dioxopiperidin-3-yl)-4-fluoro-1,3-dioxoisoindolin-5-yl)methyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(((2-(2,6-dioxopiperidin-3-yl)-6-fluoro-1,3-dioxoisoindolin-5-yl)methyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(((2-(2,6-dioxopiperidin-3-yl)-7-fluoro-1,3-dioxoisoindolin-5-yl)methyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-((2-(2,6-dioxopiperidin-3-yl)-1,3-dioxoisoindolin-5-yl)methyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-((2-(2,6-dioxopiperidin-3-yl)-4-fluoro-1,3-dioxoisoindolin-5-yl)methyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-((2-(2,6-dioxopiperidin-3-yl)-6-fluoro-1,3-dioxoisoindolin-5-yl)methyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-((2-(2,6-dioxopiperidin-3-yl)-7-fluoro-1,3-dioxoisoindolin-5-yl)methyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((2-(2,6-dioxopiperidin-3-yl)-1,3-dioxoisoindolin-5-yl)methyl)piperazin-1-yl)phenyl)amino)-5-(trifluoromethyl)pyrimidin-4-yl)amino)methyl)phenyl)-N-methylmethanesulfonamide;
N-(3-(((2-((4-(4-((2-(2,6-dioxopiperidin-3-yl)-4-fluoro-1,3-dioxoisoindolin-5-yl)methyl)piperazin-1-yl)phenyl)amino)-5-(trifluoromethyl)pyrimidin-4-yl)amino)methyl)phenyl)-N-methylmethanesulfonamide;
N-(3-(((2-((4-(4-((2-(2,6-dioxopiperidin-3-yl)-6-fluoro-1,3-dioxoisoindolin-5-yl)methyl)piperazin-1-yl)phenyl)amino)-5-(trifluoromethyl)pyrimidin-4-yl)amino)methyl)phenyl)-N-methylmethanesulfonamide;
N-(3-(((2-((4-(4-((2-(2,6-dioxopiperidin-3-yl)-7-fluoro-1,3-dioxoisoindolin-5-yl)methyl)piperazin-1-yl)phenyl)amino)-5-(trifluoromethyl)pyrimidin-4-yl)amino)methyl)phenyl)-N-methylmethanesulfonamide;
N-(3-(((2-((4-(4-(((2-(2,6-dioxopiperidin-3-yl)-1,3-dioxoisoindolin-5-yl)methyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-(((2-(2,6-dioxopiperidin-3-yl)-4-fluoro-1,3-dioxoisoindolin-5-yl)methyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-(((2-(2,6-dioxopiperidin-3-yl)-6-fluoro-1,3-dioxoisoindolin-5-yl)methyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-(((2-(2,6-dioxopiperidin-3-yl)-7-fluoro-1,3-dioxoisoindolin-5-yl)methyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-((2-(2,6-dioxopiperidin-3-yl)-1,3-dioxoisoindolin-5-yl)methyl)piperazin-1-yl)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-((2-(2,6-dioxopiperidin-3-yl)-4-fluoro-1,3-dioxoisoindolin-5-yl)methyl)piperazin-1-yl)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-((2-(2,6-dioxopiperidin-3-yl)-6-fluoro-1,3-dioxoisoindolin-5-yl)methyl)piperazin-1-yl)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-((2-(2,6-dioxopiperidin-3-yl)-7-fluoro-1,3-dioxoisoindolin-5-yl)methyl)piperazin-1-yl)piperidin-1-yl)phenyl)amino)-5-(trifluoromethyl)pyrimidin-4-yl)amino)methyl)phenyl)-N-methylmethanesulfonamide;
N-(3-(((2-((4-(((2-(2,6-dioxopiperidin-3-yl)-1,3-dioxoisoindolin-5-yl)methyl)amino)phenyl)amino)-5-(trifluoromethyl)pyrimidin-4-yl)amino)methyl)phenyl)-N-methylmethanesulfonamide;
N-(3-(((2-((4-(((2-(2,6-dioxopiperidin-3-yl)-4-fluoro-1,3-dioxoisoindolin-5-yl)methyl)amino)phenyl)amino)-5-(trifluoromethyl)pyrimidin-4-yl)amino)methyl)phenyl)-N-methylmethanesulfonamide;
N-(3-(((2-((4-(((2-(2,6-dioxopiperidin-3-yl)-6-fluoro-1,3-dioxoisoindolin-5-yl)methyl)amino)phenyl)amino)-5-(trifluoromethyl)pyrimidin-4-yl)amino)methyl)phenyl)-N-methylmethanesulfonamide;
N-(3-(((2-((4-(((2-(2,6-dioxopiperidin-3-yl)-7-fluoro-1,3-dioxoisoindolin-5-yl)methyl)amino)phenyl)amino)-5-(trifluoromethyl)pyrimidin-4-yl)amino)methyl)phenyl)-N-methylmethanesulfonamide;
N-(3-(((2-((4-(4-((2-(2,6-dioxopiperidin-3-yl)-1,3-dioxoisoindolin-5-yl)methyl)piperazin-1-yl)phenyl)amino)-5-(trifluoromethyl)pyrimidin-4-yl)amino)methyl)pyridin-2-yl)-N-methylmethanesulfonamide;
N-(3-(((2-((4-(4-((2-(2,6-dioxopiperidin-3-yl)-4-fluoro-1,3-dioxoisoindolin-5-yl)methyl)piperazin-1-yl)phenyl)amino)-5-(trifluoromethyl)pyrimidin-4-yl)amino)methyl)pyridin-2-yl)-N-methylmethanesulfonamide;
N-(3-(((2-((4-(4-((2-(2,6-dioxopiperidin-3-yl)-6-fluoro-1,3-dioxoisoindolin-5-yl)methyl)piperazin-1-yl)phenyl)amino)-5-(trifluoromethyl)pyrimidin-4-yl)amino)methyl)pyridin-2-yl)-N-methylmethanesulfonamide;
N-(3-(((2-((4-(4-((2-(2,6-dioxopiperidin-3-yl)-7-fluoro-1,3-dioxoisoindolin-5-yl)methyl)piperazin-1-yl)phenyl)amino)-5-(trifluoromethyl)pyrimidin-4-yl)amino)methyl)pyridin-2-yl)-N-methylmethanesulfonamide;
N-(3-(((2-((4-(4-(((2-(2,6-dioxopiperidin-3-yl)-1,3-dioxoisoindolin-5-yl)methyl)(methyl)amino)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(((2-(2,6-dioxopiperidin-3-yl)-7-fluoro-1,3-dioxoisoindolin-5-yl)methyl)(methyl)amino)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(((2-(2,6-dioxopiperidin-3-yl)-6-fluoro-1,3-dioxoisoindolin-5-yl)methyl)(methyl)amino)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(((2-(2,6-dioxopiperidin-3-yl)-7-fluoro-1,3-dioxoisoindolin-5-yl)methyl)(methyl)amino)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-((2-(2,6-dioxopiperidin-3-yl)-1,3-dioxoisoindolin-5-yl)methyl)piperazin-1-yl)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-((2-(2,6-dioxopiperidin-3-yl)-4-fluoro-1,3-dioxoisoindolin-5-yl)methyl)piperazin-1-yl)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-((2-(2,6-dioxopiperidin-3-yl)-6-fluoro-1,3-dioxoisoindolin-5-yl)methyl)piperazin-1-yl)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-((2-(2,6-dioxopiperidin-3-yl)-7-fluoro-1,3-dioxoisoindolin-5-yl)methyl)piperazin-1-yl)piperidin-1-yl)phenyl)amino)-5-(trifluoromethyl)pyrimidin-4-yl)amino)methyl)pyridin-2-yl)-N-methylmethanesulfonamide;
2-(2-((cyclopropylmethyl)amino)pyridin-4-yl)-N-(3-(difluoromethyl)-1-(1-((2-(2,6-dioxopiperidin-3-yl)-1,3-dioxoisoindolin-5-yl)methyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-((2-(2,6-dioxopiperidin-3-yl)-4-fluoro-1,3-dioxoisoindolin-5-yl)methyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-((2-(2,6-dioxopiperidin-3-yl)-6-fluoro-1,3-dioxoisoindolin-5-yl)methyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-((2-(2,6-dioxopiperidin-3-yl)-7-fluoro-1,3-dioxoisoindolin-5-yl)methyl)piperidin-4-yl)-1H-pyrazol-4-yl)oxazole-4-carboxamide;
N-(3-(difluoromethyl)-1-(1-((2-(2,6-dioxopiperidin-3-yl)-1,3-dioxoisoindolin-5-yl)methyl)piperidin-4-yl)-1H-pyrazol-4-yl)-5-morpholinopyrazolo[1,5-a]pyrimidine-3-carboxamide;
N-(3-(difluoromethyl)-1-(1-((2-(2,6-dioxopiperidin-3-yl)-4-fluoro-1,3-dioxoisoindolin-5-yl)methyl)piperidin-4-yl)-1H-pyrazol-4-yl)-5-morpholinopyrazolo[1,5-a]pyrimidine-3-carboxamide;
N-(3-(difluoromethyl)-1-(1-((2-(2,6-dioxopiperidin-3-yl)-6-fluoro-1,3-dioxoisoindolin-5-yl)methyl)piperidin-4-yl)-1H-pyrazol-4-yl)-5-morpholinopyrazolo[1,5-a]pyrimidine-3-carboxamide;
N-(3-(difluoromethyl)-1-(1-((2-(2,6-dioxopiperidin-3-yl)-7-fluoro-1,3-dioxoisoindolin-5-yl)methyl)piperidin-4-yl)-1H-pyrazol-4-yl)-5-morpholinopyrazolo[1,5-a]pyrimidine-3-carboxamide;
N-(2-(4-((2-(2,6-dioxopiperidin-3-yl)-1,3-dioxoisoindolin-5-yl)methyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide;
N-(2-(4-((2-(2,6-dioxopiperidin-3-yl)-4-fluoro-1,3-dioxoisoindolin-5-yl)methyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide;
N-(2-(4-((2-(2,6-dioxopiperidin-3-yl)-6-fluoro-1,3-dioxoisoindolin-5-yl)methyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide;
N-(2-(4-((2-(2,6-dioxopiperidin-3-yl)-7-fluoro-1,3-dioxoisoindolin-5-yl)methyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide;
4-((1-((2-(2,6-dioxopiperidin-3-yl)-1,3-dioxoisoindolin-5-yl)methyl)piperidin-4-yl)ethynyl)-1-(((2S,3S,4S)-3-ethyl-4-fluoro-5-oxopyrrolidin-2-yl)methoxy)-7-methoxyisoquinoline-6-carboxamide;
4-((1-((2-(2,6-dioxopiperidin-3-yl)-4-fluoro-1,3-dioxoisoindolin-5-yl)methyl)piperidin-4-yl)ethynyl)-1-(((2S,3S,4S)-3-ethyl-4-fluoro-5-oxopyrrolidin-2-yl)methoxy)-7-methoxyisoquinoline-6-carboxamide;
4-((1-((2-(2,6-dioxopiperidin-3-yl)-6-fluoro-1,3-dioxoisoindolin-5-yl)methyl)piperidin-4-yl)ethynyl)-1-(((2S,3S,4S)-3-ethyl-4-fluoro-5-oxopyrrolidin-2-yl)methoxy)-7-methoxyisoquinoline-6-carboxamide;
4-((1-((2-(2,6-dioxopiperidin-3-yl)-7-fluoro-1,3-dioxoisoindolin-5-yl)methyl)piperidin-4-yl)ethynyl)-1-(((2S,3S,4S)-3-ethyl-4-fluoro-5-oxopyrrolidin-2-yl)methoxy)-7-methoxyisoquinoline-6-carboxamide;
6-(6-(4-((2-(2,6-dioxopiperidin-3-yl)-1,3-dioxoisoindolin-5-yl)methyl)piperazin-1-yl)pyridin-3-yl)-1-i sopropyl-N-((6-methyl-2-oxo-4-propyl-1, 2-dihydropyridin-3 -yl)methyl)-1 H-indazole-4-carboxamide;
6-(6-(4-((2-(2,6-dioxopiperidin-3-yl)-4-fluoro-1,3-dioxoisoindolin-5-yl)methyl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-((2-(2,6-dioxopiperidin-3-yl)-6-fluoro-1,3-dioxoisoindolin-5-yl)methyl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-((2-(2,6-dioxopiperidin-3-yl)-7-fluoro-1,3-dioxoisoindolin-5-yl)methyl)piperazin-1-yl)pyridin-3 -yl)-1-isopropyl-N-((6-methyl-2-oxo-4-propyl-1,2-dihydropyridin-3-yl)methyl)-1H-indazole-4-carboxamide;
6-(6-(4-(((2-(2,6-dioxopiperidin-3-yl)-1,3-dioxoisoindolin-5-yl)methyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(((2-(2,6-dioxopiperidin-3-yl)-4-fluoro-1,3-dioxoisoindolin-5-yl)methyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(((2-(2,6-dioxopiperidin-3-yl)-6-fluoro-1,3-dioxoisoindolin-5-yl)methyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(((2-(2,6-dioxopiperidin-3-yl)-7-fluoro-1,3-dioxoisoindolin-5-yl)methyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-((2-(2,6-dioxopiperidin-3-yl)-1,3-dioxoisoindolin-5-yl)methyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-((2-(2,6-dioxopiperidin-3-yl)-4-fluoro-1,3-dioxoisoindolin-5-yl)methyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-((2-(2,6-dioxopiperidin-3-yl)-6-fluoro-1,3-dioxoisoindolin-5-yl)methyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-((2-(2,6-dioxopiperidin-3-yl)-7-fluoro-1,3-dioxoisoindolin-5-yl)methyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
N-((4,6-dimethyl-2-oxo-1,2-dihydropyridin-3-yl)methyl)-4'-((4-((2-(2,6-dioxopiperidin-3-yl)-1,3-dioxoisoindolin-5-yl)methyl)piperazin-1-yl)methyl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-((4-((2-(2,6-dioxopiperidin-3-yl)-4-fluoro-1,3-dioxoisoindolin-5-yl)methyl)piperazin-1-yl)methyl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-((4-((2-(2,6-dioxopiperidin-3-yl)-6-fluoro-1,3-dioxoisoindolin-5-yl)methyl)piperazin-1-yl)methyl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-((4-((2-(2,6-dioxopiperidin-3-yl)-7-fluoro-1,3-dioxoisoindolin-5-yl)methyl)piperazin-1-yl)methyl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-((2-(2,6-dioxopiperidin-3-yl)-1,3-dioxoisoindolin-5-yl)methyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-((2-(2,6-dioxopiperidin-3-yl)-4-fluoro-1,3-dioxoisoindolin-5-yl)methyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-((2-(2,6-dioxopiperidin-3-yl)-6-fluoro-1,3-dioxoisoindolin-5-yl)methyl)piperazin-1-yl)-5-(ethyl(tetrahydro-2H-pyran-4-yl)amino)-4-methyl-[1,1'-biphenyl]-3 -carboxamide;
N-((4,6-dimethyl-2-oxo-1,2-dihydropyridin-3-yl)methyl)-4'-(4-((2-(2,6-dioxopiperidin-3-yl)-7-fluoro-1,3-dioxoisoindolin-5-yl)methyl)piperazin-1-yl)-5-(ethyl(tetrahydro-2H-pyran-4-yl)amino)-4-methyl-[1,1'-biphenyl]-3 -carboxamide;
N-((4,6-dimethyl-2-oxo-1,2-dihydropyridin-3-yl)methyl)-4'-(4-((2-(2,6-dioxopiperidin-3-yl)-1,3-dioxoisoindolin-5-yl)methyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-((2-(2,6-dioxopiperidin-3-yl)-4-fluoro-1,3-dioxoisoindolin-5-yl)methyl)piperazin-1-yl)-5-(ethyl(tetrahydro-2H-pyran-4-yl)amino)-4-methyl-[1,1'-biphenyl]-3 -carboxamide;
N-((4,6-dimethyl-2-oxo-1,2-dihydropyridin-3-yl)methyl)-4'-(4-((2-(2,6-dioxopiperidin-3-yl)-6-fluoro-1,3-dioxoisoindolin-5-yl)methyl)piperazin-1-yl)-5-(ethyl(tetrahydro-2H-pyran-4-yl)amino)-4-methyl-[1,1'-biphenyl]-3 -carboxamide;
N-((4,6-dimethyl-2-oxo-1,2-dihydropyridin-3-yl)methyl)-4'-(4-((2-(2,6-dioxopiperidin-3-yl)-7-fluoro-1,3-dioxoisoindolin-5-yl)methyl)piperazin-1-yl)-5-(ethyl(tetrahydro-2H-pyran-4-yl)amino)-4-methyl-[1,1'-biphenyl]-3-carboxamide;
N-(5-(3,5-difluorobenzyl)-1H-indazol-3-yl)-4-(4-((2-(2,6-dioxopiperidin-3-yl)-4-fluoro-1,3-dioxoisoindolin-5-yl)methyl)piperazin-1-yl)-2-((tetrahydro-2H-pyran-4-yl)amino)benzamide;
N-(5-(3,5-difluorobenzyl)-1H-indazol-3-yl)-4-(4-((2-(2,6-dioxopiperidin-3-yl)-6-fluoro-1,3-dioxoisoindolin-5-yl)methyl)piperazin-1-yl)-2-((tetrahydro-2H-pyran-4-yl)amino)benzamide;
N-(5-(3,5-difluorobenzyl)-1H-indazol-3-yl)-4-(4-((2-(2,6-dioxopiperidin-3-yl)-7-fluoro-1,3-dioxoisoindolin-5-yl)methyl)piperazin-1-yl)-2-((tetrahydro-2H-pyran-4-yl)amino)benzamide;
N-(5-(3,5-difluorobenzyl)-1H-indazol-3-yl)-4-(4-(((2-(2,6-dioxopiperidin-3-yl)-1,3-dioxoisoindolin-5-yl)methyl)(methyl)amino)piperidin-1-yl)-2-((tetrahydro-2H-pyran-4-yl)amino)benzamide;
N-(5-(3,5-difluorobenzyl)-1H-indazol-3-yl)-4-(4-(((2-(2,6-dioxopiperidin-3-yl)-4-fluoro-1,3-dioxoisoindolin-5-yl)methyl)(methyl)amino)piperidin-1-yl)-2-((tetrahydro-2H-pyran-4-yl)amino)benzamide;
N-(5-(3,5-difluorobenzyl)-1H-indazol-3-yl)-4-(4-(((2-(2,6-dioxopiperidin-3-yl)-6-fluoro-1,3-dioxoisoindolin-5-yl)methyl)(methyl)amino)piperidin-1-yl)-2-((tetrahydro-2H-pyran-4-yl)amino)benzamide;
N-(5-(3,5-difluorobenzyl)-1H-indazol-3-yl)-4-(4-(((2-(2,6-dioxopiperidin-3-yl)-7-fluoro-1,3-dioxoisoindolin-5-yl)methyl)(methyl)amino)piperidin-1-yl)-2-((tetrahydro-2H-pyran-4-yl)amino)benzamide;
N-(5-(3,5-difluorobenzyl)-1H-indazol-3-yl)-4-(4-(1-((2-(2,6-dioxopiperidin-3-yl)-4-fluoro-1,3-dioxoisoindolin-5-yl)methyl)piperidin-4-yl)piperazin-1-yl)-2-((tetrahydro-2H-pyran-4-yl)amino)benzamide;
N-(5-(3,5-difluorobenzyl)-1H-indazol-3-yl)-4-(4-(1-((2-(2,6-dioxopiperidin-3-yl)-6-fluoro-1,3-dioxoisoindolin-5-yl)methyl)piperidin-4-yl)piperazin-1-yl)-2-((tetrahydro-2H-pyran-4-yl)amino)benzamide;
N-(5-(3,5-difluorobenzyl)-1H-indazol-3-yl)-4-(4-(1-((2-(2,6-dioxopiperidin-3-yl)-7-fluoro-1,3-dioxoisoindolin-5-yl)methyl)piperidin-4-yl)piperazin-1-yl)-2-((tetrahydro-2H-pyran-4-yl)amino)benzamide;
2-(2,6-dioxopiperidin-3-yl)-5-((4-(6-(6-((R)-2-(3-fluorophenyl)pyrrolidin-1-yl)imidazo[1,2-b]pyridazin-3-yl)pyridin-2-yl)piperazin-1-yl)methyl)isoindoline-1,3-dione;
2-(2,6-dioxopiperidin-3-yl)-4-fluoro-5-((4-(6-(6-((R)-2-(3-fluorophenyl)pyrrolidin-1-yl)imidazo[1,2-b]pyridazin-3-yl)pyridin-2-yl)piperazin-1-yl)methyl)isoindoline-1,3-dione;
2-(2,6-dioxopiperidin-3-yl)-5-fluoro-6-((4-(6-(6-((R)-2-(3-fluorophenyl)pyrrolidin-1-yl)imidazo[1,2-b]pyridazin-3-yl)pyridin-2-yl)piperazin-1-yl)methyl)isoindoline-1,3-dione;
2-(2, 6-dioxopiperidin-3-yl)-4-fluoro-6-((4-(6-(6-((R)-2-(3-fluorophenyl)pyrroli din-1-yl)imidazo[1,2-b]pyridazin-3-yl)pyridin-2-yl)piperazin-1-yl)methyl)isoindoline-1,3-dione;
2-(2,6-dioxopiperidin-3-yl)-5-(((1-(6-(6-((R)-2-(3-fluorophenyl)pyrrolidin-1-yl)imidazo[1,2-b]pyridazin-3-yl)pyridin-2-yl)piperidin-4-yl)(methyl)amino)methyl)isoindoline-1,3-dione;
2-(2,6-dioxopiperidin-3-yl)-4-fluoro-5-(((1-(6-(6-((R)-2-(3-fluorophenyl)pyrrolidin-1-yl)imidazo[1,2-b]pyridazin-3-yl)pyridin-2-yl)piperidin-4-yl)(methyl)amino)methyl)isoindoline-1,3-dione;
2-(2,6-dioxopiperidin-3-yl)-5-fluoro-6-(((1-(6-(6-((R)-2-(3-fluorophenyl)pyrrolidin-1-yl)imidazo[1,2-b]pyridazin-3-yl)pyridin-2-yl)piperidin-4-yl)(methyl)amino)methyl)isoindoline-1,3-dione;
2-(2,6-dioxopiperidin-3-yl)-4-fluoro-6-(((1-(6-(6-((R)-2-(3-fluorophenyl)pyrrolidin-1-yl)imidazo[1,2-b]pyridazin-3-yl)pyridin-2-yl)piperidin-4-yl)(methyl)amino)methyl)isoindoline-1,3-dione;
2-(2,6-dioxopiperidin-3-yl)-5-((4-(4-(6-(6-((R)-2-(3-fluorophenyl)pyrrolidin-1-yl)imidazo[1,2-b]pyridazin-3-yl)pyridin-2-yl)piperazin-1-yl)piperidin-1-yl)methyl)isoindoline-1,3-dione;
2-(2,6-dioxopiperidin-3-yl)-4-fluoro-5-((4-(4-(6-(6-((R)-2-(3-fluorophenyl)pyrrolidin-1-yl)imidazo[1,2-b]pyridazin-3-yl)pyridin-2-yl)piperazin-1-yl)piperidin-1-yl)methyl)isoindoline-1,3-dione;
2-(2,6-dioxopiperidin-3-yl)-5-fluoro-6-((4-(4-(6-(6-((R)-2-(3-fluorophenyl)pyrrolidin-1-yl)imidazo[1,2-b]pyridazin-3-yl)pyridin-2-yl)piperazin-1-yl)piperidin-1-yl)methyl)isoindoline-1,3-dione;
2-(2,6-dioxopiperidin-3-yl)-4-fluoro-6-((4-(4-(6-(6-((R)-2-(3-fluorophenyl)pyrrolidin-1-yl)imidazo[1,2-b]pyridazin-3-yl)pyridin-2-yl)piperazin-1-yl)piperidin-1-yl)methyl)isoindoline-1,3-dione;
5-((4-(6-(5-((R)-2-(2,4-difluorophenyl)pyrrolidin-1-yl)pyrazolo[1,5-a]pyrimidin-3-yl)pyridin-2-yl)piperazin-1-yl)methyl)-2-(2,6-dioxopiperidin-3-yl)isoindoline-1,3-dione;
5-((4-(6-(5-((R)-2-(2,4-difluorophenyl)pyrrolidin-1-yl)pyrazolo[1,5-a]pyrimidin-3-yl)pyridin-2-yl)pip erazin-1-yl)methyl)-2-(2, 6-dioxopip eri din-3 -yl)-4-fl uoroisoindoline-1, 3 -dione;
5-((4-(6-(5-((R)-2-(2,4-difluorophenyl)pyrrolidin-1-yl)pyrazolo[1,5-a]pyrimidin-3-yl)pyridin-2-yl)piperazin-1-yl)methyl)-2-(2,6-dioxopiperidin-3-yl)-6-fluoroisoindoline-1,3-dione;
6-((4-(6-(5-((R)-2-(2,4-difluorophenyl)pyrrolidin-1-yl)pyrazolo[1,5-a]pyrimidin-3-yl)pyridin-2-yl)pip erazin-1-yl)methyl)-2-(2, 6-dioxopip eri din-3 -yl)-4-fl uoroisoindoline-1, 3 -dione;
5-(((1-(6-(5-((R)-2-(2,4-difluorophenyl)pyrrolidin-1-yl)pyrazolo[1,5-a]pyrimidin-3-yl)pyridin-2-yl)piperidin-4-yl)(methyl)amino)methyl)-2-(2,6-dioxopiperidin-3-yl)isoindoline-1,3-dione;
5-(((1-(6-(5-((R)-2-(2,4-difluorophenyl)pyrrolidin-1-yl)pyrazolo[1,5-a]pyrimidin-3-yl)pyridin-2-yl)piperidin-4-yl)(methyl)amino)methyl)-2-(2,6-dioxopiperidin-3-yl)-4-fluoroisoindoline-1,3-dione;
5-(((1-(6-(5-((R)-2-(2,4-difluorophenyl)pyrrolidin-1-yl)pyrazolo[1,5-a]pyrimidin-3-yl)pyridin-2-yl)piperidin-4-yl)(methyl)amino)methyl)-2-(2,6-dioxopiperidin-3-yl)-6-fluoroisoindoline-1,3-dione;
6-(((1-(6-(5-((R)-2-(2,4-difluorophenyl)pyrrolidin-1-yl)pyrazolo[1,5-a]pyrimidin-3-yl)pyridin-2-yl)piperidin-4-yl)(methyl)amino)methyl)-2-(2,6-dioxopiperidin-3-yl)-4-fluoroisoindoline-1,3-dione;
5-((4-(4-(6-(5-((R)-2-(2,4-difluorophenyl)pyrrolidin-1-yl)pyrazolo[1,5-a]pyrimidin-3-yl)pyridin-2-yl)piperazin-1-yl)piperidin-1-yl)methyl)-2-(2,6-dioxopiperidin-3-yl)isoindoline-1,3-dione;
5-((4-(4-(6-(5-((R)-2-(2,4-difluorophenyl)pyrrolidin-1-yl)pyrazolo[1,5-a]pyrimidin-3-yl)pyridin-2-yl)piperazin-1-yl)piperidin-1-yl)methyl)-2-(2,6-dioxopiperidin-3-yl)-4-fluoroisoindoline-1,3-dione;
5-((4-(4-(6-(5-((R)-2-(2,4-difluorophenyl)pyrrolidin-1-yl)pyrazolo[1,5-a]pyrimidin-3-yl)pyridin-2-yl)piperazin-1-yl)piperidin-1-yl)methyl)-2-(2,6-dioxopiperidin-3-yl)-6-fluoroisoindoline-1,3-dione;
6-((4-(4-(6-(5-((R)-2-(2,4-difluorophenyl)pyrrolidin-1-yl)pyrazolo[1,5-a]pyrimidin-3-yl)pyridin-2-yl)piperazin-1-yl)piperidin-1-yl)methyl)-2-(2,6-dioxopiperidin-3-yl)-4-fluoroisoindoline-1,3-dione;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1,3-dioxoisoindolin-5-yl)methyl)piperazin-1-yl-2,2,3,3,5,5,6,6-*d*₈)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-4-fluoro-1,3-dioxoisoindolin-5-yl)methyl)piperazin-1-yl-2,2,3,3,5,5,6,6-*d*₈)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-6-fluoro-1,3-dioxoisoindolin-5-yl)methyl)piperazin-1-yl-2,2,3,3,5,5,6,6-*d*₈)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-7-fluoro-1,3-dioxoisoindolin-5-yl)methyl)piperazin-1-yl-2,2,3,3,5,5,6,6-*d*₈)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1,3-dioxoisoindolin-5-yl)methyl)-3,5-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-4-fluoro-1,3-dioxoisoindolin-5-yl)methyl)-3,5-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-6-fluoro-1,3-dioxoisoindolin-5-yl)methyl)-3,5-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-7-fluoro-1,3-dioxoisoindolin-5-yl)methyl)-3,5-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1,3-dioxoisoindolin-5-yl)methyl)-2,6-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-4-fluoro-1,3-dioxoisoindolin-5-yl)methyl)-2,6-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-6-fluoro-1,3-dioxoisoindolin-5-yl)methyl)-2,6-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-7-fluoro-1,3-dioxoisoindolin-5-yl)methyl)-2,6-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(6-((2-(2,6-dioxopiperidin-3-yl)-1,3-dioxoisoindolin-5-yl)methyl)-3,6-diazabicyclo[3.1.1]heptan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(6-((2-(2,6-dioxopiperidin-3-yl)-4-fluoro-1,3-dioxoisoindolin-5-yl)methyl)-3,6-diazabicyclo[3.1.1]heptan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(6-((2-(2,6-dioxopiperidin-3-yl)-6-fluoro-1,3-dioxoisoindolin-5-yl)methyl)-3,6-diazabicyclo[3.1.1]heptan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(6-((2-(2,6-dioxopiperidin-3-yl)-7-fluoro-1,3-dioxoisoindolin-5-yl)methyl)-3,6-diazabicyclo[3.1.1]heptan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-1,3-dioxoisoindolin-5-yl)methyl)-3,6-diazabicyclo[3.1.1]heptan-6-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-4-fluoro-1,3-dioxoisoindolin-5-yl)methyl)-3,6-diazabicyclo[3.1.1]heptan-6-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-6-fluoro-1,3-dioxoisoindolin-5-yl)methyl)-3,6-diazabicyclo[3.1.1]heptan-6-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-7-fluoro-1,3-dioxoisoindolin-5-yl)methyl)-3,6-diazabicyclo[3.1.1]heptan-6-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-((2-(2,6-dioxopiperidin-3-yl)-1,3-dioxoisoindolin-5-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-((2-(2,6-dioxopiperidin-3-yl)-4-fluoro-1,3-dioxoisoindolin-5-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-((2-(2,6-dioxopiperidin-3-yl)-6-fluoro-1,3-dioxoisoindolin-5-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-((2-(2,6-dioxopiperidin-3-yl)-7-fluoro-1,3-dioxoisoindolin-5-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-1,3-dioxoisoindolin-5-yl)methyl)-3,8-diazabicyclo[3.2.1]octan-8-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-4-fluoro-1,3-dioxoisoindolin-5-yl)methyl)-3,8-diazabicyclo[3.2.1]octan-8-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-6-fluoro-1,3-dioxoisoindolin-5-yl)methyl)-3,8-diazabicyclo[3.2.1]octan-8-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-7-fluoro-1,3-dioxoisoindolin-5-yl)methyl)-3,8-diazabicyclo[3.2.1]octan-8-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1R,4R)-5-((2-(2,6-dioxopiperidin-3-yl)-1,3-dioxoisoindolin-5-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1R,4R)-5-((2-(2,6-dioxopiperidin-3-yl)-4-fluoro-1,3-dioxoisoindolin-5-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1R,4R)-5-((2-(2,6-dioxopiperidin-3-yl)-6-fluoro-1,3-dioxoisoindolin-5-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1R,4R)-5-((2-(2,6-dioxopiperidin-3-yl)-7-fluoro-1,3-dioxoisoindolin-5-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1 S,4S)-5-((2-(2,6-dioxopiperidin-3-yl)-1,3-dioxoisoindolin-5-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1S,4S)-5-((2-(2,6-dioxopiperidin-3-yl)-4-fluoro-1,3-dioxoisoindolin-5-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1S,4S)-5-((2-(2,6-dioxopiperidin-3-yl)-6-fluoro-1,3-dioxoisoindolin-5-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1S,4S)-5-((2-(2,6-dioxopiperidin-3-yl)-7-fluoro-1,3-dioxoisoindolin-5-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(5-((2-(2,6-dioxopiperidin-3-yl)-1,3-dioxoisoindolin-5-yl)methyl)-2,5-diazabicyclo[2.2.2]octan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(5-((2-(2,6-dioxopiperidin-3-yl)-4-fluoro-1,3-dioxoisoindolin-5-yl)methyl)-2,5-diazabicyclo[2.2.2]octan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(5-((2-(2,6-dioxopiperidin-3-yl)-7-fluoro-1,3-dioxoisoindolin-5-yl)methyl)-2,5-diazabicyclo[2.2.2]octan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(5-((2-(2,6-dioxopiperidin-3-yl)-6-fluoro-1,3-dioxoisoindolin-5-yl)methyl)-2,5-diazabicyclo[2.2.2]octan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-4-fluoro-1,3-dioxoisoindolin-5-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-6-fluoro-1,3-dioxoisoindolin-5-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-7-fluoro-1,3-dioxoisoindolin-5-yl)methyl)piperazin-1-yl)-1-(phenylthio)butan-2-yl)amino)-3-((trifluoromethyl)sulfonyl)phenyl)sulfonyl)benzamide;
2-(4-((((2-(2,6-dioxopiperidin-3-yl)-1-oxoisoindolin-5-yl)methyl)(methyl)amino)methyl)phenyl)-5-fluorobenzofuran-7-carboxamide;
2-(4-((((2-(2,6-dioxopiperidin-3-yl)-4-fluoro-1-oxoisoindolin-5-yl)methyl)(methyl)amino)methyl)phenyl)-5-fluorobenzofuran-7-carboxamide;
2-(4-((((2-(2,6-dioxopiperidin-3-yl)-6-fluoro-1-oxoisoindolin-5-yl)methyl)(methyl)amino)methyl)phenyl)-5-fluorobenzofuran-7-carboxamide;
2-(4-((((2-(2,6-dioxopiperidin-3-yl)-7-fluoro-1-oxoisoindolin-5-yl)methyl)(methyl)amino)methyl)phenyl)-5-fluorobenzofuran-7-carboxamide;
2-(4-((((2-(2,6-dioxopiperidin-3-yl)-1,3-dioxoisoindolin-5-yl)methyl)(methyl)amino)methyl)phenyl)-5-fluorobenzofuran-7-carboxamide;
2-(4-((((2-(2,6-dioxopiperidin-3-yl)-4-fluoro-1,3-dioxoisoindolin-5-yl)methyl)(methyl)amino)methyl)phenyl)-5-fluorobenzofuran-7-carboxamide;
2-(4-((((2-(2,6-dioxopiperidin-3-yl)-6-fluoro-1,3-dioxoisoindolin-5-yl)methyl)(methyl)amino)methyl)phenyl)-5-fluorobenzofuran-7-carboxamide;
2-(4-((((2-(2,6-dioxopiperidin-3-yl)-7-fluoro-1,3-dioxoisoindolin-5-yl)methyl)(methyl)amino)methyl)phenyl)-5-fluorobenzofuran-7-carboxamide;
N-(2-(4-((6-bromo-2-(2,6-dioxopiperidin-3-yl)-1,3-dioxoisoindolin-5-yl)methyl)piperazin-1-yl)ethyl)-4,9-dioxo-4,9-dihydronaphtho[2,3-b]furan-2-carboxamide;
5-((4-(1-(4,9-dioxo-4,9-dihydronaphtho[2,3-b]furan-2-yl)ethyl)piperazin-1-yl)methyl)-2-(2,6-dioxopiperidin-3 -yl)isoindoline-1,3 -dione;
N-((4-bromo-2-(2,6-dioxopiperidin-3-yl)-1,3-dioxoisoindolin-5-yl)methyl)-4,9-dioxo-4,9-dihydronaphtho[2,3 -b]furan-2-carboxamide;
N-((6-bromo-2-(2,6-dioxopiperidin-3-yl)-1,3-dioxoisoindolin-5-yl)methyl)-4,9-dioxo-4,9-dihydronaphtho[2,3 -b]furan-2-carboxamide;
N-((7-bromo-2-(2,6-dioxopiperidin-3-yl)-1,3-dioxoisoindolin-5-yl)methyl)-4,9-dioxo-4,9-dihydronaphtho[2,3 -b]furan-2-carboxamide;
((2-(((5S,8S,10aR)-8-(((2S)-5-amino-1-(3-((6-bromo-2-(2,6-dioxopiperidin-3-yl)-1,3-dioxoisoindolin-5-yl)methyl)-2-chlorophenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(3-(4-((4-bromo-2-(2,6-dioxopiperidin-3-yl)-1,3-dioxoisoindolin-5-yl)methyl)piperazin-1-yl)-2-chlorophenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(3-(4-((7-bromo-2-(2,6-dioxopiperidin-3-yl)-1,3-dioxoisoindolin-5-yl)methyl)piperazin-1-yl)-2-chlorophenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((1-((4-bromo-2-(2,6-dioxopiperidin-3-yl)-1,3-dioxoisoindolin-5-yl)methyl)piperidin-4-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((6-bromo-2-(2,6-dioxopiperidin-3-yl)-1,3-dioxoisoindolin-5-yl)methyl)-6-oxodecahydropyrrolo[1,2-a][1,5]diazocin-5-yl)carbamoyl)-1H-indol-5-yl)difluoromethyl)phosphonic acid;
4-bromo-2-(2,6-dioxopiperidin-3-yl)-6-(((3-((3aR,4S,9bR)-4-(hydroxymethyl)-1-(pyridin-4-ylmethyl)-2,3,3a,4,5,9b-hexahydro-1H-pyrrolo[3,2-c]quinolin-8-yl)phenyl)amino)methyl)isoindoline-1,3-dione;
6-(1-((4-bromo-2-(2,6-dioxopiperidin-3-yl)-1,3-dioxoisoindolin-5-yl)methyl)piperidin-4-yl)-2-(4-phenoxyphenyl)nicotinamide;
6-(1-((6-bromo-2-(2,6-dioxopiperidin-3-yl)-1,3-dioxoisoindolin-5-yl)methyl)piperidin-4-yl)-2-(4-phenoxyphenyl)nicotinamide;
6-(1-((7-bromo-2-(2,6-dioxopiperidin-3-yl)-1,3-dioxoisoindolin-5-yl)methyl)piperidin-4-yl)-2-(4-phenoxyphenyl)nicotinamide;
6-(4-((4-bromo-2-(2,6-dioxopiperidin-3-yl)-1,3-dioxoisoindolin-5-yl)methyl)piperazin-1-yl)-2-(4-phenoxyphenyl)nicotinamide;
6-(4-(((6-bromo-2-(2,6-dioxopiperidin-3-yl)-1,3-dioxoisoindolin-5-yl)methyl)(methyl)amino)piperidin-1-yl)-2-(4-phenoxyphenyl)nicotinamide;
6-(4-(4-((7-bromo-2-(2,6-dioxopiperidin-3-yl)-1,3-dioxoisoindolin-5-yl)methyl)piperazin-1-yl)piperidin-1-yl)-2-(4-phenoxyphenyl)nicotinamide;
N-(4-(5-(4-(4-((4-bromo-2-(2,6-dioxopiperidin-3-yl)-1,3-dioxoisoindolin-5-yl)methyl)piperazin-1-yl)phenyl)-1H-indazol-3-yl)-2-methylbenzyl)-3-(tert-butyl)-1,2,4-oxadiazole-5-carboxamide;
N-(4-(5-(4-(4-(((6-bromo-2-(2,6-dioxopiperidin-3-yl)-1,3-dioxoisoindolin-5-yl)methyl)(methyl)amino)piperidin-1-yl)phenyl)-1H-indazol-3-yl)-2-methylbenzyl)-3-(tert-butyl)-1,2,4-oxadiazole-5-carboxamide;
N-(4-(5-(4-(4-(4-((7-bromo-2-(2,6-dioxopiperidin-3-yl)-1,3-dioxoisoindolin-5-yl)methyl)piperazin-1-yl)piperidin-1-yl)phenyl)-1H-indazol-3 -yl)-2-methylbenzyl)-3 -(tert-butyl)-1,2,4-oxadiazole-5-carboxamide;
4-bromo-6-((4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)methyl)-2-(2,6-dioxopiperidin-3-yl)isoindoline-1,3-dione;
4-bromo-5-((4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)methyl)-2-(2,6-dioxopiperidin-3-yl)isoindoline-1,3-dione;
5-bromo-6-((4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)methyl)-2-(2,6-dioxopiperidin-3-yl)isoindoline-1,3-dione;
4-bromo-5-((3-(4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)azetidin-1-yl)methyl)-2-(2,6-dioxopiperidin-3-yl)isoindoline-1,3-dione;
4-bromo-6-(((1-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperidin-4-yl)(methyl)amino)methyl)-2-(2,6-dioxopiperidin-3-yl)isoindoline-1,3-dione;
4-bromo-5-((4-(1-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperidin-4-yl)piperazin-1-yl)methyl)-2-(2,6-dioxopiperidin-3-yl)isoindoline-1,3-dione;
5-((4-(4-amino-3-(4-phenoxyphenyl)-1H-pyrazolo[3,4-d]pyrimidin-1-yl)piperidin-1-yl)methyl)-4-bromo-2-(2,6-dioxopiperidin-3-yl)isoindoline-1,3-dione;
5-((4-(4-amino-3-(4-phenoxyphenyl)-1H-pyrazolo[3,4-d]pyrimidin-1-yl)piperidin-1-yl)methyl)-6-bromo-2-(2,6-dioxopiperidin-3-yl)isoindoline-1,3-dione;
6-((4-(4-amino-3-(4-phenoxyphenyl)-1H-pyrazolo[3,4-d]pyrimidin-1-yl)piperidin-1-yl)methyl)-4-bromo-2-(2,6-dioxopiperidin-3-yl)isoindoline-1,3-dione;
5-((4-(4-(1,2-bis(4-hydroxyphenyl)but-1-en-1-yl)phenyl)piperazin-1-yl)methyl)-4-bromo-2-(2,6-dioxopiperidin-3 -yl)isoindoline-1,3 -dione;
5-((4-(4-(1,2-bis(4-hydroxyphenyl)but-1-en-1-yl)phenyl)piperazin-1-yl)methyl)-6-bromo-2-(2,6-dioxopiperidin-3 -yl)isoindoline-1,3 -dione;
6-((4-(4-(1,2-bis(4-hydroxyphenyl)but-1-en-1-yl)phenyl)piperazin-1-yl)methyl)-4-bromo-2-(2,6-dioxopiperidin-3 -yl)isoindoline-1,3 -dione;
4-bromo-2-(2,6-dioxopiperidin-3-yl)-5-((4-(4-((1R,2S)-6-hydroxy-2-phenyl-1,2,3,4-tetrahydronaphthalen-1-yl)phenyl)piperazin-1-yl)methyl)isoindoline-1,3 -dione;
5-bromo-2-(2,6-dioxopiperidin-3-yl)-6-((4-(4-((1R,2S)-6-hydroxy-2-phenyl-1,2,3,4-tetrahydronaphthalen-1-yl)phenyl)piperazin-1-yl)methyl)isoindoline-1,3 -dione;
4-bromo-2-(2,6-dioxopiperidin-3-yl)-6-((4-(4-((1R,2S)-6-hydroxy-2-phenyl-1,2,3,4-tetrahydronaphthalen-1-yl)phenyl)piperazin-1-yl)methyl)isoindoline-1,3 -dione;
4-bromo-2-(2,6-dioxopiperidin-3-yl)-5-((4-(4-((3S,4R)-7-hydroxy-3-phenylchroman-4-yl)phenyl)piperazin-1-yl)methyl)isoindoline-1,3-dione;
5-bromo-2-(2,6-dioxopiperidin-3-yl)-6-((4-(4-((3S,4R)-7-hydroxy-3-phenylchroman-4-yl)phenyl)piperazin-1-yl)methyl)isoindoline-1,3-dione;
4-bromo-2-(2,6-dioxopiperidin-3-yl)-6-((4-(4-((3S,4R)-7-hydroxy-3-phenyl chroman-4-yl)phenyl)piperazin-1-yl)methyl)isoindoline-1,3-dione;
4-bromo-2-(2,6-dioxopiperidin-3-yl)-5-(((1-(4-((3S,4R)-7-hydroxy-3-phenylchroman-4-yl)phenyl)piperidin-4-yl)(methyl)amino)methyl)isoindoline-1,3-dione;
5-bromo-2-(2,6-dioxopiperidin-3-yl)-6-(((1-(4-((3S,4R)-7-hydroxy-3-phenylchroman-4-yl)phenyl)piperidin-4-yl)(methyl)amino)methyl)isoindoline-1,3-dione;
4-bromo-2-(2,6-dioxopiperidin-3-yl)-6-(((1-(4-((3S,4R)-7-hydroxy-3-phenylchroman-4-yl)phenyl)piperidin-4-yl)(methyl)amino)methyl)isoindoline-1,3-dione;
4-bromo-2-(2,6-dioxopiperidin-3-yl)-5-((4-(1-(4-((3 S,4R)-7-hydroxy-3-phenylchroman-4-yl)phenyl)piperidin-4-yl)piperazin-1-yl)methyl)isoindoline-1,3-dione;
4-bromo-2-(2,6-dioxopiperidin-3-yl)-5-(((1-(4-((1R,2S)-6-hydroxy-2-phenyl-1,2,3,4-tetrahydronaphthalen-1-yl)phenyl)piperidin-4-yl)(methyl)amino)methyl)isoindoline-1,3-dione;
5-bromo-2-(2,6-dioxopiperidin-3-yl)-6-(((1-(4-((1R,2S)-6-hydroxy-2-phenyl-1,2,3,4-tetrahydronaphthalen-1-yl)phenyl)piperidin-4-yl)(methyl)amino)methyl)isoindoline-1,3-dione;
4-bromo-2-(2,6-dioxopiperidin-3-yl)-6-(((1-(4-((1R,2S)-6-hydroxy-2-phenyl-1,2,3,4-tetrahydronaphthalen-1-yl)phenyl)piperidin-4-yl)(methyl)amino)methyl)isoindoline-1,3-dione;
5-(((2-(4-(1,2-bis(4-hydroxyphenyl)but-1-en-1-yl)phenoxy)ethyl)(methyl)amino)methyl)-4-bromo-2-(2,6-dioxopiperidin-3-yl)isoindoline-1,3-dione;
5-(((2-(4-(1,2-bis(4-hydroxyphenyl)but-1-en-1-yl)phenoxy)ethyl)(methyl)amino)methyl)-6-bromo-2-(2,6-dioxopiperidin-3-yl)isoindoline-1,3-dione;
6-(((2-(4-(1,2-bis(4-hydroxyphenyl)but-1-en-1-yl)phenoxy)ethyl)(methyl)amino)methyl)-4-bromo-2-(2,6-dioxopiperidin-3-yl)isoindoline-1,3-dione;
4-bromo-5-(((1-(3-(2,3-dichlorophenyl)-1H-pyrazolo[3,4-b]pyrazin-6-yl)-4-methylpiperidin-4-yl)amino)methyl)-2-(2,6-dioxopiperidin-3-yl)isoindoline-1,3-dione;
5-((4-(3-(6-(4-amino-4-methylpiperidin-1-yl)-1H-pyrazolo[3,4-b]pyrazin-3-yl)-2-chlorophenyl)piperazin-1-yl)methyl)-6-bromo-2-(2,6-dioxopiperidin-3-yl)isoindoline-1,3-dione;
6-((4-(3-((3-amino-5-(4-amino-4-methylpiperidin-1-yl)pyrazin-2-yl)thio)-2-chlorophenyl)piperazin-1-yl)methyl)-4-bromo-2-(2,6-dioxopiperidin-3-yl)isoindoline-1,3-dione;
5-((4-(3-(3-amino-5-(4-amino-4-methylpiperidin-1-yl)pyrazin-2-yl)-2-chlorophenyl)piperazin-1-yl)methyl)-4-bromo-2-(2,6-dioxopiperidin-3-yl)isoindoline-1,3-dione;
5-((((3 S,4S)-8-(6-amino-5-((2-amino-3 -chloropyridin-4-yl)thio)pyrazin-2-yl)-3 -methyl-2-oxa-8-azaspiro[4.5]decan-4-yl)amino)methyl)-6-bromo-2-(2,6-dioxopiperidin-3-yl)isoindoline-1,3-dione;
6-((4-(4-((3 -amino-5-((3 S,4S)-4-amino-3 -methyl-2-oxa-8-azaspiro[4.5]decan-8-yl)pyrazin-2-yl)thio)pyridin-2-yl)piperazin-1-yl)methyl)-4-bromo-2-(2,6-dioxopiperidin-3-yl)isoindoline-1,3-dione;
5-(((1-(6-amino-5-(2,3-dichlorophenyl)pyrazin-2-yl)-4-methylpiperidin-4-yl)amino)methyl)-4-bromo-2-(2,6-dioxopiperidin-3-yl)isoindoline-1,3-dione;
4-bromo-5-((2,3-difluoro-6-(2-morpholinothiazol-4-yl)phenoxy)methyl)-2-(2,6-dioxopiperidin-3 -yl)isoindoline-1,3 -dione;
6-(4-(4-((6-bromo-2-(2,6-dioxopiperidin-3-yl)-1,3-dioxoisoindolin-5-yl)methyl)piperazin-1-yl)piperidin-1-yl)-N-((1r,4r)-4-(3-chloro-4-cyanophenoxy)cyclohexyl)pyridazine-3-carboxamide;
6-(4-((7-bromo-2-(2,6-dioxopiperidin-3-yl)-1,3-dioxoisoindolin-5-yl)methyl)piperazin-1-yl)-N-((1r,3r)-3-(3-chloro-4-cyanophenoxy)-2,2,4,4-tetramethylcyclobutyl)pyridazine-3-carboxamide;
6-(4-((4-bromo-2-(2,6-dioxopiperidin-3-yl)-1,3-dioxoisoindolin-5-yl)methyl)piperazin-1-yl)-N-((1r,3r)-3-(3-chloro-4-cyanophenoxy)-2,2,4,4-tetramethylcyclobutyl)nicotinamide;
6-(8-((6-bromo-2-(2,6-dioxopiperidin-3-yl)-1,3-dioxoisoindolin-5-yl)methyl)-3,8-diazabicyclo[3.2.1]octan-3-yl)-N-((1r,4r)-4-(3-chloro-4-cyanophenoxy)cyclohexyl)pyridazine-3-carboxamide;
6-(6-((7-bromo-2-(2,6-dioxopiperidin-3-yl)-1,3-dioxoisoindolin-5-yl)methyl)-3,6-diazabicyclo[3.1.1]heptan-3-yl)-N-((1r,4r)-4-(3-chloro-4-cyanophenoxy)cyclohexyl)pyridazine-3-carboxamide;
6-(3-((4-bromo-2-(2,6-dioxopiperidin-3-yl)-1,3-dioxoisoindolin-5-yl)methyl)-3,6-diazabicyclo[3.1.1]heptan-6-yl)-N-((1r,4r)-4-(3-chloro-4-cyanophenoxy)cyclohexyl)pyridazine-3-carboxamide;
6-(3-((6-bromo-2-(2,6-dioxopiperidin-3-yl)-1,3-dioxoisoindolin-5-yl)methyl)-3,8-diazabicyclo[3.2.1]octan-8-yl)-N-((1r,4r)-4-(3-chloro-4-cyanophenoxy)cyclohexyl)pyridazine-3-carboxamide;
6-(4-((7-bromo-2-(2,6-dioxopiperidin-3-yl)-1,3-dioxoisoindolin-5-yl)methyl)piperazin-1-yl)-N-((1r,4r)-4-(3-chloro-4-cyanophenoxy)cyclohexyl)pyridazine-3-carboxamide;
4-bromo-5-((4-(4-((9-cyclopentyl-8-(phenylamino)-9H-purin-2-yl)amino)phenyl)piperazin-1-yl)methyl)-2-(2,6-dioxopiperidin-3-yl)isoindoline-1,3-dione;
5-bromo-6-((4-(4-((9-cyclopentyl-8-(phenylamino)-9H-purin-2-yl)amino)phenyl)piperazin-1-yl)methyl)-2-(2,6-dioxopiperidin-3-yl)isoindoline-1,3-dione;
4-bromo-6-((4-(4-((9-cyclopentyl-8-(phenylamino)-9H-purin-2-yl)amino)phenyl)piperazin-1-yl)methyl)-2-(2,6-dioxopiperidin-3-yl)isoindoline-1,3-dione;
4-bromo-5-(((1-(4-((9-cyclopentyl-8-(phenylamino)-9H-purin-2-yl)amino)phenyl)piperidin-4-yl)(methyl)amino)methyl)-2-(2,6-dioxopiperidin-3-yl)isoindoline-1,3-dione;
5-bromo-6-(((1-(4-((9-cyclopentyl-8-(phenylamino)-9H-purin-2-yl)amino)phenyl)piperidin-4-yl)(methyl)amino)methyl)-2-(2,6-dioxopiperidin-3-yl)isoindoline-1,3-dione;
4-bromo-6-(((1-(4-((9-cyclopentyl-8-(phenylamino)-9H-purin-2-yl)amino)phenyl)piperidin-4-yl)(methyl)amino)methyl)-2-(2,6-dioxopiperidin-3-yl)isoindoline-1,3-dione;
4-bromo-5-((4-(1-(4-((9-cyclopentyl-8-(phenylamino)-9H-purin-2-yl)amino)phenyl)piperidin-4-yl)piperazin-1-yl)methyl)-2-(2,6-dioxopiperidin-3-yl)isoindoline-1,3-dione;
2-(7-(((4-bromo-2-(2,6-dioxopiperidin-3-yl)-1,3-dioxoisoindolin-5-yl)methyl)amino)-1-oxoisoindolin-2-yl)-2-(5-fluoro-2-hydroxyphenyl)-N-(thiazol-2-yl)acetamide;
2-(6-(4-((6-bromo-2-(2,6-dioxopiperidin-3-yl)-1,3-dioxoisoindolin-5-yl)methyl)piperazin-1-yl)-1-oxoisoindolin-2-yl)-2-(5-fluoro-2-hydroxyphenyl)-N-(thiazol-2-yl)acetamide;
2-(6-(4-(((7-bromo-2-(2,6-dioxopiperidin-3-yl)-1,3-dioxoisoindolin-5-yl)methyl)(methyl)amino)piperidin-1-yl)-1-oxoisoindolin-2-yl)-2-(5-fluoro-2-hydroxyphenyl)-N-(thiazol-2-yl)acetamide;
2-(6-(4-(4-((4-bromo-2-(2,6-dioxopiperidin-3-yl)-1,3-dioxoisoindolin-5-yl)methyl)piperazin-1-yl)piperidin-1-yl)-1-oxoisoindolin-2-yl)-2-(5-fluoro-2-hydroxyphenyl)-N-(thiazol-2-yl)acetamide;
2-(6-(6-(4-((6-bromo-2-(2,6-dioxopiperidin-3-yl)-1,3-dioxoisoindolin-5-yl)methyl)piperazin-1-yl)pyridazin-3-yl)-1-oxoisoindolin-2-yl)-2-(5-fluoro-2-hydroxyphenyl)-N-(thiazol-2-yl)acetamide;
4-bromo-5-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)-2-(2,6-dioxopiperidin-3-yl)isoindoline-1,3-dione;
5-bromo-6-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)-2-(2,6-dioxopiperidin-3-yl)isoindoline-1,3-dione;
4-bromo-6-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)-2-(2,6-dioxopiperidin-3-yl)isoindoline-1,3-dione;
4-bromo-5-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)-2-(2,6-dioxopiperidin-3 -yl)isoindoline-1,3 -dione;
5-bromo-6-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)-2-(2,6-dioxopiperidin-3 -yl)isoindoline-1,3 -dione;
4-bromo-6-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)-2-(2,6-dioxopiperidin-3 -yl)isoindoline-1,3 -dione;
4-bromo-5-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)-2-(2,6-dioxopiperidin-3 -yl)isoindoline-1,3 -dione;
5-bromo-6-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)-2-(2,6-dioxopiperidin-3 -yl)isoindoline-1,3 -dione;
4-bromo-6-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)-2-(2,6-dioxopiperidin-3 -yl)isoindoline-1,3 -dione;
4-bromo-2-(2,6-dioxopiperidin-3-yl)-5-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)isoindoline-1,3-dione;
5-bromo-2-(2,6-dioxopiperidin-3-yl)-6-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)isoindoline-1,3-dione;
4-bromo-2-(2,6-dioxopiperidin-3-yl)-6-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)isoindoline-1,3-dione;
1-((4-bromo-2-(2,6-dioxopiperidin-3-yl)-1,3-dioxoisoindolin-5-yl)methyl)-N-(5-(((5-(tert-butyl)oxazol-2-yl)methyl)thio)thiazol-2-yl)piperidine-4-carboxamide;
1'-((6-bromo-2-(2,6-dioxopiperidin-3-yl)-1,3-dioxoisoindolin-5-yl)methyl)-N-(5-(((5-(tert-butyl)oxazol-2-yl)methyl)thio)thiazol-2-yl)-[1,4'-bipiperidine]-4-carboxamide;
4-(1-((7-bromo-2-(2,6-dioxopiperidin-3-yl)-1,3-dioxoisoindolin-5-yl)methyl)piperidin-4-yl)-N-(5-(((5-(tert-butyl)oxazol-2-yl)methyl)thio)thiazol-2-yl)piperazine-1-carboxamide;
N-(1-((4-bromo-2-(2,6-dioxopiperidin-3-yl)-1,3-dioxoisoindolin-5-yl)methyl)piperidin-4-yl)-4-(2,6-dichlorobenzamido)-1H-pyrazole-3-carboxamide;
1-((6-bromo-2-(2,6-dioxopiperidin-3-yl)-1,3-dioxoisoindolin-5-yl)methyl)-N-(5-chloro-4-(5,5-dimethyl-5,6-dihydro-4H-pyrrolo[1,2-b]pyrazol-3-yl)pyridin-2-yl)piperidine-4-carboxamide;
2-(4-((7-bromo-2-(2,6-dioxopiperidin-3-yl)-1,3-dioxoisoindolin-5-yl)methoxy)phenyl)-N-(5-cyclobutyl-1H-pyrazol-3-yl)acetamide;
4-bromo-5-((4-((5-chloro-4-(5-(cyclopropylmethyl)-1-methyl-1H-pyrazol-4-yl)pyrimidin-2-yl)amino)piperidin-1-yl)methyl)-2-(2,6-dioxopiperidin-3-yl)isoindoline-1,3-dione;
4-((6-bromo-2-(2,6-dioxopiperidin-3-yl)-1,3-dioxoisoindolin-5-yl)methyl)-N-(4-(((5-(((S)-1-hydroxybutan-2-yl)amino)-3-isopropylpyrazolo[1,5-a]pyrimidin-7-yl)amino)methyl)phenyl)piperazine-1-carboxamide;
4-(((4-(2-((1-((4-bromo-2-(2,6-dioxopiperidin-3-yl)-1,3-dioxoisoindolin-5-yl)methyl)piperidin-4-yl)amino)-5-chloropyridin-4-yl)thiazol-2-yl)amino)methyl)tetrahydro-2H-pyran-4-carbonitrile;
4-(((2'-((1-((6-bromo-2-(2,6-dioxopiperidin-3-yl)-1,3-dioxoisoindolin-5-yl)methyl)piperidin-4-yl)amino)-5'-chloro-[2,4'-bipyridin]-6-yl)amino)methyl)tetrahydro-2H-pyran-4-carbonitrile;
3-((4-(2-(((7-bromo-2-(2,6-dioxopiperidin-3-yl)-1,3-dioxoisoindolin-5-yl)methyl)(methyl)amino)-4-methylthiazol-5-yl)-5-fluoropyrimidin-2-yl)amino)benzenesulfonamide;
2-((6-(4-((4-bromo-2-(2,6-dioxopiperidin-3-yl)-1,3-dioxoisoindolin-5-yl)methyl)piperazin-1-yl)-2-methylpyrimidin-4-yl)amino)-N-(2-chloro-6-methylphenyl)thiazole-5-carboxamide;
2-((6-(4-(((6-bromo-2-(2,6-dioxopiperidin-3-yl)-1,3-dioxoisoindolin-5-yl)methyl)(methyl)amino)piperidin-1-yl)-2-methylpyrimidin-4-yl)amino)-N-(2-chloro-6-methylphenyl)thiazole-5-carboxamide;
2-((6-(4-(4-((7-bromo-2-(2,6-dioxopiperidin-3-yl)-1,3-dioxoisoindolin-5-yl)methyl)piperazin-1-yl)piperidin-1-yl)-2-methylpyrimidin-4-yl)amino)-N-(2-chloro-6-methylphenyl)thiazole-5-carboxamide;
4-((4-((4-bromo-2-(2,6-dioxopiperidin-3-yl)-1,3-dioxoisoindolin-5-yl)methyl)piperazin-1-yl)methyl)-N-(4-methyl-3-((4-(pyridin-3-yl)pyrimidin-2-yl)amino)phenyl)benzamide;
4-(4-((6-bromo-2-(2,6-dioxopiperidin-3-yl)-1,3-dioxoisoindolin-5-yl)methyl)piperazin-1-yl)-N-(4-methyl-3-((4-(pyridin-3-yl)pyrimidin-2-yl)amino)phenyl)benzamide;
4-(4-(((7-bromo-2-(2,6-dioxopiperidin-3-yl)-1,3-dioxoisoindolin-5-yl)methyl)(methyl)amino)piperidin-1-yl)-N-(4-methyl-3-((4-(pyridin-3-yl)pyrimidin-2-yl)amino)phenyl)benzamide;
4-(4-(4-((4-bromo-2-(2,6-dioxopiperidin-3-yl)-1,3-dioxoisoindolin-5-yl)methyl)piperazin-1-yl)piperidin-1-yl)-N-(4-methyl-3-((4-(pyridin-3-yl)pyrimidin-2-yl)amino)phenyl)benzamide;
N-(4-((4-((6-bromo-2-(2,6-dioxopiperidin-3-yl)-1,3-dioxoisoindolin-5-yl)methyl)piperazin-1-yl)methyl)-3-(trifluoromethyl)phenyl)-3-(imidazo[1,2-b]pyridazin-3-ylethynyl)-4-methylbenzamide;
N-(4-(4-((7-bromo-2-(2,6-dioxopiperidin-3-yl)-1,3-dioxoisoindolin-5-yl)methyl)piperazin-1-yl)-3-(trifluoromethyl)phenyl)-3-(imidazo[1,2-b]pyridazin-3-ylethynyl)-4-methylbenzamide;
N-(4-(4-(1-((4-bromo-2-(2,6-dioxopiperidin-3-yl)-1,3-dioxoisoindolin-5-yl)methyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(((4-bromo-2-(2,6-dioxopiperidin-3-yl)-1,3-dioxoisoindolin-5-yl)methyl)(methyl)amino)piperidin-1-yl)-3-(trifluoromethyl)phenyl)-3-(imidazo[1,2-b]pyridazin-3 - ylethynyl)-4-methylbenzamide;
2-(4-((3S)-1-((4-bromo-2-(2,6-dioxopiperidin-3-yl)-1,3-dioxoisoindolin-5-yl)methyl)piperidin-3-yl)phenyl)-2H-indazole-7-carboxamide;
2-(4-((3S)-1-((6-bromo-2-(2,6-dioxopiperidin-3-yl)-1,3-dioxoisoindolin-5-yl)methyl)piperidin-3-yl)phenyl)-2H-indazole-7-carboxamide;
2-(4-(1-((6-bromo-2-(2,6-dioxopiperidin-3-yl)-1,3-dioxoisoindolin-5-yl)methyl)piperidin-4-yl)phenyl)-2H-indazole-7-carboxamide;
2-(4-(4-((7-bromo-2-(2,6-dioxopiperidin-3-yl)-1,3-dioxoisoindolin-5-yl)methyl)piperazin-1-yl)phenyl)-2H-indazole-7-carboxamide;
4-bromo-2-(2,6-dioxopiperidin-3-yl)-5-((4-(2-fluoro-5-((4-oxo-3,4-dihydrophthalazin-1-yl)methyl)benzoyl)piperazin-1-yl)methyl)isoindoline-1,3-dione;
5-bromo-2-(2,6-dioxopiperidin-3-yl)-6-(((4-(8-fluoro-1-oxo-2,3,4,6-tetrahydro-1H-azepino[5,4,3-cd]indol-5-yl)benzyl)(methyl)amino)methyl)isoindoline-1,3-dione;
2-((5-(4-((4-bromo-2-(2,6-dioxopiperidin-3-yl)-1,3-dioxoisoindolin-5-yl)methyl)piperazin-1-yl)pyridin-2-yl)amino)-7-cyclopentyl-N,N-dimethyl-7H-pyrrolo[2,3-d]pyrimidine-6-carboxamide;
2-((5-(4-(((6-bromo-2-(2,6-dioxopiperidin-3-yl)-1,3-dioxoisoindolin-5-yl)methyl)(methyl)amino)piperidin-1-yl)pyridin-2-yl)amino)-7-cyclopentyl-N,N-dimethyl-7H-pyrrolo[2,3 -d]pyrimidine-6-carboxamide;
2-((5-(4-(4-((7-bromo-2-(2,6-dioxopiperidin-3-yl)-1,3-dioxoisoindolin-5-yl)methyl)piperazin-1-yl)piperidin-1-yl)pyridin-2-yl)amino)-7-cyclopentyl-N,N-dimethyl-7H-pyrrolo[2,3-d]pyrimidine-6-carboxamide;
2-(2,6-dioxopiperidin-3-yl)-4-fluoro-5-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)isoindoline-1,3-dione;
5-bromo-6-((4-(difluoro(6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-2-(2,6-dioxopiperidin-3-yl)isoindoline-1,3-dione;
4-bromo-2-(2,6-dioxopiperidin-3-yl)-6-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)isoindoline-1,3-dione;
4-bromo-2-(2,6-dioxopiperidin-3-yl)-5-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)isoindoline-1,3-dione;
5-bromo-2-(2,6-dioxopiperidin-3-yl)-6-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)isoindoline-1,3-dione;
5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-4-bromo-2-(2,6-dioxopiperidin-3-yl)isoindoline-1,3-dione;
5-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)-6-bromo-2-(2,6-dioxopiperidin-3-yl)isoindoline-1,3-dione;
6-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)-4-bromo-2-(2,6-dioxopiperidin-3-yl)isoindoline-1,3-dione;
3-((4-(4-(4-((4-bromo-2-(2,6-dioxopiperidin-3-yl)-1,3-dioxoisoindolin-5-yl)methyl)piperazin-1-yl)piperidin-1-yl)-3-methoxyphenyl)amino)-6-ethyl-5-((tetrahydro-2H-pyran-4-yl)amino)pyrazine-2-carboxamide;
4-bromo-5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-2-(2,6-dioxopiperidin-3-yl)isoindoline-1,3-dione;
5-bromo-6-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-2-(2,6-dioxopiperidin-3-yl)isoindoline-1,3-dione;
4-bromo-6-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-2-(2,6-dioxopiperidin-3-yl)isoindoline-1,3-dione;
4-bromo-5-((4-(4-((5-chloro-4-((2-(dimethylphosphoryl)phenyl)amino)pyrimidin-2-yl)amino)-3-methoxyphenyl)piperazin-1-yl)methyl)-2-(2,6-dioxopiperidin-3-yl)isoindoline-1,3-dione;
5-bromo-6-((4-(4-(4-((5-chloro-4-((2-(dimethylphosphoryl)phenyl)amino)pyrimidin-2-yl)amino)-3-methoxyphenyl)piperazin-1-yl)piperidin-1-yl)methyl)-2-(2,6-dioxopiperidin-3-yl)isoindoline-1,3-dione;
8-(4-(4-((4-bromo-2-(2,6-dioxopiperidin-3-yl)-1,3-dioxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-((6-bromo-2-(2,6-dioxopiperidin-3-yl)-1,3-dioxoisoindolin-5-yl)methyl)piperazin-1-yl)-9-ethyl-6,6-dimethyl-11-oxo-6,11-dihydro-5H-benzo[b]carbazole-3-carbonitrile;
8-(4-(((7-bromo-2-(2,6-dioxopiperidin-3-yl)-1,3-dioxoisoindolin-5-yl)methyl)(methyl)amino)piperidin-1-yl)-9-ethyl-6,6-dimethyl-11-oxo-6,11-dihydro-5H-benzo[b]carbazole-3-carbonitrile;
4-bromo-5-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)-2-(2,6-dioxopiperidin-3-yl)isoindoline-1,3-dione;
4-bromo-5-((4-(4-(5,7-dimethoxy-4-oxo-3,4-dihydroquinazolin-2-yl)phenyl)piperazin-1-yl)methyl)-2-(2,6-dioxopiperidin-3-yl)isoindoline-1,3-dione;
5-bromo-6-(((1-(4-(5,7-dimethoxy-4-oxo-3,4-dihydroquinazolin-2-yl)phenyl)piperidin-4-yl)(methyl)amino)methyl)-2-(2,6-dioxopiperidin-3-yl)isoindoline-1,3-dione;
4-bromo-6-((4-(1-(4-(5,7-dimethoxy-4-oxo-3,4-dihydroquinazolin-2-yl)phenyl)piperidin-4-yl)pip erazin-1-yl)methyl)-2-(2, 6-dioxopip eri din-3 -yl)isoindoline-1, 3 -dione;
2-((2-((4-(4-(4-((4-bromo-2-(2,6-dioxopiperidin-3-yl)-1,3-dioxoisoindolin-5-yl)methyl)piperazin-1-yl)piperidin-1-yl)-2-methoxyphenyl)amino)-5-(trifluoromethyl)pyridin-4-yl)amino)-N-methylbenzamide;
2-((2-((4-(4-(((6-bromo-2-(2,6-dioxopiperidin-3-yl)-1,3-dioxoisoindolin-5-yl)methyl)(methyl)amino)piperidin-1-yl)-2-methoxyphenyl)amino)-5-(trifluoromethyl)pyridin-4-yl)amino)-N-methylbenzamide;
2-((2-((4-(4-((7-bromo-2-(2,6-dioxopiperidin-3-yl)-1,3-dioxoisoindolin-5-yl)methyl)piperazin-1-yl)-2-methoxyphenyl)amino)-5-(trifluoromethyl)pyridin-4-yl)amino)-N-methylbenzamide;
N-(3-(((2-((4-(4-((4-bromo-2-(2,6-dioxopiperidin-3-yl)-1,3-dioxoisoindolin-5-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(((6-bromo-2-(2,6-dioxopiperidin-3-yl)-1,3-dioxoisoindolin-5-yl)methyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-((7-bromo-2-(2,6-dioxopiperidin-3-yl)-1,3-dioxoisoindolin-5-yl)methyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((4-bromo-2-(2,6-dioxopiperidin-3-yl)-1,3-dioxoisoindolin-5-yl)methyl)piperazin-1-yl)phenyl)amino)-5-(trifluoromethyl)pyrimidin-4-yl)amino)methyl)phenyl)-N-methylmethanesulfonamide;
N-(3-(((2-((4-(4-(((6-bromo-2-(2,6-dioxopiperidin-3-yl)-1,3-dioxoisoindolin-5-yl)methyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-((7-bromo-2-(2,6-dioxopiperidin-3-yl)-1,3-dioxoisoindolin-5-yl)methyl)piperazin-1-yl)piperidin-1-yl)phenyl)amino)-5-(trifluoromethyl)pyrimidin-4-yl)amino)methyl)phenyl)-N-methylmethanesulfonamide;
N-(3-(((2-((4-(4-((4-bromo-2-(2,6-dioxopiperidin-3-yl)-1,3-dioxoisoindolin-5-yl)methyl)piperazin-1-yl)phenyl)amino)-5-(trifluoromethyl)pyrimidin-4-yl)amino)methyl)pyridin-2-yl)-N-methylmethanesulfonamide;
N-(3-(((2-((4-(4-(((6-bromo-2-(2,6-dioxopiperidin-3-yl)-1,3-dioxoisoindolin-5-yl)methyl)(methyl)amino)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-((7-bromo-2-(2,6-dioxopiperidin-3-yl)-1,3-dioxoisoindolin-5-yl)methyl)piperazin-1-yl)piperidin-1-yl)phenyl)amino)-5-(trifluoromethyl)pyrimidin-4-yl)amino)methyl)pyridin-2-yl)-N-methylmethanesulfonamide;
N-(1-(1-((4-bromo-2-(2,6-dioxopiperidin-3-yl)-1,3-dioxoisoindolin-5-yl)methyl)piperidin-4-yl)-3-(difluoromethyl)-1H-pyrazol-4-yl)-2-(2-((cyclopropylmethyl)amino)pyridin-4-yl)oxazole-4-carboxamide;
N-(1-(1-((6-bromo-2-(2,6-dioxopiperidin-3-yl)-1,3-dioxoisoindolin-5-yl)methyl)piperidin-4-yl)-3-(difluoromethyl)-1H-pyrazol-4-yl)-5-morpholinopyrazolo[1,5-a]pyrimidine-3-carboxamide;
N-(2-(4-((7-bromo-2-(2,6-dioxopiperidin-3-yl)-1,3-dioxoisoindolin-5-yl)methyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide;
4-((1-((4-bromo-2-(2,6-dioxopiperidin-3-yl)-1,3-dioxoisoindolin-5-yl)methyl)piperidin-4-yl)ethynyl)-1-(((2S,3S,4S)-3-ethyl-4-fluoro-5-oxopyrrolidin-2-yl)methoxy)-7-methoxyisoquinoline-6-carboxamide;
6-(6-(4-((4-bromo-2-(2,6-dioxopiperidin-3-yl)-1,3-dioxoisoindolin-5-yl)methyl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(((6-bromo-2-(2,6-dioxopiperidin-3-yl)-1,3-dioxoisoindolin-5-yl)methyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-((7-bromo-2-(2,6-dioxopiperidin-3-yl)-1,3-dioxoisoindolin-5-yl)methyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
4'-((4-((4-bromo-2-(2,6-dioxopiperidin-3-yl)-1,3-dioxoisoindolin-5-yl)methyl)piperazin-1-yl)methyl)-N-((4,6-dimethyl-2-oxo-1,2-dihydropyridin-3-yl)methyl)-5-(ethyl(tetrahydro-2H-pyran-4-yl)amino)-4-methyl-[1,1'-biphenyl]-3-carboxamide;
4'-(4-((6-bromo-2-(2,6-dioxopiperidin-3-yl)-1,3-dioxoisoindolin-5-yl)methyl)piperazin-1-yl)-N-((4,6-dimethyl-2-oxo-1,2-dihydropyridin-3-yl)methyl)-5-(ethyl(tetrahydro-2H-pyran-4-yl)amino)-4-methyl-[1,1'-biphenyl]-3 -carboxamide;
4-(4-((4-bromo-2-(2,6-dioxopiperidin-3-yl)-1,3-dioxoisoindolin-5-yl)methyl)piperazin-1-yl)-N-(5-(3,5-difluorobenzyl)-1H-indazol-3-yl)-2-((tetrahydro-2H-pyran-4-yl)amino)benzamide;
4-(4-(((6-bromo-2-(2,6-dioxopiperidin-3-yl)-1,3-dioxoisoindolin-5-yl)methyl)(methyl)amino)piperidin-1-yl)-N-(5-(3,5-difluorobenzyl)-1H-indazol-3-yl)-2-((tetrahydro-2H-pyran-4-yl)amino)benzamide;
4-(4-(1-((7-bromo-2-(2,6-dioxopiperidin-3-yl)-1,3-dioxoisoindolin-5-yl)methyl)piperidin-4-yl)piperazin-1-yl)-N-(5-(3,5-difluorobenzyl)-1H-indazol-3-yl)-2-((tetrahydro-2H-pyran-4-yl)amino)benzamide;
4-bromo-2-(2,6-dioxopiperidin-3-yl)-5-((4-(6-(6-((R)-2-(3-fluorophenyl)pyrrolidin-1-yl)imidazo[1,2-b]pyridazin-3-yl)pyridin-2-yl)piperazin-1-yl)methyl)isoindoline-1,3-dione;
5-bromo-2-(2,6-dioxopiperidin-3-yl)-6-(((1-(6-(6-((R)-2-(3-fluorophenyl)pyrrolidin-1-yl)imidazo[1,2-b]pyridazin-3-yl)pyridin-2-yl)piperidin-4-yl)(methyl)amino)methyl)isoindoline-1,3-dione;
4-bromo-2-(2,6-dioxopiperidin-3-yl)-6-((4-(4-(6-(6-((R)-2-(3-fluorophenyl)pyrrolidin-1-yl)imidazo[1,2-b]pyridazin-3-yl)pyridin-2-yl)piperazin-1-yl)piperidin-1-yl)methyl)isoindoline-1,3-dione;
4-bromo-5-((4-(6-(5-((R)-2-(2,5-difluorophenyl)pyrrolidin-1-yl)pyrazolo[1,5-a]pyrimidin-3-yl)pyridin-2-yl)piperazin-1-yl)methyl)-2-(2,6-dioxopiperidin-3-yl)isoindoline-1,3-dione;
4-bromo-5-((4-(6-(5-((R)-2-(2,5-difluorophenyl)pyrrolidin-1-yl)pyrazolo[1,5-a]pyrimidin-3-yl)pyridin-2-yl)piperazin-1-yl)methyl)-2-(2,6-dioxopiperidin-3-yl)isoindoline-1,3-dione;
4-bromo-6-((4-(4-(6-(5-((R)-2-(2,5-difluorophenyl)pyrrolidin-1-yl)pyrazolo[1,5-a]pyrimidin-3-yl)pyridin-2-yl)piperazin-1-yl)piperidin-1-yl)methyl)-2-(2,6-dioxopiperidin-3-yl)isoindoline-1,3-dione;
N-((4-(((2R)-4-(4-((4-bromo-2-(2,6-dioxopiperidin-3-yl)-1,3-dioxoisoindolin-5-yl)methyl)piperazin-1-yl-2,2,3,3,5,5,6,6-*d*₈)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl]-2-yl)methyl)piperazin-1-yl)benzamide;
N-((4-(((2R)-4-(4-((6-bromo-2-(2,6-dioxopiperidin-3-yl)-1,3-dioxoisoindolin-5-yl)methyl)-3,5-dimethylpiperazin-1-yl)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl]-2-yl)methyl)piperazin-1-yl)benzamide;
N-((4-(((2R)-4-(4-((7-bromo-2-(2,6-dioxopiperidin-3-yl)-1,3-dioxoisoindolin-5-yl)methyl)-2,6-dimethylpiperazin-1-yl)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl]-2-yl)methyl)piperazin-1-yl)benzamide;
N-((4-(((2R)-4-(6-((4-bromo-2-(2,6-dioxopiperidin-3-yl)-1,3-dioxoisoindolin-5-yl)methyl)-3,6-diazabicyclo[3.1.1]heptan-3-yl)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl] -2-yl)methyl)piperazin-1-yl)benzamide;
N-((4-(((2R)-4-(3-((6-bromo-2-(2,6-dioxopiperidin-3-yl)-1,3-dioxoisoindolin-5-yl)methyl)-3,6-diazabicyclo[3.1.1]heptan-6-yl)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl]-2-yl)methyl)piperazin-1-yl)benzamide;
N-((4-(((2R)-4-(8-((7-bromo-2-(2,6-dioxopiperidin-3-yl)-1,3-dioxoisoindolin-5-yl)methyl)-3,8-diazabicyclo[3.2.1]octan-3-yl)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl]-2-yl)methyl)piperazin-1-yl)benzamide;
N-((4-(((2R)-4-(3-((4-bromo-2-(2,6-dioxopiperidin-3-yl)-1,3-dioxoisoindolin-5-yl)methyl)-3,8-diazabicyclo[3.2.1]octan-8-yl)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl]-2-yl)methyl)piperazin-1-yl)benzamide;
N-((4-(((2R)-4-((1R,4R)-5-((6-bromo-2-(2,6-dioxopiperidin-3-yl)-1,3-dioxoisoindolin-5-yl)methyl)-2,5-diazabicyclo[2.2.1]heptan-2-yl)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl]-2-yl)methyl)piperazin-1-yl)benzamide;
N-((4-(((2R)-4-((1S,4S)-5-((7-bromo-2-(2,6-dioxopiperidin-3-yl)-1,3-dioxoisoindolin-5-yl)methyl)-2,5-diazabicyclo[2.2.1]heptan-2-yl)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl]-2-yl)methyl)piperazin-1-yl)benzamide;
N-((4-(((2R)-4-(5-((4-bromo-2-(2,6-dioxopiperidin-3-yl)-1,3-dioxoisoindolin-5-yl)methyl)-2,5-diazabicyclo[2.2.2]octan-2-yl)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl]-2-yl)methyl)piperazin-1-yl)benzamide;
N-((4-(((2R)-4-(4-((4-bromo-2-(2,6-dioxopiperidin-3-yl)-1,3-dioxoisoindolin-5-yl)methyl)piperazin-1-yl)-1-(phenylthio)butan-2-yl)amino)-3 - ((trifluoromethyl)sulfonyl)phenyl)sulfonyl)-4-(4-((4'-chloro-4,4-dimethyl-3,4,5,6-tetrahydro-1,1'-biphenyl]-2-yl)methyl)piperazin-1-yl)benzamide;
N-((4-(((2R)-4-(4-((6-bromo-2-(2,6-dioxopiperidin-3-yl)-1,3-dioxoisoindolin-5-yl)methyl)piperazin-1-yl)-1-(phenylthio)butan-2-yl)amino)-3 - ((trifluoromethyl)sulfonyl)phenyl)sulfonyl)-4-(4-((4'-chloro-4,4-dimethyl-3,4,5,6-tetrahydro-1,1'-biphenyl]-2-yl)methyl)piperazin-1-yl)benzamide;
N-((4-(((2R)-4-(4-((7-bromo-2-(2,6-dioxopiperidin-3-yl)-1,3-dioxoisoindolin-5-yl)methyl)piperazin-1-yl)-1-(phenylthio)butan-2-yl)amino)-3 - ((trifluoromethyl)sulfonyl)phenyl)sulfonyl)-4-(4-((4'-chloro-4,4-dimethyl-3,4,5,6-tetrahydro-1,1'-biphenyl]-2-yl)methyl)piperazin-1-yl)benzamide;
N-(2-(4-((6-bromo-2-(2,6-dioxopiperidin-3-yl)-1-oxoisoindolin-5-yl)methyl)piperazin-1-yl)ethyl)-4,9-dioxo-4,9-dihydronaphtho[2,3-b]furan-2-carboxamide;
3-(5-((4-(1-(4,9-dioxo-4,9-dihydronaphtho[2,3-b]furan-2-yl)ethyl)piperazin-1-yl)methyl)-1-oxoisoindolin-2-yl)piperidine-2,6-dione;
N-((4-bromo-2-(2,6-dioxopiperidin-3-yl)-1-oxoisoindolin-5-yl)methyl)-4,9-dioxo-4,9-dihydronaphtho[2,3-b]furan-2-carboxamide;
N-((6-bromo-2-(2,6-dioxopiperidin-3-yl)-1-oxoisoindolin-5-yl)methyl)-4,9-dioxo-4,9-dihydronaphtho[2,3-b]furan-2-carboxamide;
N-((7-bromo-2-(2,6-dioxopiperidin-3-yl)-1-oxoisoindolin-5-yl)methyl)-4,9-dioxo-4,9-dihydronaphtho[2,3-b]furan-2-carboxamide;
((2-(((5S,8S,10aR)-8-(((2S)-5-amino-1-(3-((6-bromo-2-(2,6-dioxopiperidin-3-yl)-1-oxoisoindolin-5-yl)methyl)-2-chlorophenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(3-(4-((4-bromo-2-(2,6-dioxopiperidin-3-yl)-1-oxoisoindolin-5-yl)methyl)piperazin-1-yl)-2-chlorophenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(3-(4-((7-bromo-2-(2,6-dioxopiperidin-3-yl)-1-oxoisoindolin-5-yl)methyl)piperazin-1-yl)-2-chlorophenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((1-((4-bromo-2-(2,6-dioxopiperidin-3-yl)-1-oxoisoindolin-5-yl)methyl)piperidin-4-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((6-bromo-2-(2,6-dioxopiperidin-3-yl)-1-oxoisoindolin-5-yl)methyl)-6-oxodecahydropyrrolo[1,2-a][1,5]diazocin-5-yl)carbamoyl)-1H-indol-5-yl)difluoromethyl)phosphonic acid;
3-(7-bromo-5-(((3-((3aR,4S,9bR)-4-(hydroxymethyl)-1-(pyridin-4-ylmethyl)-2,3,3a,4,5,9b-hexahydro-1H-pyrrolo[3,2-c]quinolin-8-yl)phenyl)amino)methyl)-1-oxoisoindolin-2-yl)piperidine-2,6-dione;
6-(1-((4-bromo-2-(2,6-dioxopiperidin-3-yl)-1-oxoisoindolin-5-yl)methyl)piperidin-4-yl)-2-(4-phenoxyphenyl)nicotinamide;
6-(1-((6-bromo-2-(2,6-dioxopiperidin-3-yl)-1-oxoisoindolin-5-yl)methyl)piperidin-4-yl)-2-(4-phenoxyphenyl)nicotinamide;
6-(1-((7-bromo-2-(2,6-dioxopiperidin-3-yl)-1-oxoisoindolin-5-yl)methyl)piperidin-4-yl)-2-(4-phenoxyphenyl)nicotinamide;
6-(4-((4-bromo-2-(2,6-dioxopiperidin-3-yl)-1-oxoisoindolin-5-yl)methyl)piperazin-1-yl)-2-(4-phenoxyphenyl)nicotinamide;
6-(4-(((6-bromo-2-(2,6-dioxopiperidin-3-yl)-1-oxoisoindolin-5-yl)methyl)(methyl)amino)piperidin-1-yl)-2-(4-phenoxyphenyl)nicotinamide;
6-(4-(4-((7-bromo-2-(2,6-dioxopiperidin-3-yl)-1-oxoisoindolin-5-yl)methyl)piperazin-1-yl)piperidin-1-yl)-2-(4-phenoxyphenyl)nicotinamide;
N-(4-(5-(4-(4-((4-bromo-2-(2,6-dioxopiperidin-3-yl)-1-oxoisoindolin-5-yl)methyl)piperazin-1-yl)phenyl)-1H-indazol-3-yl)-2-methylbenzyl)-3-(tert-butyl)-1,2,4-oxadiazole-5-carboxamide;
N-(4-(5-(4-(4-(((6-bromo-2-(2,6-dioxopiperidin-3-yl)-1-oxoisoindolin-5-yl)methyl) (methyl) amino)pip eridin-1-yl)phenyl)-1 H-indazol-3 -yl)-2-methylb enzyl)-3 -(tert-butyl)-1,2,4-oxadiazole-5-carboxamide;
N-(4-(5-(4-(4-(4-((7-bromo-2-(2,6-dioxopiperidin-3-yl)-1-oxoisoindolin-5-yl)methyl)piperazin-1-yl)piperidin-1-yl)phenyl)-1H-indazol-3-yl)-2-methylbenzyl)-3-(tert-butyl)-1,2,4-oxadiazole-5-carboxamide;
3-(6-bromo-5-((4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperazin-1-yl)methyl)-1-oxoisoindolin-2-yl)piperidine-2,6-dione;
3-(4-bromo-5-((3-(4-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin-5-yl)amino)pyridin-3-yl)piperazin-1 -yl)azetidin-1 -yl)methyl)-1 -oxoisoindolin-2-yl)piperidine-2,6-dione;
3-(7-bromo-5-(((1-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperidin-4-yl)(methyl)amino)methyl)-1-oxoisoindolin-2-yl)piperidine-2,6-dione;
3-(4-bromo-5-((4-(1-(6-((2'-(7,7-dimethyl-1-oxo-1,3,4,6,7,8-hexahydro-2H-cyclopenta[4,5]pyrrolo[1,2-a]pyrazin-2-yl)-3'-(hydroxymethyl)-1-methyl-6-oxo-1,6-dihydro-[3,4'-bipyridin]-5-yl)amino)pyridin-3-yl)piperidin-4-yl)piperazin-1-yl)methyl)-1-oxoisoindolin-2-yl)piperidine-2,6-dione;
3 -(5 -((4-(4-amino-3 -(4-phenoxyphenyl)-1H-pyrazolo [3,4-d]pyrimidin-1 -yl)piperidin-1 - yl)methyl)-4-bromo-1-oxoisoindolin-2-yl)piperidine-2,6-dione;
3 -(5 -((4-(4-amino-3 -(4-phenoxyphenyl)-1H-pyrazolo [3,4-d]pyrimidin-1 -yl)piperidin-1 - yl)methyl)-6-bromo-1-oxoisoindolin-2-yl)piperidine-2,6-dione;
3 -(5 -((4-(4-amino-3 -(4-phenoxyphenyl)-1H-pyrazolo [3,4-d]pyrimidin-1 -yl)piperidin-1 - yl)methyl)-7-bromo-1-oxoisoindolin-2-yl)piperidine-2,6-dione;
3-(5-((4-(4-(1,2-bis(4-hydroxyphenyl)but-1-en-1-yl)phenyl)piperazin-1-yl)methyl)-4-bromo-1-oxoisoindolin-2-yl)piperidine-2,6-dione;
3-(5-((4-(4-(1,2-bis(4-hydroxyphenyl)but-1-en-1-yl)phenyl)piperazin-1-yl)methyl)-6-bromo-1-oxoisoindolin-2-yl)piperidine-2,6-dione;
3-(5-((4-(4-(1,2-bis(4-hydroxyphenyl)but-1-en-1-yl)phenyl)piperazin-1-yl)methyl)-7-bromo-1-oxoisoindolin-2-yl)piperidine-2,6-dione;
3-(4-bromo-5-((4-(4-((1R,2S)-6-hydroxy-2-phenyl-1,2,3,4-tetrahydronaphthalen-1-yl)phenyl)piperazin-1-yl)methyl)-1-oxoisoindolin-2-yl)piperidine-2,6-dione;
3-(6-bromo-5-((4-(4-((1R,2S)-6-hydroxy-2-phenyl-1,2,3,4-tetrahydronaphthalen-1-yl)phenyl)piperazin-1-yl)methyl)-1-oxoisoindolin-2-yl)piperidine-2,6-dione;
3-(7-bromo-5-((4-(4-((1R,2S)-6-hydroxy-2-phenyl-1,2,3,4-tetrahydronaphthalen-1-yl)phenyl)piperazin-1-yl)methyl)-1-oxoisoindolin-2-yl)piperidine-2,6-dione;
3 -(4-bromo-5 -((4-(4-((3 S,4R)-7-hydroxy-3 -phenylchroman-4-yl)phenyl)piperazin-1-yl)methyl)-1-oxoisoindolin-2-yl)piperidine-2,6-dione;
3-(6-bromo-5-((4-(4-((3S,4R)-7-hydroxy-3-phenylchroman-4-yl)phenyl)piperazin-1-yl)methyl)-1-oxoisoindolin-2-yl)piperidine-2,6-dione;
3-(7-bromo-5-((4-(4-((3S,4R)-7-hydroxy-3-phenylchroman-4-yl)phenyl)piperazin-1-yl)methyl)-1-oxoisoindolin-2-yl)piperidine-2,6-dione;
3-(4-bromo-5-(((1-(4-((3S,4R)-7-hydroxy-3-phenylchroman-4-yl)phenyl)piperidin-4-yl)(methyl)amino)methyl)-1-oxoisoindolin-2-yl)piperidine-2,6-dione;
3-(6-bromo-5-(((1-(4-((3S,4R)-7-hydroxy-3-phenylchroman-4-yl)phenyl)piperidin-4-yl)(methyl)amino)methyl)-1-oxoisoindolin-2-yl)piperidine-2,6-dione;
3-(7-bromo-5-(((1-(4-((3S,4R)-7-hydroxy-3-phenylchroman-4-yl)phenyl)piperidin-4-yl)(methyl)amino)methyl)-1-oxoisoindolin-2-yl)piperidine-2,6-dione;
3-(4-bromo-5-((4-(1-(4-((3 S,4R)-7-hydroxy-3-phenylchroman-4-yl)phenyl)piperidin-4-yl)piperazin-1-yl)methyl)-1-oxoisoindolin-2-yl)piperidine-2,6-dione;
3-(4-bromo-5-(((1-(4-((1R,2S)-6-hydroxy-2-phenyl-1,2,3,4-tetrahydronaphthalen-1-yl)phenyl)piperidin-4-yl)(methyl)amino)methyl)-1-oxoisoindolin-2-yl)piperidine-2,6-dione;
3-(6-bromo-5-(((1-(4-((1R,2S)-6-hydroxy-2-phenyl-1,2,3,4-tetrahydronaphthalen-1-yl)phenyl)piperidin-4-yl)(methyl)amino)methyl)-1-oxoisoindolin-2-yl)piperidine-2,6-dione;
3-(7-bromo-5-(((1-(4-((1R,2S)-6-hydroxy-2-phenyl-1,2,3,43-(4-bromo-5-(((1-(4-((1R,2S)-6-hydroxy-2-phenyl-1,2,3,4-tetrahydronaphthalen-1-yl)phenyl)piperidin-4-yl)(methyl)amino)methyl)-1-oxoisoindolin-2-yl))piperidine-2,6-dione-tetrahydronaphthalen-1-yl)phenyl)piperidin-4-yl)(methyl)amino)methyl)-1-oxoisoindolin-2-yl))piperidine-2,6-dione;
3-(5-(((2-(4-(1,2-bis(4-hydroxyphenyl)but-1-en-1-yl)phenoxy)ethyl)(methyl)amino)methyl)-4-bromo-1-oxoisoindolin-2-yl)piperidine-2,6-dione;
3-(5-(((2-(4-(1,2-bis(4-hydroxyphenyl)but-1-en-1-yl)phenoxy)ethyl)(methyl)amino)methyl)-6-bromo-1-oxoisoindolin-2-yl)piperidine-2,6-dione;
3-(5-(((2-(4-(1,2-bis(4-hydroxyphenyl)but-1-en-1-yl)phenoxy)ethyl)(methyl)amino)methyl)-7-bromo-1-oxoisoindolin-2-yl)piperidine-2,6-dione;
3-(4-bromo-5-(((1-(3-(2,3-dichlorophenyl)-1H-pyrazolo[3,4-b]pyrazin-6-yl)-4-methylpiperidin-4-yl)amino)methyl)-1-oxoisoindolin-2-yl)piperidine-2,6-dione;
3 -(5 -((4-(3-(6-(4-amino-4-methylpiperidin-1-yl)-1H-pyrazolo[3,4-b]pyrazin-3 -yl)-2-chlorophenyl)piperazin-1-yl)methyl)-6-bromo-1-oxoisoindolin-2-yl)piperidine-2,6-dione;
3 -(5 -((4-(3 -((3 -amino-5 -(4-amino-4-methylpiperidin-1-yl)pyrazin-2-yl)thio)-2-chlorophenyl)piperazin-1-yl)methyl)-7-bromo-1-oxoisoindolin-2-yl)piperidine-2,6-dione;
3-(5-((4-(3-(3-amino-5-(4-amino-4-methylpiperidin-1-yl)pyrazin-2-yl)-2-chlorophenyl)piperazin-1-yl)methyl)-4-bromo-1-oxoisoindolin-2-yl)piperidine-2,6-dione;
3-(5-((((3S,4S)-8-(6-amino-5-((2-amino-3-chloropyridin-4-yl)thio)pyrazin-2-yl)-3-methyl-2-oxa-8-azaspiro[4.5]decan-4-yl)amino)methyl)-6-bromo-1-oxoisoindolin-2-yl)piperidine-2,6-dione;
3-(5-((4-(4-((3-amino-5-((3S,4S)-4-amino-3-methyl-2-oxa-8-azaspiro[4.5]decan-8-yl)pyrazin-2-yl)thio)pyridin-2-yl)piperazin-1-yl)methyl)-7-bromo-1-oxoisoindolin-2-yl)piperidine-2,6-dione;
3-(5-(((1-(6-amino-5-(2,3-dichlorophenyl)pyrazin-2-yl)-4-methylpiperidin-4-yl)amino)methyl)-4-bromo-1-oxoisoindolin-2-yl)piperidine-2,6-dione;
3-(4-bromo-5-((2,3-difluoro-6-(2-morpholinothiazol-4-yl)phenoxy)methyl)-1-oxoisoindolin-2-yl)piperidine-2,6-dione;
6-(4-(4-((6-bromo-2-(2,6-dioxopiperidin-3-yl)-1-oxoisoindolin-5-yl)methyl)piperazin-1-yl)piperidin-1-yl)-N-((1r,4r)-4-(3-chloro-4-cyanophenoxy)cyclohexyl)pyridazine-3-carboxamide;
6-(4-((7-bromo-2-(2,6-dioxopiperidin-3-yl)-1-oxoisoindolin-5-yl)methyl)piperazin-1-yl)-N-((1r,3r)-3-(3-chloro-4-cyanophenoxy)-2,2,4,4-tetramethylcyclobutyl)pyridazine-3-carboxamide;
6-(4-((4-bromo-2-(2,6-dioxopiperidin-3-yl)-1-oxoisoindolin-5-yl)methyl)piperazin-1-yl)-N-((1r,3r)-3-(3-chloro-4-cyanophenoxy)-2,2,4,4-tetramethylcyclobutyl)nicotinamide;
6-(8-((6-bromo-2-(2,6-dioxopiperidin-3-yl)-1-oxoisoindolin-5-yl)methyl)-3,8-diazabicyclo[3.2.1]octan-3-yl)-N-((1r,4r)-4-(3-chloro-4-cyanophenoxy)cyclohexyl)pyridazine-3-carboxamide;
6-(6-((7-bromo-2-(2,6-dioxopiperidin-3-yl)-1-oxoisoindolin-5-yl)methyl)-3,6-diazabicyclo[3.1.1]heptan-3-yl)-N-((1r,4r)-4-(3-chloro-4-cyanophenoxy)cyclohexyl)pyridazine-3-carboxamide;
6-(3-((4-bromo-2-(2,6-dioxopiperidin-3-yl)-1-oxoisoindolin-5-yl)methyl)-3,6-diazabicyclo[3.1.1]heptan-6-yl)-N-((1r,4r)-4-(3-chloro-4-cyanophenoxy)cyclohexyl)pyridazine-3-carboxamide;
6-(3-((6-bromo-2-(2,6-dioxopiperidin-3-yl)-1-oxoisoindolin-5-yl)methyl)-3,8-diazabicyclo[3.2.1]octan-8-yl)-N-((1r,4r)-4-(3-chloro-4-cyanophenoxy)cyclohexyl)pyridazine-3-carboxamide;
6-(4-((7-bromo-2-(2,6-dioxopiperidin-3-yl)-1-oxoisoindolin-5-yl)methyl)piperazin-1-yl)-N-((1r,4r)-4-(3-chloro-4-cyanophenoxy)cyclohexyl)pyridazine-3-carboxamide;
3-(4-bromo-5-((4-(4-((9-cyclopentyl-8-(phenylamino)-9H-purin-2-yl)amino)phenyl)piperazin-1-yl)methyl)-1-oxoisoindolin-2-yl)piperidine-2,6-dione;
3-(6-bromo-5-((4-(4-((9-cyclopentyl-8-(phenylamino)-9H-purin-2-yl)amino)phenyl)piperazin-1-yl)methyl)-1-oxoisoindolin-2-yl)piperidine-2,6-dione;
3-(7-bromo-5-((4-(4-((9-cyclopentyl-8-(phenylamino)-9H-purin-2-yl)amino)phenyl)piperazin-1-yl)methyl)-1-oxoisoindolin-2-yl)piperidine-2,6-dione;
3-(4-bromo-5-(((1-(4-((9-cyclopentyl-8-(phenylamino)-9H-purin-2-yl)amino)phenyl)piperidin-4-yl)(methyl)amino)methyl)-1-oxoisoindolin-2-yl)piperidine-2,6-dione;
3-(6-bromo-5-(((1-(4-((9-cyclopentyl-8-(phenylamino)-9H-purin-2-yl)amino)phenyl)piperidin-4-yl)(methyl)amino)methyl)-1-oxoisoindolin-2-yl)piperidine-2,6-dione;
3-(7-bromo-5-(((1-(4-((9-cyclopentyl-8-(phenylamino)-9H-purin-2-yl)amino)phenyl)piperidin-4-yl)(methyl)amino)methyl)-1-oxoisoindolin-2-yl)piperidine-2,6-dione;
3-(4-bromo-5-((4-(1-(4-((9-cyclopentyl-8-(phenylamino)-9H-purin-2-yl)amino)phenyl)piperidin-4-yl)piperazin-1-yl)methyl)-1-oxoisoindolin-2-yl)piperidine-2,6-dione;
2-(7-(((4-bromo-2-(2,6-dioxopiperidin-3-yl)-1-oxoisoindolin-5-yl)methyl)amino)-1-oxoisoindolin-2-yl)-2-(5-fluoro-2-hydroxyphenyl)-N-(thiazol-2-yl)acetamide;
2-(6-(4-((6-bromo-2-(2,6-dioxopiperidin-3-yl)-1-oxoisoindolin-5-yl)methyl)piperazin-1-yl)-1-oxoisoindolin-2-yl)-2-(5-fluoro-2-hydroxyphenyl)-N-(thiazol-2-yl)acetamide;
2-(6-(4-(((7-bromo-2-(2,6-dioxopiperidin-3-yl)-1-oxoisoindolin-5-yl)methyl)(methyl)amino)piperidin-1-yl)-1-oxoisoindolin-2-yl)-2-(5-fluoro-2-hydroxyphenyl)-N-(thiazol-2-yl)acetamide;
2-(6-(4-(4-((4-bromo-2-(2,6-dioxopiperidin-3-yl)-1-oxoisoindolin-5-yl)methyl)piperazin-1-yl)piperidin-1-yl)-1-oxoisoindolin-2-yl)-2-(5-fluoro-2-hydroxyphenyl)-N-(thiazol-2-yl)acetamide;
2-(6-(6-(4-((6-bromo-2-(2,6-dioxopiperidin-3-yl)-1-oxoisoindolin-5-yl)methyl)piperazin-1-yl)pyridazin-3-yl)-1-oxoisoindolin-2-yl)-2-(5-fluoro-2-hydroxyphenyl)-N-(thiazol-2-yl)acetamide;
3-(4-bromo-5-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)-1-oxoisoindolin-2-yl)piperidine-2,6-dione;
3-(6-bromo-5-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)-1-oxoisoindolin-2-yl)piperidine-2,6-dione;
3-(7-bromo-5-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)-1-oxoisoindolin-2-yl)piperidine-2,6-dione;
3-(4-bromo-5-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)-1-oxoisoindolin-2-yl)piperidine-2,6-dione;
3-(6-bromo-5-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)-1-oxoisoindolin-2-yl)piperidine-2,6-dione;
3-(7-bromo-5-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)-1-oxoisoindolin-2-yl)piperidine-2,6-dione;
3-(4-bromo-5-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)-1-oxoisoindolin-2-yl)piperidine-2,6-dione;
3-(6-bromo-5-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)-1-oxoisoindolin-2-yl)piperidine-2,6-dione;
3-(7-bromo-5-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)-1-oxoisoindolin-2-yl)piperidine-2,6-dione;
3-(4-bromo-5-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)-1-oxoisoindolin-2-yl)piperidine-2,6-dione;
3-(6-bromo-5-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)-1-oxoisoindolin-2-yl)piperidine-2,6-dione;
3-(7-bromo-5-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)-1-oxoisoindolin-2-yl)piperidine-2,6-dione;
1-((4-bromo-2-(2,6-dioxopiperidin-3-yl)-1-oxoisoindolin-5-yl)methyl)-N-(5-(((5-(tert-butyl)oxazol-2-yl)methyl)thio)thiazol-2-yl)piperidine-4-carboxamide;
1'-((6-bromo-2-(2,6-dioxopiperidin-3-yl)-1-oxoisoindolin-5-yl)methyl)-N-(5-(((5-(tert-butyl)oxazol-2-yl)methyl)thio)thiazol-2-yl)-[1,4'-bipiperidine]-4-carboxamide;
4-(1-((7-bromo-2-(2,6-dioxopiperidin-3-yl)-1-oxoisoindolin-5-yl)methyl)piperidin-4-yl)-N-(5-(((5-(tert-butyl)oxazol-2-yl)methyl)thio)thiazol-2-yl)piperazine-1-carboxamide;
N-(1-((4-bromo-2-(2,6-dioxopiperidin-3-yl)-1-oxoisoindolin-5-yl)methyl)piperidin-4-yl)-4-(2,6-dichlorobenzamido)-1H-pyrazole-3-carboxamide;
1-((6-bromo-2-(2,6-dioxopiperidin-3-yl)-1-oxoisoindolin-5-yl)methyl)-N-(5-chloro-4-(5,5-dimethyl-5,6-dihydro-4H-pyrrolo[1,2-b]pyrazol-3-yl)pyridin-2-yl)piperidine-4-carboxamide;
2-(4-((7-bromo-2-(2,6-dioxopiperidin-3-yl)-1-oxoisoindolin-5-yl)methoxy)phenyl)-N-(5-cyclobutyl-1H-pyrazol-3-yl)acetamide;
3-(4-bromo-5-((4-((5-chloro-4-(5-(cyclopropylmethyl)-1-methyl-1H-pyrazol-4-yl)pyrimidin-2-yl)amino)piperidin-1-yl)methyl)-1-oxoisoindolin-2-yl)piperidine-2,6-dione;
4-((6-bromo-2-(2,6-dioxopiperidin-3-yl)-1-oxoisoindolin-5-yl)methyl)-N-(4-(((5-(((S)-1-hydroxybutan-2-yl)amino)-3-isopropylpyrazolo[1,5-a]pyrimidin-7-yl)amino)methyl)phenyl)piperazine-1 -carboxamide;
4-(((4-(2-((1-((4-bromo-2-(2,6-dioxopiperidin-3-yl)-1-oxoisoindolin-5-yl)methyl)piperidin-4-yl)amino)-5-chloropyridin-4-yl)thiazol-2-yl)amino)methyl)tetrahydro-2H-pyran-4-carbonitrile;
4-(((2'-((1-((6-bromo-2-(2,6-dioxopiperidin-3-yl)-1-oxoisoindolin-5-yl)methyl)piperidin-4-yl)amino)-5'-chloro-[2,4'-bipyridin]-6-yl)amino)methyl)tetrahydro-2H-pyran-4-carbonitrile;
3-((4-(2-(((7-bromo-2-(2,6-dioxopiperidin-3-yl)-1-oxoisoindolin-5-yl)methyl)(methyl)amino)-4-methylthiazol-5-yl)-5-fluoropyrimidin-2-yl)amino)benzenesulfonamide;
2-((6-(4-((4-bromo-2-(2,6-dioxopiperidin-3-yl)-1-oxoisoindolin-5-yl)methyl)piperazin-1-yl)-2-methylpyrimidin-4-yl)amino)-N-(2-chloro-6-methylphenyl)thiazole-5-carboxamide;
2-((6-(4-(((6-bromo-2-(2,6-dioxopiperidin-3-yl)-1-oxoisoindolin-5-yl)methyl)(methyl)amino)piperidin-1-yl)-2-methylpyrimidin-4-yl)amino)-N-(2-chloro-6-methylphenyl)thiazole-5-carboxamide;
2-((6-(4-(4-((7-bromo-2-(2,6-dioxopiperidin-3-yl)-1-oxoisoindolin-5-yl)methyl)piperazin-1-yl)piperidin-1-yl)-2-methylpyrimidin-4-yl)amino)-N-(2-chloro-6-methylphenyl)thiazole-5-carboxamide;
4-((4-((4-bromo-2-(2,6-dioxopiperidin-3-yl)-1-oxoisoindolin-5-yl)methyl)piperazin-1-yl)methyl)-N-(4-methyl-3-((4-(pyridin-3-yl)pyrimidin-2-yl)amino)phenyl)benzamide;
4-(4-((6-bromo-2-(2,6-dioxopiperidin-3-yl)-1-oxoisoindolin-5-yl)methyl)piperazin-1-yl)-N-(4-methyl-3-((4-(pyridin-3-yl)pyrimidin-2-yl)amino)phenyl)benzamide;
4-(4-(((7-bromo-2-(2,6-dioxopiperidin-3-yl)-1-oxoisoindolin-5-yl)methyl)(methyl)amino)piperidin-1-yl)-N-(4-methyl-3-((4-(pyridin-3-yl)pyrimidin-2-yl)amino)phenyl)benzamide;
4-(4-(4-((4-bromo-2-(2,6-dioxopiperidin-3-yl)-1-oxoisoindolin-5-yl)methyl)piperazin-1-yl)piperidin-1-yl)-N-(4-methyl-3-((4-(pyridin-3-yl)pyrimidin-2-yl)amino)phenyl)benzamide;
N-(4-((4-((6-bromo-2-(2,6-dioxopiperidin-3-yl)-1-oxoisoindolin-5-yl)methyl)piperazin-1-yl)methyl)-3-(trifluoromethyl)phenyl)-3-(imidazo[1,2-b]pyridazin-3-ylethynyl)-4-methylbenzamide;
N-(4-(4-((7-bromo-2-(2,6-dioxopiperidin-3-yl)-1-oxoisoindolin-5-yl)methyl)piperazin-1-yl)-3-(trifluoromethyl)phenyl)-3-(imidazo[1,2-b]pyridazin-3-ylethynyl)-4-methylbenzamide;
N-(4-(4-(1-((4-bromo-2-(2,6-dioxopiperidin-3-yl)-1-oxoisoindolin-5-yl)methyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(((4-bromo-2-(2,6-dioxopiperidin-3-yl)-1-oxoisoindolin-5-yl)methyl)(methyl)amino)piperidin-1-yl)-3-(trifluoromethyl)phenyl)-3-(imidazo[1,2-b]pyridazin-3 - ylethynyl)-4-methylbenzamide;
2-(4-((3S)-1-((6-bromo-2-(2,6-dioxopiperidin-3-yl)-1-oxoisoindolin-5-yl)methyl)piperidin-3-yl)phenyl)-2H-indazole-7-carboxamide;
2-(4-(1-((6-bromo-2-(2,6-dioxopiperidin-3-yl)-1-oxoisoindolin-5-yl)methyl)piperidin-4-yl)phenyl)-2H-indazole-7-carboxamide;
2-(4-(4-((7-bromo-2-(2,6-dioxopiperidin-3-yl)-1-oxoisoindolin-5-yl)methyl)piperazin-1-yl)phenyl)-2H-indazole-7-carboxamide;
3-(4-bromo-5-((4-(2-fluoro-5-((4-oxo-3,4-dihydrophthalazin-1-yl)methyl)benzoyl)piperazin-1-yl)methyl)-1-oxoisoindolin-2-yl)piperidine-2,6-dione;
3-(6-bromo-5-(((4-(8-fluoro-1-oxo-2,3,4,6-tetrahydro-1H-azepino[5,4,3-cd]indol-5-yl)benzyl)(methyl)amino)methyl)-1-oxoisoindolin-2-yl)piperidine-2,6-dione;
2-((5-(4-((4-bromo-2-(2,6-dioxopiperidin-3-yl)-1-oxoisoindolin-5-yl)methyl)piperazin-1-yl)pyridin-2-yl)amino)-7-cyclopentyl-N,N-dimethyl-7H-pyrrolo[2,3-d]pyrimidine-6-carboxamide;
2-((5-(4-(((6-bromo-2-(2,6-dioxopiperidin-3-yl)-1-oxoisoindolin-5-yl)methyl)(methyl)amino)piperidin-1-yl)pyridin-2-yl)amino)-7-cyclopentyl-N,N-dimethyl-7H-pyrrolo[2,3 -d]pyrimidine-6-carboxamide;
2-((5-(4-(4-((7-bromo-2-(2,6-dioxopiperidin-3-yl)-1-oxoisoindolin-5-yl)methyl)piperazin-1-yl)piperidin-1-yl)pyridin-2-yl)amino)-7-cyclopentyl-N,N-dimethyl-7H-pyrrolo[2,3-d]pyrimidine-6-carboxamide;
3-(4-bromo-5-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-1-oxoisoindolin-2-yl)piperidine-2,6-dione;
3-(6-bromo-5-((4-(difluoro(6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-1-oxoisoindolin-2-yl)piperidine-2,6-dione;
3-(7-bromo-5-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)-1-oxoisoindolin-2-yl)piperidine-2,6-dione;
3-(4-bromo-5-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)-1-oxoisoindolin-2-yl)piperidine-2,6-dione;
3-(6-bromo-5-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)-1-oxoisoindolin-2-yl)piperidine-2,6-dione;
3-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-4-bromo-1-oxoisoindolin-2-yl)piperidine-2,6-dione;
3-(5-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)-6-bromo-1-oxoisoindolin-2-yl)piperidine-2,6-dione;
3-(5-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)-7-bromo-1-oxoisoindolin-2-yl)piperidine-2,6-dione;
3-((4-(4-(4-((4-bromo-2-(2,6-dioxopiperidin-3-yl)-1-oxoisoindolin-5-yl)methyl)piperazin-1-yl)piperidin-1-yl)-3-methoxyphenyl)amino)-6-ethyl-5-((tetrahydro-2H-pyran-4-yl)amino)pyrazine-2-carboxamide;
3-(4-bromo-5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-1-oxoisoindolin-2-yl)piperidine-2,6-dione;
3-(6-bromo-5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-1-oxoisoindolin-2-yl)piperidine-2,6-dione;
3-(7-bromo-5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-1-oxoisoindolin-2-yl)piperidine-2,6-dione;
3-(4-bromo-5-((4-(4-((5-chloro-4-((2-(dimethylphosphoryl)phenyl)amino)pyrimidin-2-yl)amino)-3-methoxyphenyl)piperazin-1-yl)methyl)-1-oxoisoindolin-2-yl)piperidine-2,6-dione;
3-(6-bromo-5-((4-(4-(4-((5-chloro-4-((2-(dimethylphosphoryl)phenyl)amino)pyrimidin-2-yl)amino)-3-methoxyphenyl)piperazin-1-yl)piperidin-1-yl)methyl)-1-oxoisoindolin-2-yl)piperidine-2,6-dione;
8-(4-(4-((4-bromo-2-(2,6-dioxopiperidin-3-yl)-1-oxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-((6-bromo-2-(2,6-dioxopiperidin-3-yl)-1-oxoisoindolin-5-yl)methyl)piperazin-1-yl)-9-ethyl-6,6-dimethyl-11-oxo-6,11-dihydro-5H-benzo[b]carbazole-3-carbonitrile;
8-(4-(((7-bromo-2-(2,6-dioxopiperidin-3-yl)-1-oxoisoindolin-5-yl)methyl)(methyl)amino)piperidin-1-yl)-9-ethyl-6,6-dimethyl-11-oxo-6,11-dihydro-5H-benzo[b]carbazole-3-carbonitrile;
3-(4-bromo-5-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)-1-oxoisoindolin-2-yl)piperidine-2,6-dione;
3-(4-bromo-5-((4-(4-(5,7-dimethoxy-4-oxo-3,4-dihydroquinazolin-2-yl)phenyl)piperazin-1-yl)methyl)-1-oxoisoindolin-2-yl)piperidine-2,6-dione;
3-(6-bromo-5-(((1-(4-(5,7-dimethoxy-4-oxo-3,4-dihydroquinazolin-2-yl)phenyl)piperidin-4-yl)(methyl)amino)methyl)-1-oxoisoindolin-2-yl)piperidine-2,6-dione;
3-(7-bromo-5-((4-(1-(4-(5,7-dimethoxy-4-oxo-3,4-dihydroquinazolin-2-yl)phenyl)piperidin-4-yl)piperazin-1-yl)methyl)-1-oxoisoindolin-2-yl)piperidine-2,6-dione;
2-((2-((4-(4-(4-((4-bromo-2-(2,6-dioxopiperidin-3-yl)-1-oxoisoindolin-5-yl)methyl)piperazin-1-yl)piperidin-1-yl)-2-methoxyphenyl)amino)-5-(trifluoromethyl)pyridin-4-yl)amino)-N-methylbenzamide;
2-((2-((4-(4-(((6-bromo-2-(2,6-dioxopiperidin-3-yl)-1-oxoisoindolin-5-yl)methyl)(methyl)amino)piperidin-1-yl)-2-methoxyphenyl)amino)-5-(trifluoromethyl)pyridin-4-yl)amino)-N-methylbenzamide;
2-((2-((4-(4-((7-bromo-2-(2,6-dioxopiperidin-3-yl)-1-oxoisoindolin-5-yl)methyl)piperazin-1-yl)-2-methoxyphenyl)amino)-5-(trifluoromethyl)pyridin-4-yl)amino)-N-methylbenzamide;
N-(3-(((2-((4-(4-((4-bromo-2-(2,6-dioxopiperidin-3-yl)-1-oxoisoindolin-5-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(((6-bromo-2-(2,6-dioxopiperidin-3-yl)-1-oxoisoindolin-5-yl)methyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-((7-bromo-2-(2,6-dioxopiperidin-3-yl)-1-oxoisoindolin-5-yl)methyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((4-bromo-2-(2,6-dioxopiperidin-3-yl)-1-oxoisoindolin-5-yl)methyl)piperazin-1-yl)phenyl)amino)-5-(trifluoromethyl)pyrimidin-4-yl)amino)methyl)phenyl)-N-methylmethanesulfonamide;
N-(3-(((2-((4-(4-(((6-bromo-2-(2,6-dioxopiperidin-3-yl)-1-oxoisoindolin-5-yl)methyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-((7-bromo-2-(2,6-dioxopiperidin-3-yl)-1-oxoisoindolin-5-yl)methyl)piperazin-1-yl)piperidin-1-yl)phenyl)amino)-5-(trifluoromethyl)pyrimidin-4-yl)amino)methyl)phenyl)-N-methylmethanesulfonamide;
N-(3-(((2-((4-(4-((4-bromo-2-(2,6-dioxopiperidin-3-yl)-1-oxoisoindolin-5-yl)methyl)piperazin-1-yl)phenyl)amino)-5-(trifluoromethyl)pyrimidin-4-yl)amino)methyl)pyridin-2-yl)-N-methylmethanesulfonamide;
N-(3-(((2-((4-(4-(((6-bromo-2-(2,6-dioxopiperidin-3-yl)-1-oxoisoindolin-5-yl)methyl)(methyl)amino)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-((7-bromo-2-(2,6-dioxopiperidin-3-yl)-1-oxoisoindolin-5-yl)methyl)piperazin-1-yl)piperidin-1-yl)phenyl)amino)-5-(trifluoromethyl)pyrimidin-4-yl)amino)methyl)pyridin-2-yl)-N-methylmethanesulfonamide;
N-(1-(1-((4-bromo-2-(2,6-dioxopiperidin-3-yl)-1-oxoisoindolin-5-yl)methyl)piperidin-4-yl)-3-(difluoromethyl)-1H-pyrazol-4-yl)-2-(2-((cyclopropylmethyl)amino)pyridin-4-yl)oxazole-4-carboxamide;
N-(1-(1-((6-bromo-2-(2,6-dioxopiperidin-3-yl)-1-oxoisoindolin-5-yl)methyl)piperidin-4-yl)-3-(difluoromethyl)-1H-pyrazol-4-yl)-5-morpholinopyrazolo[1,5-a]pyrimidine-3-carboxamide;
N-(2-(4-((7-bromo-2-(2,6-dioxopiperidin-3-yl)-1-oxoisoindolin-5-yl)methyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide;
4-((1-((4-bromo-2-(2,6-dioxopiperidin-3-yl)-1-oxoisoindolin-5-yl)methyl)piperidin-4-yl)ethynyl)-1-(((2S,3S,4S)-3-ethyl-4-fluoro-5-oxopyrrolidin-2-yl)methoxy)-7-methoxyisoquinoline-6-carboxamide;
6-(6-(4-((4-bromo-2-(2,6-dioxopiperidin-3-yl)-1-oxoisoindolin-5-yl)methyl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(((6-bromo-2-(2,6-dioxopiperidin-3-yl)-1-oxoisoindolin-5-yl)methyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-((7-bromo-2-(2,6-dioxopiperidin-3-yl)-1-oxoisoindolin-5-yl)methyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
4'-((4-((4-bromo-2-(2,6-dioxopiperidin-3-yl)-1-oxoisoindolin-5-yl)methyl)piperazin-1-yl)methyl)-N-((4,6-dimethyl-2-oxo-1,2-dihydropyridin-3-yl)methyl)-5-(ethyl(tetrahydro-2H-pyran-4-yl)amino)-4-methyl-[1,1'-biphenyl]-3-carboxamide;
4'-(4-((6-bromo-2-(2,6-dioxopiperidin-3-yl)-1-oxoisoindolin-5-yl)methyl)piperazin-1-yl)-N-((4,6-dimethyl-2-oxo-1,2-dihydropyridin-3-yl)methyl)-5-(ethyl(tetrahydro-2H-pyran-4-yl)amino)-4-methyl-[1,1'-biphenyl]-3 -carboxamide;
4-(4-((4-bromo-2-(2,6-dioxopiperidin-3-yl)-1-oxoisoindolin-5-yl)methyl)piperazin-1-yl)-N-(5-(3,5-difluorobenzyl)-1H-indazol-3-yl)-2-((tetrahydro-2H-pyran-4-yl)amino)benzamide;
4-(4-(((6-bromo-2-(2,6-dioxopiperidin-3-yl)-1-oxoisoindolin-5-yl)methyl)(methyl)amino)piperidin-1-yl)-N-(5-(3,5-difluorobenzyl)-1H-indazol-3-yl)-2-((tetrahydro-2H-pyran-4-yl)amino)benzamide;
4-(4-(1-((7-bromo-2-(2,6-dioxopiperidin-3-yl)-1-oxoisoindolin-5-yl)methyl)piperidin-4-yl)piperazin-1-yl)-N-(5-(3,5-difluorobenzyl)-1H-indazol-3-yl)-2-((tetrahydro-2H-pyran-4-yl)amino)benzamide;
3-(4-bromo-5-((4-(6-(6-((R)-2-(3-fluorophenyl)pyrrolidin-1-yl)imidazo[1,2-b]pyridazin-3-yl)pyridin-2-yl)piperazin-1-yl)methyl)-1-oxoisoindolin-2-yl)piperidine-2,6-dione;
3-(6-bromo-5-(((1-(6-(6-((R)-2-(3-fluorophenyl)pyrrolidin-1-yl)imidazo[1,2-b]pyridazin-3-yl)pyridin-2-yl)piperidin-4-yl)(methyl)amino)methyl)-1-oxoisoindolin-2-yl)piperidine-2,6-dione;
3 -(7-bromo-5-((4-(4-(6-(6-((R)-2-(3-fluorophenyl)pyrrolidin-1-yl)imidazo[1,2-b]pyridazin-3-yl)pyridin-2-yl)piperazin-1-yl)piperidin-1-yl)methyl)-1-oxoisoindolin-2-yl)piperidine-2,6-dione;
3-(4-bromo-5-((4-(6-(5-((R)-2-(2,5-difluorophenyl)pyrrolidin-1-yl)pyrazolo[1,5-a]pyrimidin-3-yl)pyridin-2-yl)piperazin-1-yl)methyl)-1-oxoisoindolin-2-yl)piperidine-2,6-dione;
3-(6-bromo-5-(((1-(6-(5-((R)-2-(2,5-difluorophenyl)pyrrolidin-1-yl)pyrazolo[1,5-a]pyrimidin-3-yl)pyridin-2-yl)piperidin-4-yl)(methyl)amino)methyl)-1-oxoisoindolin-2-yl)piperidine-2,6-dione;
3-(7-bromo-5-((4-(4-(6-(5-((R)-2-(2,5-difluorophenyl)pyrrolidin-1-yl)pyrazolo[1,5-a]pyrimidin-3-yl)pyridin-2-yl)piperazin-1-yl)piperidin-1-yl)methyl)-1-oxoisoindolin-2-yl)piperidine-2,6-dione;
N-((4-(((2R)-4-(4-((4-bromo-2-(2,6-dioxopiperidin-3-yl)-1-oxoisoindolin-5-yl)methyl)piperazin-1-yl-2,2,3,3,5,5,6,6-*d*₈)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl] -2-yl)methyl)piperazin-1-yl)benzamide;
N-((4-(((2R)-4-(4-((6-bromo-2-(2,6-dioxopiperidin-3-yl)-1-oxoisoindolin-5-yl)methyl)-3,5-dimethylpiperazin-1-yl)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl] -2-yl)methyl)piperazin-1-yl)benzamide;
N-((4-(((2R)-4-(4-((7-bromo-2-(2,6-dioxopiperidin-3-yl)-1-oxoisoindolin-5-yl)methyl)-2,6-dimethylpiperazin-1-yl)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl] -2-yl)methyl)piperazin-1-yl)benzamide;
N-((4-(((2R)-4-(6-((4-bromo-2-(2,6-dioxopiperidin-3-yl)-1-oxoisoindolin-5-yl)methyl)-3,6-diazabicyclo[3.1.1]heptan-3-yl)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl] -2-yl)methyl)piperazin-1-yl)benzamide;
N-((4-(((2R)-4-(3-((6-bromo-2-(2,6-dioxopiperidin-3-yl)-1-oxoisoindolin-5-yl)methyl)-3,6-diazabicyclo[3.1.1]heptan-6-yl)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl]-2-yl)methyl)piperazin-1-yl)benzamide;
N-((4-(((2R)-4-(8-((7-bromo-2-(2,6-dioxopiperidin-3-yl)-1-oxoisoindolin-5-yl)methyl)-3,8-diazabicyclo[3.2.1]octan-3-yl)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl]-2-yl)methyl)piperazin-1-yl)benzamide;
N-((4-(((2R)-4-(3-((4-bromo-2-(2,6-dioxopiperidin-3-yl)-1-oxoisoindolin-5-yl)methyl)-3,8-diazabicyclo[3.2.1]octan-8-yl)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl]-2-yl)methyl)piperazin-1-yl)benzamide;
N-((4-(((2R)-4-((1R,4R)-5-((6-bromo-2-(2,6-dioxopiperidin-3-yl)-1-oxoisoindolin-5-yl)methyl)-2,5-diazabicyclo[2.2.1]heptan-2-yl)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl]-2-yl)methyl)piperazin-1-yl)benzamide;
N-((4-(((2R)-4-((1S,4S)-5-((7-bromo-2-(2,6-dioxopiperidin-3-yl)-1-oxoisoindolin-5-yl)methyl)-2,5-diazabicyclo[2.2.1]heptan-2-yl)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl]-2-yl)methyl)piperazin-1-yl)benzamide;
N-((4-(((2R)-4-(5-((4-bromo-2-(2,6-dioxopiperidin-3-yl)-1-oxoisoindolin-5-yl)methyl)-2,5-diazabicyclo[2.2.2]octan-2-yl)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl]-2-yl)methyl)piperazin-1-yl)benzamide;
N-((4-(((2R)-4-(4-((4-bromo-2-(2,6-dioxopiperidin-3-yl)-1-oxoisoindolin-5-yl)methyl)piperazin-1-yl)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl]-2-yl)methyl)piperazin-1-yl)benzamide;
N-((4-(((2R)-4-(4-((6-bromo-2-(2,6-dioxopiperidin-3-yl)-1-oxoisoindolin-5-yl)methyl)piperazin-1-yl)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl]-2-yl)methyl)piperazin-1-yl)benzamide;
N-((4-(((2R)-4-(4-((7-bromo-2-(2,6-dioxopiperidin-3-yl)-1-oxoisoindolin-5-yl)methyl)piperazin-1-yl)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl]-2-yl)methyl)piperazin-1-yl)benzamide;
N-(5-(3,5-dimethoxyphenethyl)-1H-pyrazol-3-yl)-4-((3R,5S)-4-((2-(2,6-dioxopiperidin-3-yl)-1-oxoisoindolin-5-yl)methyl)-3,5-dimethylpiperazin-1-yl)benzamide;
3-(1-oxo-5-((4-(4-(3-(quinolin-4-yl)pyrazolo[1,5-a]pyrimidin-6-yl)phenyl)piperazin-1-yl)methyl)isoindolin-2-yl)piperidine-2,6-dione;
3-(1-oxo-5-((4-(4-(3-(quinolin-5-yl)pyrazolo[1,5-a]pyrimidin-6-yl)phenyl)piperazin-1-yl)methyl)isoindolin-2-yl)piperidine-2,6-dione;
1'-((2-(2,6-dioxopiperidin-3-yl)-1-oxoisoindolin-5-yl)methyl)-6-(4-phenoxyphenyl)-1',2',3',6'-tetrahydro-[2,4'-bipyridine]-5-carboxamide;
6-(4-(((2-(2,6-dioxopiperidin-3-yl)-1-oxoisoindolin-4-yl)methyl)(methyl)amino)piperidin-1-yl)-2-(4-phenoxyphenyl)nicotinamide;
6-(4-(4-((2-(2,6-dioxopiperidin-3-yl)-1-oxoisoindolin-4-yl)methyl)piperazin-1-yl)piperidin-1-yl)-2-(4-phenoxyphenyl)nicotinamide;
3 -(5 -((4-(4-amino-3-(4-phenoxyphenyl)-1H-pyrazolo [3,4-d]pyrimidin-1 -yl)piperidin-1 - yl)methyl)-4-fluoro-1-oxoisoindolin-2-yl)piperidine-2,6-dione;
3 -(4-((4-(4-amino-3-(4-phenoxyphenyl)-1H-pyrazolo [3,4-d]pyrimidin-1 -yl)piperidin-1 - yl)methyl)-1-oxoisoindolin-2-yl)piperidine-2,6-dione;
3-(5-((4-(4-(4-chloro-1,2-diphenylbut-1-en-1-yl)phenyl)piperazin-1-yl)methyl)-1-oxoisoindolin-2-yl)piperidine-2,6-dione;
3-(4-((4-(4-((9-cyclopentyl-8-(phenylamino)-9H-purin-2-yl)amino)phenyl)piperazin-1-yl)methyl)-1-oxoisoindolin-2-yl)piperidine-2,6-dione;
3-(5-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)-1-oxoisoindolin-2-yl)piperidine-2,6-dione;
4-((((2-(2,6-dioxopiperidin-3-yl)-1-oxoisoindolin-5-yl)methyl)(methyl)amino)methyl)-N-(4-methyl-3-((4-(pyridin-3-yl)pyrimidin-2-yl)amino)phenyl)benzamide;
N-(4-((4-((2-(2,6-dioxopiperidin-3-yl)-1-oxoisoindolin-4-yl)methyl)piperazin-1-yl)methyl)-3-(trifluoromethyl)phenyl)-3-(imidazo[1,2-b]pyridazin-3-ylethynyl)-4-methylbenzamide;
N-(4-(4-(1-((2-(2,6-dioxopiperidin-3-yl)-1-oxoisoindolin-4-yl)methyl)piperidin-4-yl)piperazin-1-yl)-3-(trifluoromethyl)phenyl)-3-(imidazo[1,2-b]pyridazin-3-ylethynyl)-4-methylbenzamide;
3-(4-((4-(4-((5-chloro-4-((2-(dimethylphosphoryl)phenyl)amino)pyrimidin-2-yl)amino)-3-methoxyphenyl)piperazin-1-yl)methyl)-1-oxoisoindolin-2-yl)piperidine-2,6-dione;
8-(4-(4-((2-(2,6-dioxopiperidin-3-yl)-1-oxoisoindolin-4-yl)methyl)piperazin-1-yl)piperidin-1-yl)-9-ethyl-6,6-dimethyl-11-oxo-6,11-dihydro-5H-benzo[b]carbazole-3-carbonitrile;
2-((2-((4-(4-(((2-(2,6-dioxopiperidin-3-yl)-1-oxoisoindolin-4-yl)methyl)(methyl)amino)piperidin-1-yl)-2-methoxyphenyl)amino)-5-(trifluoromethyl)pyridin-4-yl)amino)-N-methylbenzamide;
2-((2-((4-(4-((2-(2,6-dioxopiperidin-3-yl)-1-oxoisoindolin-4-yl)methyl)piperazin-1-yl)-2-methoxyphenyl)amino)-5-(trifluoromethyl)pyridin-4-yl)amino)-N-methylbenzamide;
*N*-(3-(((2-((4-(4-(((2-(2,6-dioxopiperidin-3-yl)-1-oxoisoindolin-5-yl)methyl)(methyl)amino)piperidin-1-yl)phenyl)amino)-5-(trifluoromethyl)pyrimidin-4-yl)amino)methyl)pyrazin-2-yl)-N-methylmethanesulfonamide;
*N*-(3-(((2-((4-(4-(4-((2-(2,6-dioxopiperidin-3-yl)-1-oxoisoindolin-5-yl)methyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
*N*-(3-(((2-((4-(1-((2-(2,6-dioxopiperidin-3-yl)-1-oxoisoindolin-5-yl)methyl)piperidin-4-yl)phenyl)amino)-5-(trifluoromethyl)pyrimidin-4-yl)amino)methyl)phenyl)-N-methylmethanesulfonamide;
N-(3-(difluoromethyl)-1-(1-((2-(2,6-dioxopiperidin-3-yl)-1-oxoisoindolin-4-yl)methyl)piperidin-4-yl)-1H-pyrazol-4-yl)-5-morpholinopyrazolo[1,5-a]pyrimidine-3-carboxamide;
N-((4, 6-dimethyl-2-oxo-1, 2-dihydropyridin-3 -yl)methyl)-4'-(4-(((2-(2, 6-dioxopip eri din-3 -yl)-1-oxoisoindolin-5-yl)methyl)(methyl)amino)piperidin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(1-((2-(2,6-dioxopiperidin-3-yl)-1-oxoisoindolin-5-yl)methyl)piperidin-4-yl)piperazin-1-yl)-5-(ethyl(tetrahydro-2H-pyran-4-yl)amino)-4-methyl-[1,1'-biphenyl]-3-carboxamide;
N-(5-(3,5-difluorobenzyl)-1H-indazol-3-yl)-4-(4-(((2-(2,6-dioxopiperidin-3-yl)-1-oxoisoindolin-4-yl)methyl)(methyl)amino)piperidin-1-yl)-2-((tetrahydro-2H-pyran-4-yl)amino)benzamide;
3-(5-((4-((4-(((R)-1-(3-amino-5-(trifluoromethyl)phenyl)ethyl)amino)-7-methoxy-2-methylquinazolin-6-yl)oxy)piperidin-1-yl)methyl)-1-oxoisoindolin-2-yl)piperidine-2,6-dione;
3-(4-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)-1-oxoisoindolin-2-yl)piperidine-2,6-dione;
3-(4-((4-(4-(1,2-bis(4-hydroxyphenyl)but-1-en-1-yl)phenyl)piperazin-1-yl)methyl)-1-oxoisoindolin-2-yl)piperidine-2,6-dione;
3-(5-((4-(4-((1S,2R)-6-hydroxy-2-phenyl-1,2,3,4-tetrahydronaphthalen-1-yl)phenyl)piperazin-1-yl)methyl)-1-oxoisoindolin-2-yl)piperidine-2,6-dione;
3-(4-fluoro-5-((4-(4-((1S,2R)-6-hydroxy-2-phenyl-1,2,3,4-tetrahydronaphthalen-1-yl)phenyl)piperazin-1-yl)methyl)-1-oxoisoindolin-2-yl)piperidine-2,6-dione;
3-(6-fluoro-5-((4-(4-((1S,2R)-6-hydroxy-2-phenyl-1,2,3,4-tetrahydronaphthalen-1-yl)phenyl)piperazin-1-yl)methyl)-1-oxoisoindolin-2-yl)piperidine-2,6-dione;
3-(7-fluoro-5-((4-(4-((1S,2R)-6-hydroxy-2-phenyl-1,2,3,4-tetrahydronaphthalen-1-yl)phenyl)piperazin-1-yl)methyl)-1-oxoisoindolin-2-yl)piperidine-2,6-dione;
3-(4-((4-(4-((1R,2S)-6-hydroxy-2-phenyl-1,2,3,4-tetrahydronaphthalen-1-yl)phenyl)piperazin-1-yl)methyl)-1-oxoisoindolin-2-yl)piperidine-2,6-dione;
3-(4-((4-(4-((1S,2R)-6-hydroxy-2-phenyl-1,2,3,4-tetrahydronaphthalen-1-yl)phenyl)piperazin-1-yl)methyl)-1-oxoisoindolin-2-yl)piperidine-2,6-dione;
3 -(5 -((4-(4-((3 R, 4 S)-7-hydroxy-3 -phenylchroman-4-yl)phenyl)pip erazin-1-yl) methyl)-1-oxoisoindolin-2-yl)piperidine-2,6-dione;
3-(4-fluoro-5-((4-(4-((3R,4S)-7-hydroxy-3-phenylchroman-4-yl)phenyl)piperazin-1-yl)methyl)-1-oxoisoindolin-2-yl)piperidine-2,6-dione;
3-(6-fluoro-5-((4-(4-((3R,4S)-7-hydroxy-3-phenylchroman-4-yl)phenyl)piperazin-1-yl)methyl)-1-oxoisoindolin-2-yl)piperidine-2,6-dione;
3-(7-fluoro-5-((4-(4-((3R,4S)-7-hydroxy-3-phenylchroman-4-yl)phenyl)piperazin-1-yl)methyl)-1-oxoisoindolin-2-yl)piperidine-2,6-dione;
3-(4-((4-(4-((3S,4R)-7-hydroxy-3-phenylchroman-4-yl)phenyl)piperazin-1-yl)methyl)-1-oxoisoindolin-2-yl)piperidine-2,6-dione;
3 -(4-((4-(4-((3 R, 4 S)-7-hydroxy-3 -phenylchroman-4-yl)phenyl)pip erazin-1-yl) methyl)-1-oxoisoindolin-2-yl)piperidine-2,6-dione;
3-(4-(((1-(4-((9-cyclopentyl-8-(phenylamino)-9H-purin-2-yl)amino)phenyl)piperidin-4-yl)(methyl)amino)methyl)-1-oxoisoindolin-2-yl)piperidine-2,6-dione;
3-(5-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)-1-oxoisoindolin-2-yl)piperidine-2,6-dione;
3-(5-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)-4-fluoro-1-oxoisoindolin-2-yl)piperidine-2,6-dione;
3-(5-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)-6-fluoro-1-oxoisoindolin-2-yl)piperidine-2,6-dione;
3-(5-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)-7-fluoro-1-oxoisoindolin-2-yl)piperidine-2,6-dione;
3-(4-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)-1-oxoisoindolin-2-yl)piperidine-2,6-dione;
3-(5-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)-1oxoisoindolin-2-yl)piperidine-2,6-dione;
3-(5-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)-4-fluoro-1-oxoisoindolin-2-yl)piperidine-2,6-dione;
3-(5-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)-6-fluoro-1-oxoisoindolin-2-yl)piperidine-2,6-dione;
3-(5-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)-7-fluoro-1-oxoisoindolin-2-yl)piperidine-2,6-dione;
3-(4-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)-1-oxoisoindolin-2-yl)piperidine-2,6-dione;
3-(5-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)-1-oxoisoindolin-2-yl)piperidine-2,6-dione;
3-(4-((4-((5-chloro-4-(5-(cyclopropylmethyl)-1-methyl-1H-pyrazol-4-yl)pyrimidin-2-yl)amino)piperidin-1-yl)methyl)-1-oxoisoindolin-2-yl)piperidine-2,6-dione;
4-(((4-(5-chloro-2-((1-((2-(2,6-dioxopiperidin-3-yl)-1-oxoisoindolin-4-yl)methyl)piperidin-4-yl)amino)pyridin-4-yl)thiazol-2-yl)amino)methyl)tetrahydro-2H-pyran-4-carbonitrile;
4-(((5'-chloro-2'-((1-((2-(2,6-dioxopiperidin-3-yl)-1-oxoisoindolin-4-yl)methyl)piperidin-4-yl)amino)-[2,4'-bipyridin]-6-yl)amino)methyl)tetrahydro-2H-pyran-4-carbonitrile;
N-(4-(chlorodifluoromethoxy)phenyl)-6-(4-(((2-(2,6-dioxopiperidin-3-yl)-1-oxoisoindolin-5-yl)methyl)(methyl)amino)piperidin-1-yl)-5-(1H-pyrazol-5-yl)nicotinamide;
N-(4-(chlorodifluoromethoxy)phenyl)-6-(4-(((2-(2,6-dioxopiperidin-3-yl)-4-fluoro-1-oxoisoindolin-5-yl)methyl)(methyl)amino)piperidin-1-yl)-5-(1H-pyrazol-5-yl)nicotinamide;
N-(4-(chlorodifluoromethoxy)phenyl)-6-(4-(((2-(2,6-dioxopiperidin-3-yl)-6-fluoro-1-oxoisoindolin-5-yl)methyl)(methyl)amino)piperidin-1-yl)-5-(1H-pyrazol-5-yl)nicotinamide;
N-(4-(chlorodifluoromethoxy)phenyl)-6-(4-(((2-(2,6-dioxopiperidin-3-yl)-7-fluoro-1-oxoisoindolin-5-yl)methyl)(methyl)amino)piperidin-1-yl)-5-(1H-pyrazol-5-yl)nicotinamide;
N-(4-(chlorodifluoromethoxy)phenyl)-6-(4-(((2-(2,6-dioxopiperidin-3-yl)-1-oxoisoindolin-4-yl)methyl)(methyl)amino)piperidin-1-yl)-5-(1H-pyrazol-5-yl)nicotinamide;
N-(4-(chlorodifluoromethoxy)phenyl)-6-(4-(1-((2-(2,6-dioxopiperidin-3-yl)-1-oxoisoindolin-5-yl)methyl)piperidin-4-yl)piperazin-1-yl)-5-(1H-pyrazol-5-yl)nicotinamide;
N-(4-(chlorodifluoromethoxy)phenyl)-6-(4-(1-((2-(2,6-dioxopiperidin-3-yl)-4-fluoro-1-oxoisoindolin-5-yl)methyl)piperidin-4-yl)piperazin-1-yl)-5-(1H-pyrazol-5-yl)nicotinamide;
N-(4-(chlorodifluoromethoxy)phenyl)-6-(4-(1-((2-(2,6-dioxopiperidin-3-yl)-6-fluoro-1-oxoisoindolin-5-yl)methyl)piperidin-4-yl)piperazin-1-yl)-5-(1H-pyrazol-5-yl)nicotinamide;
N-(4-(chlorodifluoromethoxy)phenyl)-6-(4-(1-((2-(2,6-dioxopiperidin-3-yl)-7-fluoro-1-oxoisoindolin-5-yl)methyl)piperidin-4-yl)piperazin-1-yl)-5-(1H-pyrazol-5-yl)nicotinamide;
N-(4-(chlorodifluoromethoxy)phenyl)-6-(4-(1-((2-(2,6-dioxopiperidin-3-yl)-1-oxoisoindolin-4-yl)methyl)piperidin-4-yl)piperazin-1-yl)-5-(1H-pyrazol-5-yl)nicotinamide;
N-(4-(4-(((2-(2,6-dioxopiperidin-3-yl)-1-oxoisoindolin-4-yl)methyl)(methyl)amino)piperidin-1-yl)-3-(trifluoromethyl)phenyl)-3-(imidazo[1,2-b]pyridazin-3-ylethynyl)-4-methylbenzamide;
3-(4-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-1-oxoisoindolin-2-yl)piperidine-2,6-dione;
2-((2-((4-(4-(4-((2-(2,6-dioxopiperidin-3-yl)-1-oxoisoindolin-4-yl)methyl)piperazin-1-yl)piperidin-1-yl)-2-methoxyphenyl)amino)-5-(trifluoromethyl)pyridin-4-yl)amino)-N-methylbenzamide;
2-((2-((4-(1-((2-(2,6-dioxopiperidin-3-yl)-1-oxoisoindolin-5-yl)methyl)piperidin-4-yl)-2-isopropoxy-5-methylphenyl)amino)-5-(trifluoromethyl)pyridin-4-yl)amino)-N-methylbenzamide;
2-((2-((4-(1-((2-(2,6-dioxopiperidin-3-yl)-4-fluoro-1-oxoisoindolin-5-yl)methyl)piperidin-4-yl)-2-isopropoxy-5-methylphenyl)amino)-5-(trifluoromethyl)pyridin-4-yl)amino)-N-methylbenzamide;
2-((2-((4-(1-((2-(2,6-dioxopiperidin-3-yl)-6-fluoro-1-oxoisoindolin-5-yl)methyl)piperidin-4-yl)-2-isopropoxy-5-methylphenyl)amino)-5-(trifluoromethyl)pyridin-4-yl)amino)-N-methylbenzamide;
2-((2-((4-(1-((2-(2,6-dioxopiperidin-3-yl)-7-fluoro-1-oxoisoindolin-5-yl)methyl)piperidin-4-yl)-2-isopropoxy-5-methylphenyl)amino)-5-(trifluoromethyl)pyridin-4-yl)amino)-N-methylbenzamide;
2-((2-((4-(1-((2-(2,6-dioxopiperidin-3-yl)-1-oxoisoindolin-4-yl)methyl)piperidin-4-yl)-2-isopropoxy-5-methylphenyl)amino)-5-(trifluoromethyl)pyridin-4-yl)amino)-N-methylbenzamide;
2-((2-((4-(4-((2-(2,6-dioxopiperidin-3-yl)-1-oxoisoindolin-5-yl)methyl)piperazin-1-yl)-2-isopropoxy-5-methylphenyl)amino)-5-(trifluoromethyl)pyridin-4-yl)amino)-N-methylbenzamide;
2-((2-((4-(4-((2-(2,6-dioxopiperidin-3-yl)-4-fluoro-1-oxoisoindolin-5-yl)methyl)piperazin-1-yl)-2-isopropoxy-5-methylphenyl)amino)-5-(trifluoromethyl)pyridin-4-yl)amino)-N-methylbenzamide;
2-((2-((4-(4-((2-(2,6-dioxopiperidin-3-yl)-6-fluoro-1-oxoisoindolin-5-yl)methyl)piperazin-1-yl)-2-isopropoxy-5-methylphenyl)amino)-5-(trifluoromethyl)pyridin-4-yl)amino)-N-methylbenzamide;
2-((2-((4-(4-((2-(2,6-dioxopiperidin-3-yl)-7-fluoro-1-oxoisoindolin-5-yl)methyl)piperazin-1-yl)-2-isopropoxy-5-methylphenyl)amino)-5-(trifluoromethyl)pyridin-4-yl)amino)-N-methylbenzamide;
2-((2-((4-(4-((2-(2,6-dioxopiperidin-3-yl)-1-oxoisoindolin-4-yl)methyl)piperazin-1-yl)-2-isopropoxy-5-methylphenyl)amino)-5-(trifluoromethyl)pyridin-4-yl)amino)-N-methylbenzamide;
2-((2-((4-(4-(((2-(2,6-dioxopiperidin-3-yl)-1-oxoisoindolin-5-yl)methyl)(methyl)amino)piperidin-1-yl)-2-isopropoxy-5-methylphenyl)amino)-5-(trifluoromethyl)pyridin-4-yl)amino)-N-methylbenzamide;
2-((2-((4-(4-(((2-(2,6-dioxopiperidin-3-yl)-4-fluoro-1-oxoisoindolin-5-yl)methyl)(methyl)amino)piperidin-1-yl)-2-isopropoxy-5-methylphenyl)amino)-5-(trifluoromethyl)pyridin-4-yl)amino)-N-methylbenzamide;
2-((2-((4-(4-(((2-(2,6-dioxopiperidin-3-yl)-6-fluoro-1-oxoisoindolin-5-yl)methyl)(methyl)amino)piperidin-1-yl)-2-isopropoxy-5-methylphenyl)amino)-5-(trifluoromethyl)pyridin-4-yl)amino)-N-methylbenzamide;
2-((2-((4-(4-(((2-(2,6-dioxopiperidin-3-yl)-7-fluoro-1-oxoisoindolin-5-yl)methyl)(methyl)amino)piperidin-1-yl)-2-isopropoxy-5-methylphenyl)amino)-5-(trifluoromethyl)pyridin-4-yl)amino)-N-methylbenzamide;
2-((2-((4-(4-(((2-(2,6-dioxopiperidin-3-yl)-1-oxoisoindolin-4-yl)methyl)(methyl)amino)piperidin-1-yl)-2-isopropoxy-5-methylphenyl)amino)-5-(trifluoromethyl)pyridin-4-yl)amino)-N-methylbenzamide;
2-((2-((4-(4-(1-((2-(2,6-dioxopiperidin-3-yl)-1-oxoisoindolin-5-yl)methyl)piperidin-4-yl)piperazin-1-yl)-2-isopropoxy-5-methylphenyl)amino)-5-(trifluoromethyl)pyridin-4-yl)amino)-N-methylbenzamide;
N-(1-((2-(2,6-dioxopiperidin-3-yl)-1-oxoisoindolin-5-yl)methyl)piperidin-4-yl)-2-fluoro-5-methoxy-4-((4-((2-methyl-3-oxoisoindolin-4-yl)oxy)-5-(trifluoromethyl)pyrimi din-2-yl)amino)benzamide;
N-(1-((2-(2,6-dioxopiperidin-3-yl)-4-fluoro-1-oxoisoindolin-5-yl)methyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide;
N-(1-((2-(2,6-dioxopiperidin-3-yl)-6-fluoro-1-oxoisoindolin-5-yl)methyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide;
N-(1-((2-(2,6-dioxopiperidin-3-yl)-7-fluoro-1-oxoisoindolin-5-yl)methyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide;
N-(1-((2-(2,6-dioxopiperidin-3-yl)-1-oxoisoindolin-4-yl)methyl)piperidin-4-yl)-2-fluoro-5-methoxy-4-((4-((2-methyl-3-oxoisoindolin-4-yl)oxy)-5-(trifluoromethyl)pyrimi din-2-yl)amino)benzamide;
N-(3-(((2-((4-(4-((2-(2,6-dioxopiperidin-3-yl)-1-oxoisoindolin-5-yl)methyl)piperazin-1-yl)phenyl)amino)-5-(trifluoromethyl)pyrimidin-4-yl)amino)methyl)pyrazin-2-yl)-N-methylmethanesulfonamide;
3-(5-((4-(4-((4-((3-(methylsulfonyl)benzyl)amino)-5-(trifluoromethyl)pyrimidin-2-yl)amino)phenyl)piperazin-1-yl)methyl)-1-oxoisoindolin-2-yl)piperidine-2,6-dione;
3-(4-fluoro-5-((4-(4-((4-((3-(methylsulfonyl)benzyl)amino)-5-(trifluoromethyl)pyrimidin-2-yl)amino)phenyl)piperazin-1-yl)methyl)-1-oxoisoindolin-2-yl)piperidine-2,6-dione;
3-(6-fluoro-5-((4-(4-((4-((3-(methylsulfonyl)benzyl)amino)-5-(trifluoromethyl)pyrimidin-2-yl)amino)phenyl)piperazin-1-yl)methyl)-1-oxoisoindolin-2-yl)piperidine-2,6-dione;
3-(7-fluoro-5-((4-(4-((4-((3-(methylsulfonyl)benzyl)amino)-5-(trifluoromethyl)pyrimidin-2-yl)amino)phenyl)piperazin-1-yl)methyl)-1-oxoisoindolin-2-yl)piperidine-2,6-dione;
3-(4-((4-(4-((4-((3-(methylsulfonyl)benzyl)amino)-5-(trifluoromethyl)pyrimidin-2-yl)amino)phenyl)piperazin-1-yl)methyl)-1-oxoisoindolin-2-yl)piperidine-2,6-dione;
N-(2-(4-((2-(2,6-dioxopiperidin-3-yl)-1-oxoisoindolin-4-yl)methyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide;
3-(4-((4-(6-(6-((R)-2-(3-fluorophenyl)pyrrolidin-1-yl)imidazo[1,2-b]pyridazin-3-yl)pyridin-2-yl)pip erazin-1-yl)methyl)-1-oxoisoindolin-2-yl)piperidine-2,6-dione;
2-(2,6-dioxopiperidin-3-yl)-5-((4-(4-((1S,2R)-6-hydroxy-2-phenyl-1,2,3,4-tetrahydronaphthalen-1-yl)phenyl)piperazin-1-yl)methyl)isoindoline-1,3-dione;
2-(2,6-dioxopiperidin-3-yl)-5-((4-(4-((3R,4S)-7-hydroxy-3-phenylchroman-4-yl)phenyl)piperazin-1-yl)methyl)isoindoline-1,3-dione;
5-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)-2-(2,6-dioxopiperidin-3-yl)isoindoline-1,3-dione;
5-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)-2-(2,6-dioxopiperi din-3-yl)isoindoline-1,3-dione;
N-(4-(chlorodifluoromethoxy)phenyl)-6-(4-(((2-(2,6-dioxopiperidin-3-yl)-1,3-dioxoisoindolin-5-yl)methyl)(methyl)amino)piperidin-1-yl)-5-(1H-pyrazol-5-yl)nicotinamide;
N-(4-(chlorodifluoromethoxy)phenyl)-6-(4-(1-((2-(2,6-dioxopiperidin-3-yl)-1,3-dioxoisoindolin-5-yl)methyl)piperidin-4-yl)piperazin-1-yl)-5-(1H-pyrazol-5-yl)nicotinamide;
2-((2-((4-(1-((2-(2,6-dioxopiperidin-3-yl)-1,3-dioxoisoindolin-5-yl)methyl)piperidin-4-yl)-2-isopropoxy-5-methylphenyl)amino)-5-(trifluoromethyl)pyridin-4-yl)amino)-N-methylbenzamide;
2-((2-((4-(4-((2-(2,6-dioxopiperidin-3-yl)-1,3-dioxoisoindolin-5-yl)methyl)piperazin-1-yl)-2-isopropoxy-5-methylphenyl)amino)-5-(trifluoromethyl)pyridin-4-yl)amino)-N-methylbenzamide;
2-((2-((4-(4-(((2-(2,6-dioxopiperidin-3-yl)-1,3-dioxoisoindolin-5-yl)methyl)(methyl)amino)piperidin-1-yl)-2-isopropoxy-5-methylphenyl)amino)-5-(trifluoromethyl)pyridin-4-yl)amino)-N-methylbenzamide;
N-(1-((2-(2,6-dioxopiperidin-3-yl)-1,3-dioxoisoindolin-5-yl)methyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide;
2-(2,6-dioxopiperidin-3-yl)-5-((4-(4-((4-((3-(methylsulfonyl)benzyl)amino)-5-(trifluoromethyl)pyrimidin-2-yl)amino)phenyl)piperazin-1-yl)methyl)isoindoline-1,3-dione;
N-(3-(difluoromethyl)-1-(1-((2-(2,6-dioxopiperidin-3-yl)-1-oxoisoindolin-5-yl)methyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(1-((2-(2,6-dioxopiperidin-3-yl)-4-fluoro-1-oxoisoindolin-5-yl)methyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(1-((2-(2,6-dioxopiperidin-3-yl)-6-fluoro-1-oxoisoindolin-5-yl)methyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(1-((2-(2,6-dioxopiperidin-3-yl)-7-fluoro-1-oxoisoindolin-5-yl)methyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(1-((2-(2,6-dioxopiperidin-3-yl)-1-oxoisoindolin-4-yl)methyl)piperidin-4-yl)-1H-pyrazol-4-yl)-2-(2-((2,2,2-trifluoroethyl)amino)pyridin-4-yl)oxazole-4-carboxamide;
6-(5-cyano-1H-pyrrolo[2,3-b]pyridin-1-yl)-N-(1-((2-(2,6-dioxopiperidin-3-yl)-1-oxoisoindolin-5-yl)methyl)piperidin-4-yl)-4-(isopropylamino)nicotinamide;
6-(5-cyano-1H-pyrrolo[2,3-b]pyridin-1-yl)-N-(1-((2-(2,6-dioxopiperidin-3-yl)-4-fluoro-1-oxoisoindolin-5-yl)methyl)piperidin-4-yl)-4-(isopropylamino)nicotinamide;
6-(5-cyano-1H-pyrrolo[2,3-b]pyridin-1-yl)-N-(1-((2-(2,6-dioxopiperidin-3-yl)-6-fluoro-1-oxoisoindolin-5-yl)methyl)piperidin-4-yl)-4-(isopropylamino)nicotinamide;
6-(5-cyano-1H-pyrrolo[2,3-b]pyridin-1-yl)-N-(1-((2-(2,6-dioxopiperidin-3-yl)-7-fluoro-1-oxoisoindolin-5-yl)methyl)piperidin-4-yl)-4-(isopropylamino)nicotinamide;
6-(5-cyano-1H-pyrrolo[2,3-b]pyridin-1-yl)-N-(1-((2-(2,6-dioxopiperidin-3-yl)-1-oxoisoindolin-4-yl)methyl)piperidin-4-yl)-4-(isopropylamino)nicotinamide;
6-(5-cyano-1H-pyrrolo[2,3-b]pyridin-1-yl)-N-((1r,4r)-4-(((2-(2,6-dioxopiperidin-3-yl)-1-oxoisoindolin-5-yl)methyl)carbamoyl)cyclohexyl)-4-(isopropylamino)nicotinamide;
2-(2-((cyclopropylmethyl)amino)pyridin-4-yl)-N-(3-(difluoromethyl)-1-(1-((2-(2,6-dioxopiperidin-3-yl)-1-oxoisoindolin-5-yl)methyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-((2-(2,6-dioxopiperidin-3-yl)-4-fluoro-1-oxoisoindolin-5-yl)methyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-((2-(2,6-dioxopiperidin-3-yl)-6-fluoro-1-oxoisoindolin-5-yl)methyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-((2-(2,6-dioxopiperidin-3-yl)-7-fluoro-1-oxoisoindolin-5-yl)methyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-((2-(2,6-dioxopiperidin-3-yl)-1-oxoisoindolin-4-yl)methyl)piperidin-4-yl)-1H-pyrazol-4-yl)oxazole-4-carboxamide;
N-(3-(difluoromethyl)-1-(4-(4-((2-(2,6-dioxopiperidin-3-yl)-1-oxoisoindolin-5-yl)methyl)piperazin-1-yl)phenyl)-1H-pyrazol-4-yl)-2-(2-((2,2,2-trifluoroethyl)amino)pyridin-4-yl)oxazole-4-carboxamide;
N-(3-(difluoromethyl)-1-(4-(4-((2-(2,6-dioxopiperidin-3-yl)-4-fluoro-1-oxoisoindolin-5-yl)methyl)piperazin-1-yl)phenyl)-1H-pyrazol-4-yl)-2-(2-((2,2,2-trifluoroethyl)amino)pyridin-4-yl)oxazole-4-carboxamide;
N-(3-(difluoromethyl)-1-(4-(4-((2-(2,6-dioxopiperidin-3-yl)-6-fluoro-1-oxoisoindolin-5-yl)methyl)piperazin-1-yl)phenyl)-1H-pyrazol-4-yl)-2-(2-((2,2,2-trifluoroethyl)amino)pyridin-4-yl)oxazole-4-carboxamide;
N-(3-(difluoromethyl)-1-(4-(4-((2-(2,6-dioxopiperidin-3-yl)-7-fluoro-1-oxoisoindolin-5-yl)methyl)piperazin-1-yl)phenyl)-1H-pyrazol-4-yl)-2-(2-((2,2,2-trifluoroethyl)amino)pyridin-4-yl)oxazole-4-carboxamide;
N-(3-(difluoromethyl)-1-(4-(4-((2-(2,6-dioxopiperidin-3-yl)-1-oxoisoindolin-4-yl)methyl)piperazin-1-yl)phenyl)-1H-pyrazol-4-yl)-2-(2-((2,2,2-trifluoroethyl)amino)pyridin-4-yl)oxazole-4-carboxamide;
N-(3-(difluoromethyl)-1-(1-((2-(2,6-dioxopiperidin-3-yl)-1,3-dioxoisoindolin-5-yl)methyl)piperidin-4-yl)-1H-pyrazol-4-yl)-2-(2-((2,2,2-trifluoroethyl)amino)pyridin-4-yl)oxazole-4-carboxamide;
6-(5-cyano-1H-pyrrolo[2,3-b]pyridin-1-yl)-N-(1-((2-(2,6-dioxopiperidin-3-yl)-1,3-dioxoisoindolin-5-yl)methyl)piperidin-4-yl)-4-(isopropylamino)nicotinamide;
2-(2-((cyclopropylmethyl)amino)pyridin-4-yl)-N-(3-(difluoromethyl)-1-(1-((2-(2,6-dioxopiperidin-3-yl)-1,3-dioxoisoindolin-5-yl)methyl)piperidin-4-yl)-1H-pyrazol-4-yl)oxazole-4-carboxamide;
N-(3-(difluoromethyl)-1-(4-(4-((2-(2,6-dioxopiperidin-3-yl)-1,3-dioxoisoindolin-5-yl)methyl)piperazin-1-yl)phenyl)-1H-pyrazol-4-yl)-2-(2-((2,2,2-trifluoroethyl)amino)pyridin-4-yl)oxazole-4-carboxamide;
N-((1r,4r)-4-(3-chloro-4-cyanophenoxy)cyclohexyl)-6-(4-((3-((2-(2,6-dioxopiperidin-3-yl)-1-oxoisoindolin-5-yl)methyl)azetidin-1-yl)methyl)piperi din-1-yl)pyridazine-3-carboxamide;
N-((1r,4r)-4-(3-chloro-4-cyanophenoxy)cyclohexyl)-6-(4-((4-((2-(2,6-dioxopiperidin-3-yl)-1-oxoisoindolin-5-yl)methyl)piperidin-1-yl)methyl)piperidin-1-yl)pyridazine-3-carboxamide;
N-((1r,4r)-4-(3-chloro-4-cyanophenoxy)cyclohexyl)-6-(4-((3-((2-(2,6-dioxopiperidin-3-yl)-1-oxoisoindolin-5-yl)methylene)azetidin-1-yl)methyl)piperidin-1-yl)pyridazine-3-carboxamide;
N-((1r,4r)-4-(3-chloro-4-cyanophenoxy)cyclohexyl)-6-(4-((3-((1S)-(2-(2,6-dioxopiperidin-3-yl)-1-oxoisoindolin-5-yl)(hydroxy)methyl)-3-hydroxyazetidin-1-yl)methyl)piperidin-1-yl)pyridazine-3-carboxamide;
N-((1r,3r)-3-(3-chloro-4-cyanophenoxy)-2,2,4,4-tetramethylcyclobutyl)-6-(4-((4-((2-(2,6-dioxopiperidin-3-yl)-1-oxoisoindolin-5-yl)methyl)piperidin-1-yl)methyl)piperidin-1-yl)nicotinamide;
N-((1r,3r)-3-(3-chloro-4-cyanophenoxy)-2,2,4,4-tetramethylcyclobutyl)-6-(4-((3-((2-(2,6-dioxopiperidin-3-yl)-1-oxoisoindolin-5-yl)methylene)azetidin-1-yl)methyl)piperidin-1-yl)nicotinamide;
7-cyclopentyl-2-((5-(4-((4-((2-(2,6-dioxopiperidin-3-yl)-1-oxoisoindolin-5-yl)methyl)piperidin-1-yl)methyl)piperidin-1-yl)pyridin-2-yl)amino)-N,N-dimethyl-7H-pyrrolo[2,3-d]pyrimidine-6-carboxamide;
7-cyclopentyl-2-((5-(4-((3-((2-(2,6-dioxopiperidin-3-yl)-1-oxoisoindolin-5-yl)methyl)azetidin-1-yl)methyl)piperidin-1-yl)pyridin-2-yl)amino)-N,N-dimethyl-7H-pyrrolo[2,3-d]pyrimidine-6-carboxamide;
N-(2-chloro-6-methylphenyl)-2-((6-(4-((4-((2-(2,6-dioxopiperidin-3-yl)-1-oxoisoindolin-5-yl)methyl)piperidin-1-yl)methyl)piperidin-1-yl)-2-methylpyrimidin-4-yl)amino)thiazole-5-carboxamide;
3-(5-((4-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)-1-oxoisoindolin-2-yl)piperidine-2,6-dione;
3-(5-((4-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)-6-fluoro-1-oxoisoindolin-2-yl)piperidine-2,6-dione;
3-(4-((4-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)-1-oxoisoindolin-2-yl)piperidine-2,6-dione;
3-(5-((4-(6-(5-((R)-2-(2,5-difluorophenyl)pyrrolidin-1-yl)pyrazolo[1,5-a]pyrimidin-3-yl)pyridin-2-yl)piperazin-1-yl)methyl)-1-oxoisoindolin-2-yl)piperidine-2,6-dione;
3-(5-((4-(6-(5-((R)-2-(2,5-difluorophenyl)pyrrolidin-1-yl)pyrazolo[1,5-a]pyrimidin-3-yl)pyridin-2-yl)piperazin-1-yl)methyl)-4-fluoro-1-oxoisoindolin-2-yl)piperidine-2,6-dione;
3-(5-((4-(6-(5-((R)-2-(2,5-difluorophenyl)pyrrolidin-1-yl)pyrazolo[1,5-a]pyrimidin-3-yl)pyridin-2-yl)piperazin-1-yl)methyl)-6-fluoro-1-oxoisoindolin-2-yl)piperidine-2,6-dione;
3-(5-((4-(6-(5-((R)-2-(2,5-difluorophenyl)pyrrolidin-1-yl)pyrazolo[1,5-a]pyrimidin-3-yl)pyridin-2-yl)piperazin-1-yl)methyl)-7-fluoro-1-oxoisoindolin-2-yl)piperidine-2,6-dione;
5-((4-(6-(5-((R)-2-(2,5-difluorophenyl)pyrrolidin-1-yl)pyrazolo[1,5-a]pyrimidin-3-yl)pyridin-2-yl)piperazin-1-yl)methyl)-2-(2,6-dioxopiperidin-3-yl)isoindoline-1,3-dione;
2-(6,7-dihydro-5H-pyrrolo[1,2-c]imidazol-1-yl)-2-(6-(4-(4-((2-(2,6-dioxopiperidin-3-yl)-1-oxoisoindolin-5-yl)methyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-((2-(2,6-dioxopiperidin-3-yl)-1-oxoisoindolin-5-yl)methyl)piperazin-1-yl)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-l-yl)-2-(6-(4-(4-(1-((2-(2,6-dioxopiperidin-3-yl)-1-oxoisoindolin-5-yl)methyl)piperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1S,4S)-5-((2-(2,6-dioxopipendin-3-yl)-1-oxoisoindolin-5-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(((2-(2,6-dioxopiperidin-3-yl)-1-oxoisoindolin-5-yl)methyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(8-((2-(2,6-dioxopiperidin-3-yl)-1-oxoisoindolin-5-yl)methyl)-3,8-diazabicyclo[3.2.1]octan-3-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-((2-(2,6-dioxopiperidin-3-yl)-1-oxoisoindolin-5-yl)methyl)-3,3-difluoropiperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-((2-(2,6-dioxopiperidin-3-yl)-1-oxoisoindolin-5-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-((2-(2,6-dioxopiperidin-3-yl)-1-oxoisoindolin-5-yl)methyl)piperazin-1-yl)-3,3-difluoropiperidin-1-yl)phenyl)-4-fluoro-1-oxoisoindolin-2-yl)-N-(thiazol-2-yl)acetamide;
N-(2-(1-((2-(2,6-dioxopiperidin-3-yl)-1-oxoisoindolin-5-yl)methyl)piperidin-4-yl)-5-(2-hydroxypropan-2-yl)benzo[d]thiazol-6-yl)-6-(trifluoromethyl)picolinamide;
N-(2-(1-((2-(2,6-dioxopiperidin-3-yl)-4-fluoro-1-oxoisoindolin-5-yl)methyl)piperidin-4-yl)-5-(2-hydroxypropan-2-yl)benzo[d]thiazol-6-yl)-6-(trifluoromethyl)picolinamide;
N-(2-(1-((2-(2,6-dioxopiperidin-3-yl)-6-fluoro-1-oxoisoindolin-5-yl)methyl)piperidin-4-yl)-5-(2-hydroxypropan-2-yl)benzo[d]thiazol-6-yl)-6-(trifluoromethyl)picolinamide;
N-(2-(1-((2-(2,6-dioxopiperidin-3-yl)-7-fluoro-1-oxoisoindolin-5-yl)methyl)piperidin-4-yl)-5-(2-hydroxypropan-2-yl)benzo[d]thiazol-6-yl)-6-(trifluoromethyl)picolinamide;
N-(2-(1-((2-(2,6-dioxopiperidin-3-yl)-1-oxoisoindolin-5-yl)methyl)piperidin-4-yl)-5-(2-hydroxypropan-2-yl)benzo[d]oxazol-6-yl)-6-(trifluoromethyl)picolinamide;
N-(2-(1-((2-(2,6-dioxopiperidin-3-yl)-4-fluoro-1-oxoisoindolin-5-yl)methyl)piperidin-4-yl)-5-(2-hydroxypropan-2-yl)benzo[d]oxazol-6-yl)-6-(trifluoromethyl)picolinamide;
N-(2-(1-((2-(2,6-dioxopiperidin-3-yl)-6-fluoro-1-oxoisoindolin-5-yl)methyl)piperidin-4-yl)-5-(2-hydroxypropan-2-yl)benzo[d]oxazol-6-yl)-6-(trifluoromethyl)picolinamide;
N-(2-(1-((2-(2,6-dioxopiperidin-3-yl)-1-oxoisoindolin-5-yl)methyl)piperidin-4-yl)-6-(2-hydroxypropan-2-yl)-2H-indazol-5-yl)-6-(trifluoromethyl)picolmamide;
N-(2-(1-((2-(2,6-dioxopiperidin-3-yl)-4-fluoro-1-oxoisoindolin-5-yl)methyl)piperidin-4-yl)-6-(2-hydroxypropan-2-yl)-2H-indazol-5-yl)-6-(trifluoromethyl)picolmamide; and
N-(2-(1-((2-(2,6-dioxopiperidin-3-yl)-6-fluoro-1-oxoisoindolin-5-yl)methyl)piperidin-4-yl)-6-(2-hydroxypropan-2-yl)-2H-indazol-5-yl)-6-(trifluoromethyl)picolinamide.

31. The compound of Formula (II) or salts, enantiomers, stereoisomers, solvates, isotopically enriched analogs, prodrugs, or polymorphs thereof as claimed in any one of claims 12-30, which is hydrohalides (including hydrochlorides and hydrobromates), sulfates, citrates, maleates, methanesulfonates, citrates, lactates, L-tartrates, fumarates, L-malates, phosphates, dihydrophosphates, pyrophosphates, metaphosphates, oxalates, malonates, benzoates, mandelates, succinates, trifluoroacetates, methanesulfonates, hippurates, hydroxyacetates, or p-toluenesulfonates of the compound of Formula (II).

32. A pharmaceutical composition comprising as active ingredient the compound of Formula (I) or pharmaceutically acceptable salts, enantiomers, stereoisomers, solvates, isotopically enriched analogs, prodrugs, or polymorphs thereof as claimed in any one of claims 1-11, and at least one pharmaceutically acceptable carrier.

33. The pharmaceutical composition as claimed in claim 32, further comprising at least one additional therapeutic agent, e.g., an anticancer agent.

34. A pharmaceutical composition comprising as active ingredient the compound of Formula (II) or pharmaceutically acceptable salts, enantiomers, stereoisomers, solvates, isotopically enriched analogs, prodrugs, or polymorphs thereof as claimed in any one of claims 12-30, and at least one pharmaceutically acceptable carrier.

35. The pharmaceutical composition as claimed in claim 34, further comprising at least one additional therapeutic agent, e.g., an anticancer agent.

36. A medicine kit or reagent kit comprising:
the compound of Formula (I) or a pharmaceutically acceptable salt thereof as claimed in any one of claims 1-11; or
the compound of Formula (II) or a pharmaceutically acceptable salt thereof as claimed in any one of claims 12-30; or
the pharmaceutical composition as claimed in claims 32 or 34.

37. Use of the compound of Formula (I) or salts, enantiomers, stereoisomers, solvates, isotopically enriched analogs, prodrugs, or polymorphs thereof as claimed in any one of claims 1-11 for the manufacture of a medicament for the prevention or treatment of cereblon protein-mediated disease or disorder.

38. The use as claimed in claim 37, wherein the cereblon protein-mediated disease or disorder comprises: tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, cardiovascular disease, Unverricht's syndrome, Richter syndrome (RS), acute liver failure, and diabetes.

39. The use as claimed in claim 37, wherein the cereblon protein-mediated disease or disorder is selected from the group consisting of:
myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout, etc; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; cardiovascular disease (including congestive heart failure, myocardial infarction, coronary heart disease, atherosclerosis); multiple organ dysfunction caused by cachexia and septic shock; and acute liver failure.

40. Use of the compound of Formula (II) or salts, enantiomers, stereoisomers, solvates, isotopically enriched analogs, prodrugs, or polymorphs thereof as claimed in any one of claims 12-30 for the manufacture of a medicament for the prevention and/or treatment of a disease or disorder selected from the group consisting of tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, cardiovascular disease, Unverricht's syndrome, Richter syndrome (RS), acute liver failure, diabetes, Kennedy disease, seborrheic alopecia, hirsutism, skin disease, dysfunctional uterine bleeding, anemia, pediatric aplastic anemia, endometriosis, transplant rejection, polycystic ovary syndrome, and thyroid disease.

41. The use as claimed in claim 40, wherein the disease or disorder is selected from the group consisting of:
myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma, non-small cell lung cancer and small cell lung cancer; inflammatory myofibroblastoma; colorectal cancer; intestinal cancer; brain glioma; astroblastoma; glioma; peripheral neuroepithelioma; ovarian cancer; bronchial cancer; prostate cancer; breast cancer, including triple negative breast cancer, sporadic breast cancer and patients with Cowden syndrome; pancreatic cancer; central nervous system tumor; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout, etc; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; multiple organ dysfunction caused by cachexia and septic shock; acute liver failure; dysfunctional uterine bleeding; anemia; pediatric aplastic anemia; endometriosis; polycystic ovary syndrome; thyroid disease; cardiovascular diseases (e.g., coronary heart disease, congestive heart failure, myocardial infarction, atherosclerosis); skin diseases (e.g., acne); Kennedy disease; seborrheic alopecia; and hirsutism.

42. The compound of Formula (I) or salts, enantiomers, stereoisomers, solvates, isotopically enriched analogs, prodrugs, or polymorphs thereof as claimed in any one of claims 1-11, for use in the prevention and/or treatment of a cereblon protein-mediated disease or disorder.

43. The compound of Formula (I) or salts, enantiomers, stereoisomers, solvates, isotopically enriched analogs, prodrugs, or polymorphs thereof as claimed in claim 42, wherein the cereblon protein-mediated disease or disorder comprises: tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, cardiovascular disease, Unverricht's syndrome, Richter syndrome (RS), acute liver failure, and diabetes.

44. The compound of Formula (I) or salts, enantiomers, stereoisomers, solvates, isotopically enriched analogs, prodrugs, or polymorphs thereof as claimed in claim 42, wherein the cereblon protein-mediated disease or disorder is selected from the group consisting of:
myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout, etc; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; cardiovascular disease (including congestive heart failure, myocardial infarction, coronary heart disease, atherosclerosis); multiple organ dysfunction caused by cachexia and septic shock; and acute liver failure.

45. The compound of Formula (II) or salts, enantiomers, stereoisomers, solvates, isotopically enriched analogs, prodrugs, or polymorphs thereof as claimed in any one of claims 12-30, for use in the prevention and/or treatment of a disease or disorder selected from the group consisting of tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, Unverricht's syndrome, Richter syndrome (RS), acute liver failure, diabetes, Kennedy disease, seborrheic alopecia, hirsutism, skin disease, cardiovascular disease, dysfunctional uterine bleeding, anemia, pediatric aplastic anemia, endometriosis, transplant rejection, polycystic ovary syndrome, and thyroid disease.

46. The compound of Formula (II) or salts, enantiomers, stereoisomers, solvates, isotopically enriched analogs, prodrugs, or polymorphs thereof as claimed in claim 45, wherein the disease or disorder mediated by or associated with the target protein is selected from the group consisting of:
myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout, etc; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; multiple organ dysfunction caused by cachexia and septic shock; acute liver failure; dysfunctional uterine bleeding; anemia; pediatric aplastic anemia; endometriosis; polycystic ovary syndrome; thyroid disease; cardiovascular diseases (e.g., coronary heart disease, congestive heart failure, myocardial infarction, atherosclerosis); skin diseases (e.g., acne); Kennedy disease; seborrheic alopecia; and hirsutism.

47. A method for treating or preventing a cereblon protein-mediated disease or disorder in a subject, comprising administering to the subject a therapeutically effective amount of the compound of Formula (I) or salts, enantiomers, stereoisomers, solvates, isotopically enriched analogs, prodrugs, or polymorphs thereof as claimed in any one of claims 1-11, or the pharmaceutical composition as claimed in claim 32 or 33.

48. The method as claimed in claim 47, wherein the cereblon protein-mediated disease or disorder comprises: tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, cardiovascular disease, Unverricht's syndrome, Richter syndrome (RS), acute liver failure, and diabetes.

49. The method as claimed in claim 47, wherein the cereblon protein-mediated disease or disorder is selected from the group consisting of:
myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer; inflammatory myofibroblastoma; colorectal cancer; intestinal cancer; brain glioma; astroblastoma; glioma; peripheral neuroepithelioma; ovarian cancer; bronchial cancer; prostate cancer; breast cancer, including triple negative breast cancer, sporadic breast cancer and patients with Cowden syndrome; pancreatic cancer; central nervous system tumor; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout, etc; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; cardiovascular disease (including congestive heart failure, myocardial infarction, coronary heart disease, atherosclerosis); multiple organ dysfunction caused by cachexia and septic shock; and acute liver failure.

50. The method as claimed in any one of claims 47-49, wherein the compound of Formula (I) or a pharmaceutically acceptable salt thereof, or the pharmaceutical composition is administered to the subject through at least one mode of administration selected from the group consisting of nasal administration, inhalation administration, topical administration, oral administration, oral mucosal administration, rectal administration, pleural cavity administration, peritoneal administration, vaginal administration, intramuscular administration, subcutaneous, transdermal, epidural, intrathecal, and intravenous administration.

51. A method for treating or preventing a disease or disorder in a subject, comprising administering to the subject a therapeutically effective amount of the compound of Formula (II) or salts, enantiomers, stereoisomers, solvates, isotopically enriched analogs, prodrugs, or polymorphs thereof as claimed in any one of claims 12-30, or the pharmaceutical composition as claimed in claim 34 or 35, wherein the disease or disorder comprises tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, Unverricht's syndrome, Richter syndrome (RS), acute liver failure, diabetes, Kennedy disease, seborrheic alopecia, hirsutism, skin disease, cardiovascular disease, dysfunctional uterine bleeding, anemia, pediatric aplastic anemia, endometriosis, transplant rejection, polycystic ovary syndrome, and thyroid disease.

52. The method as claimed in claim 51, wherein the disease or disorder is selected from the group consisting of:
myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma; inflammatory myofibroblastoma; colorectal cancer; intestinal cancer; brain glioma; astroblastoma; glioma; peripheral neuroepithelioma; ovarian cancer; bronchial cancer; prostate cancer; breast cancer, including triple negative breast cancer, sporadic breast cancer and patients with Cowden syndrome; pancreatic cancer; central nervous system tumor; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout, etc; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; multiple organ dysfunction caused by cachexia and septic shock; acute liver failure; dysfunctional uterine bleeding; anemia; pediatric aplastic anemia; endometriosis; polycystic ovary syndrome; thyroid disease; cardiovascular diseases (e.g., coronary heart disease, congestive heart failure, myocardial infarction, atherosclerosis); skin diseases (e.g., acne); Kennedy disease; seborrheic alopecia; and hirsutism.

53. The method as claimed in claim 51 or 52, wherein the compound of Formula (II) or a pharmaceutically acceptable salt thereof, or the pharmaceutical composition is administered to the subject through at least one mode of administration selected from the group consisting of nasal administration, inhalation administration, topical administration, oral administration, oral mucosal administration, rectal administration, pleural cavity administration, peritoneal administration, vaginal administration, intramuscular administration, subcutaneous, transdermal, epidural, intrathecal, and intravenous administration.
